# EUROPEAN PATENT APPLICATION

(11) **EP 1 972 686 A1**
(43) Date of publication of application: **24.09.2008**
(21) Application number: 08100428.5
(22) Date of filing: 14.01.2008
(51) Int. Cl.: C12N 9/02, C12N 15/53, C12N 15/82, C12N 5/14, C12N 5/04

(54) **Crystal structure of dicamba monooxygenase (DMO) and uses thereof**

(30) Priority: 12.01.2007 US 884854 P; 21.05.2007 US 939278 P
(71) Applicant: Monsanto Technology, LLC, St. Louis, MO 63167 (US)
(72) Inventor: D'Ordine, Robert L., St. Louis, MO 63167 (US); Sturman, Eric J., St. Louis, MO 63167 (US); Storek, Michael J., St. Louis, MO 63167 (US); Rydel, Timothy J., St. Louis, MO 63167 (US); Moshiri, Farhad, St. Louis, MO 63167 (US); English, Leigh, St. Louis, MO 63167 (US)
(74) Representative: Helbing, Jörg

(57) **Abstract**

The invention provides for identification and use of crystal structures of Dicamba monooxygenase (DMO) that may be complexed with iron or cobalt cofactor and substrate (dicamba), or product (DCSA) in order to define residues important for enzymatic structure and function. Methods of using such structures are described. Data storage media comprising the crystal structural coordinate information are also described.

## Description

### BACKGROUND OF THE INVENTION

This application claims the priority of U.S. provisional application Serial No. 60/884,854 filed January 12, 2007, and U.S. provisional application Serial No. 60/939,278, filed May 21, 2007, the entire disclosures of which are incorporated herein by reference.

### Field of the Invention

The present invention relates generally to the fields of enzymology and X-ray crystallography. More specifically, the present invention relates to identification of the structure of dicamba monooxygenase (DMO) and methods for providing variants thereof, including variants with altered enzymatic activity.

### Description of the Related Art

Dicamba monooxygenase (DMO) catalyzes the degradation of the herbicide dicamba (3,6-dichloro-o-anisic acid; also termed 3,6-dichloro-2-methoxybenzoic acid) to non-herbicidal 3,6-dichlorosalicylic acid (3,6-DCSA; DCSA) (Herman *et al.,* 2005; GenBank accession AY786443; encoded sequence shown in SEQ ID NO:1). Expression of DMO in transgenic plants confers herbicide tolerance (U.S. Patent No. 7,022,896).

The wild-type bacterial oxygenase gene (isolated from *Pseudomonas maltophilia)* encodes a 37 kDa protein composed of 339 amino acids that is similar to other Rieske non-heme iron oxygenases that function as monooxygenases (Chakraborty *et al.,* 2005; Gibson and Parales, 2000; Wackett, 2002). In its active form the enzyme comprises a homo-oligomer of three monomers, or a homotrimer, of which the monomers are termed molecules "a", "b", and "c". Activity of DMO typically requires two auxiliary proteins for shuttling electrons from NADH and/or NADPH to dicamba, a reductase and a ferredoxin (U.S. Patent 7,022,896; Herman *et al.,* 2005). However dicamba tolerance in transgenic plants has been demonstrated through transformation with DMO alone, indicating that a plant's endogenous reductases and ferredoxins may substitute in shuttling the electrons. The three dimensional structure of DMO, including identification of functional domains important to function and the nature of interaction with dicamba has not previously been determined. There is, therefore, a great need in the art for such information as it could allow, for the first time, targeted development of variant molecules exhibiting altered or even enhanced dicamba degrading activity. Furthermore, identification of other proteins with the same structural properties described here in could be used to create dicamba binding or degrading activity.

### SUMMARY OF THE INVENTION

The present invention provides a crystallized dicamba monooxygenase polypeptide comprising a sequence at least 85% identical to any of SEQ ID NO:1, SEQ ID NO:2, or SEQ ID NO:3. The invention further provides, in one embodiment, a molecule comprising a binding surface for dicamba, that binds to dicamba with a K_{D} or K_{M} of between 0.1-500µM, wherein the molecule does not comprise an amino acid sequence of any of SEQ ID NOs:1-3. In another embodiment, the invention provides a molecule comprising a binding surface for dicamba wherein the K_{D} or K_{M} for dicamba is between 0.1-100µM. In certain embodiments, the molecule may be further defined as a polypeptide.

In another aspect, the invention provides an isolated polypeptide comprising dicamba monooxygenase (DMO) activity, wherein the polypeptide comprises a sequence selected from the group consisting of: a) a polypeptide sequence that when in crystalline form comprises a space group of P3₂; b) a polypeptide sequence that when in crystalline form comprises a binding site for a substrate, the binding site defined as comprising the characteristics of: (i) a volume of 175-500Å³, (ii) electrostatically accommodative of a negatively charged carboxylate, (iii) accommodative of at least one chlorine moiety if present in the substrate, (iv) accommodative of a planar aromatic ring in the substrate, and (v) displays a distance from an iron atom that activates oxygen in the polypeptide to a carbon of the methoxy group of the substrate, sufficient for catalysis, of about 2.5Å to about 7Å; c) a polypeptide sequence that when in crystalline form comprises a unit-cell parameter of a=79-81Å, b=79-81Å, and c=158-162 Å; d) a polypeptide sequence that folds to produce a three-dimensional macromolecular structure characterized by the atomic structure coordinates of peptide backbone atoms of any of Tables 1-5, and 25-26, or a macromolecular structure that exhibits a root-mean-square difference (rmsd) in α-carbon positions of less than 2.0Å with the atomic structure coordinates of peptide backbone atoms of any of Tables 1-5, and 25-26, when superimposed on the corresponding backbone atoms described by the structure coordinates of amino acid residues comprising the polypeptide, when 70% or more of the total macromolecular structure α-carbon atoms are used in the superimposition; e) a polypeptide sequence that folds to produce a three-dimensional macromolecular structure that has the same tertiary and quaternary fold as that characterized by the α-carbon coordinates for the structure represented in any of Tables 1-5, and 25-26; f) a polypeptide sequence comprising substantially all of the amino acid residues corresponding to H51, A316, L318, C49, P55, V308, F53, D47, A54, L73, I48, I301, Y307, H86, R304, C320, A300, V297, N84, E322, P50, R52, C68, Y70, L95, P315, P31, T30, L46, I313, G87, D321, D29, N154, G89, S94, M317, H71, D157, G72, V296, V298, D58, D153, R314, and R98 of SEQ ID NO:2 or SEQ ID NO:3; g) a polypeptide sequence comprising a Rieske center domain, further defined as comprising a polypeptide sequence that folds to produce a three-dimensional macromolecular structure characterized by the atomic structure coordinates of peptide backbone atoms of any of Tables 1-5, and 25-26, corresponding to amino acid residues 2-124 of SEQ ID NO:2 or SEQ ID NO:3, or a macromolecular structure that exhibits a root-mean-square difference (rmsd) in α-carbon positions of less than 2.0Å with the atomic structure coordinates of peptide backbone atoms of any of Tables 1-5, and 25-26, corresponding to amino acid residues 2-124 of SEQ ID NO:2 or SEQ ID NO:3 when superimposed on the corresponding backbone atoms described by the structure coordinates of amino acid residues comprising the polypeptide, when 70% or more of the macromolecular structure α-carbon atoms corresponding to amino acid residues 2-124 of SEQ ID NO:2 or SEQ ID NO:3 are used in the superimposition; and h) a polypeptide sequence comprising a DMO catalytic domain, further defined as comprising a polypeptide sequence that folds to produce a three-dimensional macromolecular structure characterized by the atomic structure coordinates of peptide backbone atoms of any of Tables 1-5, and 25-26, corresponding to amino acid residues 125-343 of SEQ ID NO:2 or SEQ ID NO:3, or a macromolecular structure that exhibits a root-mean-square difference (rmsd) in α-carbon positions of less than 2.0Å with the atomic structure coordinates of peptide backbone atoms of any of Tables 1-5, and 25-26, corresponding to amino acid residues 125-343 of SEQ ID NO:2 or SEQ ID NO:3 when superimposed on the corresponding backbone atoms described by the structure coordinates of amino acid residues comprising the polypeptide, when 70% or more of the macromolecular structure α-carbon atoms corresponding to amino acid residues 125-343 of SEQ ID NO:2 or SEQ ID NO:3 are used in the superimposition; wherein the polypeptide does not comprise the amino acid sequence of any of SEQ ID NOs:1-3.

In particular embodiments, the invention further provides the isolated polypeptide comprising dicamba monooxygenase (DMO) activity, wherein the polypeptide comprises a DMO enzyme having the sequence domain: -W-X₁-X₂-X₃-X₄-L- (SEQ ID NO:152), in which X₁ is Q, F, or H; X₂ is A, D, F, I, R, T, V, W, Y, C, E, G, L, M, Q, or S; X₃ is Q, G, I, V, A, C, D, H, L, M, N, R, S, T, or E; and X₄ is A, C, G, or S. In other embodiments the isolated comprises a DMO enzyme having the sequence domain: -N-X₁-Q-, in which X₁ is A, L, C, F, F, I, N, Q, S, V, W, Y, M or T. In yet other embodiments, the isolated polypeptide comprises a DMO enzyme having the sequence domain: -W-X₁-D- in which X₁ is N, K, A, C, E, I, L, S, T, W, Y, H, or M. In still further embodiments, the isolated polypeptide exhibits an increased level of DMO activity relative to the activity of a wild type DMO. In particular embodiments the isolated polypeptide comprises a DMO enzyme having the sequence domain: X₁-X₂-G-X₃-H (SEQ ID NO:153) in which X₁ is S, H, or T; X₂ is R, Q, S, T, F, H, N, V, W, Y, C, I, K, L, or M; and X₃ is T, Q, or M. In particular embodiments, the isolated polypeptide comprises a substitution in residue X₂, numbered according to the numbering of SEQ ID NO:2 or SEQ ID NO:3, selected from the group consisting of: R248C, R2481, R248K, R248L, R248M. In yet other embodiments, the isolated polypeptide comprises a DMO enzyme comprising one or more substitution(s) in residues numbered according to the numbering of SEQ ID NO:2 or SEQ ID NO:3, selected from the group consisting of: A169M, N218H, N218M, G266S, L282I, A287C, A287E, A287M, A287S, and Q288E.

In certain embodiments, the isolated polypeptide, comprising dicamba monooxygenase (DMO) activity wherein the polypeptide does not comprise the amino acid sequence of any of SEQ ID NOs:1-3, comprises the secondary structural elements of table 6 or table 8. The isolated polypeptide may also be defined as comprising a polypeptide sequence that when in crystalline form comprises a unit-cell parameter α=β= 90° and γ=120° wherein the polypeptide does not comprise the amino acid sequence of any of SEQ ID NOs:1-3. In certain embodiments, the isolated polypeptide may further be defined as comprising one monomer per asymmetric unit. The isolated polypeptide may also further be defined as a crystal.

In other embodiments, the isolated polypeptide may be defined as comprising a polypeptide sequence that when in crystalline form diffracts X-rays for a determination of atomic coordinates at a resolution higher than 3.2Å. In particular embodiments, the isolated polypeptide may be defined as comprising a polypeptide sequence that when in crystalline form diffracts X-rays for a determination of atomic coordinates at a resolution higher than 3.0Å, or about 2.65Å, or about 1.9Å. In each of these embodiments, the polypeptide does not comprise the amino acid sequence of any of SEQ ID NOs:1-3.

In certain embodiments, the present invention also includes the isolated polypeptide comprising dicamba monooxygenase (DMO) activity as described above, wherein the presence of free iron enhances binding to dicamba and wherein the polypeptide does not comprise the amino acid sequence of any of SEQ ID NOs:1-3. The isolated polypeptide comprising dicamba monooxygenase (DMO) activity, wherein the polypeptide comprises a sequence selected from the group consisting of: a) a polypeptide sequence that when in crystalline form comprises a space group of P3₂; b) a polypeptide sequence that when in crystalline form comprises a binding site for a substrate, the binding site defined as comprising the characteristics of: (i) a volume of 175-500Å3, (ii) electrostatically accommodative of a negatively charged carboxylate, (iii) accommodative of at least one chlorine moiety if present in the substrate, (iv) accommodative of a planar aromatic ring in the substrate, and (v) displays a distance from an iron atom that activates oxygen in the polypeptide to a carbon of the methoxy group of the substrate, sufficient for catalysis, of about 2.5Å to about 7Å; c) a polypeptide sequence that when in crystalline form comprises a unit-cell parameter of a=79-81Å, b=79-81Å, and c=158-162 Å; d) a polypeptide sequence that folds to produce a three-dimensional macromolecular structure characterized by the atomic structure coordinates of peptide backbone atoms of any of Tables 1-5, and 25-26, or a macromolecular structure that exhibits a root-mean-square difference (rmsd) in α-carbon positions of less than 2.0Å with the atomic structure coordinates of any of Tables 1-5, and 25-26, when superimposed on the corresponding backbone atoms described by the structure coordinates of amino acid residues comprising the polypeptide, when 70% or more of the total macromolecular structure α-carbon atoms are used in the superimposition; e) a polypeptide sequence that folds to produce a three-dimensional macromolecular structure that has the same tertiary and quaternary fold as that characterized by the α-carbon coordinates for the structure represented in any of Tables 1-5, and 25-26; f) a polypeptide sequence comprising substantially all of the amino acid residues corresponding to H51, A316, L318, C49, P55, V308, F53, D47, A54, L73, I48, I301, Y307, H86, R304, C320, A300, V297, N84, E322, P50, R52, C68, Y70, L95, P315, P31, T30, L46, I313, G87, D321, D29, N154, G89, S94, M317, H71, D157, G72, V296, V298, D58, D153, R314, and R98 of SEQ ID NO:2 or SEQ ID NO:3; g) a polypeptide sequence comprising a Rieske center domain, further defined as comprising a polypeptide sequence that folds to produce a three-dimensional macromolecular structure characterized by the atomic structure coordinates of peptide backbone atoms of any of Tables 1-5, and 25-26, corresponding to amino acid residues 2-124 of SEQ ID NO:2 or SEQ ID NO:3, or a macromolecular structure that exhibits a root-mean-square difference (rmsd) in α-carbon positions of less than 2.0Å with the atomic structure coordinates of peptide backbone atoms of any of Tables 1-5, and 25-26, corresponding to amino acid residues 2-124 of SEQ ID NO:2 or SEQ ID NO:3 when superimposed on the corresponding backbone atoms described by the structure coordinates of amino acid residues comprising the polypeptide, when 70% or more of the macromolecular structure α-carbon atoms corresponding to amino acid residues 2-124 of SEQ ID NO:2 or SEQ ID NO:3 are used in the superimposition; and h) a polypeptide sequence comprising a DMO catalytic domain, further defined as comprising a polypeptide sequence that folds to produce a three-dimensional macromolecular structure characterized by the atomic structure coordinates of peptide backbone atoms of any of Tables 1-5, and 25-26, corresponding to amino acid residues 125-343 of SEQ ID NO:2 or SEQ ID NO:3, or a macromolecular structure that exhibits a root-mean-square difference (rmsd) in α-carbon positions of less than 2.0Å with the atomic structure coordinates of peptide backbone atoms of any of Tables 1-5, and 25-26, corresponding to amino acid residues 125-343 of SEQ ID NO:2 or SEQ ID NO:3 when superimposed on the corresponding backbone atoms described by the structure coordinates of amino acid residues comprising the polypeptide, when 70% or more of the macromolecular structure α-carbon atoms corresponding to amino acid residues 125-343 of SEQ ID NO:2 or SEQ ID NO:3 are used in the superimposition; wherein the polypeptide does not comprise the amino acid sequence of any of SEQ ID NOs:1-3, may further be defined as a folded polypeptide bound to a non-heme iron ion and comprising a Rieske center domain. The isolated polypeptide may also be further defined as a folded polypeptide bound to dicamba. In particular embodiments, the polypeptide comprises an amino acid sequence with from about 20% to about 99% sequence identity to the polypeptide sequence of any of SEQ ID NOs:1-3. Alternatively, the isolated polypeptide comprising dicamba monooxygenase (DMO) activity may comprise an amino acid sequence with less than about 95%, less than about 85%, less than about 65%, or less than about 45% identity to any of SEQ ID NOs:1-3.

The invention further relates to an isolated polypeptide comprising dicamba monooxygenase (DMO) activity, wherein the polypeptide comprises a C-terminal domain for donating an electron to a Rieske center, and further comprises an electron transport path from a Rieske center to a catalytic site having a conserved surface with a macromolecular structure formed by the amino acid residues N154, D157 H160, H165, and D294, corresponding to SEQ ID NO:2 or SEQ ID NO:3, or conservative substitutions thereof, and wherein the polypeptide does not comprise the amino acid sequence of any of SEQ ID NOs:1-3. In certain embodiments, the isolated polypeptide wherein the polypeptide does not comprise the amino acid sequence of any of SEQ ID NOs:1-3, comprises a polypeptide wherein the distance for iron FE2 to His71 ND1 is 2.57Å ±0.2-0.3Å; the distance for the His71 NE2 to Asp157 OD1 is 3.00Å ±0.2-0.3Å, the distance for Asp157 OD1 to His160 ND1 is 2.80Å ±0.2-0.3Å, and the distance for His160 NE2 to Fe is 2.43Å ±0.2-0.3Å.

The invention further provides an isolated polypeptide wherein the polypeptide does not comprise the amino acid sequence of any of SEQ ID NOs:1-3, further comprising a subunit interface region having a conserved surface with a macromolecular structure formed by amino acid residues V325, E322, D321, C320, L318, M317, A316, P315, R314, I313, V308, Y307, R304, I301, A300, V297, V296, V164, Y163, H160, G159, D157, N154, D153, R98, L95, S94, G89, G87, H86, P85, N84, L73, G72, H71, Y70, P69, C68, Q67, D58, P55, A54, F53, R52, H51, P50, I48, D47, L46, P31, T30, and D29, corresponding to SEQ ID NO:2 or SEQ ID NO:3, or conservative substitutions thereof. The invention also relates to an isolated polypeptide, wherein the polypeptide does not comprise the amino acid sequence of any of SEQ ID NOs:1-3, and further comprises a motif of residues H51a:R52:F53a:Y70a:H71a:H86a:H160c:Y163c: R304c:Y307c:A316c:L318c numbered corresponding to SEQ ID NO:2 or SEQ ID NO:3. The isolated polypeptide may further be defined as a homotrimer. A plant cell comprising a polypeptide comprising DMO activity, wherein the polypeptide does not comprise the amino acid sequence of any of SEQ ID NOs:1-3, is also an embodiment of the invention.

In another aspect, the invention relates to a method for determining the three dimensional structure of a crystallized DMO polypeptide to a resolution of about 3.0Å or better comprising: (a) obtaining a crystal comprising a sequence at least 85% identical to any of SEQ ID NO:1, SEQ ID NO:2, or SEQ ID NO:3; and (b) analyzing the crystal to determine the three dimensional structure of crystallized DMO. In particular embodiments, the method comprises a method wherein the analyzing comprises subjecting the crystal to diffraction analysis or spectrophotometric analysis.

In still another aspect, the invention provides a computer readable data storage medium encoded with computer readable data comprising atomic structural coordinates representing the three dimensional structure of crystallized DMO or a dicamba binding domain thereof. In particular embodiments, the computer readable data comprises atomic structural coordinates representing: (a) a dicamba binding domain defined by structural coordinates of one or more residues according to any of Tables 1-5, and 25-26, selected from the group consisting of L155, D157, L158, H160, A161, H165, R166, A169, Q170, D172, A173, A216, W217, N218, 1220, N230, I232, A233, V234, S247, R248, G249, T250, H251, G266, S267, L282, Q286, A287, Q288, A289, and V291 numbered corresponding to SEQ ID NO:2, or conservative substitutions thereof; (b) an interface domain defined by structure coordinates of one or more residues according to any of Tables 1-5, and 25-26, selected from the group consisting of V325, E322, D321, C320, L318, M317, A316, P315, R314, I313, V308, Y307, R304, I301, A300, V297, V296, V164, Y163, H160, G159, D157, N154, D153, R98, L95, S94, G89, G87, H86, P85, N84, L73, G72, H71, Y70, P69, C68, Q67, D58, P55, A54, F53, R52, H51, P50,148, D47, L46, P31, T30, and D29, numbered corresponding to SEQ ID NO:2 or SEQ ID NO:3, or conservative substitutions thereof; (c) an electron transport path from a Rieske center to a catalytic site defined by structure coordinates of one or more residues according to any of Tables 1-5, and 25-26, selected from the group consisting of N154, D157 H160, H165, and D294, numbered corresponding to SEQ ID NO:2 or SEQ ID NO:3, or conservative substitutions thereof; (d) a C-terminal domain defined by structure coordinates of one or more residues according to any of Tables 1-5, and 25-26, selected from the group consisting of A323, A324, V325, R326, V327, S328, R329, E330, I331, E332, K333, L334, E335, Q336, L337, E338, A339, A340 numbered corresponding to SEQ ID NO:2 or SEQ ID NO:3; or (e)a domain of any of (a)- (d) exhibiting a root mean square deviation of amino acid residues, comprising α-carbon backbone atoms, of less than 2Å with the atomic structure coordinates of any of Tables 1-5, and 25-26, when superimposed on the backbone atoms described by the structure coordinates of said amino acids when 70% or more of the macromolecular structure α-carbon atoms are used in the superimposition. In yet other particular embodiments, the computer readable data storage medium comprises the structural coordinates of any of Tables 1-5, and 25-26. A computer programmed to produce a three-dimensional representation of the data comprised on the computer readable data storage medium is also an aspect of the invention.

### BRIEF DESCRIPTION OF THE DRAWINGS

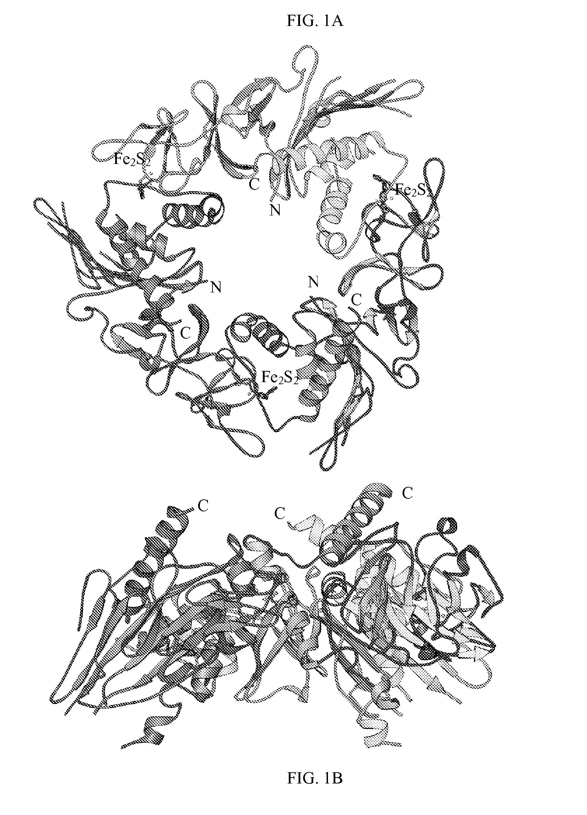

FIG. 1**.** (A) Ribbon Figures of Initial DMO Crystal Structure. Molecule "a" of the homotrimer is red-purple, molecule "b" is yellow-green, and molecule "c" is blue-turquoise. Rotation of the top view figure by 90° about a horizontal axis generates the bottom view figure (B). Each monomer has a pie slice shape, with the Rieske domain near the apex and the catalytic domain near the wider base of the pie slice. This figure was prepared using the Ribbons program (Carson, 1991).

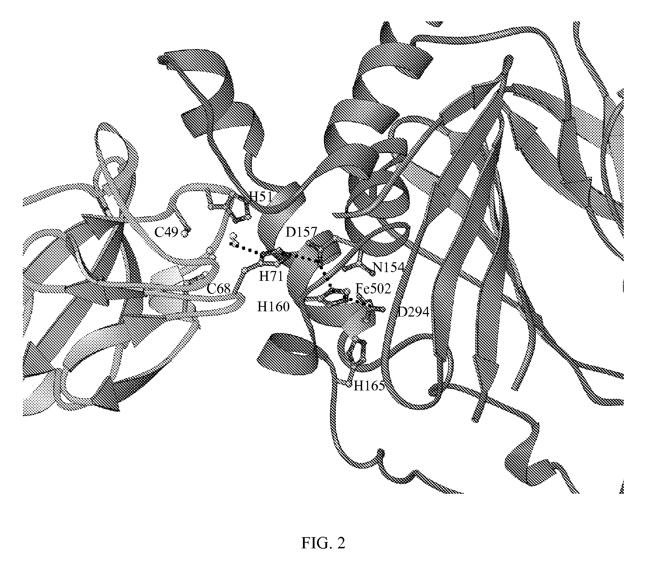

**FIG. 2****.** Active Site of "DMO Crystal 5" (Table 3). Molecule "b" (green) is on the left and molecule "a" (red) is on the right. The structure demonstrates the non-heme iron site (502, orange sphere) being occupied, the residues which chelate the iron (H160,H165, & D294), and the electron transfer path across the molecular interface (molecule "b"-H71 to molecule "a"-D157 to molecule "a"-H160 to molecule "a"-Fe502). This electron transfer path is indicated by dotted lines in the figure. This figure was prepared using the Ribbons program (Carson, 1991).

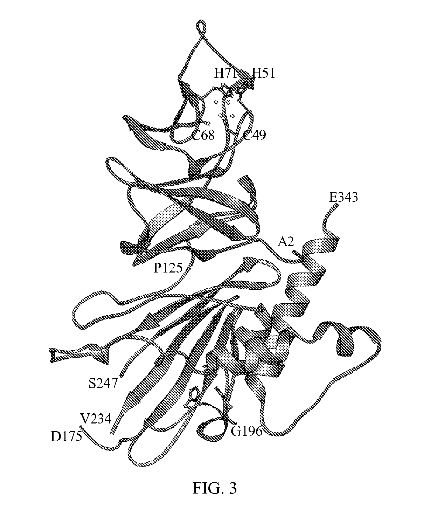

**FIG. 3****.** The structure of molecule "a" of the DMO-non-heme iron structure in "DMO Crystal 5". The DMO structure is composed of two domains: the Rieske domain (residues A2-1124, red) and the catalytic domain (residues P125-E343, purple). The catalytic domain contains the following structurally defined segments: P125-D175; G196-V234; S247-E343. Also displayed in the figure are the residues that chelate the Fe₂S₂ cluster (C49, H51, C68, H71) and residues that chelate the non-heme Fe502 (H160, H165, D294). Sulfur atoms are rendered as yellow spheres and iron atoms as orange spheres. This figure was prepared using the Ribbons program (Carson, 1991).

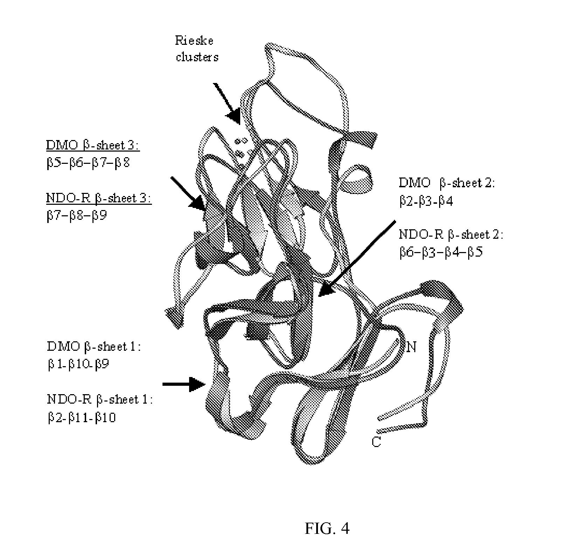

**FIG. 4****.** The Rieske Domain of NDO-R (cyan) aligned to the Rieske Domain of DMO (red). The DMO Rieske domain from "DMO Crystal 5" (molecule "a", residues 2-124) is displayed in a similar orientation to that of FIG. 3. NDO-R refers to naphthalene 1,2-dioxygenase from *Rhodococcus sp.* NCIMB12038; the aligned Rieske domain from molecule "a" (residues 45 -166) is displayed. Each domain is comprised of three β sheets, each sheet made up of antiparallel β-strands, and the composition and topological location of the sheets is similar in the aligned structures. The Rieske iron (Fe) and sulfur (S) cluster atoms are displayed in colors appropriate for each domain, and their location is noted. The best alignment for the two structures uses 84 Cα atoms, and has a root-mean-square distance (r.m.s.d.) of 1.90 Å. The approximate locations of the N- and C-termini for the domains are indicated in the figure. This figure was prepared using the Ribbons program (Carson, 1991).

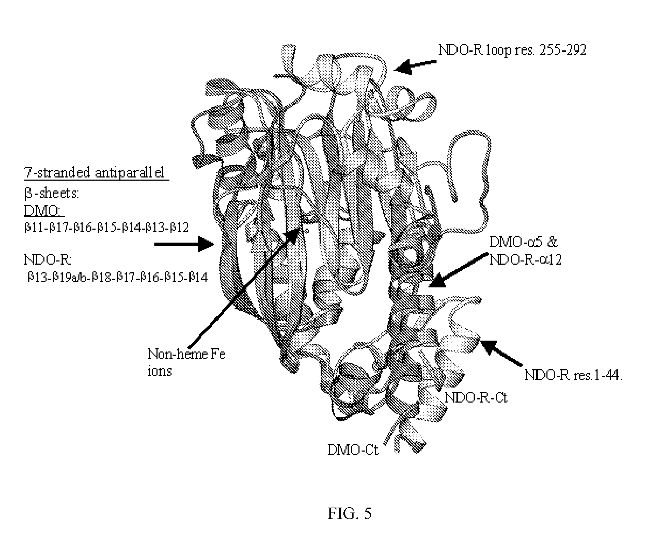

**FIG. 5****.** The Catalytic Domain of NDO-R (cyan & yellow) aligned to the Catalytic Domain of DMO (purple). The DMO catalytic domain from "DMO Crystal 5" (molecule "a", residues 125-175; 196-234; 247-343) is displayed. The aligned catalytic domain from NDO-R molecule "a" (residues 1-44; 167-440) is displayed. Two helix-containing NDO-R catalytic domain components that are lacking in DMO are colored in yellow - (a) an N-terminal peptide defined by residues 1-44 and (b) a loop coming off the 7-stranded sheet comprised of residues 255-292. The non-heme iron ions are displayed in colors appropriate for each domain. The best alignment for the two structures used 106 Cα atoms and had a root mean-square distance of 1.76 Å. The view reveals the 7-stranded anti-parallel β-sheet, and the non-heme iron ions and the C-terminal helices behind it. The C-termini are labeled. This figure was prepared using the Ribbons program (Carson, 1991).

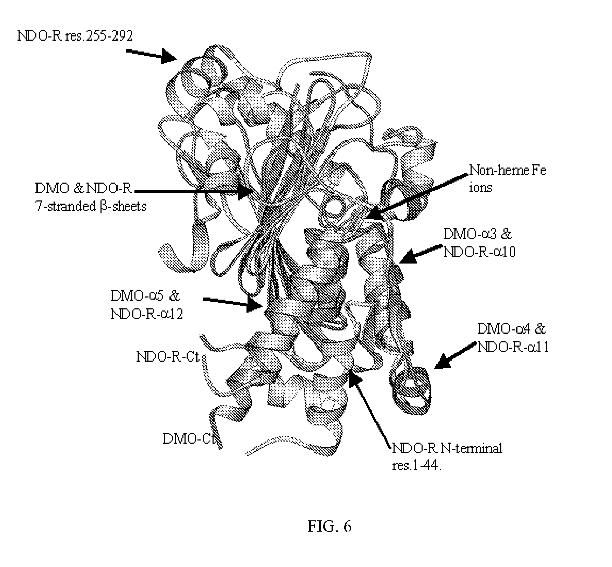

**FIG. 6****.** The Catalytic Domain of NDO-R (cyan and yellow) aligned to the Catalytic Domain of DMO (purple). The DMO catalytic domain from "DMO Crystal 5" (molecule "a", residues 125-175; 196-234; 247-343) and the NDO-R molecule "a" protein residues 1-44 and 167-440 are displayed. NDO-R residues 1-44 and 255-292 have no structural equivalent in DMO and are colored yellow; all other NDO-R residues are colored cyan. The view is approximately perpendicular to the one in FIG. 5. The C-termini for each catalytic domain are labeled, as well as several spatially similar structural elements between the two domains. This figure was prepared using the Ribbons program (Carson, 1991).

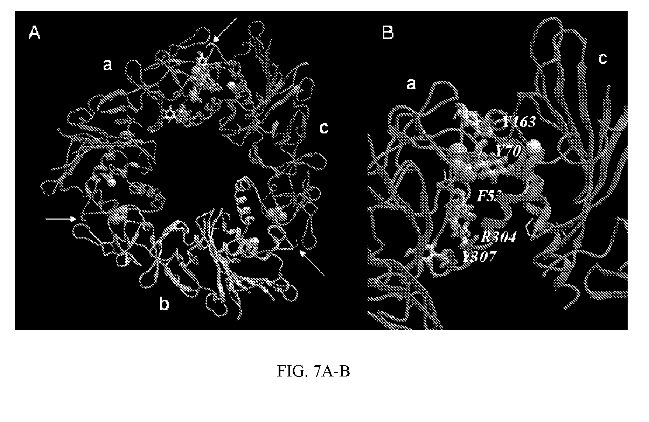

**FIG. 7 A-B**. A.) Interface region of DMO. The interface region between subunits is indicated by the white arrows. The protein is a symmetrical trimer, and the three subunits are shown in different colors. The non-heme iron domains are shown as gray balls, and the gold-blue balls are the Rieske centers. B.) Interface region between molecules "a" and "c". Some of the key residues located along the interface, F53a:Y70a:Y163c:R304c:Y307c, are indicated. Lower case letters (a-c) describe the subunits. This figure was made using the Molsoft ICM-Pro program version 3.4.

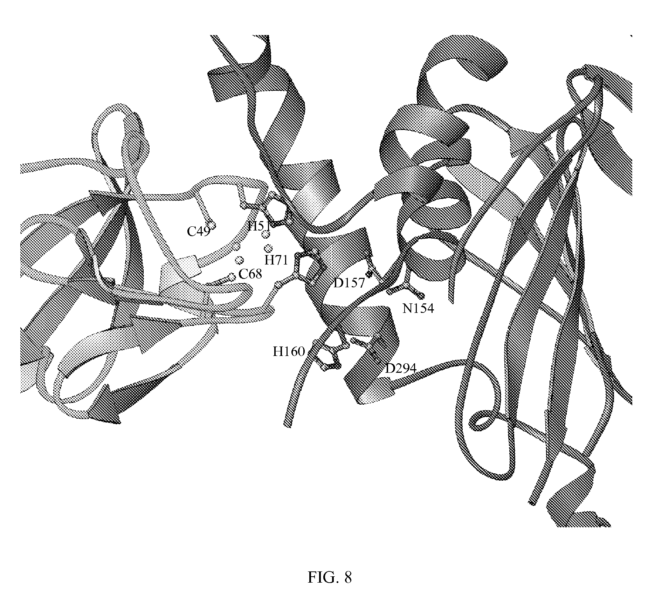

**FIG. 8****.** Active site region of "DMO Crystal 4" (crystallographic parameters found in Table 12). Molecule "b" (green) is on the left and molecule "a" (red ) is on the right. This structure reveals no evidence of the non-heme iron site being occupied. This figure was prepared using the Ribbons program (Carson, 1991).

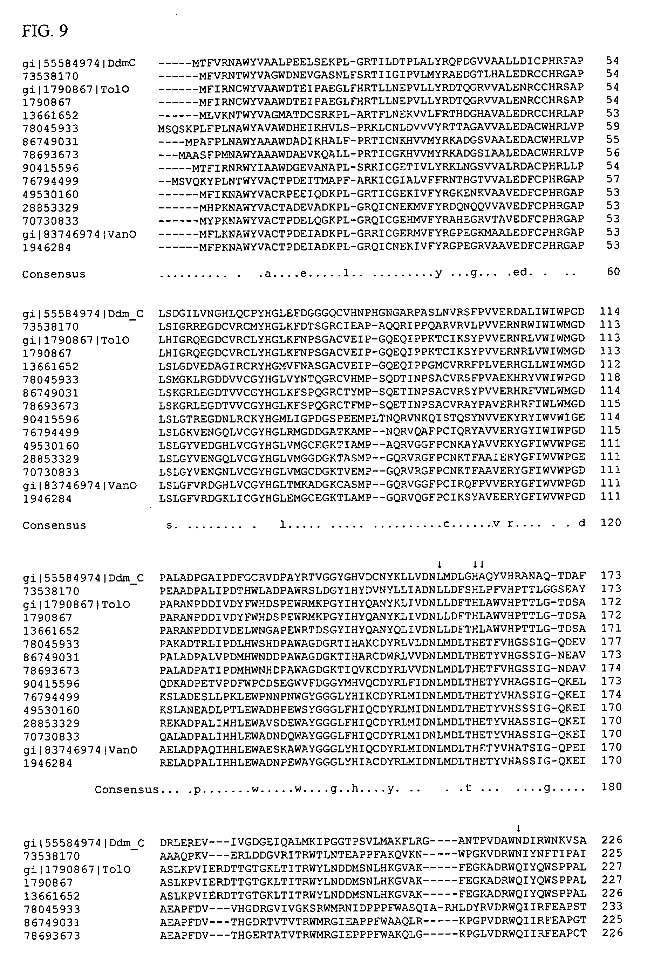

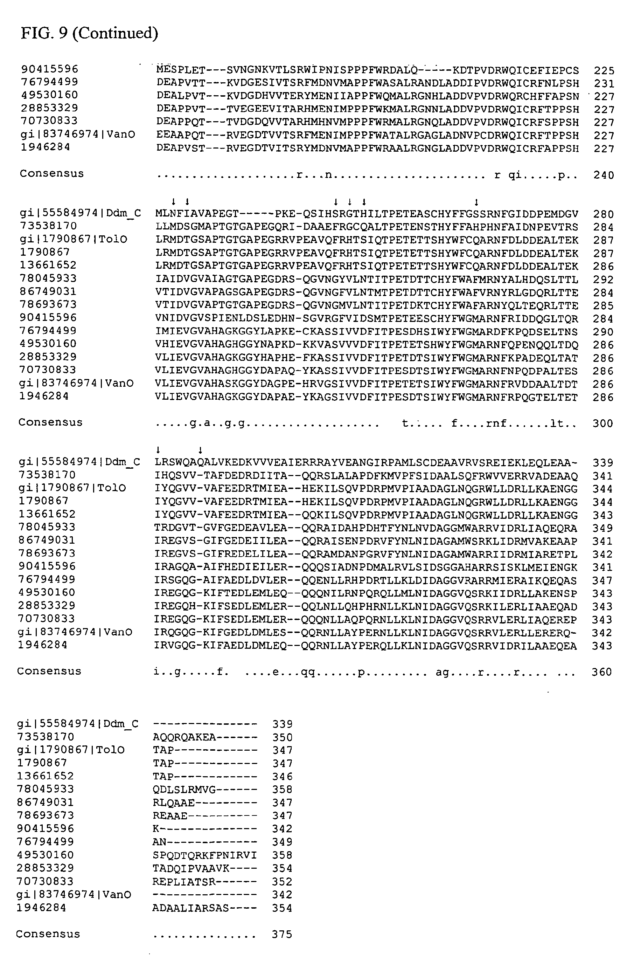

**FIG. 9****.** Sequence alignments of TolO (Toluene sulfonate monooxygenase), VanO (Vanillate O-demethylase), and some of the most closely related enzymes, to both of the these and to DMO. DMO residues with down arrow (↓) are those that interact with dicamba at its binding site within a 4Å radius. The numbering of DMO residues in the figure reflects the amino acid sequence of gi55584974, equivalent to SEQ ID NO:1, while the numbering in the text of the specification reflects the numbering based, for instance, on SEQ ID NO:3, which contains an alanine residue inserted at position 2. This alignment was made using the Muscle algorithm.

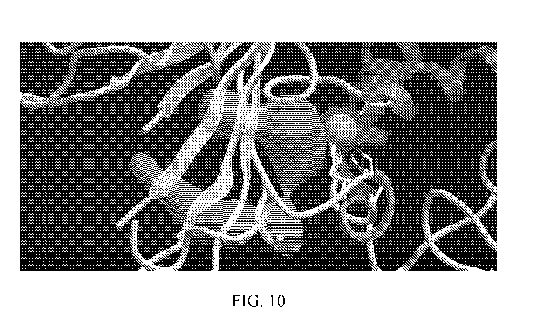

**FIG. 10****.** *In silico* identification of the dicamba binding site of 'DMO Crystal 5' using the Molsoft program as described. Dicamba binding pocket: gray mesh; green ribbon: β sheet; red ribbon: α-helix; blue sphere: non-heme iron center; sticks - H160, H165 and D294 (red - oxygen, gray - carbon, blue - nitrogen). This figure was made using the Molsoft ICM-Pro program version 3.4.

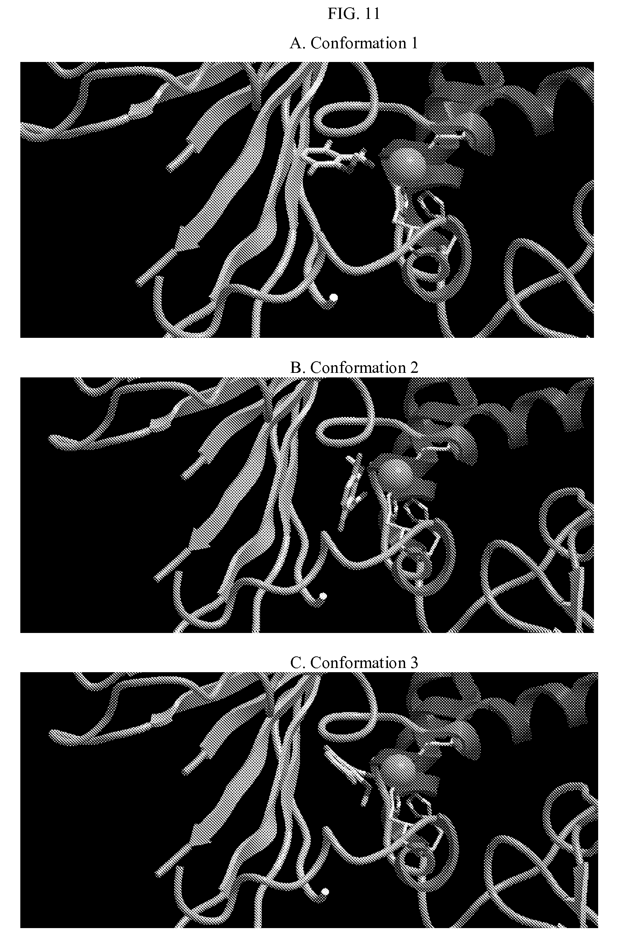

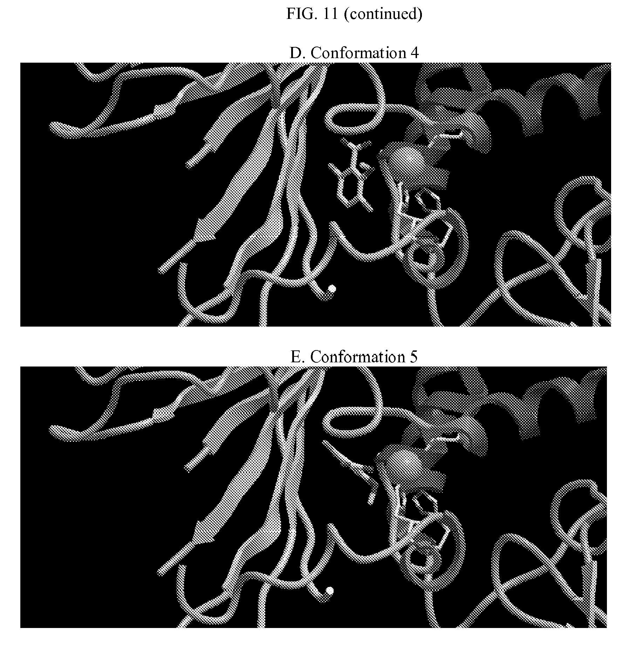

**FIG. 11 A-E.** *In silico* identification of dicamba conformations from 'DMO Crystal 5', with Molsoft Program. Molecular structures for dicamba docked into binding pocket. Green ribbon, β sheet; red ribbon, α-helix; blue sphere, non-heme iron center; sticks - dicamba and H160, H165 and D294 (red - oxygen, yellow - dicamba carbon, gray - protein carbon, blue - nitrogen, green - chlorine). Five lowest energy conformations are shown. These figures were made using the Molsoft ICM-Pro program version 3.4.

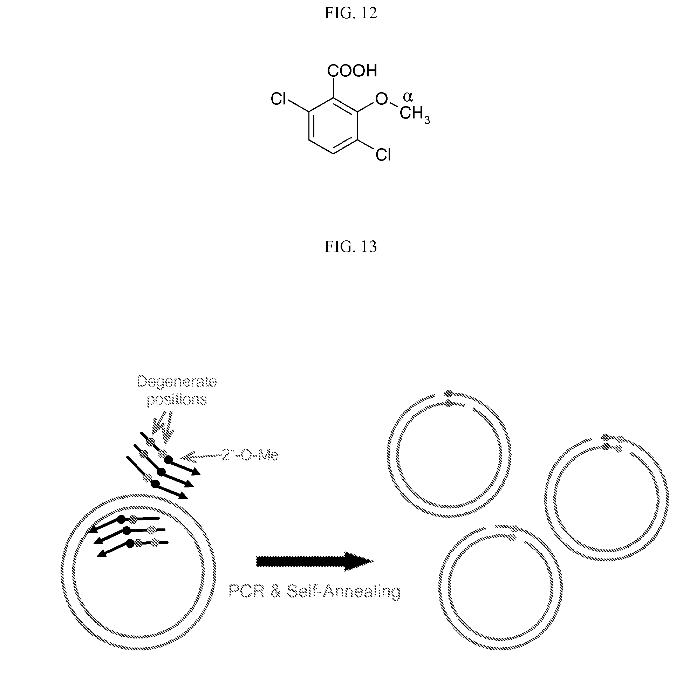

**FIG. 12****.** Chemical structure of dicamba. The α denotes the position of the oxygenation by DMO.
**FIG. 13** Schematic diagram showing degenerate oligonucleotide tail (DOT) mutagenesis approach.

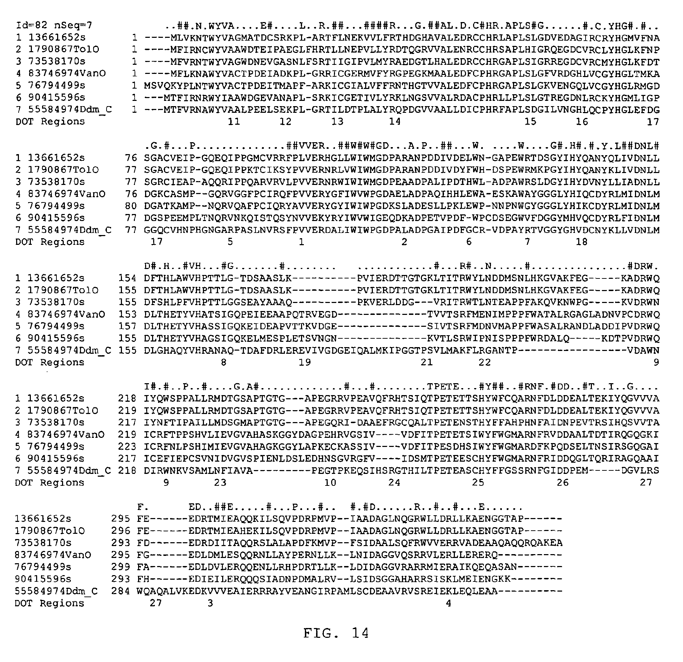

**FIG. 14****.** Sequence alignment showing location of DMO residues targeted for mutagenesis. The red boxed areas are regions where mutagenesis of any or all of the selected amino acids could be carried out in a number of combinations. This figure was made using the Molsoft ICM-Pro program version 3.4 and the ZEGA alignment algorithm therein.

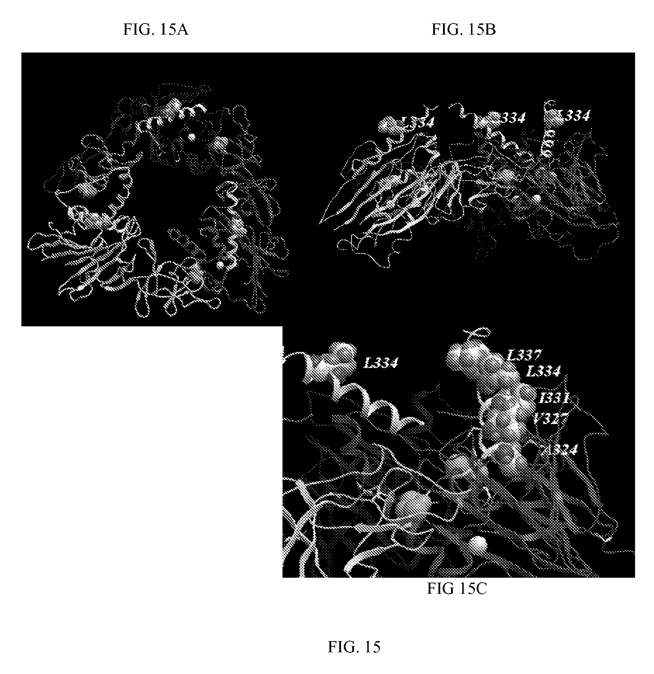

**FIG. 15****.** A-C: C-terminal helix location in the structure. The white helix in the structure is the helix in question. In the top two pictures (FIGs. 15A-B; two different orientations) the helix is oriented in the trimer crystal and the L334 residue is clearly on the solvent exposed surface. The bottom picture (FIG. 15C) shows this helix from one of the trimers in more detail. The hydrophobic surface residues are highlighted shown in Van der Waals radii depiction with colors that are as follows grey hydrogen, light grey carbon, red oxygen, and blue nitrogen (and numbered in white). This figure was made using the Molsoft ICM-Pro program version 3.4.

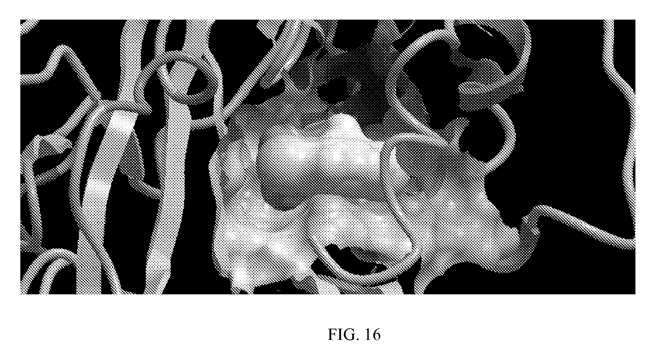

**FIG. 16****.** Structure of DMO dicamba binding pocket with dicamba bound. This figure was made using the Molsoft ICM-Pro program version 3.4.

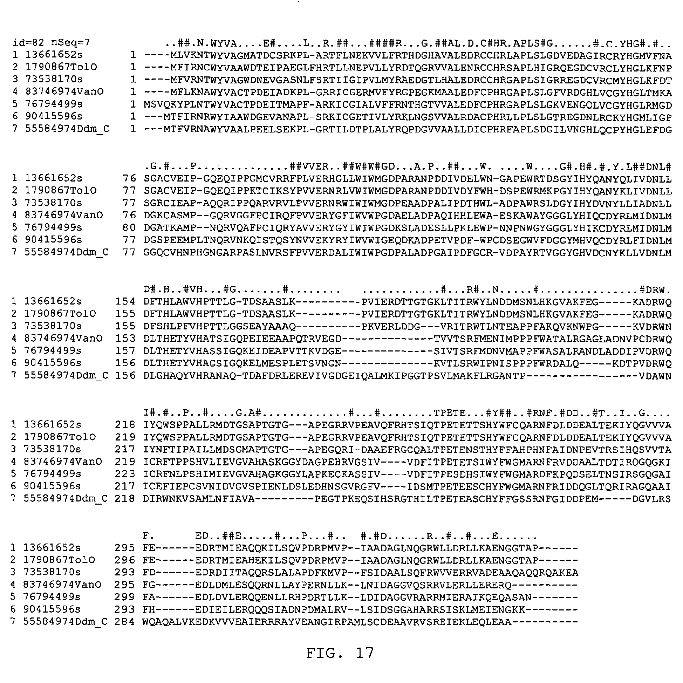

**FIG. 17****.** Additional sequence alignments of DMO and related polypeptides. This figure was made using the Molsoft ICM-Pro program version 3.4 and the ZEGA alignment algorithm therein.

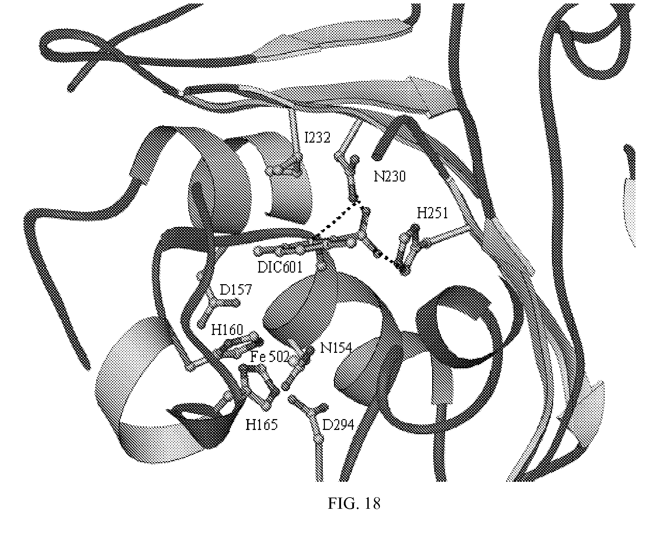

**FIG. 18****.** Structure of Dicamba Binding in "DMO Crystal 6" (molecule "c"). The structure reveals clear evidence of the non-heme iron site (502, orange sphere) being occupied, the residues which chelate this iron ion (H160, H165, & D294), and of dicamba (DIC 601) binding. (NOTE: The C1 atoms in dicamba are colored purple.) The hydrogen bonds between dicamba and N230 and H251 are rendered as dotted lines, and are listed in Table 17. Dicamba binds under 1232 and the methoxy carbon, which is lost in the conversion of dicamba to DCSA, is directed toward the non-heme 502 iron ion. This figure was prepared using the Ribbons program (Carson, 1991).

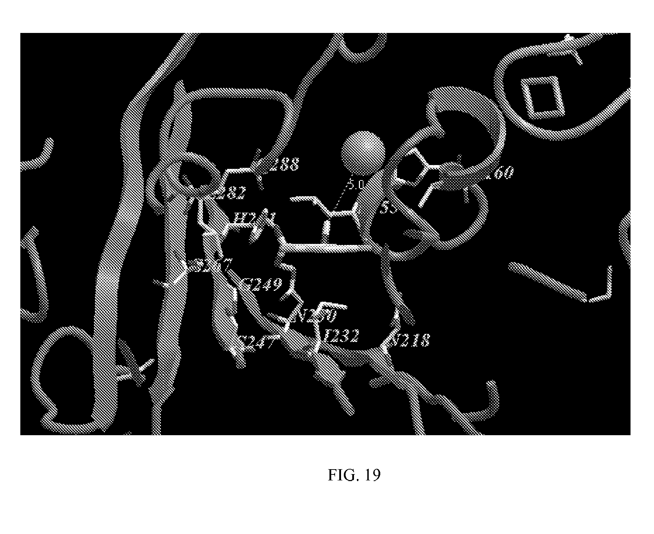

**FIG. 19****.** Dicamba binding site with bound dicamba, using atomic coordinates of Crystal 6 structure (found in Table 4). This figure was made using the Molsoft ICM-Pro program version 3.4.

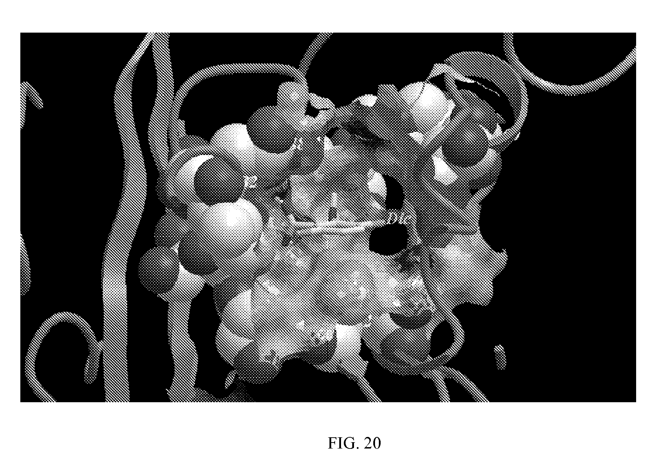

**FIG. 20****.** Model showing surface that describe the 4Å interaction residues shown as space filled structures with CPK coloring (blue-nitrogen; red-oxygen; gray-carbon). This figure was made using the Molsoft ICM-Pro program version 3.4 and the ZEGA alignment algorithm therein.

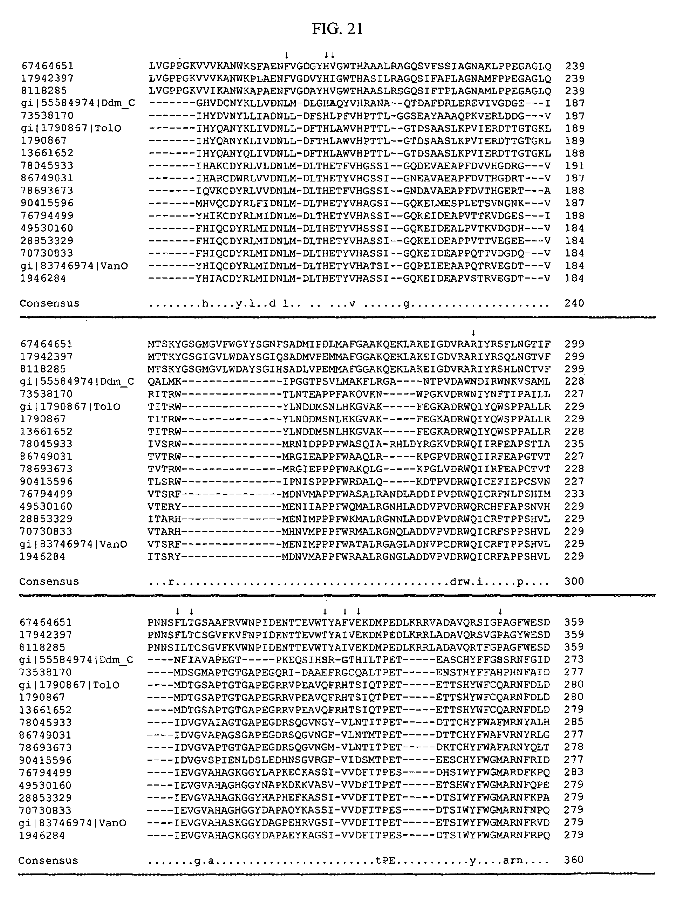

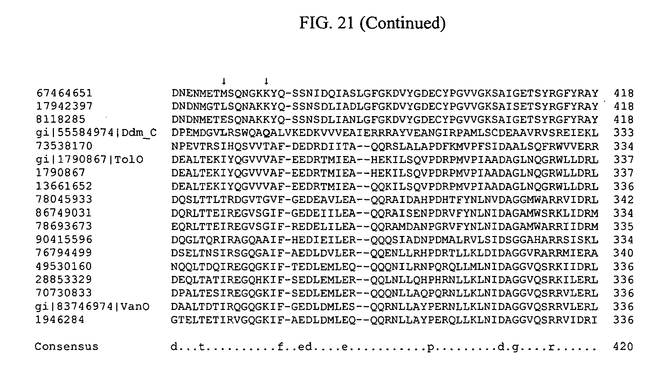

**FIG. 21****.** Alignment of TolO, VanO, with some of the most closely related enzymes, to both of the latter and to DMO, and including Nitrobenzene Dioxygenase (gi67464651) and the top two BLAST hits. Nitrobenzene Dioxygenase is of lesser identity (16%) and similarity to DMO. These additional three sequences are in addition to the sequences shown in FIG. 9. DMO residues with down arrow (↓) are those that interact with dicamba at its binding site within a 4Å radius. The numbering of DMO residues in the figure reflects the amino acid sequence of gi55584974, numbering equivalent to SEQ ID NO:1, while the numbering in the text of the specification reflects the numbering based, for instance, on SEQ ID NO:2, which contains an alanine residue inserted at position 2. This alignment was made using the Muscle algorithm.

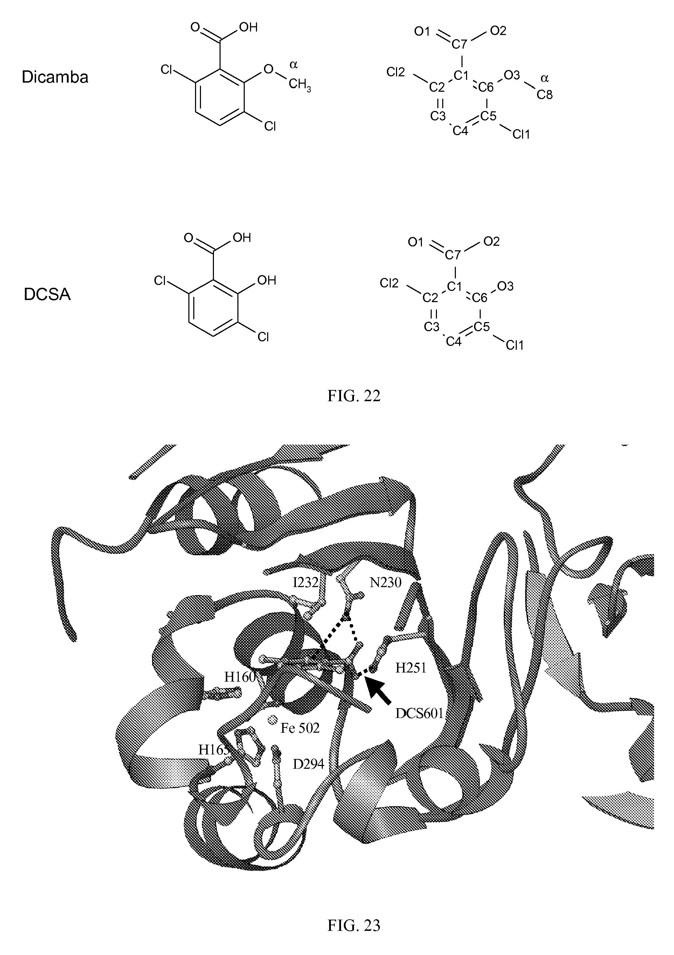

**FIG. 22****.** Structure and connectivity information for dicamba and DCSA.
**FIG. 23****.** "Dicamba" binding site with bound DCSA, using atomic coordinates of "DMO Crystal 7" structure (found in Table 5). The structure demonstrates the non-heme iron site (502, orange sphere) being occupied, the residues which chelate this iron ion H160, H165, & D294), and the site of DCSA (DCS 601) binding. C1 atoms in DCSA are colored purple. The hydrogen bonds between DCSA and N230 and H251 are rendered as dotted lines, and are listed in Table 18. DCSA binds like dicamba does in the DMO active site - under I232 and with the methoxy oxygen, which is demethylated in the conversion of dicamba to DCSA, directed toward the non-heme 502 iron ion. This figure was prepared using the Ribbons program (Carson, 1991).

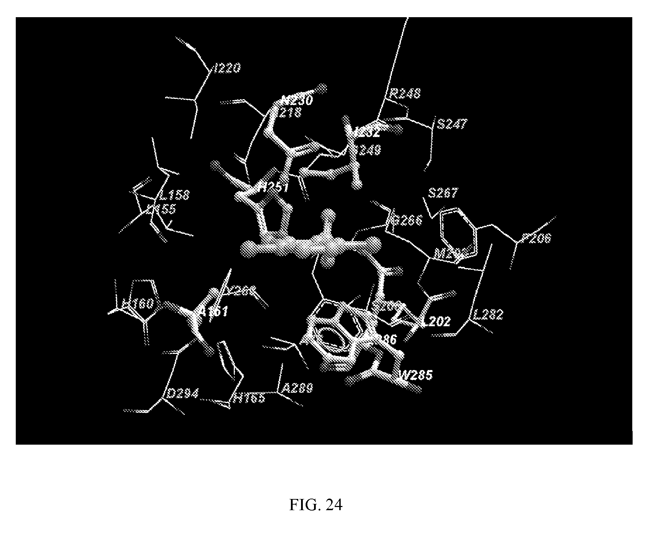

**FIG. 24** ~6Å Active site sphere of dicamba/DCSA binding site, using atomic coordinates of Crystal 12 structure (found in Table 26). This figure was made using the Molsoft ICM-Pro program version 3.5-1k.

### DETAILED DESCRIPTION OF THE INVENTION

In accordance with the invention, compositions and methods are provided for engineering molecules with dicamba binding activity. In specific embodiments the molecules comprise a variant of dicamba monooxygenase (DMO) that can be engineered based on the identification of the dicamba monooxygenase crystal structure and residues important for DMO function as described herein. Such variants may be defined in the presence or absence of a non-heme iron cofactor as well as of the substrate, dicamba. In one aspect, the invention comprises a crystallized DMO polypeptide, wherein the crystal comprises a space group of P3₂ with unit cell parameters of about a= 79-81 Å, b = 79-81 Å, and c = 158-162 Å; for instance a=80.06Å, b=80.06Å, and c=160.16Å, or a=80.56Å, b=80.56Å, and c=159.16Å; and about α=β= 90° and about γ=120°, and wherein the crystal comprises a polypeptide with a primary sequence that does not comprise SEQ ID NOs:l -3.

In another aspect, the invention comprises an isolated polypeptide with DMO activity, wherein the polypeptide sequence does not comprise SEQ ID NOs:1-3, and which when in crystalline form comprises a space group of P3₂ with unit cell parameters of about a= 79-81 Å, b = 79-81 Å, and c = 158-162 Å; for instance a=80.06Å, b=80.06Å, and c=160.16Å, or a=80.56Å, b=80.56Å, and c=159.16Å; and about α=β= 90° and about γ=120°. The asymmetric unit may be a monomer. Three monomers form a symmetric (trimer) unit, and when in crystalline form the three Rieske (Fe₂S₂) clusters of the symmetric unit may be defined as arranged about 50 Å apart in an approximately equilateral triangle. The trimer can form a lattice with a high solvent content of about 51%. The invention also relates to such an isolated polypeptide comprising an amino acid sequence with, for example, from about 20% to about 99% sequence identity with SEQ ID NOs:1-3, as determined, for instance, by BLAST (Altschul *et al.,* 1990) or another alignment method as described herein.

The invention further relates to a molecule, such as a polypeptide, displaying dicamba binding activity, as well as one also displaying DMO activity, for instance, as determined by a measurable K_{D} (see, for example, Copeland (2000)); or K_{M} (see, for example, Copeland (2000); Cleland (1990); and Johnson (1992)) for dicamba of about 0.1-500µM under physiological conditions (e.g. pH, ionic strength, and temperature) found in plants and in terrestrial and aquatic environments. In specific embodiments, the K_{D} or K_{M} for dicamba may be about 0.1-100µM.

A polypeptide or other molecule provided by the invention may also be defined as comprising a three-dimensional macromolecular structure characterized by the atomic structure coordinates of peptide backbone atoms of any of Tables 1-5, and 25-26, or a macromolecular structure exhibiting a root-mean-square difference (r.m.s.d) in α-carbon positions over the length of each of the three polypeptides that make up the asymmetric unit of less than 1.5Å with the atomic structure coordinates of Tables 1-5, and 25-26, when superimposed on the corresponding backbone atoms described by the structural coordinates of amino acid residues comprising the polypeptide, when at least 70% of the total macromolecular structure α-carbon atoms are used in the superimposition, and wherein the polypeptide does not comprise an amino acid sequence of SEQ ID NOs:1-3.

A macromolecular structure provided by the invention may also be defined as comprising one or more of: (i) a path for the donation of an electron(s) to a Rieske (Fe₂S₂) center; (ii) a macromolecular structure defining a Rieske center; (iii) a macromolecular structure defining an electron transport path from the Rieske center to a substrate binding (catalytic) site; (iv) a macromolecular structure defining a substrate binding site; (v) a macromolecular structure defining a subunit interface region; and (vi) a macromolecular structure defining a C-terminal region corresponding to residues 323-340 of SEQ ID NO:3. The invention also relates to macromolecular structures of (i) to (vi) exhibiting a root-mean-square difference (r.m.s.d) in α-carbon positions over the length of each of the three polypeptides that make up the asymmetric unit of less than 1.5Å or less than 2.0 Å with the atomic structure coordinates of Tables 1-5, and 25-26, when superimposed on the corresponding backbone atoms corresponding to each of portions (i) to (vi) of the structure described by the structural coordinates of amino acid residues comprising the polypeptide, when at least 70% of the total macromolecular structure α-carbon atoms defining the given structure of (i)- (vi) are used in the superimposition.

A conserved pocket or surface of a macromolecular structure, such as a polypeptide, may be defined as the space or surface in or on which a molecule of interest, for example a dicamba molecule, or other structure, such as a polypeptide, can interact due to its shape complementary properties. The "fit" may be spatial as that of a "lock and key", and also may address properties such as those described below for conservative acid replacement (i.e. physico-chemical structure). A conserved pocket allows for the correct positioning and orientation of a ligand or substrate for their desired binding and for the possible enzymatic activity associated with the macromolecular structure, while also possessing the appropriate electrostatic potential, e.g. proper charge(s), and/or binding property, e.g. ability to form hydrogen bond(s), for instance as donor or acceptor. Thus a conserved pocket or surface is a space with the proper shape (spatial arrangement of atoms) as well as physicochemical properties to accept, for instance, a dicamba molecule or other substrate, with a given range of specificity and affinity, and such that the space may be designed for. A conserved space or surface may be identified by use of available modeling software, such as Molsoft ICM (Molsoft LLC, La Jolla, CA). The concept of a conserved surface or complimentary space has been discussed in the art (e.g. Fersht, 1985; Dennis *et al.,* 2002; Silberstein *et al.,* 2003; Morris *et al.,* 2005).

Modification may be made to the polypeptide sequence of a protein such as the sequences provided herein while retaining enzymatic activity. The following is a discussion based upon changing the amino acids of a protein to create similar, or even an improved, modified polypeptide and corresponding coding sequences. It is known, for example, that certain amino acids may be substituted for other amino acids in a protein structure without appreciable loss of interactive binding capacity with structures such as binding sites on substrate molecules. Since it is the interactive capacity and nature of a protein that defines that protein's biological functional activity, certain amino acid sequence substitutions can be made in a protein sequence, and, of course, its underlying DNA coding sequence, and nevertheless obtain a protein with like properties. It is thus contemplated that various changes may be made in a DMO peptide sequences as described herein, and corresponding DNA coding sequences, without appreciable loss of their three-dimensional structure, or their biological utility or activity.

In making such changes, the hydropathic index of amino acids may be considered. The importance of the hydropathic amino acid index in conferring interactive biologic function on a protein is generally understood in the art (Kyte *et al.,* 1982). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of their hydrophobicity and charge characteristics (Kyte *et al.,* 1982), these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is known in the art that amino acids may be substituted by other amino acids having a similar hydropathic index or score and still result in a protein with similar biological activity, i.e., still obtain a biological functionally equivalent protein. In making such changes, the substitution of amino acids whose hydropathic indices are within ±2 is preferred, those which are within ±1 are particularly preferred, and those within ±0.5 are even more particularly preferred.

It is also understood in the art that the substitution of like amino acids can be made effectively on the basis of hydrophilicity. U.S. Patent 4,554,101 states that the greatest local average hydrophilicity of a protein, as governed by the hydrophilicity of its adjacent amino acids, correlates with a biological property of the protein. As detailed in U.S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent protein. In such changes, the substitution of amino acids whose hydrophilicity values are within ±2 is preferred, those which are within ±1 are particularly preferred, and those within ±0.5 are even more particularly preferred. Exemplary substitutions which take these and various of the foregoing characteristics into consideration are well known to those of skill in the art and include: arginine and lysine; glutamate and aspartate; serine and threonine; glutamine and asparagine; and valine, leucine and isoleucine.

Conservative amino acid substitutions may thus be identified based on physico-chemical properties, function, and Van der Waals volumes. Physico-chemical properties may include chemical structure, charge, polarity, hydrophobicity, surface properties, volume, presence of an aromatic ring, and hydrogen bonding potential, among other properties. Several classifications of the amino acids have been proposed (e.g. Taylor, 1986; Livingstone *et al.,* 1993; Mocz, 1995; and Stanfel, 1996). A hierarchical classification of the twenty natural amino acids has also been described (May, 1999). Descriptions of amino acid surfaces are known (e.g. Chothia, 1975); amino acid hydrophobicity is discussed by Zamyatin (1972); and hydrophobicity of amino acid residues is discussed in Karplus (1997). Amino acid substitutions at enzymatic active sites have also been described (e.g. Gutteridge and Thornton, 2005). Binding pocket shape has also been discussed (e.g. Morris *et al.,* 2005). Binding pockets may also be defined based on other properties, such as charge (e.g. Bate *et al.,* 2004). These teachings may be utilized to identify amino acid substitutions that result in altered (e.g. improved) enzymatic function.

It is also understood that a polypeptide sequence sharing a degree of primary amino acid sequence identity with a polypeptide that displays a similar function, such as dicamba binding activity or the presence of a Rieske domain, would possess a common structural fold, and vice versa. The term "fold" refers to the three-dimensional arrangement of secondary structural elements (*i.e.,* helices and β-sheets) in a protein. Moreover, a "folded polypeptide" refers to a polypeptide sequence, or linear sequence of amino acids, that possesses a fold. Generally, at least about 30% primary amino acid sequence identity within the region of the binding/catalytic domain would be necessary to specify such a structural fold (*e.g.* Todd *et al.,* 2001), although proteins with the same function (*i.e.* type of chemical transformation) are known that nonetheless display even less than 25% primary amino acid sequence identity when given catalytic site regions are compared. Specific structural folds and substrate binding surfaces may be predicted based on amino acid sequence similarity (e.g. Lichtarge and Sowa, 2002). In certain embodiments, a polypeptide comprising a structural fold in common with DMO comprises an amino acid sequence with from about 20% to about 99% sequence identity to the polypeptide sequence of any of SEQ ID NOs:1-3. In particular embodiments, the level of sequence identity with any of SEQ ID NOs:1-3 may be less than 95%, less than 85%, less than 65%, less than 45%, or about 30% to about 99%.

An "interface region" may be defined as that region that describes the surface between two adjacent molecules, such as neighboring polypeptide chains or subunits, wherein molecules (e.g. amino acids) interact, for example within their Van der Waals radii. The interface region in the DMO trimer bridges the two iron centers, is functionally important for contact between subunits in the homo-oligomer (trimer), and is critical for the catalytic cycle. Interfaces in proteins can be described or classified (e.g. Ofran *et al.,* 2003; Tsuchiya *et al.,* 2006; Russell *et al., 2004).*

The invention also relates to a macromolecular structure defining a path for donation of an electron to a Rieske (Fe₂S₂) center as an electron is transferred from the electron donor (e.g. ferredoxin) to the Rieske center. In specific embodiments, the macromolecular structure may be defined as a polypeptide and may be a DMO.

The invention also further relates to a polypeptide comprising a macromolecular structure defining a Rieske center domain, comprising substantially all of the amino acid residues 2-124 of a polypeptide sequence numbered corresponding to SEQ ID NO:2 or SEQ ID NO:3, in which the C49, H51, C68, and H71 residues of one monomer, or ones corresponding to them, participate in the formation of the Rieske cluster in DMO.

In yet another embodiment, the invention relates to a DMO comprising a macromolecular structure defining an electron transport path from the Rieske center of one monomer to a non-heme iron at the substrate binding (catalytic) site in a second monomer that comprises the trimeric DMO asymmetric unit, comprising substantially all of the following amino acid residues: H71 in one monomer; and in the second monomer: D157, the residues which chelate the non-heme iron: H160, H165, and D294, and a residue which plays a role in such chelation, N154. This structure is shown for instance in FIG. 2. The numbering of residues corresponds to that of, for instance, SEQ ID NO:2 or SEQ ID NO:3.

The invention also relates to a molecule, such as a DMO polypeptide, comprising a substrate (dicamba) binding site which comprises the following characteristics: (i) a volume of 175-500Å³; (ii) electrostatically accommodative of a negatively charged carboxylate; (iii) accommodative of at least one chlorine moiety if present in the substrate; (iv) accommodative of a planar aromatic ring in a substrate; and (v) displays a distance from an iron atom that activates oxygen in the DMO polypeptide to a carbon of the methoxy group of the substrate, sufficient for catalysis, of about 2.5Å to about 7Å.

The substrate binding site/catalytic domain may also be defined as comprising a macromolecular structure defining a substrate binding pocket, within 4Å of the bound substrate/product, and comprising substantially all of the amino acid residues L155, D157, L158, H160, A161, H165, R166, A169, Q170, D172, A173, S200, A216, W217, N218, 1220, N230, 1232, A233, V234, S247, G249, H251, S267, L282, W285, Q286, A287, Q288, A289, L290, and V291. Additional residues are within a 5-6Å sphere around the bound substrate/product, including S200, L202, M203, and F206. The active site and the dicamba/DCSA substrate/product binding site nearly overlap. The catalytic domain extends between about residues corresponding to those numbered 125-343 from the N-terminus of a full length DMO polypeptide, for instance as numbered in SEQ ID NO:2 or SEQ ID NO:3.

The invention further relates to a DMO comprising a macromolecular structure defining a subunit interface region, also termed an "interaction domain", comprising substantially all of the amino acid residues V325, E322, D321, C320, L318, M317, A316, P315, R314, I313, V308, Y307, R304, I301, A300, V297, V296, E293, R166, V164, Y163, H160, G159, D157, N154, D153, R98, L95, S94, G89, G87, H86, P85, N84, L73, G72, H71, Y70, P69, C68, Q67, D58, P55, A54, F53, R52, H51, P50,148, D47, L46, P31, T30, and D29, numbered corresponding to SEQ ID NO:3.

The invention also relates to a macromolecular structure defining a dicamba binding site, comprising substantially all of the amino acid residues L155, D157, L158, H160, A161, H165, R166, A169, Q170, D172, A173, A216, W217, N218, 1220, N230, 1232, A233, V234, S247, G249, H251, S267, L282, Q286, A287, Q288, A289, and V291 numbered corresponding to SEQ ID NO:3, wherein the atomic structure coordinates for these residues are as listed in Table 4.

The invention further relates to a macromolecular structure defining a DCSA binding site, comprising substantially all of the amino acid residues L155, D157, L158, H160, A161, H165, R166, A169, Q170, D172, A173, A216, W217, N218, 1220, N230, I232, A233, V234, S247, G249, H251, S267, L282, Q286, A287, Q288, A289, and V291 numbered corresponding to SEQ ID NO:3, wherein the atomic structure coordinates for these residues are as listed in Table 5.

The invention further relates to an isolated polypeptide comprising dicamba monooxygenase (DMO) activity, wherein the polypeptide comprises a DMO enzyme having the following sequence domain near residue W285 of the primary peptide sequence numbered for instance according to SEQ ID NO:3: -W-X₁-X₂-X₃-X₄-L- (SEQ ID NO:152), in which X₁ is Q, F, or H; X₂ is A, D, F, I, R, T, V, W, Y, C, E, G, L, M, Q, or S; X₃ is Q, G, I, V, A, C, D, H, L, M, N, R, S, T, or E; and X₄ is A, C, G, or S. The isolated polypeptide may comprise a DMO enzyme having the following sequence domain near residue A169: -N-X₁-Q-, in which X₁ is A, L, C, F, F, I, N, Q, S, V, W, Y, M or T. In yet other embodiments, the isolated polypeptide comprises a DMO enzyme comprising a sequence domain near residue N218: -W-X₁-D- in which X₁ is N, K, A, C, E, I, L, S, T, W, Y, H, or M.

The invention also relates to an isolated polypeptide exhibiting an increased level of DMO activity relative to the activity of a wild type DMO when measured under identical, or substantially identical, conditions. For instance, the isolated polypeptide may exhibit at least 105%, 110%, 120%, 130%, 140%, 150%, or more of the activity of a wild type DMO enzyme when measured under identical, or substantially identical, conditions. Thus, for instance, the isolated polypeptide may comprise a DMO enzyme having a sequence domain near residue R248 which comprises: X₁-X₂-G-X₃-H (SEQ ID NO:153) in which X₁ is S, H, or T; X₂ is R, Q, S, T, F, H, N, V, W, Y, C, I, K, L, or M; X₂ is R, Q, S, T, F, H, N, V, W, Y, C, I K, L, or M, and X₃ is T, Q, or M. The isolated polypeptide may also, or alternatively, comprise a substitution in residue X₂, numbered according to the numbering of SEQ ID NO:2 or SEQ ID NO:3, and selected from the group consisting of: R248C, R248I, R248K, R248L, R248M. Or, the isolated may comprise a DMO enzyme comprising one or more substitution(s) in residues numbered according to the numbering of SEQ ID NO:2 or SEQ ID NO:3, selected from the group consisting of: A169M, N218H, N218M, G266S, L282I, A287C, A287E, A287M, A287S, and Q288E.

The invention further relates to a macromolecular structure defining a DCSA binding site, comprising substantially all of the amino acid residues L155, D157, L158, H160, A161, H165, R166, A169, Q170, D172, A173, A216, W217, N218, I220, N230, I232, A233, V234, S247, G249, H251, S267, L282, W285, Q286, A287, Q288, A289, L290, and V291 numbered corresponding to SEQ ID NO:3, wherein the atomic structure coordinates for these residues are as listed in Tables 25-26.

The invention still further relates to a plant cell exhibiting tolerance to the herbicidal effect of dicamba comprising a polypeptide with DMO activity, wherein the polypeptide sequence does not comprise SEQ ID NO:1, SEQ ID NO:2, or SEQ ID NO:3, and in which the polypeptide, when in crystalline form, comprises a space group of P3₂ with unit cell parameters of about a= 79-81 Å, b = 79-81 Å, and c = 158-162 Å, for instance a=80.06Å, b=80.06Å, and c=160.16Å, or a=80.56Å, b=80.56Å, and c=159.16Å; and about α=β= 90° and about γ=120°

In another aspect, the invention relates to a method for determining the three dimensional structure of a crystallized DMO polypeptide. Methods for obtaining the polypeptide in crystalline form are disclosed, as are methods for analysis by diffraction and spectrophotometric methods, including analysis of the resulting diffraction and spectrophotometric data. Such analysis results in sets of atomic coordinates that define the three dimensional structure of the active DMO structure and of a crystal lattice comprising the structure, and are found, for instance, in Tables 1-5, and 25-26. Such structures have been determined for DMO in the presence and absence of Fe²⁺, as well as in the presence or absence of the substrate (dicamba) and the product (DCSA).

In yet another aspect, the invention relates to computer readable storage media comprising atomic structural coordinates of any of Tables 1-5, and 25-26, or a subset of a one of these tables, representing for instance the three dimensional structure of crystallized DMO, or the dicamba binding domain thereof, and to a computer programmed to produce a three dimensional representation of the data comprised on such a computer readable storage medium.

Methods for identifying the potential for an agent to bind to the substrate binding pocket of DMO are also related to the invention. Such an agent may be an inhibitor of DMO activity, acting for instance as a synergist or potentiator for dicamba's herbicidal activity, or may also demonstrate herbicidal activity. Such methods may be carried out by one of skill in the art by computer-based modeling of the three dimensional structure of such an agent in the presence of a three dimensional model of DMO structure, and analyzing the ability of such an agent to bind to DMO, and also in certain embodiments to undergo catalysis by DMO.

The invention further relates to methods for utilizing the physico-chemical characteristics and three dimensional structure of the substrate binding pocket of DMO to identify molecules useful for dicamba degradation, water purification, degradation of other xenobiotics, or identification of analogous structures, including polypeptides that are functional homologs of DMO, from closely or otherwise related organisms, or are obtained via mutagenesis.

### EXAMPLES

The following examples are included to illustrate embodiments of the invention. It should be appreciated by those of skill in the art that the techniques disclosed in the examples that follow represent techniques discovered by the inventor to function well in the practice of the invention. However, those of skill in the art should, in light of the present disclosure, appreciate that many changes can be made in the specific embodiments which are disclosed and still obtain a like or similar result without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

### Example 1

### Summary of Results

The DMO X-ray crystal structure was solved by multiwavelength anomalous dispersion (MAD) methods. Four basic types of DMO structures were obtained first: (a) DMO with the non-heme iron site disordered and unoccupied: ("DMO Crystal 3", "DMO Crystal 4"); (b) DMO with the non-heme iron site ordered and occupied ( "DMO Crystal 5"); (c) DMO with the non-heme iron site ordered and occupied and dicamba bound ("DMO Crystal 6"); and (d) DMO with DCSA bound ("DMO Crystal 7"). Subsequently, refined crystal structures of (e) DMO co-crystallized with dicamba and with cobalt at the non-heme iron site ("DMO Crystal 11"); (f) DMO co-crystallized with DCSA and with cobalt at the non-heme iron site ("DMO Crystal 12"); were also determined. The DMO structures lacking ordered and occupied non-heme iron sites are DMO Crystal 3, with R_{work}=30.8% and R_{free}=35.0% for 20-3.0 Å data, and DMO Crystal 4, with R_{work}=31.3% and R_{free}=36.6% for 20-3.2 Å data. The DMO structure with the occupied non-heme iron site is DMO Crystal 5, which has R_{work}=33.4% and R_{free}=37.3% for 20-2.65 Å data. The DMO structure with the occupied non-heme iron sites and dicamba bound is DMO Crystal 6, which has R_{work}=31.6% and R_{free}=35.7% for 20-2.7 Å data. Detailed refinement information for these crystals is listed in Tables 11-13. The DMO Crystal 5 structure, which contains an occupied and ordered non-heme iron site in all three molecules of the crystallographic asymmetric unit, is the DMO structure used for several further structural comparisons and assessments. Residue names, atom names, and connectivity conventions used in these protein structure determinations as described below follow Protein Data Bank (PDB) standards (deposit.rcsb.org/het_dictionary.txt; Berman *et al.,* 2000). The connectivity information for dicamba (residue name "DIC") and for DCSA (residue name "DCS") is listed in FIG. 22.

DMO possesses a unique Rieske non-heme iron oxygenase ("RO"; Gibson & Parales, 2000) structure that is in some aspects similar to, yet distinct from, other RO enzymes of known structure. Detailed descriptions of the DMO quaternary structure, Rieske domain, catalytic domain, the electron transfer pathway, substrate biding pocket, interaction domain, and C-terminal helix region are provided below (Sections A-G).

### A. Quaternary structure of DMO is compositionally like other RO α subunit structures

The three-fold symmetric arrangement of DMO oxygenase molecules in the crystallographic asymmetric unit, as shown in FIG. 1A-B, is structurally similar to the arrangement of α subunits in other known RO structures to date (e.g. Ferraro *et al.,* 2005). In the figure, helices are rendered as coils and β-strands are rendered as arrows. Non-regular structure is rendered as rope. The Fe₂S₂ clusters are displayed with orange spheres for iron atoms and yellow spheres for the sulfur atoms. The Cys49, His51, Cys68, and His71 residues that bind the clusters are displayed. The N- and C-termini of each monomer are indicated in FIG. 1A. The N- and C- termini within each monomer are 13 Å apart. The 3-fold axis relating the monomers in the trimer is located in the center of the top figure, equidistant from all three N-termini, and perpendicular to the plane of the figure; in the lower figure the 3-fold axis is vertical and in the plane of the figure. The Fe₂S₂ clusters are arranged ~50 Å apart from one another, and together define an approximate equilateral triangle.

Moreover in the DMO-non-heme iron structure (FIG. 2; also see Example 3 below), the Rieske cluster center of one subunit is ~12 Å from the non-heme iron atom in the neighboring subunit, which arrangement is also observed in other RO structures (e.g. Ferraro *et al.,* 2006).

The DMO structure is also similar in composition to other RO α subunit structures (Ferraro *et al.,* 2005). Other RO oc subunit structures possess a Rieske Fe₂S₂ cluster domain and a mononuclear iron-containing catalytic domain, and the DMO-iron soak structure possesses these elements as well. In DMO, the Rieske domain is found from residue 2-124 and contains the 501- Fe₂S₂ cluster. The catalytic domain extends from residue 125-343, and contains the 502-non-heme Fe (Numbering of iron atoms as per PDB format). In the DMO-non-heme iron site structure (DMO Crystal 5), residues 176-195 and 235-246 are disordered, and not visible in the structure. The structure for molecule A of DMO Crystal 5, with the Rieske and catalytic domains clearly visible, is displayed in FIG. 3. The pie slice shape of the DMO monomer is also similar to that of other RO α subunit structures (Ferraro *et al.,* 2005).

It is noteworthy that DMO is significantly smaller in size to the α subunits of other structurally characterized RO enzymes. This DMO construct for crystallography contained 349 amino acids, and contains 343 if the C-terminal hexa-His tag is excluded. The α-subunits for other RO enzymes with Rieske domains when compared to DMO are significantly larger in size. The naphthalene 1,2-dioxygenase from *Rhodococcus sp.* (PDB entry 2B24; SEQ ID NO:20) α-subunit contains 470 amino acids; the naphthalene 1,2-dioxygenase from *Pseudomonas sp.* (PDB entry 1NDO; SEQ ID NO:22) α-subunit has 449 amino acids; the nitrobenzene dioxygenase from *Comamonas sp.* (PDB entry 2BMO; SEQ ID NO:17) α-subunit contains 447 amino acids; and the biphenyl dioxygenase from *Rhodococcus sp.* (PDB entry 1ULI; SEQ ID NO:21) α-subunit has 460 amino acids.

### B. Rieske domain

The DMO Rieske domain (residues 2-124) from DMO Crystal 5 is displayed in FIGs. 3 and 4. Residues Cys49, His51, Cys68 and His71 participate in the formation of the Fe₂S₂ Rieske cluster in DMO. FIG. 4 also contains the Rieske domain of naphthalene 1,2-dioxygenase (NDO-R) from *Rhodococcus sp.* (PDB entry 2B24; SEQ ID NO:20), containing residues 45-166, aligned to the DMO Rieske domain. Tables 6 & 7 list the key secondary structural elements (i.e., β-strands, etc.), in the Rieske domains of DMO and NDO-R, respectively.

The domain alignment results and a review of FIG.4 and tables 6 & 7 indicate that the Rieske domain of DMO shares the same basic fold as that in NDO-R, though the two have some structural differences. By the term "fold", we refer to the three-dimensional arrangement of secondary structural elements (*i.e.,* helices and β-sheets) in a protein. Moreover, a 'folded polypeptide' refers to a polypeptide sequence, or linear sequence of amino acids, that possesses a fold. The r.m.s.d. in α-carbon positions between the two Rieske domain structures using 84 corresponding residues is 1.90Å; the fact that 68% of the Cα atoms in the DMO Rieske domain have an r.m.s.d. < 2 Å with those in NDO-R indicates that the two domains share notable structural similarities. In addition, FIG.4 reveals that both domains are comprised of three β sheets, each sheet containing antiparallel β-strands, located in topologically equivalent locations. Also, the Rieske clusters are in approximately equivalent locations. FIG. 4, however, also reveals noteworthy Cα-trace spatial gaps between the two domains and that the composition of β-sheets 2 &3, though similar, is not identical in the two structures. These structural differences help one understand why the NDO-R Rieske domain, and likely the Rieske domains of other ROs of known structure, was not useful in molecular replacement phasing with the DMO X-ray data. All in all, the Rieske domain of DMO is structurally distinct from that observed in NDO-R even though it does share a basic fold motif with it.

**Table 6. DMO Rieske domain secondary structural elements**

| Folding element | Residue range |
|---|---|
| β1 | 10-12 |
| β2 | 23-27 |
| β3 | 30-36 |
| β4 | 42-46 |
| β5 | 60-62 |
| β6 | 65-67 |
| β7 | 74-75 |
| β8 | 81-82 |
| β9 | 102-105 |
| β10 | 108-111 |

**Table 7. NDO-R Rieske domain secondary structural elements**

| Folding element | Residue range |
|---|---|
| β2 | 47-51 |
| β3 | 60-66 |
| β4 | 69-75 |
| β5 | 81-85 |
| β6 | 100-102 |
| β7 | 105-107 |
| β8 | 114-116 |
| β9 | 121-122 |
| β10 | 147-151 |
| β11 | 154-158 |

### C. Catalytic domain

The structure of the catalytic domain in DMO Crystal 5, which extends from residues 125-343, is displayed in FIGs. 3, 5, and 6. FIGS. 5 and 6 also contain the catalytic domain of naphthalene 1,2-dioxygenase from *Rhodococcus sp.* (PDB entry 2B24; SEQ ID NO:20), comprised of residues 1-44 and 167-440, aligned to the DMO catalytic domain. The r.m.s.d. in α-carbon positions between the two catalytic domain structures, using 106 corresponding residues, is 1.76Å. The secondary structural elements in the DMO and NDO-R catalytic domains are listed in Tables 8 and 9, respectively. In addition, Table 10 contains the Rieske and catalytic domain composition of seven Rieske oxygenase structures from the PDB, as well as of DMO.

A consideration of the alignment results immediately suggests that there are both key structural commonalities and differences between the catalytic domains of DMO and NDO-R. Clearly, 106 Cα atoms in the two aligned domains have close spatial positions relative to one another; however, this represents only 48% of the residues in the DMO catalytic domain. A close examination of FIGs. 5 and 6 allows one to better appreciate the structural commonalities and differences in the two domains. The central secondary structural element in both domains is a seven-stranded antiparallel β-sheet; in DMO this sheet is comprised of β11-β17-β16-β15-β14-β13-β12, and in NDO-R, this sheet is comprised of β13-β19a/b-β18-β17-β16-β15-β14. The spatial orientation and sequential β-strand threading of these two sheets is very similar. FIGs. 5 & 6 also reveal structural element overlaps between the two domains involving helices: DMO-α5 and NDO-R-α12; DMO-α3 and NDO-R-α10; DMO-α4 and NDO-R-α11. In addition, the two remaining α-helices in DMO catalytic domain, α1 and α2, have spatial orientations that map to helices in the NDO-R catalytic domain: DMO-α1 and NDO-R-α5; DMO-α2 and NDO-R-α6. Thus, every defined secondary structural element in the DMO catalytic domain (Table 8) maps to a corresponding element in NDO-R catalytic domain (Table 9), even if the spatial overlap is not always very close.

The non-heme iron (Fe) ions in the aligned domains are also in a similar location. FIG. 2 indicates that the non-heme iron in DMO is chelated by two His residues (His160 and His165) and an Asp residue (Asp294), which has been observed in all RO structures (Ferraro *et al,* 2006). Additionally, while the Asn154 side chain ND2 atom is on average 3.3 A from the non-heme iron in the DMO structures, which is longer than a standard coordinating ligand distance, the DMO structure (FIG. 2) and the observation that the N154A mutant has only 2% activity relative to the parent enzyme (Table 21) strongly indicates that N154, plays an ancillary role in non-heme iron chelation, metal site stability, and possibly in the electron transfer process.

FIGs. 5 and 6 also reveal some notable structural differences between the DMO and NDO-R catalytic domains. In particular, NDO-R's catalytic domain contains some key 'structural additions' which the DMO catalytic domain lacks, and the two most notable ones are colored yellow. First, NDO-R's catalytic domain is defined not only by residues from the C-terminal portion of its sequences, residues 167-440, but also by a 44-residue, mostly helical (containing α1 and α2) peptide segment from its N-terminus. The DMO catalytic domain contains no contribution from the residues N-terminal to 125, because all of these contribute to its Rieske domain only. Moreover, DMO appears unique among Rieske oxygenase structures in that its catalytic domain comprises only a single, contiguous, C-terminal segment of polypeptide. If one examines Table 10, which lists the Rieske oxygenase compositions of seven entries from the PDB, all of these possess catalytic domains with contributions from two non-contiguous polypeptides - a small peptide segment from the N-terminus and a larger segment from the C-terminal end. NDO-R also contains a large, helix-containing (i.e., α5-α6) loop involving residues 255-292, colored yellow in FIGs. 5 and 6, which follows β13 and precedes β14 in the 7-stranded sheet, which DMO lacks. The NDO-R catalytic domain also has more helical structure in front of its central sheet than does DMO and additional secondary structural elements compared to DMO.

All in all, while the catalytic domains of DMO and NDO-R possess a common 7-stranded antiparallel β-sheet, and have good to reasonable spatial overlaps in five helical regions and in the location of the non-heme iron binding sites, less than 50% of the Cα's in the DMO catalytic domain align well with those in NDO-R. FIGs.5 and 6 reveal a significant amount of helical and loop structure not shared by DMO and NDO-R. Moreover, the DMO catalytic domain is unique among those of other RO enzymes in that its catalytic domain contains no contribution from N-terminal peptide, being defined only by a contiguous stretch of polypeptide. These significant structural differences are likely why the catalytic domain of NDO-R, or that of other RO enzymes with Rieske domain sequence similarity to DMO, could not be successfully used in MR phasing with DMO X-ray data. These results, along with the fact that the catalytic domain sequence of DMO shares no notable sequence similarity with the catalytic domain sequences of other known RO enzymes, clearly indicate that the catalytic domain of DMO is structurally distinct from the catalytic domain structures of other known RO enzymes, and may represent a unique, minimalist catalytic domain fold for an RO enzyme.

**Table 8. DMO catalytic domain secondary structural elements.**

| Folding element | Residue Range |
|---|---|
| β11 | 138-144 |
| α1 | 148-155 |
| α2 | 161-164 |
| β12 | 200-201 |
| β13 | 205-208 |
| β14 | 217-223 |
| β15 | 227-233 |
| β16 | 250-256 |
| β17 | 260-266 |
| α3 | 294-302 |
| α4 | 305-310 |
| α5 | 323-341 |

**Table 9. NDO-R catalytic domain secondary structural elements.**

| **Folding Element** | **Residue Range** | **Folding Element** | **Residue Range** |
|---|---|---|---|
| α1 | 4-16 | α8 | 282-288 |
| β1 | 22-24 | β16 | 293-298 |
| α2 | 31-40 | β17 | 302-309 |
| α3 | 167-170 | β18 | 318-329 |
| α4 | 174-181 | β19α | 333-338 |
| β12 | 188-190 | β19β | 341-343 |
| β13 | 195-199 | α9 | 348-361 |
| α5 | 204-212 | α10 | 369-382 |
| α6 | 222-227 | α11 | 386-389 |
| β14 | 240-241 | β20 | 392-394 |
| β15 | 250-254 | α12 | 422-436 |
| α7 | 270-279 | | |

**Table 10. Rieske & Catalytic Domain Compositions of Rieske Oxygenases of Known Structure.**

| **PDB ID** | **Rieske domain Amino acid (AA) extents** | **Rieske domain AA Size (max.)** | **Catalytic domain AA extents** | **Catalytic domain AA size (max.)** |
|---|---|---|---|---|
| 1NDO (SEQ ID NO:22) | 38-158 | 121 | 1-37; 159-440 | 319 |
| 2B24 (SEQ. ID NO:20) | 45-166 | 122 | 1-44; 167-440 | 318 |
| 1ULJ, 1ULI (SEQ. ID NO:21) | 57-166 | 110 | 17-57; 167-451 | 326 |
| 2BMO, 2BMQ (SEQ. ID NO:17) | 38-158 | 121 | 3-37; 159-439 | 316 |
| IWQL (SEQ. ID NO:43) | 41-191 | 152 | 19-40; 192-459 | 316 |
| 1Z03 (SEQ. ID NO:44) | 40-155 | 116 | 16-39; 156-442 | 311 |
| 1WW9 (SEQ. ID NO:45) | 27-143 | 117 | 1-26; 144-389 | 272 |
| | | | | |
| DMO Crystal 5 (SEQ ID NO:3) | 2-124 | 123 | 125-343 | 219 |

| | | | | |
|---|---|---|---|---|
| PDB entries: INDO (naphthalene 1,2-dioxygenase; Kauppi *et al.,* 1998); 2B24 (naphthalene 1,2-dioxygenase from *Rhodoccus sp.;* Gakhar *et al.,* 2005); 1ULJ (biphenyl dioxygenase; Furusawa *et al.,* 2004); 2BMO or 2BMQ (nitrobenzene dioxygenase; Friemann *et al.,* 2005); 1WQL (cumene dioxygenase from *Pseudomonas fluorescens* 1P01; Dong *et al.,* 2005); 1Z03 (2-oxoquinoline 8-monooxygenase component; Berta *et al,* 2005); 1WW9 (carbazole 1,9-dioxygenase, terminal oxygenase; Nojiri *et al.,* 2005) | | | | |

### D. Electron transfer

In the DMO-Fe⁺² soak crystal structure, DMO Crystal 5, the DMO Rieske domain of one subunit is ~12 Å away from the non-heme iron site in another subunit (FIG. 2), as is seen in other RO structures (Ferraro *et al,* 2006), consistent with the path of electron flow. More specifically, the DMO-Fe⁺² soak crystal structure reveals that electrons flow from the Fe₂S₂ cluster His71 side chain nitrogen in one molecule, to the following residues in the neighboring molecule: Asp157, His160, and then to the non-heme iron site (FIG. 2). Coordinating residues at the non-heme iron site -His160, His165 and Asp294 also play a role in the electron transport path, as well as the nearby Asn154. From the non-heme iron (Fe 502), the electrons would flow to the oxygen in order to activate it for the oxygenase reaction, as is the case in other RO structures with substrates or substrate analogues structures (Ferraro *et al.* 2005). Example 6 describes the electron transfer path from the Rieske center to a dicamba molecule, using the atomic coordinates of Crystal 5.

### E. Dicamba and DCSA binding

The DMO Crystal 6 structure reveals that dicamba binds in the same, chemically relevant orientation in molecules "b" and "c". Dicamba binds in a cavity under Ile232, and it is oriented by three key hydrogen bonds involving Asn230 and His251 with dicamba (FIG. 18). The Asn230 side chain N atom engages in a hydrogen bond with the upper of the two carboxylate oxygens in dicamba; the Asn230 side chain N also engages in a hydrogen bond with the methoxy O of dicamba. The third hydrogen bond is between the His251 side chain nitrogen and the lower of the two carboxylate oxygens of dicamba (FIG. 18). These three hydrogen bonds orient dicamba so that the methoxy carbon is directed at the non-heme iron ion. The distance from the non-heme iron ion to the methoxy carbon is 5.1- 5.3Å in molecules "b" and "c". This methoxy carbon-non-heme distance provides ample space for oxygen to insert for catalysis to occur. Hydrogen bond interactions between DMO and dicamba are listed in Table 17 below.

Dicamba binds in a pocket directly below the Ile232 side chain, and this pocket is further bounded above by Asn218, I1e220, Ser247, and Asn230. On the sides this pocket is bounded by Leu158, Asp172, Leu282, Gly249, Ser267, and His251. Below dicamba, this cavity is bordered by Ala289, the non-heme iron, the residues which chelate it (His160, His165, Asp294) and Asn154, A1a169, A1a287, Tyr263, and Leu155.

DCSA, or 3,6-dichlorosalicylic acid, binds to DMO in a nearly identical manner as does dicamba, and the DMO-DCSA interaction in molecule A of the structure can be viewed in FIG. 23. Hydrogen bond interactions between DMO and DCSA, as observed in molecules "a" and "c" of the structure, are listed in Table 18 below.

The "Crystal 11" and "Crystal 12" structures (Tables 25-26) indicate that dicamba and DCSA bind above W285, which is opposite I232 in the active site cavity. These two residues provide key hydrophobic contacts to the dicamba/ DCSA ring. Residues H251, N230, Q286, and W285 provide key polar interactions and in almost all cases H251, W285 and N230 engage in hydrogen bonds with substrate or product and provide stabilizing polar interactions to the carboylate moiety of dicamba/DCSA. In a number of the structures specific hydrogen bonds are predicted/observed for N230, H251 and W285: (a) Q286 NE2-DCSA or dicamba O1 (possible H bond depending on rotamer); (b) W285 NE1-DCSA or dicamba O1; (c)H251 NE2 to O1; and (d.) N230 nd2 to 02 are in the carboxylate moiety of dicamba and DCSA. The non-heme Co⁺² ion binds a water molecule or an oxygen (O₂) molecule in the 2.05Å resolution DMO-Co-DCSA and DMO-Co-dicamba structures. Since Co²⁺ binds to the non-heme iron site in DMO, the above observations are also valid for the DMO-Fe²⁺-dicamba and the DMO-Fe²⁺-DCSA structures.

### F. Interaction Domain

Interaction domains in proteins, those that provide a surface for other entities (e.g. proteins or polynucleotides) to dock in certain spaces, are conserved domains that contain functionally similar residues in many cases. In addition, this conservation is usually coincident with the chemical functionality and properties of the residues. For instance, a common substitution comprises leucine for isoleucine and vice versa. Similarly, phenylalanine and tyrosine may substitute for one another in that they have much of the same aromatic character and nearly the same steric volume while tyrosine provides the added possibility of a polar interaction with its hydroxyl group. In this context, conservative amino acid substitution is defined as the replacement of an amino acid with another amino acid of similar physico-chemical properties, e.g. chemical structure, charge, polarity, hydrophobicity, surface, volume, presence of aromatic ring, hydrogen bonding potential. There are several classifications of the amino acids that can be found in the literature, e.g. Taylor, 1986, Livingstone *et. al.,* 1993; Mocz, 1995 and Stanfel, 1996. Hierarchical classification of the twenty natural amino acids has also been described (May, 1999). Example descriptions of the surfaces of amino acids can be found in Chothia, (1975). Example descriptions of amino acid volumes can be found in Zamyatin (1972), and hydrophobicity descriptions in Karplus (1997). While one may infer conserved sequence homology among a group of proteins and even identify the conserved secondary motifs, in the absence of structural data it is not easy to identify the specific function of these residues and domains and describe them in detail. The functional nature of these residues is more subtle than that of the more commonly identified amino acid motifs, for example those that are likely to participate in binding the Rieske center in the protein. Thus below are described a handful of key residues which are largely responsible for the subunit to subunit interactions that bring together the Rieske domain of one monomer with the non-heme iron domain of another monomer.

The trimer structure of the DMO crystal reveals that the interface between subunits is at the Rieske center of one subunit to the non-heme iron of another. The residues that make up this region are important for maintaining surface contact for oligomeric structure, productive electron transport and ultimately for catalysis. FIG. 7A-B show this interface region(white arrows). Some of the key residues discussed below are those involved in inter-subunit interactions specifically described at this interface. Example 4 highlights some of these key residues to define the interface region in more detail.

### G. C-terminal helix region

The C-terminal helix of DMO is defined by hydrophobic residues around positions 323-340, AAVRVSREIEKLEQLEAA (SEQ ID NO:24). Mutation of many of these residues results in loss of enzymatic activity, and this region apparently interacts with helper proteins such as ferredoxin.

### Example 2

### Crystallization of DMO

A protein sample of DMO (hereafter referred to as DMO or DMO_{w}; SEQ ID NO:3) was prepared to 10-20mg/mL in 30mM Tris-pH 8.0 buffer, 10mM NaCl, 0.1mM EDTA, and trace amount of PMSF. Protein crystals suitable for X-ray diffraction studies were obtained using the vapor diffusion crystallization method (McPherson, 1982). Deep red, diamond-shaped crystals were obtained using a precipitant solution of 17% PEG 6000 and 100mM sodium citrate-pH 6.0 in the reservoir, and suspending over this reservoir a droplet that contained equal volumes of the protein solution and the reservoir solution.

### Example 3

### Initial Diffraction Analysis of DMO Crystals

An initial 2.8Å X-ray diffraction data set was obtained on a cryo-cooled DMO crystal using a Rigaku protein crystallography data collection system (Rigaku Americas Corporation, The Woodlands, Texas). The data were collected on an R-AXIS IV++ imaging plate detector, with Cu Kα X-rays produced by a MicroMax-007 X-ray generator operating at 40 kV and 20 mA; beam collimation was provided by HiRes² Confocal Optics using a beam path that was evacuated with He gas. Cryo-cooling to approximately -170 °C was provided by an X-stream 2000 system. Prior to data collection, the DMO crystal was transferred to a cryo-solution that was 22% PEG 6000, 0.1M citrate buffer-pH 6.0, 22% glycerol, 1 mM NaN3 prior, and then plunge-cooled in liquid nitrogen. This crystal was then transferred to the R-AXIS IV++ goniostat for data collection using cryo-cooled tongs.

The initial 2.8 Å DMO crystal data were processed using the HKL2000 package (Otwinowski & Minor, 1997). Extensive analyses revealed that the DMO crystal belongs to the trigonal crystal system, and is space group P3₁ or P3₂. A summary of the initial data collection statistics are listed in Table 11 under the header 'DMO Crystal 1'. Assuming three molecules of DMO per crystallographic asymmetric unit results in a Matthews parameter of 2.52 Å³/Da (Matthews, 1968) and a crystal solvent content of 51%, which are reasonable values and consistent with the high-quality diffraction displayed using the X-ray diffraction unit.

**Table 11. Data Collection Statistics on Initial Structure Determination DMO Crystals**

| Data Collection Statistics | DMO Crystal 1 | DMO Crystal 2 | DMO Crystal 3 |
|---|---|---|---|
| Wavelength (Å) | 1.5418 | 2.29 | 1.5418 |
| Space group | P3₁ or P3₂ | P3₂ | P3₂ |
| Cell dimensions | | | |
| *a, b, c* (Å) | 79.80, 79.80, 159.46 | 79.55,79.55, 159.43 | 80.17,80.17, 159.35 |
| α, β, γ (°) | 90.0, 90.0, 120.0 | 90.0, 90.0, 120.0 | 90.0, 90.0, 120.0 |
| Resolution (Å) | 50-2.8 (2.9-2.8)* | 50-3.2 (3.31-3.2)* | 50-3.0 (3.11-3.0)* |
| *R*_{sym} or *R*_{merge} | 0.094 (0.374) | 0.072 (0.243) | 0.075 (0.304) |
| *I*/σ*I* | 10.1(1.1) | 26.2(7.9) | 25.8(6.7) |
| Completeness (%) | 95.5(96.7) | 98.7 (100) | 99.6 (100) |
| Redundancy | 2.7(2.5) | 7.2 (7.0) | 8.1(7.5) |

| | | | |
|---|---|---|---|
| *Highest resolution shell is shown in parenthesis. | | | |

### Example 4

### Crystallographic Structure Solution Work on DMO

### A. Efforts to Solve the Structure by Molecular Replacement Phasing Methods.

DMO has been classified as a member of the Rieske non-heme iron family of oxygenases (Chakraborty *et al.,* 2005). Sequence comparison analysis of the DMO sequence with sequences of known protein structures from the Protein Data Bank (PDB; www.rcsb.org) revealed some similarity in the N-terminal Rieske domain portion of DMO with the following Rieske-containing dioxygenases, which are listed in order of decreasing similarity: naphthalene 1,2-dioxygenase from *Rhodoccocus sp.* (PDB entry 2B24; SEQ ID NO:20; Gakhar *et al.,* 2005); nitrobenzene dioxygenase from *Comamonas sp.* (PDB entry 2BMO; SEQ ID NO:17; Friemann *et al.,* 2005); biphenyl dioxygenase from *Rhodoccocus sp.* (PDB entry IULI; SEQ ID NO:21; Furusawa *et al.,* 2004); and naphthalene 1,2-dioxygenase from *Pseudomonas sp.* (PDB entry 1EG9; SEQ ID NO:22; Kauppi *et al.,* 1998; Carredano *et al.,* 2000). Using this information, molecular replacement (MR) phasing calculations were conducted using DMO X-ray data and all or portions, most notably the Rieske domain portions, of each of the PDB entries 2B24, 2BMO, IULI and 1EG9 as phasing models. This MR work was performed using the Phaser program (McCoy *et al.,* 2005), and, to a lesser extent, the AMoRe program (Navaza, 1994). No promising MR solutions were obtained.

### B. Solving the Structure Using Single Anomalous Dispersion Phasing Methods and High-Redundancy Cu (λ=1.5418 Å) and Cr (λ=2.29 Å) Anode X-ray Data Sets.

As the DMO crystal structure could not be solved using MR methods, phases for crystallographic structure solution had to be sought by other methods. Crystallographic phasing was pursued using the method of single wavelength anomalous dispersion (SAD), taking advantage of the anomalous scattering from the sulfur (S) and iron (Fe) atoms in DMO. Data from more than one wavelength was obtained, allowing for multiple wavelength anomalous dispersion (MAD) analysis (e.g. Hendrickson, 1991). Each DMO monomer contains 16 sulfur atoms (seven from Cys residues, seven from Met residues, and two from the Rieske Fe₂S₂ cluster), and, maximally, three iron atoms (two in the Rieske Fe₂S₂ cluster and one from the non-heme Fe site). Moreover, since each DMO crystallographic asymmetric unit contains three monomers, each asymmetric can contain up to 48 sulfurs and nine iron sites for phasing. Prior sulfur SAD phasing of 44 kDa glucose isomerase, which contains nine sulfurs, and 33 kDa xylanase, which contains five sulfurs (Ramagopal *et al.,* 2003), suggested that sulfur SAD phasing may be a plausible strategy for 39 kDa DMO, which contains 16 sulfurs. Moreover, the successful structure determination of a 129-residue Rieske iron-sulfur protein fragment using the anomalous signal from iron (Iwata *et al.,* 1996) indicated that the Fe atoms in the Fe₂S₂ cluster were useful for structure solution phasing.

A 3.2Å resolution, high-redundancy X-ray data set for sulfur SAD phasing was collected from a DMO crystal, prepared as previously described, at the Rigaku Americas Corporation headquarters (The Woodlands, TX). The X-ray data collection system that was employed used a MicroMax-007HF X-ray generator, Cr Varimax HR collimating optics, an R-AXIS-IV++ detector, an X-stream 2000 low temperature device, and a chromium (Cr) anode, which produces a 2.29Å wavelength X-ray. The data collection statistics for this data set are listed in Table 11 under the header "DMO Crystal 2". In addition, a 3.0Å resolution, high-redundancy, X-ray data set for Fe SAD phasing was also collected. The X-ray data collection system used to obtain these data contained a MicroMax-007HF X-ray generator, Cu Varimax HR collimating optics, a Saturn 944 CCD detector, an X-stream 2000 low temperature device, and a copper (Cu) anode, which produces a 1.5418 Å wavelength X-ray. The data collection statistics for these data are listed in Table 11 under the header "DMO Crystal 3".

Sulfur SAD phasing calculations using 20-3.2Å DMO Crystal 2 data were conducted with the program SOLVE (Terwilliger & Berendzen, 1999), and subsequent density modification calculations were conducted with its partner program RESOLVE (Terwilliger, 1999). Calculations were performed in both space group P3₁ and P3₂. No encouraging phasing solutions and density maps resulted from this work, indicating that the crystal structure of DMO is distinct from that of other known Rieske non-heme iron oxygenase family members in this regard.

Iron SAD phasing calculations were next conducted using the 20-3 Å DMO Crystal 3 data and the SOLVE and RESOLVE programs, and in both space group P3₁ and P3₂. SOLVE calculations in both space groups produced three 'Fe' sites, but only the electron density map resulting from the P3₂ work had the features of a promising protein structure map, with clear protein-solvent boundaries and peptide paths. This map evaluation work, and all subsequent crystallographic map work, was done with program O (Jones *et al.,* 1991), using a linux workstation with stereo-graphics capabilities.

Further evaluation of this 20-3 Å P3₂ map from Fe SAD phasing revealed several noteworthy features. Each 'Fe' site clearly represented an individual Fe₂S₂ cluster in the structure, and an equilateral triangle arrangement of these sites, with sides of ~50Å in length, was located in the map. Similar intermolecular Fe₂S₂ cluster arrangements have been observed in other known dioxygenase crystal structures, such as the naphthalene 1,2-dioxygenase structures from *Pseudomonas sp.* (PDB entries 1NDO and 1EG9; SEQ ID NO:22;) and from *Rhodoccocus sp.* (PDB entries 2B24 and 2B1X; SEQ ID NO:20). From this realization, and by using the Rieske cluster in 2B1X as a guide, the peptide stretches that covalently interact with the Rieske cluster, residues 42-58 and 68-82, as well as the Rieske cluster itself, denoted 501, were built into the density map. Once the three Rieske clusters in the DMO asymmetric unit were defined, 20-3 Å program SOLVE phasing was performed using six Fe atoms (instead of the initial three Fe atoms), followed by program RESOLVE density modification. The resulting Fourier map was significantly improved over the initial one, with better peptide path definition and side chain clarity. From this density map, and by concentrating on building DMO structure into just one DMO molecule in the asymmetric unit, a preliminary DMO structure containing 193 (residues 1-130; 146-155; 272-324) of the 349 total residues and the Fe₂S₂ cluster was built.

To improve the quality of the DMO density map for further map-fitting, the coordinates of the three Fe₂S₂ clusters were included in subsequent 20-3 Å resolution RESOLVE density modification work to allow non-crystallographic symmetry (NCS) averaging to be performed; from these Fe₂S₂ coordinates, the program could define the 3-fold NCS symmetry axis relating the individual DMO monomers in the DMO trimer, and use this axis for density averaging. Using NCS-averaging improved the Fourier map quality. In addition, calculating 20-3.2 Å difference anomalous Fourier maps using the Cr anode X-ray data and these phases yielded strong (>3.5σ) peaks at the location of well-defined sulfur atoms (found in Cys and Met residues, and in the Fe₂S₂ clusters) in the map. With these enhancements, further map fitting allowed 83% of the DMO structure to be built: residues 2-157; 196-234; 247-269; 278-343; and the 501-Fe. The Phaser MR program was then used to locate the remaining two DMO molecules in the asymmetric unit, and this was followed by 20-3 Å crystallographic refinement using the CNX program (Accelrys, Inc., San Diego, CA). The CNX program is based on the once widely used program X-PLOR (Brünger, 1992a). This refined DMO structure has an R_{work}=30.8% and an R_{free}=35.0% for 20-3 Å data. 90% of the X-ray data were used to calculate the R_{work} and 10% of the X-ray data were used to calculate the R_{free} (Brünger, 1992b). The complete refinement statistics are listed in Table 12 under 'DMO Crystal 3'. Ribbon-style renditions of the initial DMO trimer structure are displayed in FIG. 1.

**Table 12. Data collection and refinement statistics on DMO Crystals yielding solved structures**

| | | | |
|---|---|---|---|
| | DMO Crystal 3 | DMO Crystal 4 | DMO Crystal 5 |
| Wavelength (Å) | 1.5418 | 1.00 | 1.5418 |
| Space Group | P3₂ | P3₂ | P3₂ |
| Cell dimensions | | | |
| *a, b, c* (Å) | 80.17,80.17, 159.35 | 79.80,79.80, 159.40 | 80.06,80.06, 160.16 |
| α, β, γ (°) | 90.0, 90.0, 120.0 | 90.0, 90.0, 120.0 | 90.0, 90.0, 120.0 |
| Resolution (Å) | 50-3.0 (3.11-3.0)* | 50-3.2 (3.31-3.2)* | 50-2.65 (2.74-2.65)^{*} |
| *R*_{sym} or *R*_{merge} | 0.075 (0.304) | 0.099 (0.492) | 0.058 (0.299) |
| *I*/*σI* | 25.8 (6.7) | 13.6 (2.5) | 11.3 (1.1) |
| Completeness (%) | 99.6 (100) | 99.8 (99.8) | 93.5 (99.5) |
| Redundancy | 8.1 (7.5) | 4.1 (4.0) | 1.7(1.7) |
| | | | |
| **Refinement** | | | |
| Resolution (Å) | 20-3.0 | 20-3.2 | 20-2.65 |
| No. reflections | 21042 | 18689 | 32021 |
| *R*_{work} / *R*_{free} | 30.8% / 35.0% | 31.3% / 36.6% | 33.3% / 37.3% |
| No. atoms | | | |
| Protein | 6606 | 6642 | 7218 |
| Ligand/ion | 12 | 12 | 15 |
| Water | 0 | 0 | 0 |
| *B*-factors (Å²) | | | |
| Protein | 30.4 | 47.0 | 56.3 |
| Ligand/ion | 17.5 | 29.6 | 45.9 |
| Water | -- | -- | -- |
| R.m.s deviations | | | |
| Bond lengths (Å) | 0.009 | 0.009 | 0.008 |
| Bond angles (°) | 1.569 | 1.525 | 1.436 |

| | | | |
|---|---|---|---|
| *Highest resolution shell is shown in parenthesis. | | | |

### C. Pursuing a DMO Structure with the non-heme Iron Site Occupied and Dicamba or DCSA Bound.

When the structure of DMO Crystal 3 was solved, conspicuously absent from the structure was the iron (Fe) ion that must bind to the non-heme or catalytic iron site. In all known Rieske non-heme iron oxygenase (RO) structures to date, electron transfer in the RO α3 or α3β3 quaternary unit involves a flow of electrons from the Fe₂S₂ Rieske center in one subunit to the mononuclear iron, ~12 Å away, in a neighboring subunit (Ferraro *et al,* 2006). This iron site is chelated by two histidines and a single aspartic acid, and it is at this site that molecular oxygen and an aromatic substrate react to and produce the oxidized product (Ferraro *et al,* 2006). Although the invention is not bound by any particular mechanism for electron transport and catalysis, it is believed that electron transfer and catalysis involving dicamba likely occurs by a similar route in DMO, and it was decided to obtain a crystal structure with the non-heme iron site occupied.

As a first step toward getting the non-heme iron site occupied in the DMO crystals, crystallizations were performed as before, but with 5 mM Fe⁺² and 5 mM Fe⁺³ included in the droplets, as well as 5 mM of other isostructural divalent ions, like Mn⁺², Co⁺², Ni ⁺², Cu⁺² and Zn⁺². Crystals resulted only from droplets including Co⁺², Ni⁺² and Mn⁺². X-ray data were collected on a DMO crystal grown in 5 mM Ni⁺² and soaked in a cryo-solution, similar in composition to the one noted previously, but containing 5 mM Ni⁺² for 1.7 hours prior to cryo-cooling. The data collection summary and structure solution statistics for this crystal are listed in Table 12 under "DMO Crystal 4". The X-ray data from DMO Crystal 4 were collected employing an X-ray synchrotron (SER-CAT 22-BM beamline; Argonne National Laboratory, Argonne, IL) at a wavelength of 1.000 Å, using a Mar225 CCD detector (Mar USA, Evanston, IL).

The refined structure for DMO Crystal 4 revealed no evidence of a non-heme iron site being occupied within ~12 Å of the Fe₂S₂ clusters in the DMO trimer, as was the case in the refined structure of DMO Crystal 3 (FIG. 1). The DMO Crystal 4 active site structure at the molecule A-B interface is shown in FIG. 8. In light of what has been observed in other RO oxygenases (Ferraro *et al,* 2006) and in the DMO sequence, His160 and His165 are the likely histidine residues to chelate the non-heme iron atom. Molecule A in DMO Crystal 4 reveals ordered structure only up to Gln162, and no evidence of His160 interacting with a divalent ion. Additional X-ray data collection and evaluation of a DMO crystal grown in the presence of 5 mM Co⁺², and soaked in the presence of a cryo-solution containing 5 mM Co⁺² and further in a cryo-solution containing 10 mM Co⁺² and 25 mM dicamba for 4.25 hours yielded similar results: no evidence of Co⁺² in the non-heme iron site, no ordered electron density shortly beyond His 160, and no evidence of dicamba bound to the enzyme (FIG. 8).

The difficulty in obtaining a DMO crystal structure with the non-heme iron site occupied led to a review of all aspects of the DMO structure determination process, and to a review of the RO crystallization literature. This review suggested that use of citrate buffer in both the crystallizations and crystal cryo-solutions could be the root cause. Citric acid is known to bind a variety of divalent metal ions, including ions of magnesium, calcium, manganese, iron, cobalt, nickel, copper, and zinc (Dawson *et al.,* 1986). Additionally, other RO oxygenases that crystallize in the pH 5.5-6.5 range and have solved structures with the non-heme iron site occupied, such as naphthalene 1,2-dioxygenase from *Pseudomonas sp.* (Kauppi *et al.,* 1998; PDB entry 1NDO; SEQ ID NO:22) and nitrobenzene dioxygenase (PDB entry 2BMO; SEQ ID NO:17), were crystallized using the MES or HEPES buffers, which have low to negligible metal ion binding properties (Good & Izawa, 1968).

Growth of DMO crystals using either MES or acetate buffers in the crystallization, initially yielded crystals of lesser size and visual quality than those grown using citrate buffer. However a crystal structure of DMO with the non-heme iron site occupied was pursued by first equilibrating DMO crystals in a cryo-solution containing MES buffer and then equilibrating the DMO crystals in a cryo-solution containing MES and Fe⁺². DMO crystals, grown by means already described, were soaked in a cryo-solution of 23% PEG 4K, 23% glycerol, and 0.1M MES-pH 6.0 buffer for ~16 hours and were then transferred to a cryo-solution that was 20.7% PEG 4K, 20.7% glycerol, 0.09 M MES-pH 6.0, and ~10 mM FeSO₄ for ~9 hours. X-ray data were collected on an Fe⁺²-soaked DMO crystal using the Cu-anode X-ray data collection system at a wavelength of 1.5418 Å, and in a manner previously described in this example. These data were processed and the structure solved by previously noted methods. The data collection and refinement stats for this crystal are listed in Table 12 under "DMO Crystal 5", and the active site region of this crystal structure is displayed in FIG. 2.

Evaluation of the 'DMO Crystal 5' X-ray data indicated success in achieving Fe⁺² - binding at the non-heme iron site of the crystal. As has been noted previously, iron has a significant anomalous scattering signal at the 1.5418 Å wavelength used in the data collection, and anomalous difference Fourier maps calculated using these data revealed a strong (>3.5σ) peak at the Fe⁺² site position in all three molecules in the crystallographic asymmetric unit. This is strong evidence of non-heme iron binding. The structural results in 'DMO Crystal 4' and 'DMO Crystal 5' indicate that in the absence of an ion to fill the non-heme iron site, the peptide from residues 157-162 adopts an extended conformation (FIG. 8) and disorders beyond this point, while in the presence of a suitable ion to occupy non-heme site, a helical loop structure results, where His160 and His165 are oriented to chelate the non-heme iron site ion (FIG. 2), with Asp294 completing the chelation of the iron site. In addition, since the side chain N atom of Asn154 ranges from 2.7-3.3 Å to the 502-Fe non-heme iron site and averages 3.1 Å in length in all three molecules of the asymmetric unit, which is slightly longer when compared to the average chelation distances involving H160 (2.4 Å), H165 (2.8 Å), and D294 (2.4 Å), this suggests that N154 may play an ancillary role in this iron site chelation.

### D. Crystal structure of DMO with substrate or product.

A 2.7Å resolution crystal structure of DMO was next obtained, with the non-heme iron and substrate binding sites occupied in two molecules of the crystallographic asymmetric unit. The dicamba and Fe⁺² ions were introduced into pre-formed protein crystals by soaking. The crystals were growing by previously noted methods using 16-20(w/v)% PEG 6,000 and 0.1 M sodium acetate buffer, pH 6.0, as the precipitating agent. Crystals were then transferred to a stabilization solution that contained 20.4(w/v)% PEG 6,000, 20.4% glycerol, 0.09 M HEPES-pH 7 buffer, 1.25 mM dicamba, and ~10mM FeSO₄. The crystal used for X-ray data collection was stored in this solution for 30 hours (1.25 days) prior to cryo-cooling. Data collection and structure solution statistics for this crystal are listed in Table 13 below under "DMO Crystal 6". Atomic coordinates are given in Table 4. The structure solution statistics for the crystal bound to DCSA are listed in Table 13 below under "DMO Crystal 7". Atomic coordinates are given in Table 5. Both data sets for DMO Crystal 6 and DMO Crystal 7 were collected on the SER-CAT 22-ID beamline at the Advanced Photon Source synchrotron, (Argonne National Laboratory, Argonne, IL). The X-ray wavelength employed was 1.000 Å, and these data were collected using a Mar300 CCD detector (Mar USA, Evanston, IL).

**Table 13. Data collection and refinement statistics on DMO Crystal 6 and DMO Crystal 7.**

| | DMO Crystal 6 | DMO Crystal 7 |
|---|---|---|
| Wavelength (Å) | 1.000 | 1.000 |
| Space Group | P3₂ | P3₂ |
| Cell dimensions | | |
| *a, b, c* (Å) | 80.56,80.56,159.16 | 80.78,80.78,159.22 |
| α, β, γ (°) | 90.0, 90.0, 120.0 | 90.0, 90.0, 120.0 |
| Resolution (Å) | 50-2.7 (2.8 - 2.7)* | 50-2.8 (2.8 - 2.9)* |
| *R*_{sym} or *R*_{merge} | 0.061 (0.485) | 0.086 (0.459) |
| *I*/σ*I* | 24.2(1.7) | 16.8(1.9) |
| Completeness (%) | 99.4 (100) | 99.7 (100) |
| Redundancy | 3.8 (3.8) | 3.4 (3.4) |
| | | |
| **Refinement** | | |
| Resolution (A) | 20 - 2.7 | 20 - 2.8 |
| No. reflections | 25794 | 23395 |
| *R*_{work} / *R*_{free} | 31.6% / 35.7% | 30.5% / 34.2% |

| No. atoms | | |
|---|---|---|
| Protein | 7084 | 7084 |
| Ligand/ion | 40 | 38 |
| Water | 0 | 0 |
| *B*-factors (Å²) | | |
| Protein | 65.7 | 59.0 |
| Ligand/ion | 64.4 | 62.9 |
| Water | -- | -- |
| R.m.s deviations | | |
| Bond lengths (Å) | 0.009 | 0.009 |
| Bond angles (°) | 1.452 | 1.532 |

| | | |
|---|---|---|
| *Highest resolution shell is shown in parenthesis. | | |

The DMO-dicamba crystal structure ("DMO Crystal 6" crystal structure), was solved by the molecular replacement (MR) method using all the crystal Fobs X-ray data from 35.9-2.70 Å resolution with |Fobs|/σ|Fobs| > 2.0, the coordinates for DMO molecule "a" from the DMO Crystal 5 coordinates as the search model, and the Phaser program to perform the MR phasing. Evaluation of the initial 2Fo-Fc density maps revealed that molecule "a" of the trimer contained no evidence for non-heme iron binding and that the peptide stretch from residues a159-a175 was disordered. However, this map also revealed evidence of non-heme iron binding and indications of dicamba binding in molecules "b" and "c". As a consequence of this observation, the first modeled DMO segment of molecule "a" was trimmed from a2-a175 to a2-a158. After two additional rounds of map-fitting and refinement, a refined structure was obtained which revealed clear, well-defined electron density for dicamba in DMO molecules "b" and "c" of the asymmetric unit. FIG. 18 displays how dicamba binds in molecule "c" of the crystallographic asymmetric unit. The structure reveals clear evidence of the non-heme iron site ("502", orange sphere) being occupied, of the residues which chelate the site (H160, H165 and D294), neighboring residue N154, and of dicamba ("DIC 601") binding. In FIG. 18 the Cl atoms in dicamba are colored purple, and the hydrogen bonds between dicamba and N230 and H251 are rendered as dotted lines. Dicamba binds under I232, and the methoxy carbon, which is lost in the conversion of dicamba to DCSA, is directed toward the non-heme 502 iron ion. Interestingly, the presence of free iron has been found to stimulate enzymatic activity.

It was also possible to obtain a 2.8Å resolution crystal structure of DMO with clear electron density for the non-heme iron site occupied and DCSA bound in two molecules of the crystallographic asymmetric unit. DCSA is an acronym for 3,6-dichlorosalicylic acid, and DCSA is the product resulting when DMO dealkylates dicamba. The DCSA and Fe⁺² ions were introduced into pre-formed protein crystals by soaking. The crystals were growing by previously noted methods using 16-20(w/v)% PEG 6,000 and 0.1 M sodium acetate buffer-pH 6.0 as the precipitating agent. The crystals were then transferred to a stabilization solution that contained 20.4(w/v)% PEG 6,000, 20.4% glycerol, 0.09 M HEPES-pH 7 buffer, 1.25 mM DCSA, and ~10mM FeSO4. The crystal used for X-ray data collection was stored in this solution for 30 hours (1.25 days) prior to cryo-cooling. The data collection and structure solution statistics for this crystal are listed in Table 13 above, under "DMO Crystal 7".

The DMO-DCSA "DMO Crystal 7" structure was solved by the MR method using the crystal Fobs X-ray data file, the coordinates for DMO molecule "a" from the DMO Crystal 6 coordinates, and the Phaser program. The Phaser search model used DCSA rather than dicamba, and this was prepared by removing the methoxy carbon from the dicamba coordinates. Evaluation of the initial 2Fo-Fc density maps revealed that molecule 'b' contained no evidence for non-heme iron binding and that the peptide stretch from residues a159- a175 was disordered. However, this map also revealed evidence of non-heme iron binding and indications of DCSA binding in molecules 'a' and 'c'. The density for DCSA binding was strongest in molecule 'a', and is only partially complete in molecule c. As a consequence of this observation, in the second round of refinement DMO molecule b contained only residues b2-b158 for the first protein segment, and no longer included the non-heme iron ion or DCSA. After one additional round of refinement, the 'DMO Crystal 7' structure resulted. Figure 23 displays how DCSA binds in molecule 'a' of the crystallographic asymmetric unit.

### Example 5

### Interaction Domain Modeling

ICM pro from MolSoft (ver3.4-7h; Molsoft, LLC; La Jolla, CA) was used to probe the interaction domains in the DMO Crystal 5 structure. Default settings were used for the identification of the contact regions and the results were examined by hand to identify those residues on the protein subunit interface.

While a DMO monomer alone will likely perform a single turnover, for full catalysis interaction with other subunits is necessary. In addition helper proteins are required such as an electron donor to the Rieske center (e.g. ferredoxin) and a reductase to shuttle electrons from NADH or NADPH.. The interface (i.e. "interaction domain") between subunits is described by and includes these 52 amino acids numbered from the N-terminus of a monomer: V325, E322, D321, C320, L318, M317, A316, P315, R314, I313, V308, Y307, R304, I301, A300, V297, V296, V164, Y163, H160, G159, D157, N154, D153, R98, L95, S94, G89, G87, H86, P85, N84, L73, G72, H71, Y70, P69, C68, Q67, D58, P55, A54, F53, R52, H51, P50,148, D47, L46, P31, T30, and D29. All of these cross subunit contacts are described below with the most significant of these contacts used as the anchors for this discussion.

Key interface residues include H51a:R52:F53a:Y70a:H71a:H86a:H160c: Y163c:R304c:Y307c:A316c:L318c. These residues account for over 85% of the interface contacts (i.e. non- redundant contacts, possibly even more of total contacts since some interact with the same residues). These identified residues are thought to make up an interaction domain motif. The functional description of these residues as specific to interactions between the subunits has not previously been described. Conserved residues in the alignments (FIG. 9) define another super family motif as has been described elsewhere in some cases, although such motifs and functions were not previously described for DMO.

Some of the key interactions mediated by the residues of the interaction domain include: those which involve residue H51, which is involved in the Rieske cluster (2.2Å to Fe cluster, subunit a) and participates in a bifurcated hydrogen bond with the E322 side chain in an adjoining subunit (subunit c). H-bond (defined from donor to acceptor) interactions are between H51 NE2 and E322 OE1(2.5Å) and OE2(2.7Å) respectively. H51 also has Van der Waals contacts (≤ 3.8Å) with P315, A316, and L318. This is a key residue for Rieske center binding and electron transport and participates in the interface region.

The R52 side chain is adjacent to H51, a member of the Rieske cluster. The main chain NH appears to have a hydrogen bond (3.2Å) with the Rieske center sulfur. All of the N atoms of the R52 side chain are with in 3.4 to 3.8Å of E322 side chain oxygen atom OE1 and up to 4.6 Å away from OE2. The side chain has various contacts of less than 4.3Å with adjacent subunit residues D153, P315, M317, and V325.

The F53 side chain of subunit a inserts into a hydrophobic cavity defined by the following subunit a residues: P315, R314, I313, V308, Y307, I301, and the main chain of R304. These hydrophobic contacts range from 3.8 to 4.3Å. This is a significant grouping of hydrophobic contacts which is likely a key anchor to this portion of the interface. This residue is not conserved in other oxygenases of this type which typically utilize much smaller residues in this position (G, L). Its proximity to H51 (<4.0Å in same subunit) and the non-heme iron suggest this could play a role in excluding solvent from this side of the non-heme iron site.

Y70 is less than 6.0 Å away from the non-heme iron of the adjoining subunit and less than 4.0 Å from the Rieske center. The side chain is involved in Van der Waals contacts/hydrophobic interactions of 4.0Å or less with the in subunit c side chains H160, I301, V297, V164 and the main chain of Y163. The Y70 (OH) has a polar contact with N154 OD1 (or ND1) (2.5Å) from subunit c. N 154 ND1 is only 3.3Å away from the non-heme iron molecule and provides a possible ligand interaction. V297 CG1 side has Van der Waals contacts with A54a CB (3.8Å). A54a engages in Van der Waals contacts with I301 (4.3Å). These residues form a series of hydrophobic contacts along an interface helix.

The H71 side chain NE2 in subunit "a" engages in a hydrogen bond with the OD1 of D157 (2.8Å), in subunit "c", which is in turn hydrogen bonded to H160 ND1 through D157 OD1 (2.8Å). This is an important interaction analogous to that in NDO (Kauppi, 1998; FIG. 8). D157 is analogous to D205 of NDO and HB71 is analogous to H104 of NDO. This residue are also involved in longer Van der Waals contacts of 4.1-4.8Å through H71 ring atoms and with D321. In addition this H71 is nearly stacked (3.5-3.7Å) with the neighboring Y70 residue forming an interesting interaction pocket with H160 right between the iron centers.

H86 is 6.1Å from the Rieske center and its ND1 and CE1 atoms have Van der Waals contacts of 3.8-4.4Å with the underside of the side chain of D321 of the adjoining subunit. H86 side chain atoms also have cross subunit Van der Waals/hydrophobic interactions of less than 4.3Å with G159, Y163, C 320 and L318. Few other amino acid residues would fit in place of G159 in this structure.

The H160 residue is important to binding the non-heme iron. It is also on the interface between the subunits and its side chain methylene group is flanked by Y70 and H71 of the neighboring subunit and V297 of the same subunit. These three residues are highly conserved among all oxygenases and this pocket of Y70a, H71a and H160c is likely critical to the function of this enzyme.

Y163 is highly a solvent exposed residue in subunit "c". It forms contacts with subunit a through hydrophobic and Van der Waals interactions (4Å or less) with 8 residues: Q67, C68, P69, Y70 (main chain), G72, H71 (main chain), P85, and H86; most from the same face of the aromatic residue side chain. It appears to shield H71 and possibly C68 (Rieske center cysteine) from solvent although it interacts with the main chain of this residue. Interestingly it is juxtaposed with the Y70 residue in subunit a and both are likely key interactions for keeping the surfaces together and shielding the non-heme iron center. Y163 appears to have conserved function i.e. hydrophobic aromatic while Y70 appears to be universally conserved in the most closely (although with a limited over all homology, i.e. about 37%) related oxygenases. This is a significant residue that may be part of a motif. Structural information thus provides insight into its function (e.g. as a key interaction residue and for solvent exclusion from the Rieske site).

The subunit "c" R304 side chain and the subunit "a" D47 side chain engage in an polar interaction (salt bridge or hydrogen bond), the distances between adjacent side chain atoms ranges from 3.0 to 3.7Å. The NH1 N of R304 appears to be in position to form a hydrogen bond with the carbonyl O of D47 of 3.0 Å in length.). The 148 side chain has multiple contacts (4.0 and 5.0Å) with the R304 side chain. P31, P55 and A 54 side chains and residues have van der Waals contacts with R304 (3.2-4.7Å). R304 is part of the F53 cluster of residues.

The subunit "c" Y307 residue is partially solvent exposed and has numerous van der Waals contacts with subunit a residues (4.3Å or less) in length: D29, T30 (CG2 side chain), P31 (CD ring carbon), L46,148, and R98 are among them. L46 and 148 (3.2Å) appear to be the most significant of these hydrophobic interactions. It may also participate in a polar interaction with the side chain of R98 (NH1) which is only 3.9Å away from the Y307 (OH).

The subunit "c" A316 (involved in the H51 set of residues described above) side chain lies along a hydrophobic surface coil near the subunit a Rieske cluster. The CB has hydrophobic contacts with the L95 side chain CD2 (3.7Å), the carbonyl O of S94 (3.6Å), and the carbonyl O of P50 (3.2Å). Also, A316 N engages in a hydrogen bond with P50 (2.8Å). It also has a Van der Waals interaction of 4.3A with the F53 ring.

The L318 (subunit "c") side chain inserts into a shallow cavity in subunit a, defined by the side chains of H51, H71, L73, N84, H86, and L95. All of these hydrophobic contacts range from 3.5 to 4.3Å in distance. This cluster of 6 contact residues is likely very important to the protein-protein interaction of the subunits. It also appears that the L318 hydrophobic cluster shields the Rieske center from solvent. A neighboring residue, C320, has a polar contact across the subunits (3.4Å with N84) to the outside of this cluster at the end of the C terminal helix.

Table 14 below shows the interaction residues described above along with the contact area and exposed area. "Contact area" is the area of the residue that is in contact with another residue or metal ion. The "Exposed area" is the area of the residue that is exposed to solvent and the contact area is the area of the residue that is in contact with another residue or metal ion. The stated "percent" is the ratio of contact/exposed x 100. These contacts were determined using the ICM Pro program with the algorithm developed from Abagyan and Totrov (1997). The bolded portion of the table corresponds to the most variable region among the alignments of FIG. 9 and FIG. 17. Alignments were performed by use of the ZEGA (zero-end gap alignment) pairwise alignment algorithm (Abagyan and Batalov, 1997) of Molsoft ICM-Pro (MolSoft LLC, LaJolla, CA), under default parameters, or by the MUSCLE algorithm (Edgar, 2004), using default parameters. The VanO and TolO columns refer to the corresponding residue in those proteins as compared to that in DdmC by structure based alignments as shown in FIG. 9 and FIG.17. Alignments with additional oxygenases may similarly be performed. It is interesting that the contact residues are, in many cases, conserved when compared to the other most closely related (at primary sequence level) monooxygenases, for example TolO (Toluene sulfonate monooxygenase) and VanO (Vanillate O-demethylase) as compared in Table 14. Such VanO and TolO polypeptides include those from other bacteria (Leahy *et al.,* 2003; Morawski *et al.,* 2000; Buswell *et al.,* 1988; Junker *et al.,* 1997; Shimoni *et al.,* 2003). Numbering is based on the structure file residue numbers e.g Crystal 5.

**Table 14: Interface Region contact residues as compiled by ICM pro alignment using VanO and TolO sequences for comparison.. The bolded portion of the table corresponds to the most variable region among the alignments.**

| Residue | # | Contact Area | Exposed Area | Percent ((contact/exposed)X100) | VanO | TolO |
|---|---|---|---|---|---|---|
| V | 325 | 12.9 | 34.0 | 38 | V | N,M |
| E | 322 | 24.5 | 65.6 | 37 | A | A |
| D | 321 | 17.0 | 75.7 | 22 | D | D |
| C | 320 | 44.5 | 96.6 | 46 | I | A |
| L | 318 | 78.1 | 106.2 | 74 | L | I |
| **M** | **317** | **14.3** | **104.9** | **14** | **x** | **x** |
| **A** | **316** | **65.9** | **92.2** | **71** | **x** | **x** |
| **P** | **315** | **30.7** | **97.4** | **31** | **K** | **P** |
| **R** | **314** | **5.6** | **182.1** | **3** | **L** | **V** |
| **I** | **313** | **21.9** | **101.3** | **22** | **L** | **M** |
| **V** | **308** | **33.9** | **59.7** | **57** | **x** | **V** |
| **Y** | **307** | **71.6** | **135.1** | **53** | **x** | **x** |
| **R** | **304** | **72.6** | **146.7** | **49** | **Q(N)** | **Q(I)** |
| **I** | **301** | **61.5** | **88.1** | **70** | **Q** | **Q** |
| **A** | **300** | **17.9** | **38.1** | **47** | **R** | **A** |
| **V** | **297** | **32.4** | **73.2** | **44** | **M,I** | **M,I** |
| V | 296 | 3.8 | 83.3 | 5 | D,E | T |
| V | 164 | 16.0 | 44.9 | 36 | V | V |
| Y | 163 | 65.3 | 170.3 | 38 | Y | W |
| H | 160 | 32.7 | 56.1 | 58 | H | H |
| G | 159 | 24.5 | 52.6 | 47 | T | T |
| D | 157 | 10.8 | 50.4 | 22 | D | D |
| N | 154 | 20.8 | 50.6 | 41 | N | N |
| D | 153 | 10.4 | 61.6 | 17 | D | D |
| R | 98 | 2.6 | 124.3 | 2 | Q | R |
| L | 95 | 26.4 | 50.9 | 52 | P | X |
| S | 94 | 11.8 | 82.0 | 14 | F | X |
| G | 89 | 1.0 | 31.0 | 3 | Q | E |
| G | 87 | 14.9 | 58.9 | 25 | x | x |
| H | 86 | 66.6 | 107.4 | 62 | x | x |
| P | 85 | 12.2 | 71.2 | 17 | P | P |
| N | 84 | 16.1 | 33.3 | 48 | M | I |
| L | 73 | 24.6 | 38.0 | 65 | L | L |
| G | 72 | 14.4 | 21.0 | 69 | G | G |
| H | 71 | 73.8 | 99.1 | 75 | H | H |
| Y | 70 | 99.9 | 132.4 | 75 | Y | Y |
| P | 69 | 19.6 | 74.5 | 26 | G | X |
| C | 68 | 7.7 | 17.1 | 45 | C | C |
| Q | 67 | 13.5 | 55.9 | 24 | V | R |
| D | 58 | 3.8 | 88.2 | 4 | L | I |
| P | 55 | 44.3 | 69.3 | 64 | P | P |
| A | 54 | 23.0 | 32.3 | 71 | A | A |
| F | 53 | 84.7 | 148.5 | 57 | G | L, S |
| R | 52 | 70.4 | 143.5 | 49 | R | R |
| H | 51 | 83.3 | 104.5 | 80 | H | H |
| P | 50 | 19.0 | 57.4 | 33 | P | C |
| I | 48 | 49.4 | 80.4 | 61 | F(A) | R(A) |
| D | 47 | 17.9 | 29.4 | 61 | D | D |
| L | 46 | 19.5 | 56.8 | 34 | E | E |
| P | 31 | 16.4 | 56.1 | 29 | x | P |
| T | 30 | 12.5 | 30.7 | 41 | E | E |

Five additional residues are involved in subunit interactions: A300, V296, G89, G87, and D58. The A300c CB has van der Waals contacts of 3.4-4.1Å with P55 ring carbon atoms. P55 is near the Rieske center and is part of the R304 interactions. The C320c SG interacts with a neighboring molecule near the subunit a Rieske cluster. It has van der Waals contacts of less than 4.3 Å with N84 side chain ND2, and the carbonyl oxygens of G89 and G87. The side chain nitrogen ND2 of N84 has van der Waals contacts of 3.9Å with the C320c SG. These interactions were described in part in the description of the L318 cluster (see above). The V296c CG1 has long van der Waals contacts with the D58 and P55 on the order of 5Å or so.

A number of residues are conserved in these sequences as described above and their conservation allows the definition of an interface structure, domain or motif. Numbering is based on the structure file residue numbers.

### Example 6

### Modeling of Active Site and Dicamba Docking

### A. Active site modeling using structure with a bound non-heme iron

DMO Crystal 5 structure coordinates were analyzed to define the structure of the active site and the interactions of DMO with dicamba. The pdb file was loaded into Molsoft ICM-Pro, version 3.4 (MolSoft LLC, La Jolla, CA), and converted to a Molsoft object. Hydrogen atoms were added and optimized, and the resulting structure was defined as a docking receptor in Molsoft, with default parameters, and used to identify potential binding pockets. The largest pocket (volume 443Å³; area 479Å²) is in the vicinity of the non-heme iron ion. This is thought to be the dicamba binding pocket as required by the chemical constraints for dicamba demethylation. The pocket is formed by residues L155, D157, L158, H160, A161, H165, R166, A169, Q170, D172, A173, A216, W217, N218, 1220, N230, 1232, A233, V234, S247, G249, H251, S267, L282, W285, Q286, A287, Q288, A289, V291 (as shown in FIG. 10 or FIG. 24).

A receptor map was calculated using default Molsoft parameters and dicamba docking was performed. The five lowest energy conformations of docked dicamba are shown in FIG. 11 A-E) and their corresponding docking energies can be found in Table 15.

**Table 15: Energies of docked dicamba and Cα-non heme iron distances.**

| **Conformation** | **Energy** | **Cα-Fe Distance** |
|---|---|---|
| | **kcal/mol** | **Å** |
| 1 | -30.9 | 5.4 |
| 2 | -28.8 | 4.0 |
| 3 | -28.1 | 3.7 |
| 4 | -28.1 | 3.8 |
| 5 | -28.0 | 3.7 |

The dicamba binding pocket of DMO (DdmC) was identified using Molsoft ICM-Pro (version 3.4) and the residues forming the pocket were mapped onto the primary sequence (e.g. FIG. 9, FIG. 21). Despite the fact that DdmC is a trimer, all the residues of the pocket are from the same subunit. The dicamba molecule (FIG. 12) was docked into this binding pocket and several different conformations were identified, and their energies and Cα-non-heme iron were calculated. It is noteworthy that despite these conformations being significantly different, they exhibit very similar Cα-non-heme iron distances. Moreover, some of the dicamba conformations show significant carboxy group interaction with the non-heme iron.

### B. Active site modeling using structure with a bound non-heme iron and bound dicamba in the crystal (Crystal 6)

In order to obtain a structure of the DMO bound to dicamba, a molecular structure with dicamba present in subunits b and c of the DMO trimer was constructed. As above, a data file in pdb (Protein database) format with atomic coordinates for DMO with bound dicamba (e. g. data of Table 4) was loaded into Molsoft ICM-Pro (version 3.4) and converted to Molsoft object (hydrogen atoms were added and optimized). The dicamba contact residues in the binding pocket with orientation elucidated from "Crystal structure 6" were calculated using default Molsoft parameters. Corresponding residues in toluene sulfonate monooxygenase and vanillate monooxygenase (aka Vanillate O-demethylase) were identified from sequence alignment for the purpose of further engineering of these oxygenases (e.g. Tables 16, 23).

The resulting structure indicates that the list of residues predicted to form the dicamba binding pocket, by modeling described above, are contained in the pocket identified based on the actual X-ray structure of DMO with dicamba. The list of predicted residues is as follows; underlined residues were identified to be within 4Å and/or to participate in binding or to be in contact with the dicamba molecule (see FIG. 9 as well): L155, D157, L158, H160, A161, H165, R166, A169, Q170, D172, A173, A216, W217, N218, 1220, N230, I232, A233, V234, S247, G249, H251, S267, L282, W285, Q286, A287, Q288, A289, V291.

Many of the DMO active site residues forming the binding pocket and those interacting with the substrate (i.e. within a 4Å distance) as identified by the three dimensional structure are not readily identifiable by primary amino acid sequence alignment, for instance as shown in FIG. 9 or FIG. 21.

**Table 16: Residues in contact with the dicamba molecule as determined with the Molsoft program, with corresponding residues from TolO's, VanO's by alignment. Residues S247 and S267 were determined to be within the 4Å radius but not identified as being in direct contact with dicamba.**

| **Residue (crystal 6)** | **ContactArea (Å²)** | **ExposedArea (Å²)** | **Percent (contact/exposed X100)** | **AA in TolO** | **AA in VanO** |
|---|---|---|---|---|---|
| I232 | 34.0 | 150.4 | 22.6% | T | V |
| N230 | 17.2 | 95.8 | 18.0% | M | I |
| H251 | 15.0 | 151.6 | 9.9% | S | D |
| Q288 | 9.0 | 104.0 | 8.7% | (V) | (Q) |
| N218 | 8.1 | 139.1 | 5.8% | Q | Q |
| A161 | 6.6 | 72.0 | 9.2% | L | E |
| L155 | 2.5 | 171.3 | 1.5% | L | L |
| L282 | 3.5 | 163.2 | 2.1% | I | I |
| H160 | 0.6 | 154.0 | 0.4% | H | H |
| G249 | 1.9 | 57.8 | 3.3% | H | V |

FIG. 16 shows dicamba in the active center of DdmC; the surface rendering is based on electrostatic potential. FIG. 19 shows the dicamba binding site with subunit "b" in orange. The dicamba binding site is exclusively in one subunit. Within a 4Å radius the residues (shown in green) that interact with the dicamba through Van der Waals interactions or polar interactions, such as hydrogen bonds, are as follows: L155, H160, A161, N218, N230, I232, S247, G249, H251, S267, L282, and Q288. Key H-bonds which play a role in orientation of the dicamba are from residues H251 and N230 (NE2 2.9 Å to carboxylate O1 and ND2 to 02 2.8 Å). Within a 5Å radius the following additional residues also play a role in substrate binding or pocket formation: L158, H165, A169, I220, R248, T250, G266, S267, A287, and A289. The non-heme iron is shown as an aqua sphere and is 5Å away (distance shown in FIG. 19) from the methyl carbon of the methoxy group of dicamba. The Rieske center (yellow aqua diamond shape) of the neighboring subunit c (in gray) is shown in the top right of the graphic. FIG. 20 shows the binding surface describing the 4Å interaction residues which are shown as spacefill models with CPK coloring (blue-nitrogen, red-oxygen grey-carbon). The space into which the carboxylate binds is in the back of the pocket in blue, and the large spaces for the chlorines are also observed. In addition the hydrophobic surface presented at the bottom of the figure (chiefly I232) is clearly defined. This surface defines the binding space at 4Å for dicamba in crystal 6, using the atomic coordinates of Table 4. Color code: Green - hydrophobic; Red - hydrogen bond acceptor; Blue - hydrogen bond donor. In both FIGs. 19 and 20, dicamba is colored as follows: carbons -gold, oxygens-red, chlorines -green.

### C. Active site modeling with DCSA bound

As can be seen in FIG. 18 and in Table 17, Asn230 plays a critical role in properly orienting dicamba in the DMO active site for dealkylation. The side chain nitrogen (ND2) atom of Asn230 is involved in two hydrogen bonds with dicamba, and these hydrogen bonds involve two of the oxygen atoms in dicamba - 03, the oxygen atom involved in the dealkylation, and 02, one of the carboxylate moiety oxygen atoms. These hydrogen bonds, along with the His251-Dic O1 bond, appear to be critical in directing the methoxy oxygen and methoxy group toward the non-heme iron for catalysis. Not surprisingly, these key interactions are also seen in the DMO-DCSA structure (FIG. 23 and Table 18). It's worth noting that in the crystal structure of the nitrobenzene dioxygenase-nitrobenzene complex from *Comamonas sp.* (PDB entry 2BMQ; Friemann *et al,* 2005; Ferraro *et al.,* 2005), which is unlike most known RO-substrate crystal structures in that the substrate contains a polar nitro moiety, hydrogen bonds between the nitro group and Asn258 are important to proper substrate orientation, and a mutation of N258V disrupts the regio-selectivity of product formation (Ferraro *et al.,* 2005).

**Table 17. DMO-Dicamba Hydrogen Bonds in the DMO Crystal 6 Structure**

| Mol. B DMO-DIC H Bonds | | |
|---|---|---|
| Hydrogen Bond Donor | Hydrogen Bond Acceptor | Distance (Å) |
| 230 Asn ND2 | 601 Dic O2 | 2.8 |
| 230 Asn ND2 | 601 Dic O3 | 2.8 |
| 251 NE2 | 601 Dic O1 | 2.9 |

| Mol. C DMO-DIC H Bonds | | |
|---|---|---|
| Hydrogen Bond Donor | Hydrogen Bond Acceptor | Distance (Å) |
| 230 Asn ND2 | 601 Dic O2 | 2.8 |
| 230 Asn ND2 | 601 Dic O3 | 3.0 |
| 251 NE2 | 601 Dic O1 | 3.3 |

**Table 18. DMO-DCSA Hydrogen Bonds in the DMO Crystal 7 Structure**

| Mol. A DMO-DCSA H Bonds | | |
|---|---|---|
| Hydrogen Bond Donor | Hydrogen Bond Acceptor | Distance (Å) |
| 230 Asn ND2 | 601 Dcs O2 | 3.2 |
| 230 Asn ND2 | 601 Dcs O3 | 3.3 |
| 251 NE2 | 601 Dcs O1 | 3.1 |
| Mol. C DMO-DCSA H Bonds | | |
| Hydrogen Bond Donor | Hydrogen Bond Acceptor | Distance (Å) |
| 230 Asn ND2 | 601 Dcs O2 | 2.8 |
| 251 NE2 | 601 Dcs O1 | 2.9 |

### Example 7

### Electron Transport

The electron transfer distances in the DMO Crystal 5 coordinates are listed in Table 19 below. The electron transfer path from a Rieske center to the non heme iron center with an activated oxygen and ultimately resulting in the oxidation of the dicamba to DCSA are described, using the atomic coordinates of Crystal 5, for the three active sites in the entire trimer with approximate atomic distances, and "A", or "B" or "C" denoting in which monomer the given residue is found.

**Table 19. Electron Transfer Distances in the DMO Crystal 5 Structure**

| Molecule B-Molecule A Interface | Distance (Å) |
|---|---|
| B501 Fes FE2 - B71 His ND1 | 2.4 |
| B71 His NE2 - A157 Asp OD 1 | 3.2 |
| A157 Asp OD1 - A160 His ND1 | 2.8 |
| A160 His NE2 - A502 Fe FE | 2.3 |

| Molecule A-Molecule C Interface | Distance (Å) |
|---|---|
| A501 Fes FE2 - A71 His ND1 | 2.6 |
| A71 His NE2 - C157 Asp OD1 | 2.8 |
| C157 Asp OD1 - C160 His ND1 | 2.8 |
| C160 His NE2 - C502 Fe FE | 2.5 |

| Molecule C-Molecule B Interface | Distance (Å) |
|---|---|
| C501 Fes FE2 - C71 His ND1 | 2.7 |
| B71 His NE2 - B157 Asp OD 1 | 3.0 |
| B157 Asp OD1 - B160 His ND 1 | 2.8 |
| B160 His NE2 - B502 Fe FE | 2.5 |

The entire set of residues that forms the extended electron transport chain is H71, N154, D157, H160, H165 and D294 numbered corresponding to SEQ ID NO:2 or SEQ ID NO:3, or conservative substitutions thereof. These residues constitute a motif and the distances and arrangements above constitute a necessary element of the functional catalytic enzyme. On average the distance for Fes FE2 to His71 ND1 is 2.57Å±0.15; the distance for the His71 NE2 to Asp157 OD1 is 3.00 Å ±0.20, the distance for Asp157 OD1 to His160 ND1 is 2.80Å, and the distance for His 160 NE2 to Fe is 2.43±0.12. These distances may vary by about 0.2- 0.3Å.

### Example 8

### DdmC Variant Generation and Activity Screen

The closest potential homologs of DdmC were identified by Blast search (Altschul *et al.,* 1990), selected for chemical similarity of substrate and reaction, and aligned (Molsoft ICM-Pro, version 3.4; Molsoft LLC, La Jolla, CA). Identity and similarity tables for the sequences used is shown below appendix. These were aligned using the ZEGA algorithm inside ICM pro for multiple sequence alignments. Based on the alignment, several regions for degenerate oligonucleotide tail ('DOT'; FIG. 13) mutagenesis were identified (FIG. 14). The changes were designed to sample from the diversity observed in polypeptides with sequence similarity at the primary level. Additional conservative amino acid substitutions were included in the designs as well.

The sets of DOT primers (SEQ ID NOs:46-151) introducing the amino acid combinations indicated below were designed and used to introduce mutations into the *ddmC* gene by means of terminated PCR on the template (*ddmC* gene with His tag and two changes, T2S + I123L (SEQ ID NO:23) or V4L + L281I (SEQ ID NO:42) in pMV4 vector (Modular Genetics, Cambridge, MA). Resulting PCR products were treated with *Dpn***I** to remove the parental template molecules, self-annealed and transformed into chemically competent *E. coli* Top10 F' (Invitrogen, Carlsbad, CA). Standard DNA cloning methods were utilized *(e.g.* Sambrook *et al.,* 1989). The individual colonies were grown in liquid culture. DNA was isolated by a standard miniprep procedure and used to transform the chemically competent *E. coli (e.g.* BL21 (DE3)).

An LC-MS/MS screen for oxygenase activity was used to detect DCSA. The method comprises a two stage process made up of a liquid cell culture assay coupled to an LC-MS/MS detection screen. In the liquid cell culture stage, the gene of interest, *i.e. ddmC* or a variant thereof, was cloned under the control of a promoter (e.g. T7 promoter, pET vector) and transformed into an *E. coli* host cell. The transformed *E. coli* cells harbouring the gene of interest were then grown in LB/carbenicillin media containing 200 to 500 µM Dicamba (30 hrs, 37°C, shaking at 450 RPM). Cells were spun down, and the supernatant was filtered and diluted tenfold with the inclusion of 8 µM salicylic acid as an internal standard. Samples of the supernatant, and/or optionally the cell pellet (or lysate thereof), from this first stage were analyzed for DCSA levels *(i.e.* DMO activity level) by LC-MS/MS.

This method was used to rapidly screen and provide feedback regarding enzymatic activity for use in protein design and engineering procedures, or to screen libraries of genes (e.g. bacterial genes) for activity toward dicamba or other similar substrates. While many oxygenases such as DMO are multi component systems requiring other helper enzymes for activity, it was observed that components in E *coli* may substitute for these helper enzymes or functions. Thus, transformation of E. *coli* with a single "oxygenase" gene from a multi component system nevertheless results in measurable activity for the gene alone, even without other components being co-transformed into the same E. *coli* cell. Activity is observed because, in E. *coli* cells, surrogates with homology to the original helper enzymes (e.g. ferredoxin and reductase) may be utilized. Additionally, *E*. *coli* can take up substrate (*e.g*. dicamba) and excrete the product *(e.g.* DCSA) into the media supernatant, allowing for the speed and simplicity of this cell based screen. No lysis of cells is required. Alternatively, an HPLC-based assay for DMO activity may be utilized. Promising variants were used as templates for additional rounds DOT and or other mutagenesis methods in an iterative manner.

Table 20 illustrates an identity and similarity table calculated using, for instance, NEEDLE a pairwise global alignment program (GAP, based on the Needleman-Wunsch global alignment algorithm to find the optimum alignment (including gaps) of two sequences when considering their entire length), and aligned as shown in FIG. 14 (using ZEGA algorithm), with a set of TolO and VanO sequences utilized for selection of corresponding DMO residues to be mutagenized.

**Table 20. Summary of results of NEEDLE Global alignments between DMO (DdmC) and selected potential homologs used in FIG. 17.**

| Sequence 1% | 73538170 | 1790867 TolO | 13661652 | 55584974 Ddm C | 90415596 | 76794499 | 83746974 VanO |
|---|---|---|---|---|---|---|---|
| 73538170 | 100 | 52 | 48.2 | 37.2 | 34.8 | 32.5 | 37.5 |
| 1790867TolO | | 100 | 82 | 34.7 | 32.6 | 31.3 | 35.5 |
| 13661652 | | | 100 | 34.5 | 30.9 | 33.1 | 37.3 |
| 55584974Ddm_C | | | | 100 | 35.1 | 34.2 | 37.7 |
| 90415596 | | | | | 100 | 46.7 | 47 |
| 76794499 | | | | | | 100 | 63.3 |
| 83746974VanO | | | | | | | 100 |
| 73538170 | 100 | 69 | 63.9 | 49.6 | 50.8 | 48.2 | 51 |
| 1790867TolO | | 100 | 88 | 49.2 | 54.8 | 49.3 | 51.9 |
| 13661652 | | | 100 | 49 | 53.7 | 51.2 | 53.5 |
| 55584974Ddm_C | | | | 100 | 48.7 | 47.9 | 51.5 |
| 90415596 | | | | | 100 | 61.5 | 61.3 |
| 76794499 | | | | | | 100 | 77.7 |
| 83746974VanO | | | | | | | 100 |

### A. Non-heme iron and Electron Transport Variants

The DMO crystallographic data so far have revealed that His160, His 165, and Asp294 are involved in chelating the non-heme iron ion, and that Asn154 plays an ancillary role in the non-heme iron chelation, and this can be seen in FIG. 2. In addition, as was noted above, in a DMO structure with the non-heme iron site occupied, such as DMO Crystal 5 in FIG. 2, in converting dicamba to DCSA, electrons must flow in the DMO trimer from the from the Fe₂S₂ cluster to His71 in one molecule, to the following residues in a neighboring molecule: Asp157, His160, and then to the non-heme iron site. Asp157, which transfers electrons from the Rieske cluster of a neighboring molecule to the non-heme iron ion within its subunit, is clearly a key to electron transfer. These structural data suggest that mutating N154, D157, H160, H165, or D294 should severely impair or destroy DMO enzymatic activity. To confirm the importance of these residues, variant polypeptides with mutation(s) corresponding to these residues were prepared and assayed for enzymatic activity.

Based on the DMO three dimensional structure, five amino acid mutants interfering with electron transport and non-heme iron coordination were initially suggested: N154A, D157N, H160N, H165N, D294N. Five pairs of GeneDirect primers (SEQ ID NO:32-41; Table 22) introducing the individual mutations were designed and used as PCR primers on the template, a *ddmC* gene with two changes, T2S and I123L (SEQ ID NO:23), cloned in the pMV4 vector (Modular Genetics, Inc. Cambridge, MA). The resulting PCR products were treated with *Dpn*I to remove the parental template molecules, self-annealed and transformed into chemically competent E. *coli* Top10 F' (Invitrogen, Carlsbad, CA). The individual colonies were grown in liquid culture and DNA was isolated by a standard miniprep procedure and used to transform chemically competent *E. coli* BL21(DE3). The *E. coli* culture was grown in LB/carbenicillin media containing 500 µM Dicamba (30 hrs, 37 °C, 450 RPM). Cells were spun down, and the media was filtered and diluted tenfold with 8 µM salicylic acid as an internal standard. The samples were frozen and DCSA levels (i.e. DMO activity level) were determined by LC-MS analysis. Results are shown in Table 21, demonstrating that these residues participate in electron transport and non-heme iron coordination.

**Table 21. Relative enzymatic activity of DMO variants with altered residues involved in electron transport.**

| **Mutant** | **% of wt activity** |
|---|---|
| **N154A** | 0-2 |
| **D157N** | 23 |
| **H160N** | 0 |
| **H165N** | 0 |
| **D294N** | 0 |

**Table 22. Genedirect primers for introduction of single amino acid changes. Abbreviation mA denotes 2'-O-methylated A, mC denotes 2'-O-methylated C, mG denotes 2'-O-methylated G, mU denotes 2'-O-methylated U (note there is no mT) (SEQ ID NOs:32-41).**

| | |
|---|---|
| dSL_M_6his_pMV4_1-77-4-sense D157N | TGAACCTCGGCCACmGCCCAATATGTCCATCGCGC |
| dSL_M_6his_pMV4_1-77-4-anti D157N | CGTGGCCGAGGTTCmATCAGGTTGTCGACCAGCAGCT |
| dSL_M_6his_pMV4_1-77-5-sense D294N | GGAGAACAAGGTCGTCmGTCGAGGCGATCGAGCGC |
| dSL_M_6his_pMV4_1-77-5-anti D294N | CGACGACCTTGTTCTCmCTTGACCAGCGCCTGAGCC |
| dSL_M_6hispMV4_1-77-6-sense H160N | CGGCAACGCCCAATmATGTCCATCGCGCCAACG |
| dSL_M_6his_pMV4_1-77-6-anti H160N | TATTGGGCGTTGCCmGAGGTCCATCAGGTTGTCGACCA |
| dSL_M_6his_pMV4_1-77-7-sense H165N | ATGTCAATCGCGCCmAACGCCCAGACCGACGCC |
| dSL_M_6his_pMV4_1-77-7-anti H165N | TGGCGCGATTGACAmUATTGGGCGTGGCCGAGG |
| dSL_M_6his_pMV4_1-77-8-sense N154A | GCTGGTCGACGCCCmUGATGGACCTCGGCCACGC |
| dSL_M_6his_pMV4_1-77-8-anti N154A | AGGGCGTCGACCAGmCAGCTTGTAGTTGCAGTCGACATGC |

These data clearly confirm the importance of residues N154, D157, H160, H165, or D294 to the functioning of DMO. Mutating any of the residues implicated in non-heme iron binding - H160, H165, & D294 - leads to an inactive enzyme relative to the wild type (WT) enzyme, and mutating N154, which also appears to play a role in iron chelation, yields an enzyme with only 2% or less activity relative to the WT. Moreover, mutating D157, the aspartate residue responsible for electron transfer between DMO's protein subunits, to Asn results in an enzyme with only 23% activity relative to the WT. This indicates that N157, which is iso-structural to D157, still has some minor electron transfer capabilities relative to the WT enzyme. Additional variants and their activities are also described in Example 12.

### B. C-Terminal Helix Variants

The C-terminal helix of the DdmC protein is defined by the following residues (AAVRVSREIEKLEQLEAA crystal structure residue numbers 323-340; SEQ ID NO:24; FIG. 15). The surface of this helix, which is exposed to solvent in the crystal structure, has significant hydrophobic character. Five of the approximately nine surface residues are hydrophobic (aliphatic) in nature. These residues are L337, L334, I331, V327, and A324. The residues L337, L334 and I331 form a cluster of hydrophobic residues on the surface of the upper half of this helix (see FIG. 15). Conversion of L334 (crystal structure numbering) to the conservative substitution I results in complete loss of activity in the *in vivo* screen. This residue is part of hydrophobic region that is necessary for activity and is likely included in the surface that interacts with helper proteins; most likely ferredoxin. Additional variants are described in Examples 12 and 13.

Additional changes were also made in the IEKLEQLE (SEQ ID NO:25) region (SEQ ID NOs:26-31) which includes this residue; some examples are shown in Table 23. None of these mutants showed detectable activity in an *in vivo* screen. However, some residues in this region may be changed while retaining DMO activity.

**Table 23. Changes made to the C-terminal helix which resulted in no activity (SEQ ID NOs: 25-31).**

| Mutant starting material | IEK**I**EQLE | | L334I |
|---|---|---|---|
| Native sequence | IEKLEQLE | | |
| Changes made | LDRLDDID | | Exemplary variants screened. |
| | VH | VHEVQ | |
| | N | QH H | No activity observed. |
| | Q | K | |
| | K | N | |

### C. Interface Residue Variants

The F53 side chain of subunit a inserts into a hydrophobic cavity as mentioned above. This residue is not highly conserved in other oxygenases of this type. Site-directed mutagenesis and activity assays indicate that residue F53 can be altered, for instance to histidine and to leucine, which are functionally equivalent to phenylalanine for hydrophobic interactions, and retain some activity. Additional variants are described in Examples 12-13.

### Example 9

### Primary Sequence Alignments

Polypeptides encoded by genes for Toluene sulfonate mono-oxygenases ("TolO's" and like) and Vanillate O-demethylases ("VanO's" and like; Table 24, (SEQ ID NOs:4-22) were aligned with DMO (SEQ ID NO:1) to evaluate available oxygenase enzymes with the highest known degree of identity or similarity to DMO (e.g. Table 20, FIG. 9; FIG. 17). The smaller set for which identity and similarity are described in Table 20 is aligned in Figure 17. Figure 9 and Figure 21 extend these alignments to include more distantly related oxygenases. These alignments serve to define the conserved nature of the interaction domain as described in Table 14 for example. In addition the conserved residues in these alignments could be considered to make up an oxygenase superfamily motif in general.

**Table 24. Sequence identifiers and descriptions of sequences used for alignments.**

| **SEQ ID NO** | **NCBI GI sequence identifier or PDB identifier** | **Description** | **Source organism** |
|---|---|---|---|
| 8 | 86749031 | Rieske (2Fe-2S) protein | *Rhodopseudomonas palustris* HaA2 |
| 10 | 90415596 | Vanillate O-demethylase | Gamma proteobacterium HTCC2207 |
| 7 | 78045933 | putative vanillate O-demethylase oxygenase subunit | *Xanthomonas campestris* pv. *vesicatoria* str. 85-10 |
| 13 | 28853329 | putative vanillate O-demethylase oxygenase subunit | *Pseudomonas syringae* pv. *tomato* str. DC3000 |
| 5 | 1790867 | toluenesulfonate methyl-monooxygenase oxygenase component | *Comamonas testosteroni* |
| 1 | 55584974 | DdmC | *Pseudomonas maltophilia* |
| 14 | 70730833 | vanillate O-demethylase, oxygenasesubunit | *Pseudomonas fluorescens* Pf 5 |
| 9 | 78693673 | vanillate O-demethylase oxygenase subunit | *Bradyrhizobium* sp. BTAil I |
| 12 | 49530160 | vanillate O-demethylase oxygenase subunit | *Acinetobacter* sp. ADP |
| 16 6 | 1946284 | *Pseudomonas* sp. *vanA* gene | *Pseudomonas* sp. |
| 4 | 73538170 | Rieske (2Fe-2S) region | *Ralstonia eutropha* JMP134 |
| 11 1 | 76794499 | Rieske (2Fe-2S) region | *Pseudoalteromonas atlantica* T6c |
| 6 | 13661652 | monooxygenase TsaM2 | *Comamonas testosteroni* |
| 19 | 8118285 | polyaromatic hydrocarbon dioxygenase large subunit | *Comamonas testosteroni* |
| 18 | 17942397 | DntAc | *Burkholderia cepacia* |
| 17 | gi 67464651 (PDB 2BMO; PDB 2BMR) | Nitrobenzene Dioxygenase, chain A | *Comamonas* sp.; strain: Js765 |
| 20 | PDB 2B24; PDB 2B1X | naphthalene 1,2 dioxygenase | *Rhodococcus sp.* |
| | | (NDO-R) | |
| 21 | PDB1ULI | biphenyl dioxygenase | *Rhodococcus* sp |
| 22 | PDB 1EG9; PDB 1NDO | naphthalene 1,2 dioxygenase | *Pseudomonas* sp |
| 15 | 83746974 | Vanillate O-demethylase oxygenase subunit | *Ralstonia solanacearum* strain UW551 |

### Example 10

### Additional DMO Crystal Structures

Additional DMO crystal soaking and structure determination has yielded refined structures with Co⁺² in the non-heme iron site at higher resolution, and coordinates as shown in Table 25 ("DMO Crystal 11"), with R_{work}= 27.0% and R_{free}=30.2% for 20-2.05 Å data, and Table 26 ("DMO Crystal 12"), with R_{work}= 24.5% and R_{free}= 27.9% for 20-2.05 Å data. "Crystal 11" represents the refined structure with cobalt and dicamba, while "Crystal 12" represents the refined structure with cobalt and DCSA. The data collection and refinement statistics for DMO Crystal 11, and DMO Crystal 12, are listed in Table 27.

**Table 27. Data collection and refinement statistics on DMO Crystal 11 and DMO Crystal 12.**

| | DMO Crystal 11 | DMO Crystal 12 |
|---|---|---|
| Wavelength (Å) | 1.000 | 1.000 |
| Space Group | P3₂ | P3₂ |
| Cell dimensions | | |
| *a, b, c* (Å) | 81.55,81.55, 161.29 | 81.01,81.01, 161.05 |
| α, β, γ (°) | 90.0, 90.0, 120.0 | 90.0, 90.0, 120.0 |
| Resolution (Å) | 50-2.05 (2.12 - 2.05)* | 50-2.05 (2.12 - 2.05)* |
| *R*_{sym} or *R*_{merge} | 0.068 (0.485) | 0.069 (0.451) |
| *I*/*σI* | 23.1 (1.7) | 22.8 (1.9) |
| Completeness (%) | 99.6 (99.9) | 99.5 (99.9) |
| Redundancy | 4.1 (4.0) | 4.0 (4.0) |
| | | |
| Refinement | | |
| Resolution (Å) | 20 - 2.05 | 20 - 2.05 |
| No. reflections | 61905 | 60645 |
| *R*_{work} / *R*_{free} | 27.0% / 30.2% | 24.5% / 27.9% |
| No. atoms | | |
| Protein | 7722 | 7653 |
| Ligand/ion | 54 | 56 |
| Water | 0 | 381 |
| *B*-factors (Å²) | | |
| Protein | 42.0 | 44.3 |
| Ligand/ion | 47.9 | 62.0 |
| Water | -- | 46.2 |
| R.m.s deviations | | |
| Bond lengths (Å) | 0.007 | 0.006 |
| Bond angles (°) | 1.341 | 1.331 |

| | | |
|---|---|---|
| *Highest resolution shell is shown in parenthesis. | | |

These structures (Crystals 11 and 12) have Co⁺² in the non-heme iron site instead of Fe⁺². The Co⁺², dicamba, and DCSA were introduced into pre-formed protein crystals by soaking methods similar to those used to obtain the DMO-Fe⁺²-dicamba and DMO-Fe⁺²-DCSA structures, which have already been described. The crystals were grown by previously noted methods. The DMO-Co⁺²-dicamba crystals were soaked in stabilization solutions containing 10mM CoCl₂ and 1.25mM dicamba for 24 hours prior to cryo-cooling. The DMO-Co⁺²-DCSA crystals were soaked in stabilization solutions containing 10mM CoCl₂ and 1.25mM DCSA for 24 hours prior to cryo-cooling. The data and refinement statistics for the DMO-Co⁺²-dicamba crystal structure are listed under "DMO Crystal 11" in Table 27 and those for DMO-Co⁺²-DCSA are listed under "DMO Crystal 12" in Table 27. Data sets for DMO Crystal 11 and DMO Crystal 12 were collected on the SER-CAT 22-ID beamline at the Advanced Photon Source synchrotron, (Argonne National Laboratory, Argonne, IL). The X-ray wavelength employed was 1.000Å, and these data were collected using a Mar300 CCD detector (Mar USA, Evanston, IL).

### Example 11

### Creation of Variant DMO Polypeptides- NNS Mutagenesis

Sets of saturation mutagenesis primers (using NNS degenerate triplets) for 31 residues at the active site and involved in the electron transfer chain as indicated in Table 28 were designed and used to introduce mutations into the *ddmC* gene by means of terminated PCR on the template *(ddmC* gene with His tag and two changes, T2S + I123L (SEQ ID NO:23) in pMV4 vector (Modular Genetics, Cambridge, MA). Resulting PCR products were treated with *DpₕI* to remove the parental template molecules, self-annealed and transformed into chemically competent E. *coli* Top10 F' (Invitrogen, Carlsbad, CA). Standard DNA cloning methods were utilized (e.g. Sambrook *et al.,* 1989). The individual colonies were grown in liquid culture. DNA was isolated by a standard miniprep procedure and used to transform the chemically competent *E. coli (e.g.* BL21(DE3)). The *E. coli* culture was grown in LB/carbenicillin media containing 200 to 500 µM Dicamba (30 hrs, 37°C, 450 RPM). Cells were spun down, and the supernatant was filtered and diluted tenfold with 8 µM salicylic acid as an internal standard. The DCSA levels *(i.e.* DMO activity level) were determined by LC-MS analysis as described above (e.g. Example 8).

Determination of activity of mutants at specific residues as shown in Table 28 indicated that certain residues tolerate changes, while others did not. Interestingly, while N154 is outside of the 5Å sphere for the substrate based on the structural determination, it is within the chelating sphere for the non-heme iron. N154 is though to play a role in metal binding and possibly in modulating the activation of oxygen. Substitution at L158 resulted in loss of activity. H160 could not be changed to any other residue tried while retaining > 2% activity as compared to the wild type enzyme. H160 is thus a key Fe ligand and electron transfer residue required for activity. Likewise, substitution at H165, another key Fe binding residue, also resulted in loss of activity in all cases except one, when M was substituted for very low activty. Substitution at I232, a hydrophobic contact to substrate / product in the active site, only resulted in retention of appreciable activity when the conservative substitution, to Val, was made. Substitution at G249 resulted in loss of most activity. Substitution by a larger residue at G249 appears to be sterically unfaforable and likely interferes with hydrogen bonding to the substrate and/or product by neighboring residues. No good substitutions were found for T250, H251, Y263. and F265. All residues play a role in forming the three dimensional inner and outer sphere of the active site and H251 contributes a key polar contact , in this case a hydrogen bond. S267 and L282 were also inflexible toward substitution, i.e. showed a complete or nearly complete loss of activity following almost all substitutions. Refined crystal structure information indicated that W285 is a residue for substrate and product binding. It engages in Van der Waals contact with substrate/product at the active site. The saturation mutagenesis results confirm this in that no viable substitution for W285 was observed while retaining any appreciable activity. L290 was also generally intolerant of substitutions, with only one variant, comprising the conservative substitution I, retaining >50% activity as compared to wild type.

In contrast, certain residues tolerated a degree of substitution while retaining moderate activity or even demonstrating increased enzymatic activity. Thus, for instance, substitution at A169, N218, S247, R248, L282, G266, A287, or Q288 could yield a variant enzyme with >50% of wild type activity, while substitution(s) at A169, N218, R248, G266, L282, A287, or Q288, resulted, in at least some instances, in variant enzymes with activity increased above the control level. In particular, R248 and A287 showed a high flexibility for substitution while retaining activity or even showing increased activity. Although most substitutions at G266, a residue in the outer part of the carboxylate binding pocket, resulted in loss of activity, G266S was more active than the control.

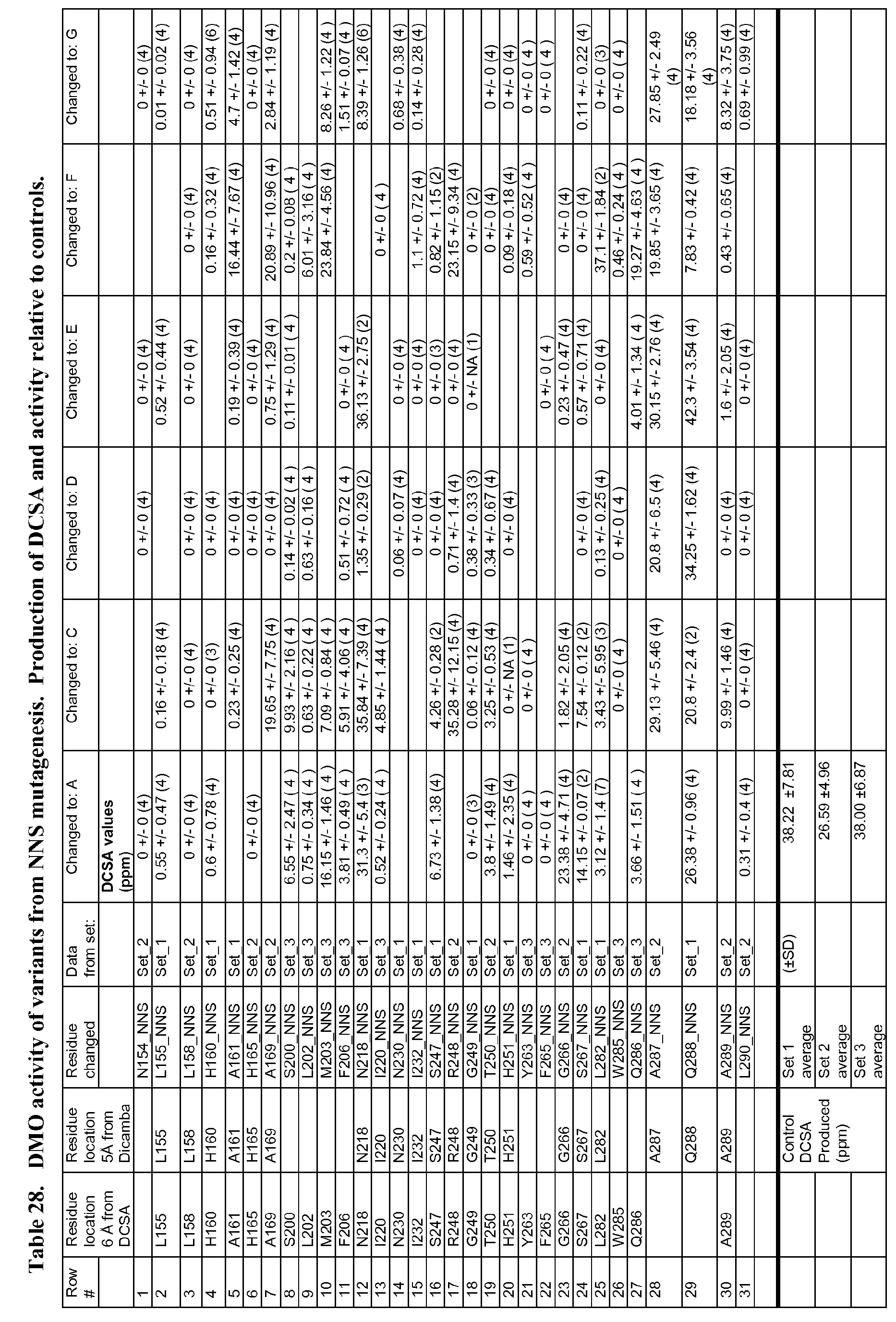

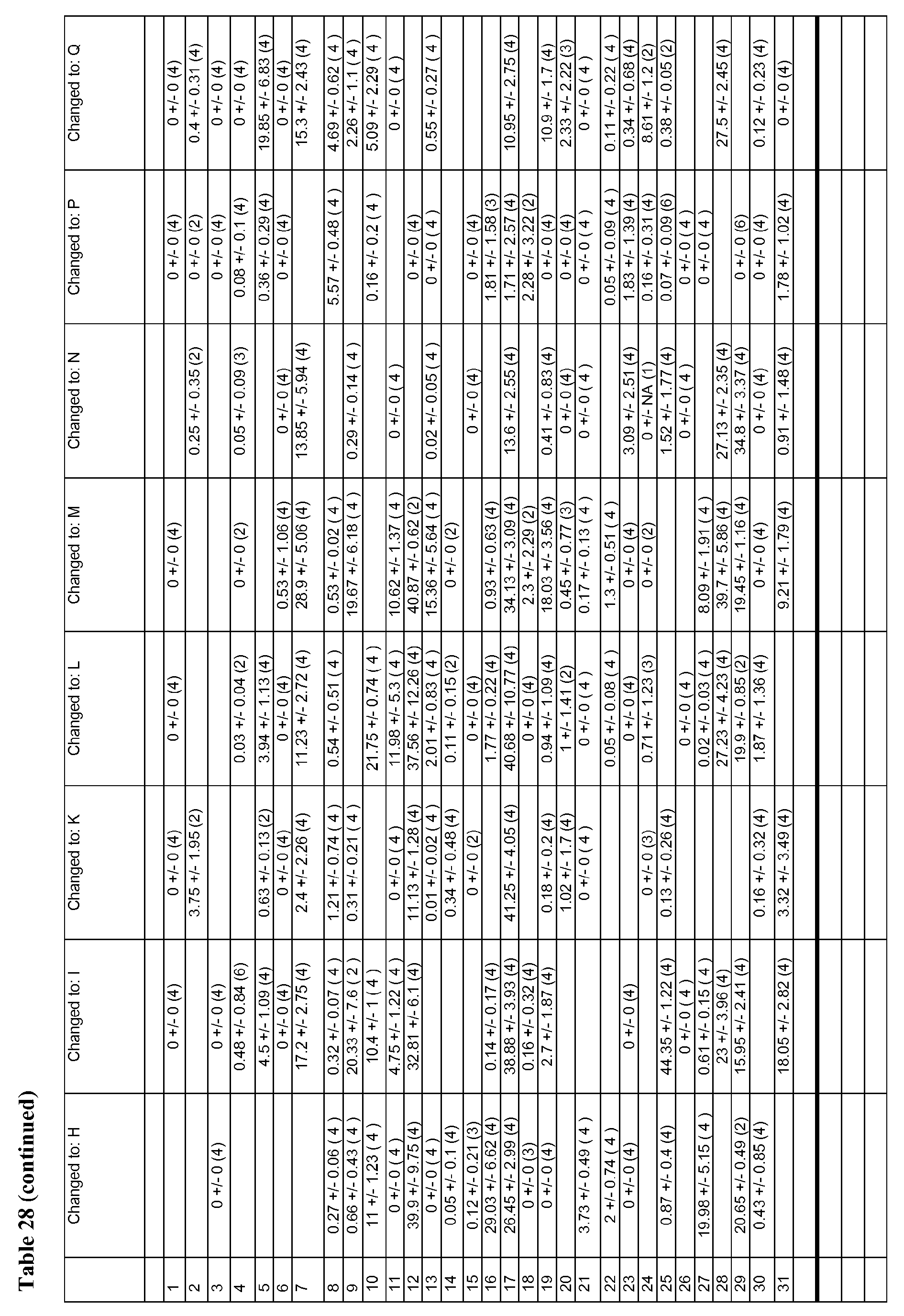

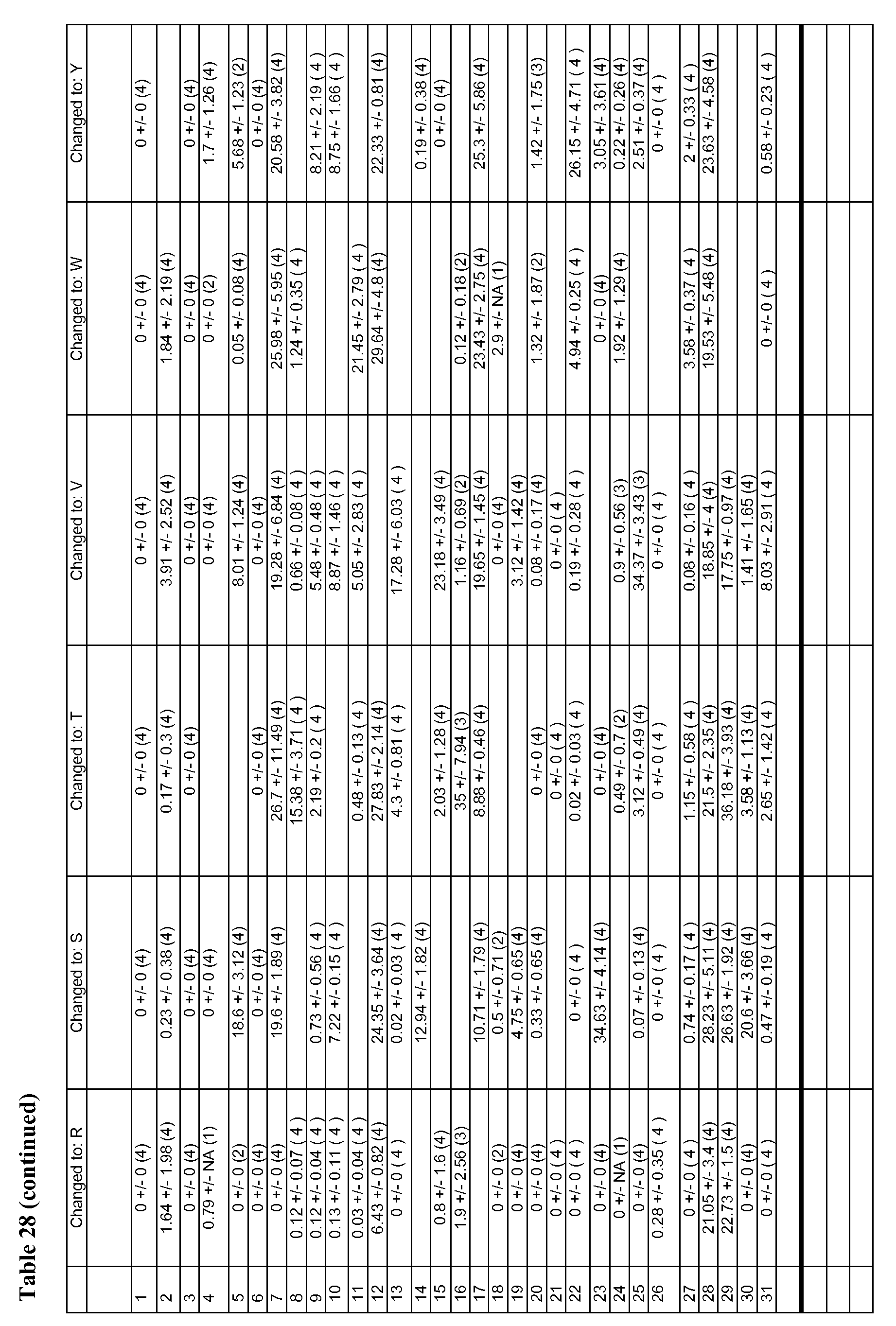

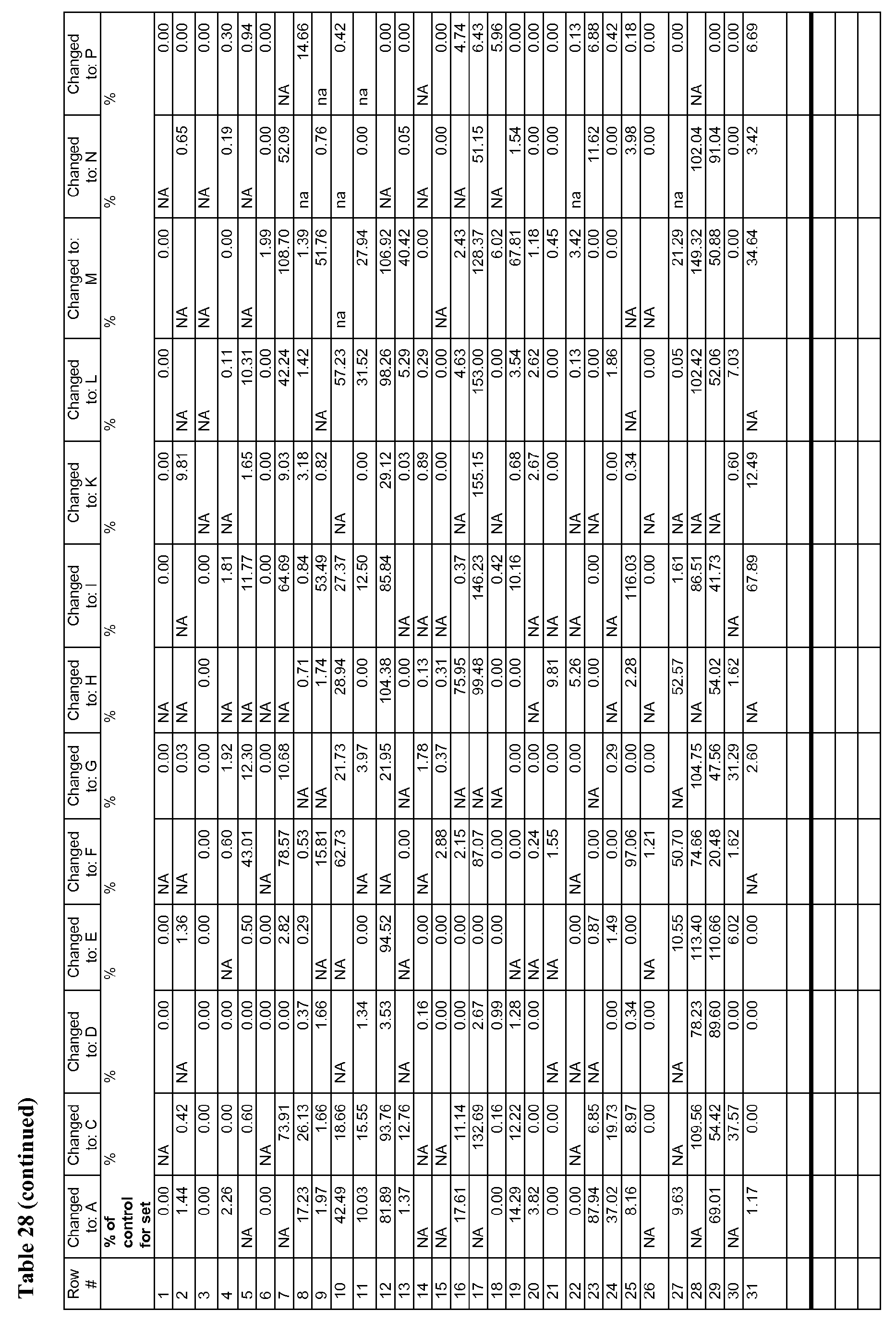

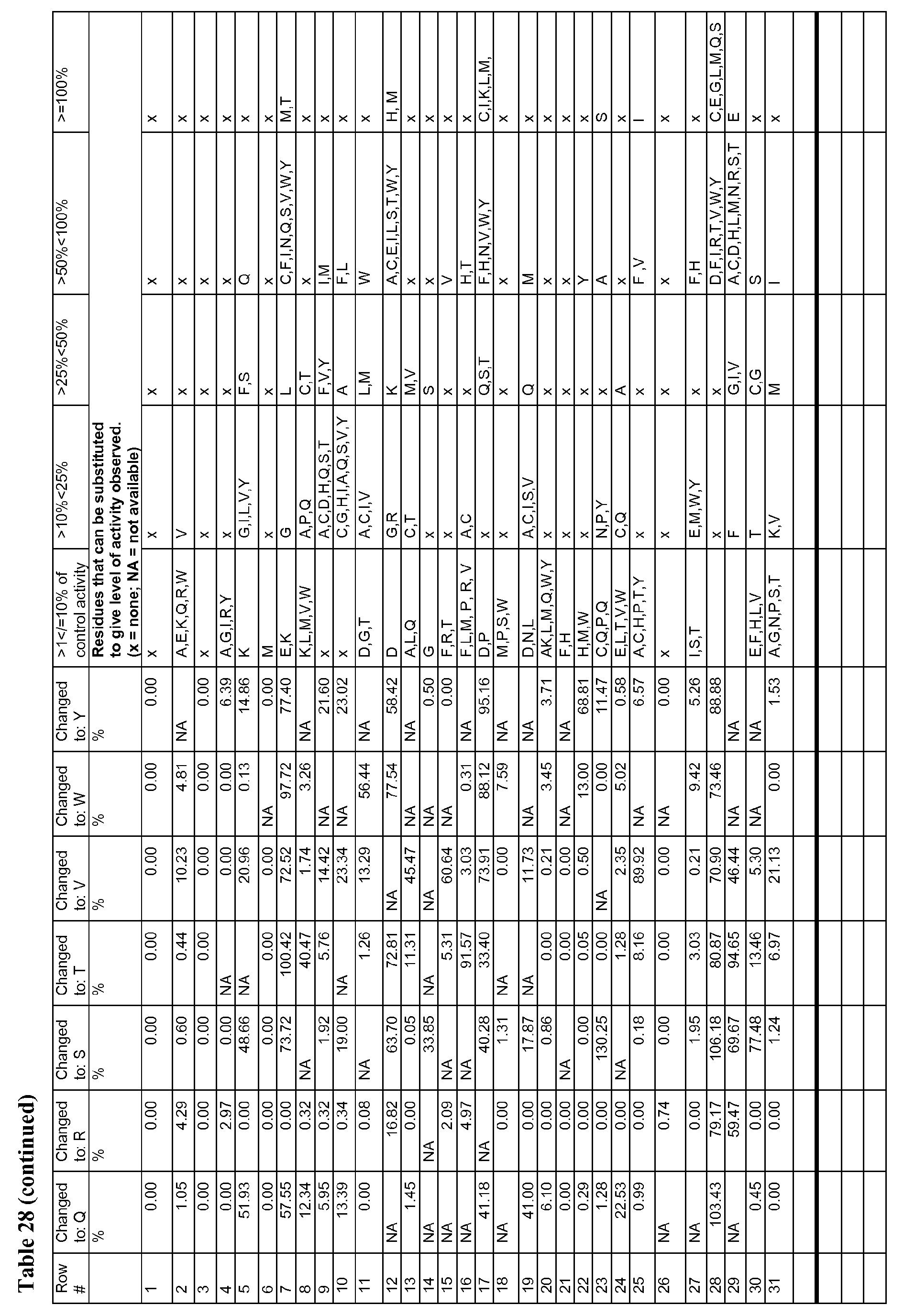

### Example 12

### Creation of Variant DMO Polypeptides- Single Substitutions

Additional single substitution variants of DMO at residues corresponding to interaction (e.g. subunit interface) domains and the ferredoxin binding site were also prepared and assayed for their effect on enzymatic activity (See also Examples 8A, 8B) by the LC/MS screen. In most cases shown in Table 29, one conservative and one neutral substitution was tried at a given residue. None of the tested variants displayed enzymatic activity greater than the wild type, and many substitutions resulted in >50% loss of activity.

**Table 29. Effect of single substitutions at Interface Domain and Ferredoxin binding residues.**

| **PARENTAL AA** | **ACTUAL CHANGED TO AA** | **AVERAGE ppm DCSA FOR VARIANT** | **STDEV_(Number of REPs)** | **% WT activity** | **Comments** |
|---|---|---|---|---|---|
| A316 | T | 21.67 | _+/-1.22 (4) | 57.03 | Hydrophilic-Interface of subunits |
| A316 | V | 18.68 | _+/-1.6(4) | 49.16 | Hydrophobic-Interface of subunits |
| A93 | L | 17.22 | +/- 1.36 (4) | 45.32 | Hydrophobic-Ferredoxin interface prediction |
| A93 | T | 24.69 | _+/- 0.98 (4) | 64.97 | Hydrophilic-Ferredoxin interface prediction |
| E293 | A | 1.28 | +/- 0.09 (4) | 3.37 | Subunit interface; change to non-charged residue reduces activity >95%. Could also affect neighboring D294 which is a key Fe binding residue. |
| E293 | Q | 11.71 | _+/-1.59 (4) | 30.82 | Subunit interface; change to larger hydrophilic residue cuts activity by 70%. |
| F53 | S | 23.55 | _+/- 4.99 (4) | 61.97 | Interface substitution; Hydrophilic and hydrophobic both cut activity but tolerated. |
| F53 | Y | 20.04 | _+/- 3.54 (4) | 52.74 | Interface substitution; Hydrophilic and hydrophobic both cut activity but tolerated. |
| G159 | A | 10.11 | _+/-0.8(4) | 26.61 | A and V are the most conservative substitutions. Based on structure this is due to size. |
| G159 | V | 0.66 | _+/-0.07(4) | 1.74 | A and V are the most conservative substitutions and V causes almost complete loss of activity. Based on structure this is due to size. |
| H160 | A | 0.12 | _+/-0.08 (4) | 0.32 | Electron transfer; no good substitution expected |
| H160 | N | 0.43 | _+/-0.07(4) | 1.13 | Electron transfer; no good substitution expected |
| H51 | N | 0.11 | +/- 0.03 (4) | 0.29 | Electron transfer/ Rieske Center; no good substitution expected |
| H71 | A | 0.28 | _+/-0.06(4) | 0.74 | Electron transfer/ Rieske Center; no good substitution expected |
| H71 | N | 0.18 | _+/-0.03(2) | 0.47 | Electron transfer Rieske Center; no good substitution expected |
| L318 | A | 7.94 | _+/-3.84 (8) | 20.89 | Significant cross subunit contacts-both conservative and hydrophilic substitutions cause drop below 25% activity. |
| L318 | S | 6.24 | _+/-2.61(8) | 16.42 | Significant cross subunit contacts-both conservative and hydrophilic substitutions cause drop below 25% activity. |
| L334 | A | 5.52 | _+/- 0.27 (4) | 14.53 | L334 Ferredoxin interface. Changes reduce activity by ∼10x for reasonably conservative substitutions |
| L334 | I | 2.89 | +/- 0.38 (4) | 7.61 | L334 Ferredoxin interface. Changes reduce activity by ∼10x for reasonably conservative substitutions |
| M317 | A | 17.93 | _+/-1.01 (4) | 47.18 | Ferredoxin Interface residue; substitution reduces activity |
| M317 | S | 15.13 | _+/-0.79(4) | 39.82 | Ferredoxin Interface residue; substitution reduces activity |
| P315 5 | G | 25.73 | _+/- 2.34 (4) | 67.71 | Ferredoxin Interface; only G substitution made- ~30% loss in activity. |
| R166 | C | 13.28 | _+/-0.15(4) | 34.95 | Subunit interface; activity cut by 2/3. |
| R304 | A | 12.1 | _+/-L11(4) | 31.84 | Interface substitution; results in loss of 60% of activity |
| R304 | D | 0.58 | _+/- 0.26 (4) | 1.53 | Interface of subunits substitution; change in charge-large effect on activity likely loss of salt bridge to D47. |
| R314 | A | 16.47 | _+/-1.79 (4) | 43.34 | Ferredoxin interface; A substitution cuts activity by 60% |
| R314 | K | 19.57 | _+/-3.41(4) | 51.50 | Ferredoxin interface; K conservative substitution cuts activity by 50% |
| R326 | A | 16.57 | _+/- 1.42 (4) | 43.61 | Ferredoxin interface; A substitution cuts activity by ~60% |
| R326 | K | 17.19 | _+/-2.64(4) | 45.24 | Ferredoxin interface; K conservative substitution cuts activity by ~60% |
| R329 | A | 14.9 | _+/- 3.06 (4) | 39.21 | Ferredoxin interface; A substitution cuts activity by 60% |
| R329 | K | 20.83 | _+/-1.5(4) | 54.82 | Ferredoxin interface; K conservative substitution cuts activity by 50% |
| R52 | A | 8.26 | _+/-1.15 (4) | 21.74 | Interface of subunits; substitution change loss of 80% activity |
| R52 | K | 18.07 | _+/-3.35(4) | 47.55 | Interface of subunits; substitution change loss of 20% activity with conservative change. Positive residue problem desired here possible salt bridge to D153 |
| S94 | A | 17.43 | +/- 0.78 (4) | 45.87 | Ferredoxin Interface residue; substitution reduces activity >50% |
| S94 | L | 14.04 | _+/- 0.68 (4) | 36.95 | Ferredoxin Interface residue; substitution reduces activity >50% |
| V327 | A | 5.07 | +/- 1.06 (4) | 13.34 | Ferredoxin interface; like L334 even a small change from V to still hydrophobic A causes >90% loss in activity |
| V327 | S | 0.55 | _+/-0.37(4) | 1.45 | Ferredoxin interface; change from V to hydrophobic residue causes >98% loss in activity. |
| Y163 | F | 16.63 | _+/-2.12(4) | 43.76 | Interface of subunits; key residue even very conservative substitution to F causes greater than 50% loss in activity. |
| Y163 | S | 0.9 | +/- 0.04 (4) | 2.37 | Interface of subunits; key residue non-conservative substitution to S causes nearly complete loss in activity. |
| Y307 | A | 11.23 | _+/- 0.65 (4) | 29.55 | Interface of subunits; even some what conservative substitution to A causes greater than 70 % loss in activity. |
| Y307 | F | 16.02 | +/- 0.36 (4) | 42.16 | Interface of subunits; even very conservative substitution to F causes greater than 50% loss in activity. |
| Y70 | F | 5.42 | _+/-0.54(4) | 14.26 | Interface of subunits; key residue- even very conservative substitution to F causes greater than 80% loss in activity. |
| Y70 | S | 1.26 | _+/-0.16(4) | 3.32 | Interface of subunits; key residue- non-conservative substitution to S causes nearly complete loss in activity. |
| Control activity for this set was 38+/-7 ppm DCSA. | | | | | |

### Example 13

### Additional Variant DMO Polypeptides

Table 30 lists DMO activity data (by LC/MS screen) for 58 variants relative to ddmC_M_6his (the wild type *ddmC* sequence with an N-terminal His tag (SEQ ID NO:154), while Table 31 lists DMO activity data (by LC/MS screen) for 1685 variants relative to ddmC_RLE6his (the wild type *ddmC* sequence with T2S and I123L mutations (SEQ ID NO:23), and a C-terminal 6xHis tag). The numbering of residues is based on the numbering found in SEQ ID NO:2 or SEQ ID NO:3.

**Table 30. Mutants of ddmC_M_6his.**

| row | clone IDs | mutations | mean ppm DCSA |
|---|---|---|---|
| 1 | LIB6174-006-B4 | M1L, L287I | 88.5 |
| 2 | LIB6174-004-B9 | T8S, K300R, V301L, V302L | 20.1 |
| 3 | LIB6174-001-E5, | L287I | 14.0 |
| | LIB6174-001-B5, | | |
| | LIB6174-001-C5, | | |
| | LIB6174-006-A4, | | |
| | LIB6174-001-H5, | | |
| | LIB6174-006-D4, | | |
| | LIB6174-006-H4, | | |
| | LIB6174-001-G5, | | |
| | LIB6174-006-E4, | | |
| | LIB6174-001-D5, | | |
| | LIB6174-006-F4, | | |
| | LIB6174-006-G4 | | |
| 4 | LIB6043-005-C2, | H2R, TBS, I129L | 10.9 |
| | LIB6043-005-F7 | | |
| 5 | LIB6174-004-F8, | T8S | 10.4 |
| | LIB6174-002-B1, | | |
| | LIB6174-006-G5, | | |
| | LIB6174-006-E5, | | |
| | LIB6174-002-G1, | | |
| | LIB6174-002-A1, | | |
| | LIB6174-004-G8, | | |
| | LIB6174-006-H5, | | |
| | LIB6174-004-H8, | | |
| | LIB6174-002-F1, | | |
| | LIB6174-006-F5 | | |
| 6 | LIB6134-019-E7 | T8S, I129L, V206A, K300R, V301L, V302L | 8.1 |
| 7 | LIB6043-008-E3 | V10L, P121T, A122T, L123V, A124P, D125E, P126N, G127S, A128S, L287I | 6.9 |
| 8 | LIB6134-002-B2 | T8S, A122T, P126T, G127S, A128T, I129L, K300R, V301L, V302L | 6.0 |
| 9 | LIB6174-004-H5, | A122D, P126D, G127A, A128T, I129V, A172T | 5.3 |
| | LIB6174-004-F5, | | |
| | LIB6174-004-E5 | | |
| 10 | LIB6174-004-B1, | A122D, P126D, G127A, A128T, I129V, K300I, V301L | 5.2 |
| | LIB6174-004-A1, | | |
| | LIB6174-001-A8 | | |
| 11 | LIB6134-019-B3 | T8S, I129L, A172S, N173T, A174I, K300R, V301L, V302L | 4.9 |
| 12 | LIB6134-018-G3, | T8S, I129L, K300R, V301L, V302L | 4.8 |
| | LIB6134-019-D2, | | |
| | LIB6134-001-B4, | | |
| | LIB6134-019-C3, | | |
| | LIB6134-018-H8, | | |
| | LIB6134-019-F6, | | |
| | LIB6134-018-C6, | | |
| | LIB6174-006-D7, | | |
| | LIB6134-019-B7, | | |
| | LIB6134-017-C10, | | |
| | LIB6134-017-B6, | | |
| | LIB6134-017-F6, | | |
| | LIB6134-019-E4, | | |
| | LIB6134-019-G7, | | |
| | LIB6134-017-A1, | | |
| | LIB6134-018-G5, | | |
| | LIB6134-017-A8, | | |
| | LIB6134-017-C5, | | |
| | LIB6134-019-B6, | | |
| | LIB6134-017-D4, | | |
| | LIB6134-019-C4, | | |
| | LIB6134-002-B5, | | |
| | LIB6134-002-E6, | | |
| | LIB6088-003-H3, | | |
| | LIB6134-019-F1, | | |
| | LIB6134-019-D6, | | |
| | LIB6134-018-A5, | | |
| | LIB6088-003-C8, | | |
| | LIB6134-002-D7, | | |
| | LIB6134-019-F8, | | |
| | LIB6134-019-A6, | | |
| | LIB6134-019-F9, | | |
| | LIB6134-019-G8, | | |
| | LIB6134-001-F9, | | |
| | LIB6134-019-F4, | | |
| | LIB6134-017-C9, | | |
| | LIB6134-019-G10, | | |
| | LIB6134-019-G9, | | |
| | LIB6134-017-F7, | | |
| | LIB6134-017-B4, | | |
| | LIB6134-018-B5, | | |
| | LIB6134-019-B8, | | |
| | LIB6134-018-H6, | | |
| | LIB6134-019-A4, | | |
| | LIB6134-019-G1, | | |
| | LIB6134-018-A3, | | |
| | LIB6134-019-B4, | | |
| | LIB6134-018-E10, | | |
| | LIB6134-002-C8, | | |
| | LIB6134-017-H6, | | |
| | LIB6134-017-D6, | | |
| | LIB6134-017-A3, | | |
| | LIB6134-017-E4, | | |
| | LIB6134-019-G5, | | |
| | LIB6134-018-C5, | | |
| | LIB6134-017-F5, | | |
| | LIB6134-018-H9, | | |
| | LIB6134-019-E3, | | |
| | LIB6088-003-E7, | | |
| | LIB6134-017-A6, | | |
| | LIB6134-001-HS | | |
| 13 | LIB6174-001-A9, | A122D, P126D, G127A, A128T, I129V, R171A | 4.5 |
| | LIB6174-001-G9, | | |
| | LIB6174-001-D9, | | |
| | LIB6174-001-H9, | | |
| | LIB6174-001-C9, | | |
| | LIB6174-001-F9, | | |
| | LIB6174-001-E9, | | |
| | LIB6174-001-B9, | | |
| | LIB6174-004-A3, | | |
| | LIB6174-004-D3 | | |
| 14 | LIB6174-001-B1 | A122D, P126D, G127A, A128T, I129V | 4.3 |
| 15 | LIB6174-001-G2, | I129L | 4.2 |
| | LIB6174-001-D2, | | |
| | LIB6174-001-D3, | | |
| | LIB6174-001-F2, | | |
| | LIB6174-006-F2, | | |
| | LIB6174-001-E3, | | |
| | LIB6174-001-A3, | | |
| | LIB6174-001-G3, | | |
| | LIB6174-001-H2, | | |
| | LIB6174-001-E2 | | |
| 16 | LIB6174-004-G5 | A122D, P126D, G127A, A128T, I129V, A139S, A172T | 3.8 |
| 17 | LIB6134-017-G8, | T8S, I129L, R171P, K300R, V301L, V302L | 3.4 |
| | LIB6134-019-F5, | | |
| | LIB6134-018-G7 | | |
| 18 | LIB6134-019-C8 | T8S, I129L, R171A, A174S, K300R, V301L, V302L | 3.4 |
| 19 | LIB6043-010-D3 | V10L, P121S, A122T, L123I, D125H, P126H, G127A, A128G, I129V, L287I | 3.3 |
| 20 | LIB6043-010-A2 | V10L, P121S, A122K, L123F, A124S, D125H, P126N, G127S, A128S, I129F, L287I | 2.5 |
| 21 | LIB6043-010-H9 | V10L, P121T, A124T, P126H, G127S, A128G, I129V, L287I | 2.4 |
| 22 | LIB6174-004-B10 | V10L, R171A, A172G, N173G, D177E, A178I, F179D, E192D | 2.3 |
| 23 | LIB6043-003-H7 | T8S, P60Q, I129L | 2.3 |
| 24 | LIB6134-001-G10 | T8S, P121A, L123F, A124S, D125H, P126S, G127T, A128T, I129V, K300R, V301L, V302L | 2.1 |
| 25 | LIB6043-010-E3 | V10L, P121S, A122K, L123I, A124S, D125H, P126H, G127T, A128G, I129L, L287I | 2.1 |
| 26 | LIB6043-003-E3, | T8S, I129L | 2.1 |
| | LIB6043-001-E10, | | |
| | LIB6043-003-A5, | | |
| | LIB6043-007-B2, | | |
| | LIB6043-003-C2, | | |
| | LIB6043-003-C5, | | |
| | LIB6043-011-G3, | | |
| | LIB6043-O11-H8, | | |
| | LIB6043-014-E10, | | |
| | LIB6043-009-D7, | | |
| | LIB6043-007-E4, | | |
| | LIB6043-003-G8, | | |
| | LIB6043-007-C8, | | |
| | LIB6043-009-E9, | | |
| | LIB6043-001-F10, | | |
| | LIB6043-010-F10, | | |
| | LIB6043-003-F3, | | |
| | LIB6043-003-D8, | | |
| | LIB6043-003-G3, | | |
| | LIB6043-004-F10, | | |
| | LIB6043-003-B5, | | |
| | LIB6043-011-A8, | | |
| | LIB6043-005-F10, | | |
| | LIB6043-009-F10, | | |
| | LIB6043-009-G5, | | |
| | LIB6043-009-A1, | | |
| | LIB6088-009-G6, | | |
| | LIB6043-011-G2, | | |
| | LIB6088-003-D3, | | |
| | LIB6043-003-C8, | | |
| | LIB6043-002-E10, | | |
| | LIB6043-003-G5, | | |
| | LIB6088-005-C3, | | |
| | LIB6043-003-H4, | | |
| | LIB6043-009-D8, | | |
| | LIB6043-006-F10, | | |
| | LIB6043-007-E8, | | |
| | LIB6043-009-E6, | | |
| | LIB6043-009-F6, | | |
| | LIB6083-005-F10, | | |
| | LIB6088-009-G3, | | |
| | LIB6043-003-E2, | | |
| | LIB6083-005-E10, | | |
| | LIB6083-008-E10, | | |
| | LIB6043-009-D5, | | |
| | LIB6043-007-A3, | | |
| | LIB6043-003-B1, | | |
| | LIB6043-005-G8, | | |
| | LIB6043-006-E10, | | |
| | LIB6088-009-A3, | | |
| | LIB6043-009-F9, | | |
| | LIB6043-003-F2, | | |
| | LIB6088-009-B3, | | |
| | LIB6043-003-G4, | | |
| | LIB6043-003-C9, | | |
| | LIB6043-009-A9, | | |
| | LIB6088-009-E5, | | |
| | LIB6043-003-B8, | | |
| | LIB6043-007-F10, | | |
| | LIB6043-011-F10, | | |
| | LIB6043-009-A6, | | |
| | LIB6043-009-C10, | | |
| | LIB6043-003-F6, | | |
| | LIB6043-009-E7, | | |
| | LIB6043-011-D7, | | |
| | LIB6043-010-E10, | | |
| | LIB6043-011-G1, | | |
| | LIB6088-007-G2, | | |
| | LIB6043-007-E10, | | |
| | LIB6043-003-H1, | | |
| | LIB6043-011-E9, | | |
| | LIB6043-003-E1, | | |
| | LIB6043-007-G6, | | |
| | LIB6043-009-F1, | | |
| | LIB6043-009-B7, | | |
| | LIB6088-003-A7, | | |
| | LIB6043-009-B2, | | |
| | LIB6043-011-E10, | | |
| | LIB6043-011-F6, | | |
| | LIB6043-007-A1, | | |
| | LIB6043-004-E10, | | |
| | LIB6043-003-A10, | | |
| | LIB6043-003-D1, | | |
| | LIB6088-009-H4, | | |
| | LIB6043-003-D10, | | |
| | LIB6043-003-D4, | | |
| | LIB6083-006-E10, | | |
| | LIB6043-003-E4, | | |
| | LIB6043-003-G7, | | |
| | LIB6043-009-E4, | | |
| | LIB6043-003-F5, | | |
| | LIB6043-003-C6, | | |
| | LIB6043-007-B6, | | |
| | LIB6043-003-A2, | | |
| | LIB6043-005-E10, | | |
| | LIB6043-003-E9, | | |
| | LIB6043-003-H5, | | |
| | LIB6043-011-C1, | | |
| | LIB6043-009-B4, | | |
| | LIB6088-009-F6, | | |
| | LIB6043-003-F4, | | |
| | LIB6043-007-G7, | | |
| | LIB6043-003-G2, | | |
| | LIB6043-003-H8, | | |
| | LIB6043-007-D6, | | |
| | LIB6043-003-F9, | | |
| | LIB6043-007-A9, | | |
| | LIB6043-011-G4, | | |
| | LIB6043-003-C4, | | |
| | LIB6083-006-F10, | | |
| | LIB6043-009-B8, | | |
| | LIB6043-003-C7, | | |
| | LIB6043-005-D8, | | |
| | LIB6043-003-E5, | | |
| | LIB6043-003-F8 | | |
| 27 | LIB6174-004-B6, | A122D, P126D, G127A, A128T, I129V, N173T, A174L, | 2.1 |
| | LIB6174-004-A6, | Q175R, T176S, A178S, F179Y | |
| | LIB6174-004-C6, | | |
| | LIB6174-001-G10, | | |
| | LIB6174-004-D6 | | |
| 28 | LIB6174-001-F3 | I129L, D158N | 2.0 |
| 29 | LIB6174-004-G3, | A122D, P126D, G127A, A128T, I129V, A172T, N173D | 2.0 |
| | LIB6174-001-A10, | | |
| | LIB6174-004-H3, | | |
| | LIB6174-001-C10, | | |
| | LIB6174-001-B10 | | |
| 30 | LIB6043-010-G3 | V10L, P121T, A122S, L123V, A124P, D125E, P126T, | 2.0 |
| | | G127T, I129L, L287I | |
| 31 | LIB6043-010-B7 | V10L, A122K, A124P, G127S, I129V, L287I | 1.9 |
| 32 | LIB6043-010-D1 | V10L, A122E, A124P, D125H, P126Y, G127A, A128T, L287I | 1.9 |
| 33 | LIB6043-010-H5 | V10L, P121S, A122S, A124S, D125Q, G127A, I129V, L287I | 1.9 |
| 34 | LIB6043-010-F8 | V10L, P121S, A122E, L123V, D125E, P126Y, G127S, I129L,L287I | 1.9 |
| 35 | LIB6043-010-C4 | V10L, P121A, A122K, A124T, D125Q, P126N, A128S, I129F, L287I | 1.9 |
| 36 | LIB6174-001-G8, | A122D, P126D, G127A, A128T, I129V, N173T, A174I, | 1.8 |
| | LIB6174-001-C8, | Q175R | |
| | LIB6174-004-H2, | | |
| | LIB6174-004-G2, | | |
| | LIB6174-001-F8, | | |
| | LIB6174-001-E8, | | |
| | LIB6174-004-F2, | | |
| | LIB6174-001-D8 | | |
| 37 | LIB6043-010-E8 | V10L, P121A, A122E, L123V, P126Y, A128G, L287I | 1.8 |
| 38 | LIB6174-001-A2, LIB6174-001-B2 | P121A, A122R, L123F, A128G | 1.8 |
| 39 | LIB6134-001-B5 | T8S, P121T, A122R, D125H, P126T, G127S, K300R, V301L, V302L | 1.8 |
| 40 | LIB6174-001-F10, | A122D, P126D, G127A, A128T, I129V, A172T, N173D, | 1.7 |
| | LIB6174-001-D10, | A174V, Q175E, T176S | |
| | LIB6174-004-F4, | | |
| | LIB6174-004-E4, | | |
| | LIB6174-001-D7, | | |
| | LIB6174-004-H4 | | |
| 41 | LIB6043-010-F9 | V10L, P121S, A122D, L123F, A124S, D125E, P126D, A128T, I129V, L287I | 1.6 |
| 42 | LIB6134-002-F6 | T8S, A122D, L123F, D125E, P126T, G127S, A128G, K300R, V301L, V302L | 1.5 |
| 43 | LIB6174-001-D6, | A122D, P126D, G127A, A128T, I129V, E298D, V301L, | 1.5 |
| | LIB6174-001-H6, | V302M | |
| | LIB6174-001-A6, | | |
| | LIB6174-001-C6, | | |
| | LIB6174-001-F6, | | |
| | LIB6174-001-E6 | | |
| 44 | LIB6043-003-D9 | T8S, F105Y, I129L | 1.5 |
| 45 | LIB6174-001-G1, | A122G, P126A, G127A, A128T, I129L | 1.5 |
| | LIB6174-001-E1, | | |
| | LIB6174-001-H1, | | |
| | LIB6174-001-F1 | | |
| 46 | LIB6083-004-E2 | V10L, R171A, A172T, N173D, A174L, Q175G, T176N, D177E, A178S, L287I | 1.5 |
| 47 | LIB6172-001-G4, | T8S, A122G, P126A, G127A, A128T, I129L, K300R, | 1.5 |
| | LIB6172-001-F4, | V301L, V302L | |
| | LIB6172-001-E4 | | |
| 48 | LIB6134-019-G4 | T8S, I129L, R171P, A174I, Q175R, F179D, K300R, V301L, V302L | 1.5 |
| 49 | LIB6172-001-C8, | T8S, P121A, A122R, L123F, A128G, K300R, V301L, | 1.4 |
| | LIB6172-001-D8, | V302L | |
| | LIB6172-002-H3, | | |
| | LIB6172-001-B8, | | |
| | LIB6172-001-A8 | | |
| 50 | LIB6043-007-G9, | T8S, A122G, P126A, G127A, A128T, I129L | 1.4 |
| | LIB6174-006-B7, | | |
| | LIB6172-001-B10, | | |
| | LIB6043-011-H1 | | |
| 51 | LIB6103-004-G4, | T8S, A122D, P126D, G127A, A128T, I129V, R171A | 1.4 |
| | LIB6103-002-G9, | | |
| | LIB6103-004-F6 | | |
| 52 | LIB6134-001-H9 | T8S, P121T, A122T, L123I, P126Y, G127S, A128S, K300R, V301L, V302L | 1.3 |
| 53 | LIB6083-004-F2 | V10L, A172T, N173A, A174T, T176S, F179V, L287I | 1.3 |
| 54 | LIB6174-001-B8, | A122D, P126D, G127A, A128T, I129V, A172T, N173T, | 1.2 |
| | LIB6174-001-H8 | A174I, Q175R | |
| 55 | LIB6083-004-H5 | V10L, A172S, A174L, Q175G, F179D, L287I | 1.2 |
| 56 | LIB6088-010-H4, | V10L, L287I | 1.2 |
| | LIB6088-010-D3, | | |
| | LIB6088-010-G9, | | |
| | LIB6088-010-G2, | | |
| | LIB6088-010-B7, | | |
| | LIB6043-010-G9, | | |
| | LIB6088-010-E10, | | |
| | LIB6088-010-E5, | | |
| | LIB6088-008-G7, | | |
| | LIB6088-008-B4, | | |
| | LIB6088-010-C9, | | |
| | LIB6088-006-F7, | | |
| | LIB6088-O10-H7, | | |
| | LIB6088-010-F4, | | |
| | LIB6043-008-D4, | | |
| | LIB6088-010-D7, | | |
| | LIB6043-008-H2, | | |
| | LIB6088-010-E8, | | |
| | LIB6088-010-G3, | | |
| | LIB6088-010-B6, | | |
| | LIB6088-010-C3, | | |
| | LIB6088-010-D10, | | |
| | LIB6088-010-H9, | | |
| | LIB6088-008-D10, | | |
| | LIB6088-010-F8, | | |
| | LIB6088-010-HIO, | | |
| | LIB6088-010-G10, | | |
| | LIB6043-008-B2, | | |
| | LIB6088-010-B5, | | |
| | LIB6088-008-B5, | | |
| | LIB6088-010-D9, | | |
| | LIB6088-004-C9, | | |
| | LIB6088-010-D6, | | |
| | LIB6088-010-A7, | | |
| | LIB6043-008-B7, | | |
| | LIB6088-010-F5, | | |
| | LIB6088-010-D8, | | |
| | LIB6088-O10-C4, | | |
| | LIB6043-008-A10, | | |
| | LIB6043-006-H10, | | |
| | LIB6088-O10-E6, | | |
| | LIB6043-008-D7, | | |
| | LIB6088-010-F6, | | |
| | LIB6043-008-E1, | | |
| | LIB6088-010-F9, | | |
| | LIB6043-014-G7, | | |
| | LIB6088-010-F2, | | |
| | LIB6088-010-H1, | | |
| | LIB6043-010-A1, | | |
| | LIB6088-010-D5, | | |
| | LIB6088-010-G7, | | |
| | LIB6043-008-G9, | | |
| | LIB6043-010-C5, | | |
| | LIB6088-010-C7, | | |
| | LIB6088-010-E9, | | |
| | LIB6088-008-F10, | | |
| | LIB6043-008-C2, | | |
| | LIB6043-003-H10, | | |
| | LIB6088-010-D4, | | |
| | LIB6088-O10-F7, | | |
| | LIB6088-010-C8, | | |
| | LIB6043-008-E7, | | |
| | LIB6088-008-A8, | | |
| | LIB6088-008-G4, | | |
| | LIB6088-008-H9, | | |
| | LIB6088-010-B8, | | |
| | LIB6088-010-G5, | | |
| | LIB6088-008-E6, | | |
| | LIB6088-010-A10, | | |
| | LIB6043-006-G4, | | |
| | LIB6088-008-B9, | | |
| | LIB6088-010-G6 | | |
| 57 | LIB6103-008-G5 | TBS, A122D, P126D, G127A, A128T, I129V, A172T | 1.1 |
| 58 | LIB6174-006-F3, | I129L, E298D, V302M, V303I, A305S | 1.1 |
| | LIB6174-006-C3 | | |

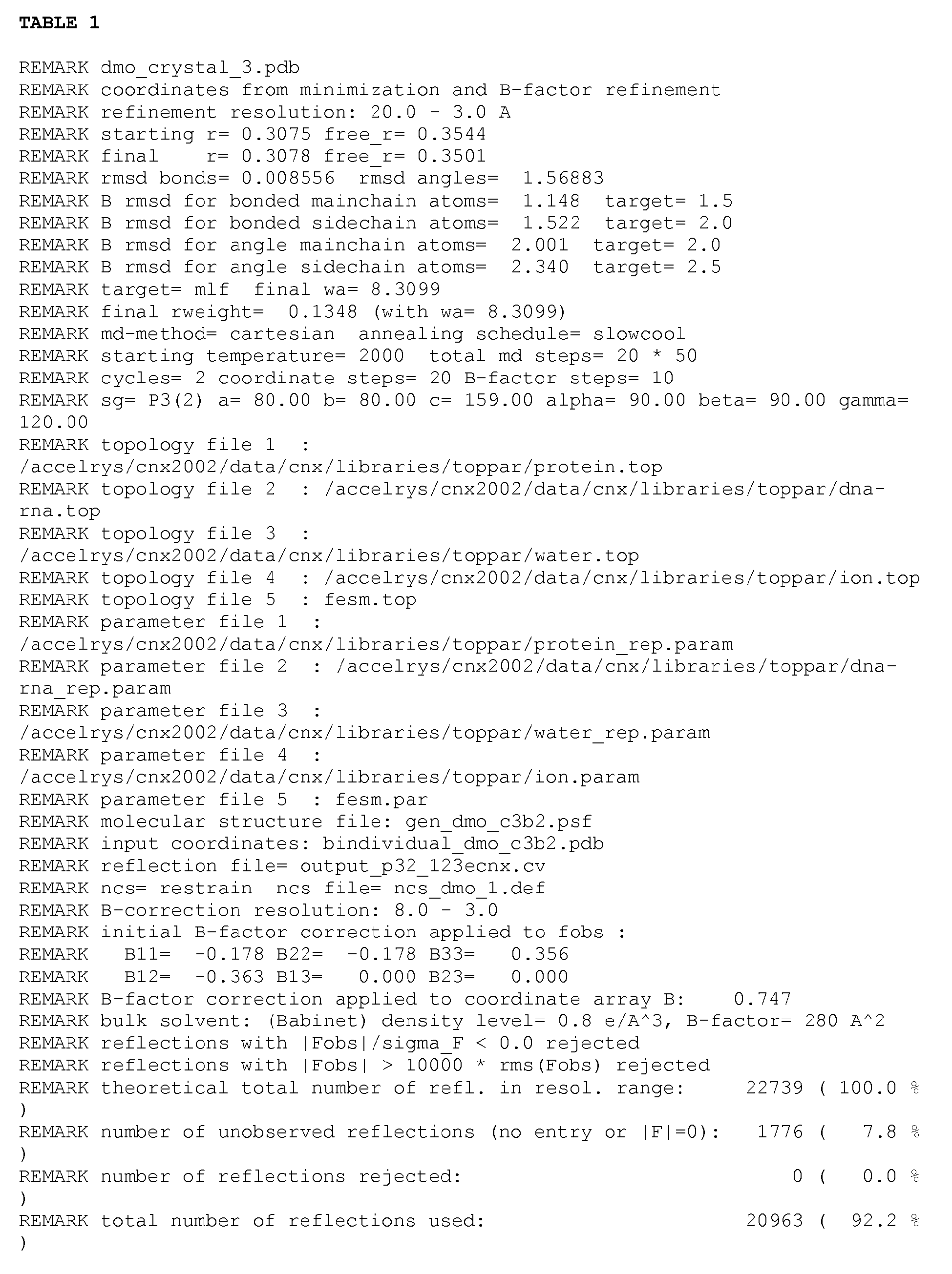

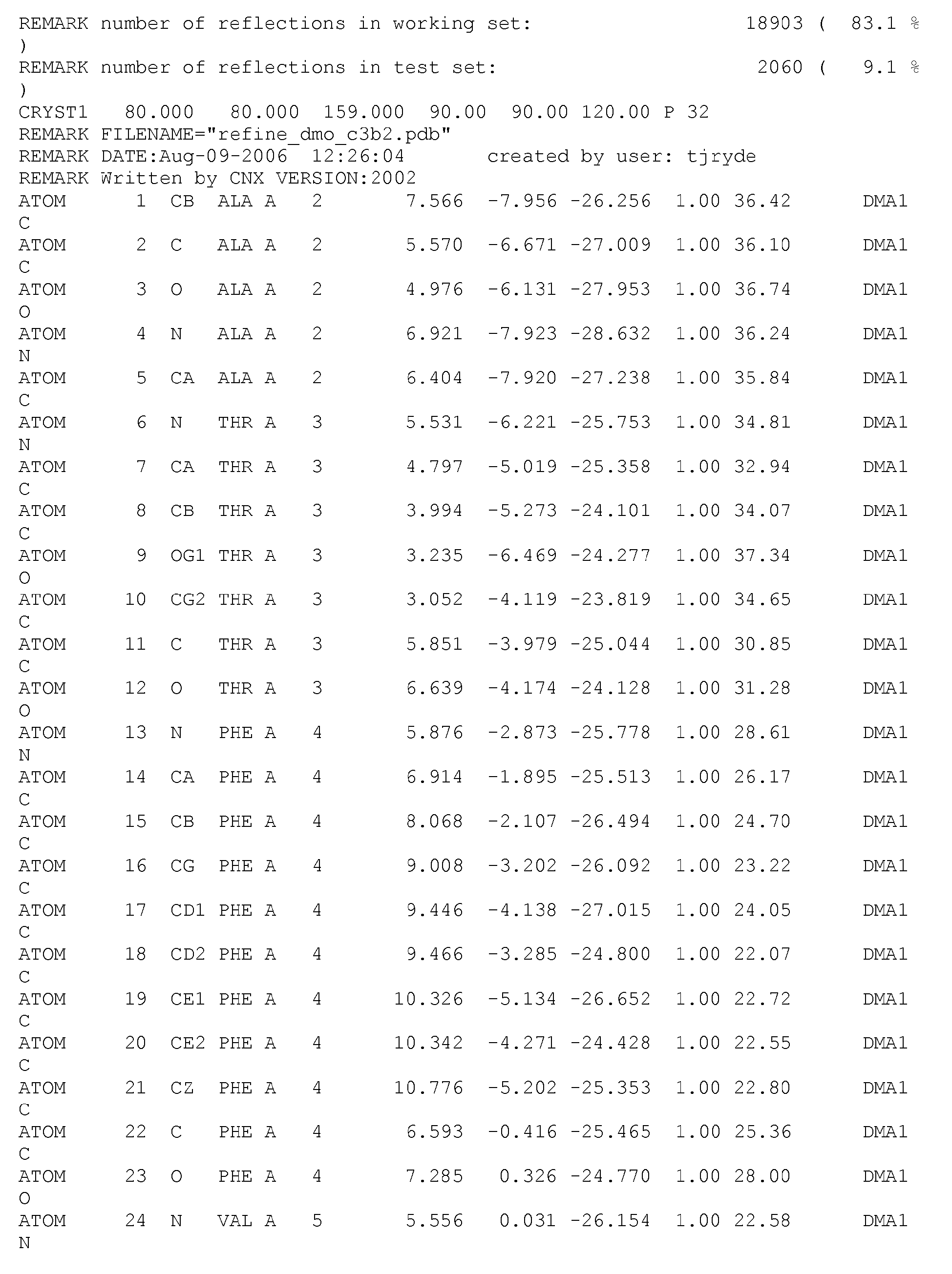

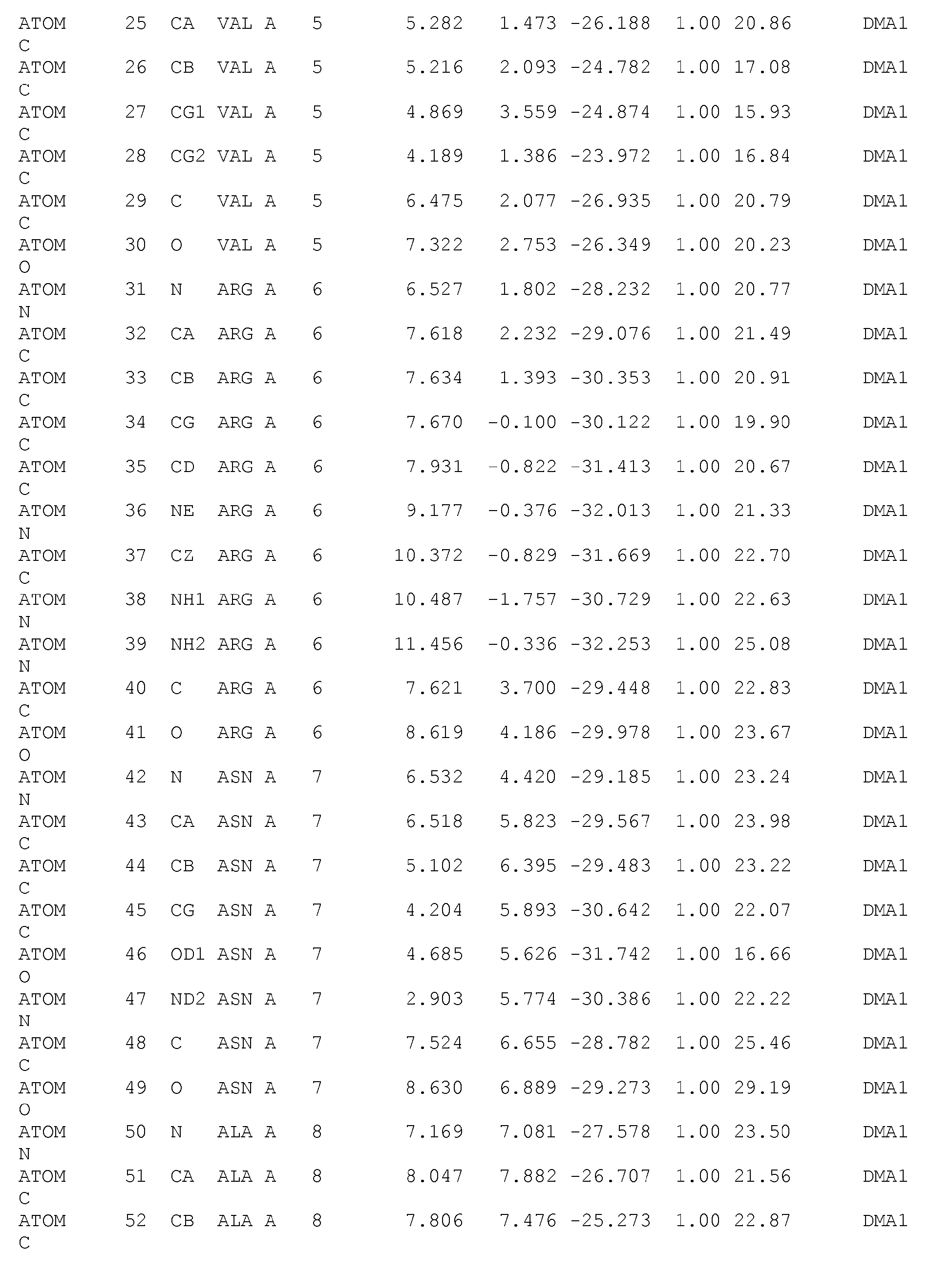

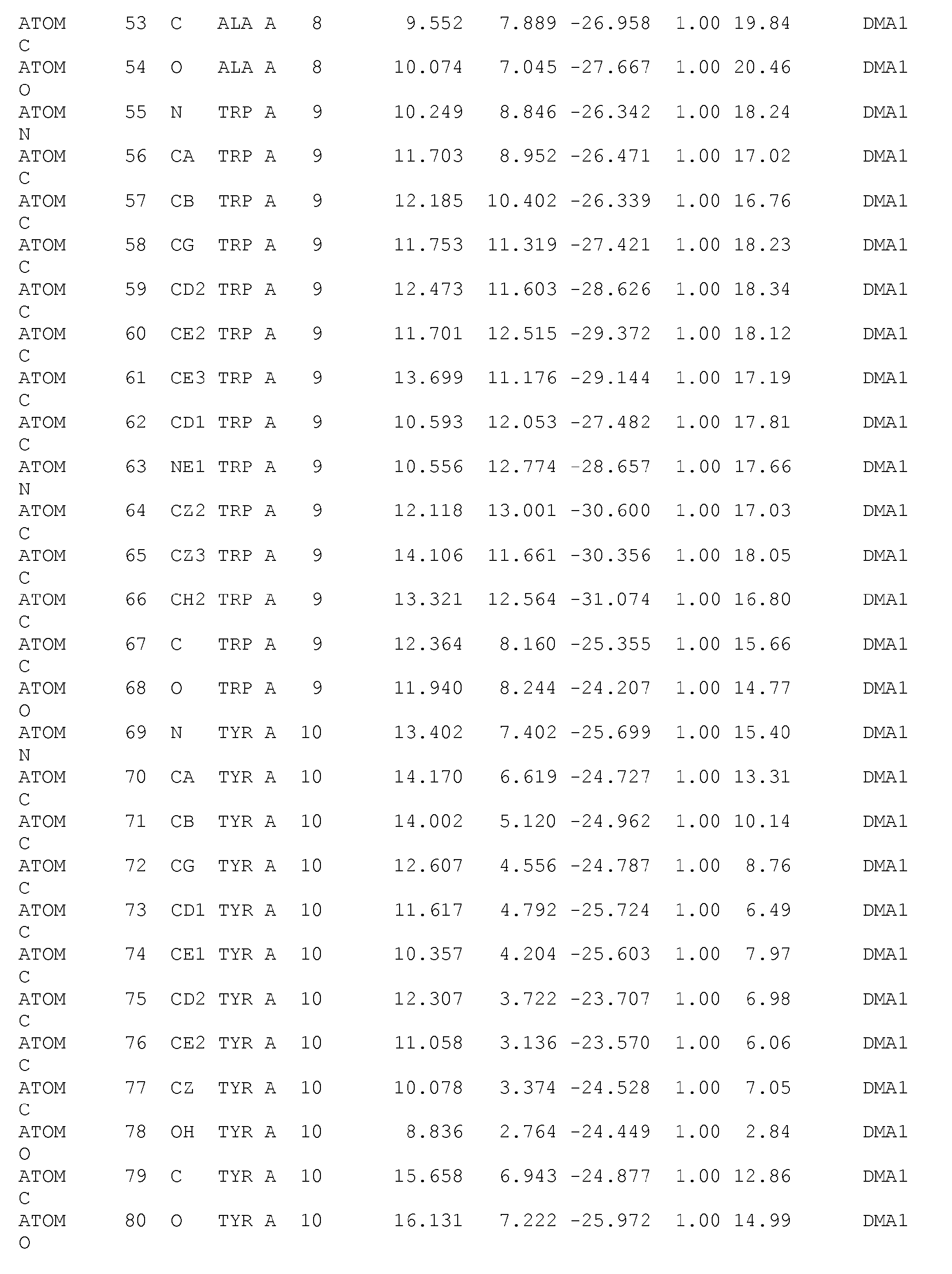

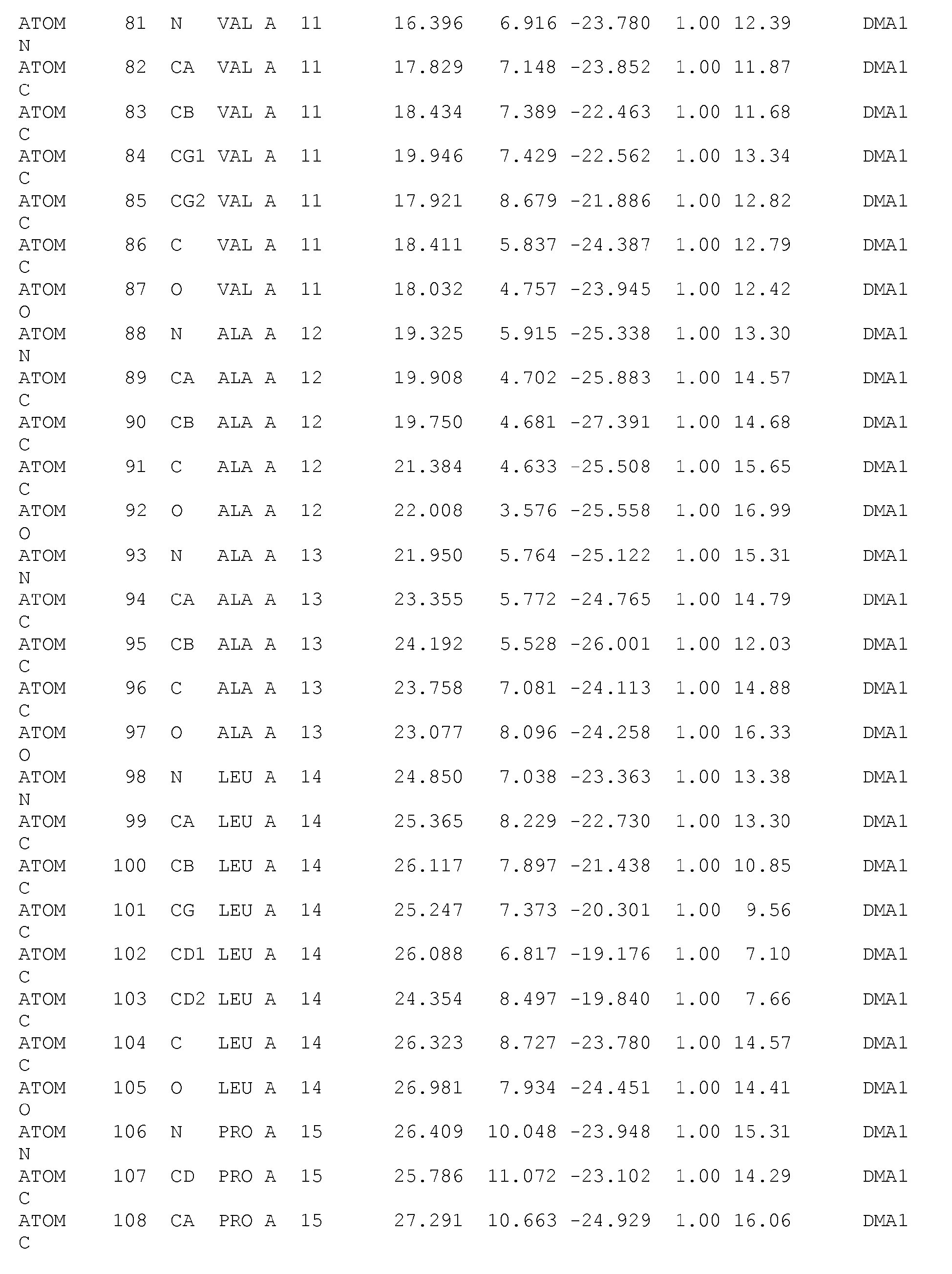

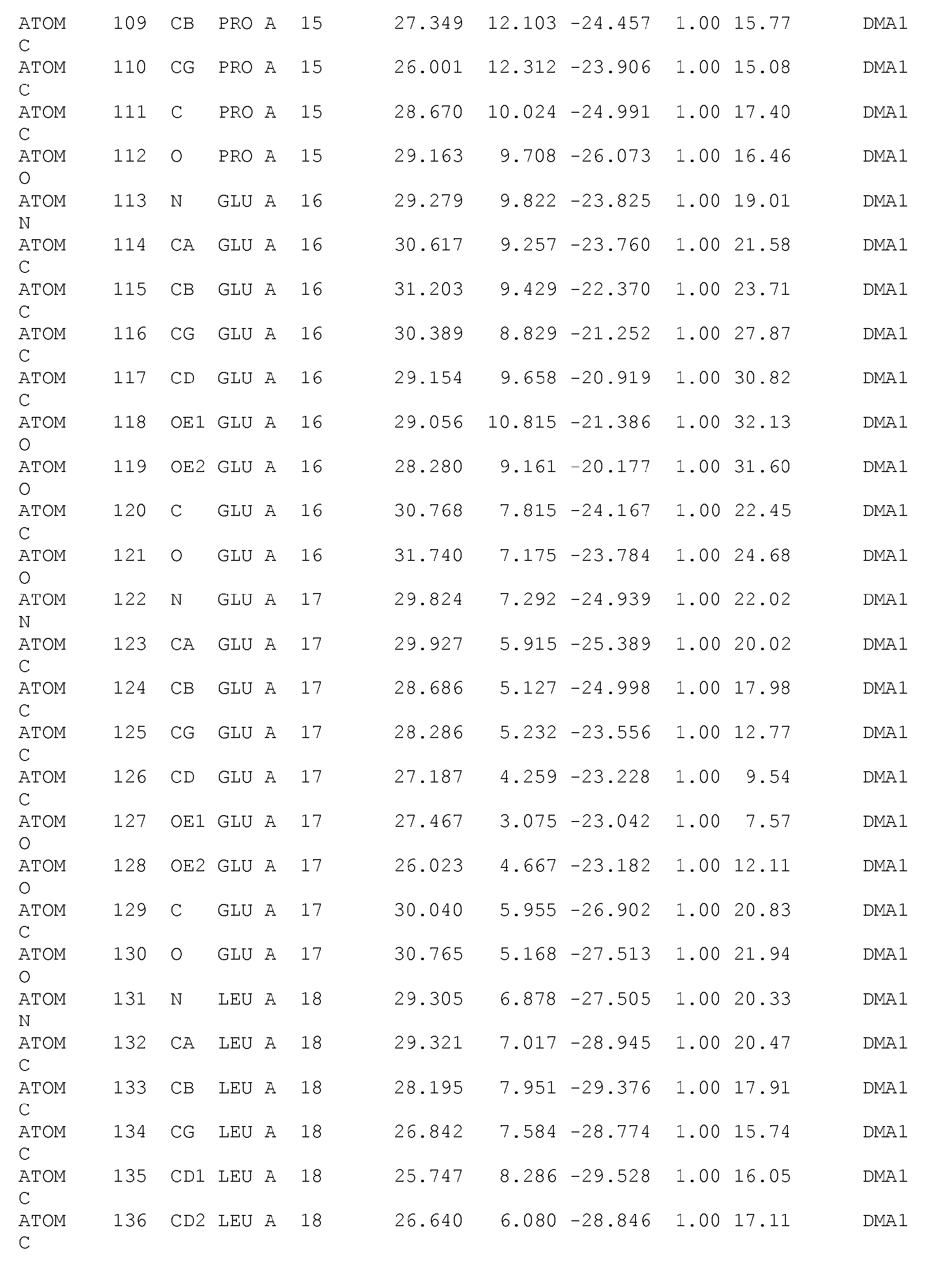

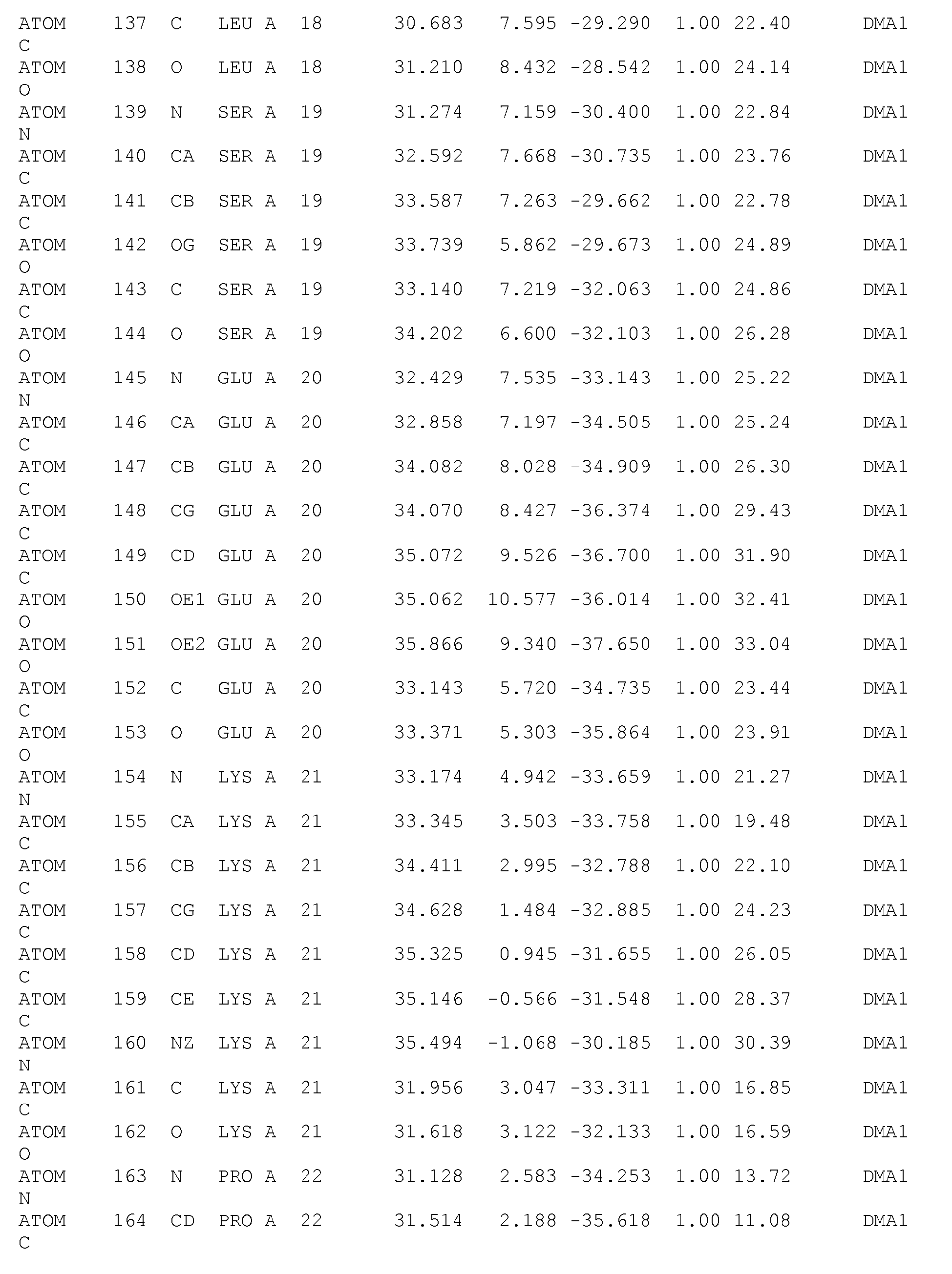

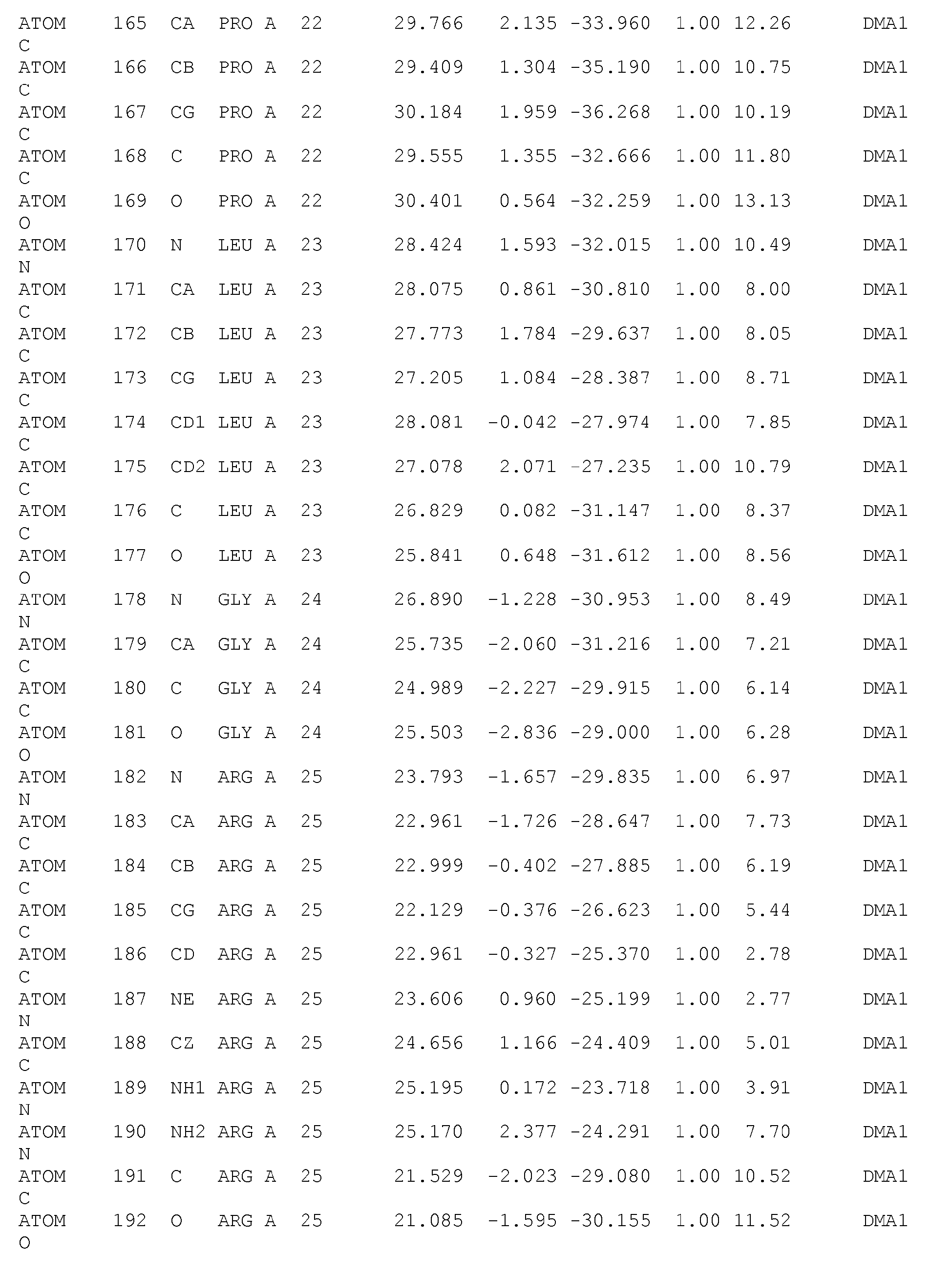

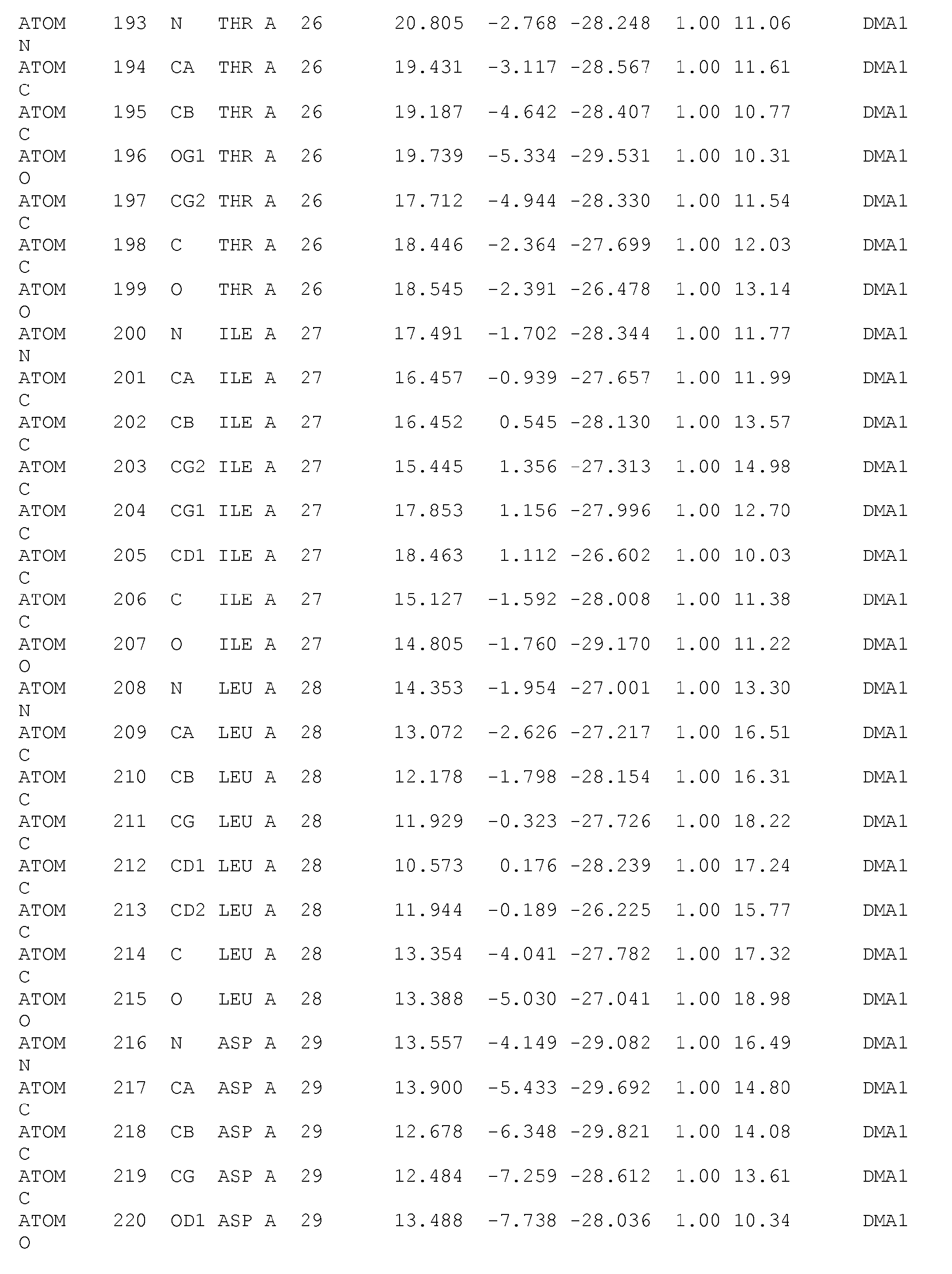

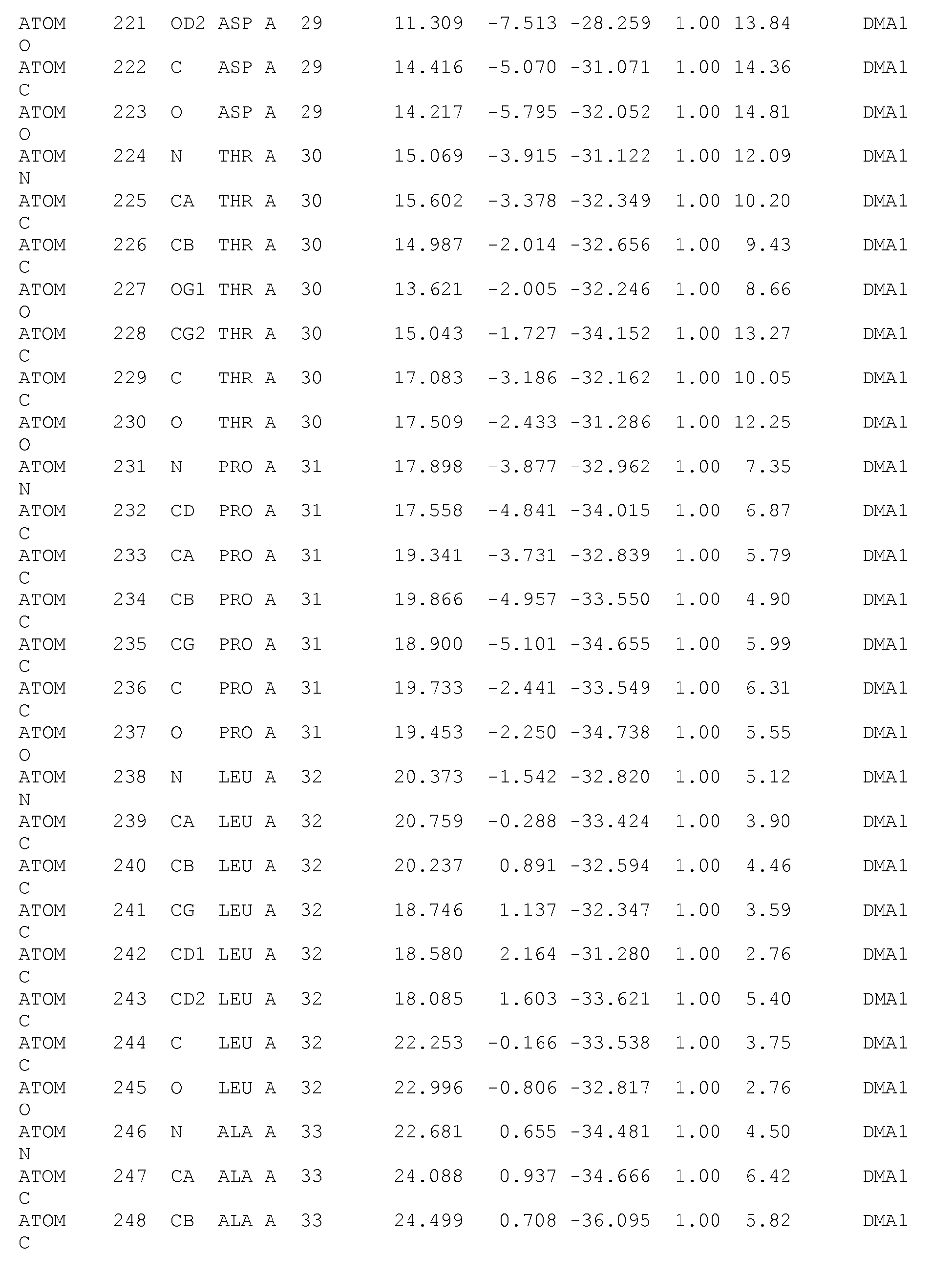

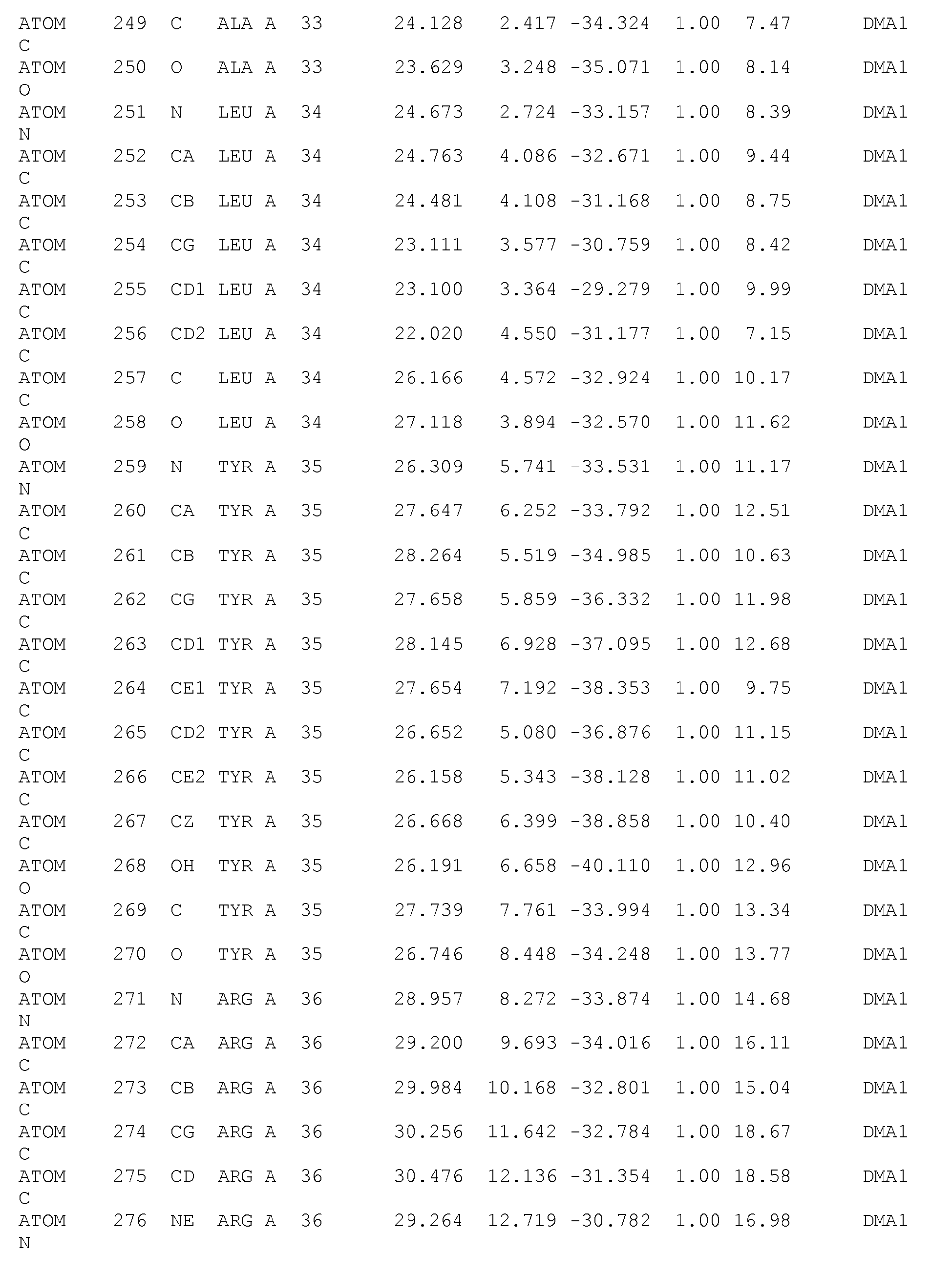

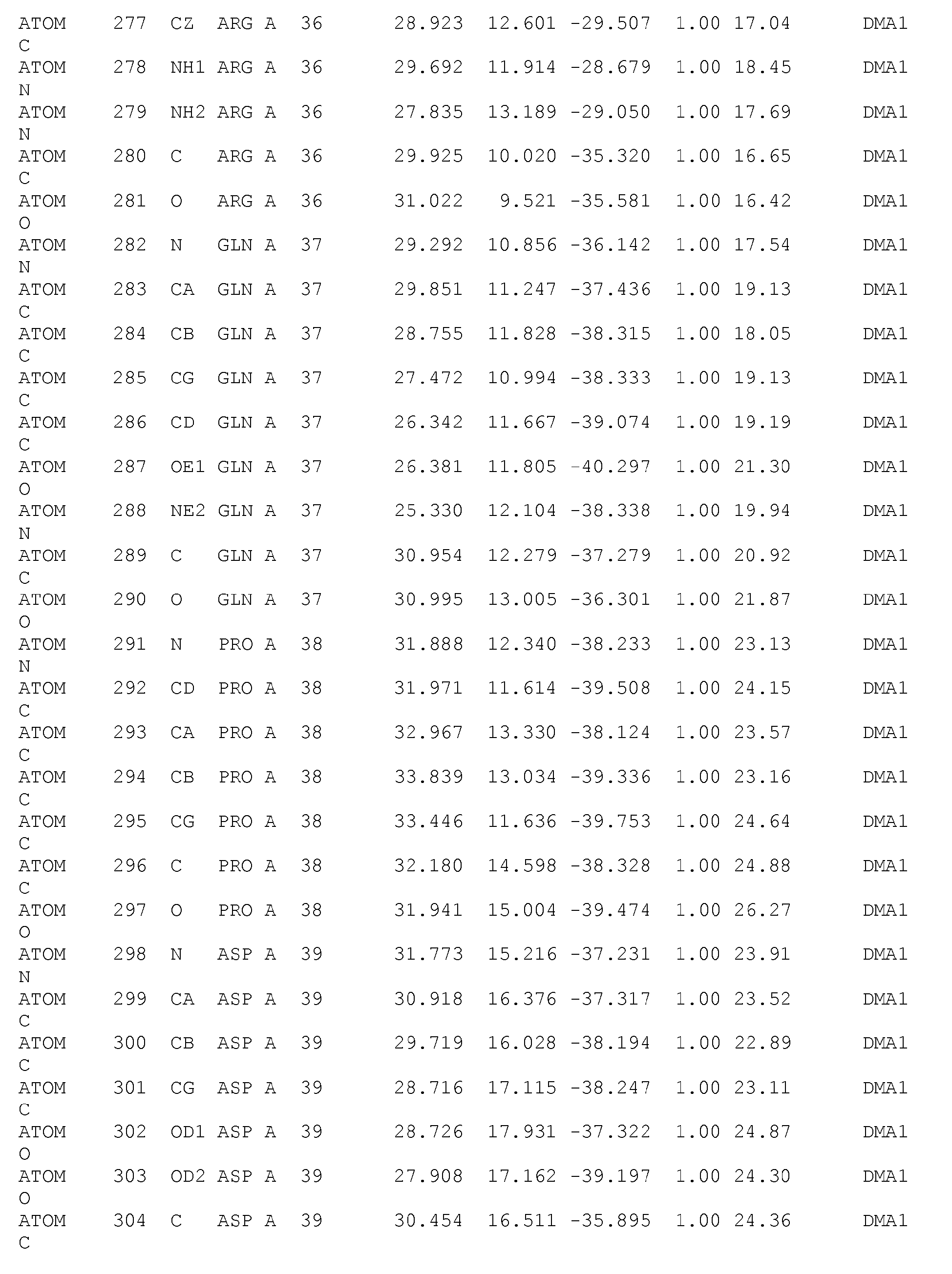

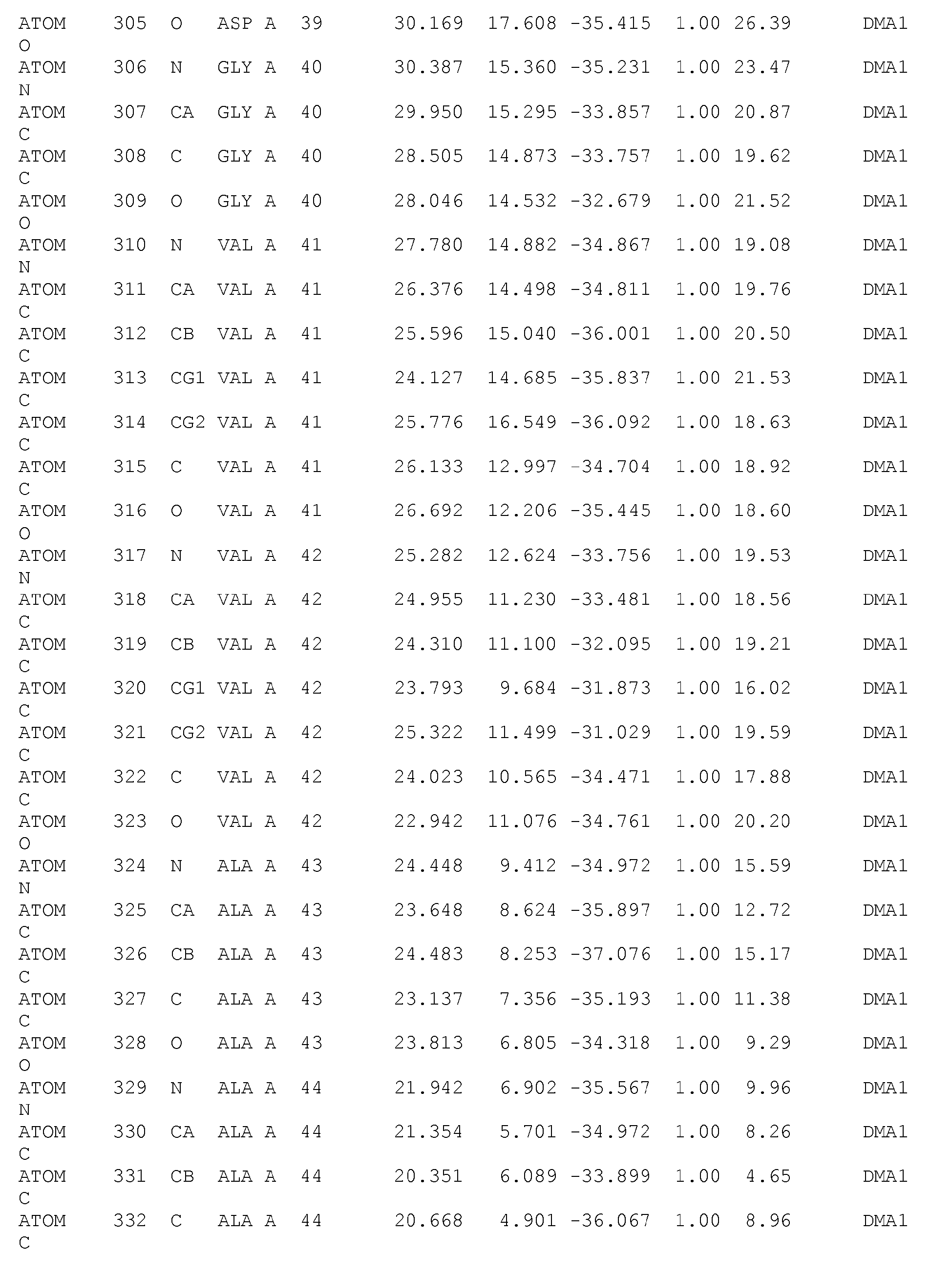

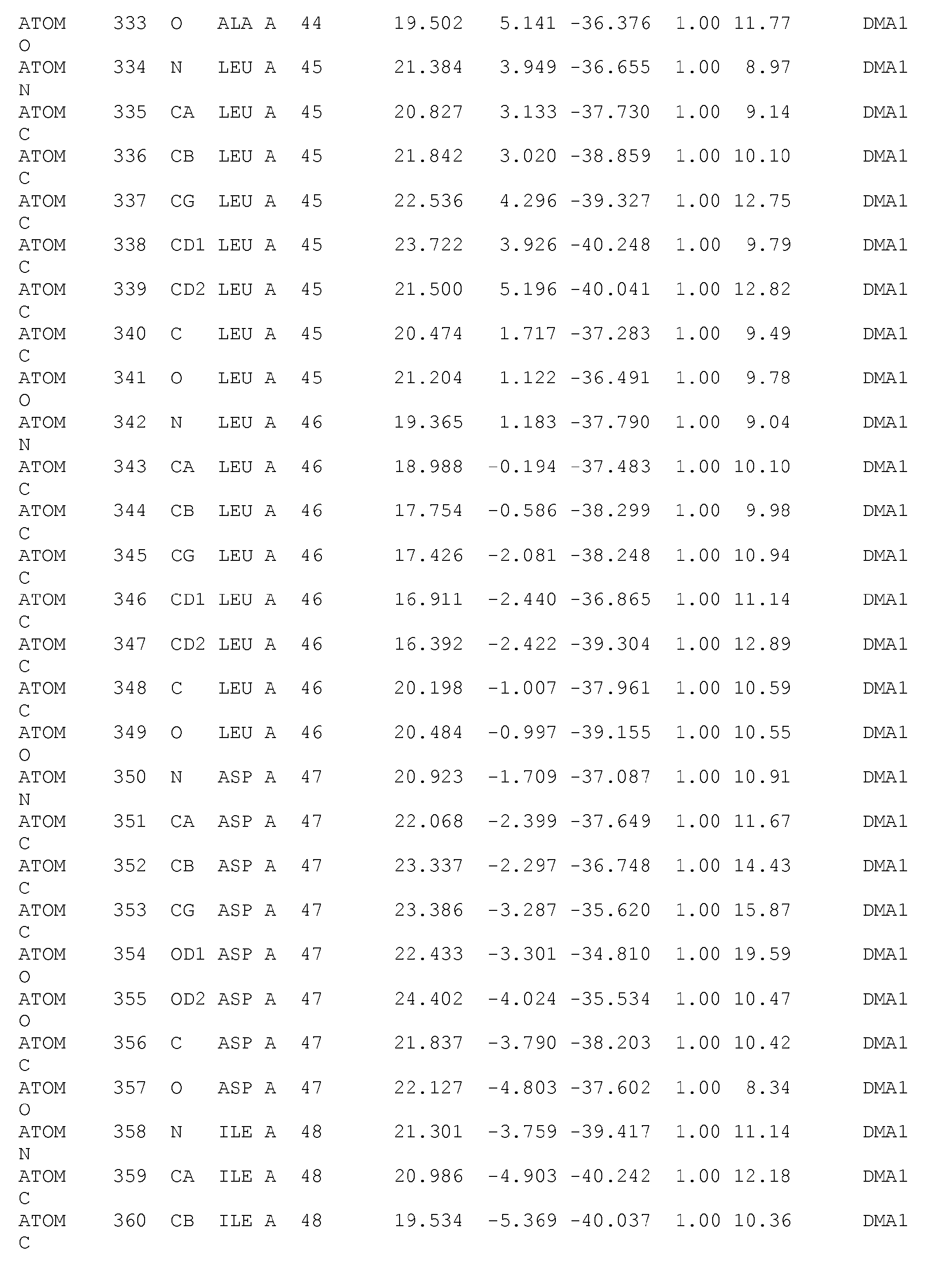

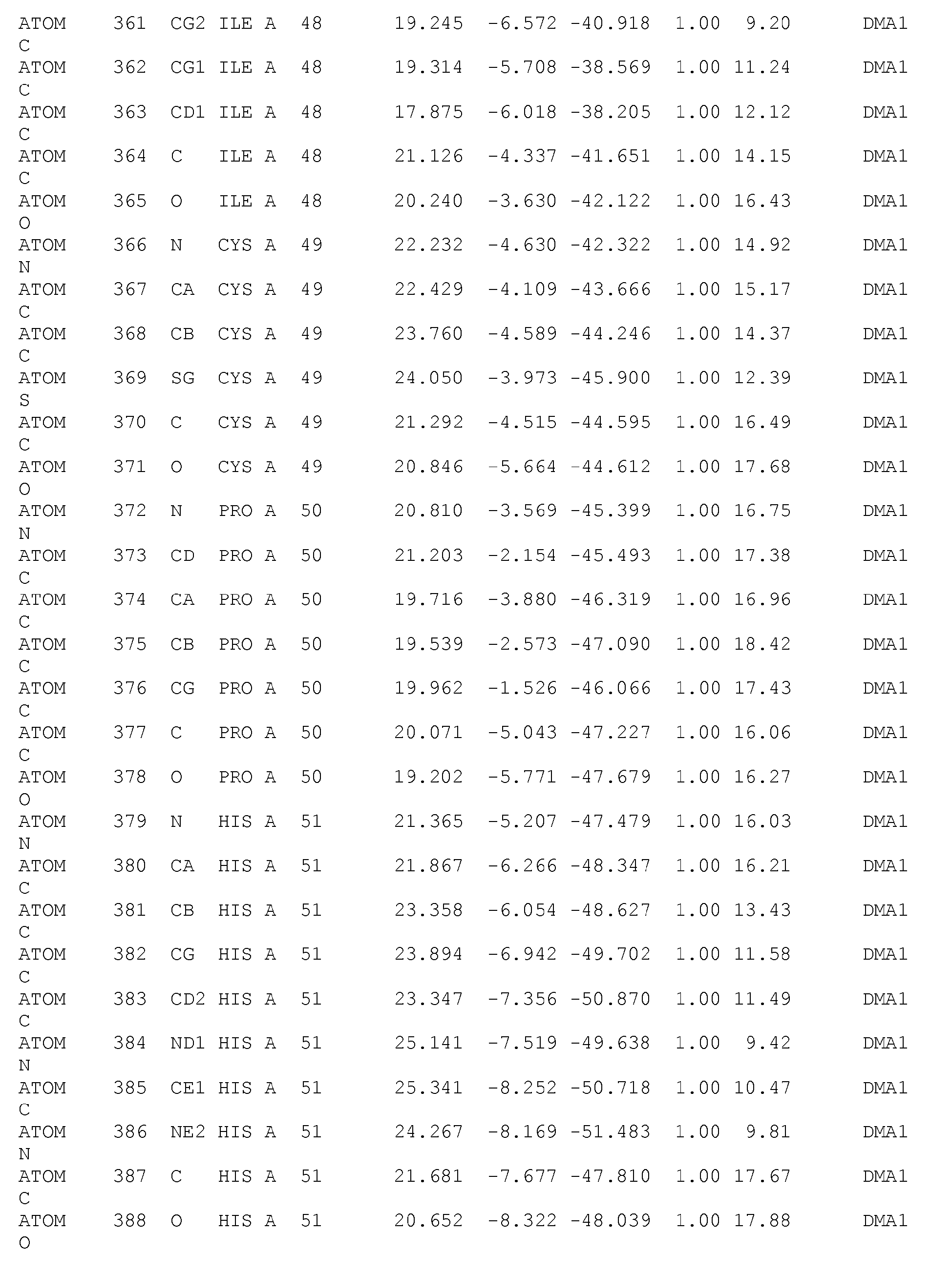

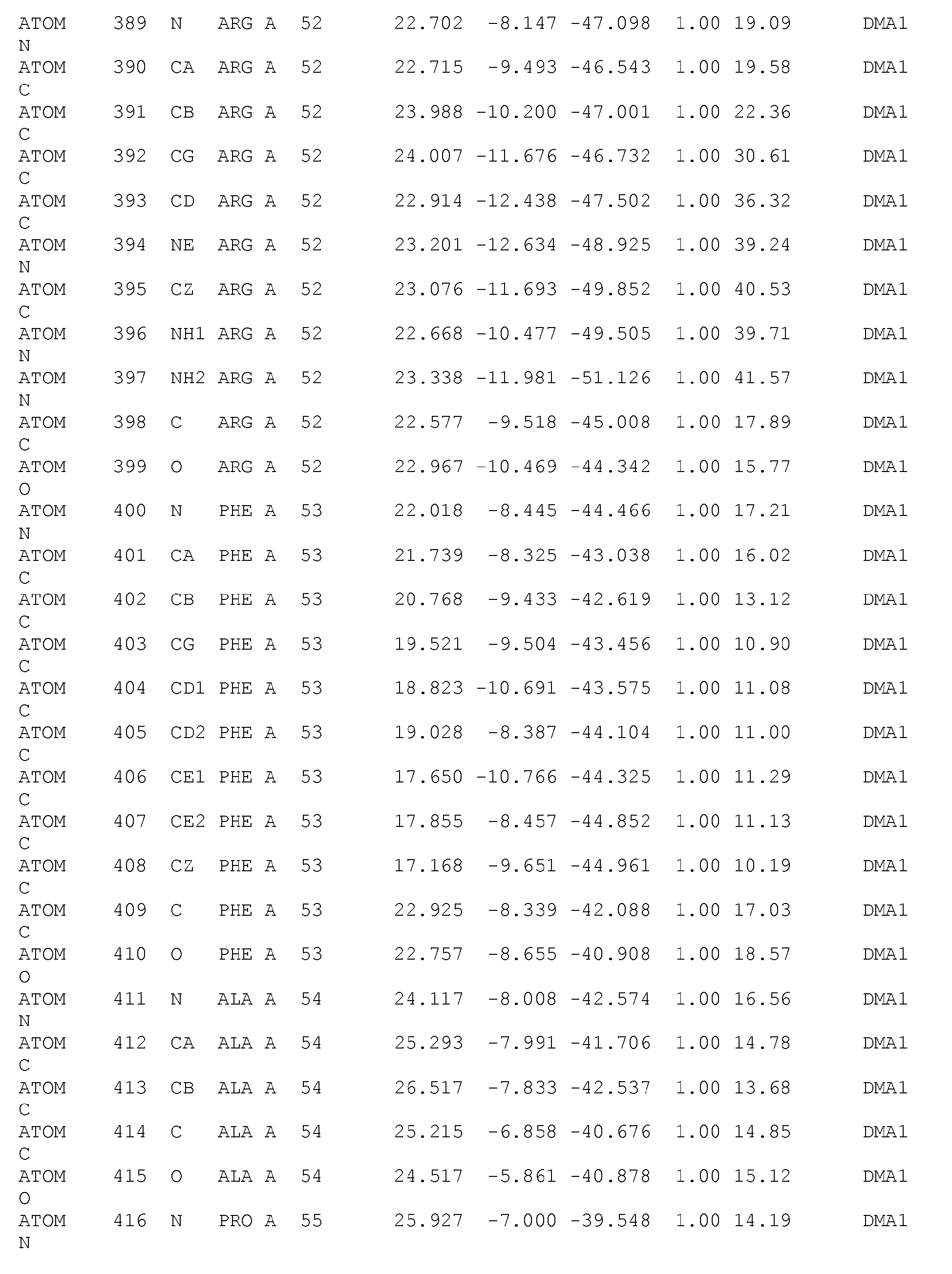

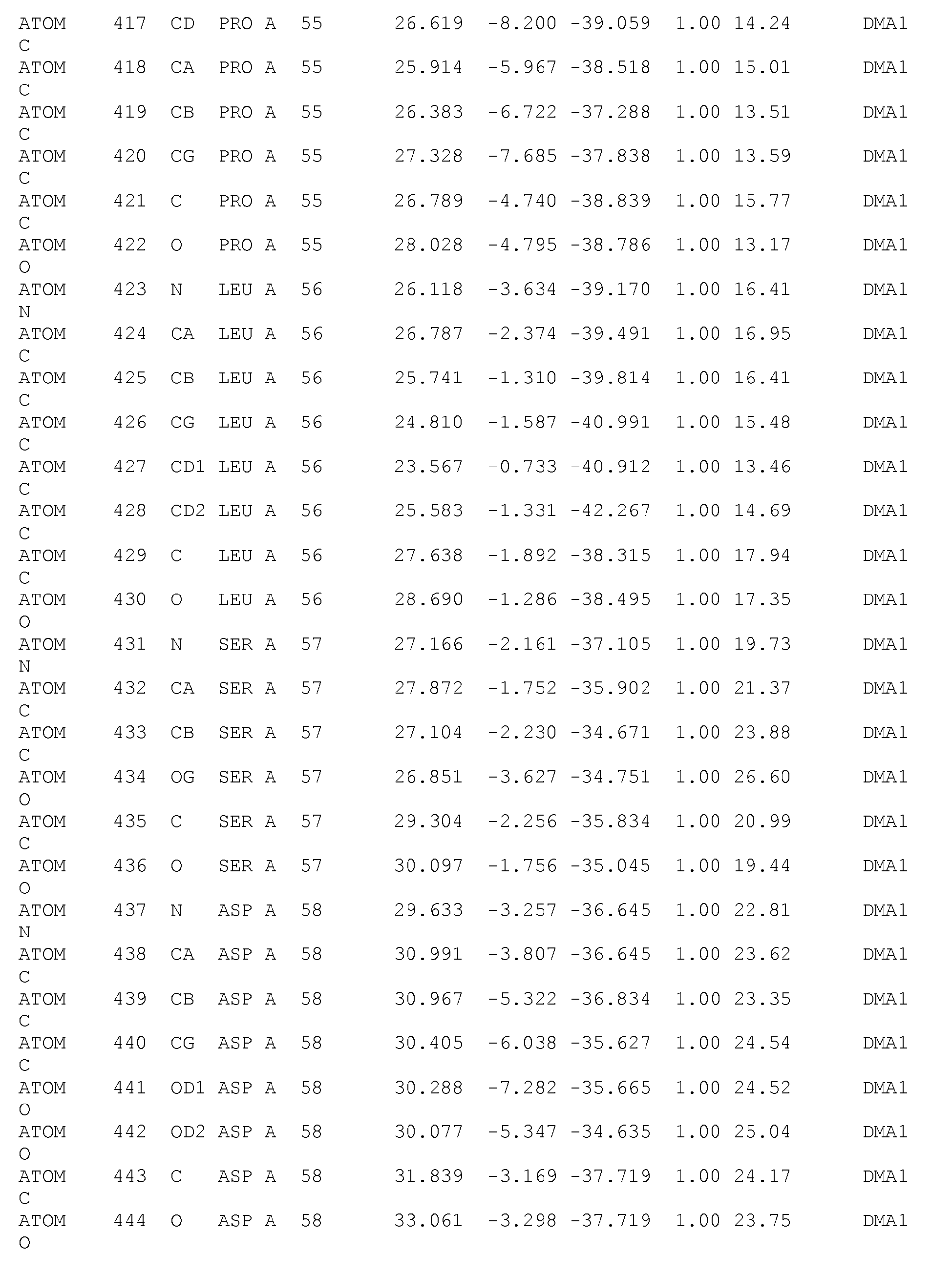

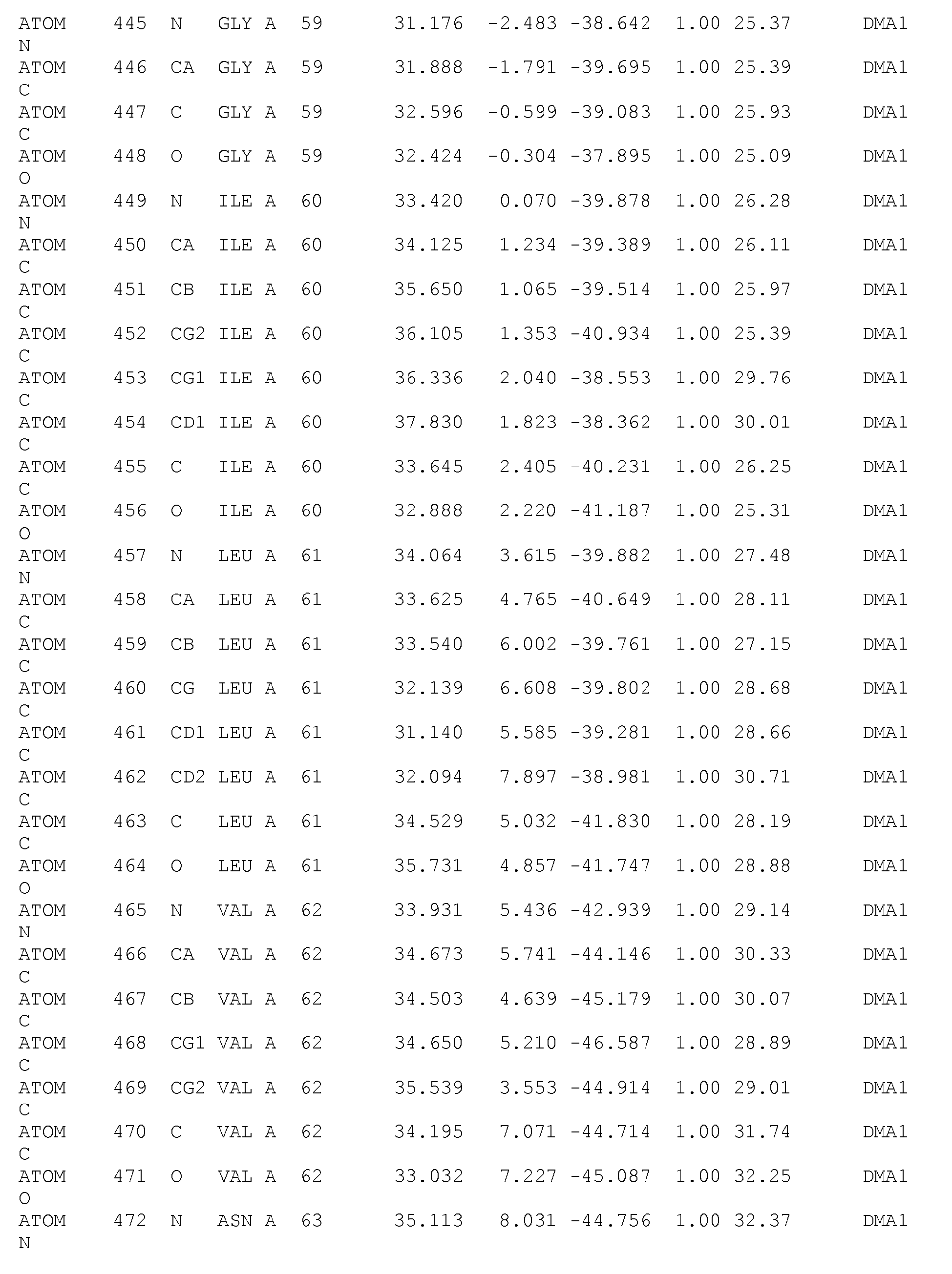

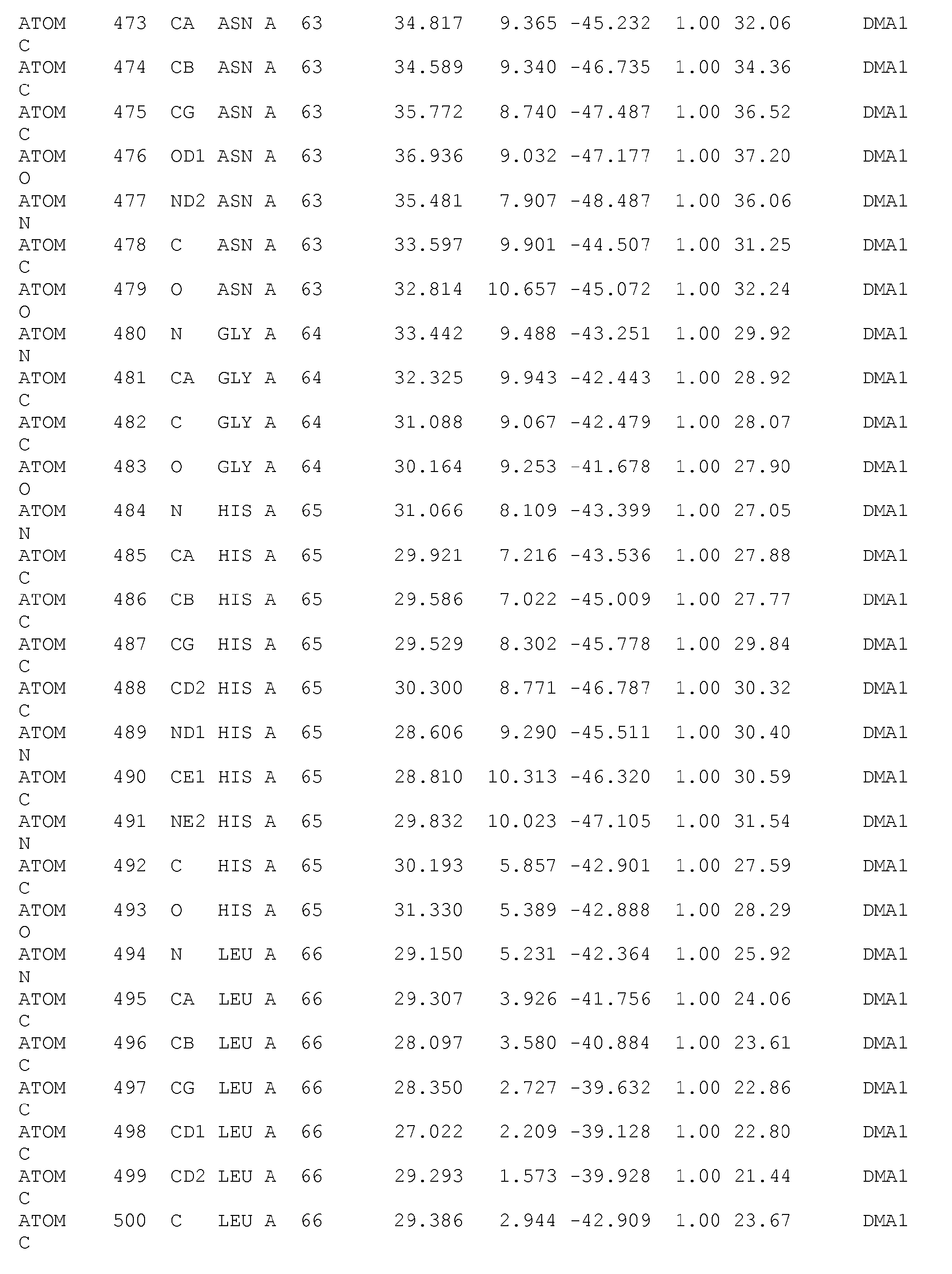

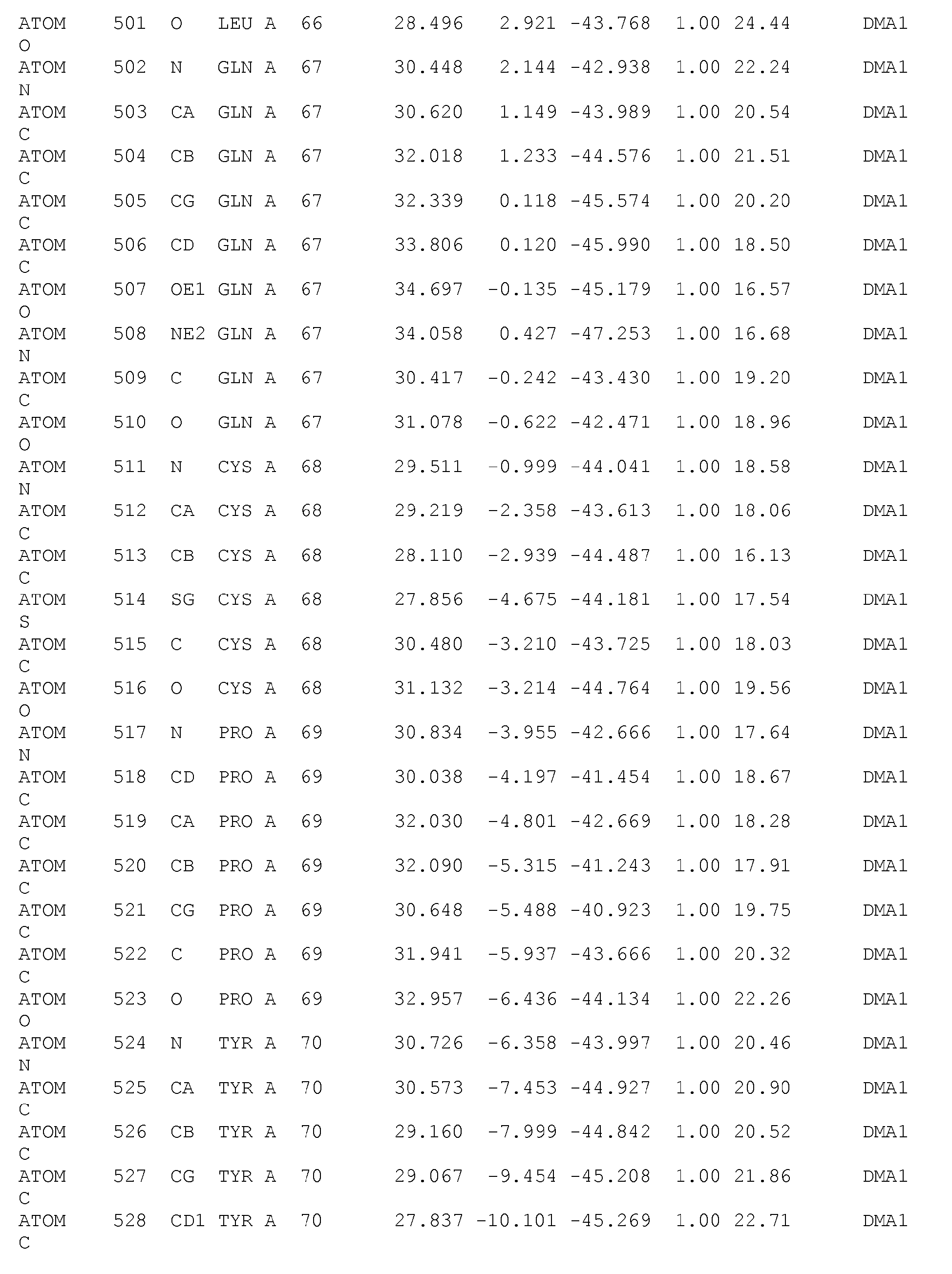

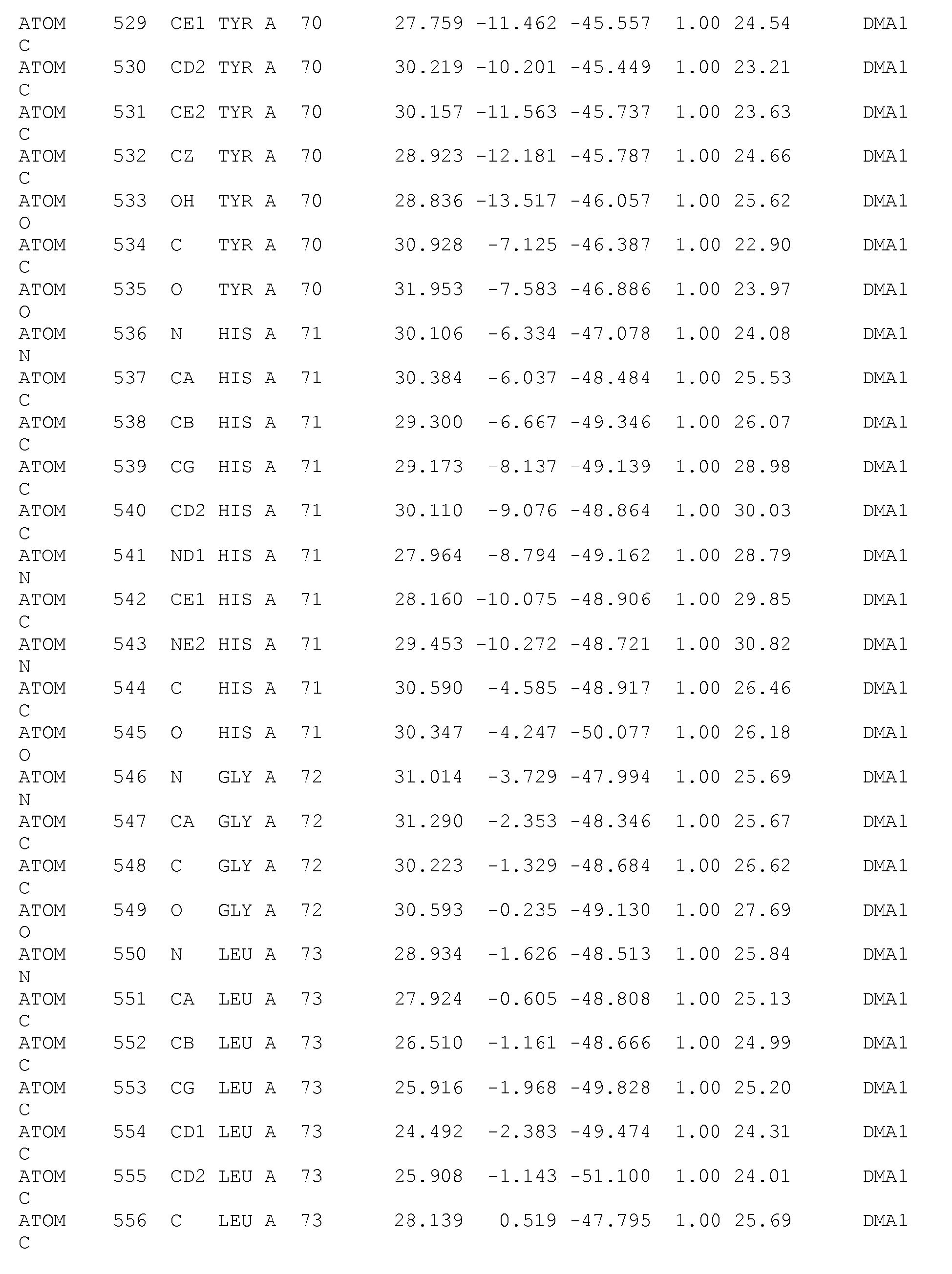

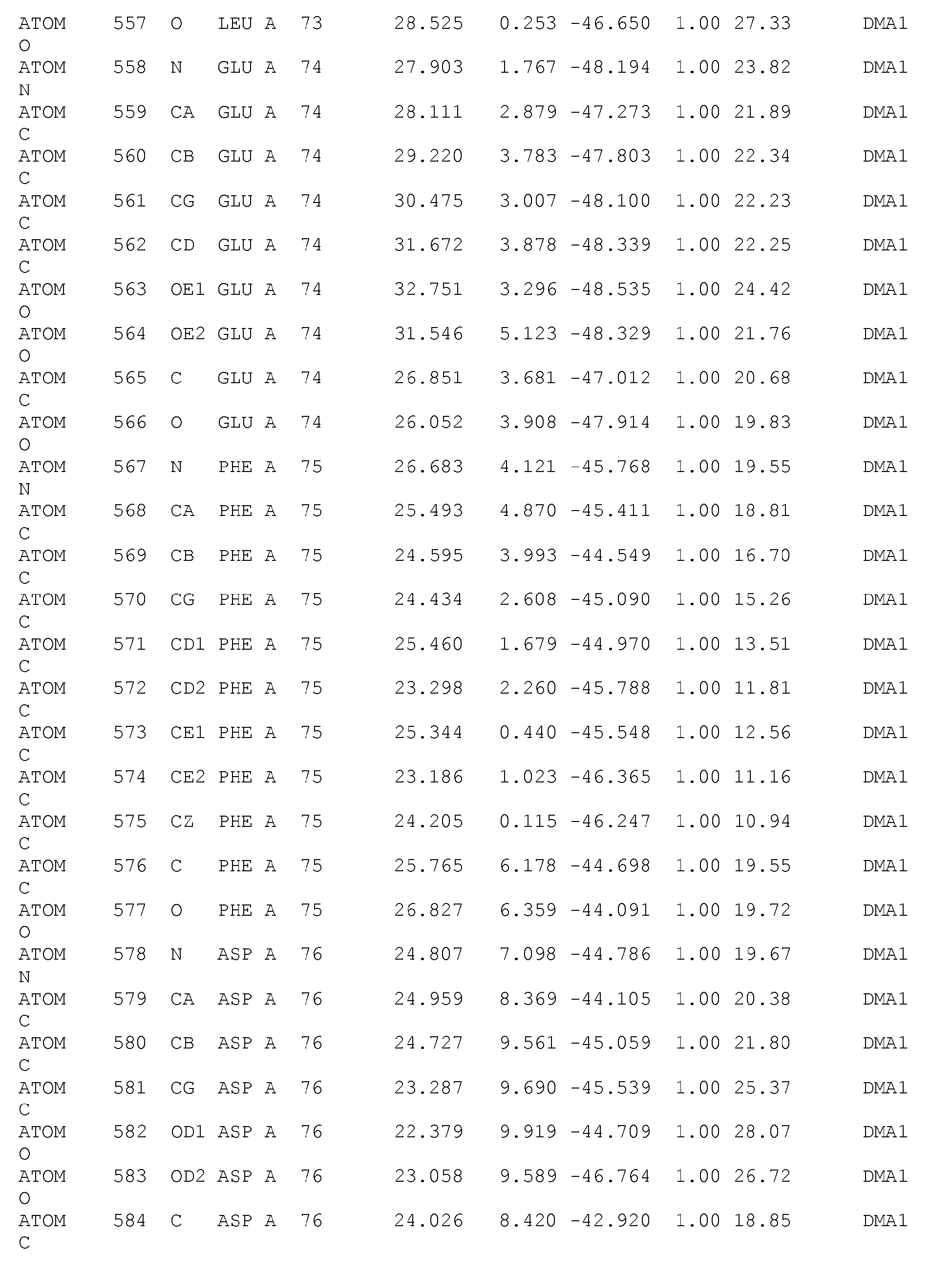

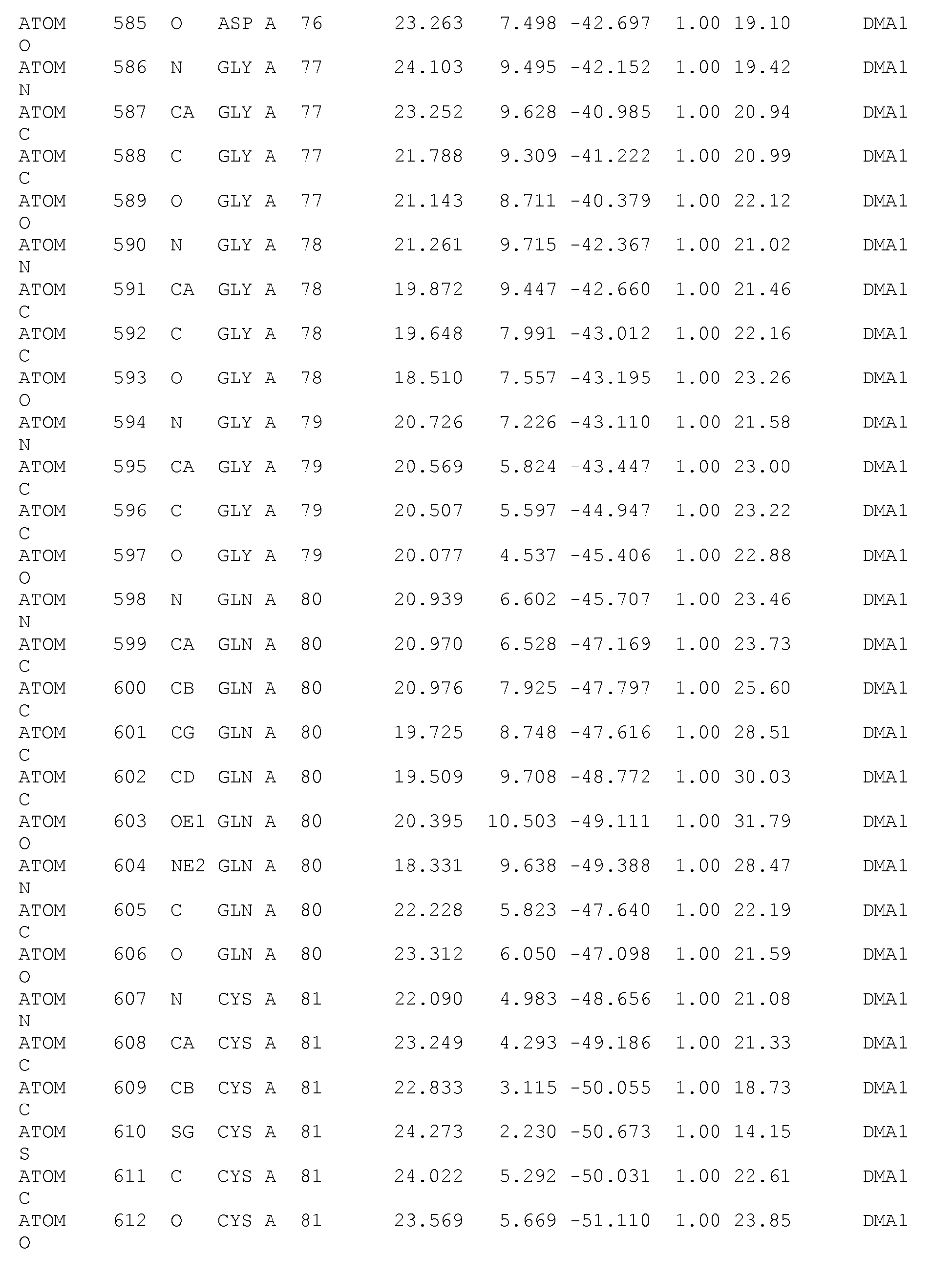

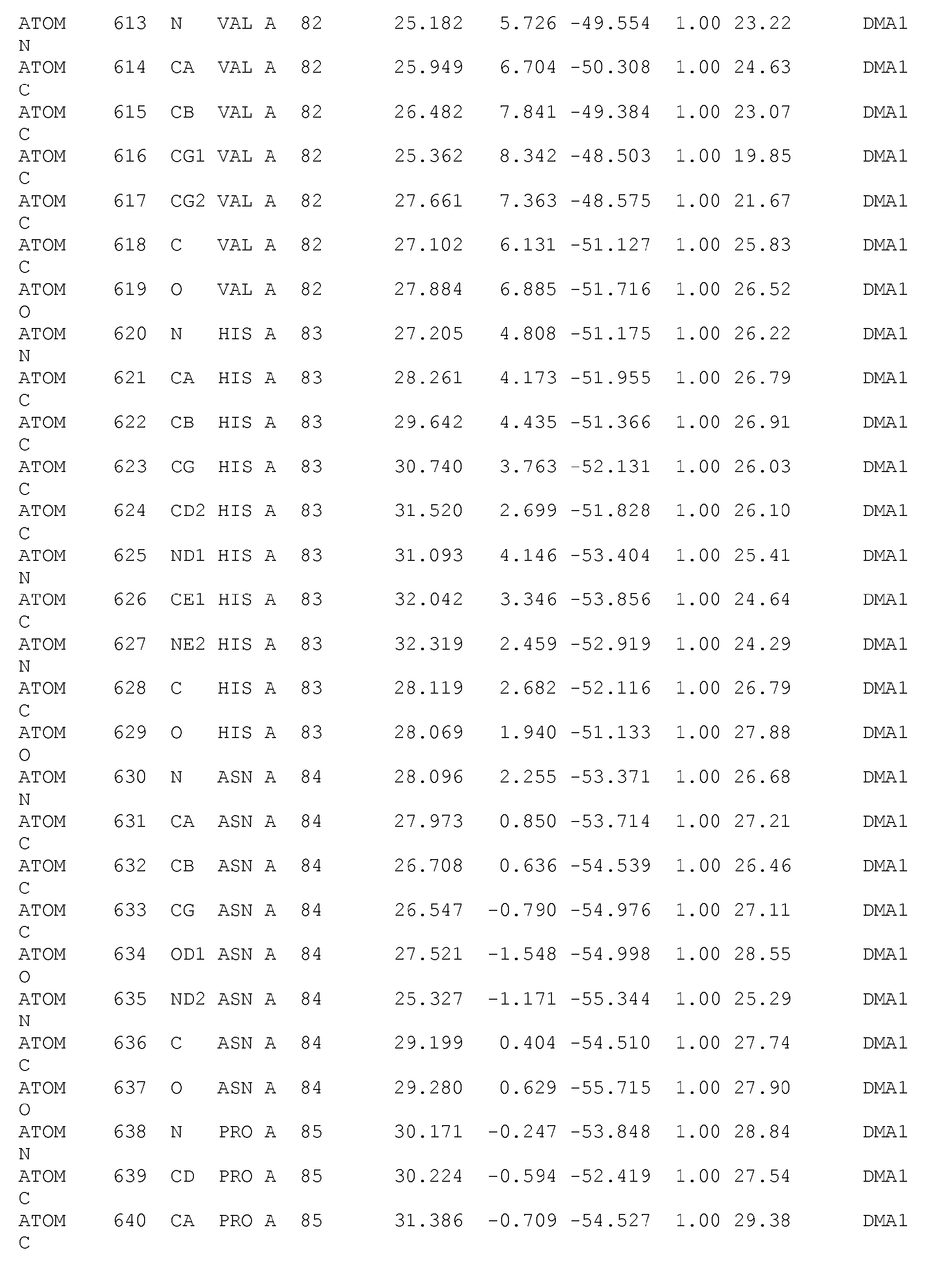

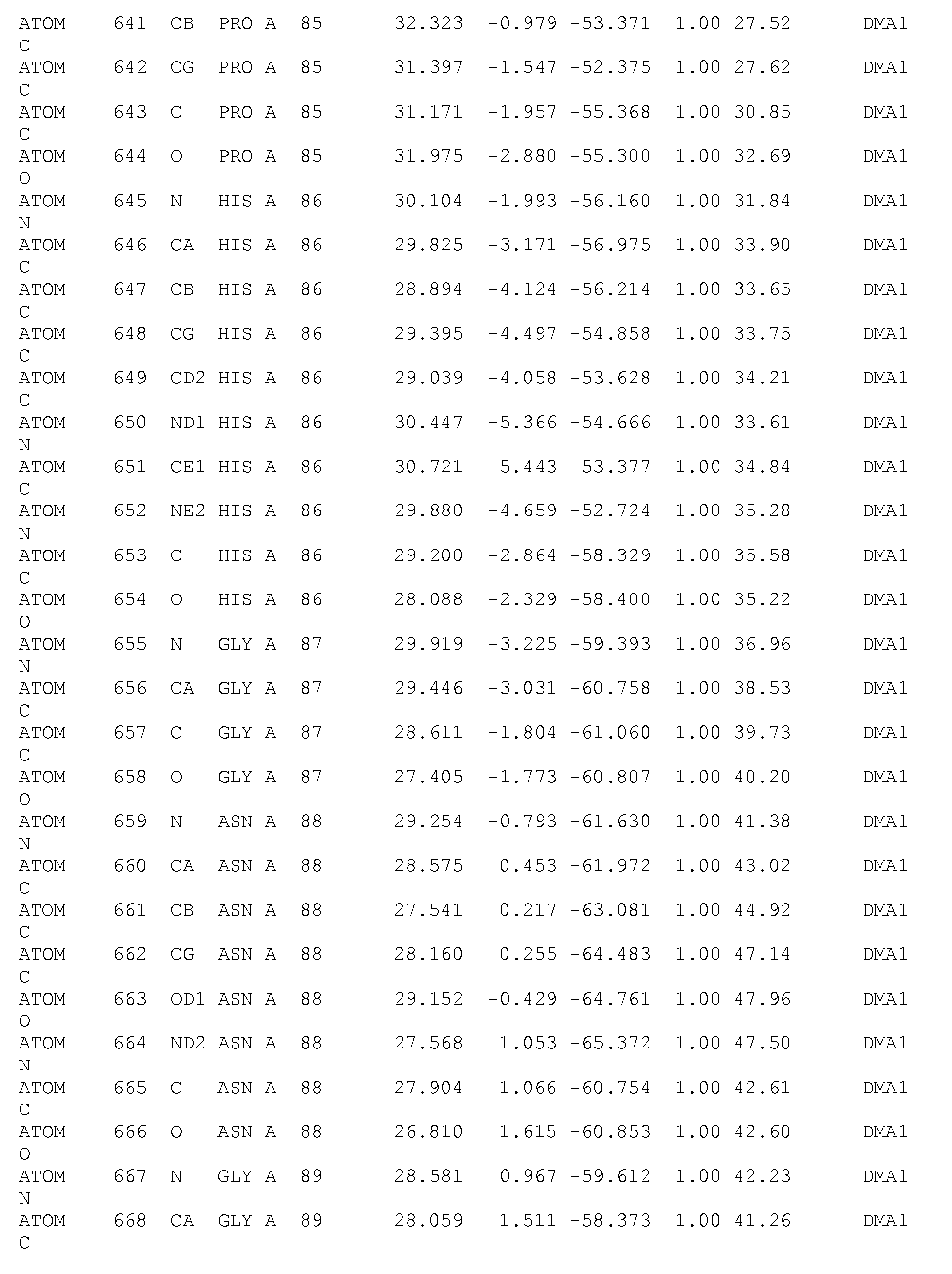

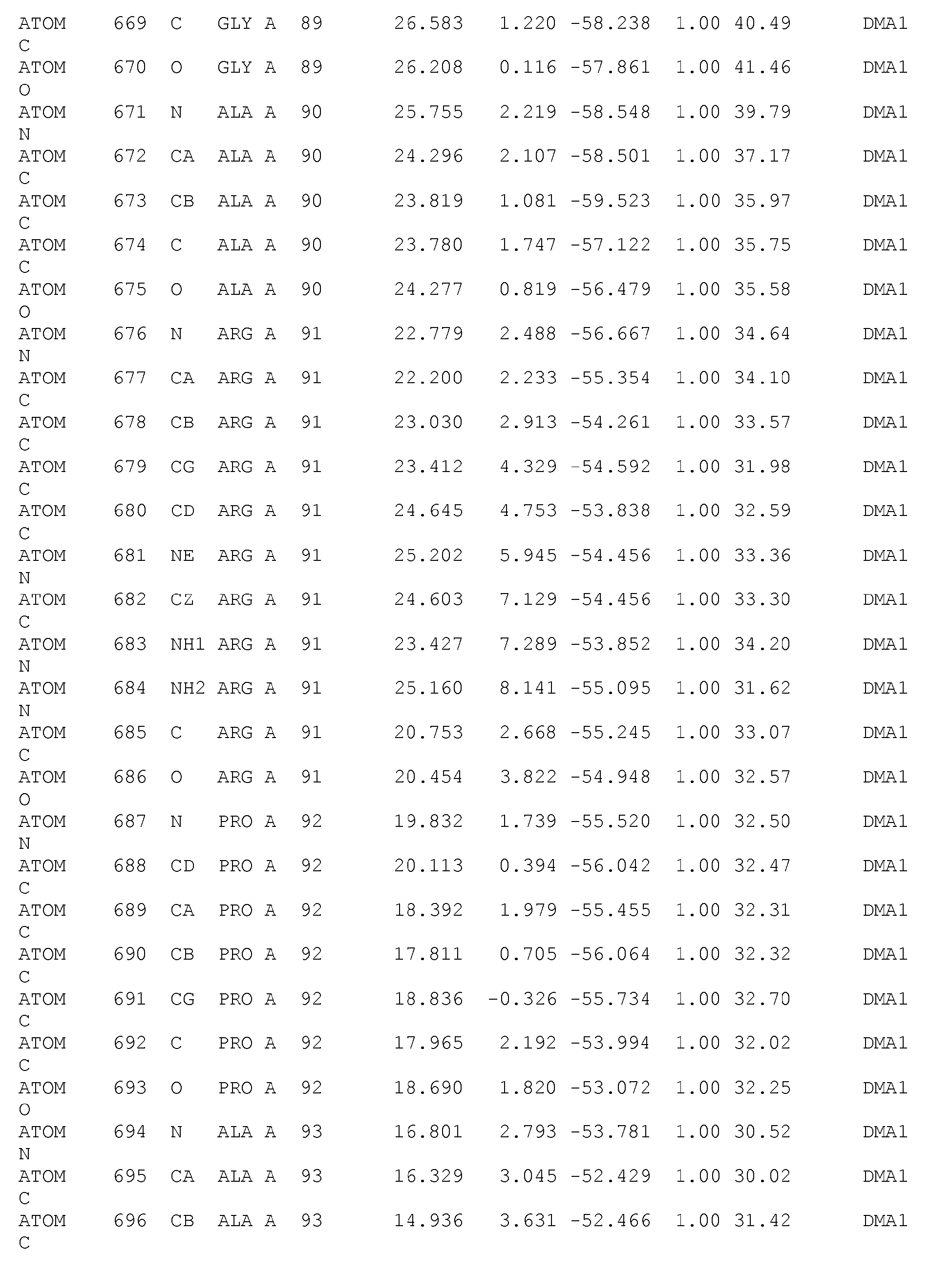

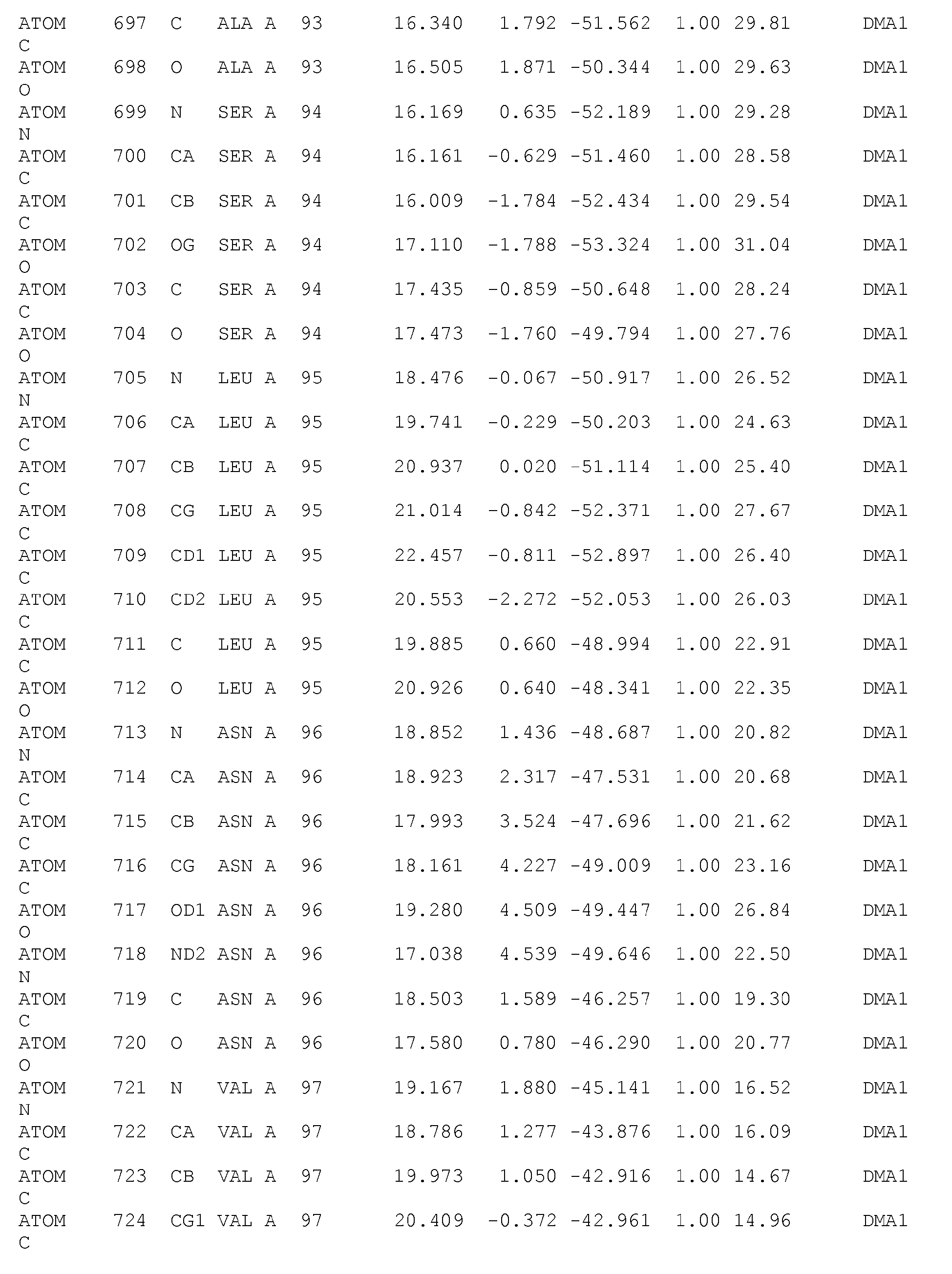

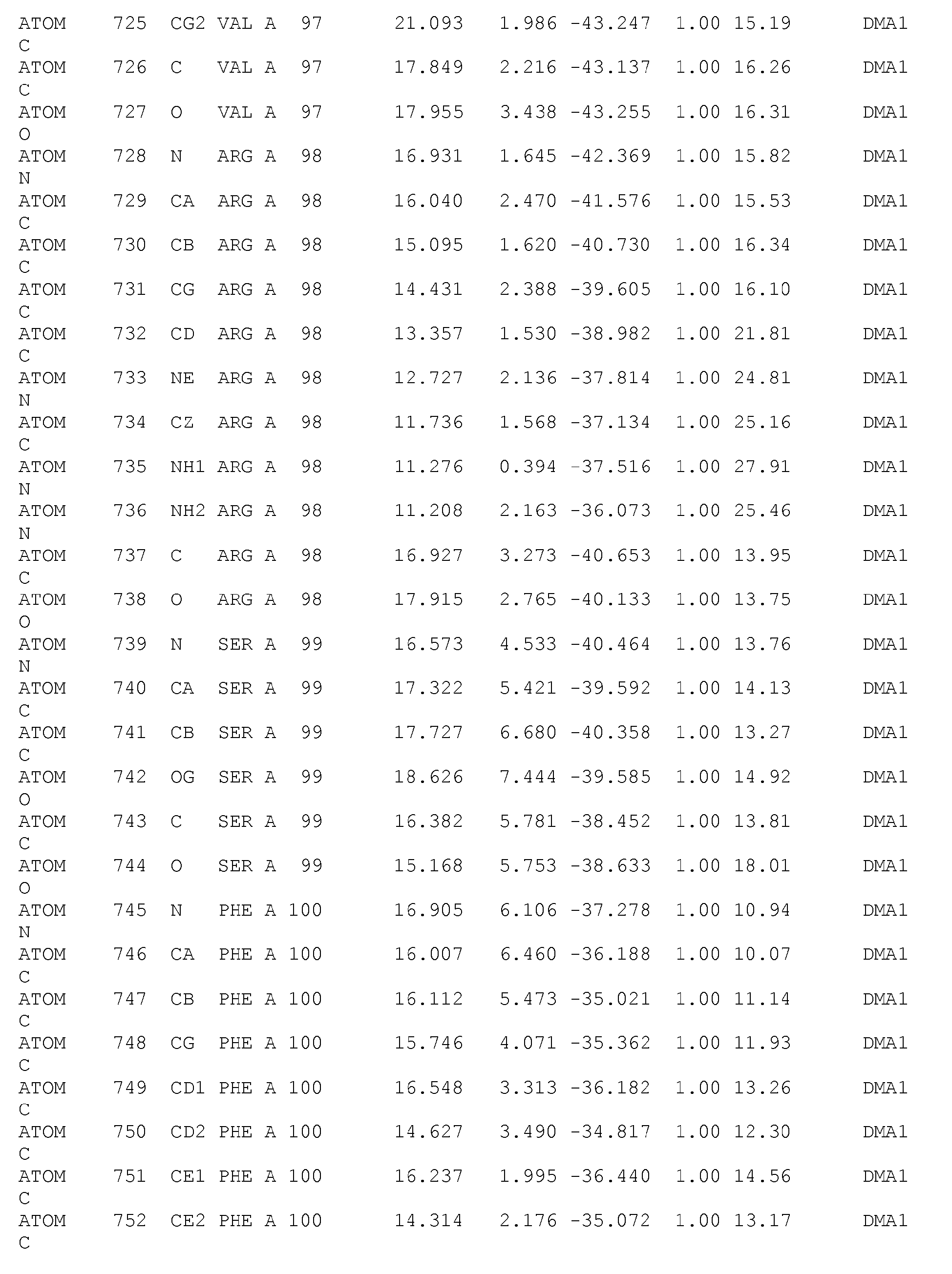

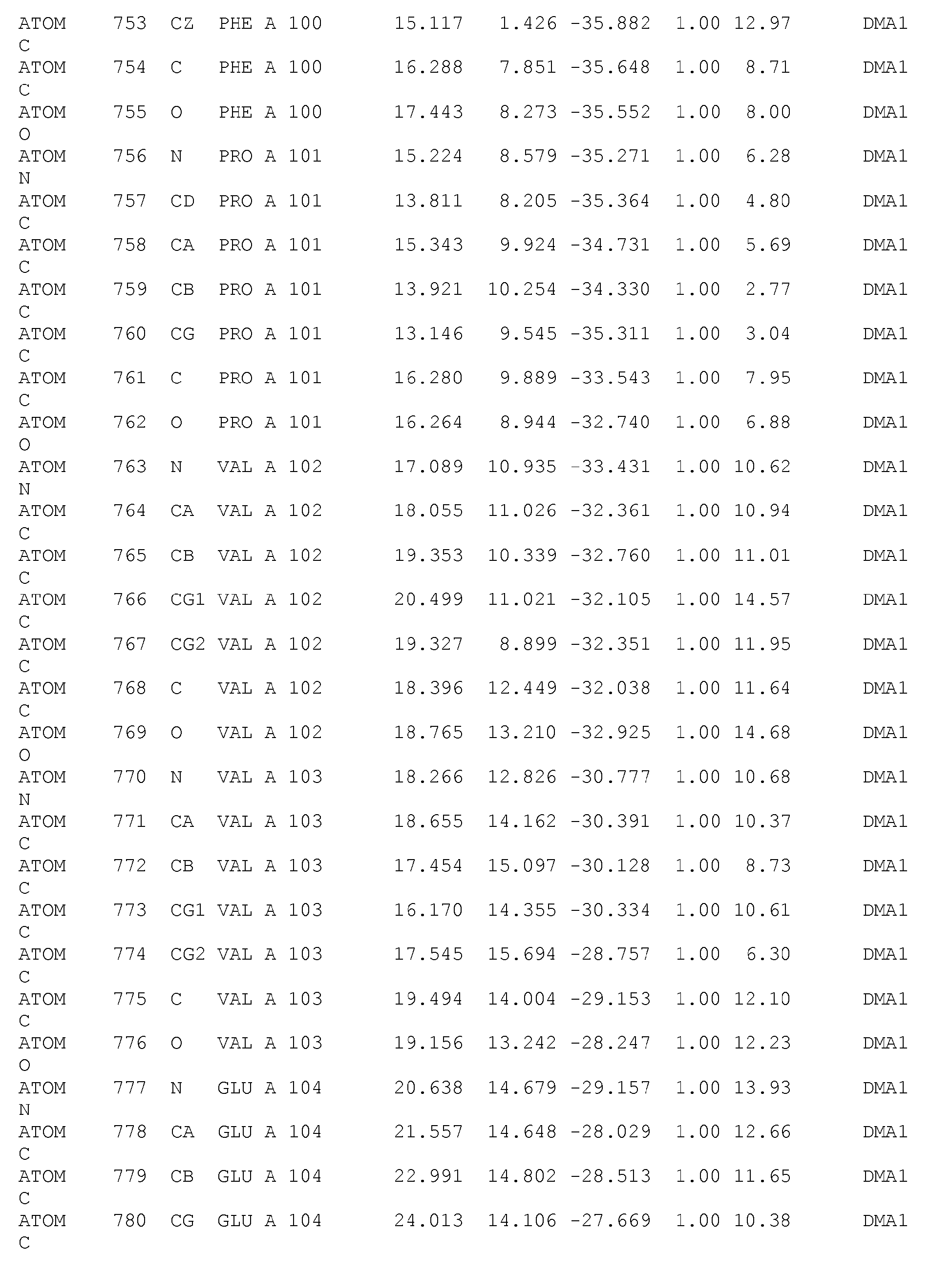

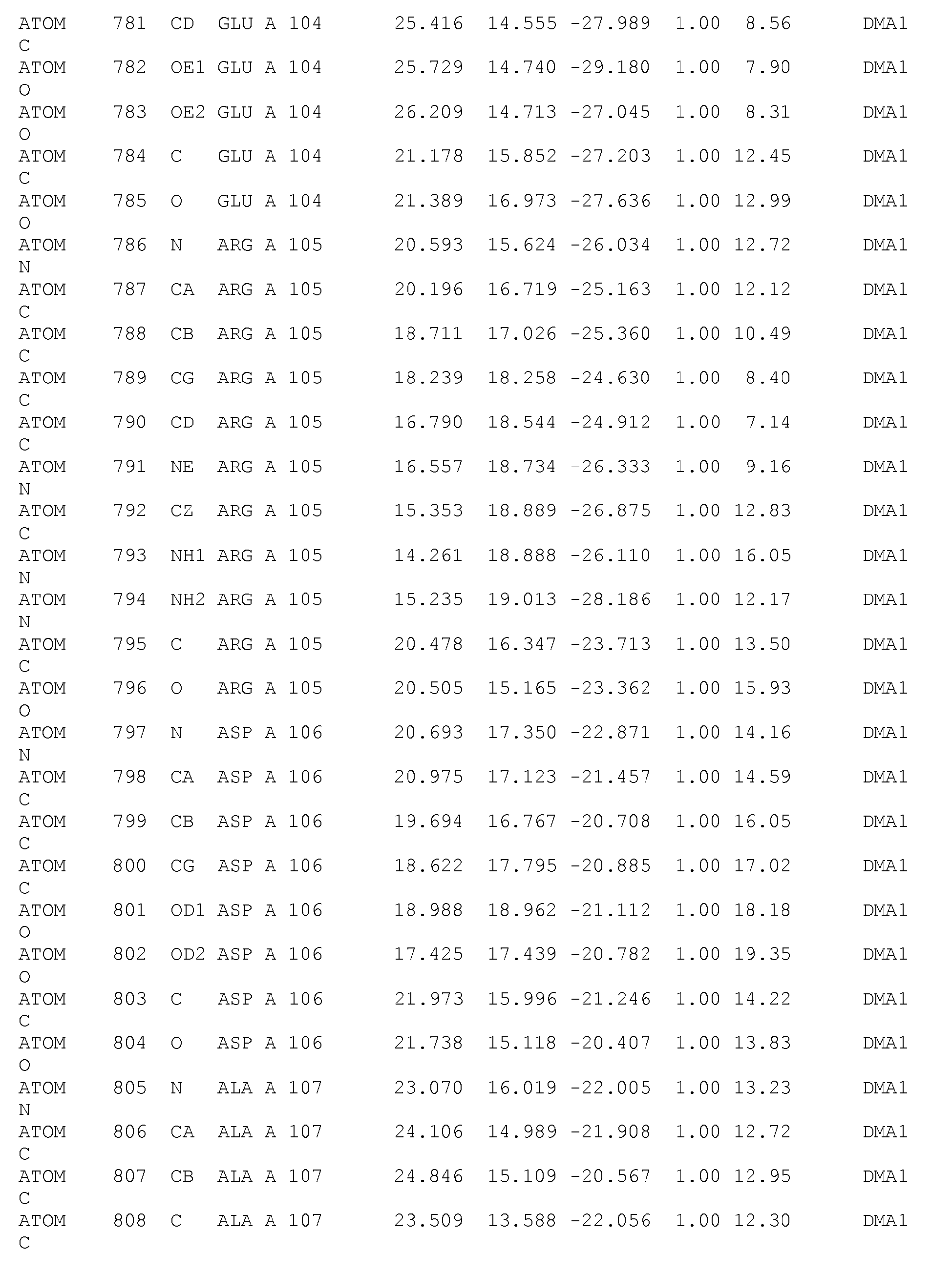

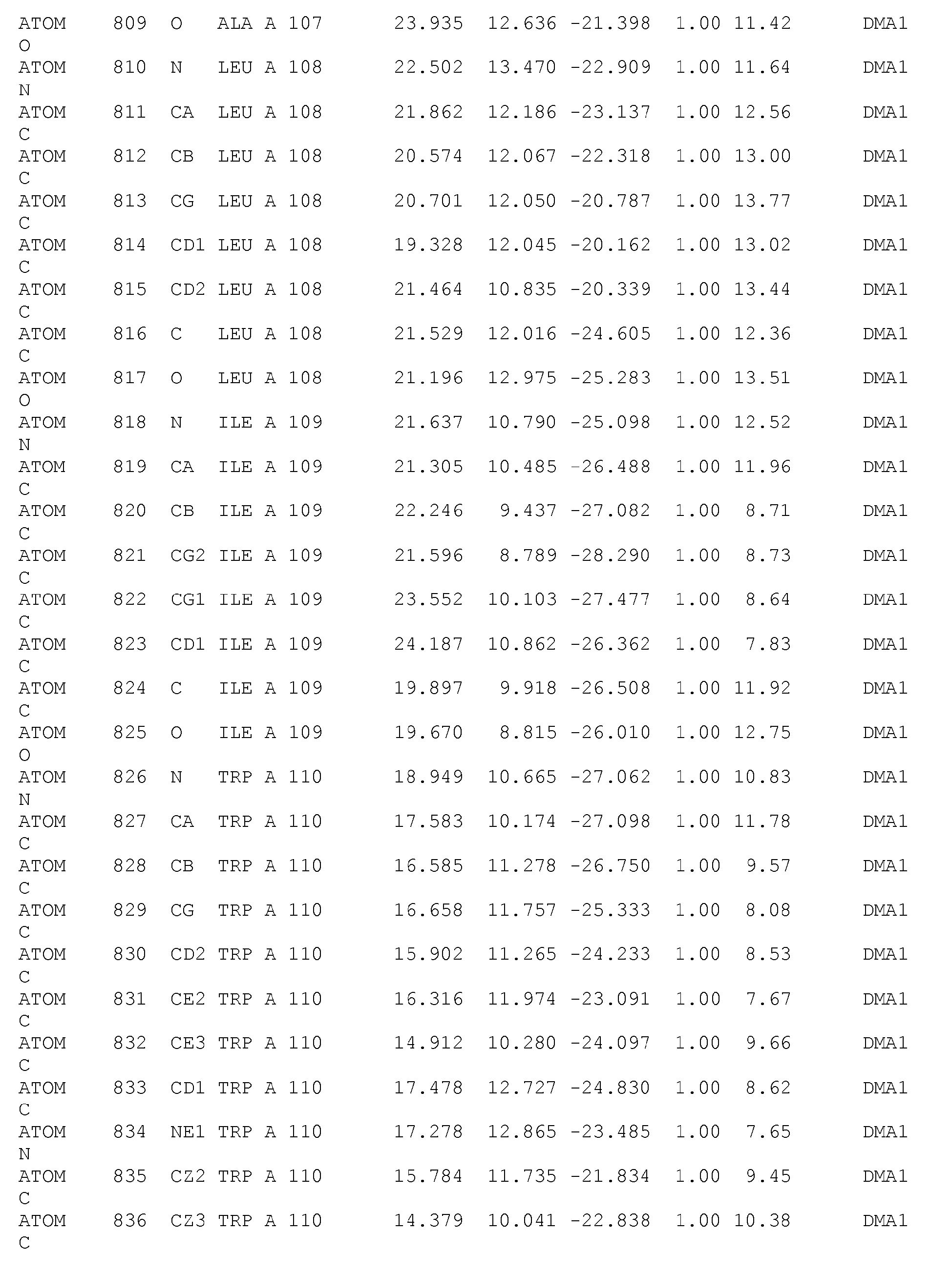

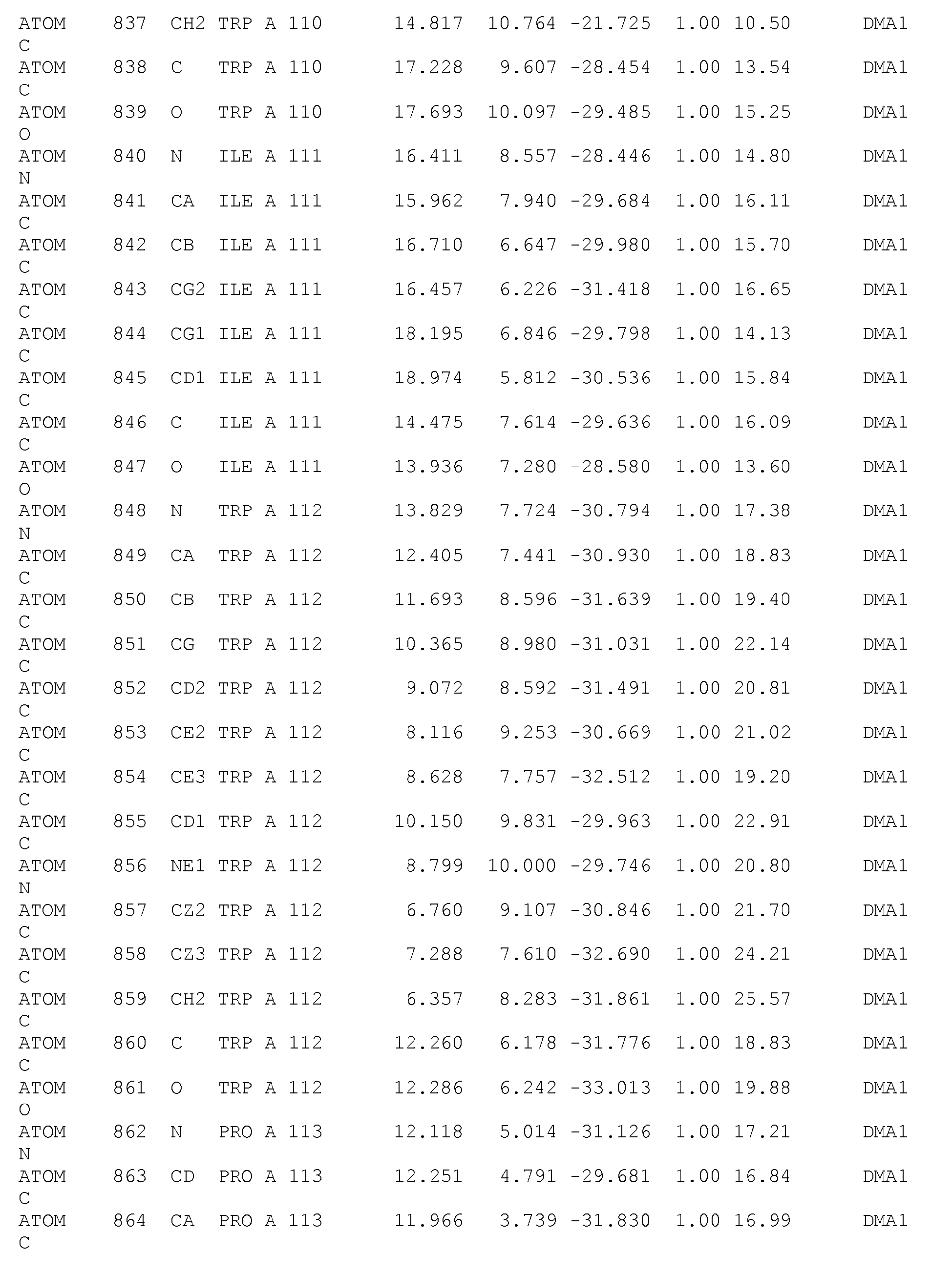

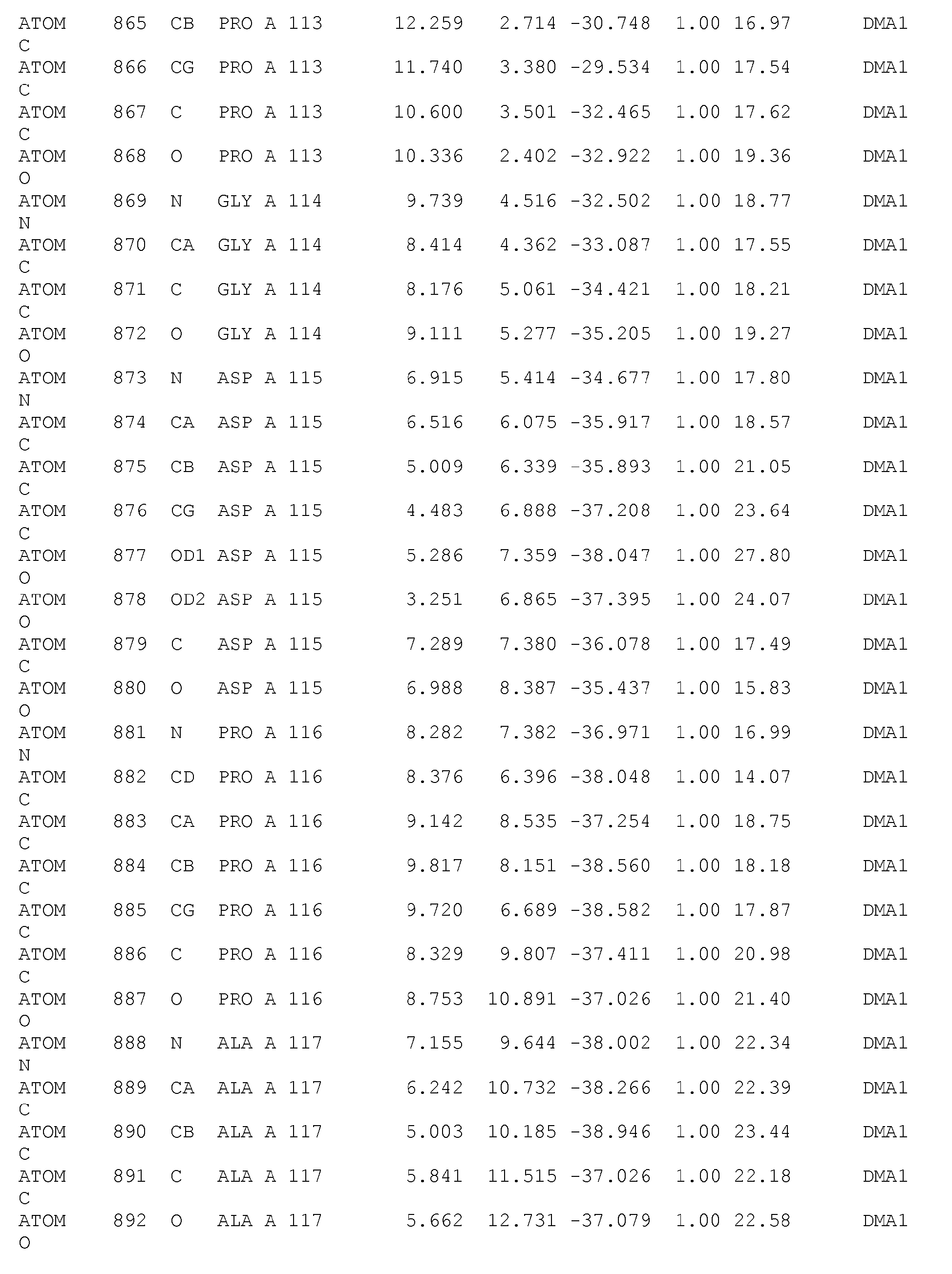

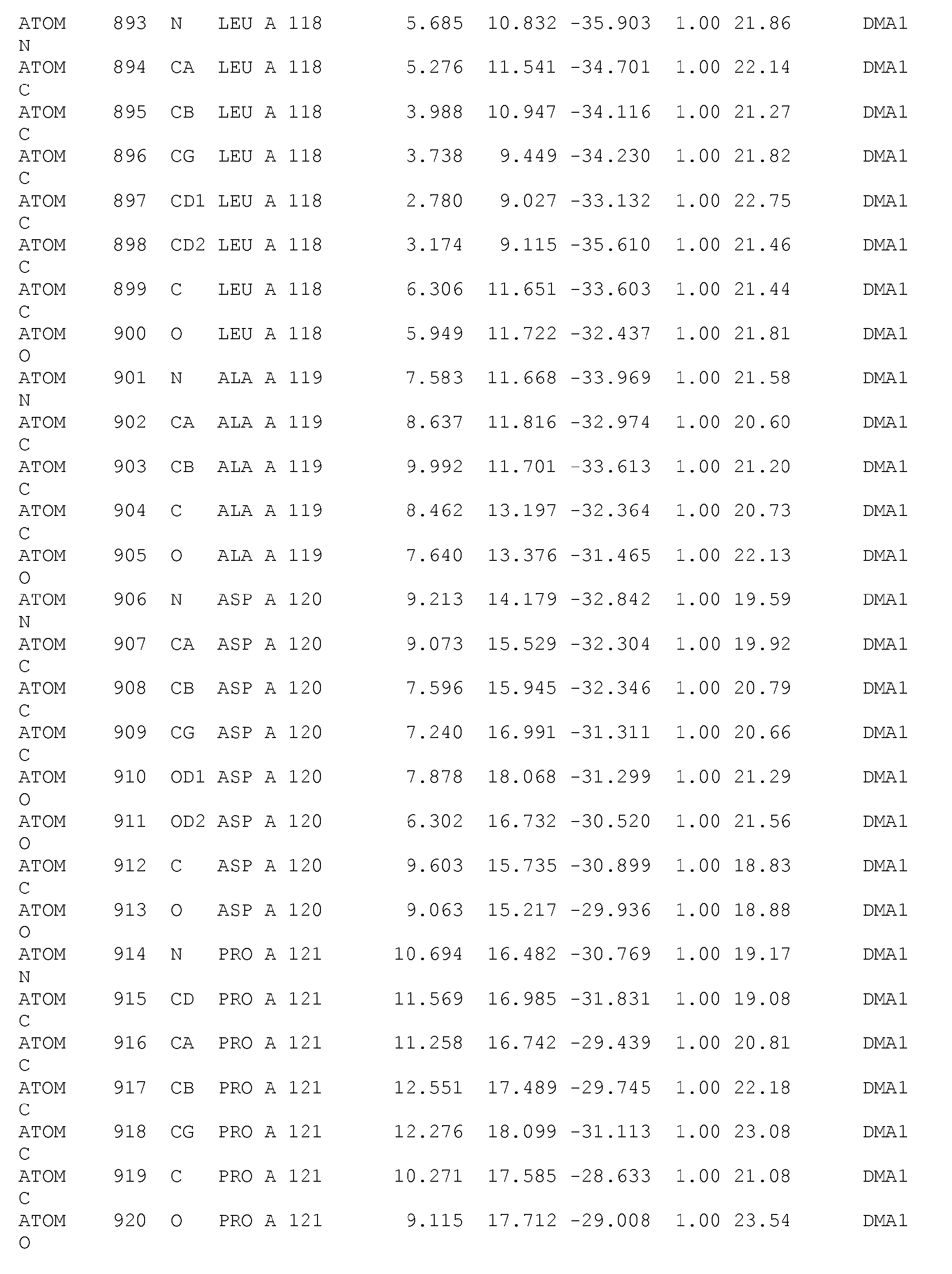

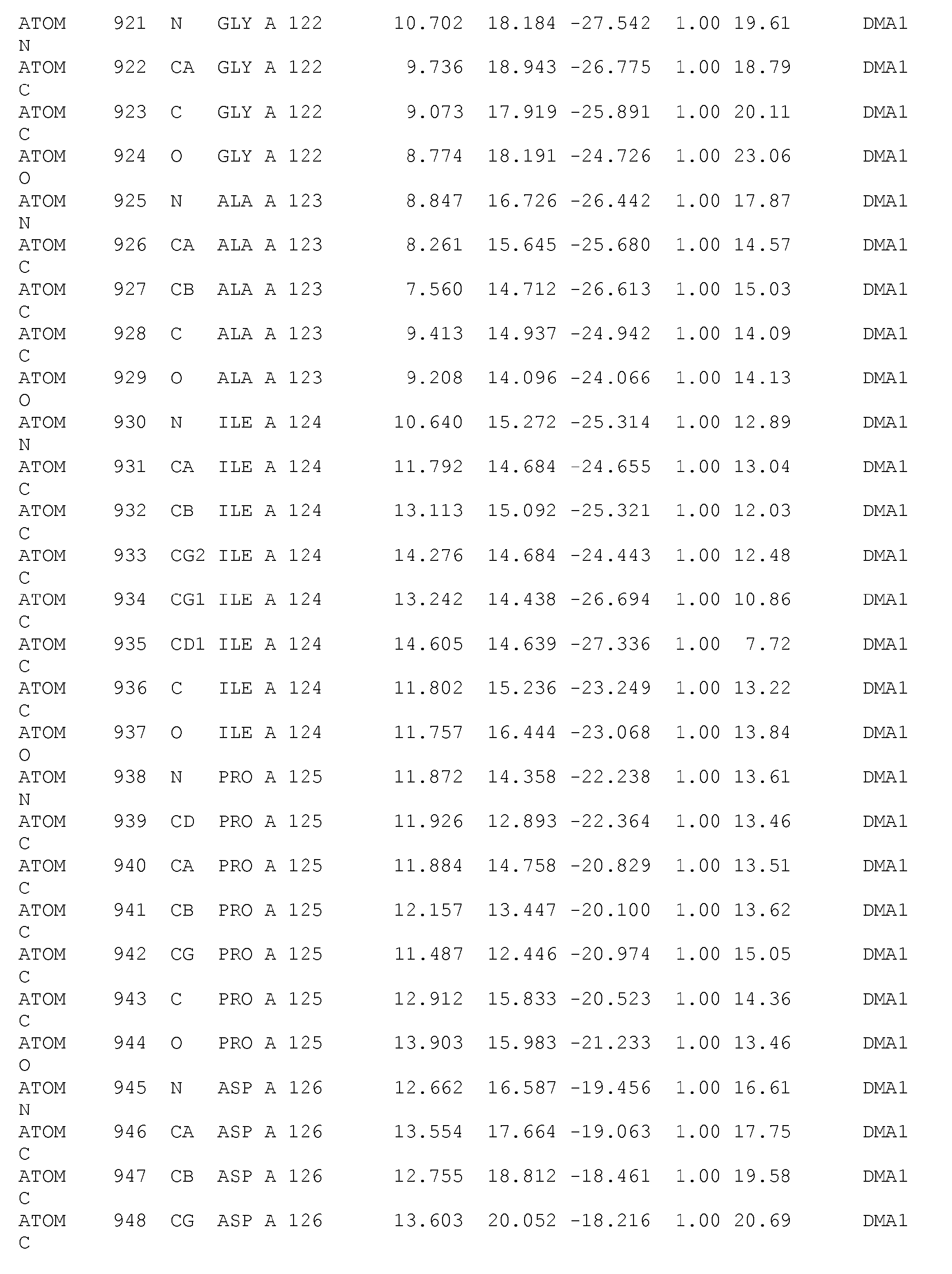

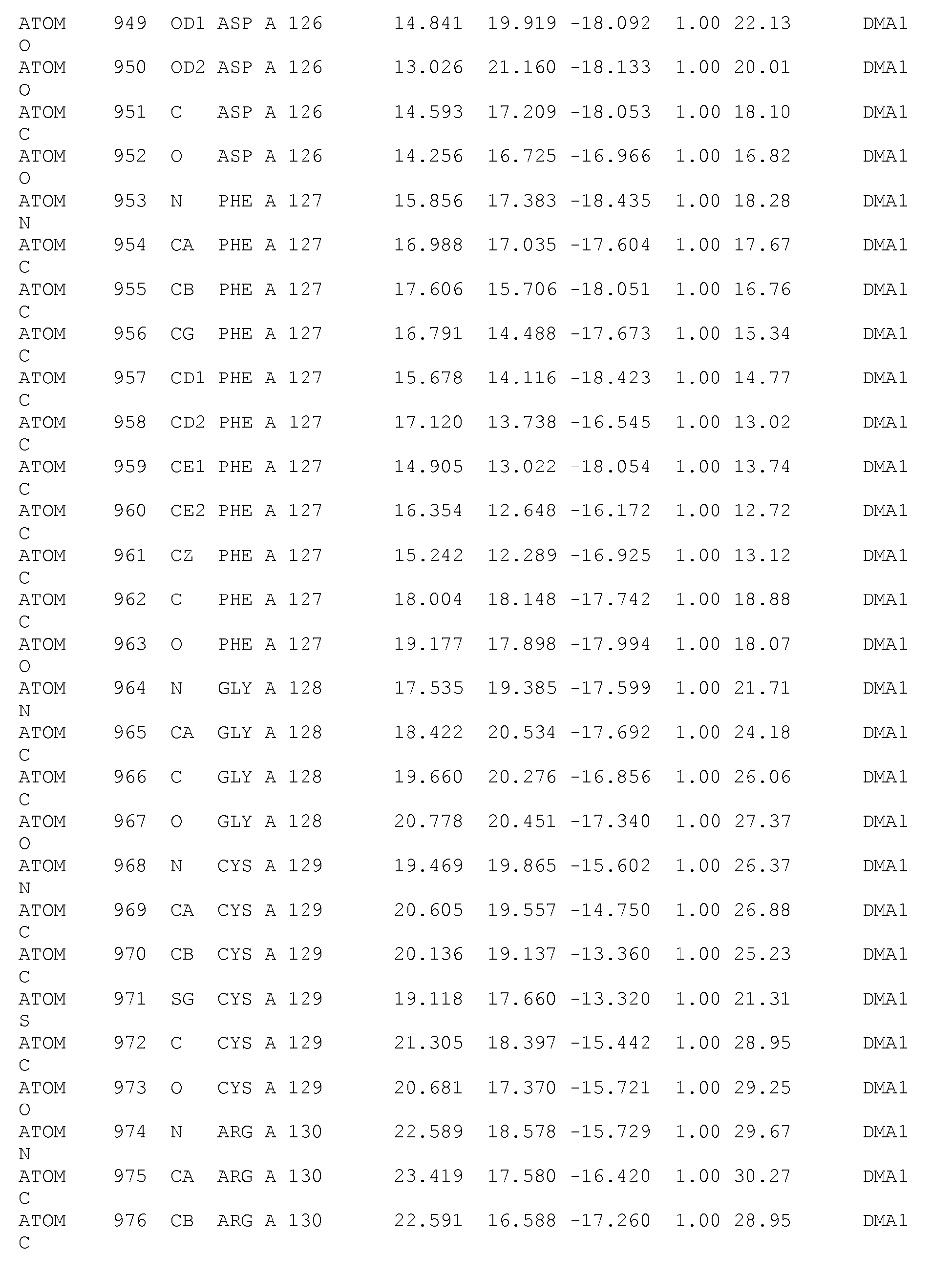

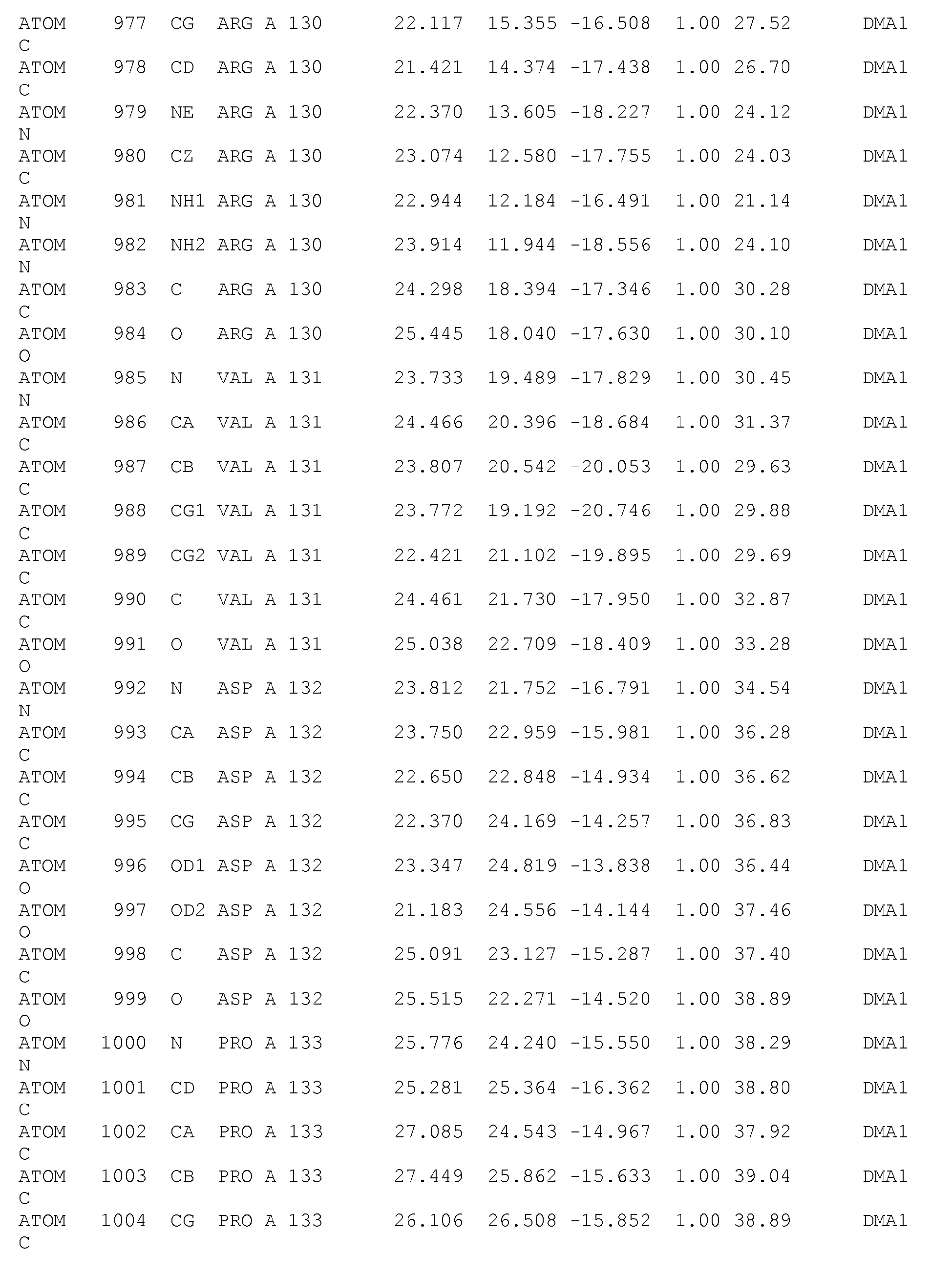

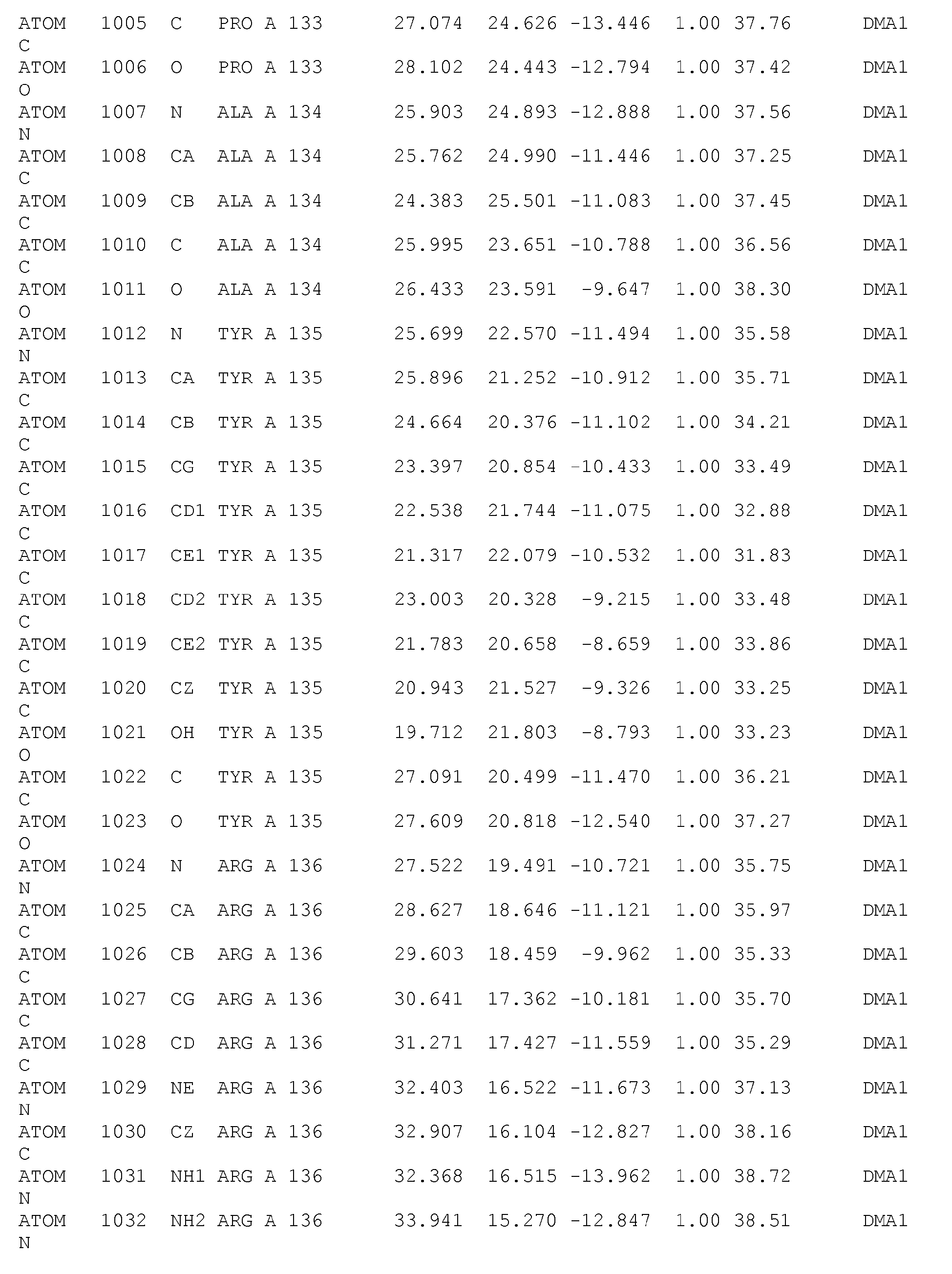

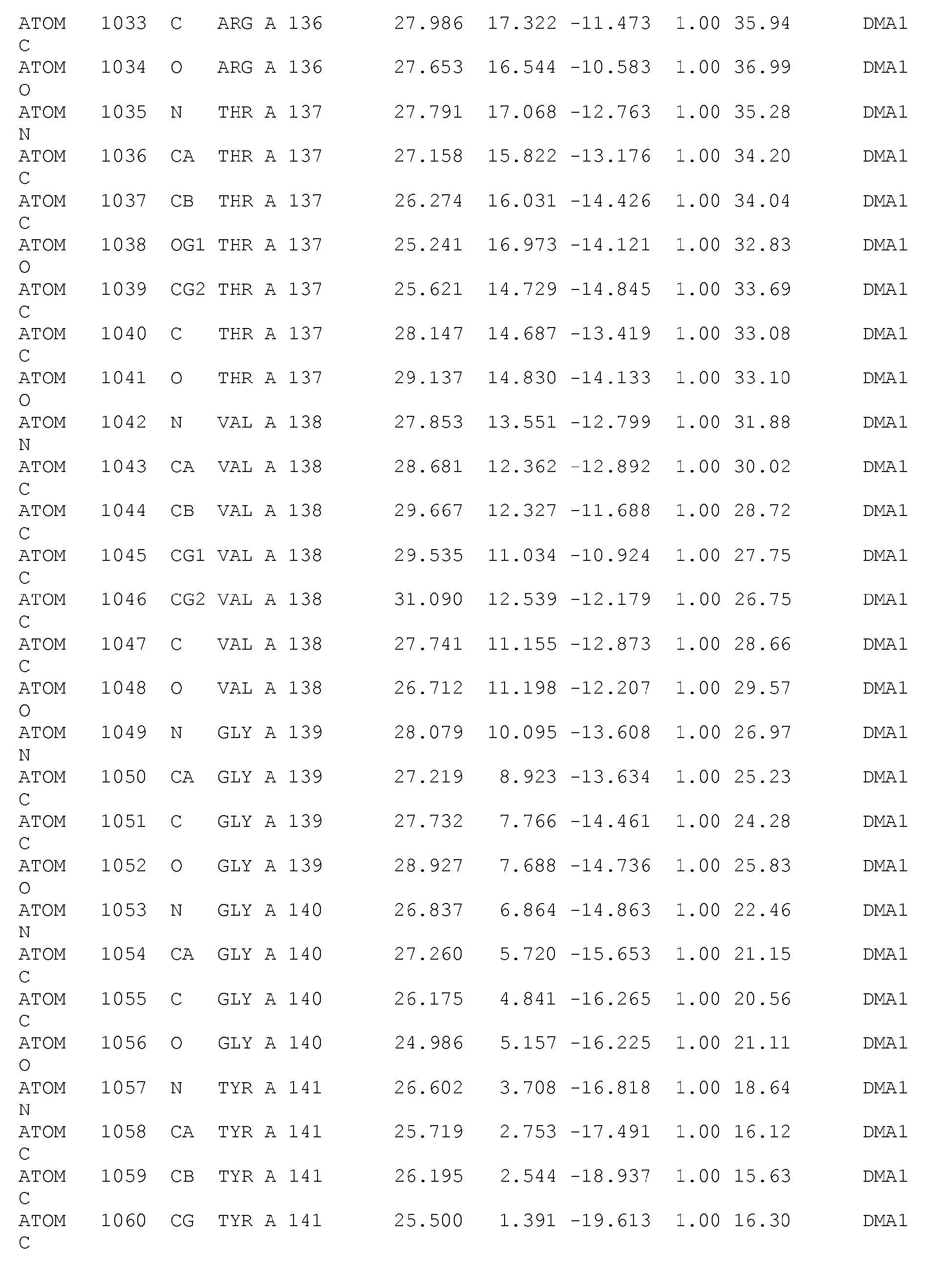

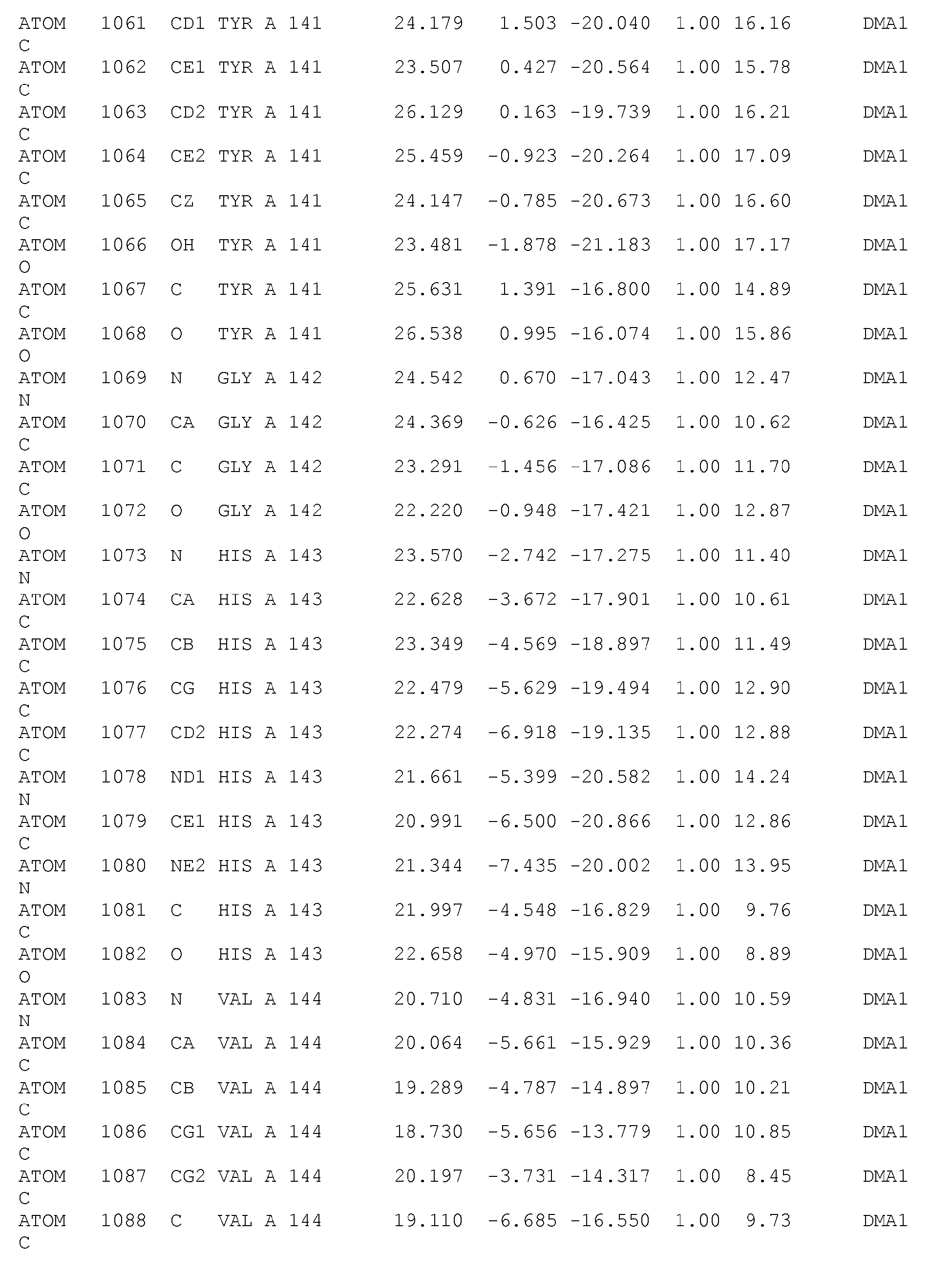

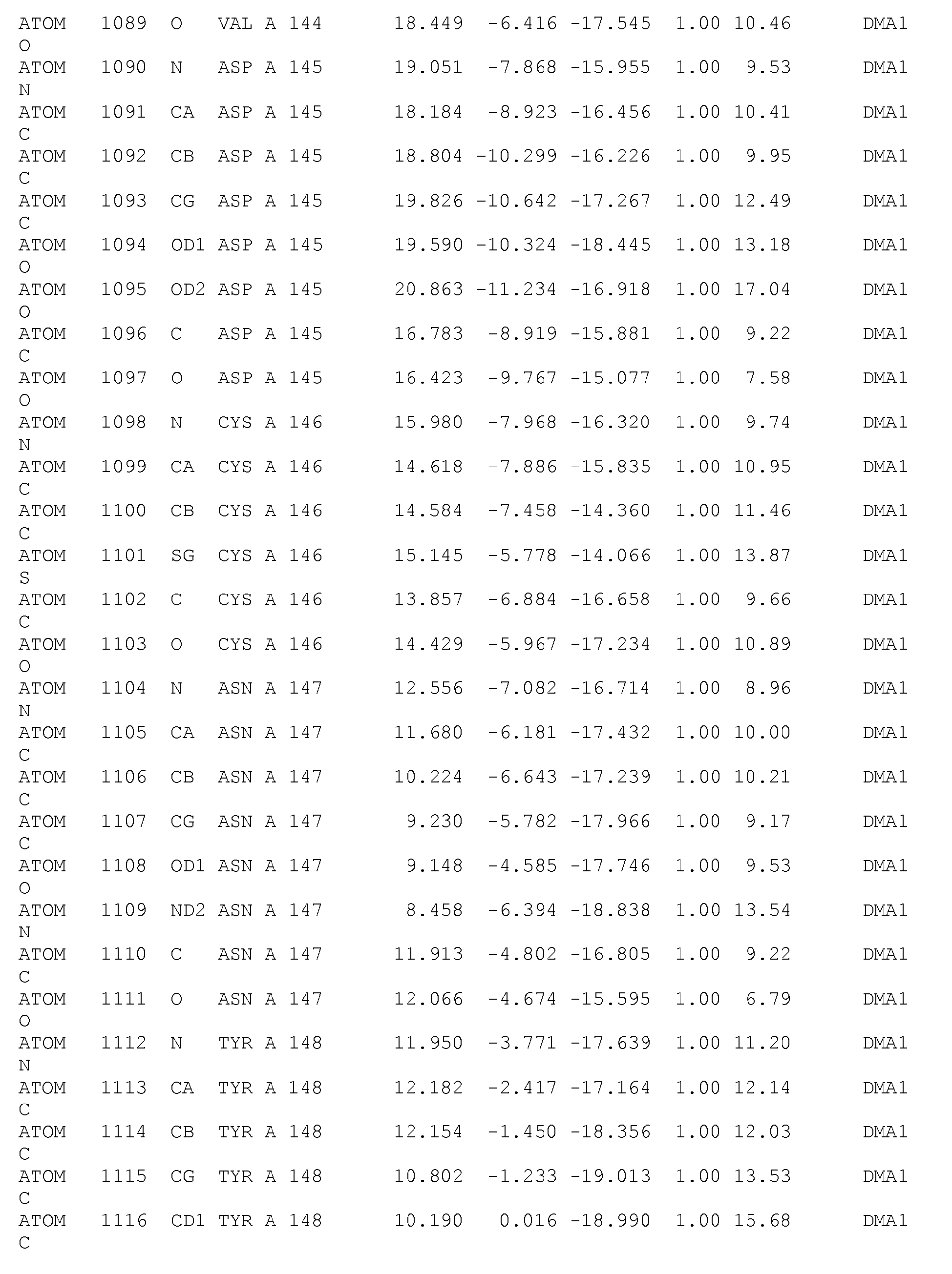

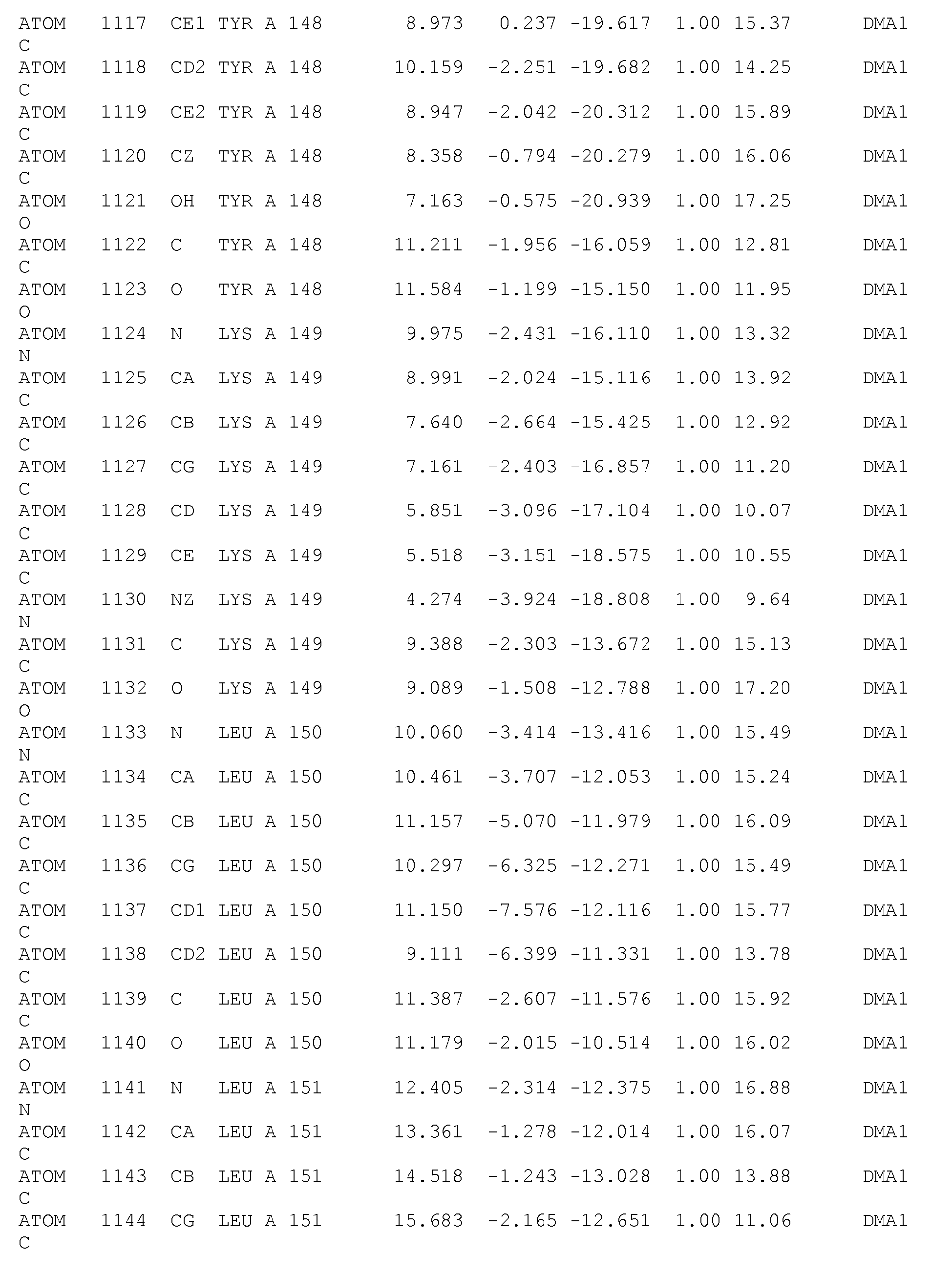

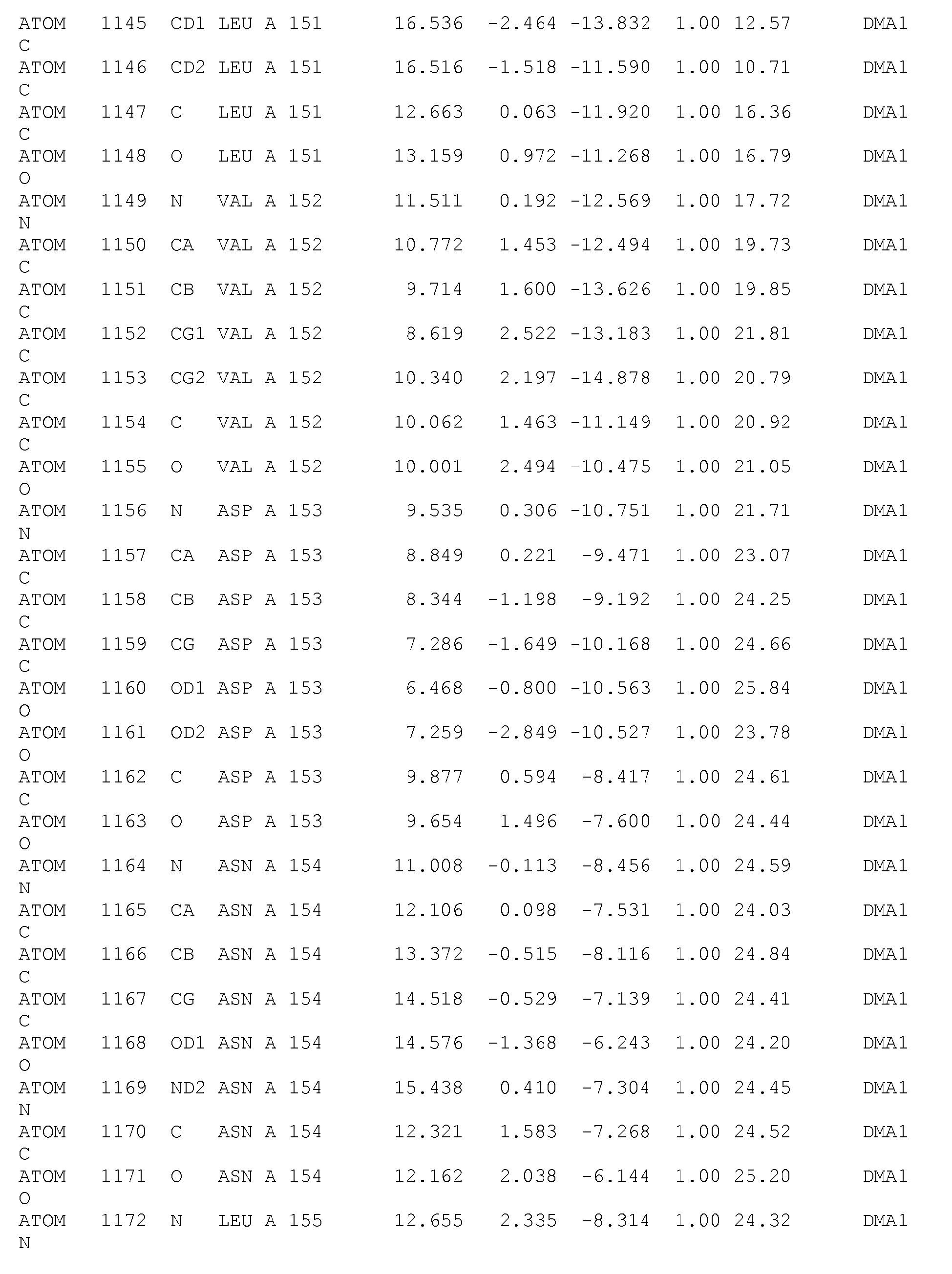

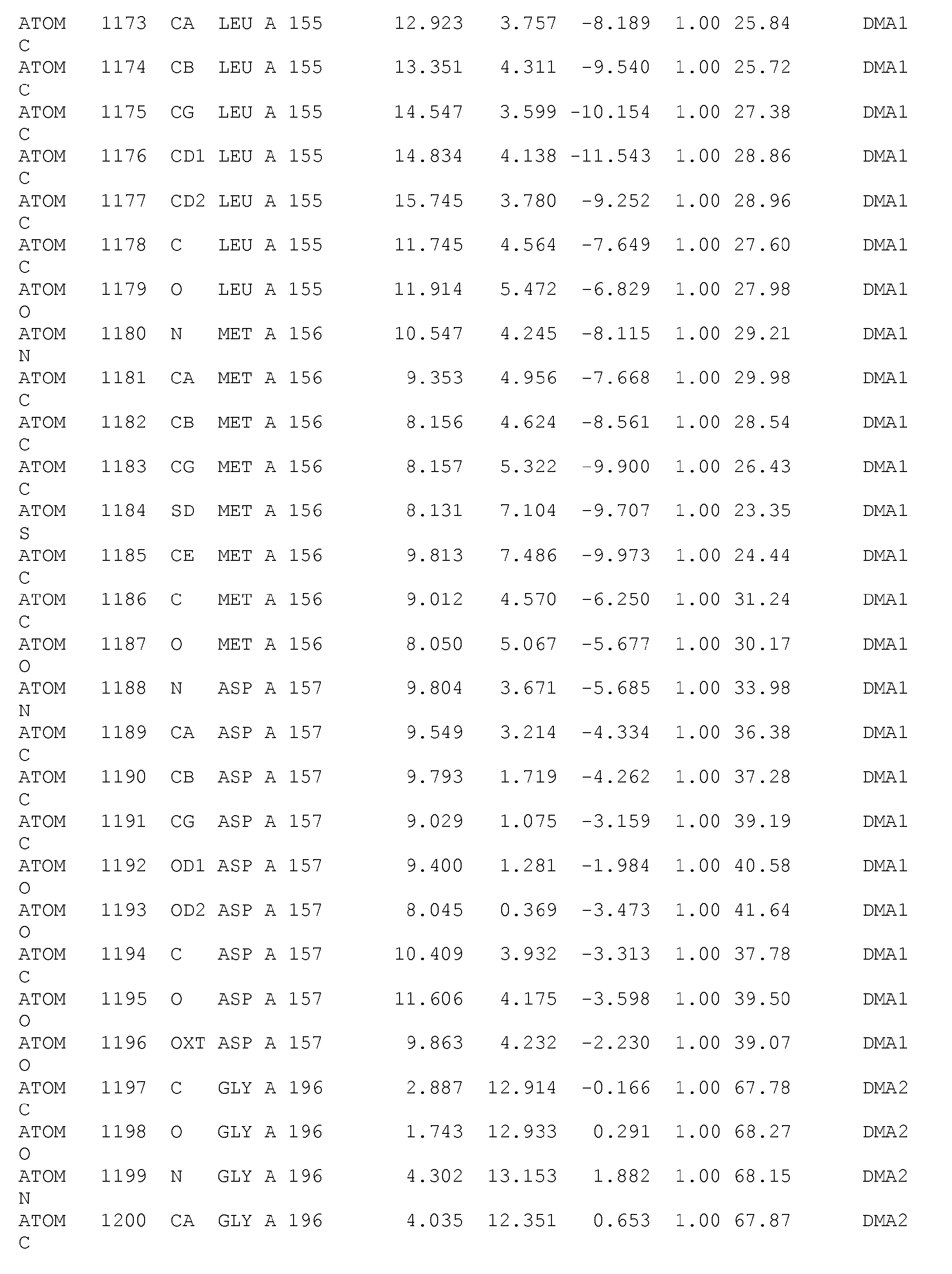

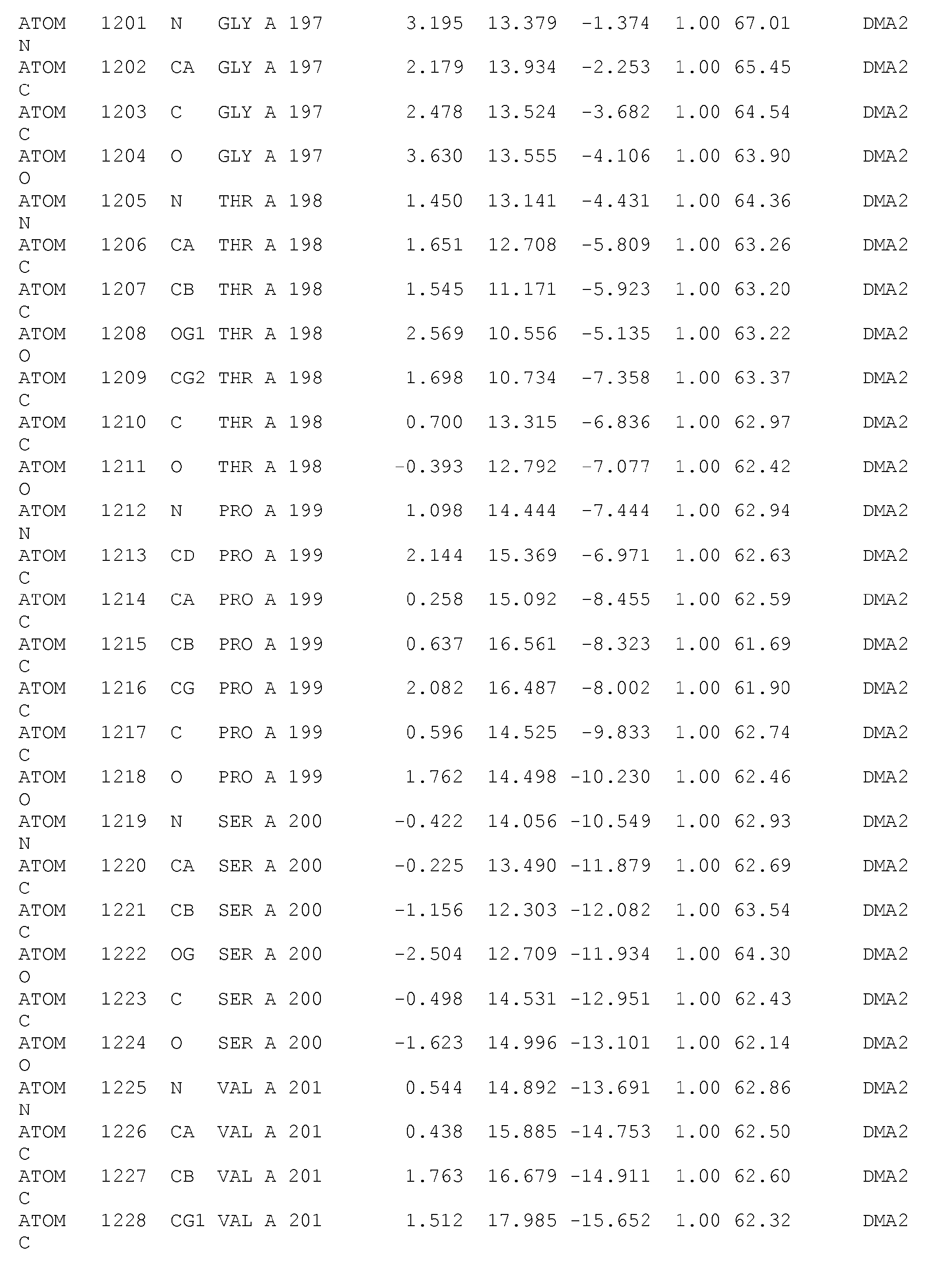

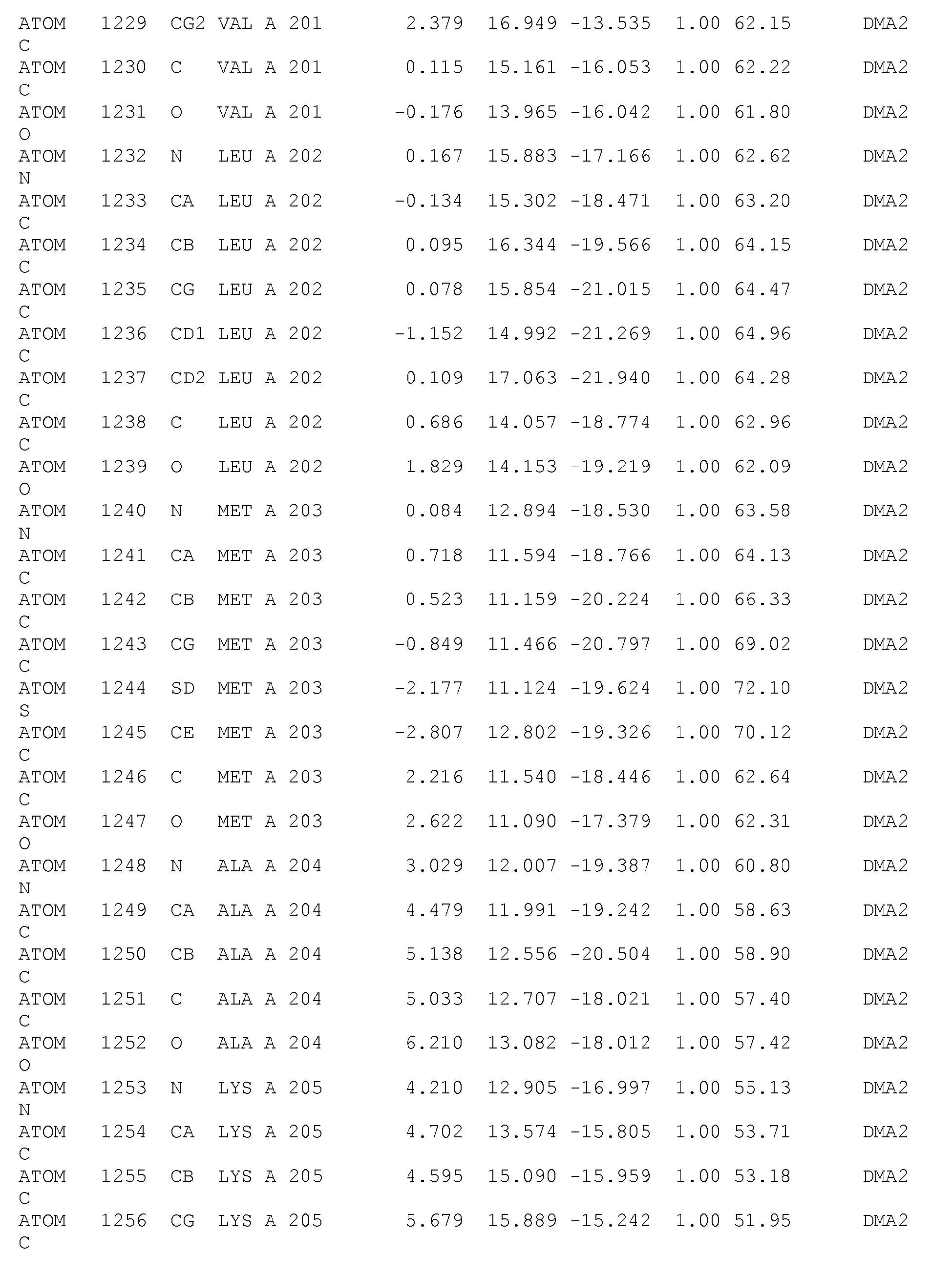

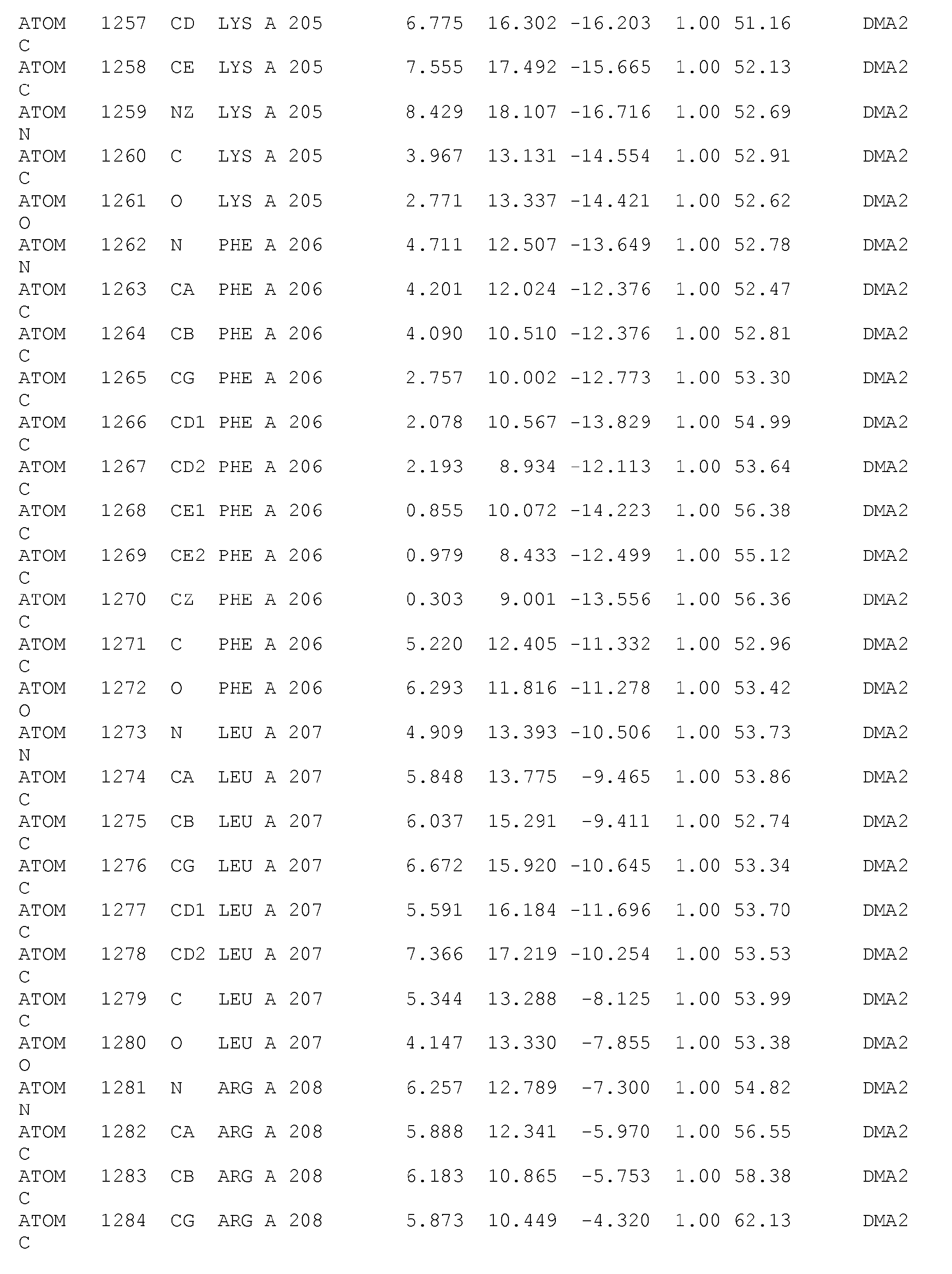

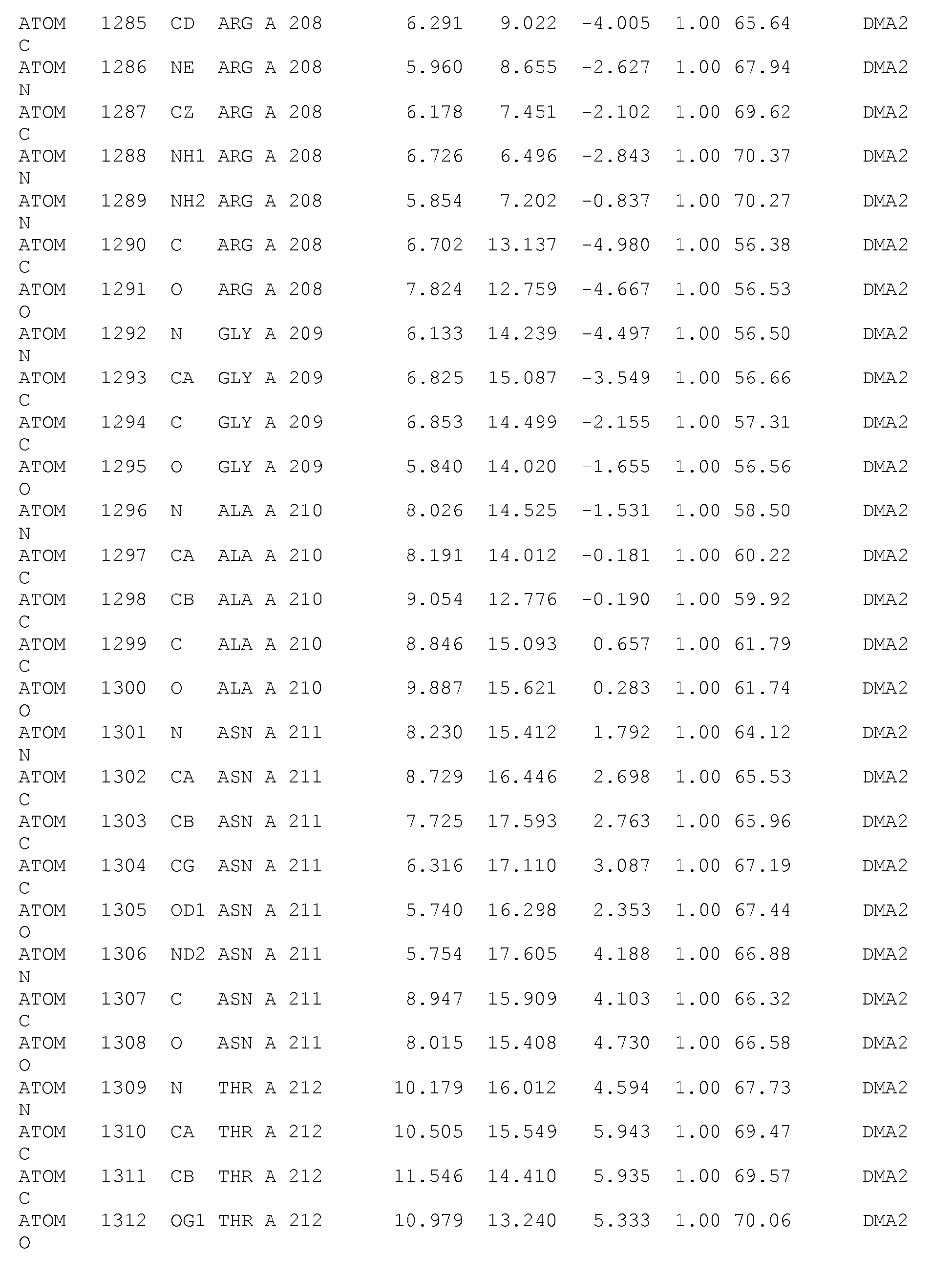

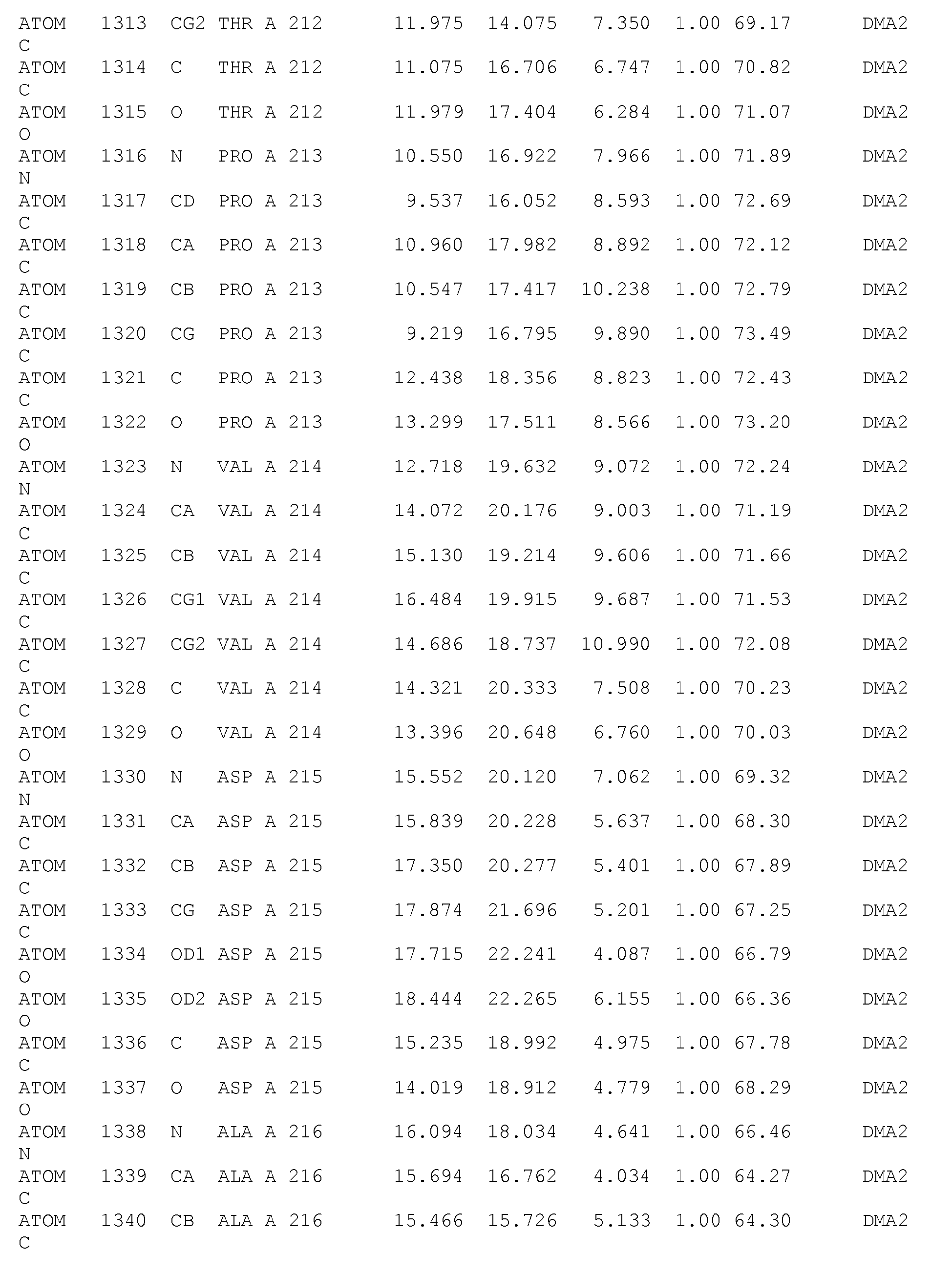

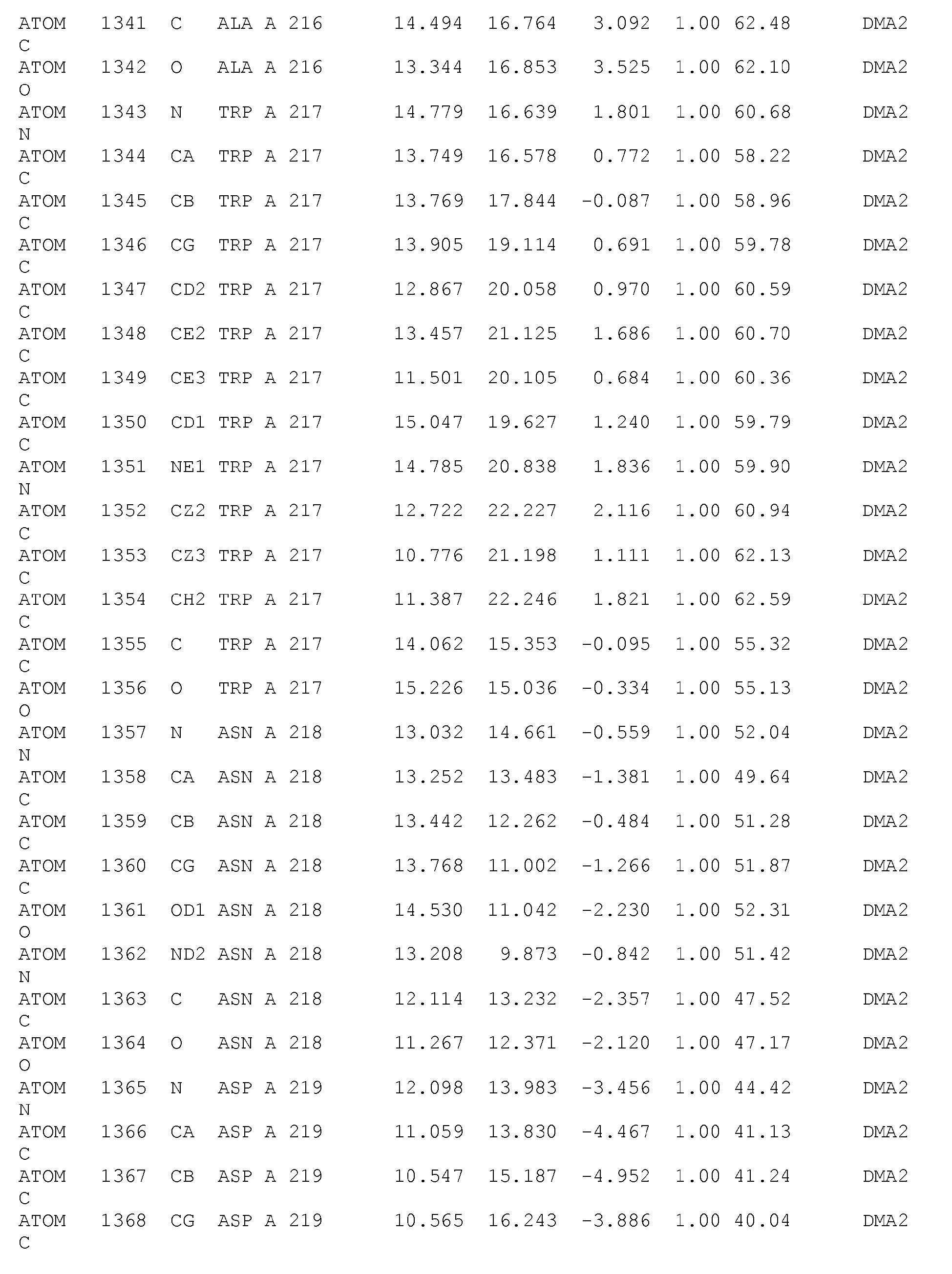

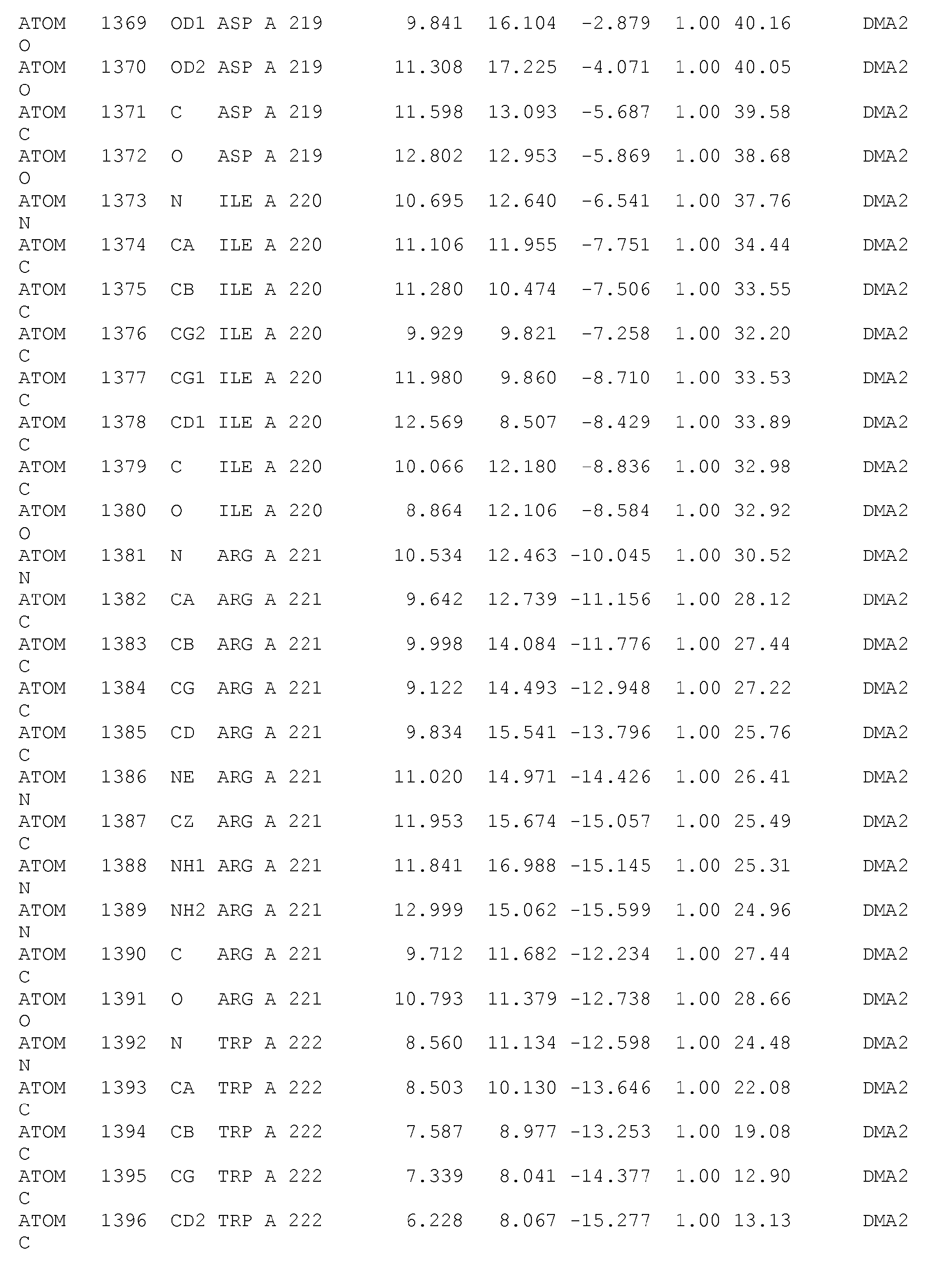

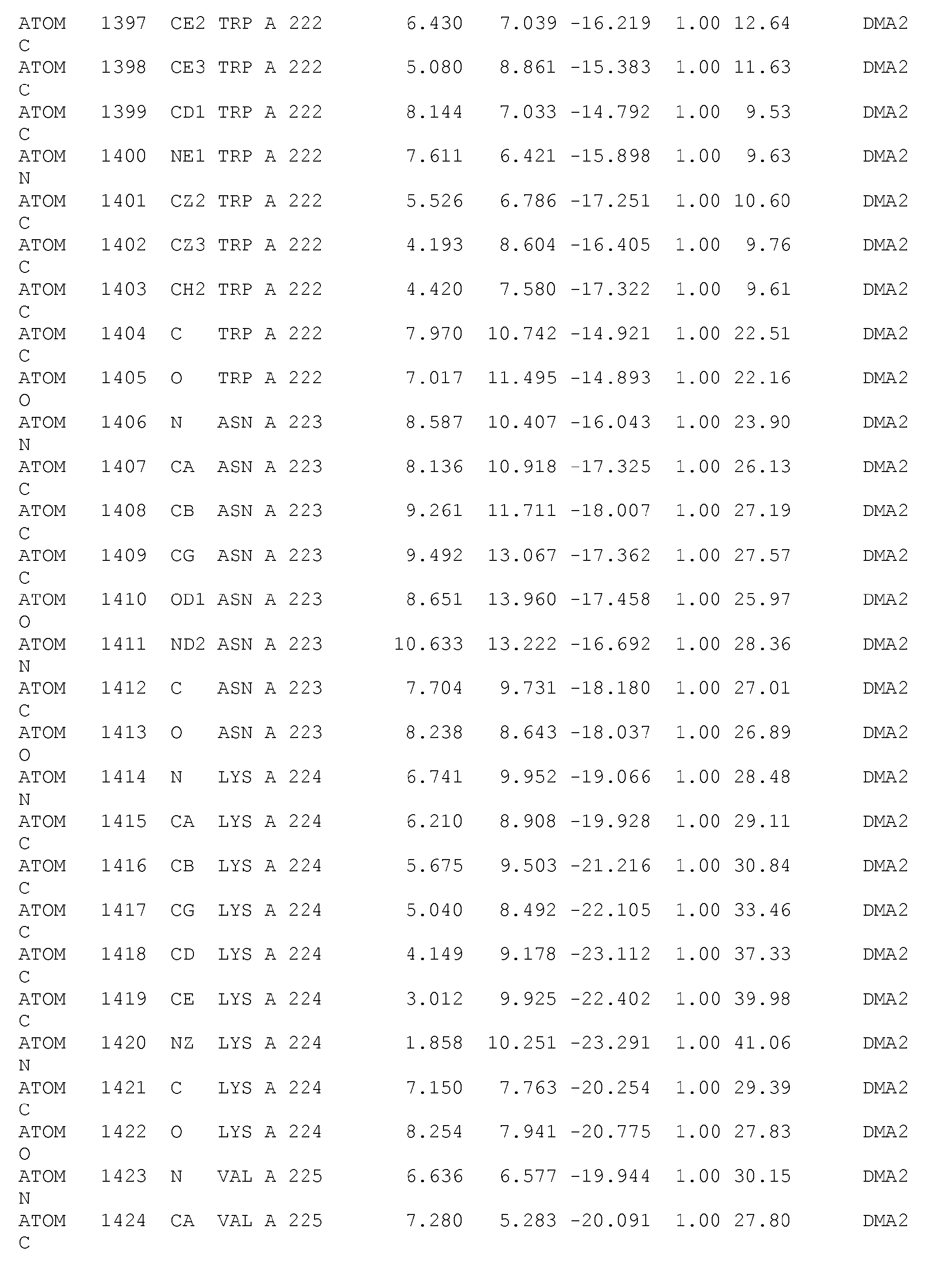

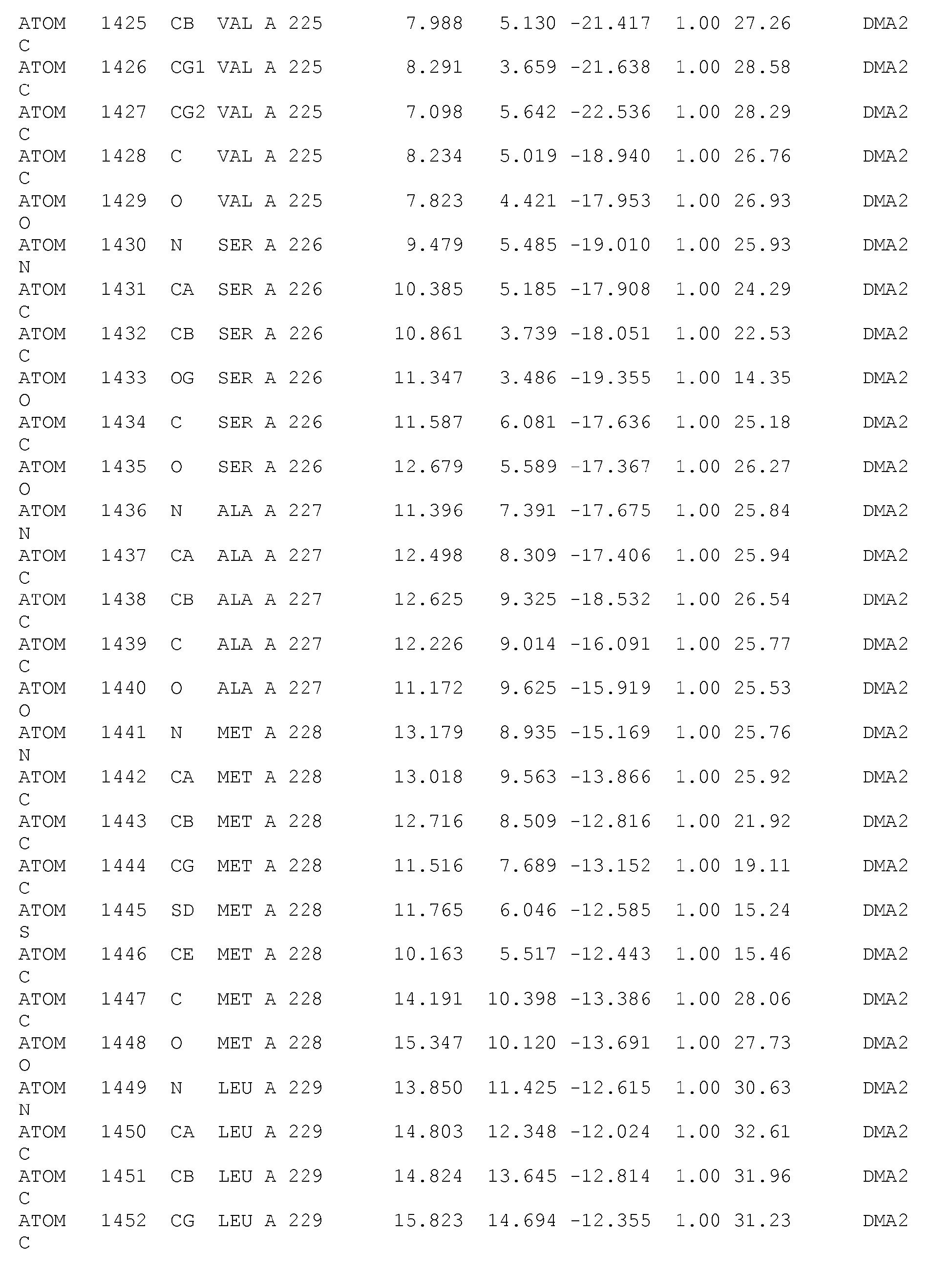

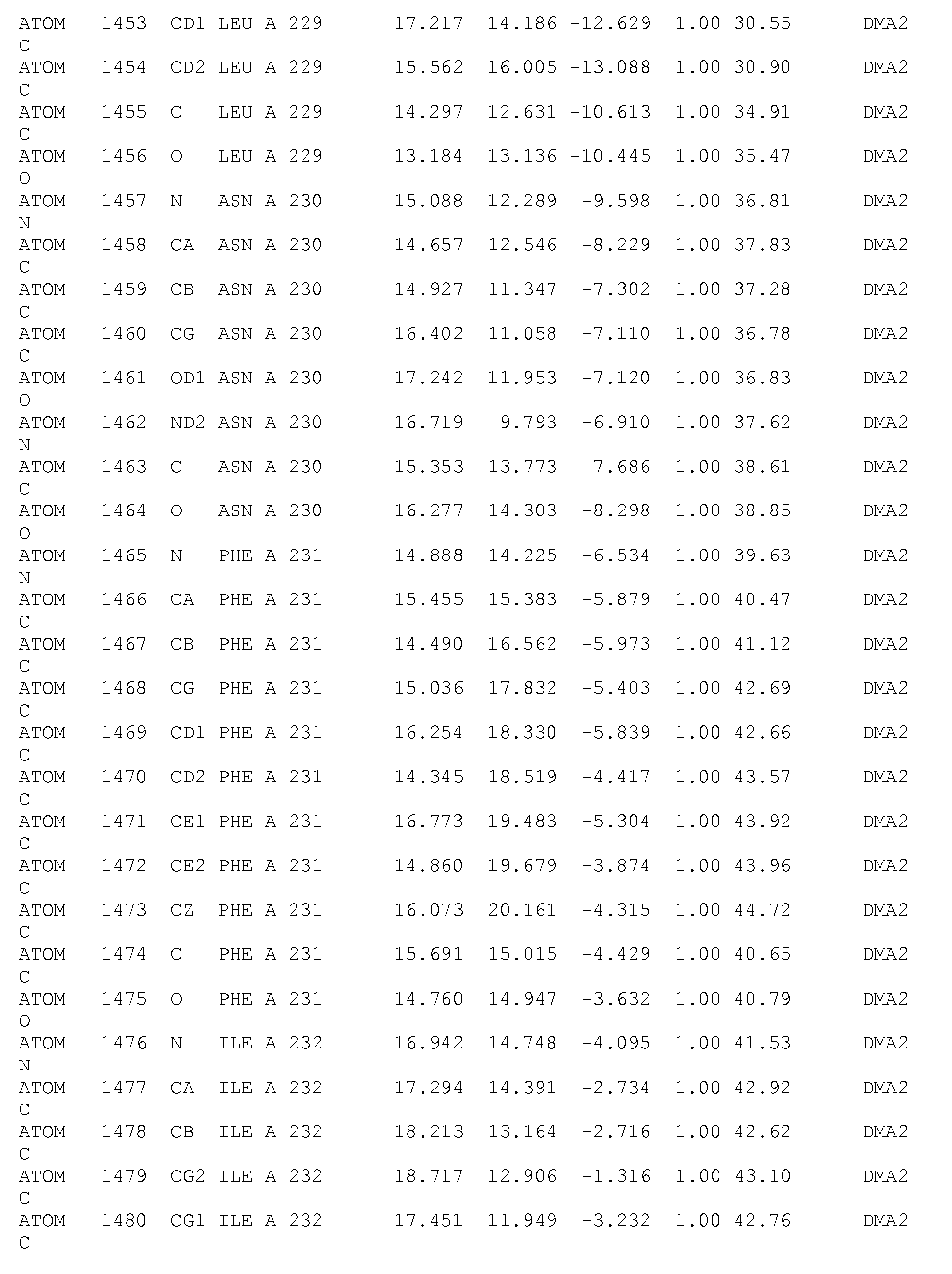

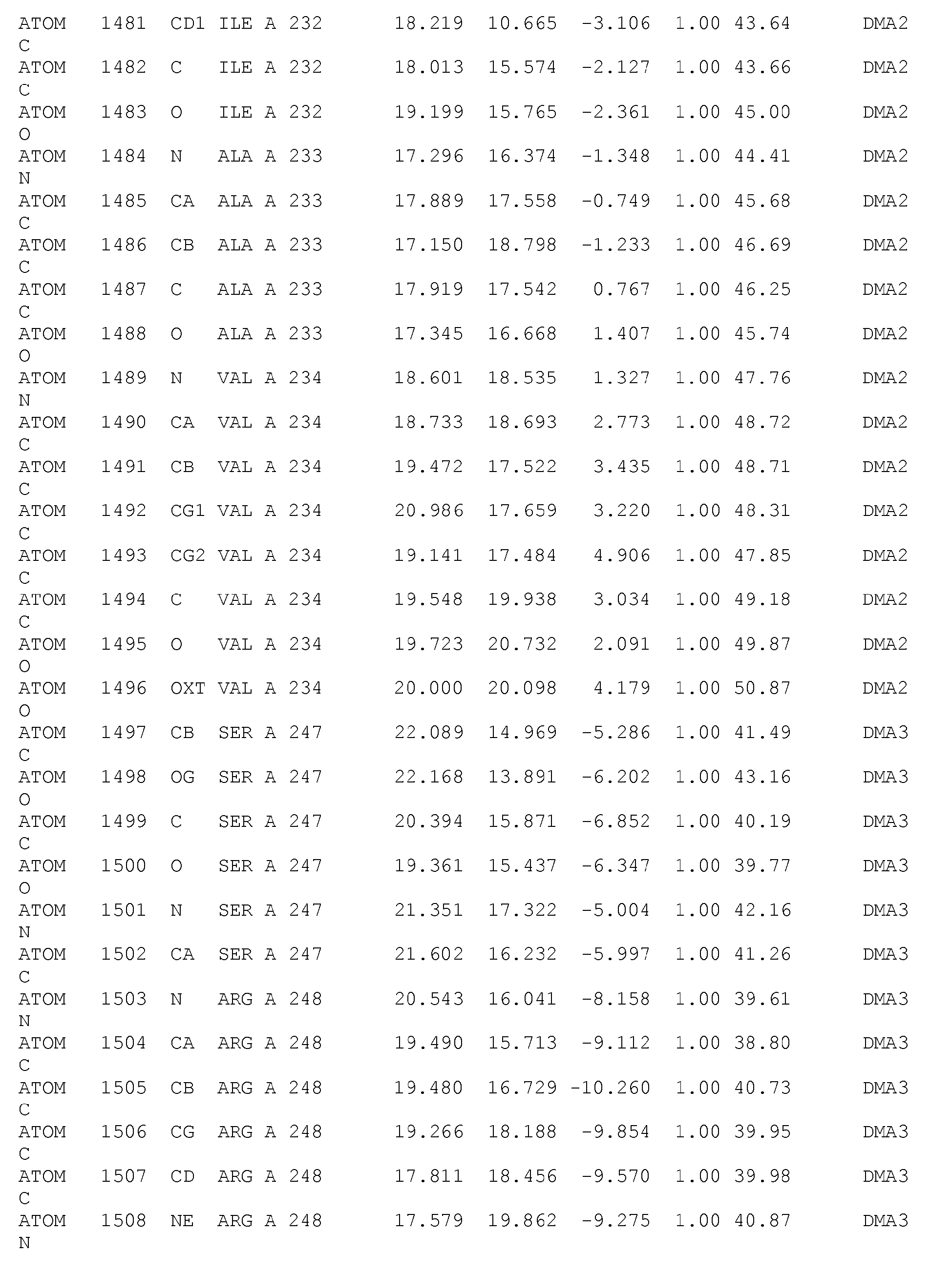

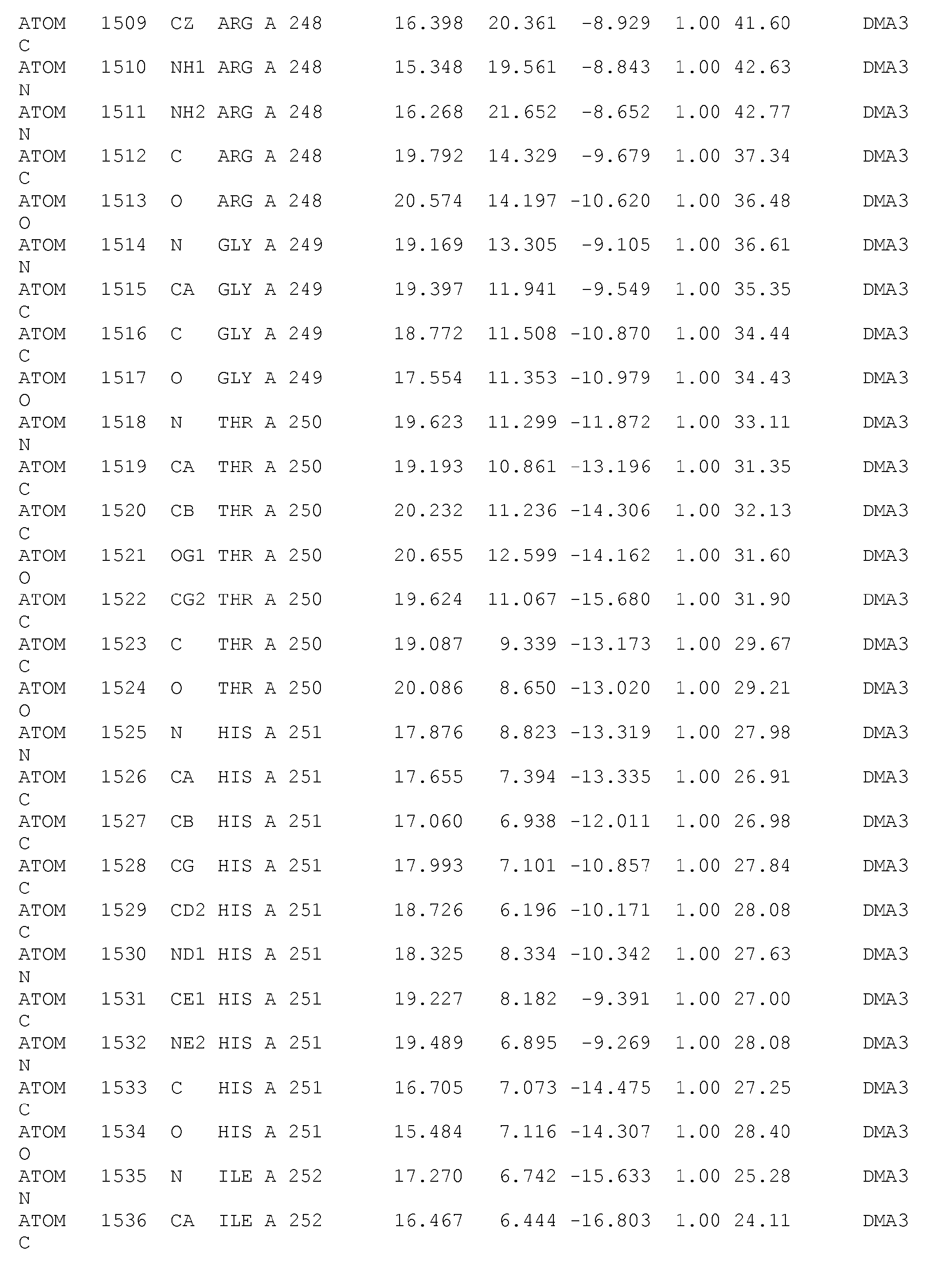

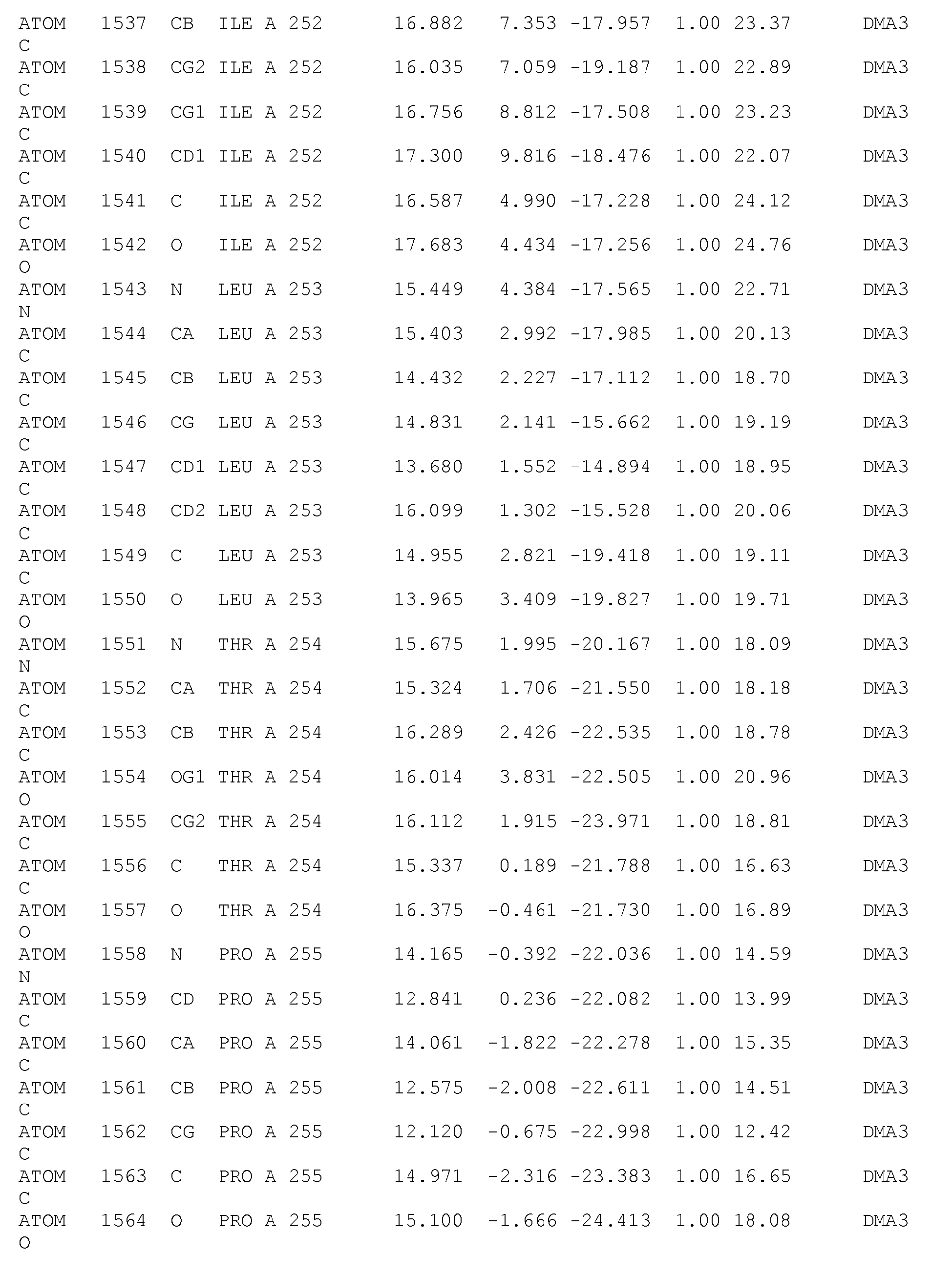

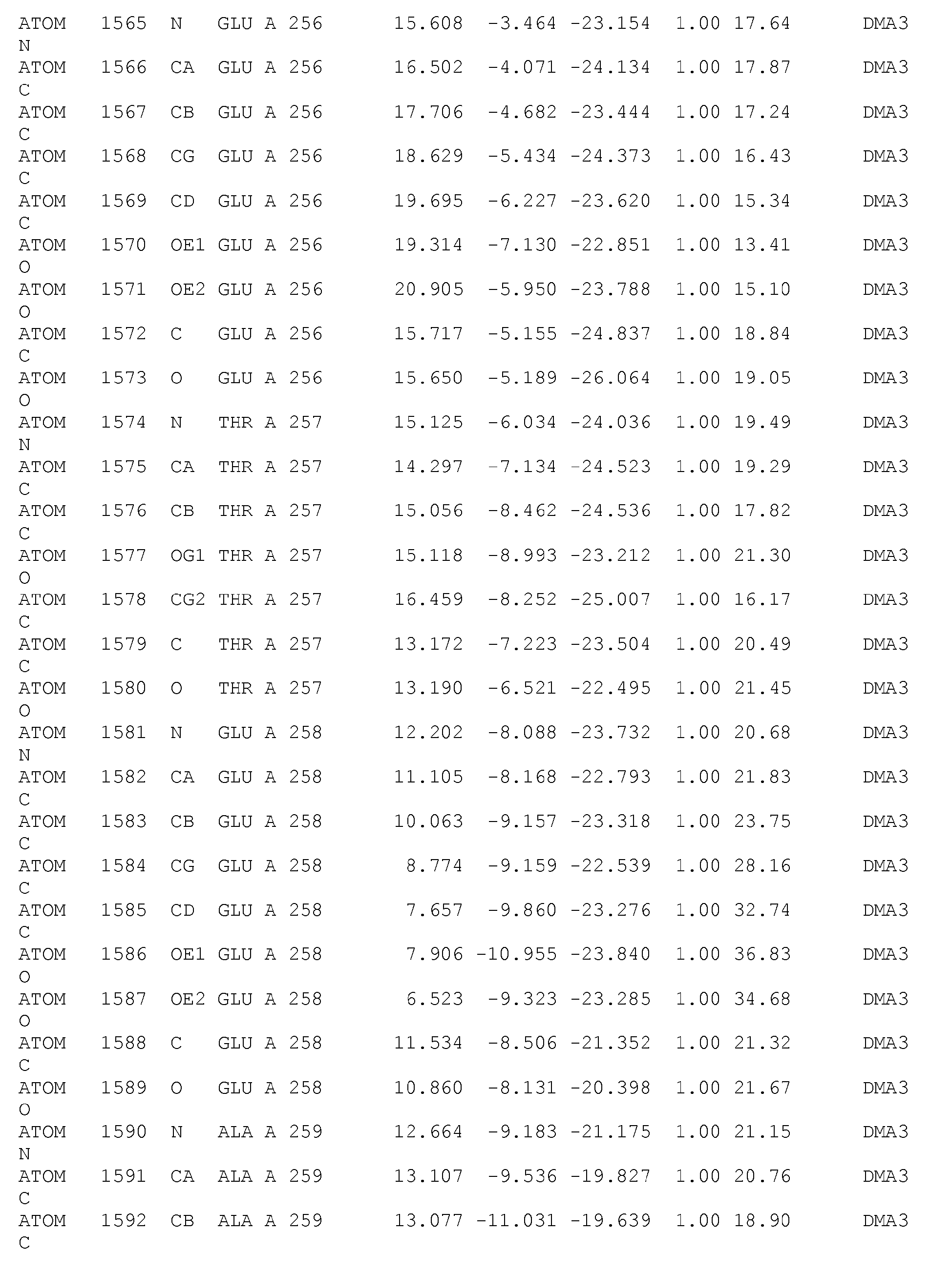

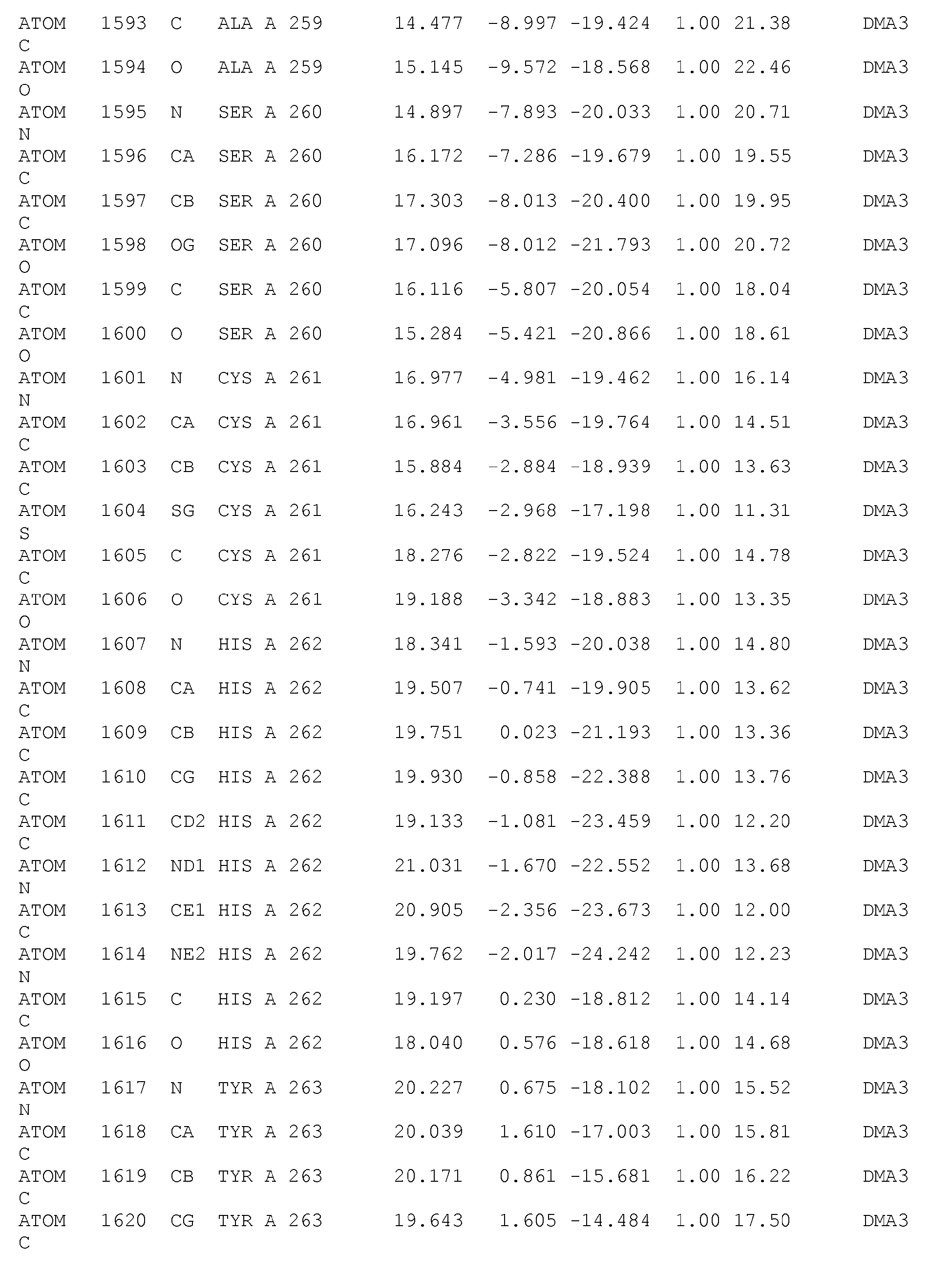

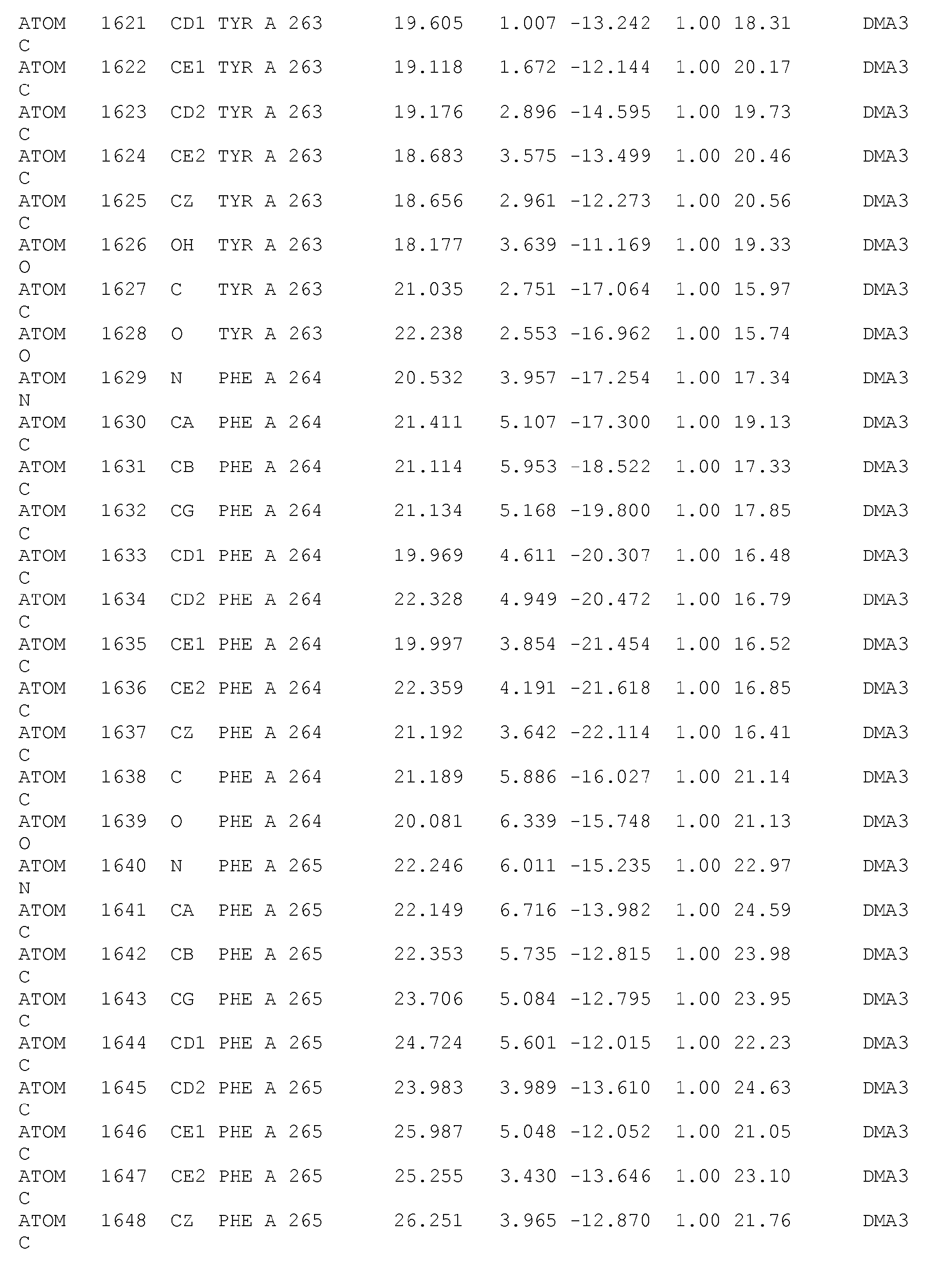

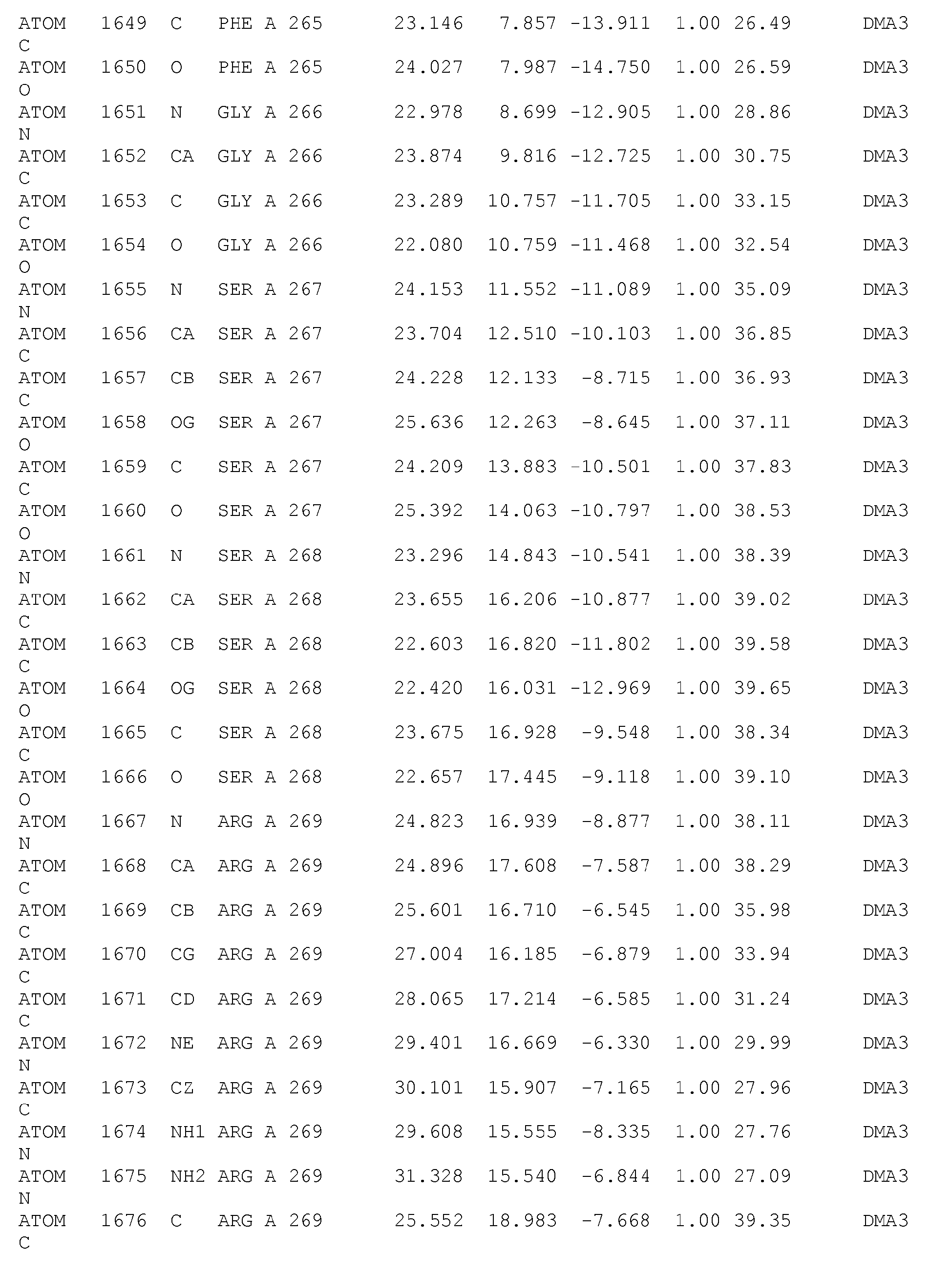

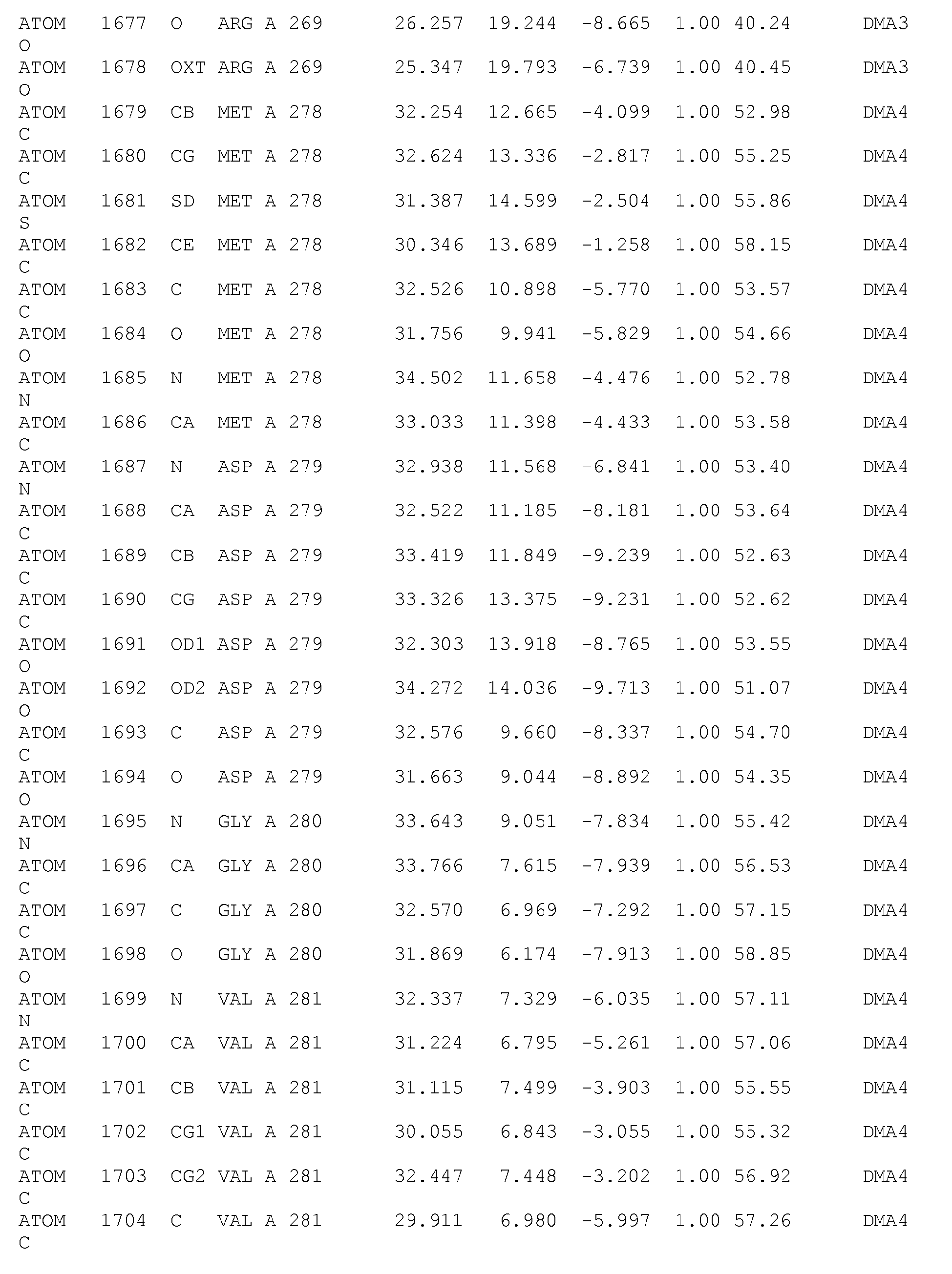

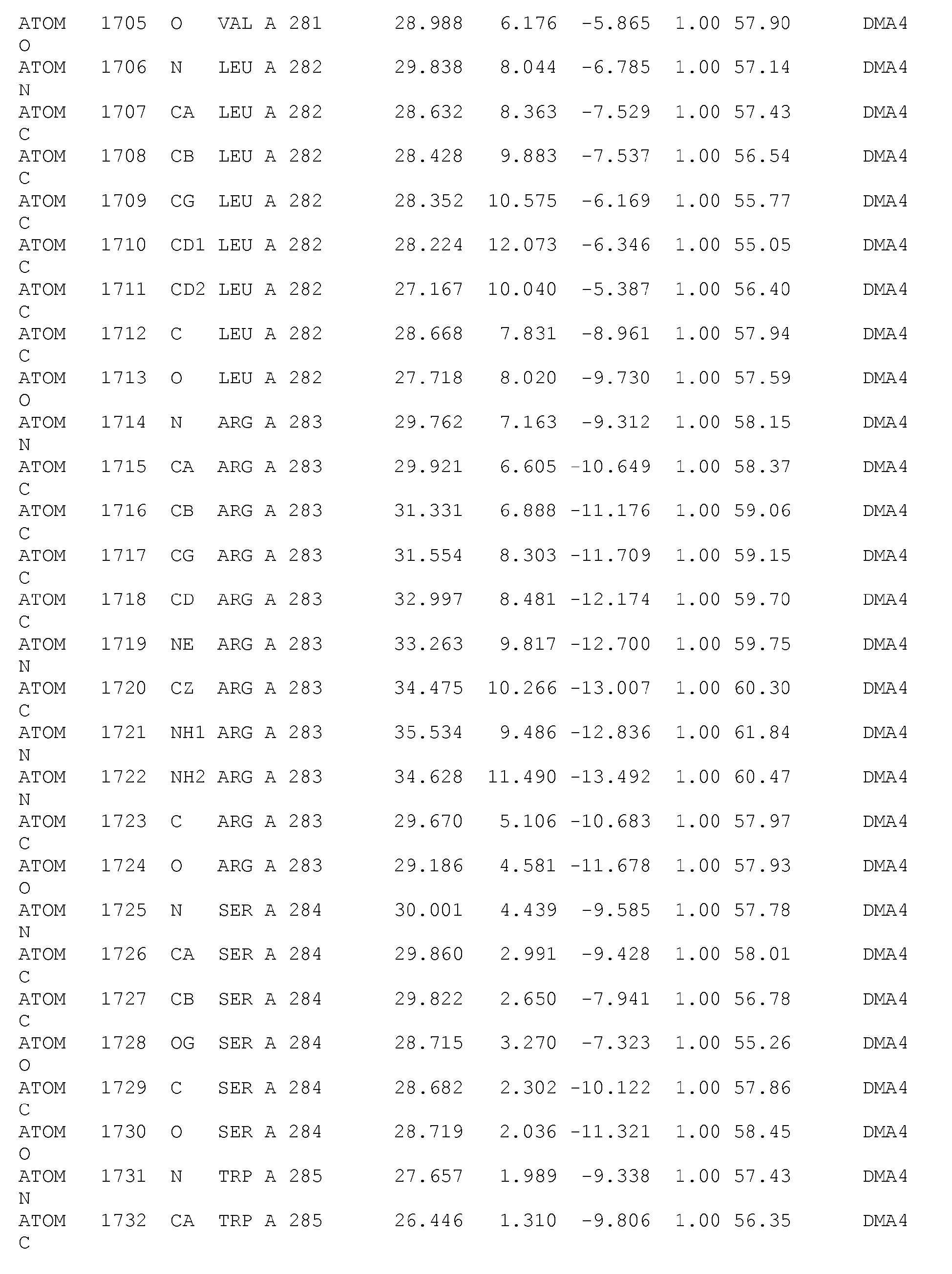

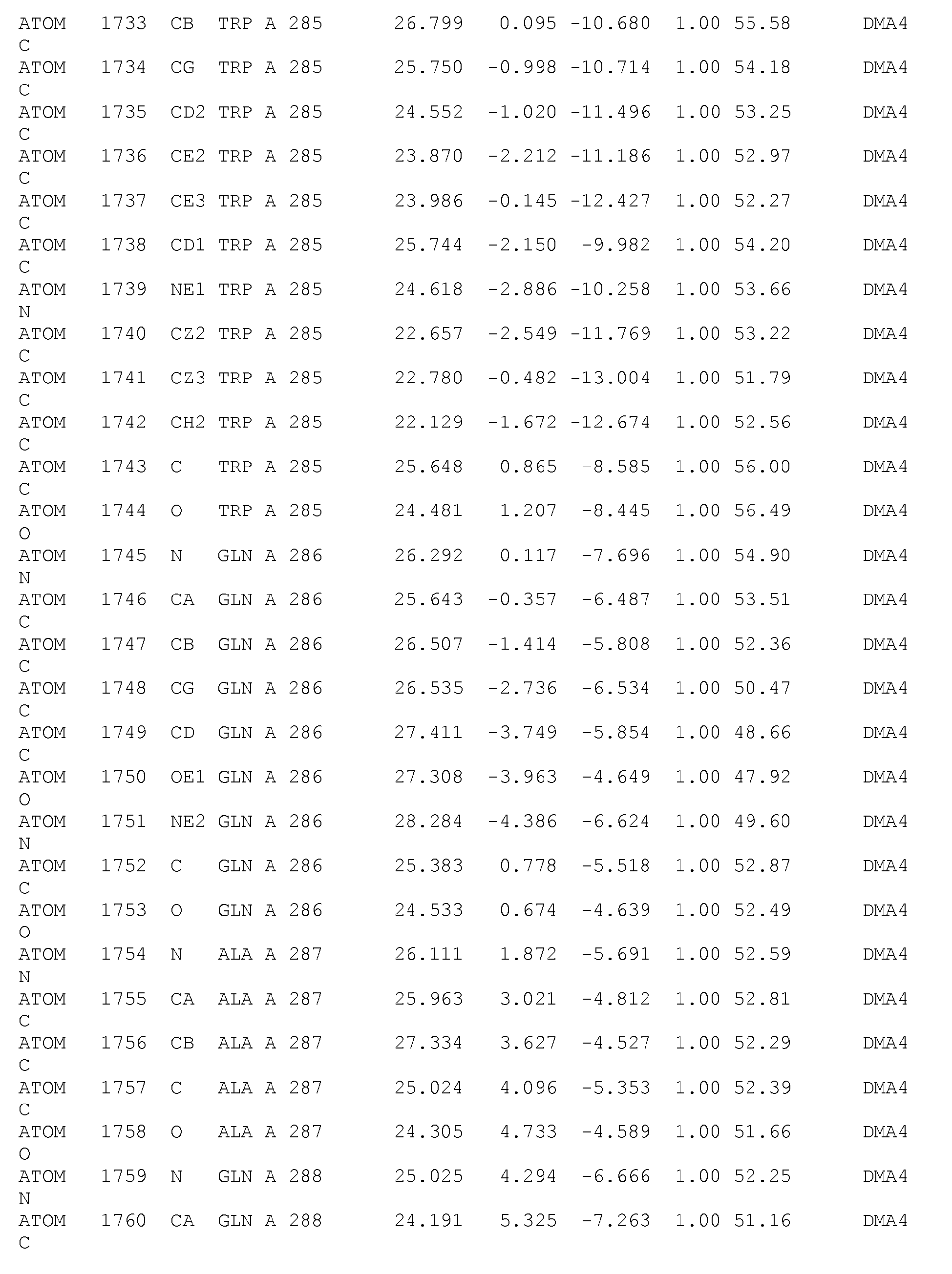

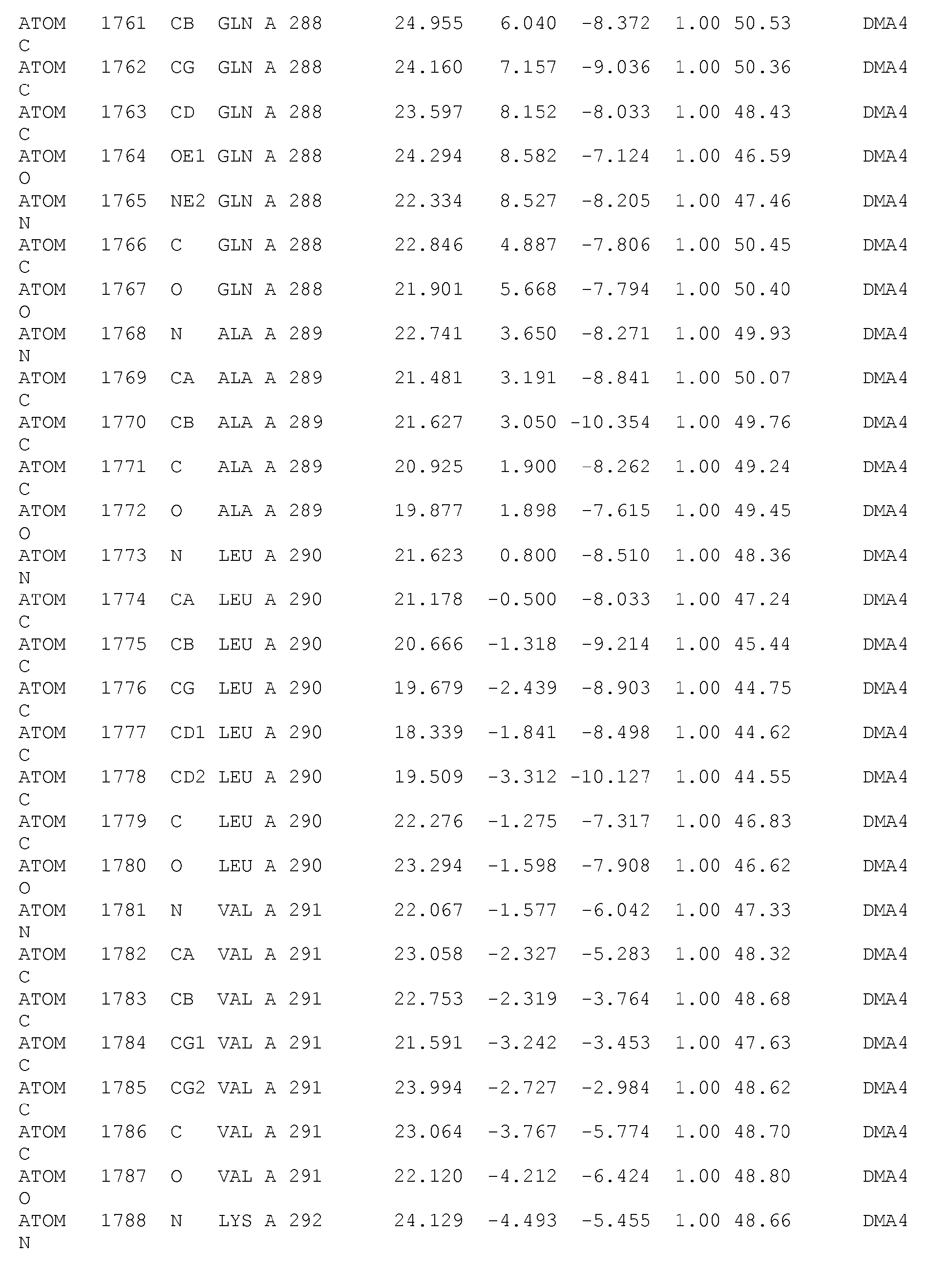

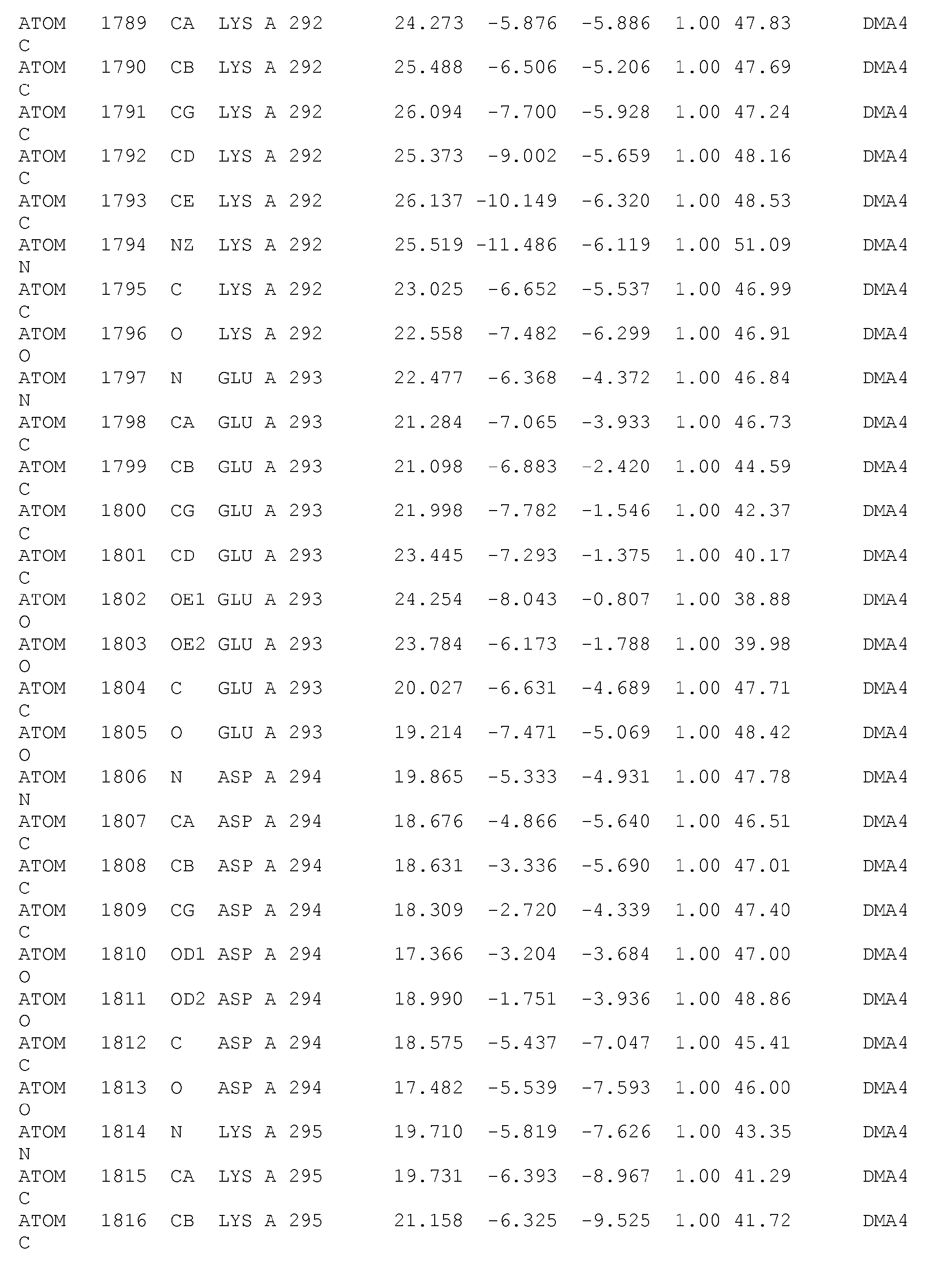

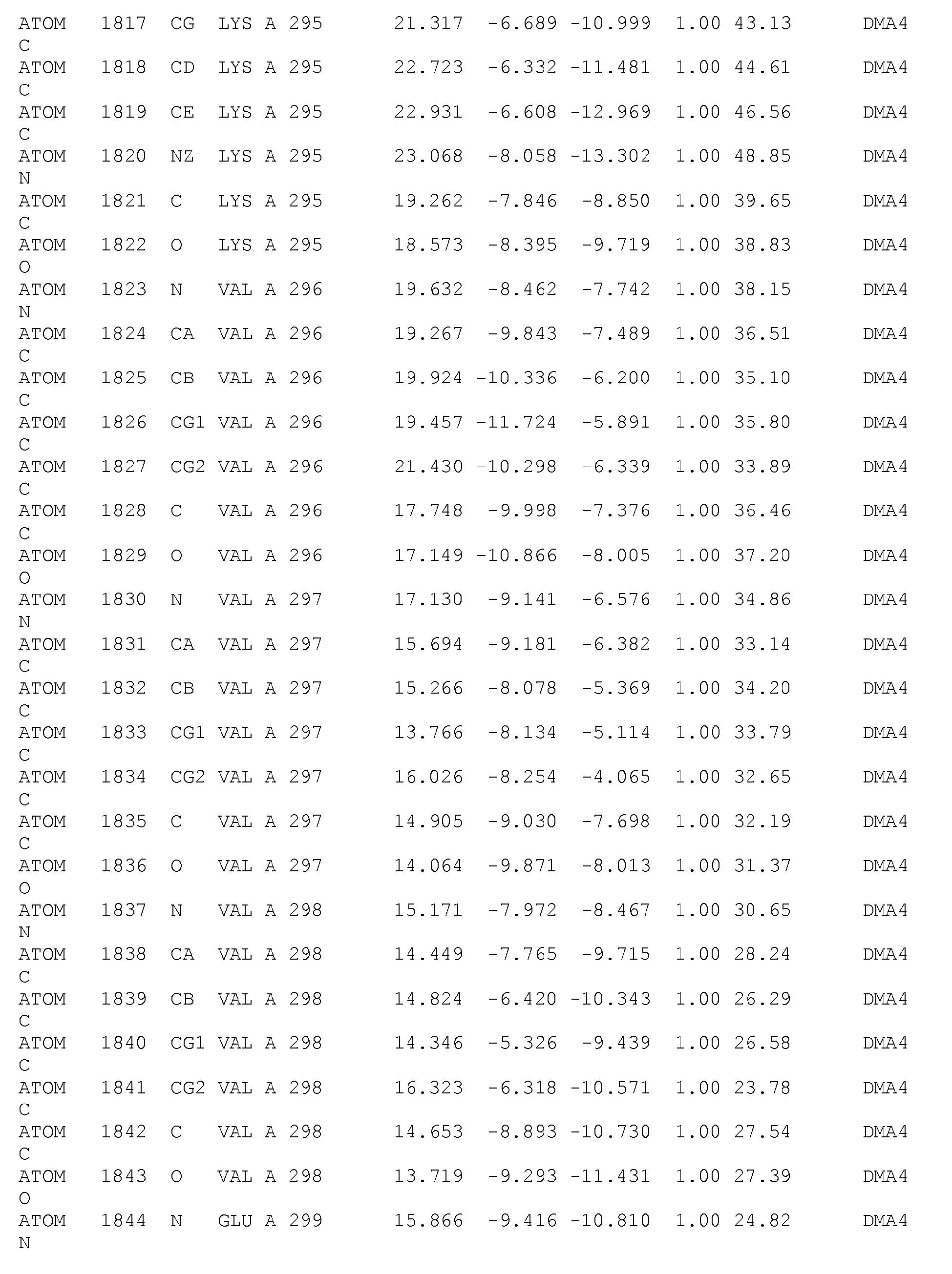

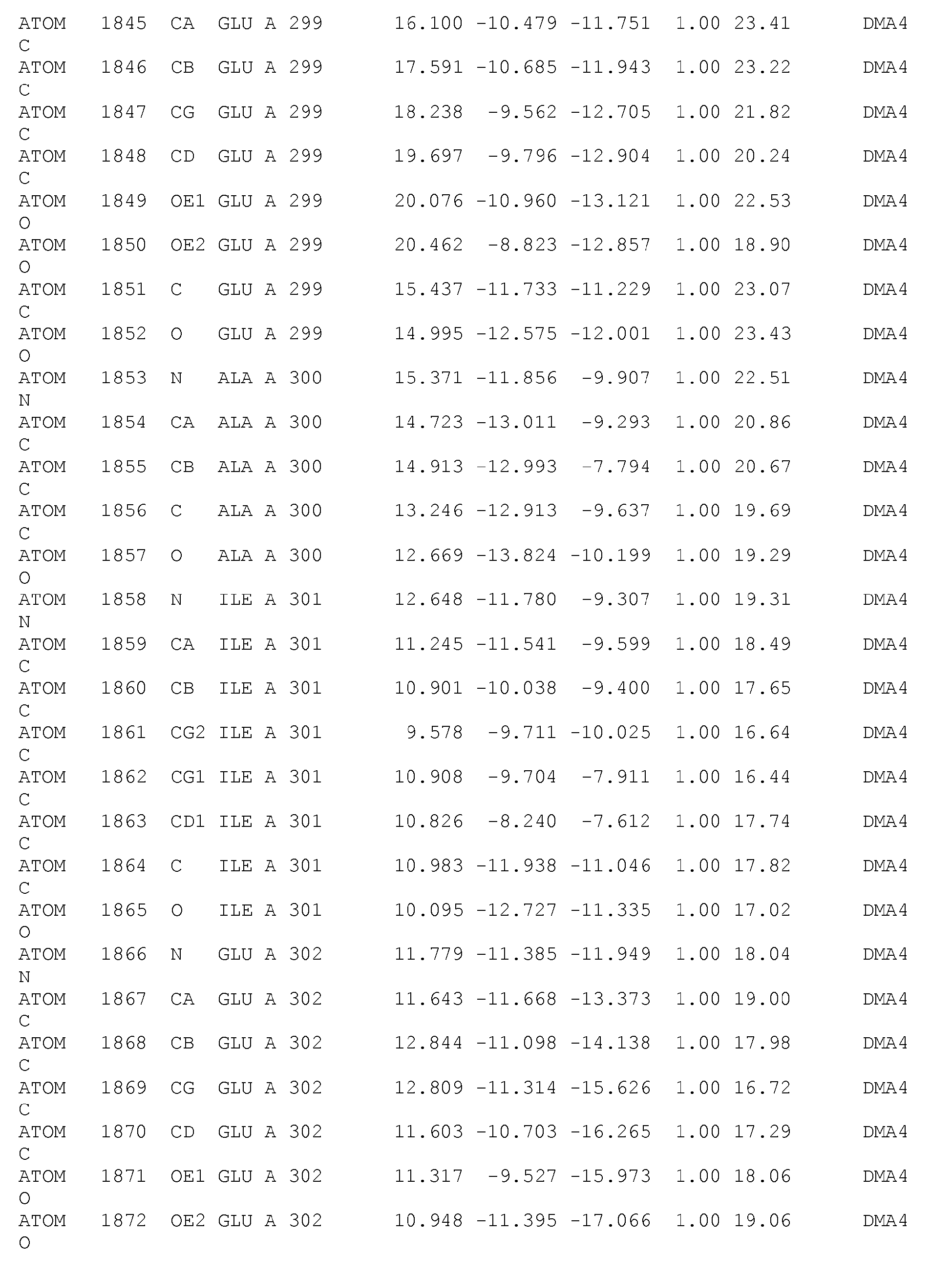

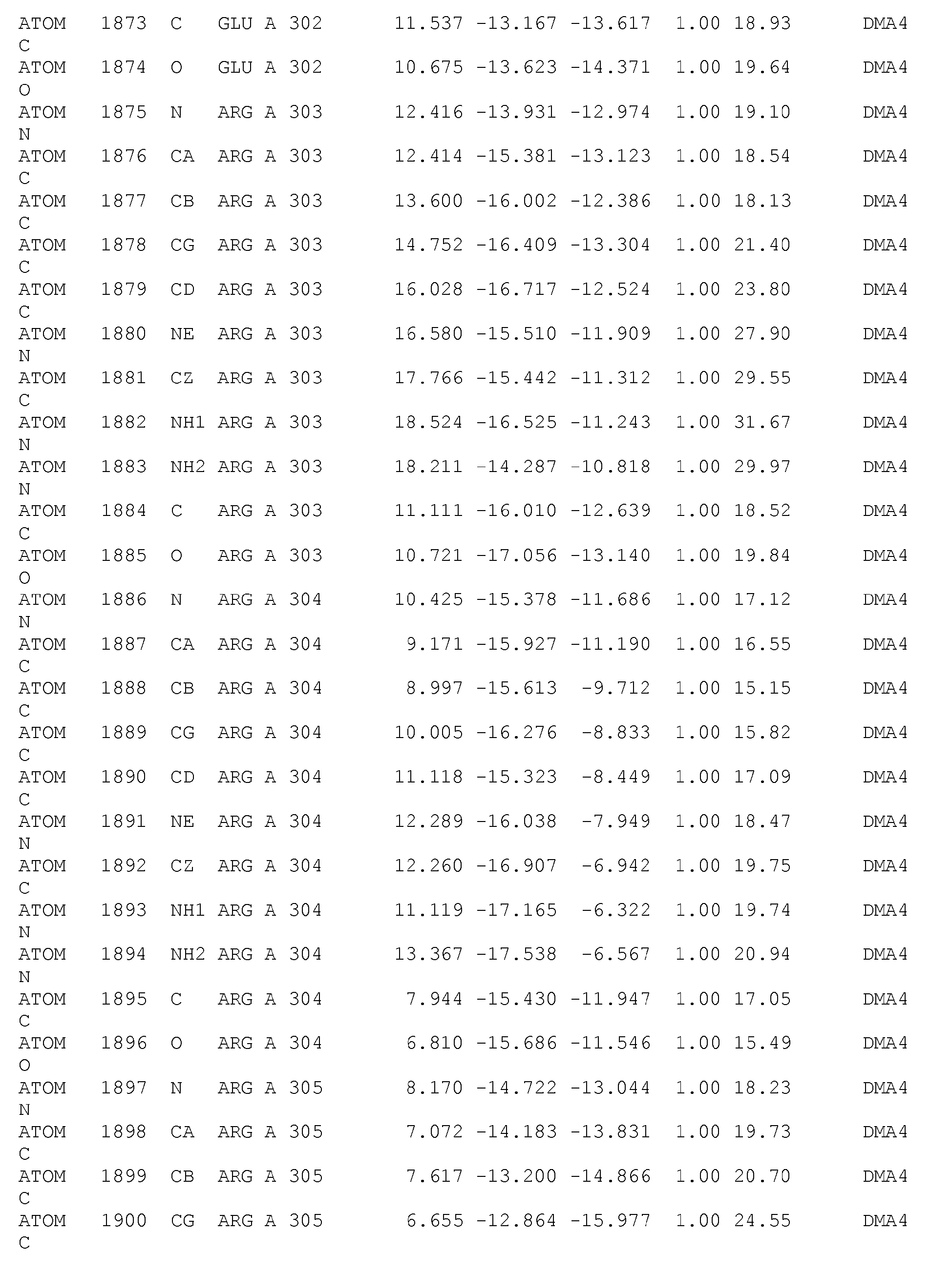

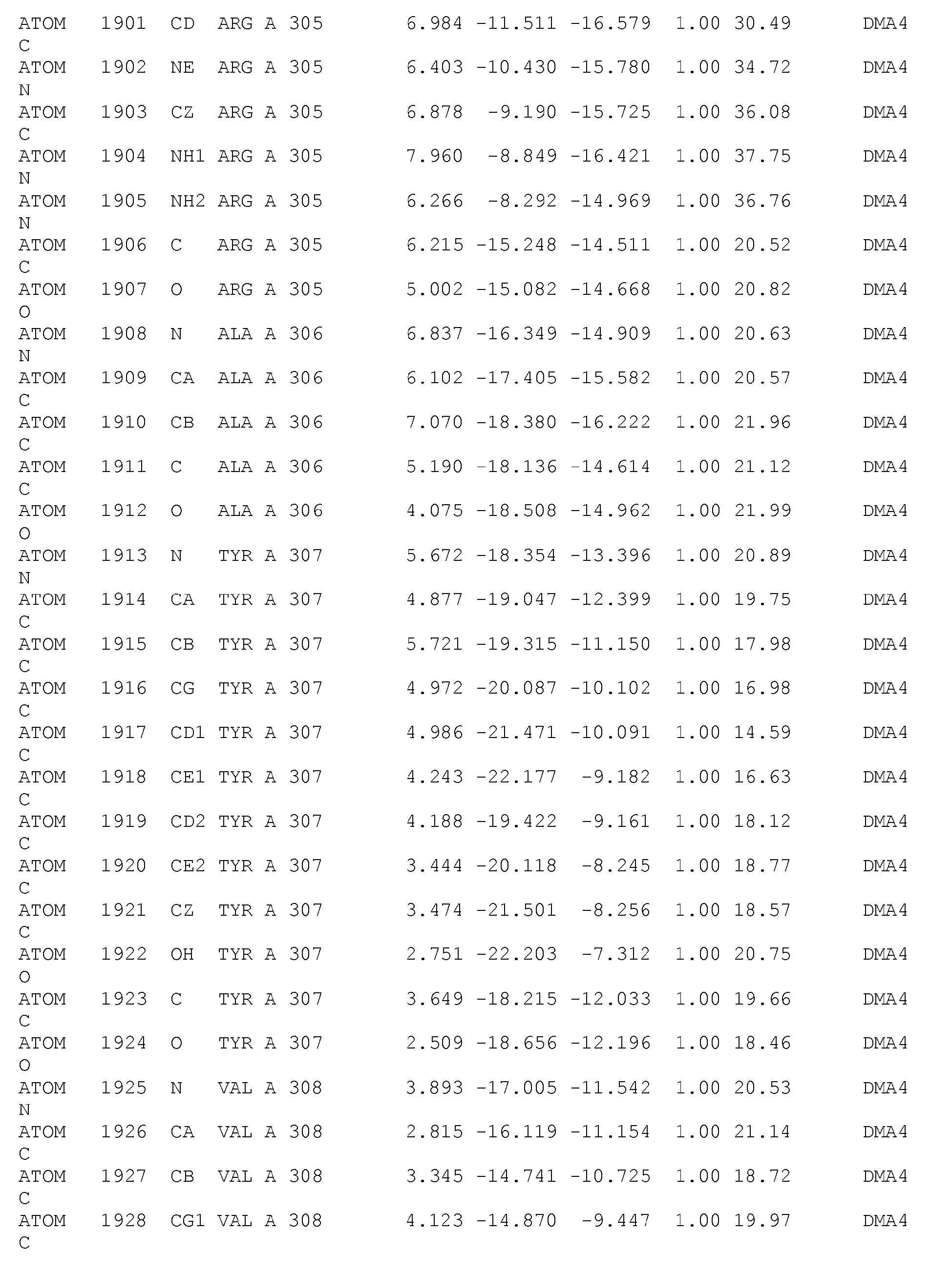

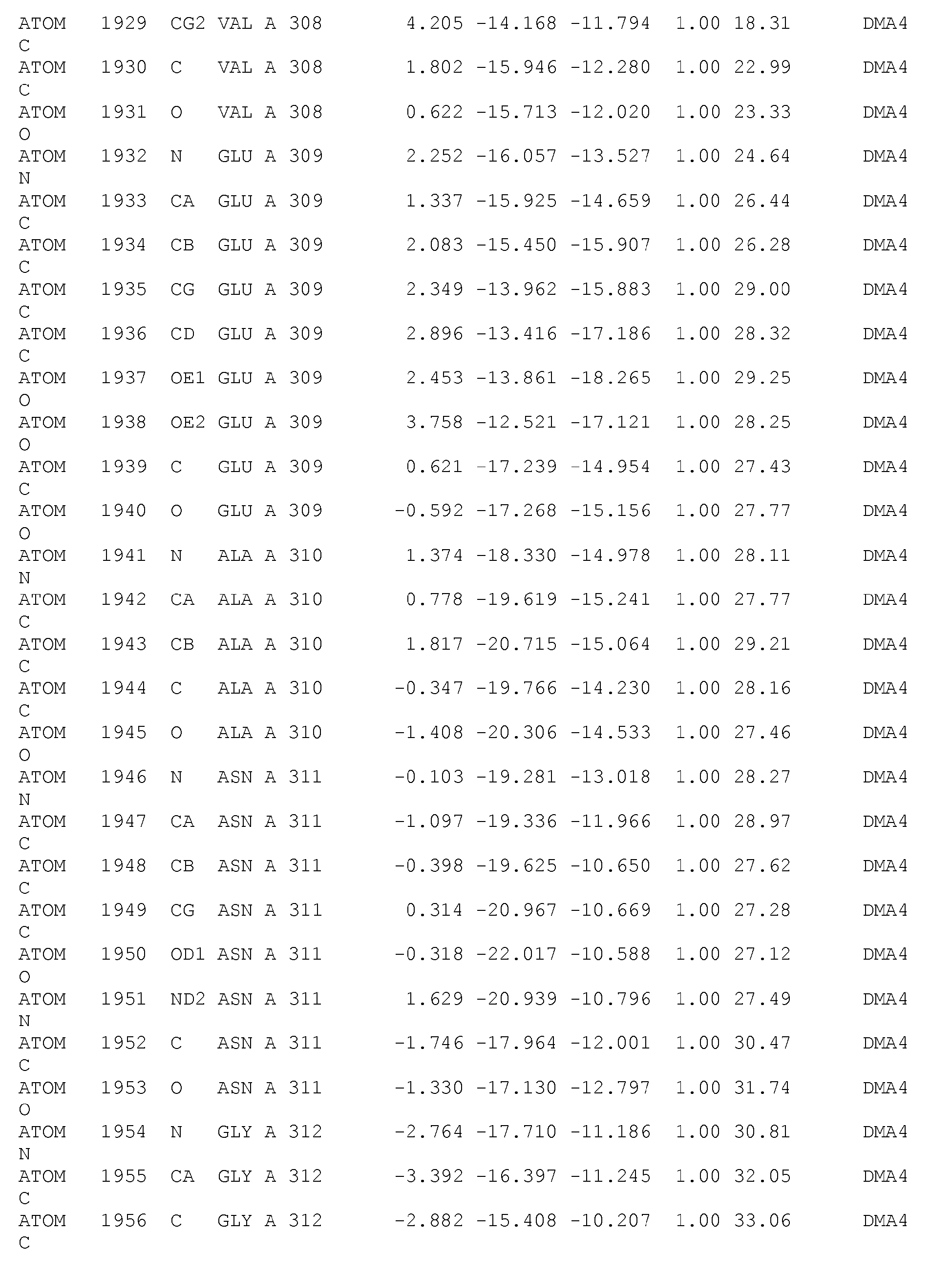

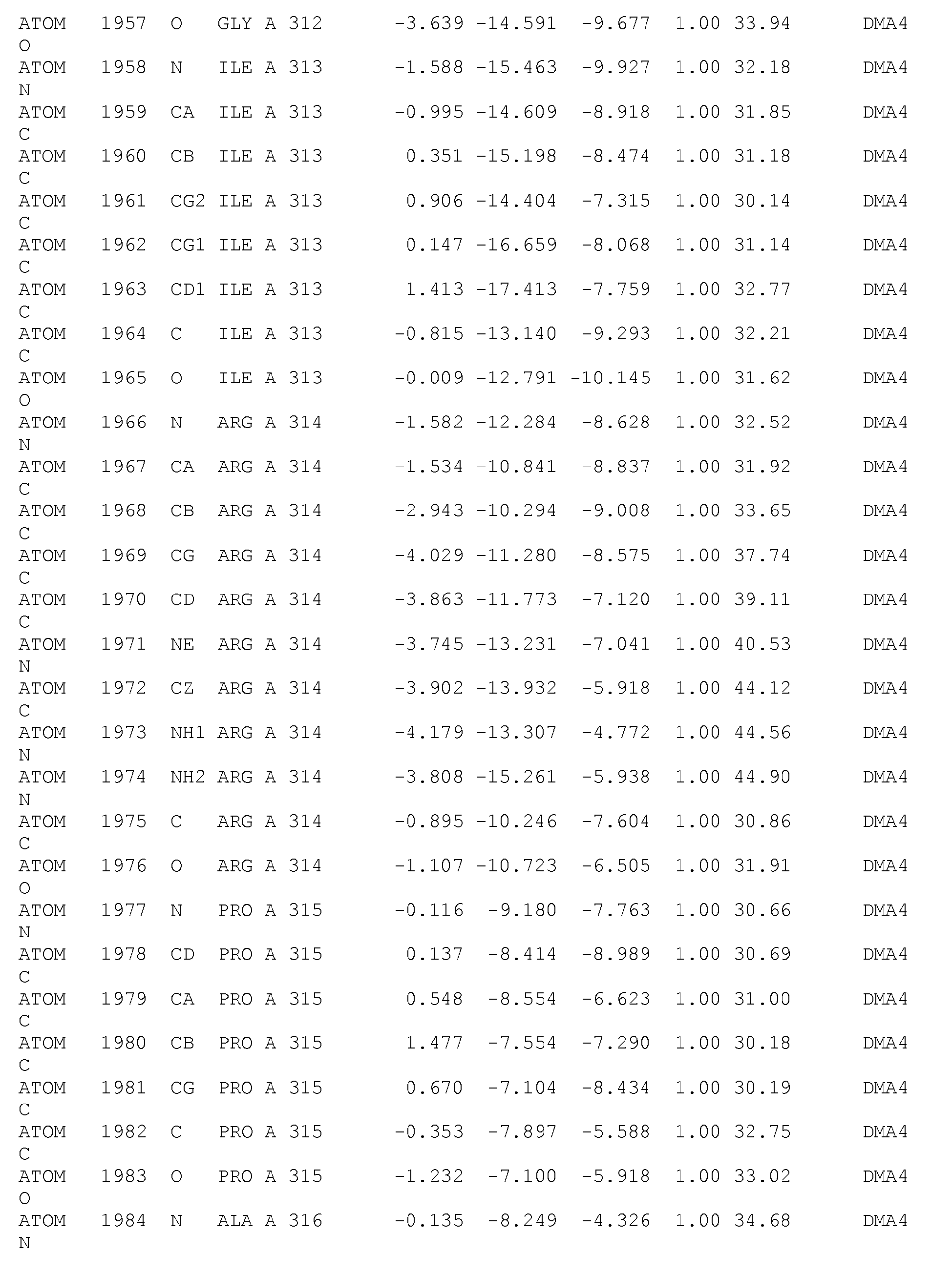

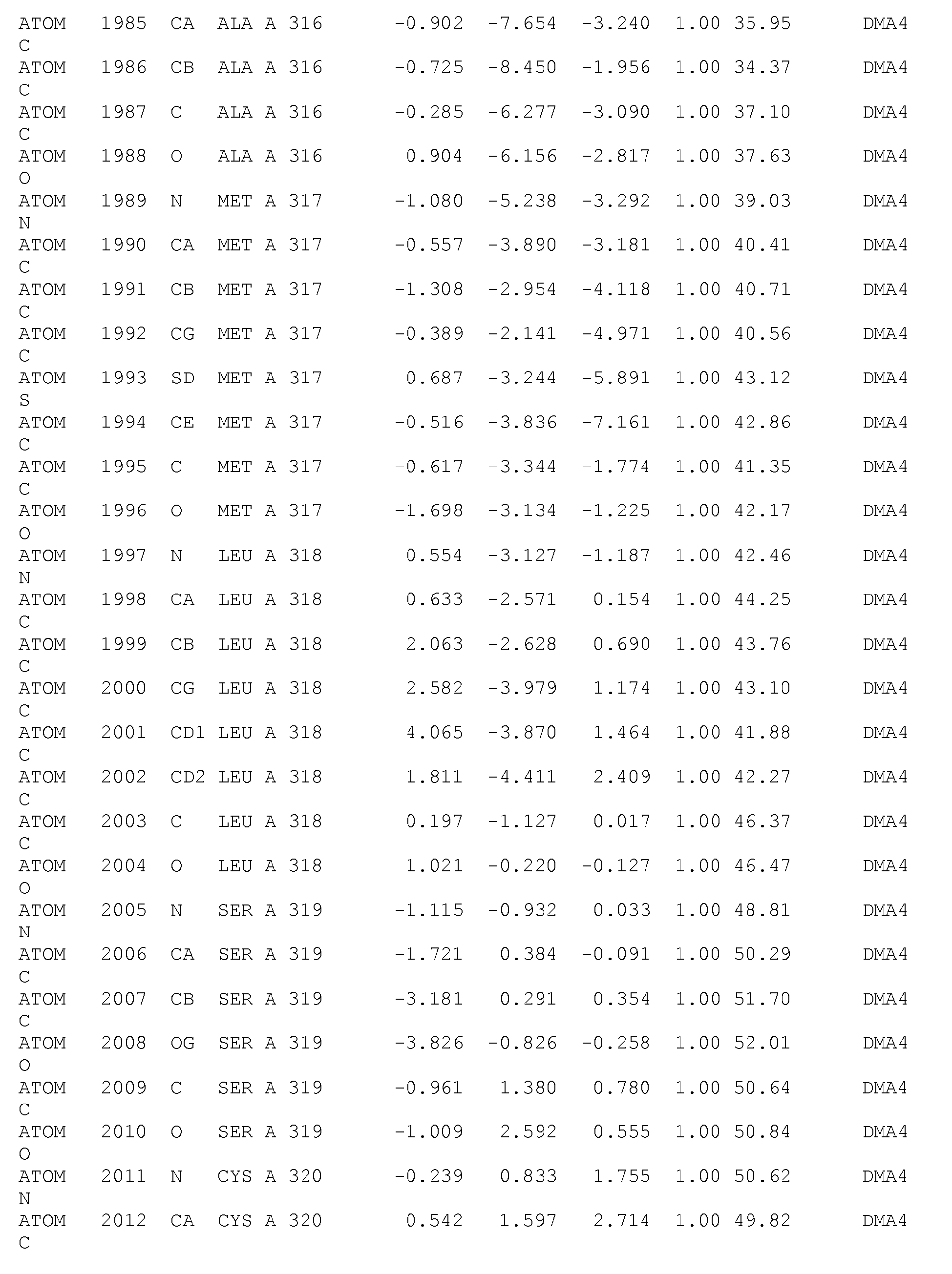

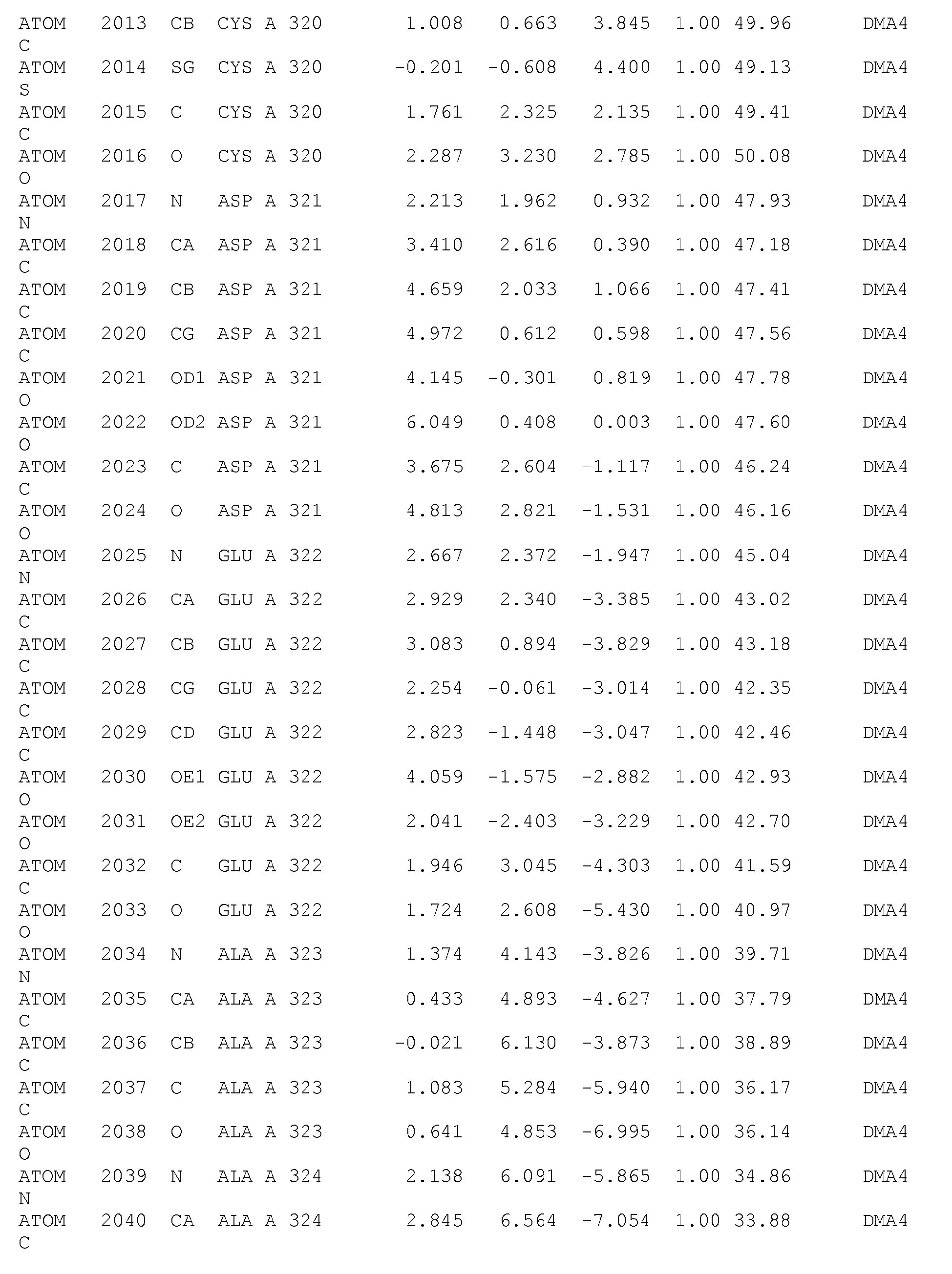

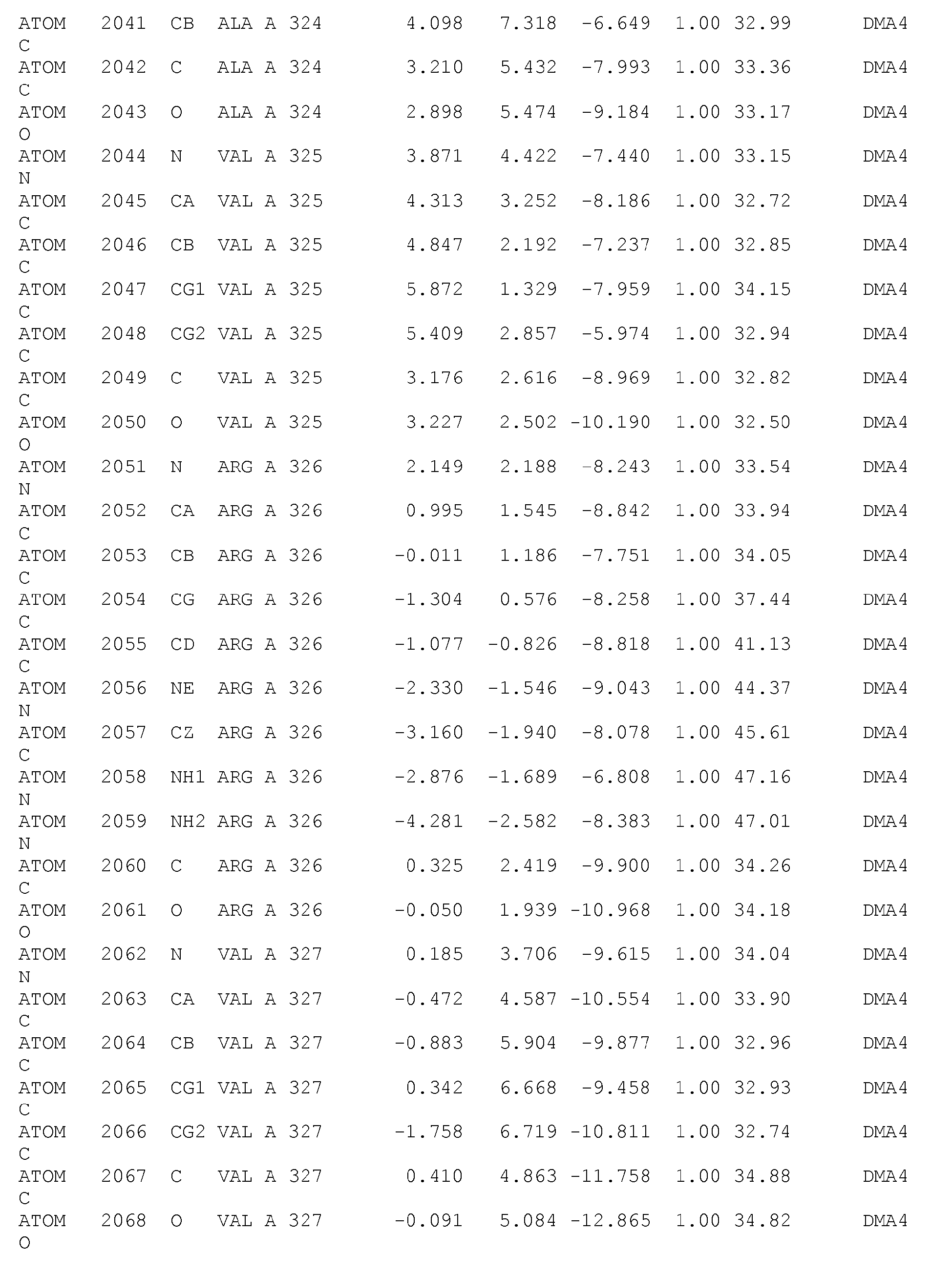

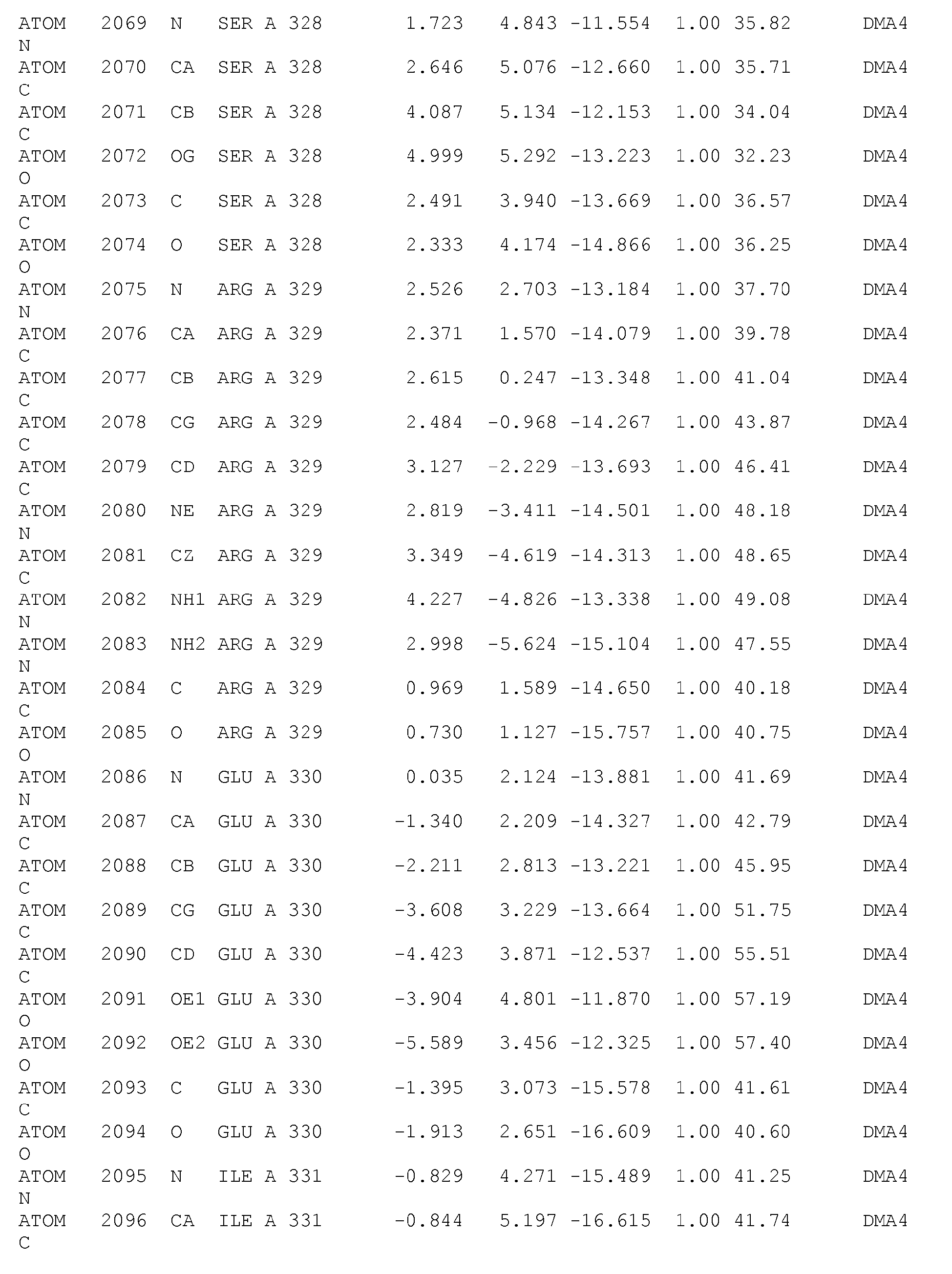

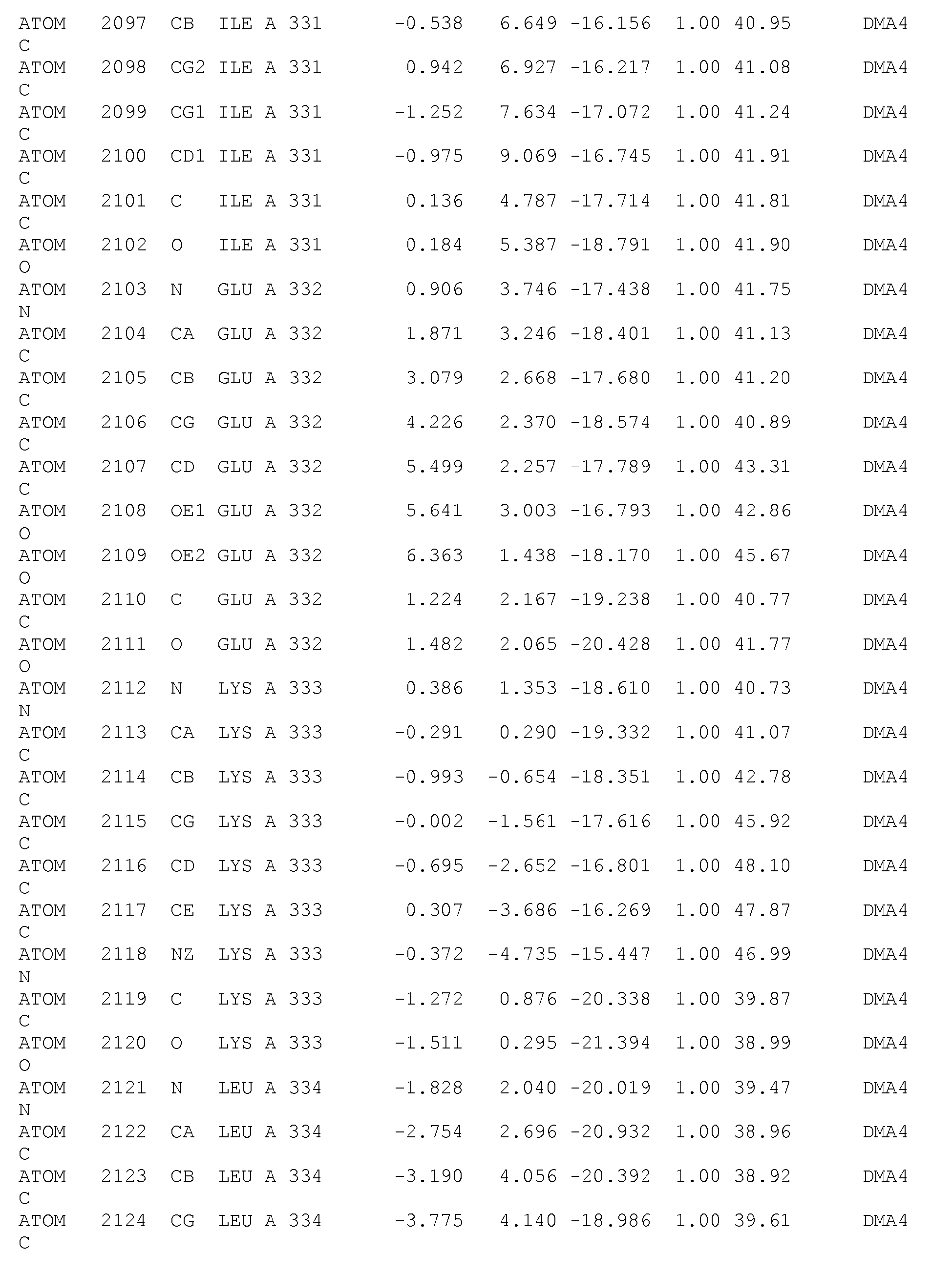

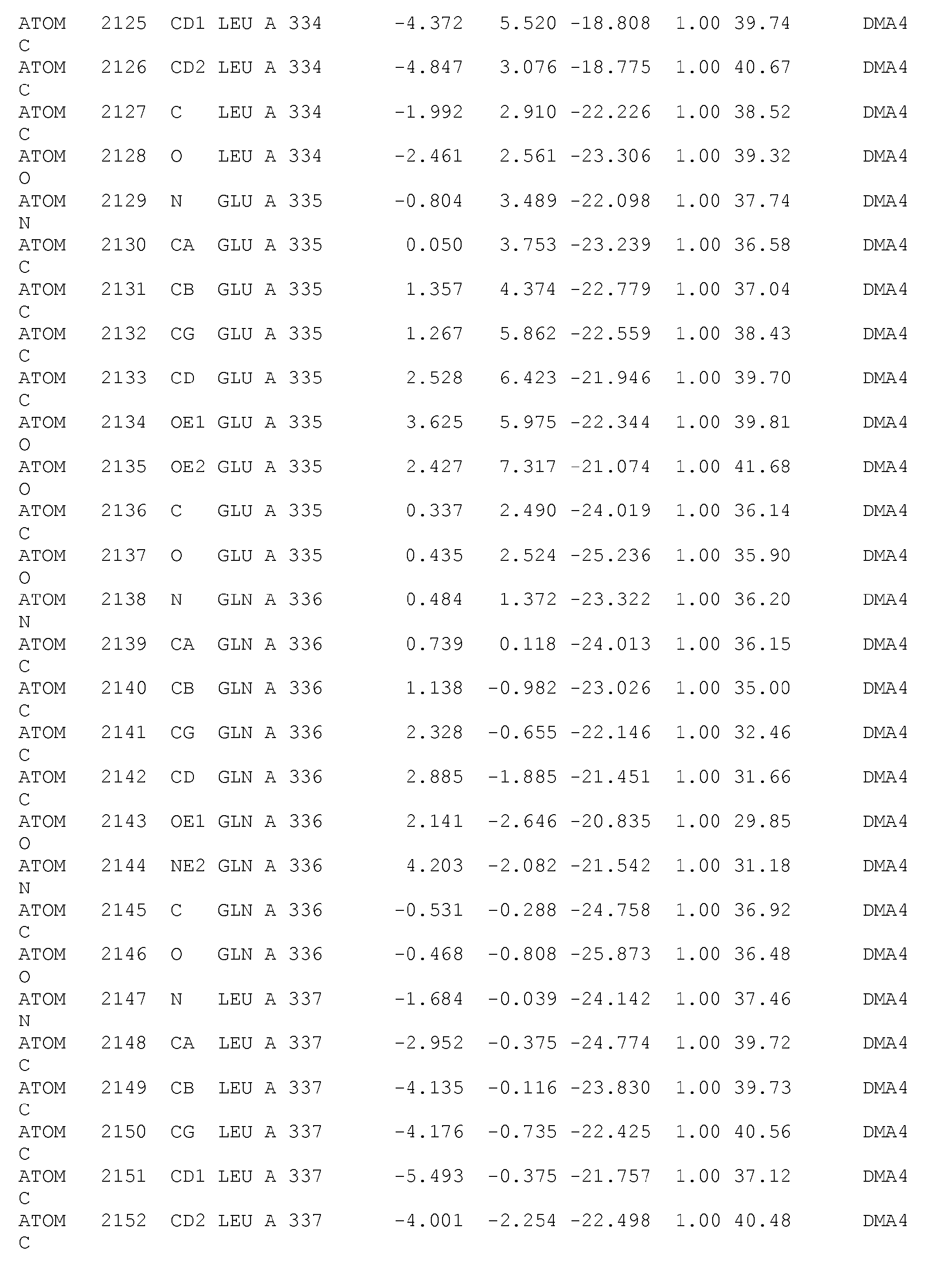

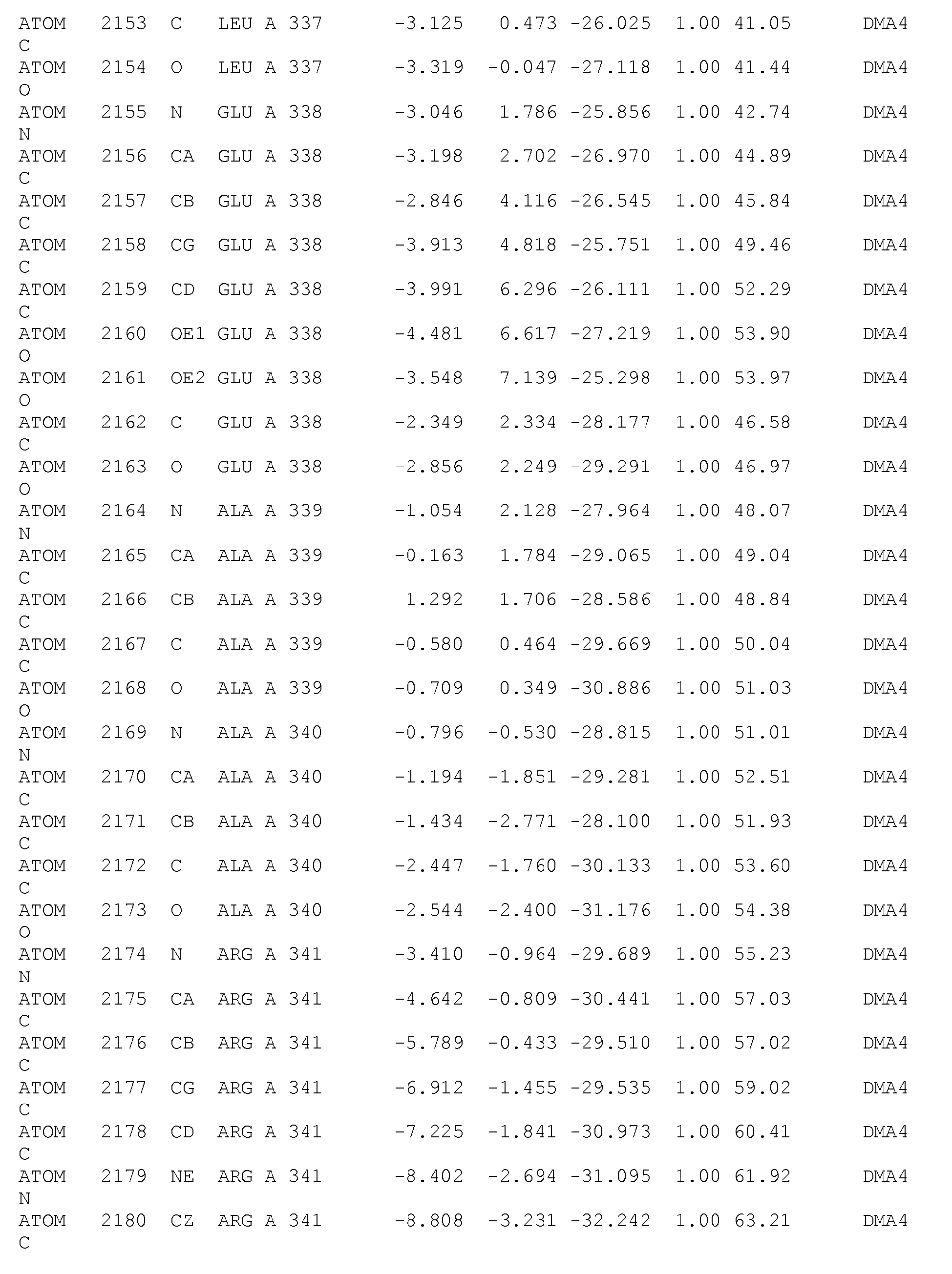

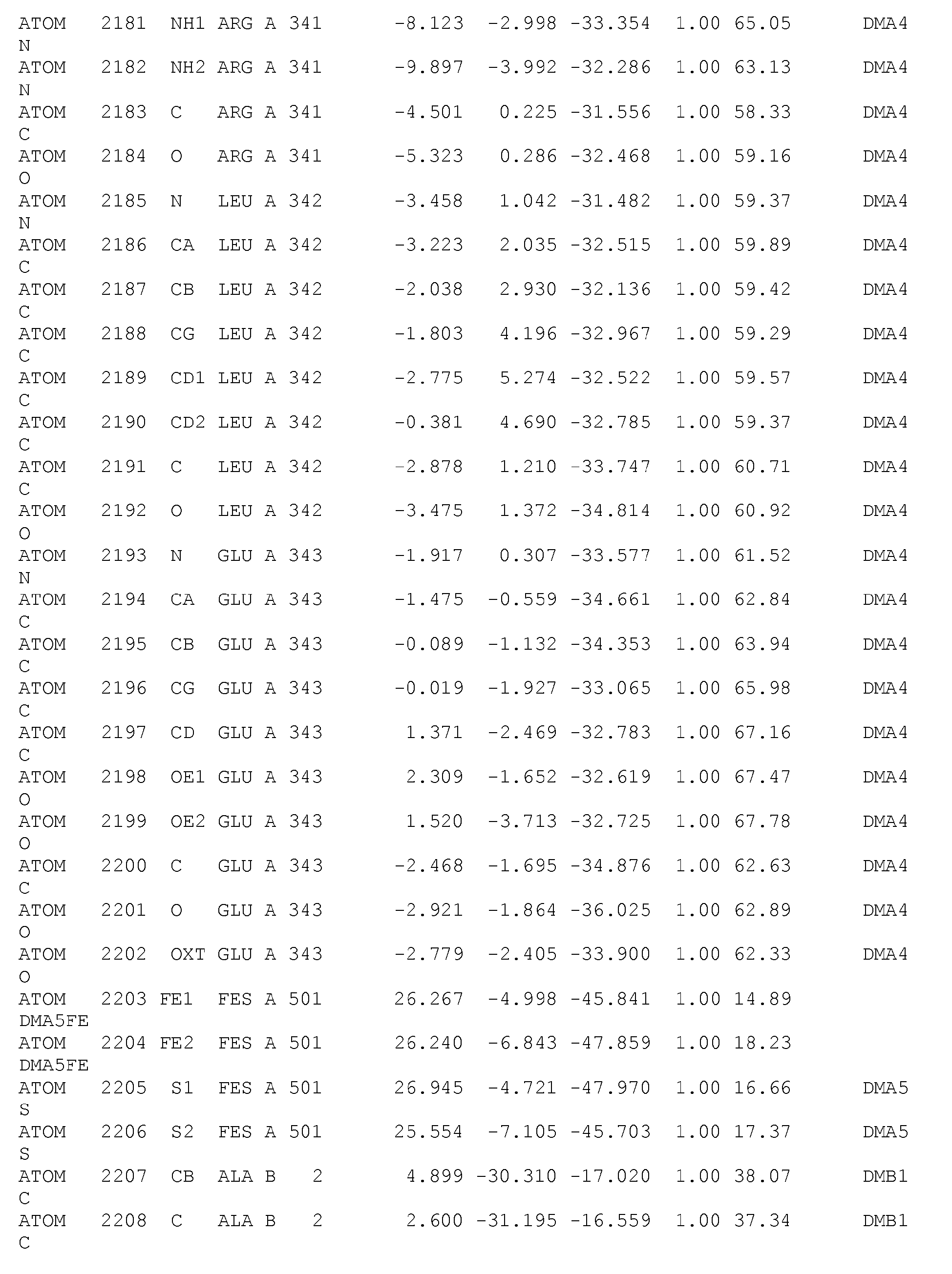

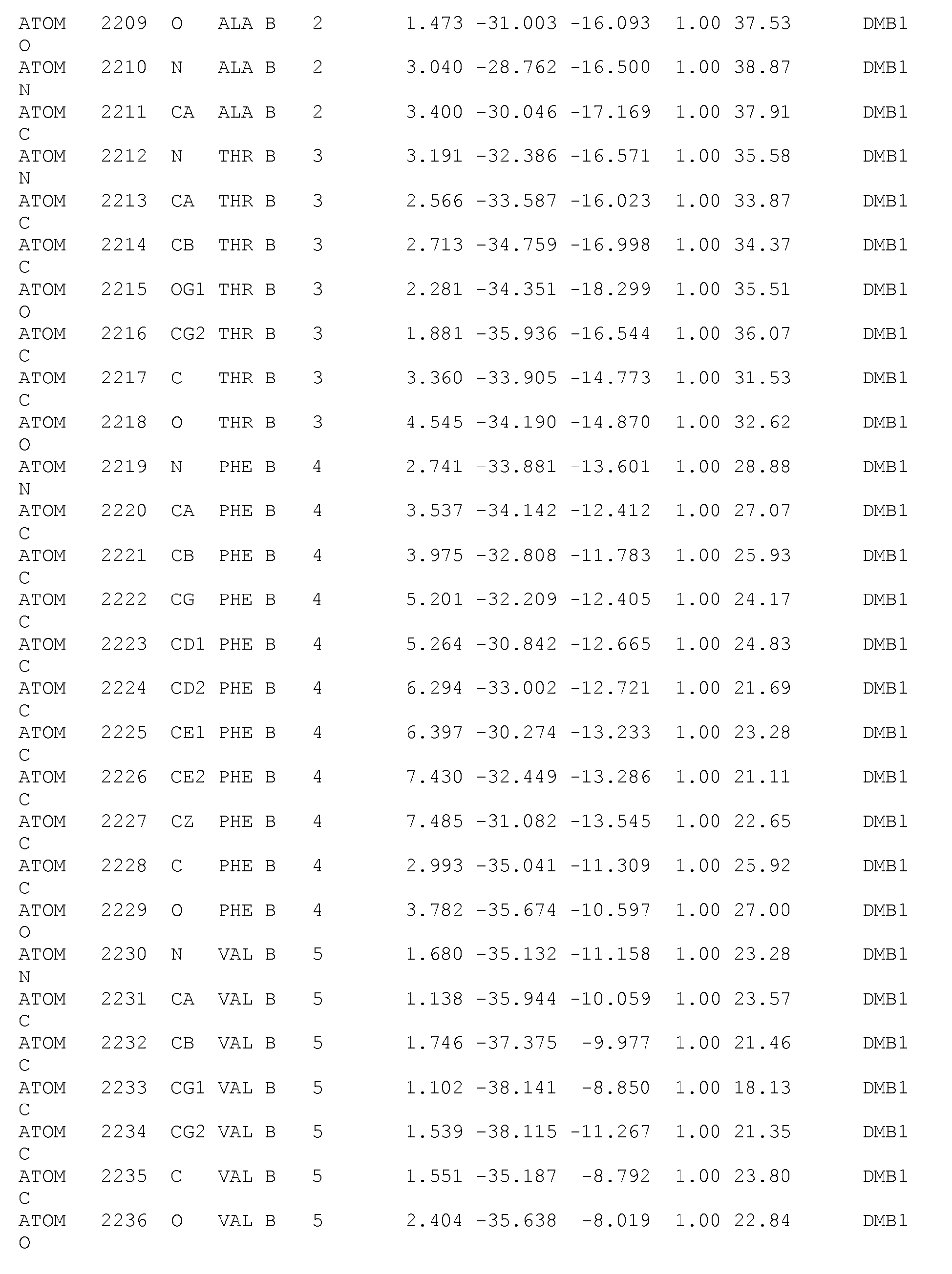

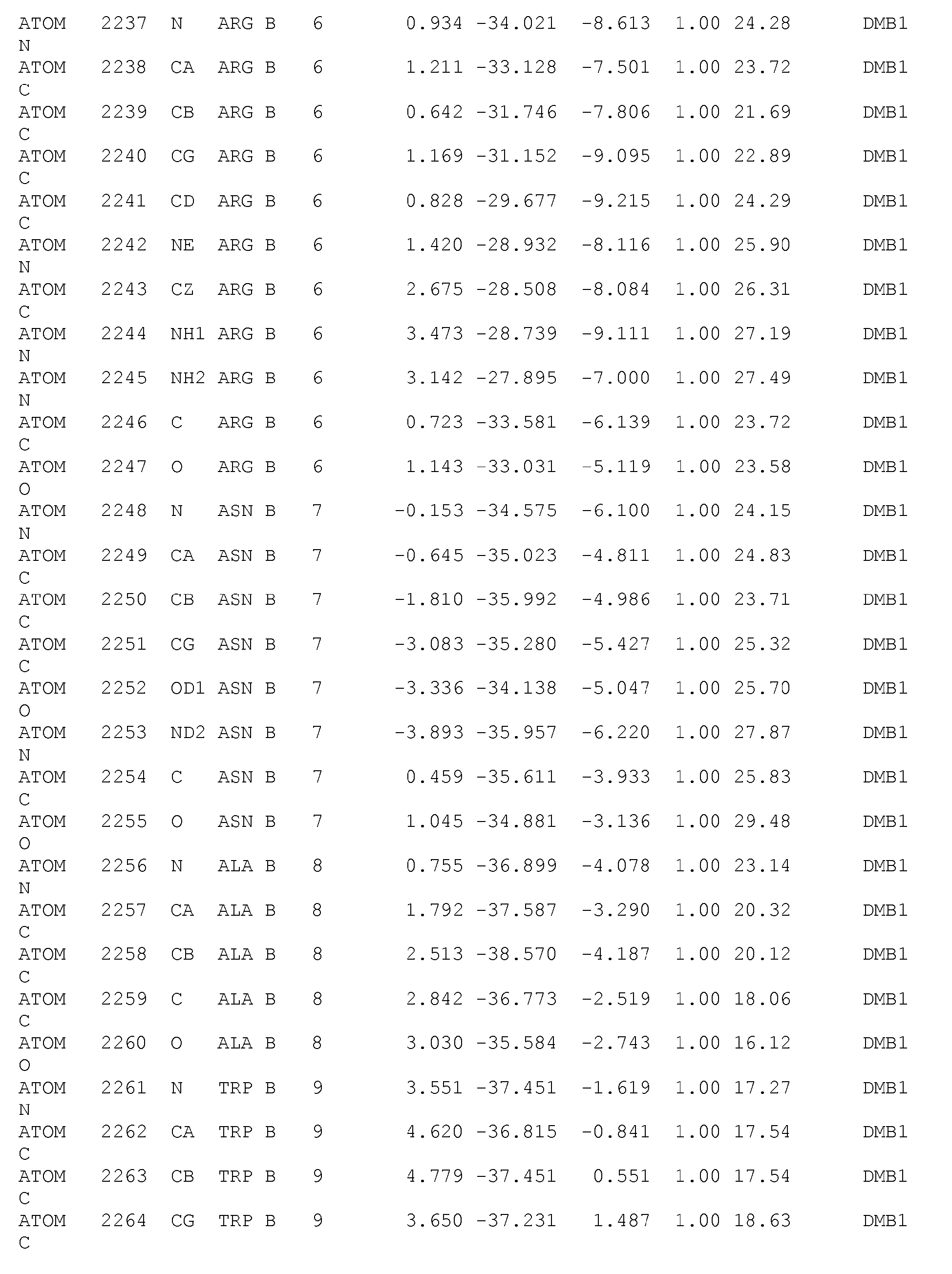

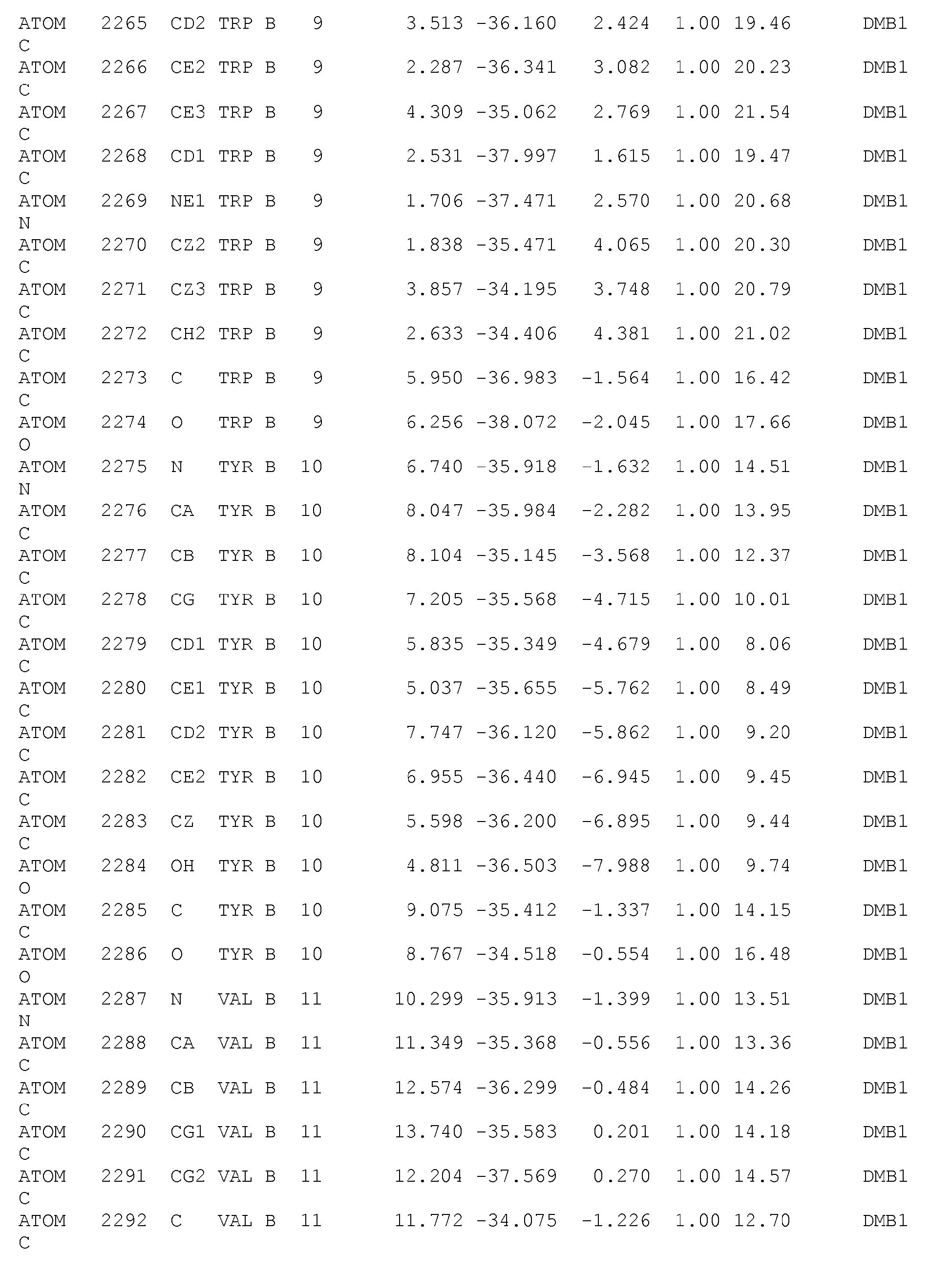

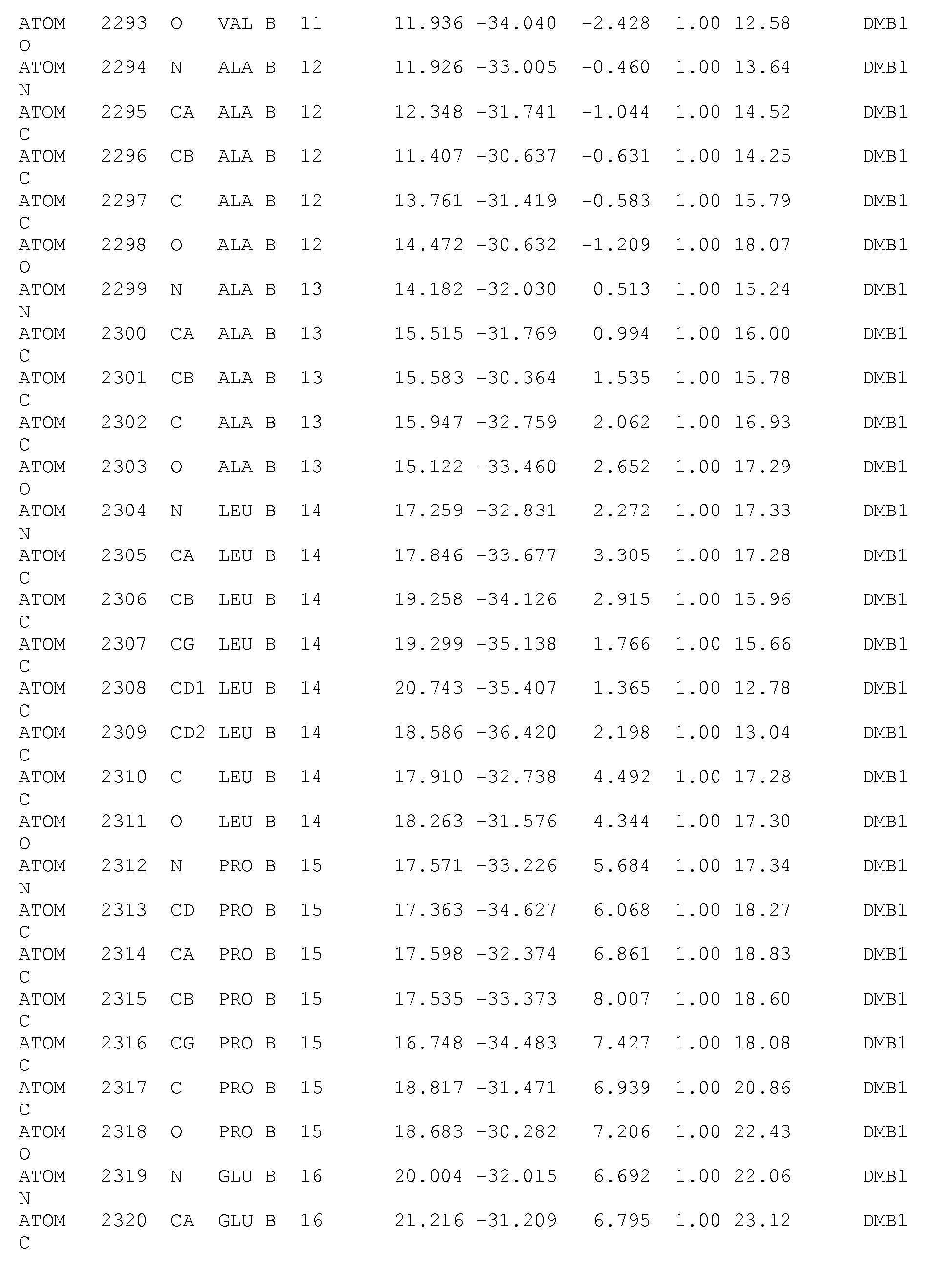

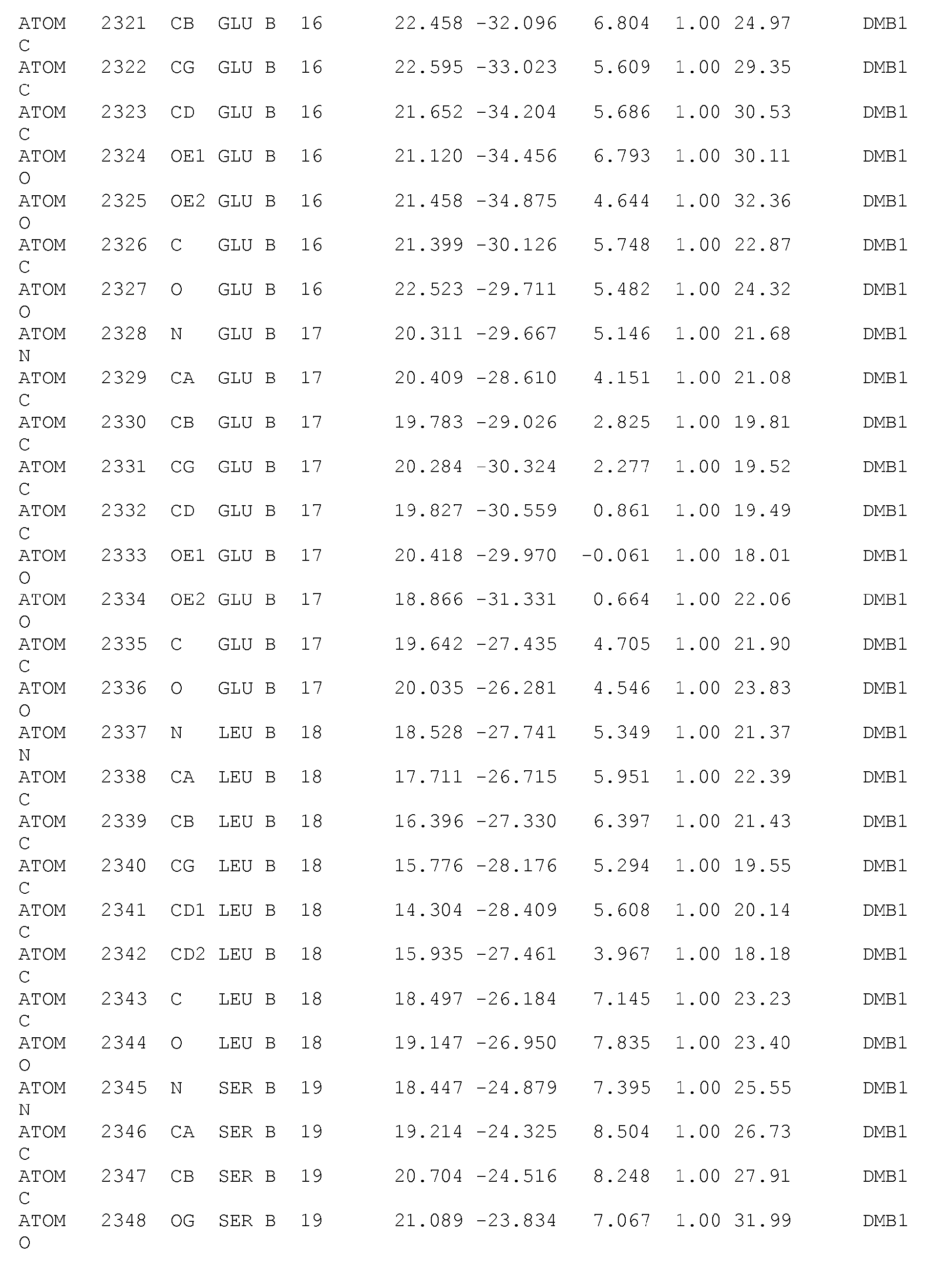

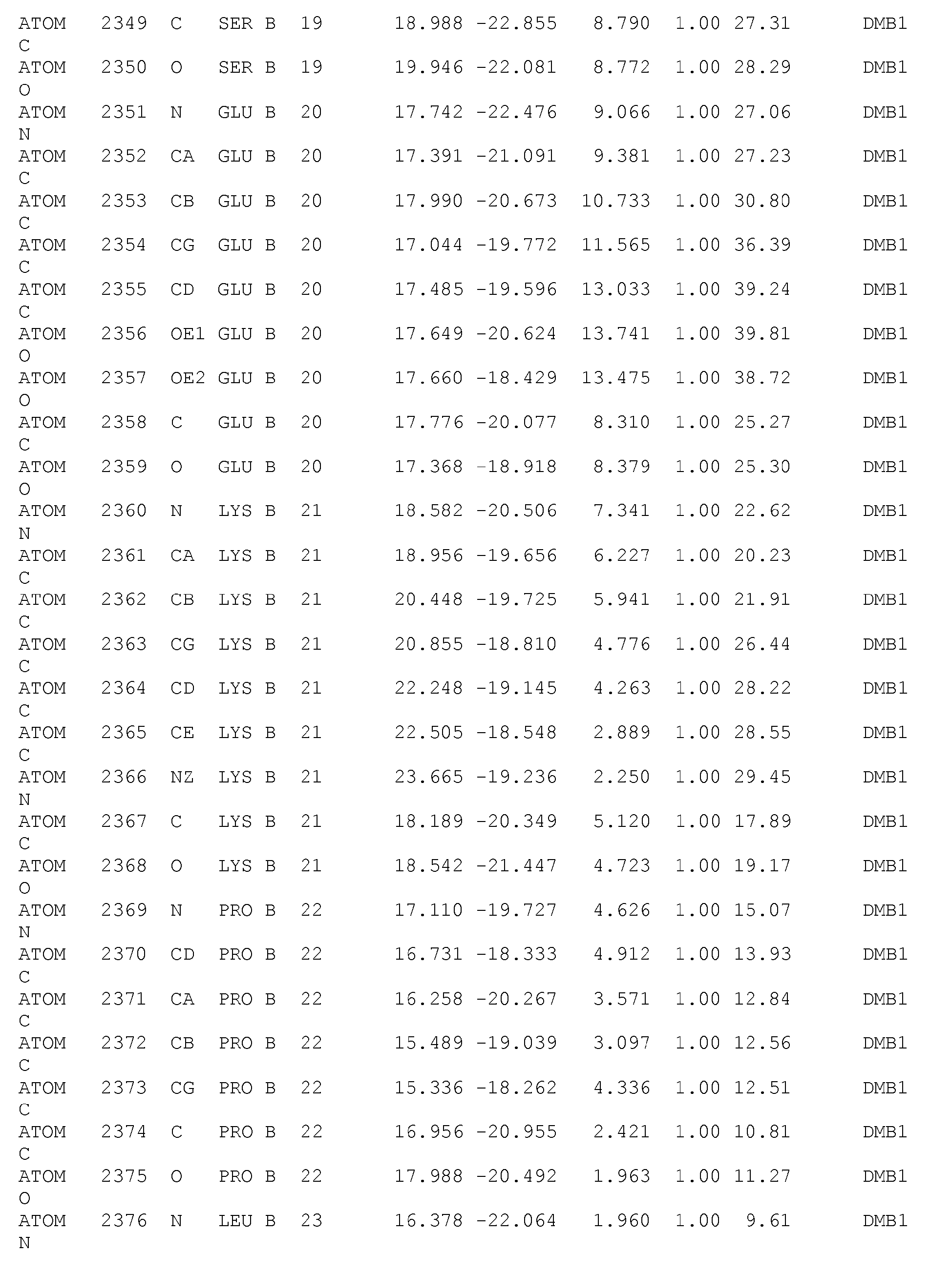

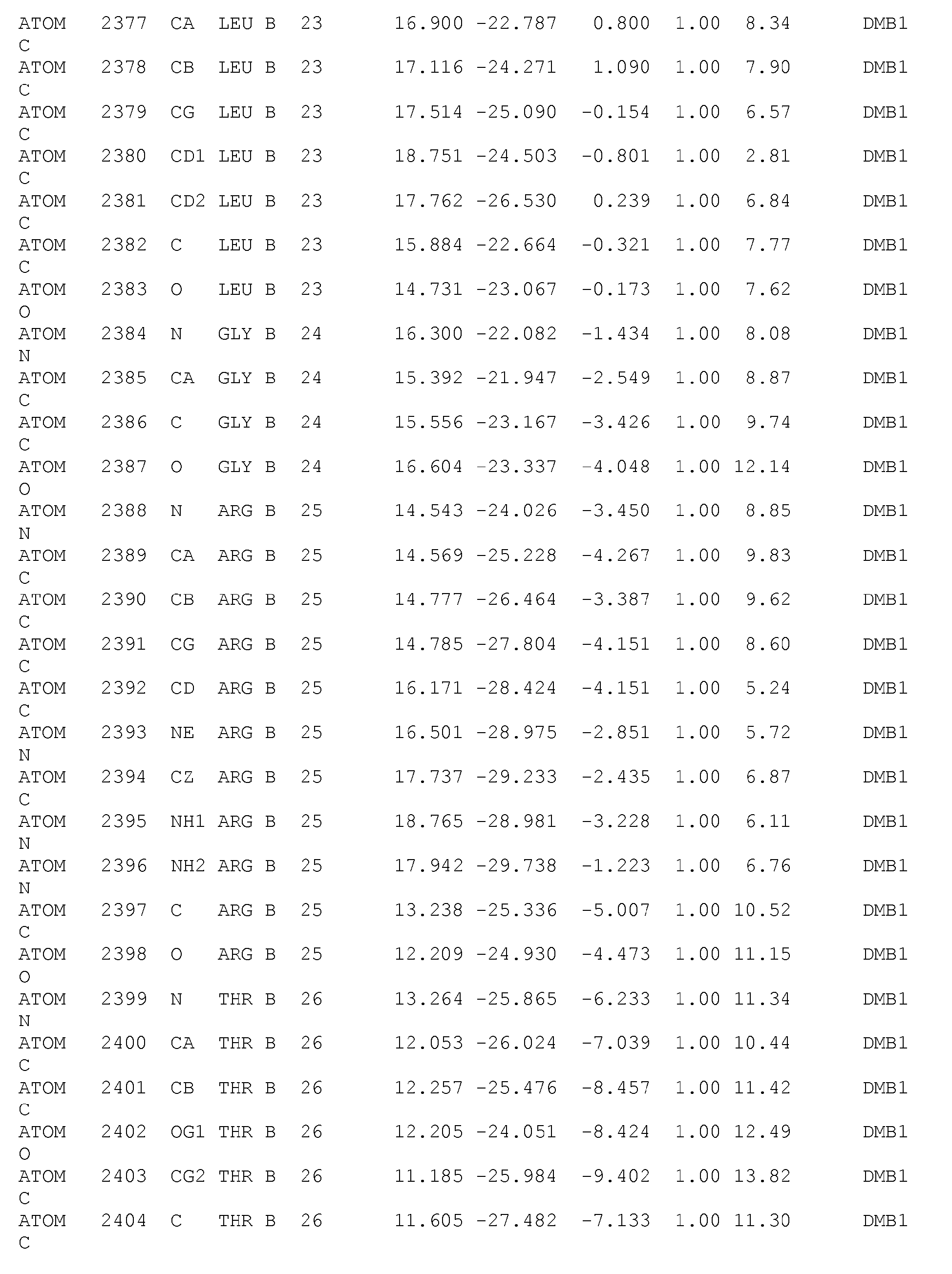

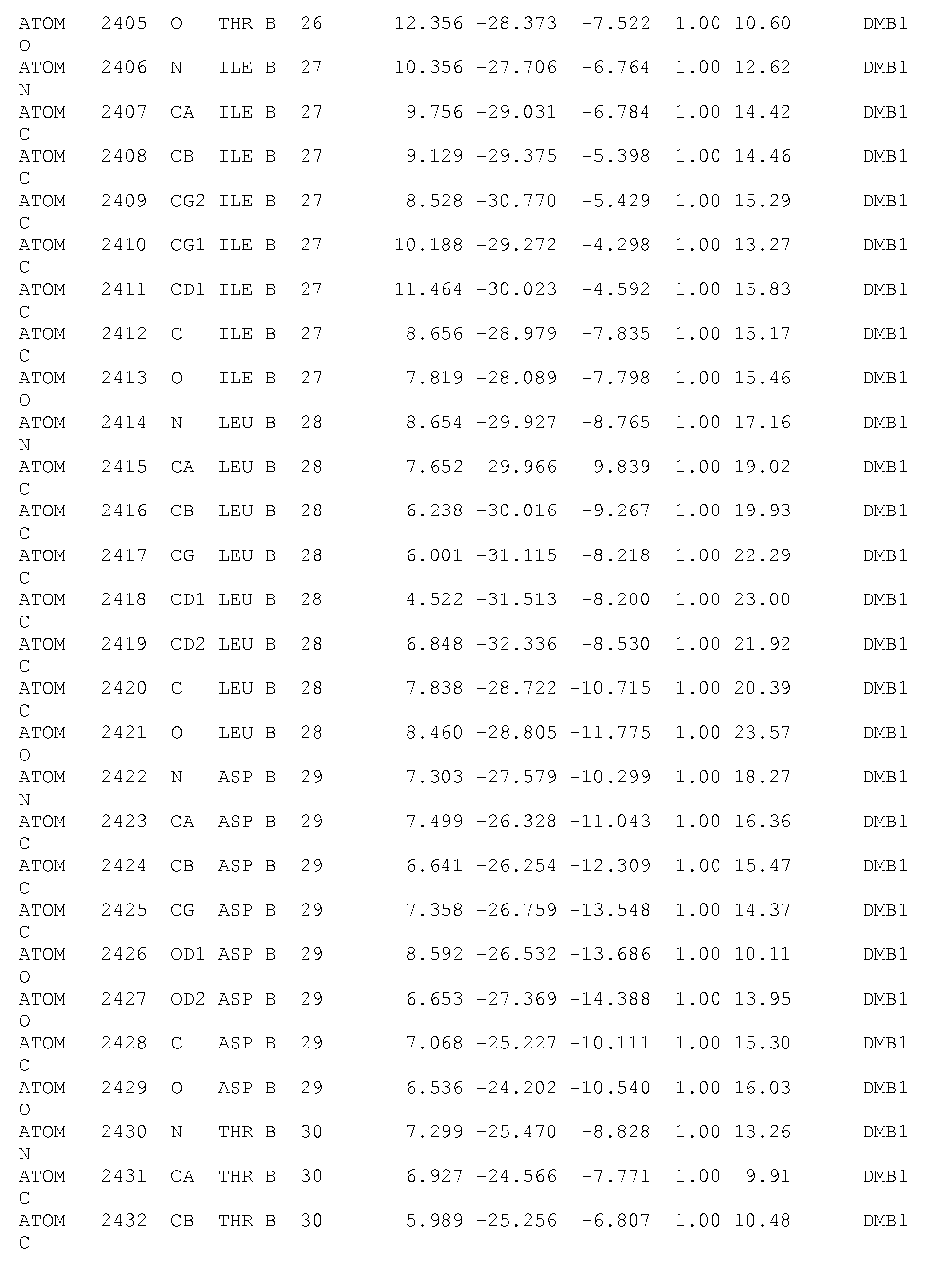

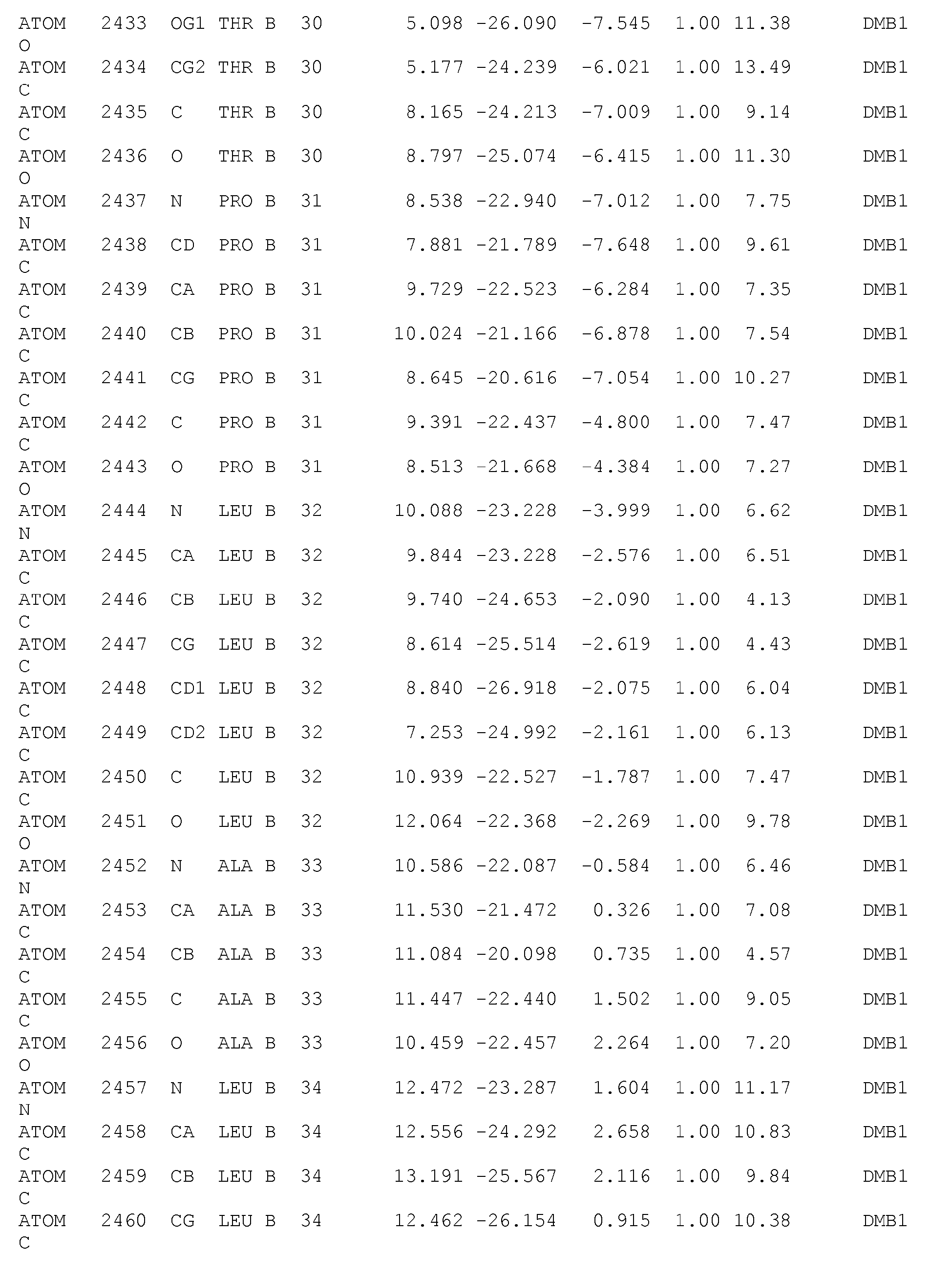

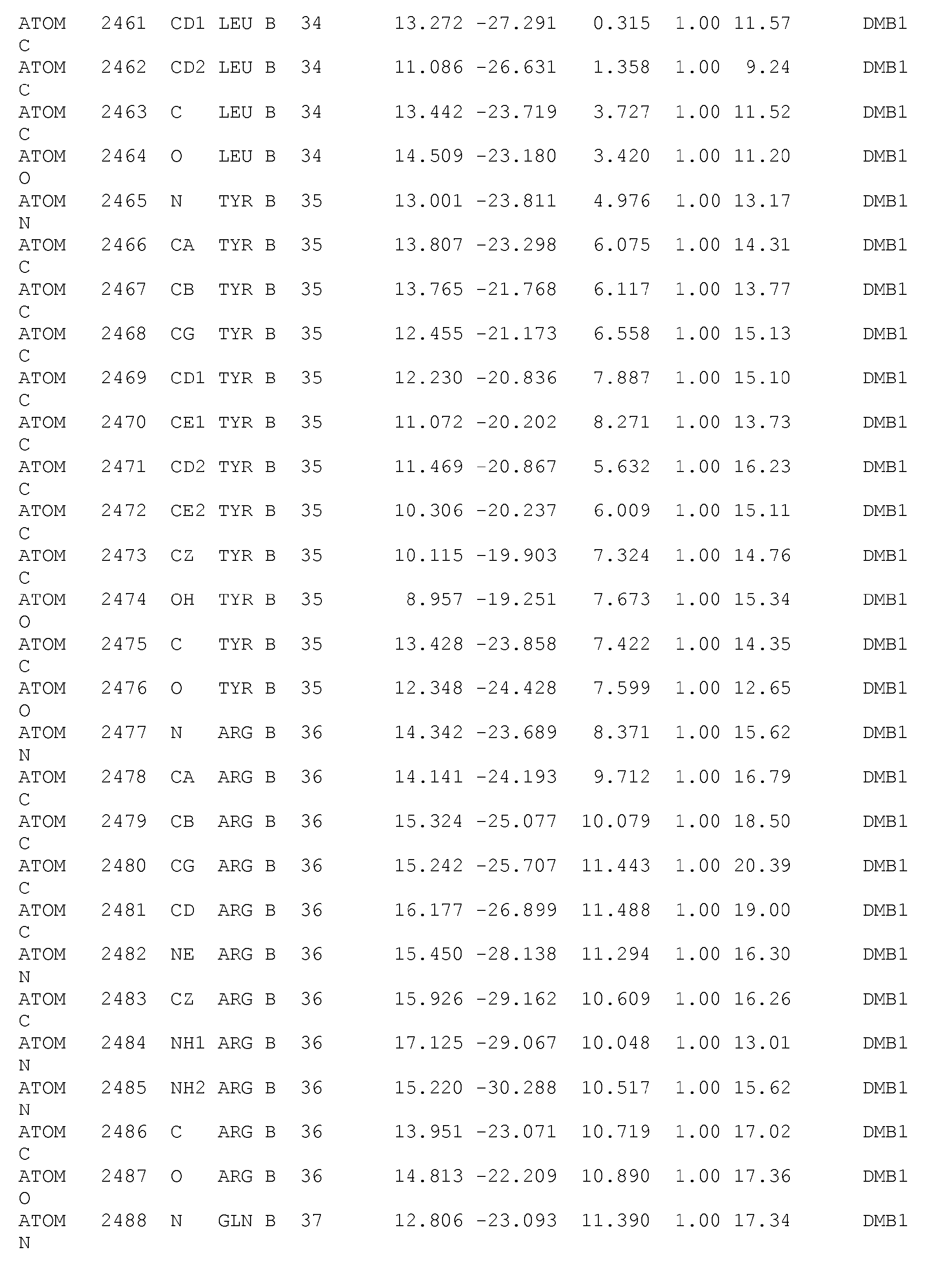

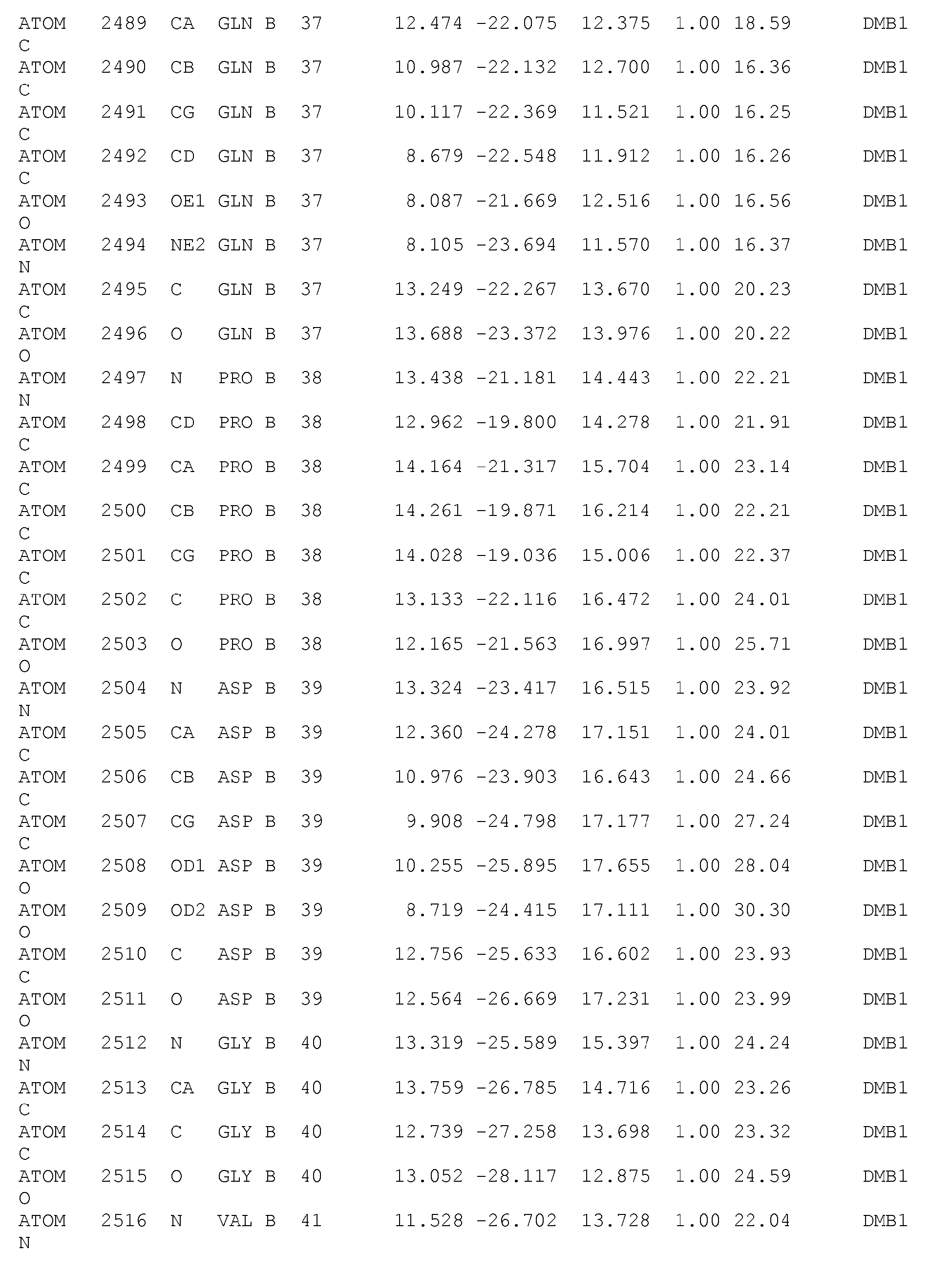

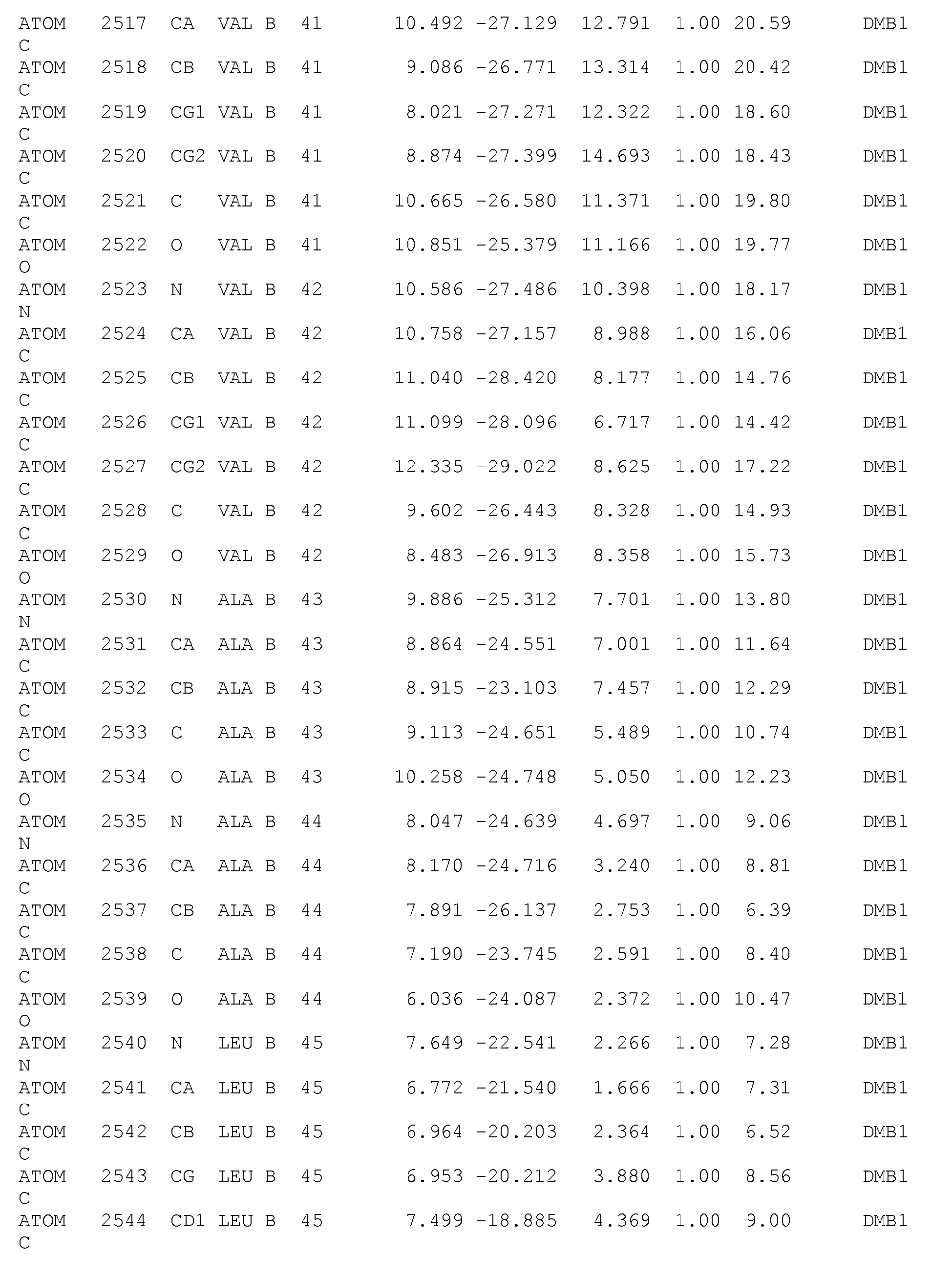

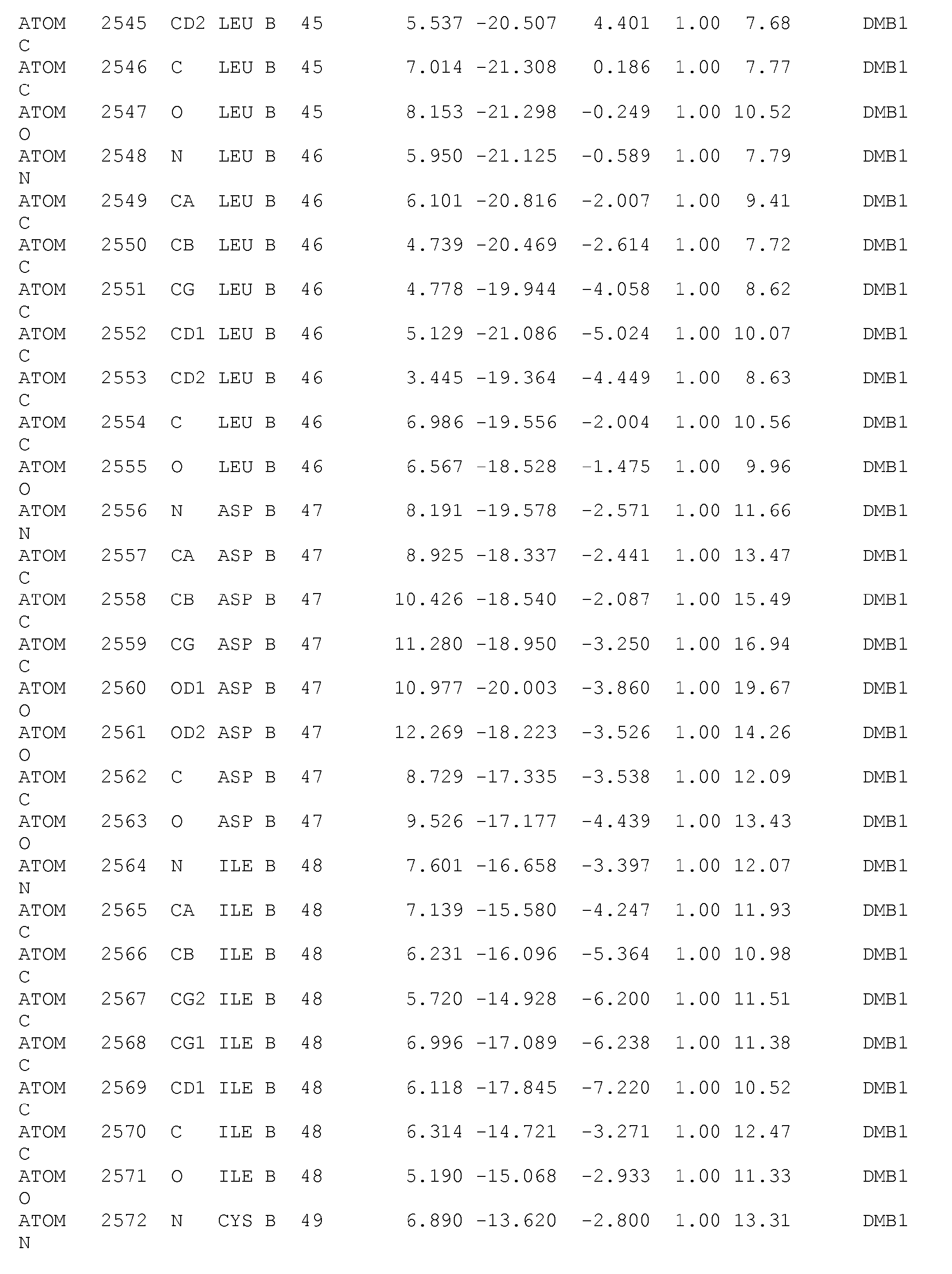

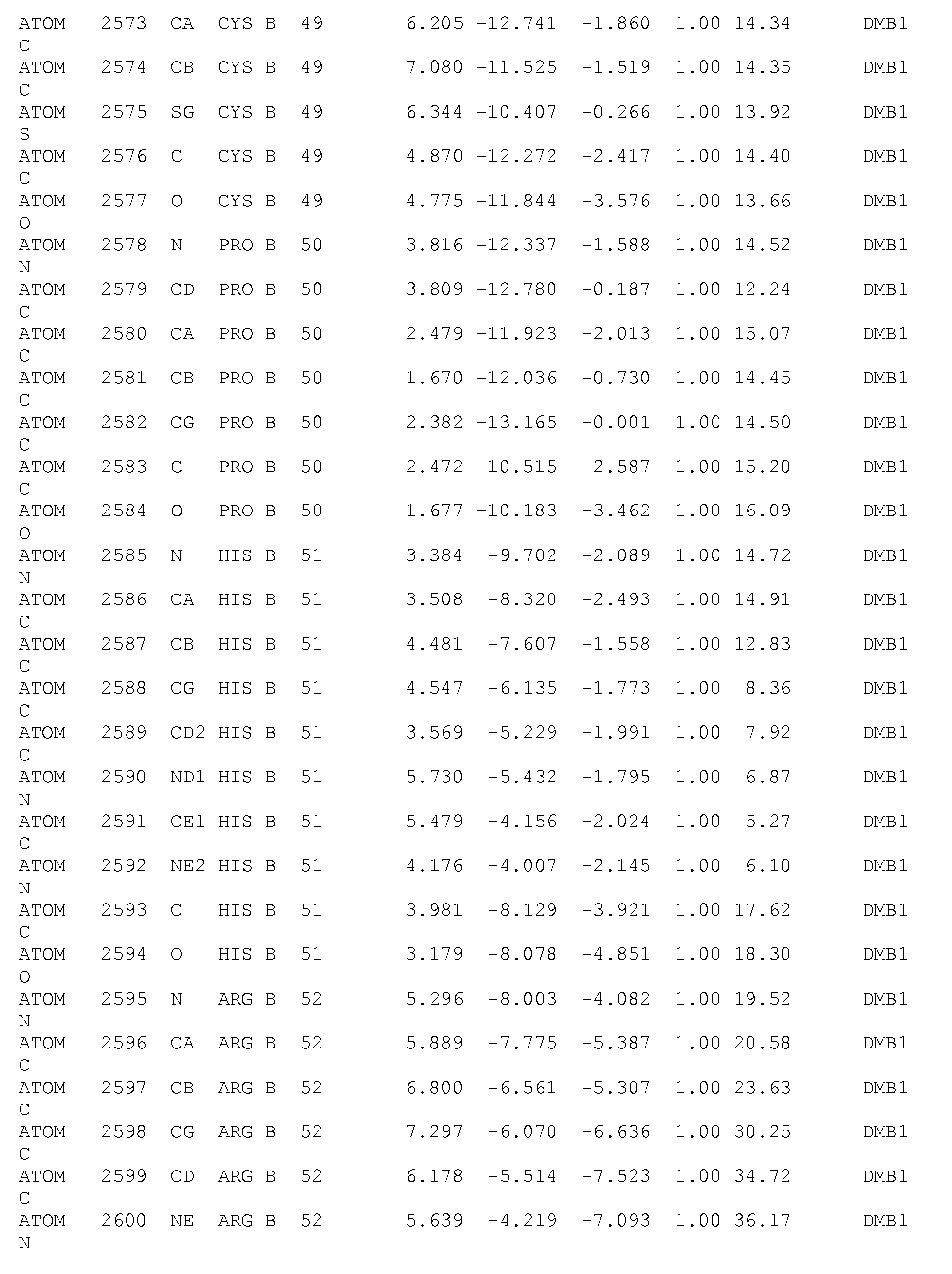

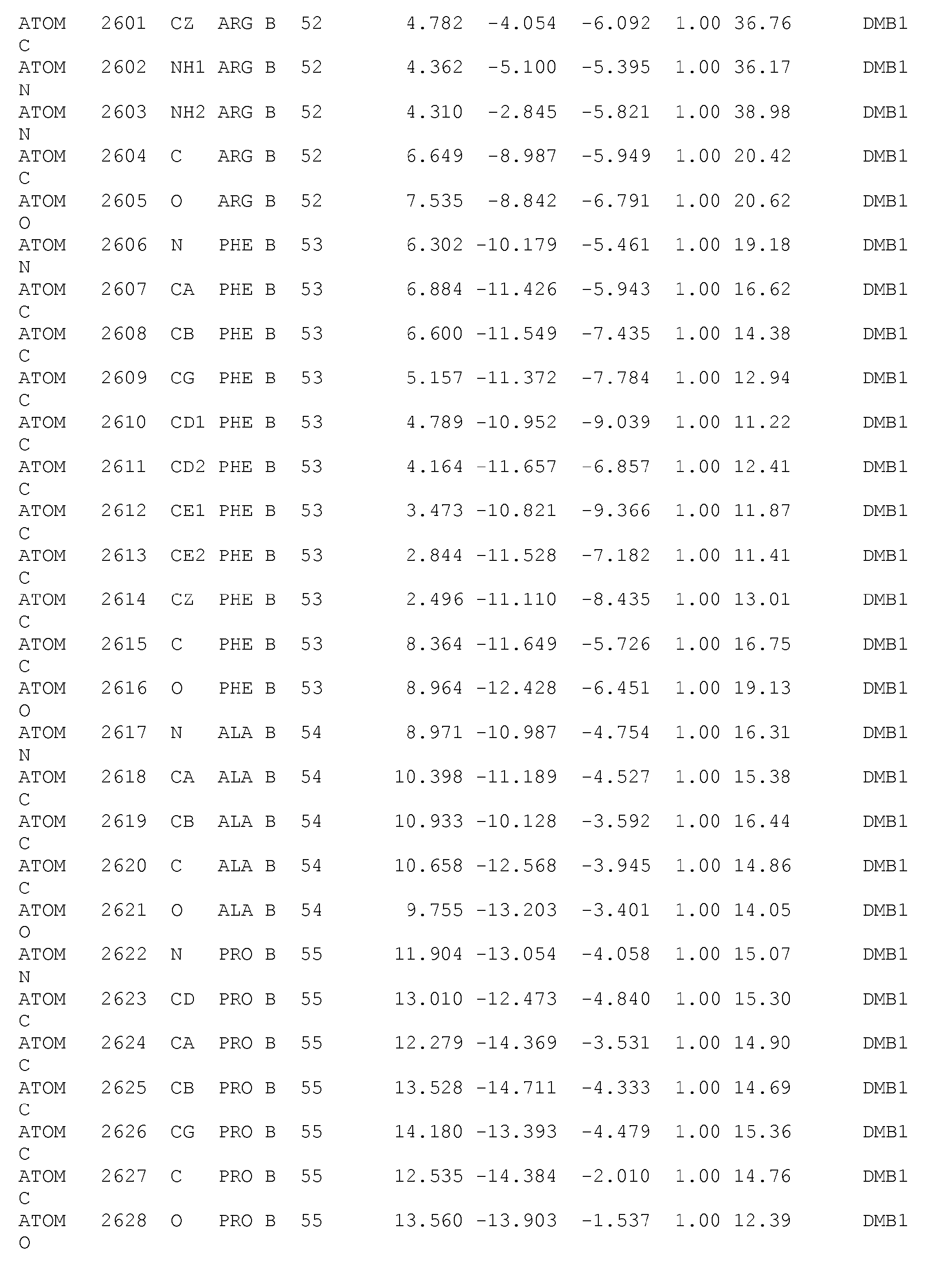

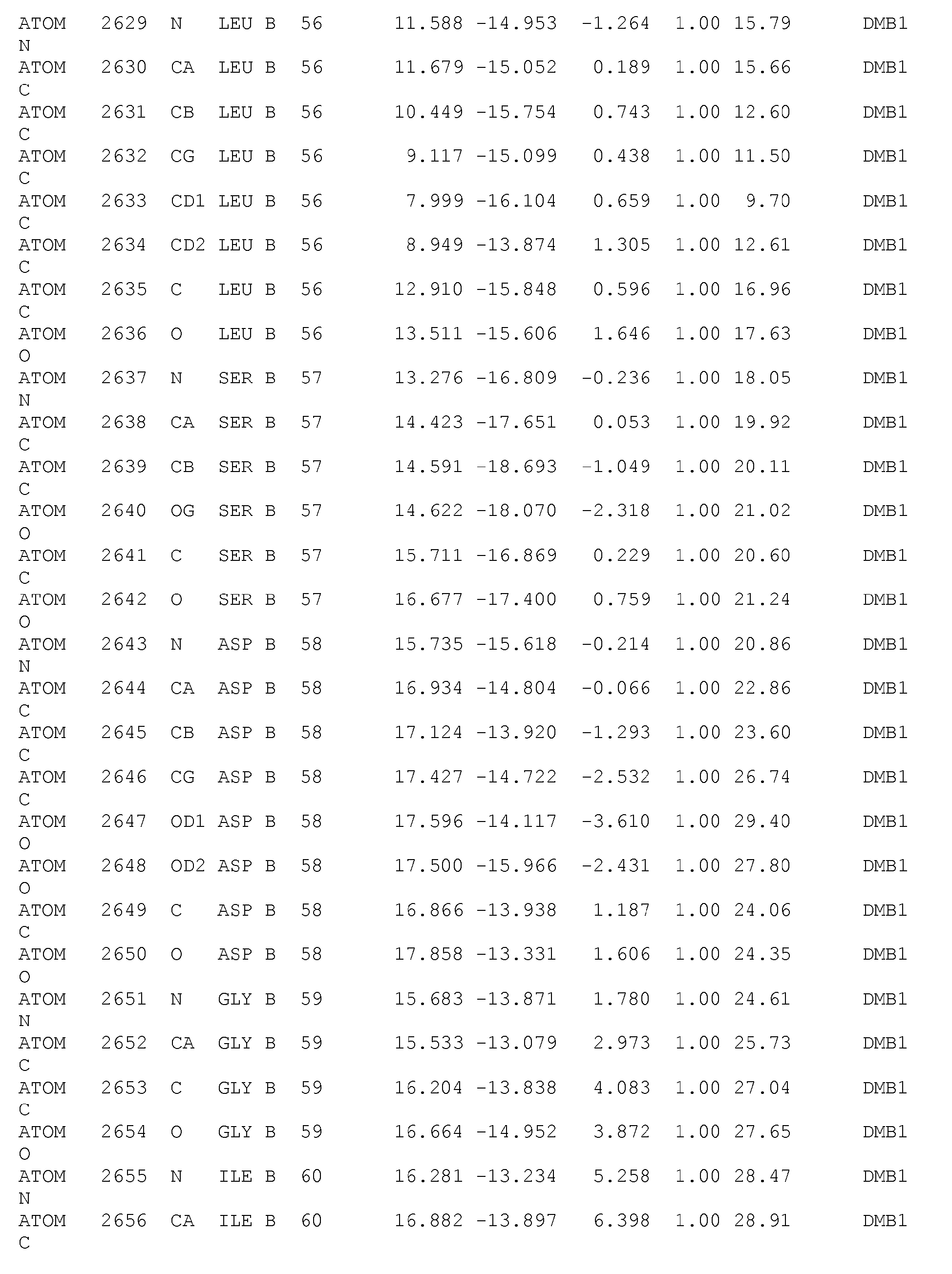

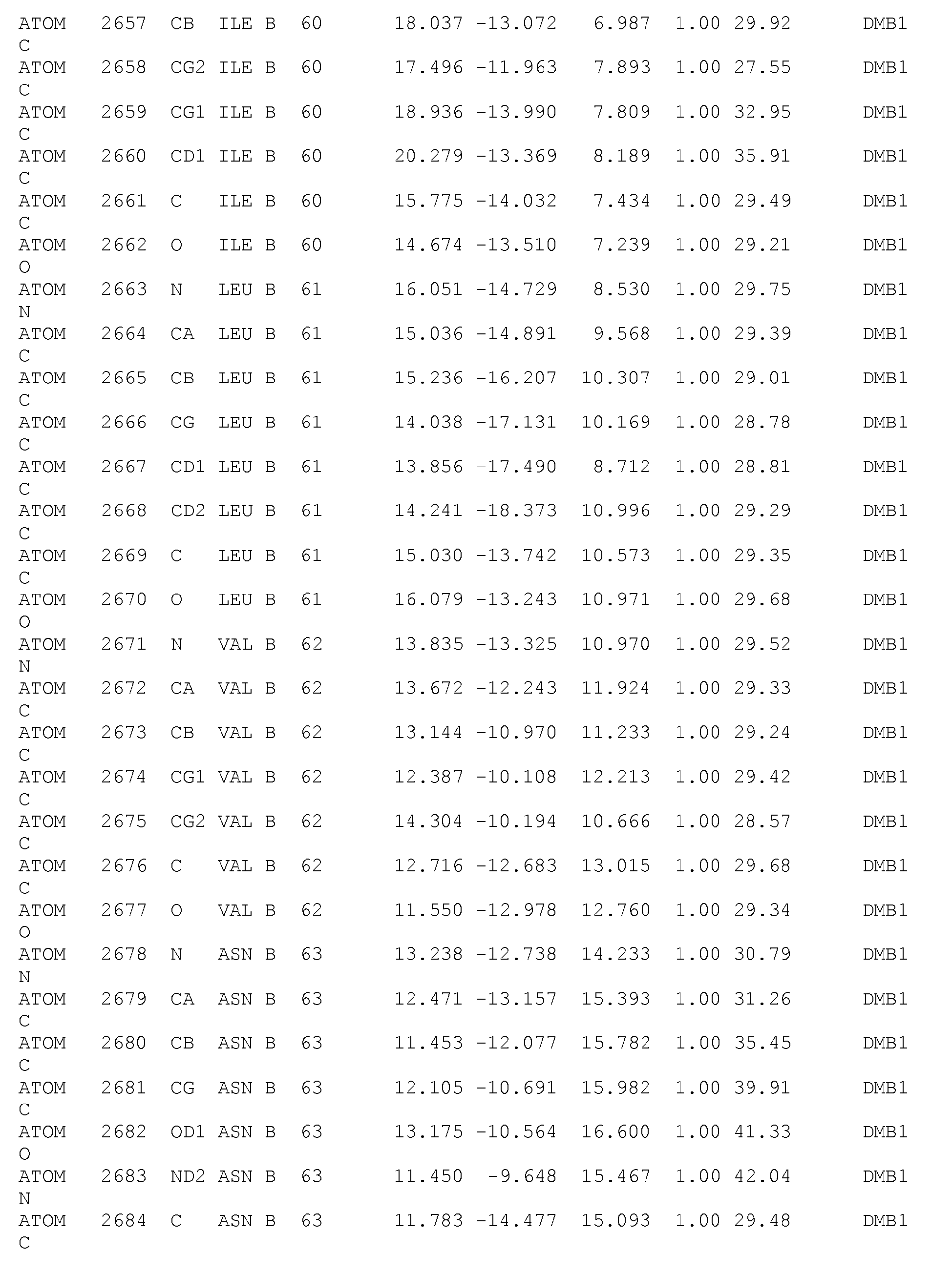

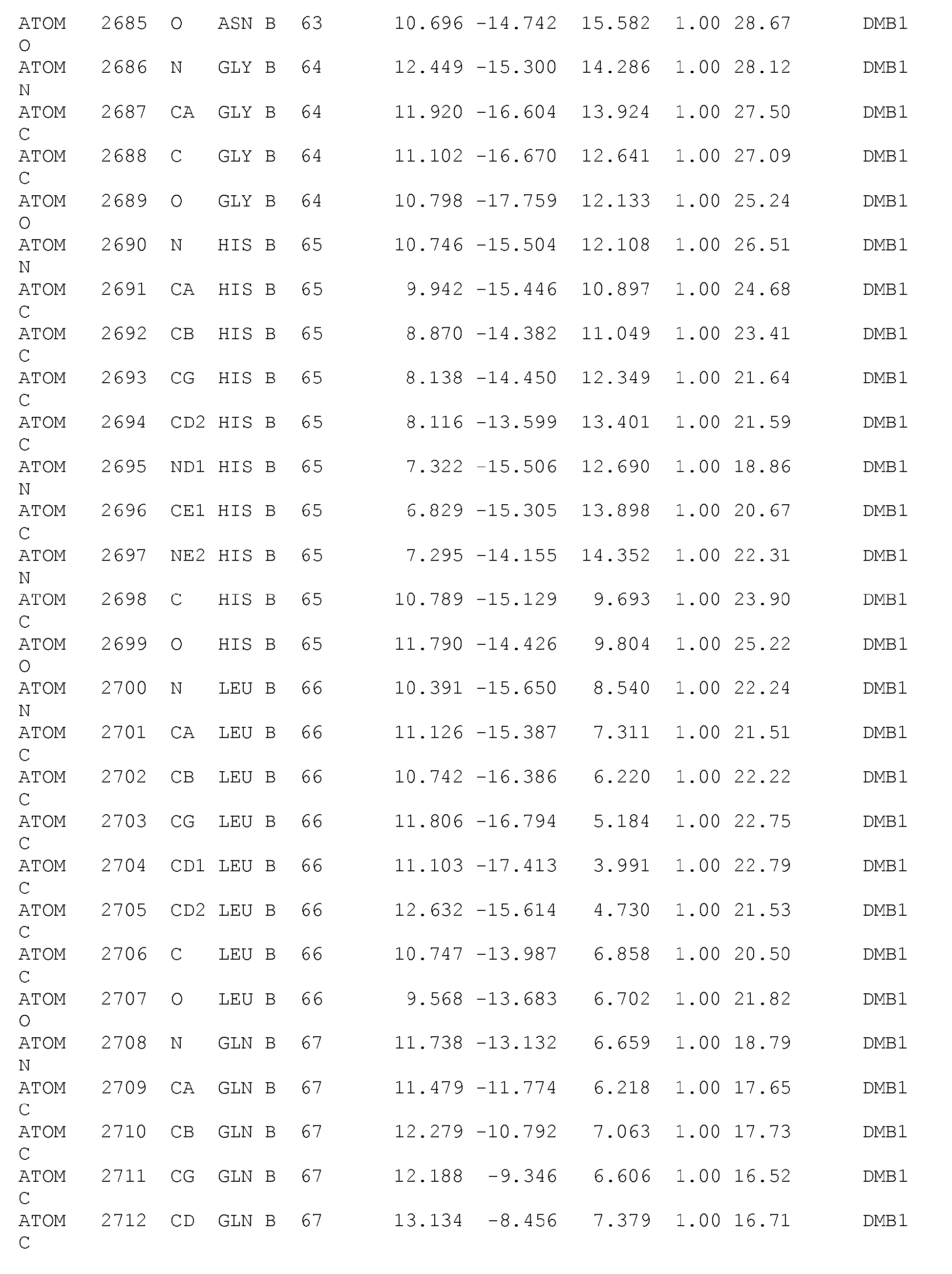

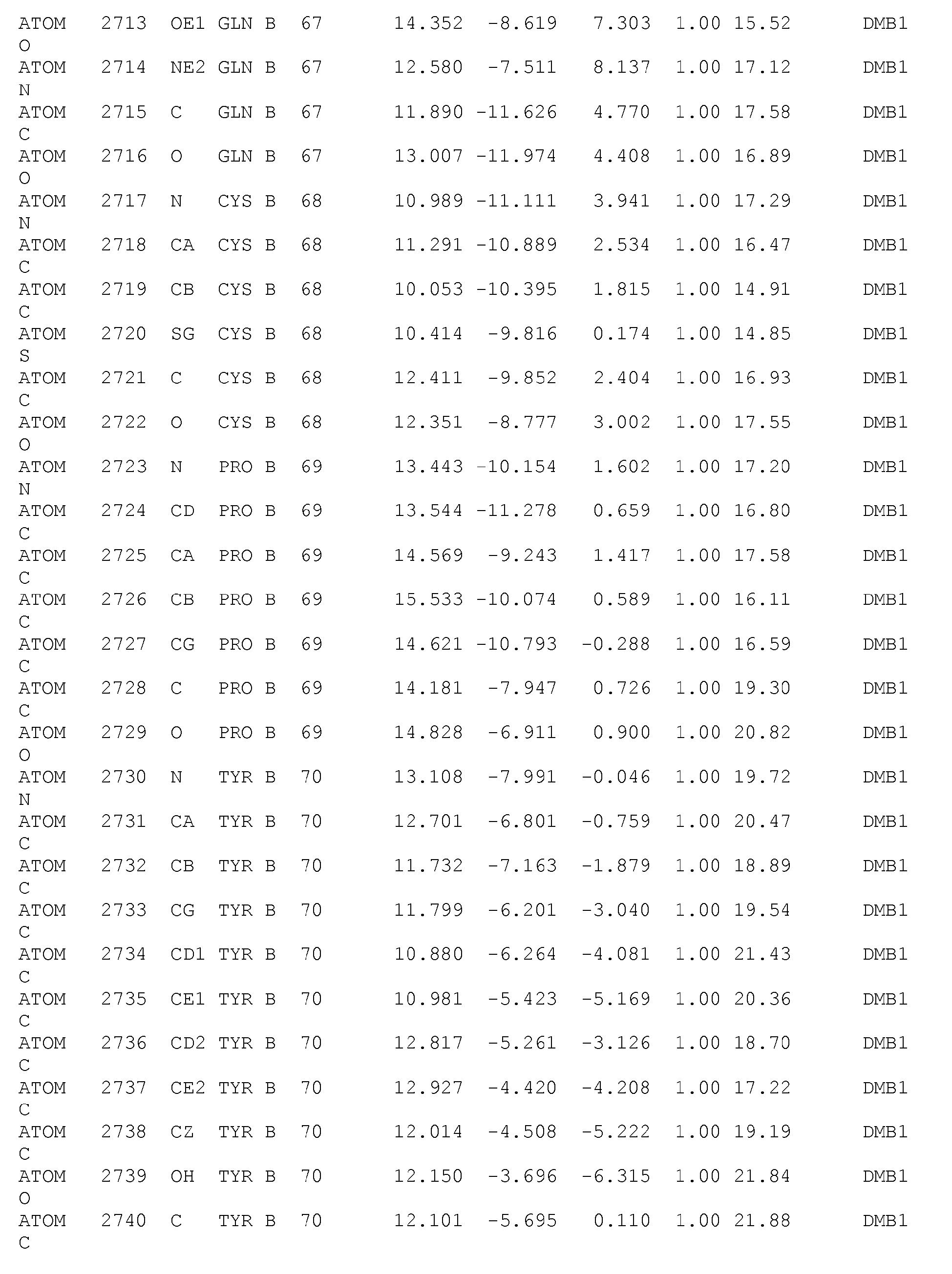

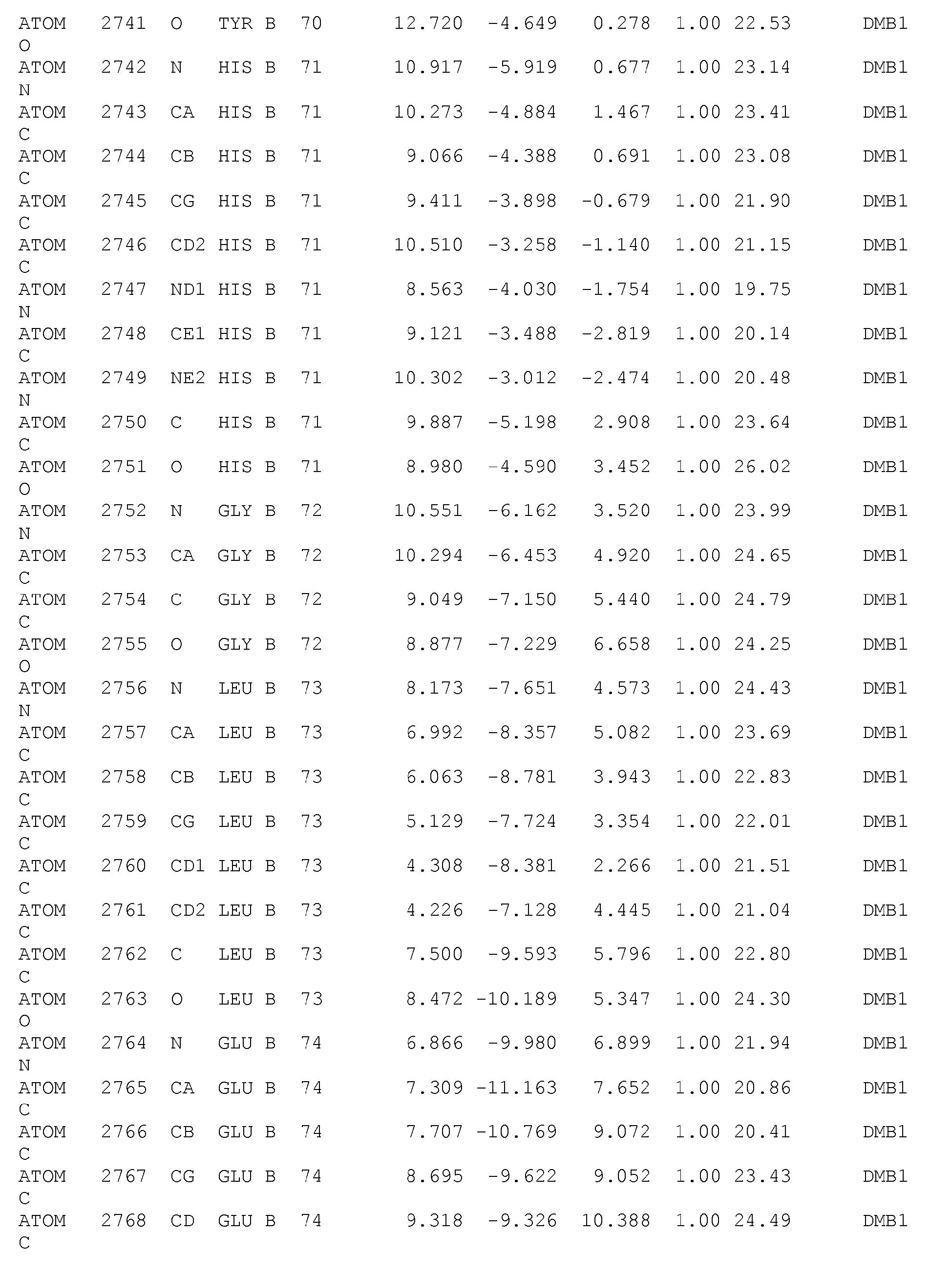

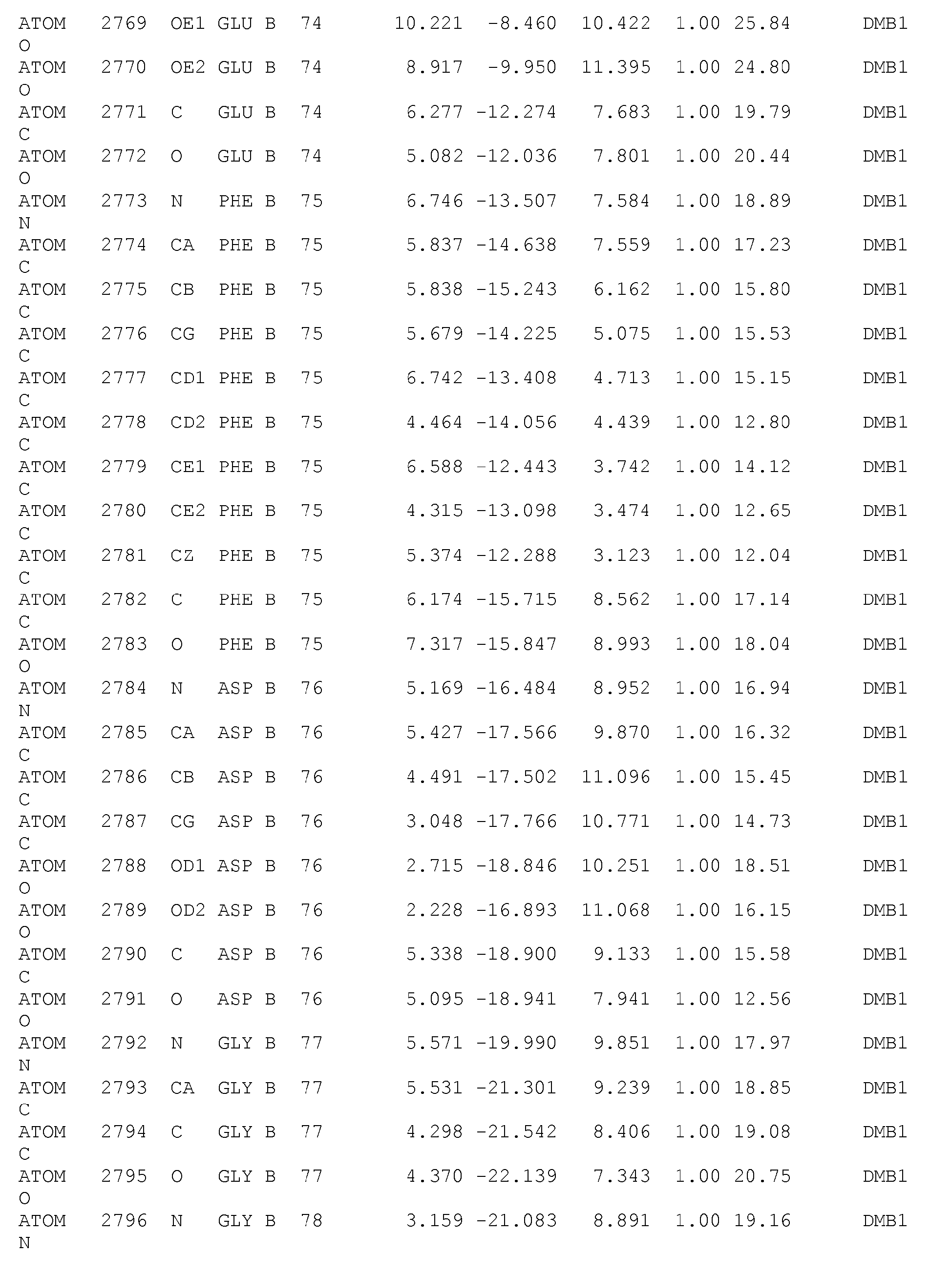

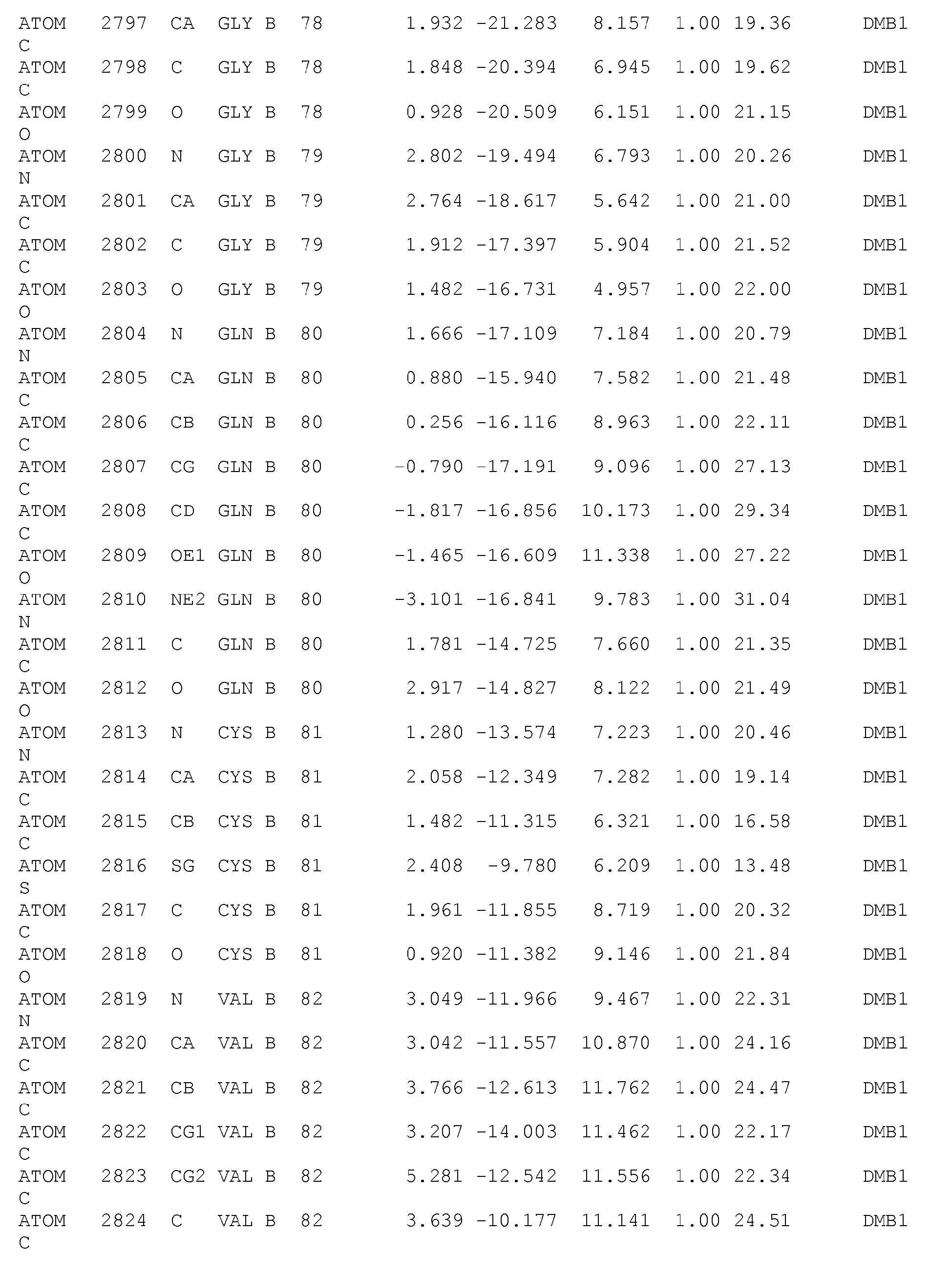

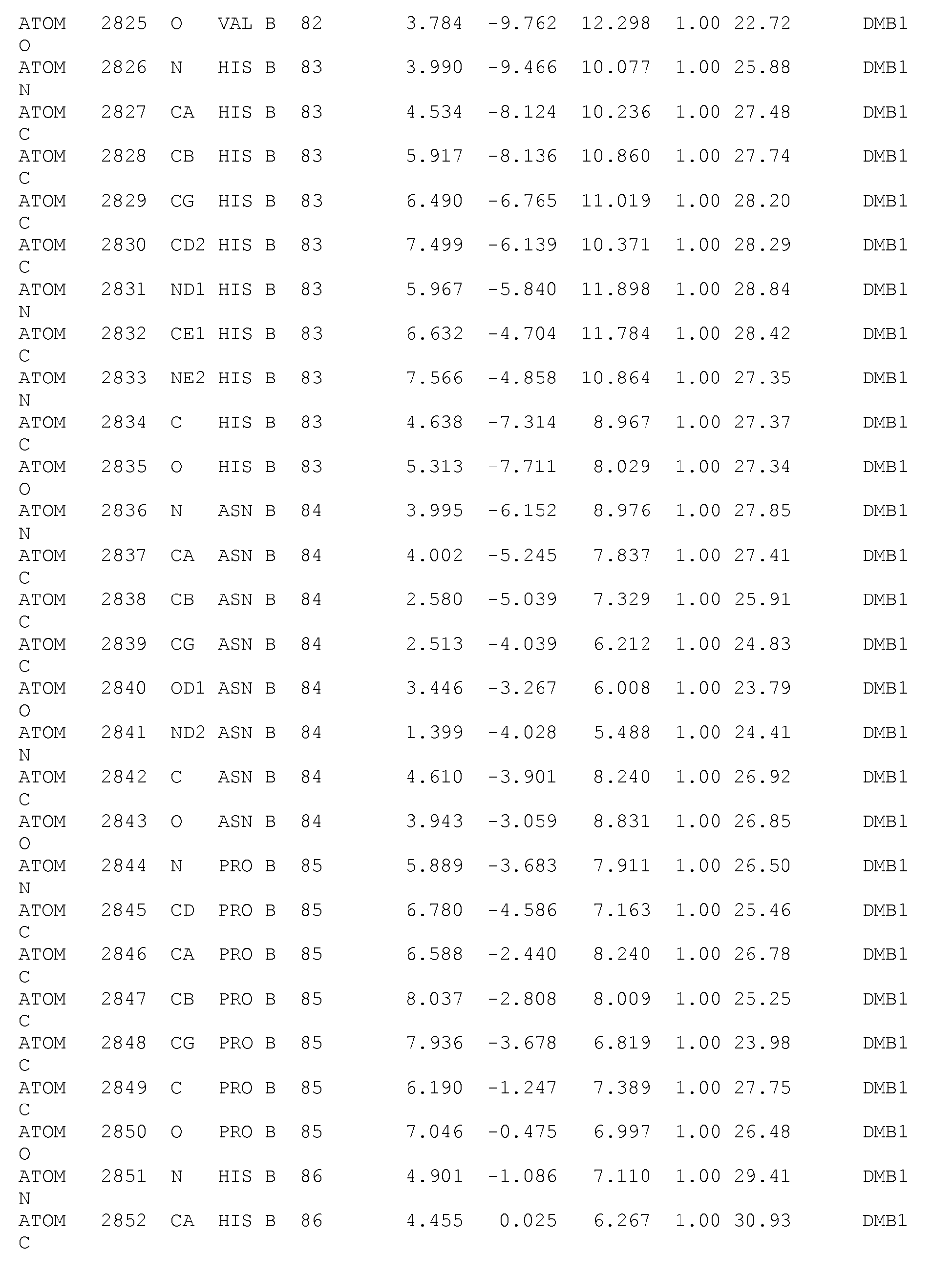

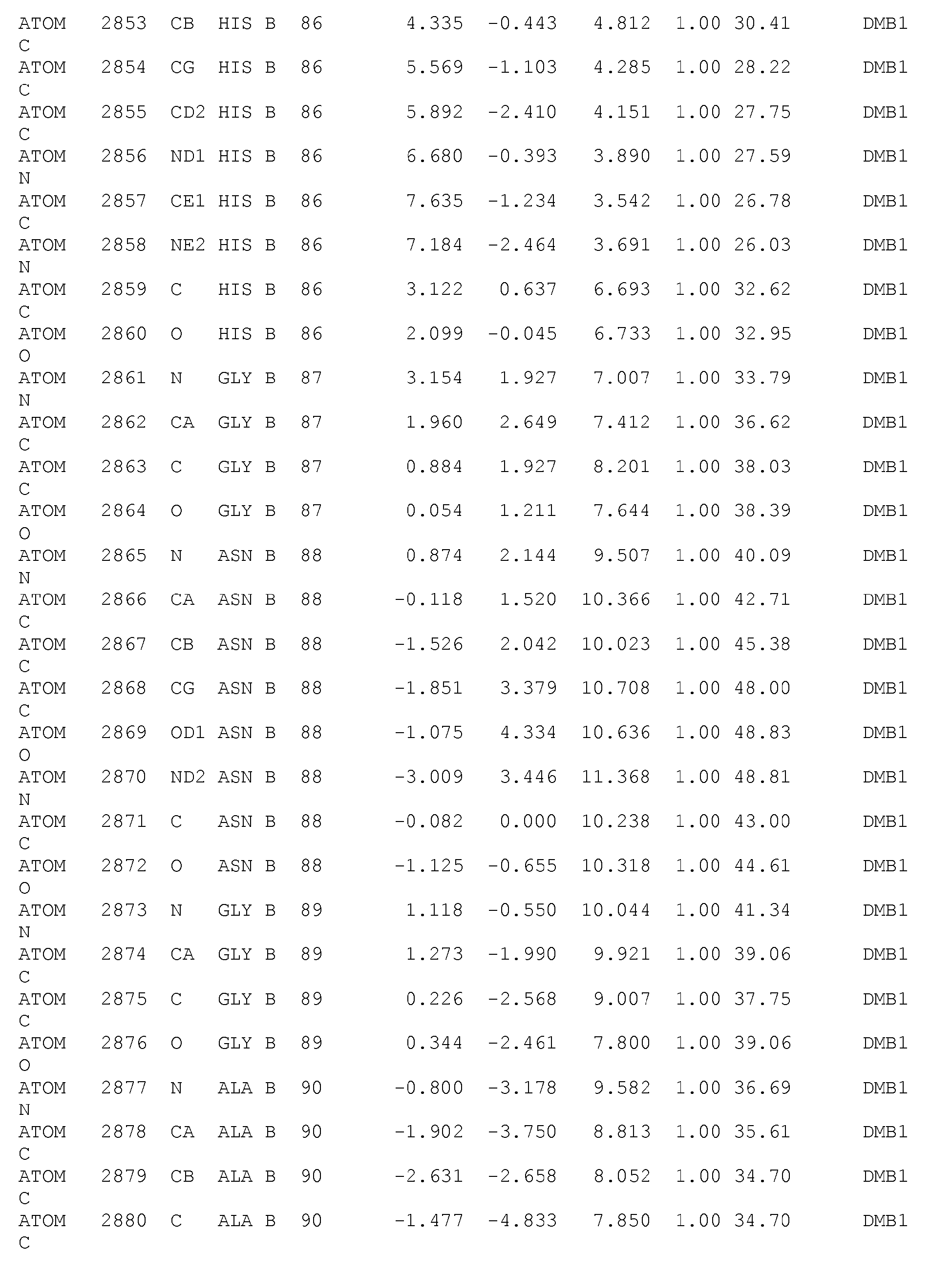

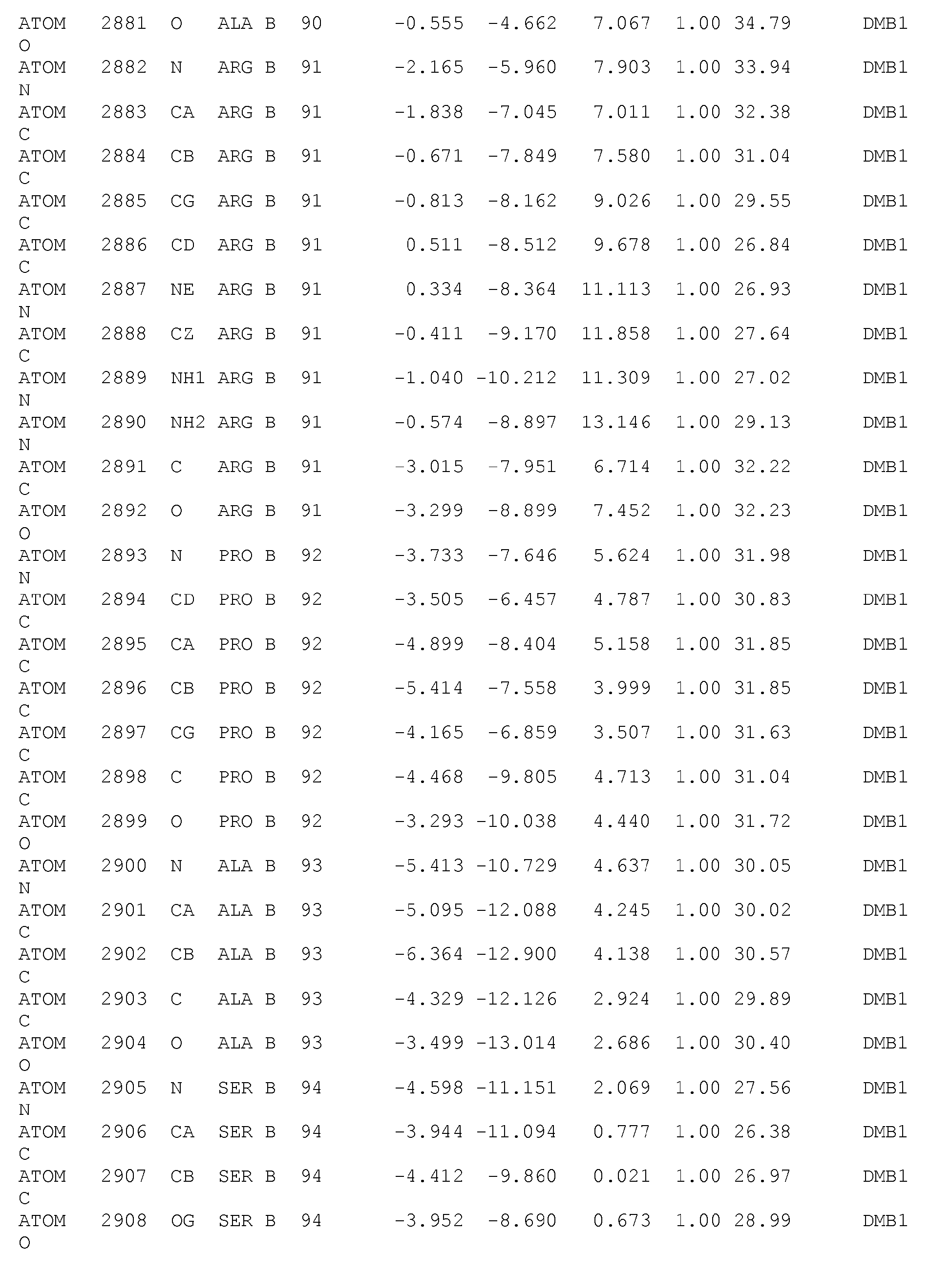

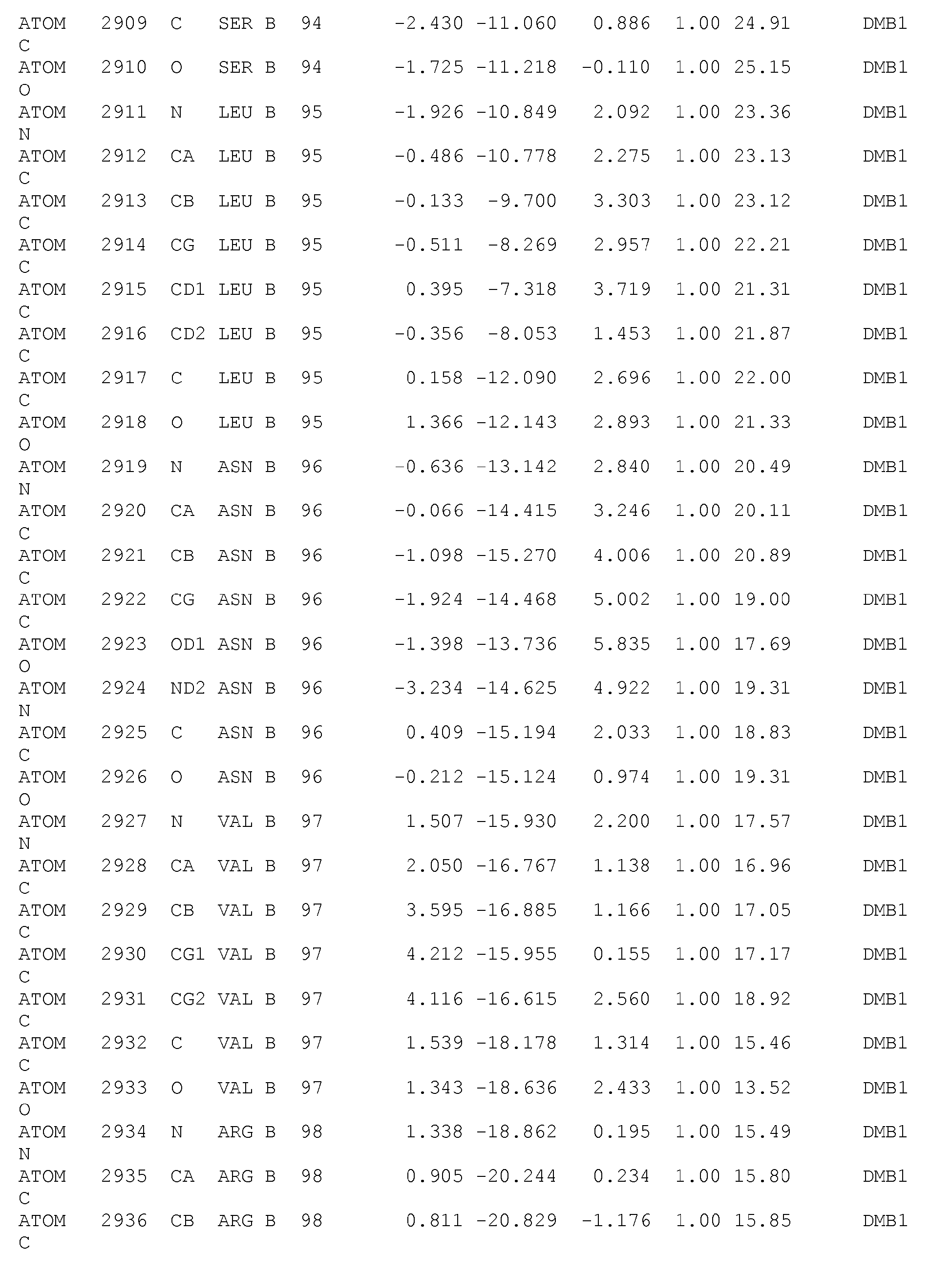

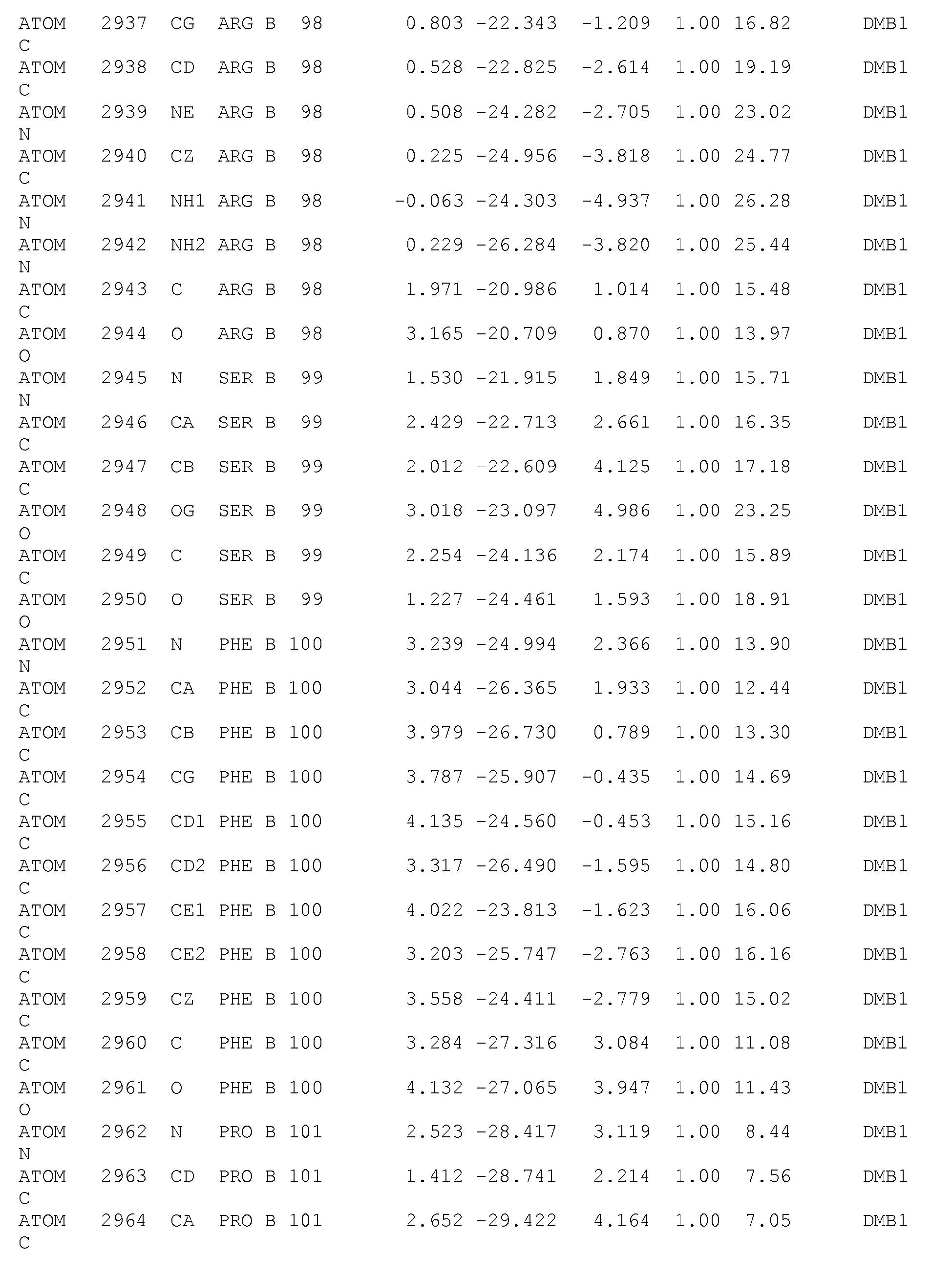

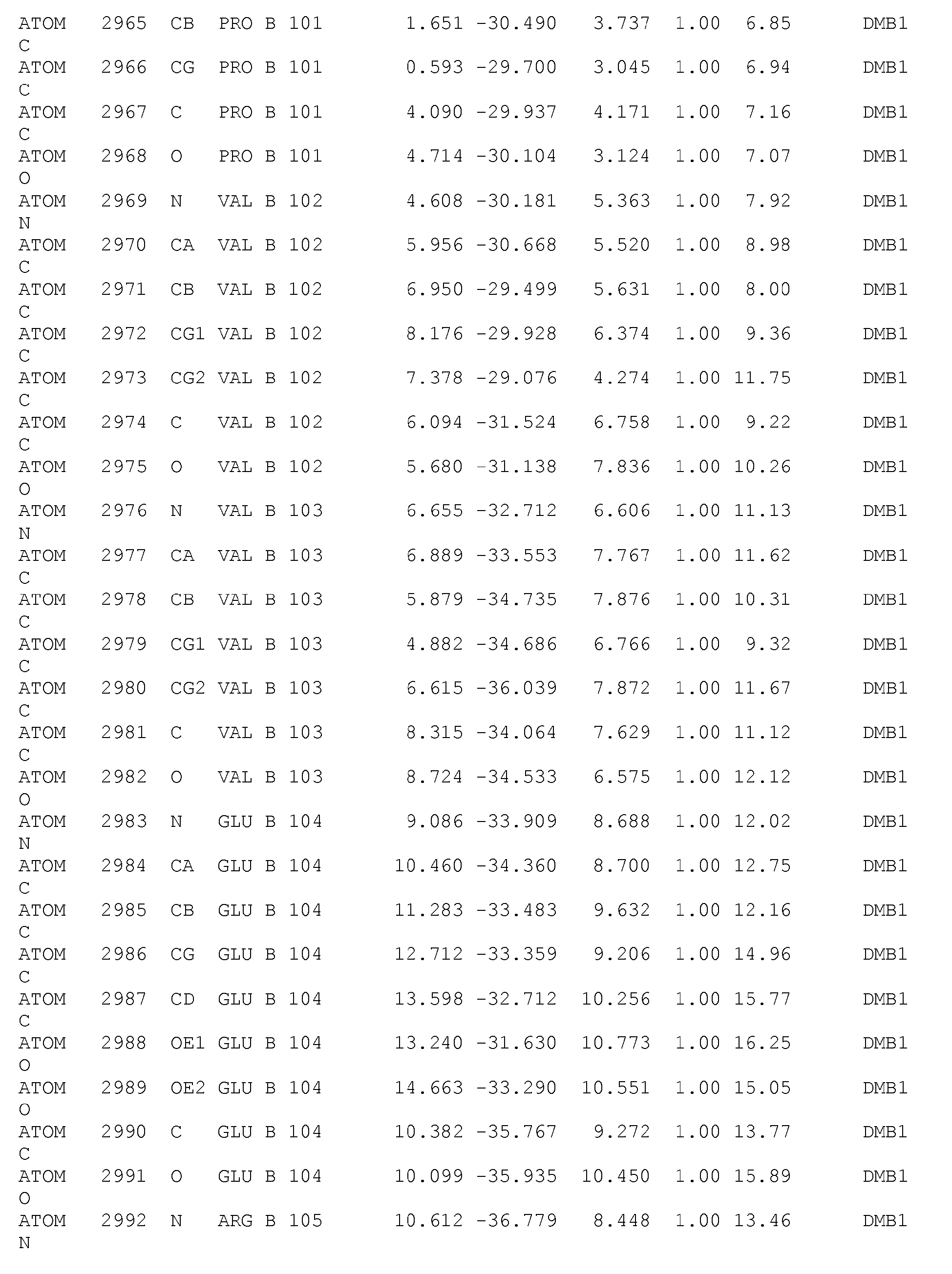

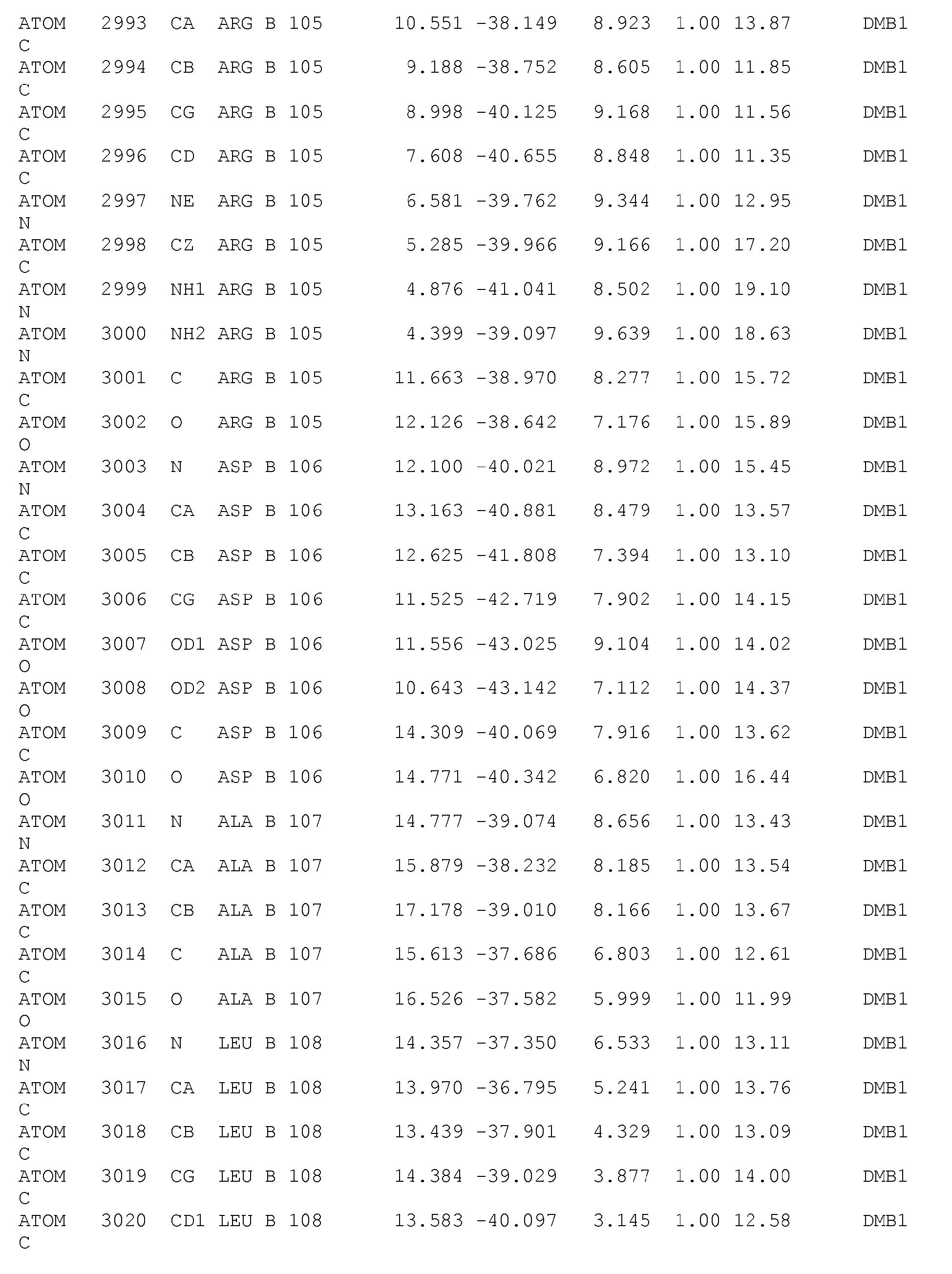

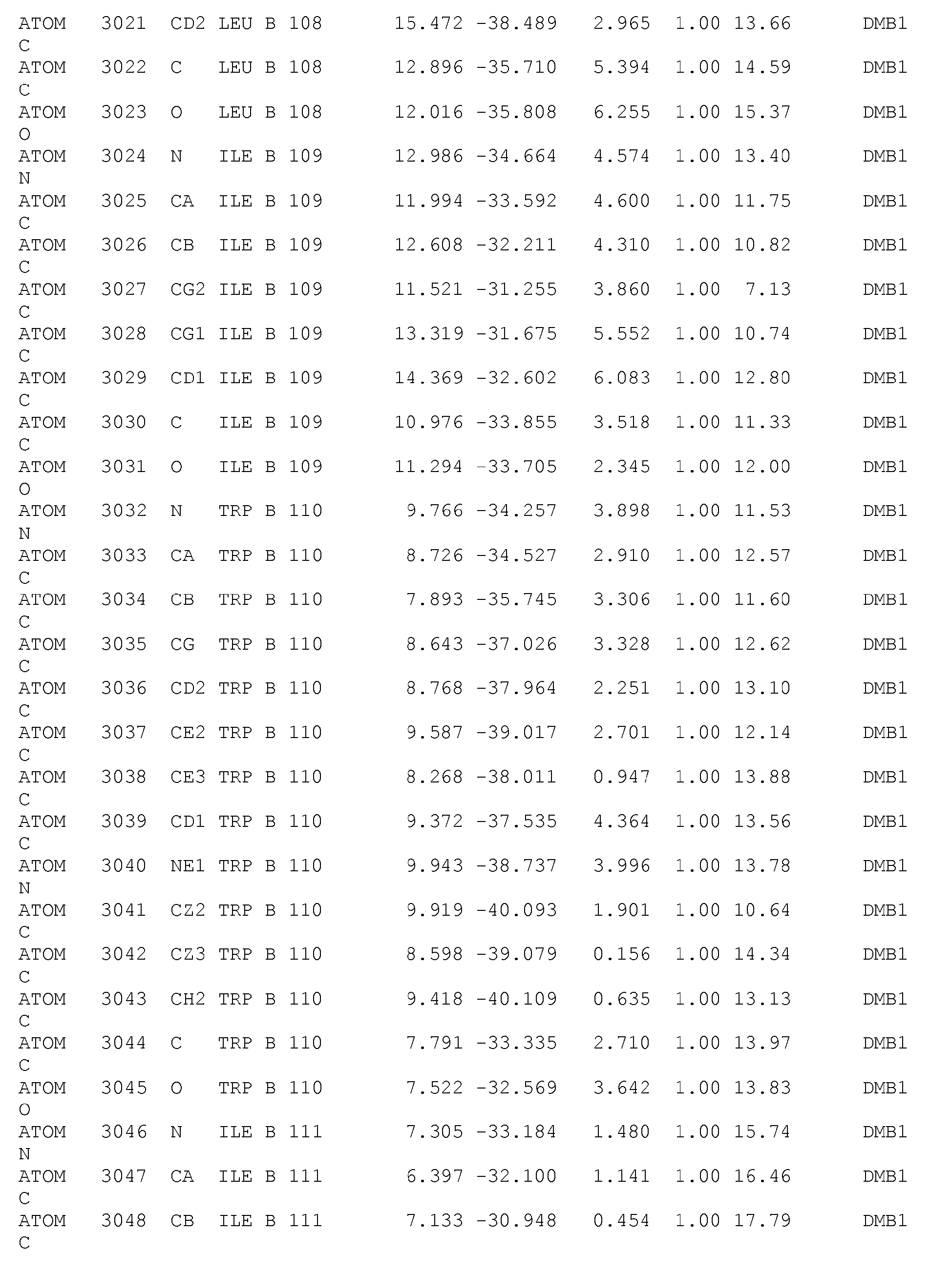

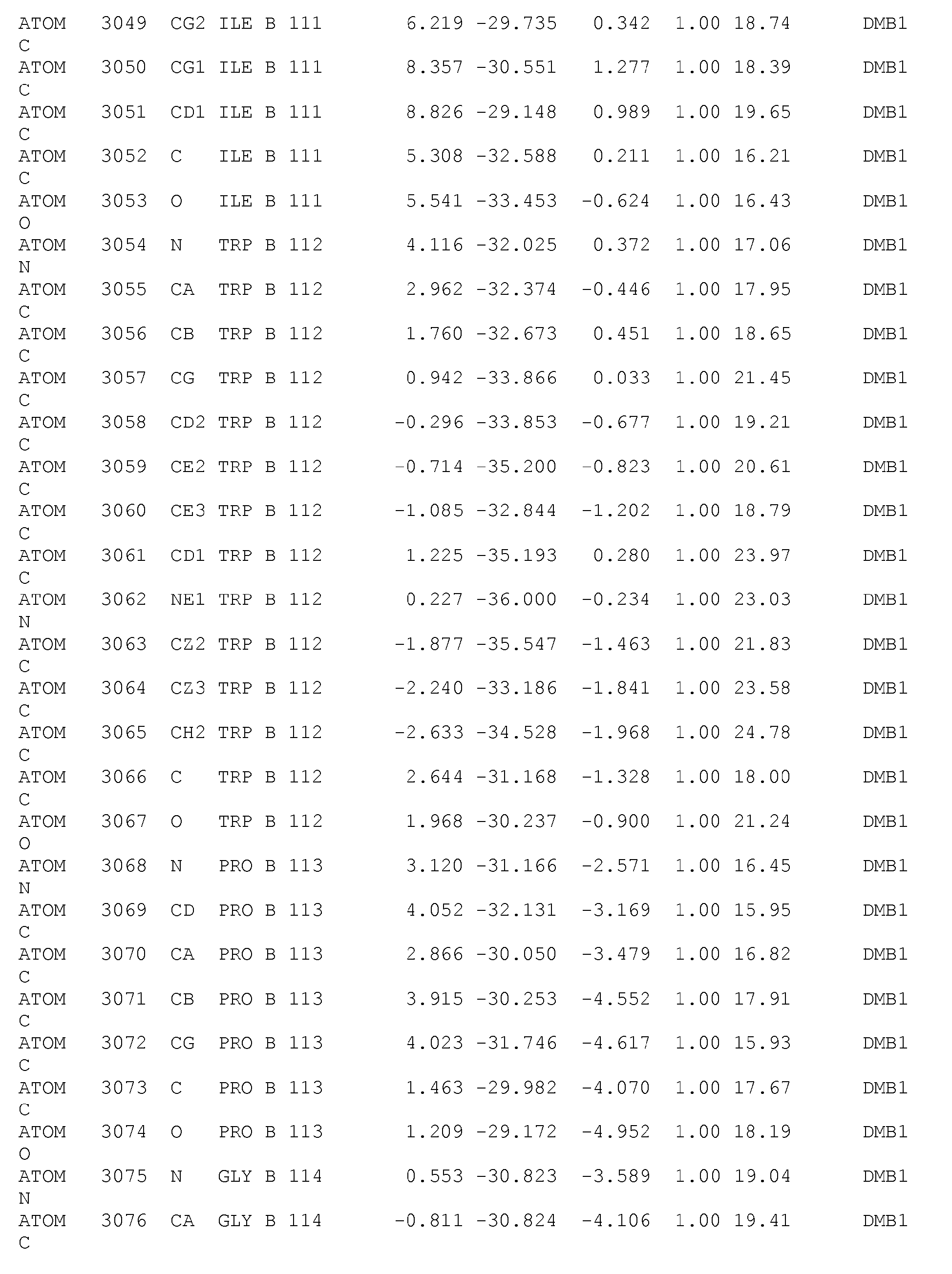

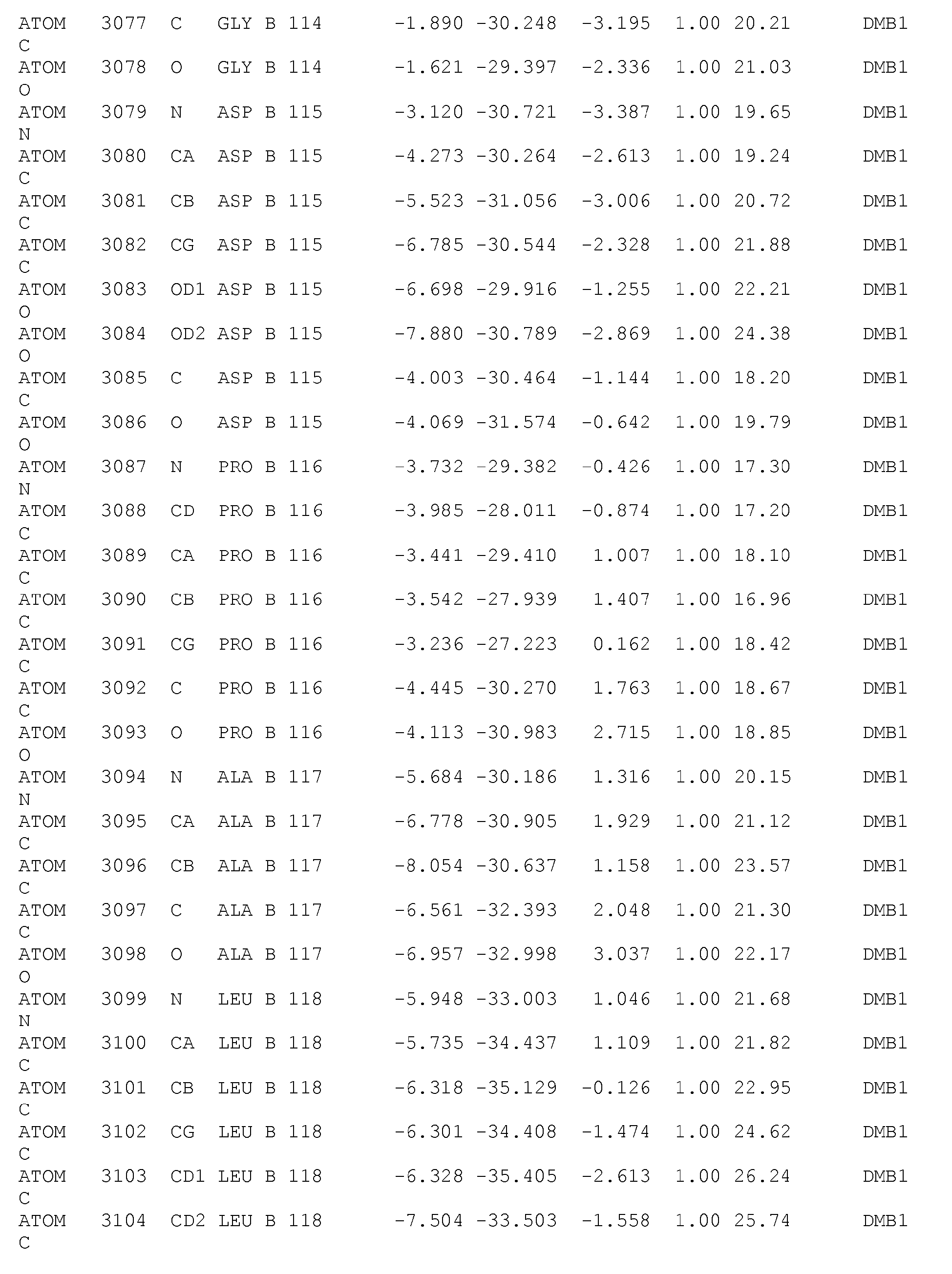

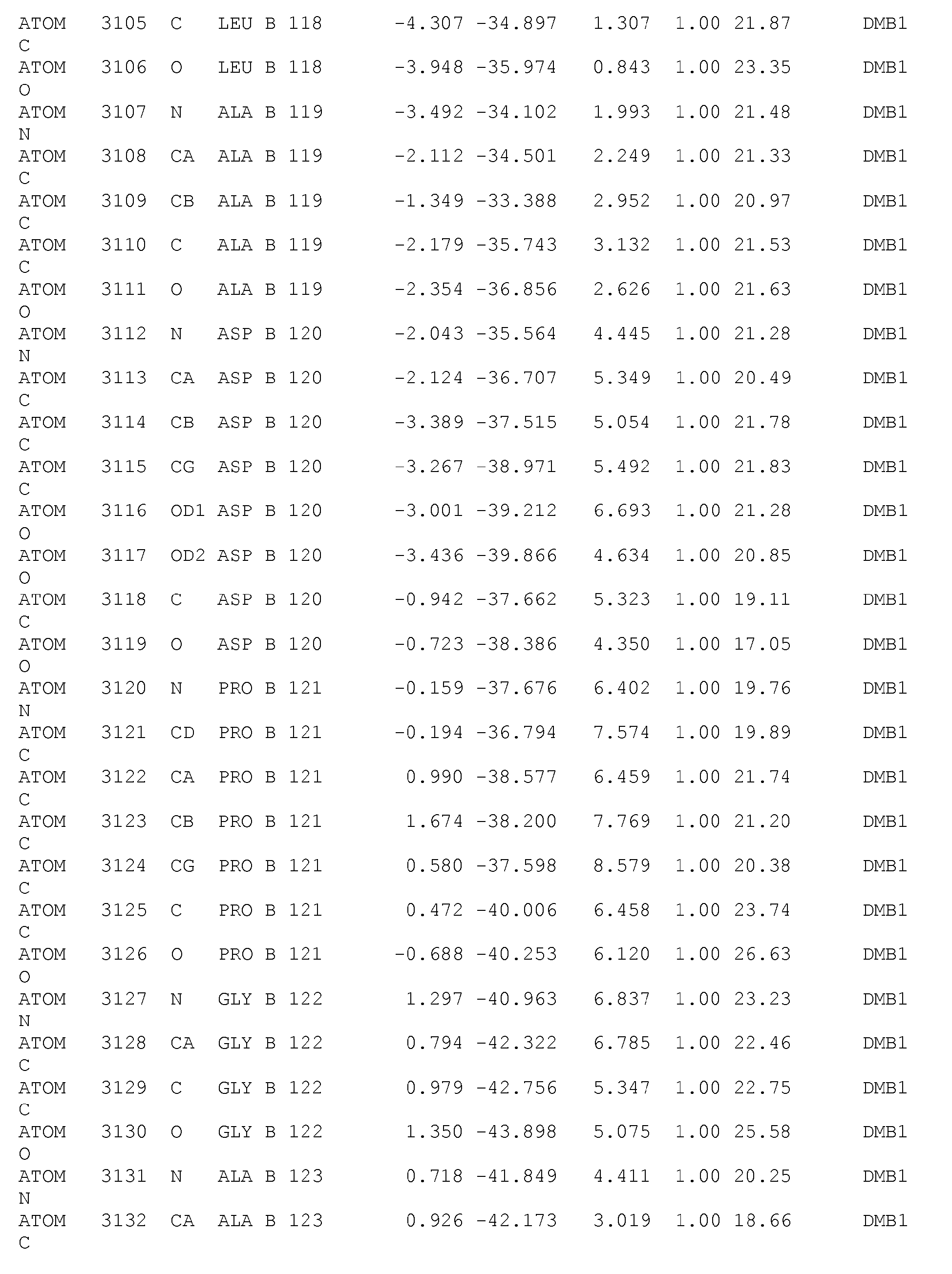

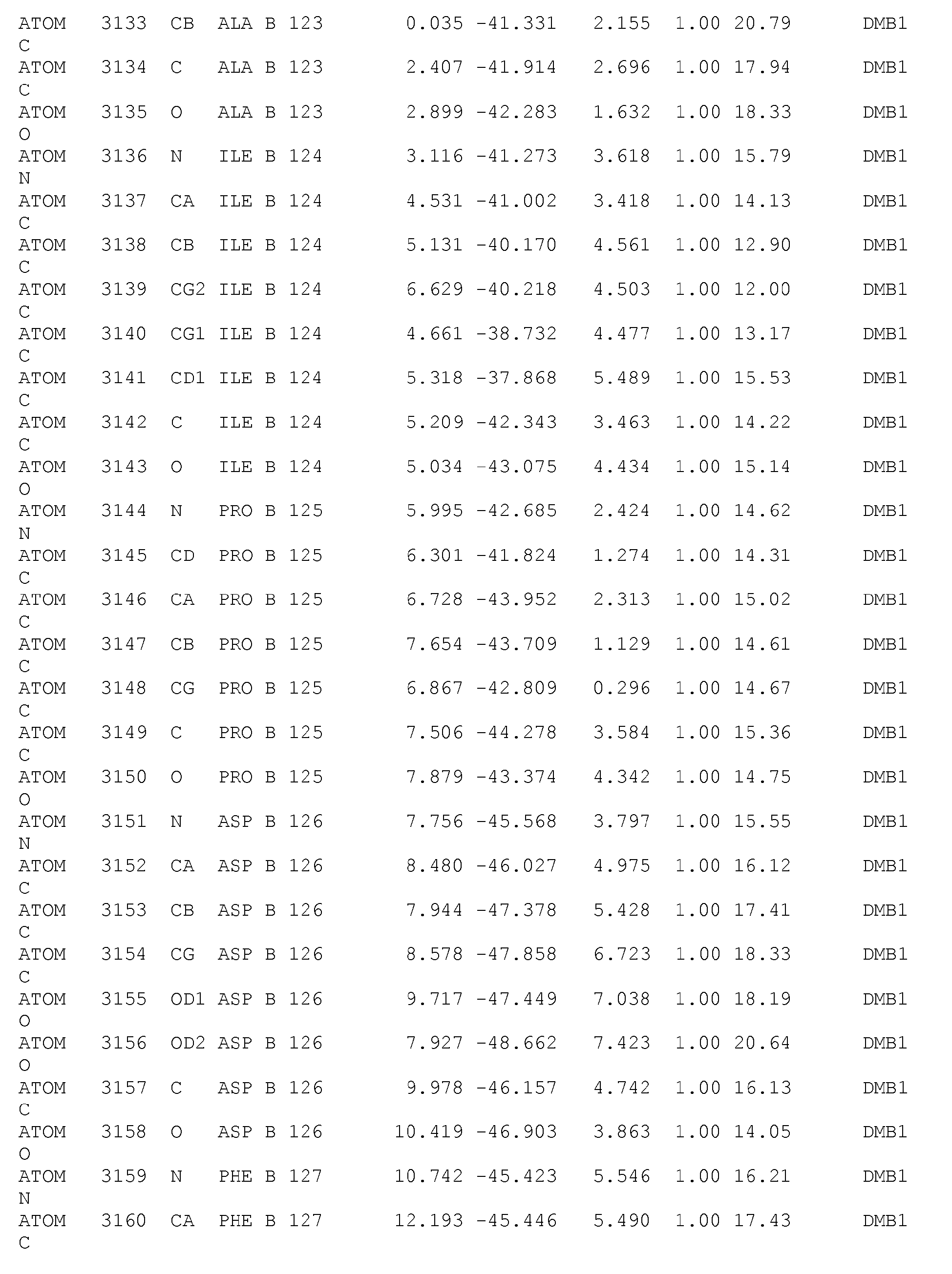

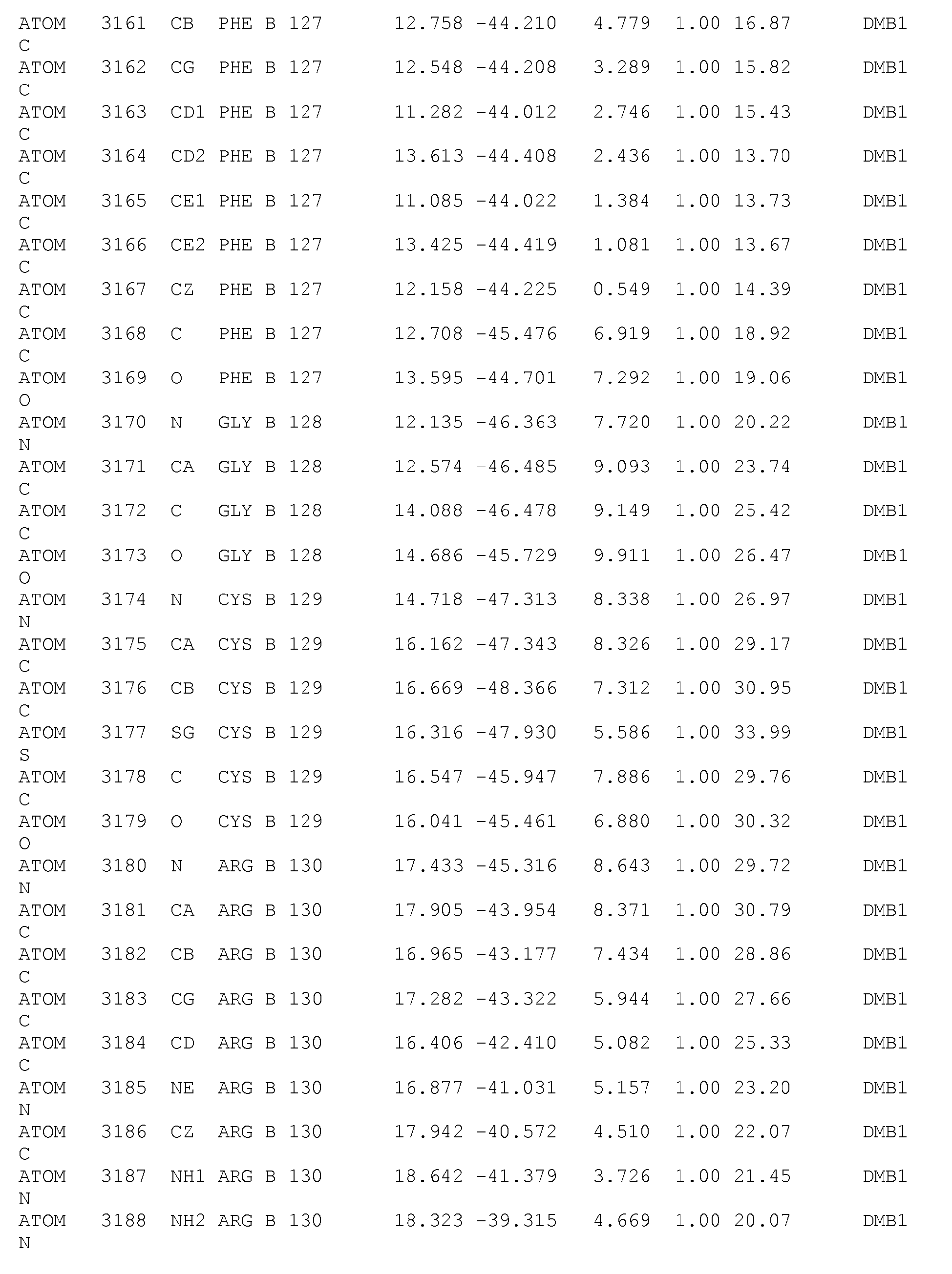

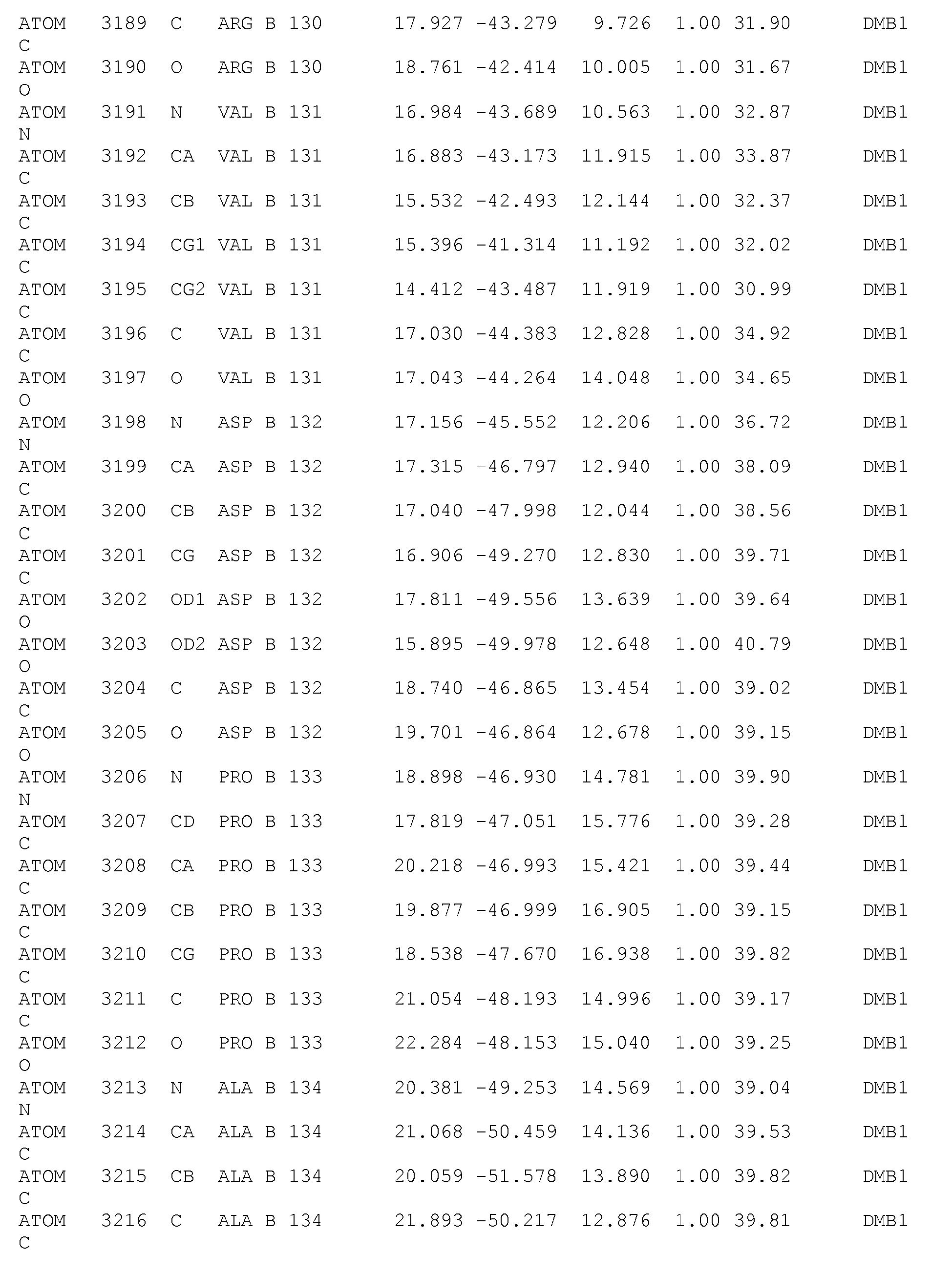

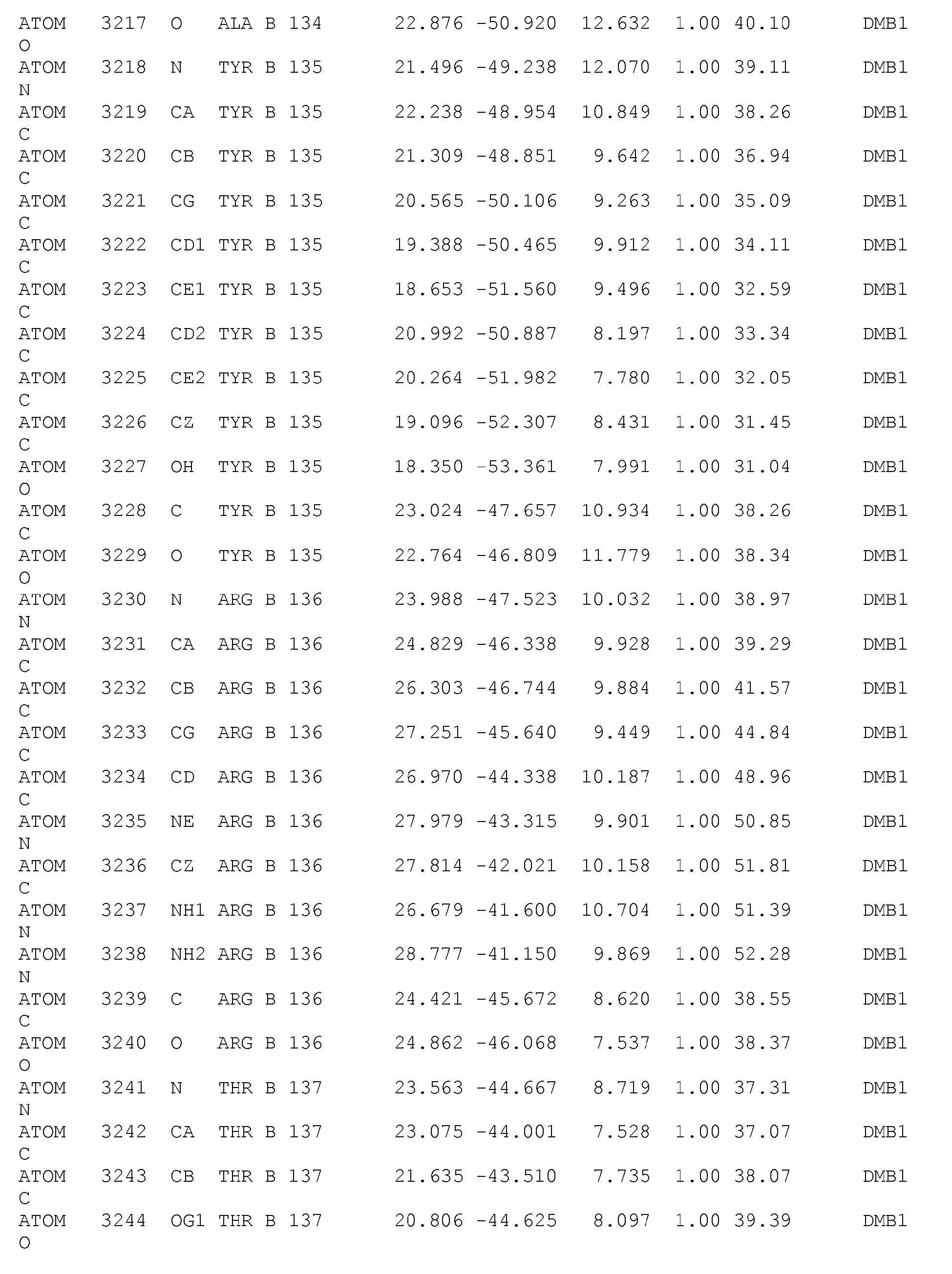

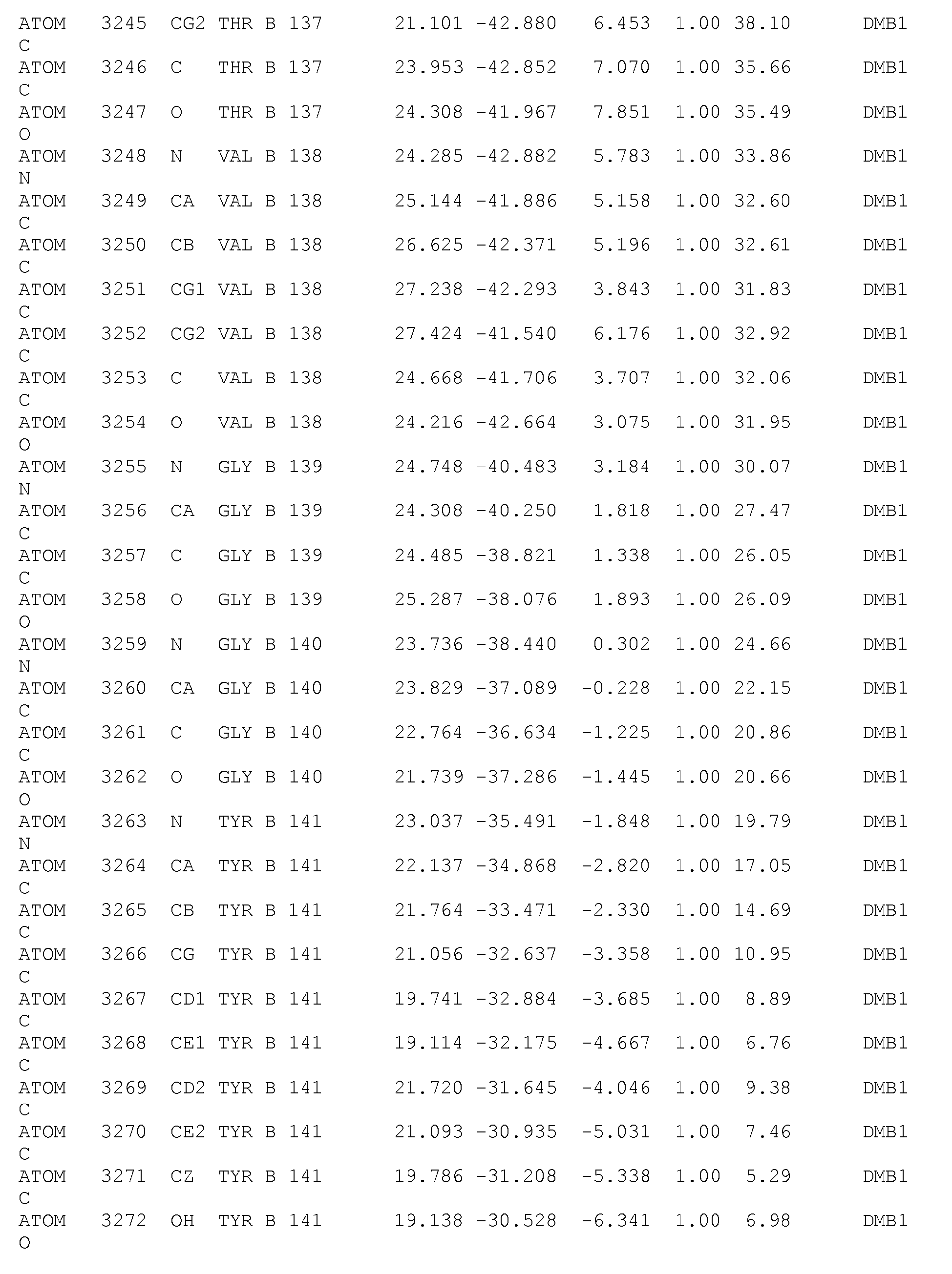

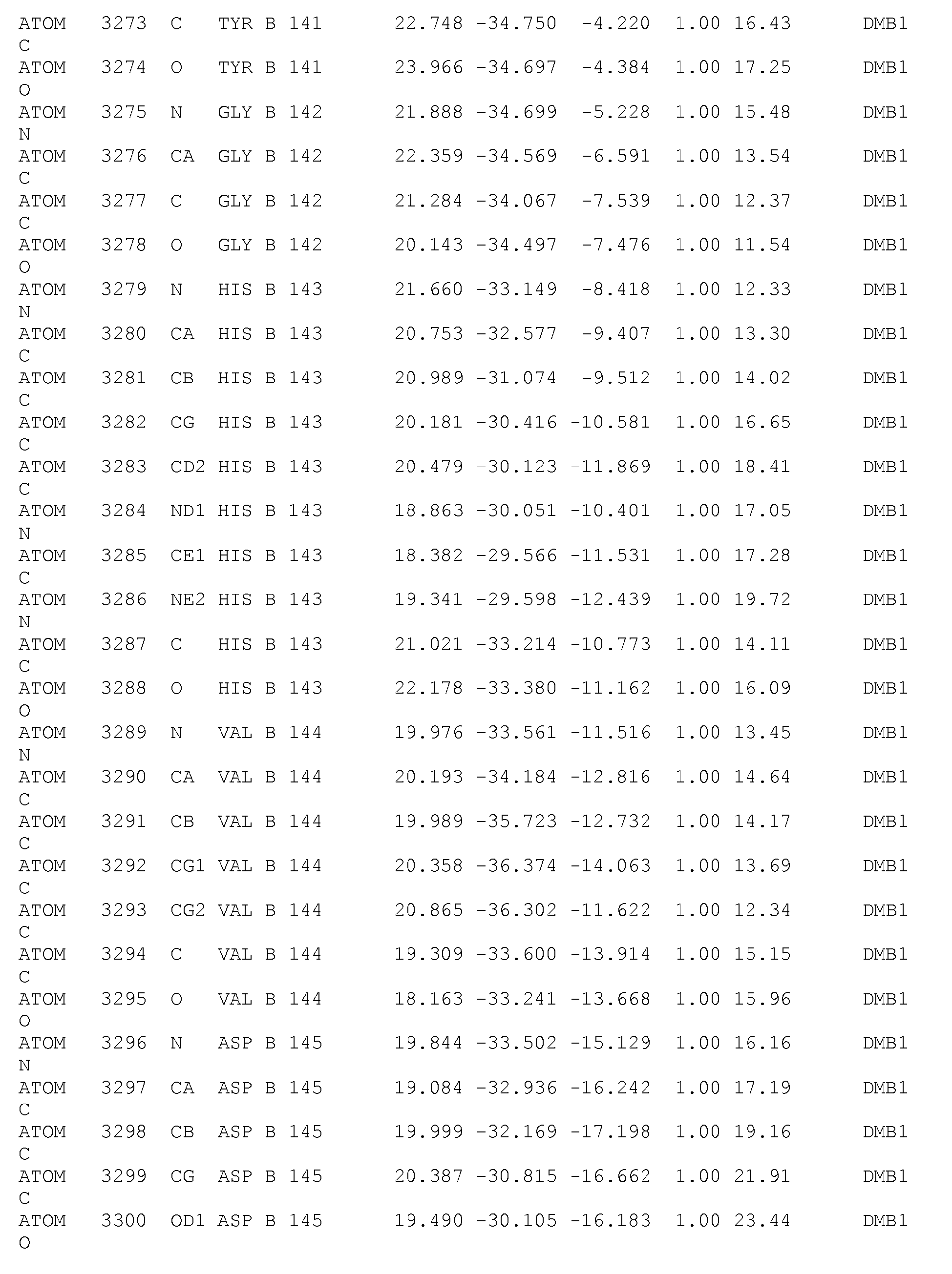

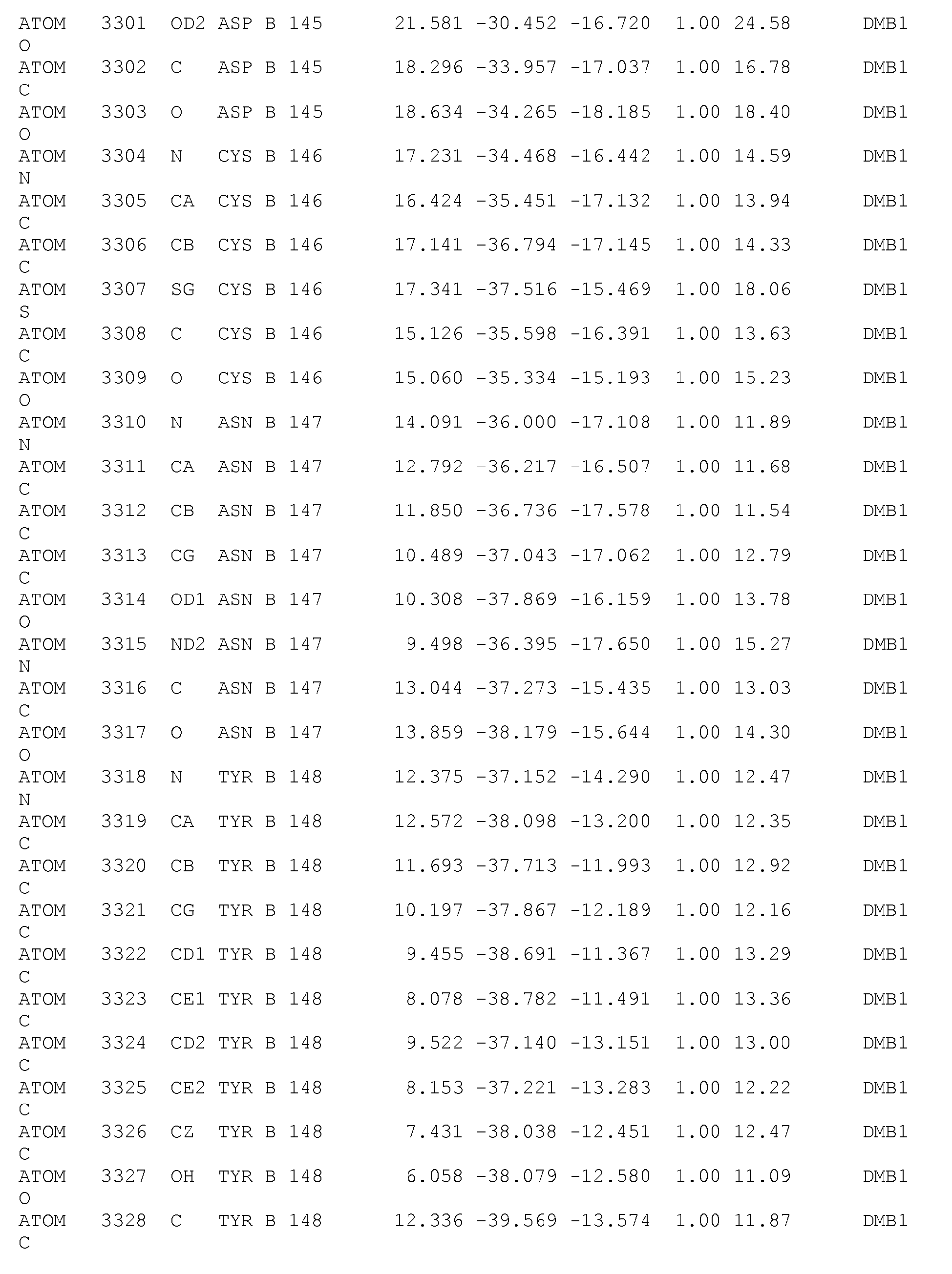

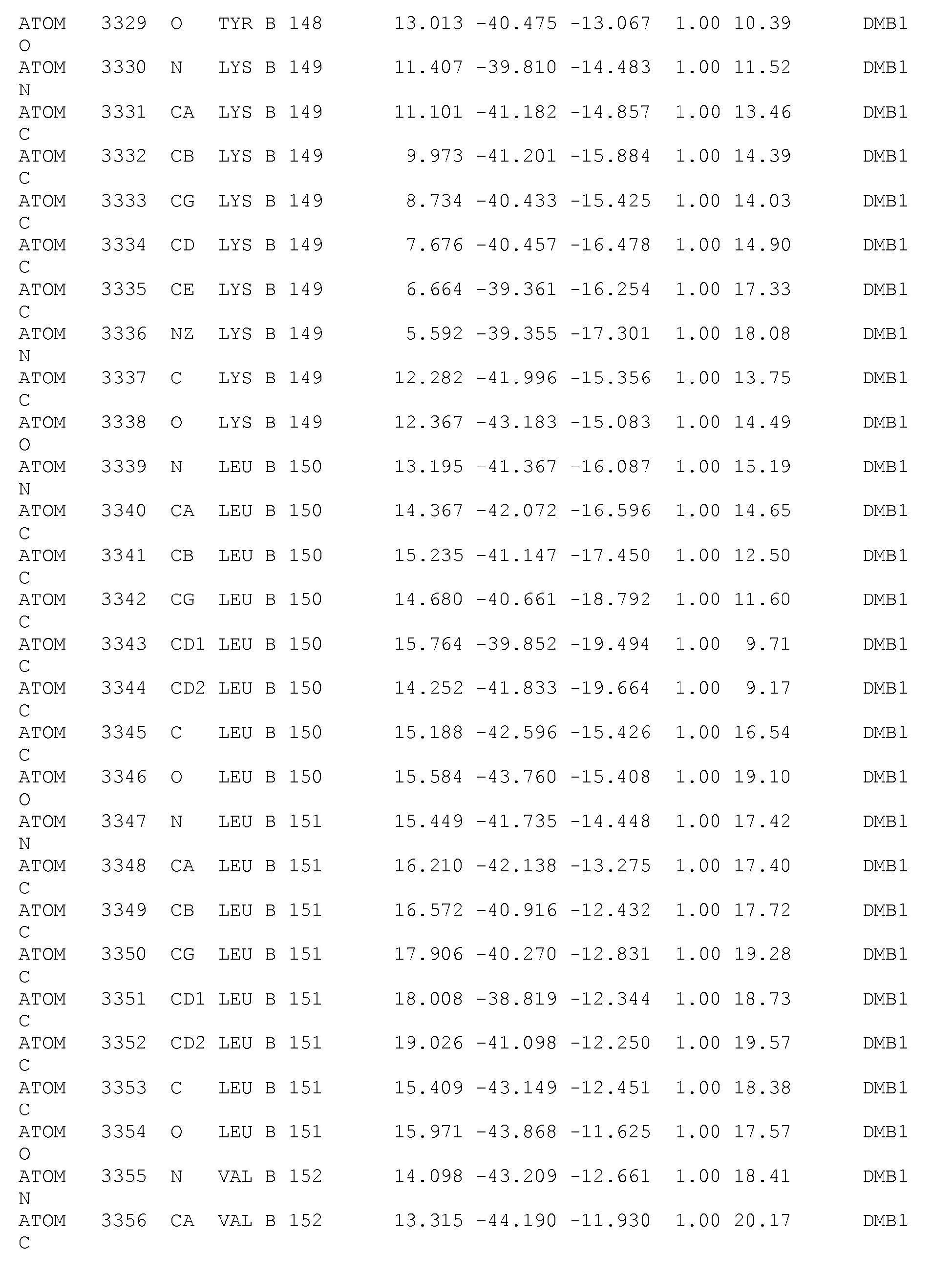

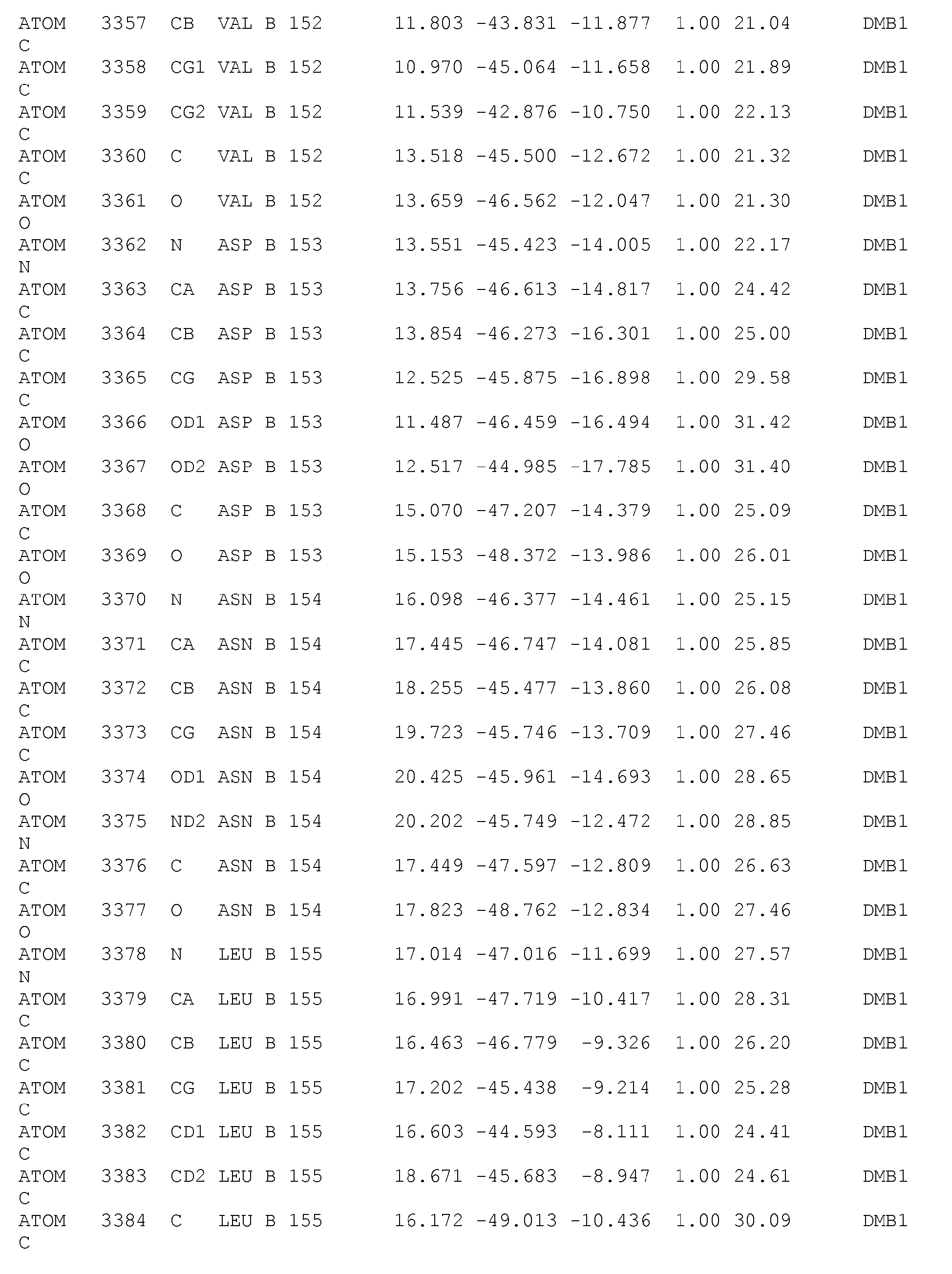

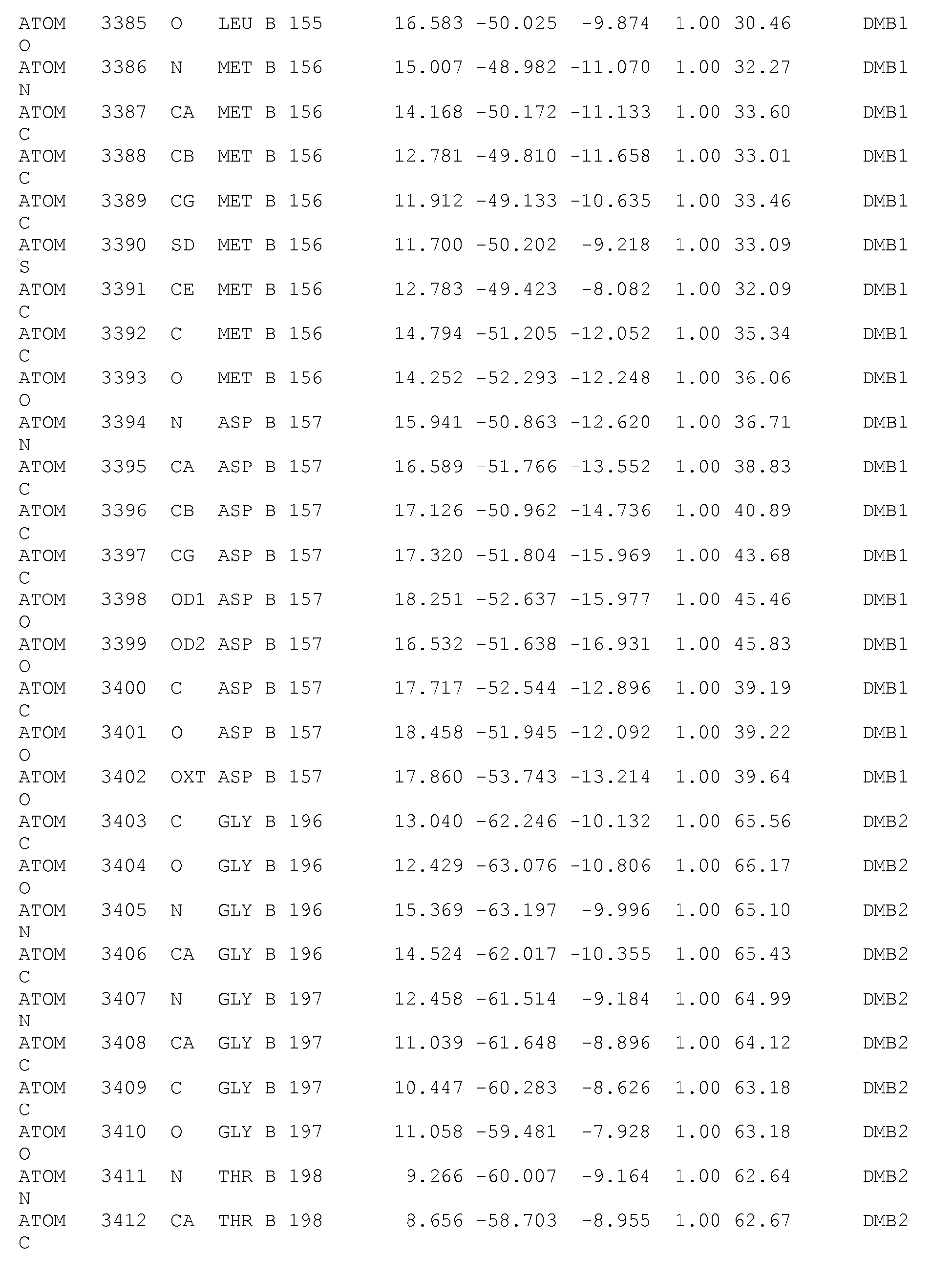

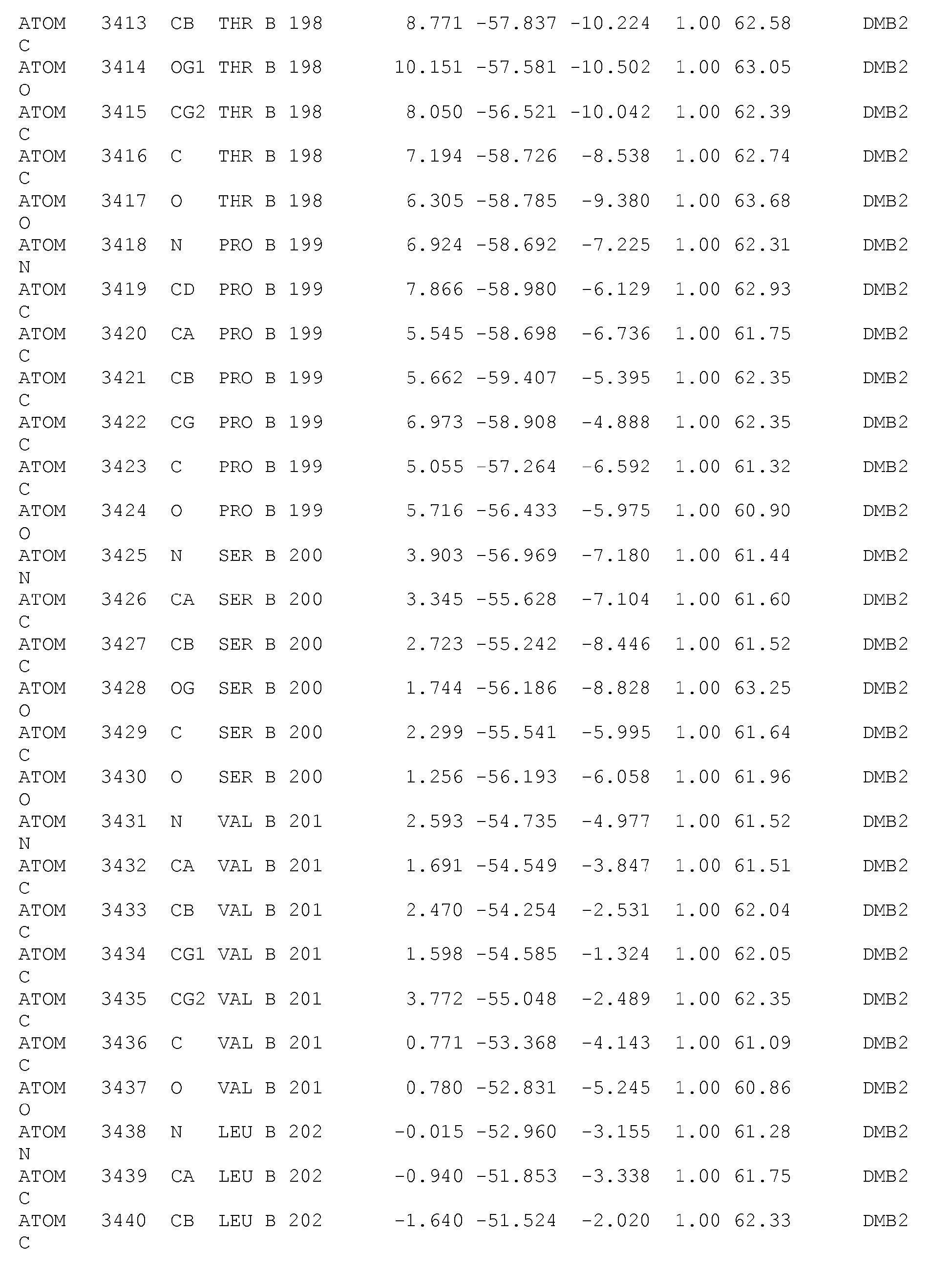

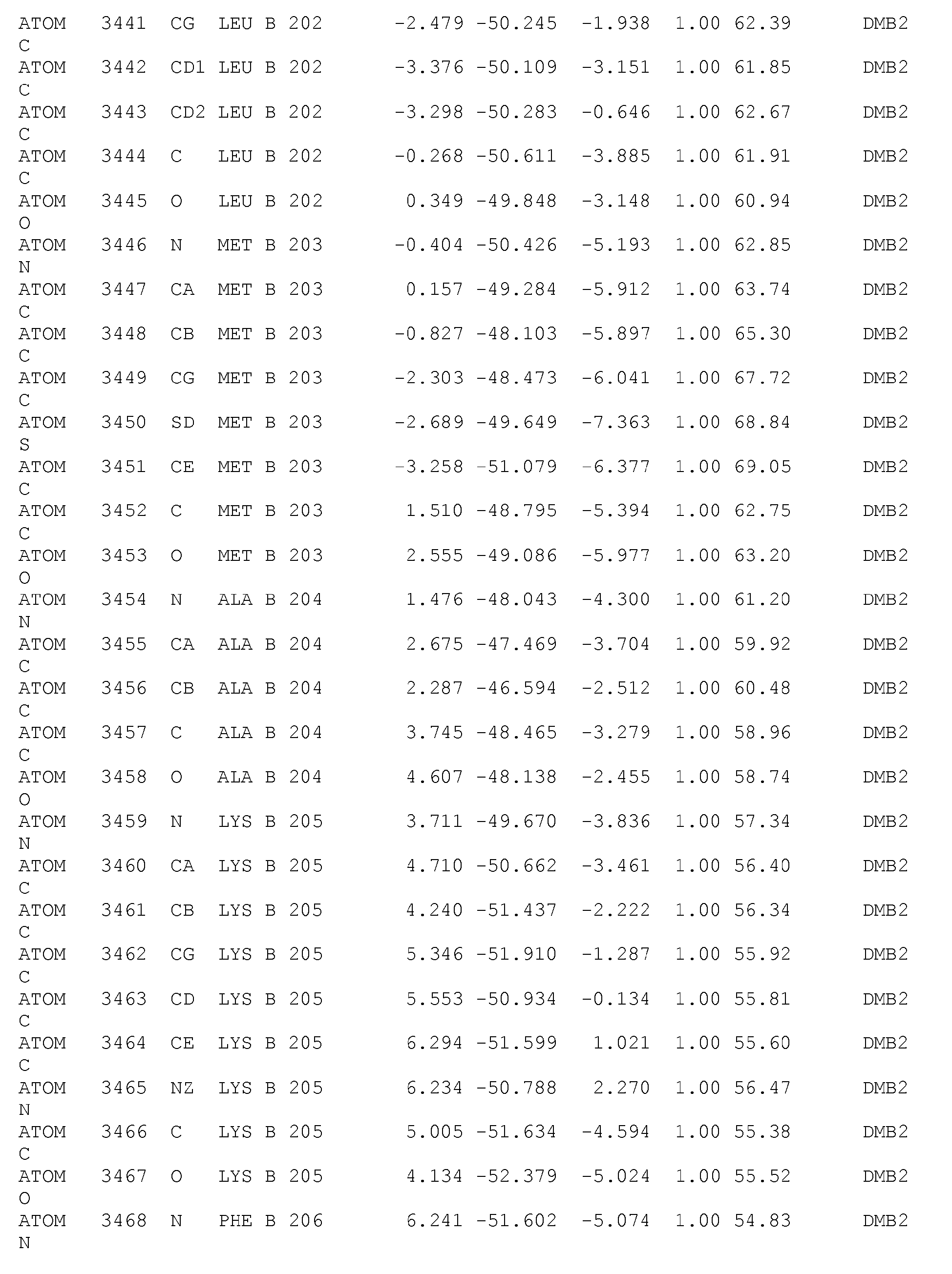

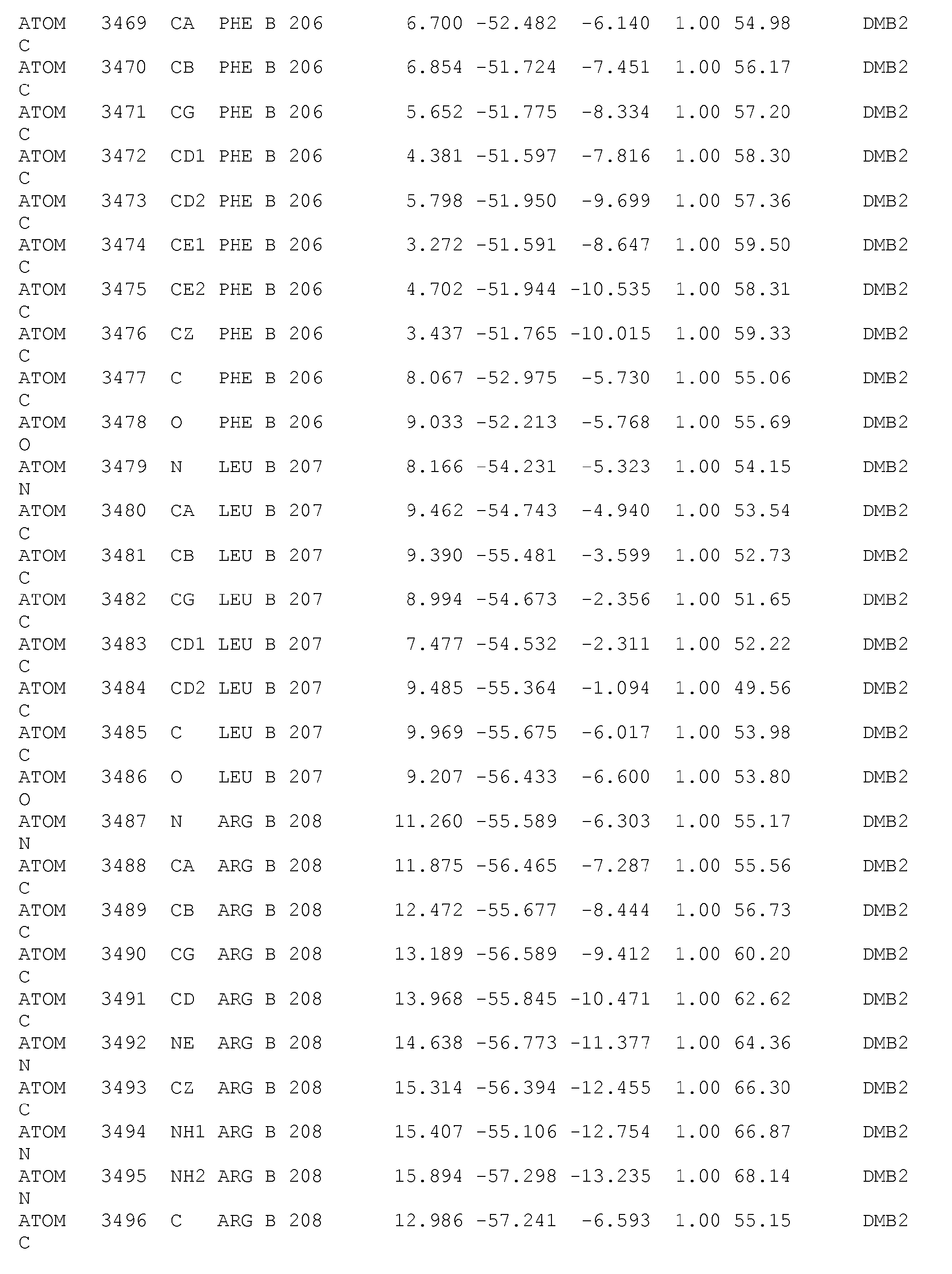

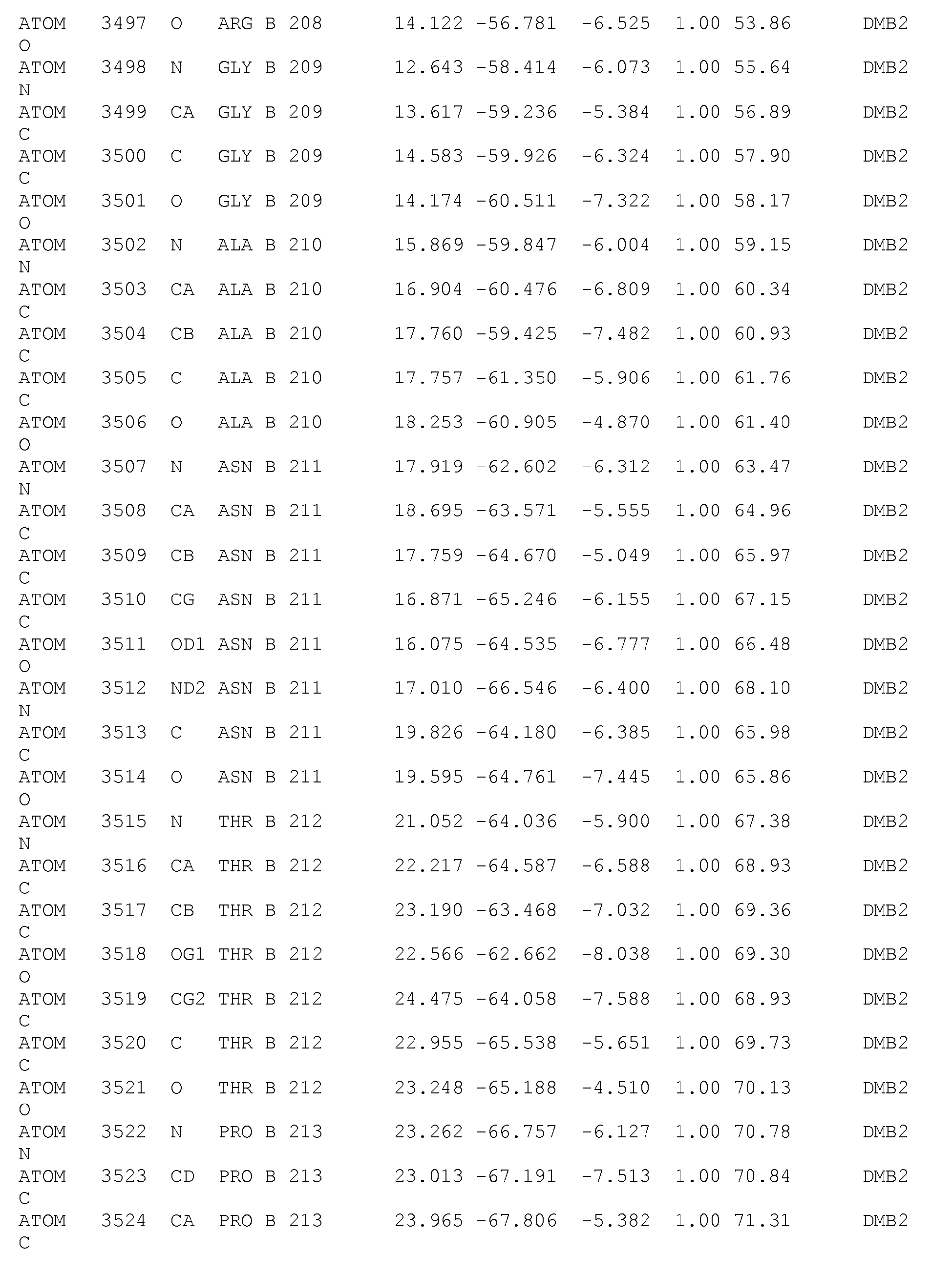

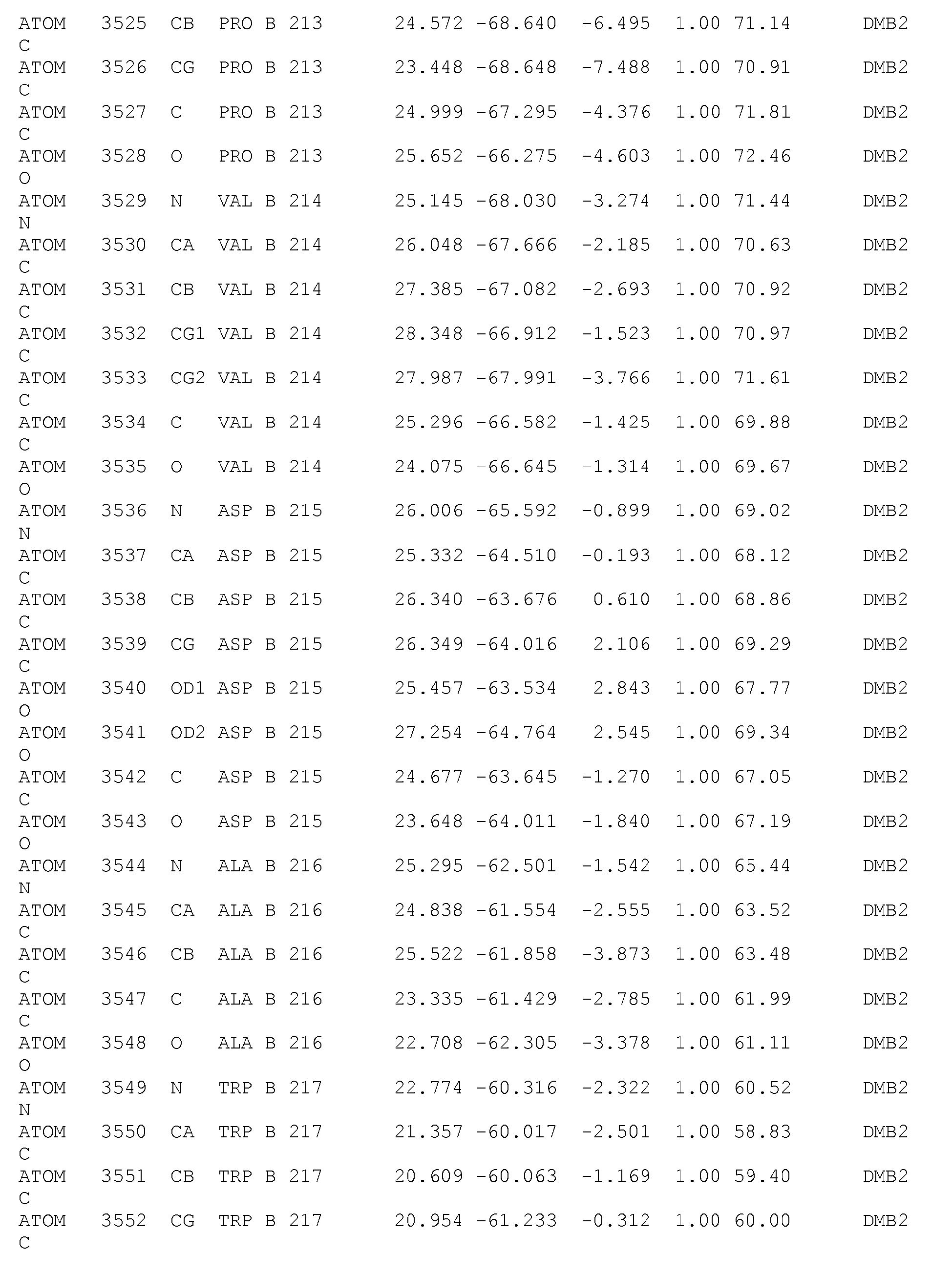

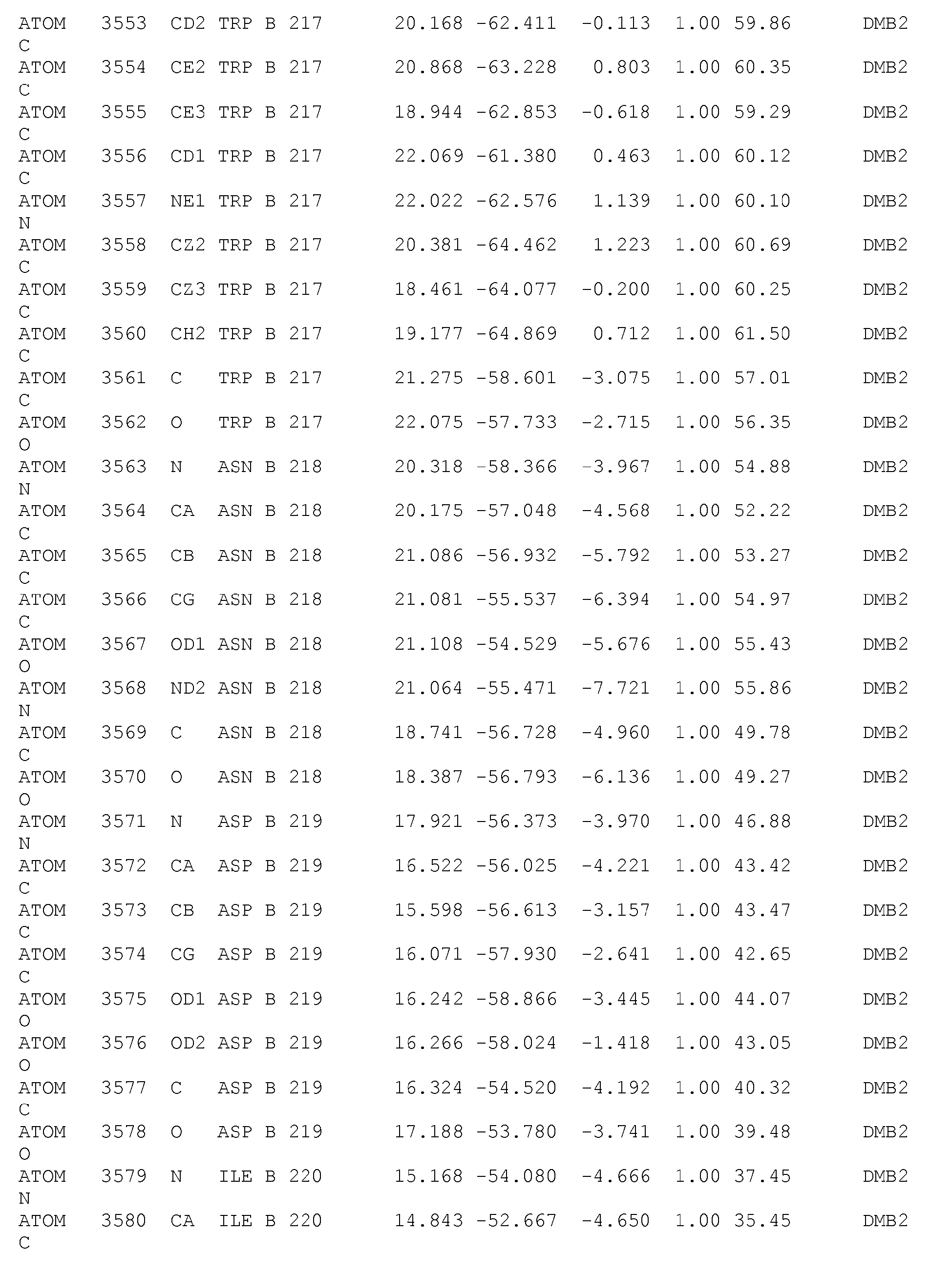

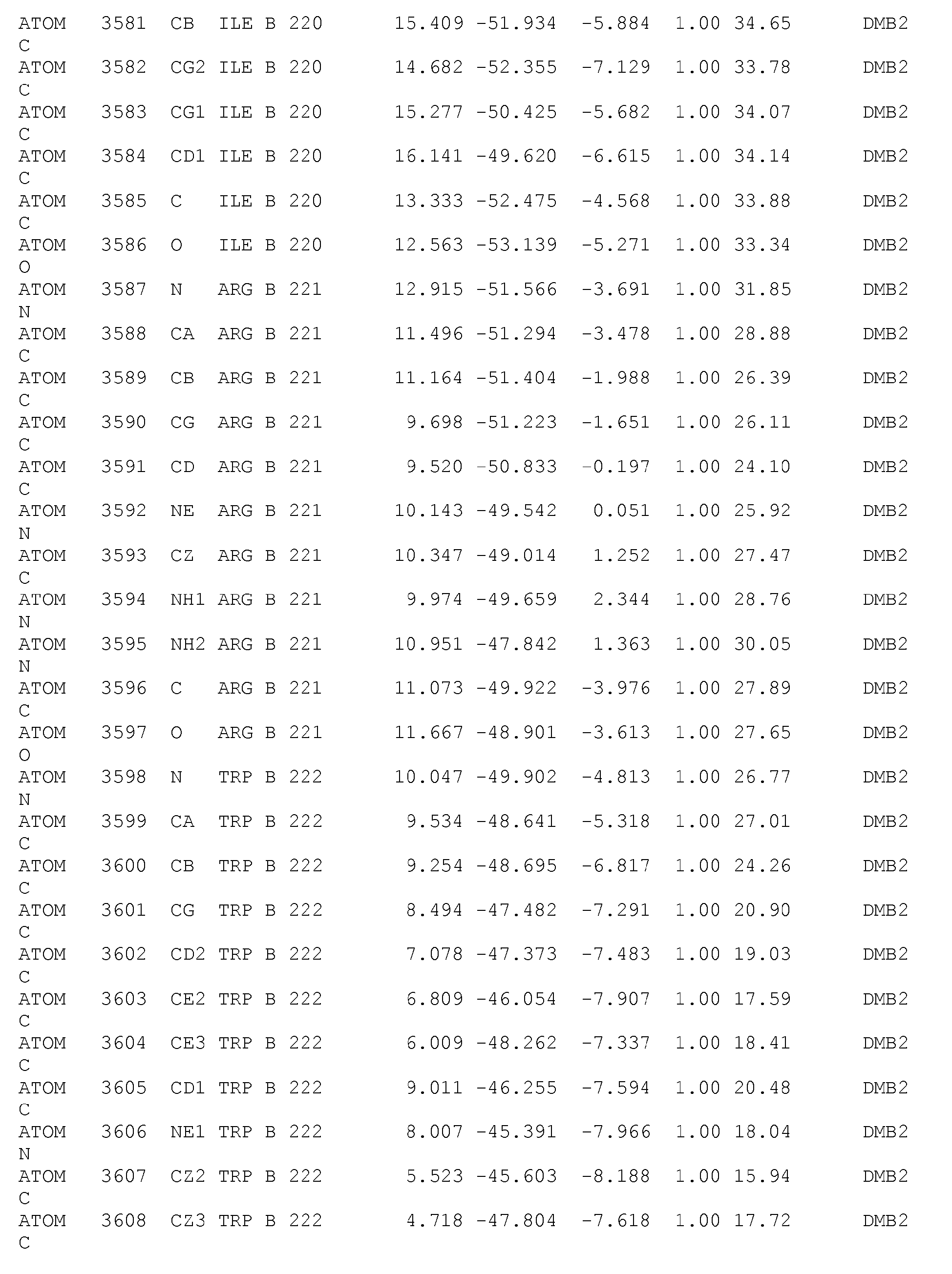

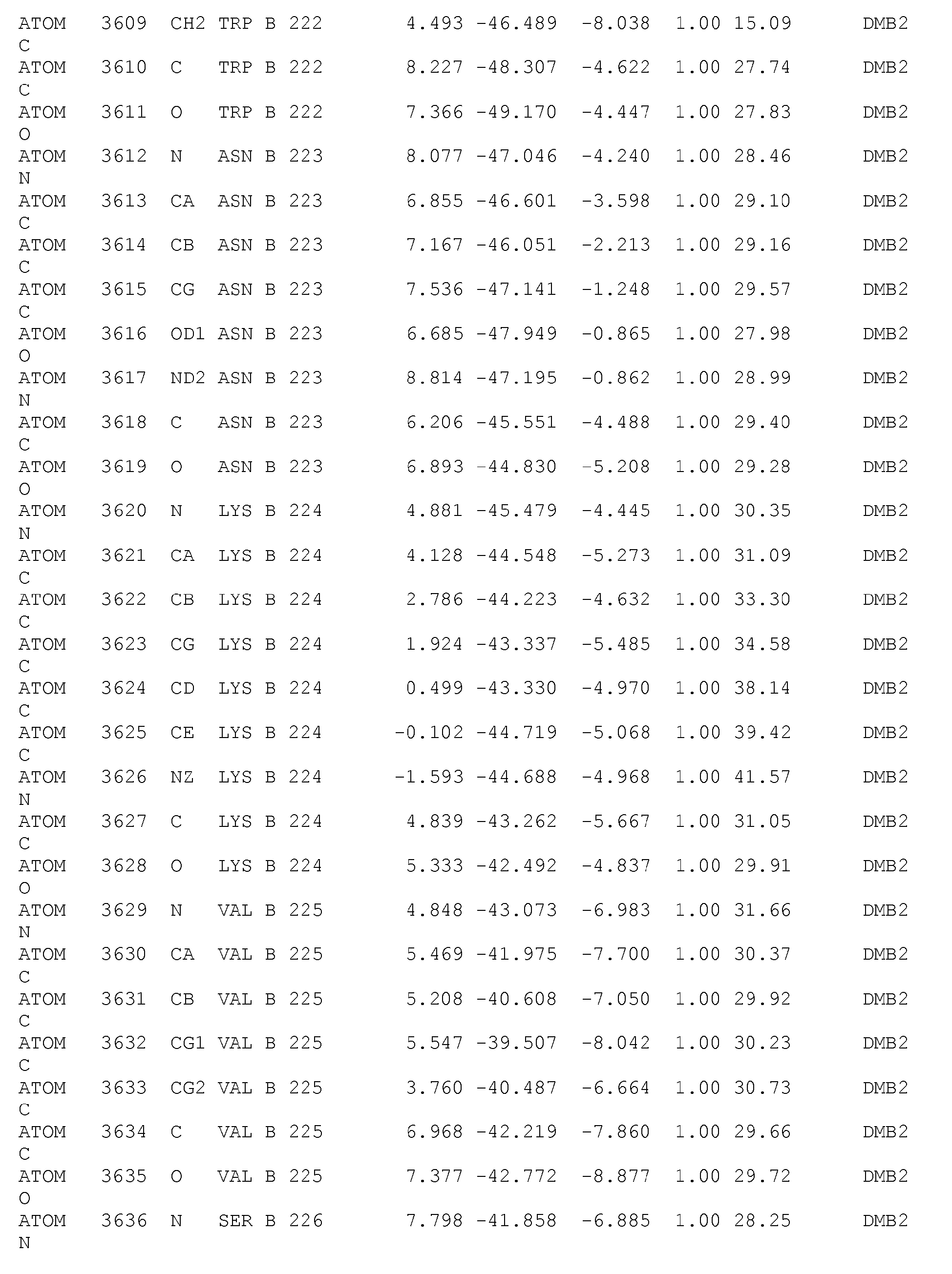

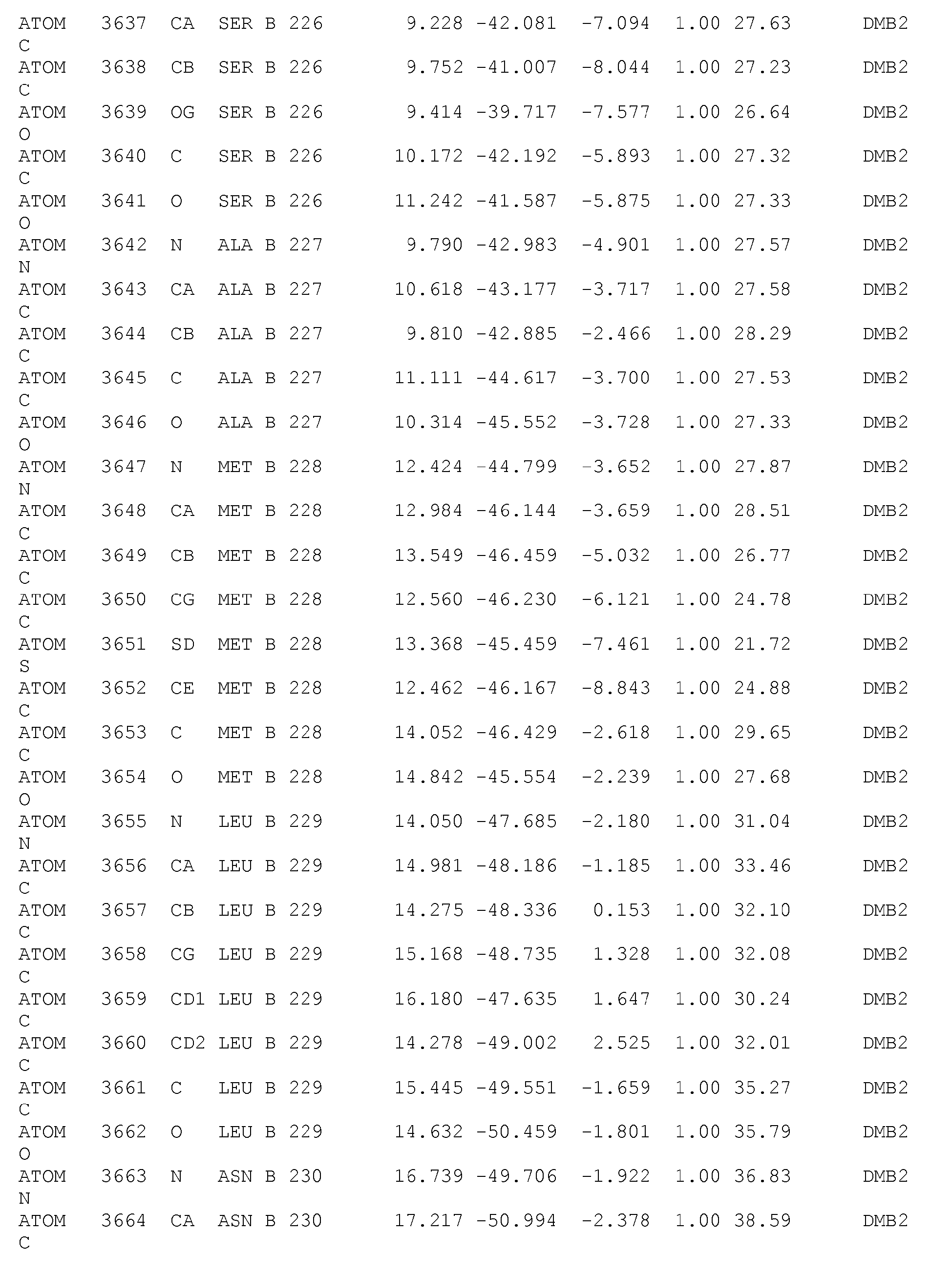

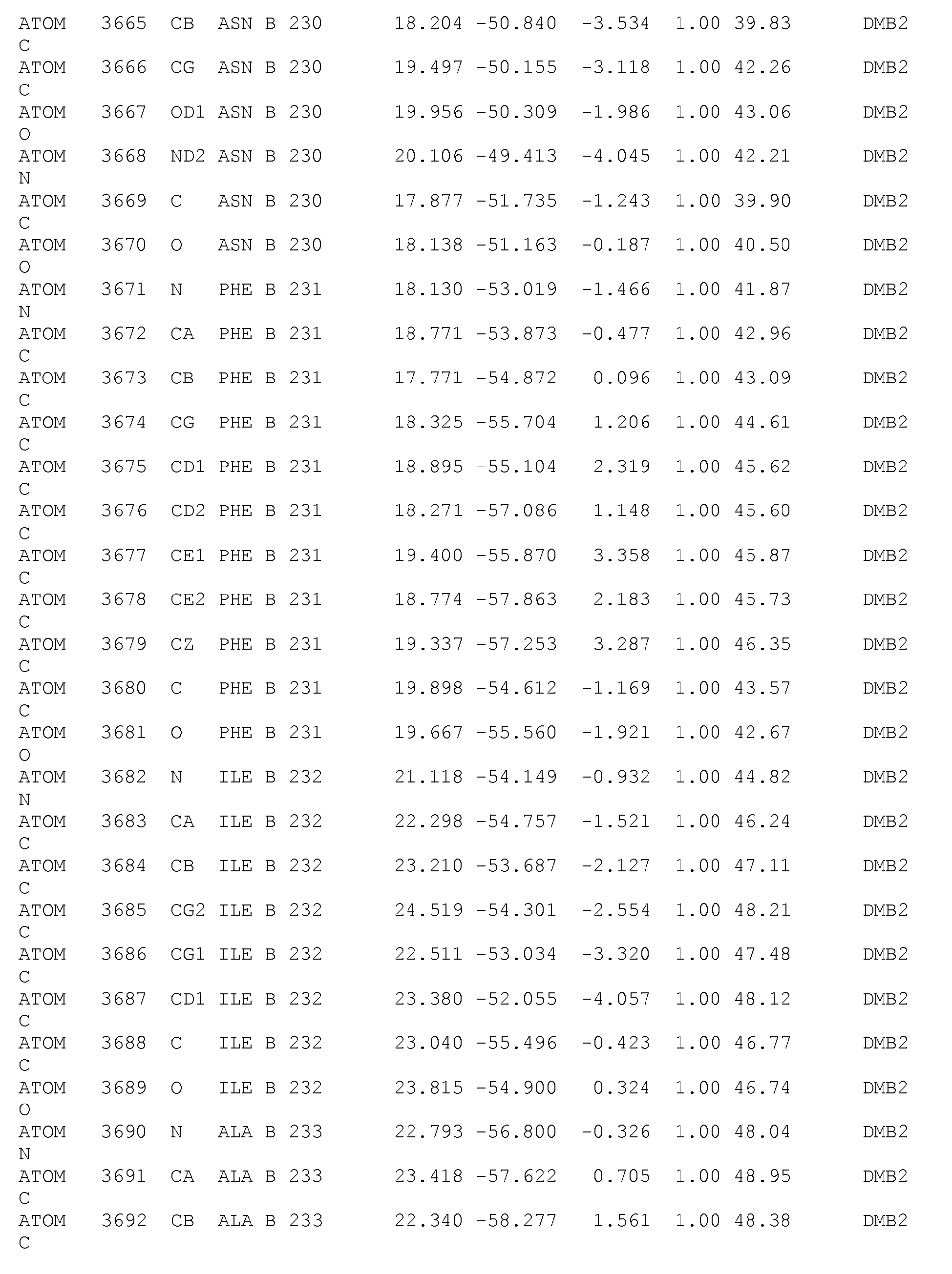

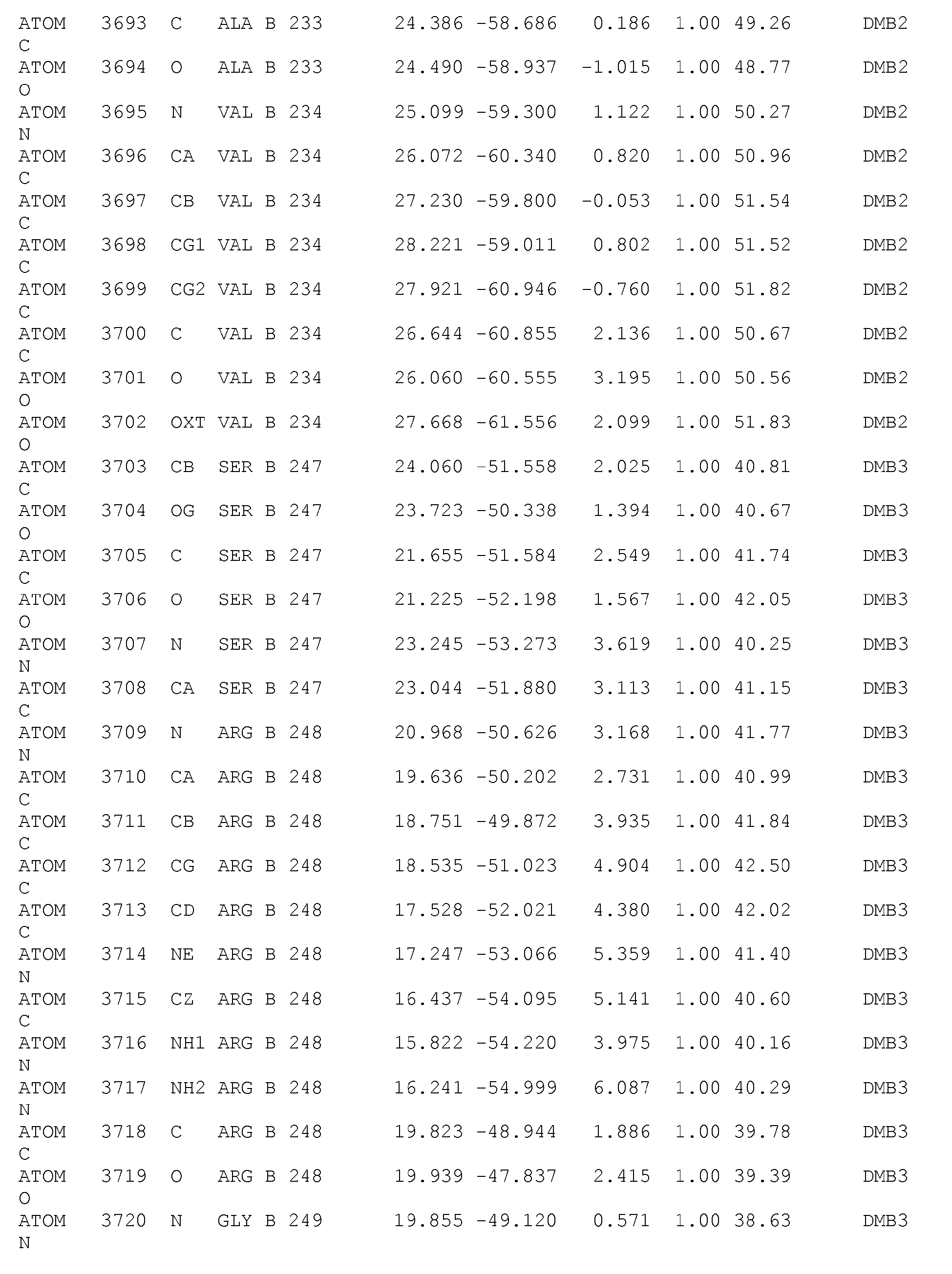

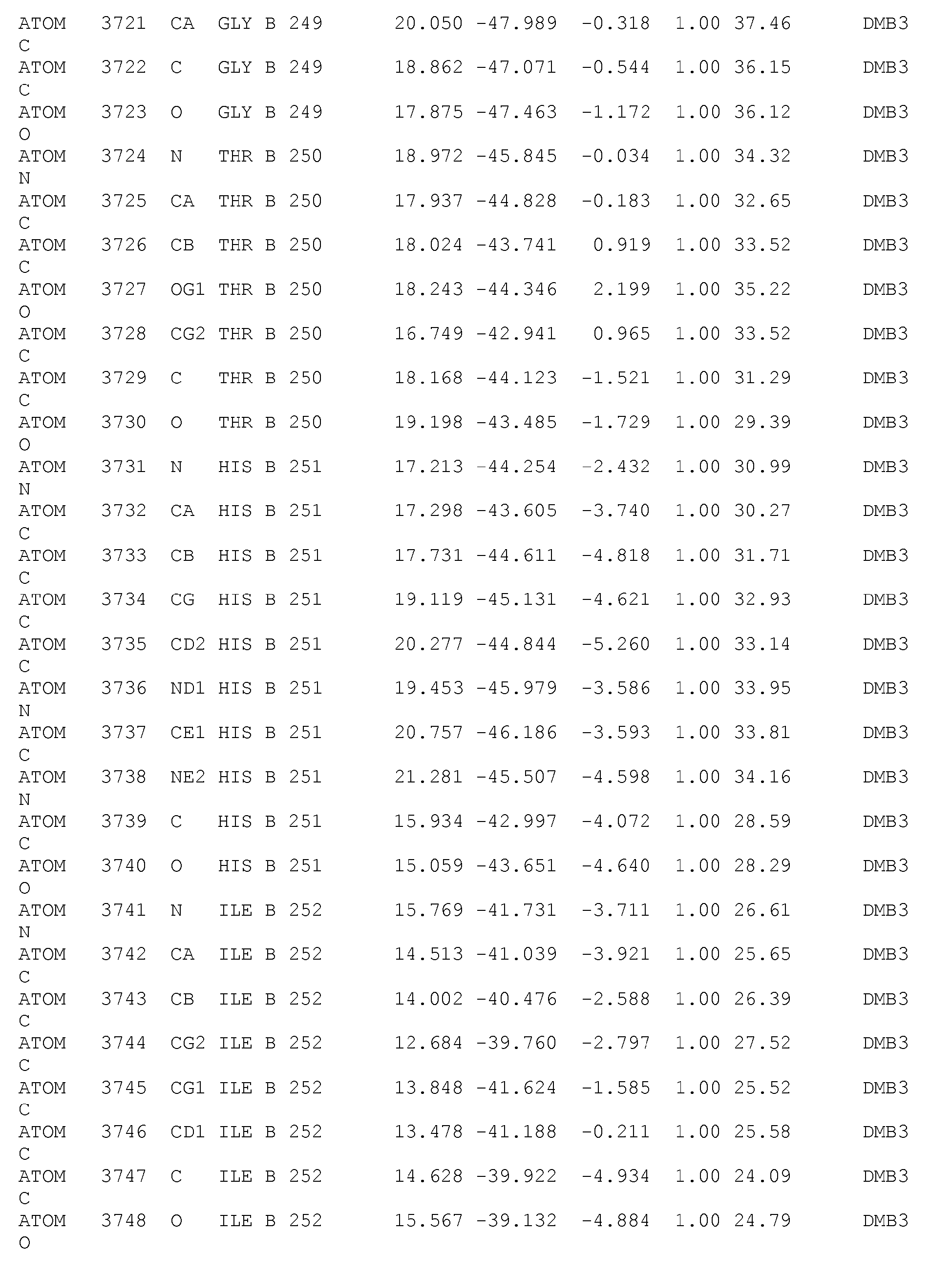

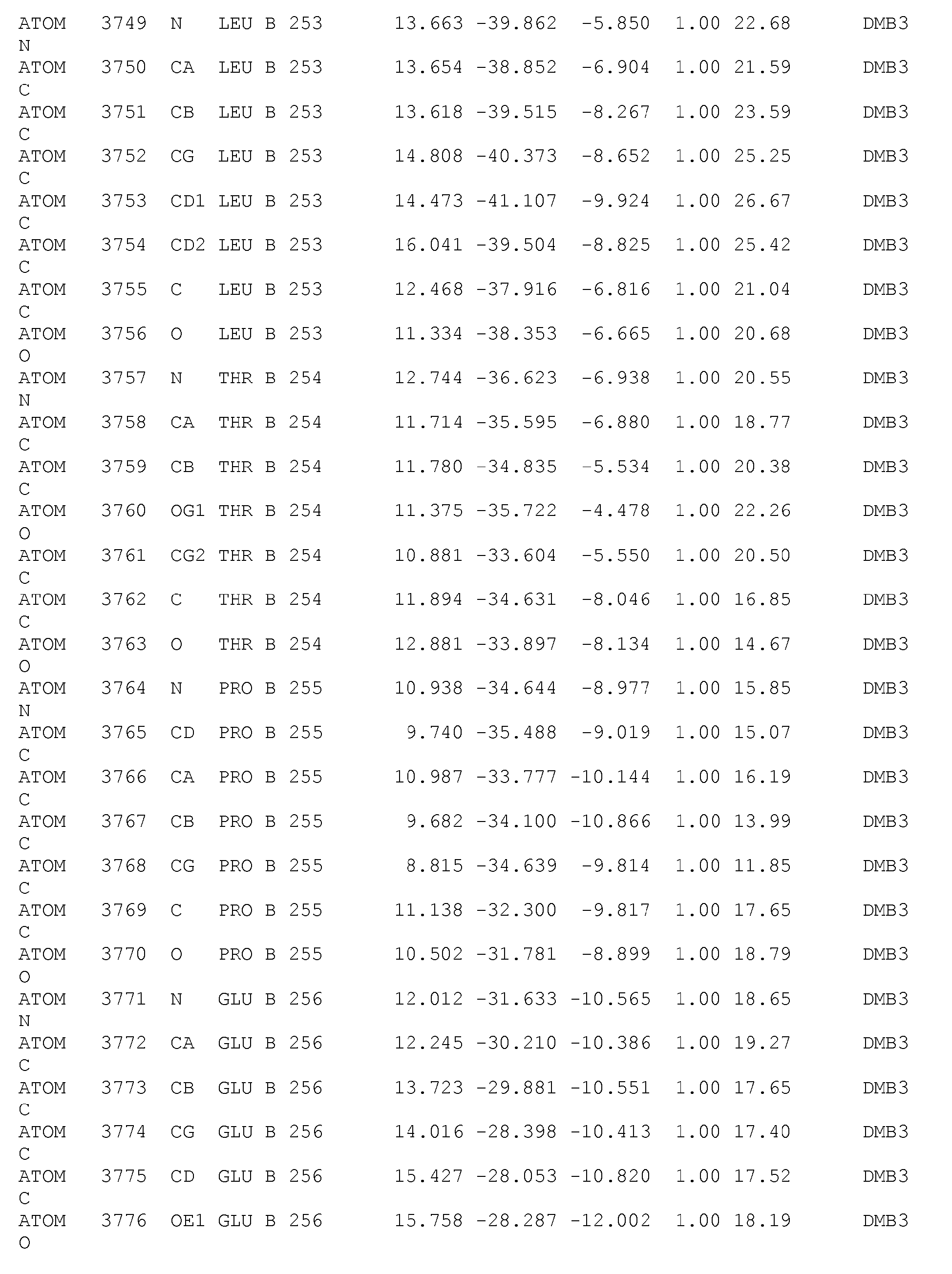

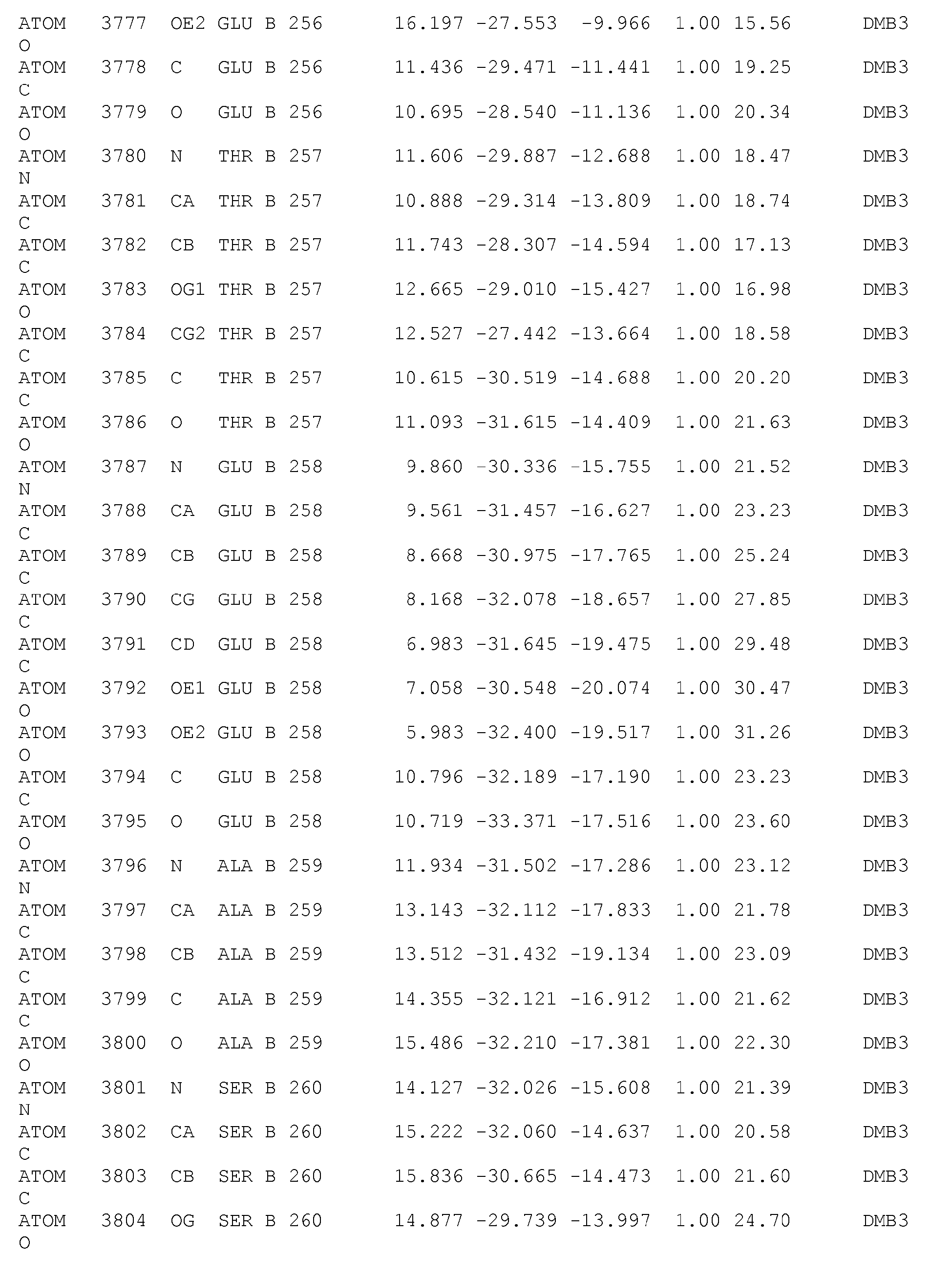

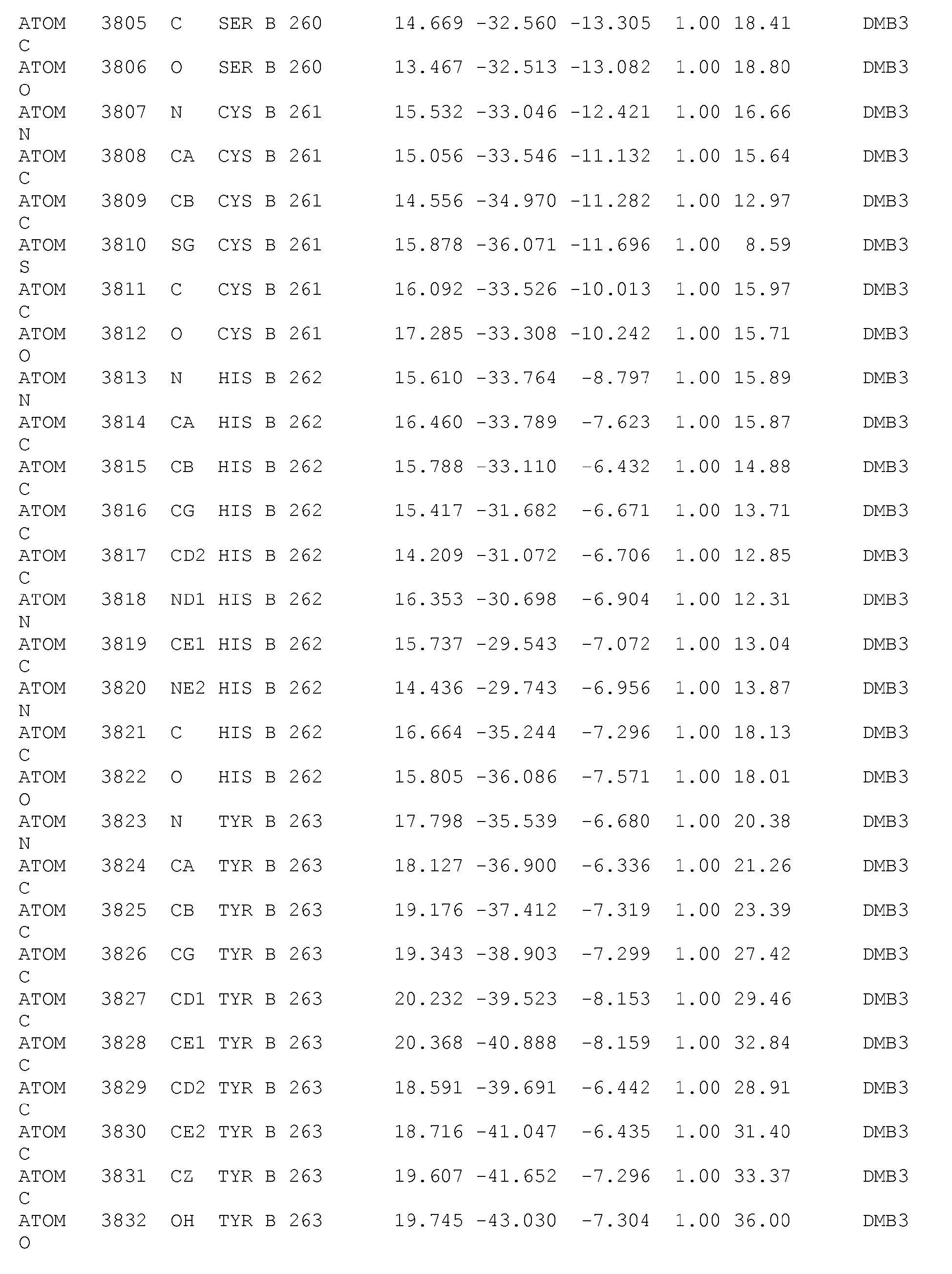

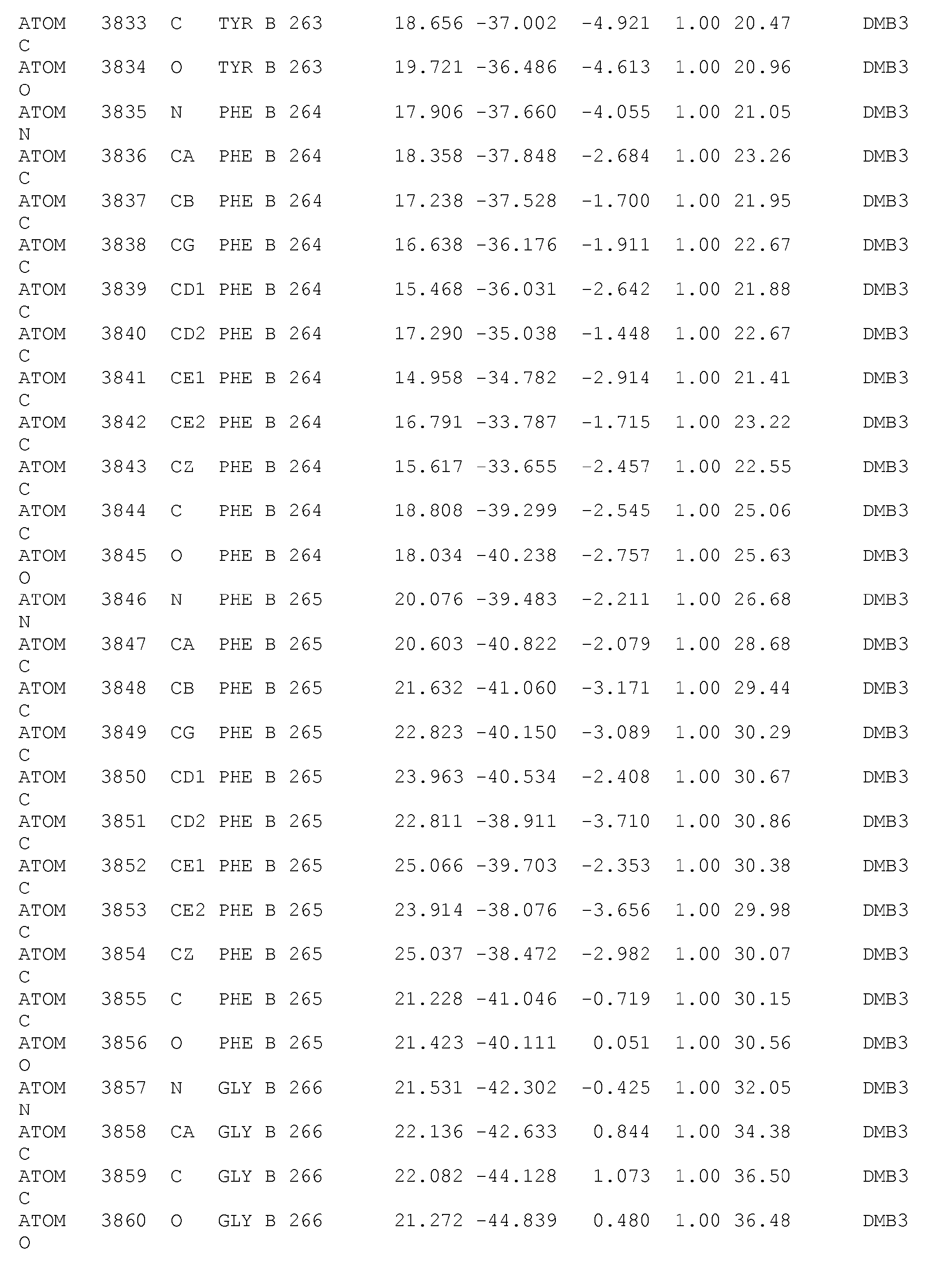

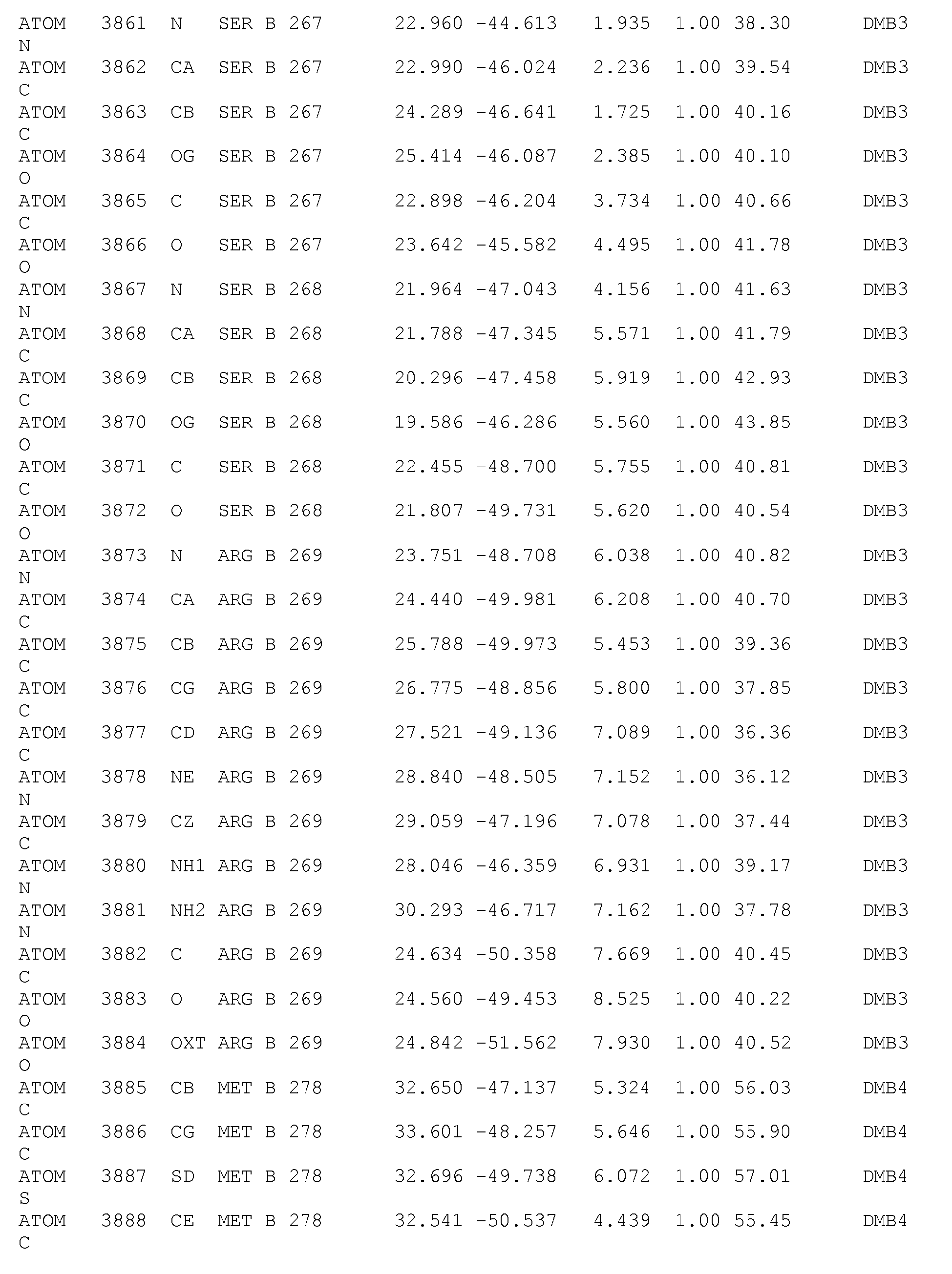

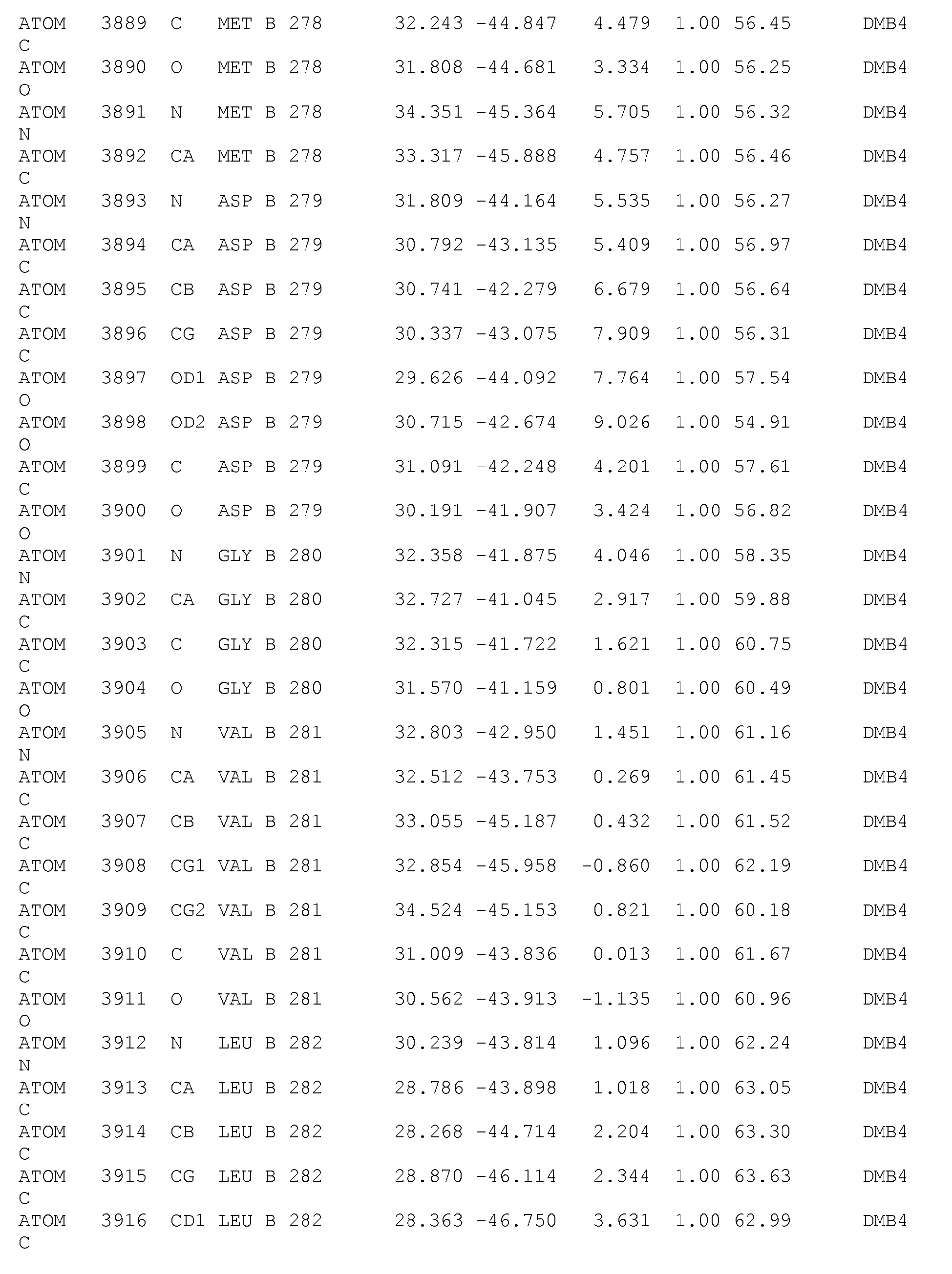

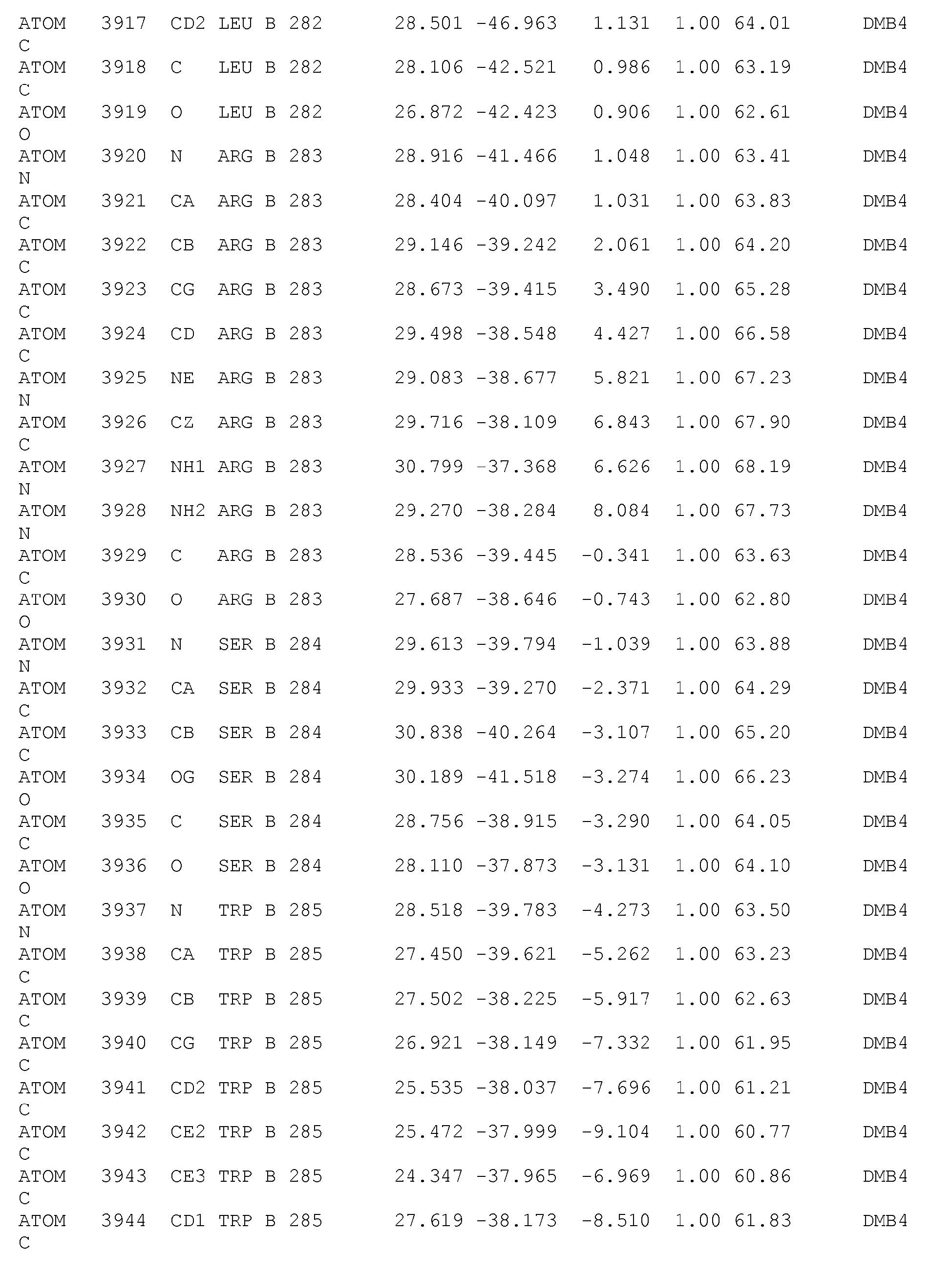

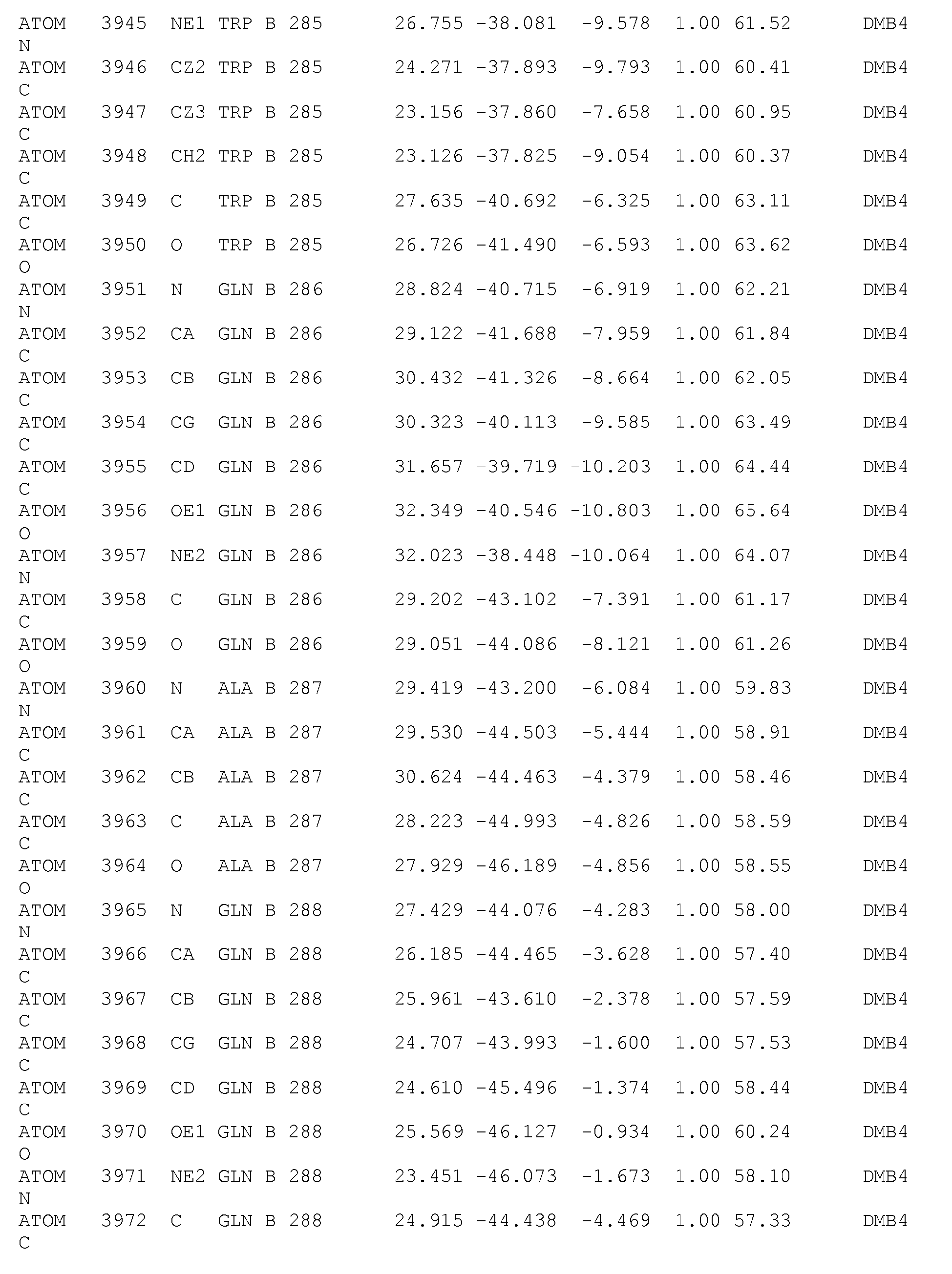

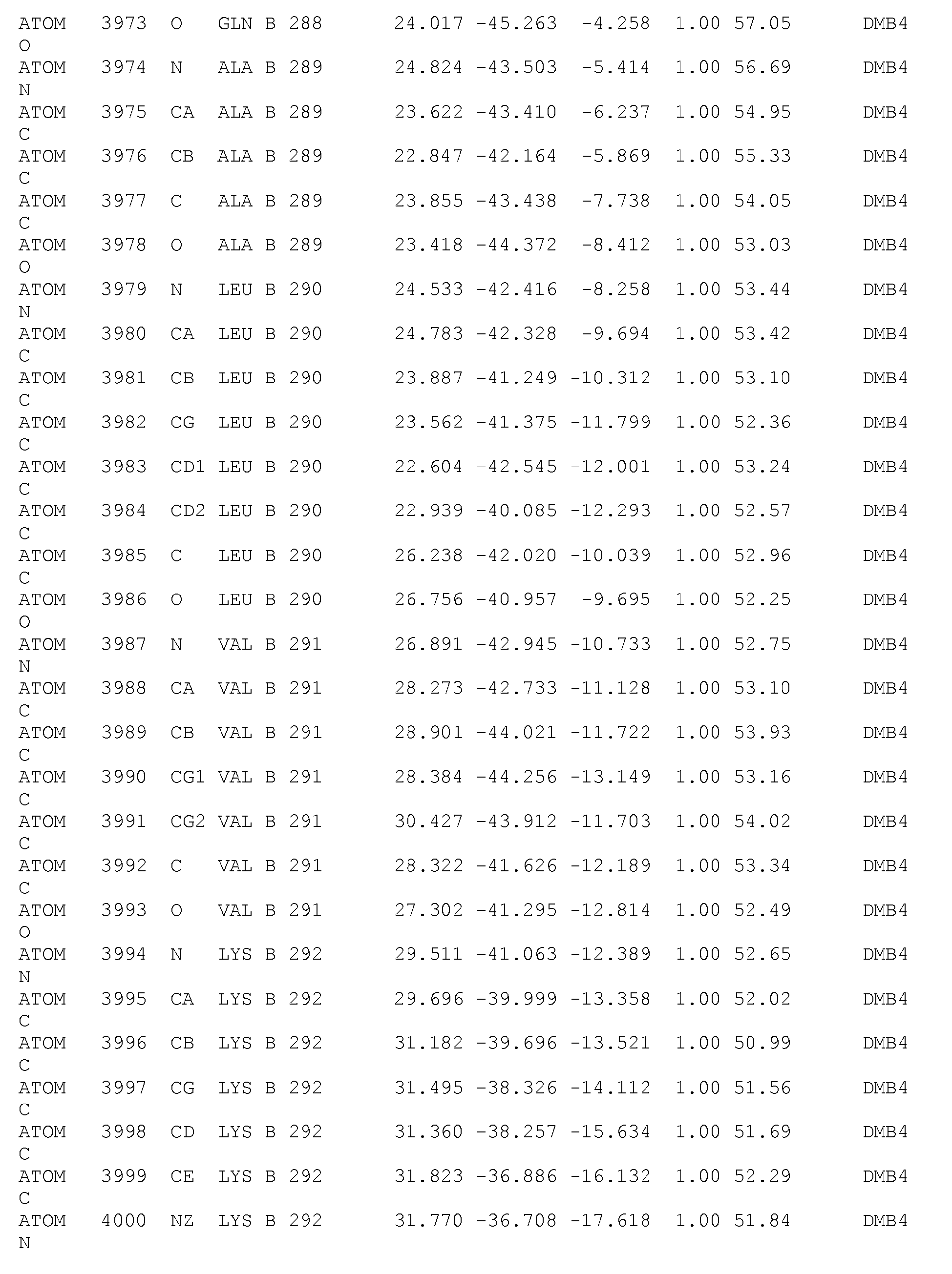

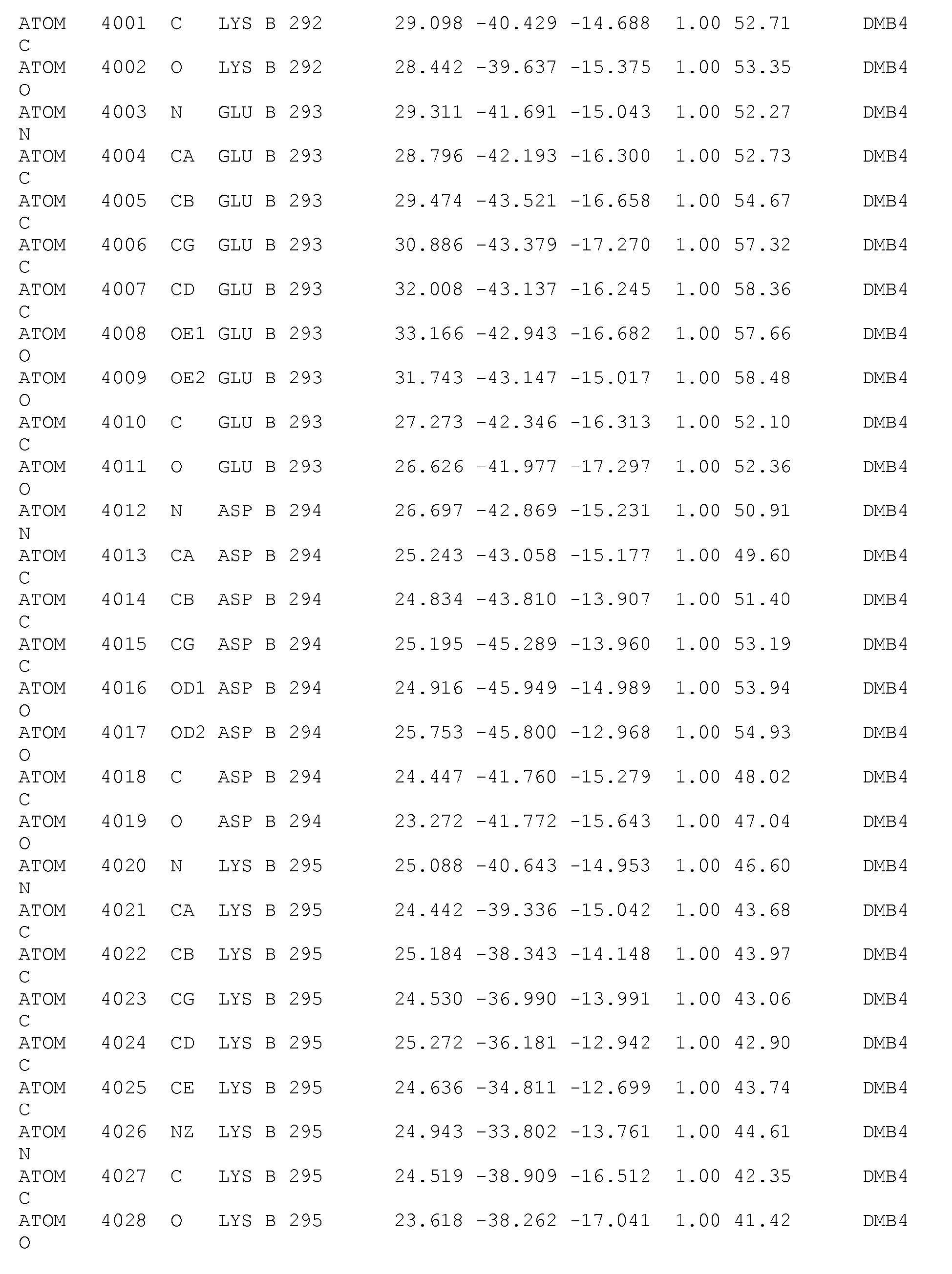

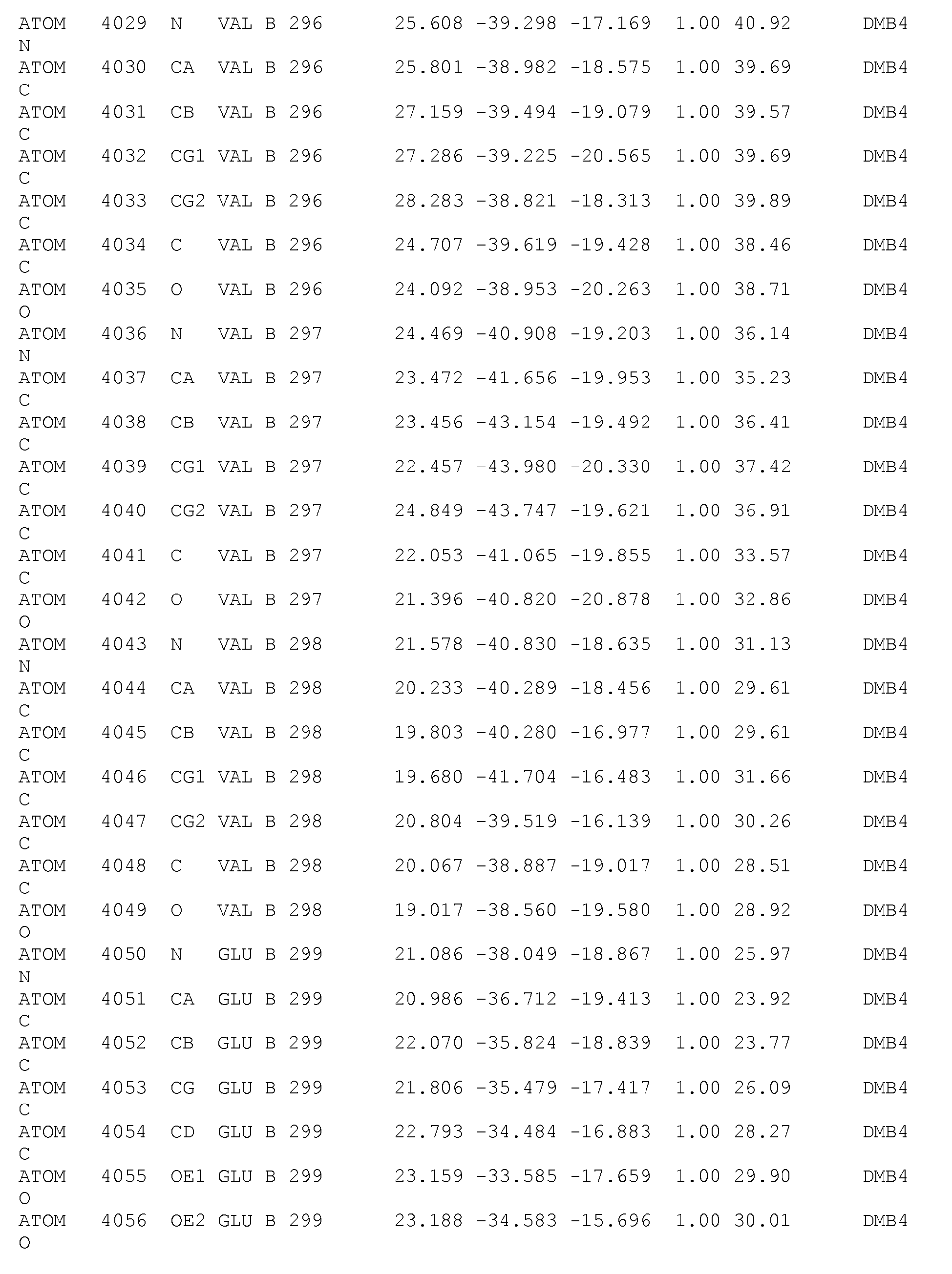

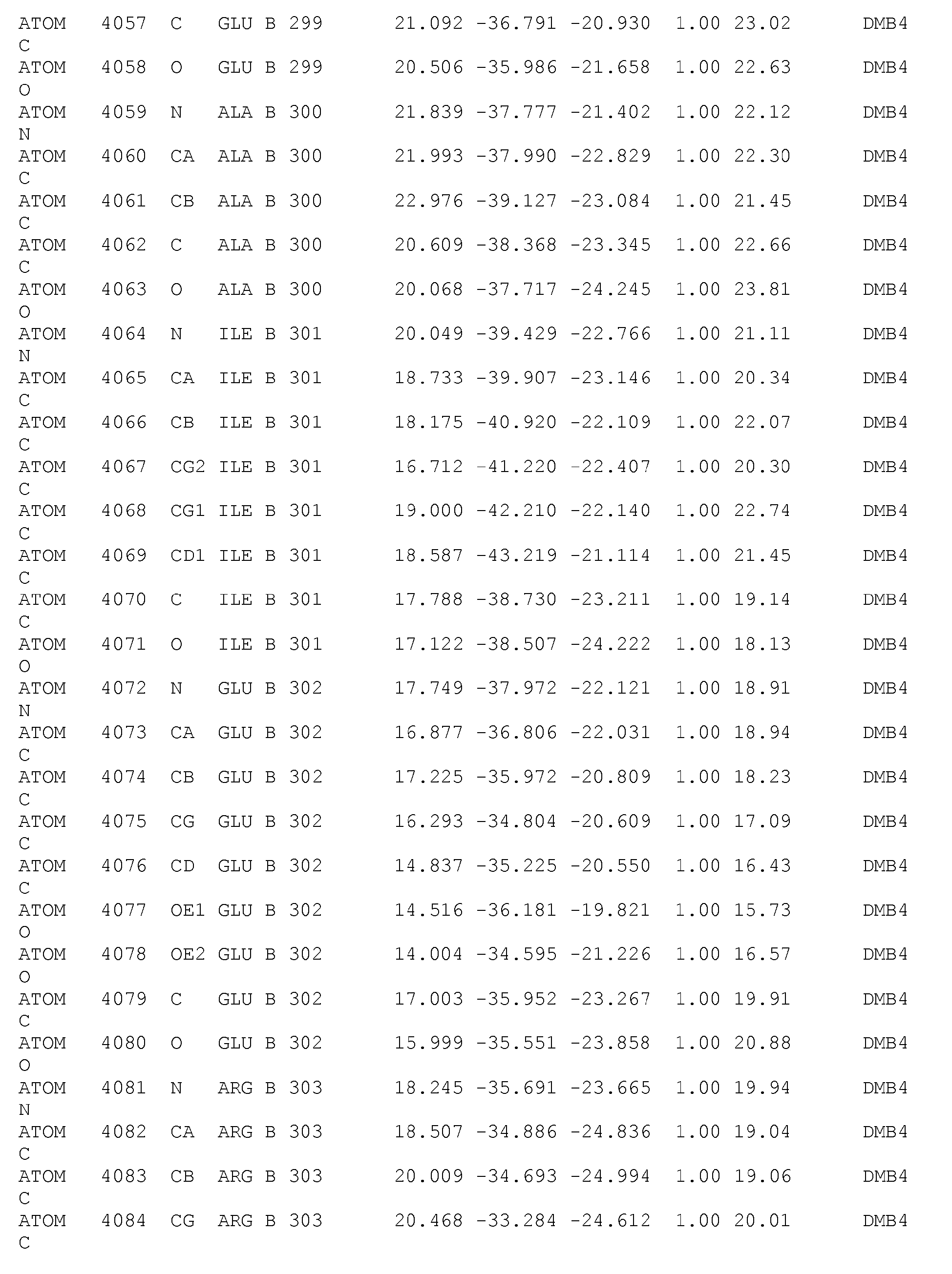

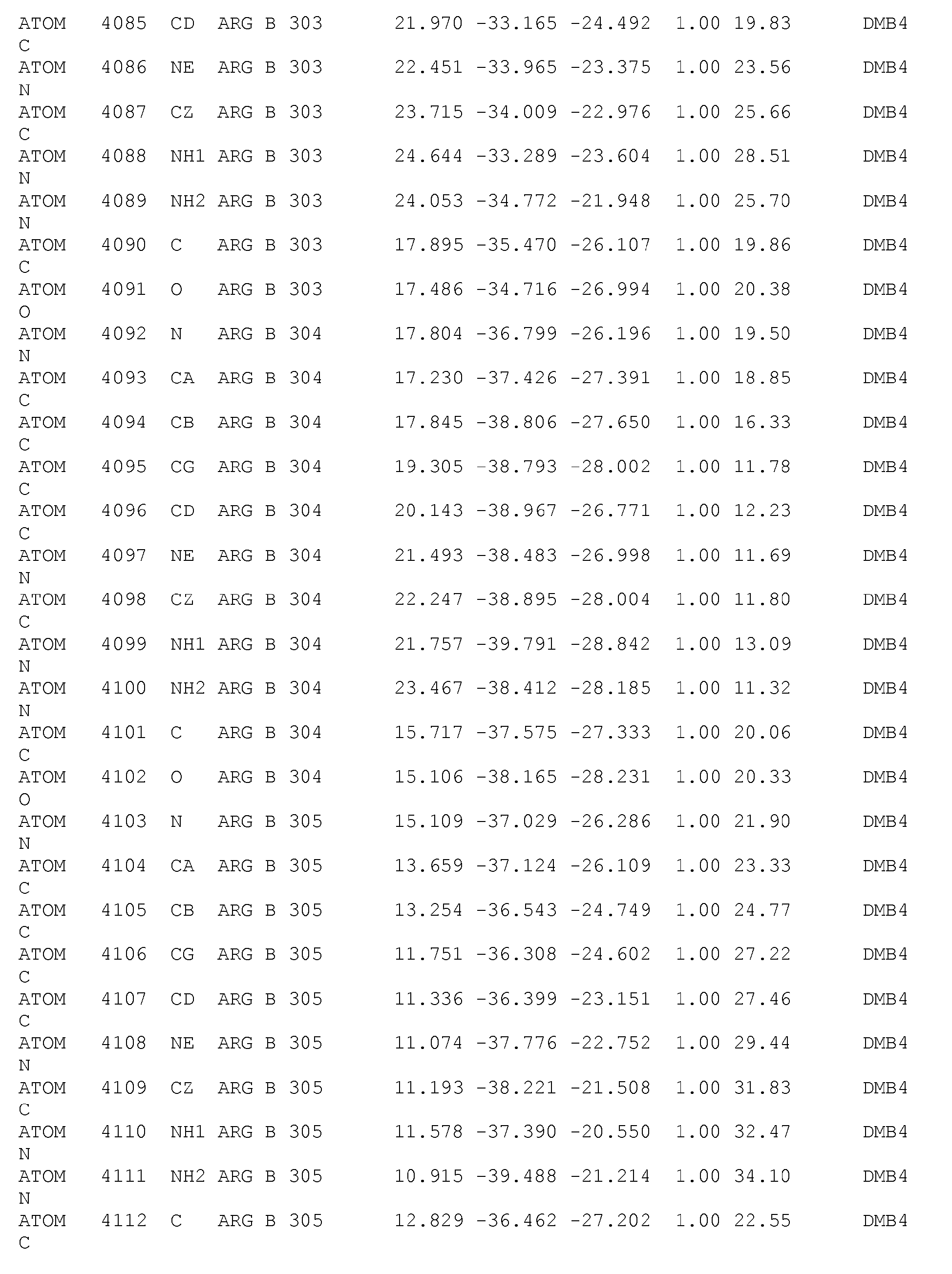

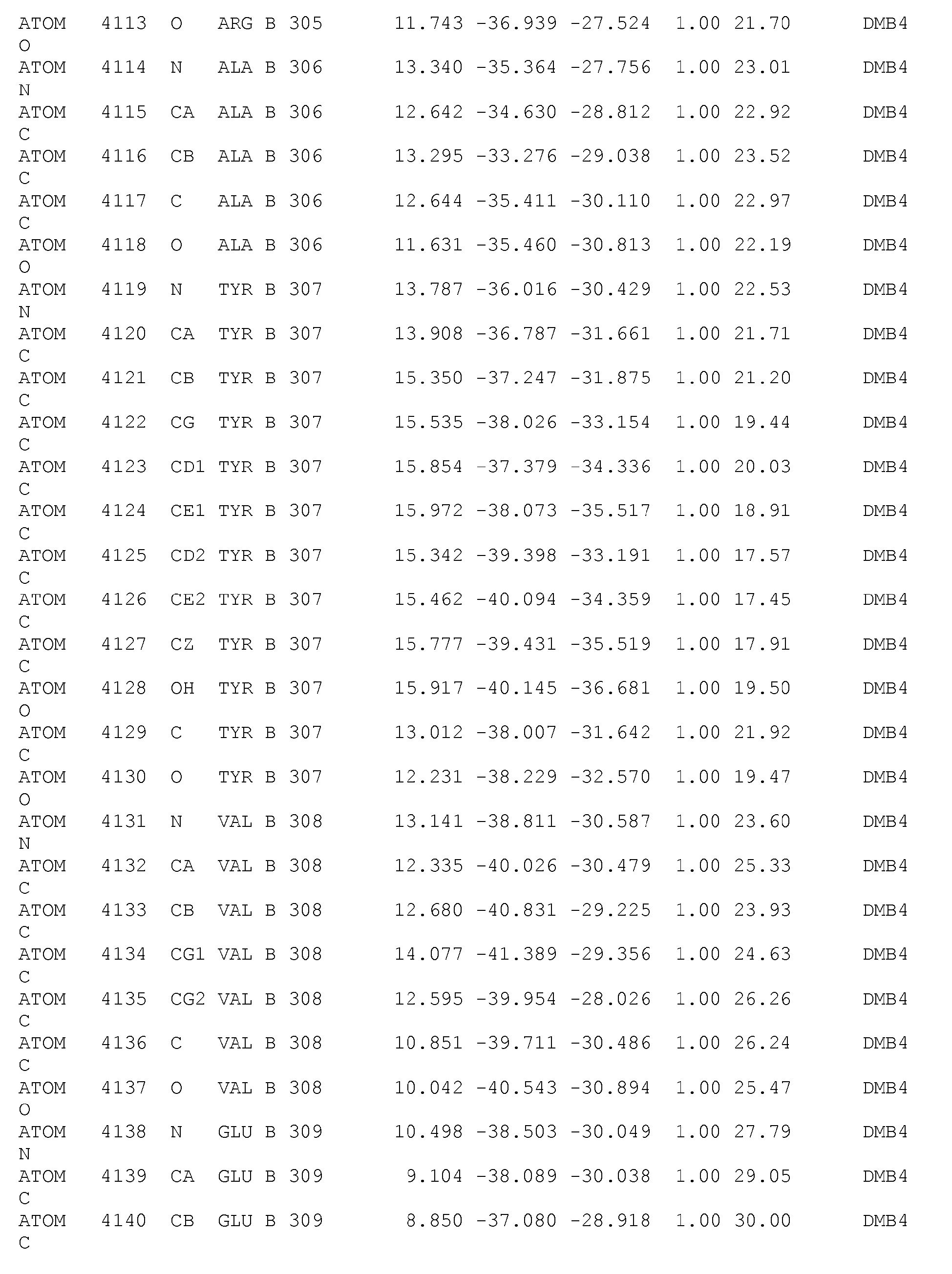

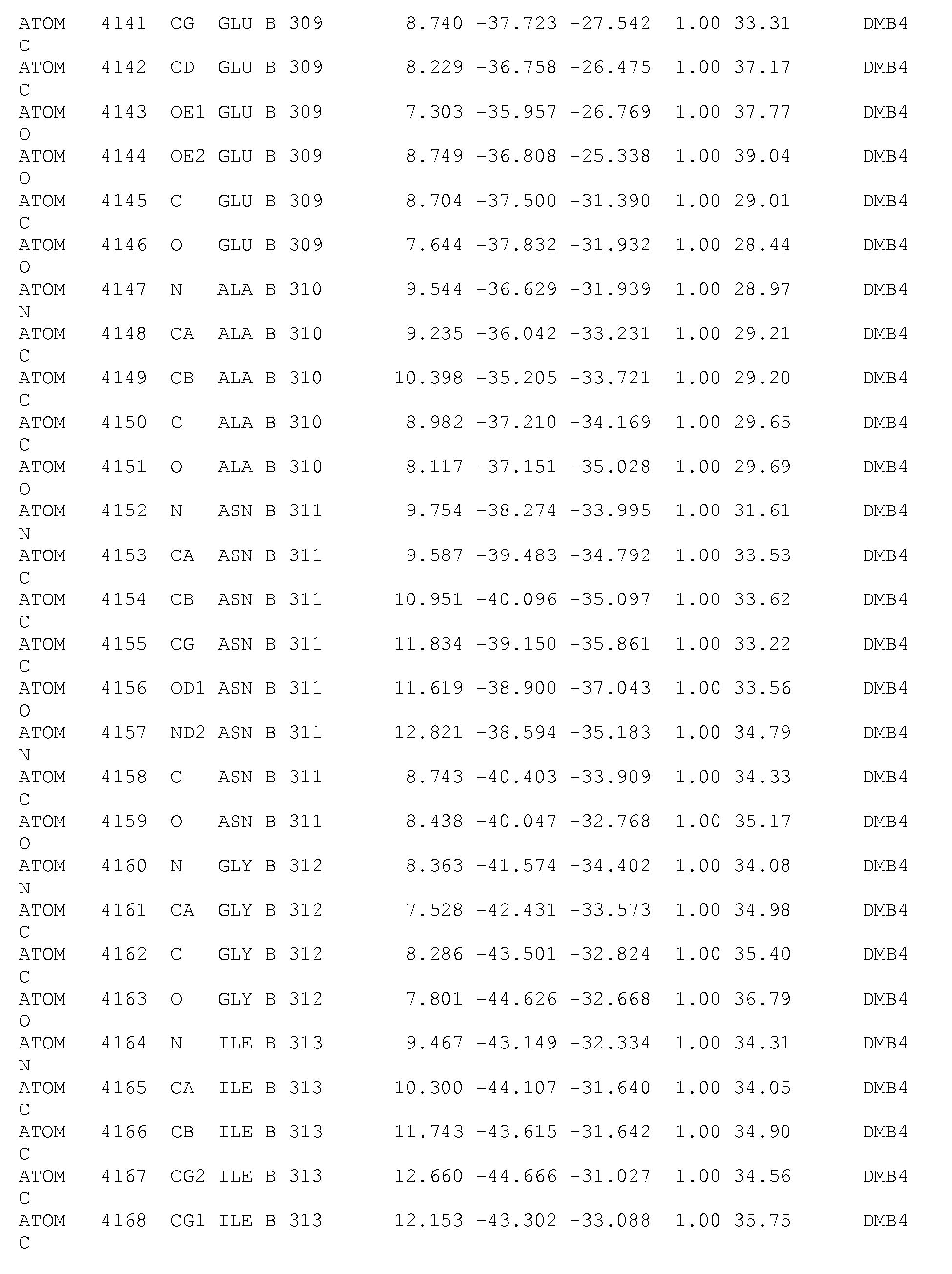

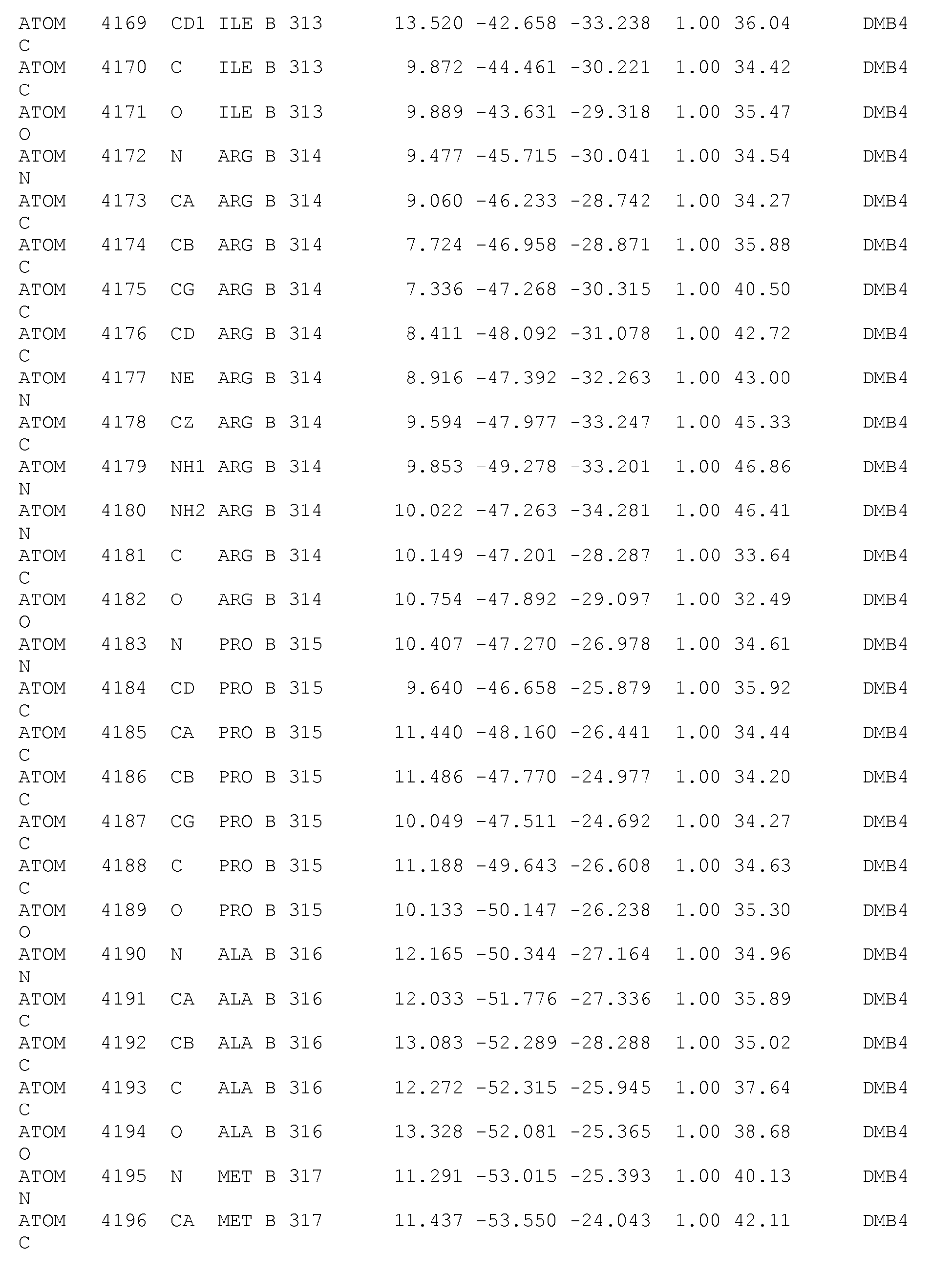

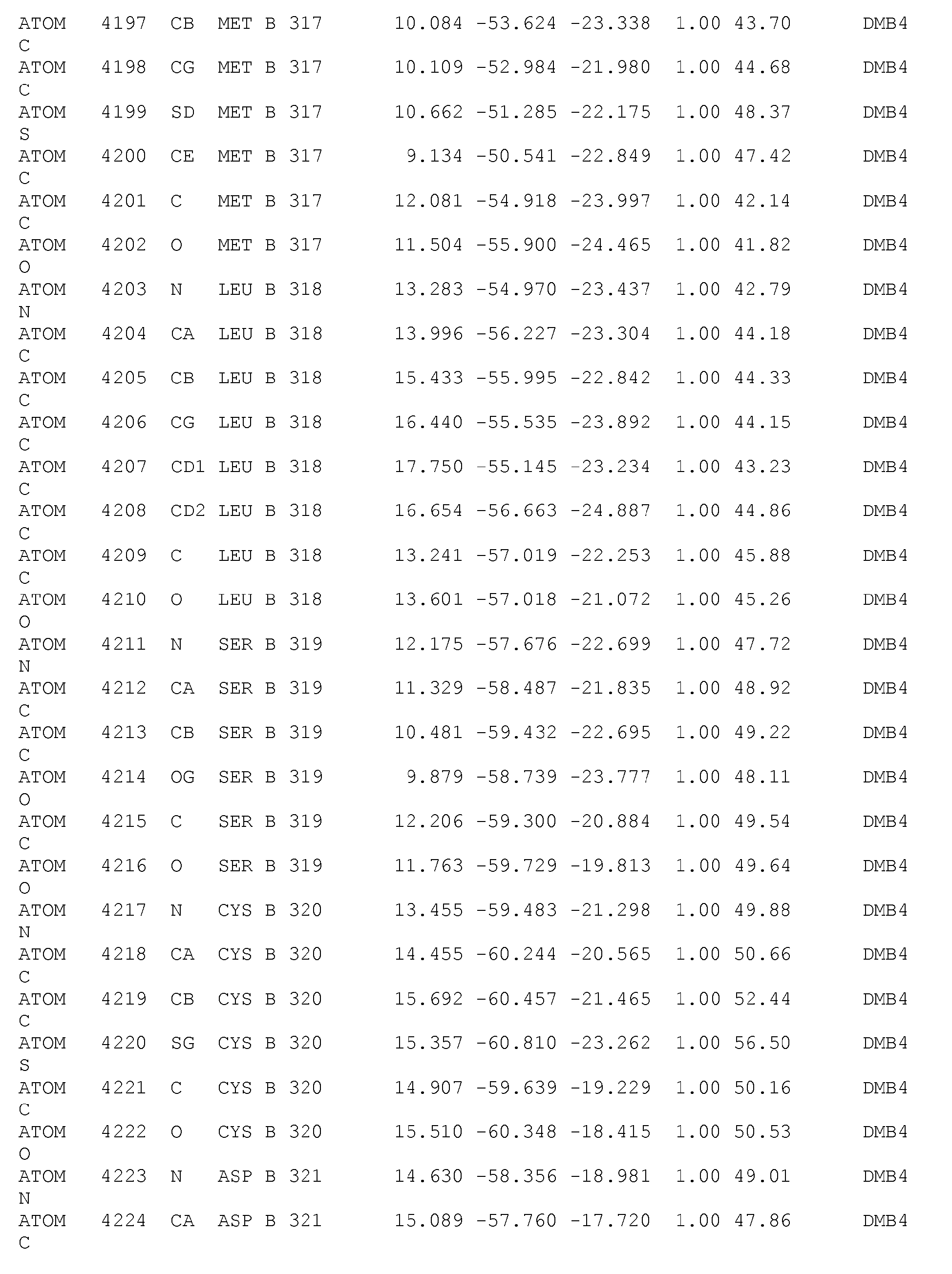

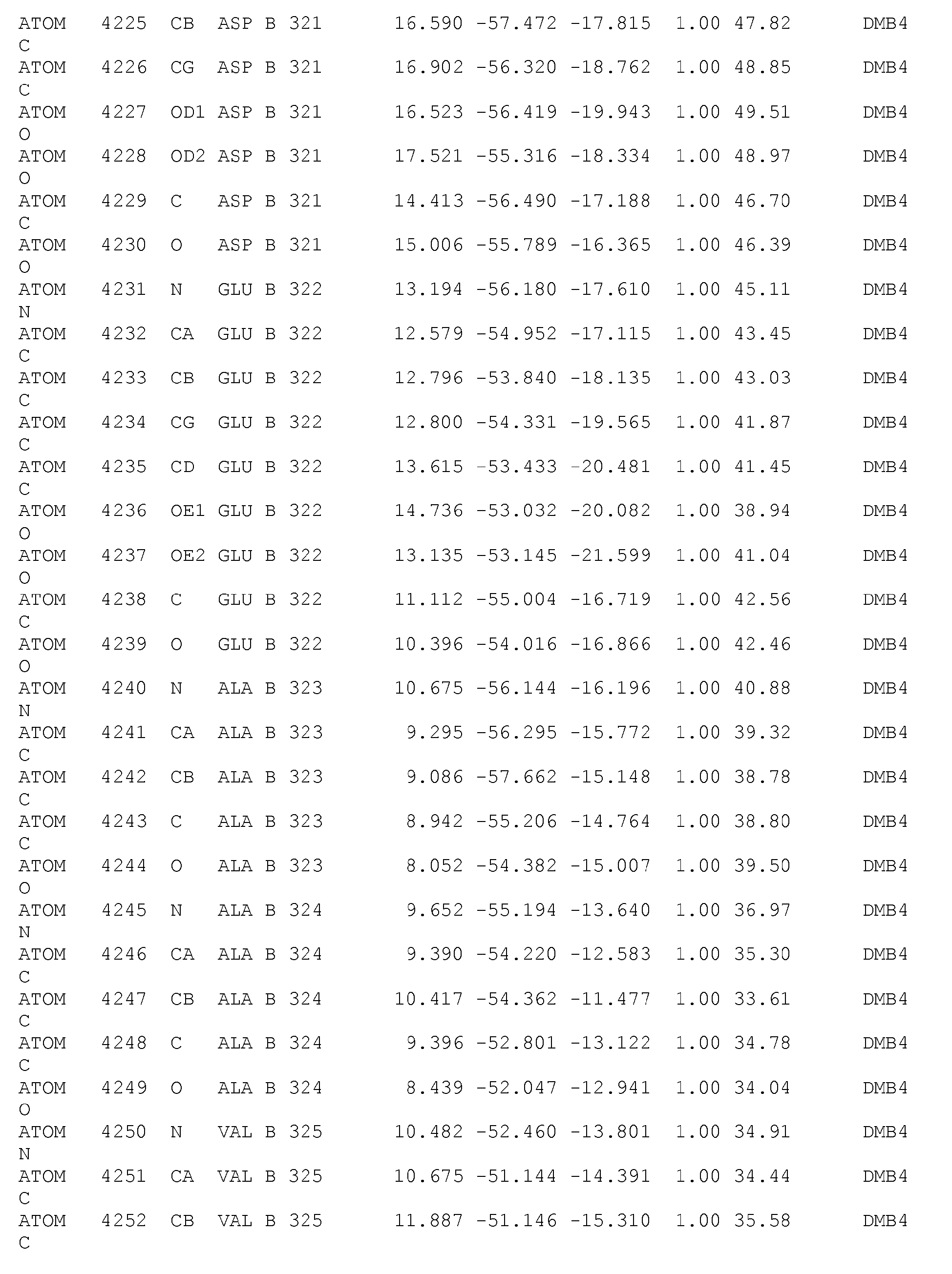

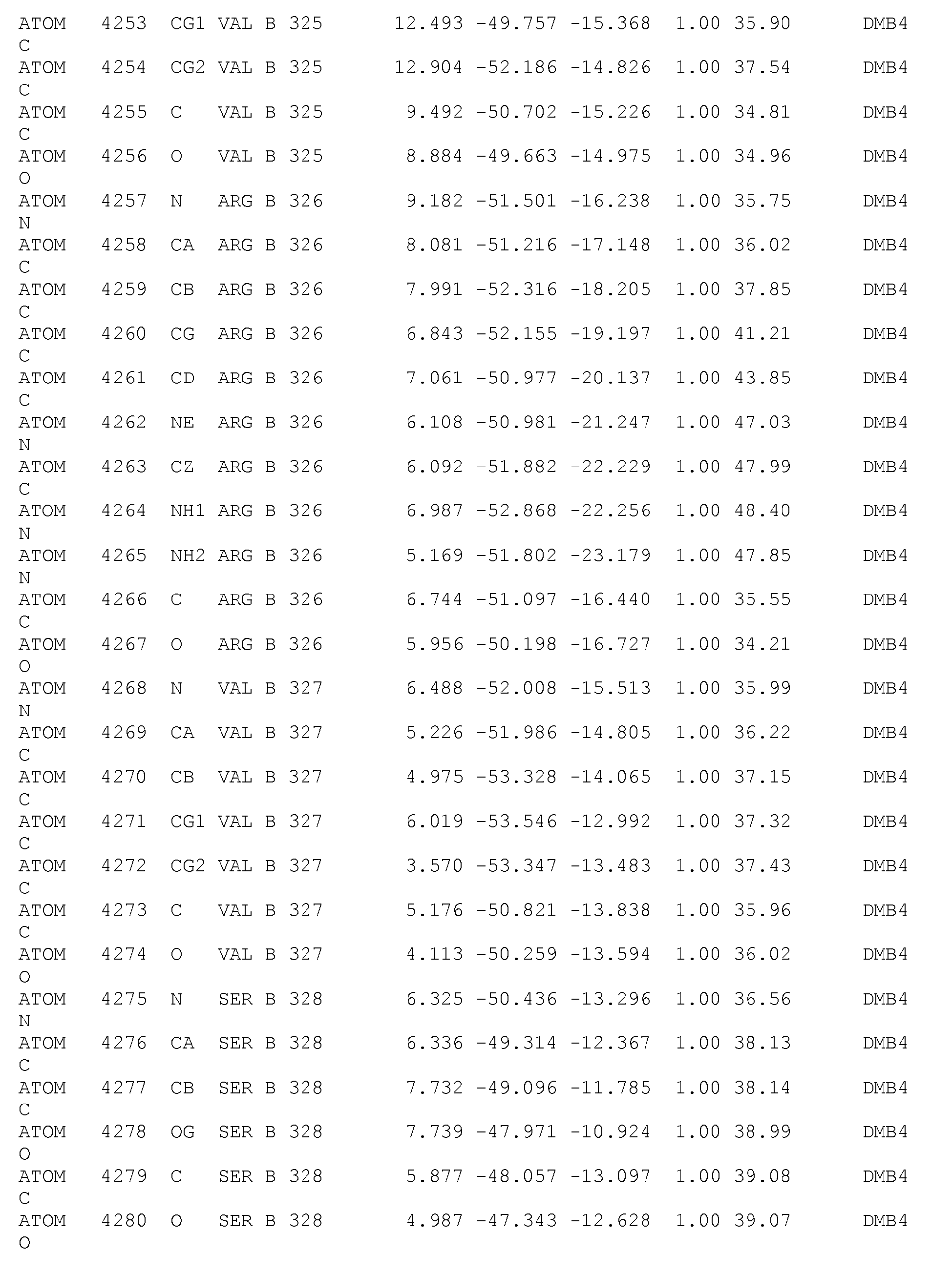

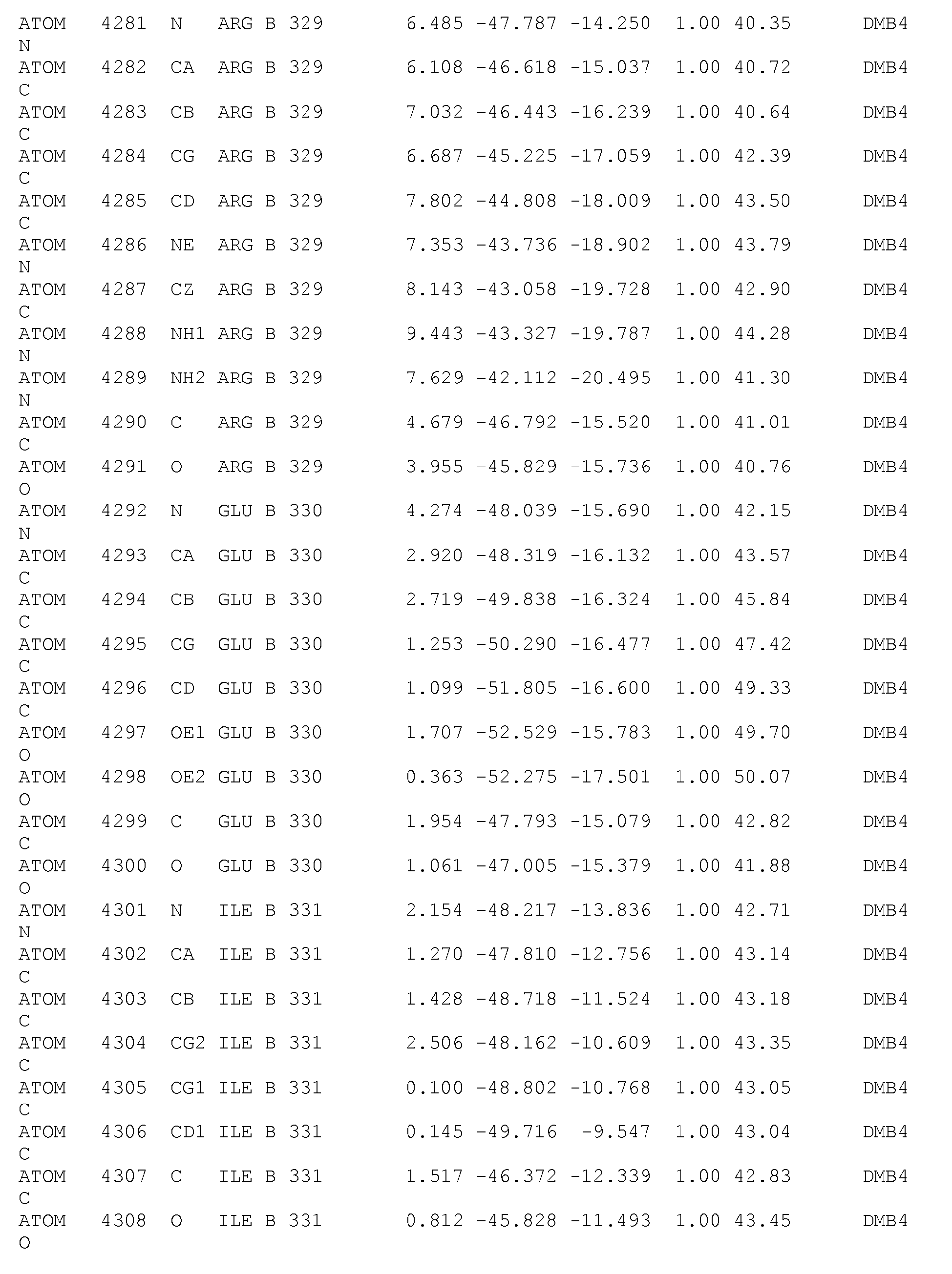

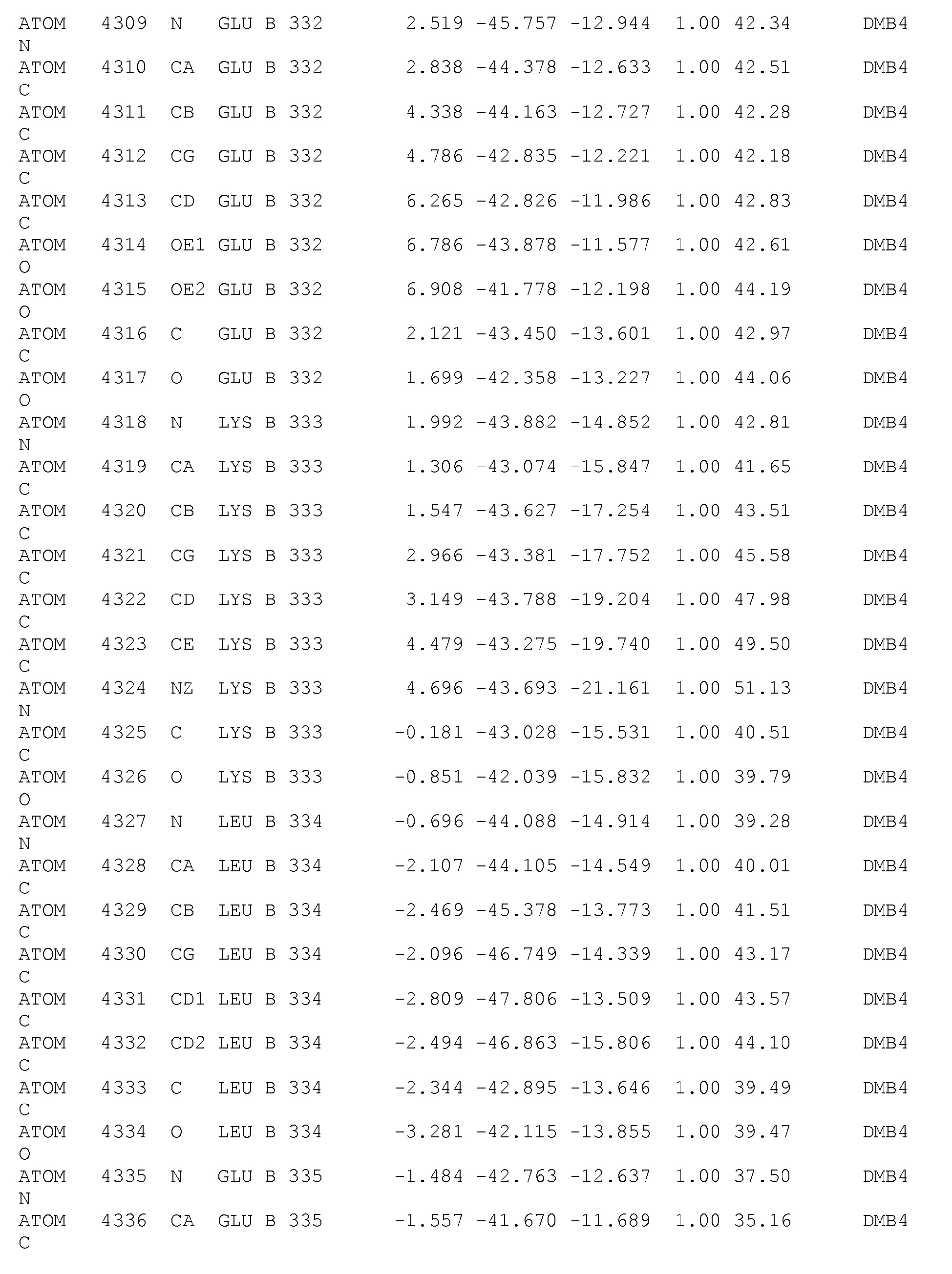

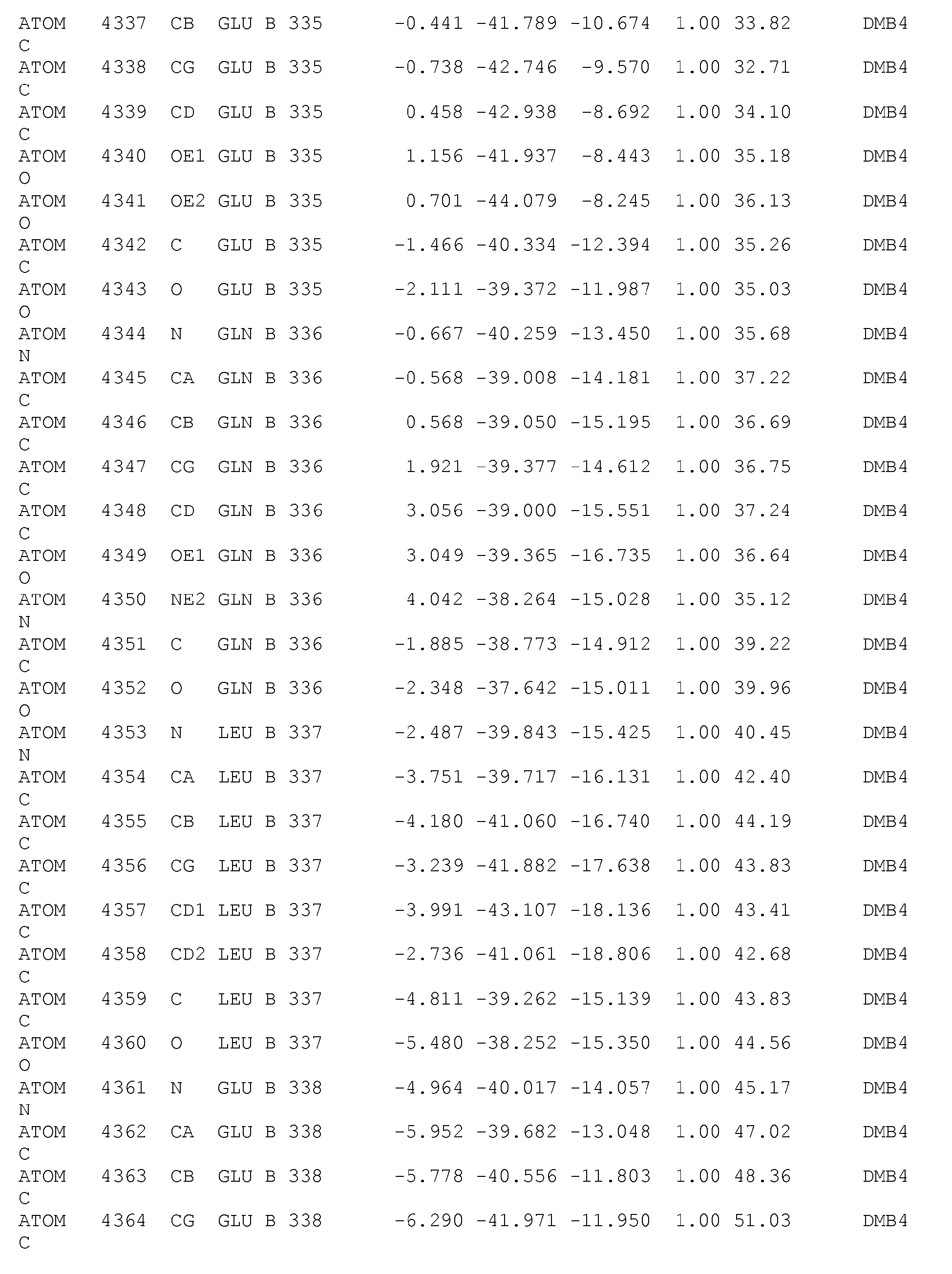

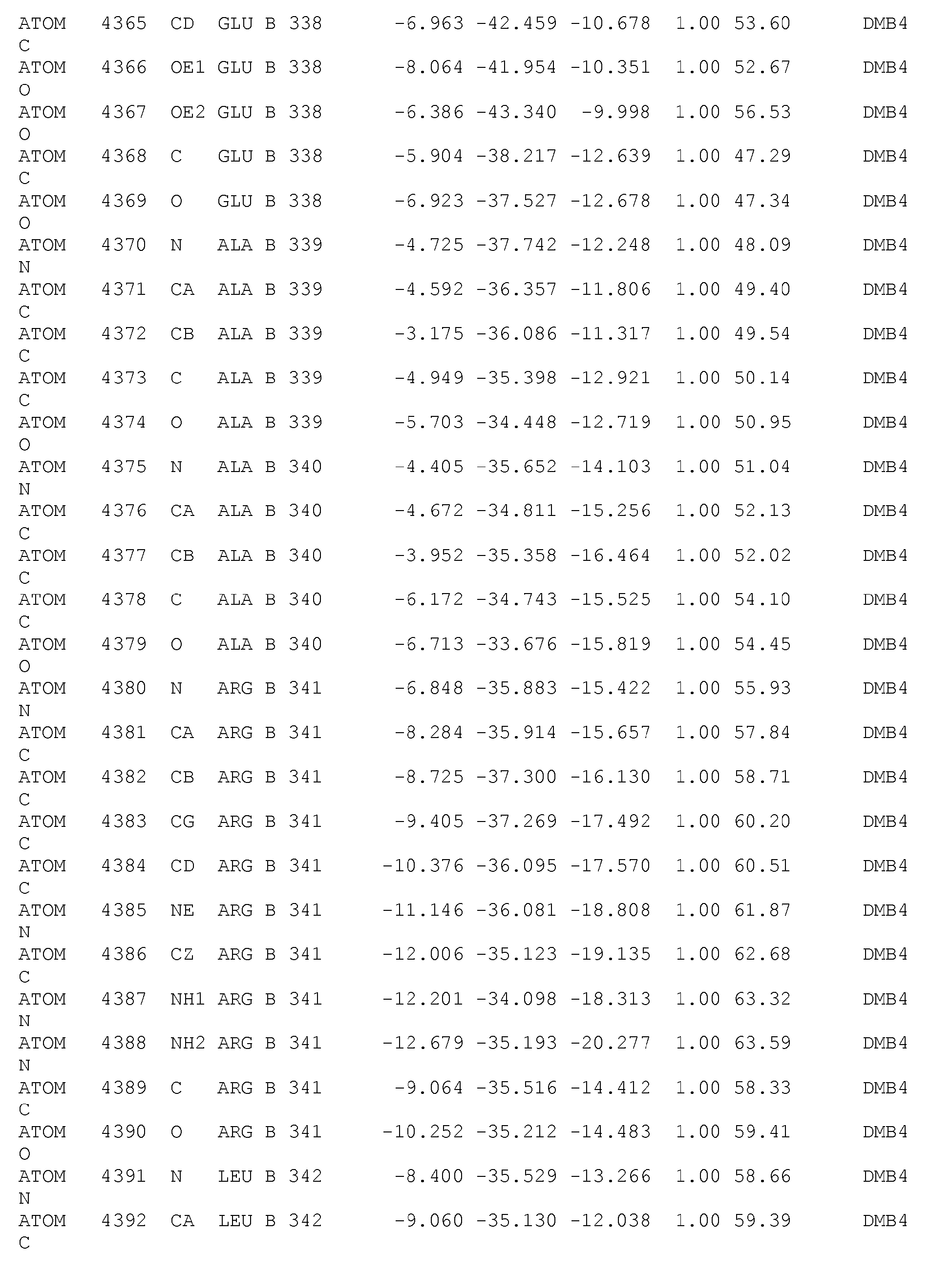

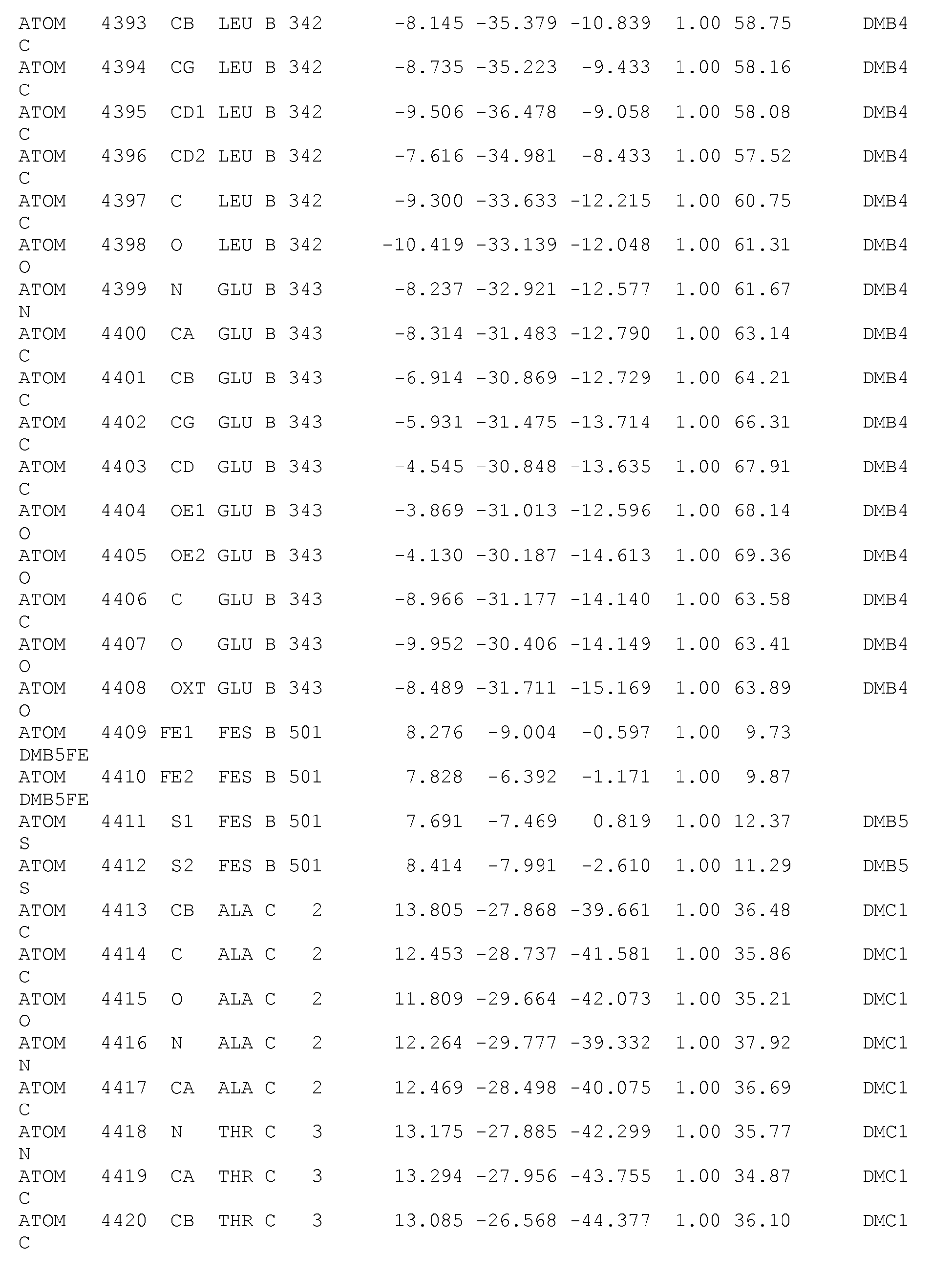

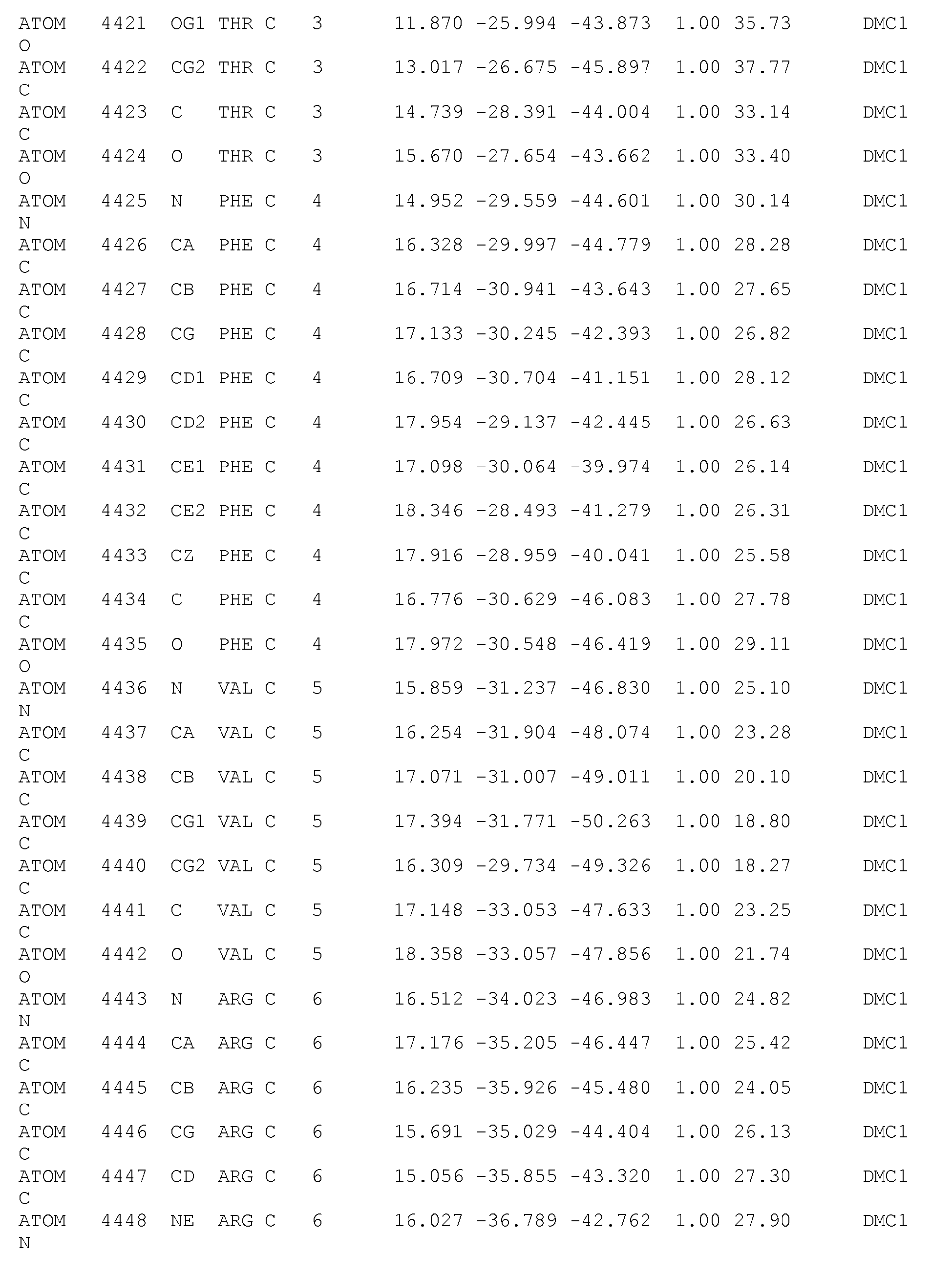

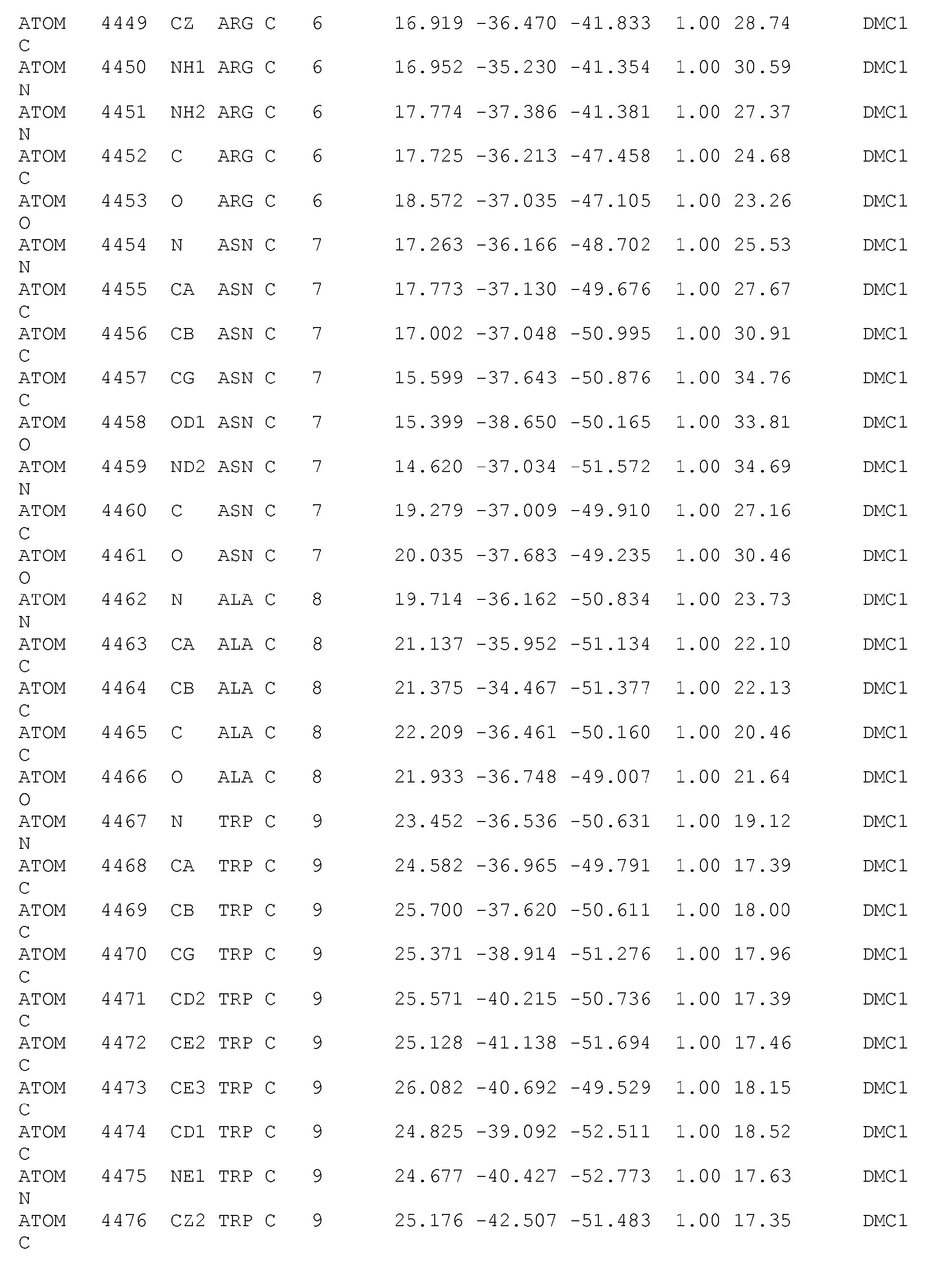

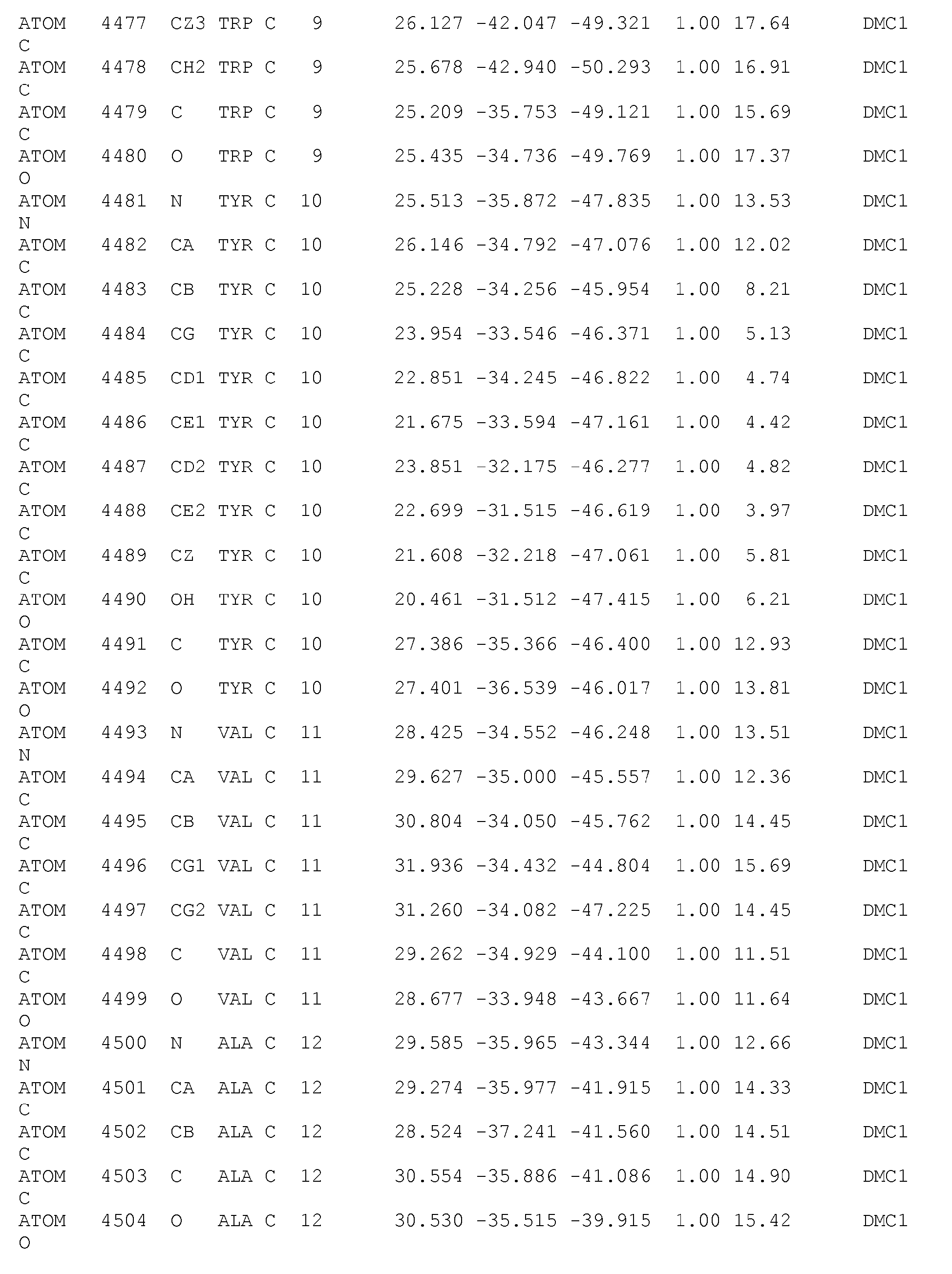

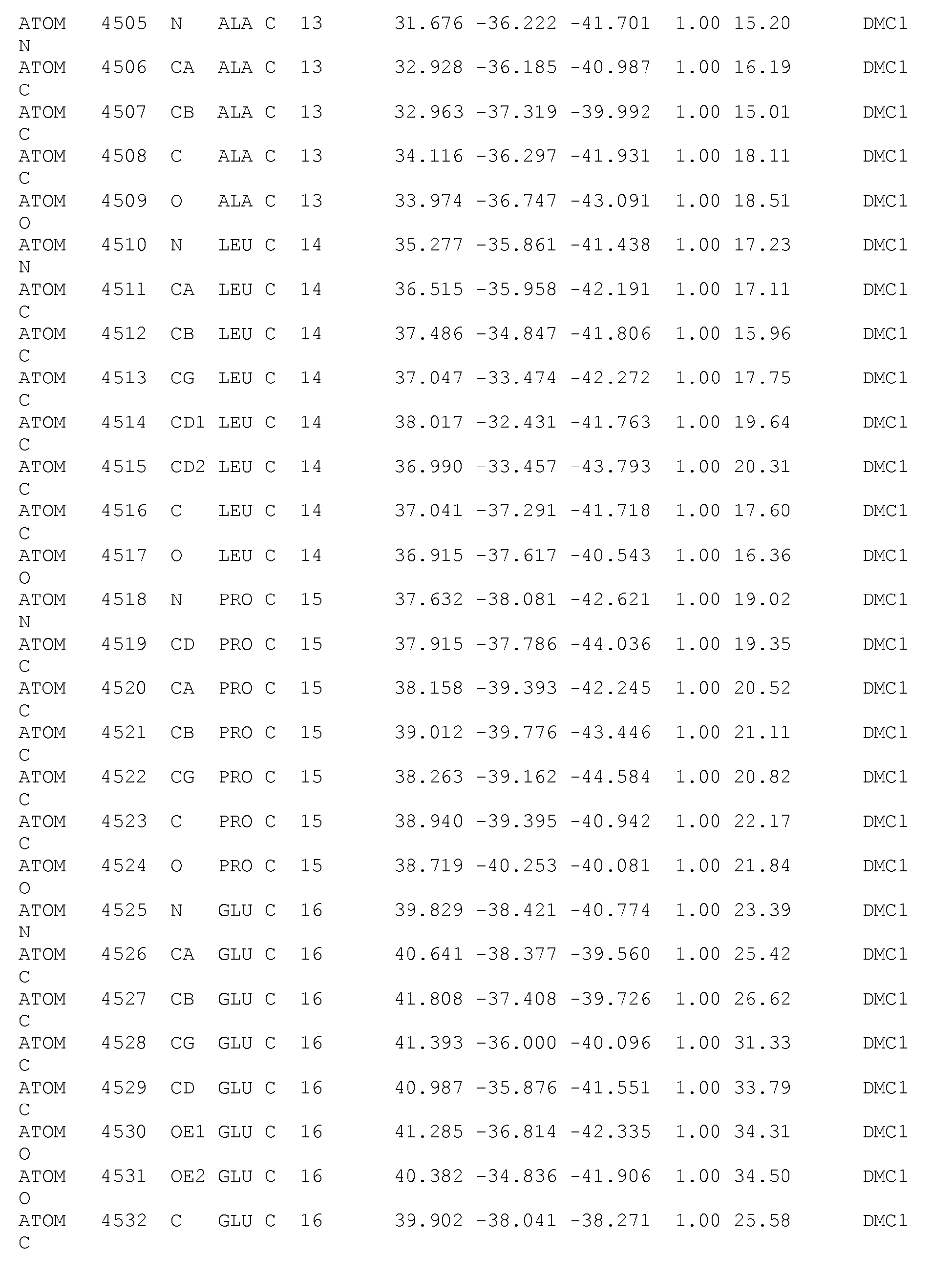

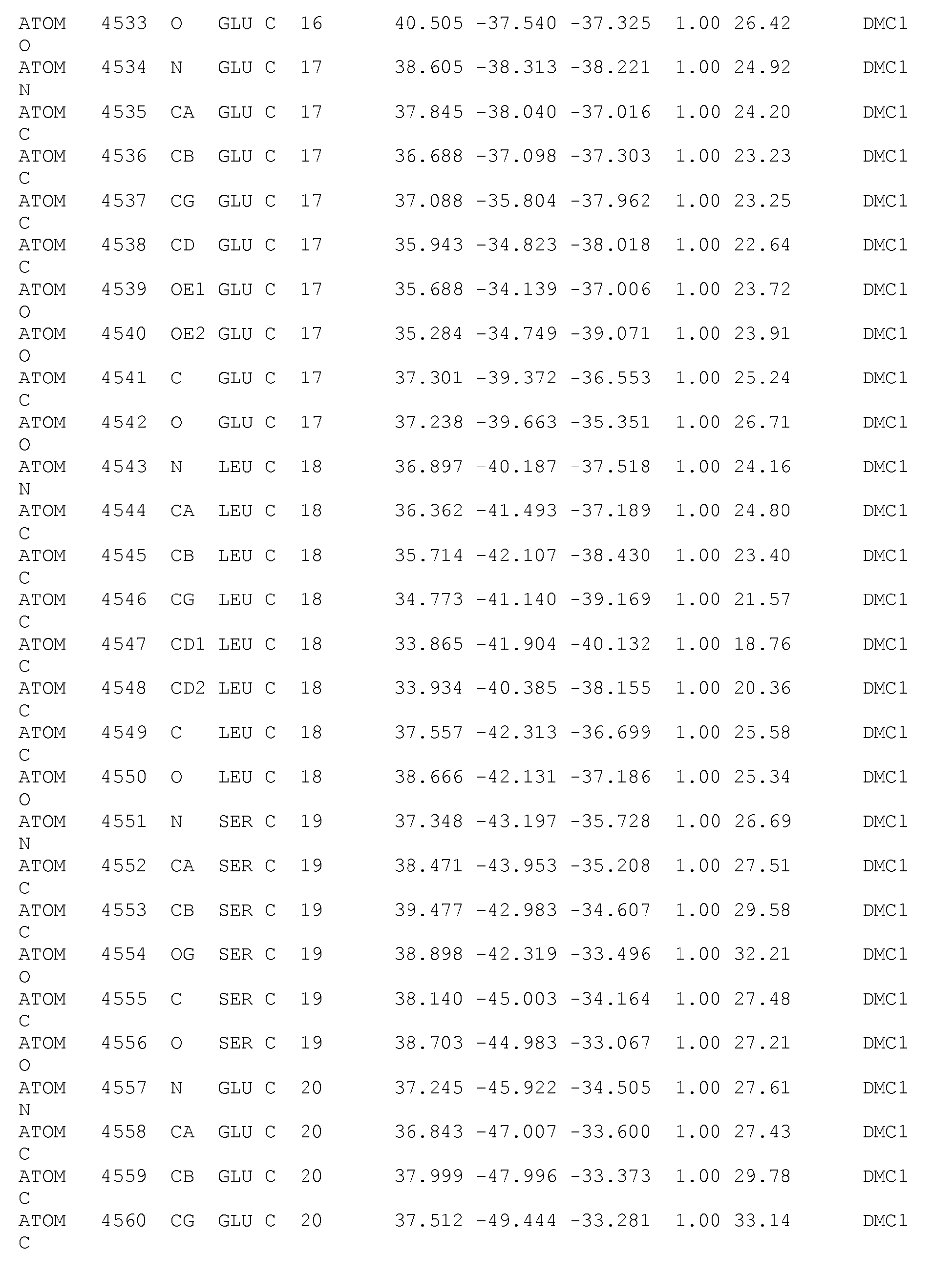

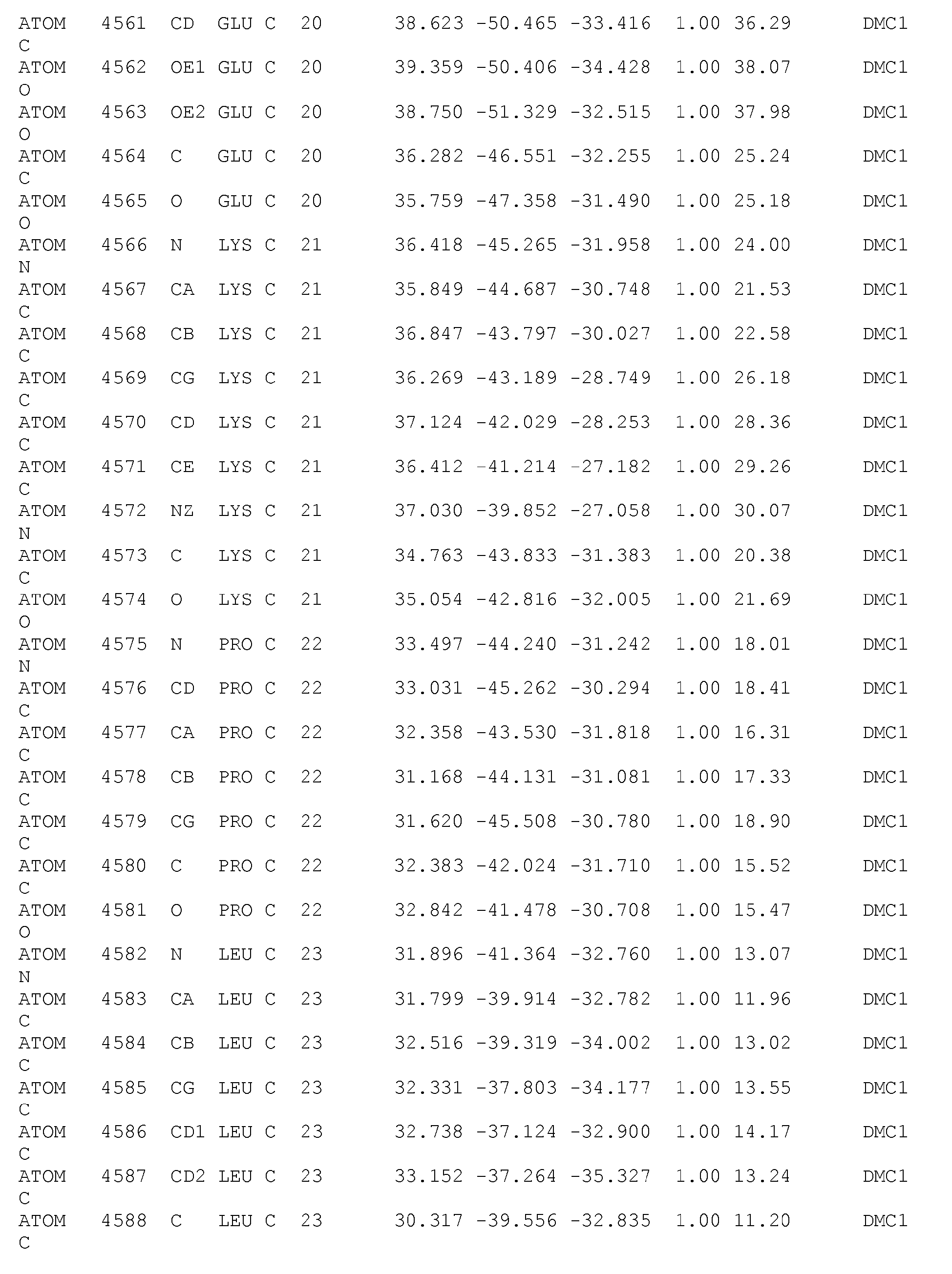

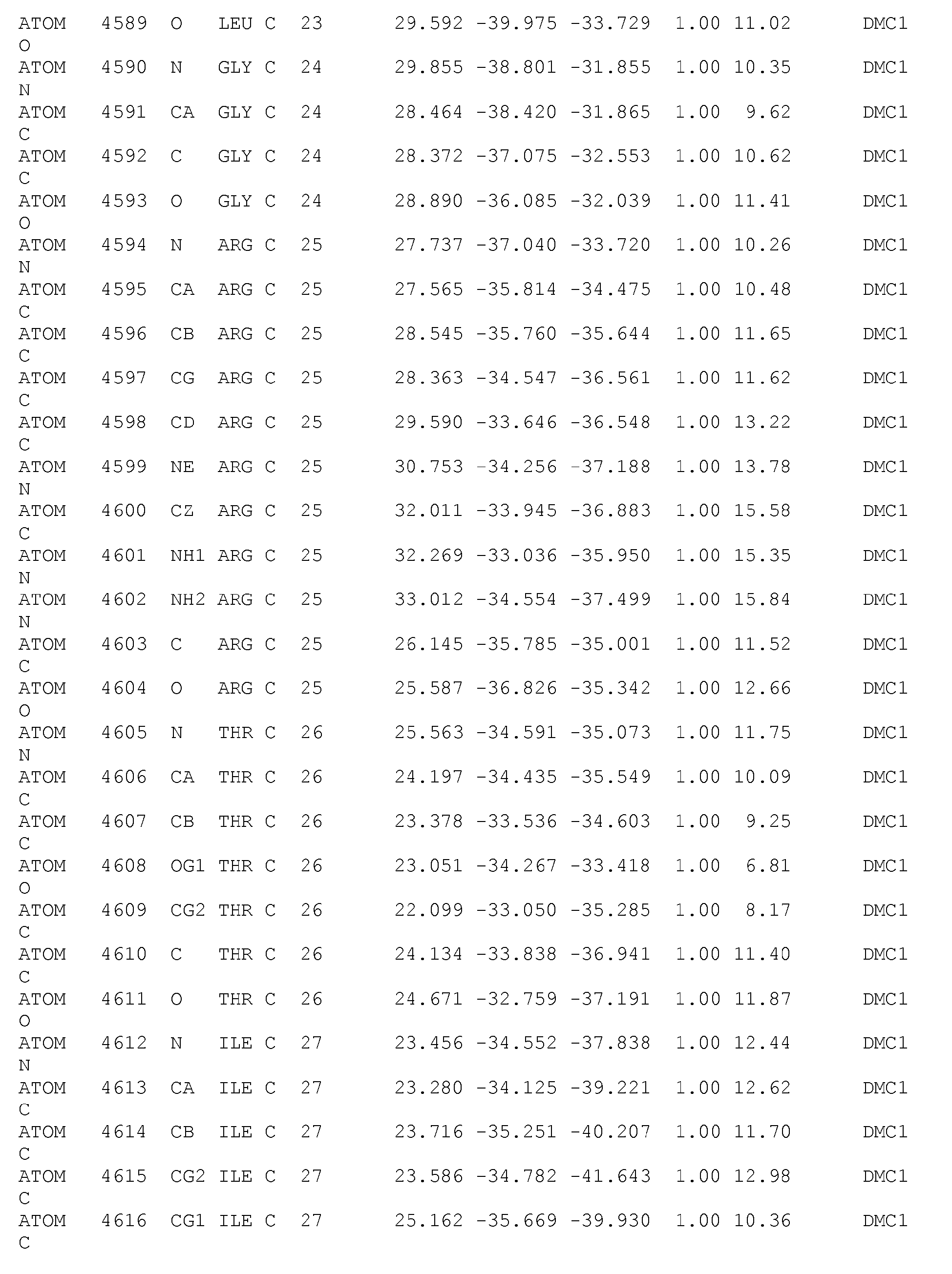

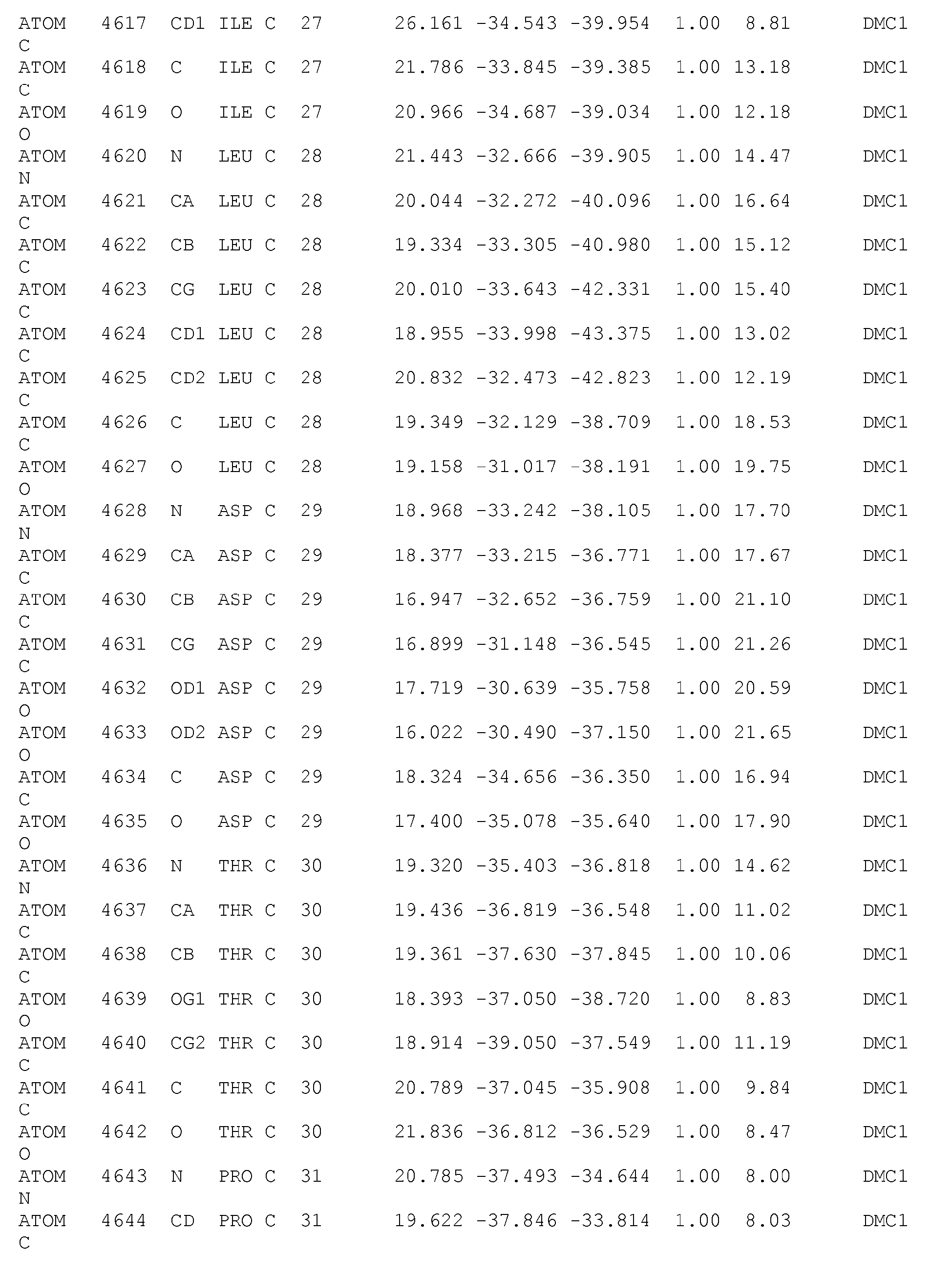

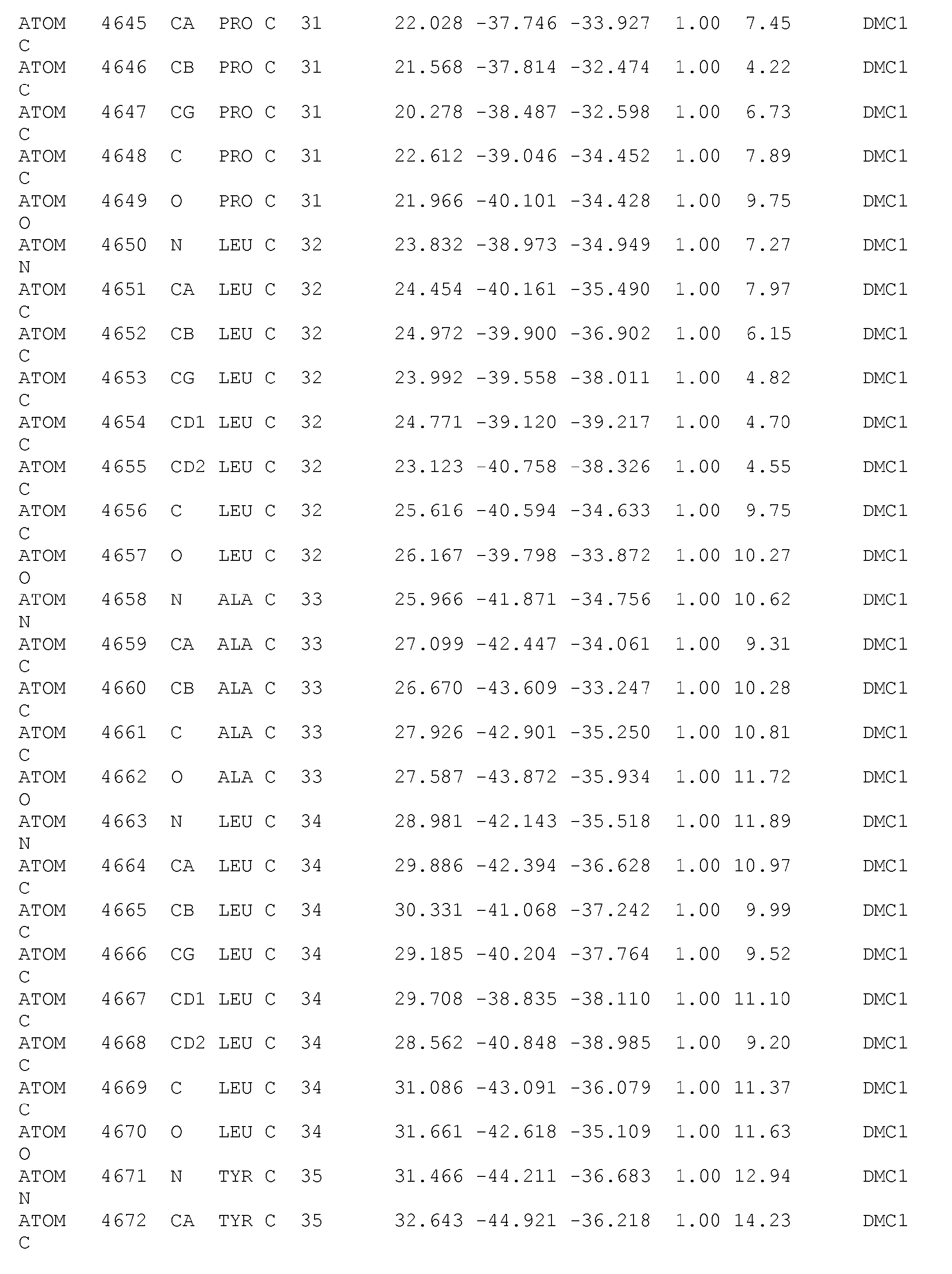

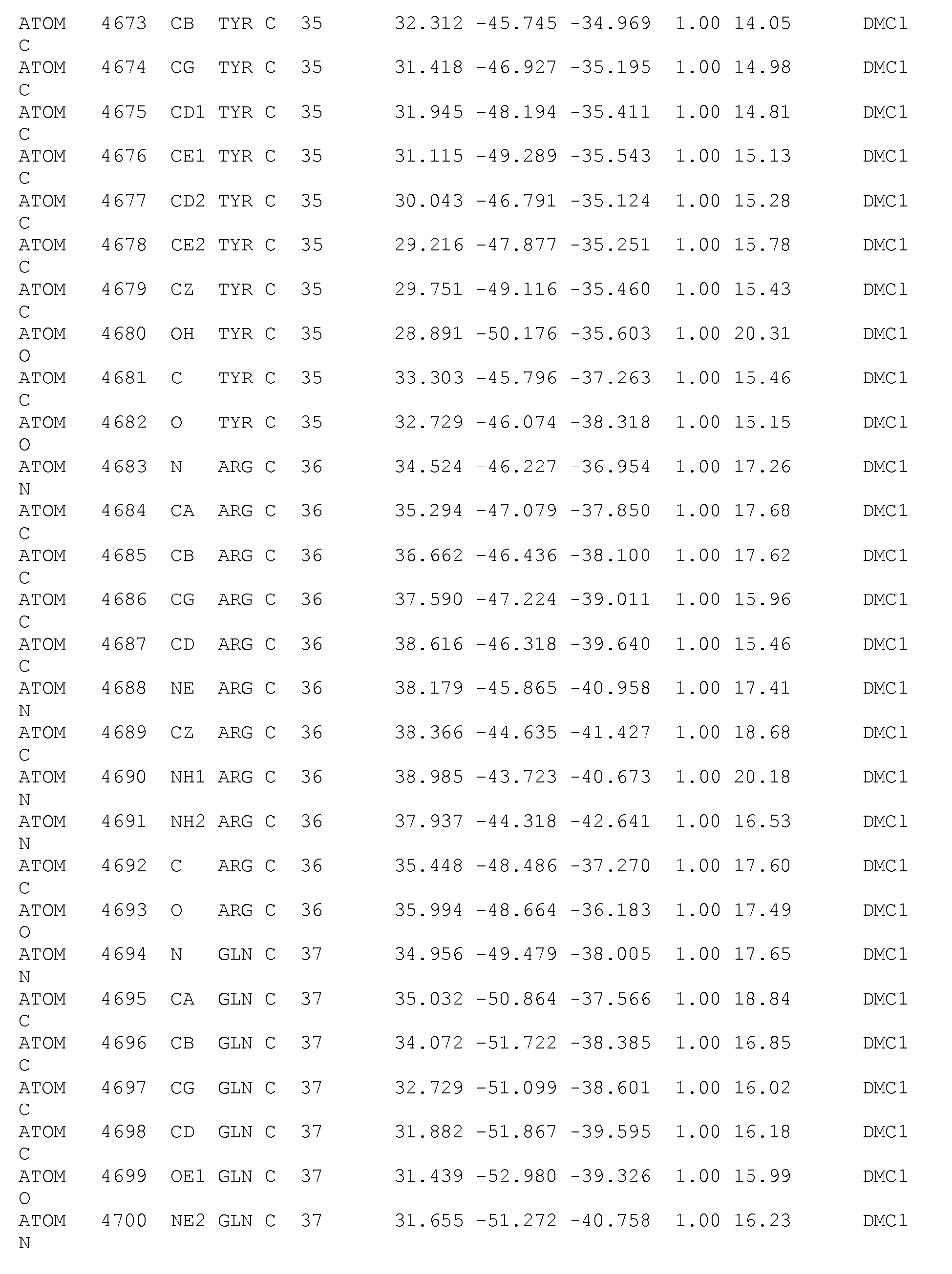

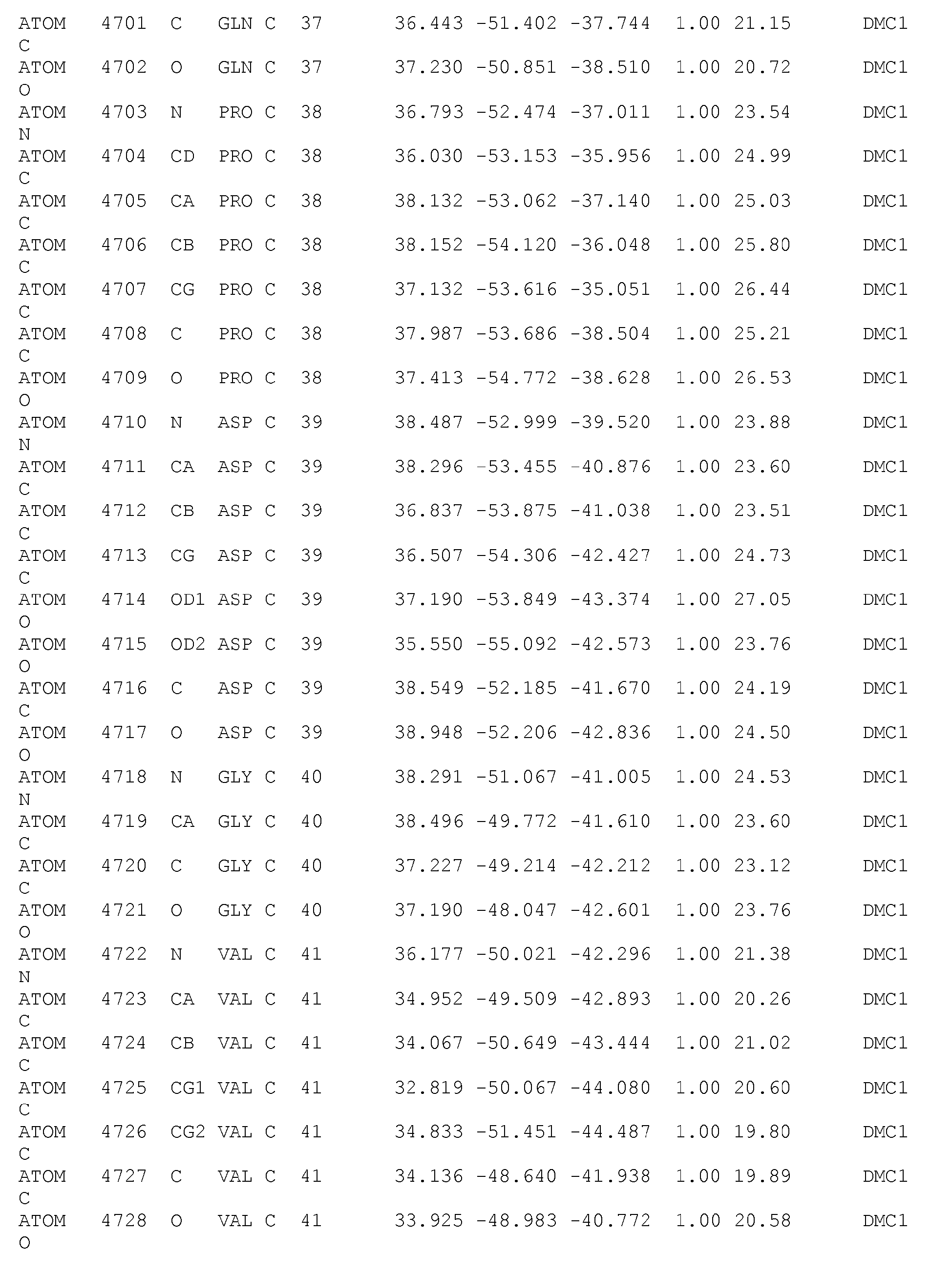

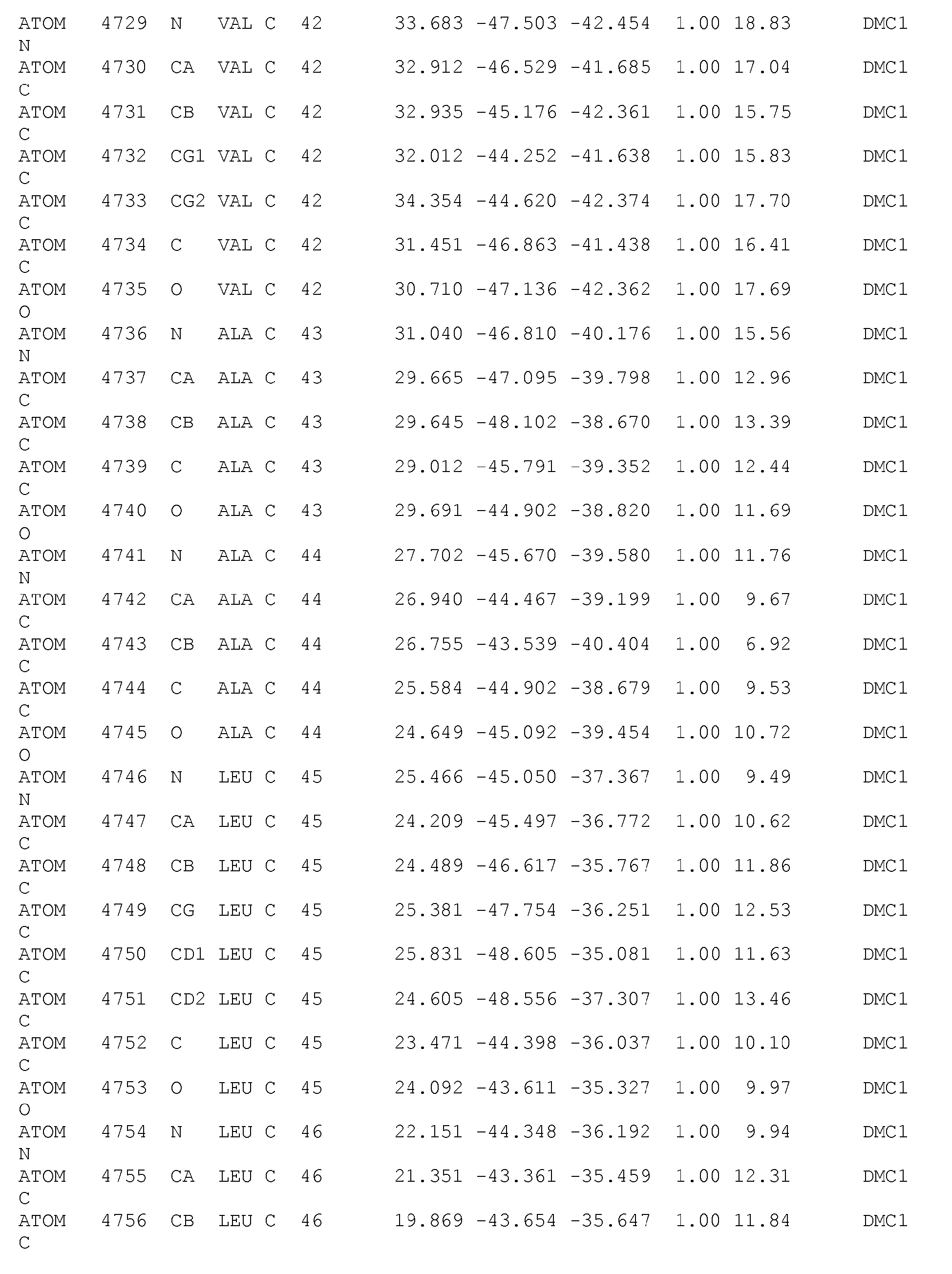

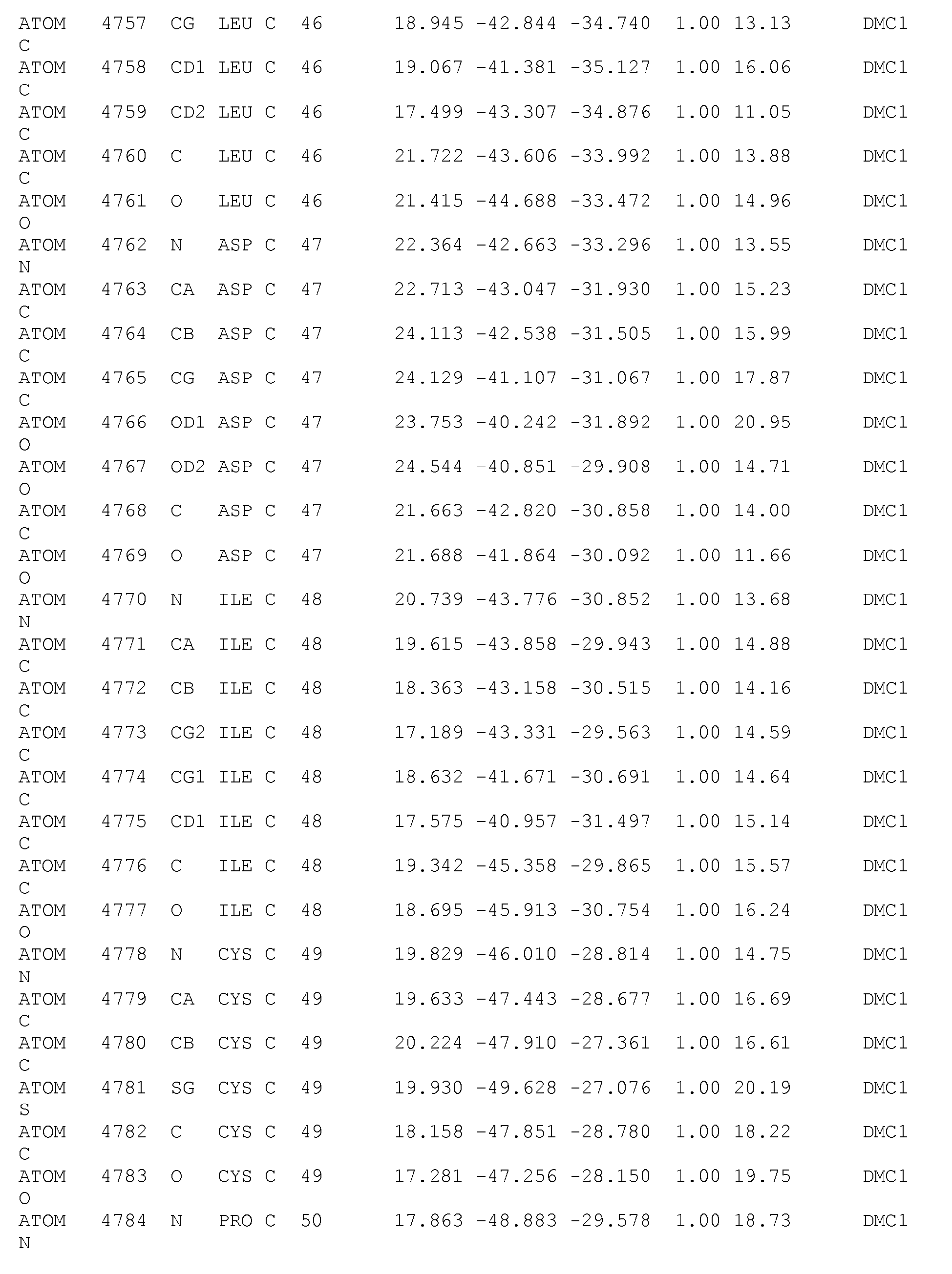

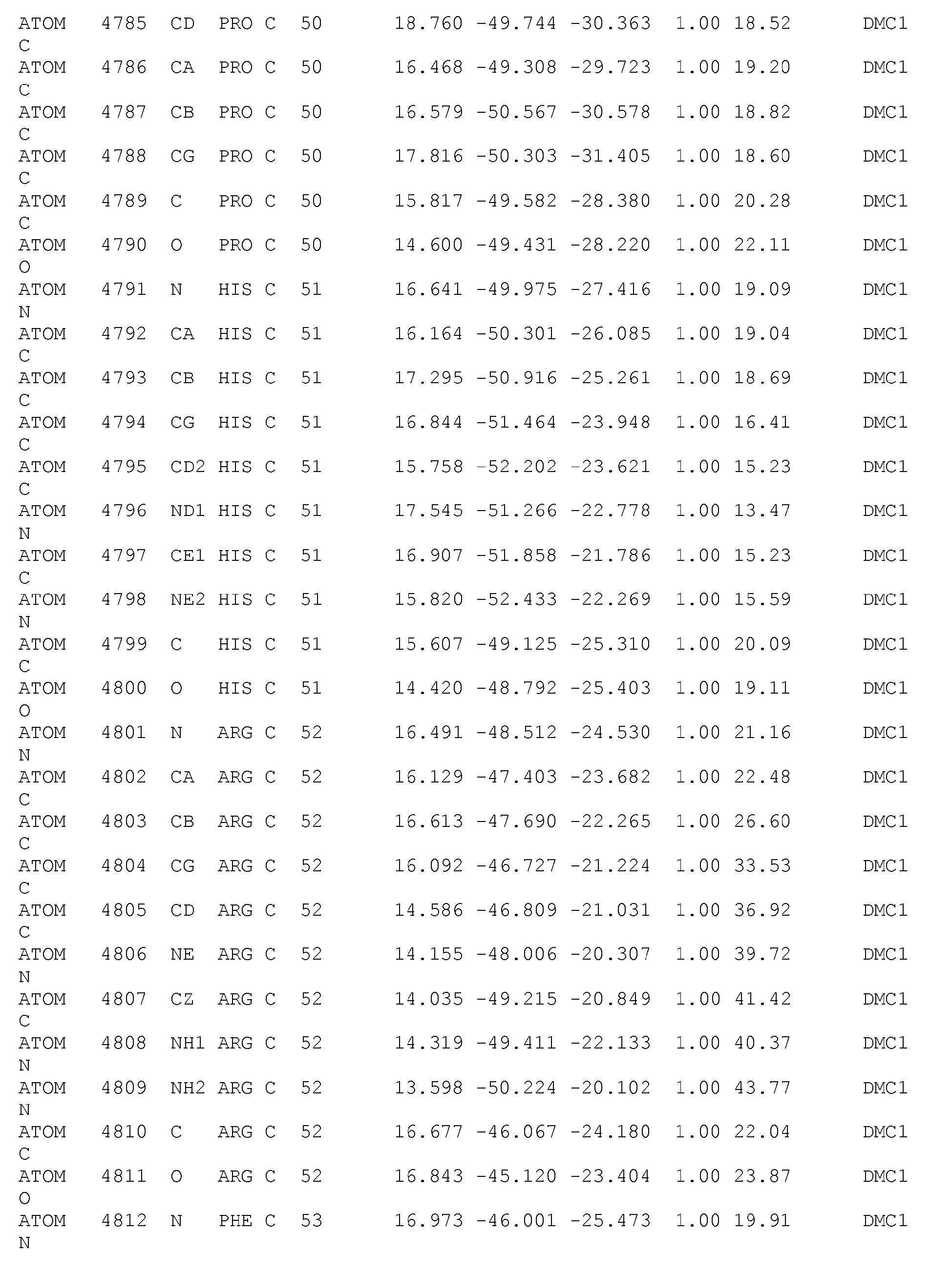

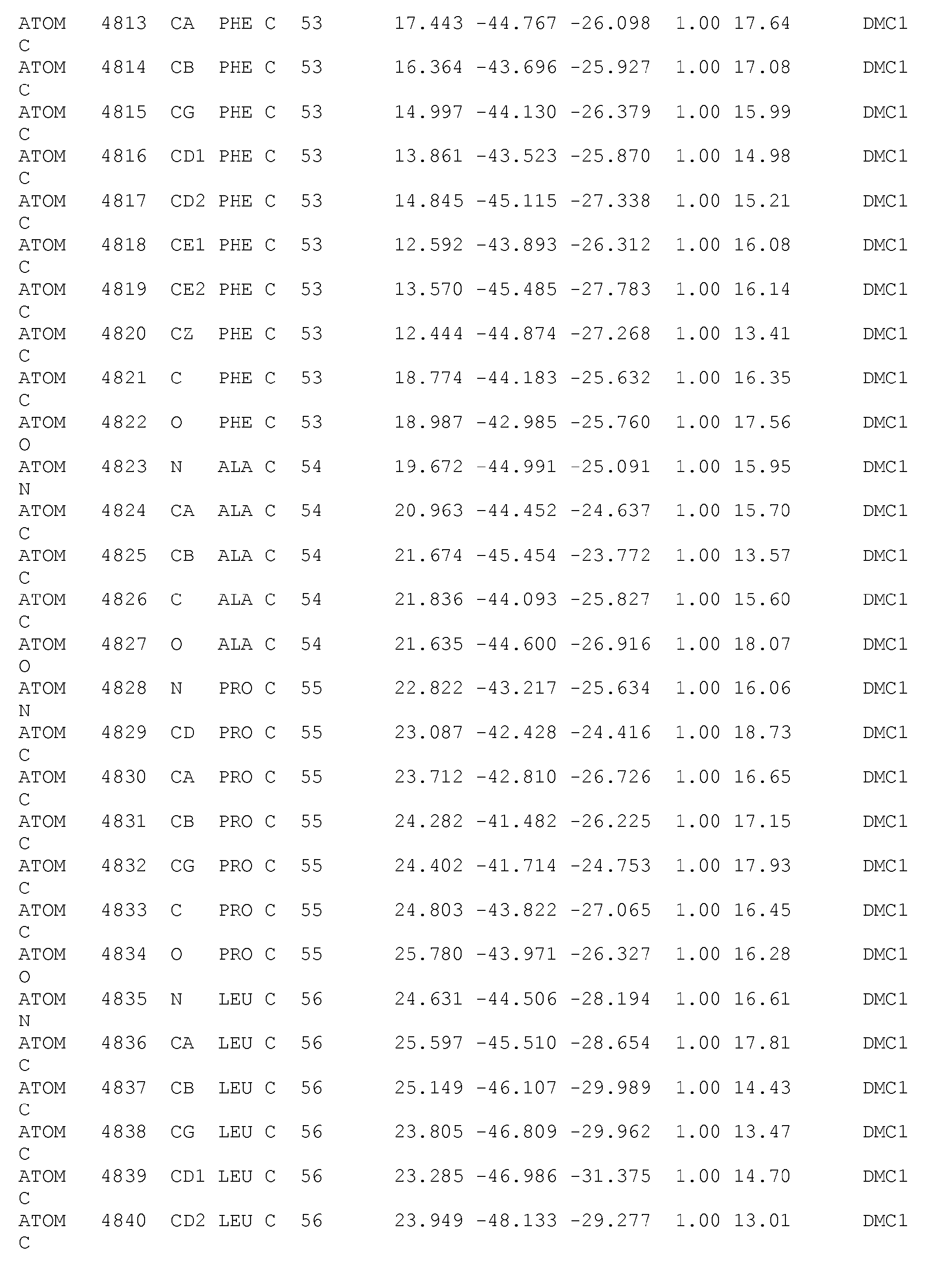

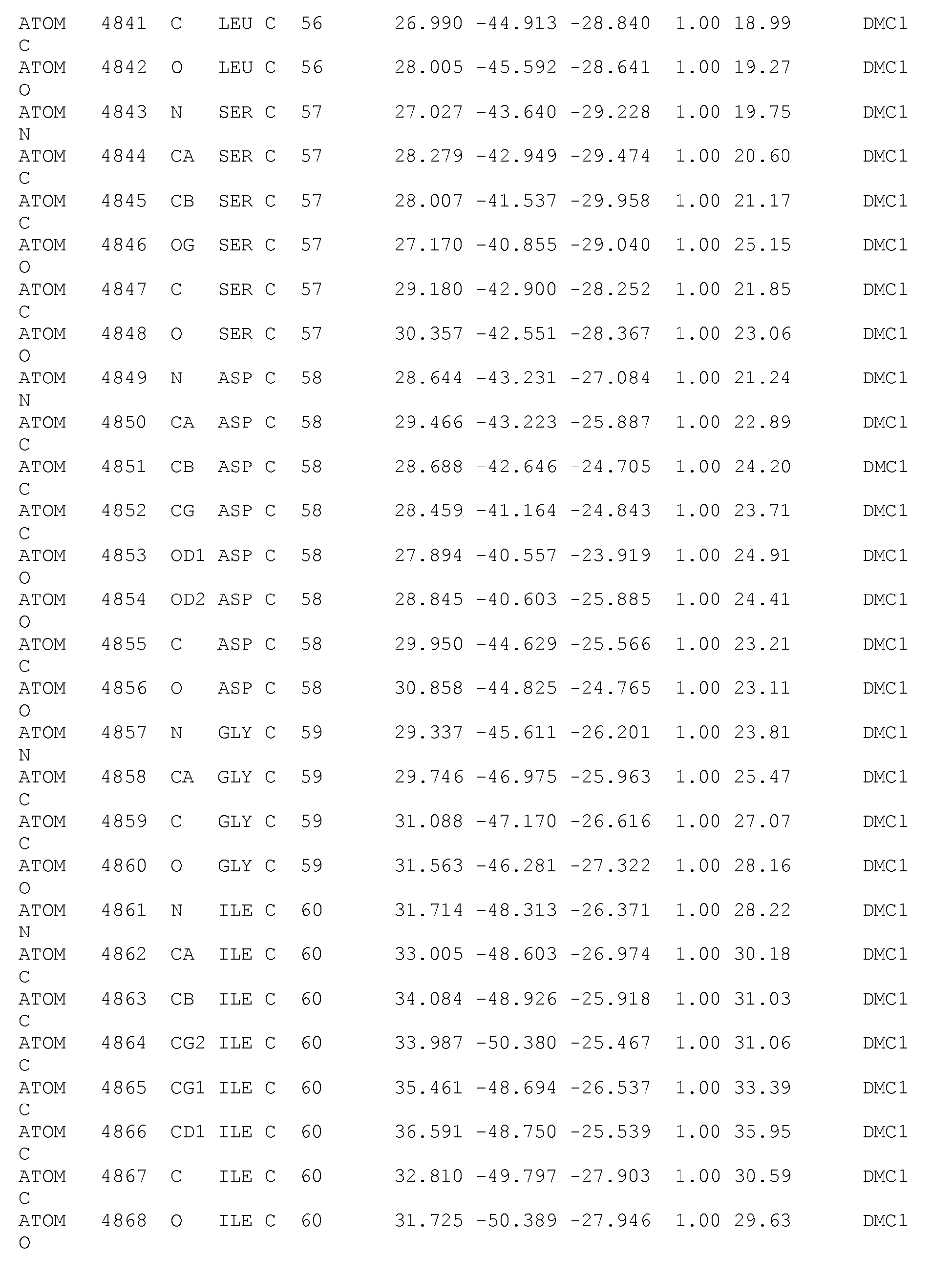

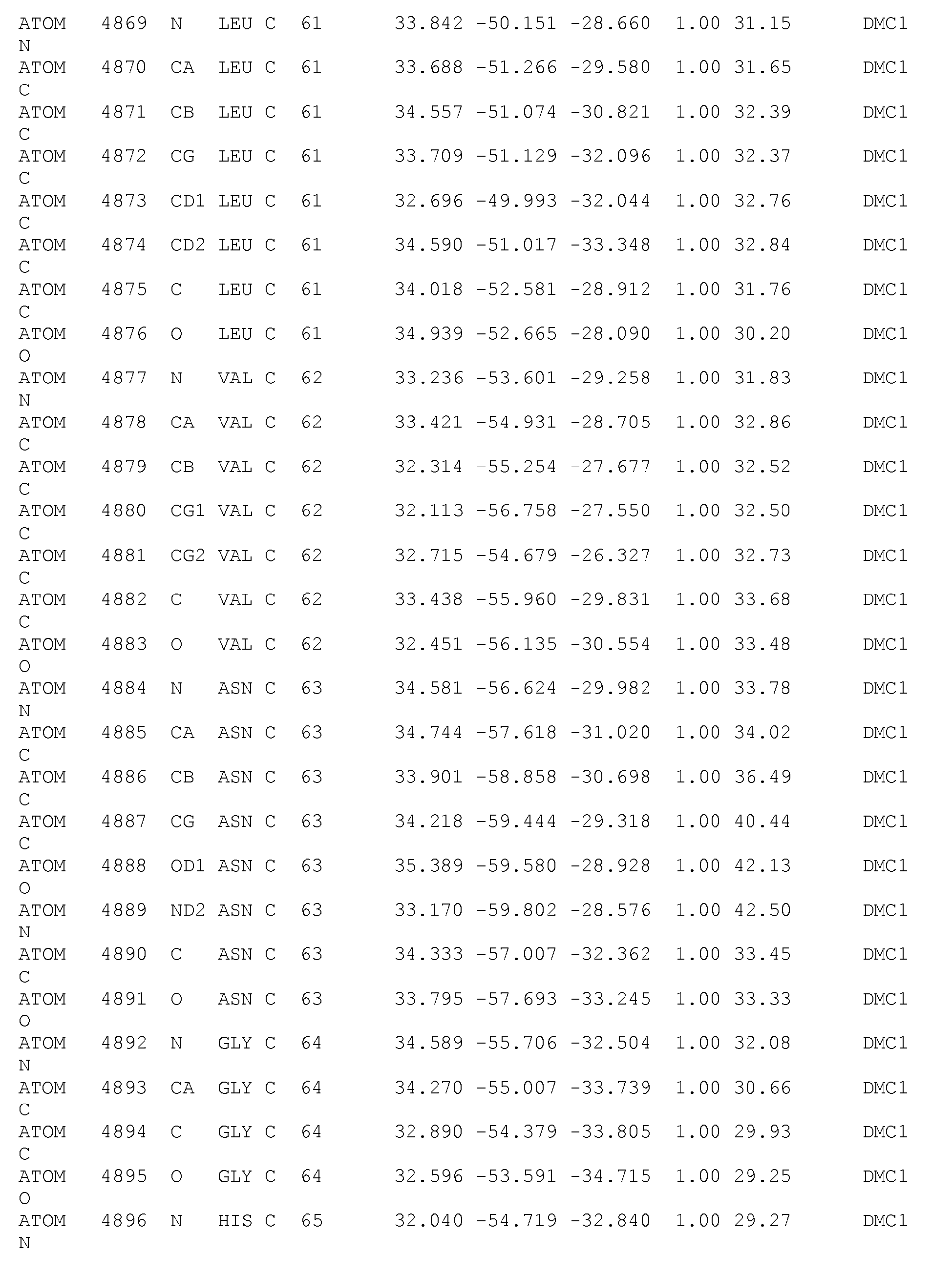

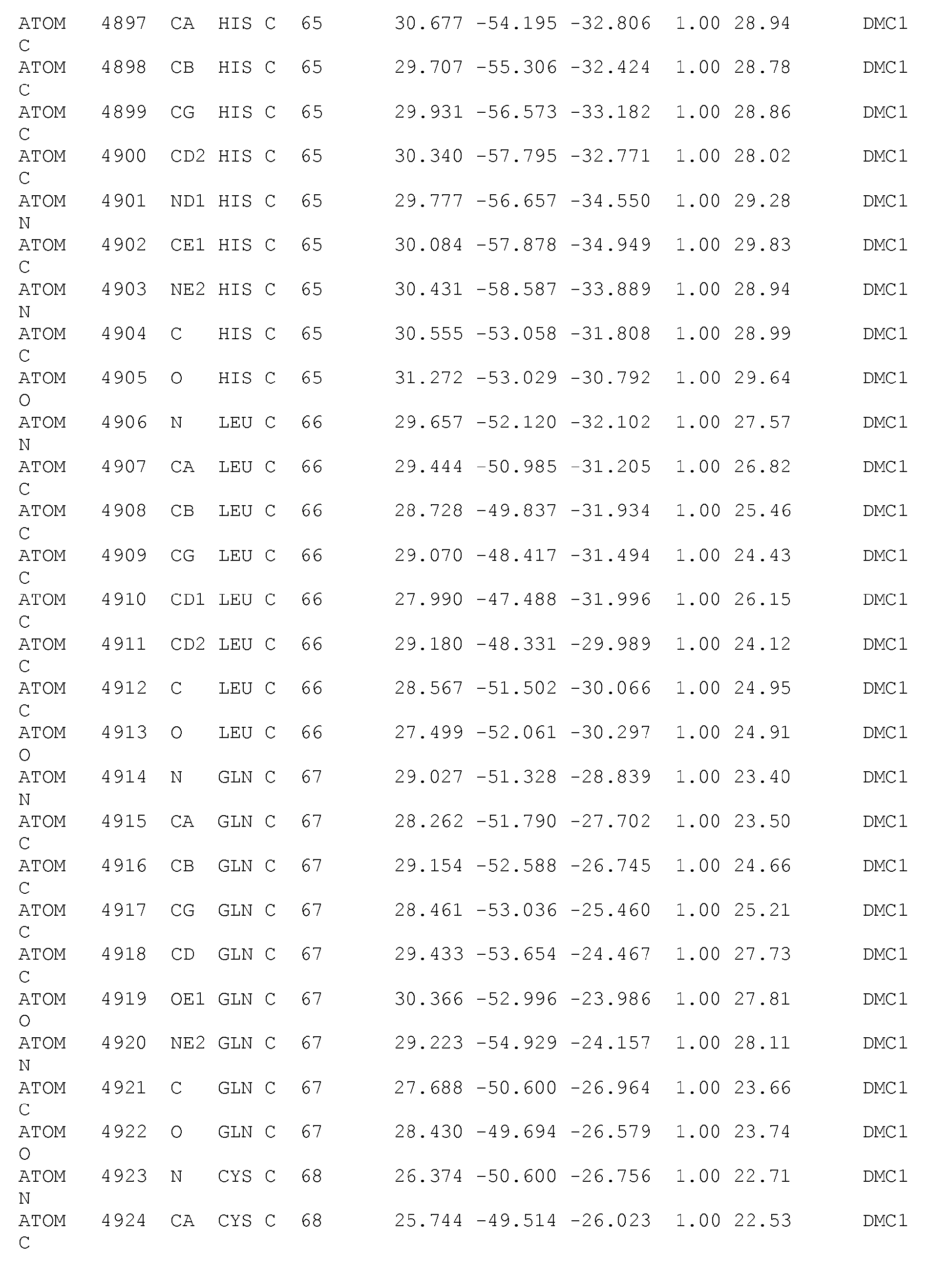

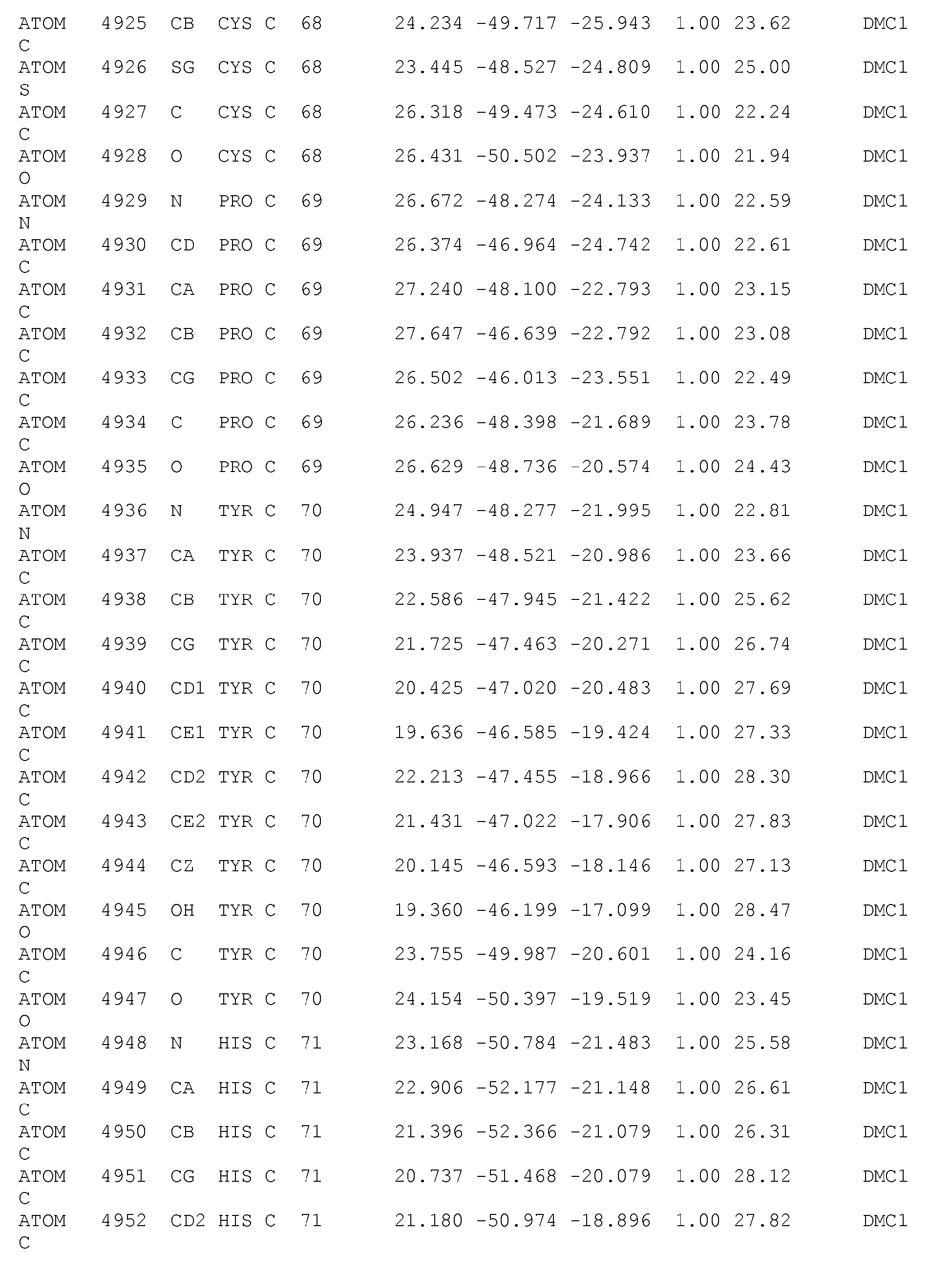

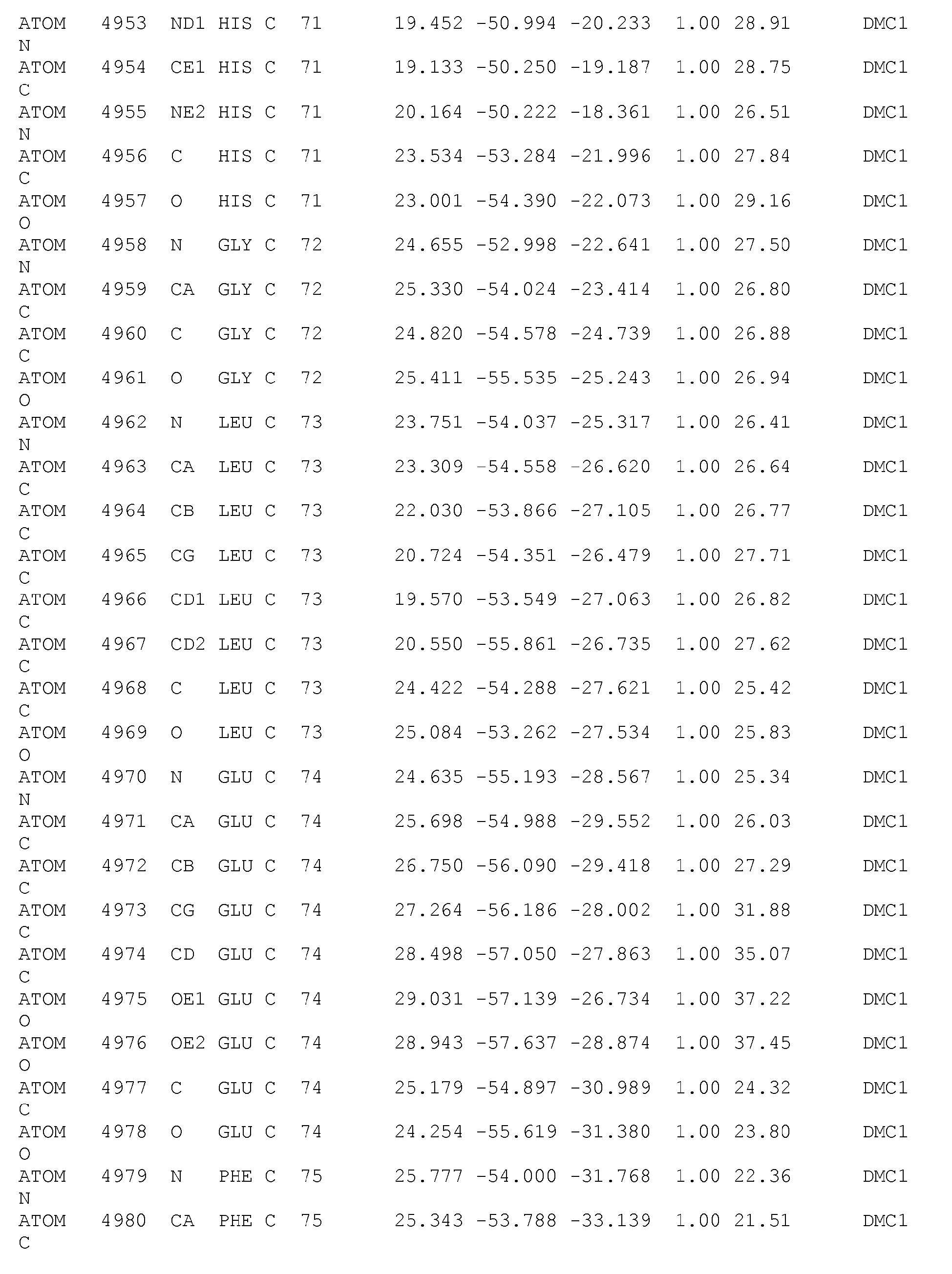

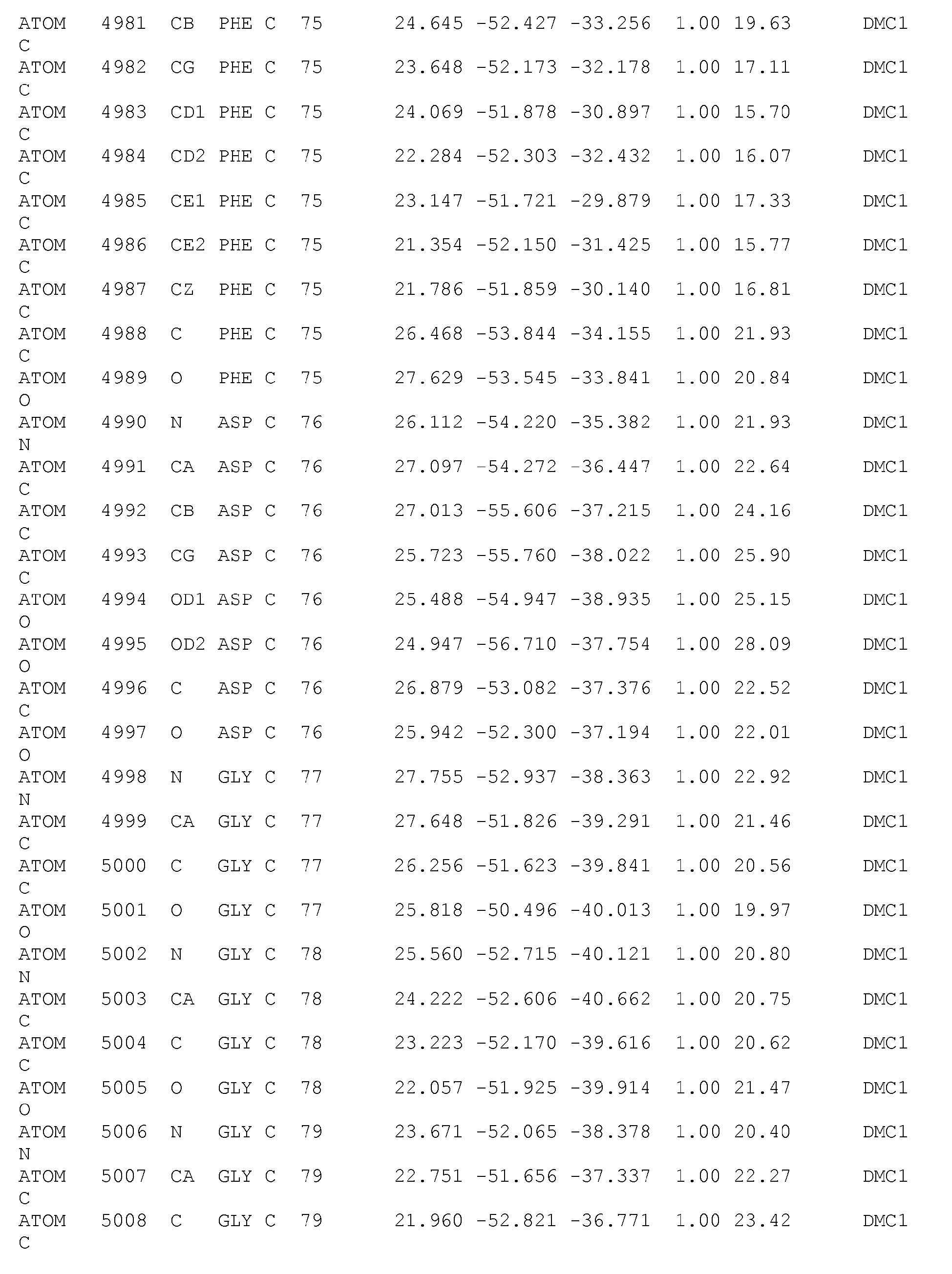

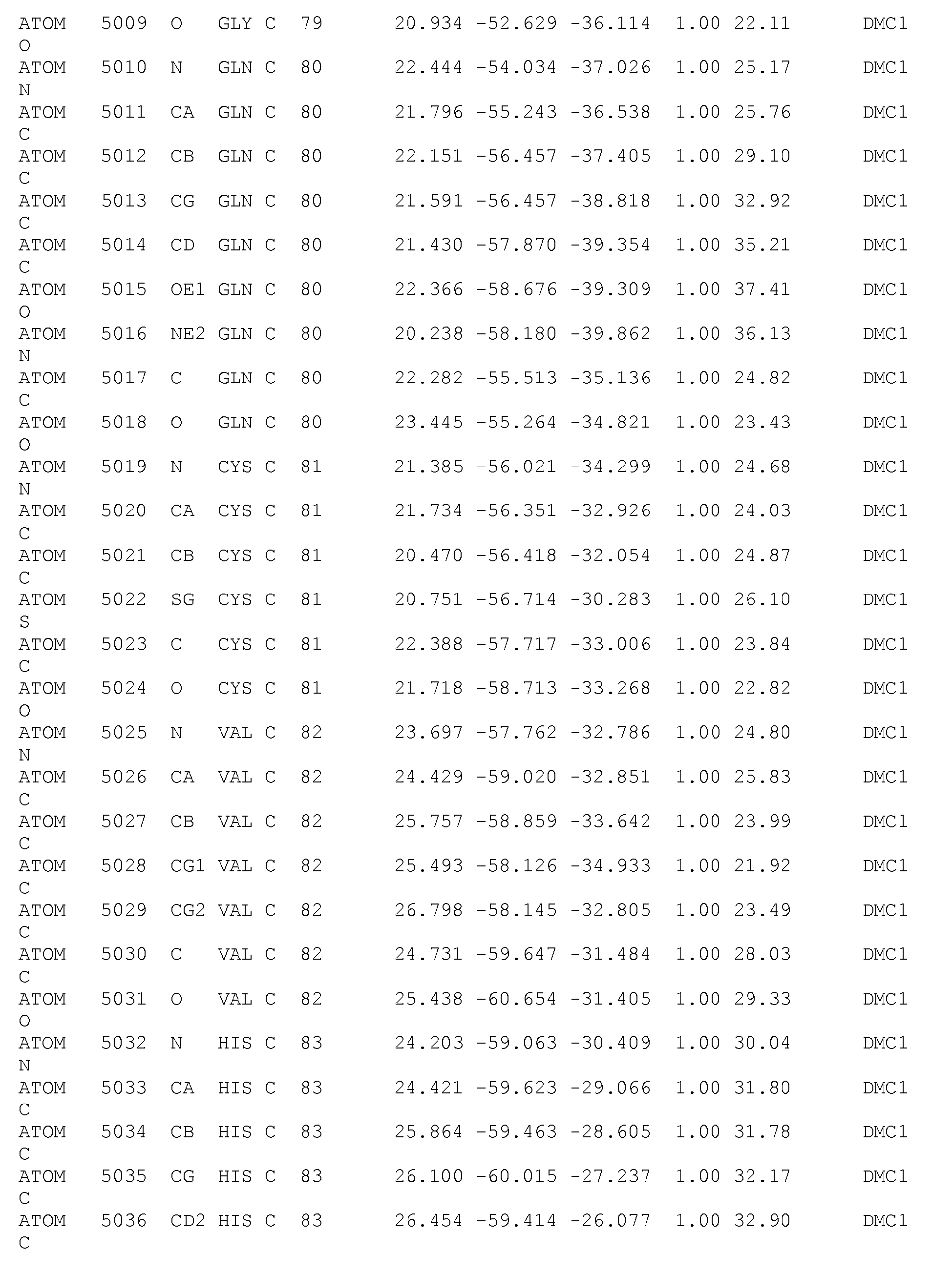

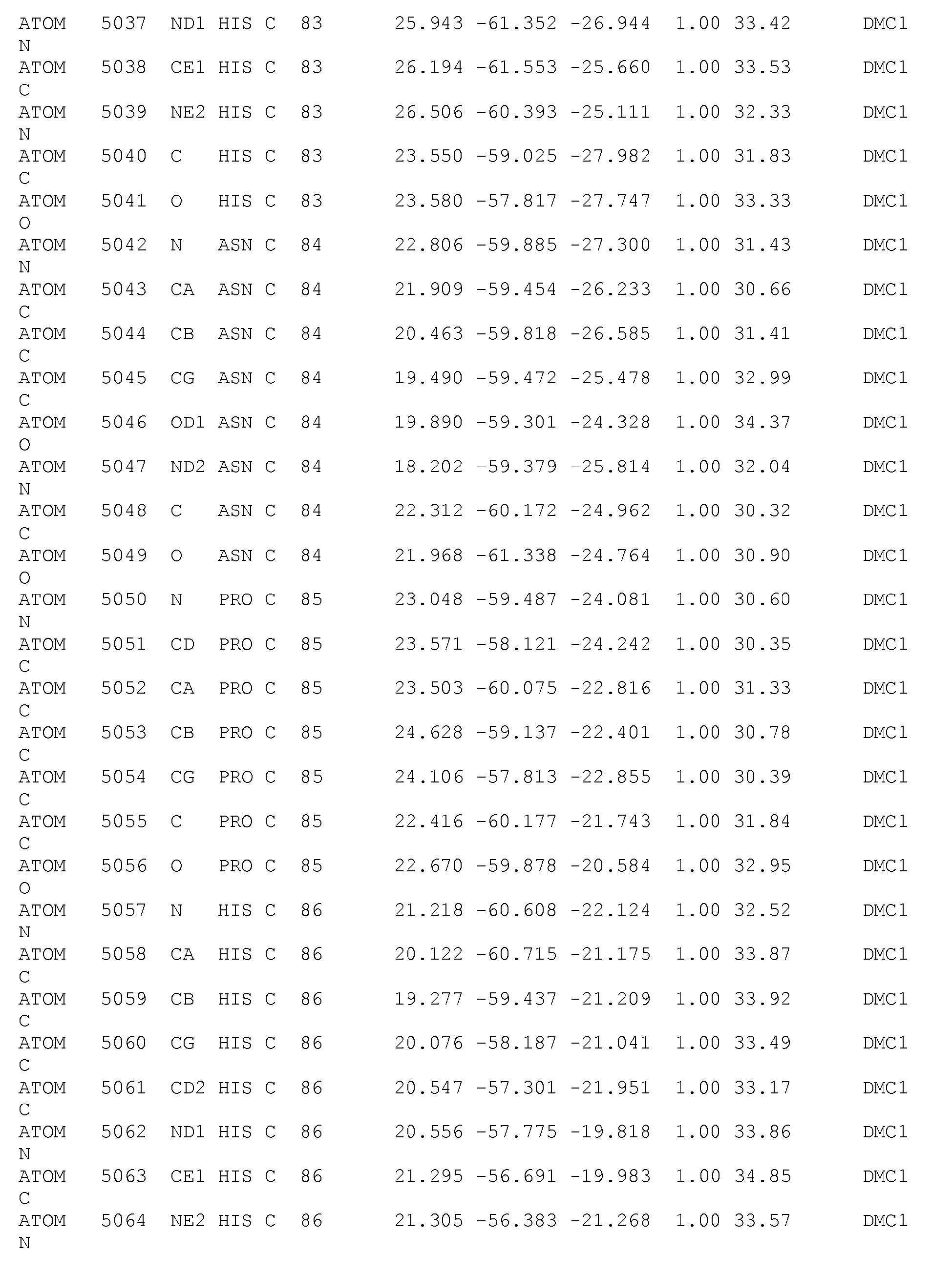

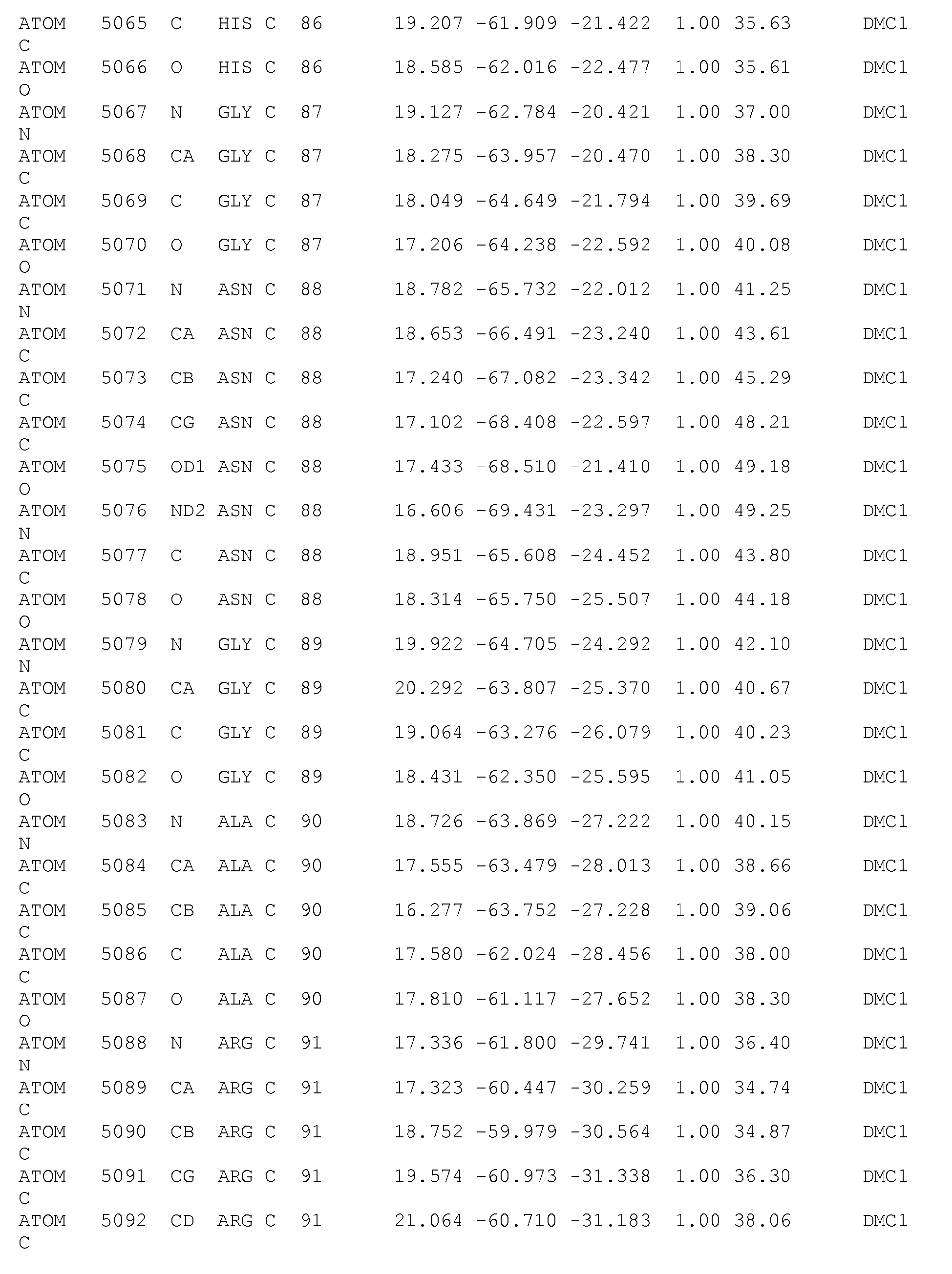

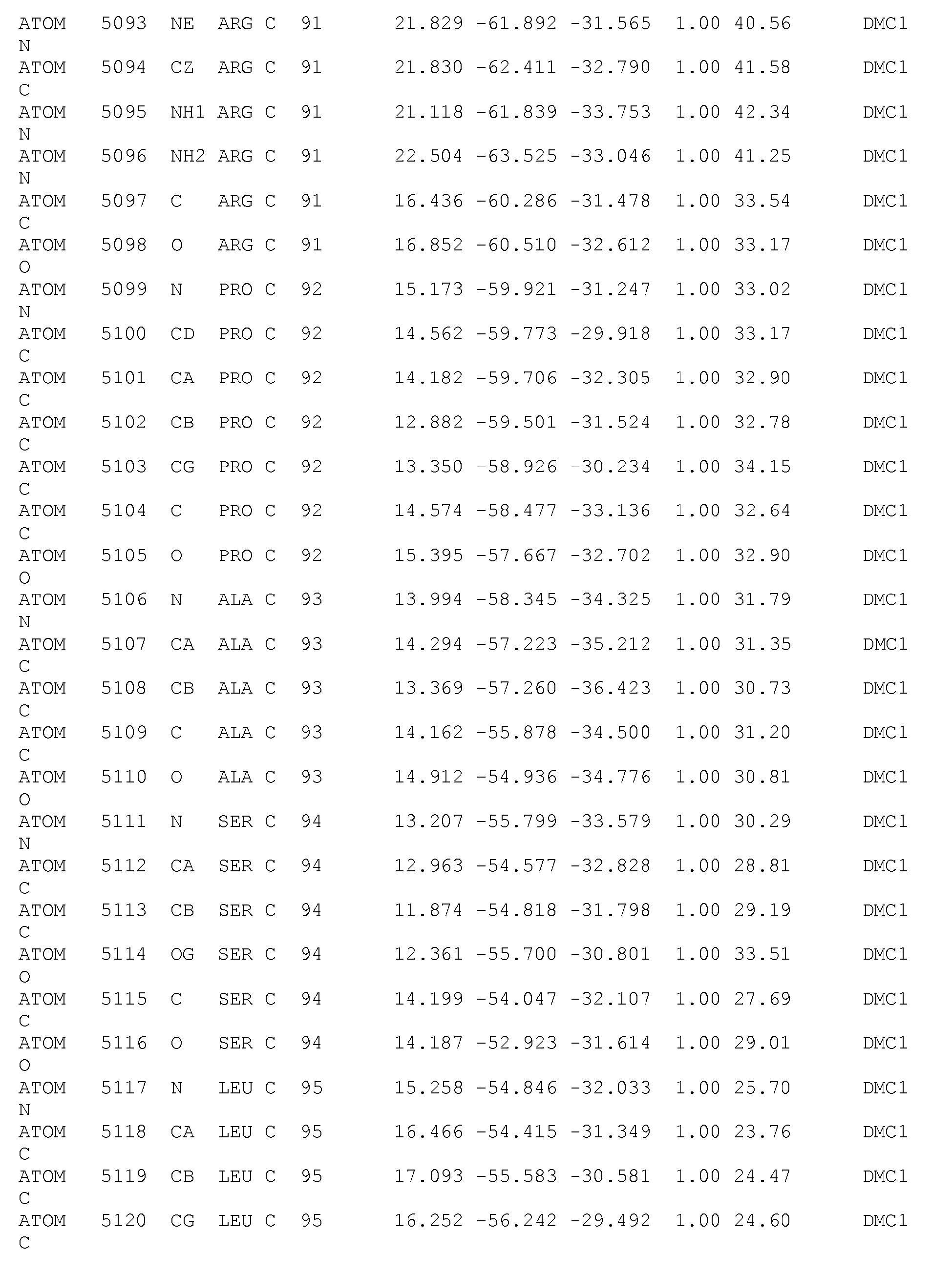

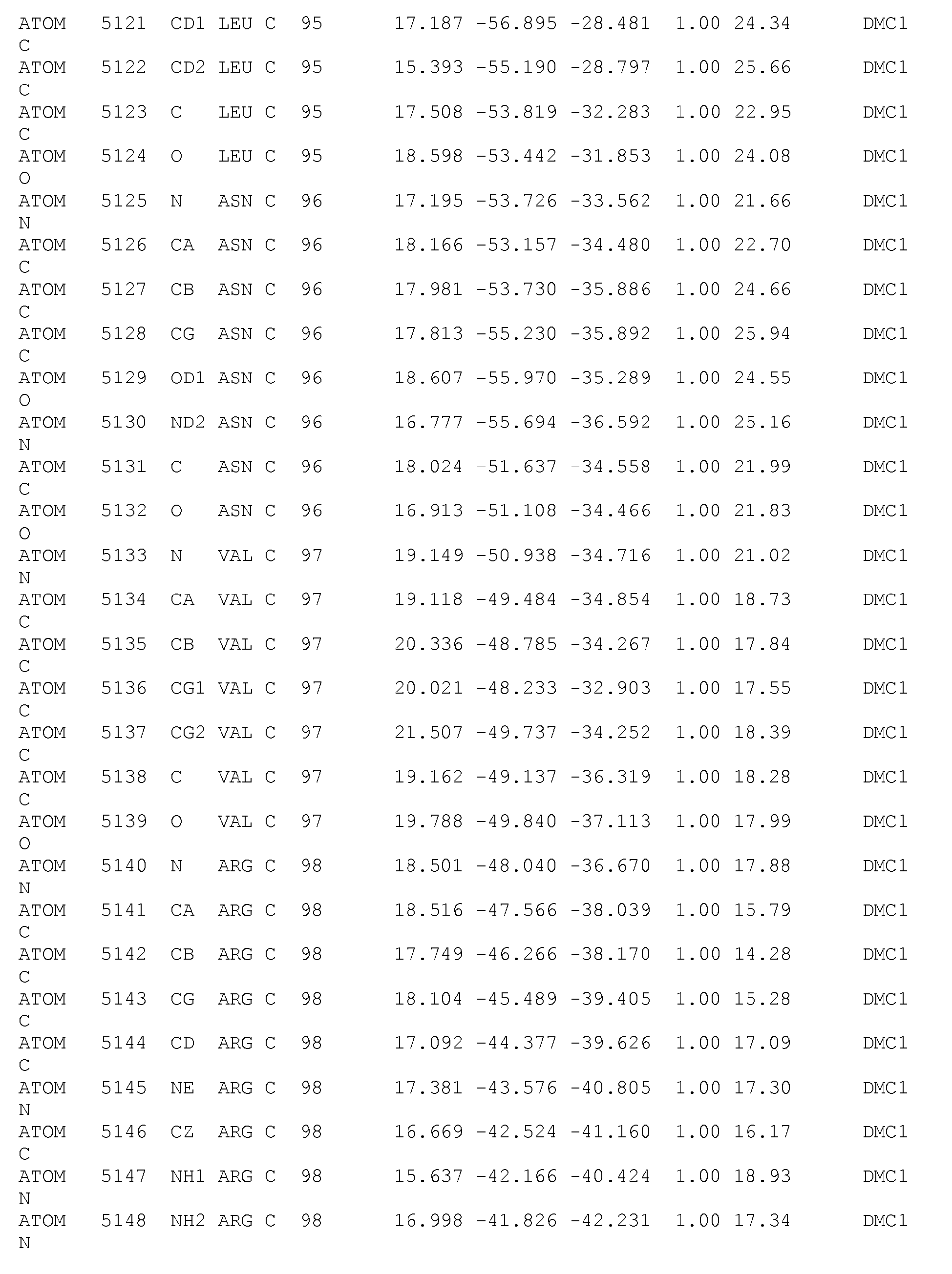

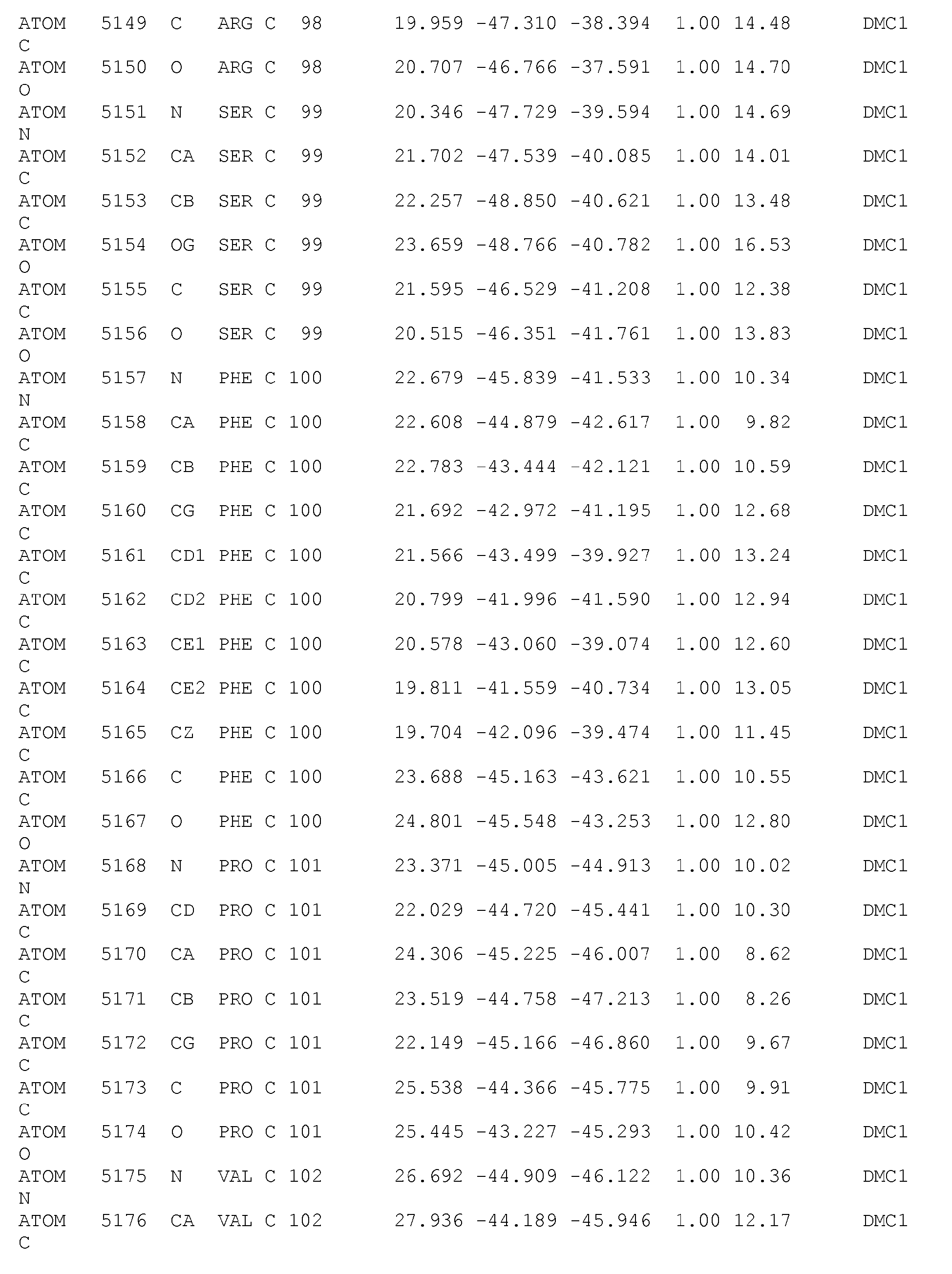

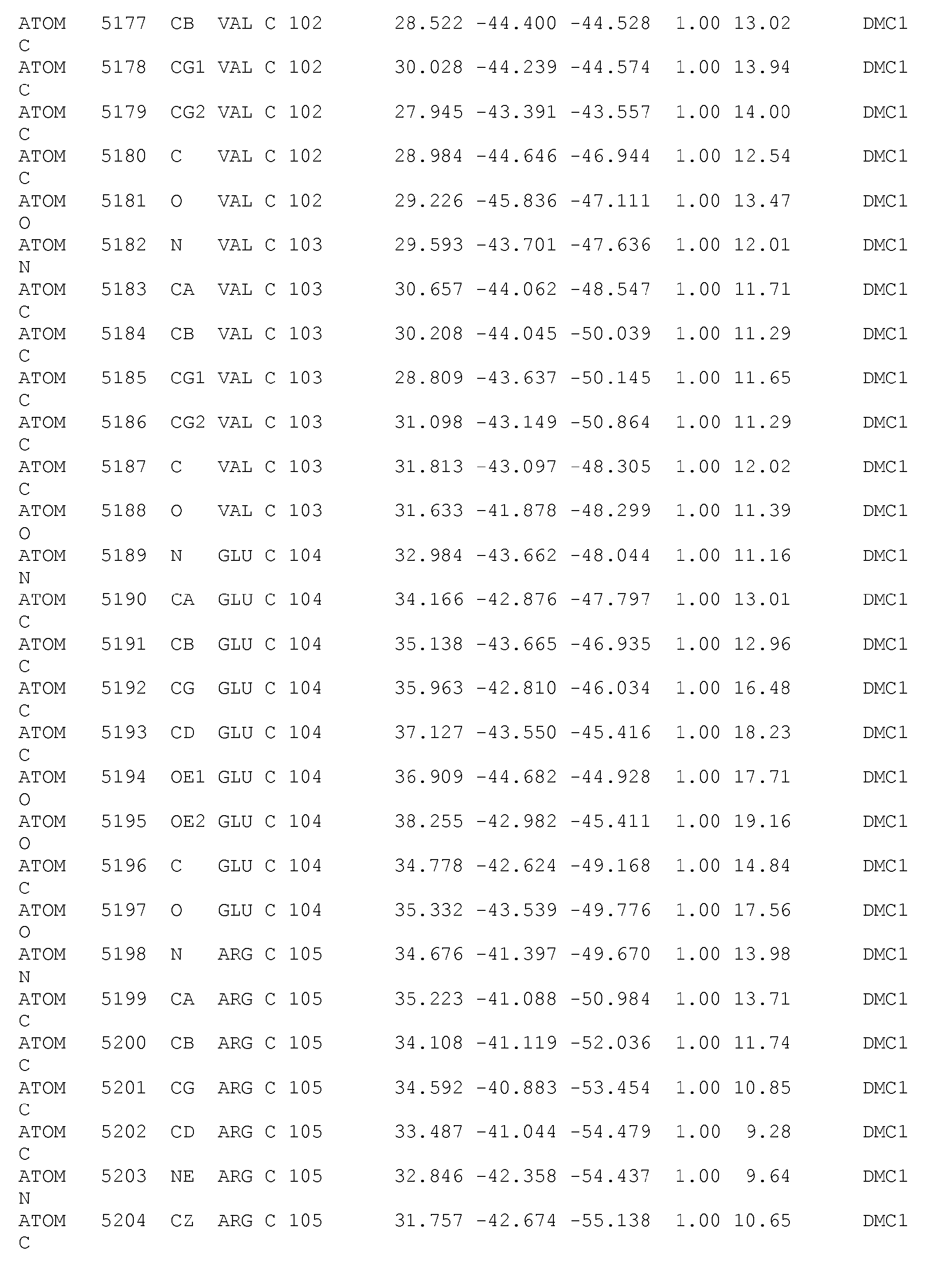

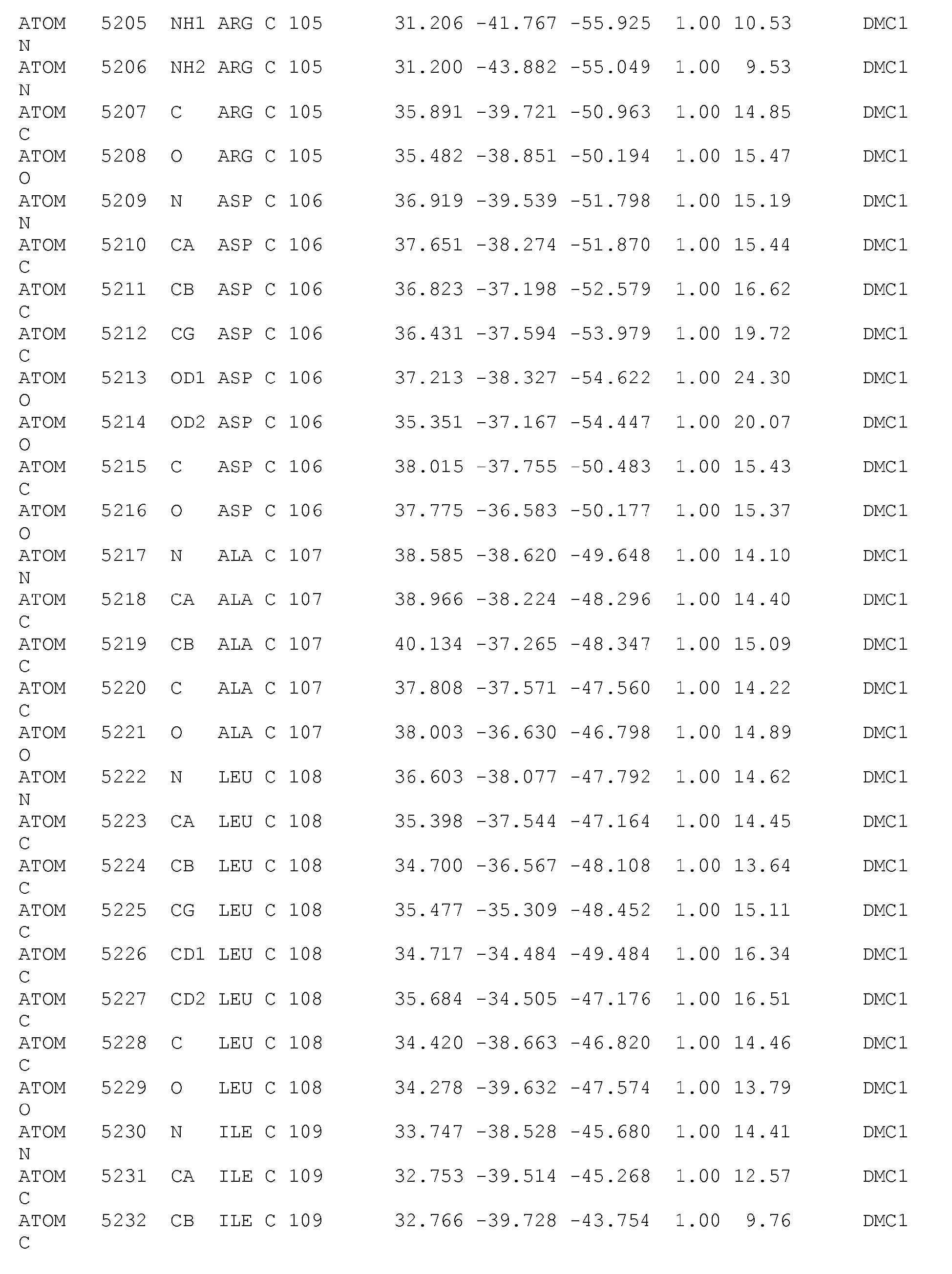

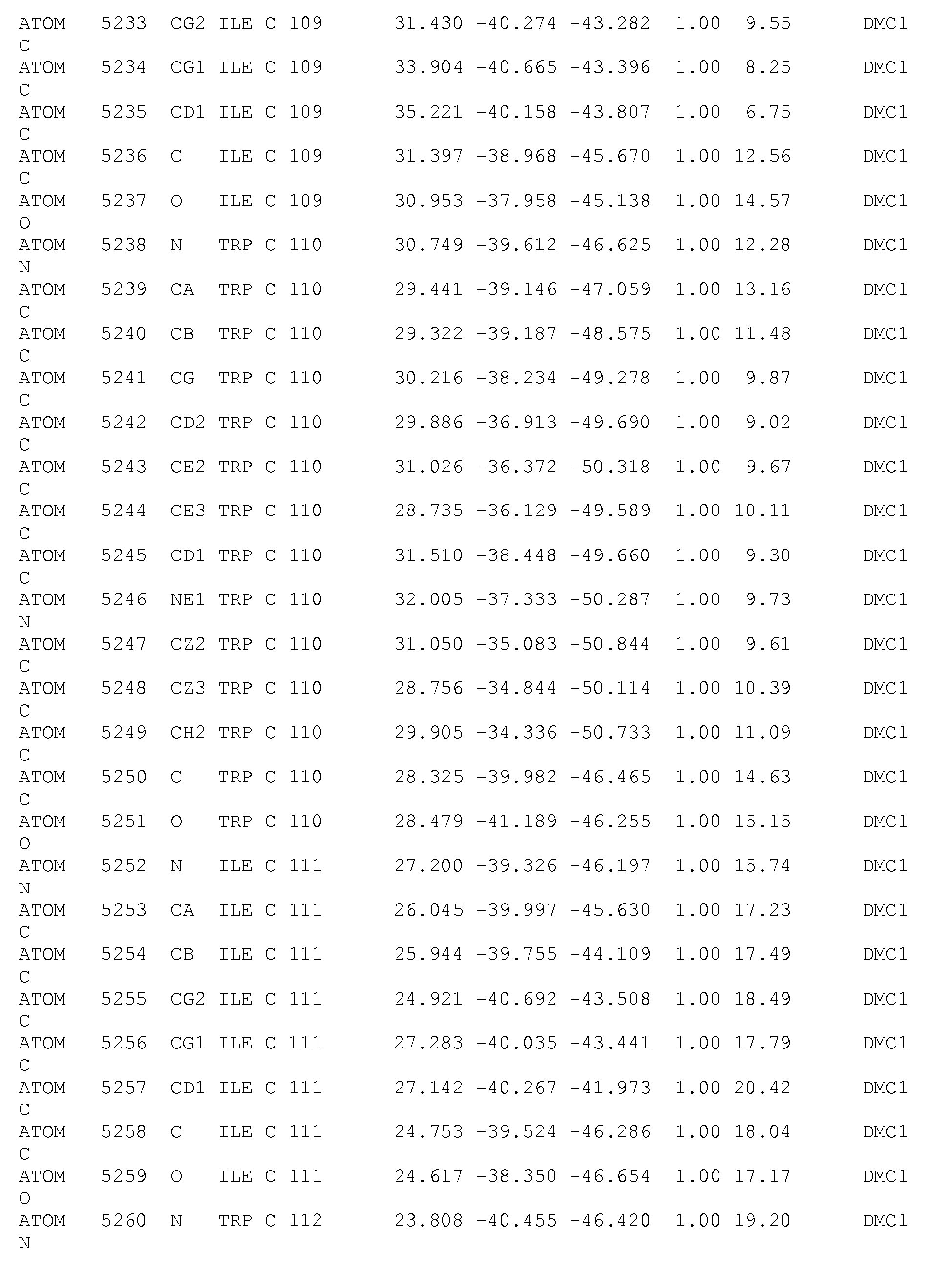

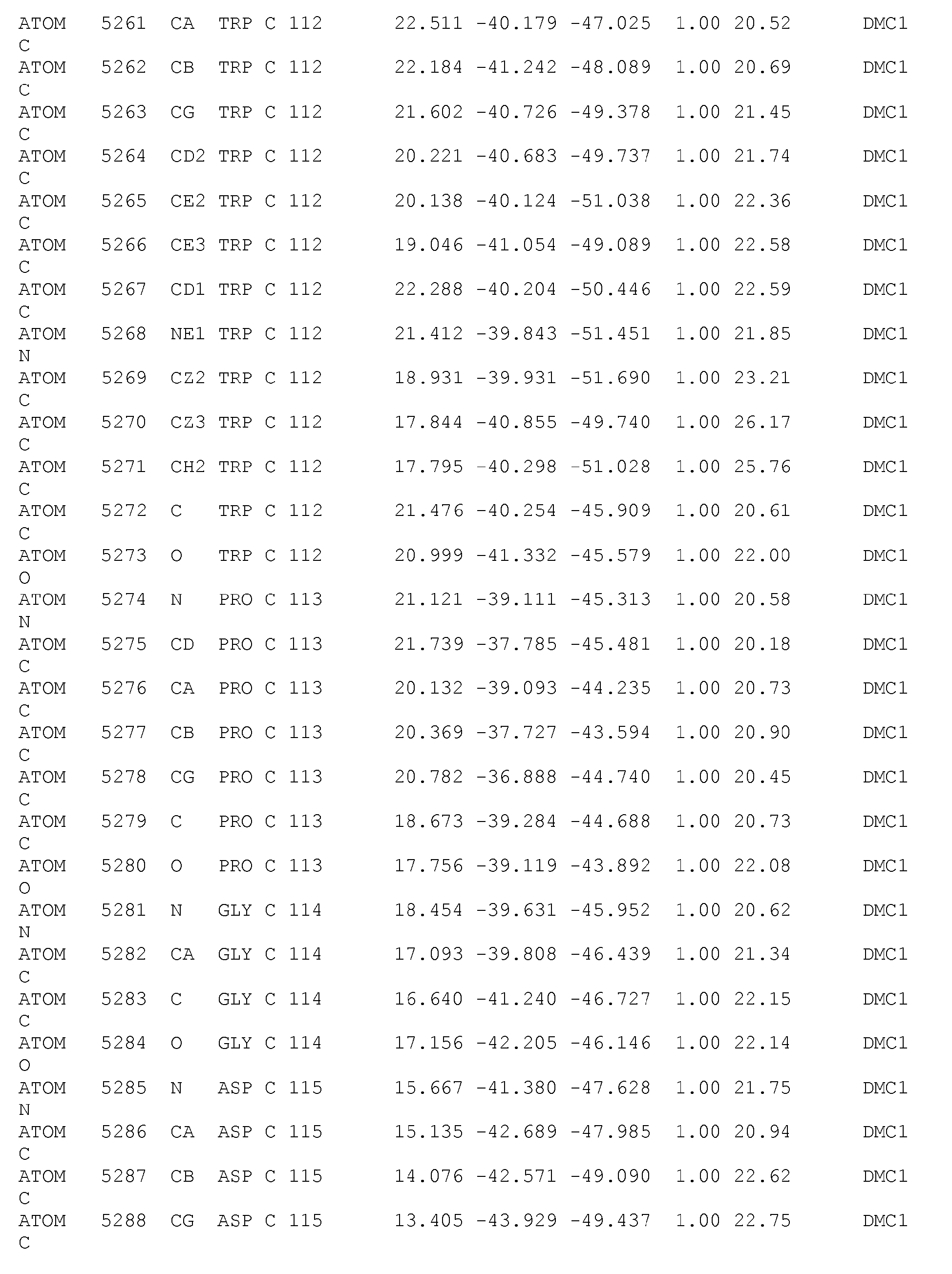

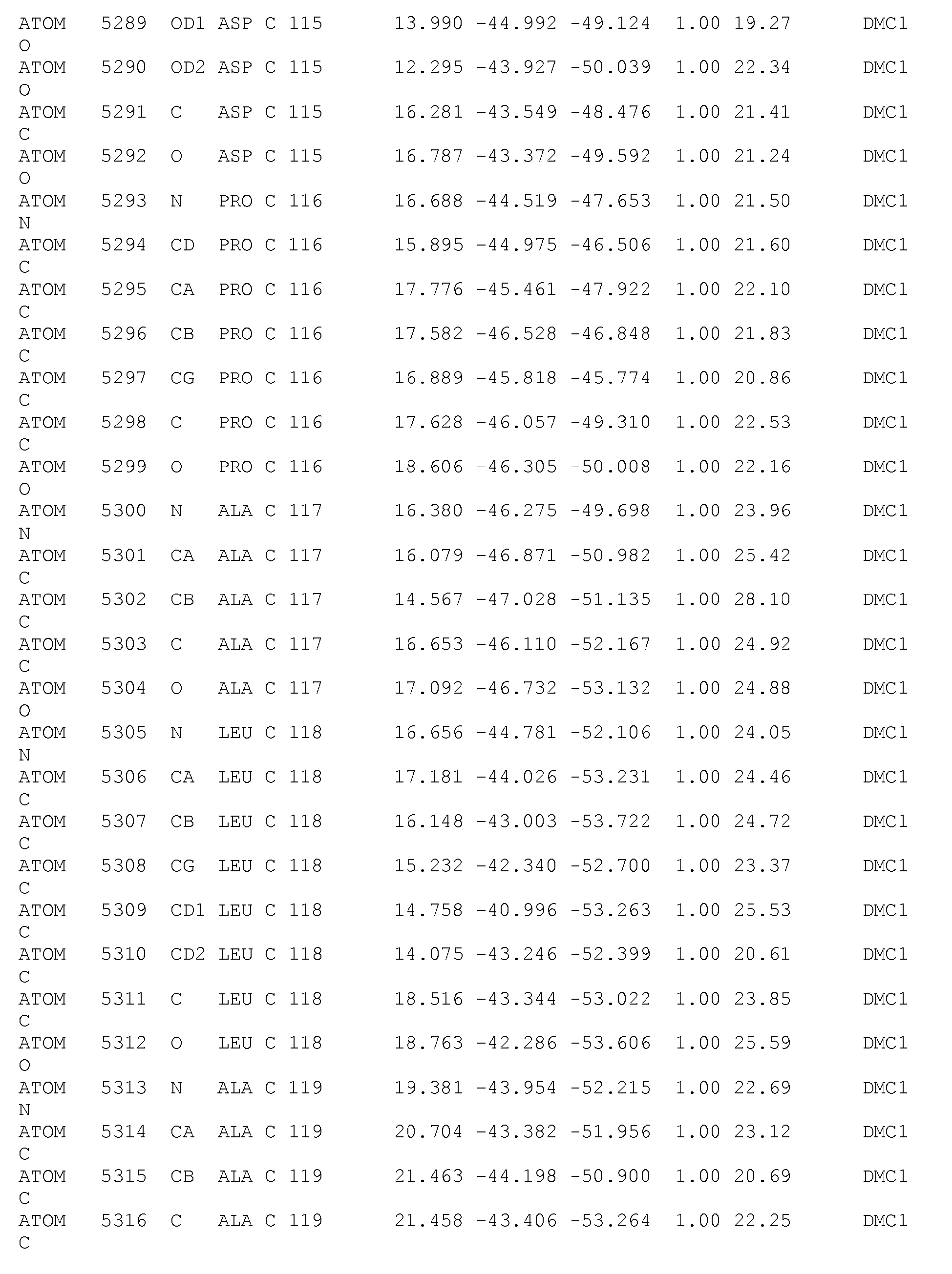

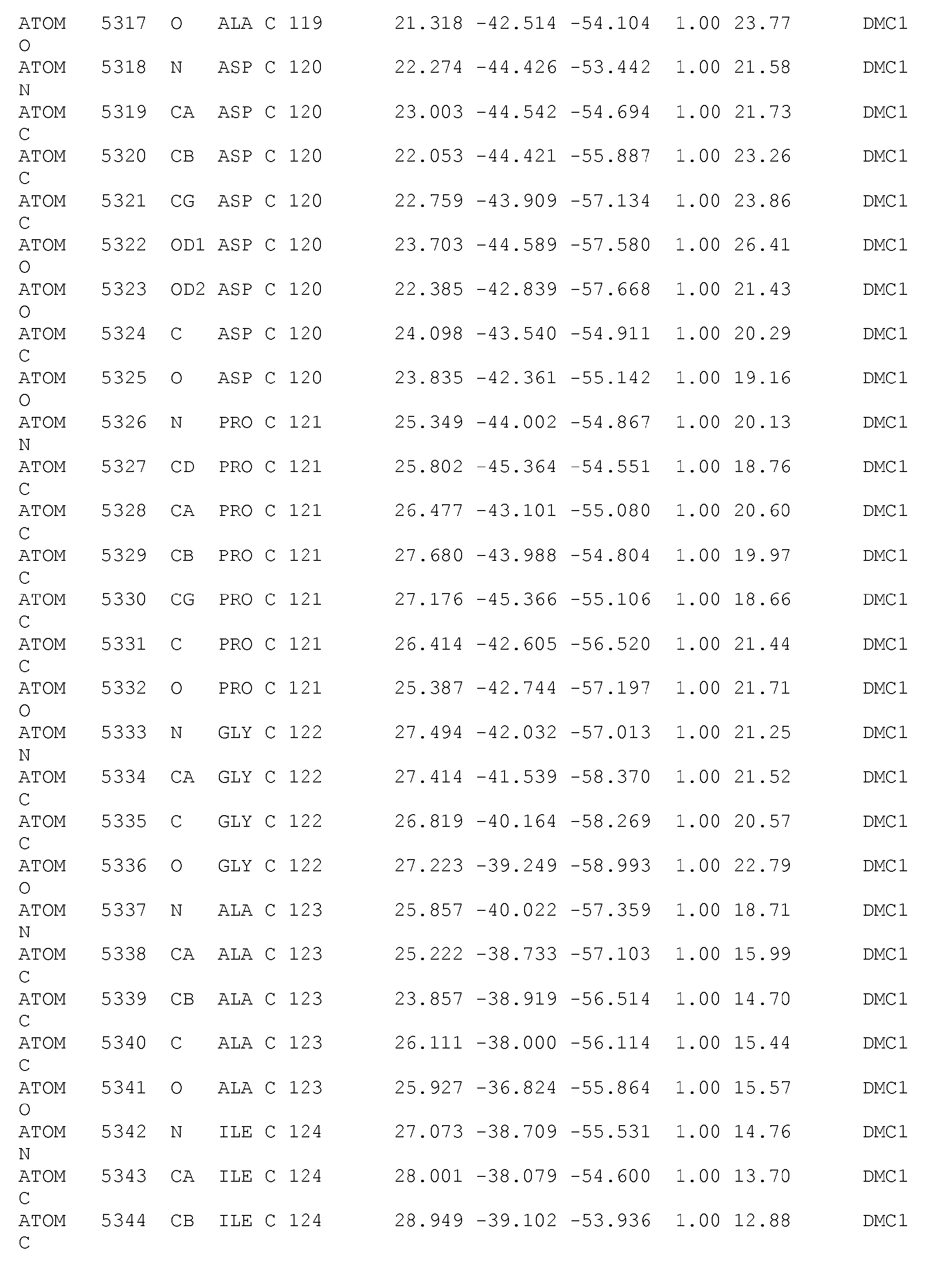

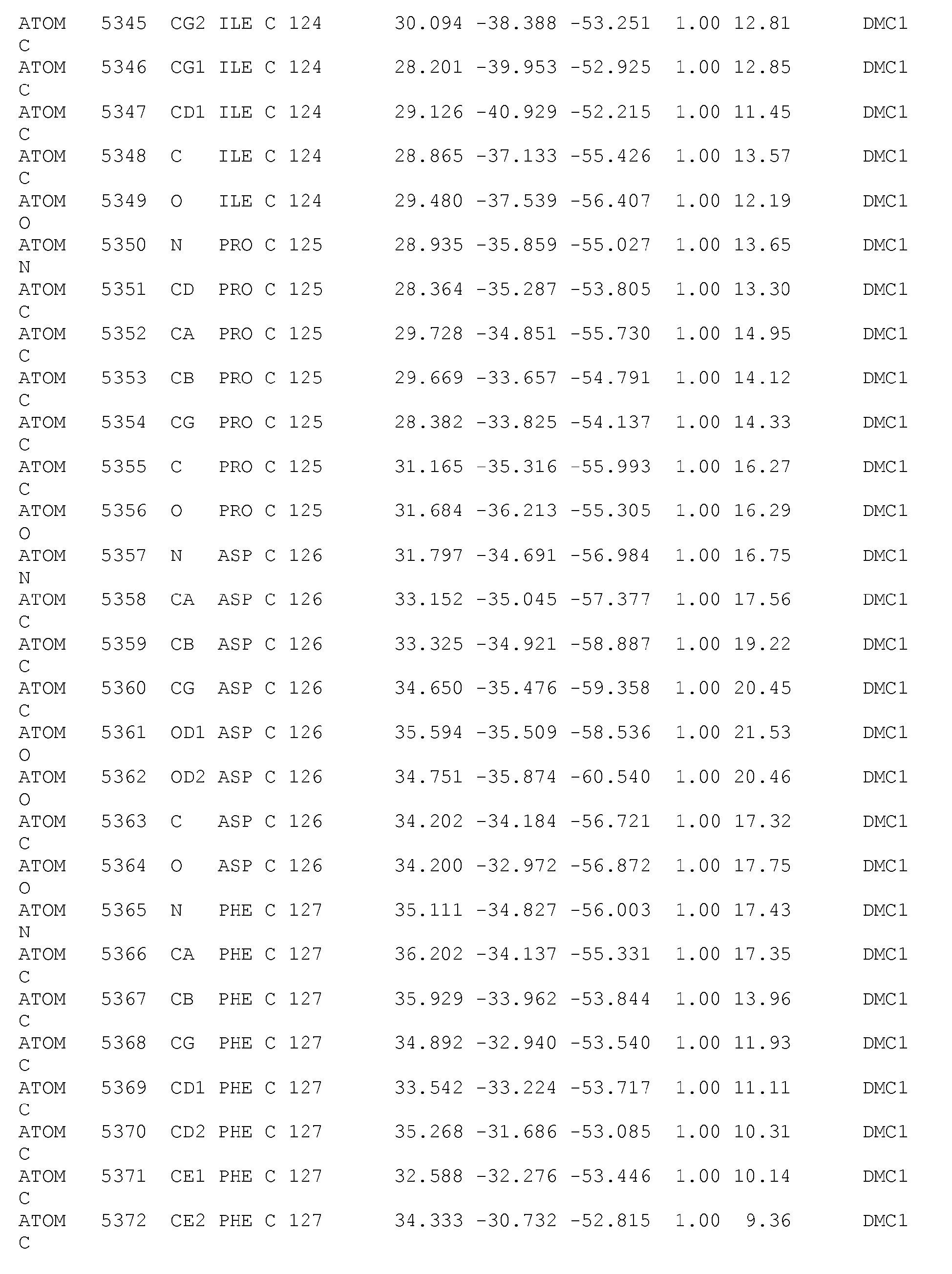

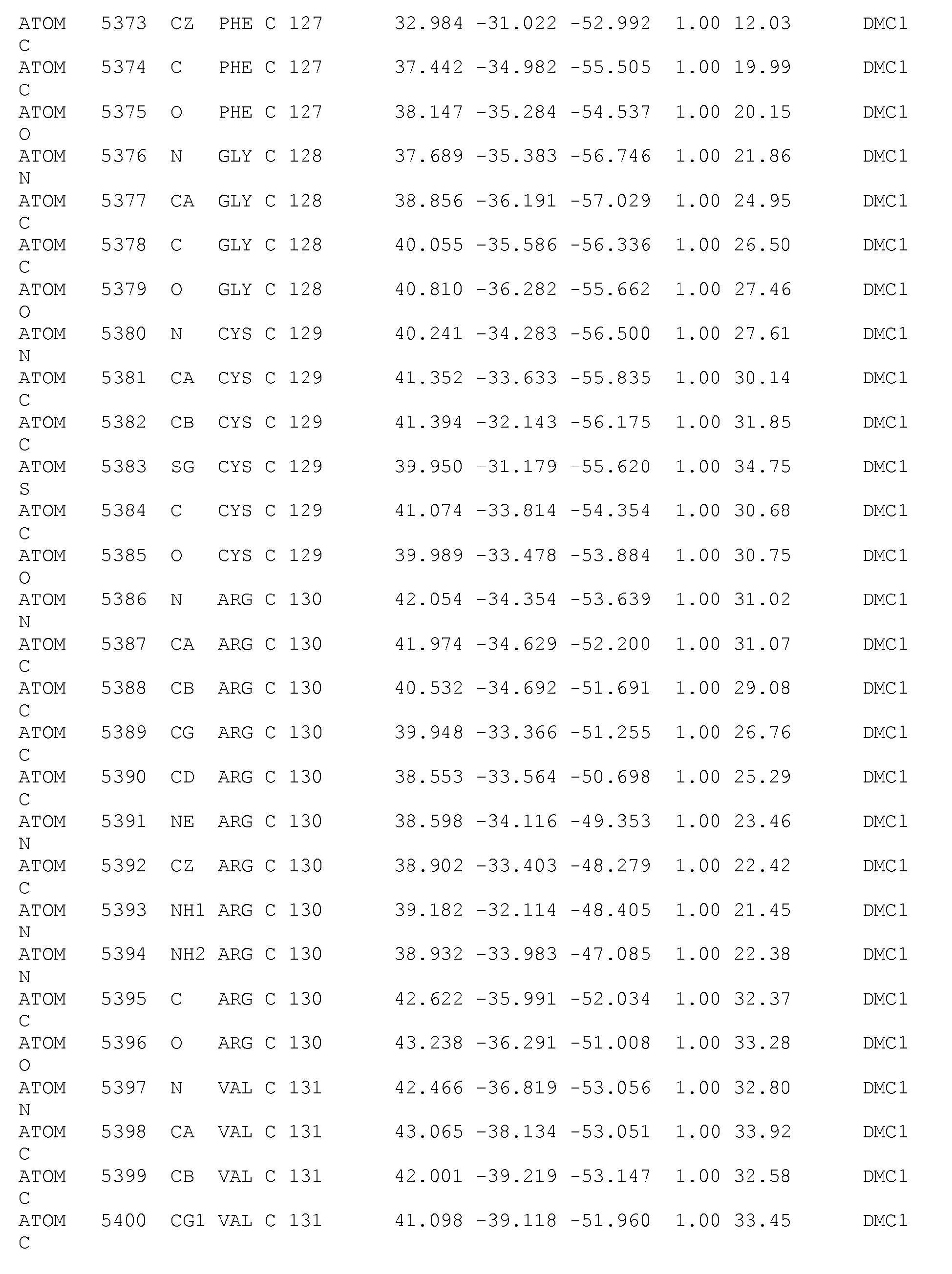

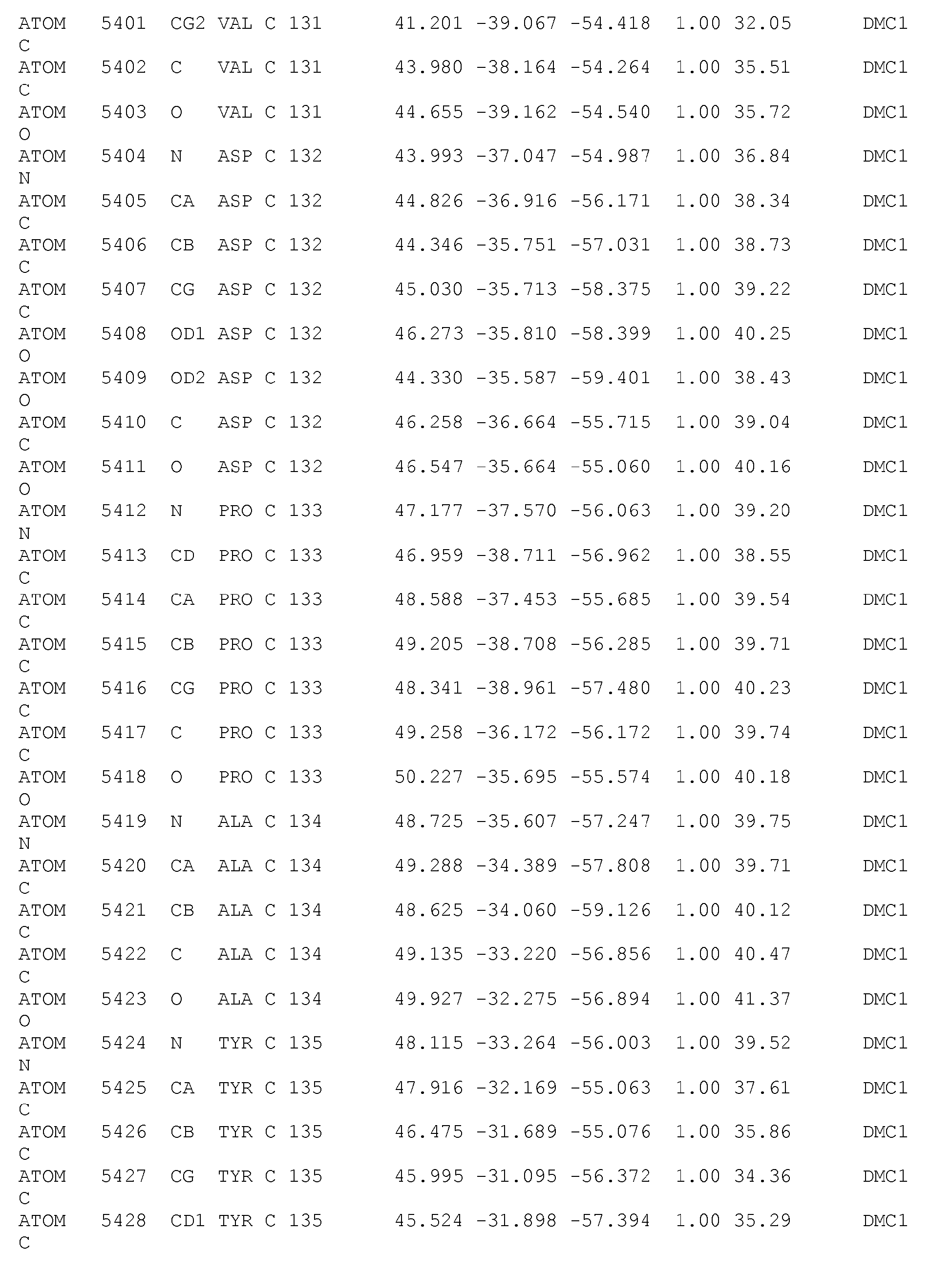

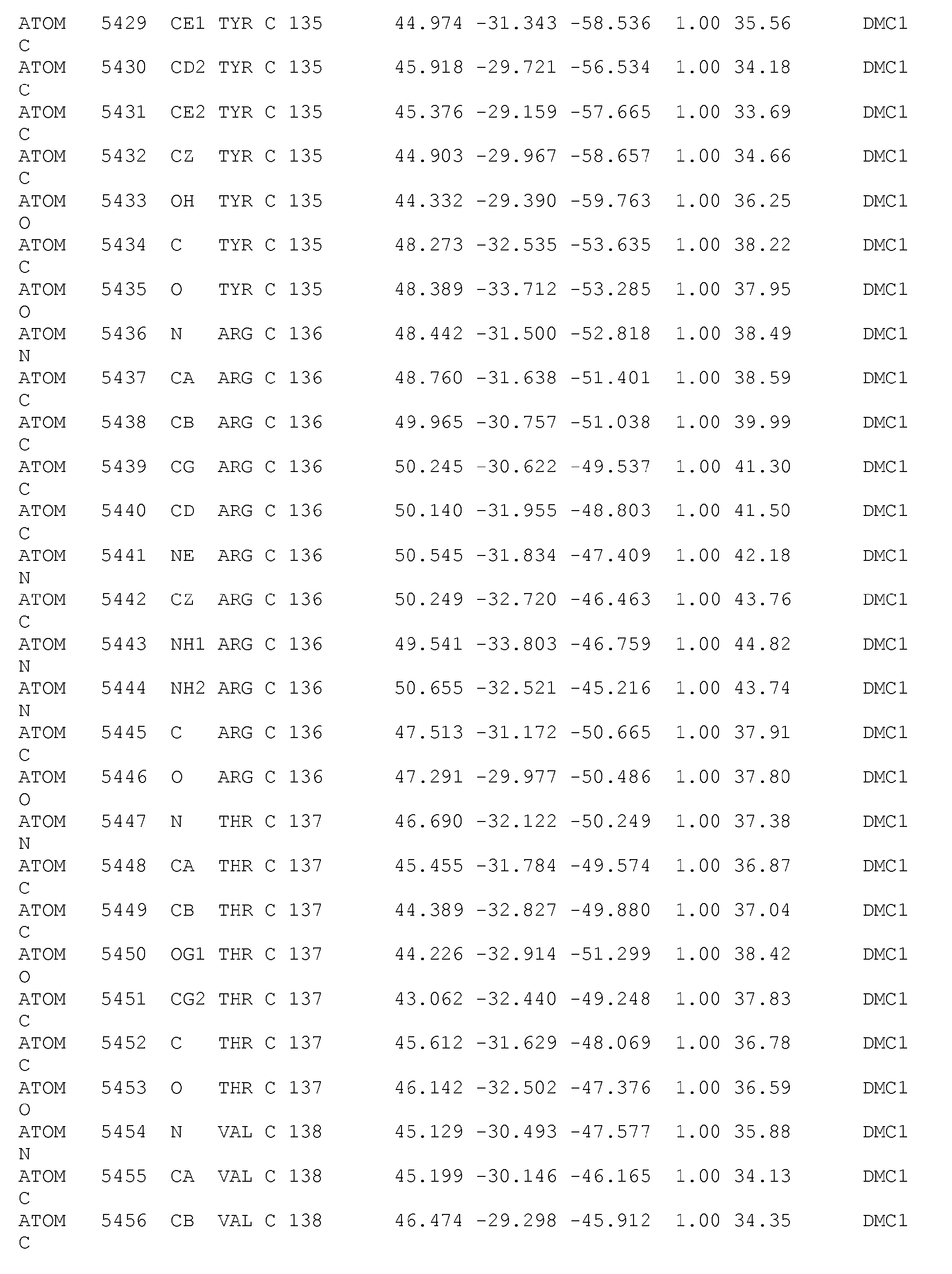

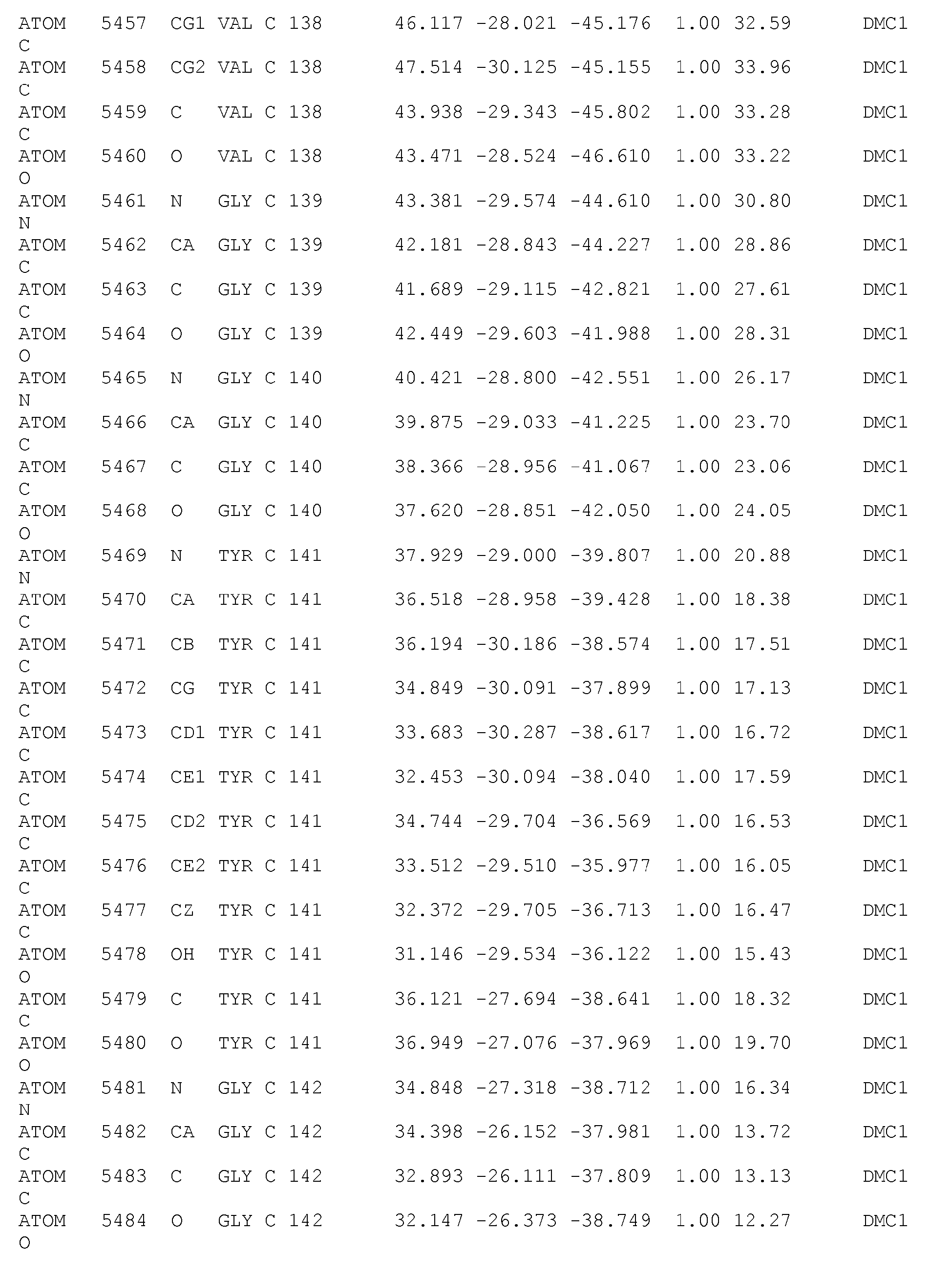

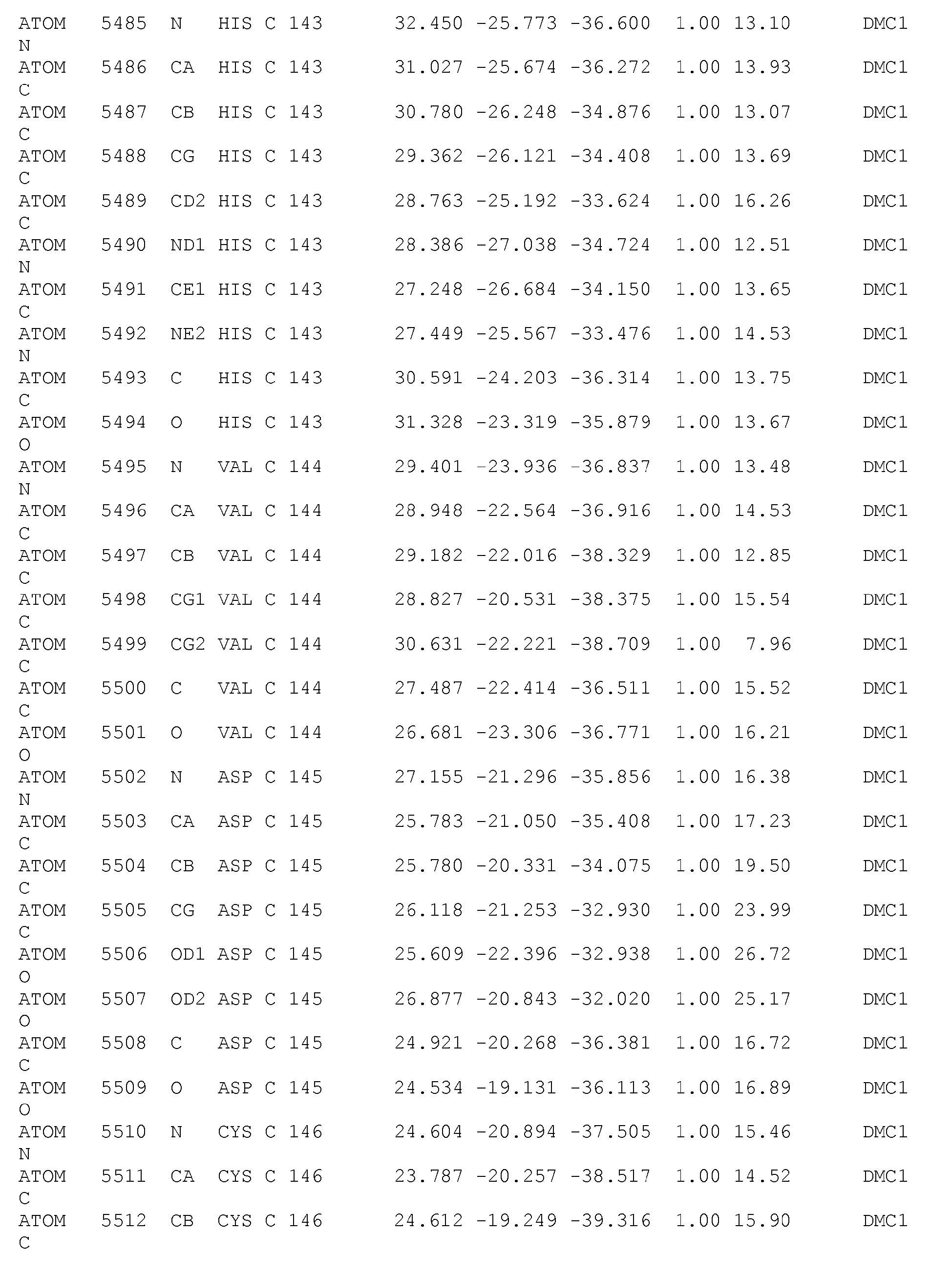

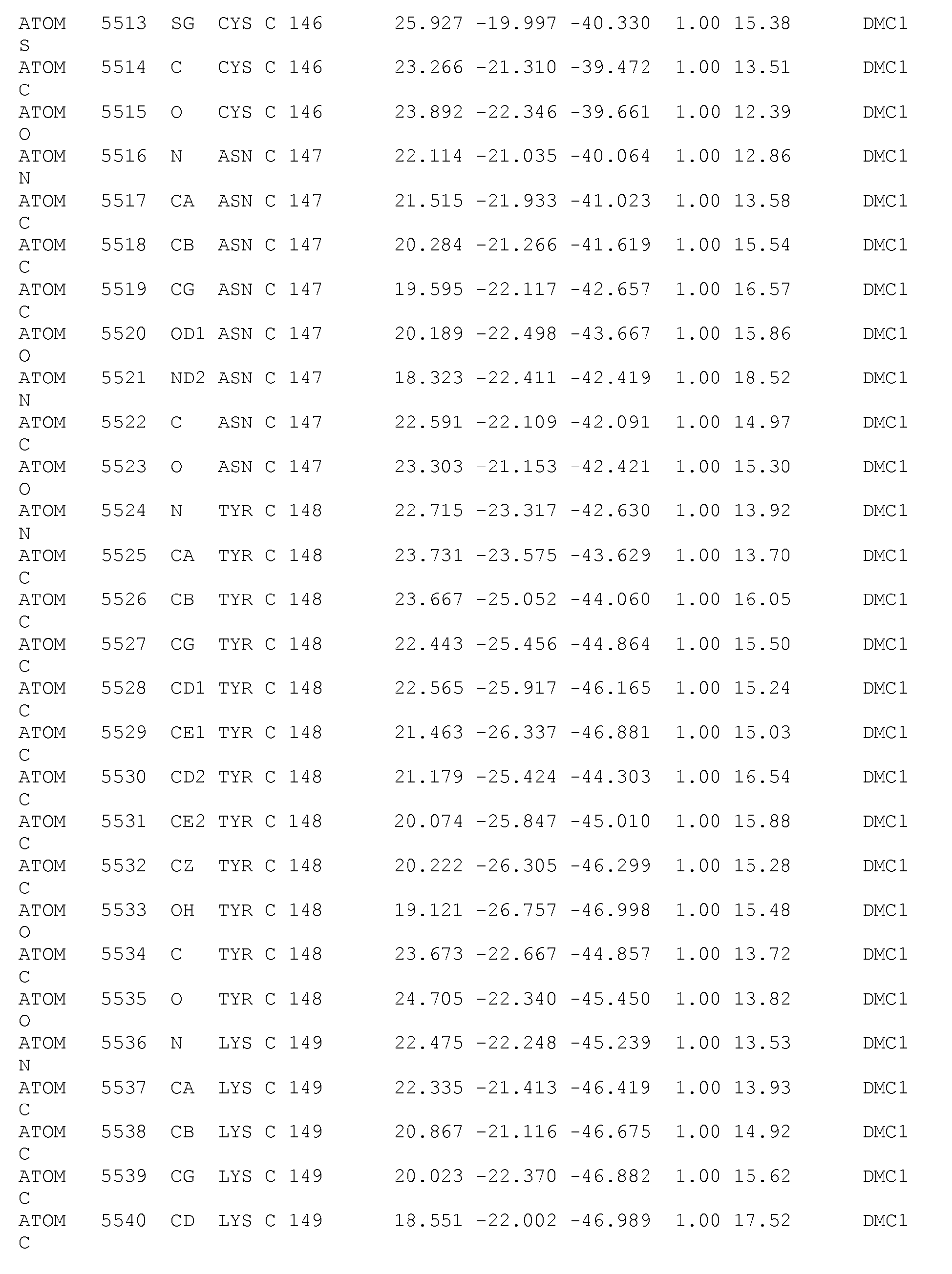

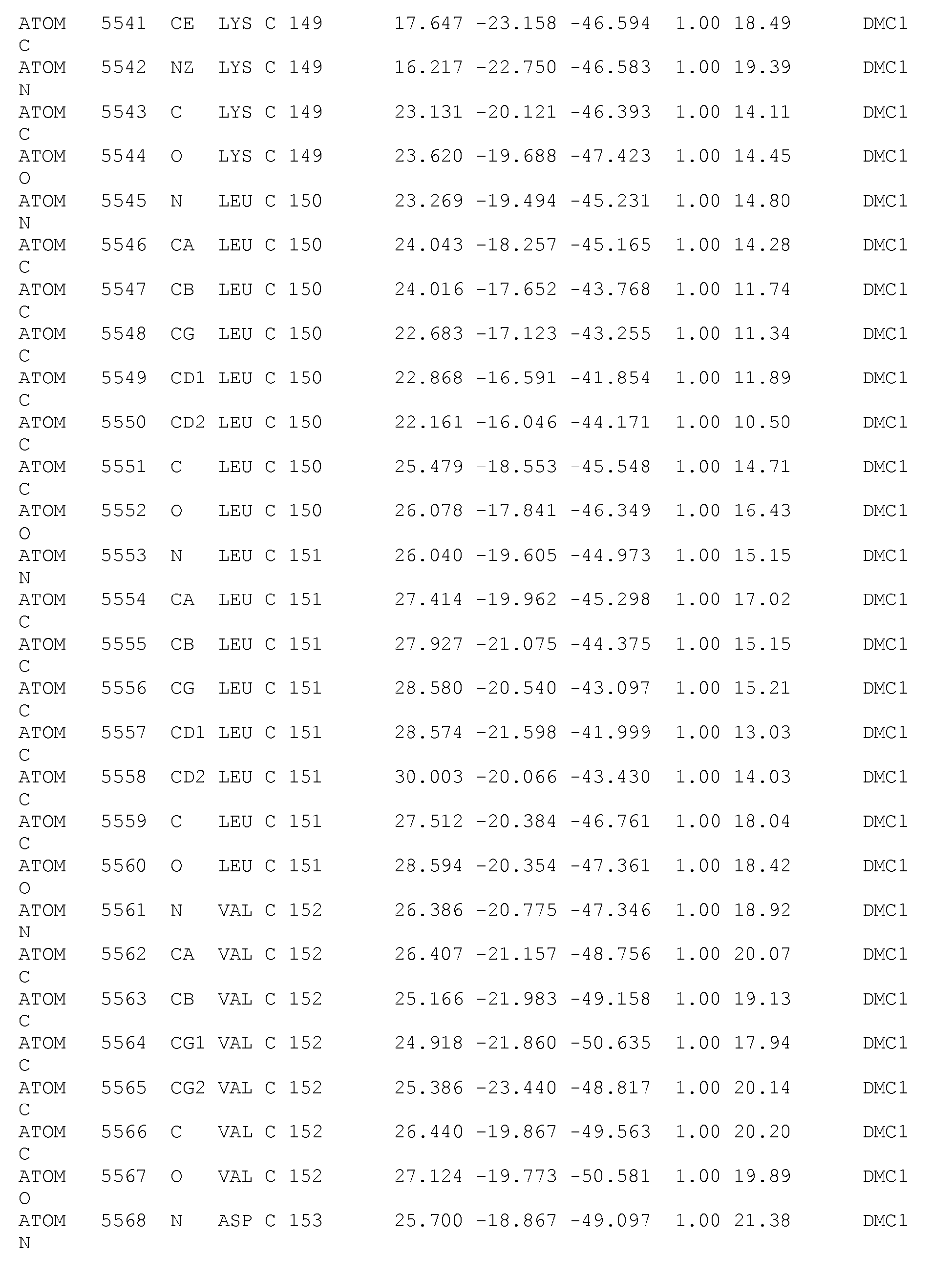

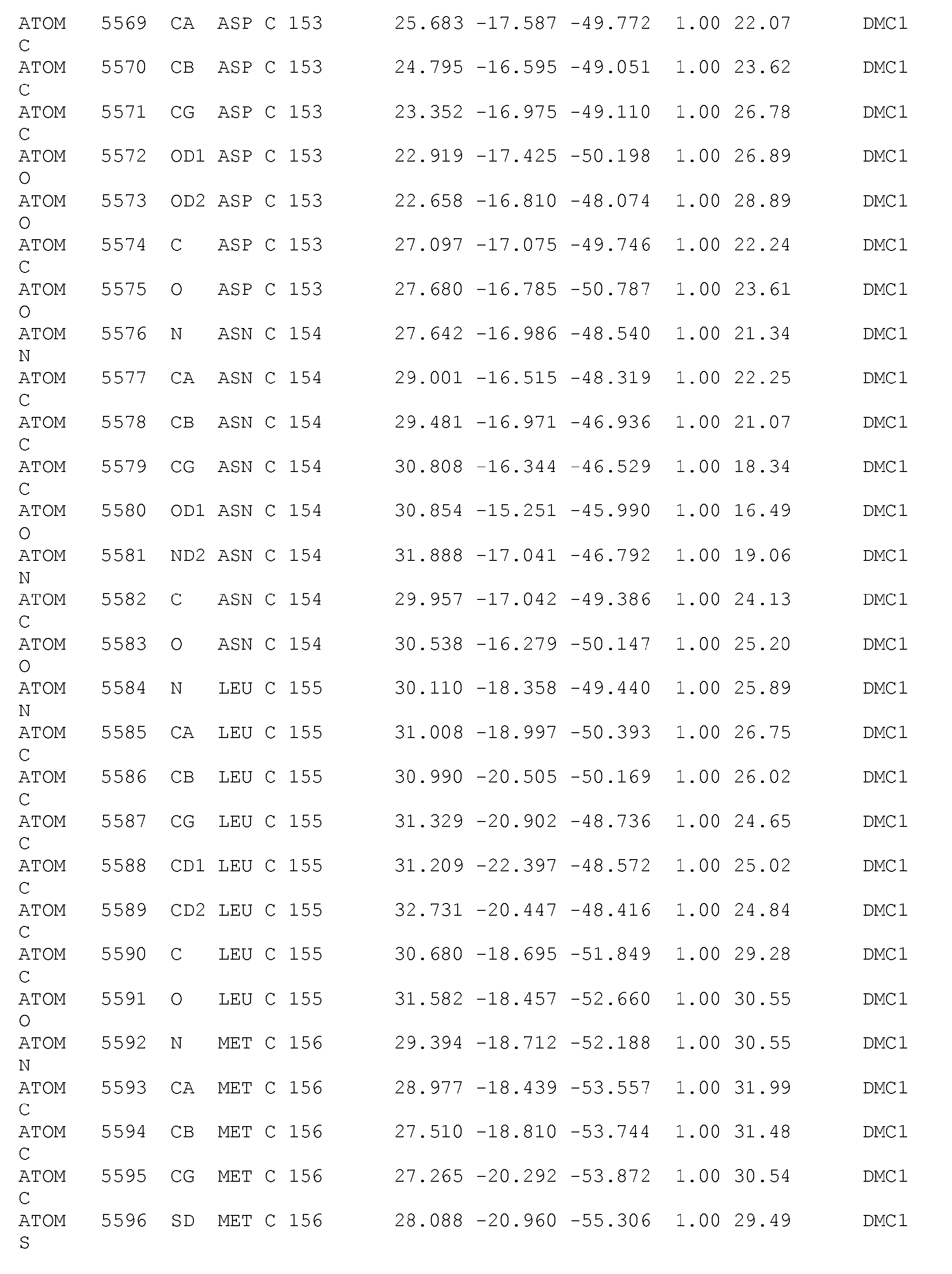

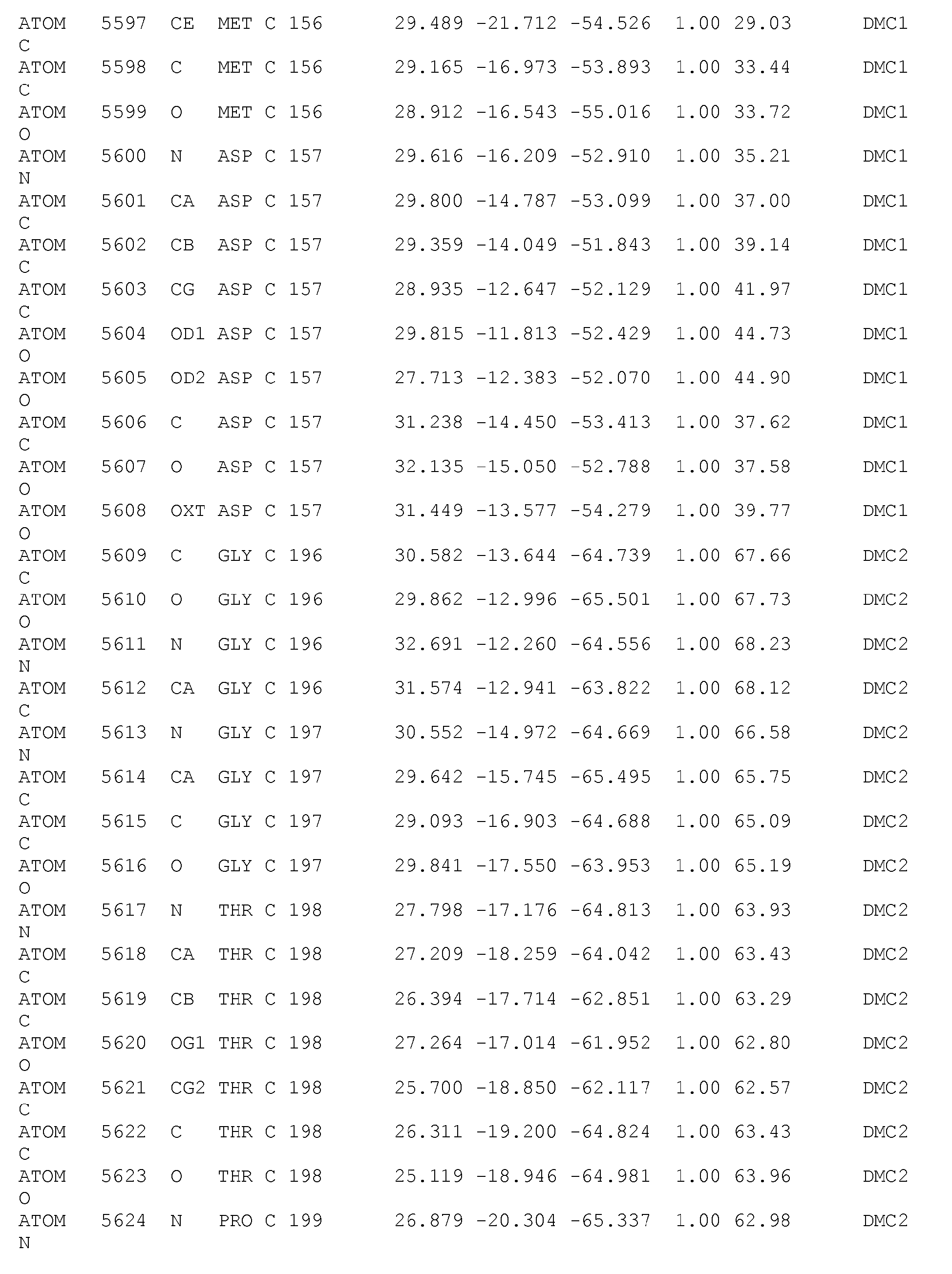

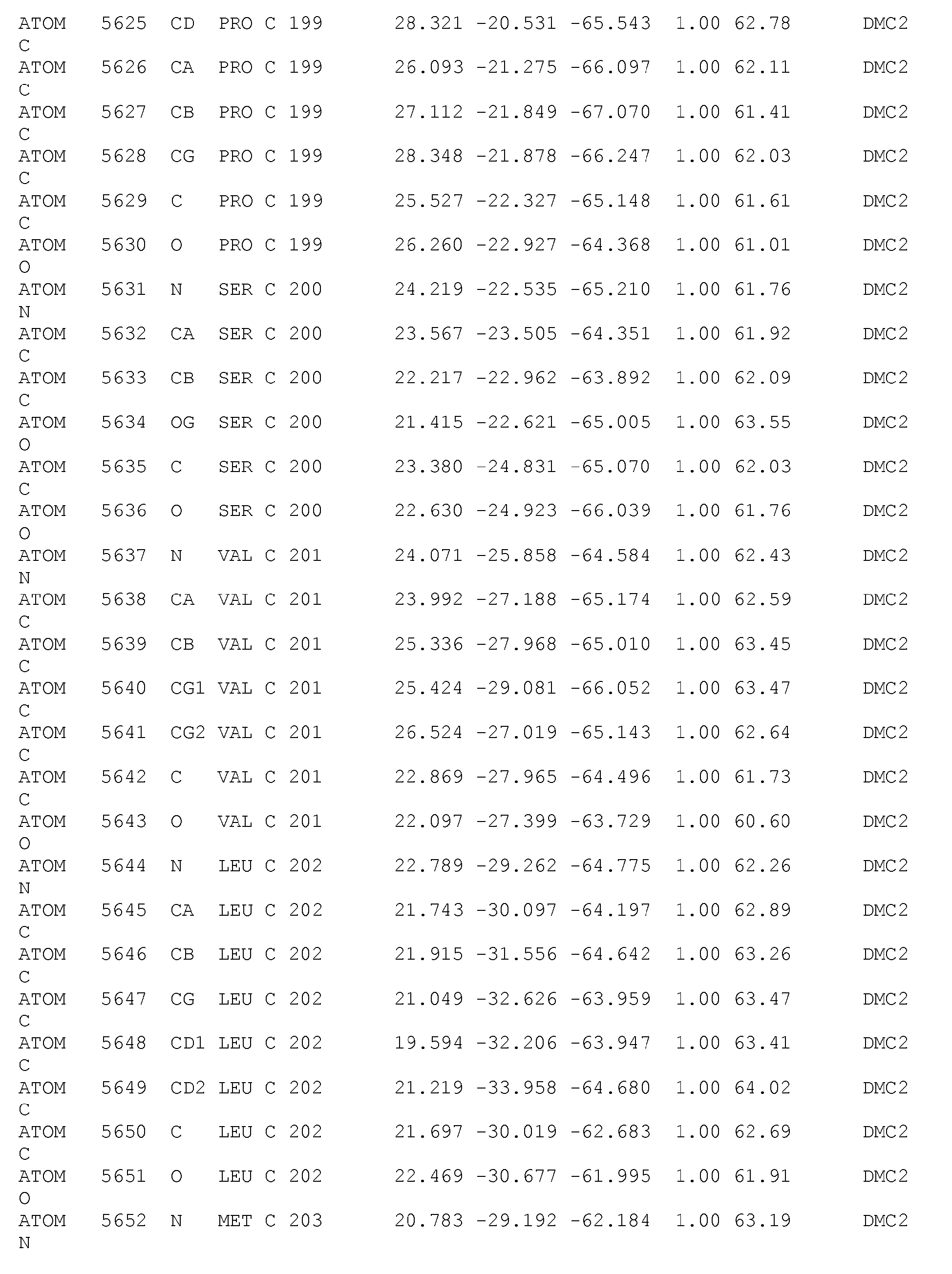

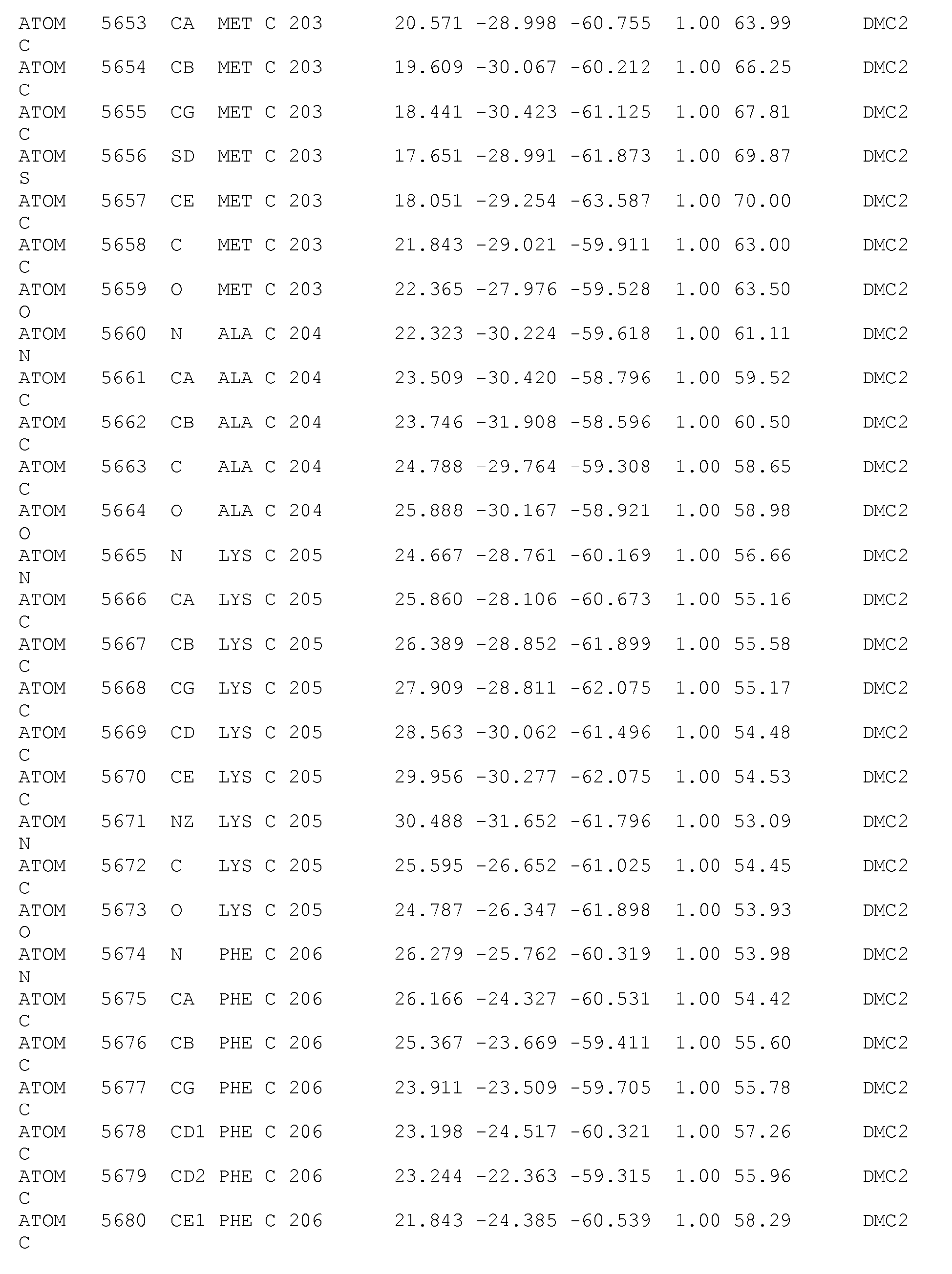

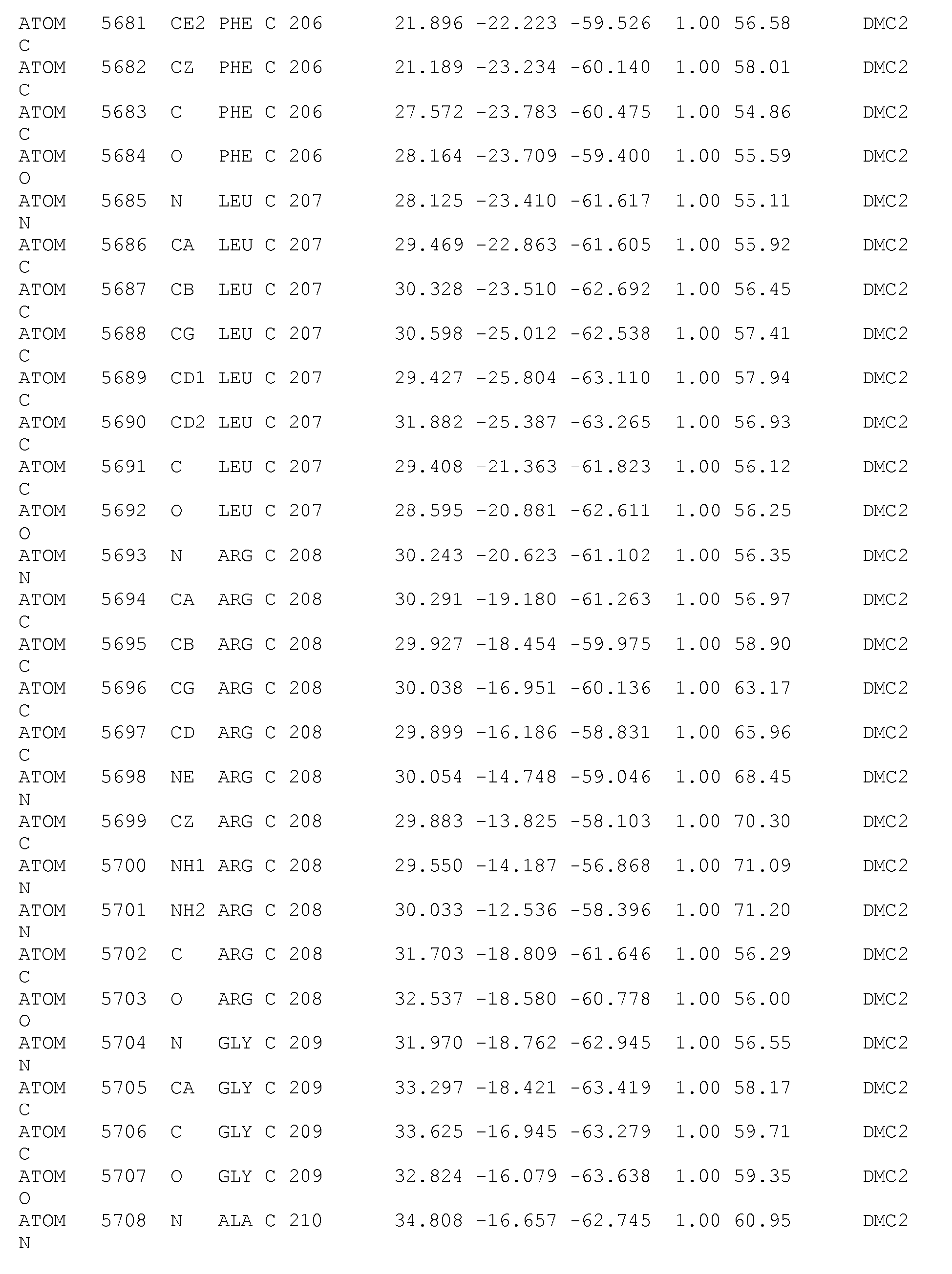

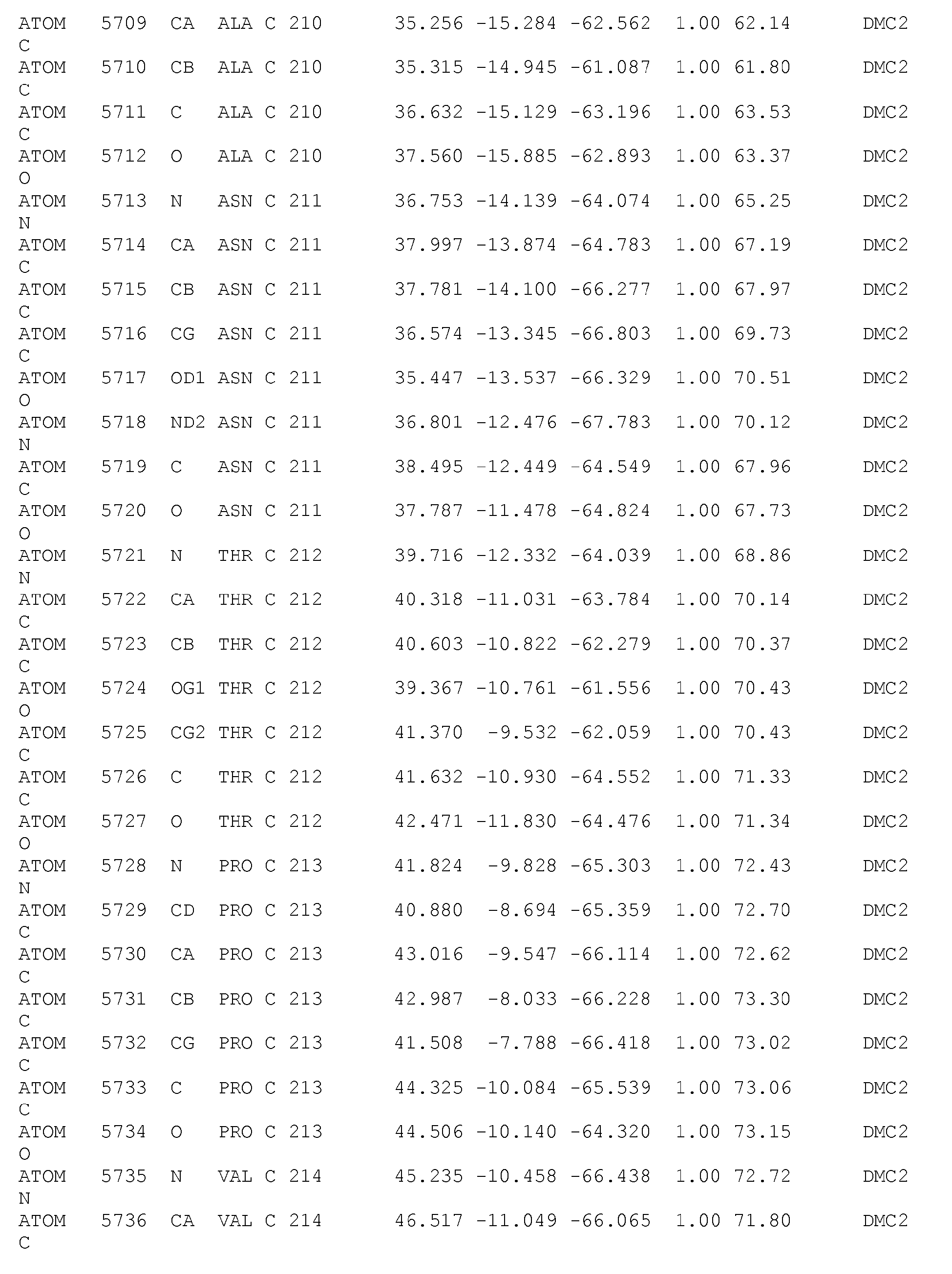

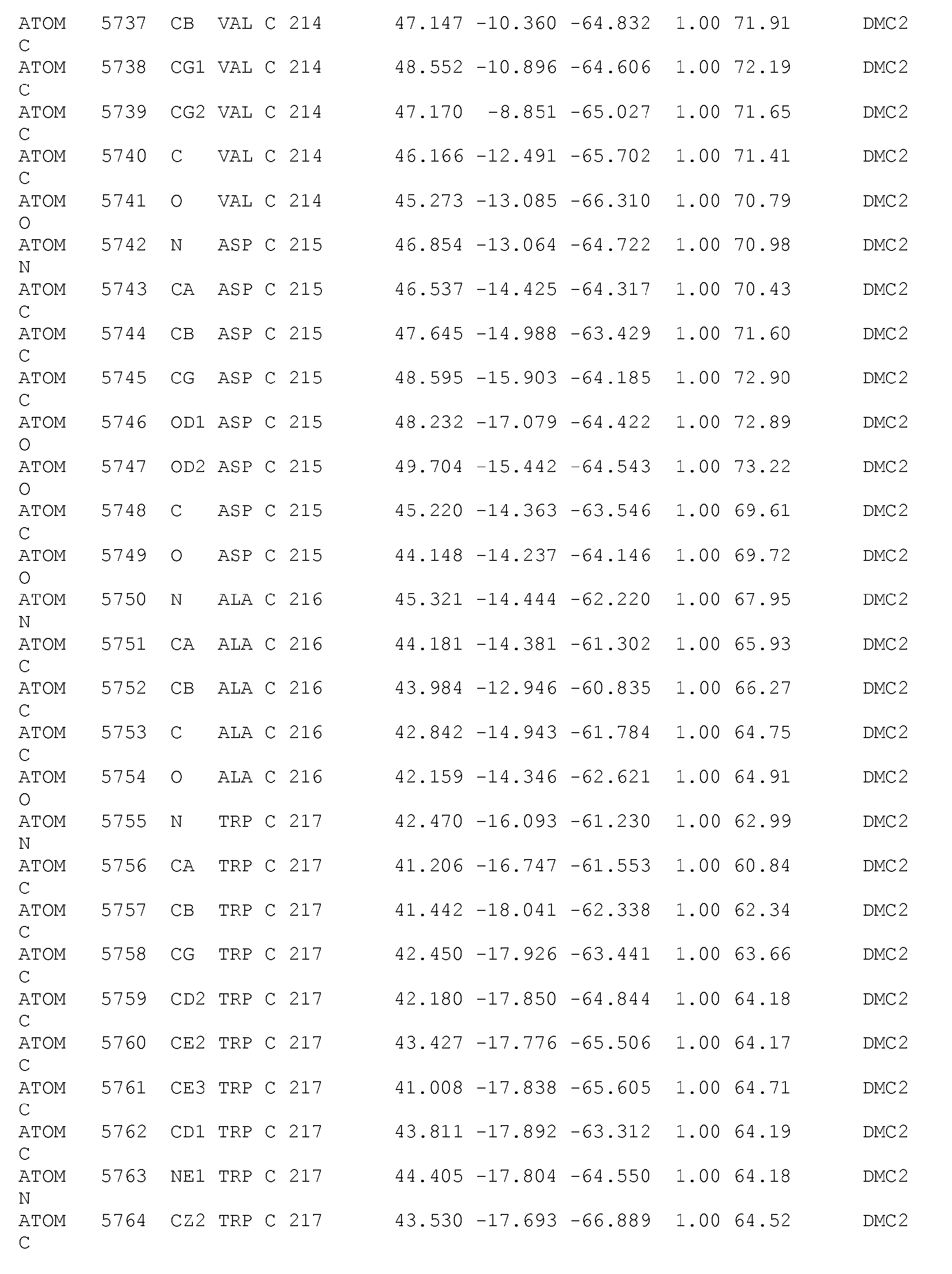

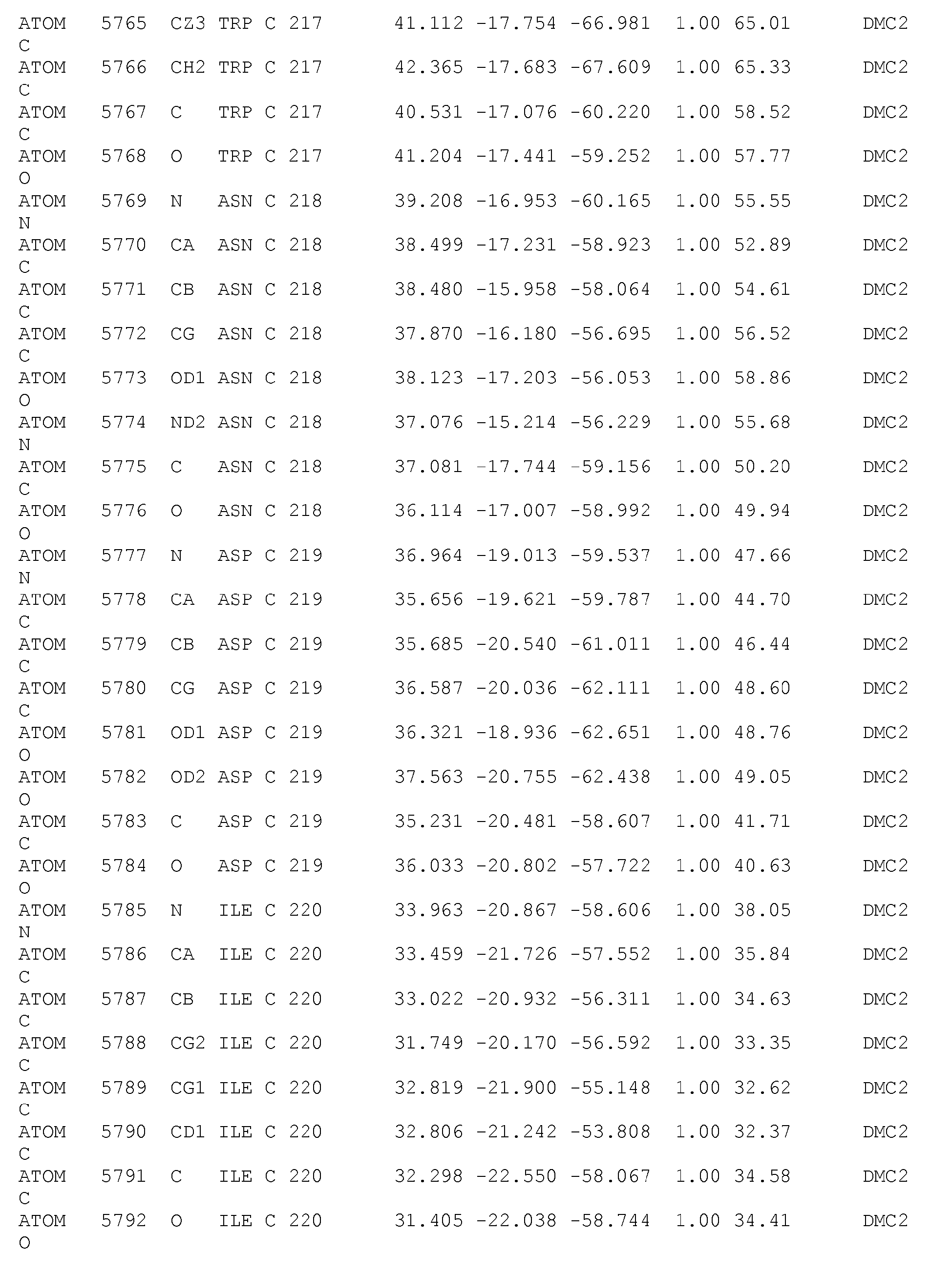

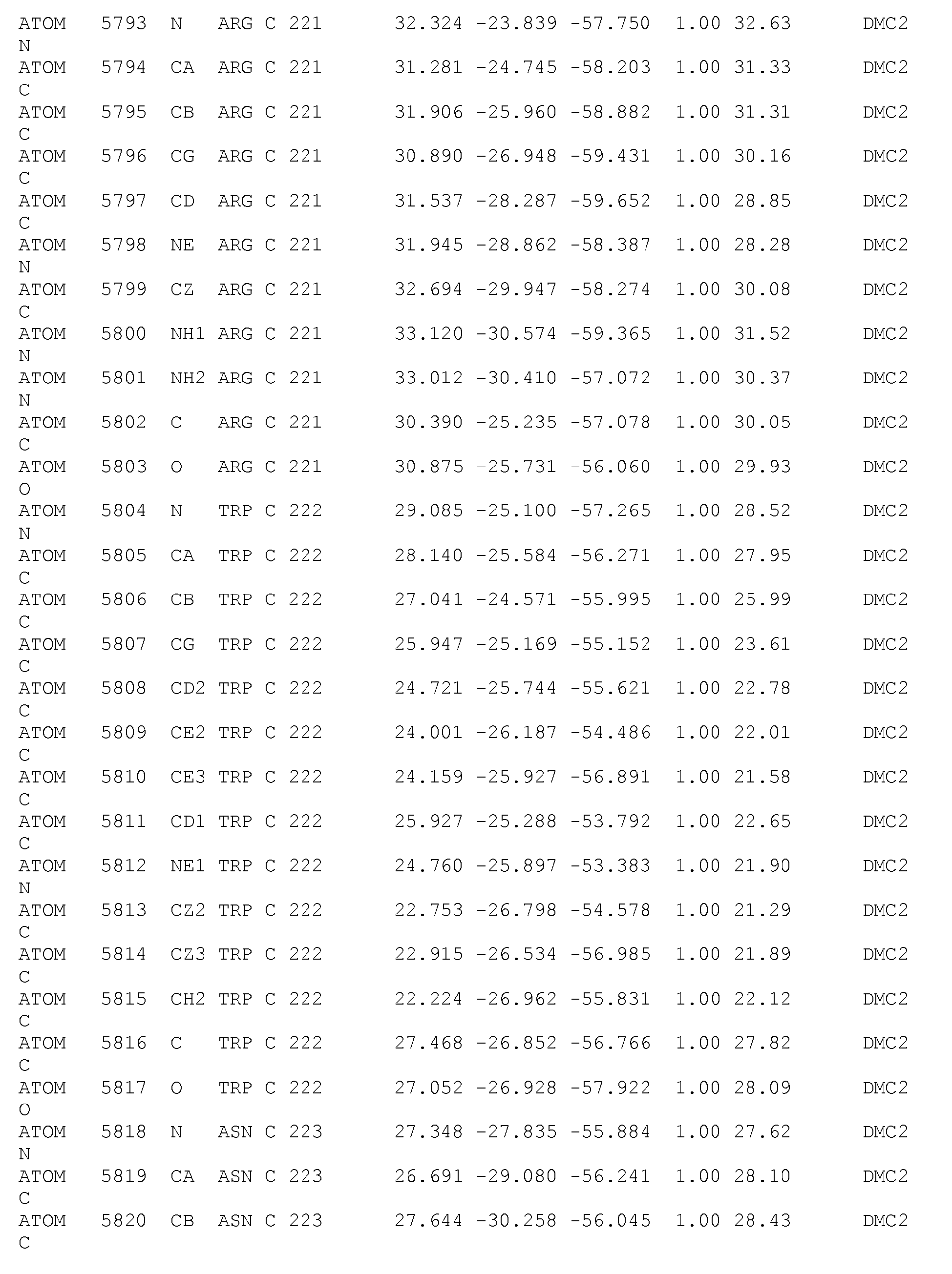

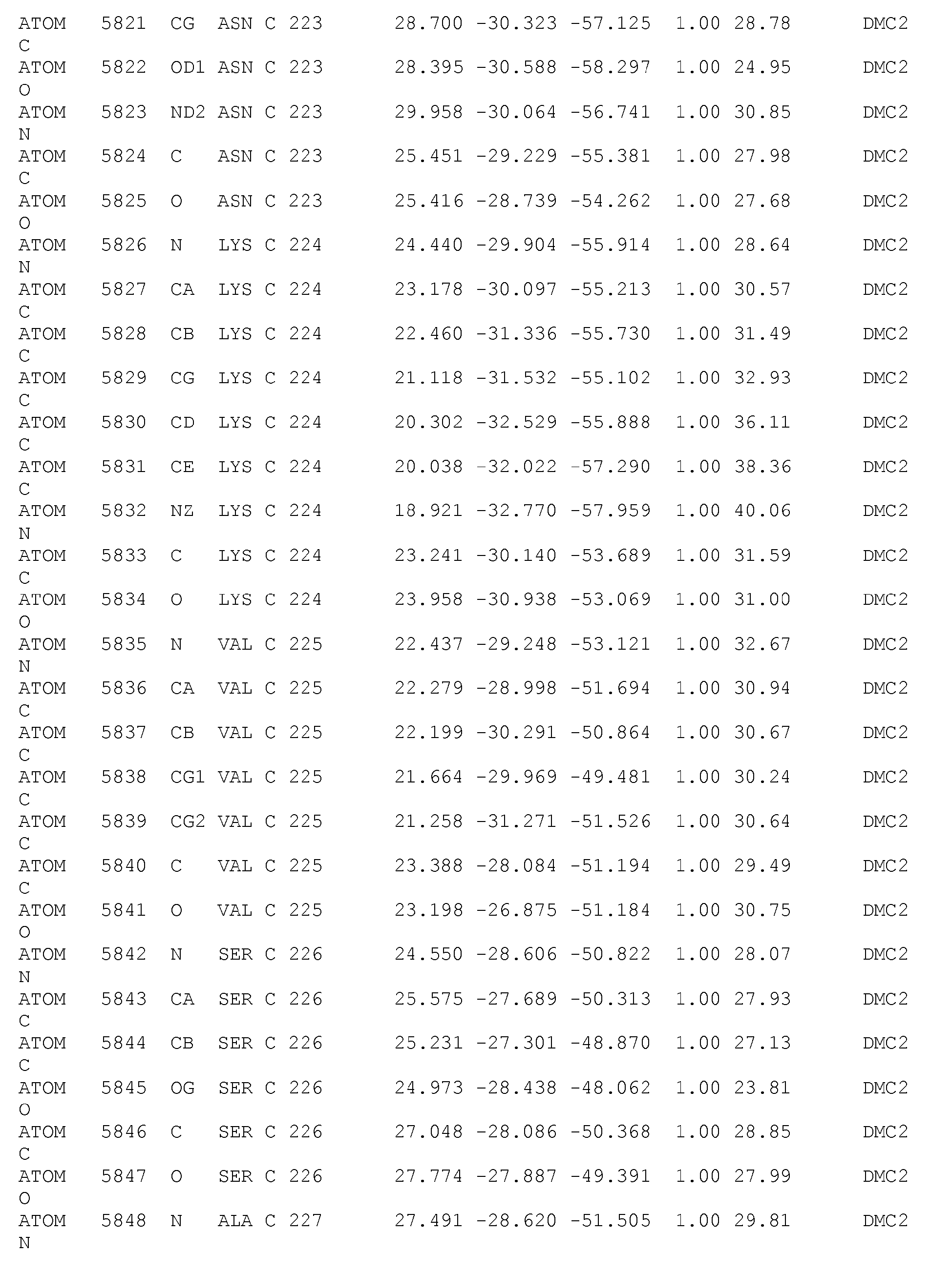

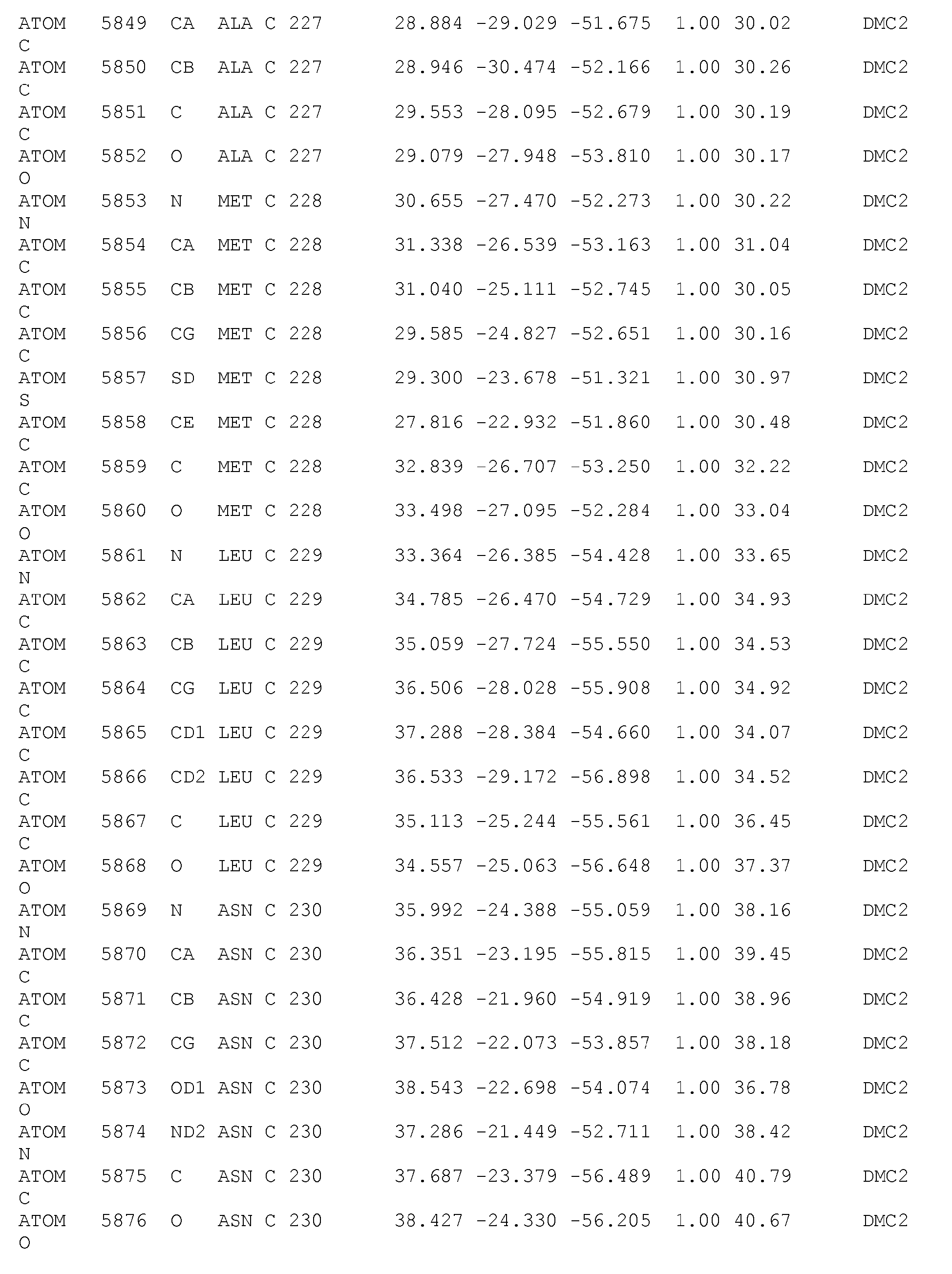

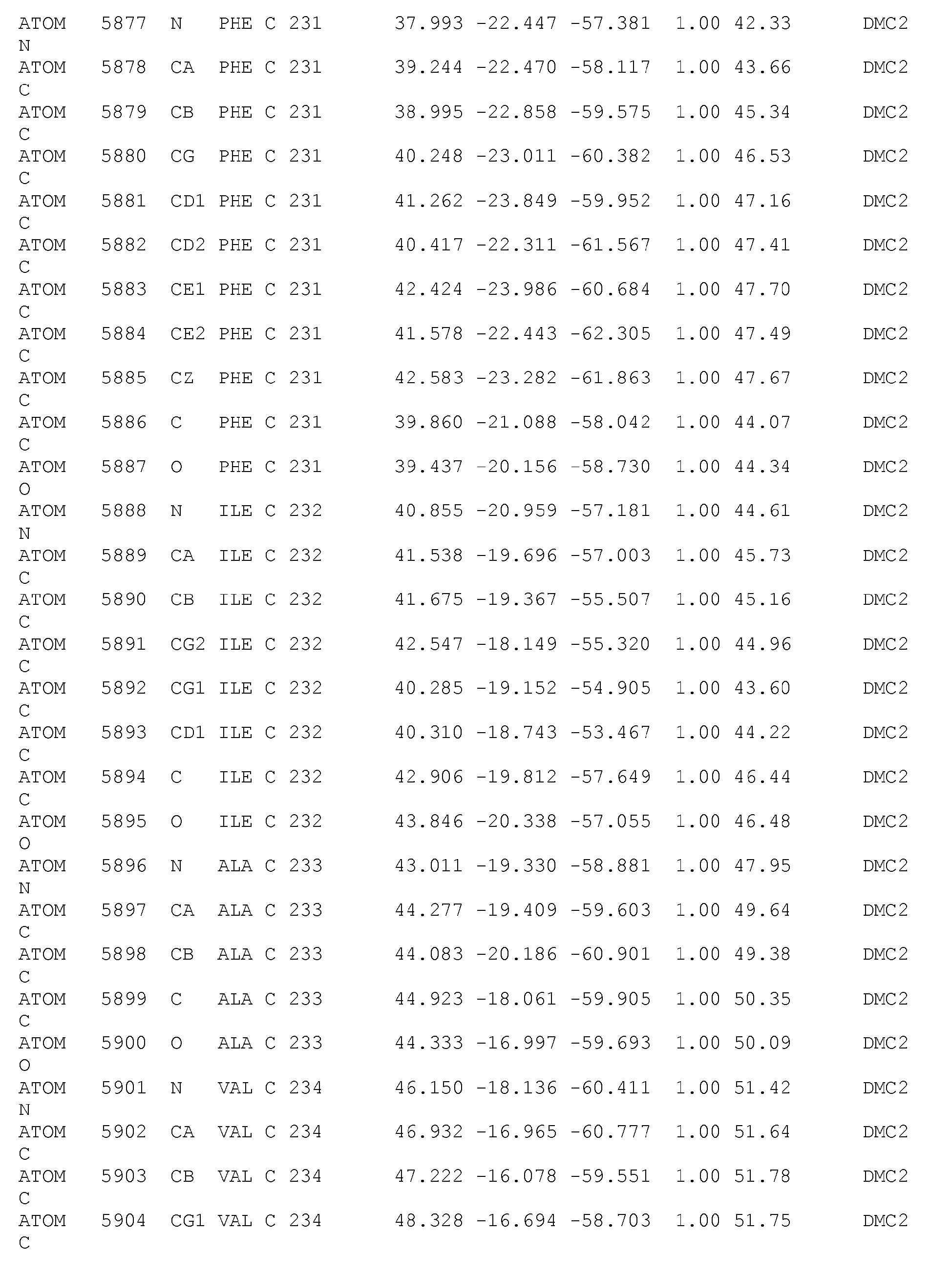

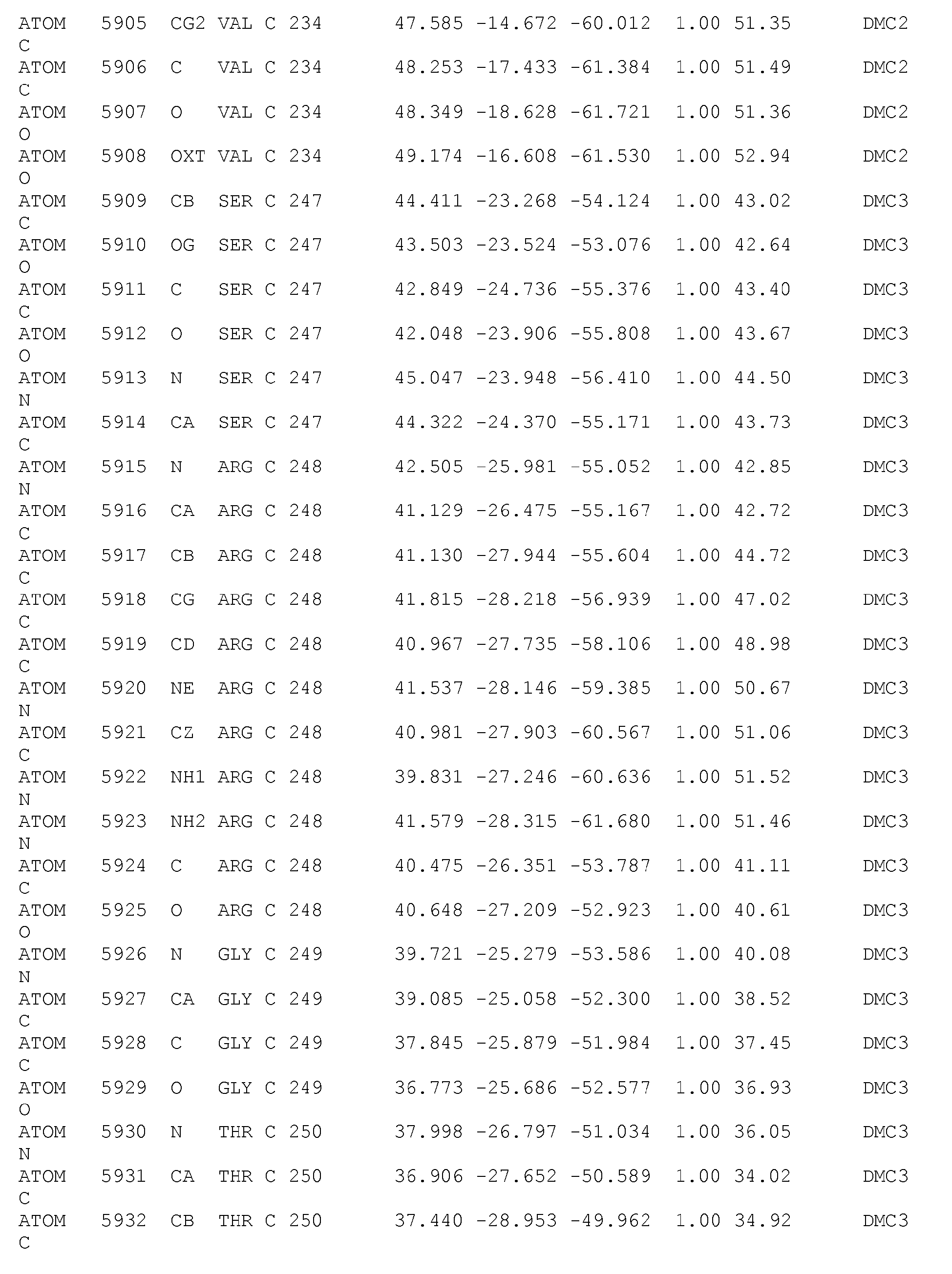

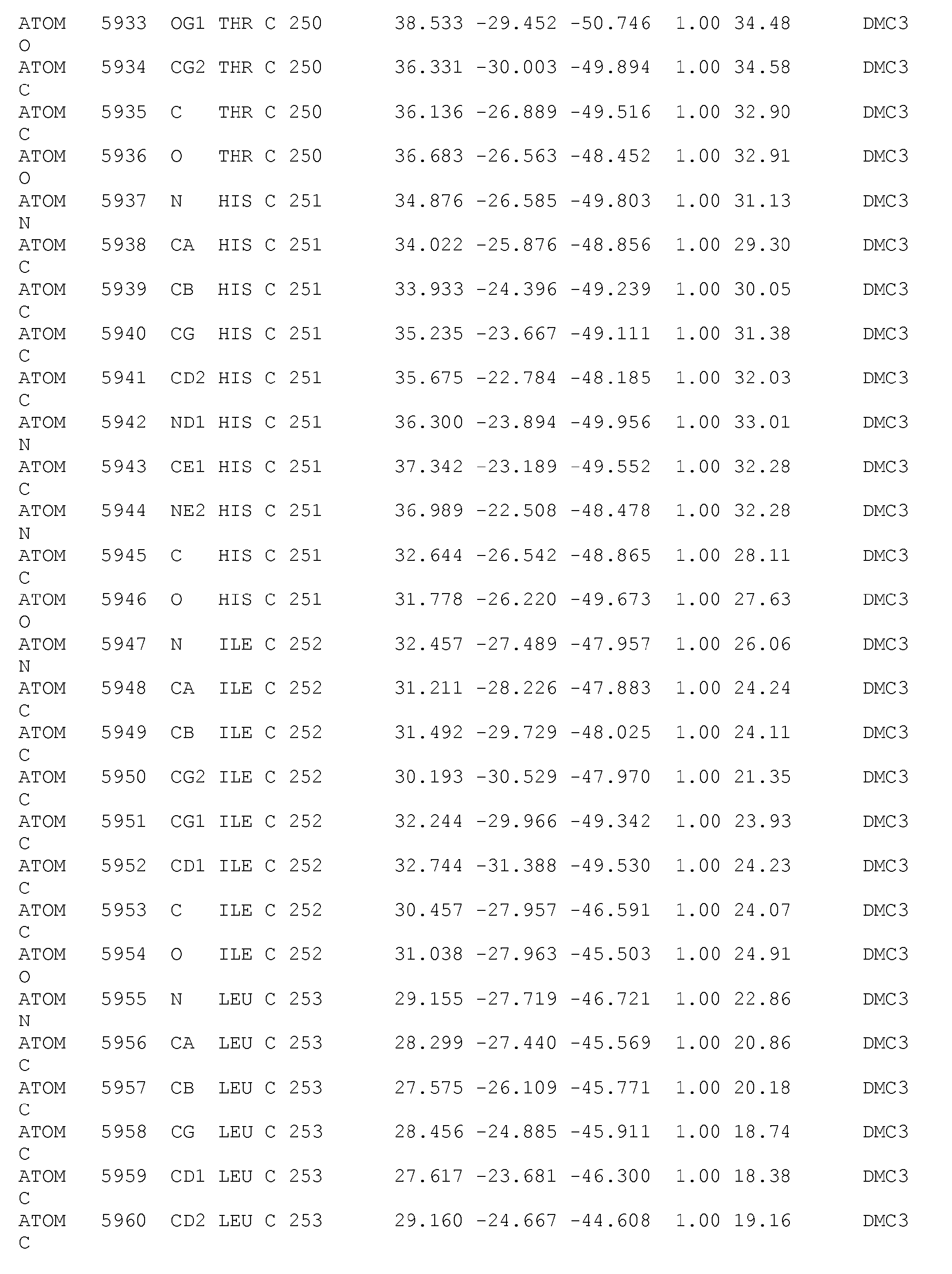

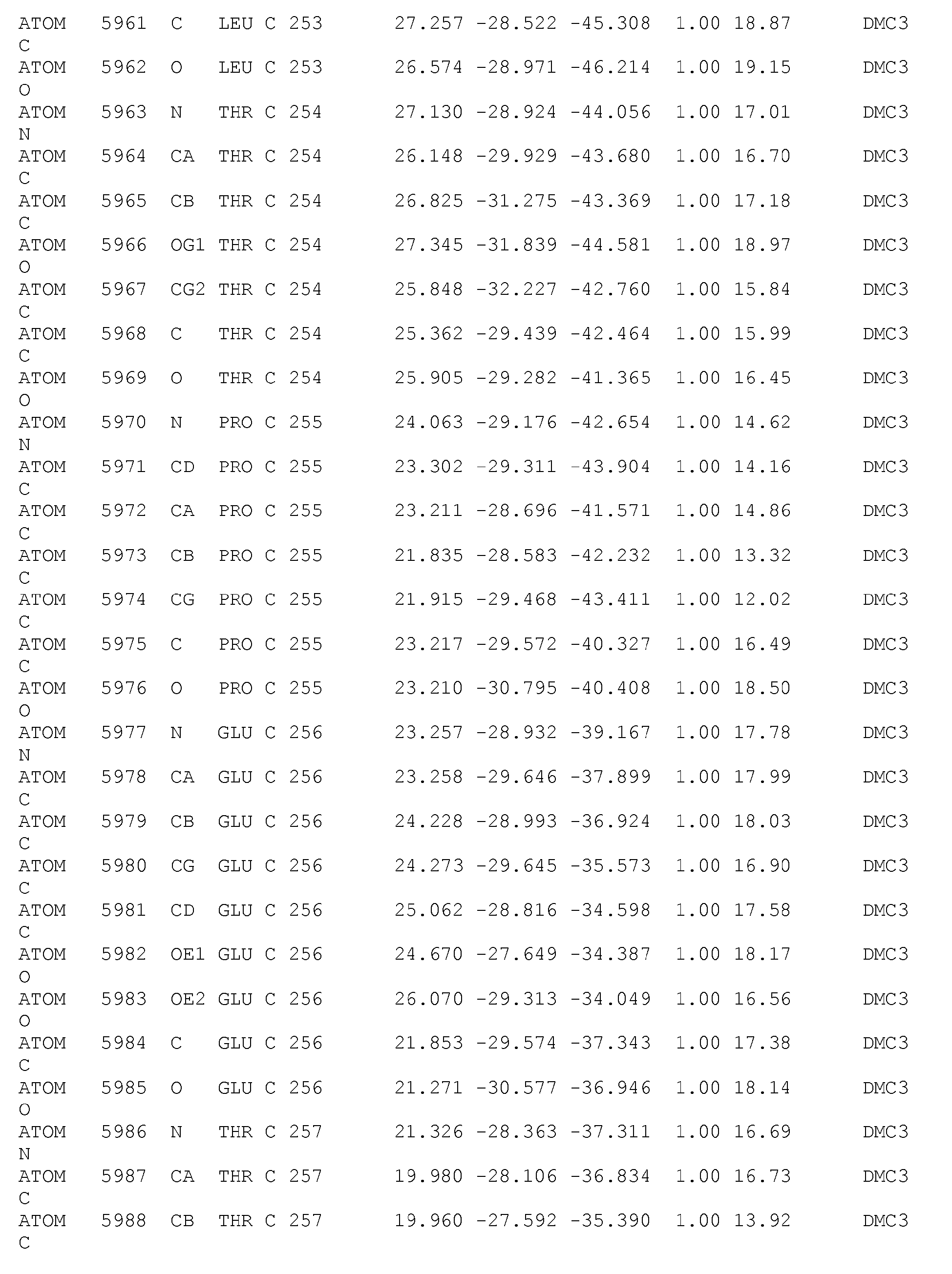

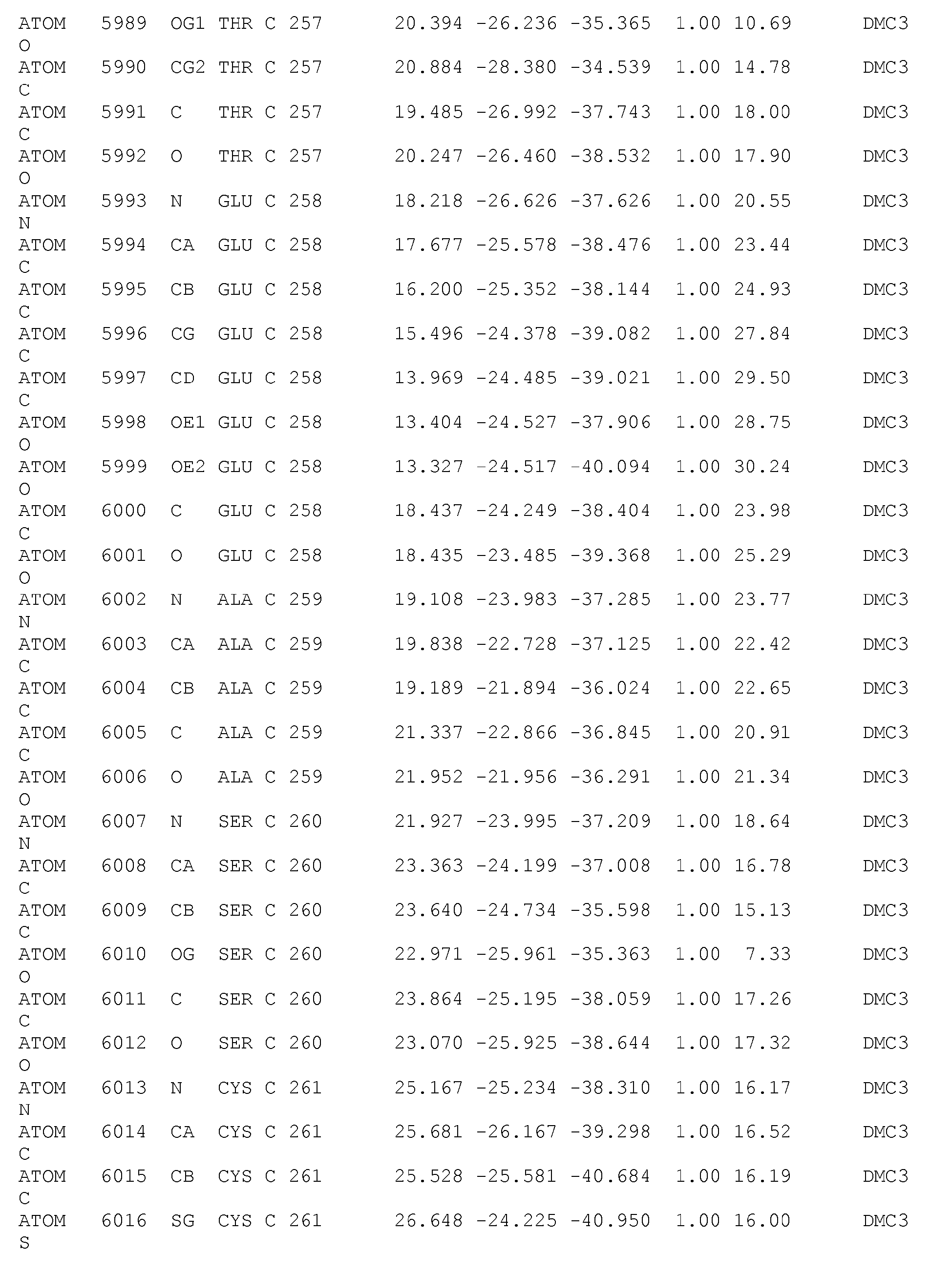

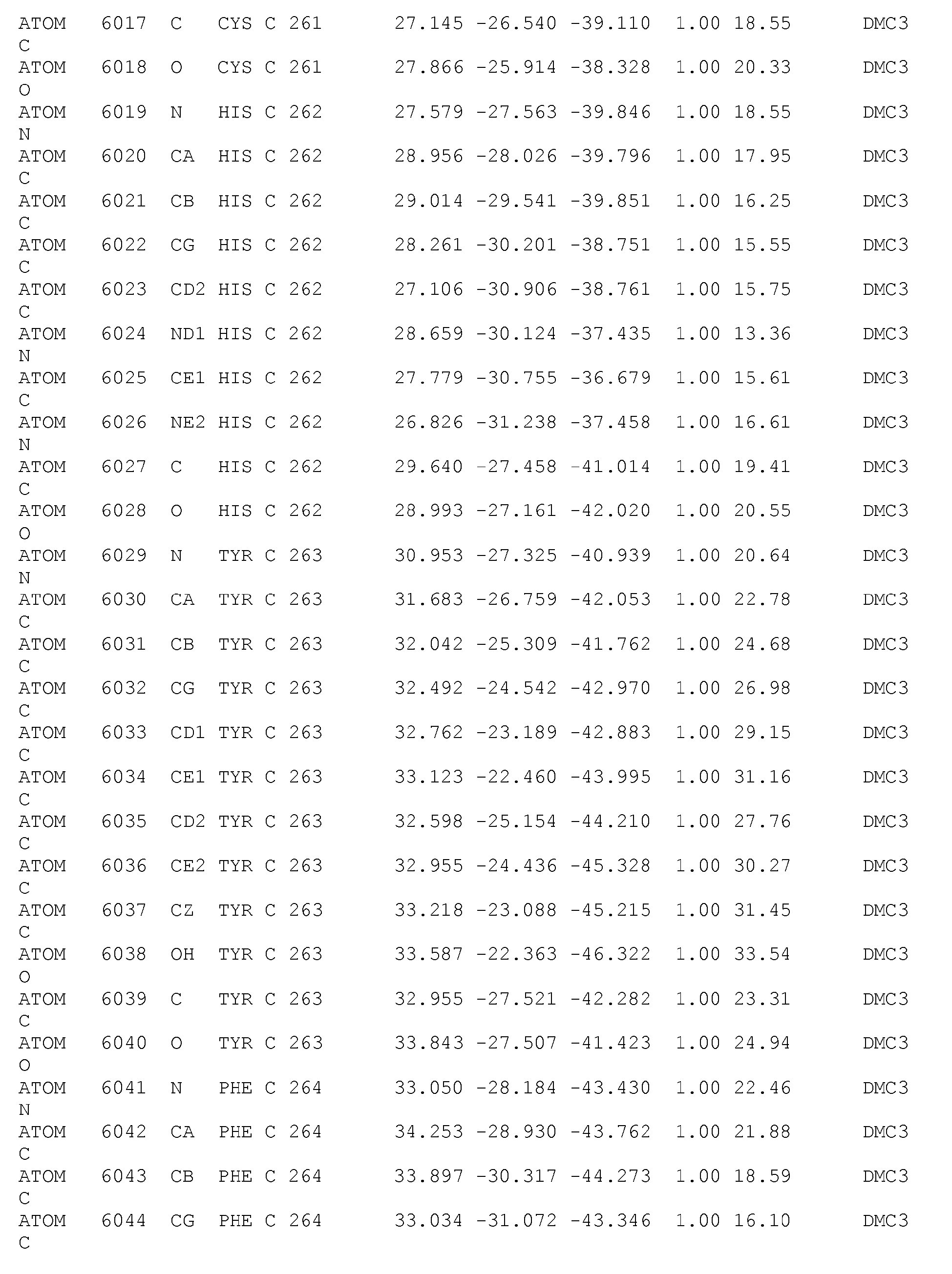

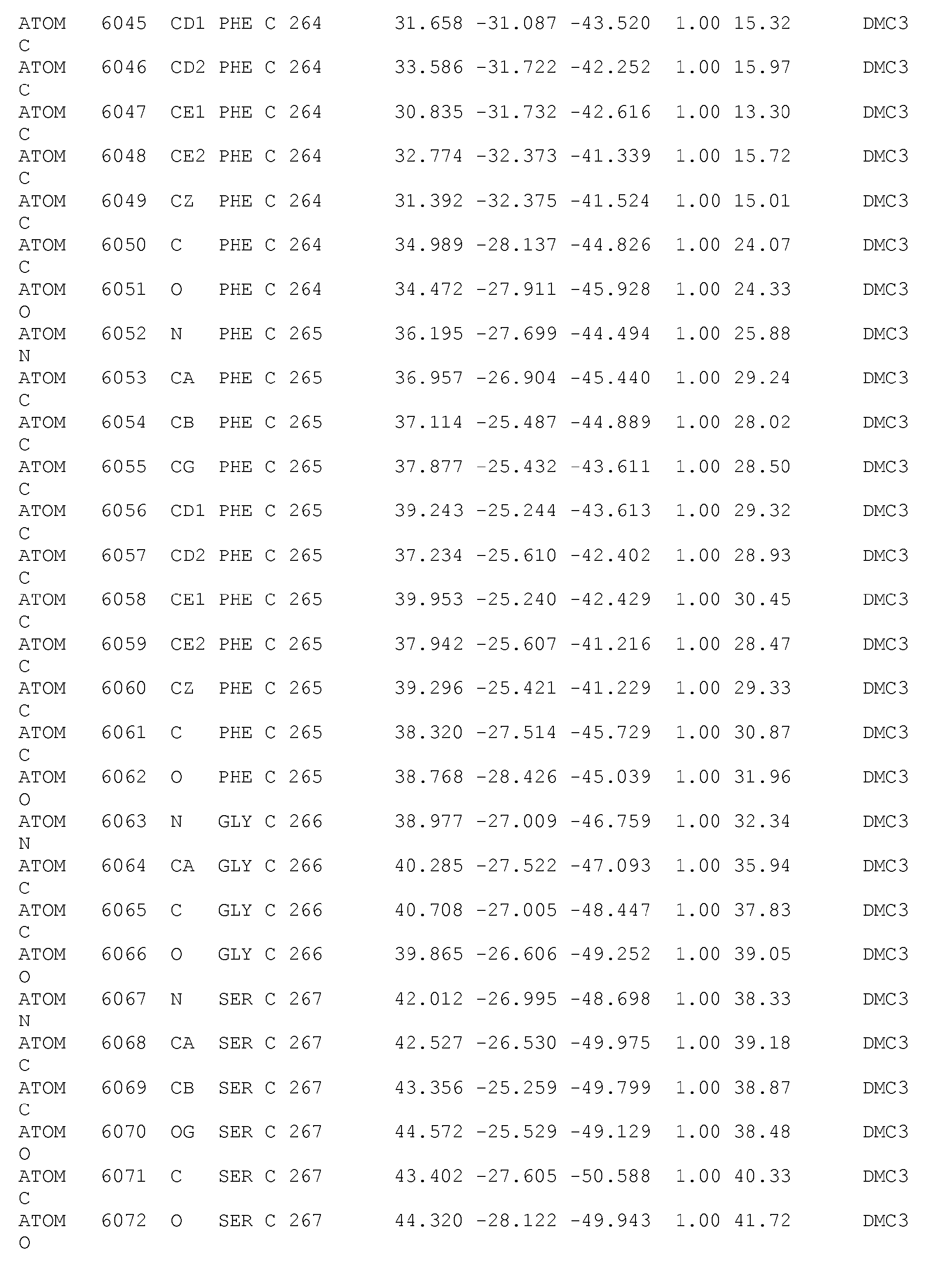

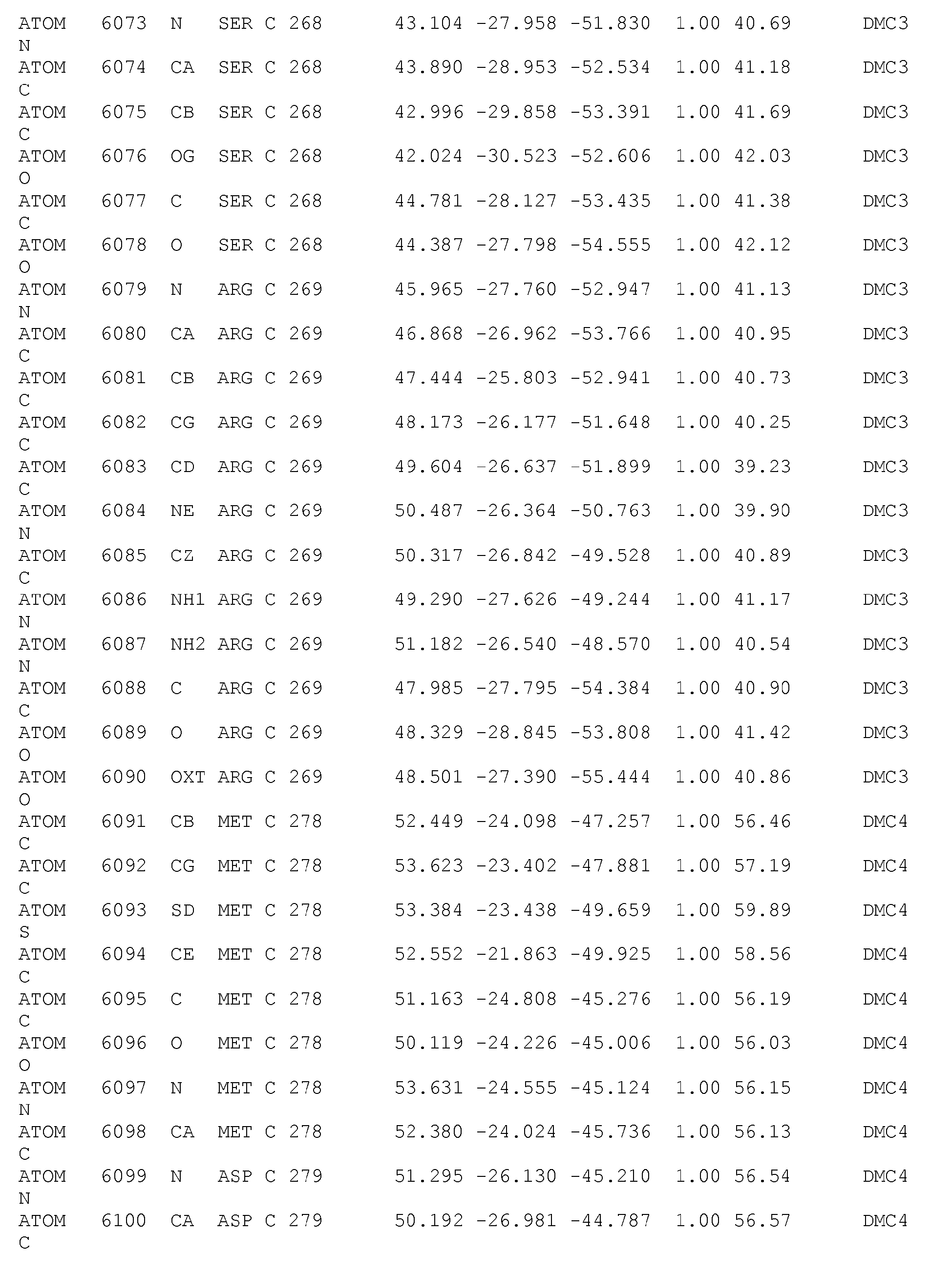

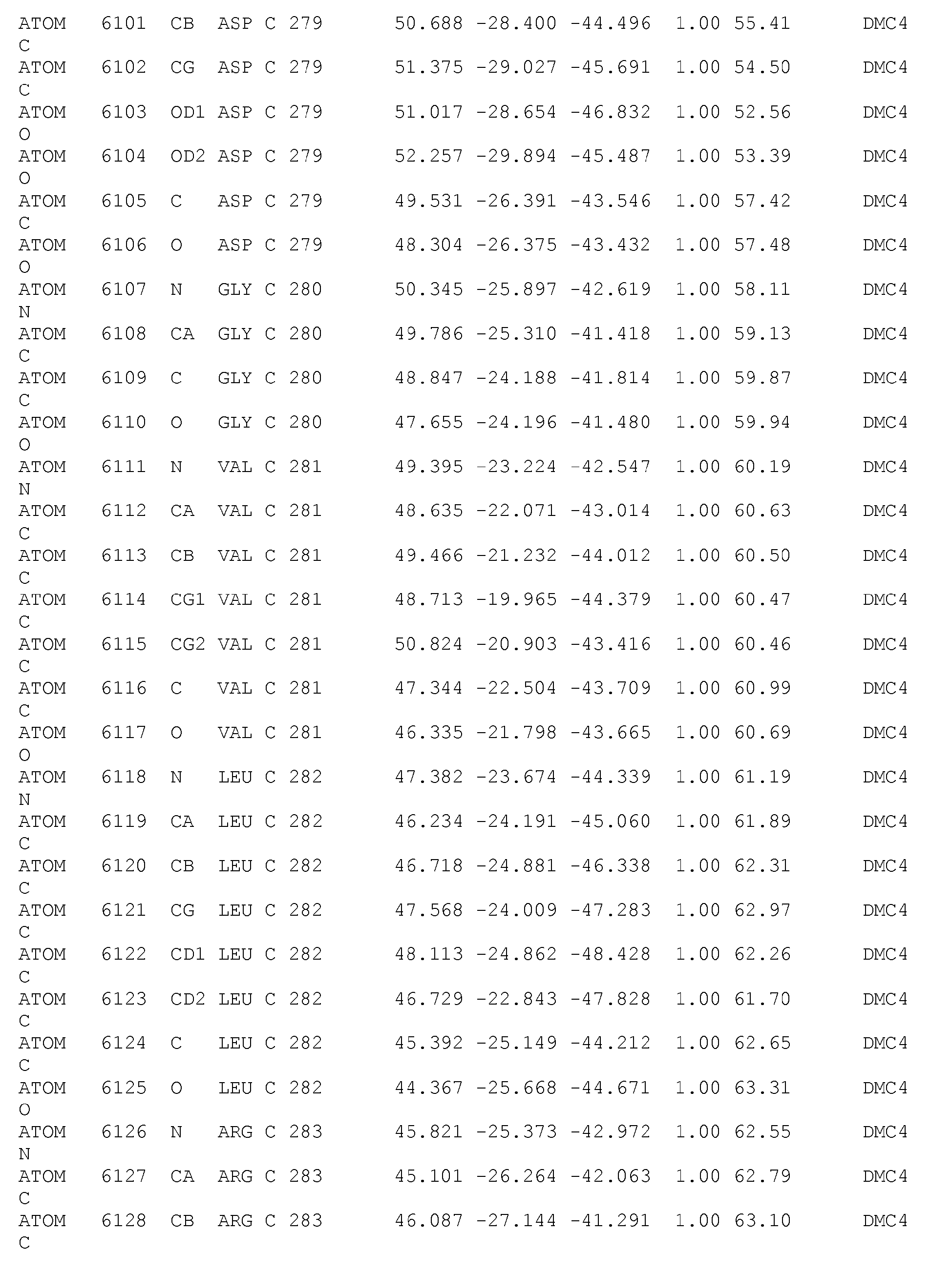

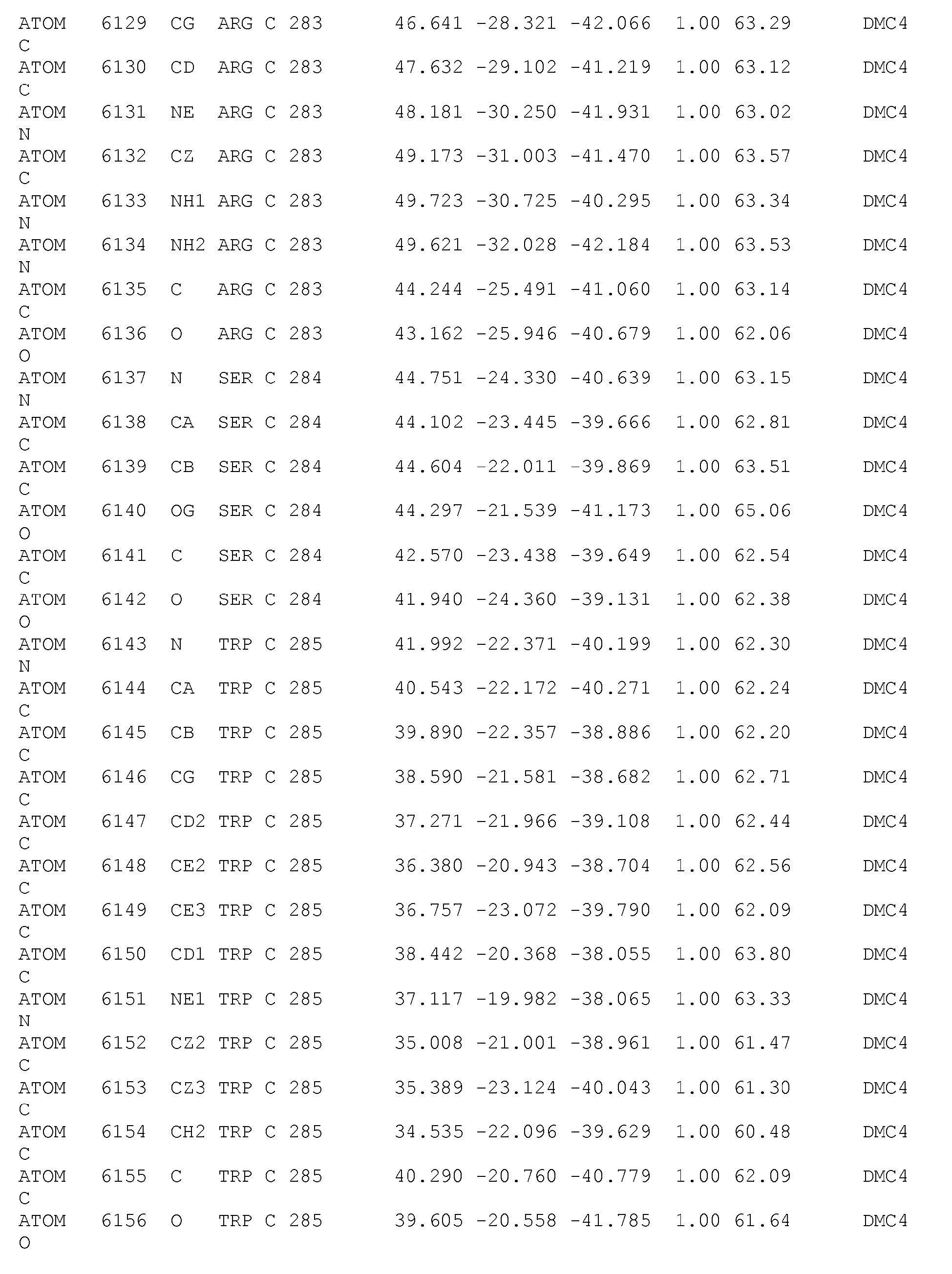

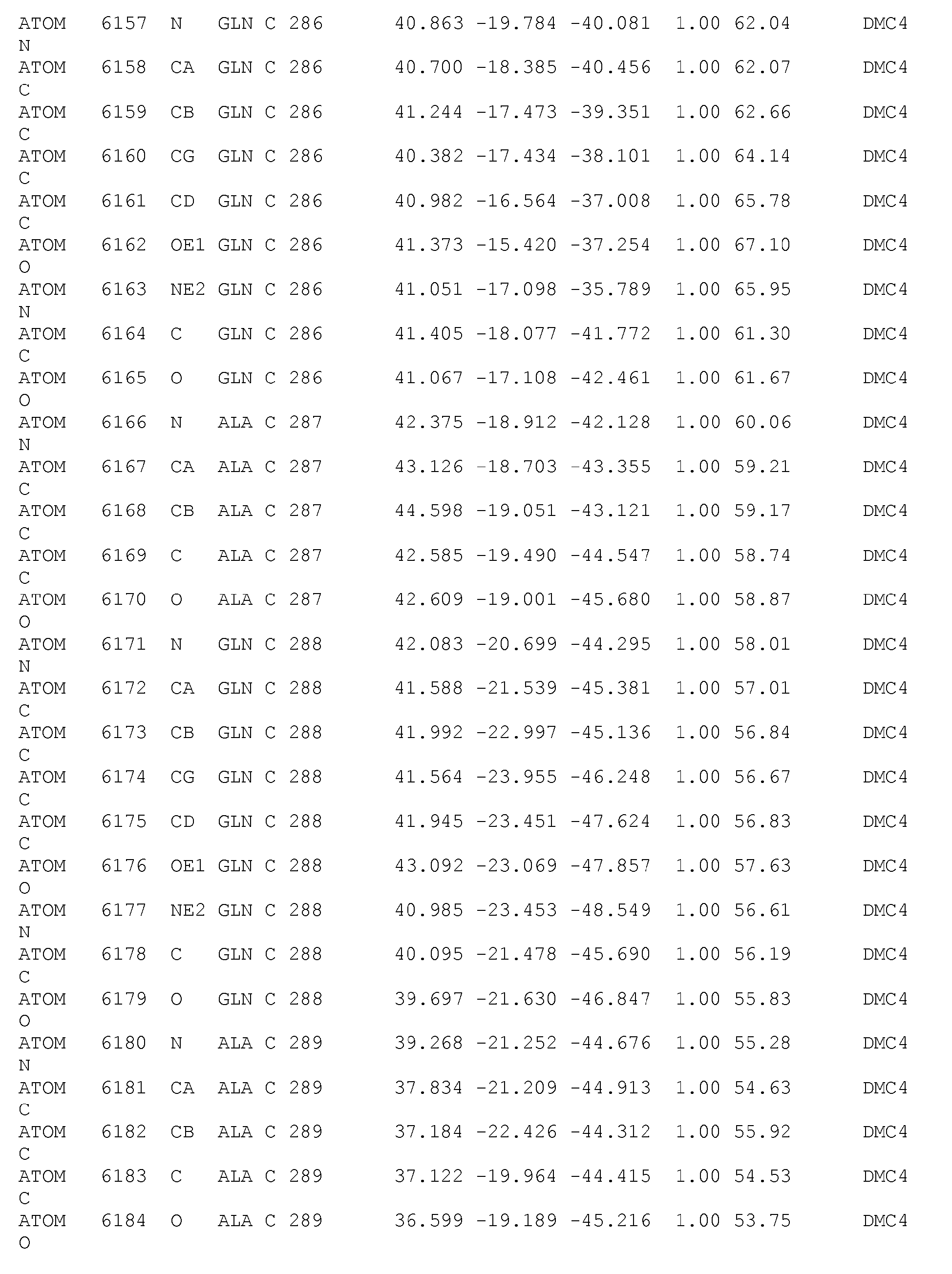

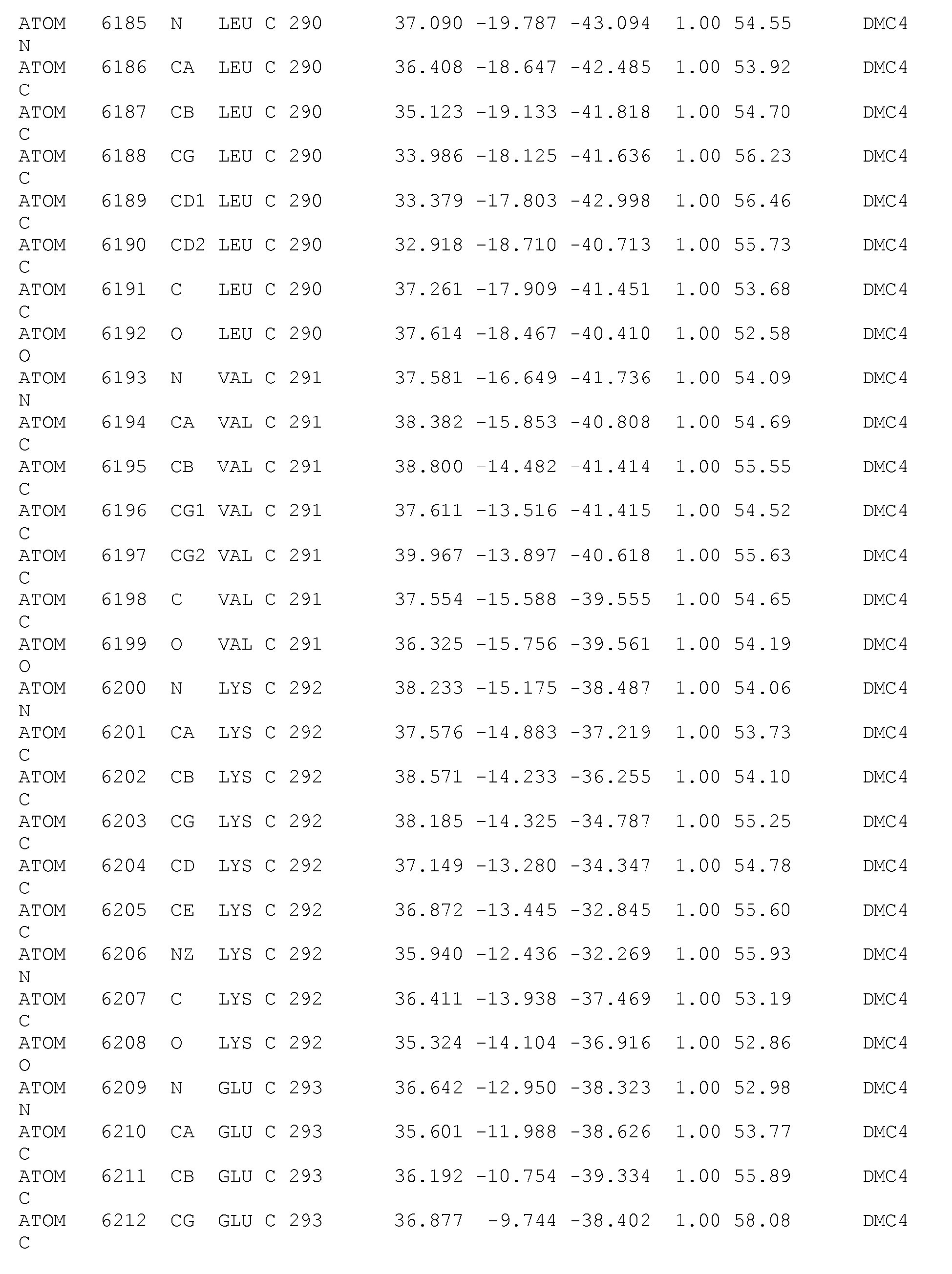

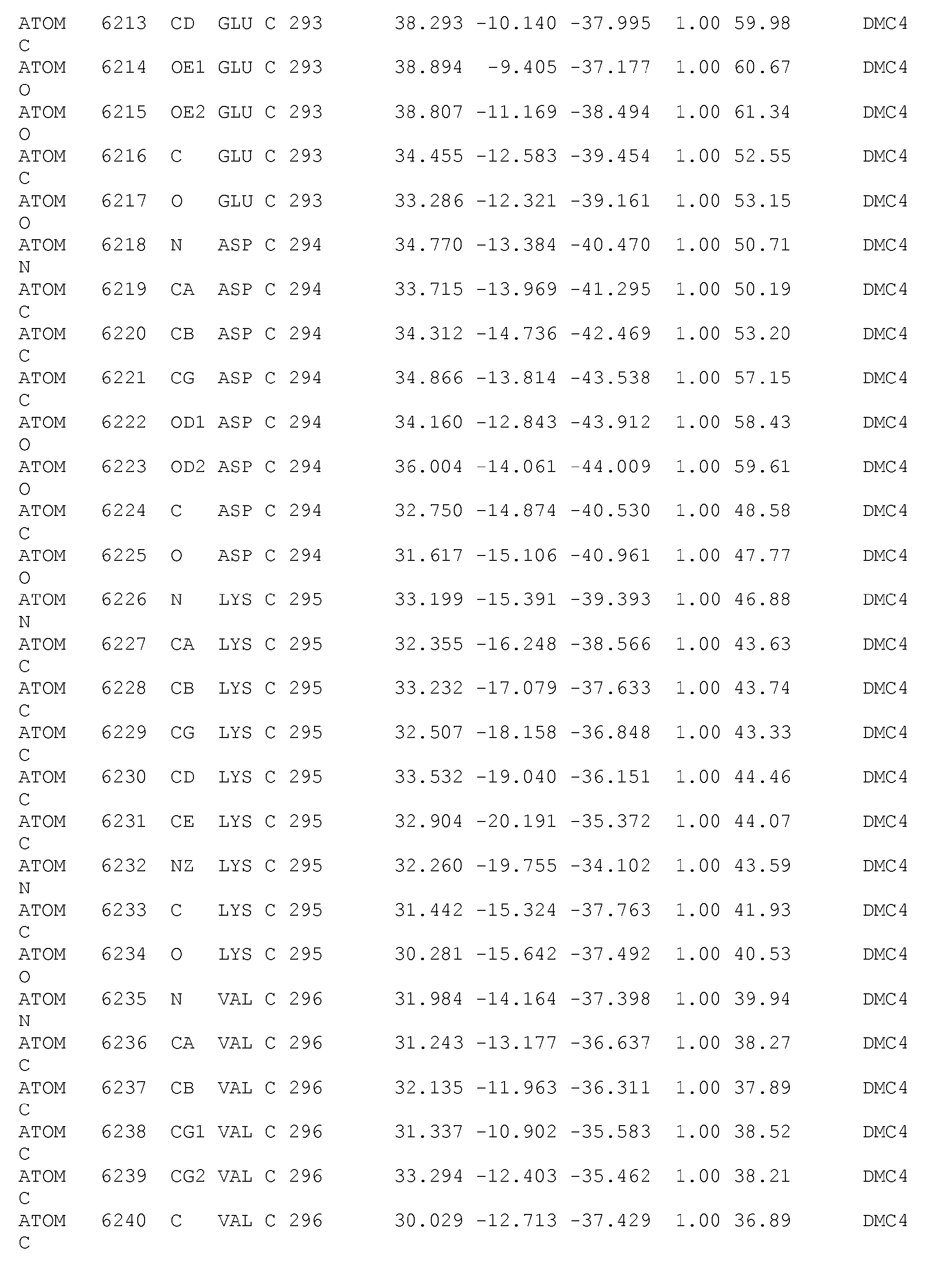

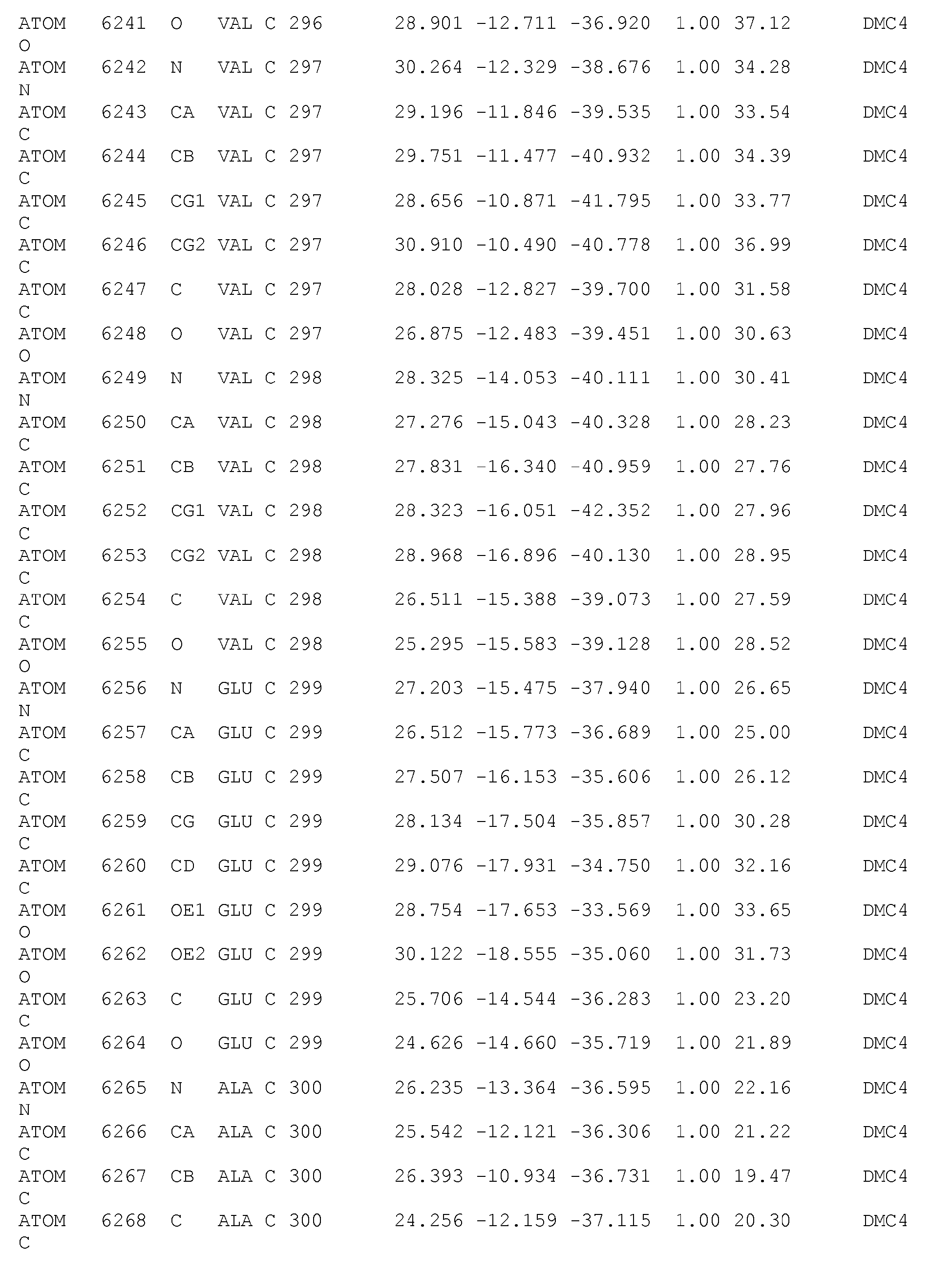

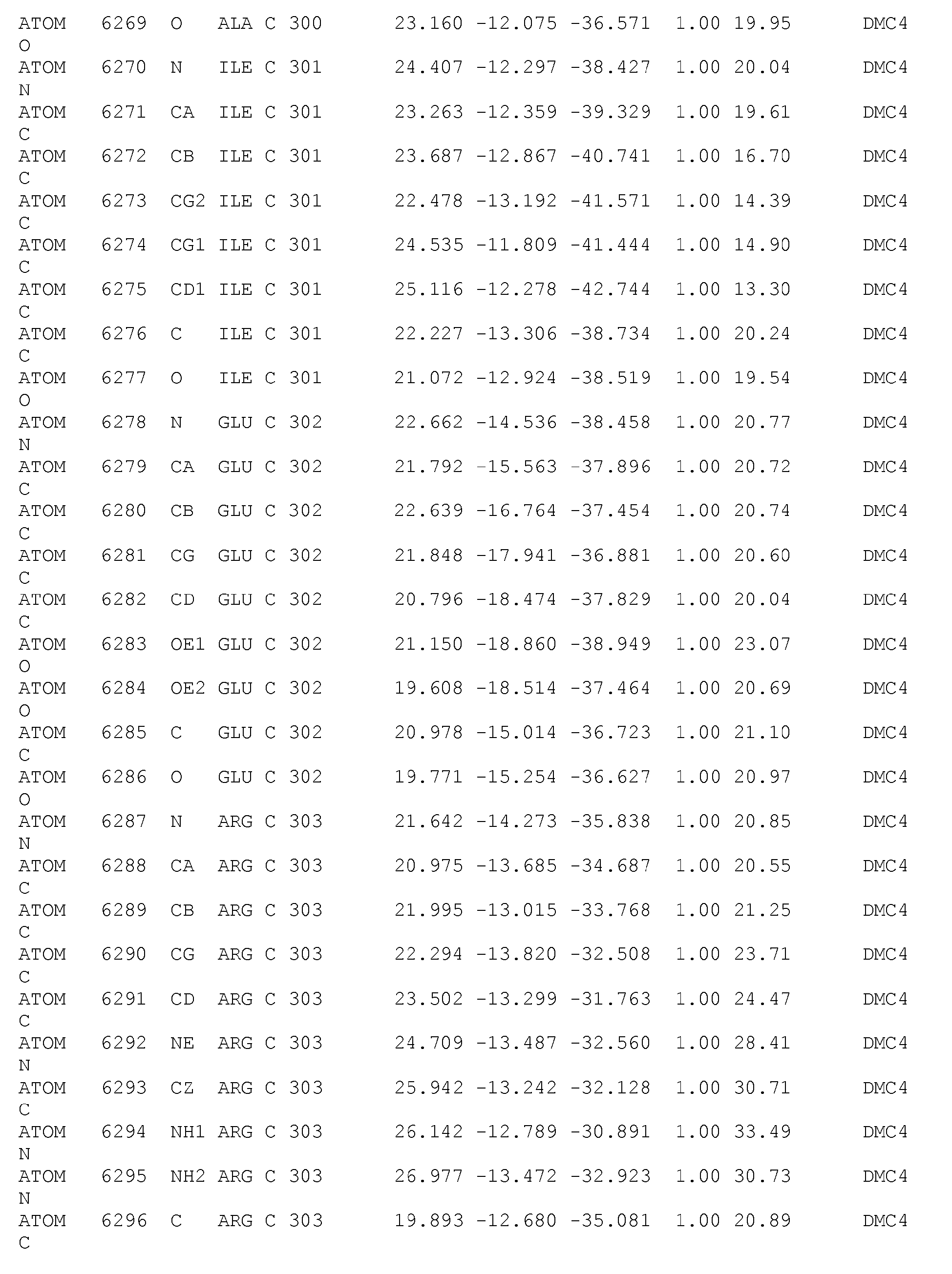

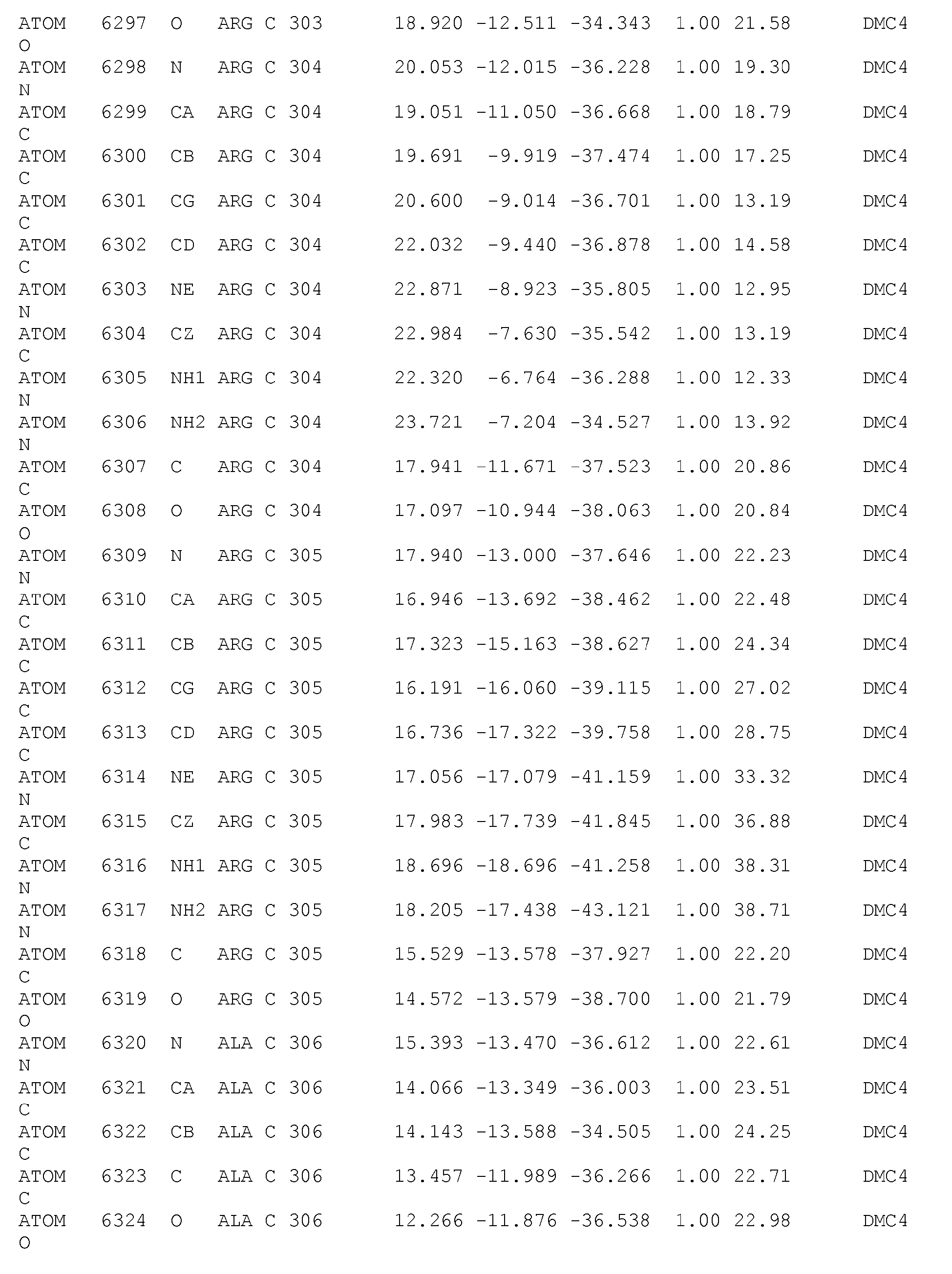

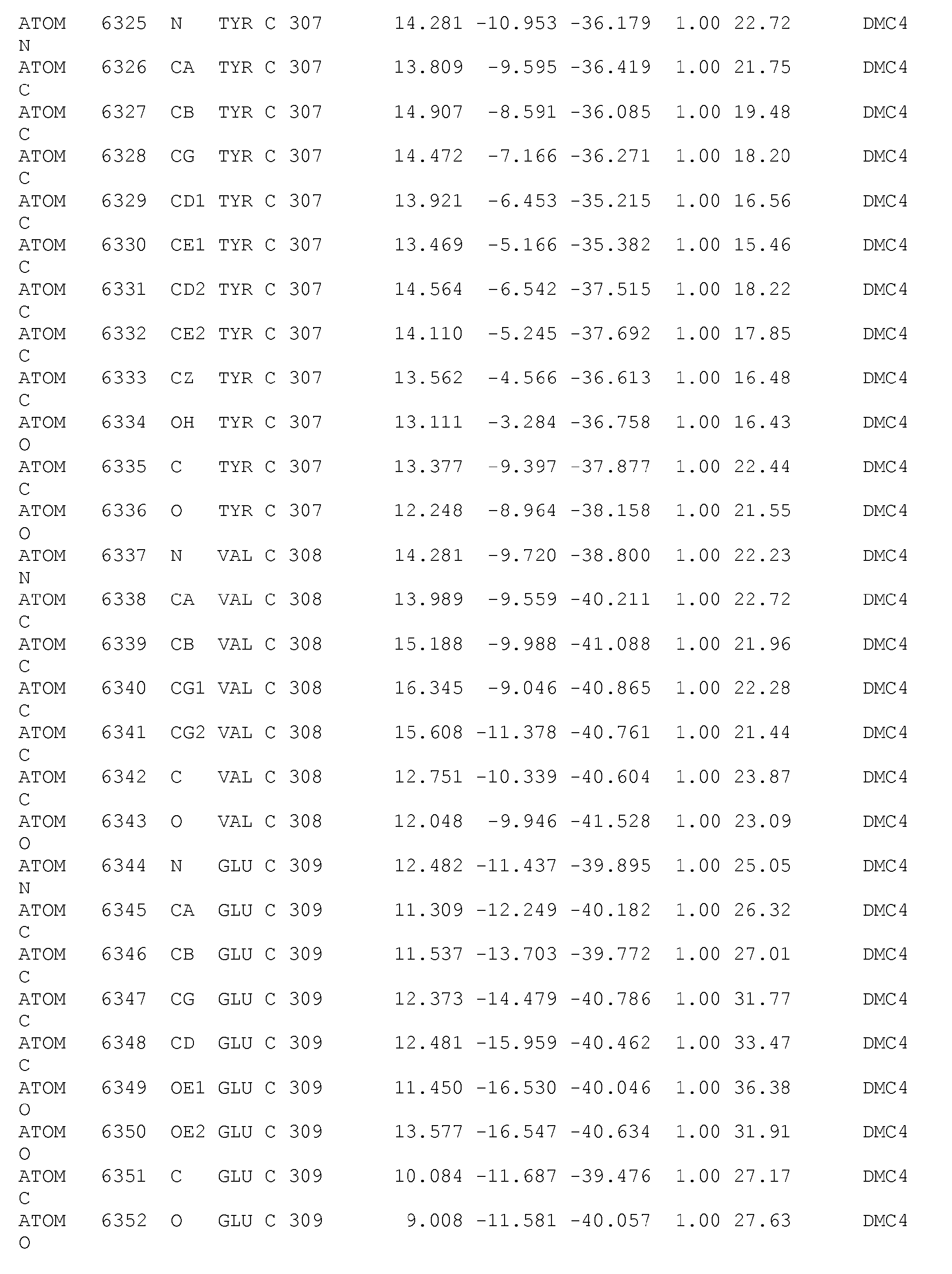

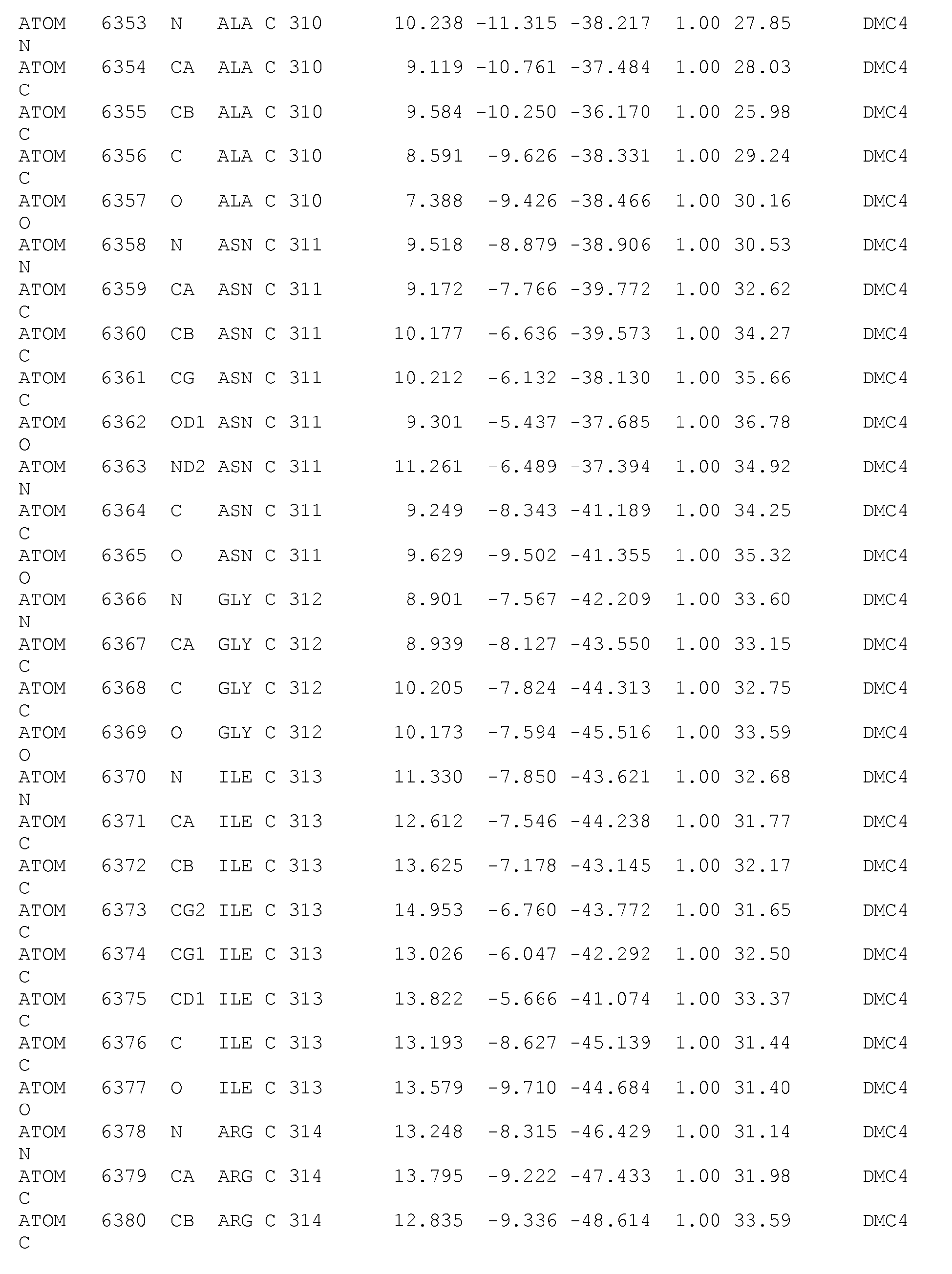

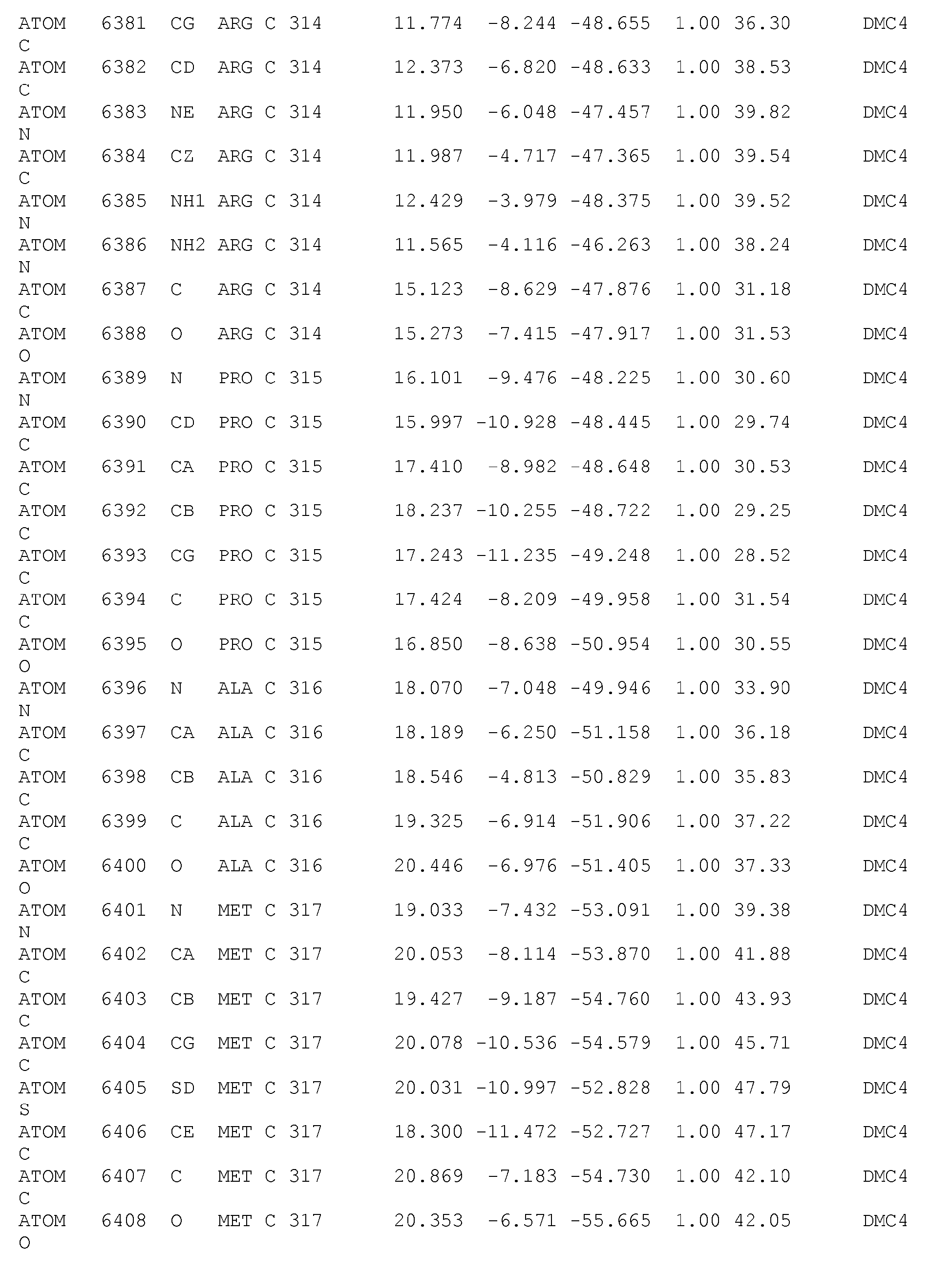

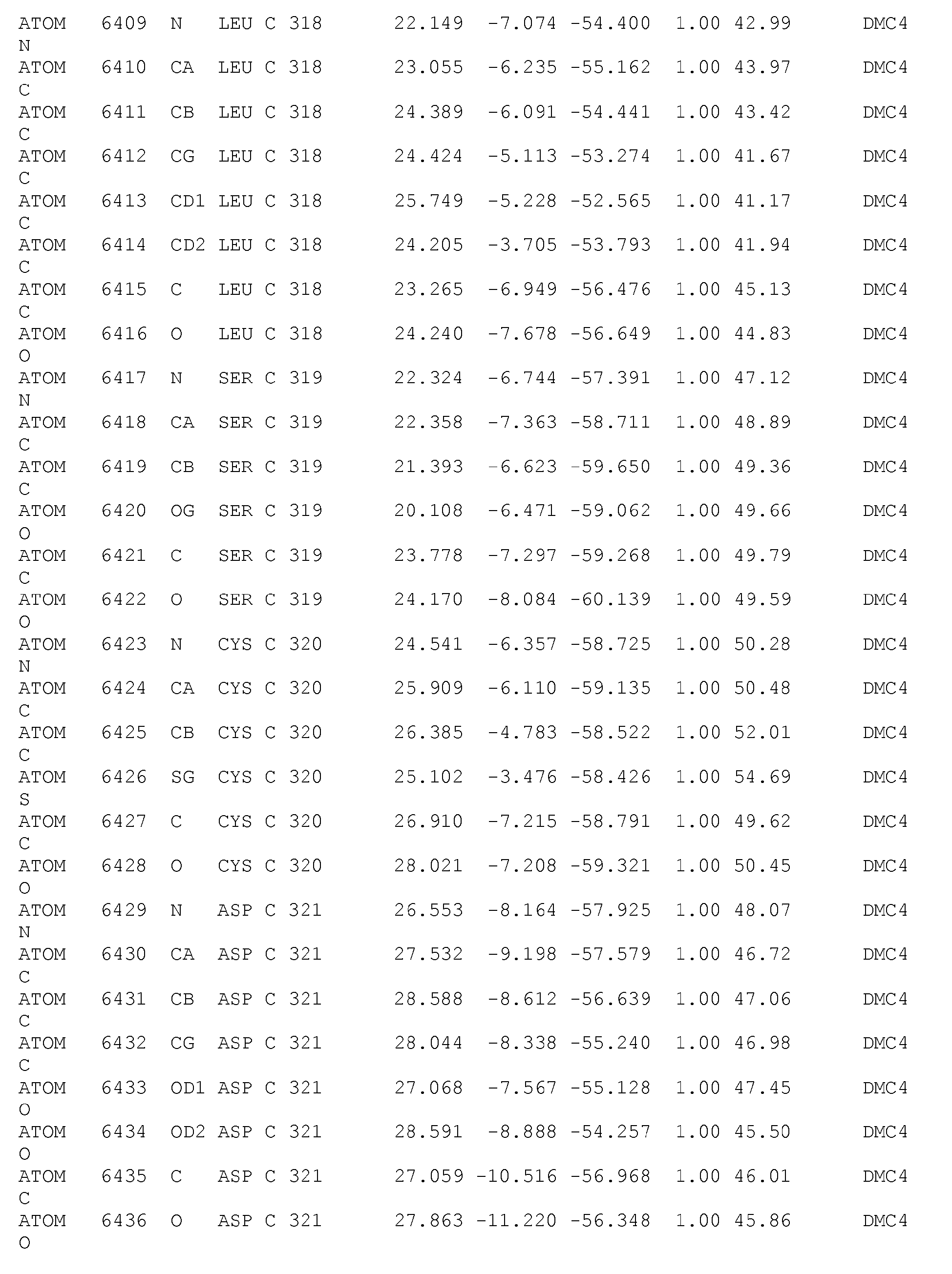

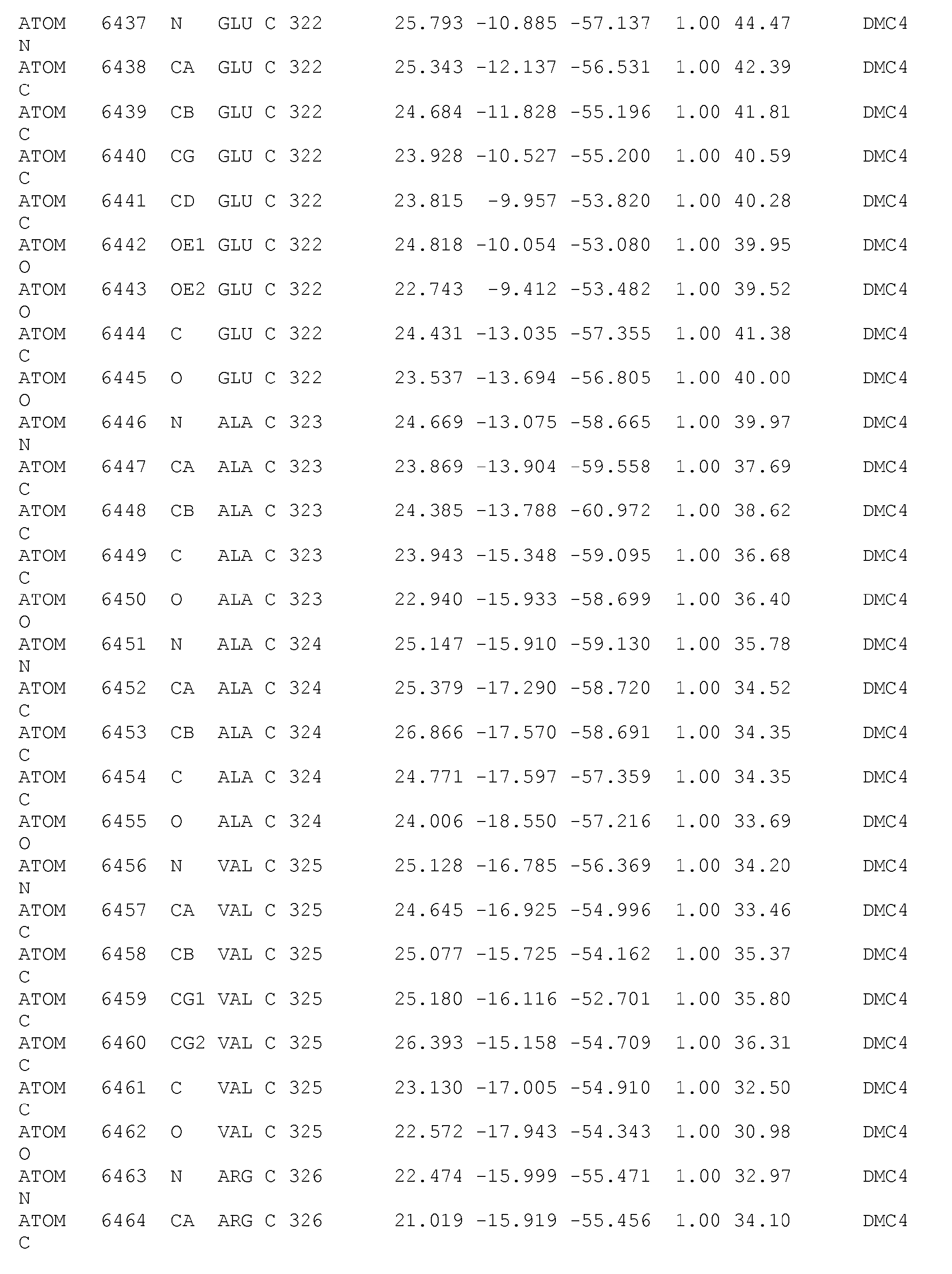

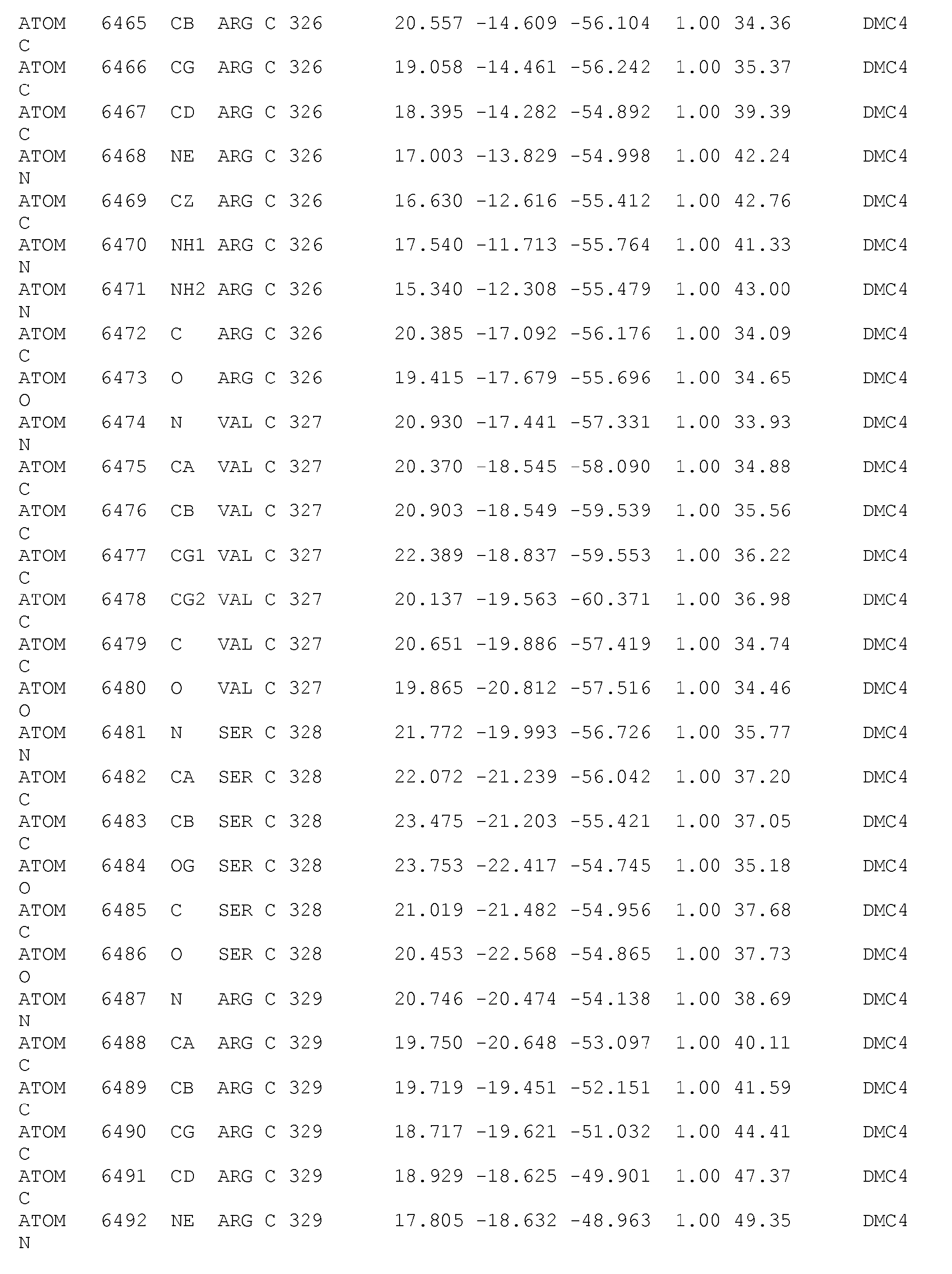

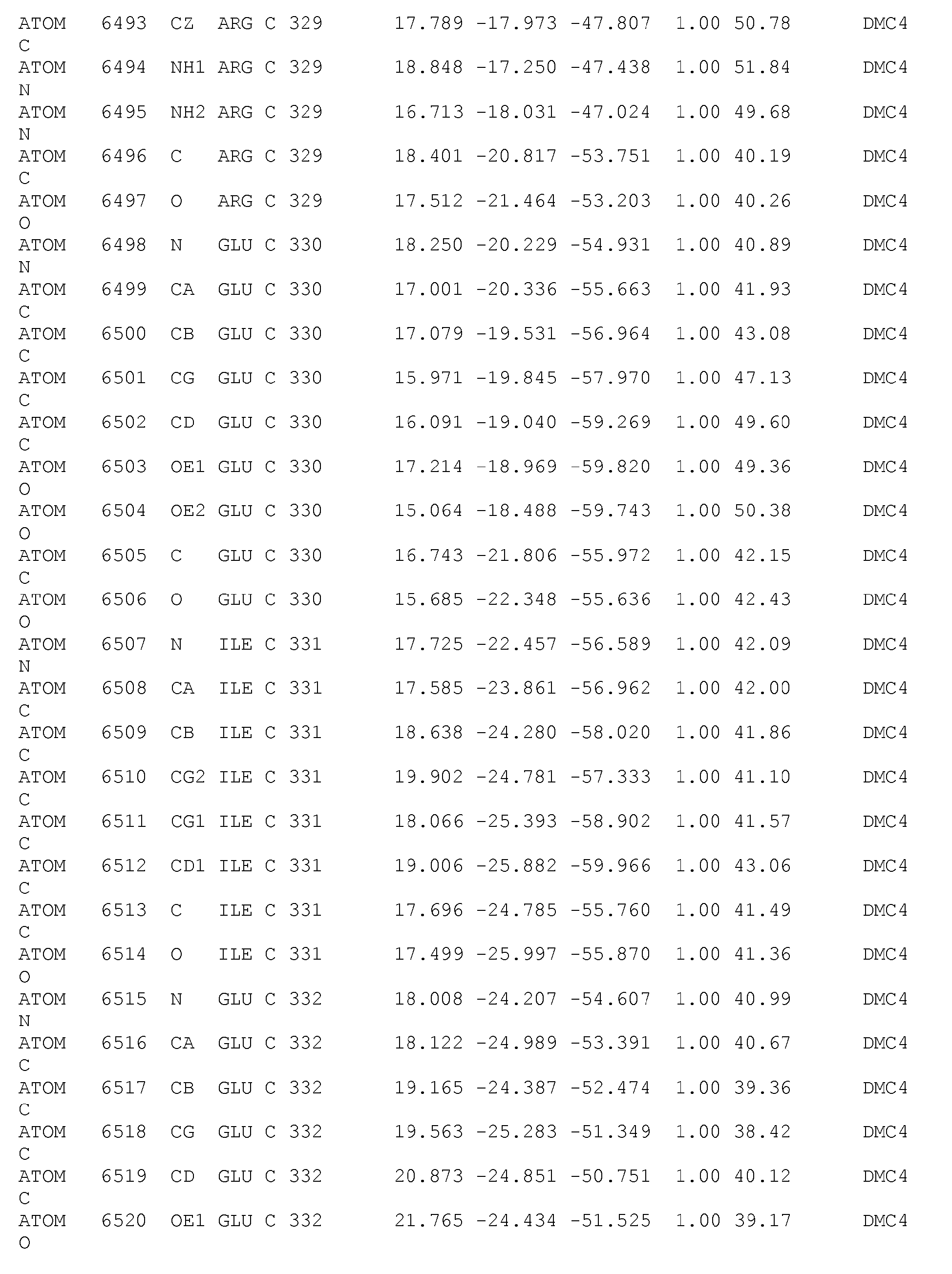

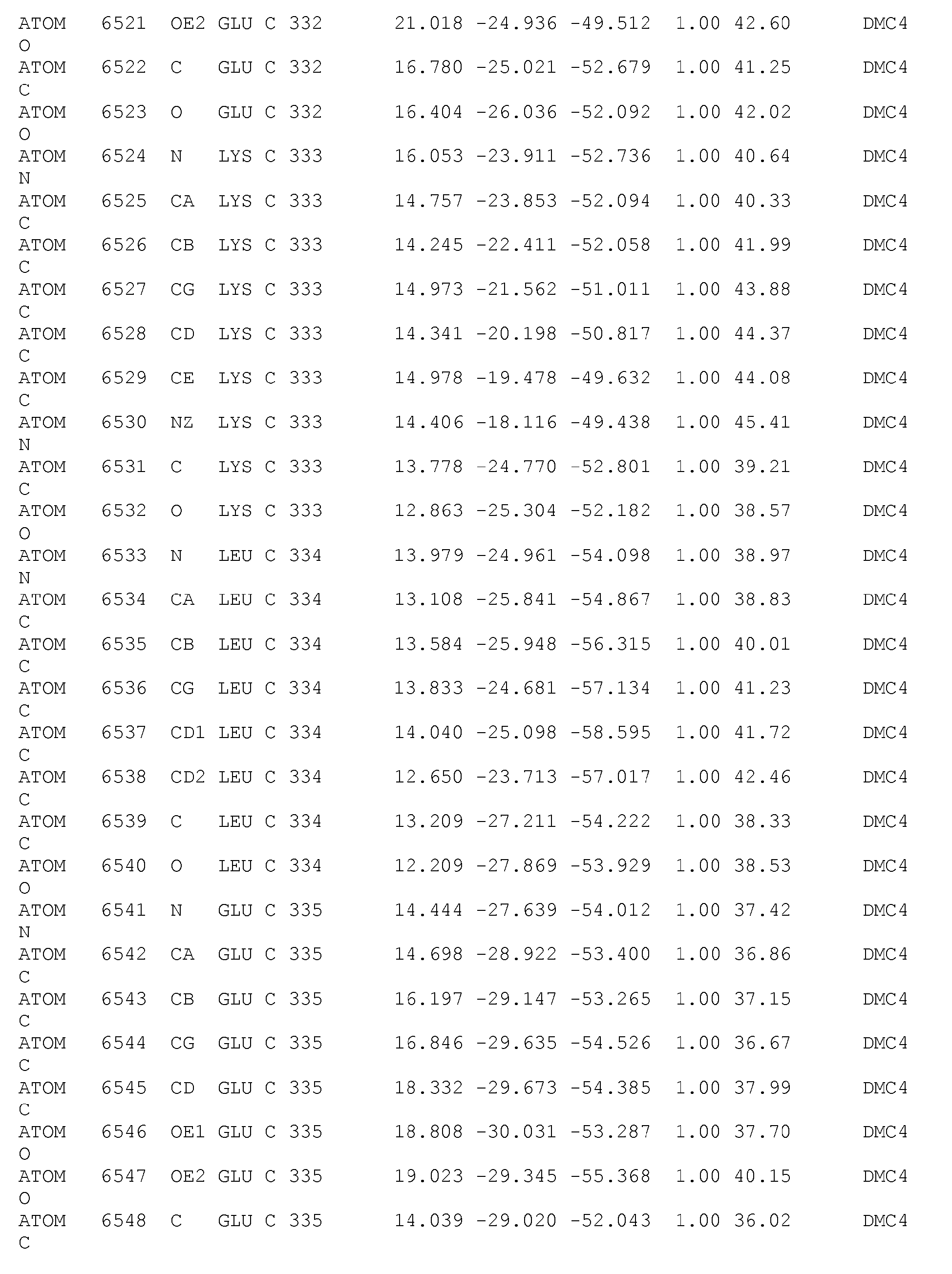

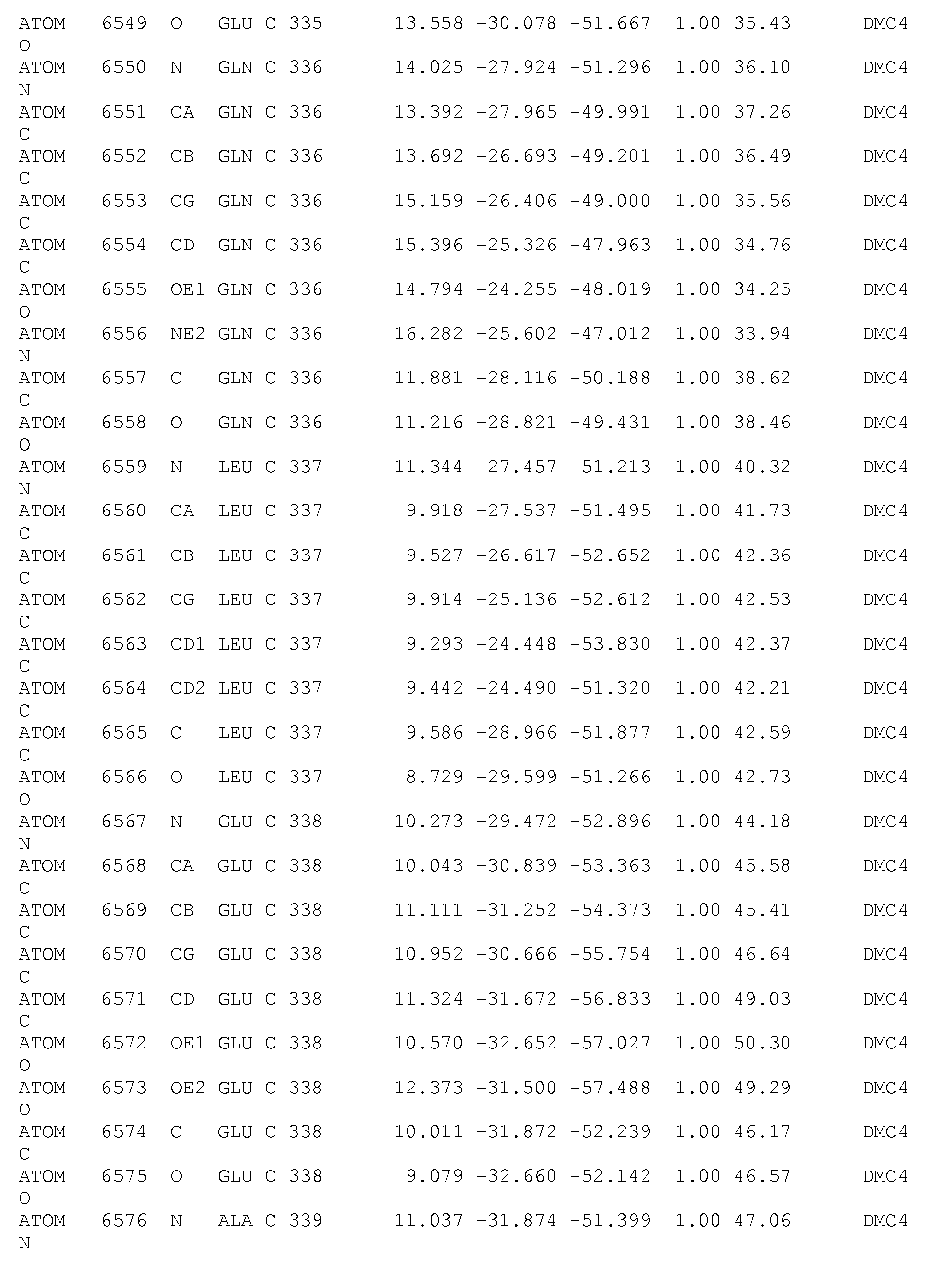

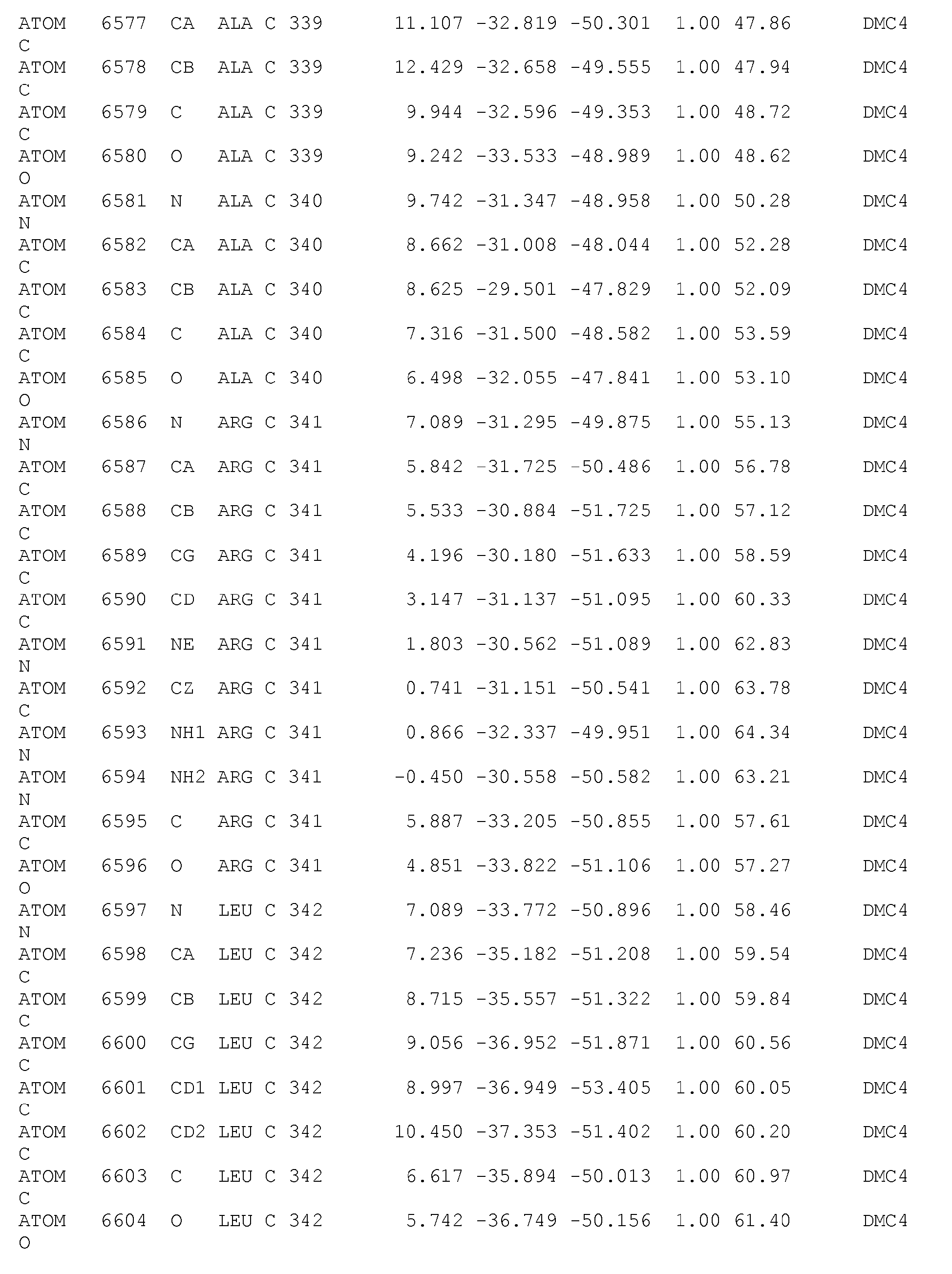

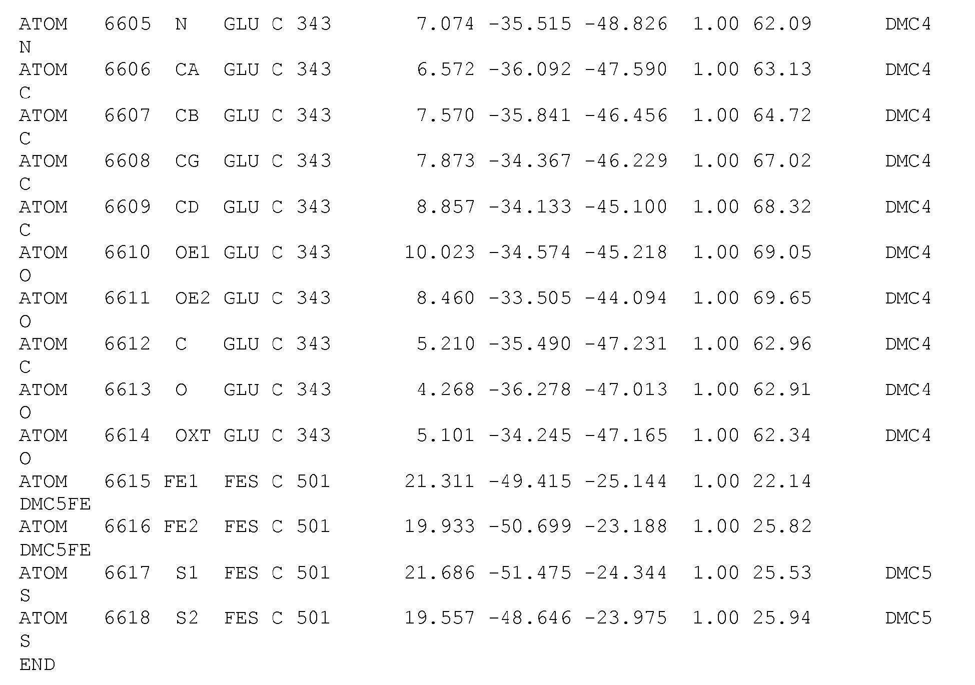

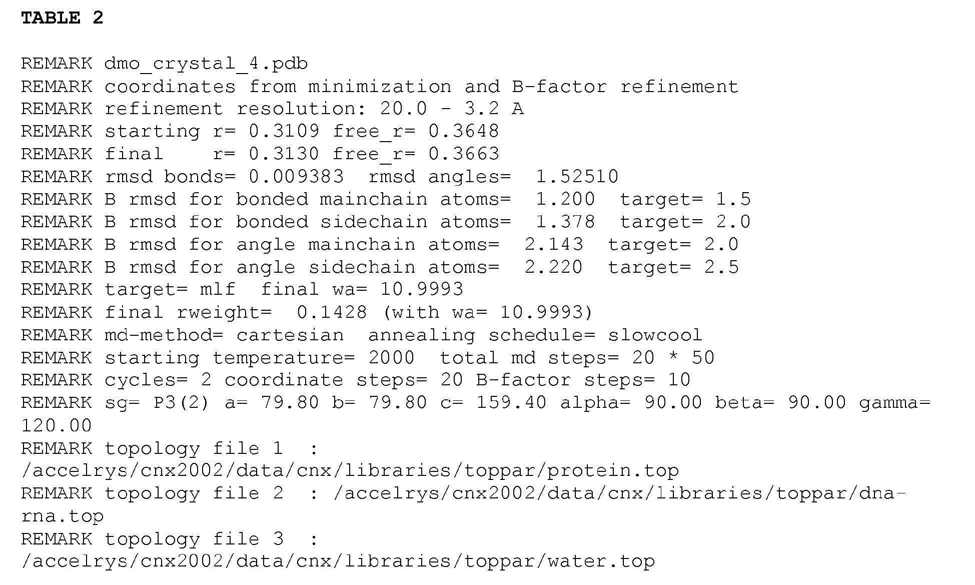

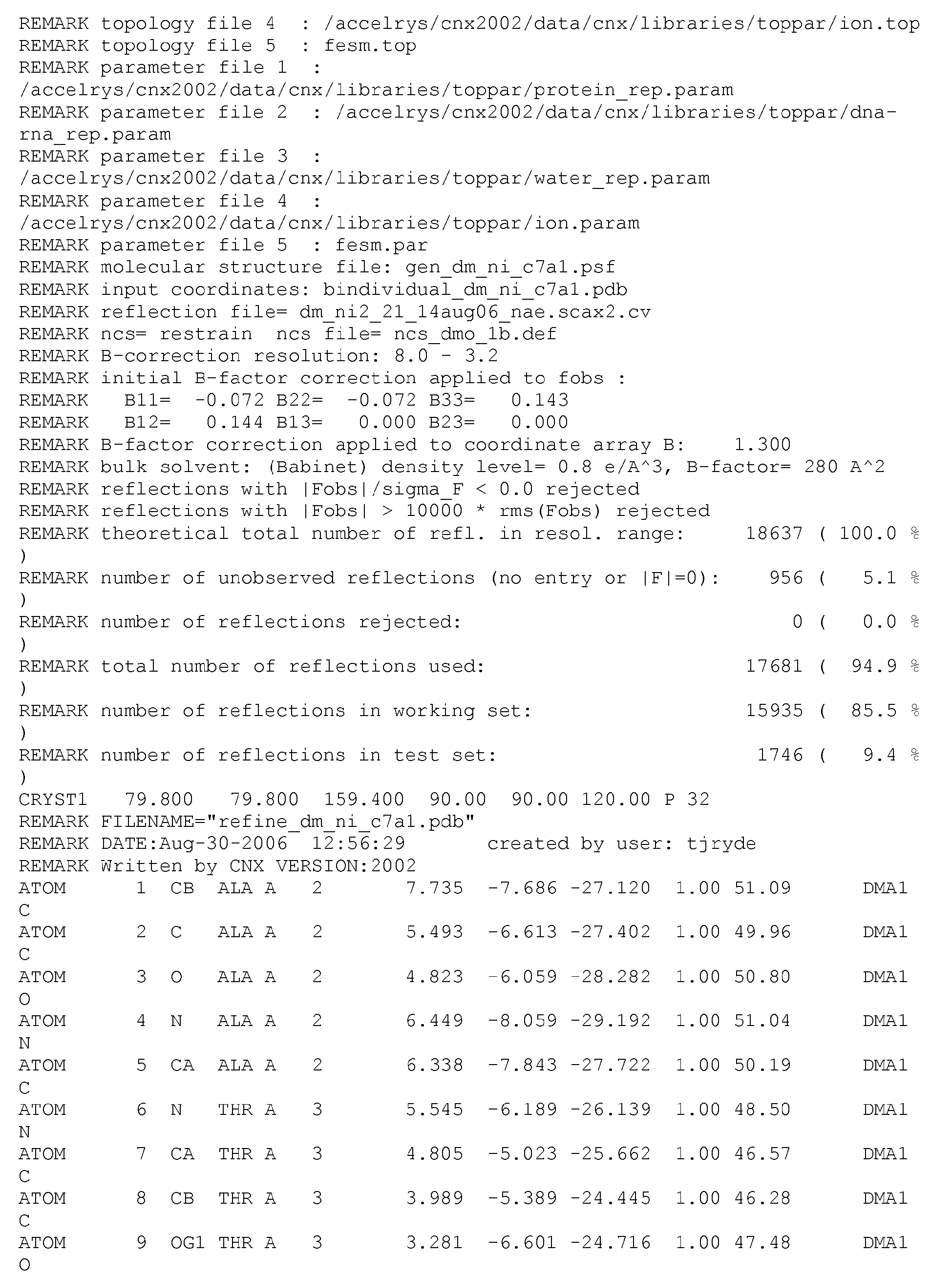

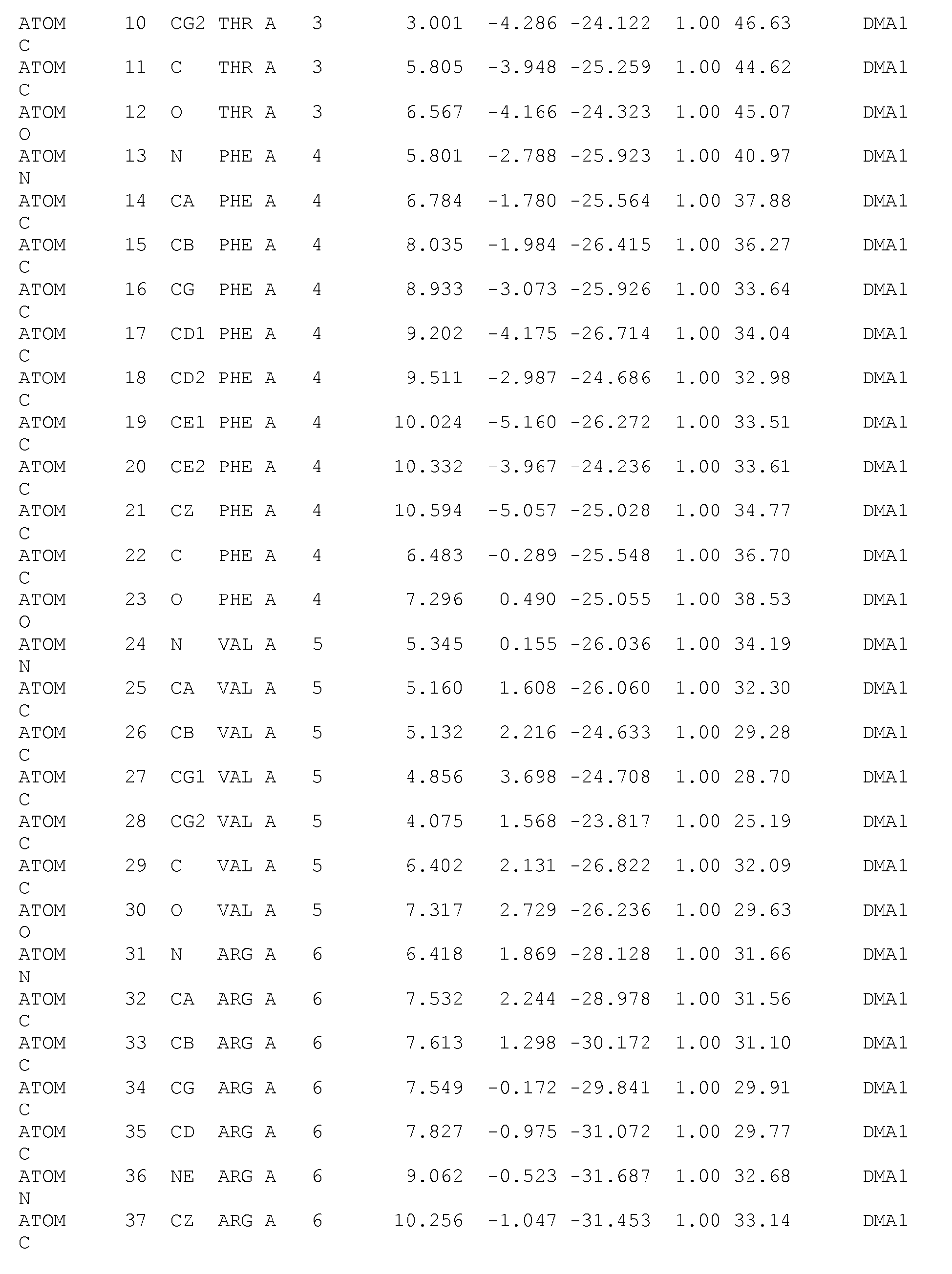

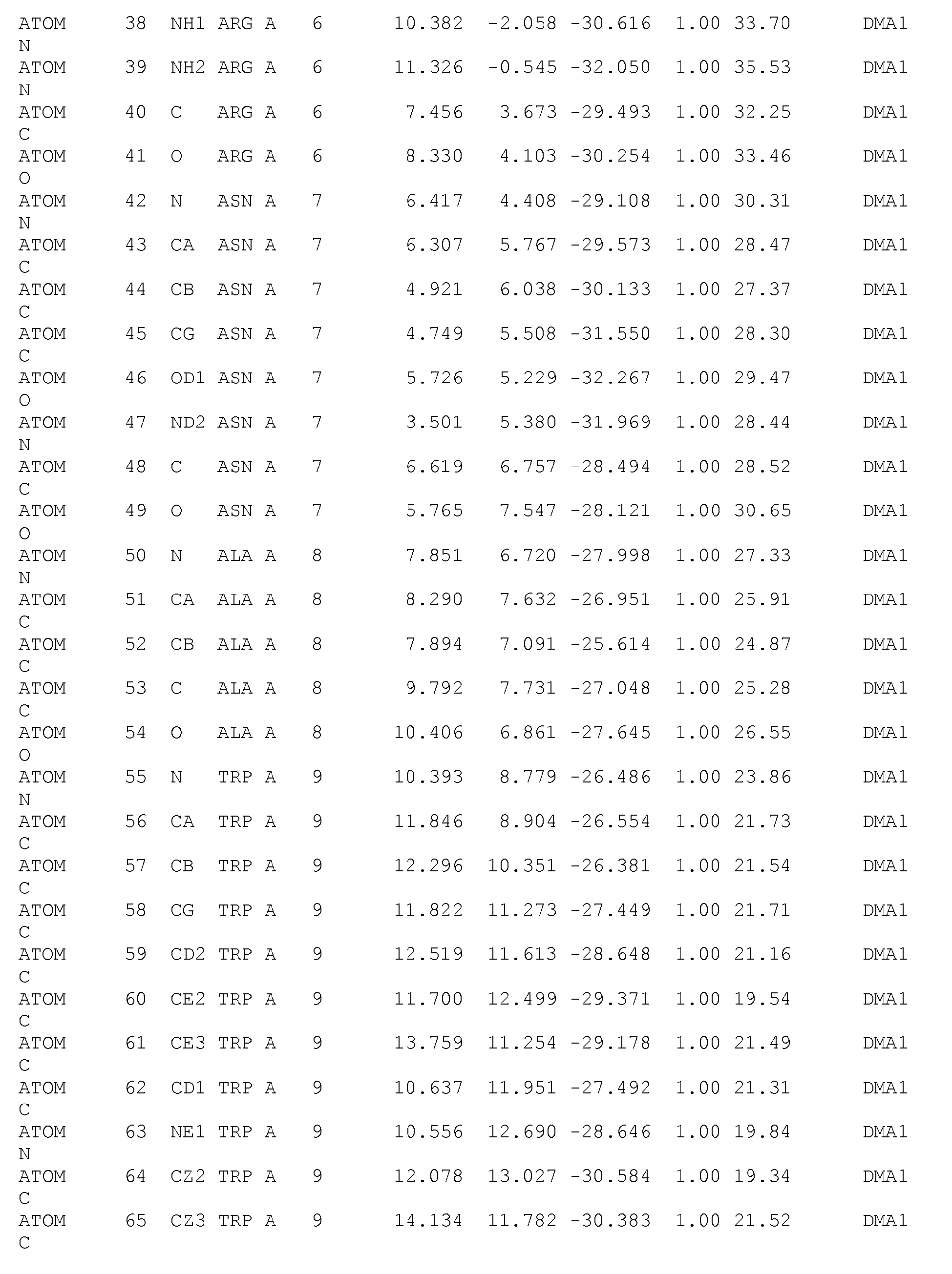

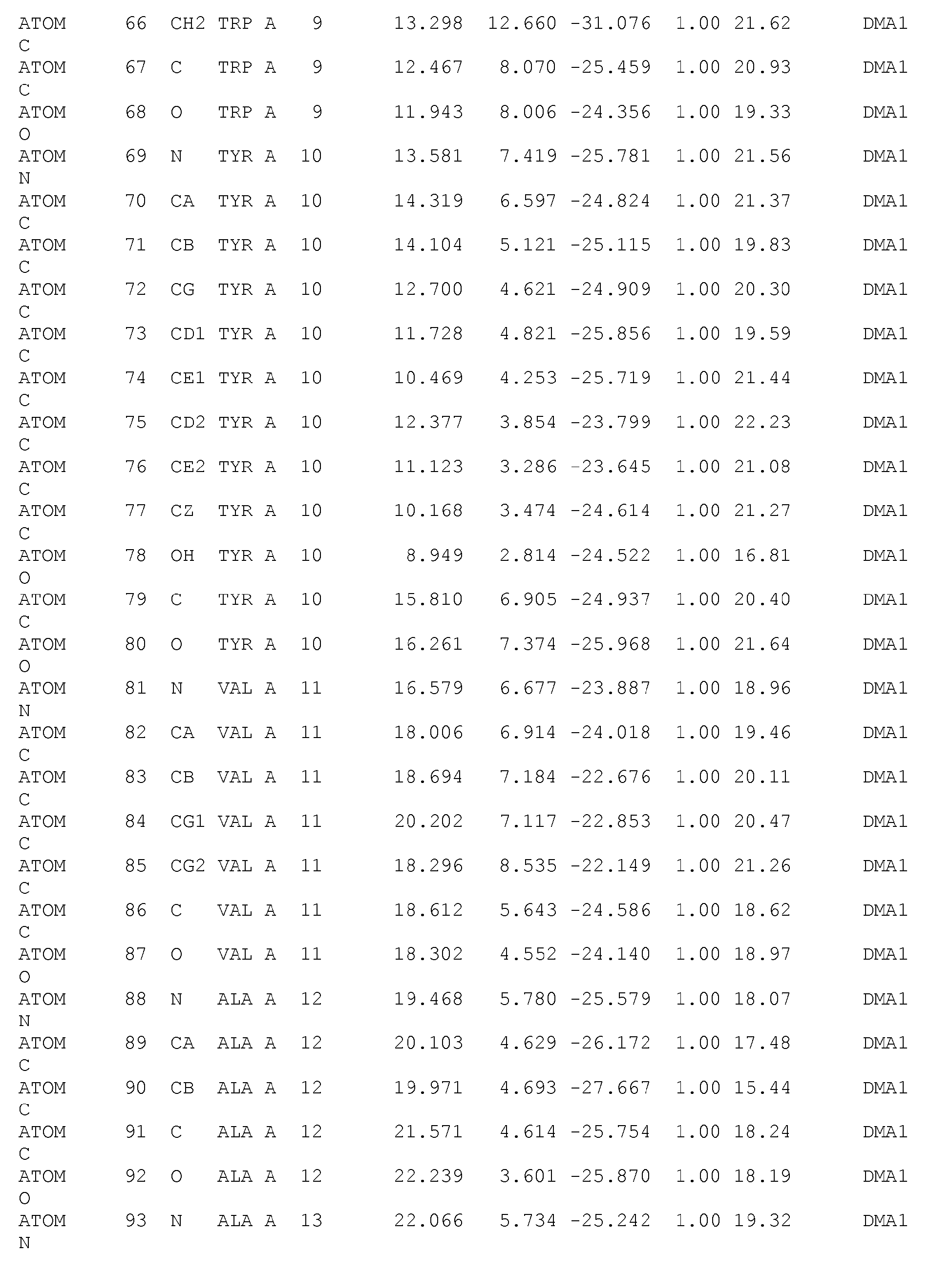

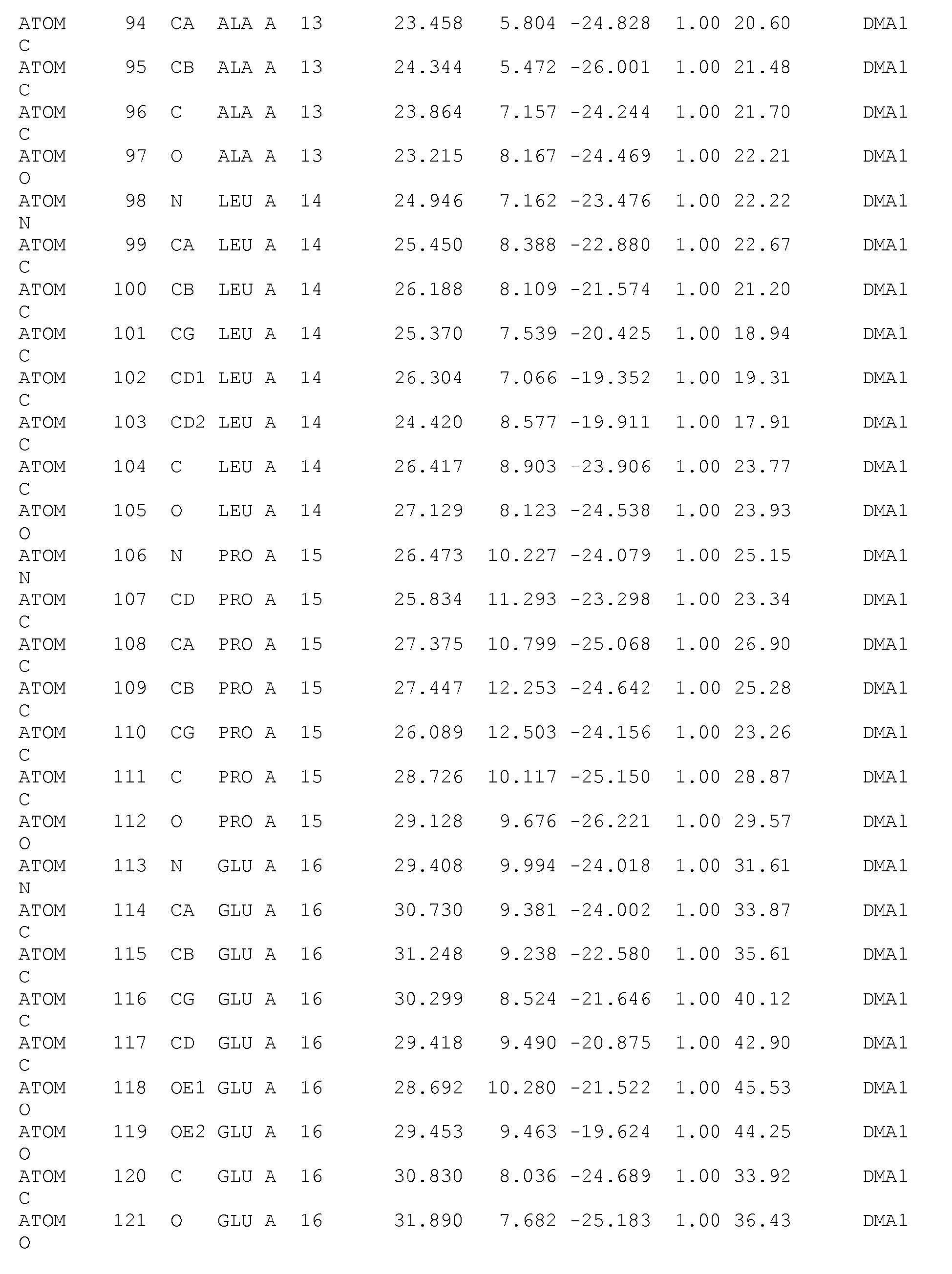

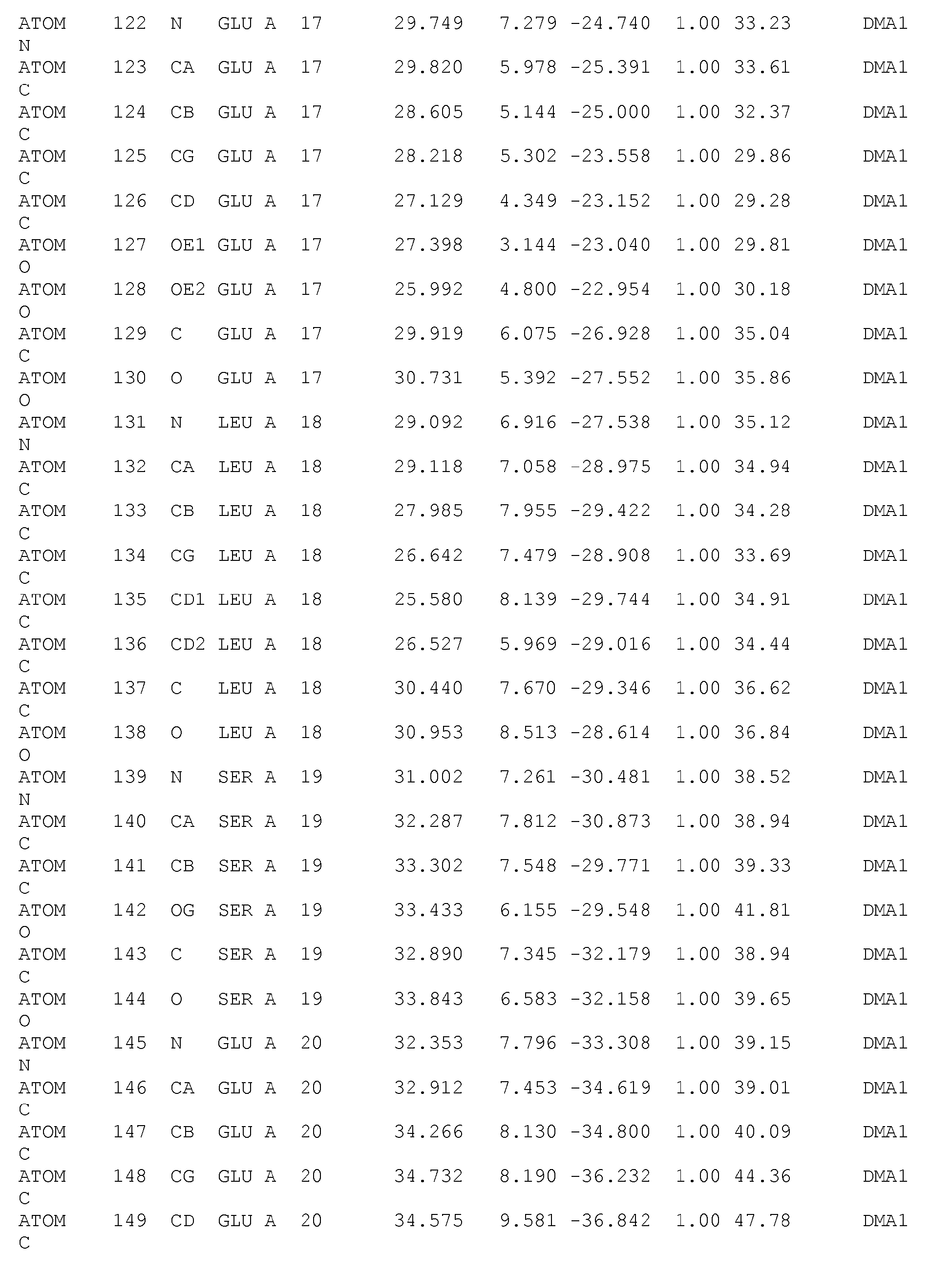

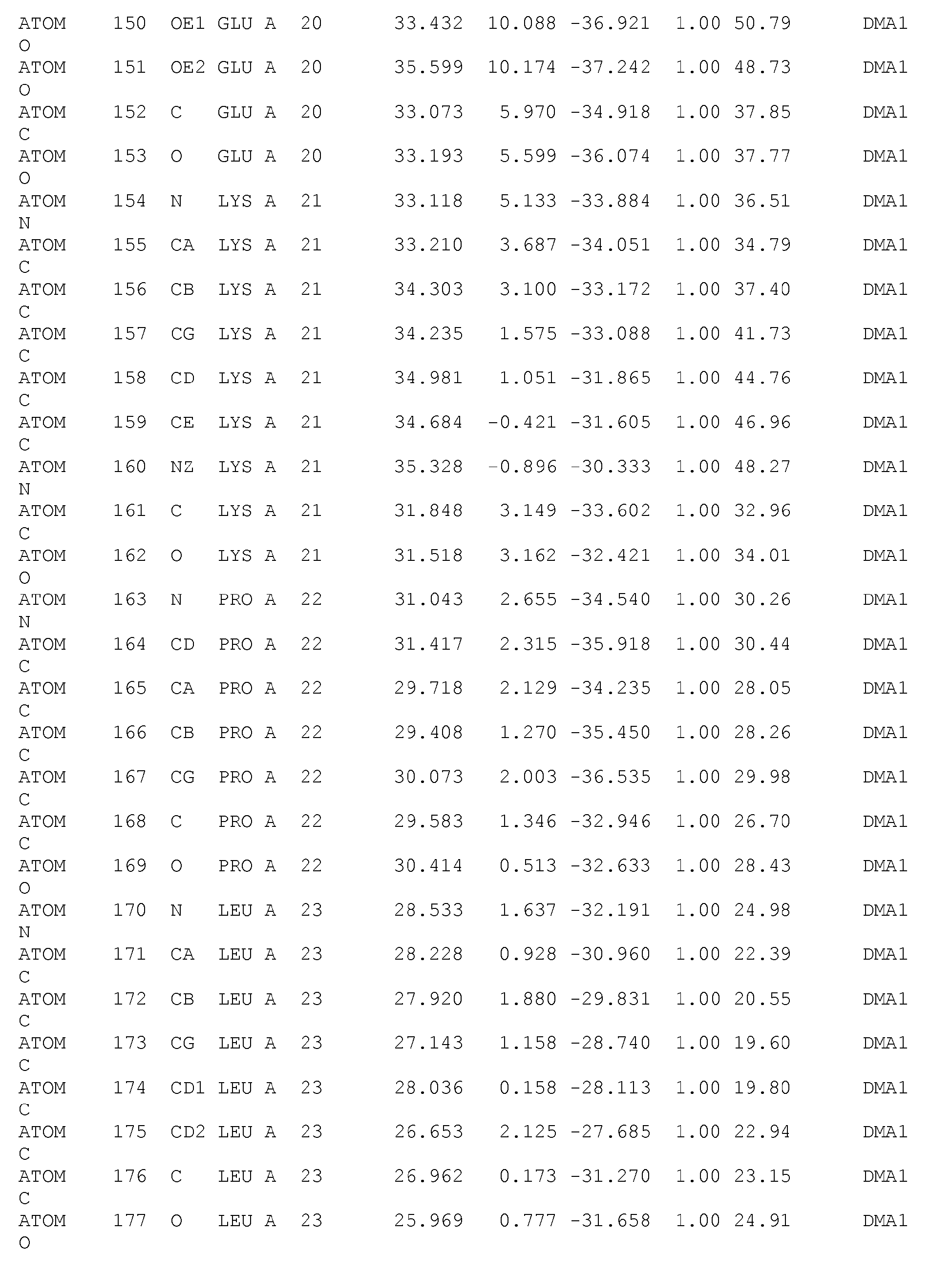

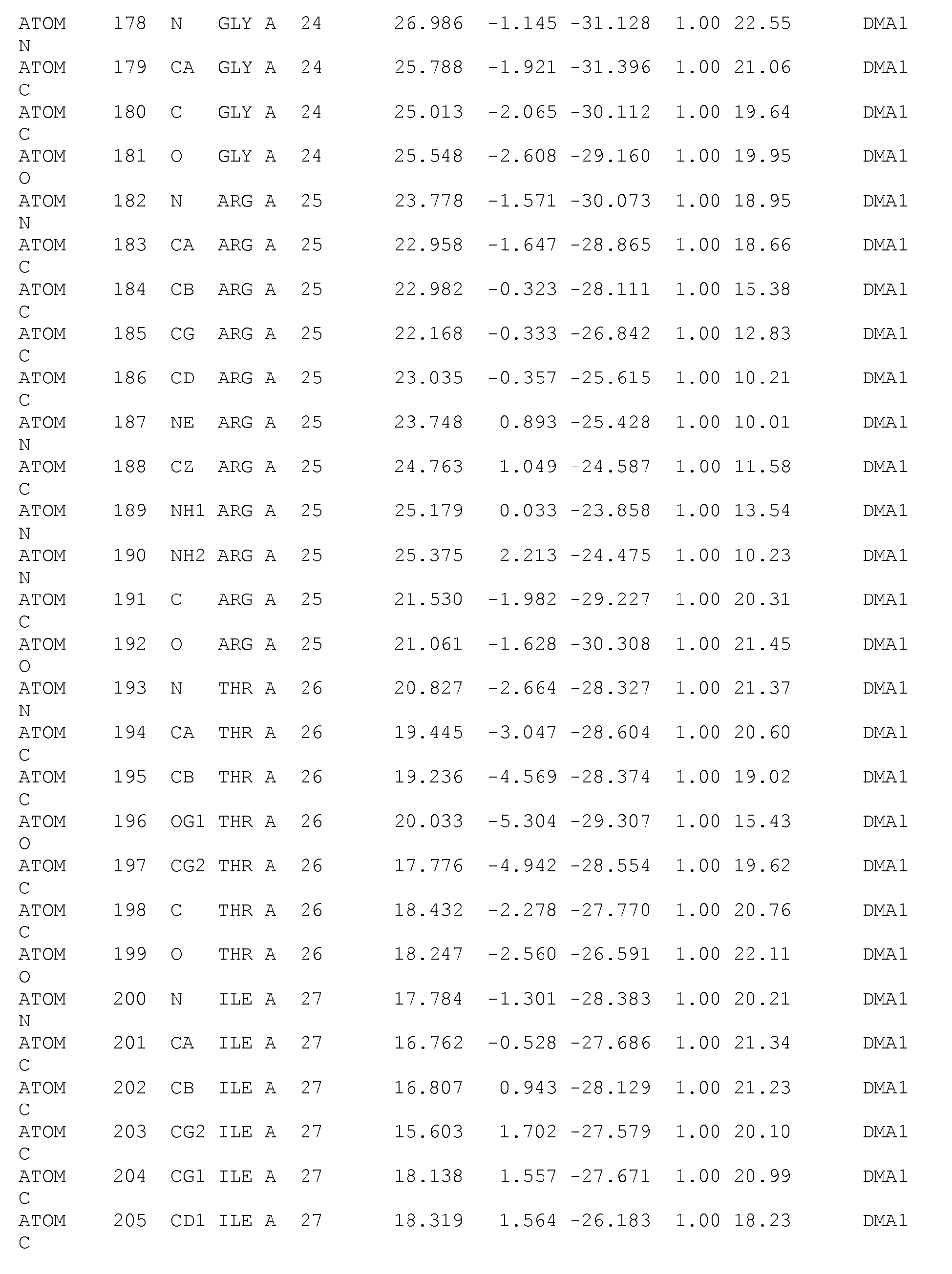

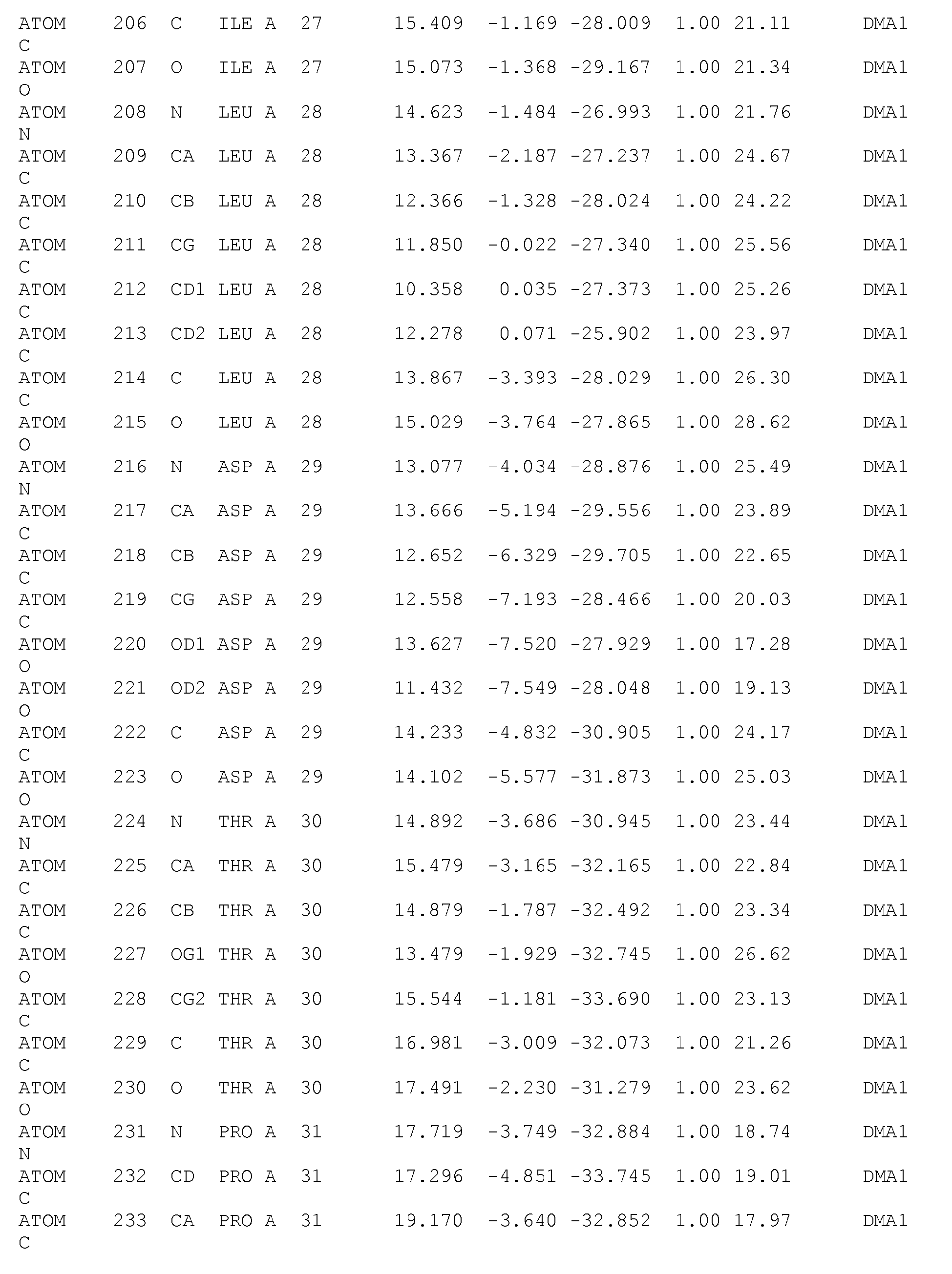

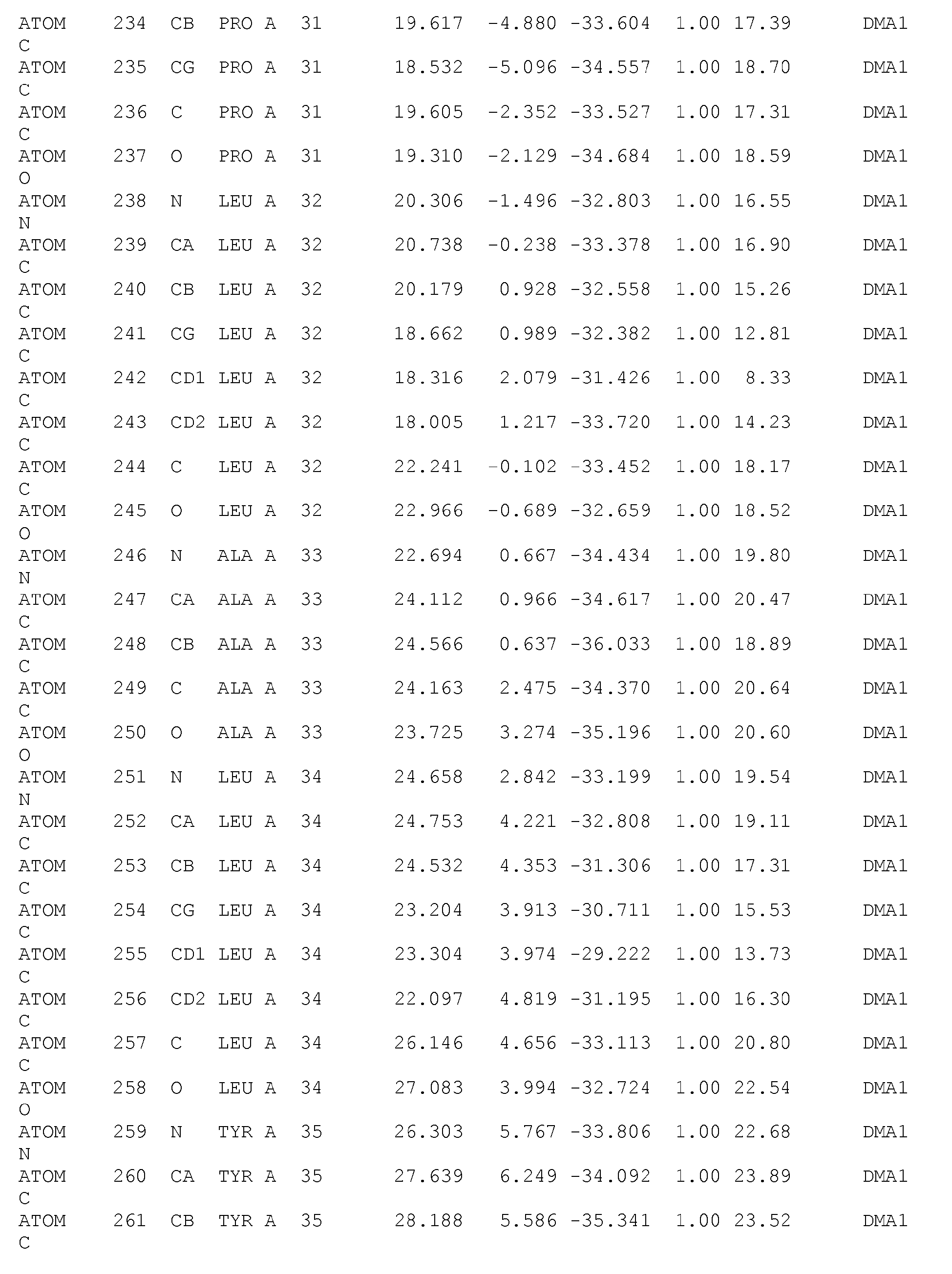

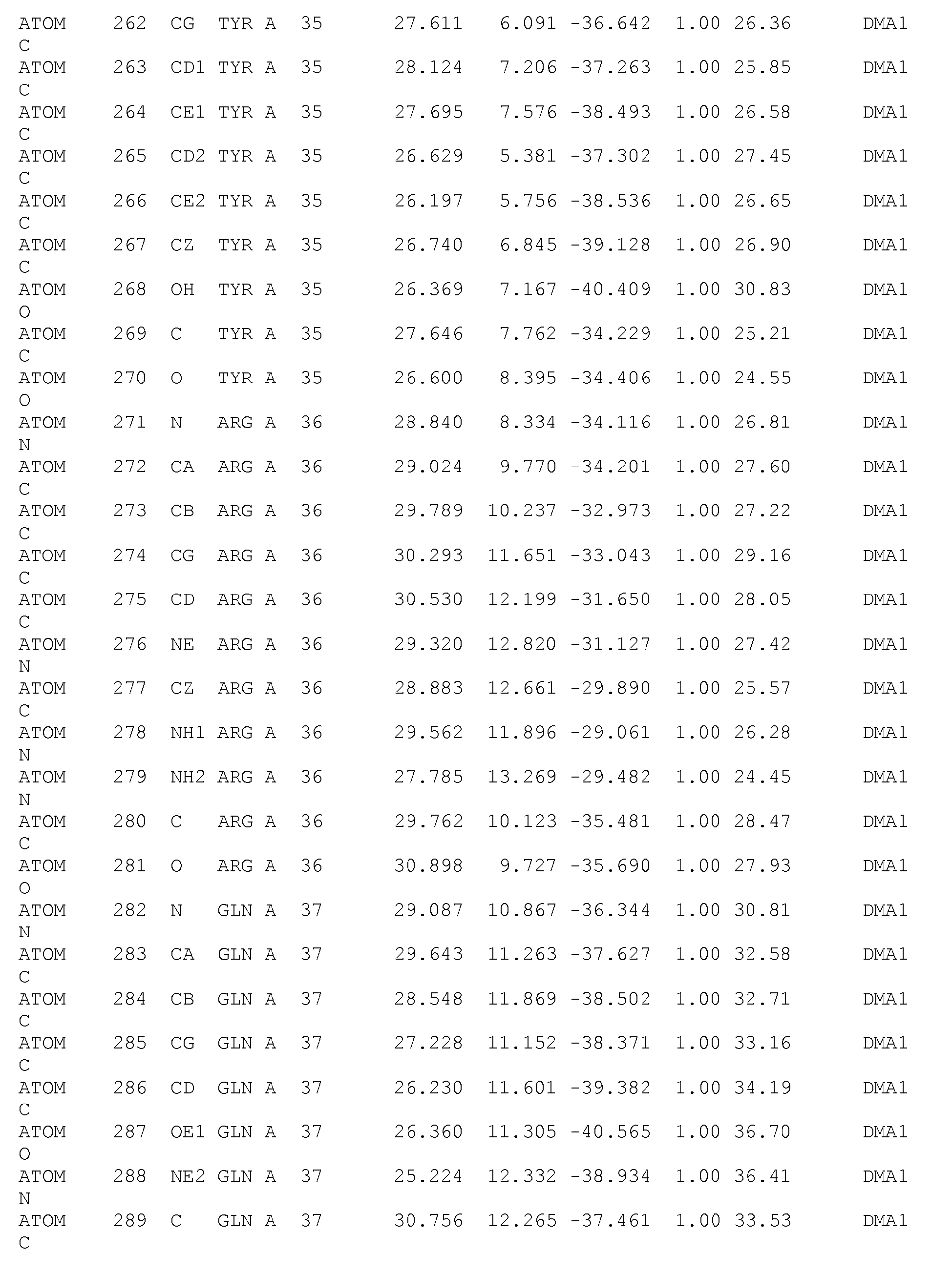

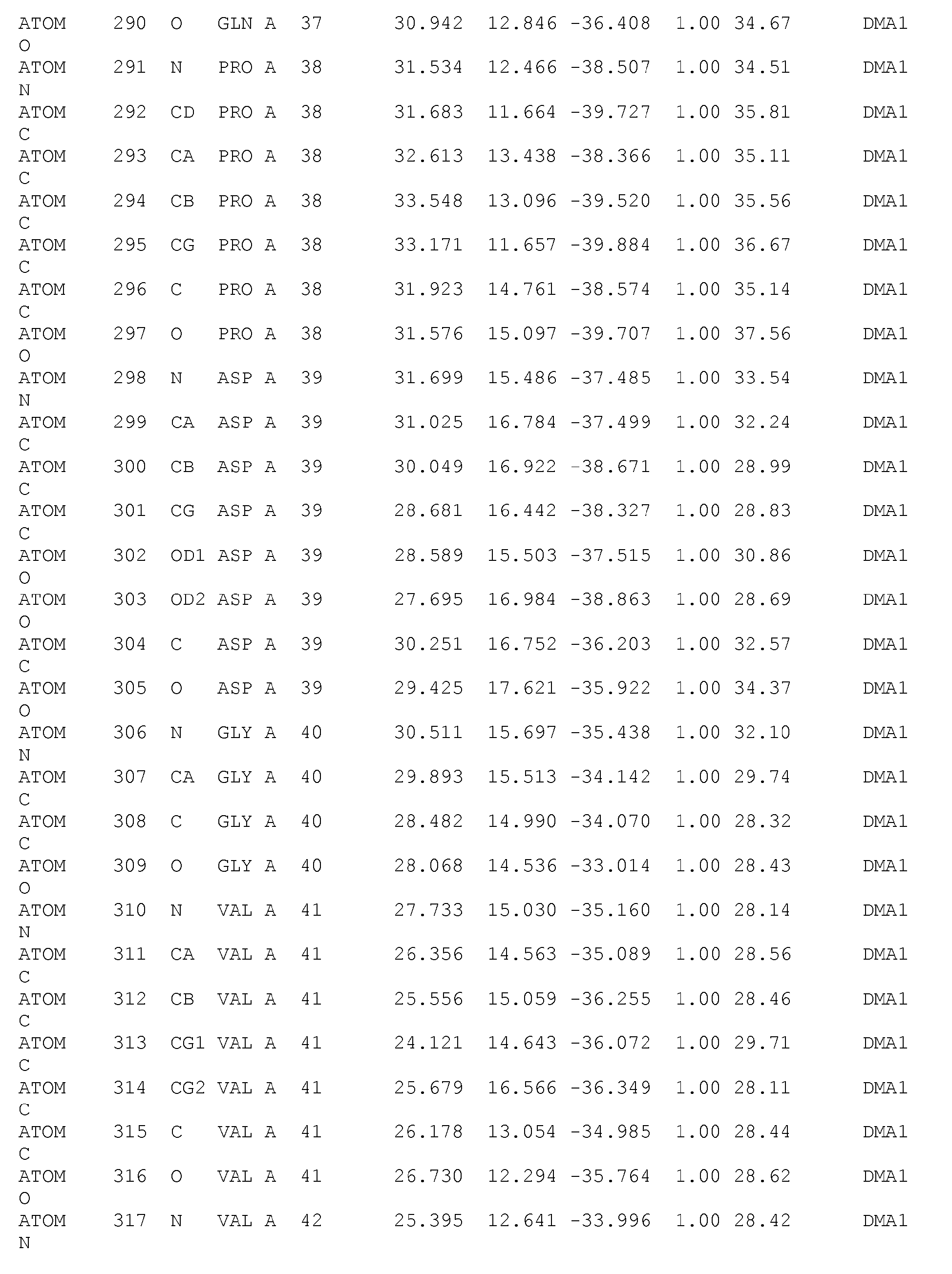

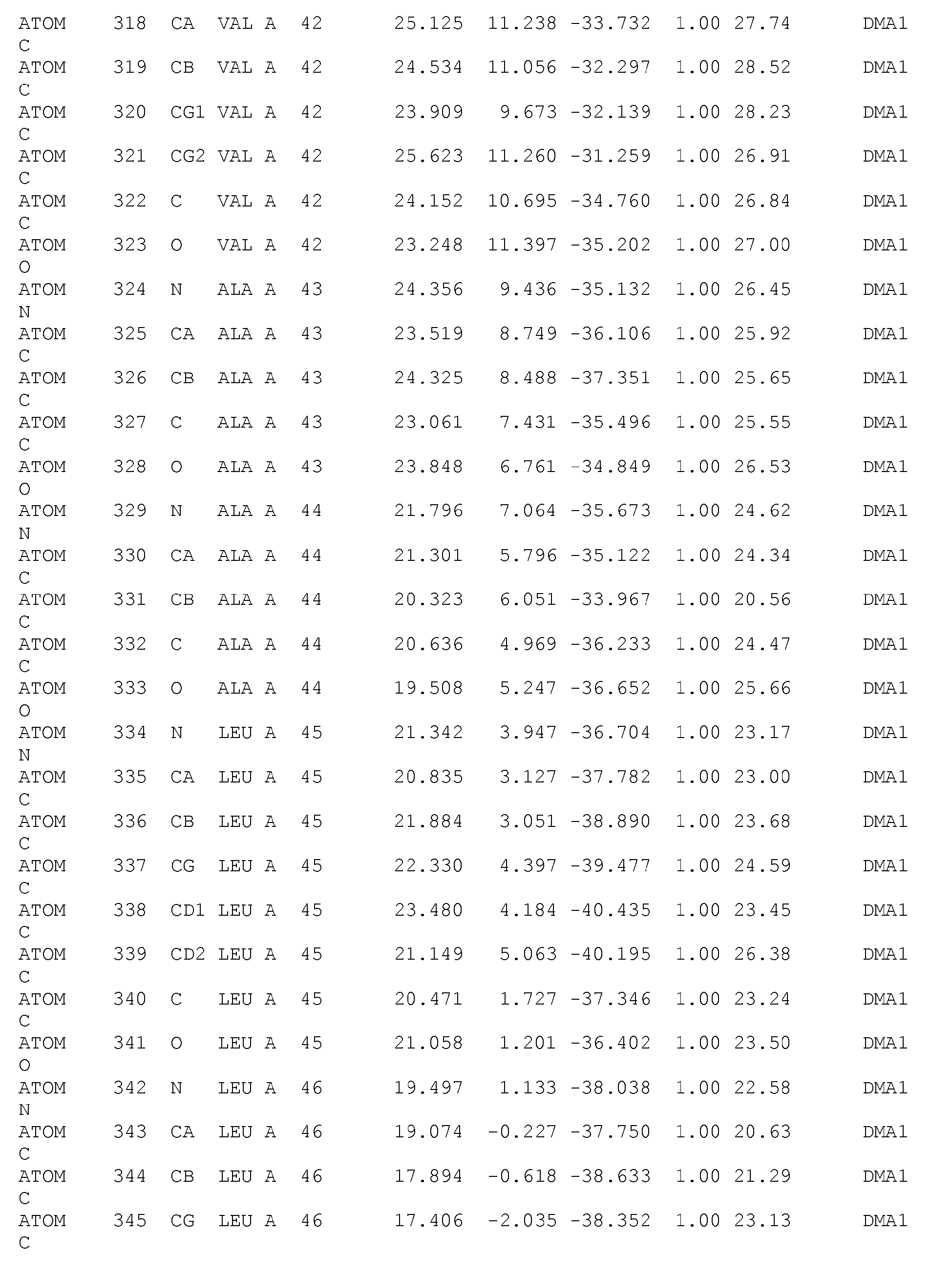

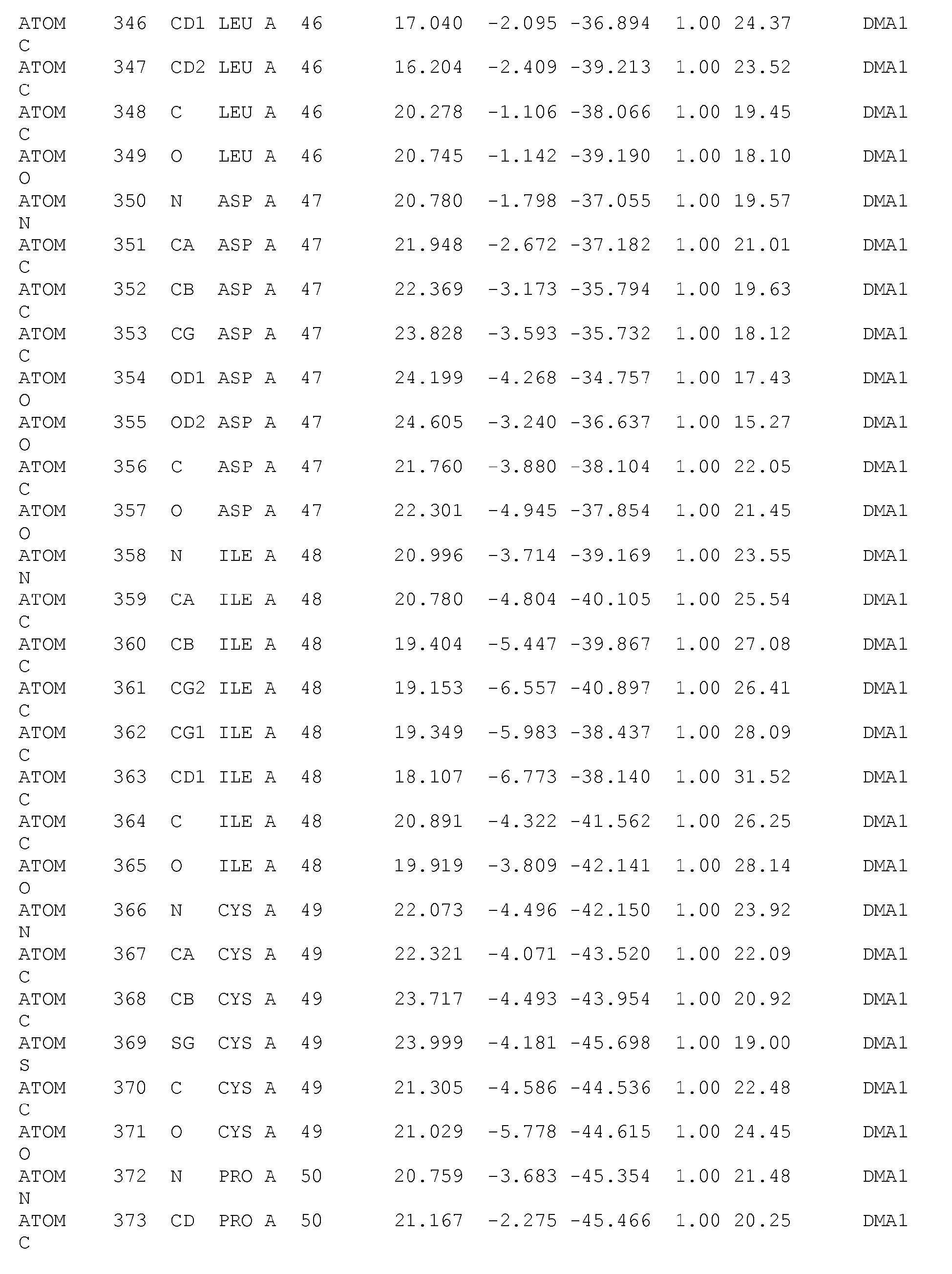

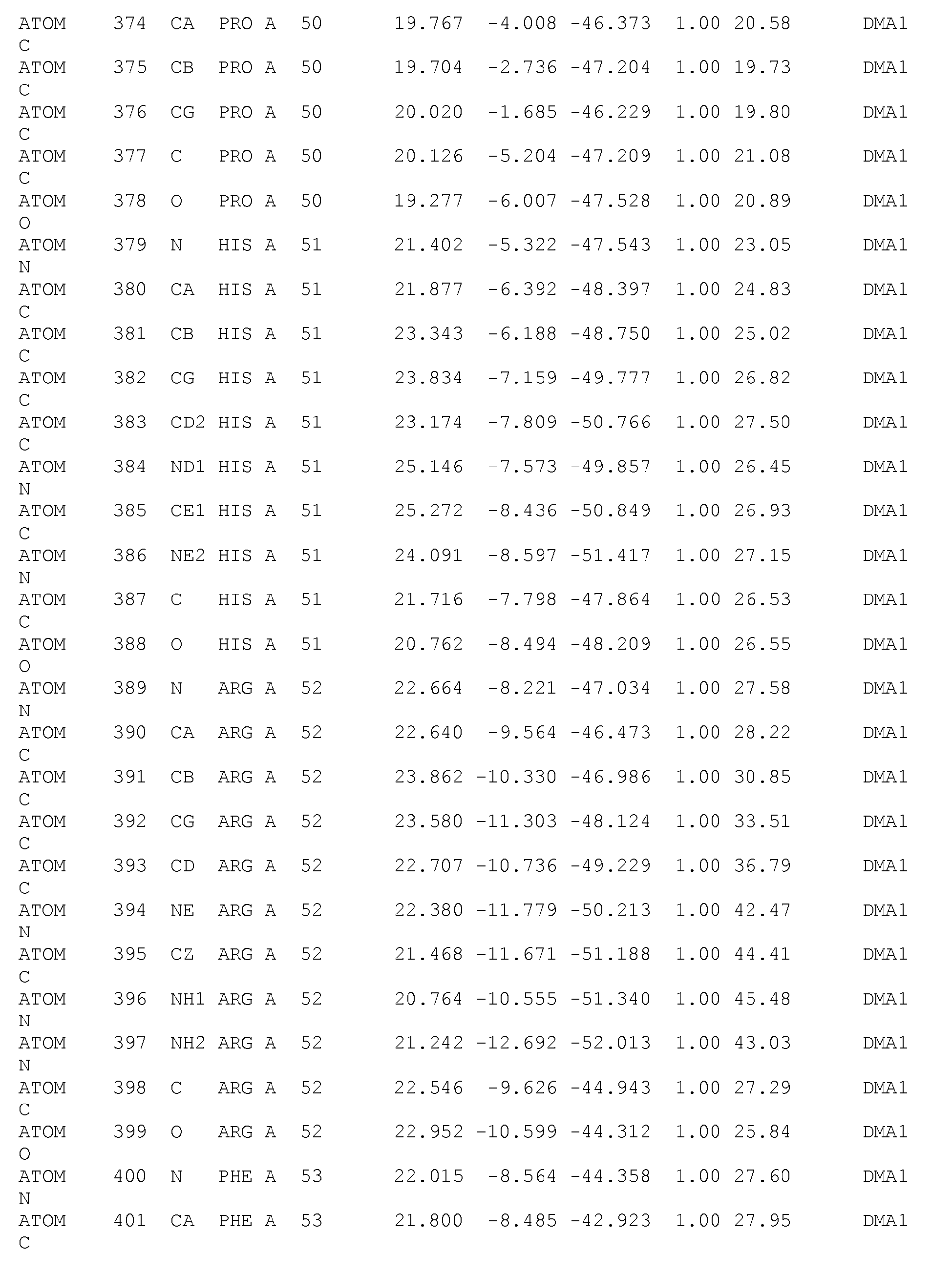

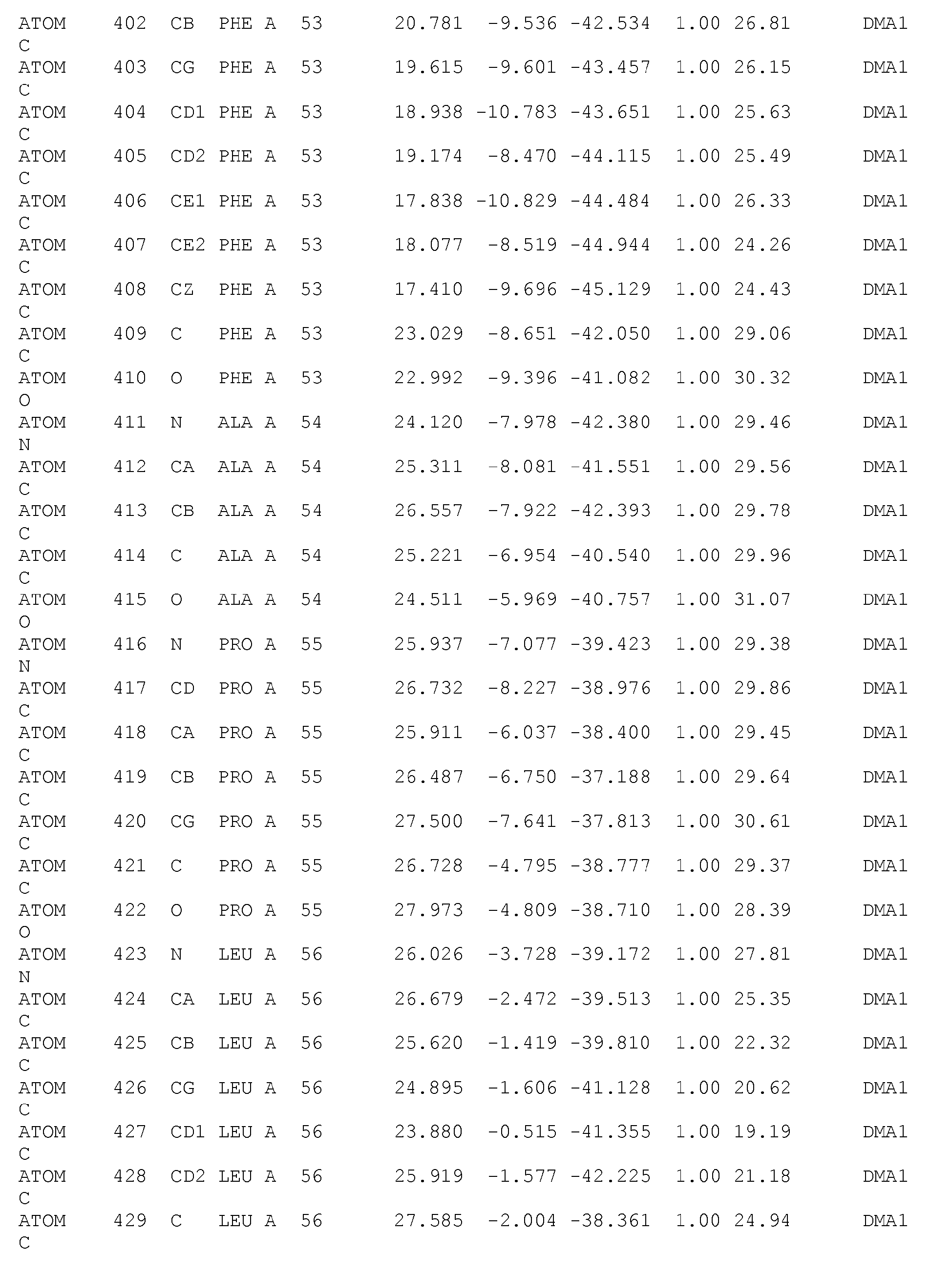

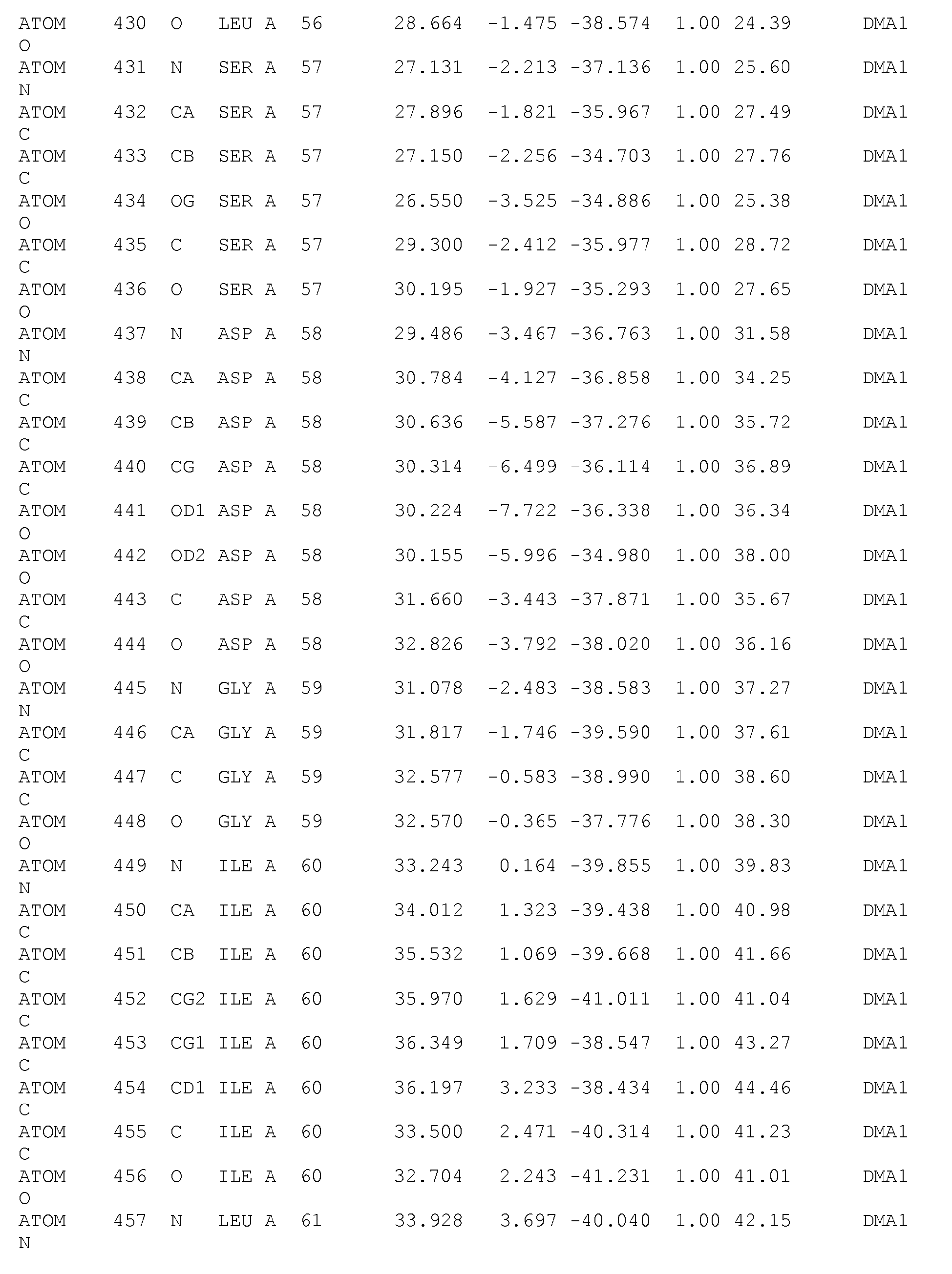

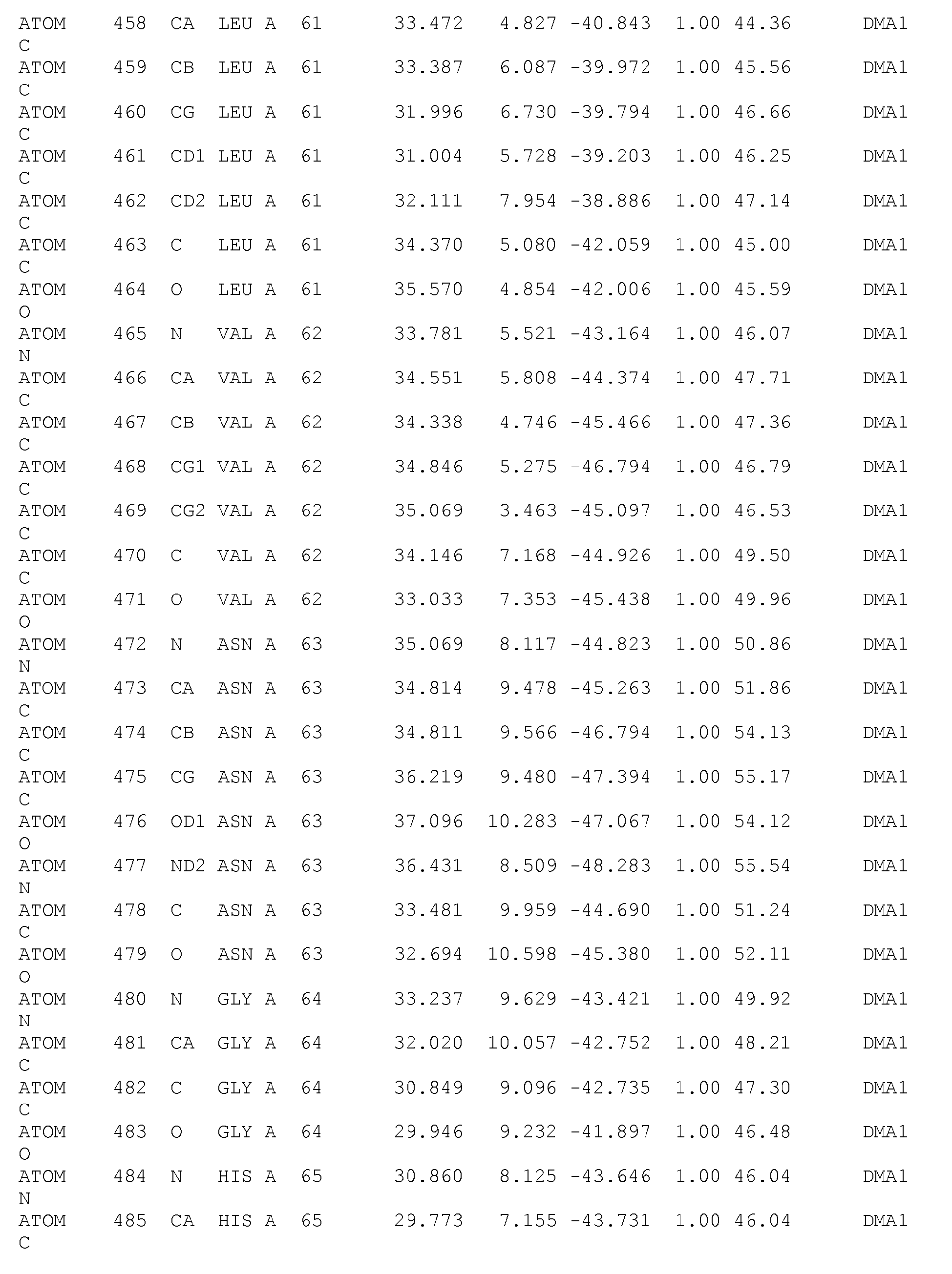

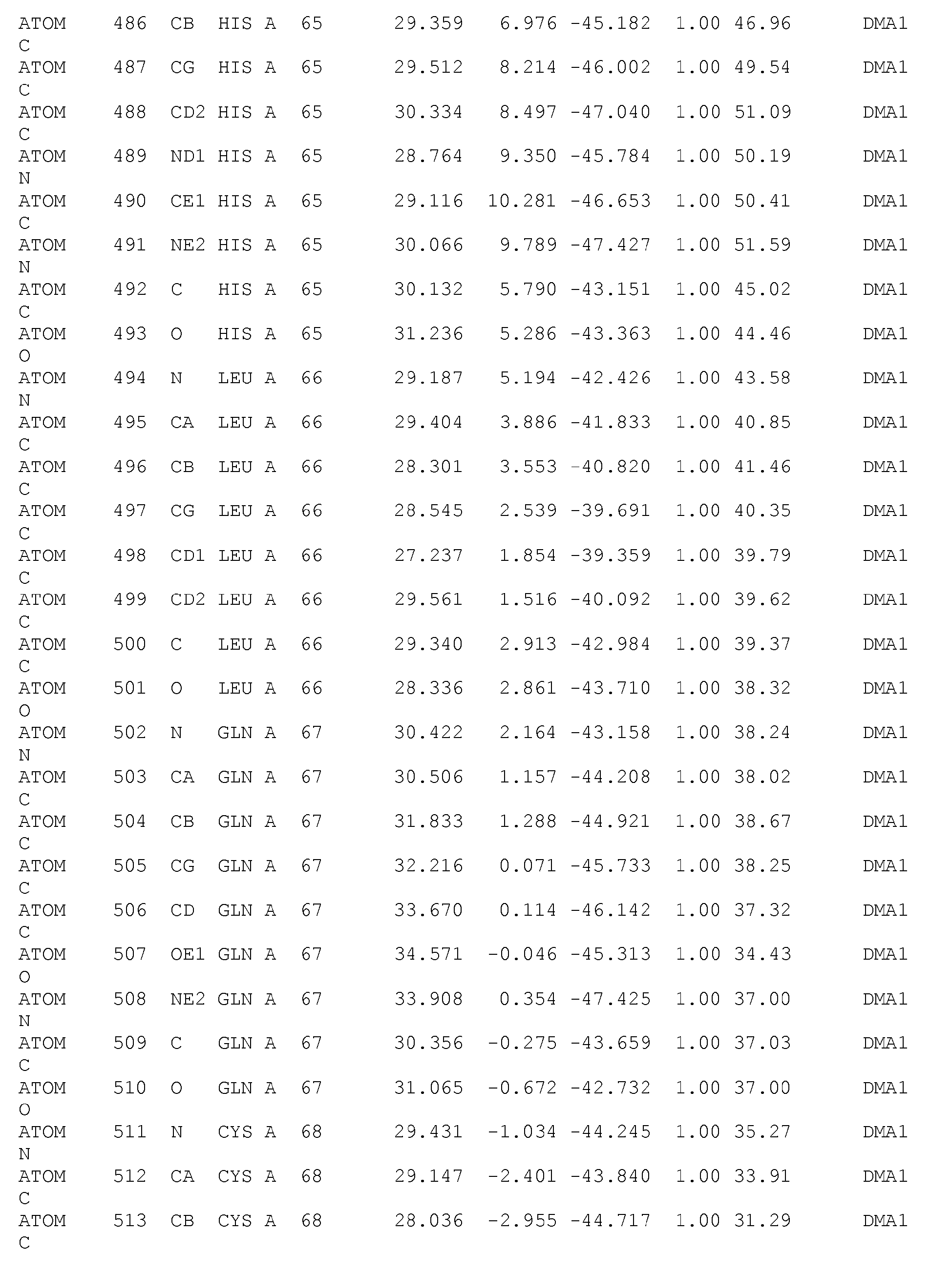

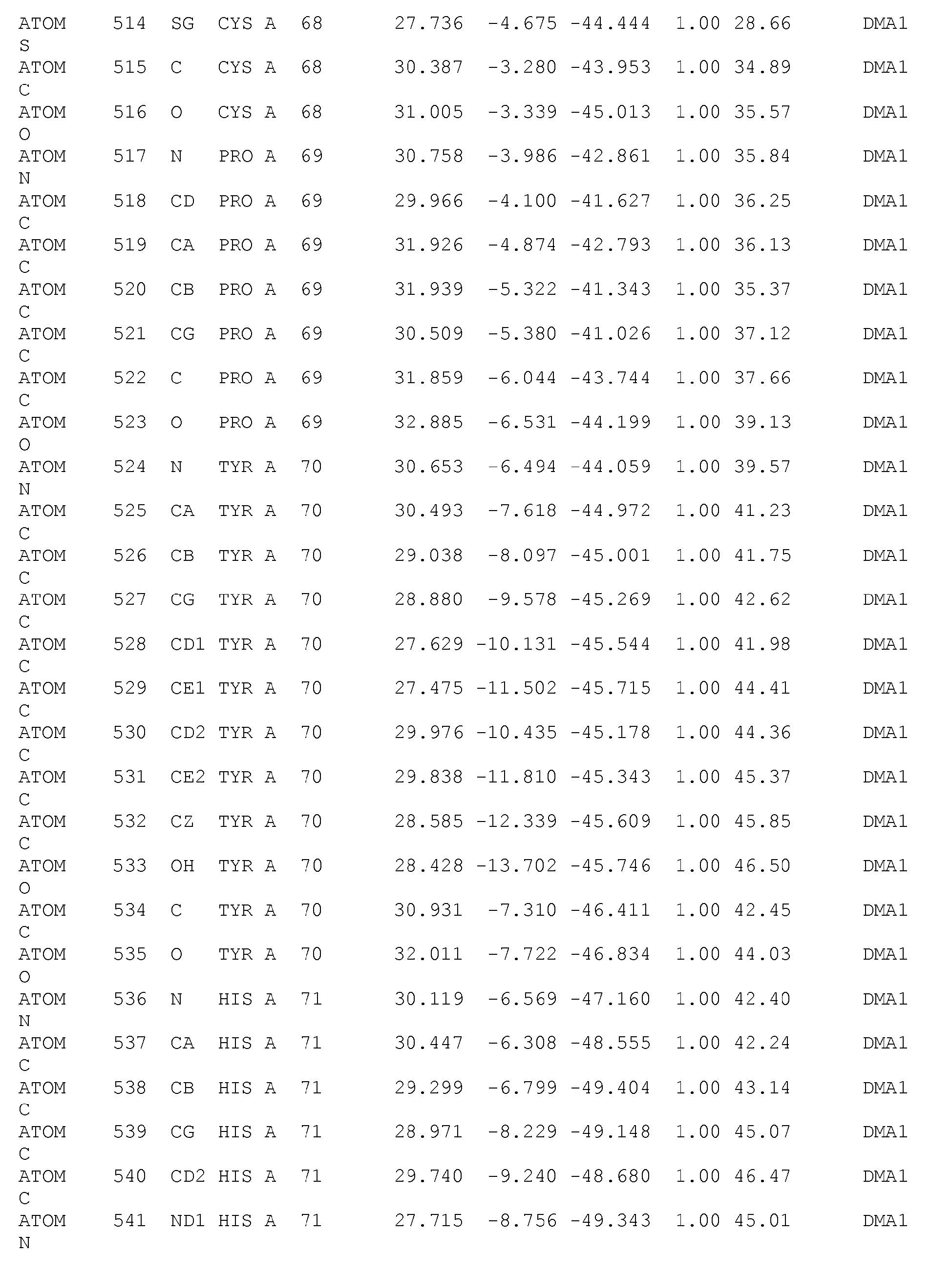

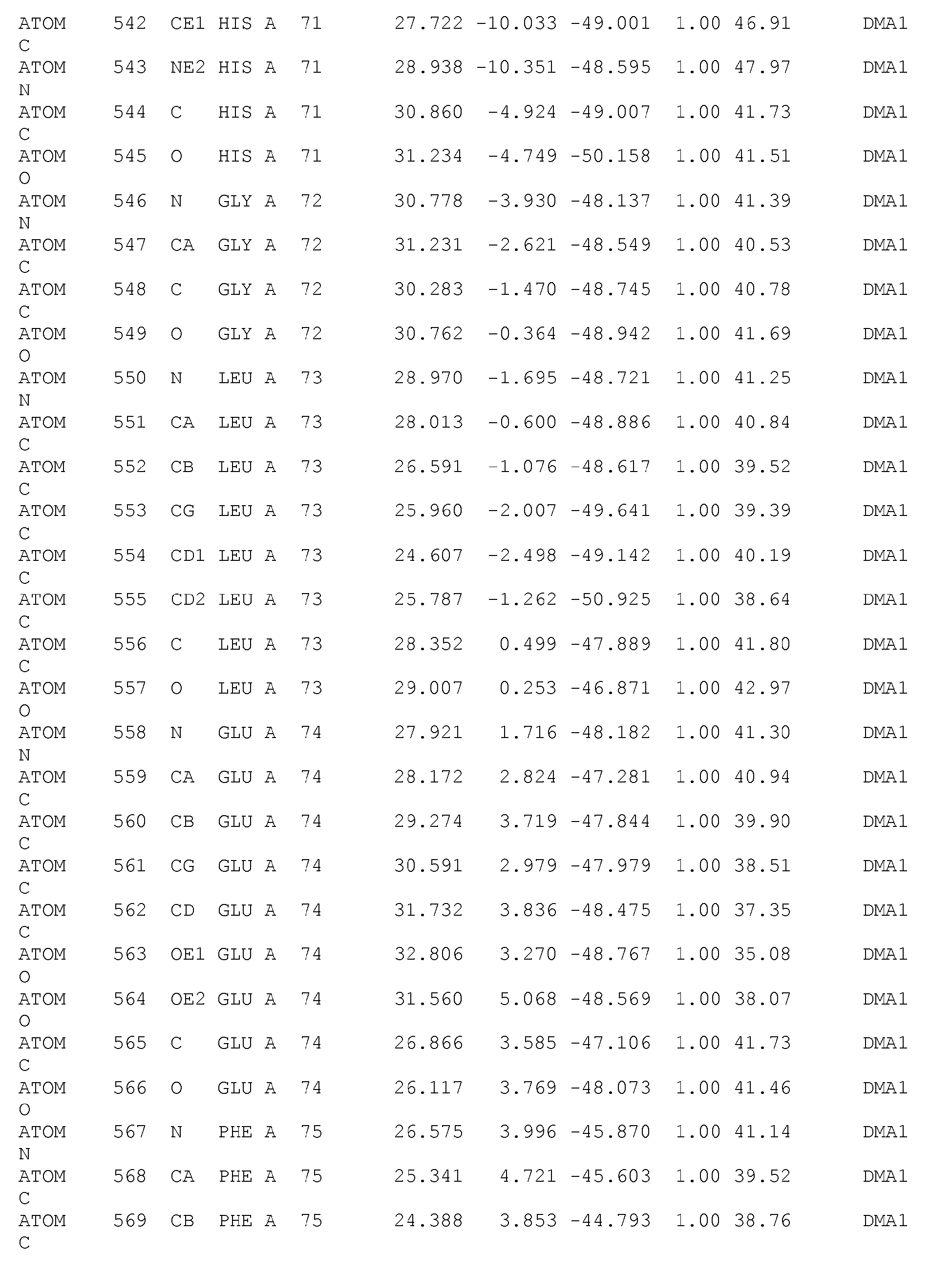

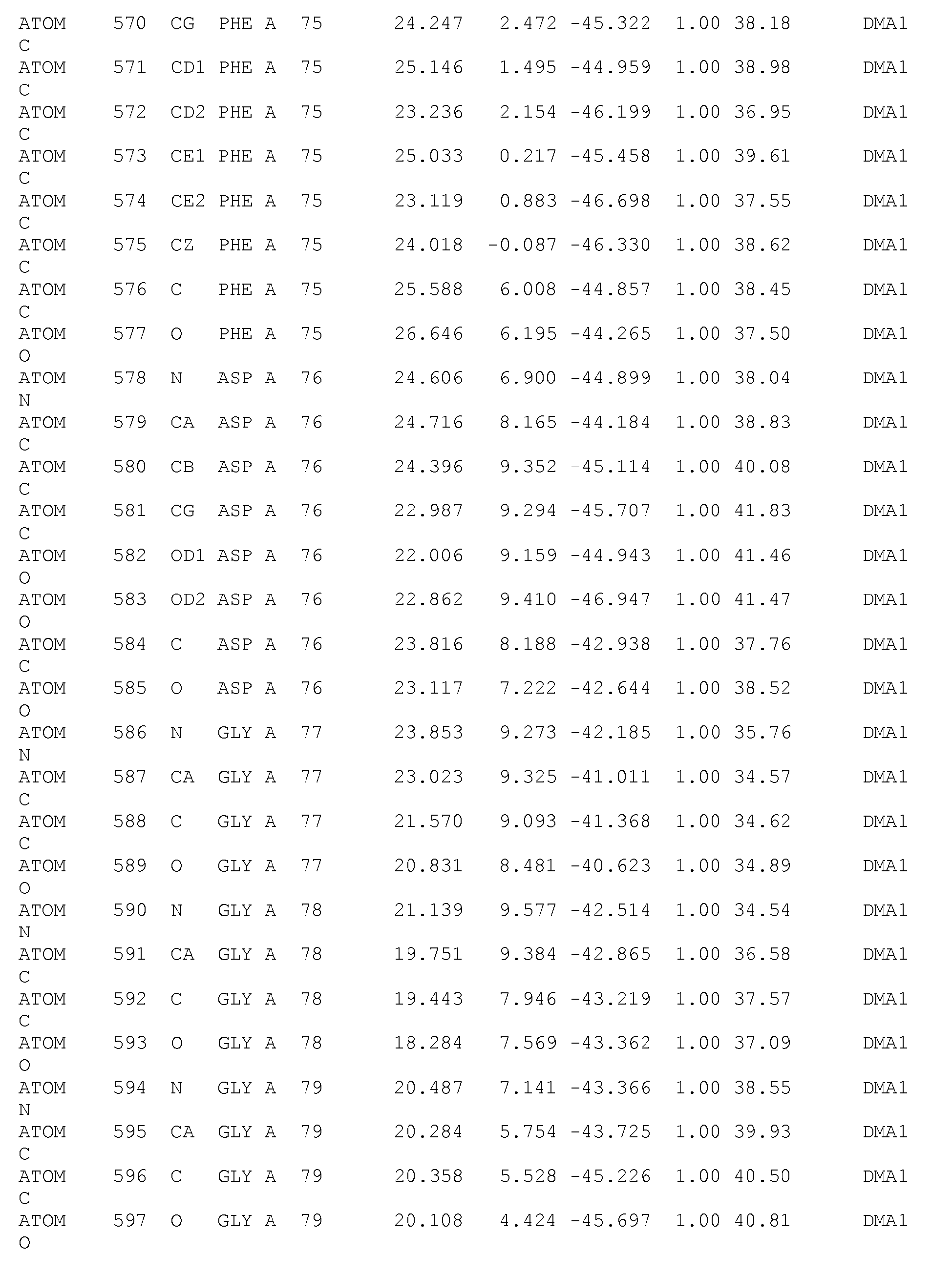

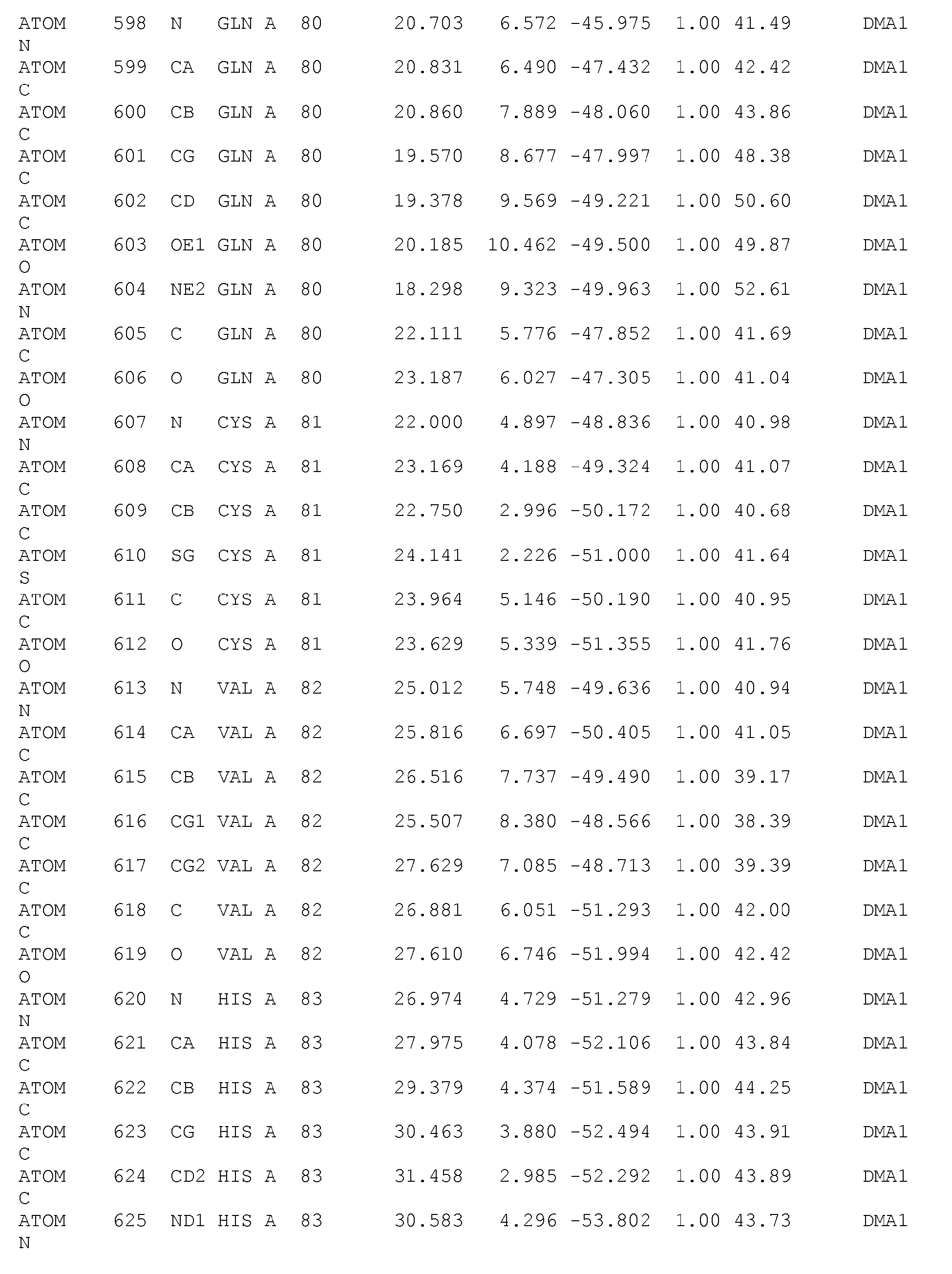

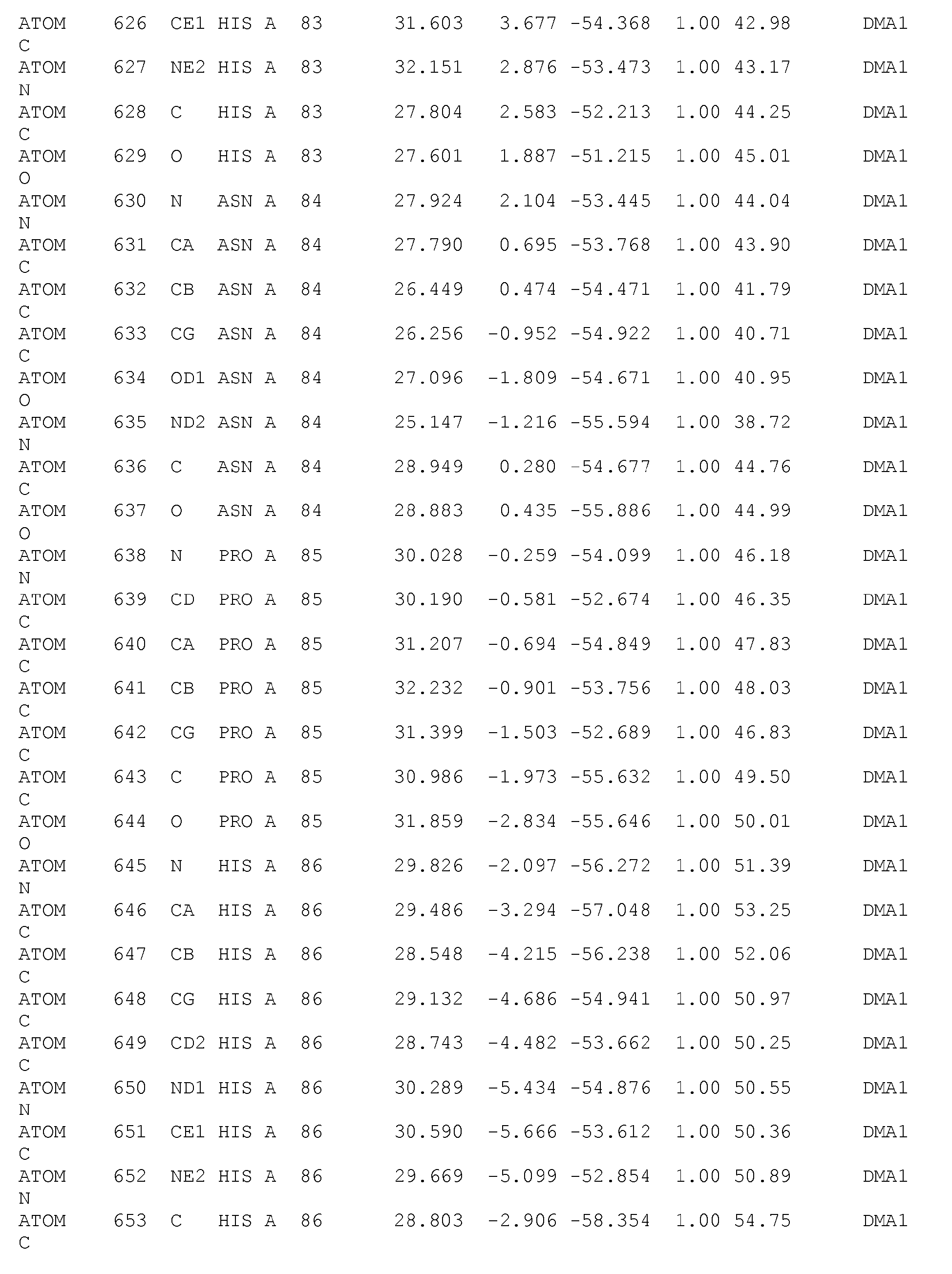

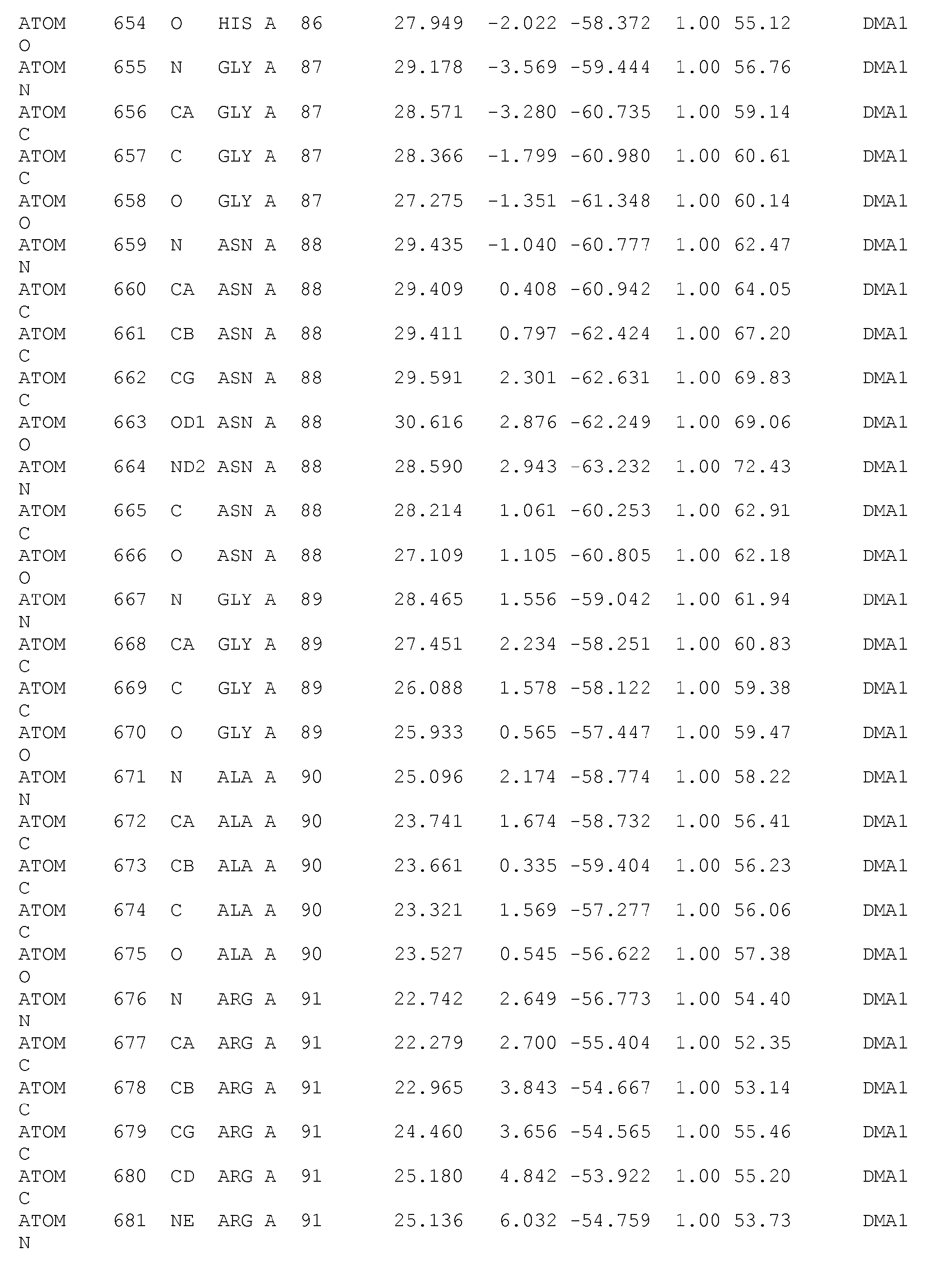

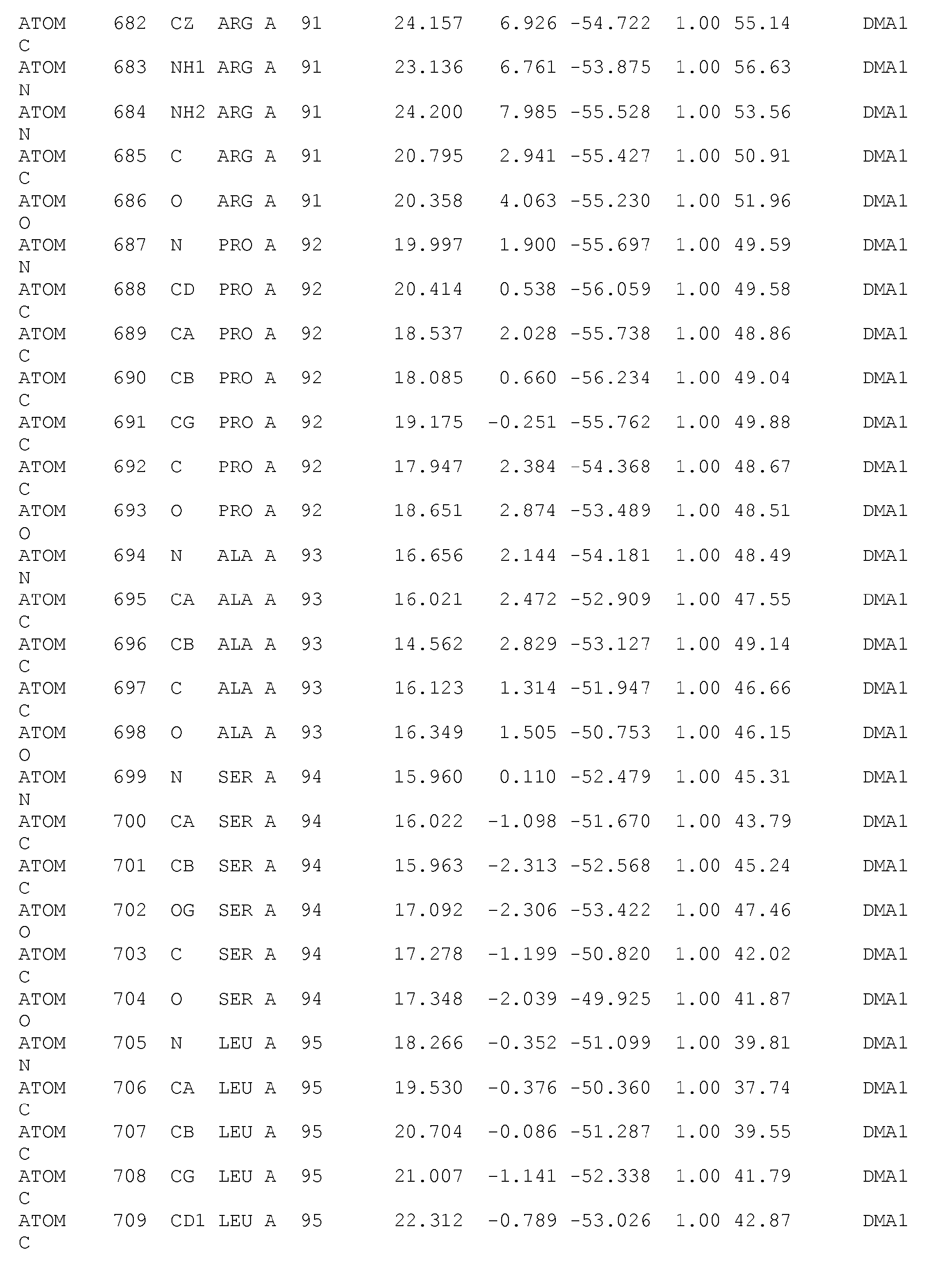

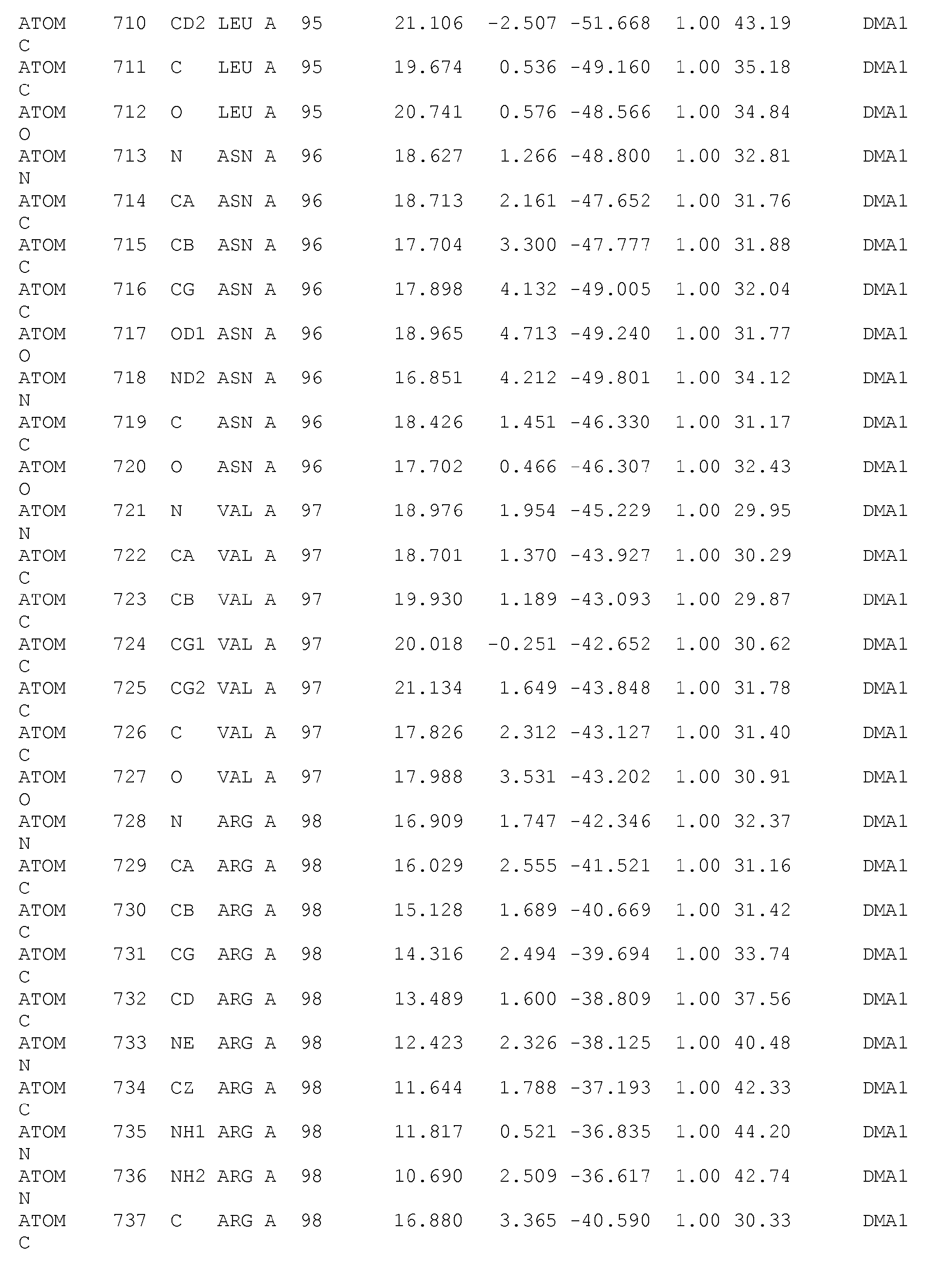

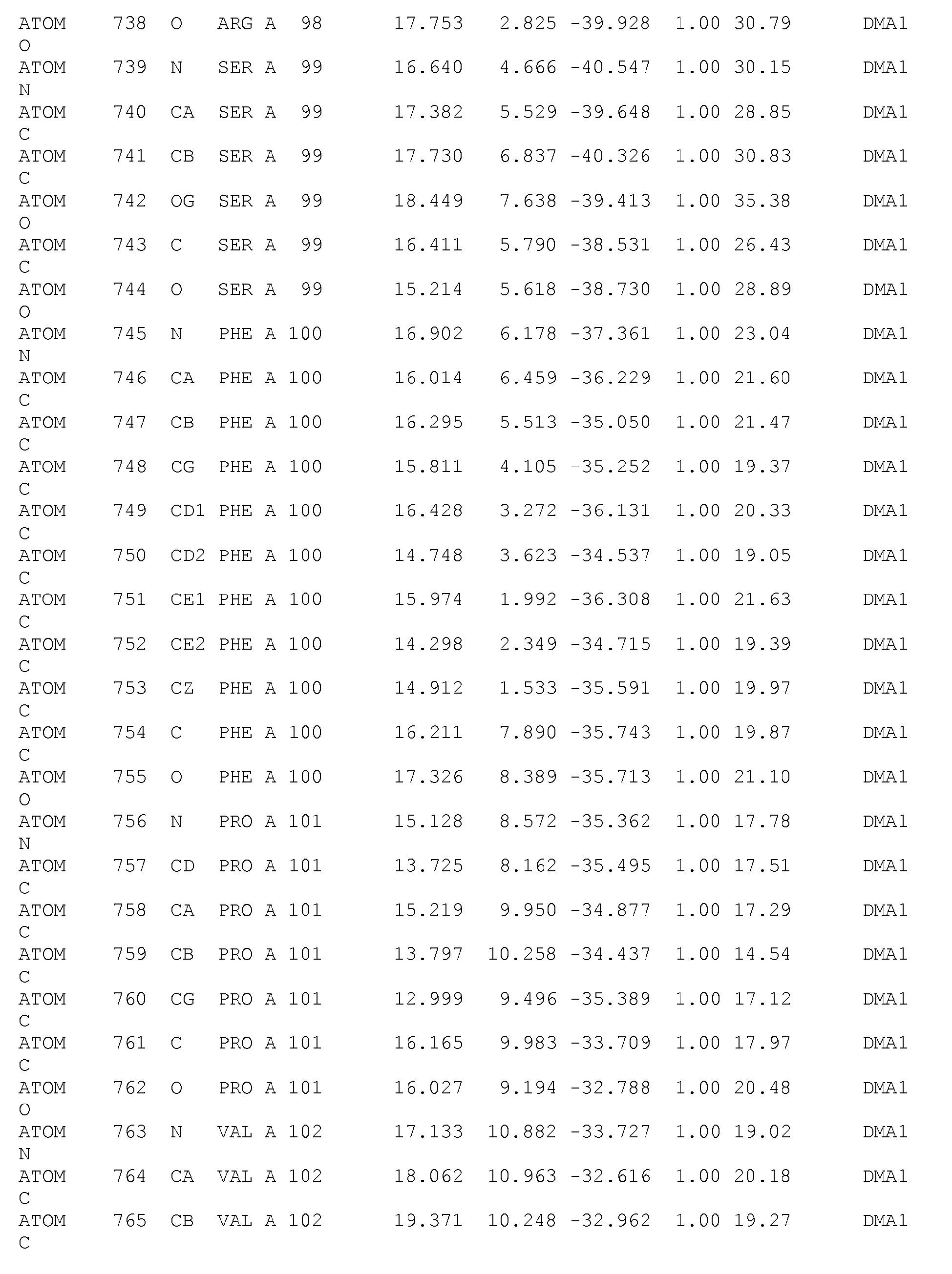

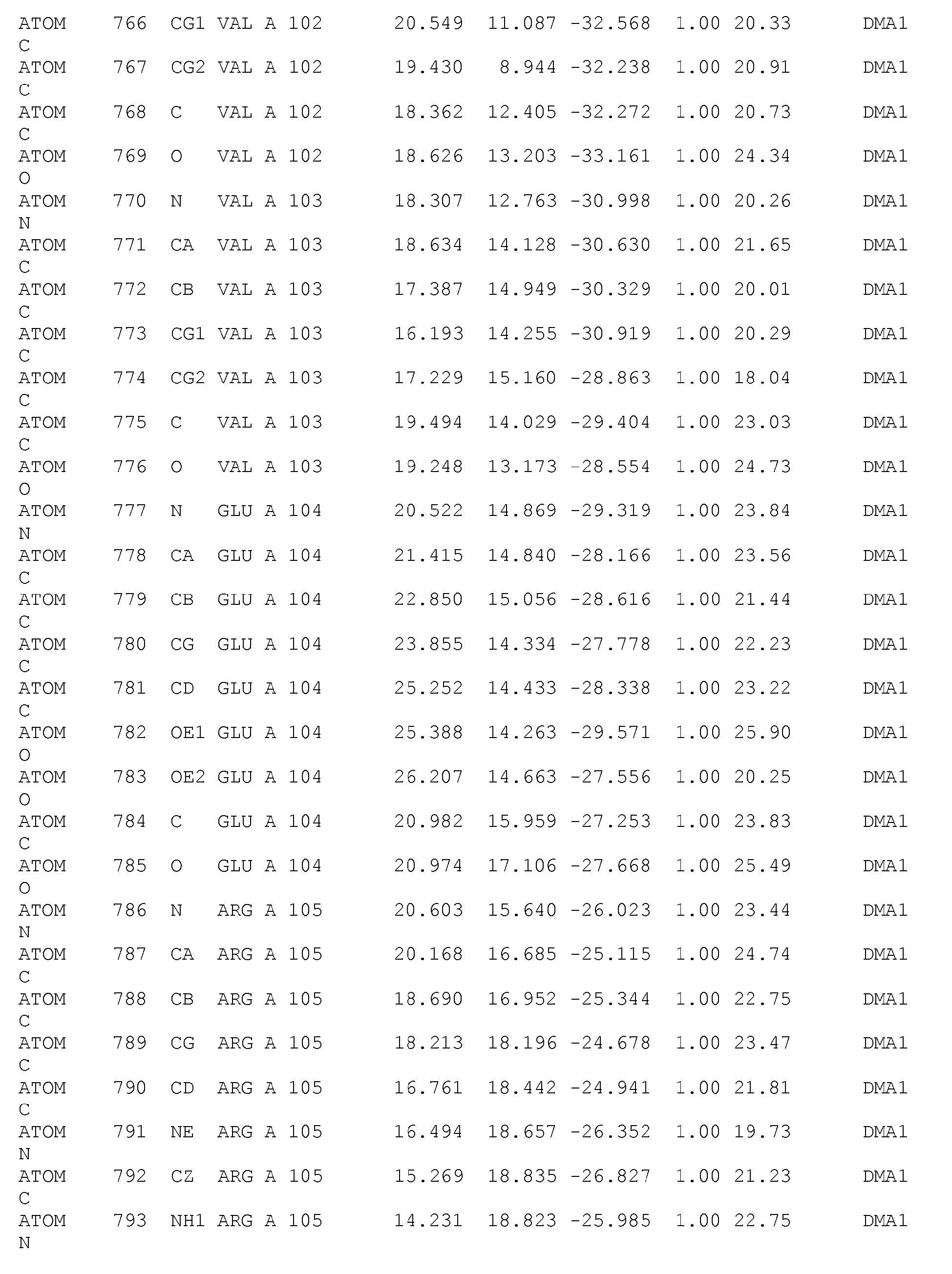

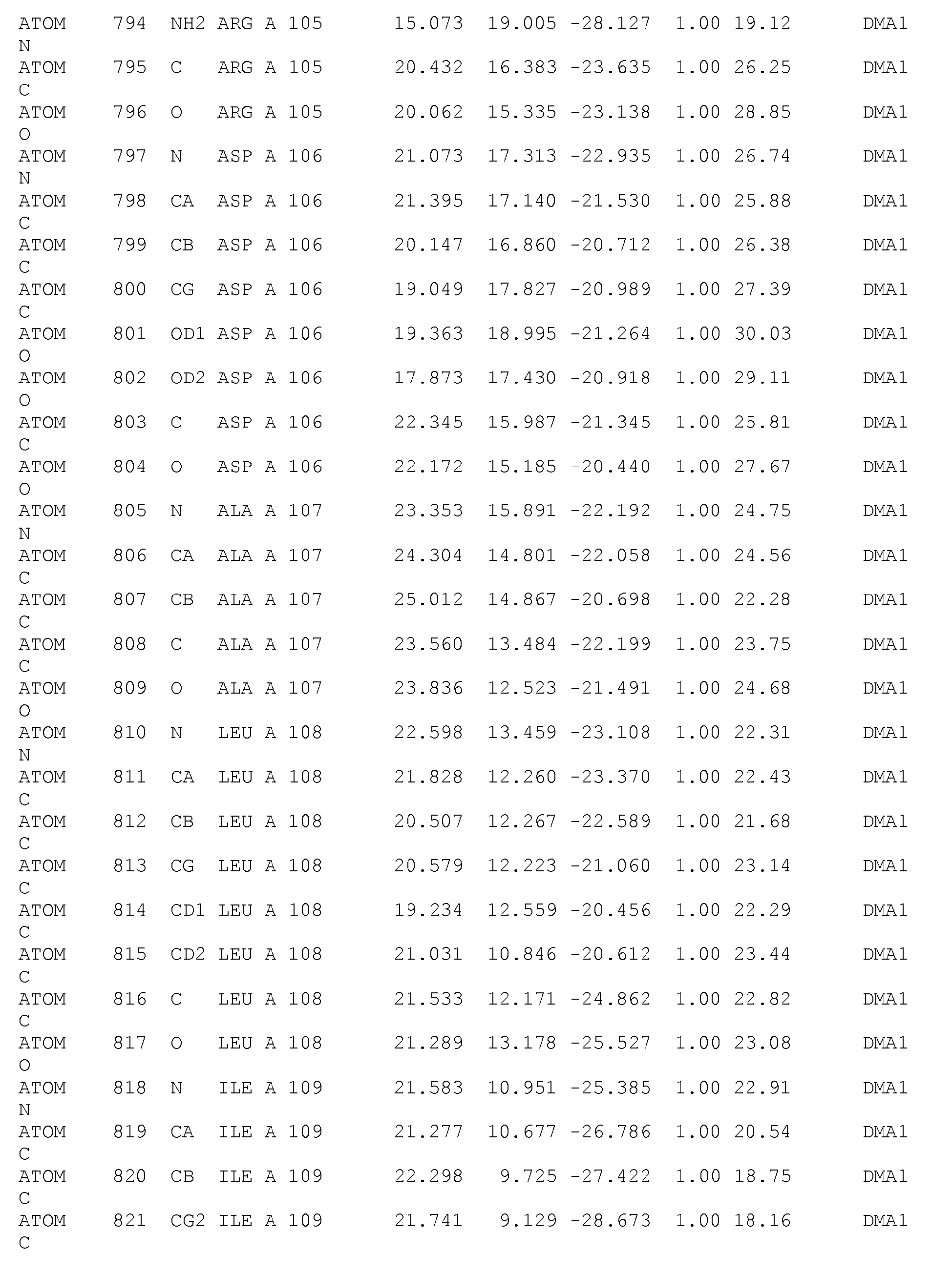

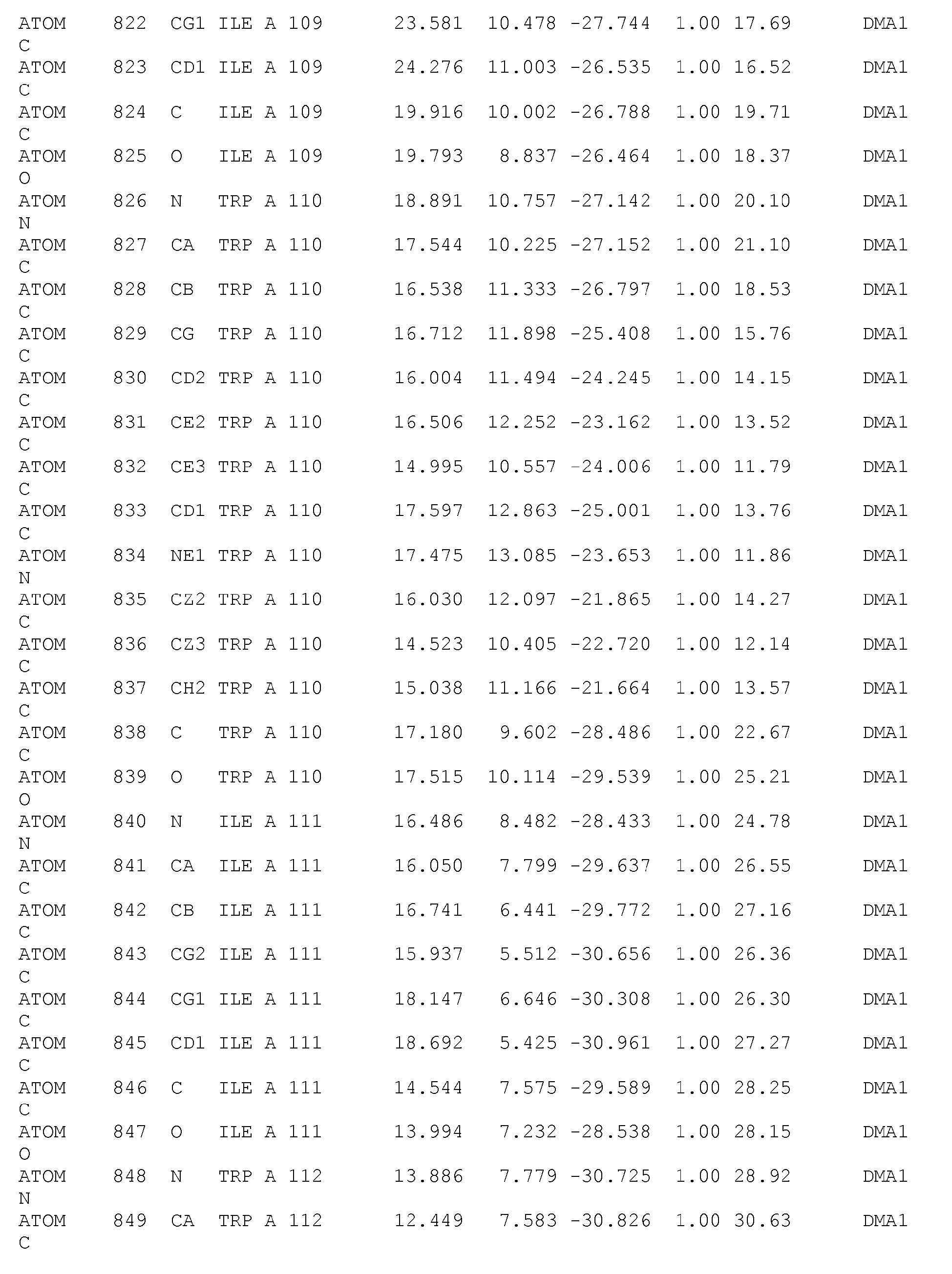

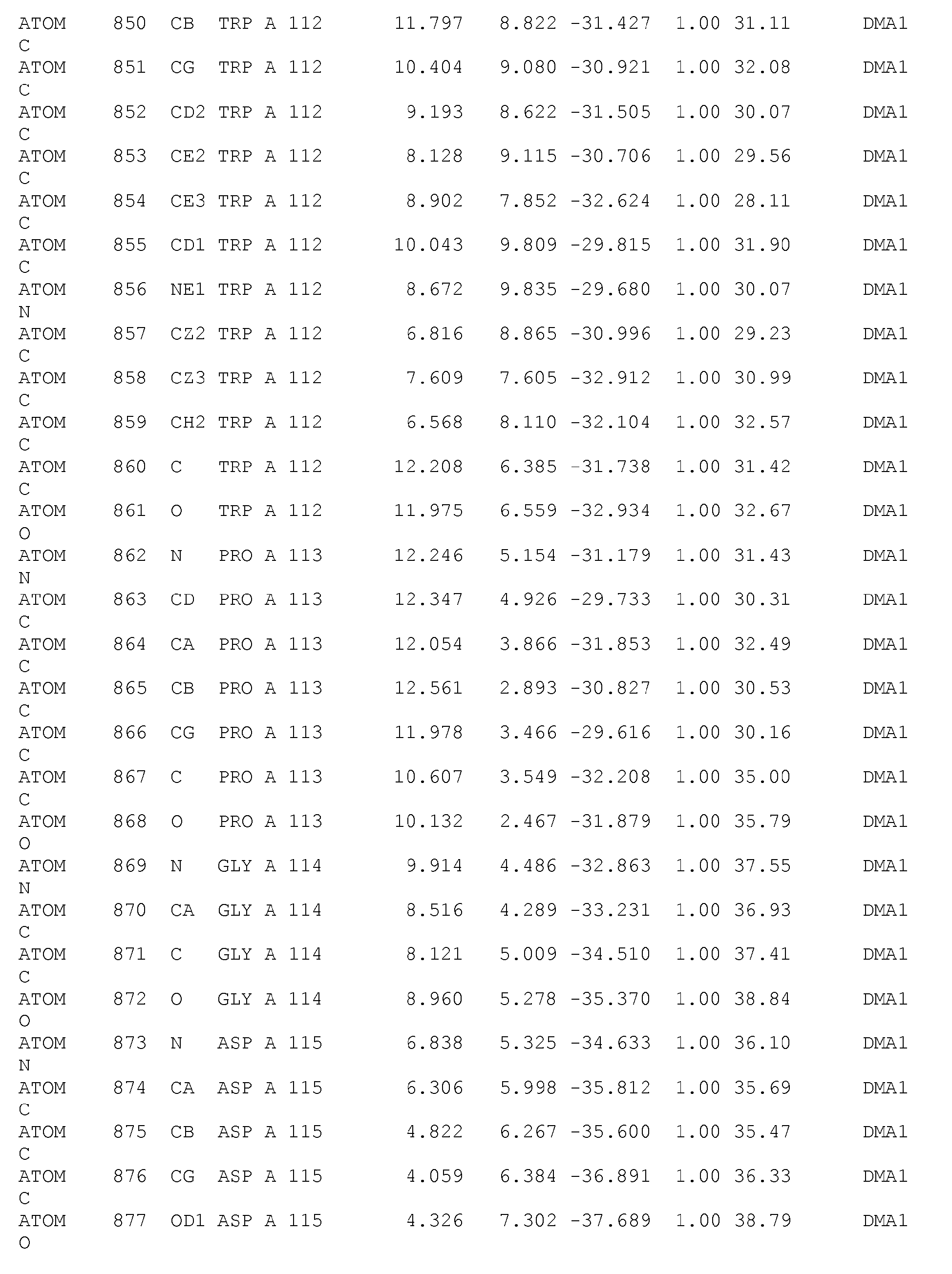

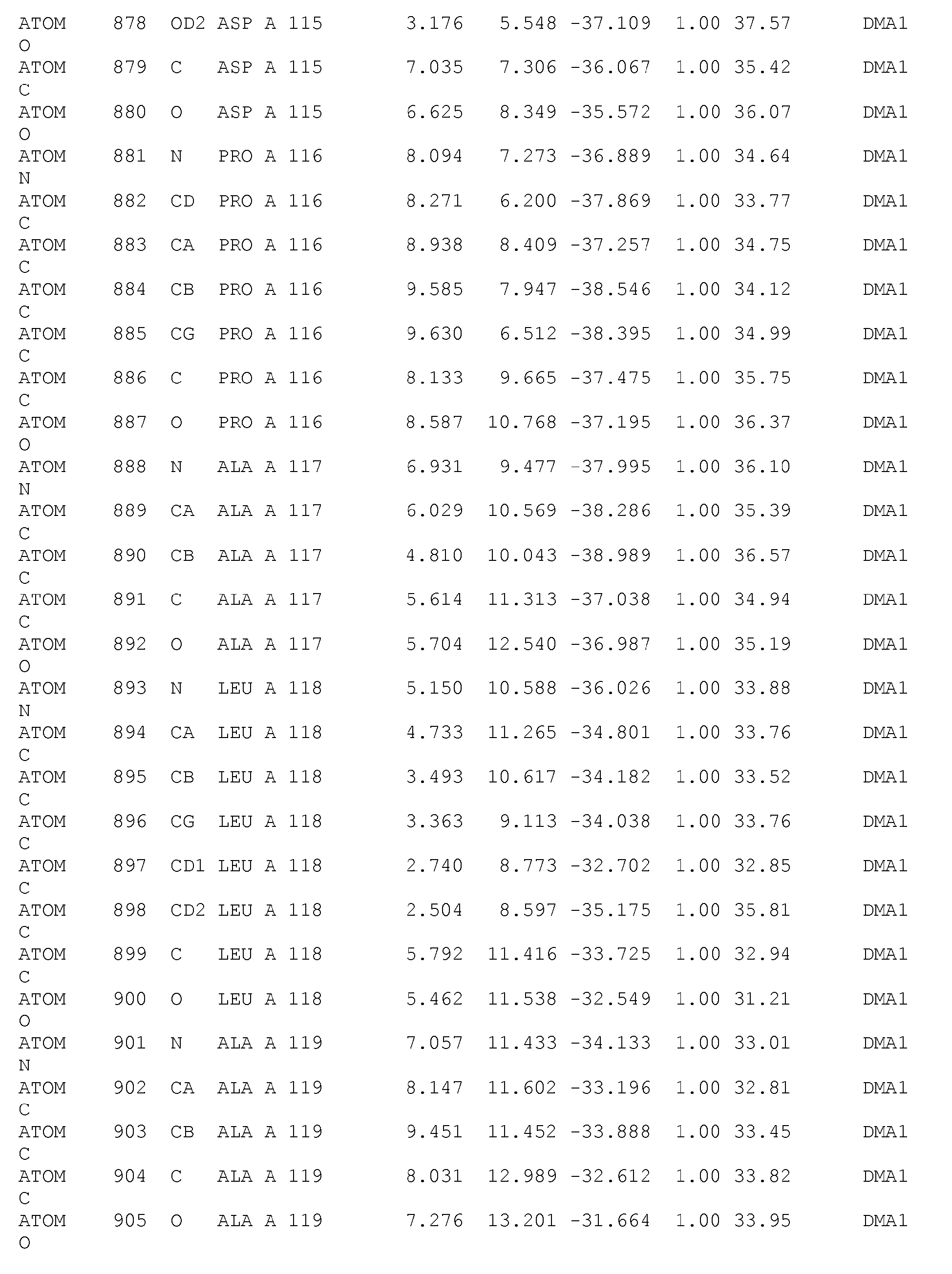

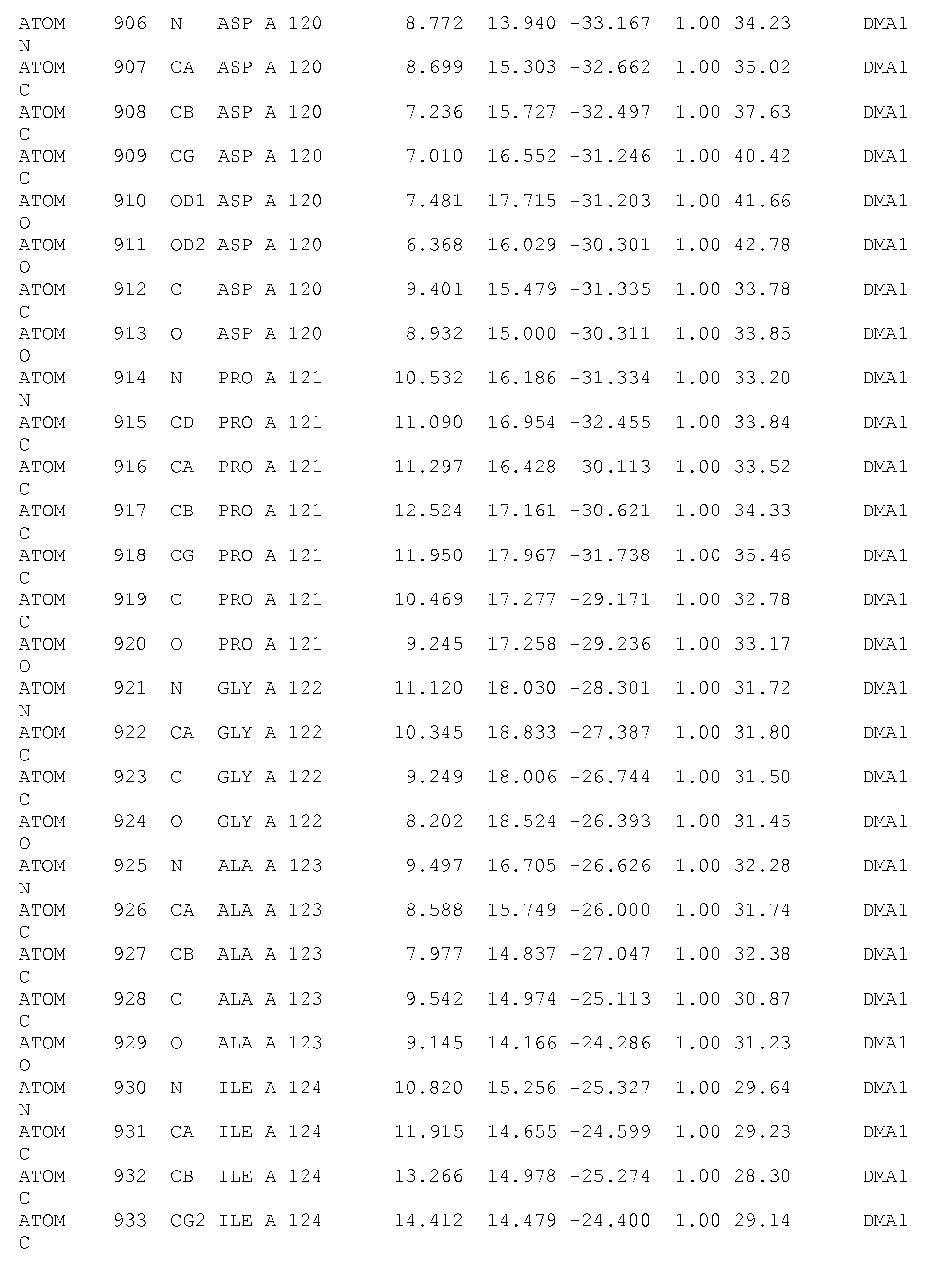

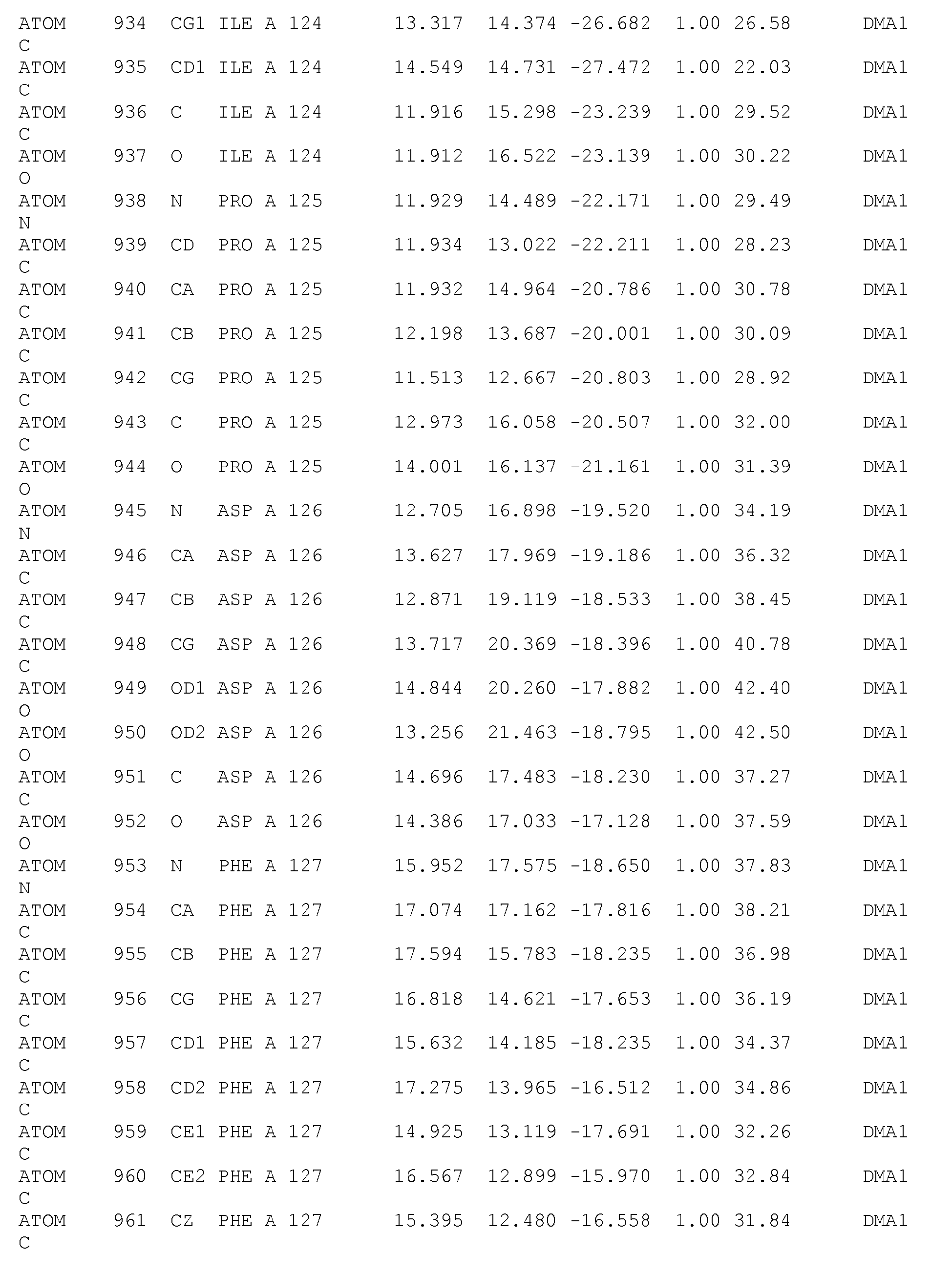

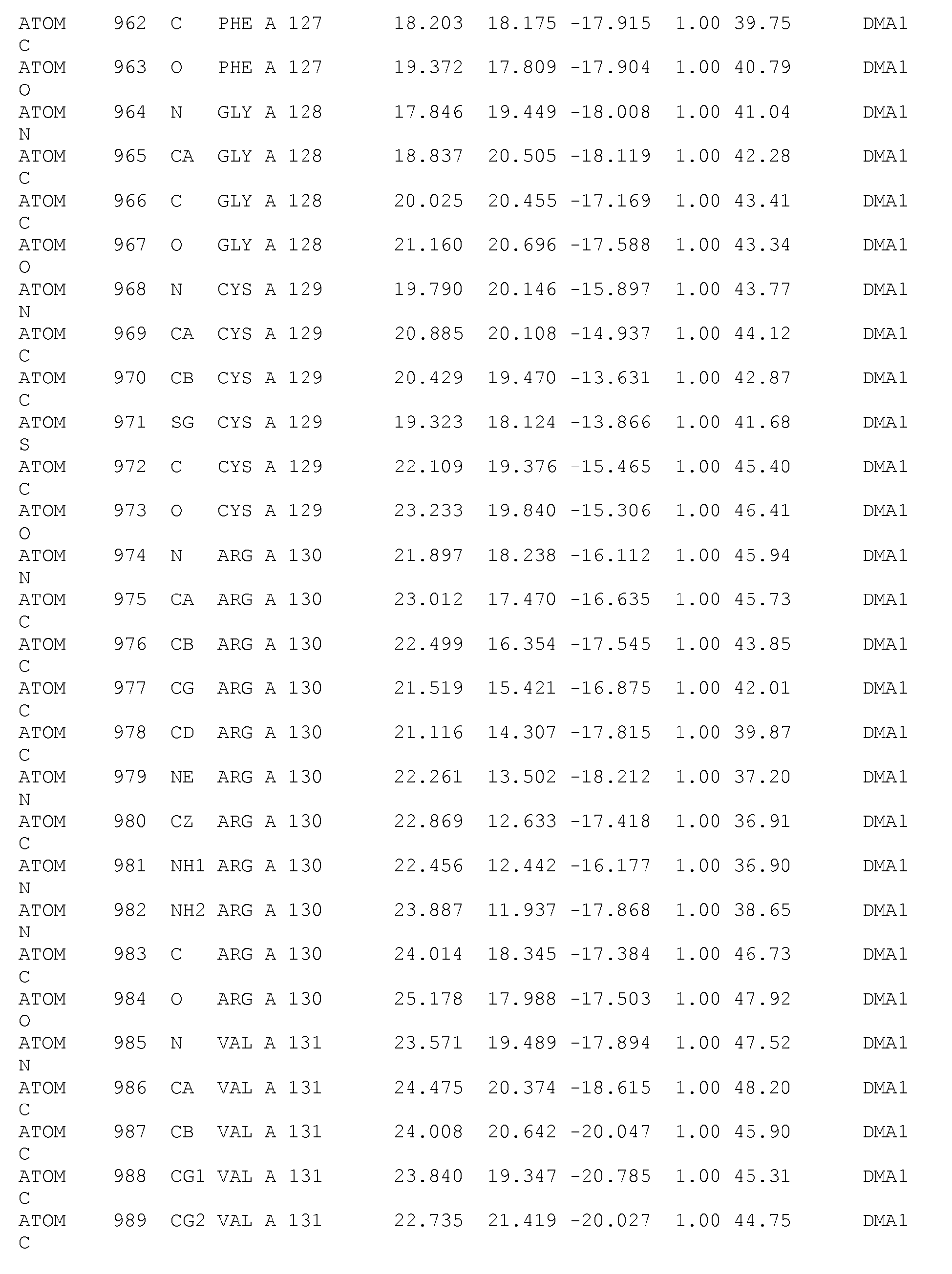

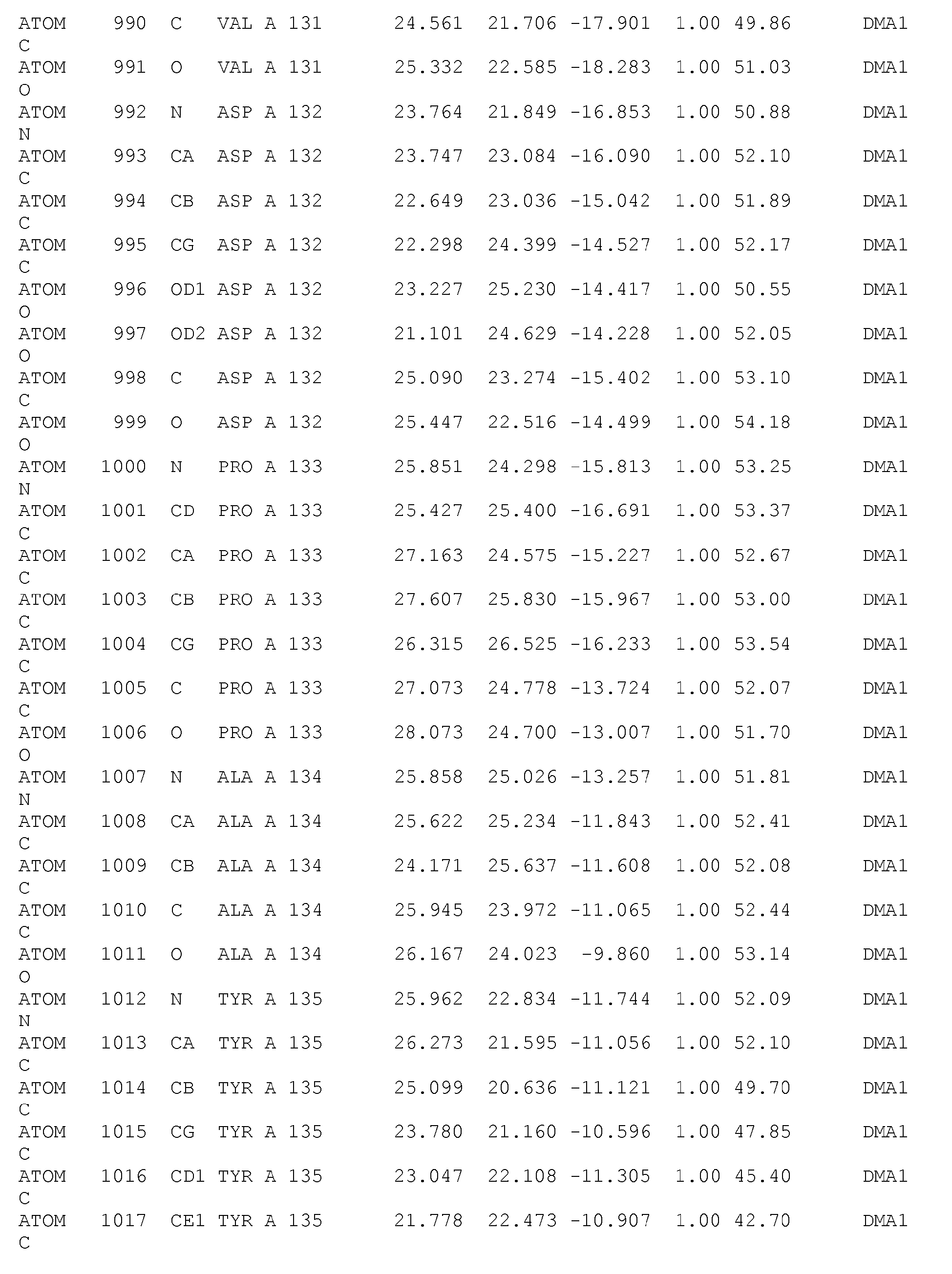

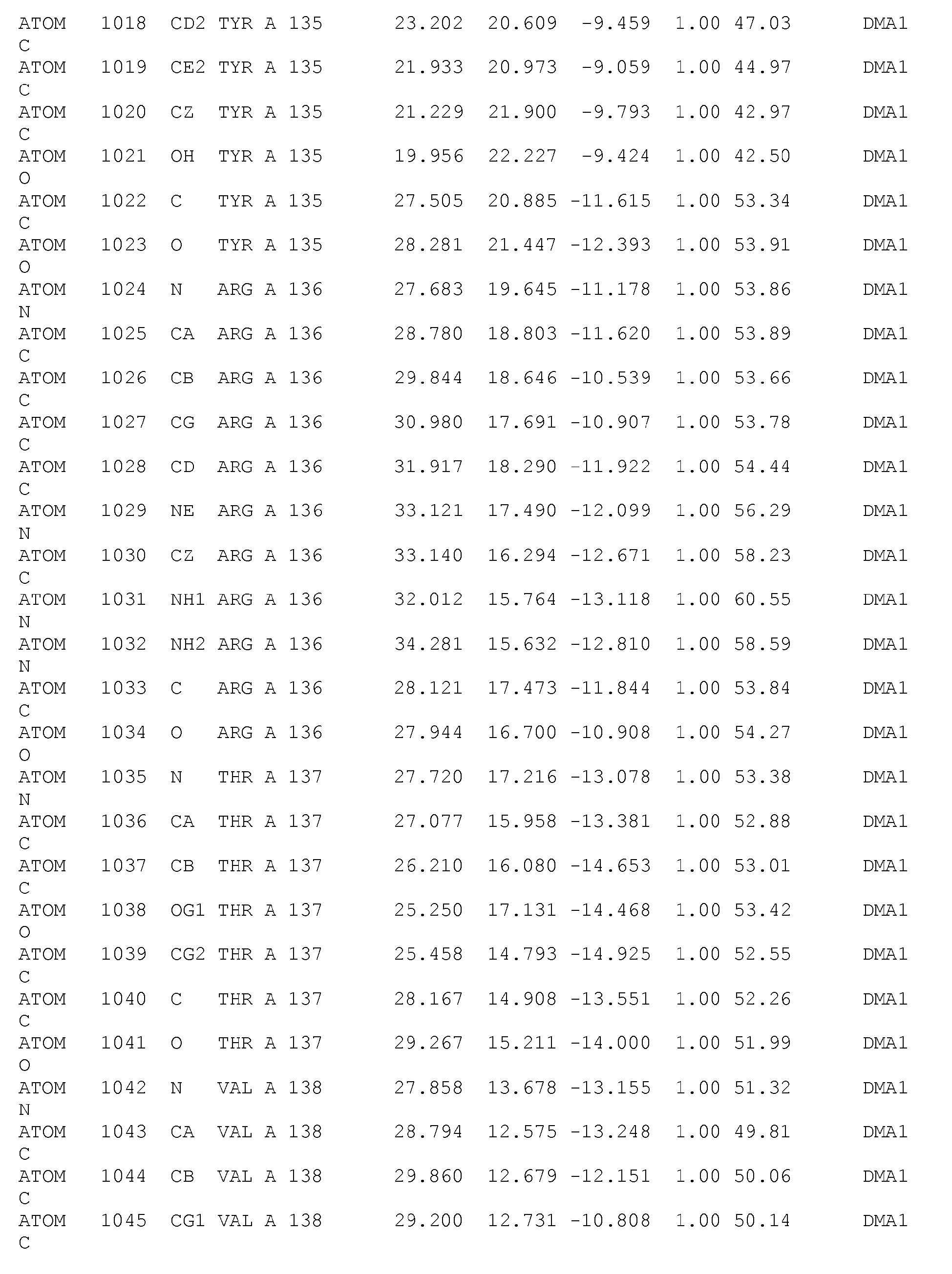

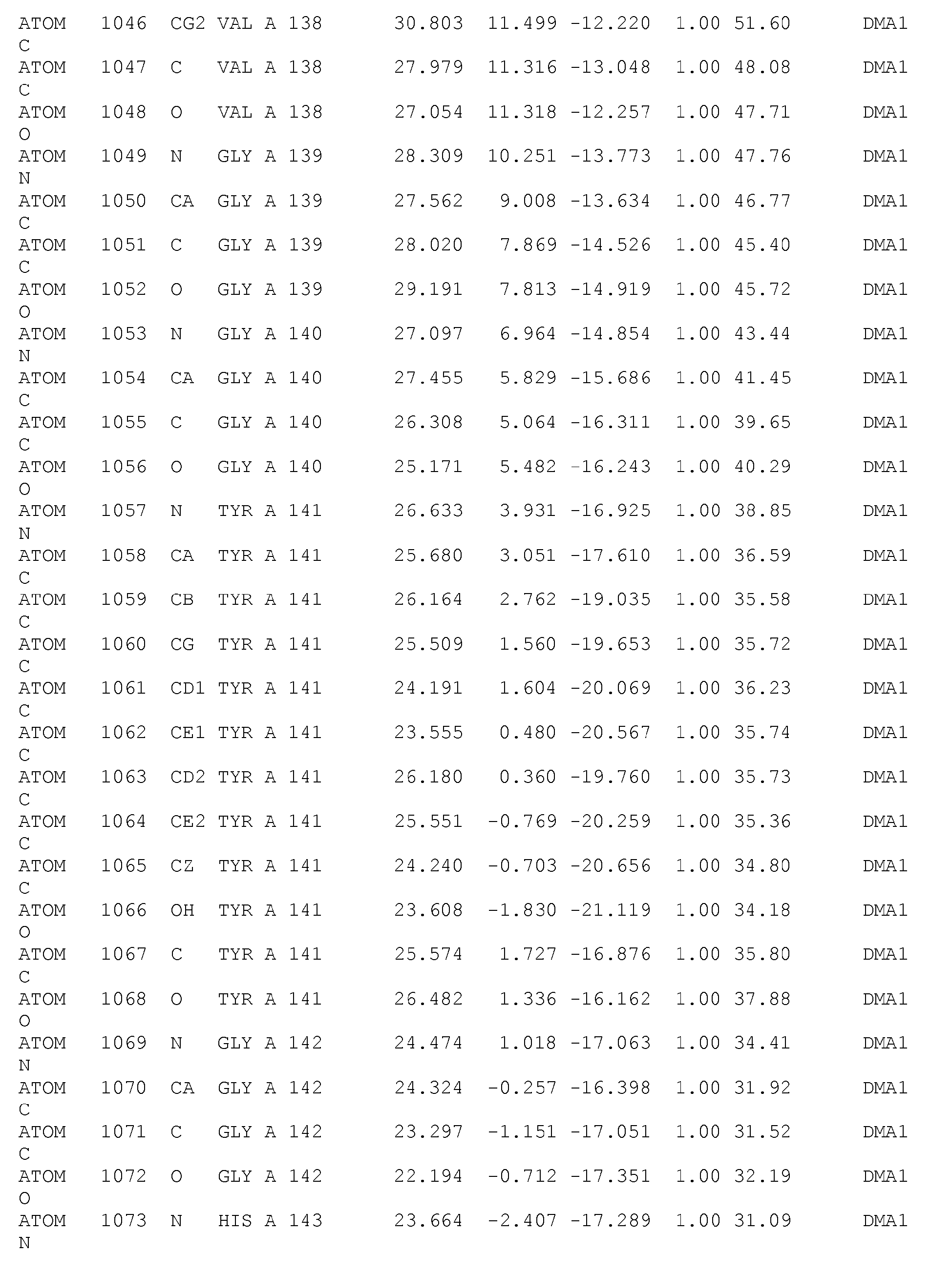

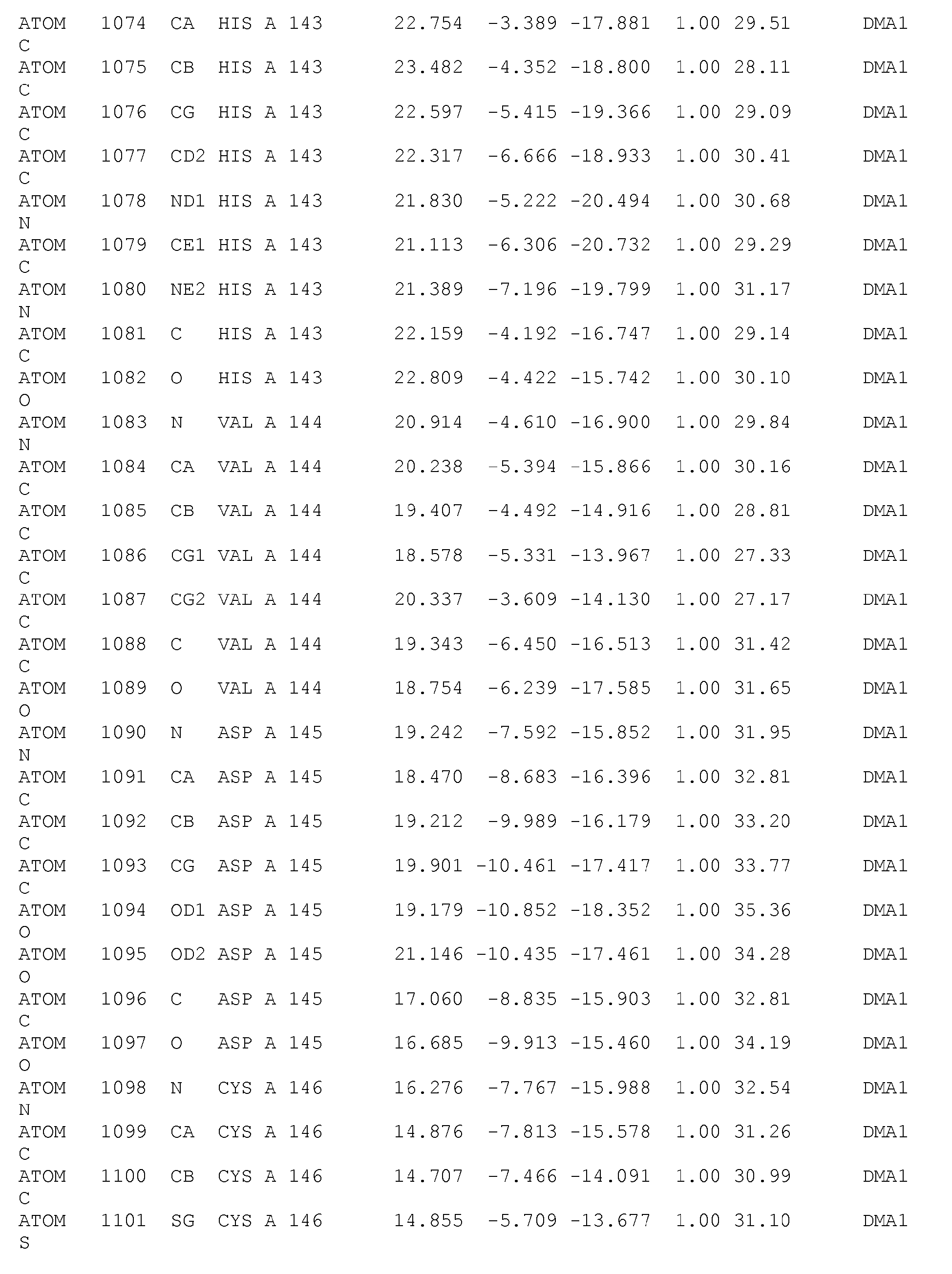

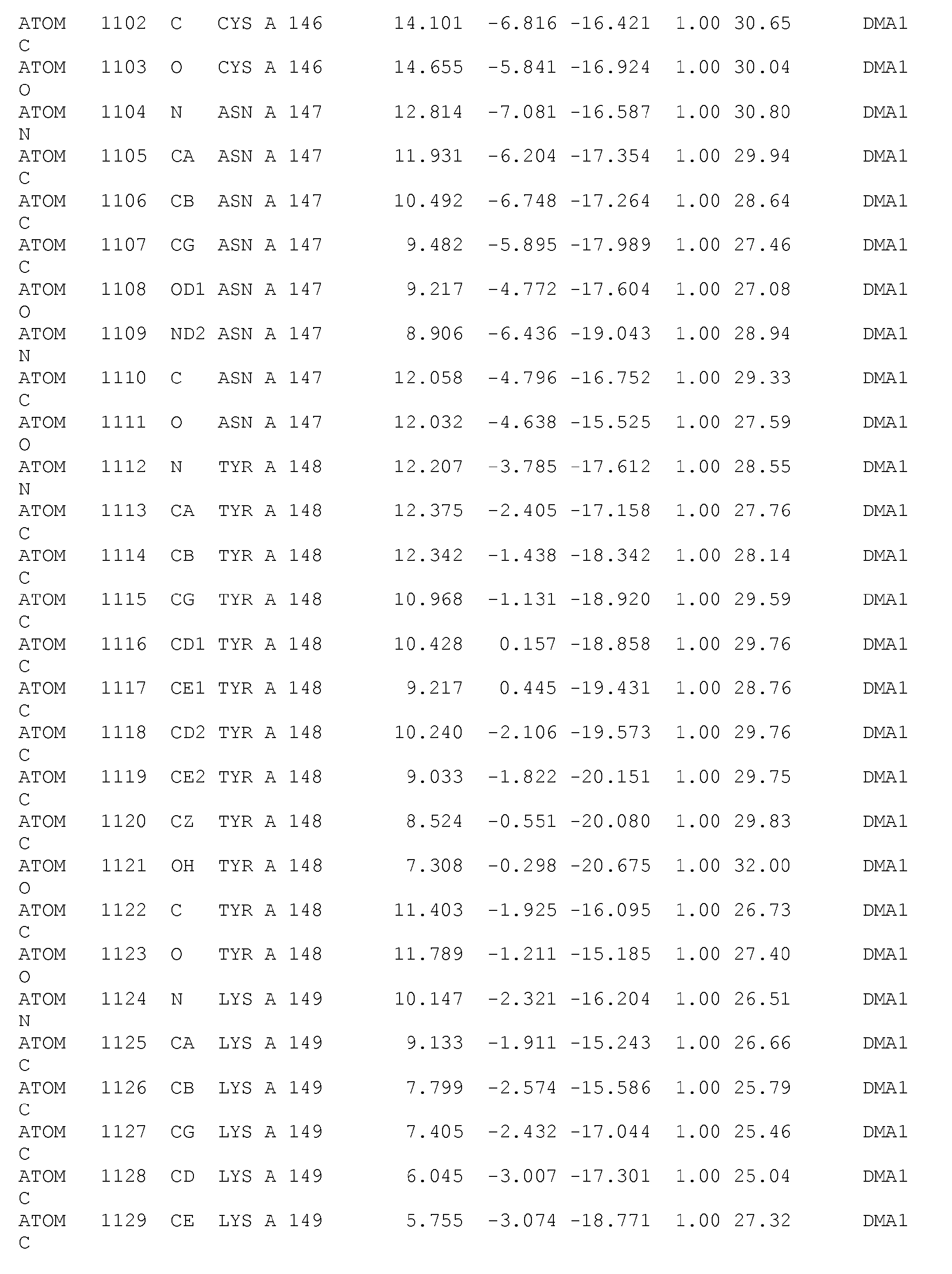

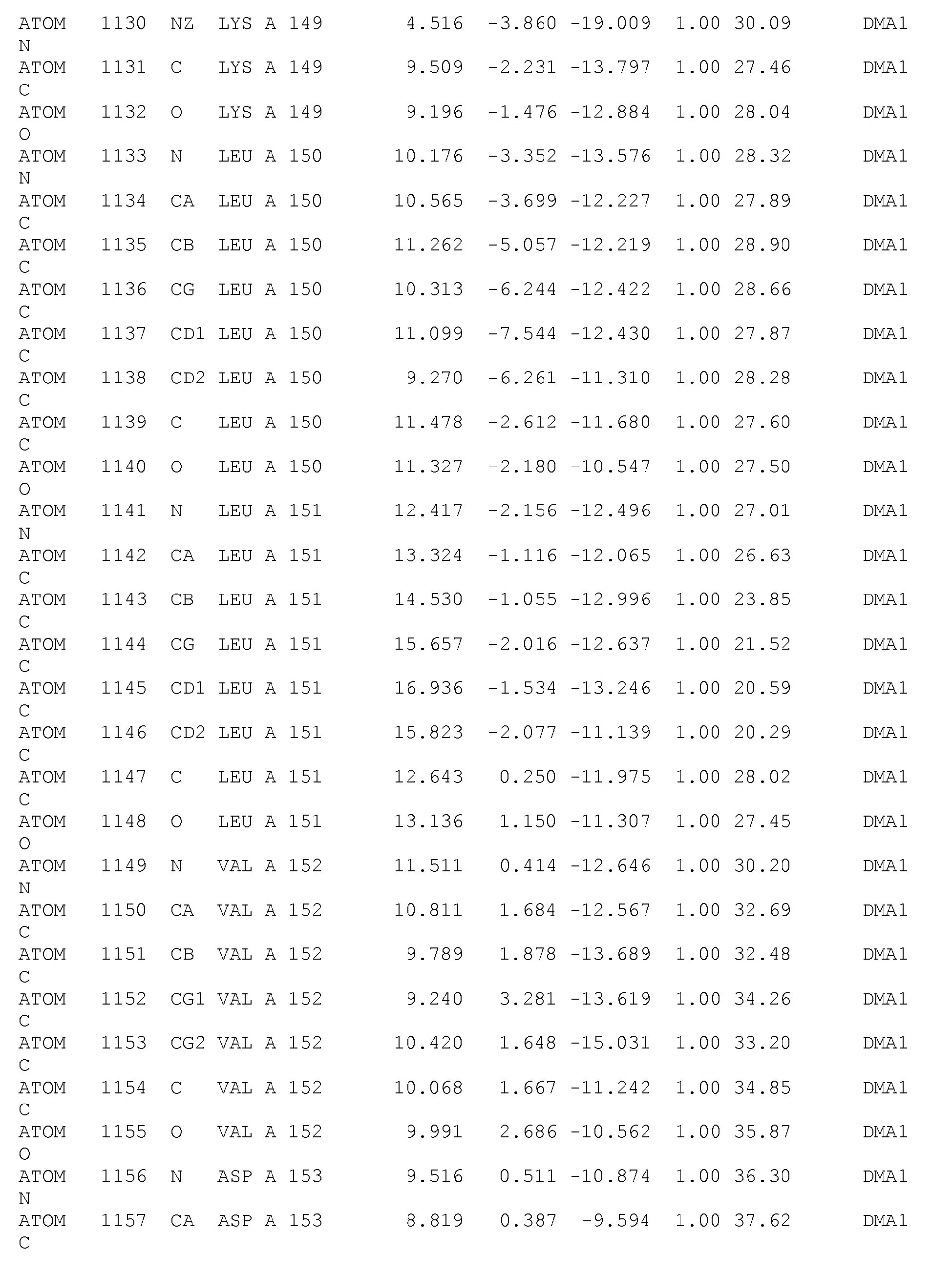

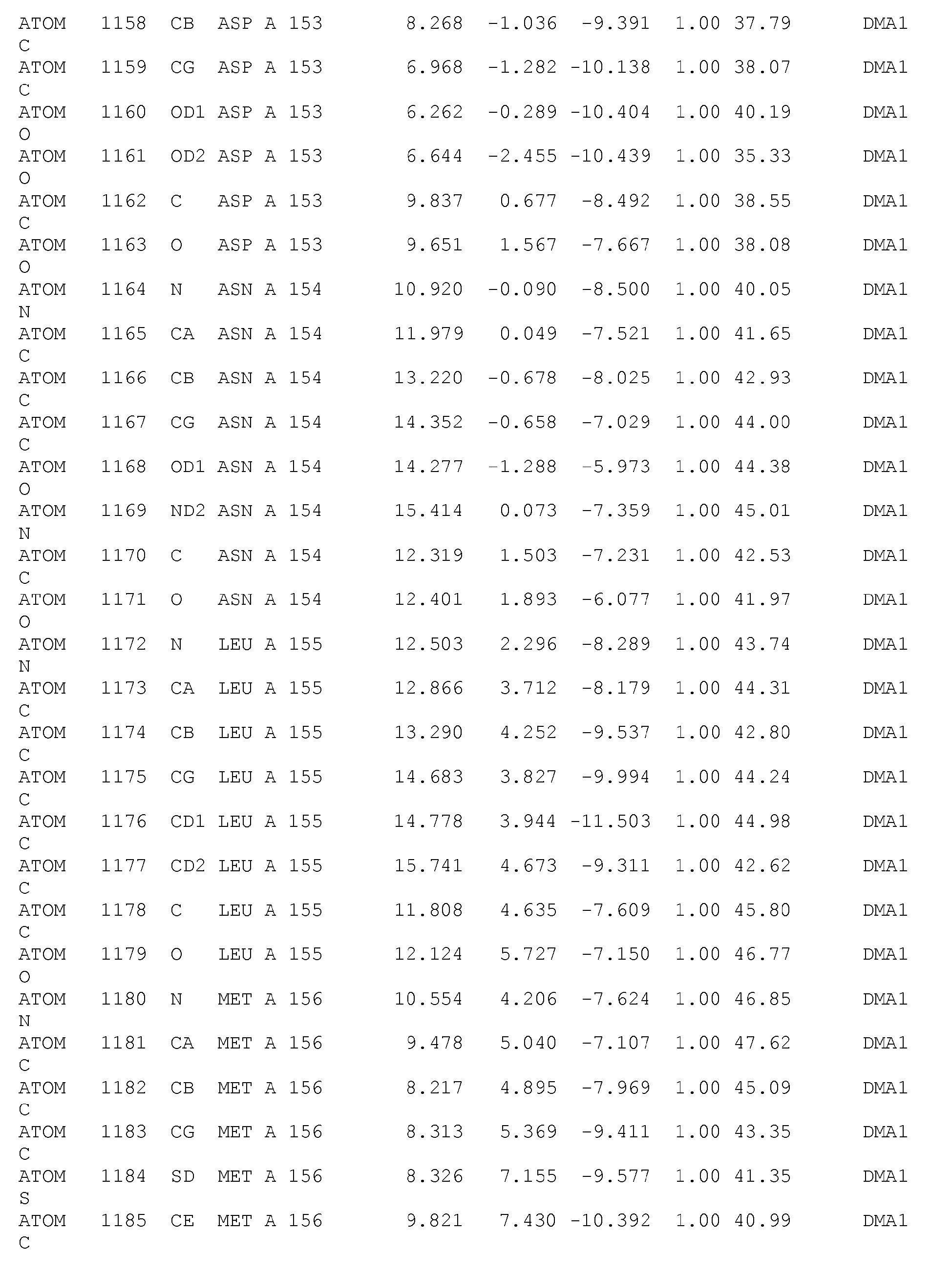

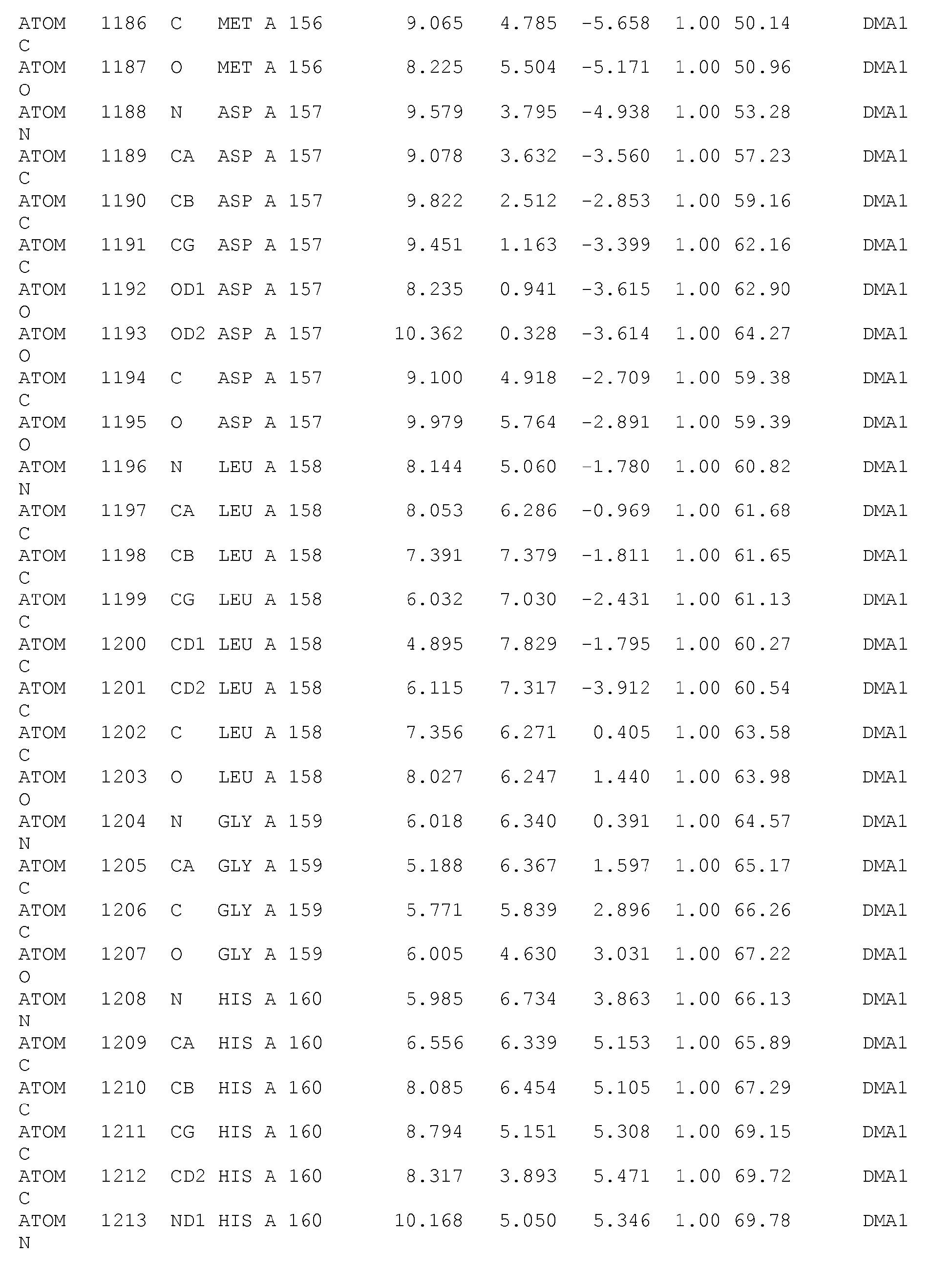

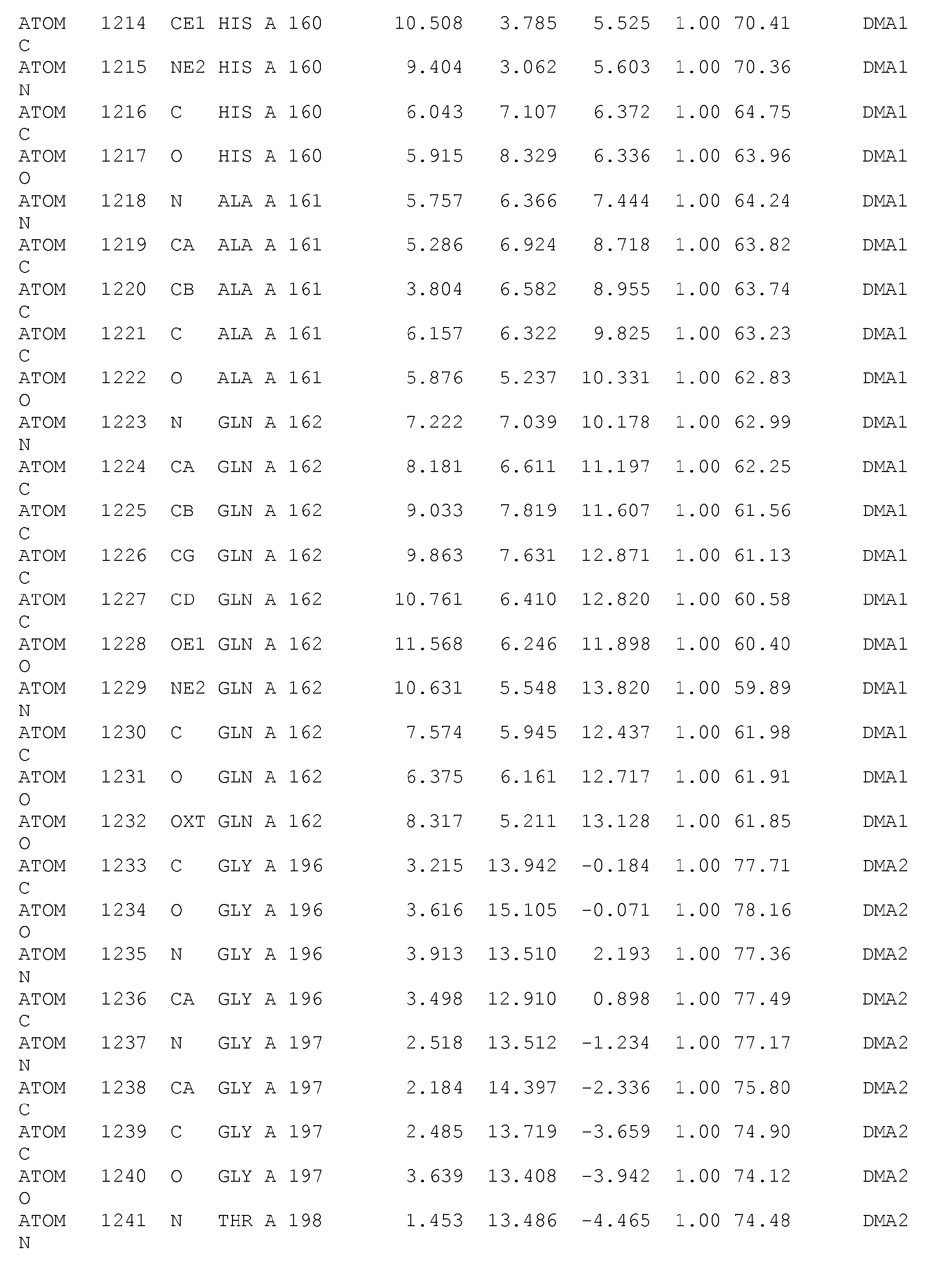

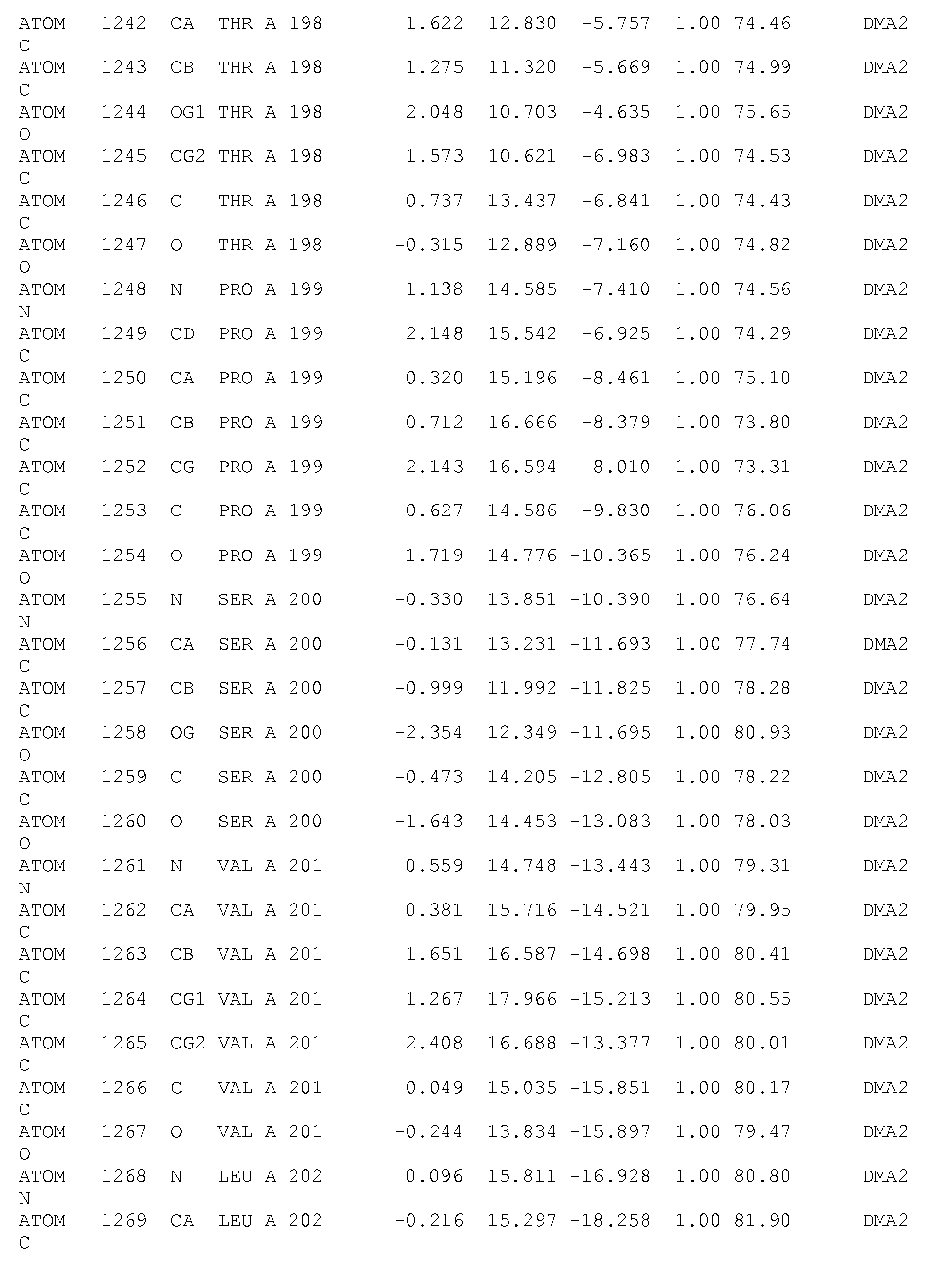

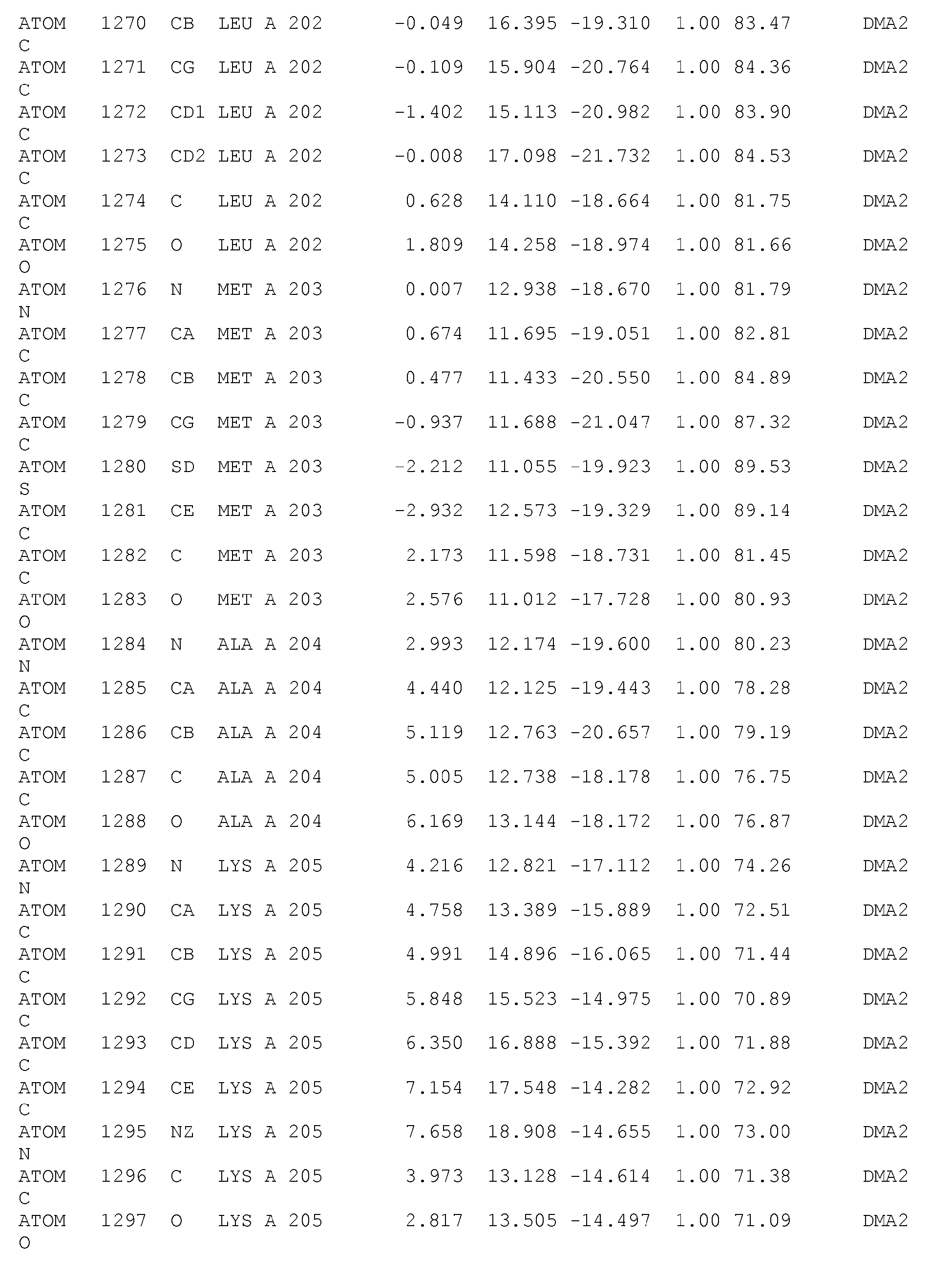

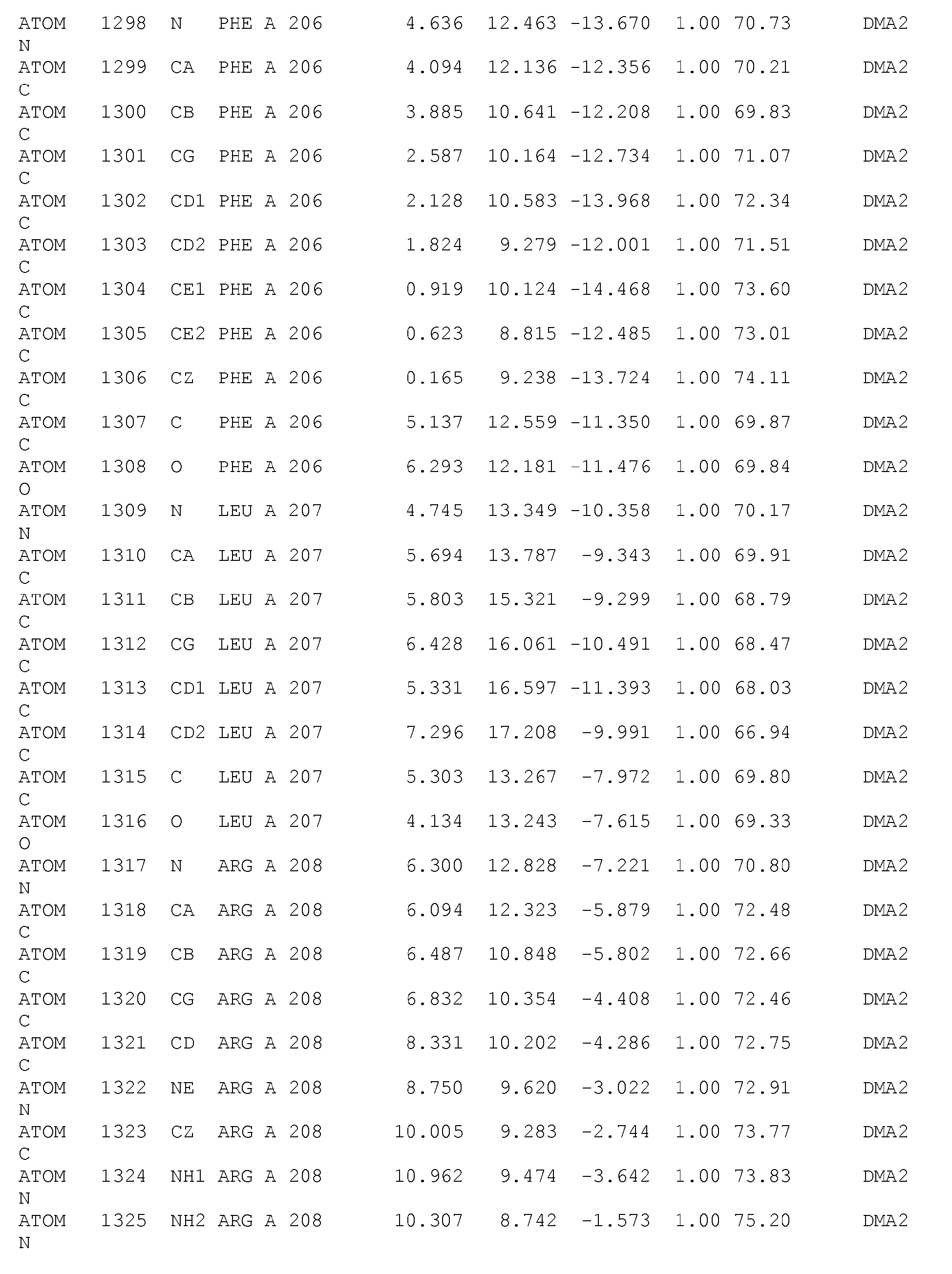

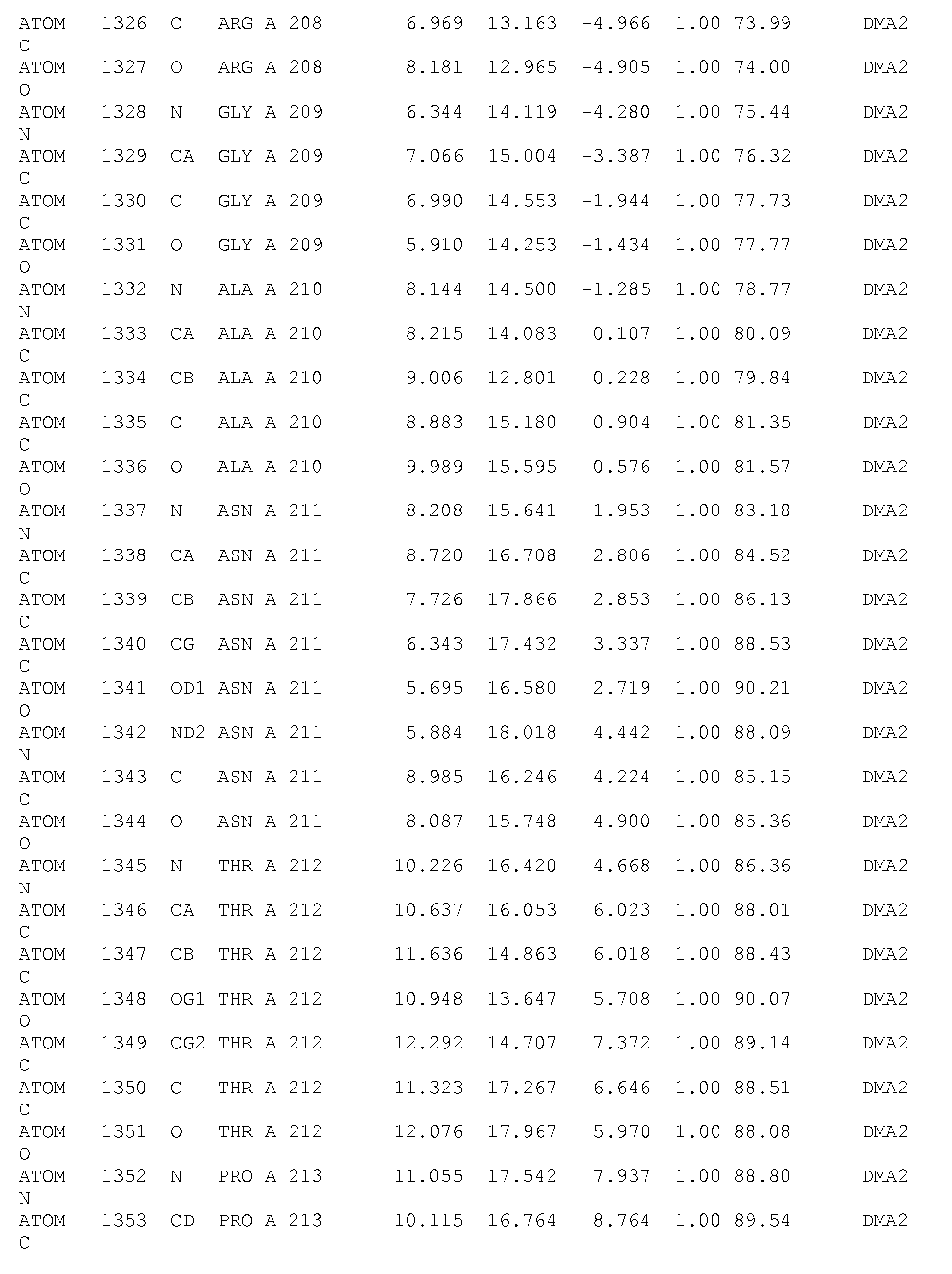

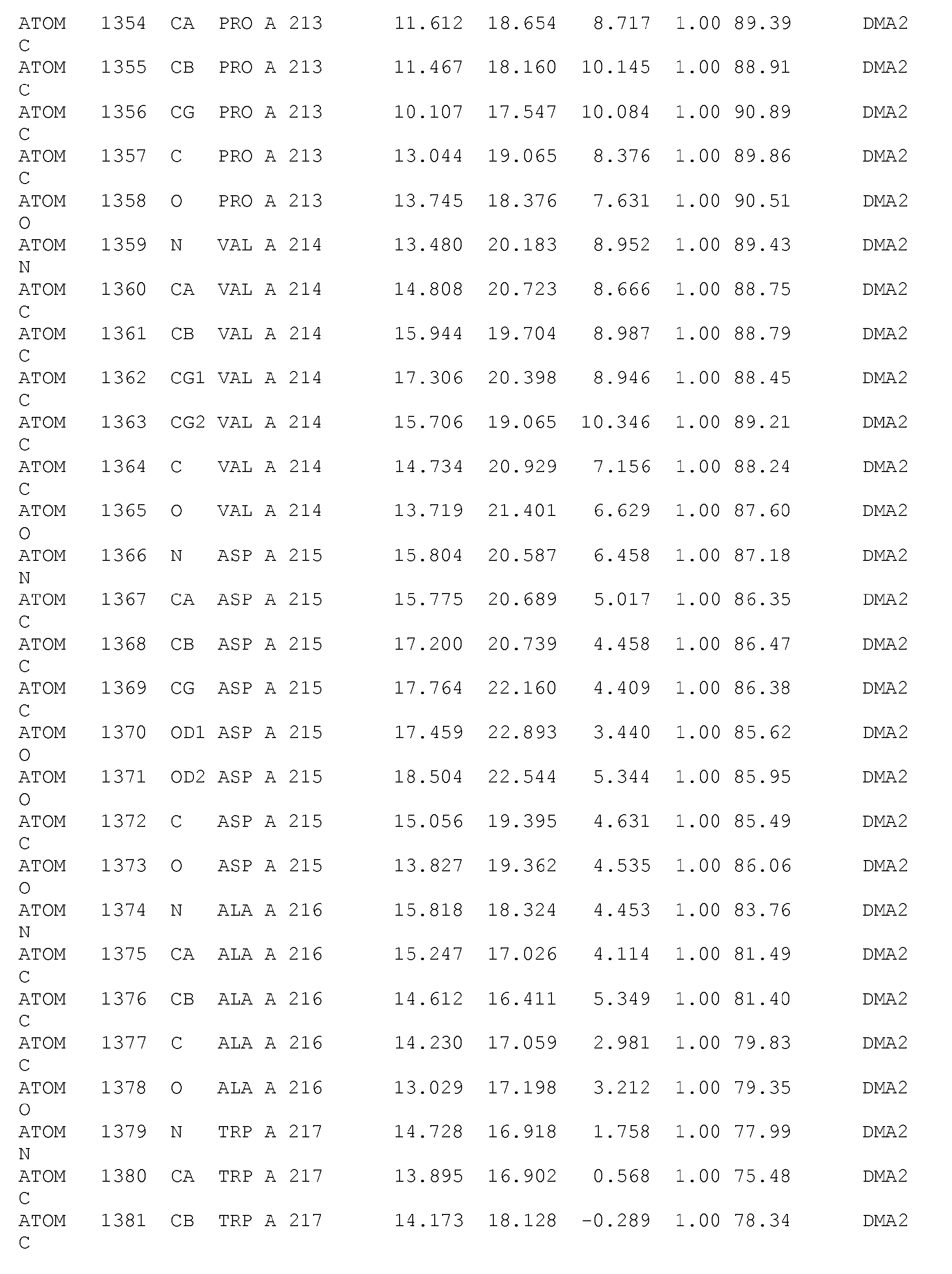

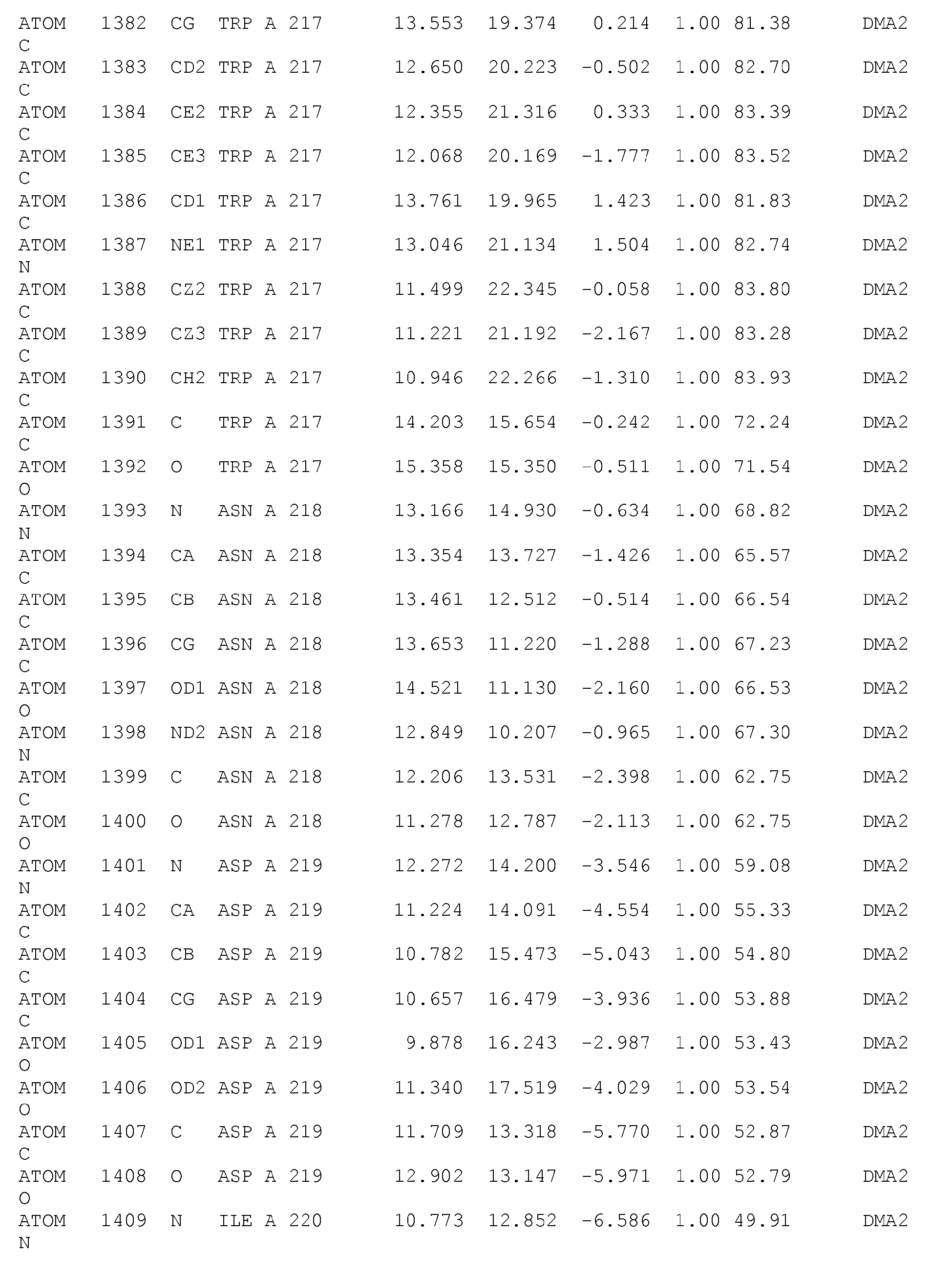

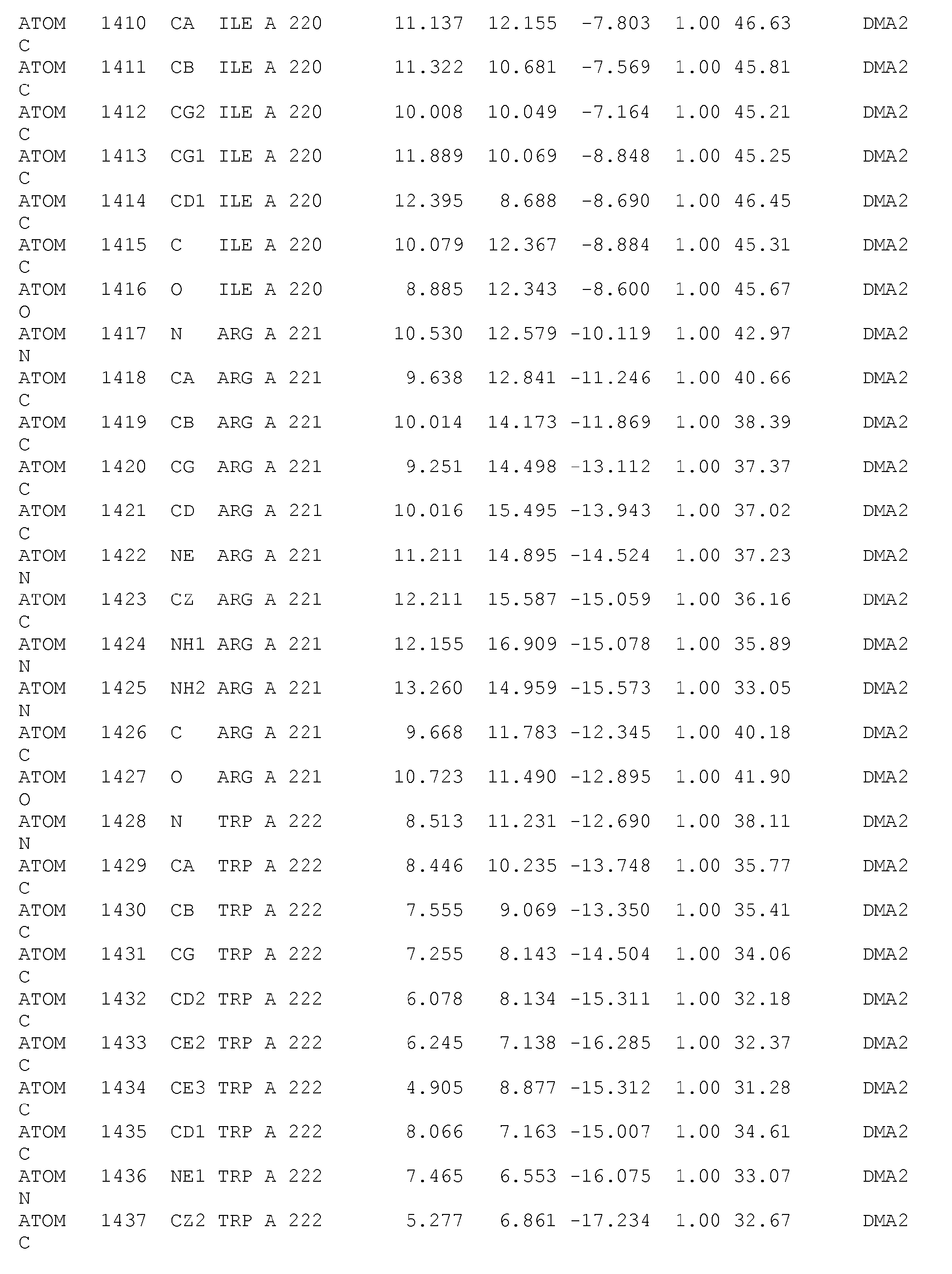

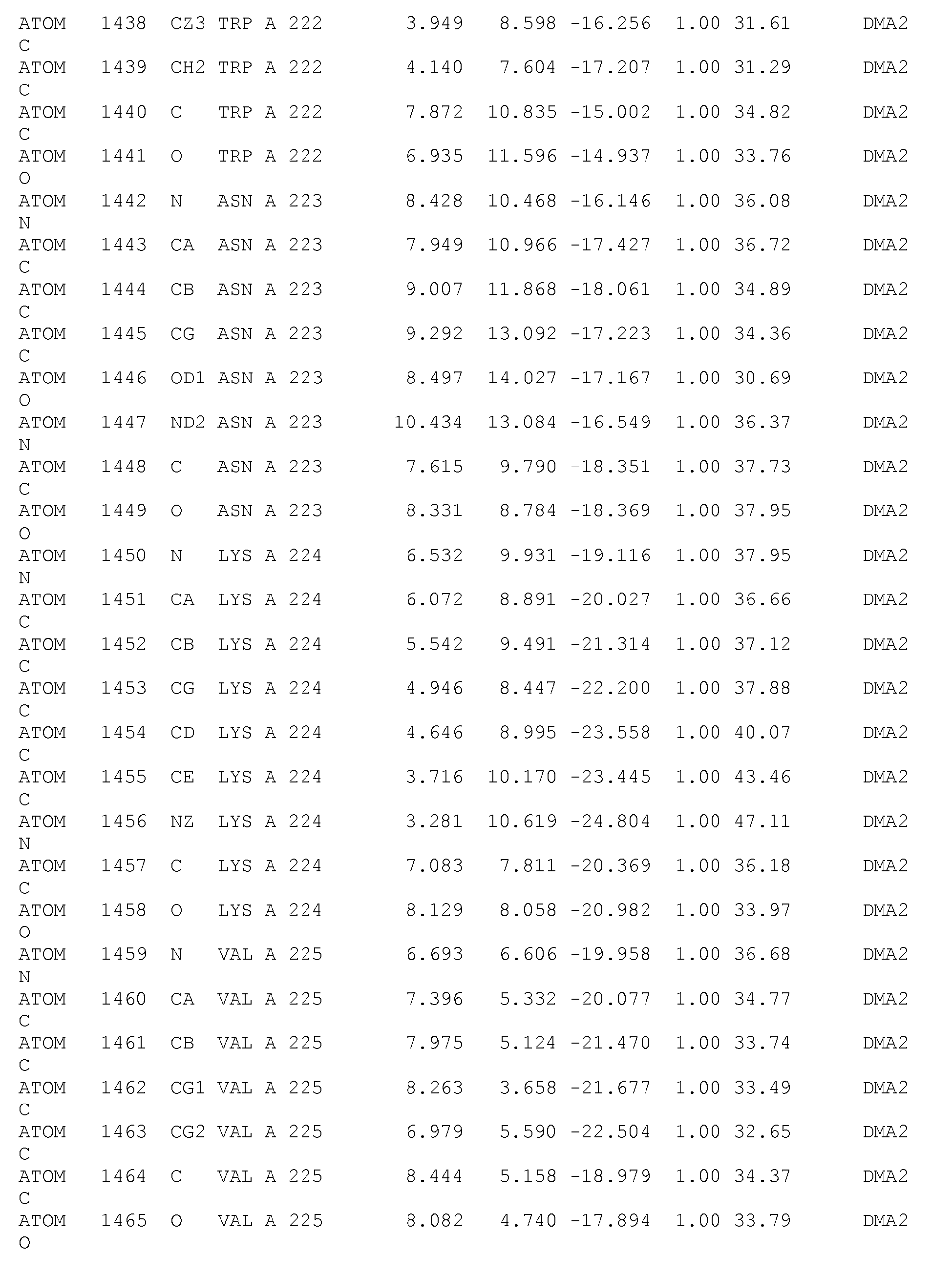

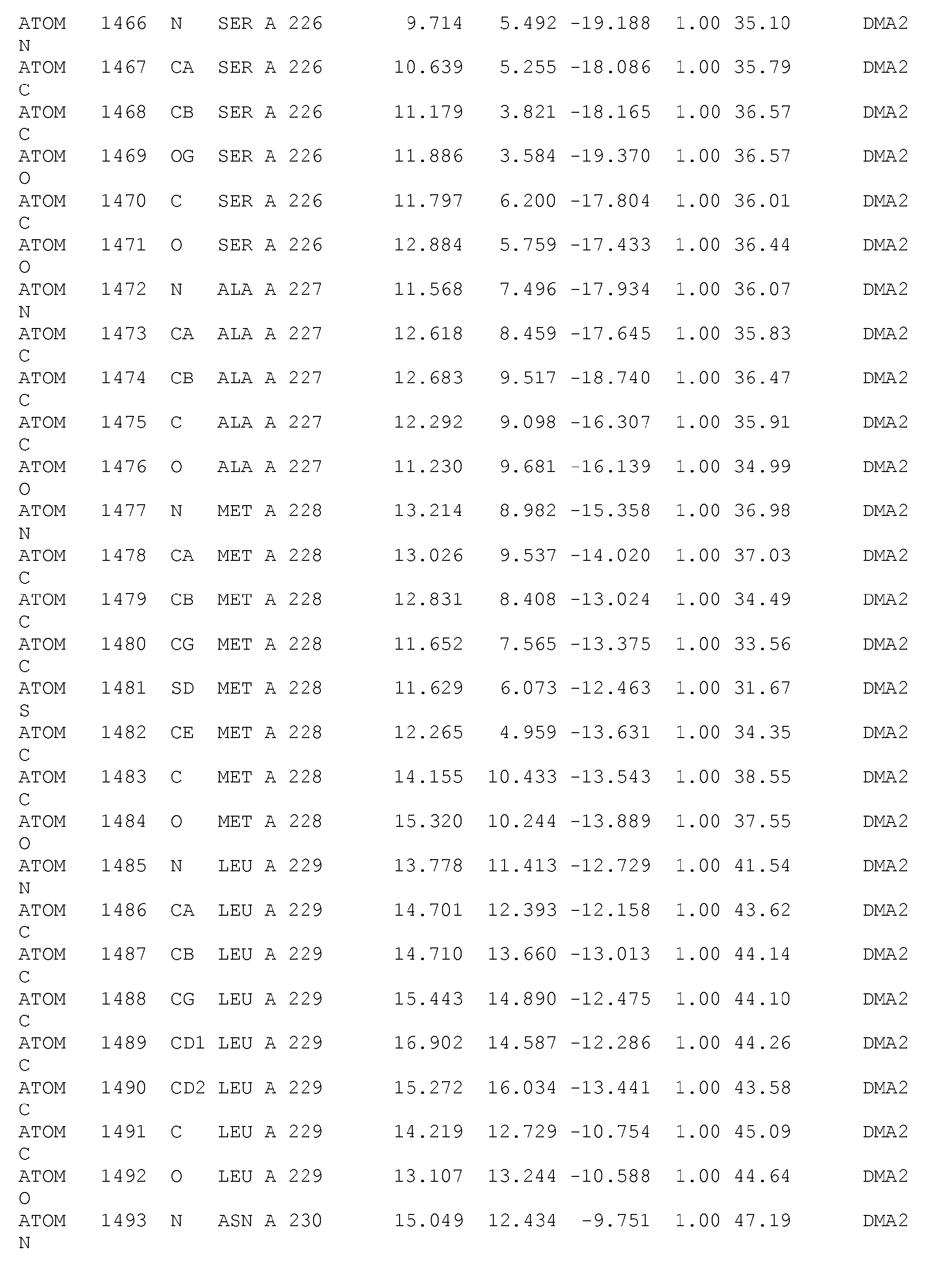

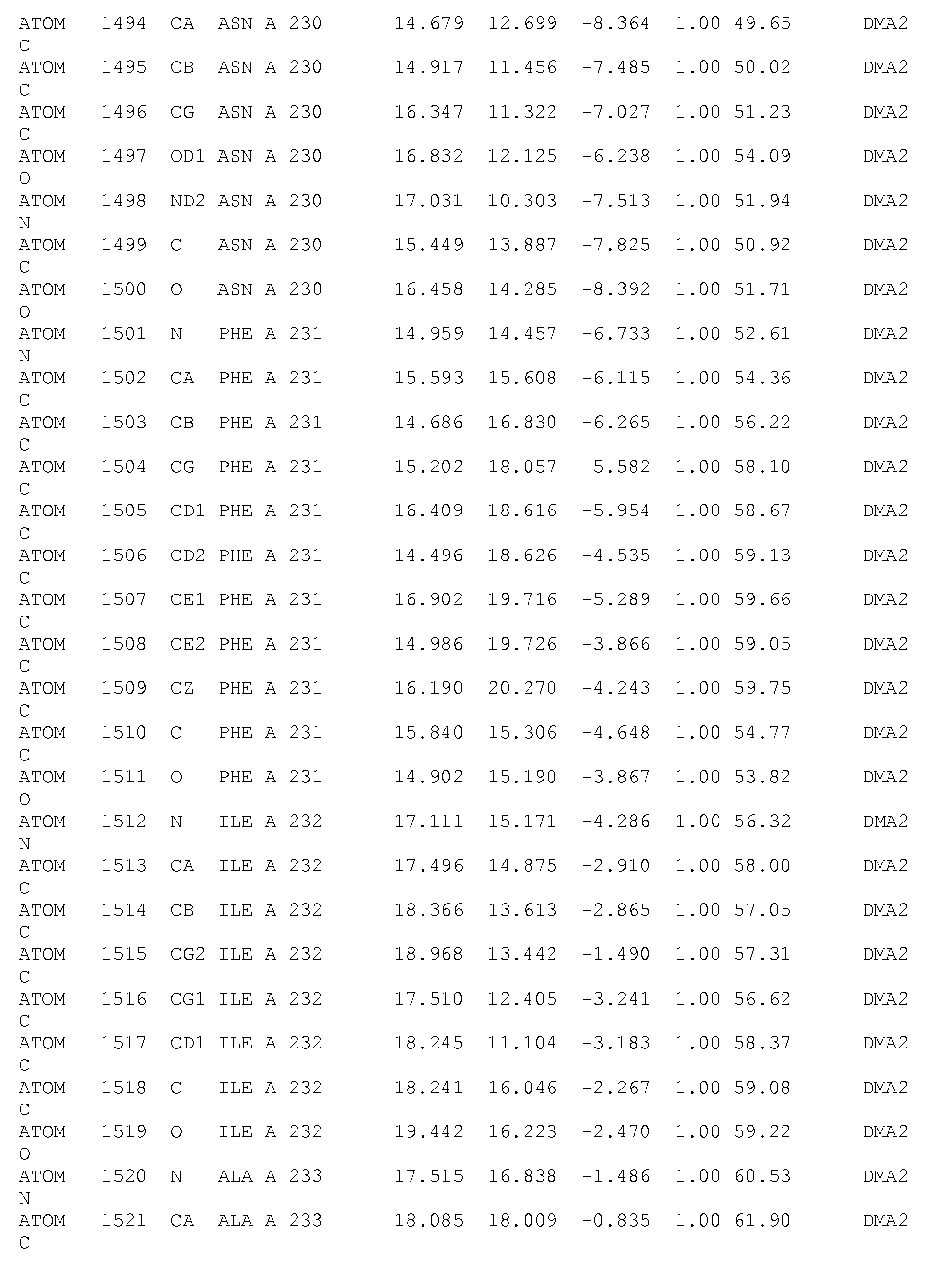

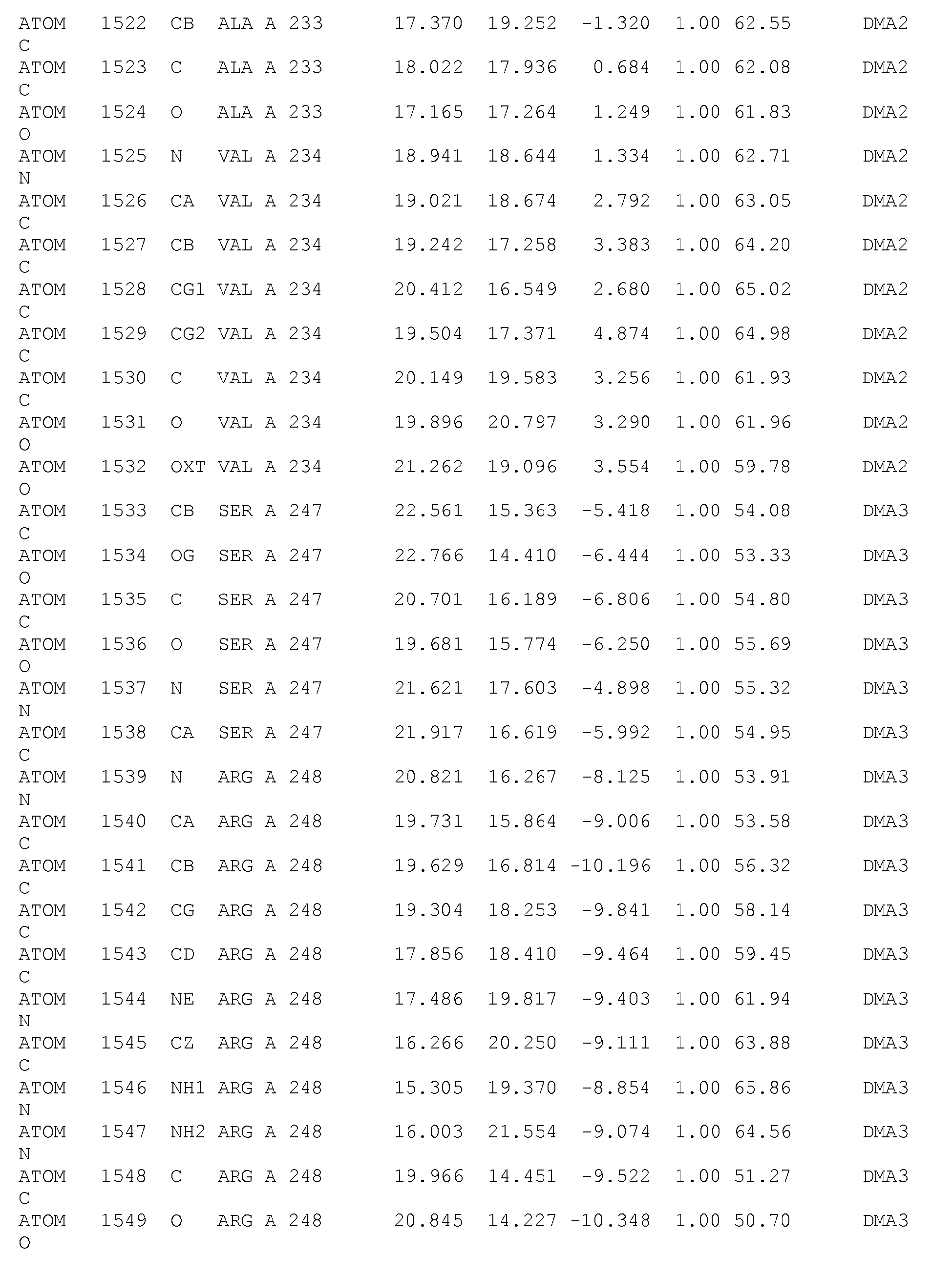

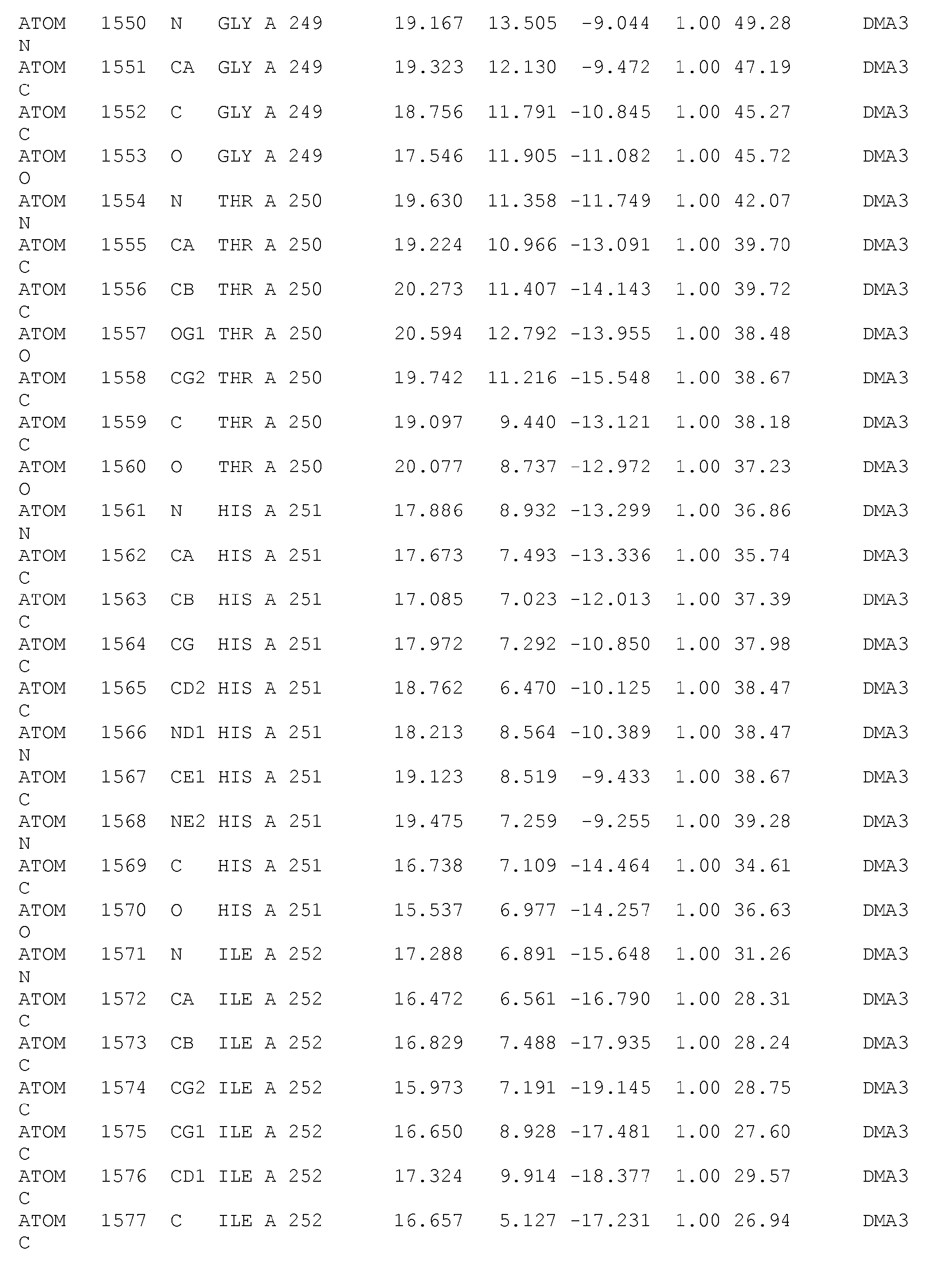

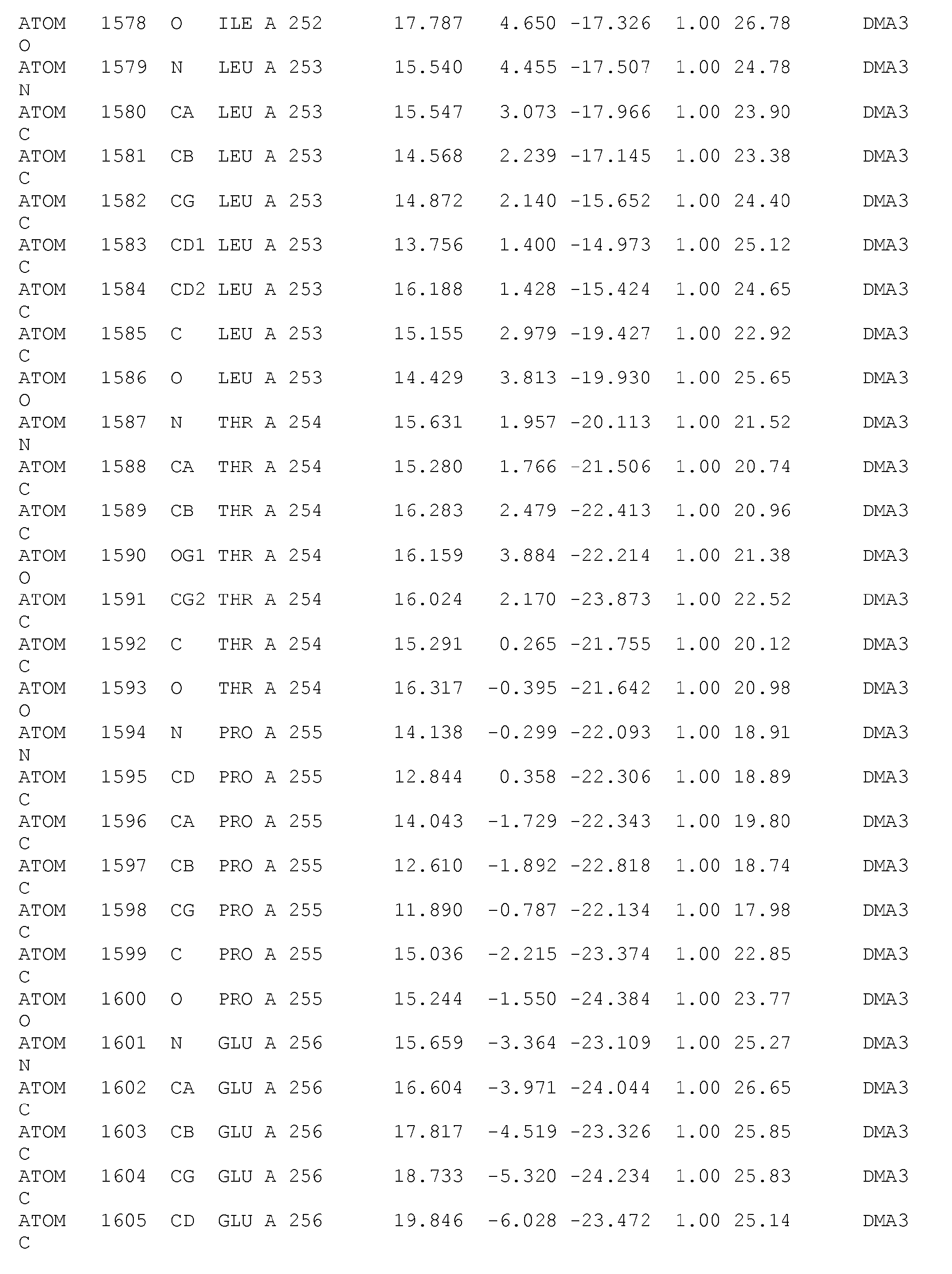

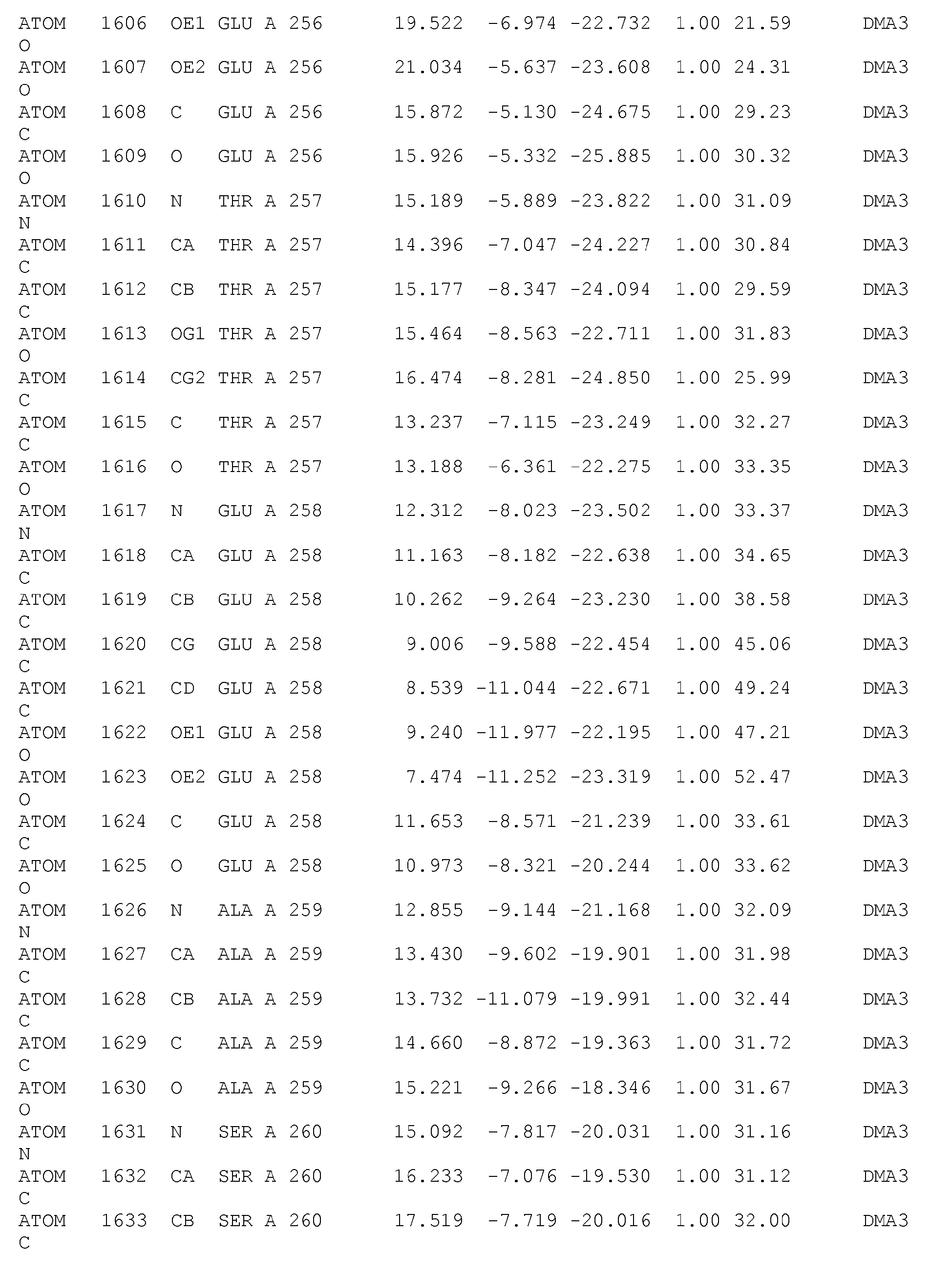

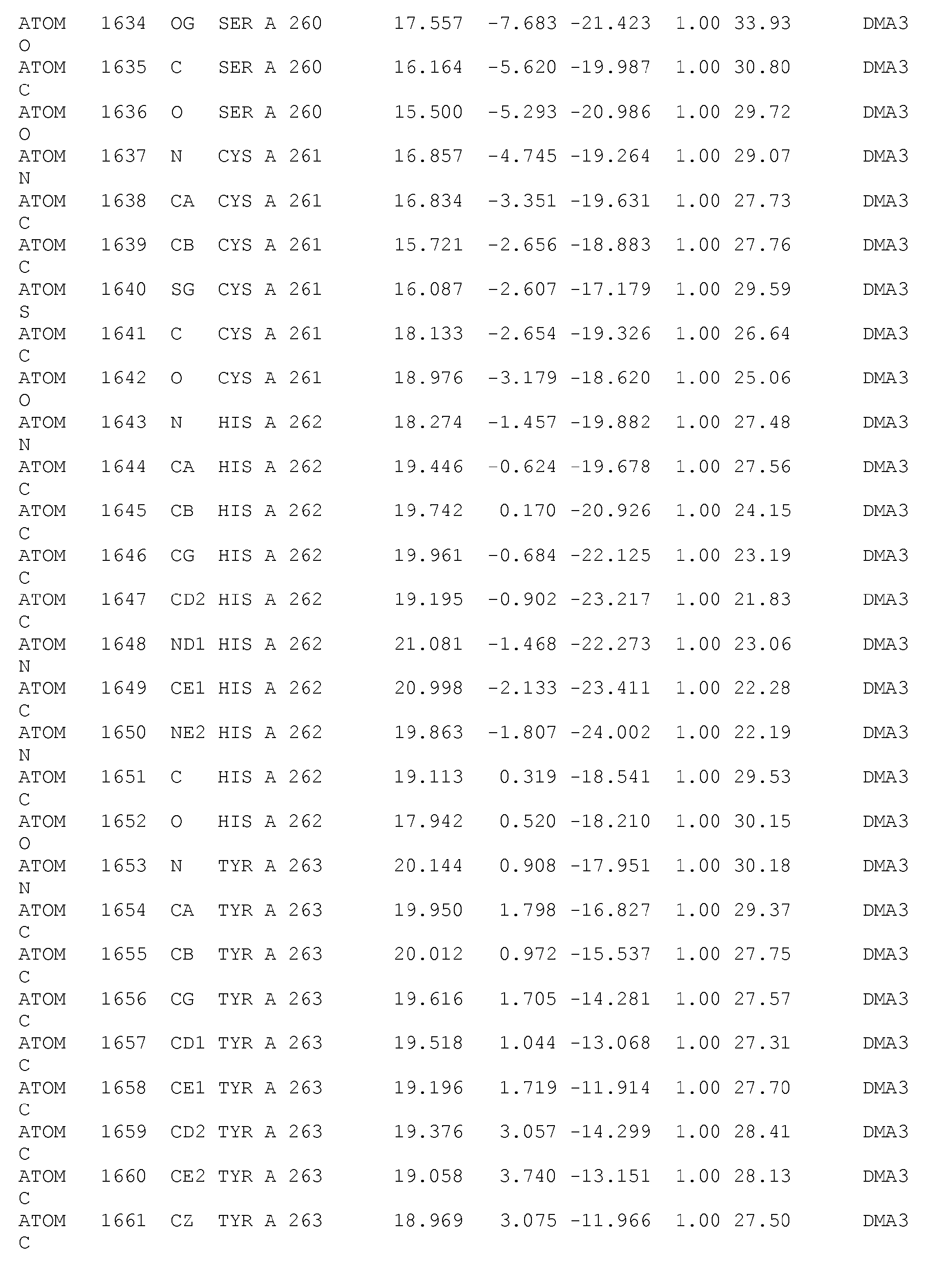

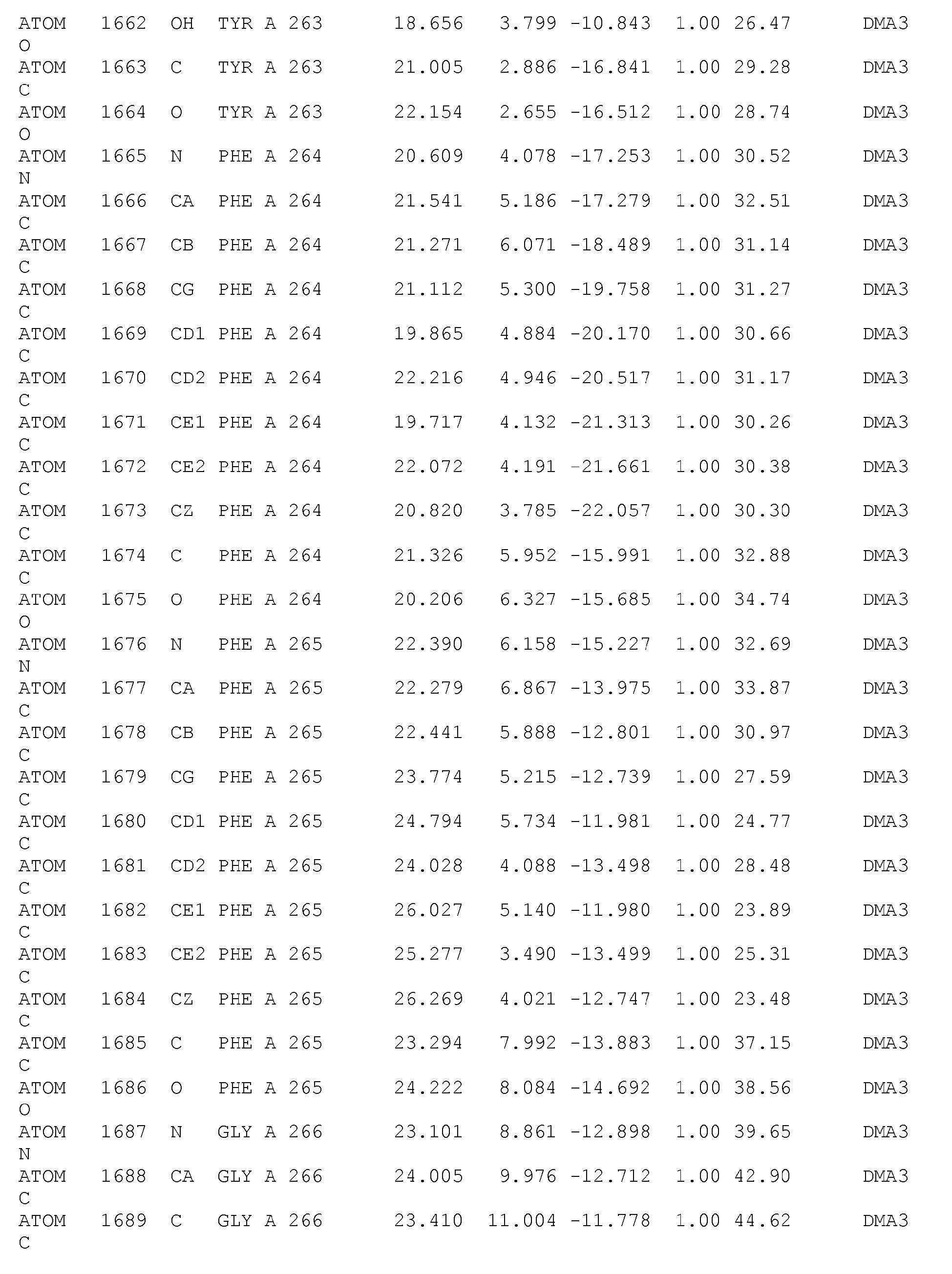

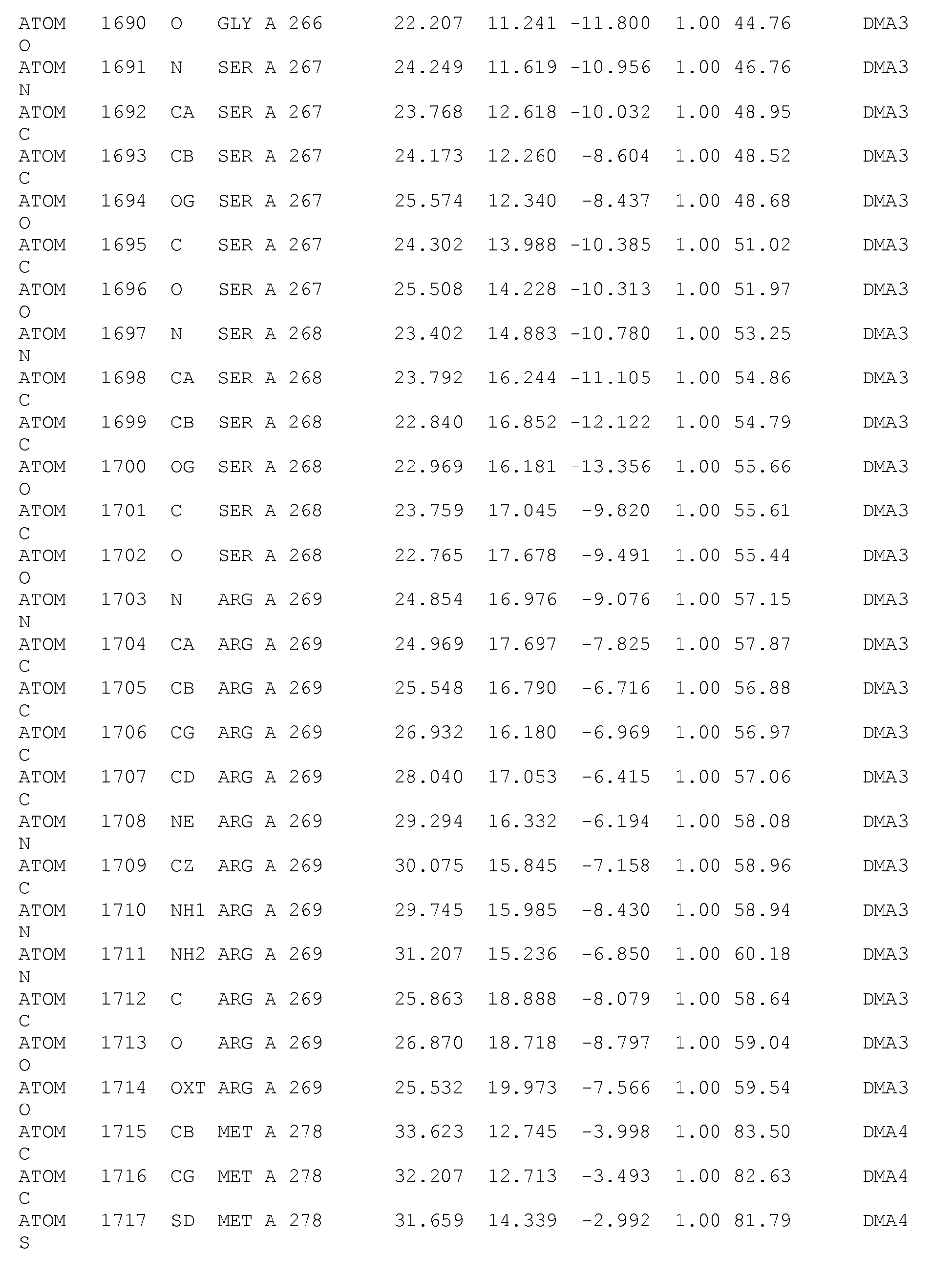

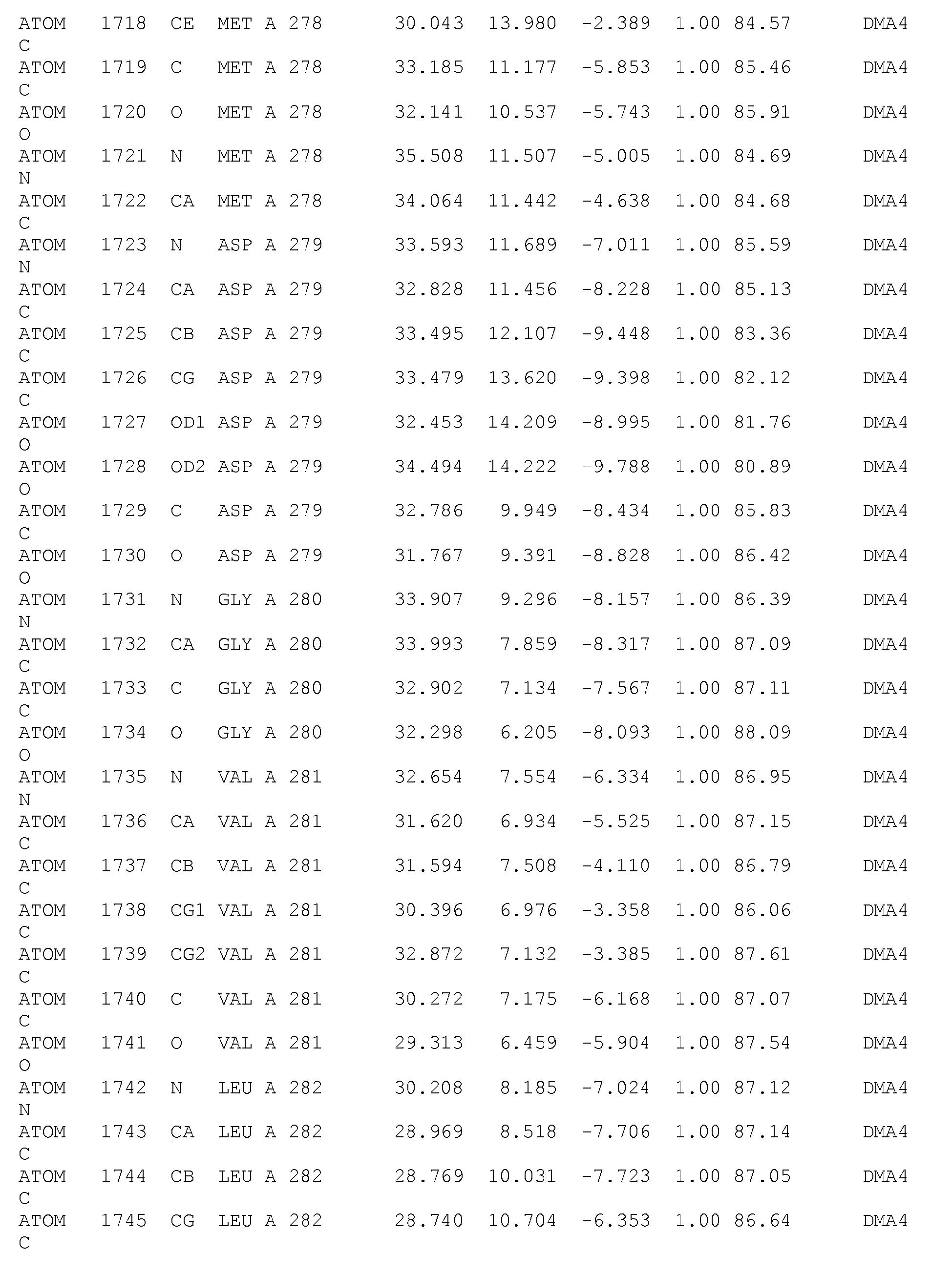

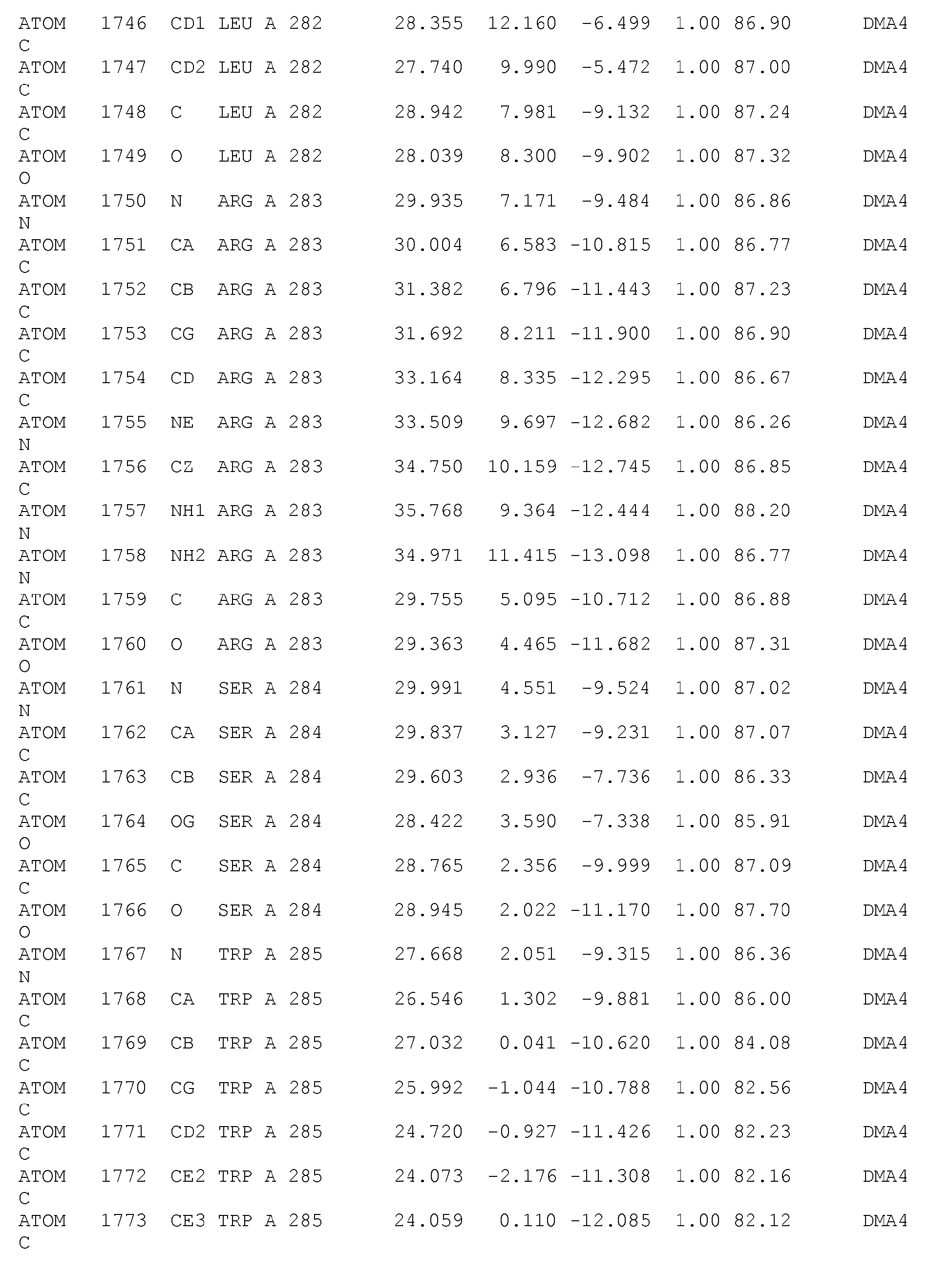

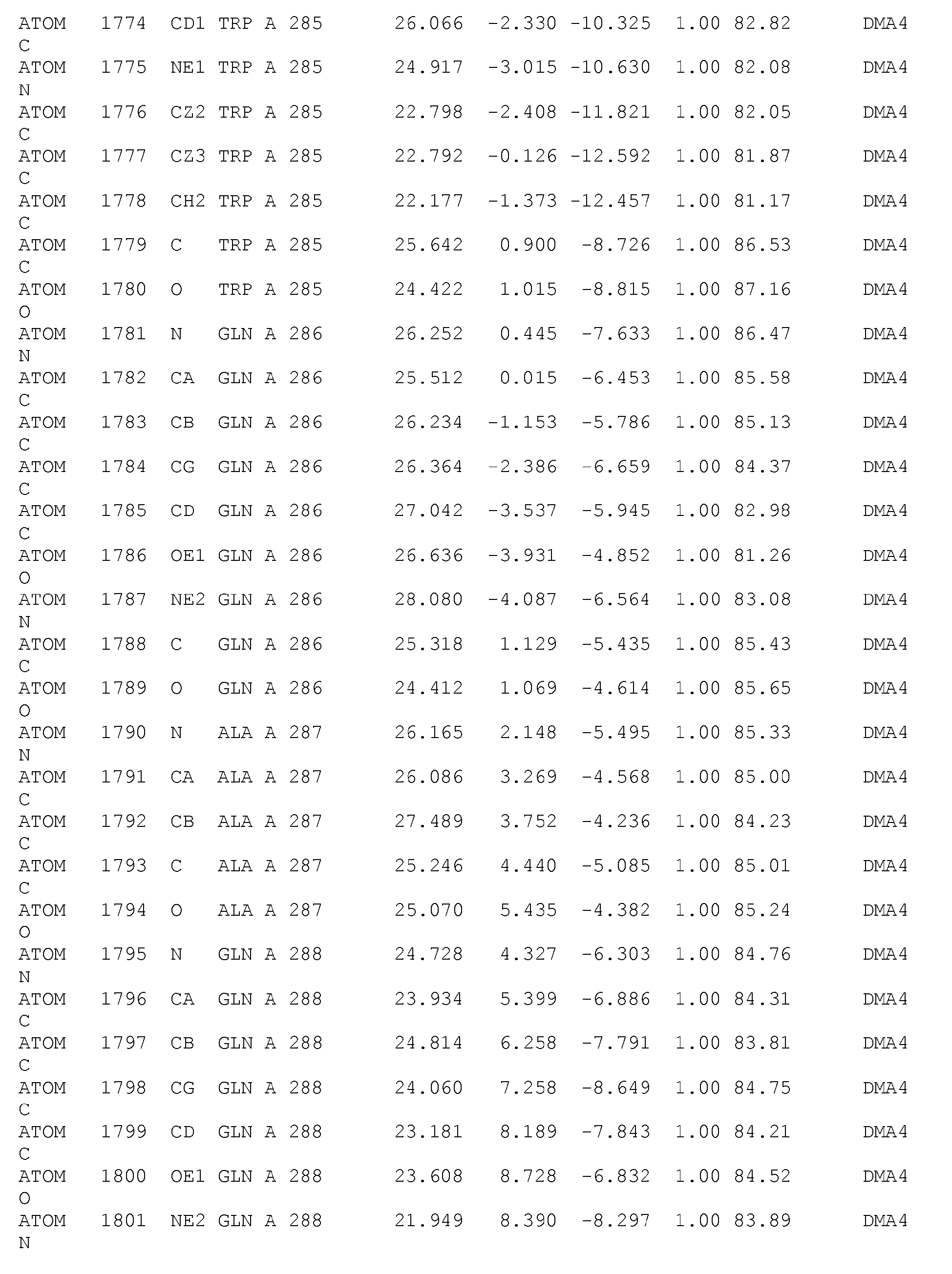

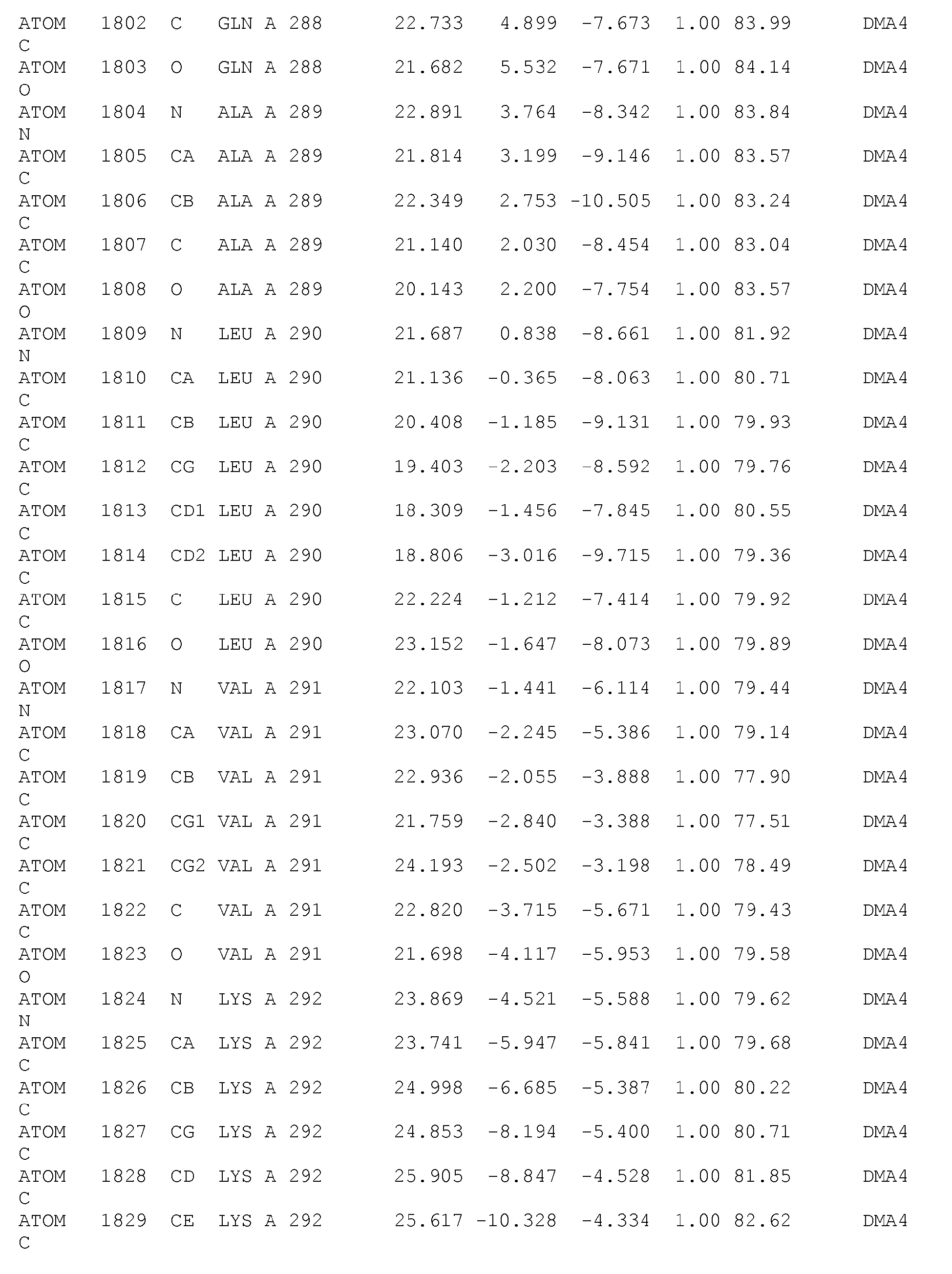

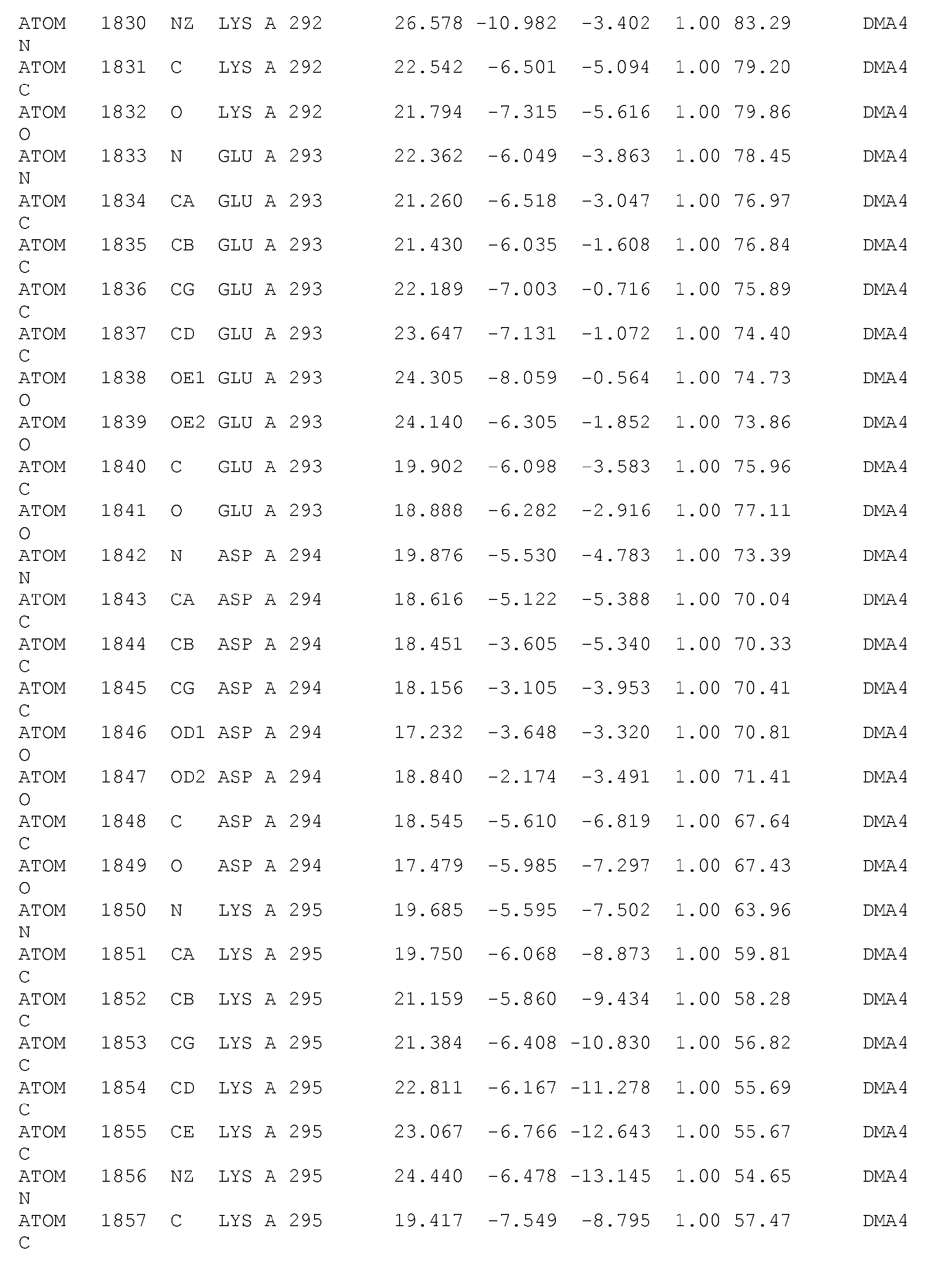

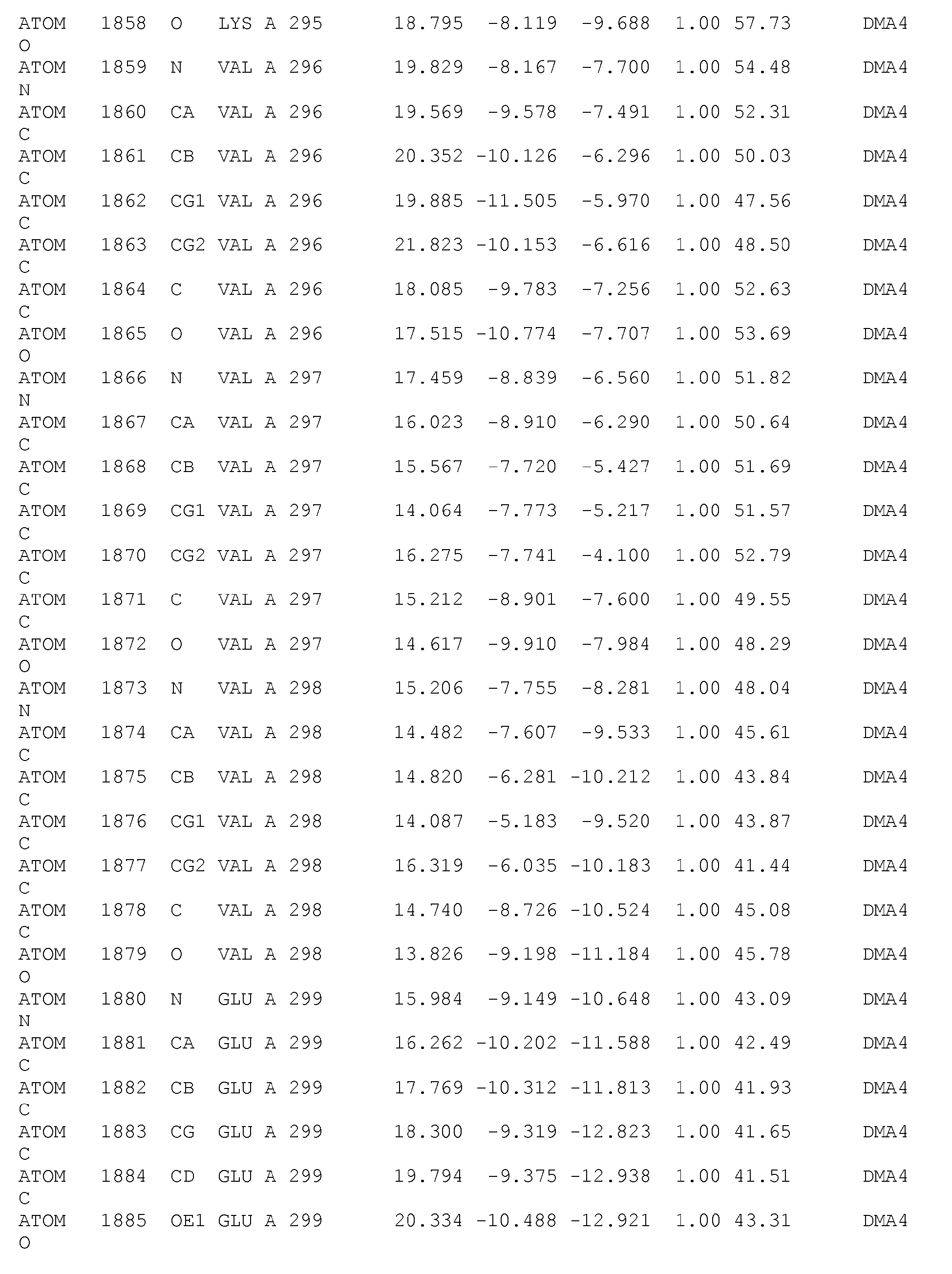

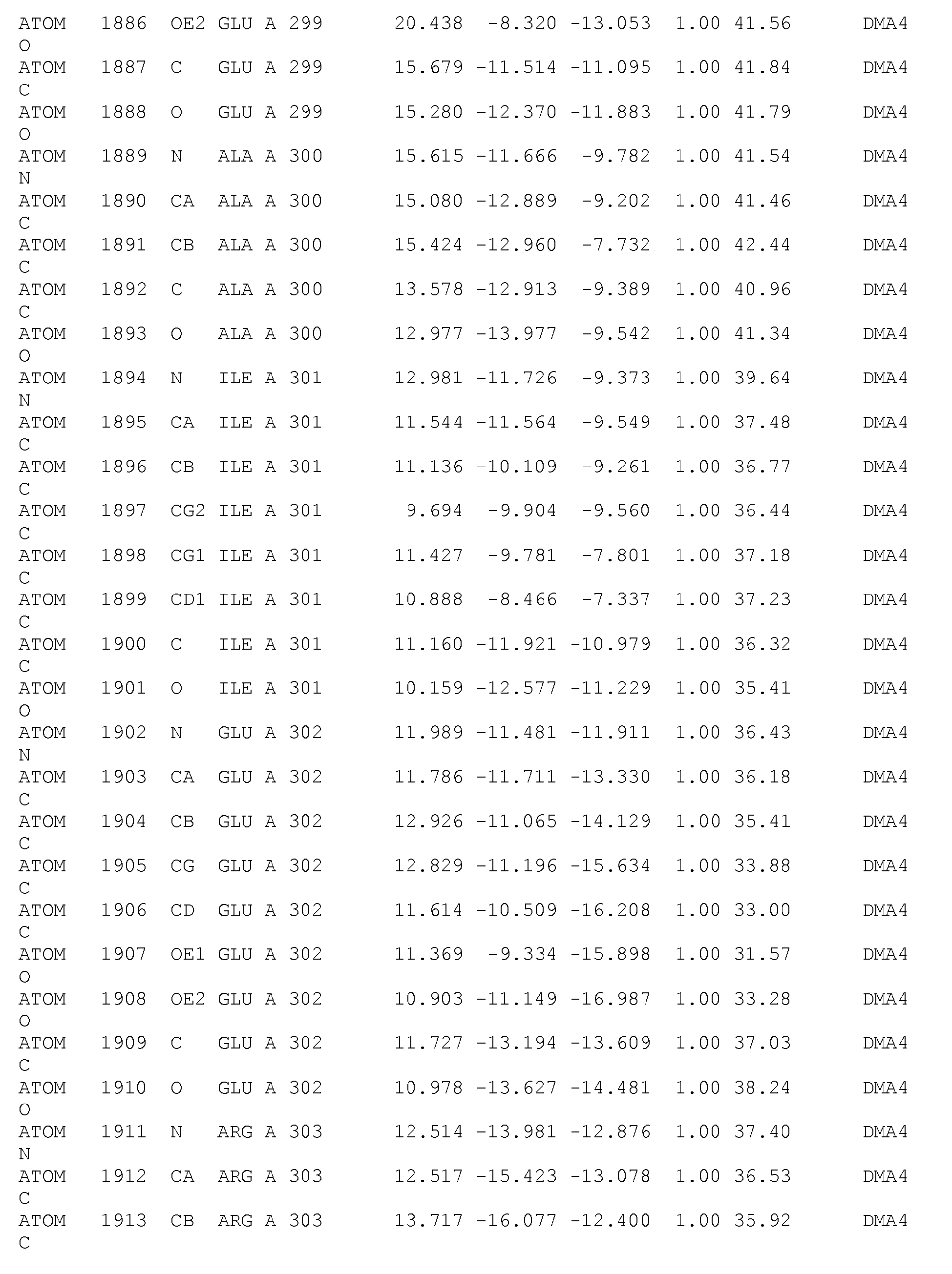

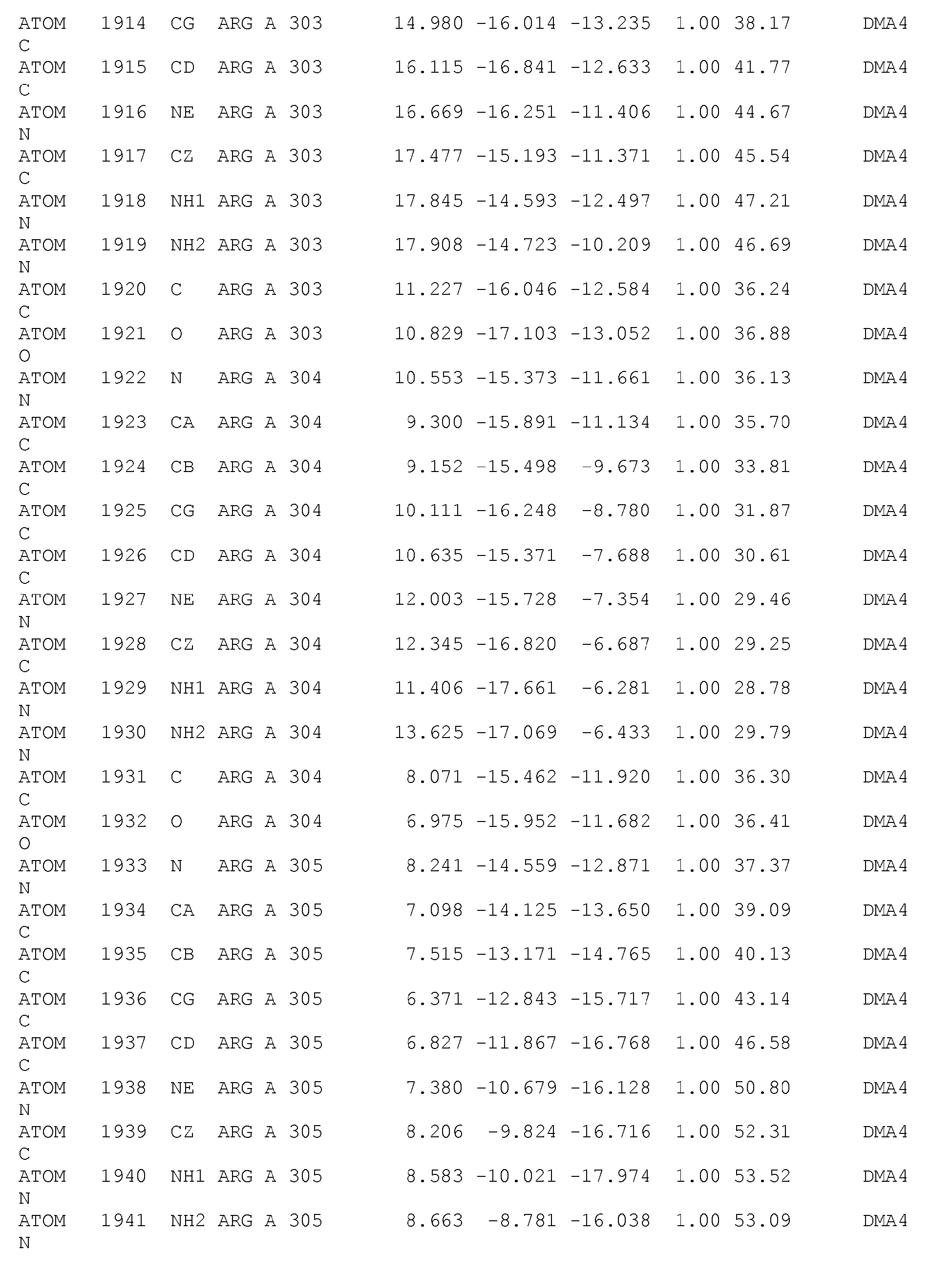

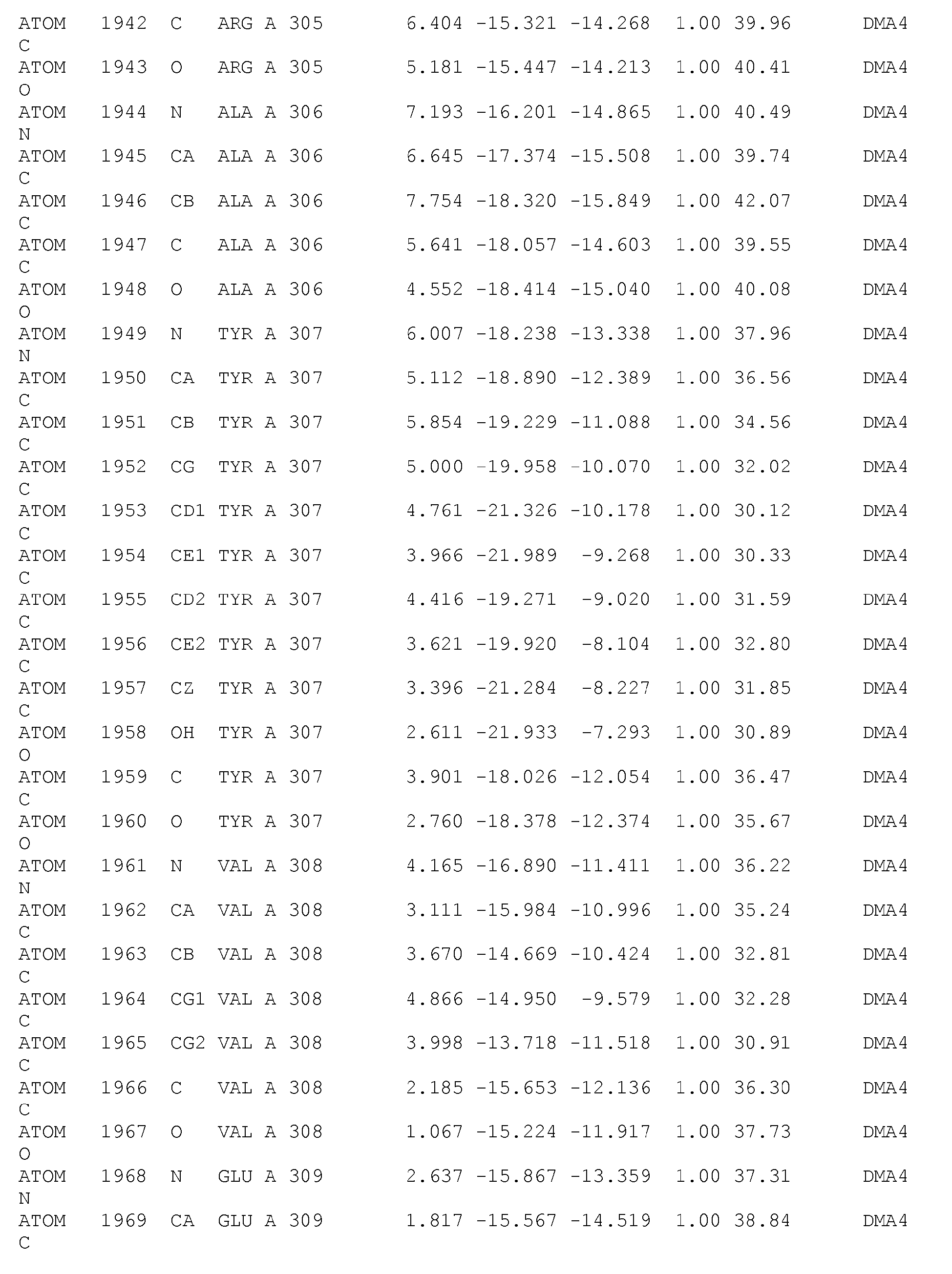

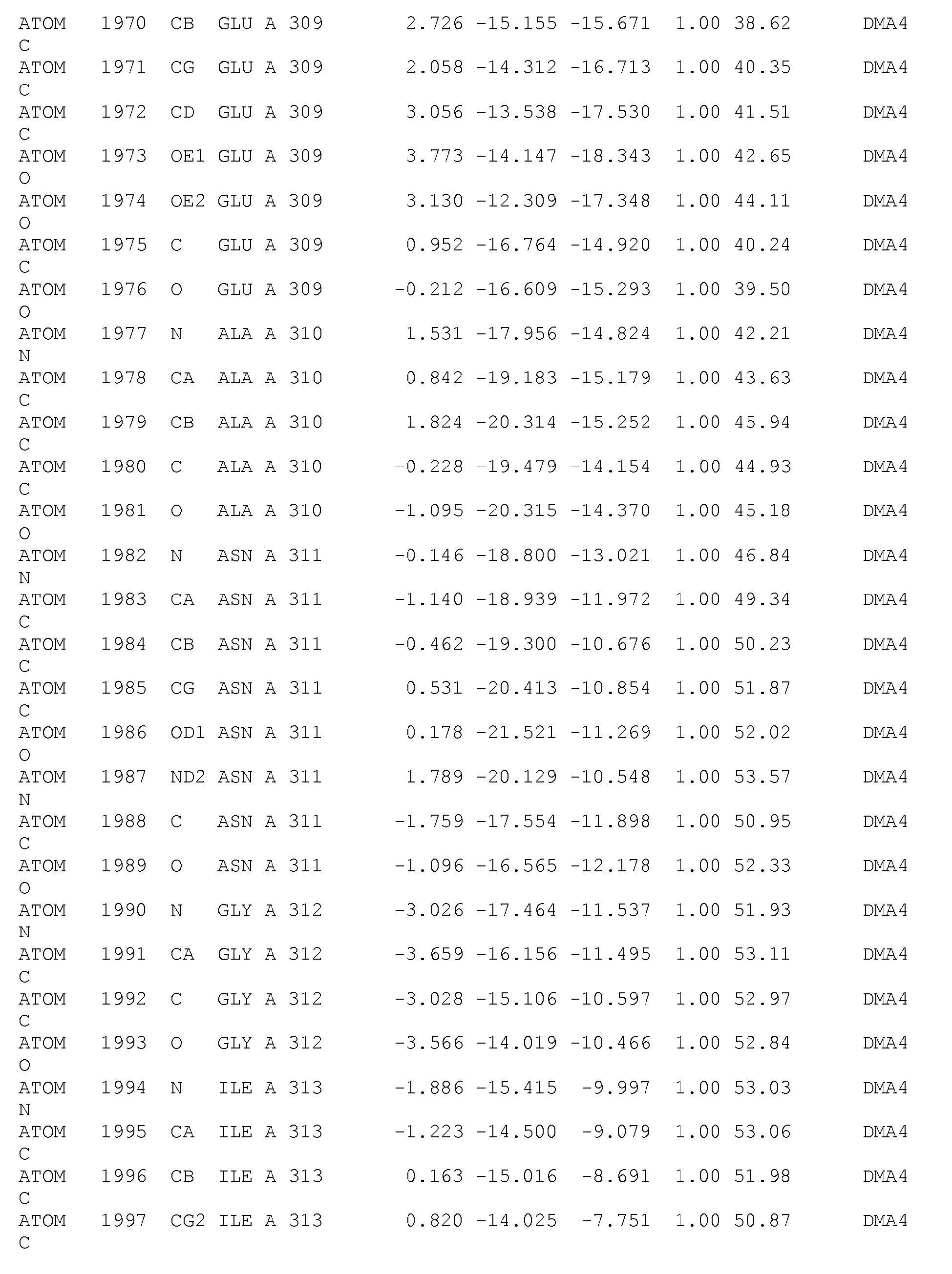

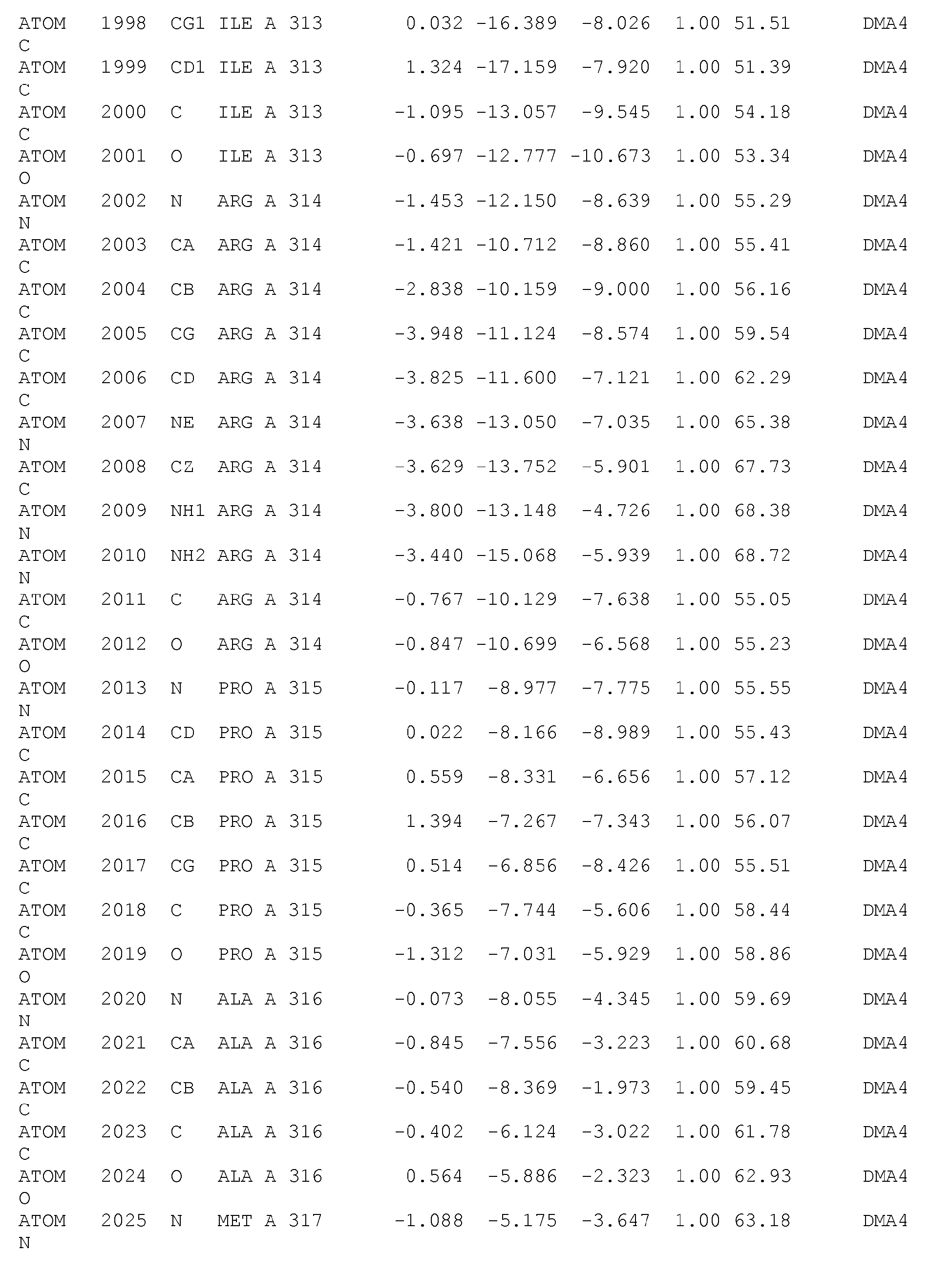

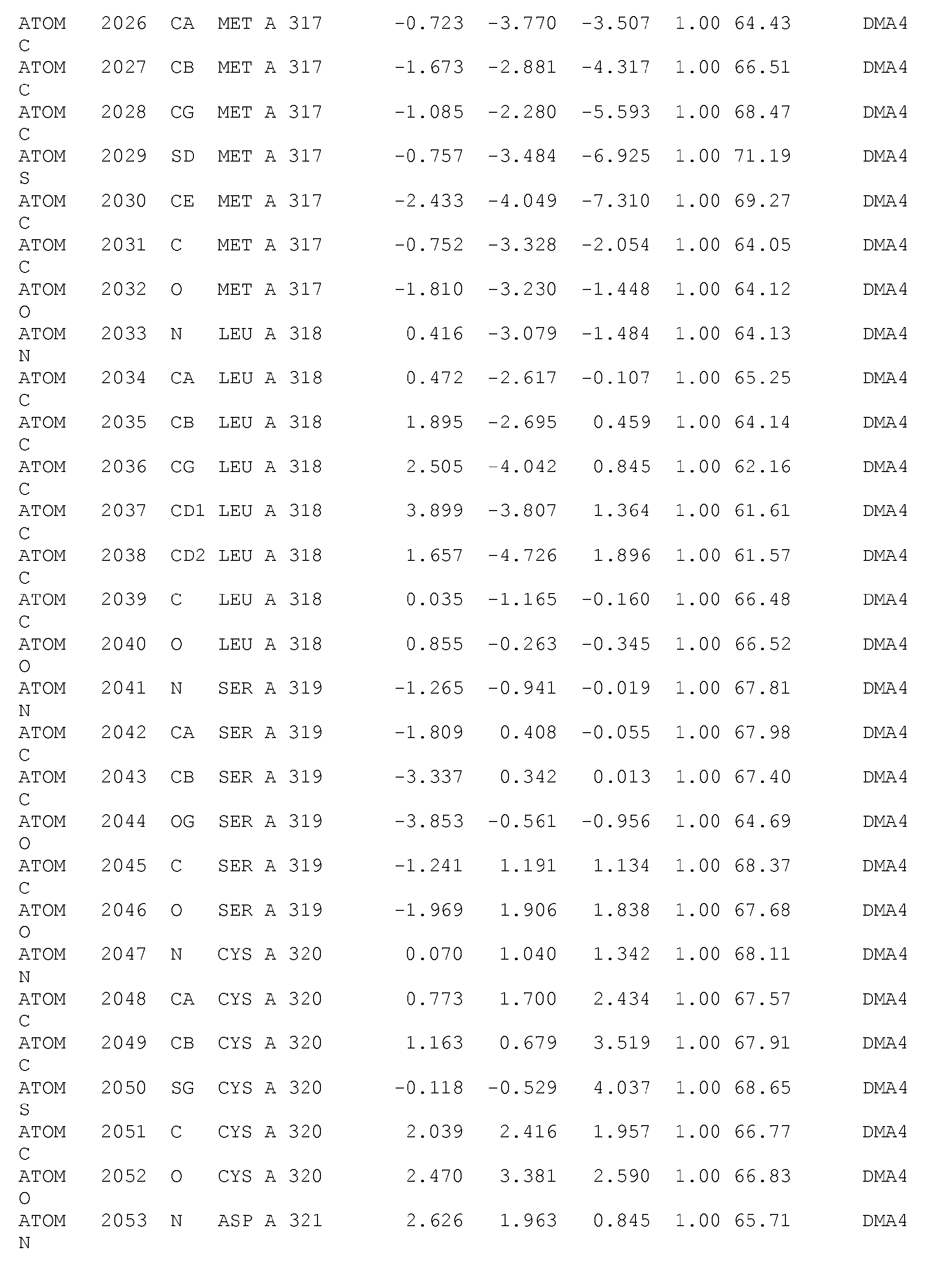

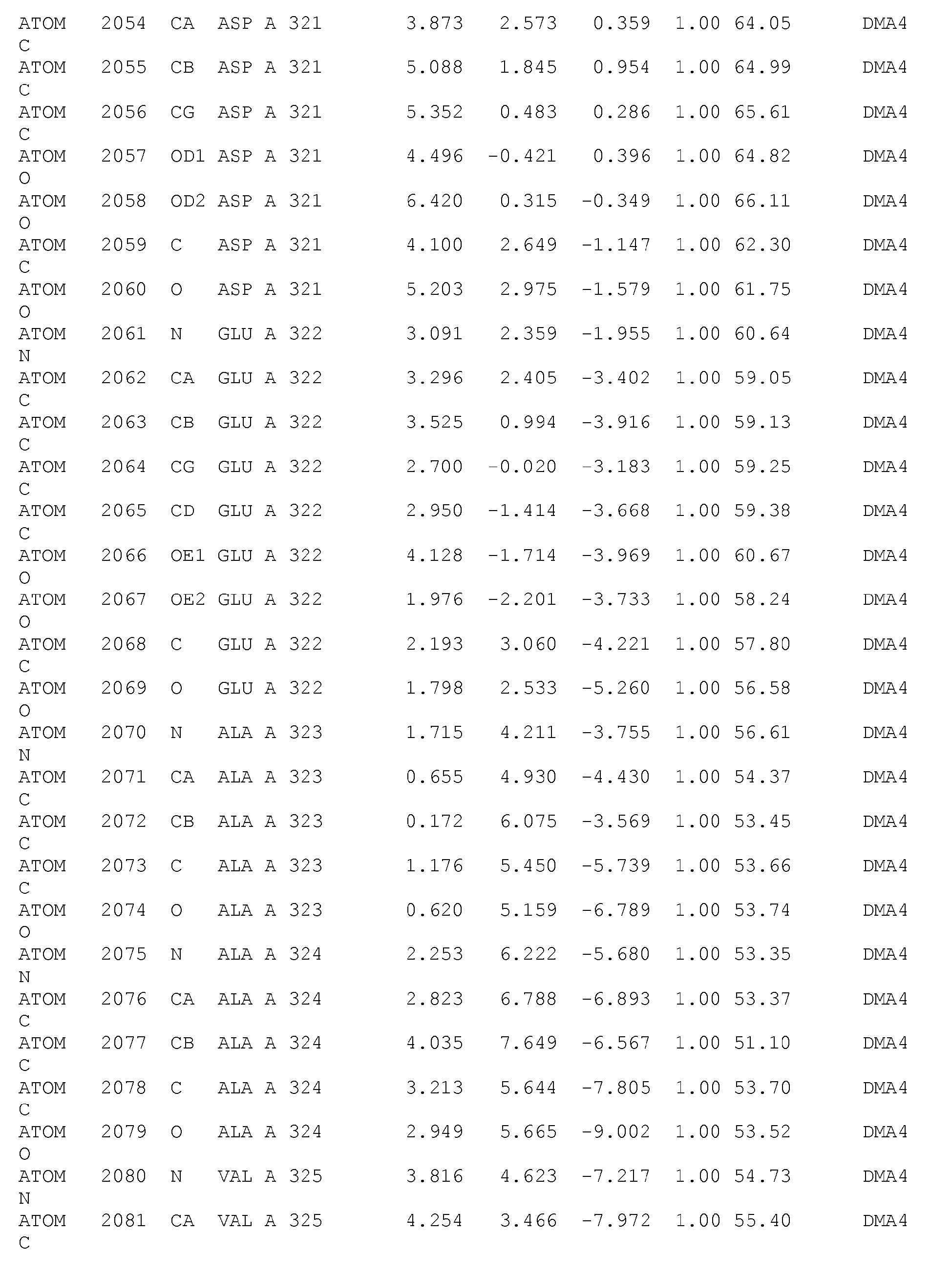

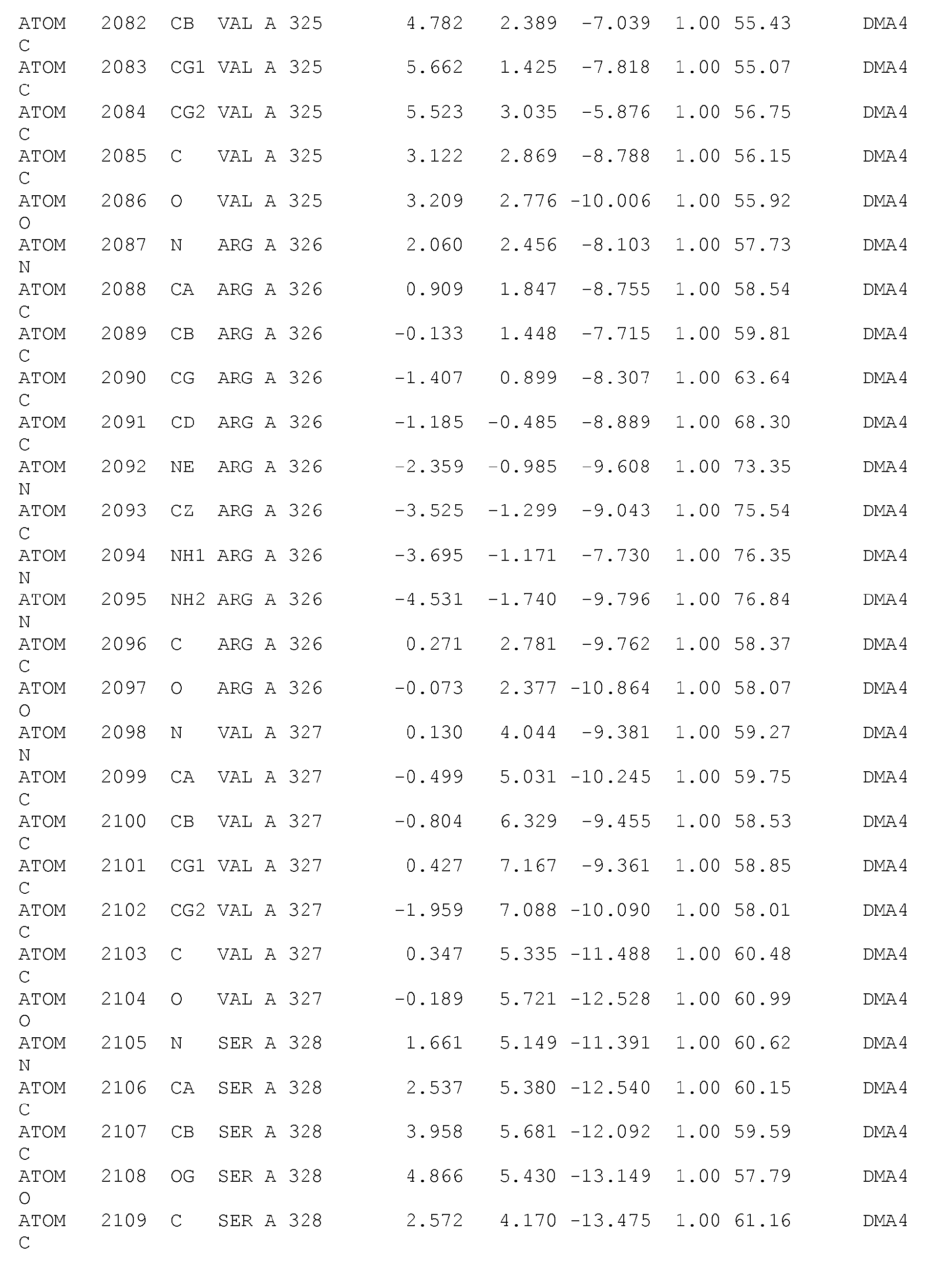

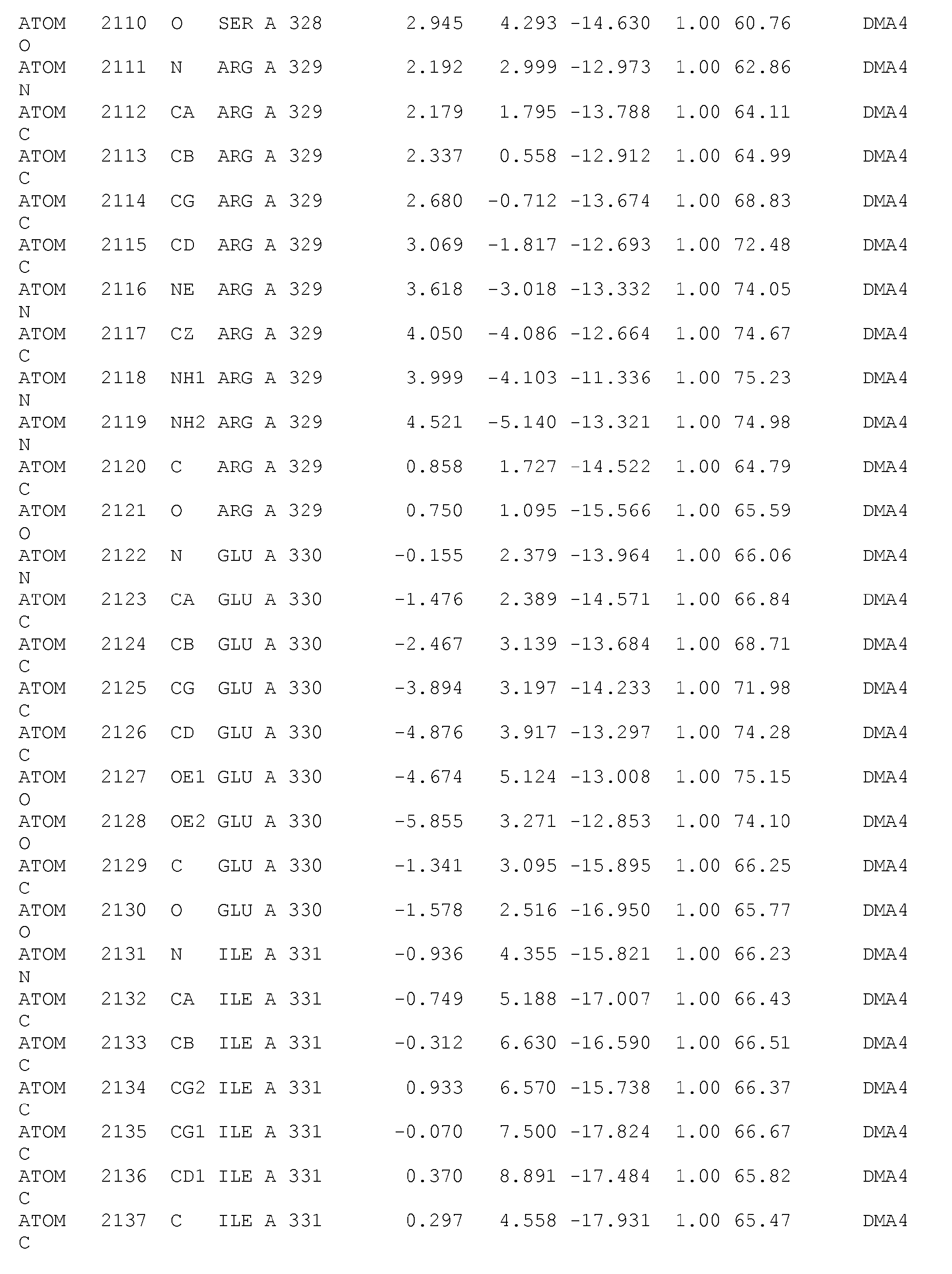

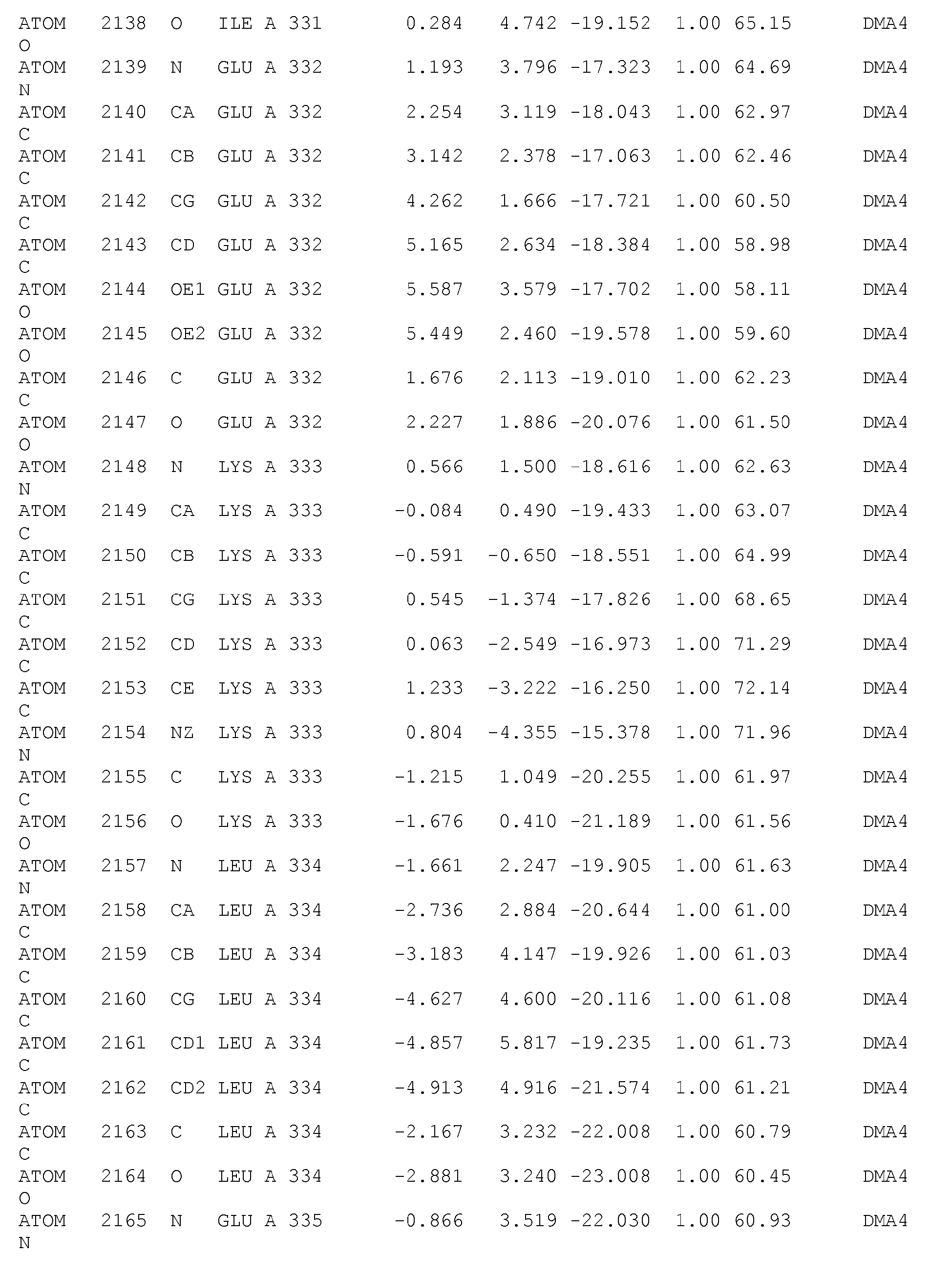

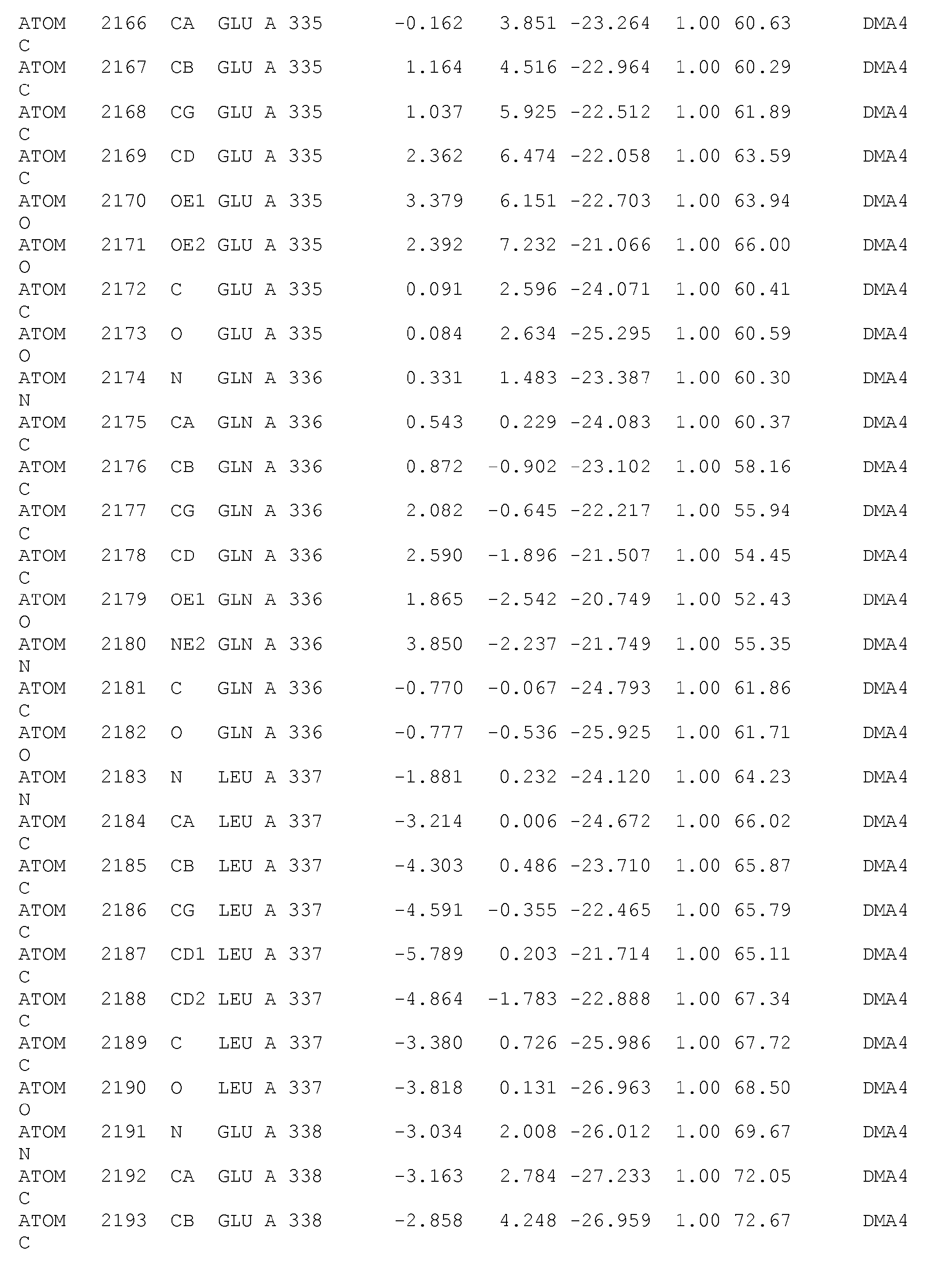

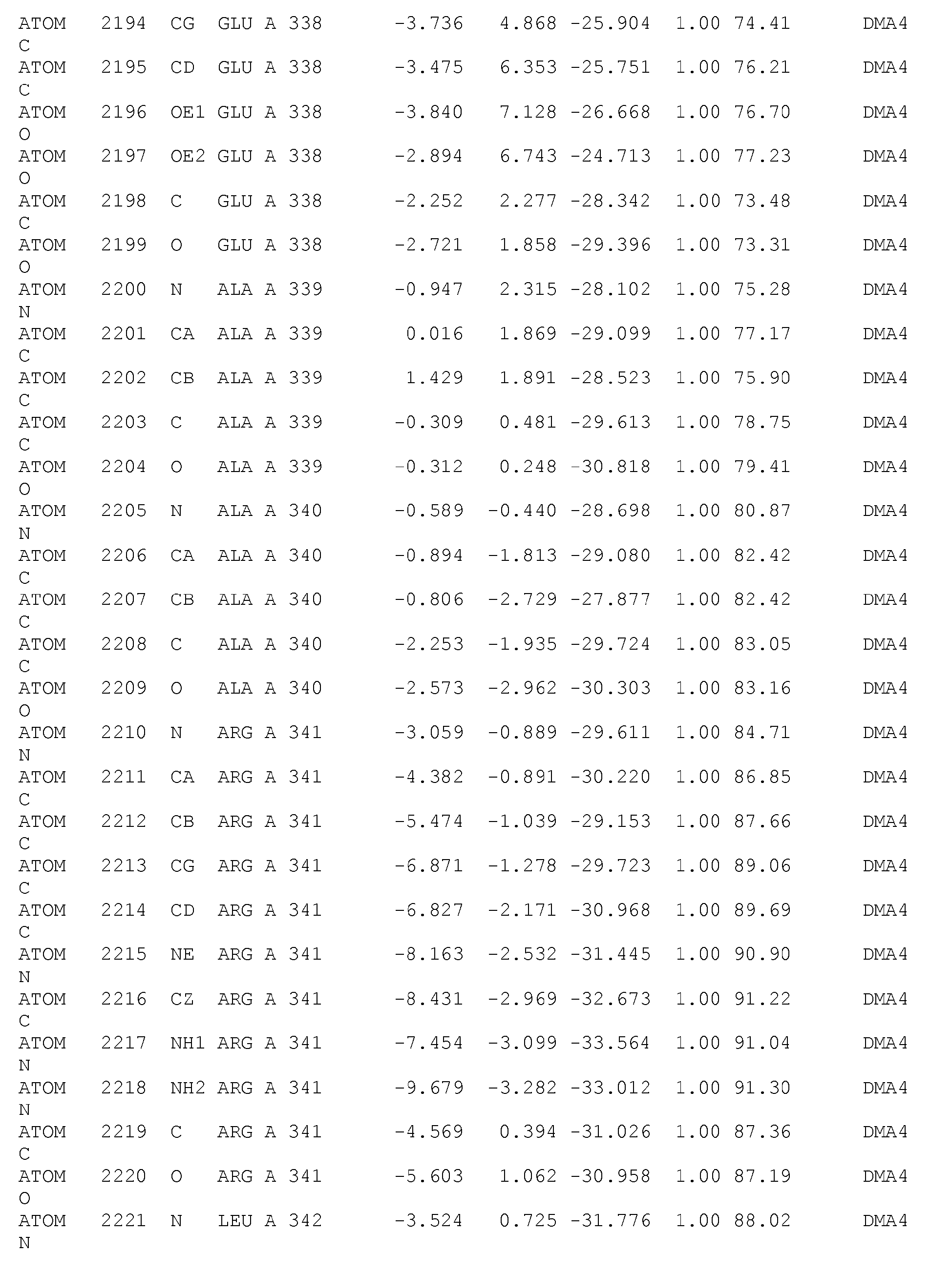

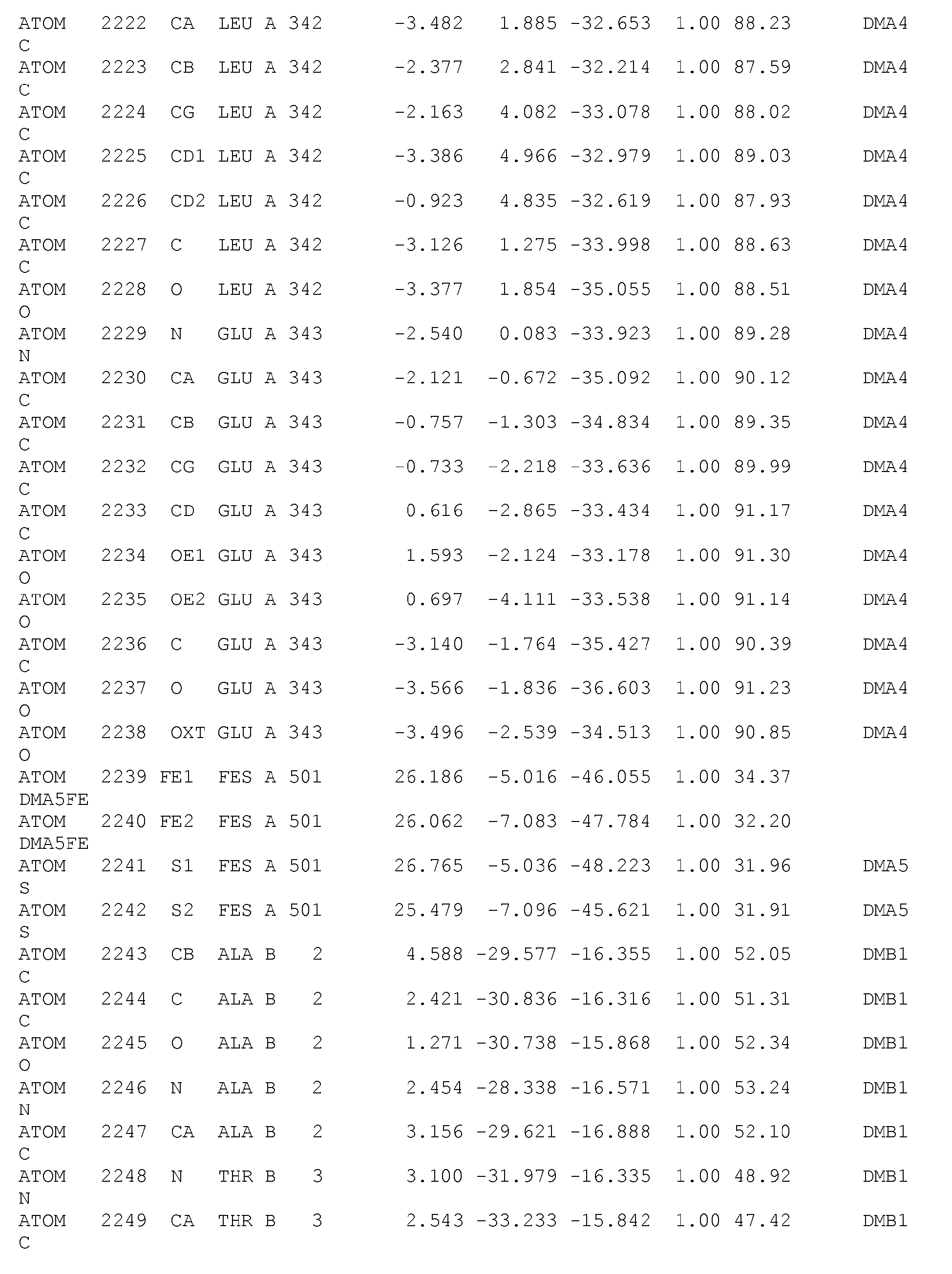

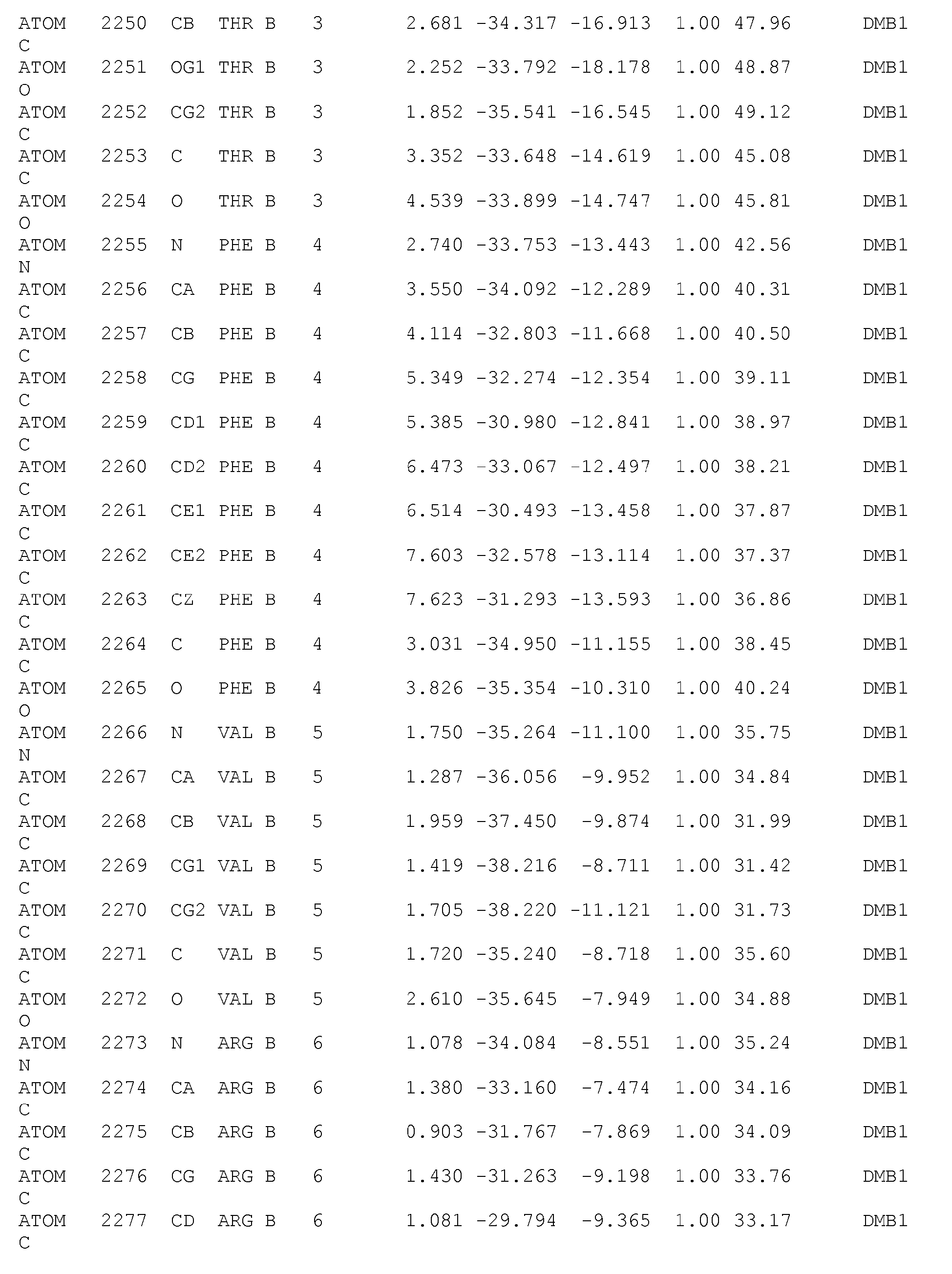

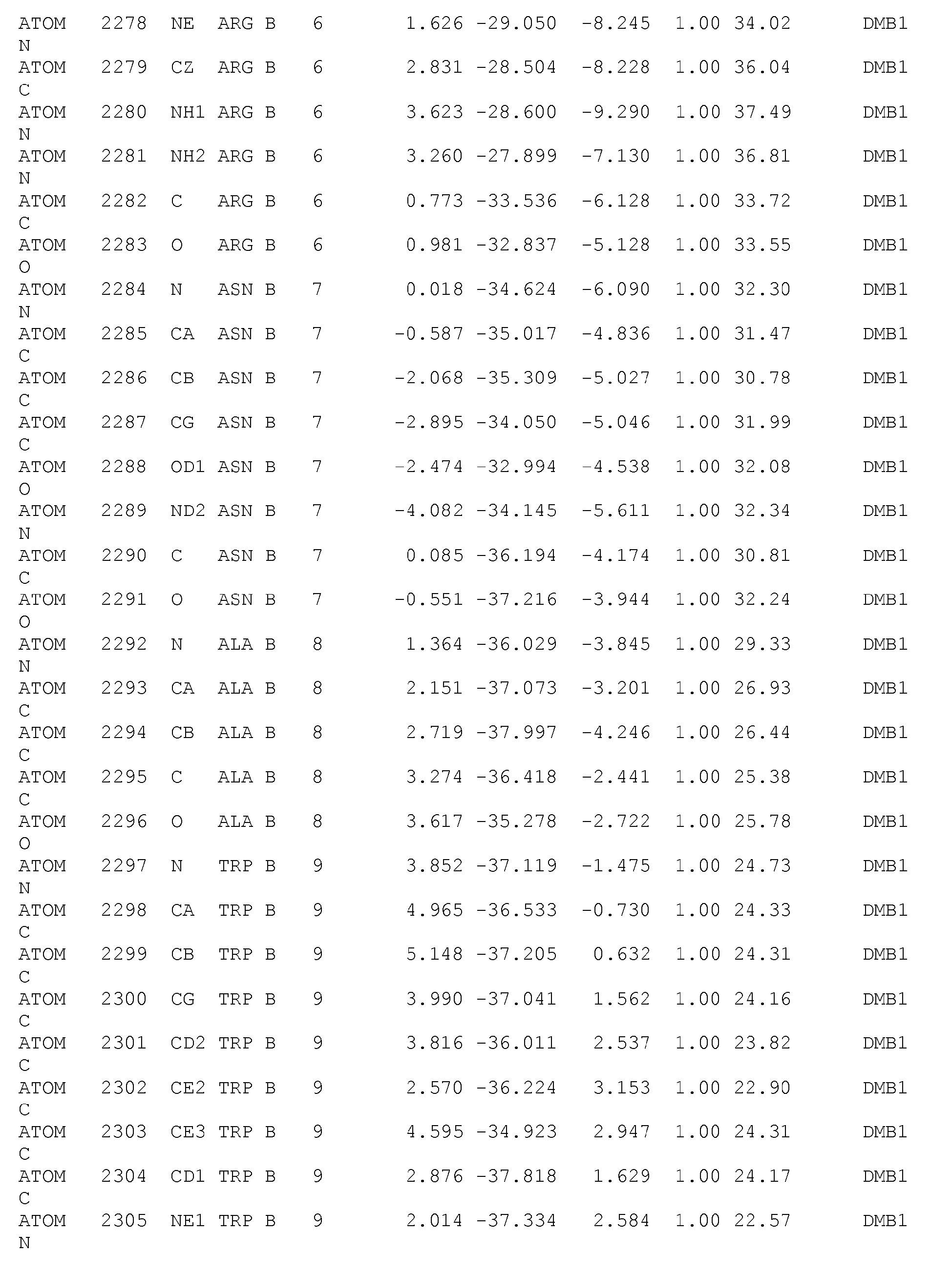

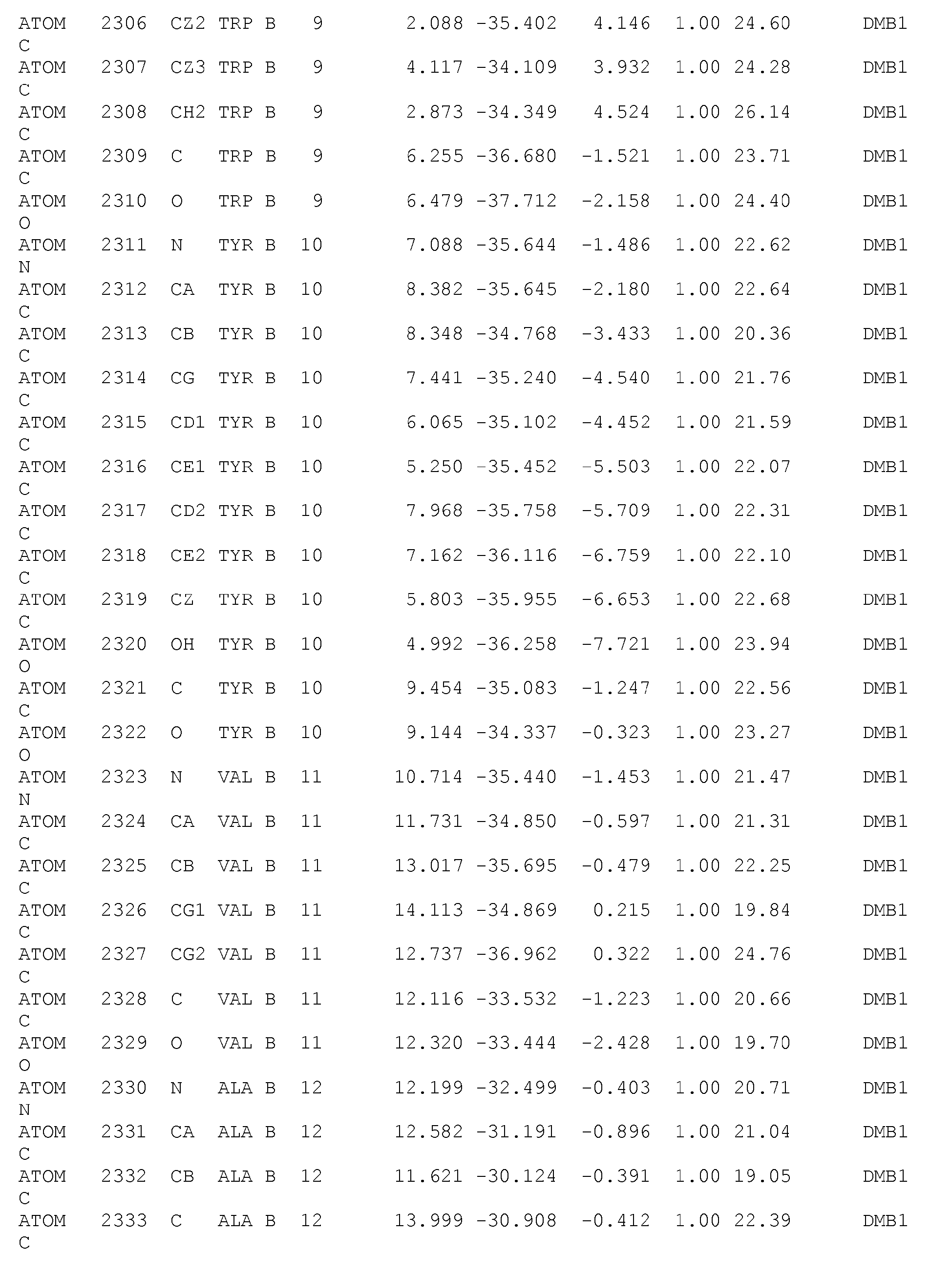

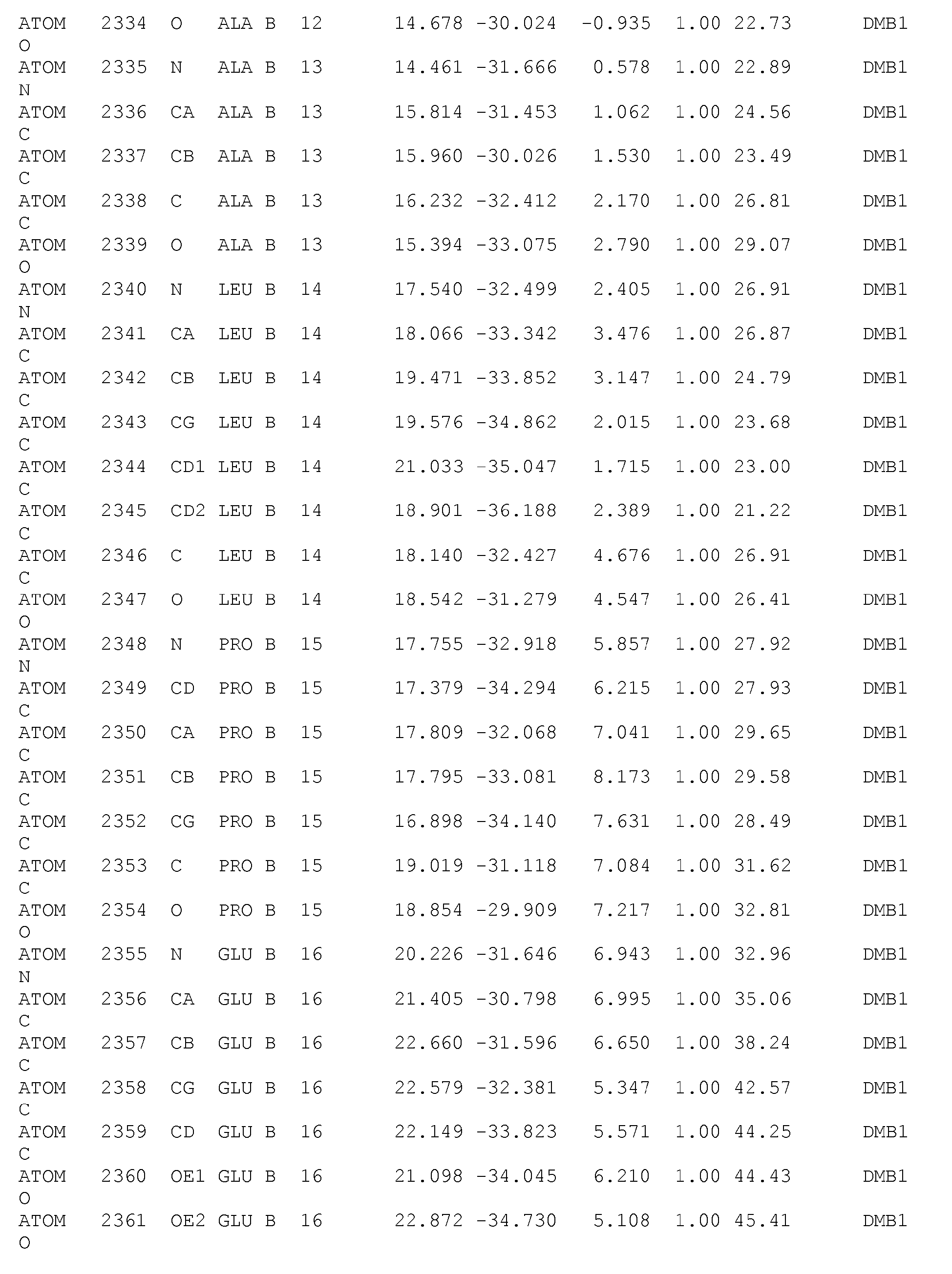

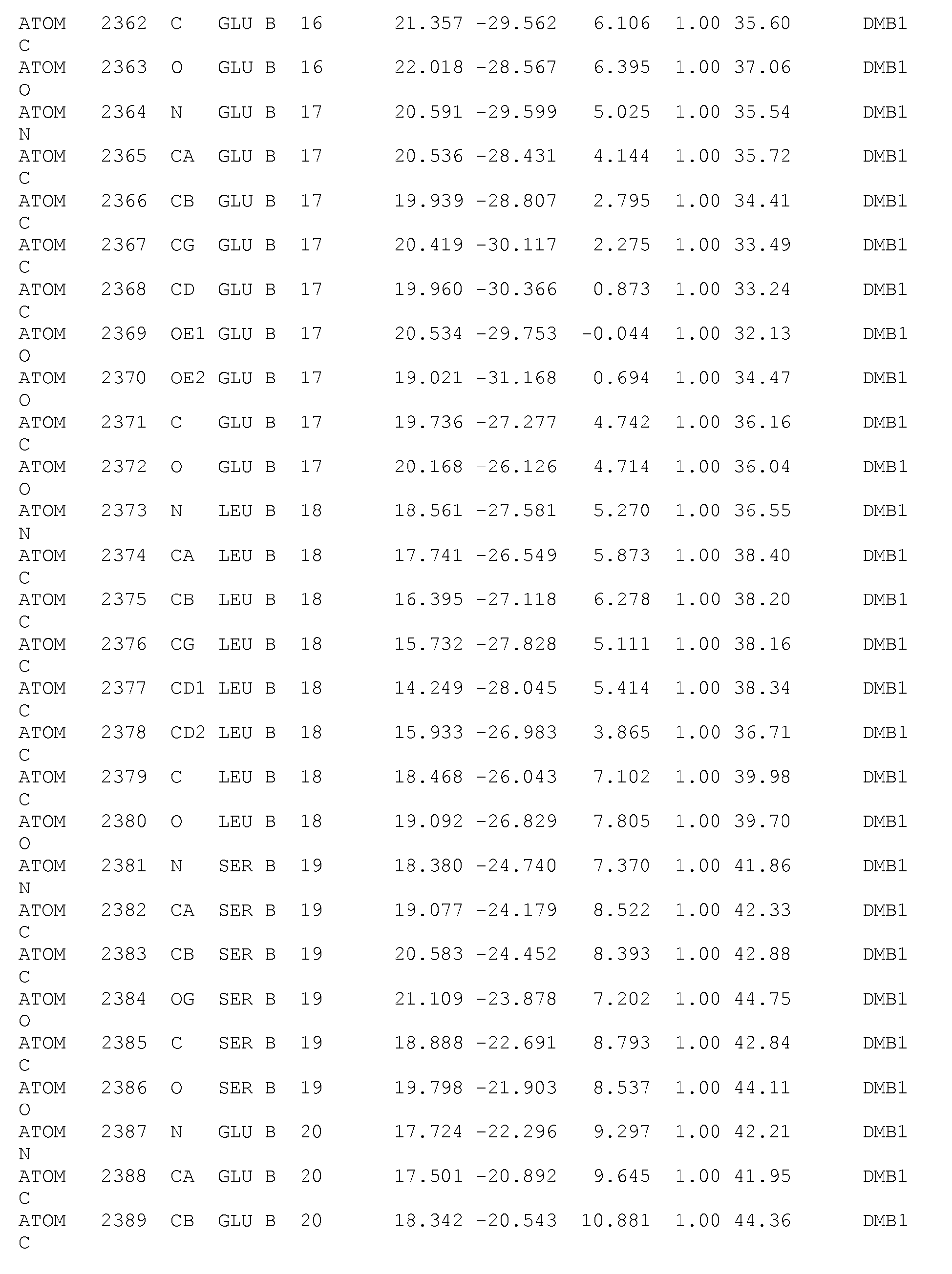

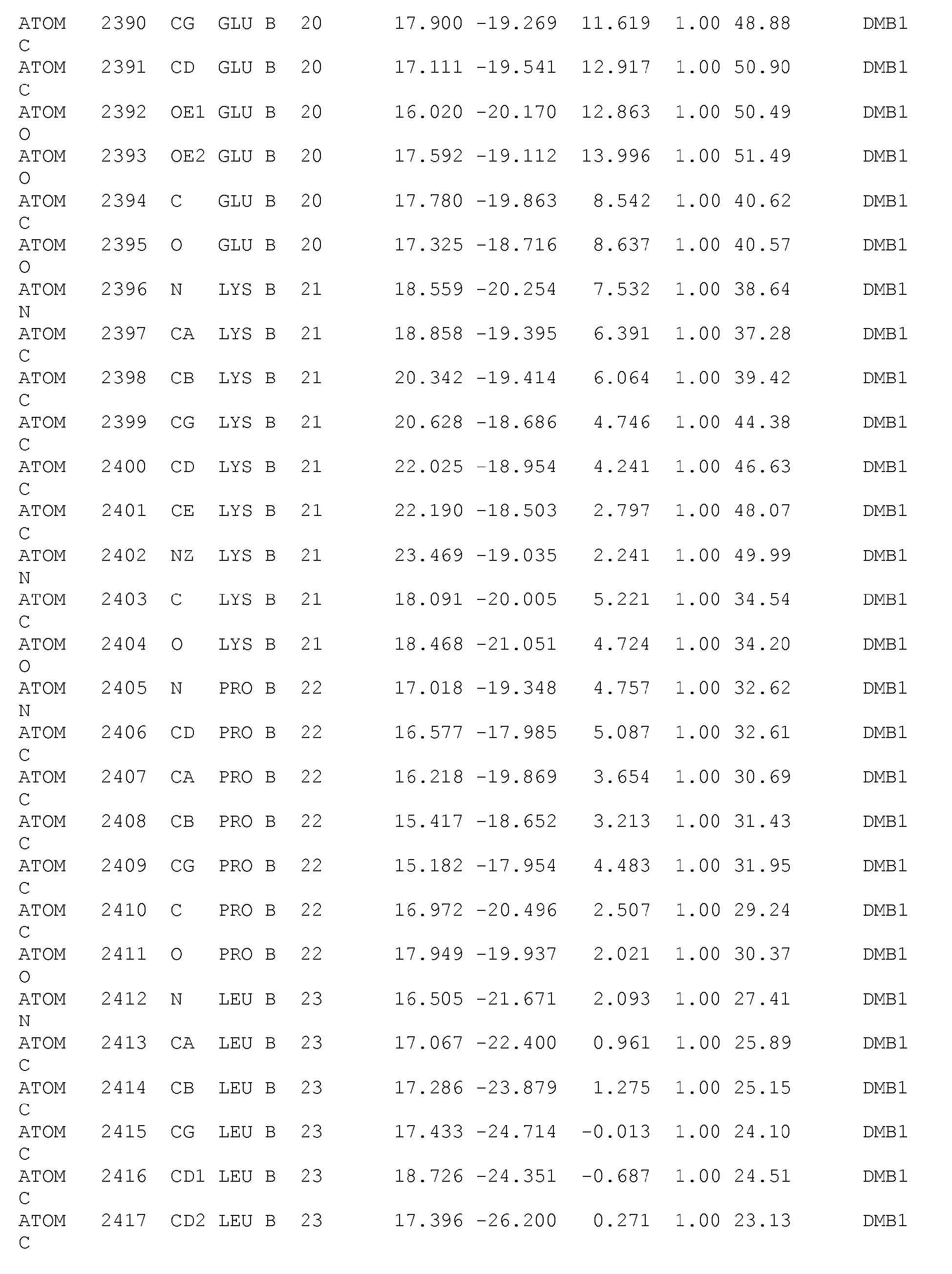

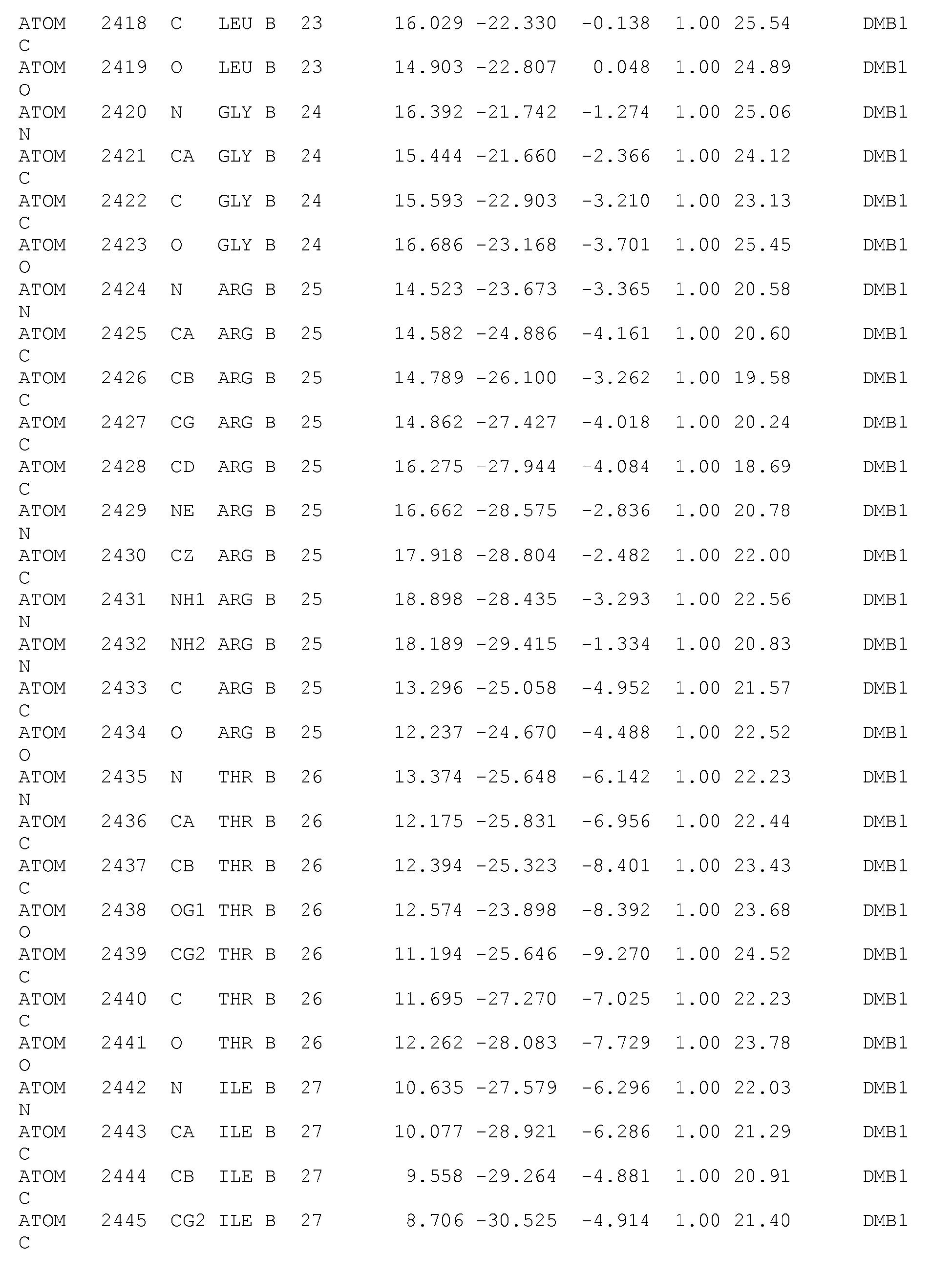

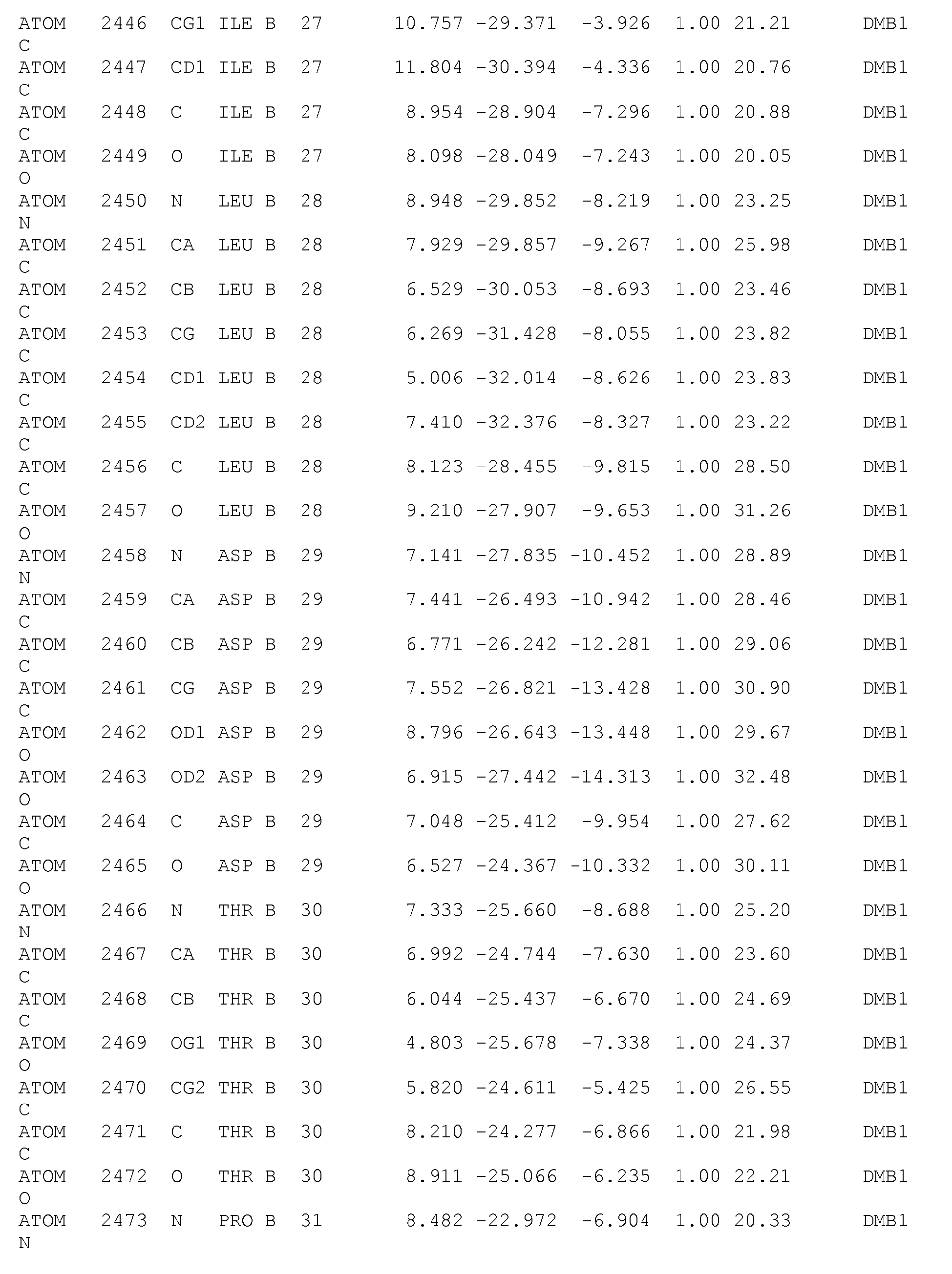

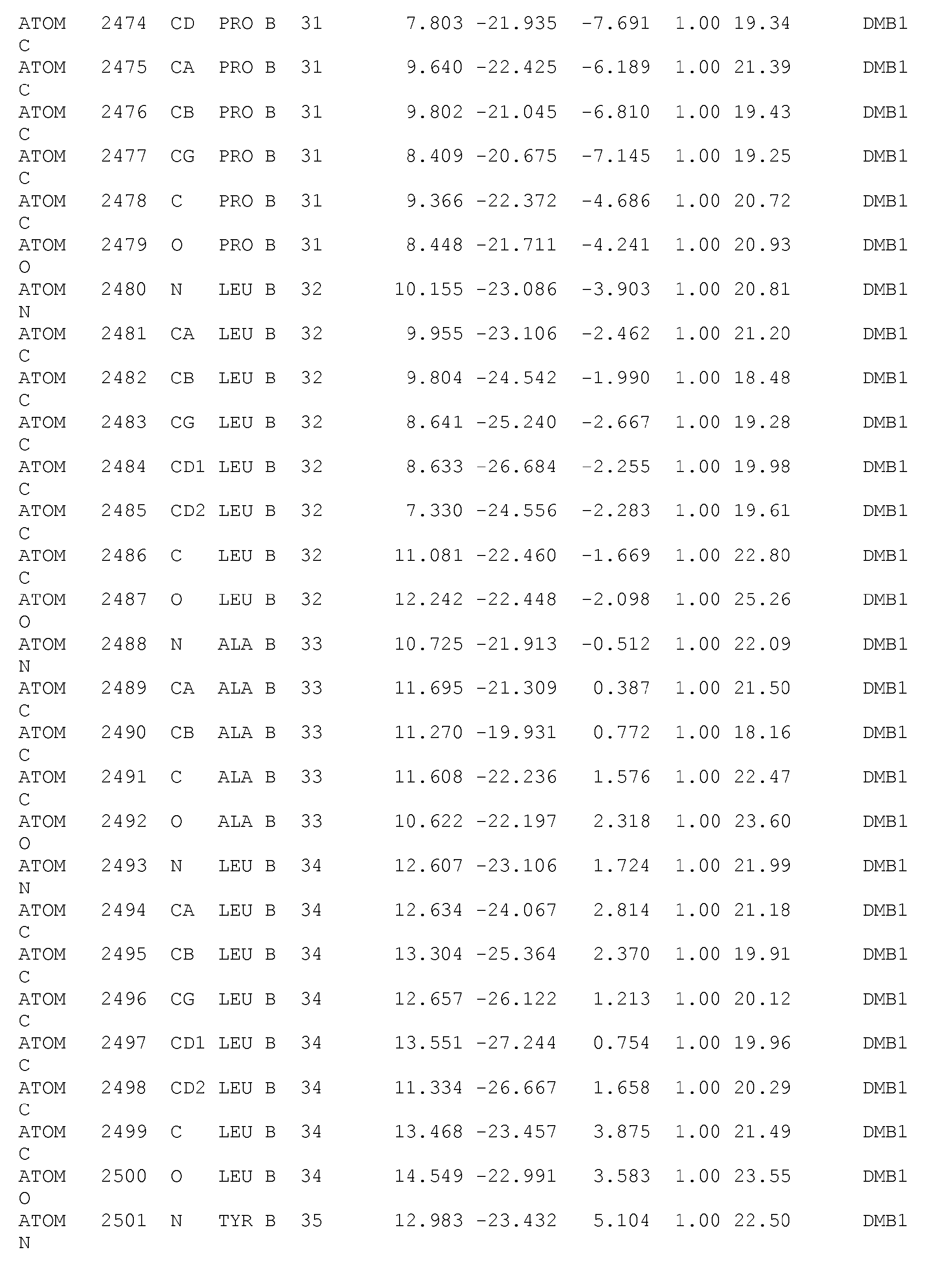

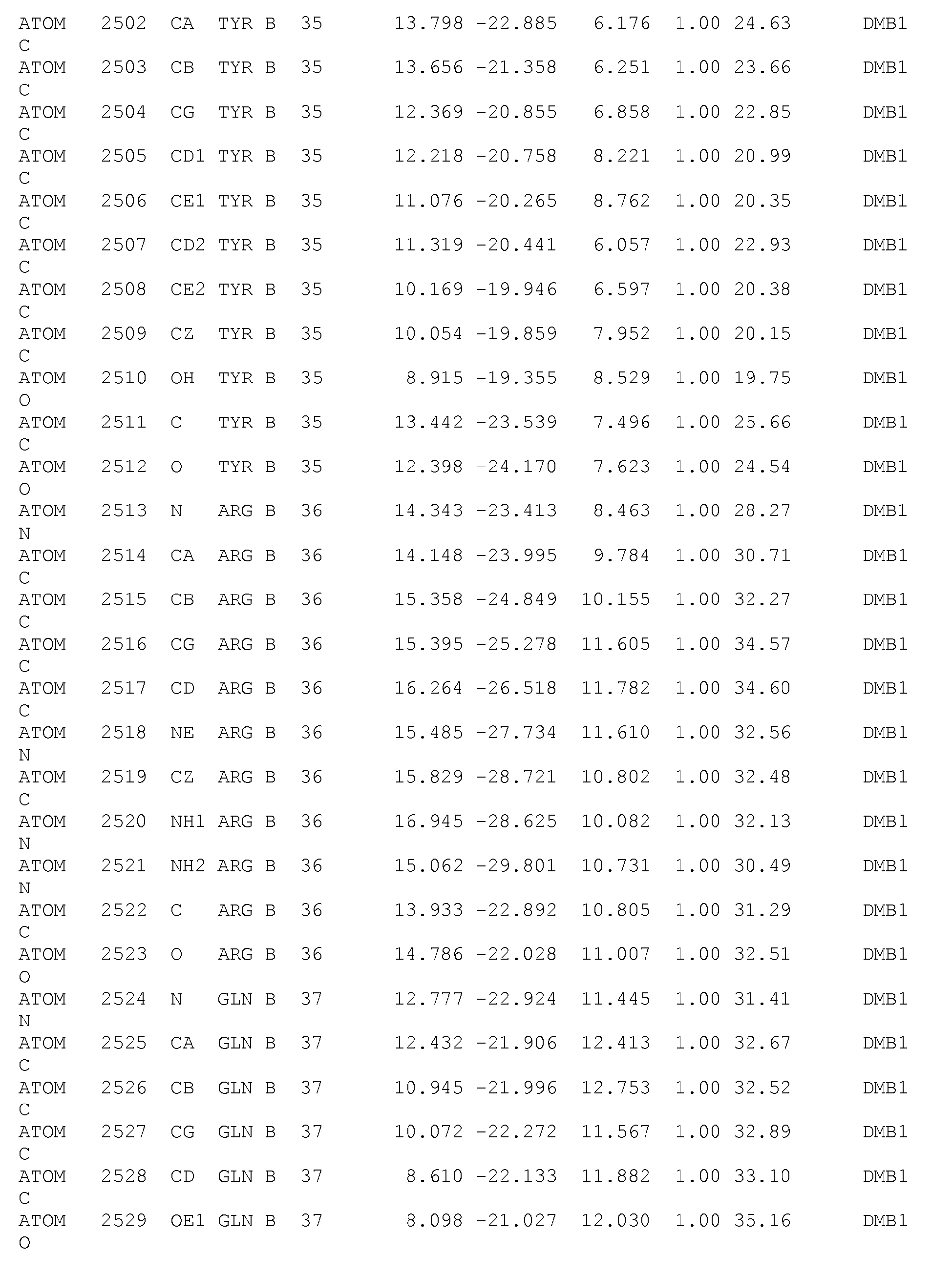

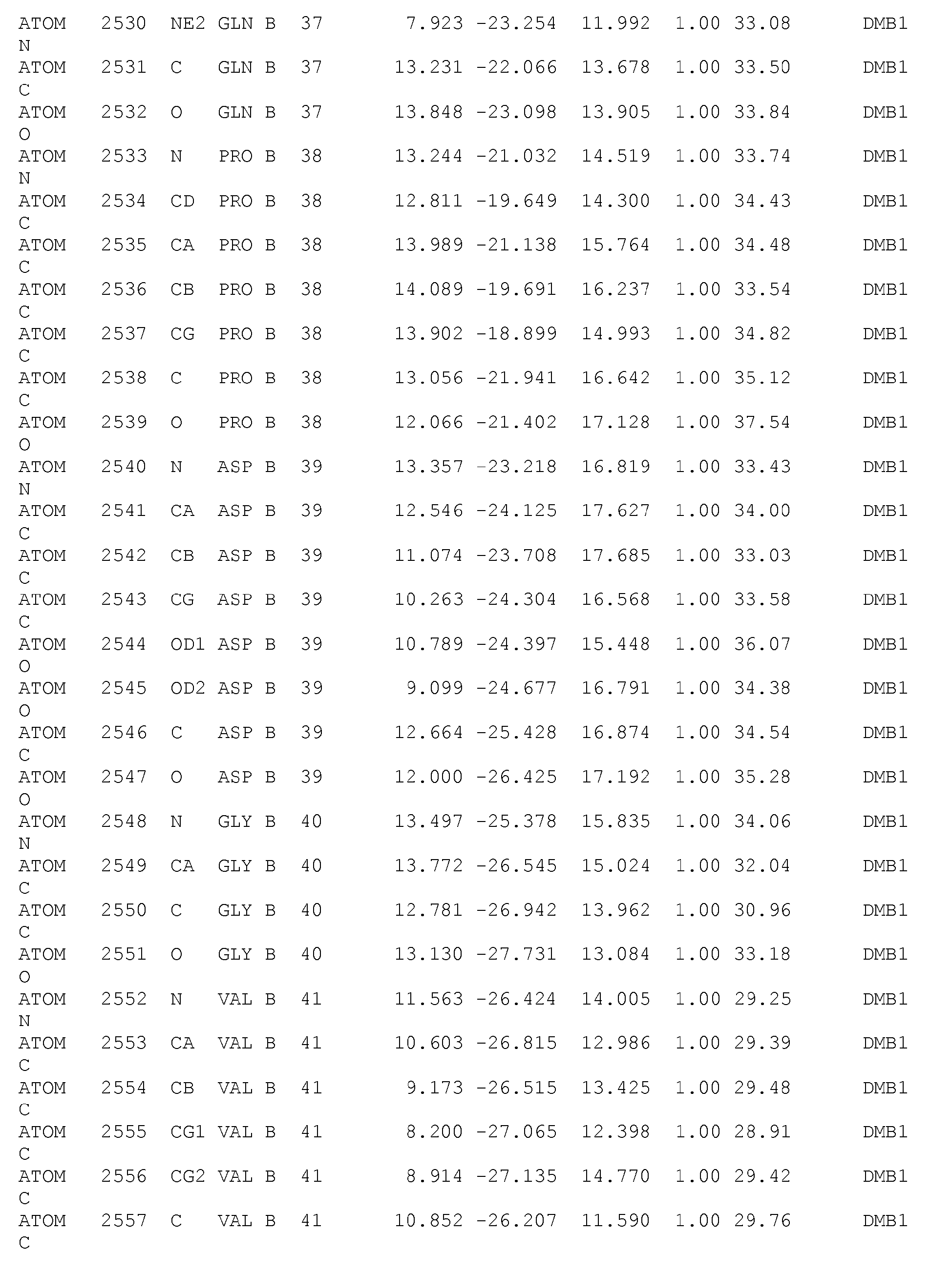

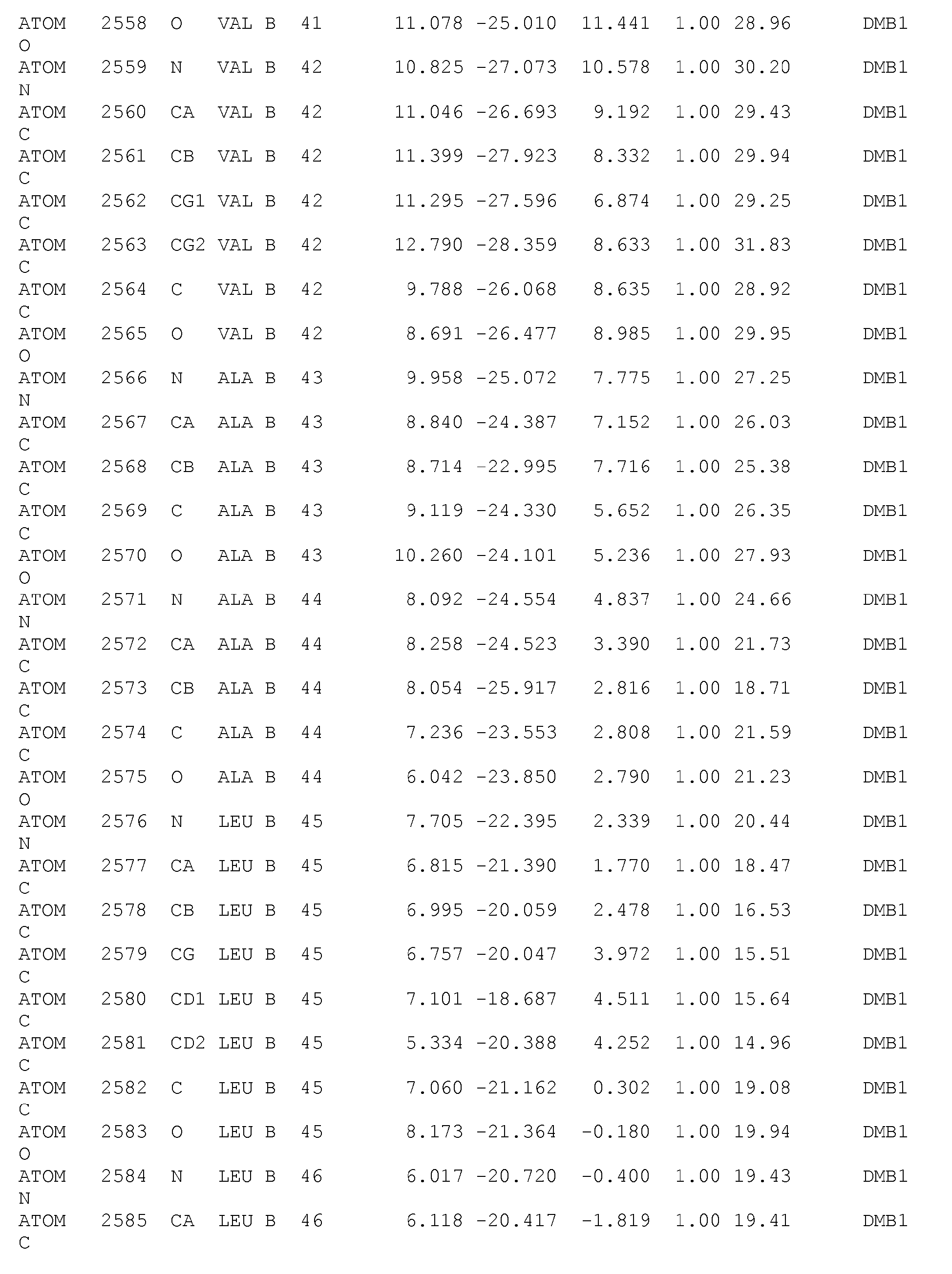

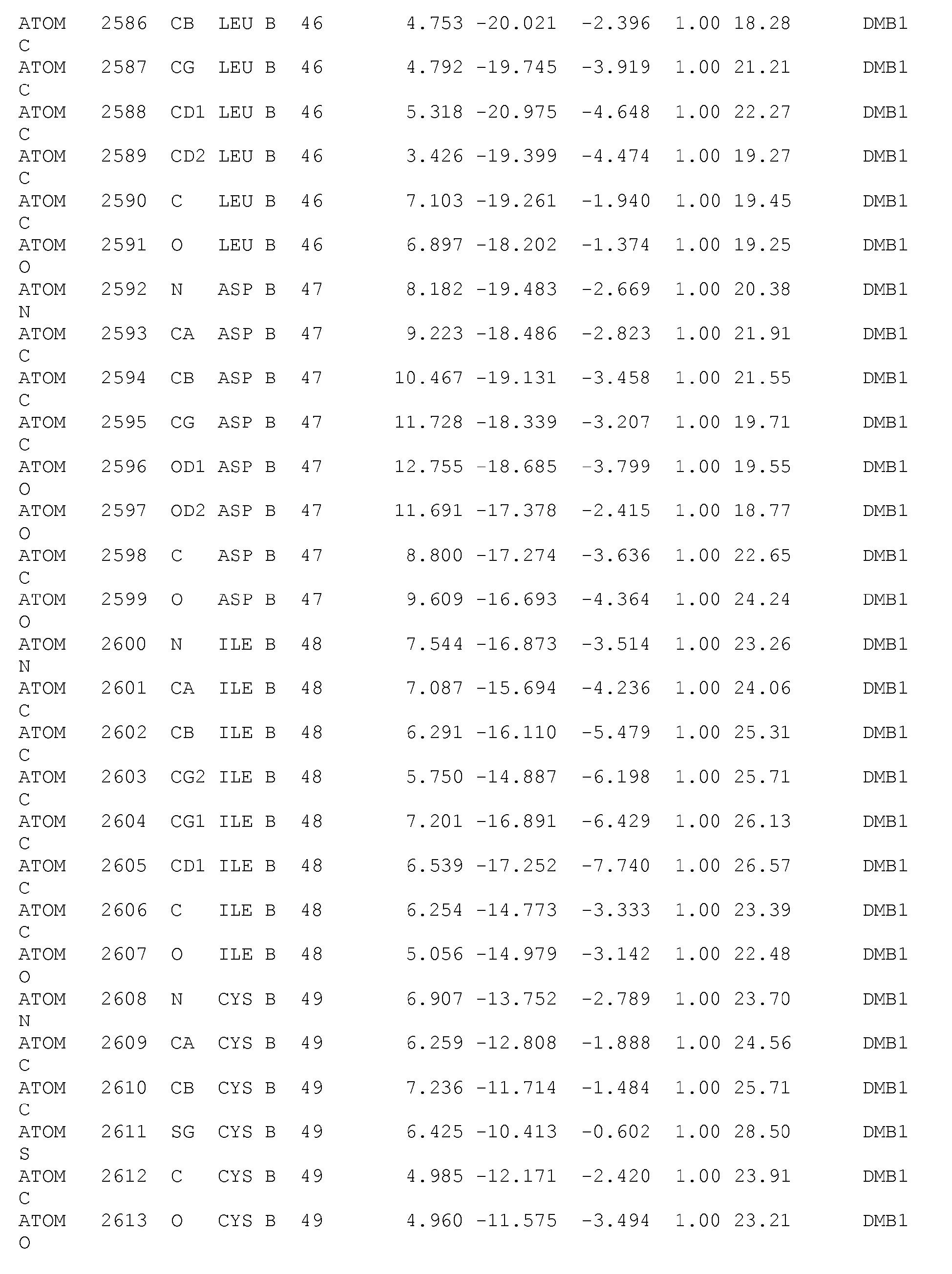

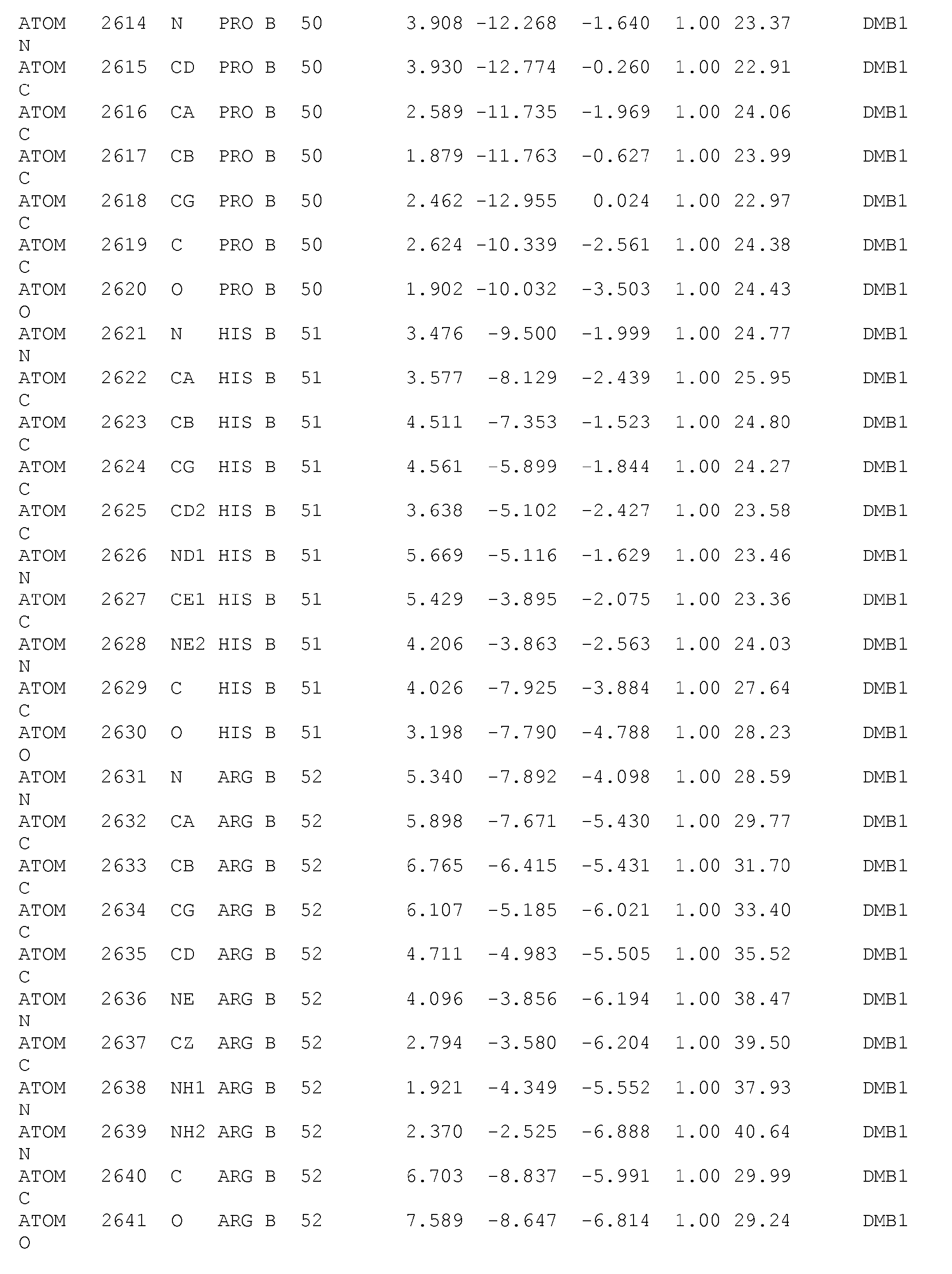

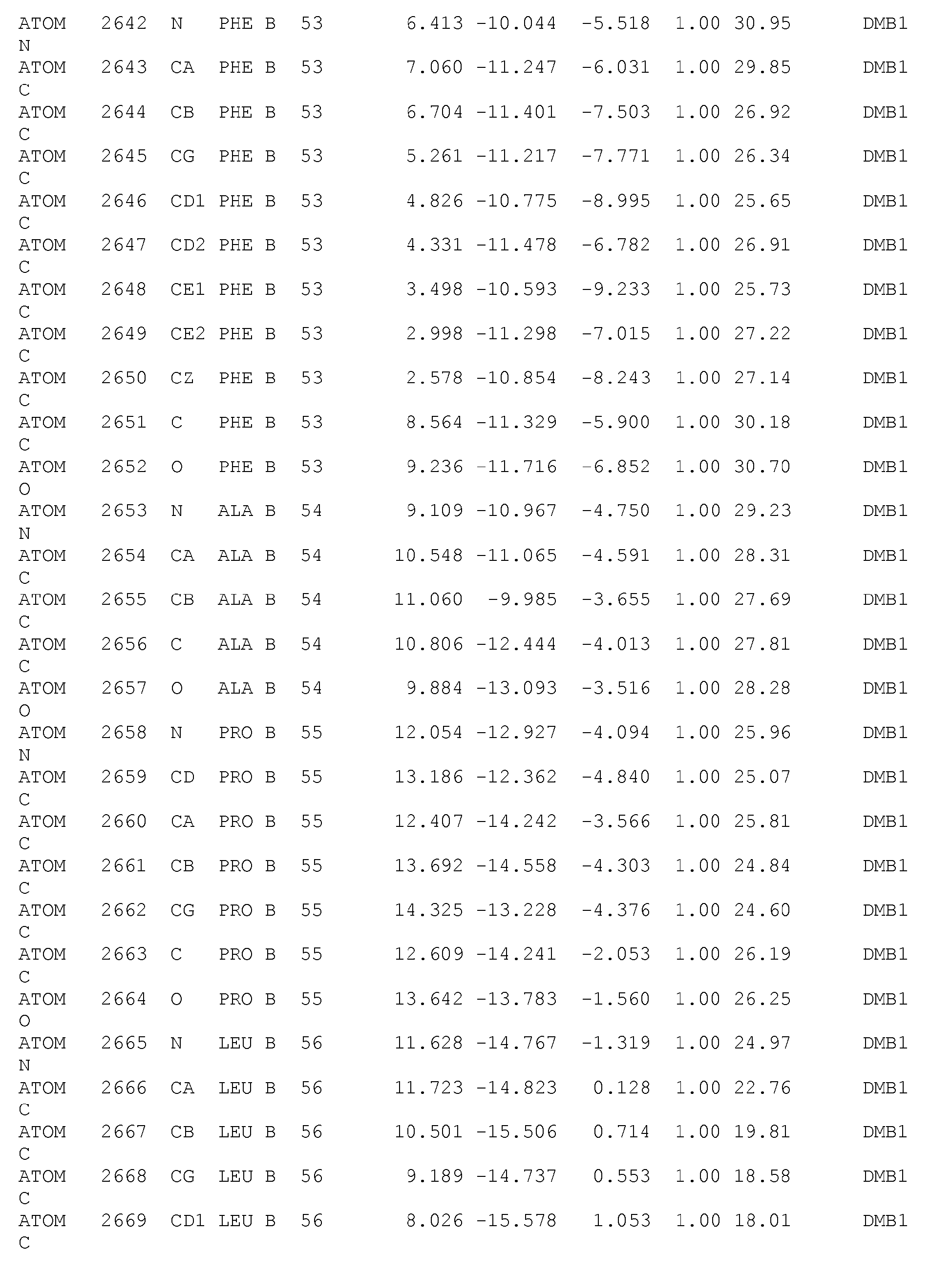

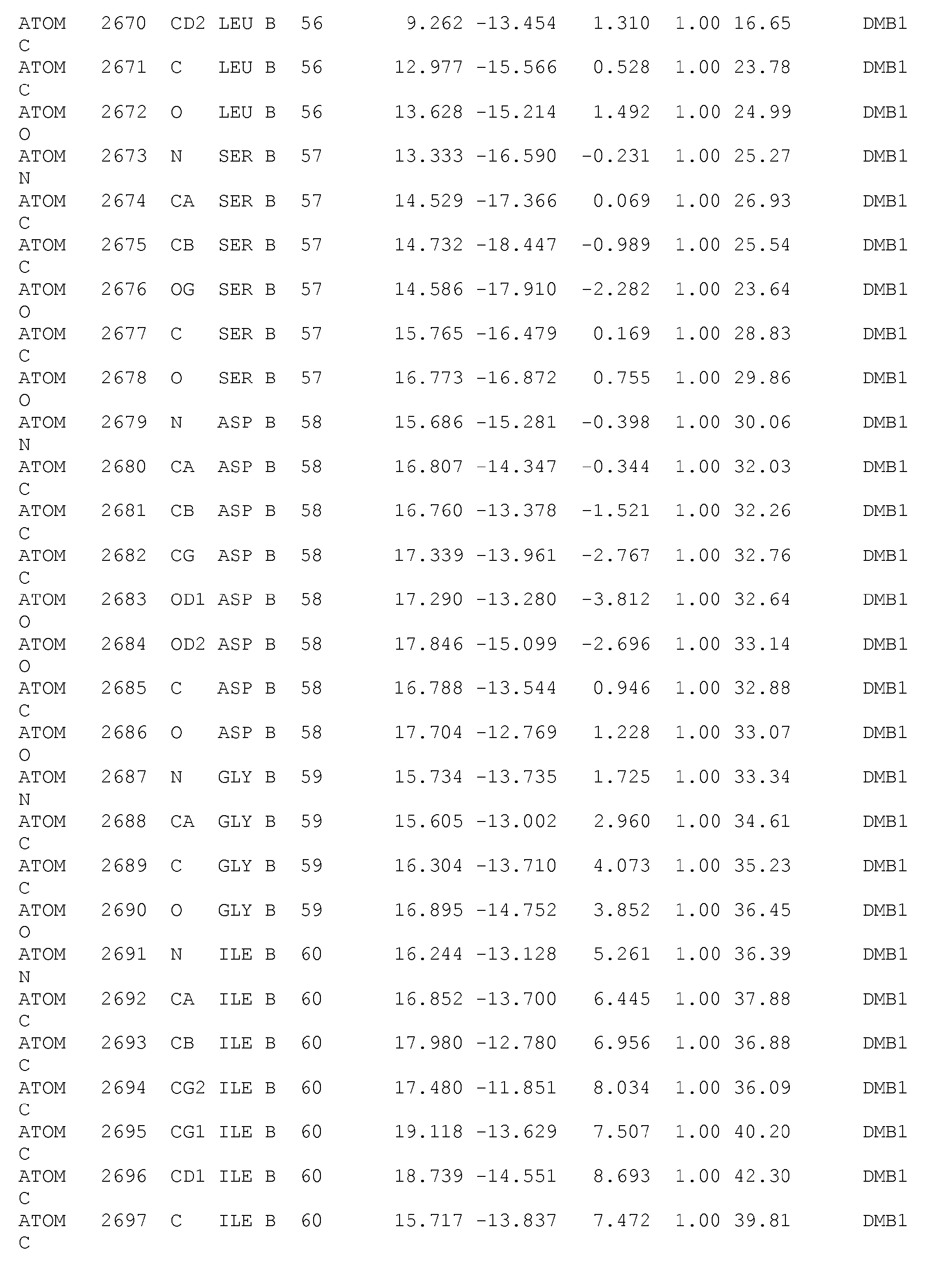

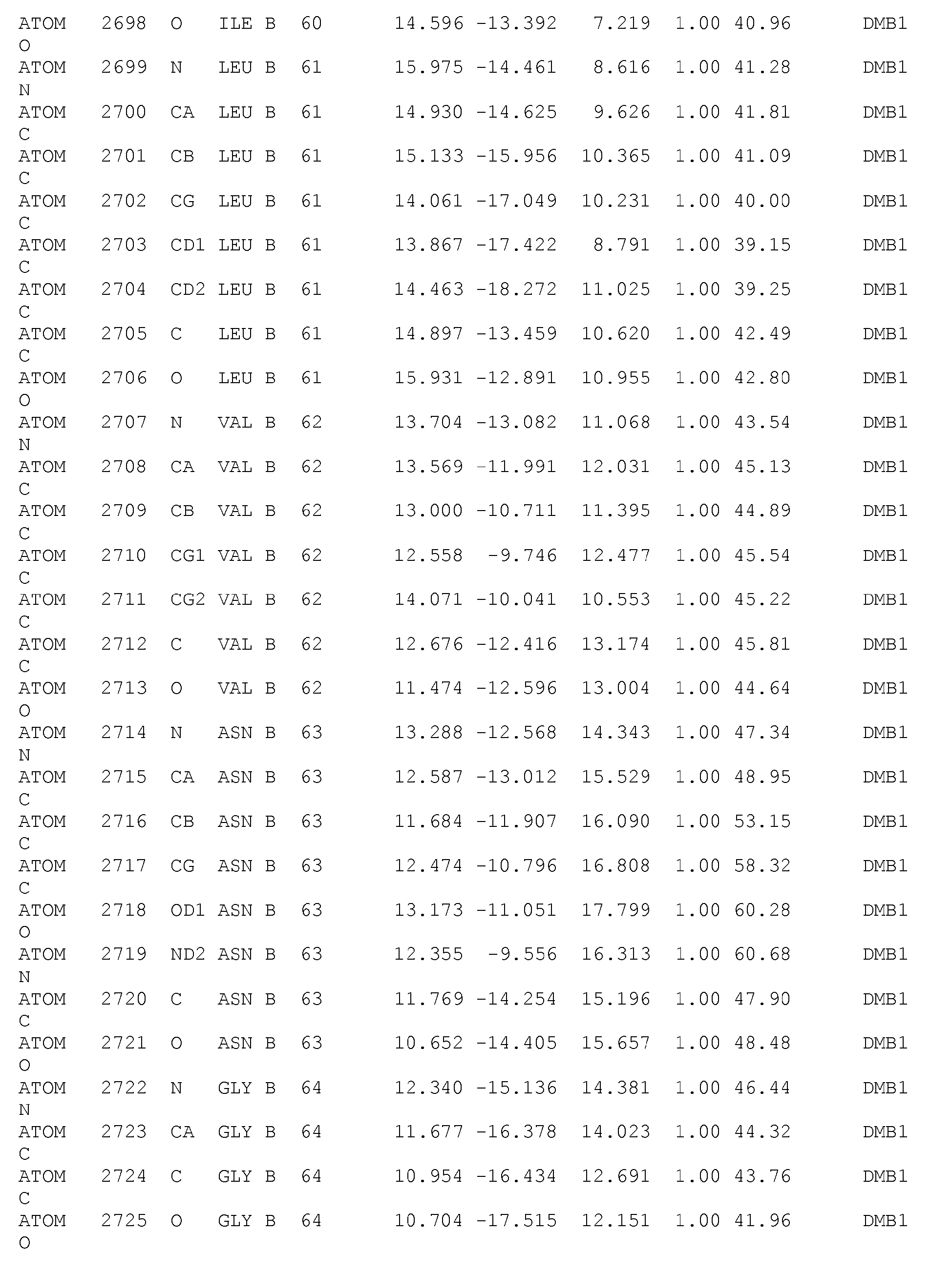

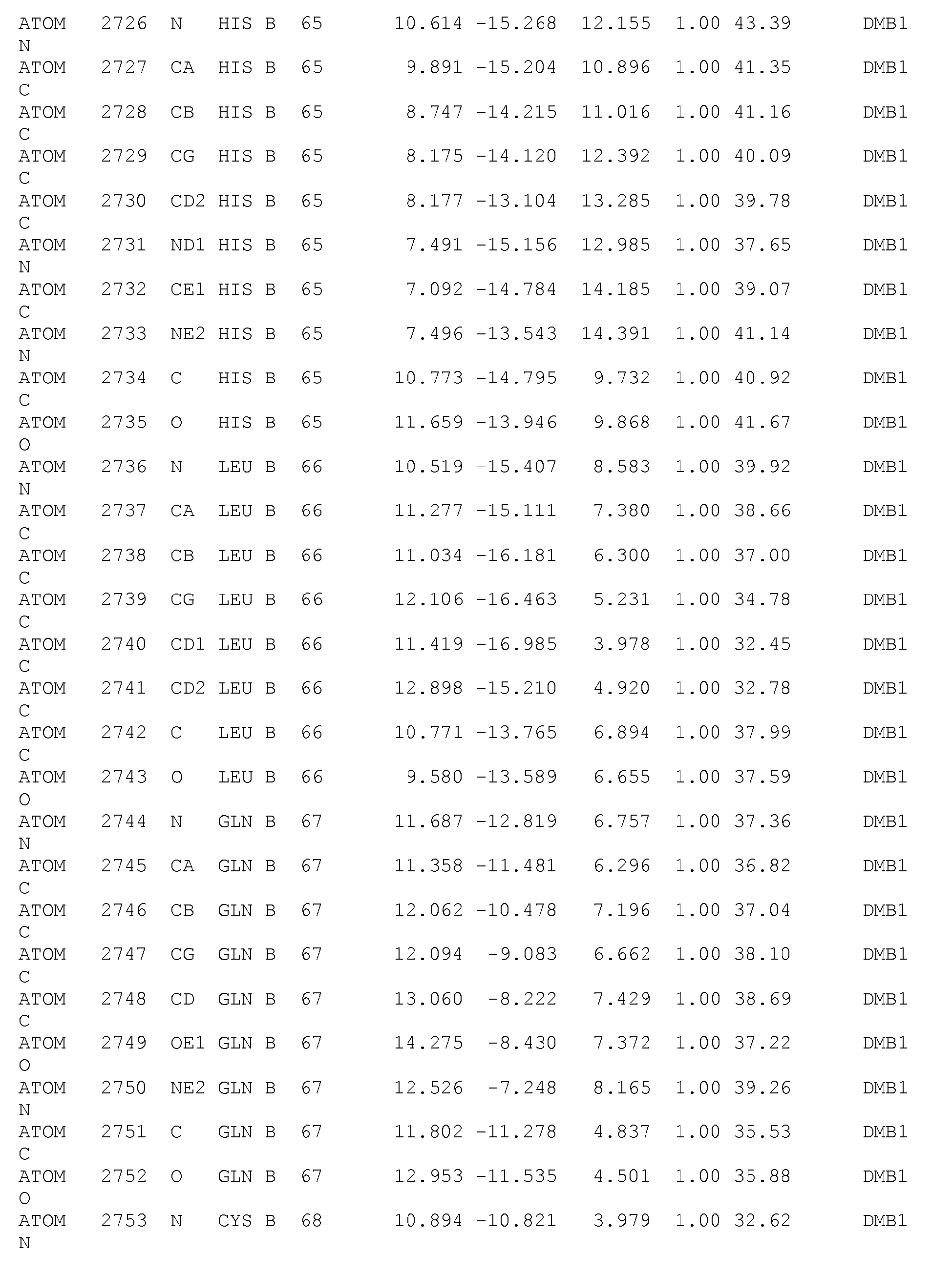

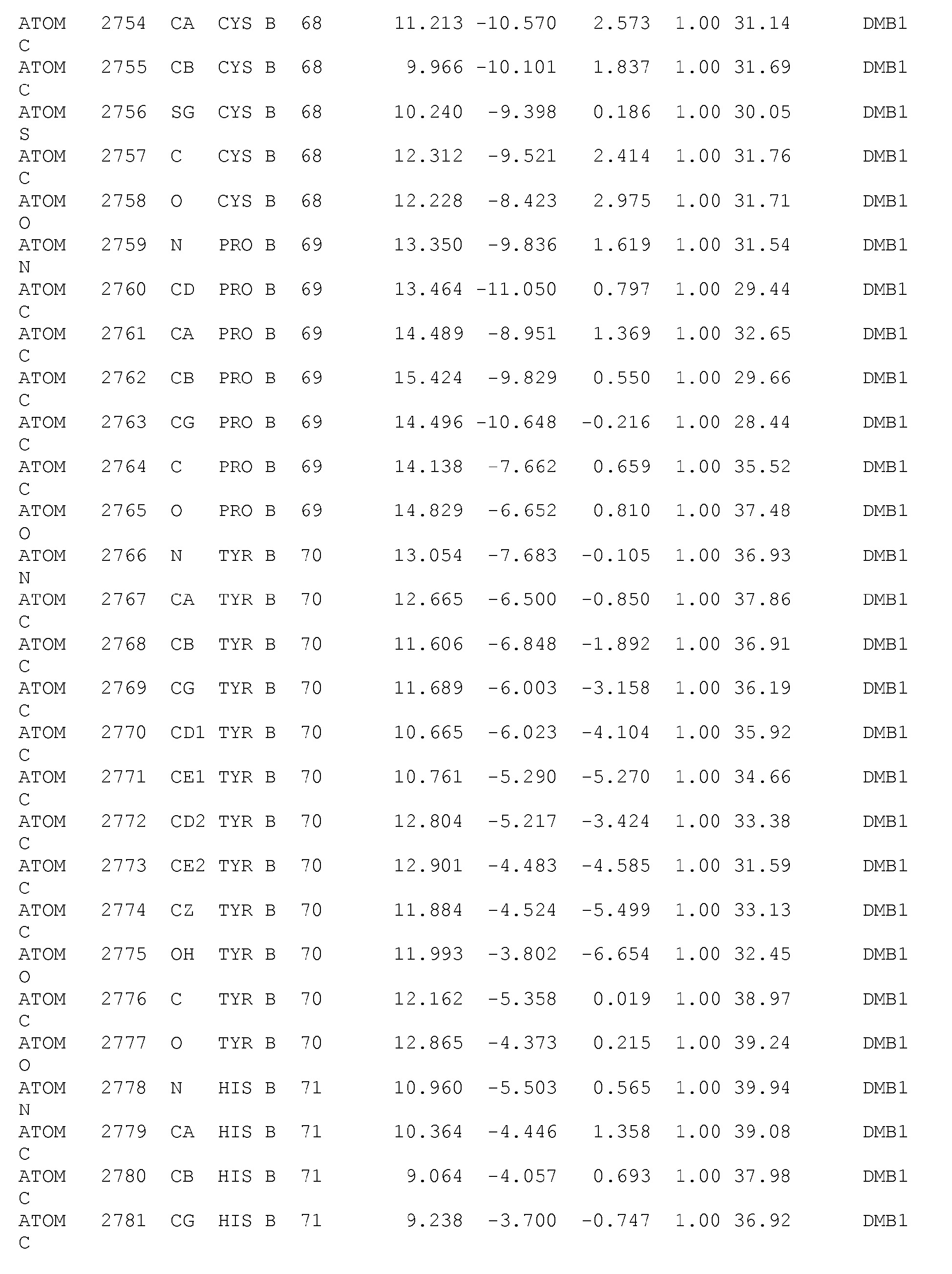

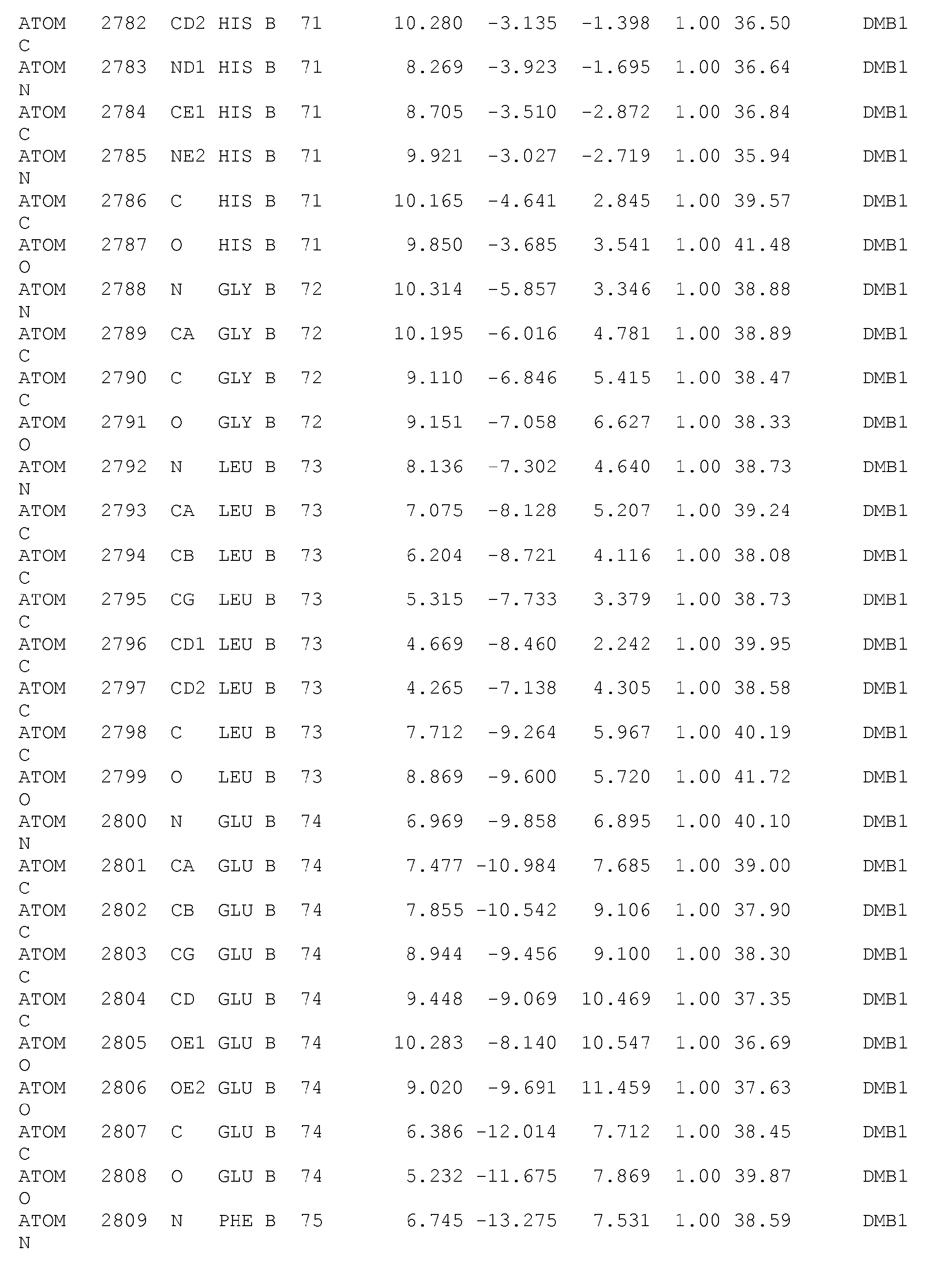

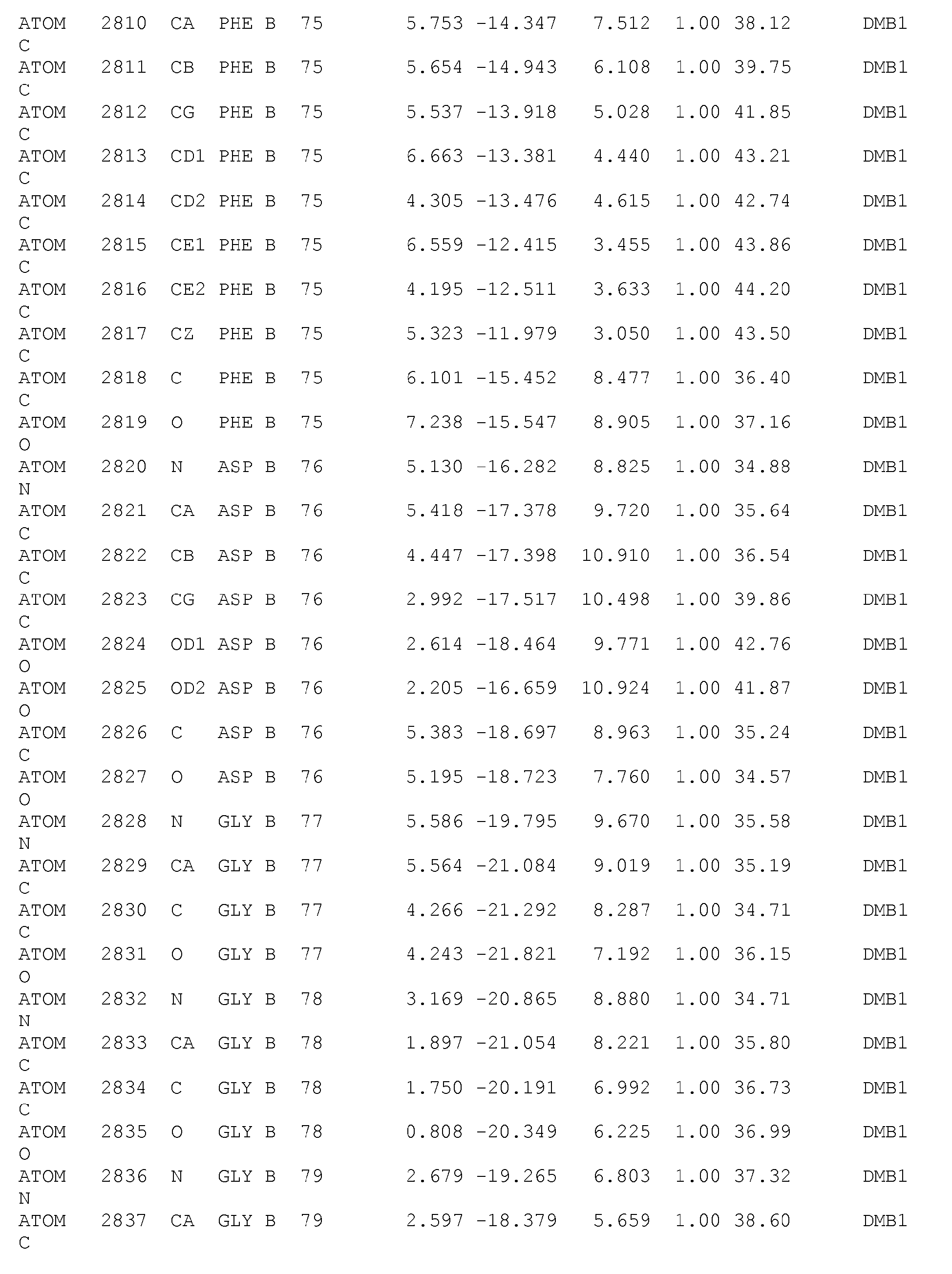

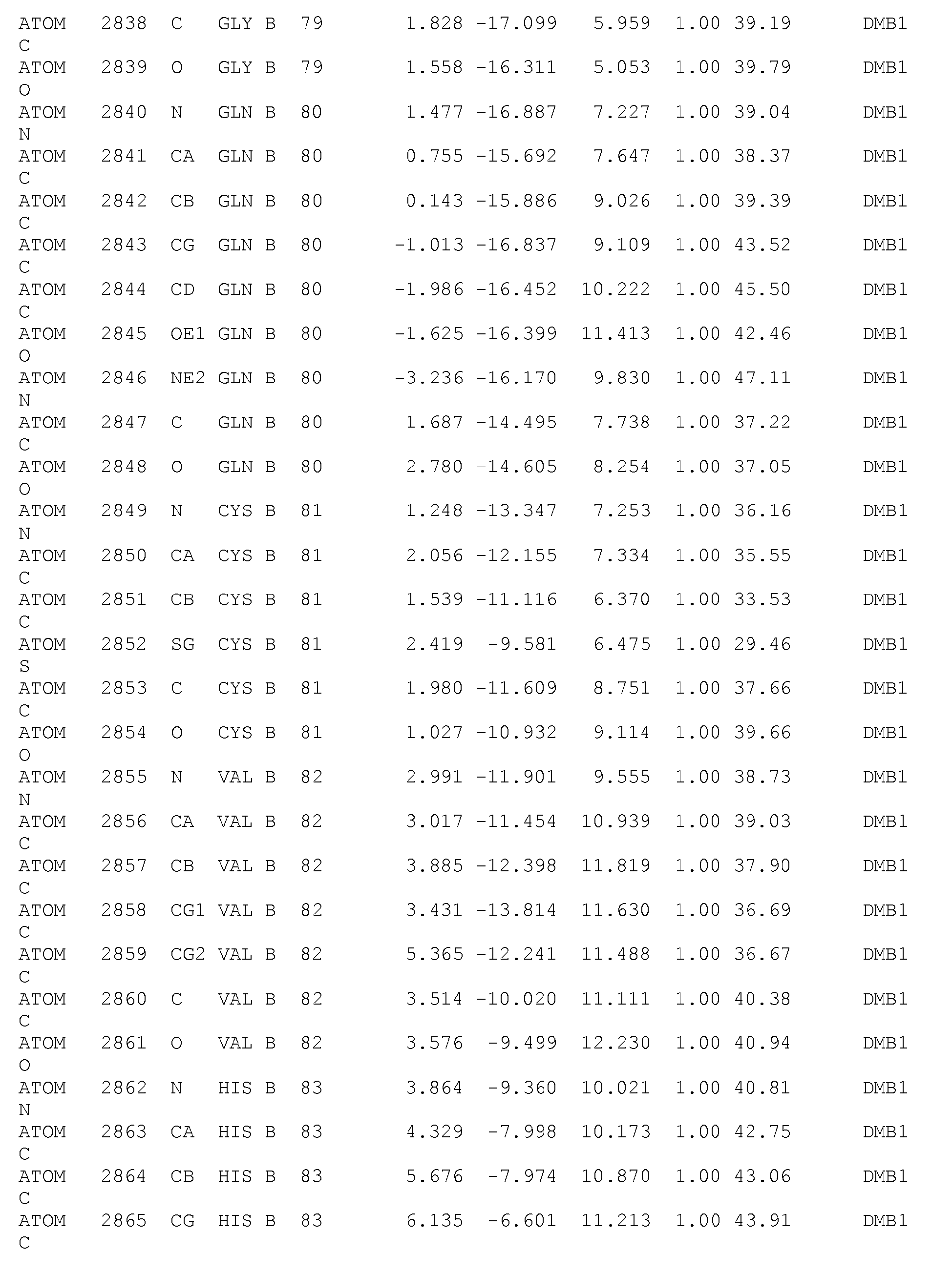

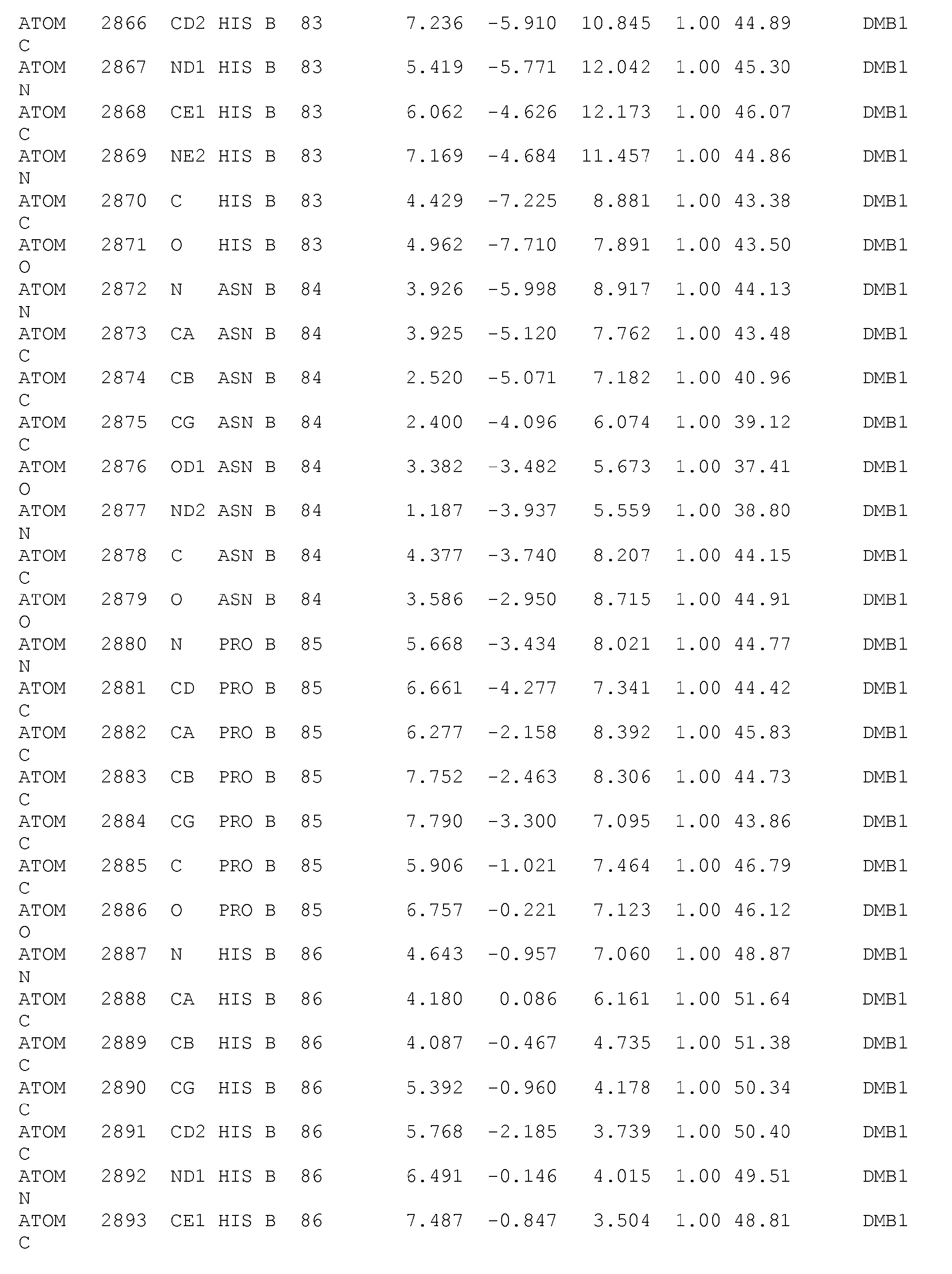

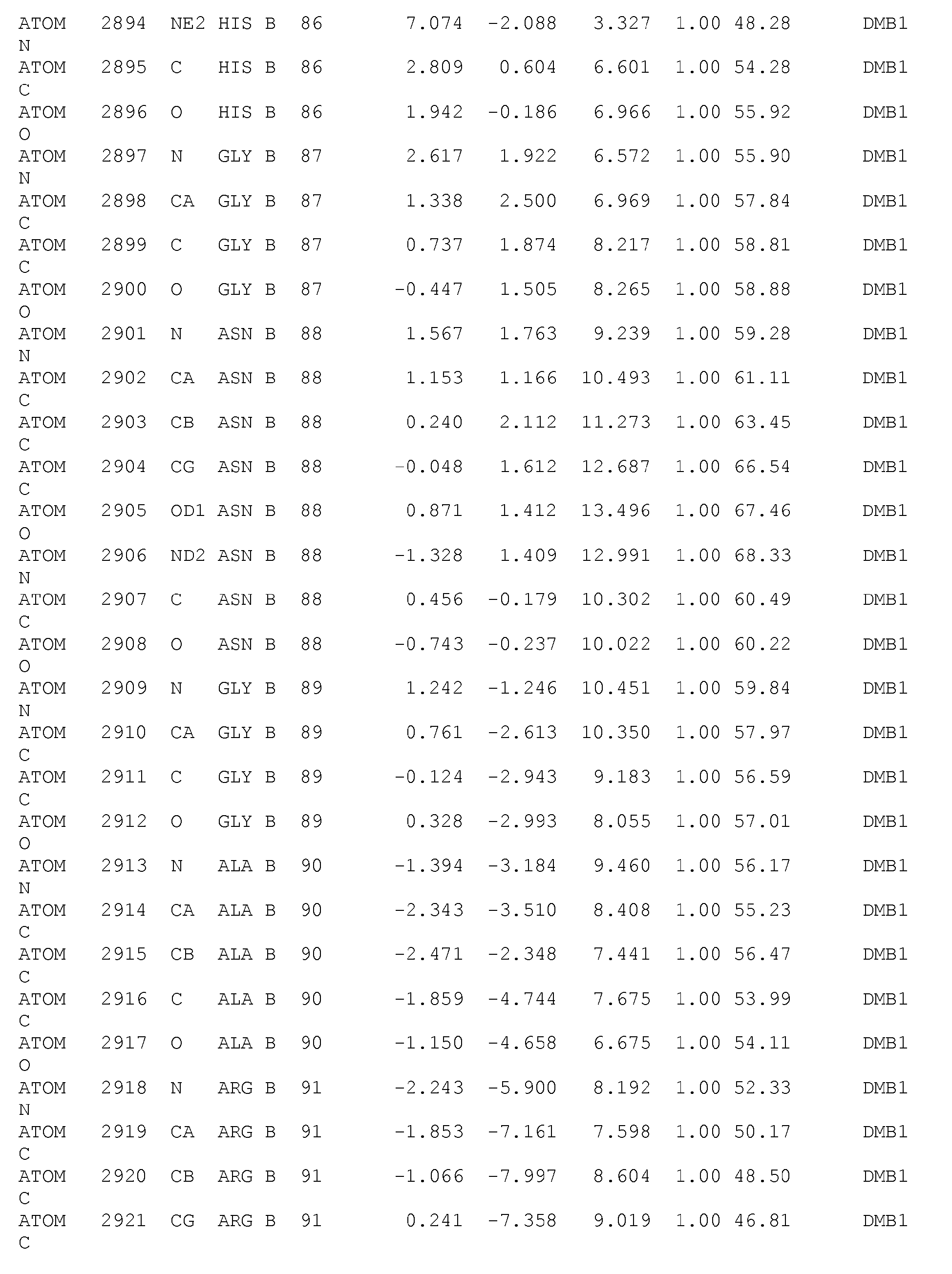

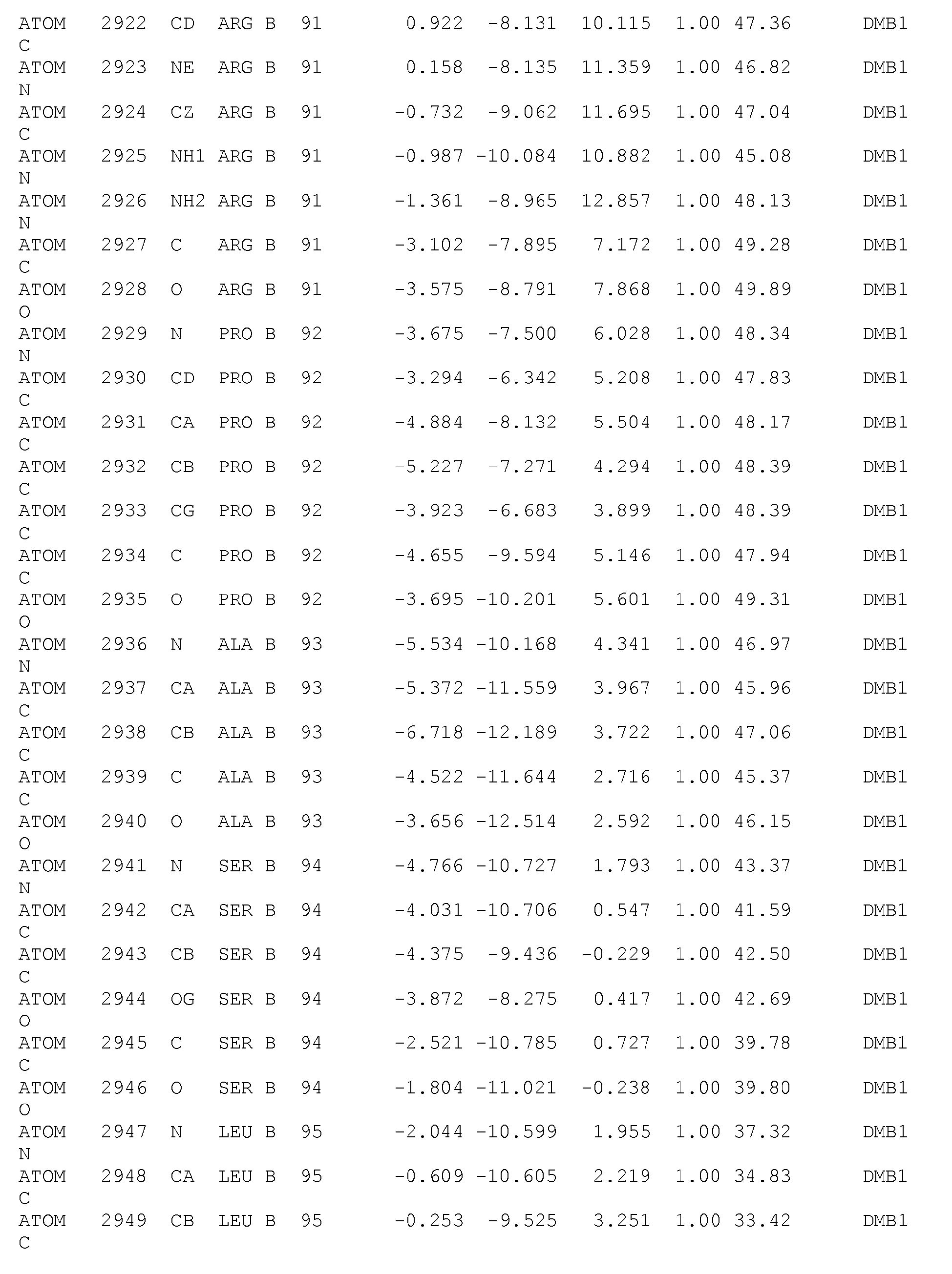

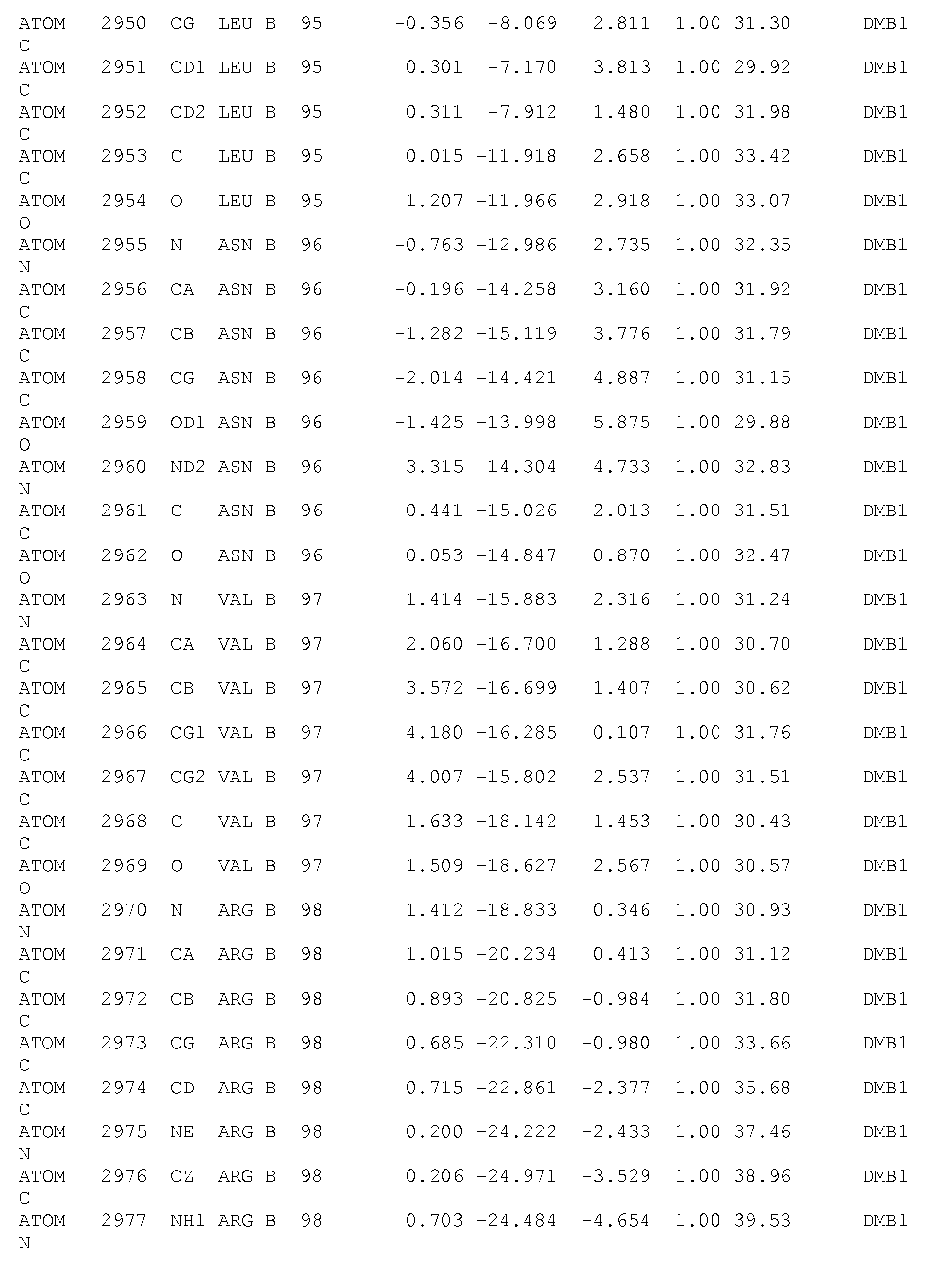

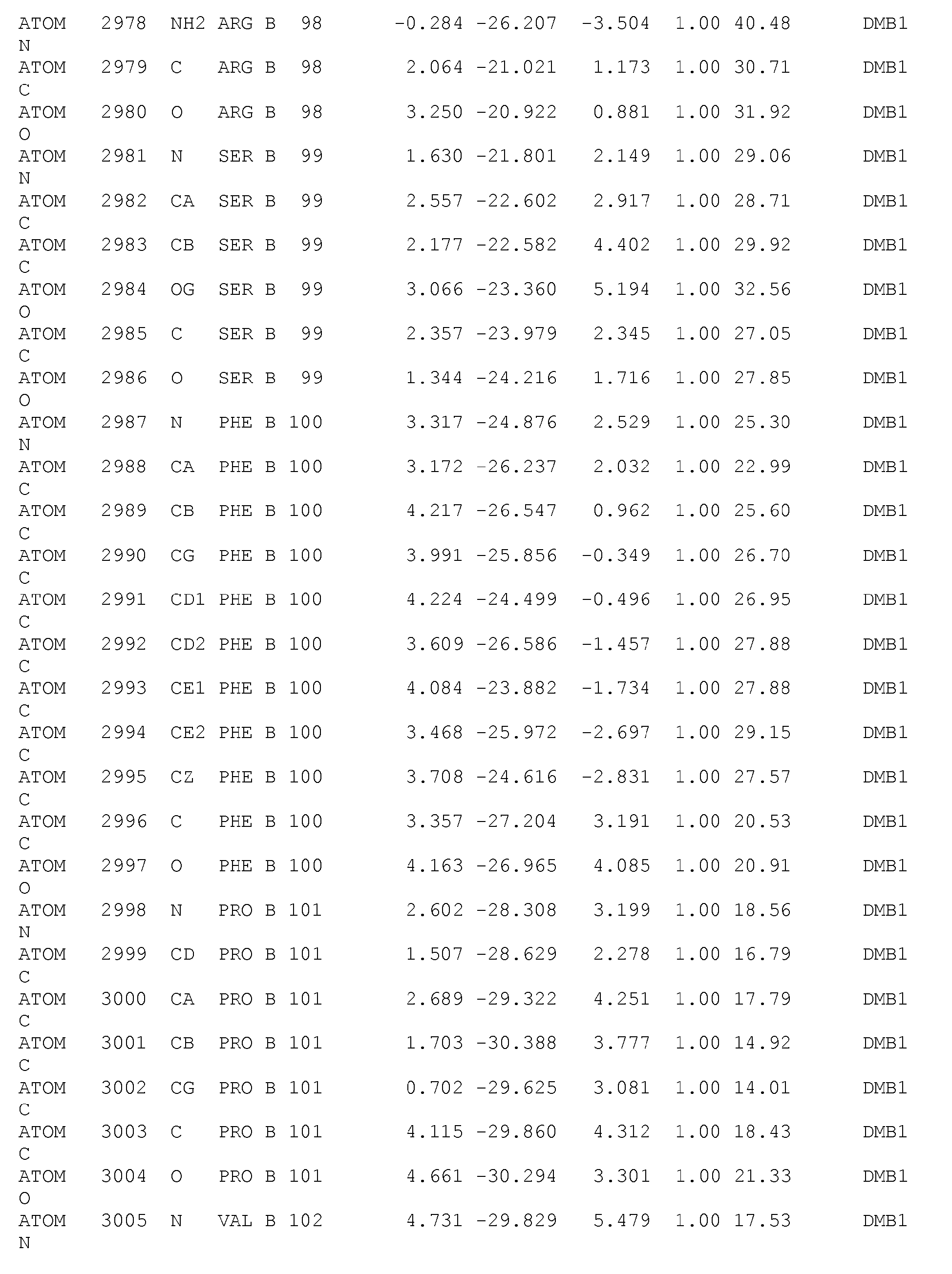

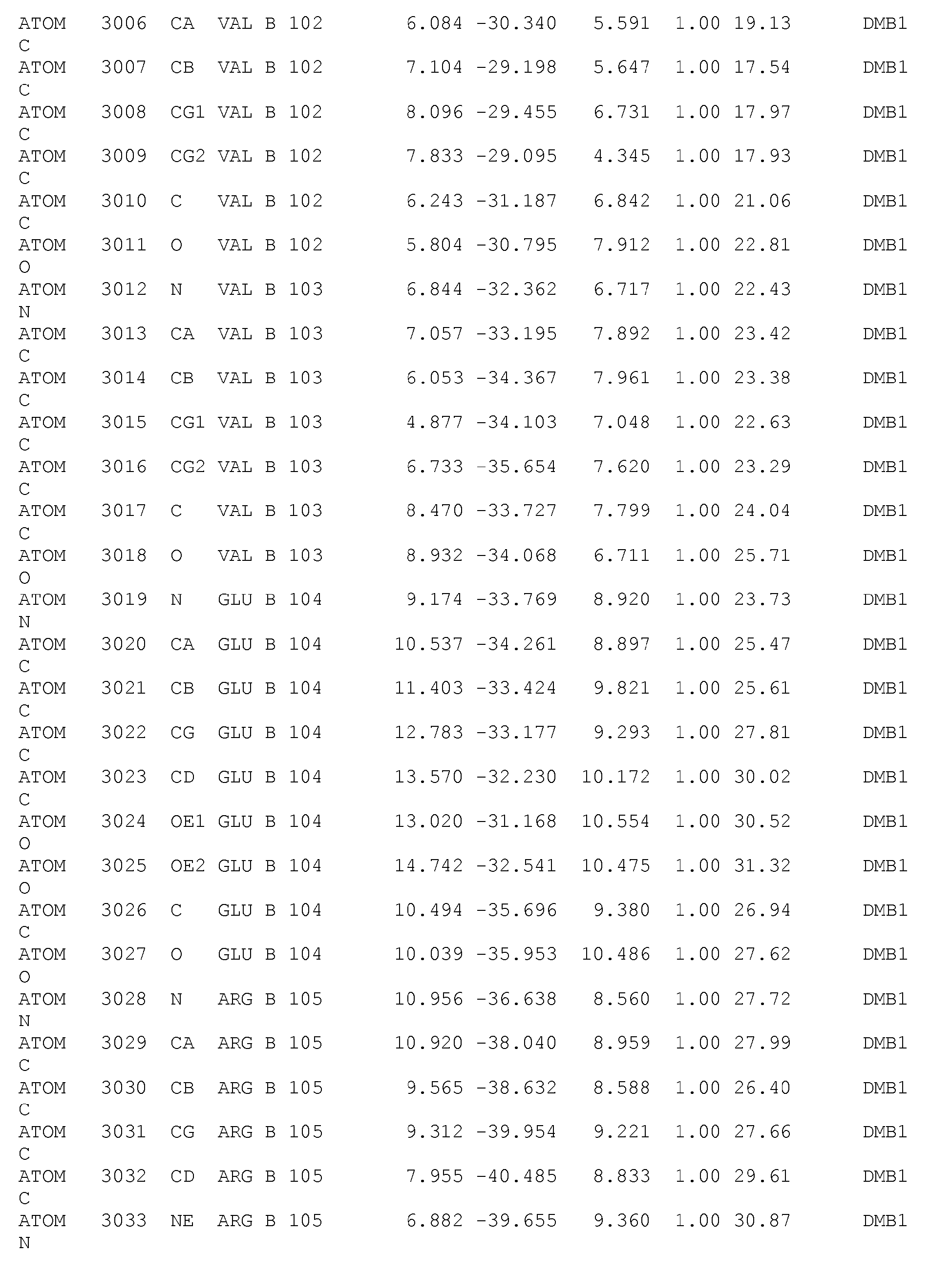

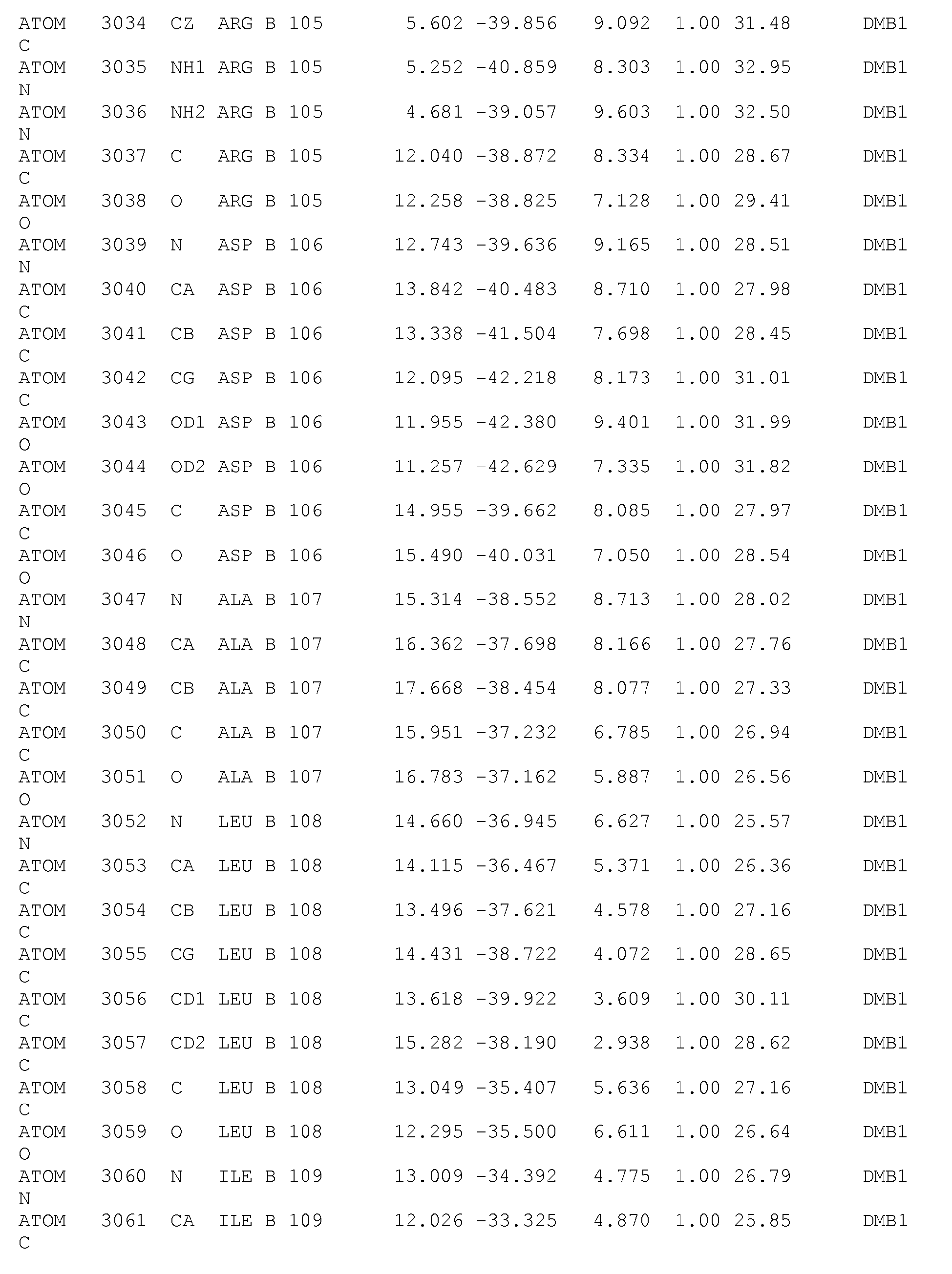

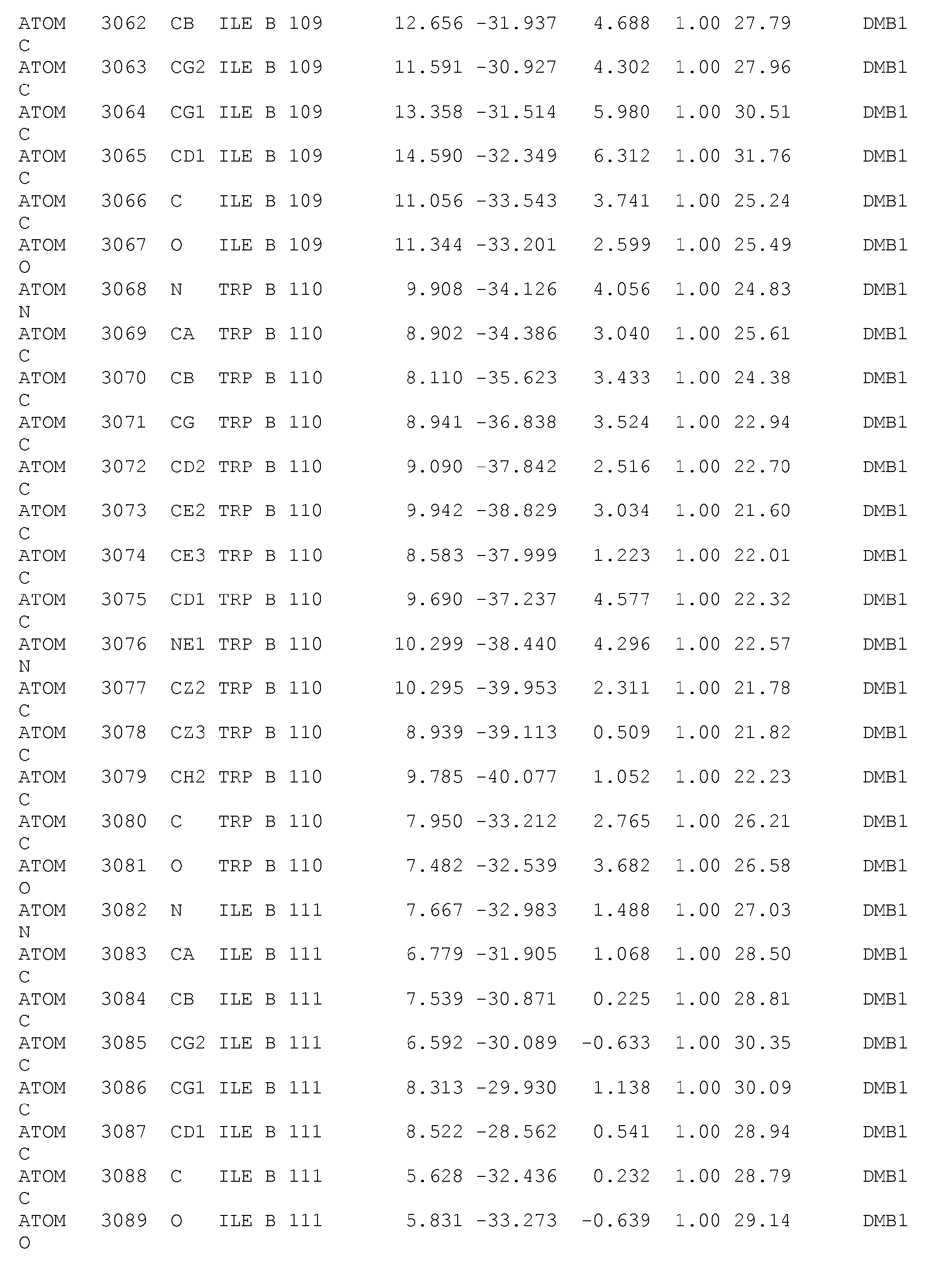

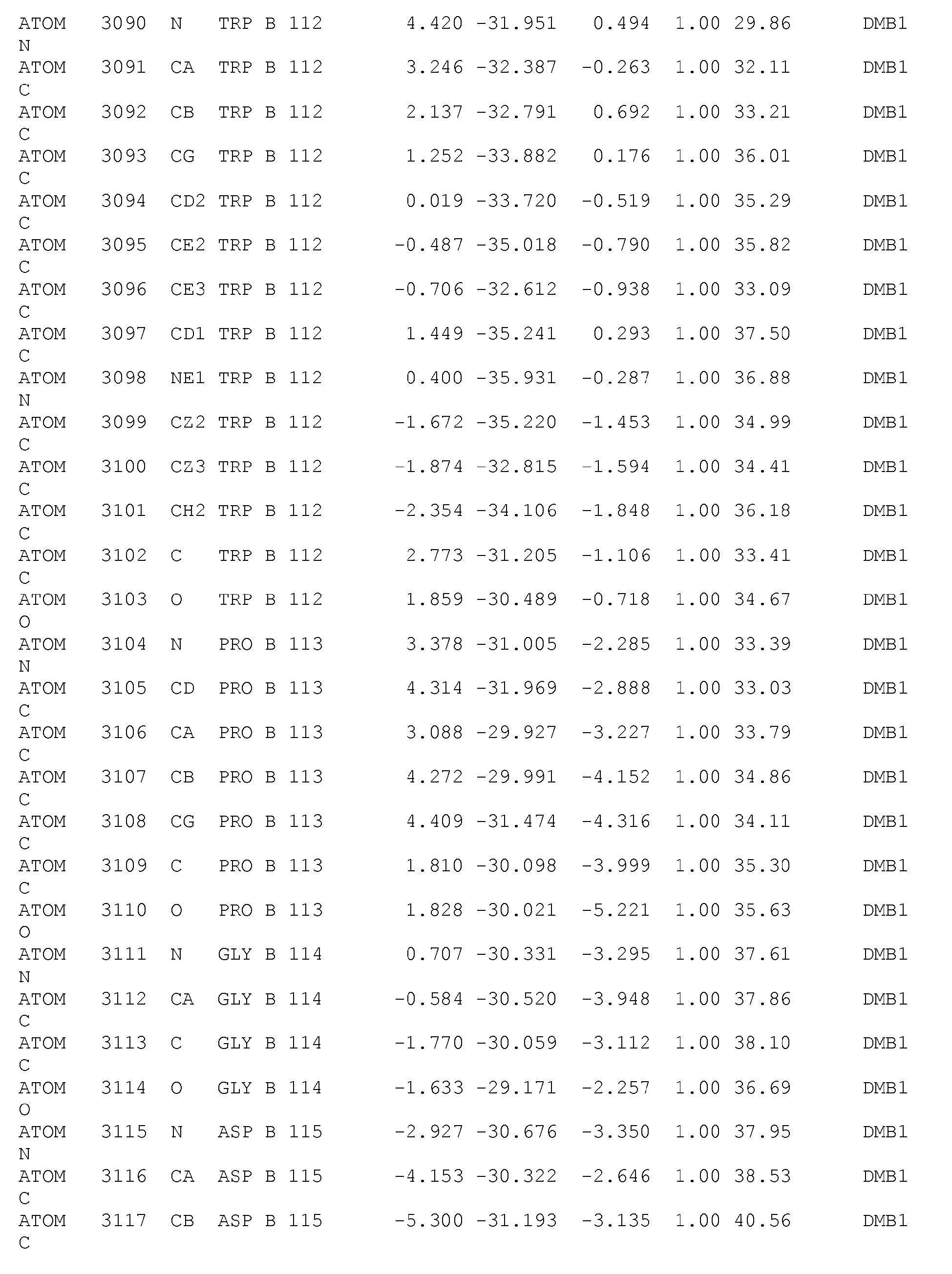

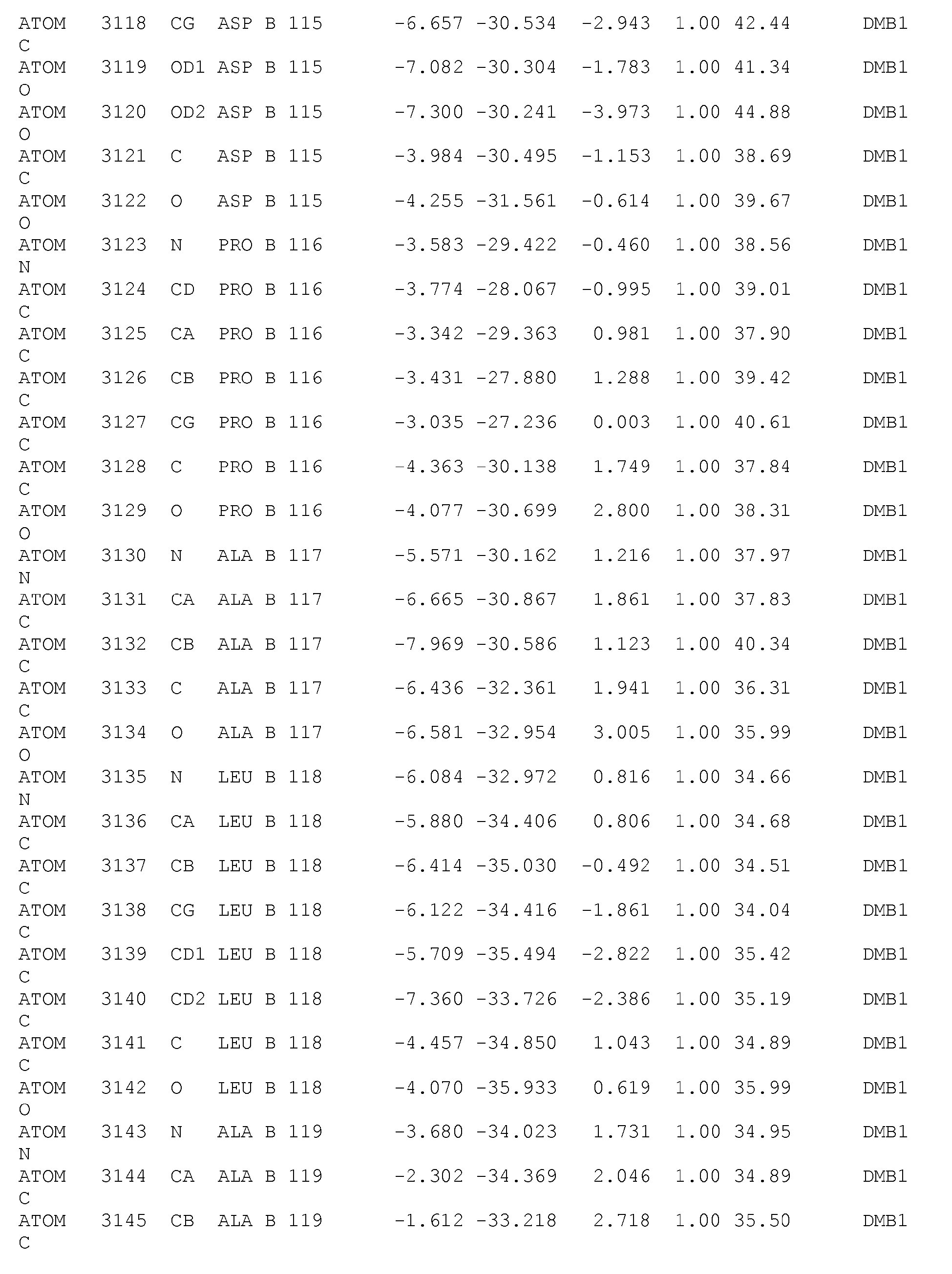

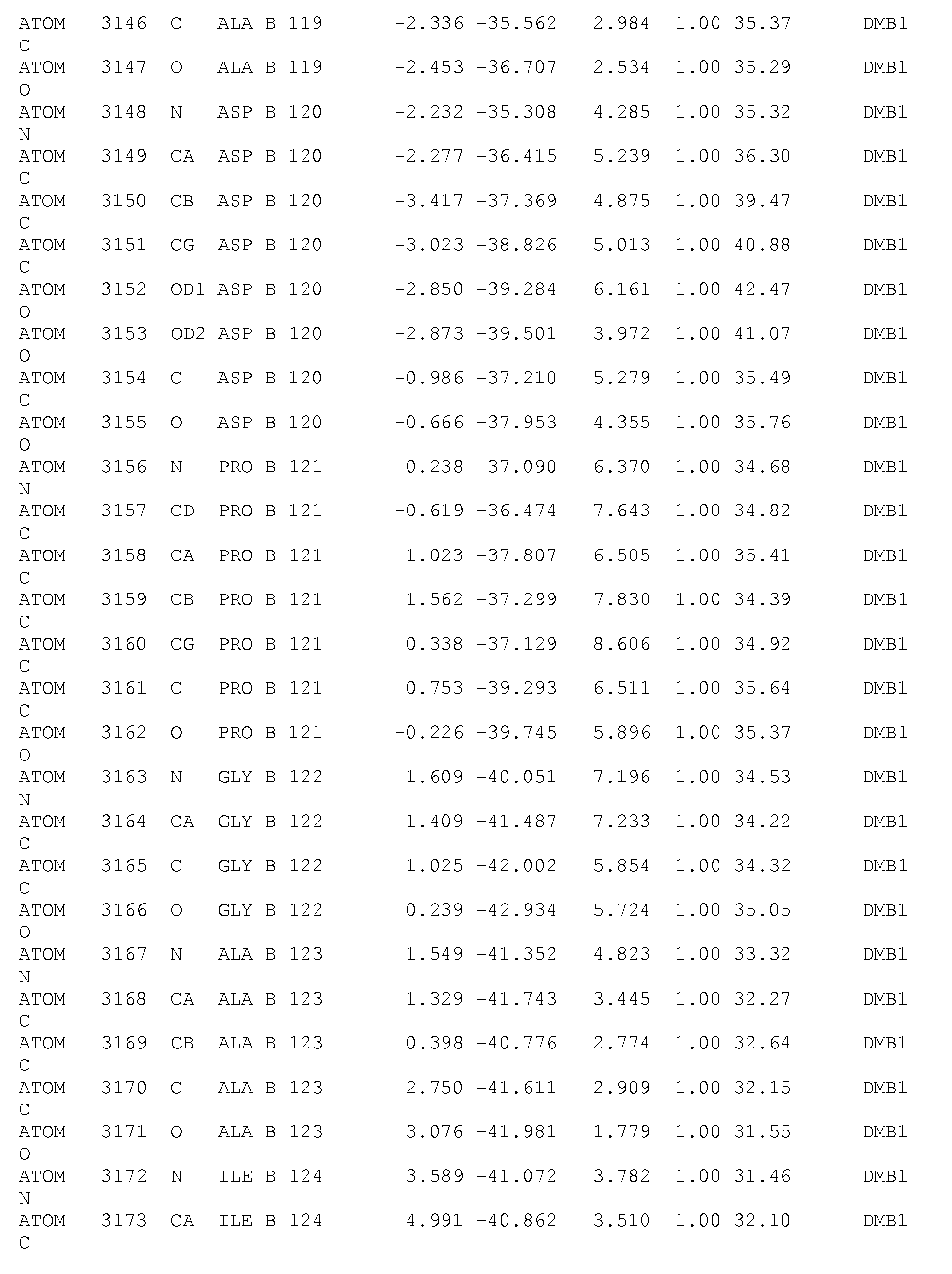

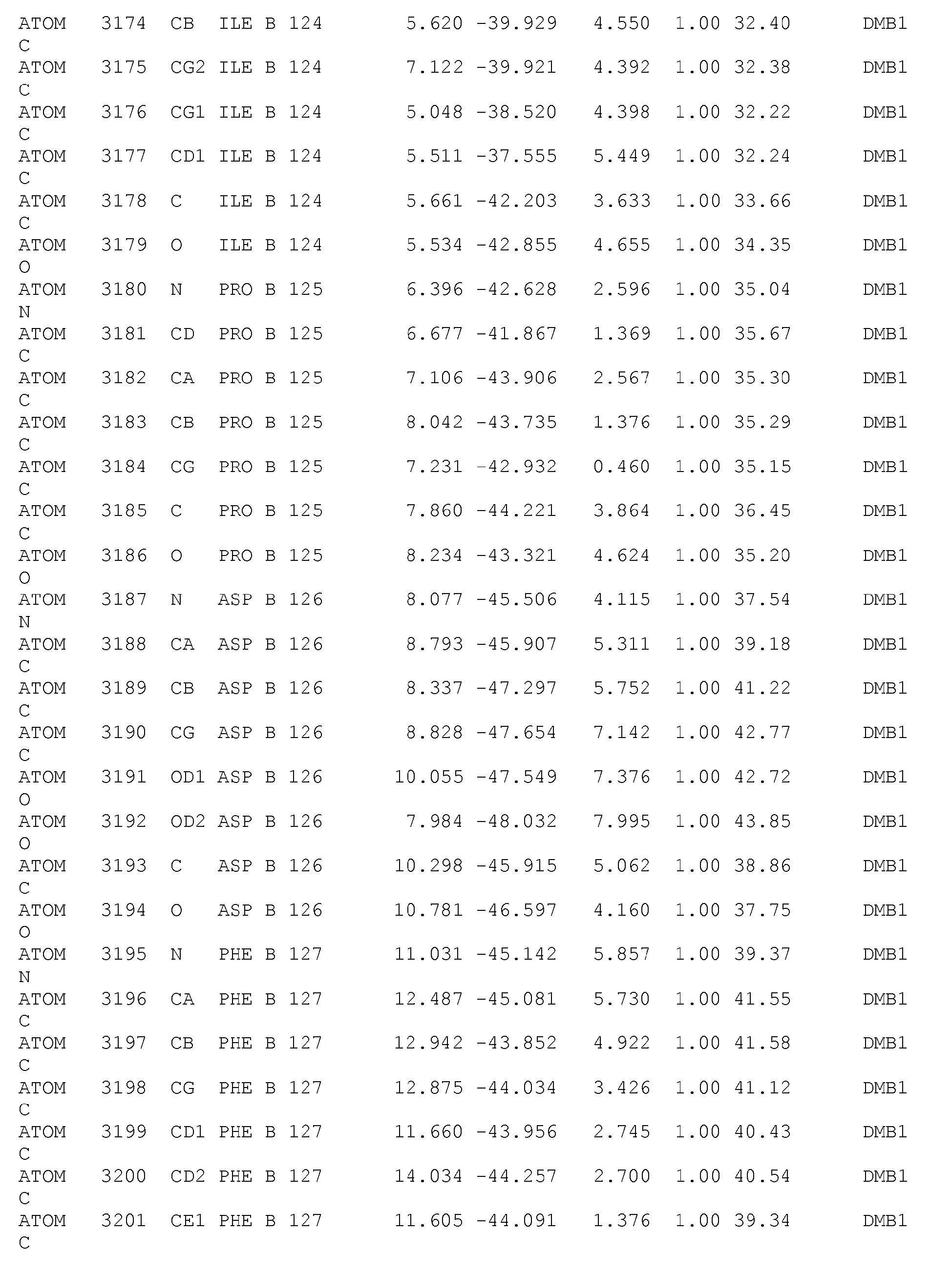

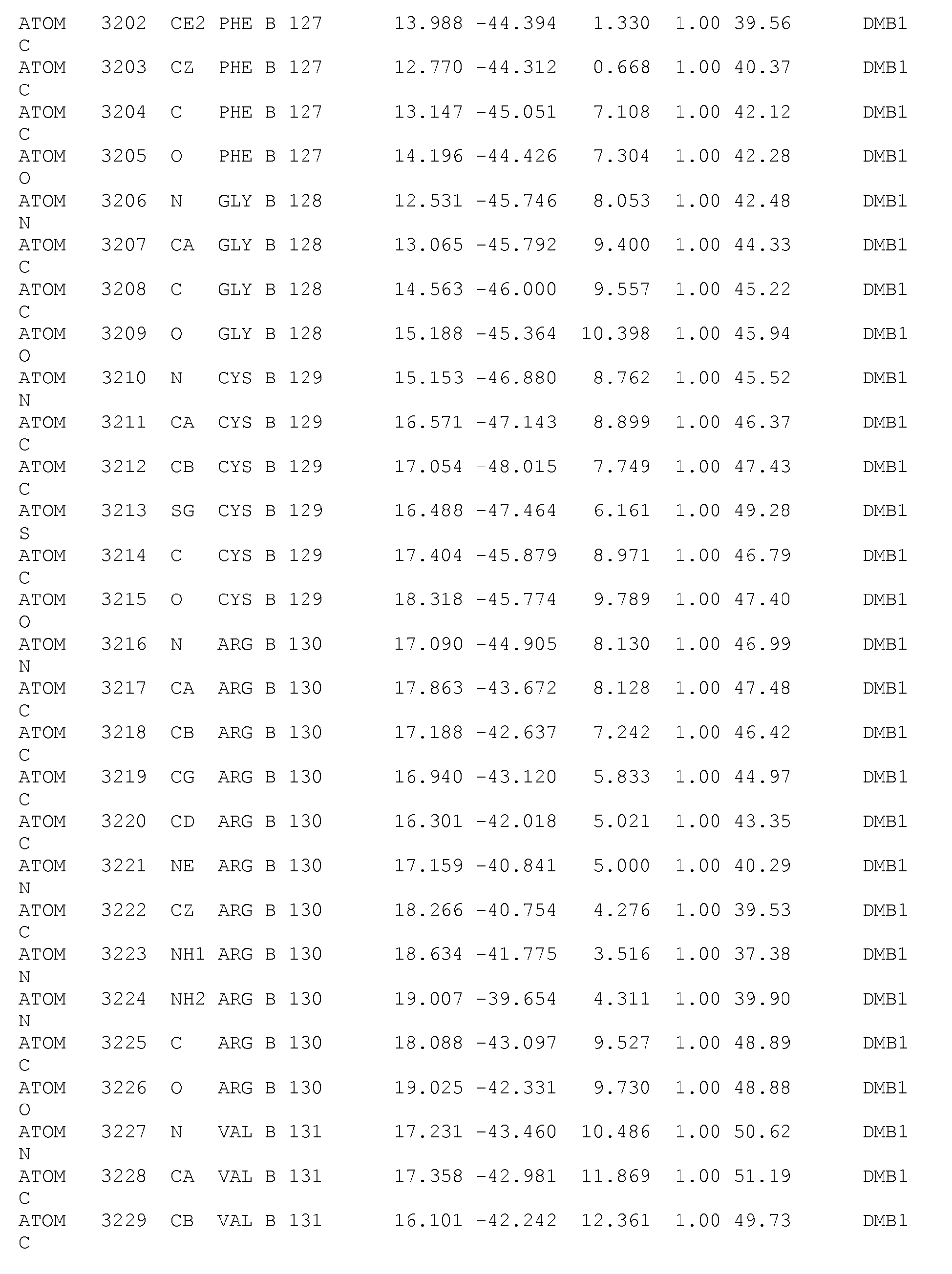

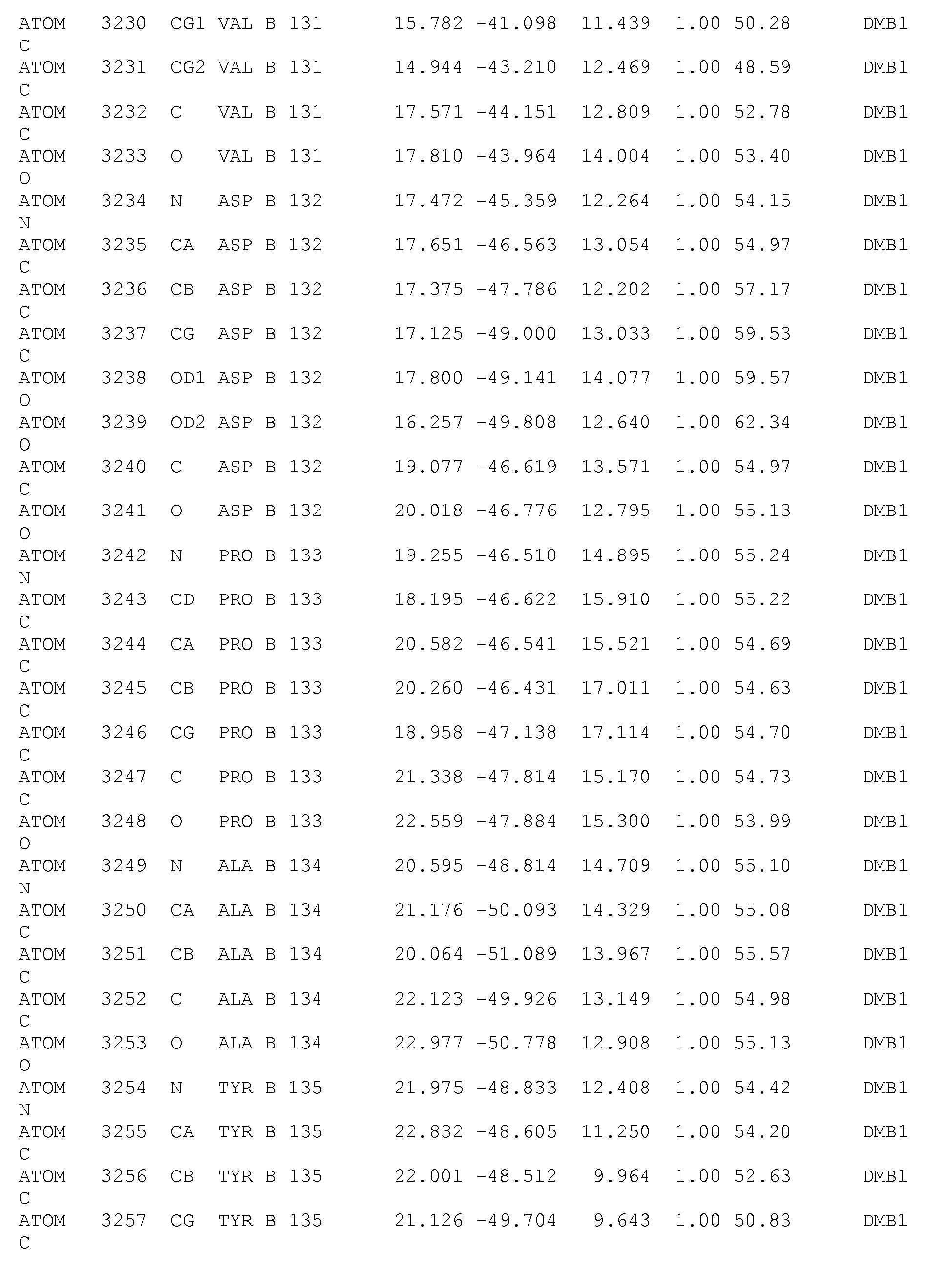

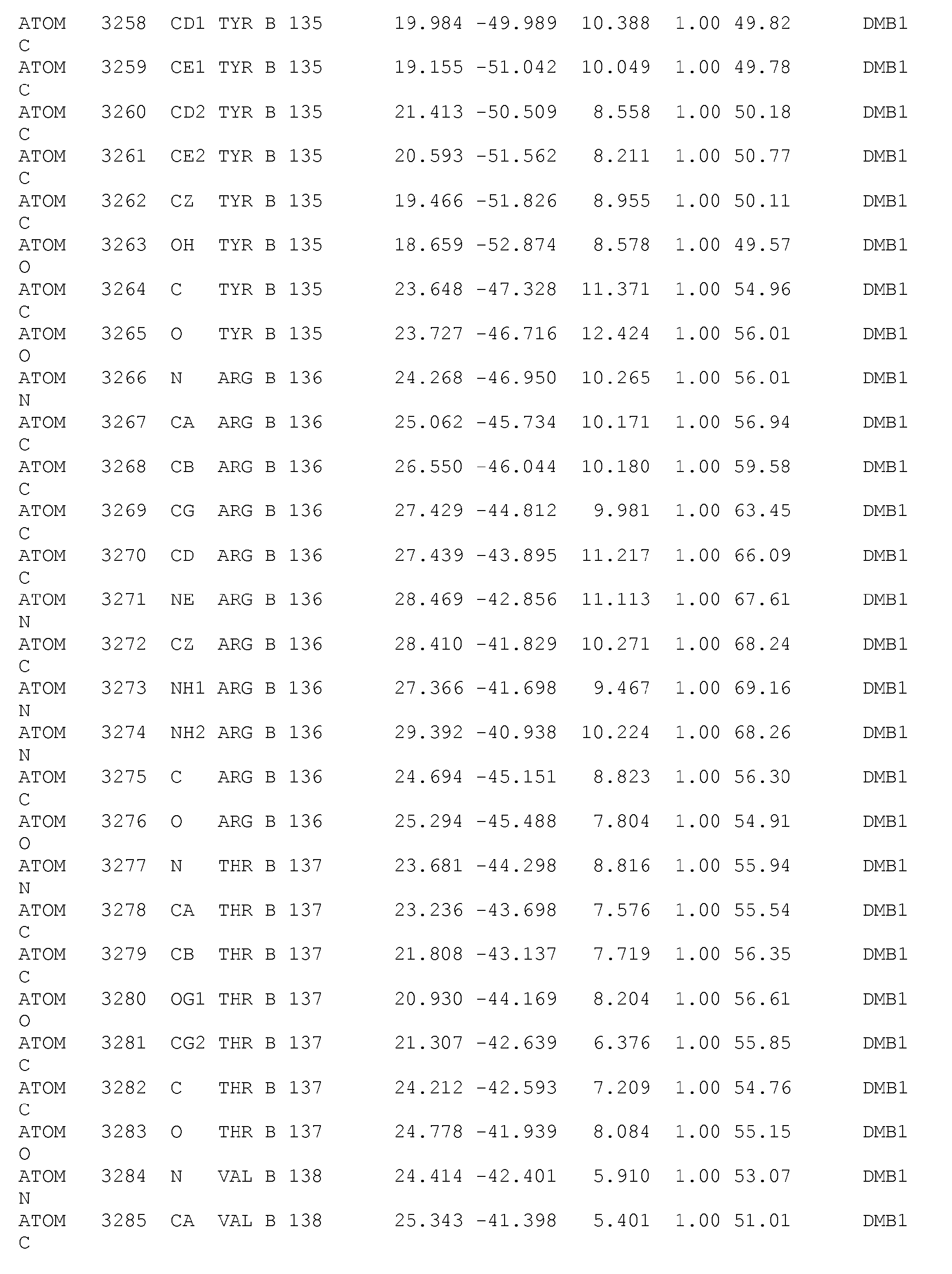

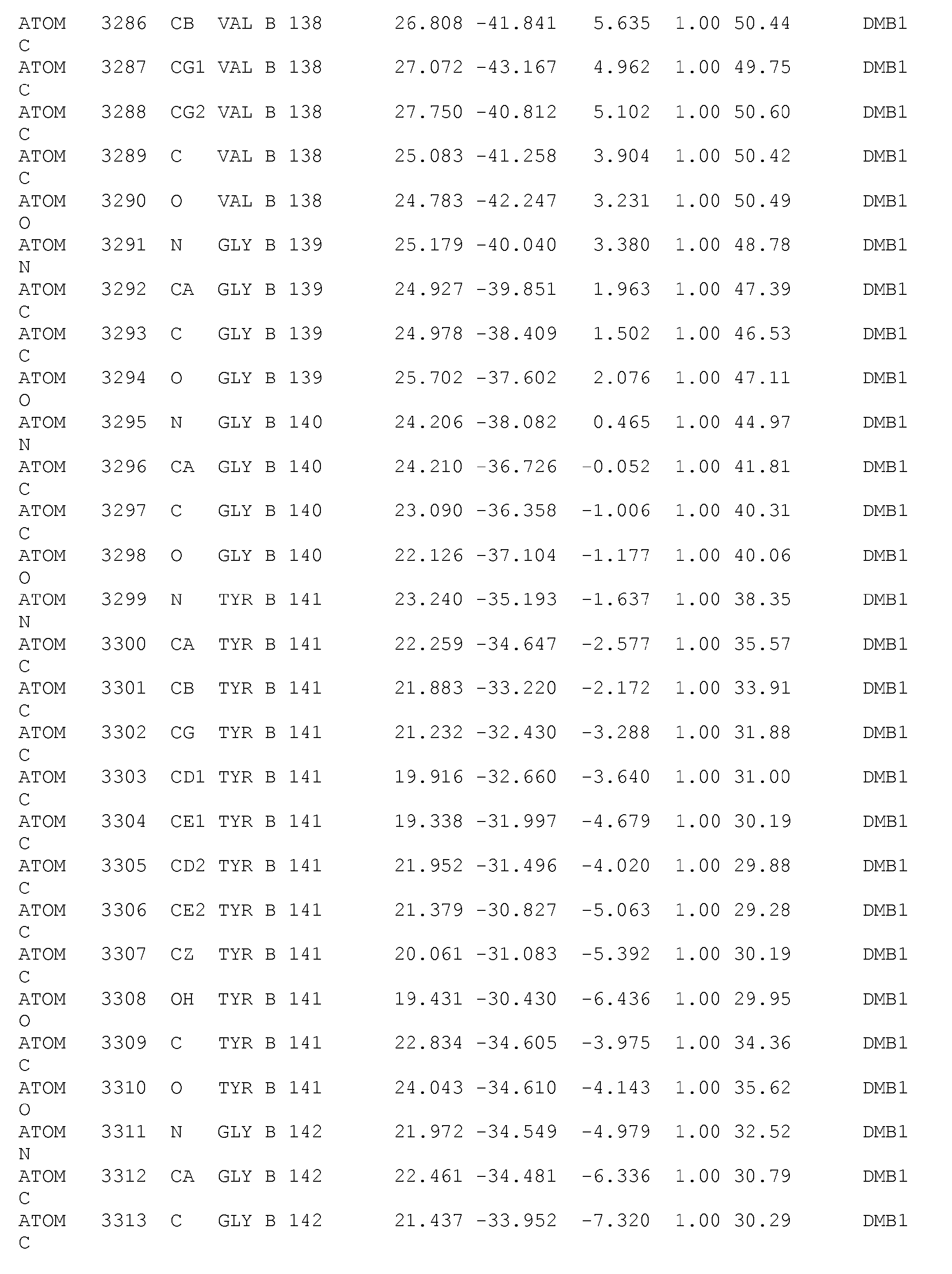

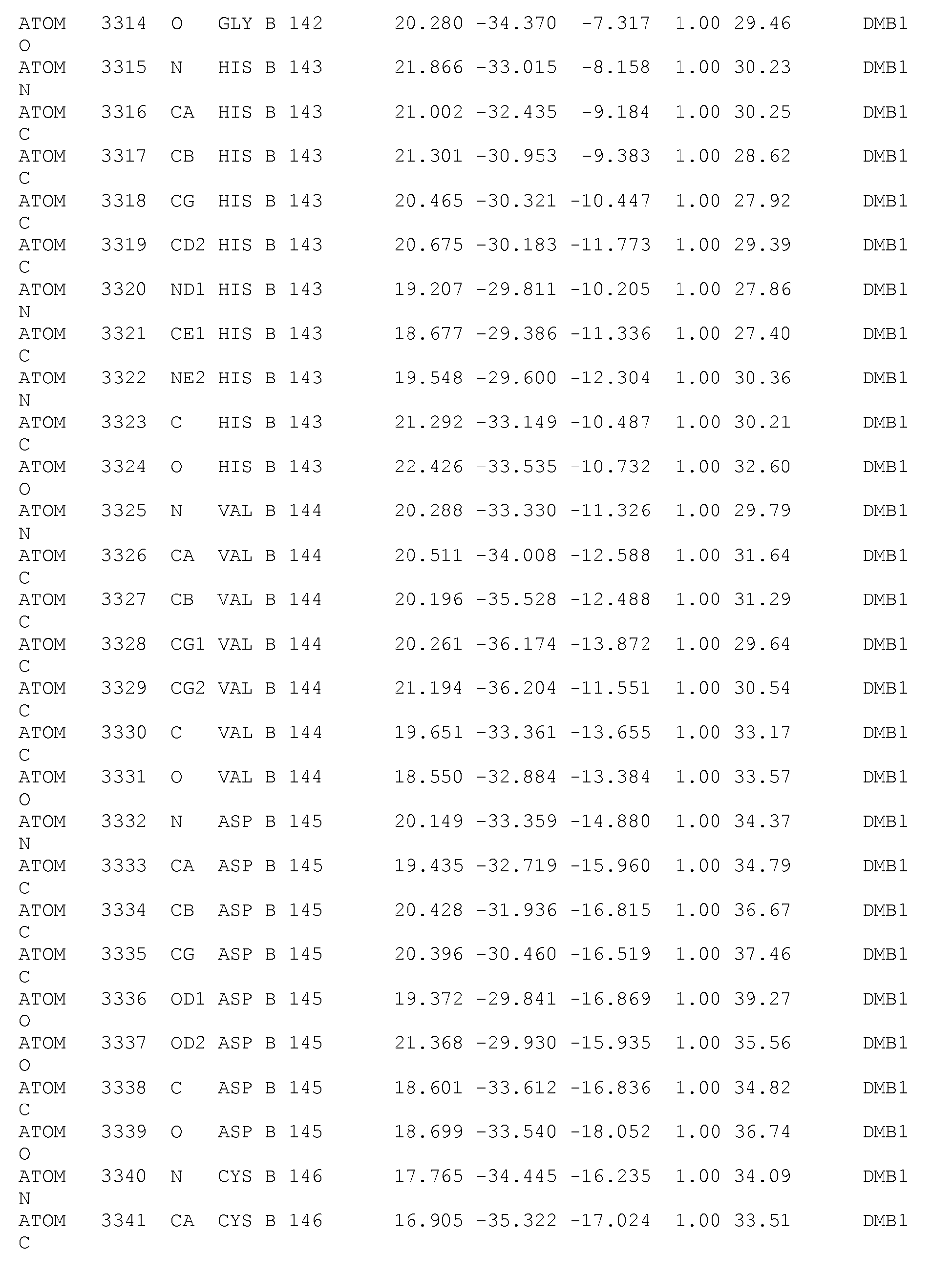

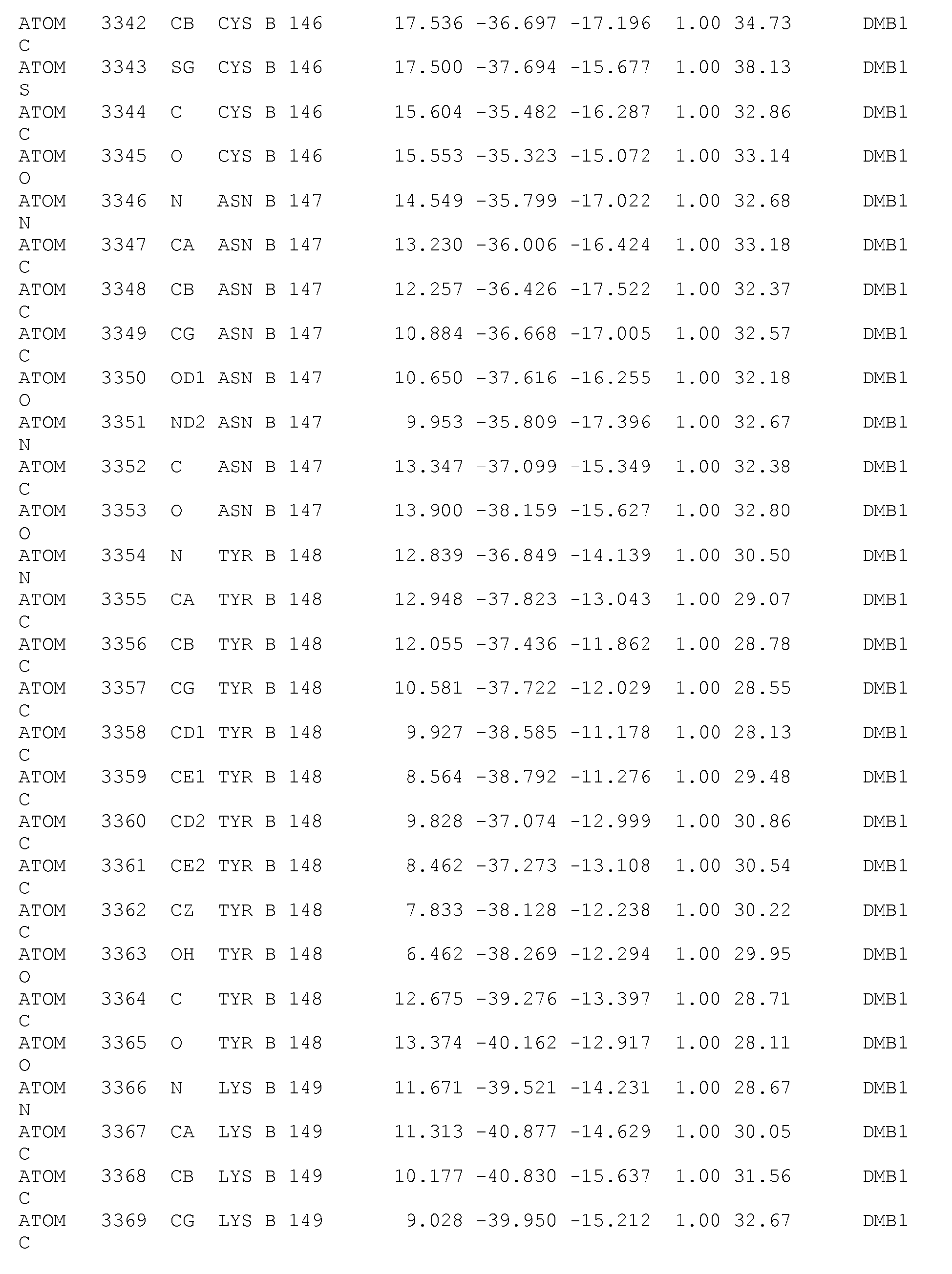

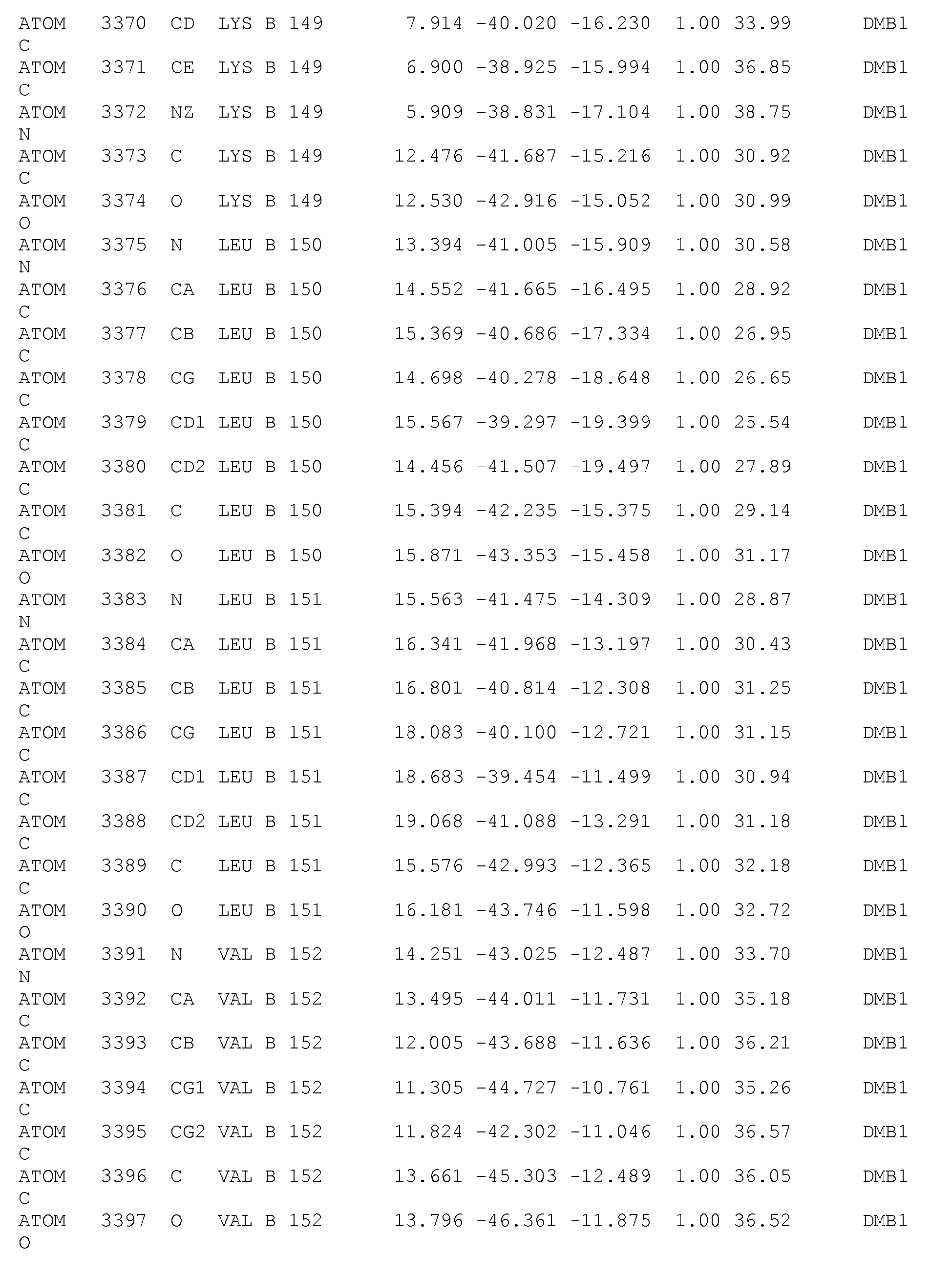

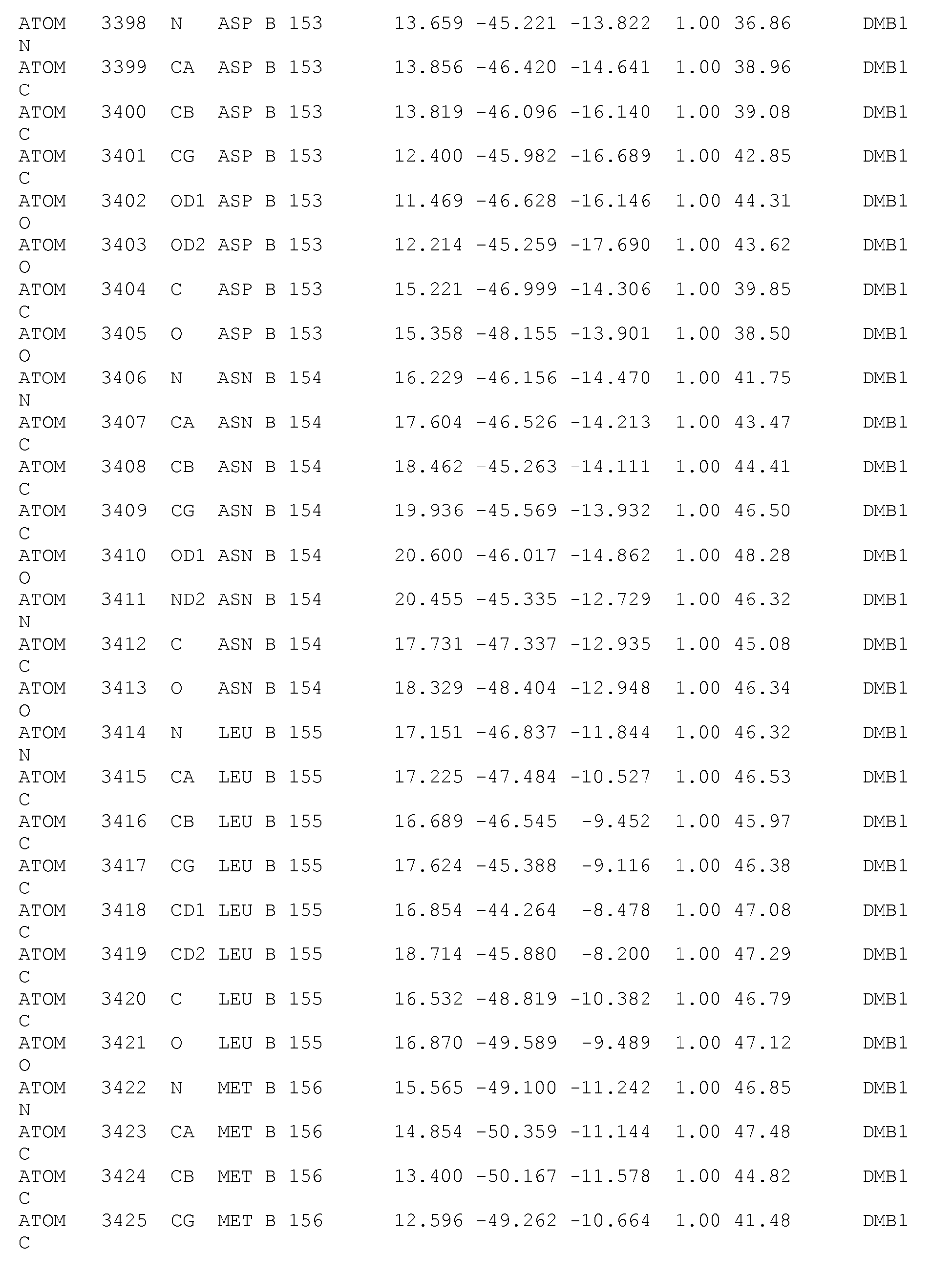

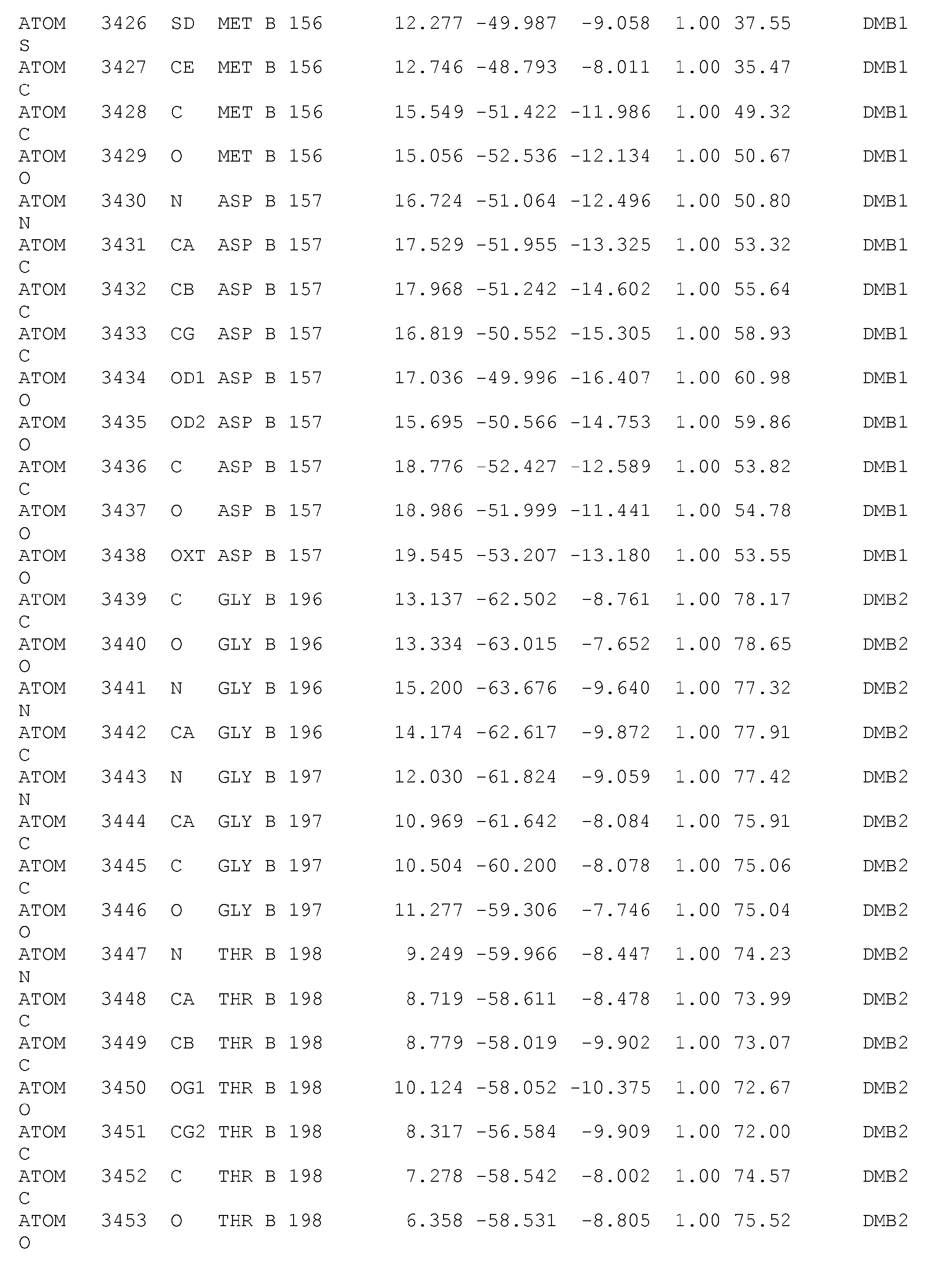

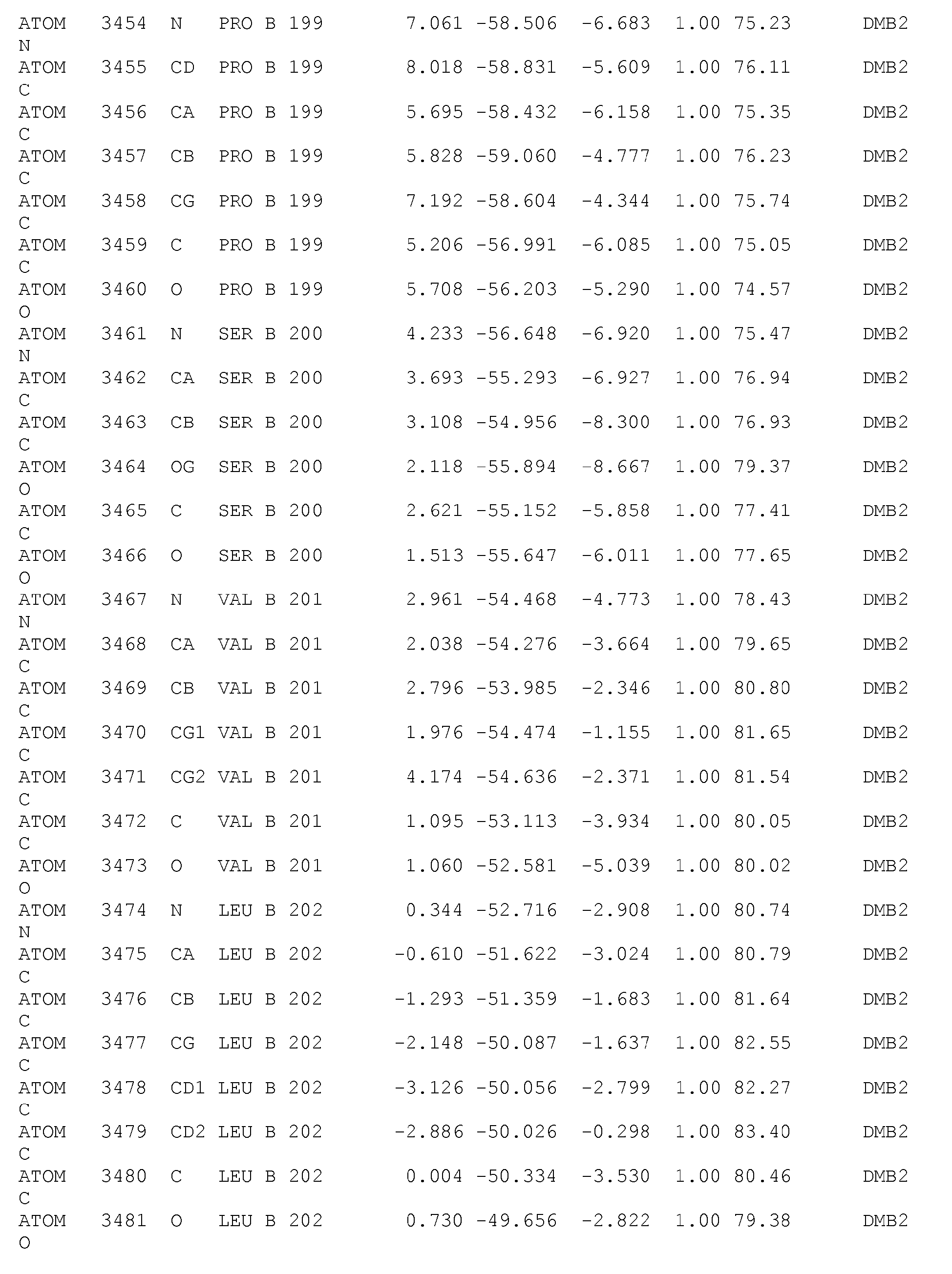

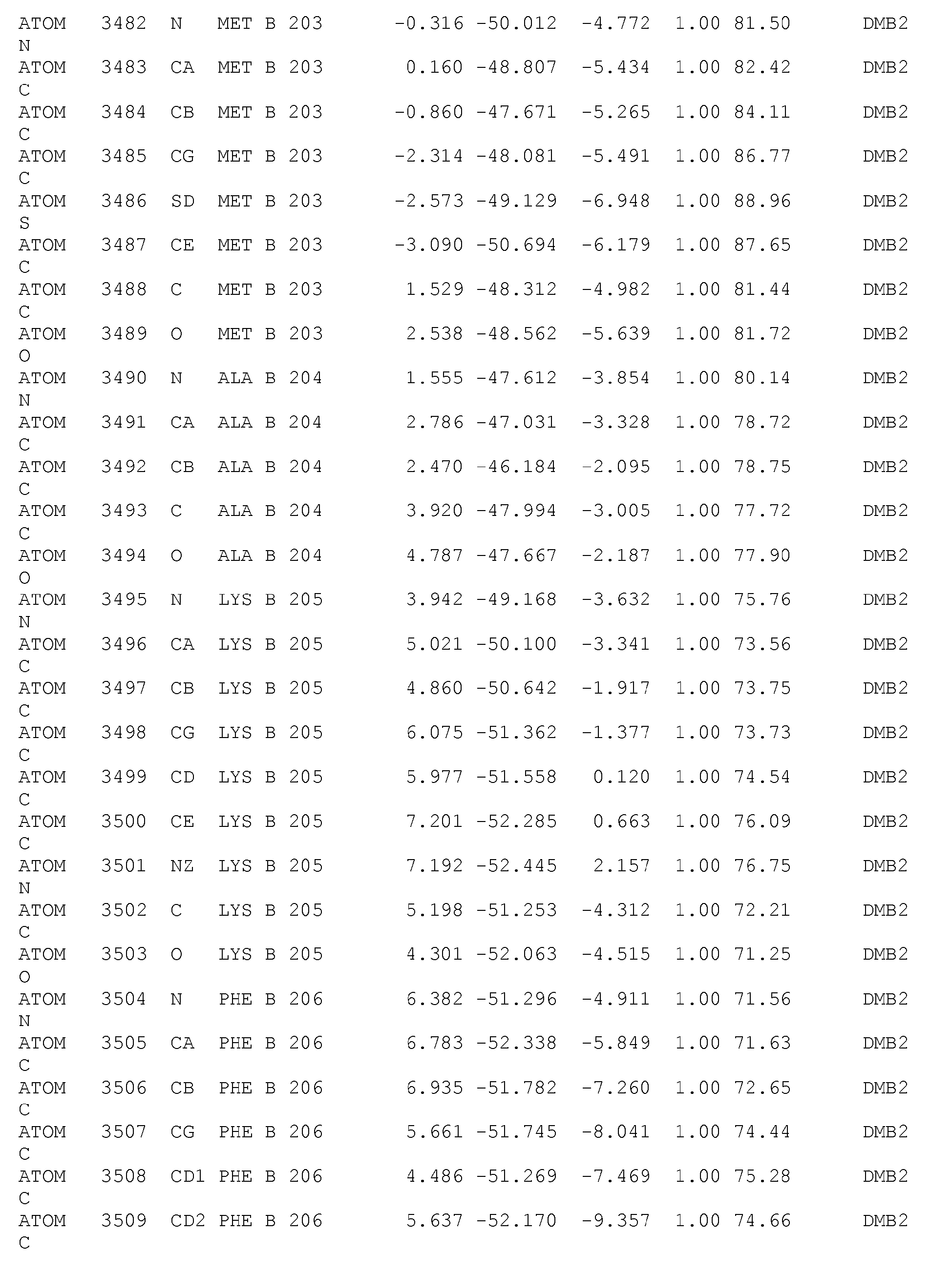

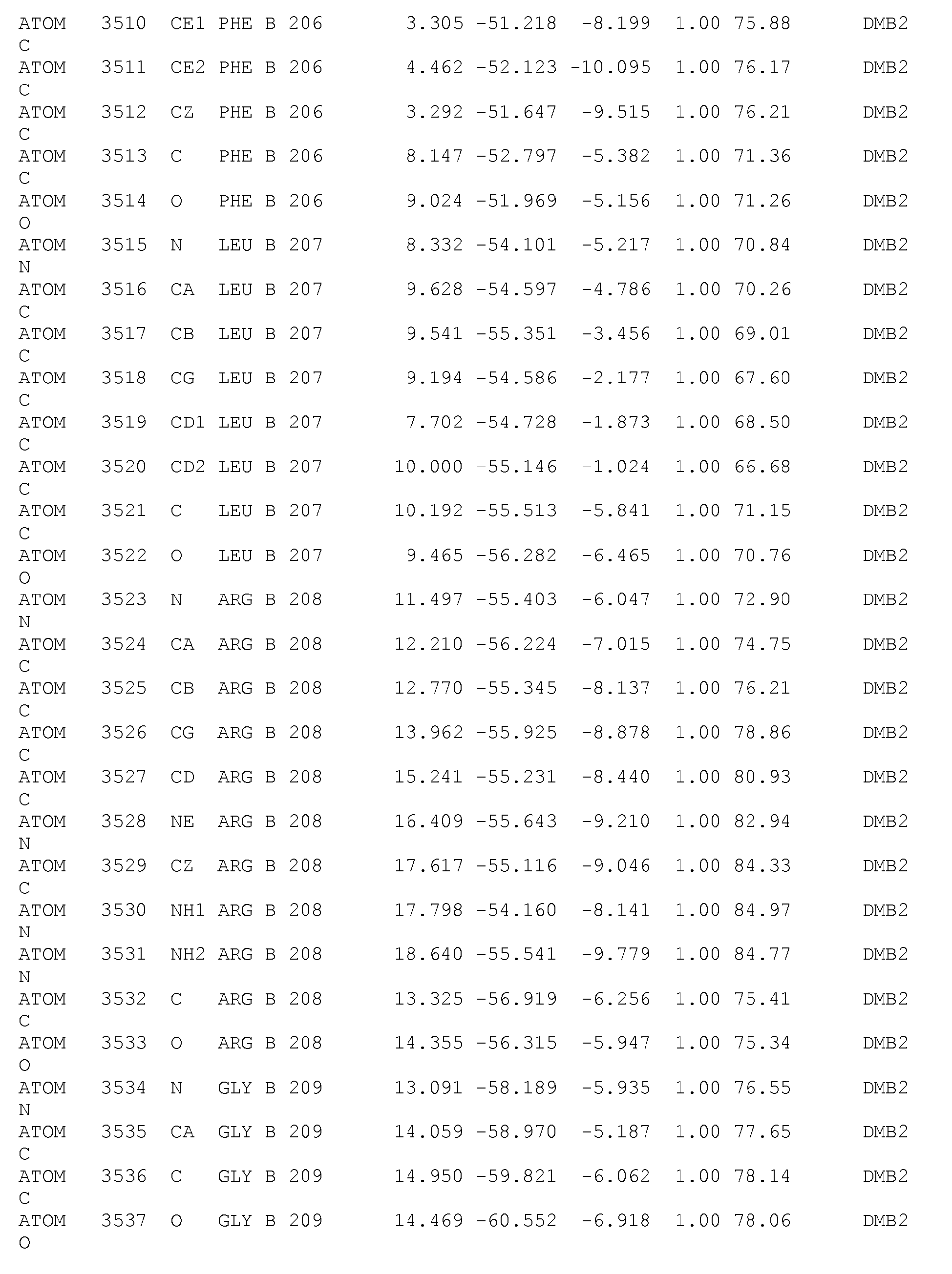

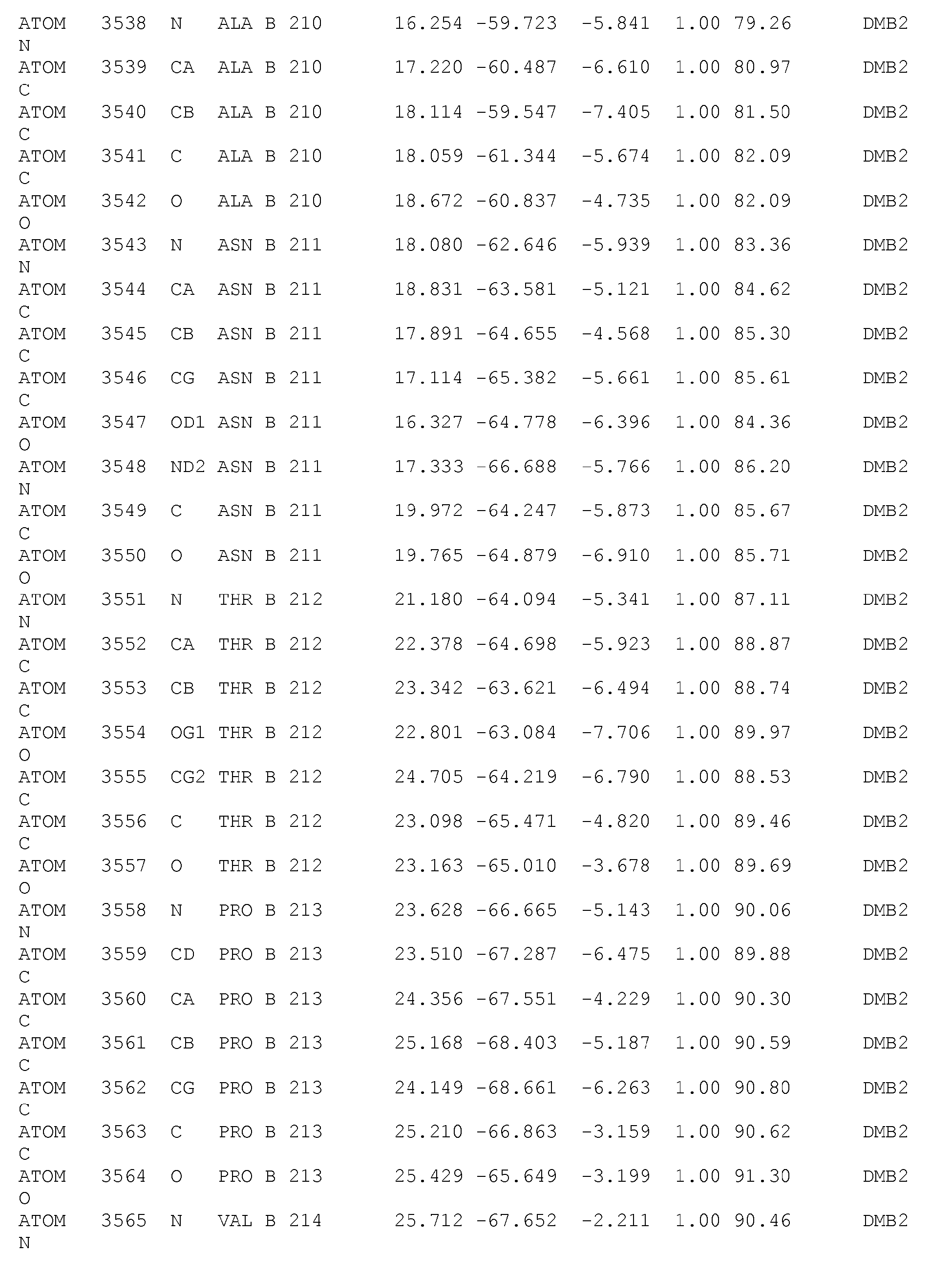

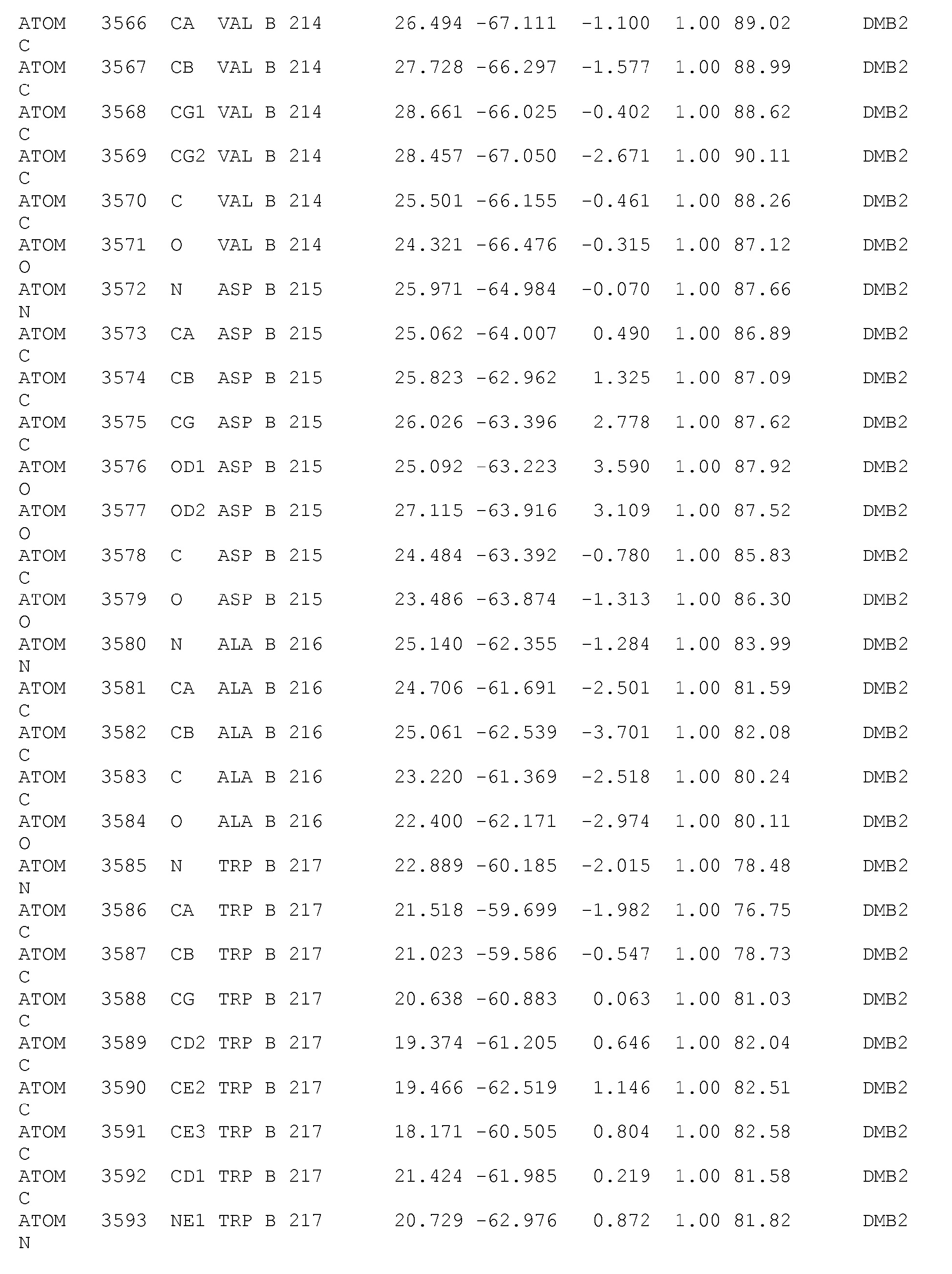

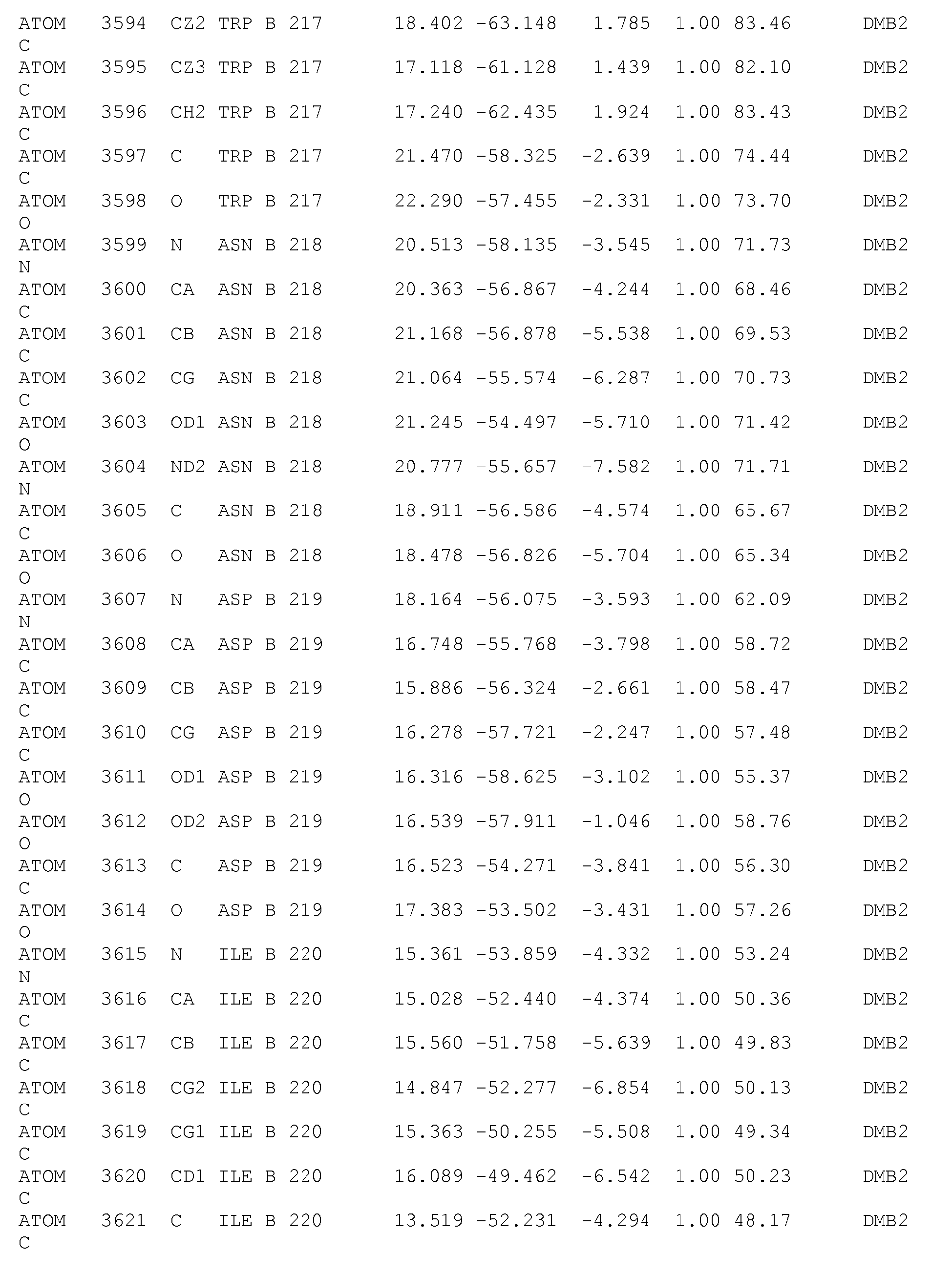

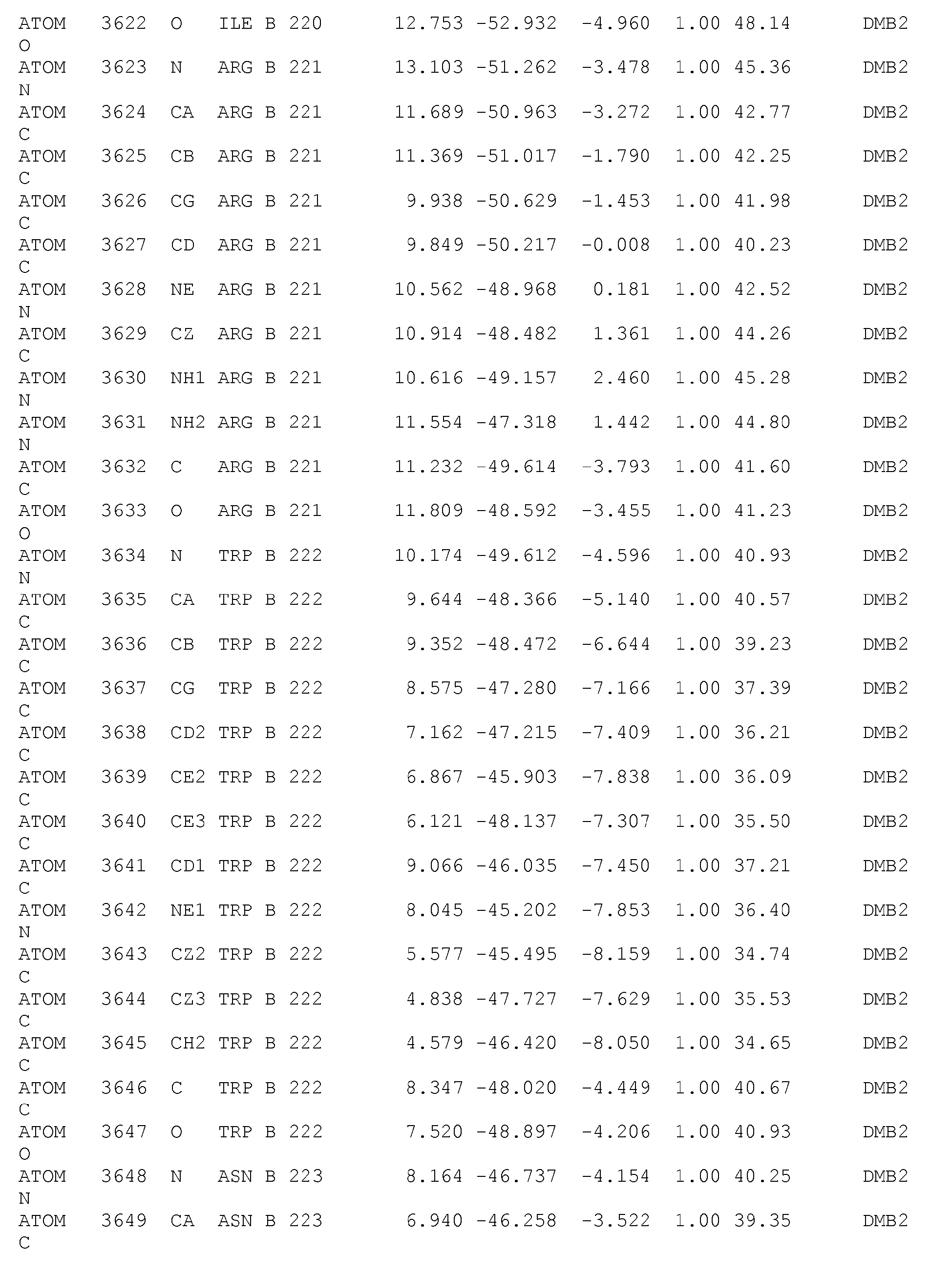

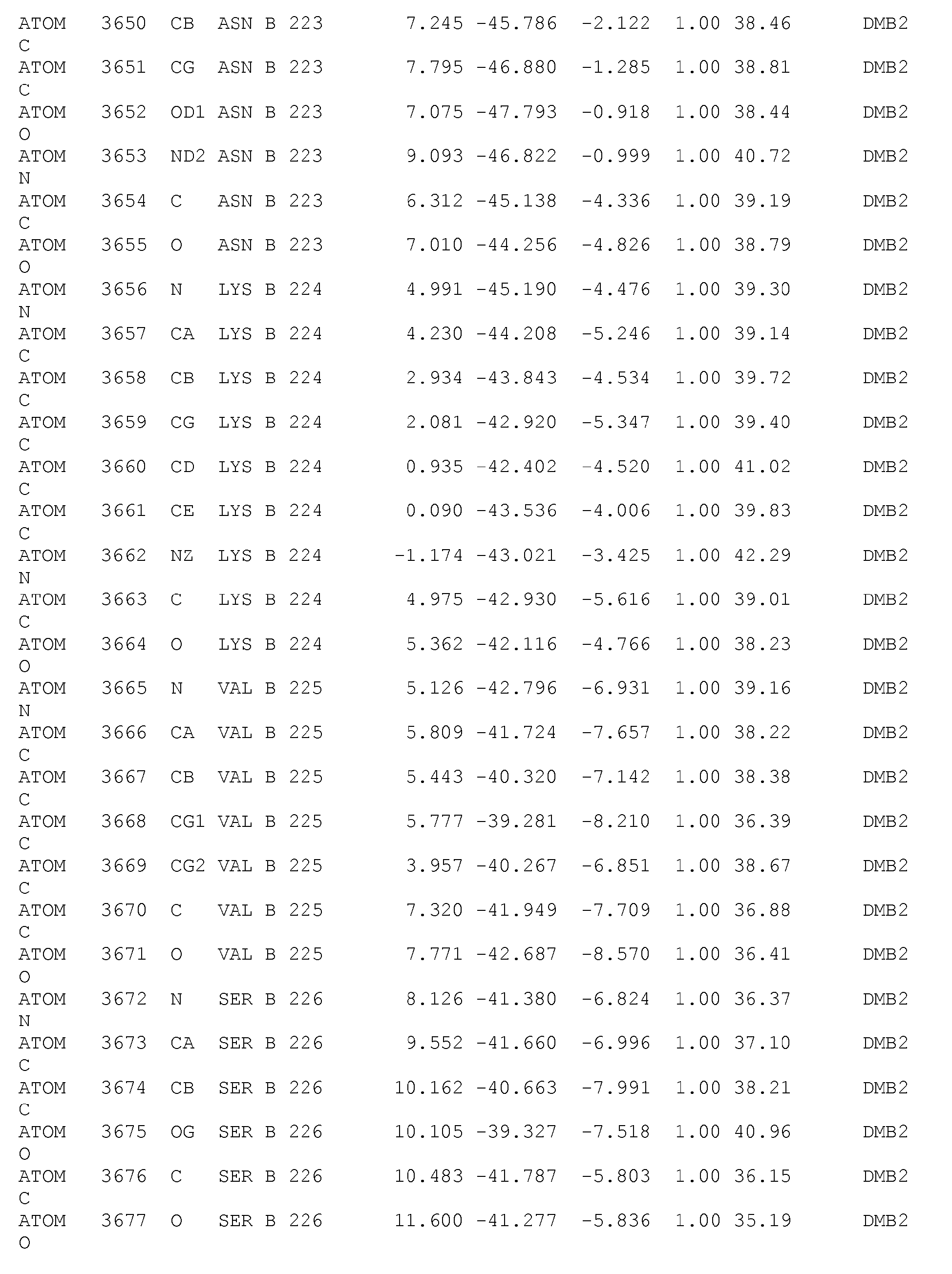

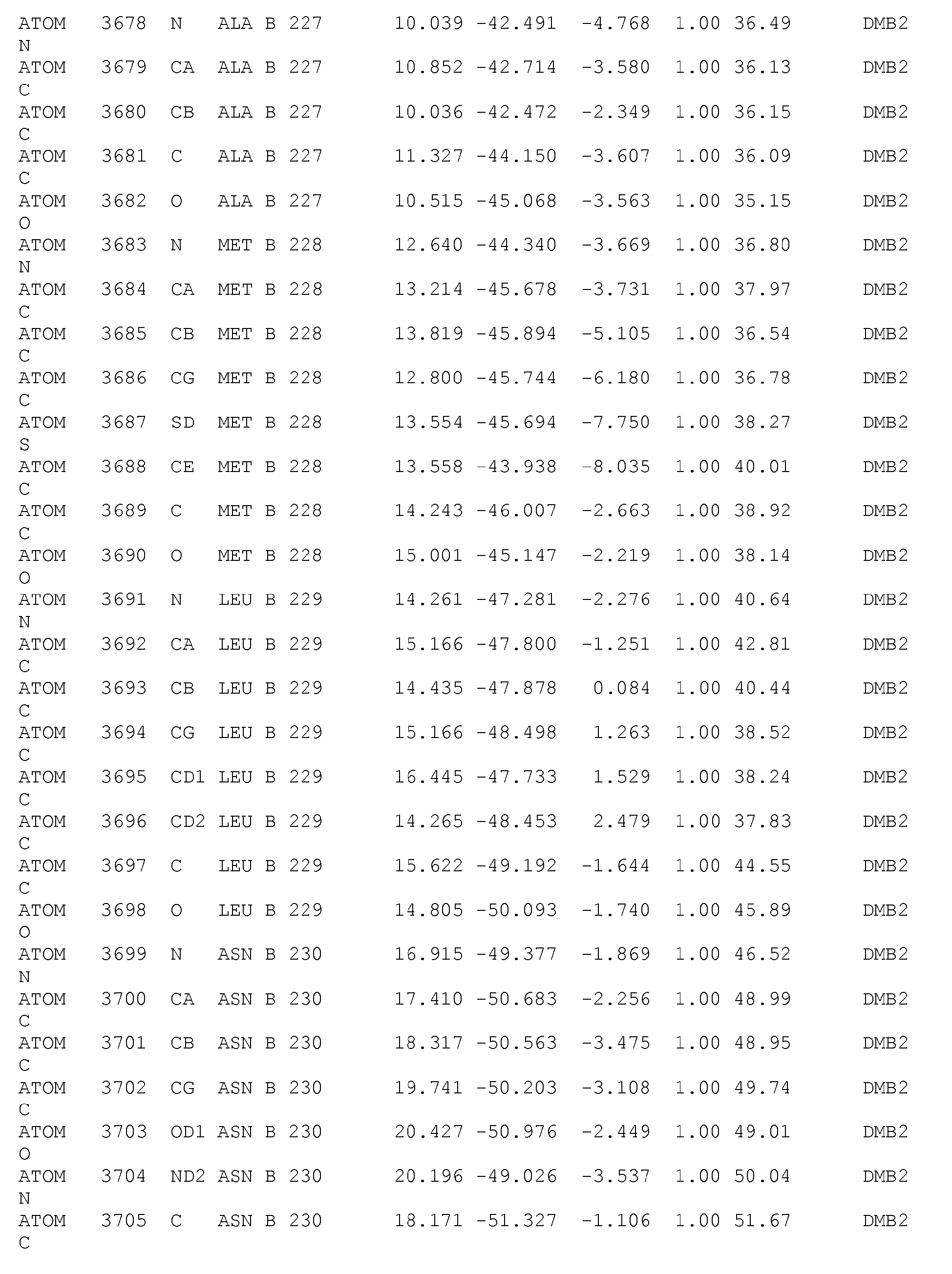

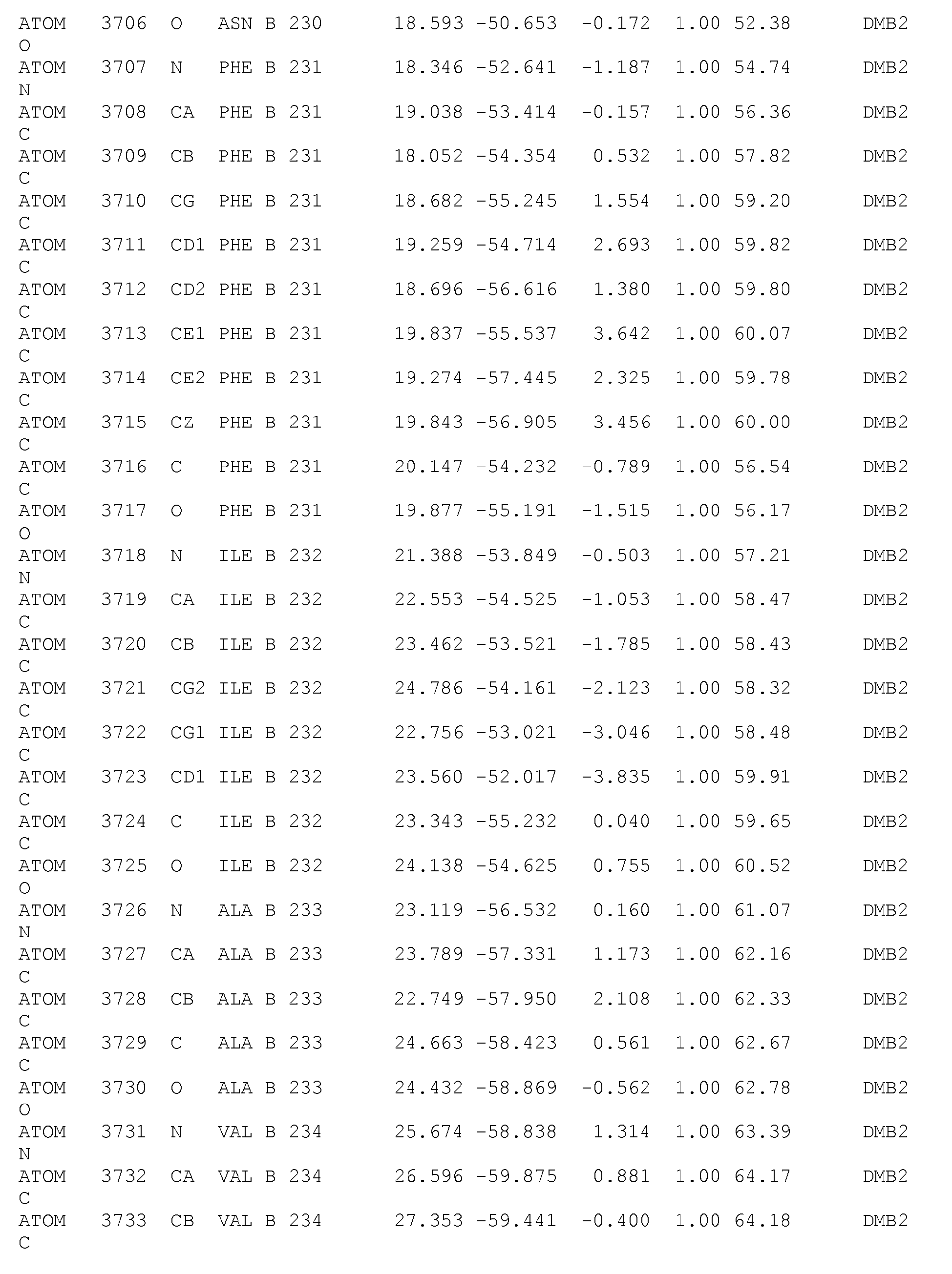

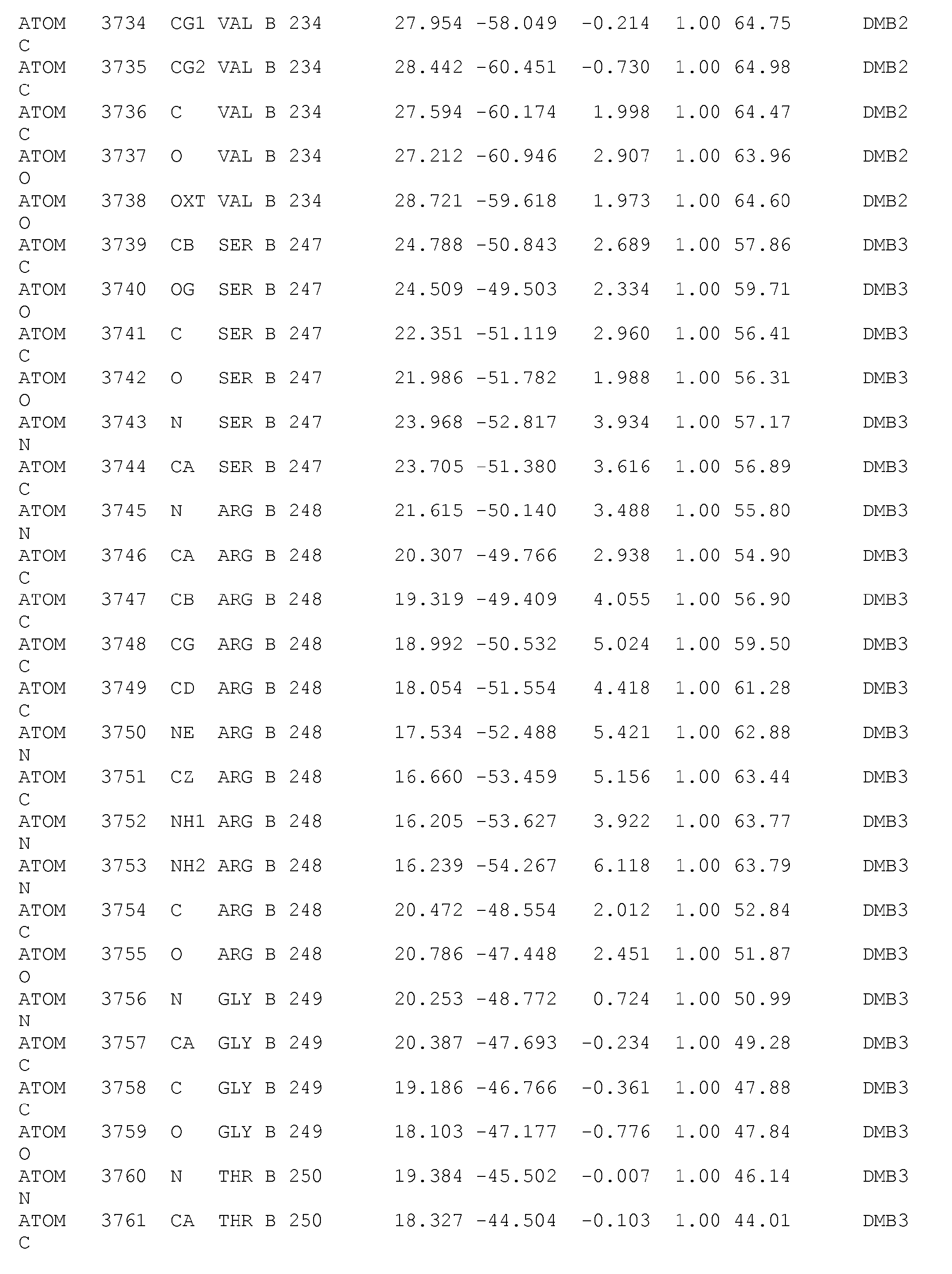

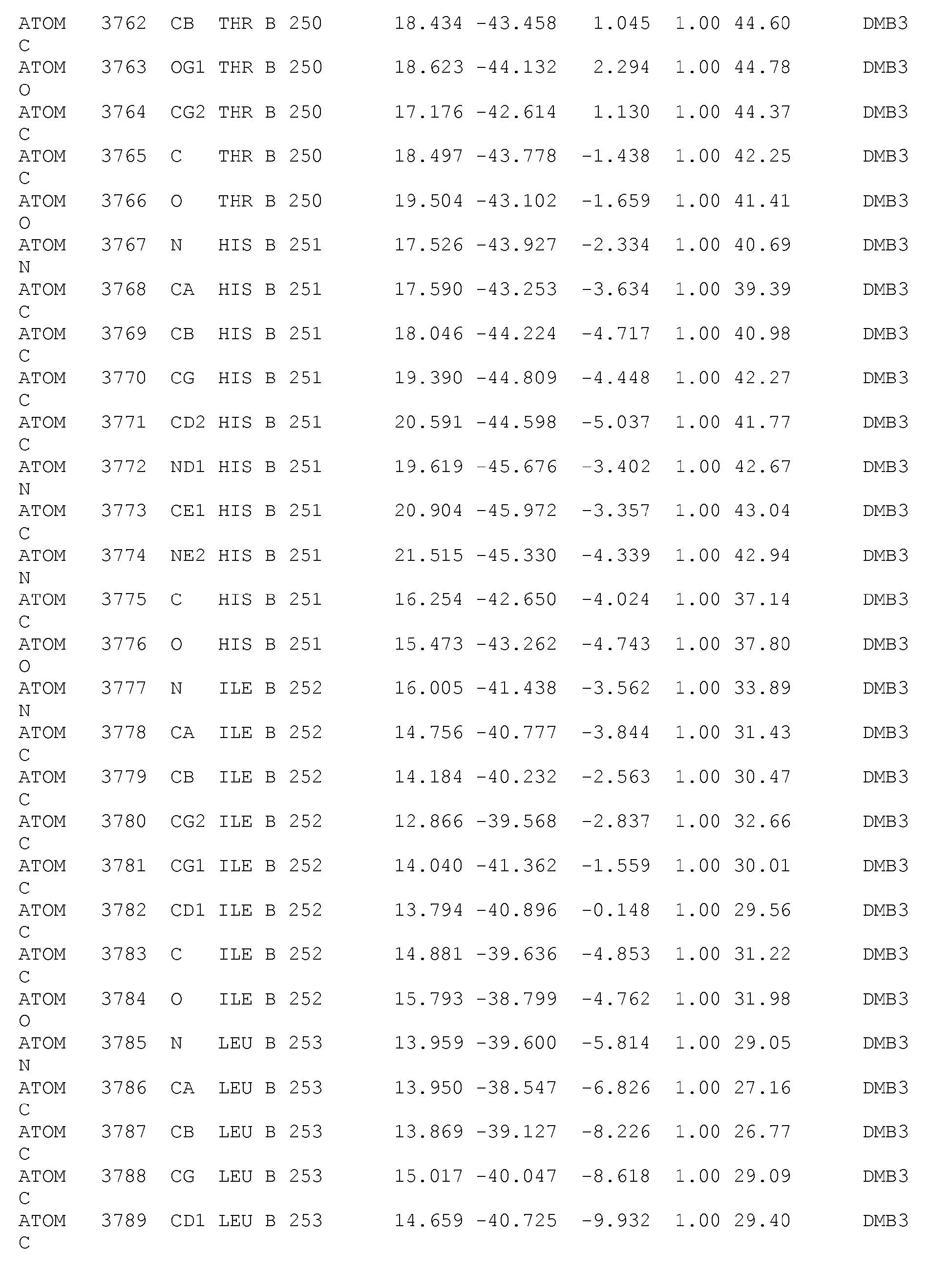

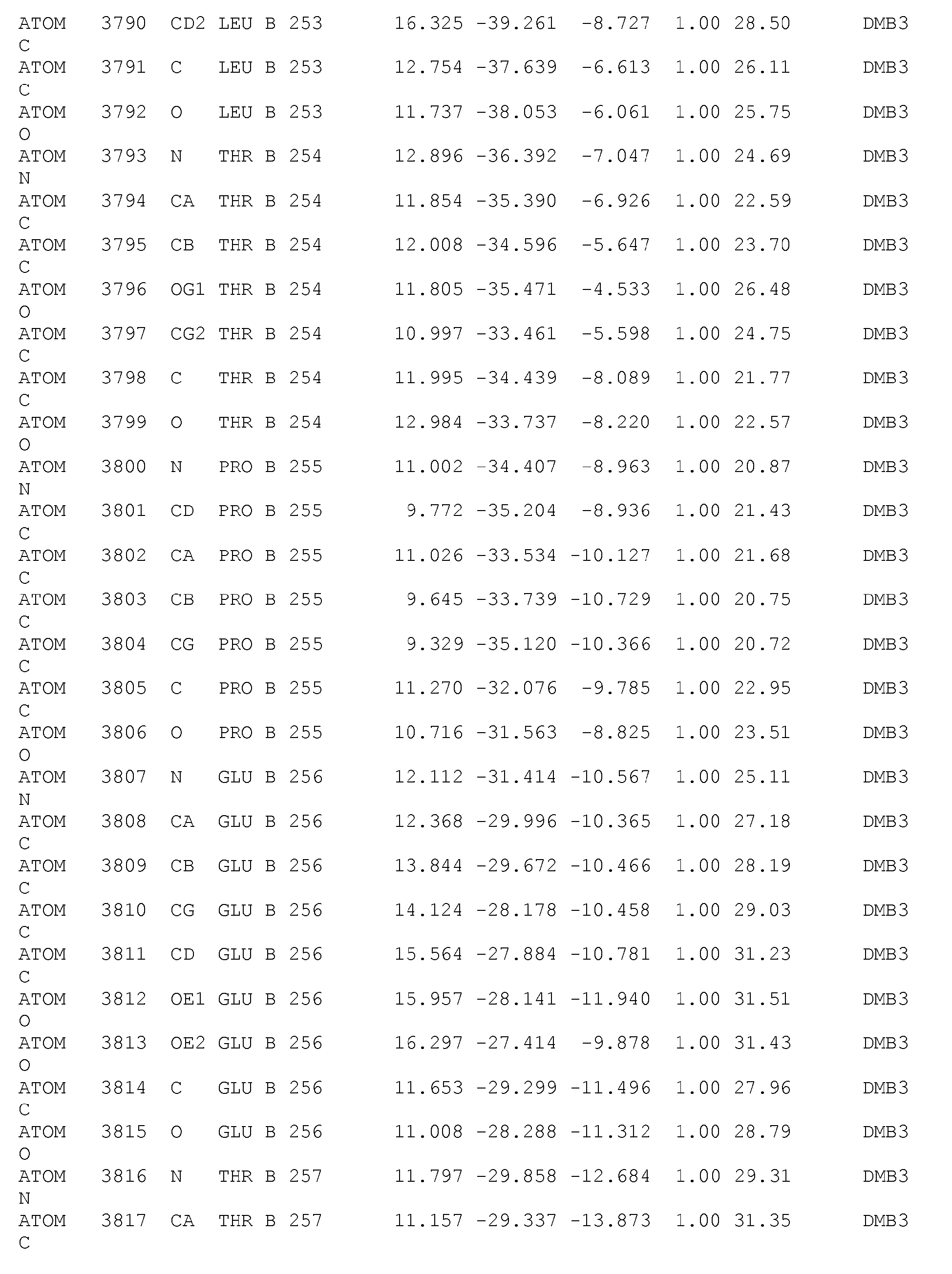

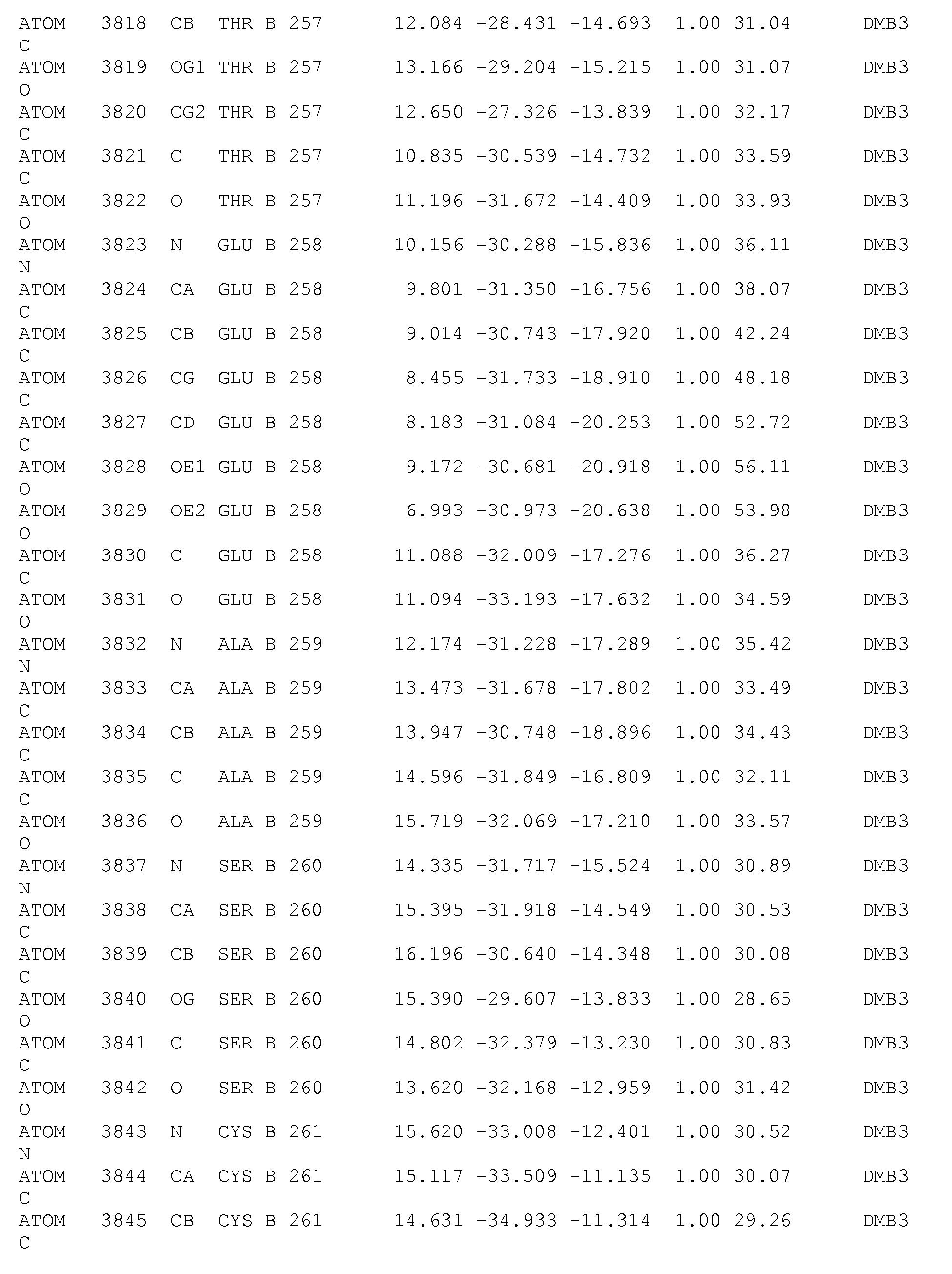

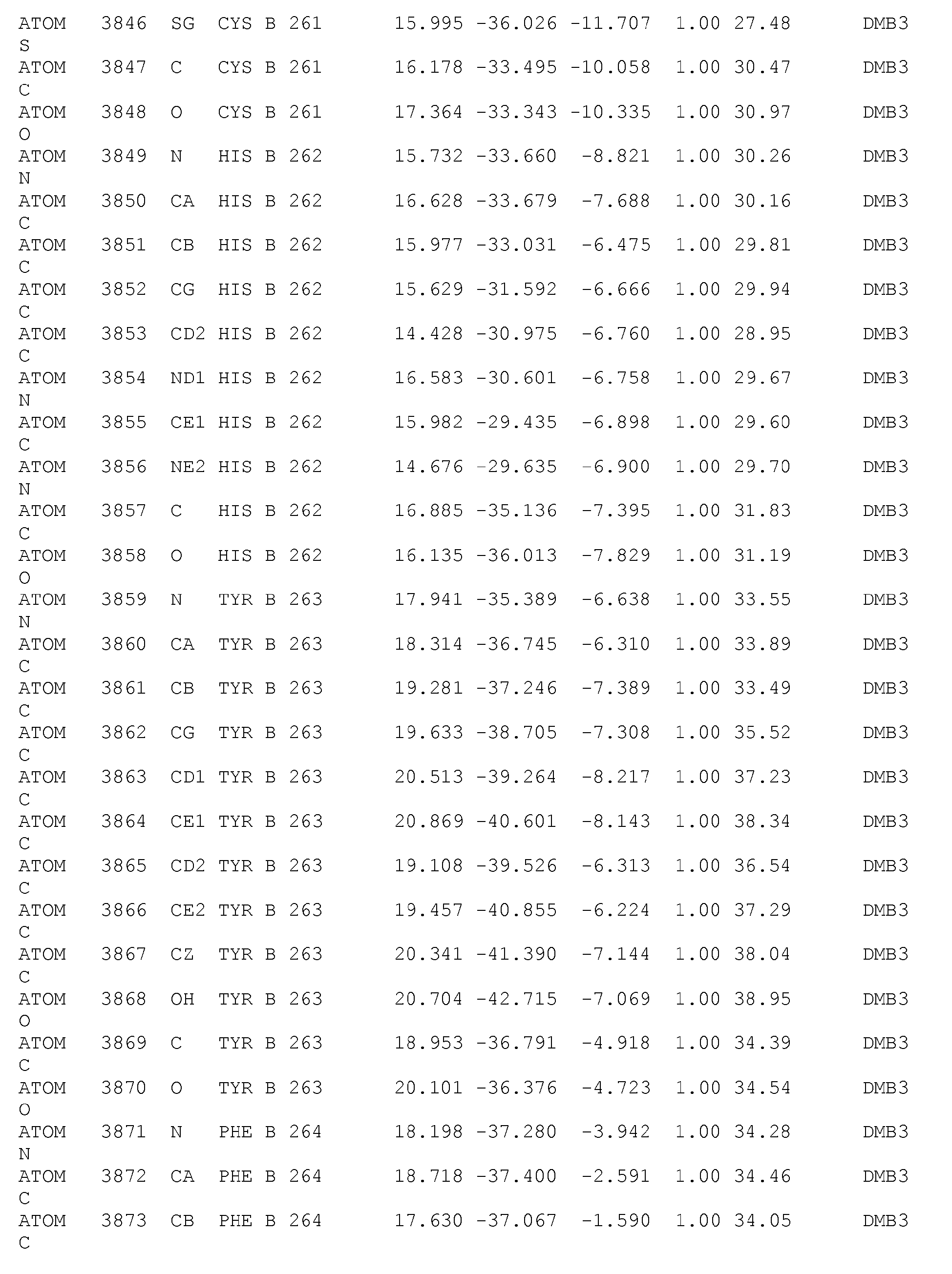

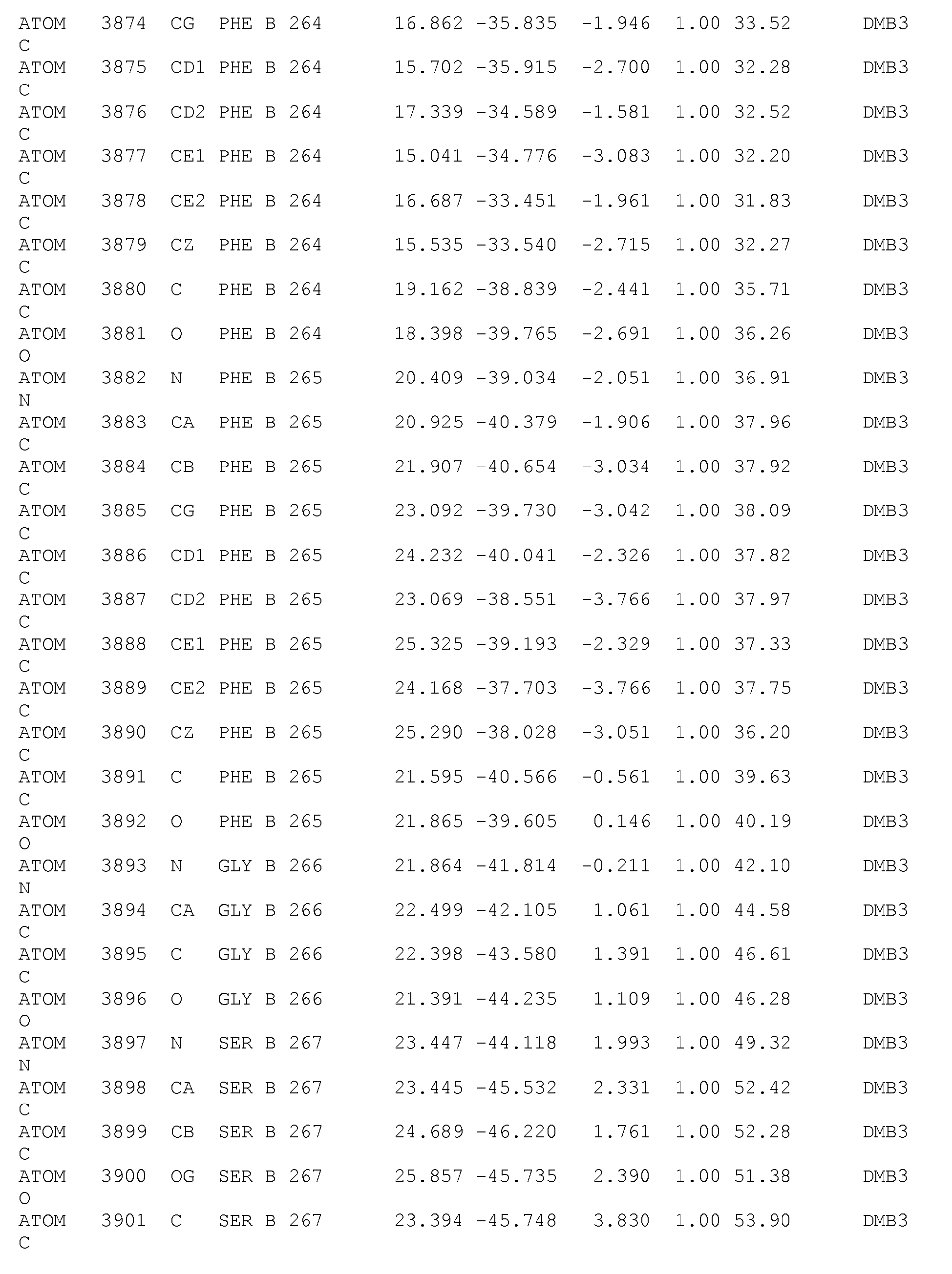

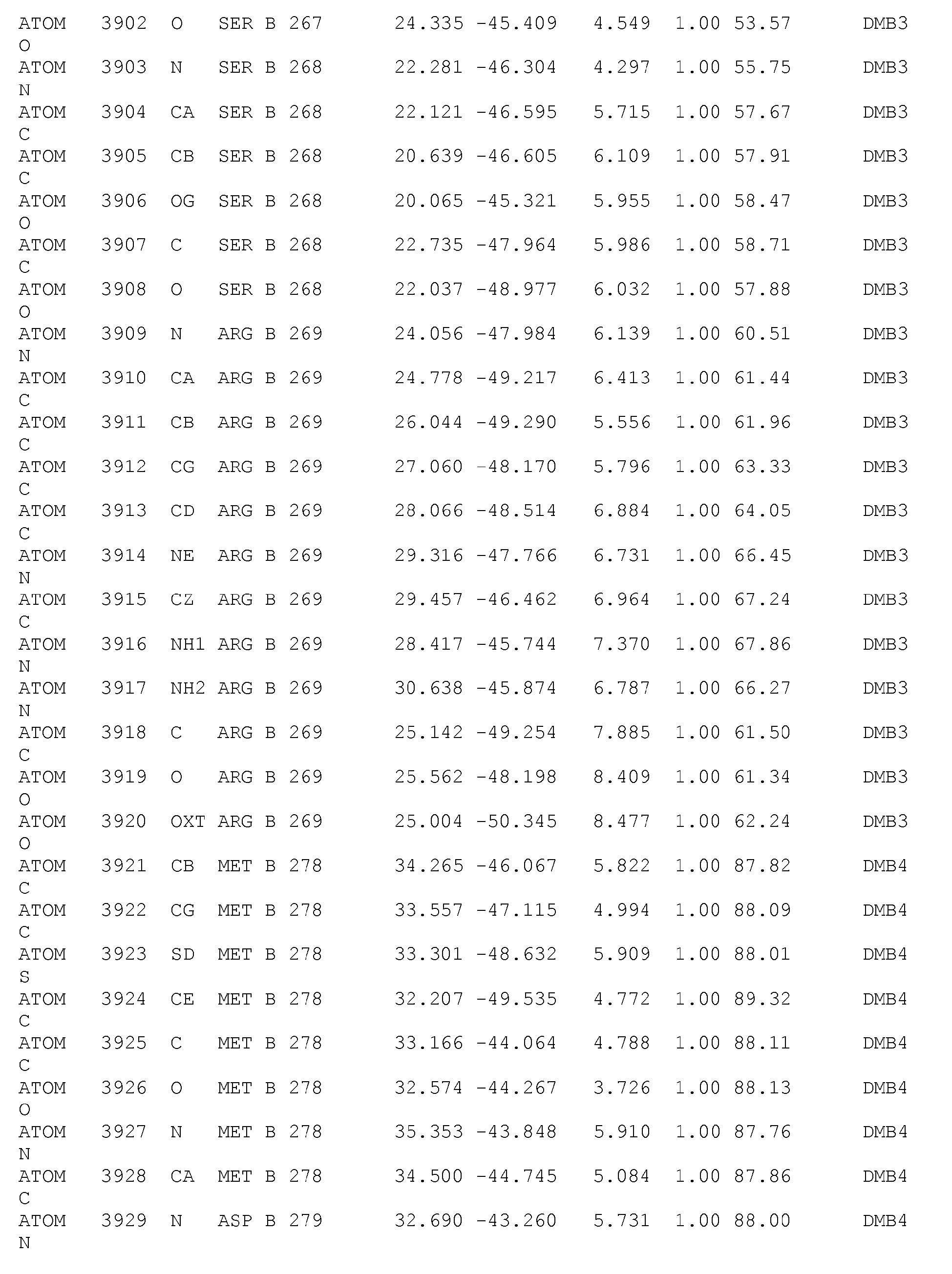

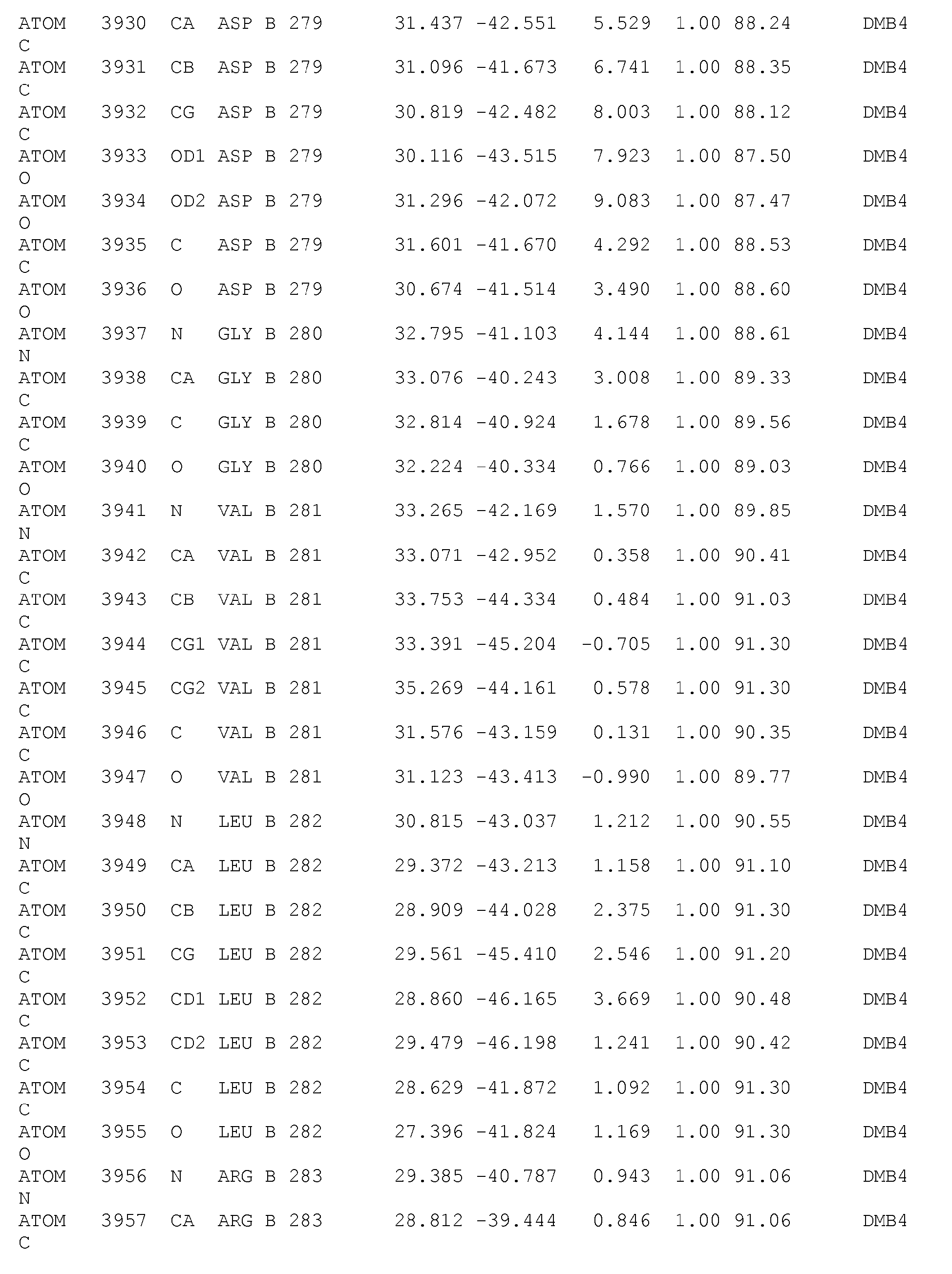

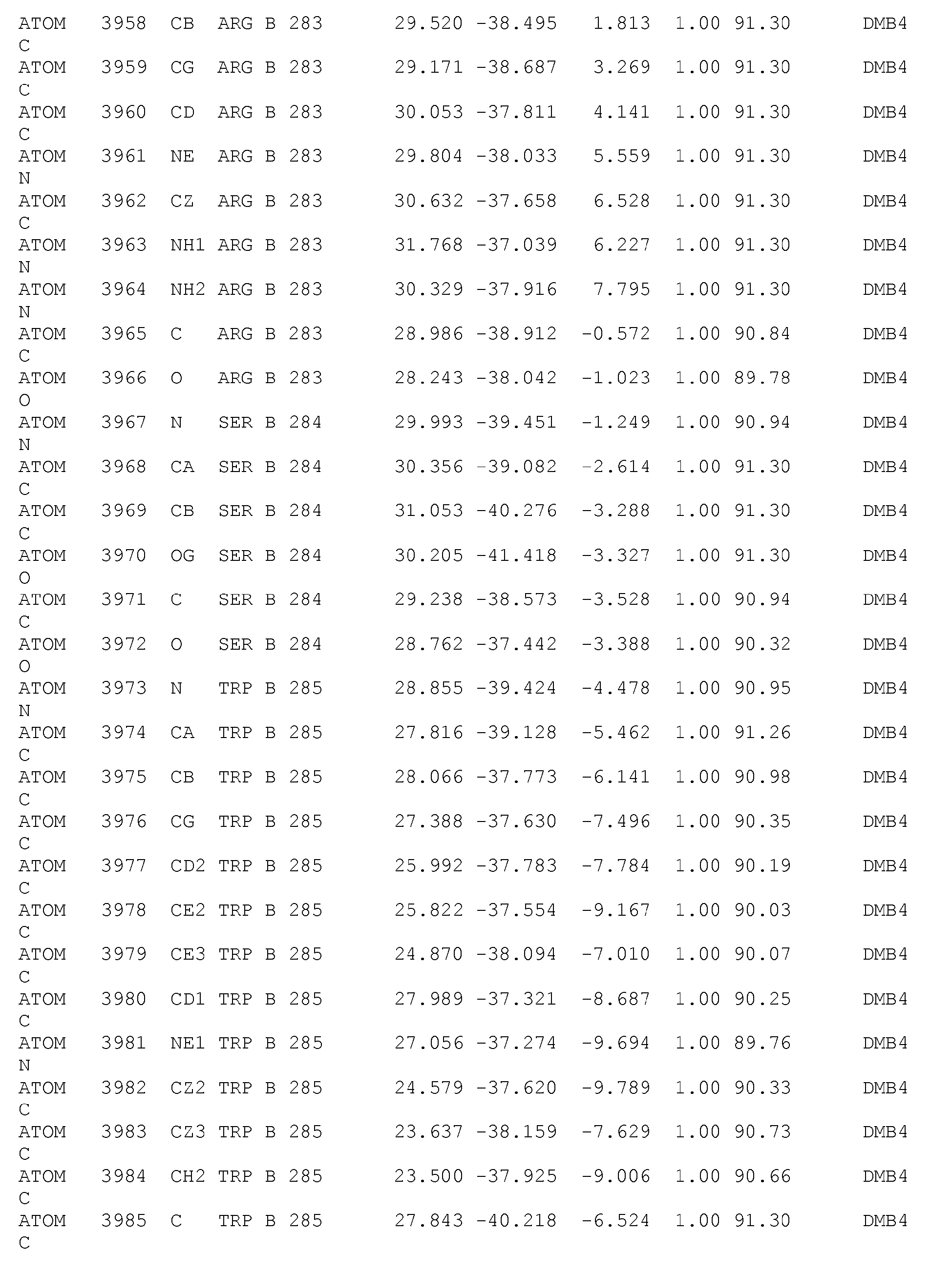

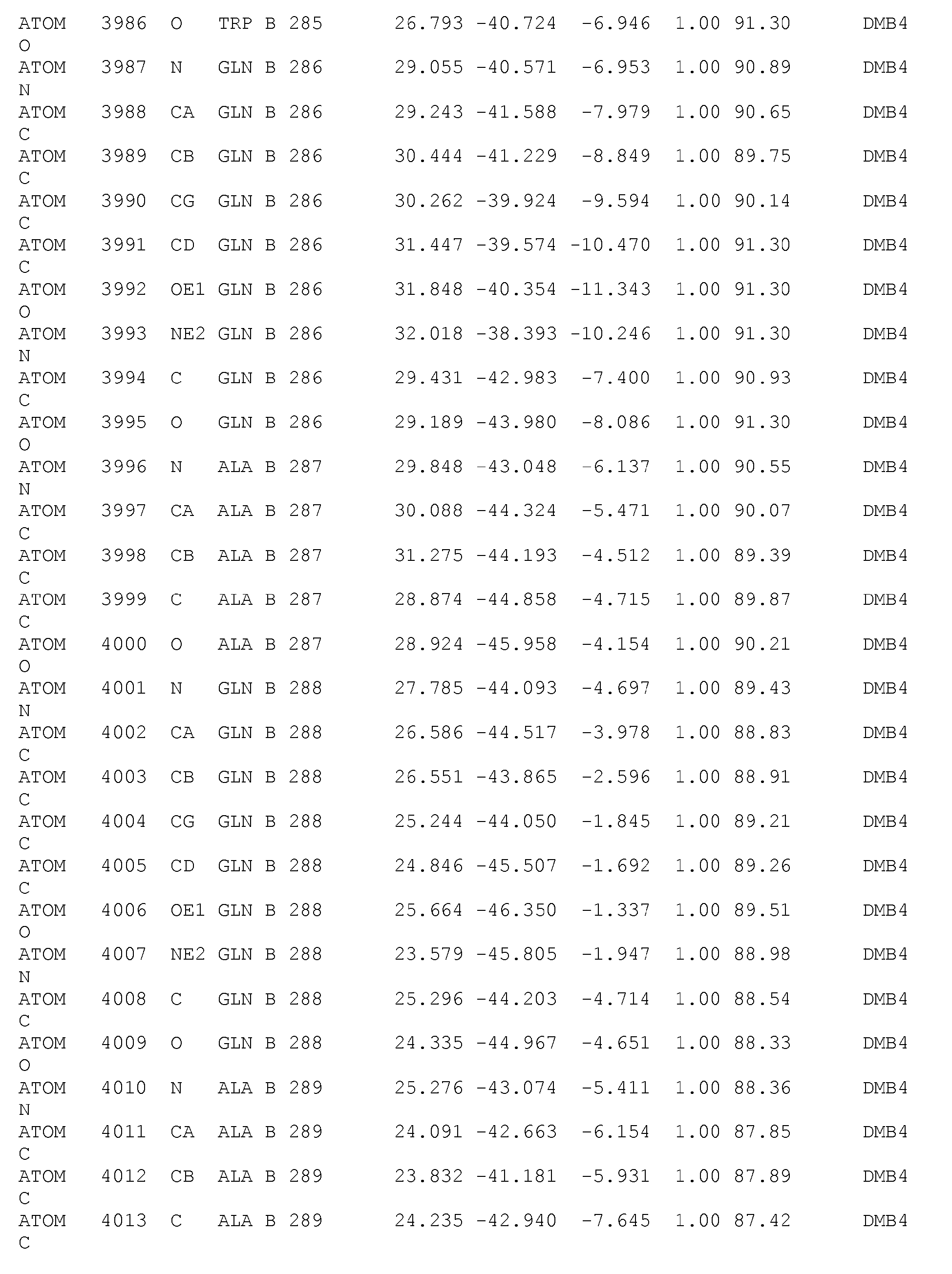

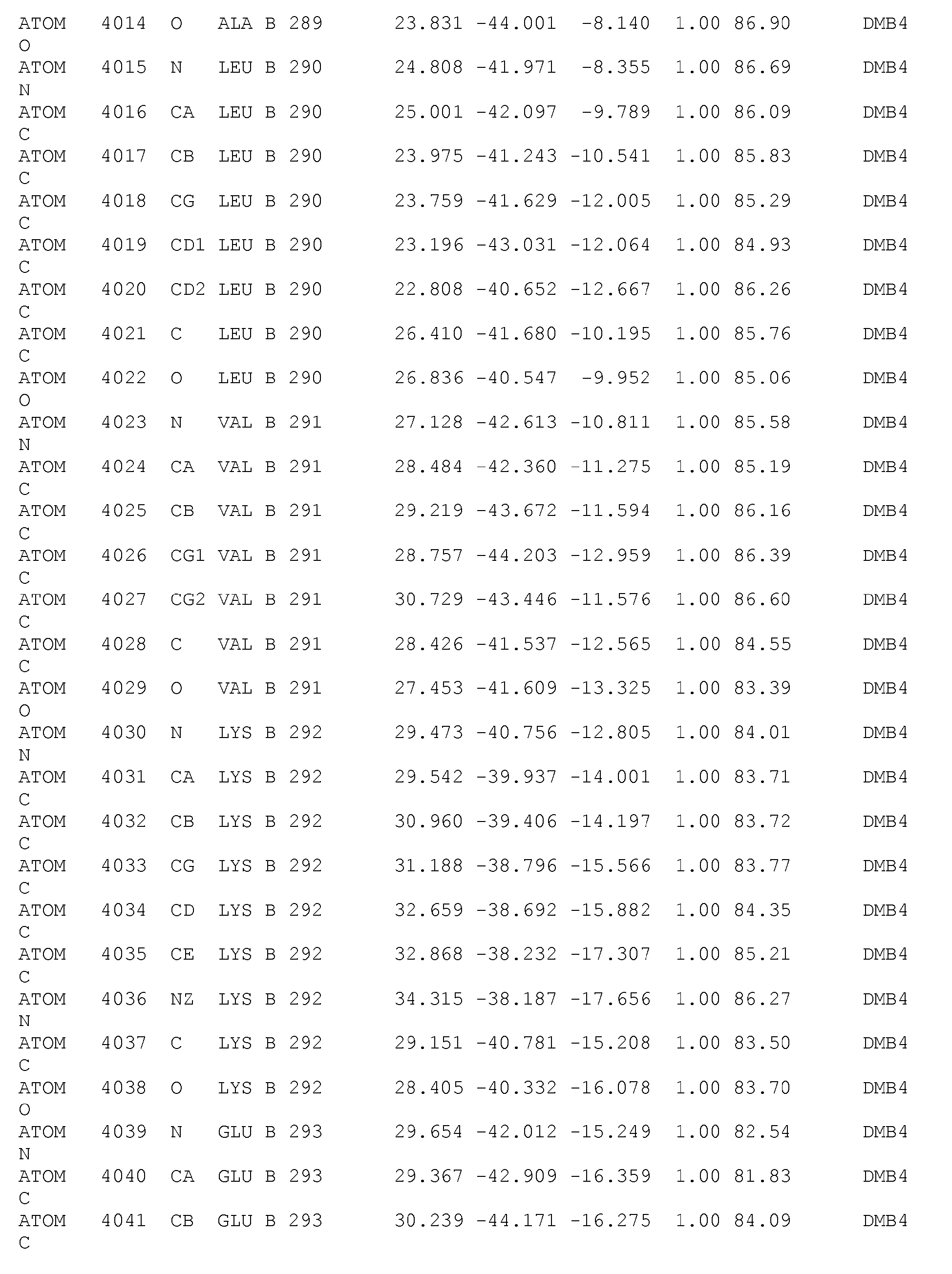

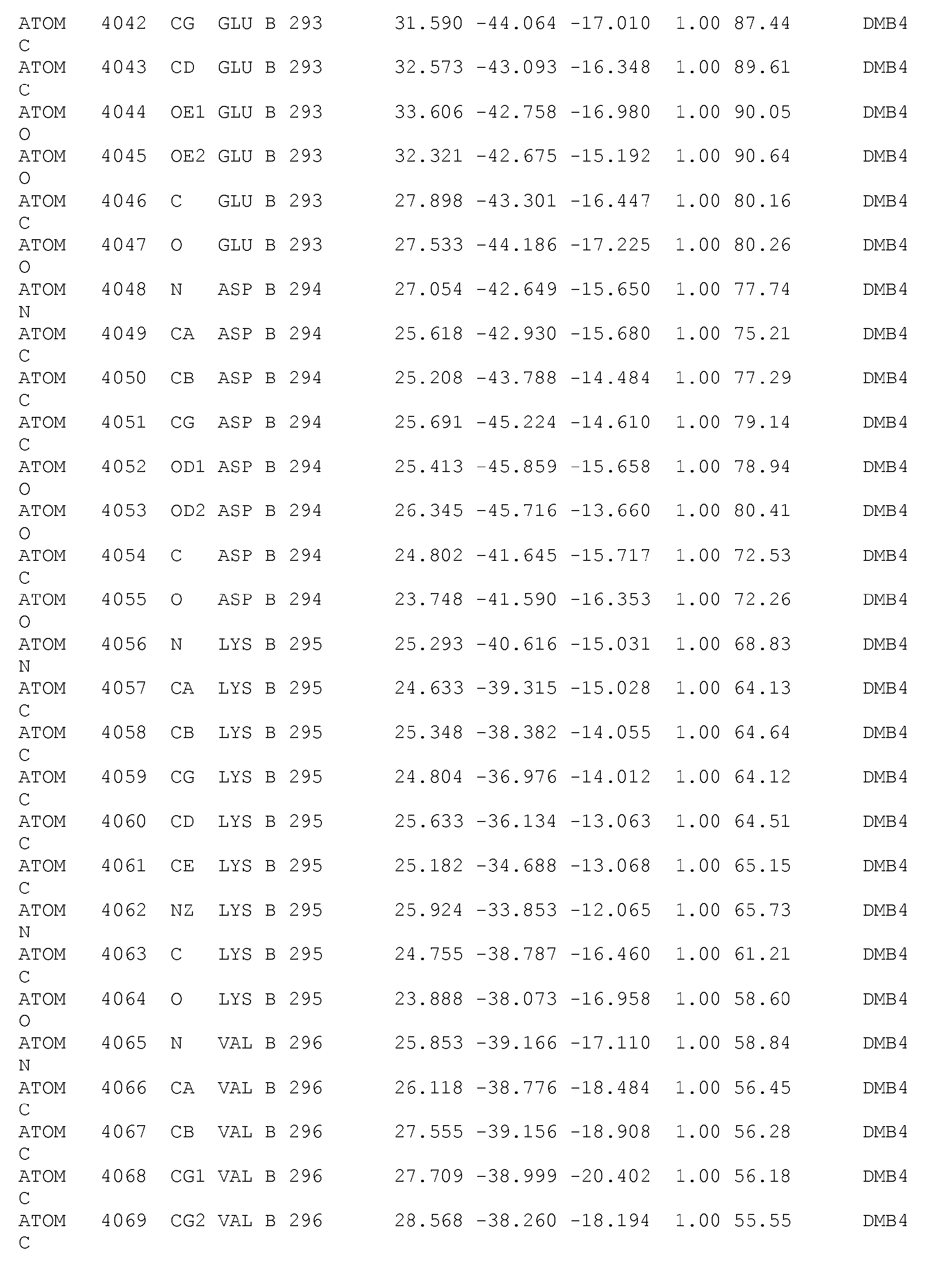

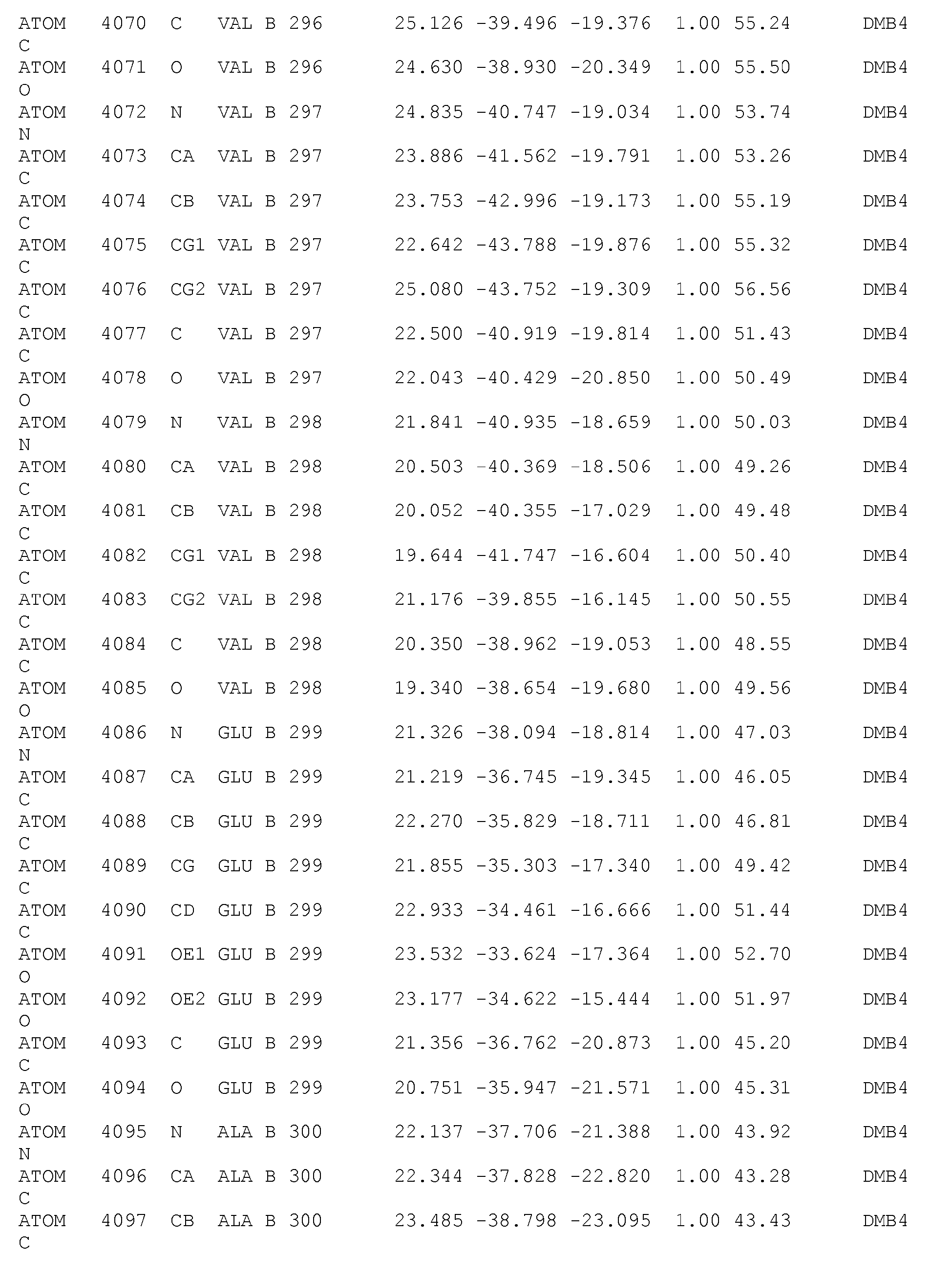

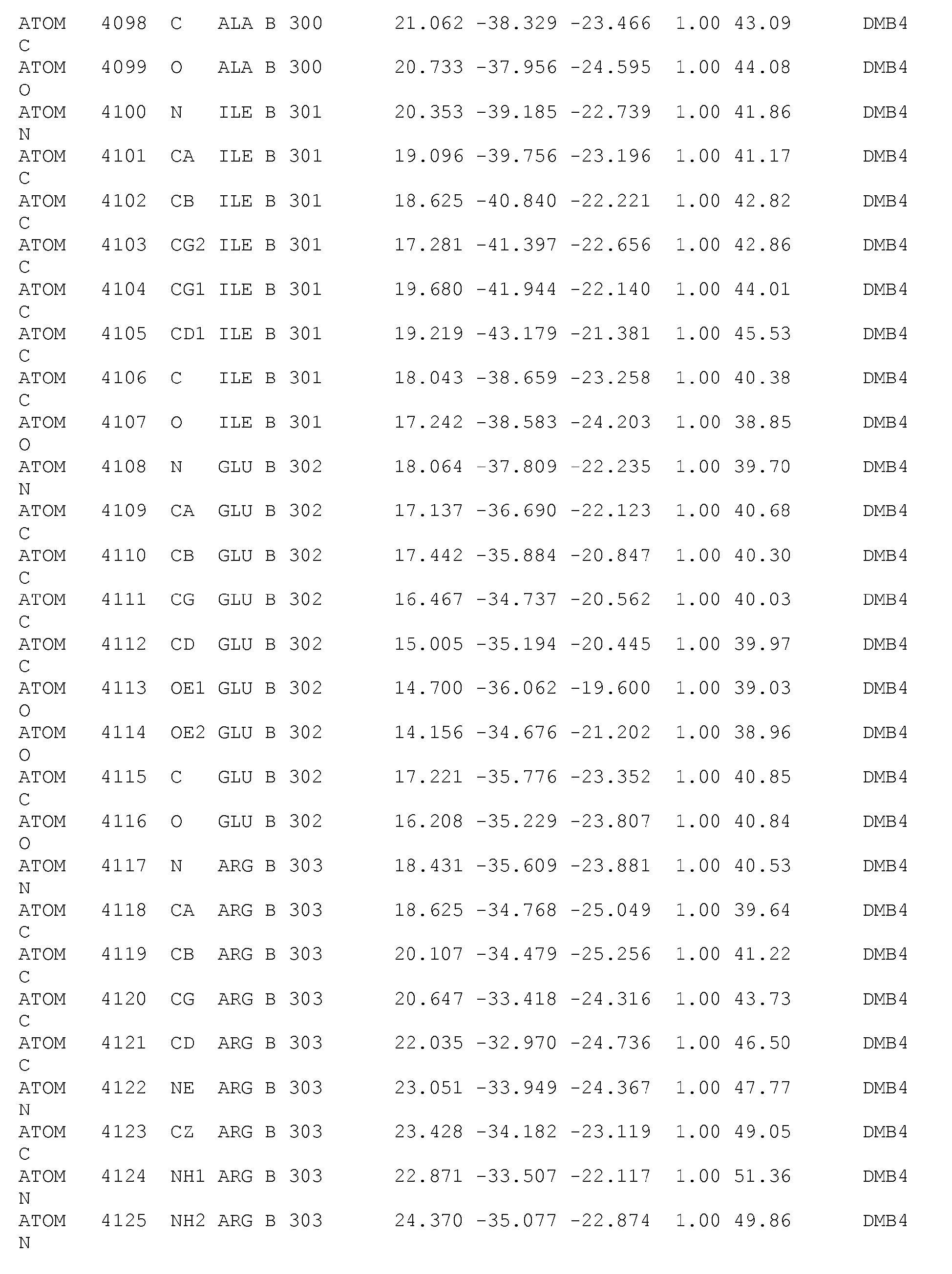

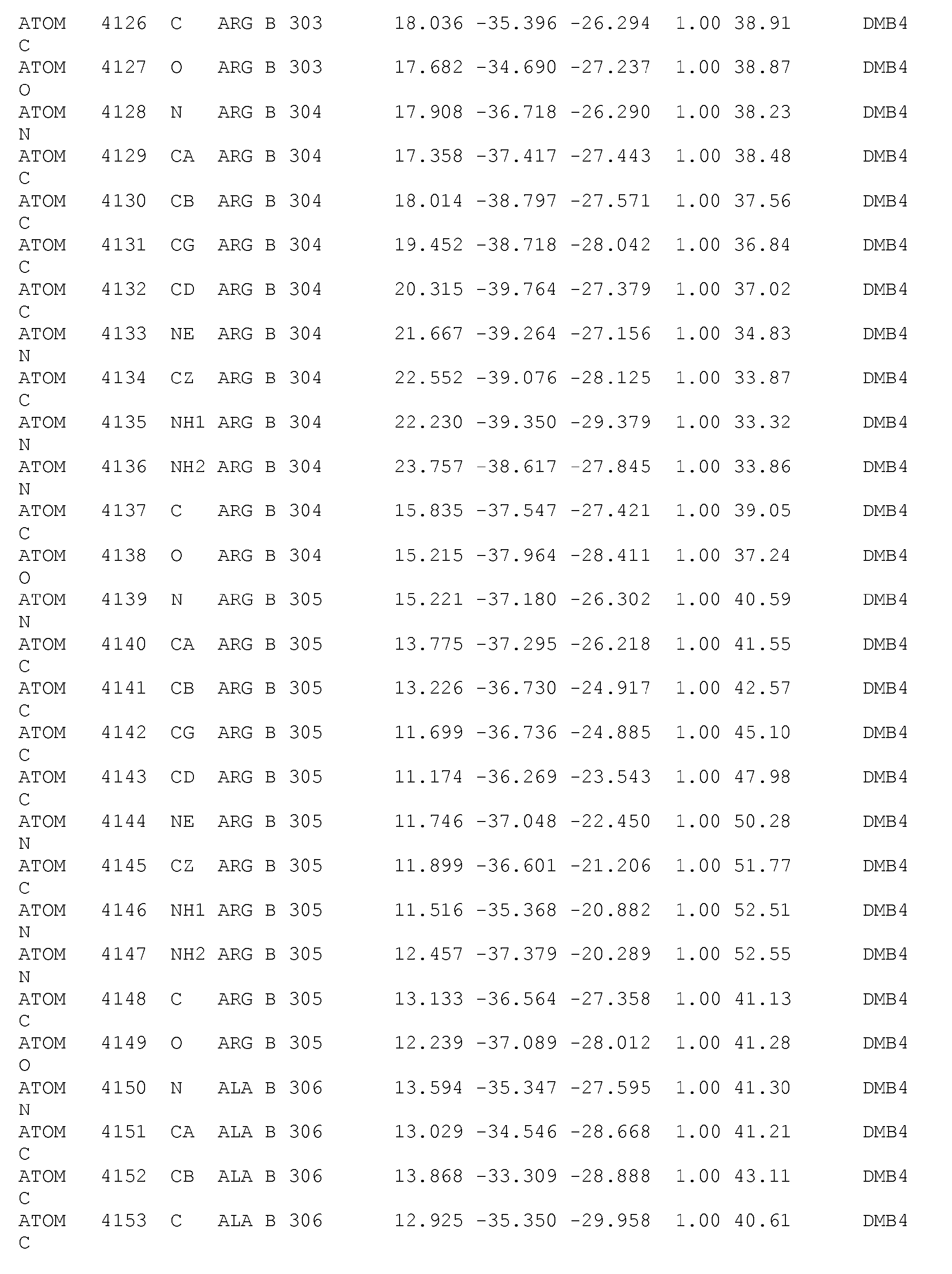

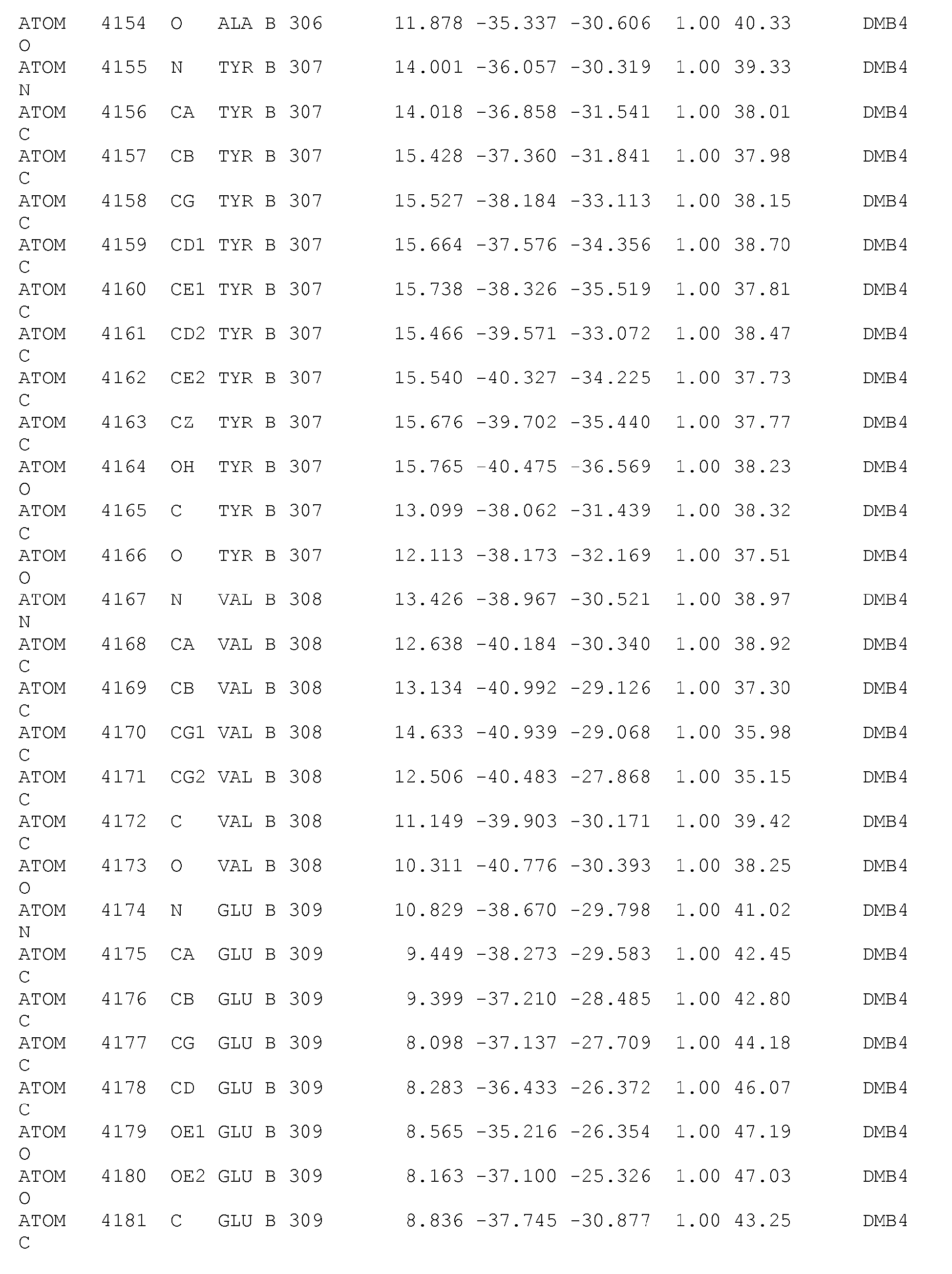

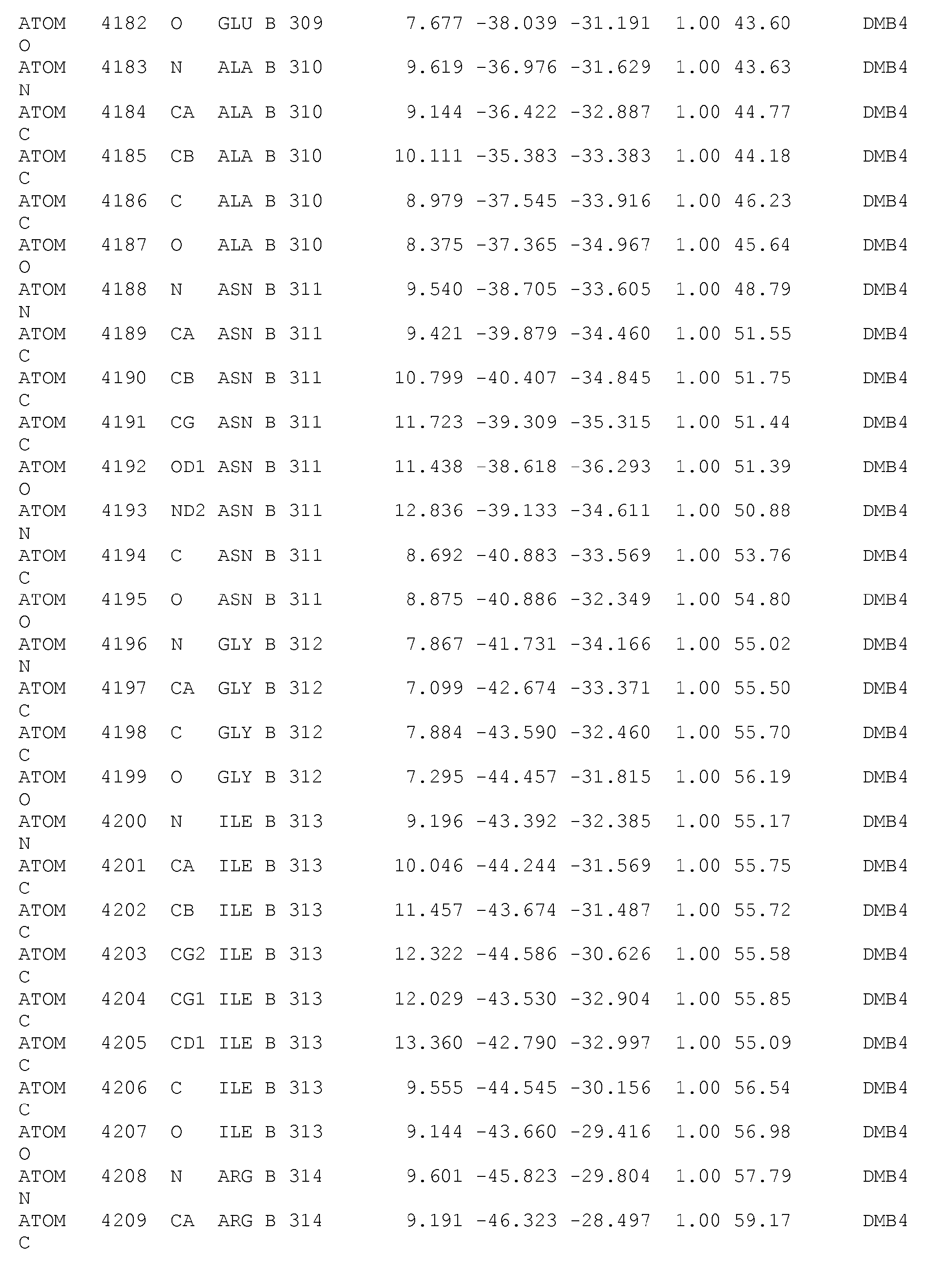

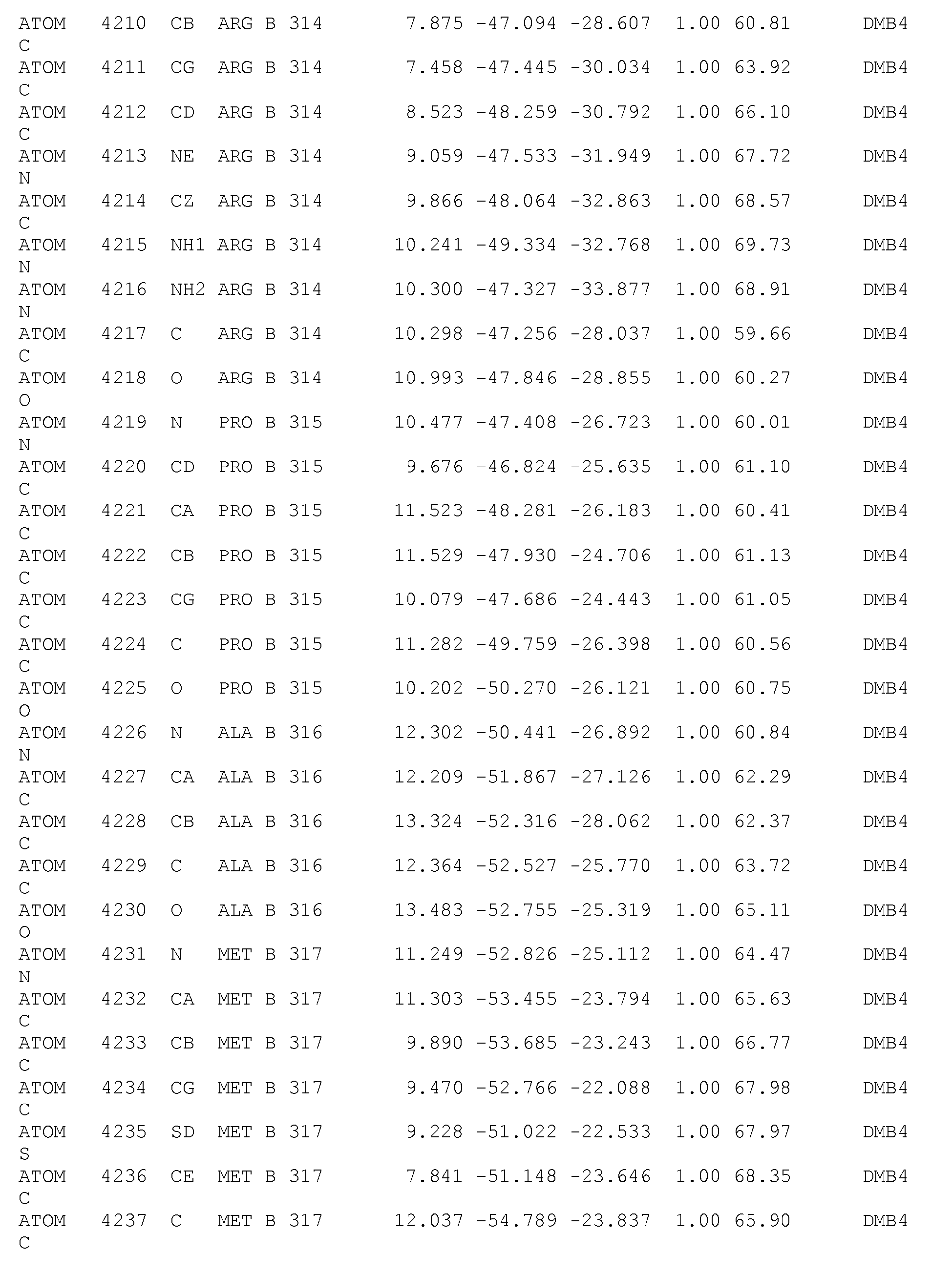

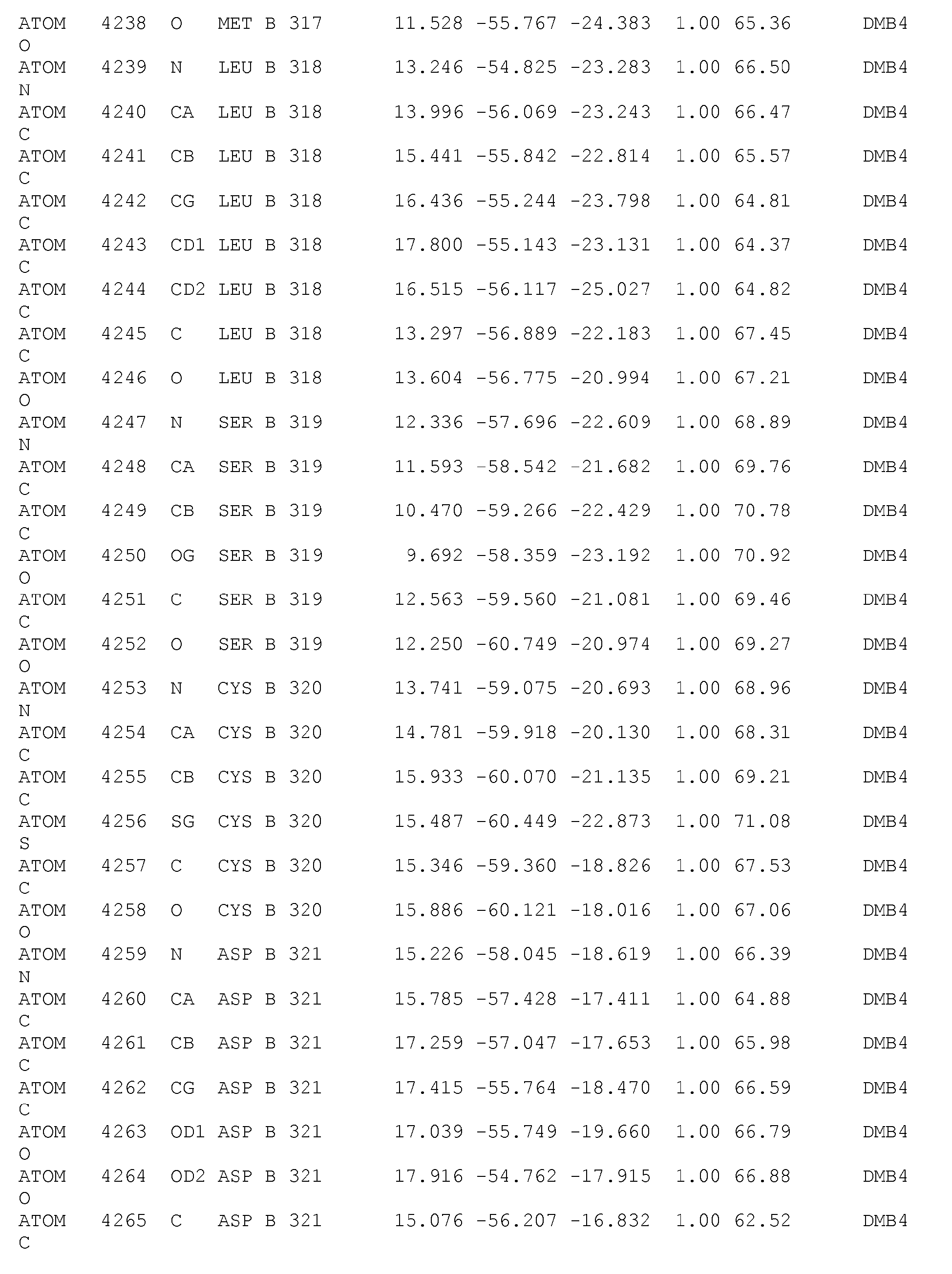

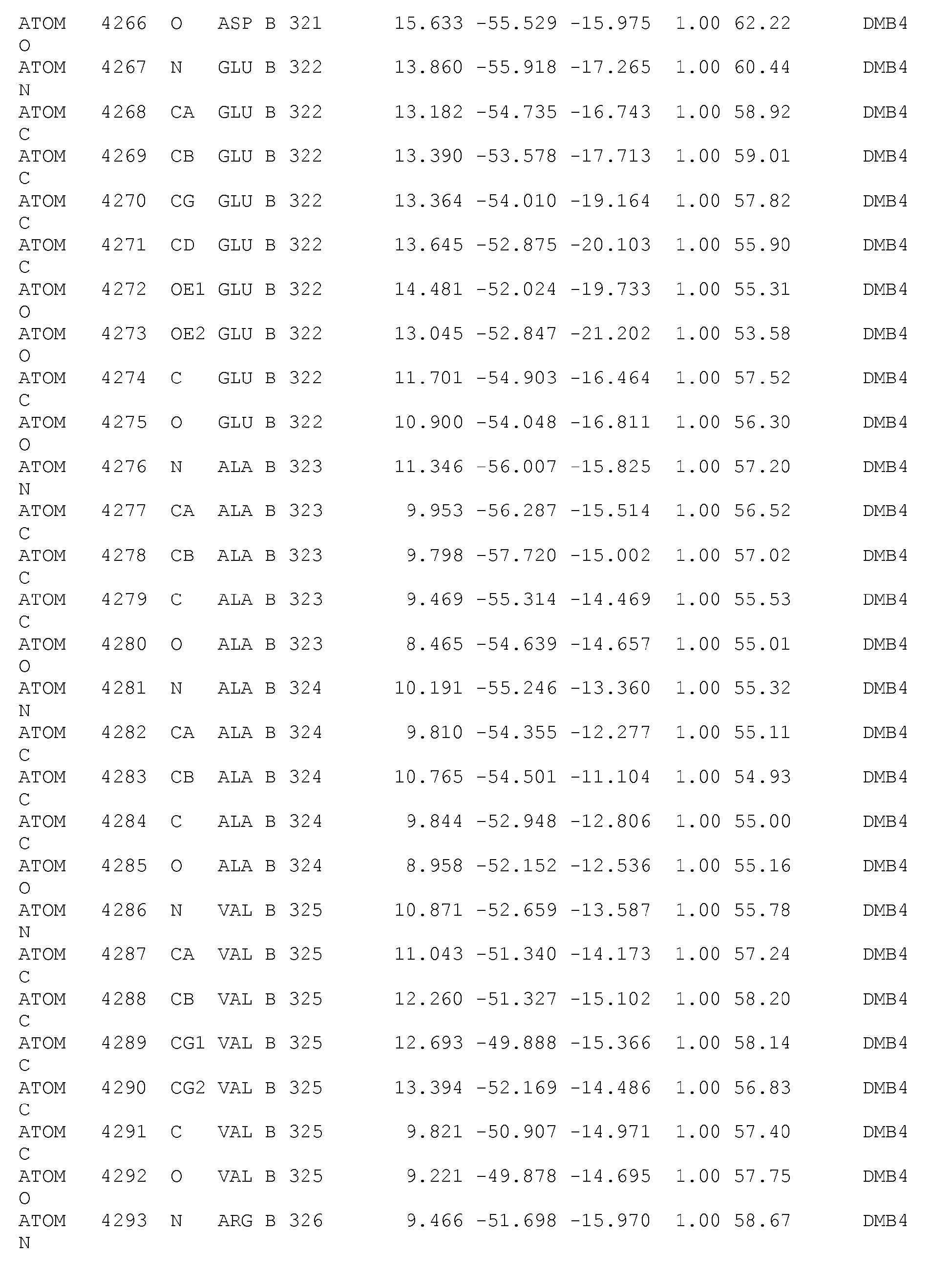

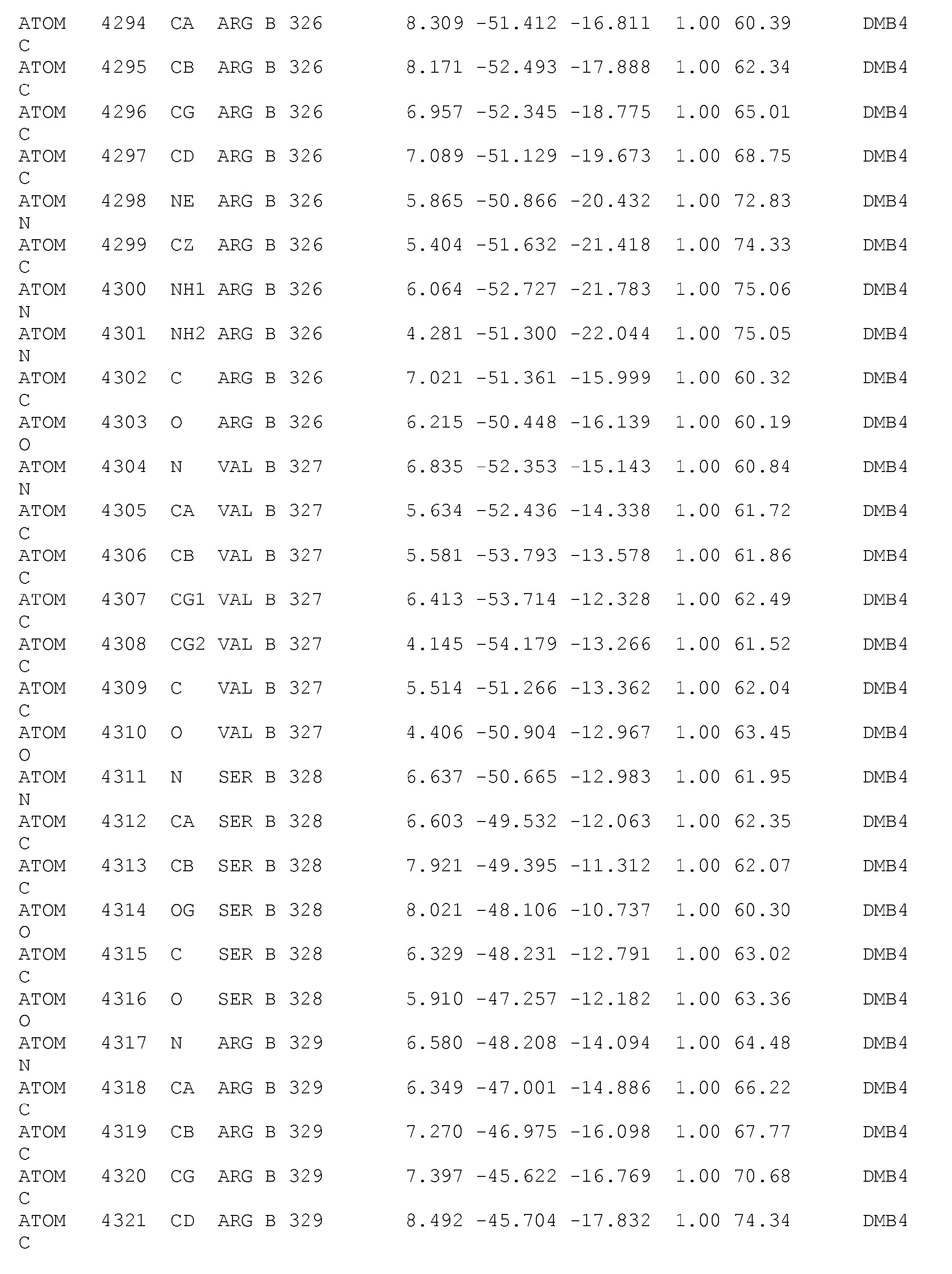

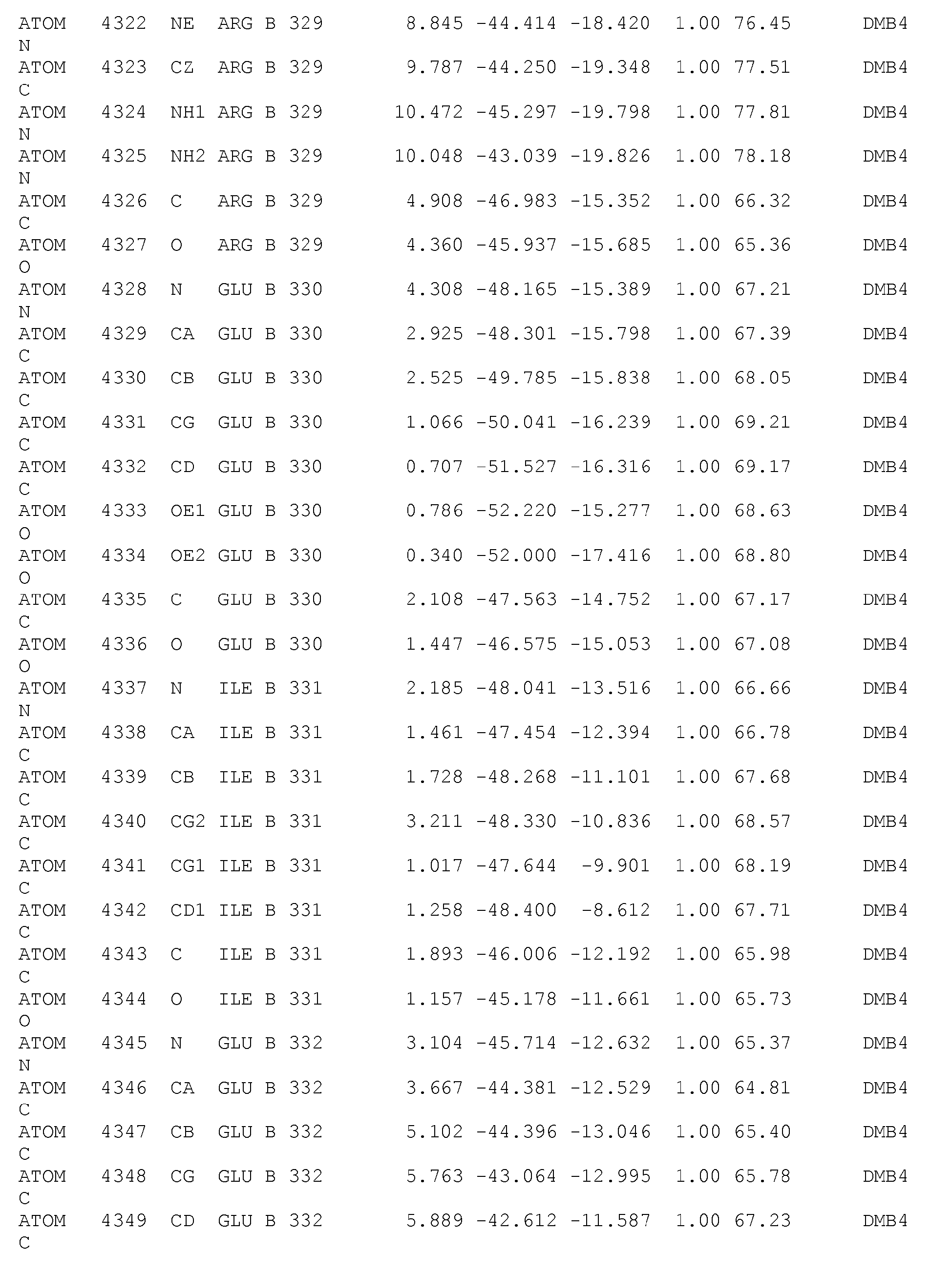

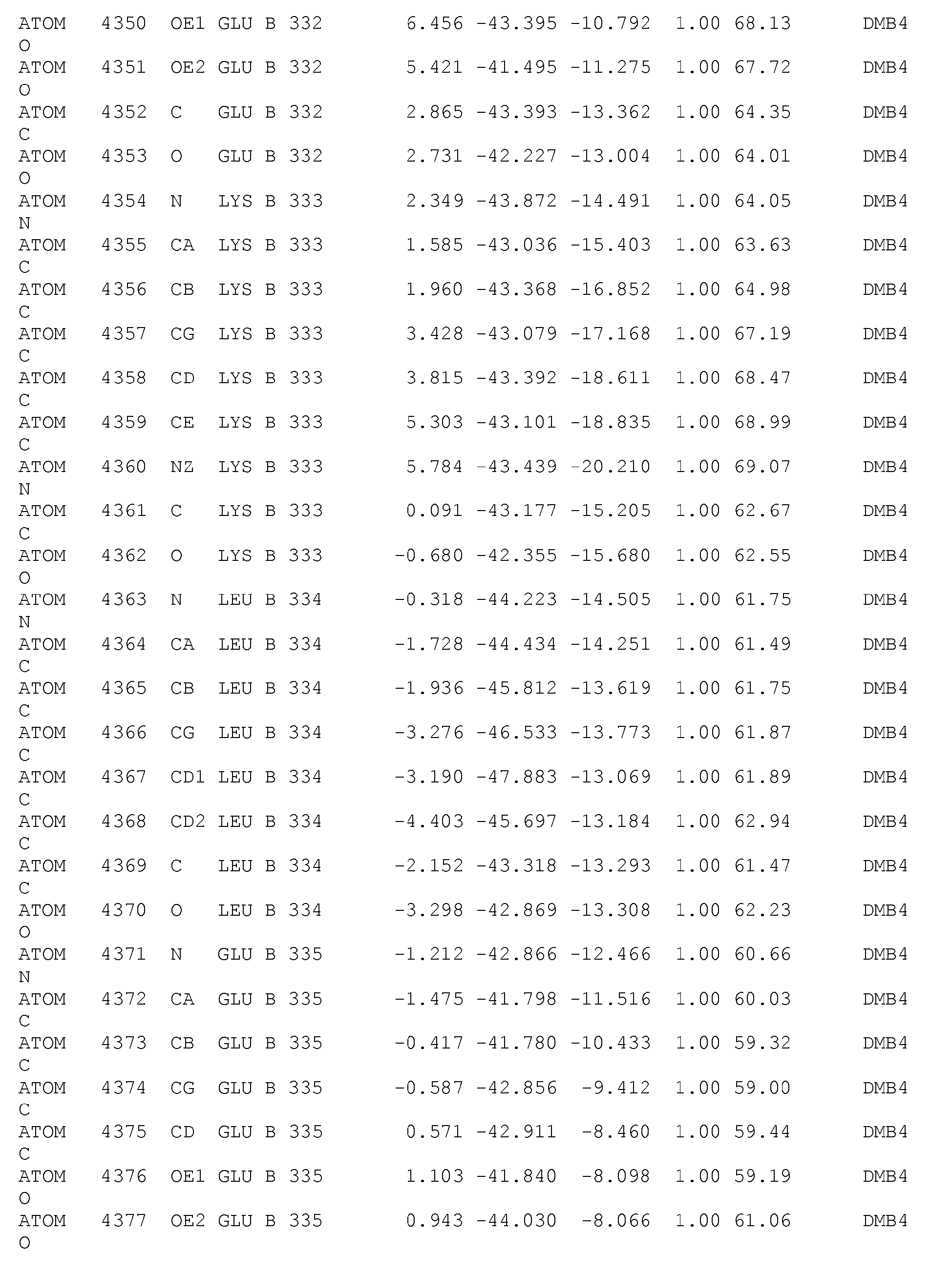

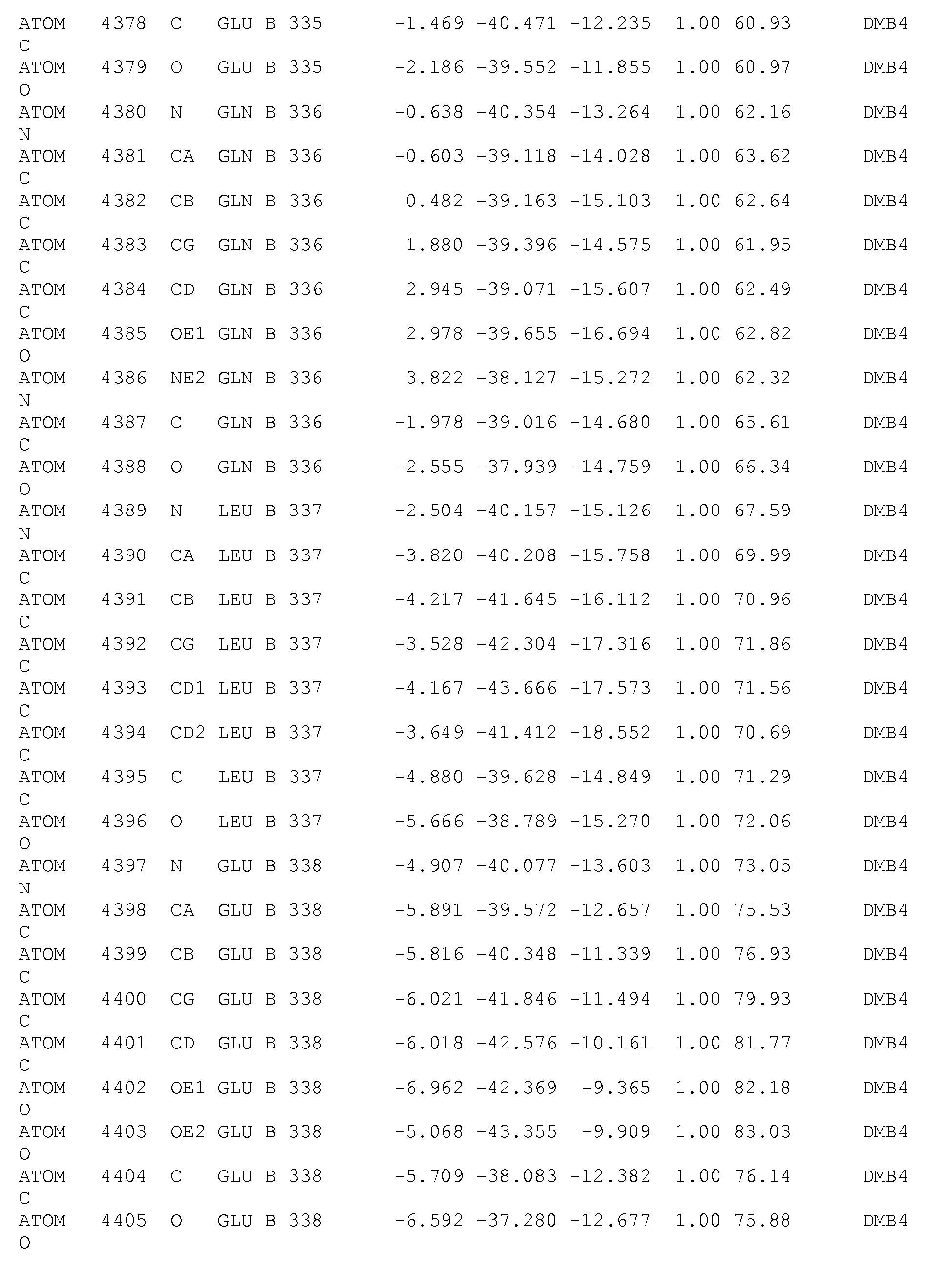

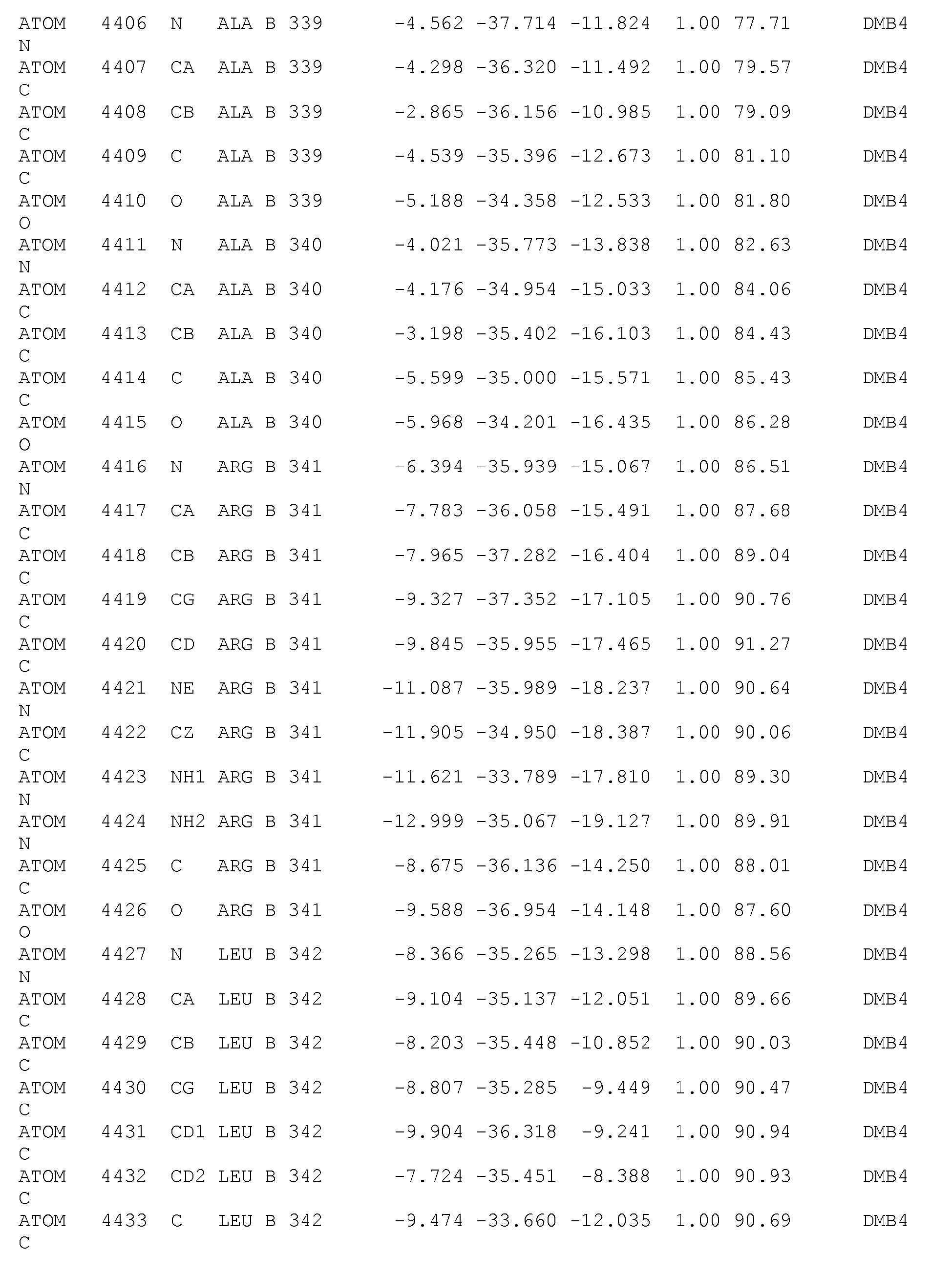

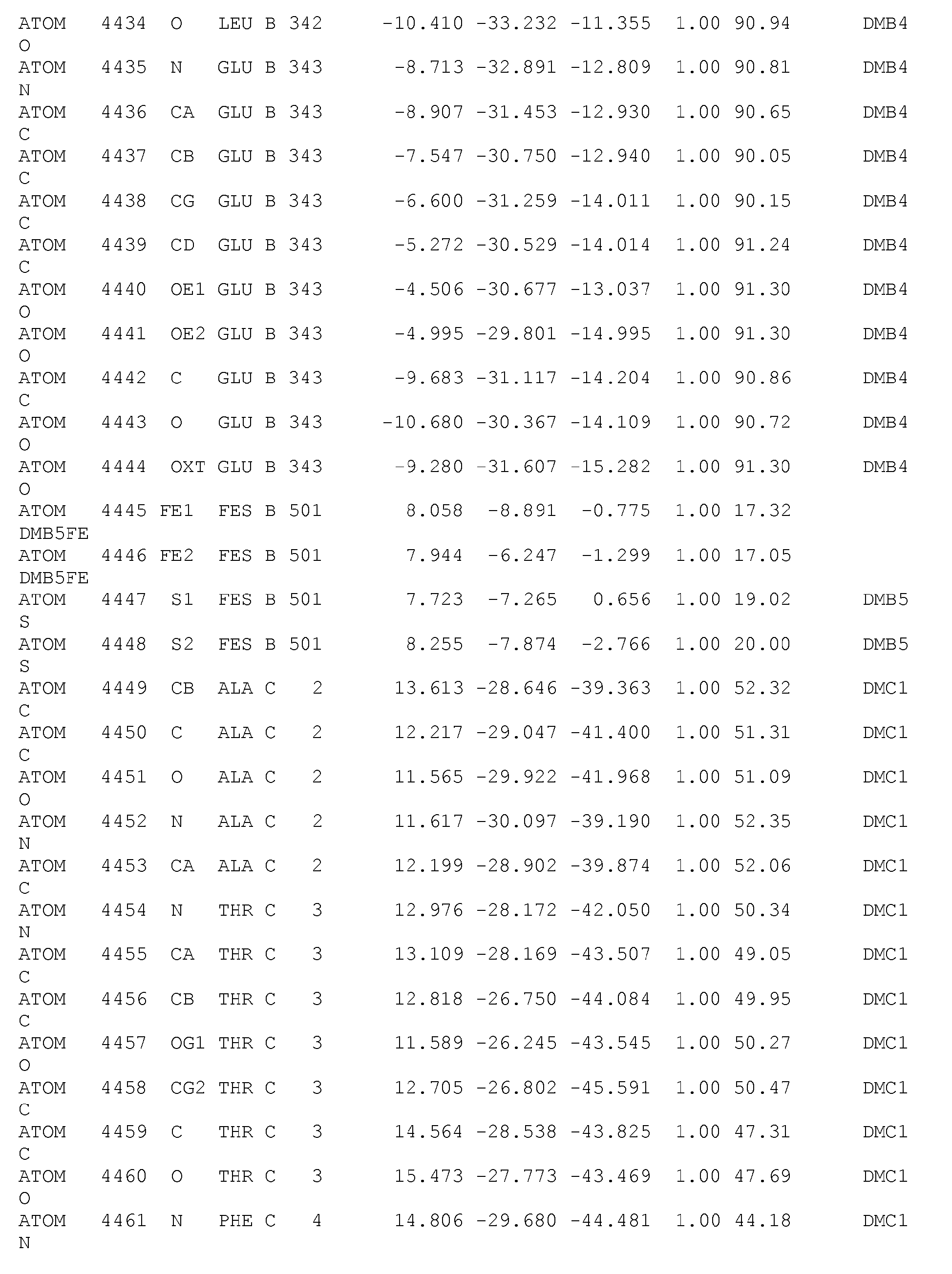

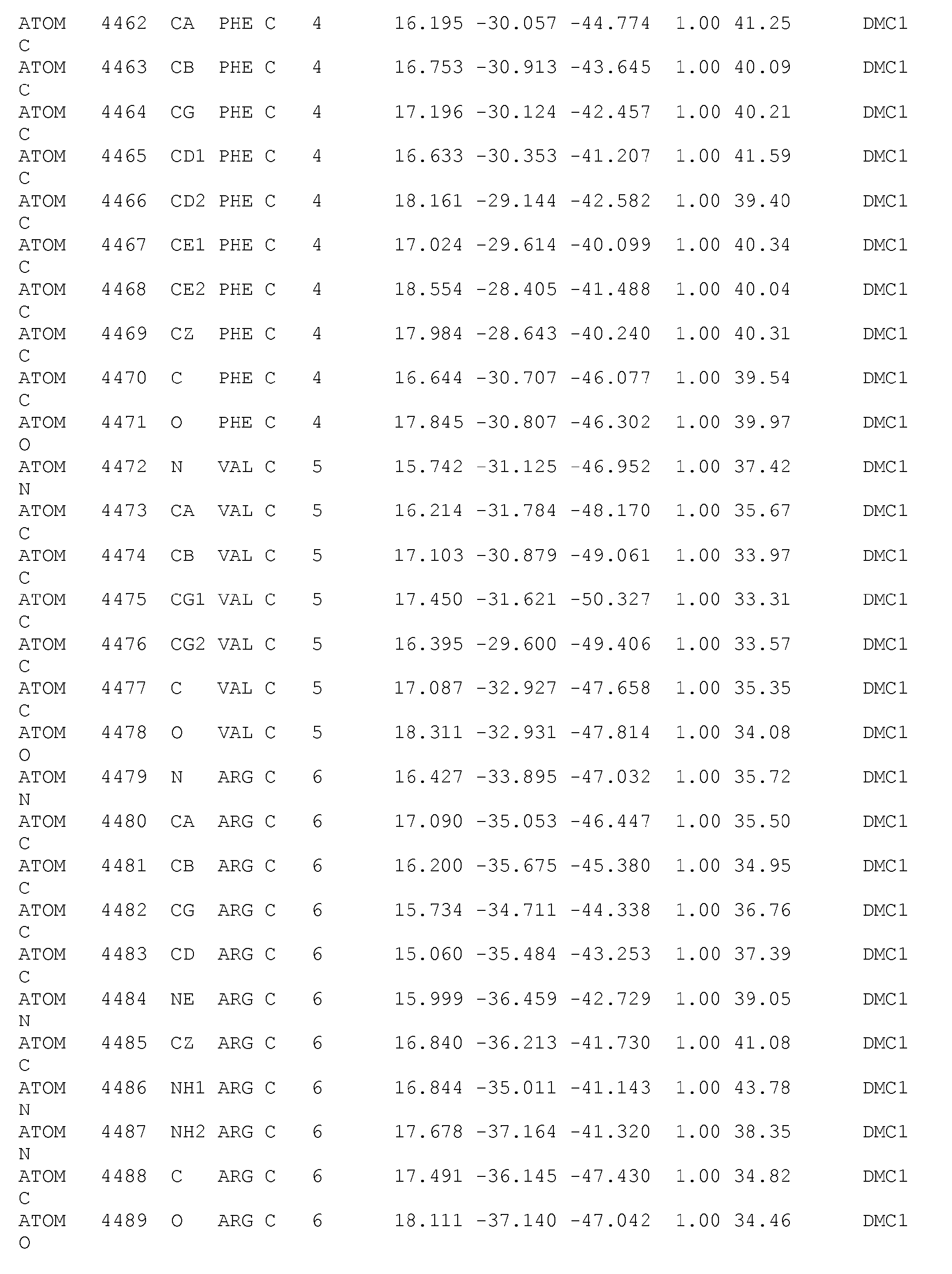

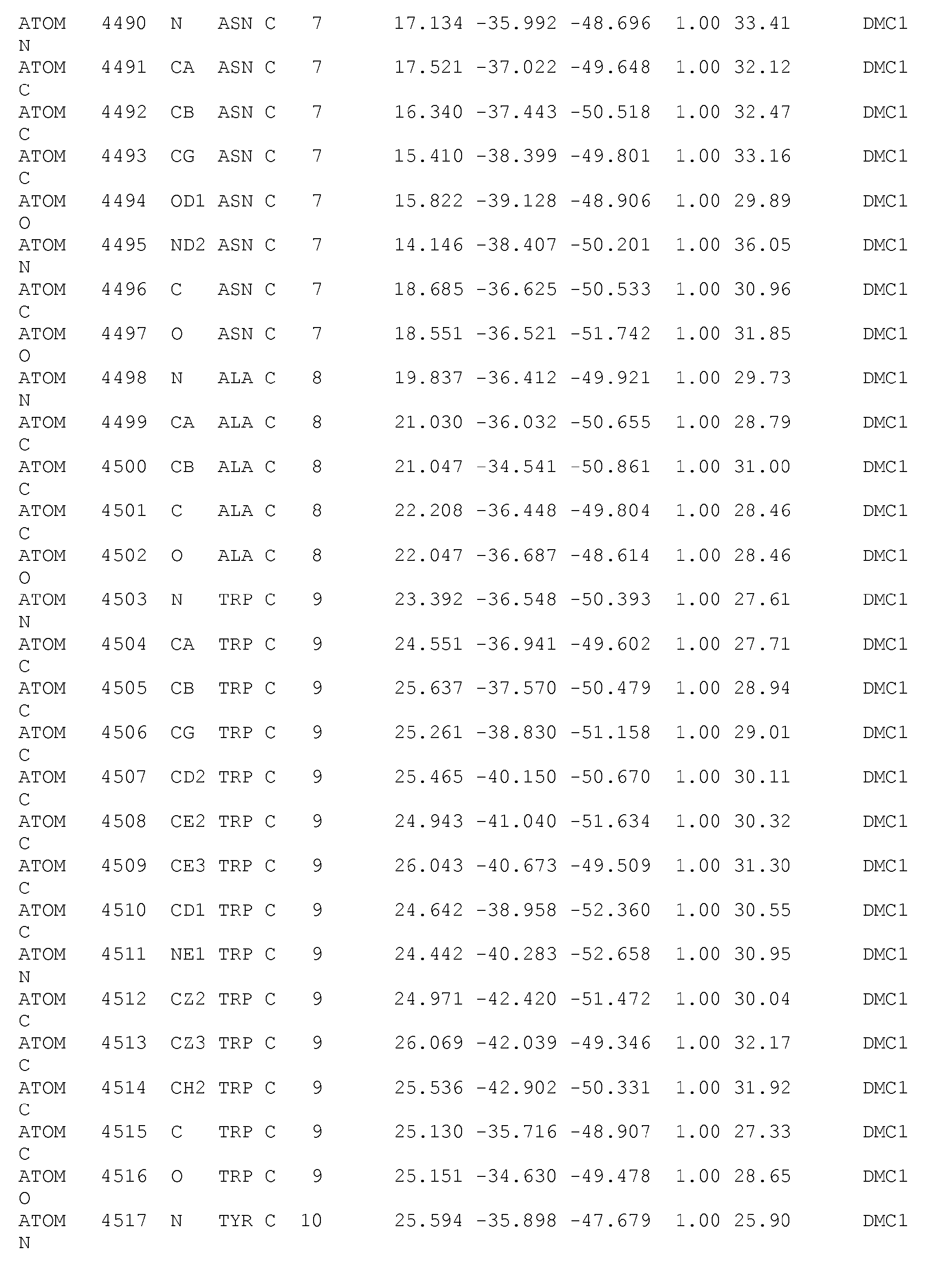

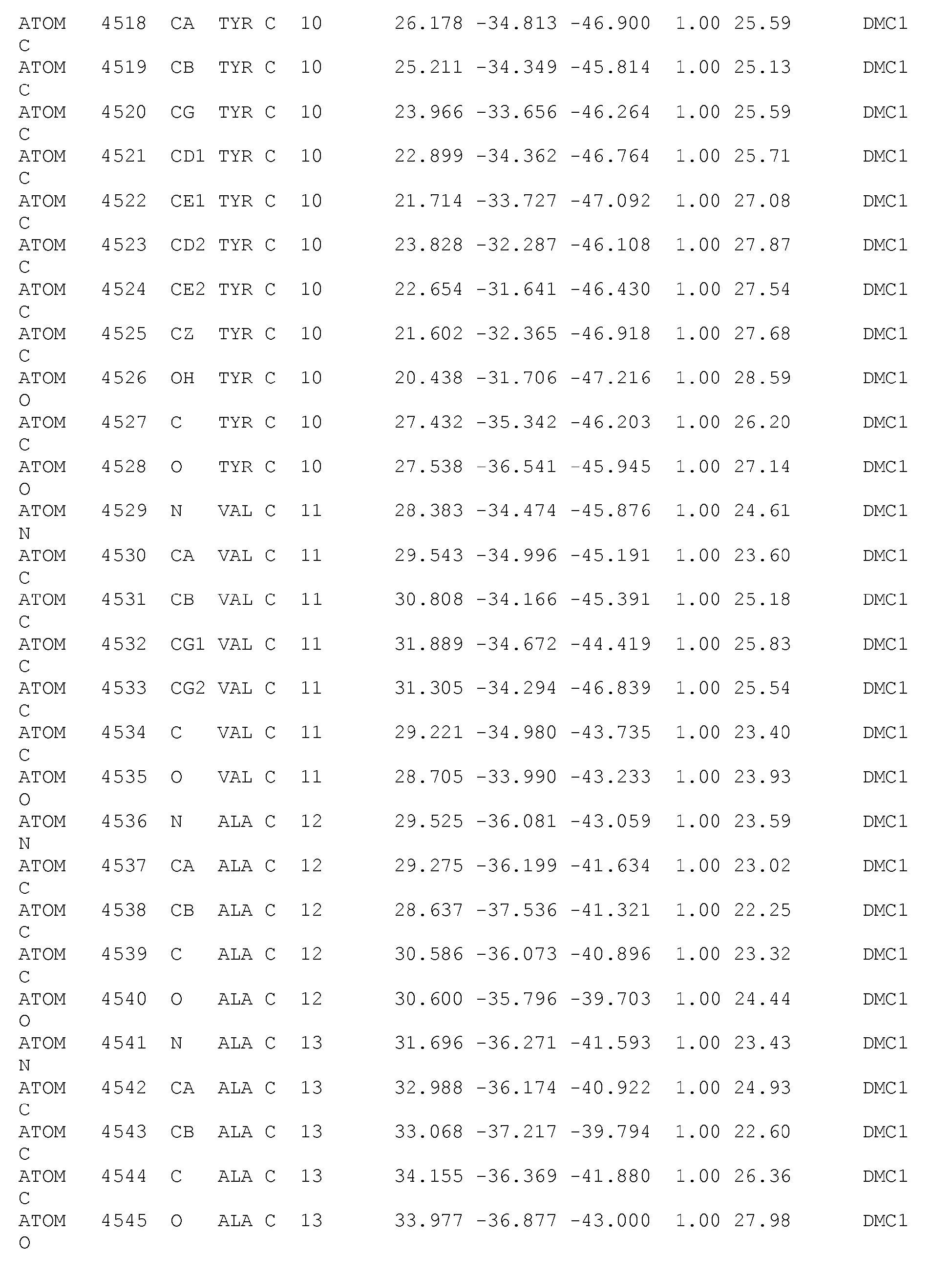

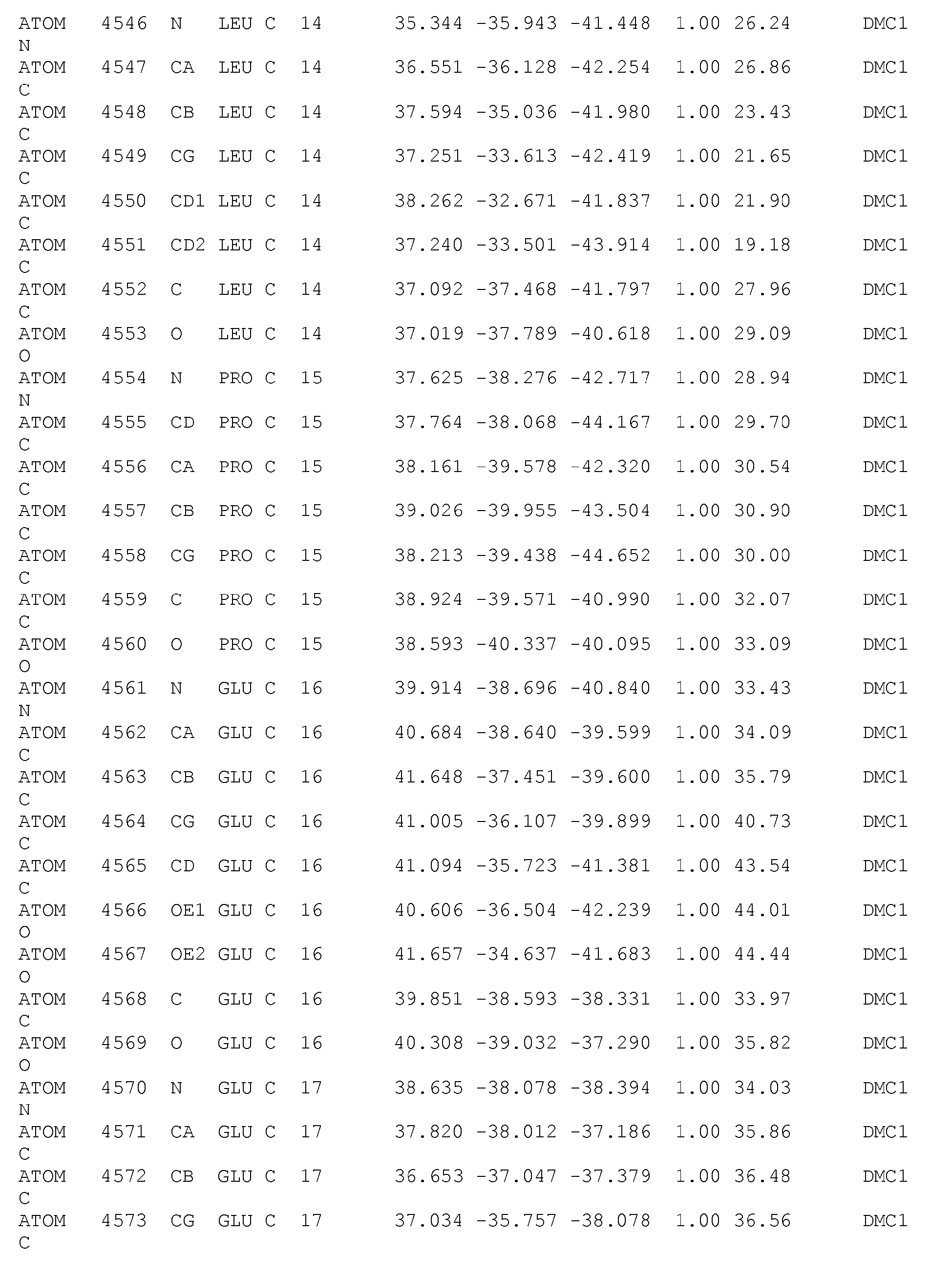

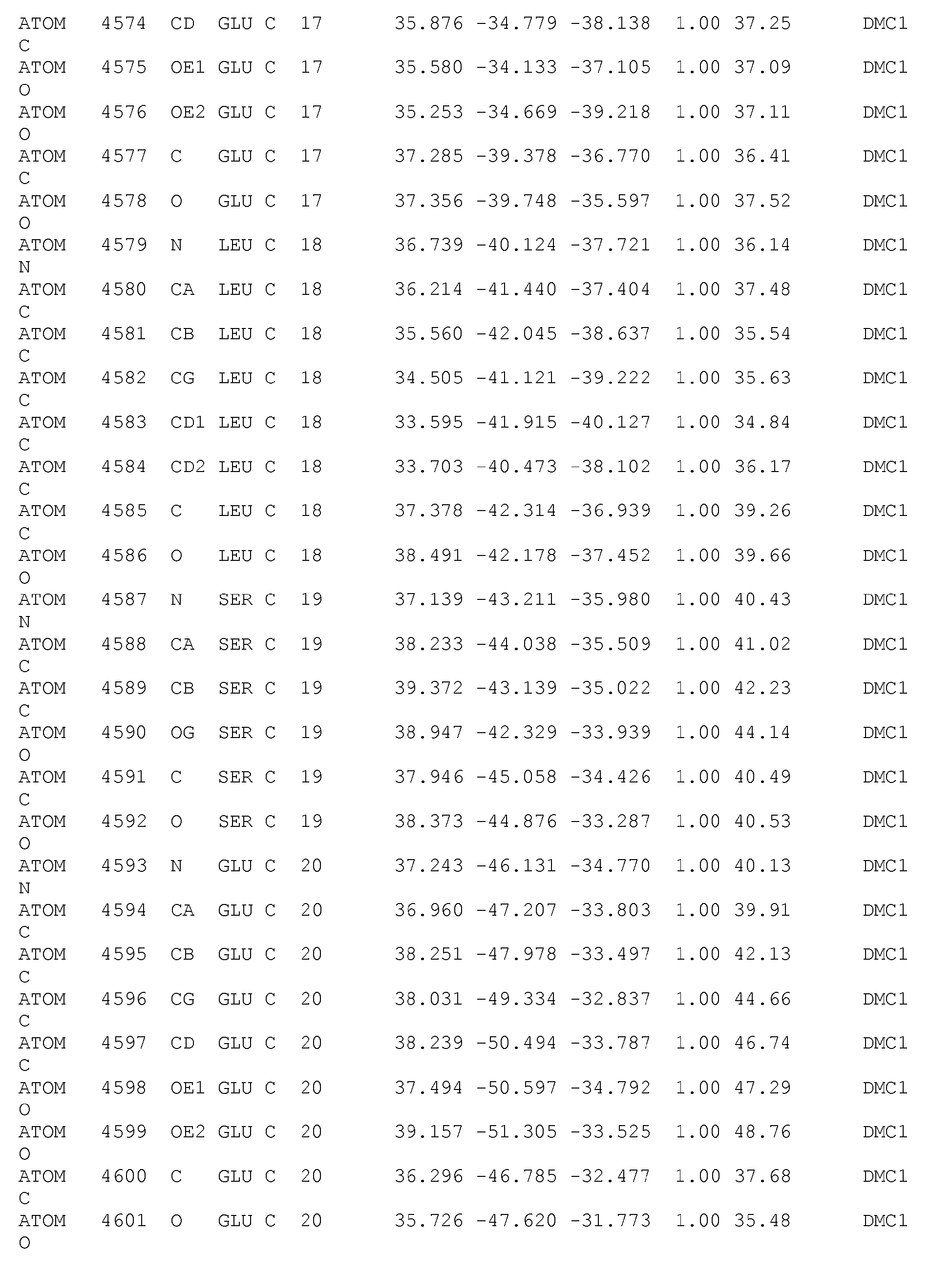

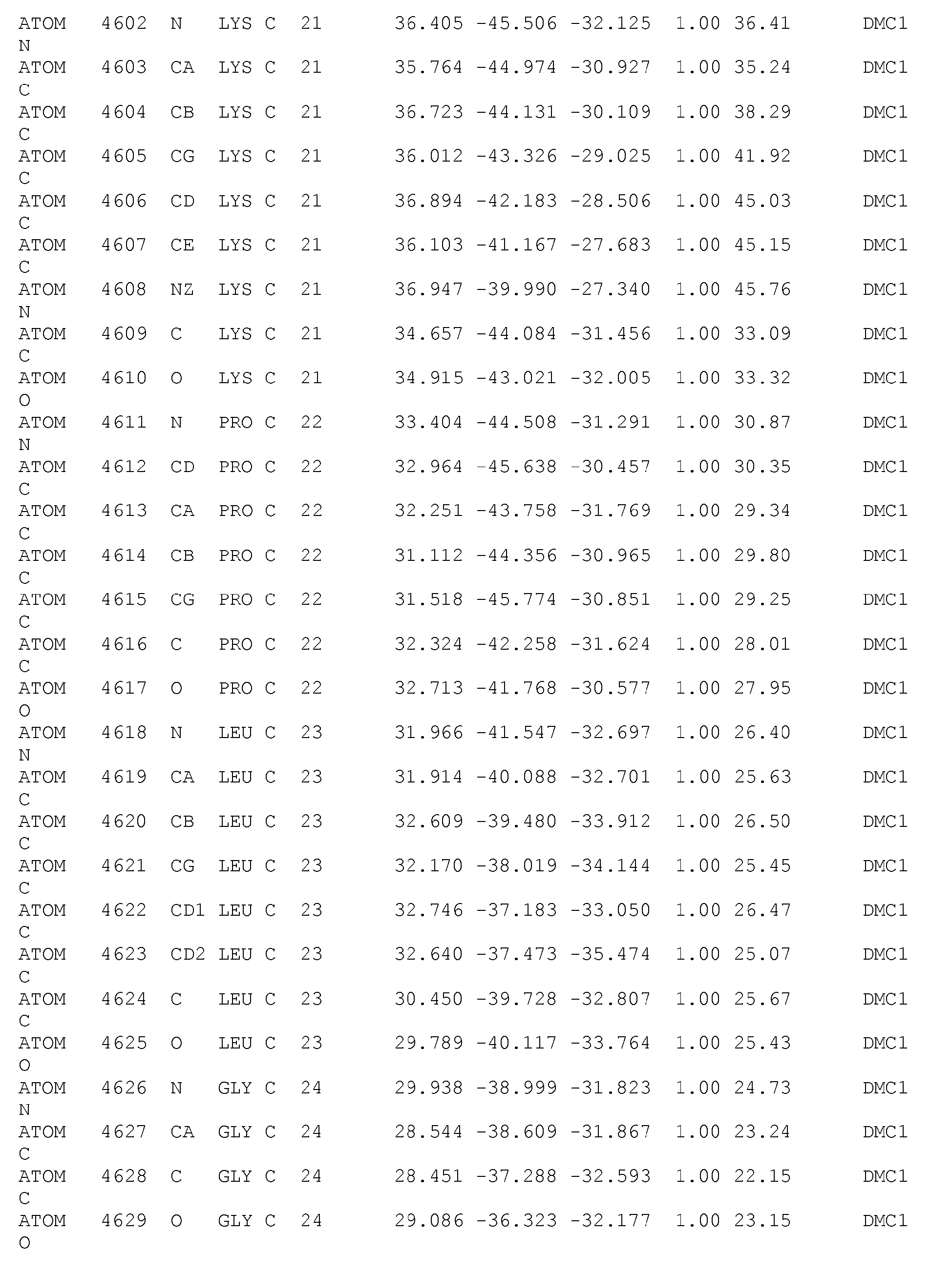

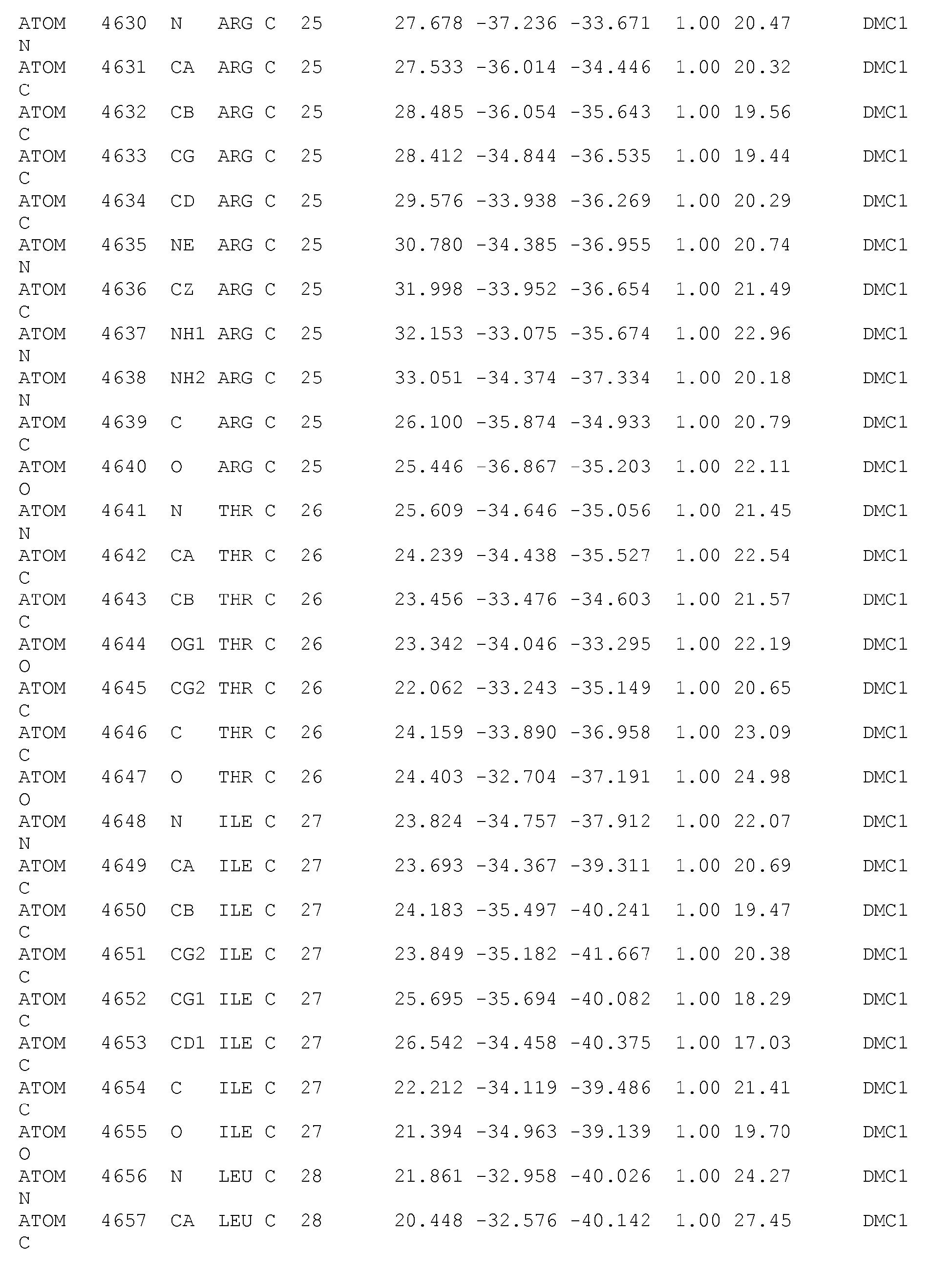

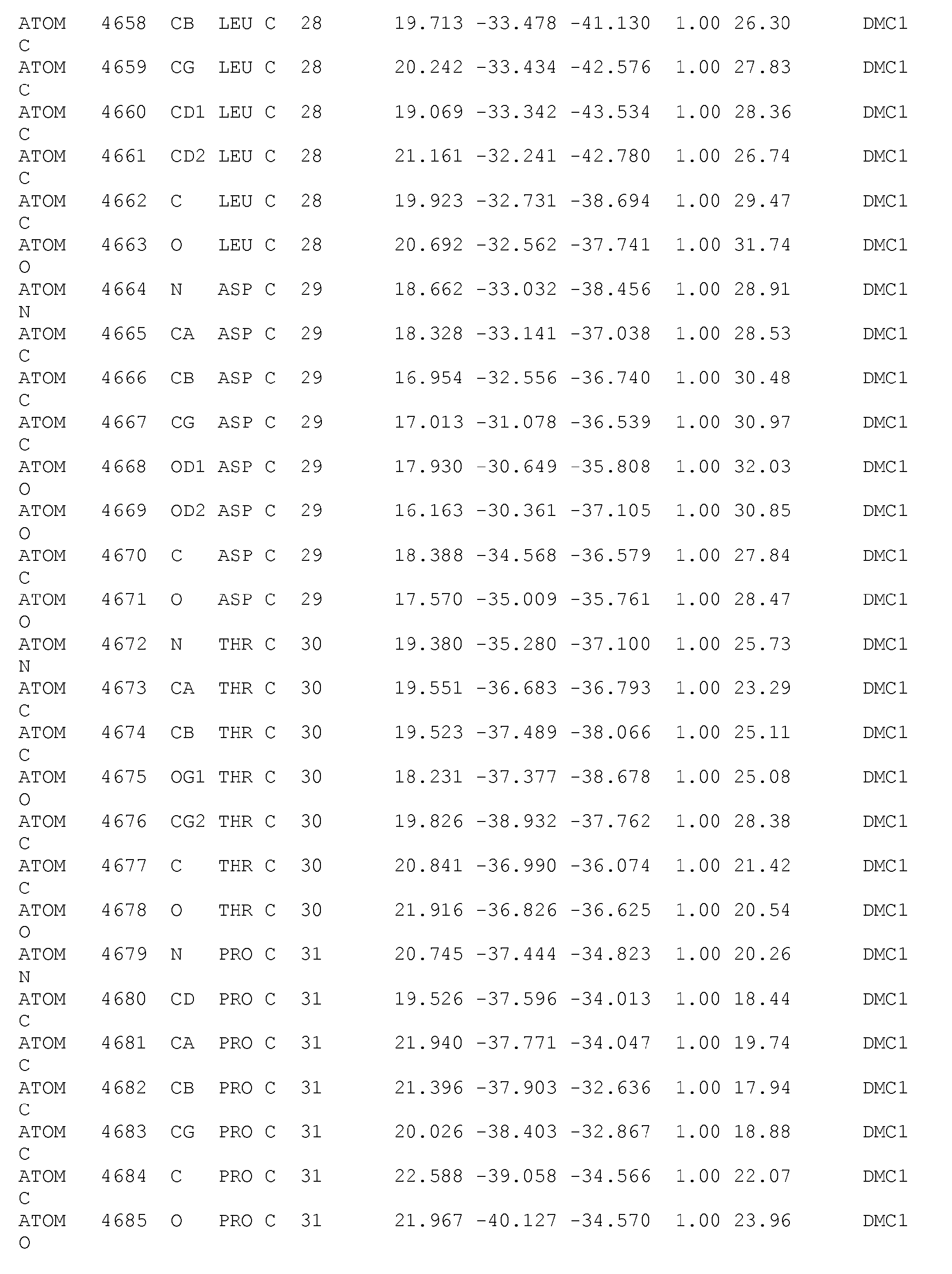

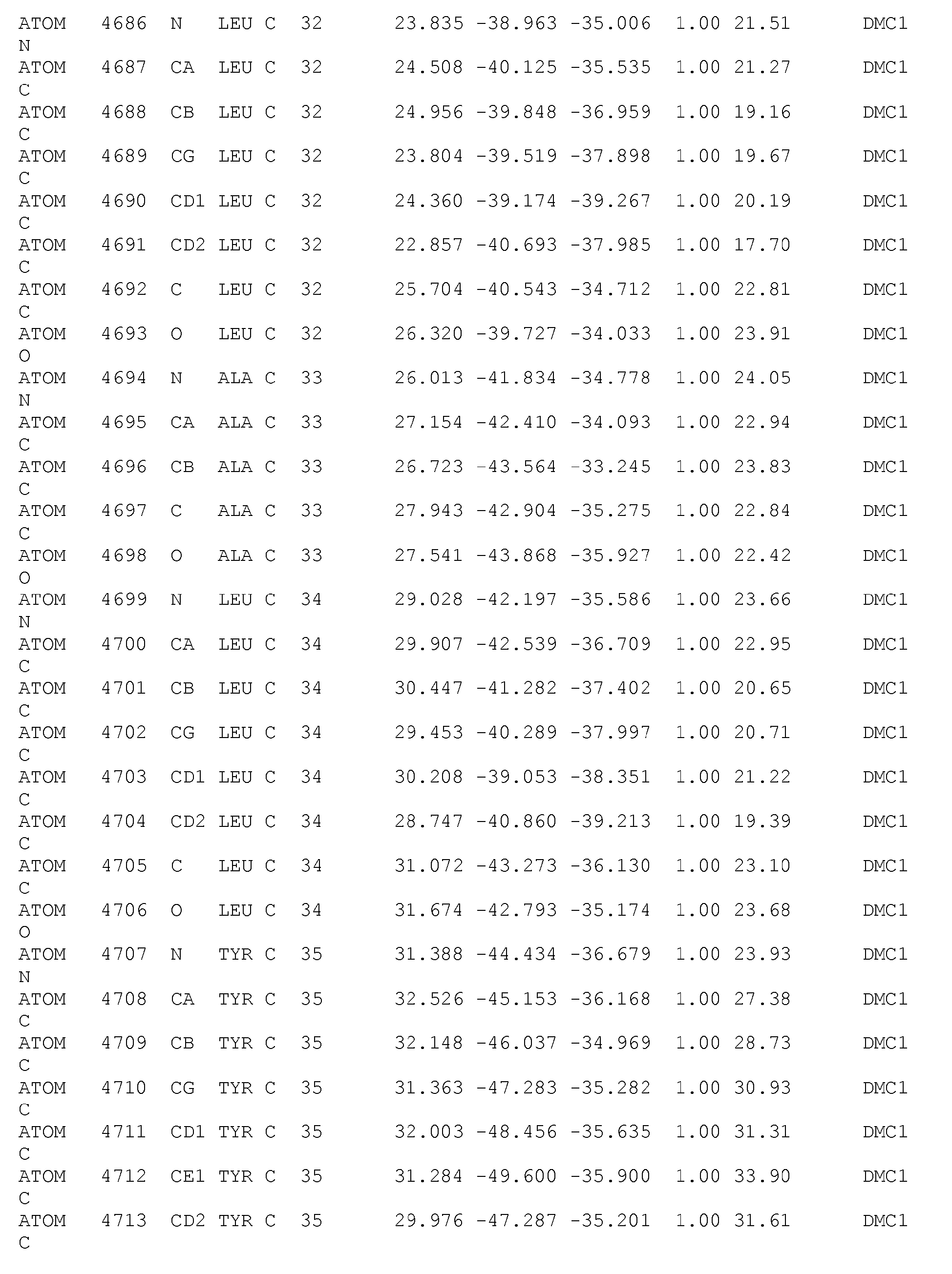

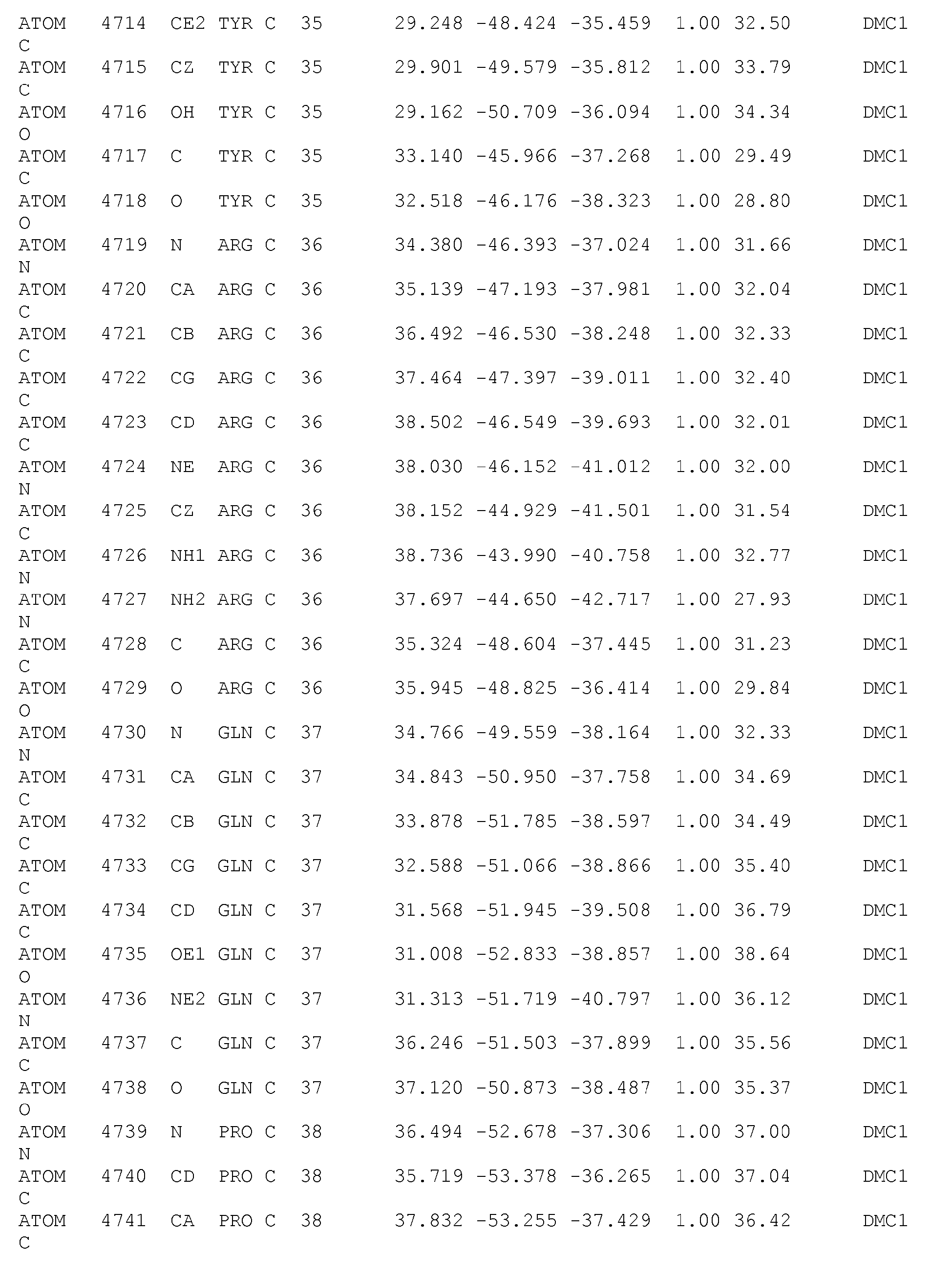

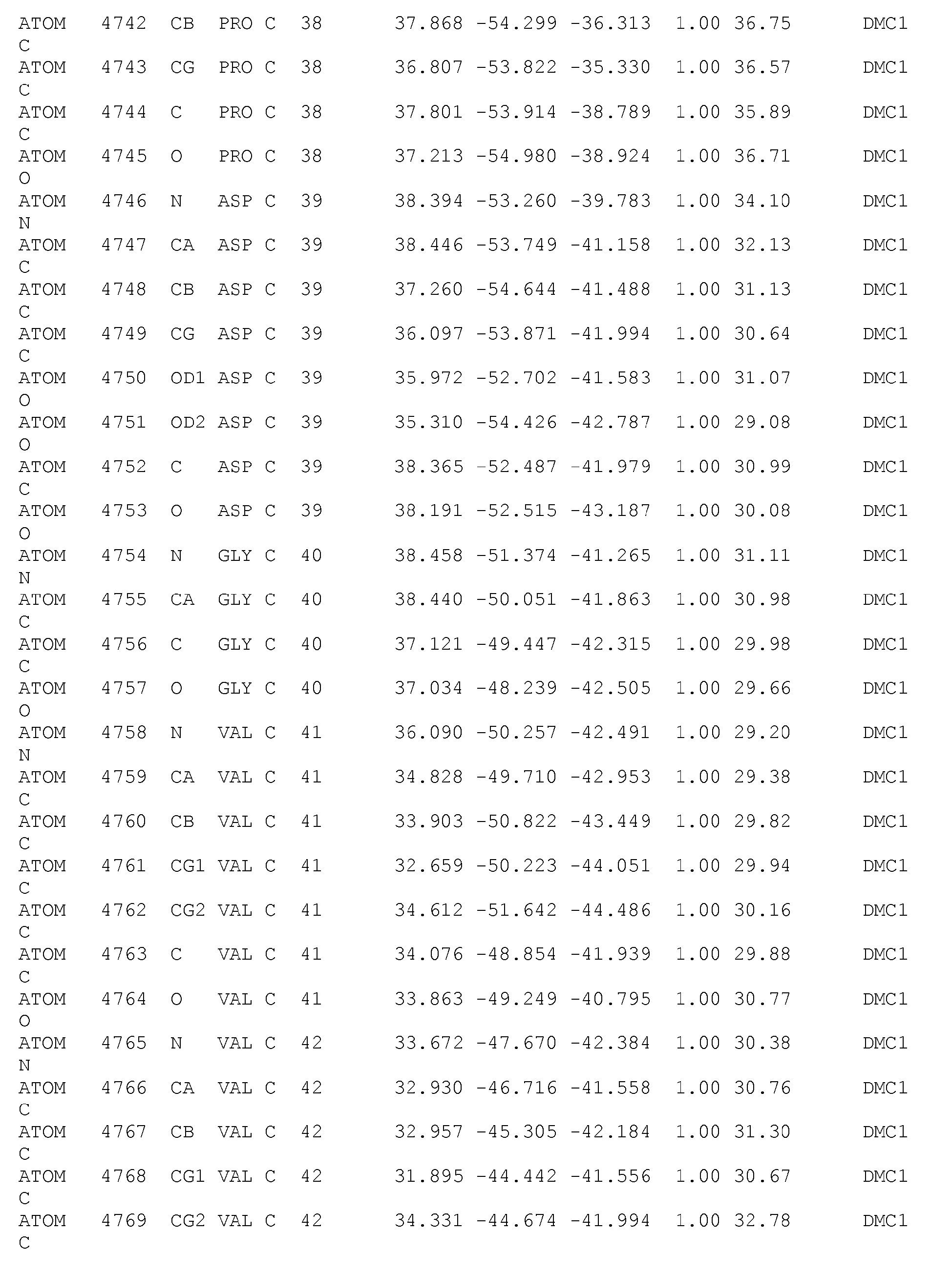

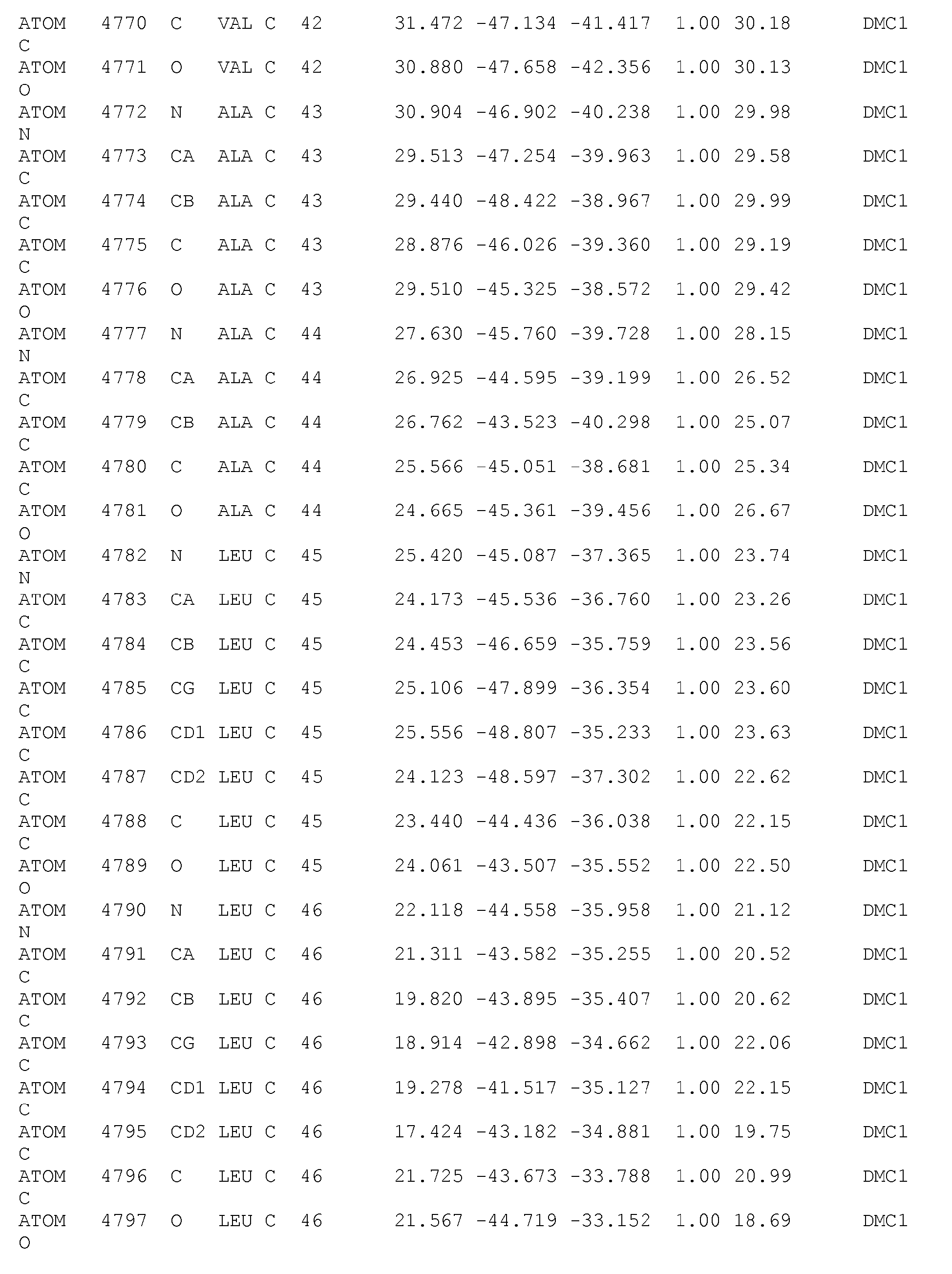

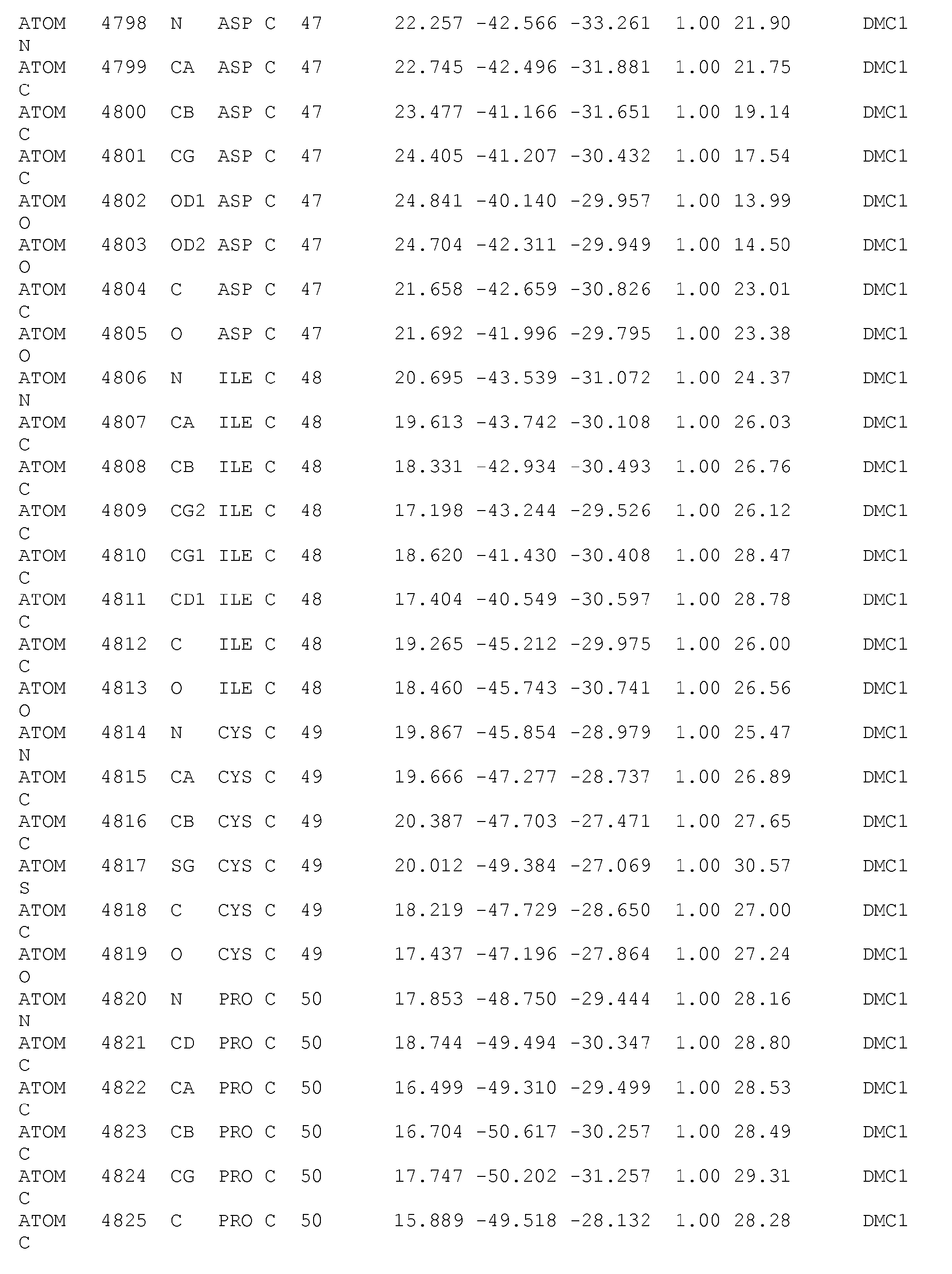

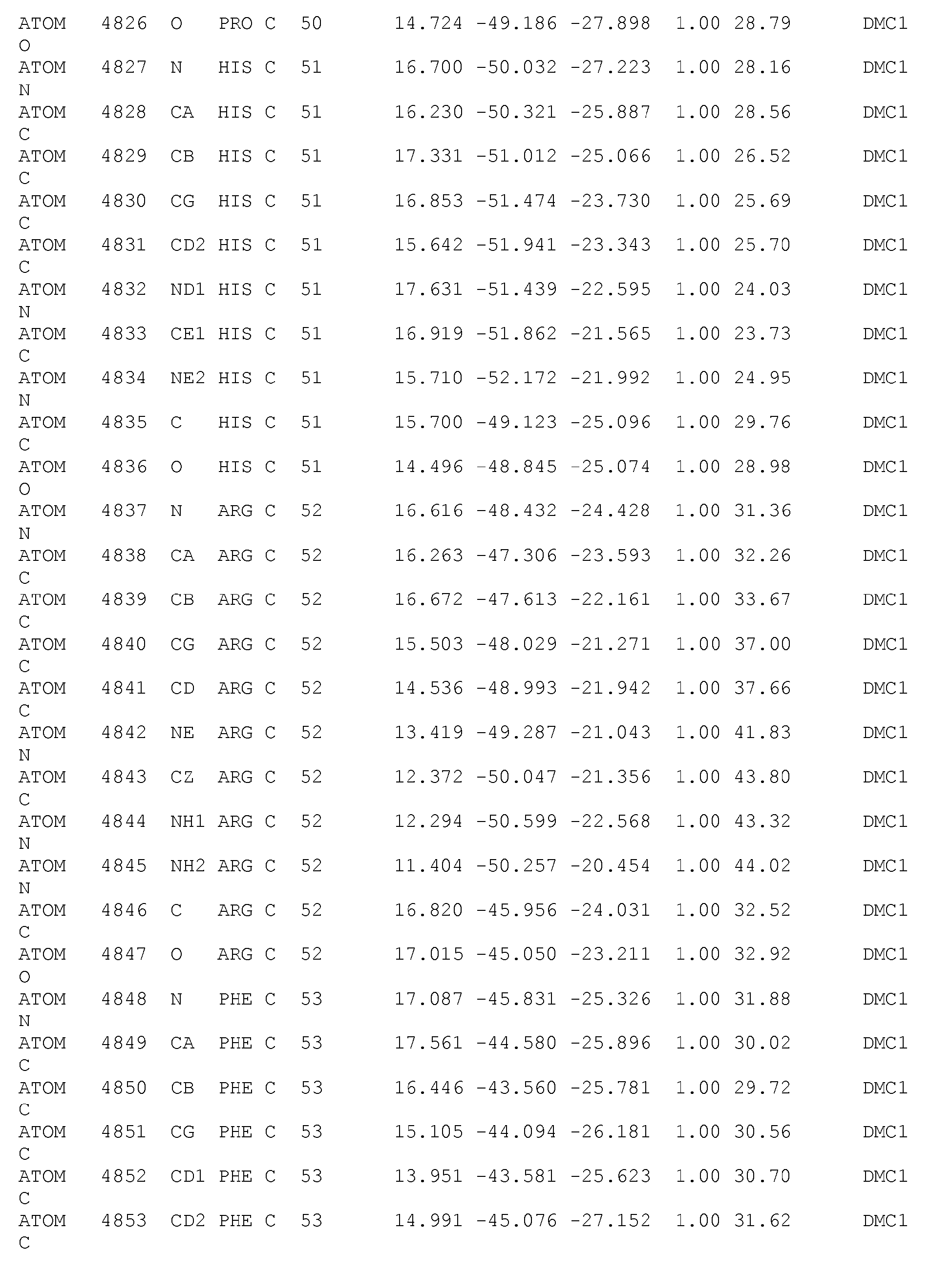

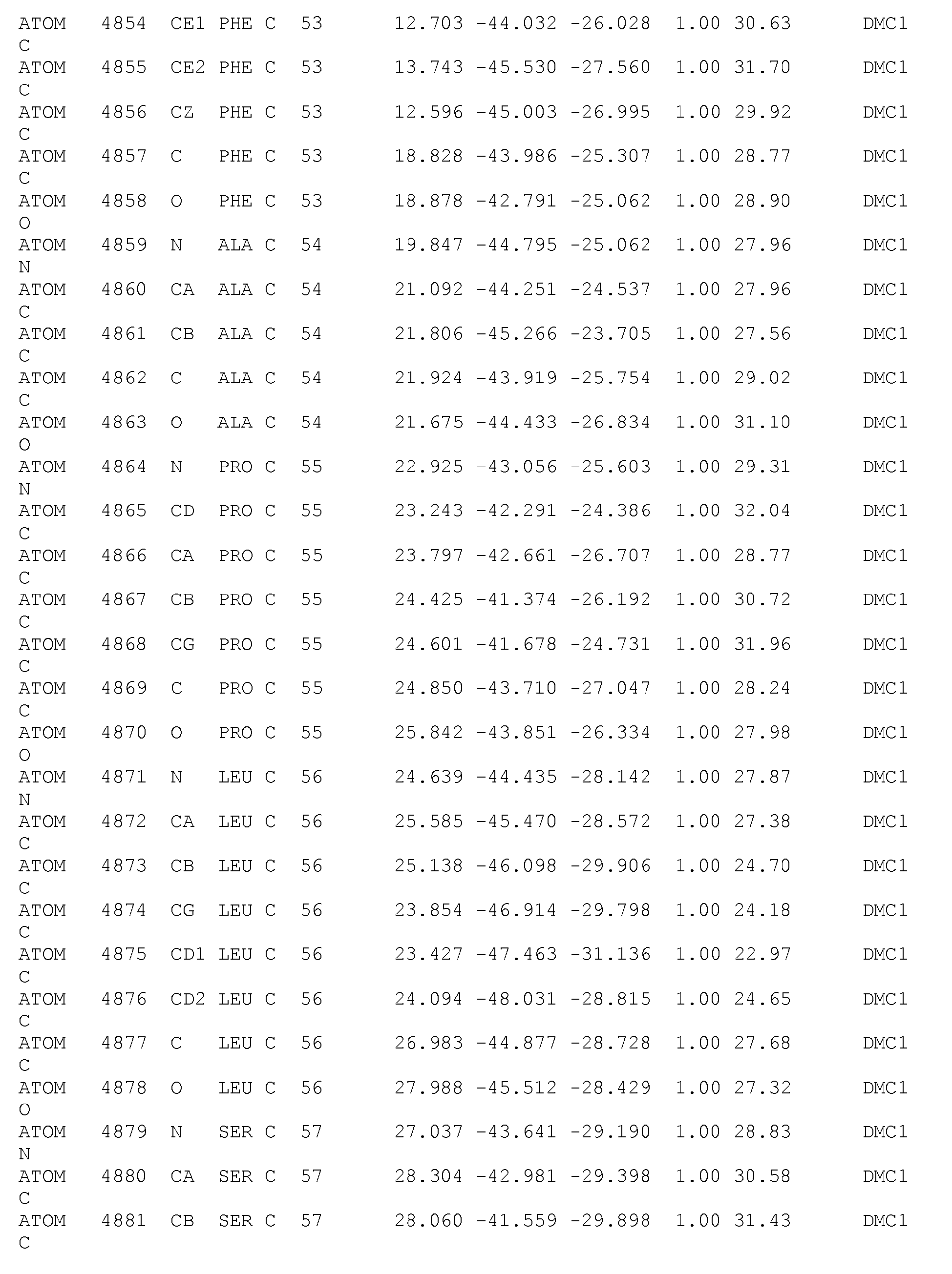

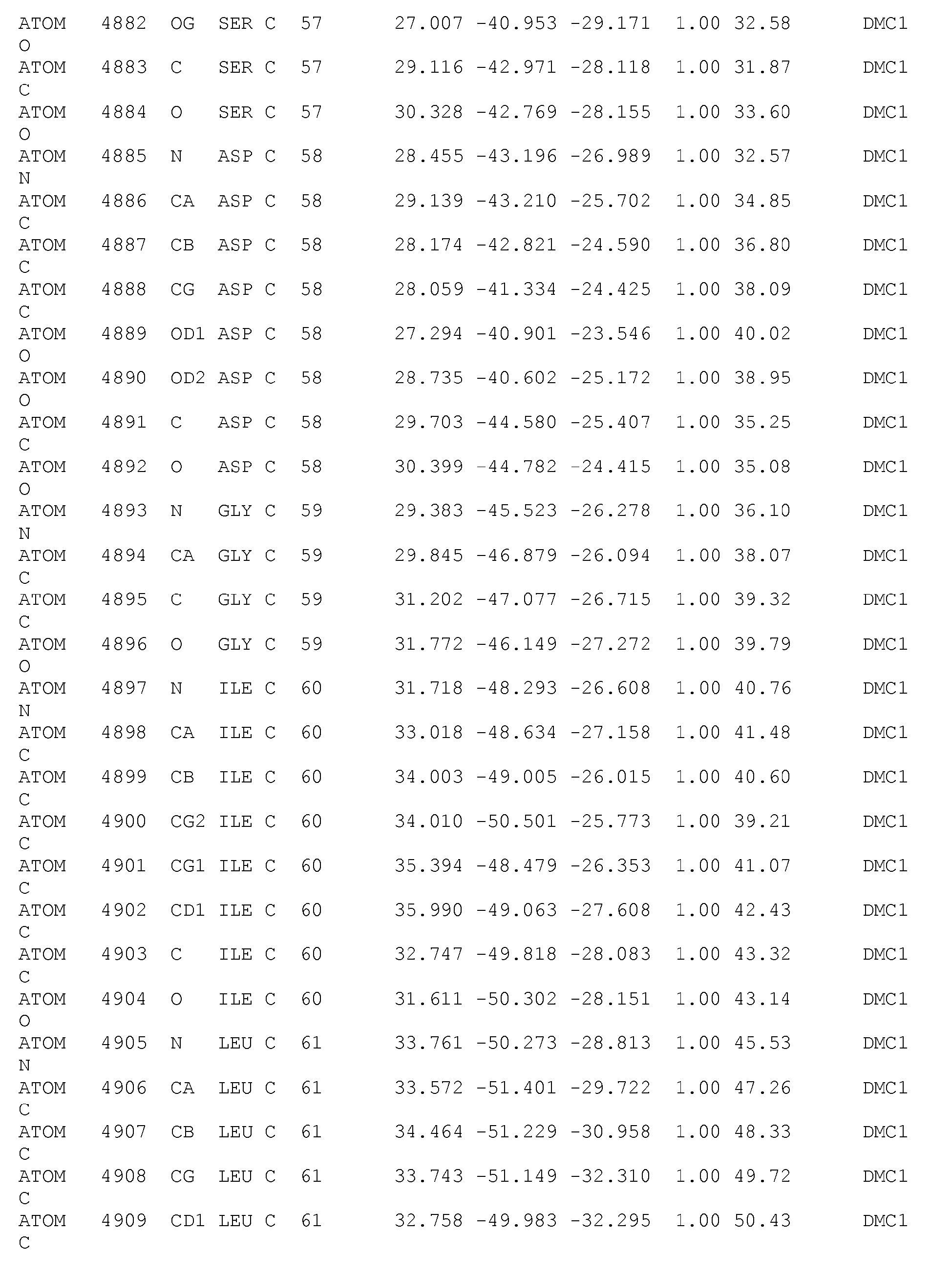

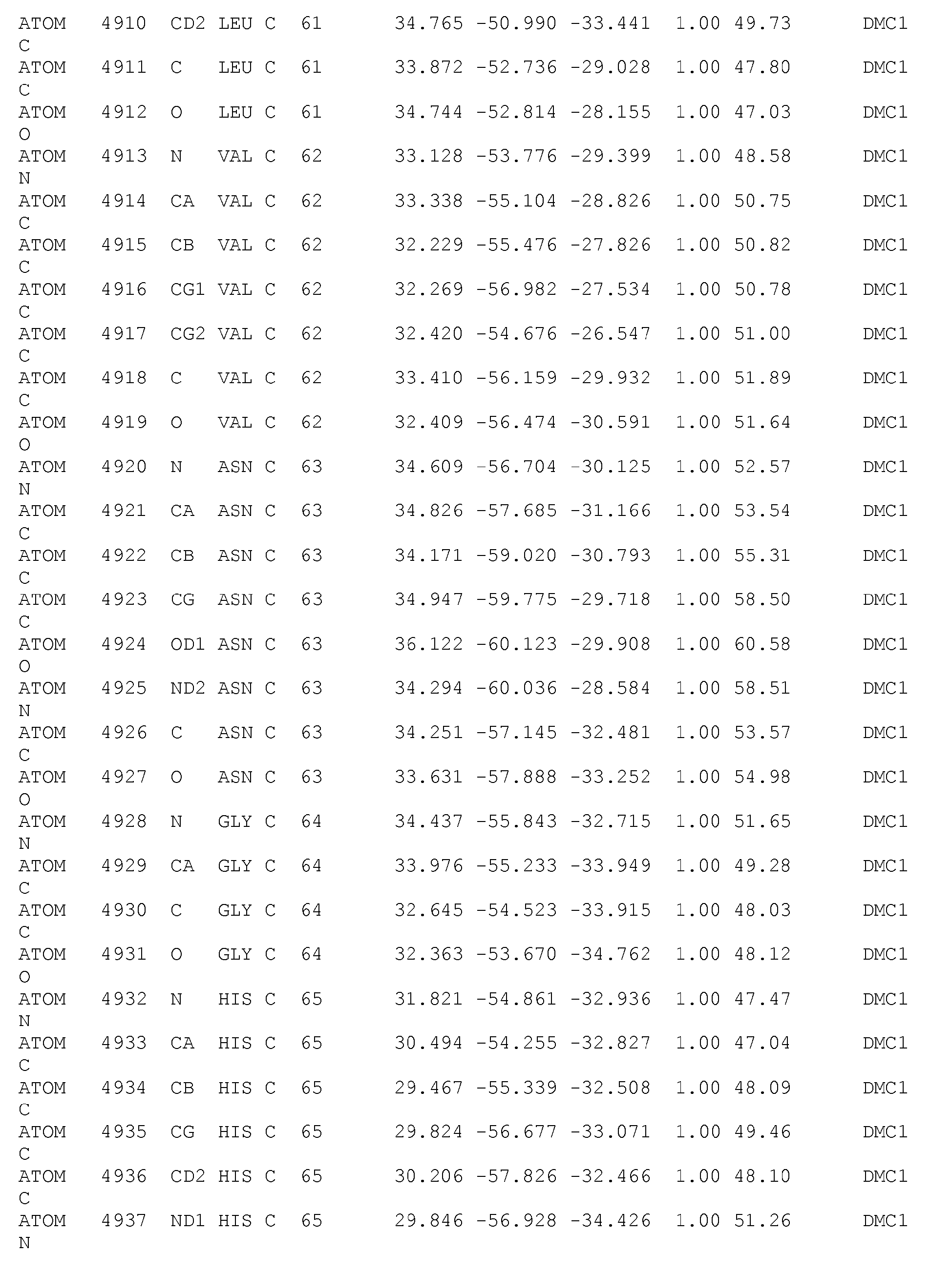

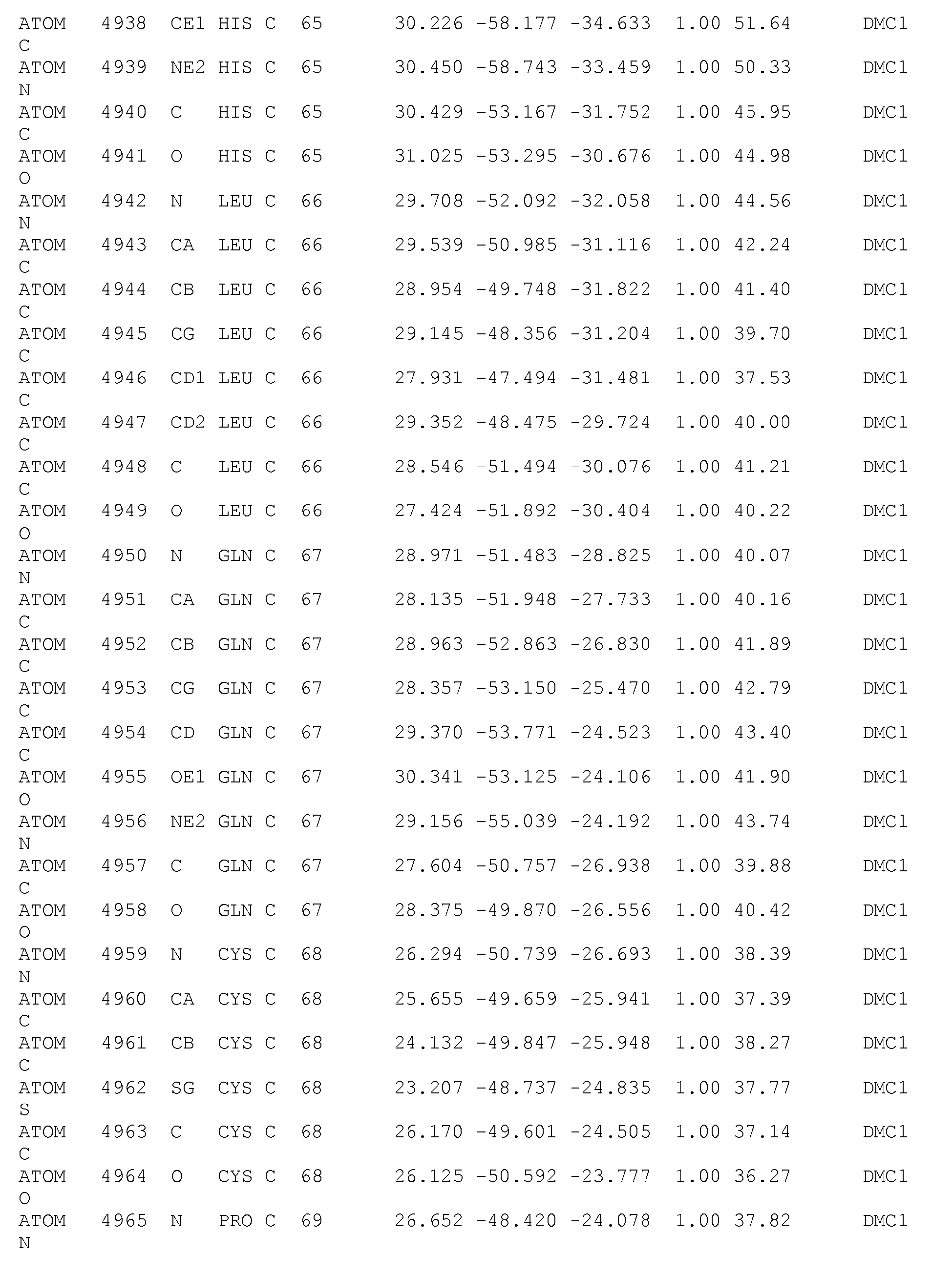

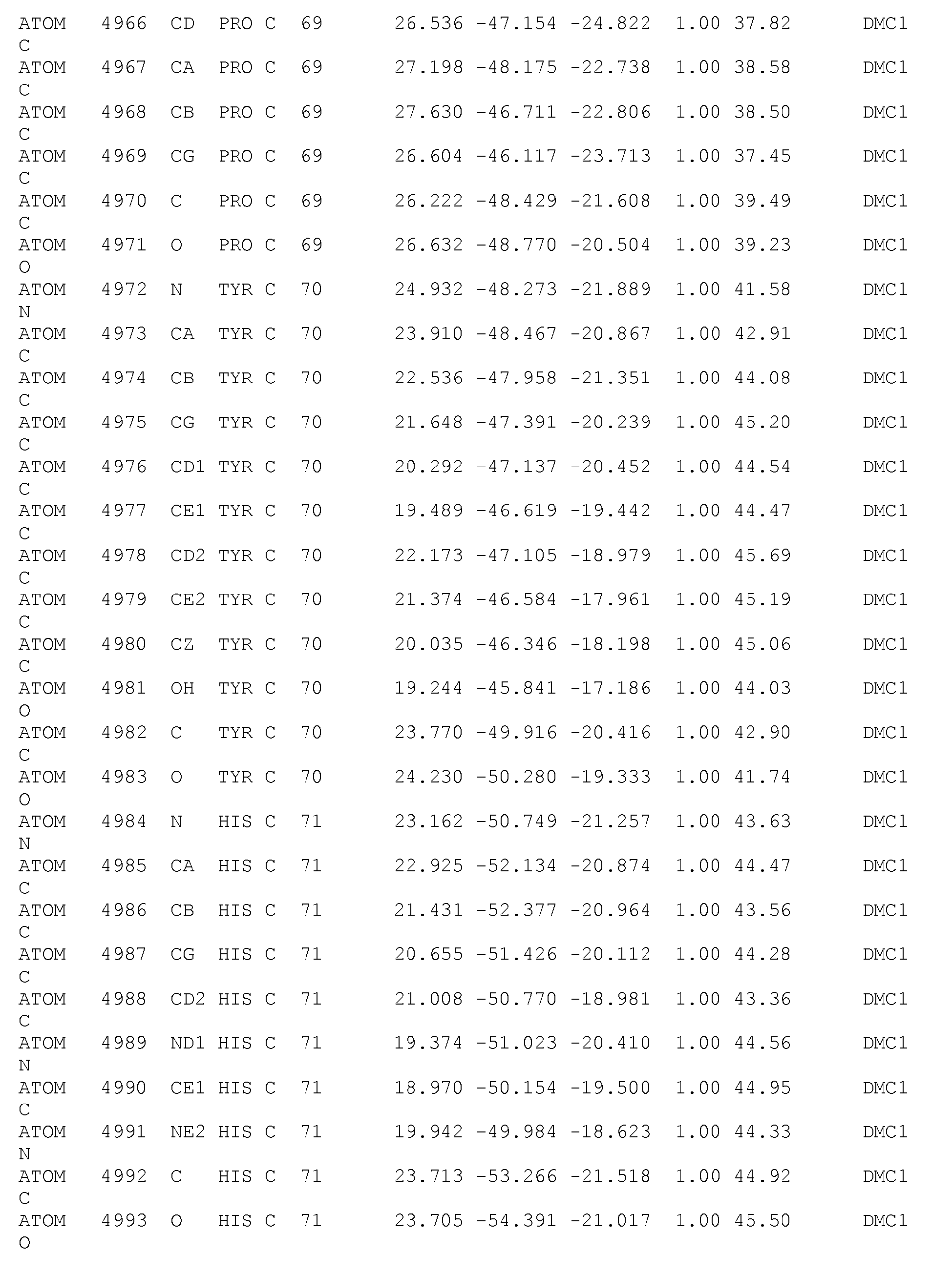

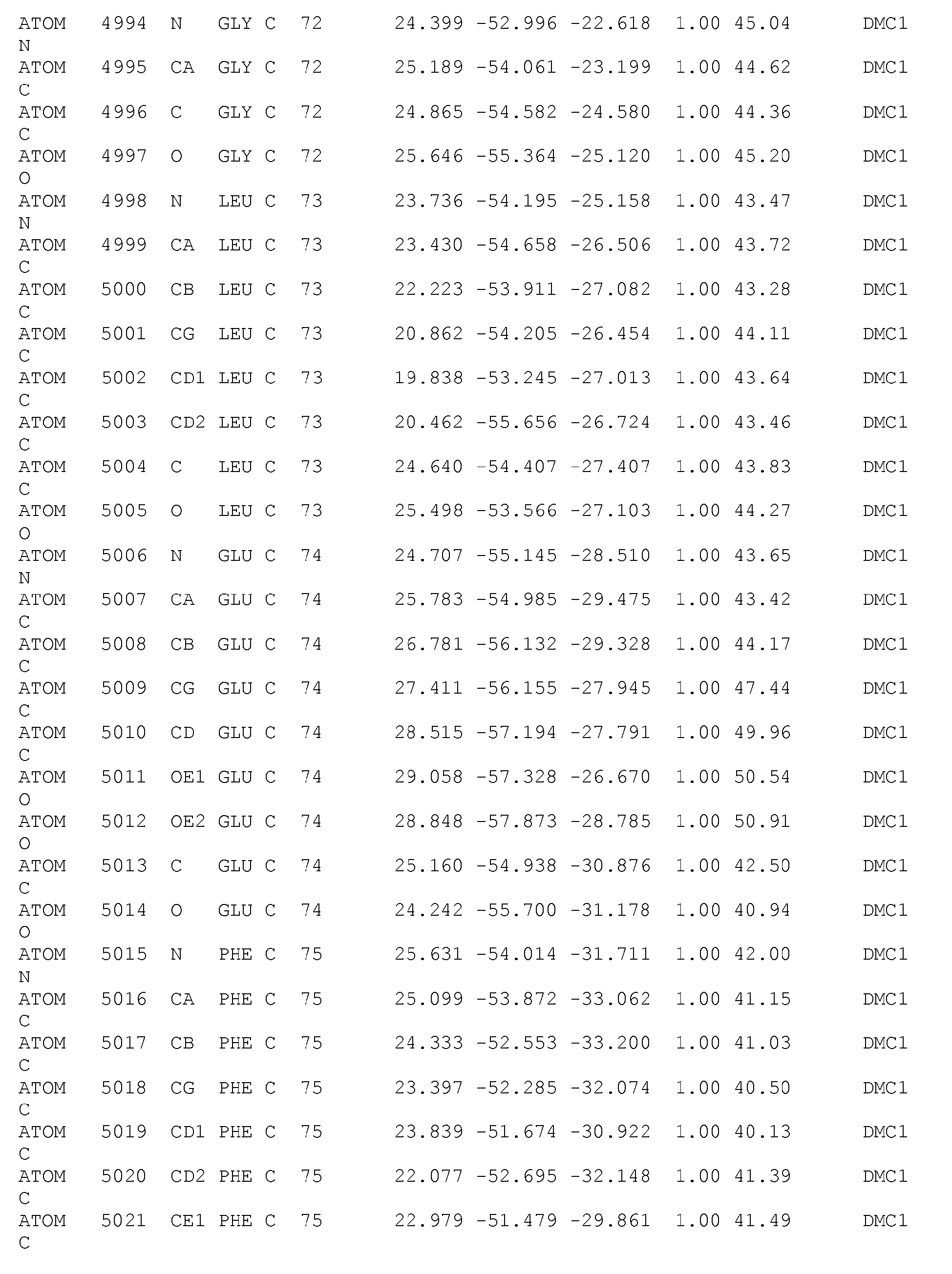

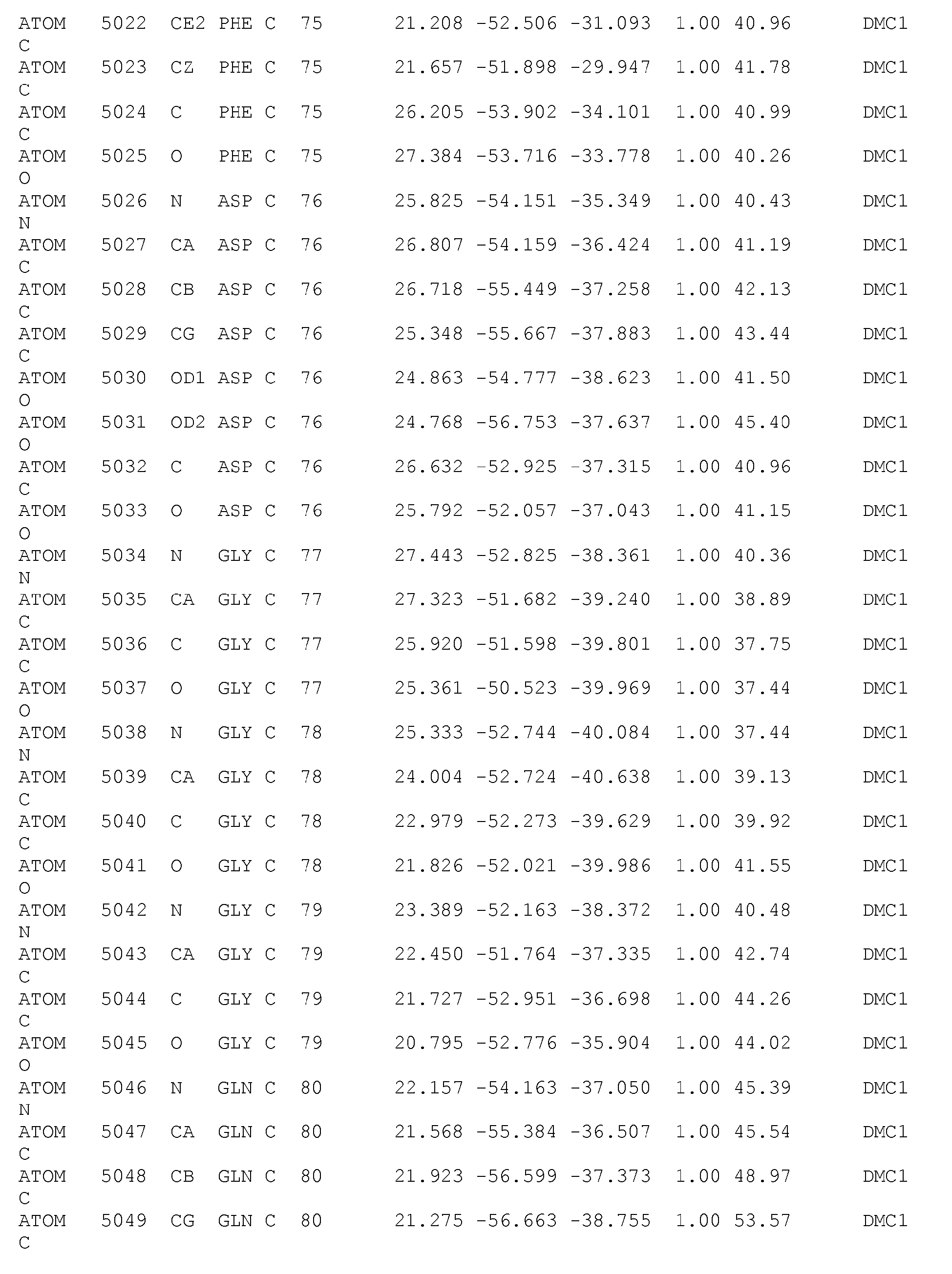

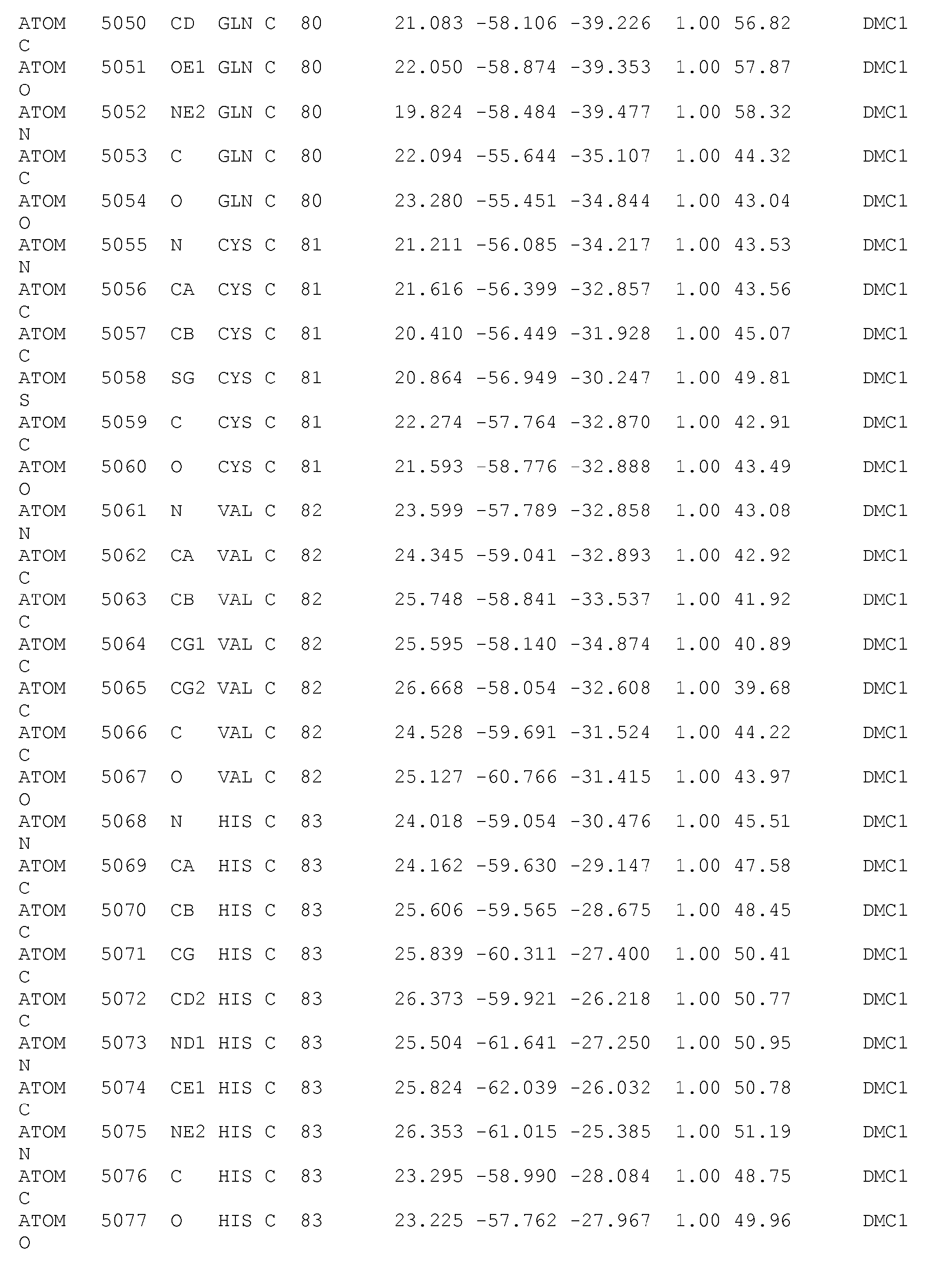

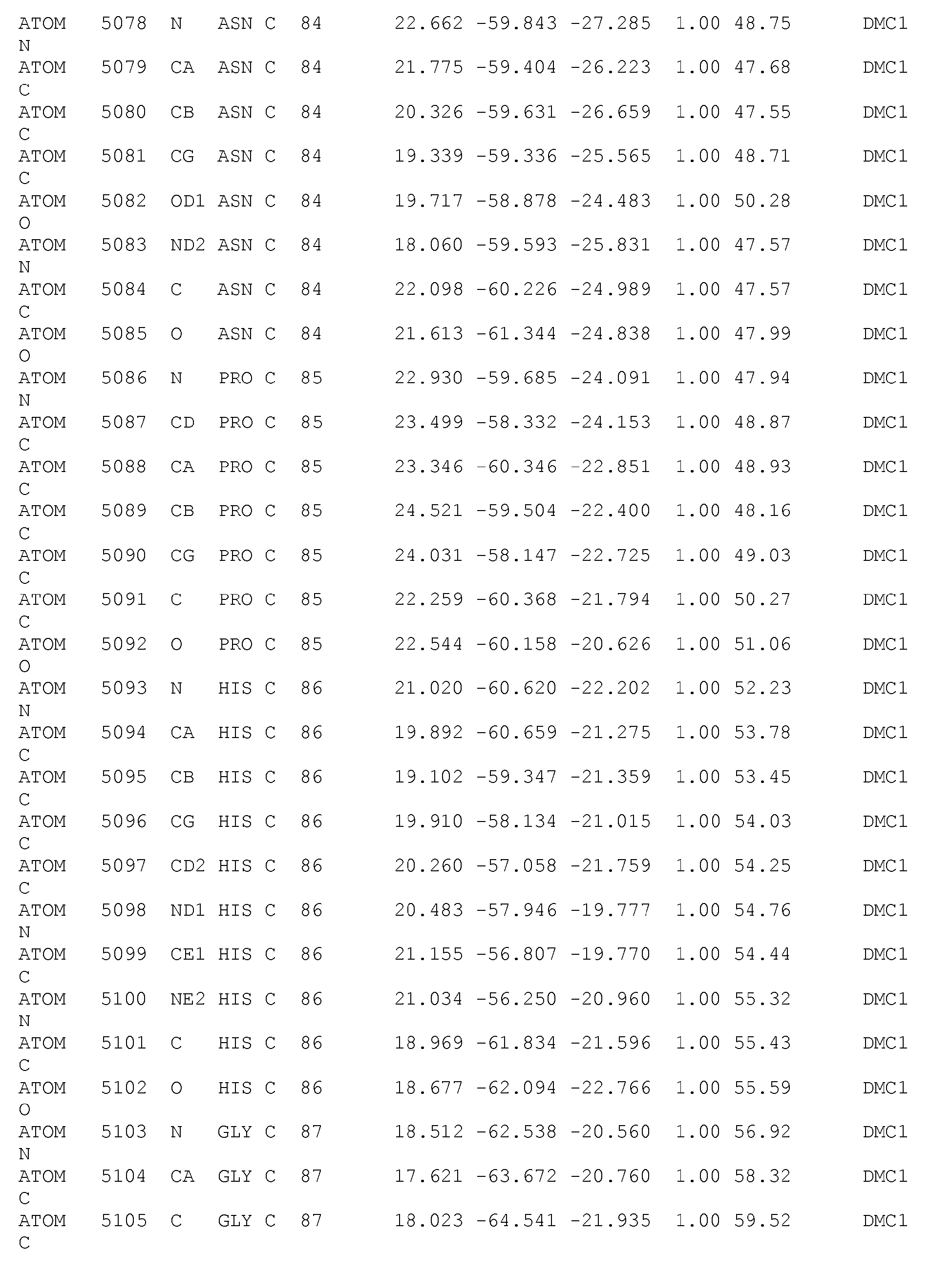

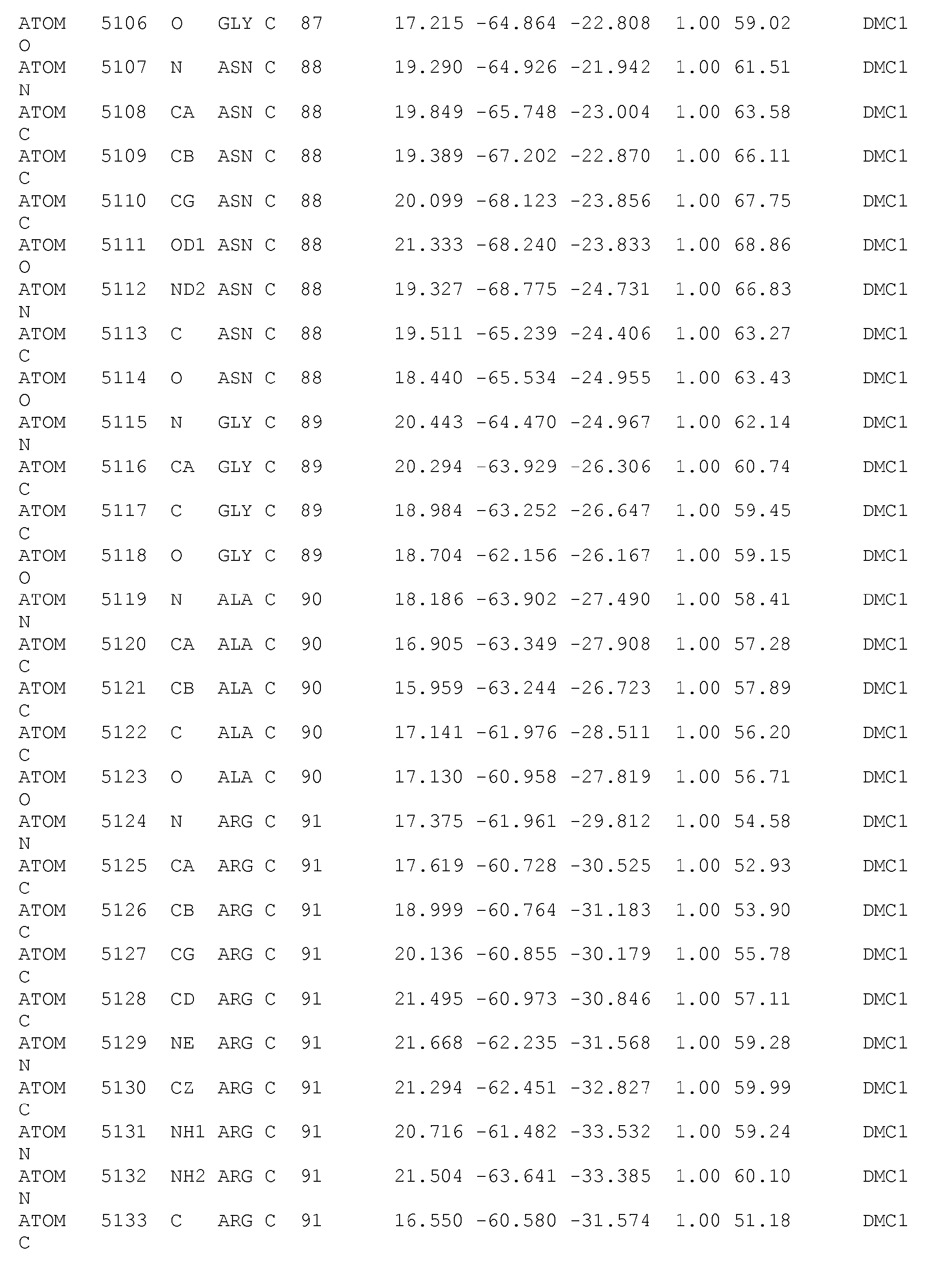

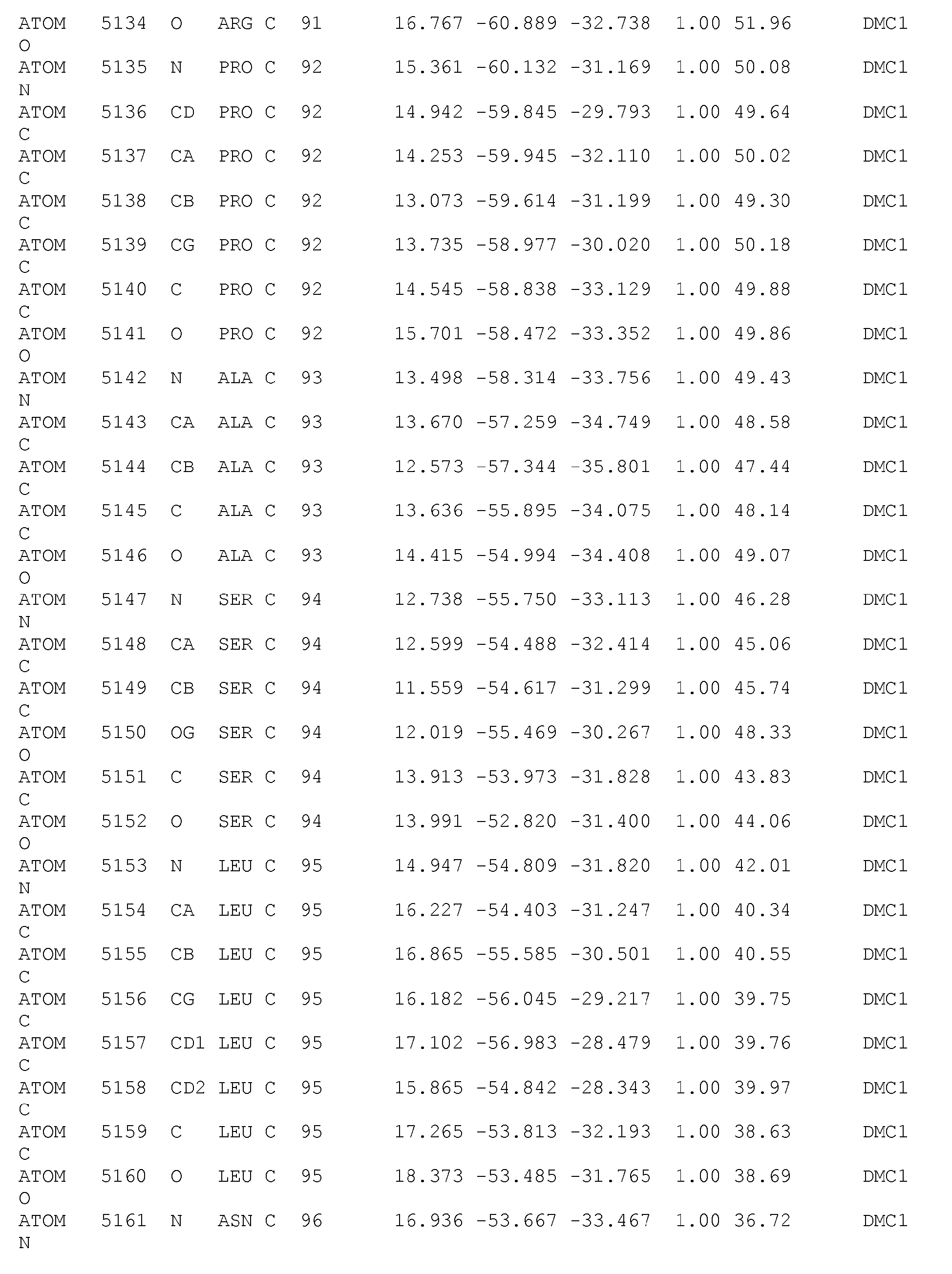

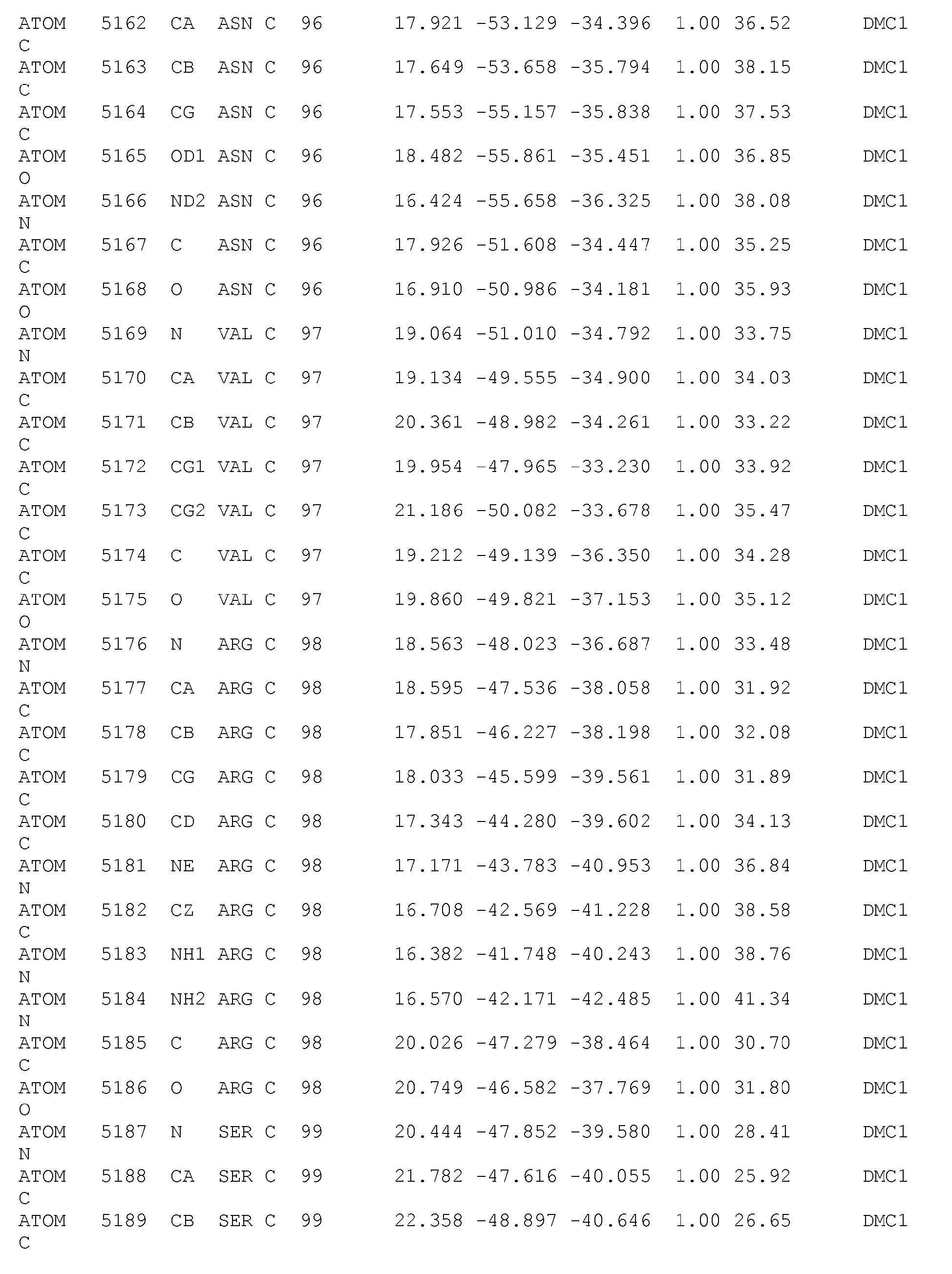

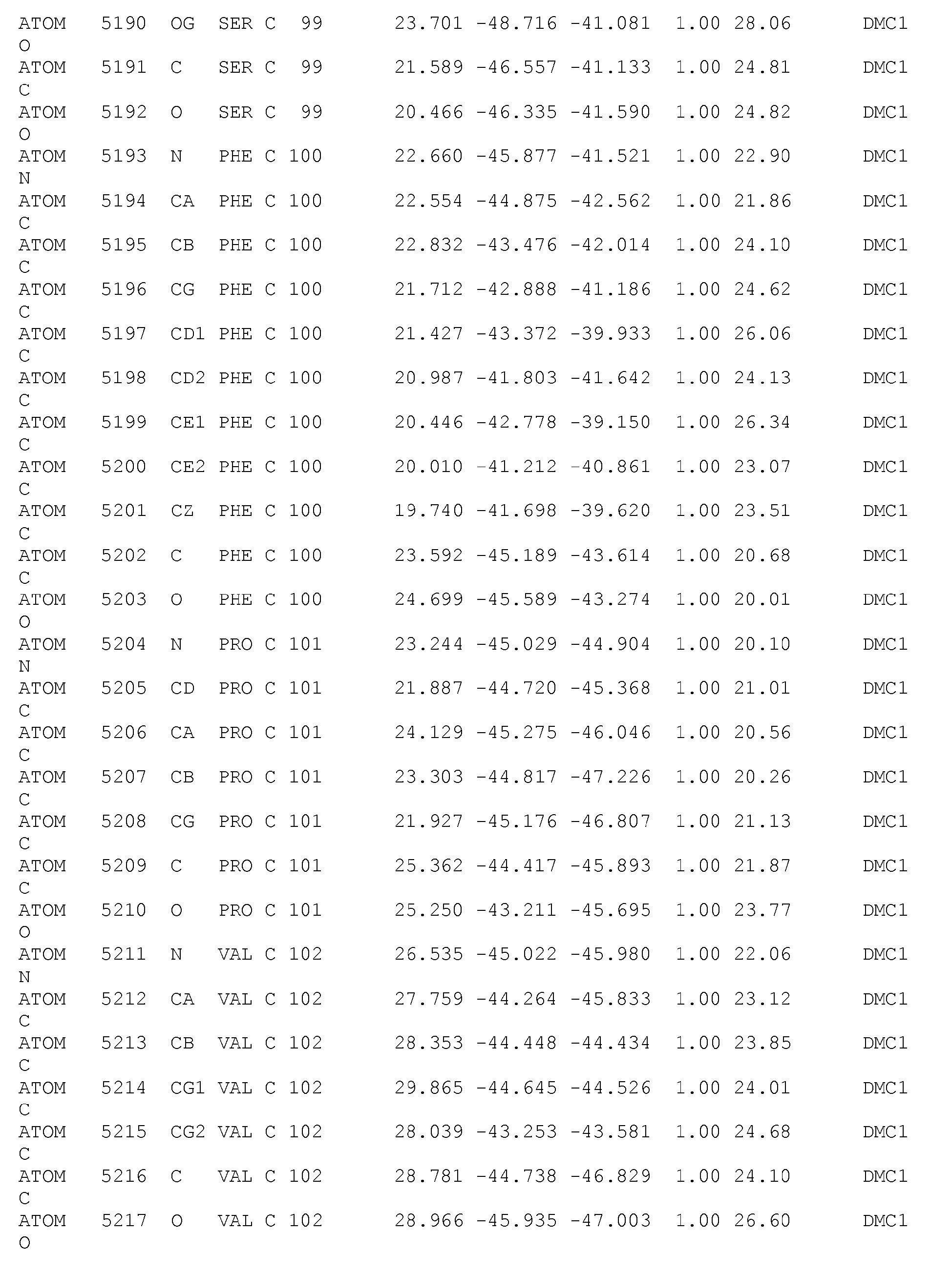

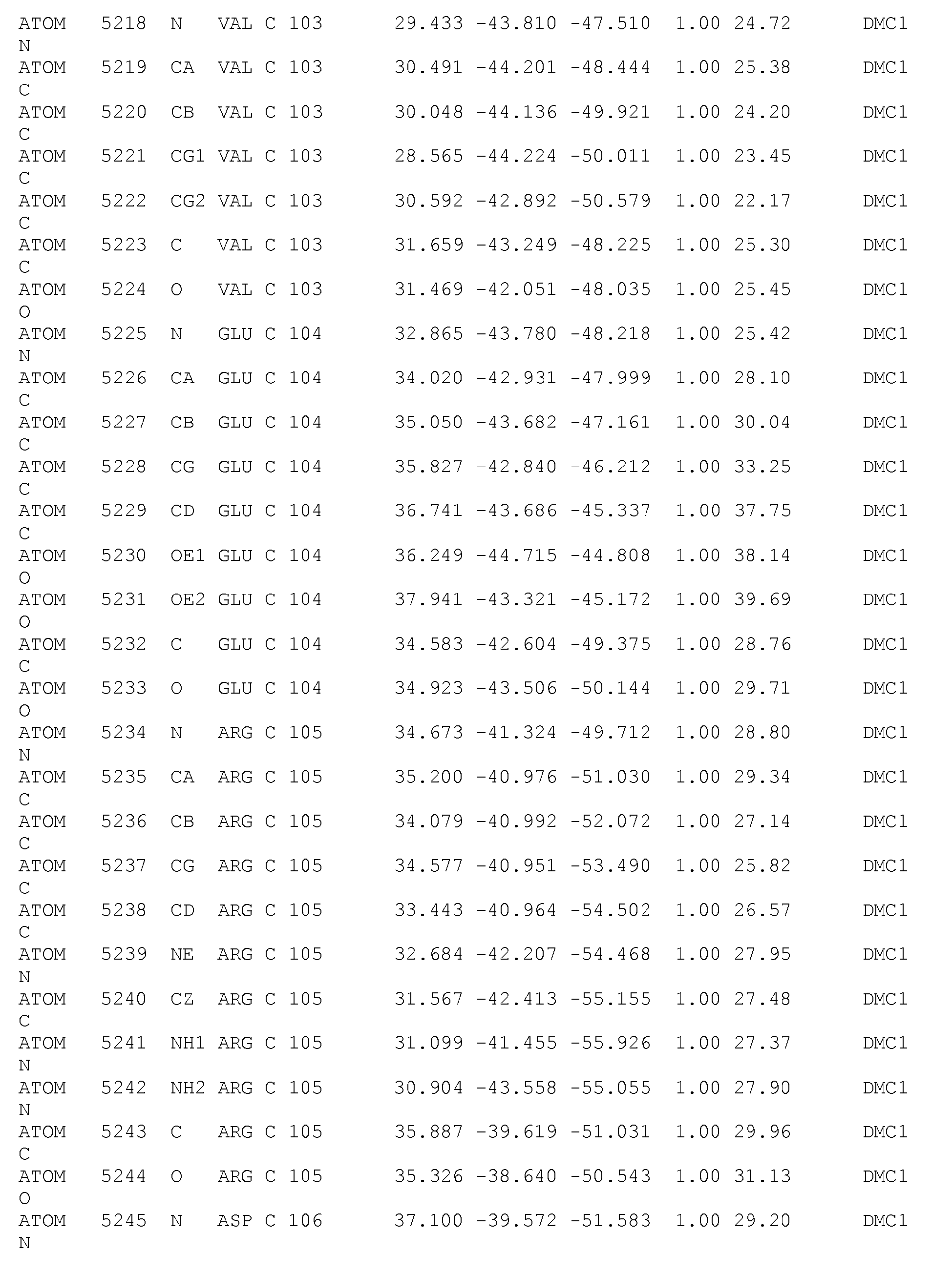

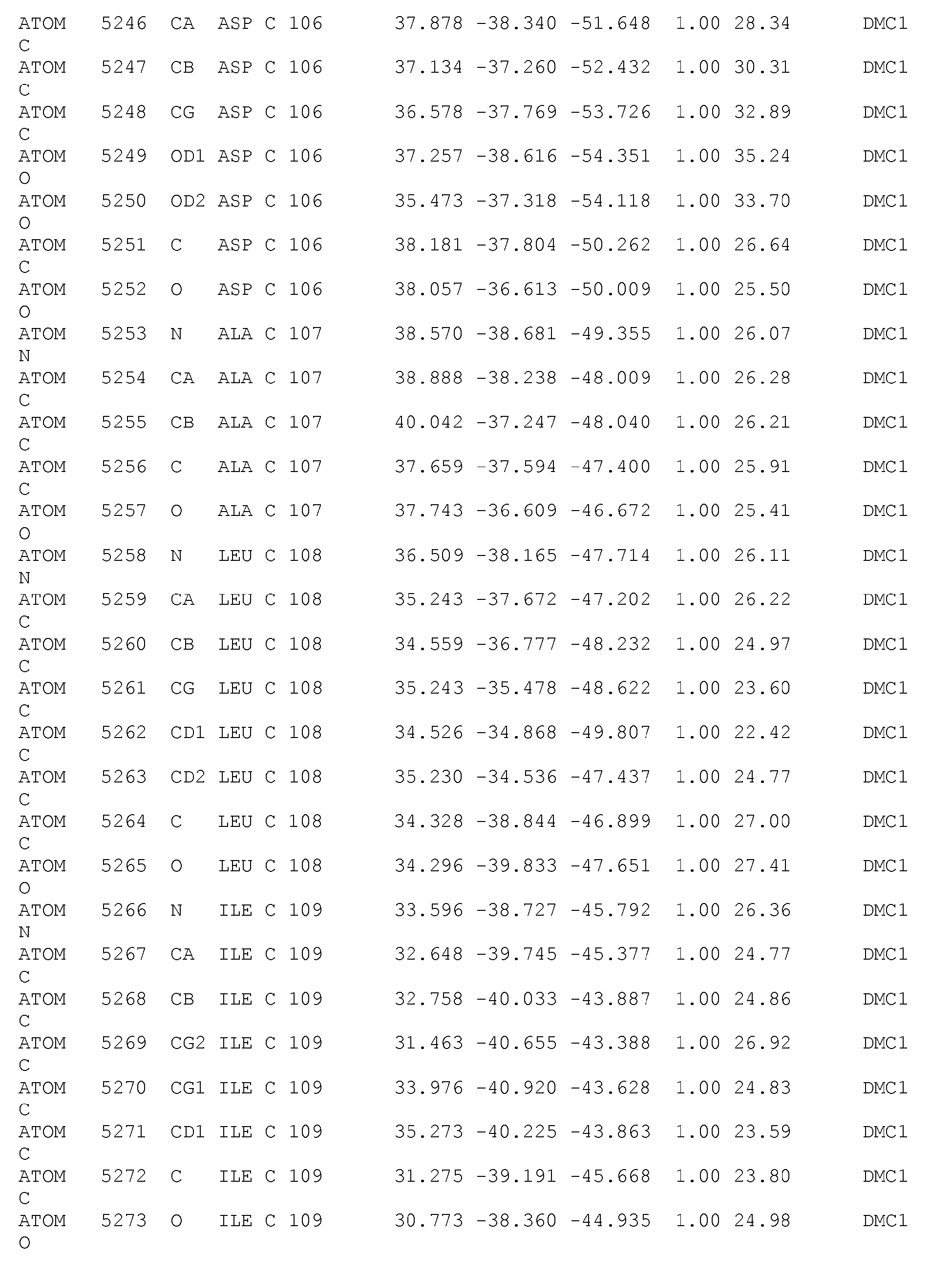

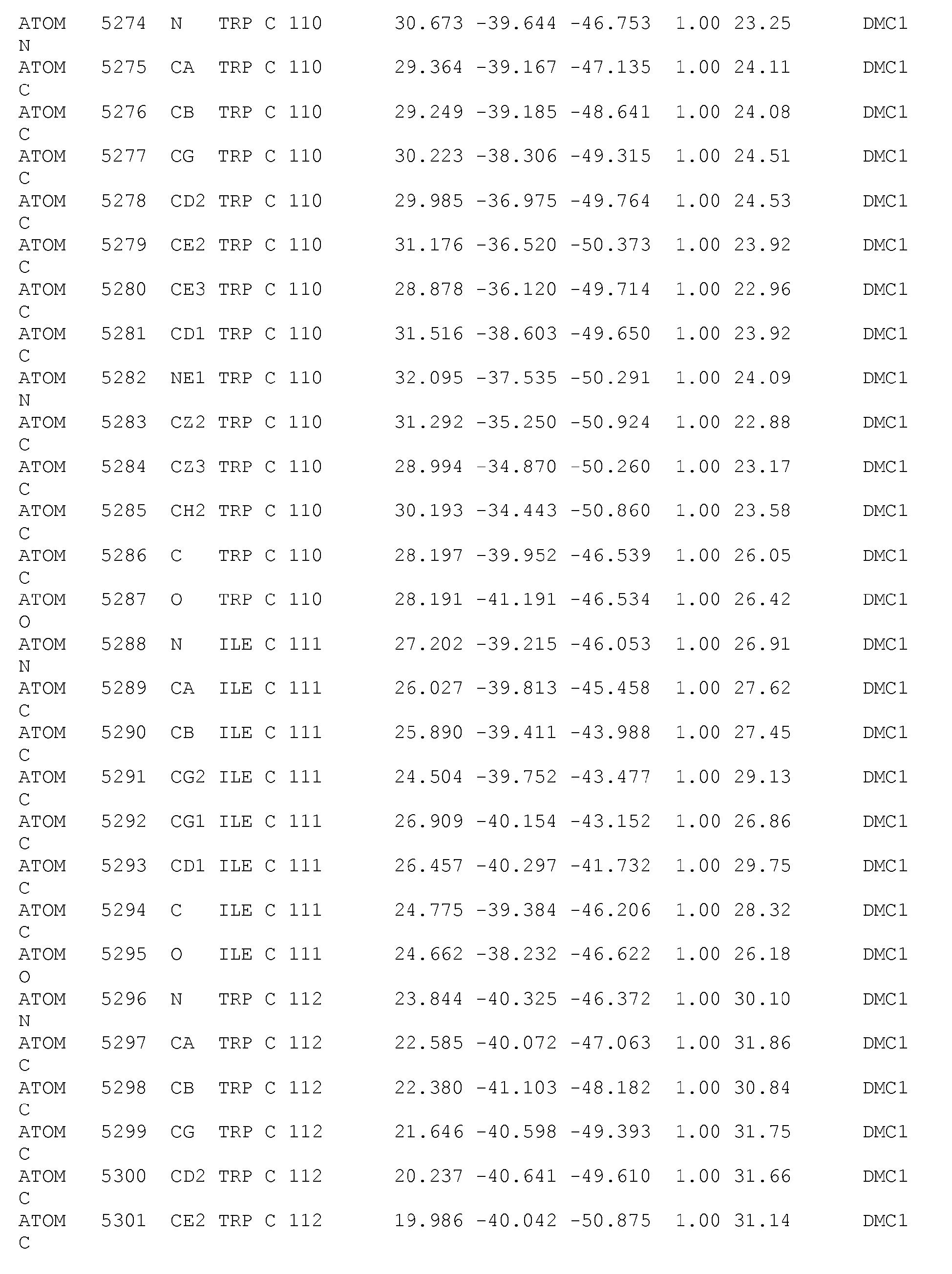

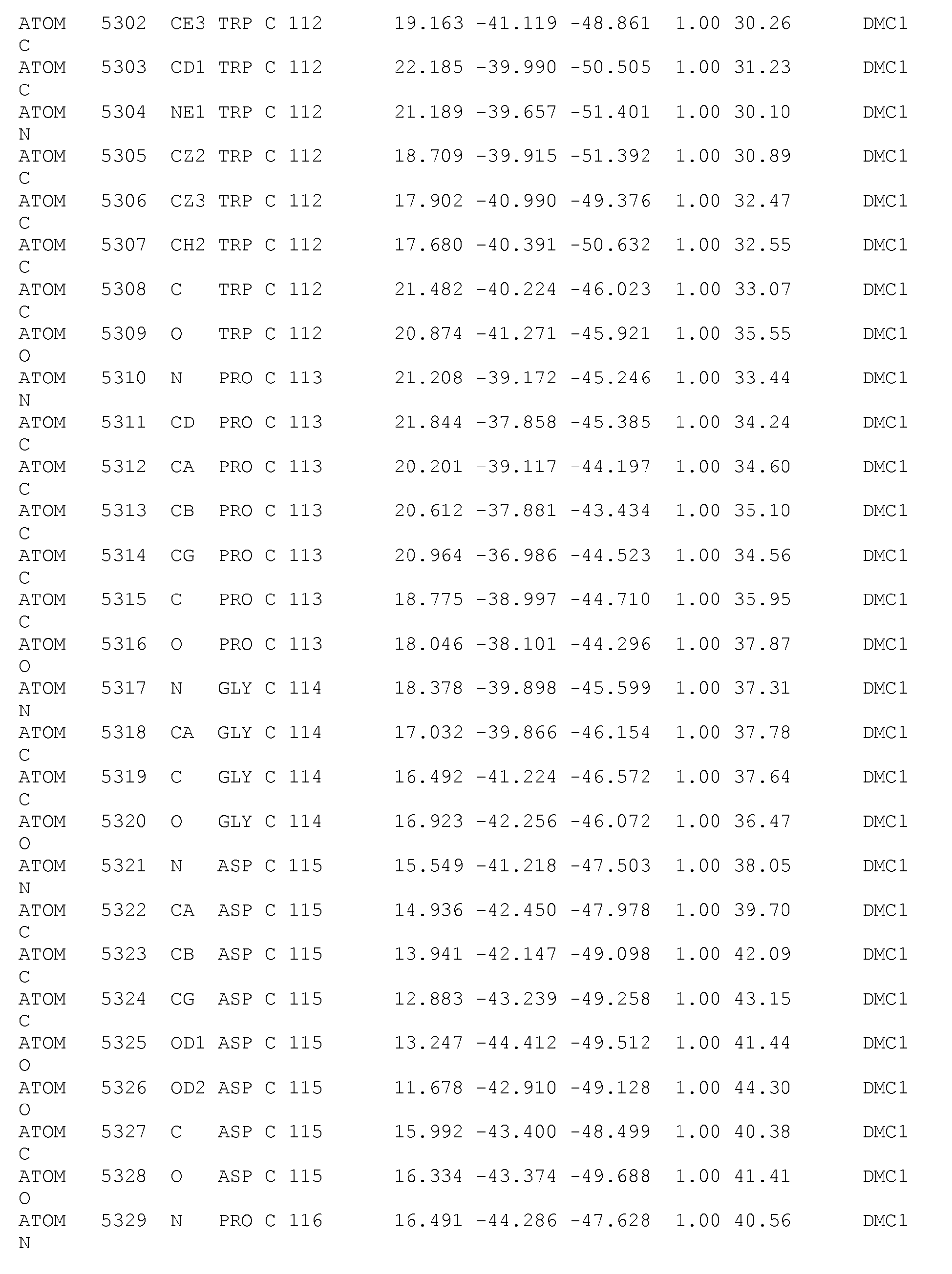

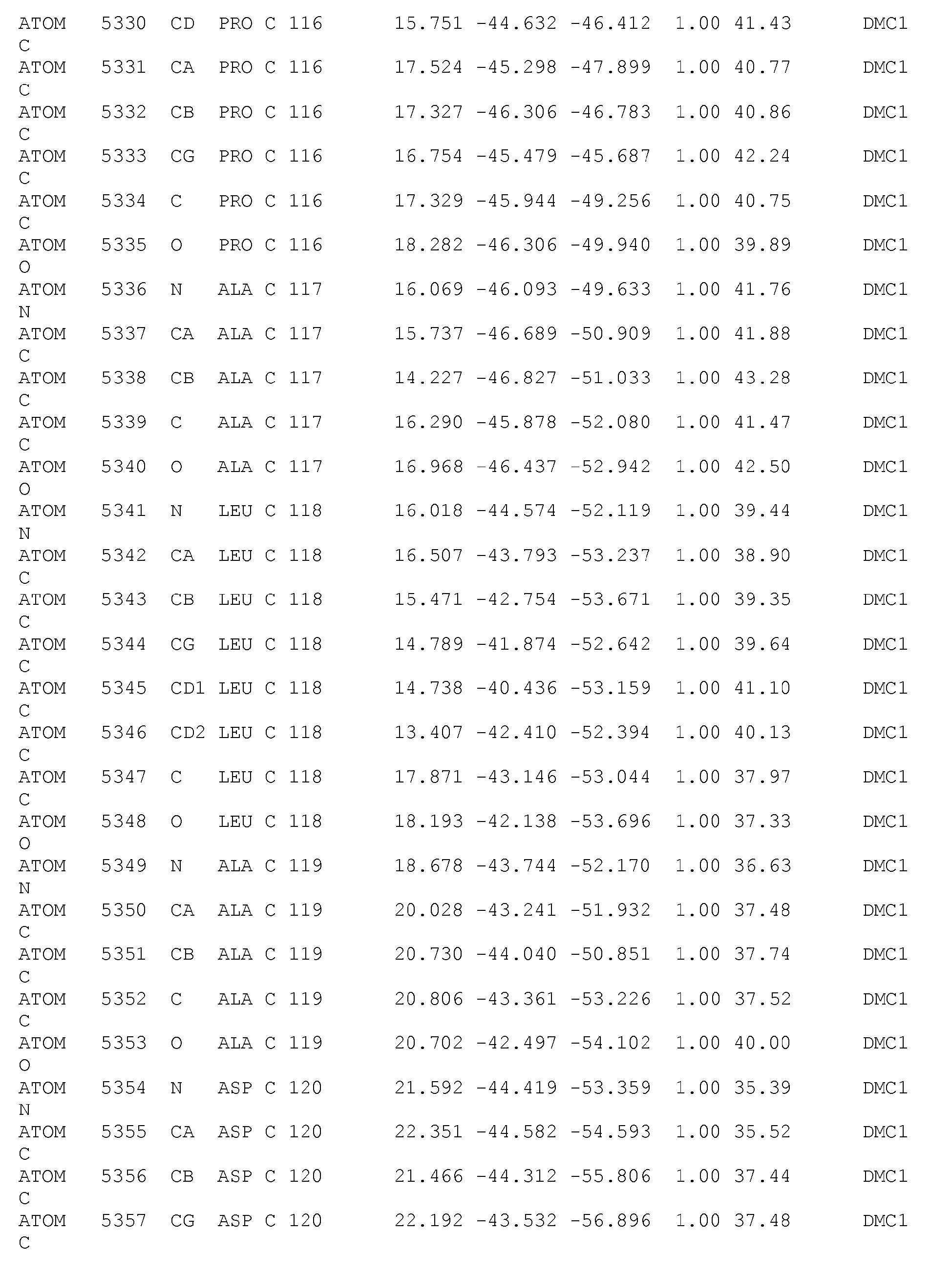

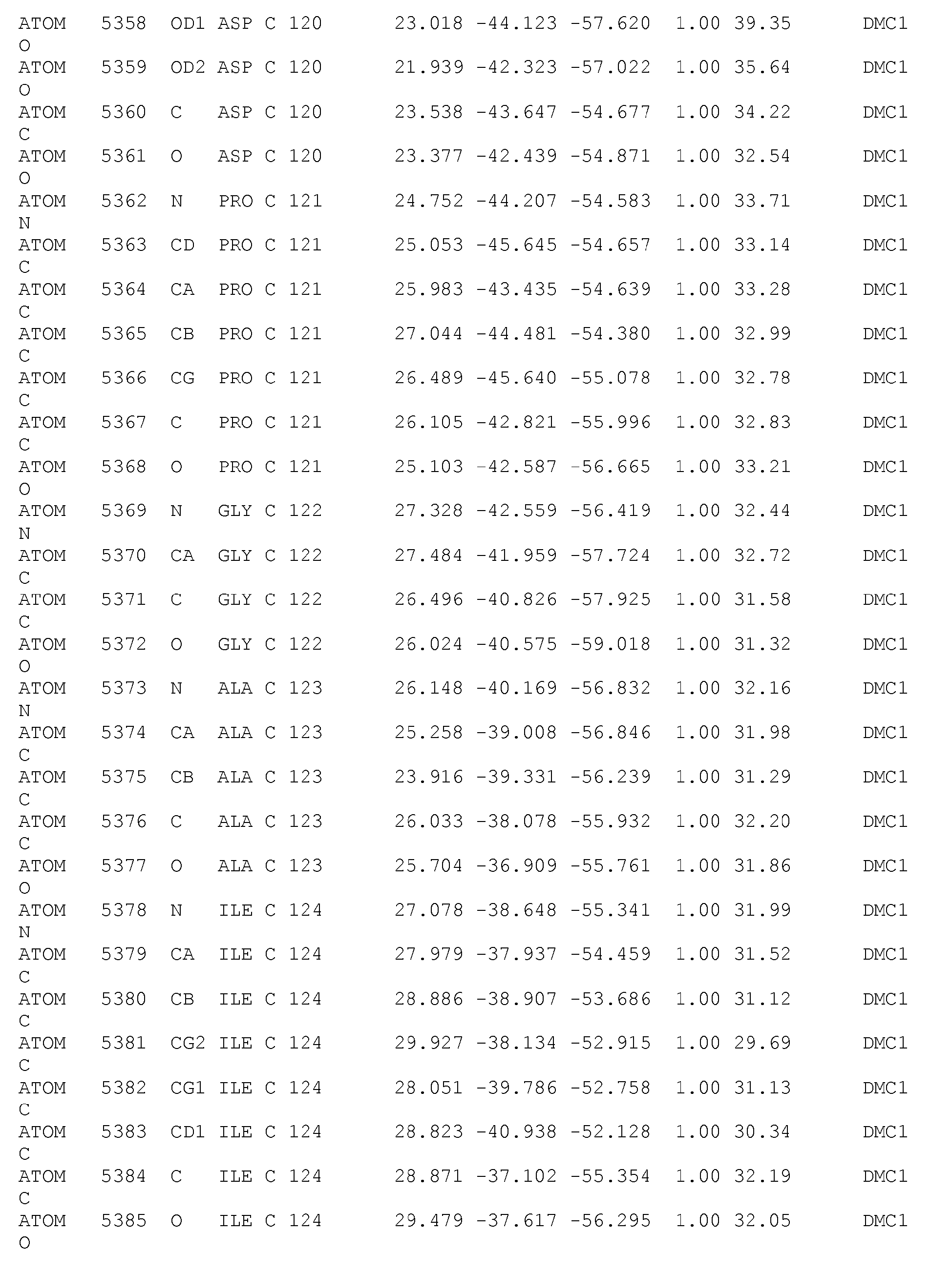

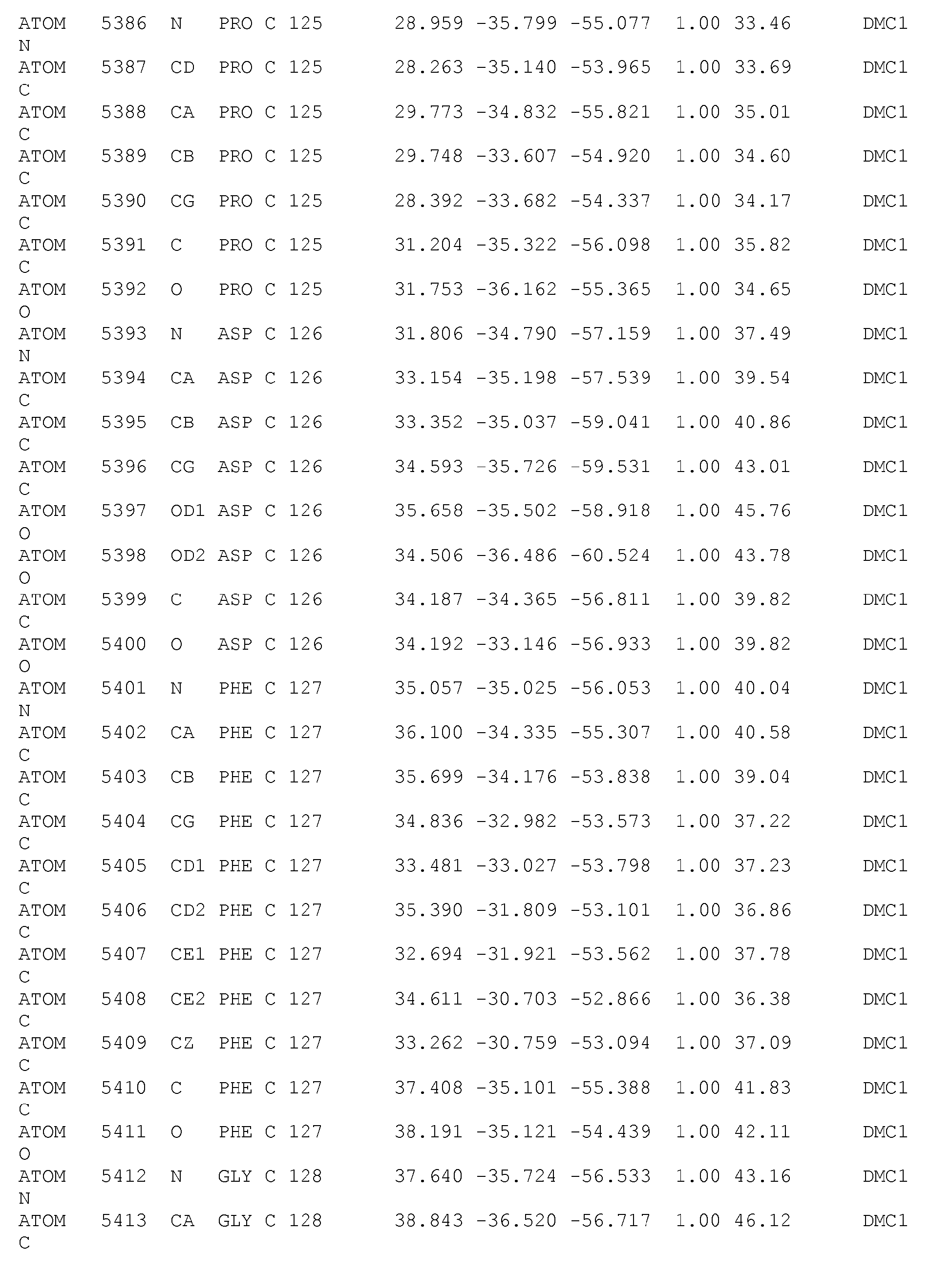

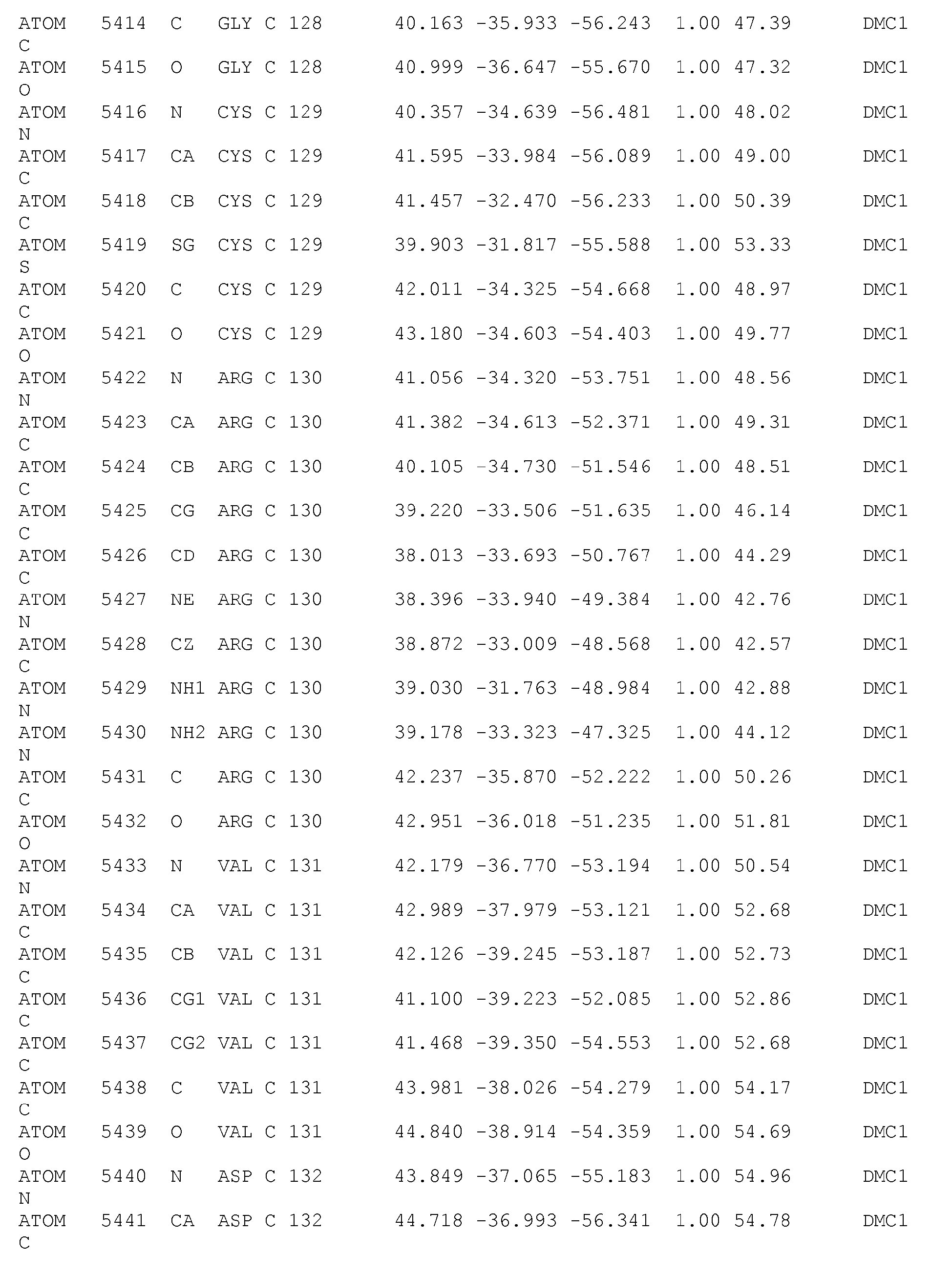

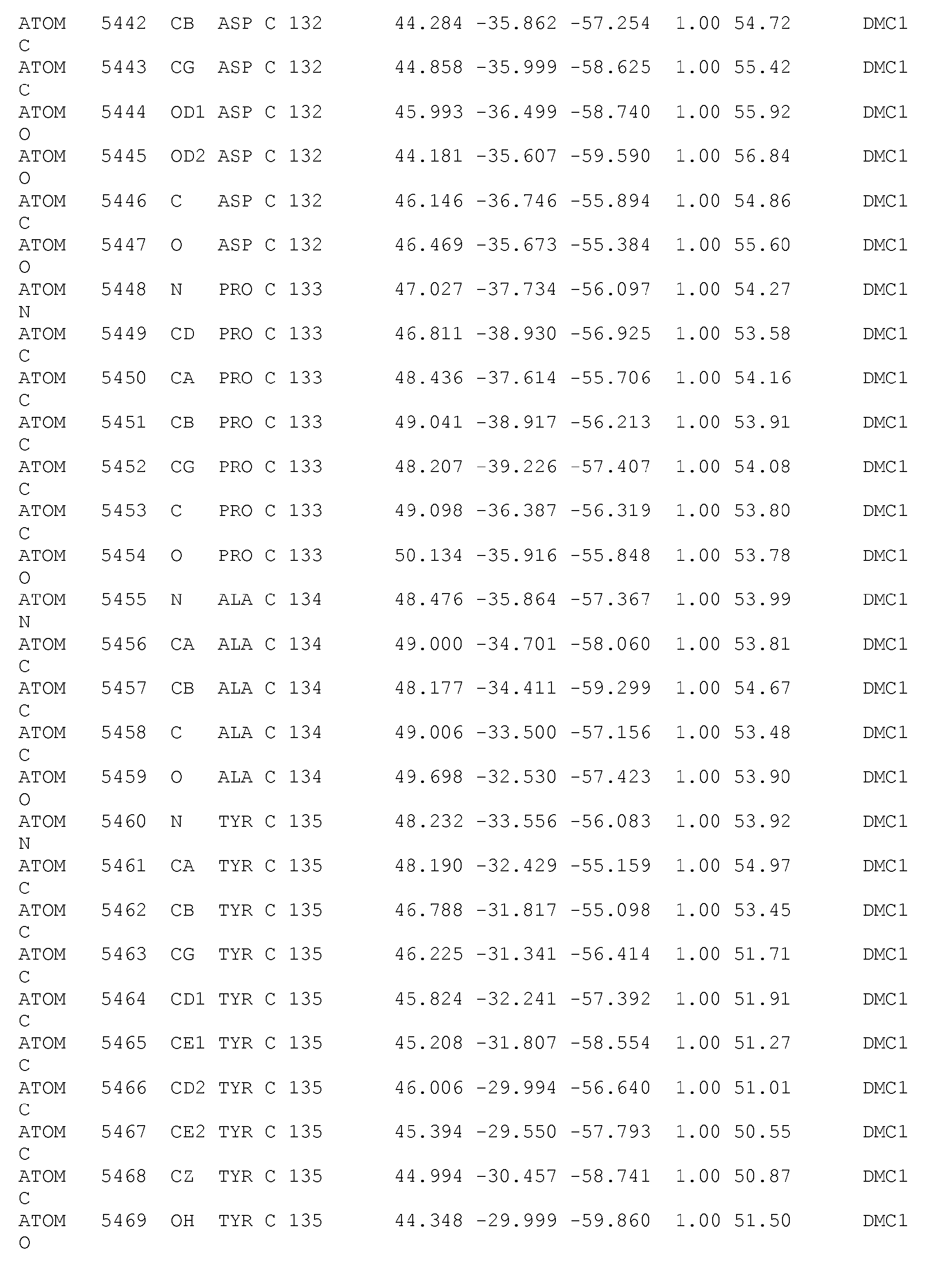

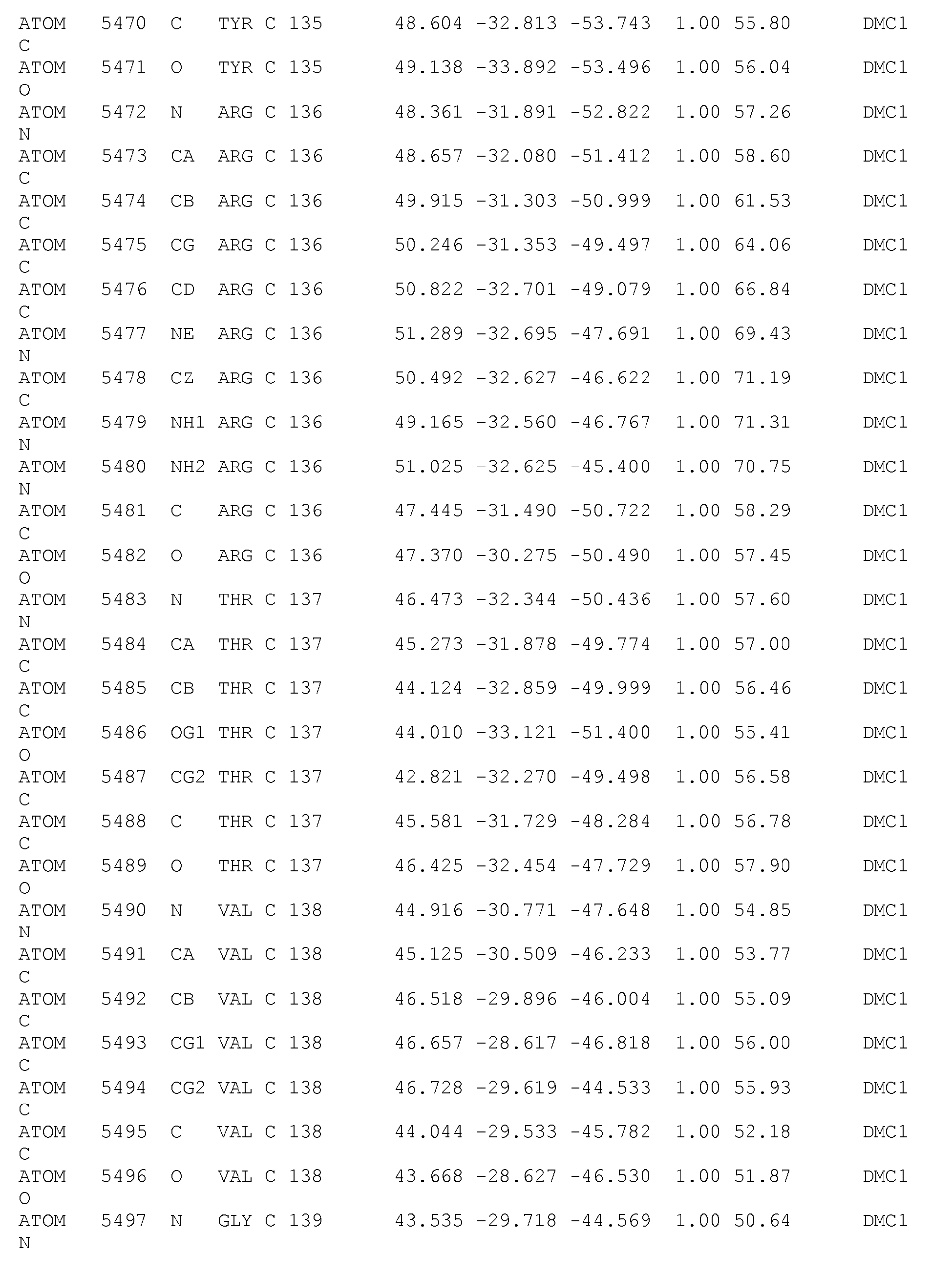

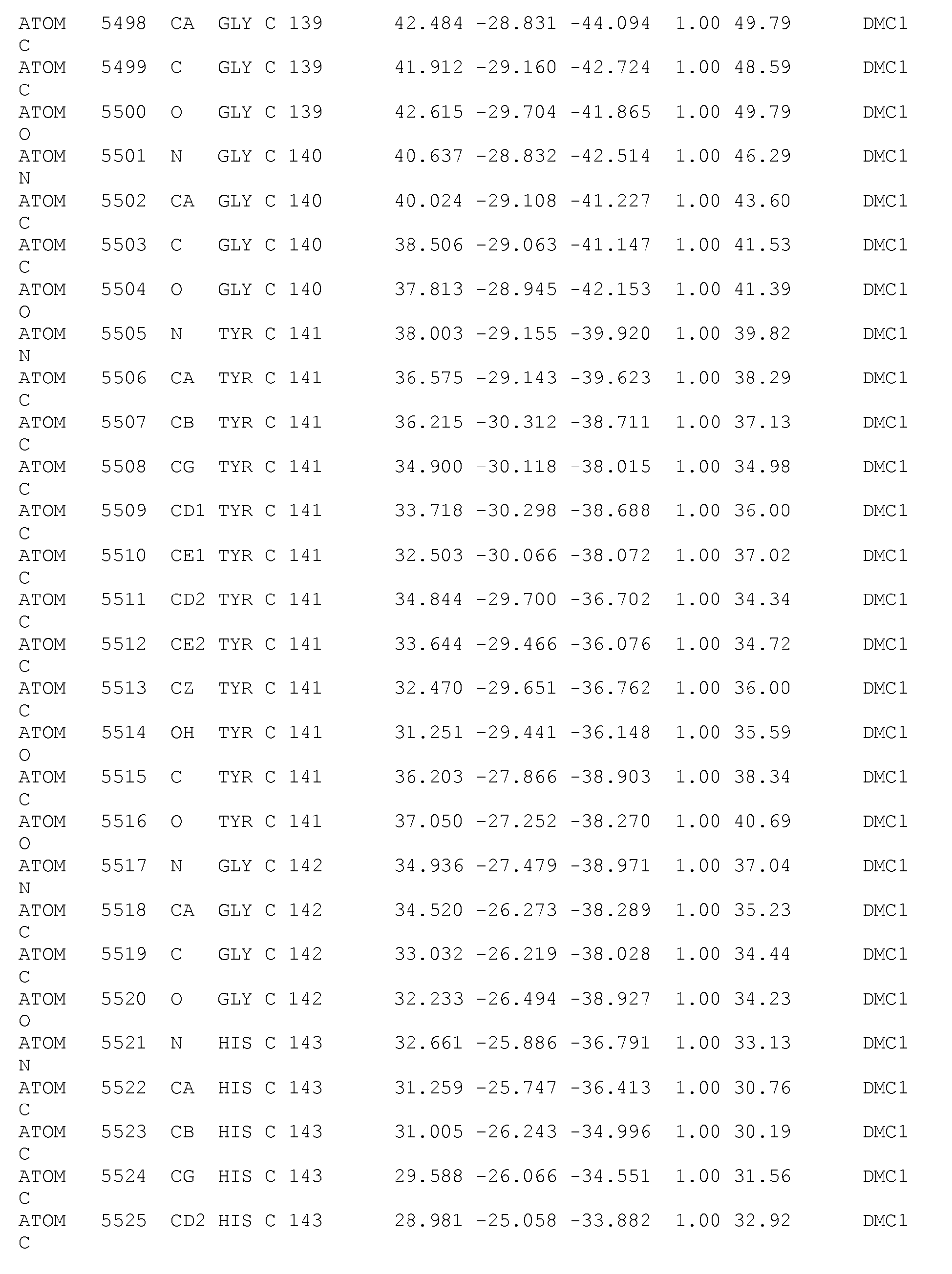

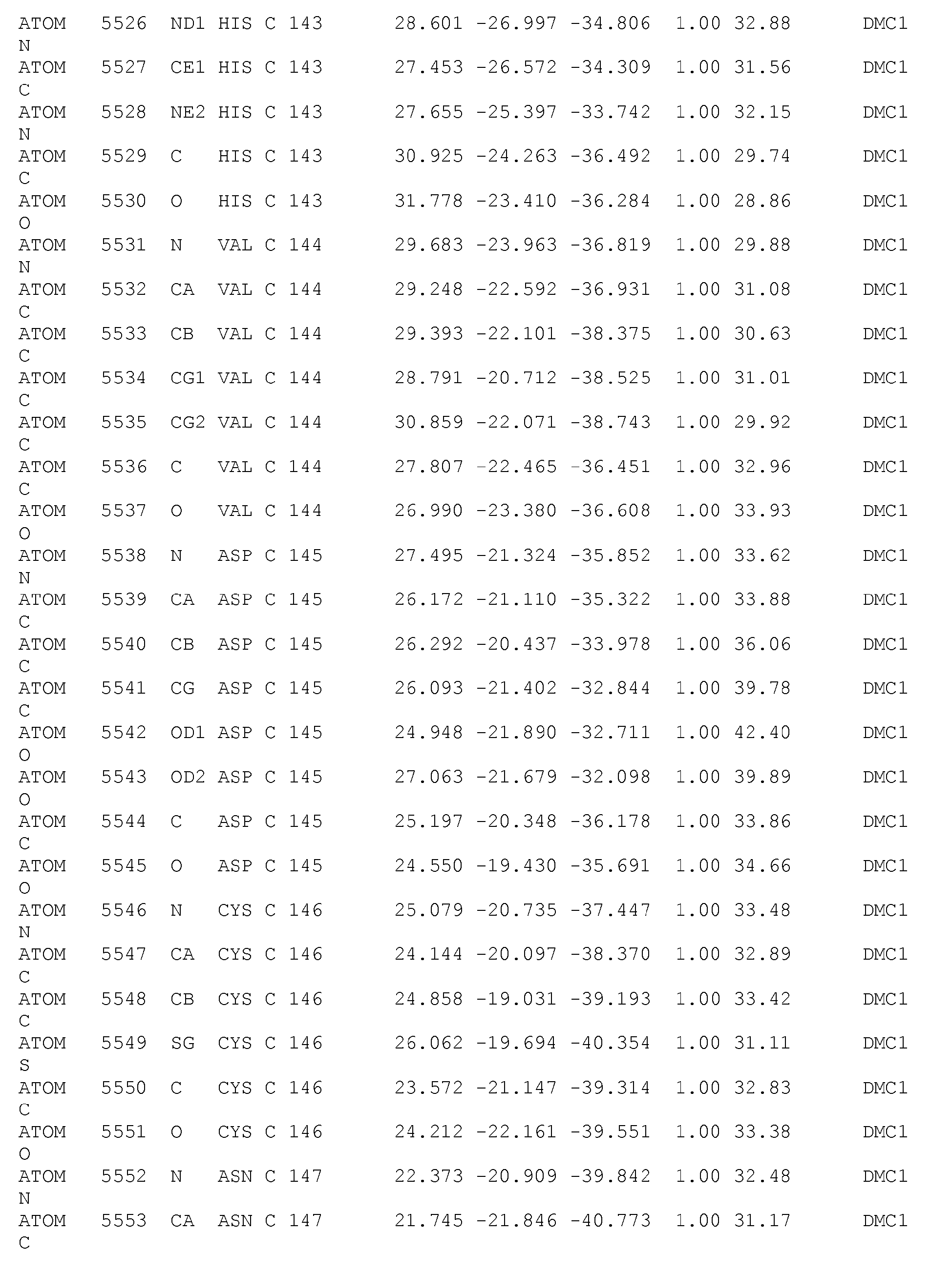

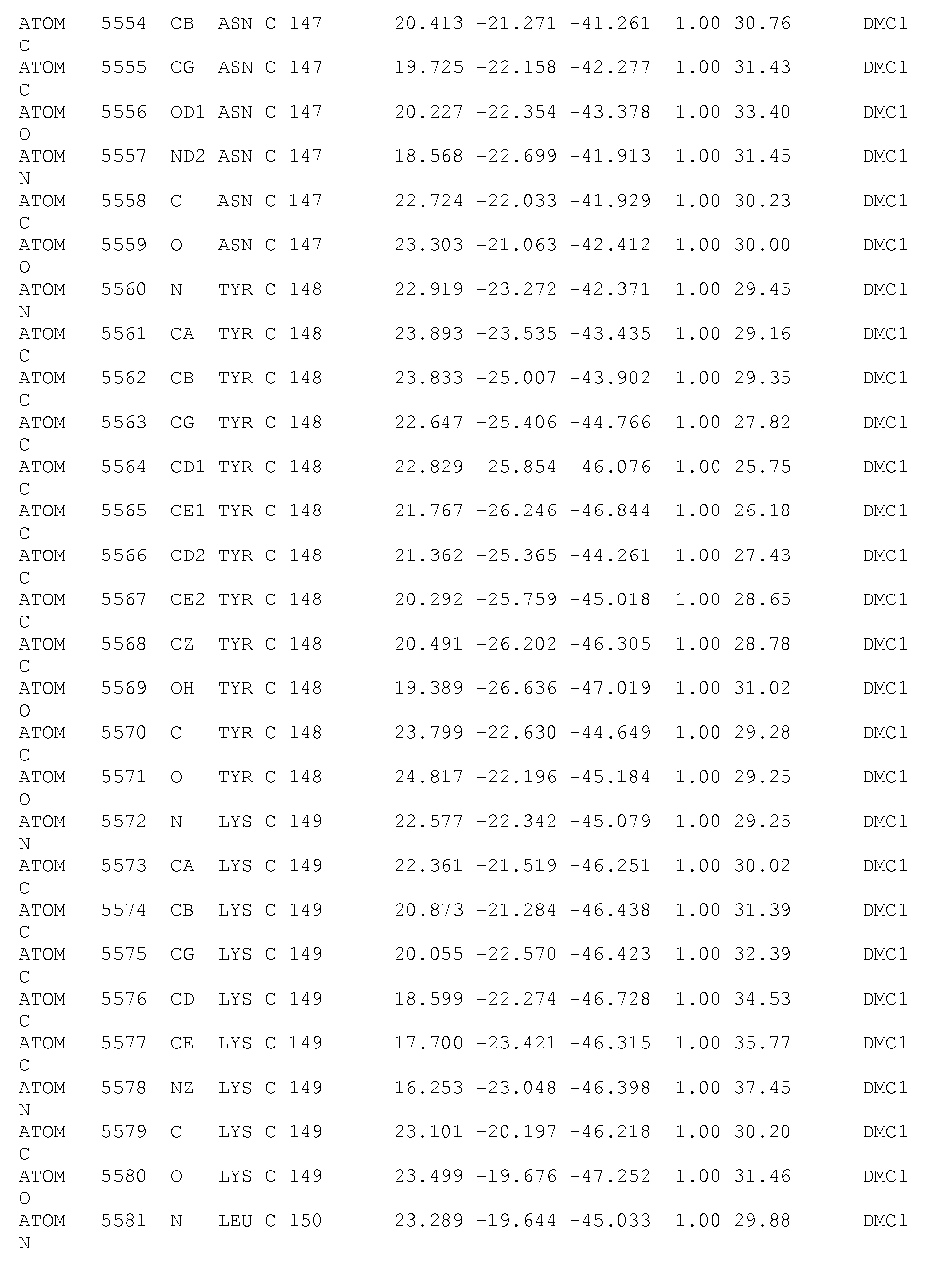

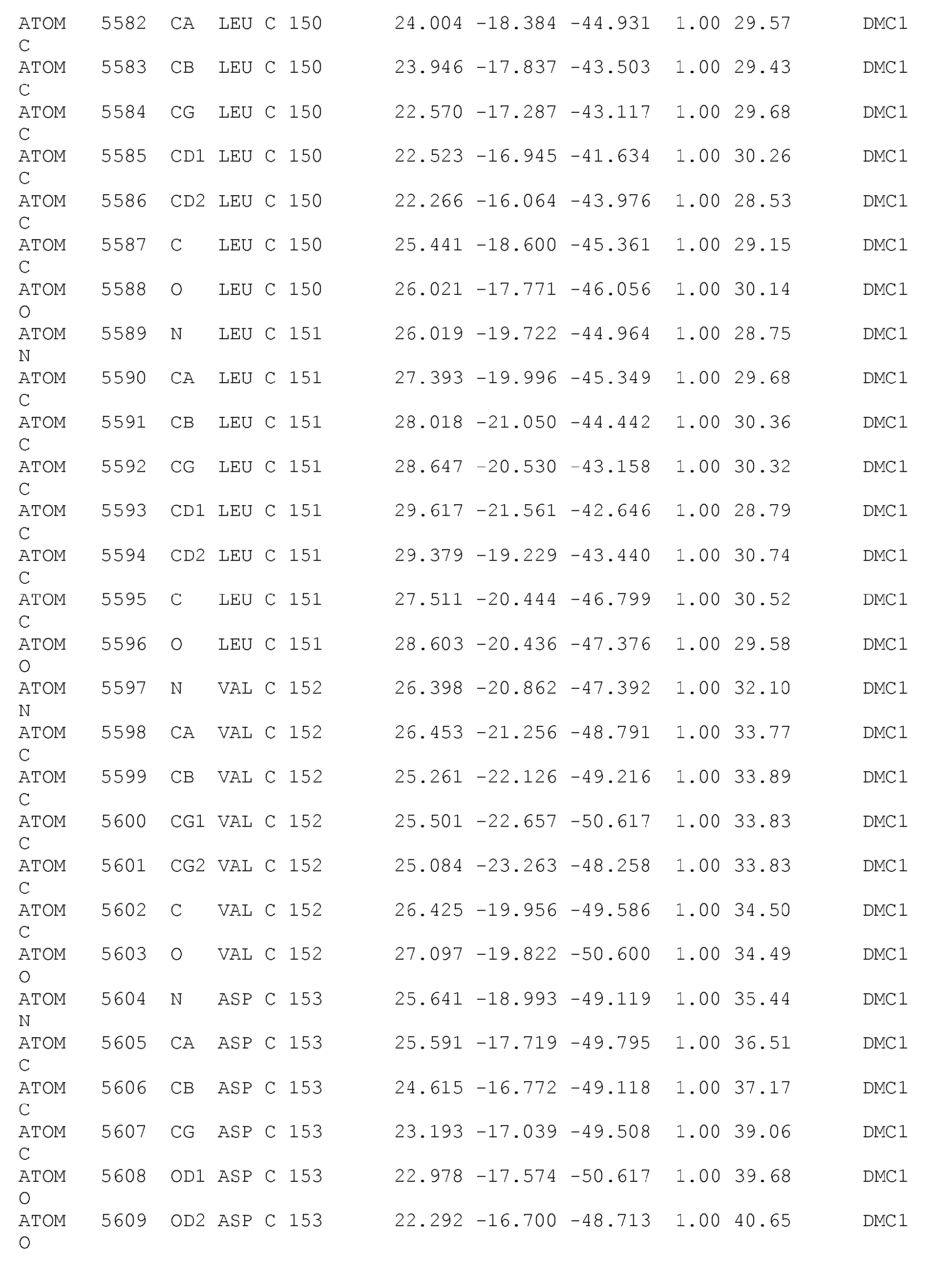

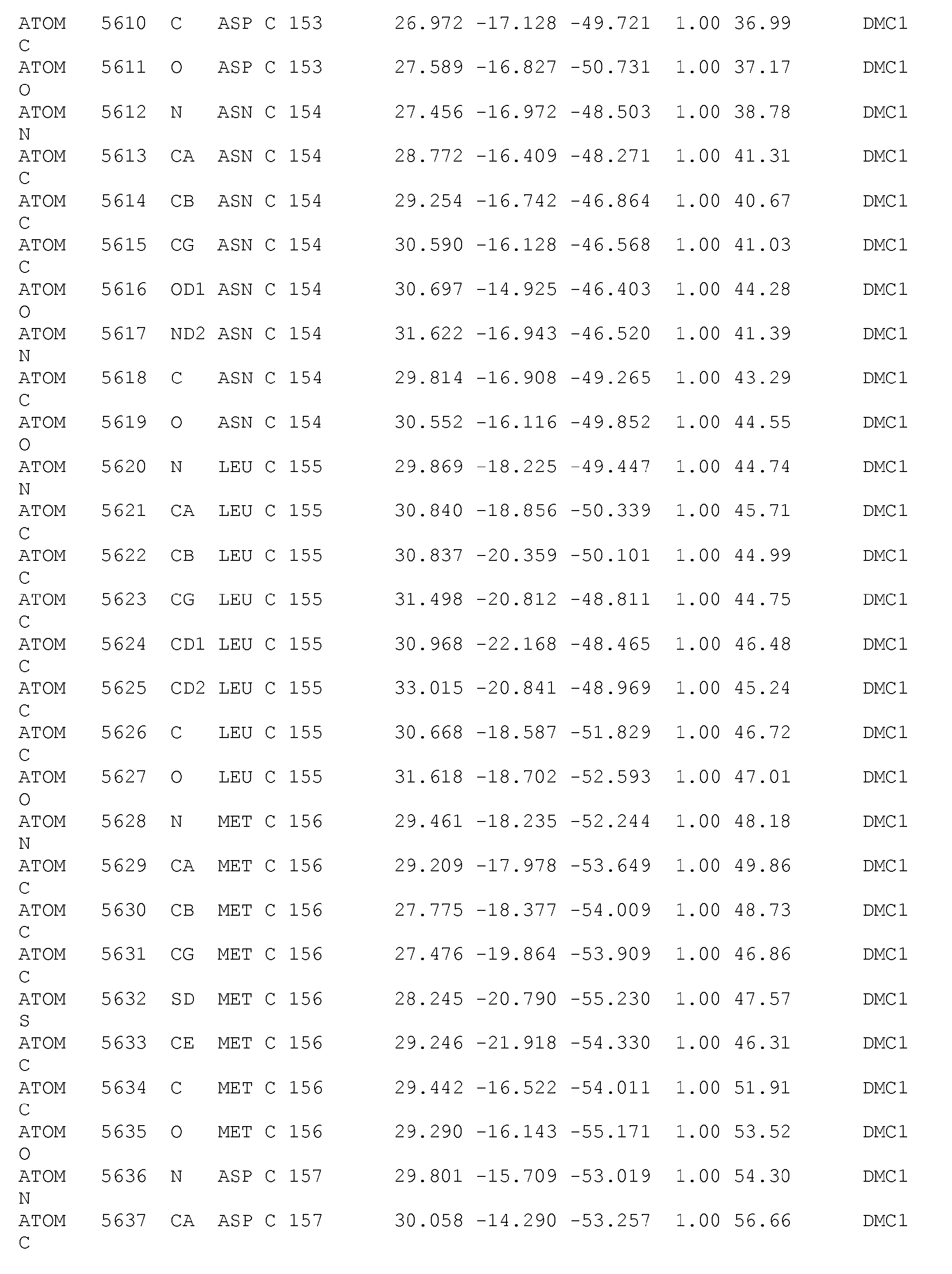

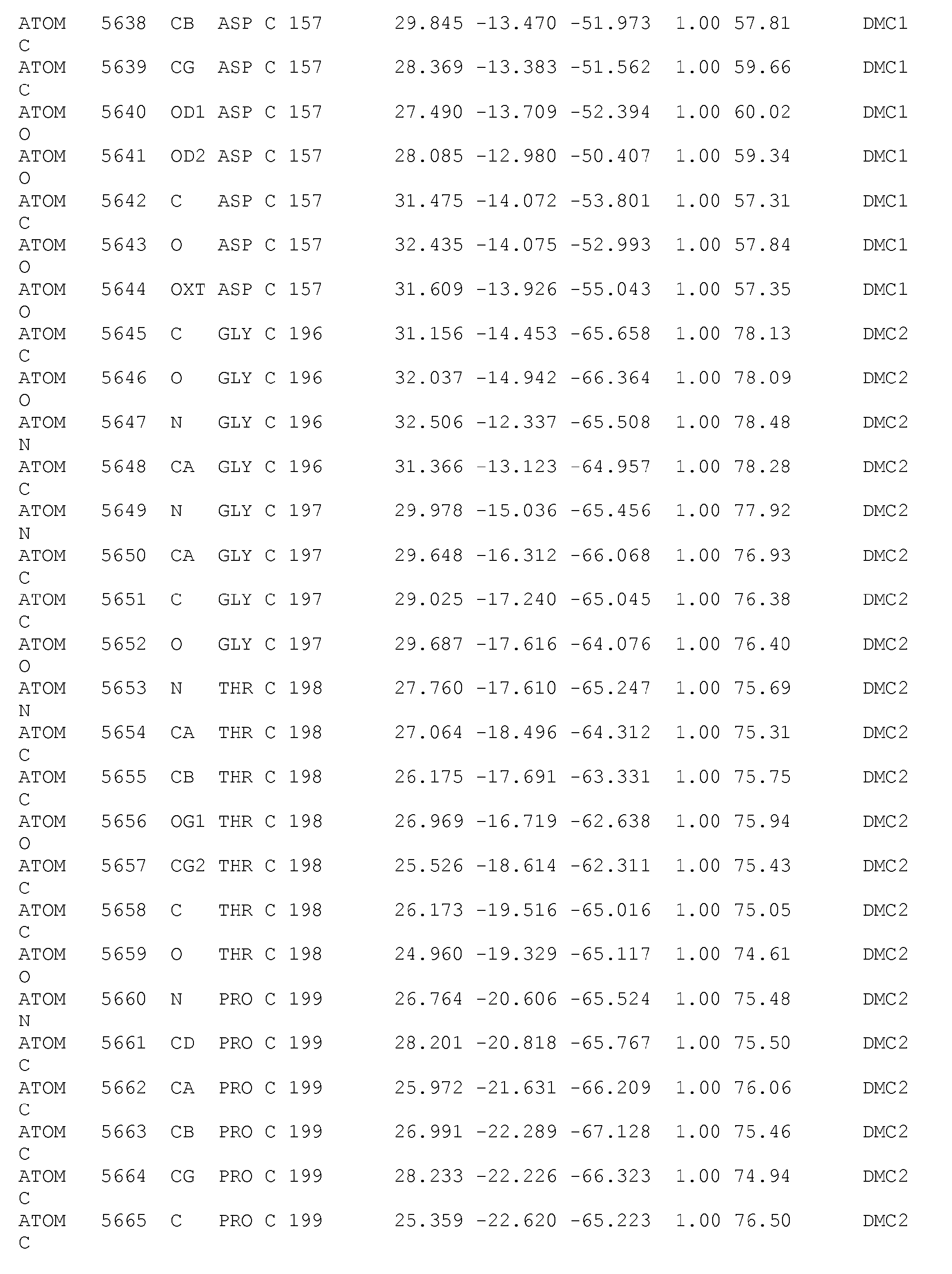

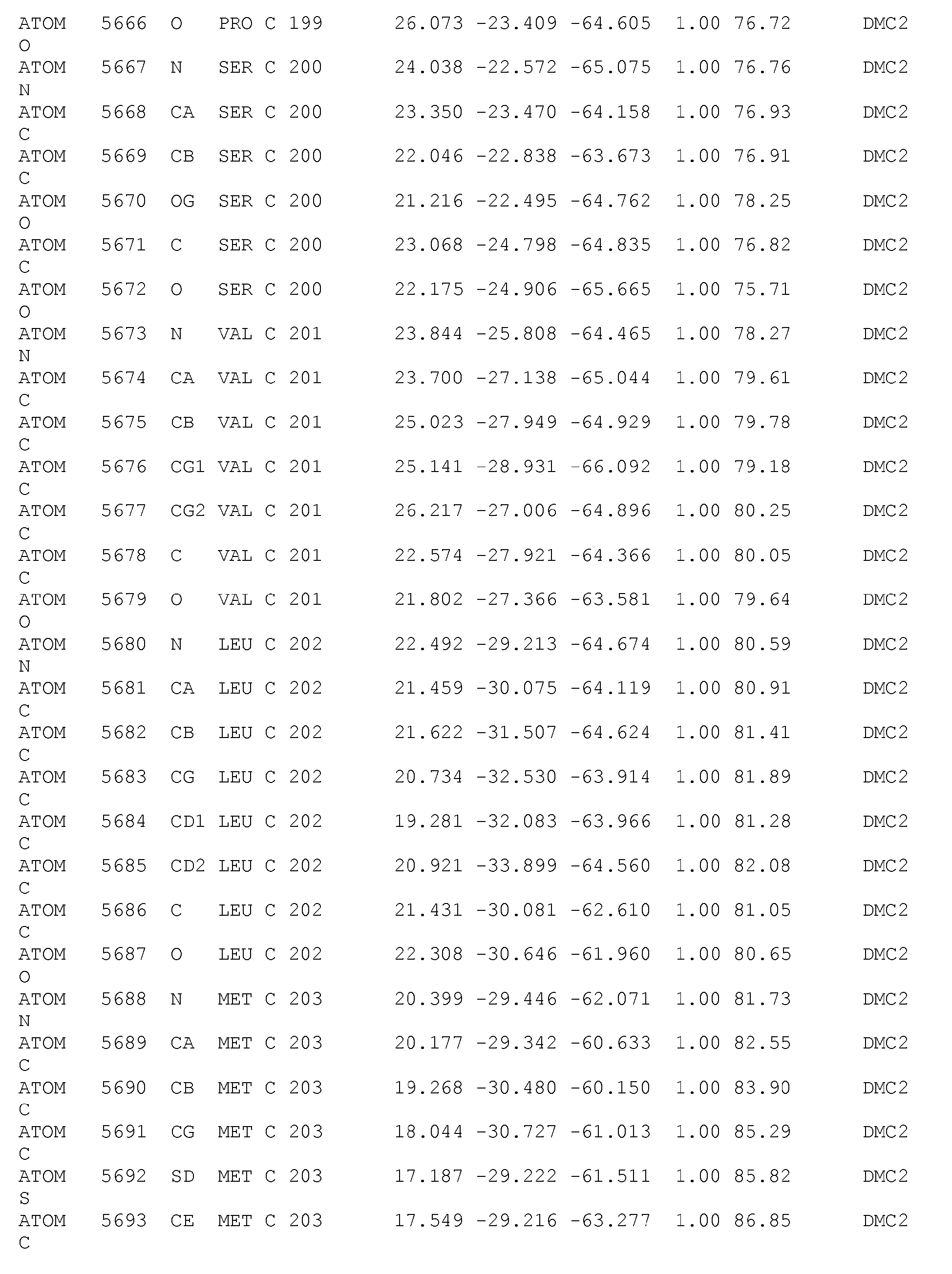

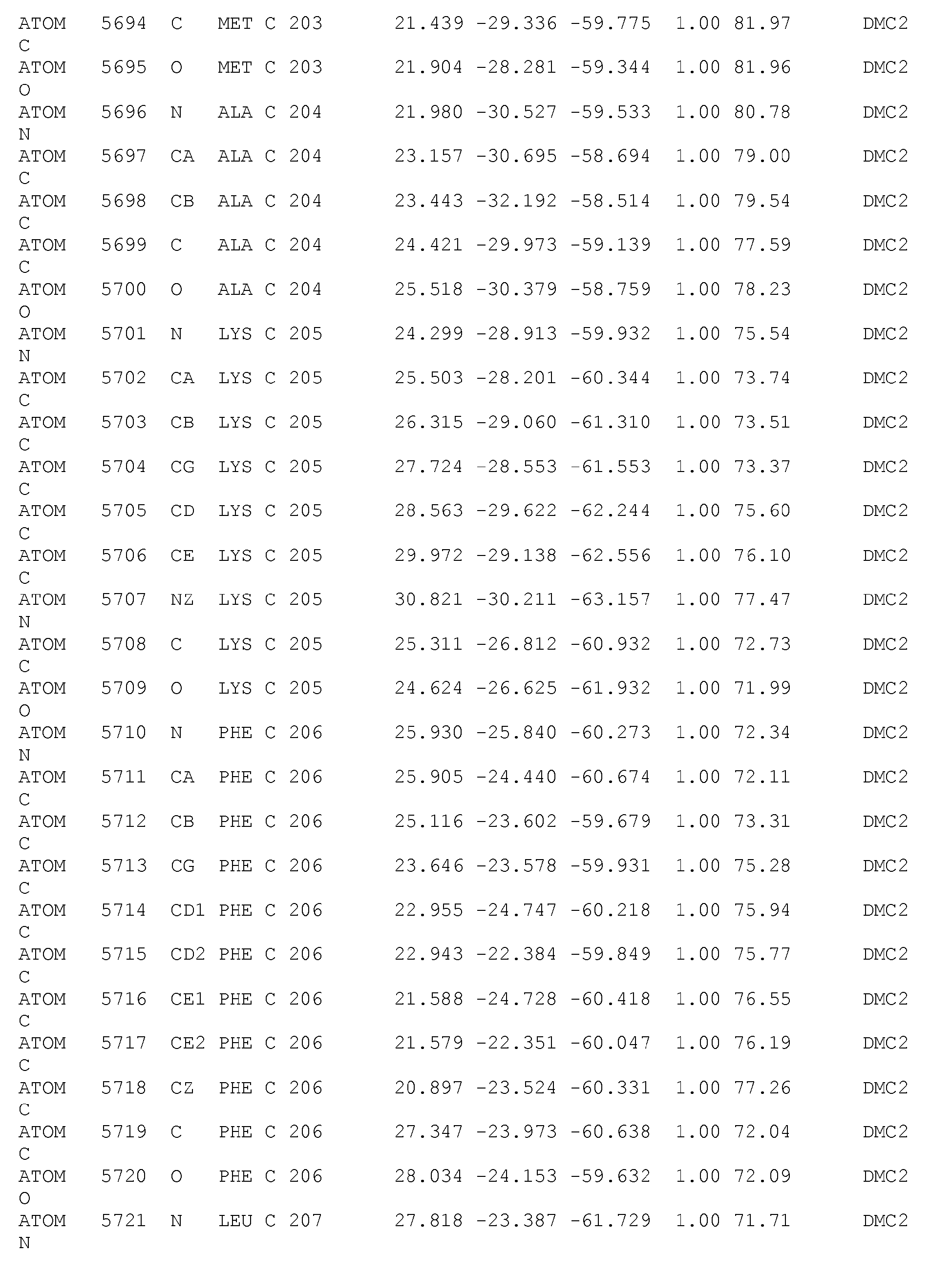

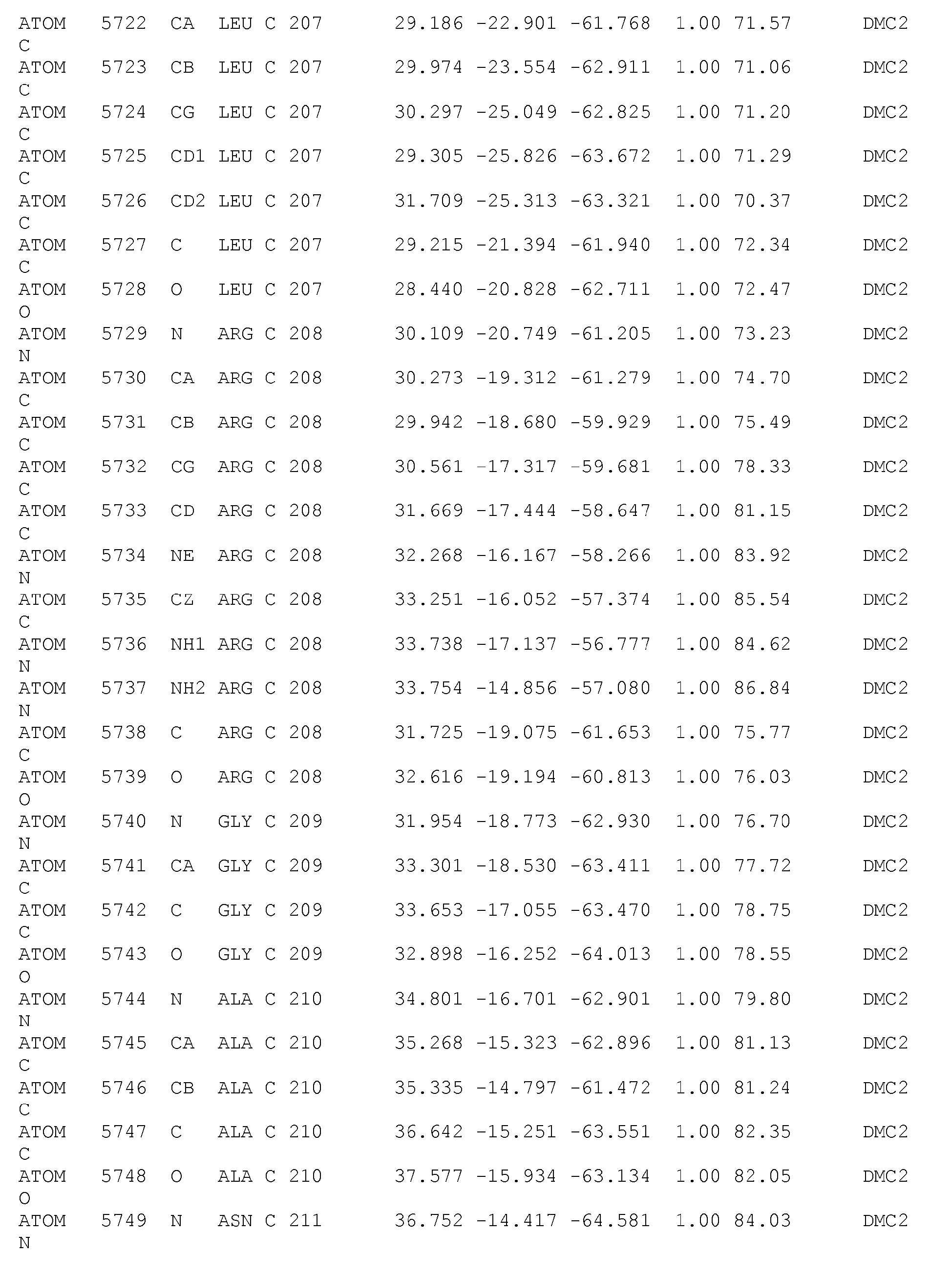

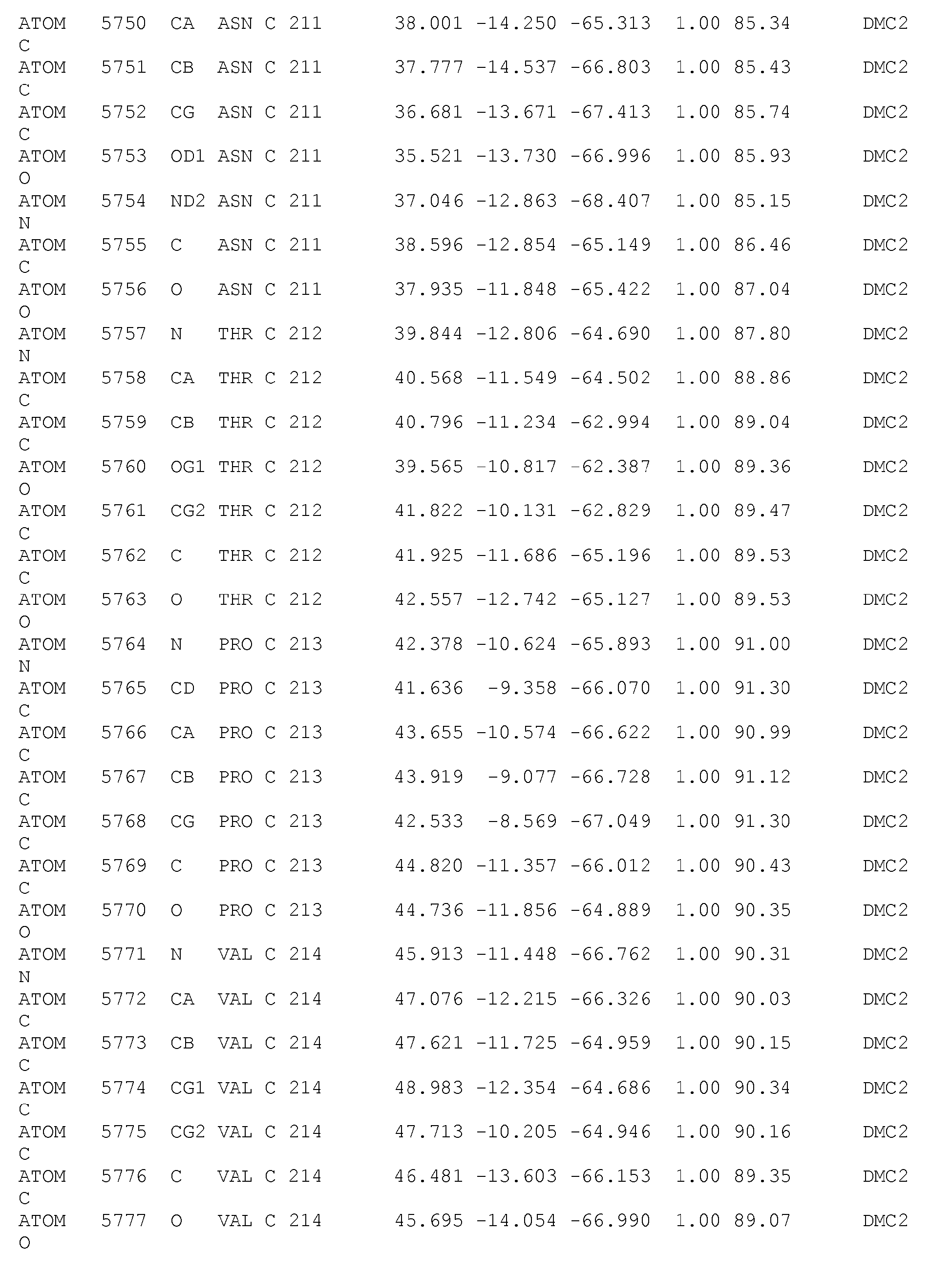

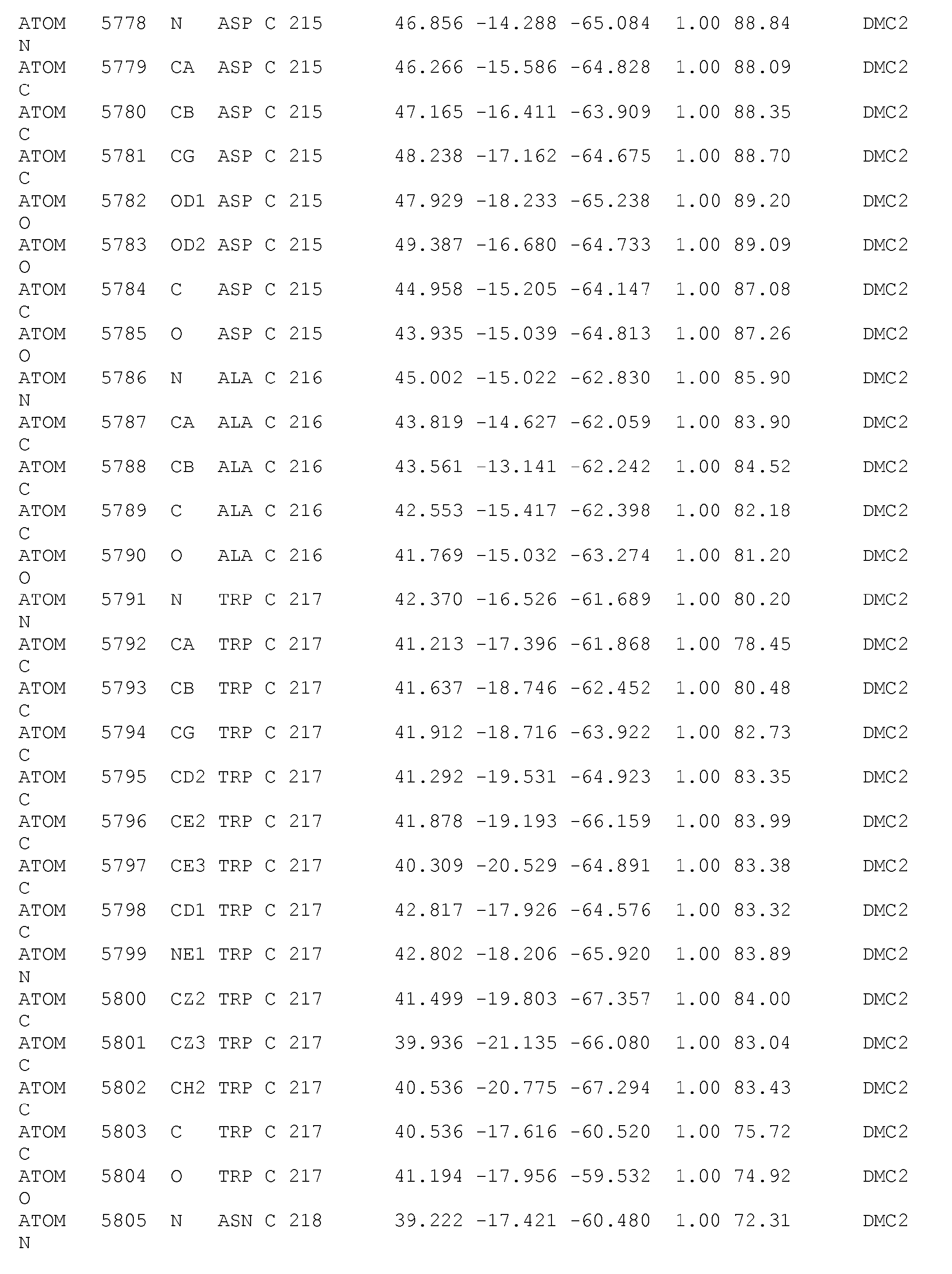

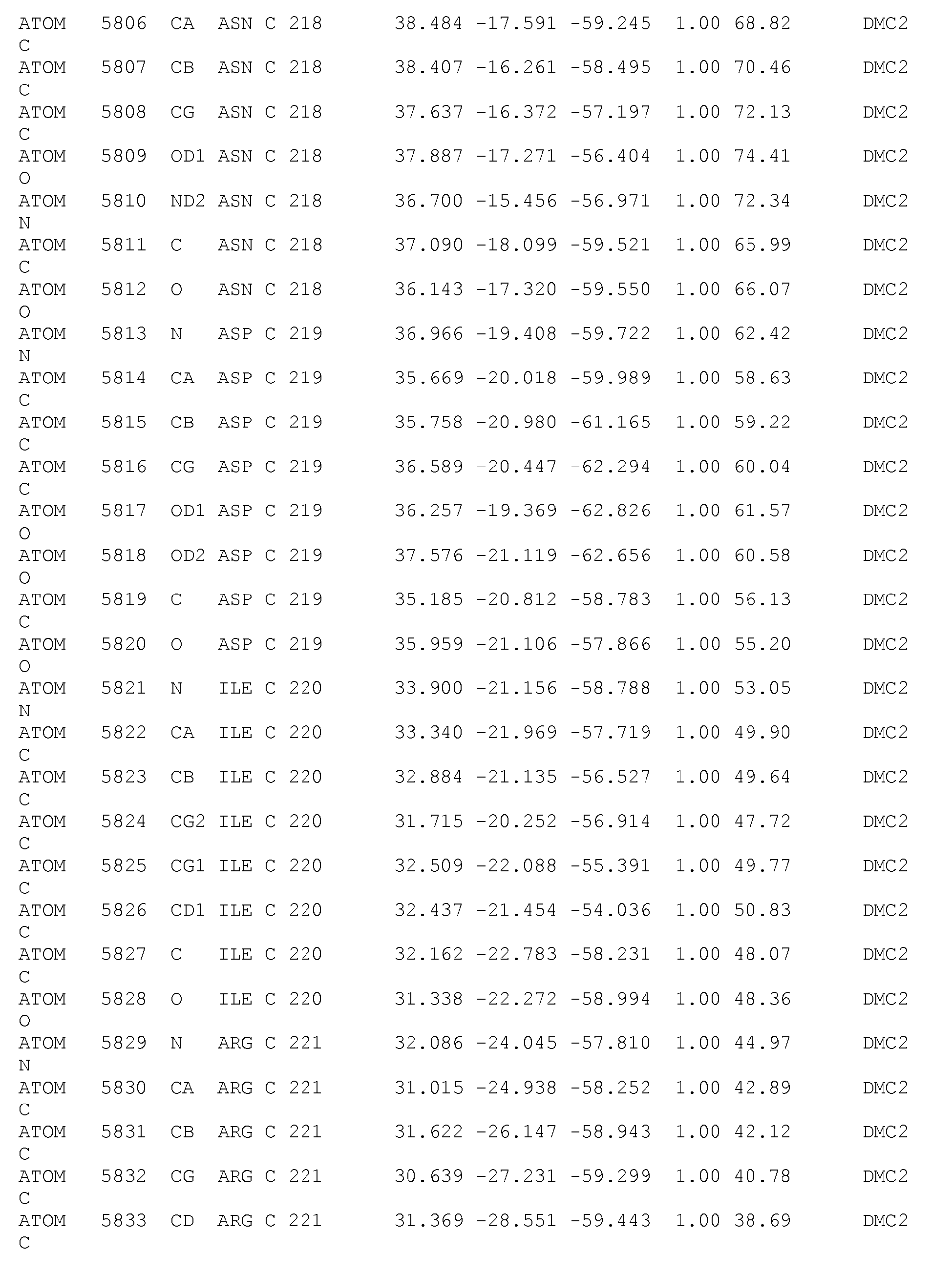

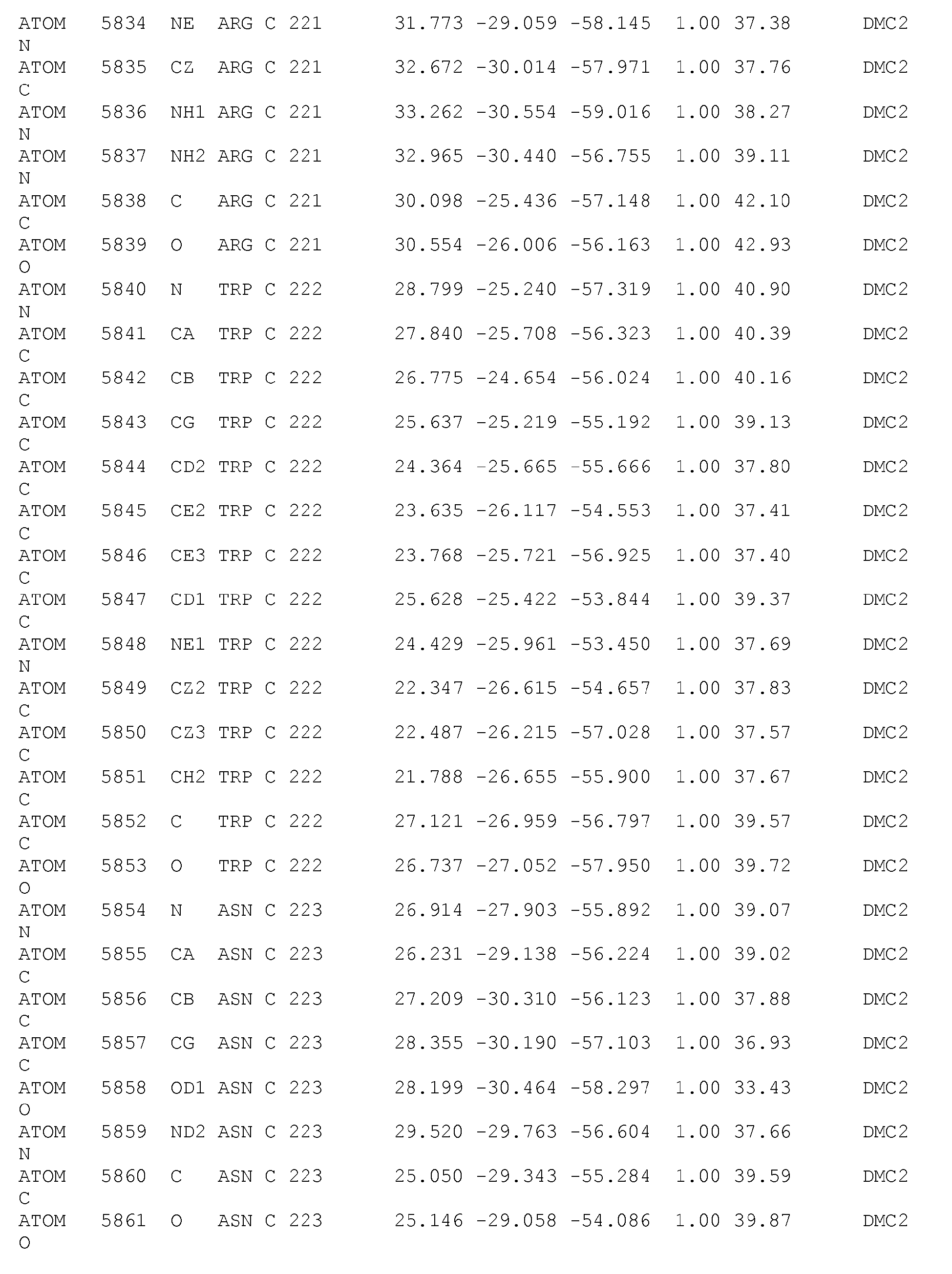

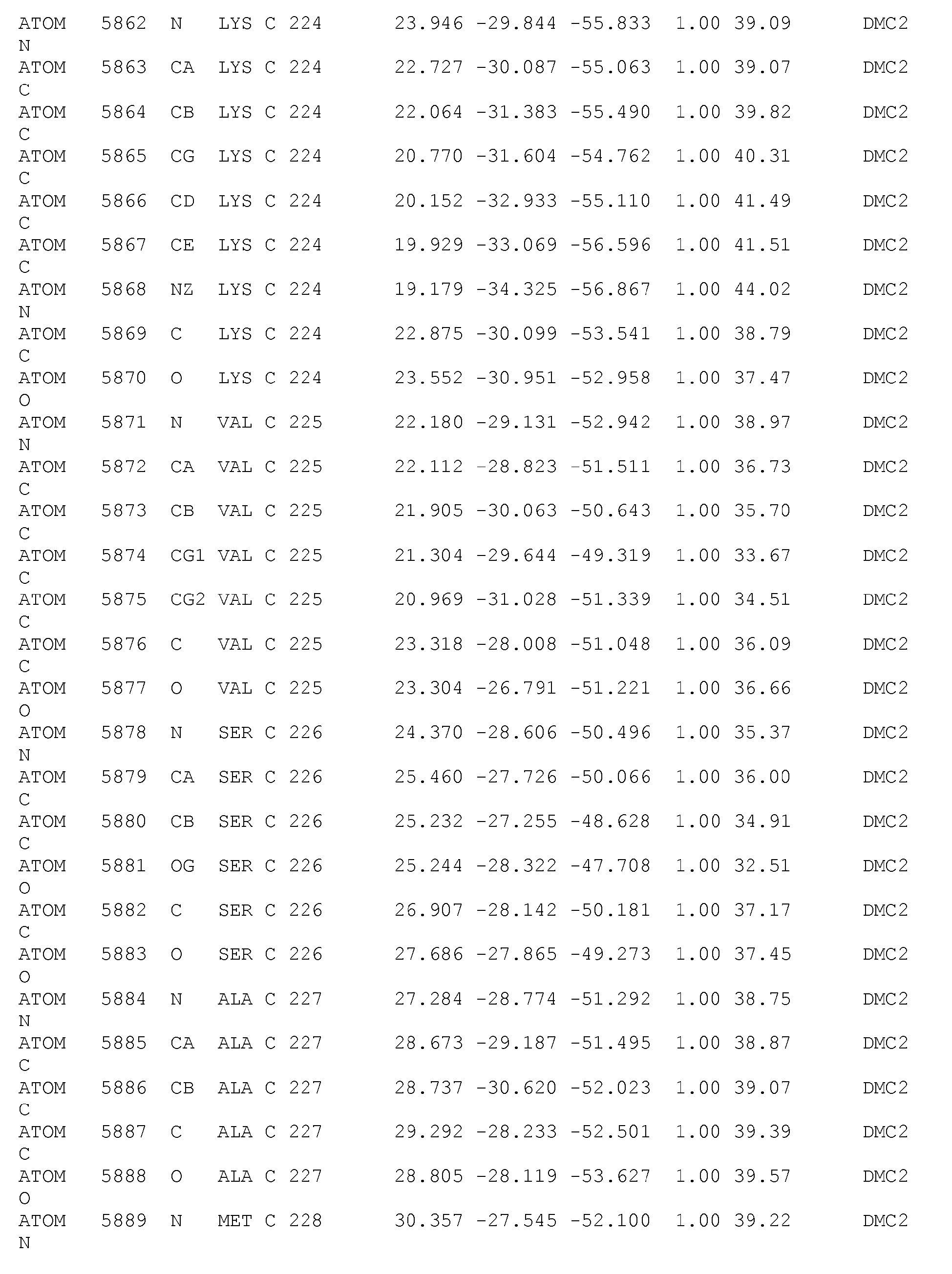

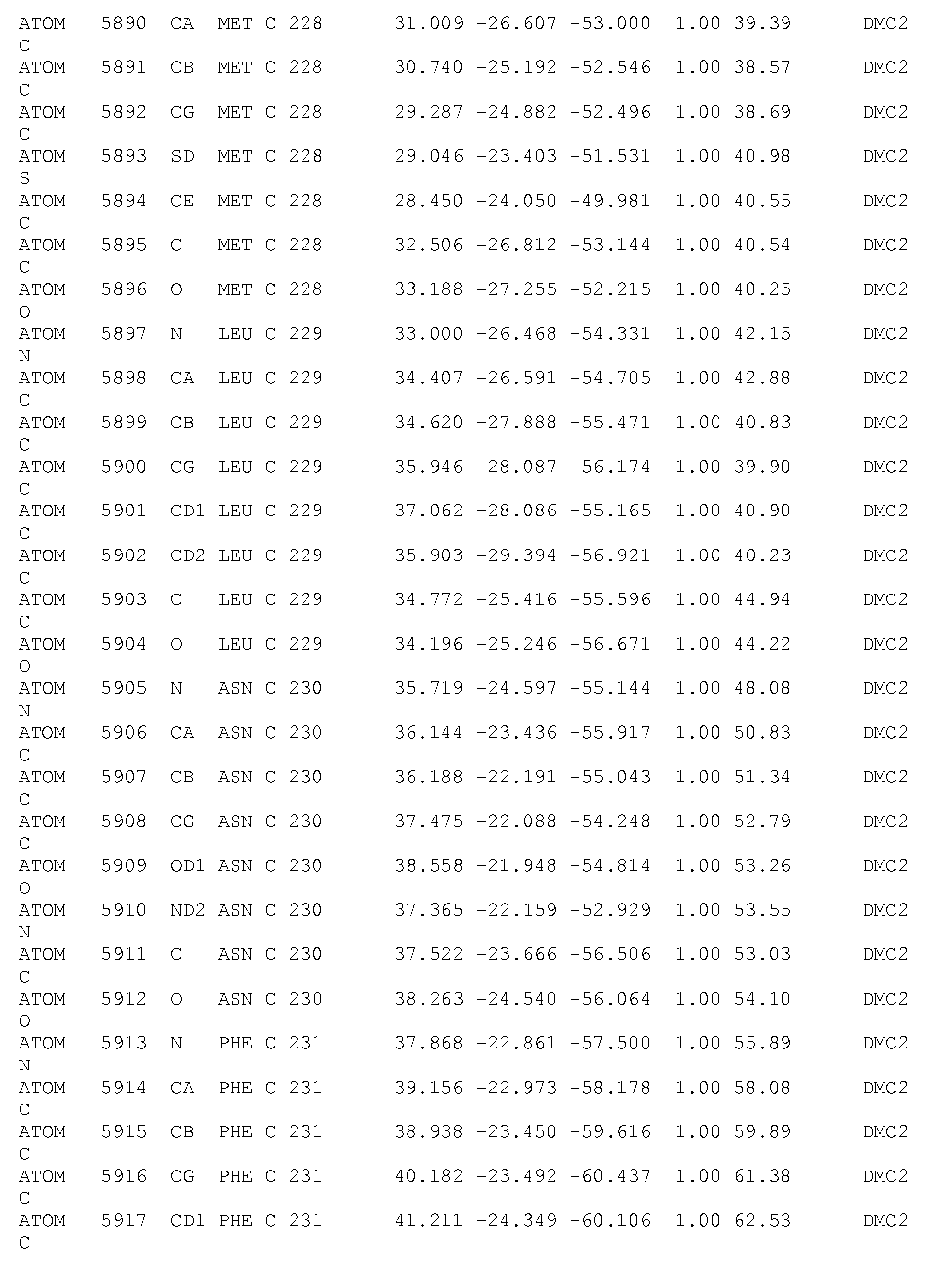

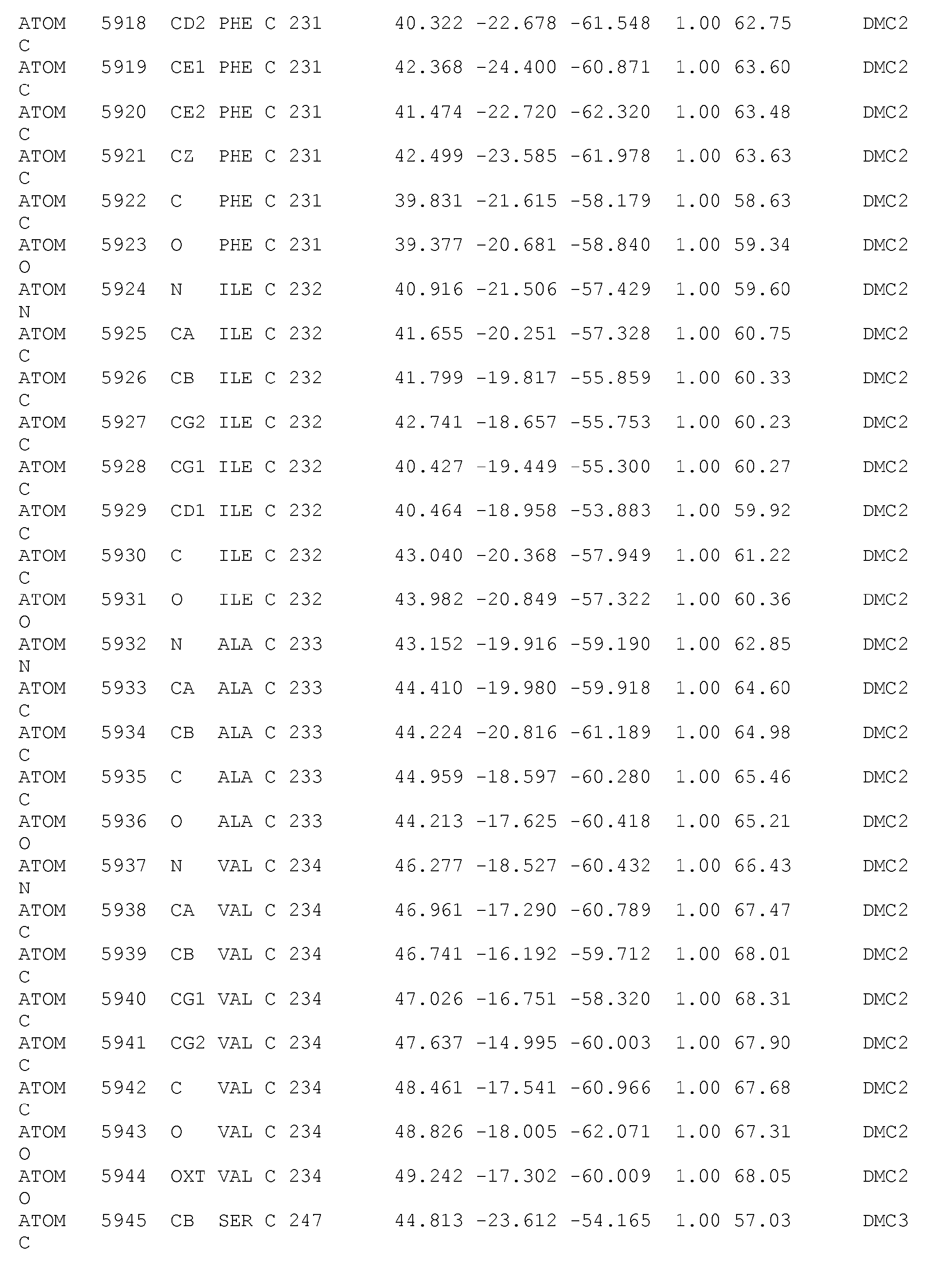

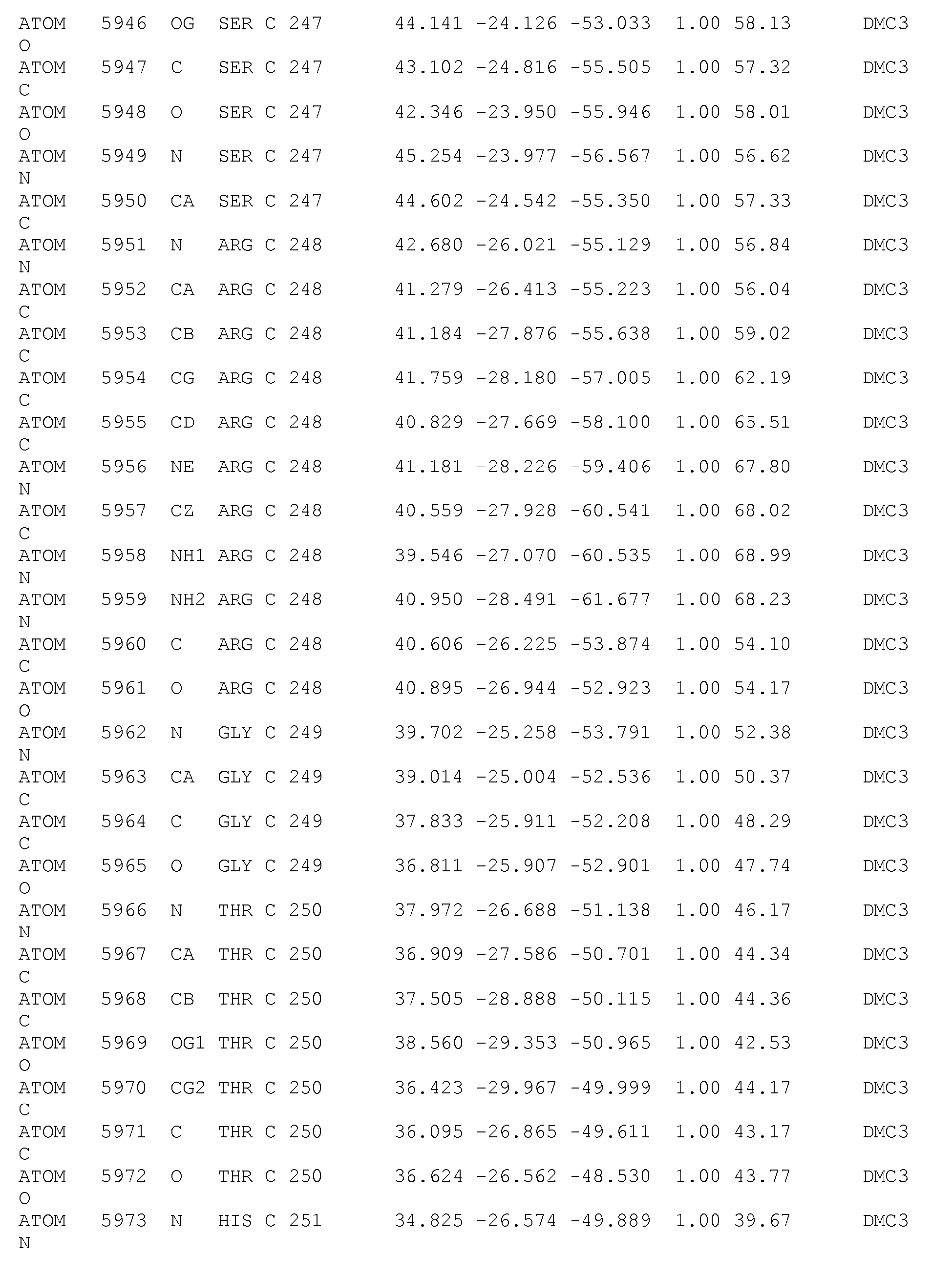

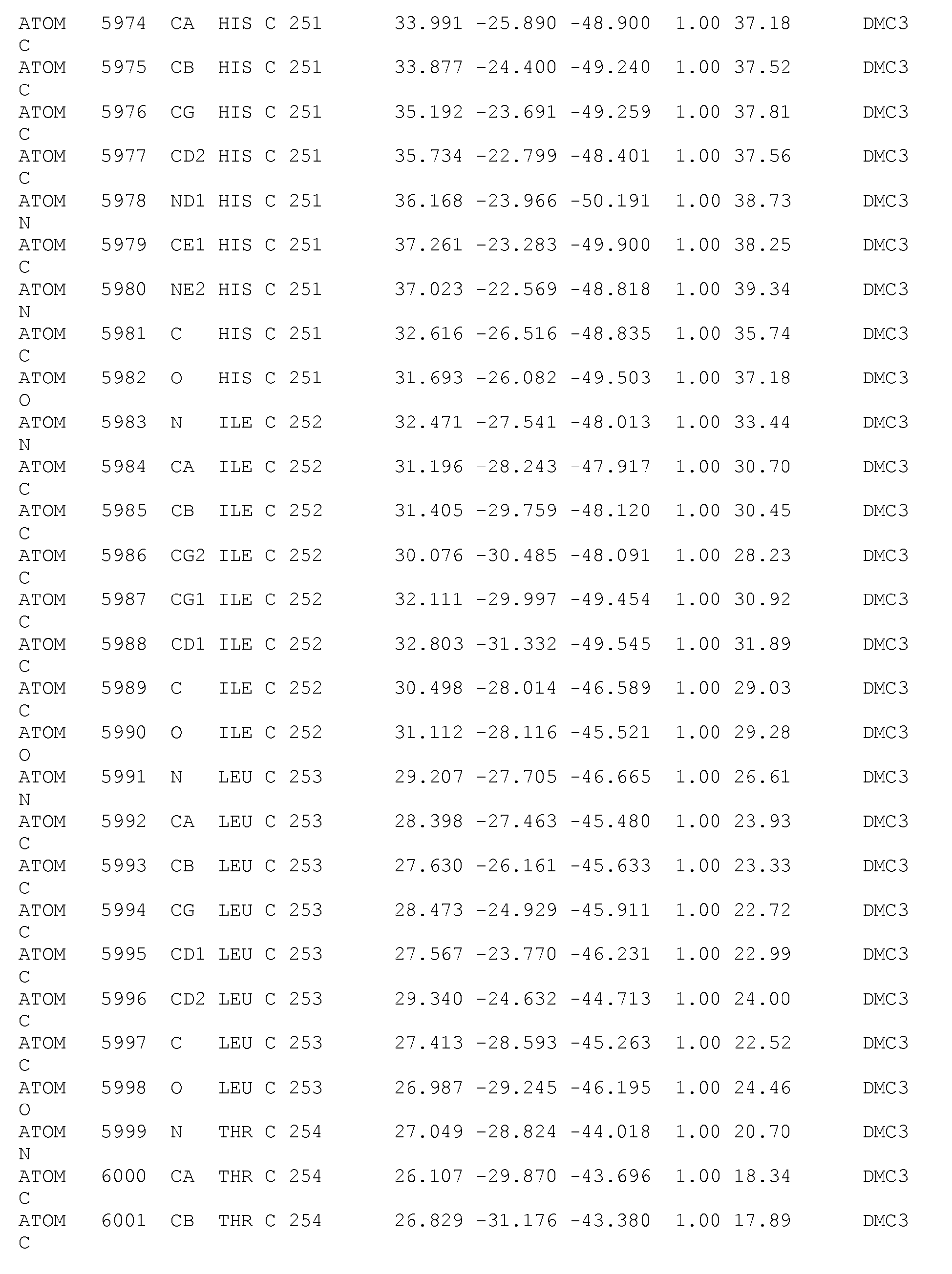

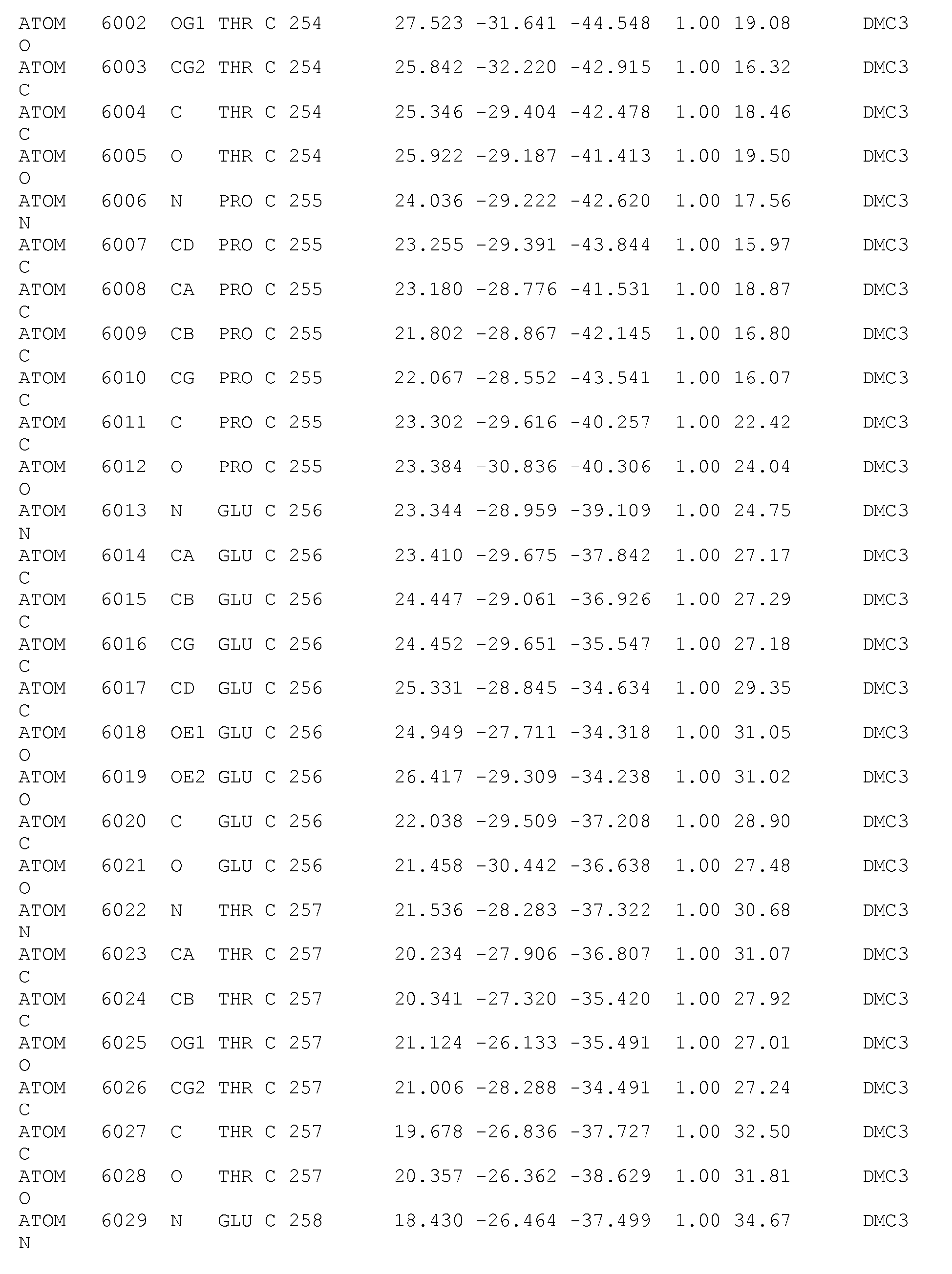

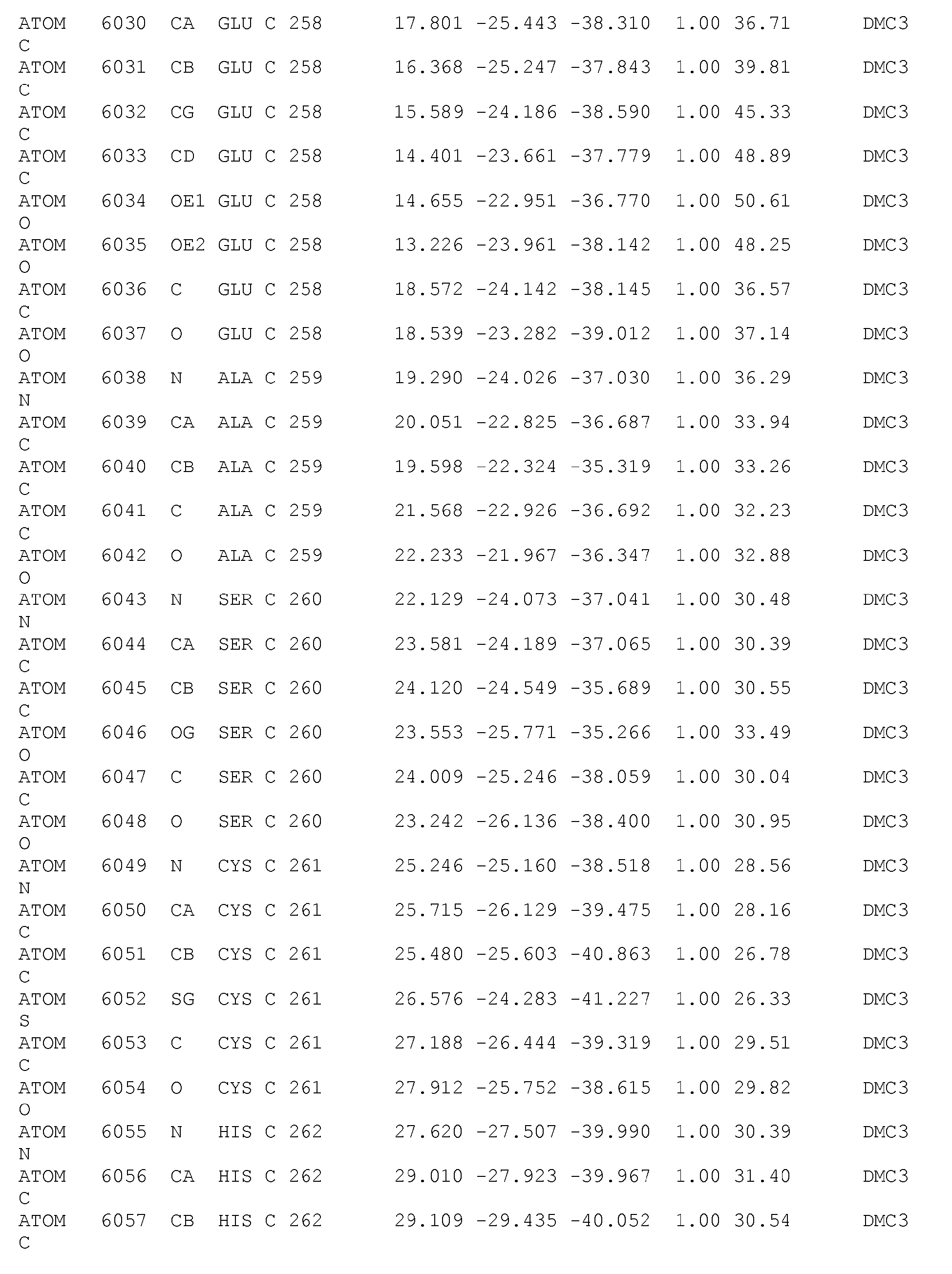

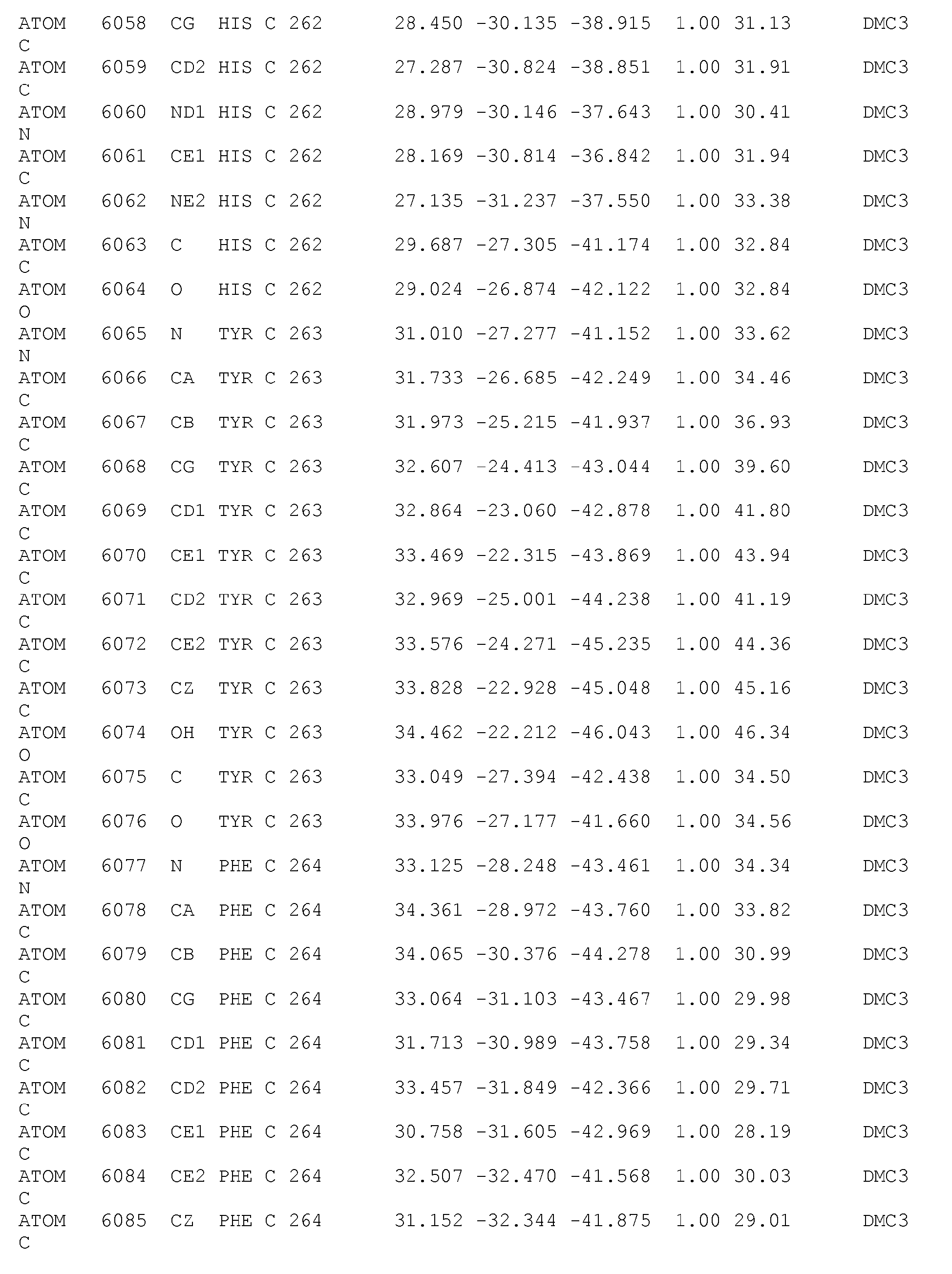

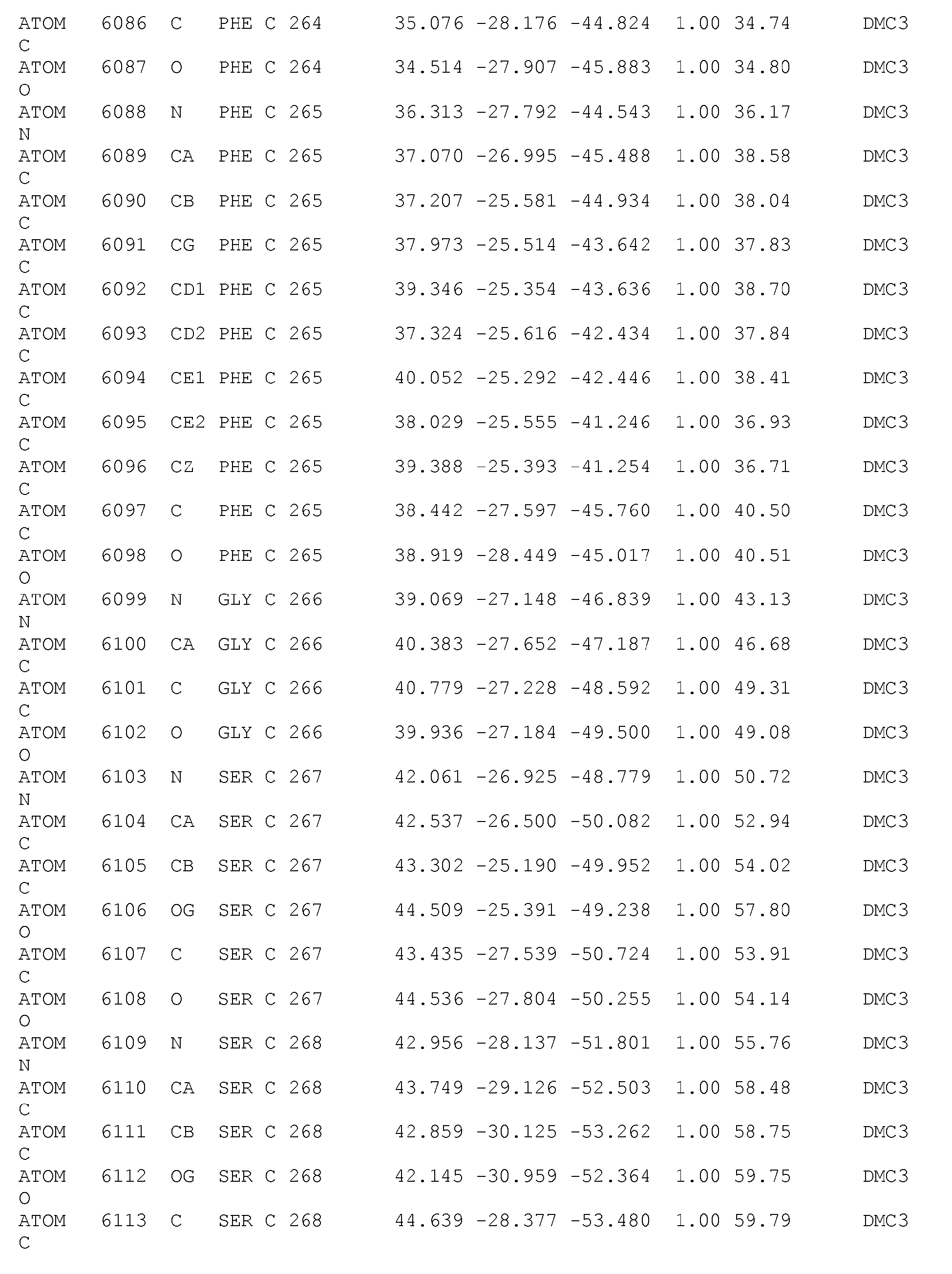

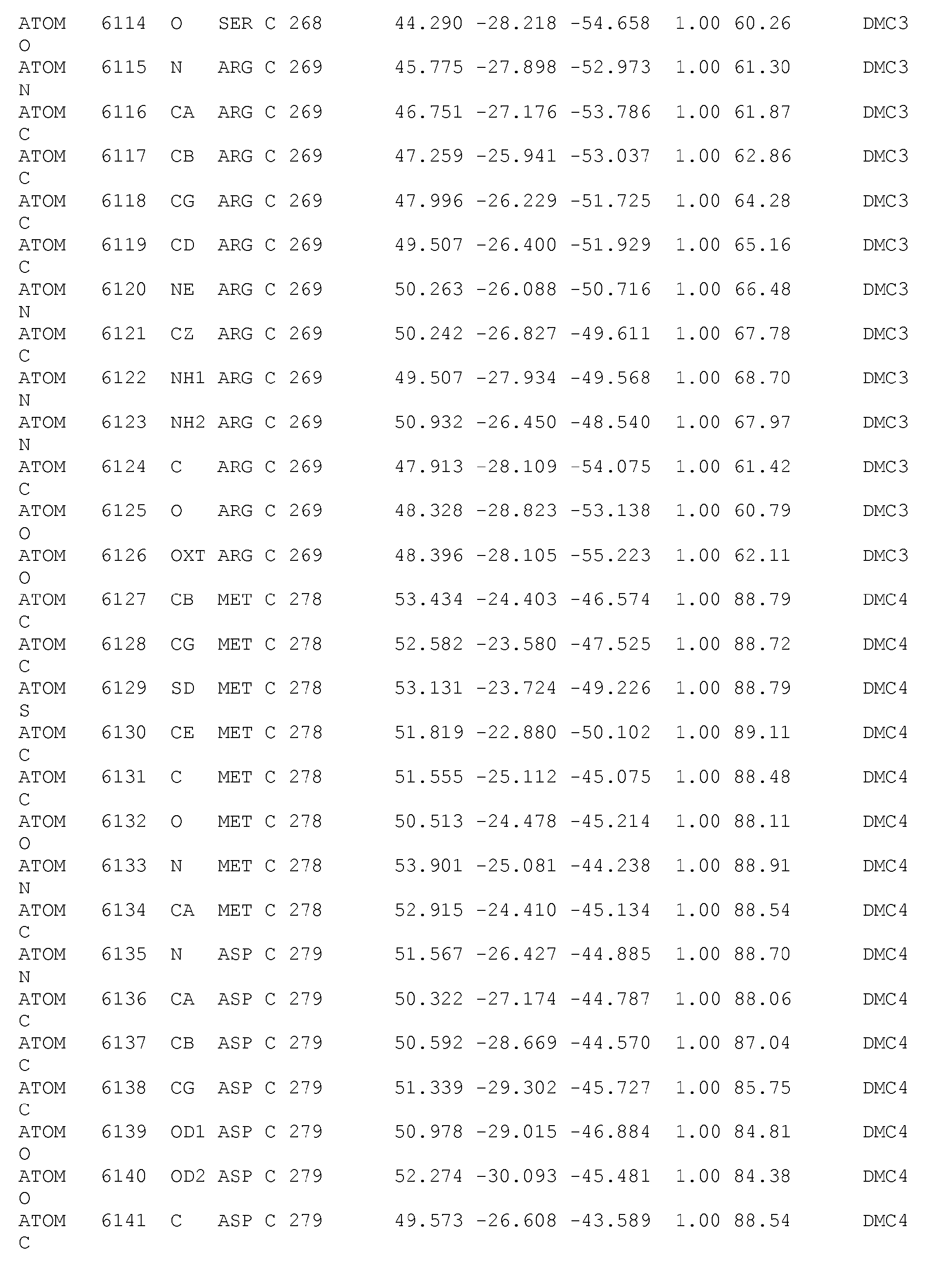

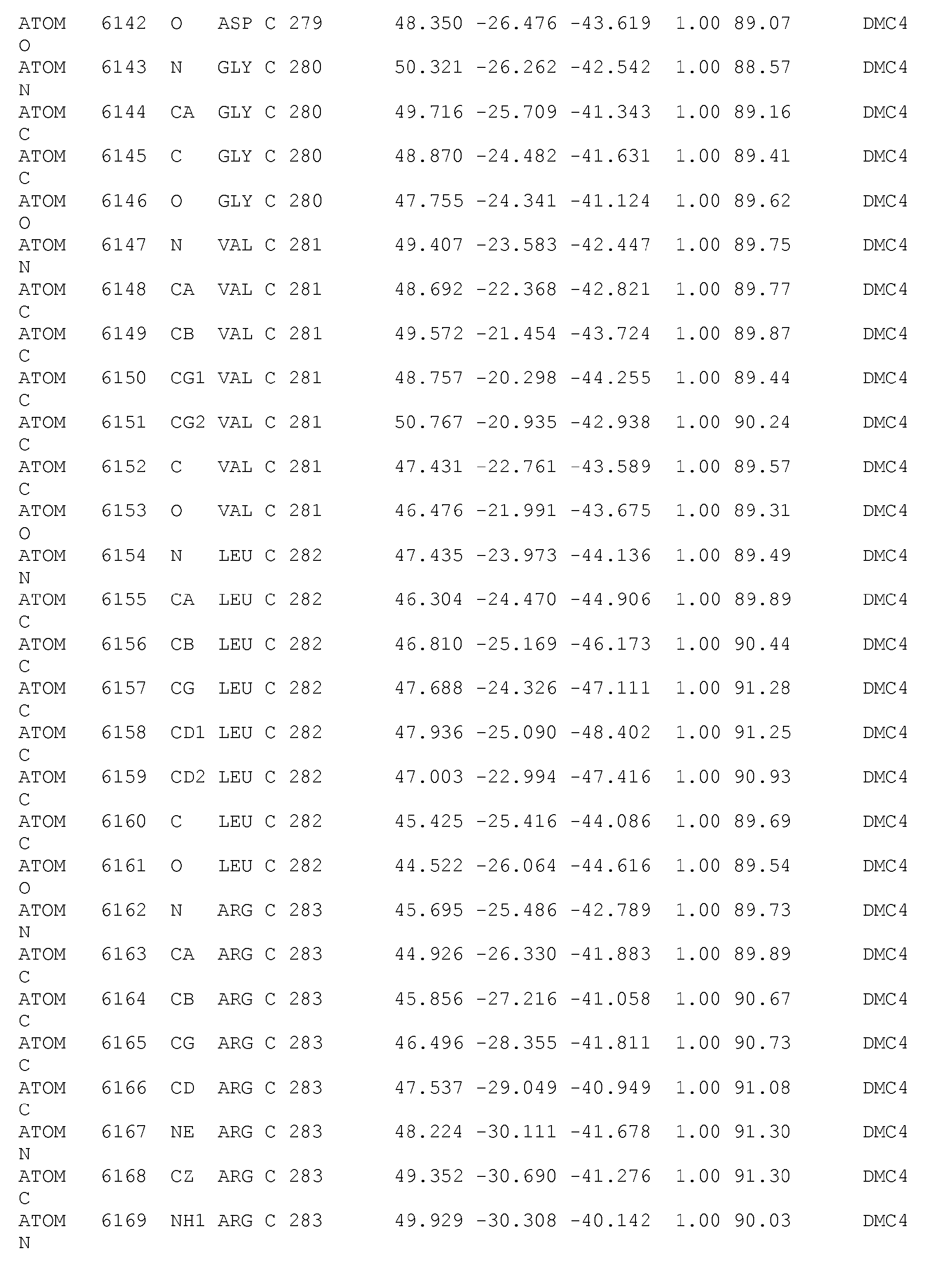

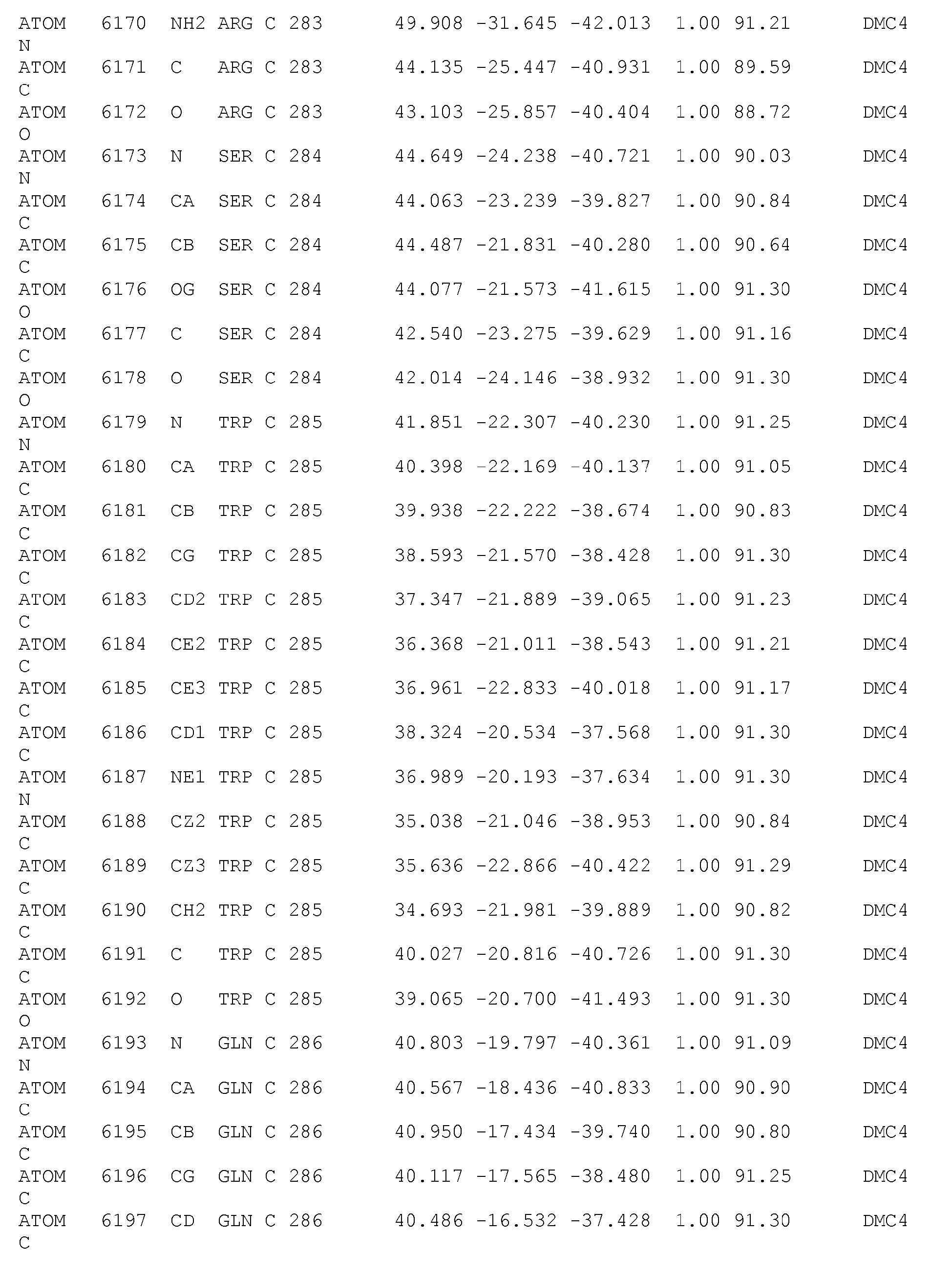

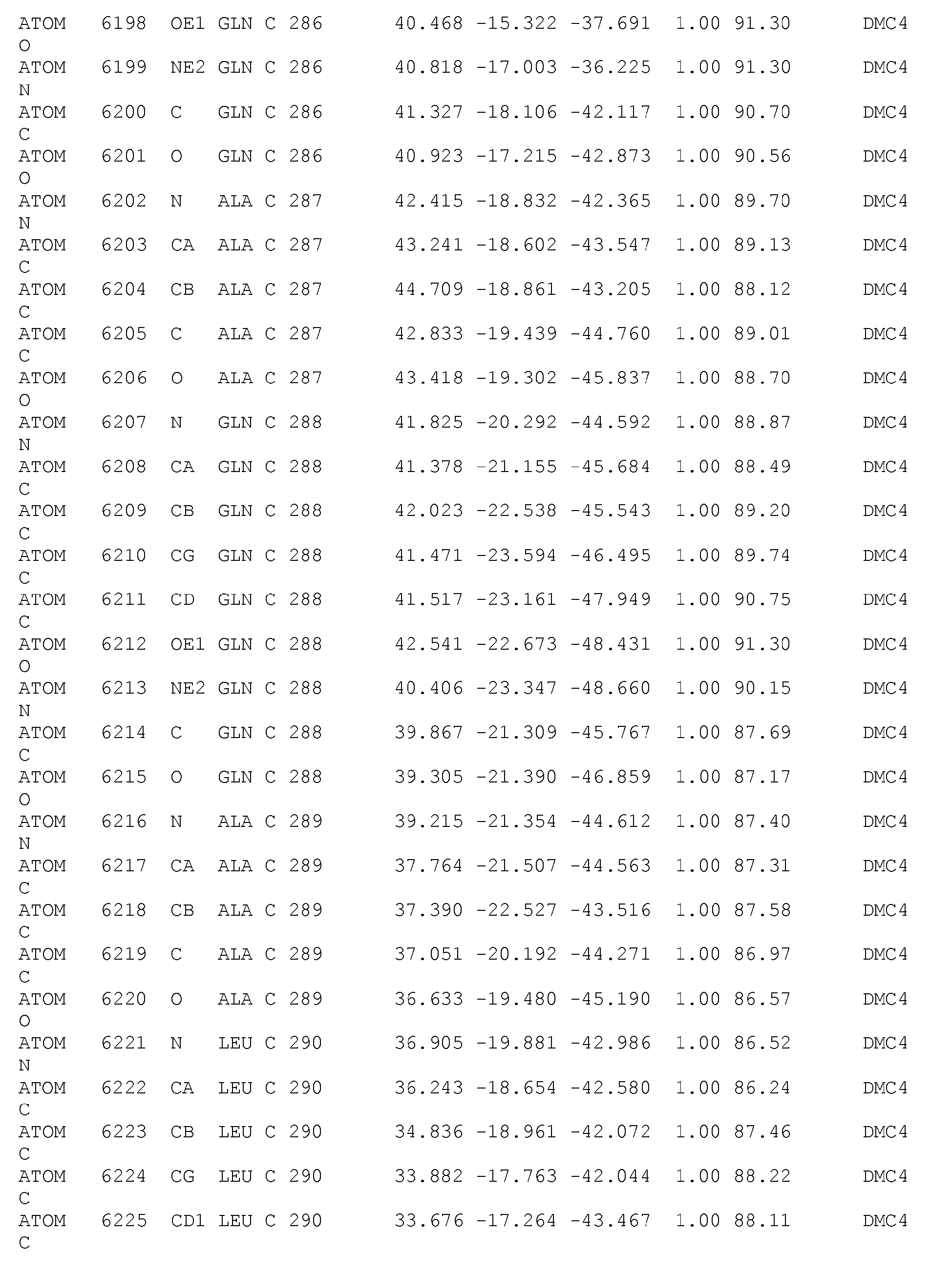

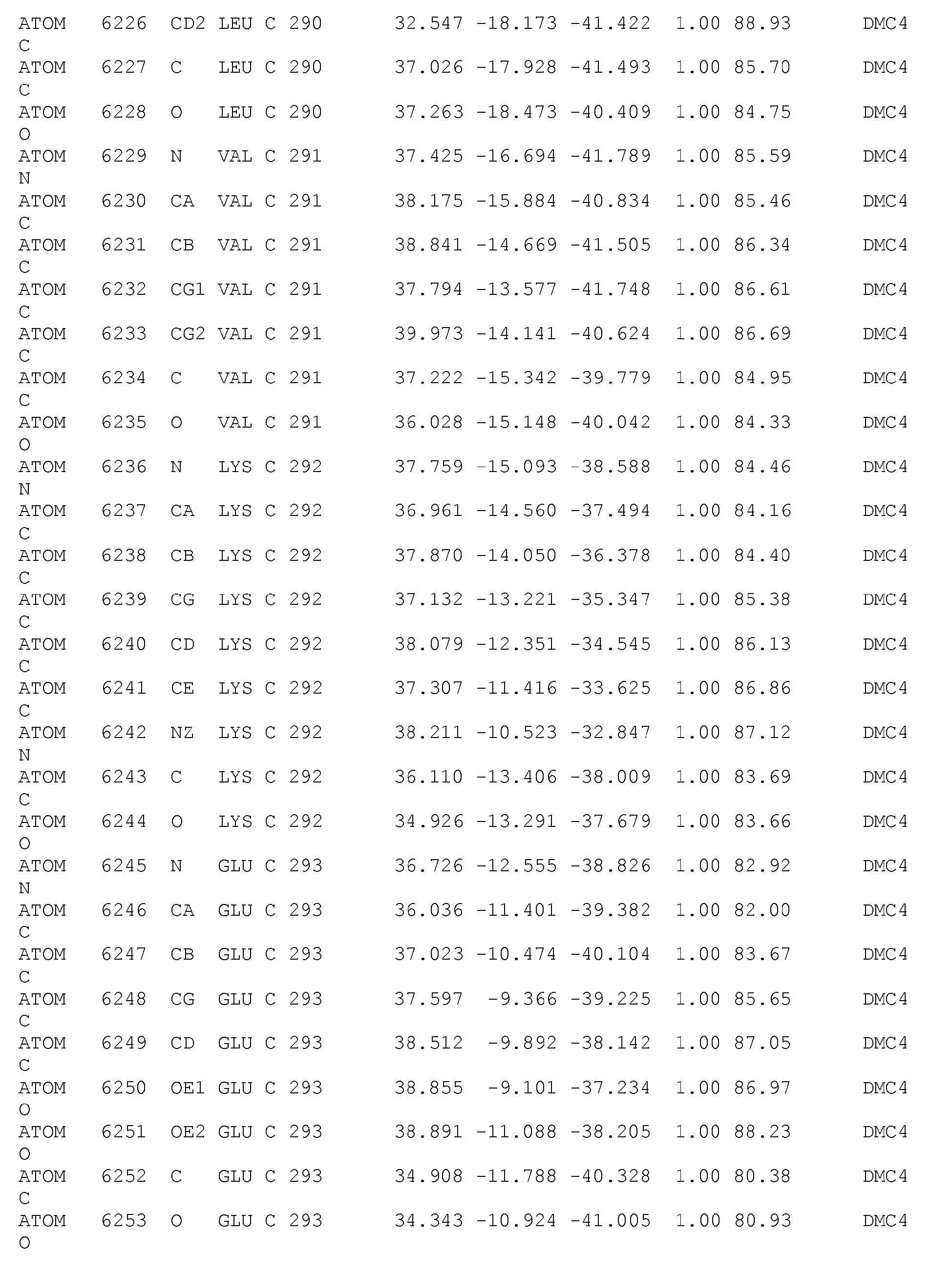

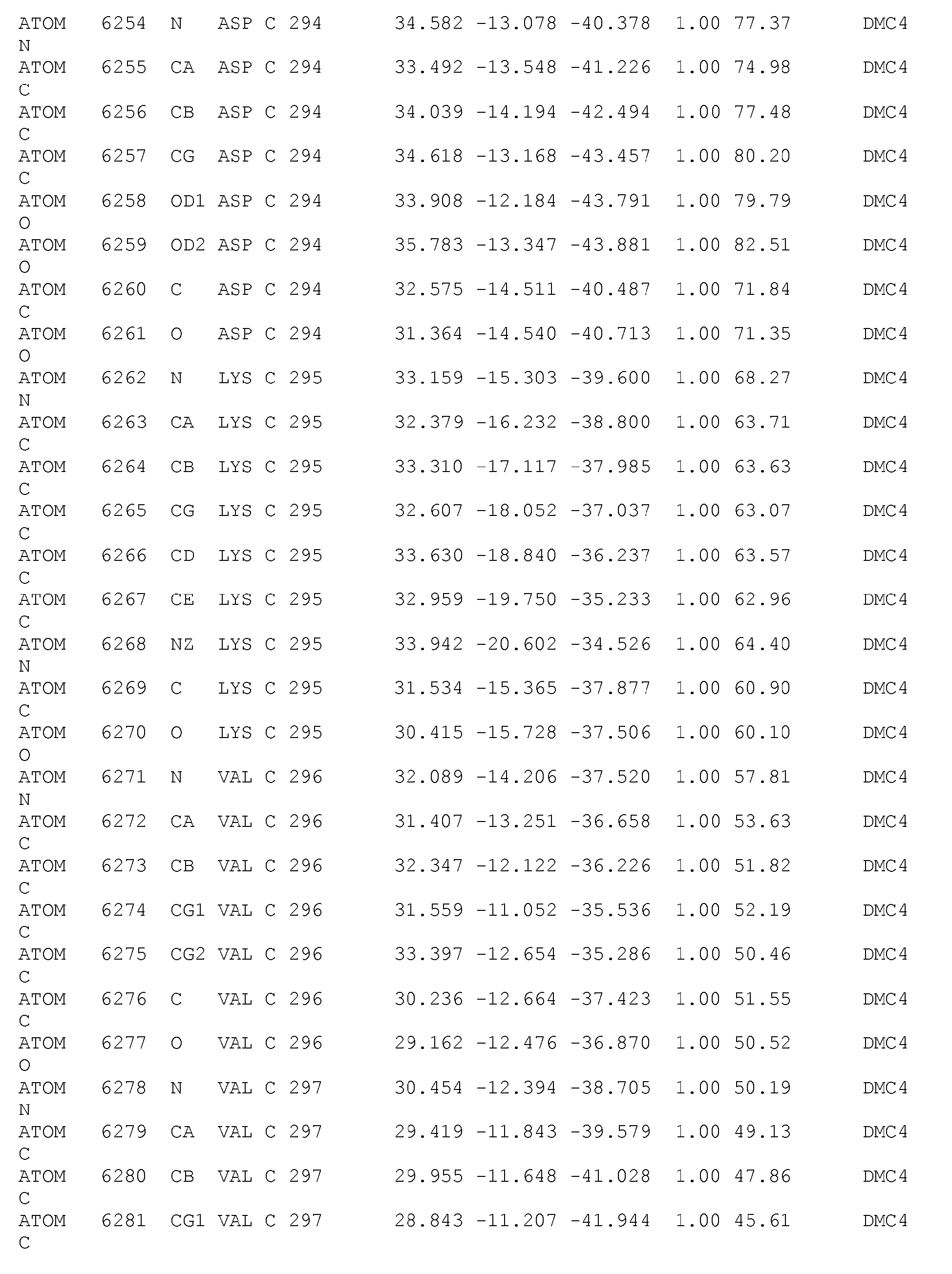

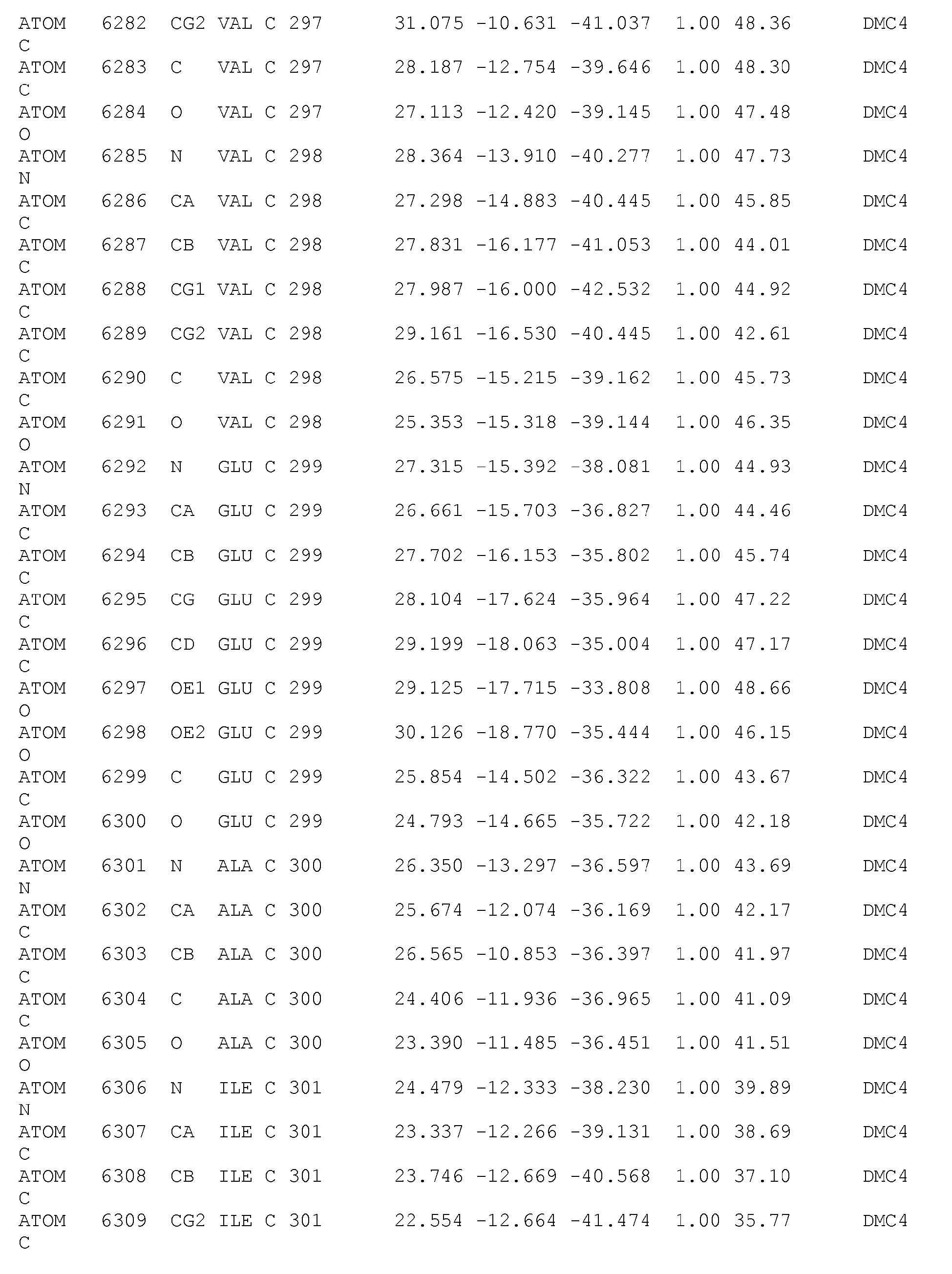

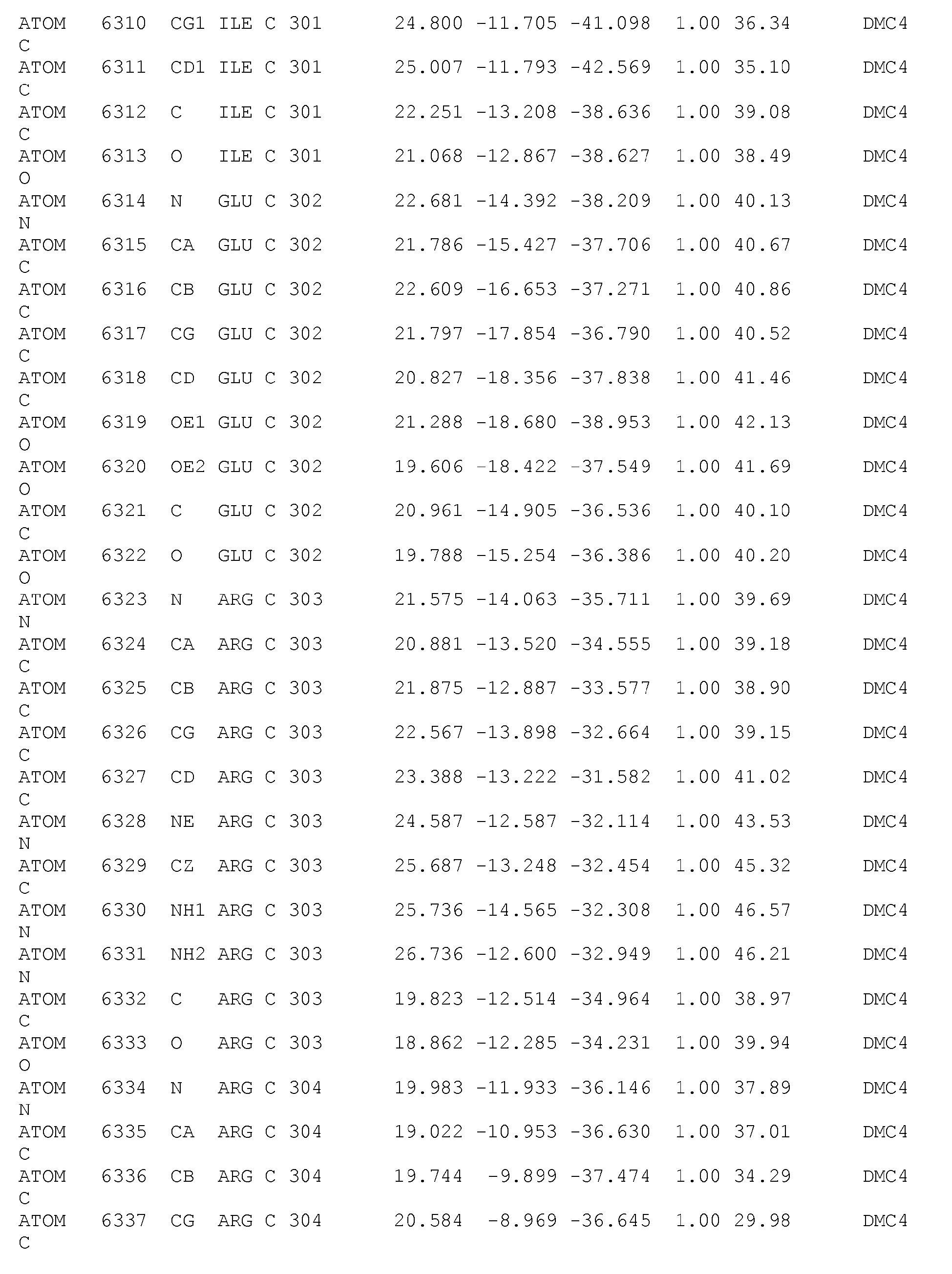

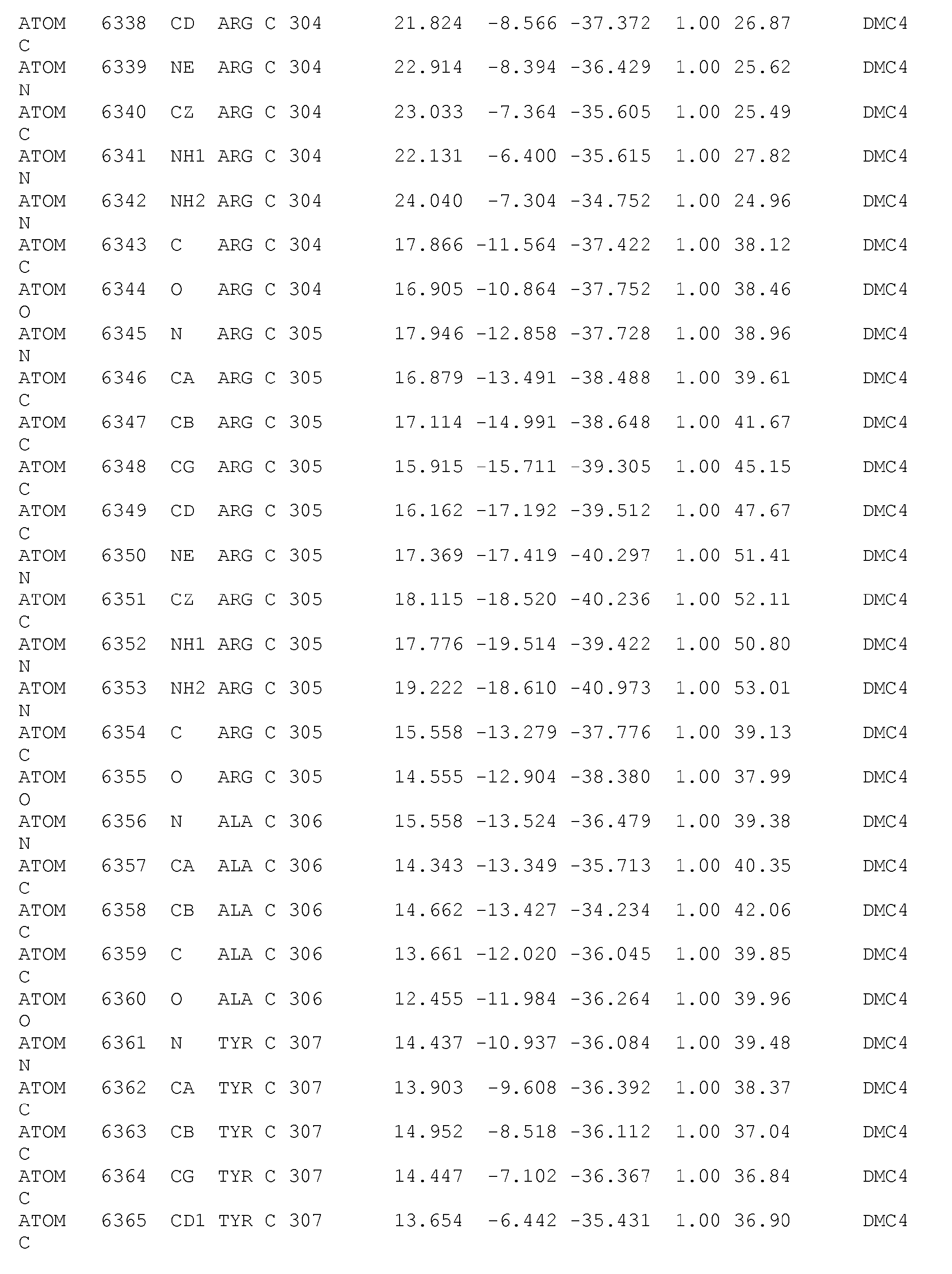

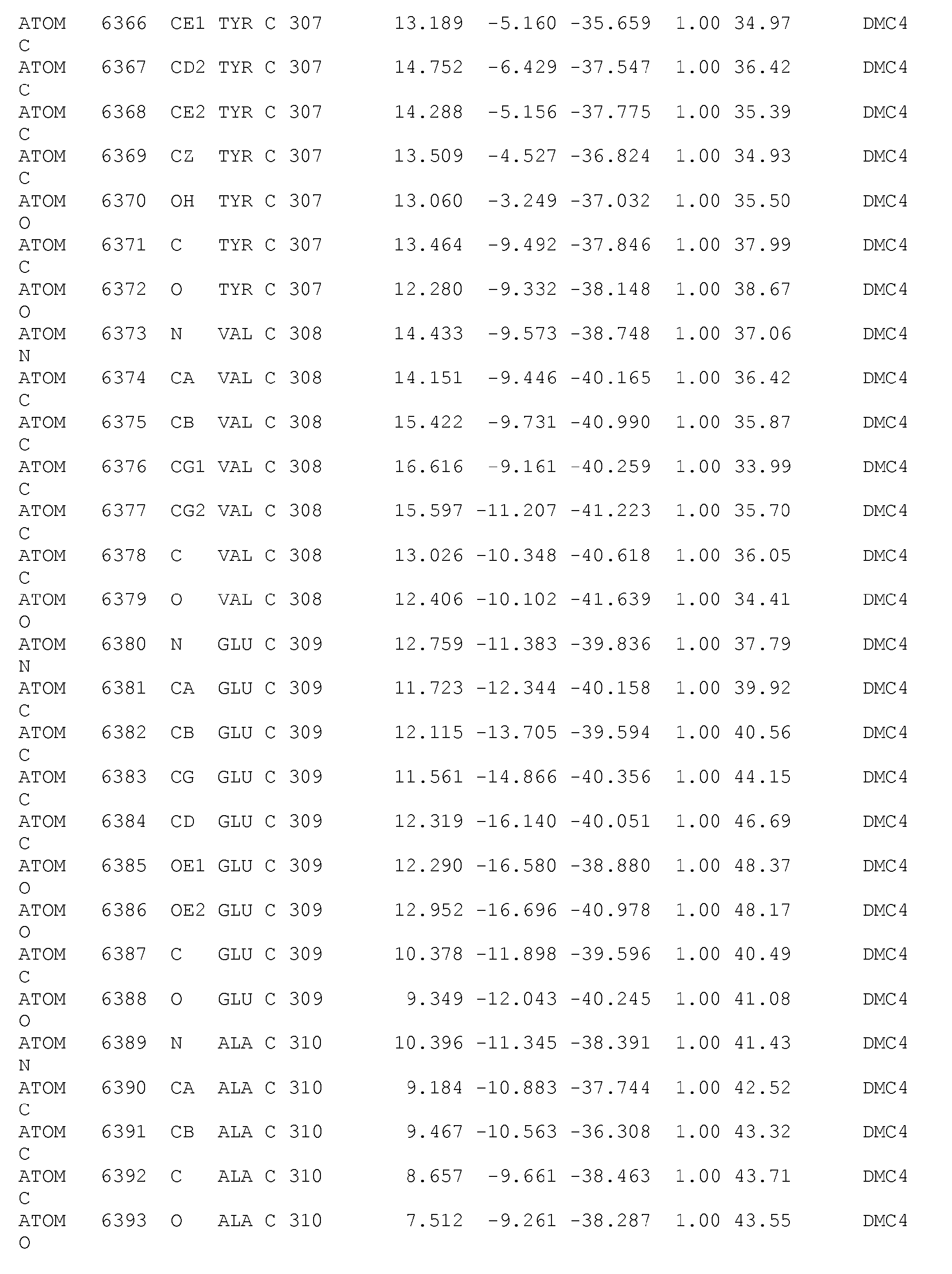

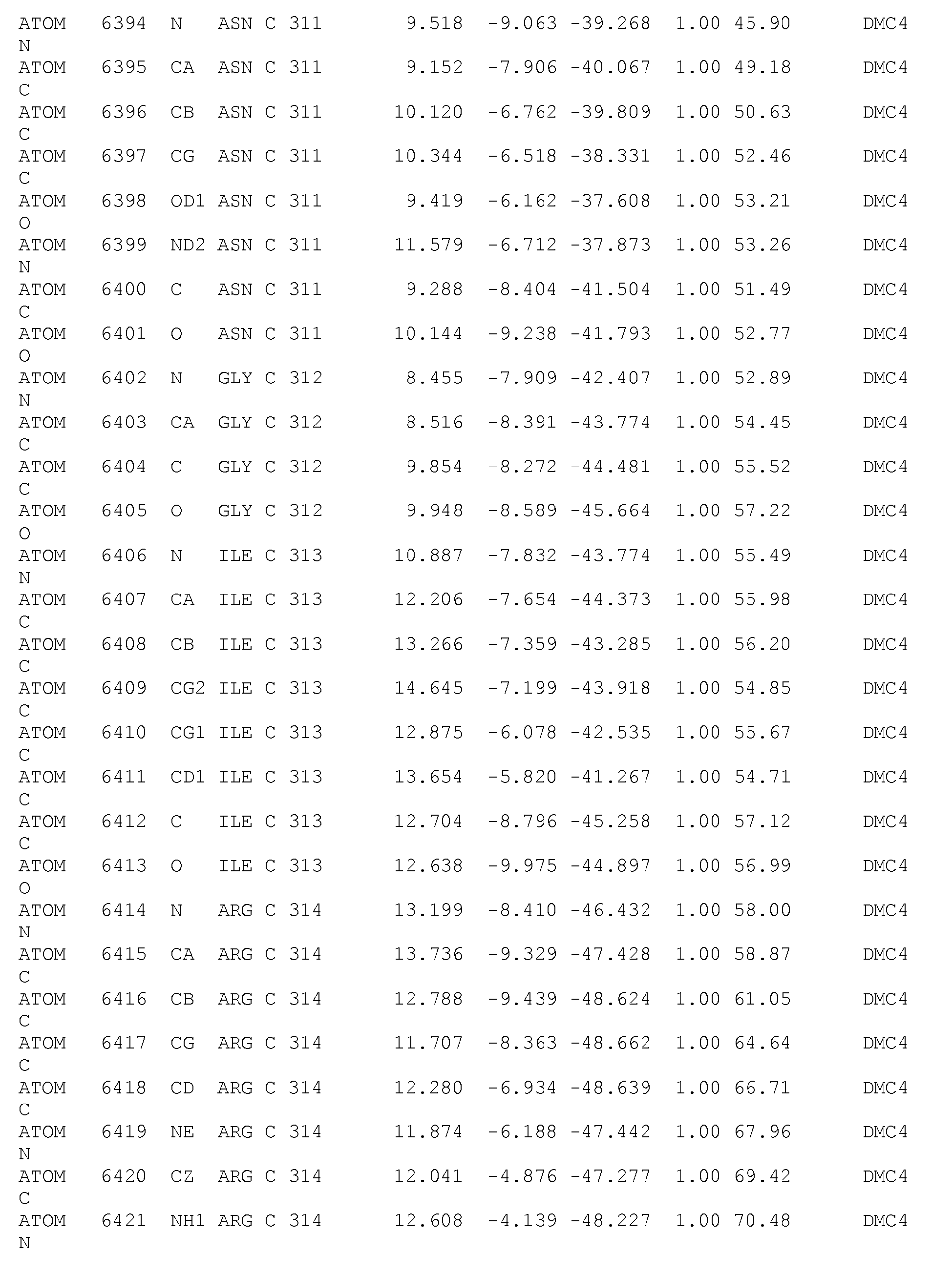

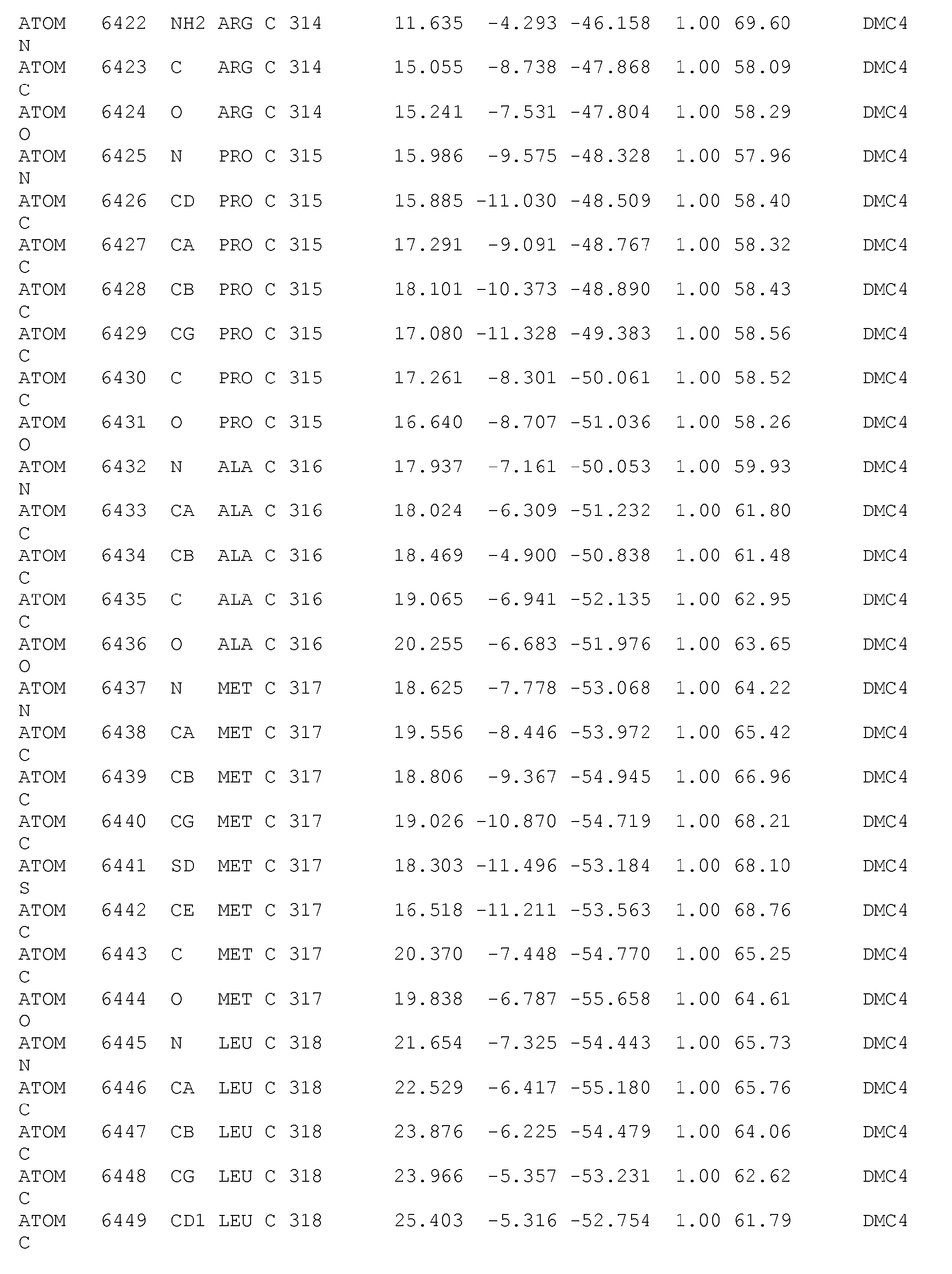

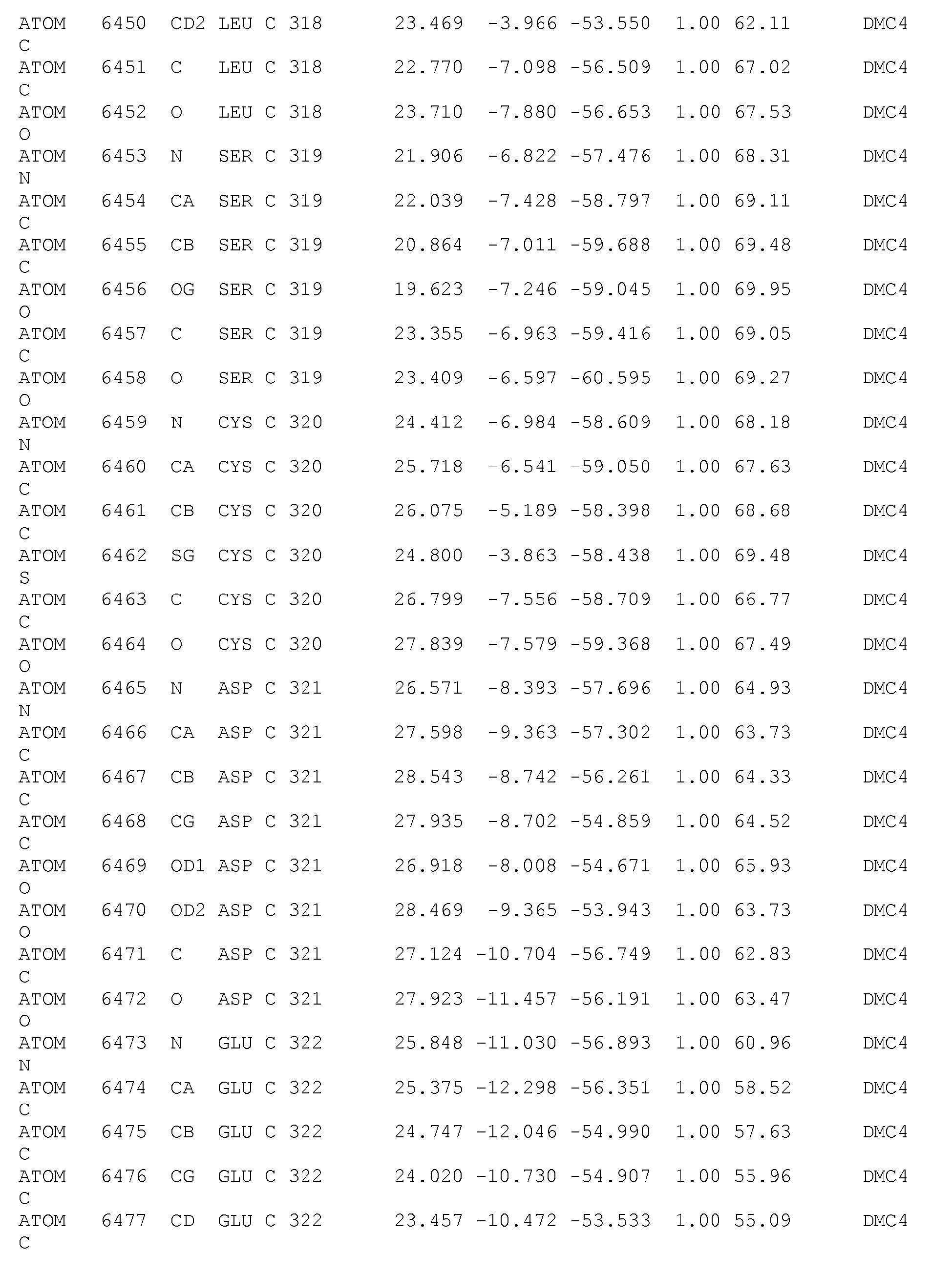

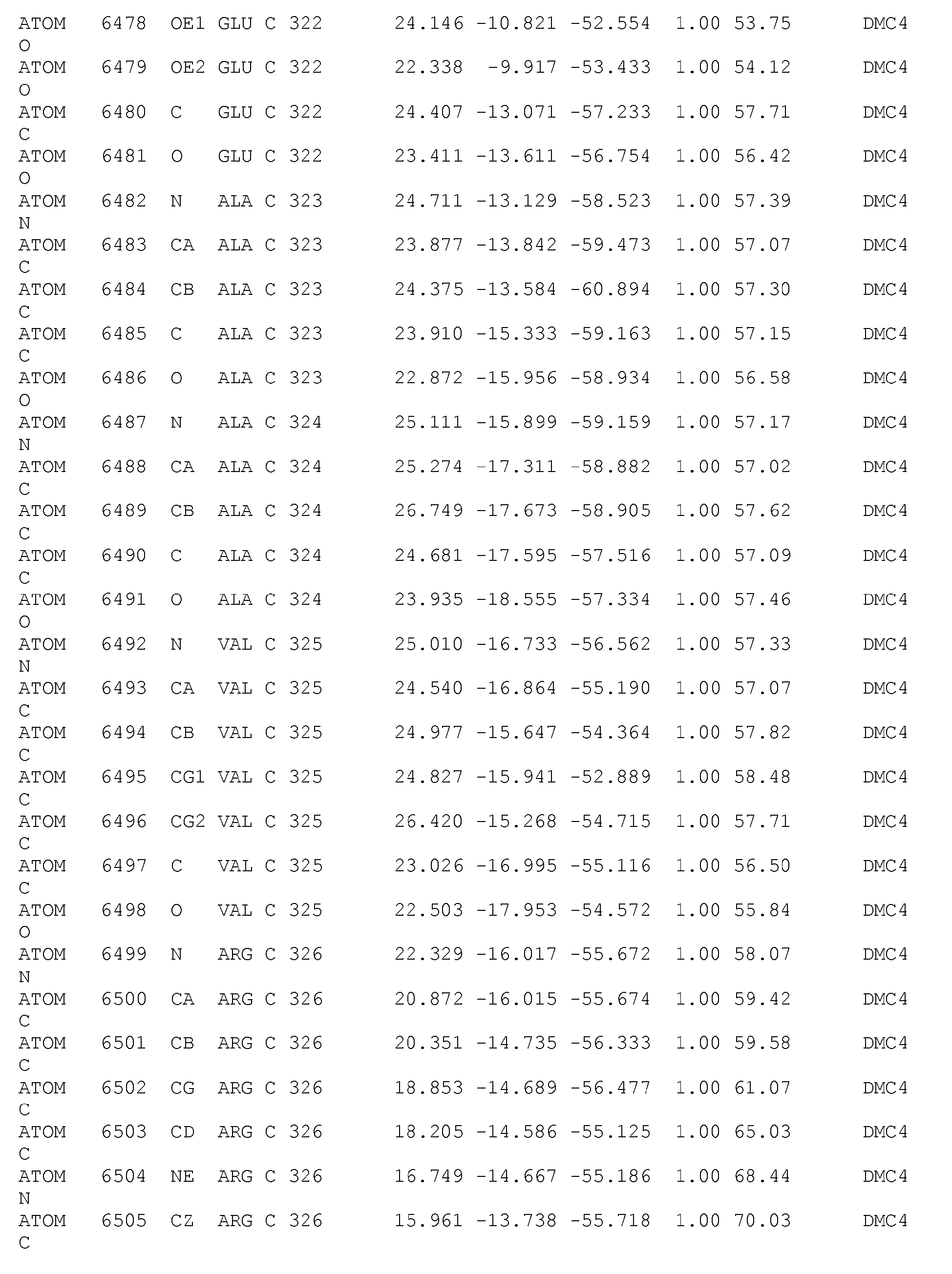

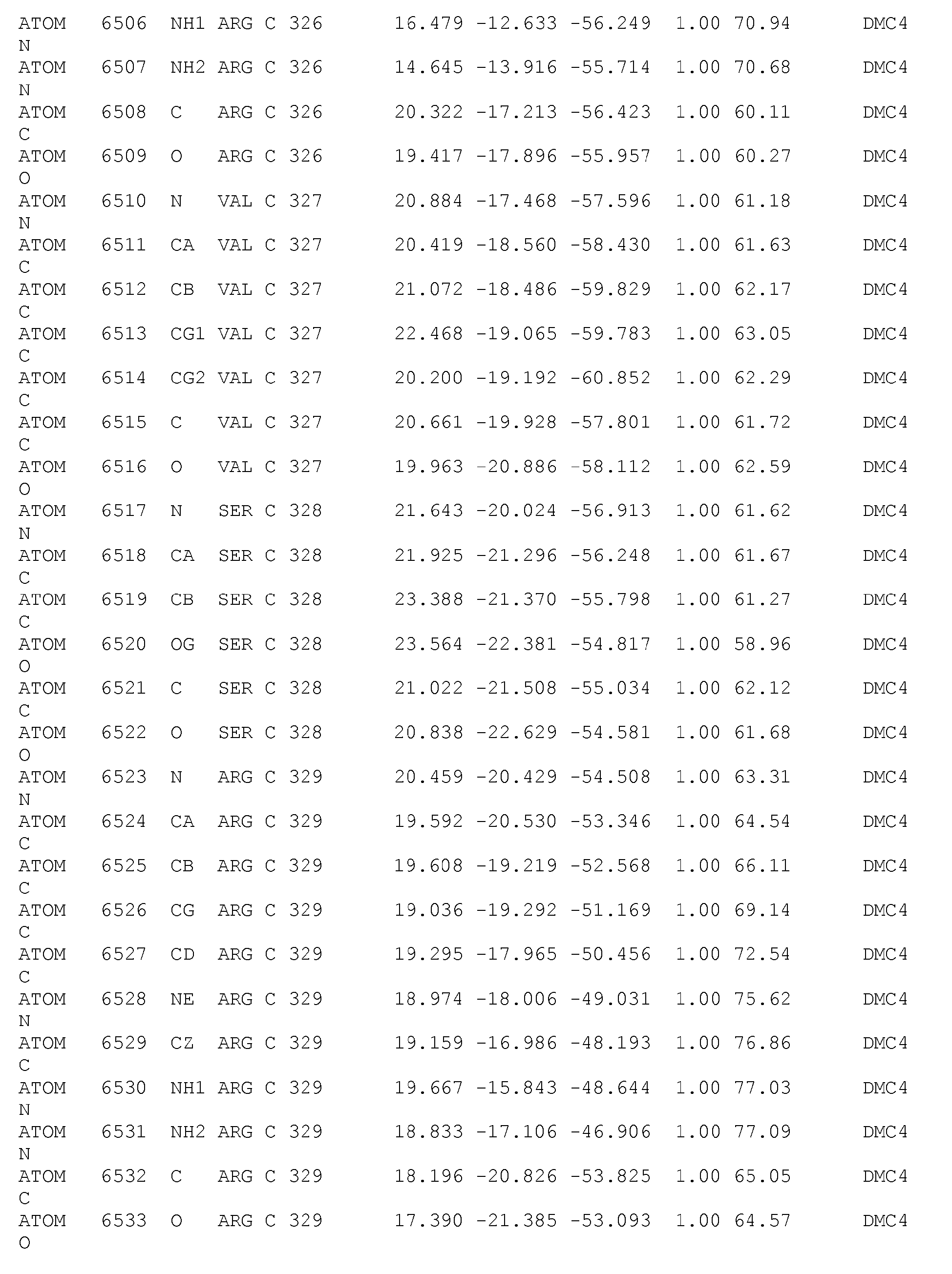

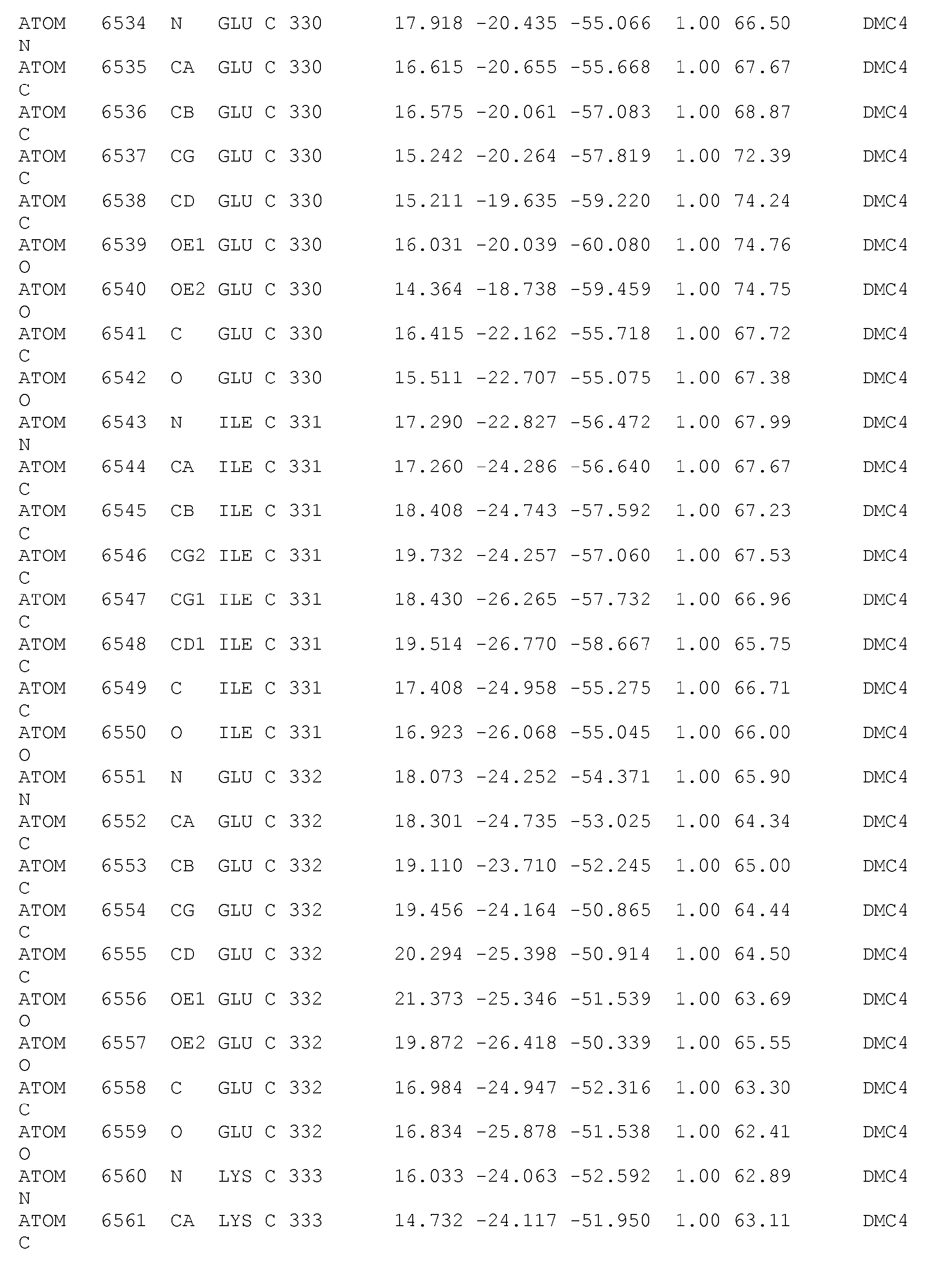

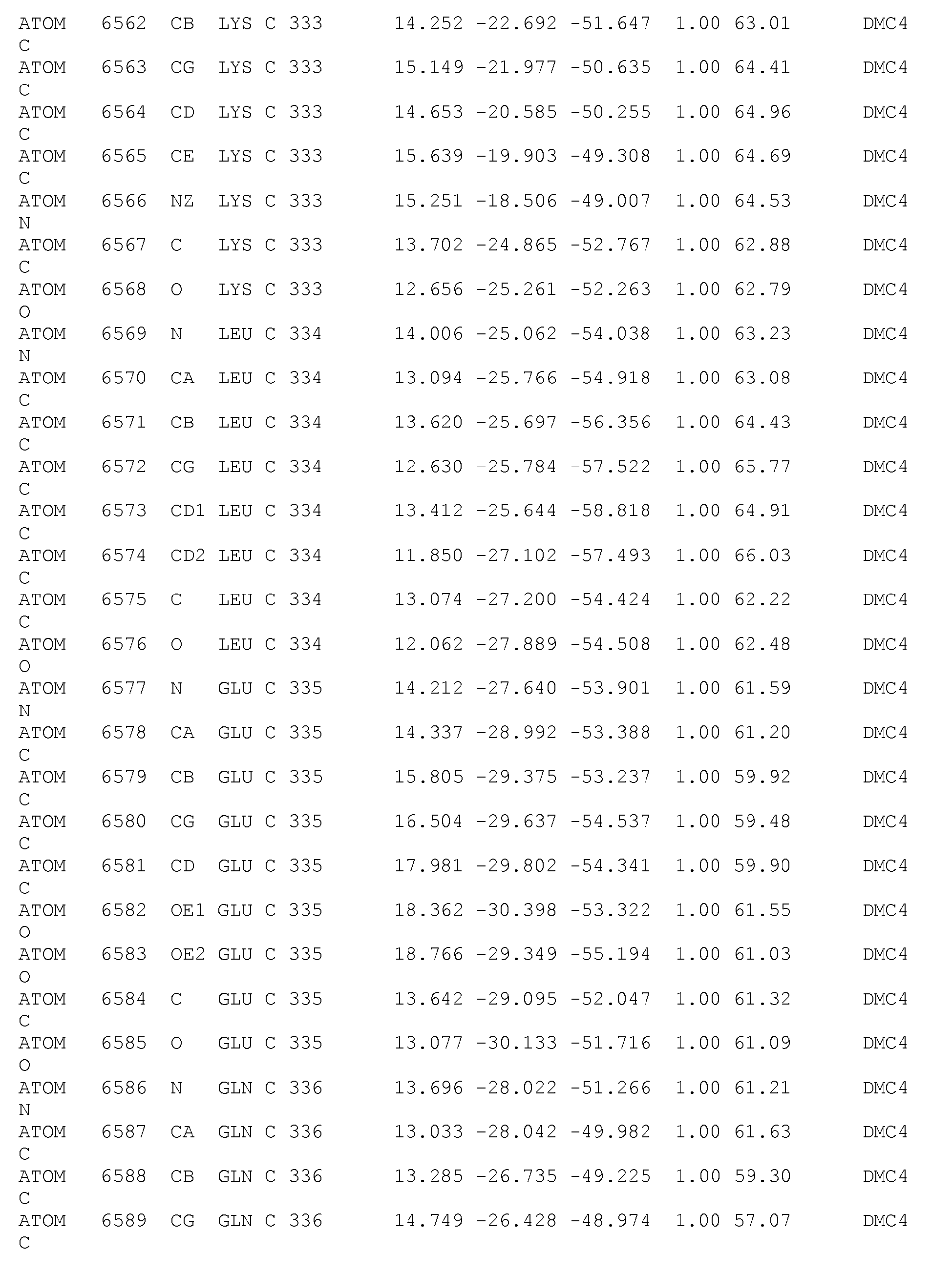

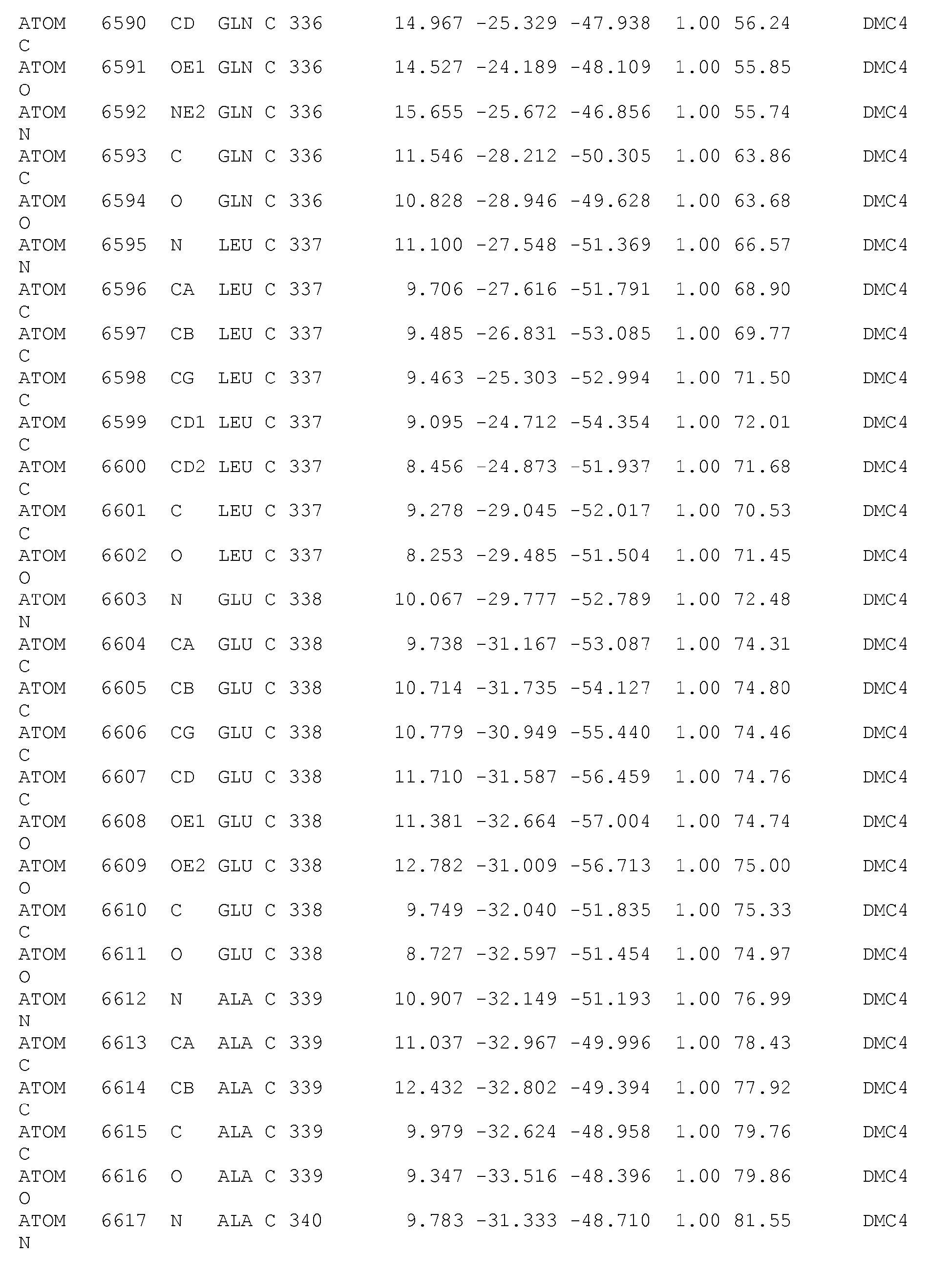

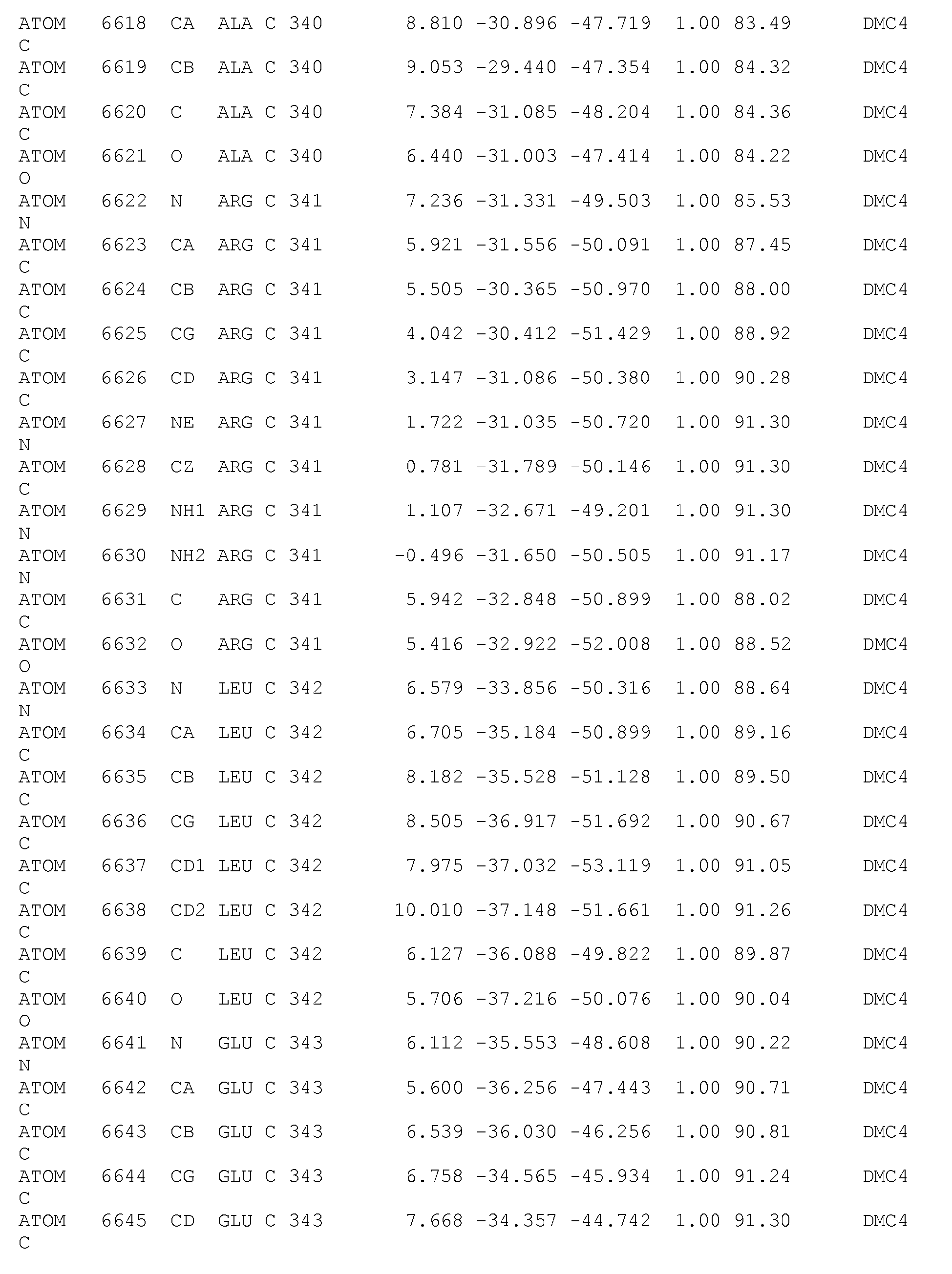

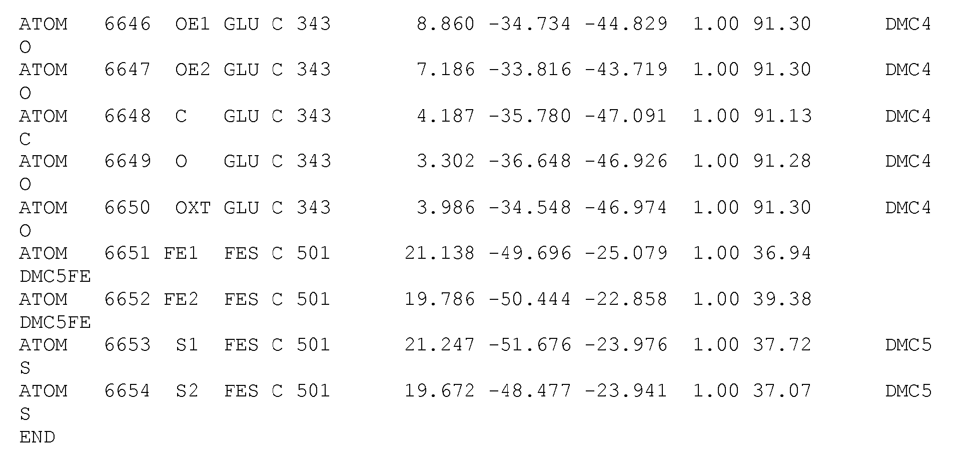

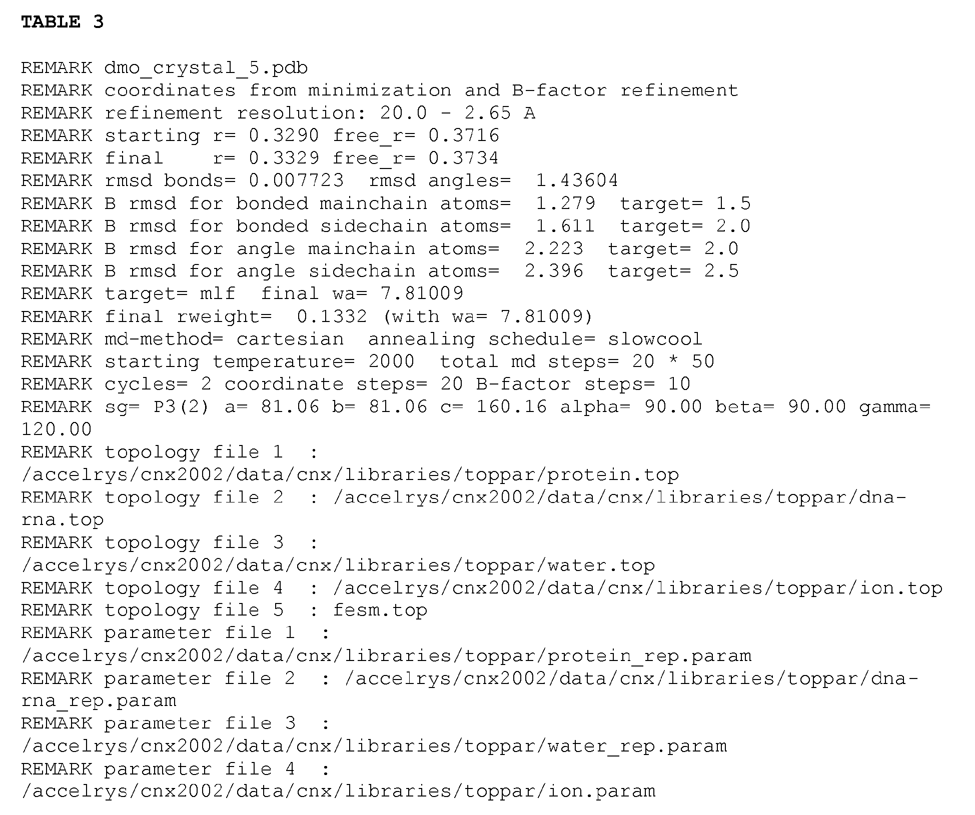

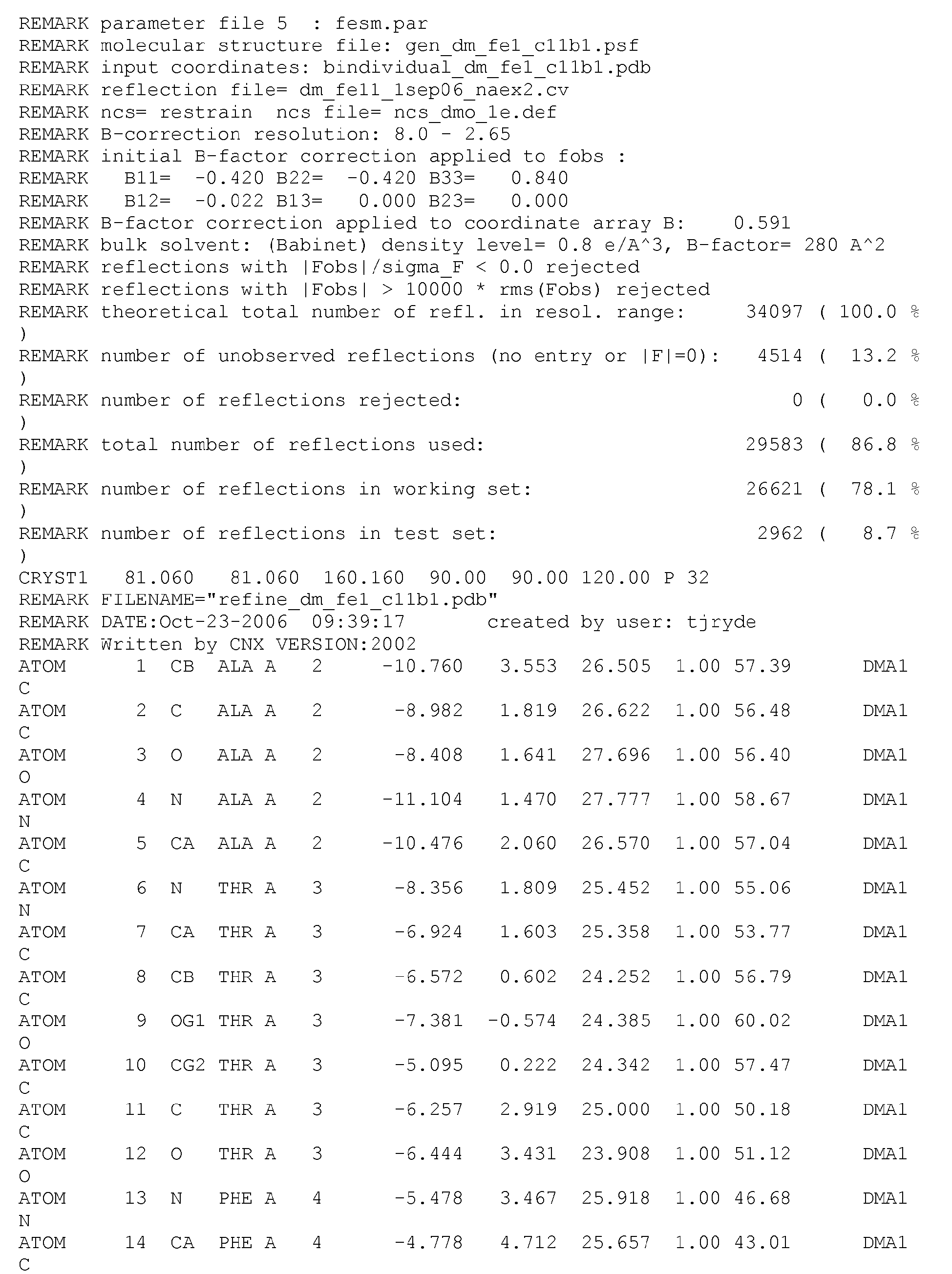

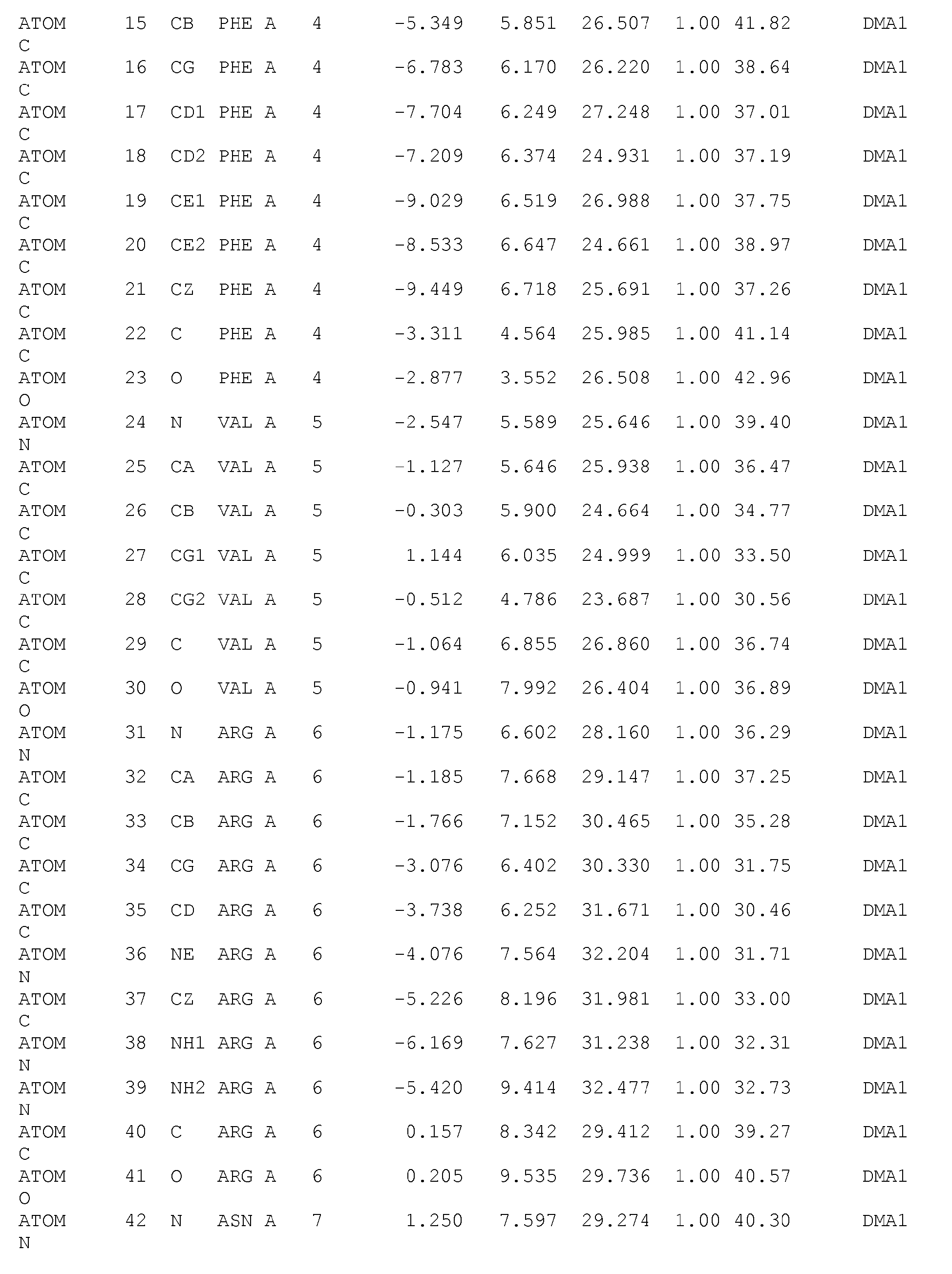

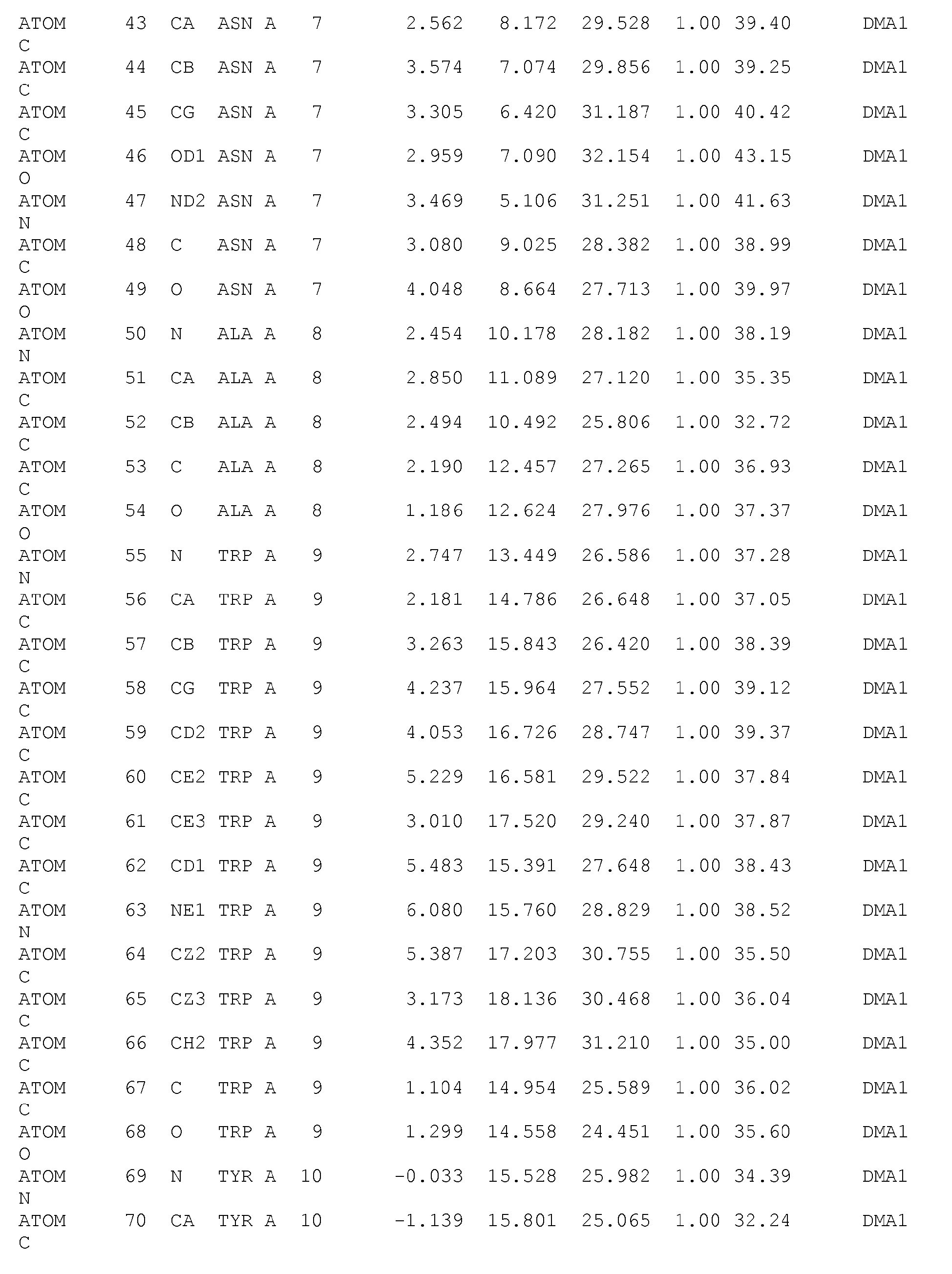

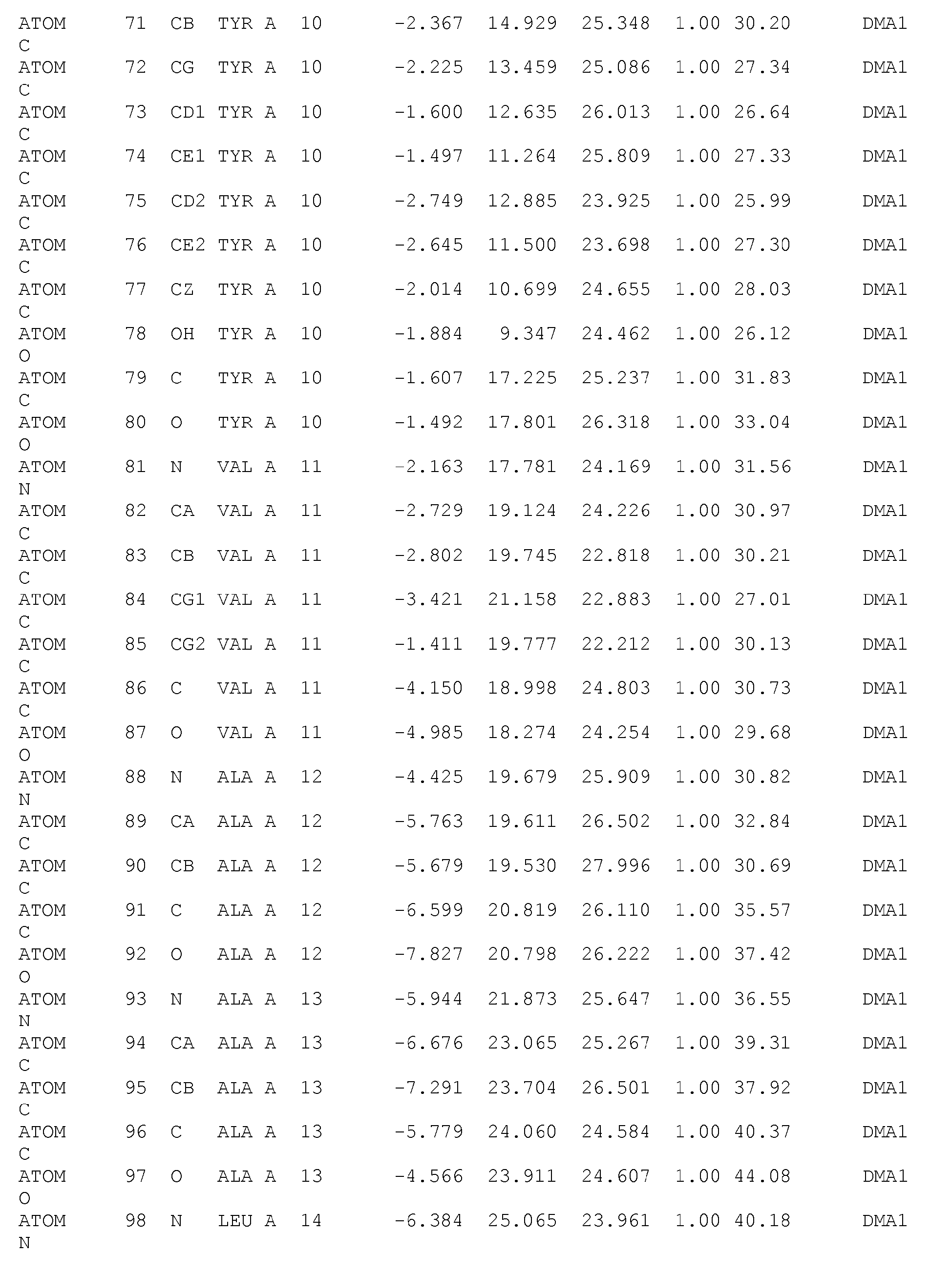

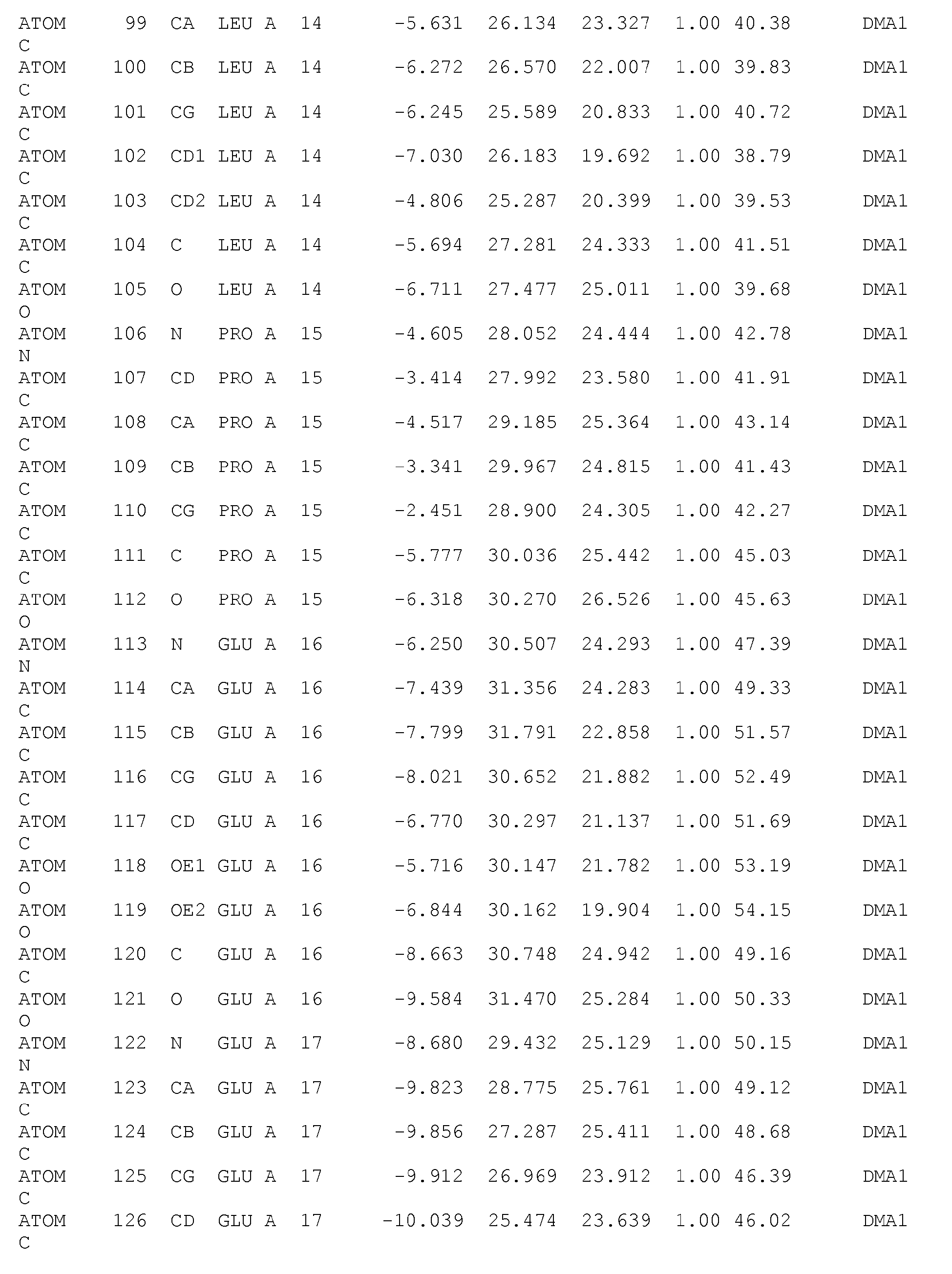

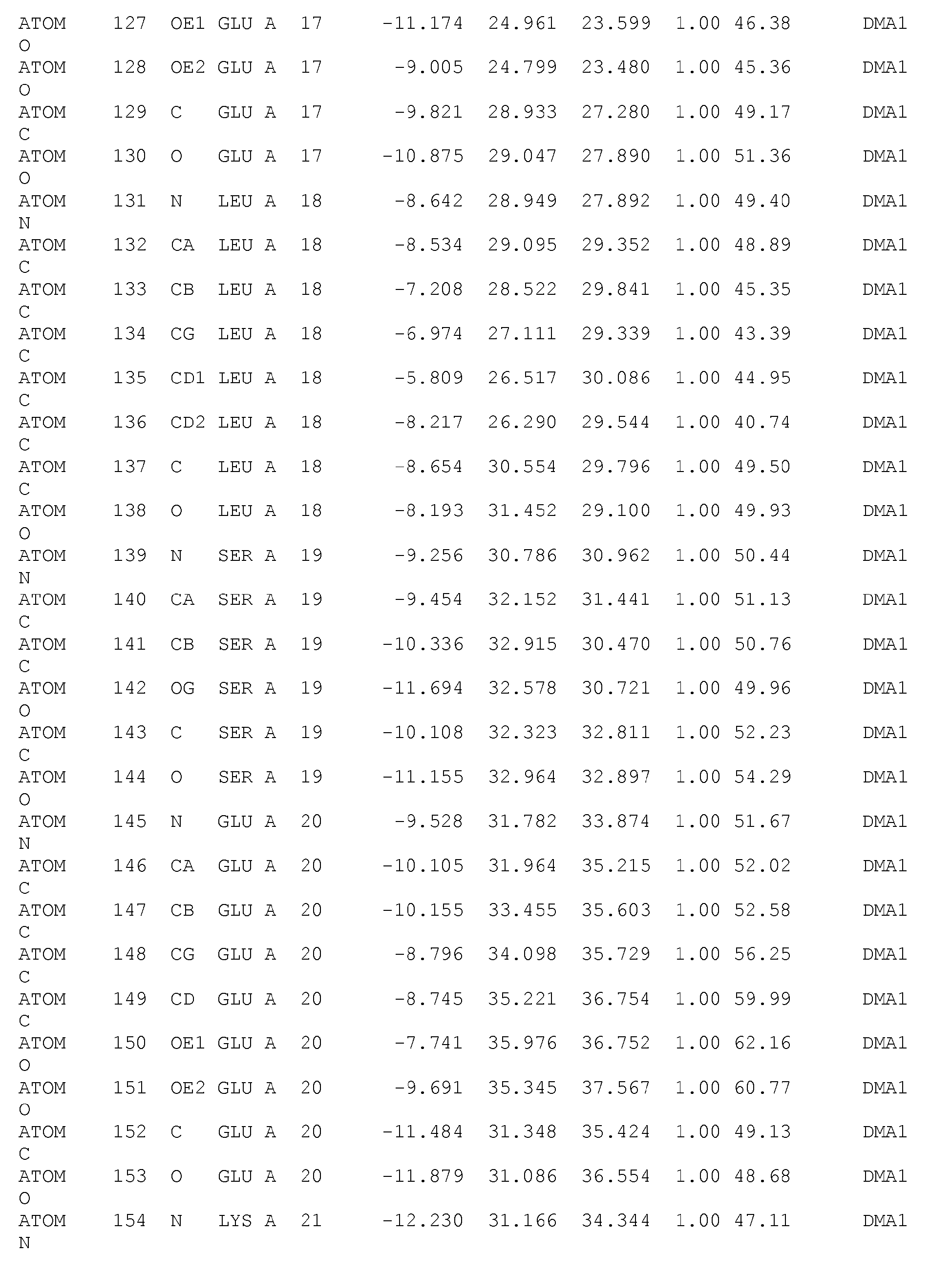

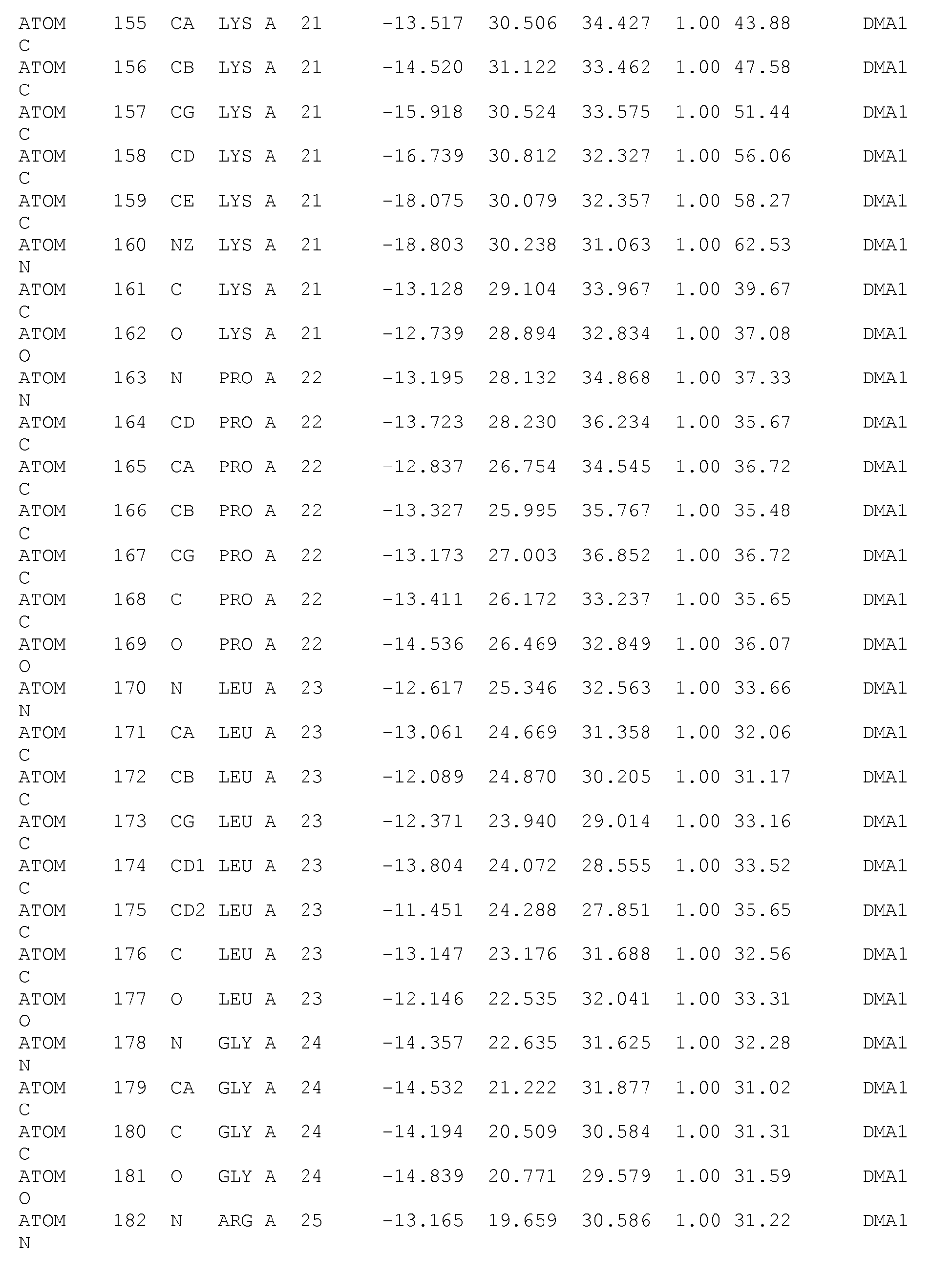

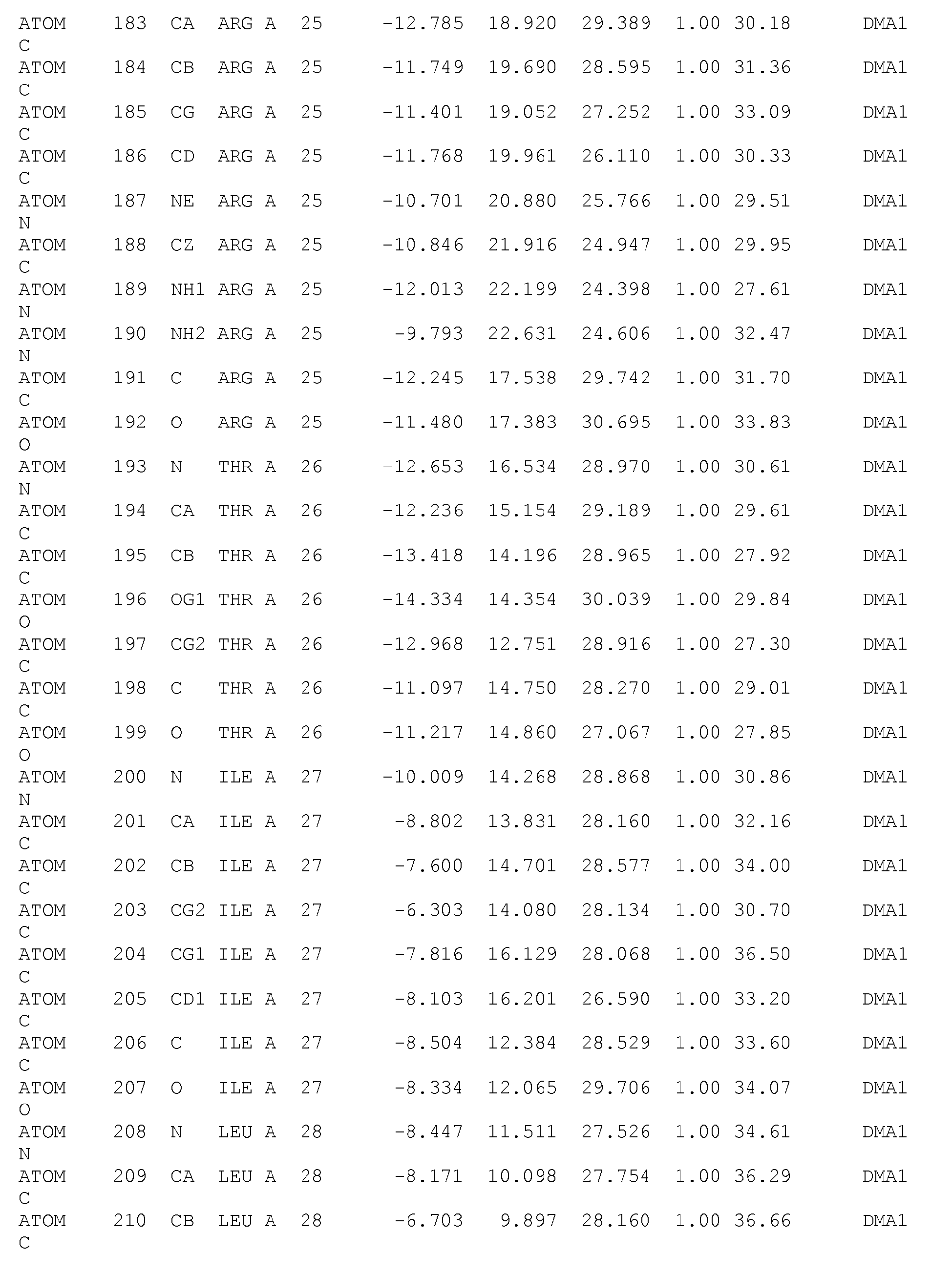

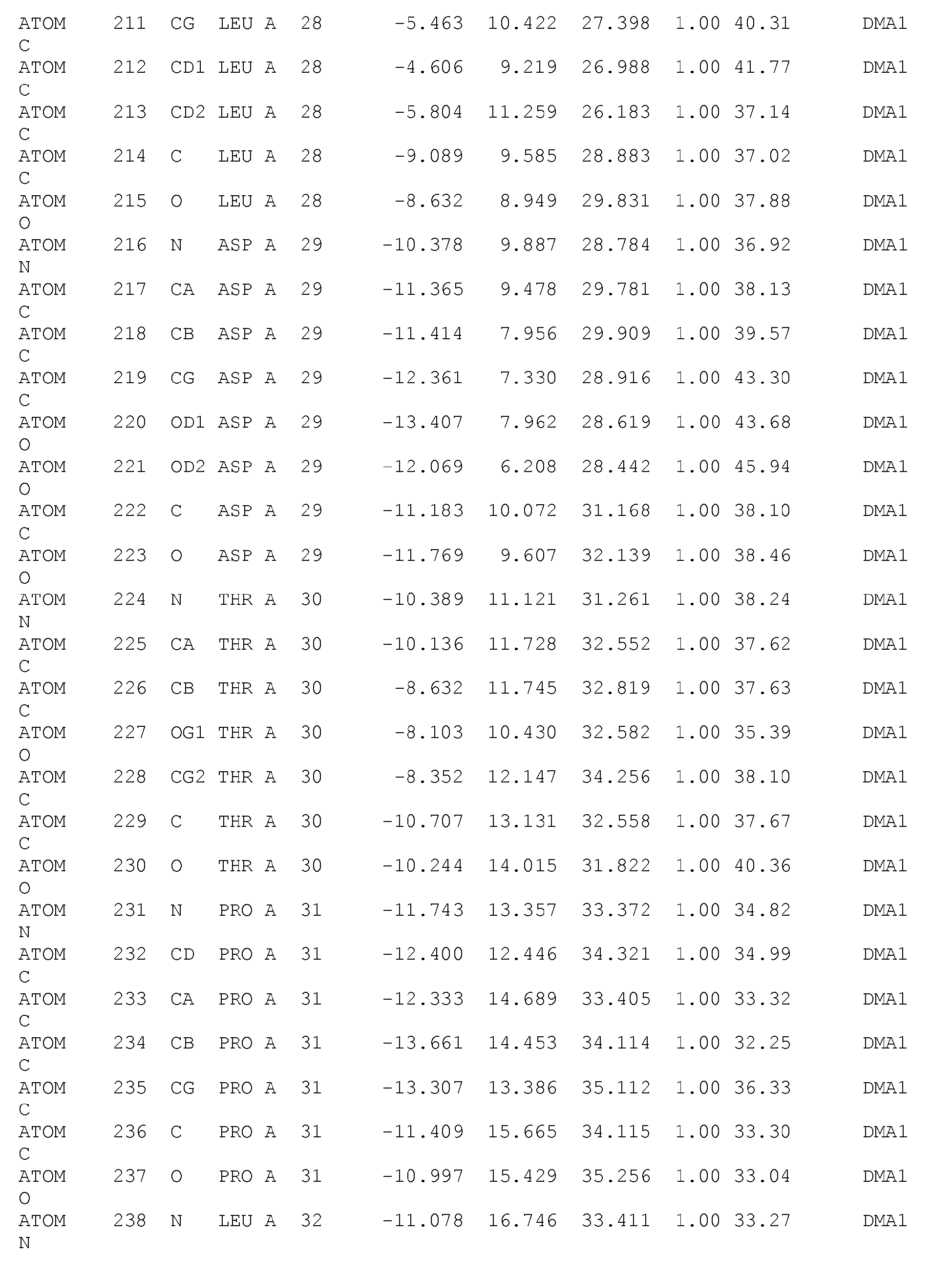

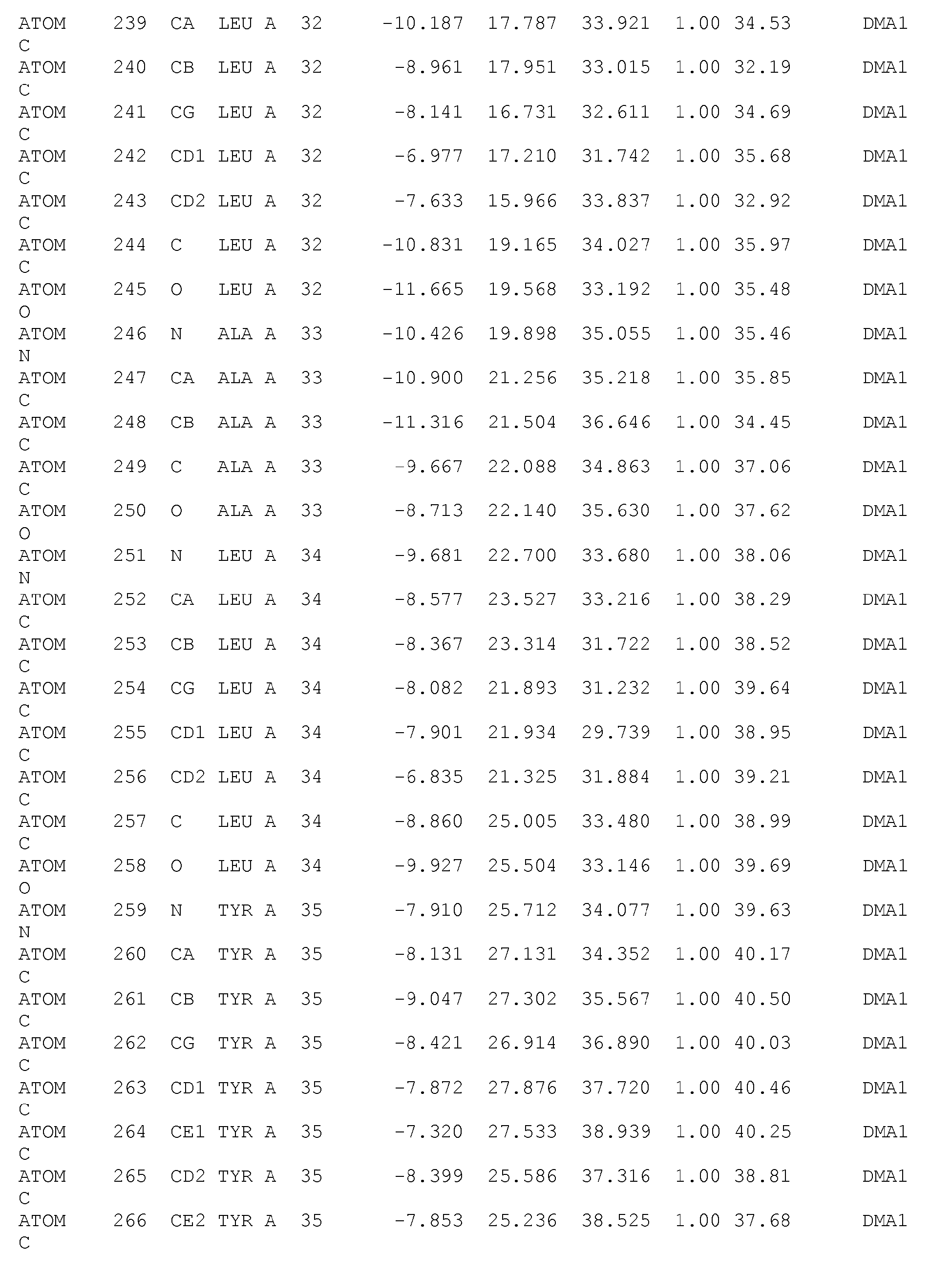

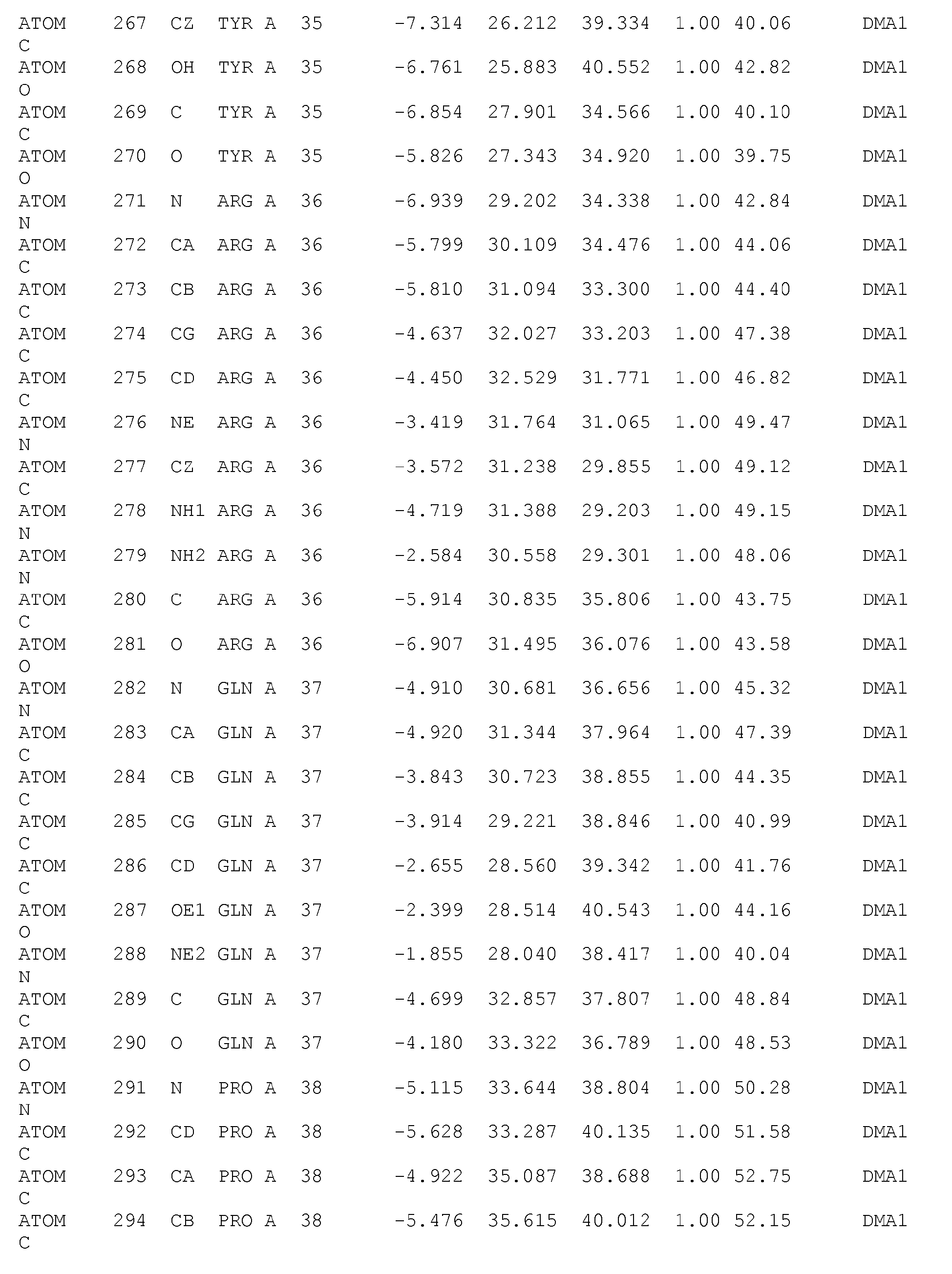

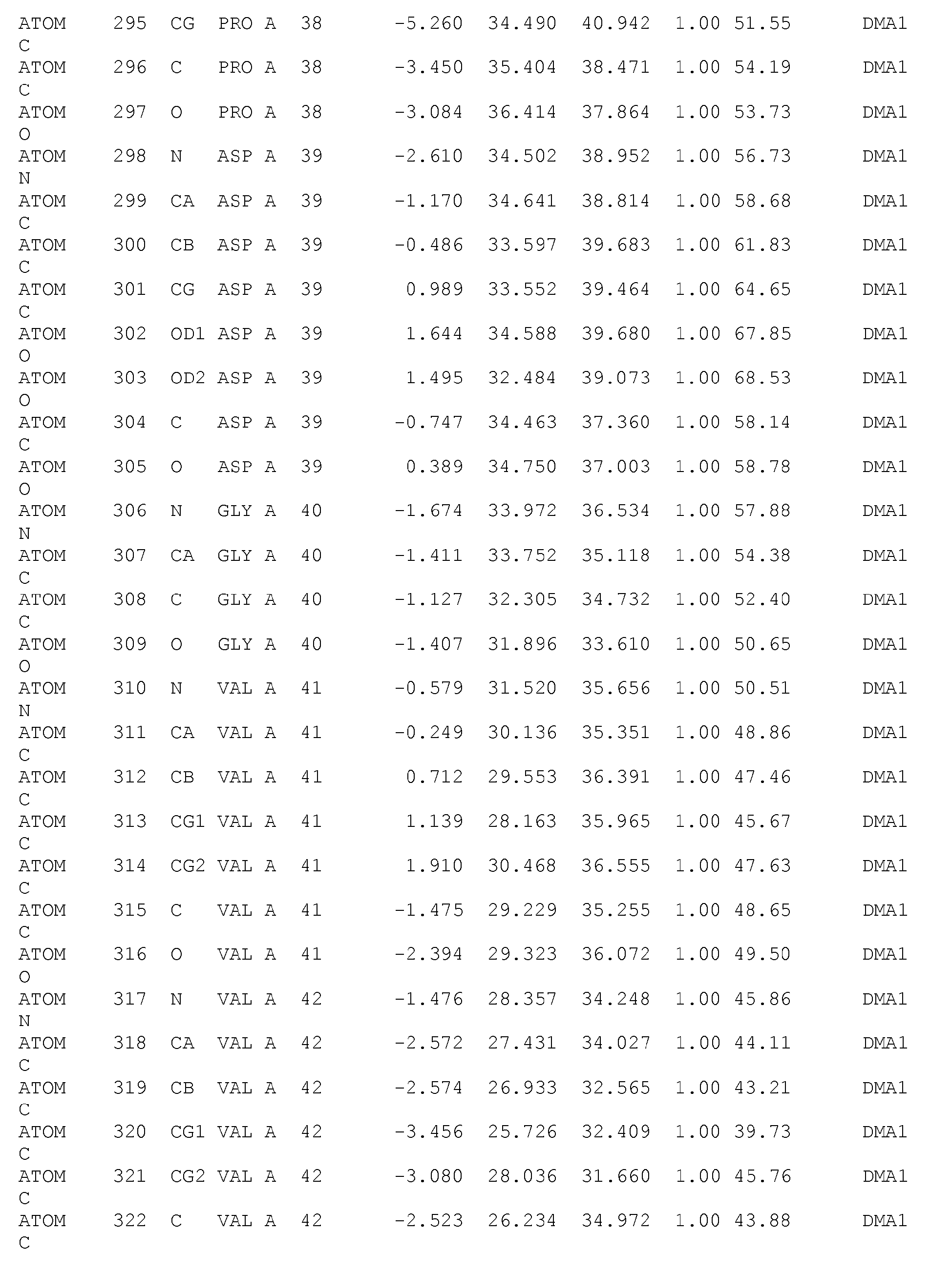

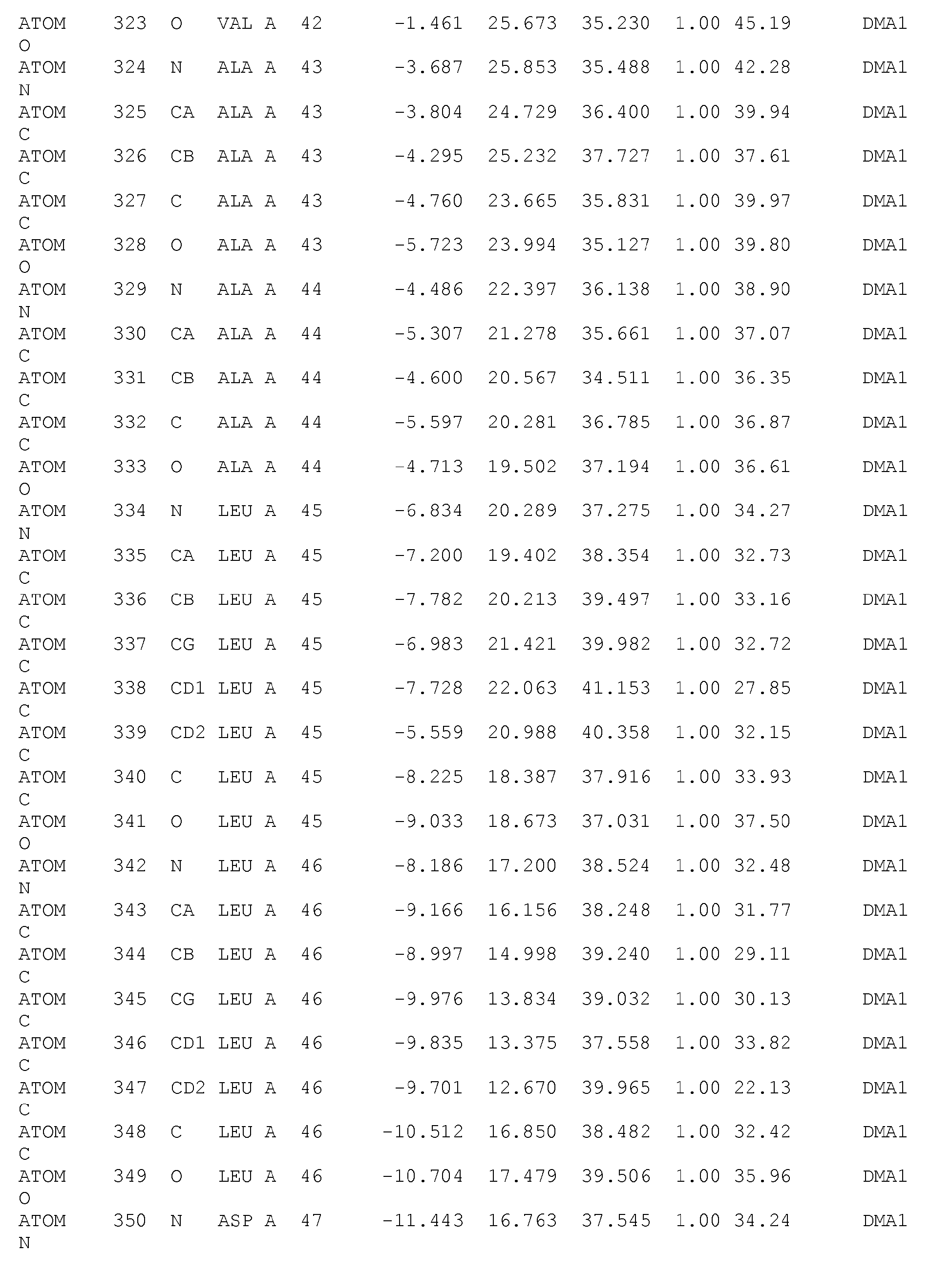

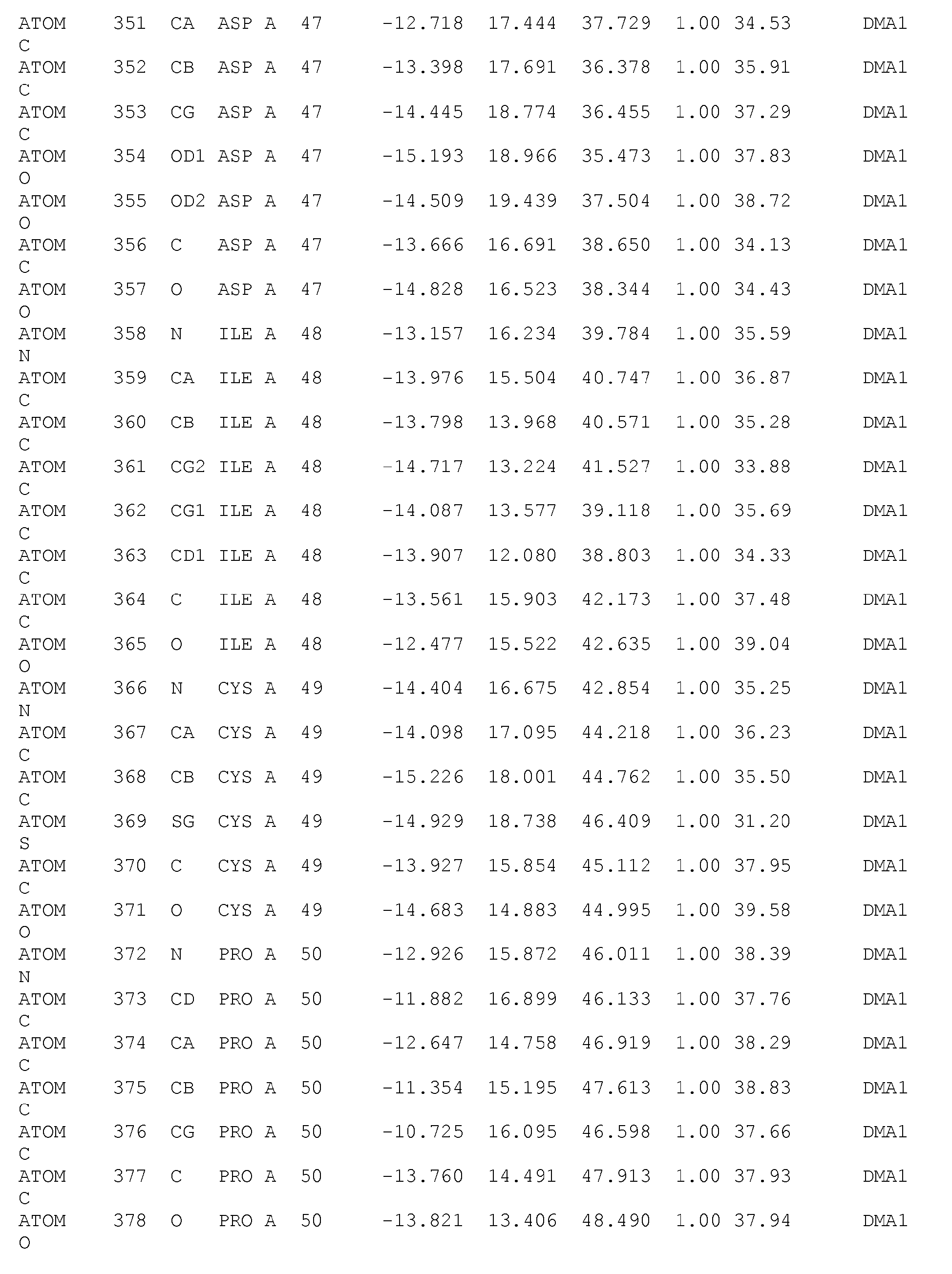

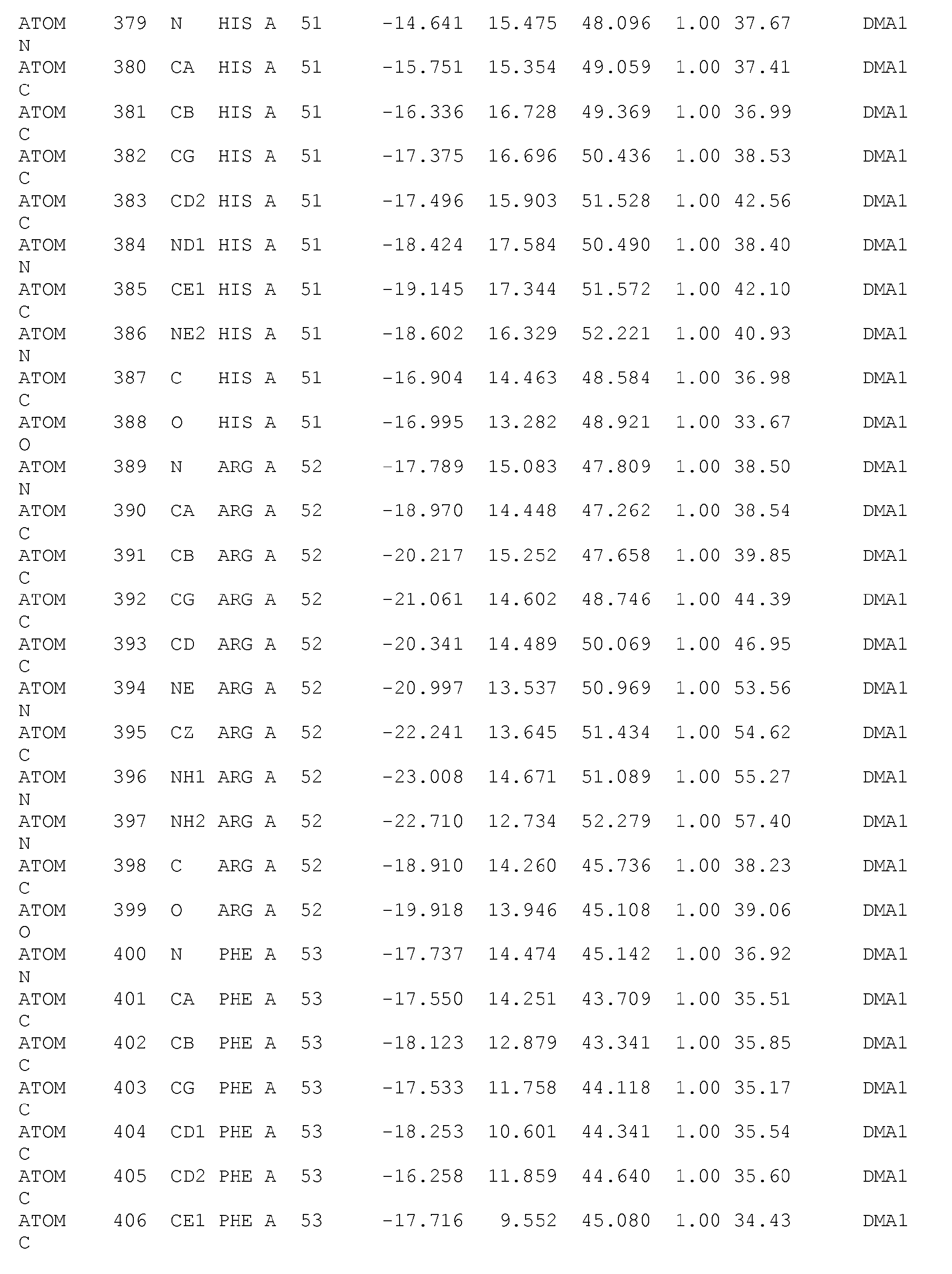

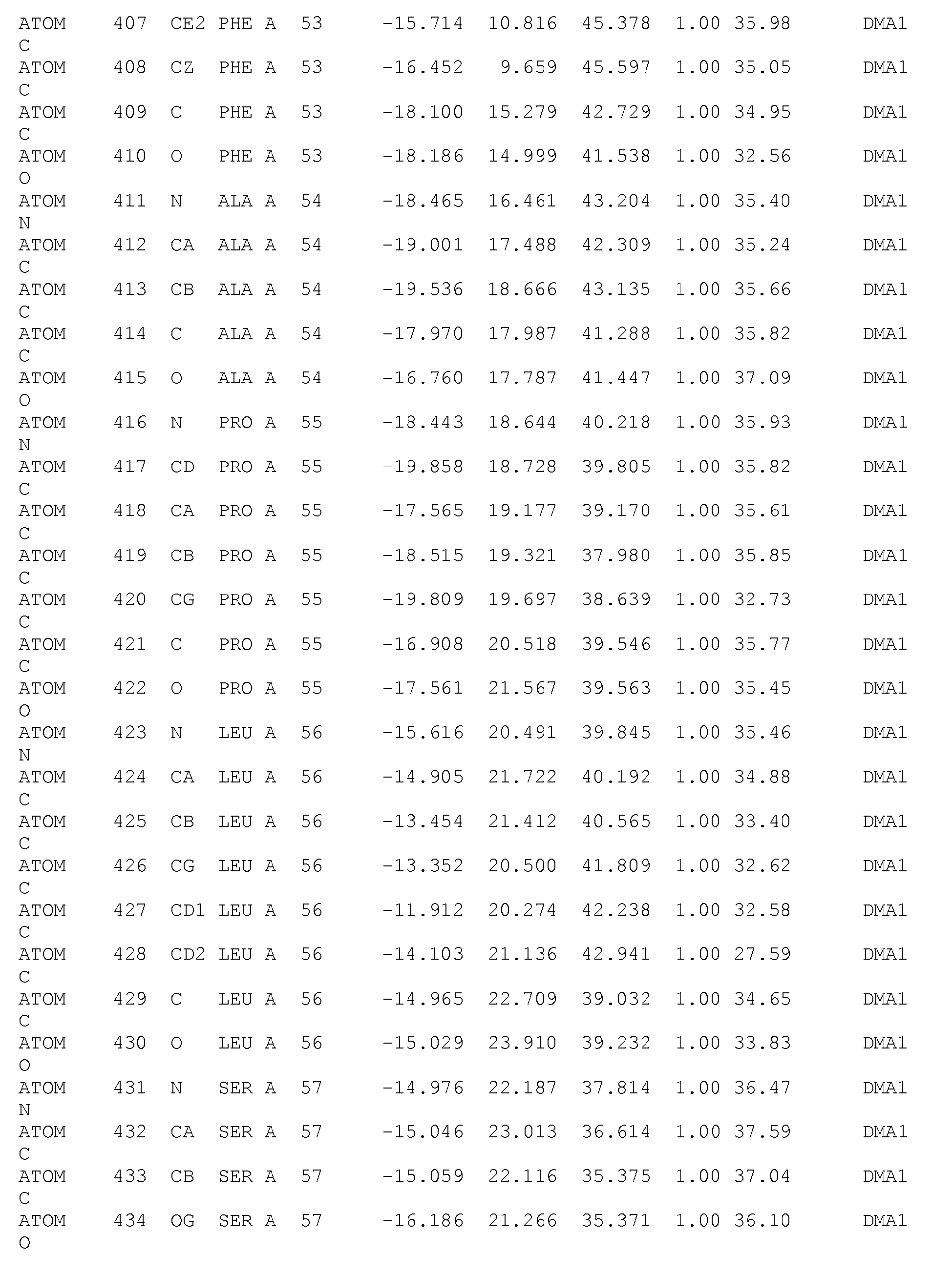

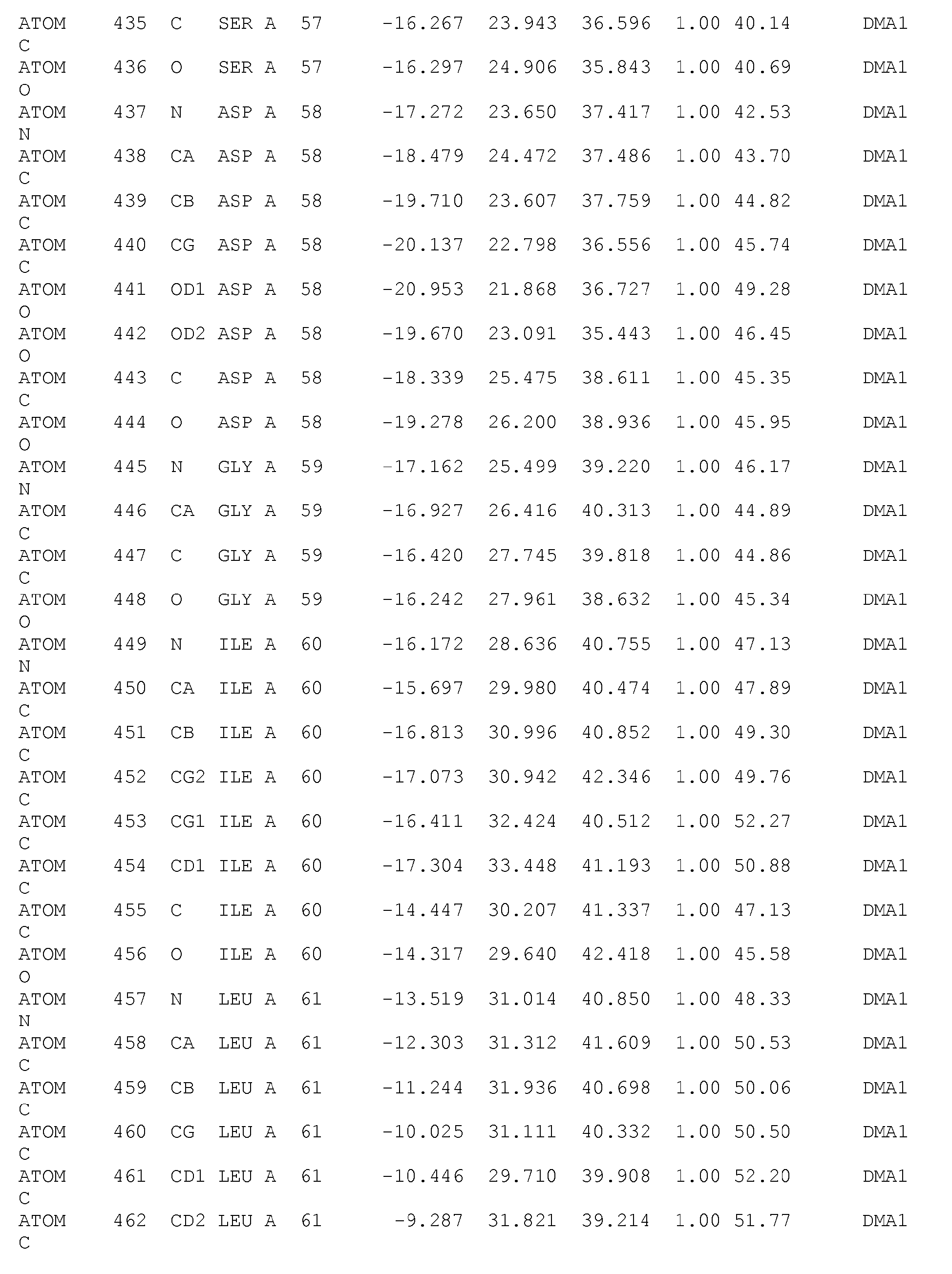

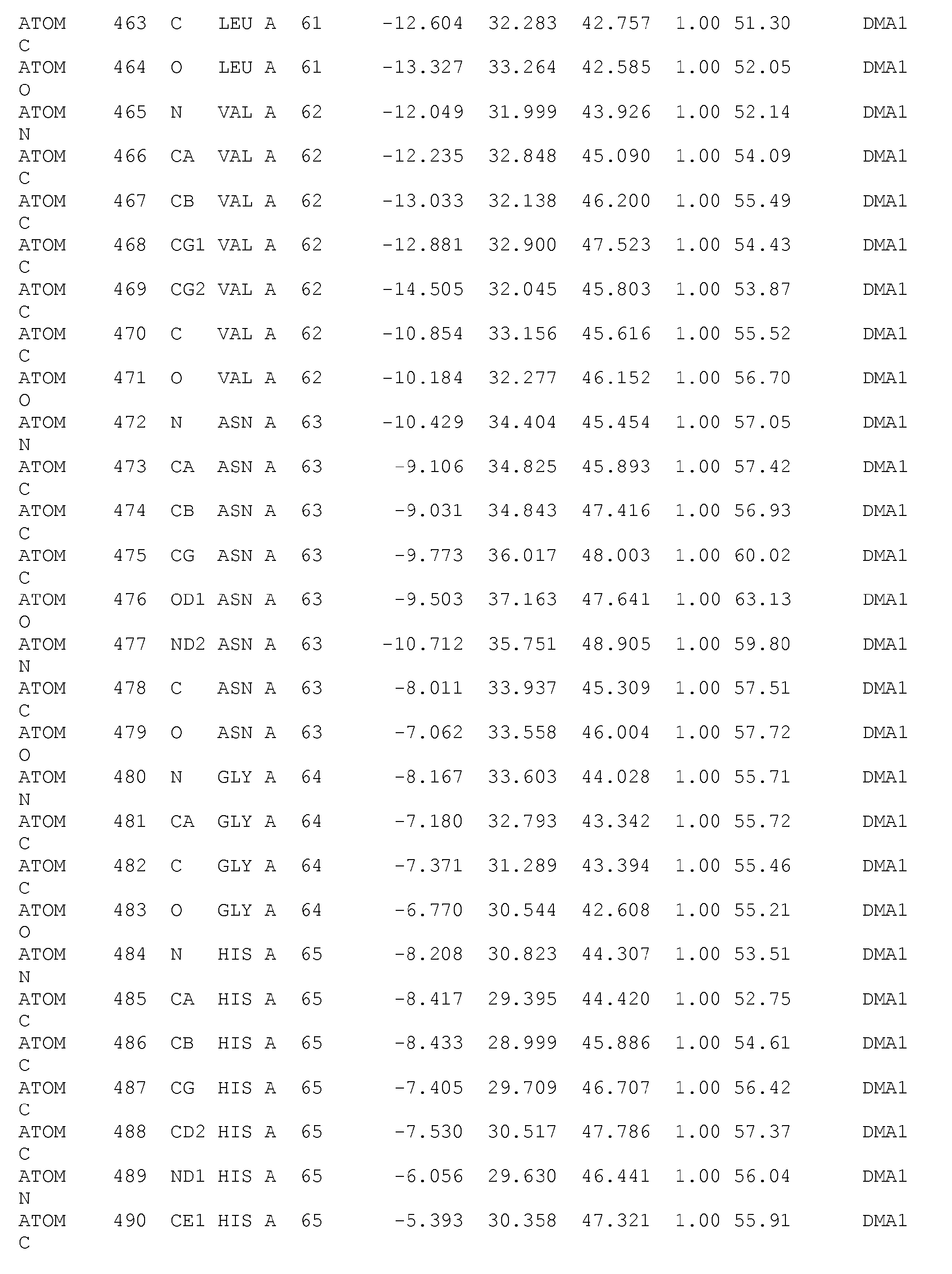

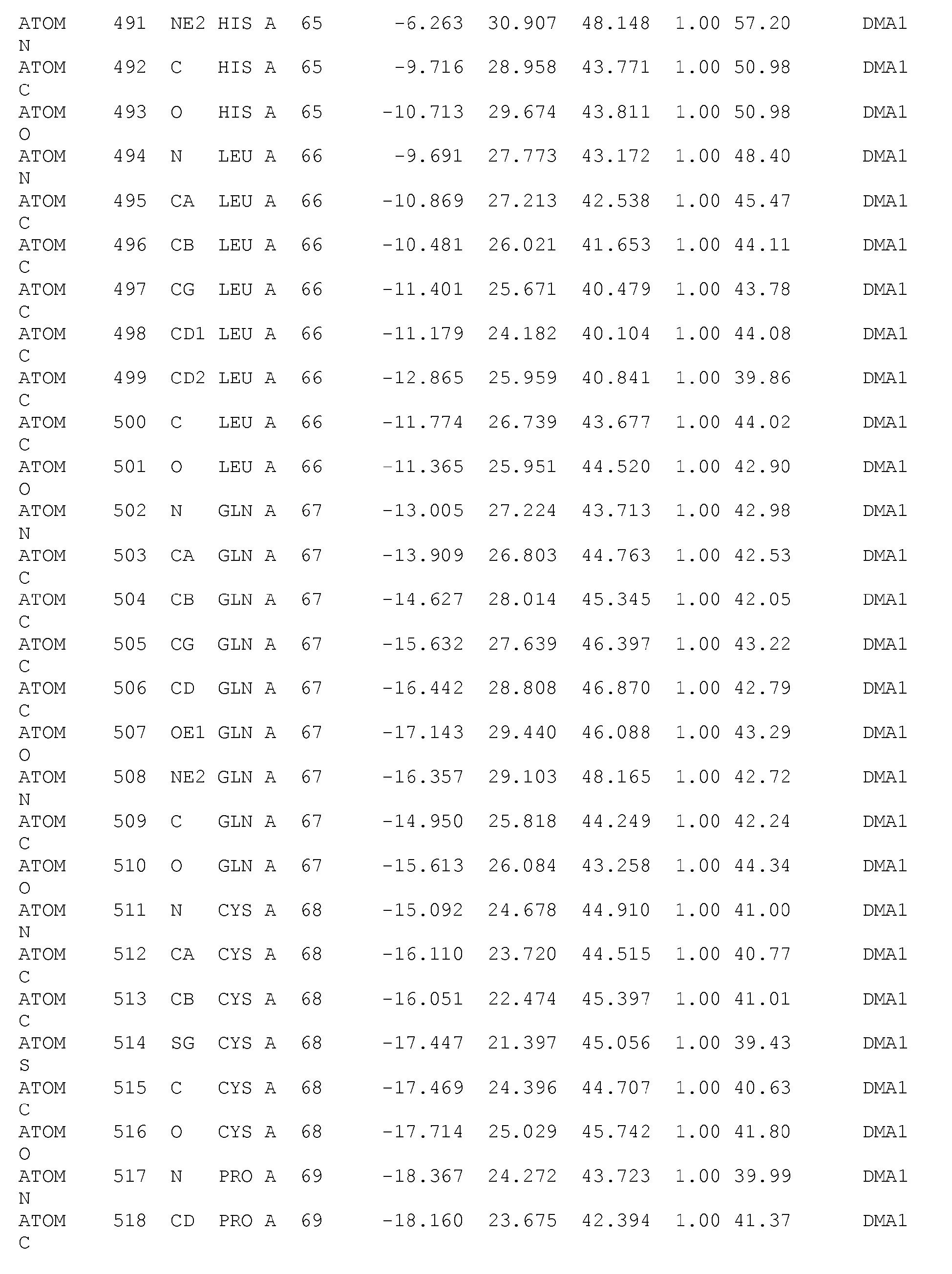

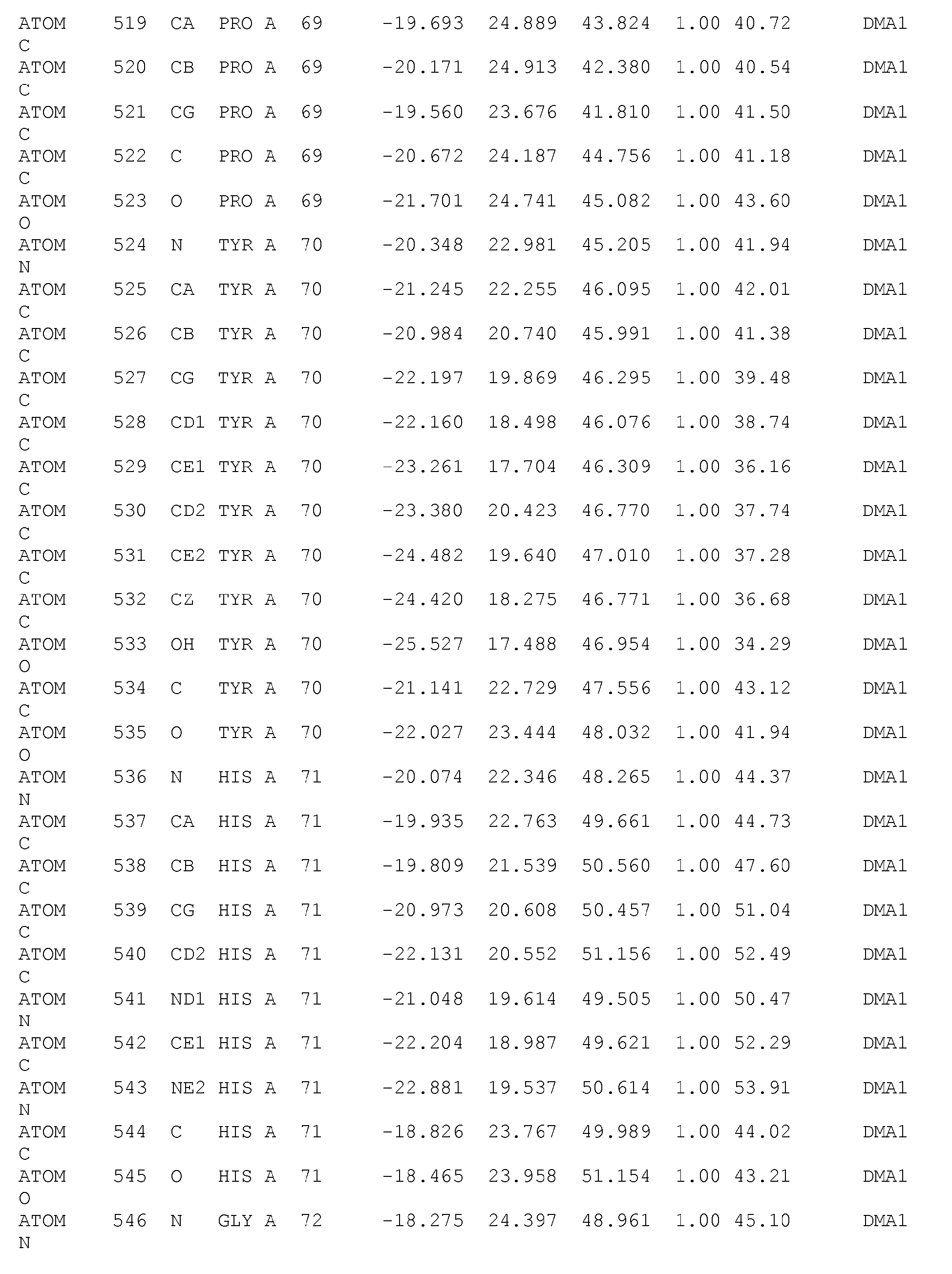

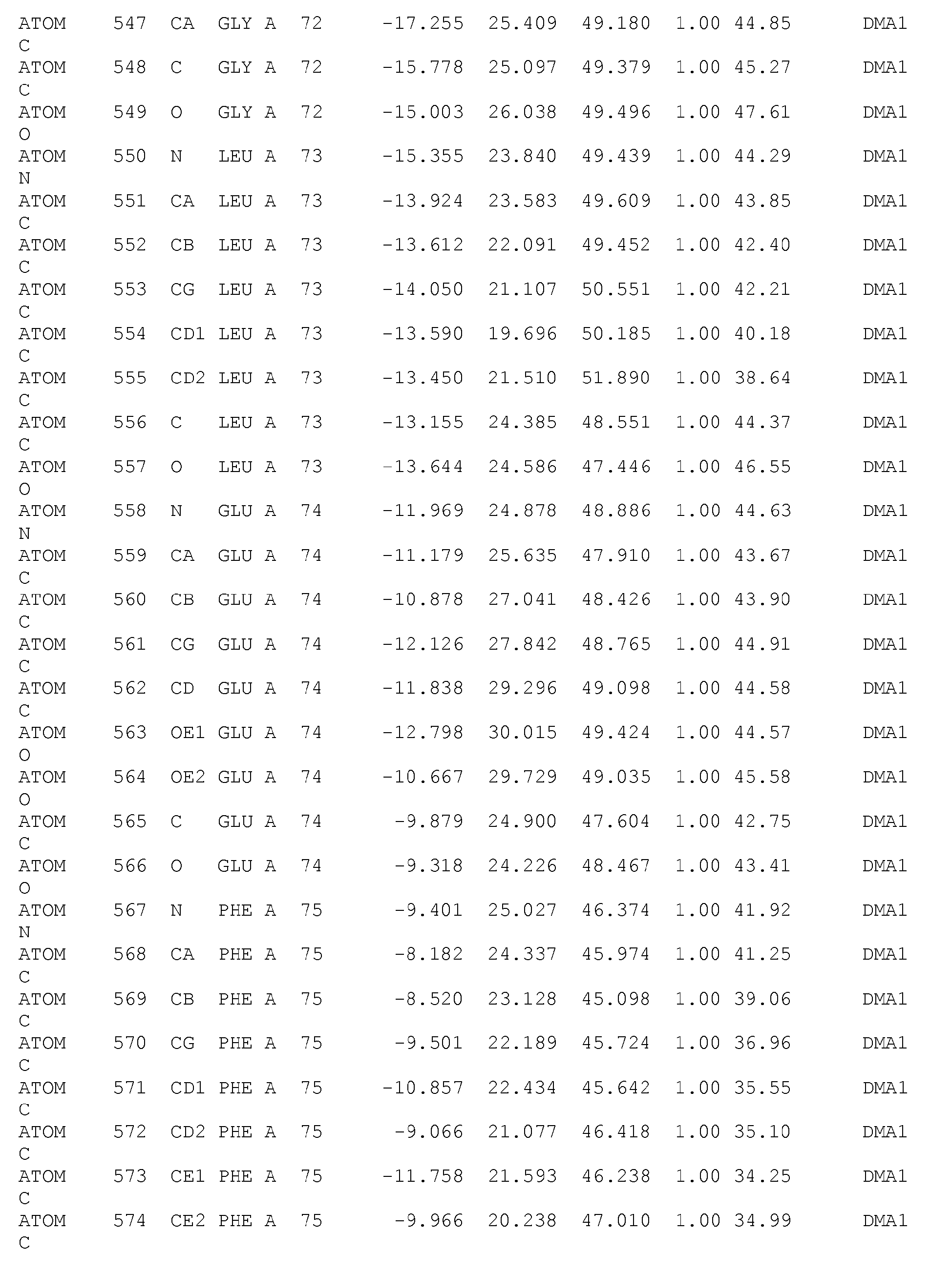

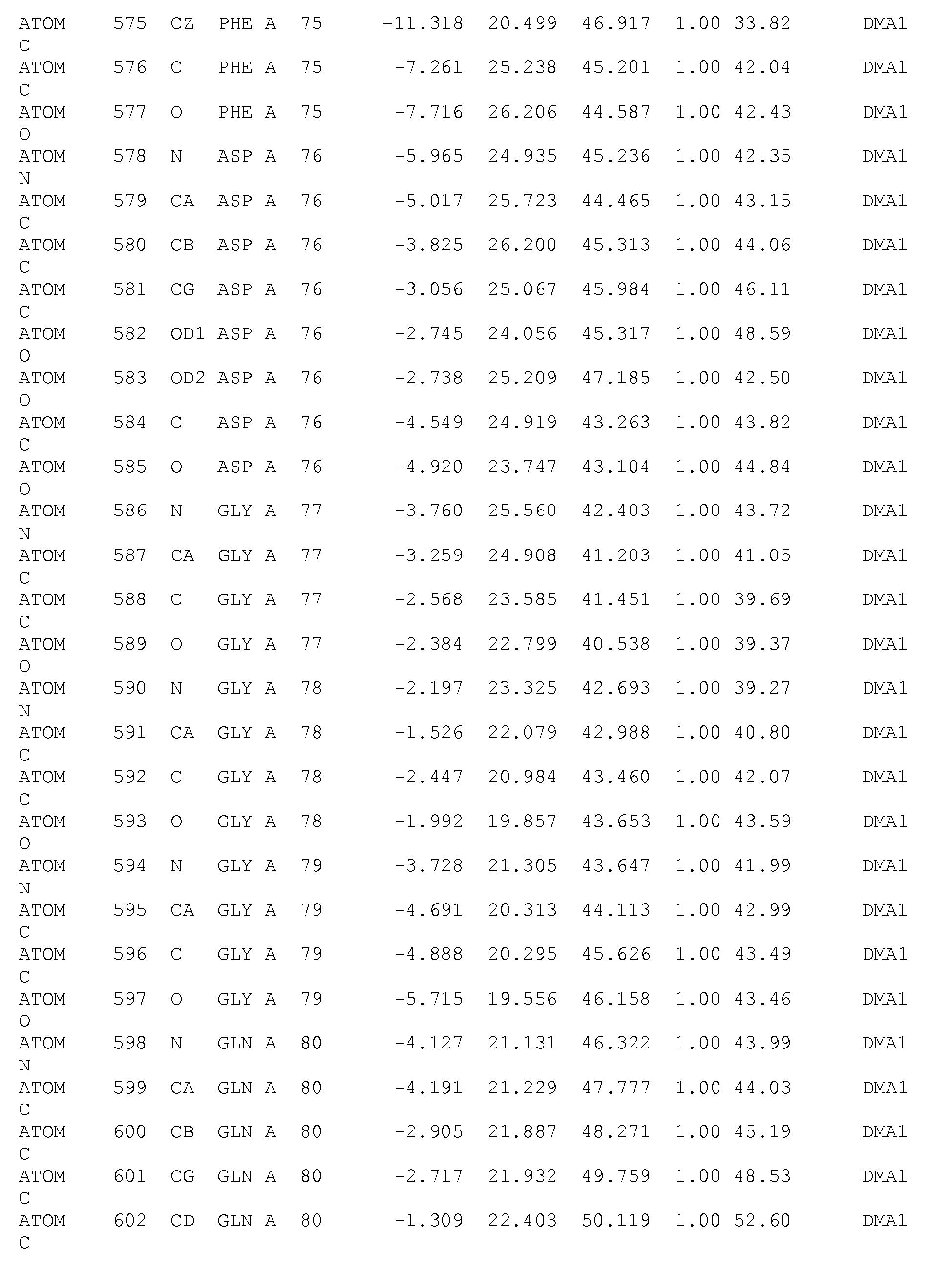

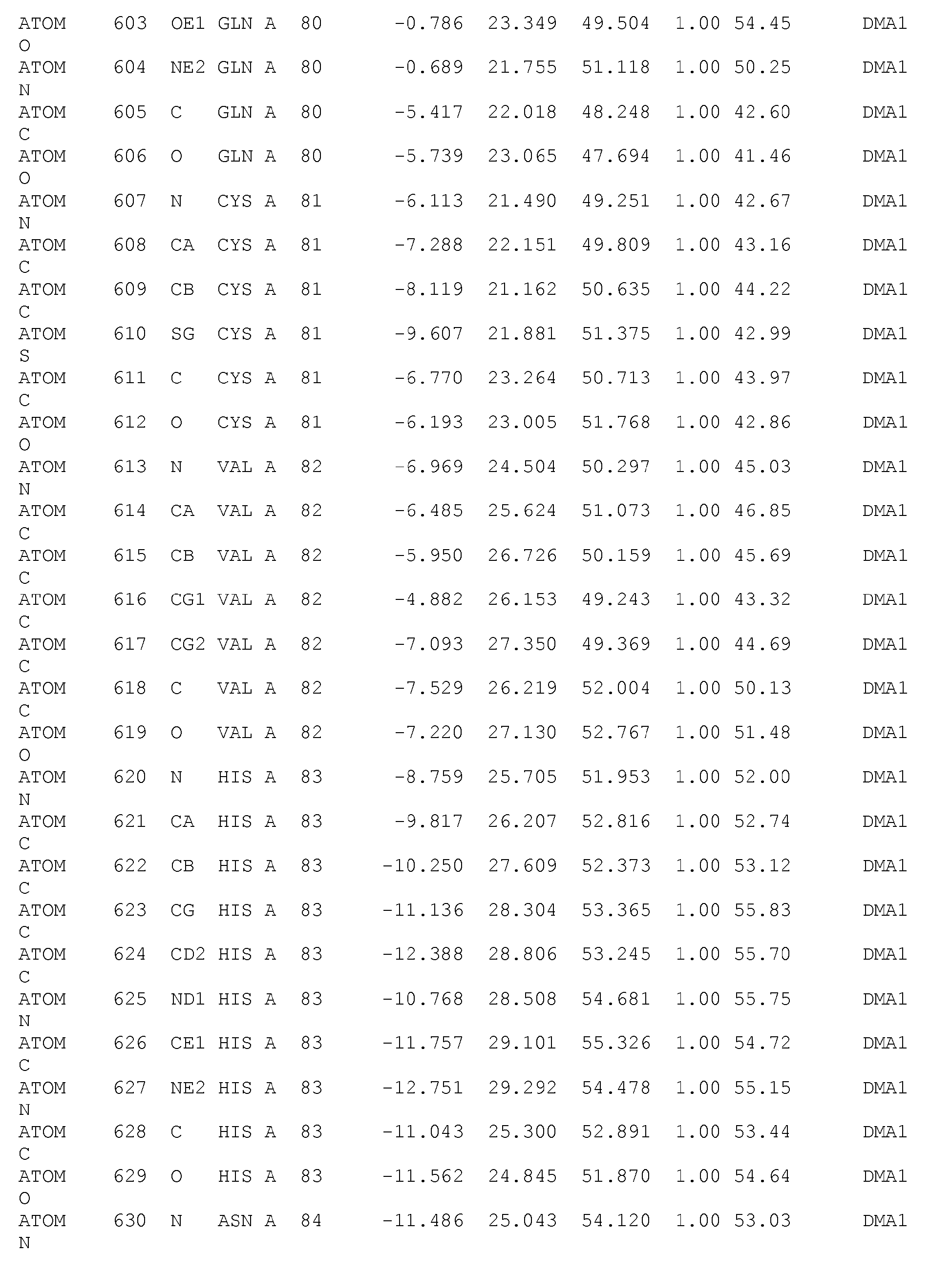

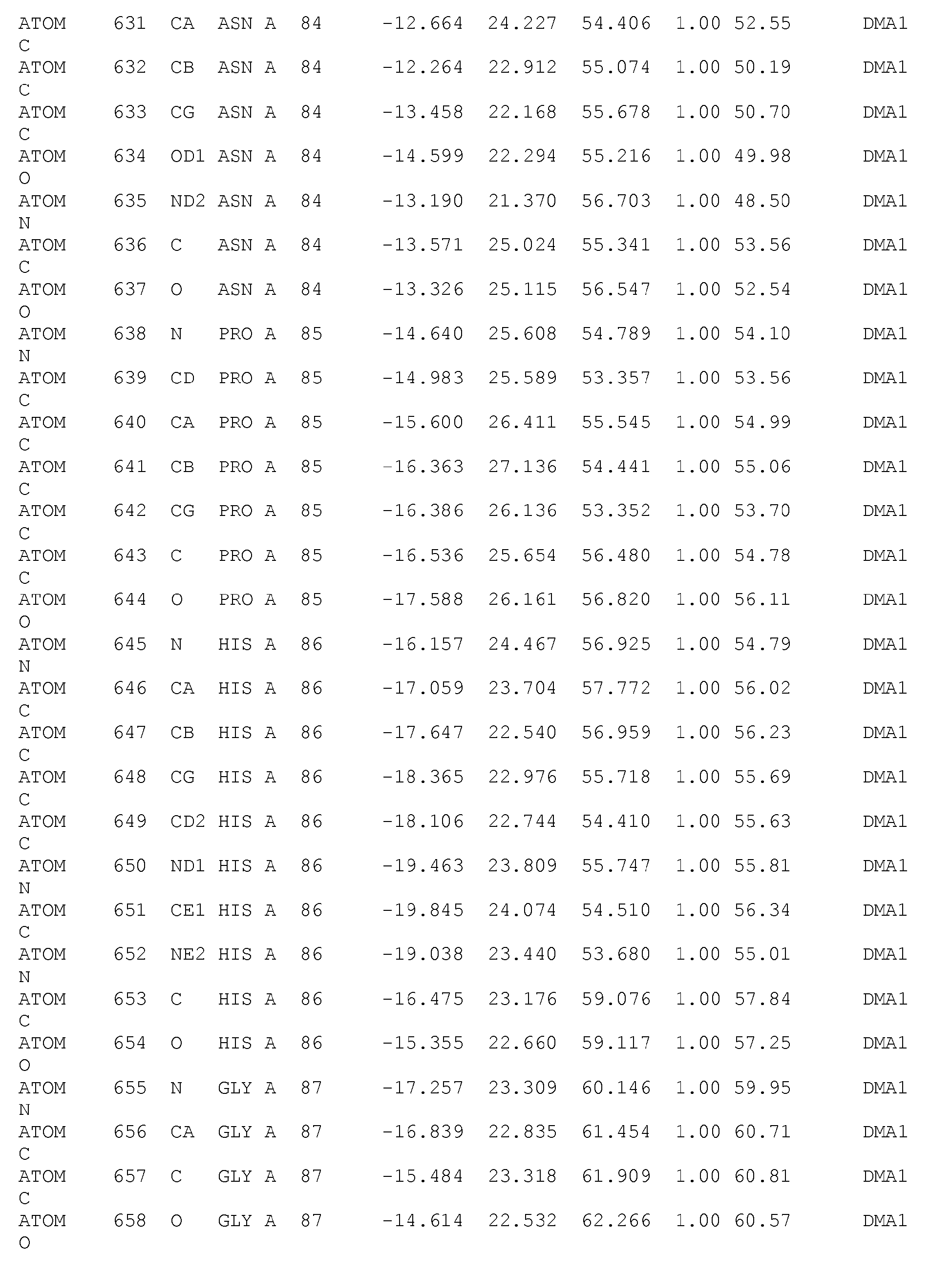

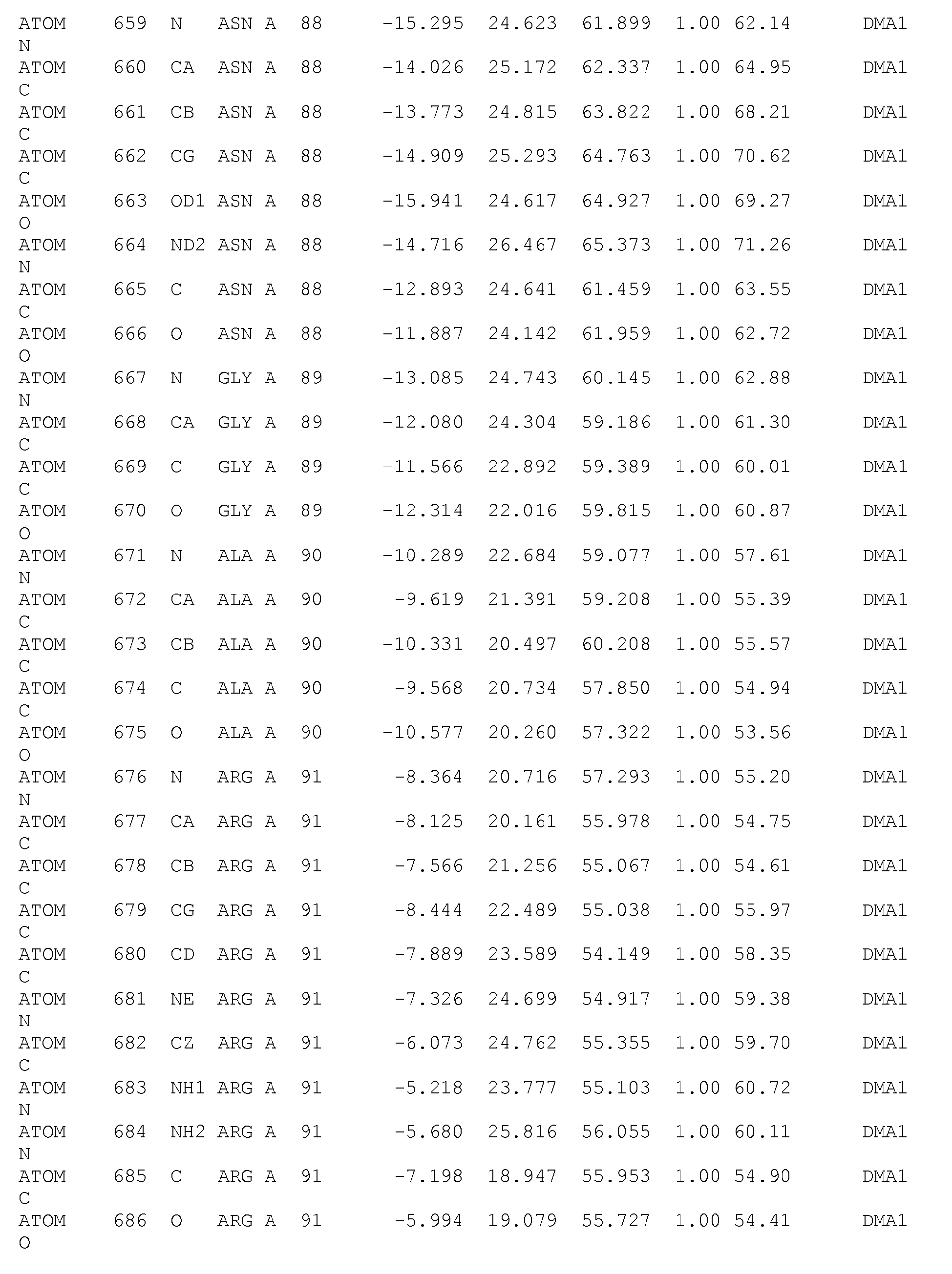

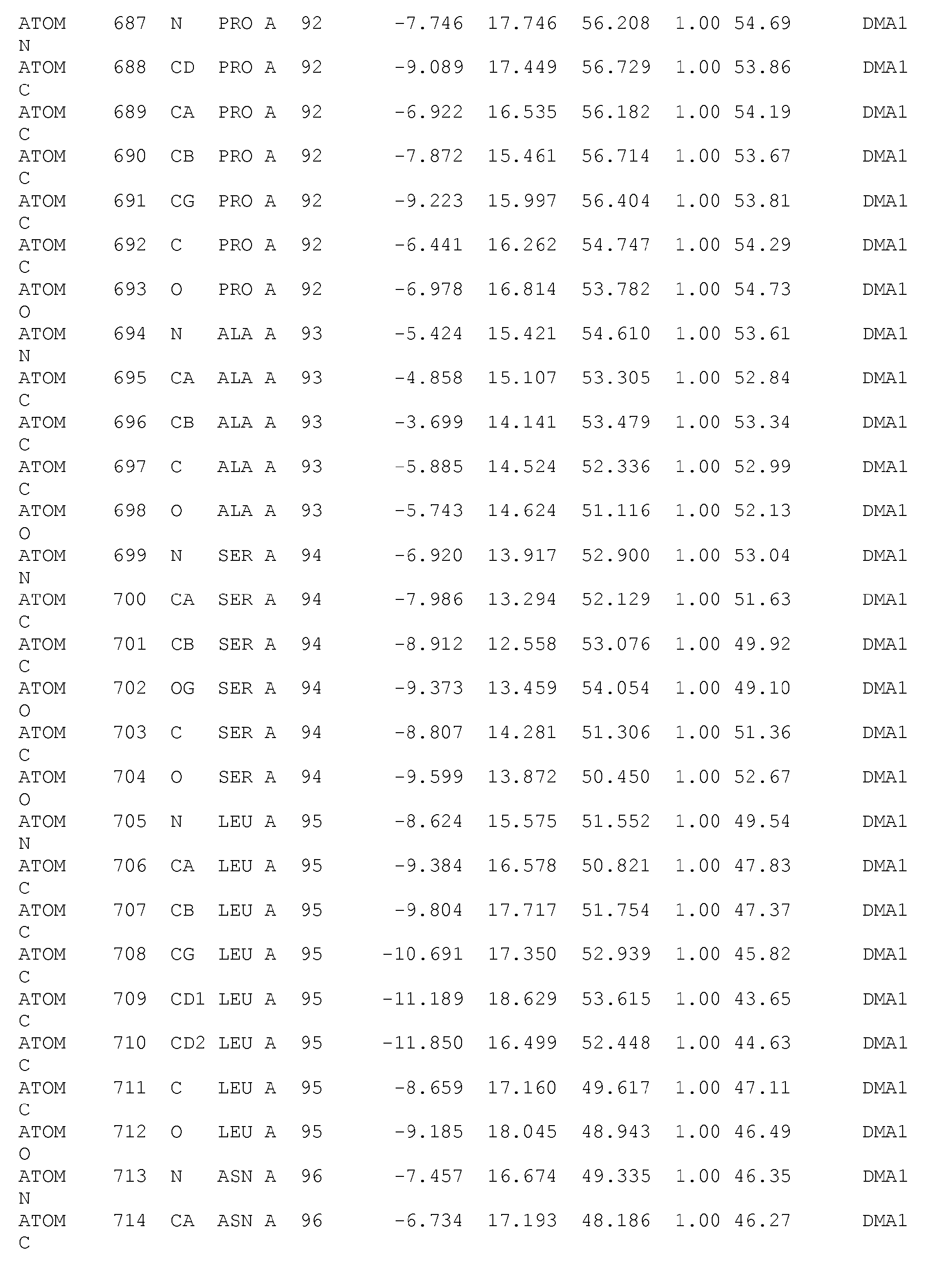

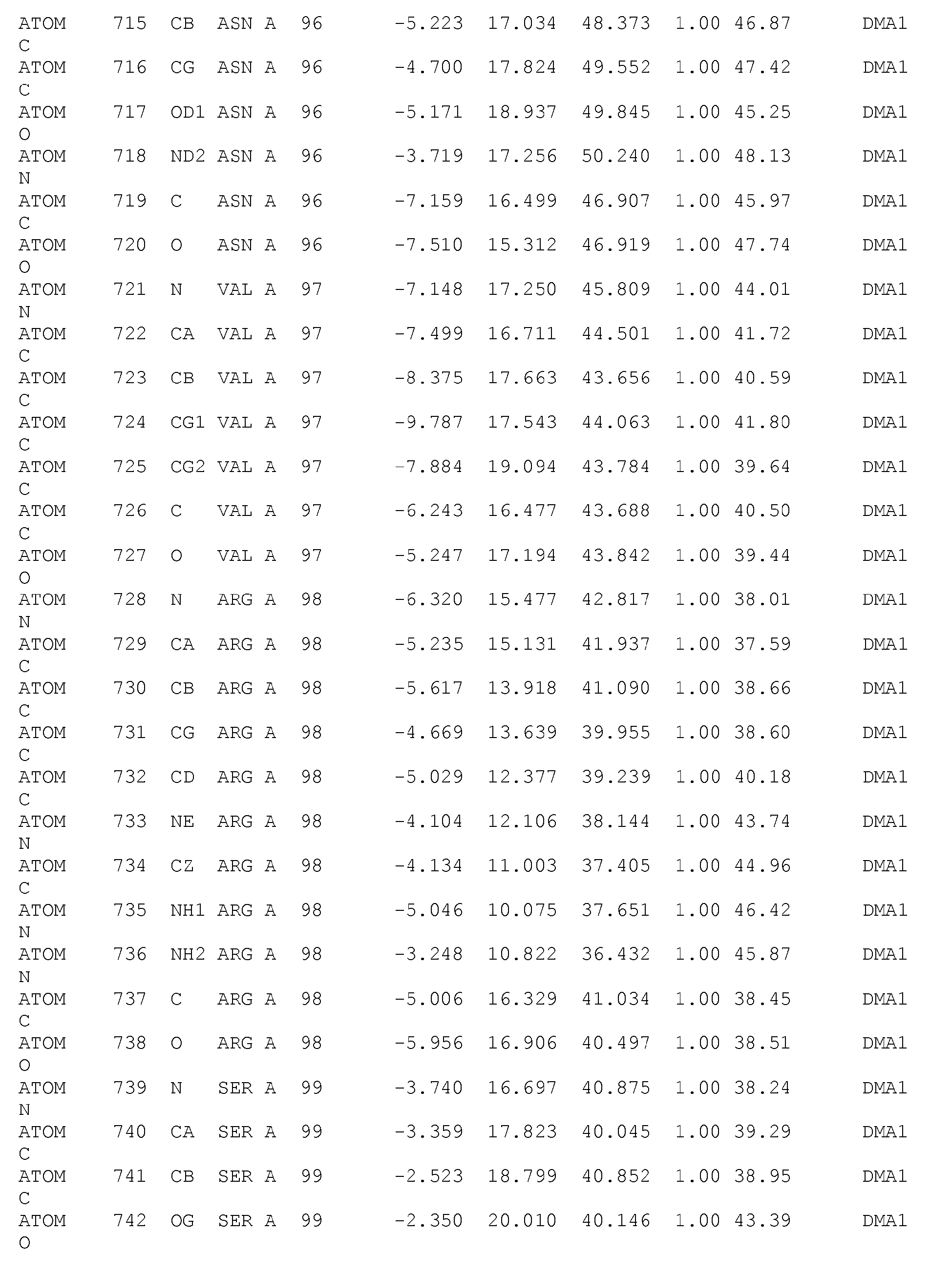

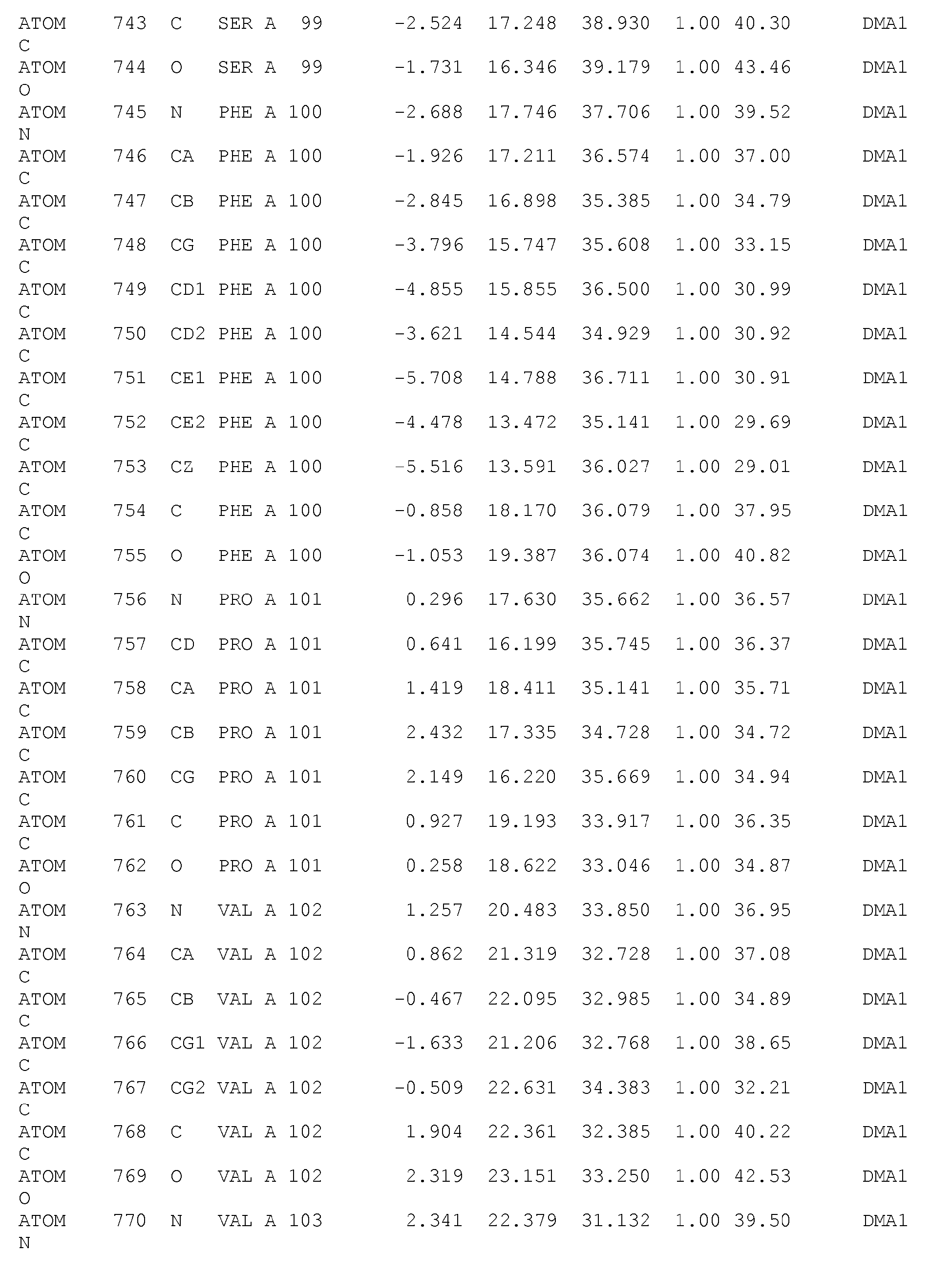

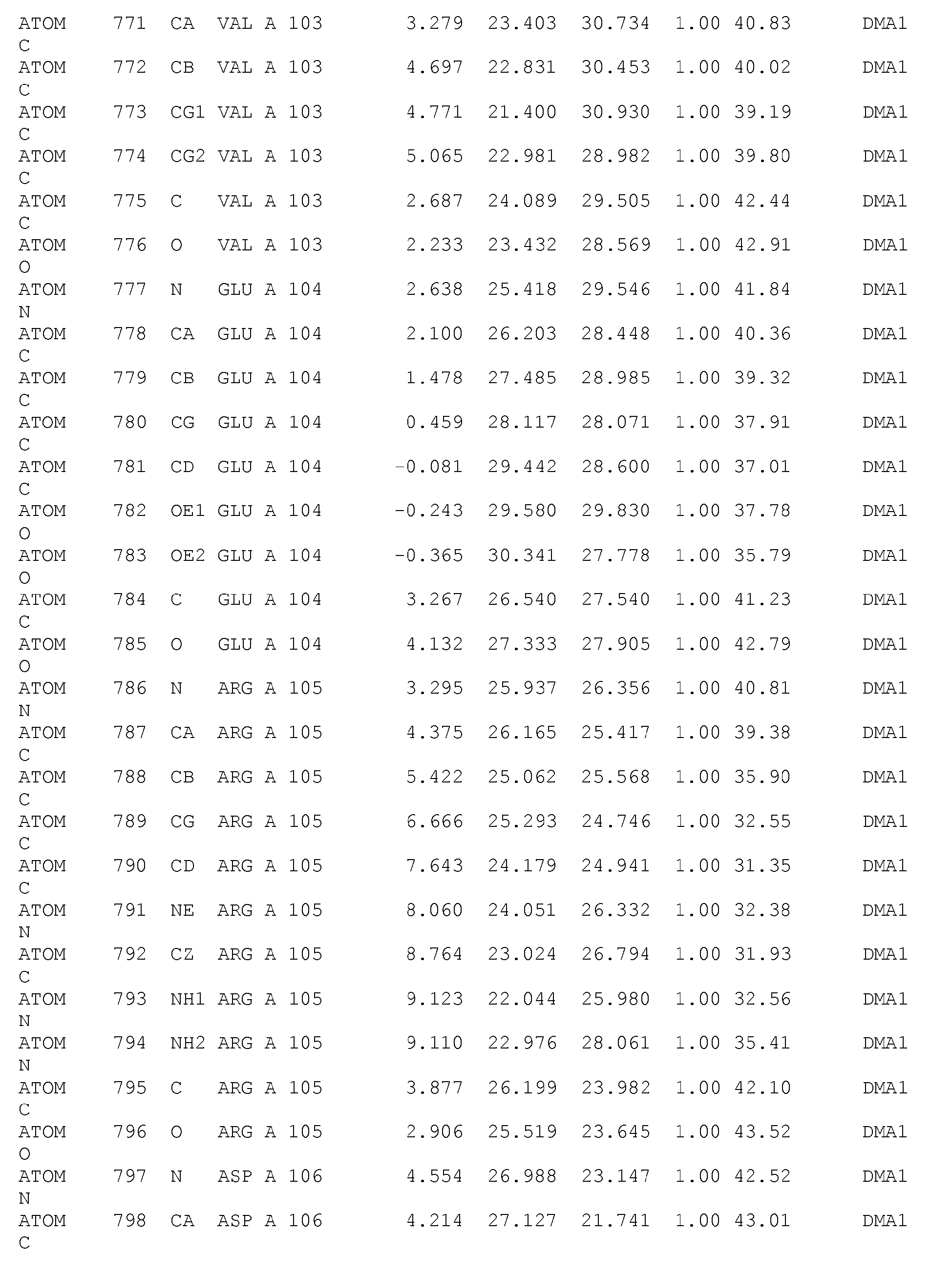

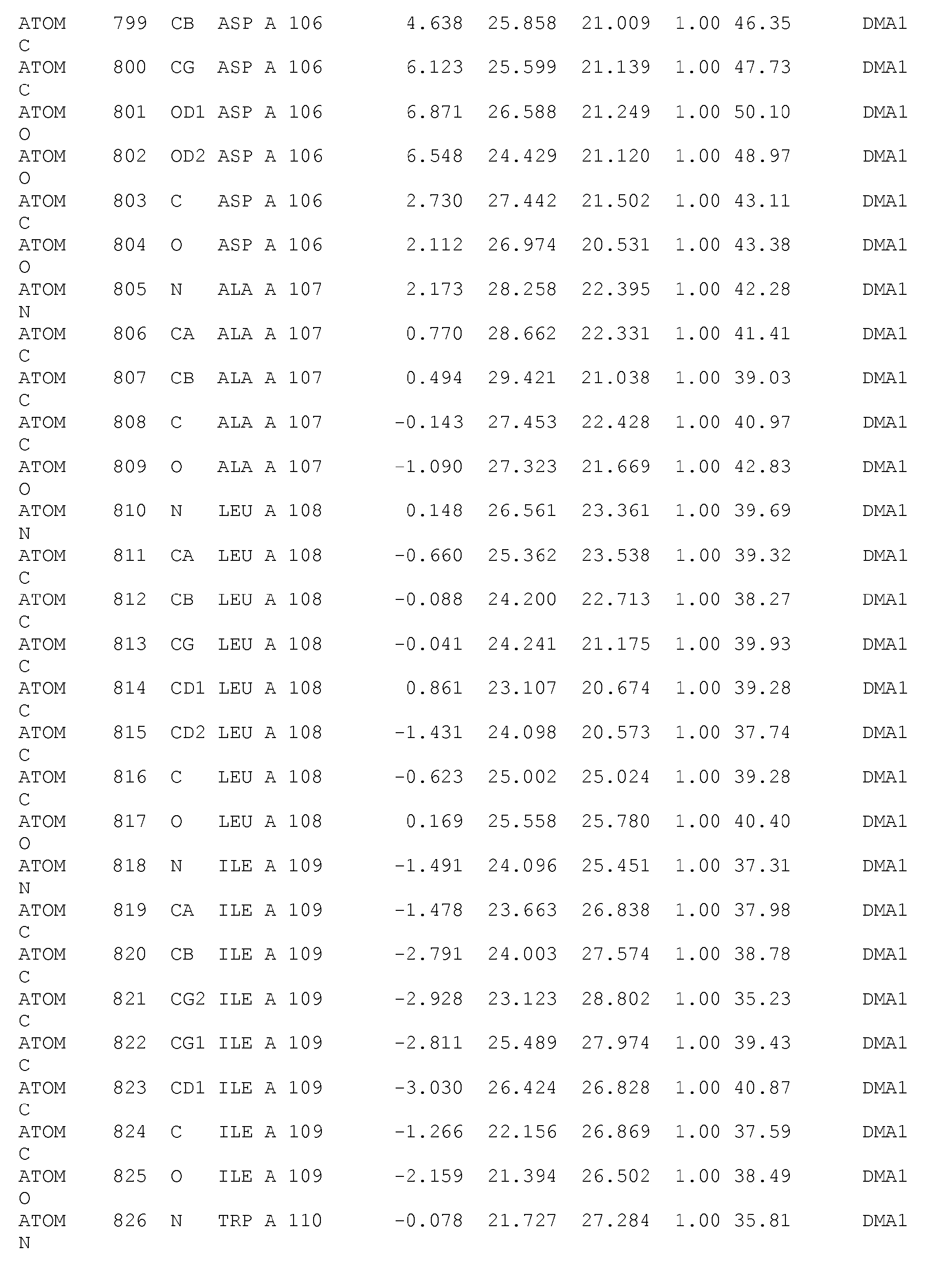

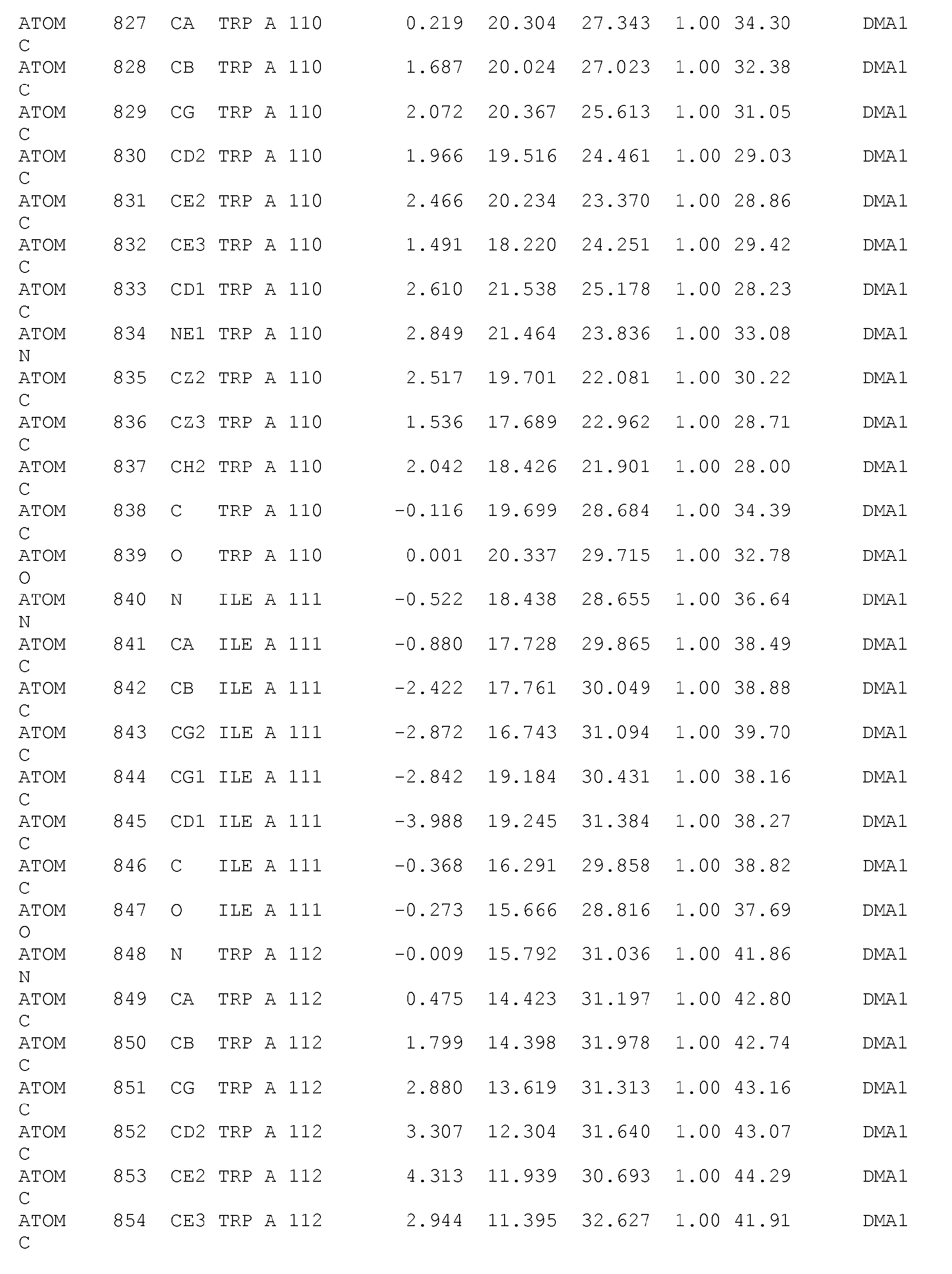

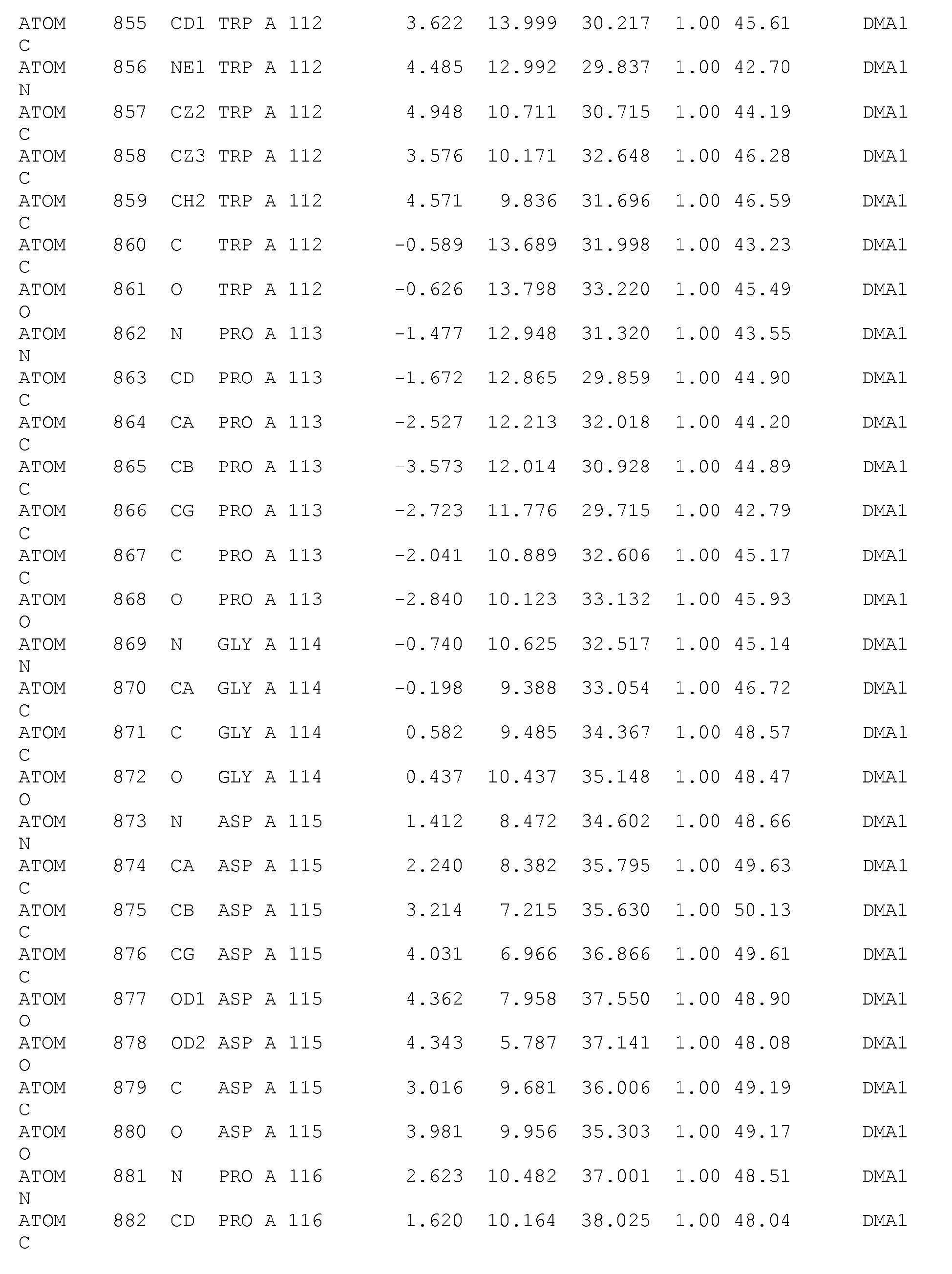

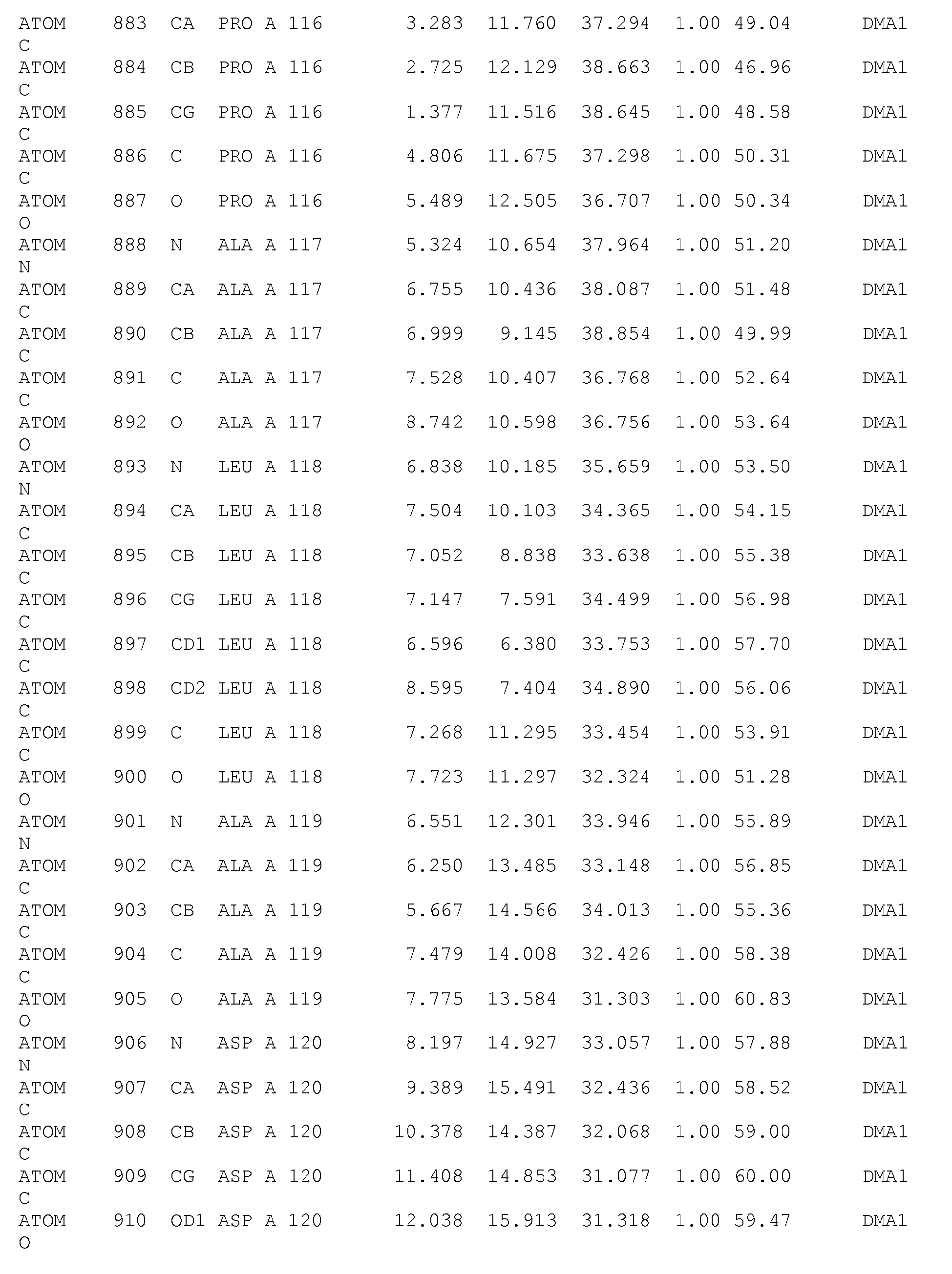

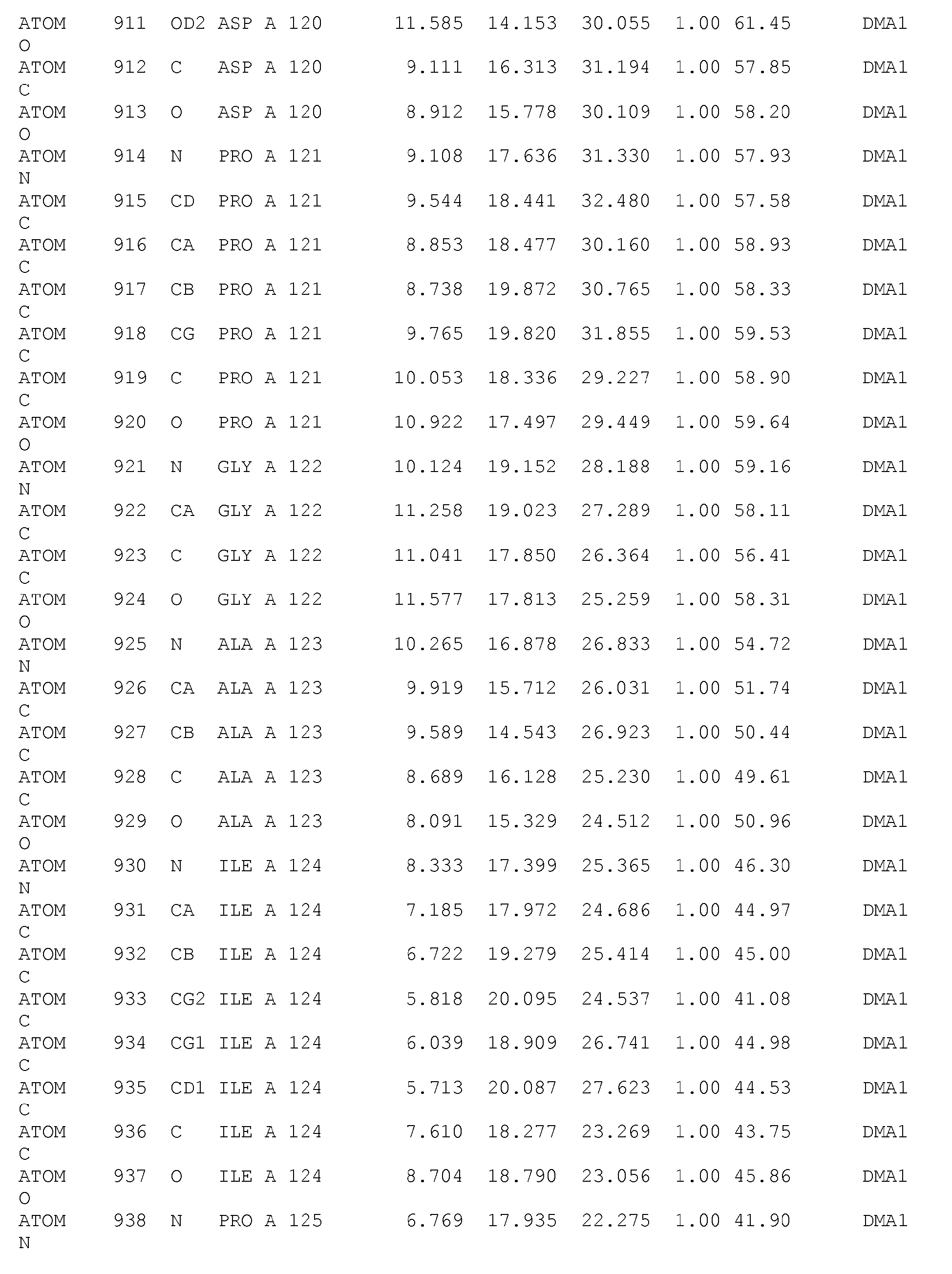

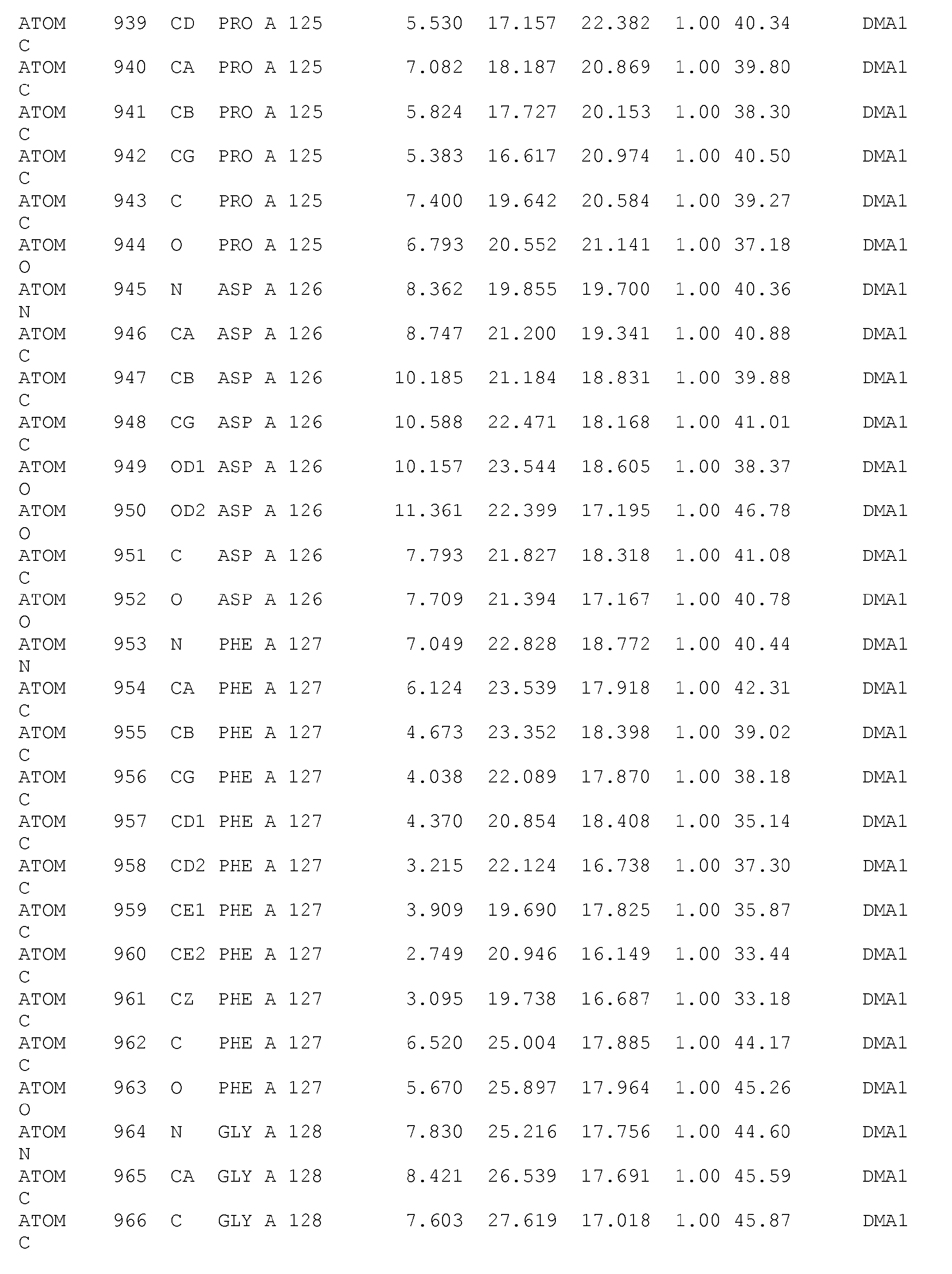

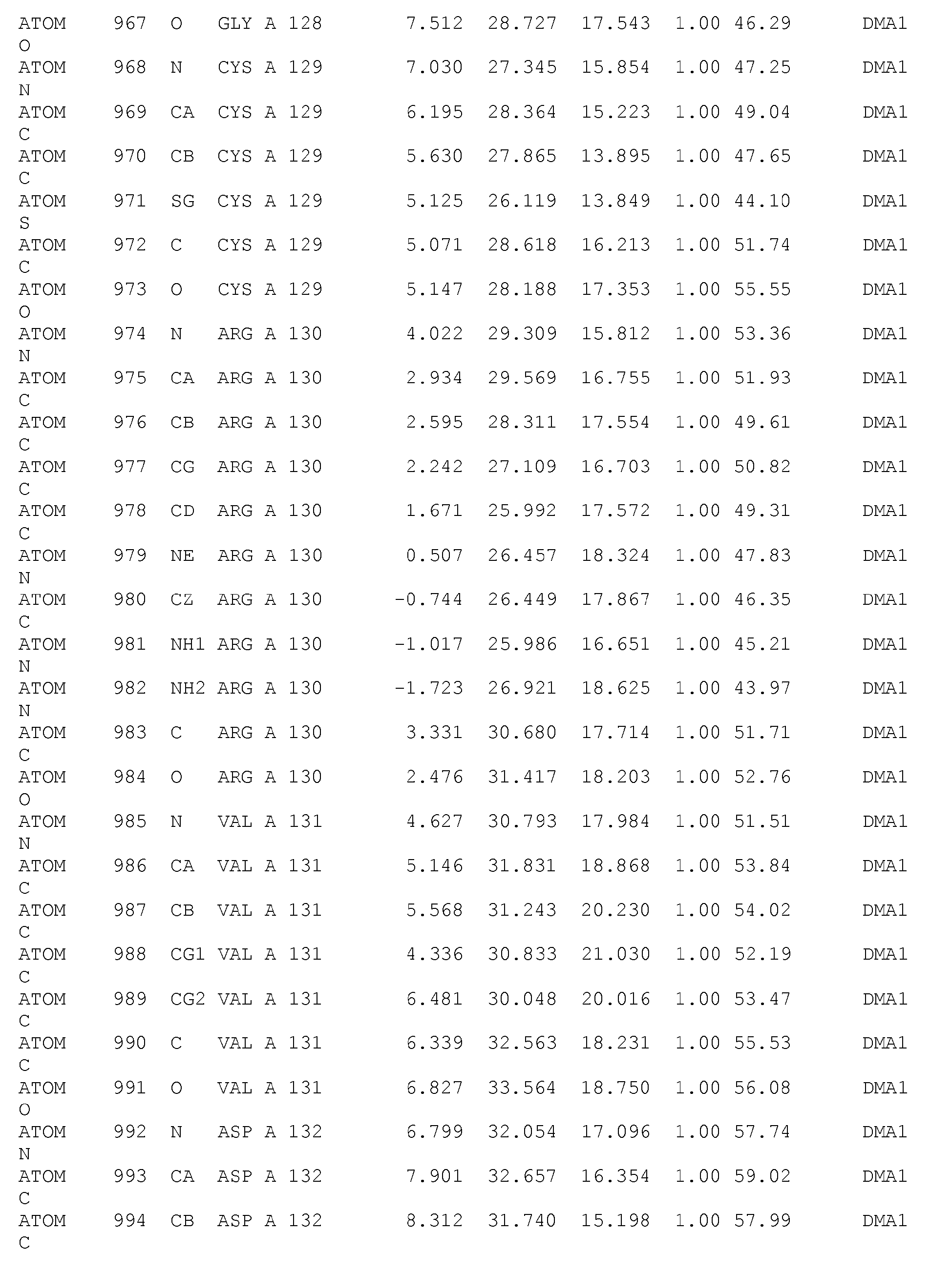

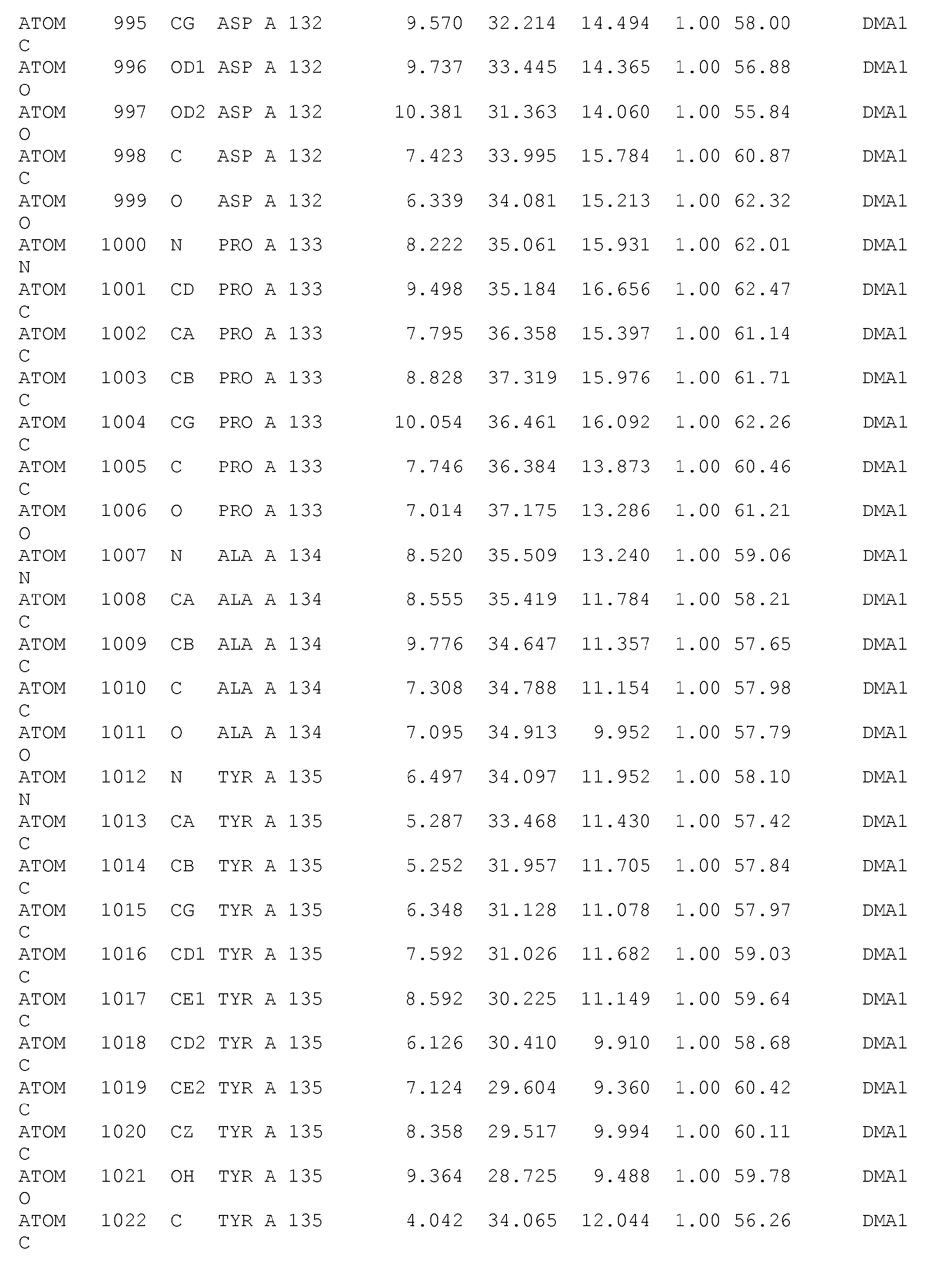

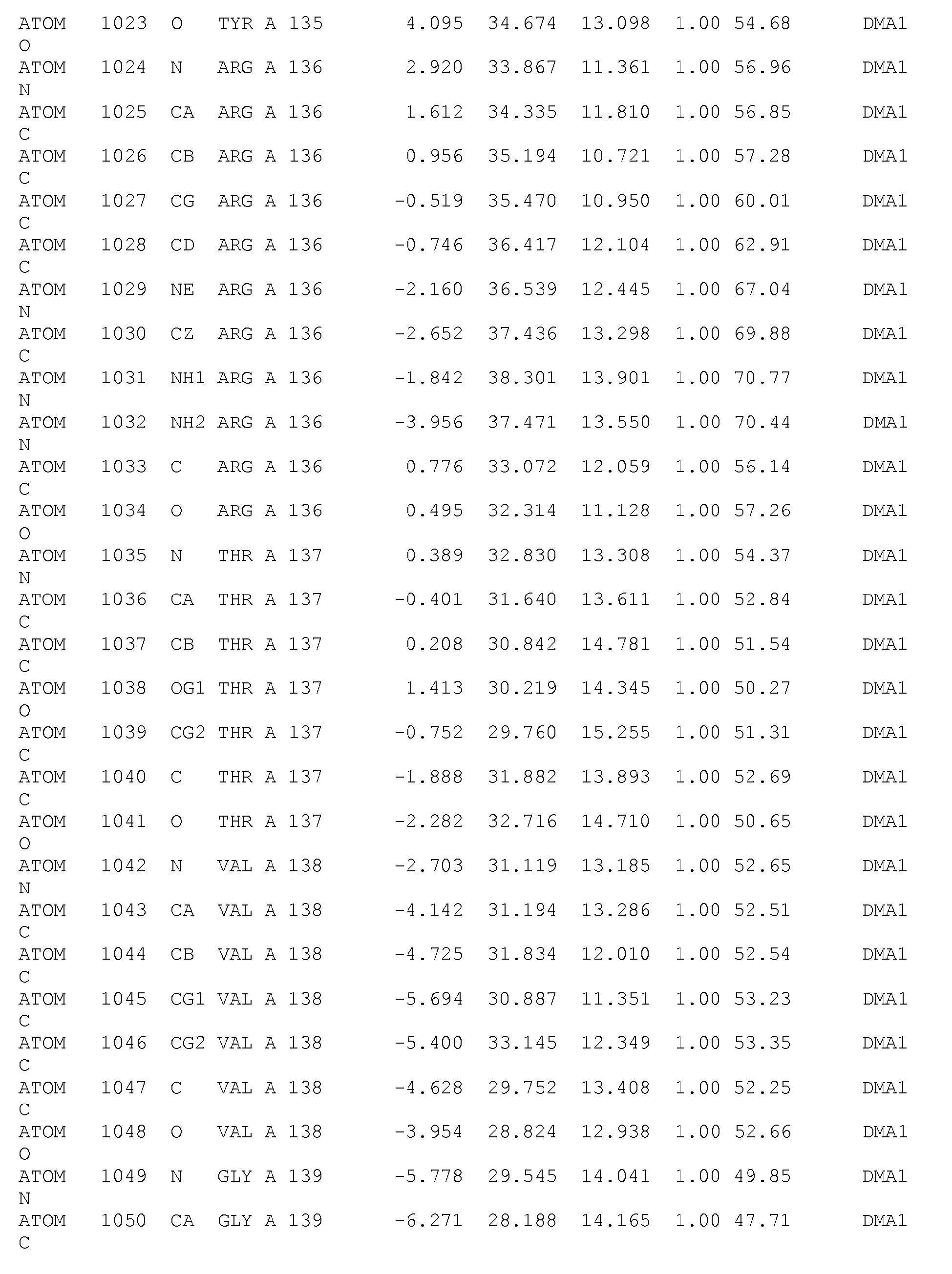

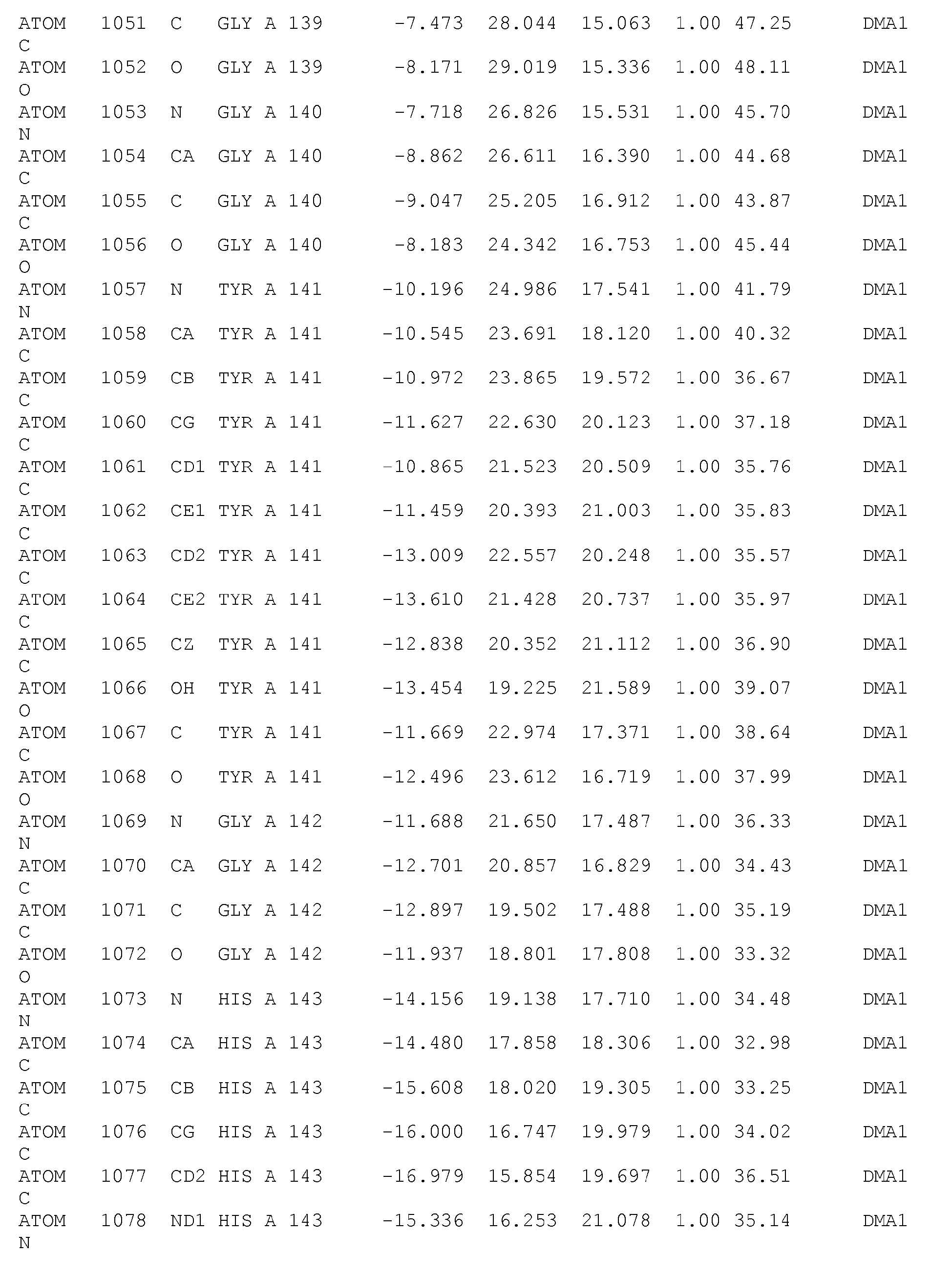

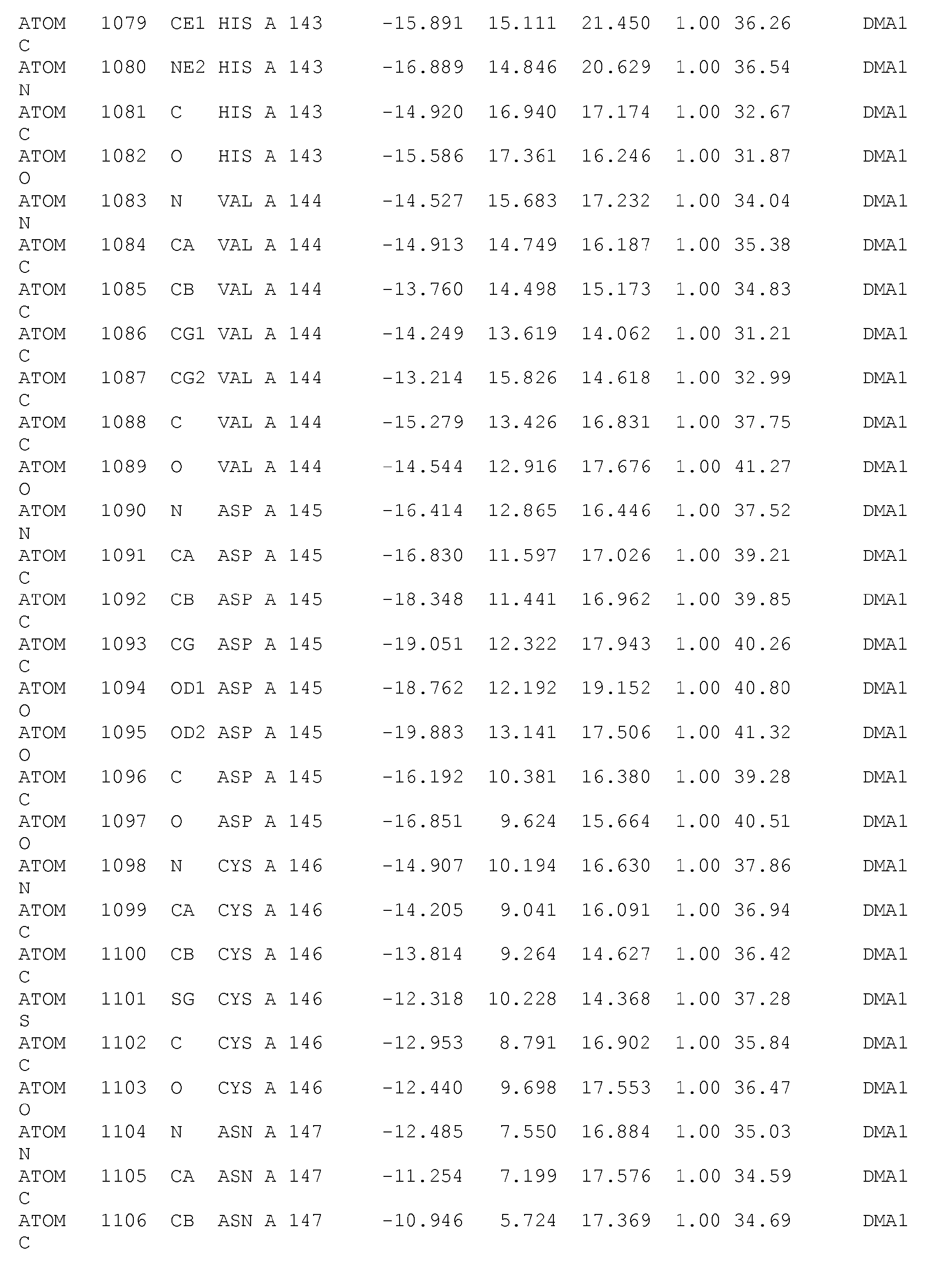

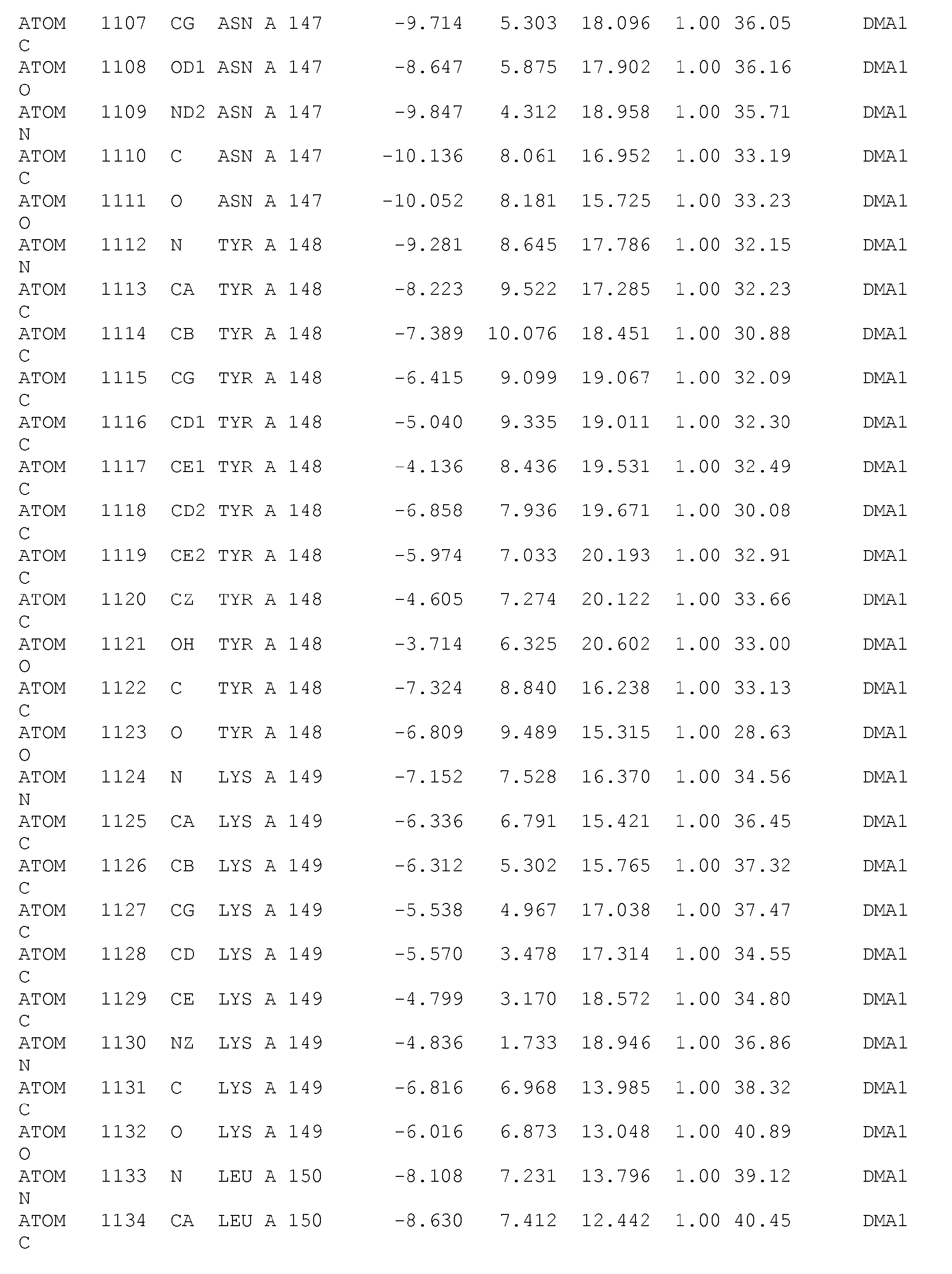

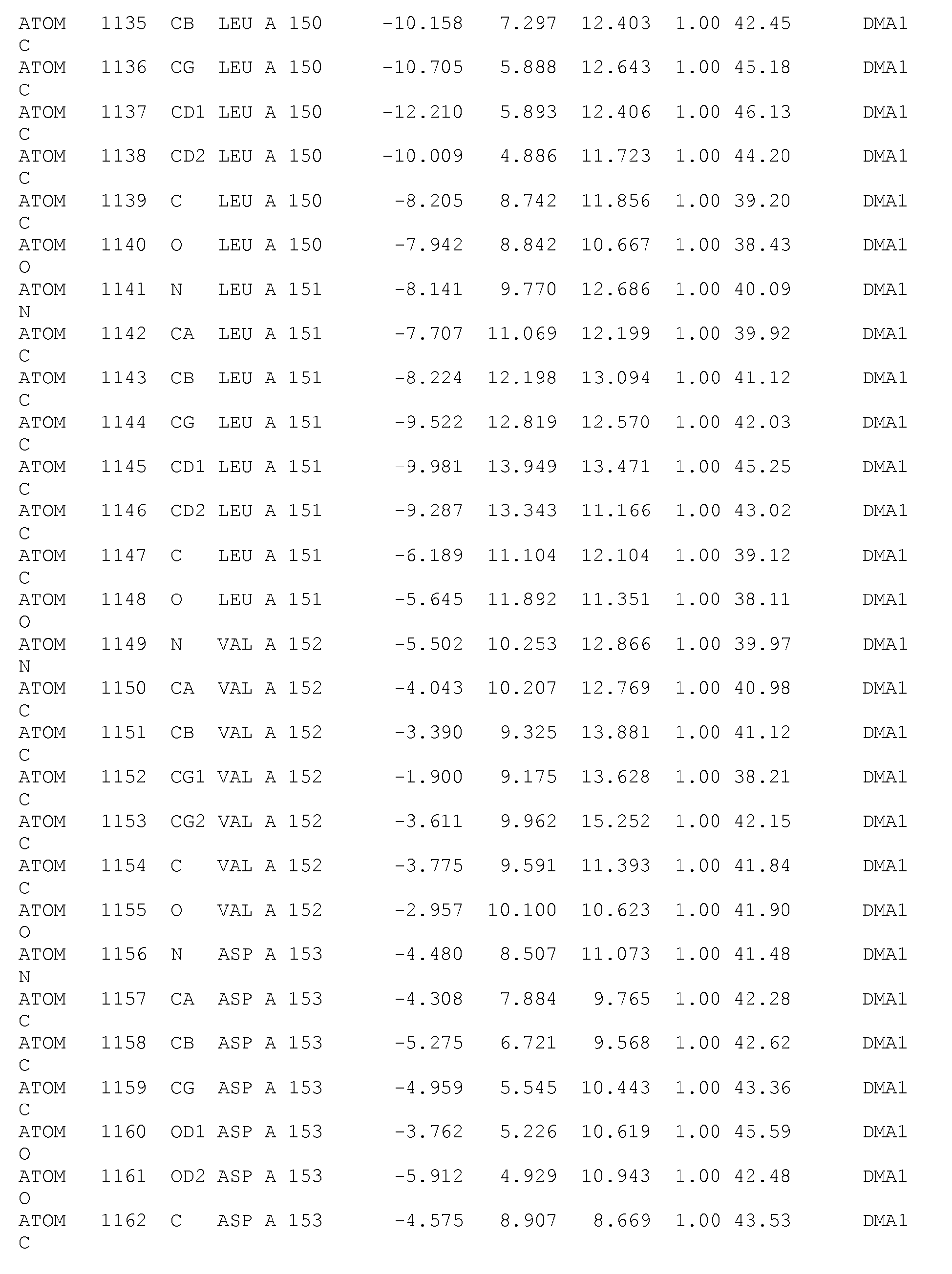

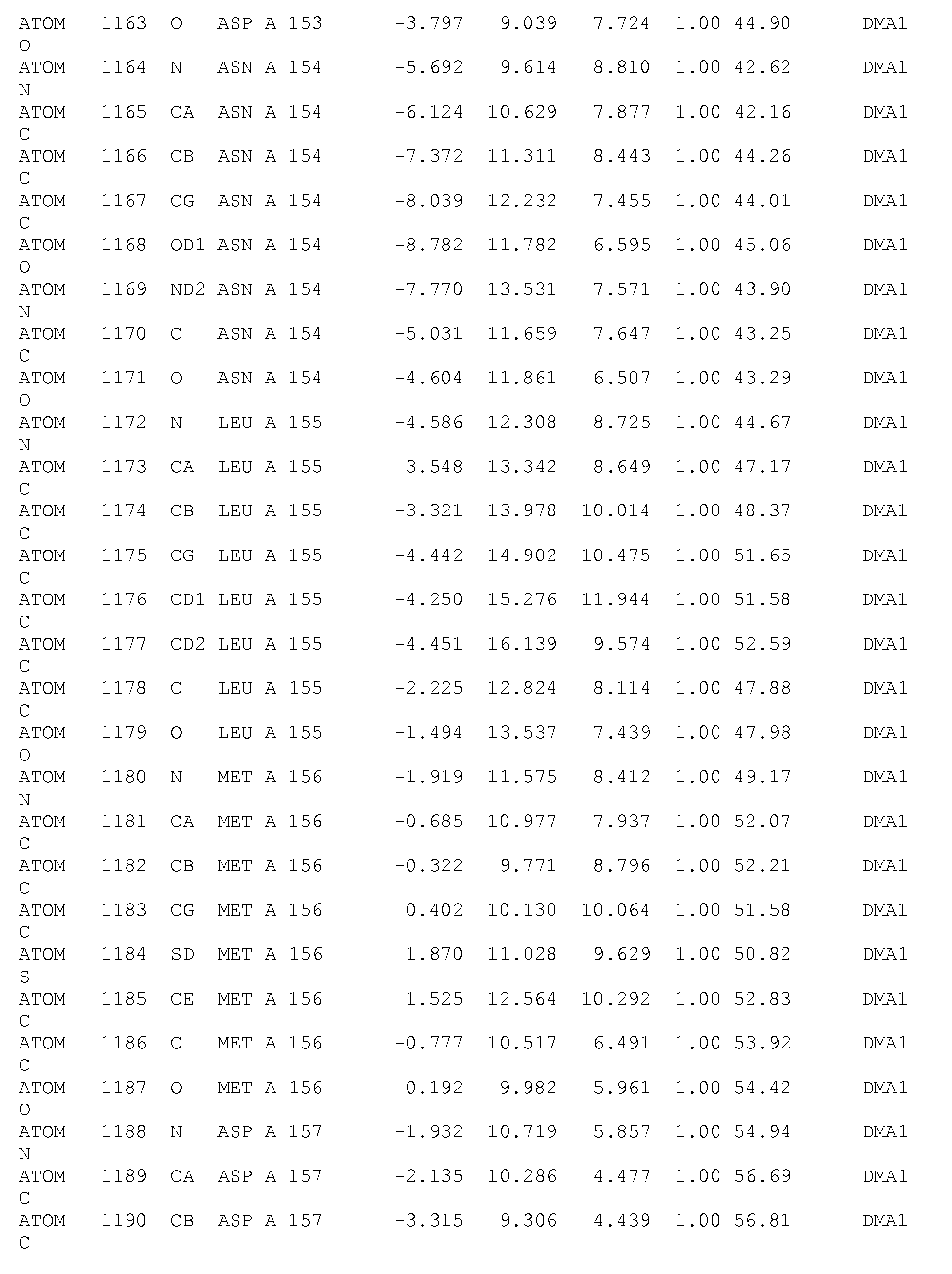

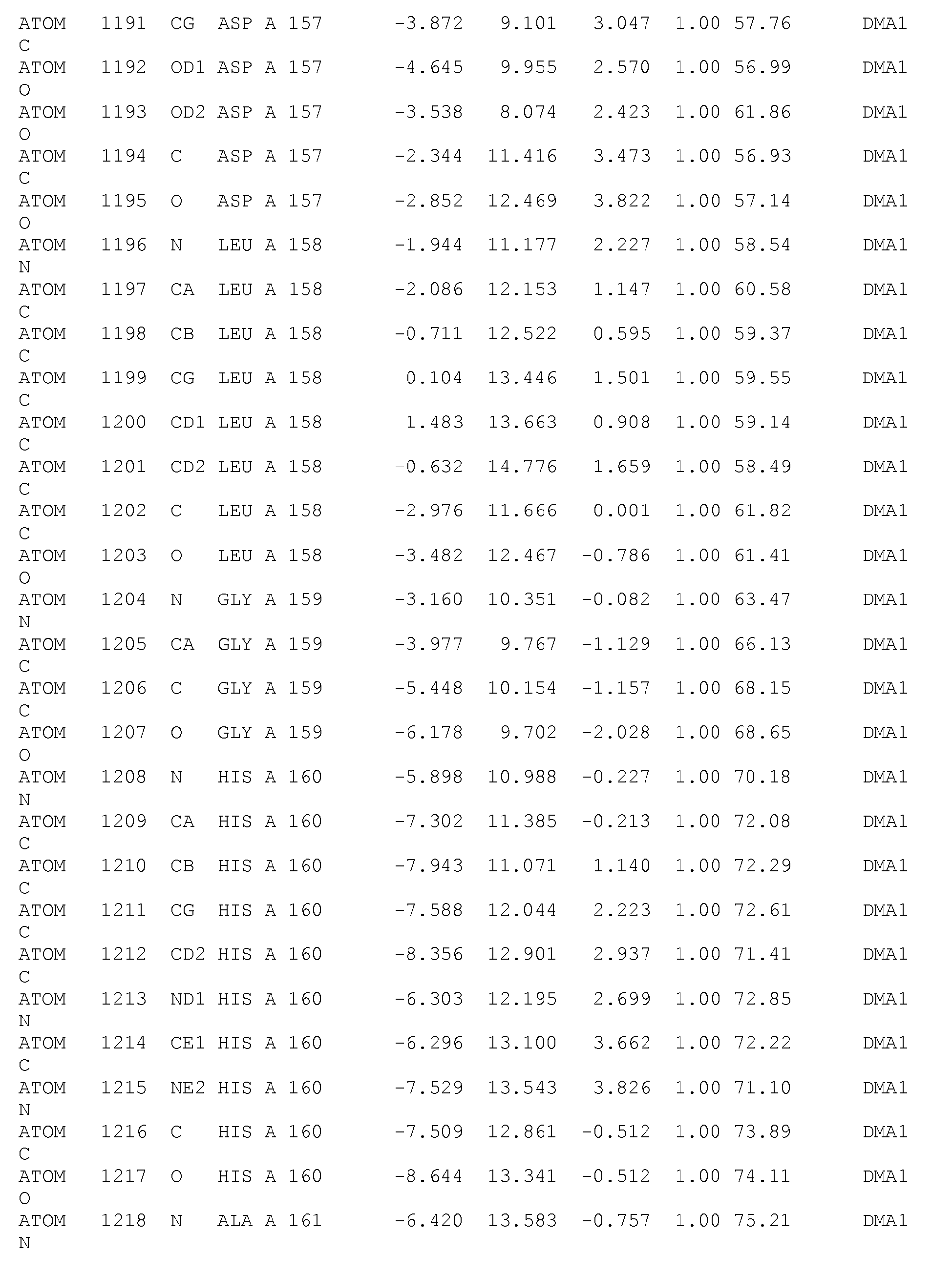

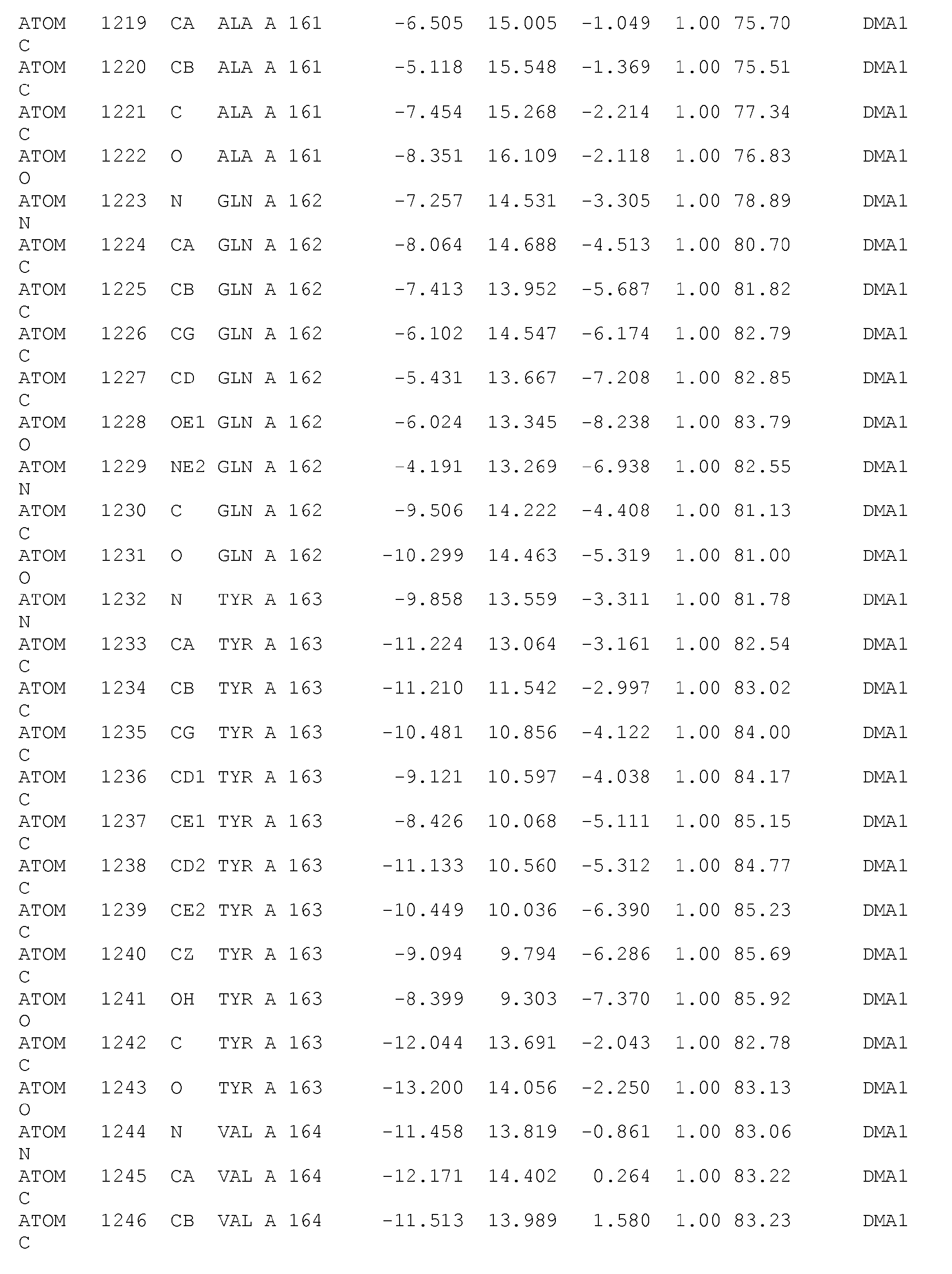

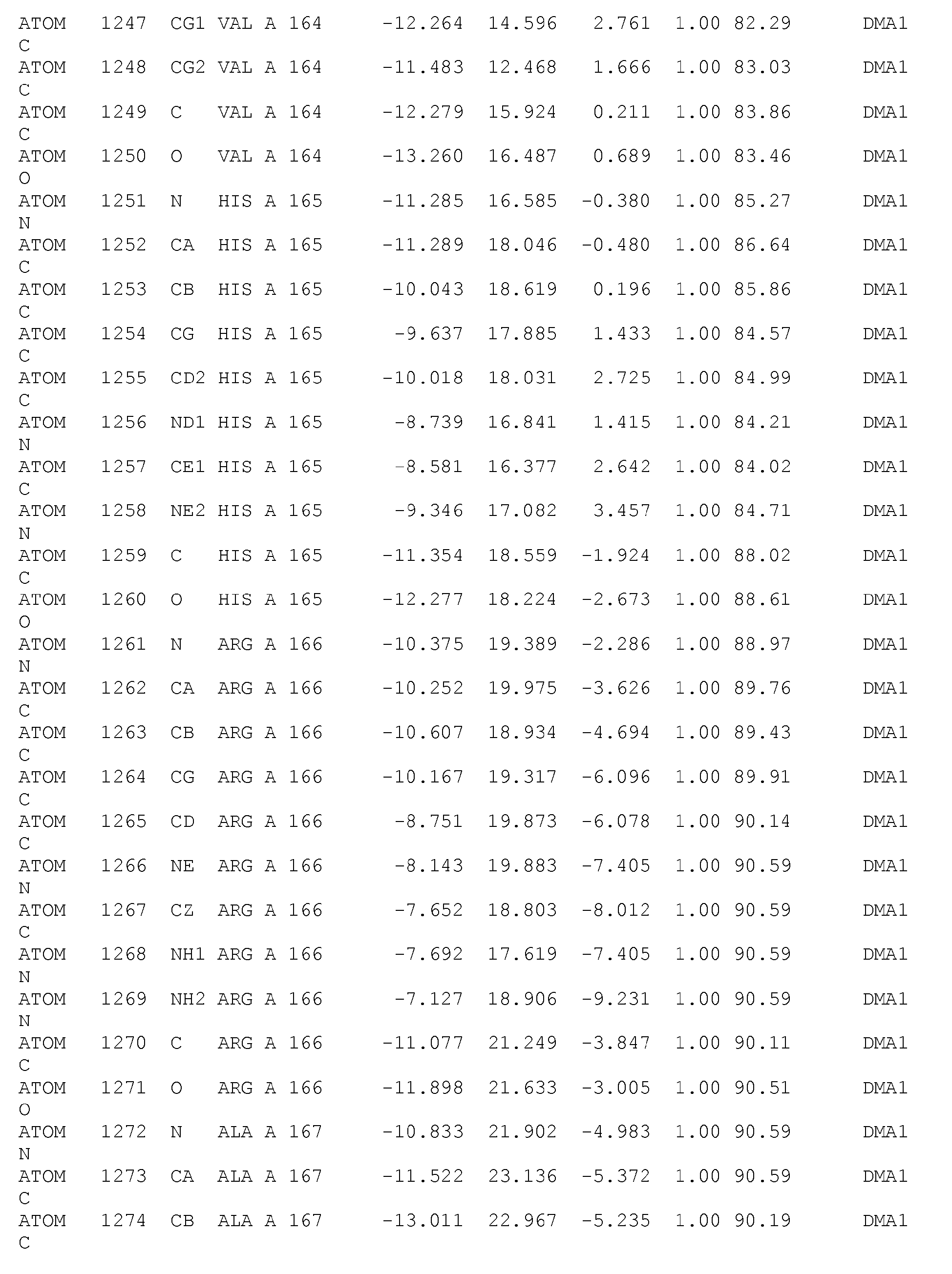

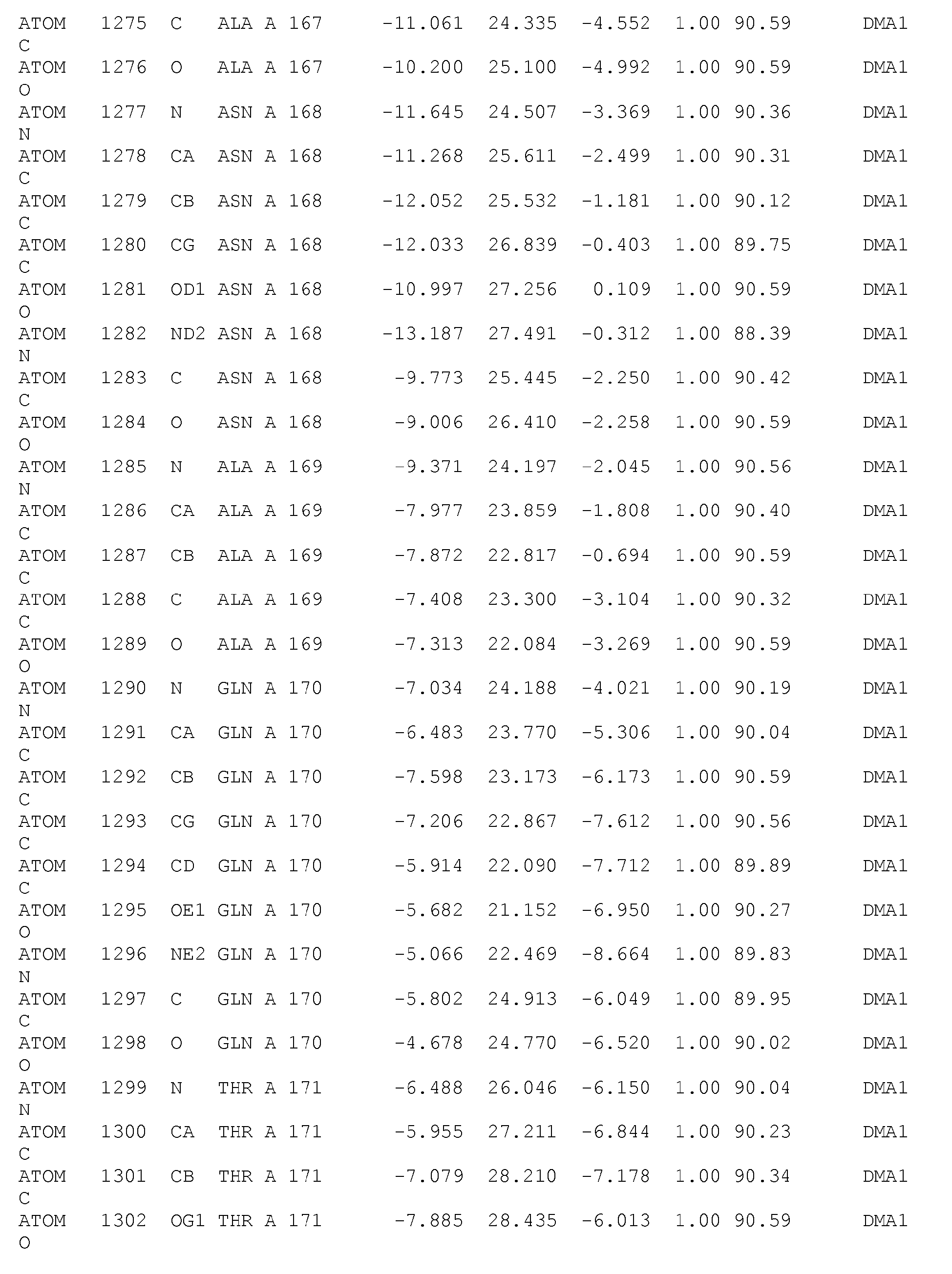

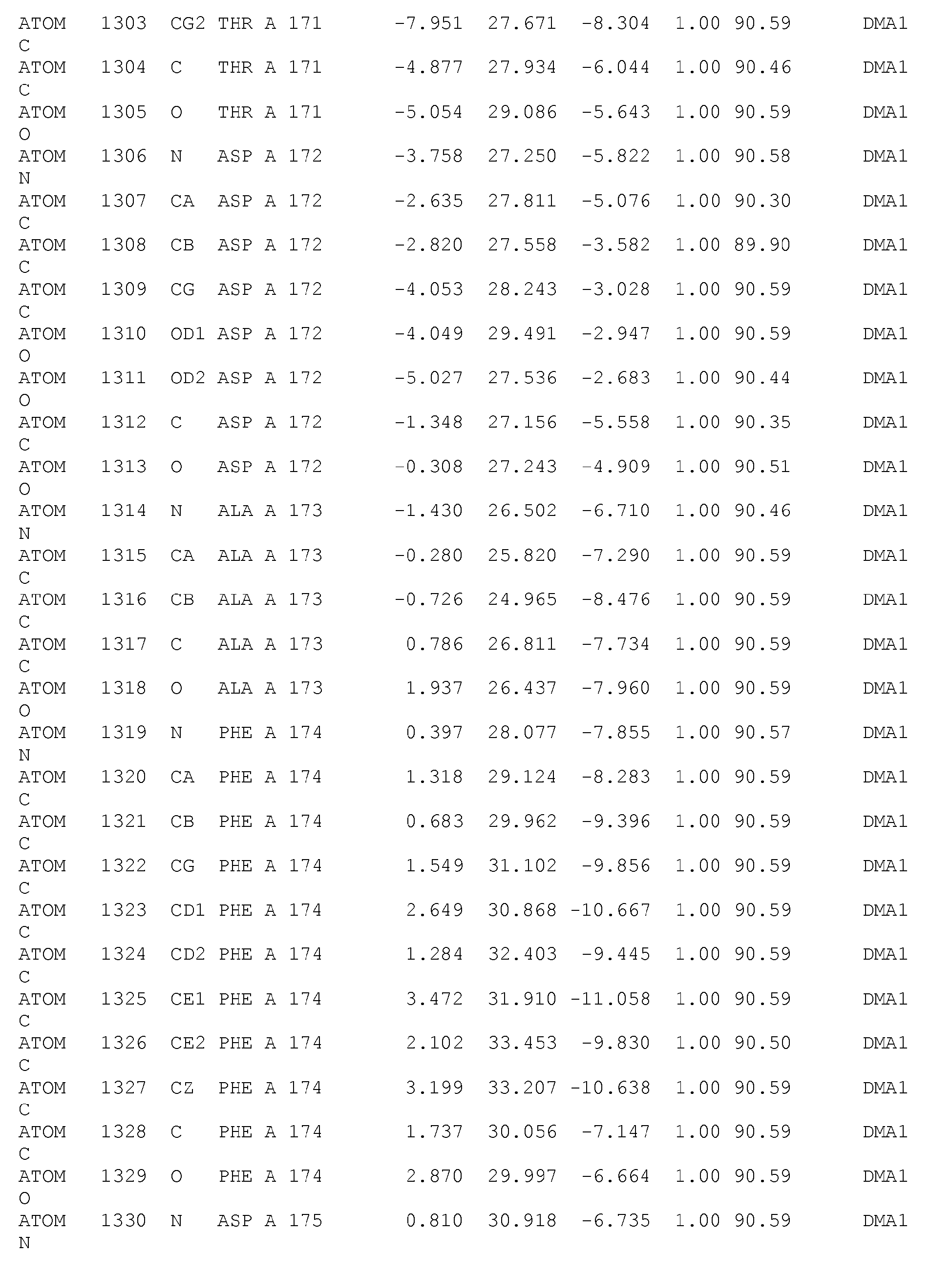

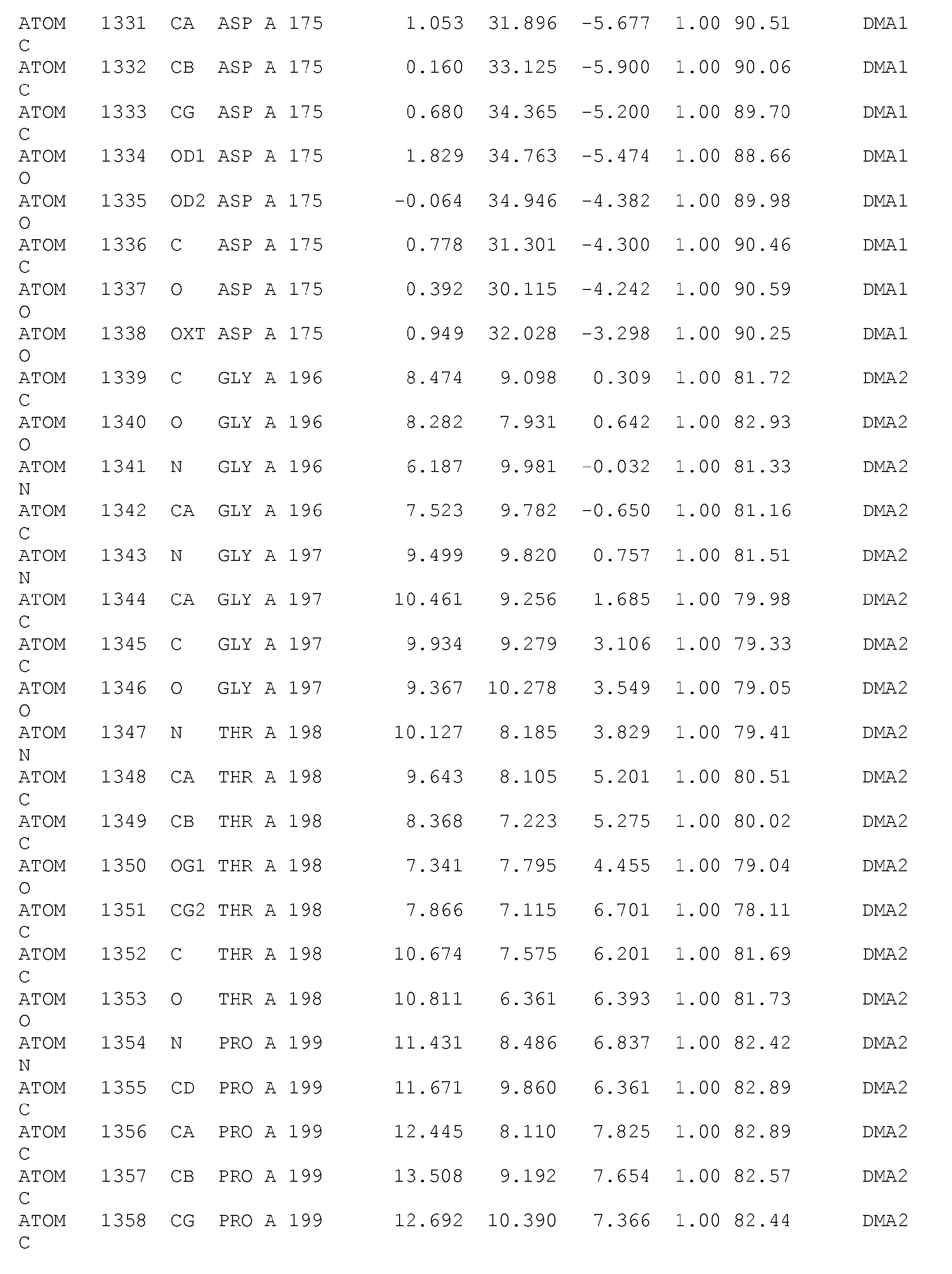

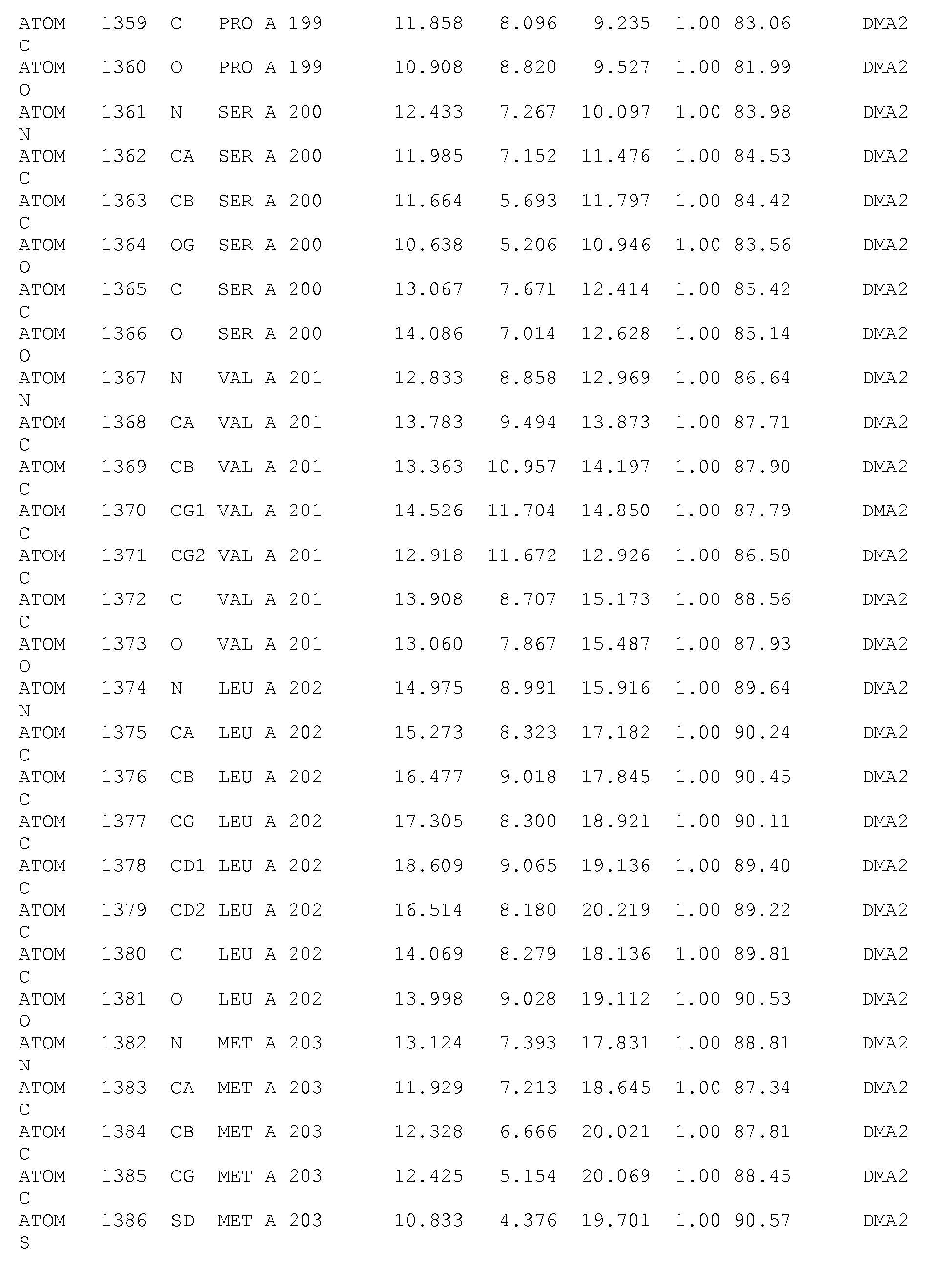

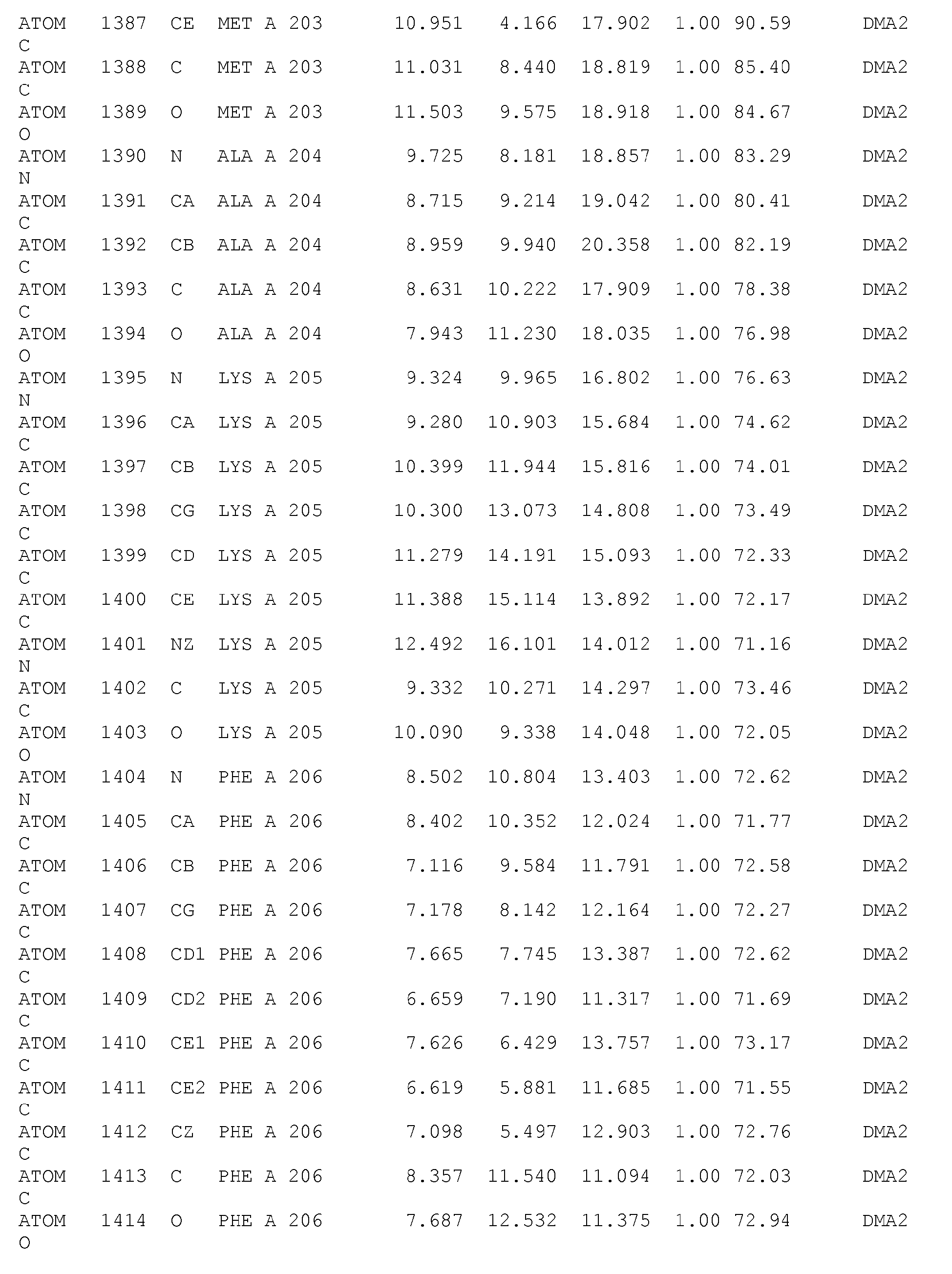

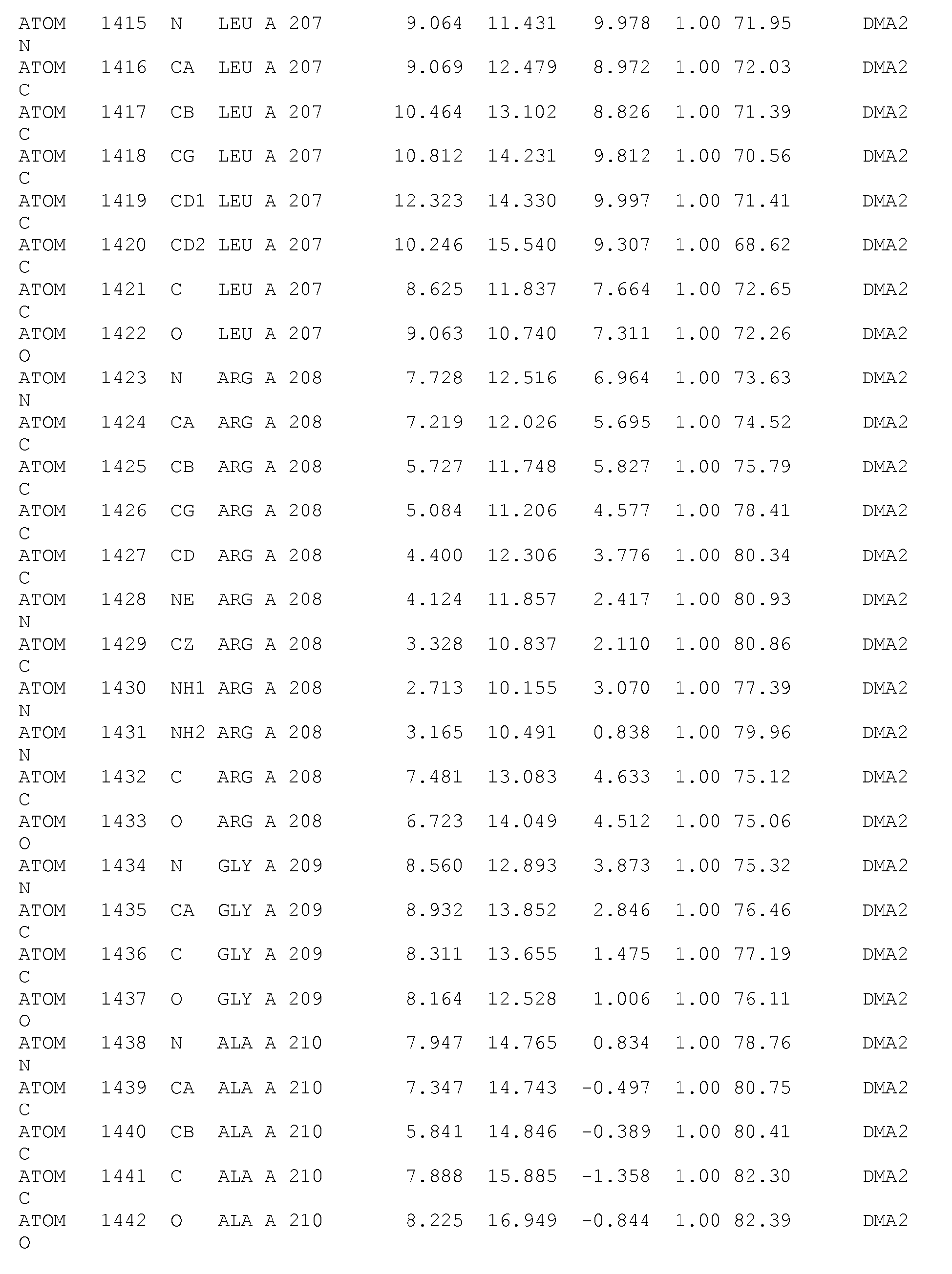

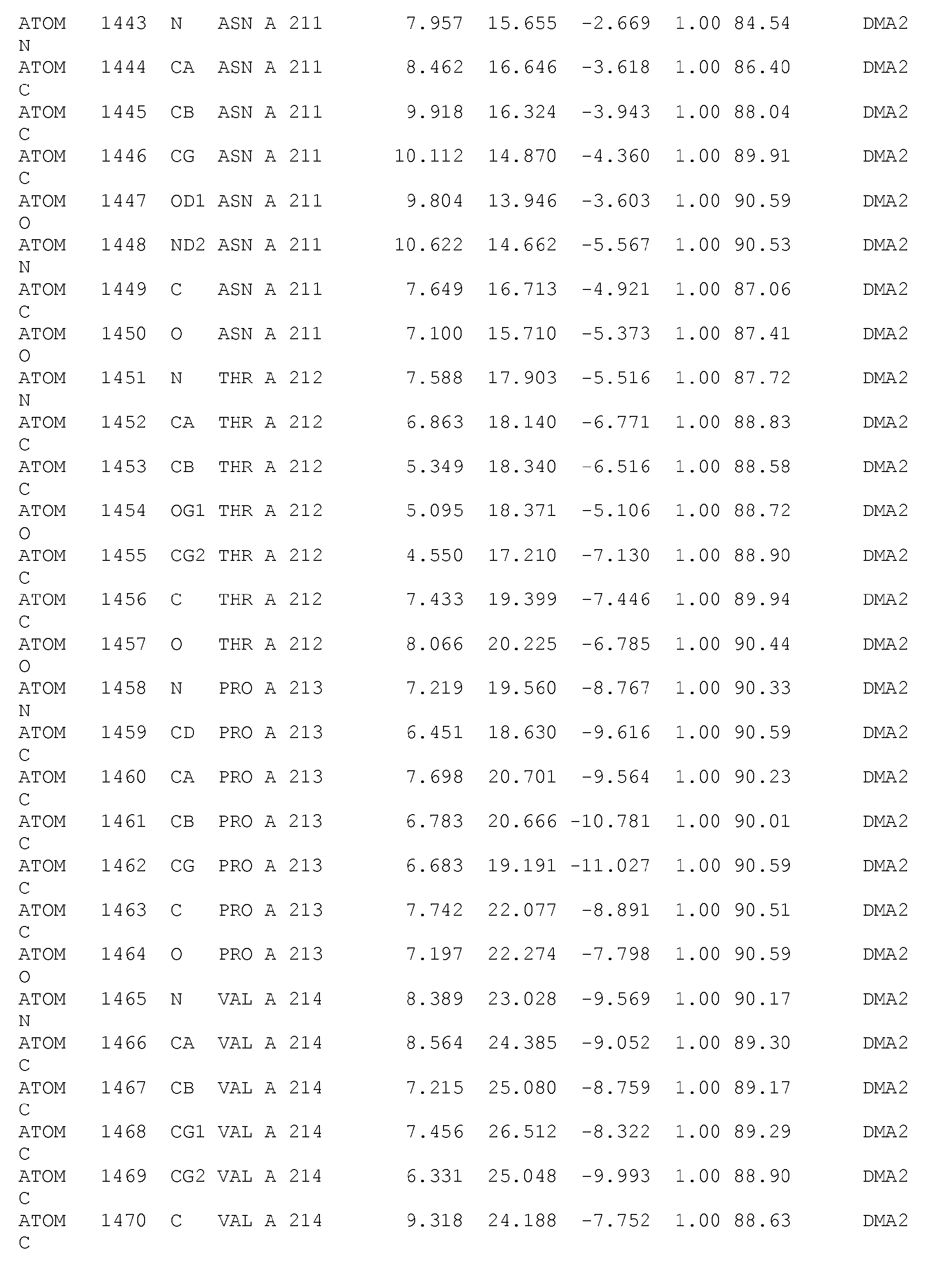

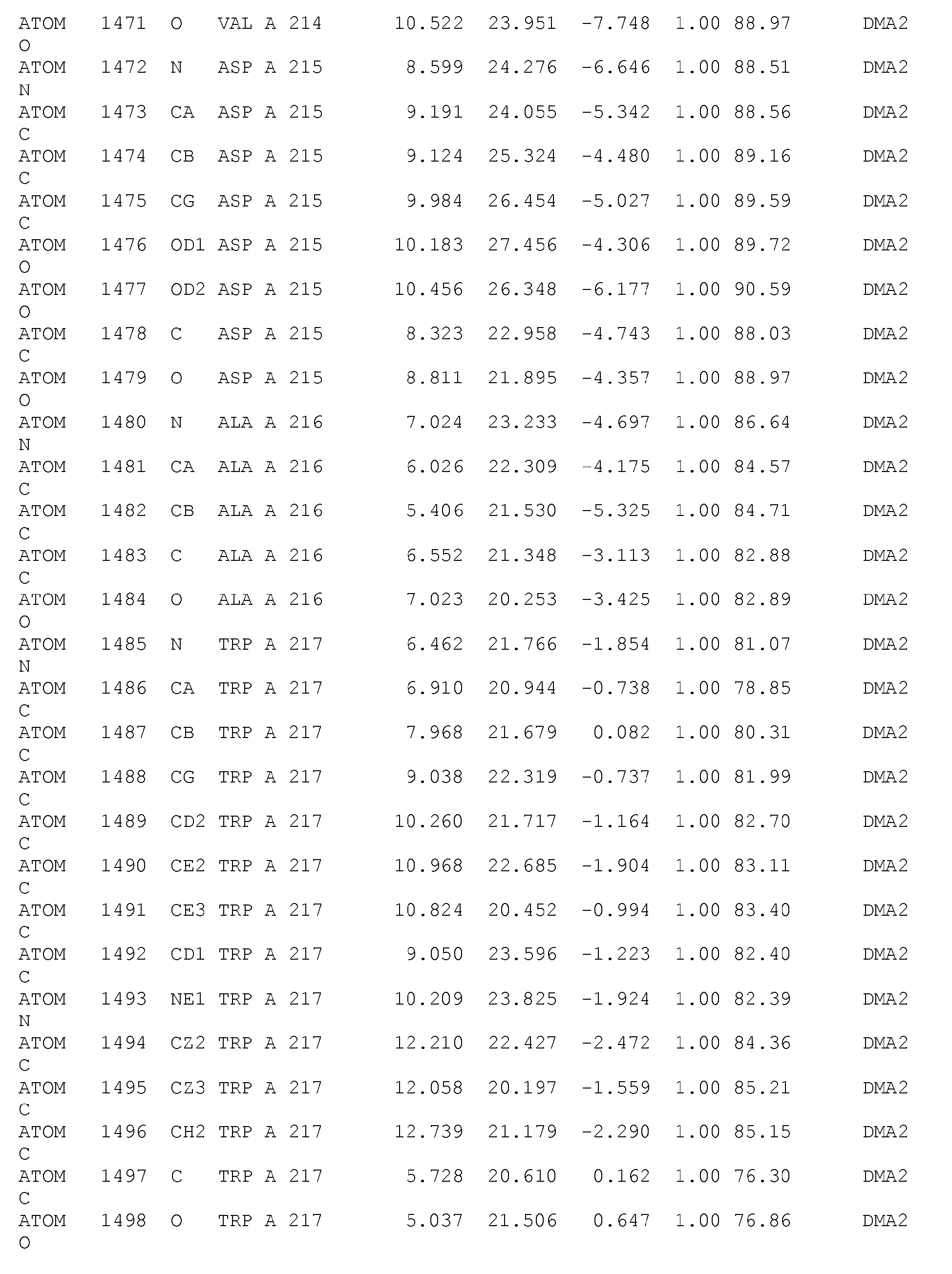

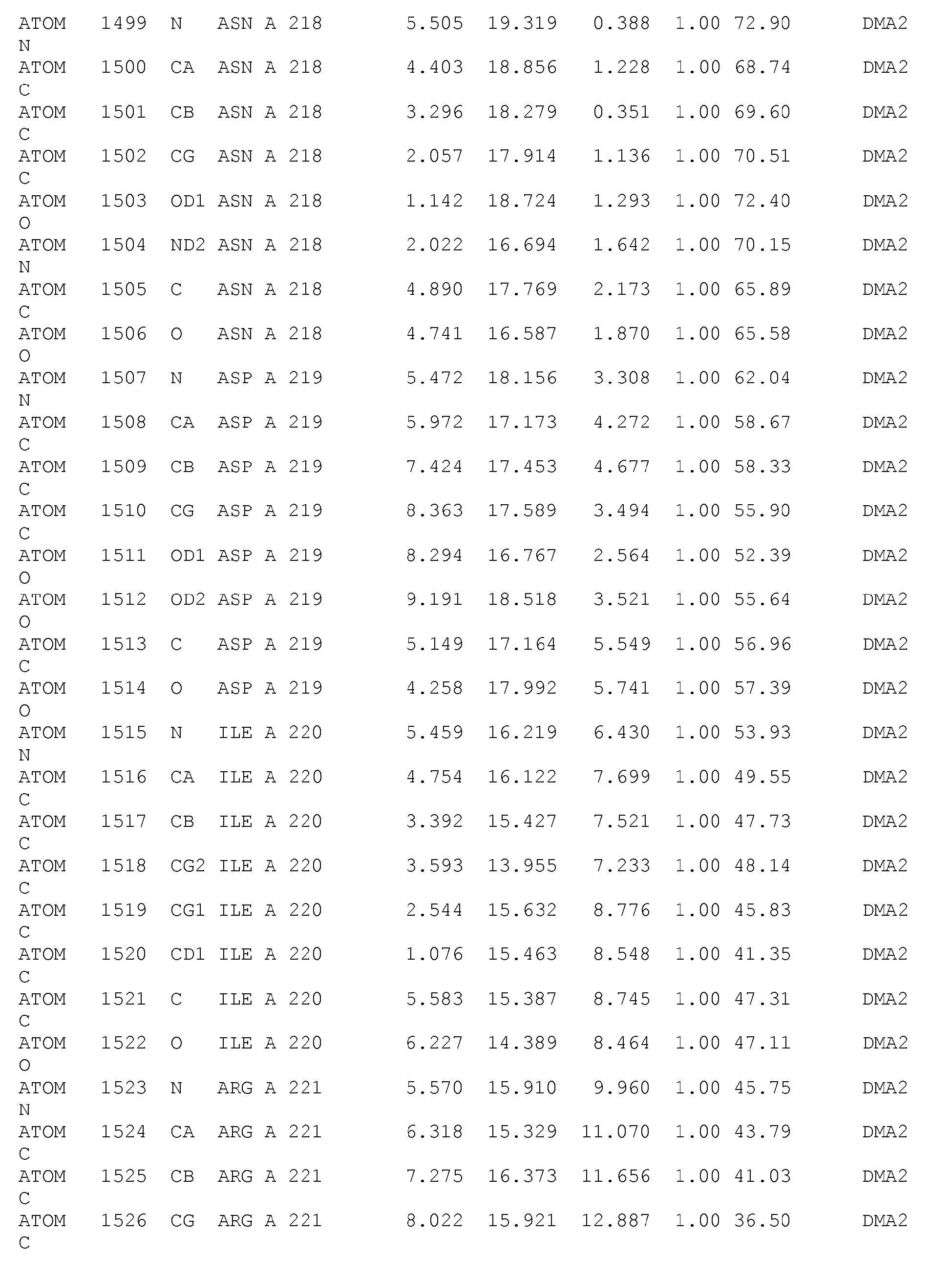

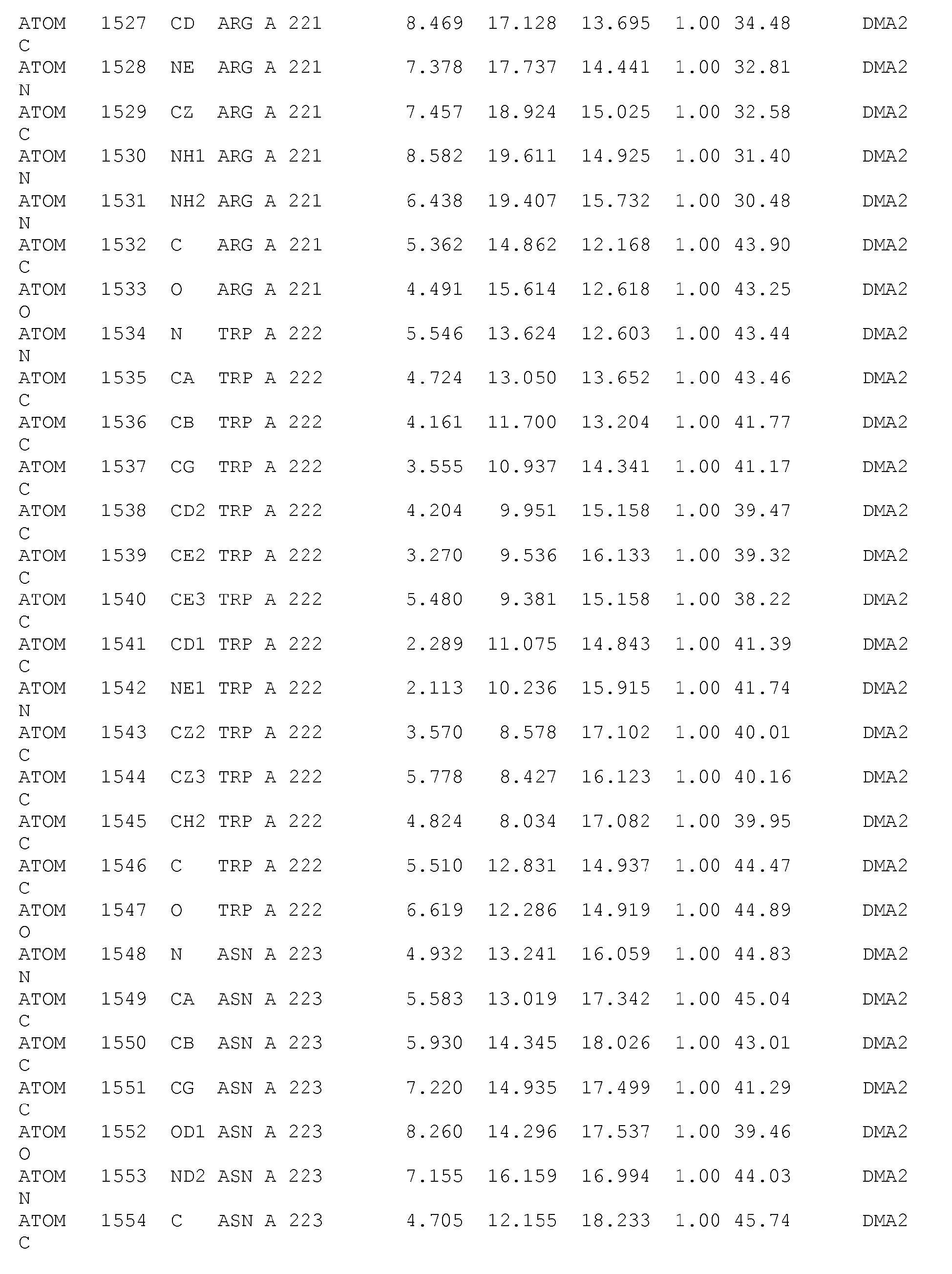

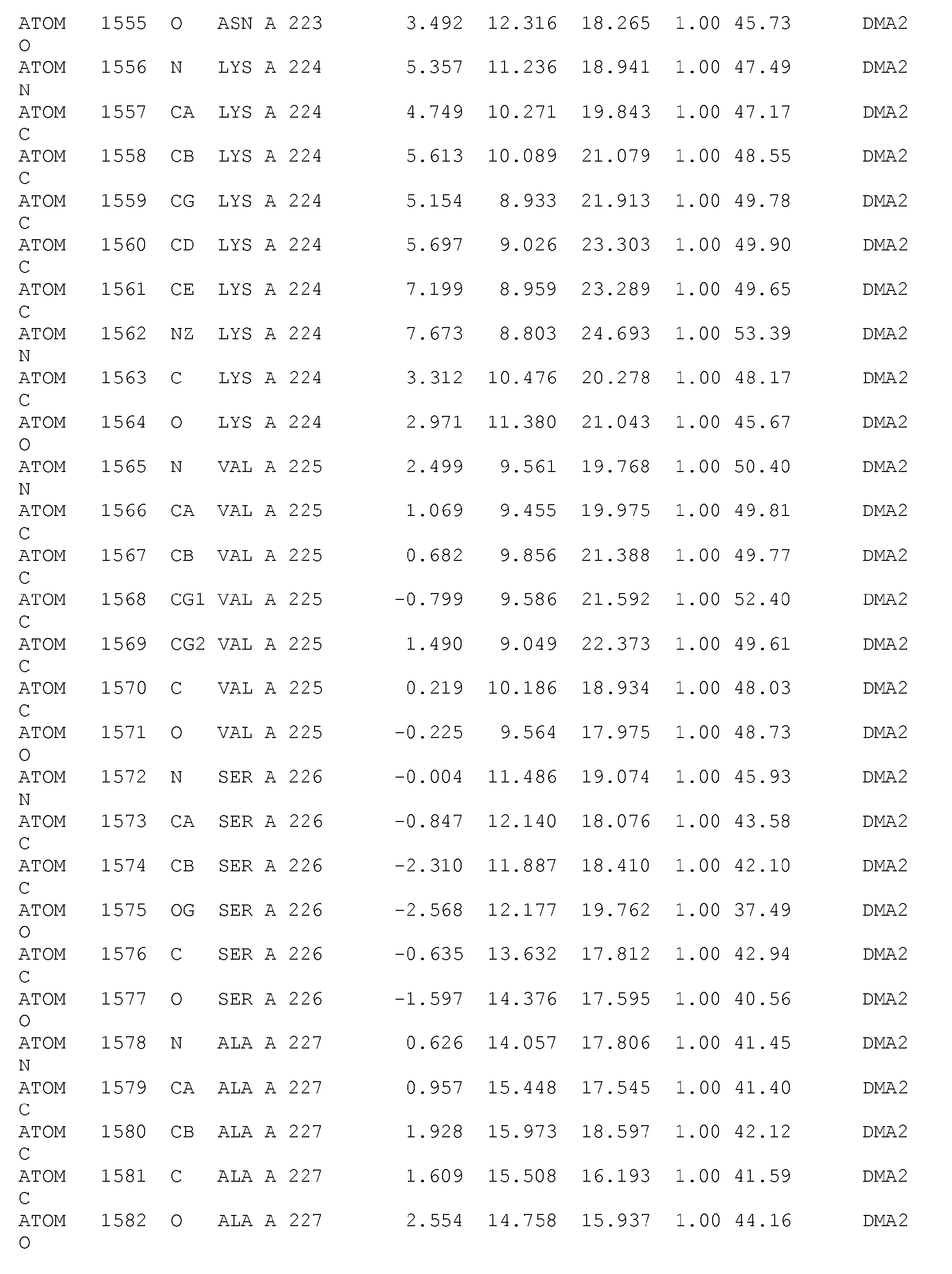

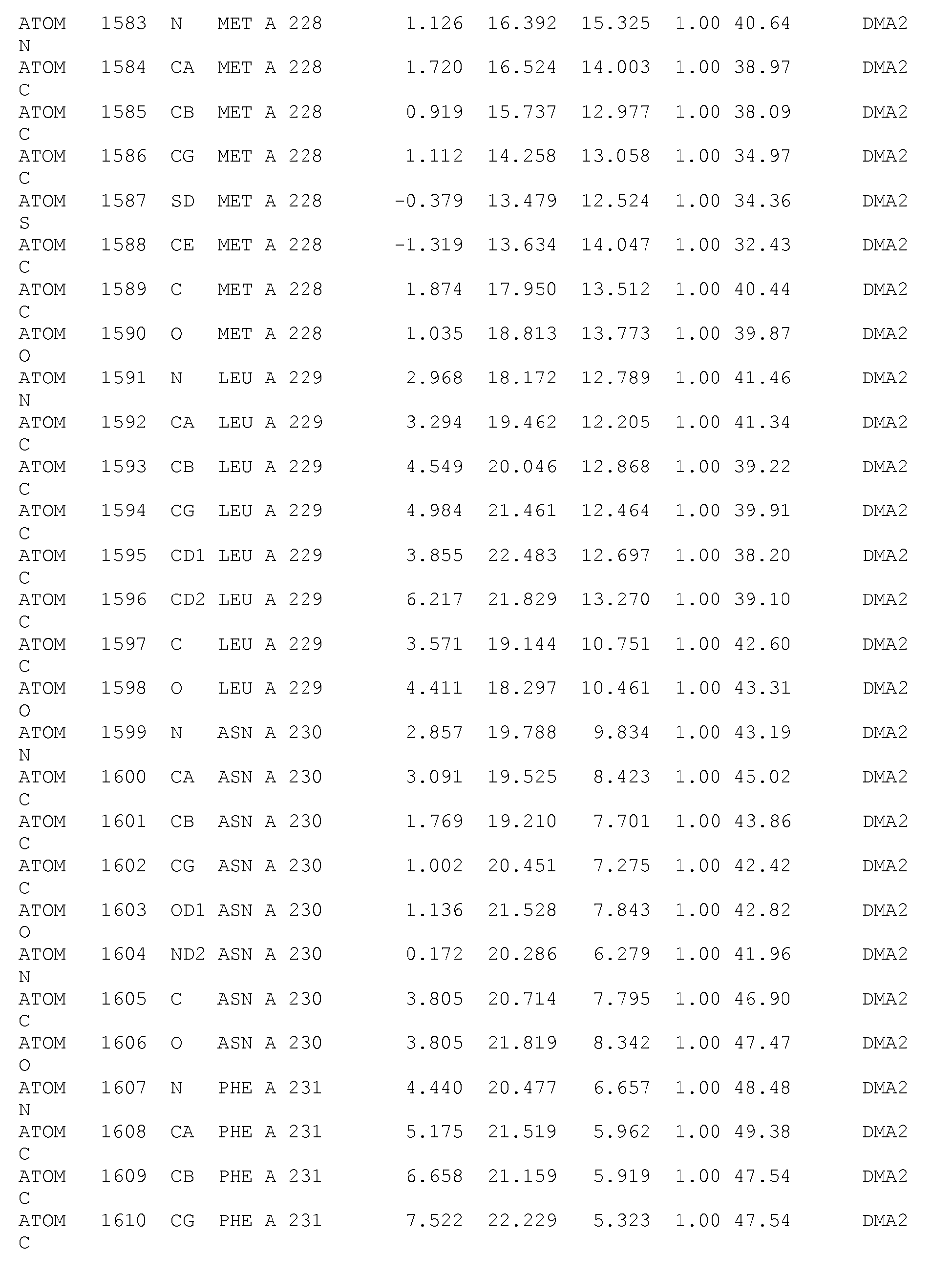

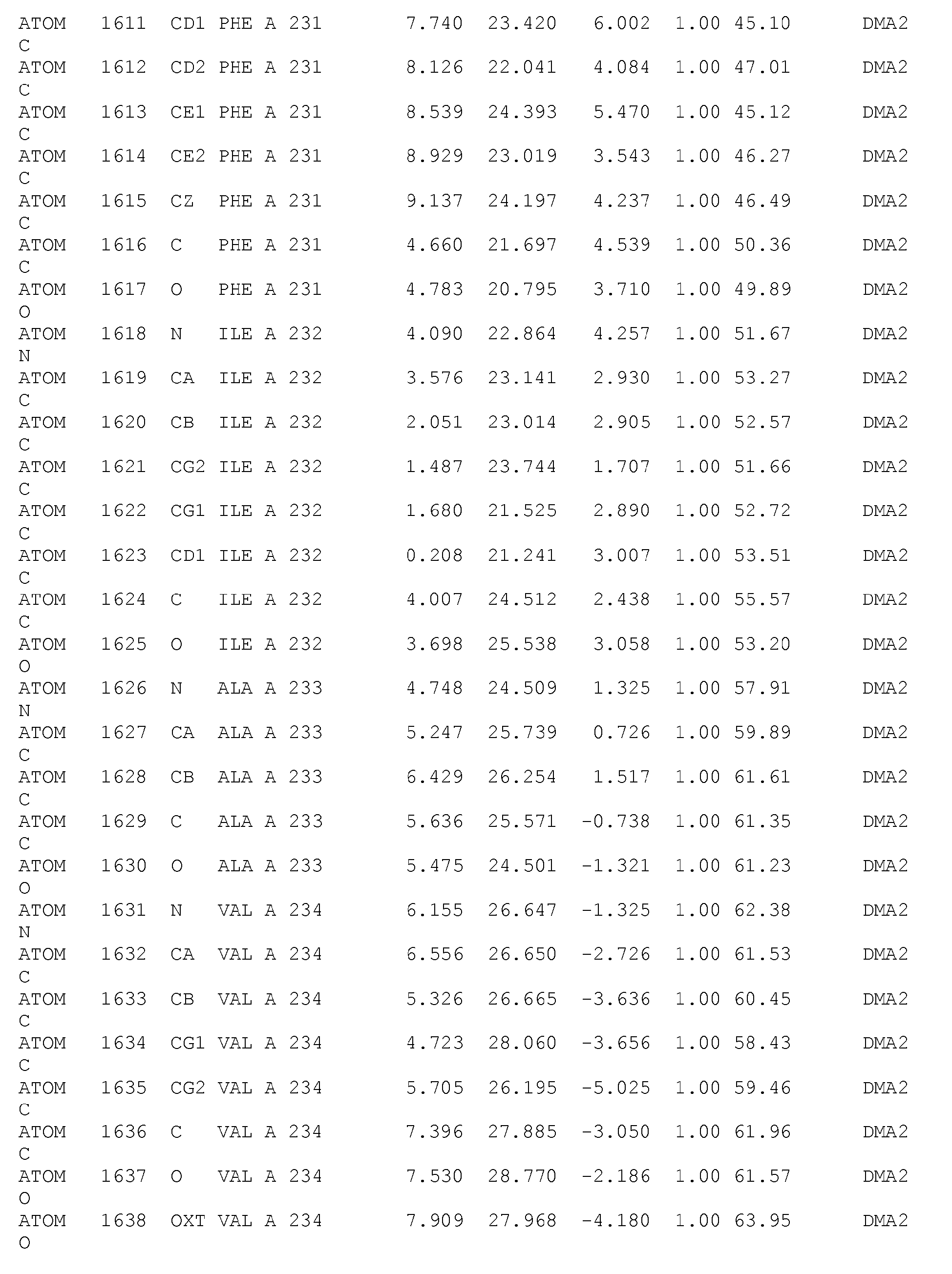

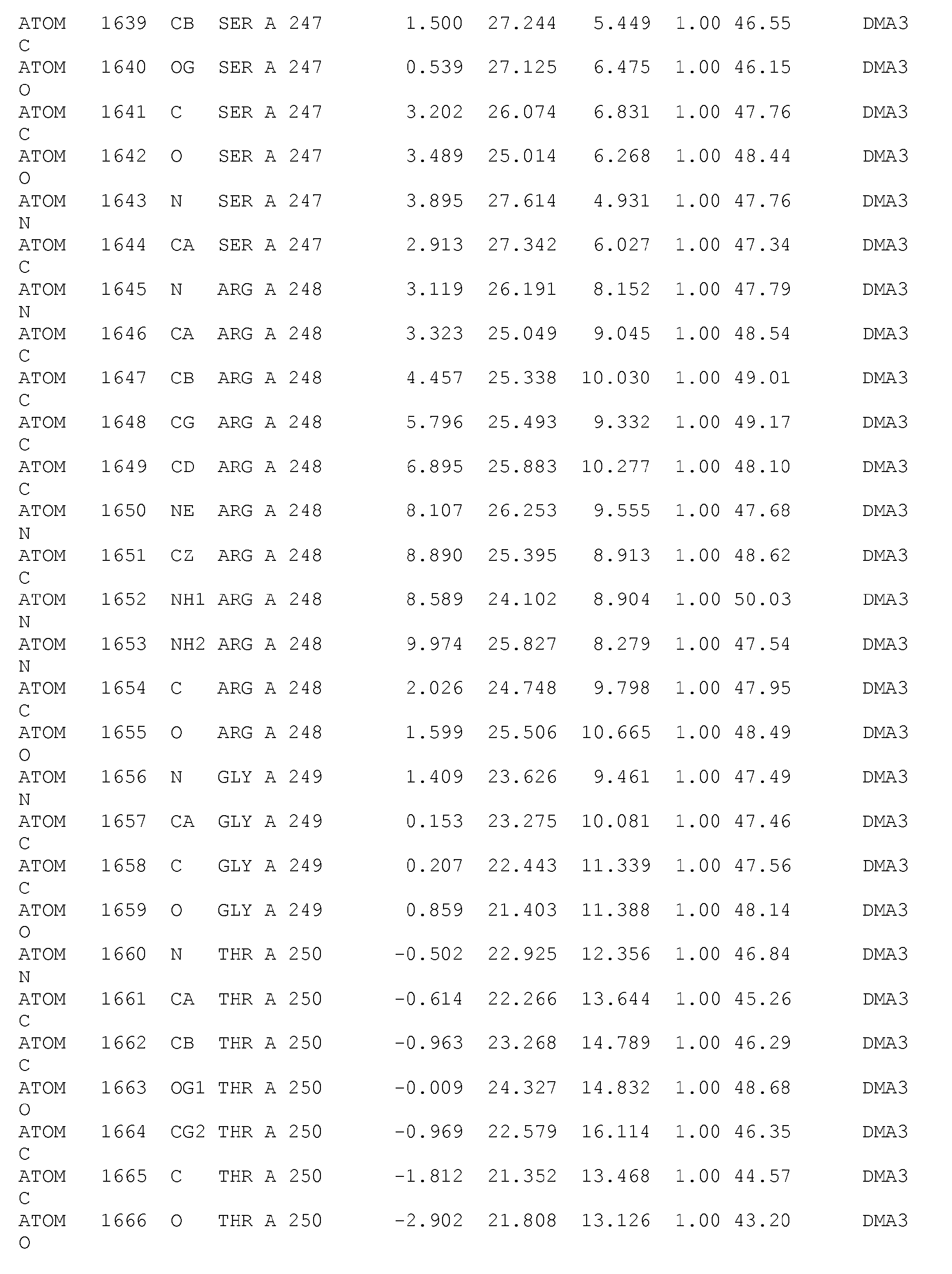

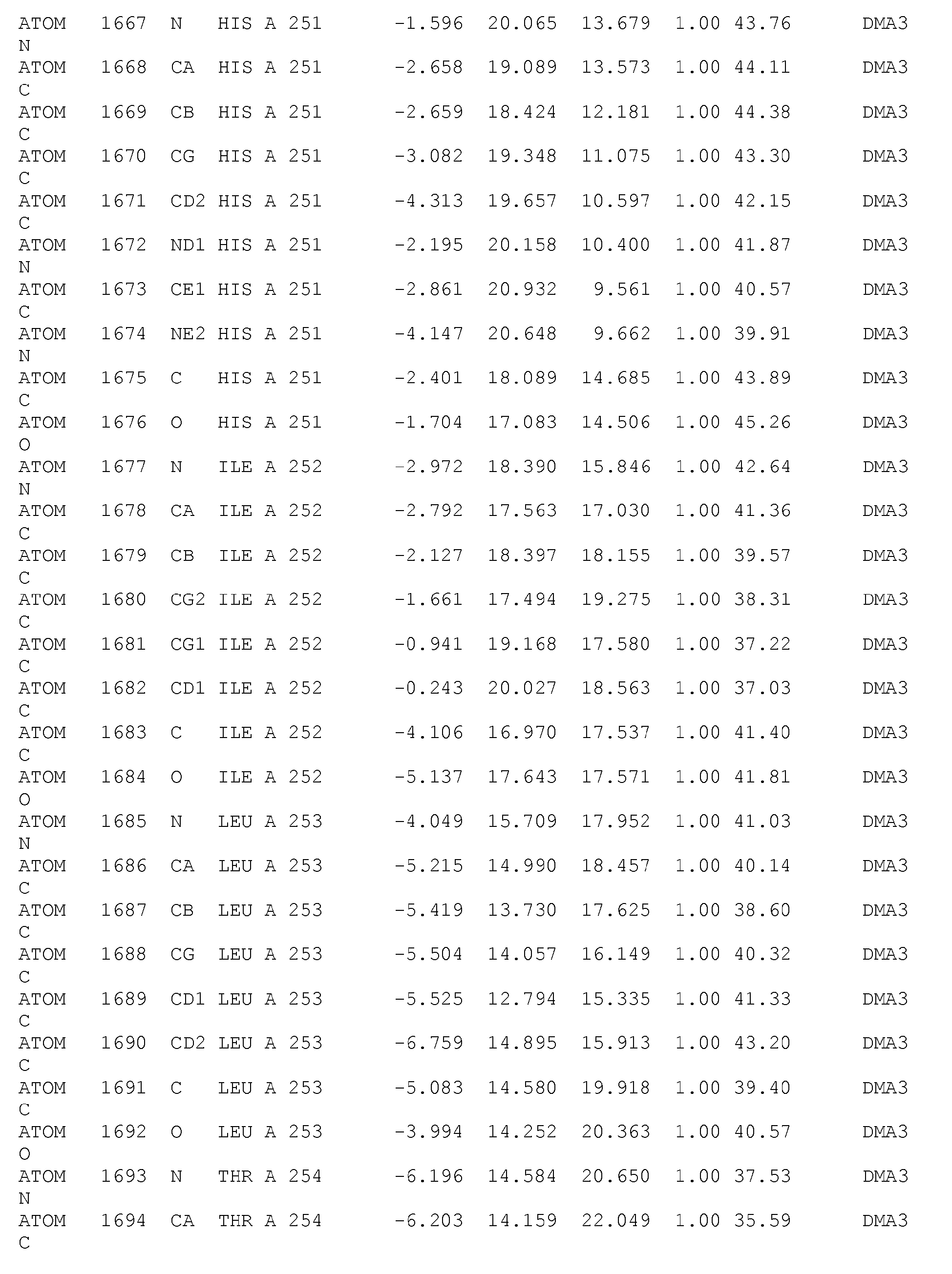

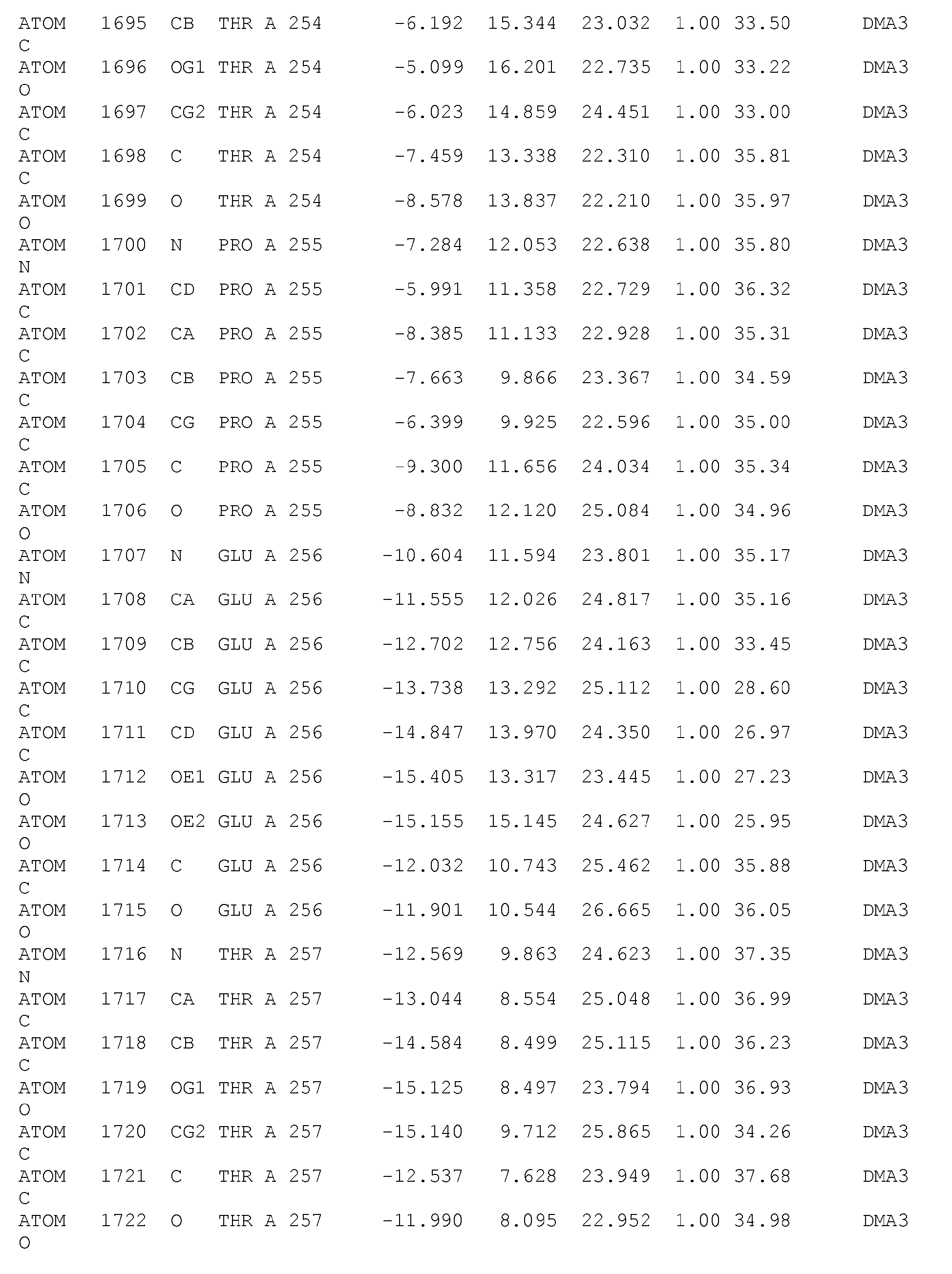

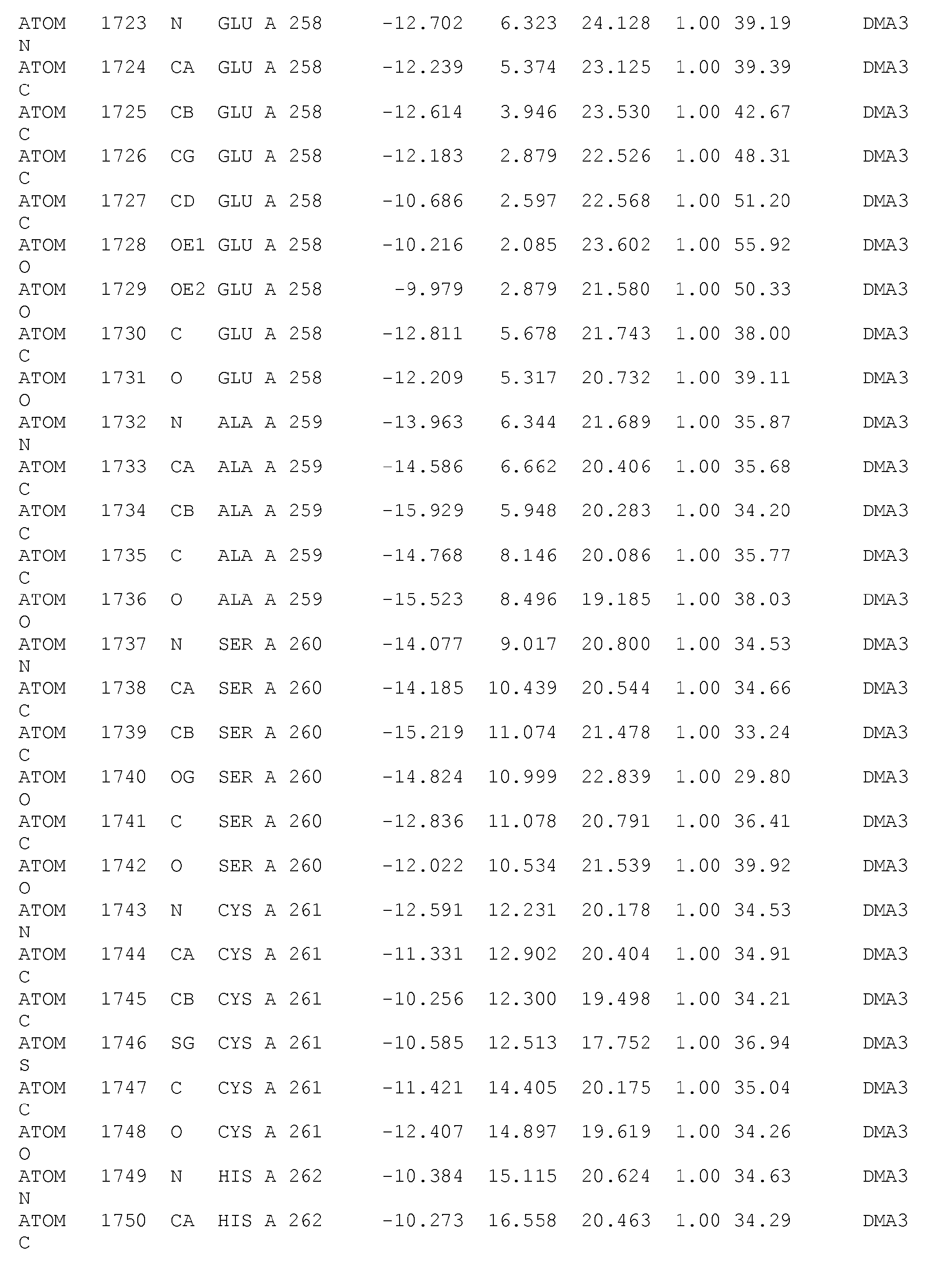

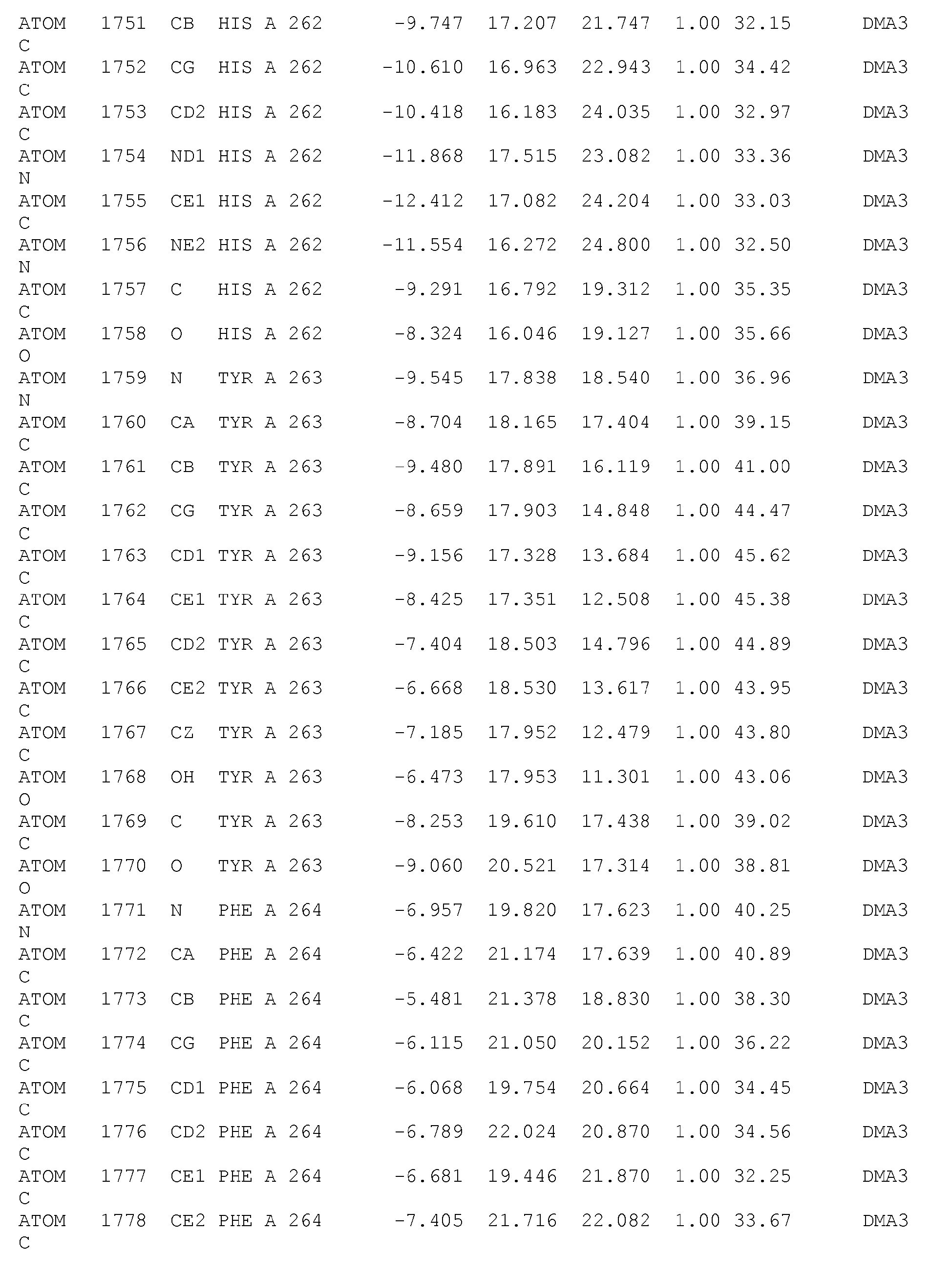

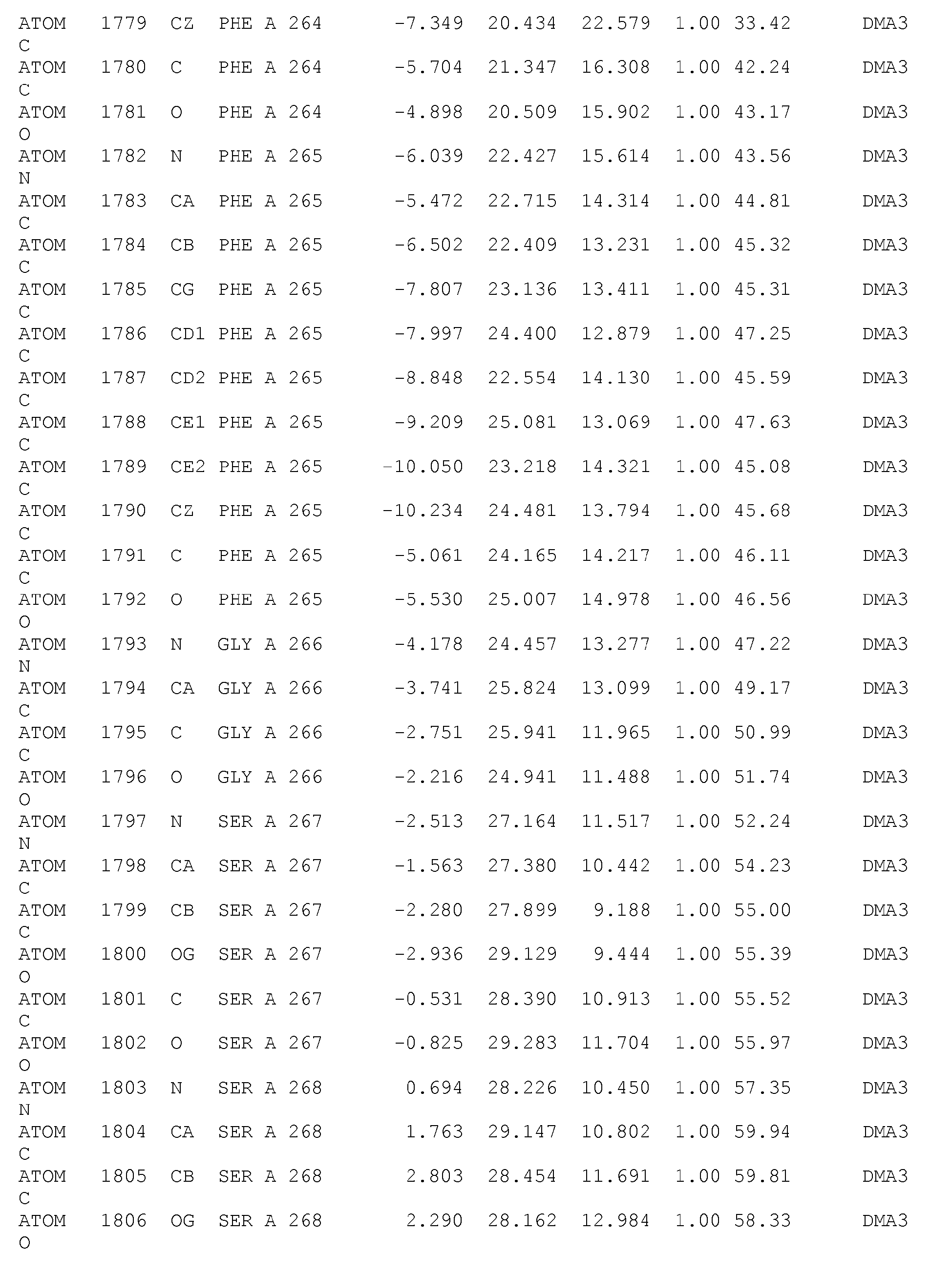

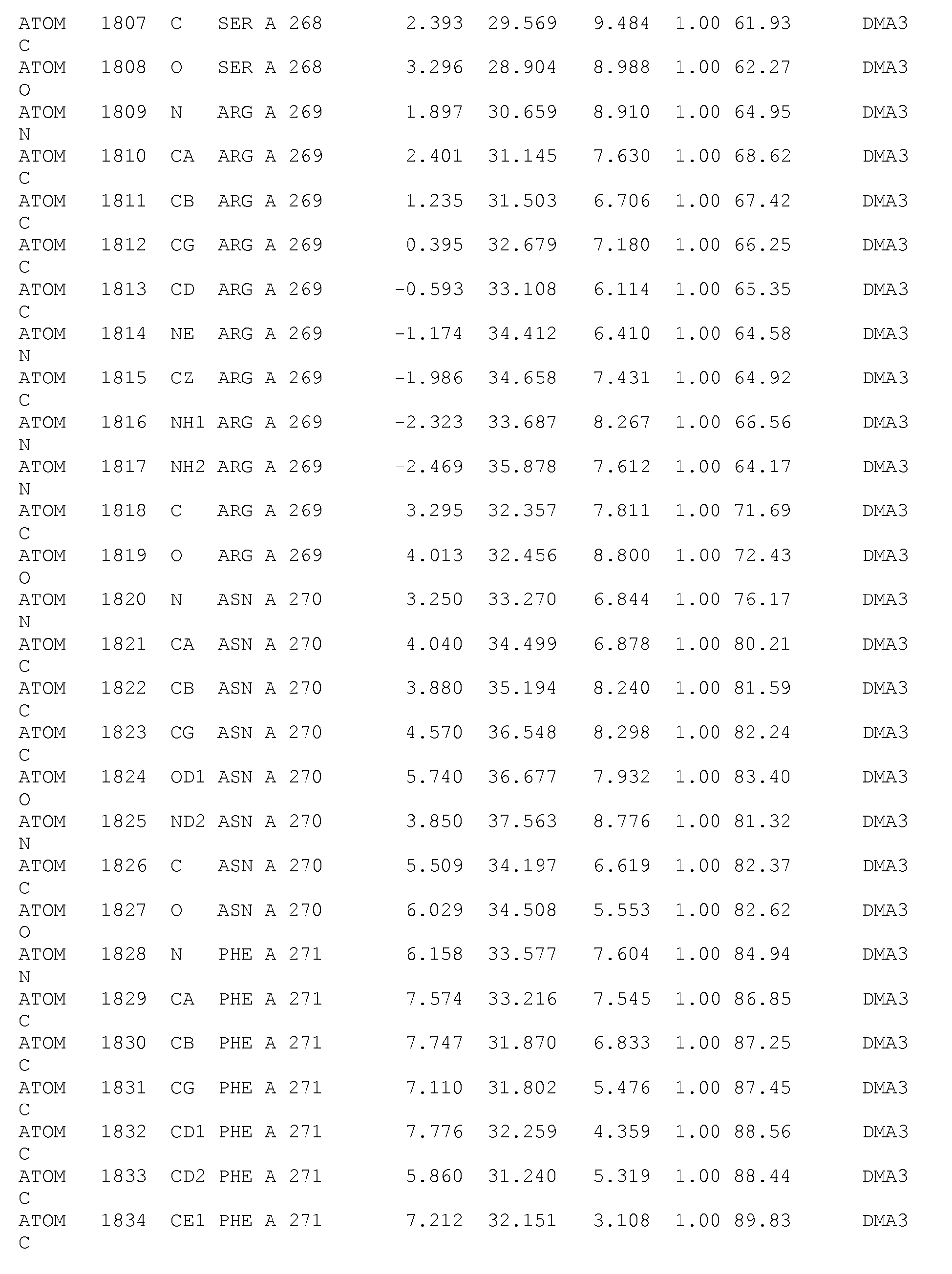

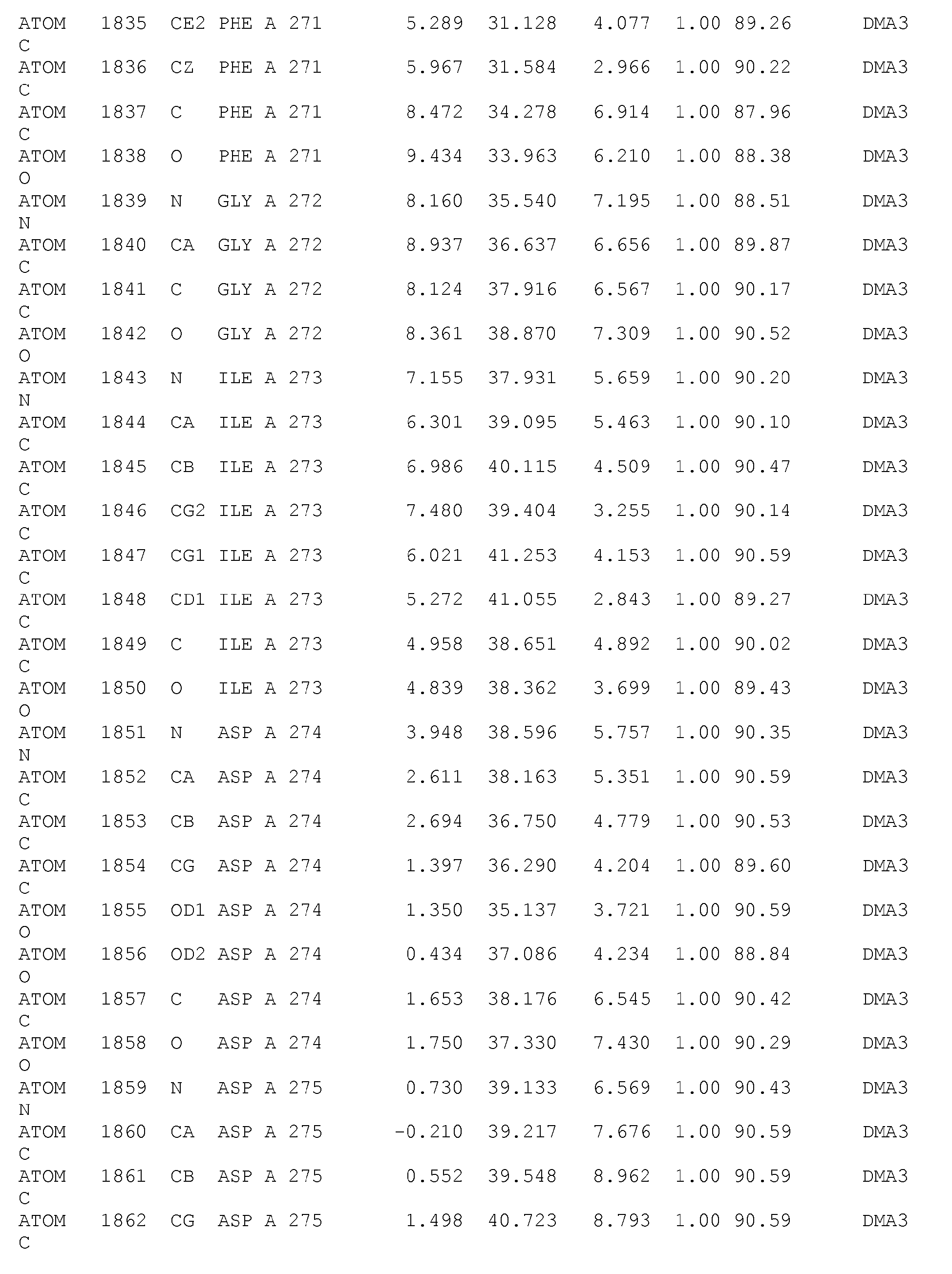

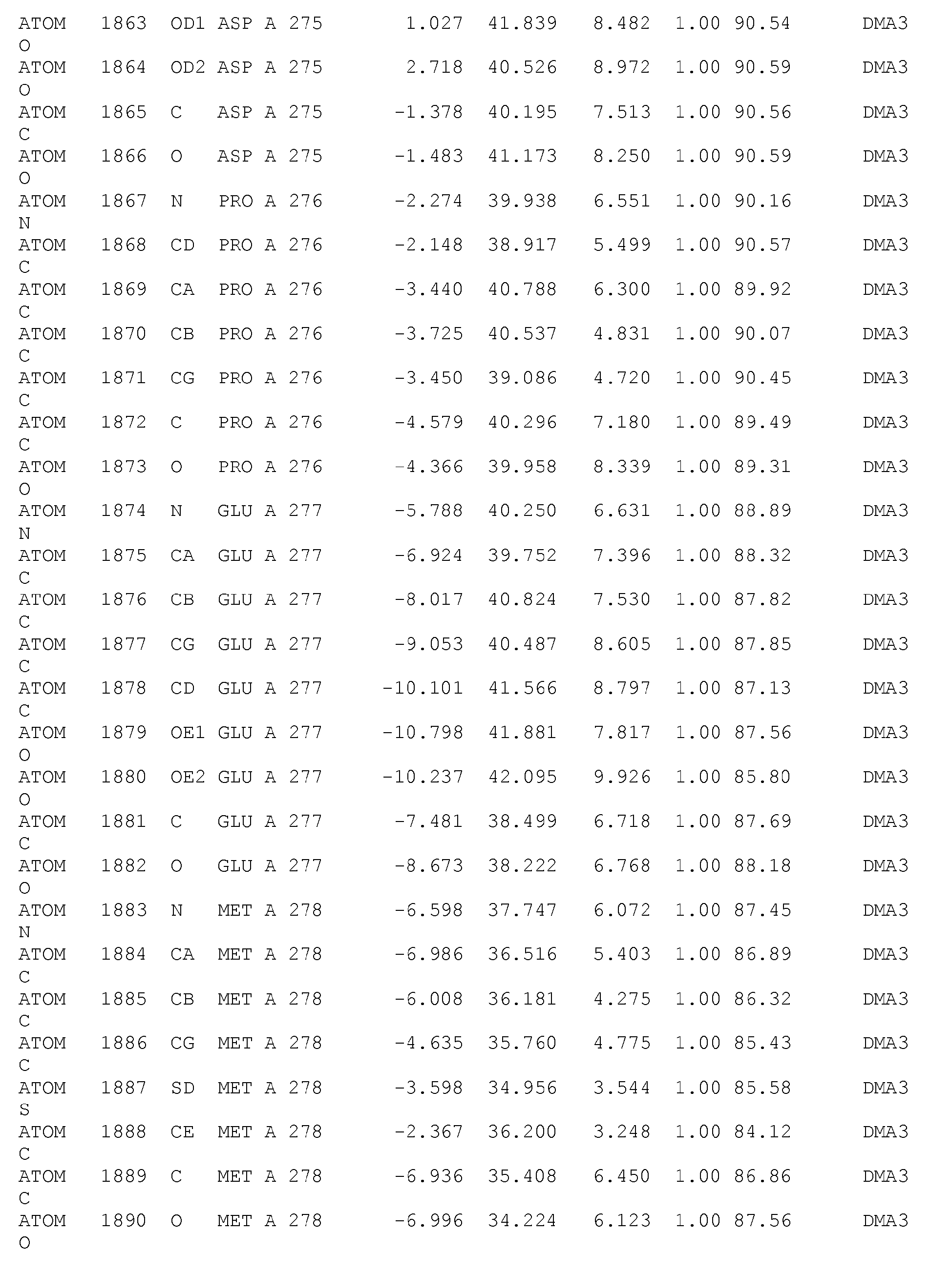

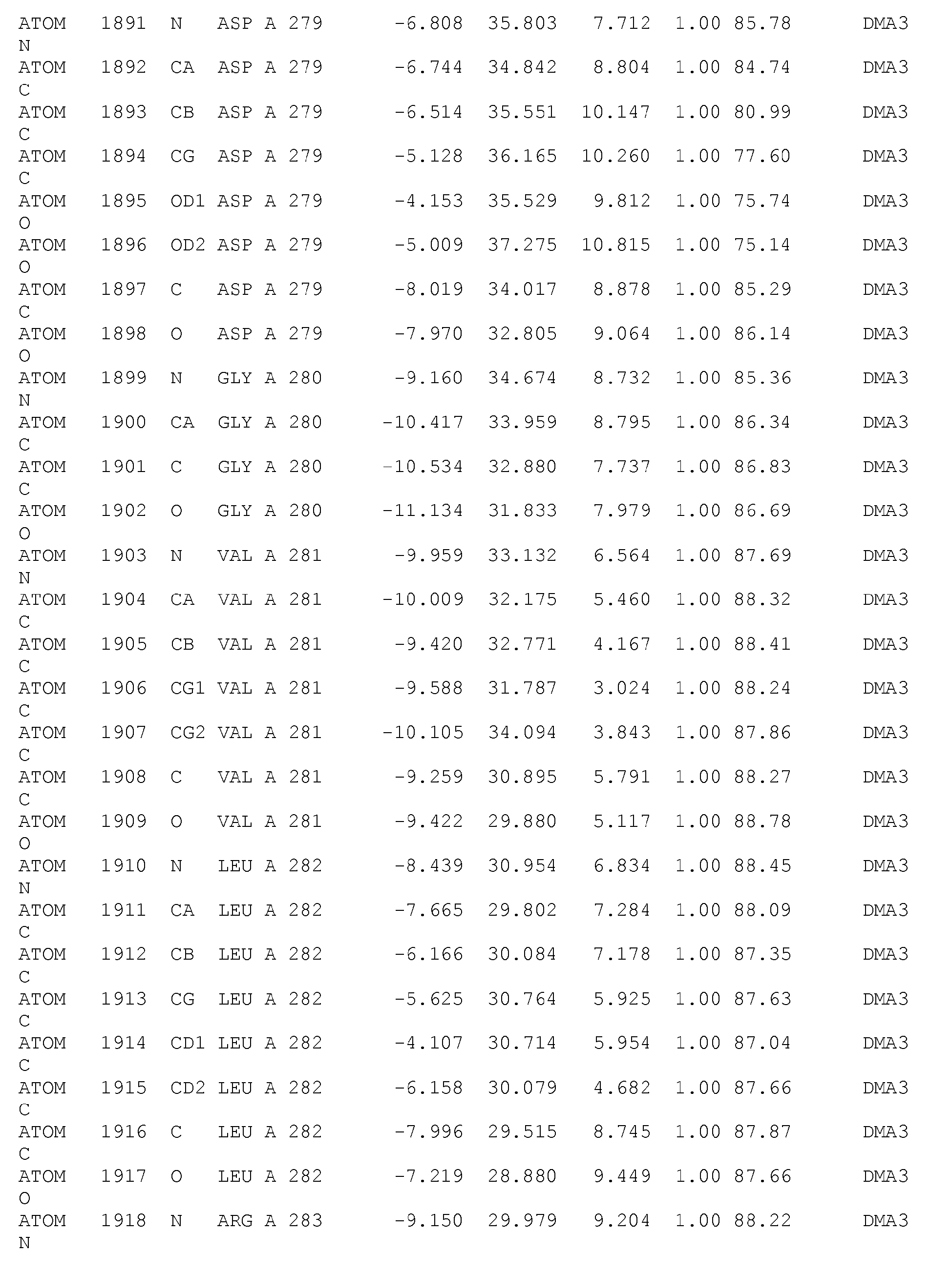

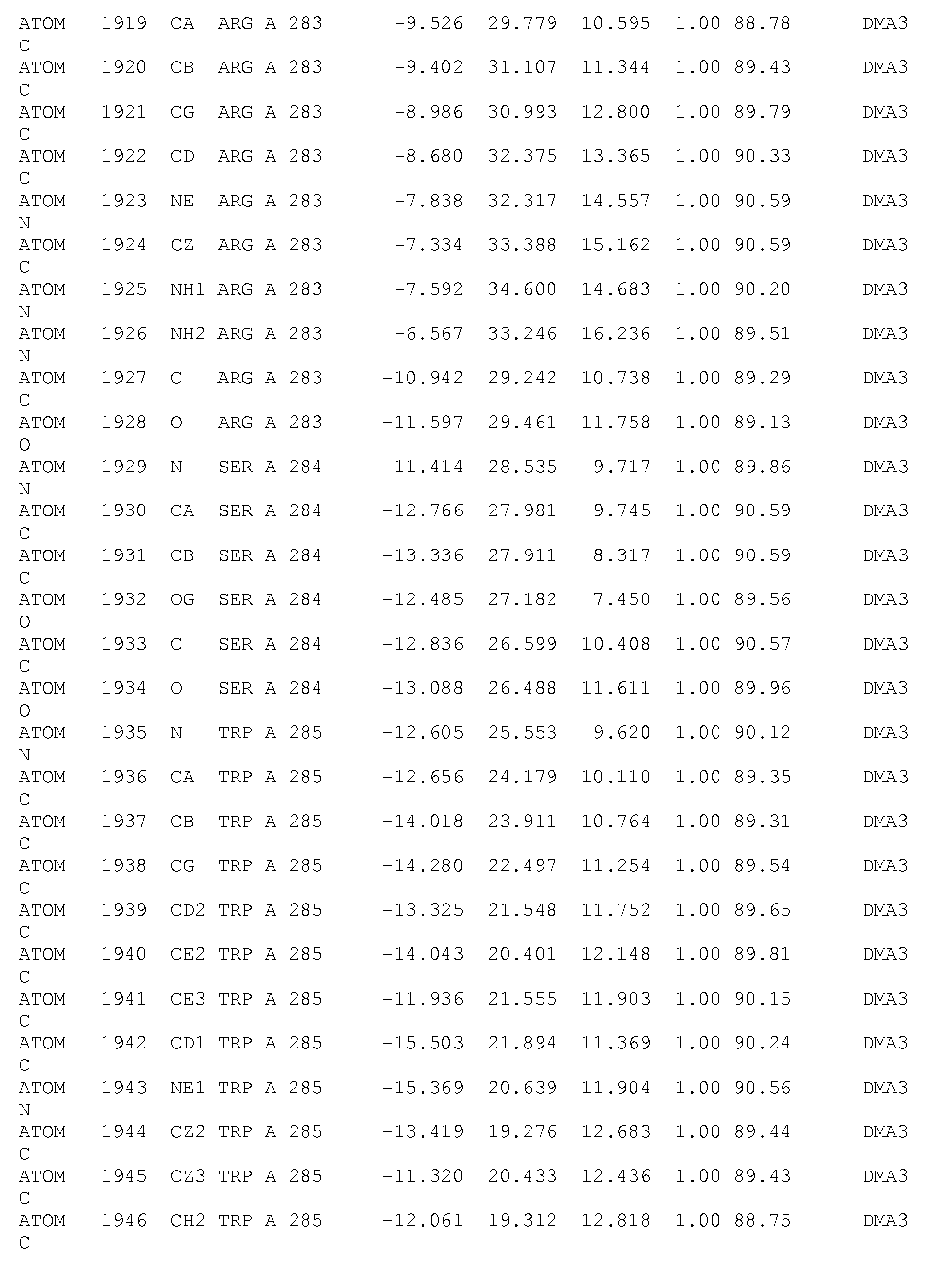

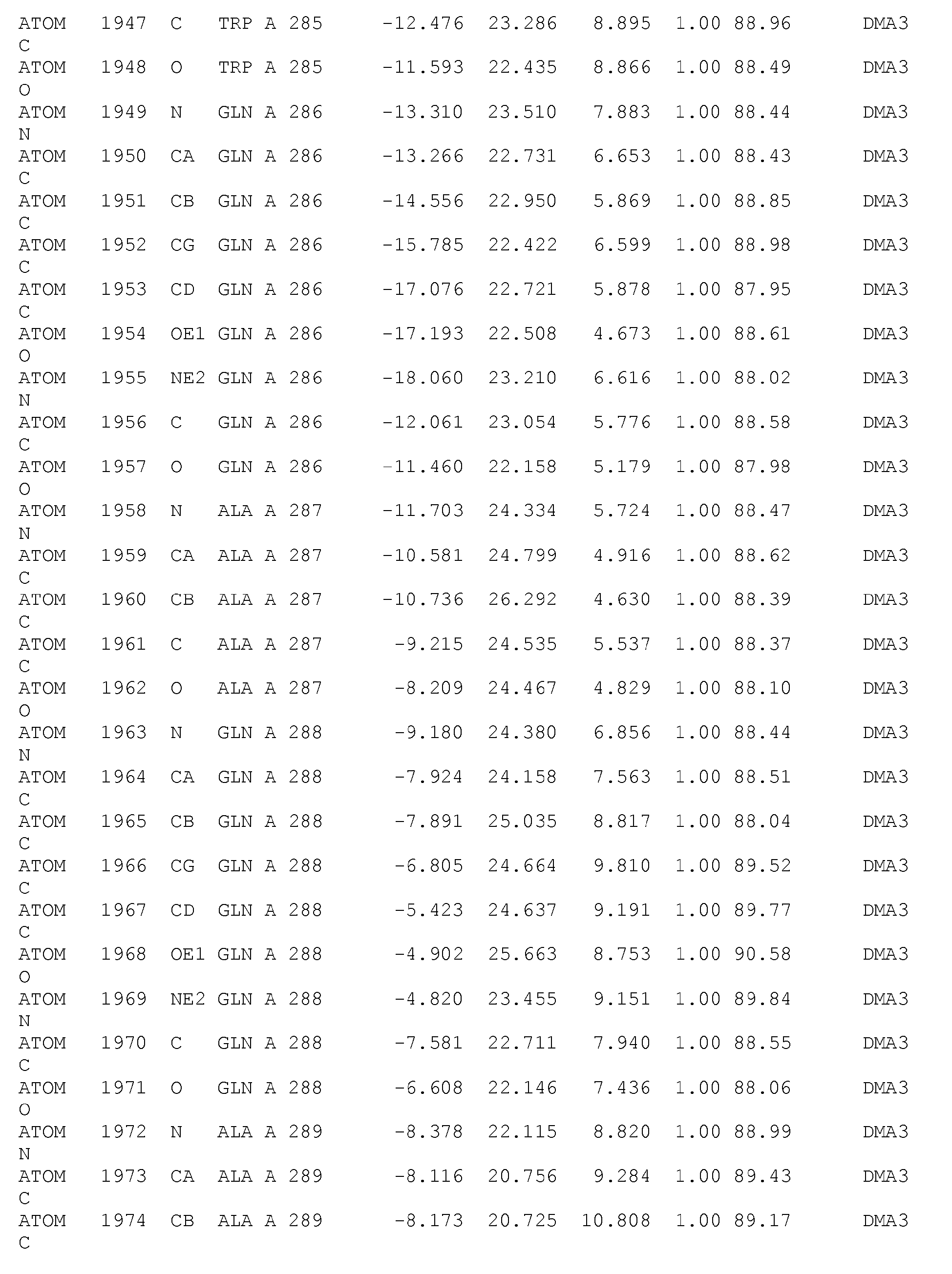

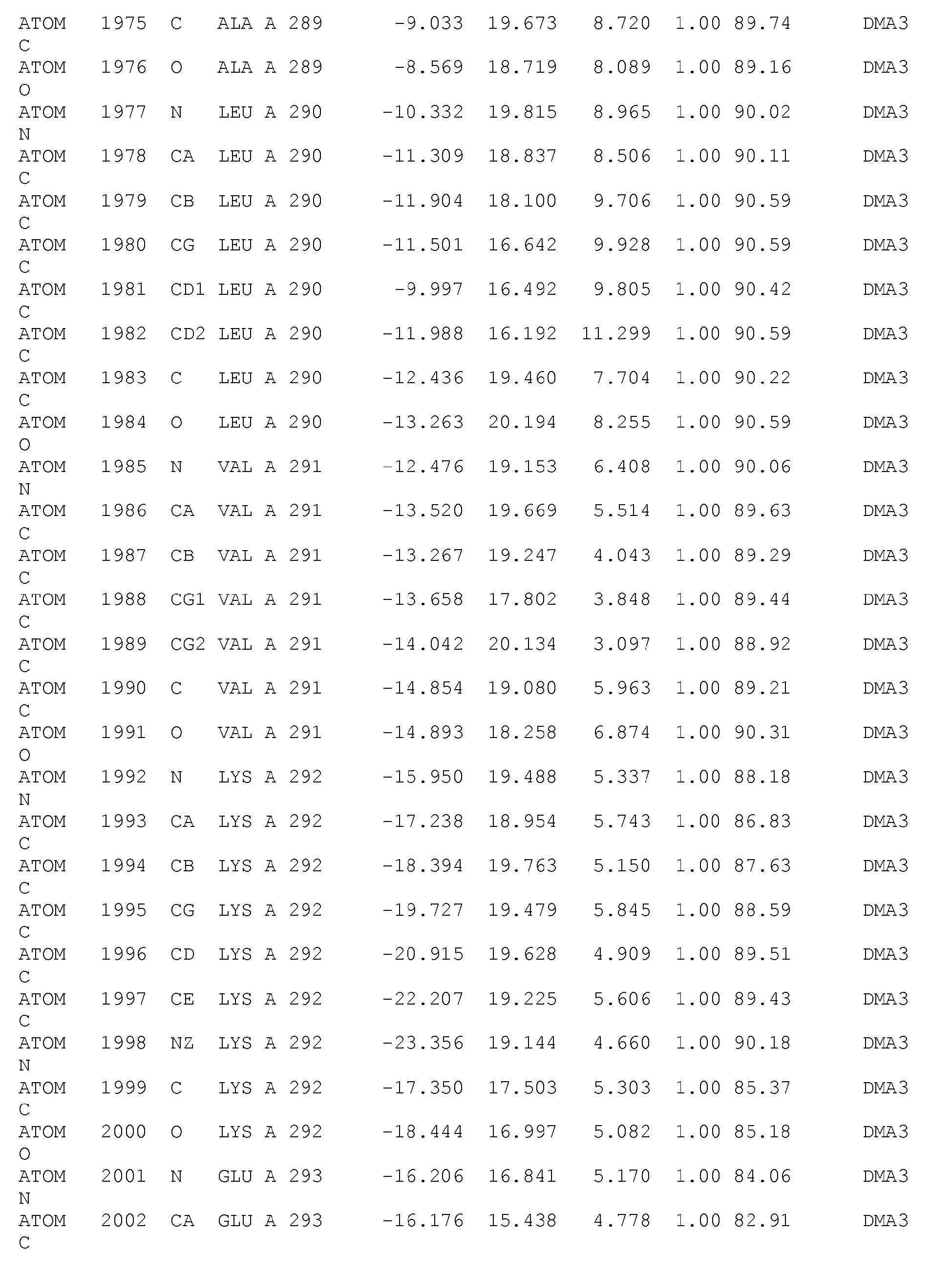

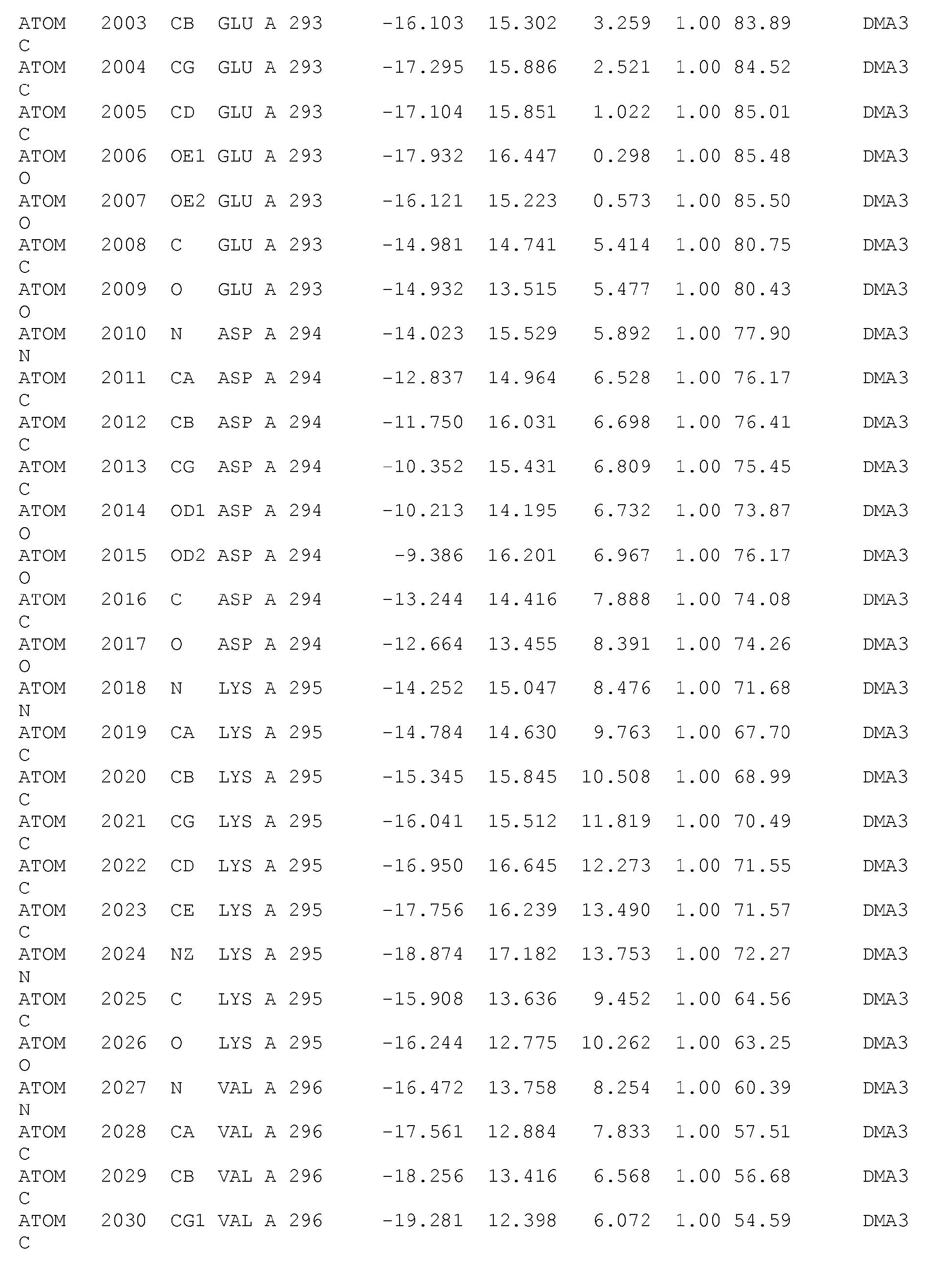

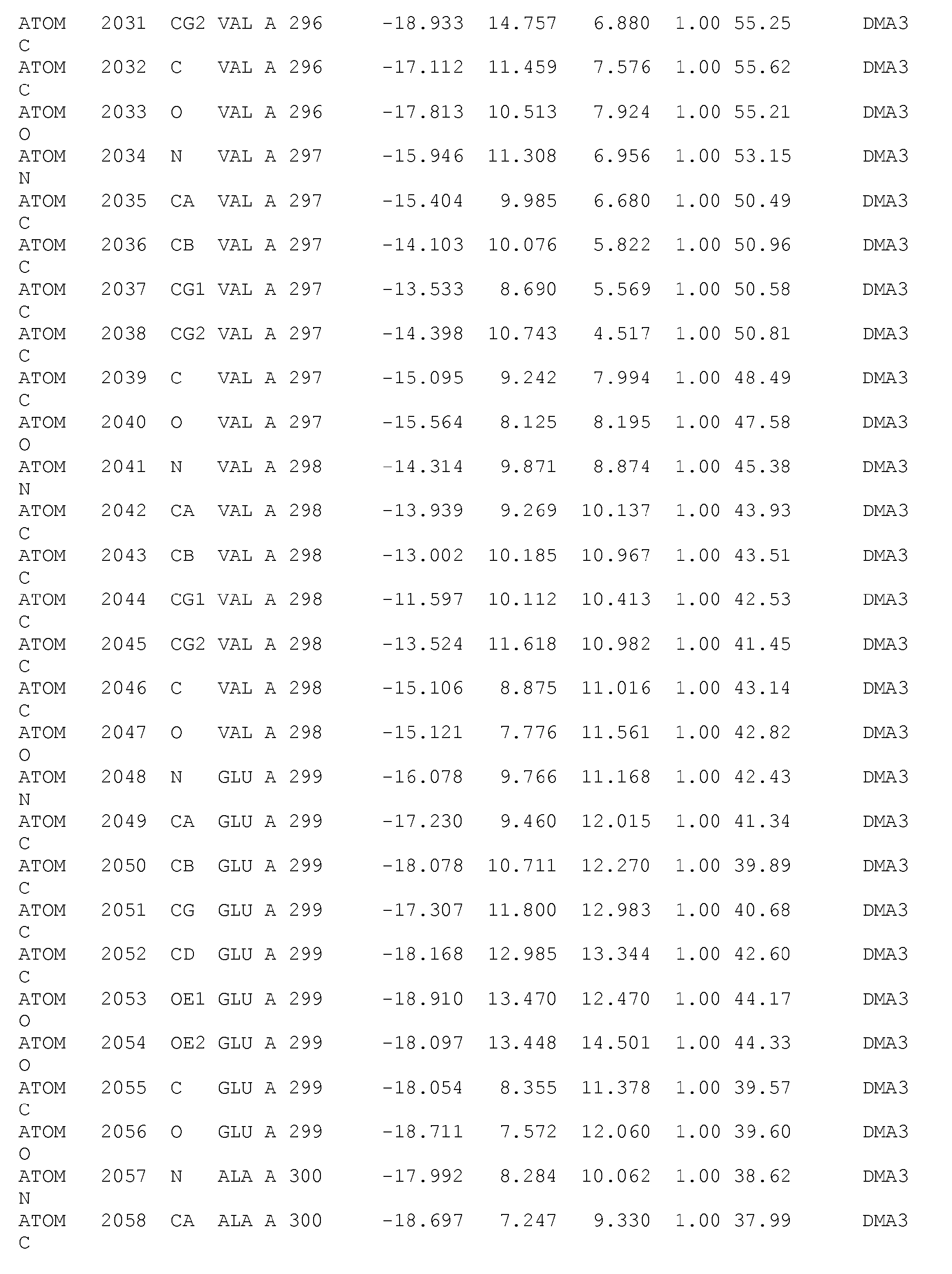

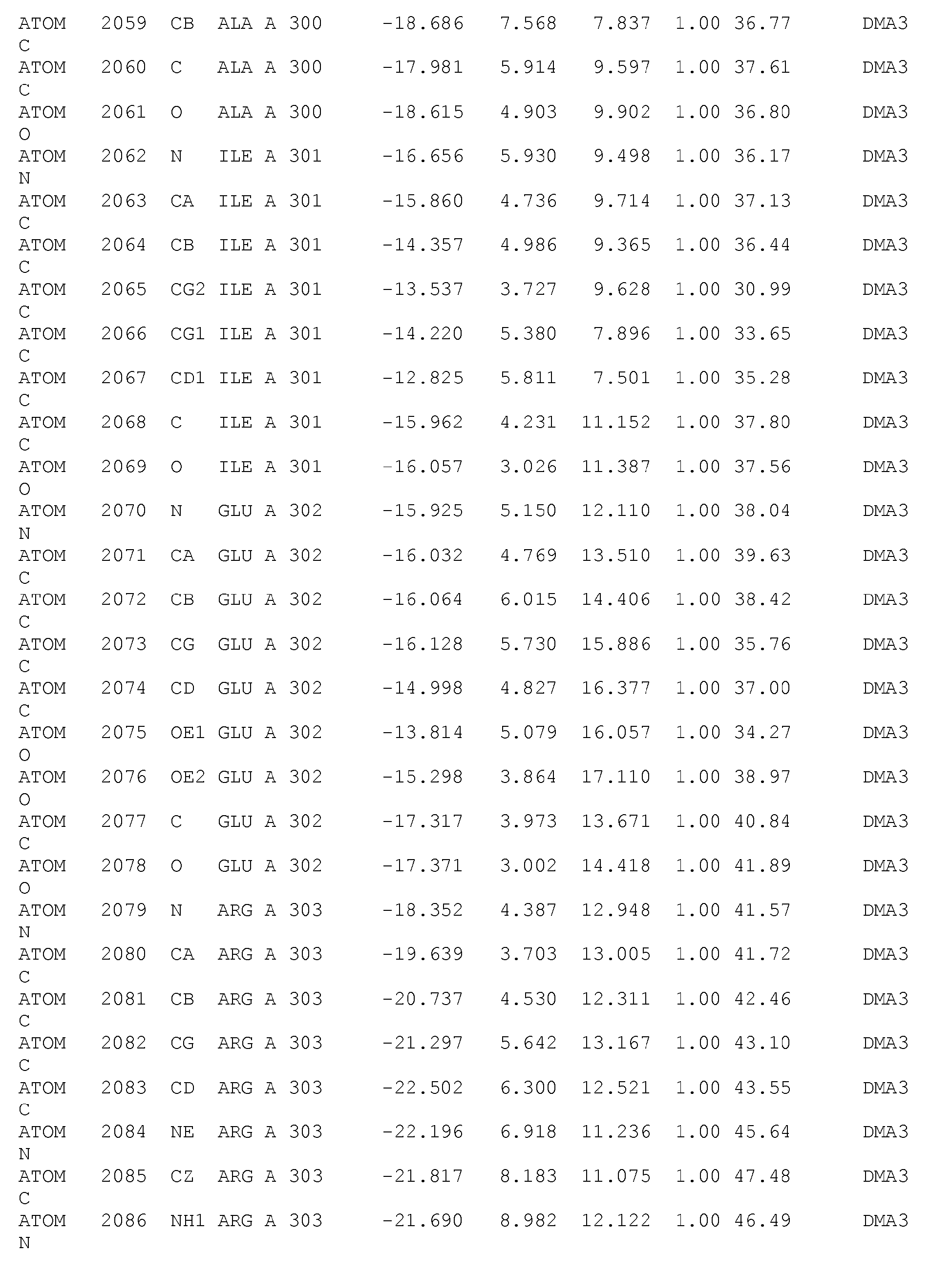

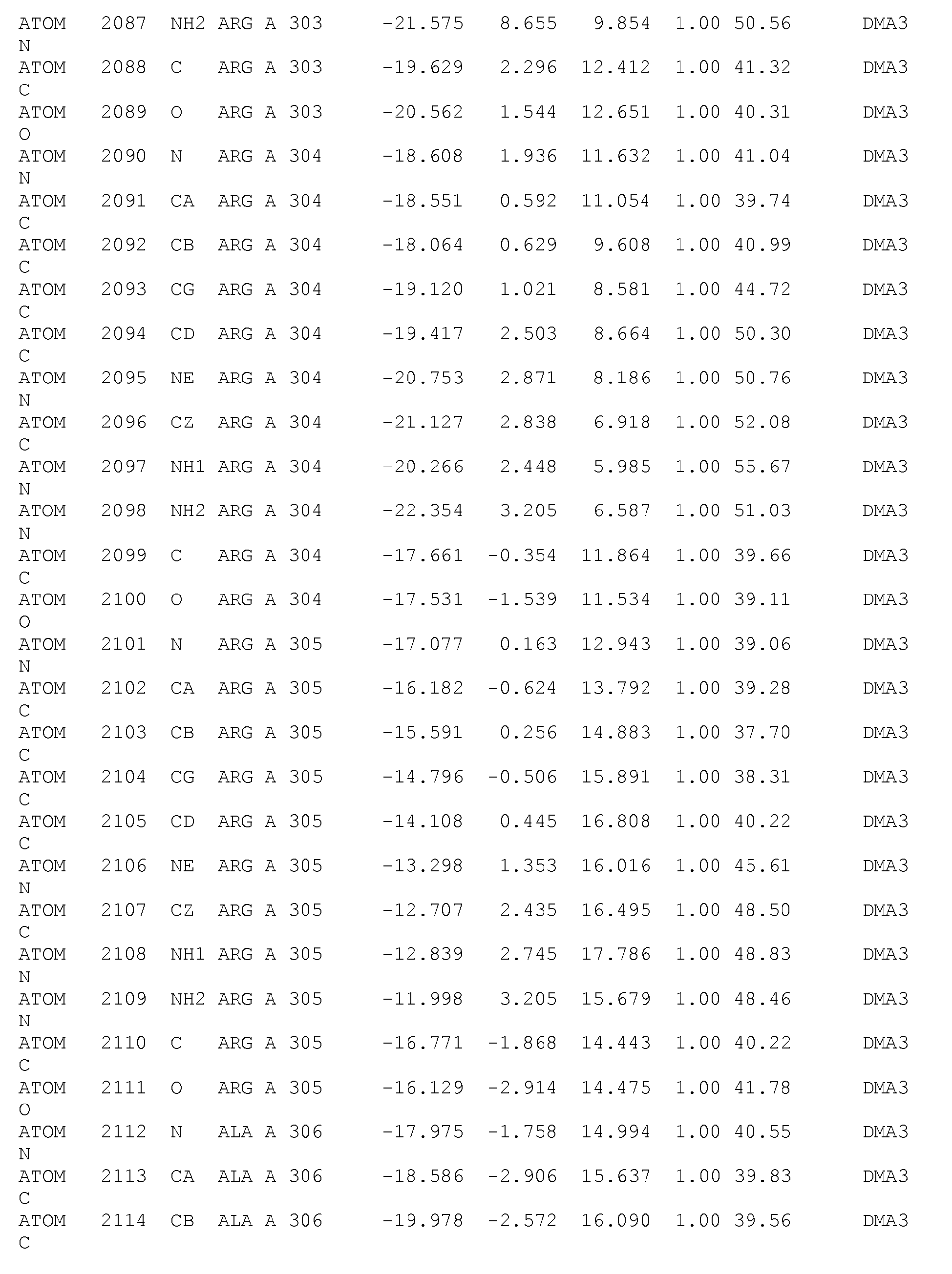

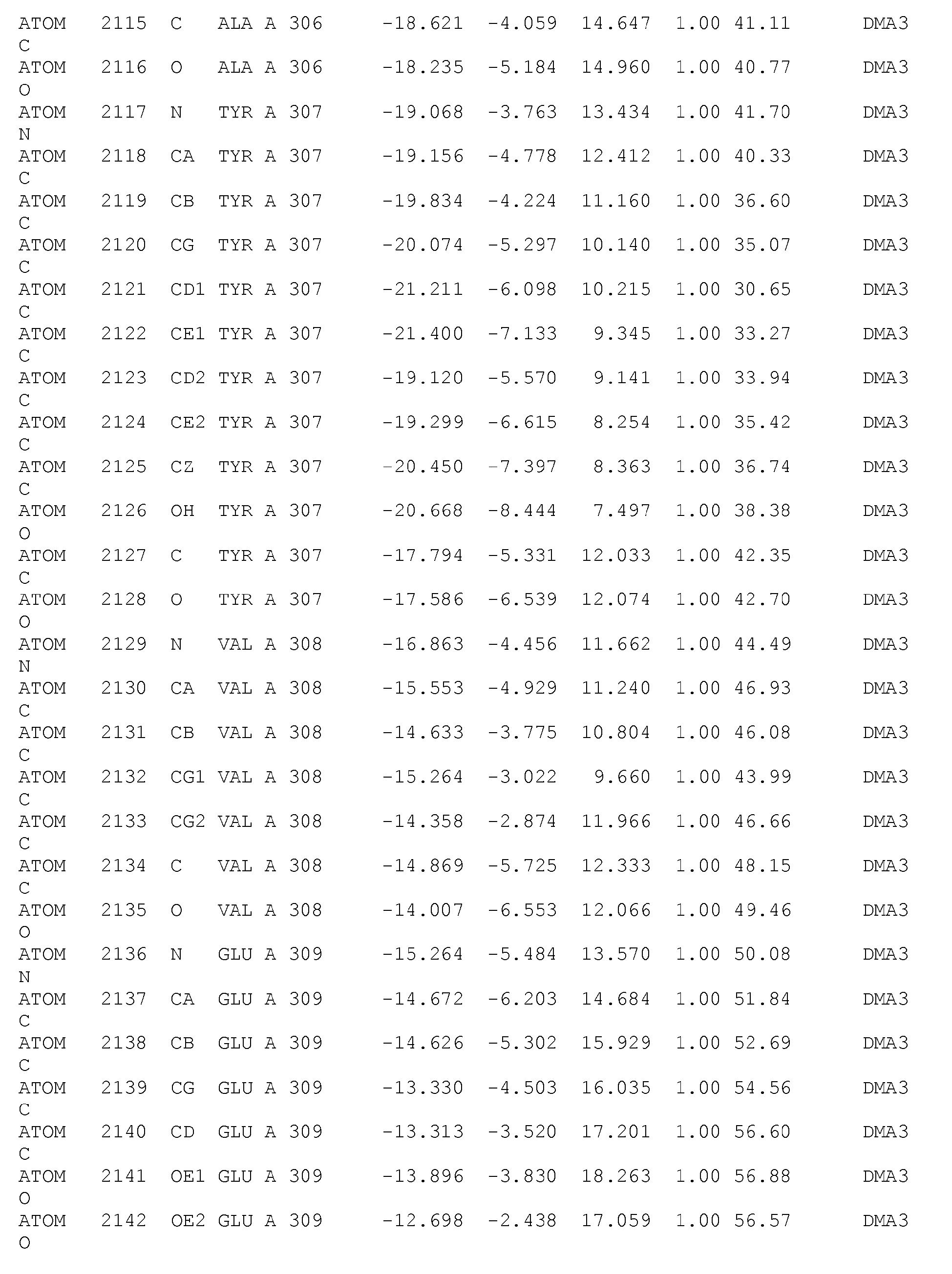

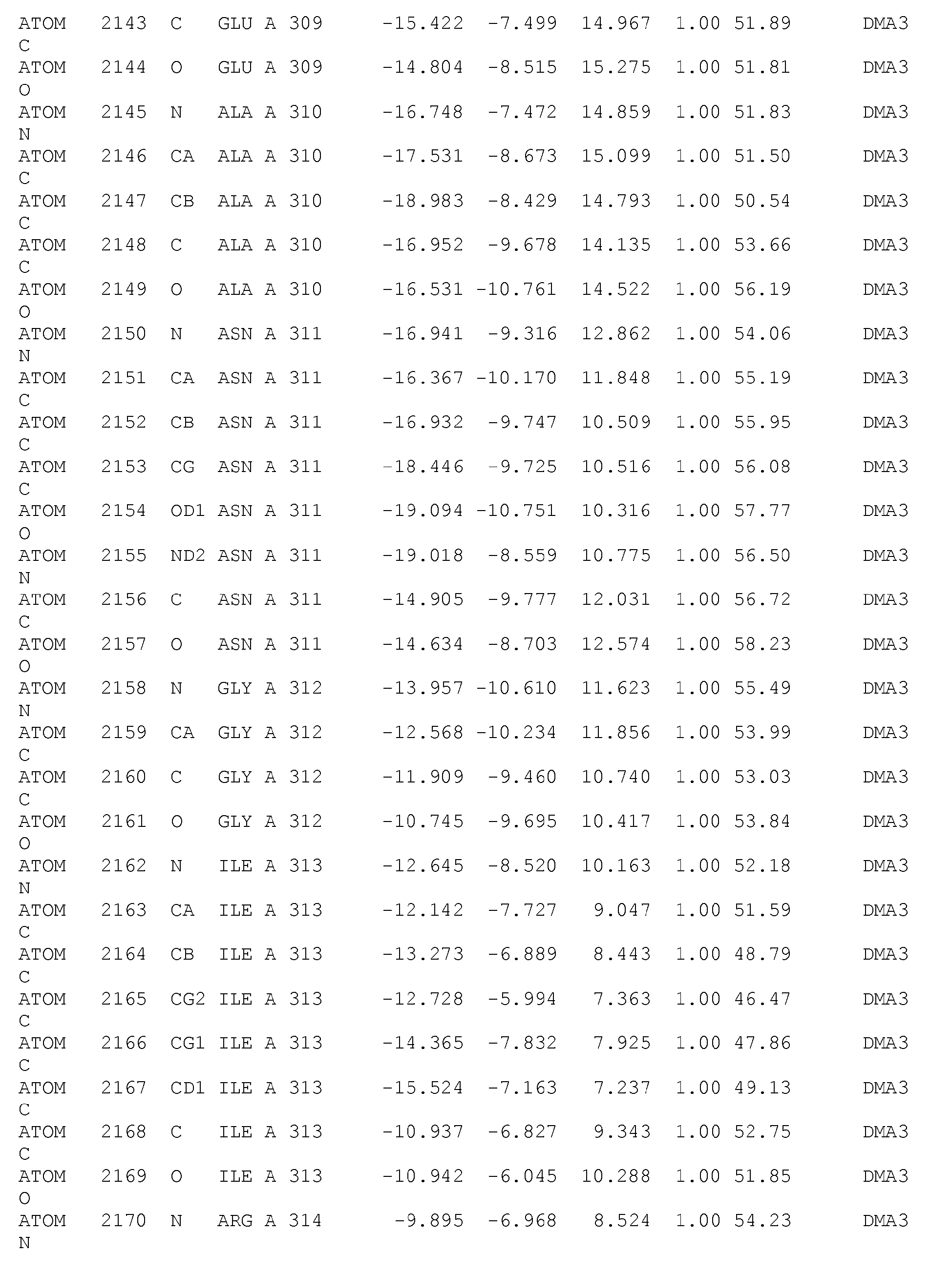

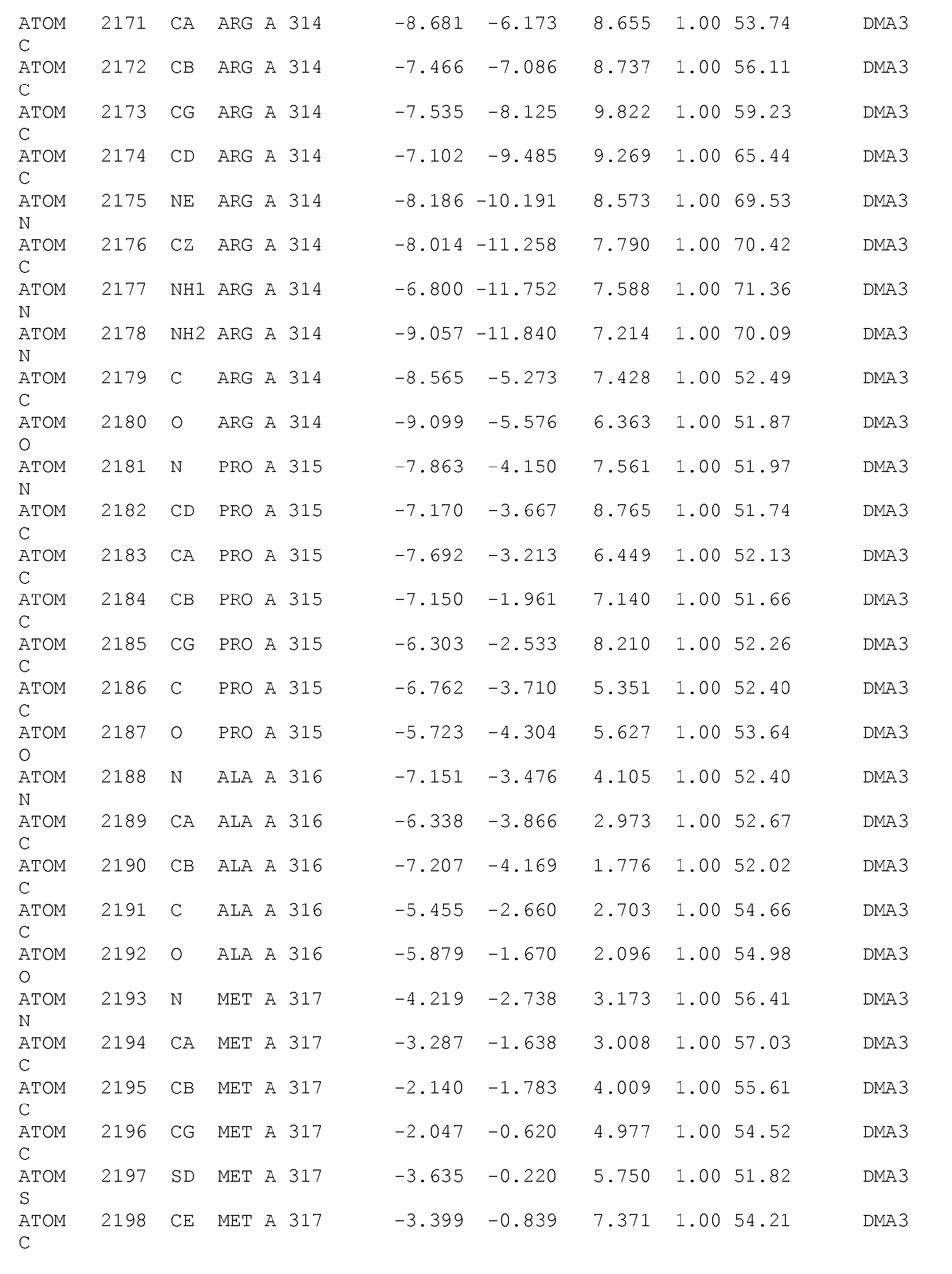

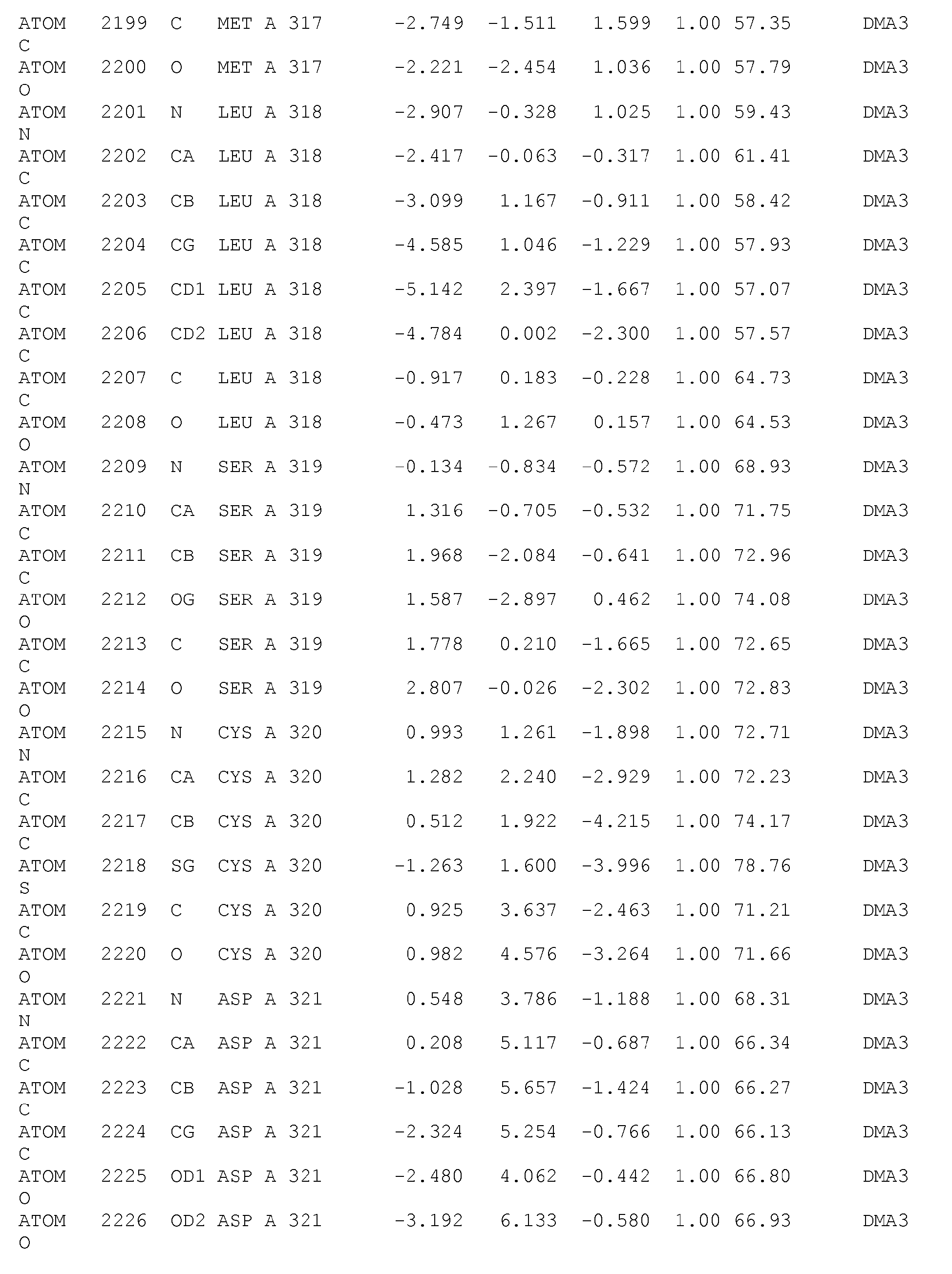

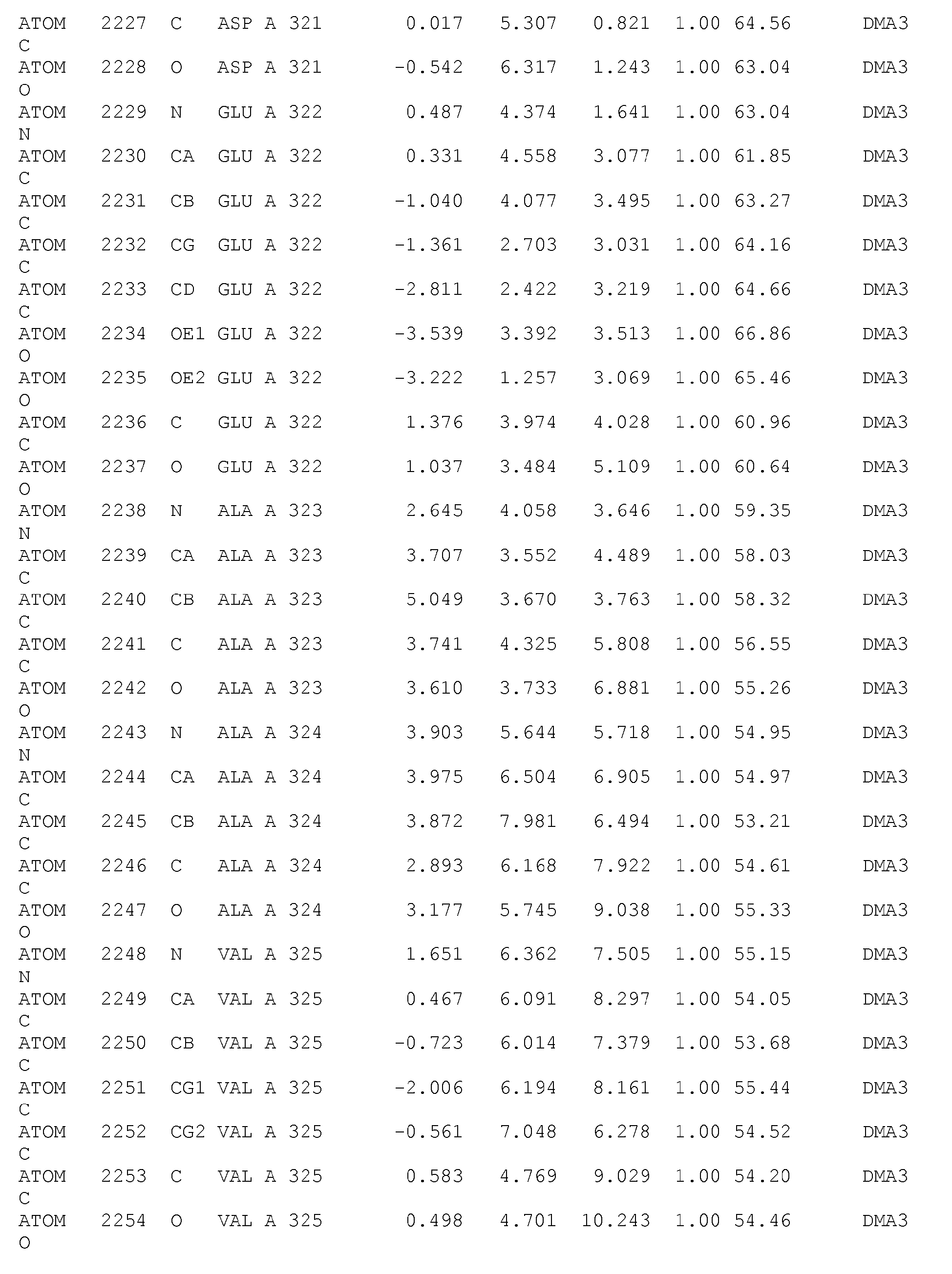

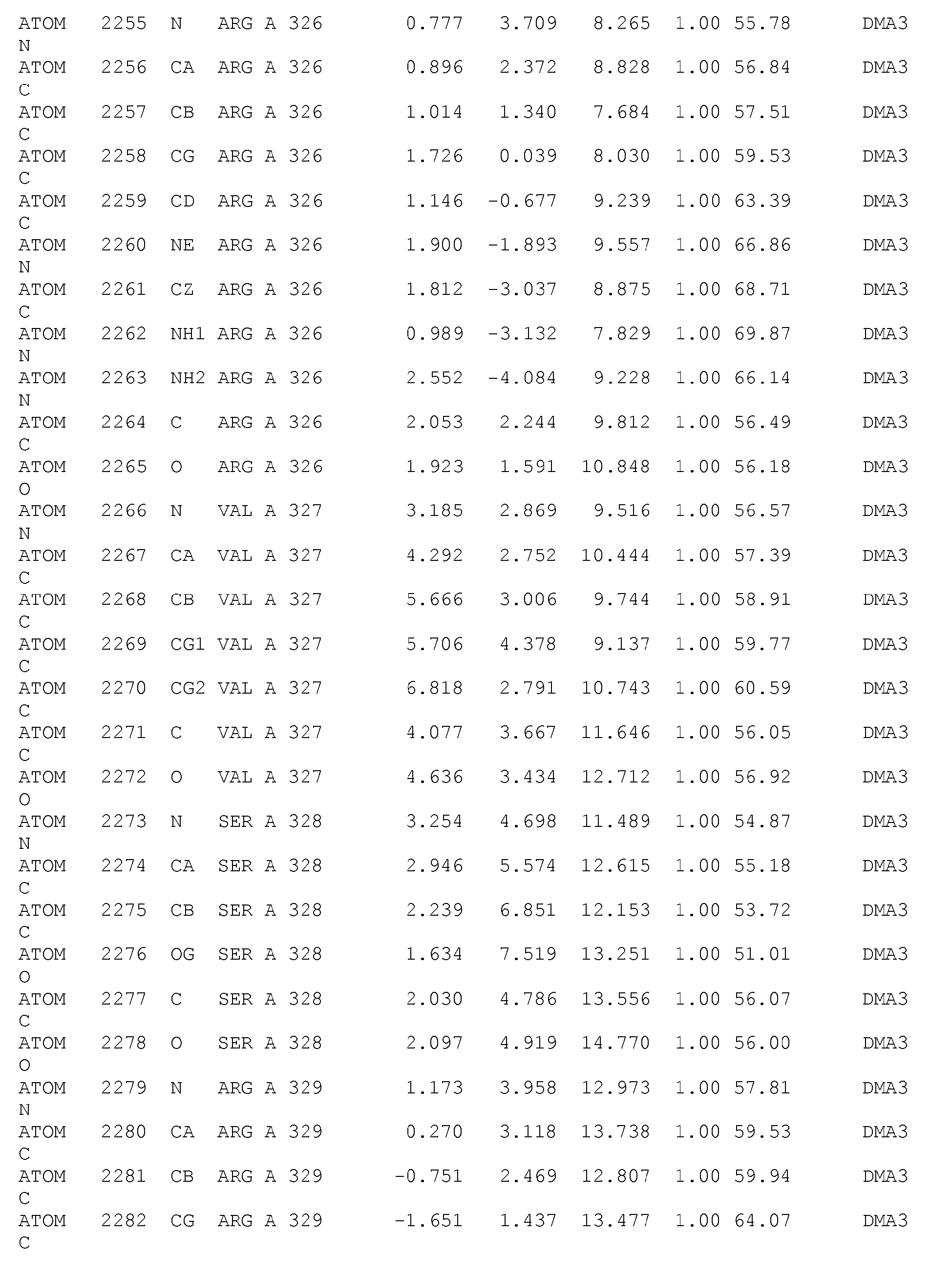

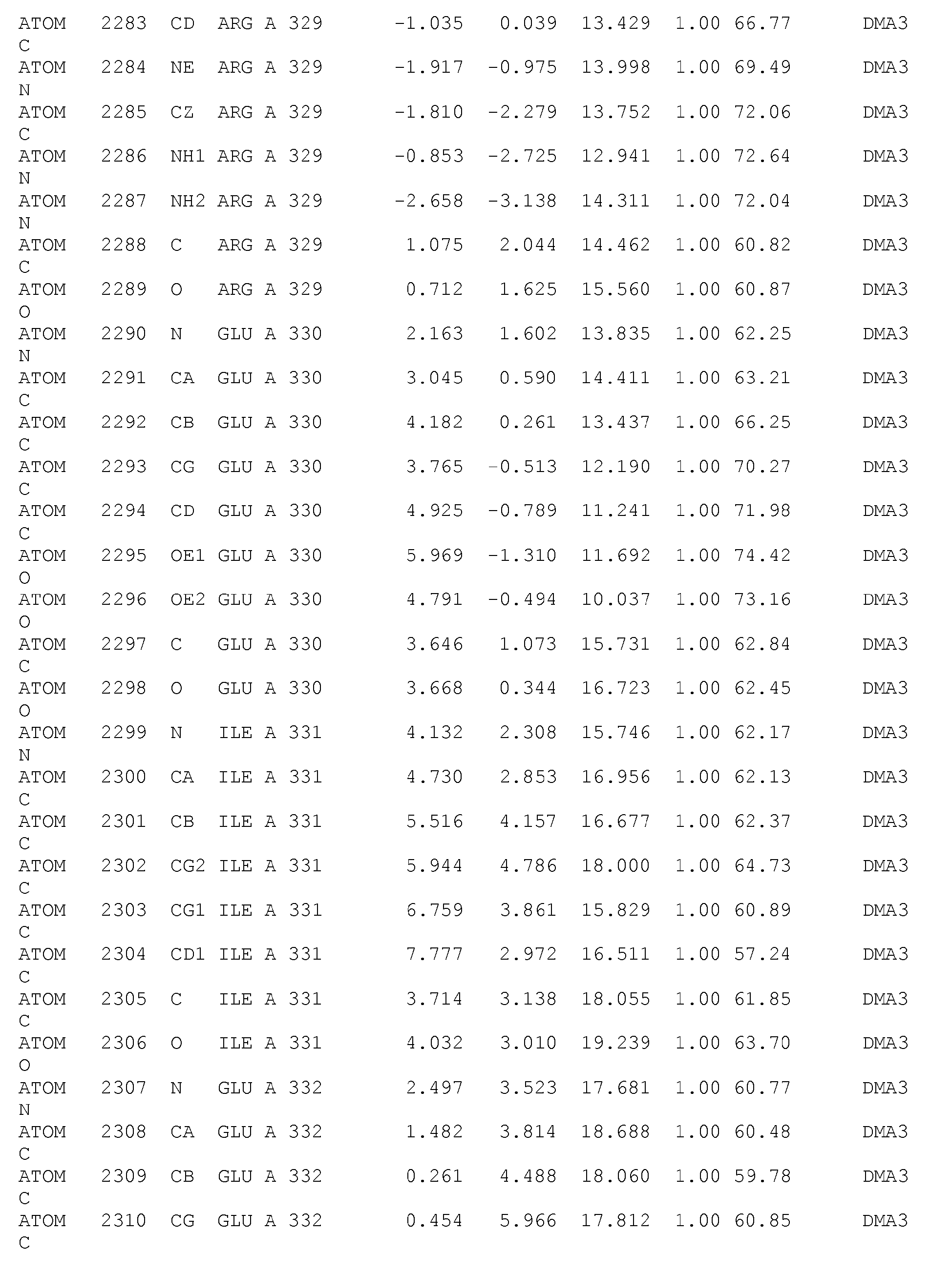

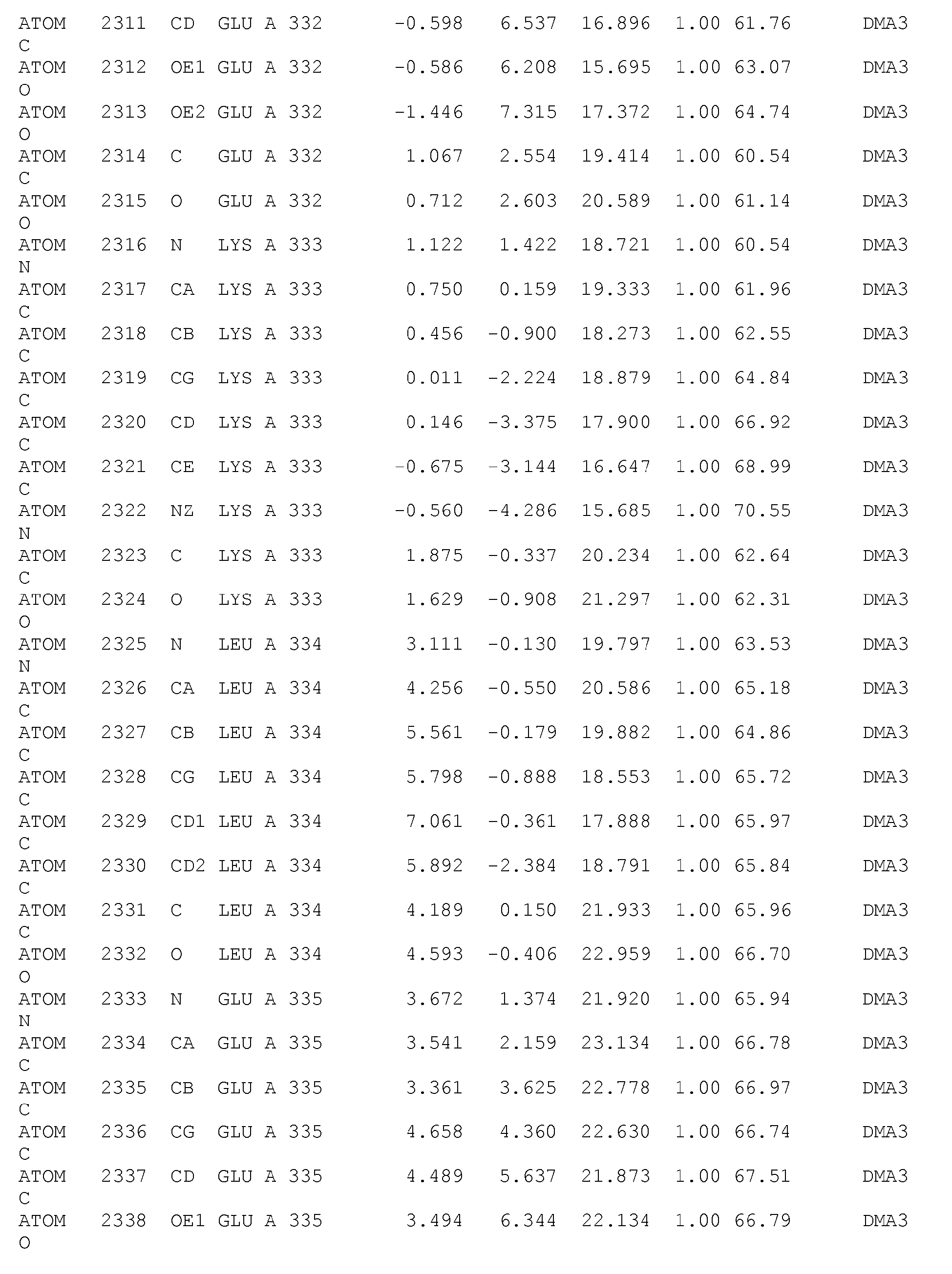

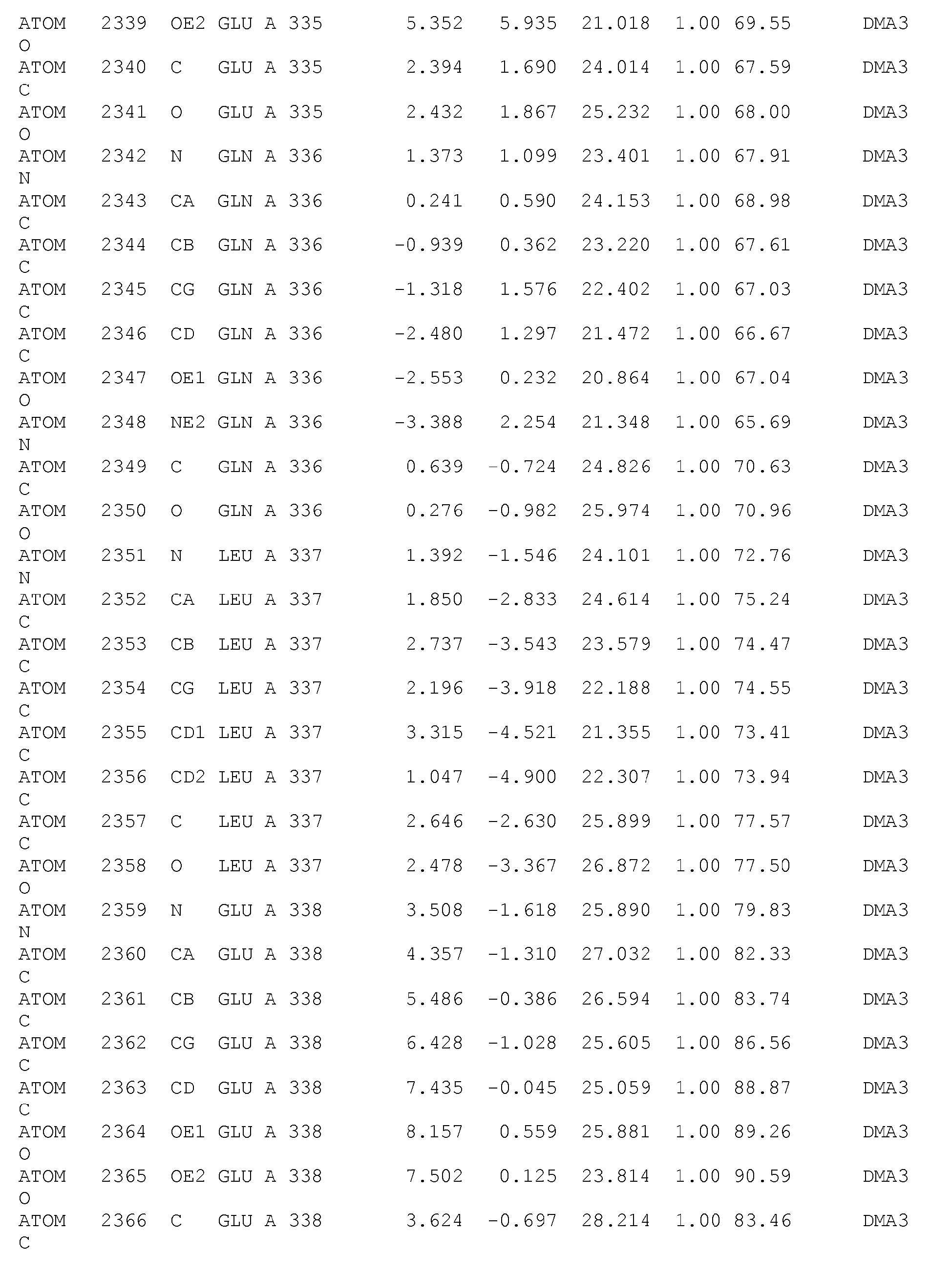

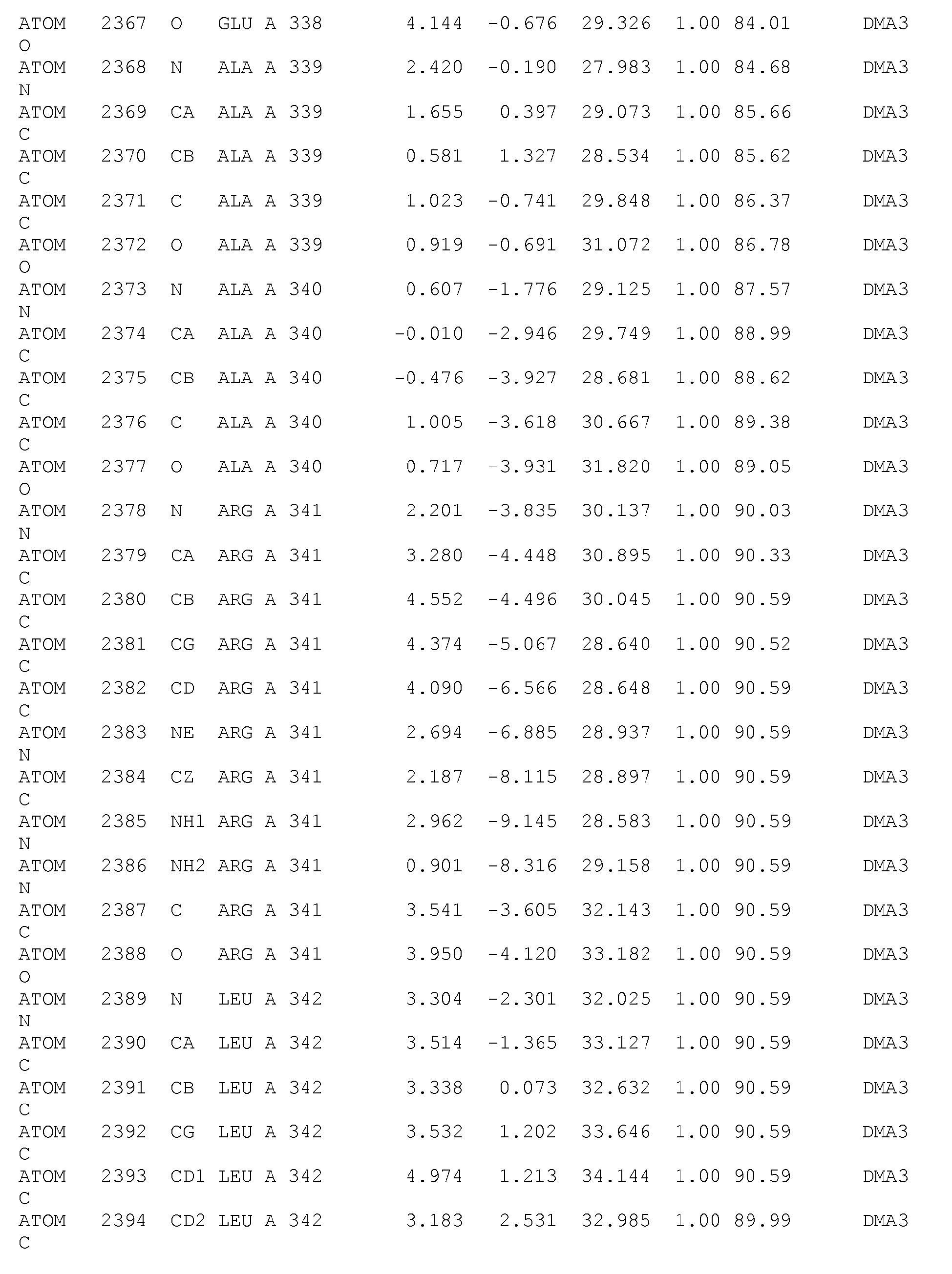

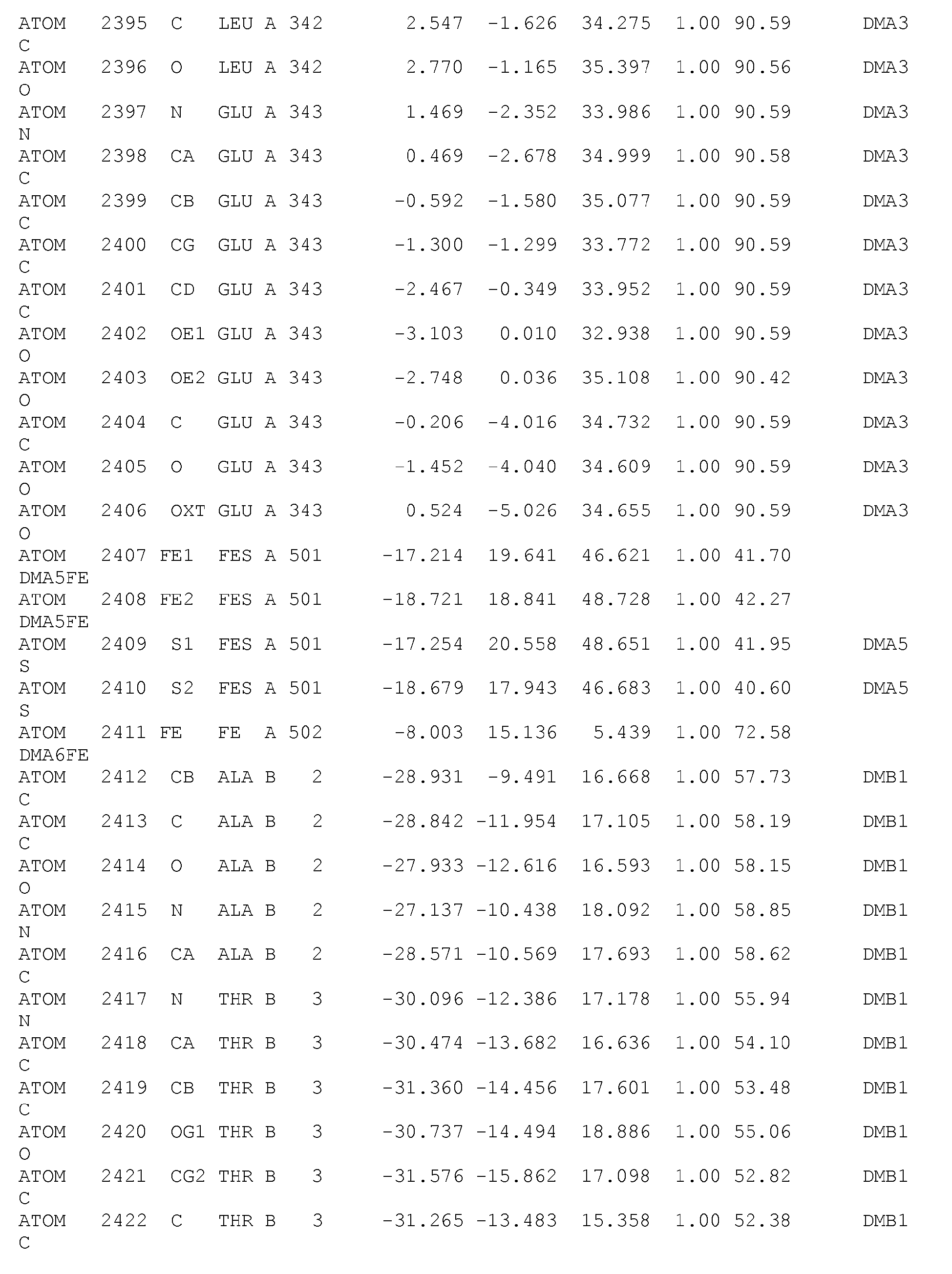

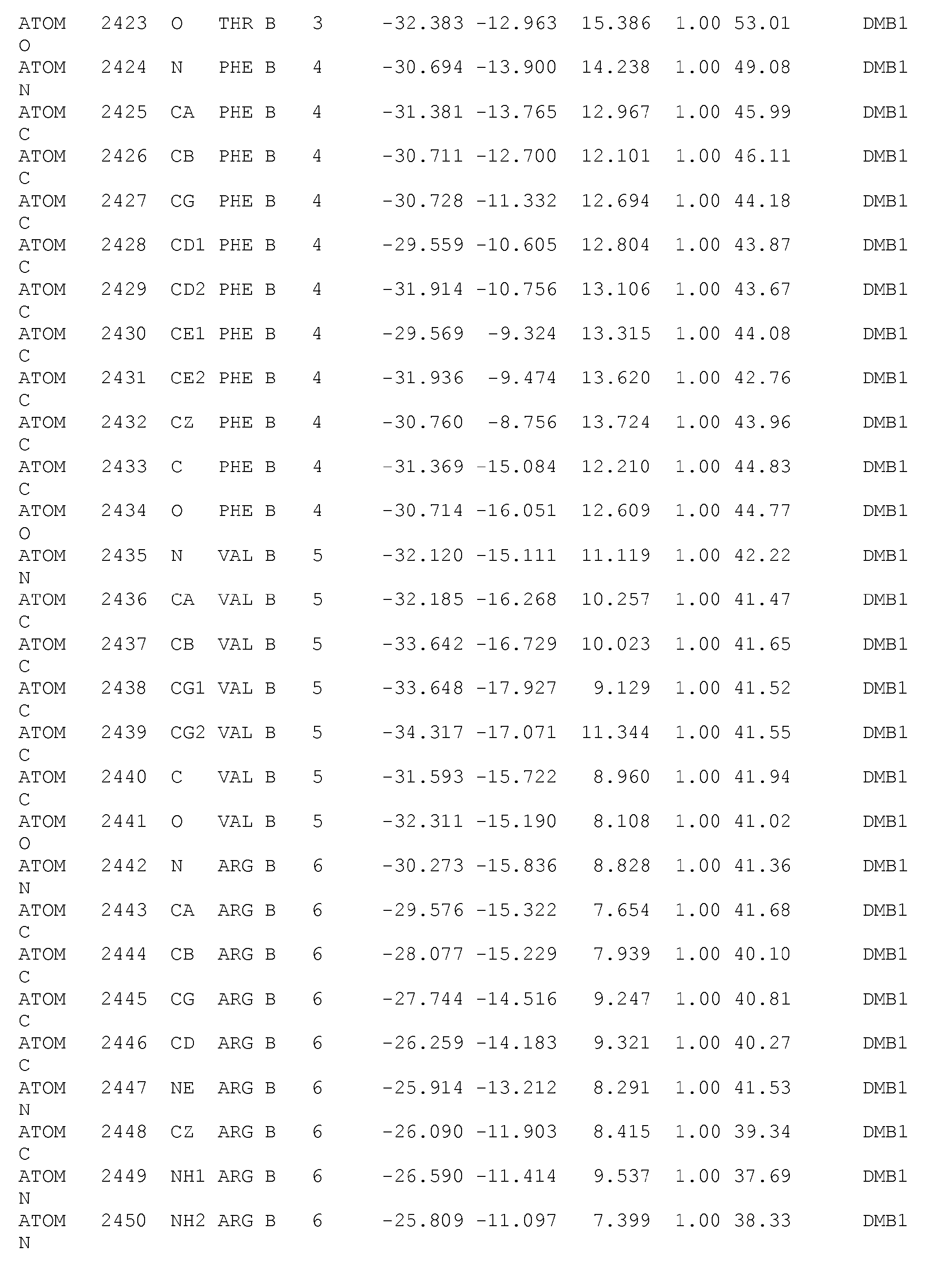

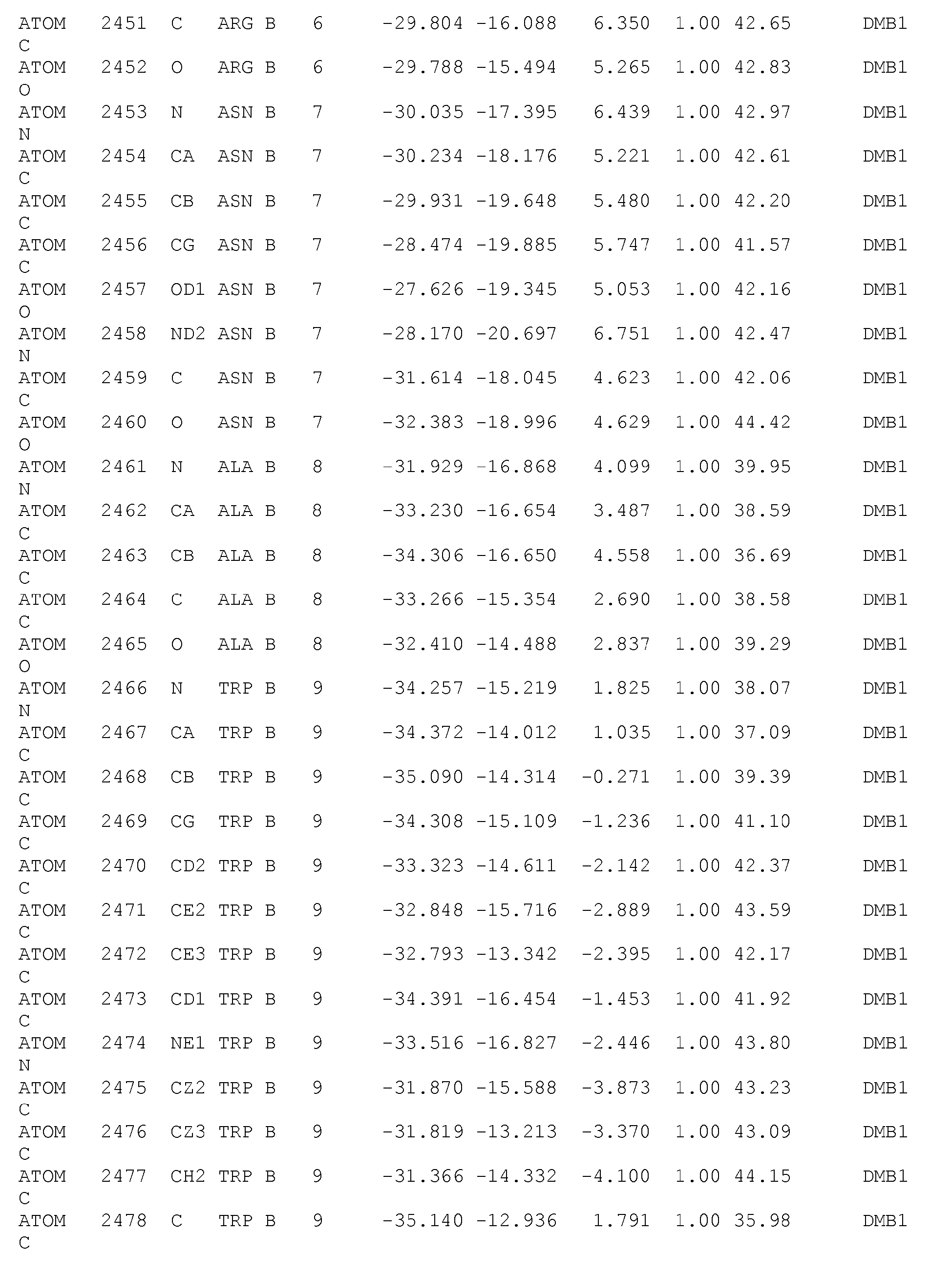

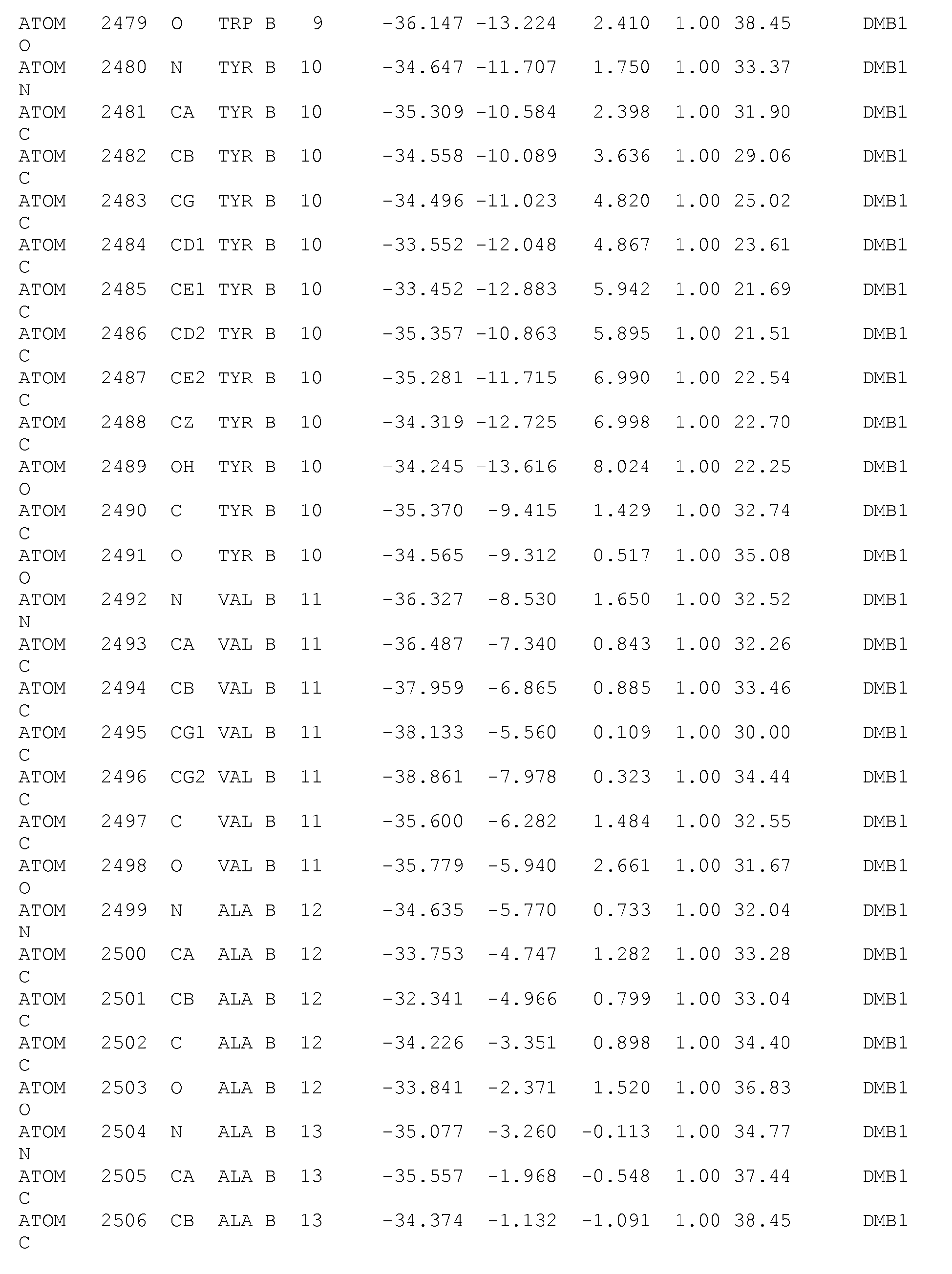

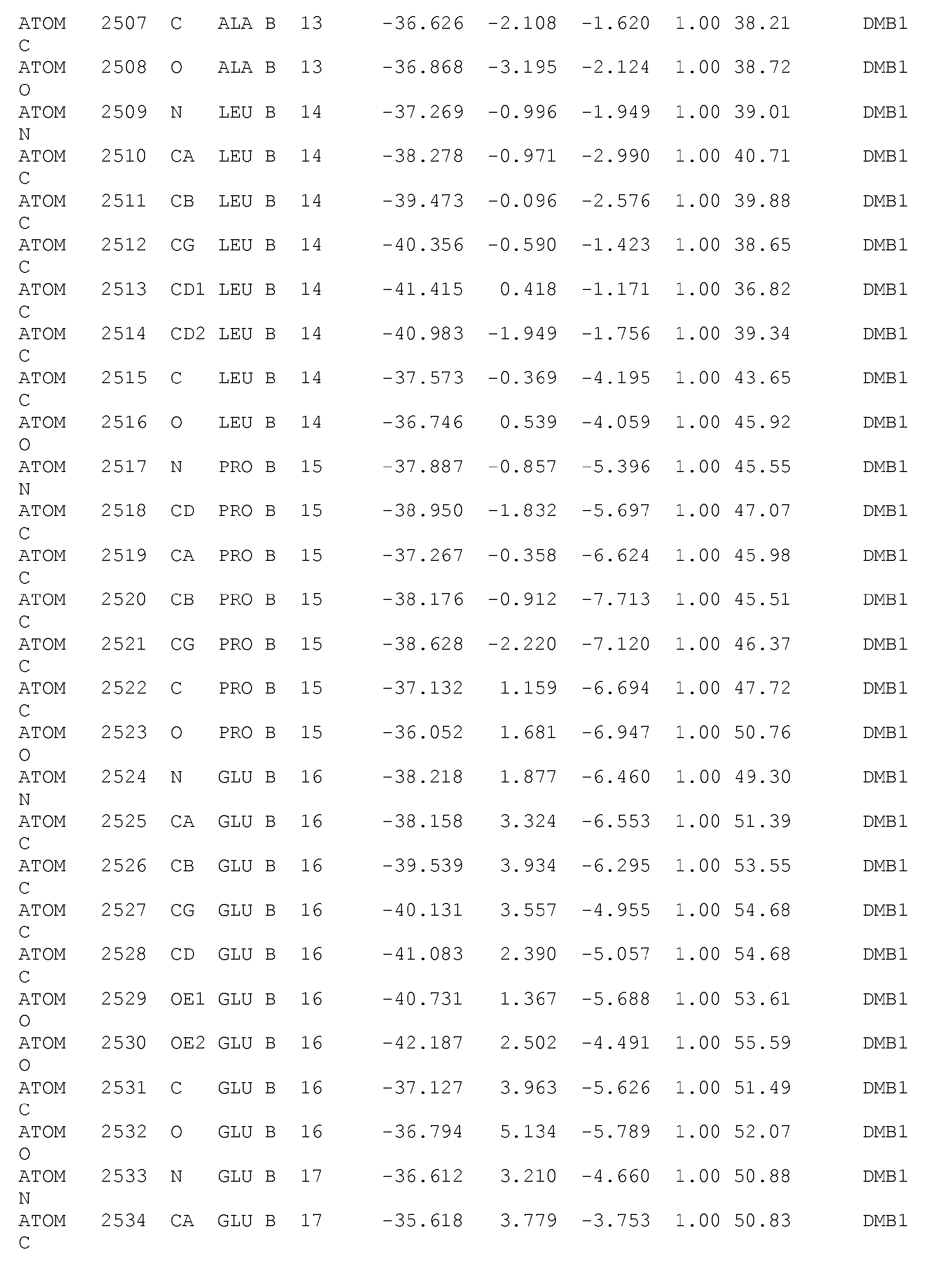

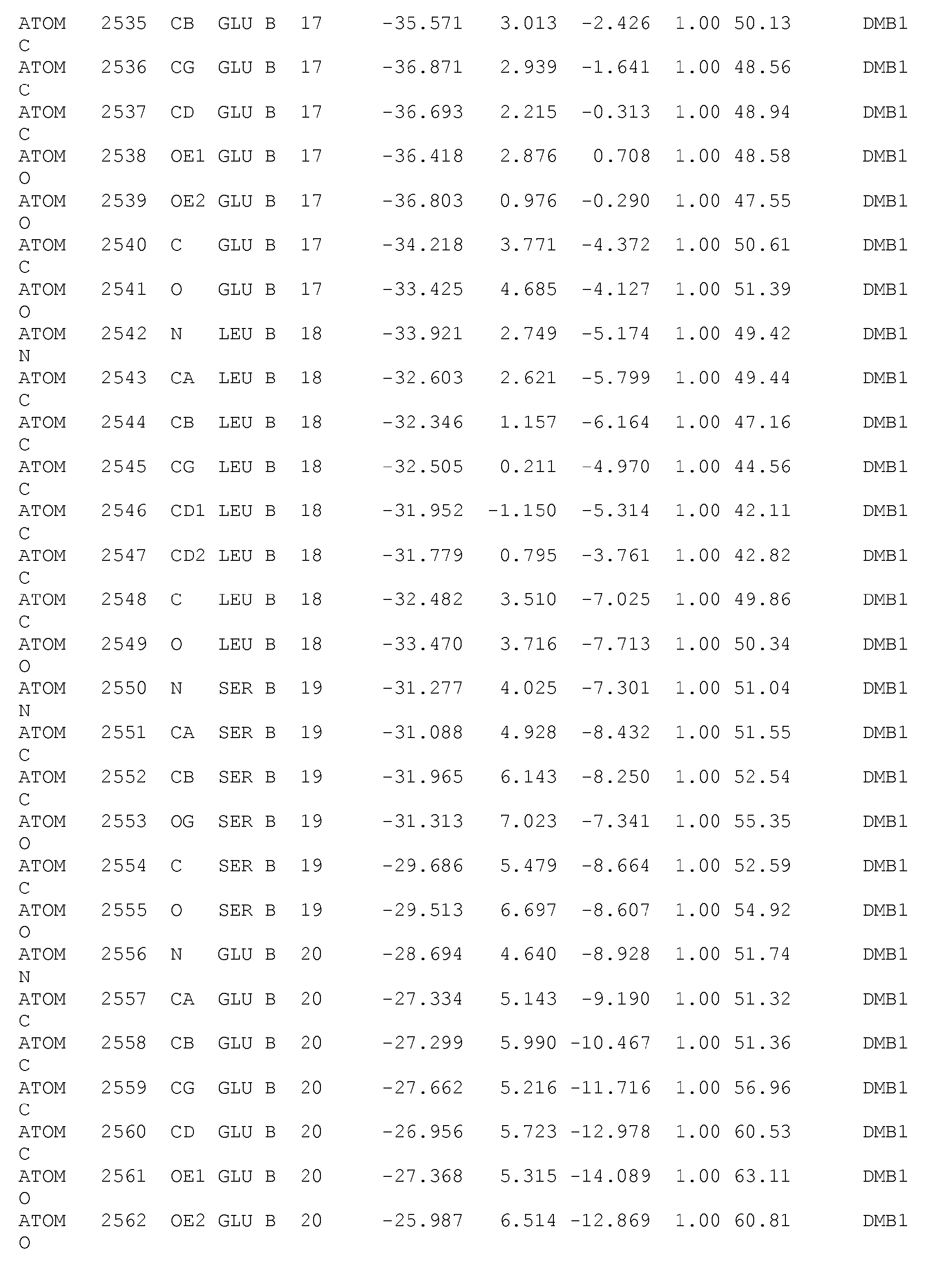

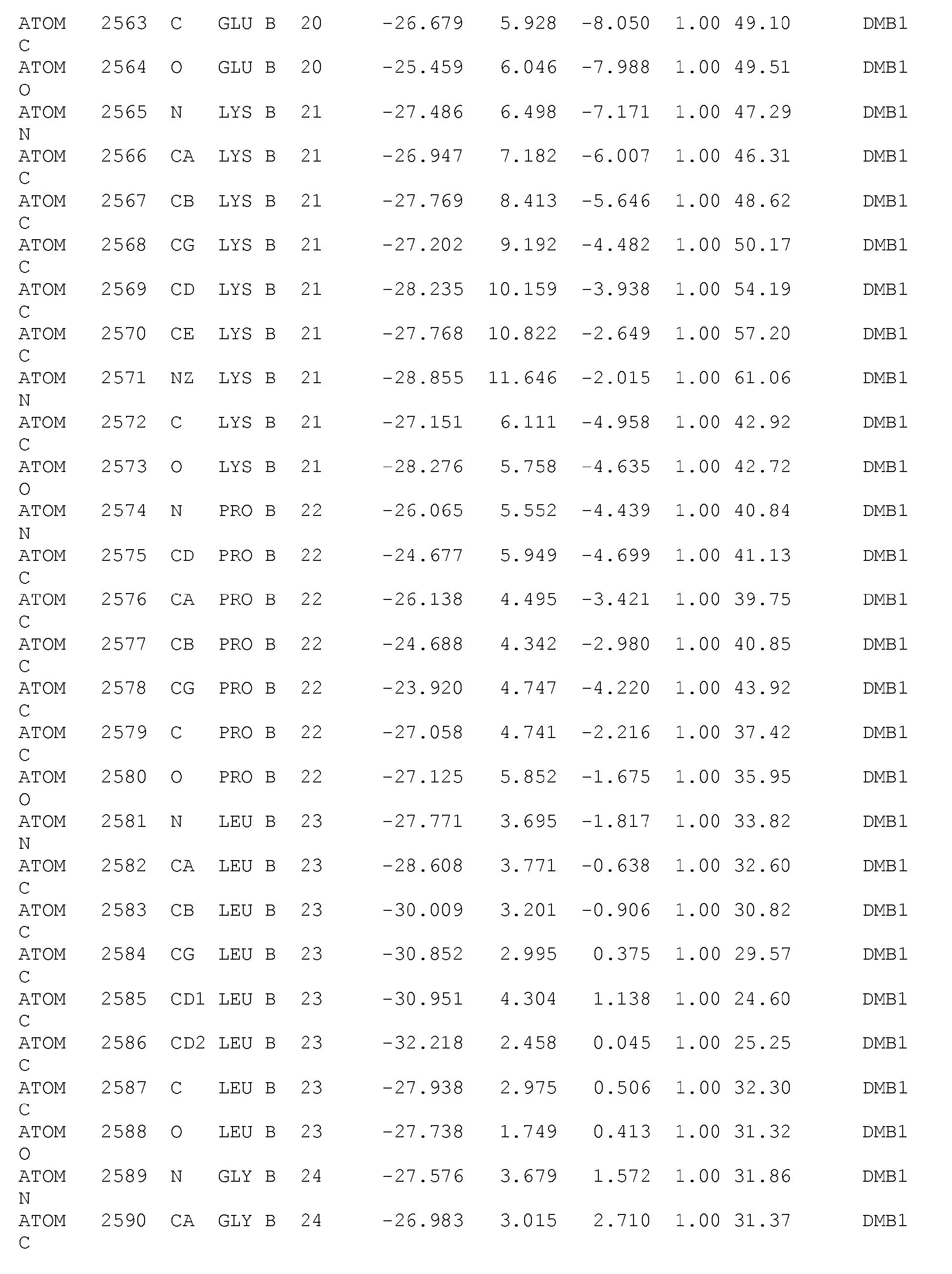

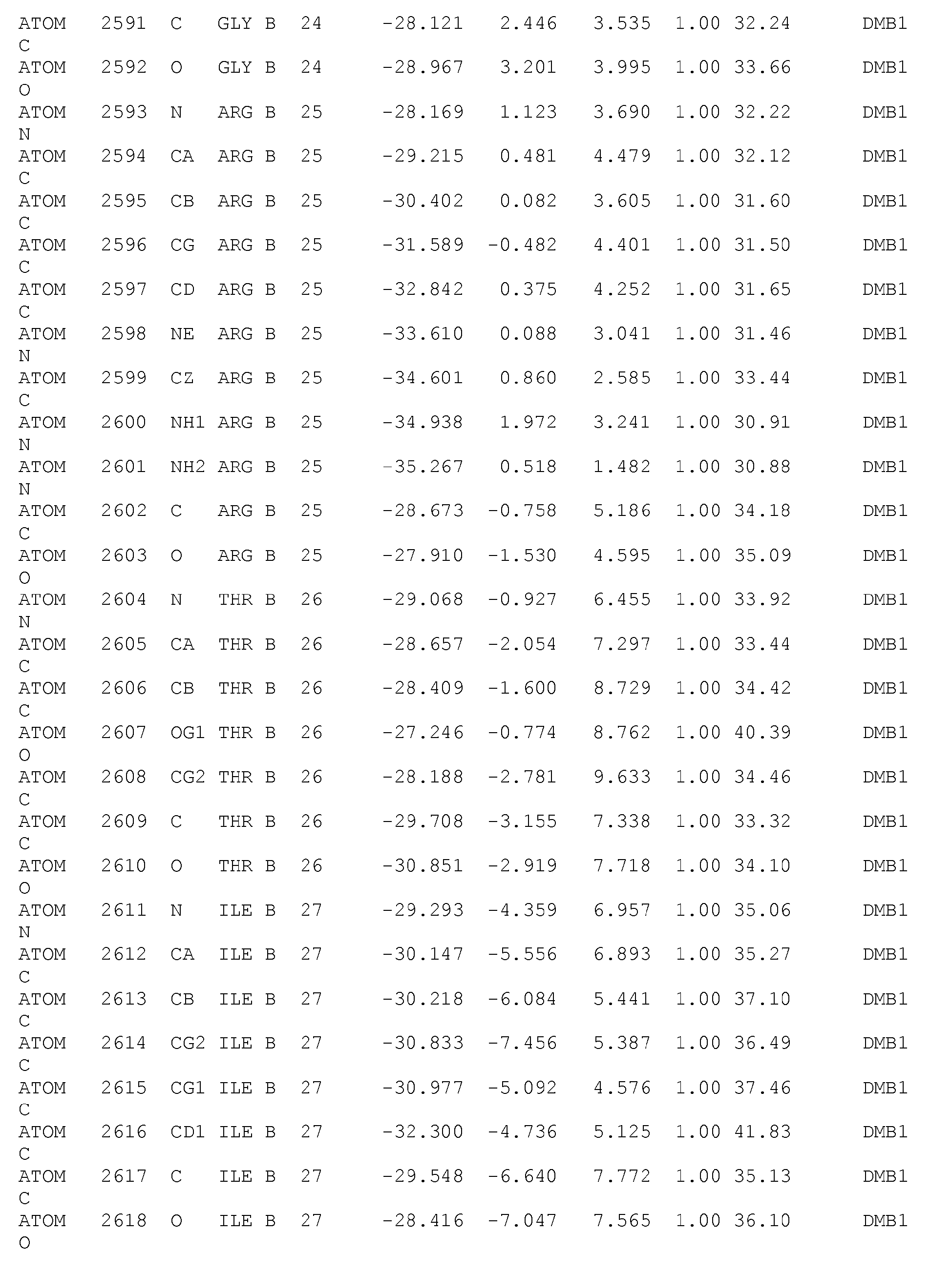

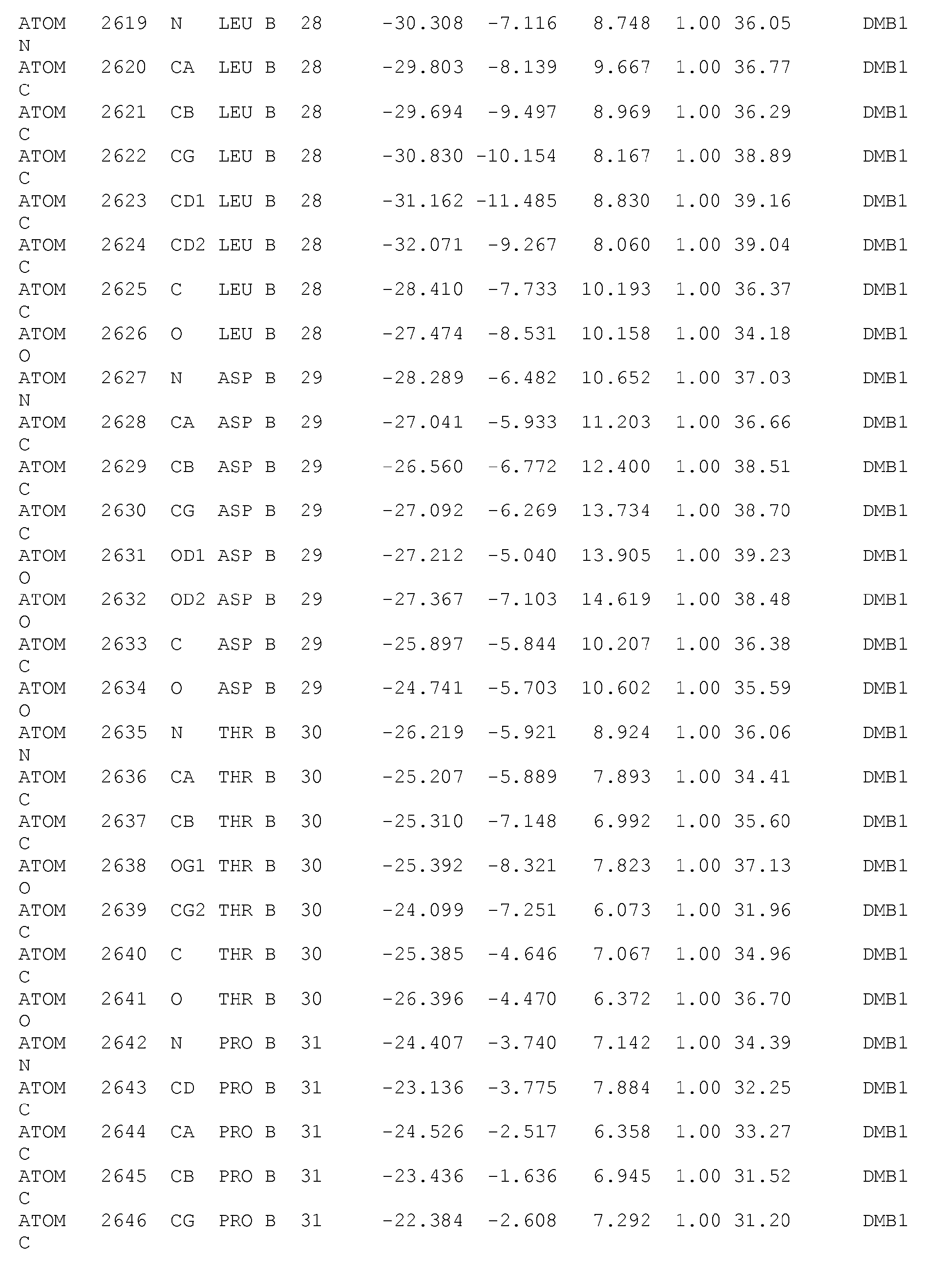

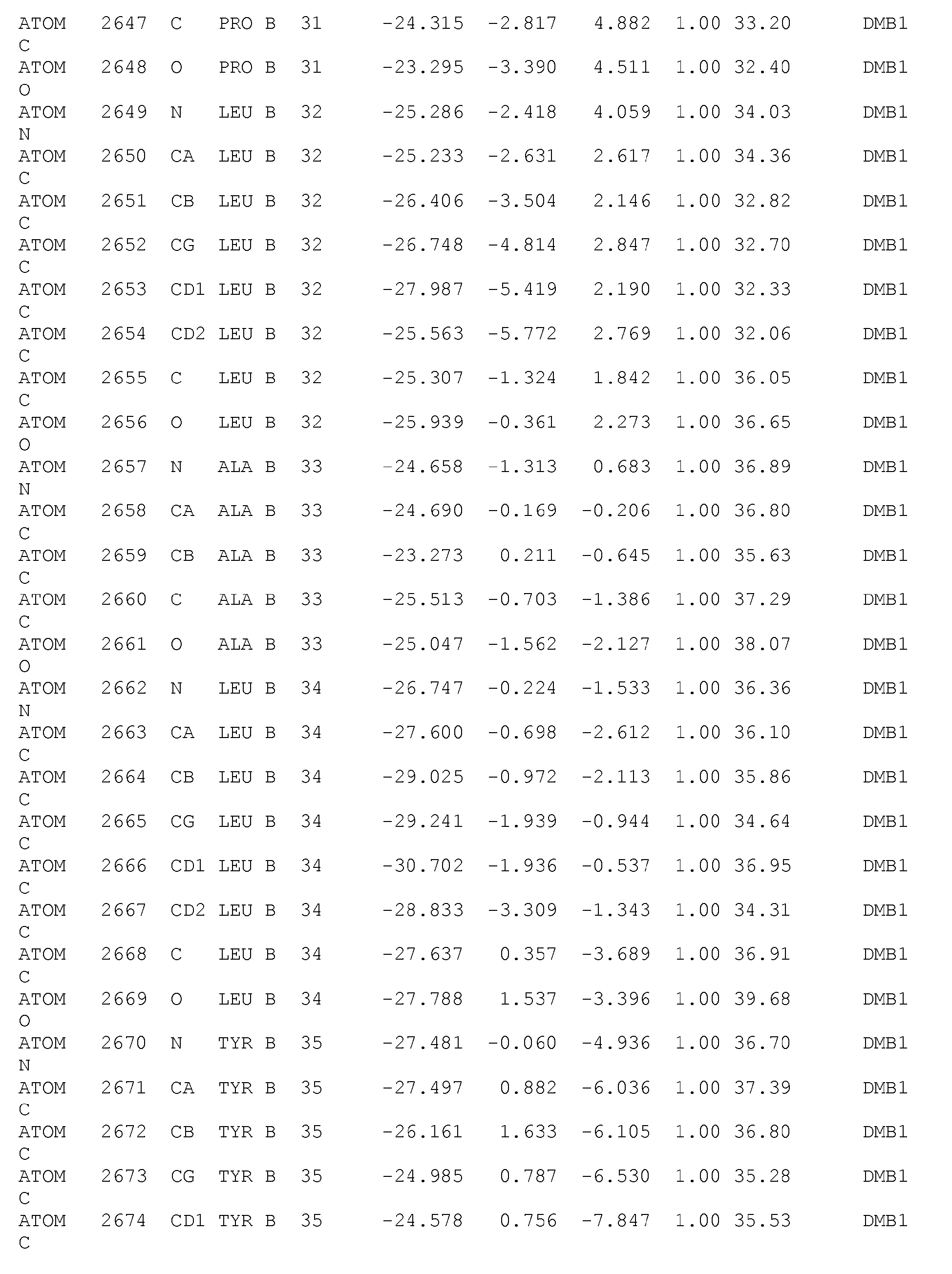

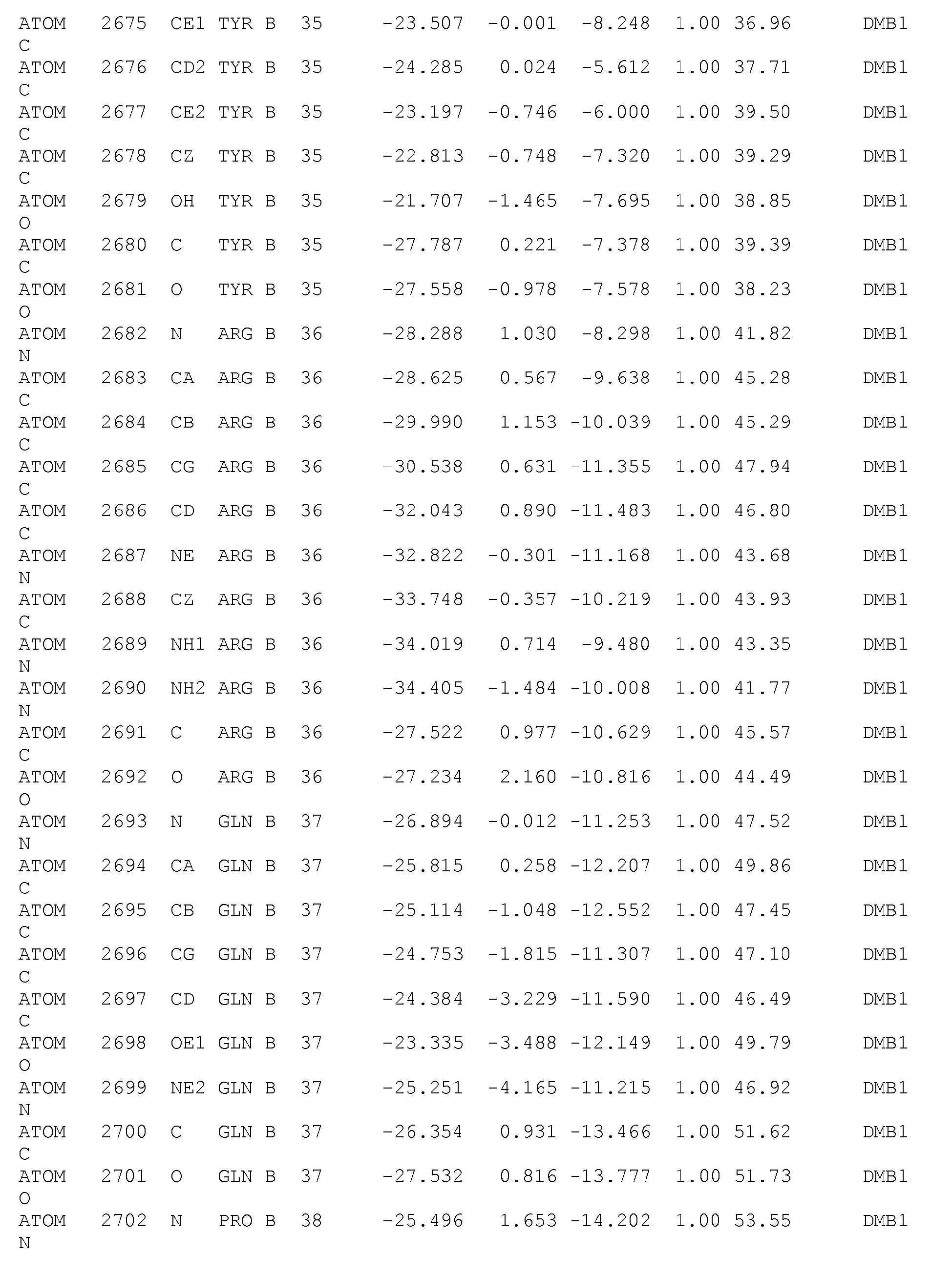

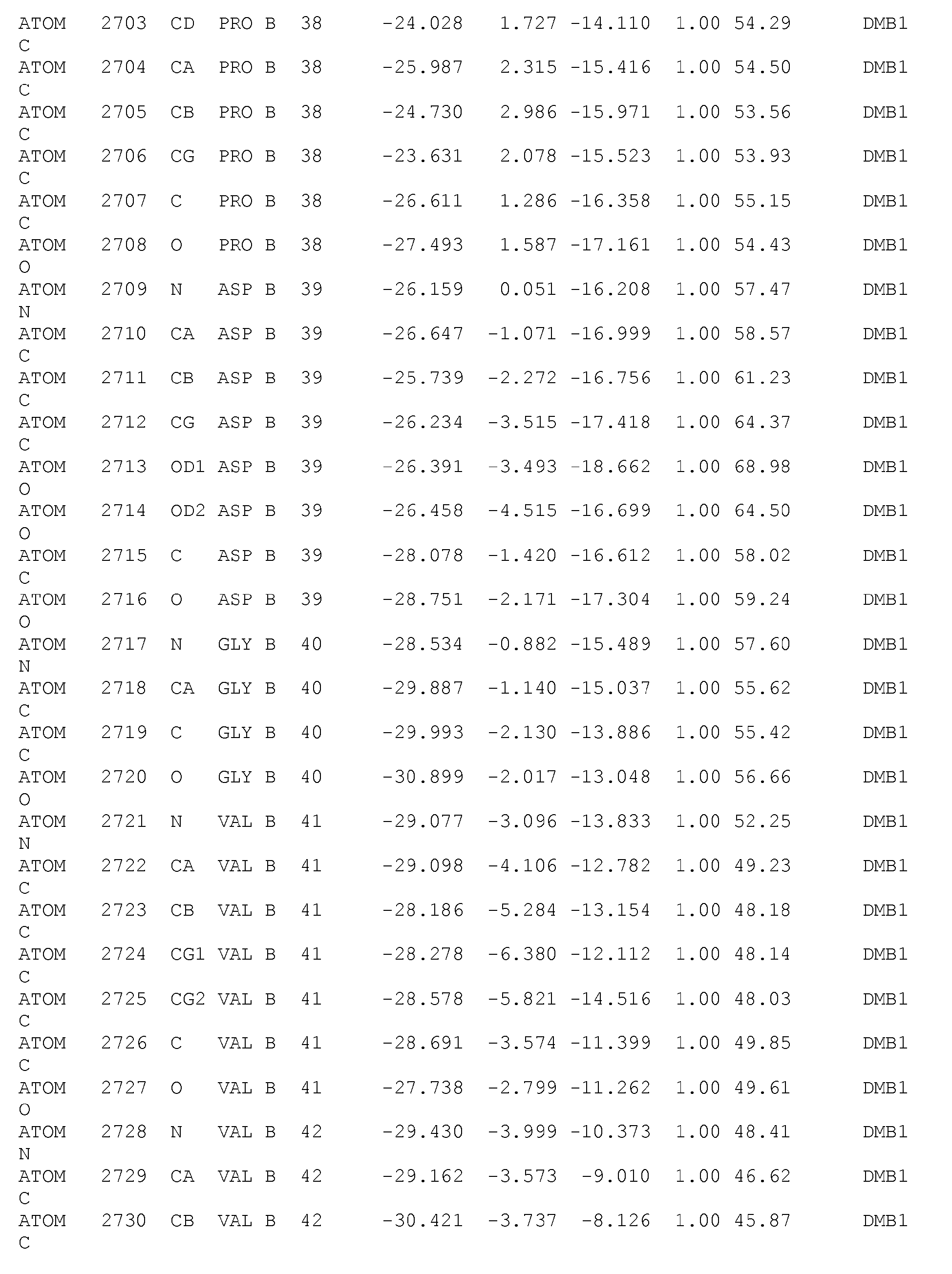

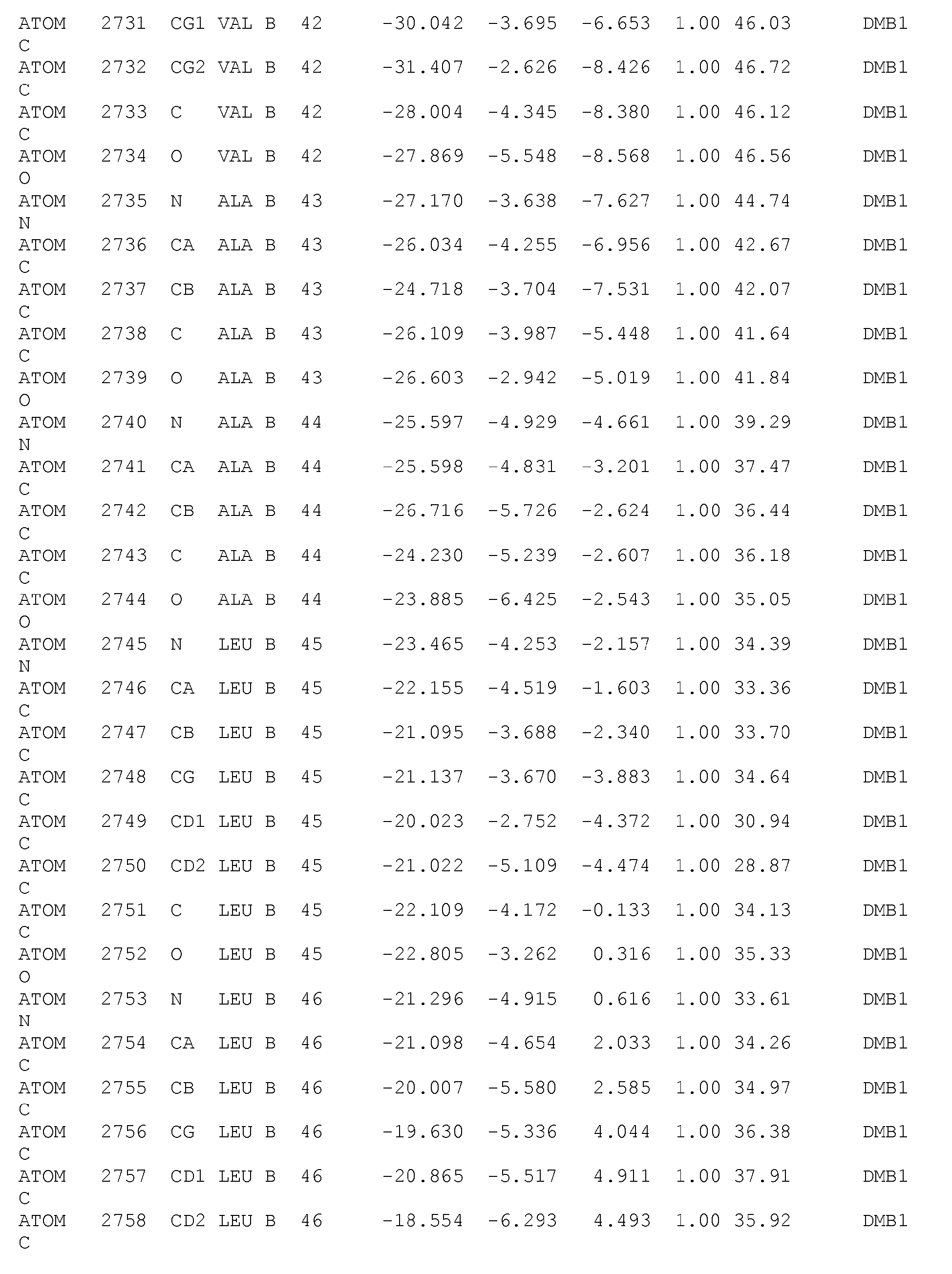

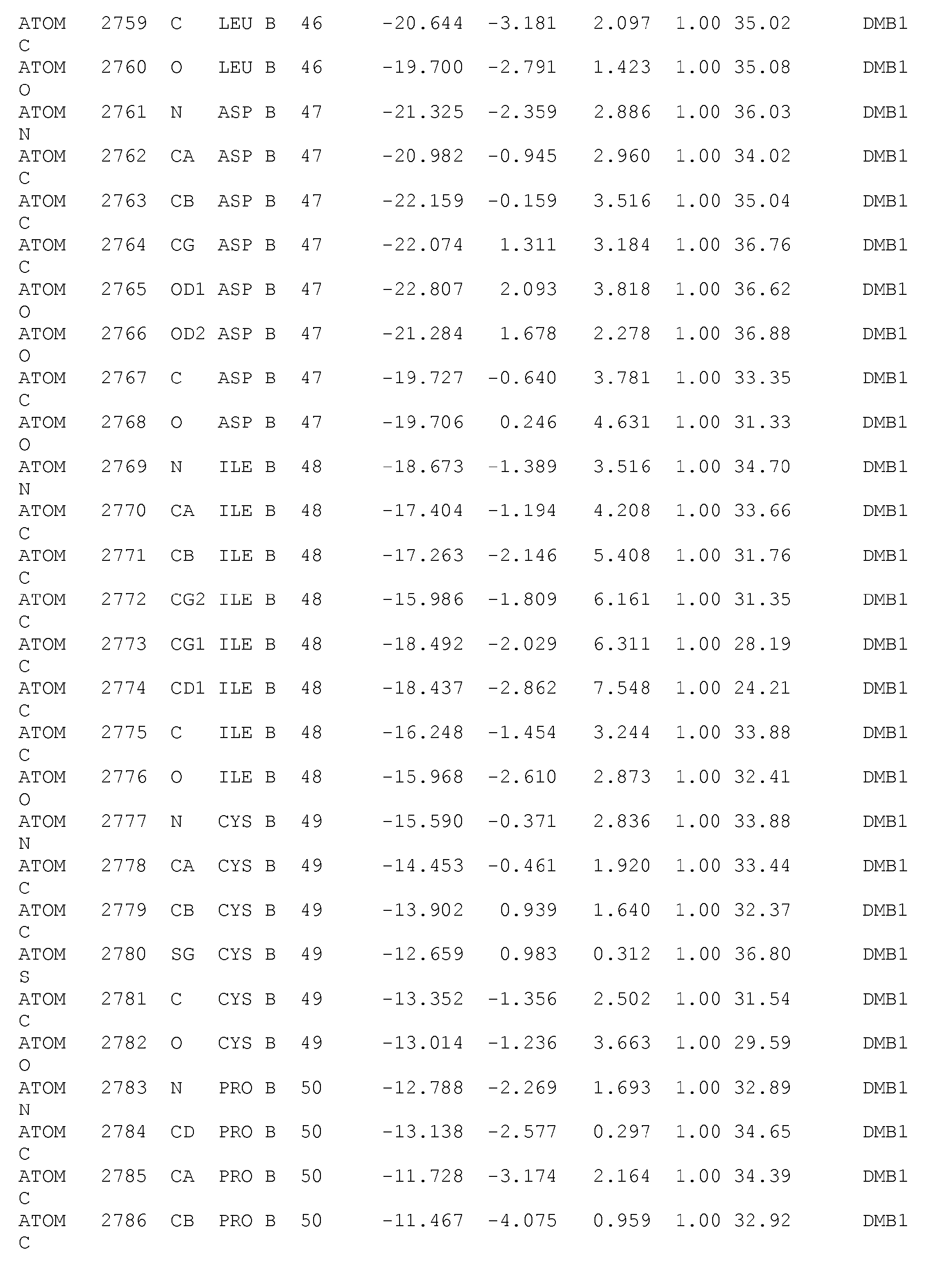

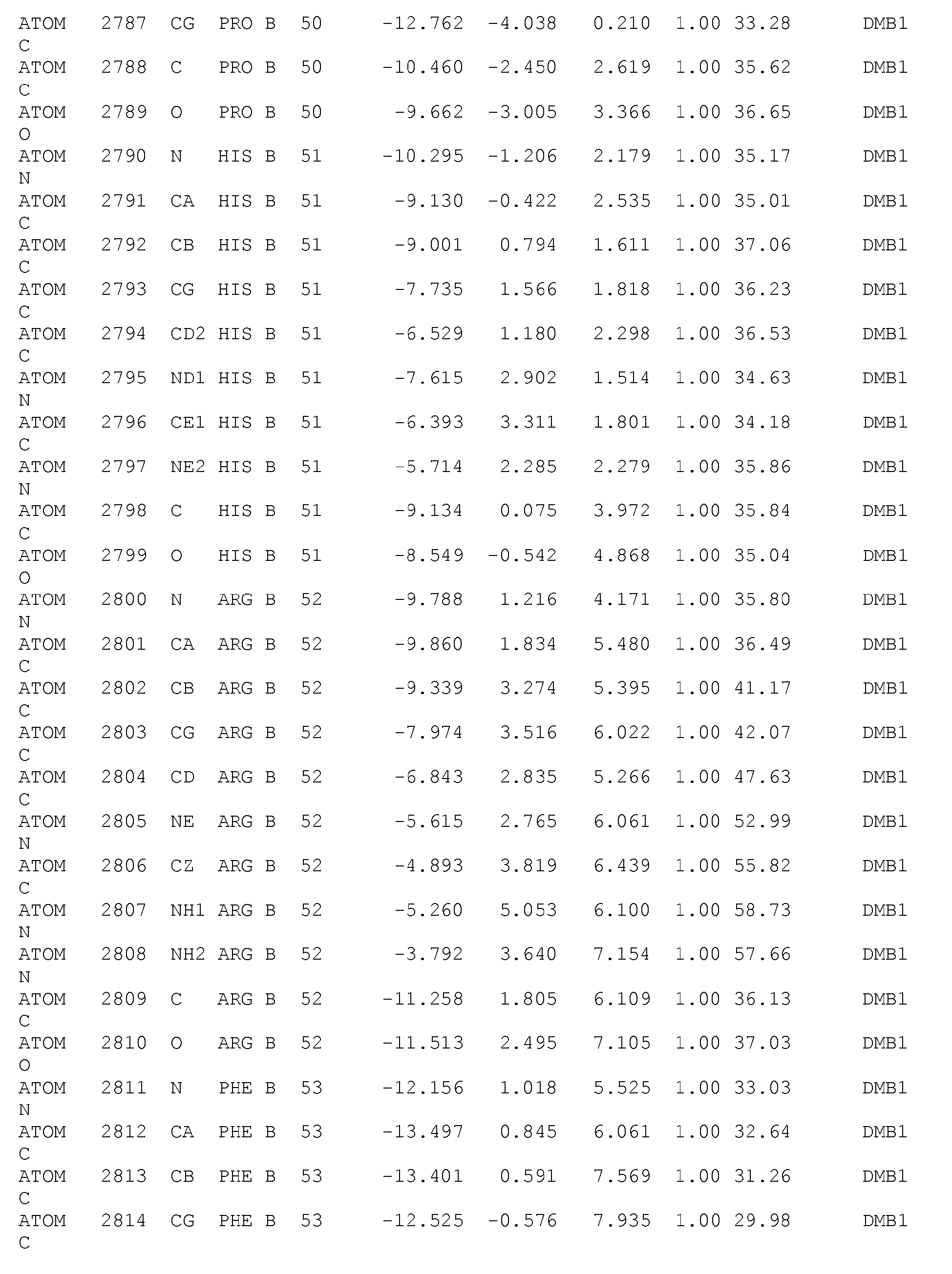

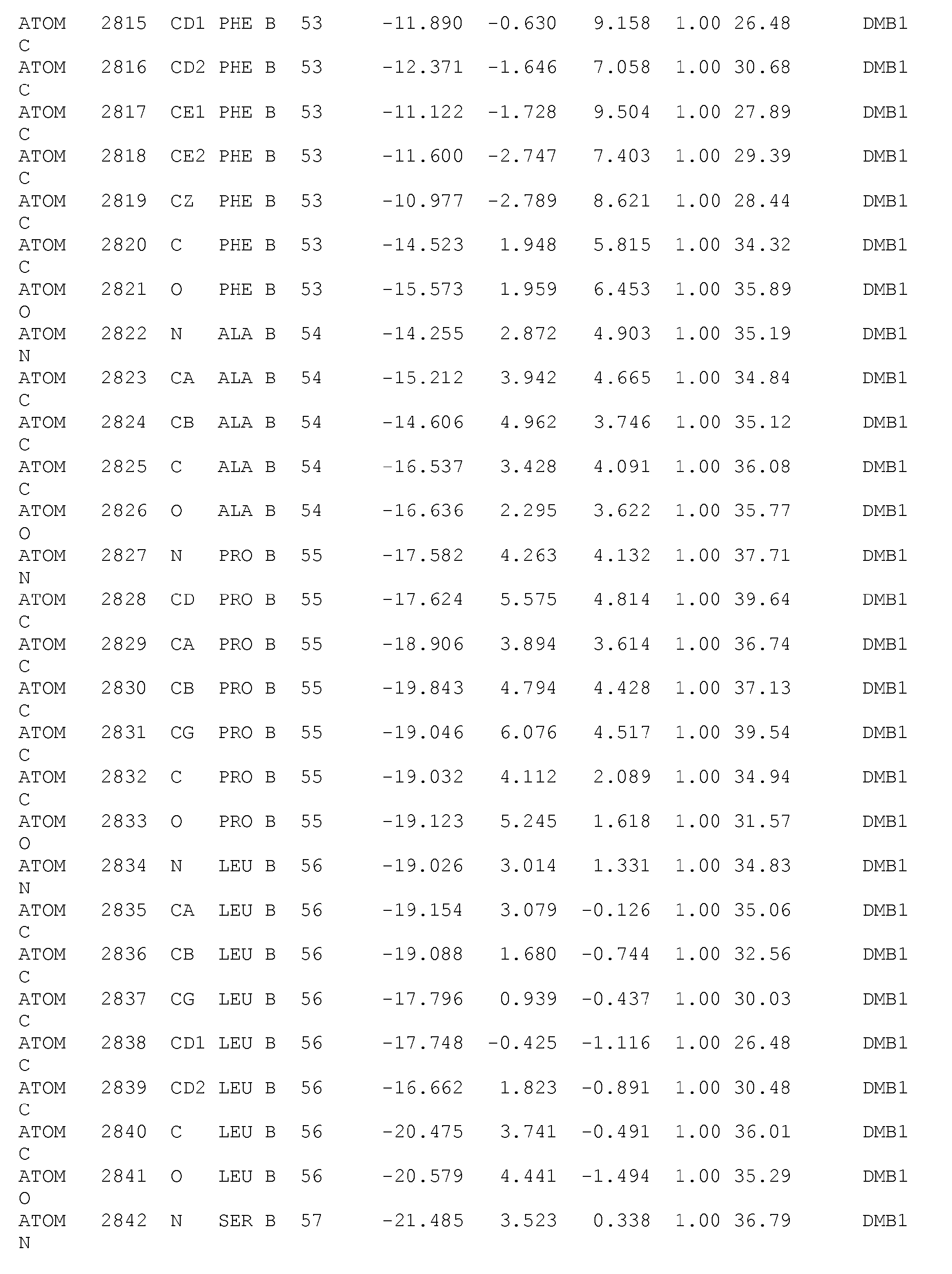

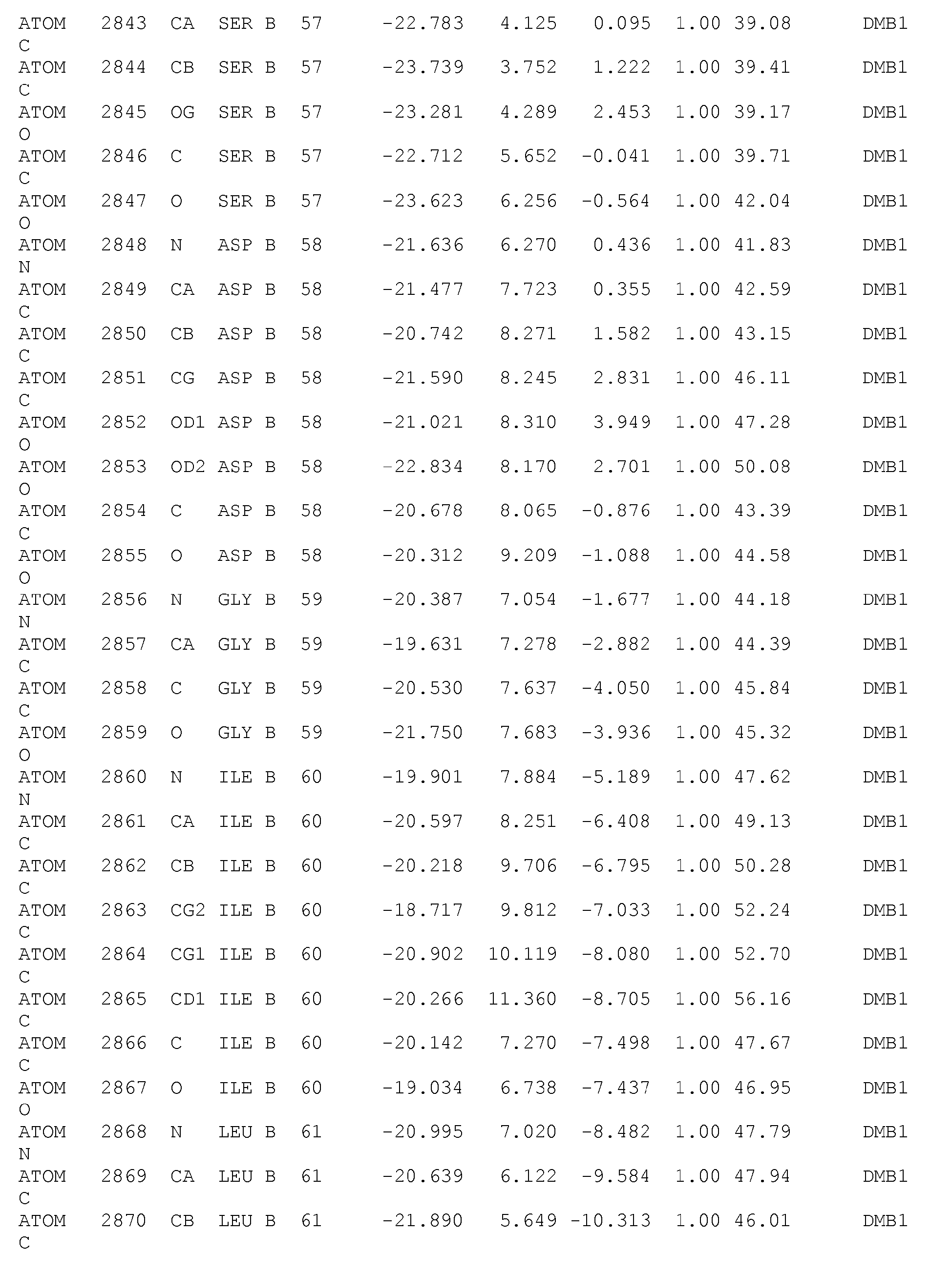

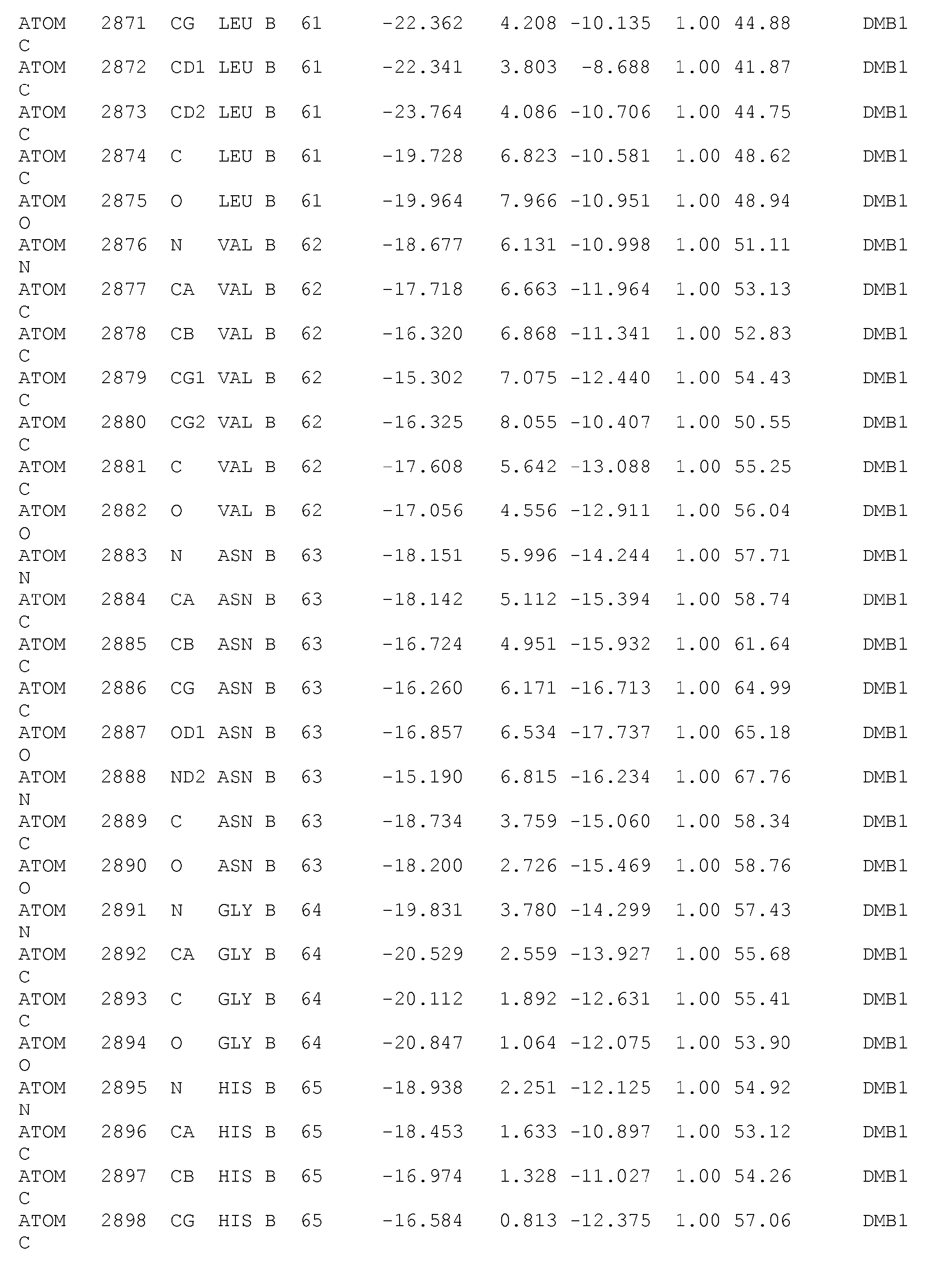

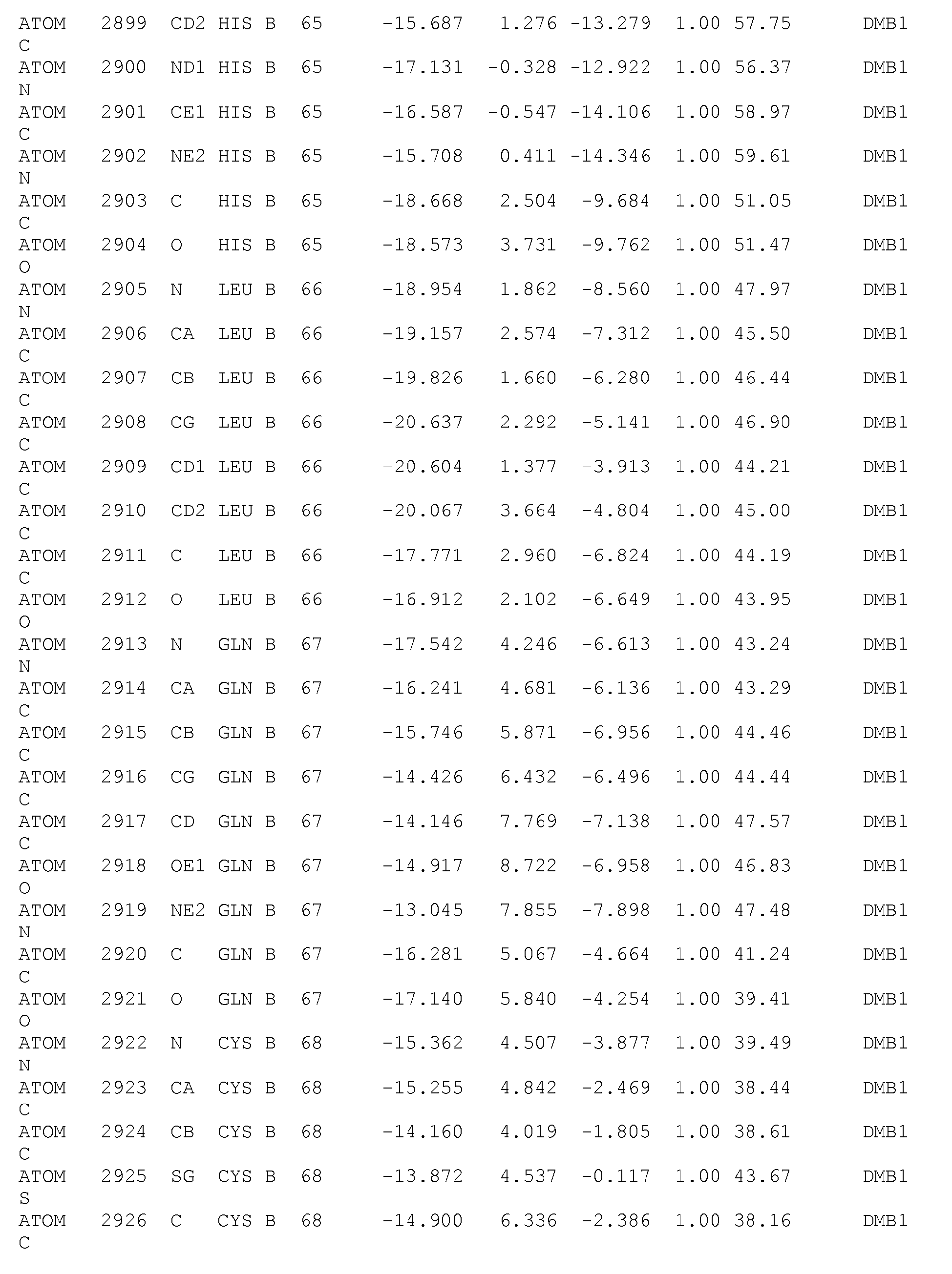

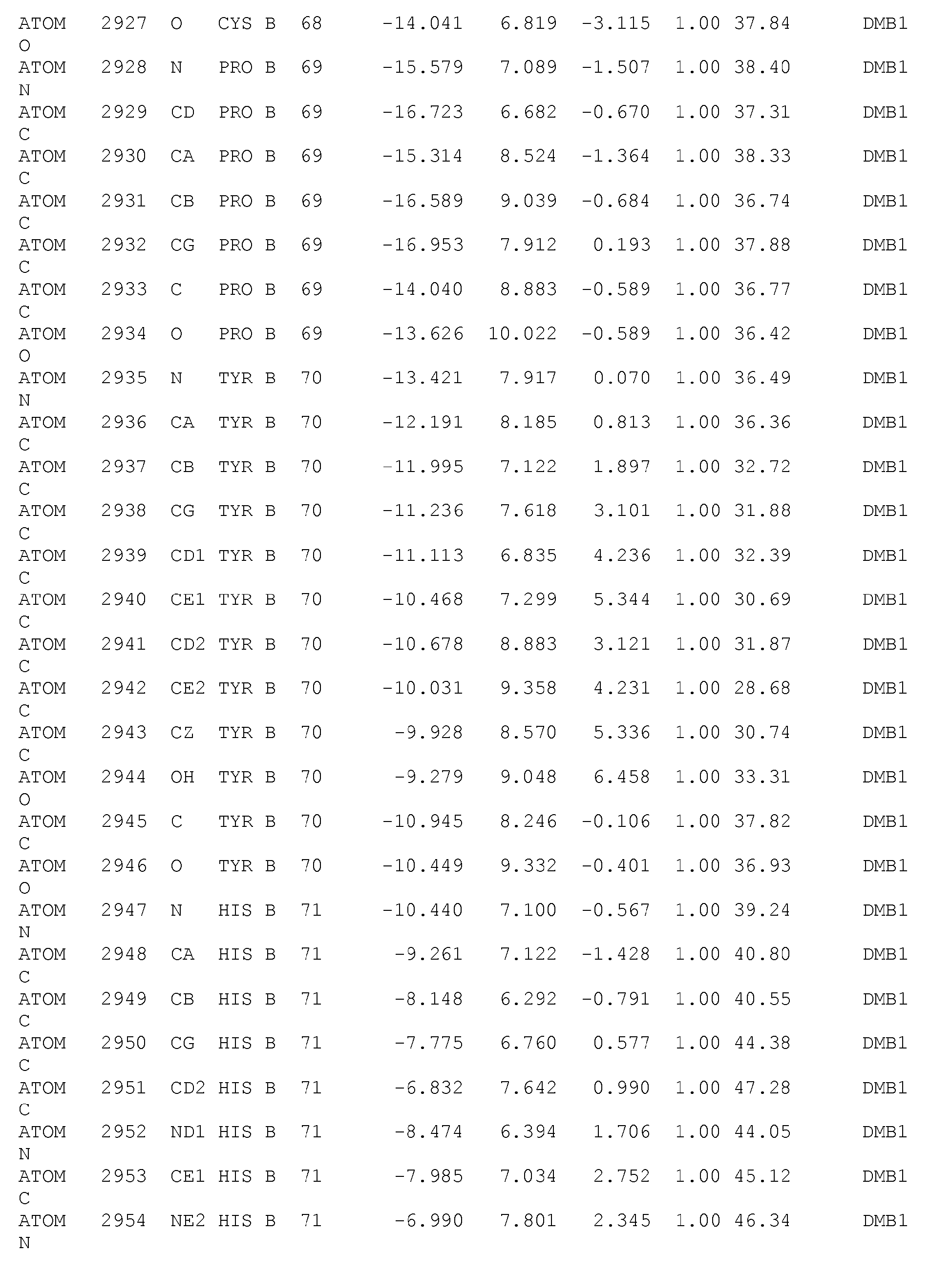

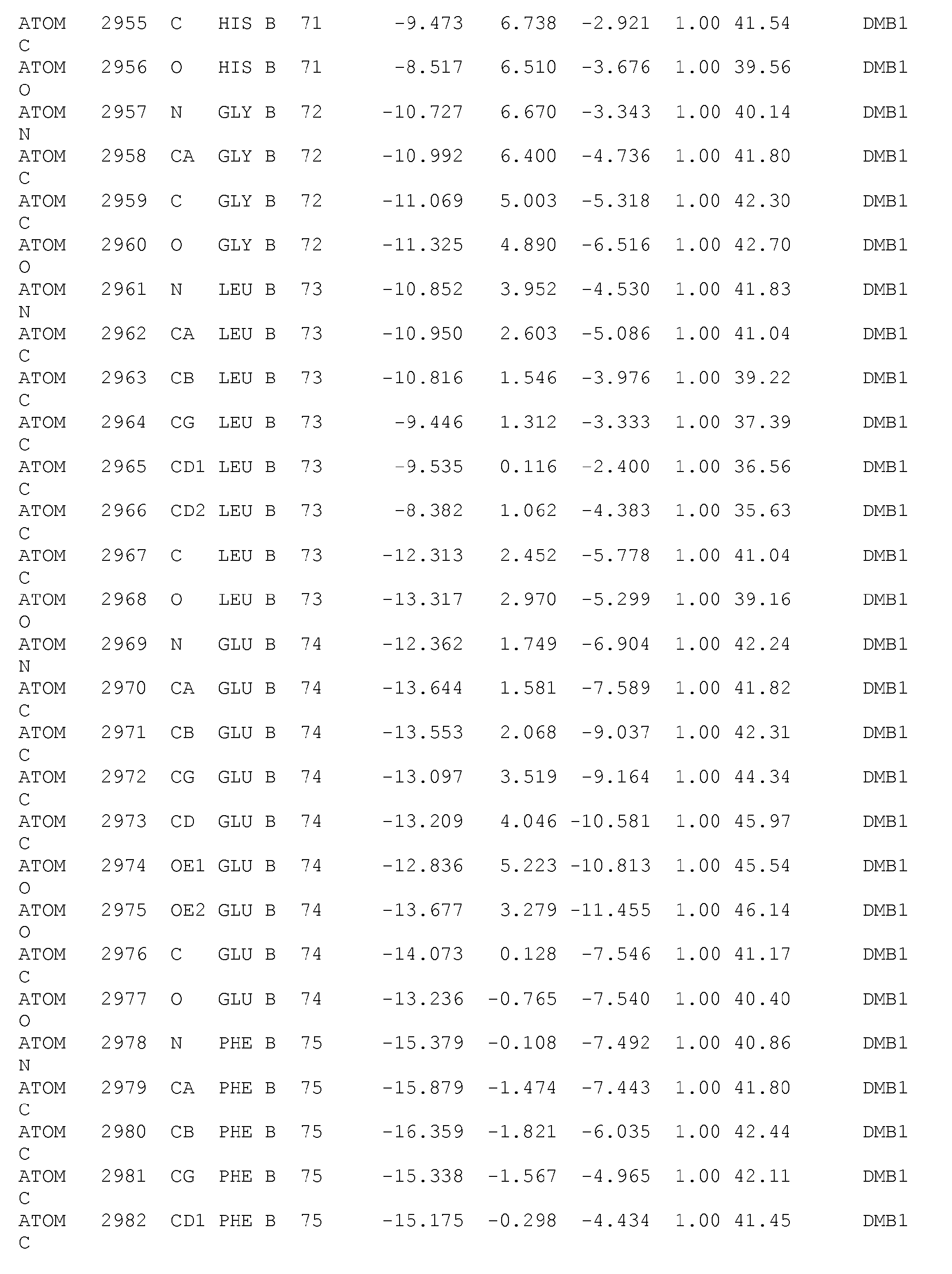

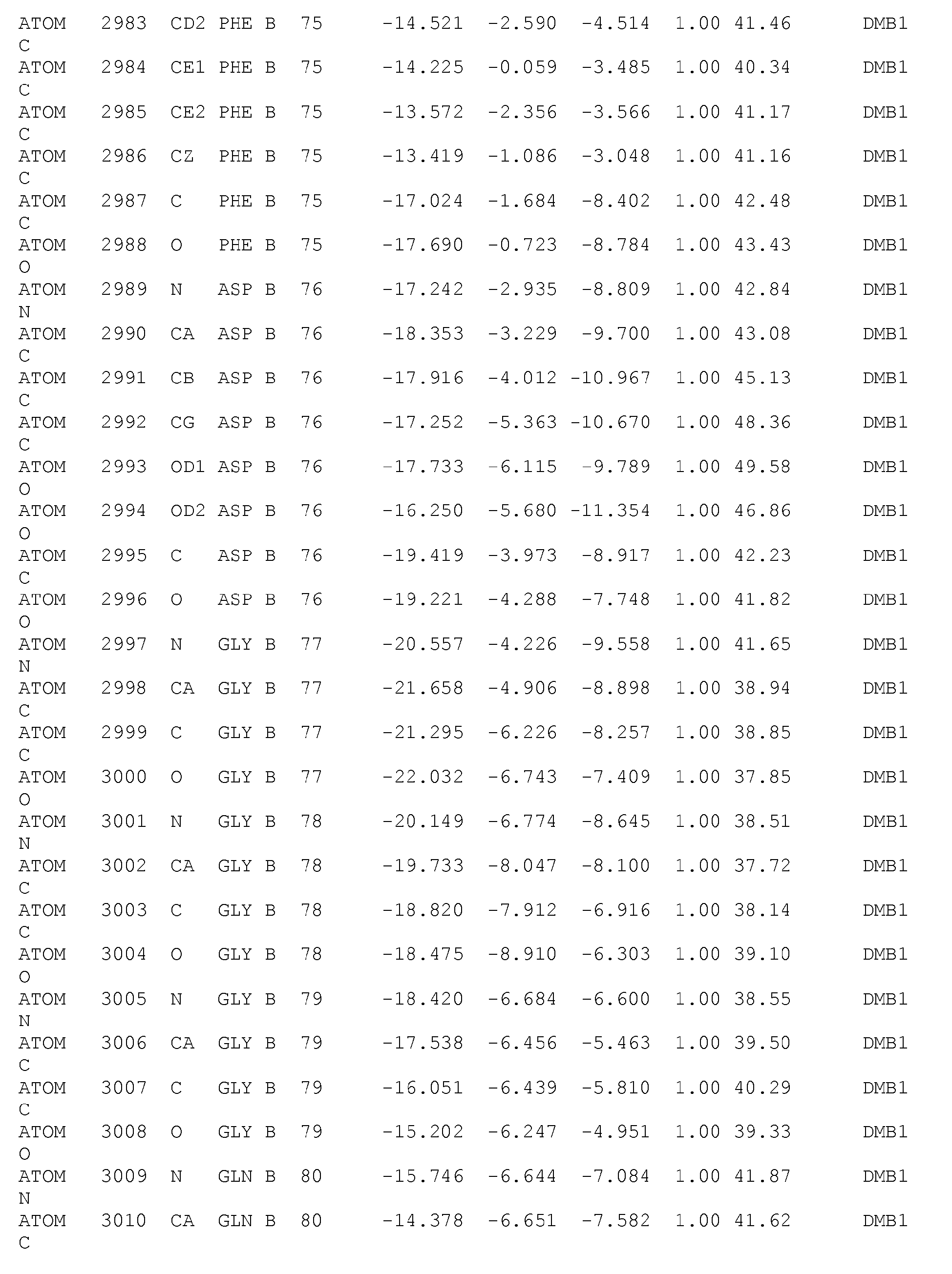

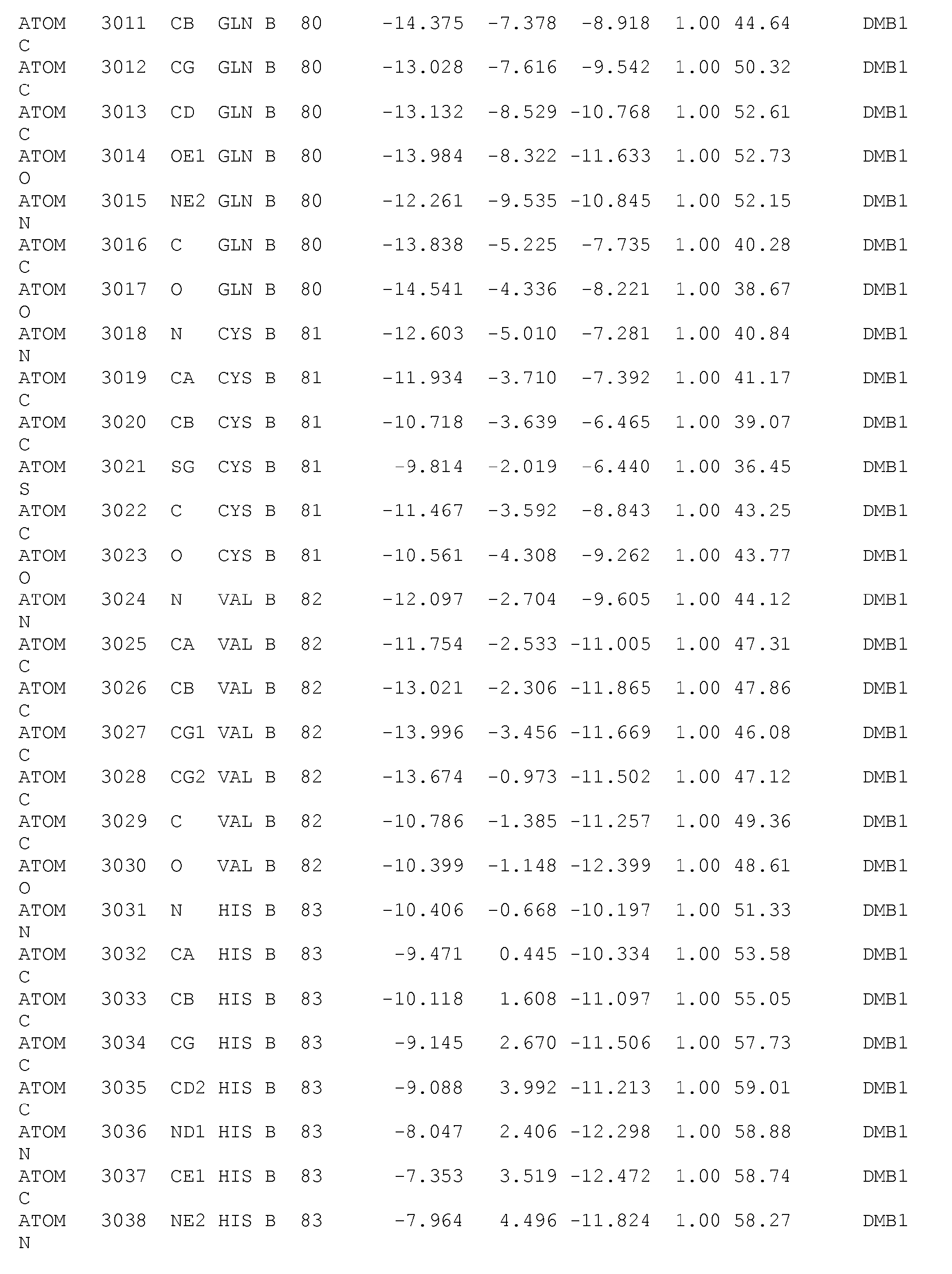

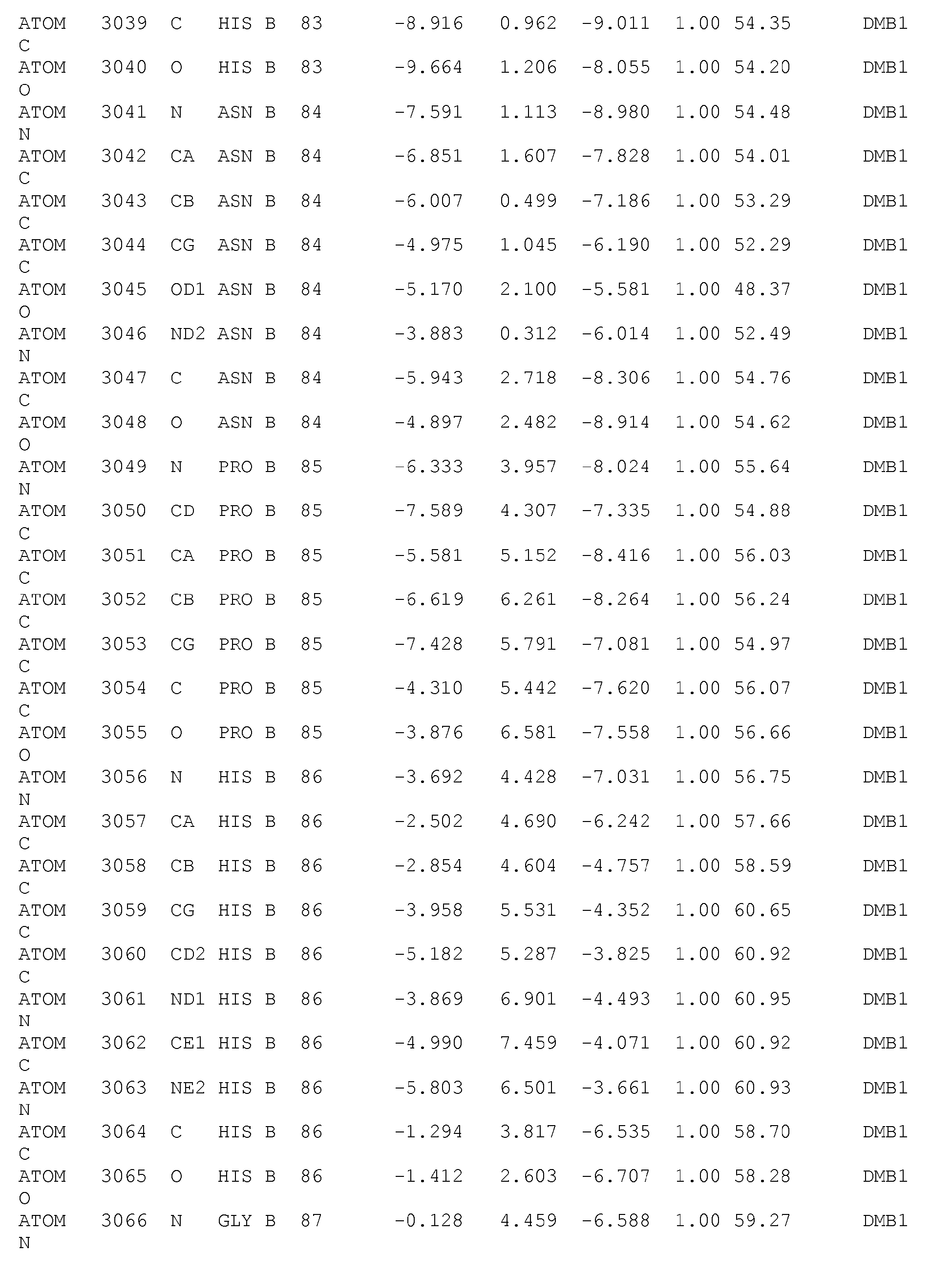

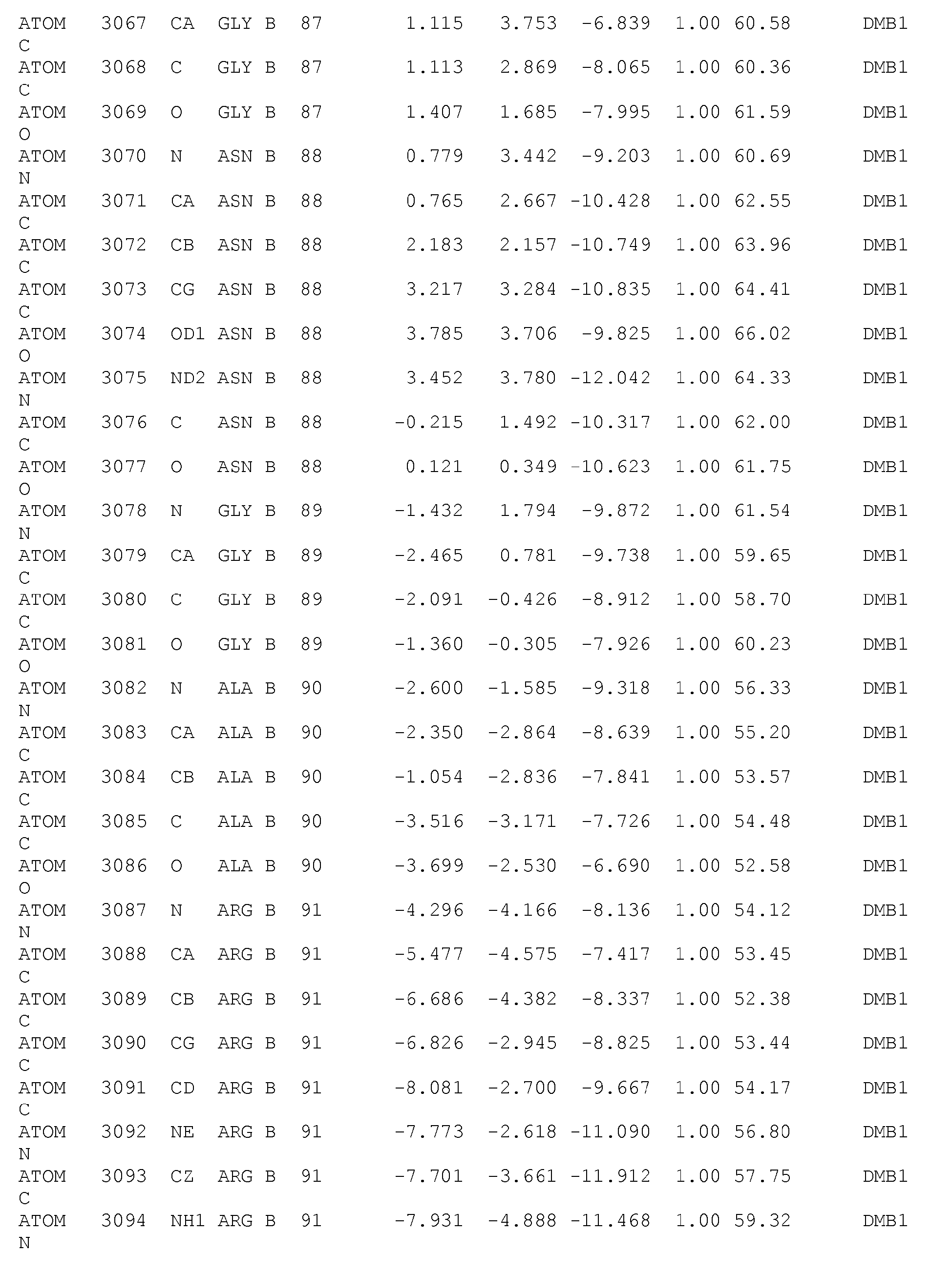

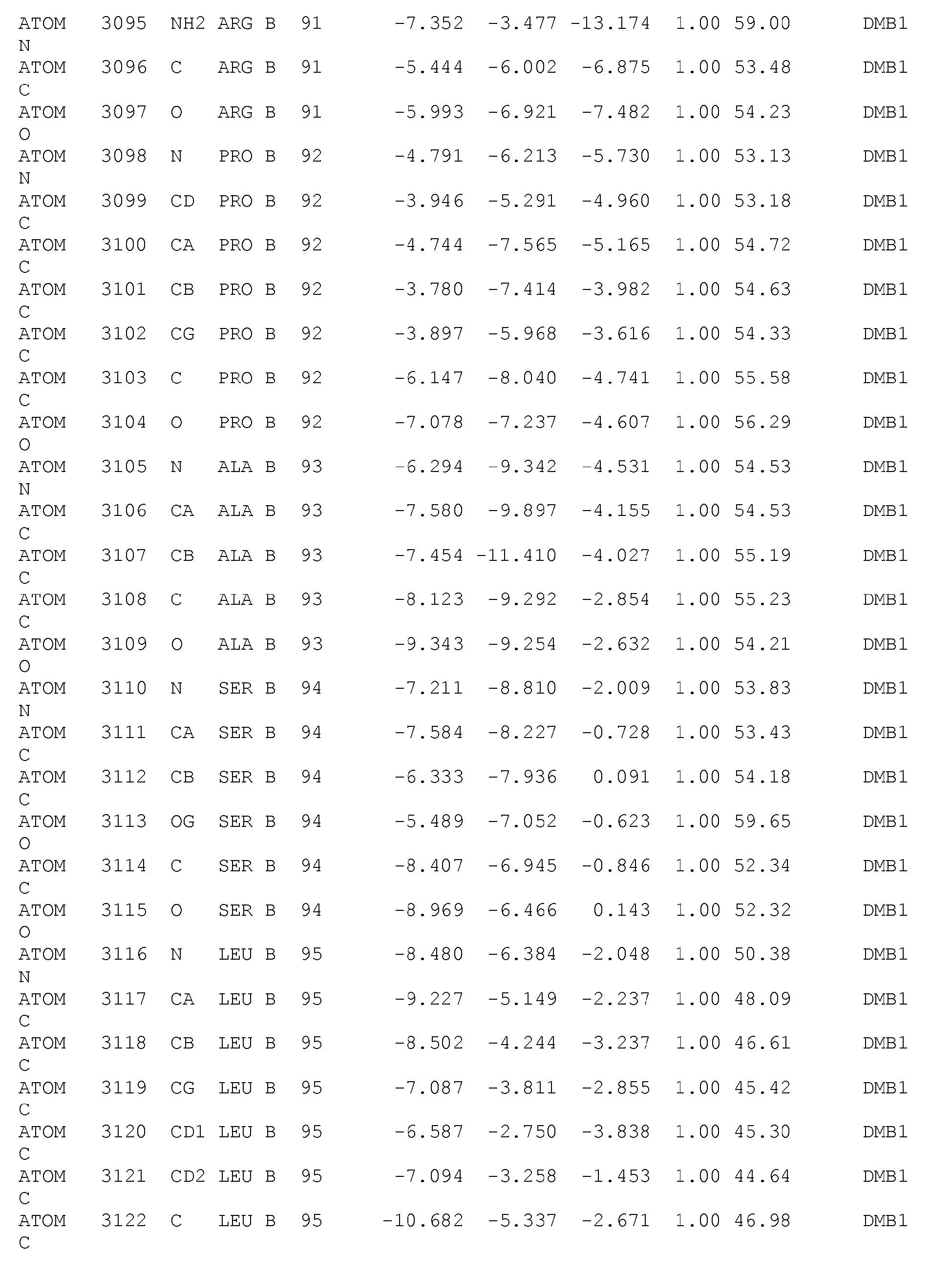

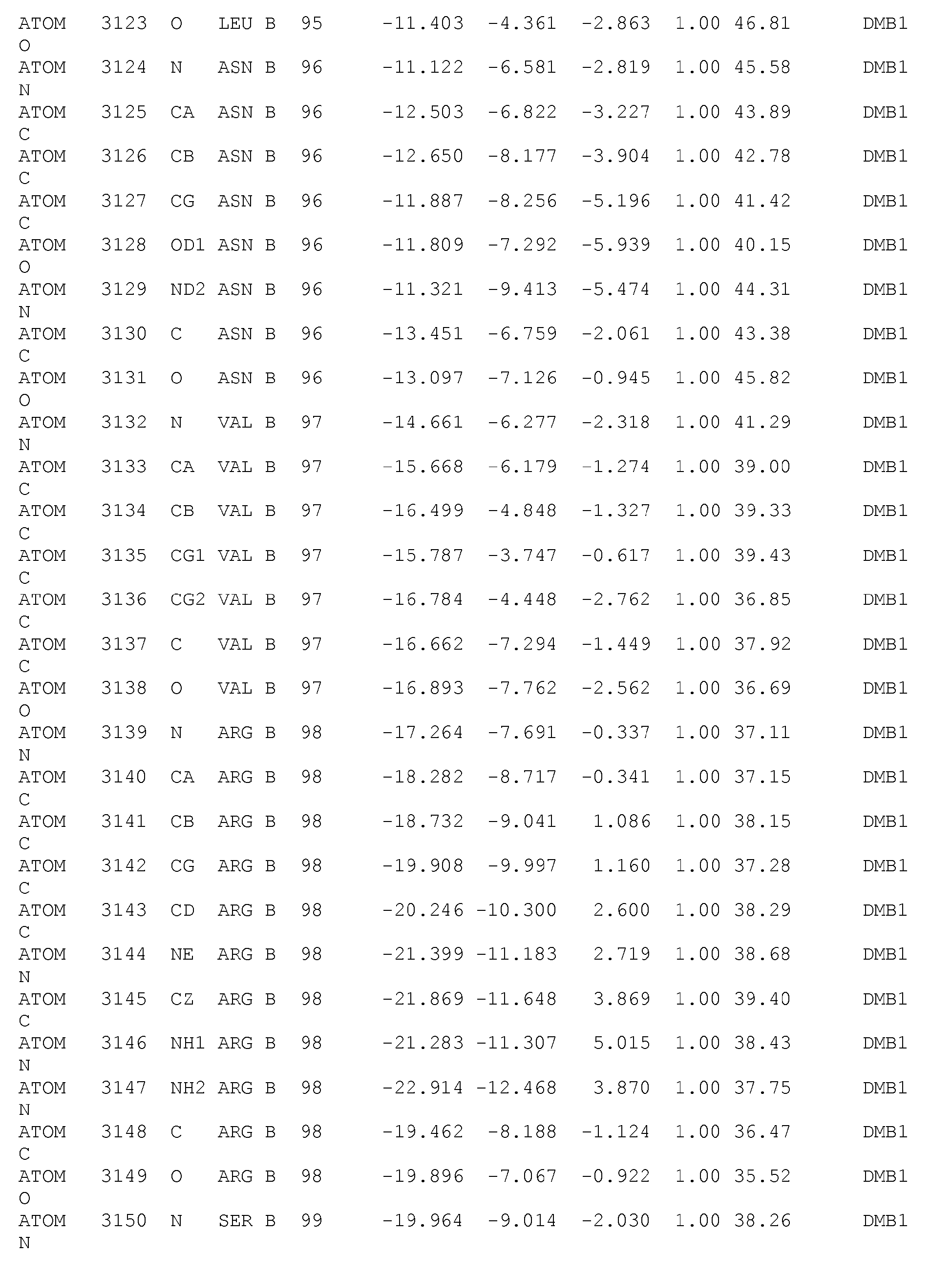

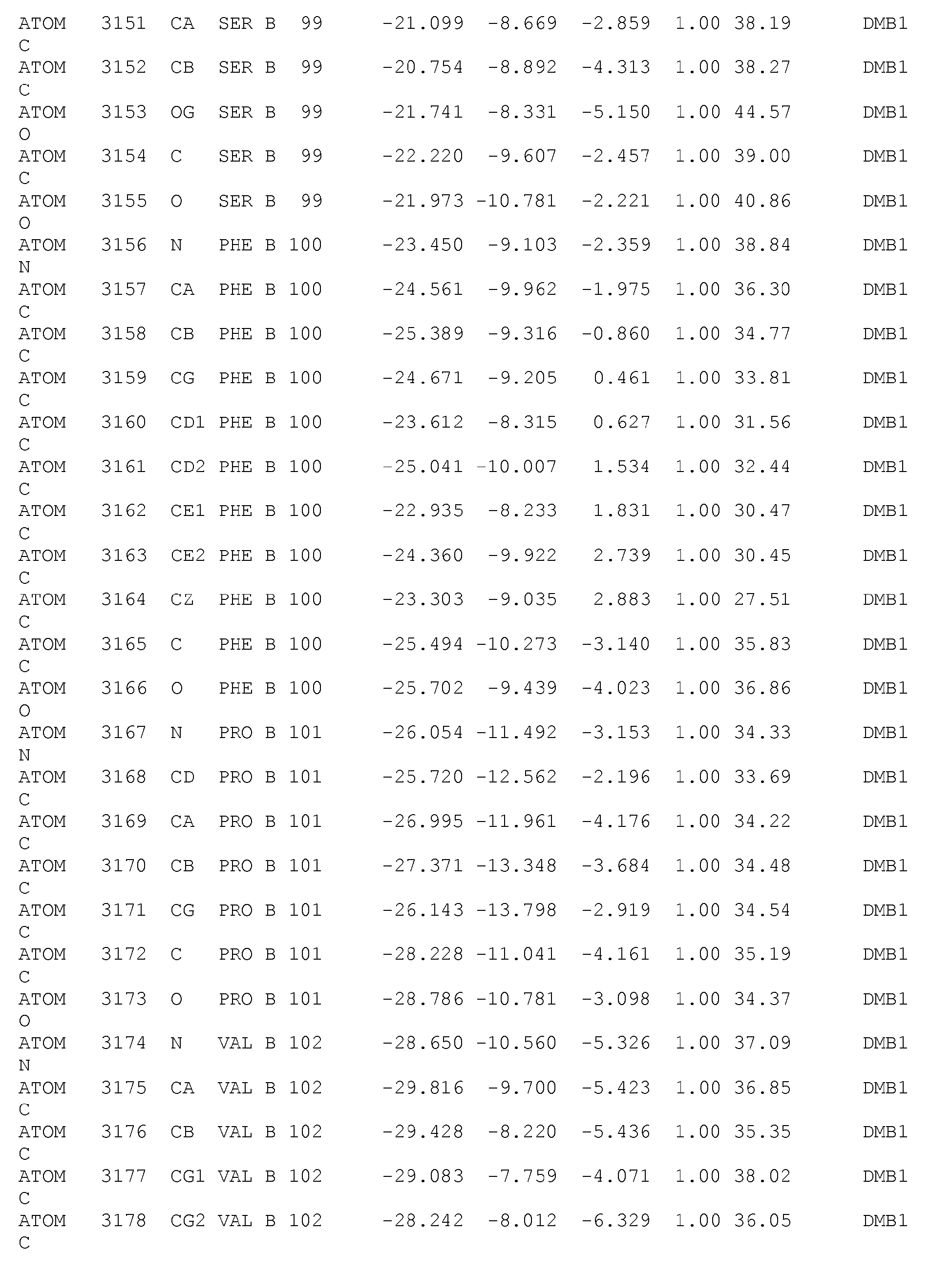

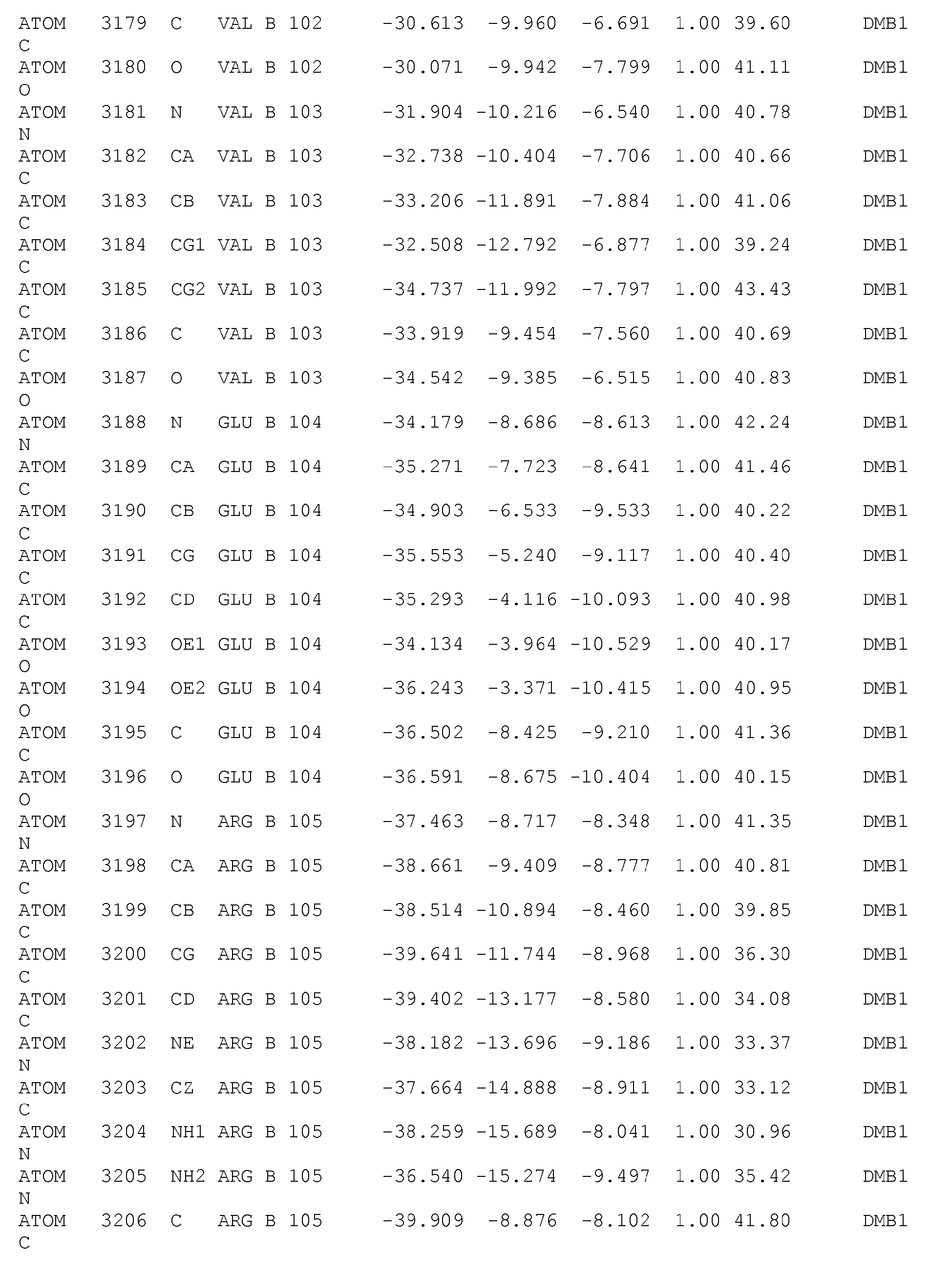

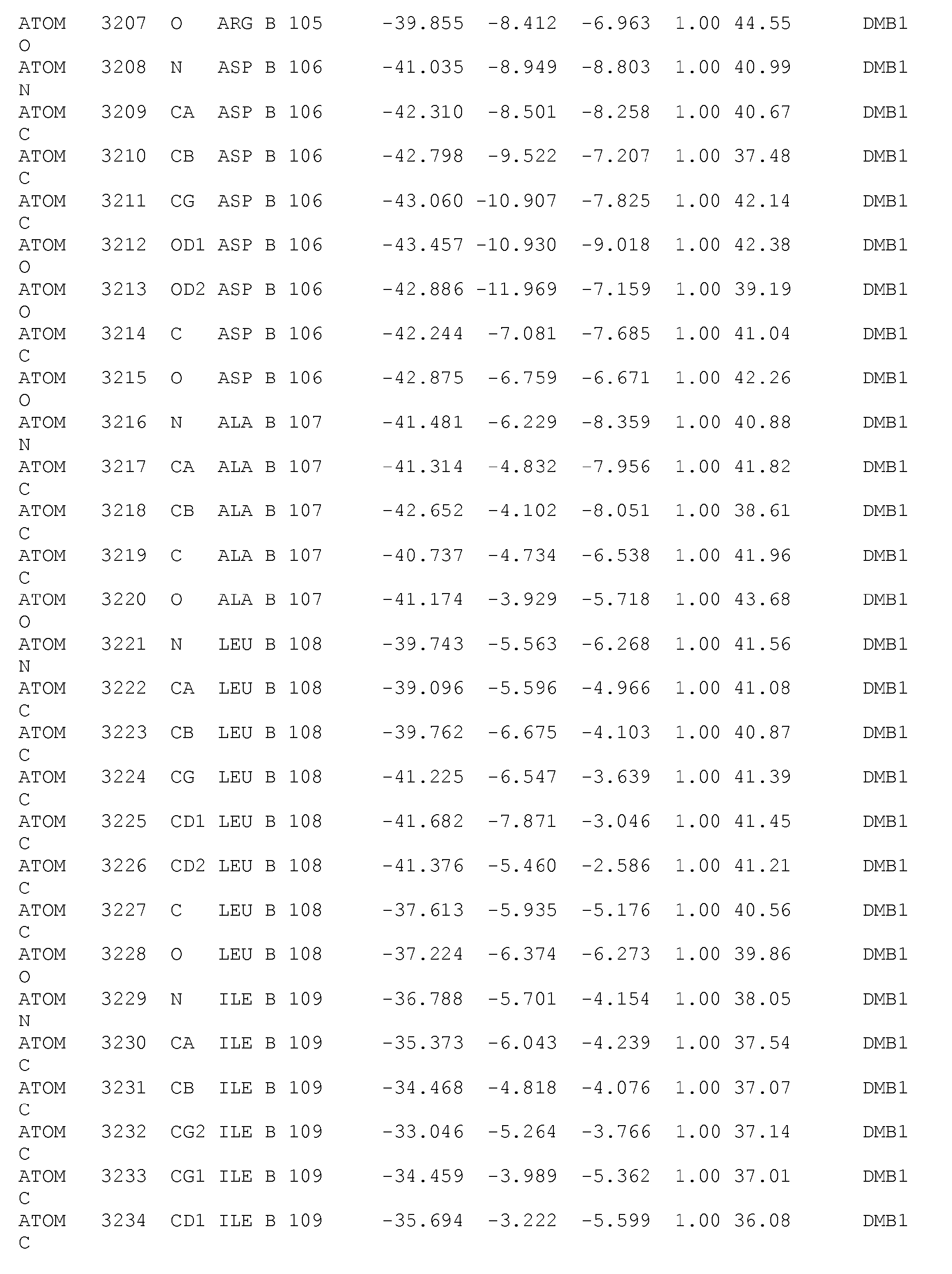

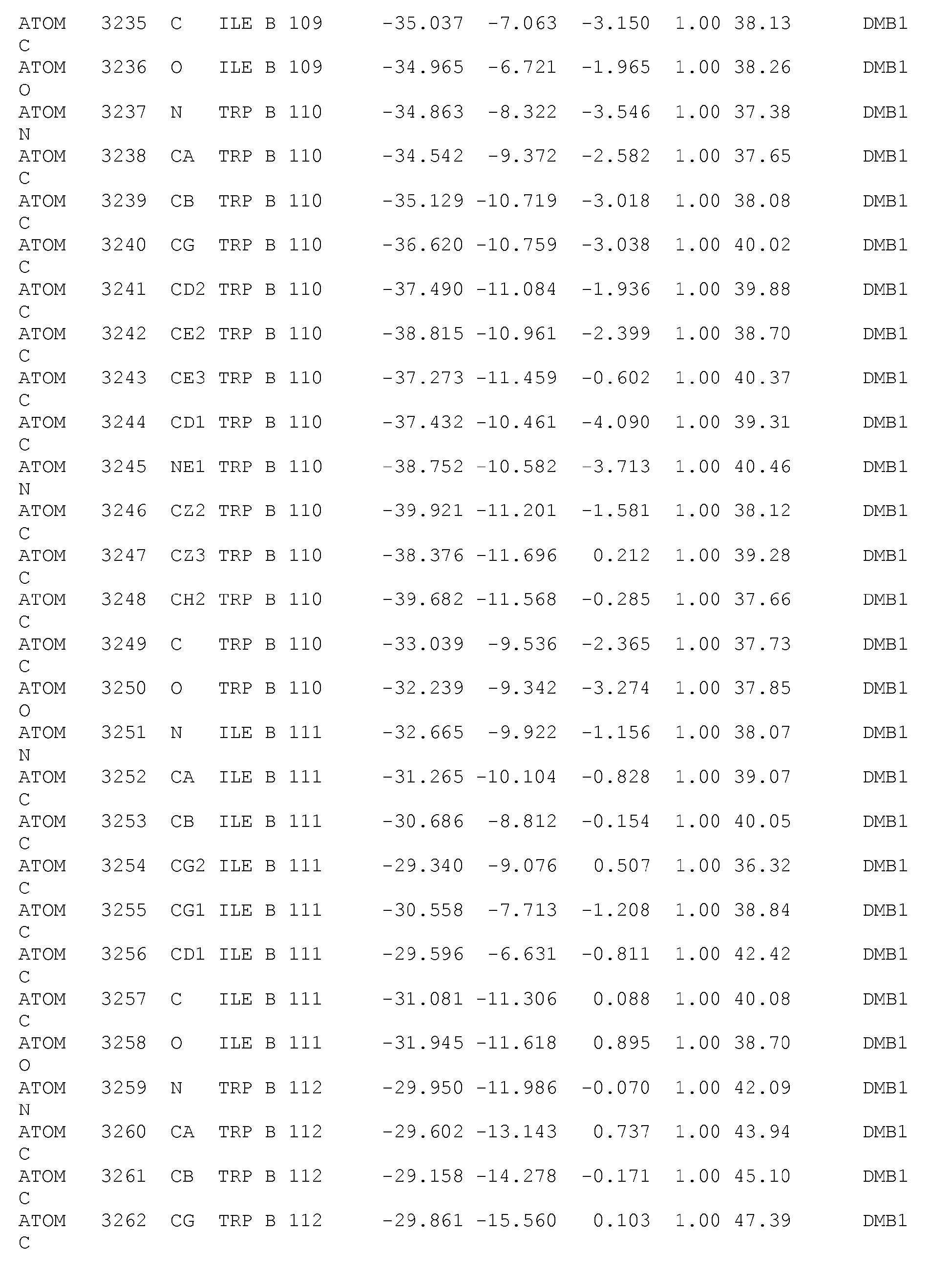

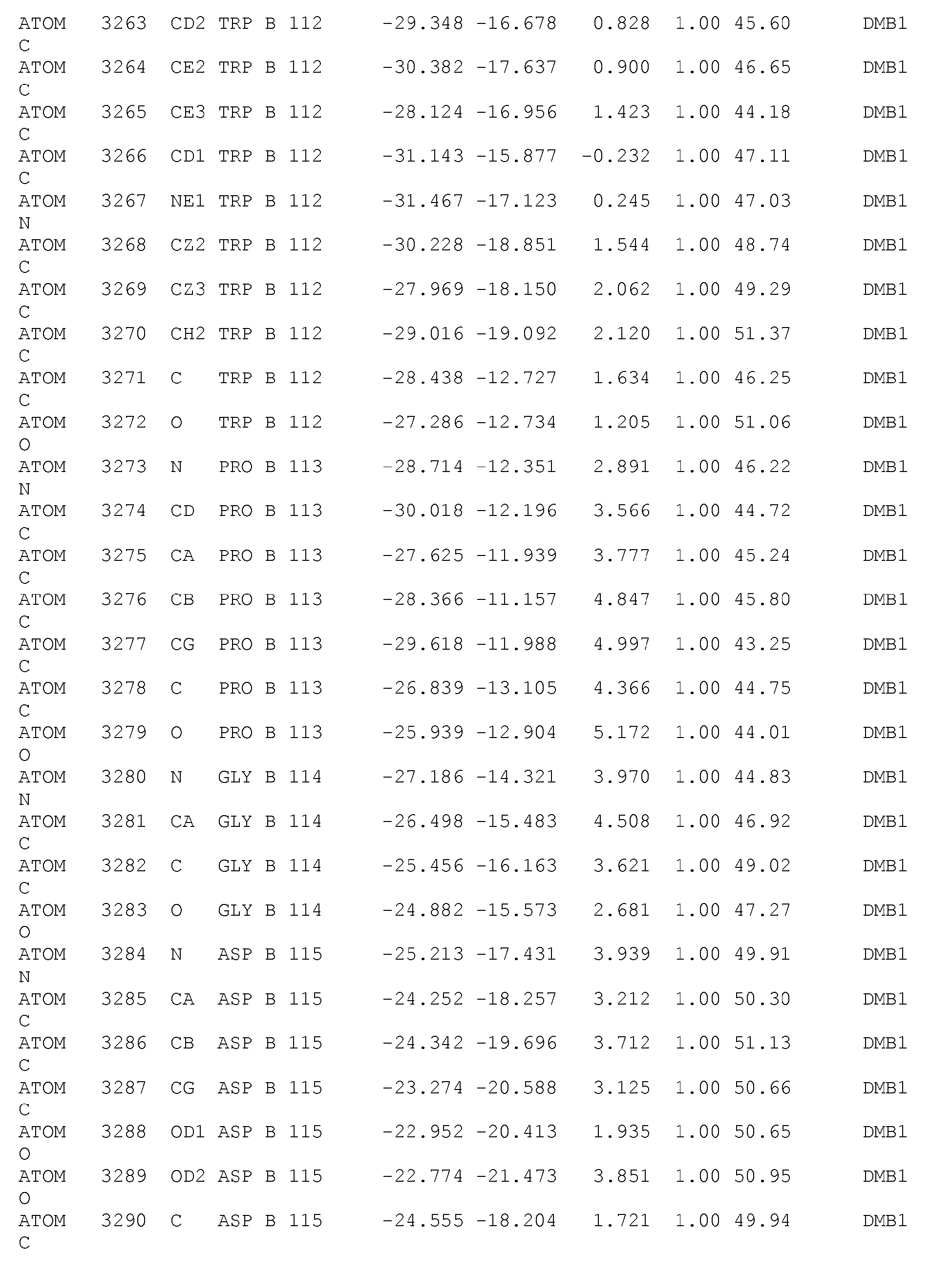

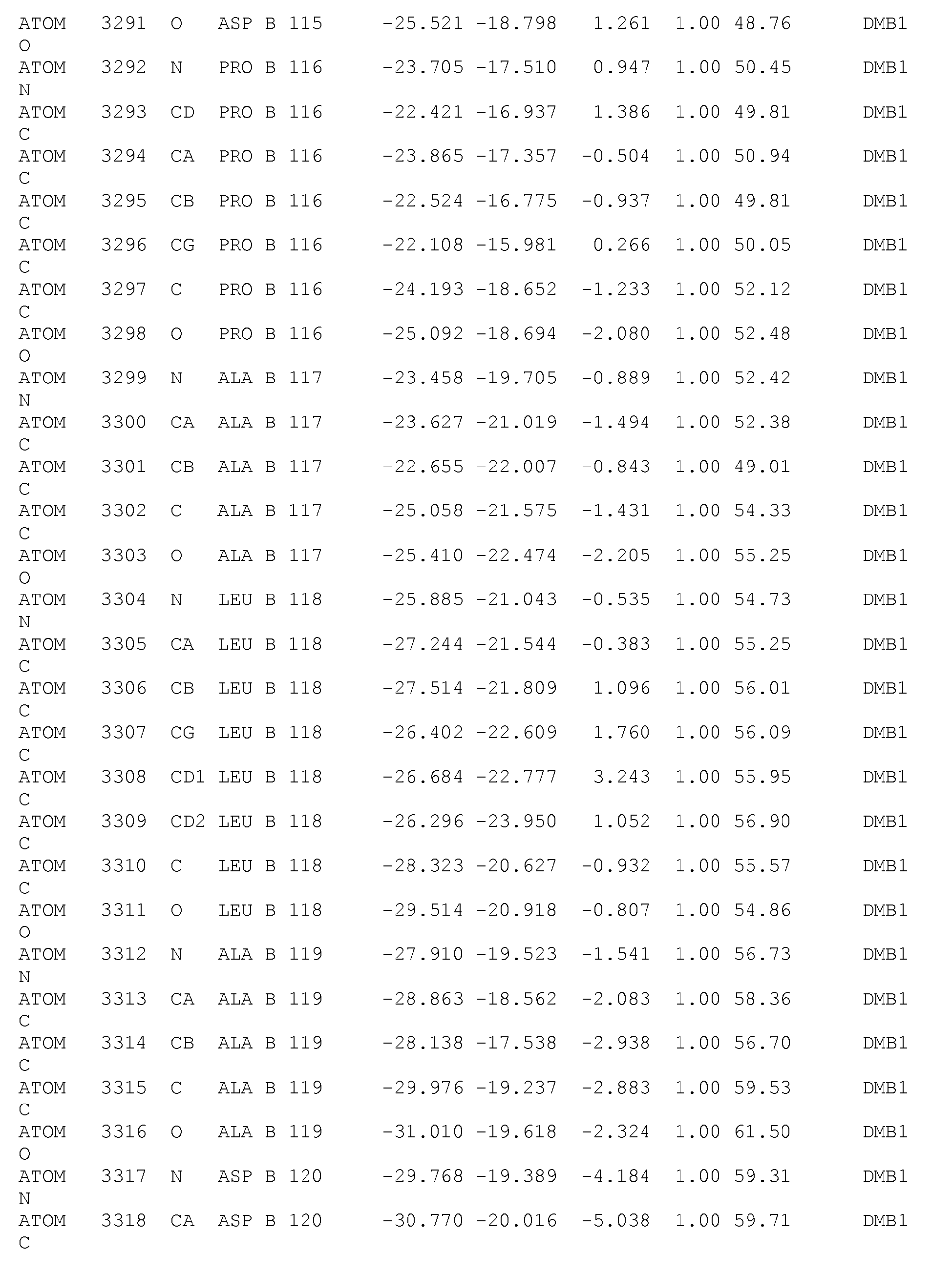

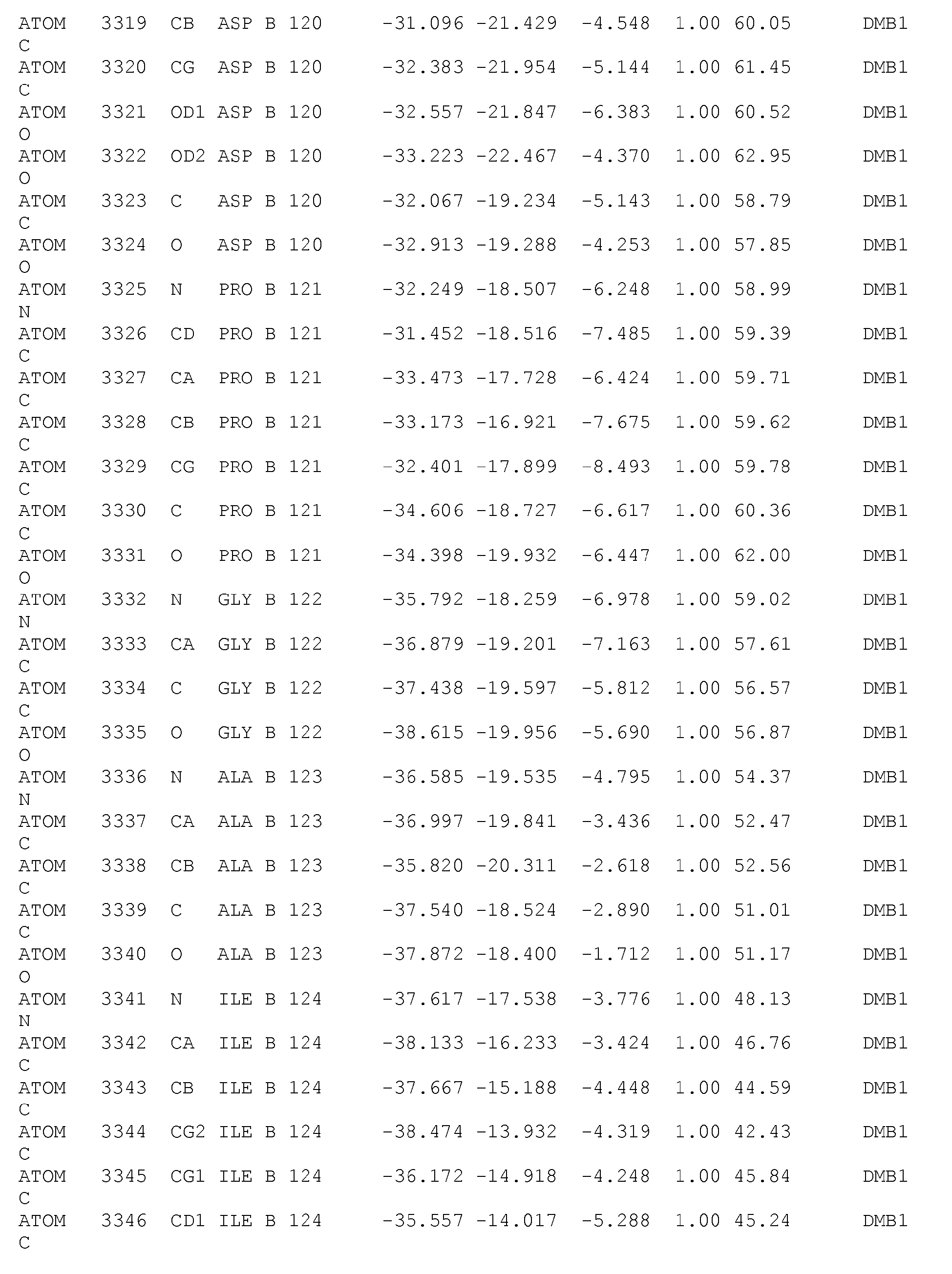

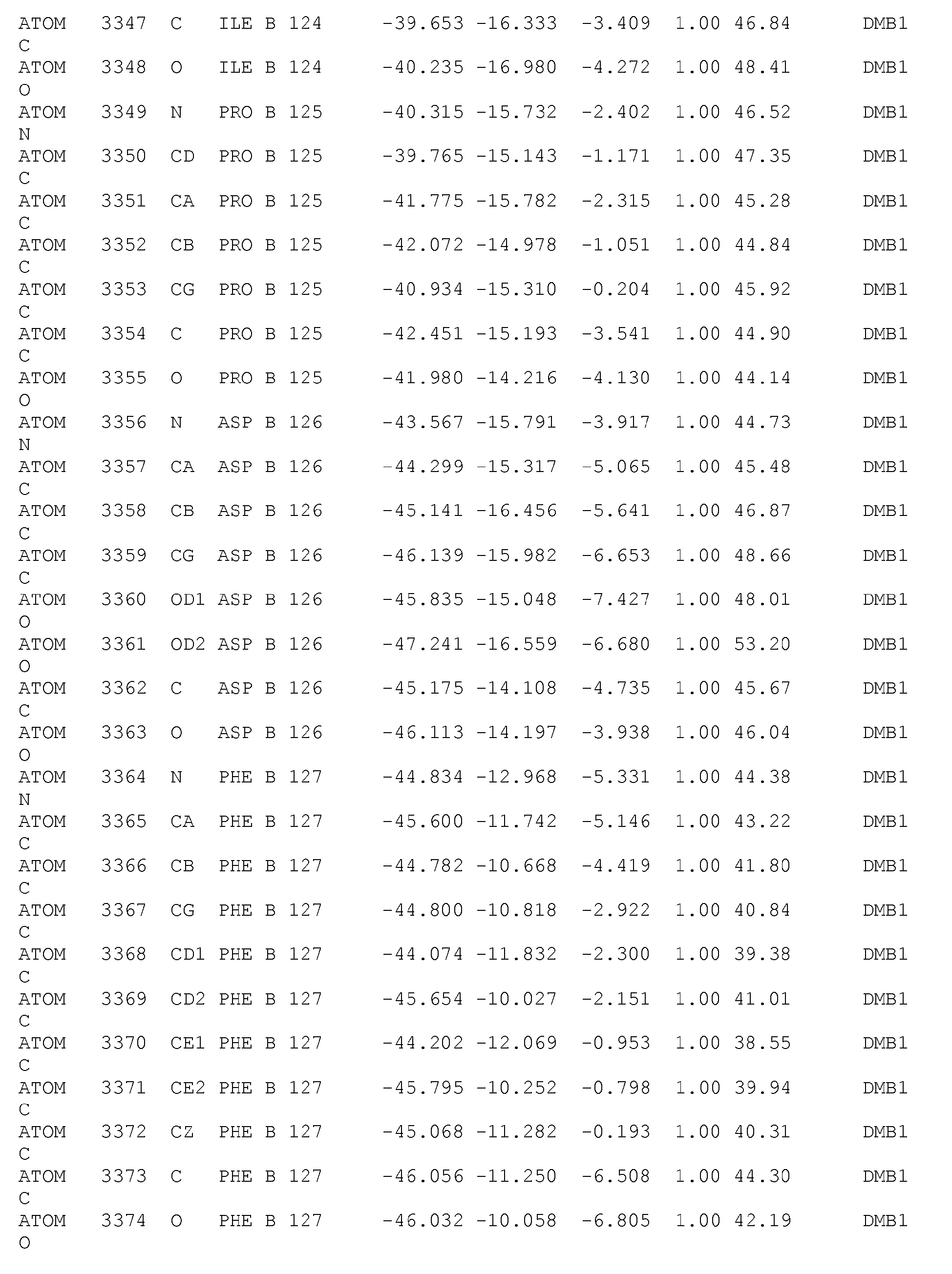

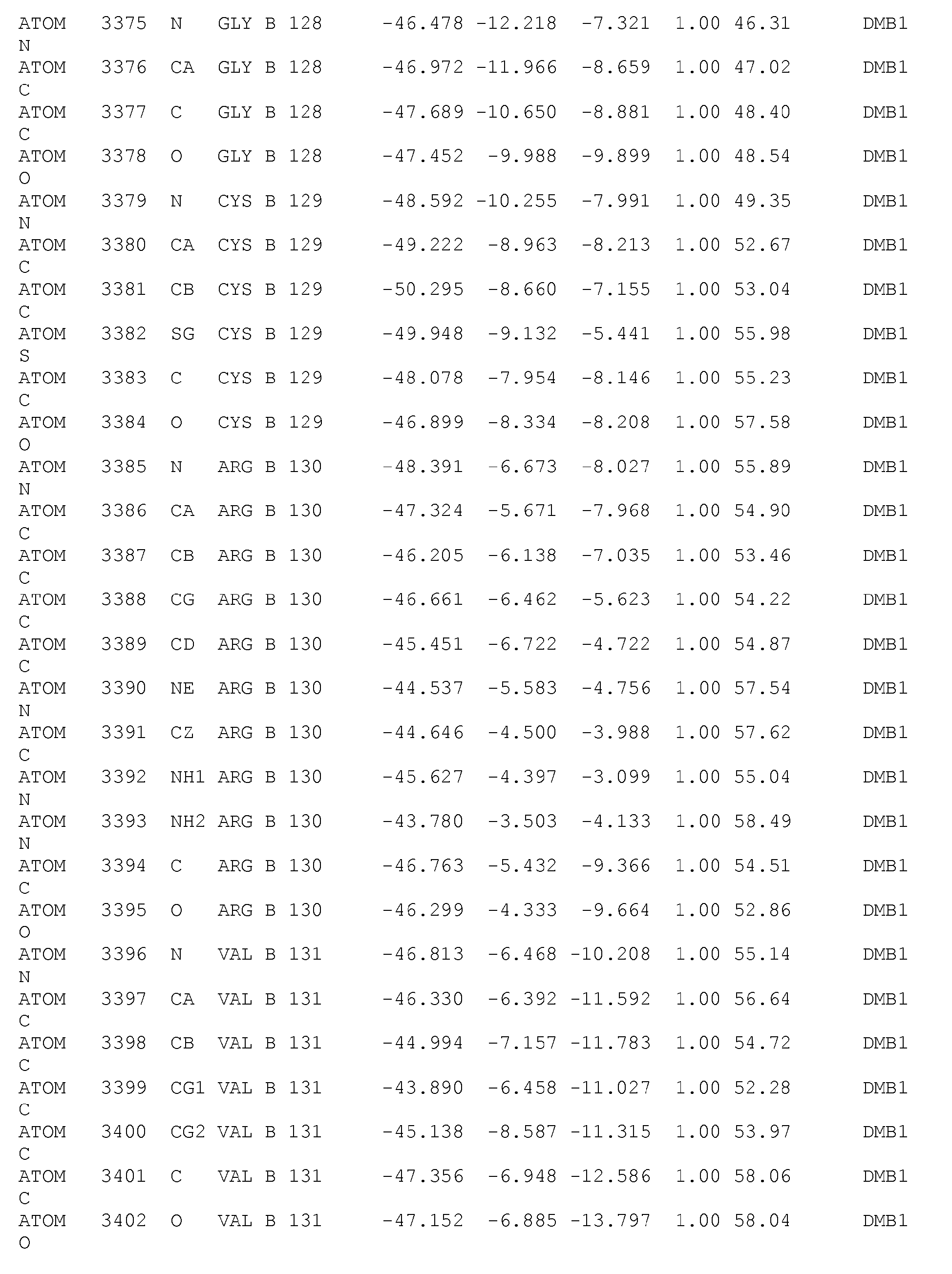

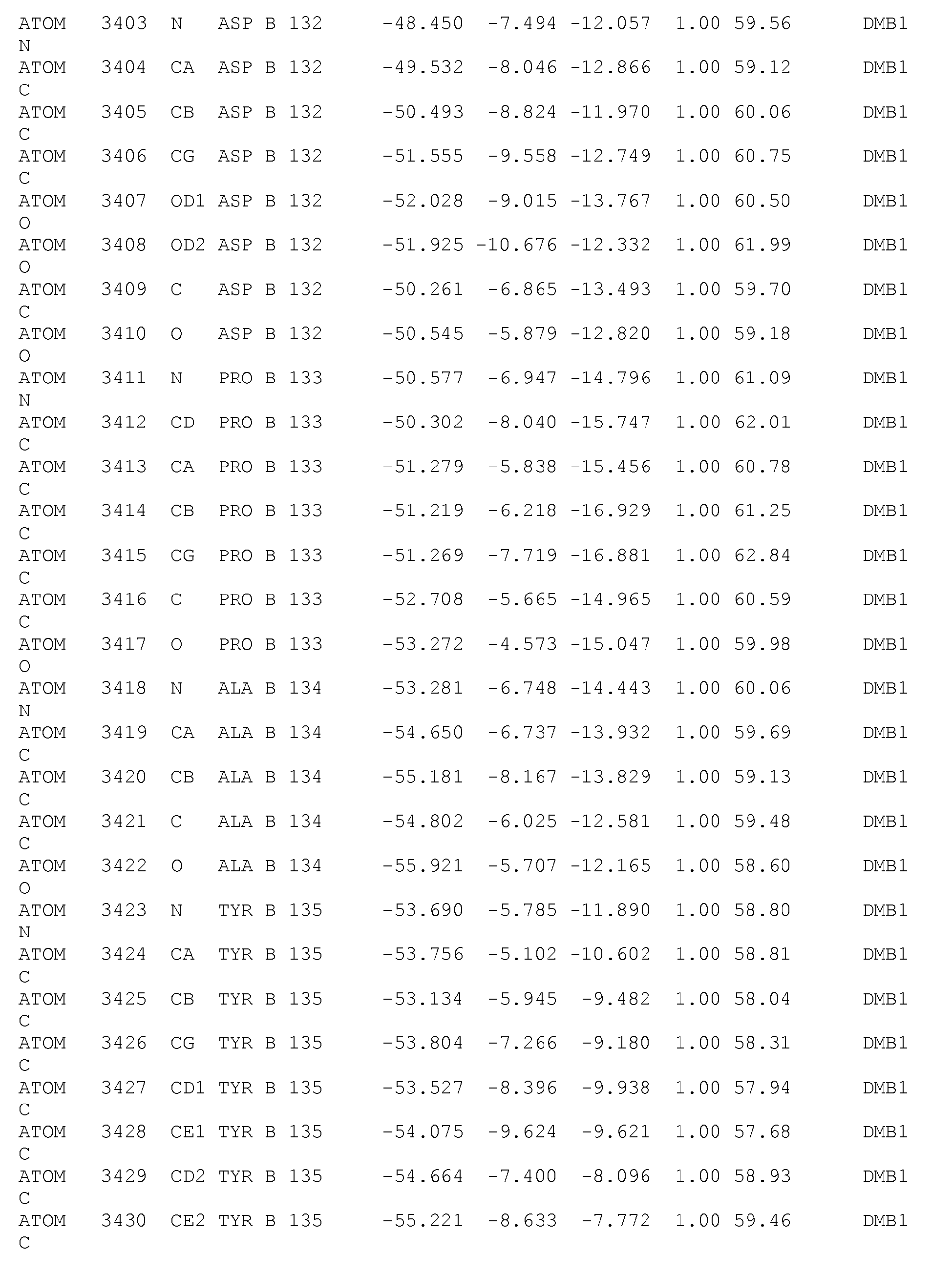

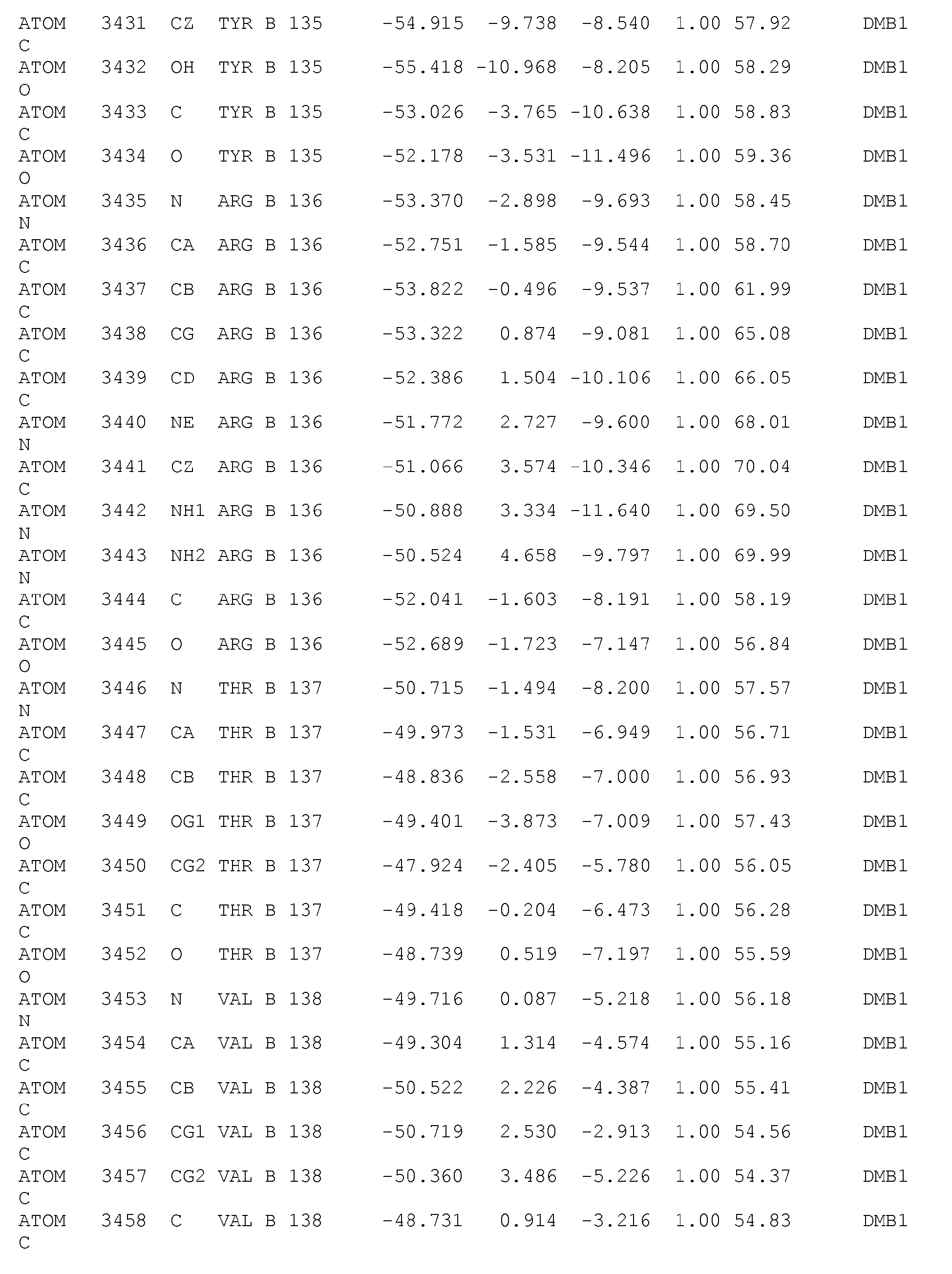

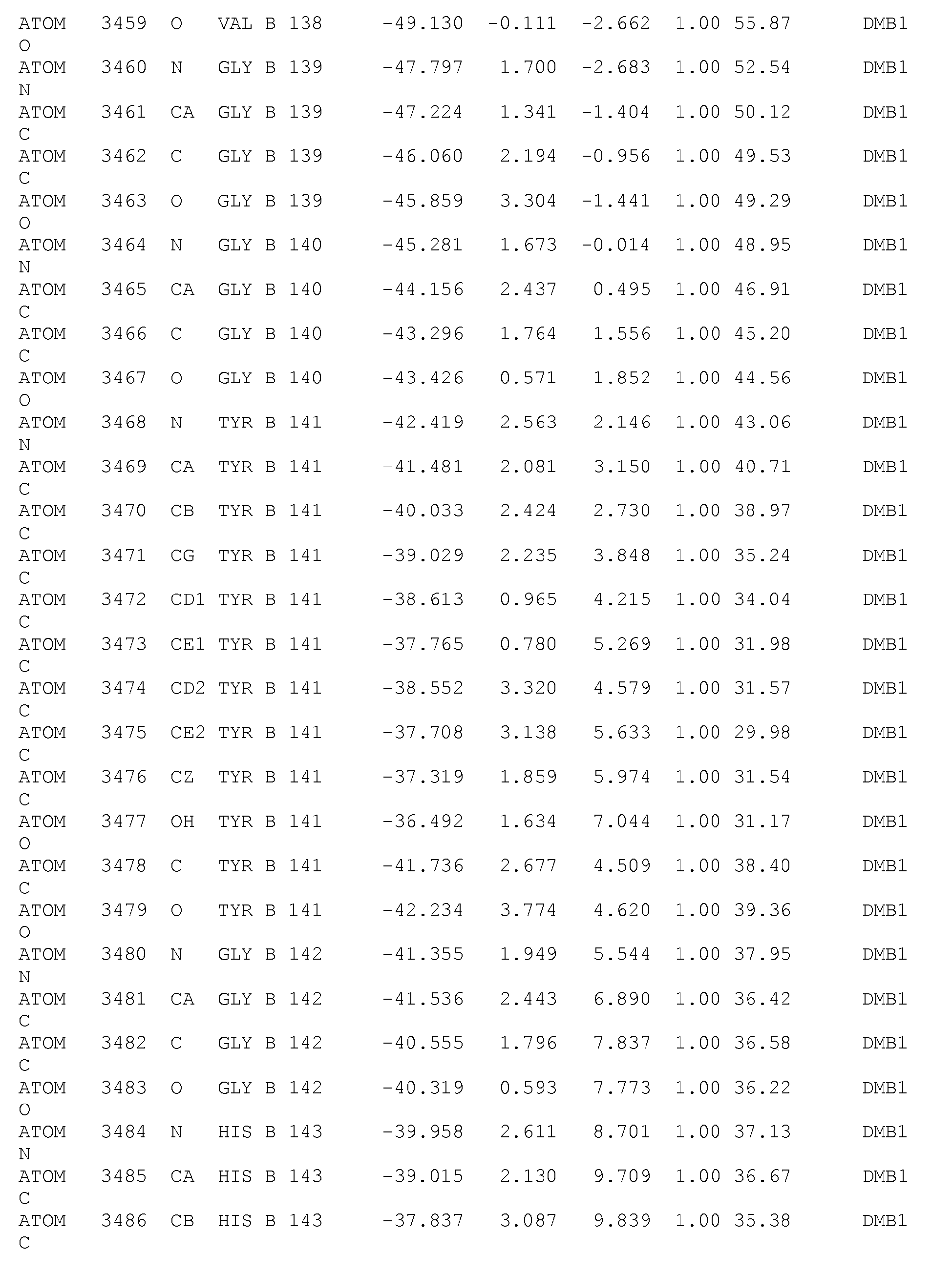

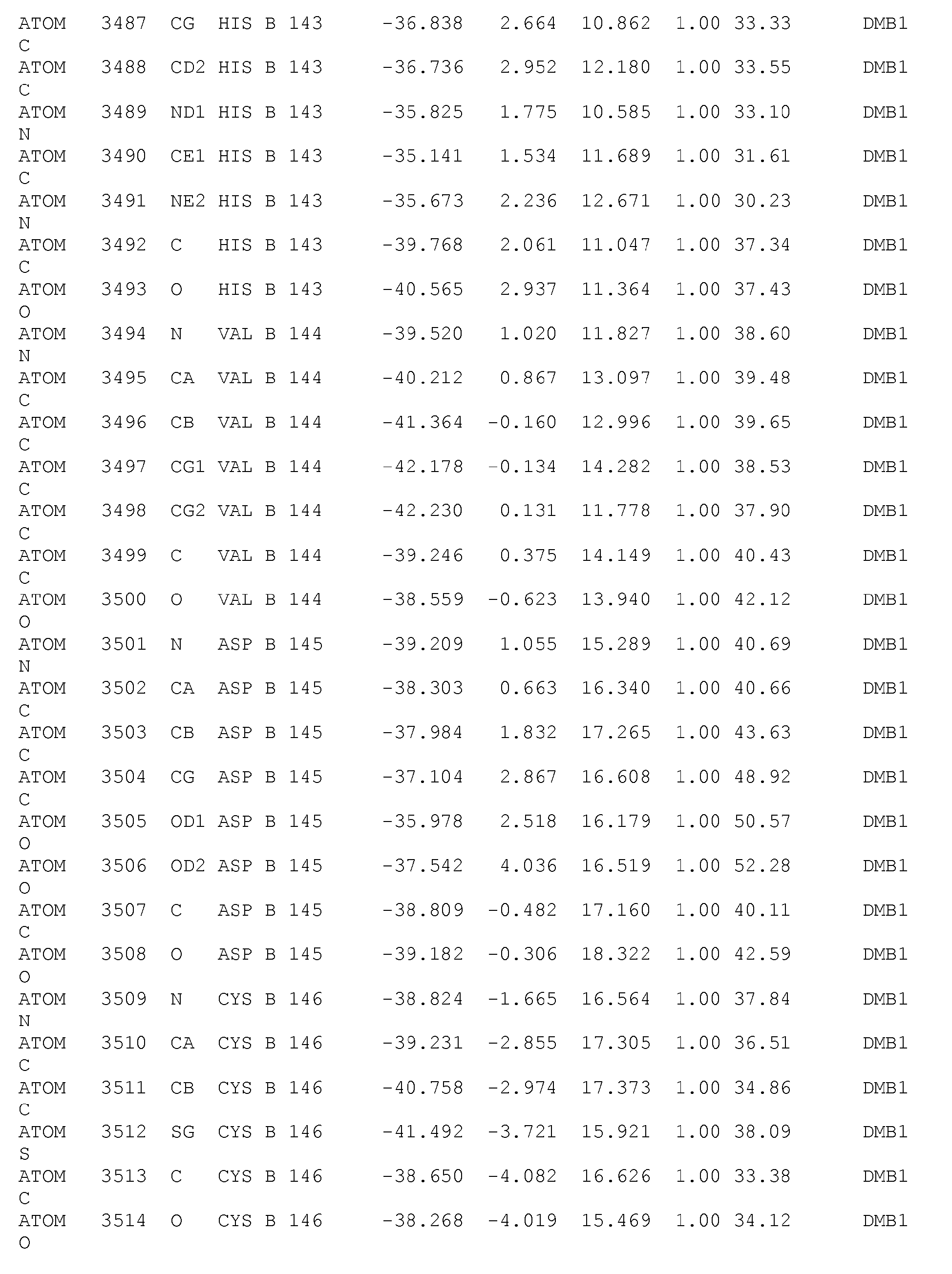

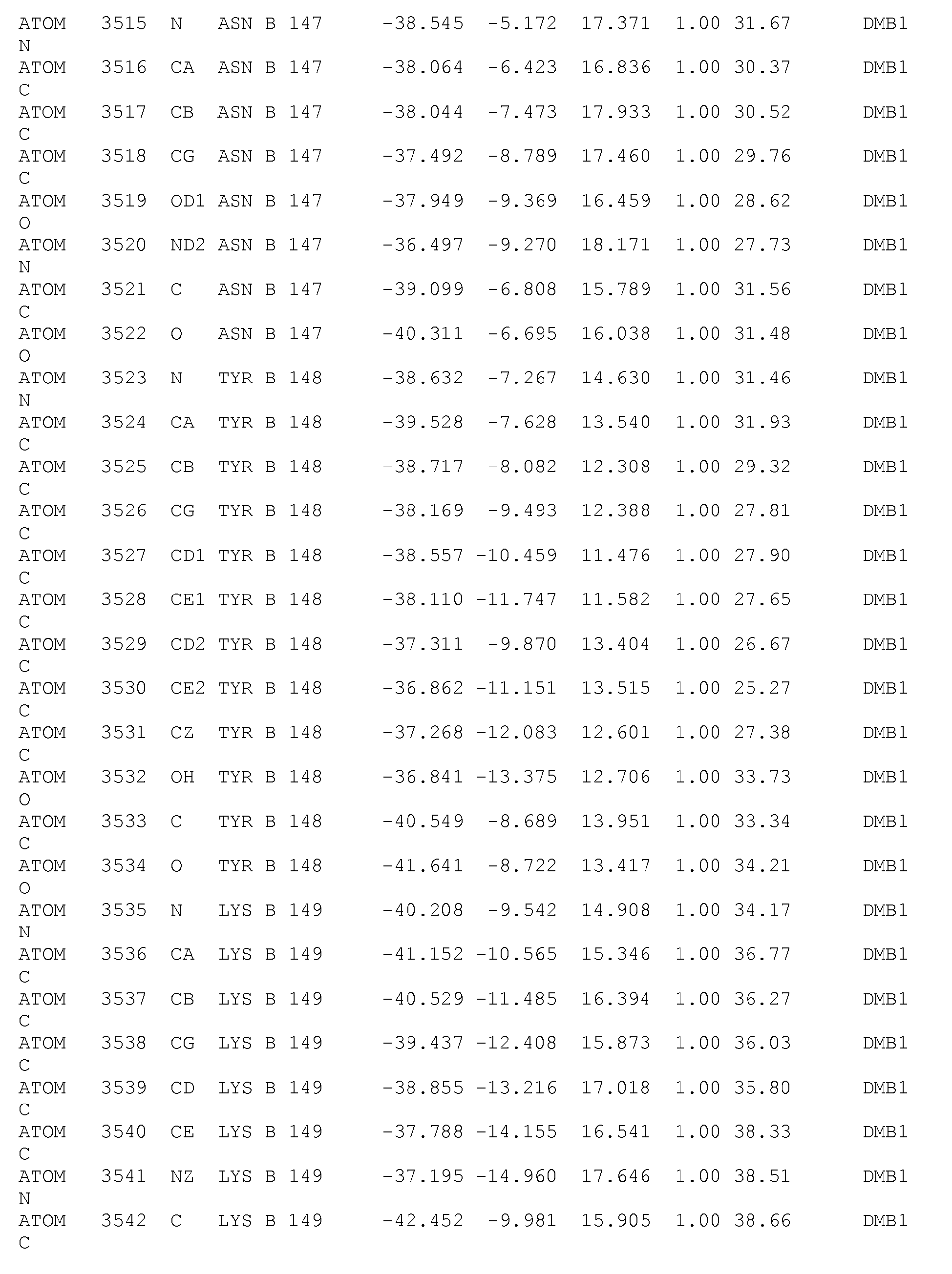

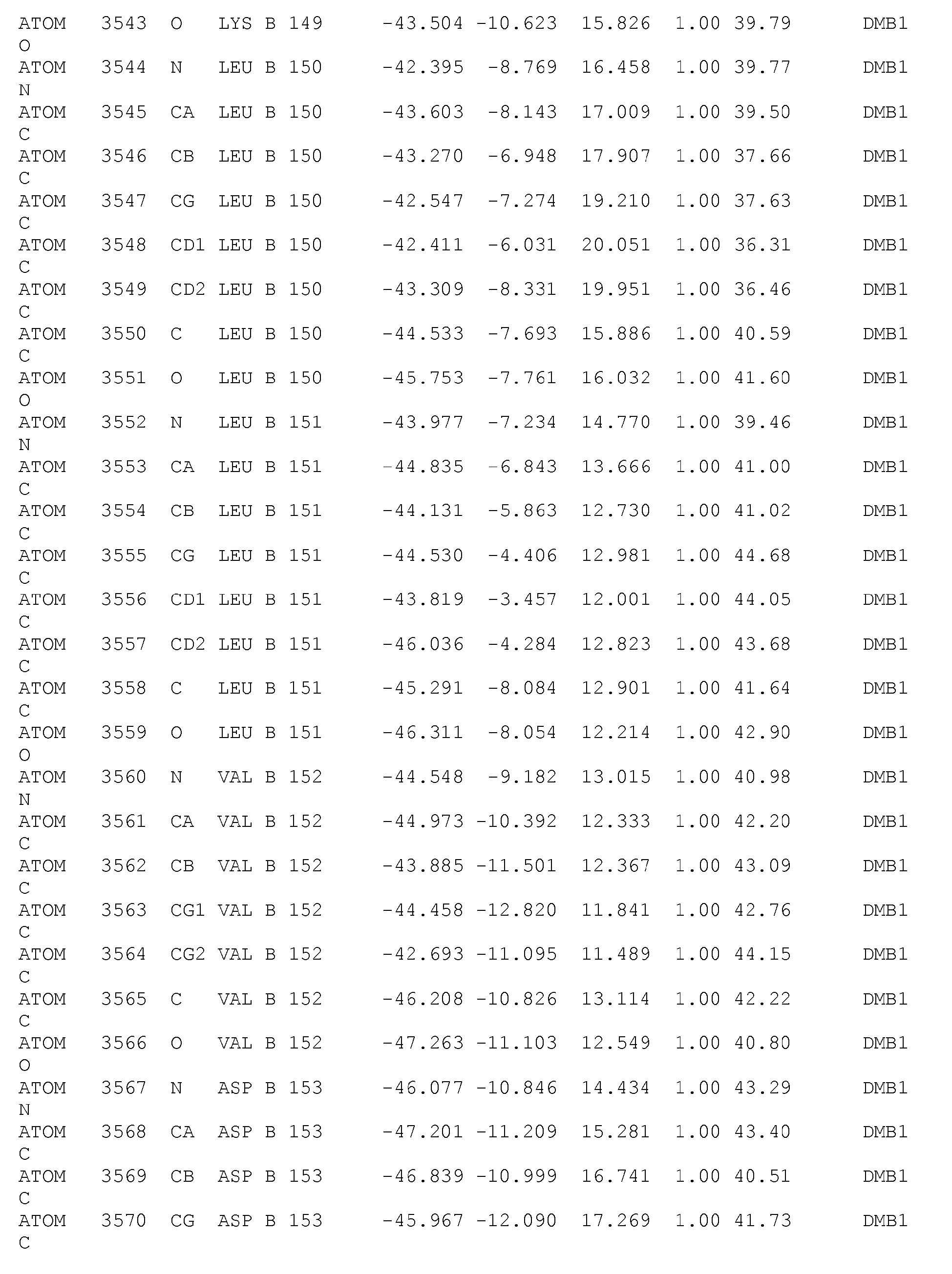

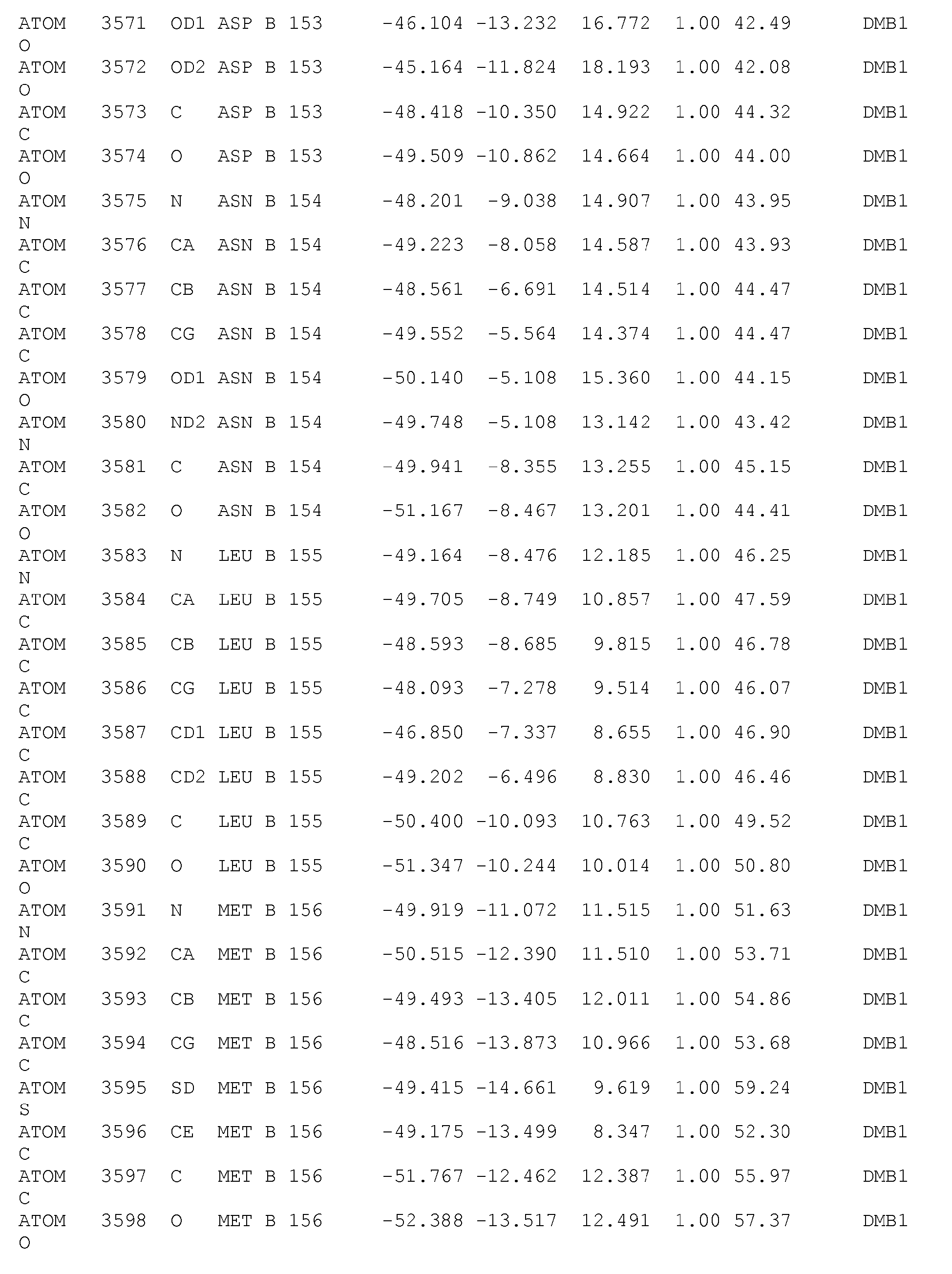

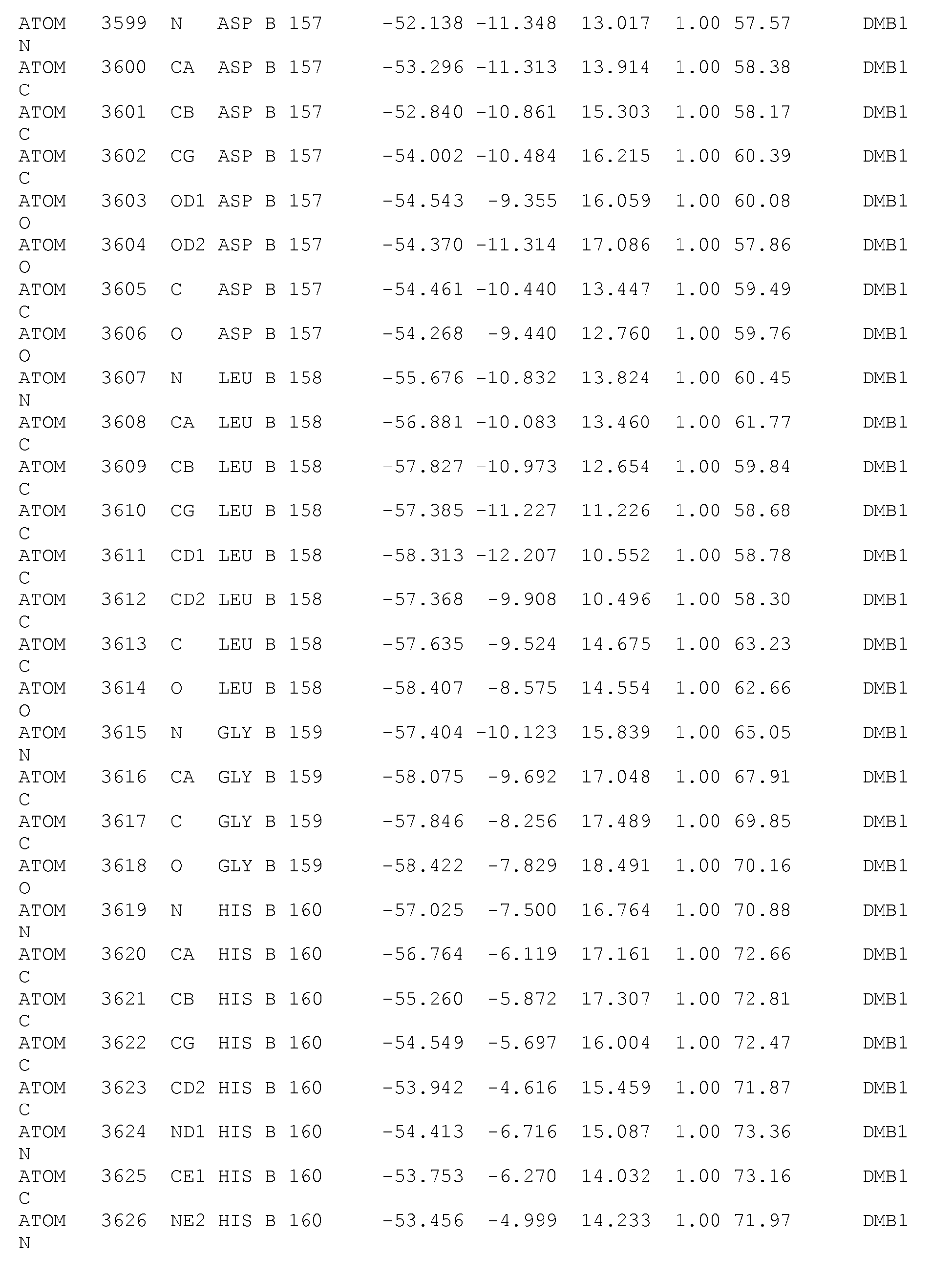

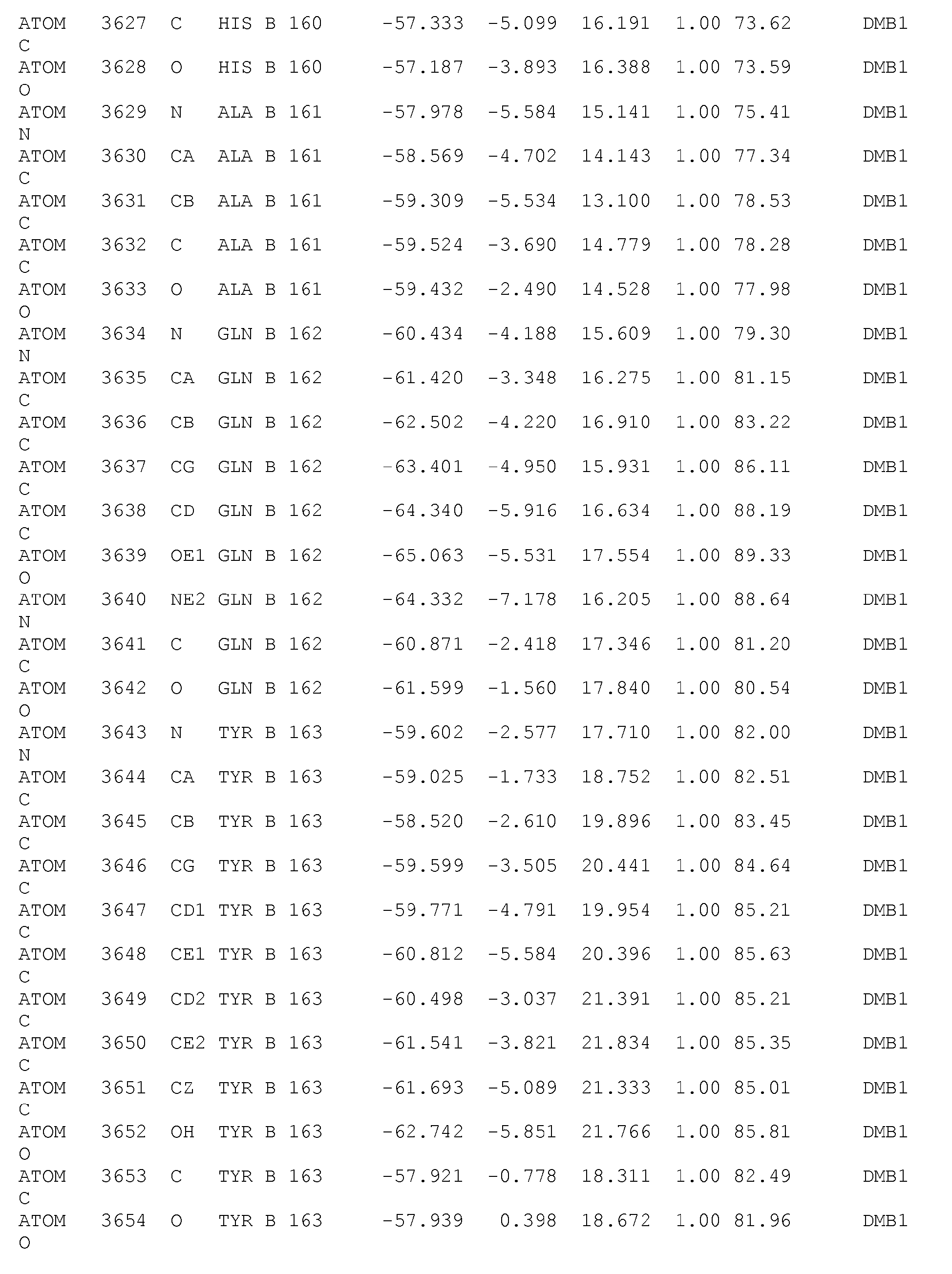

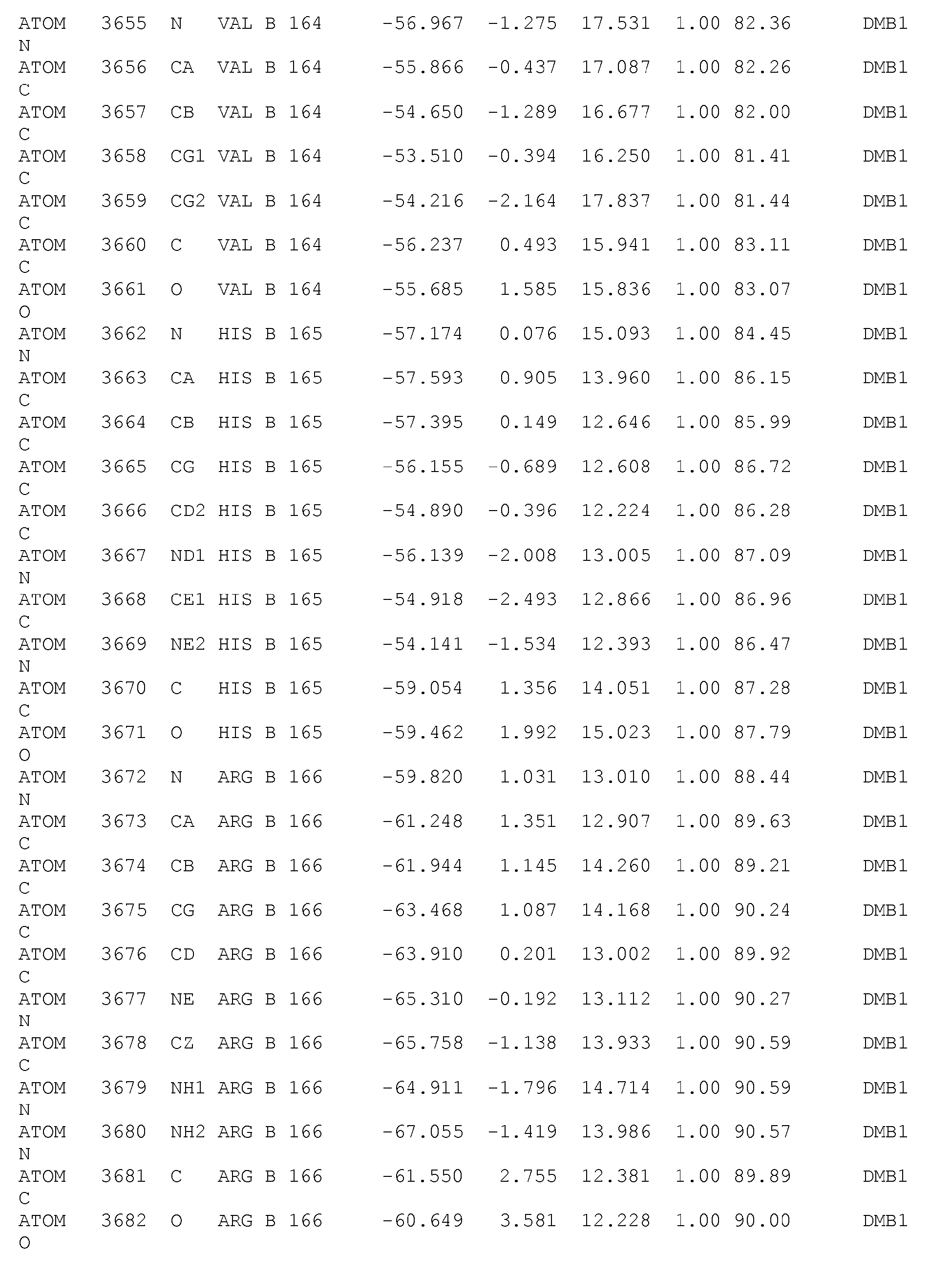

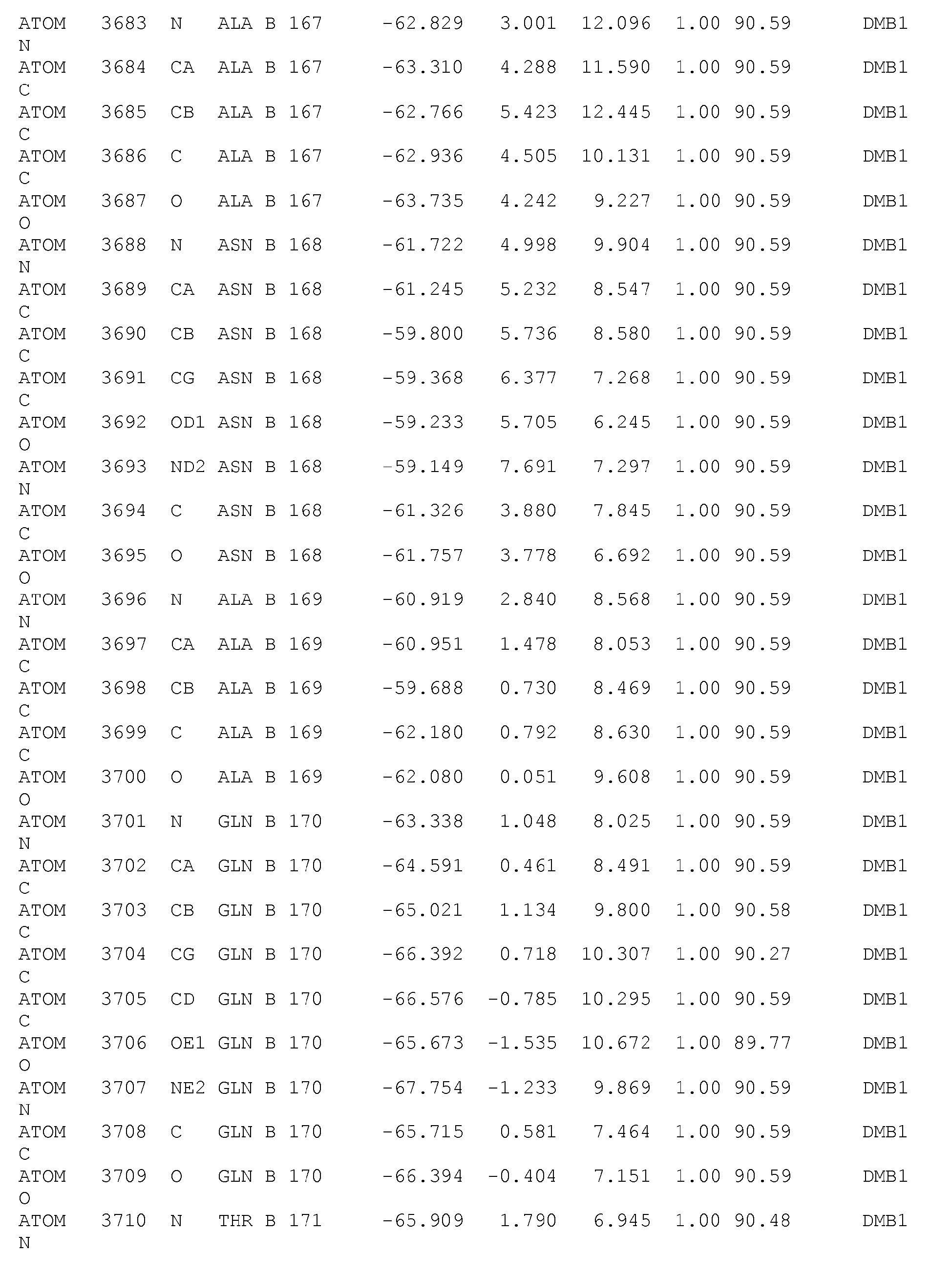

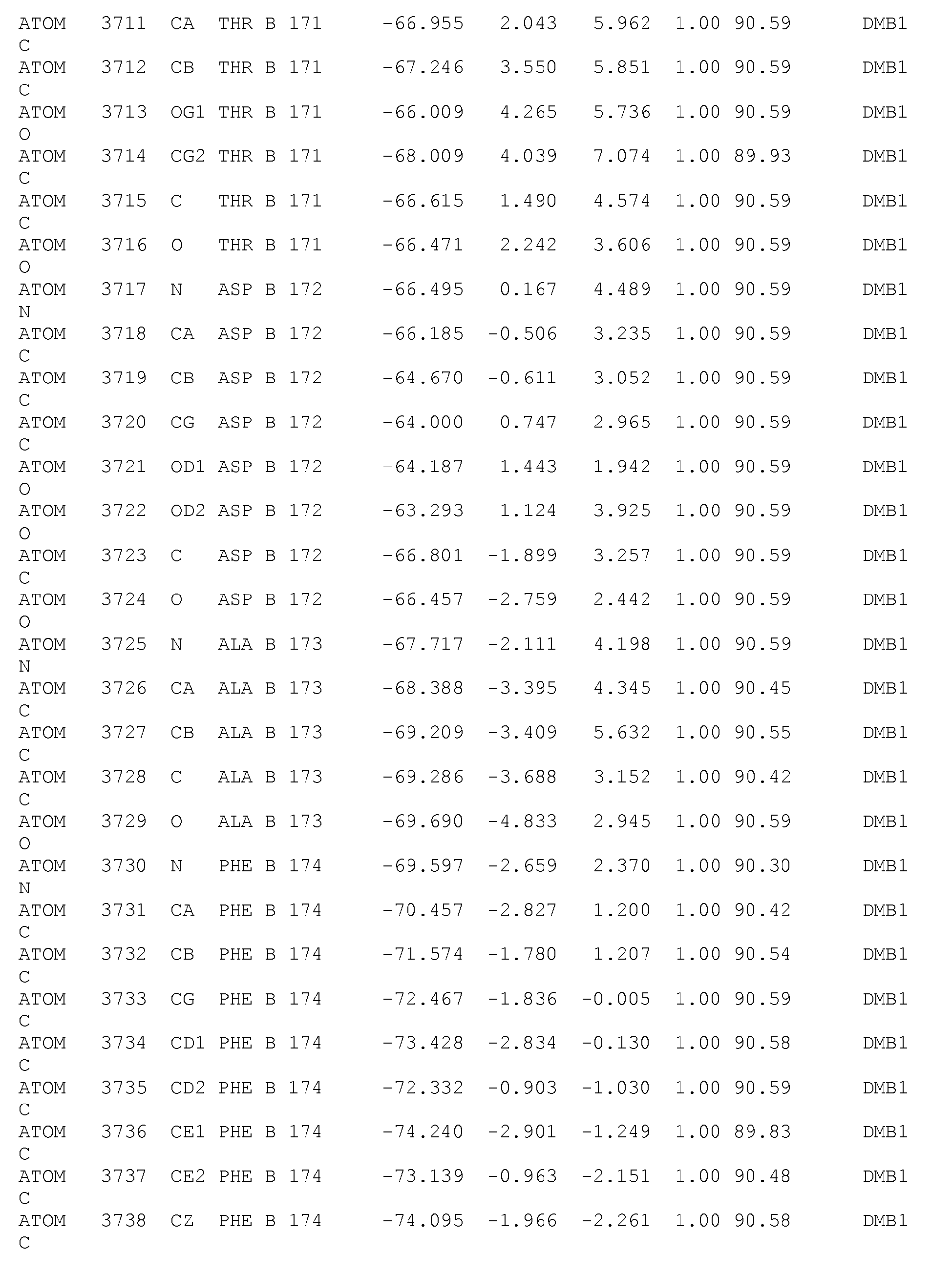

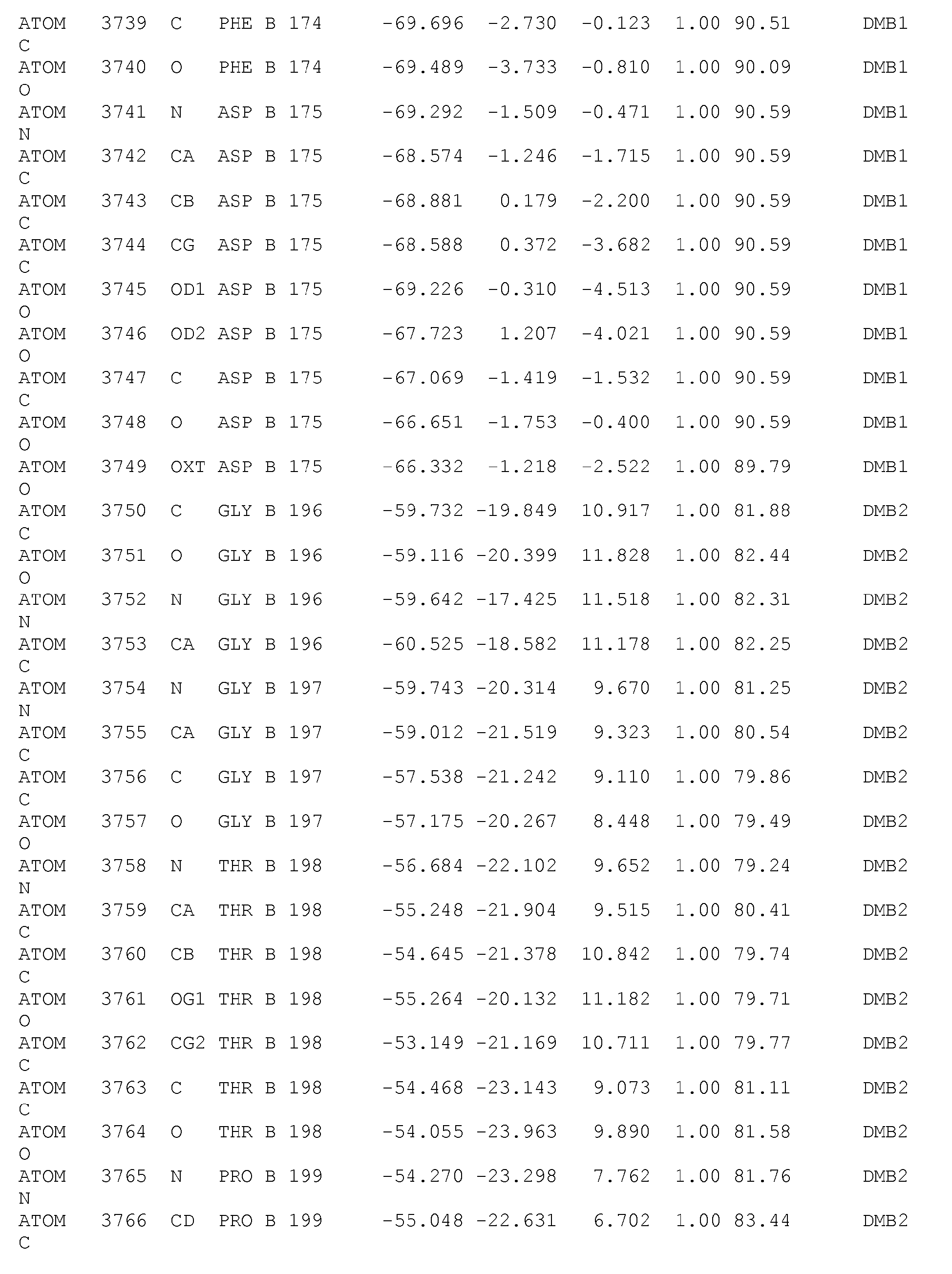

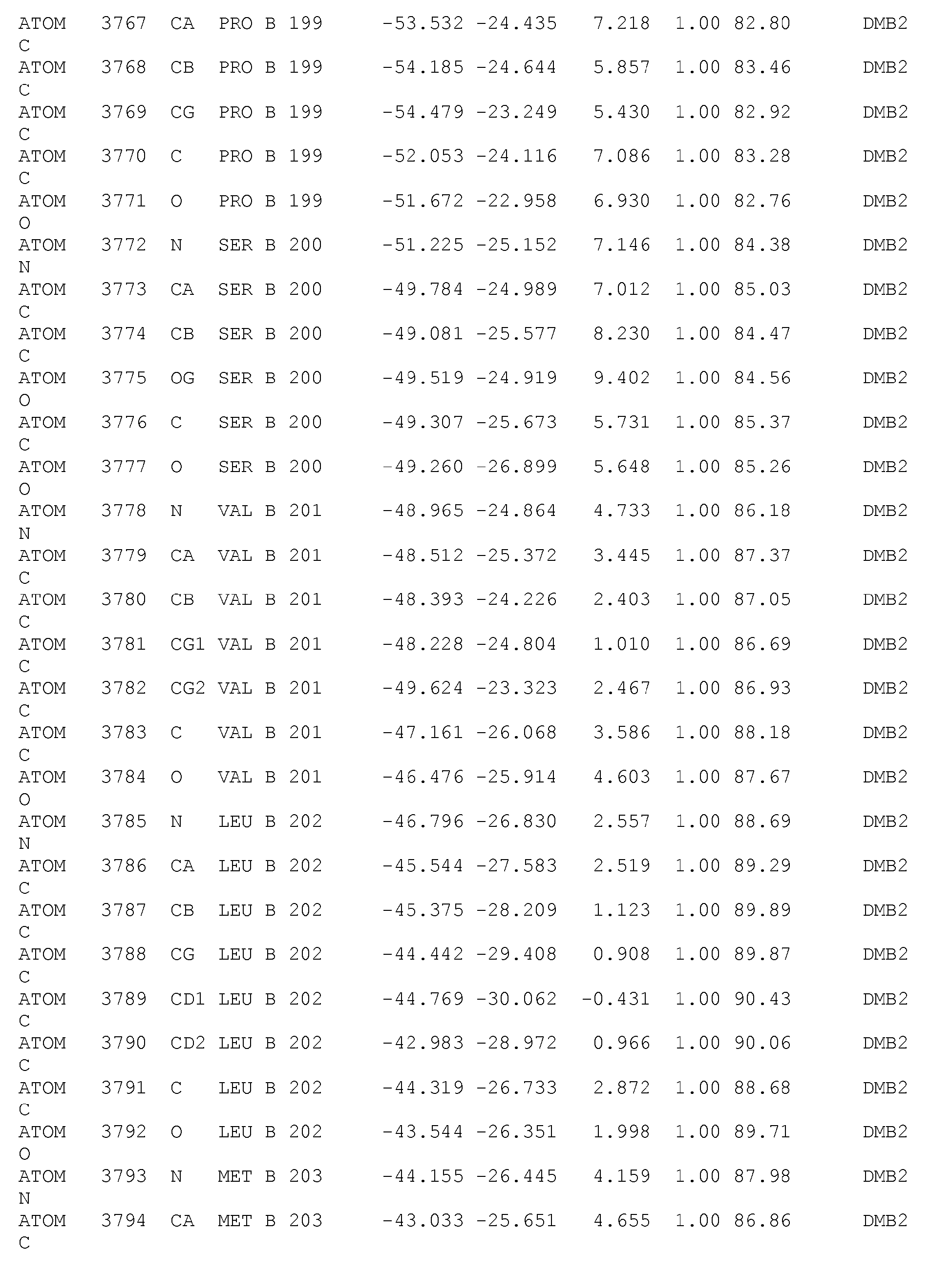

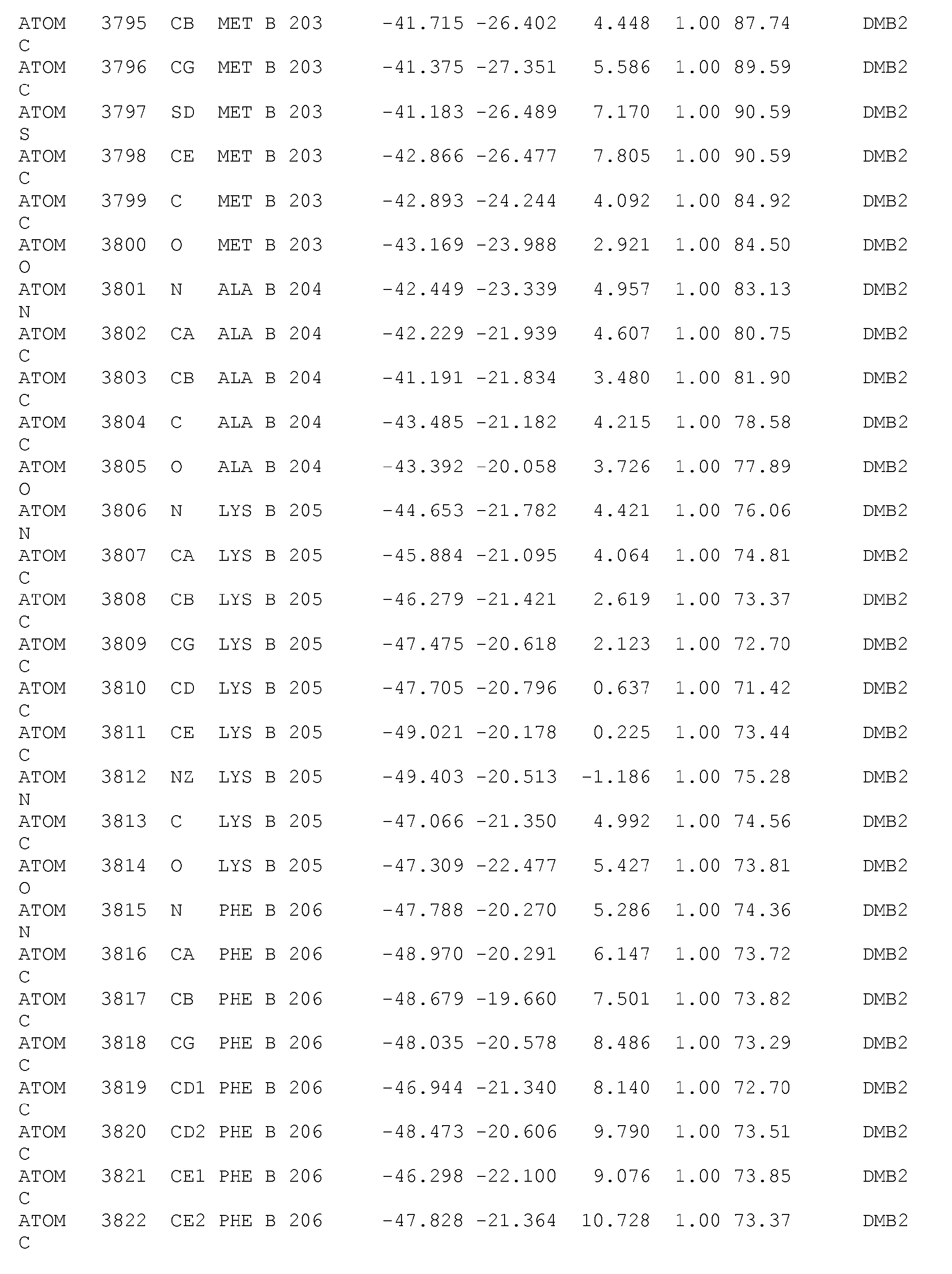

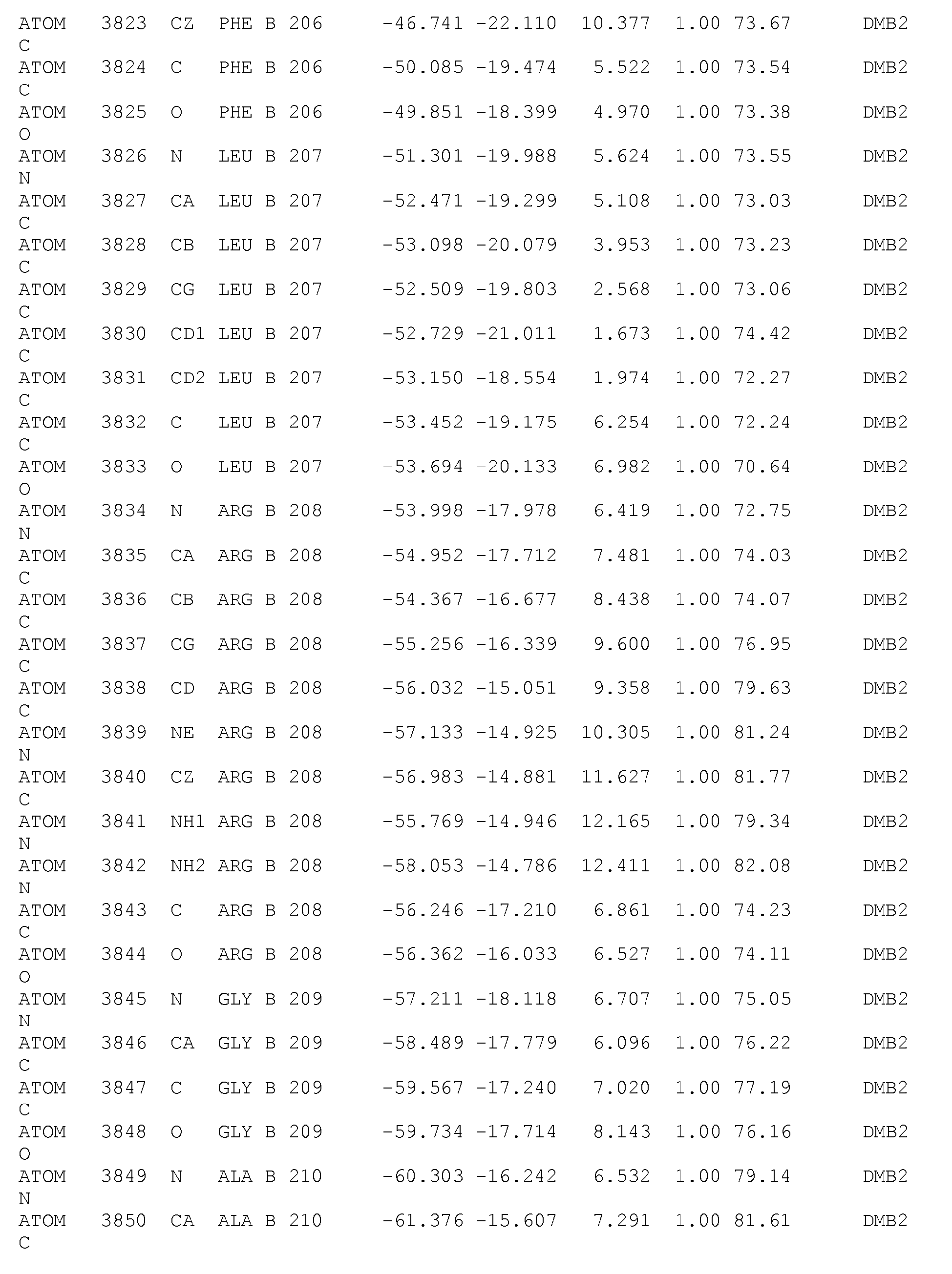

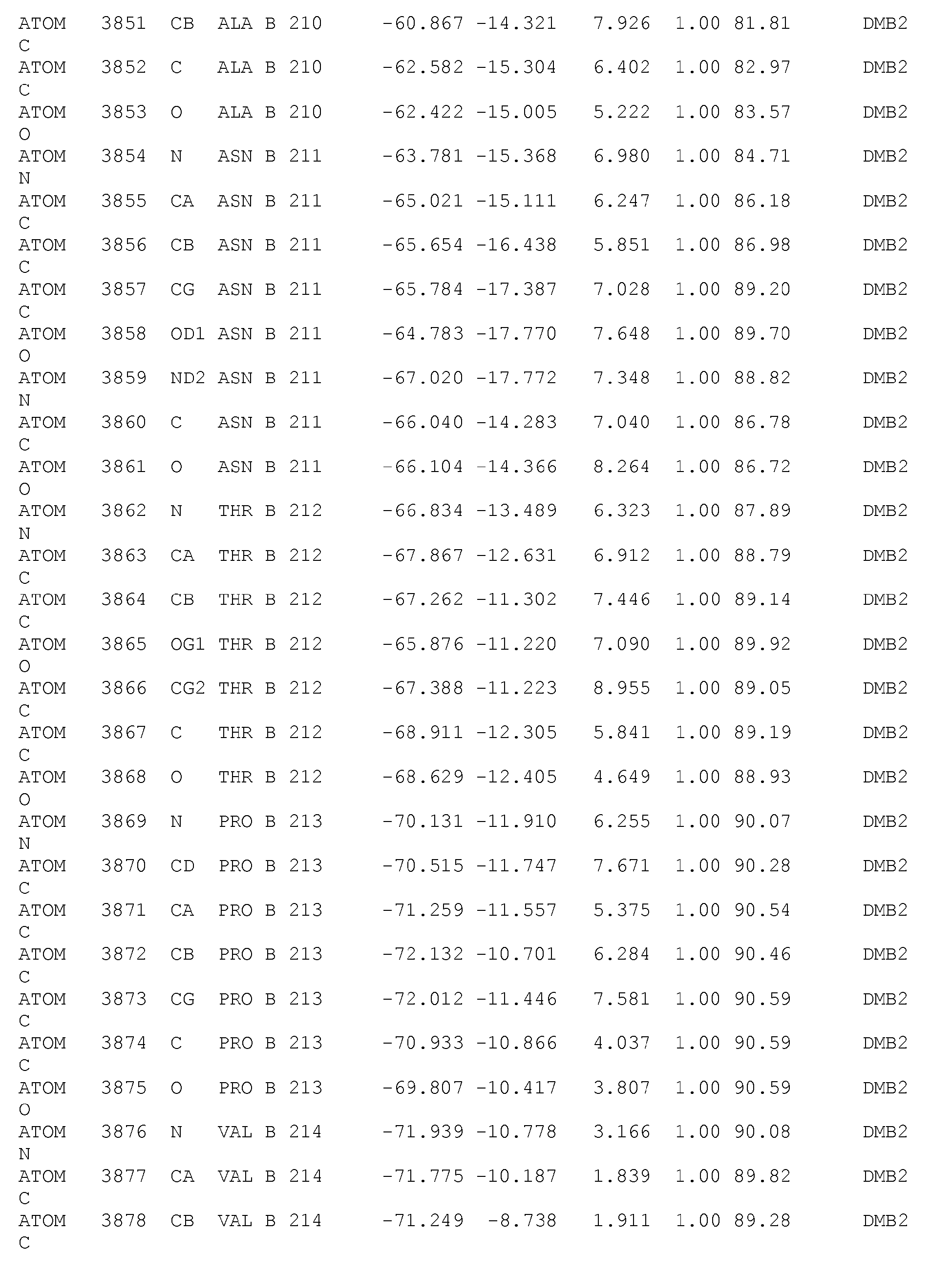

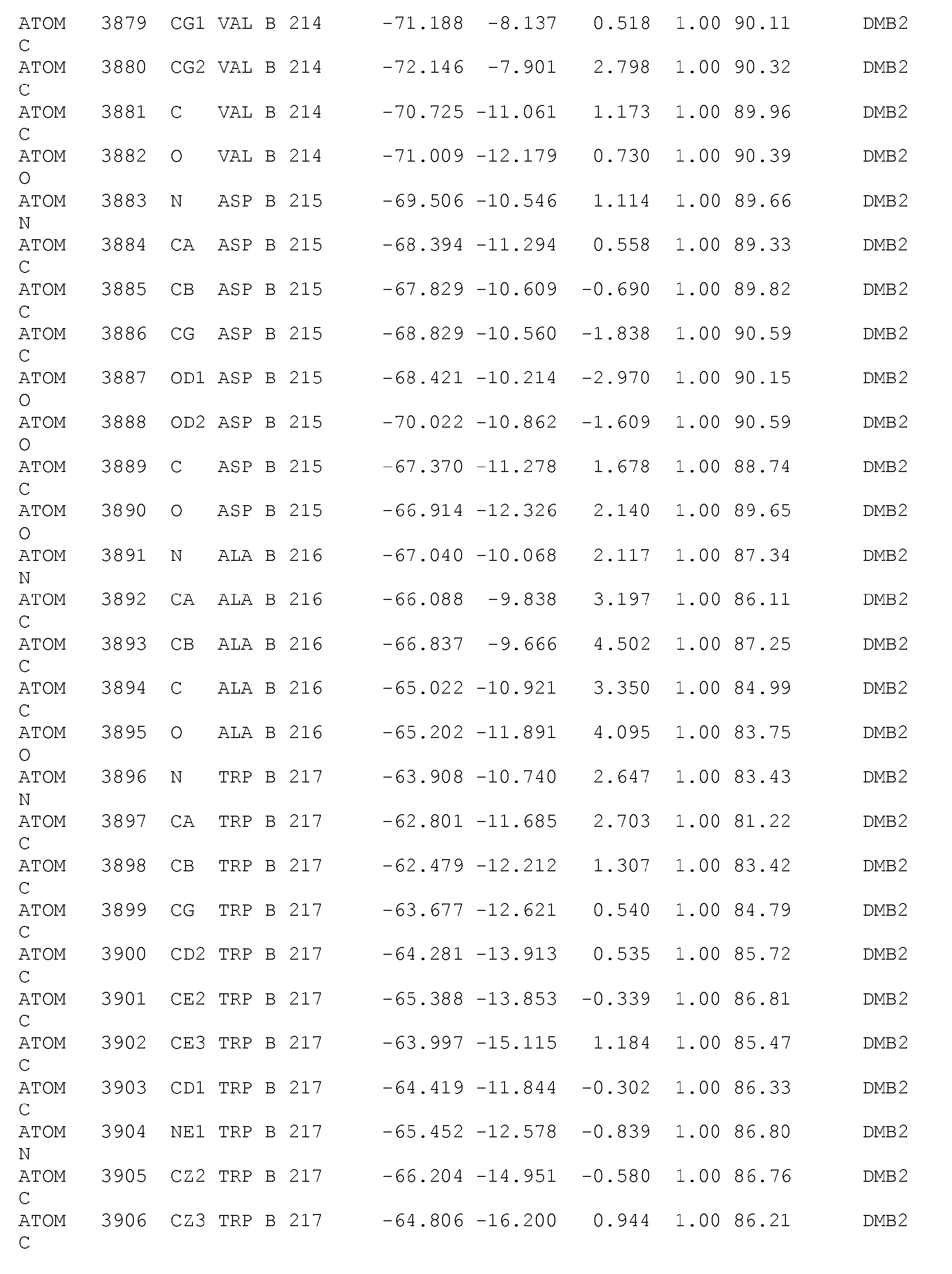

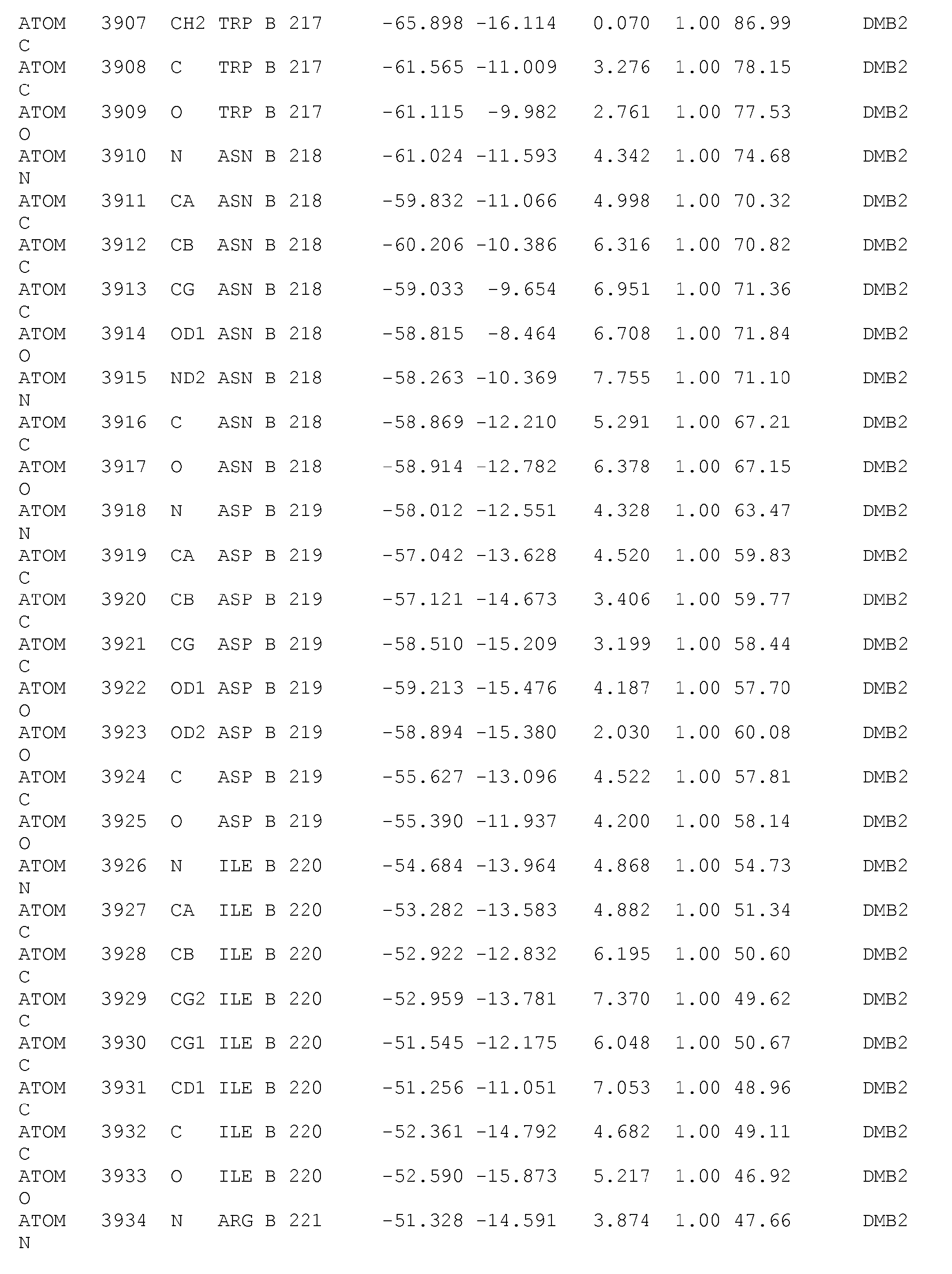

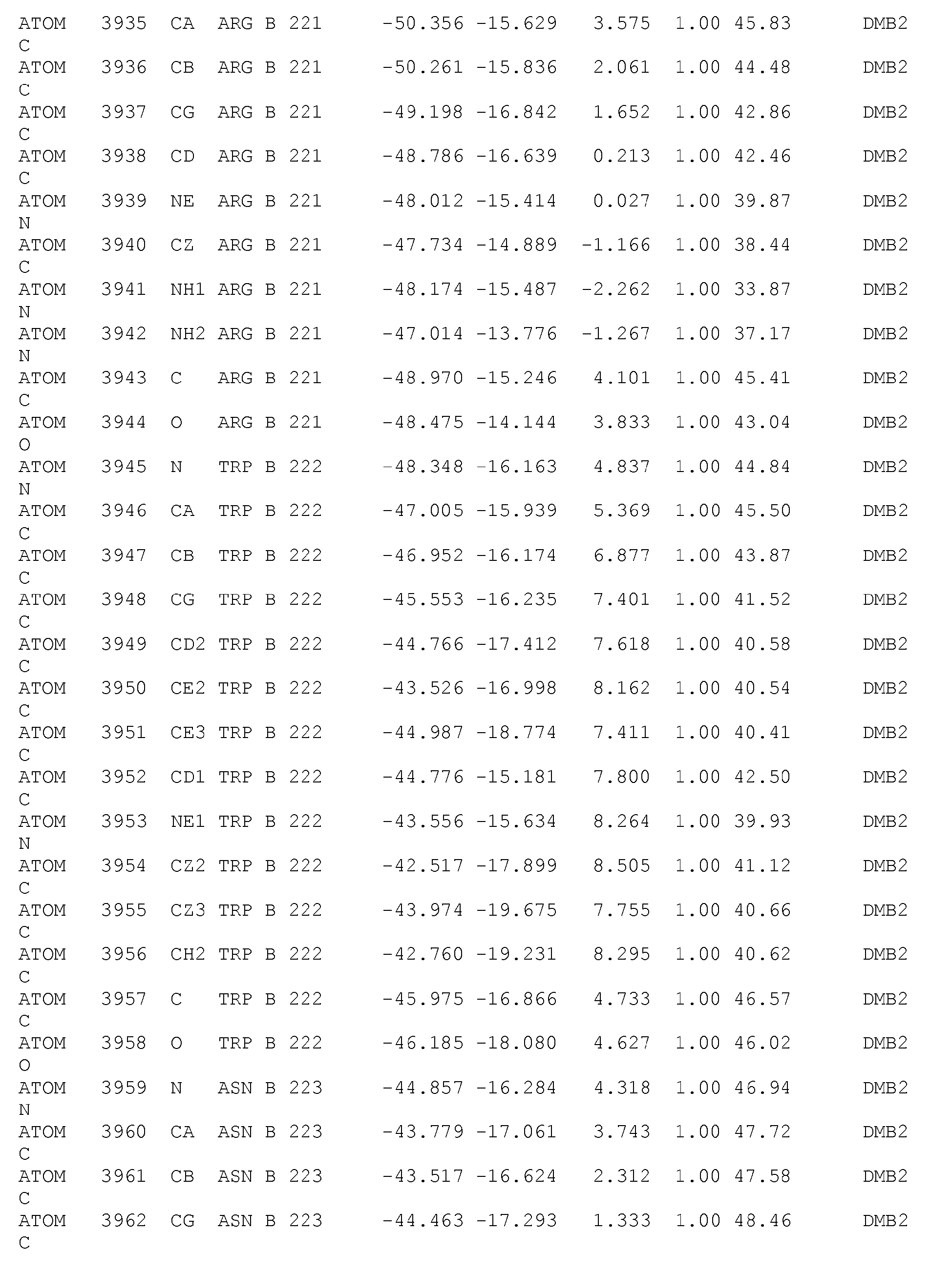

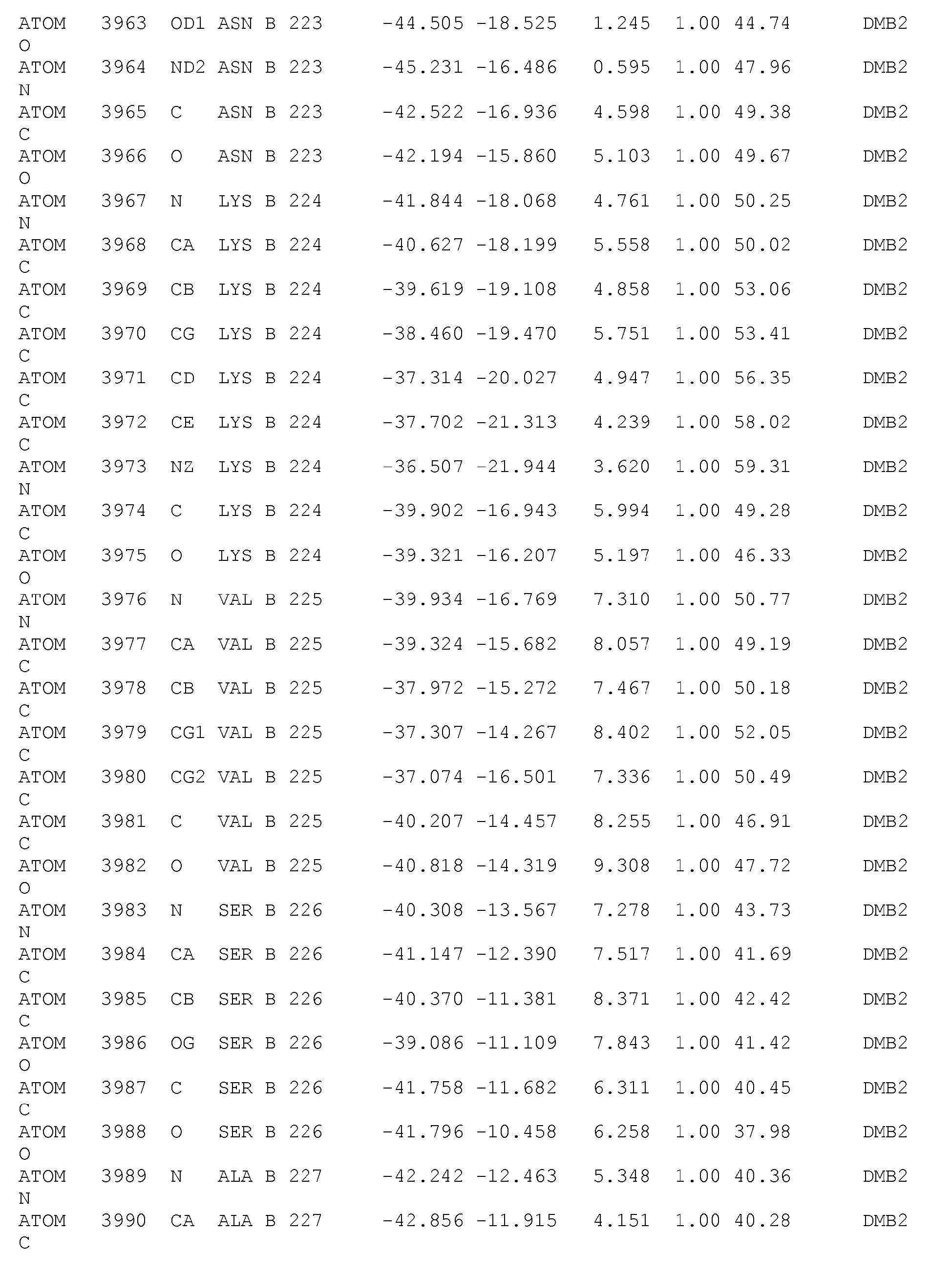

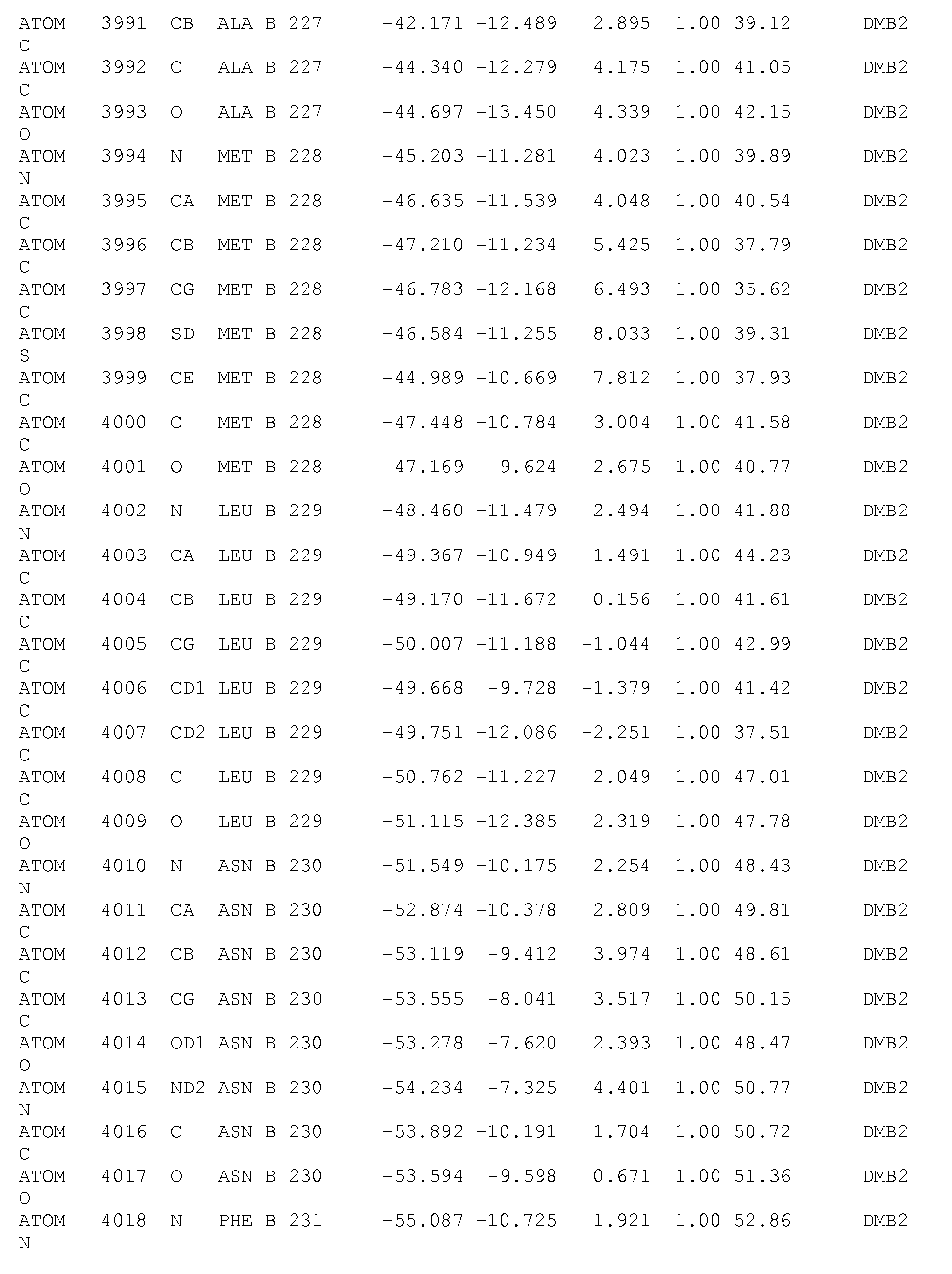

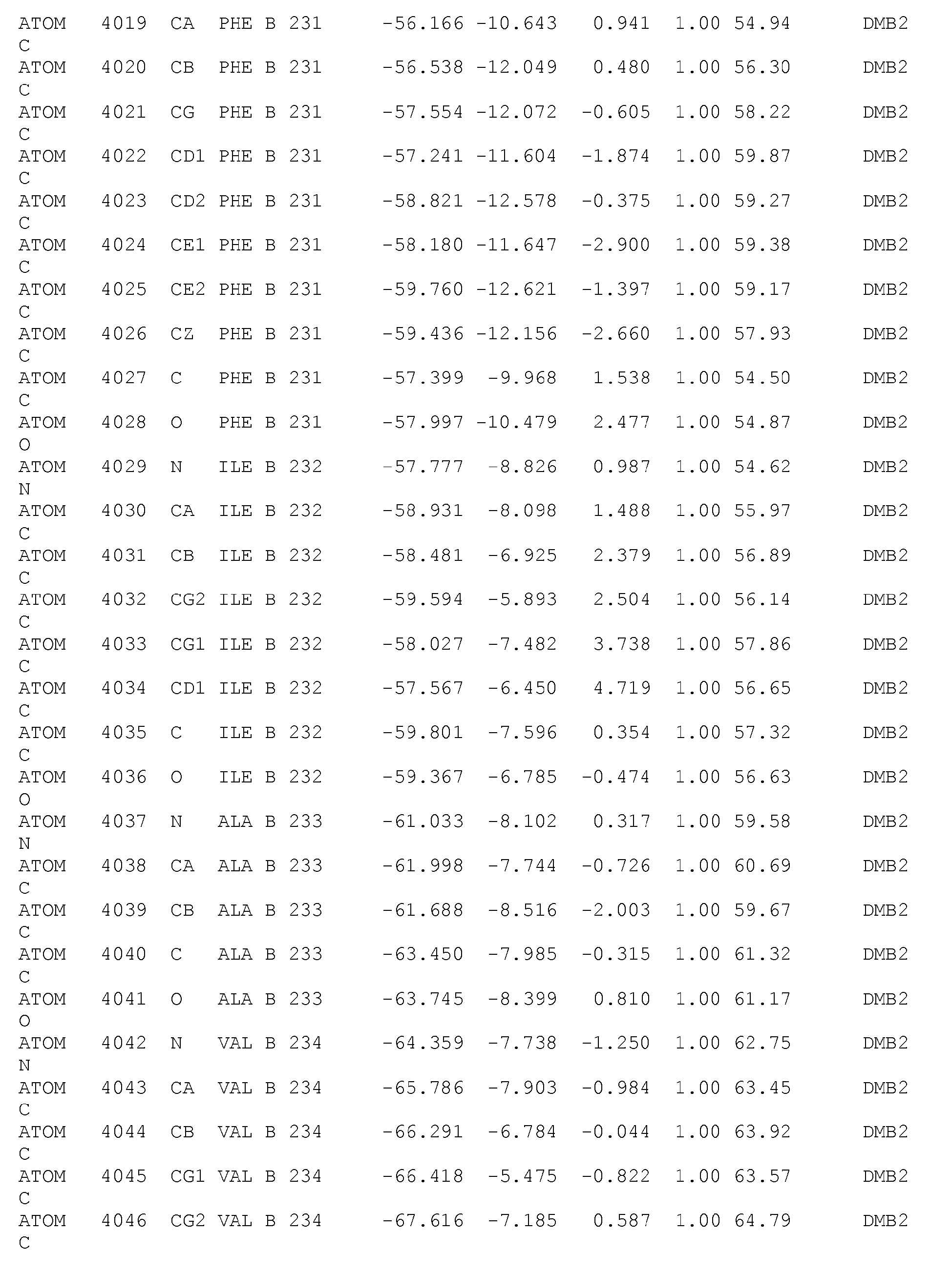

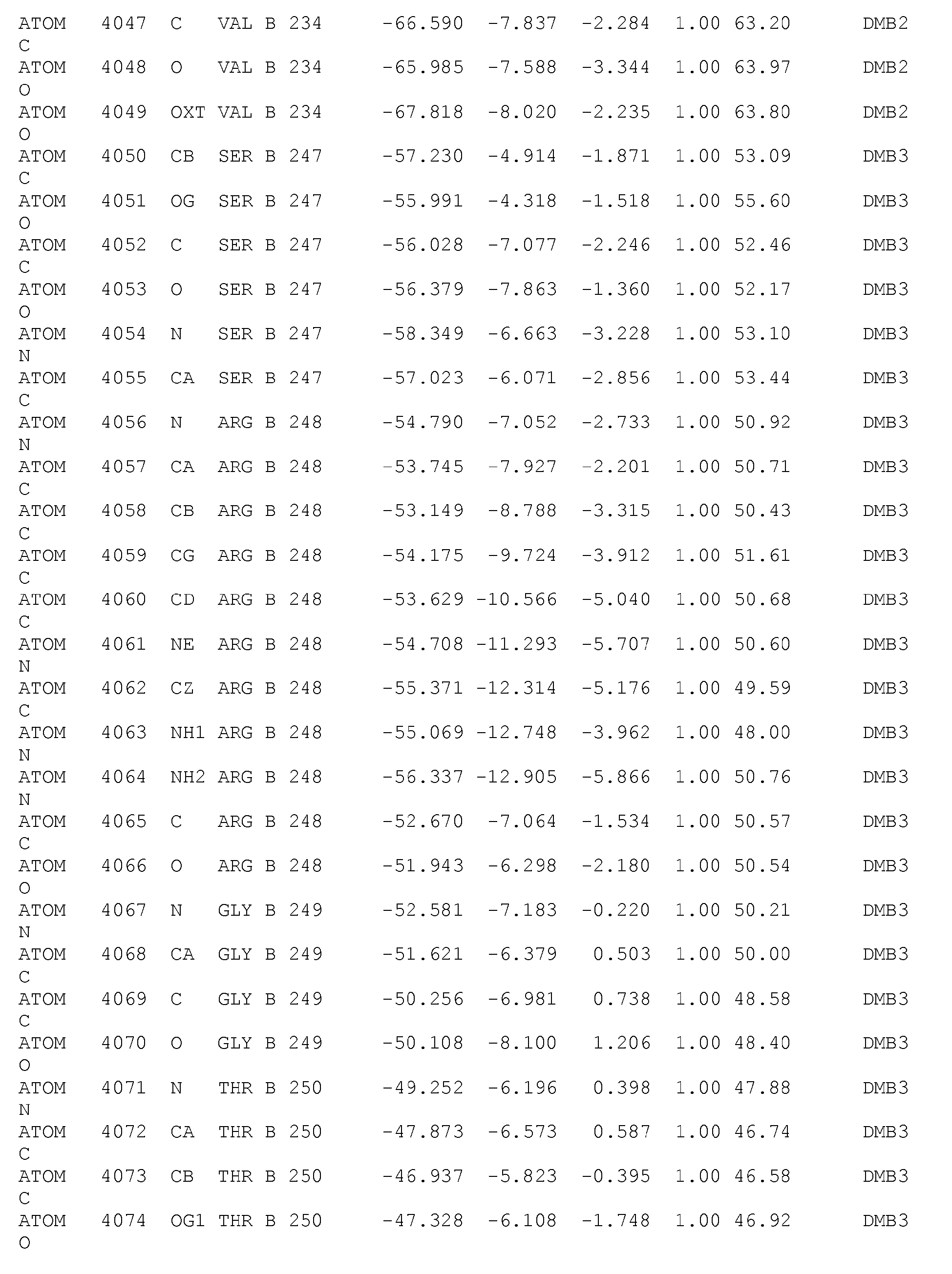

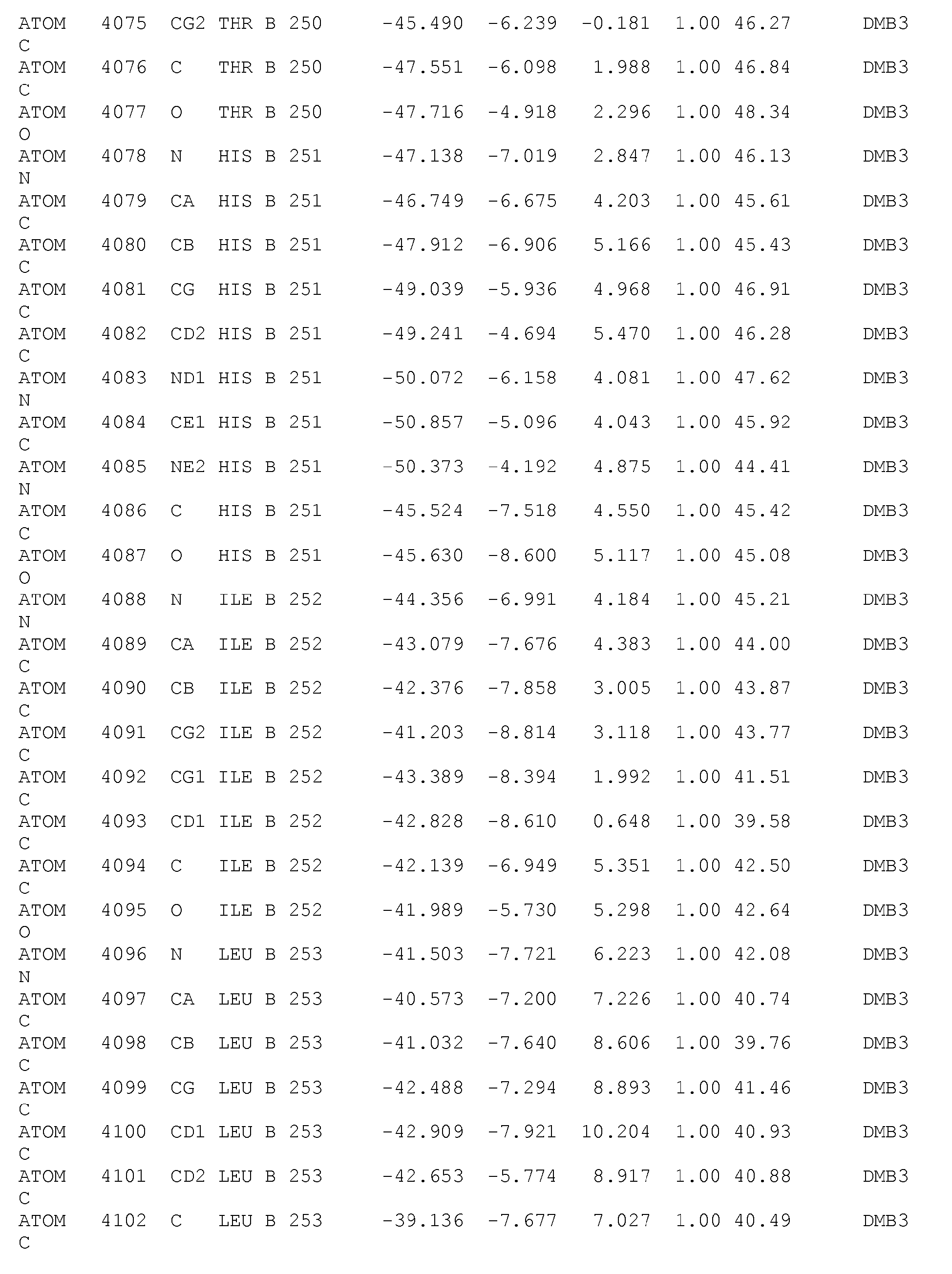

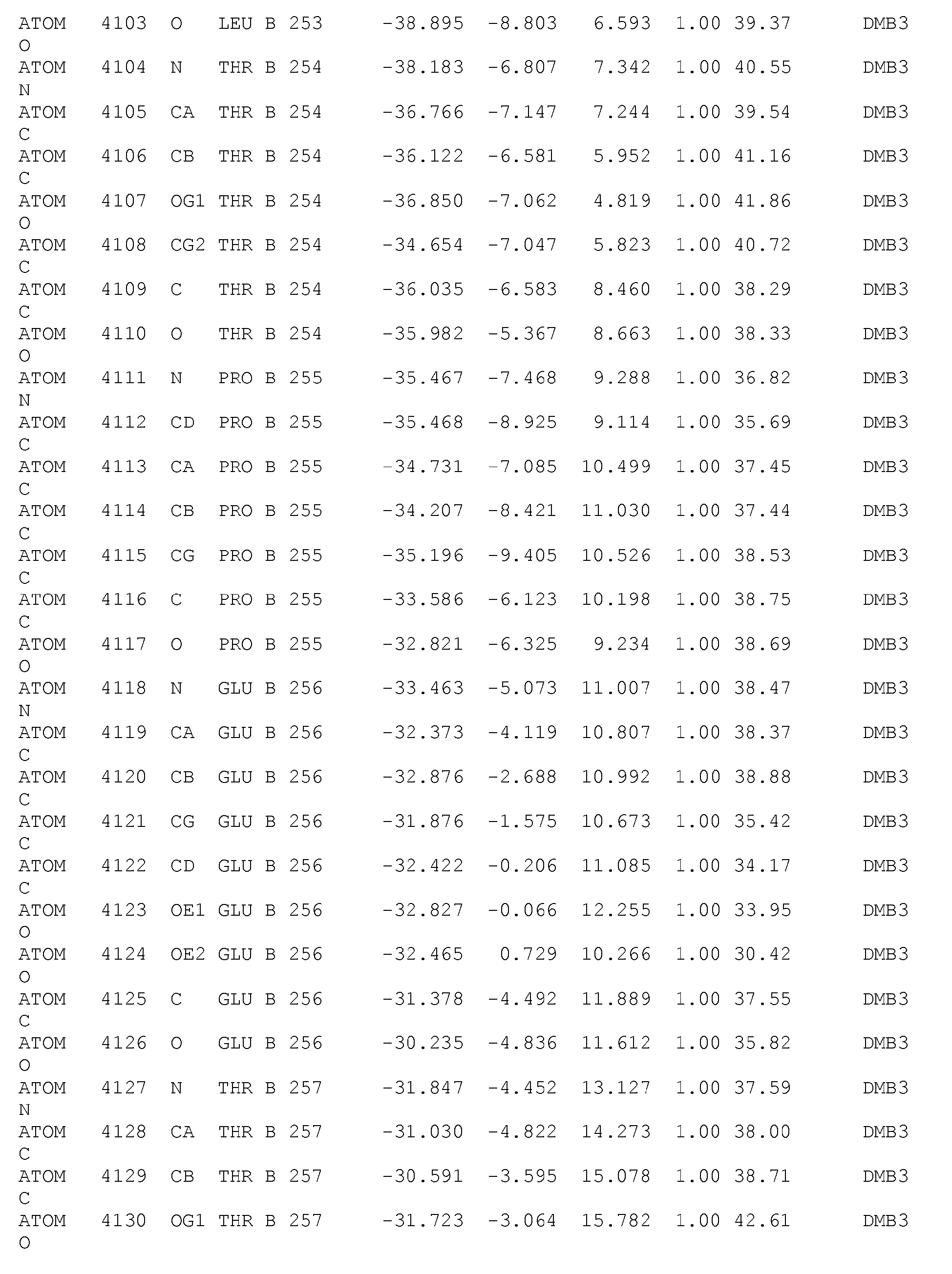

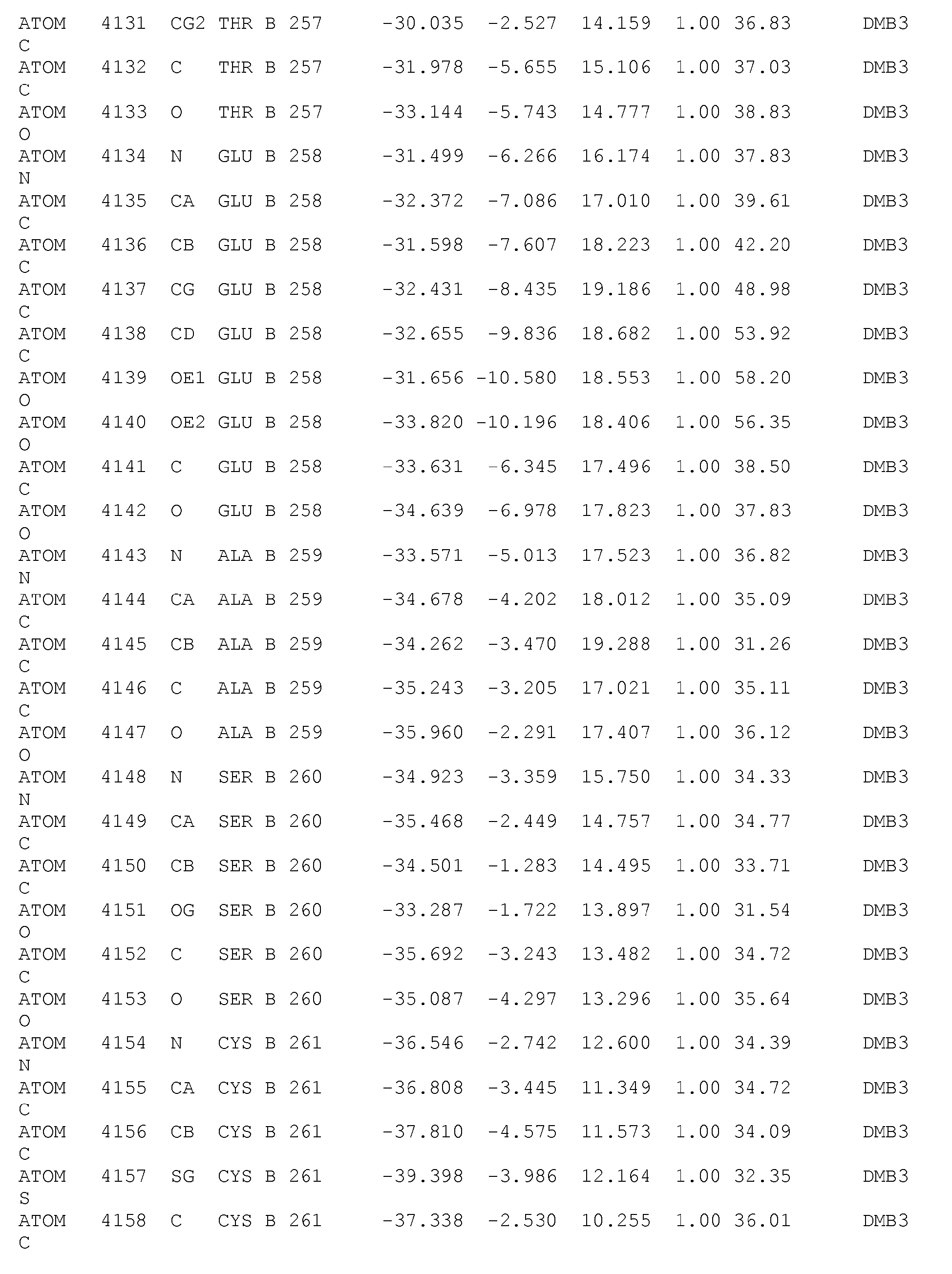

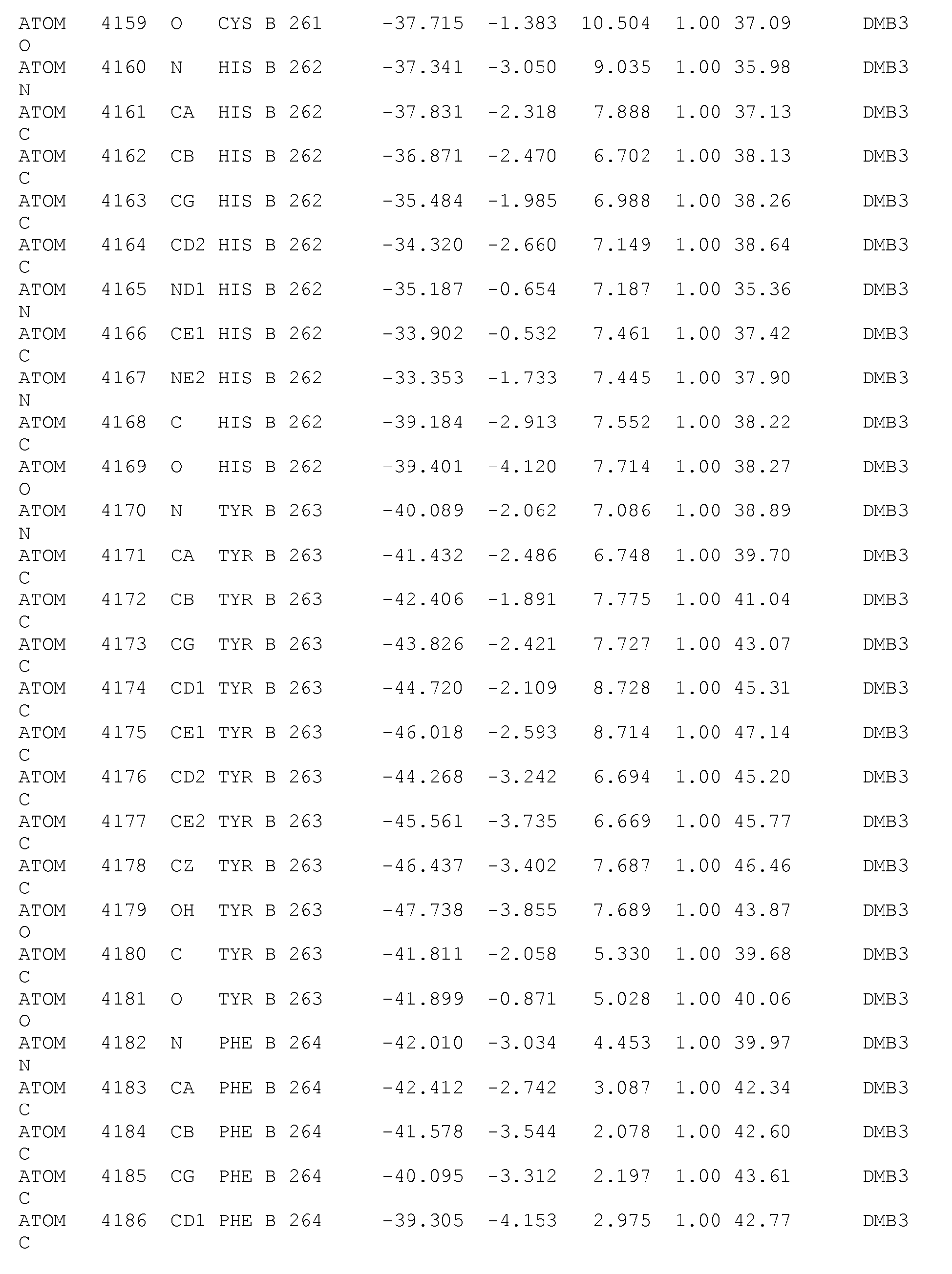

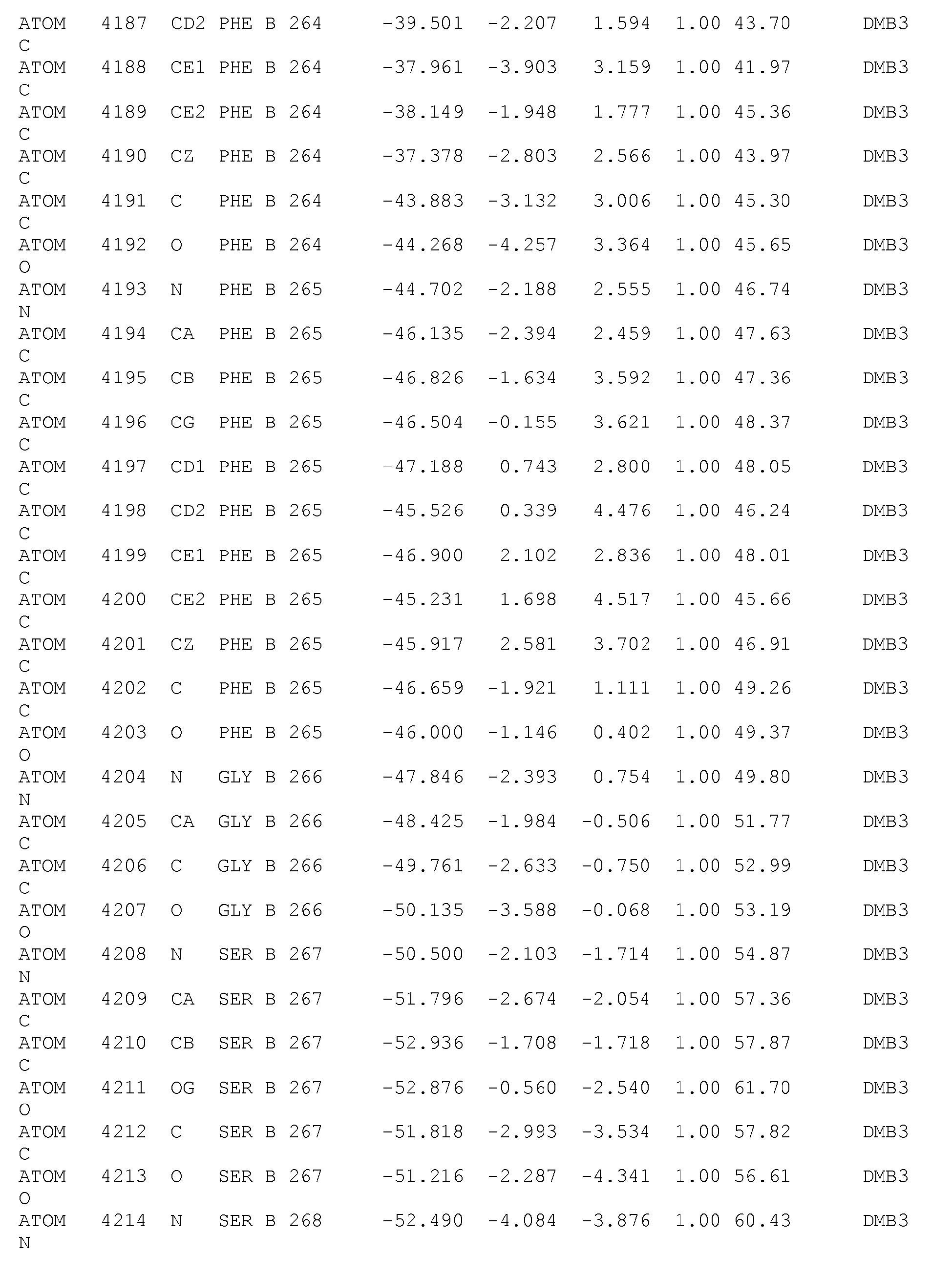

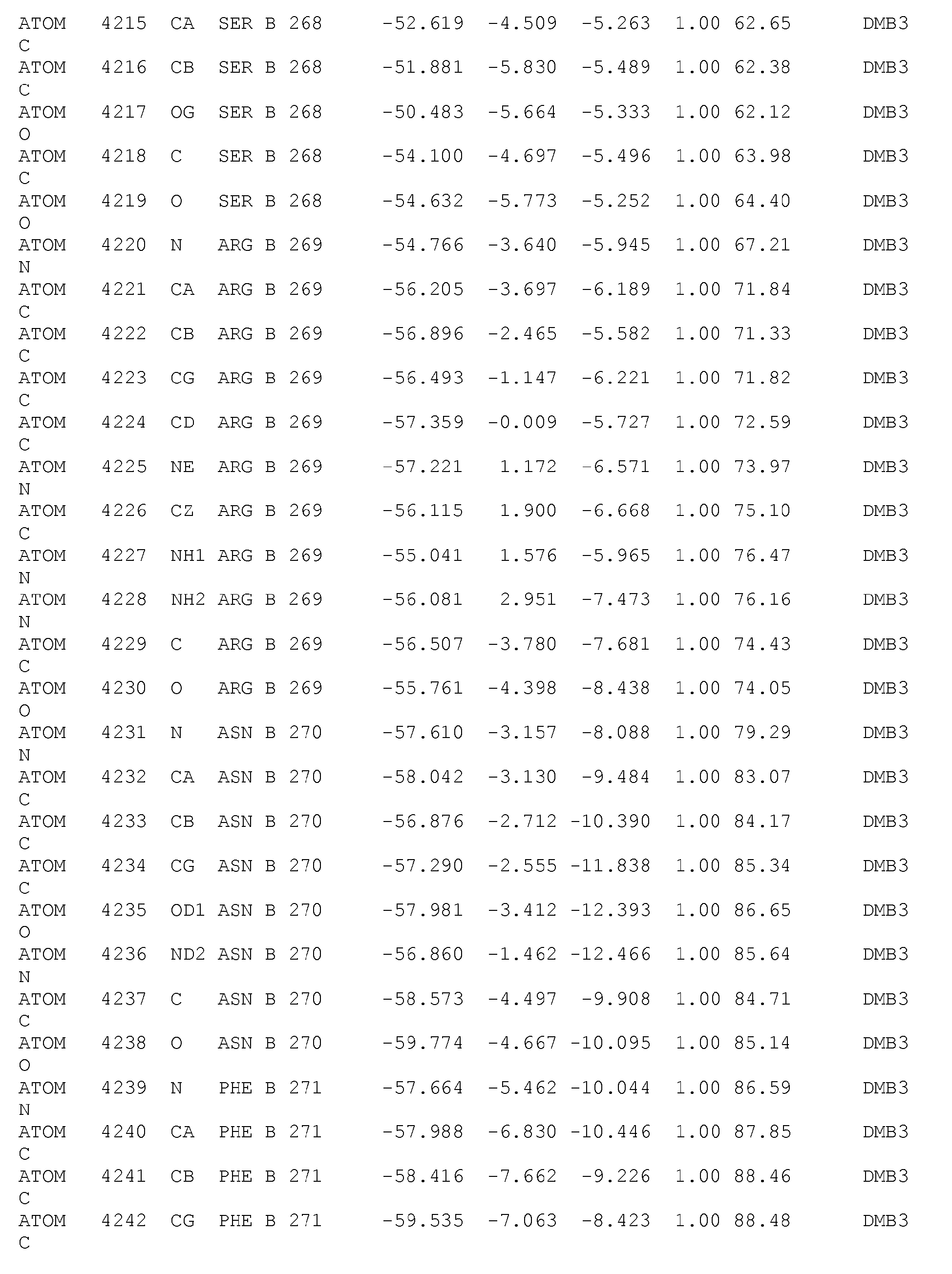

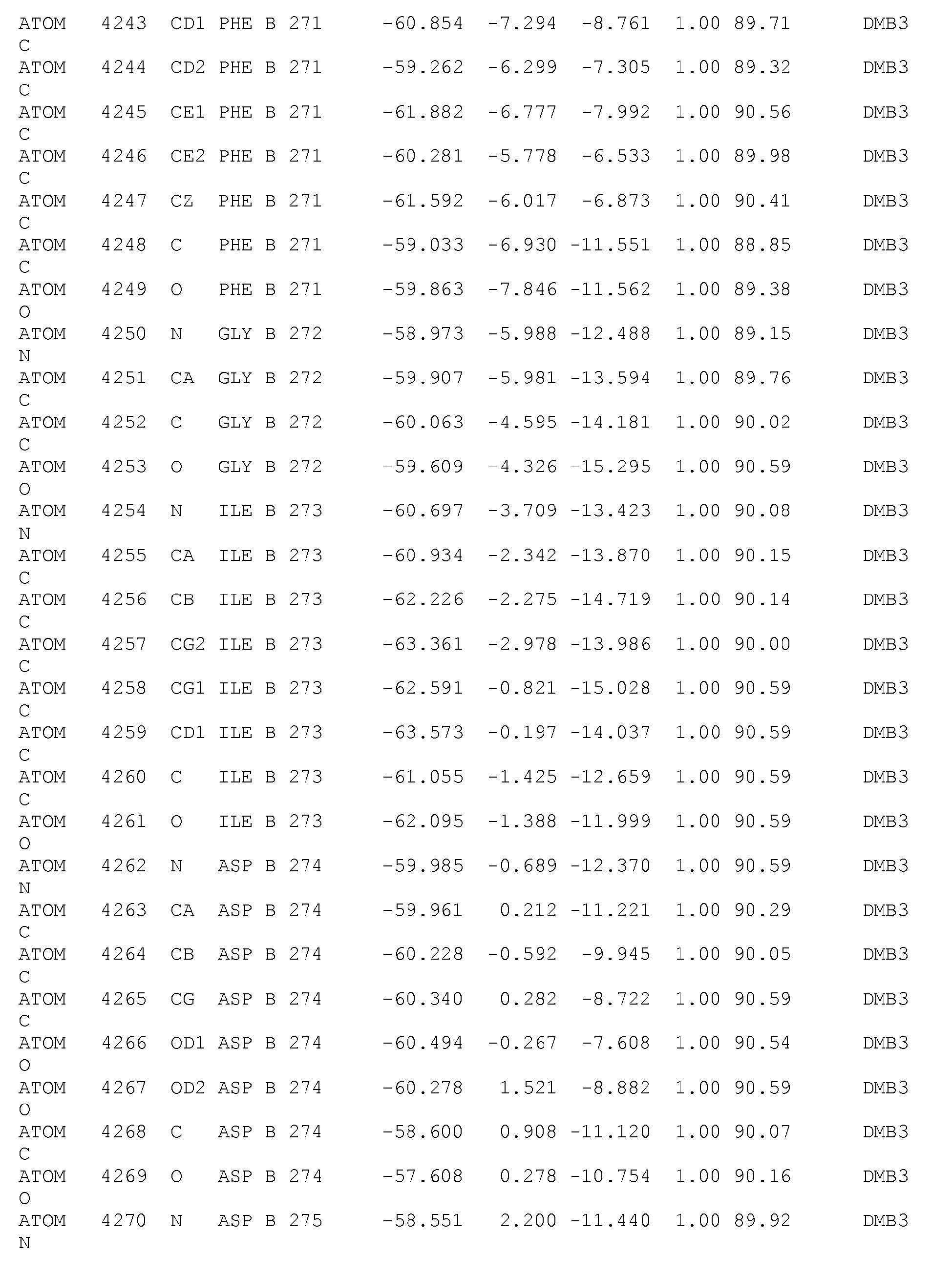

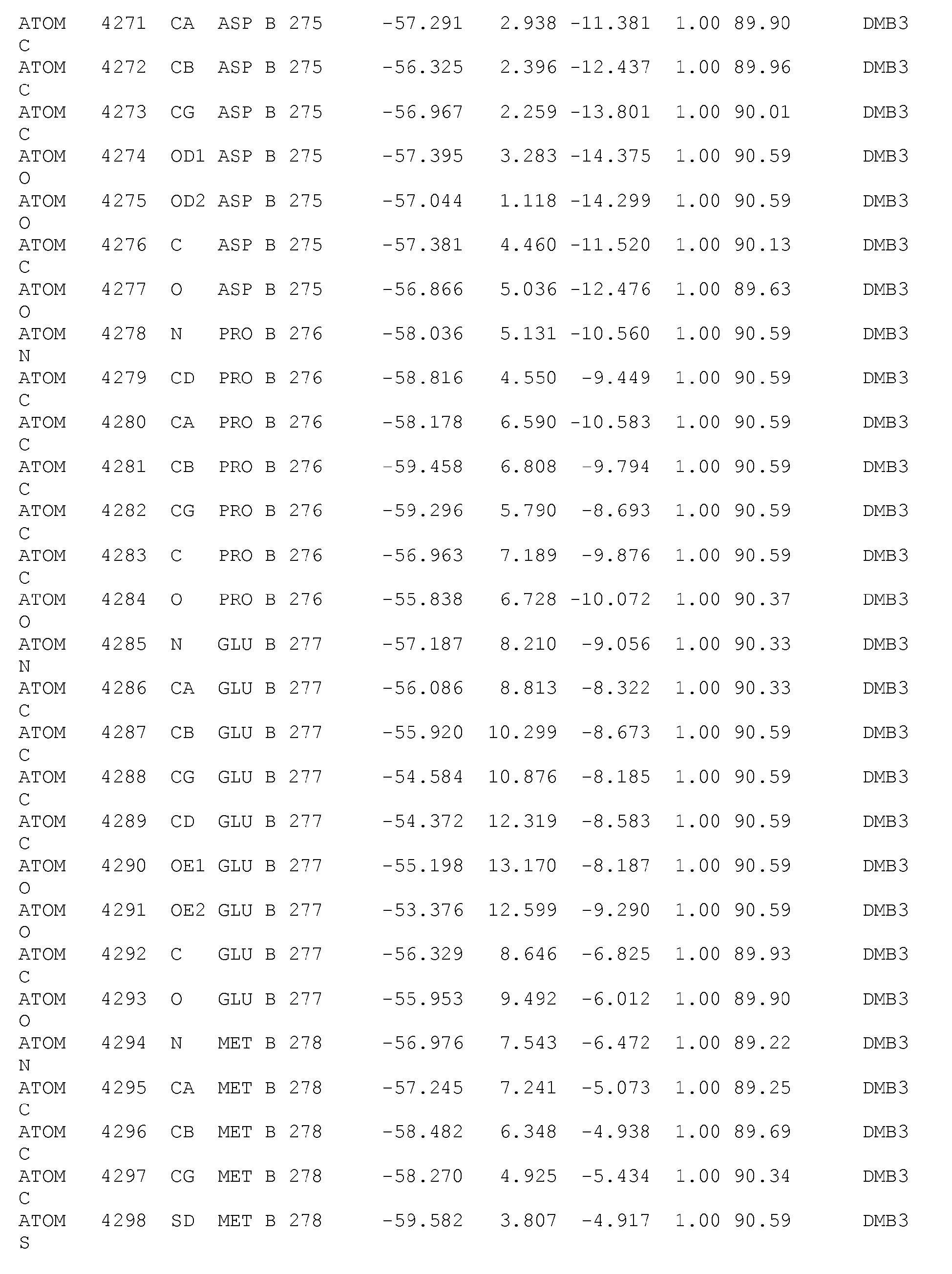

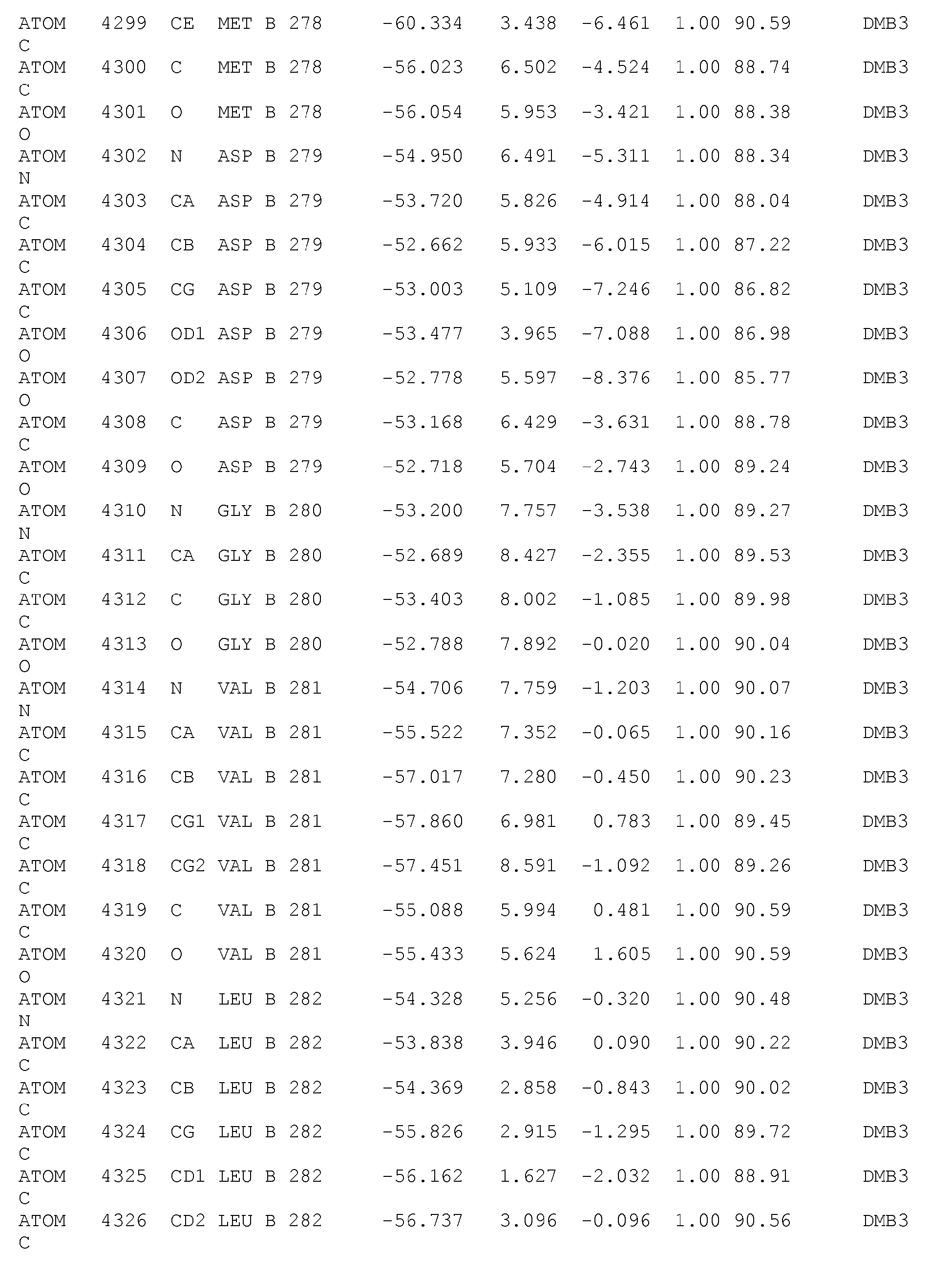

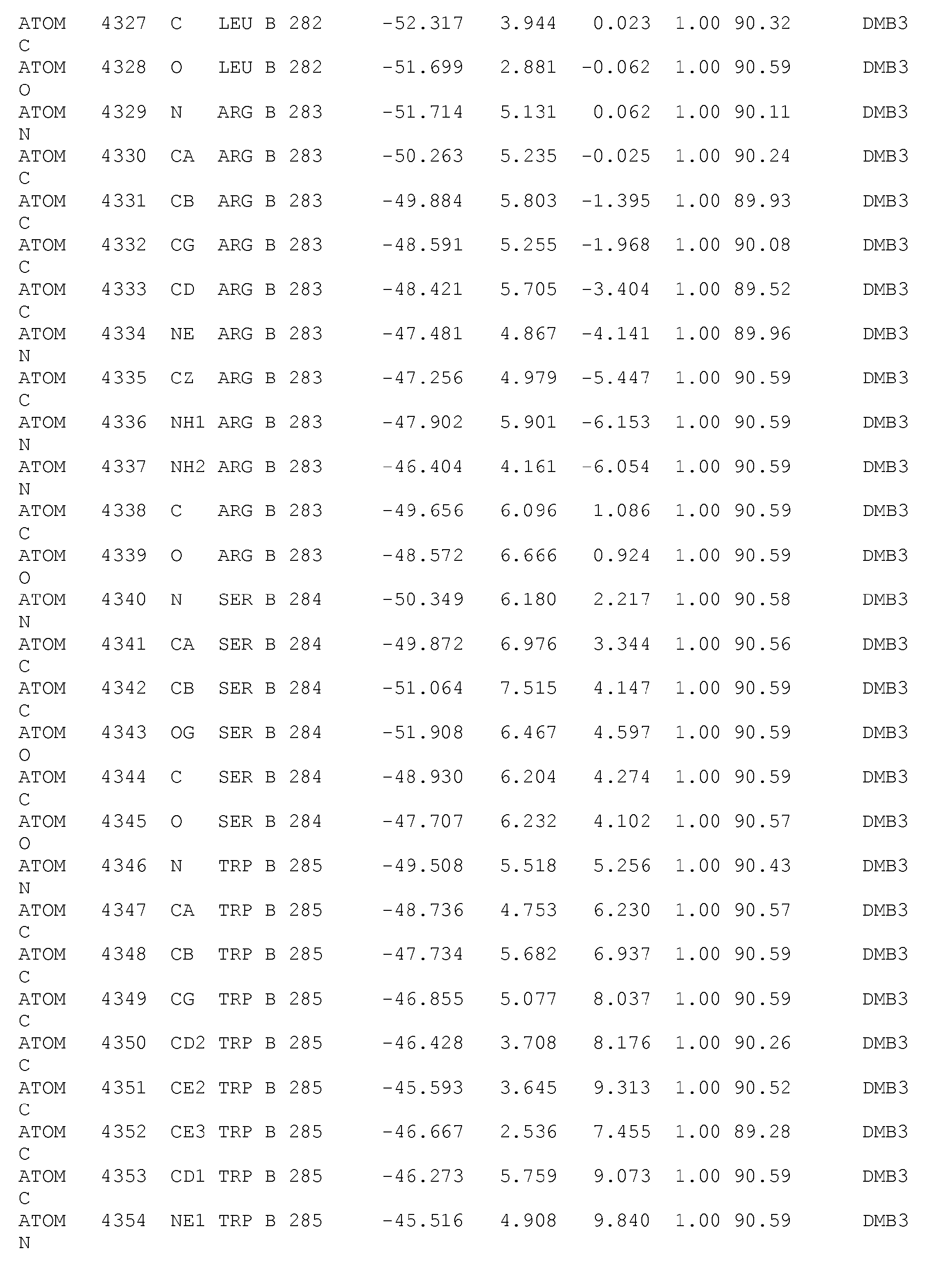

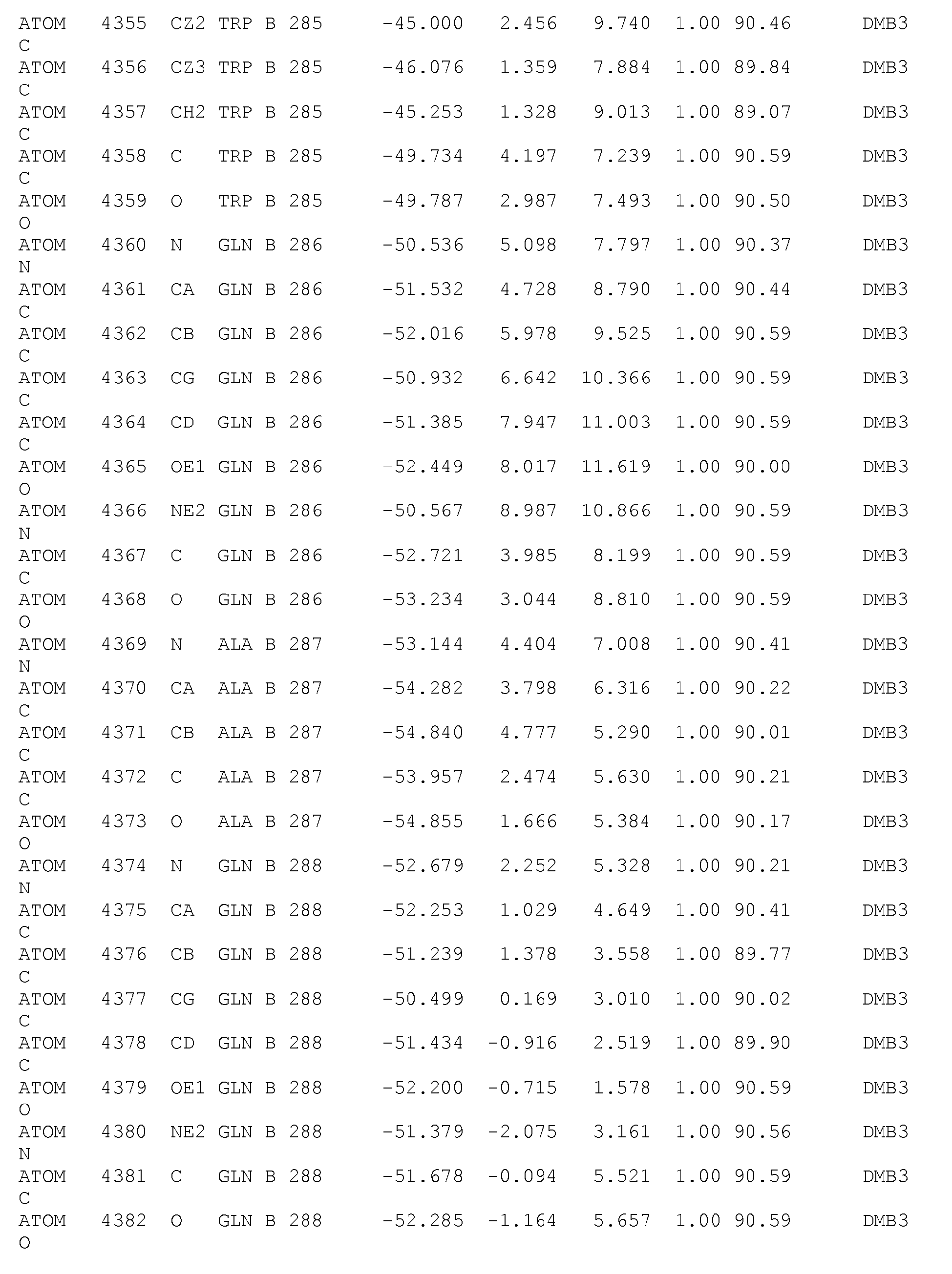

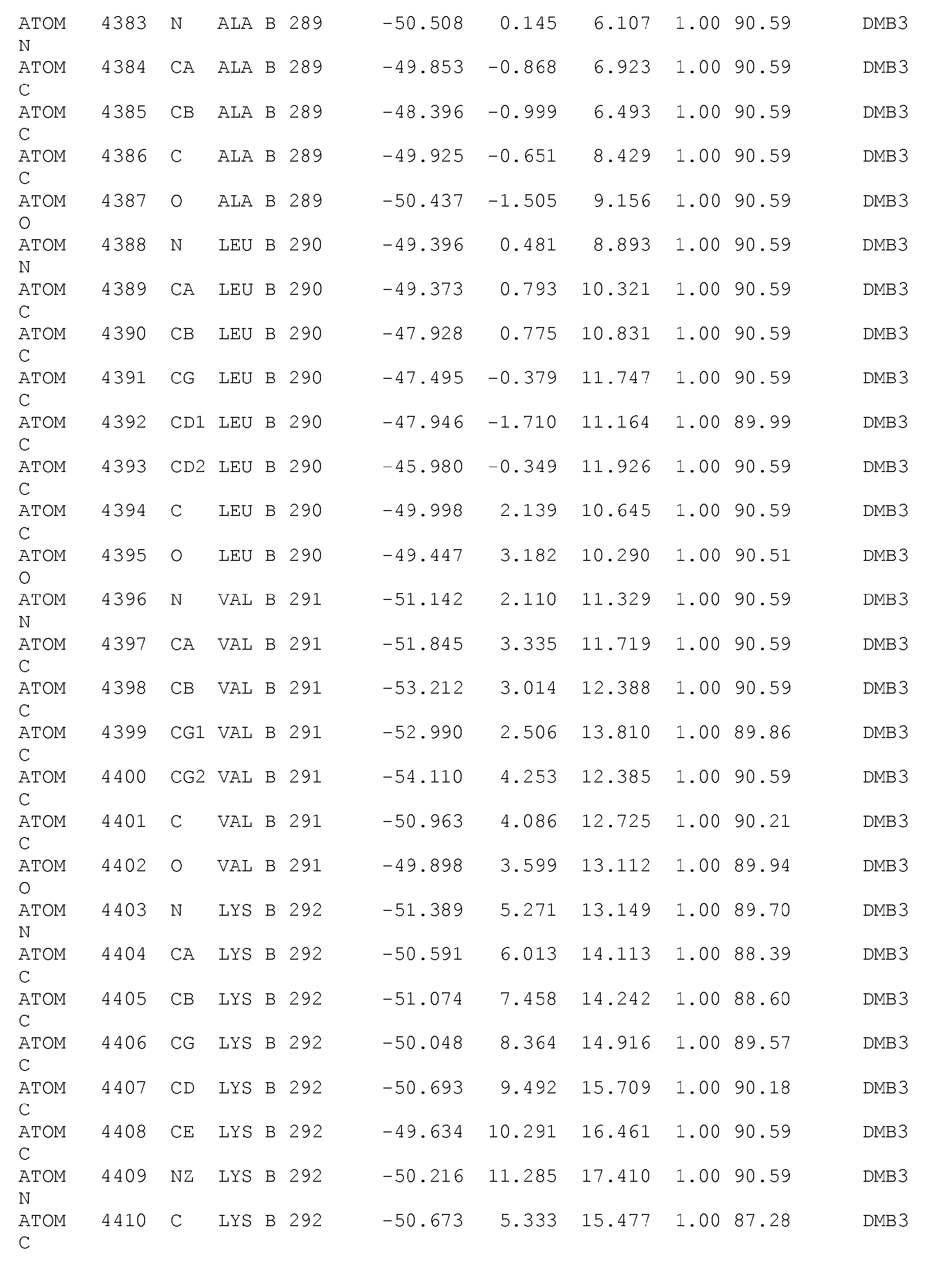

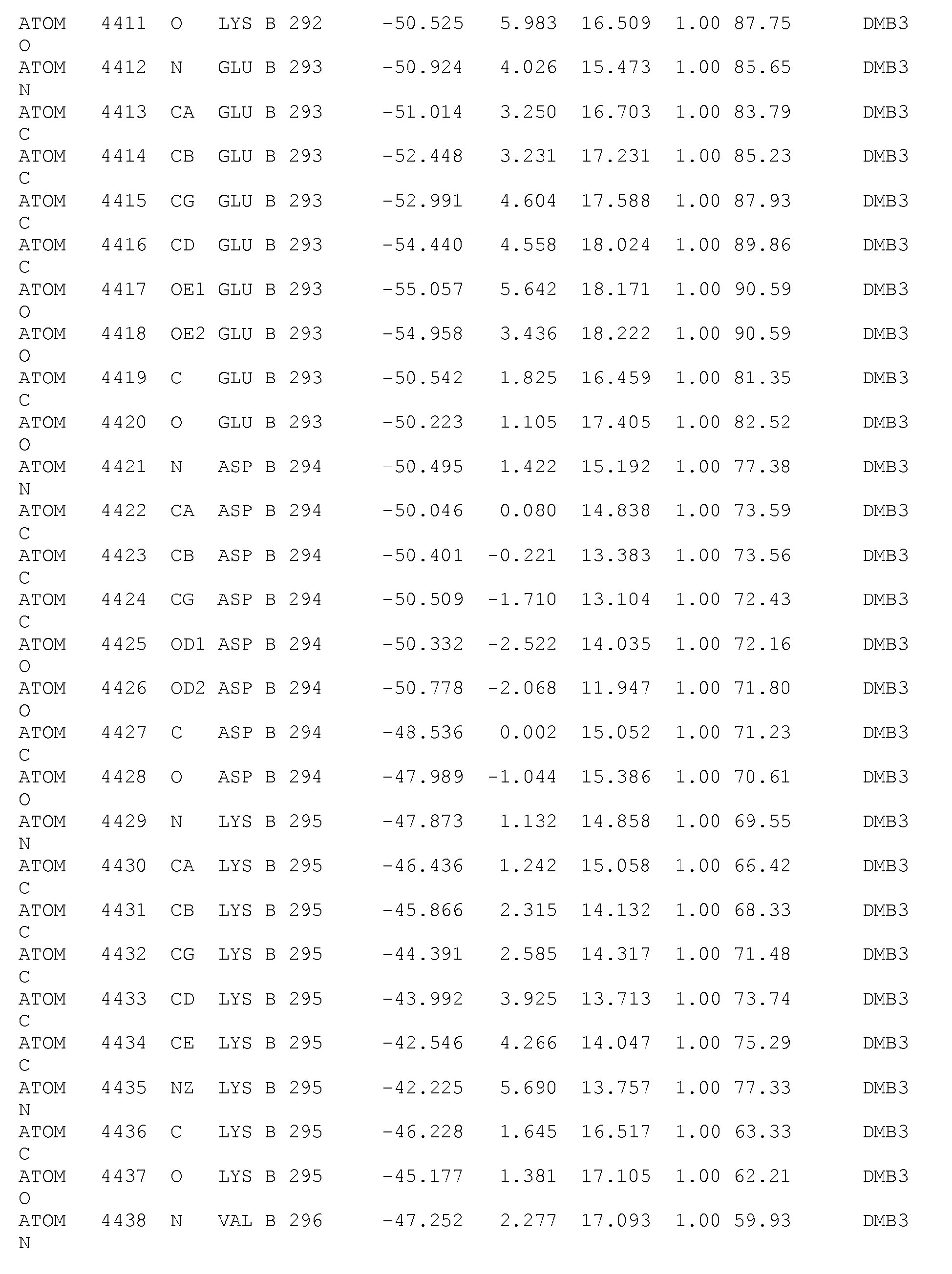

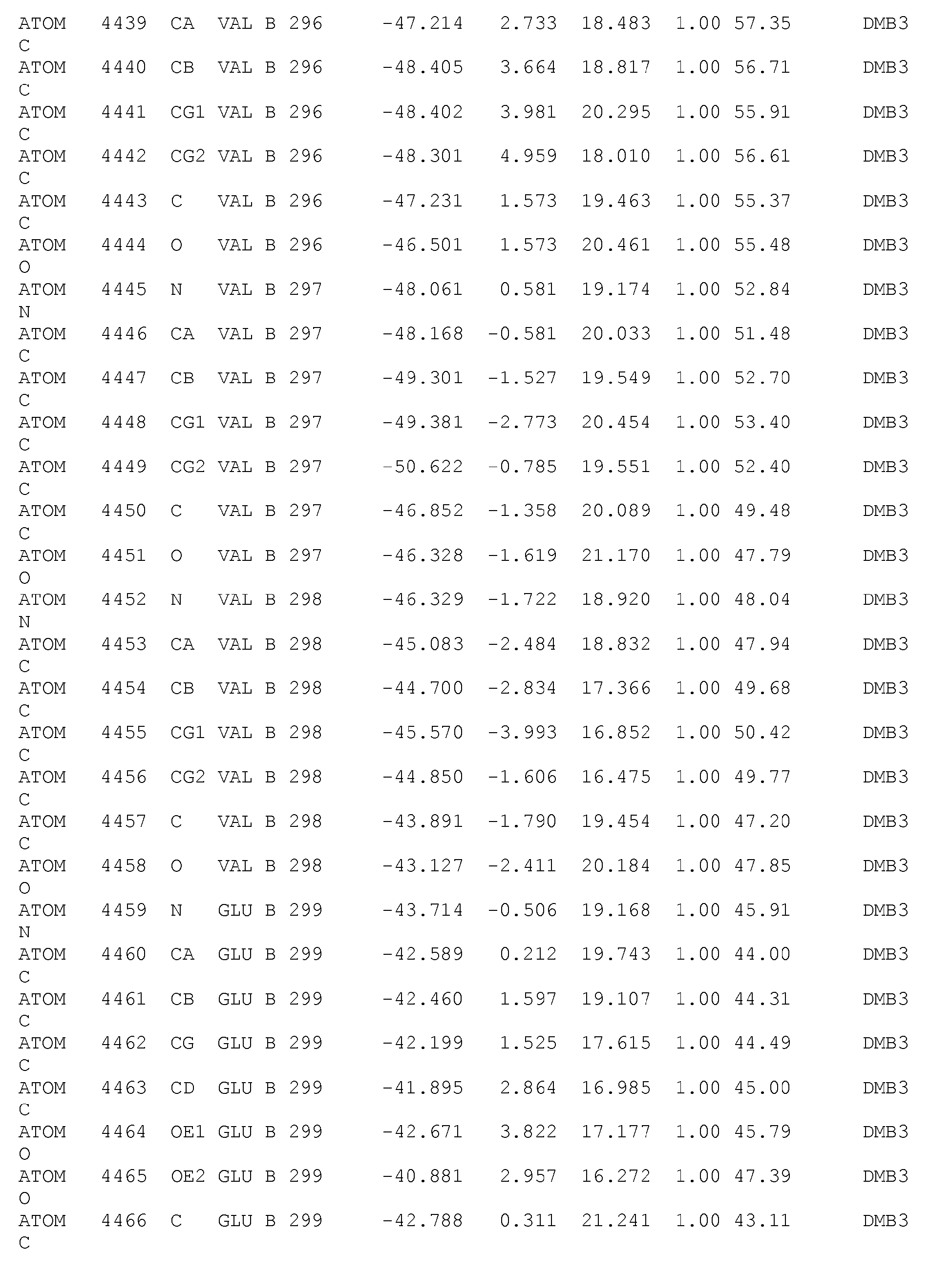

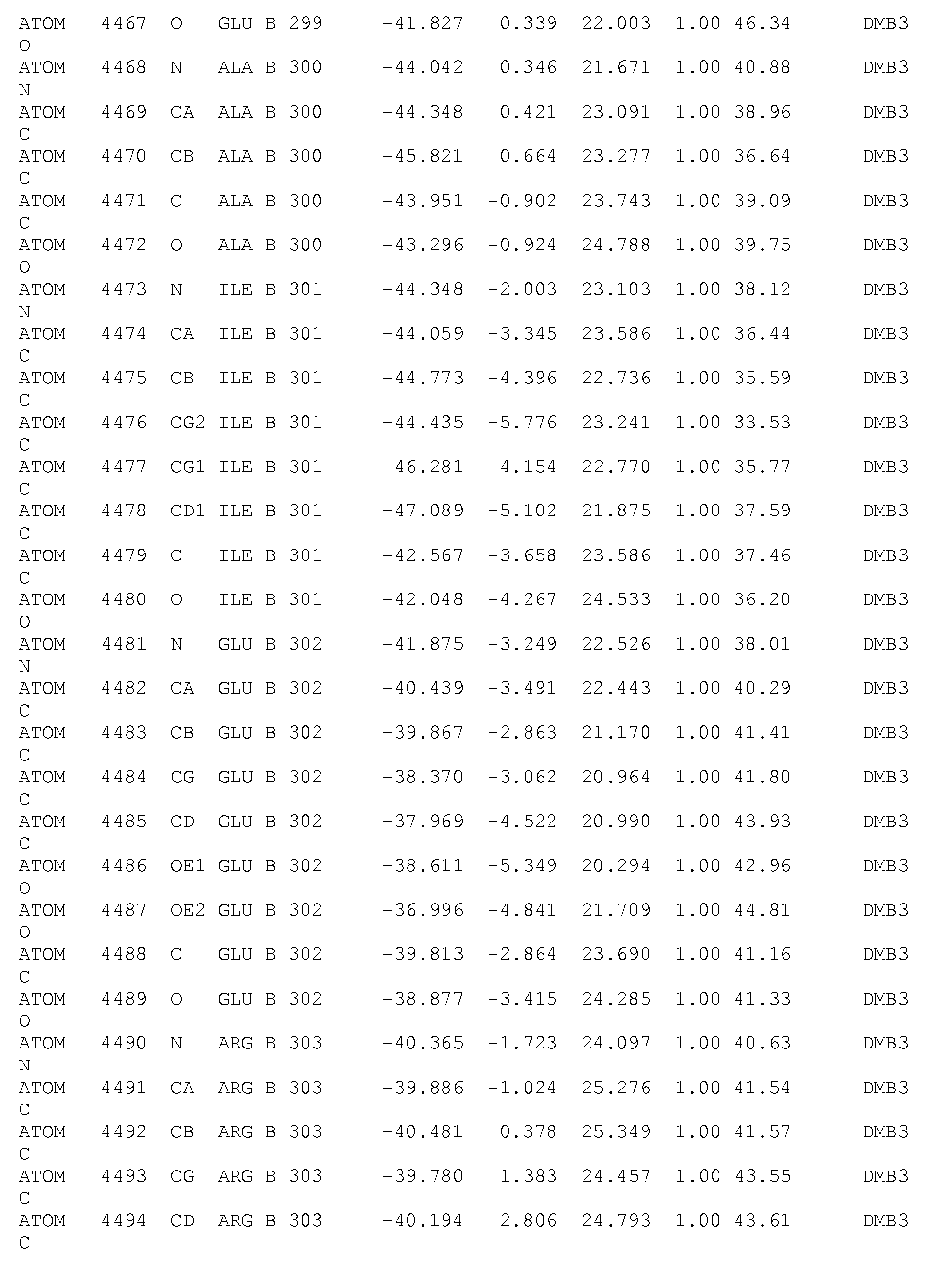

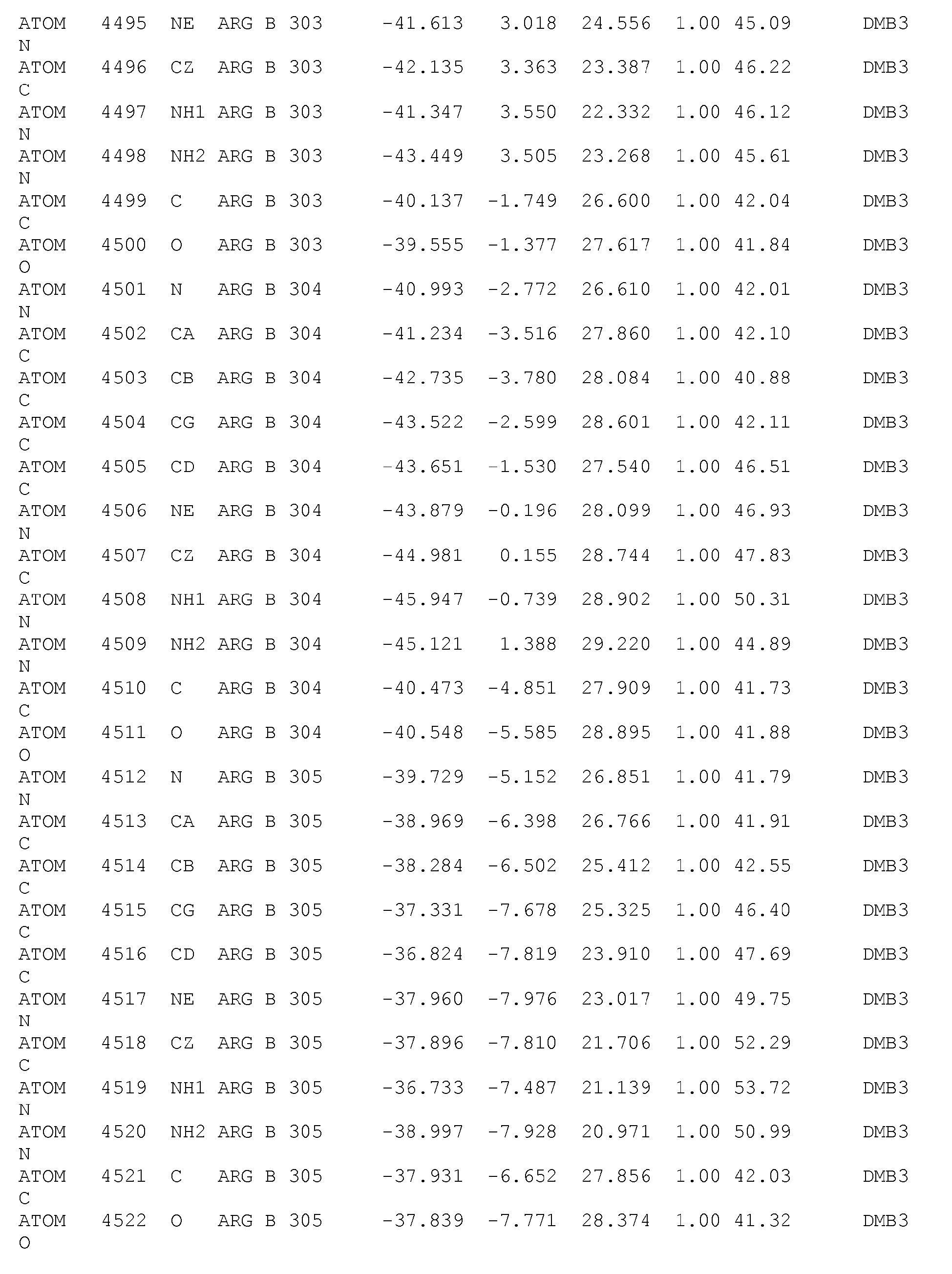

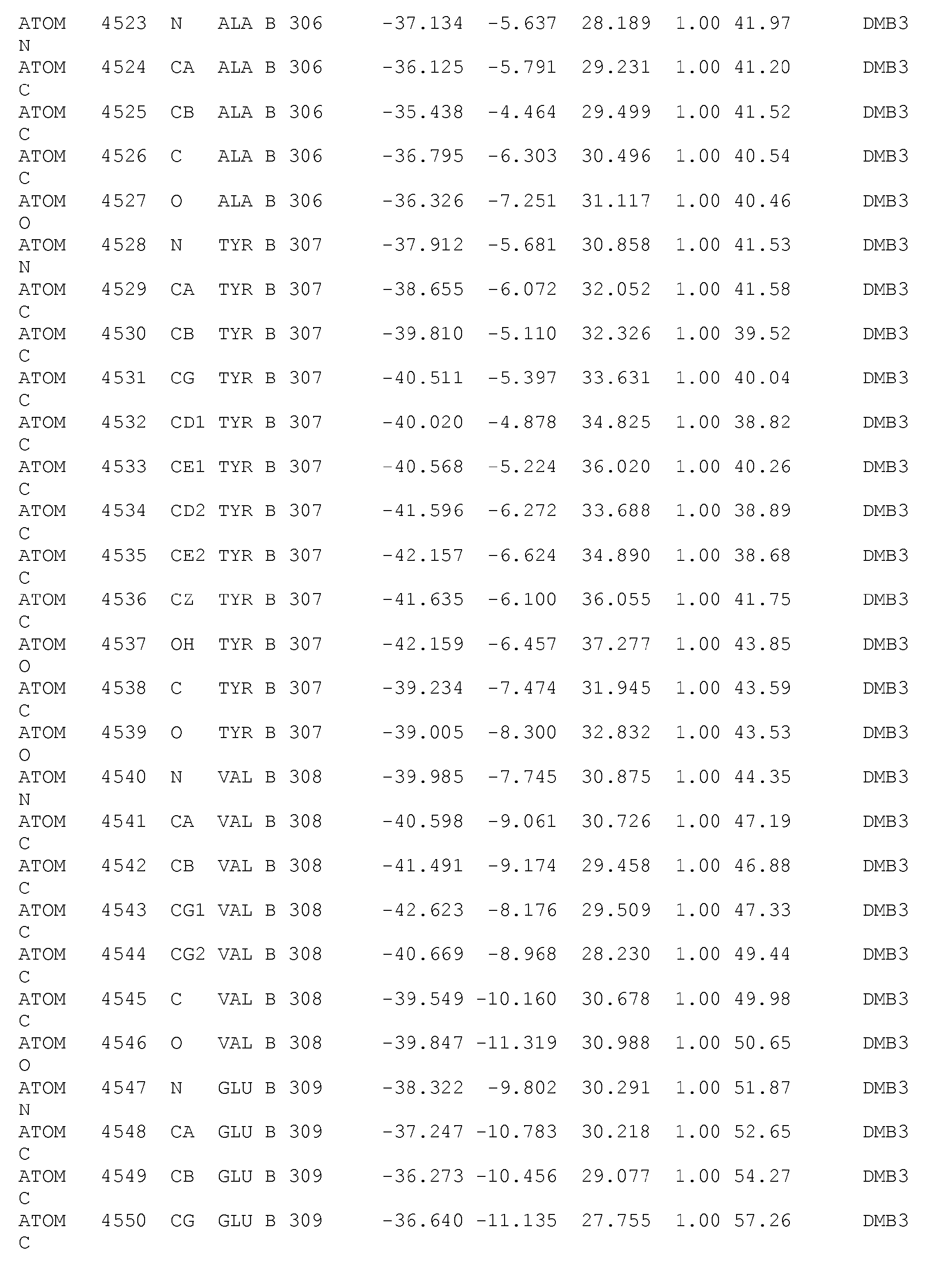

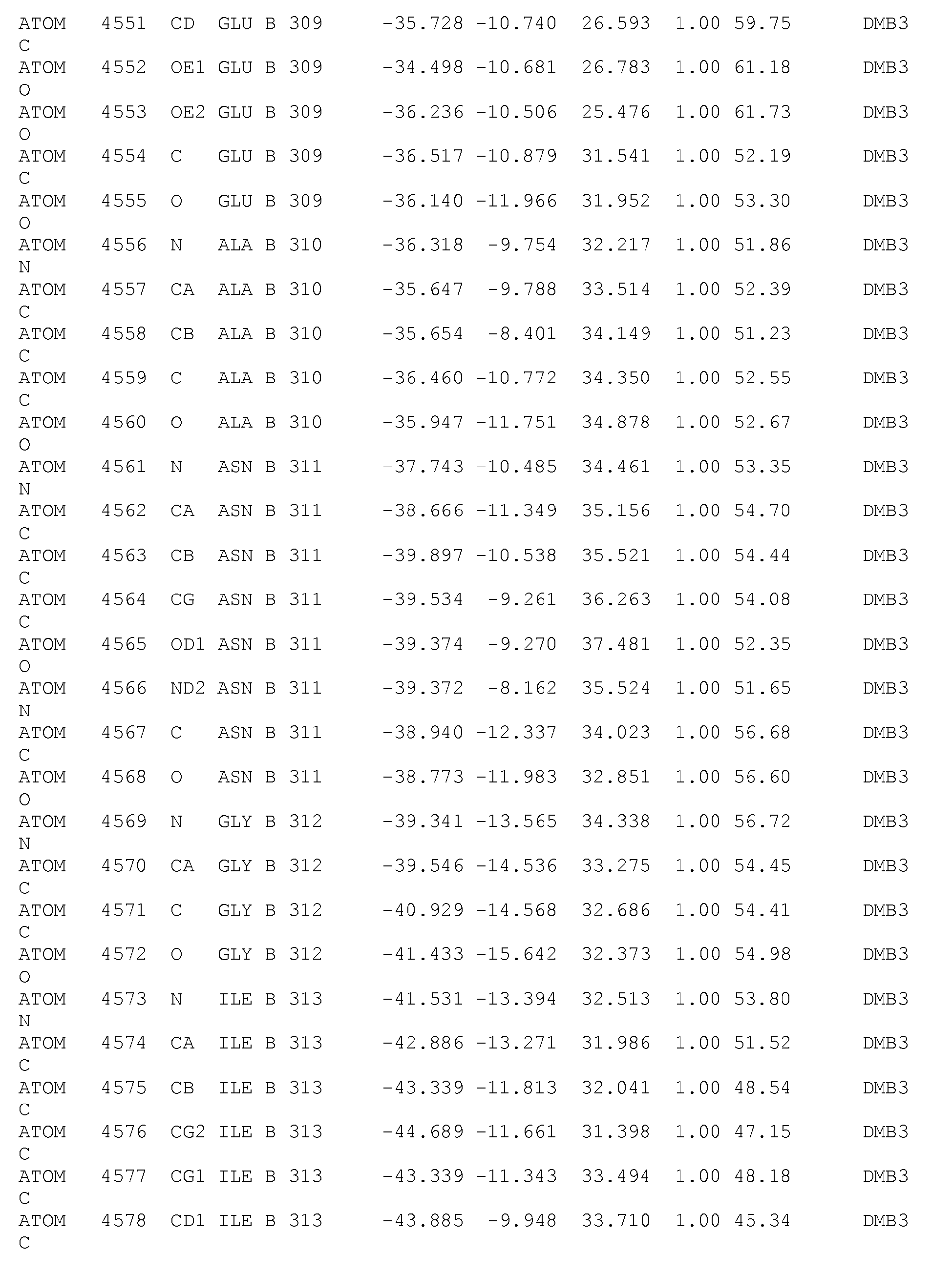

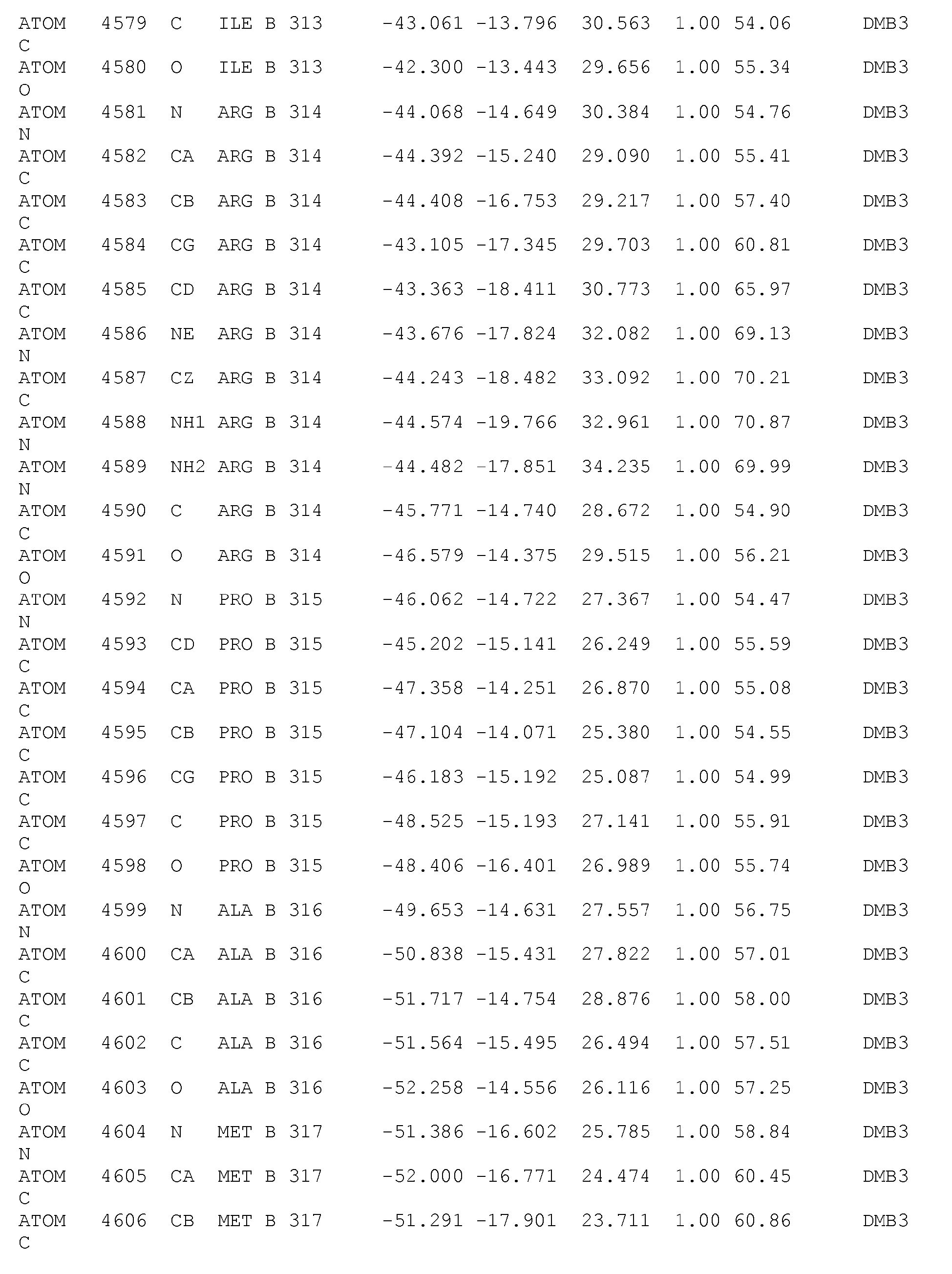

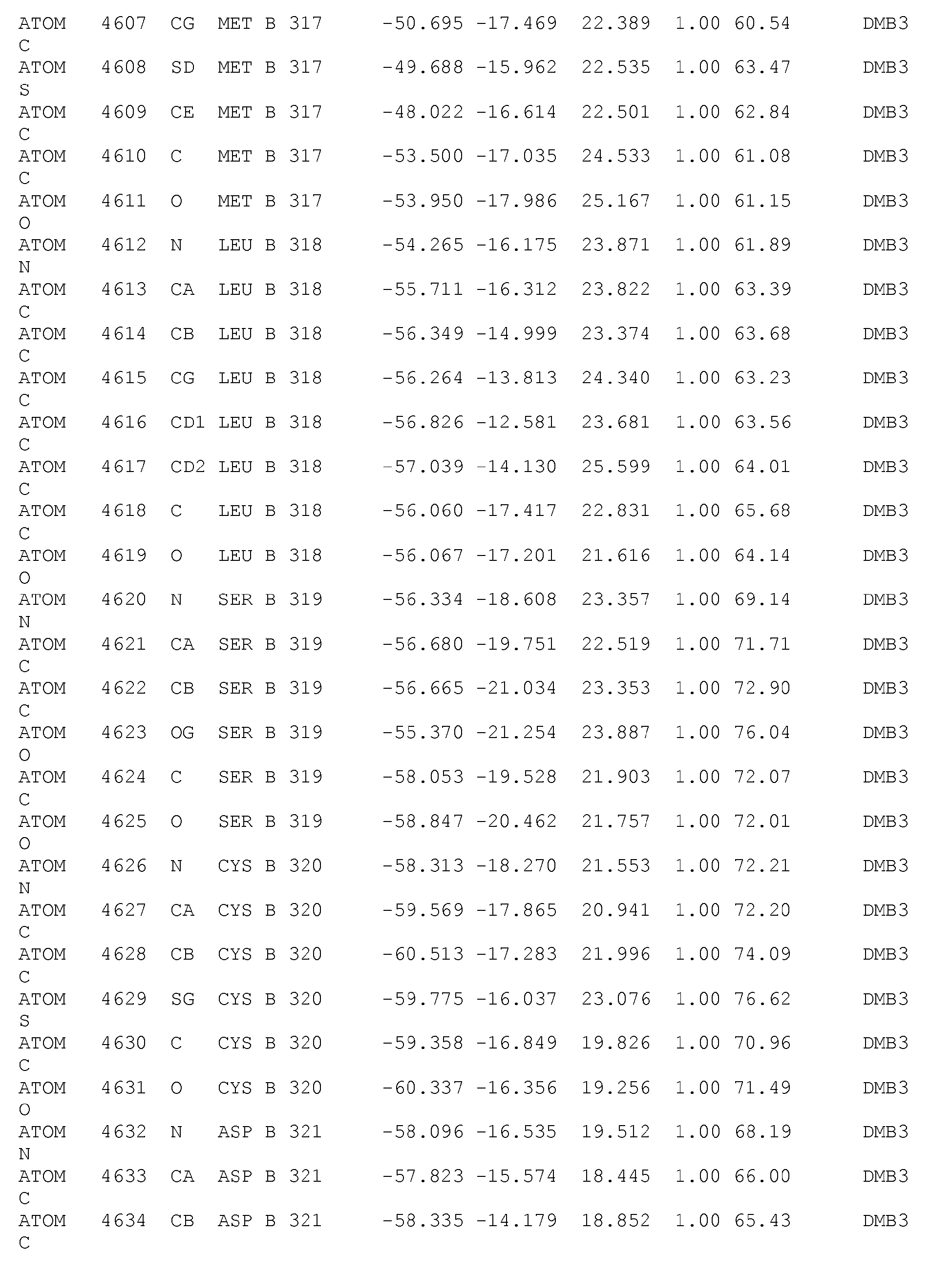

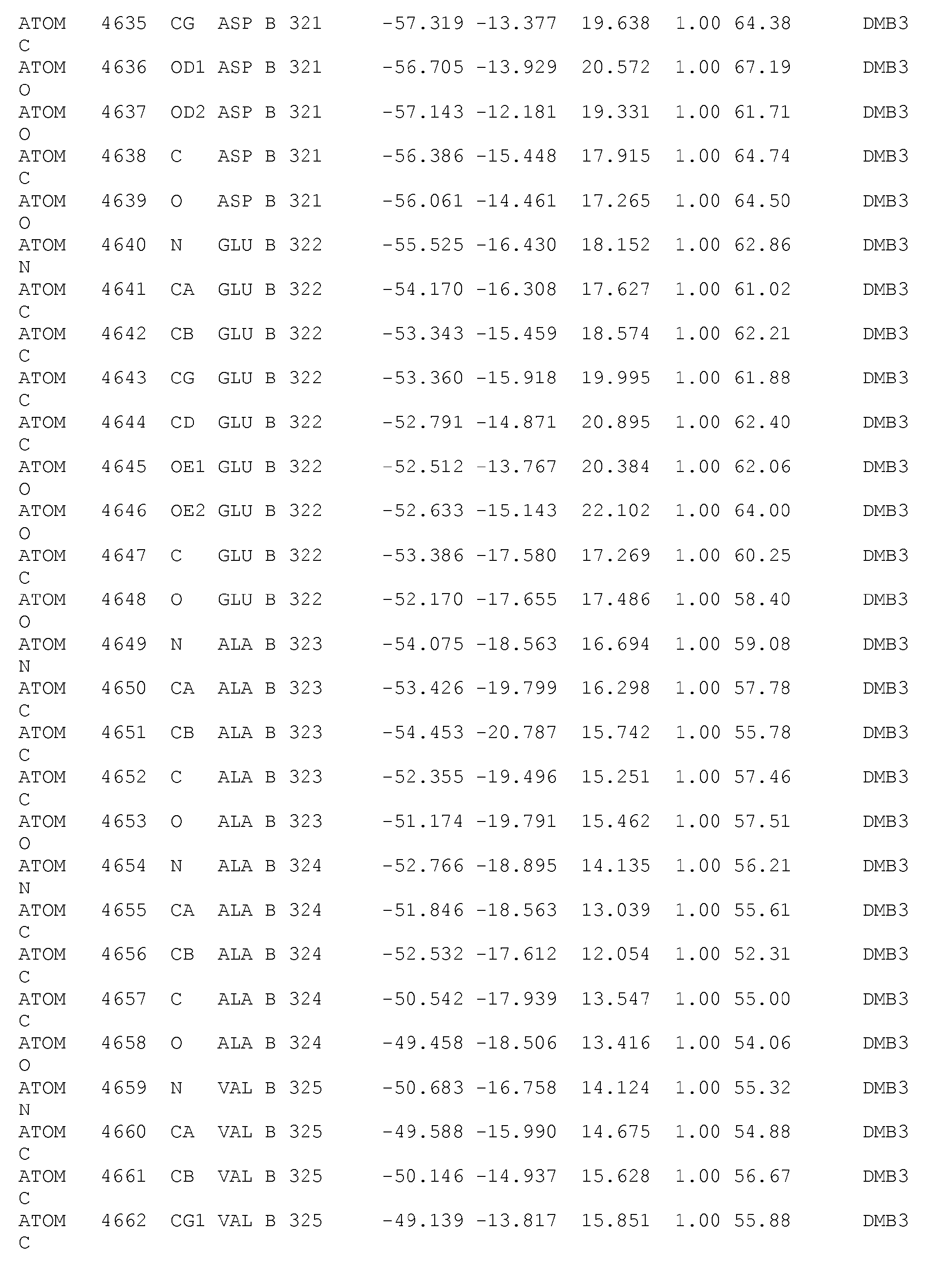

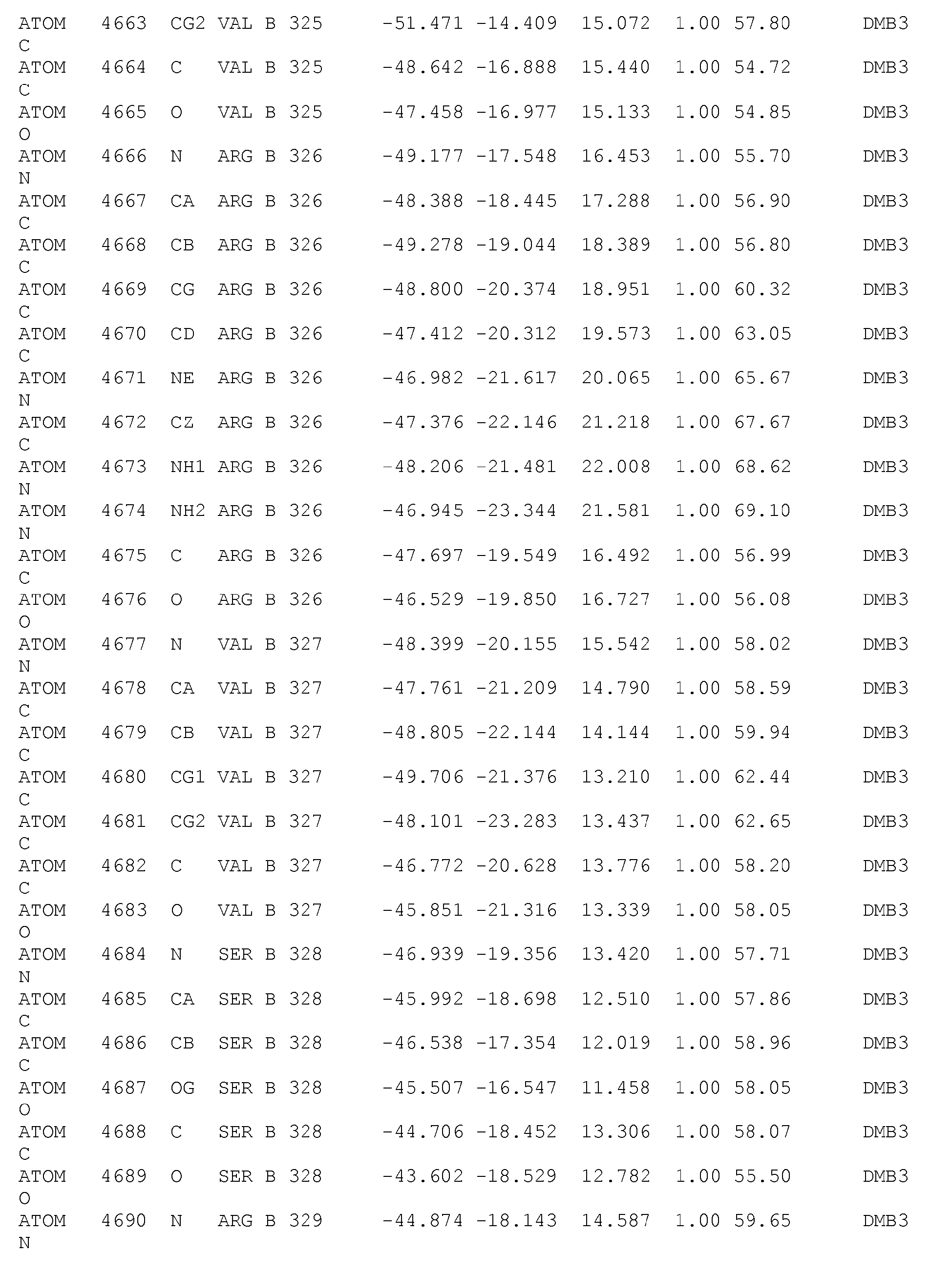

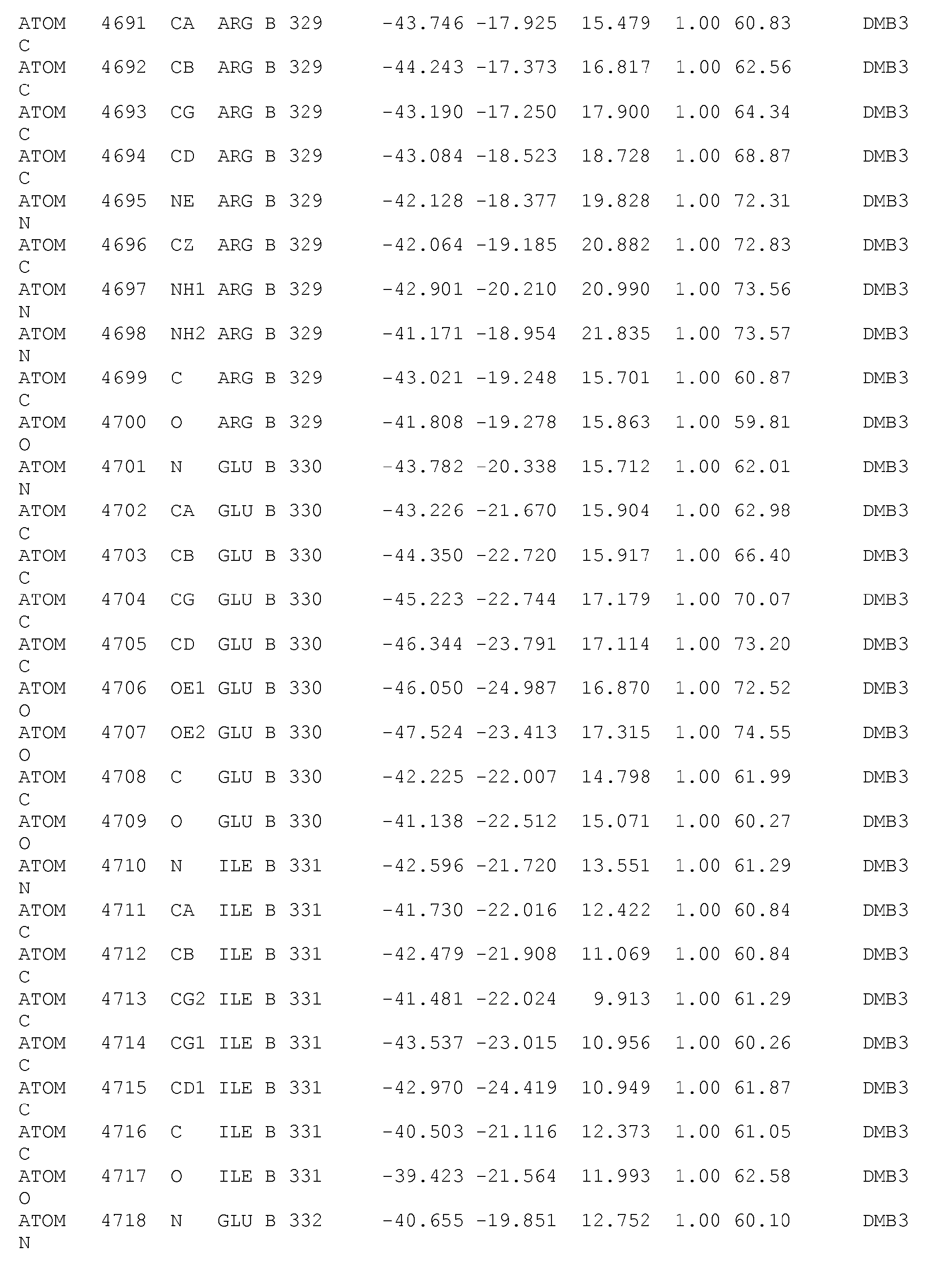

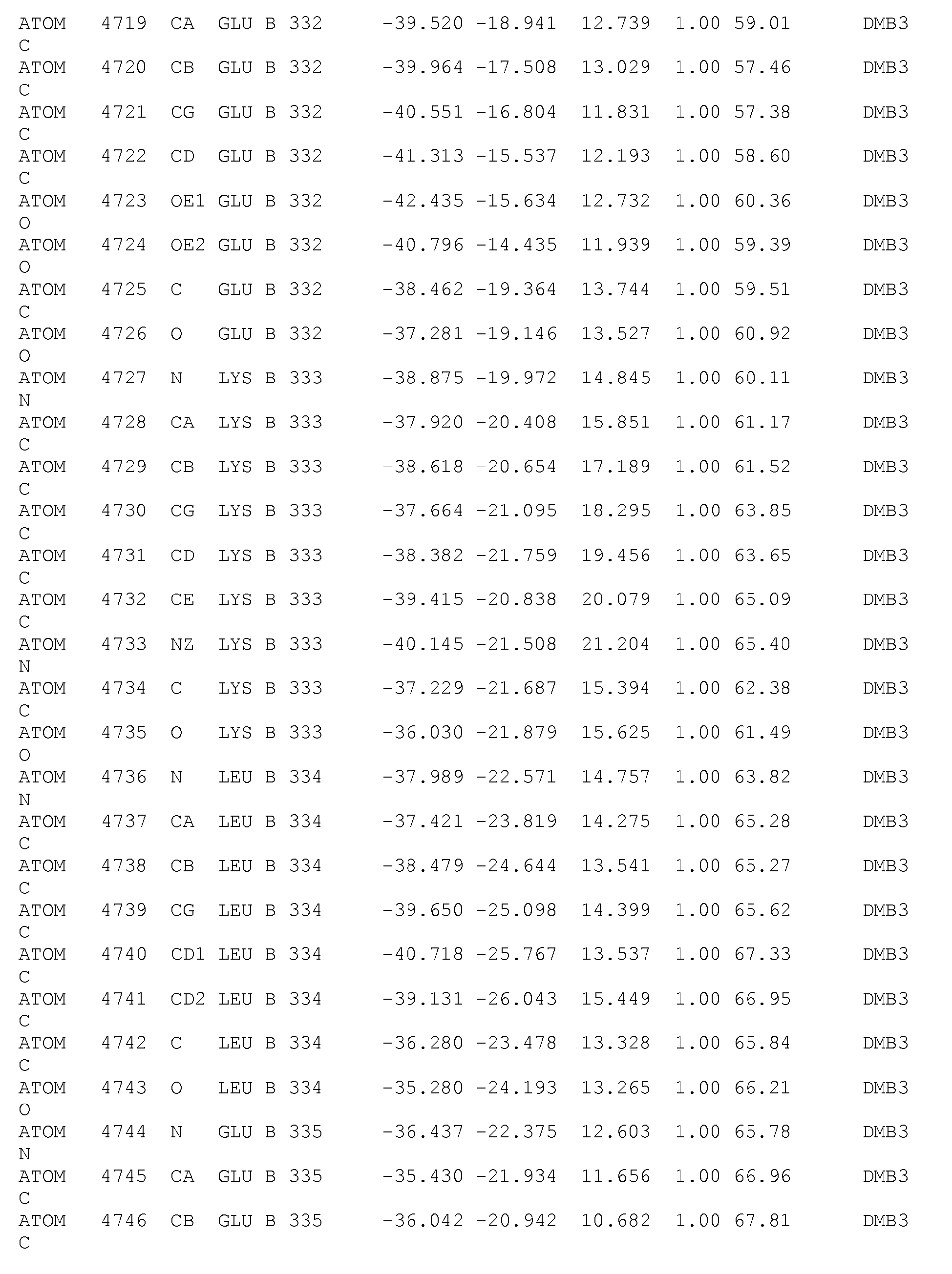

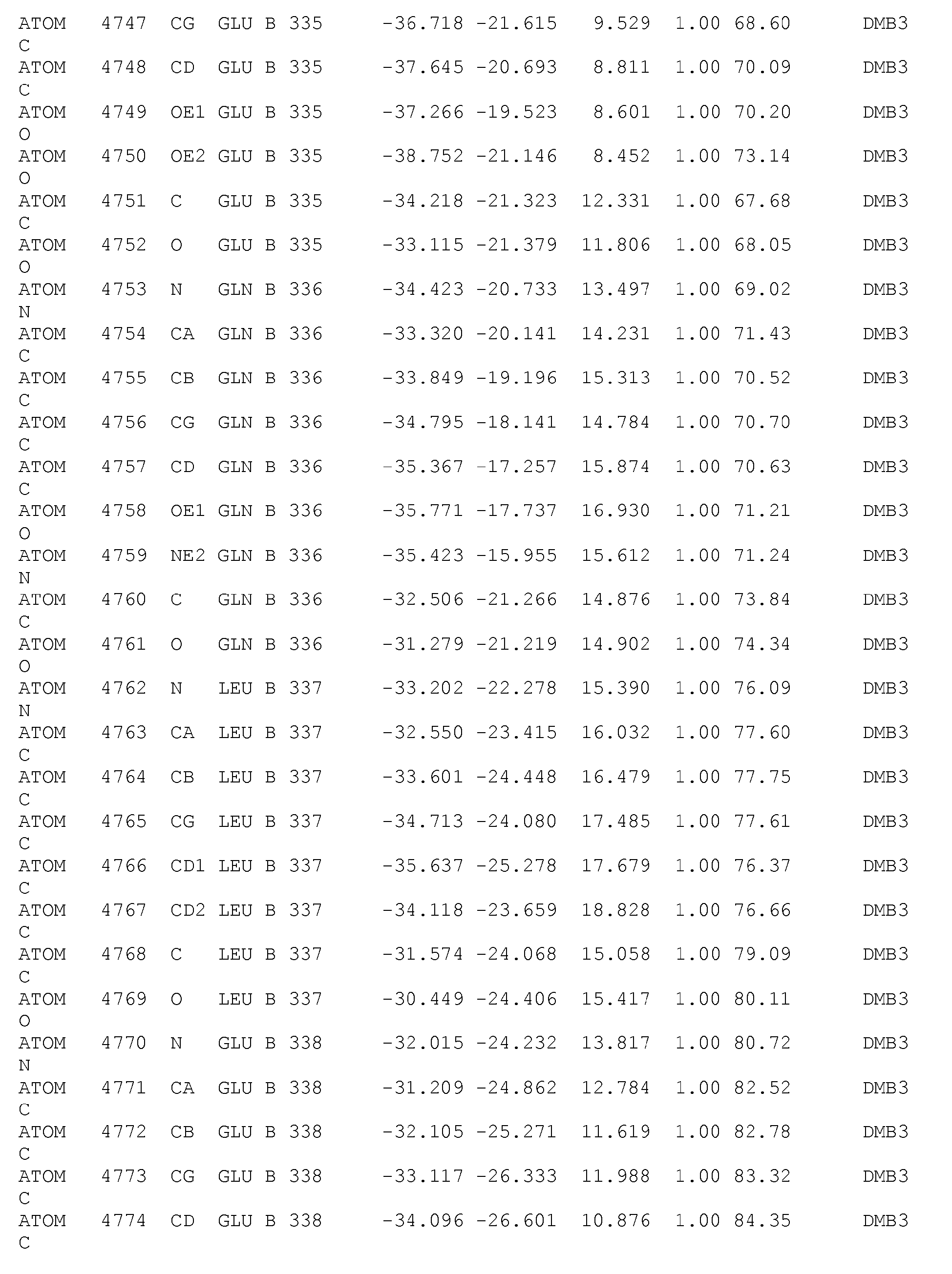

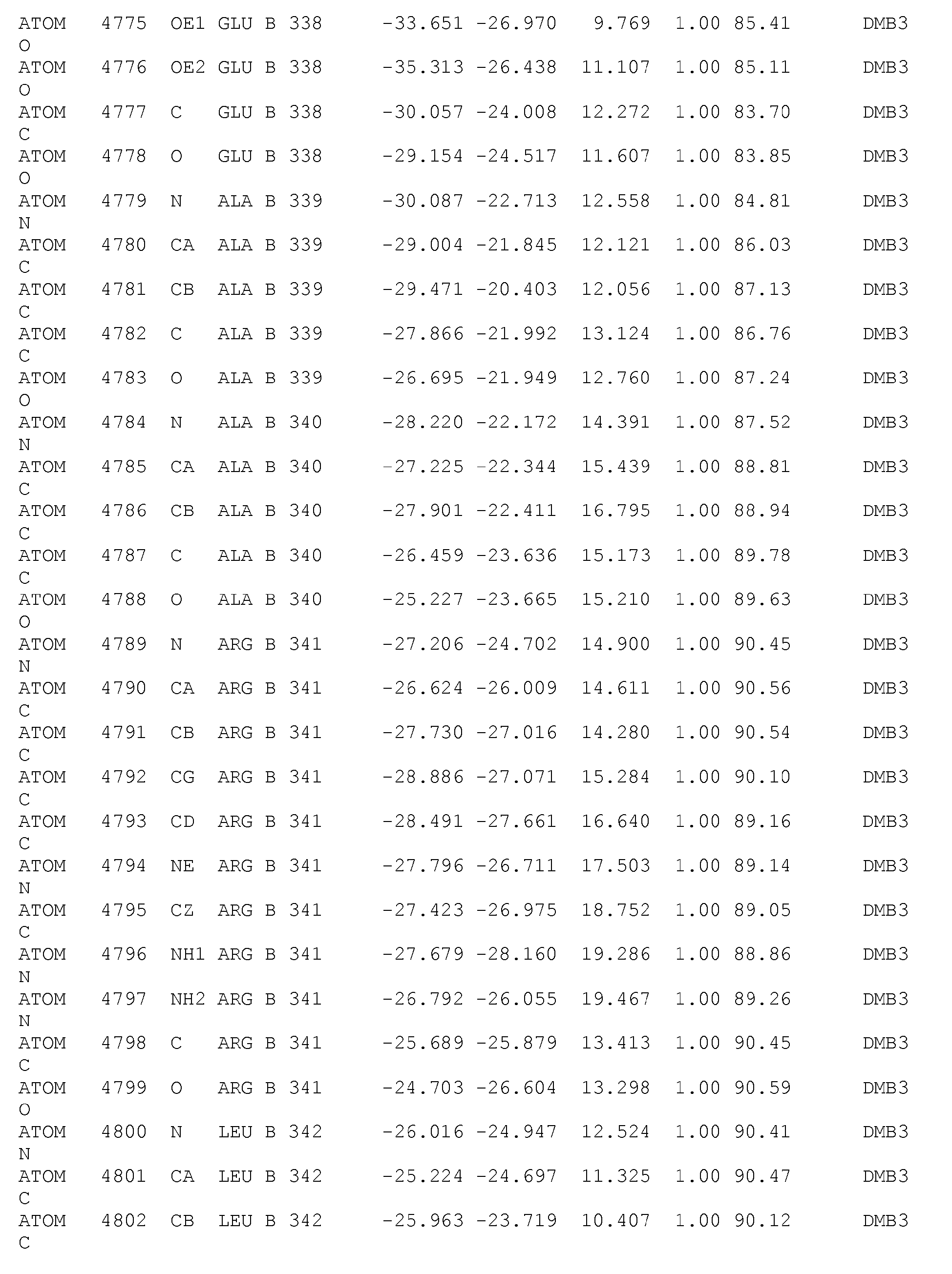

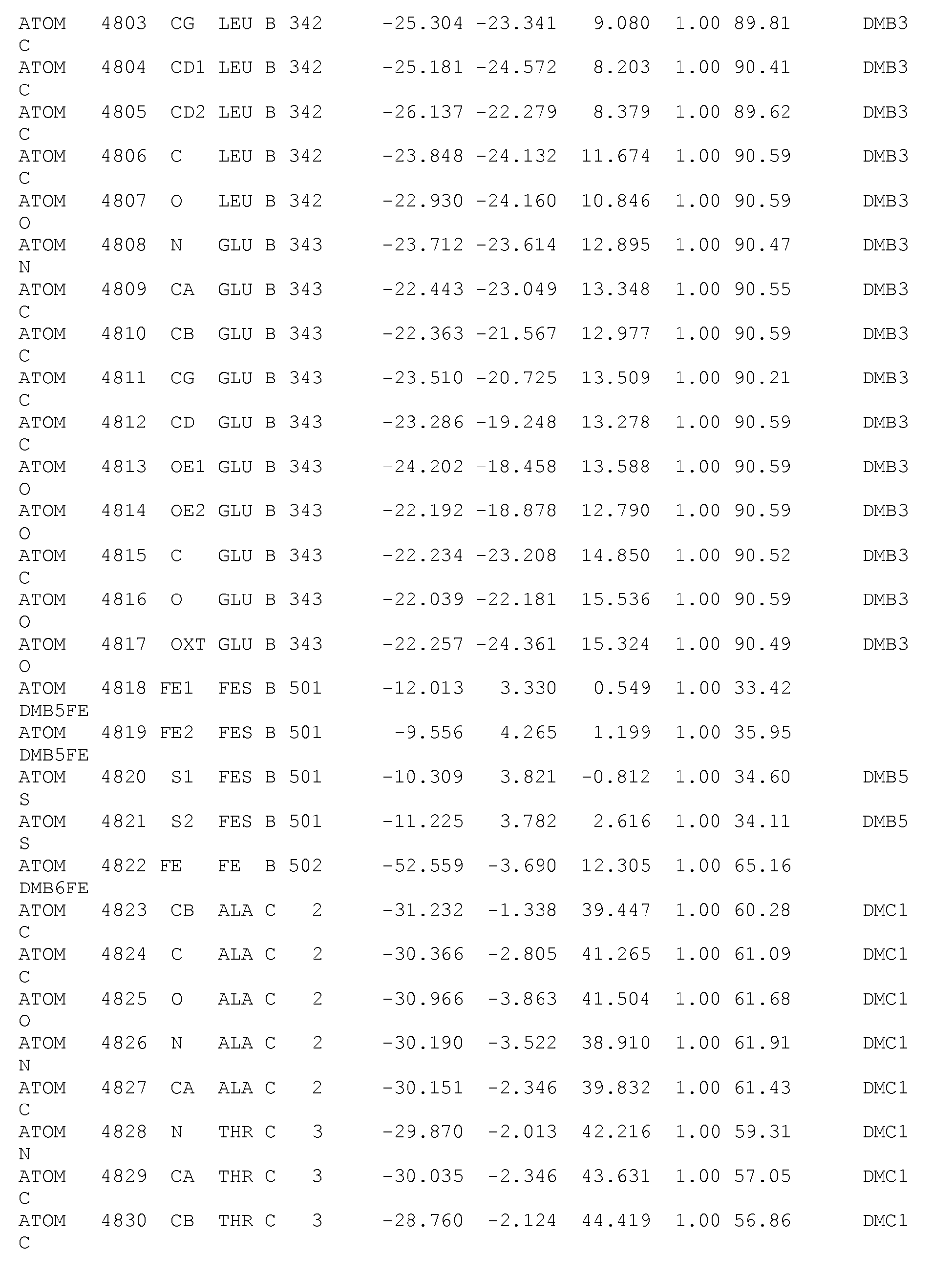

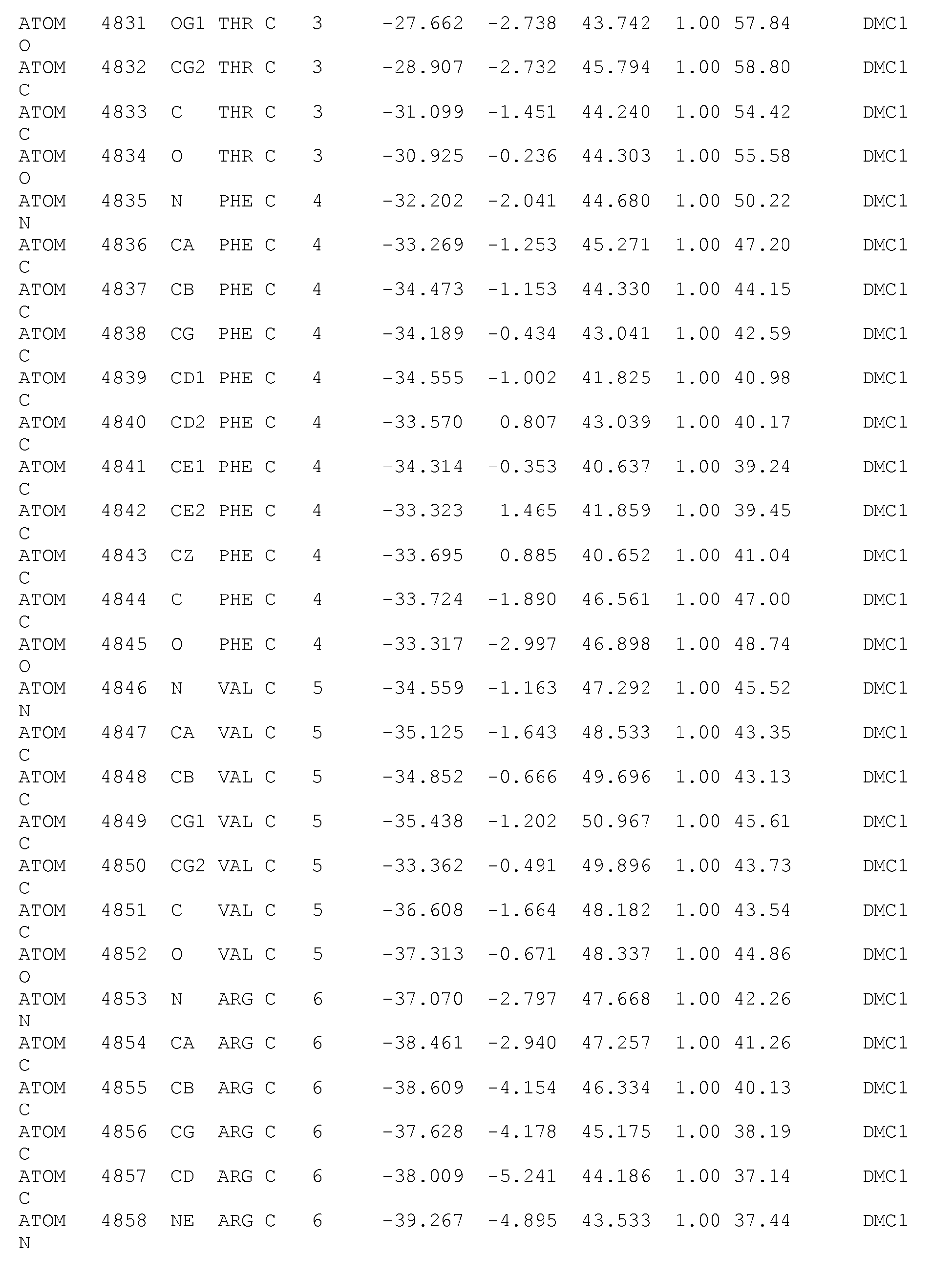

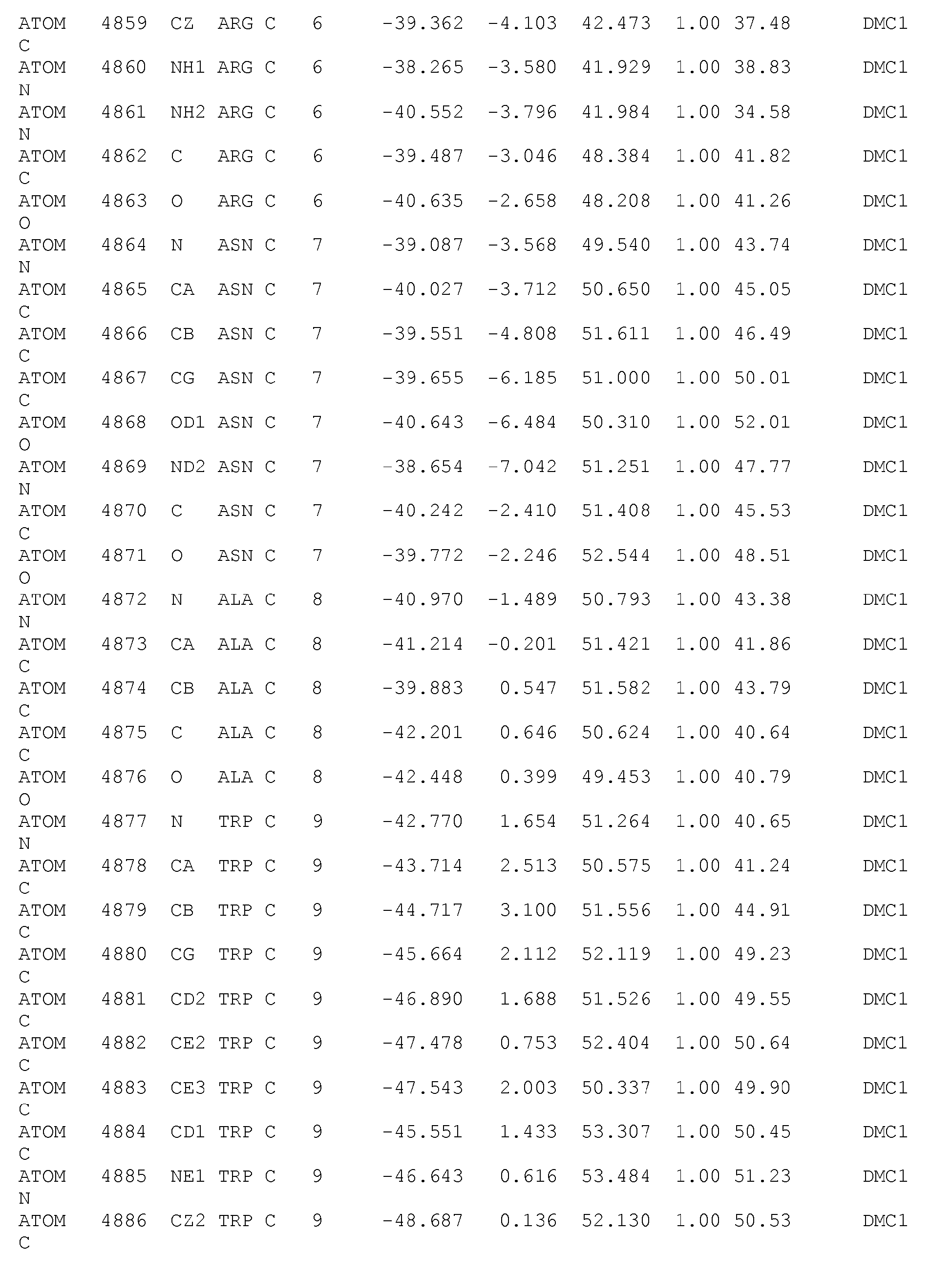

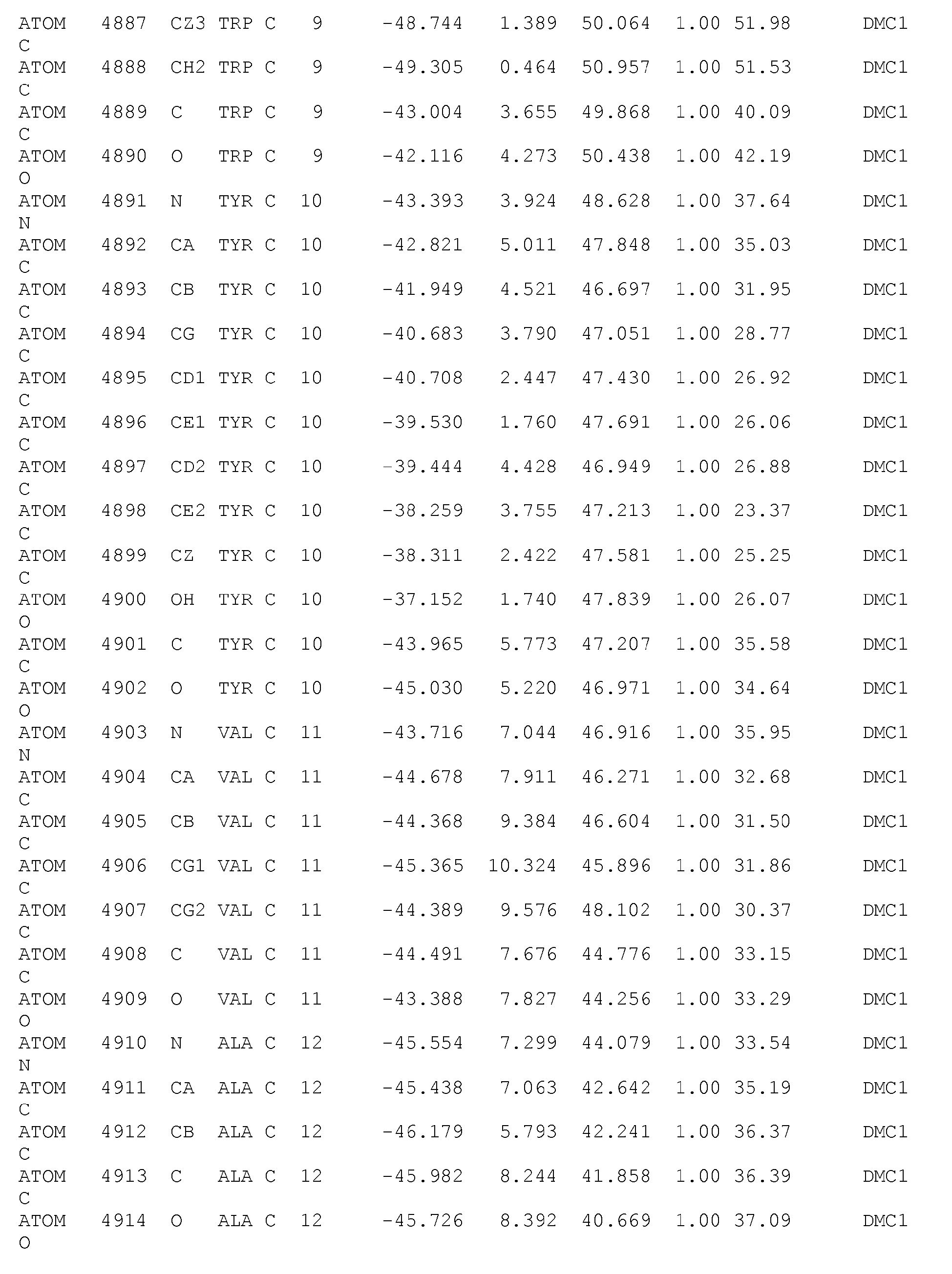

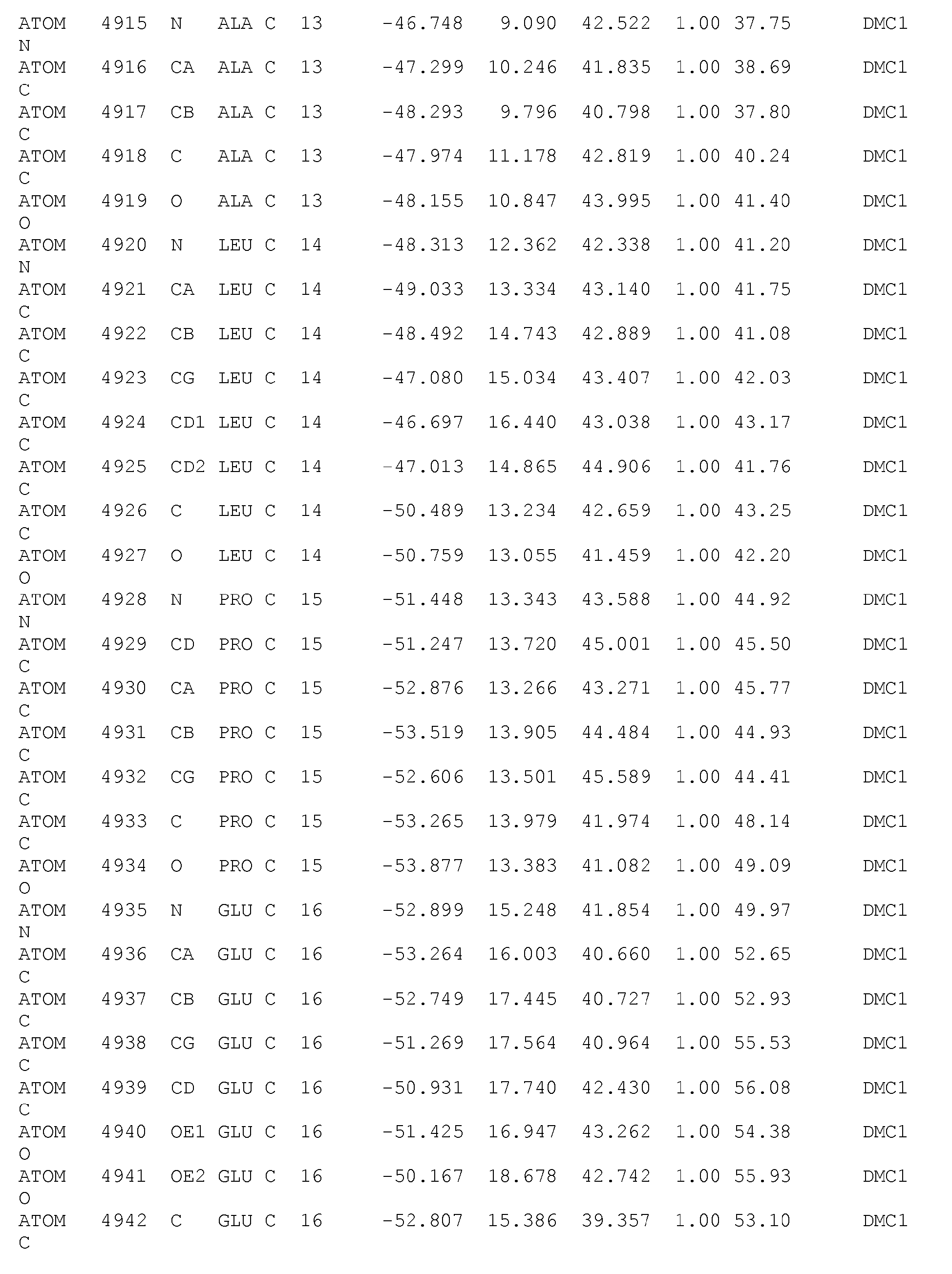

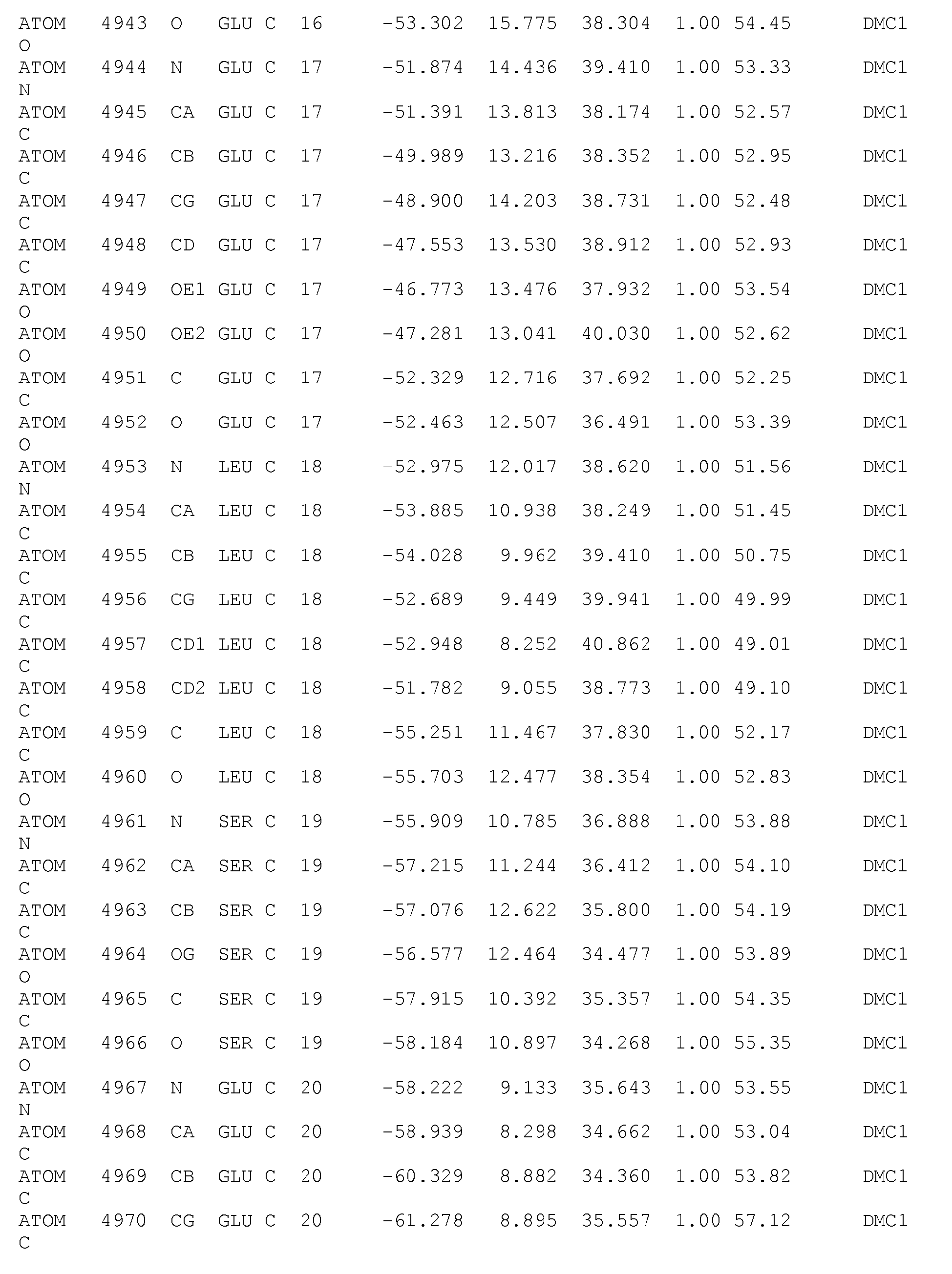

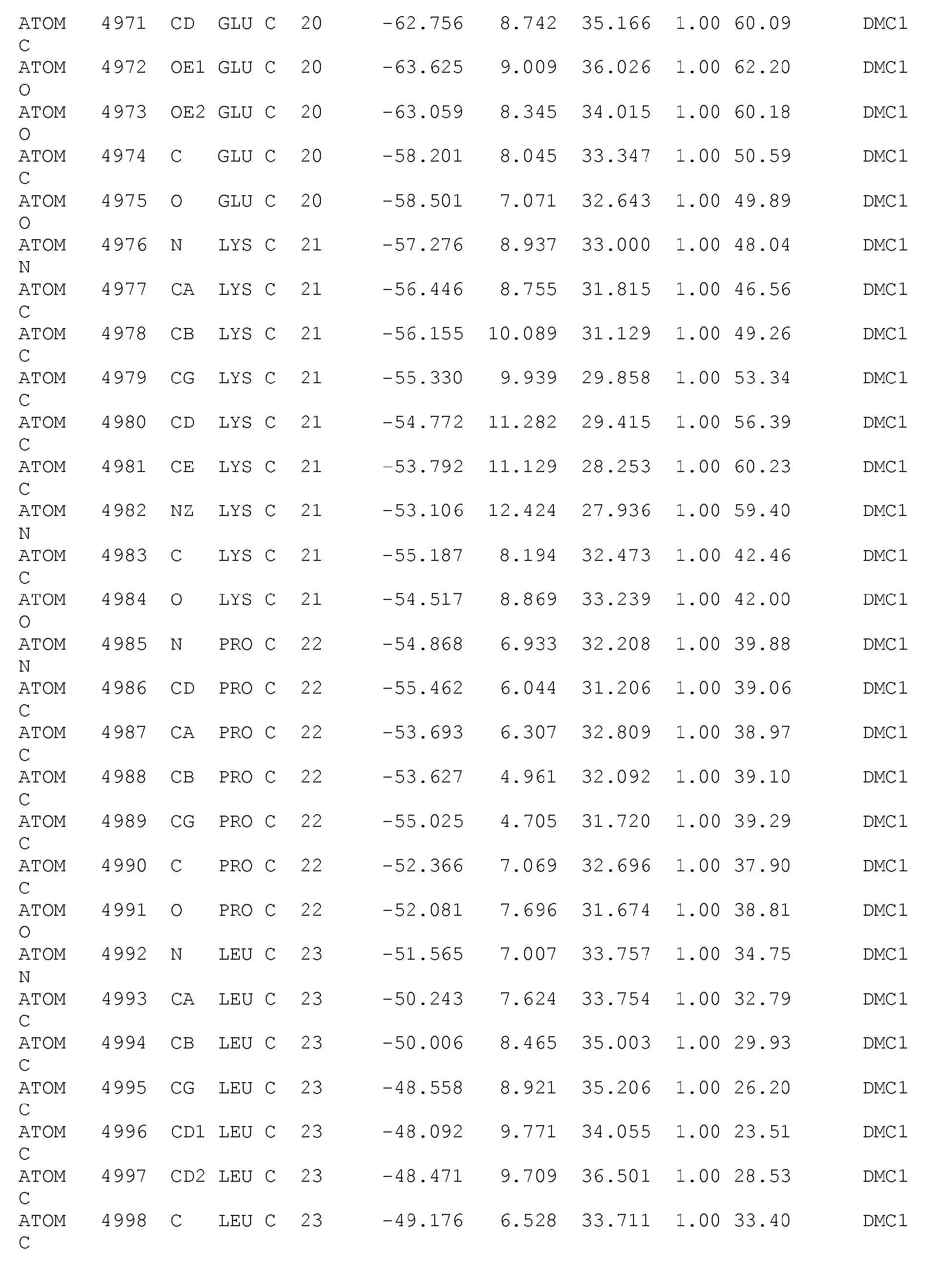

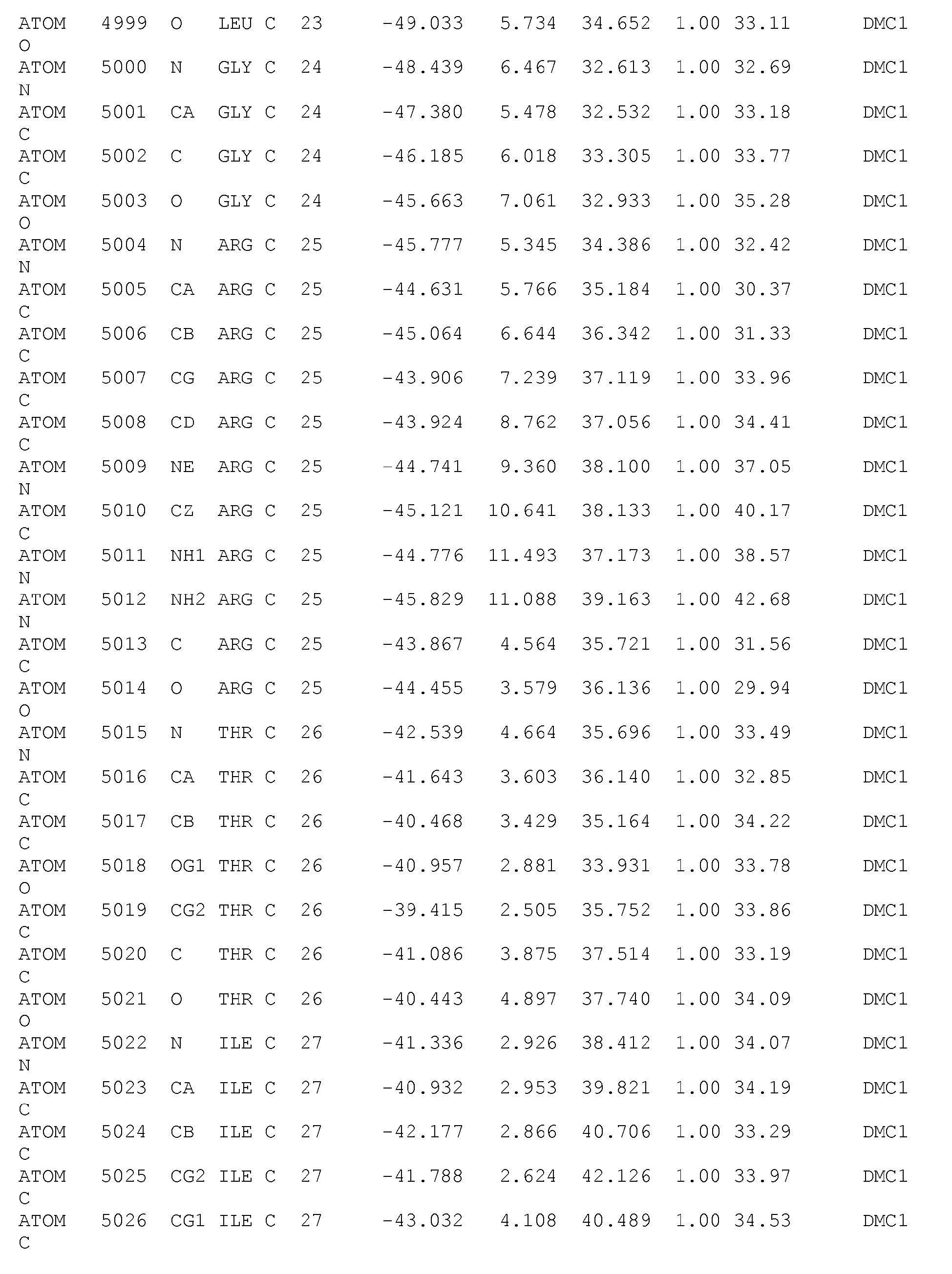

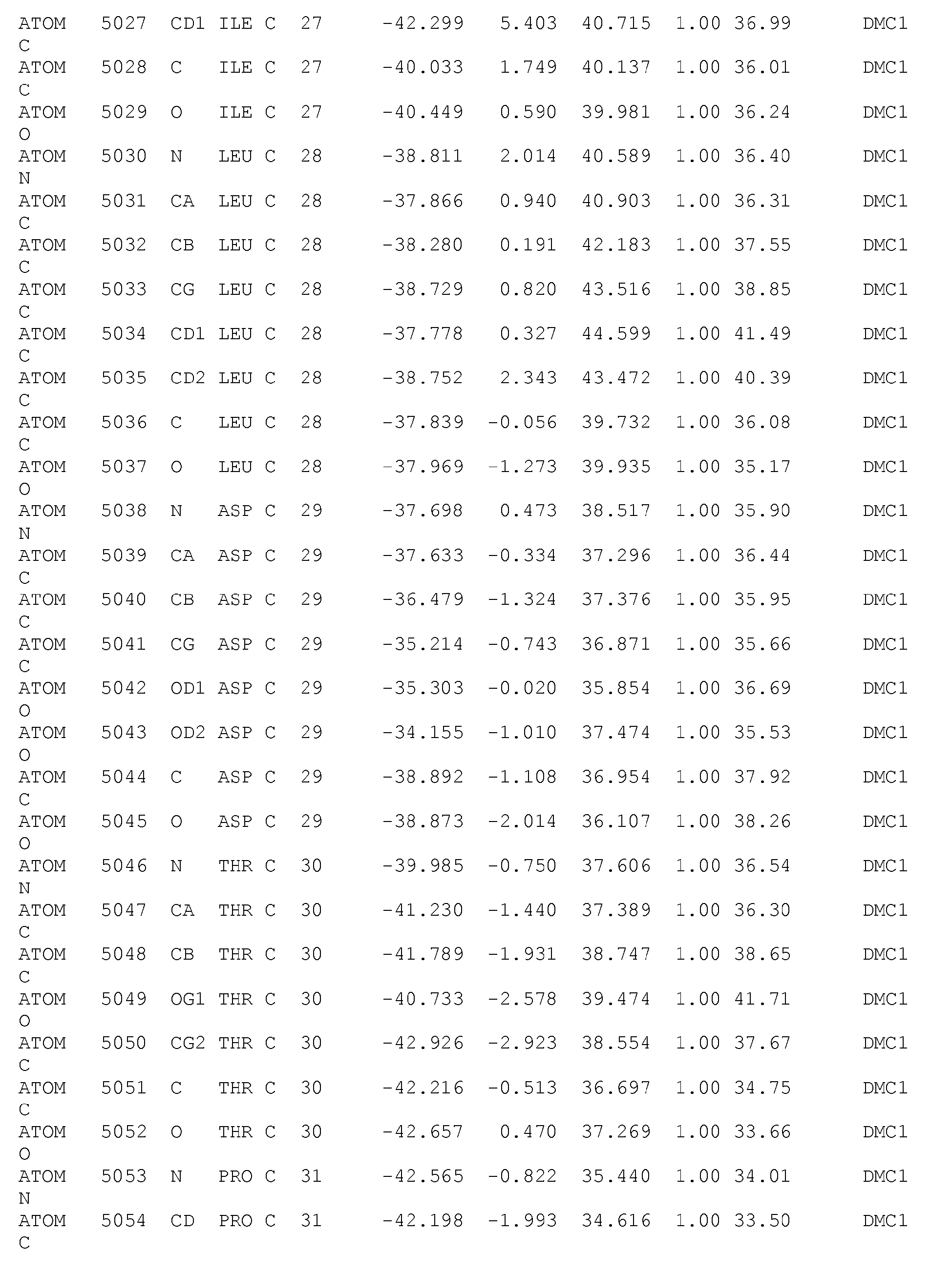

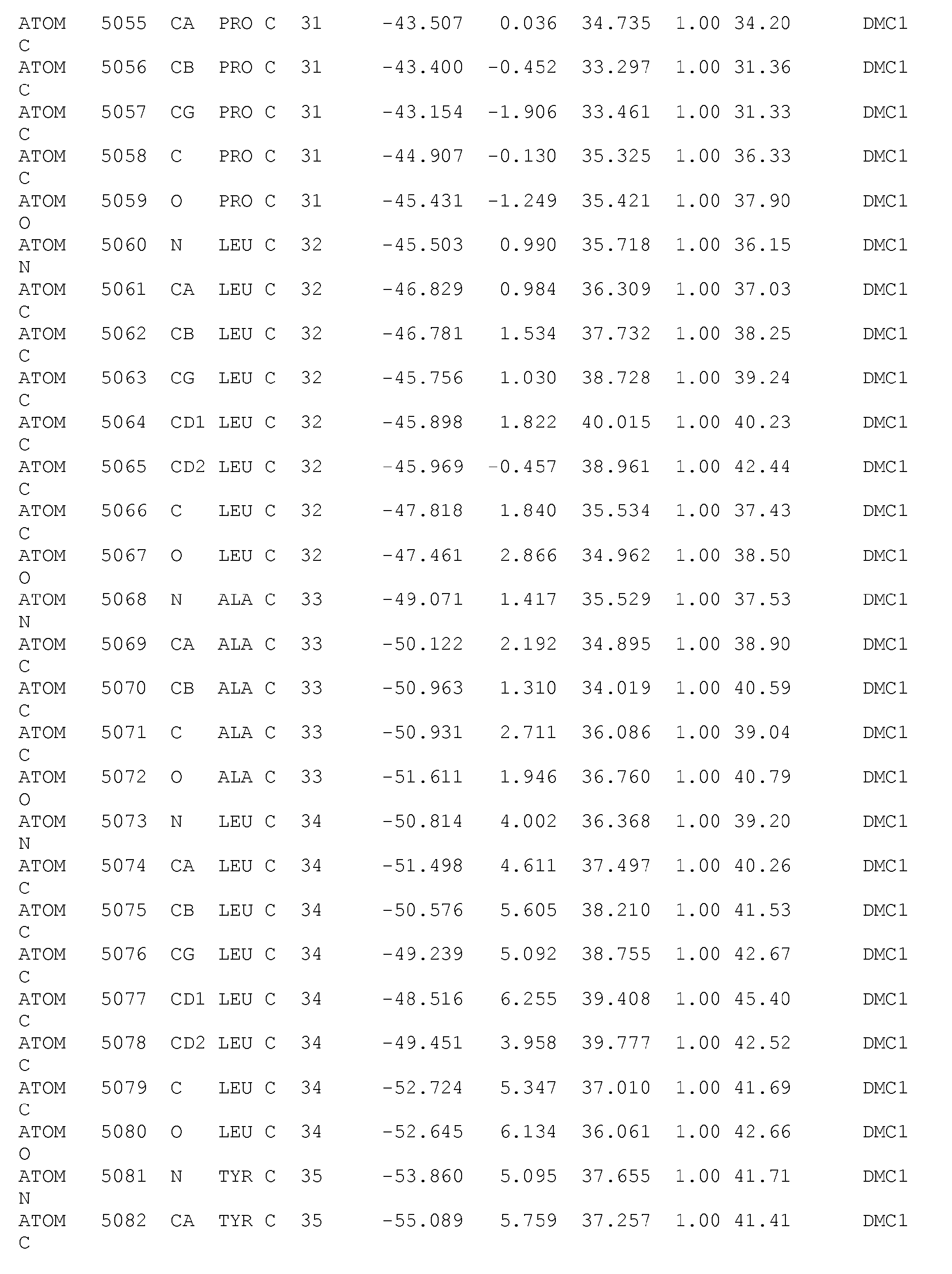

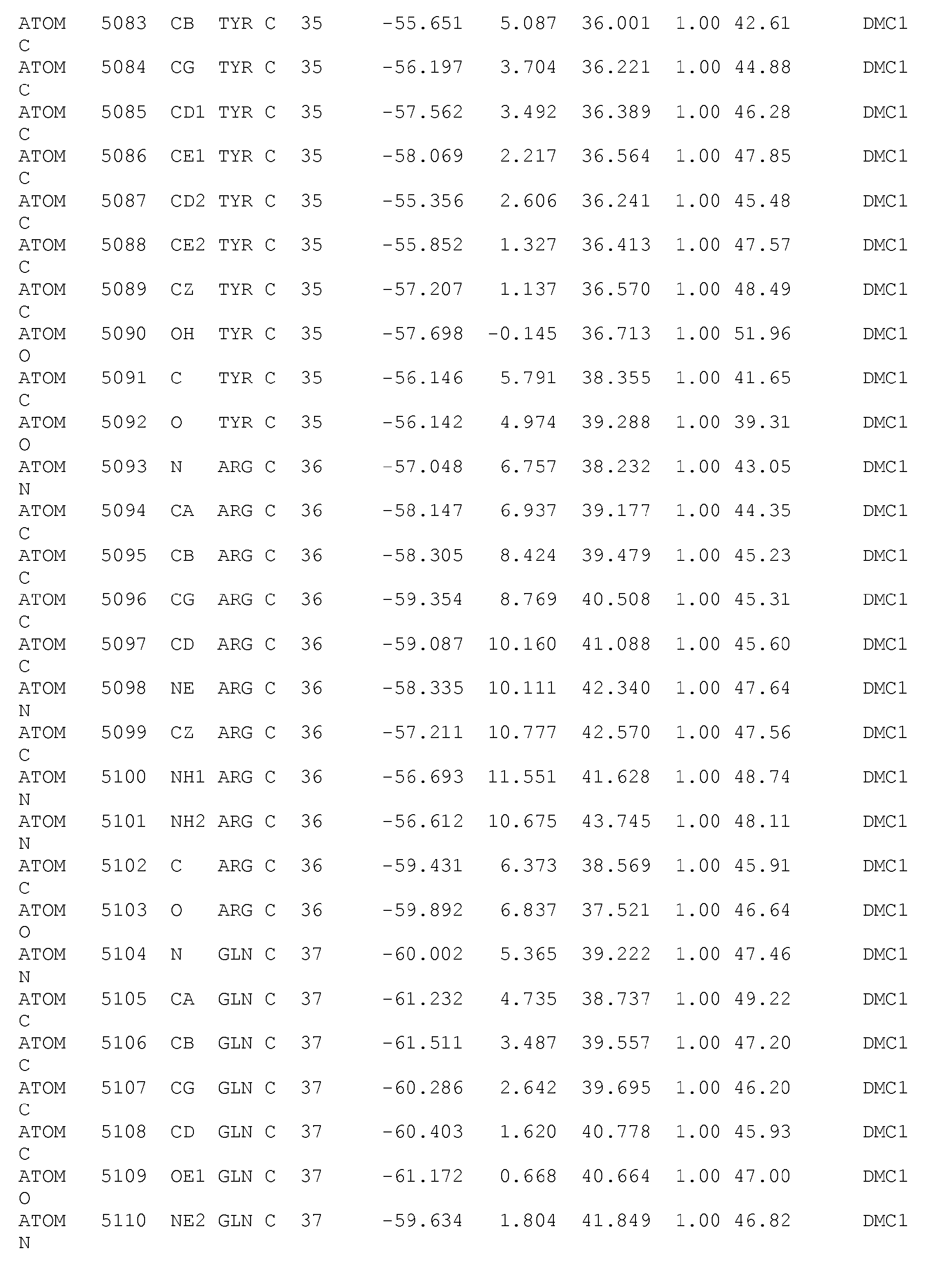

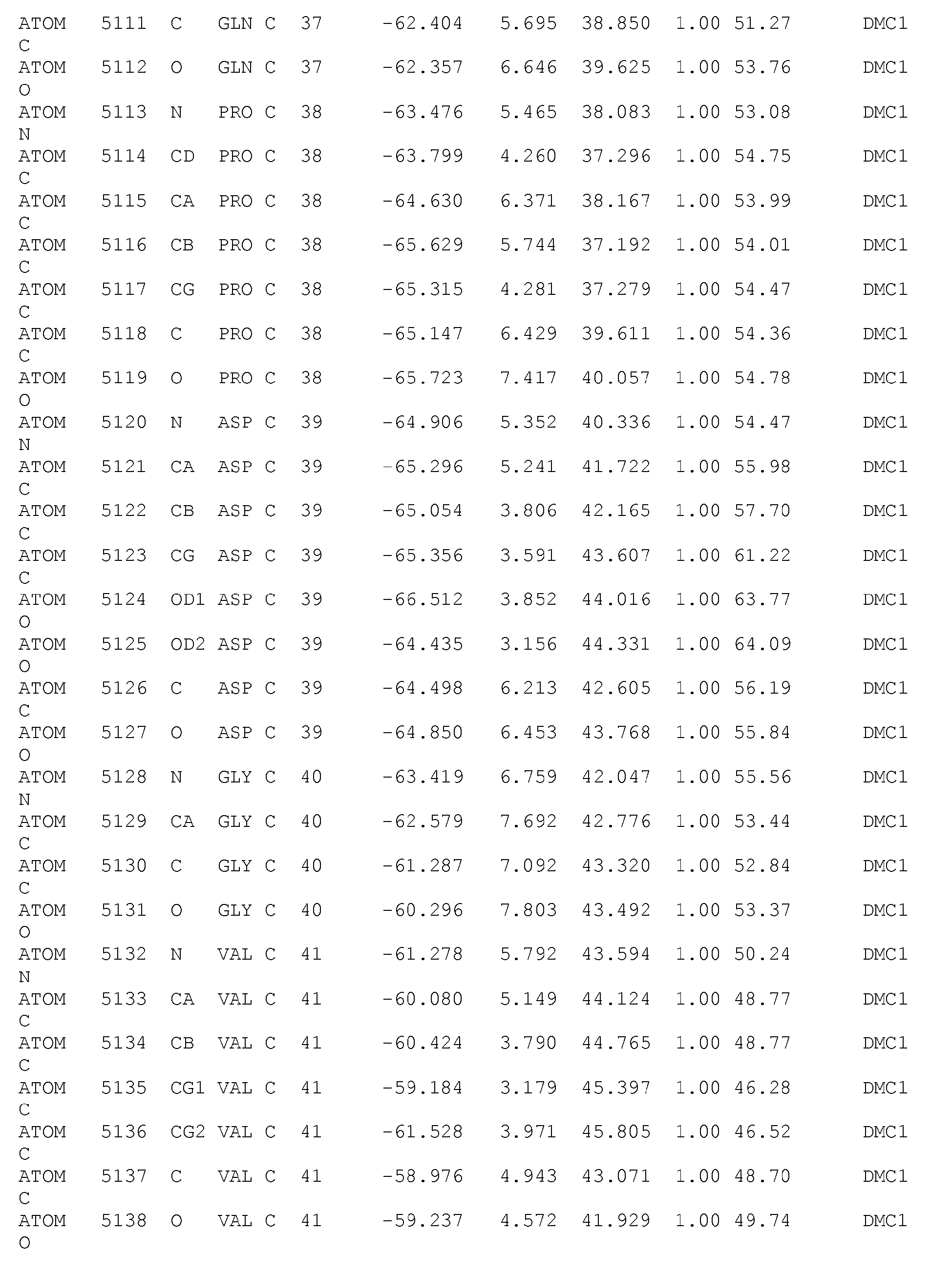

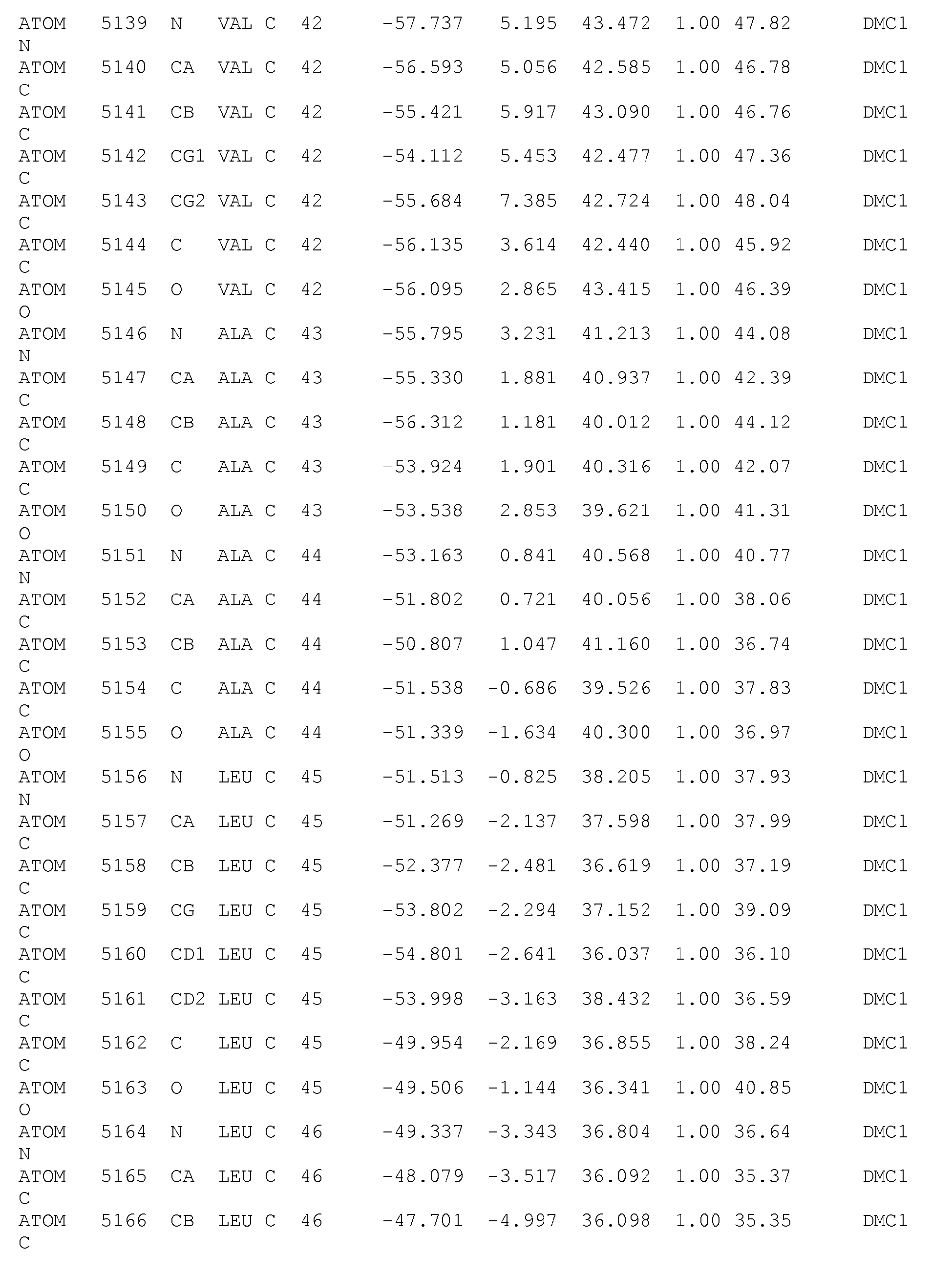

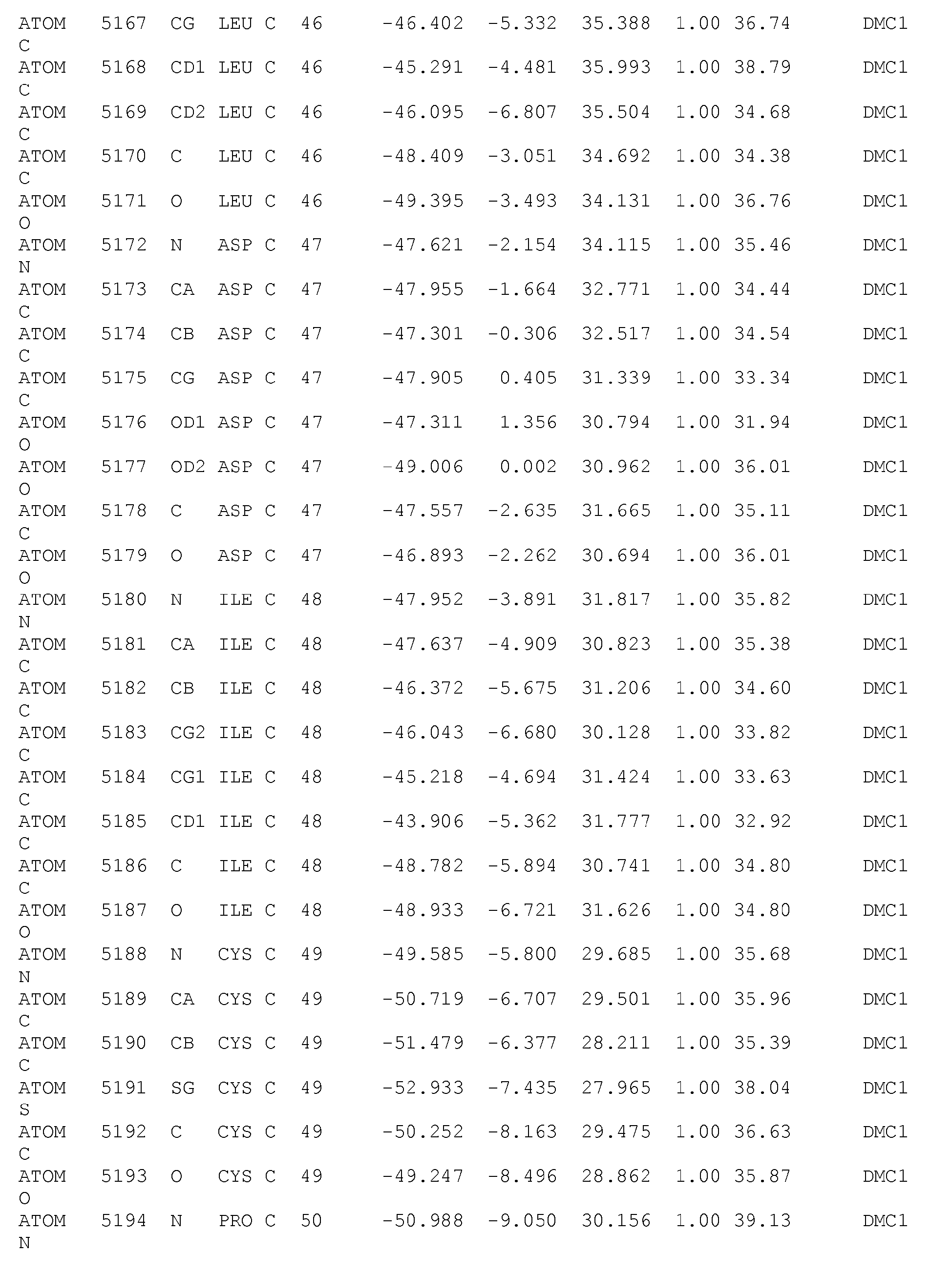

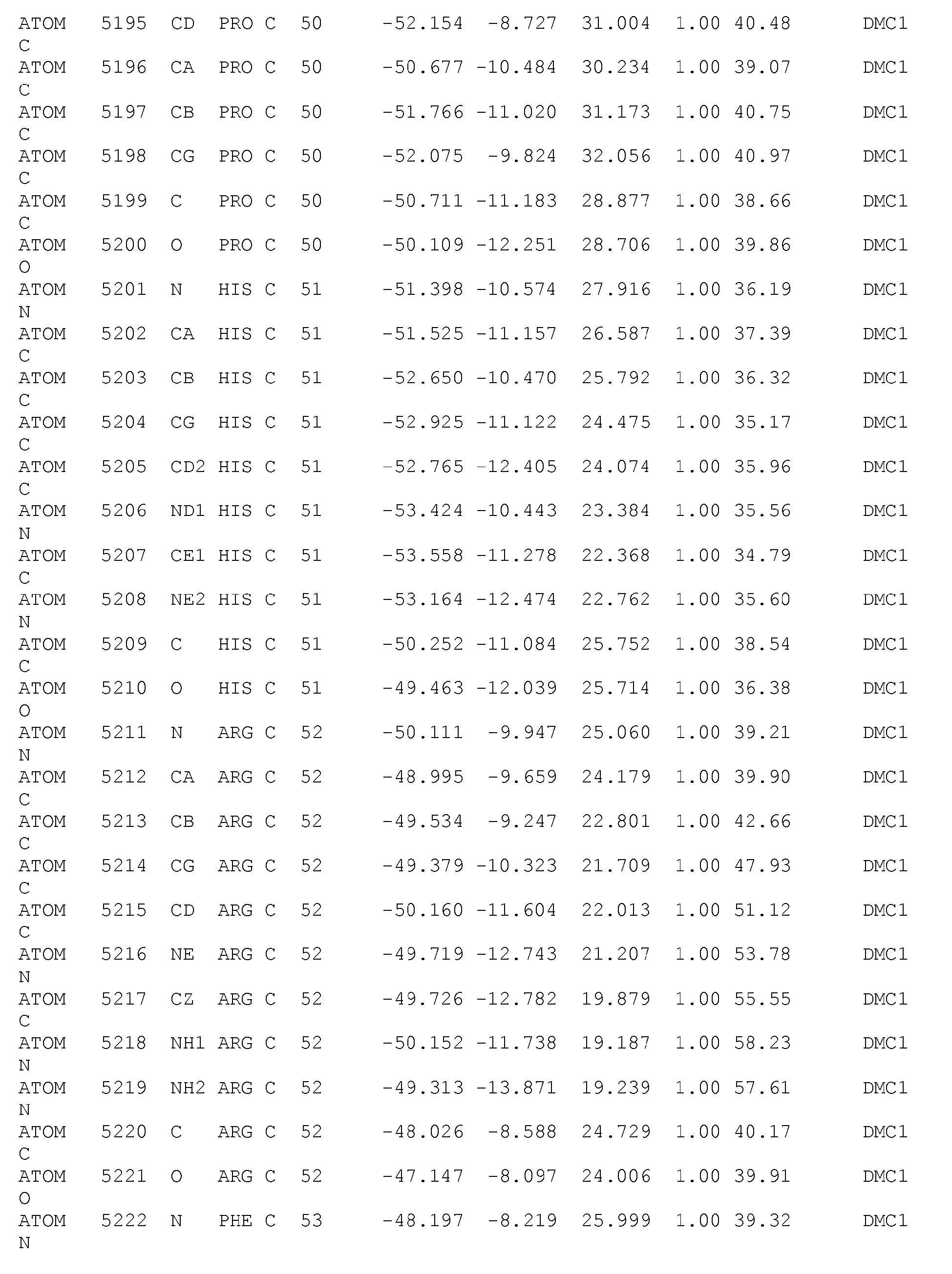

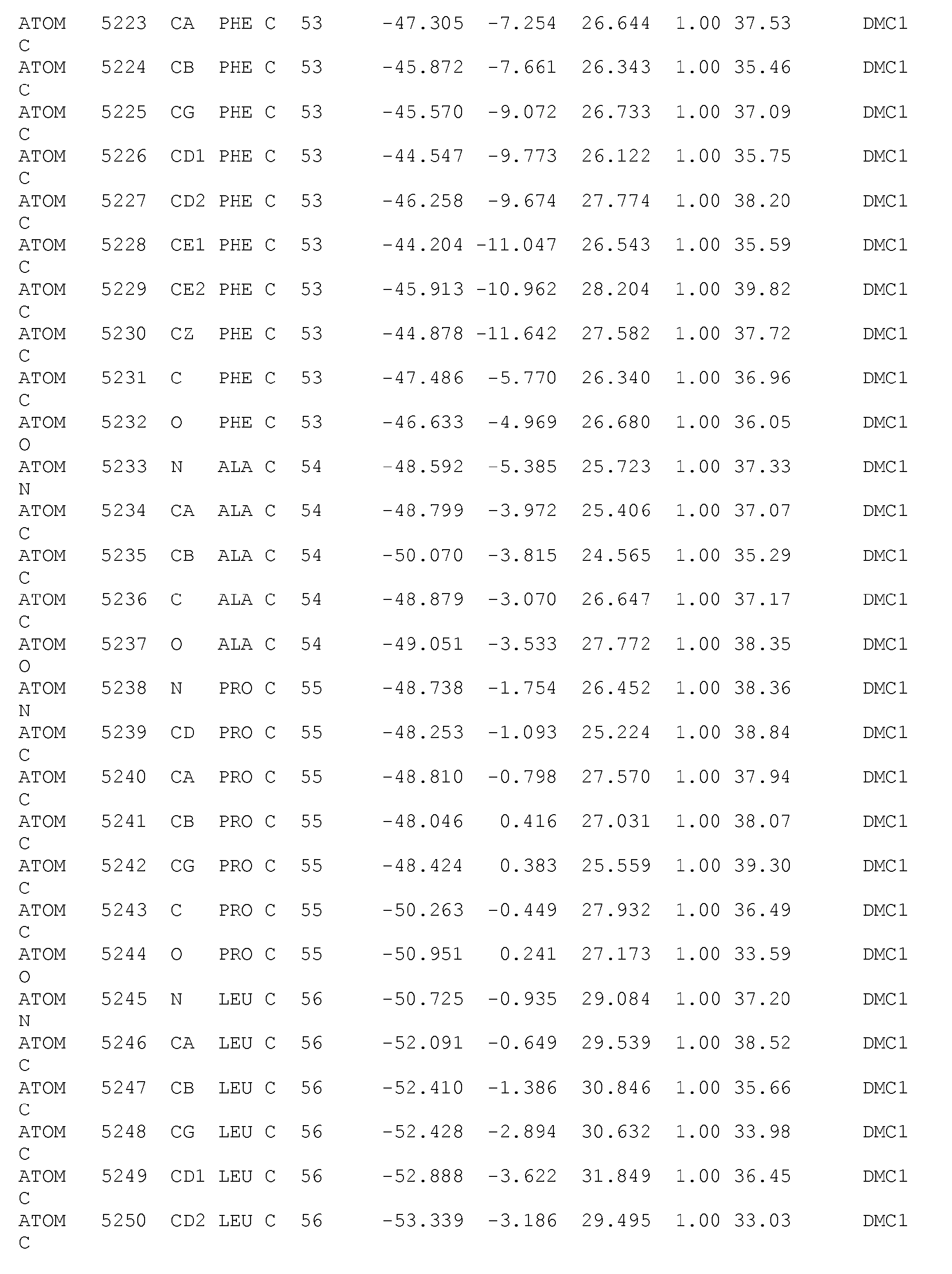

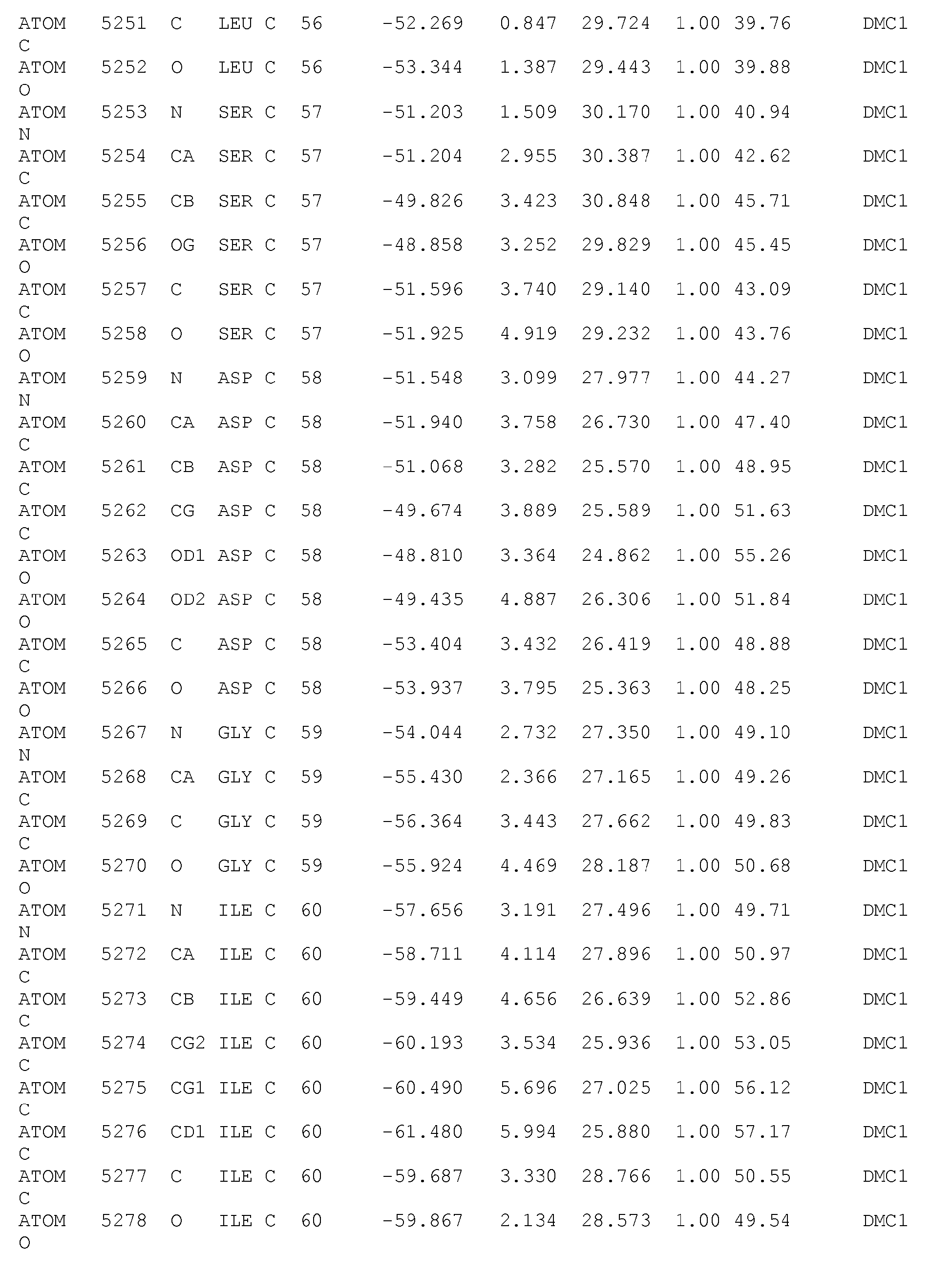

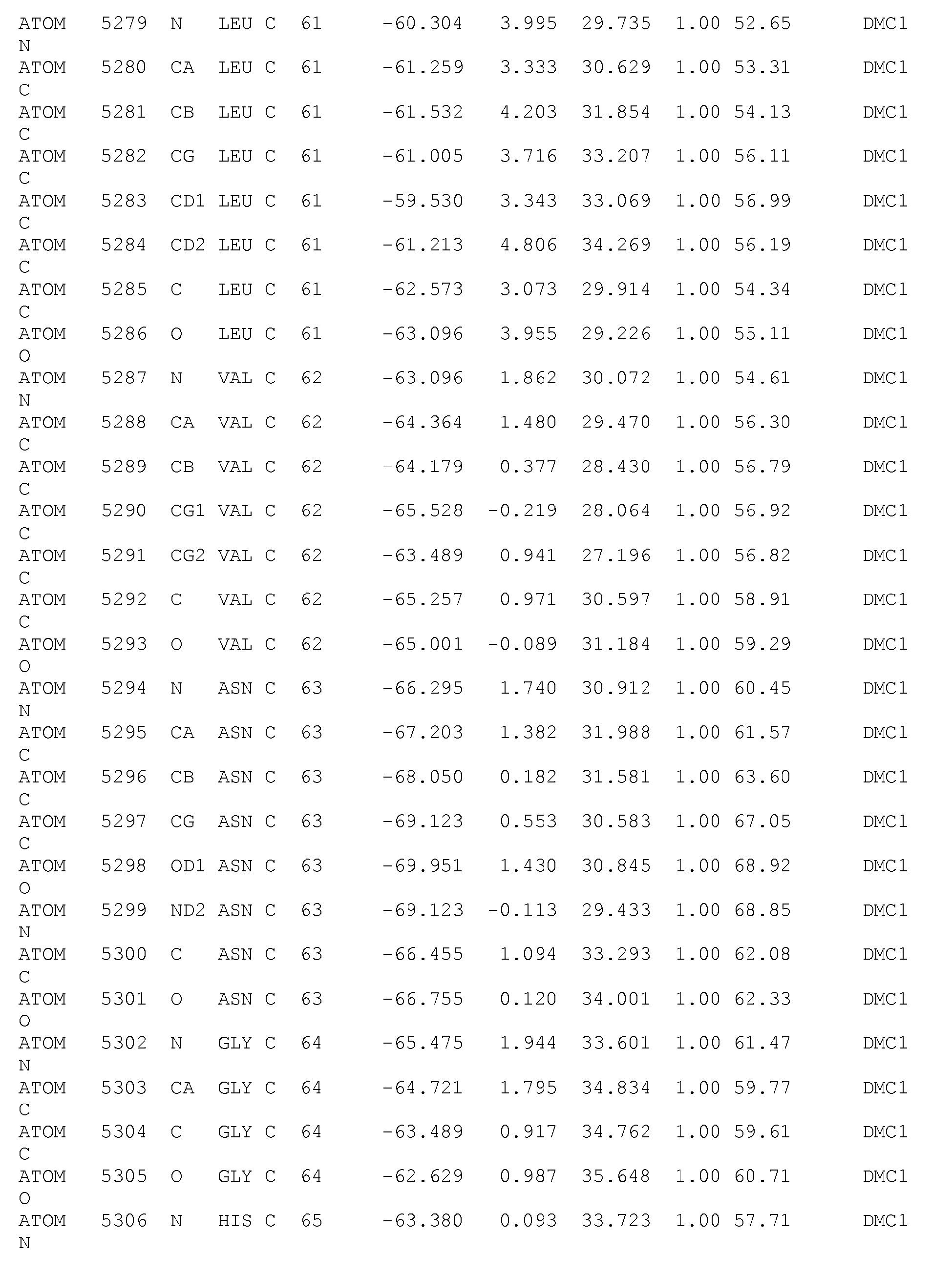

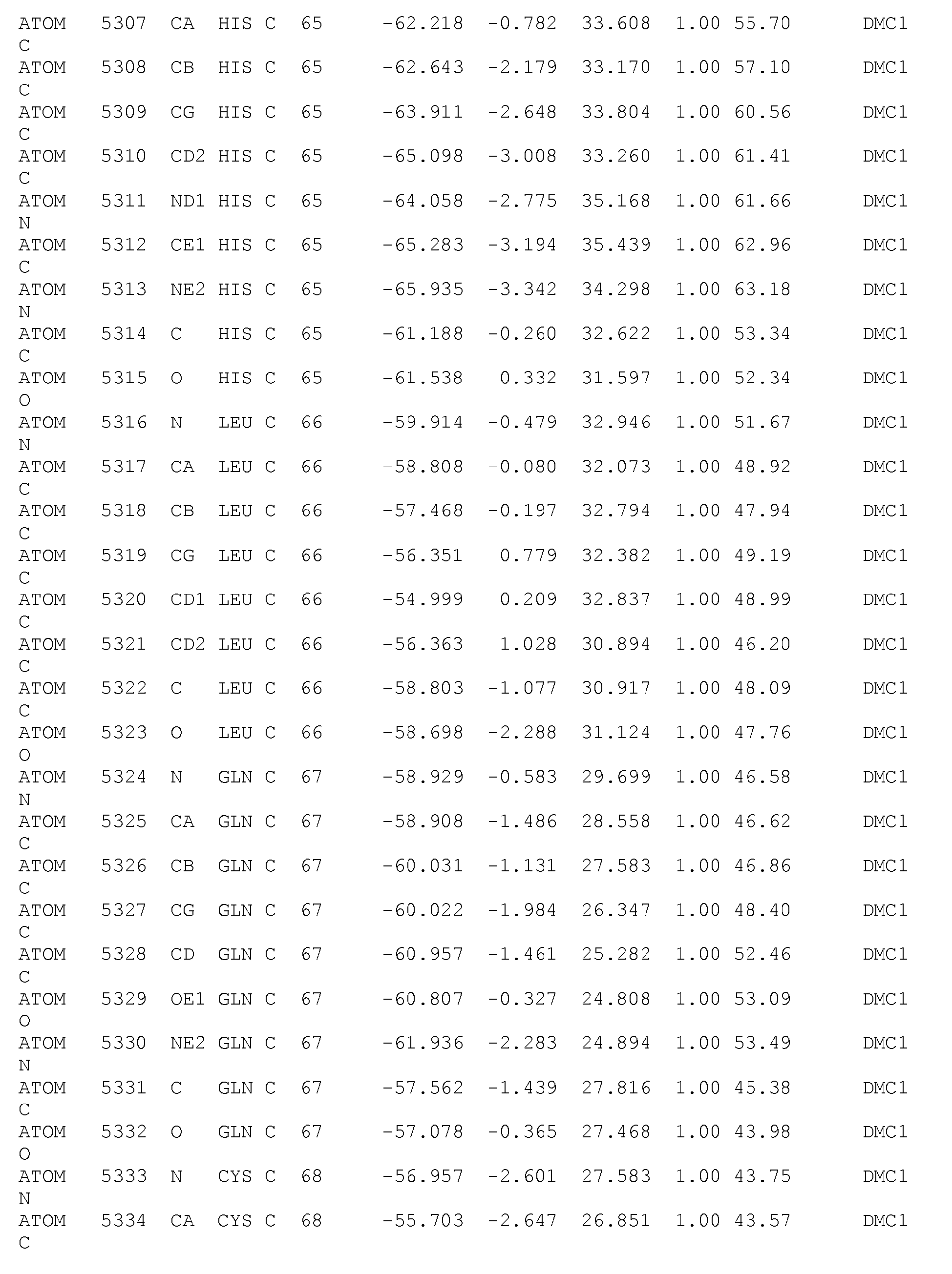

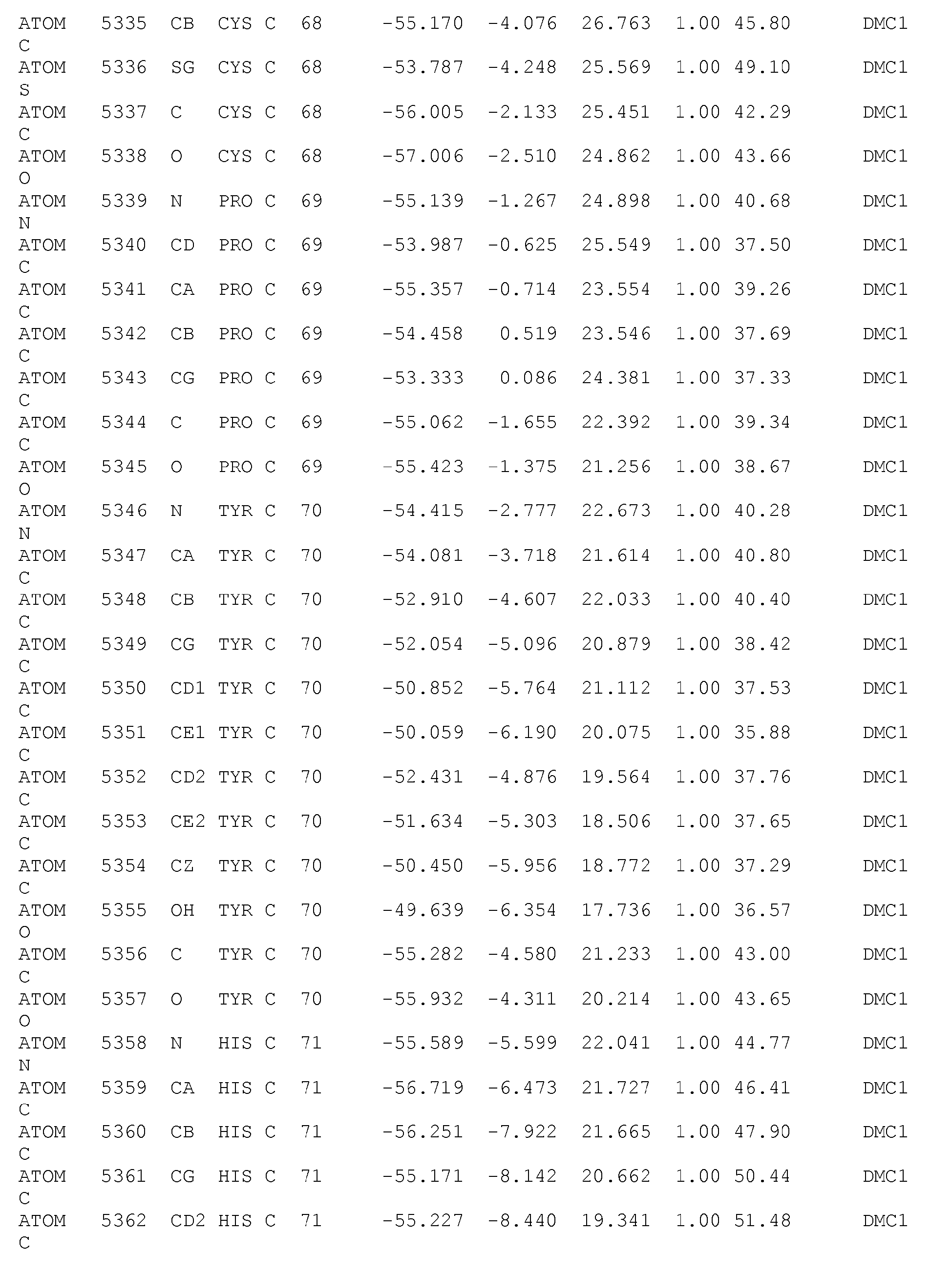

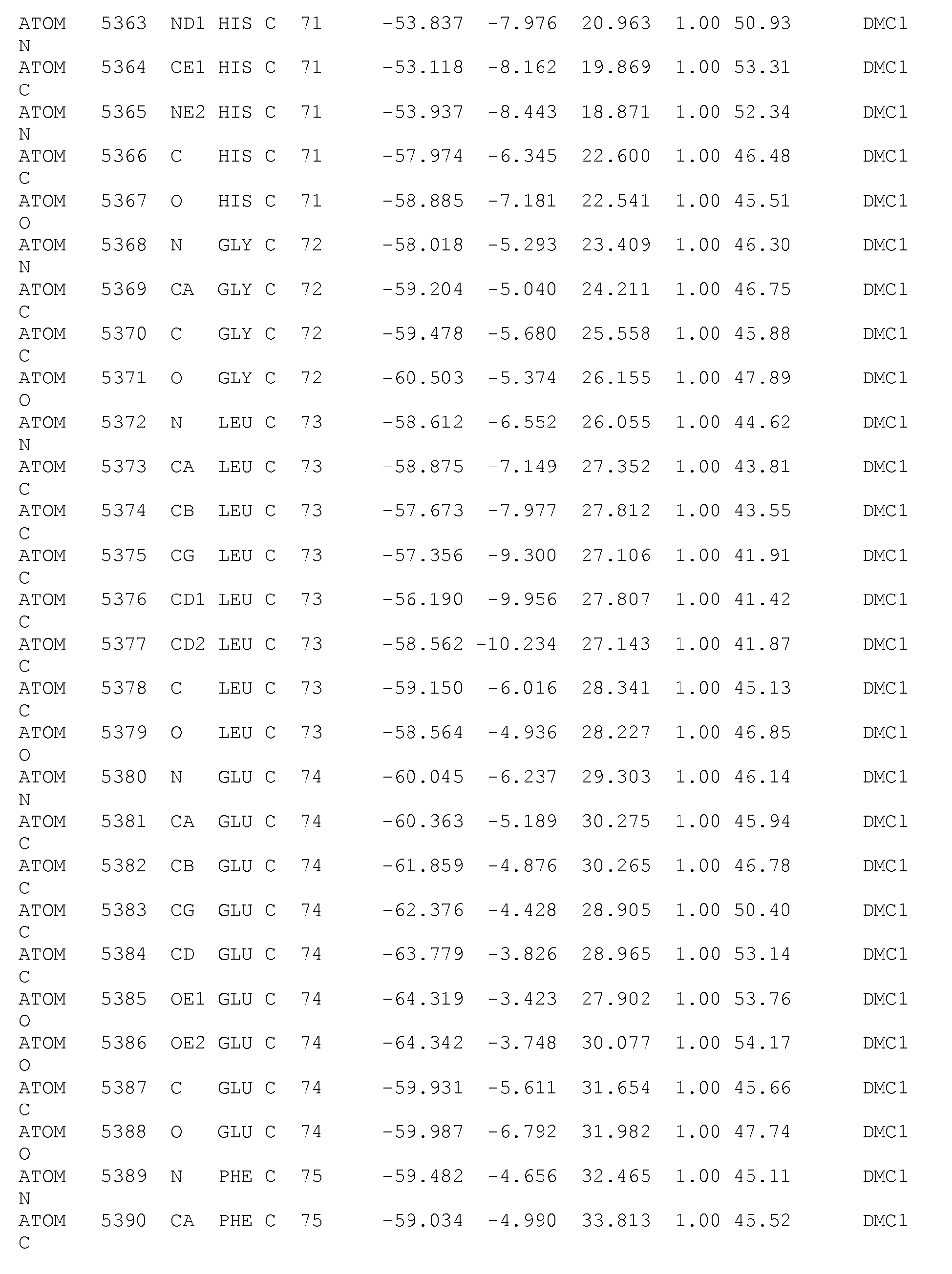

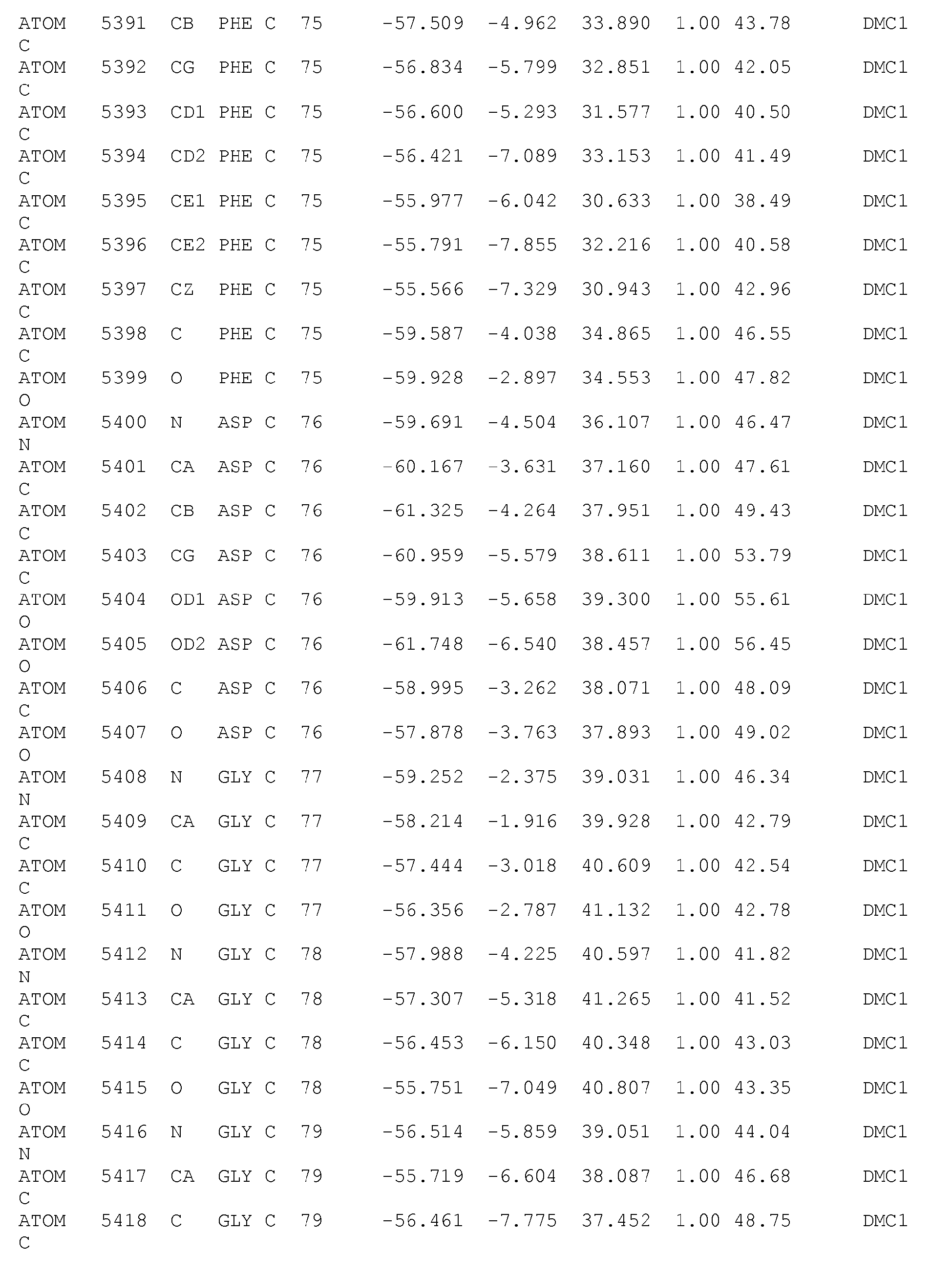

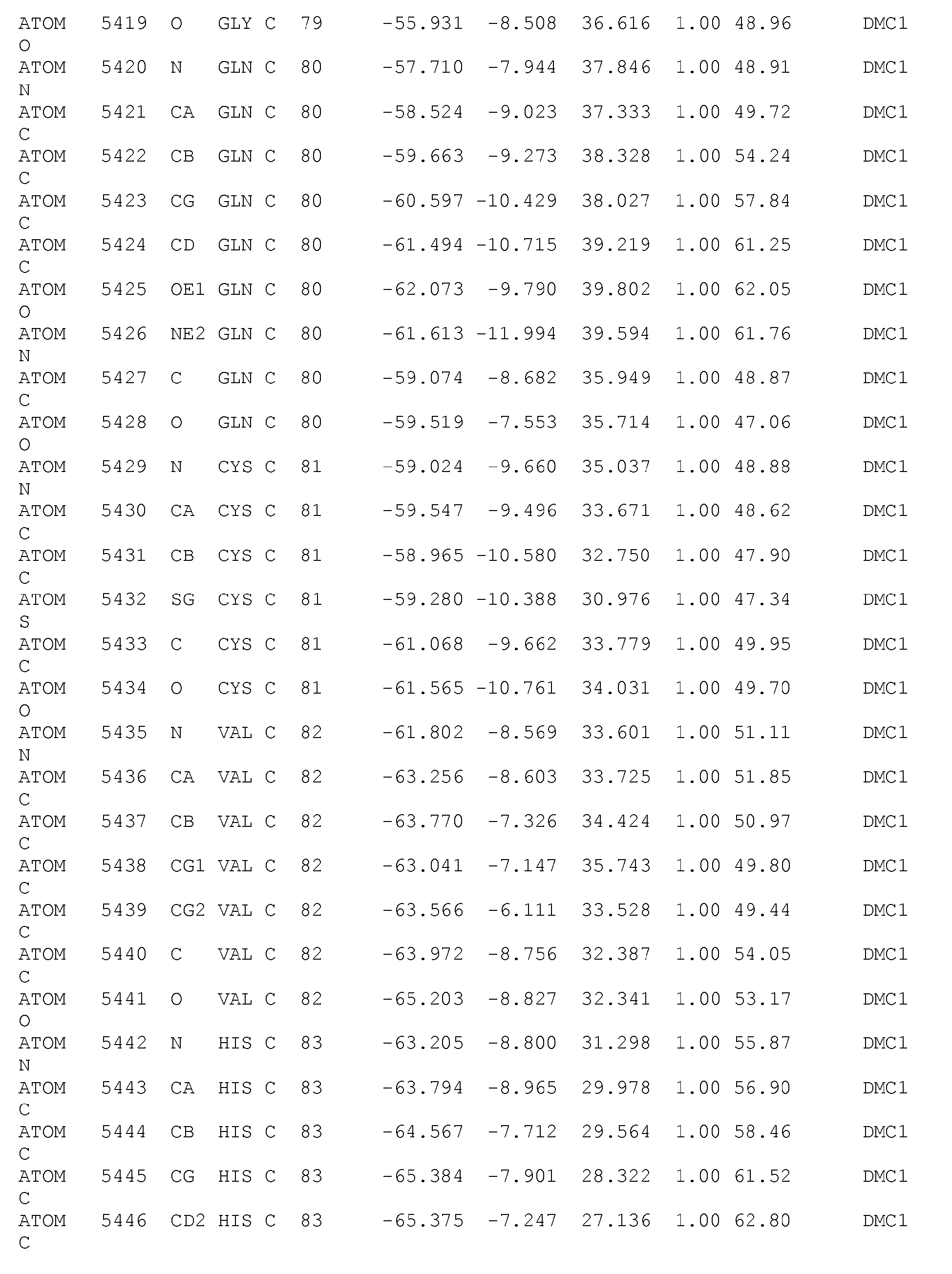

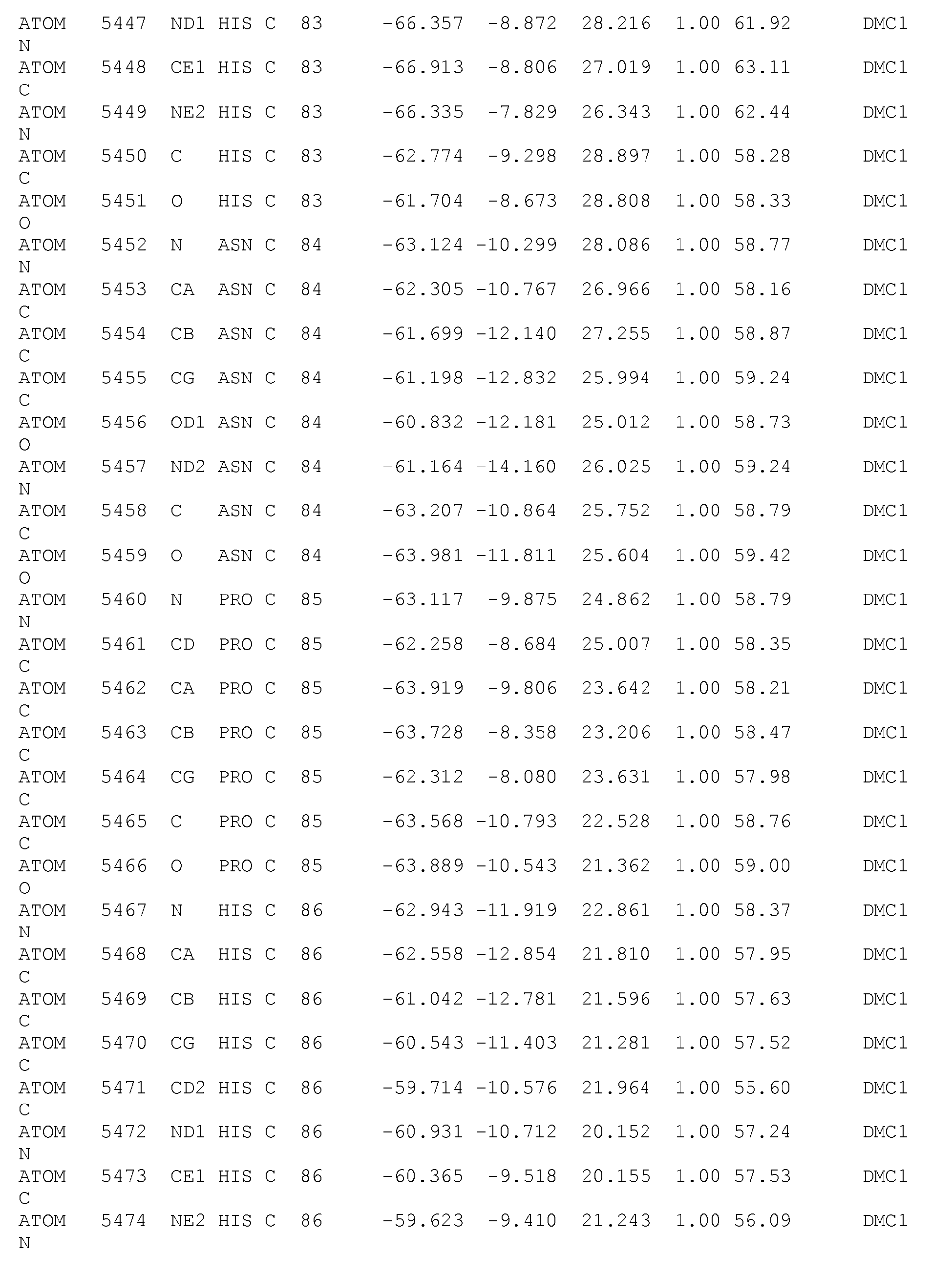

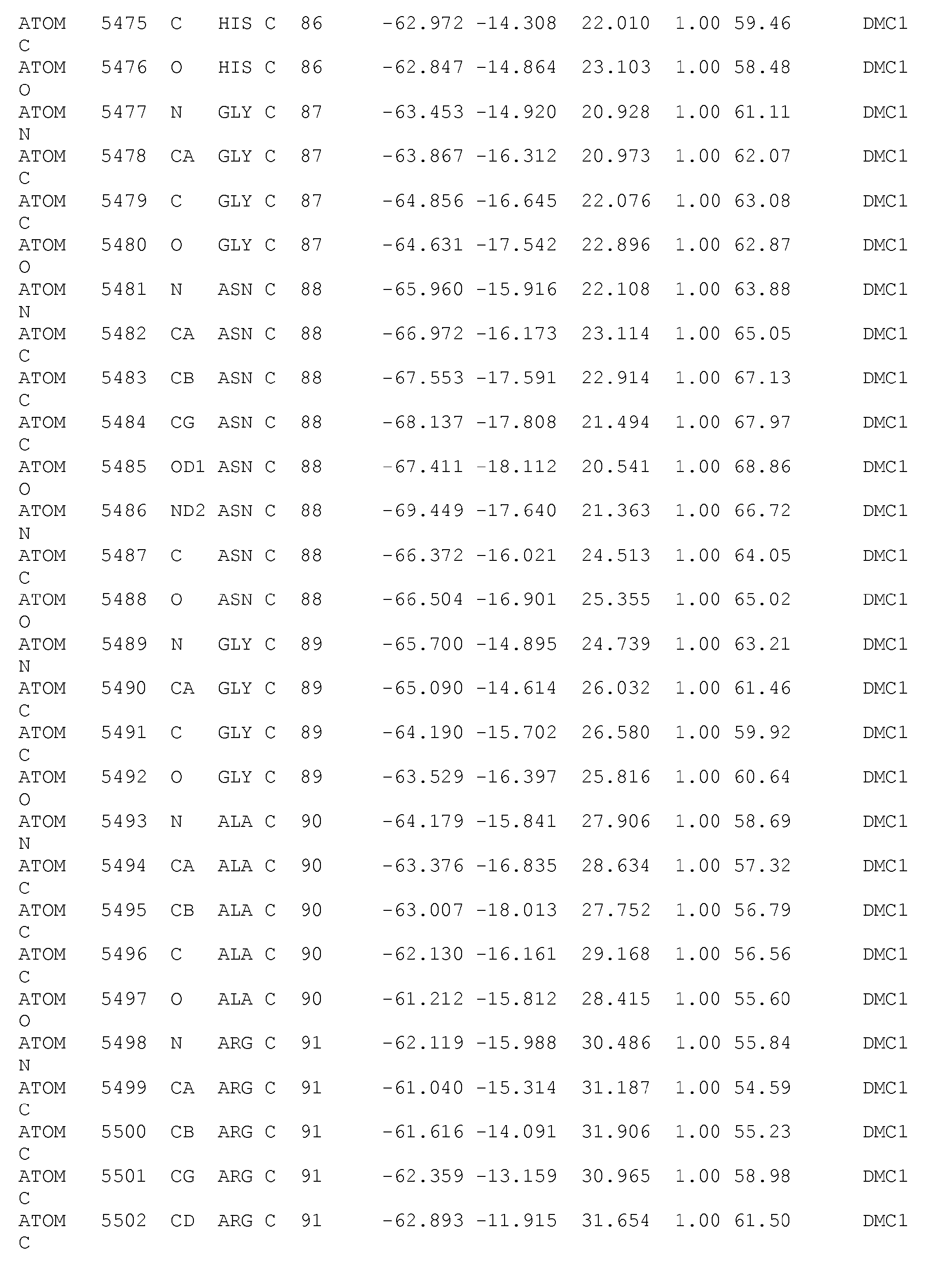

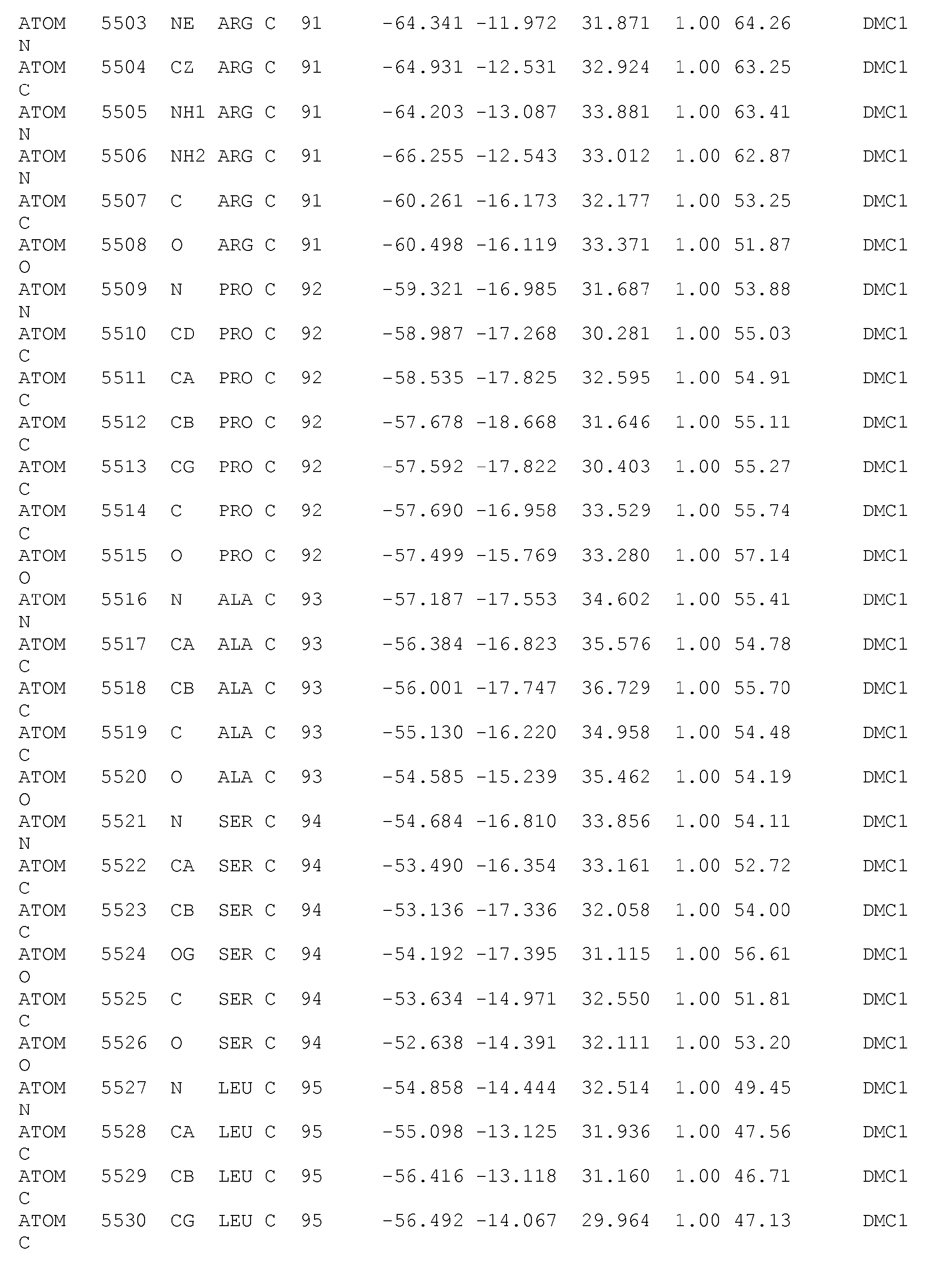

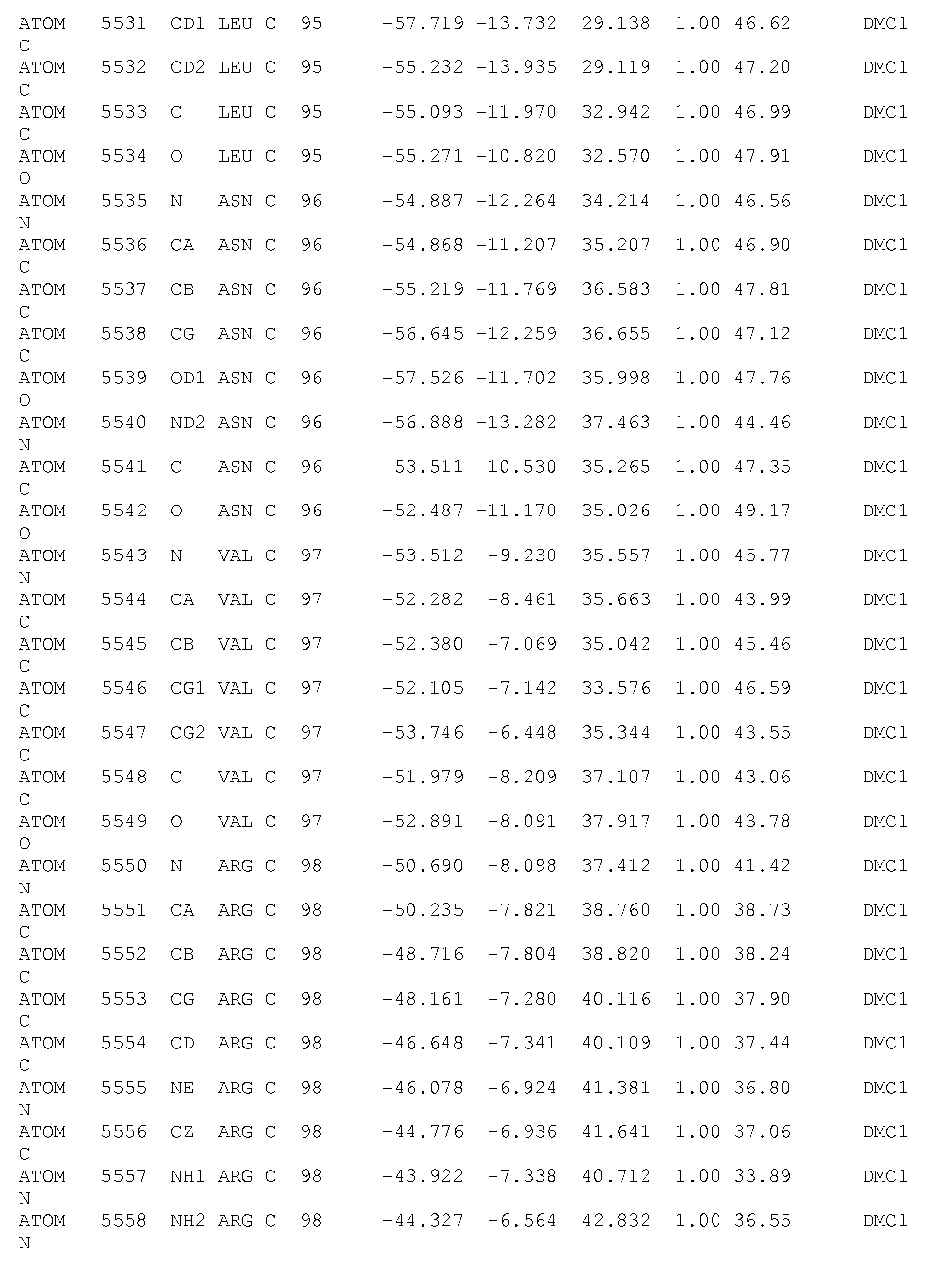

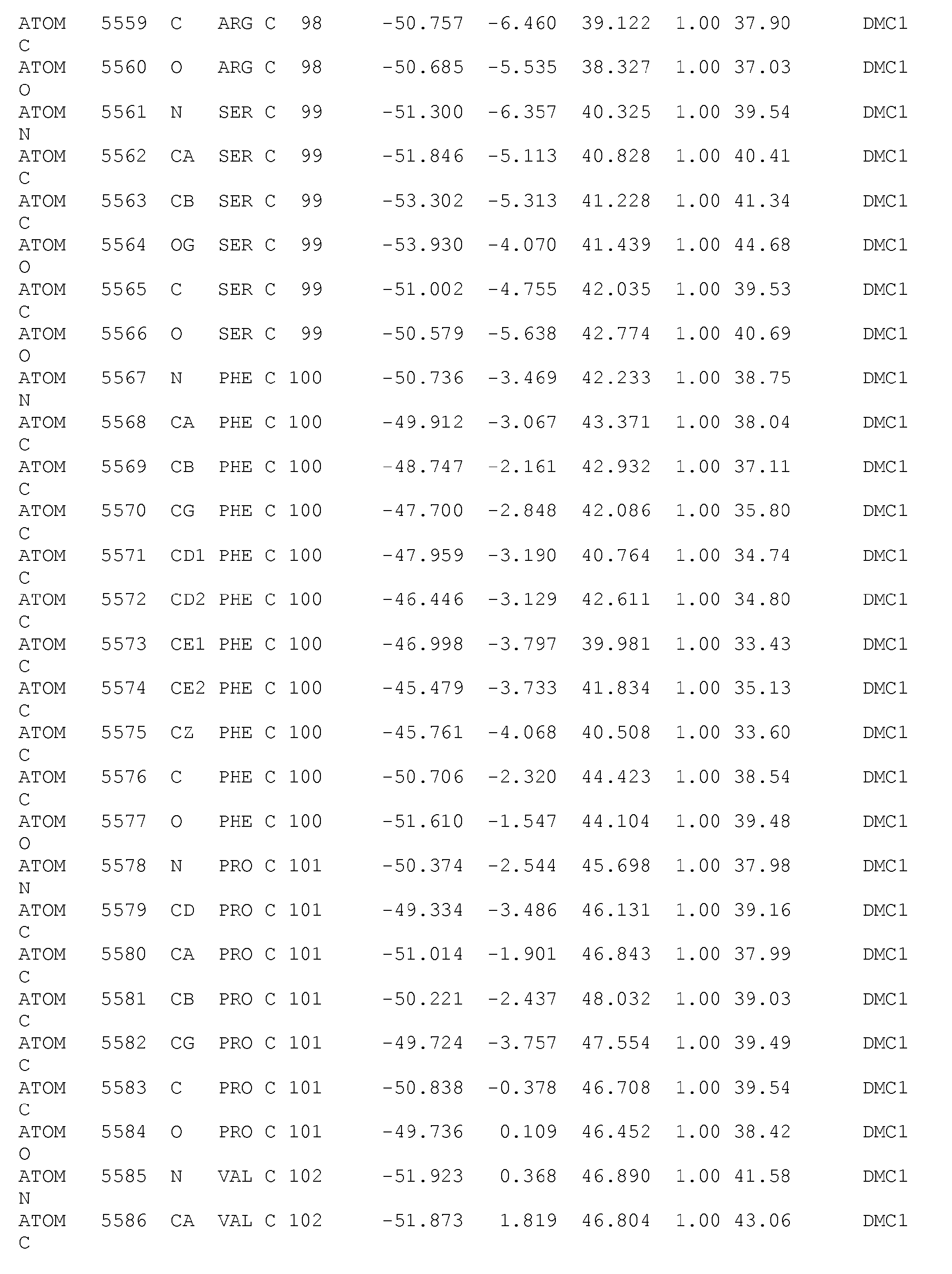

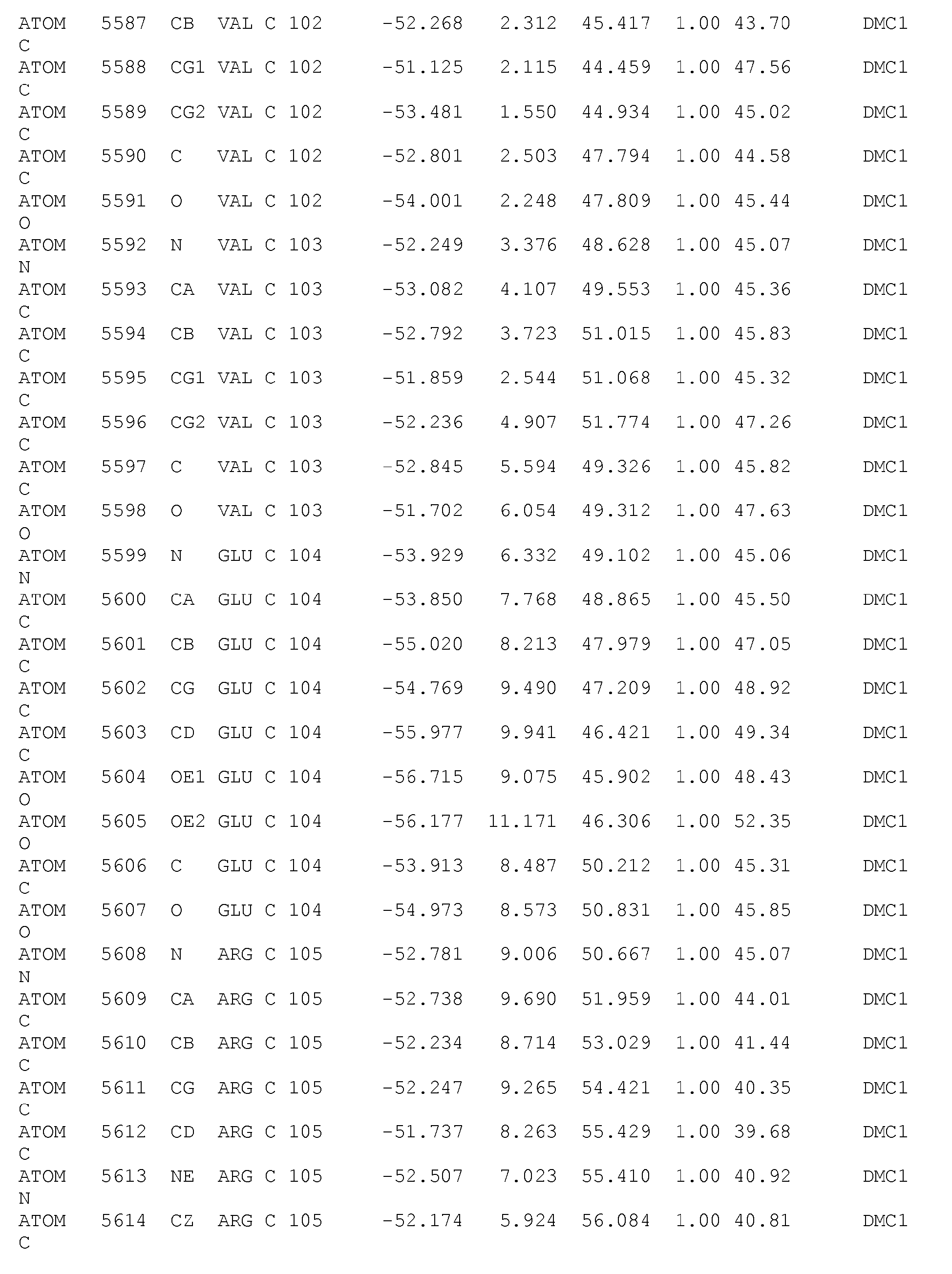

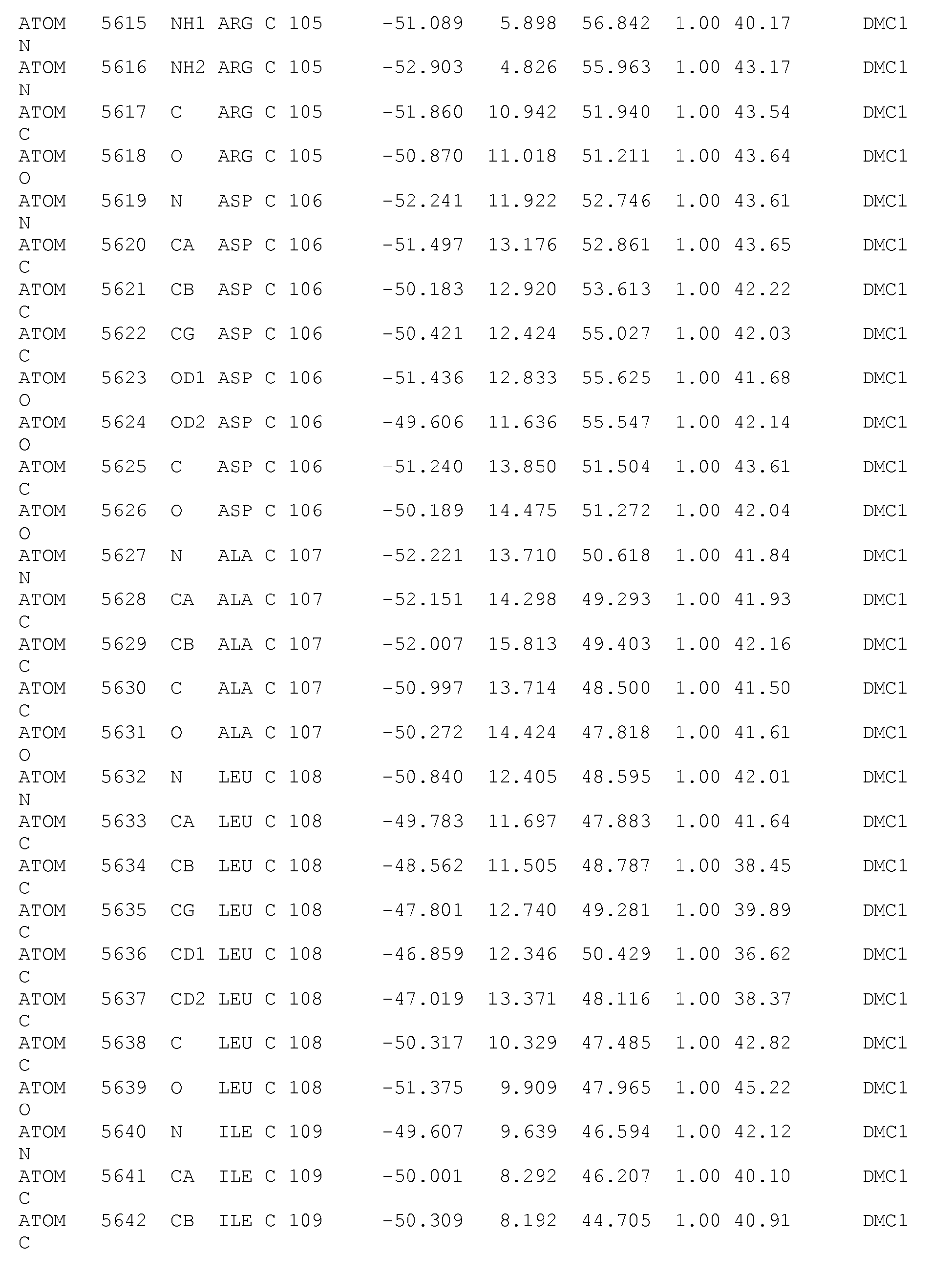

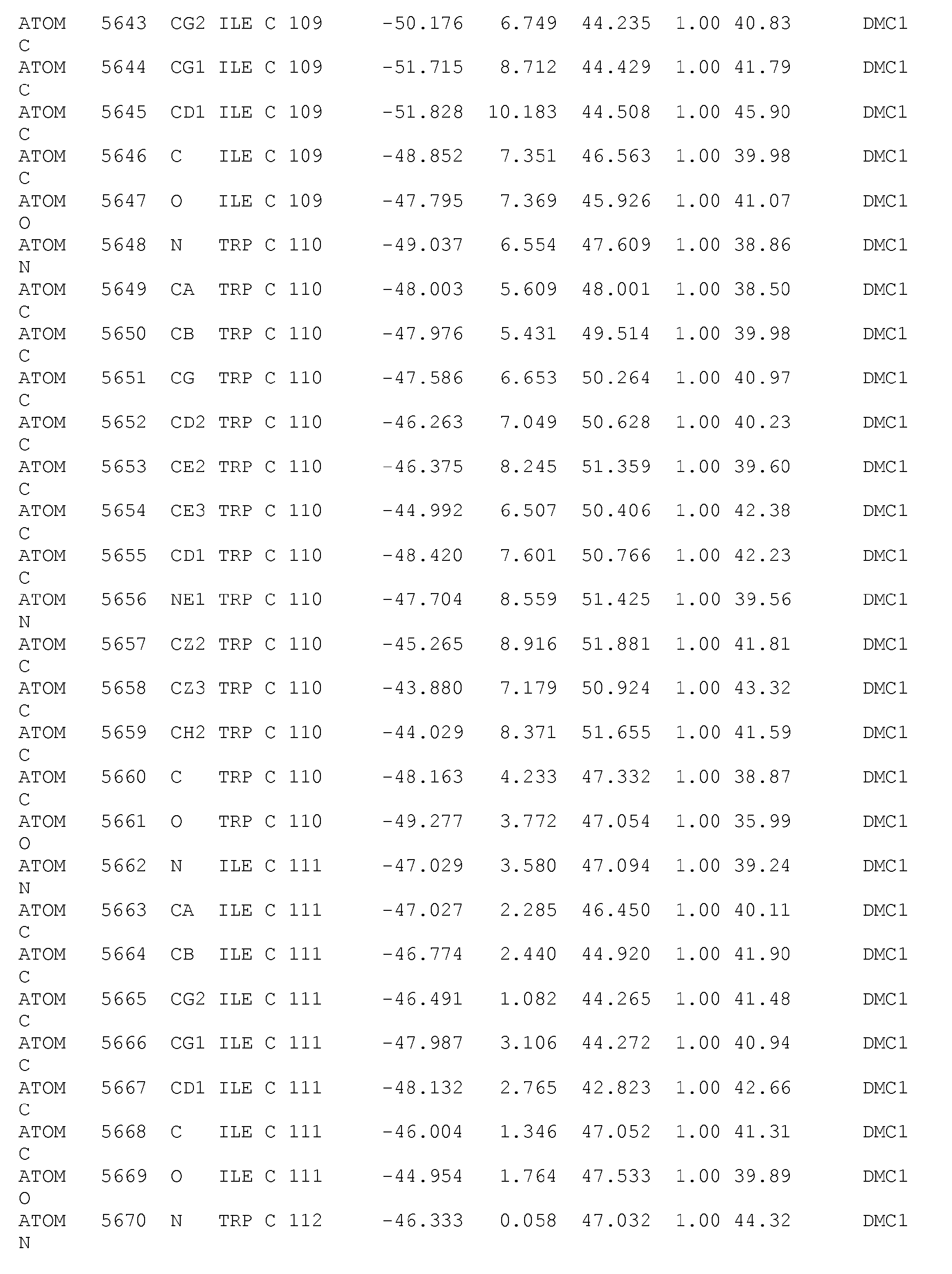

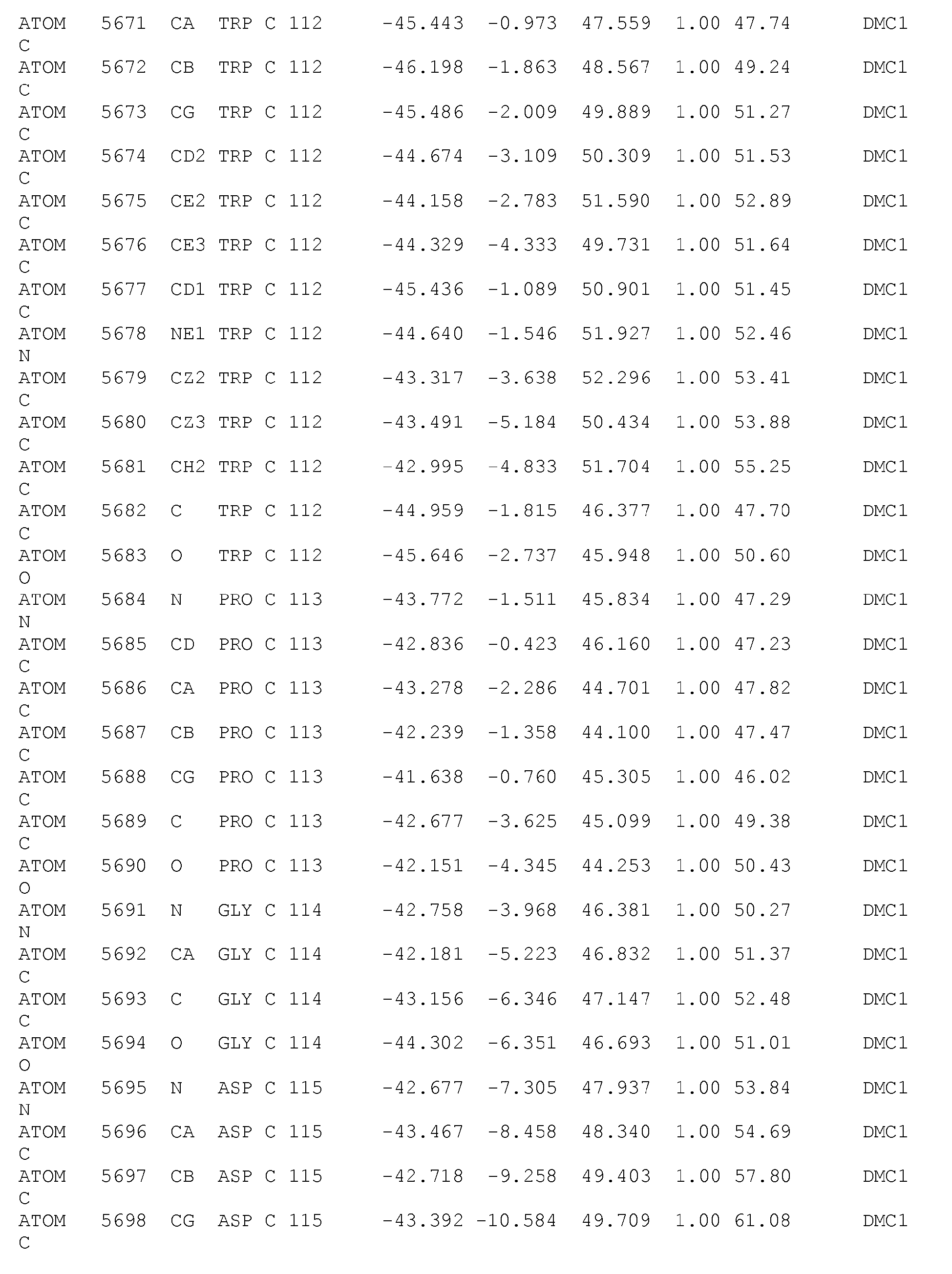

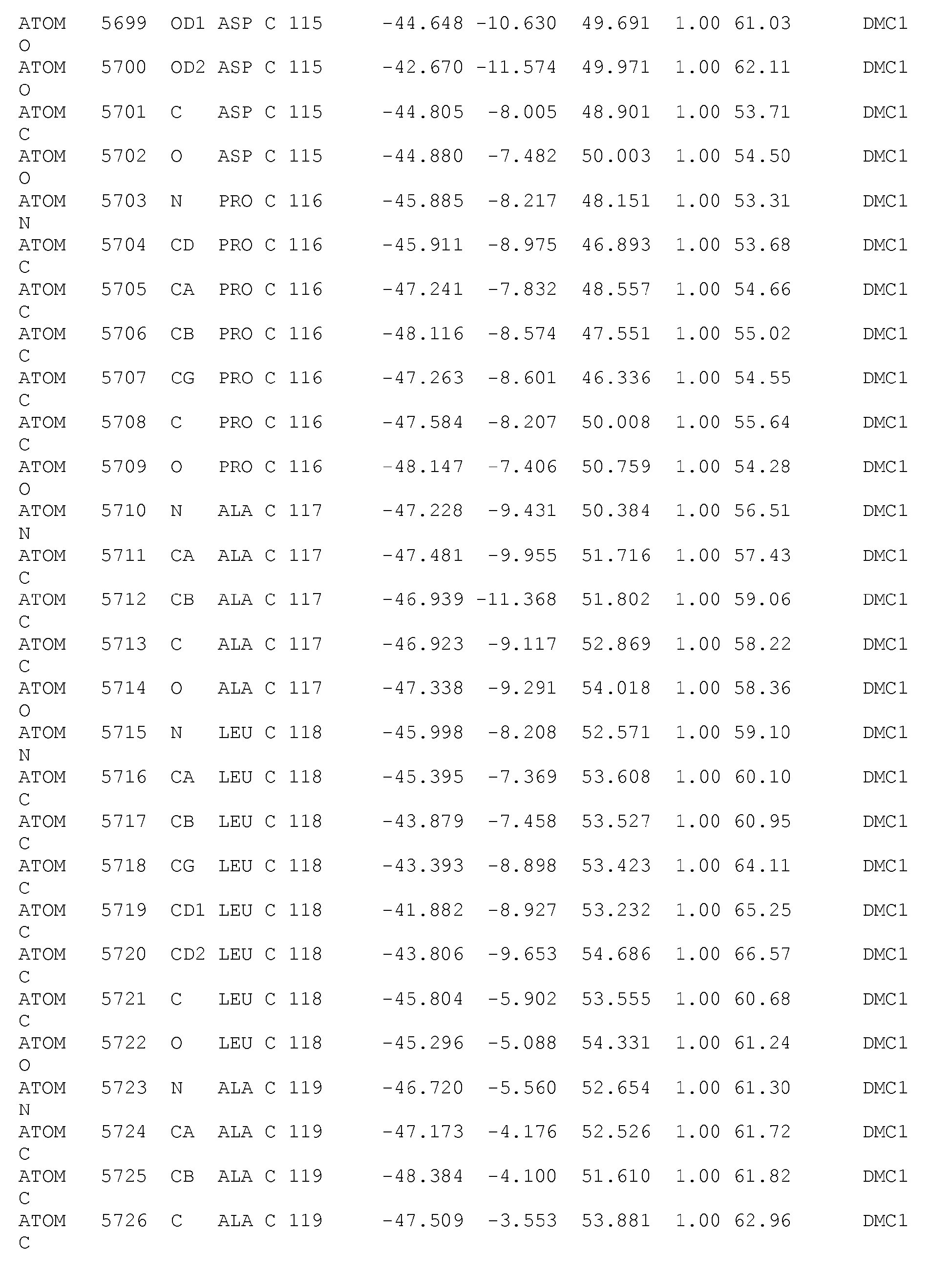

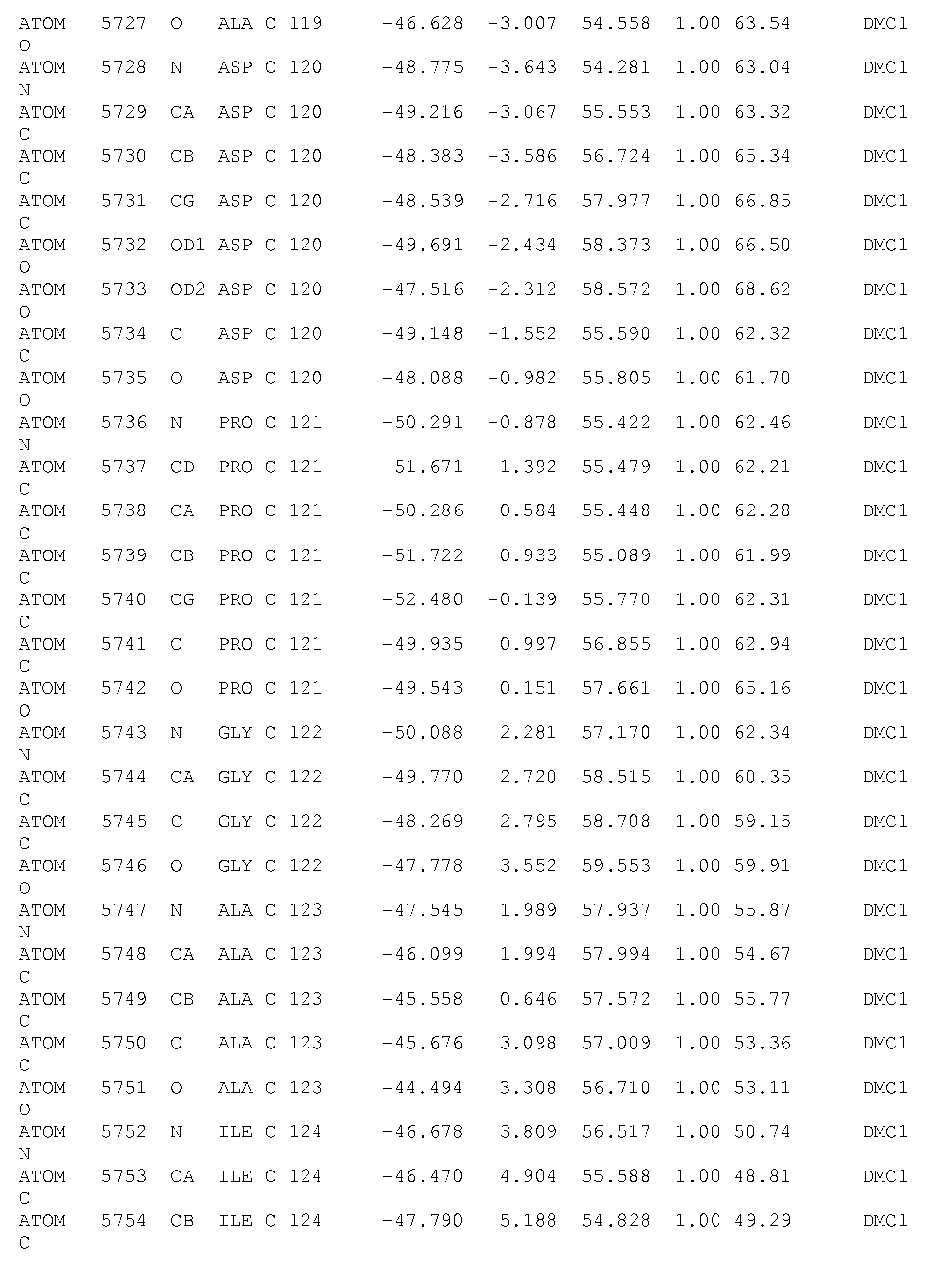

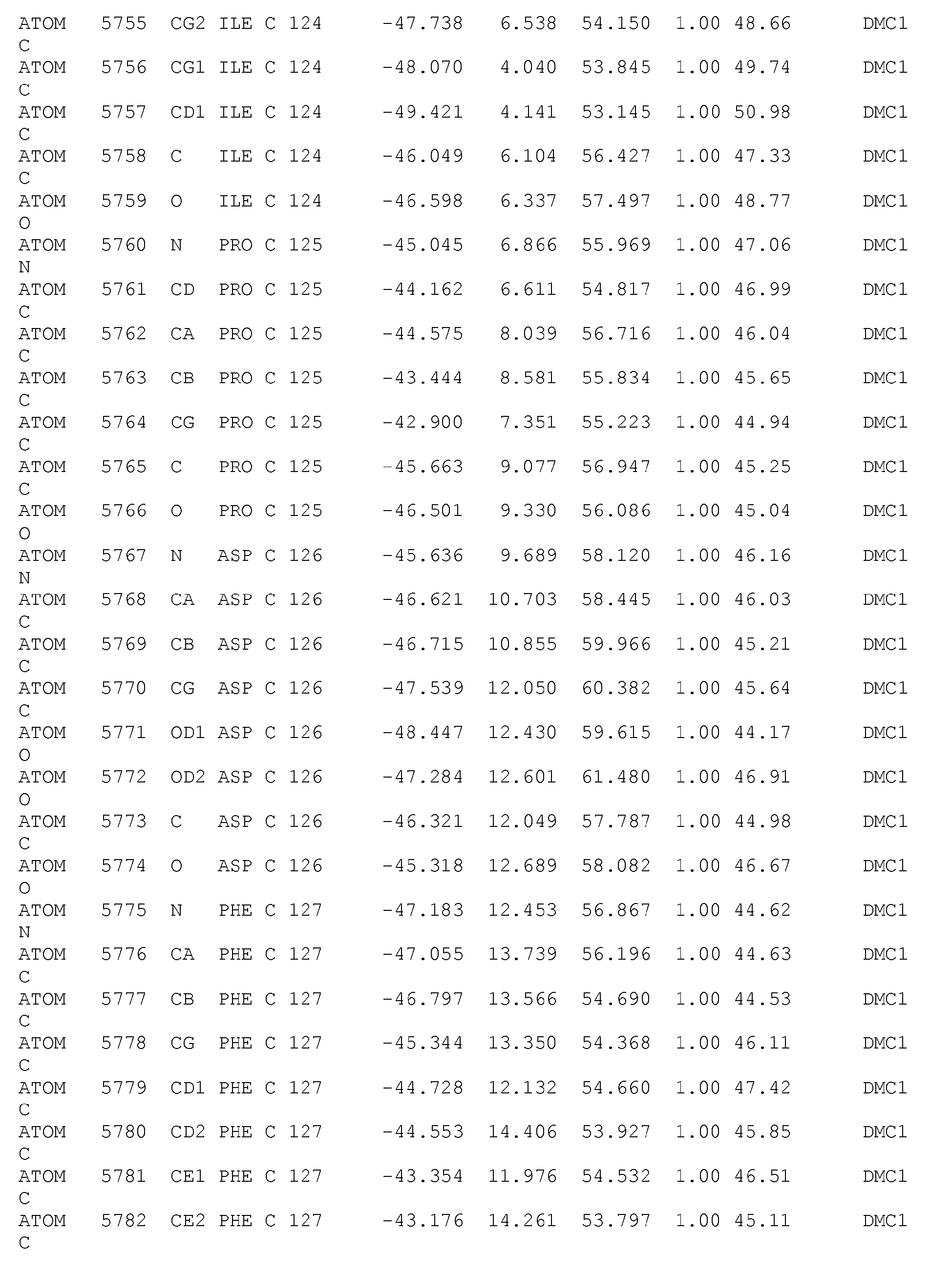

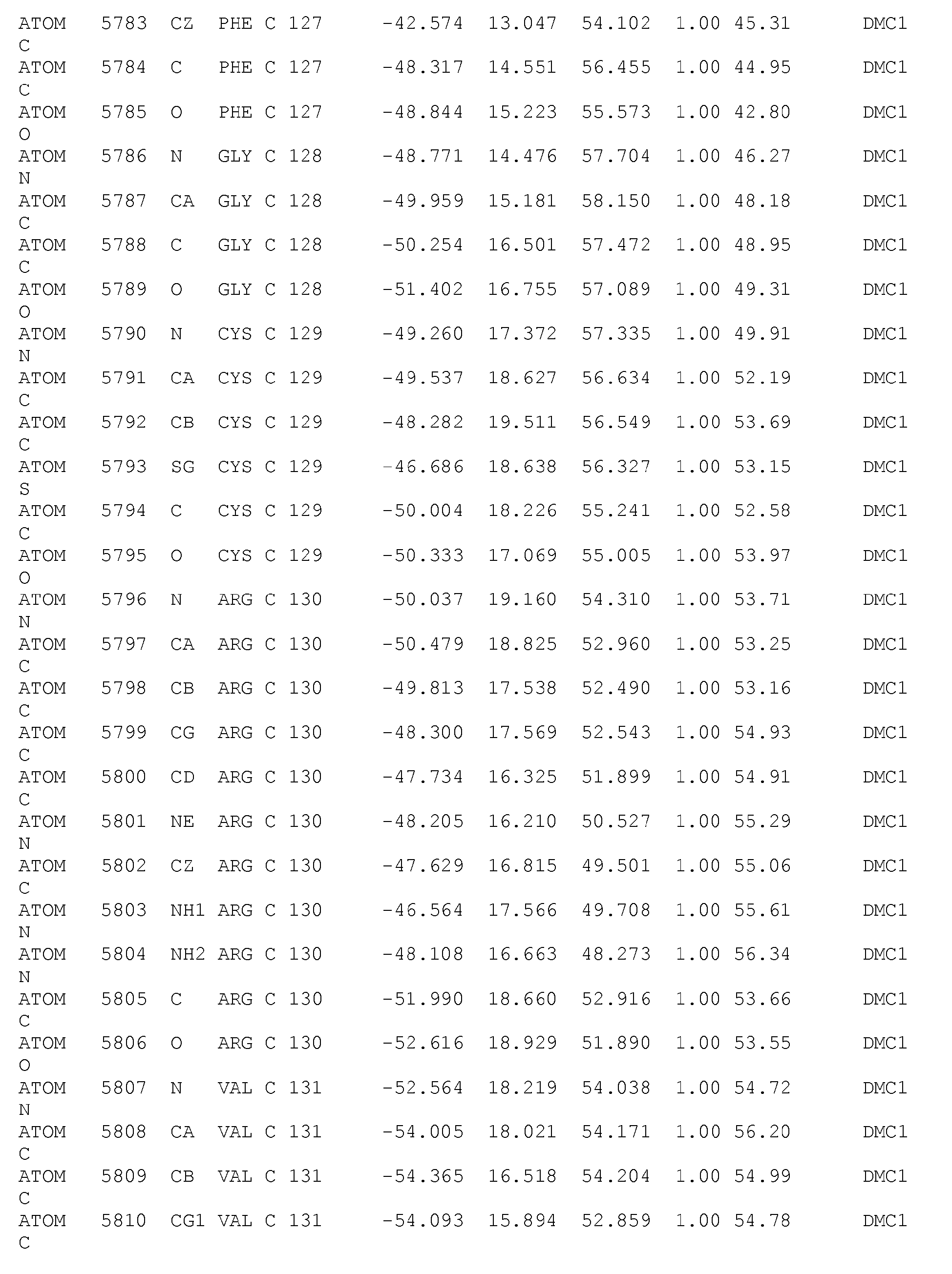

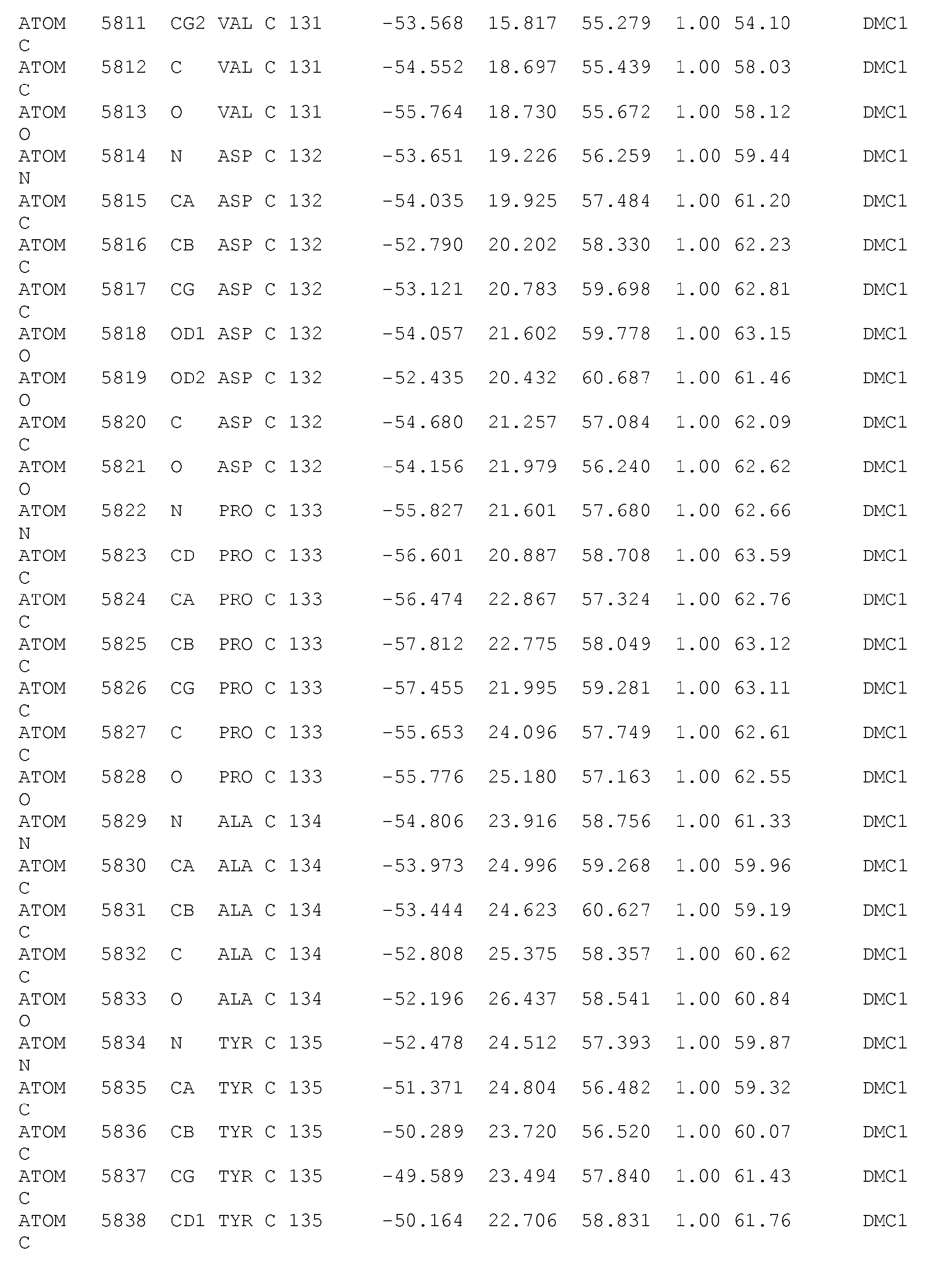

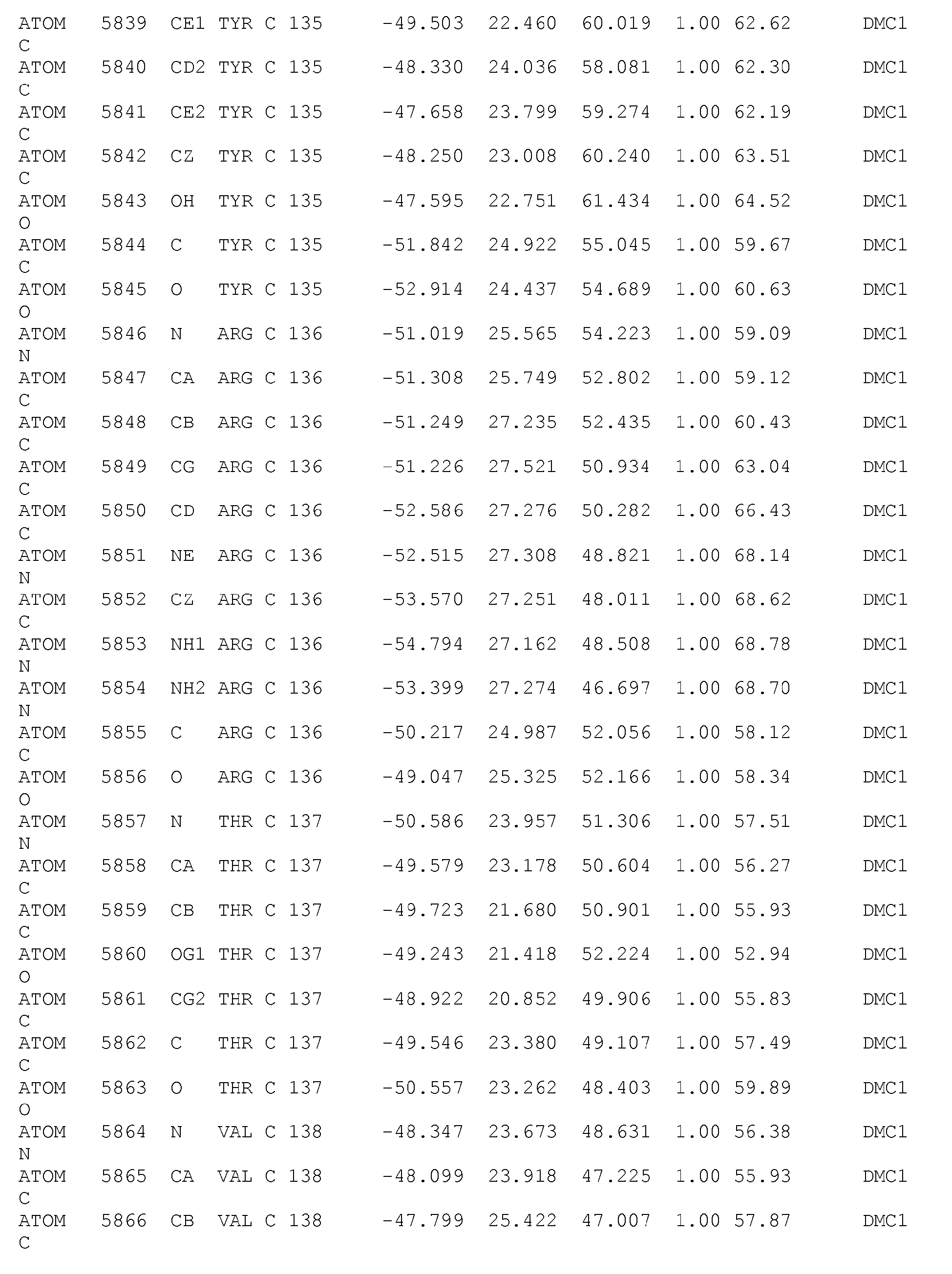

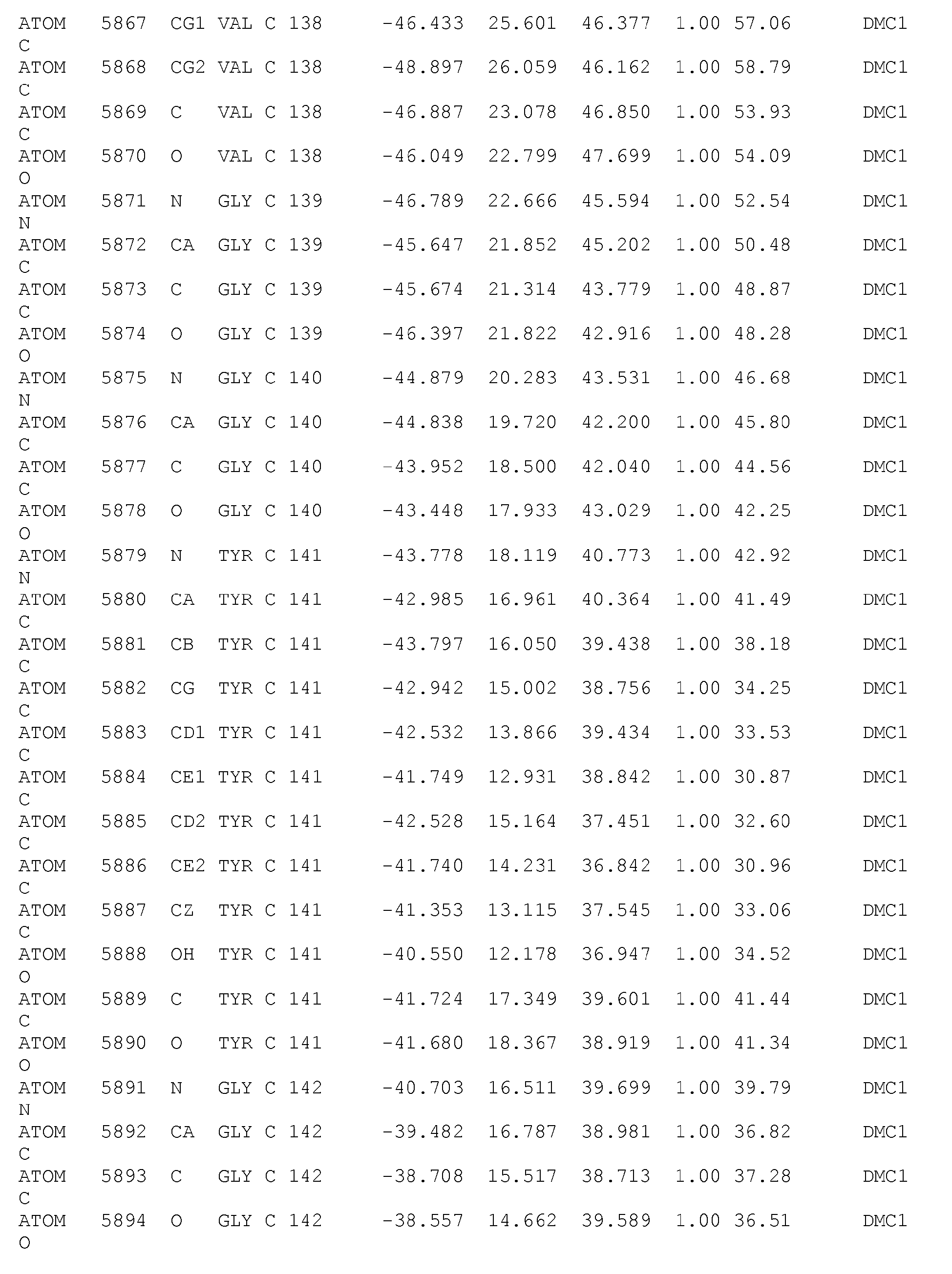

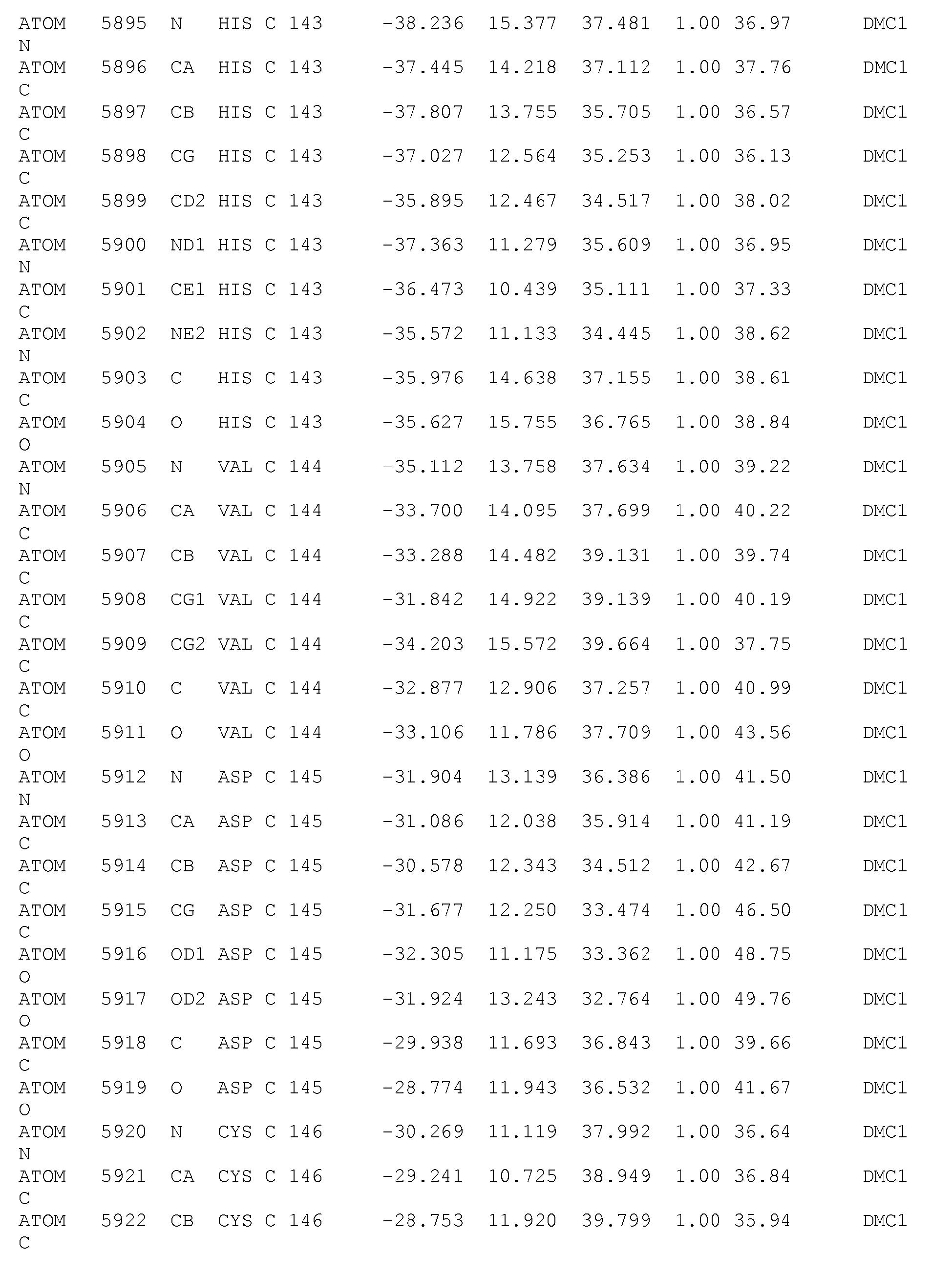

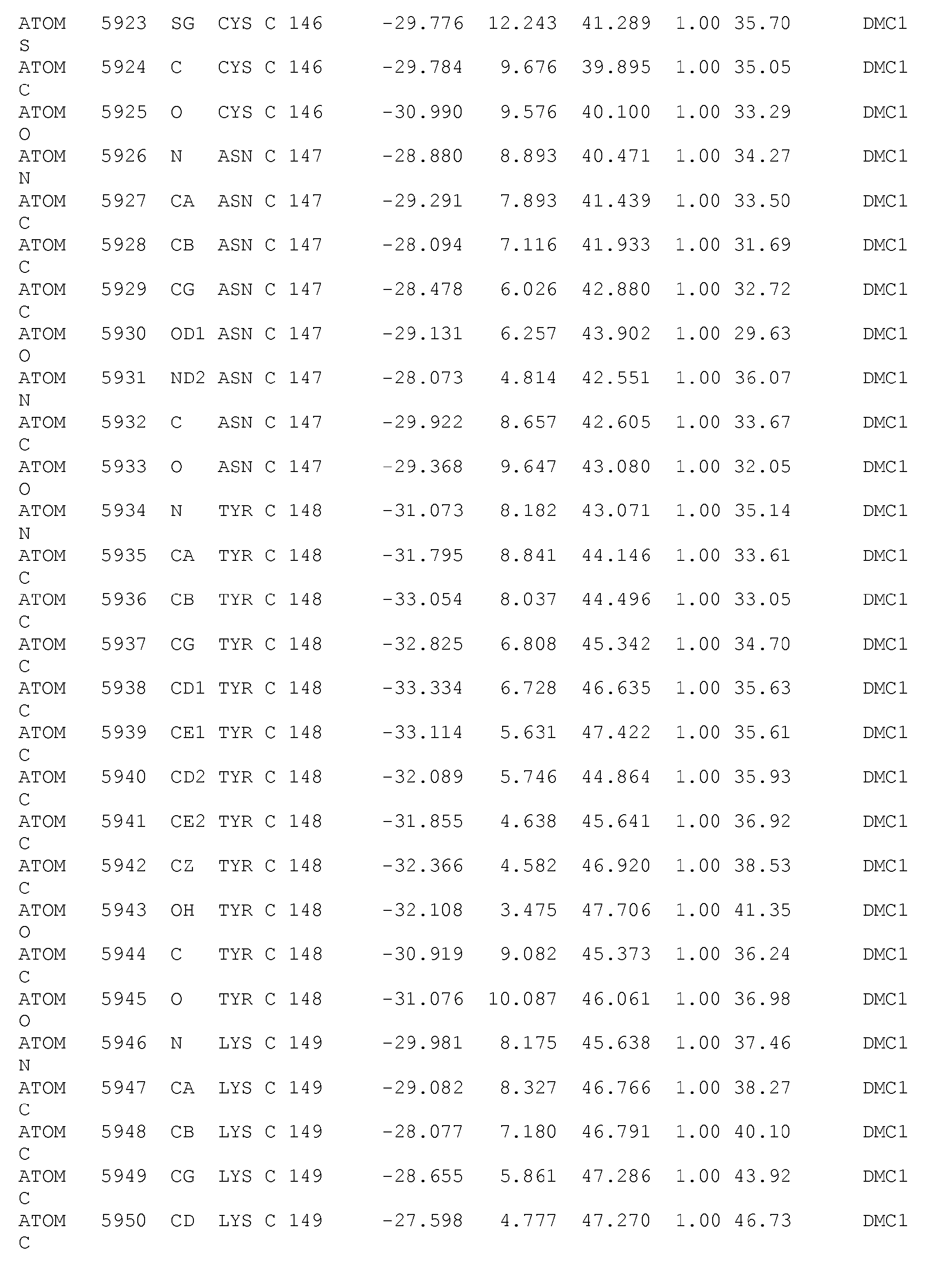

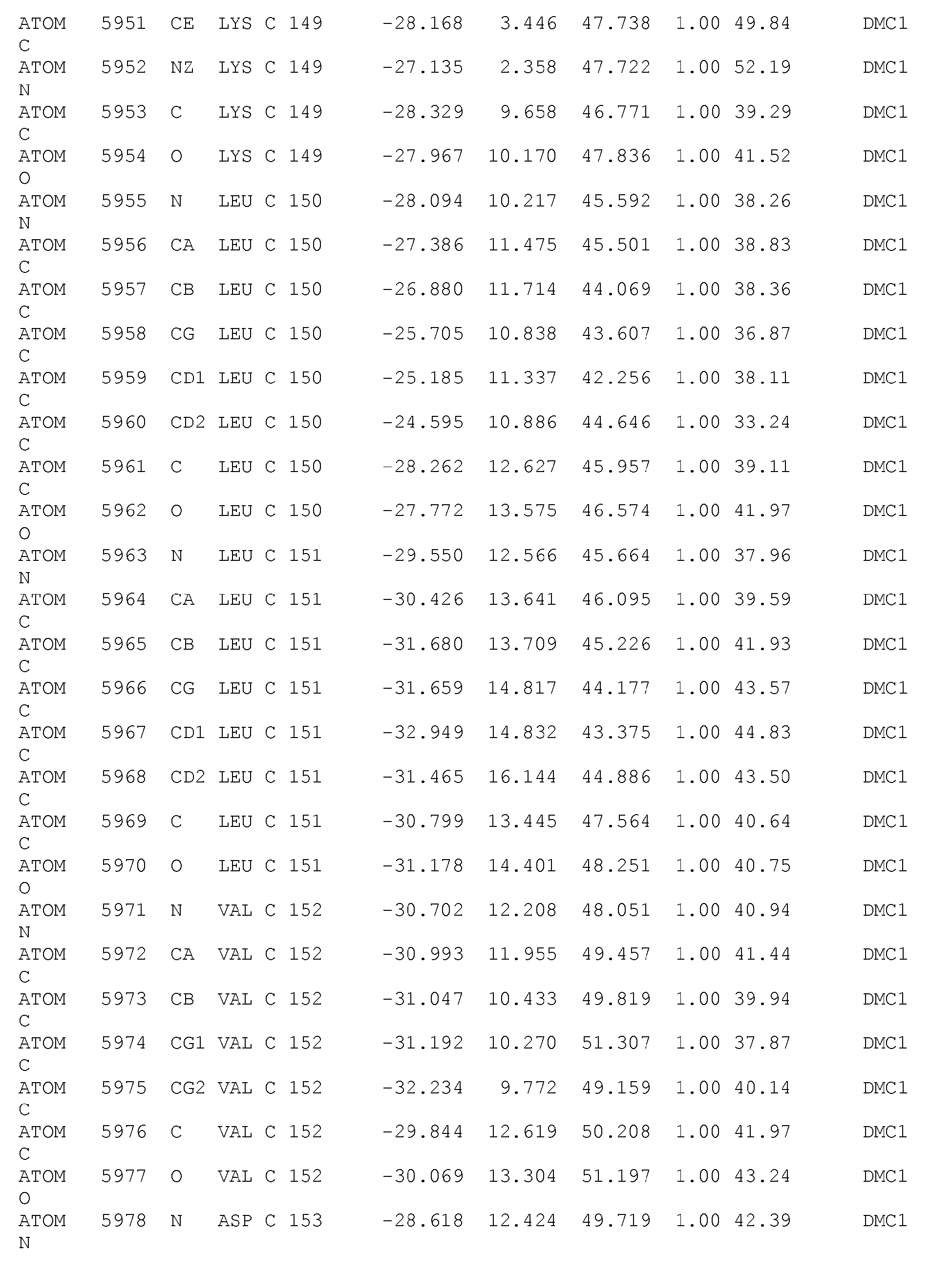

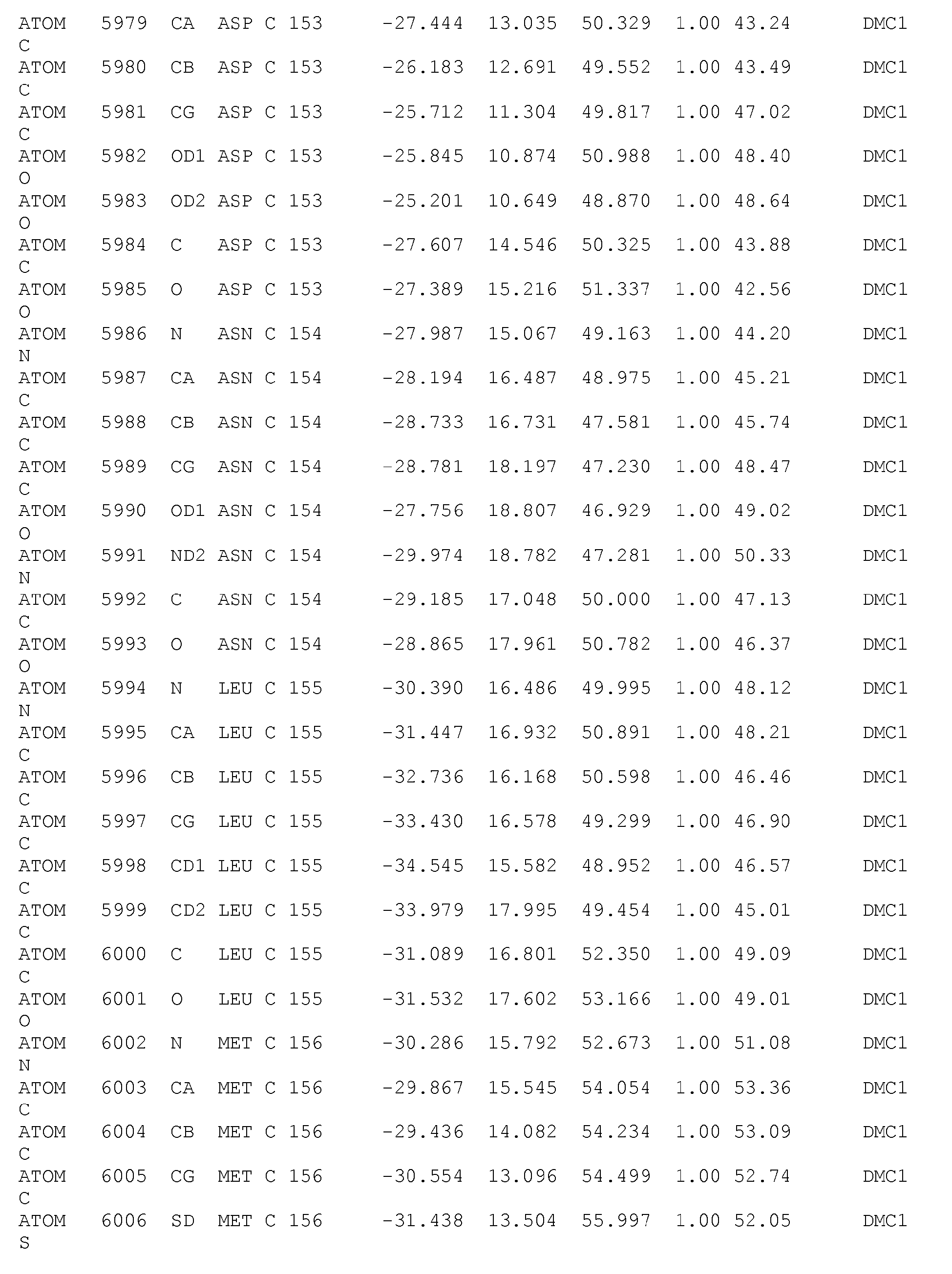

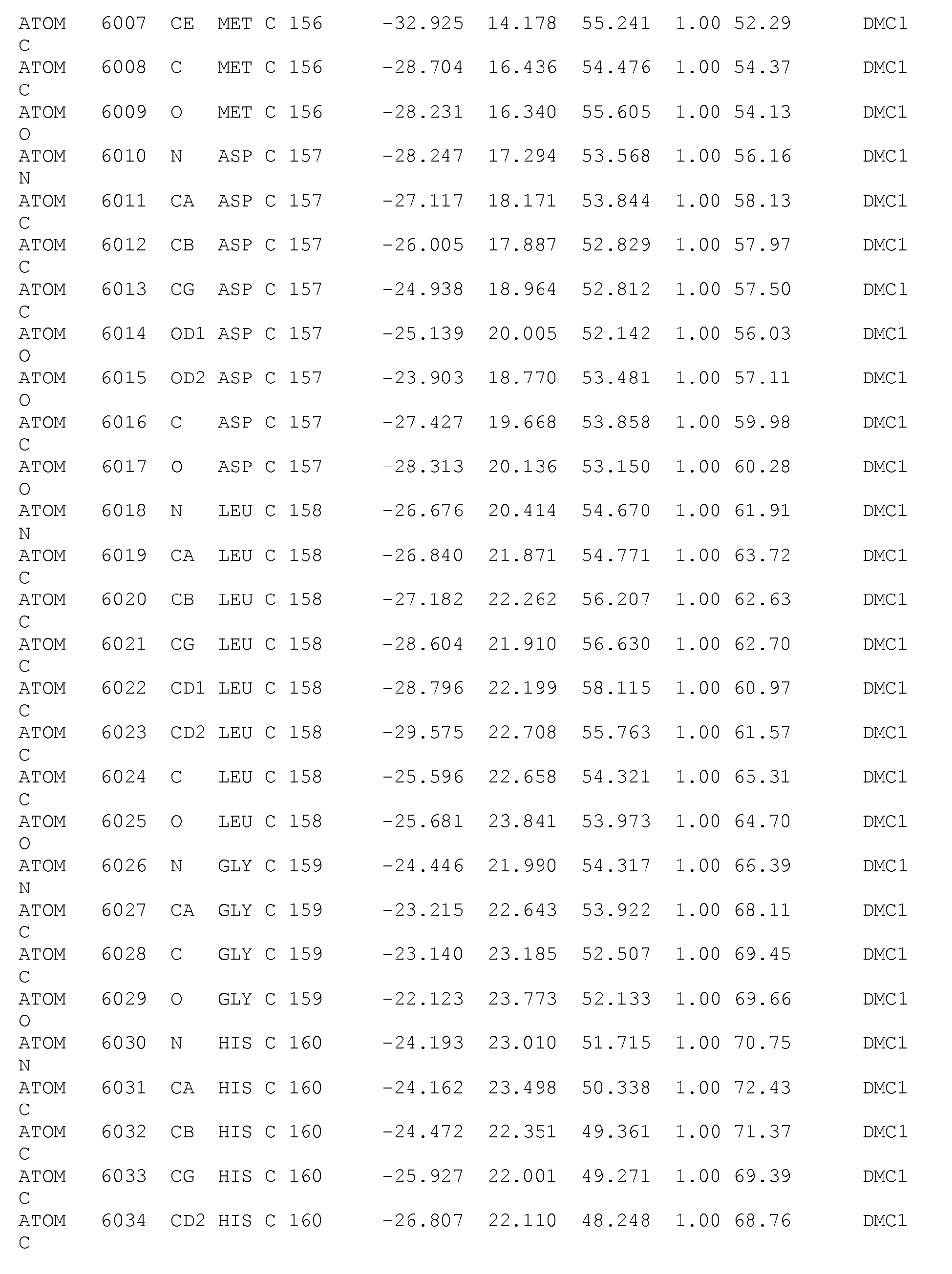

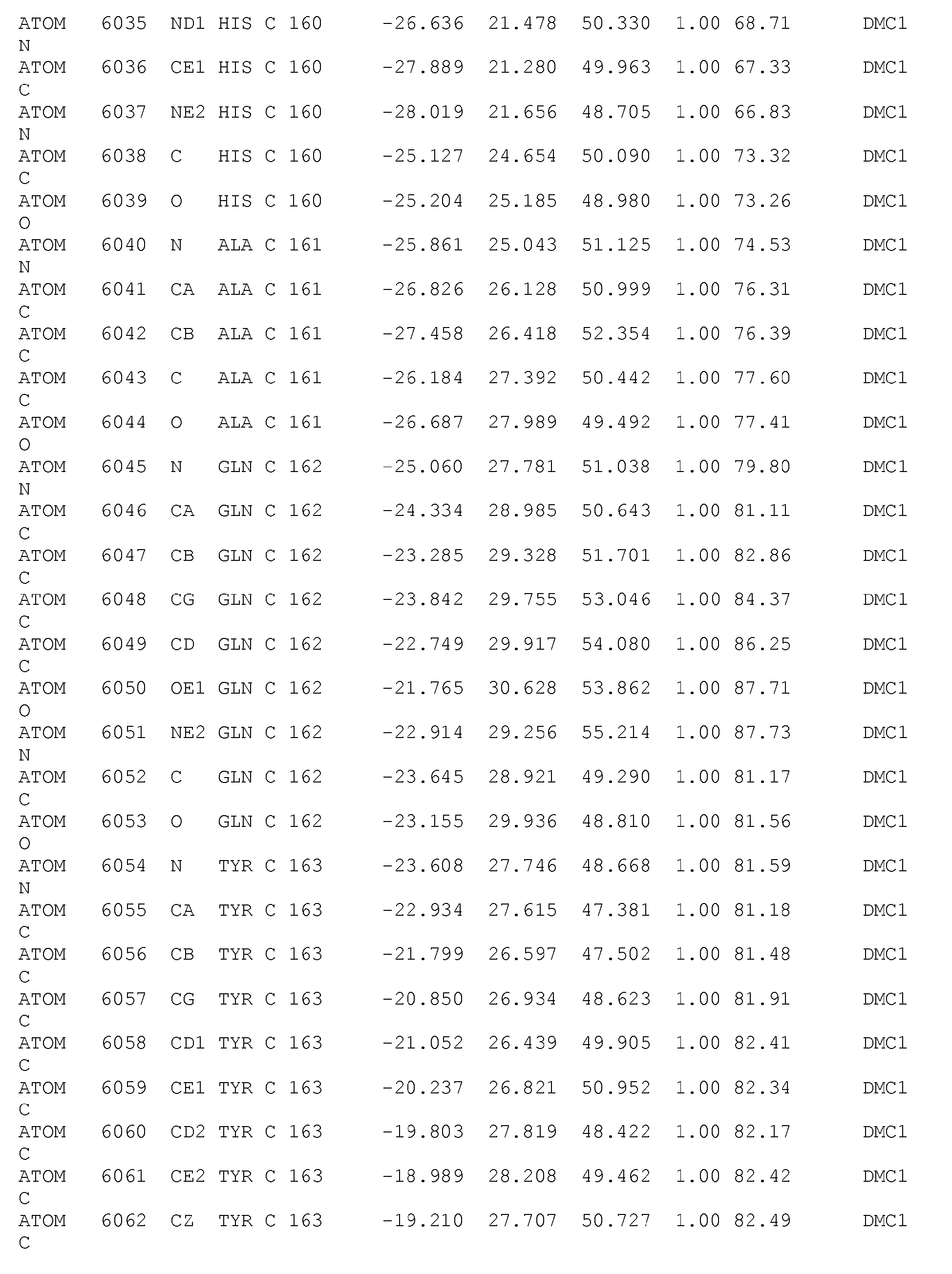

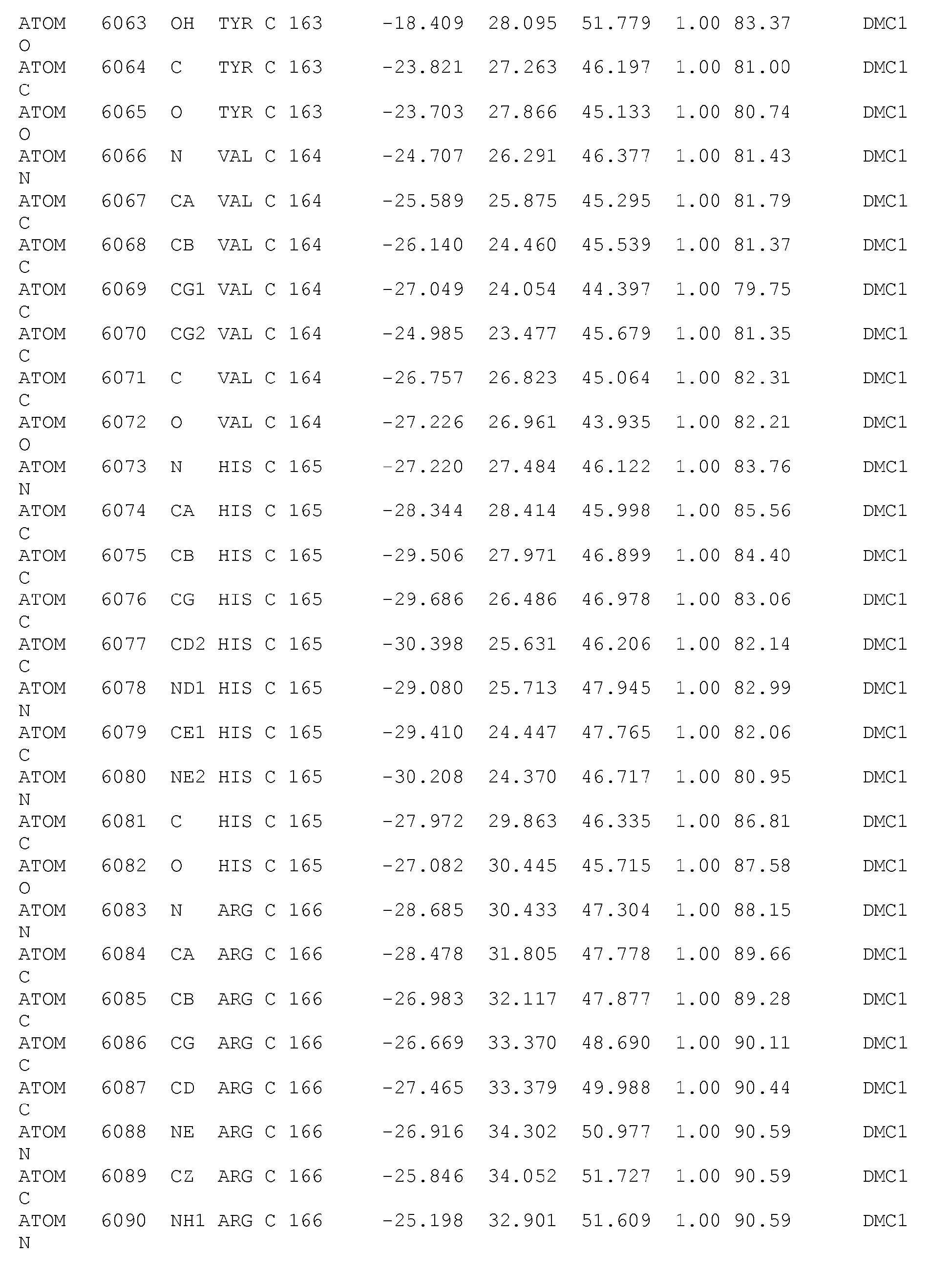

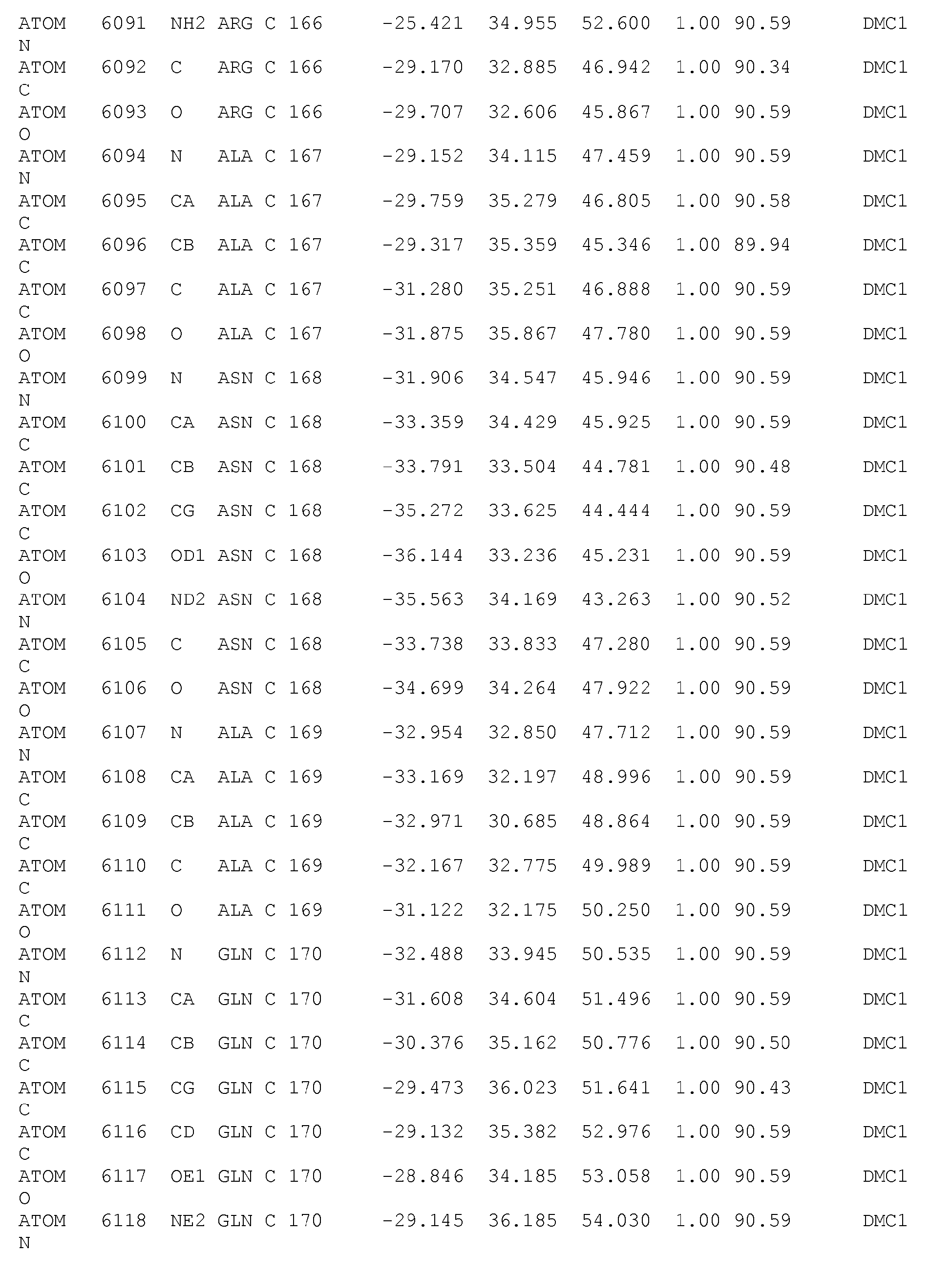

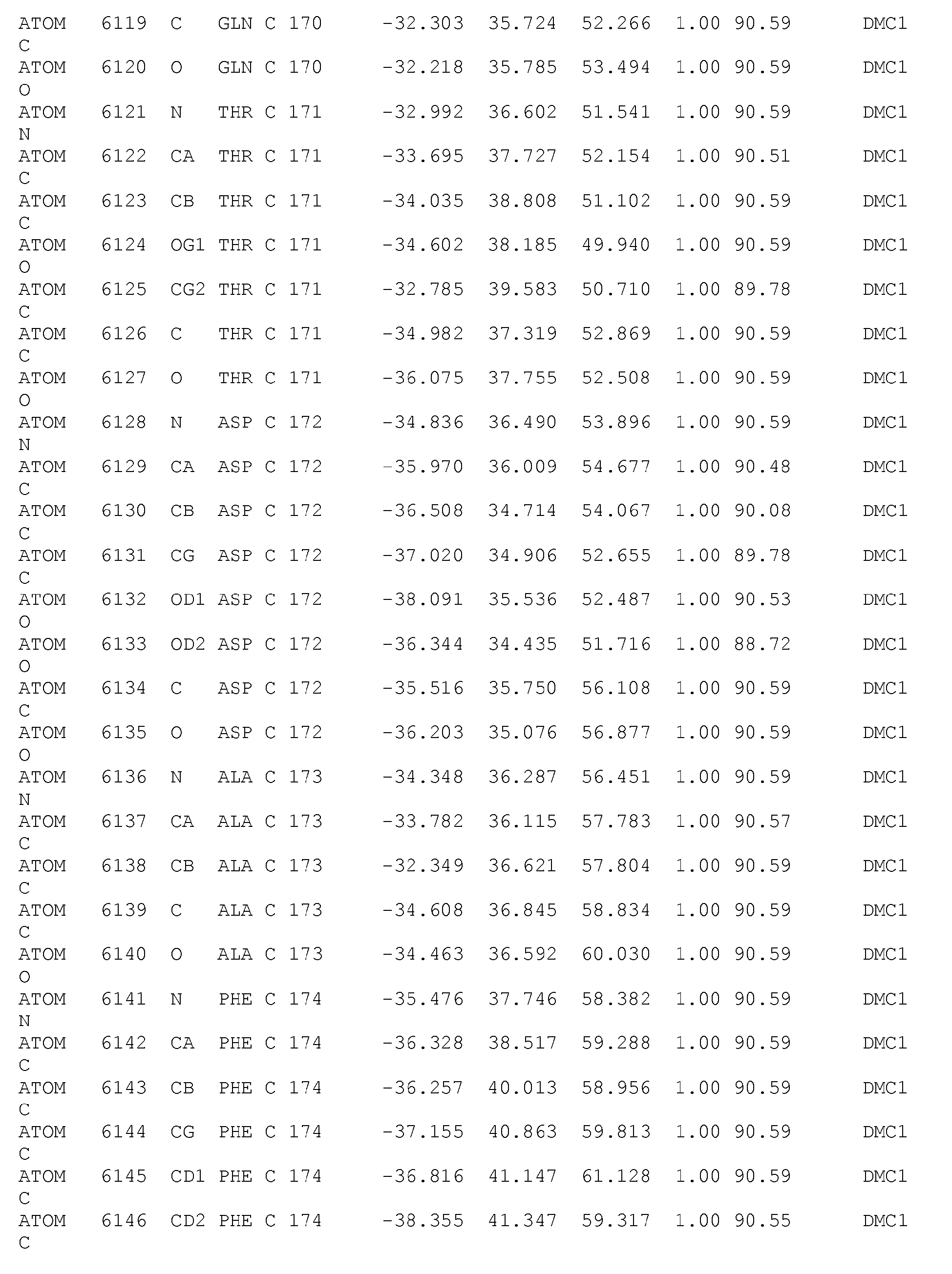

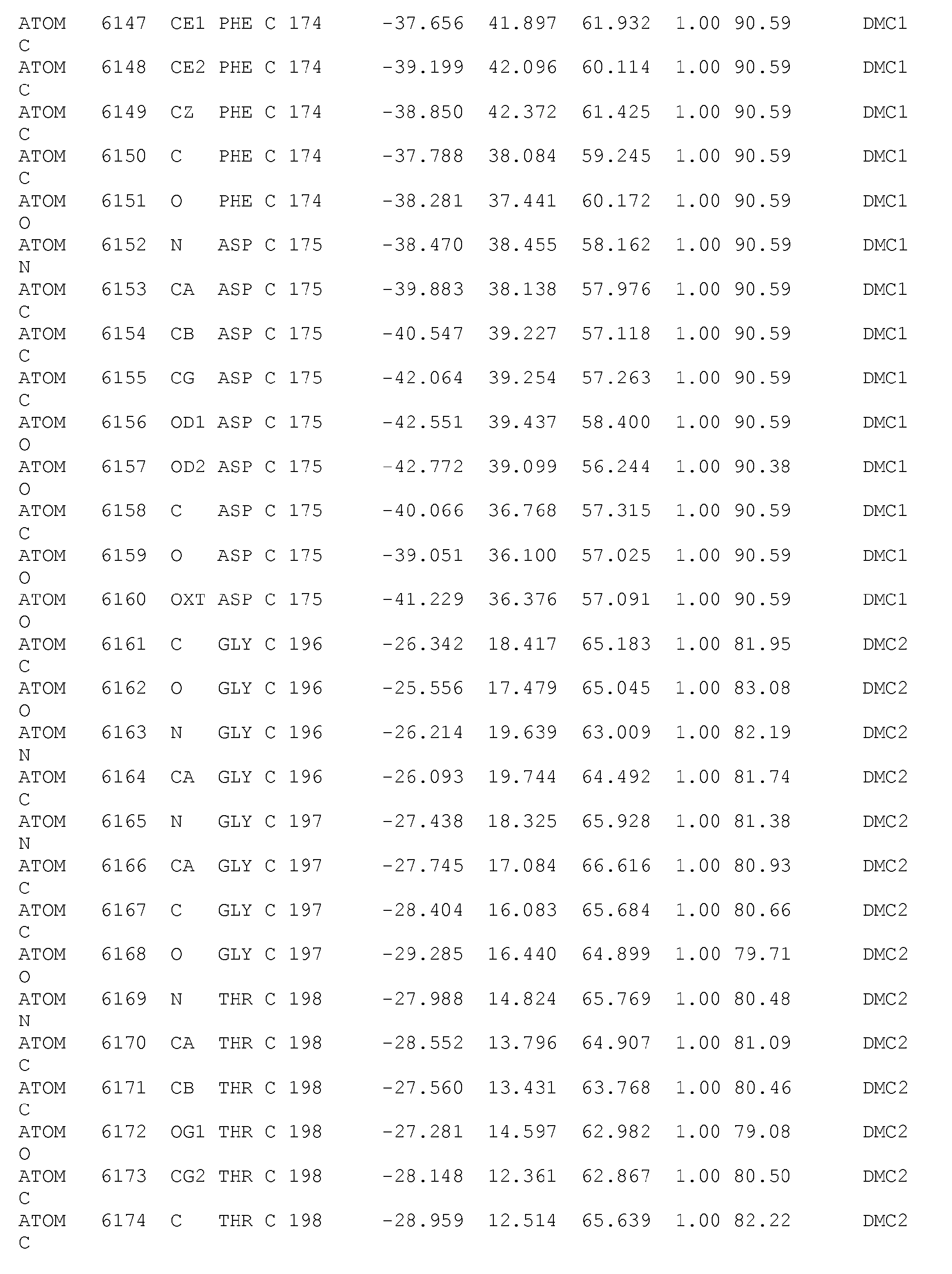

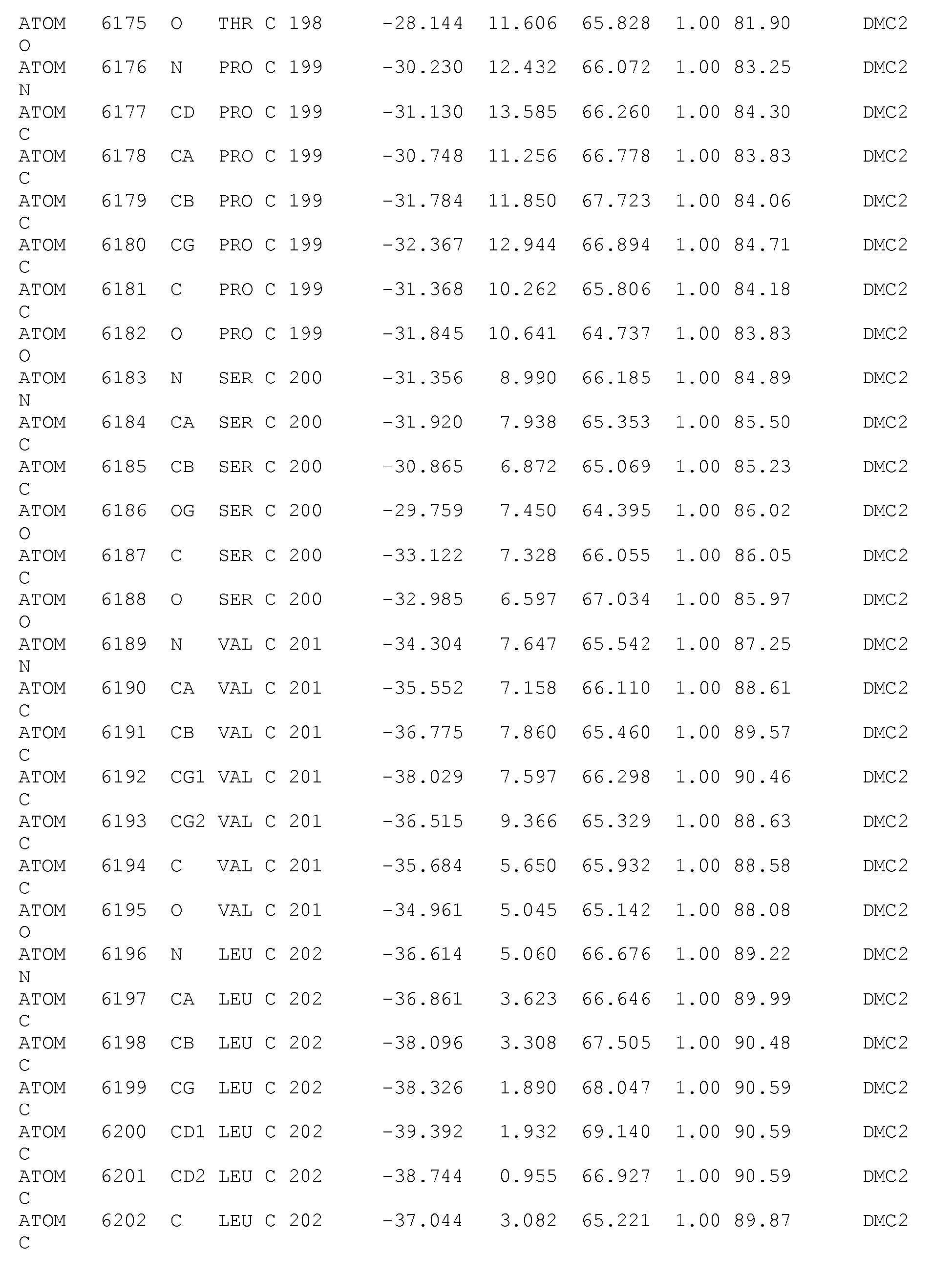

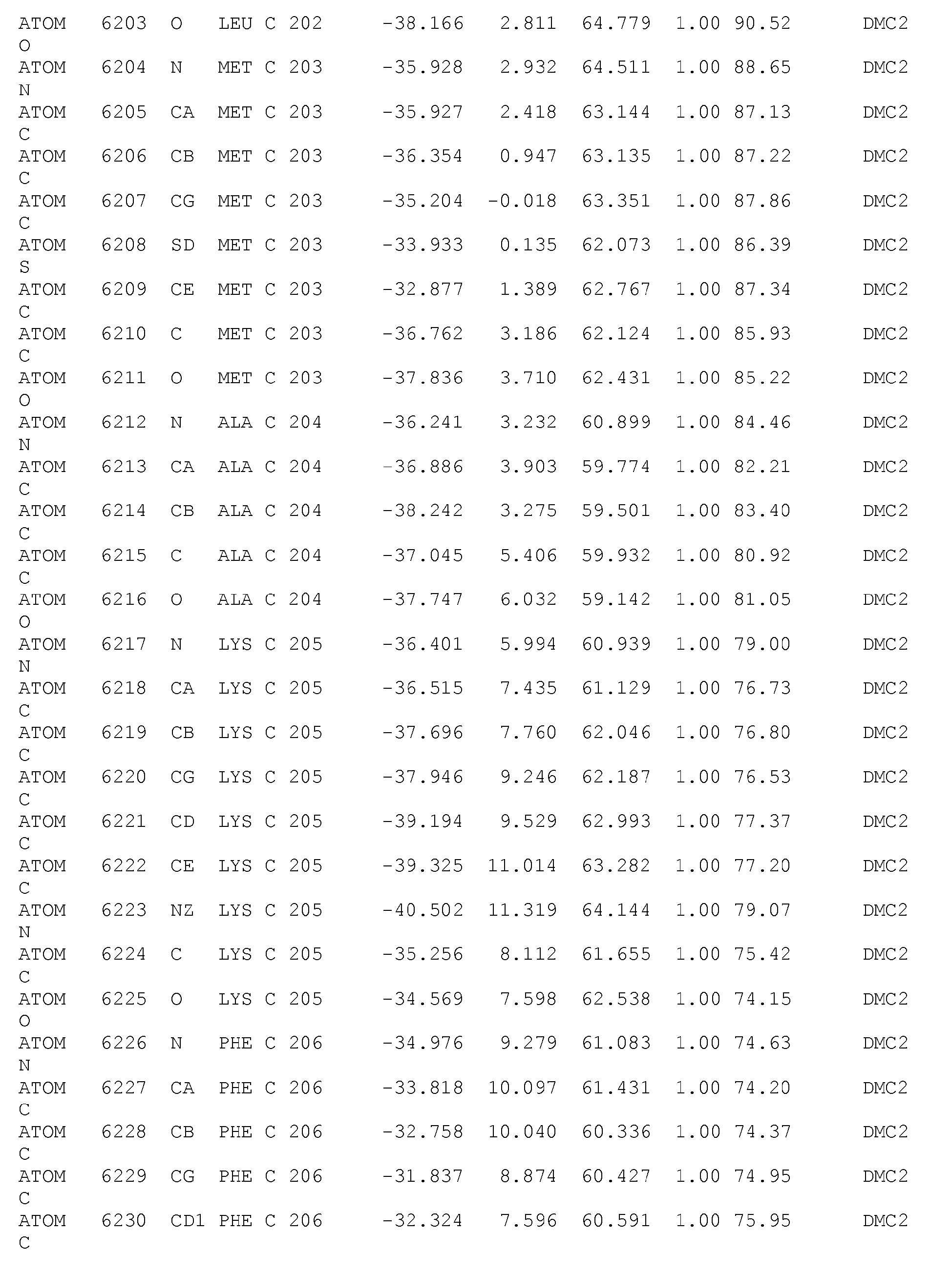

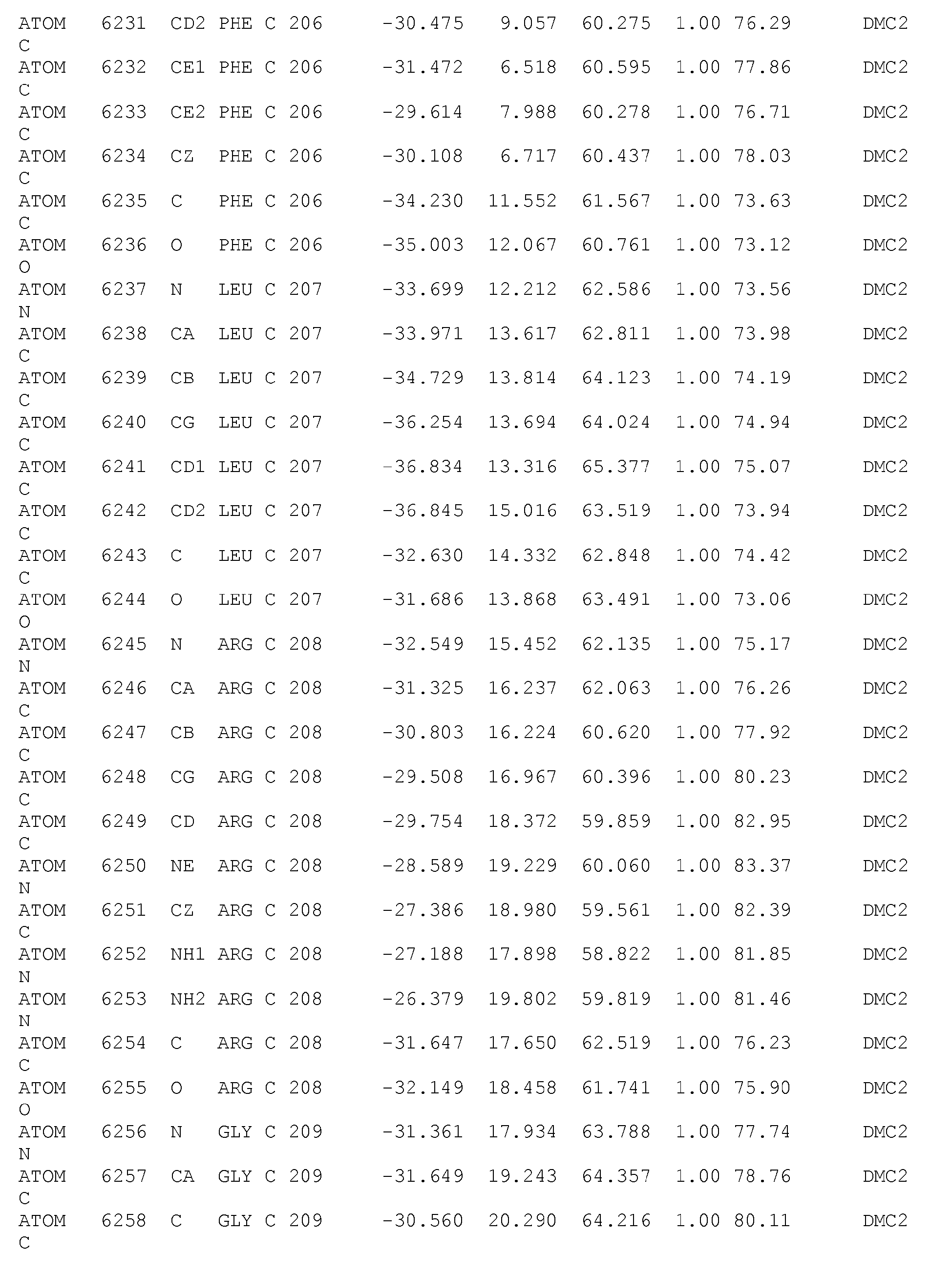

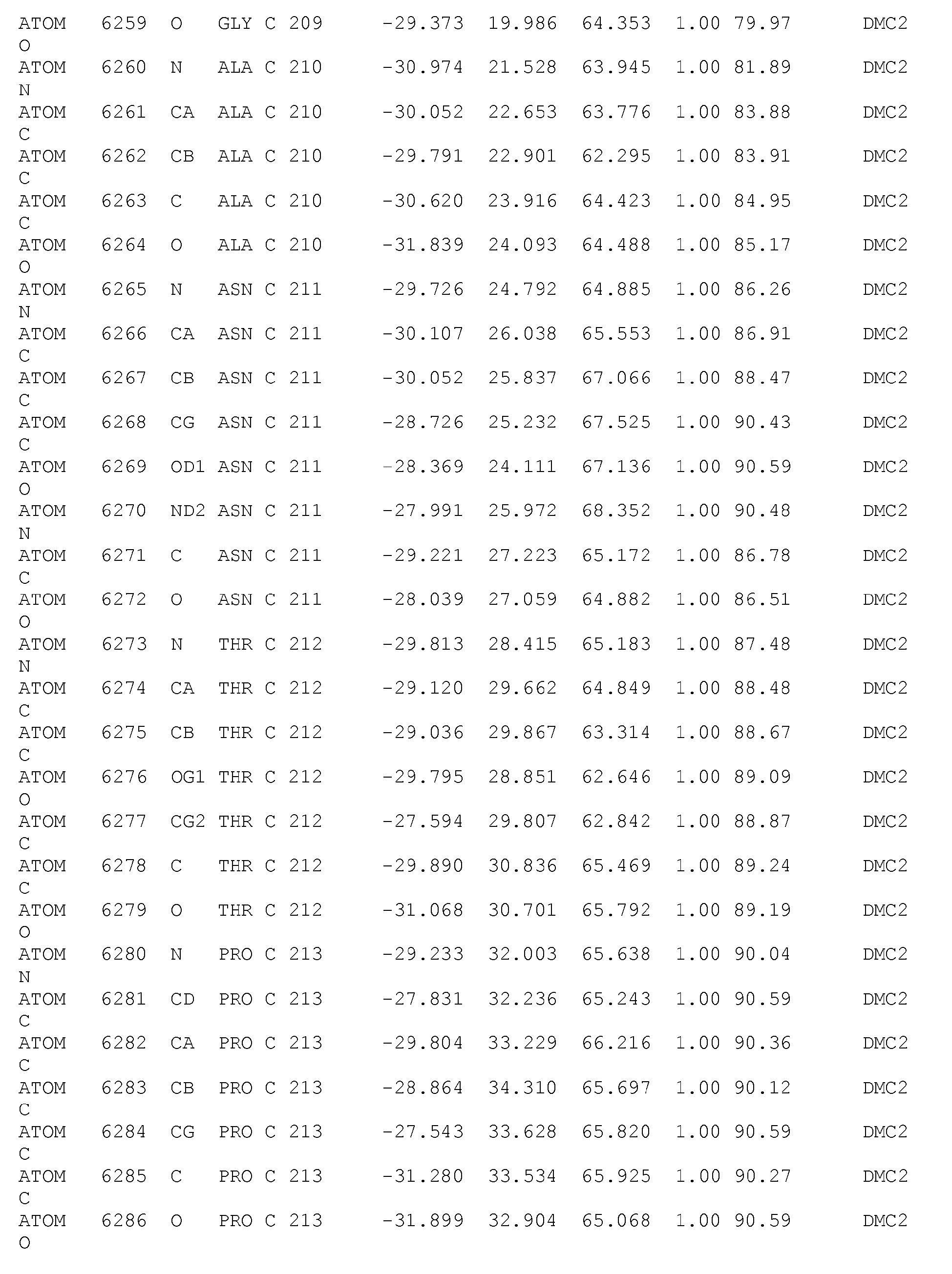

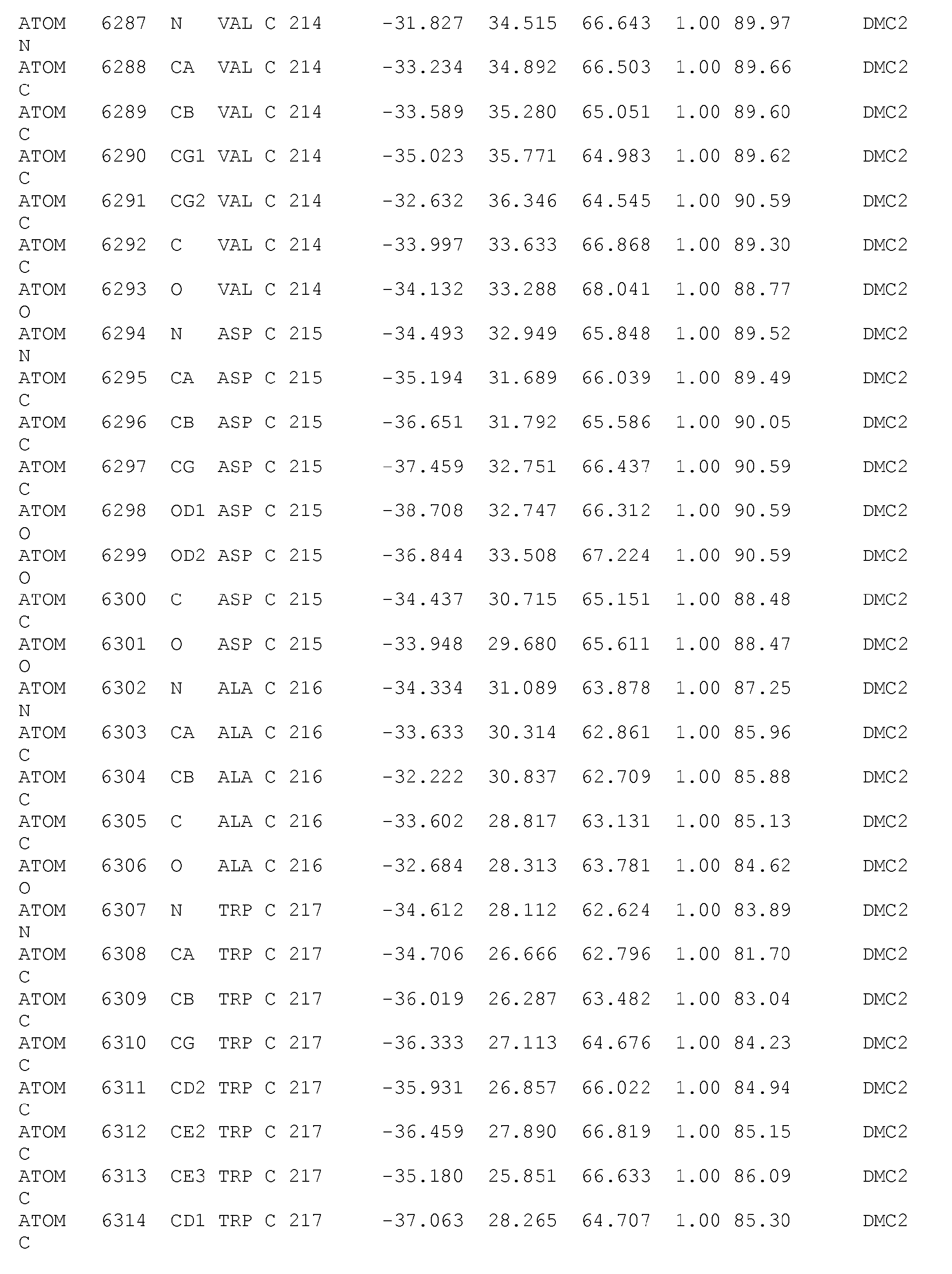

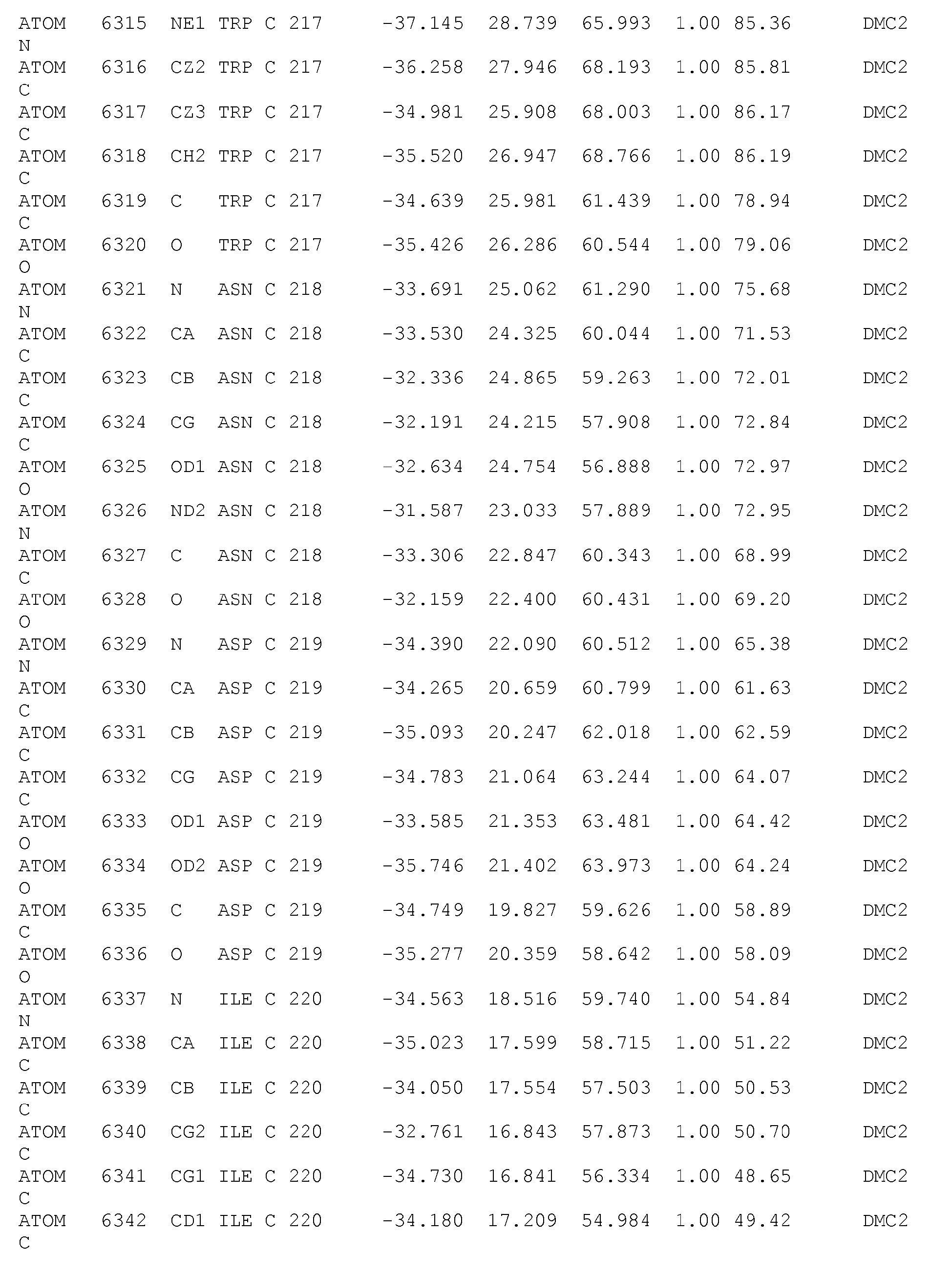

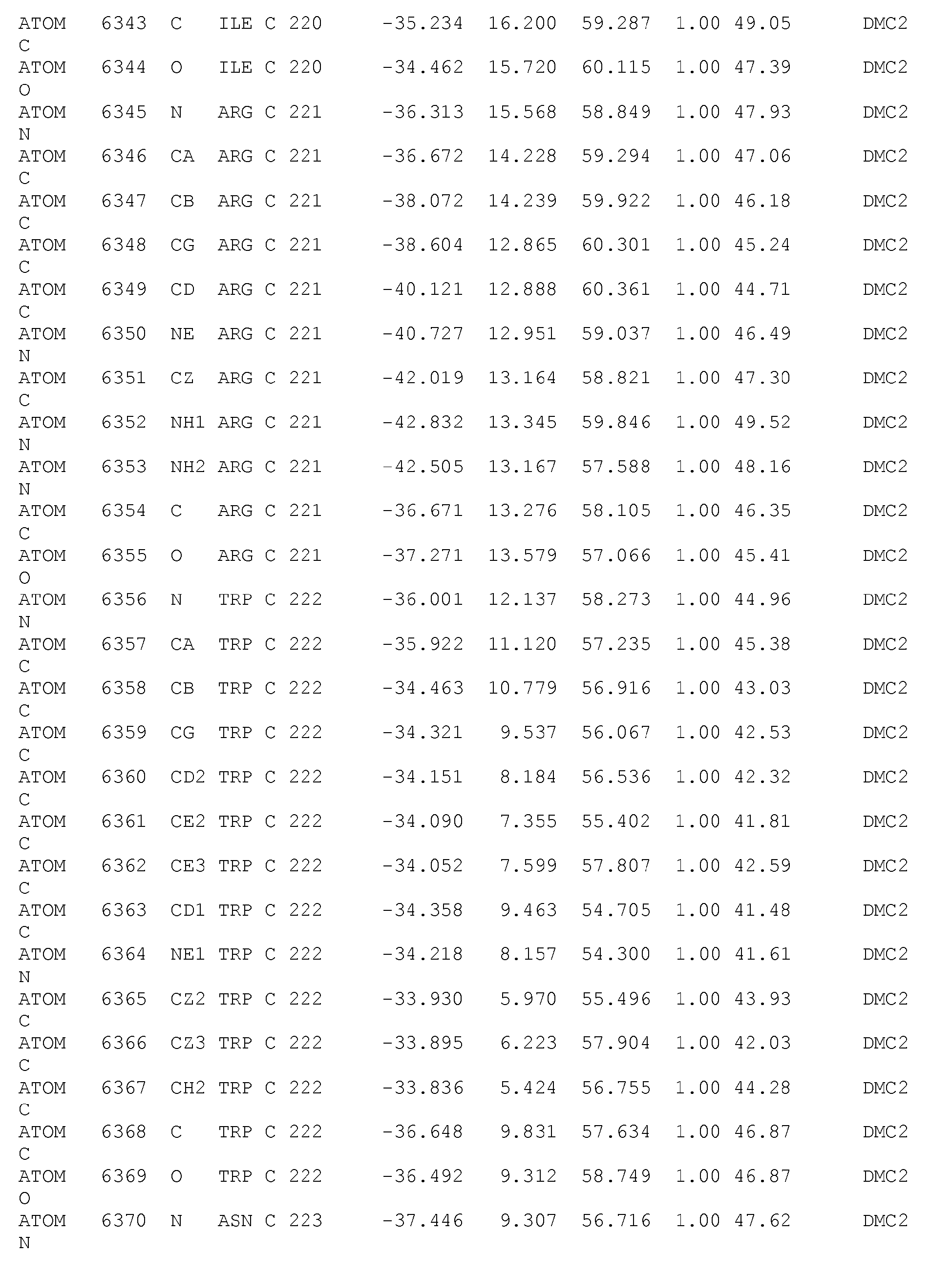

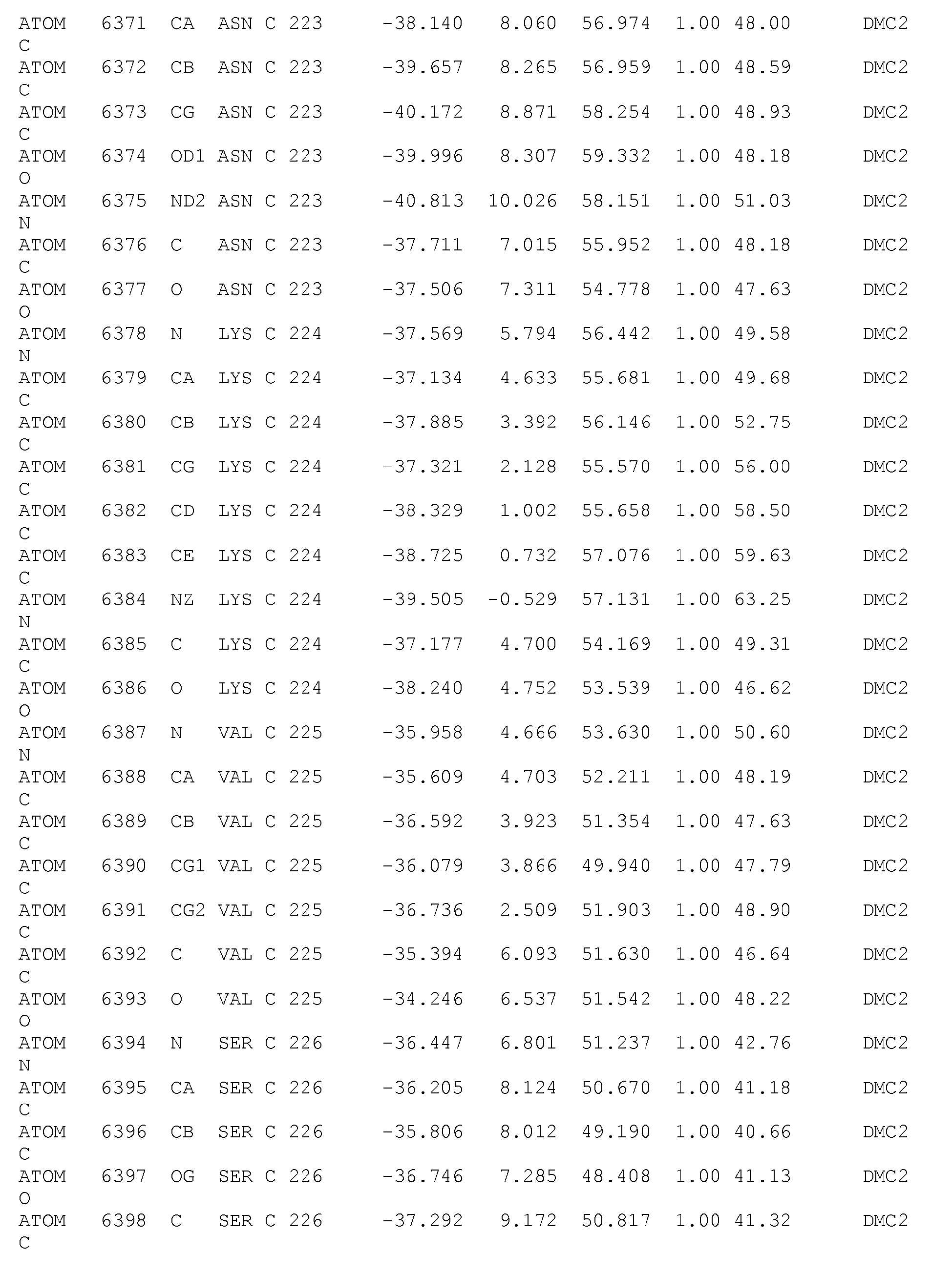

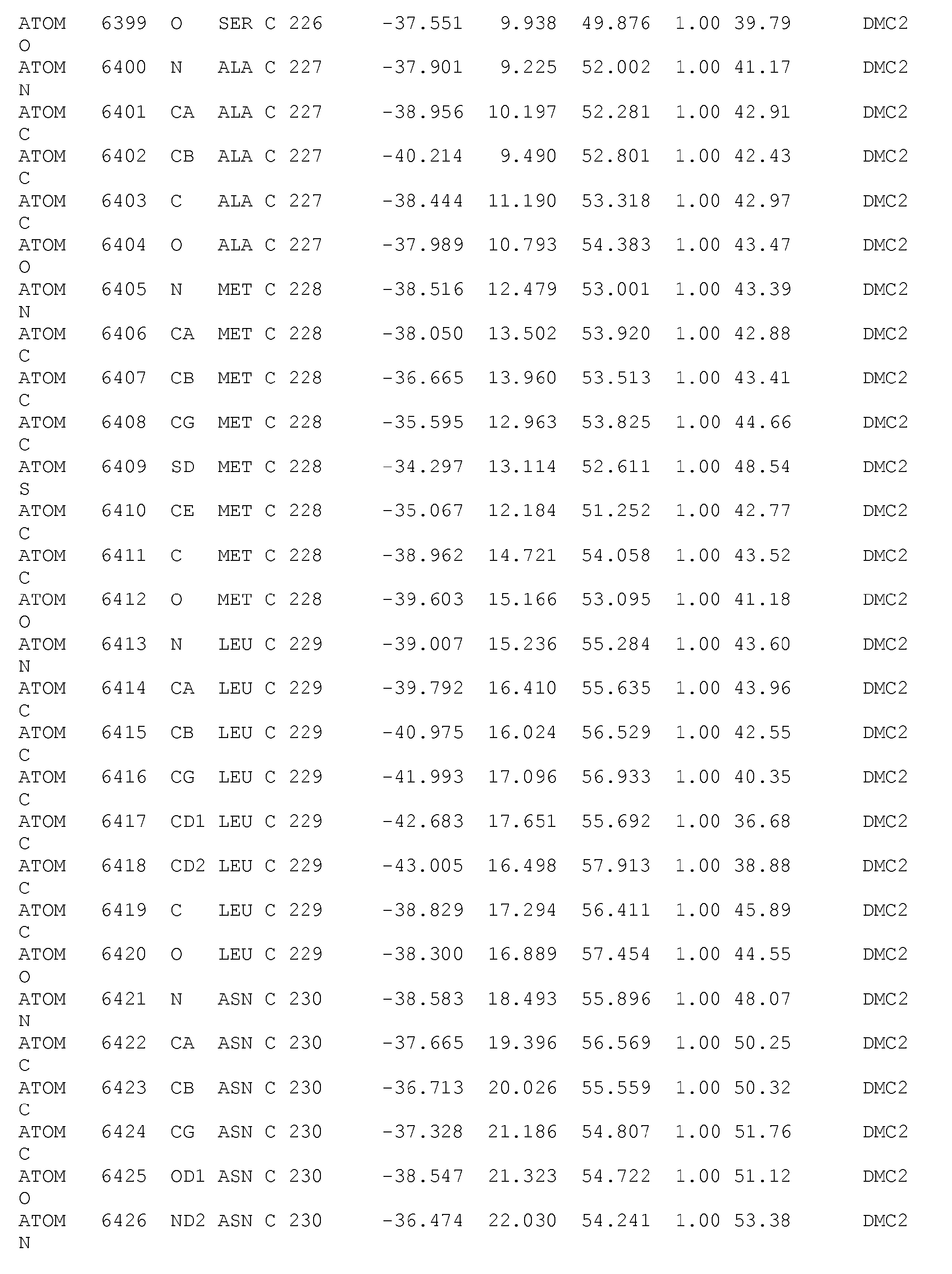

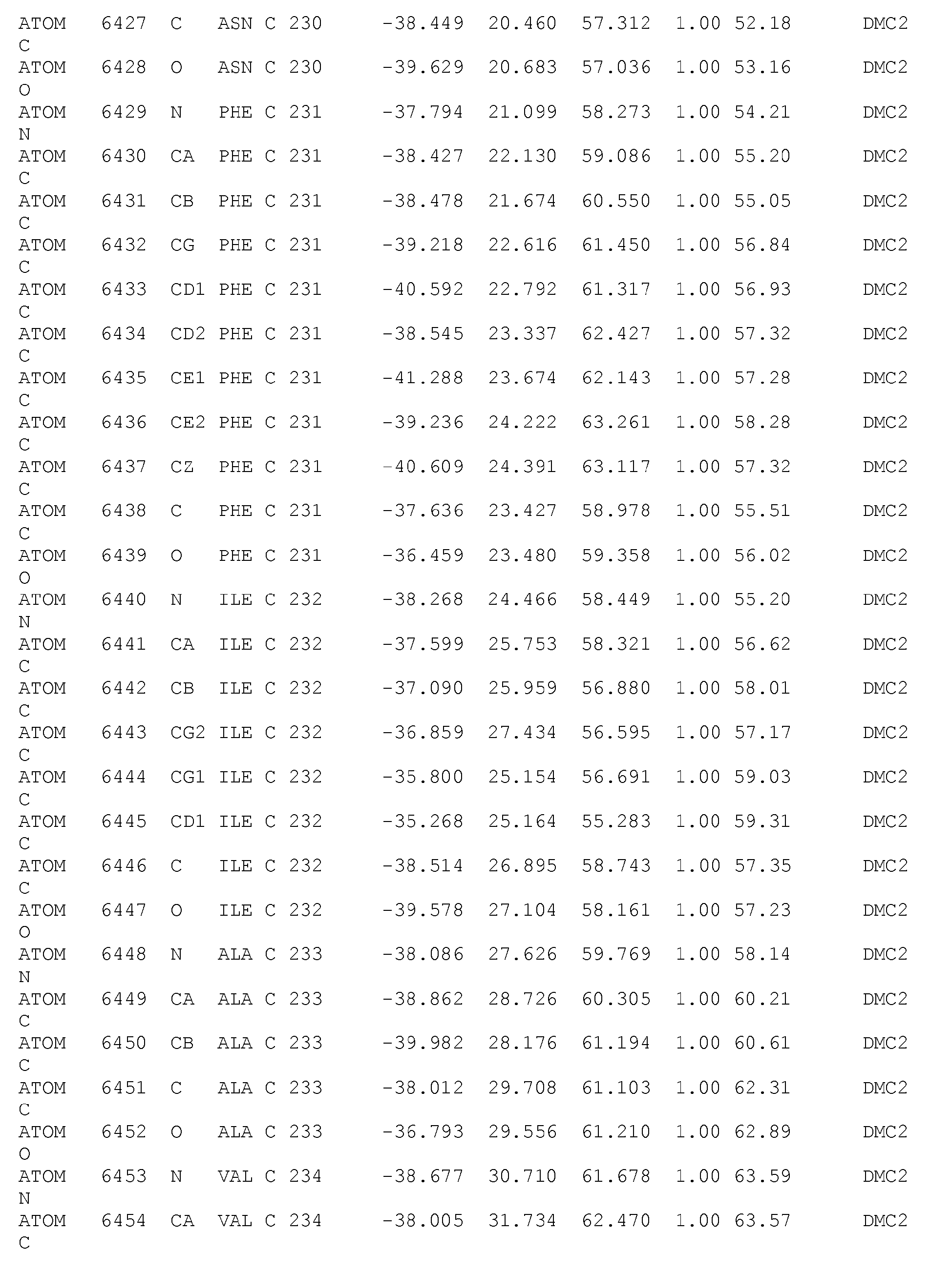

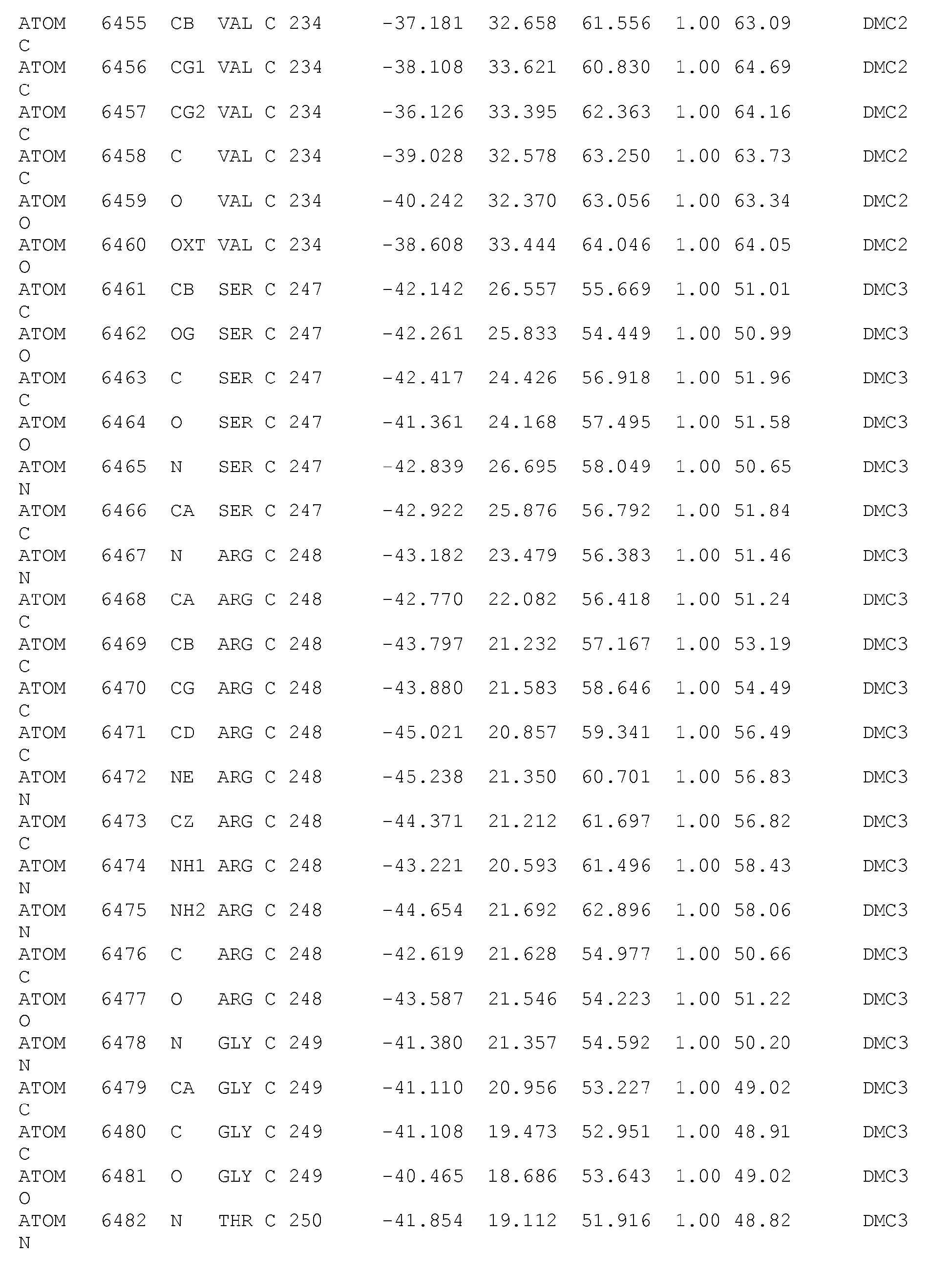

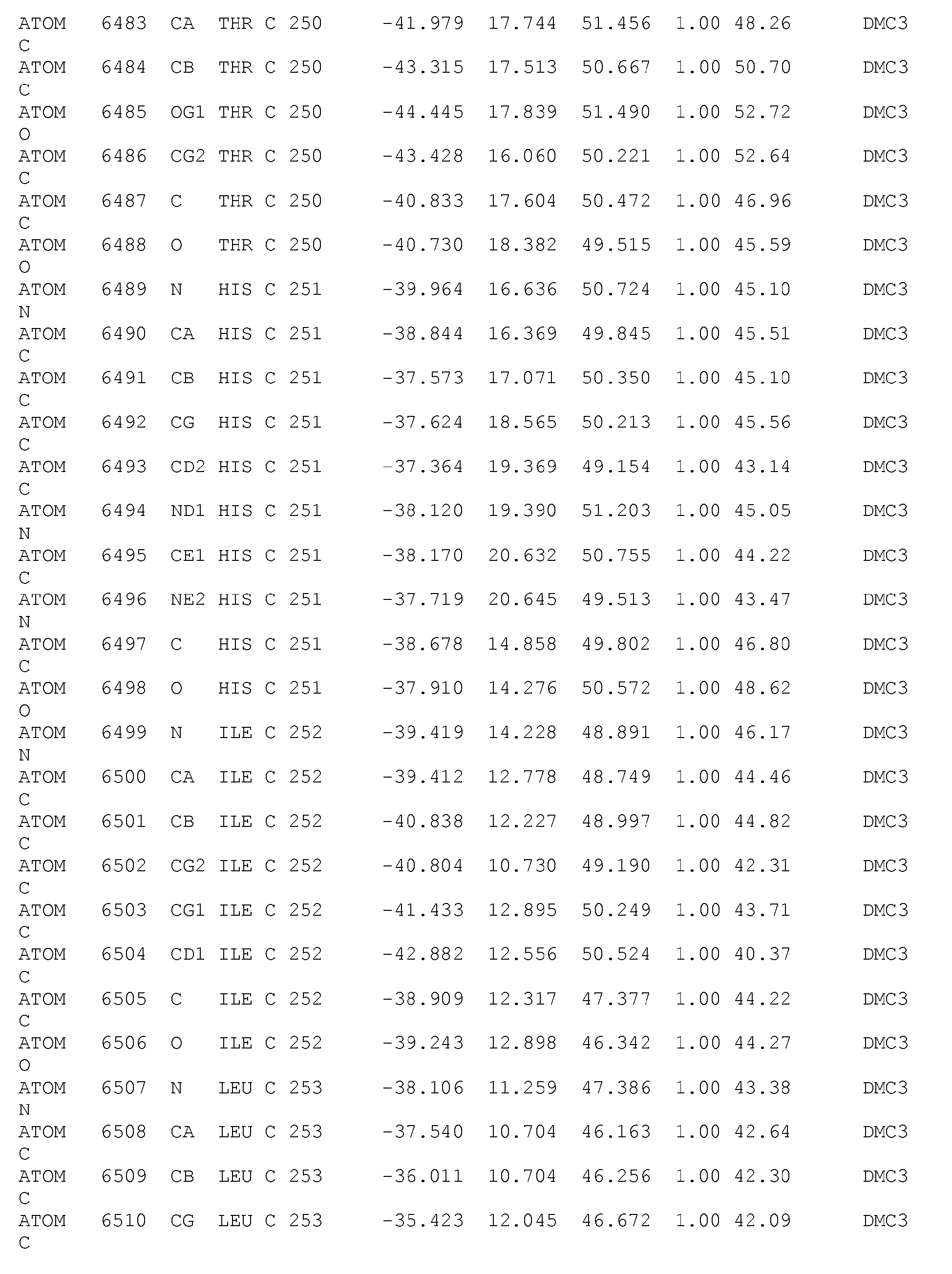

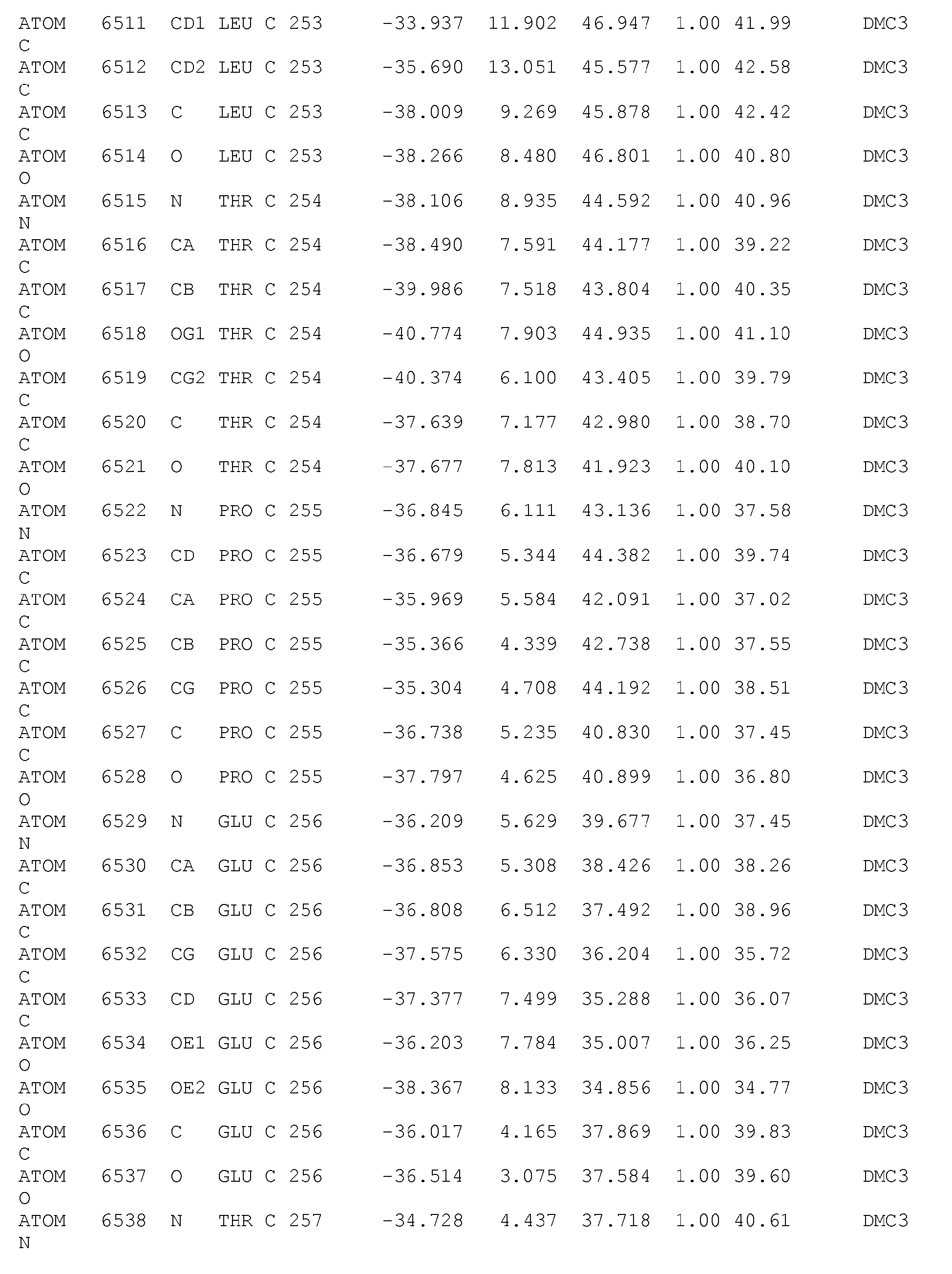

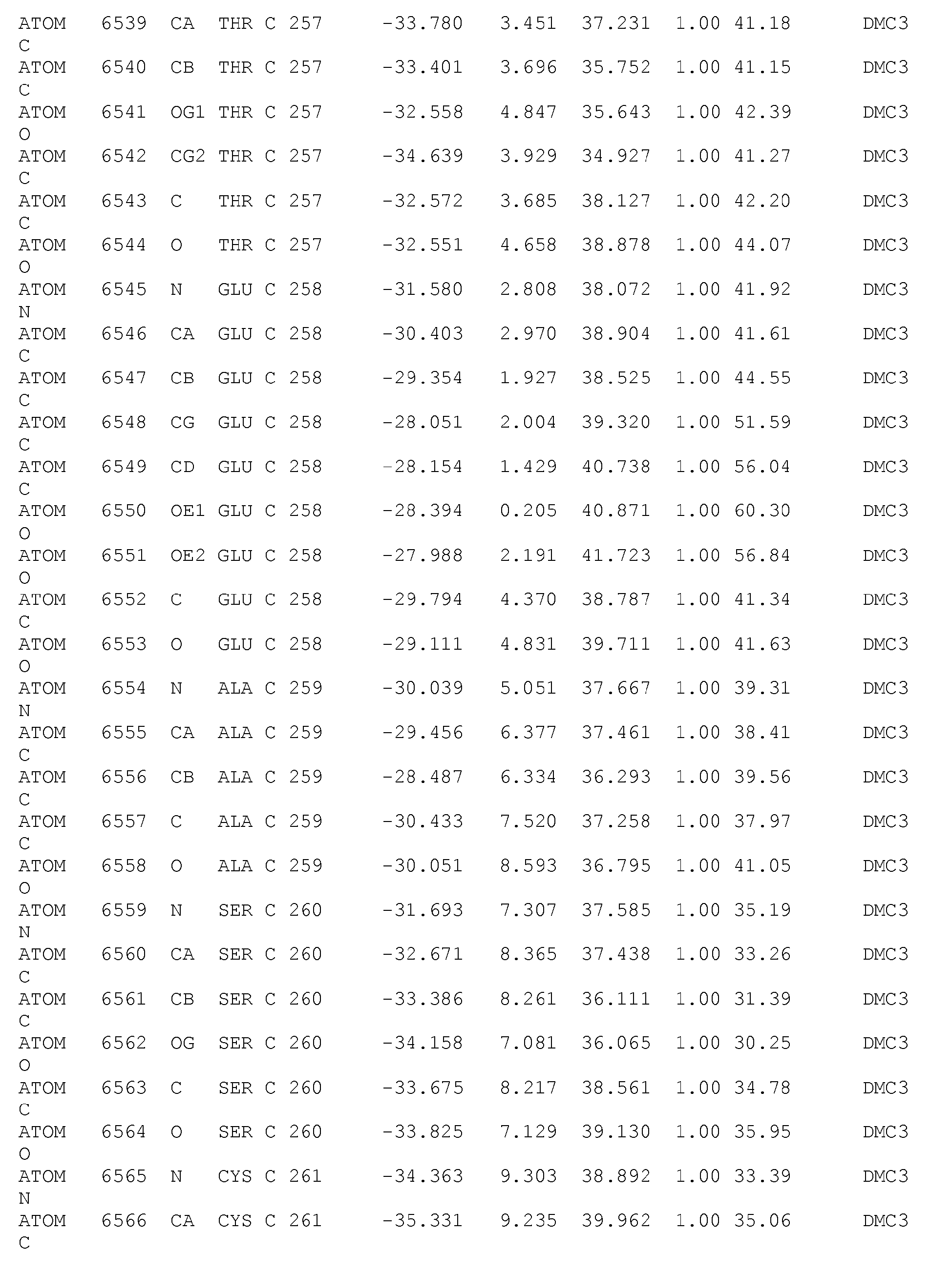

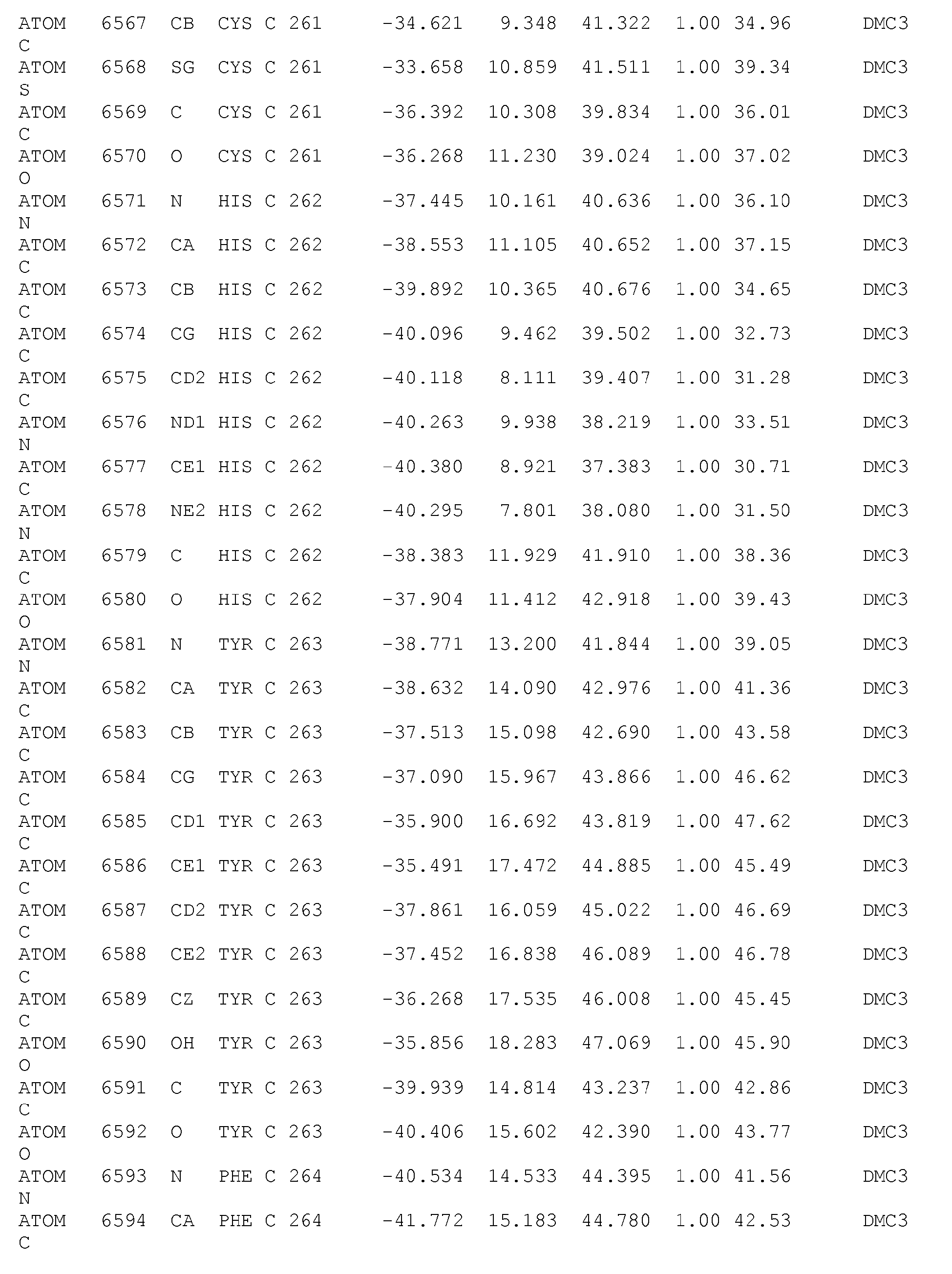

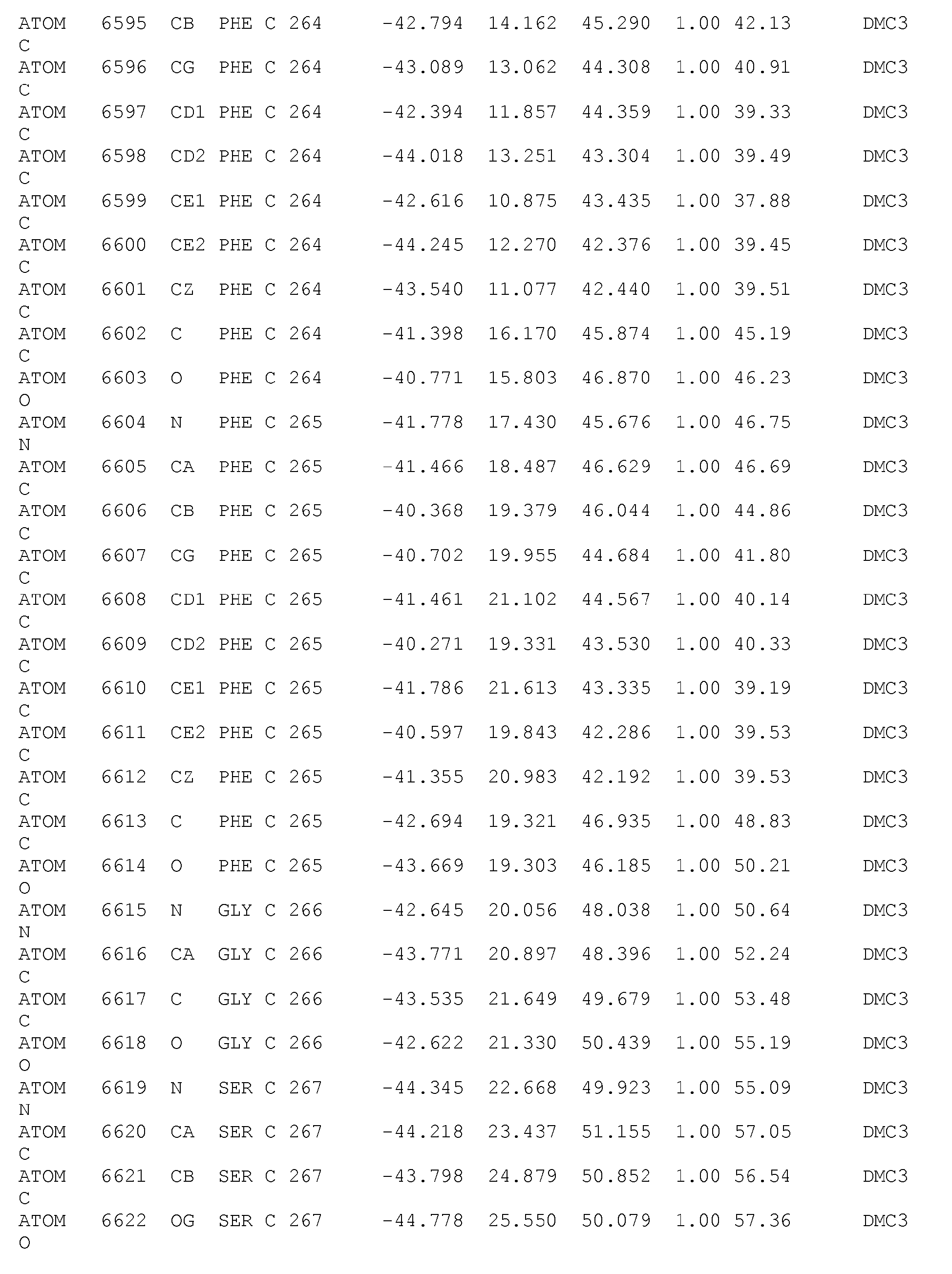

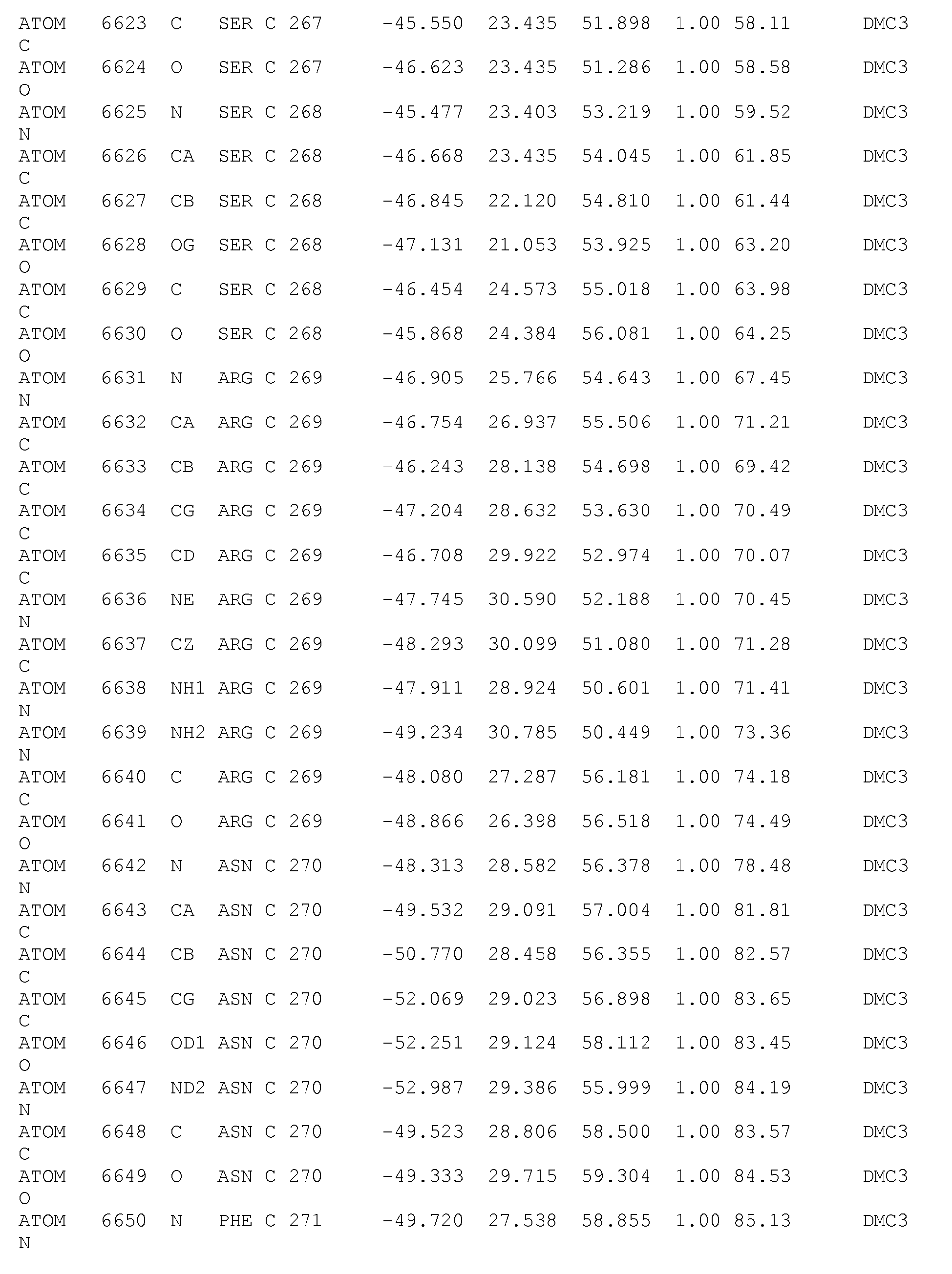

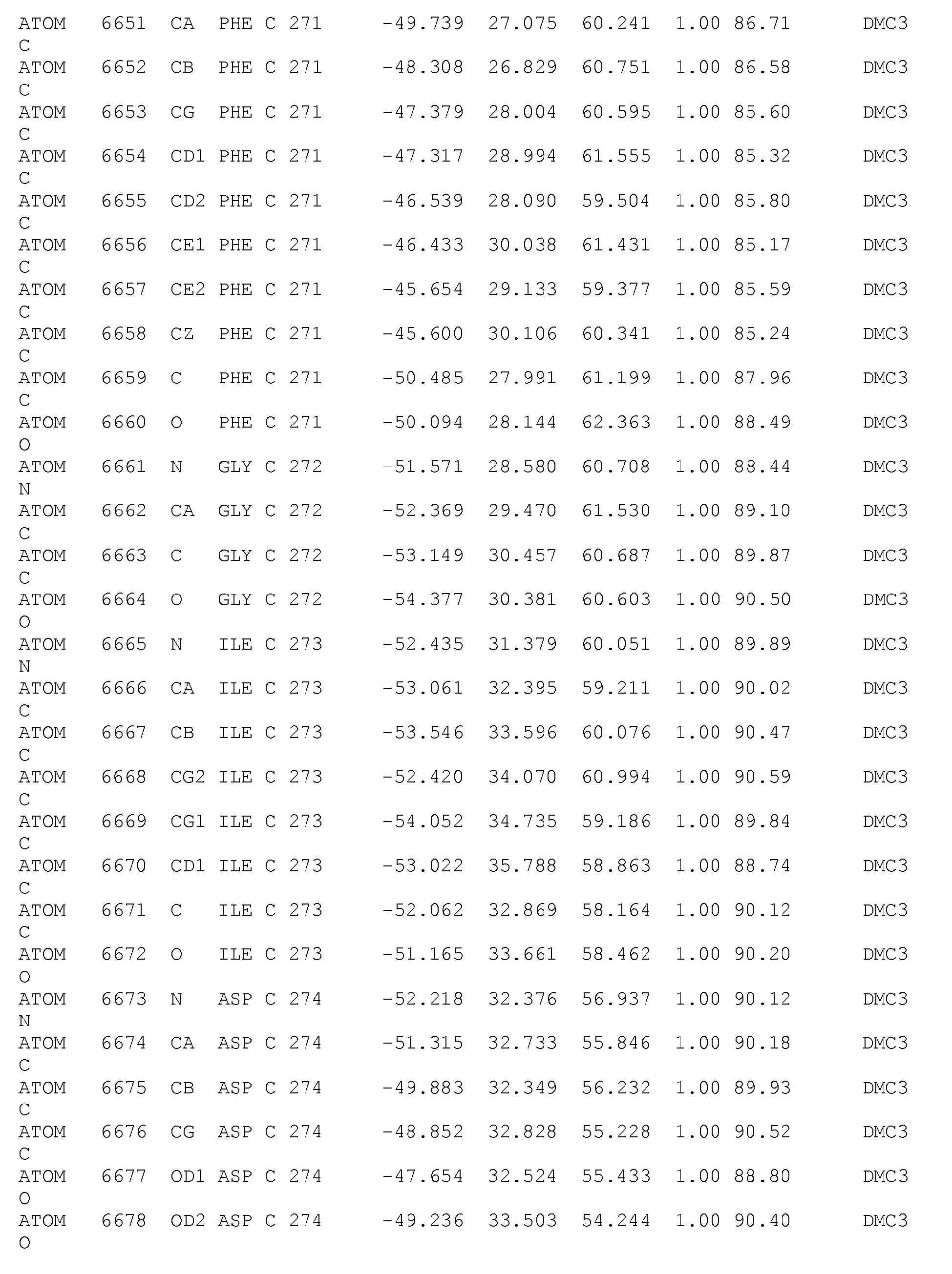

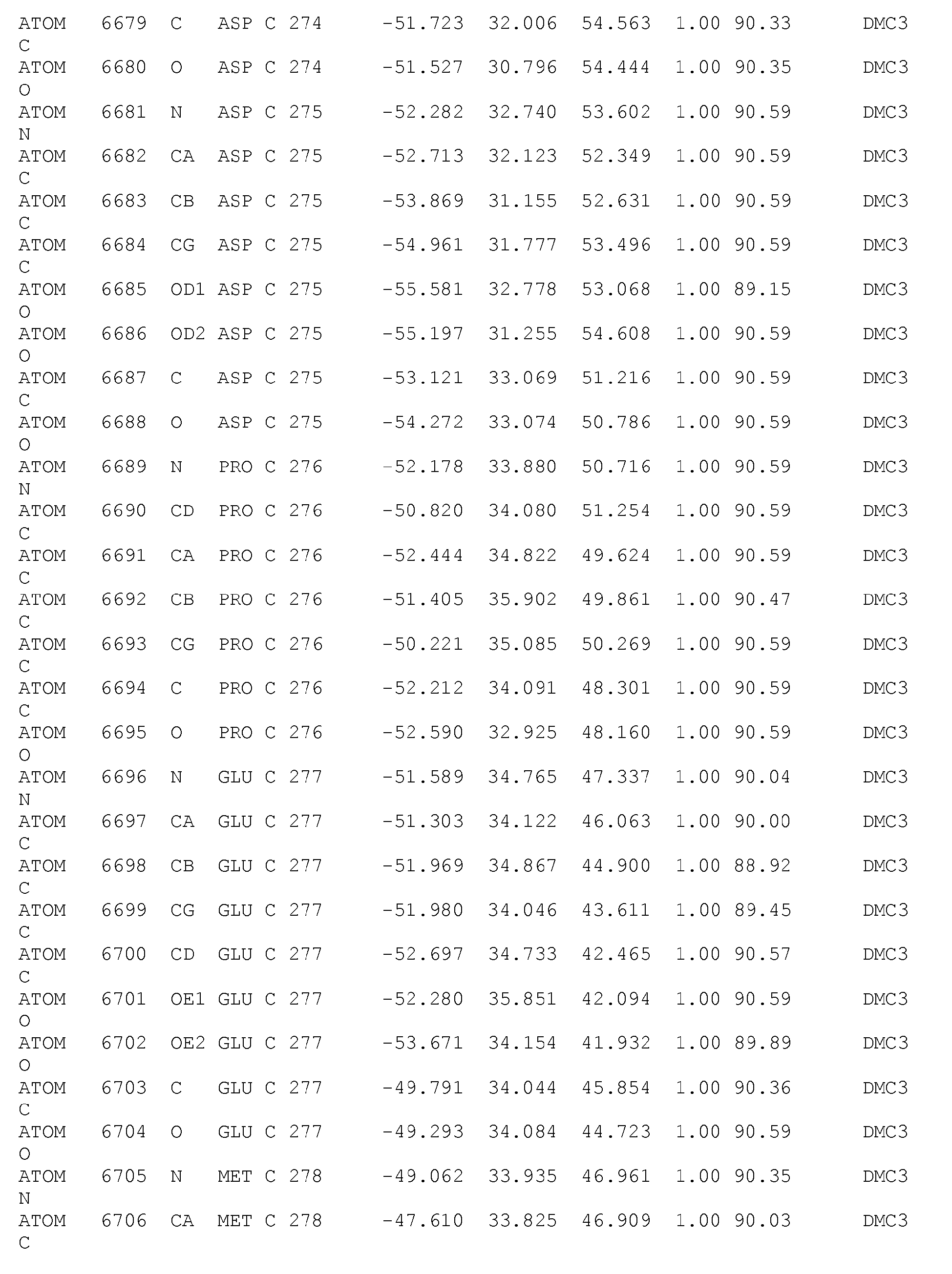

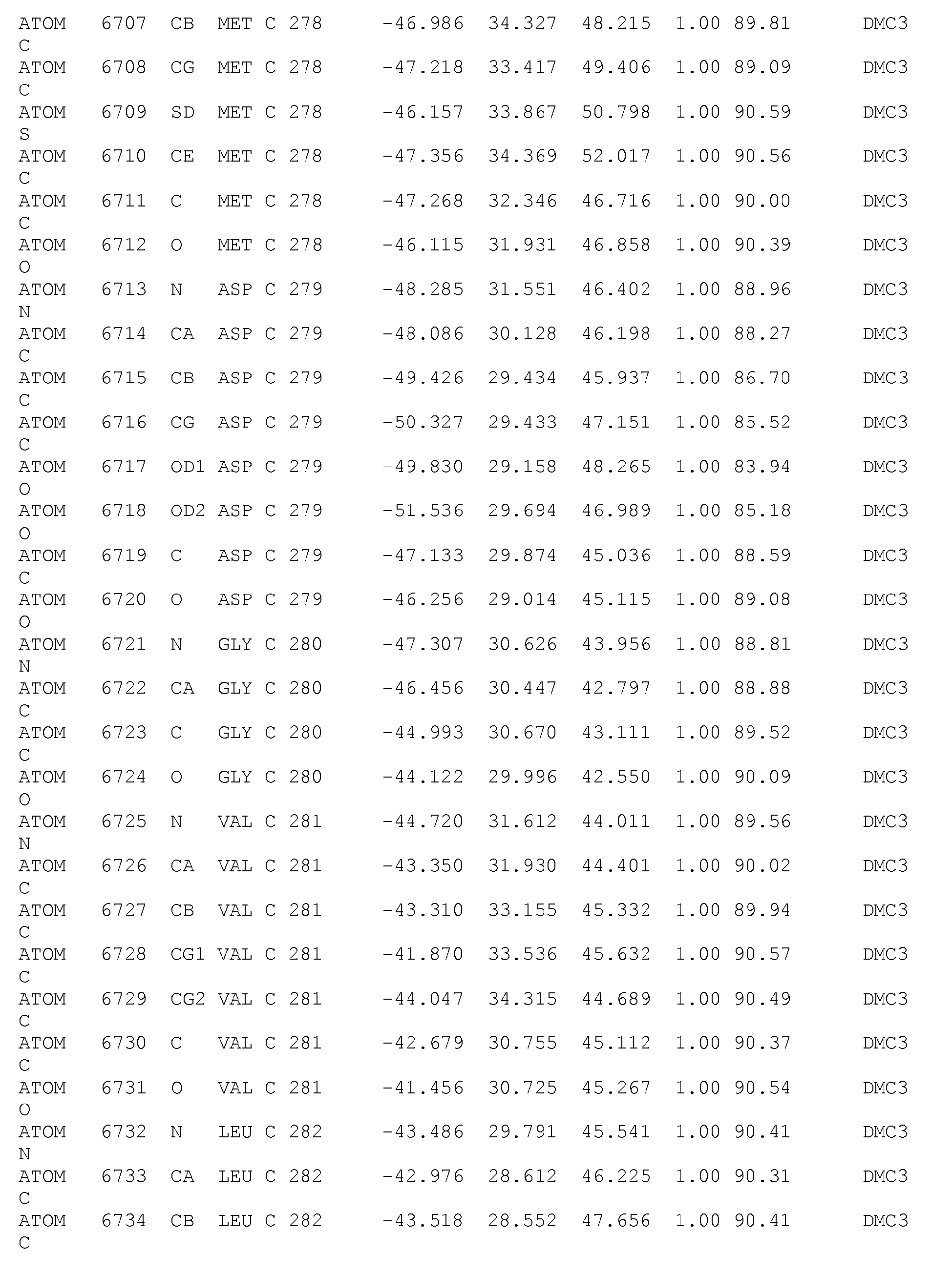

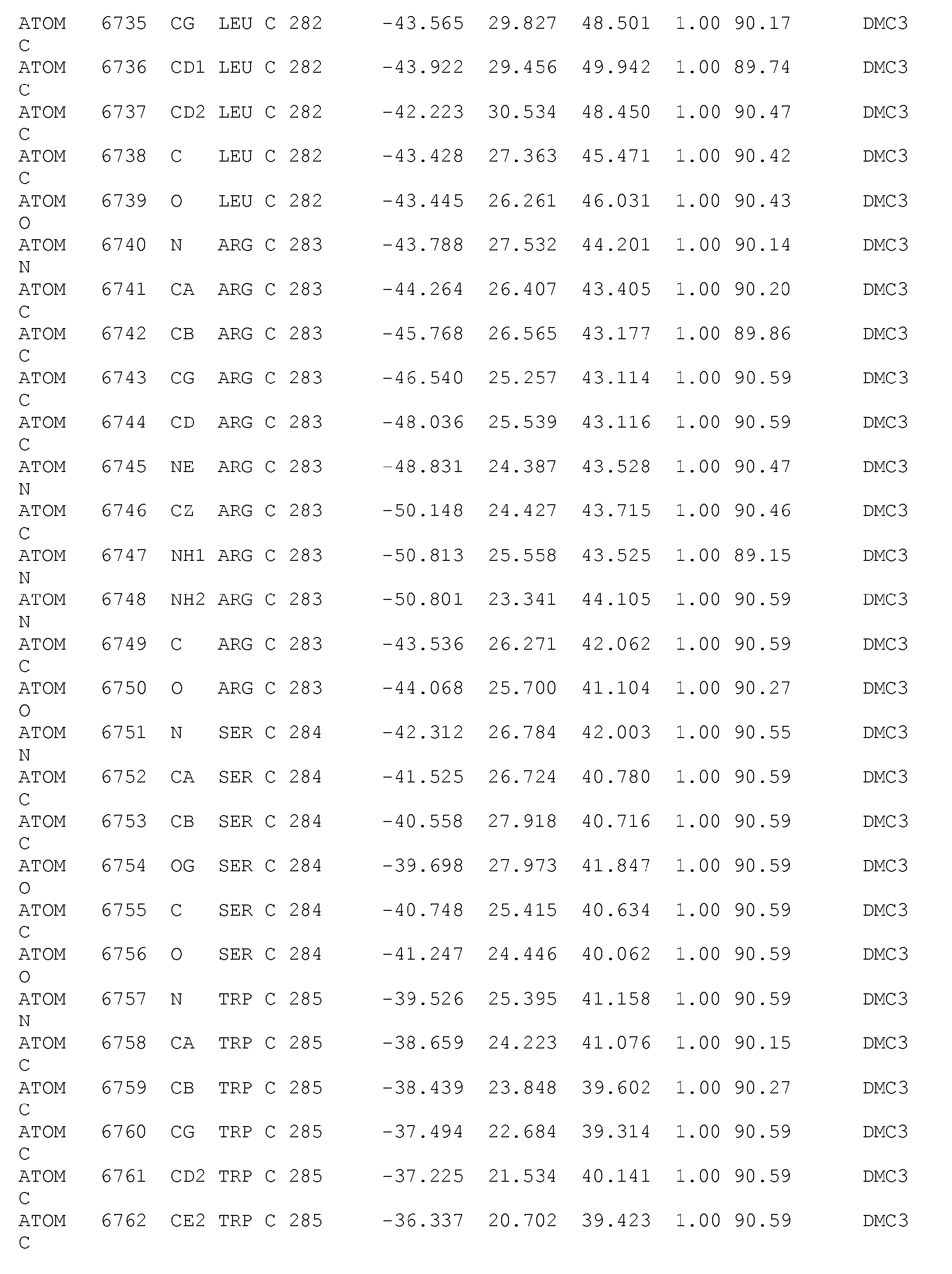

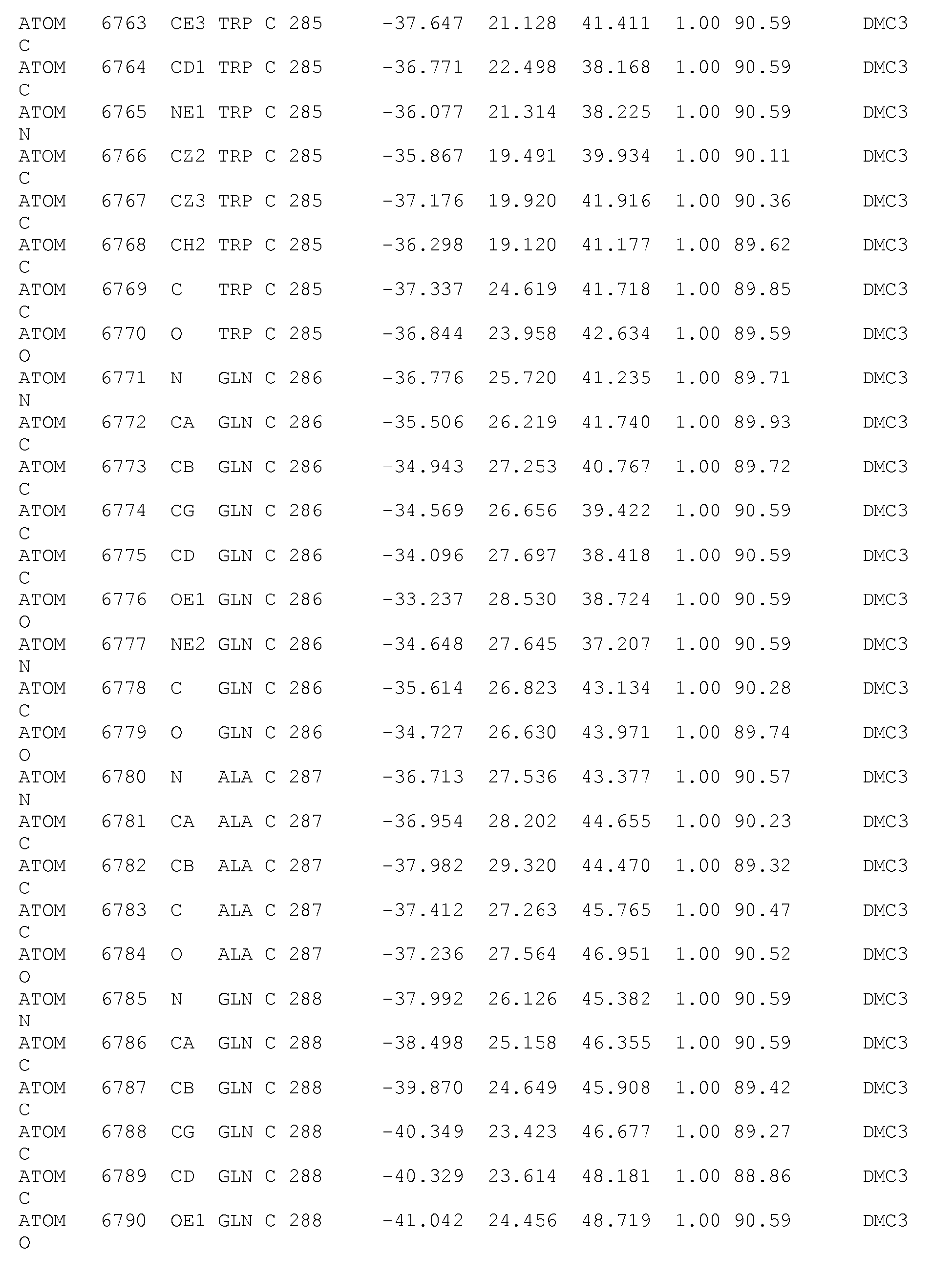

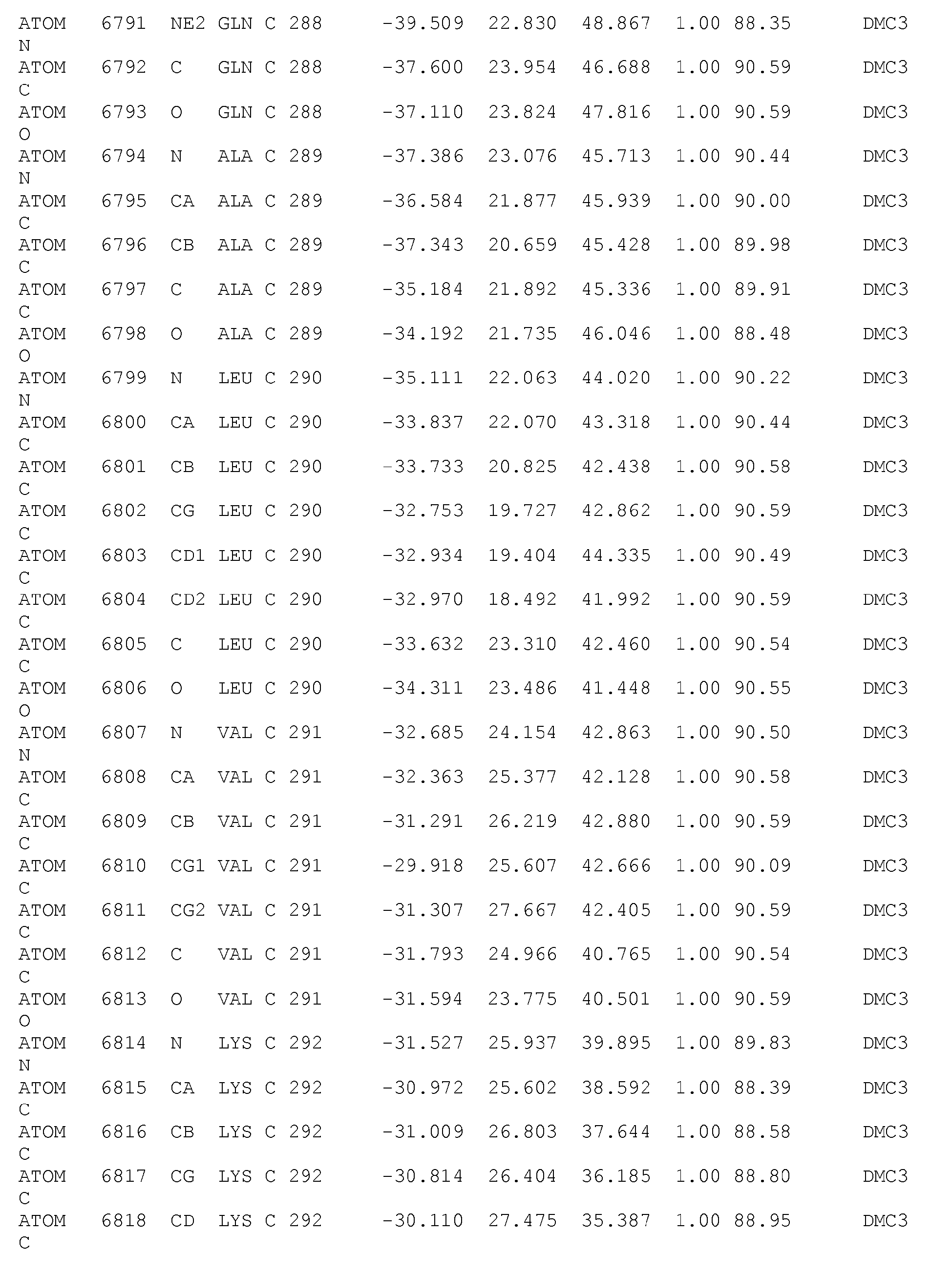

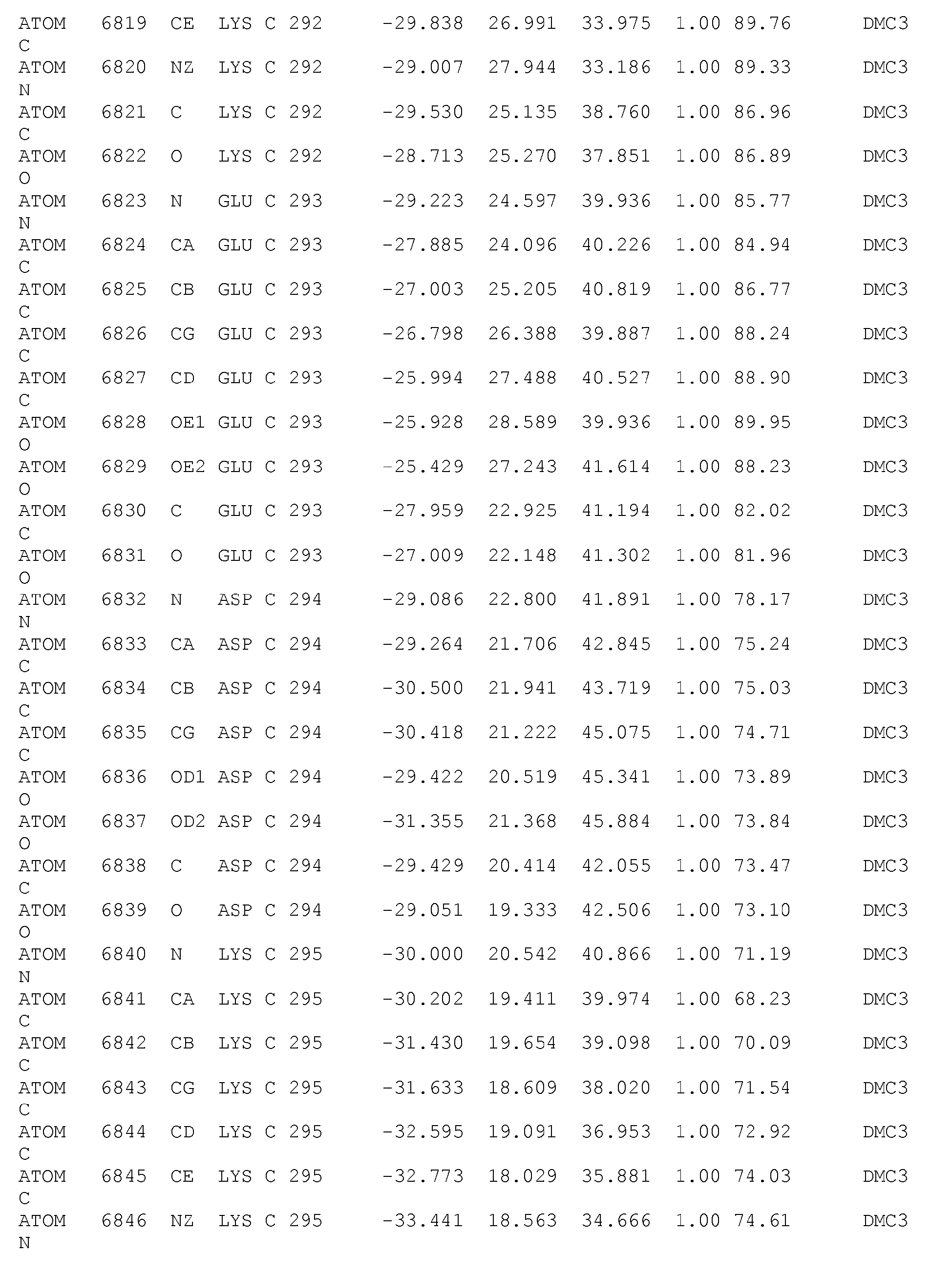

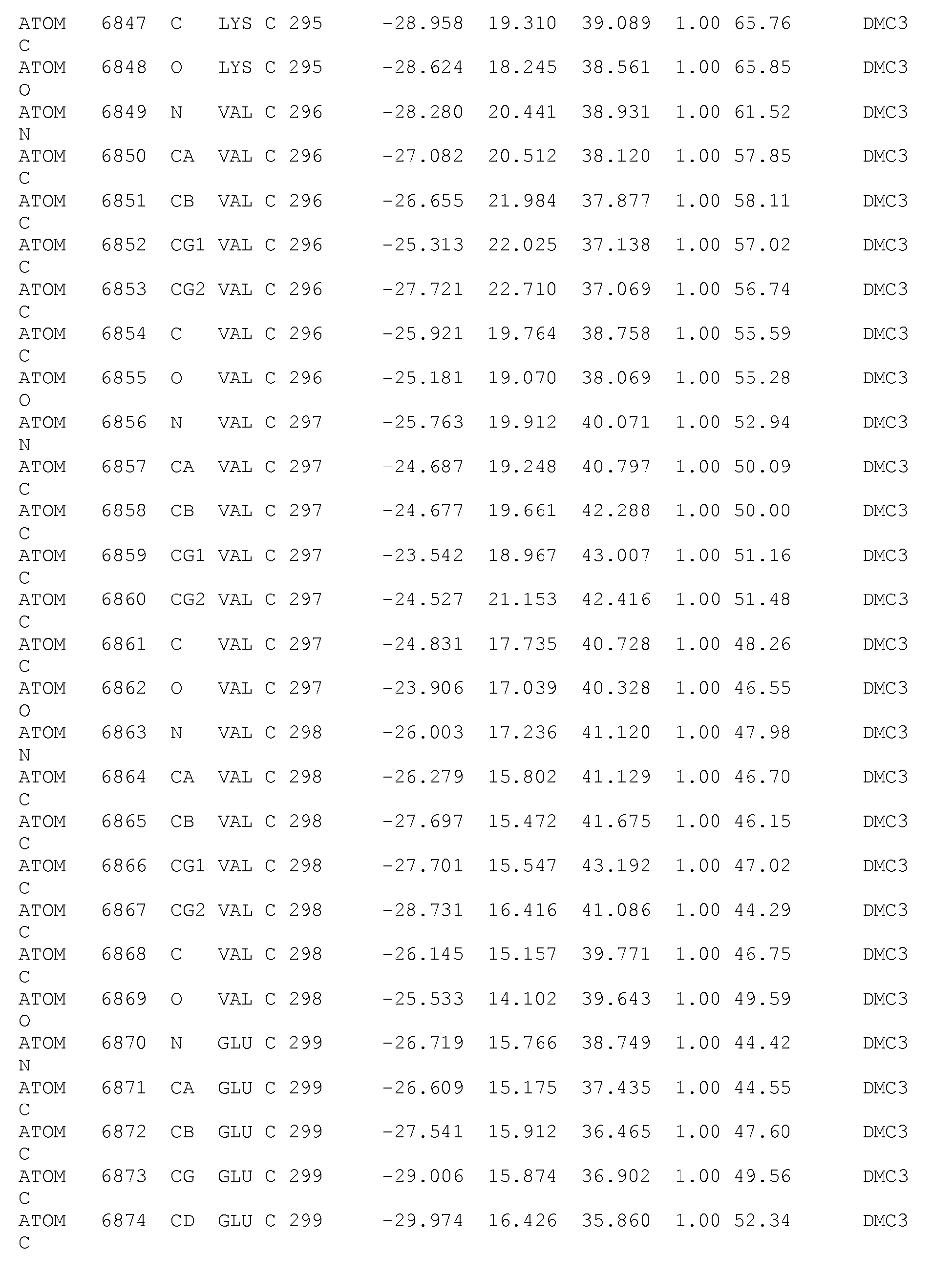

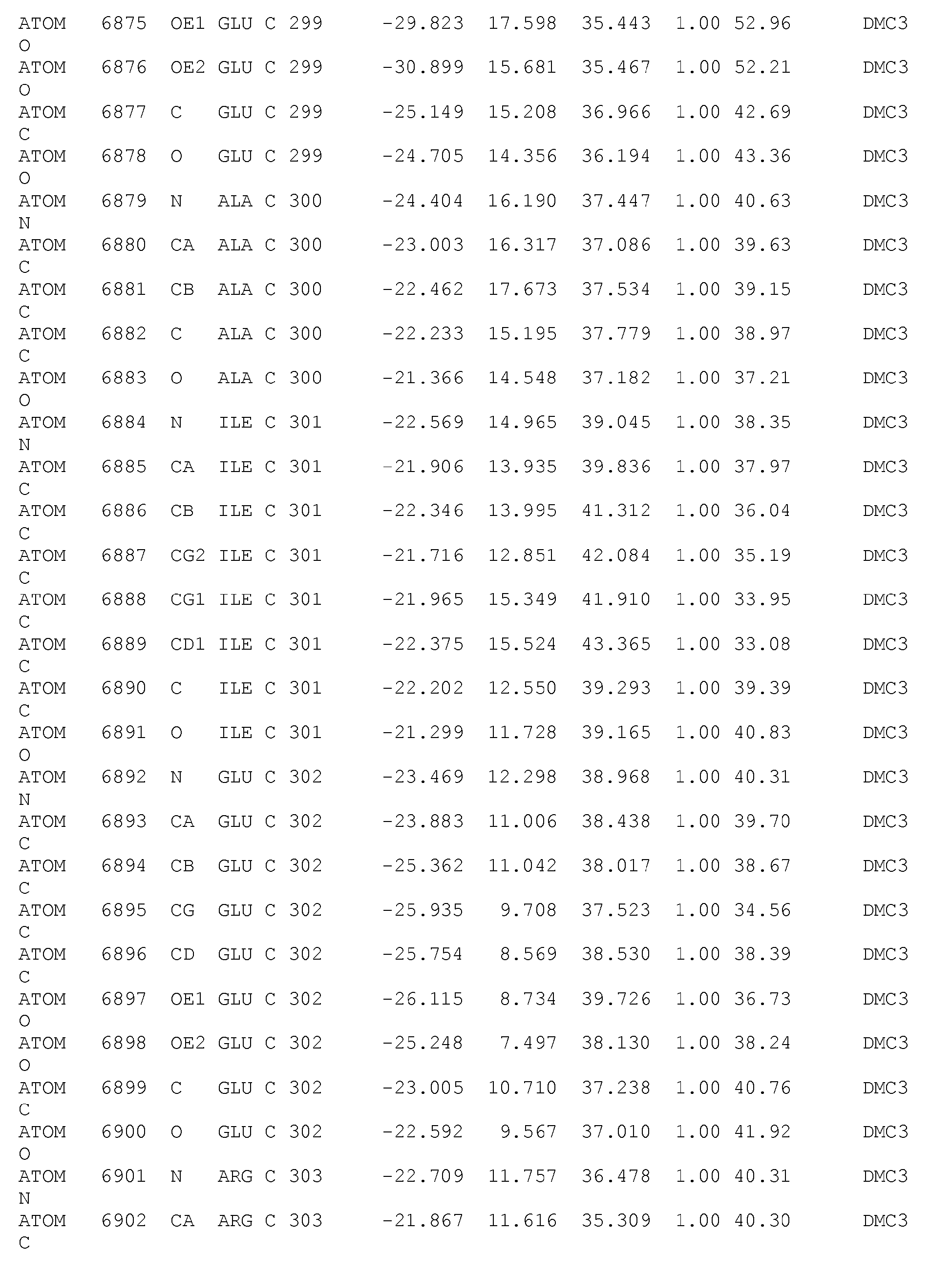

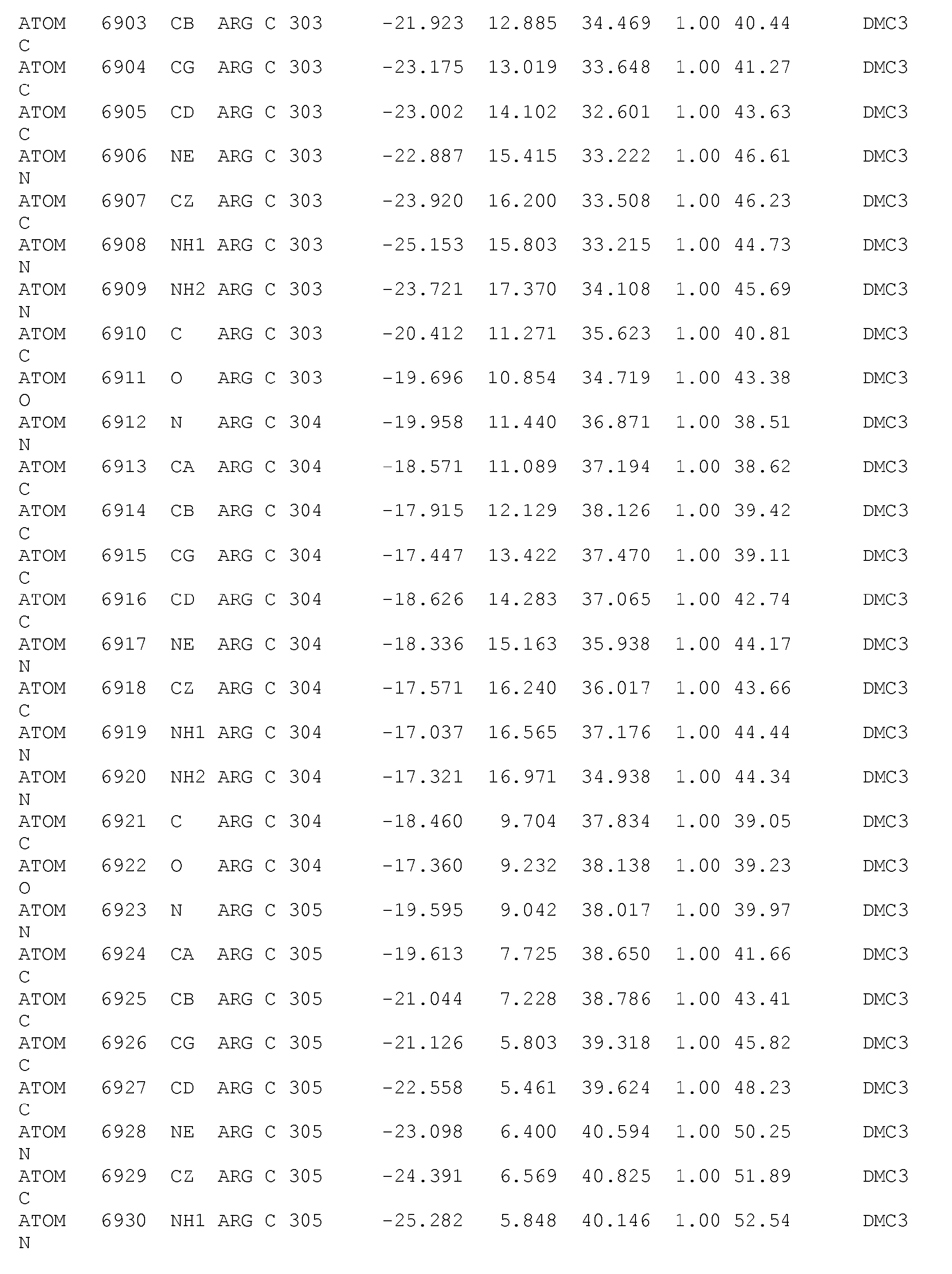

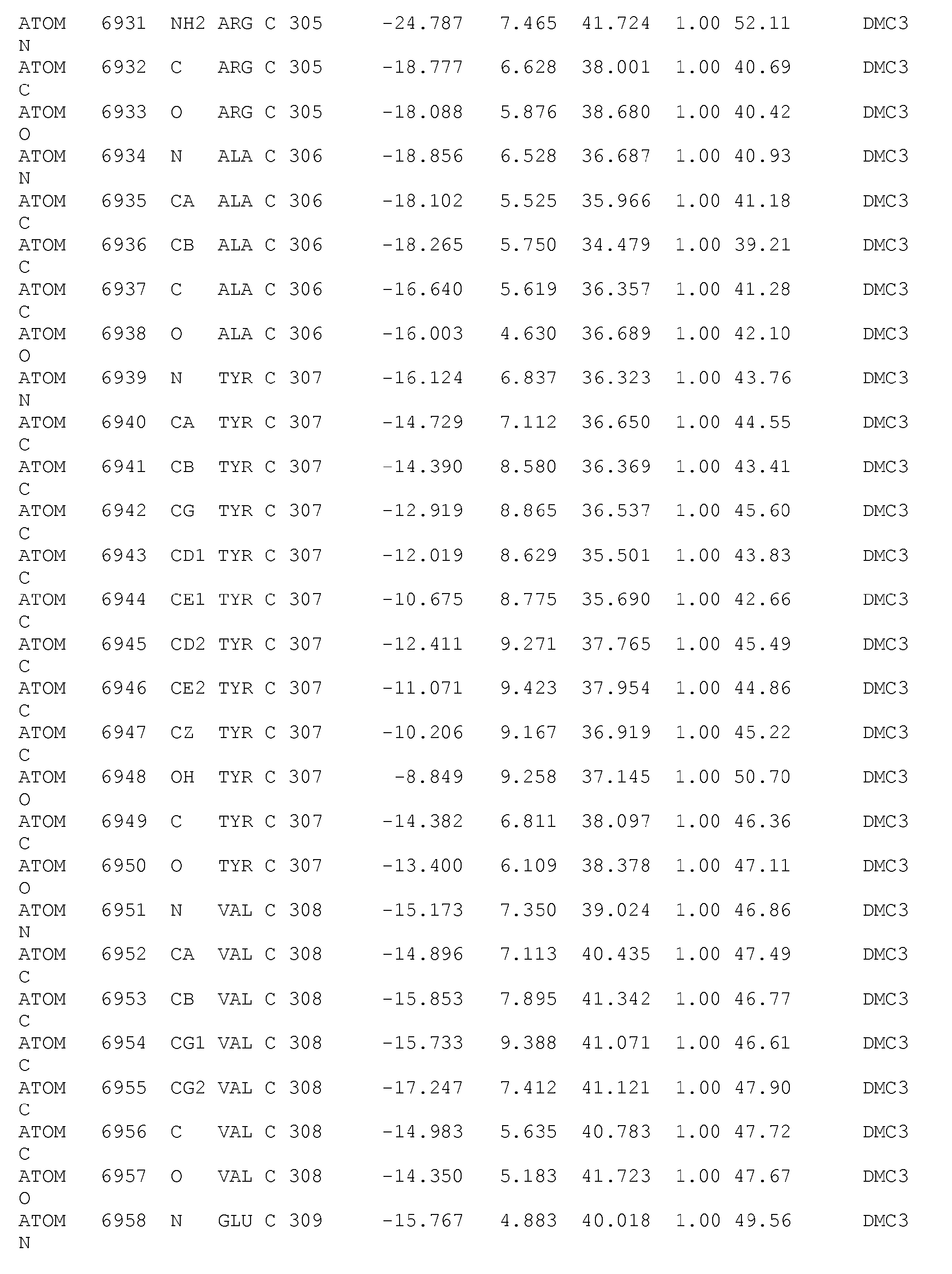

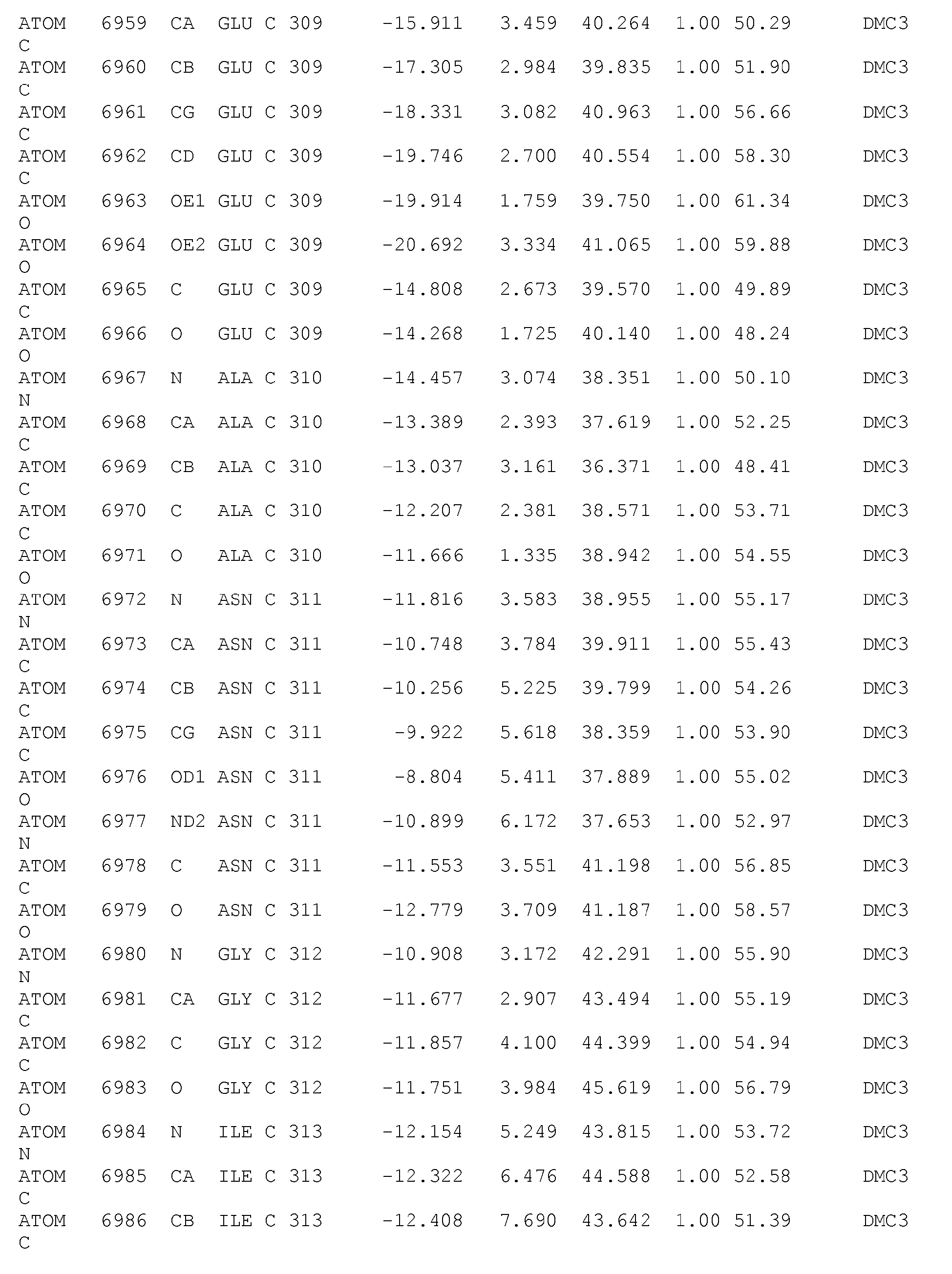

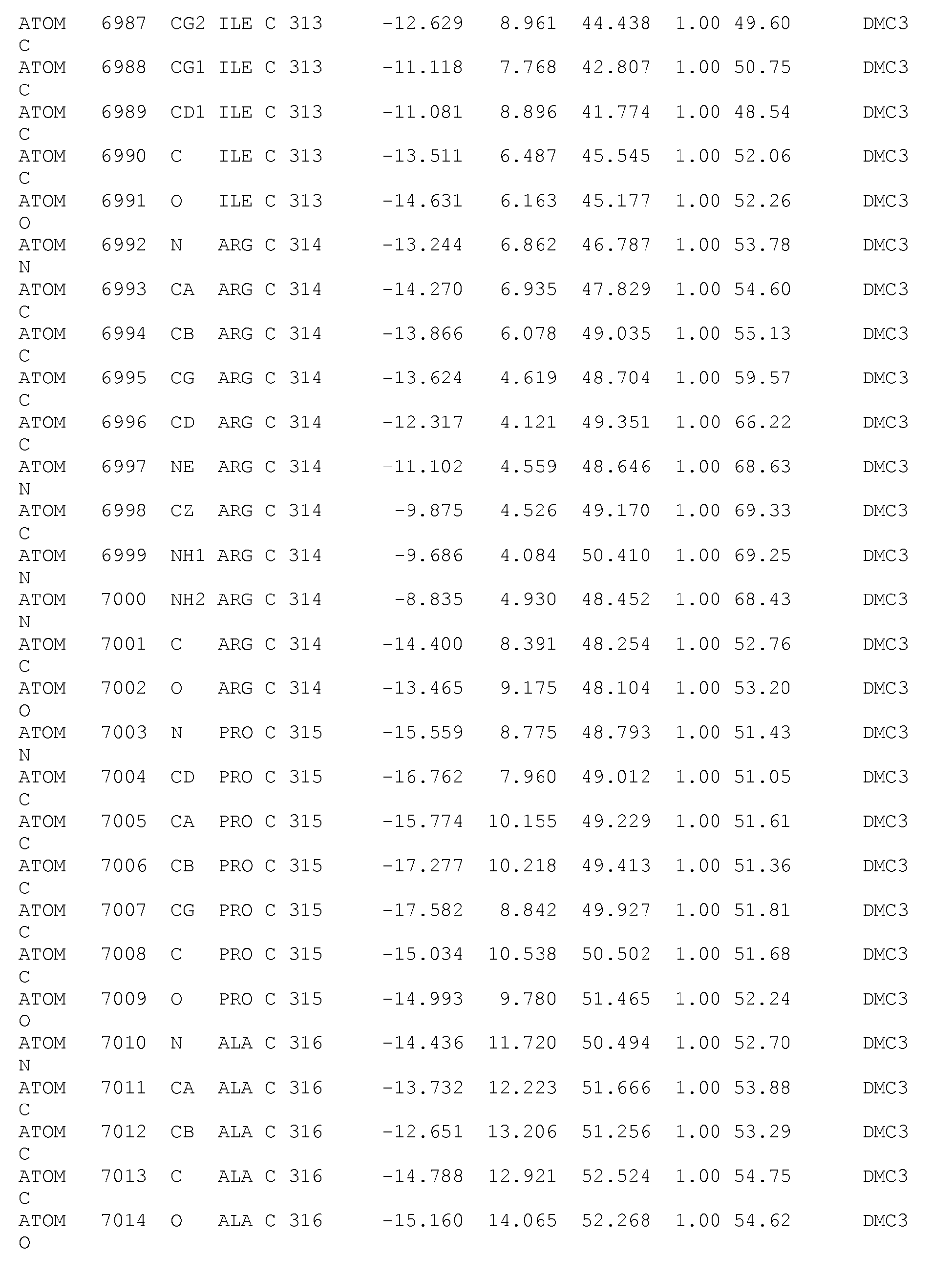

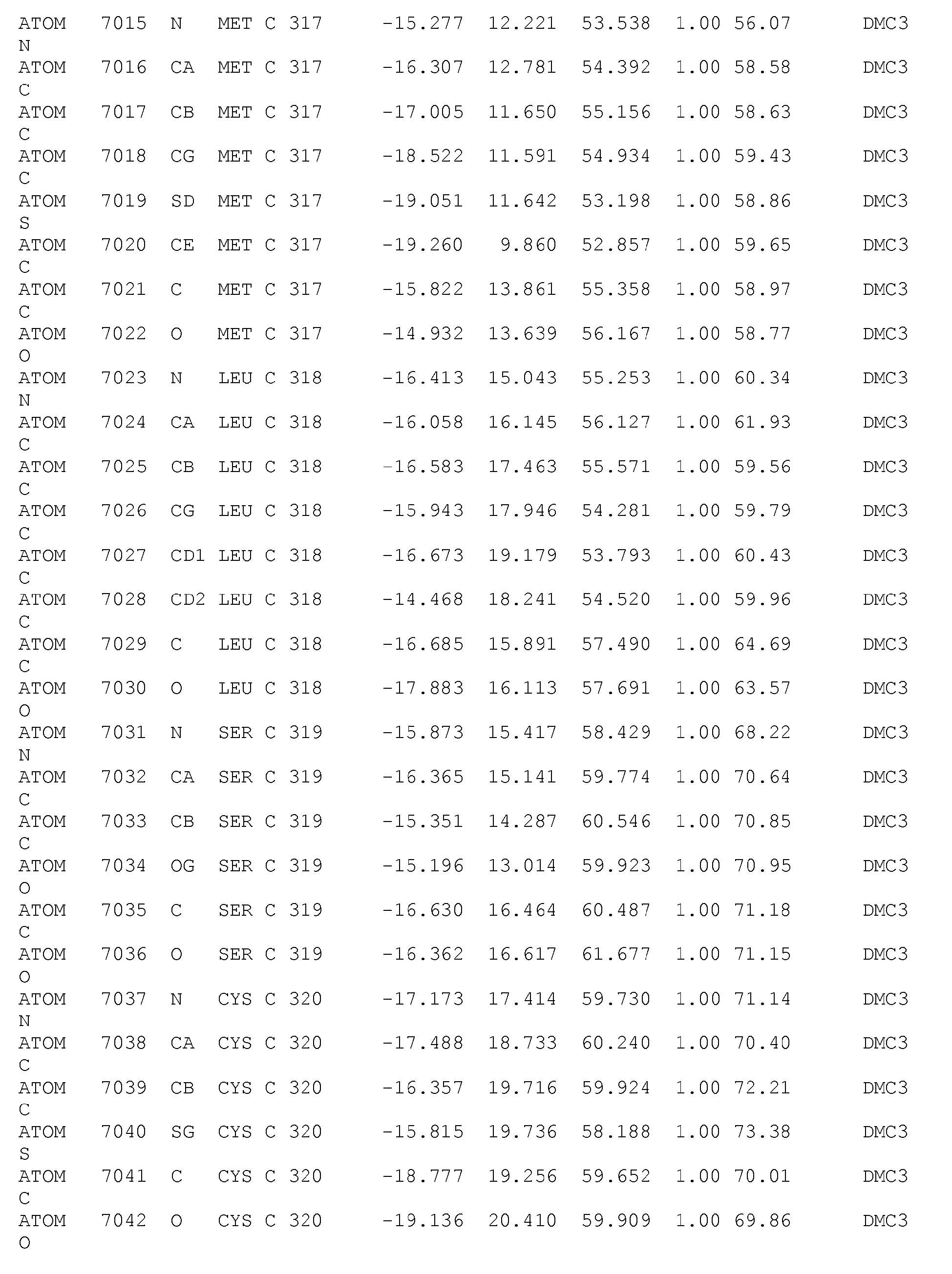

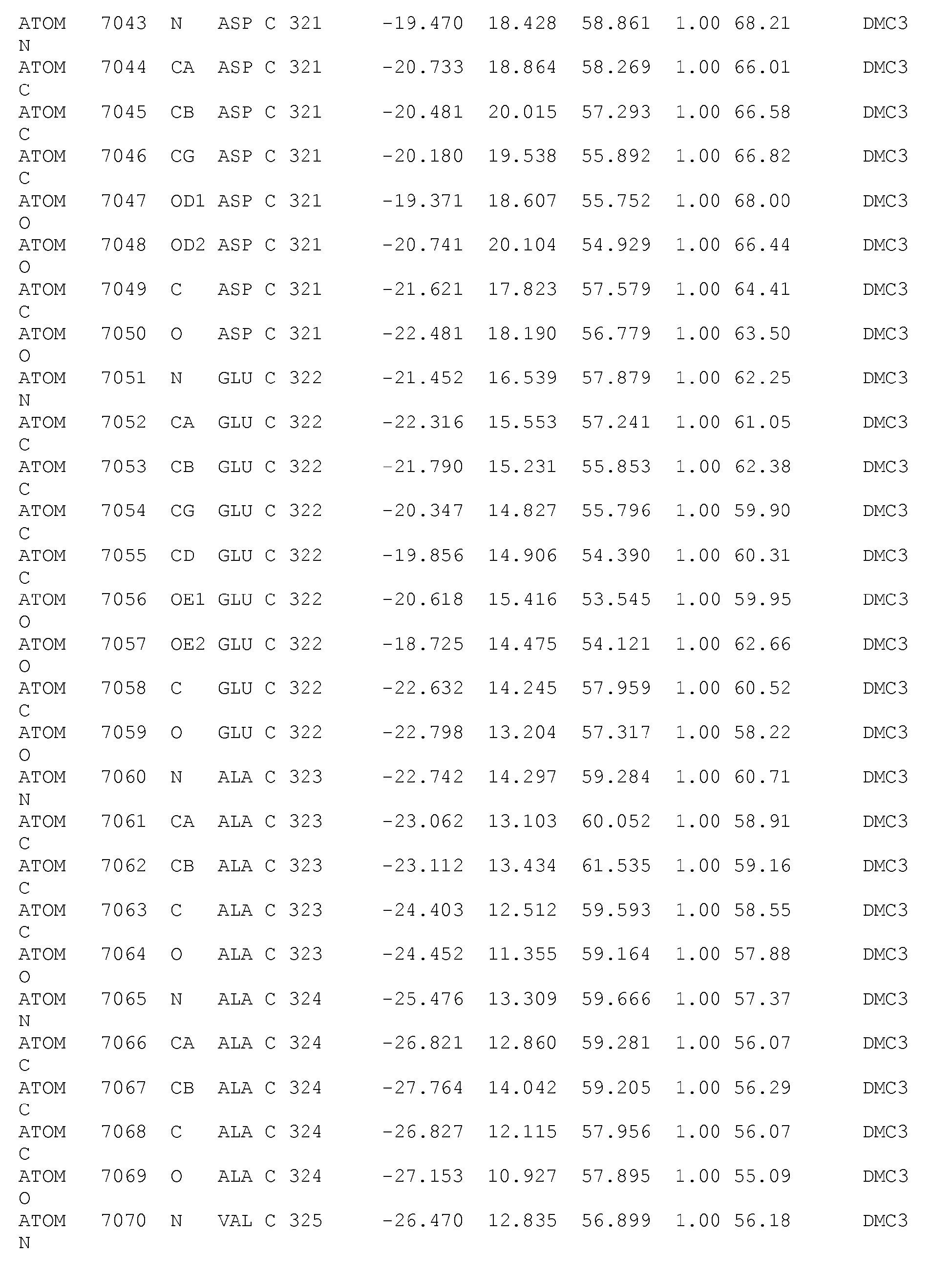

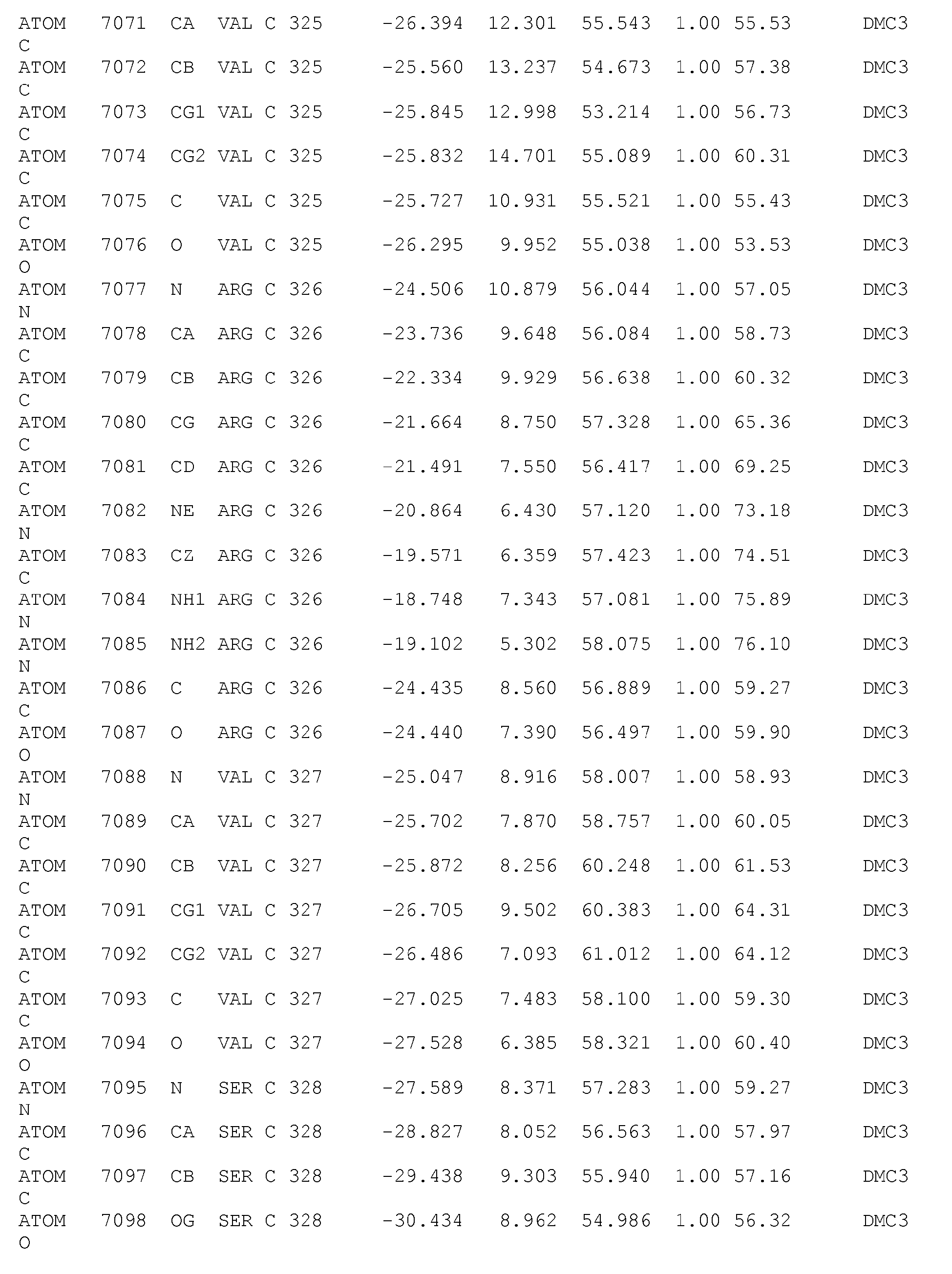

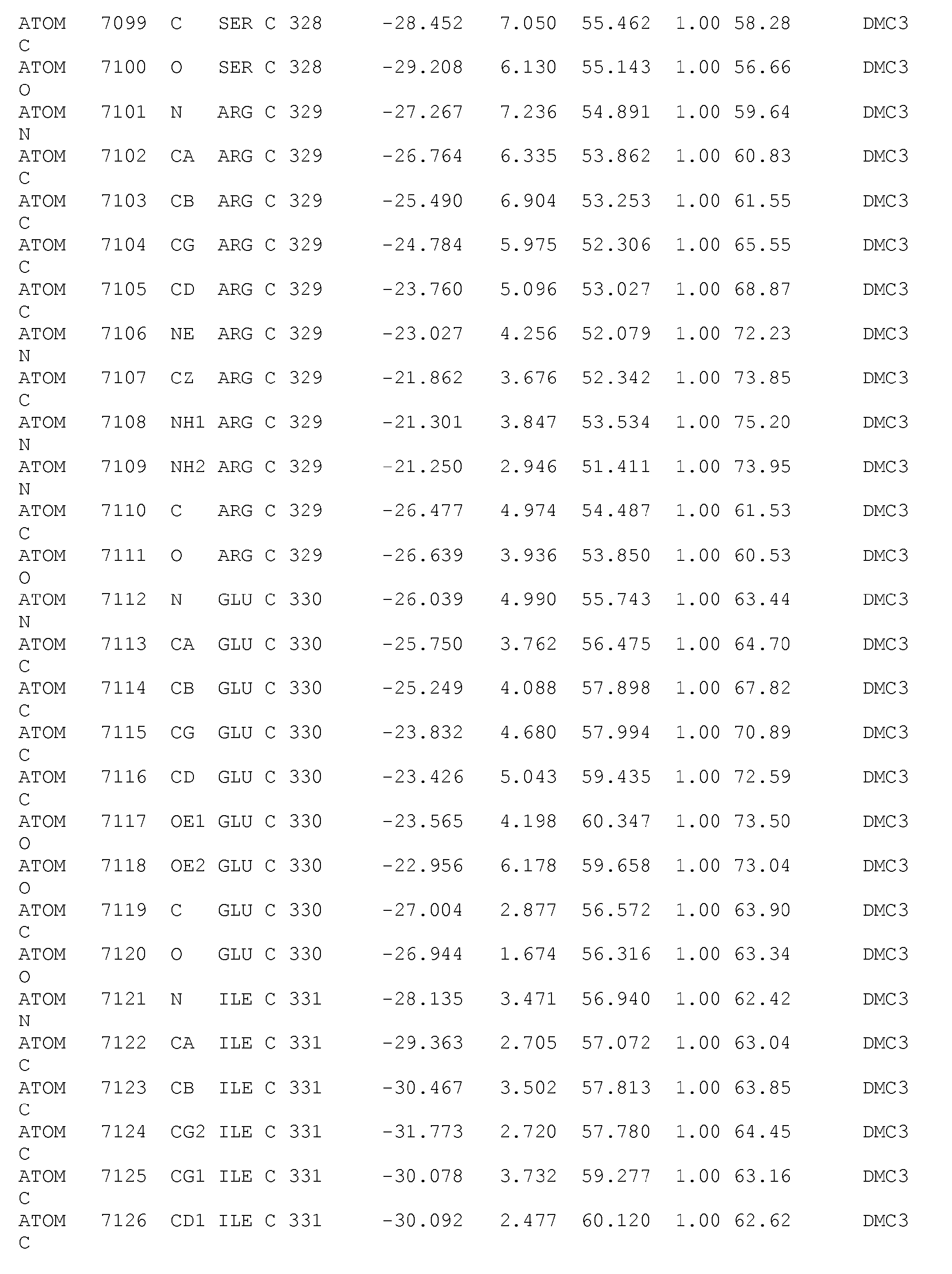

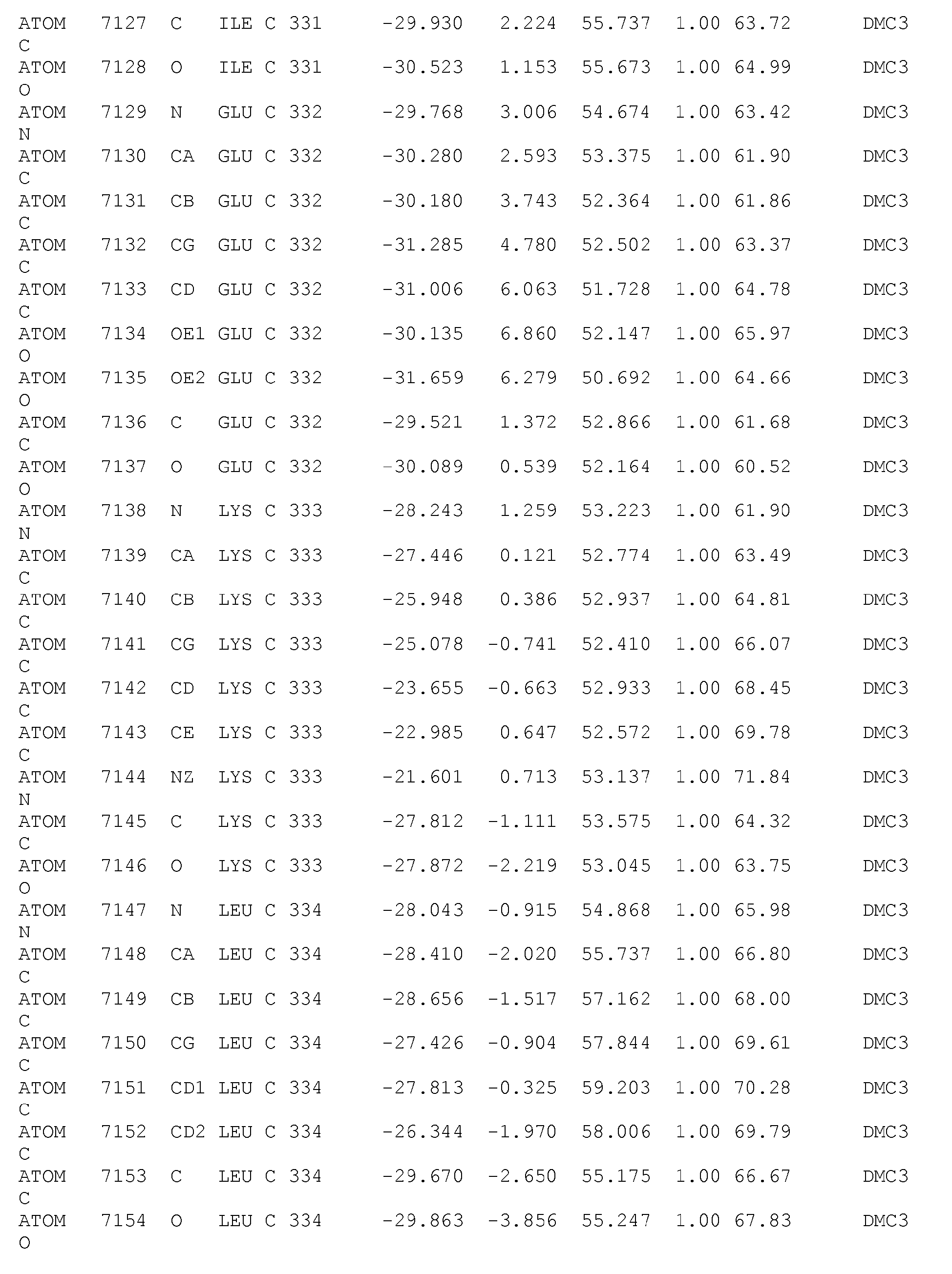

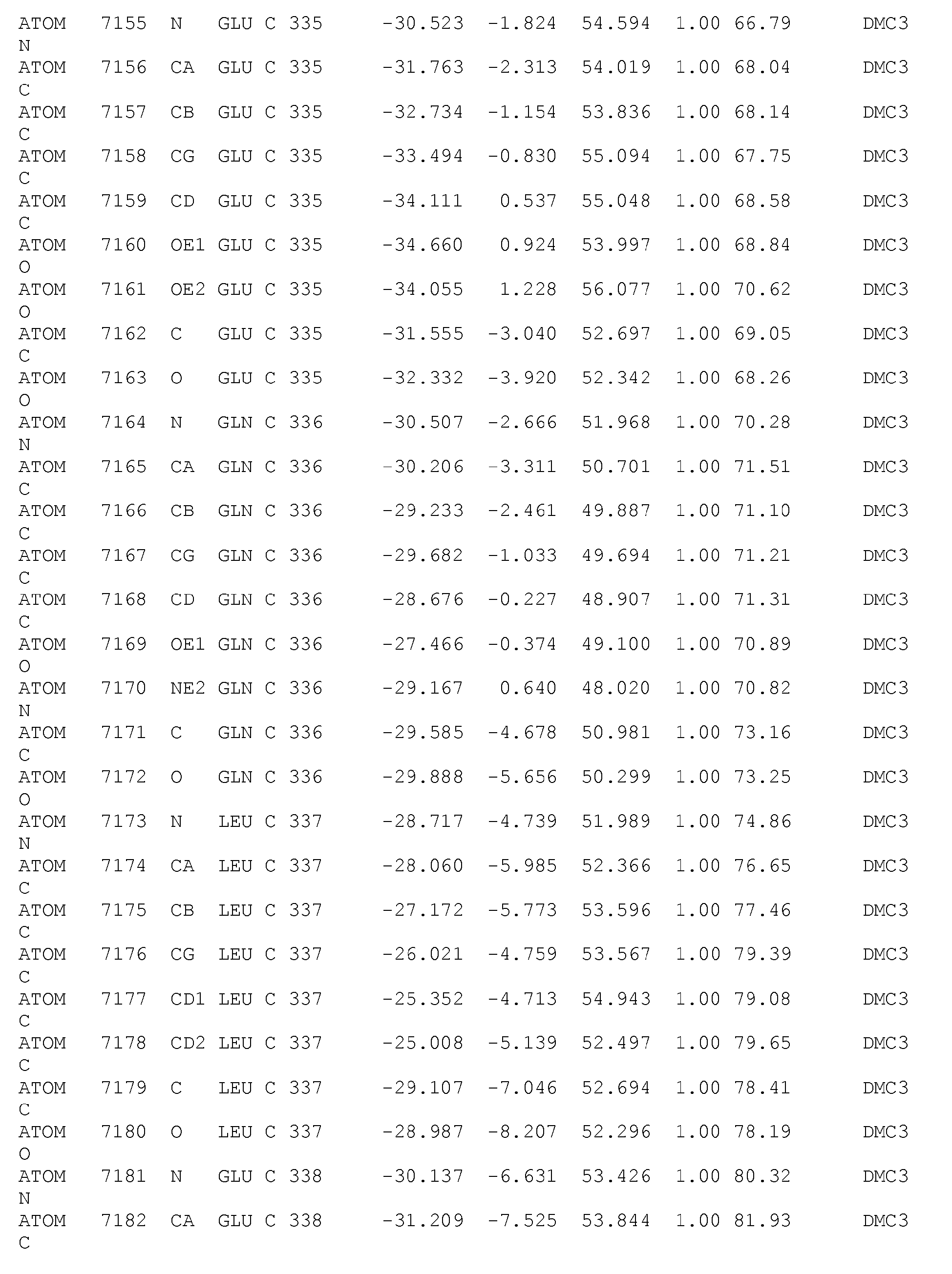

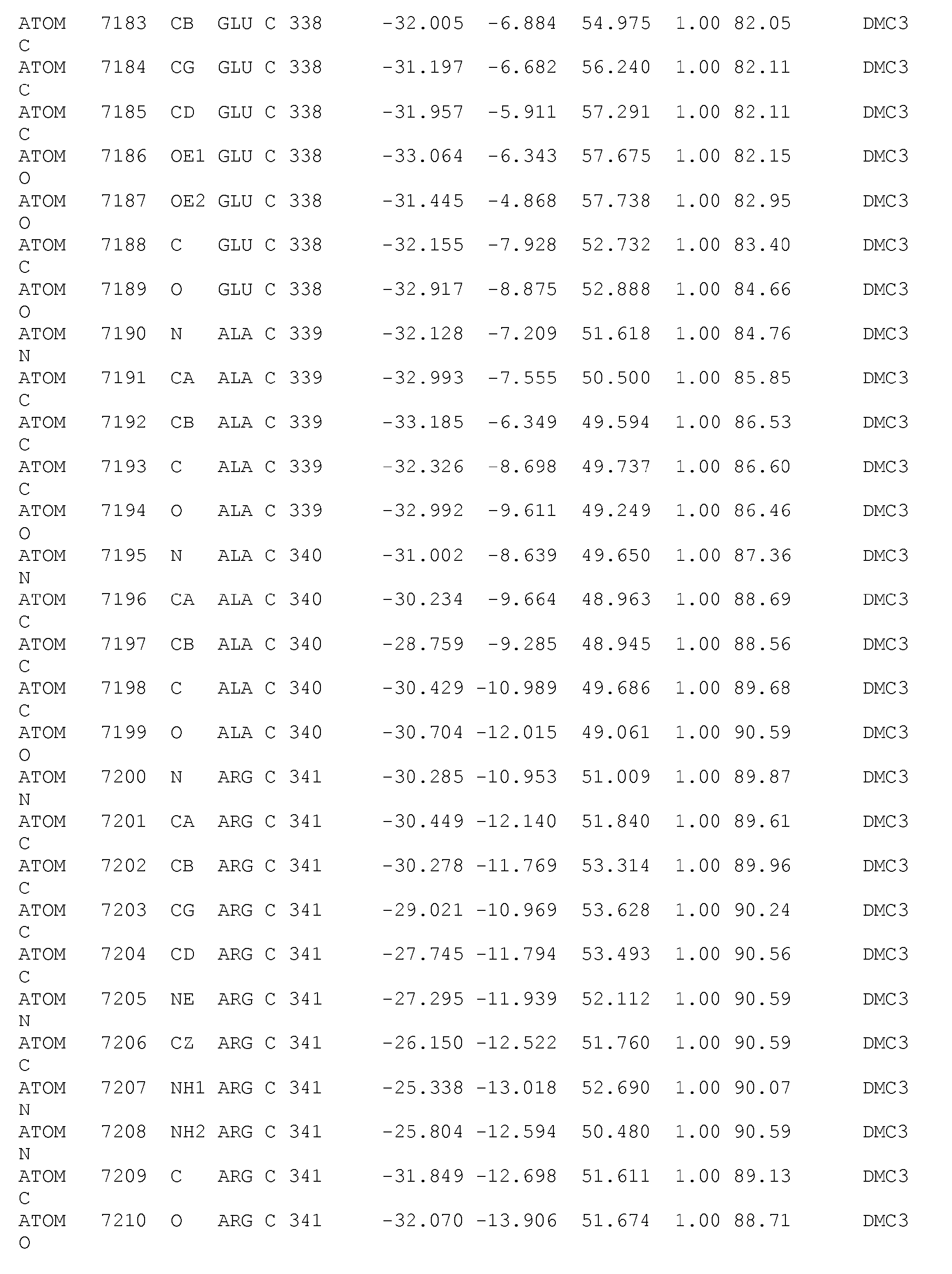

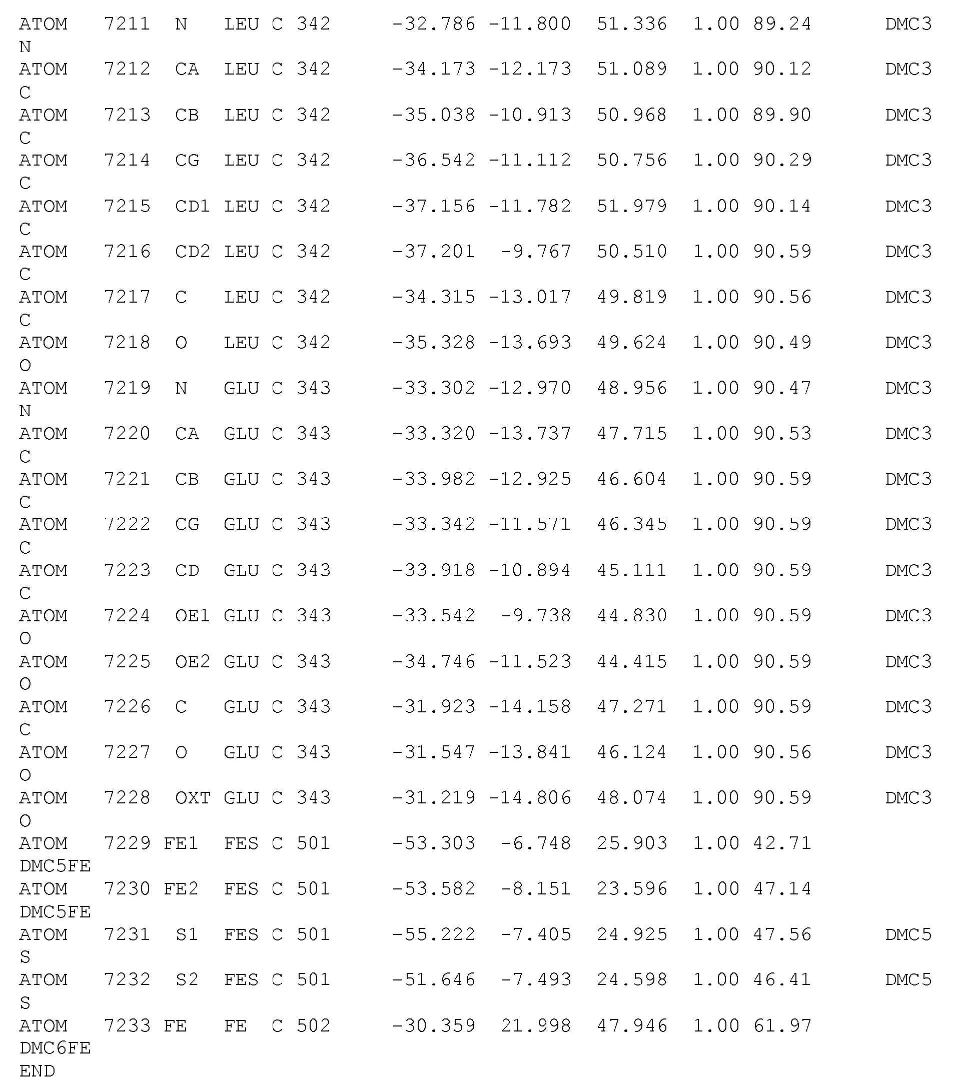

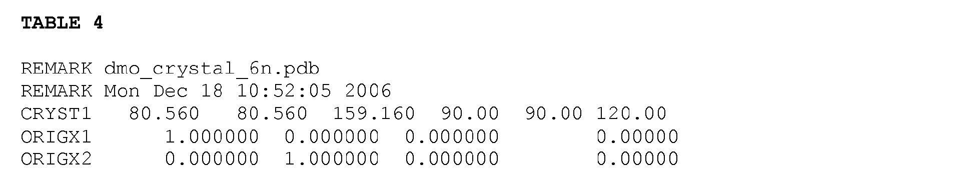

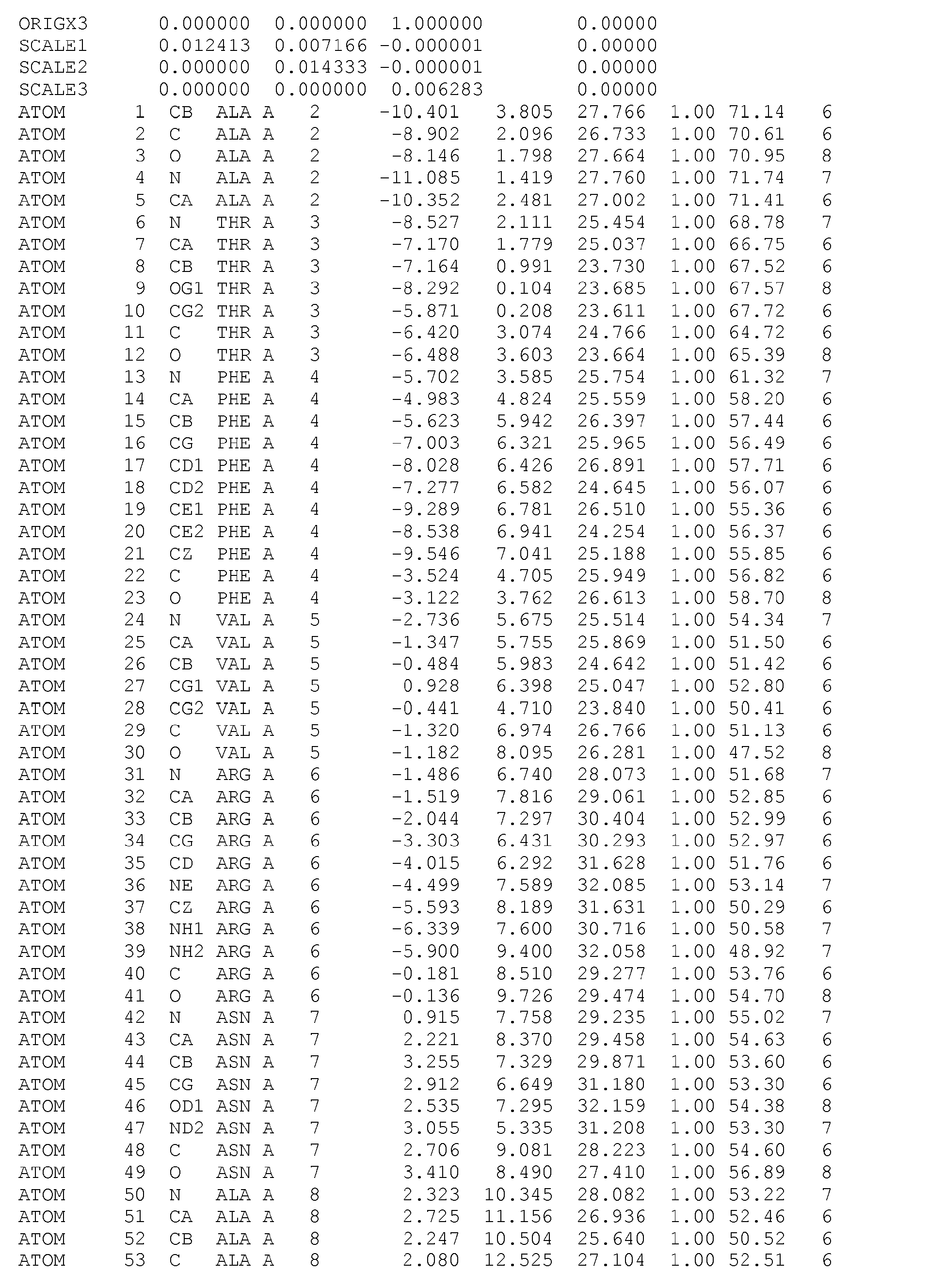

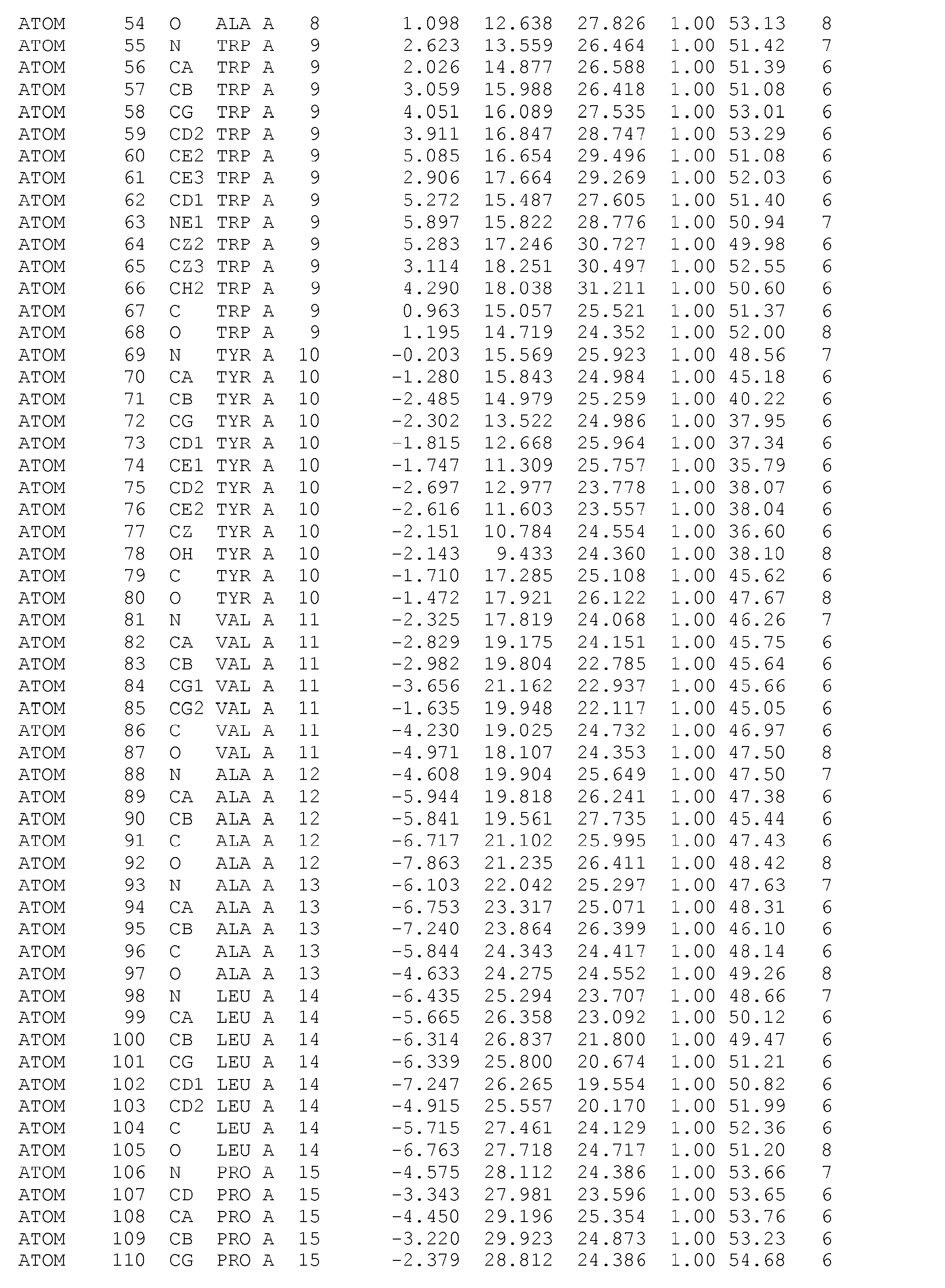

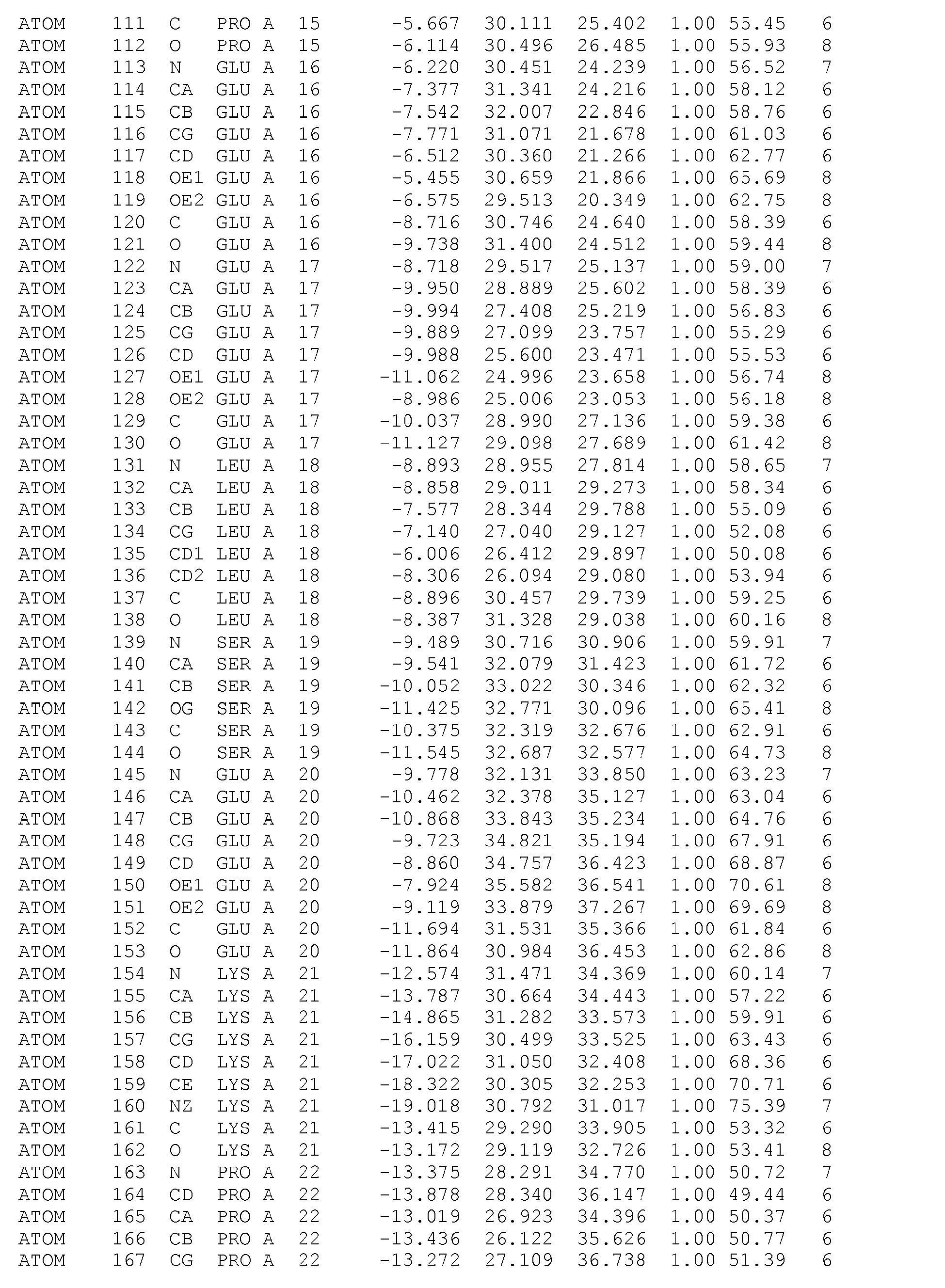

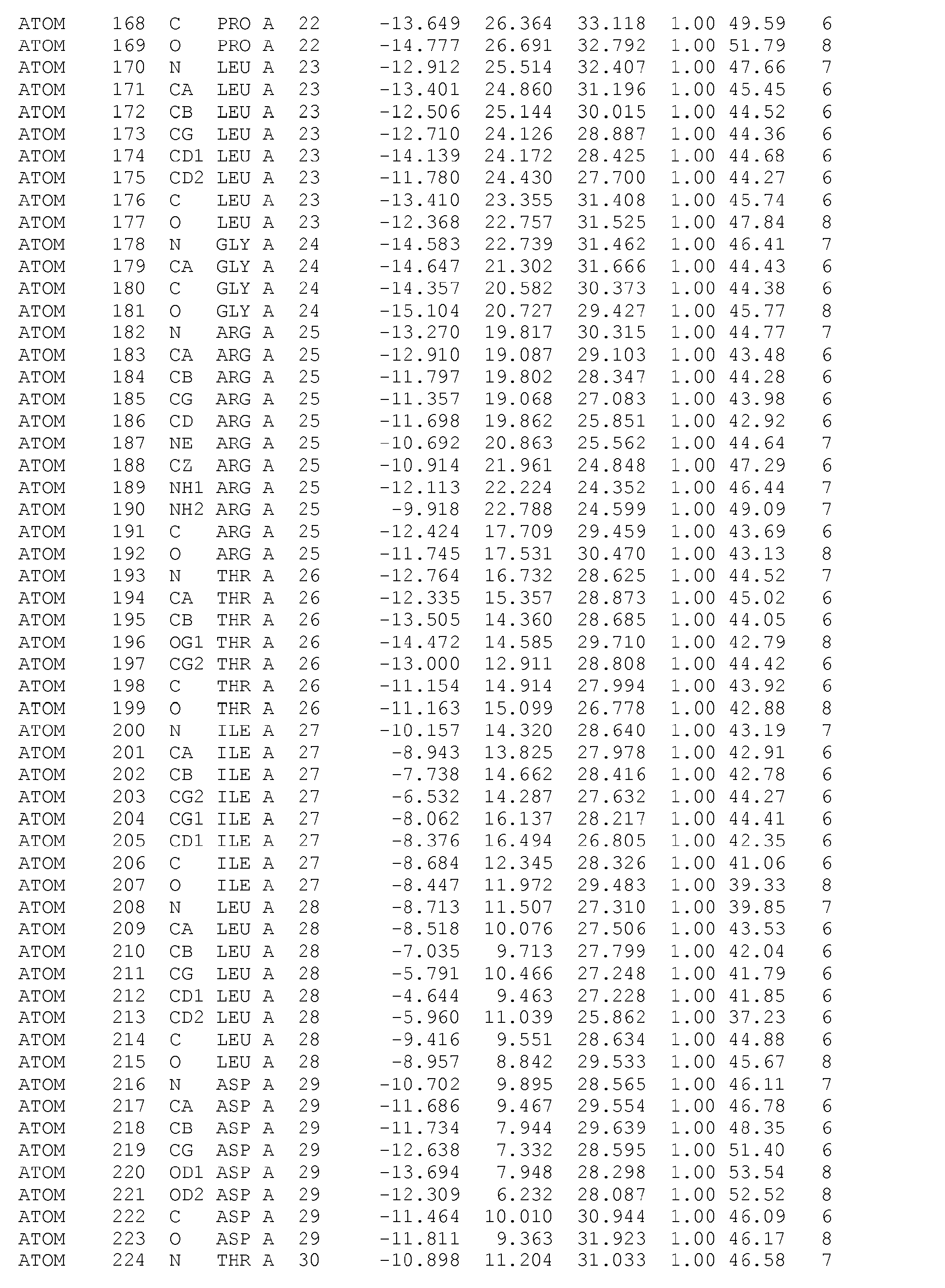

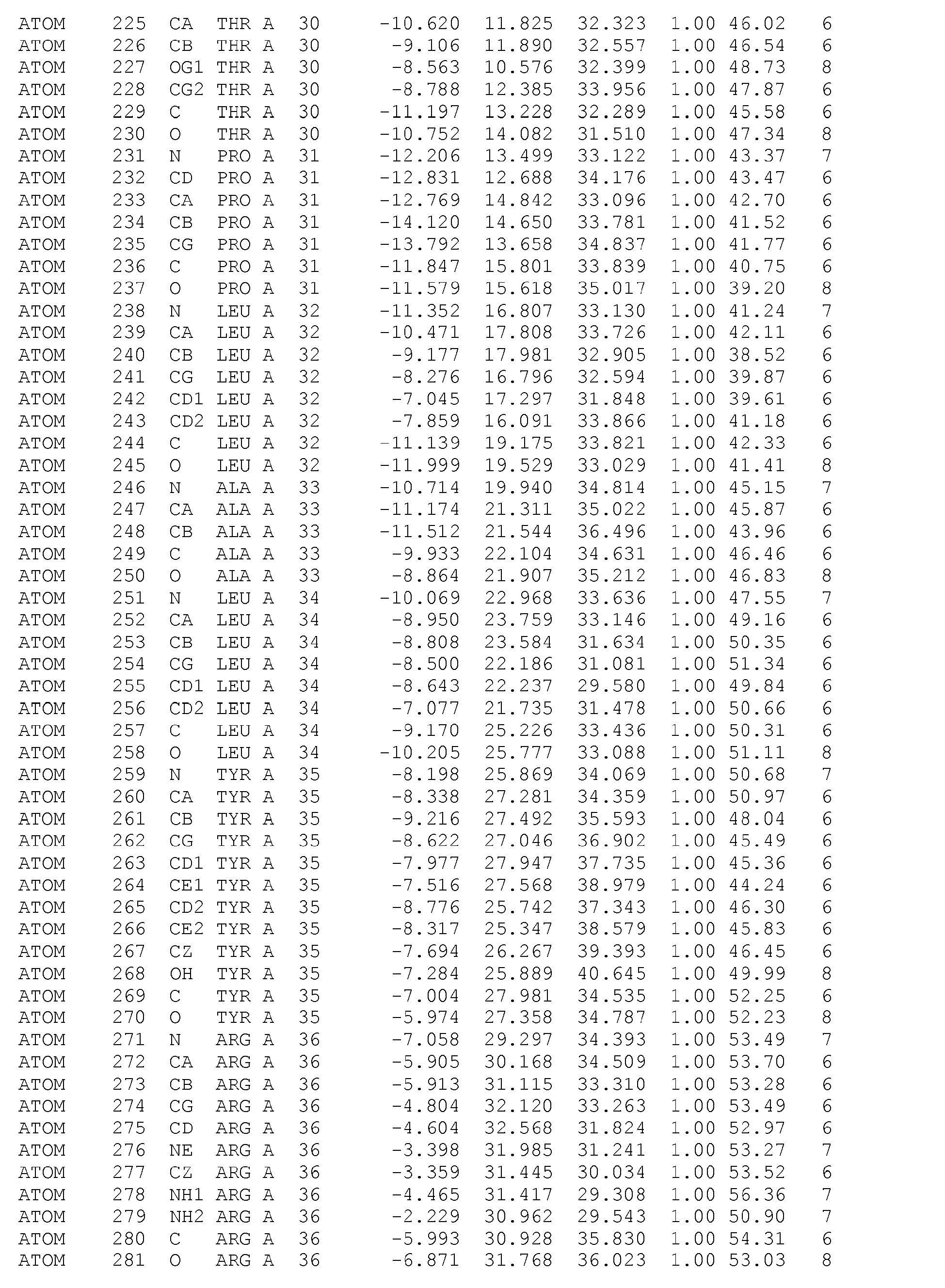

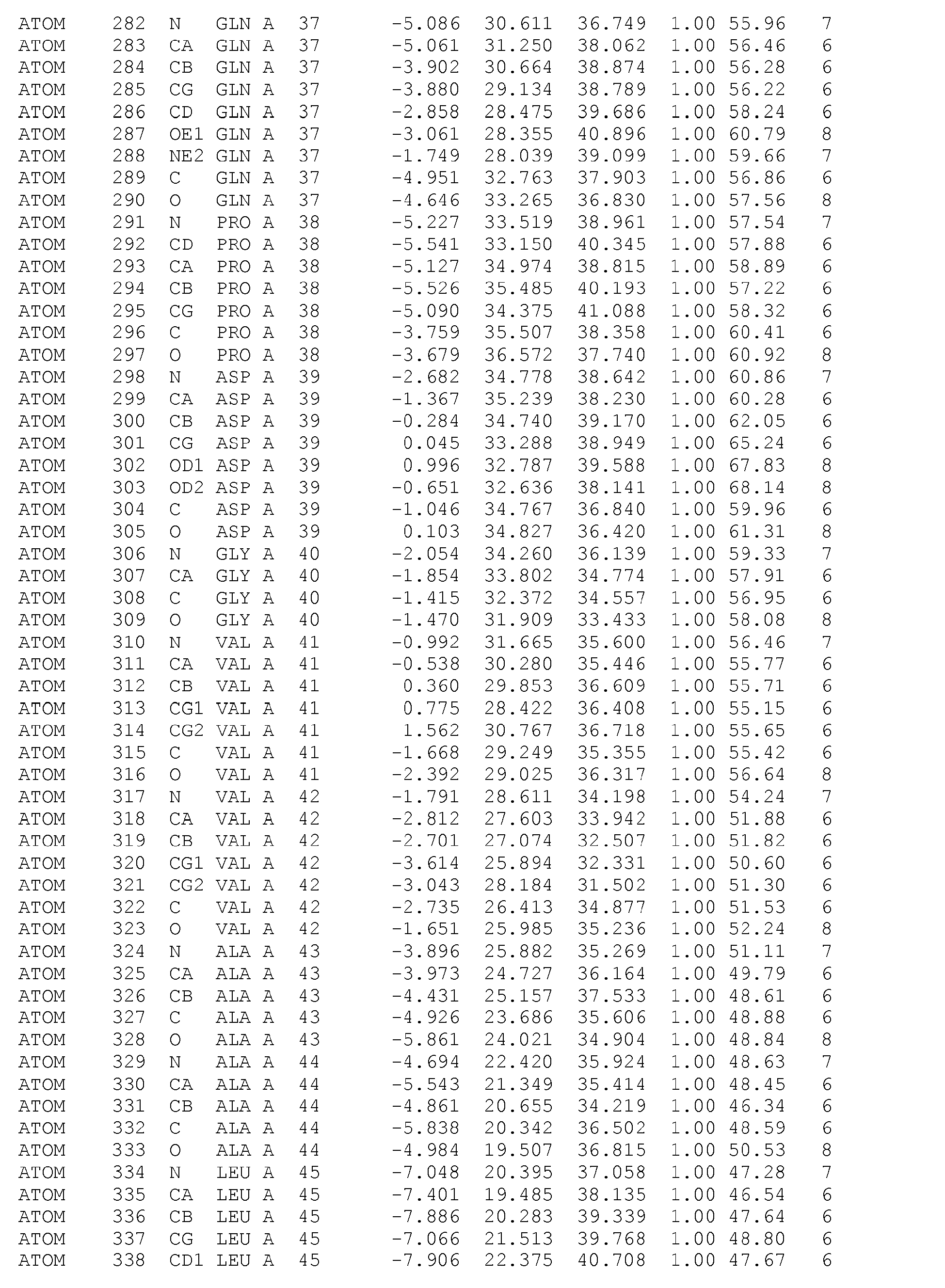

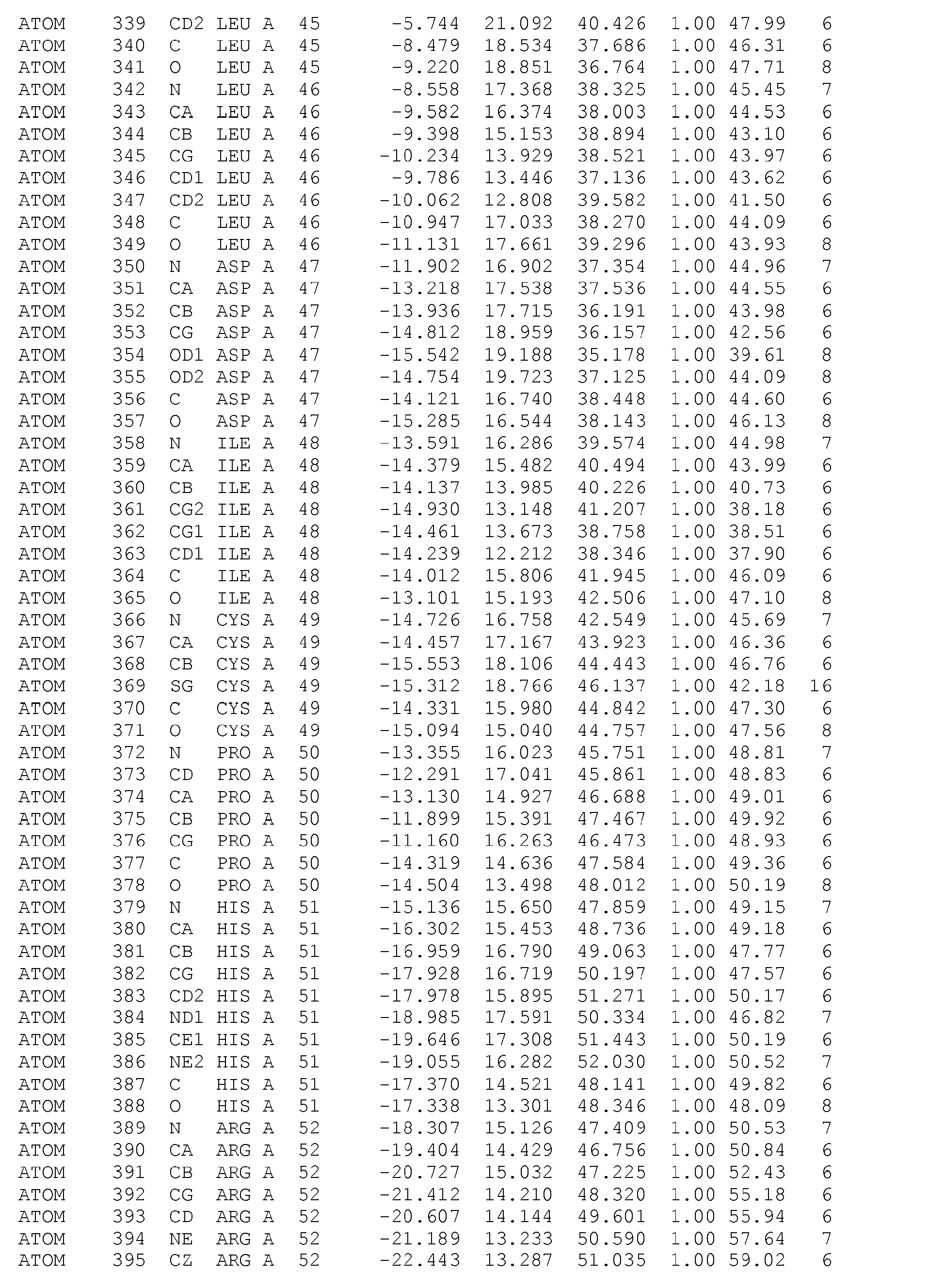

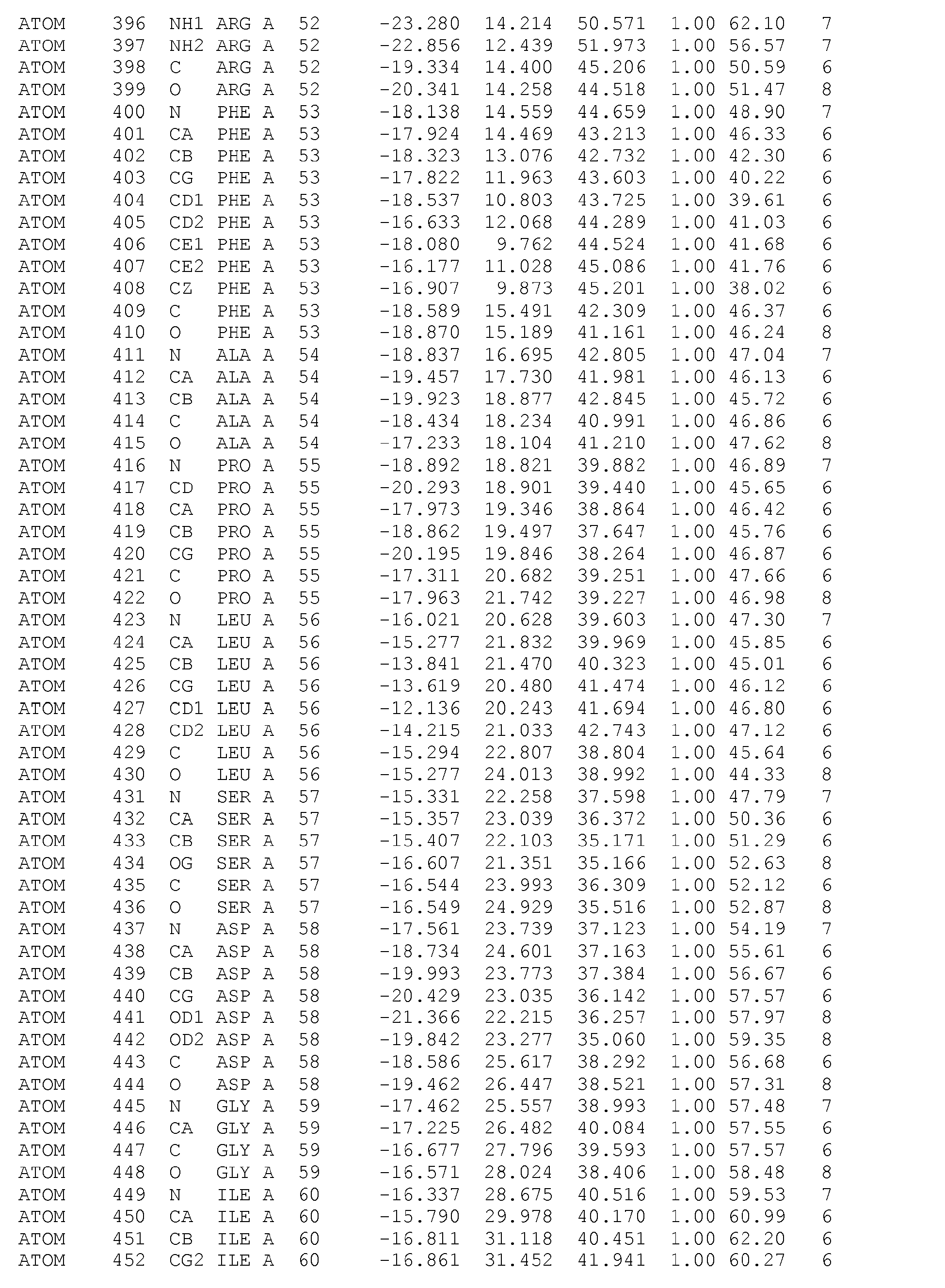

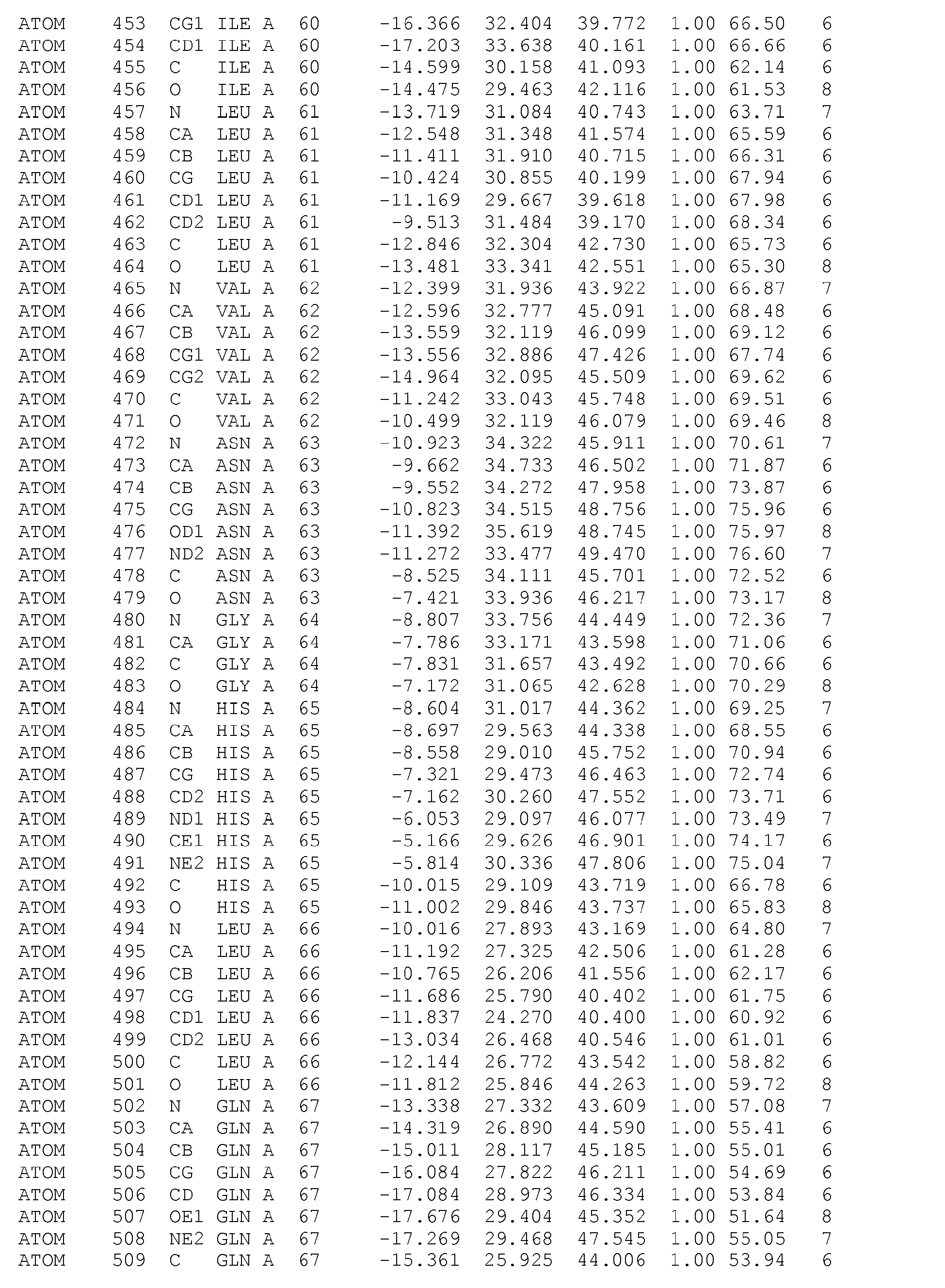

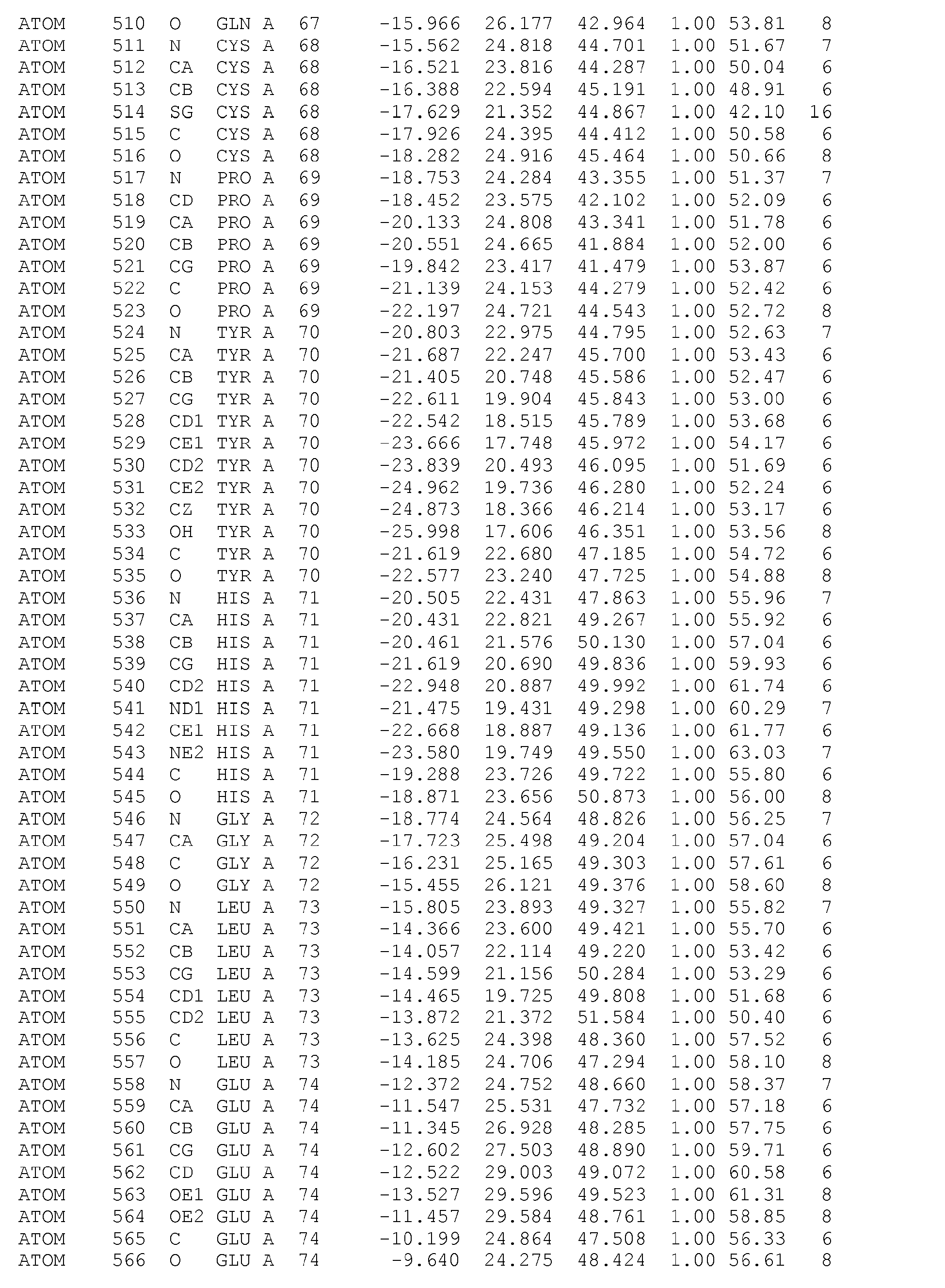

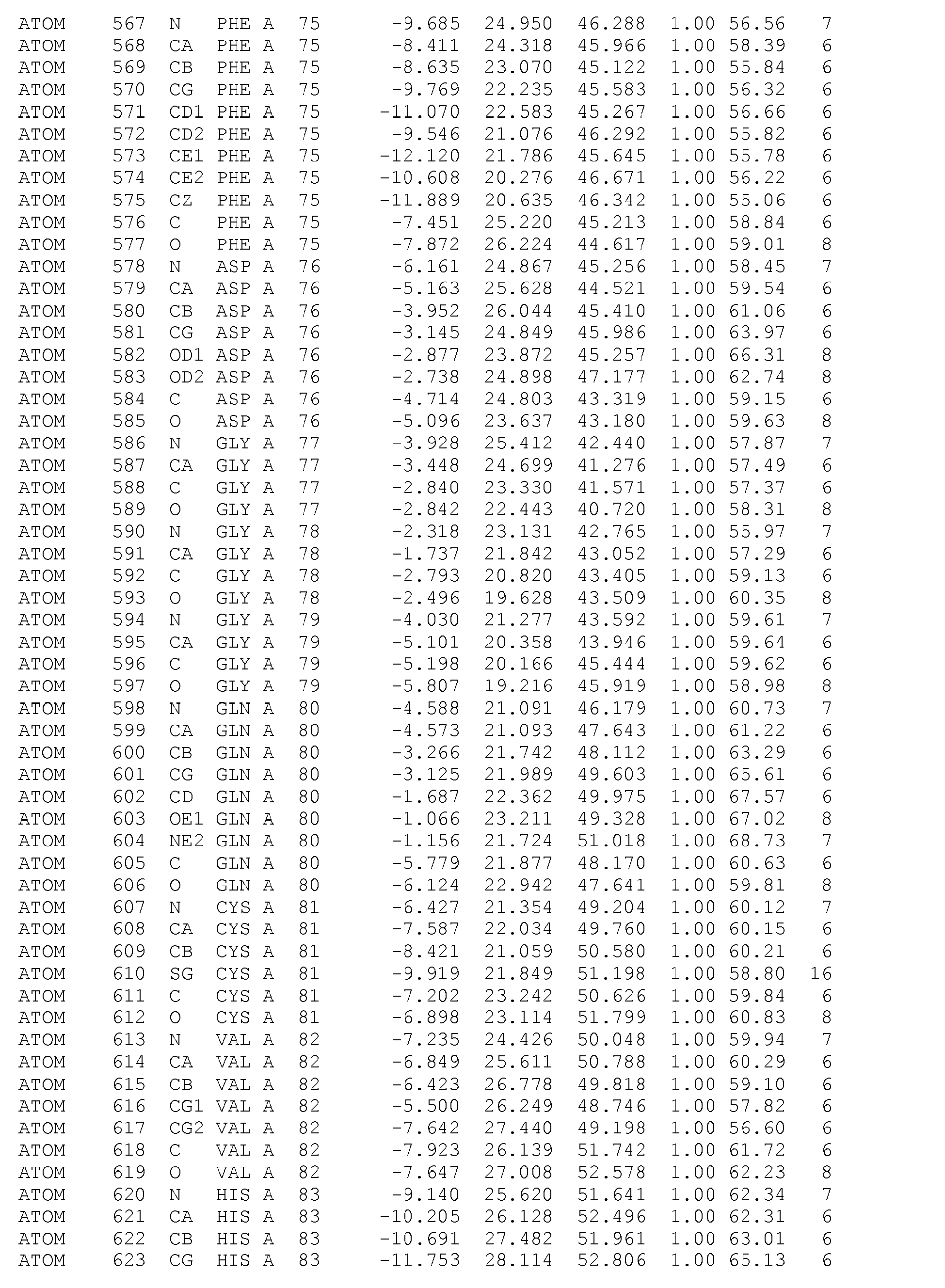

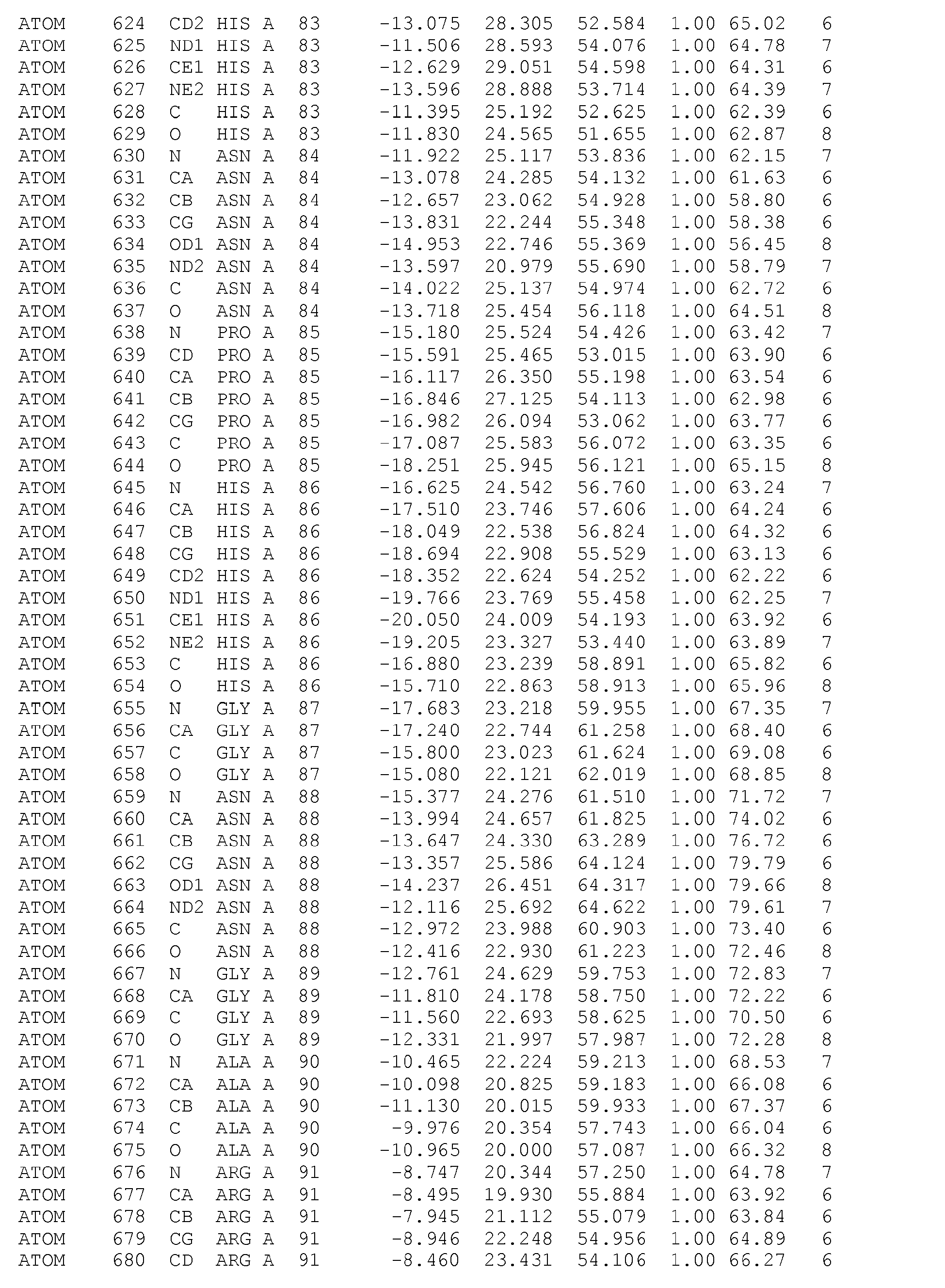

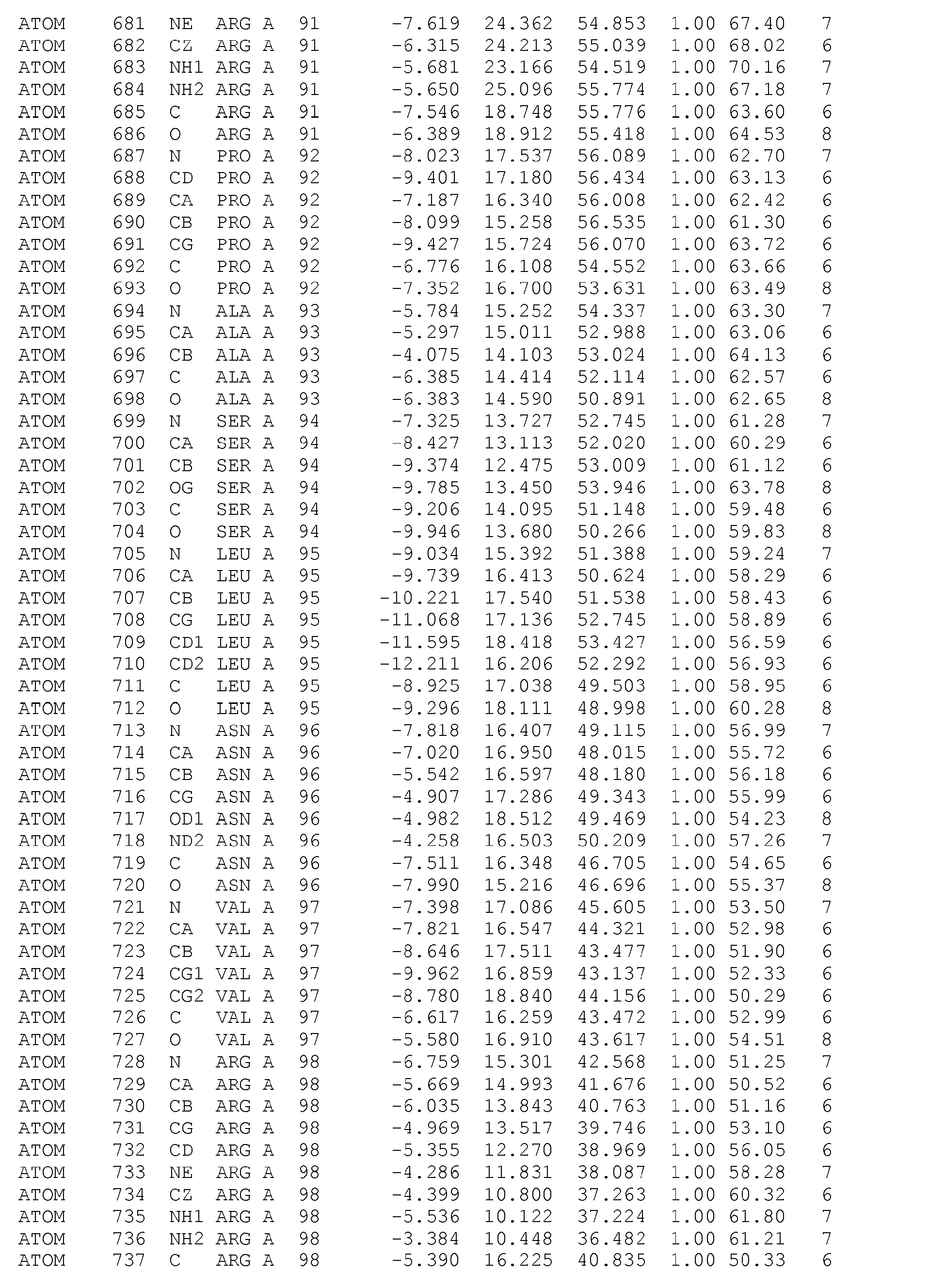

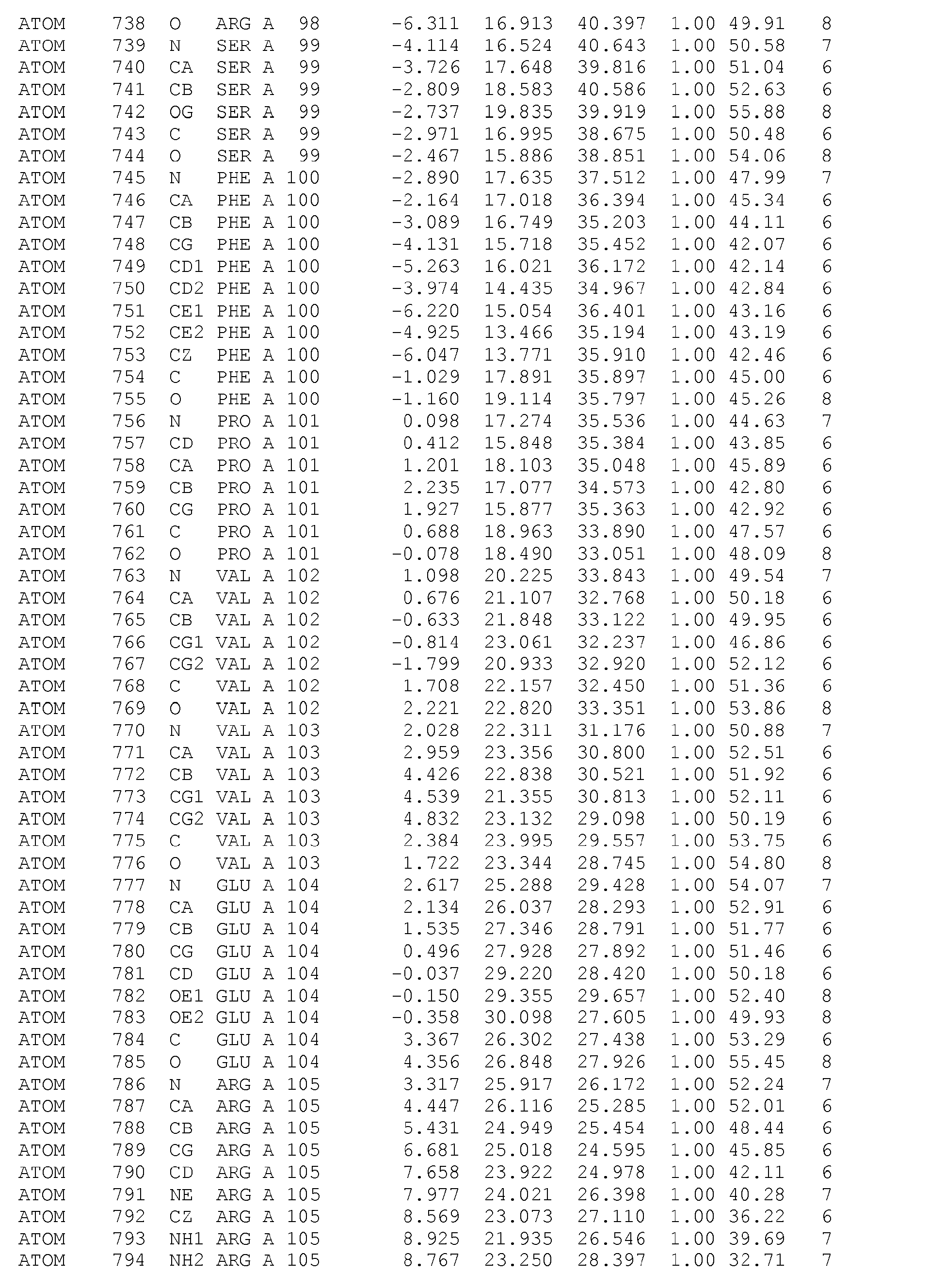

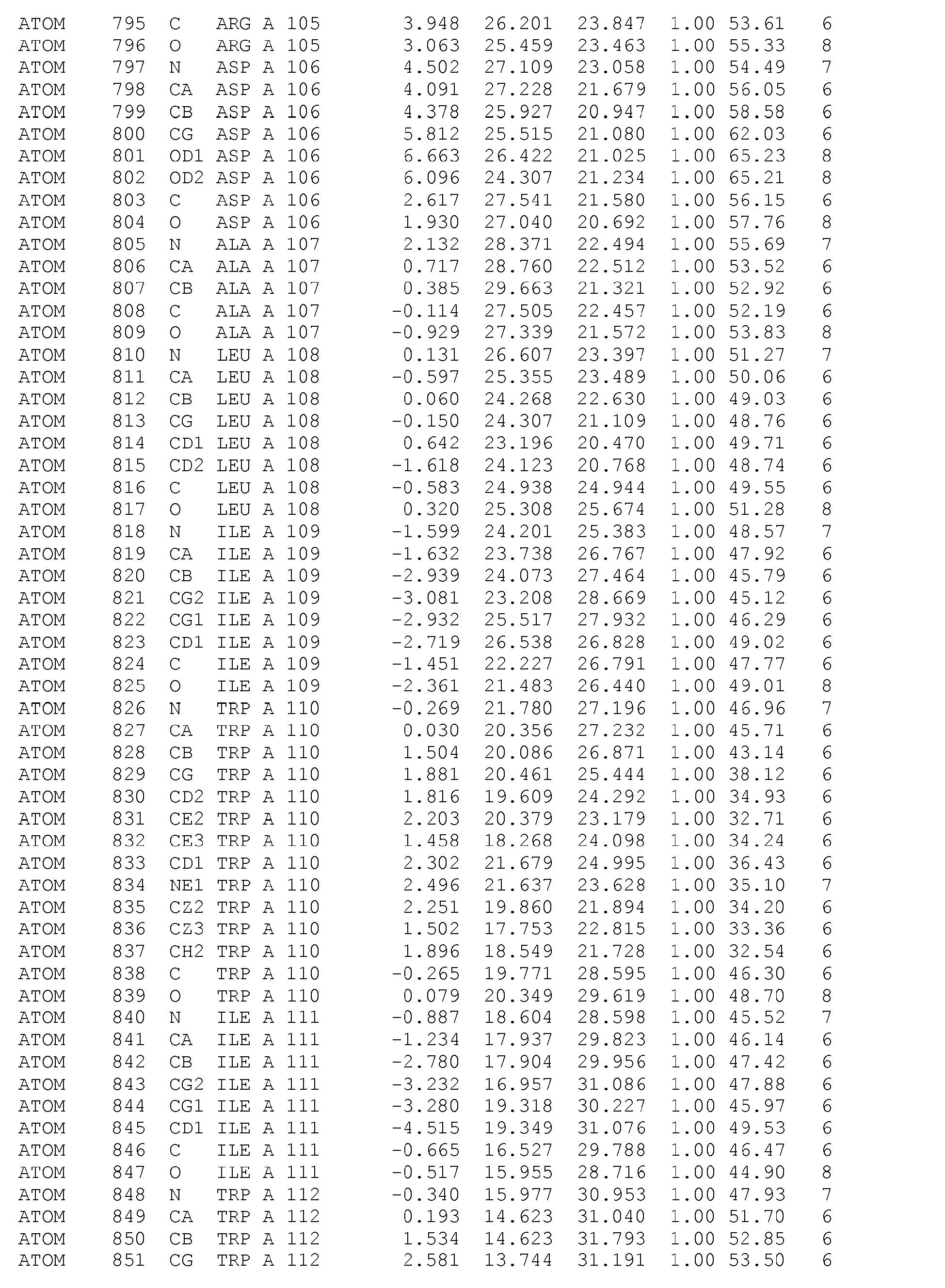

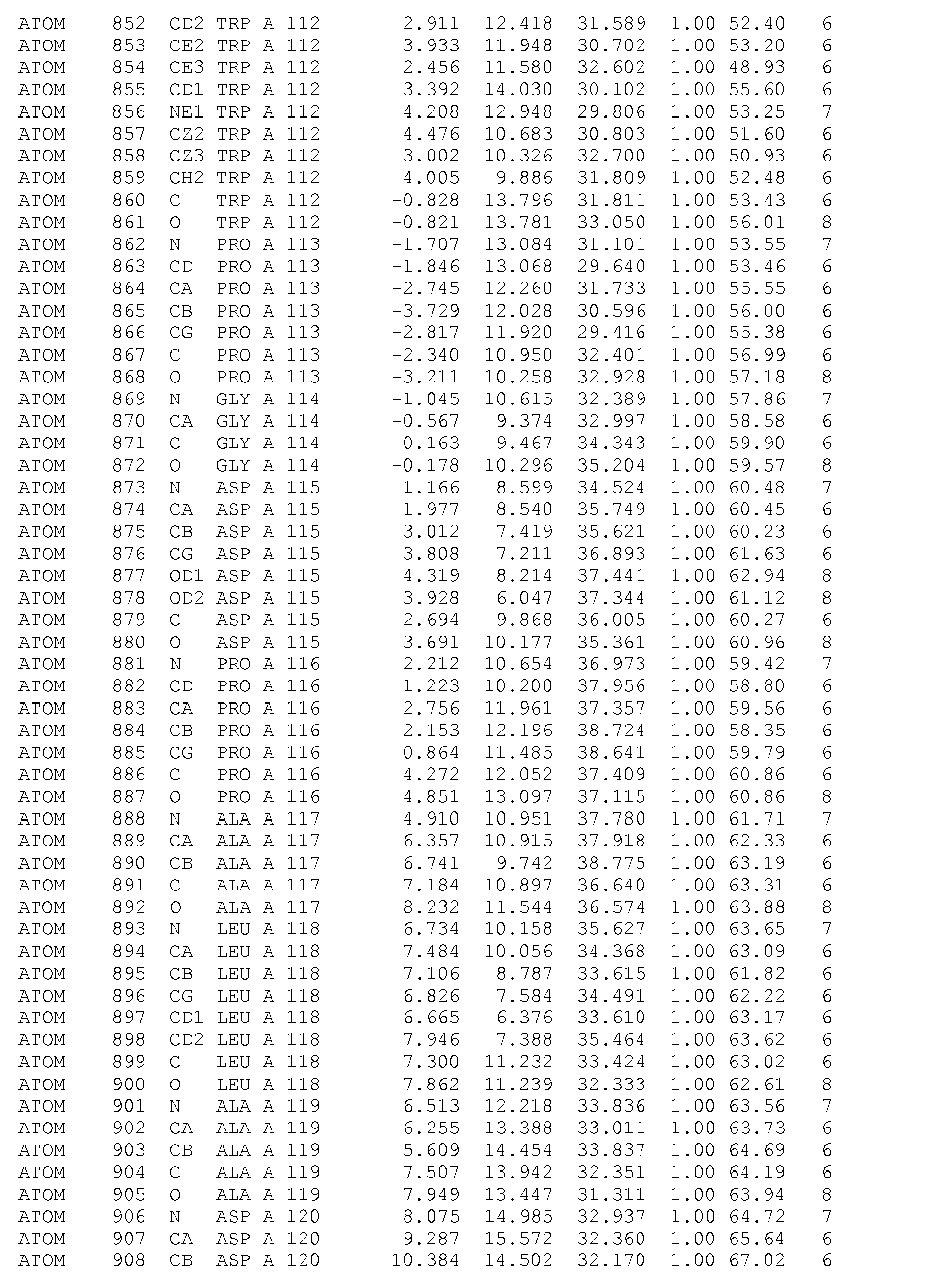

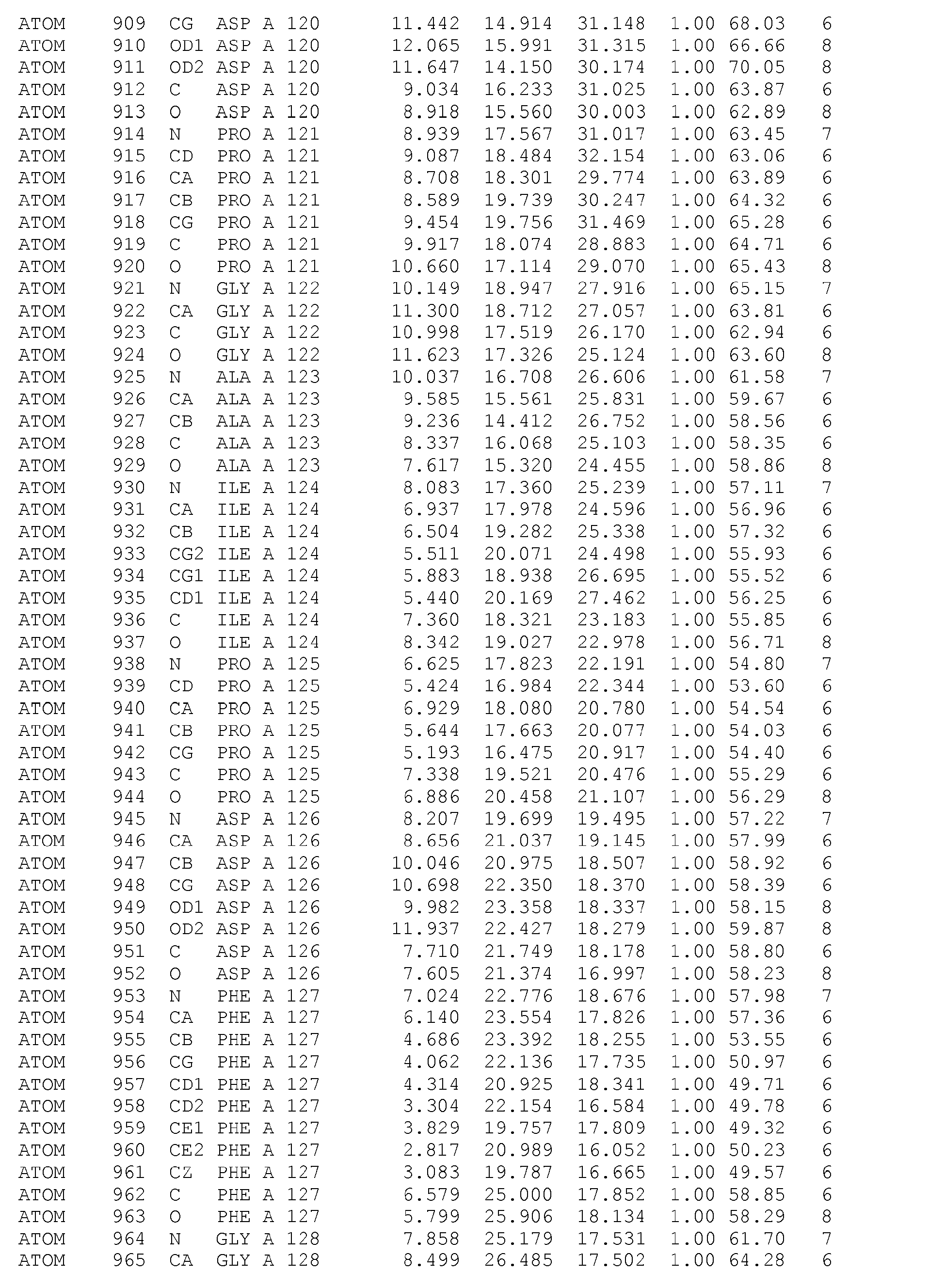

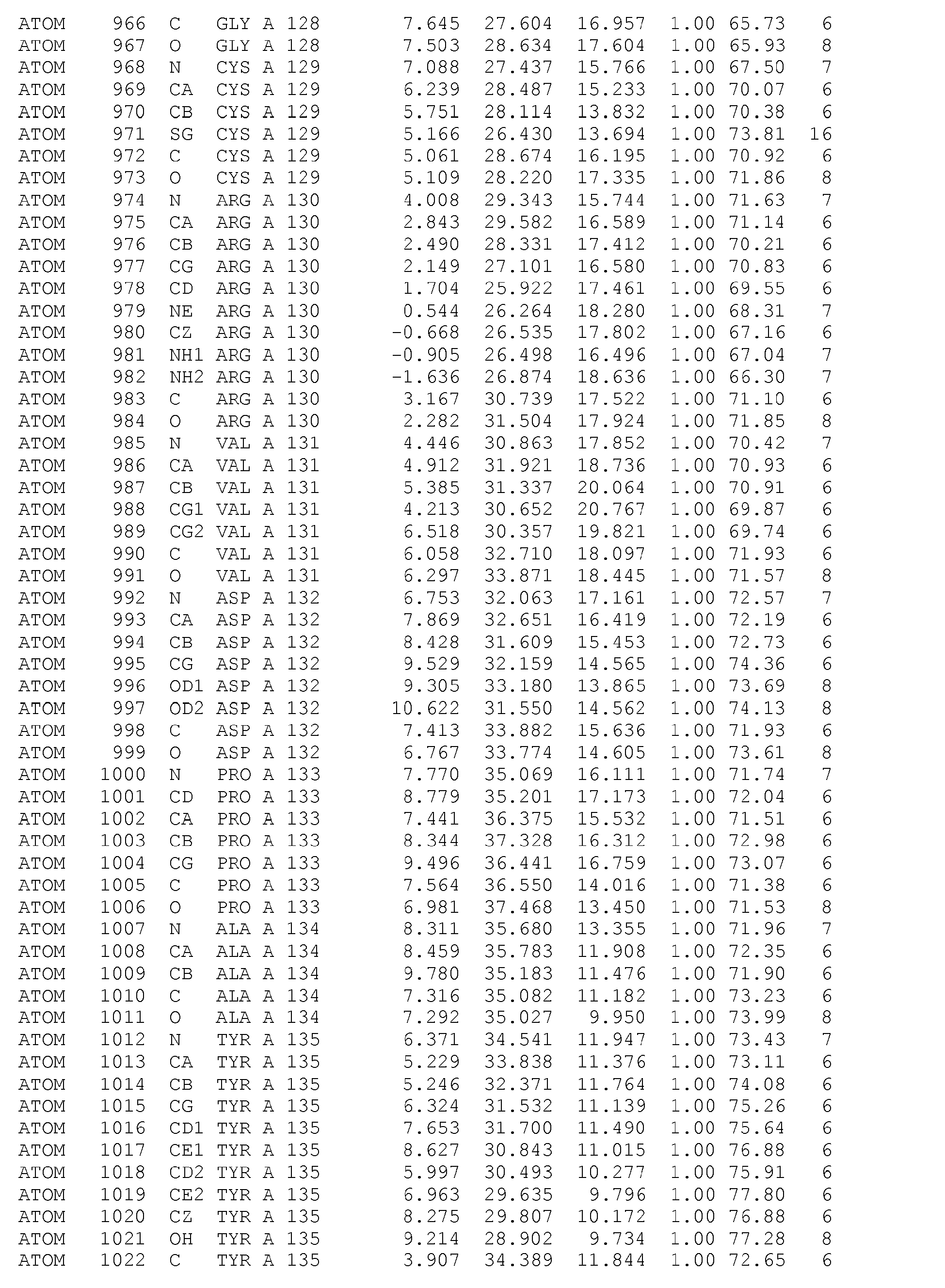

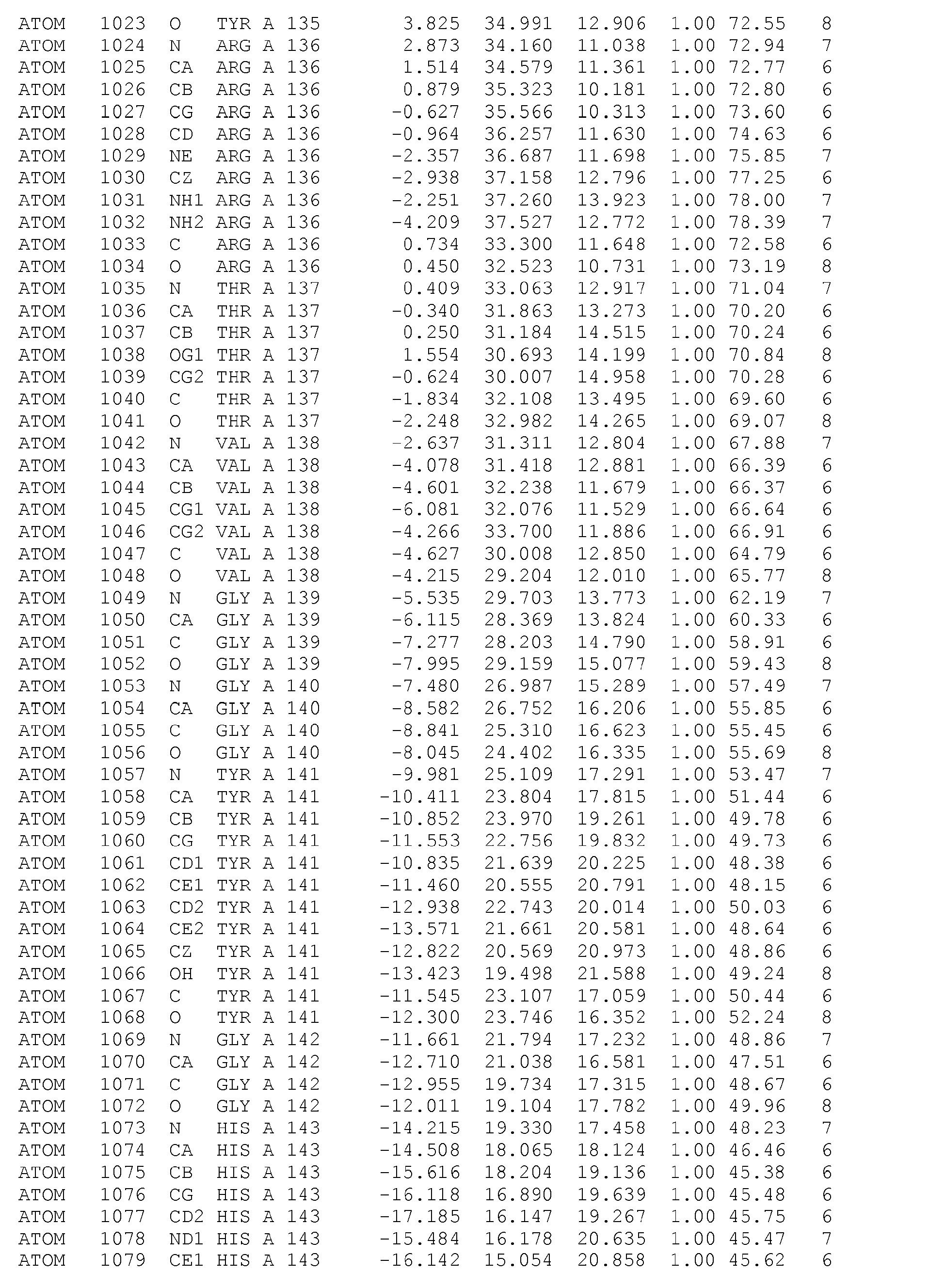

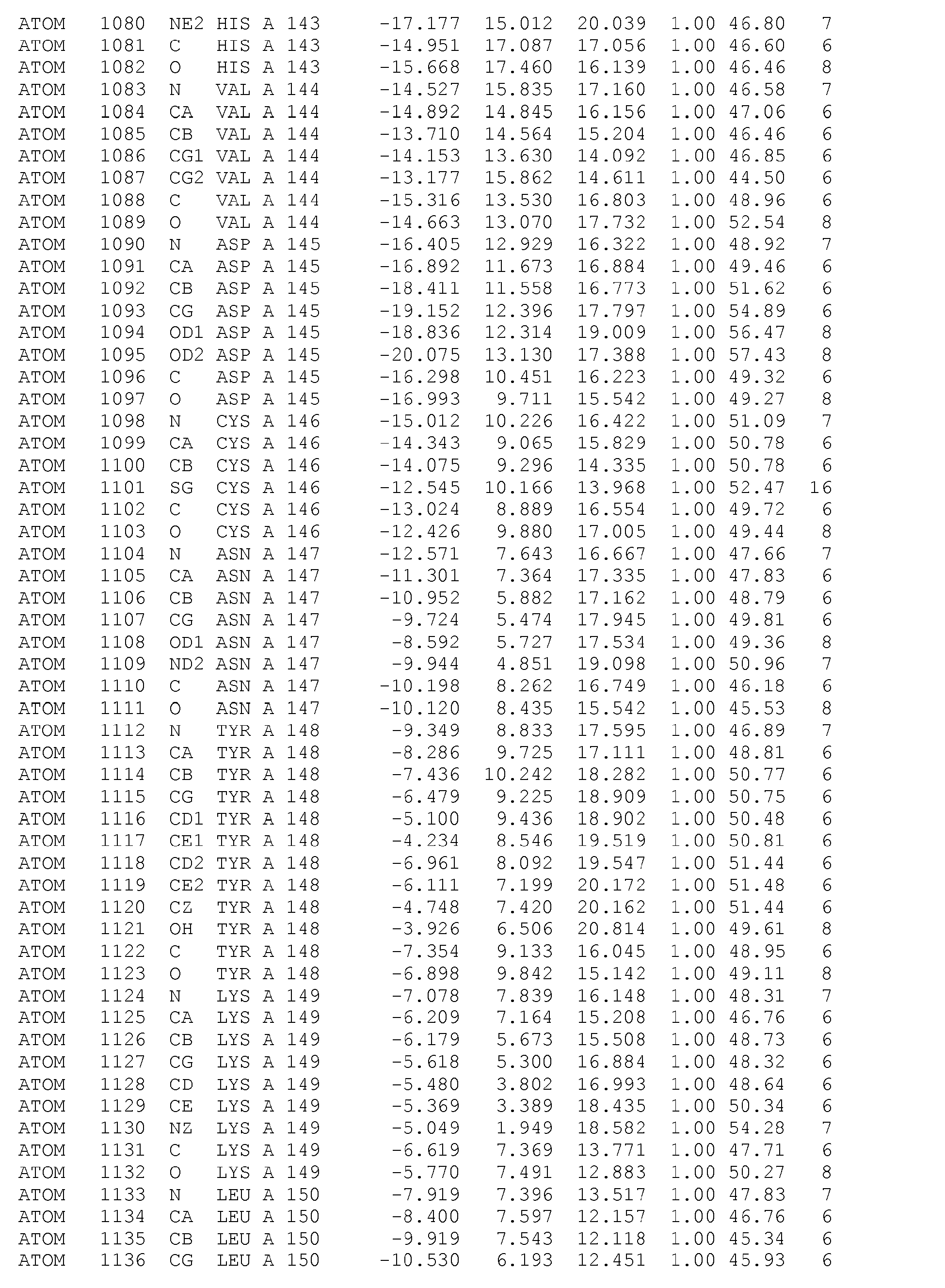

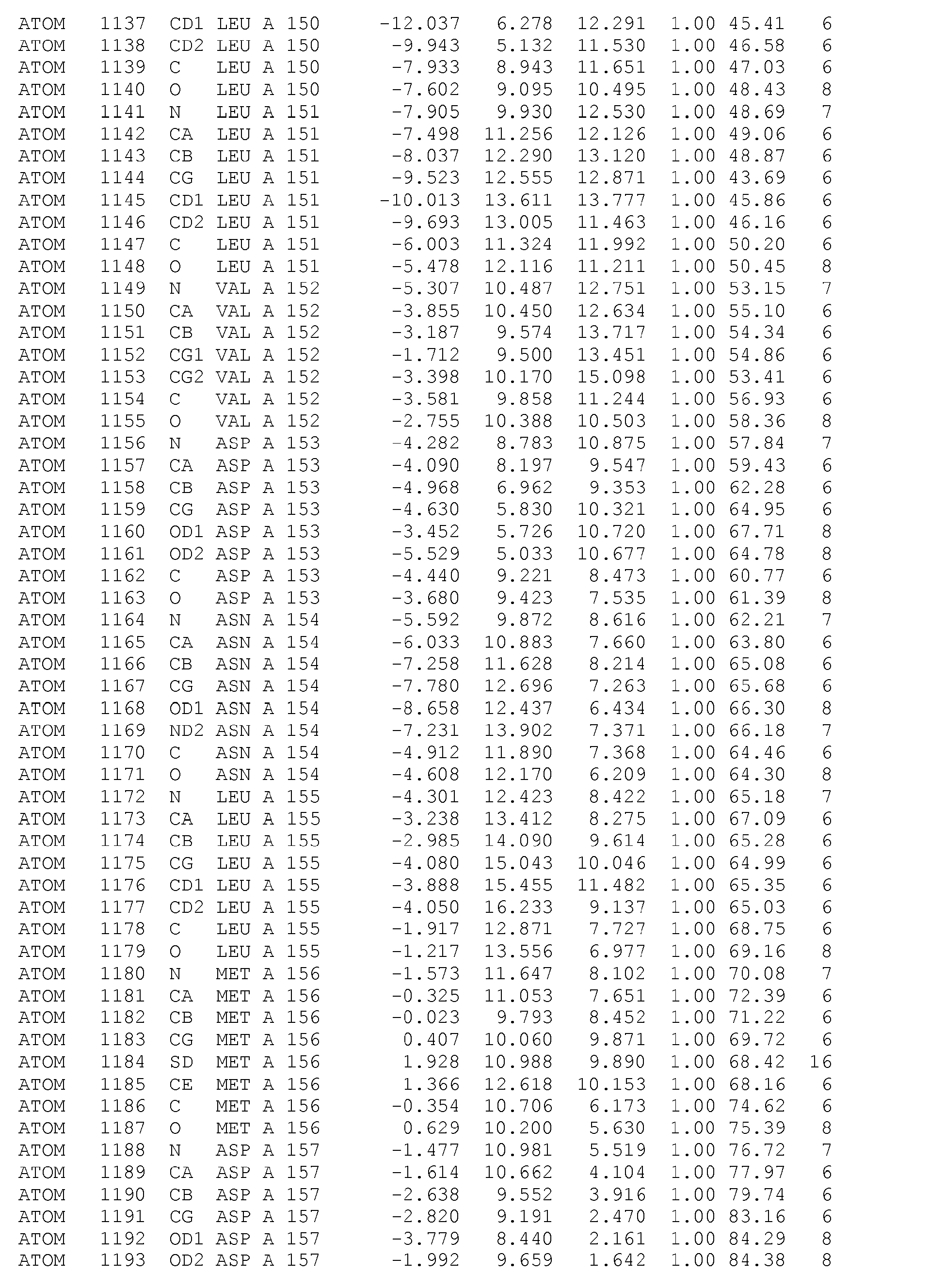

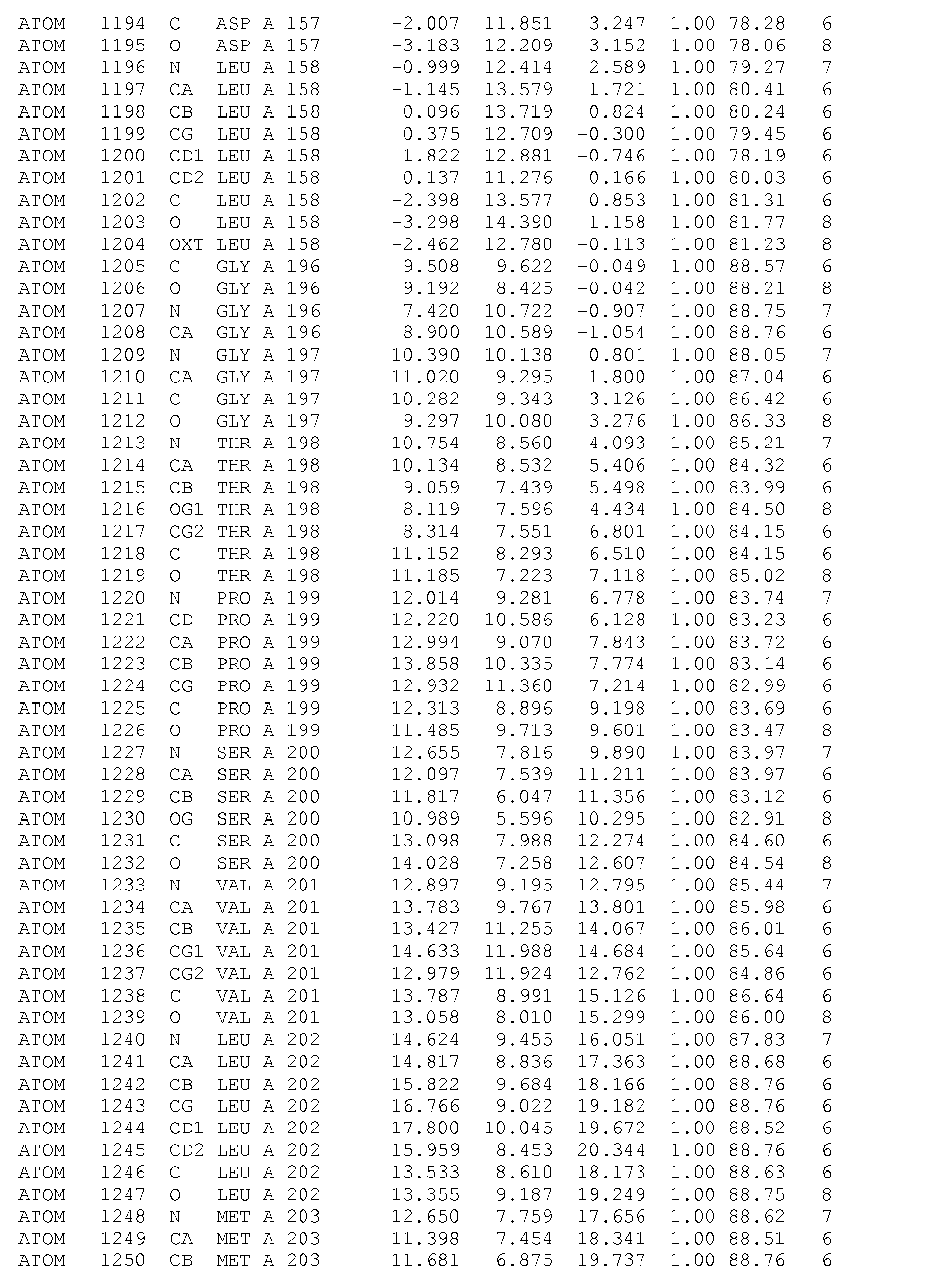

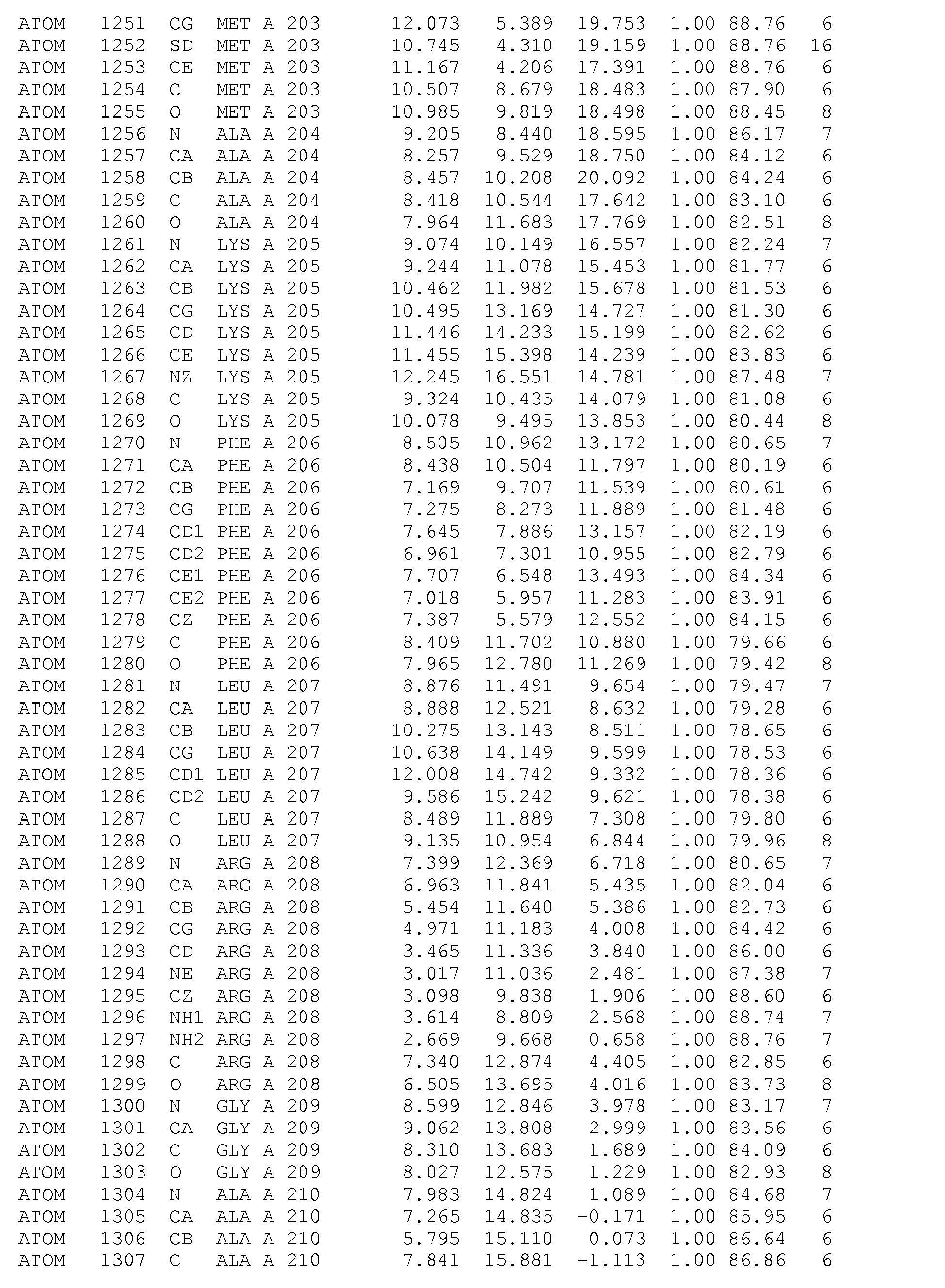

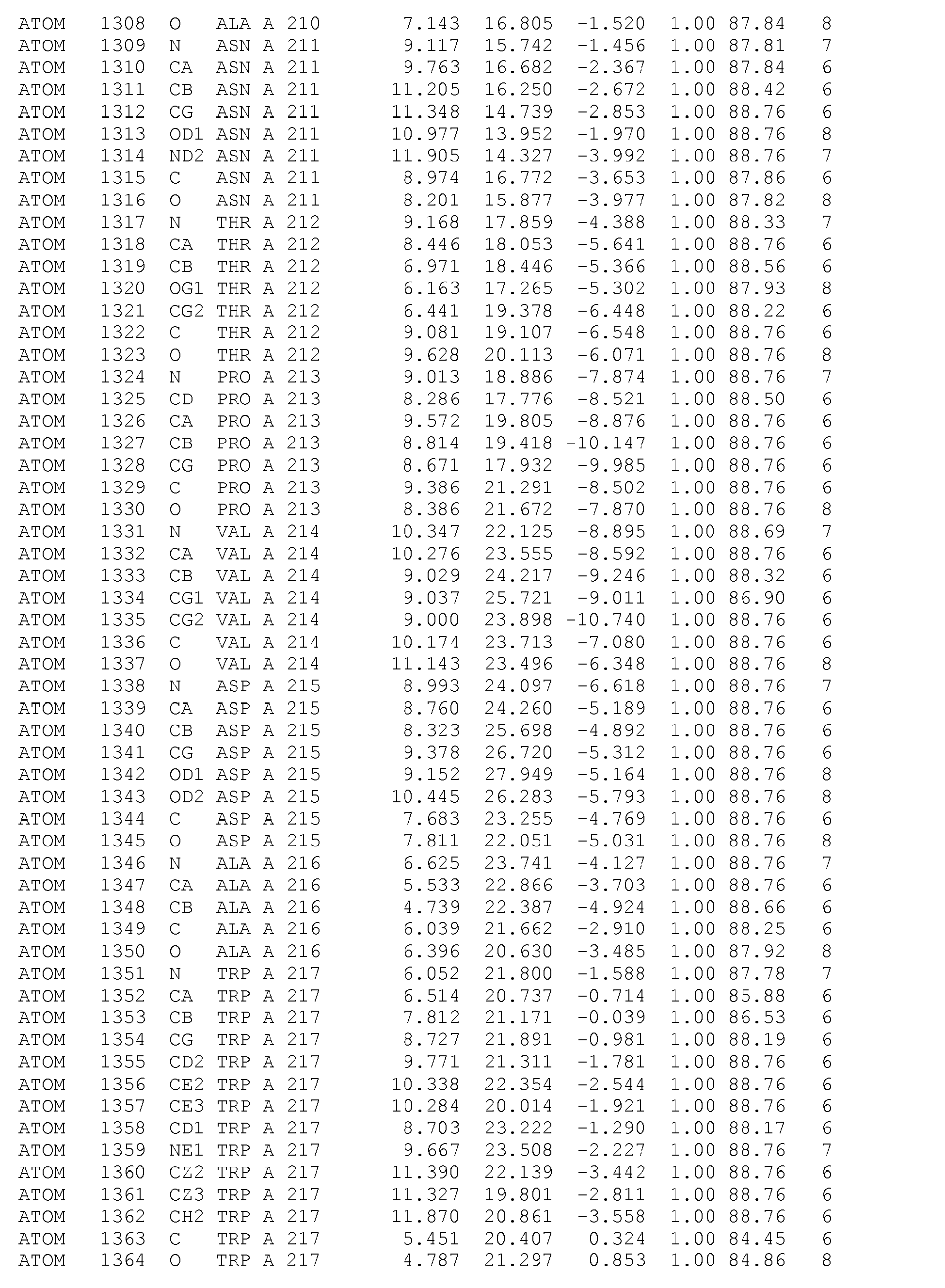

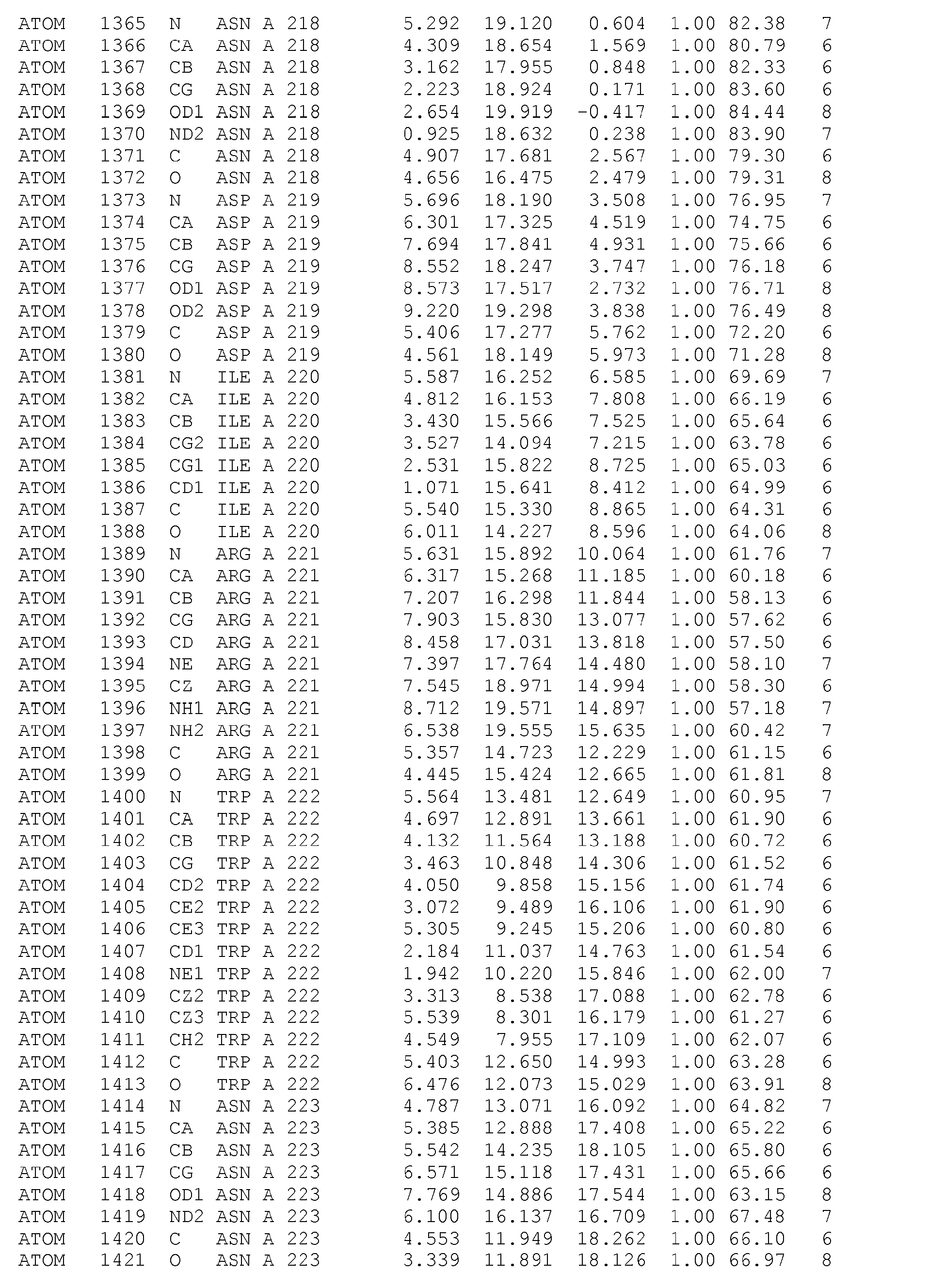

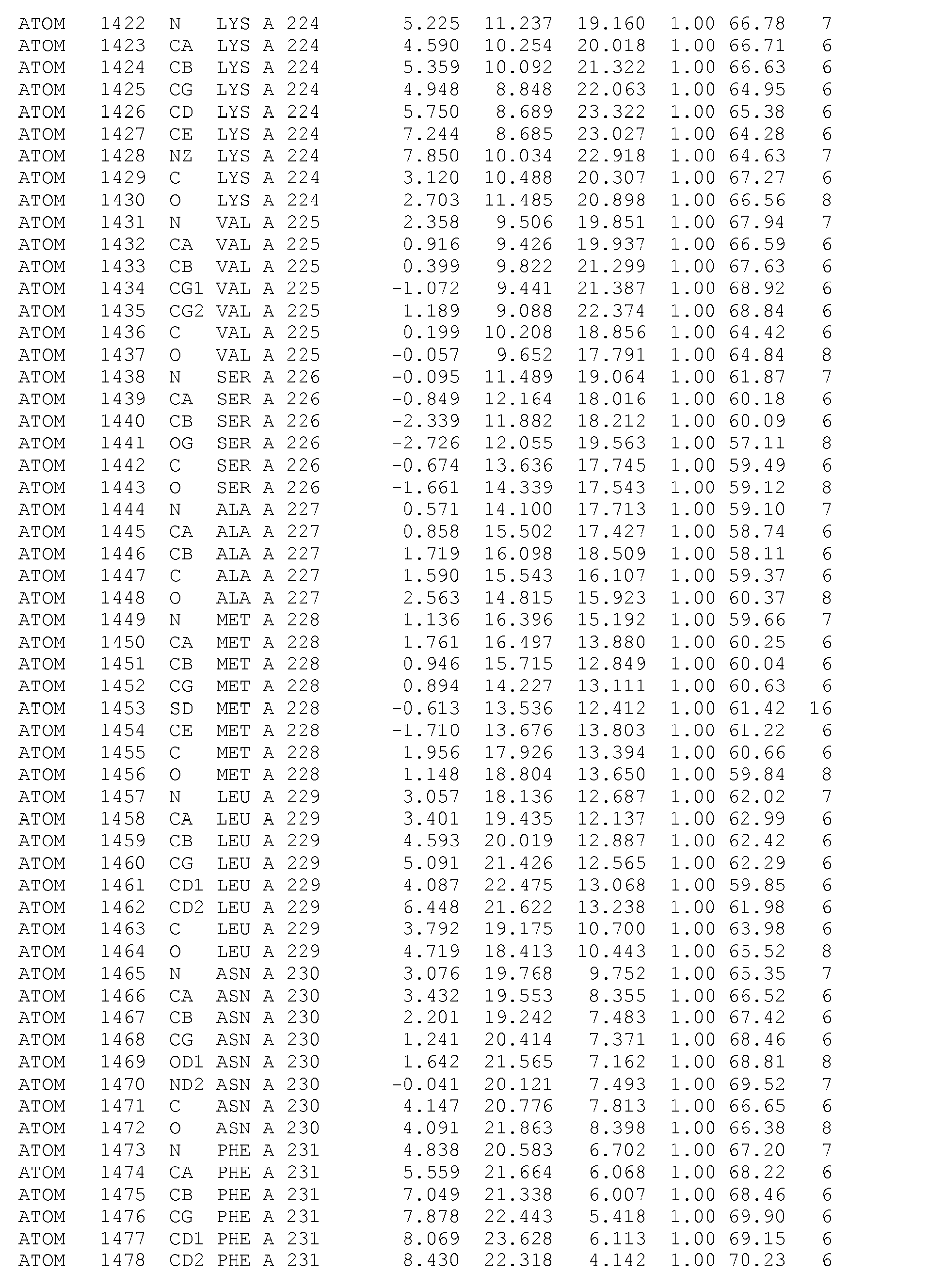

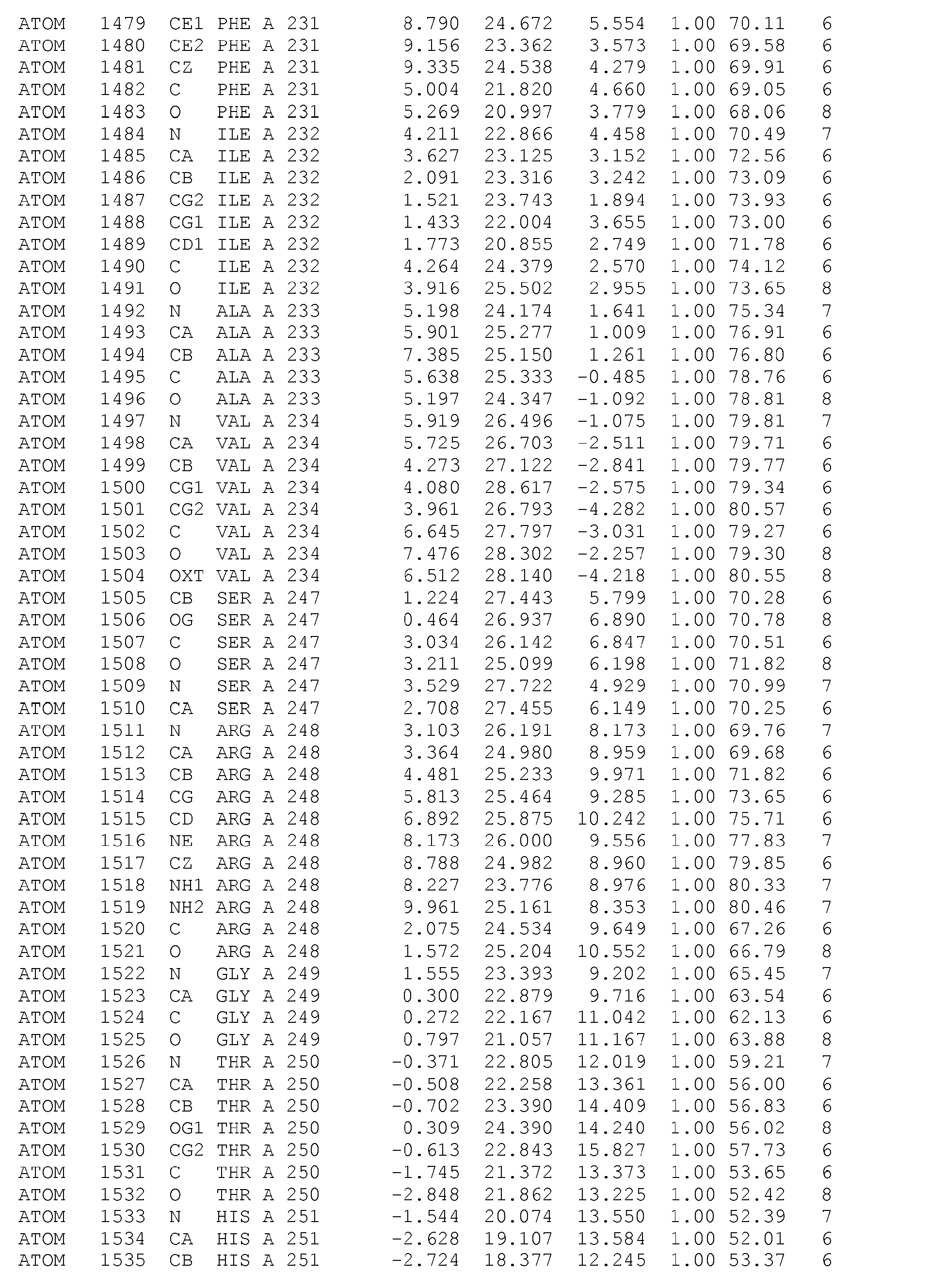

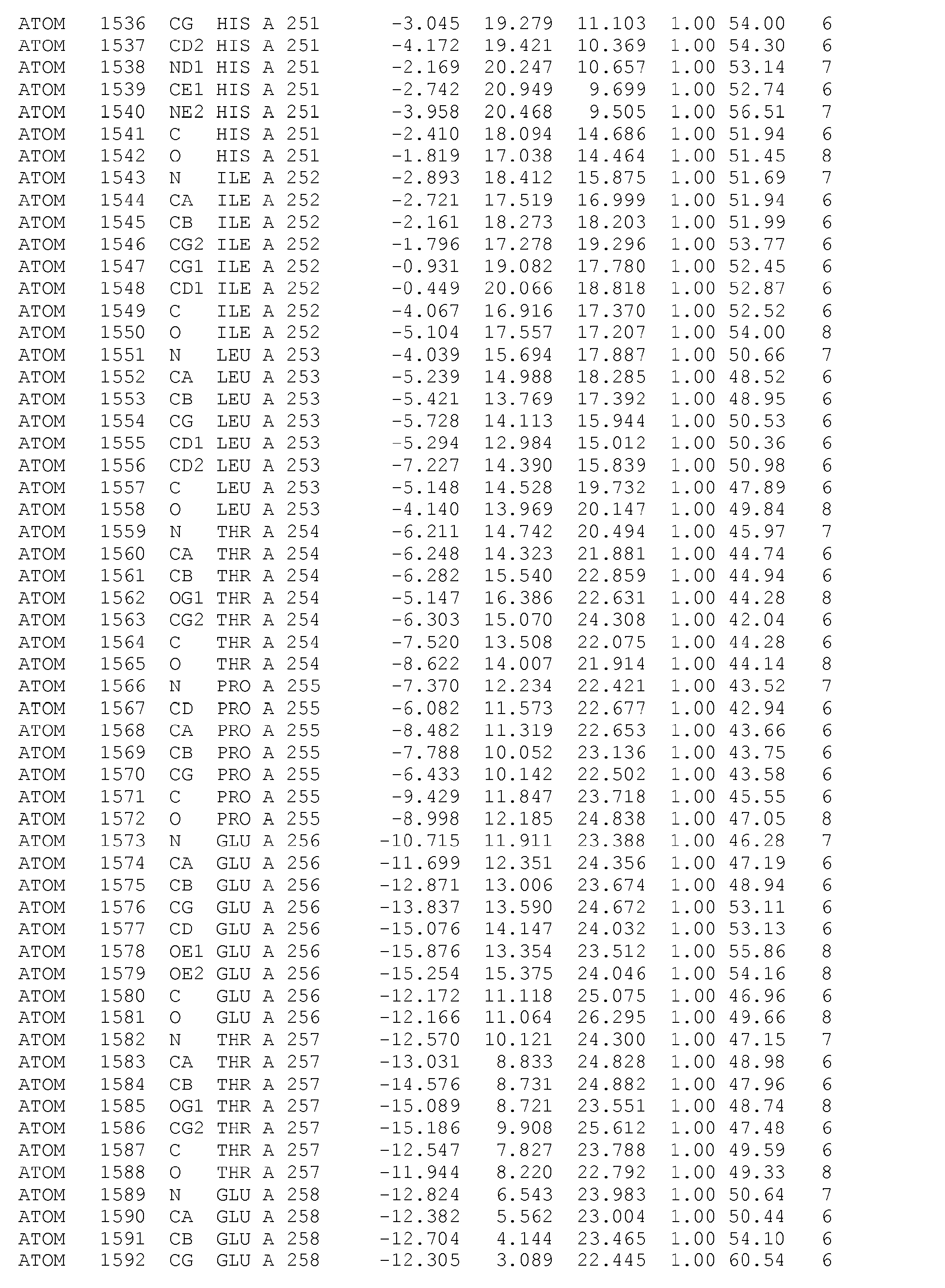

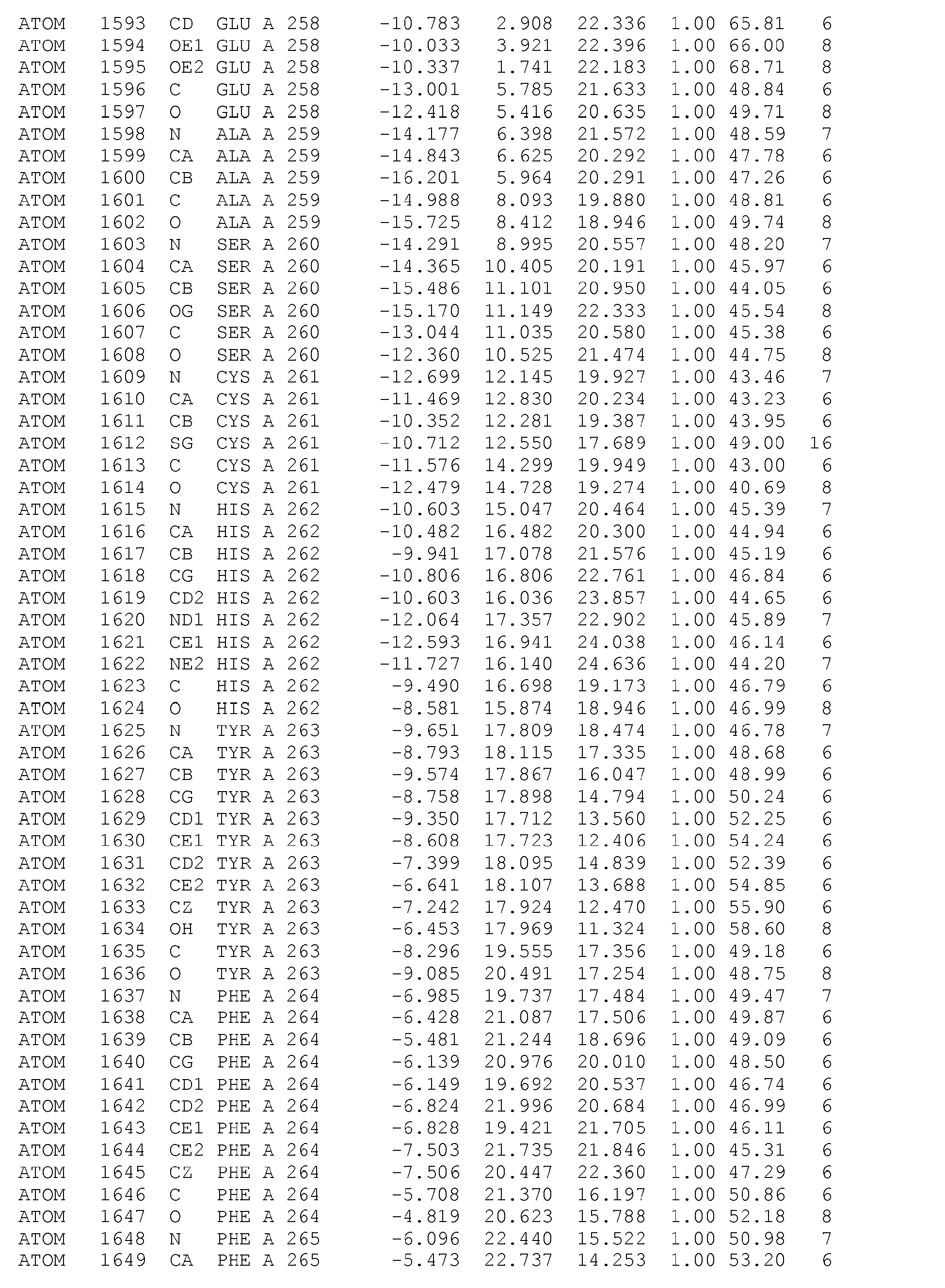

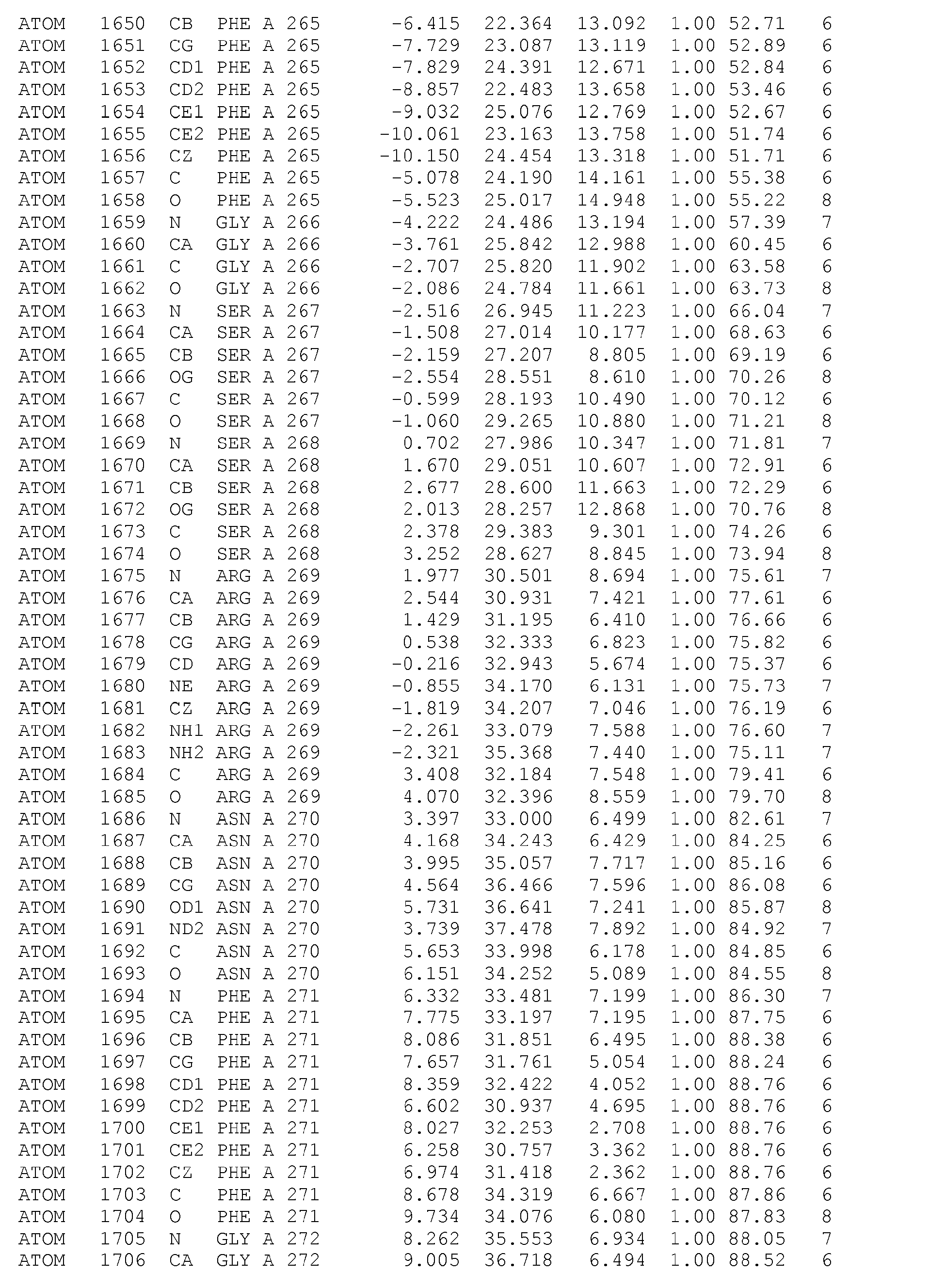

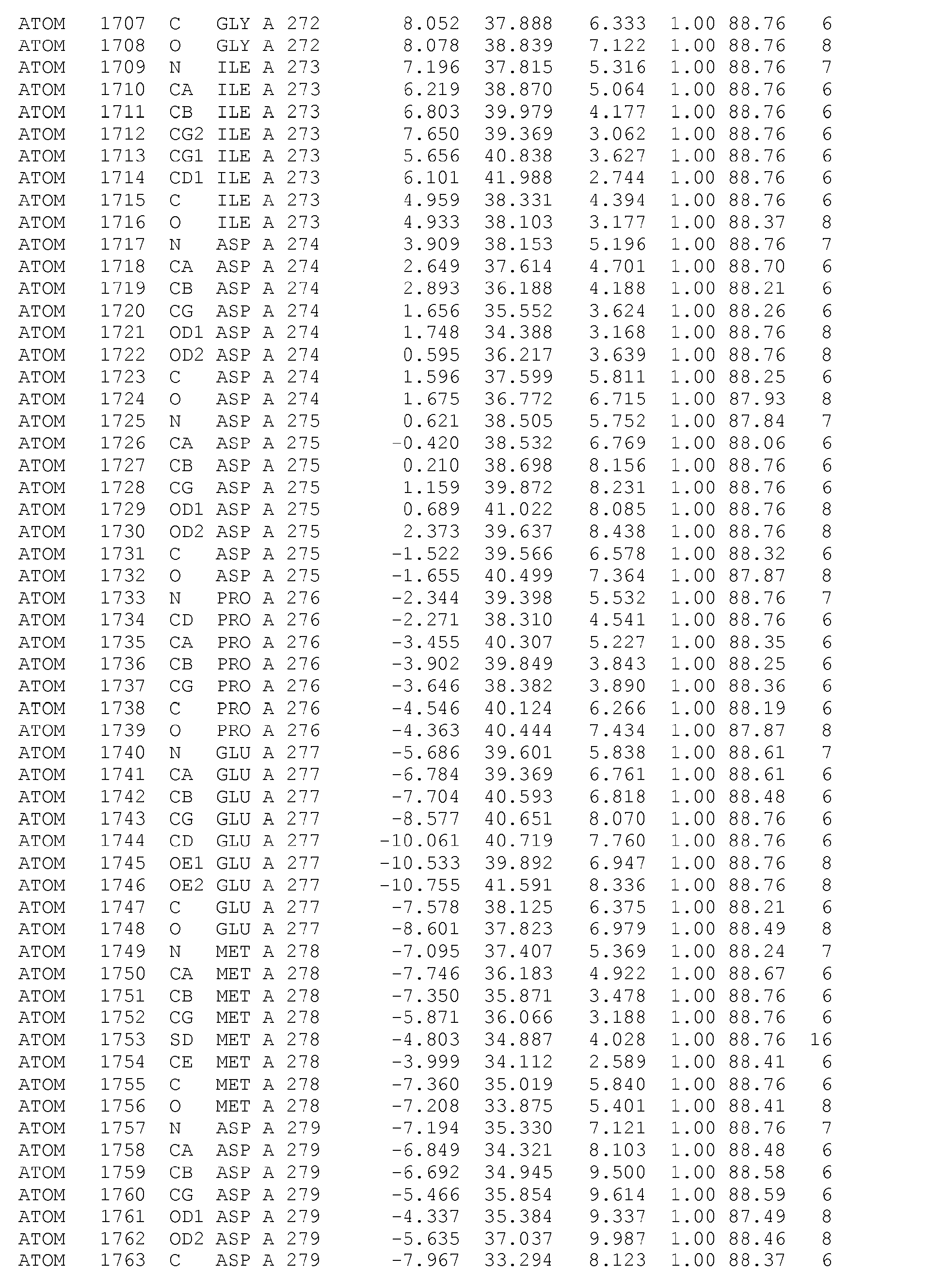

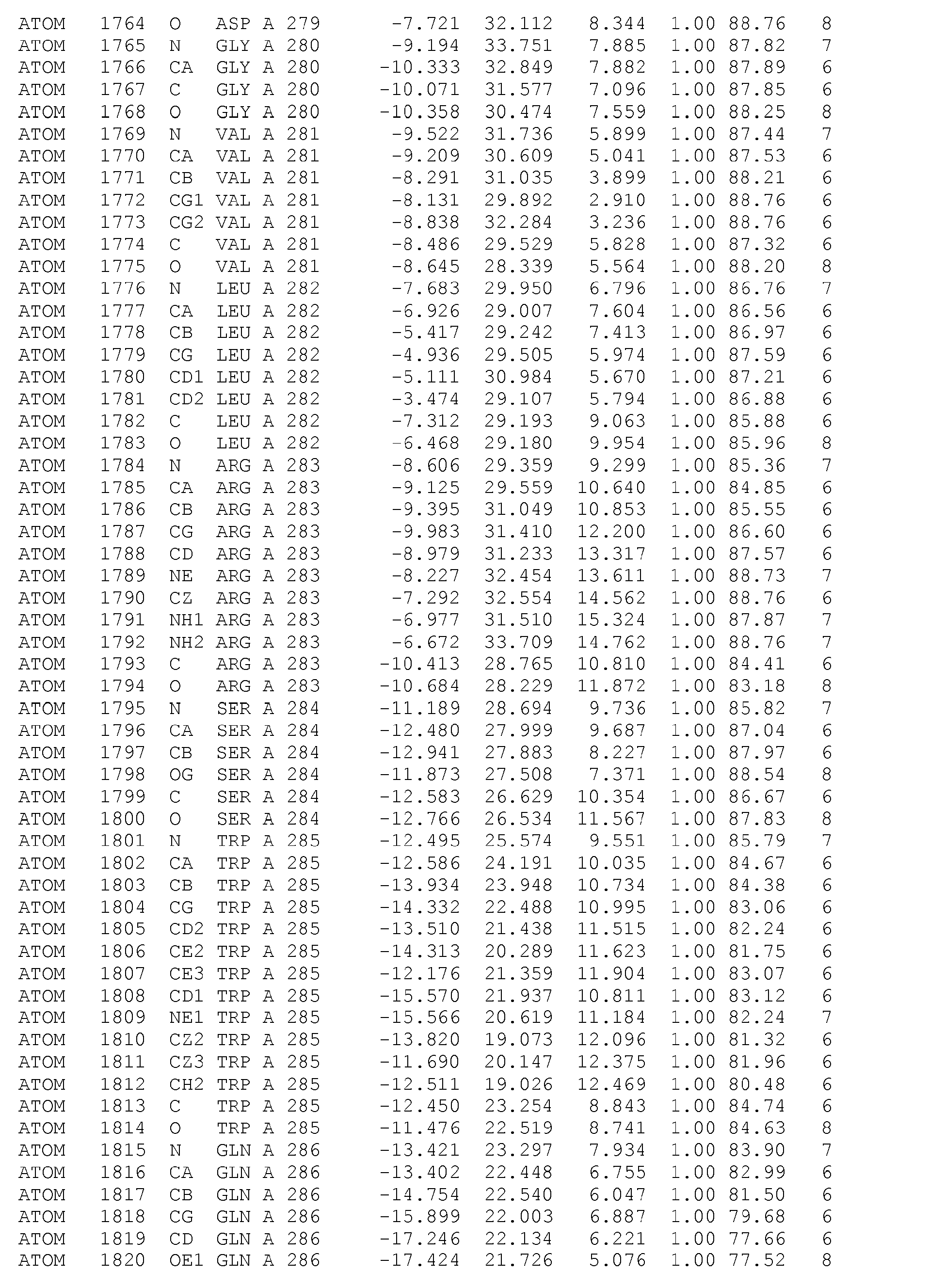

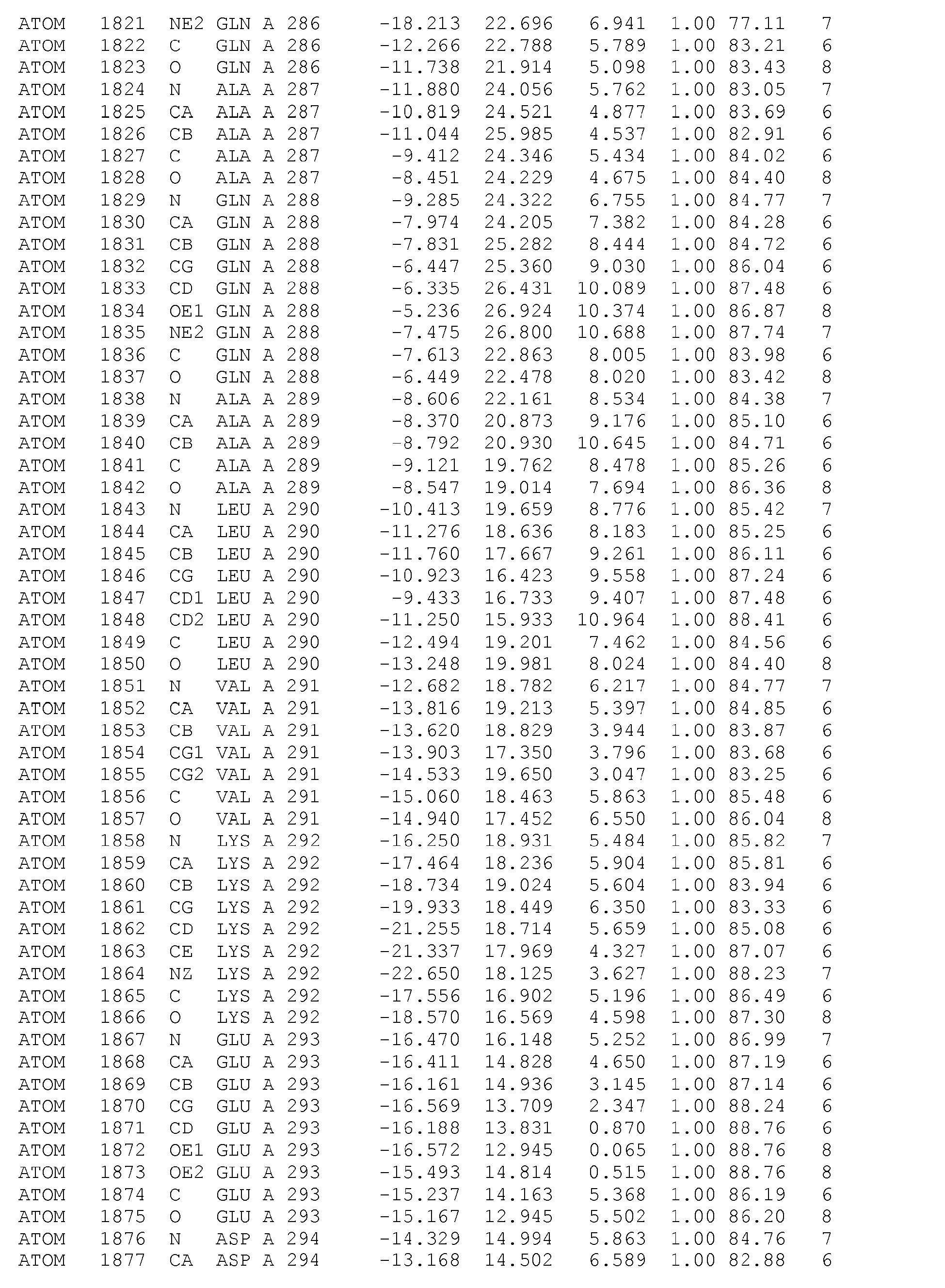

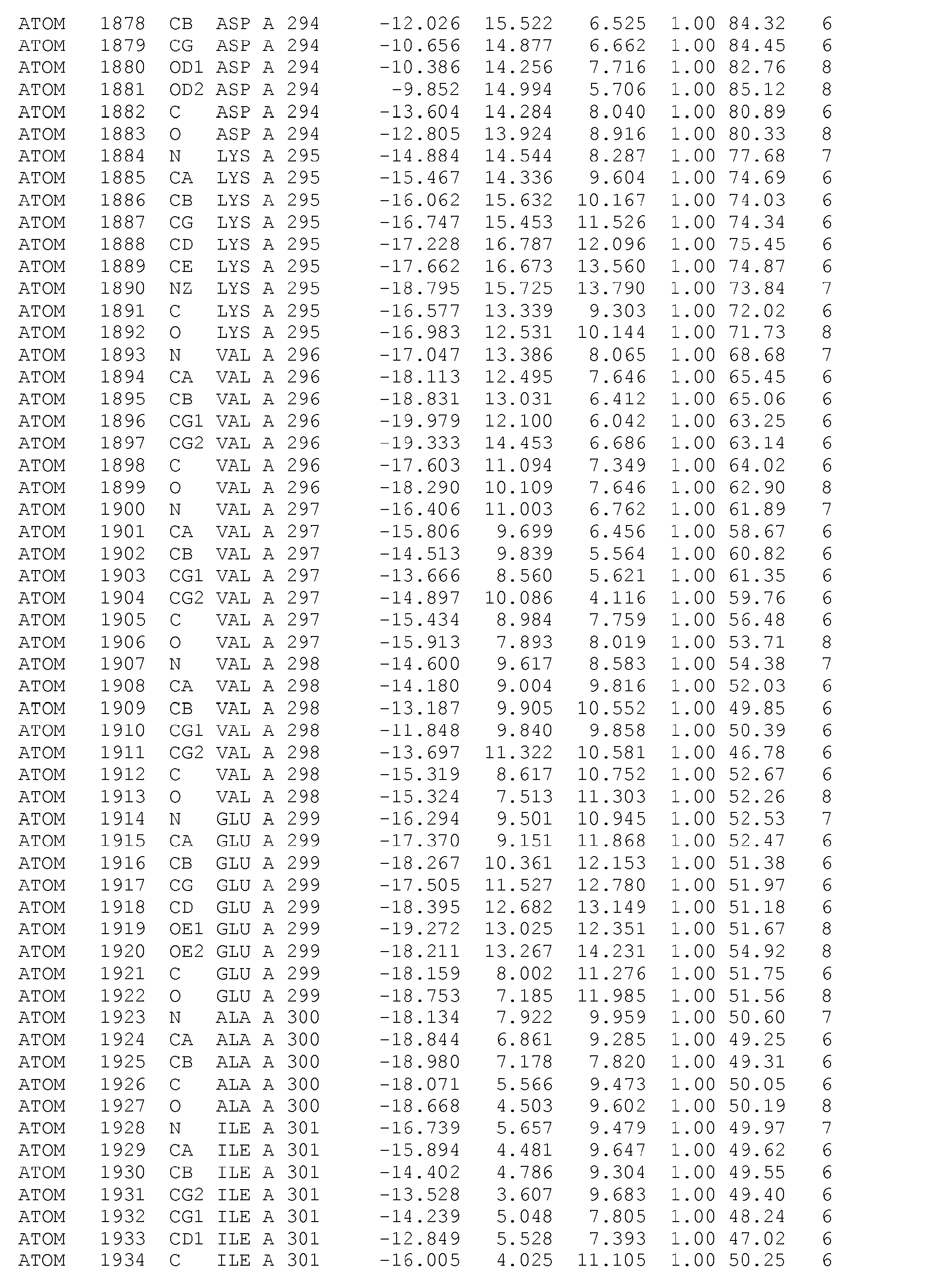

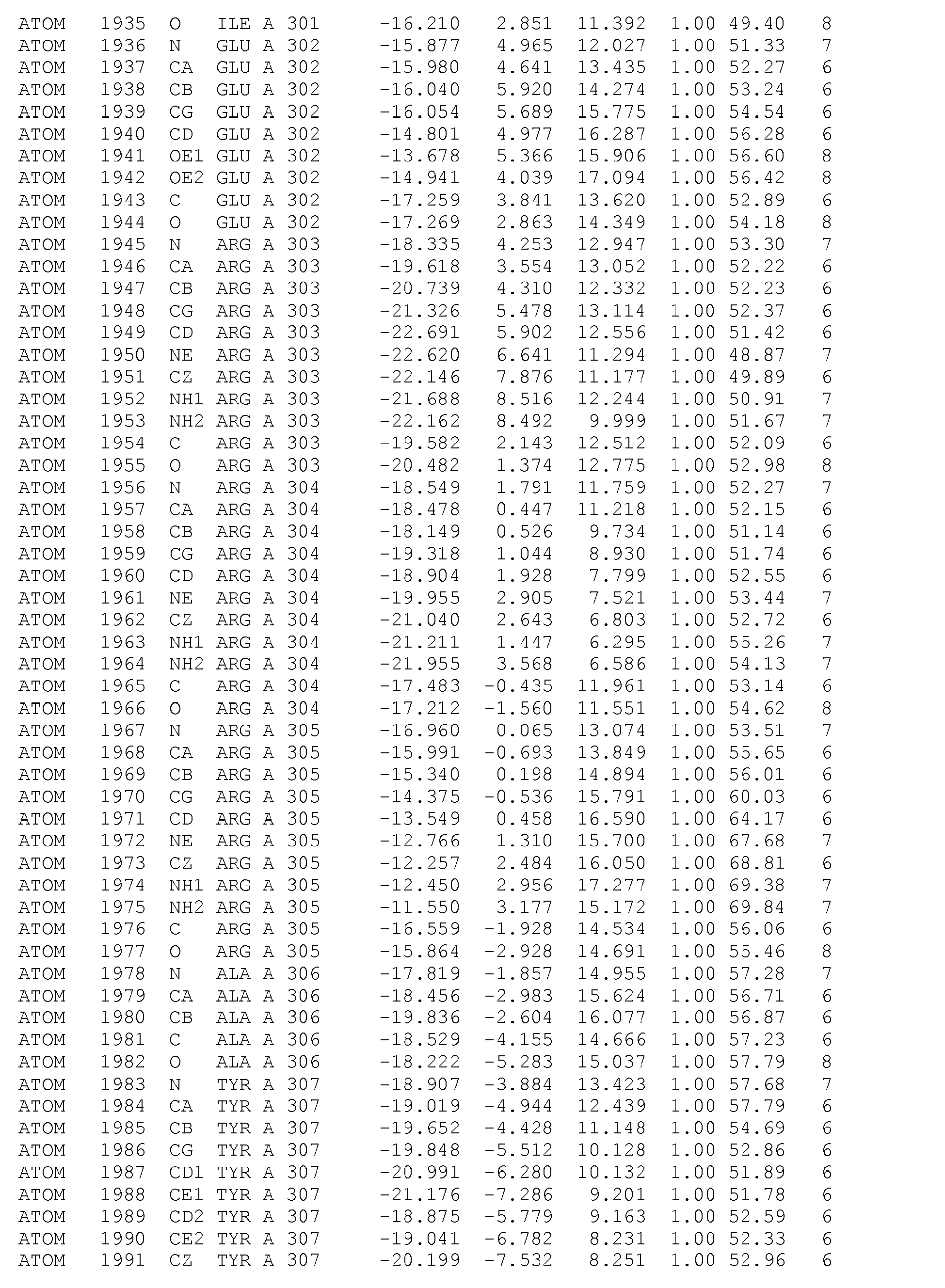

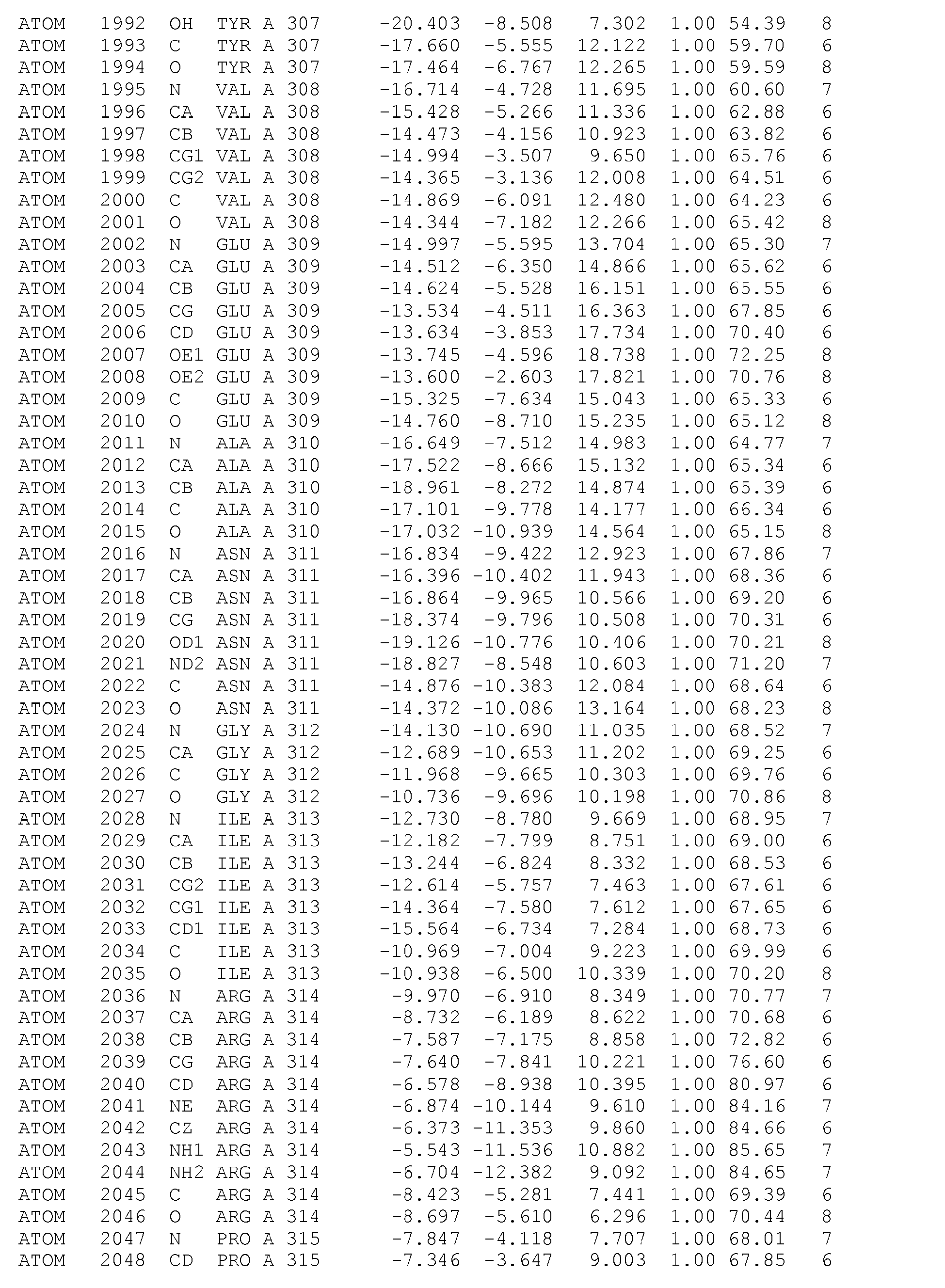

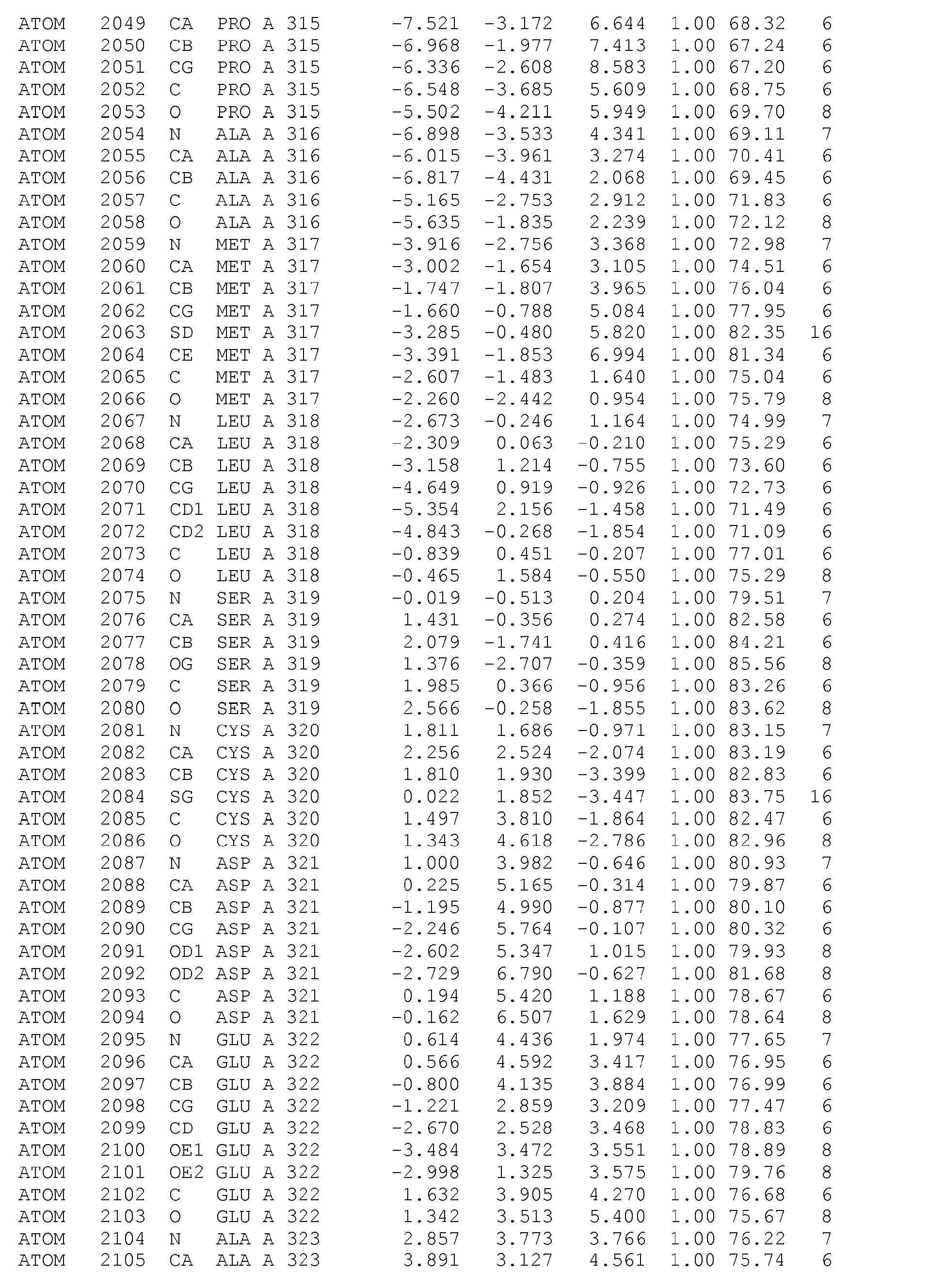

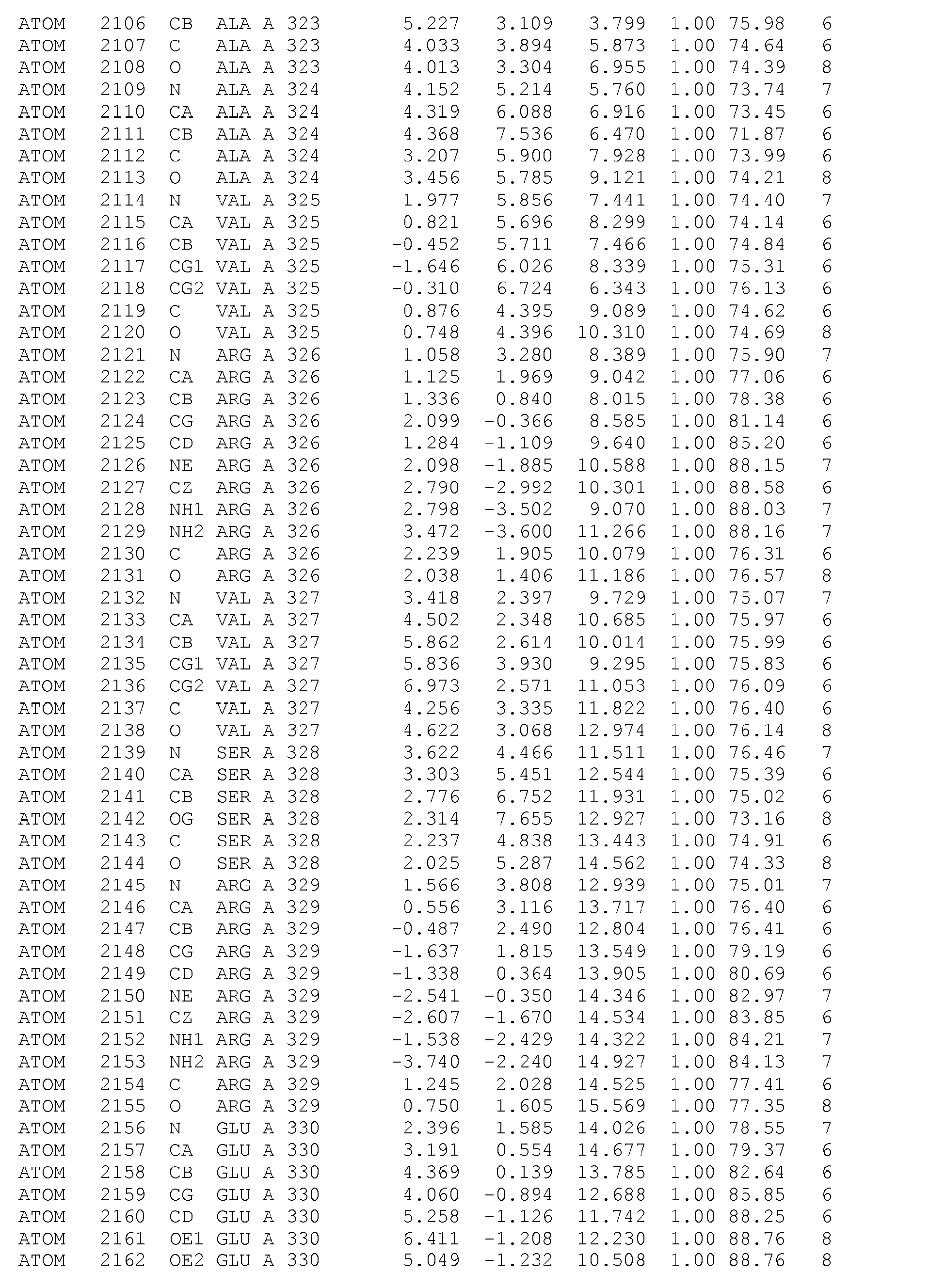

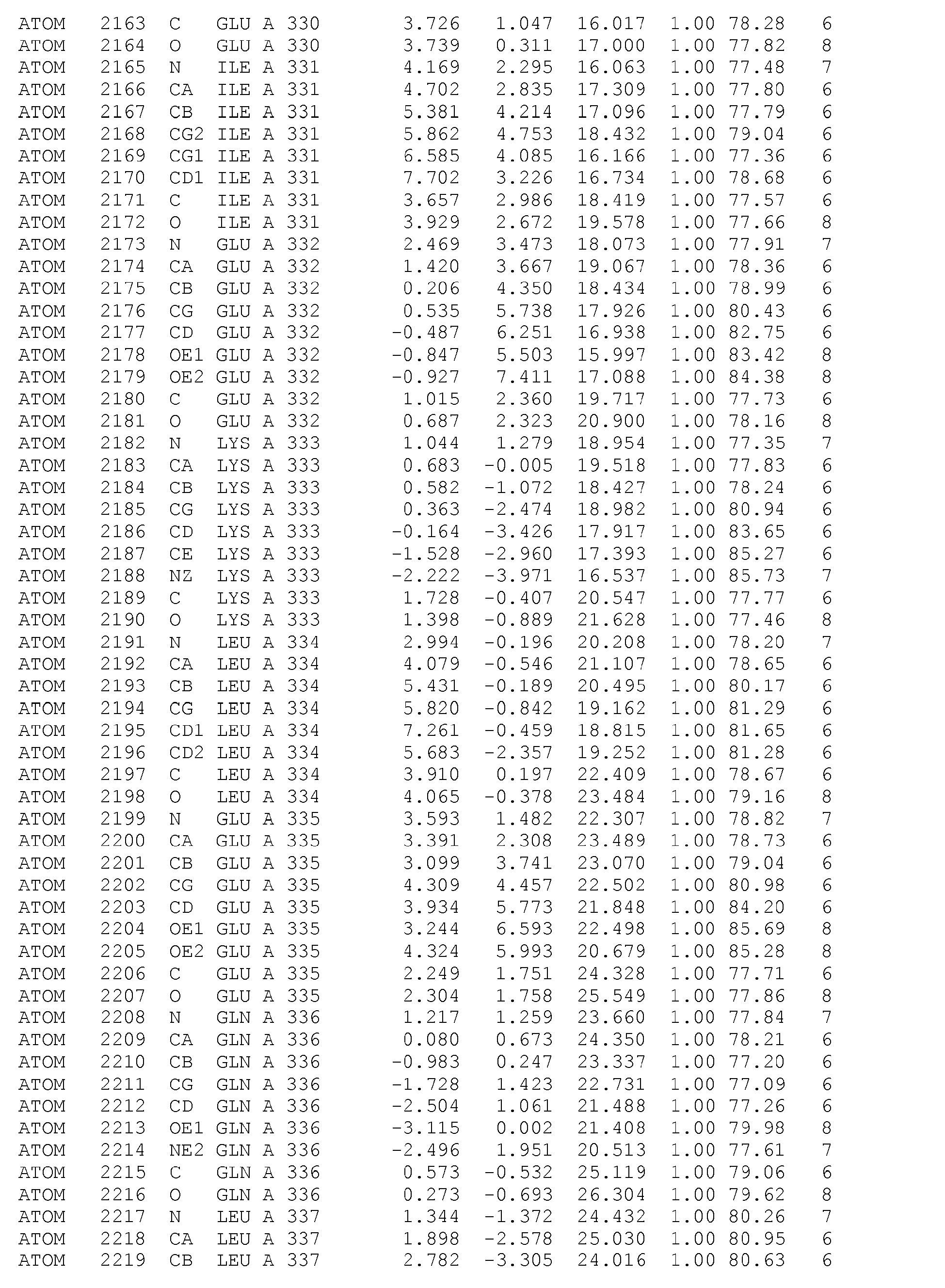

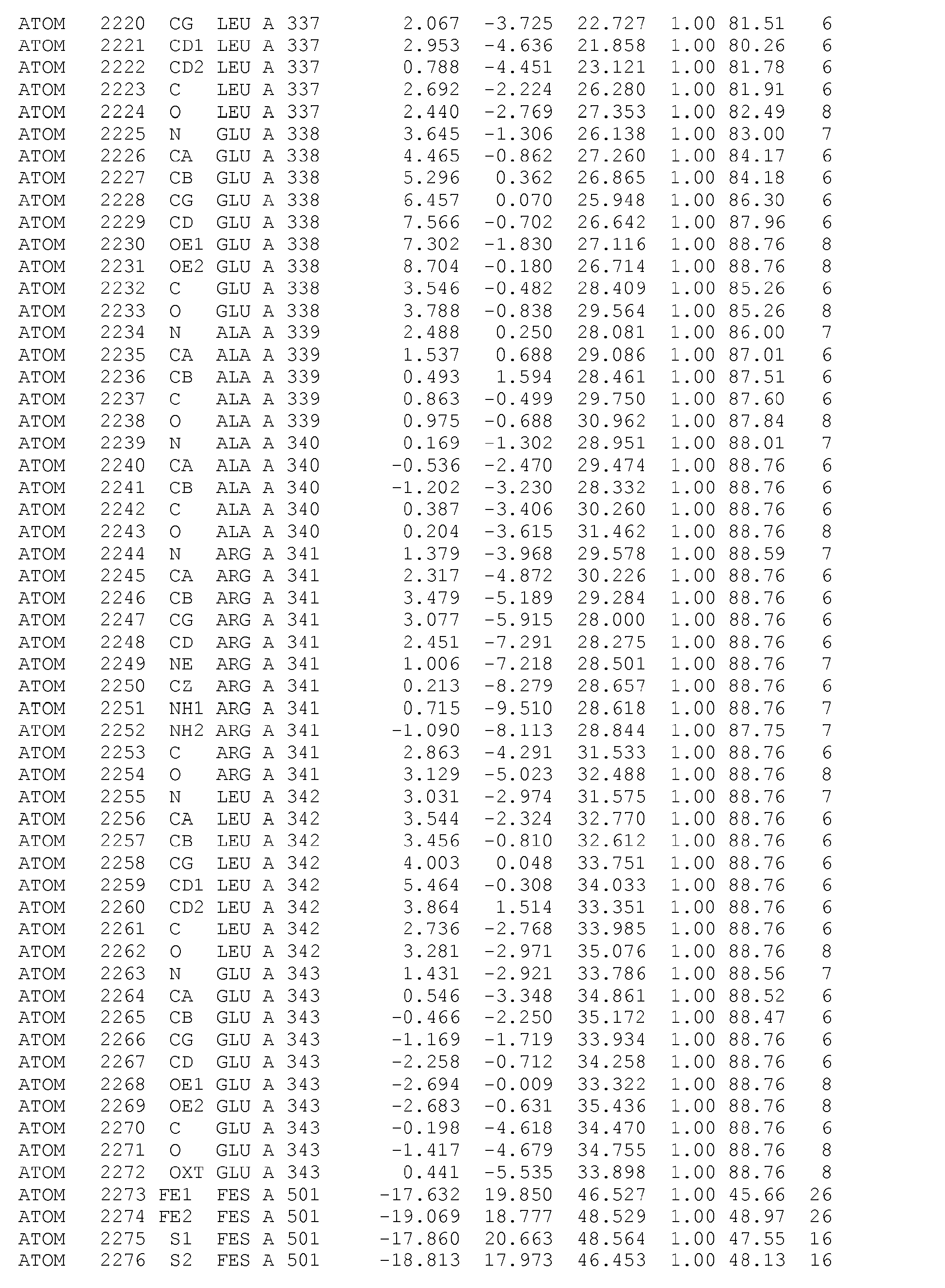

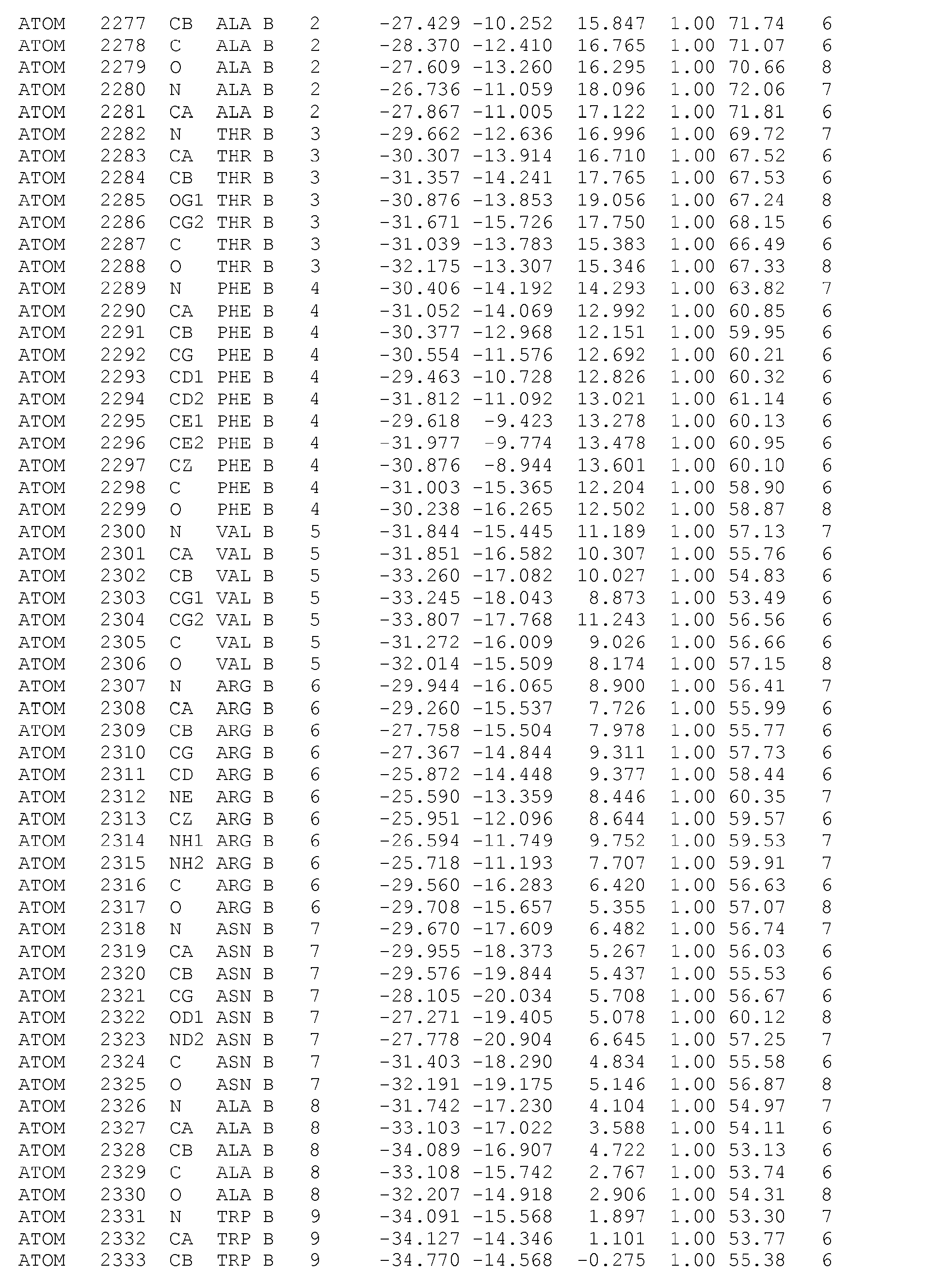

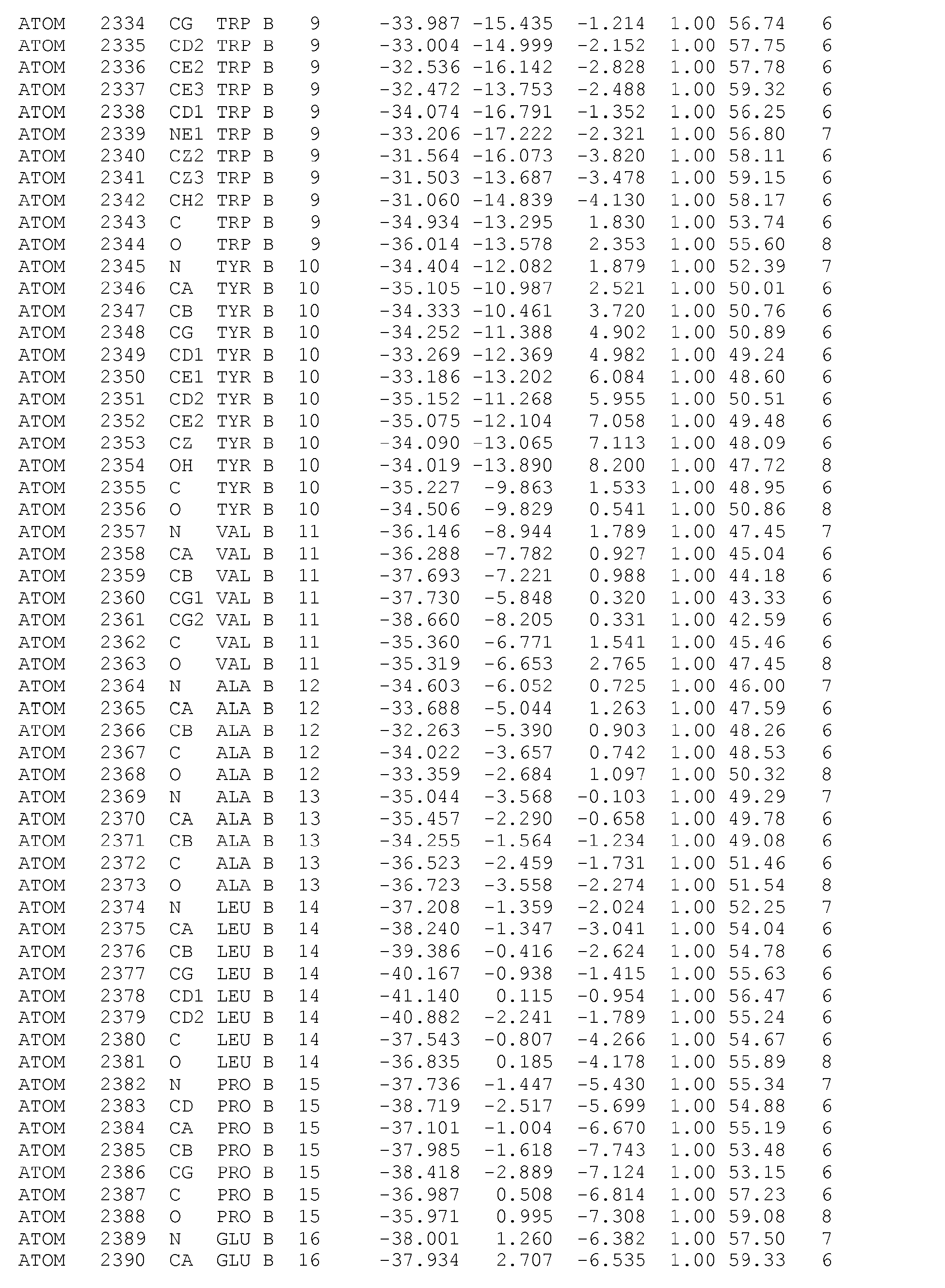

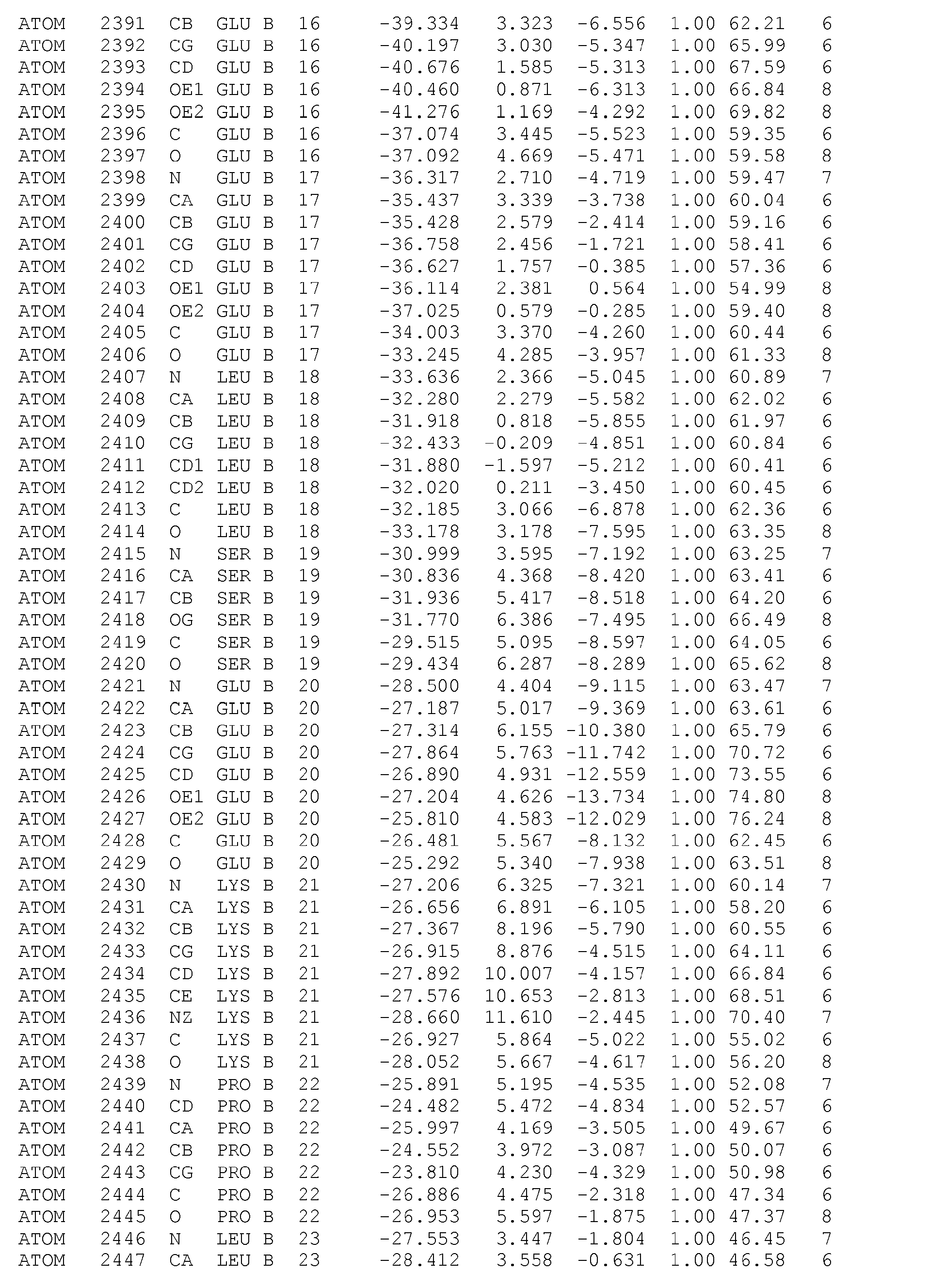

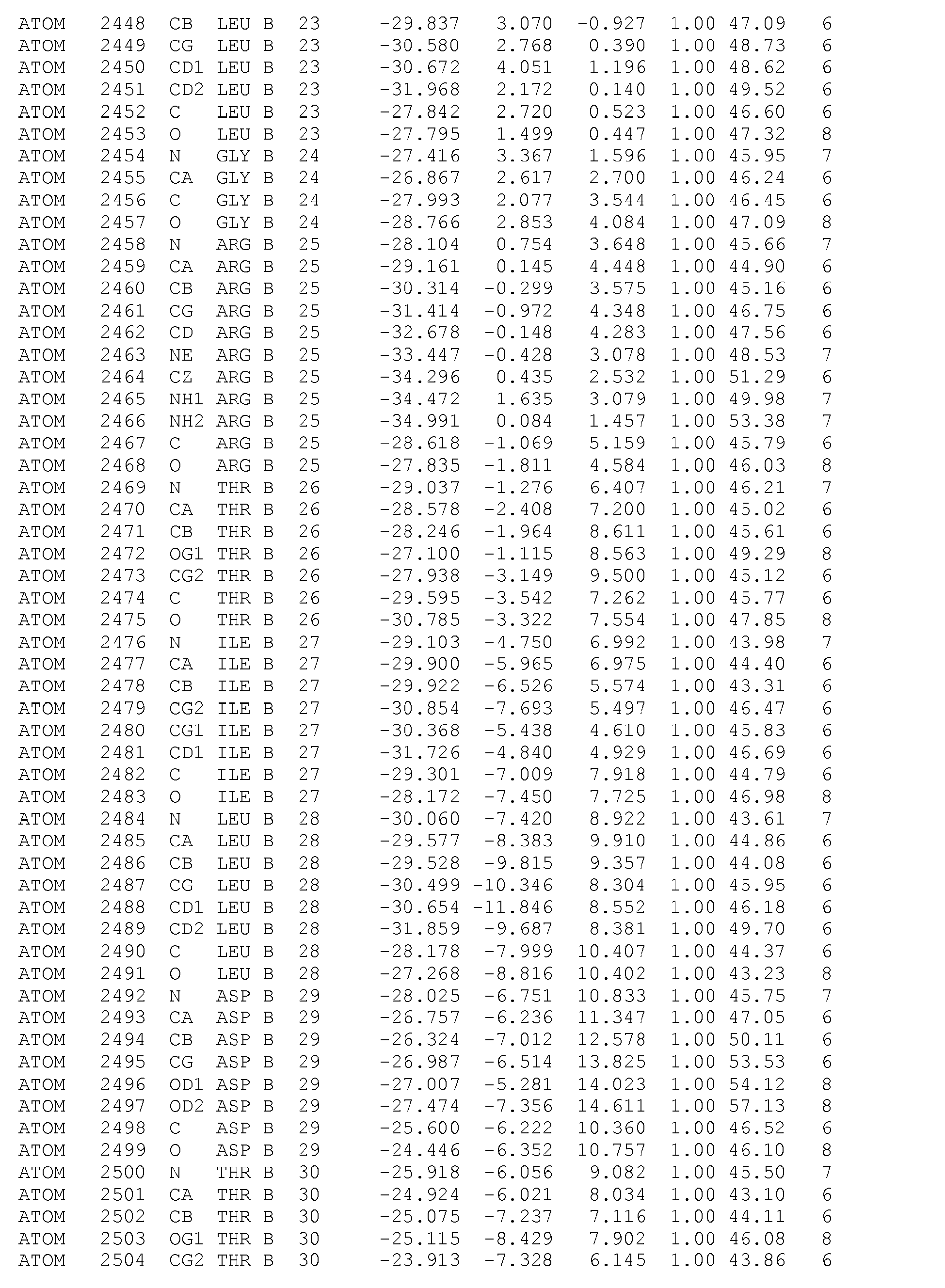

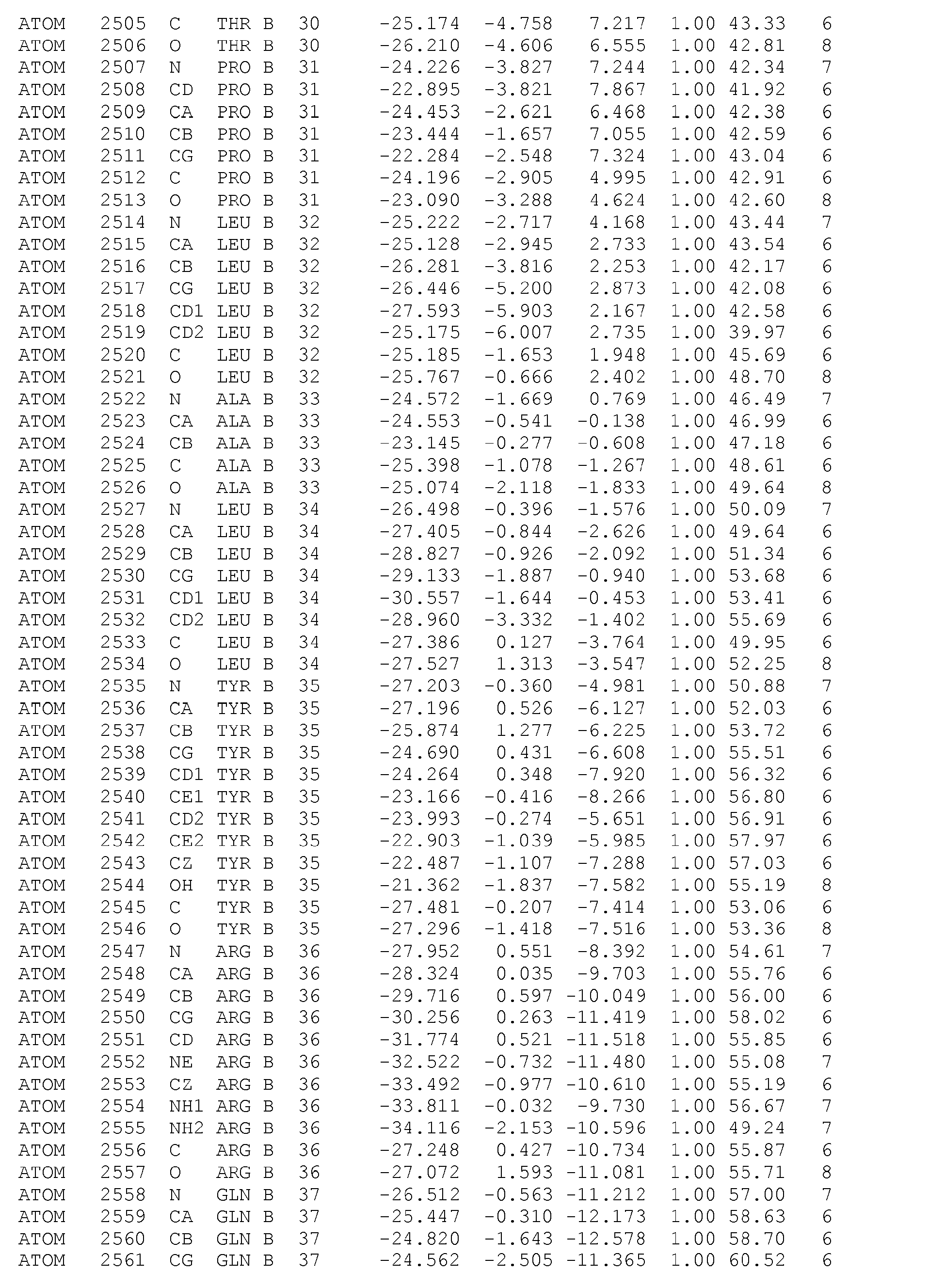

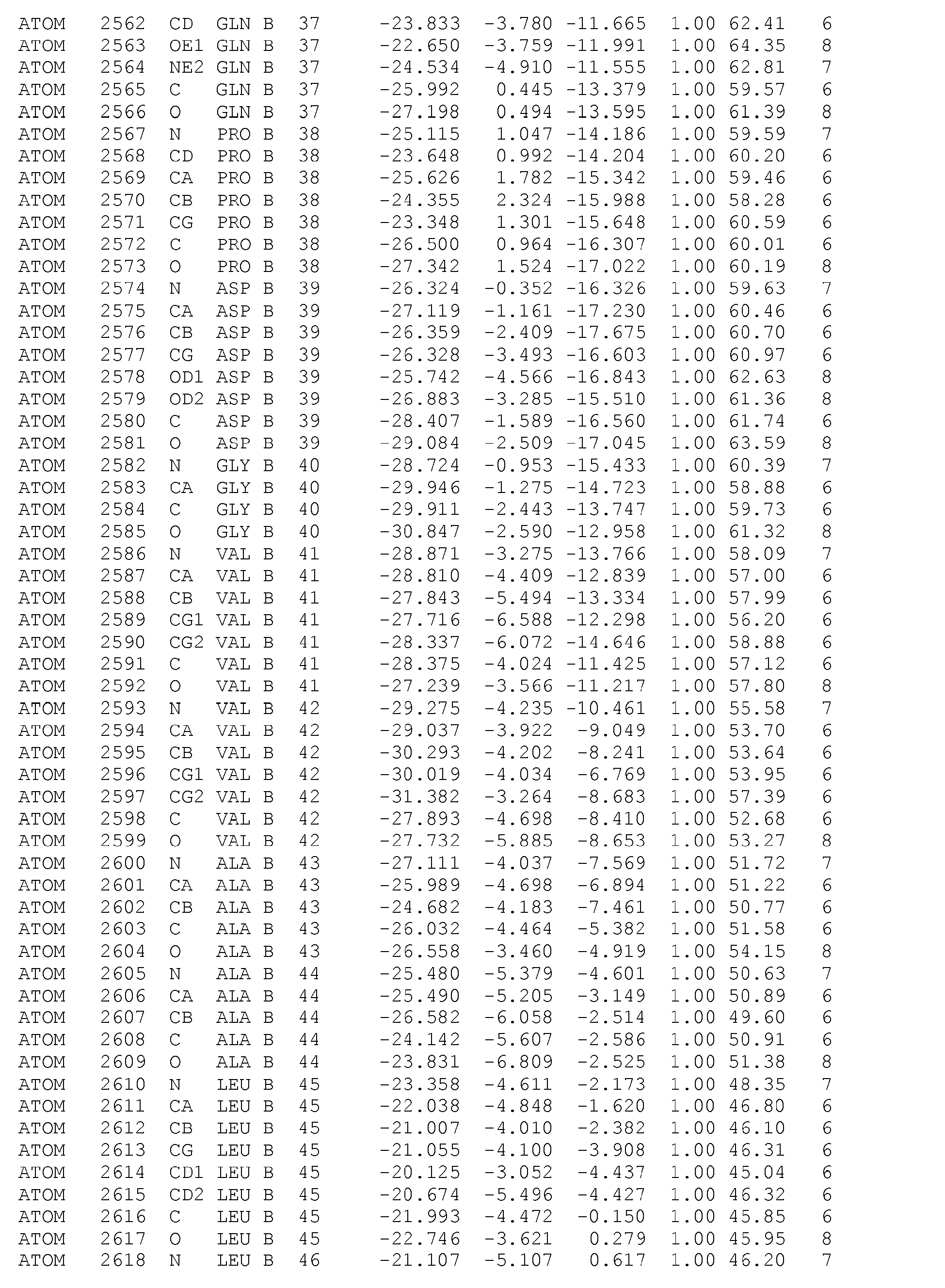

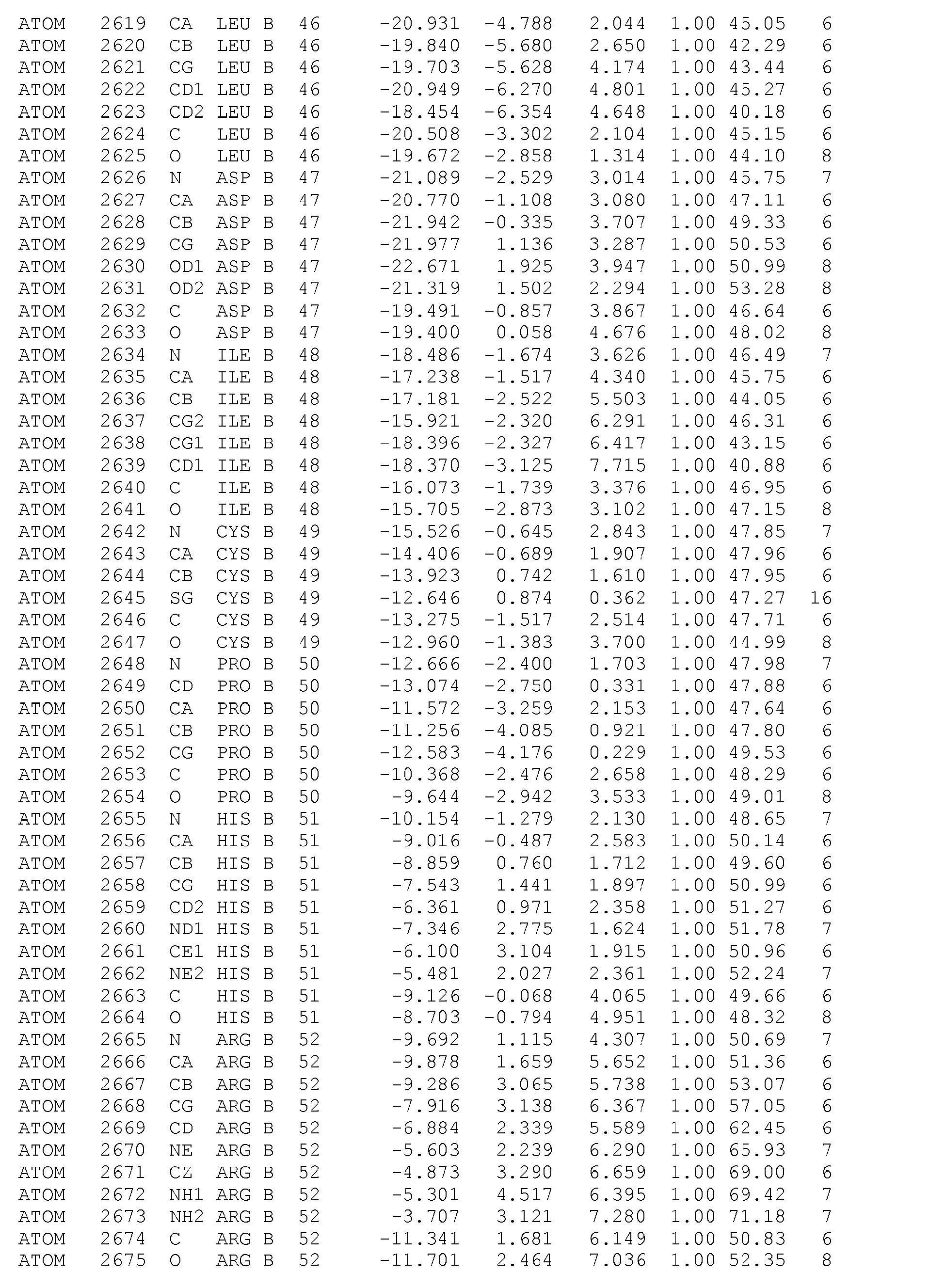

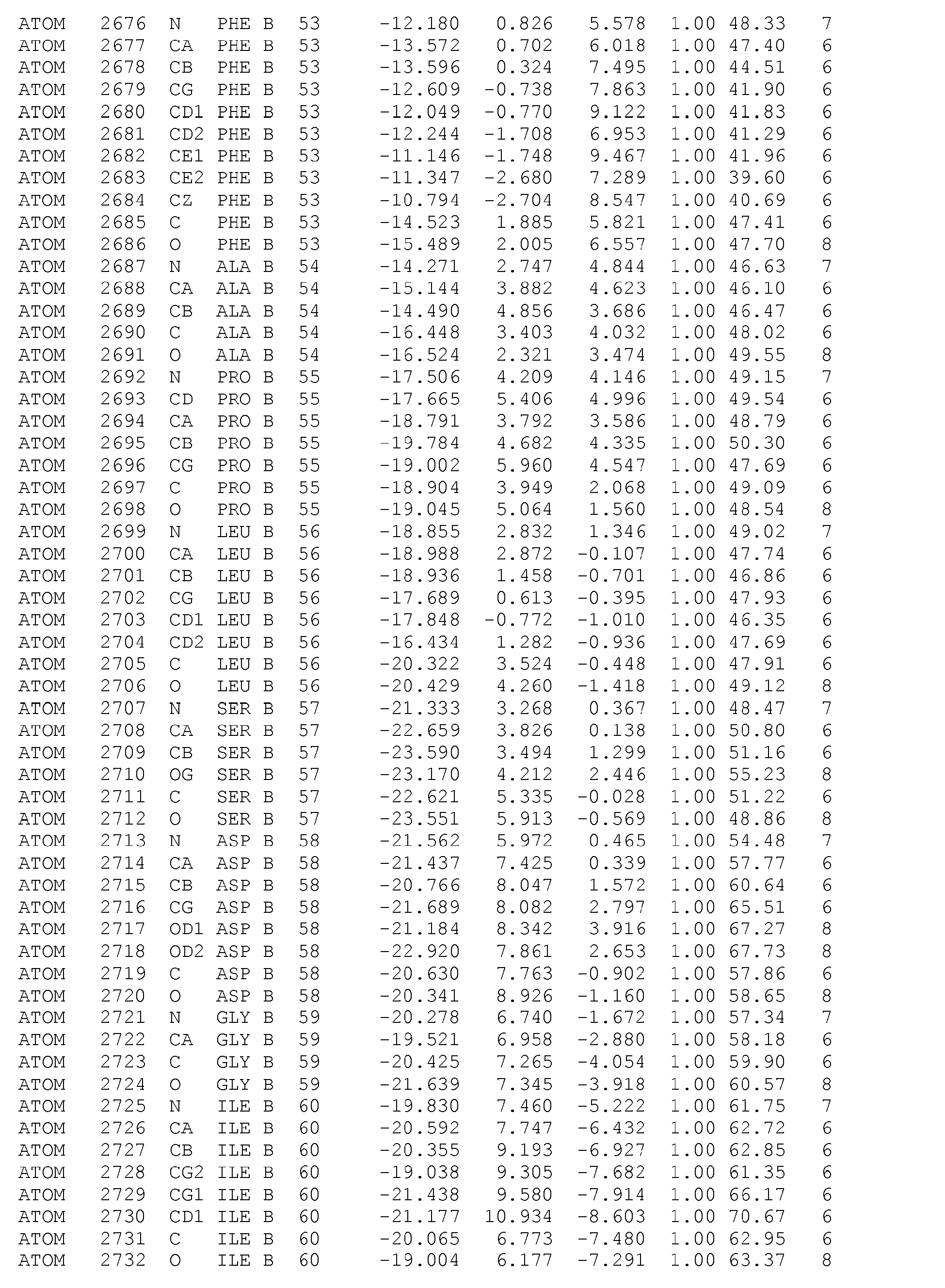

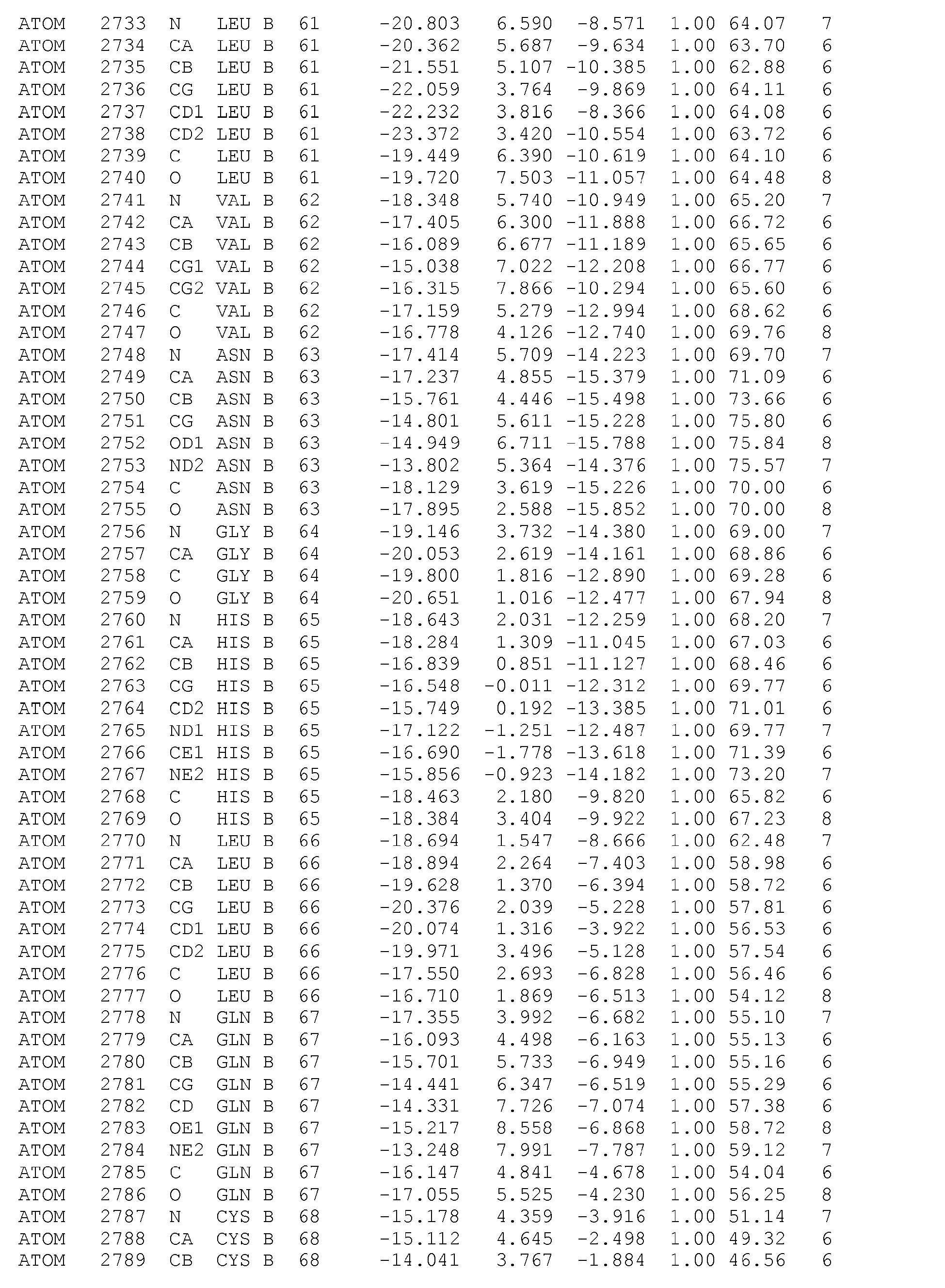

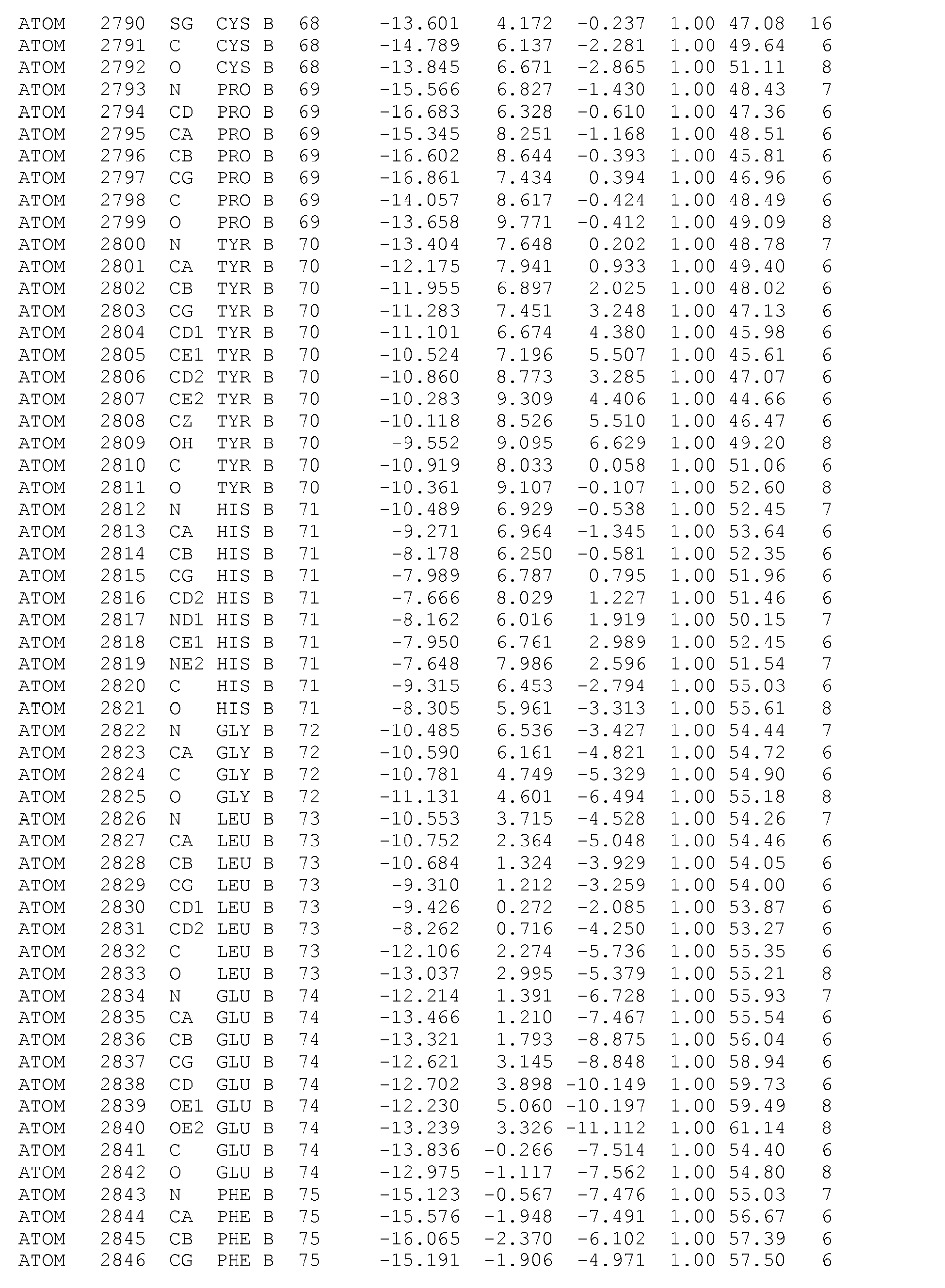

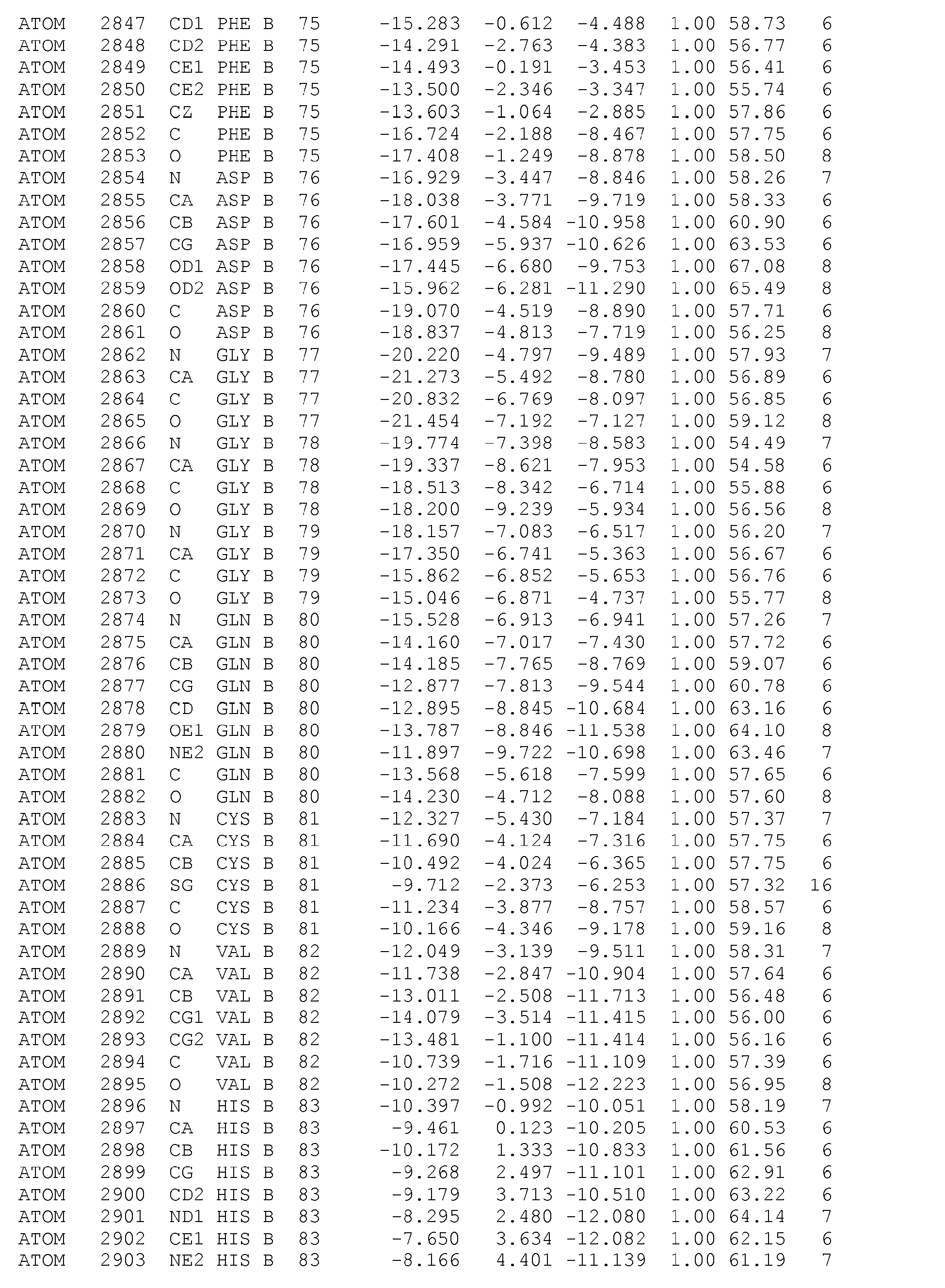

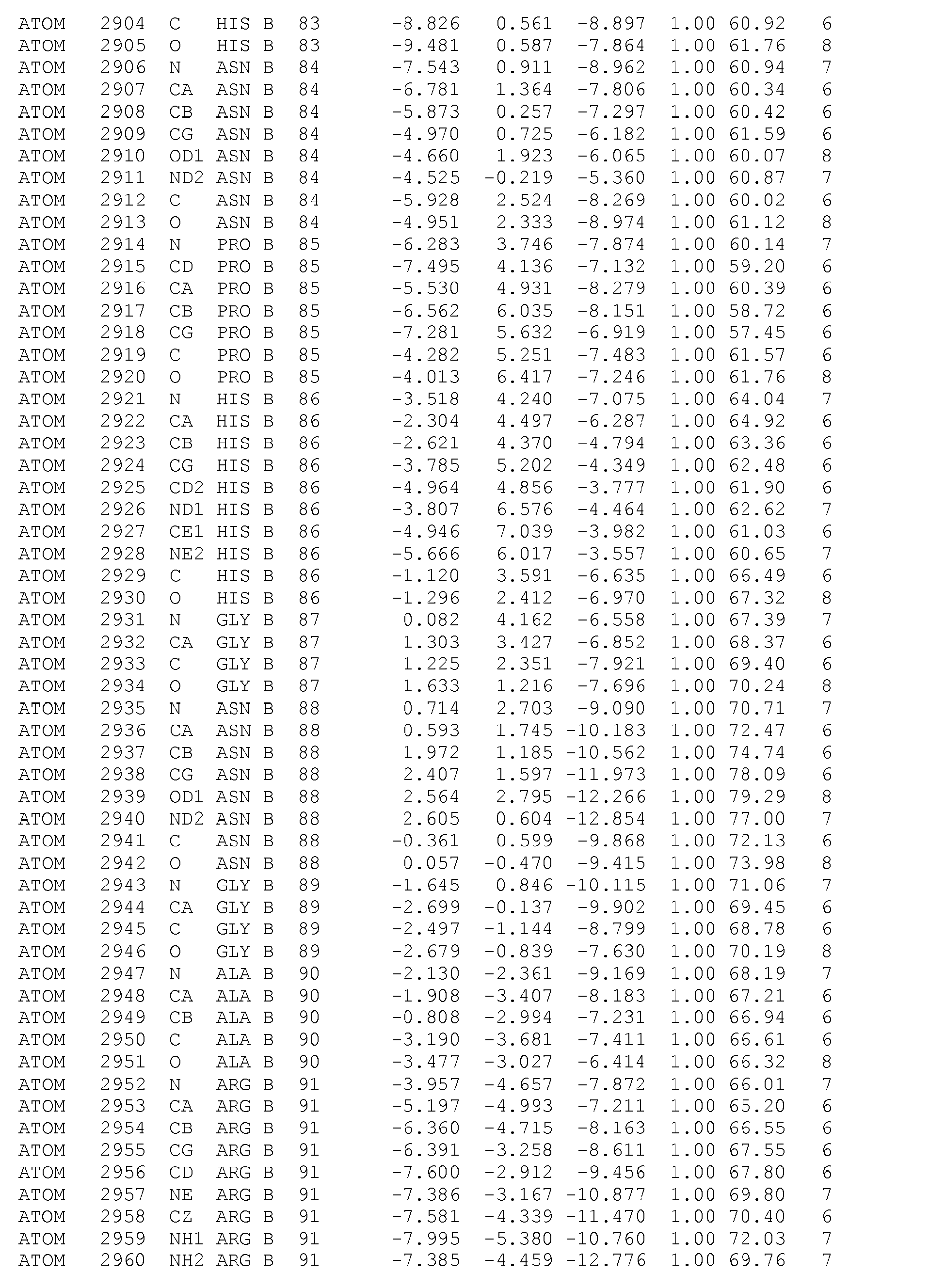

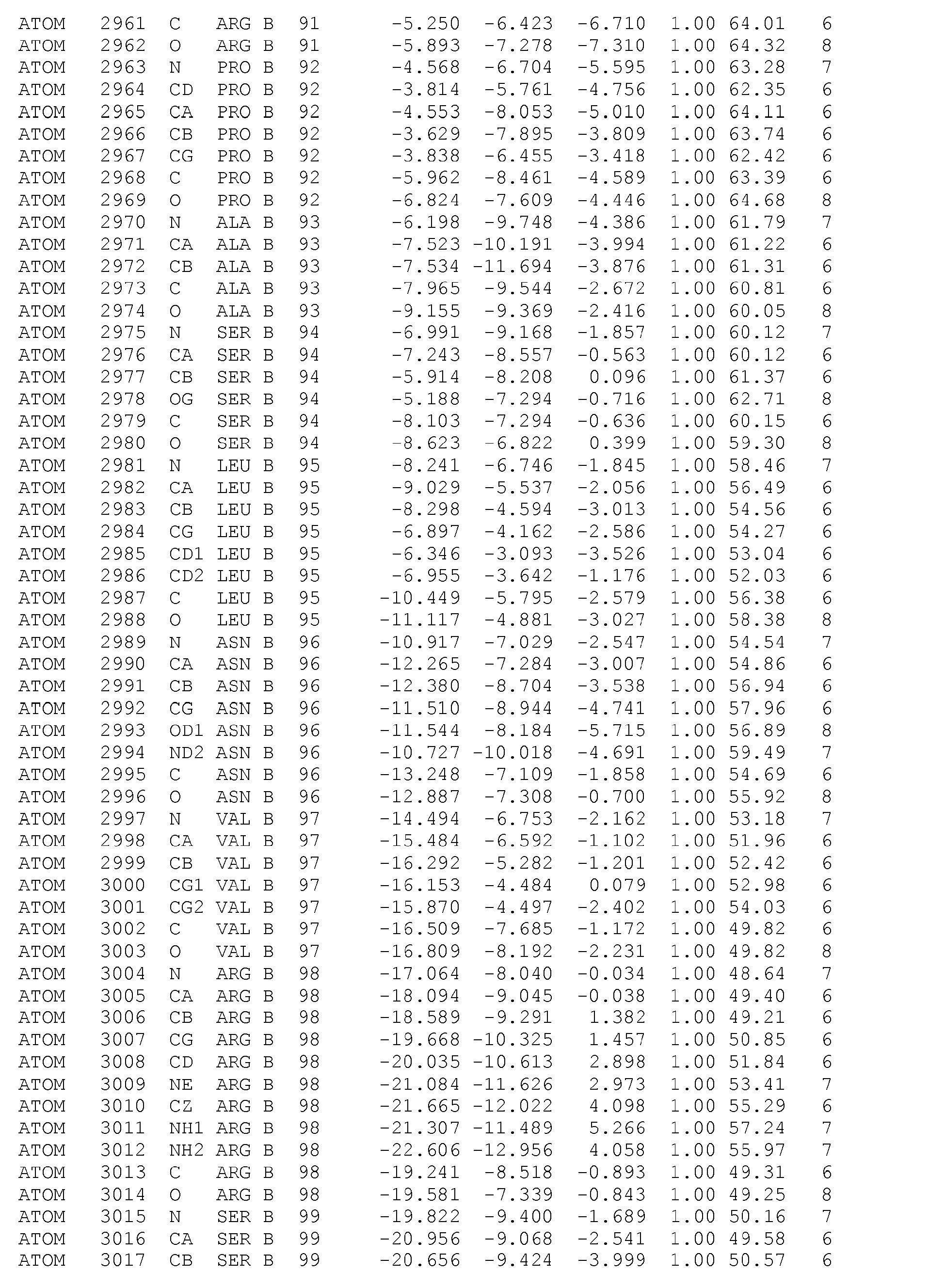

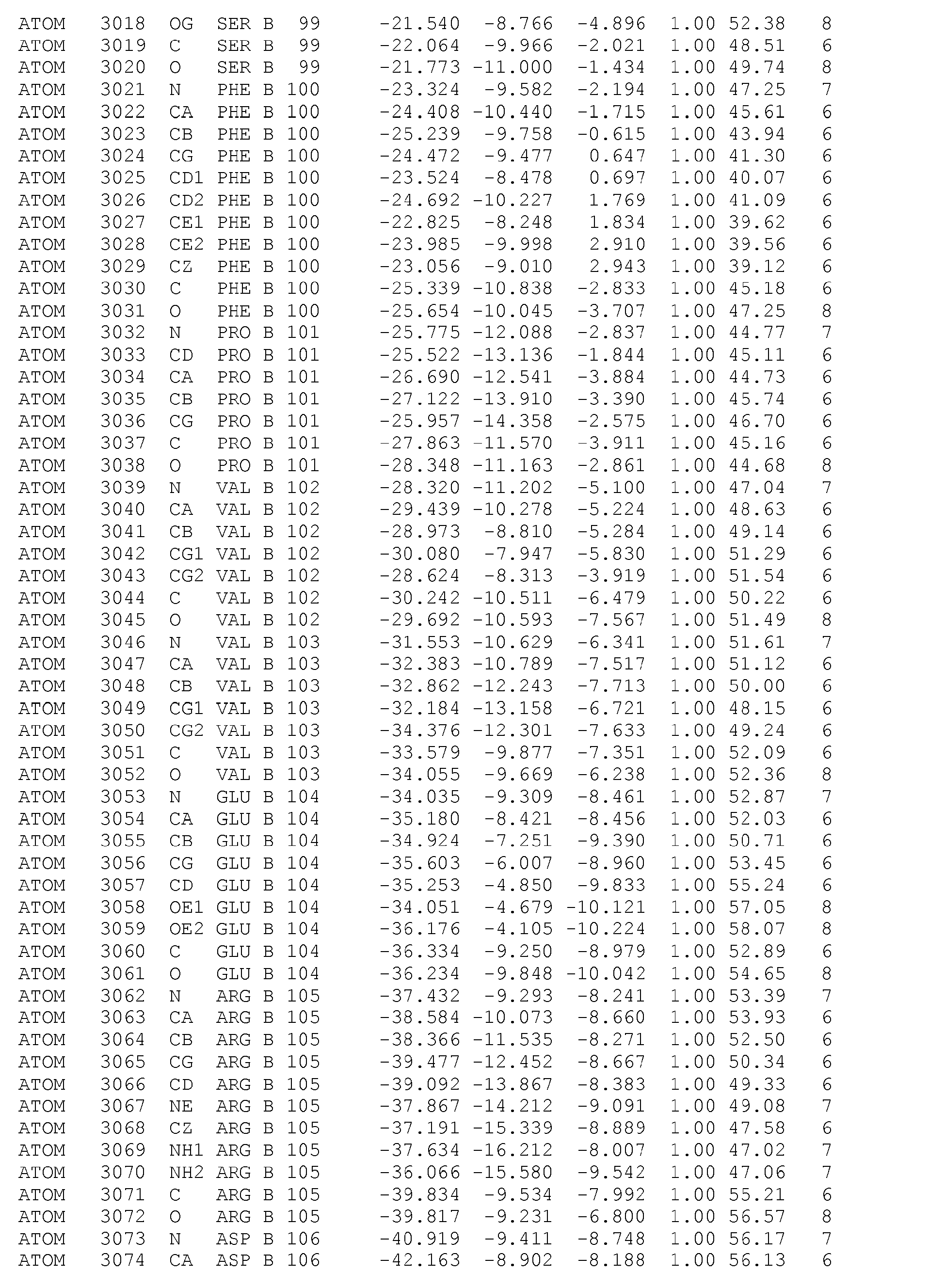

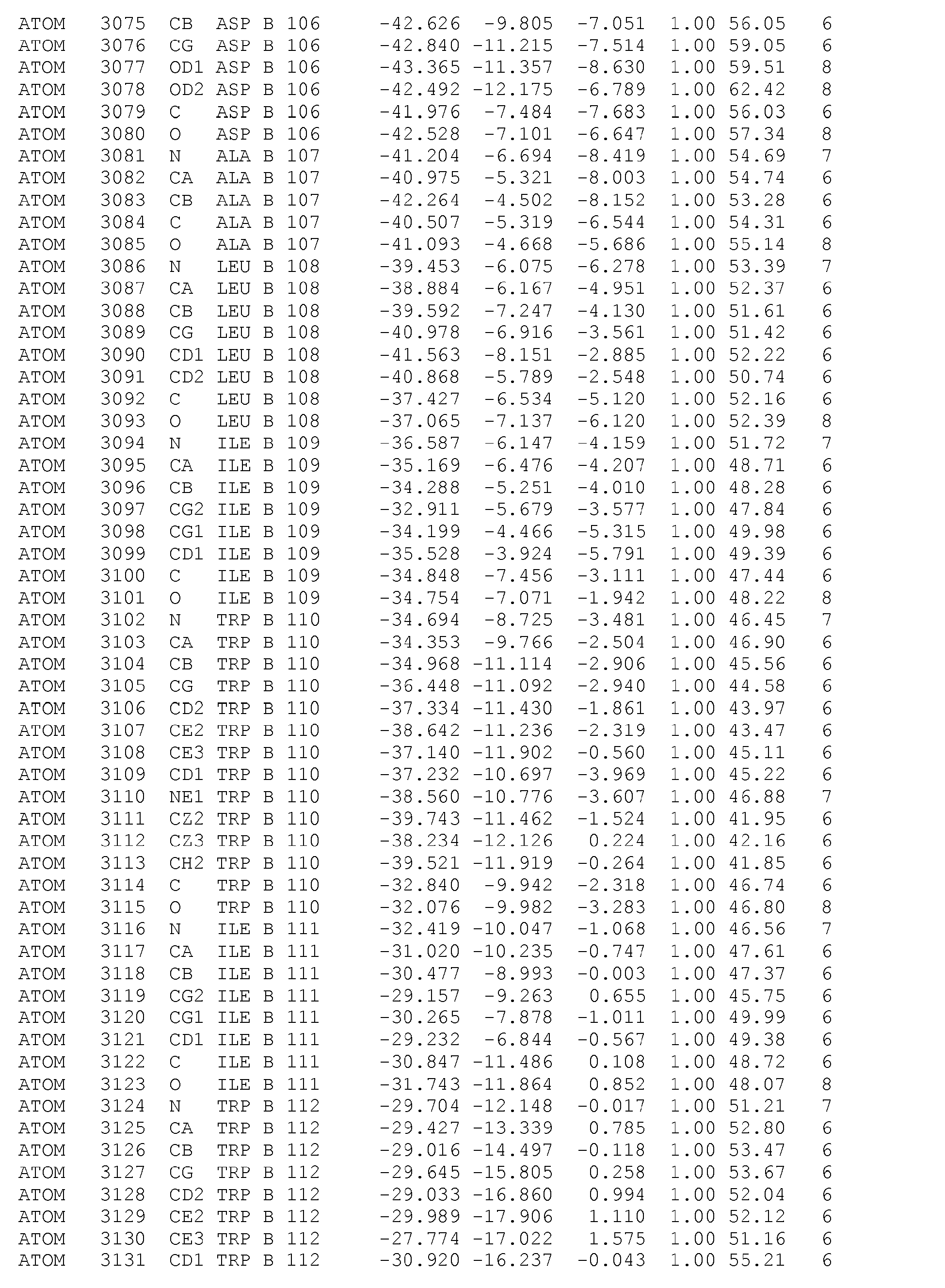

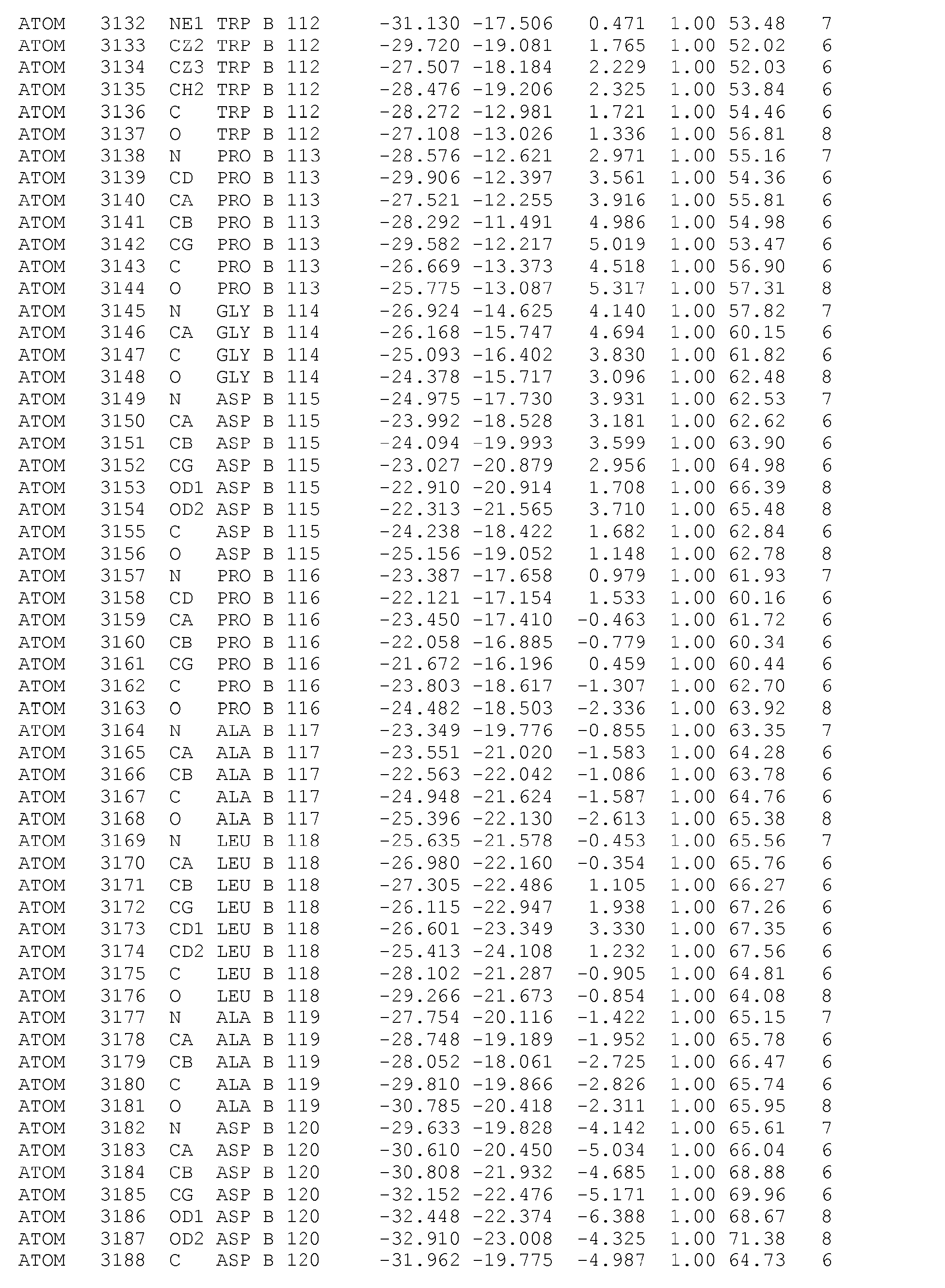

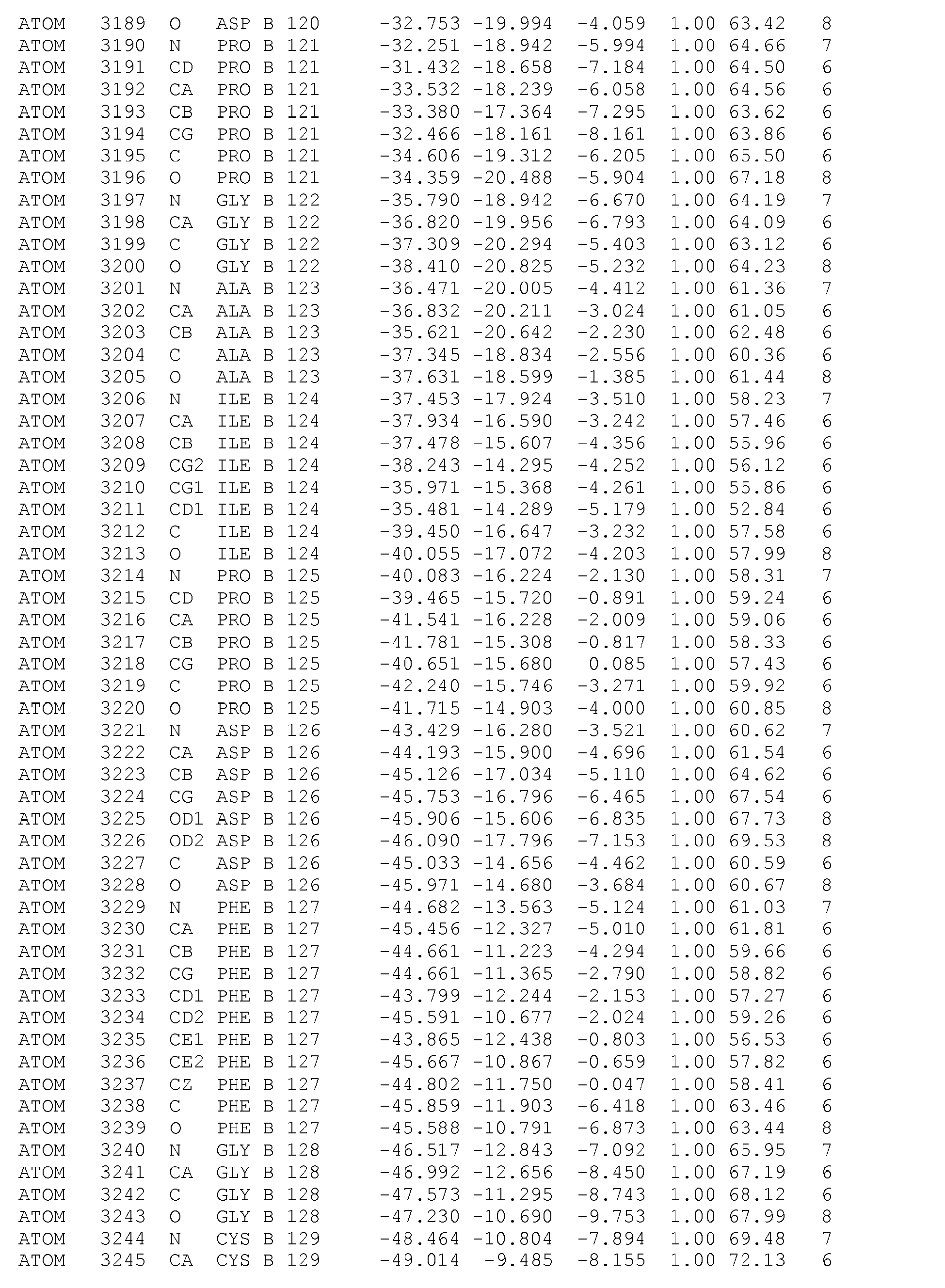

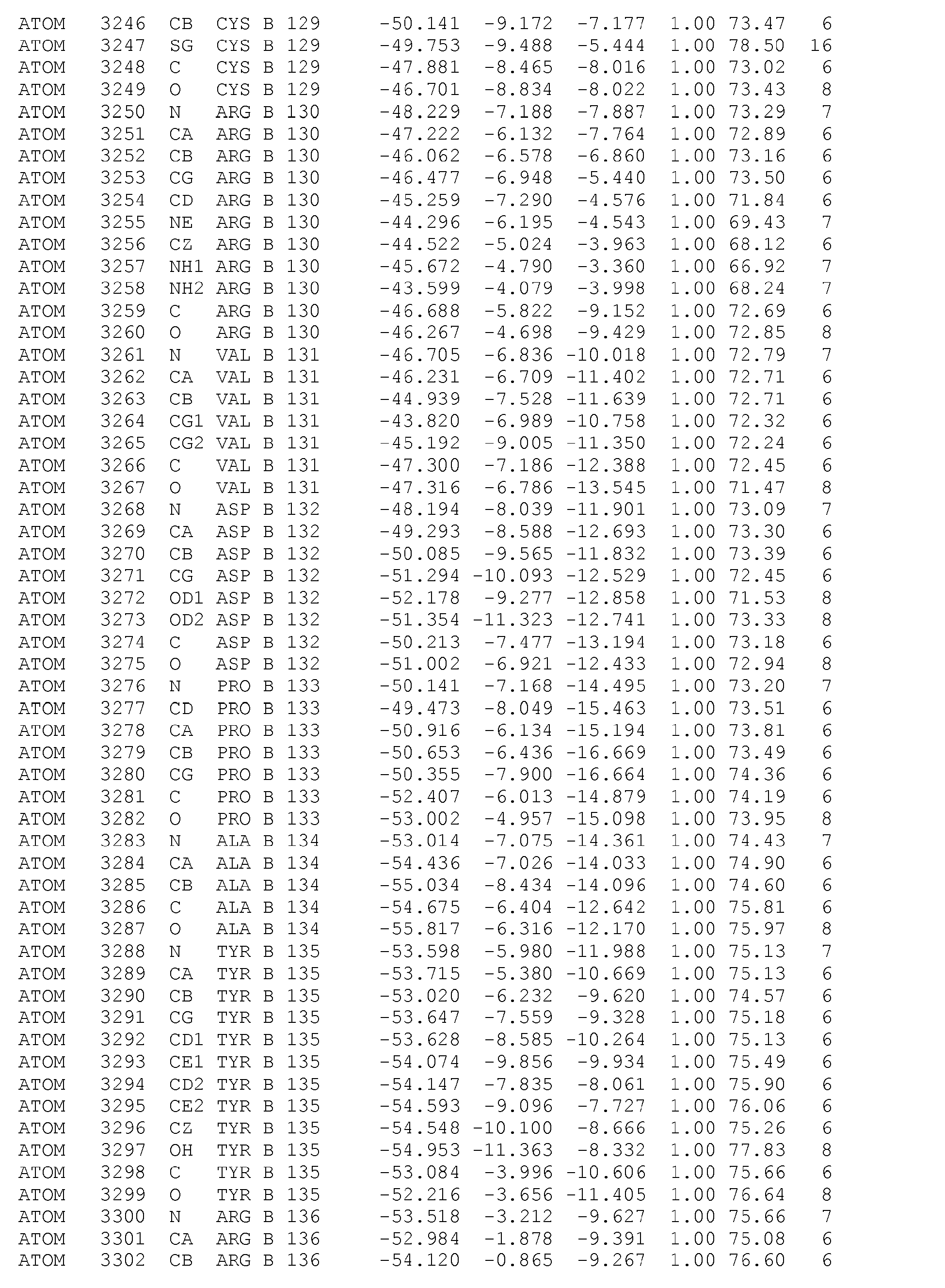

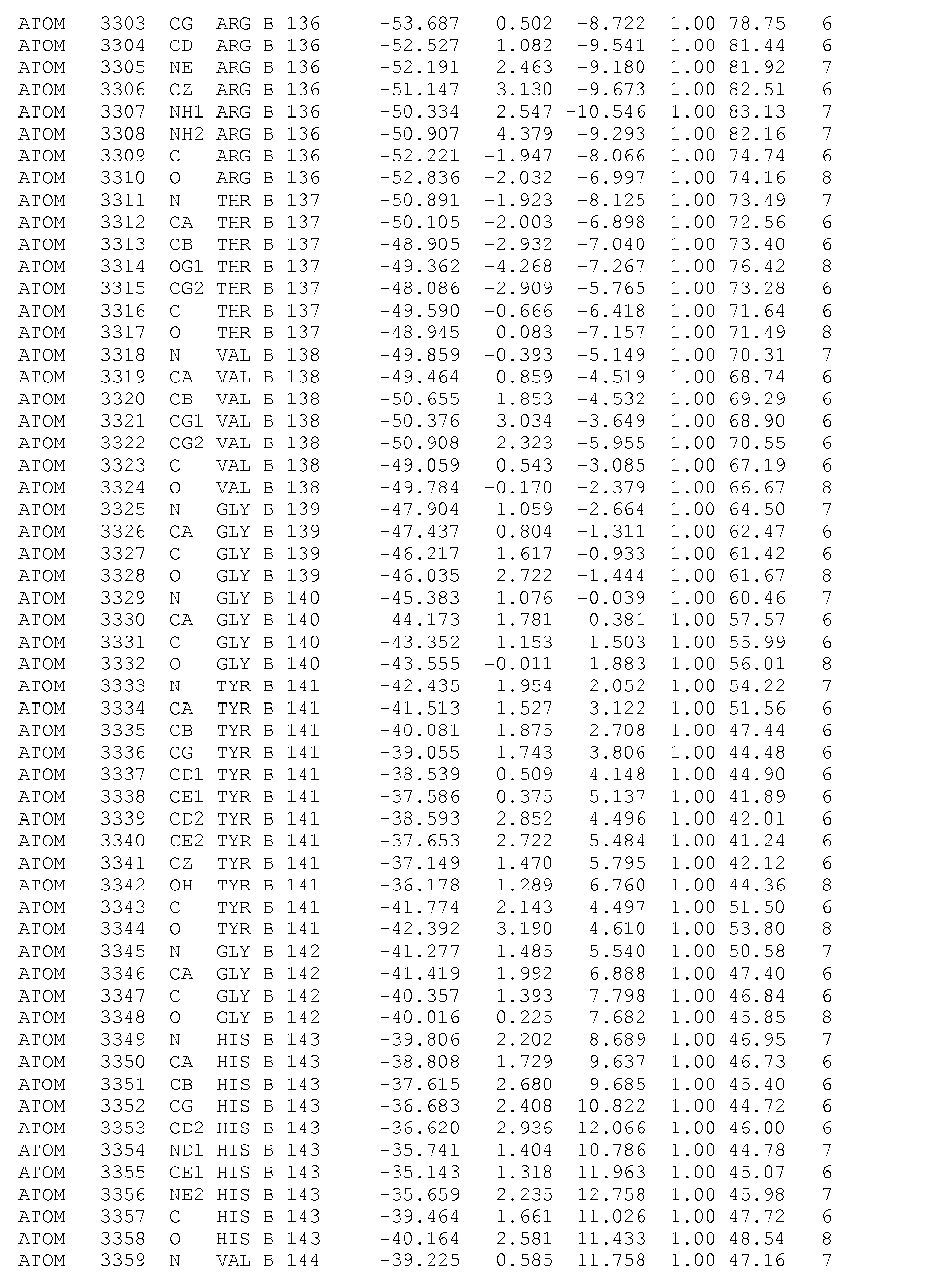

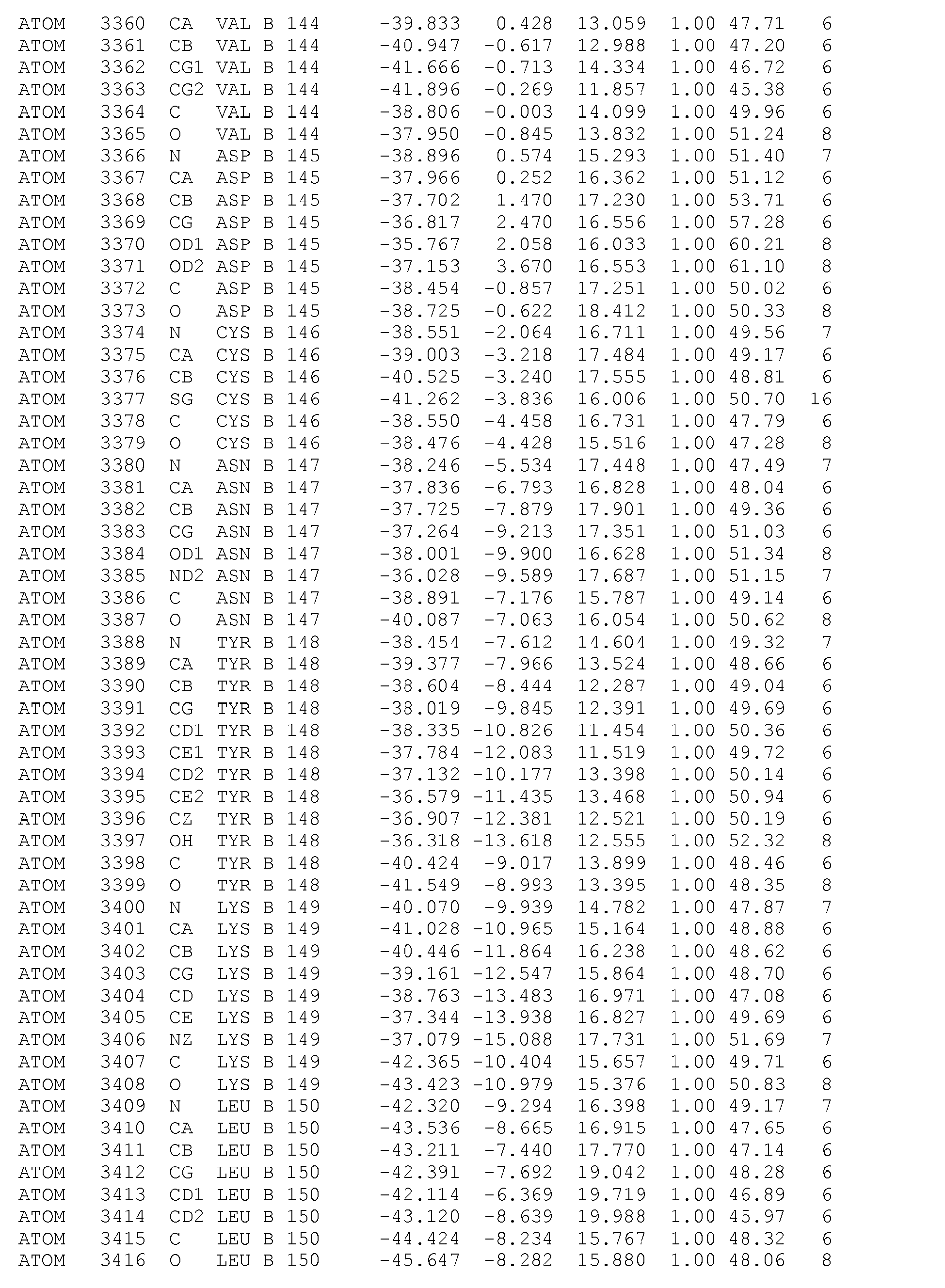

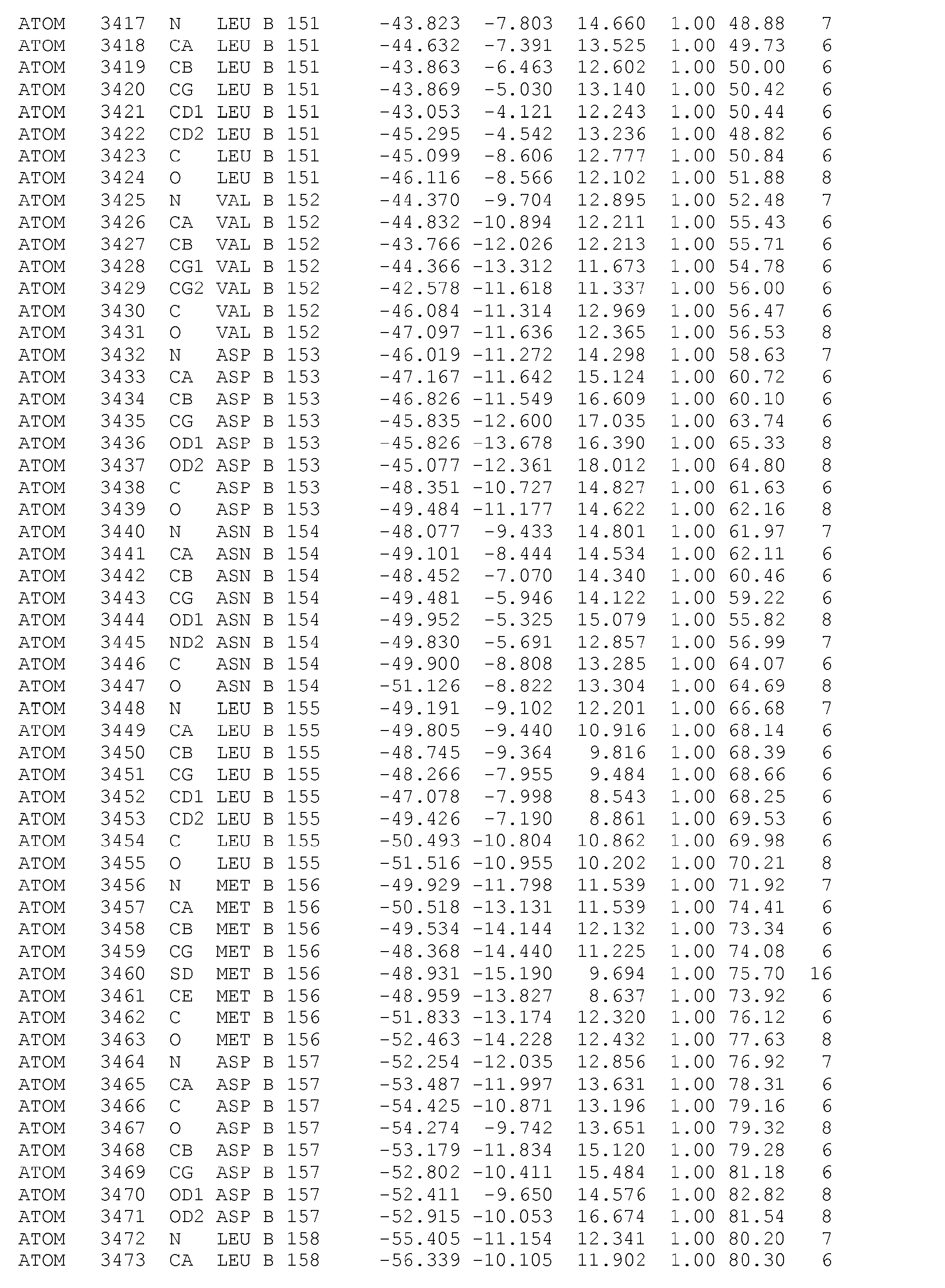

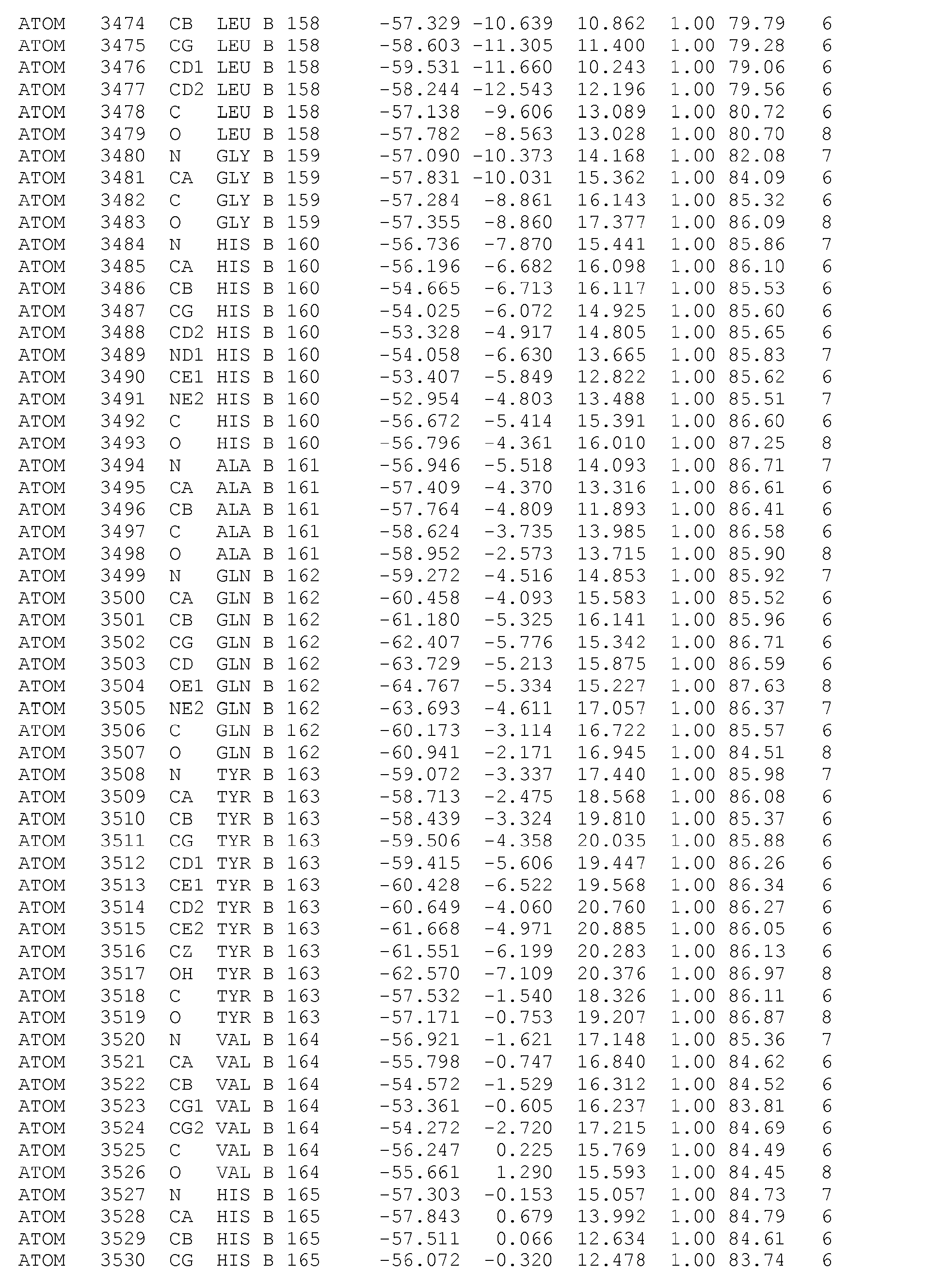

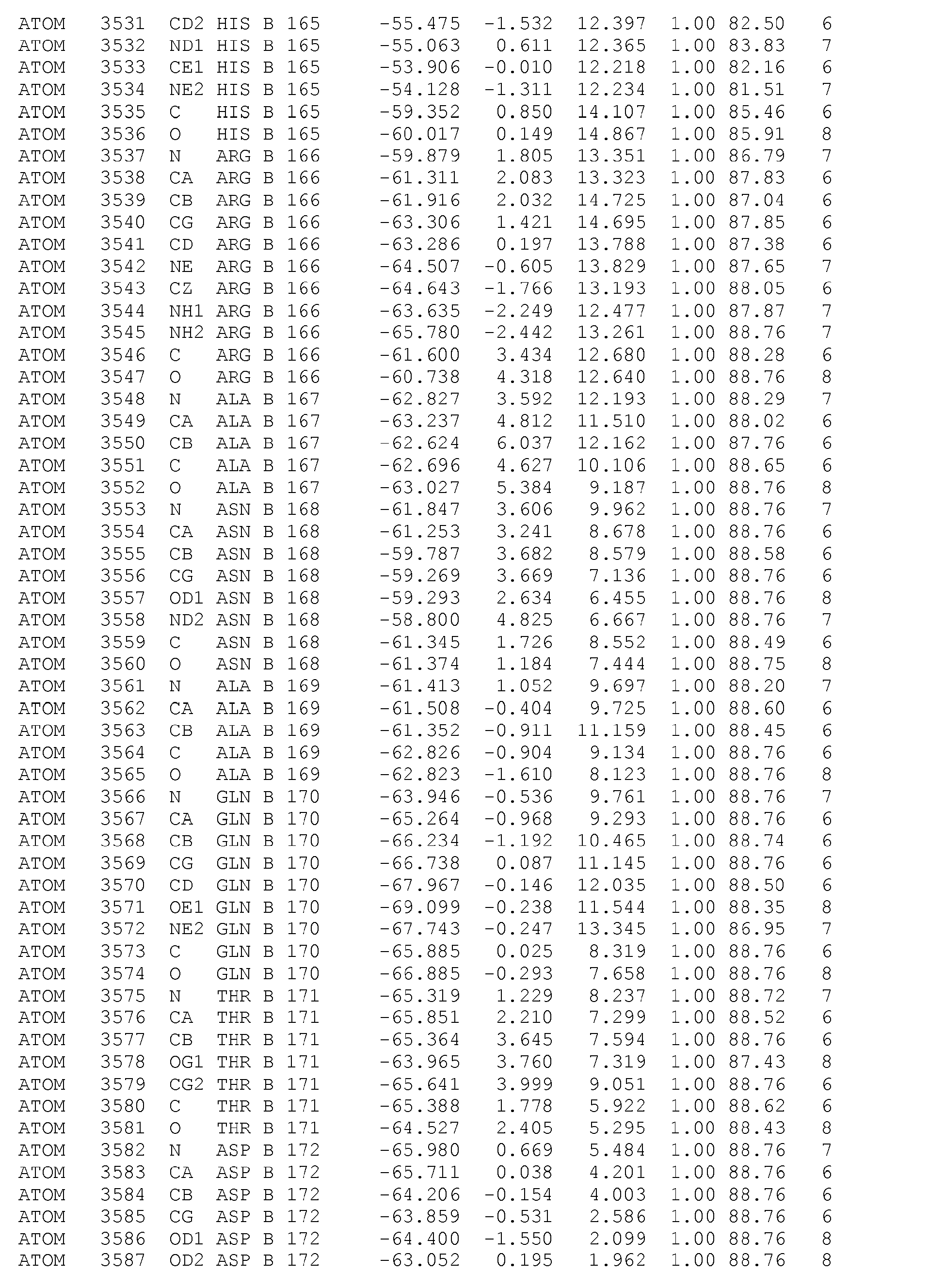

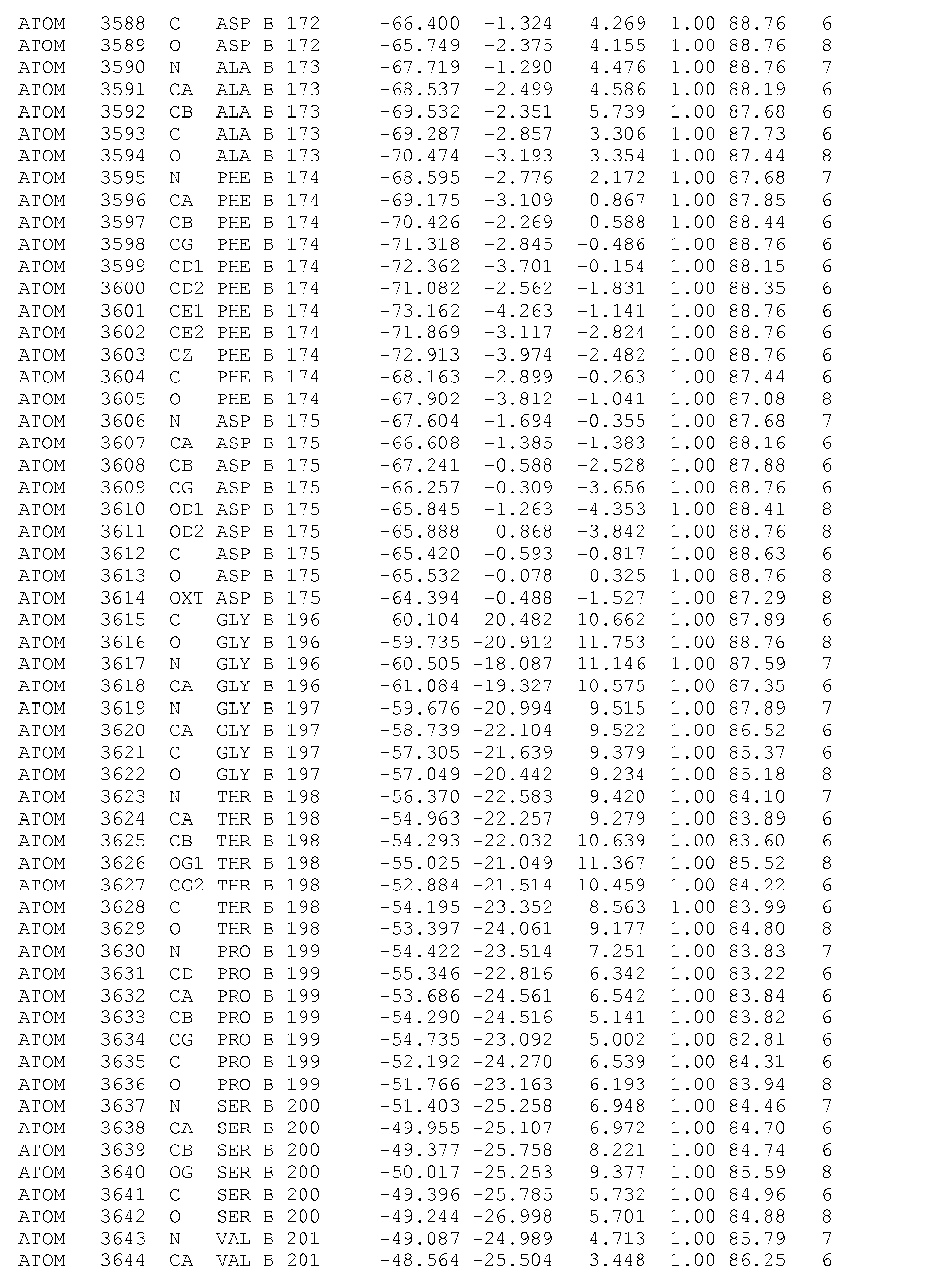

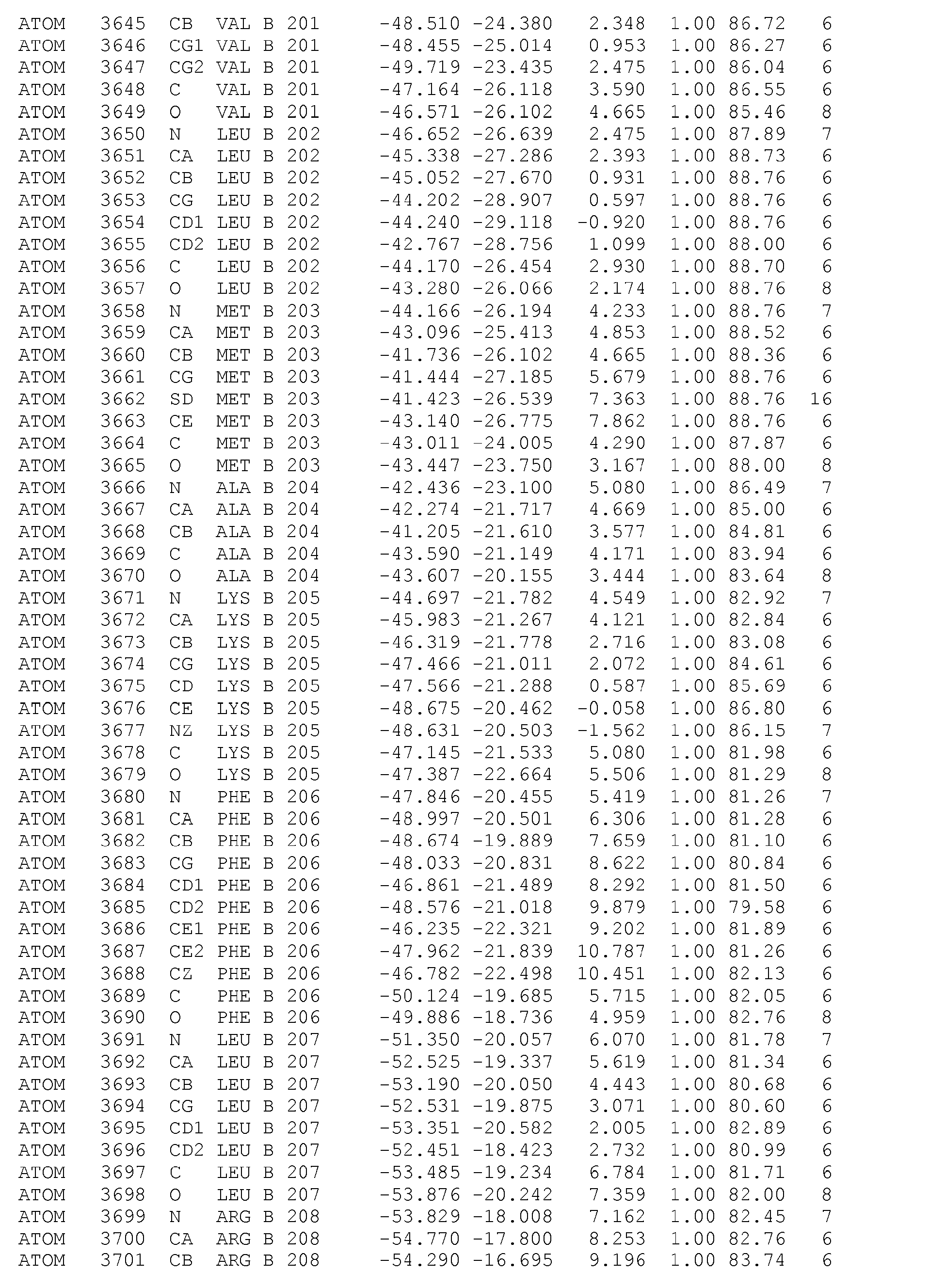

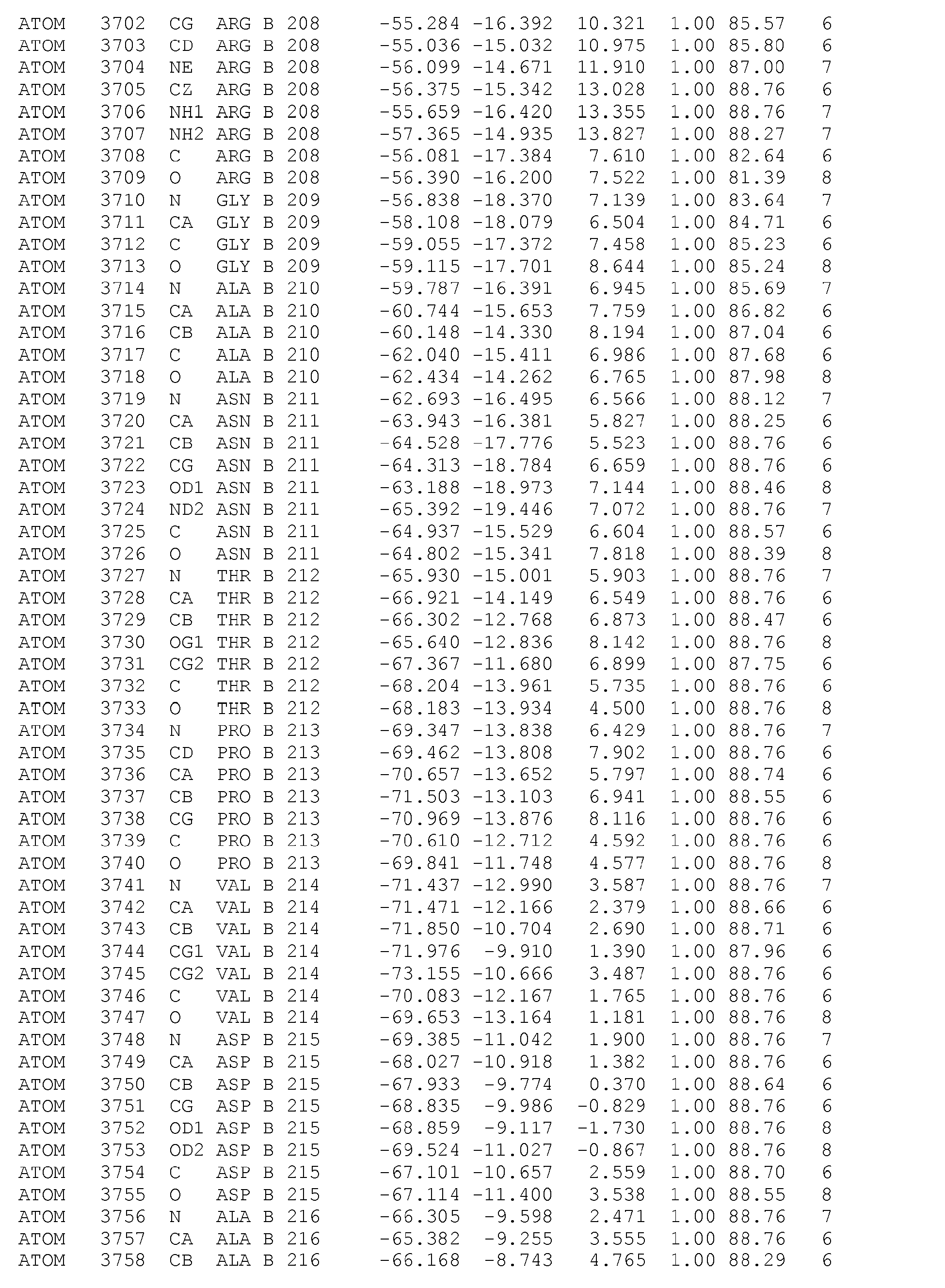

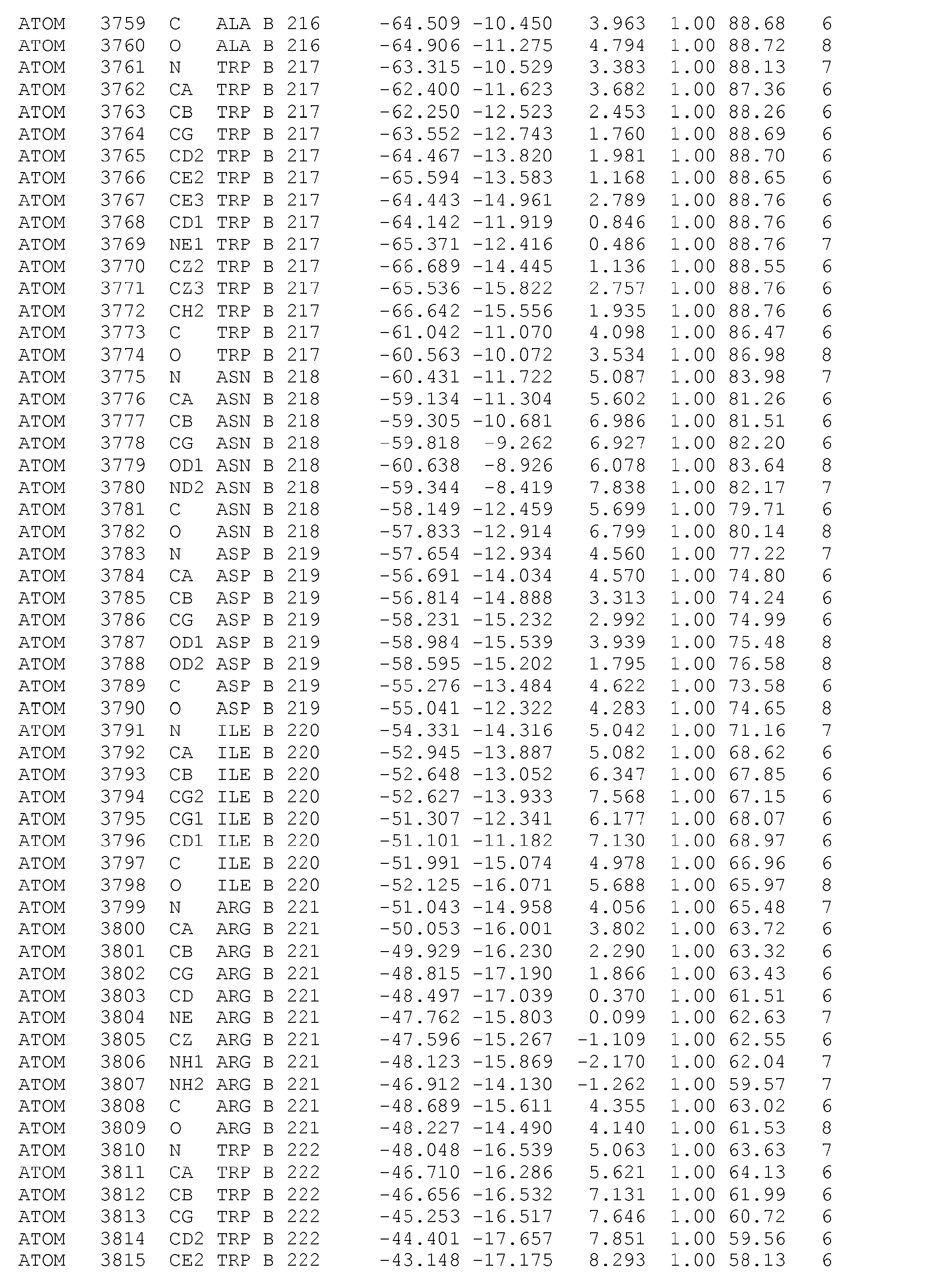

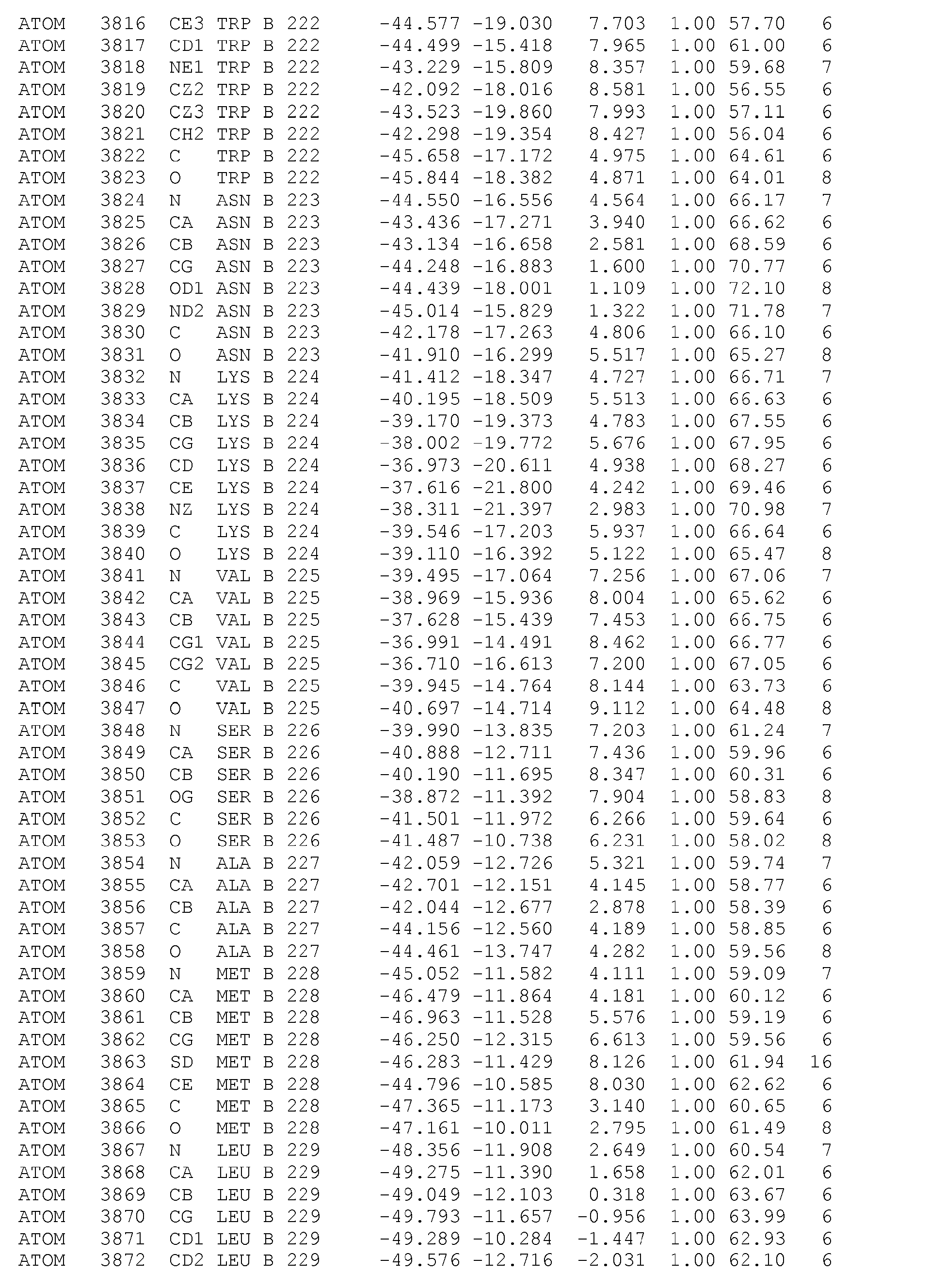

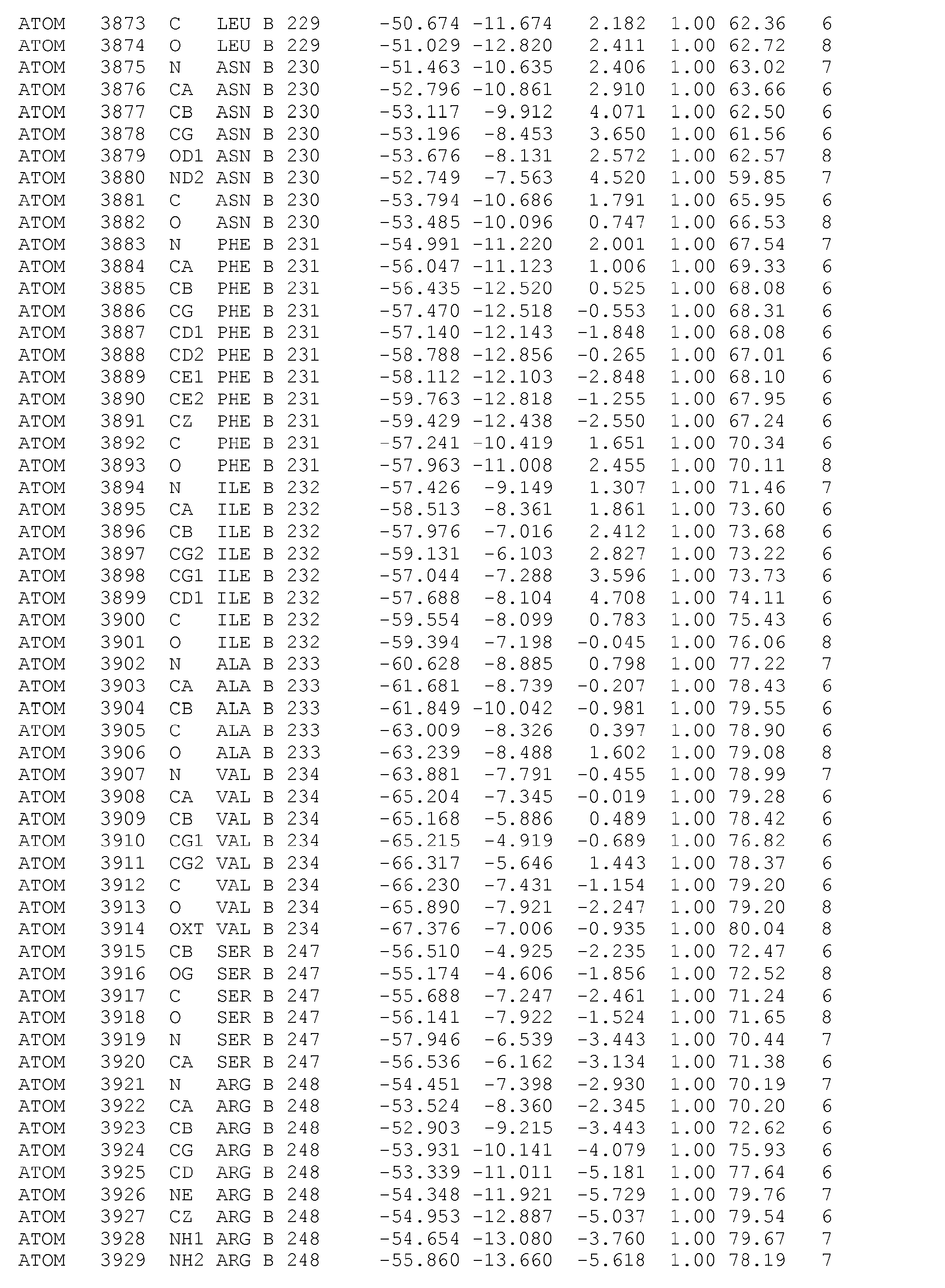

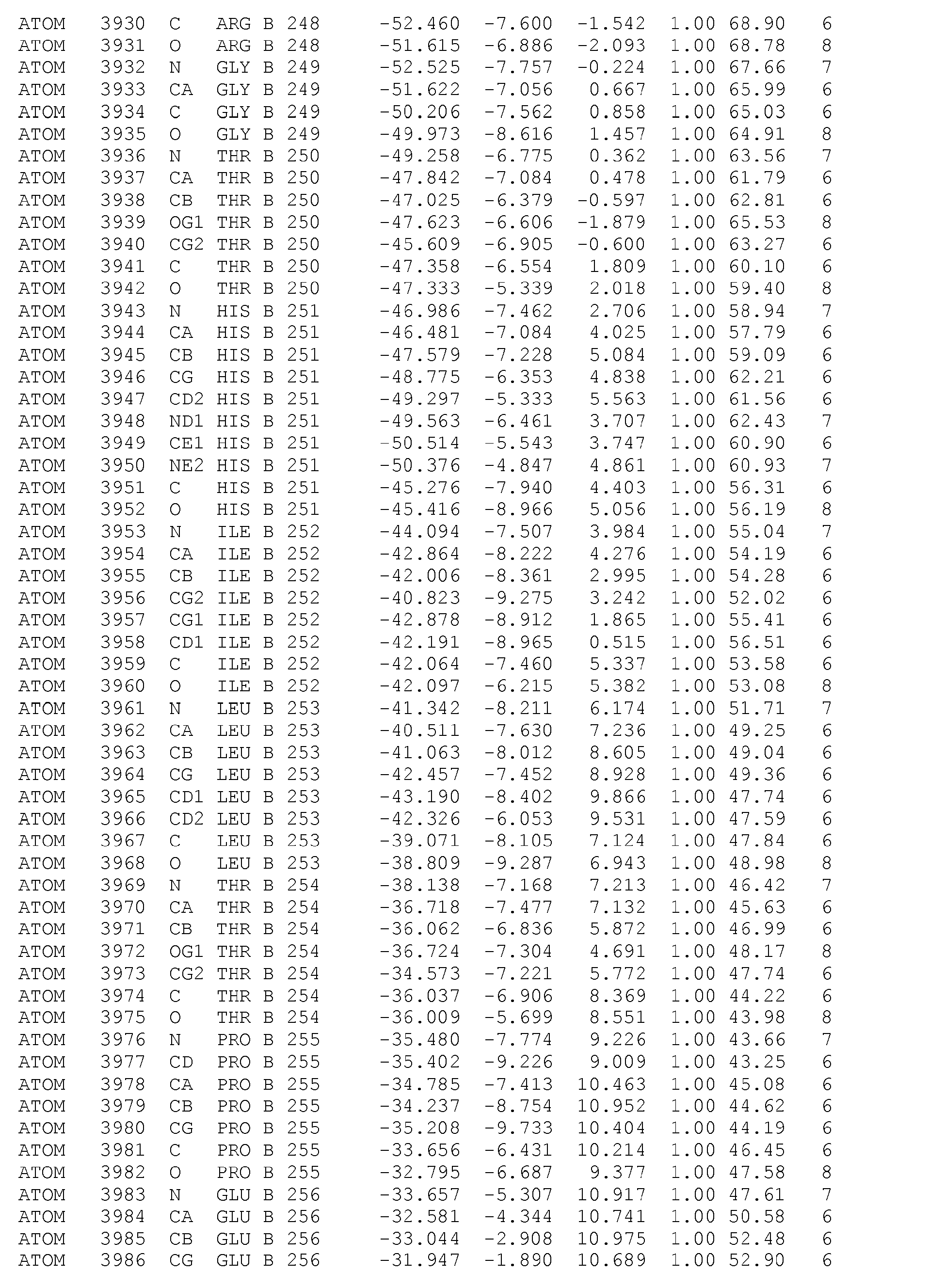

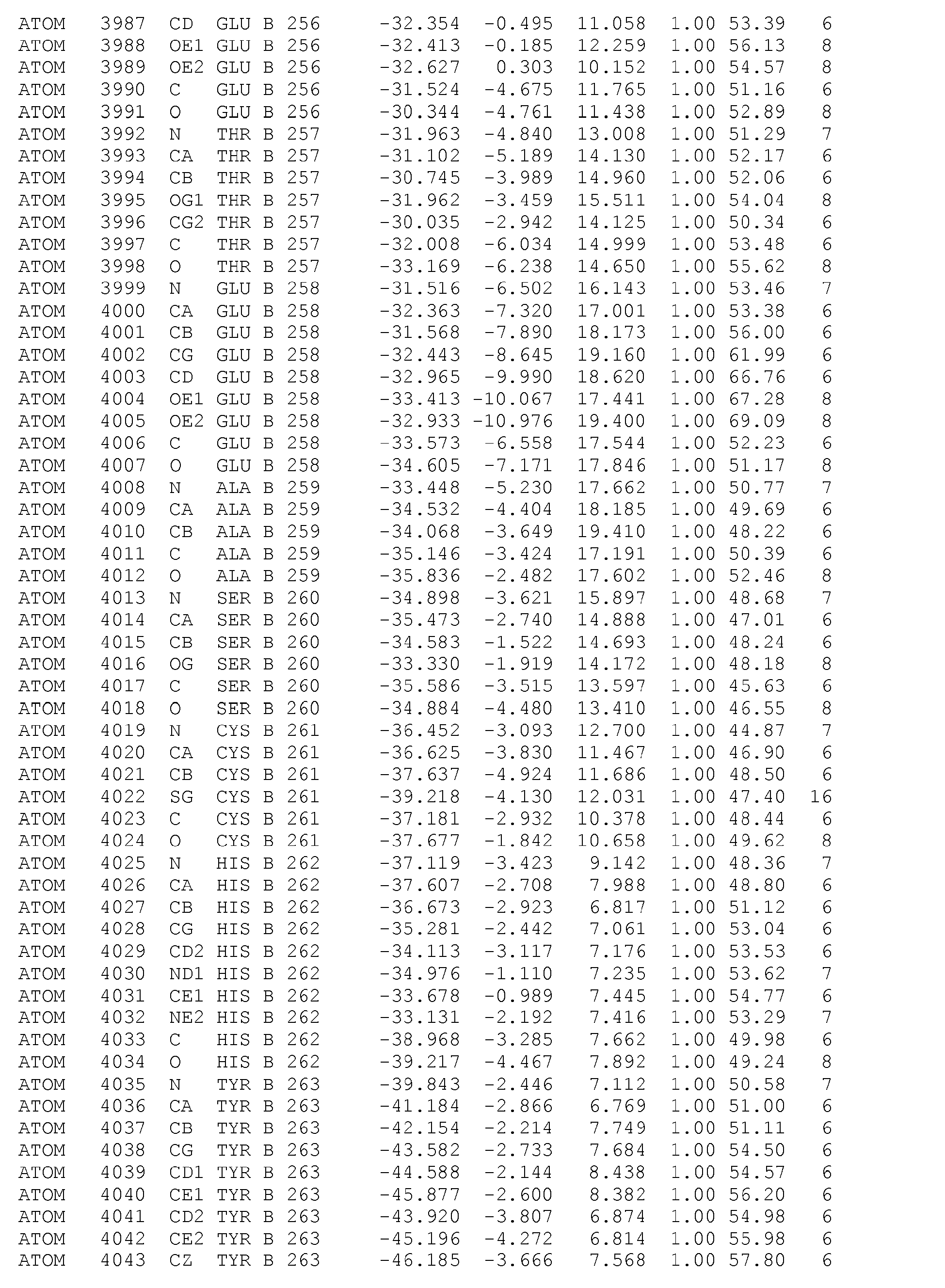

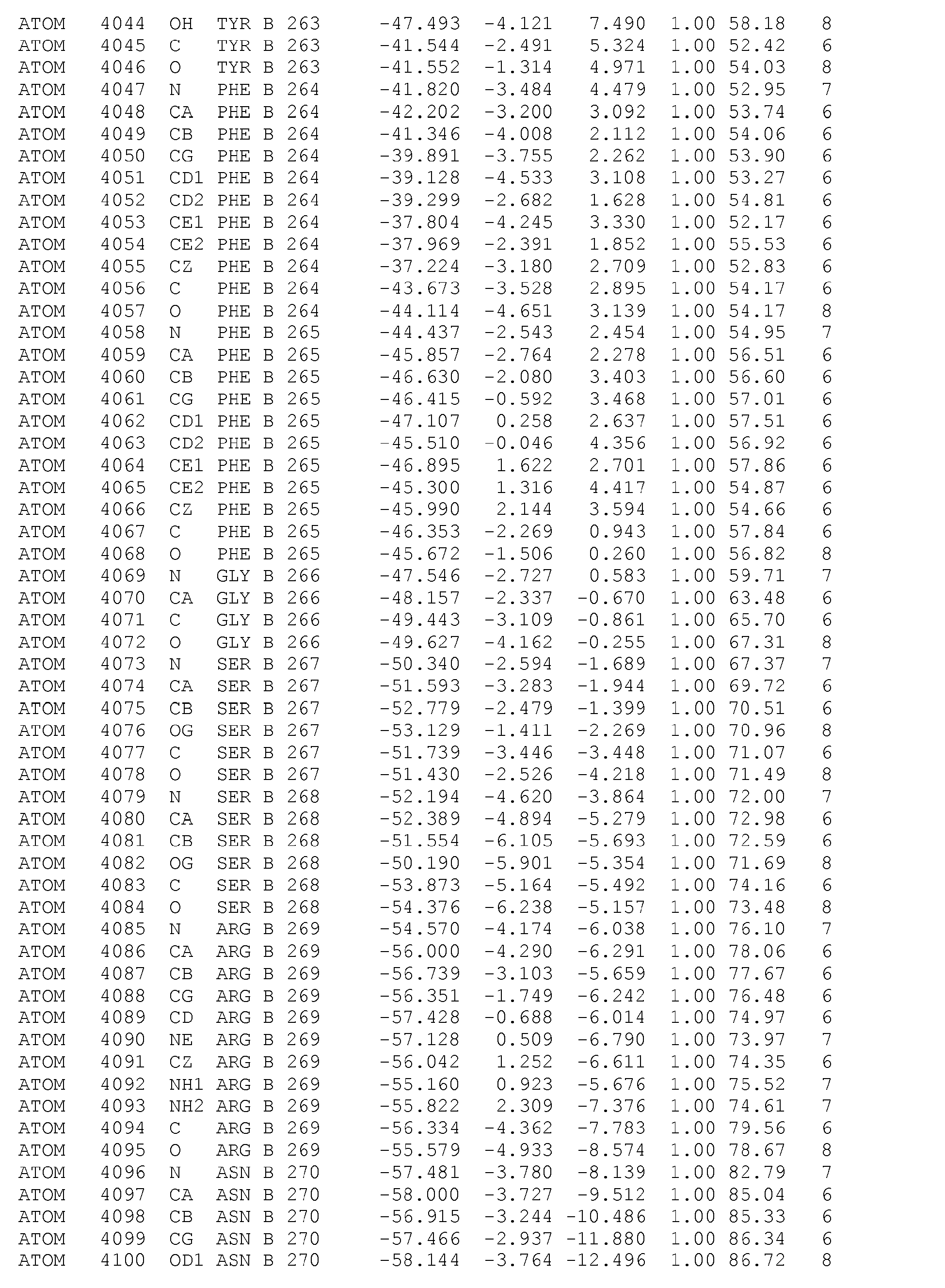

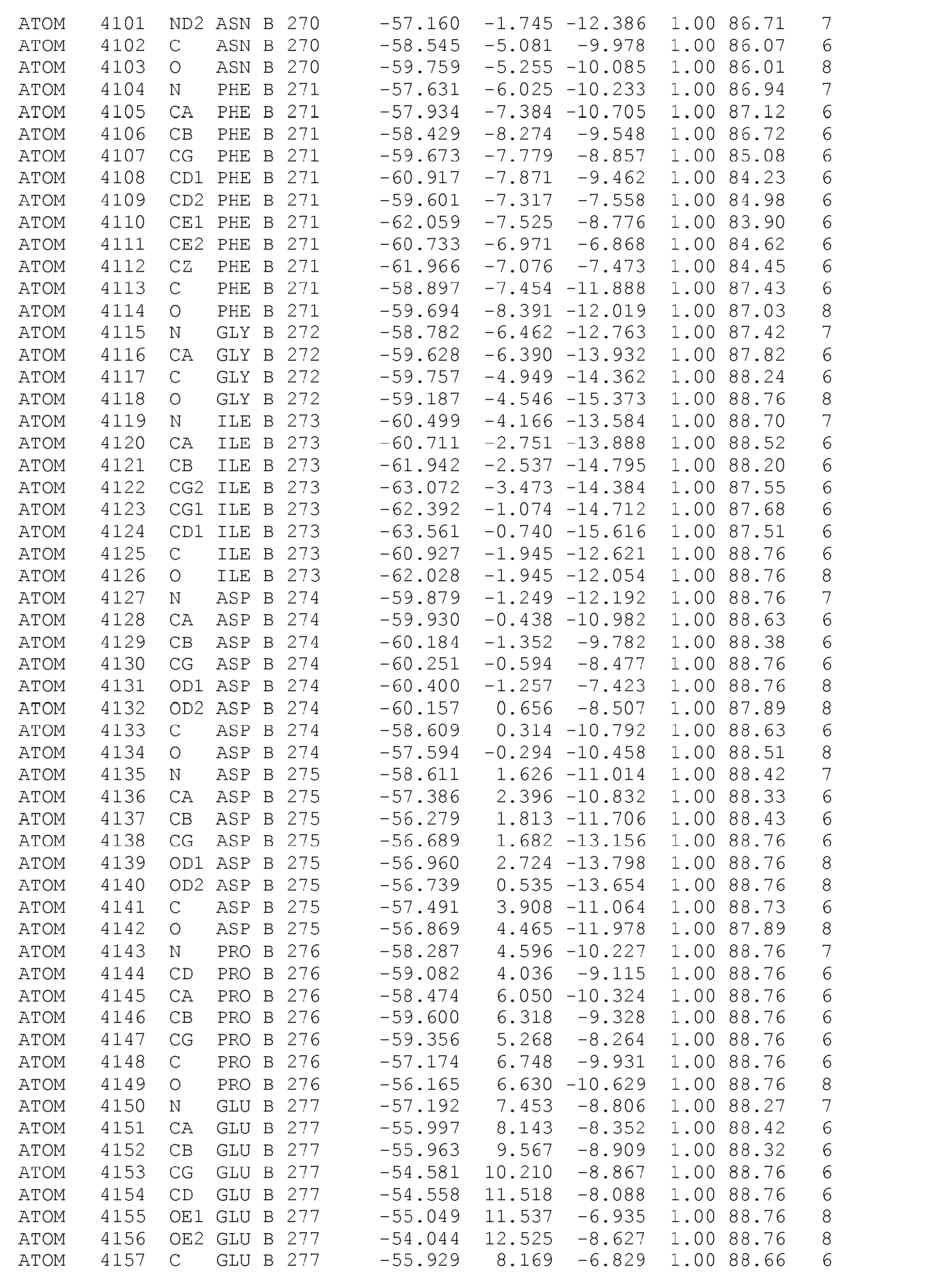

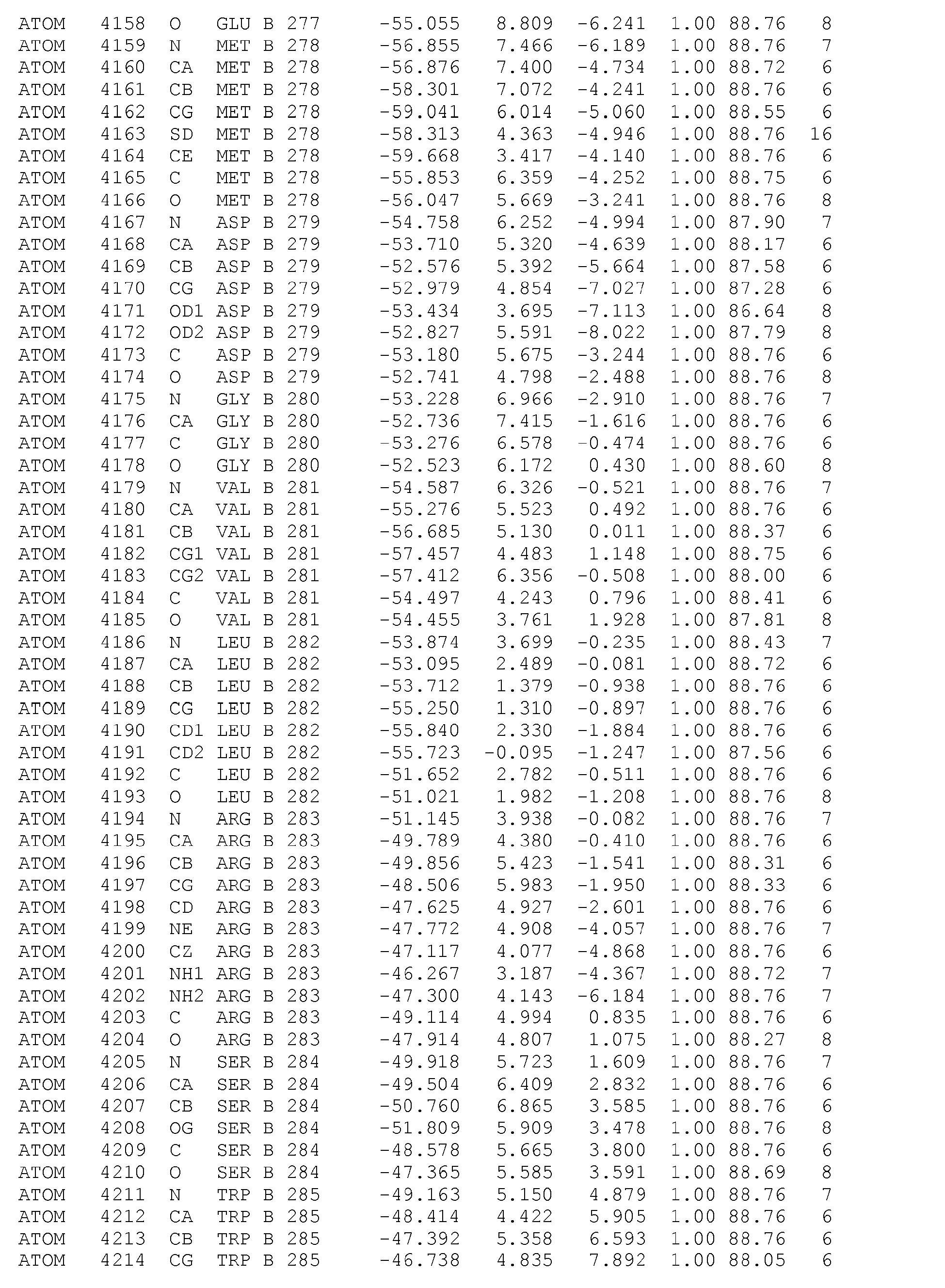

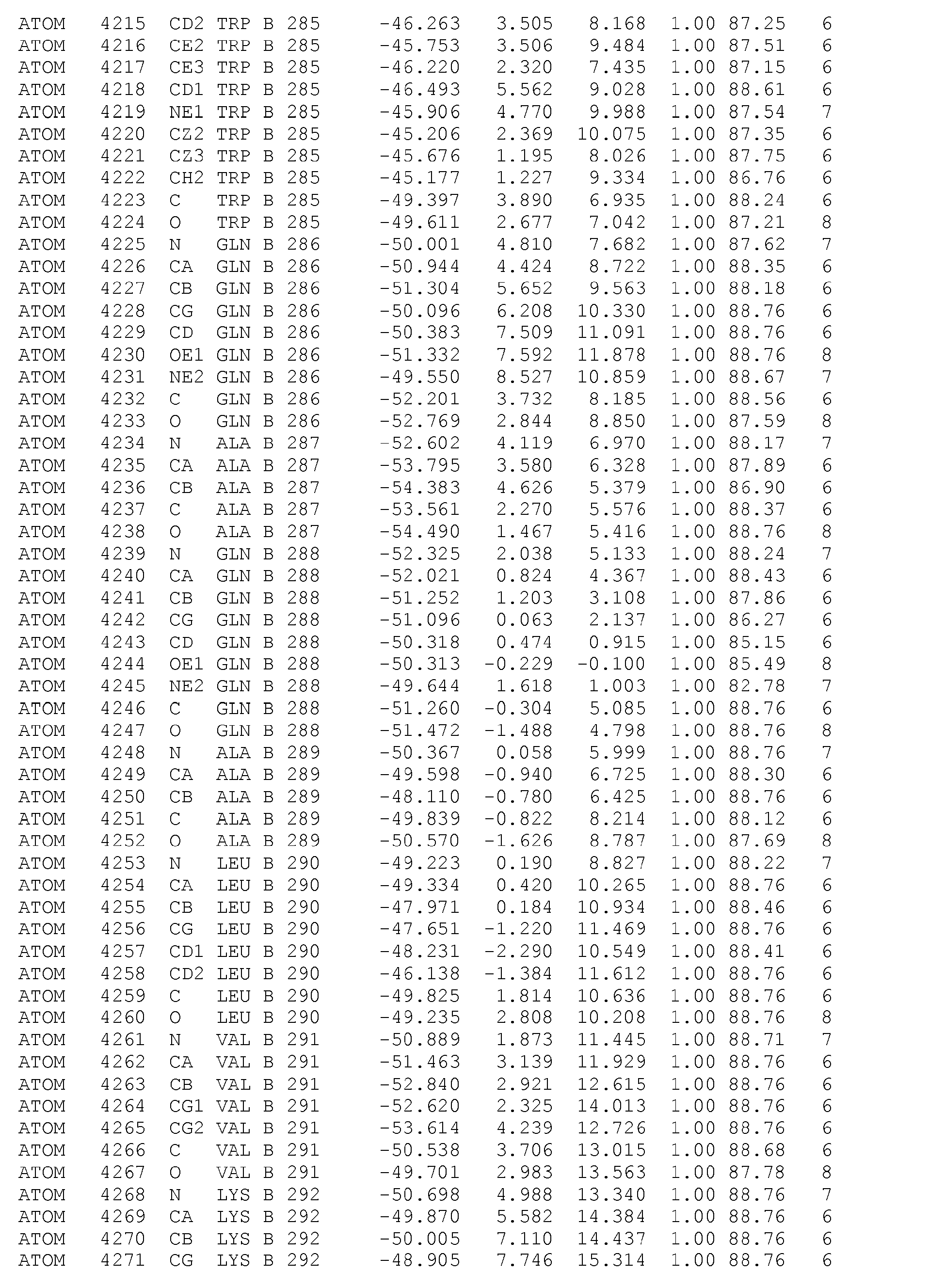

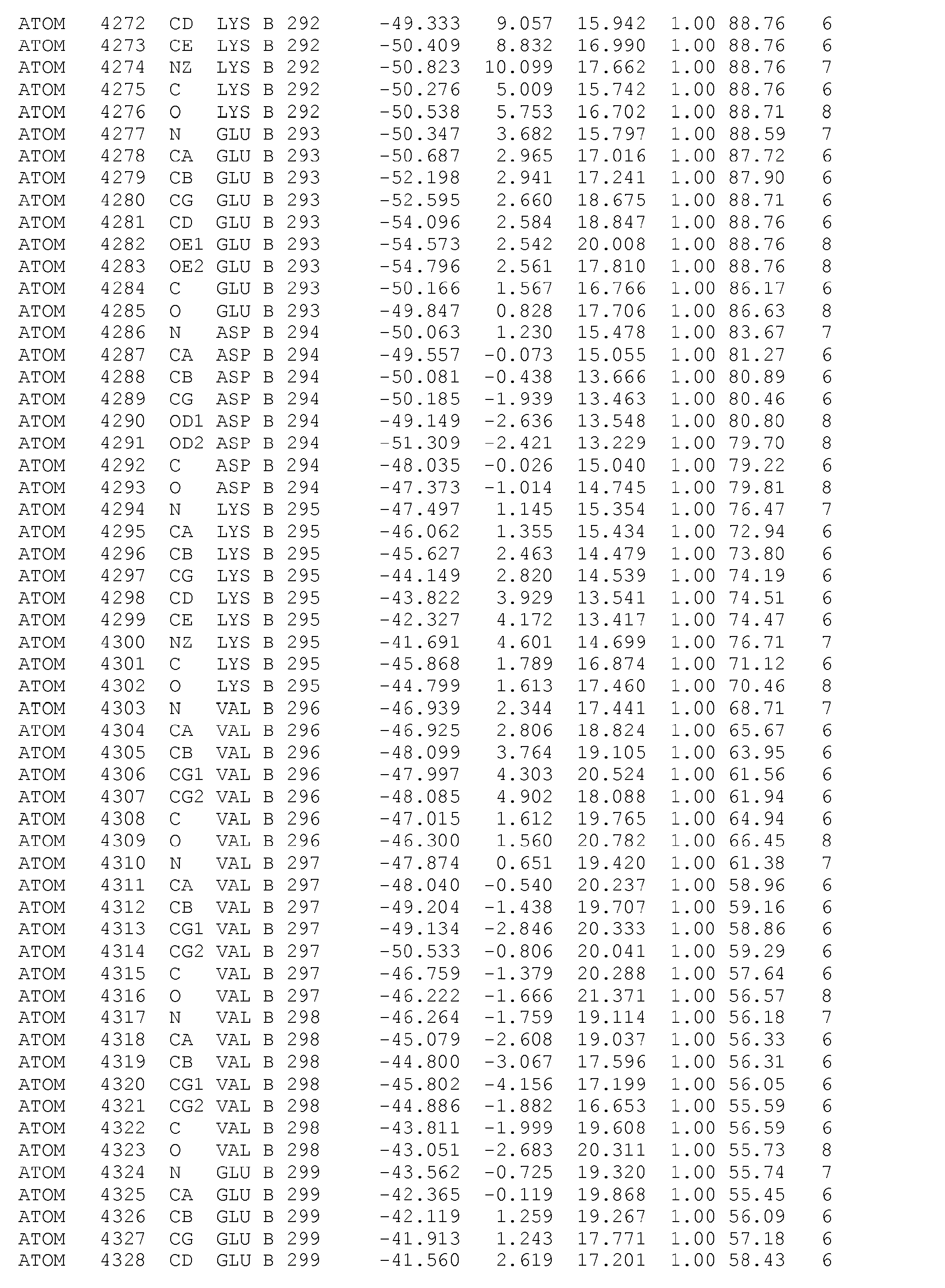

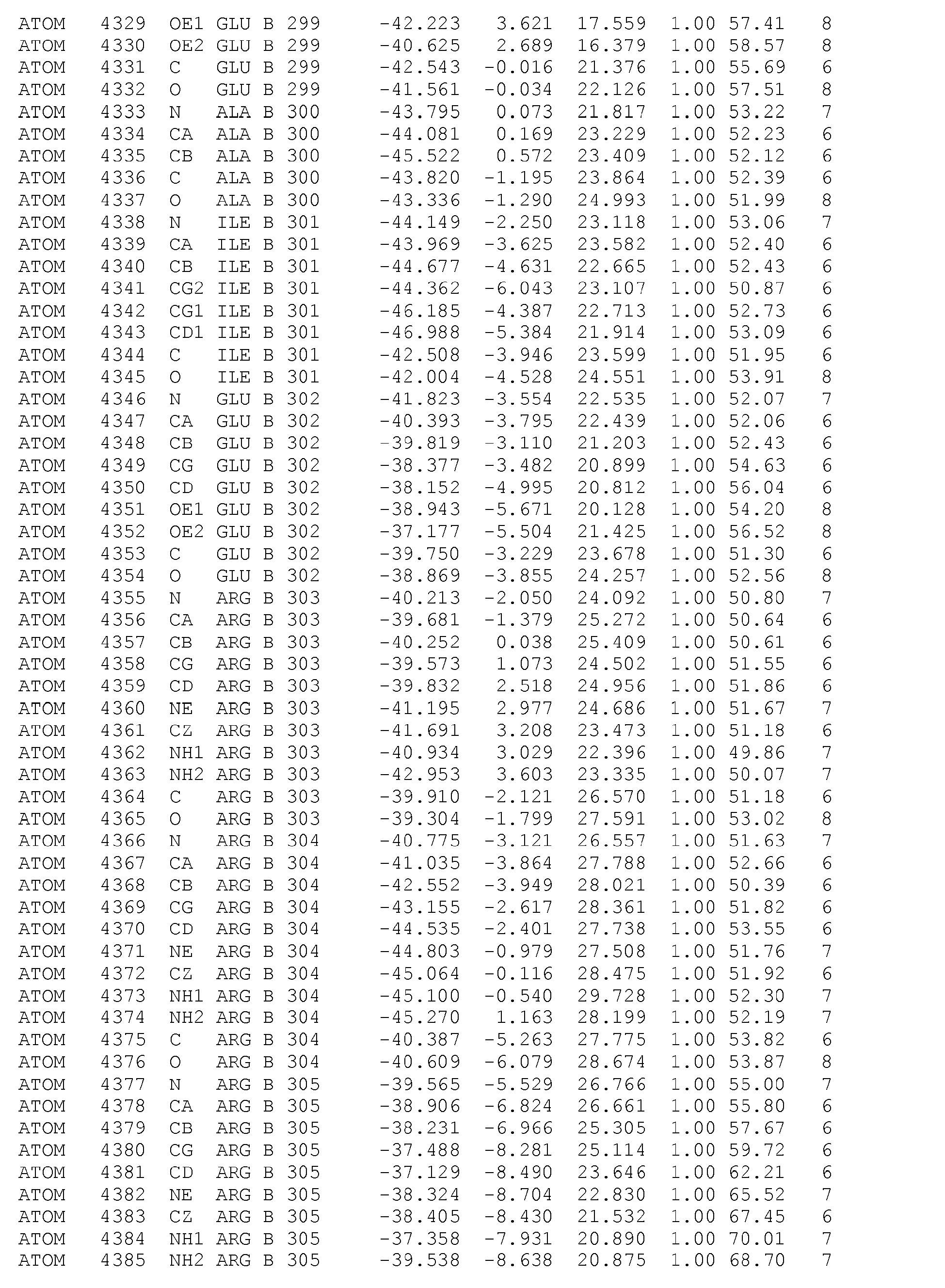

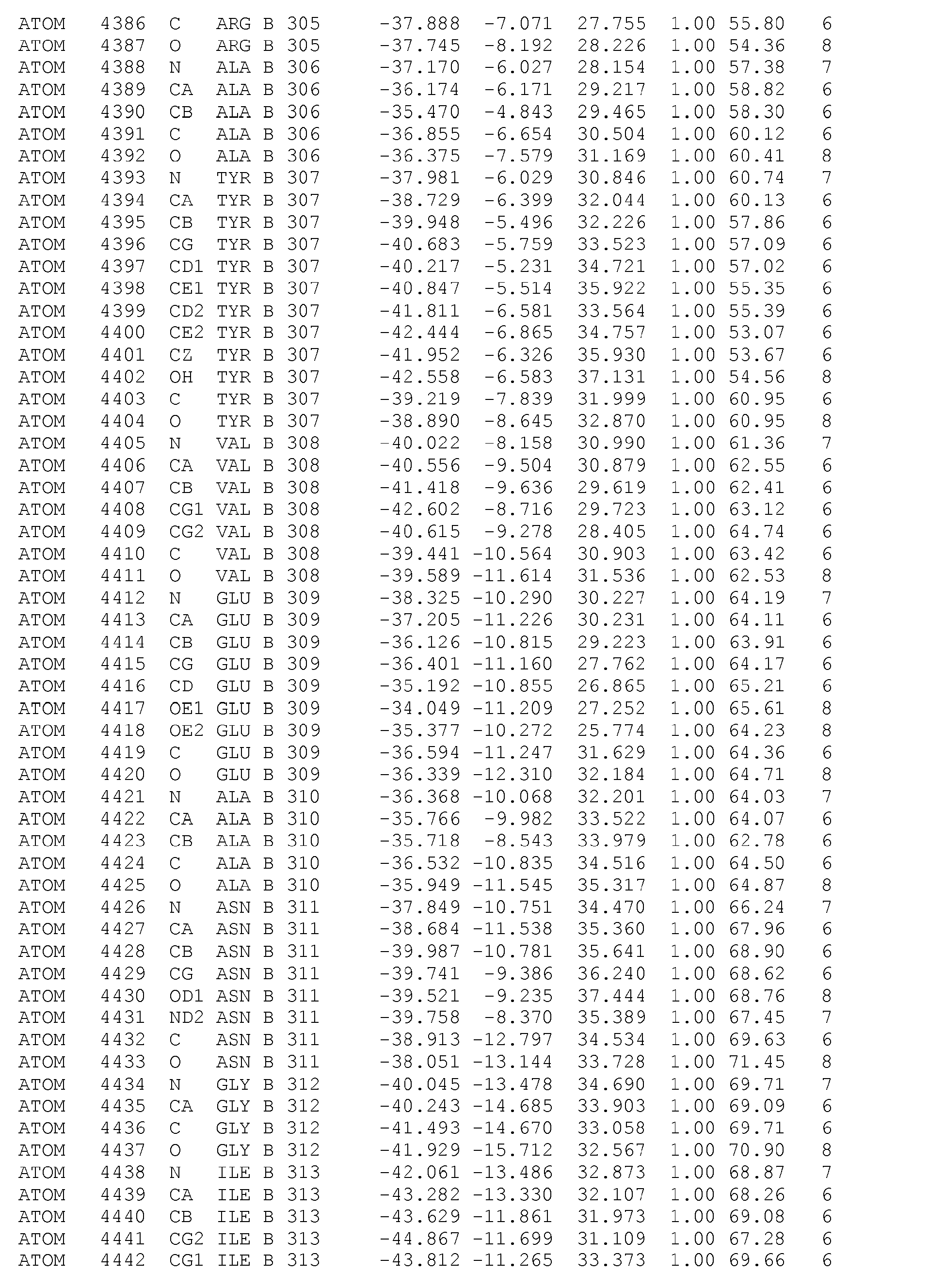

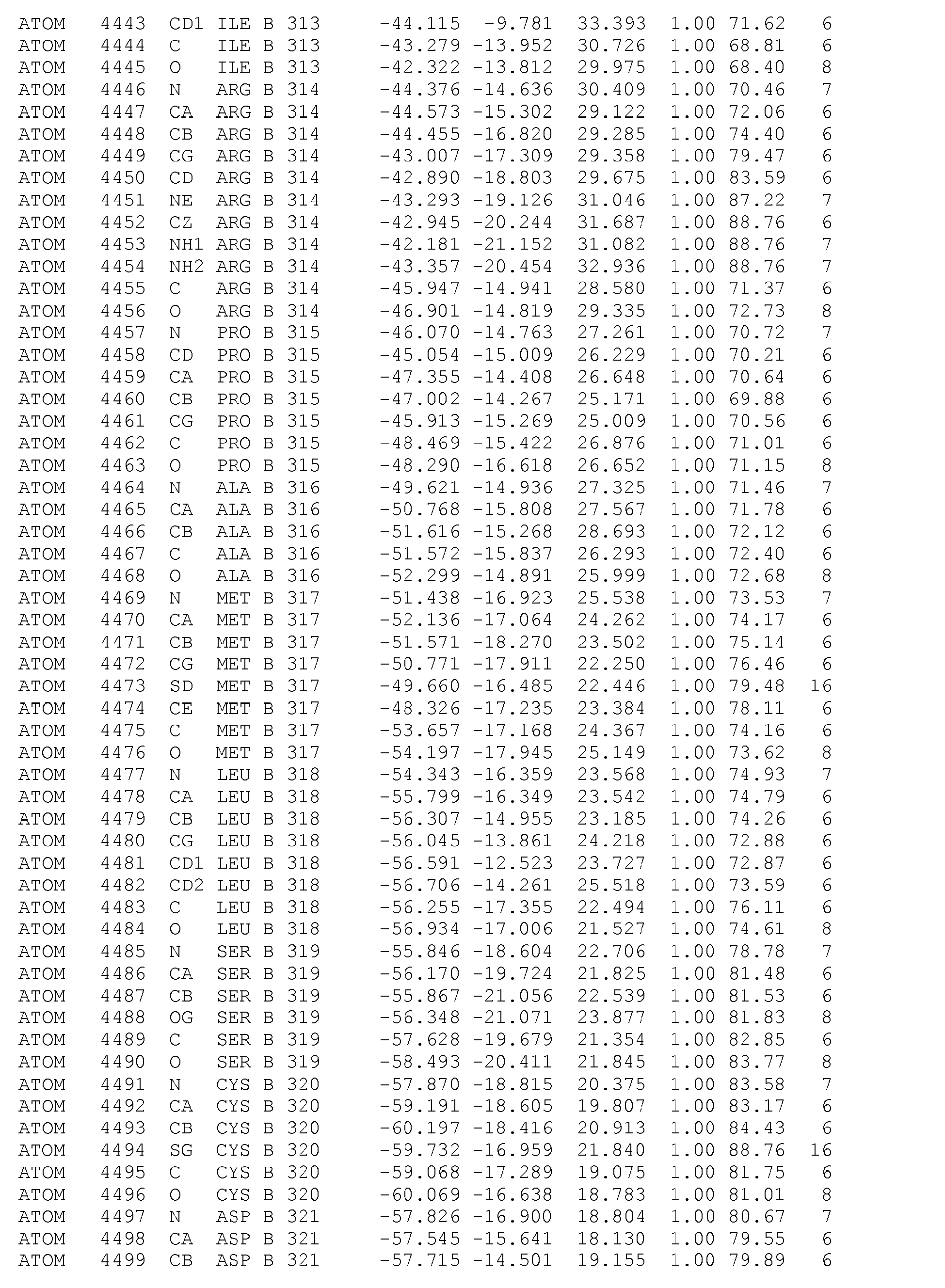

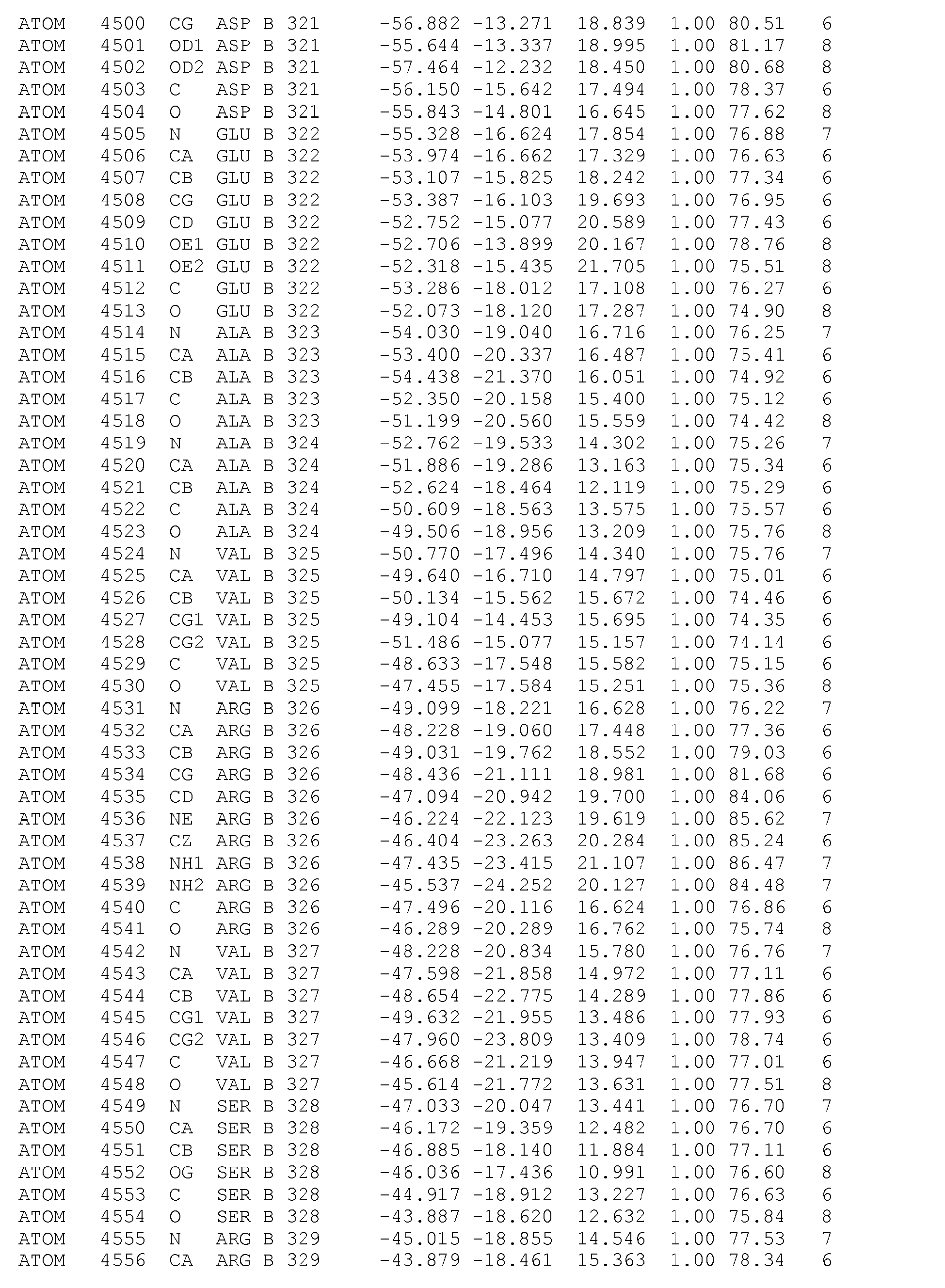

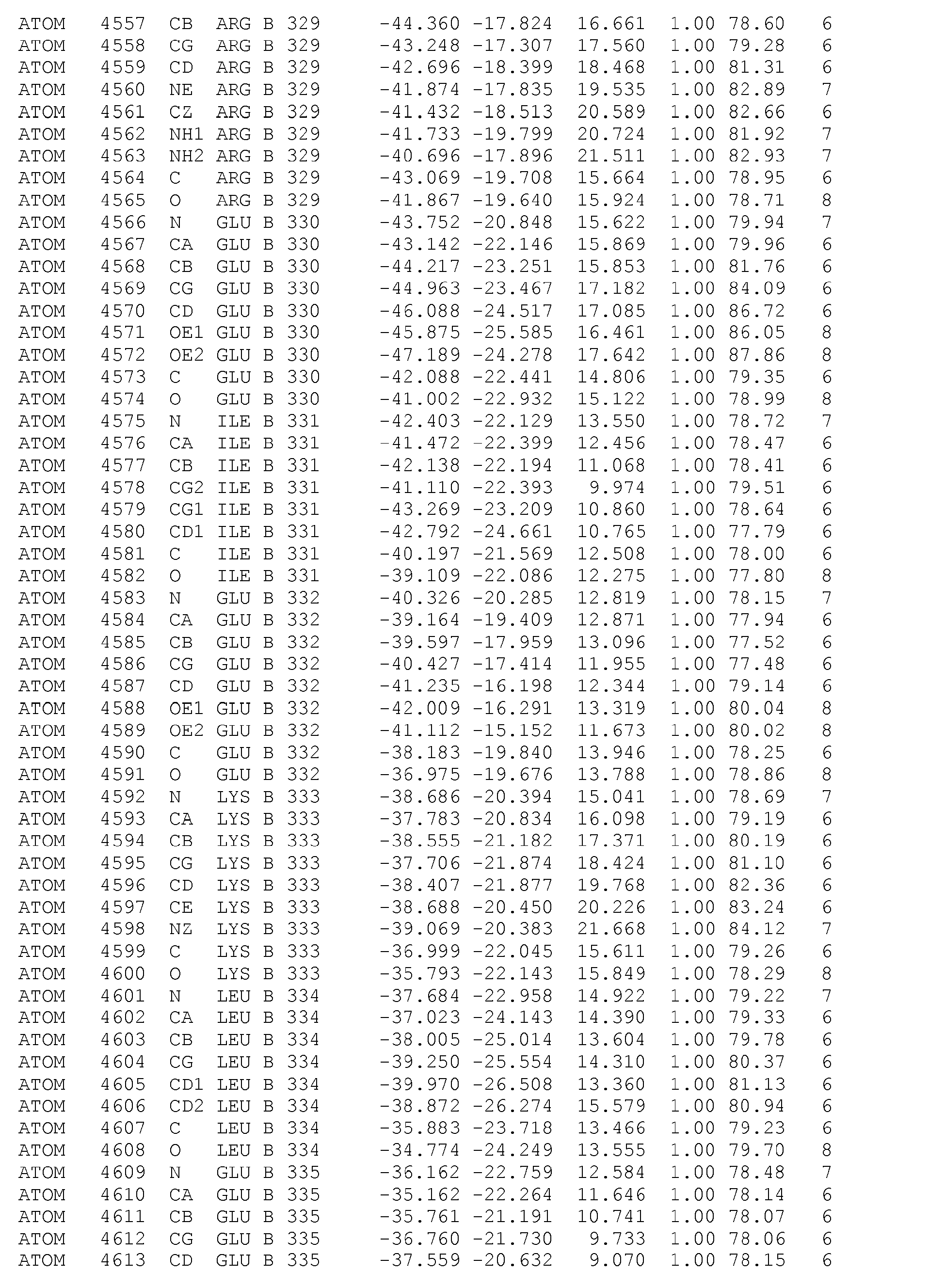

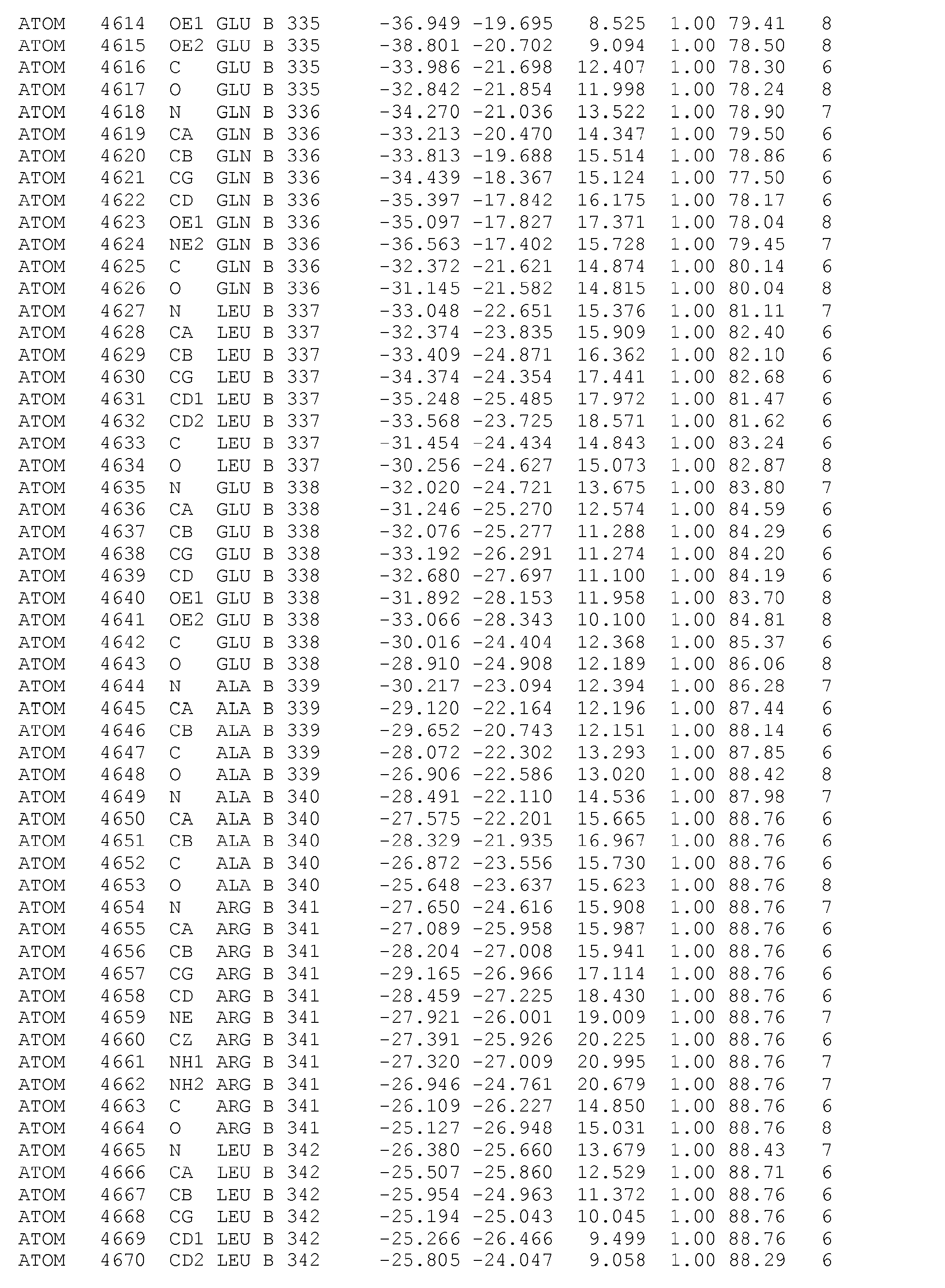

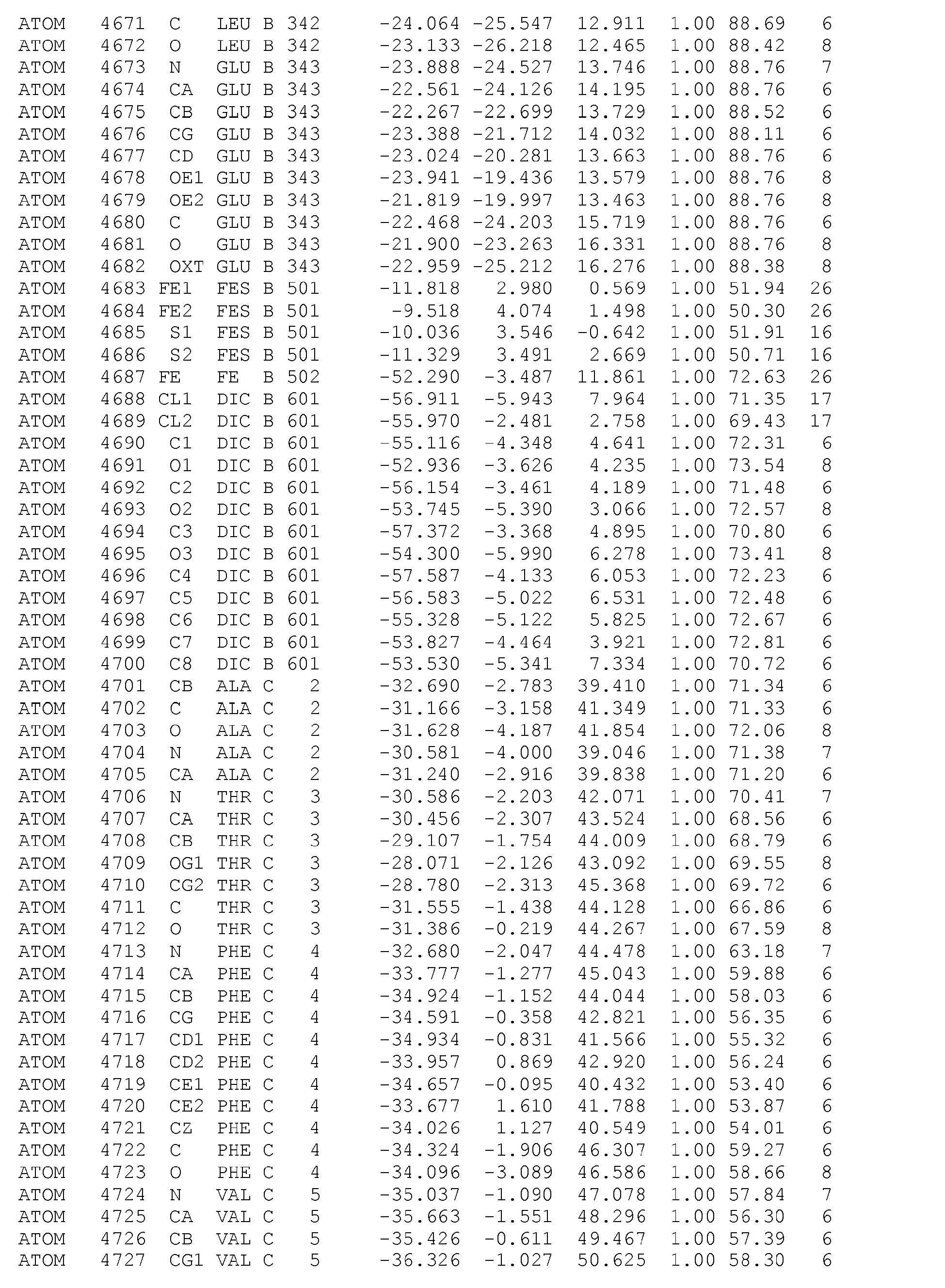

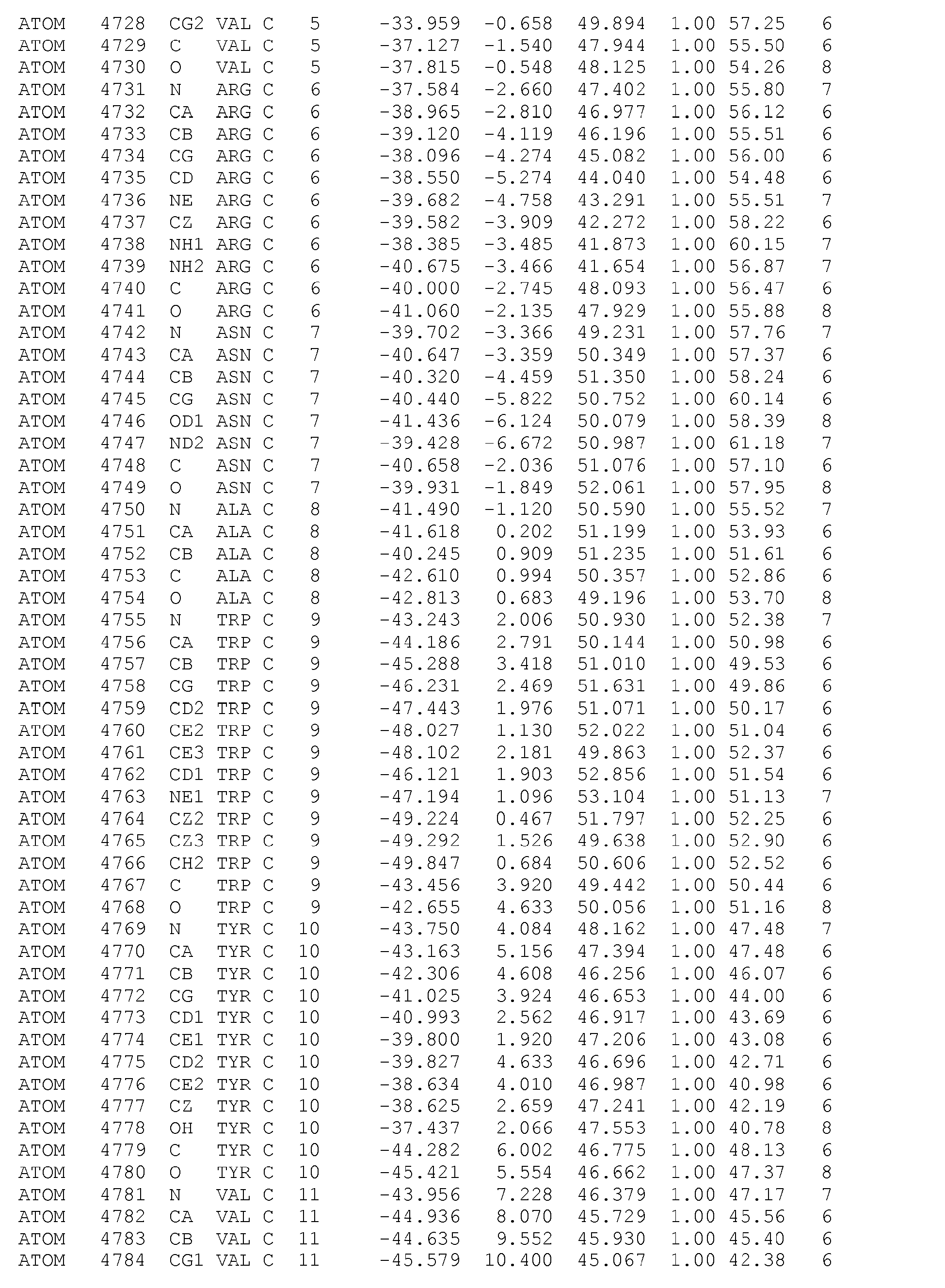

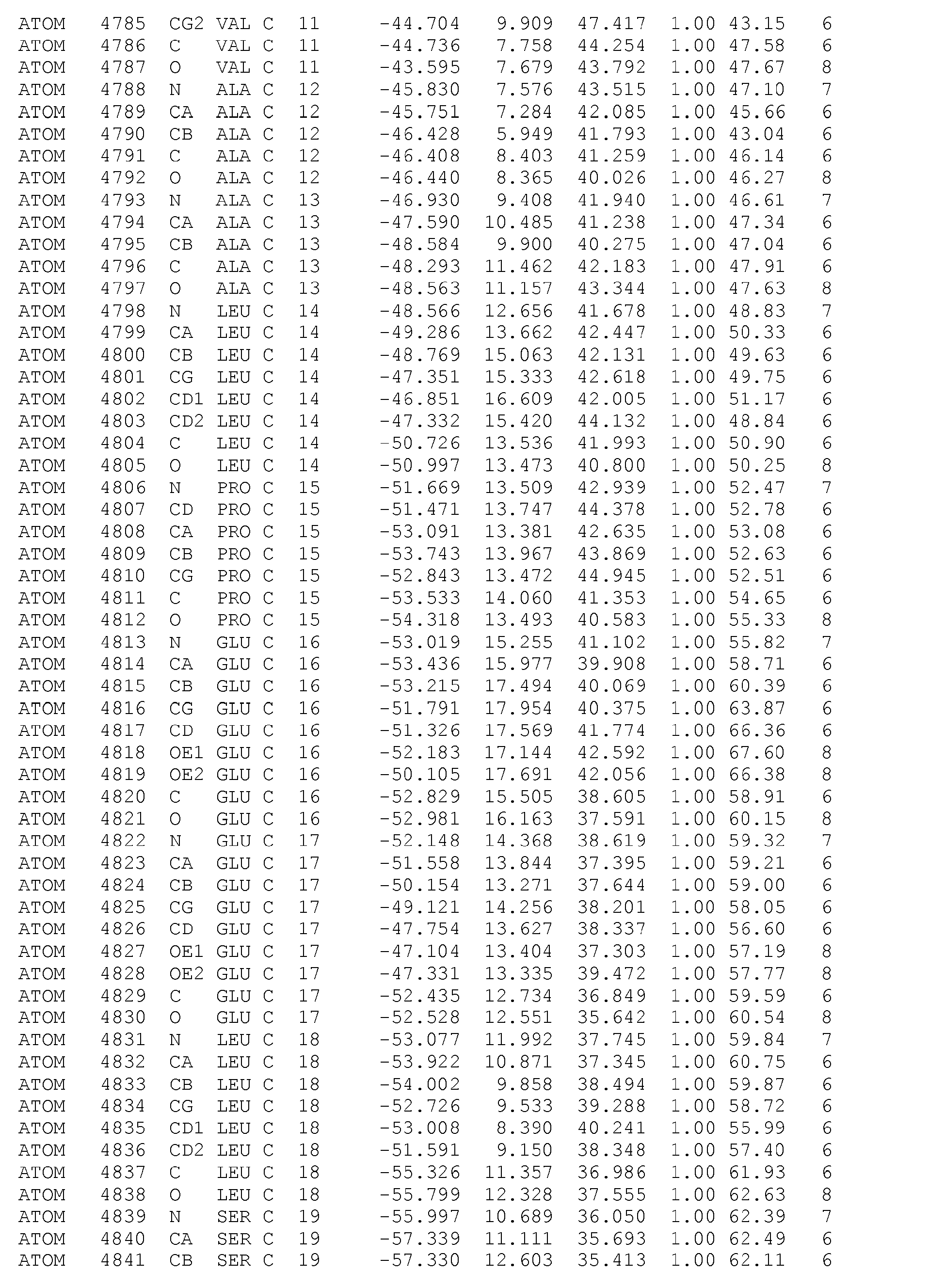

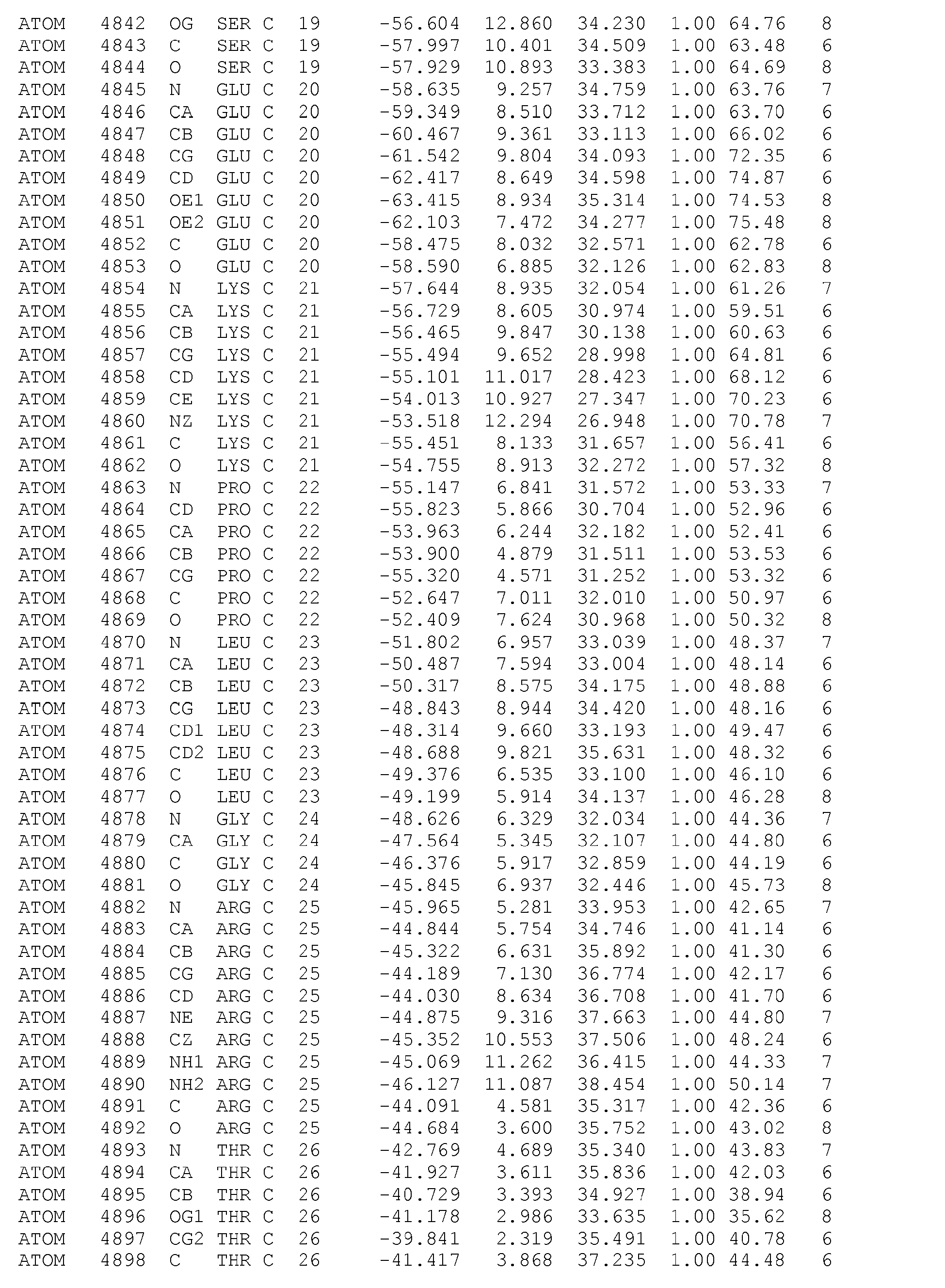

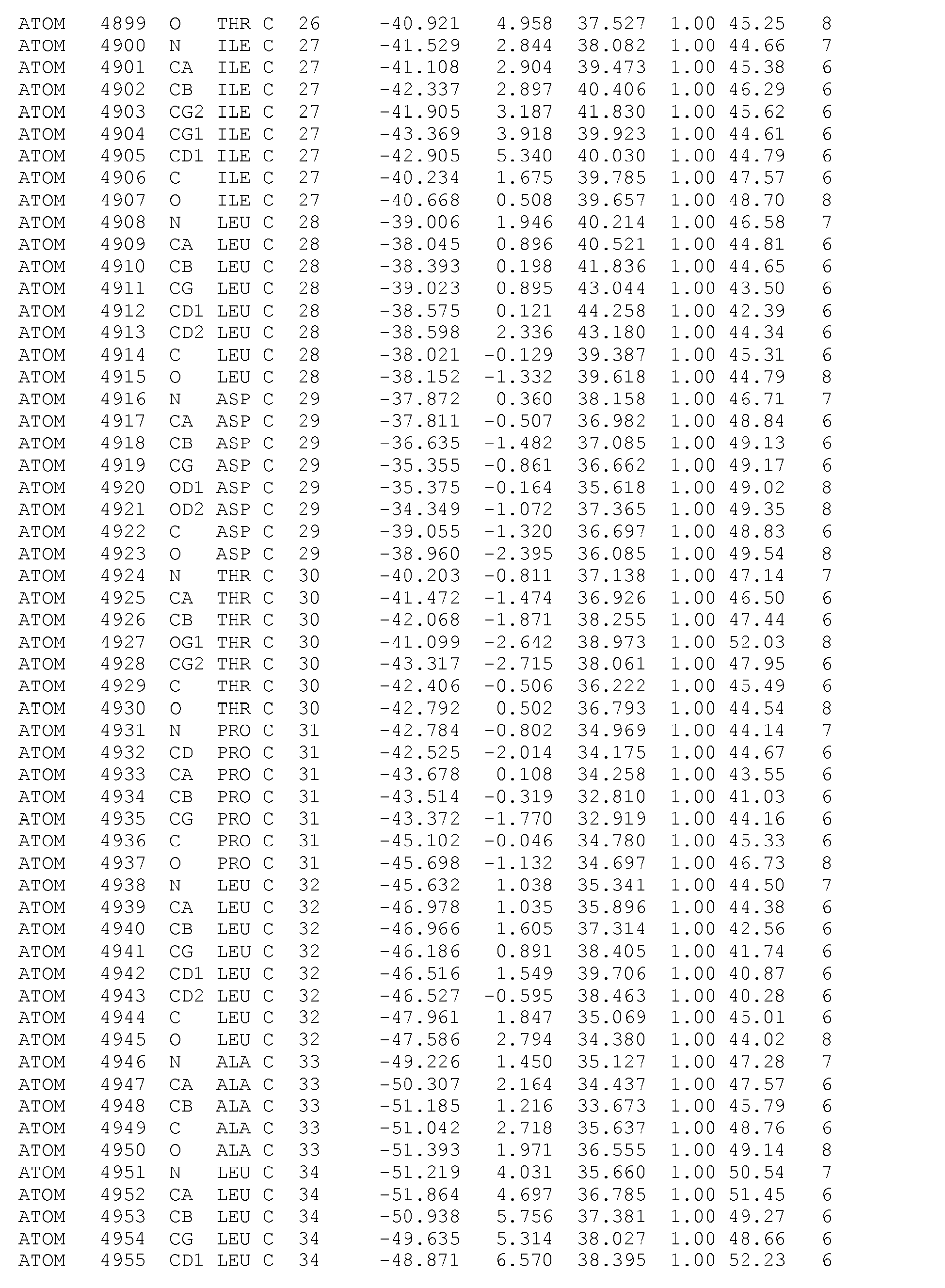

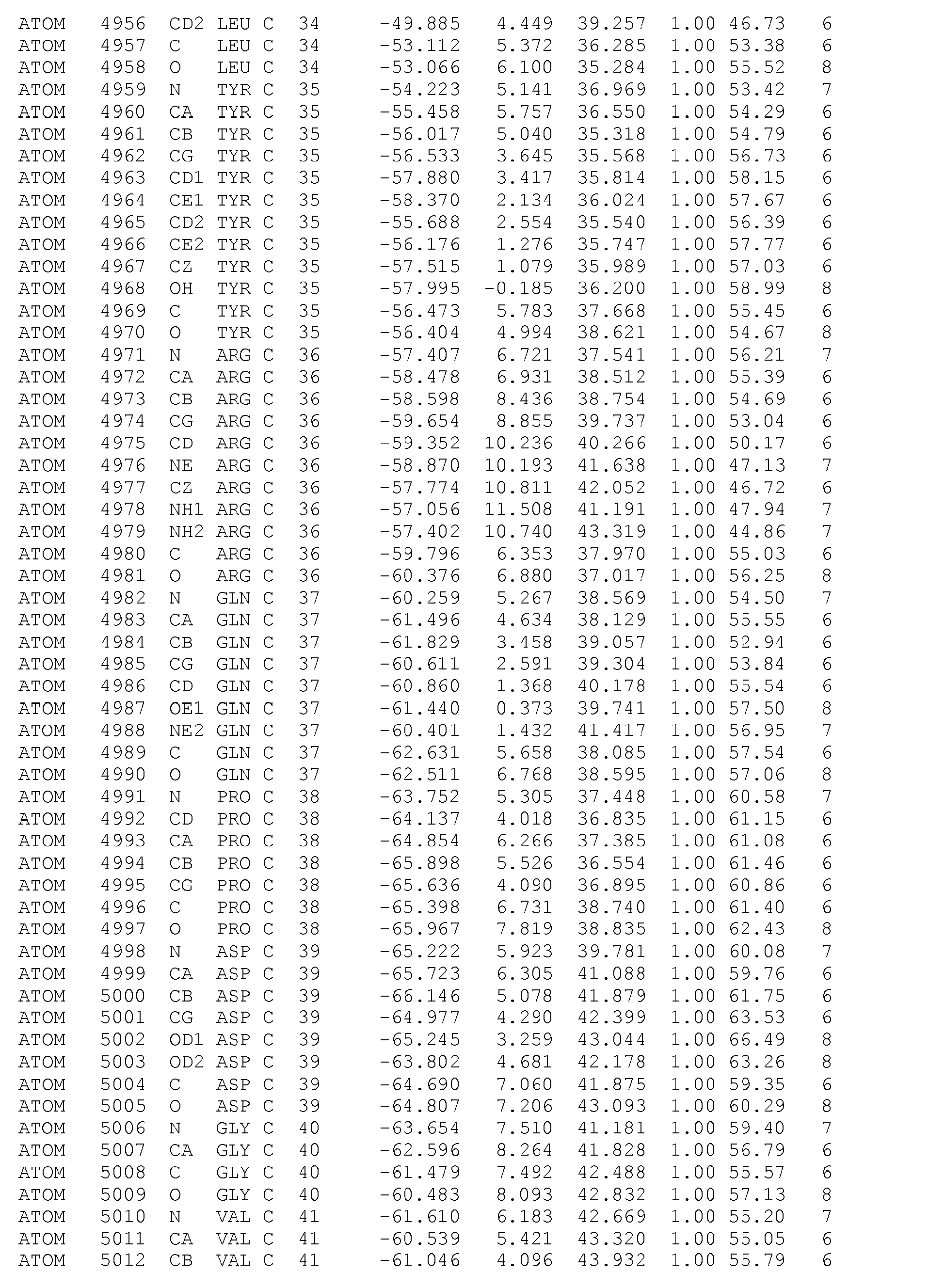

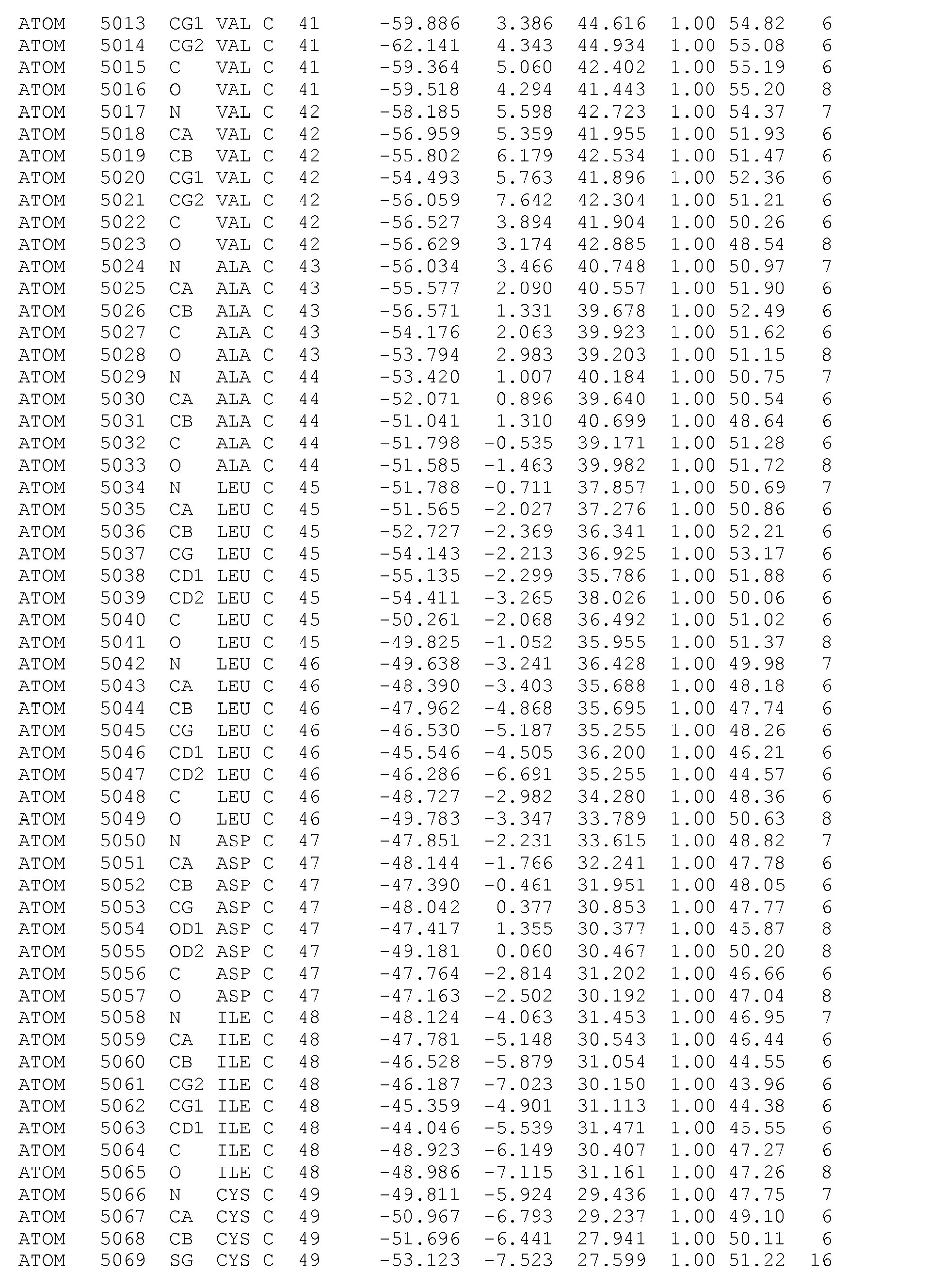

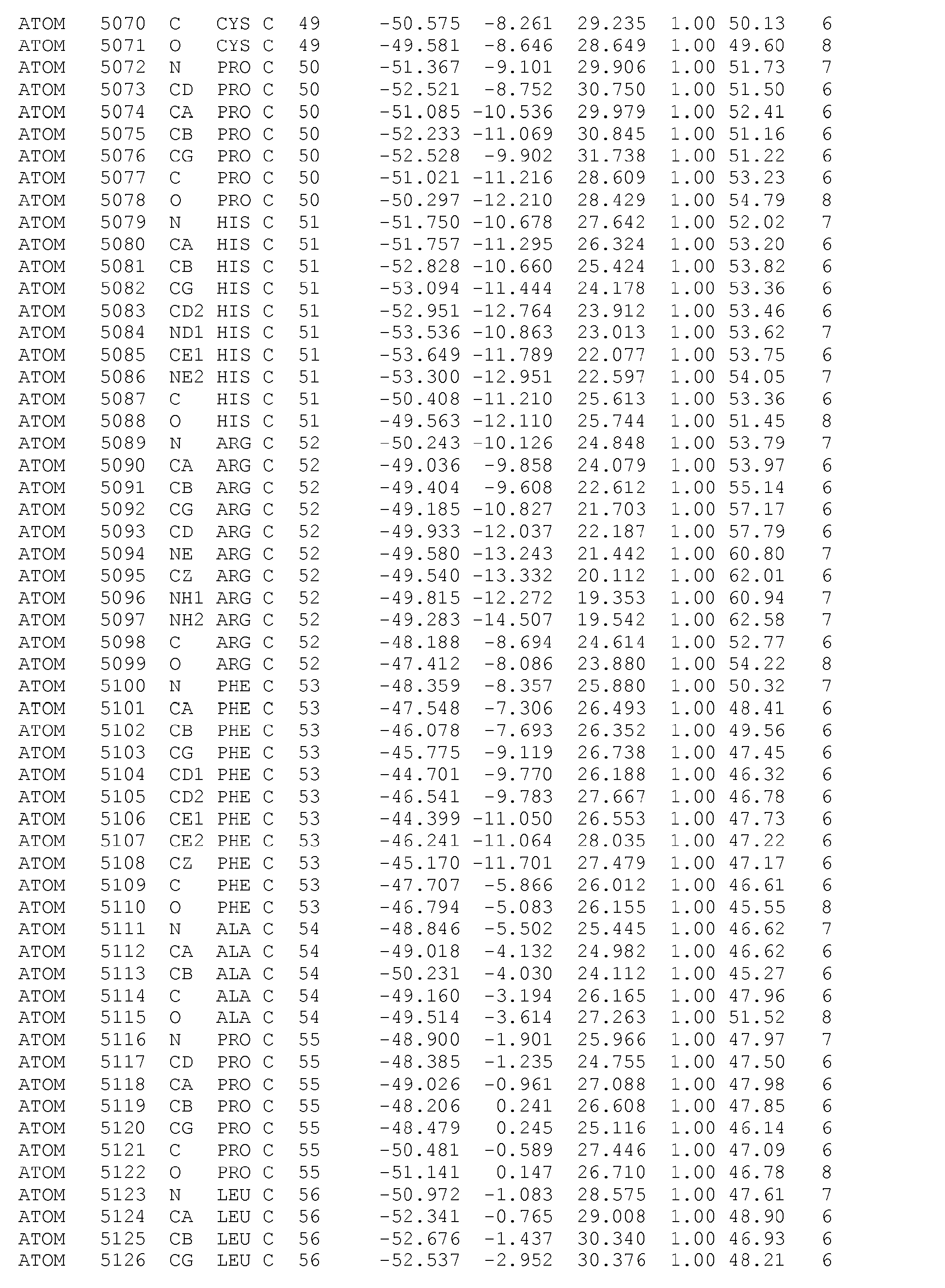

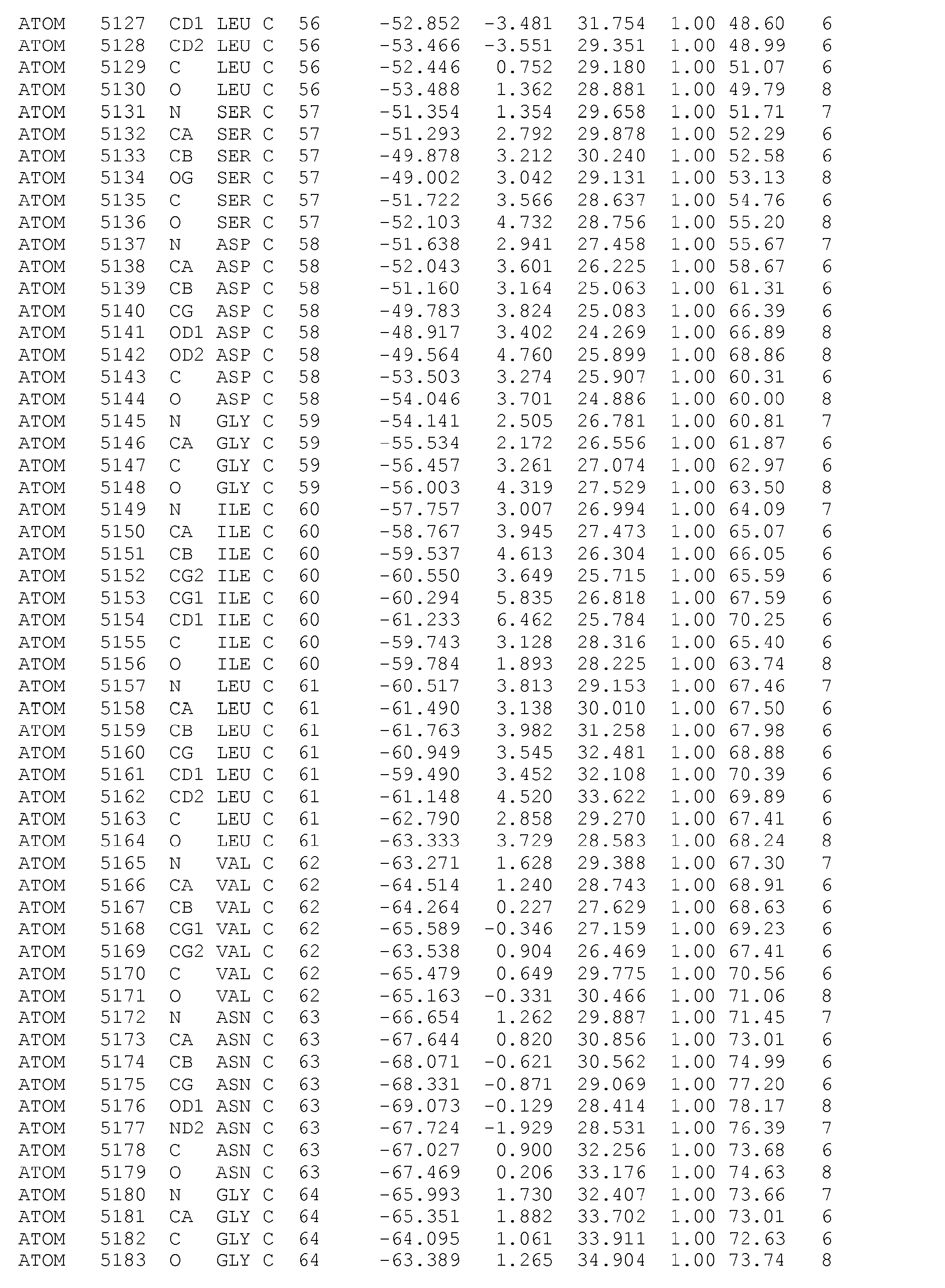

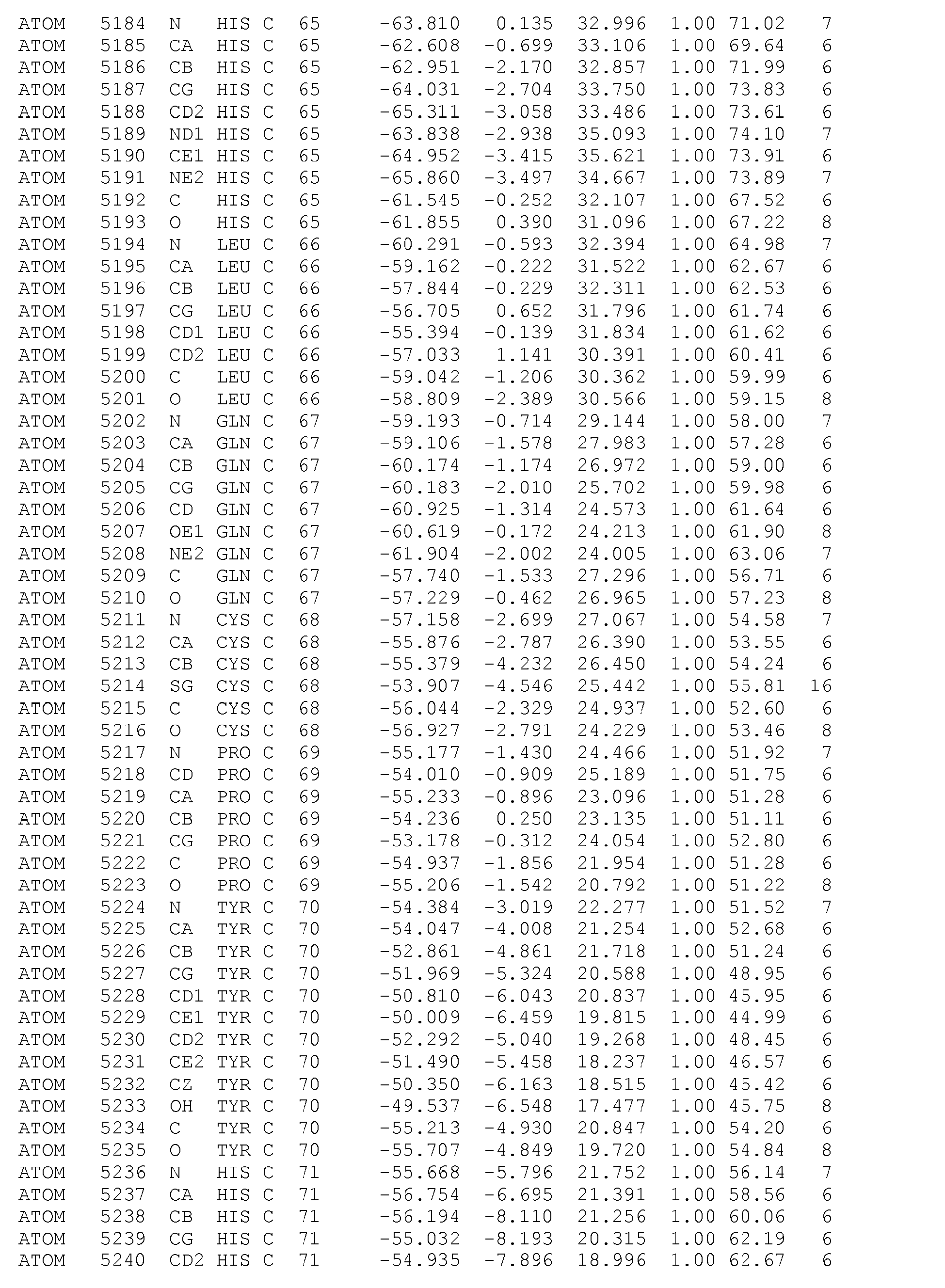

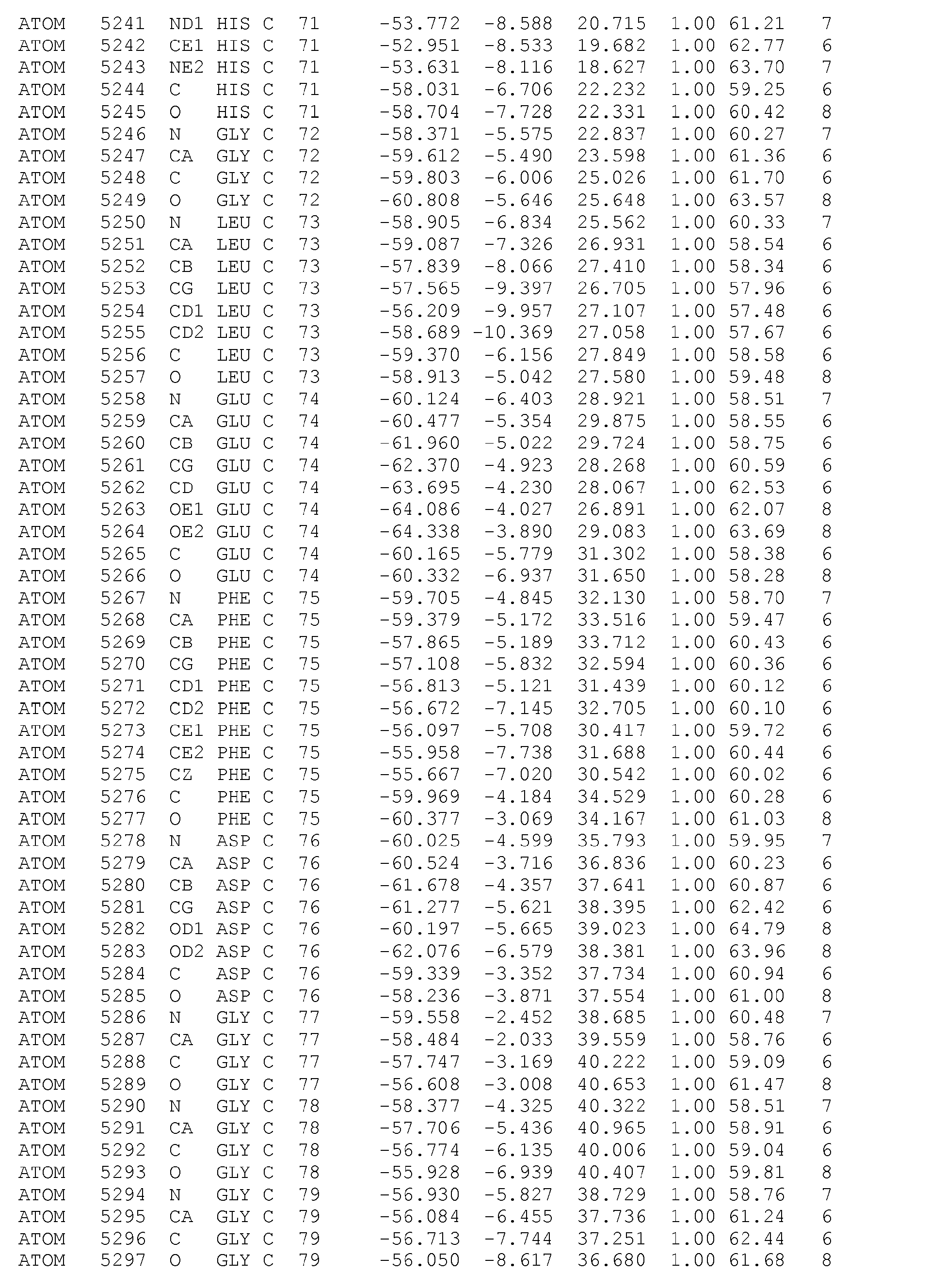

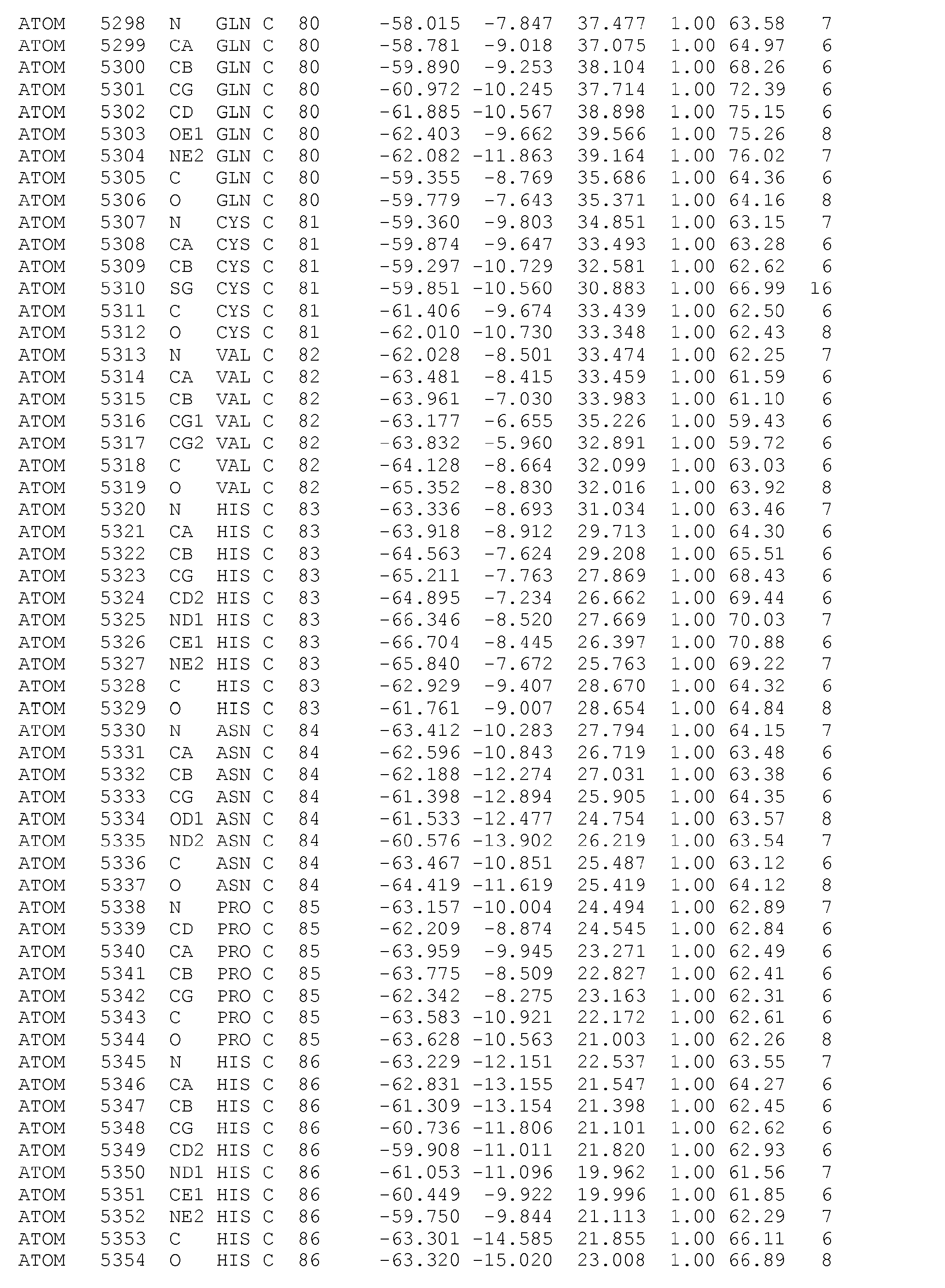

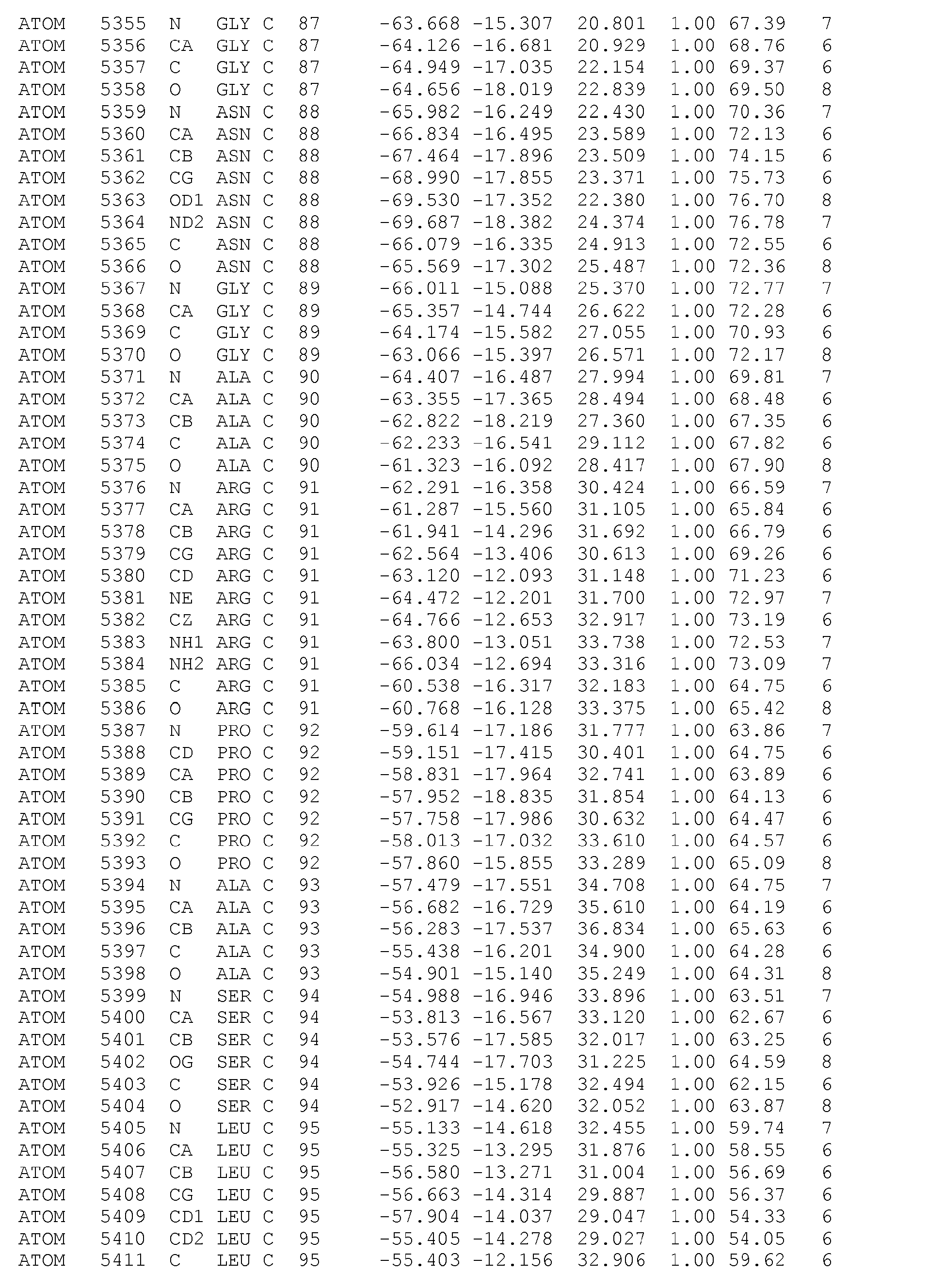

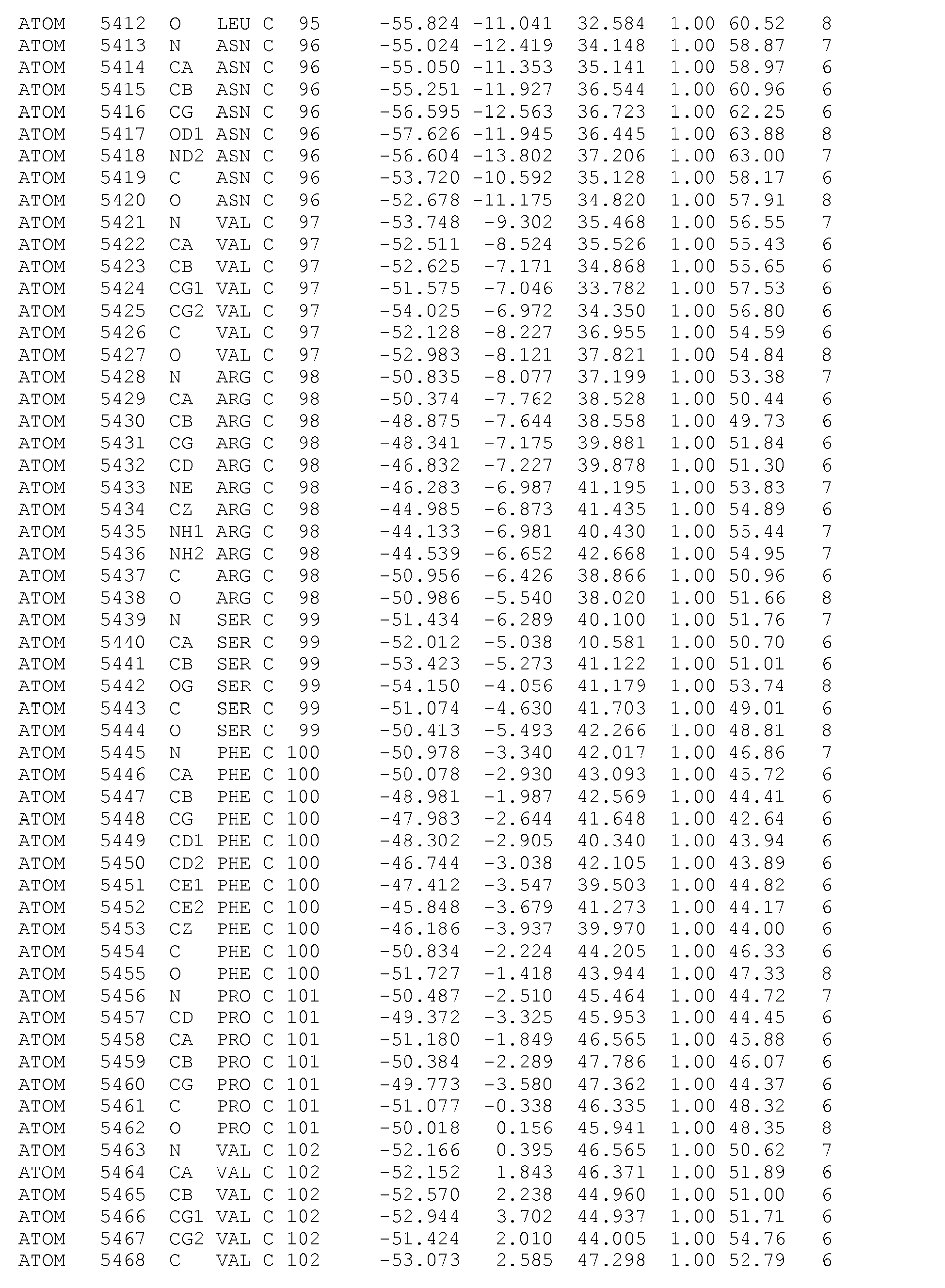

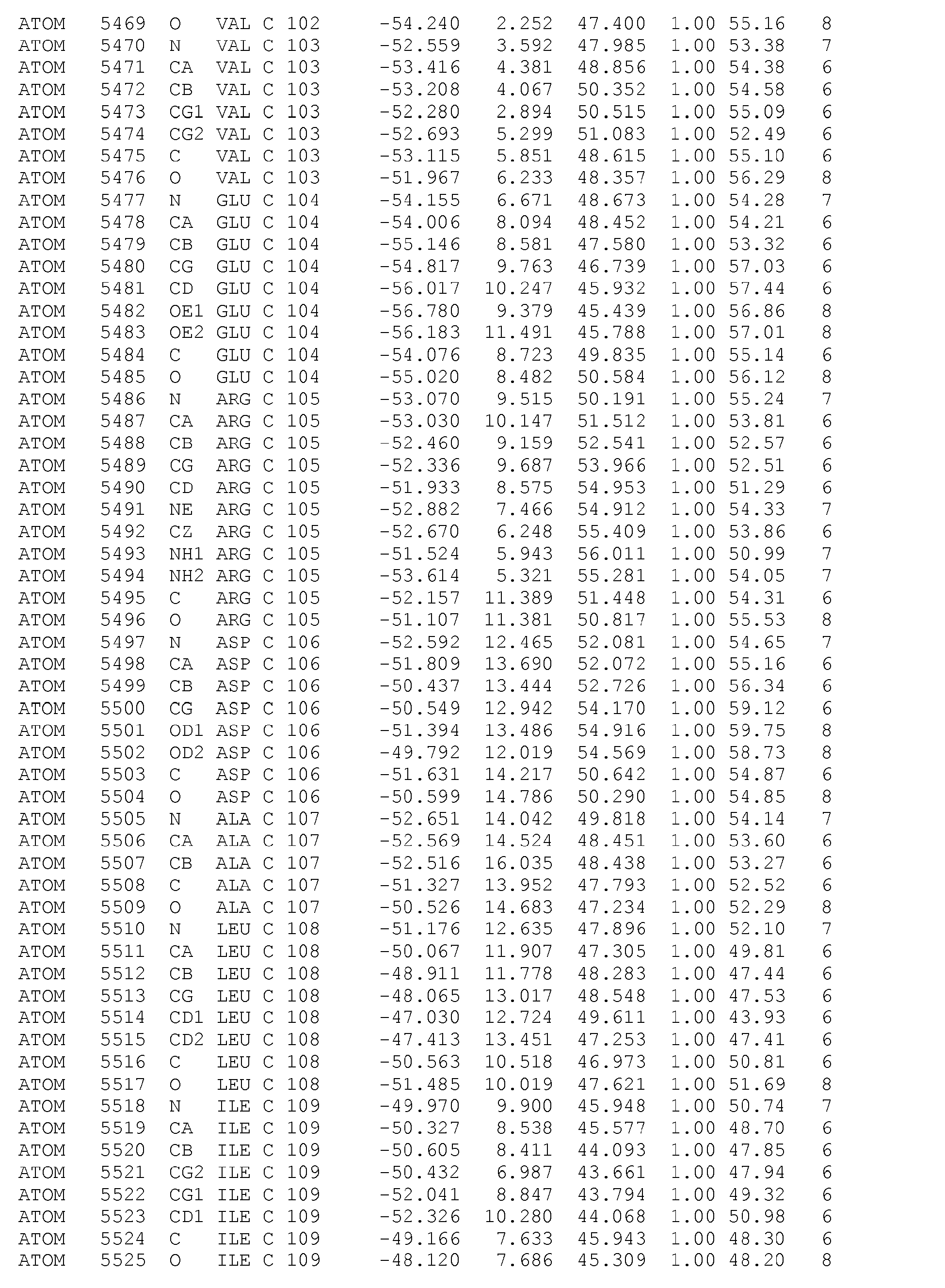

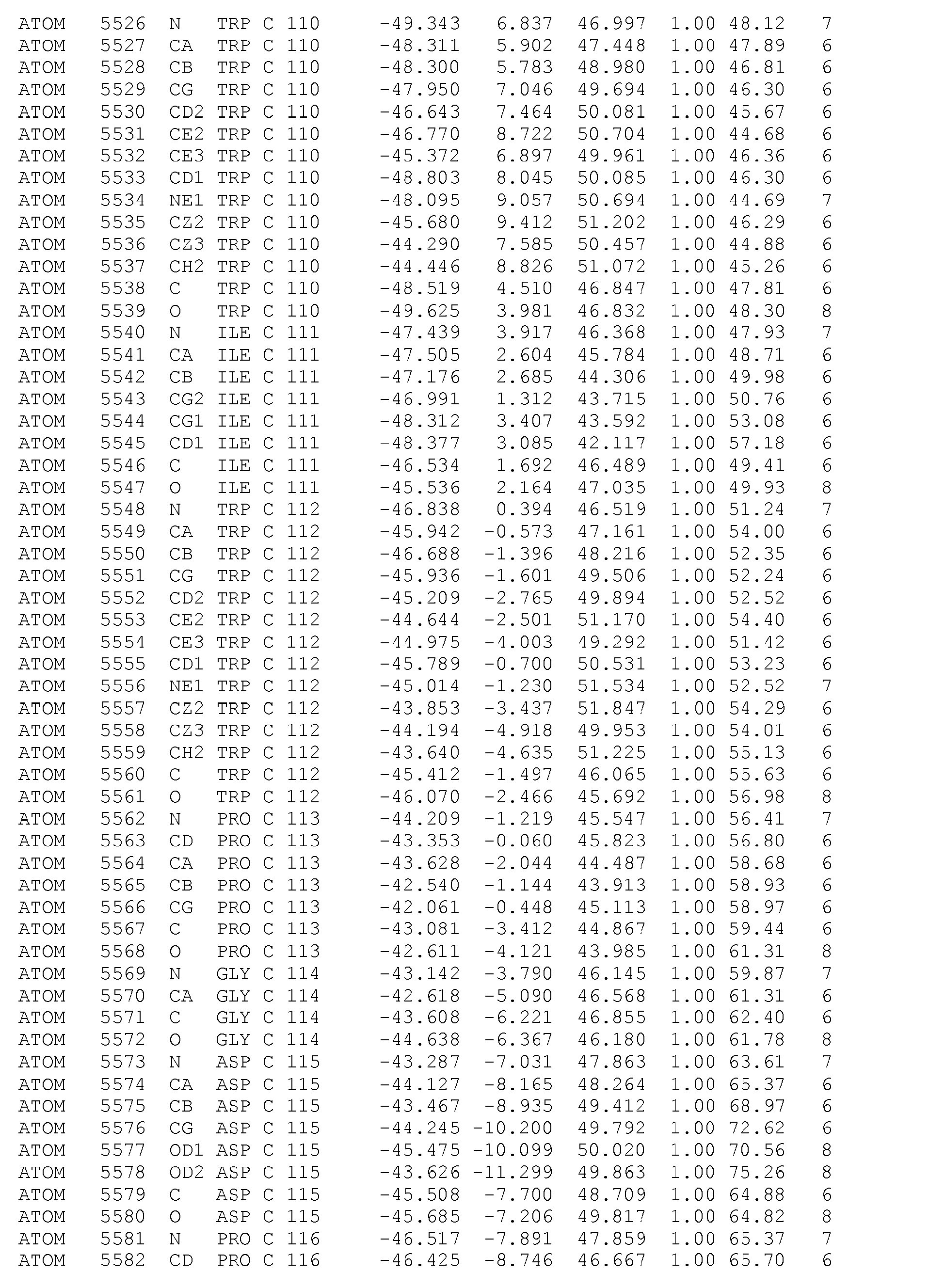

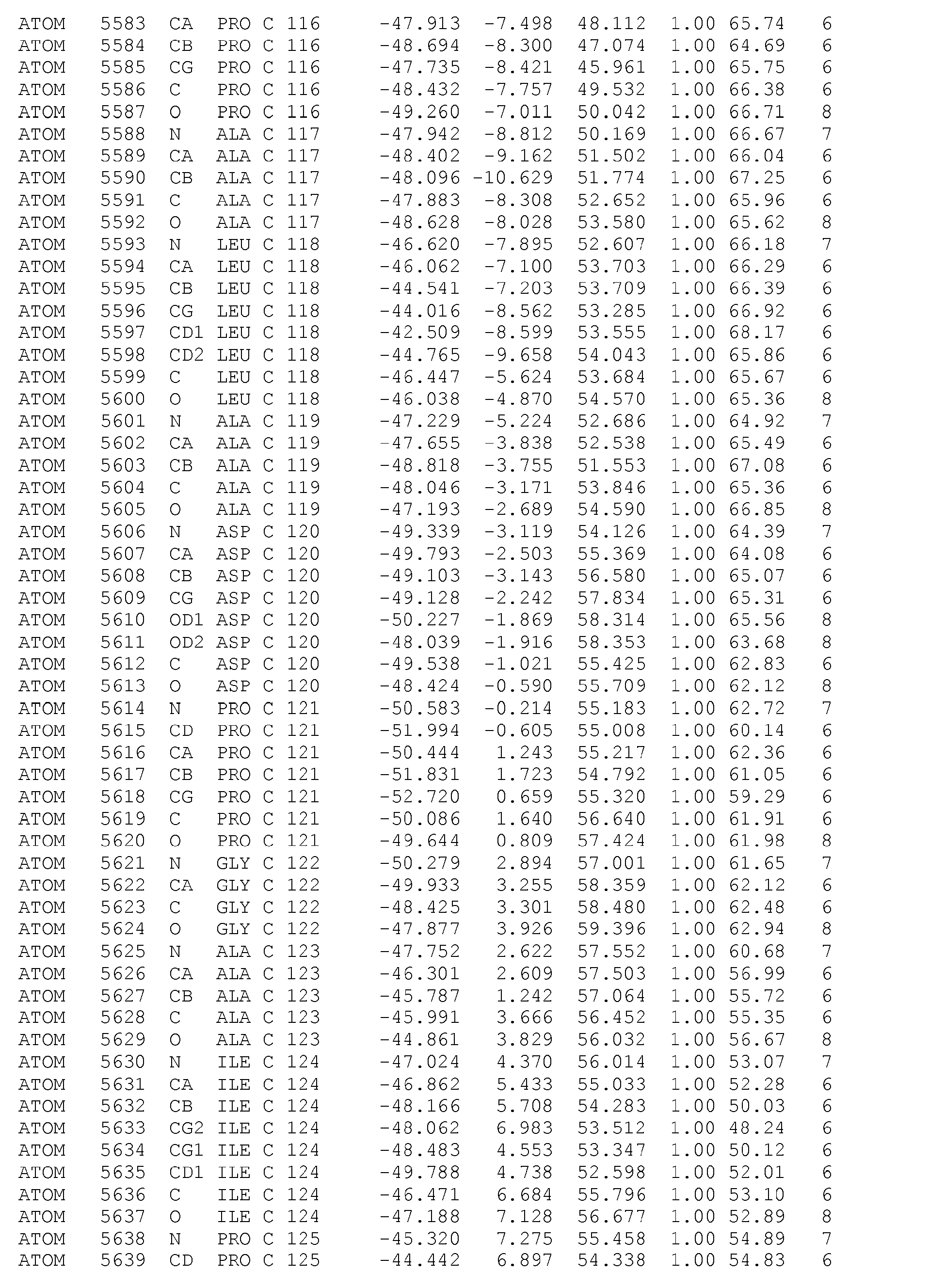

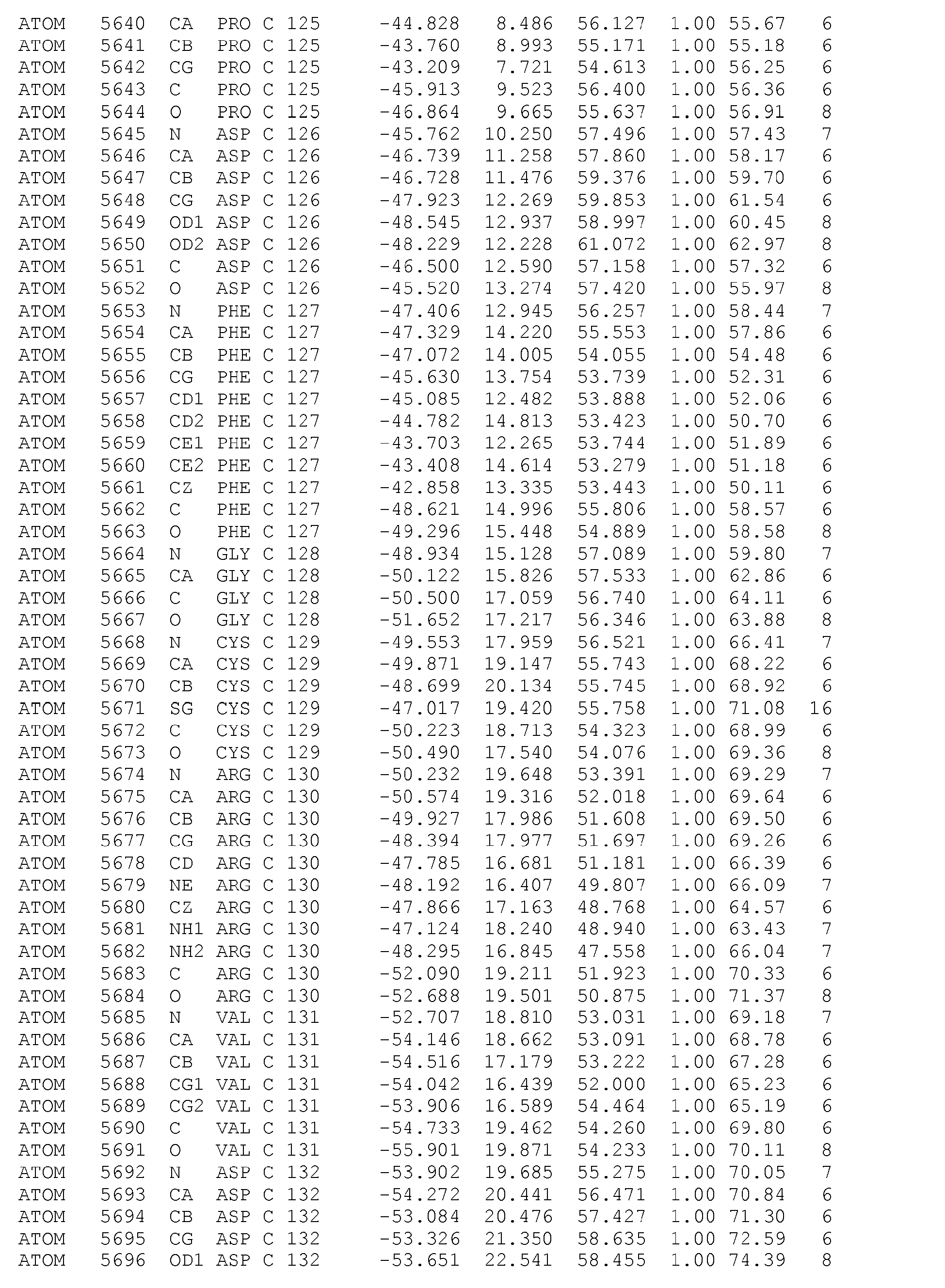

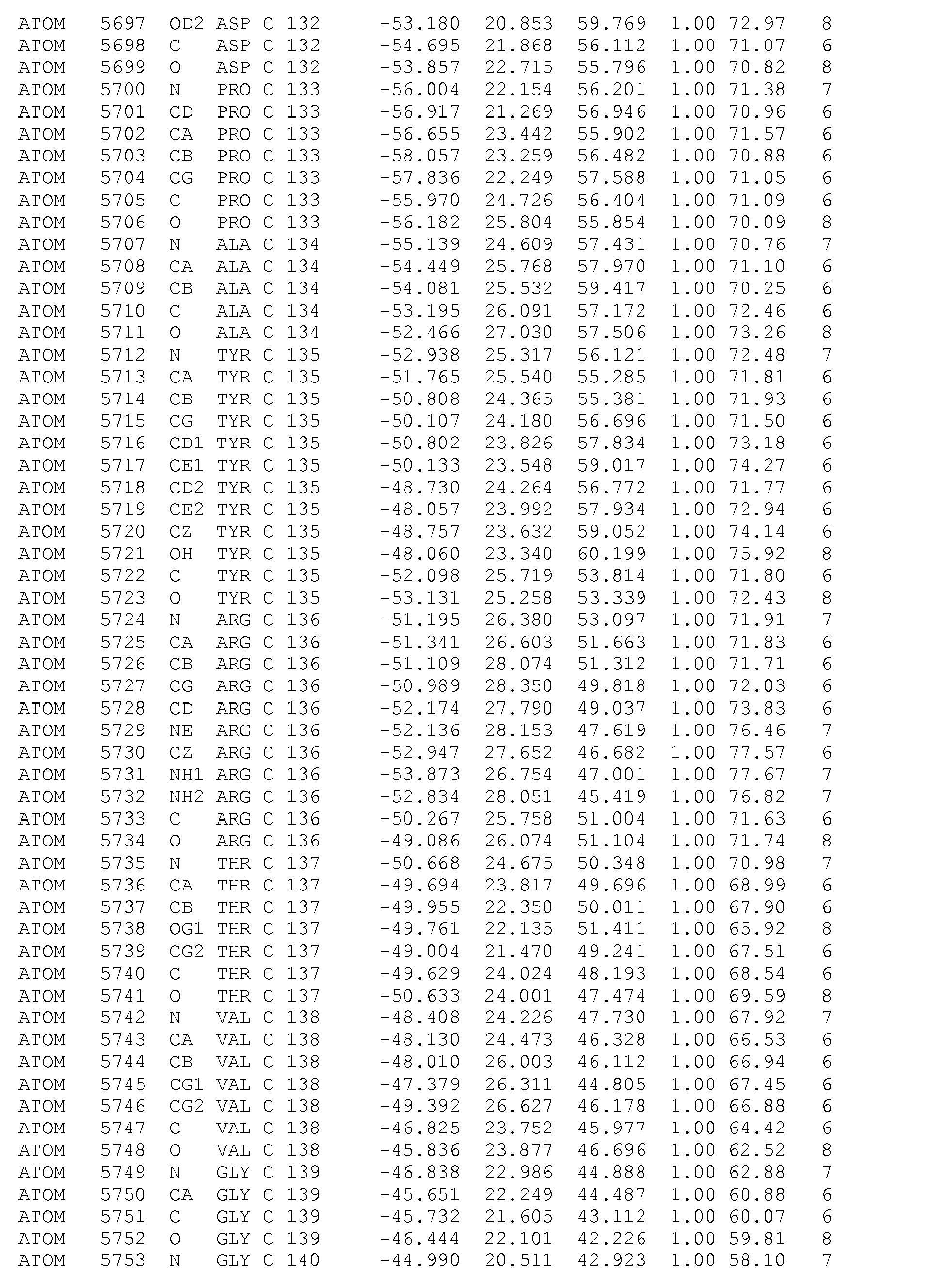

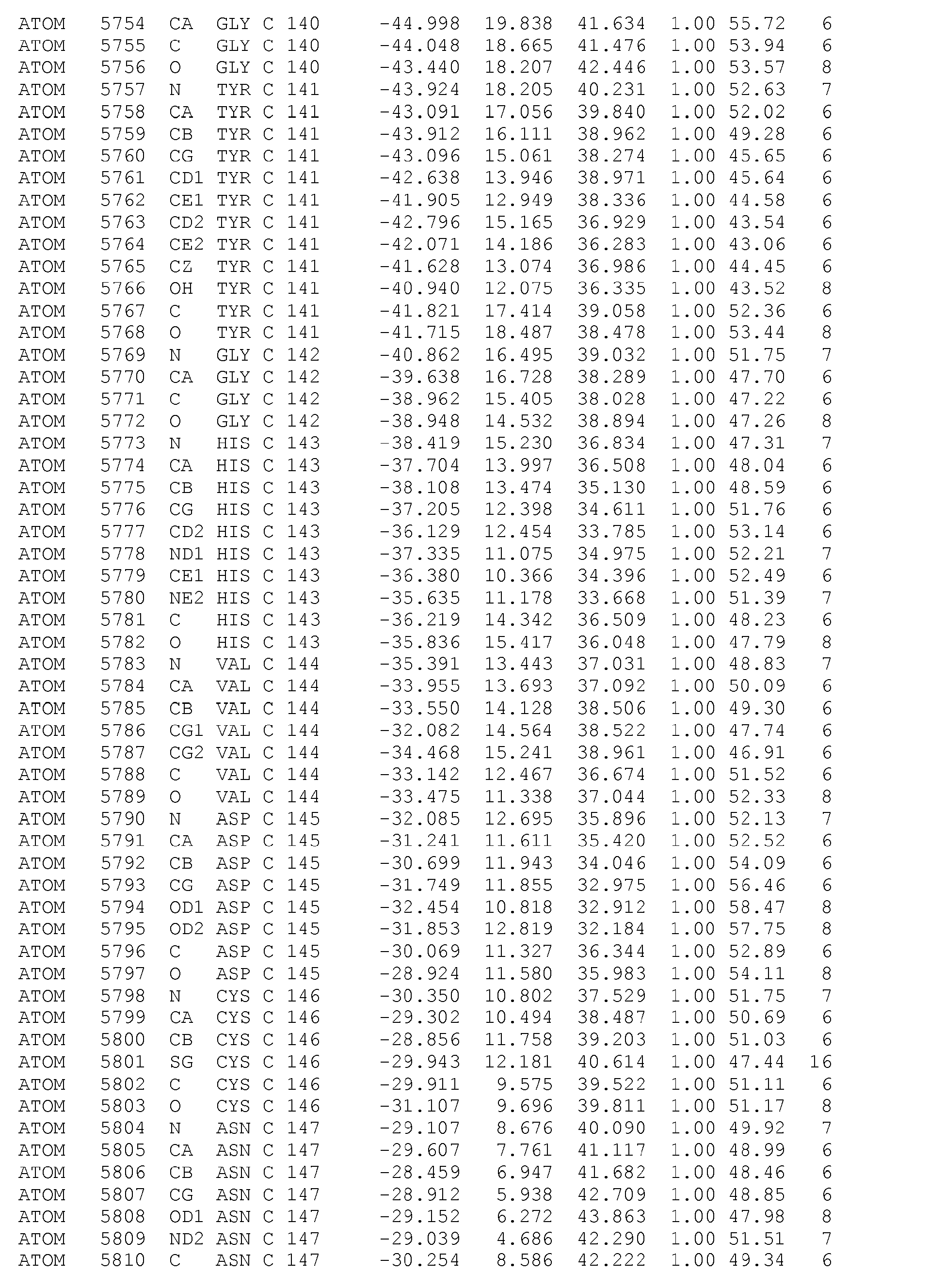

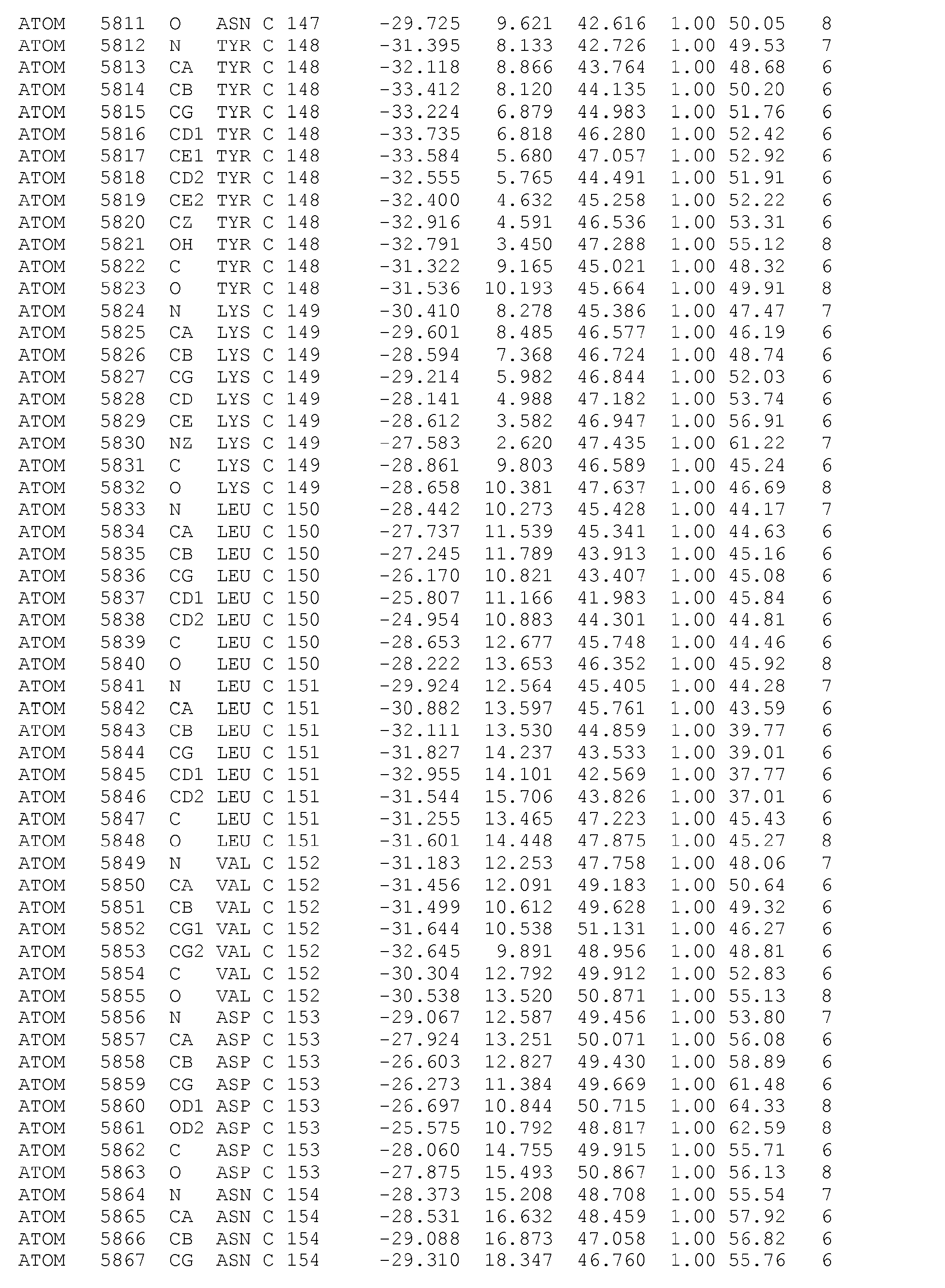

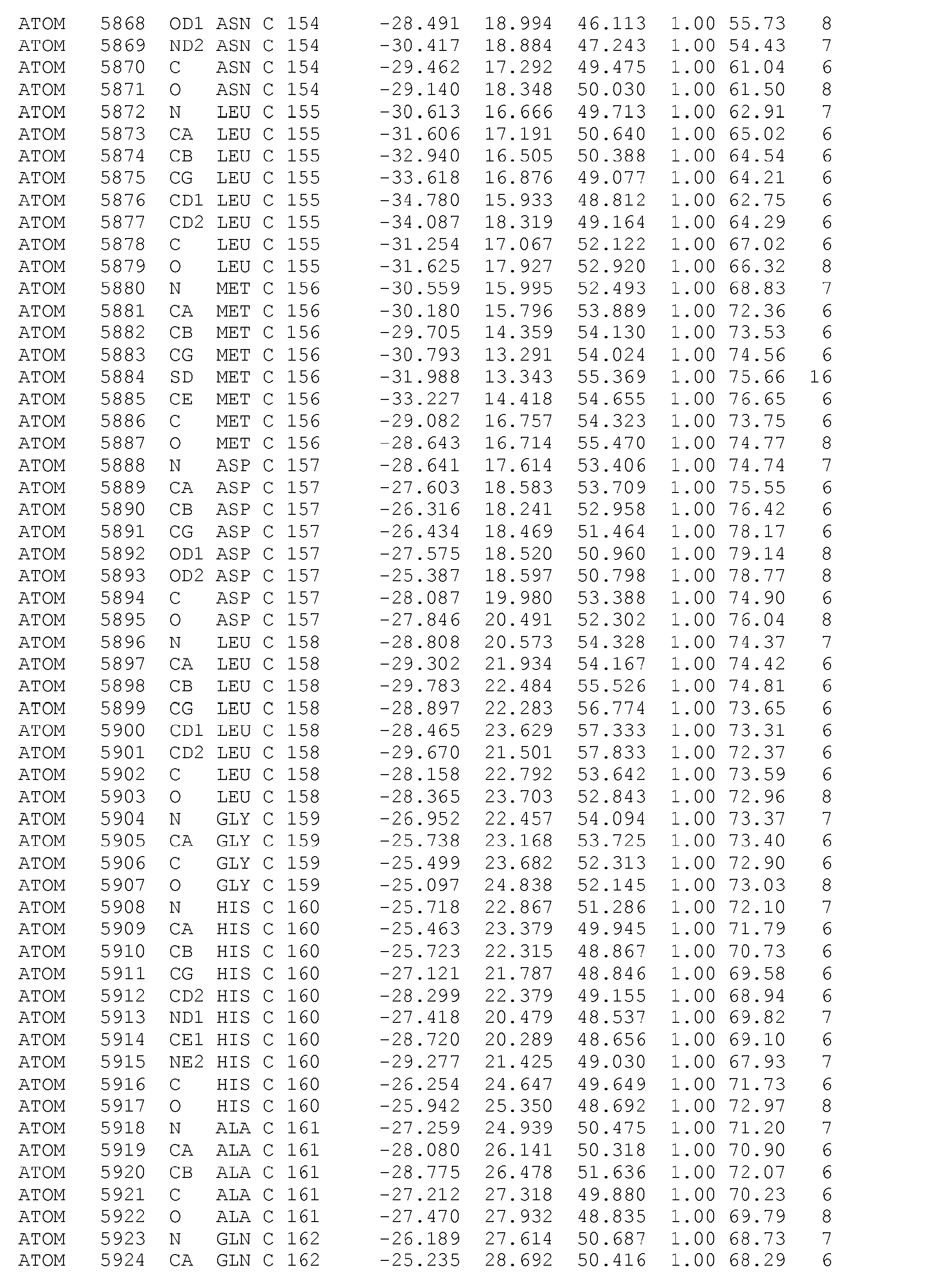

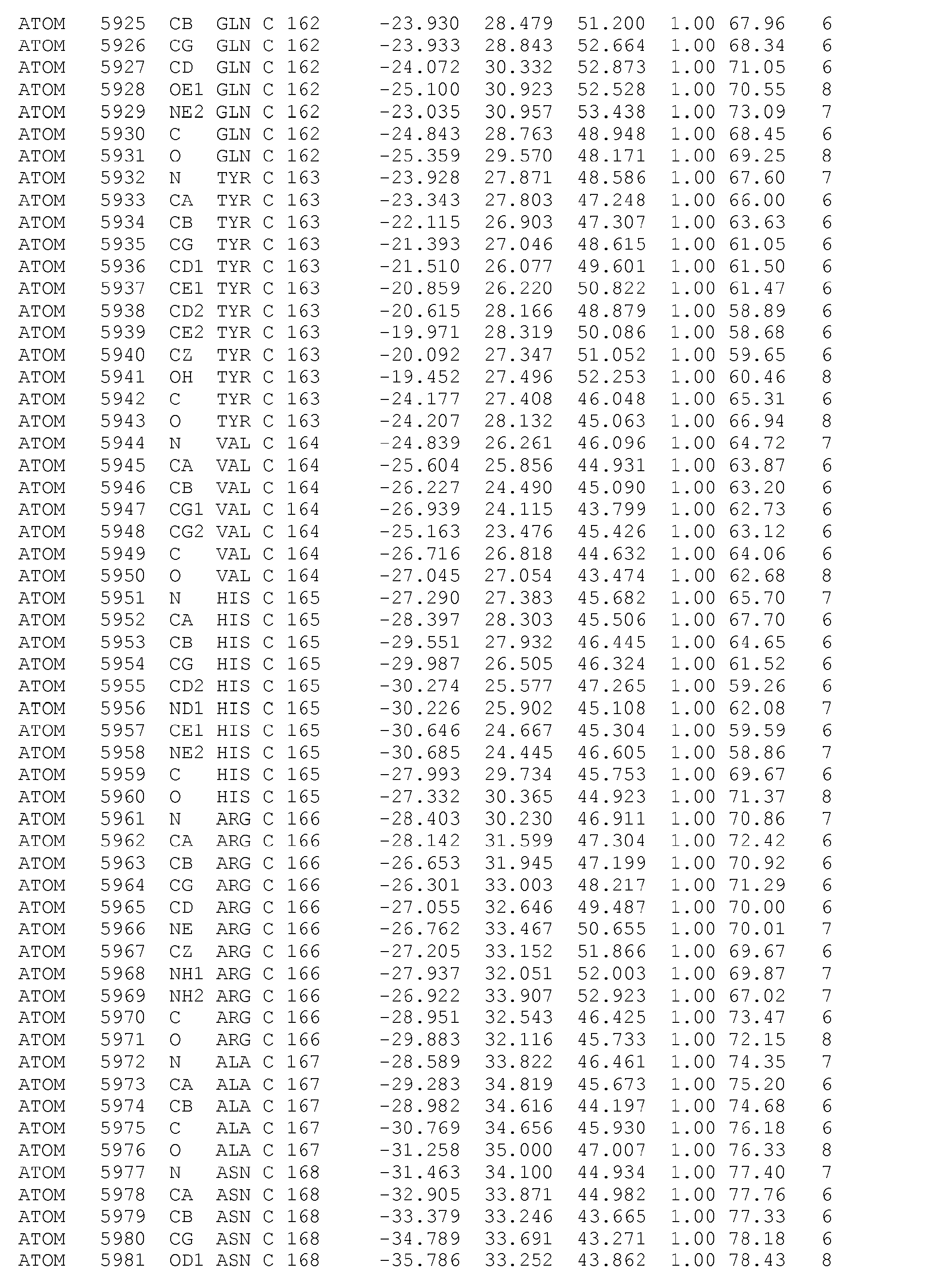

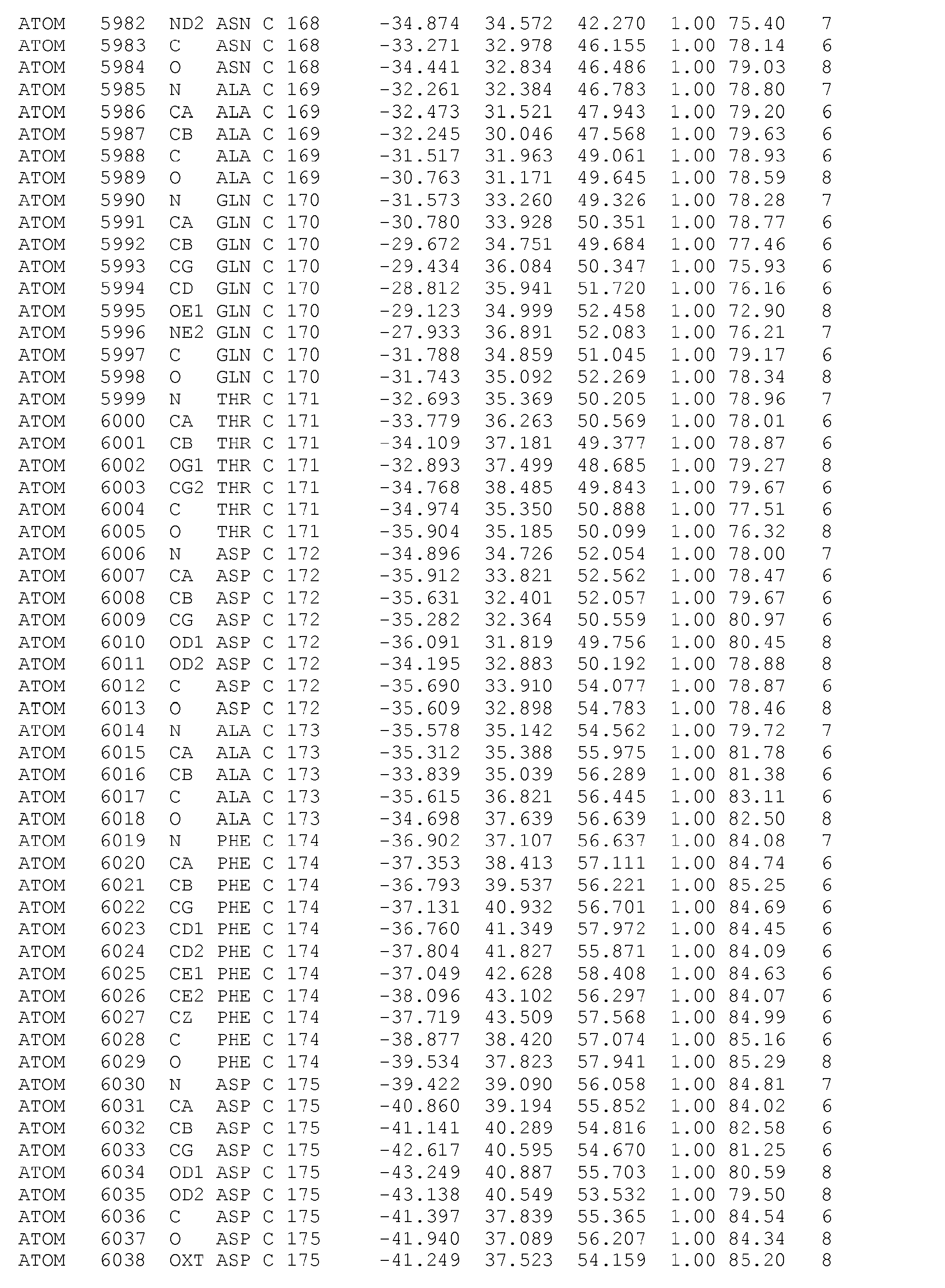

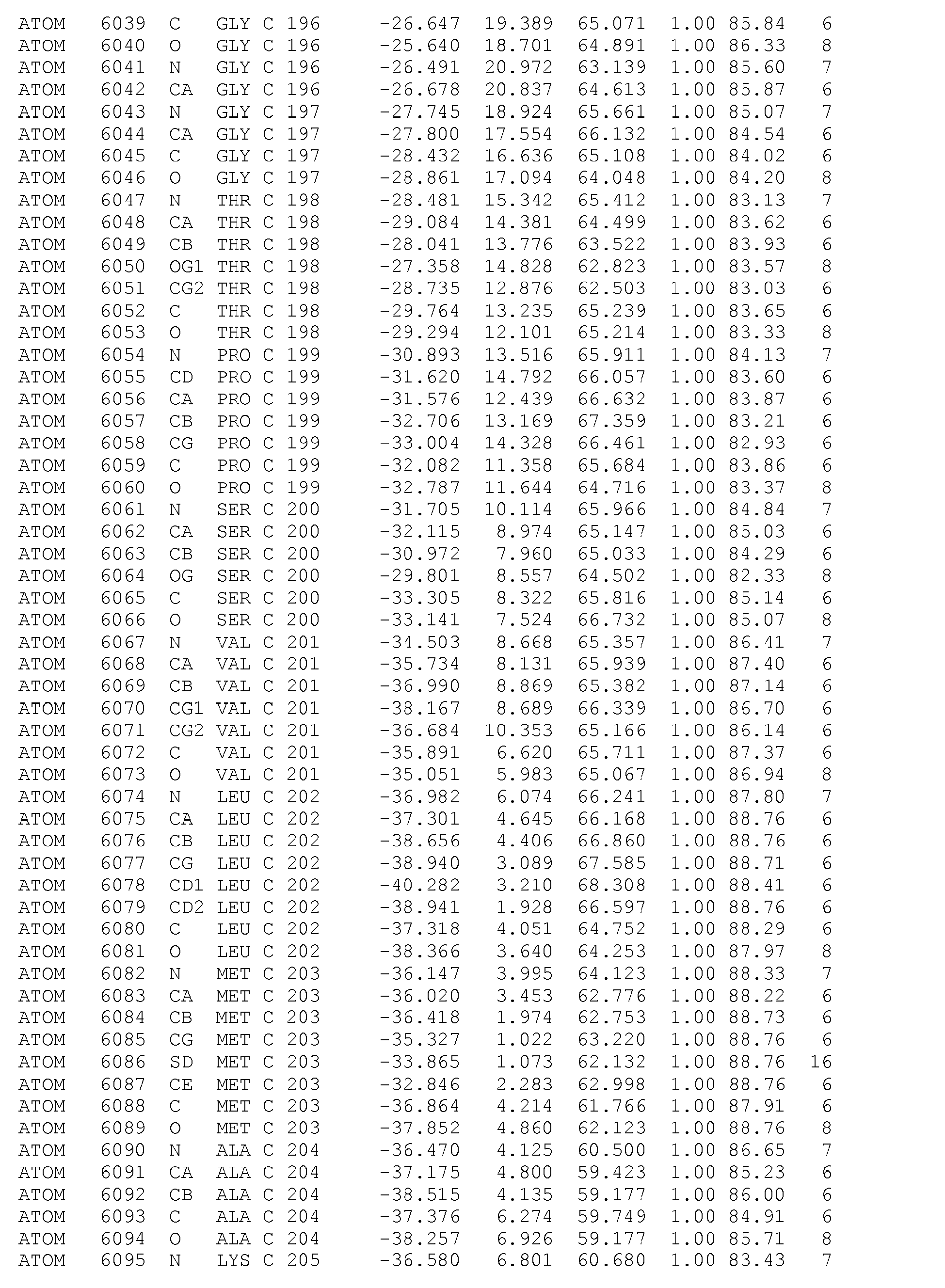

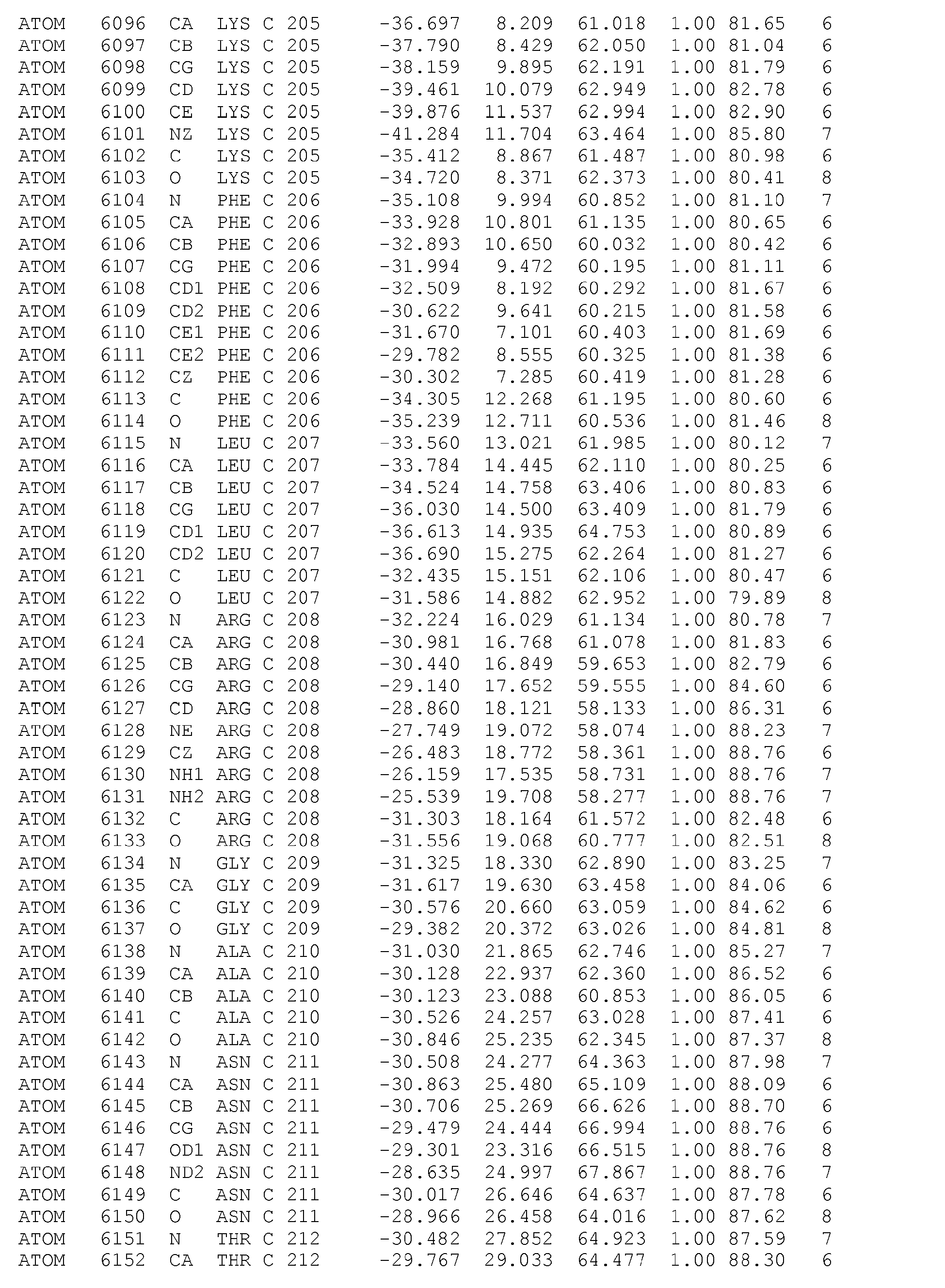

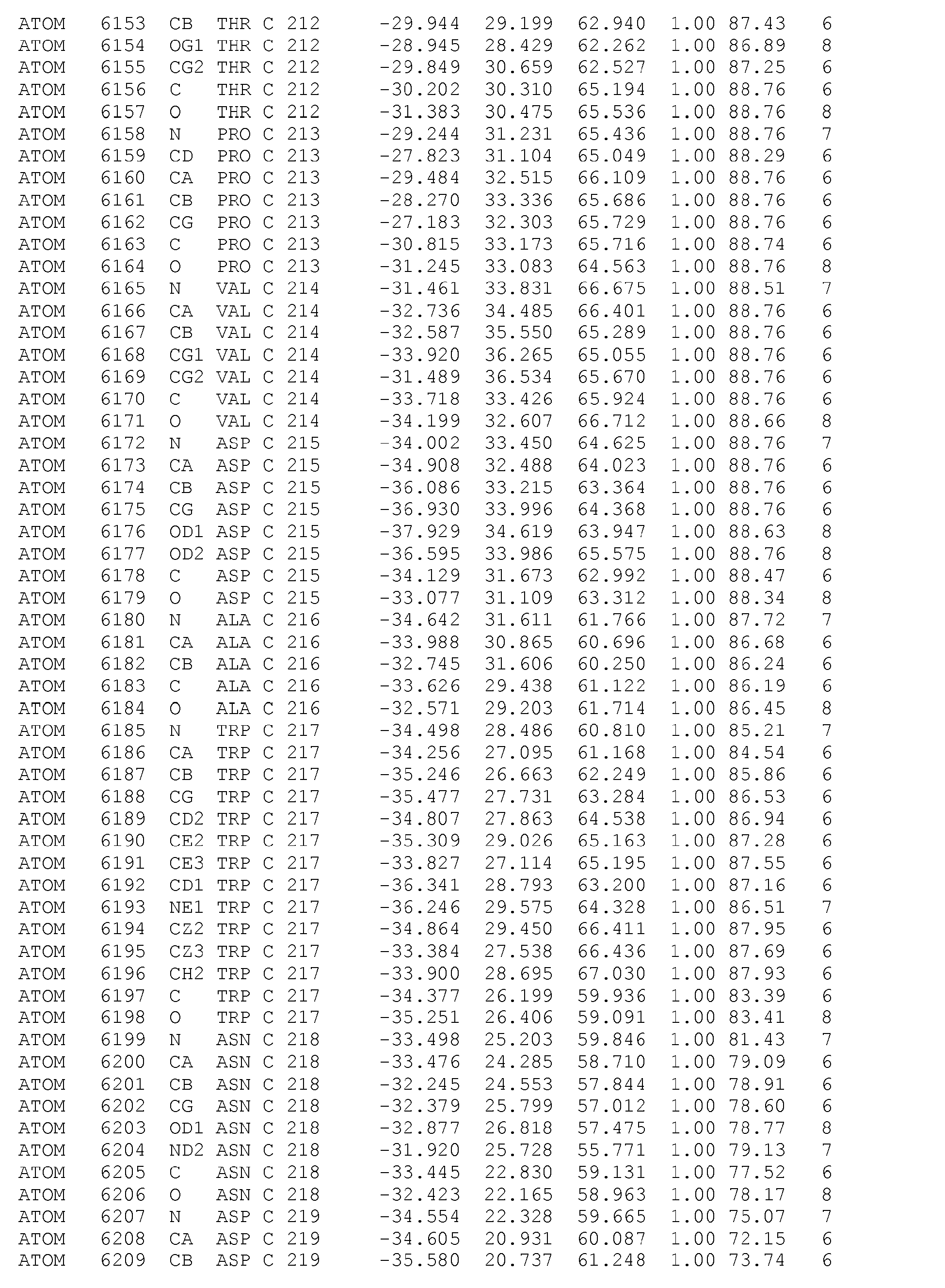

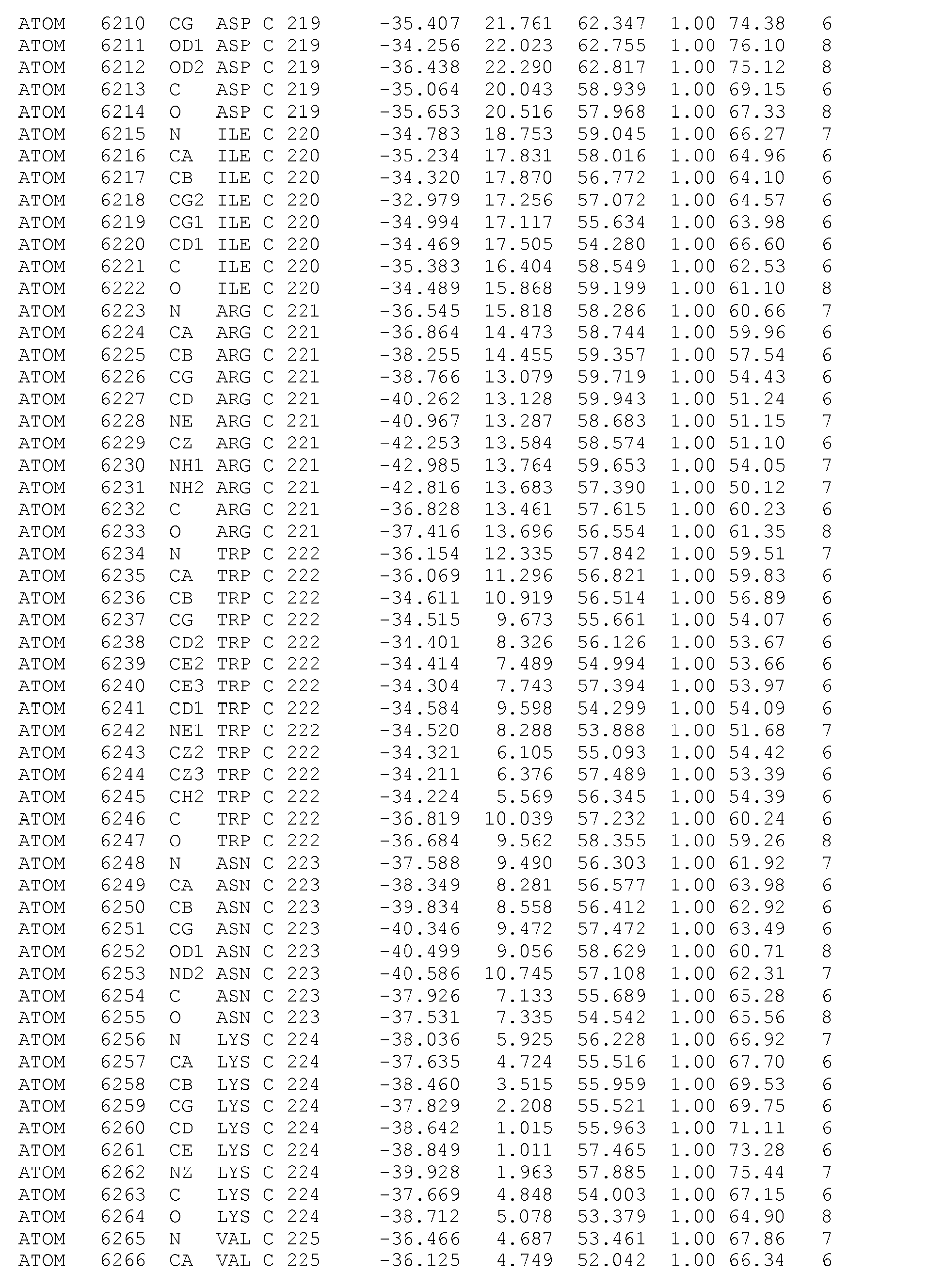

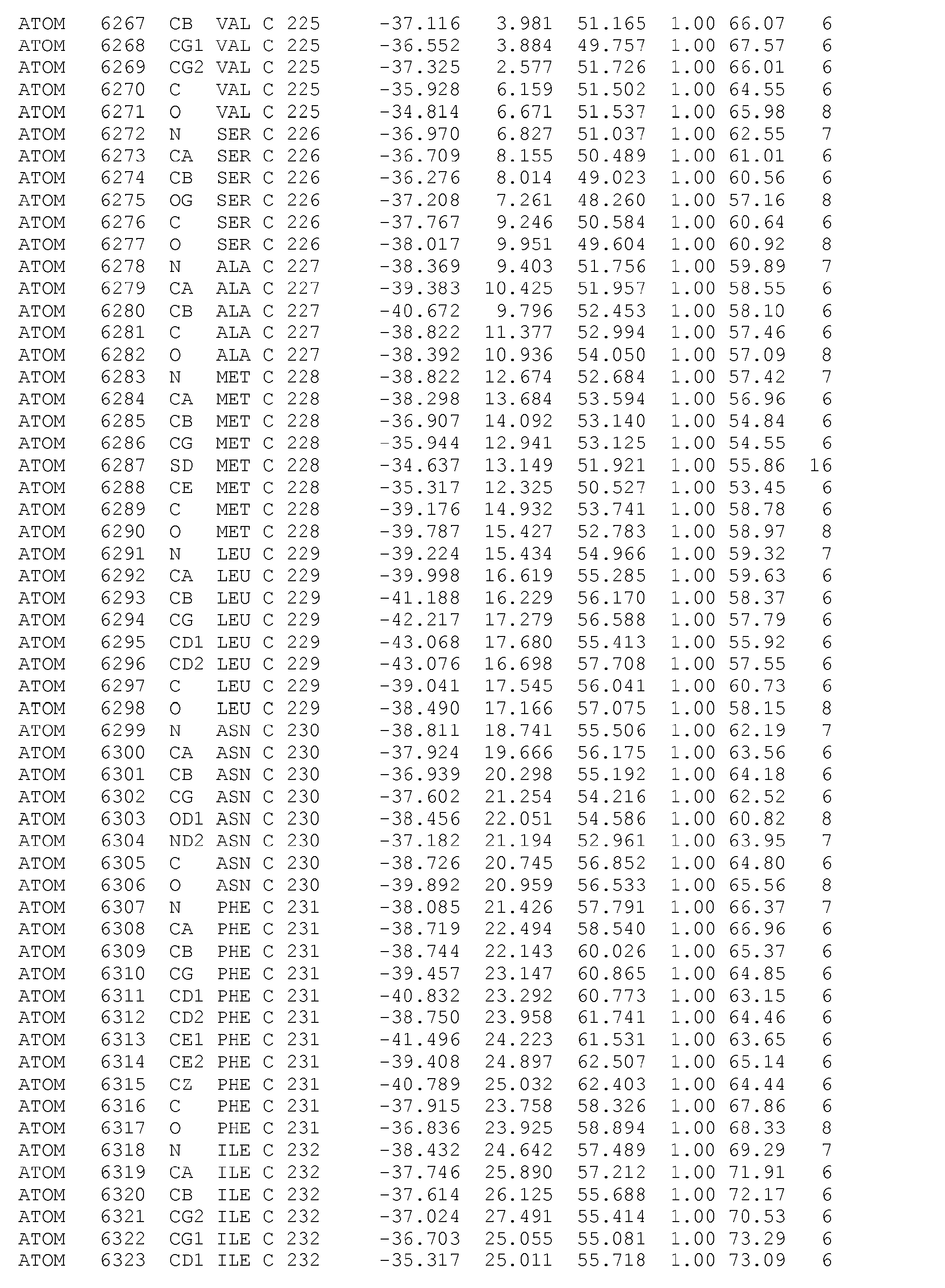

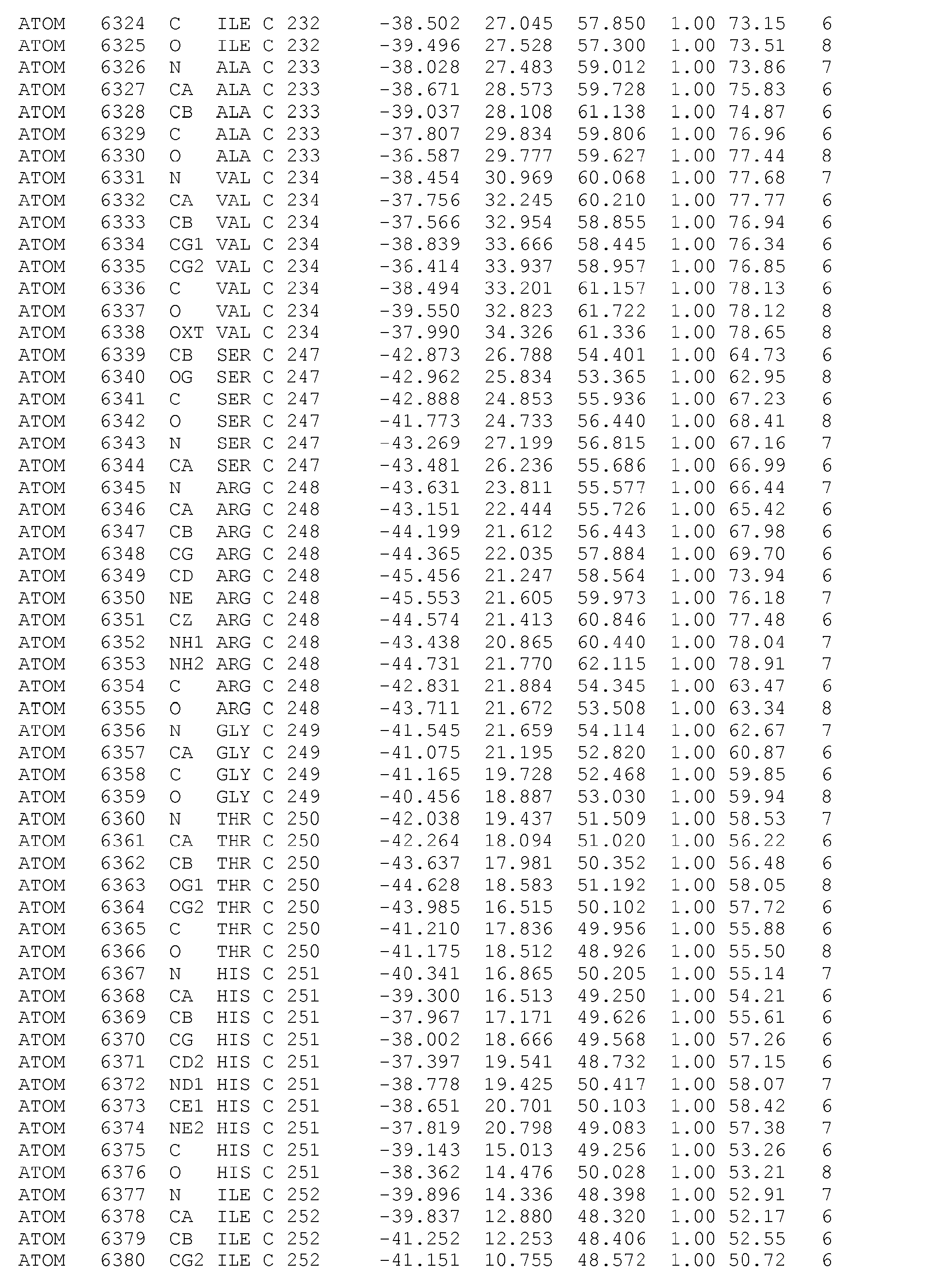

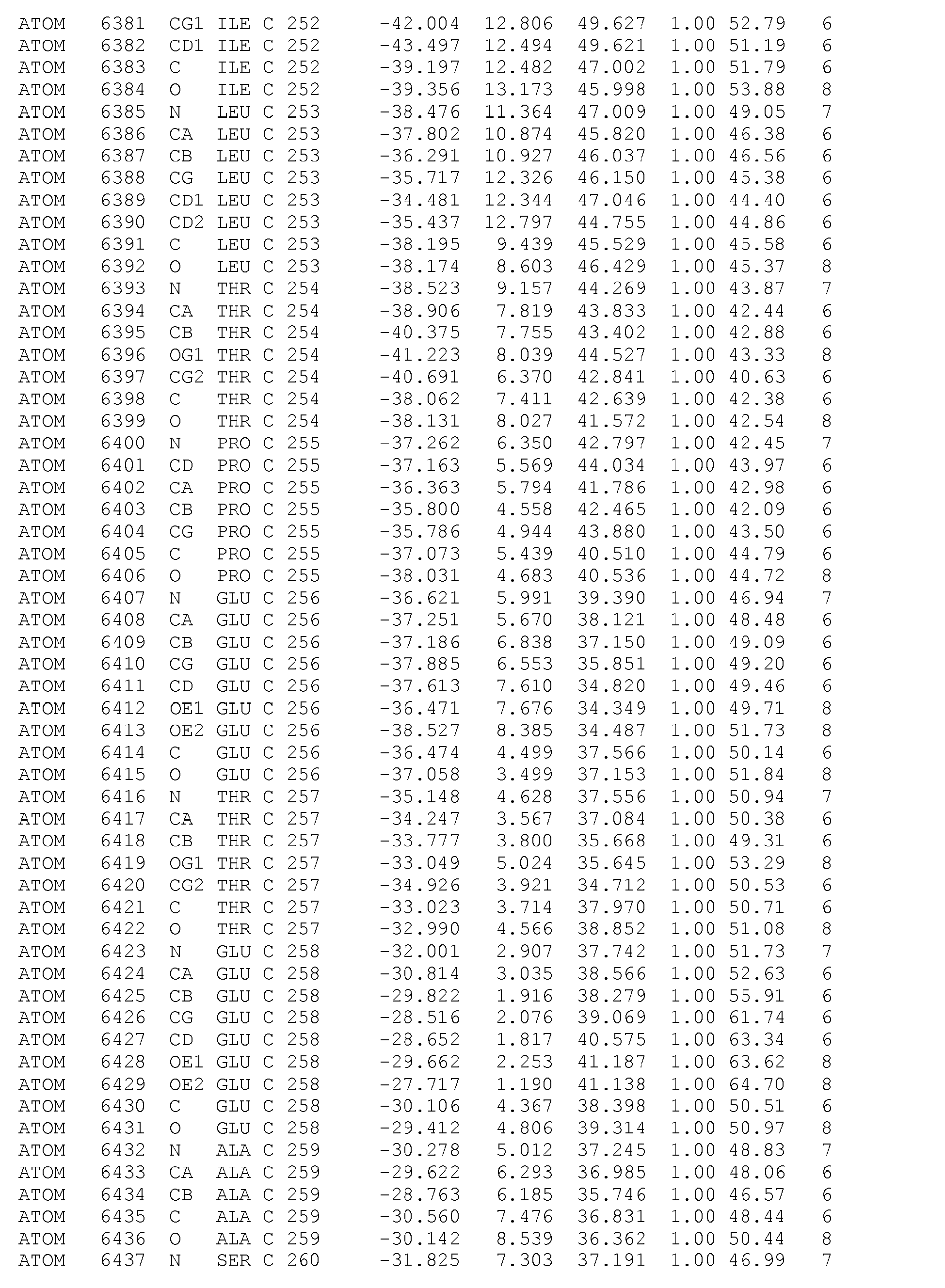

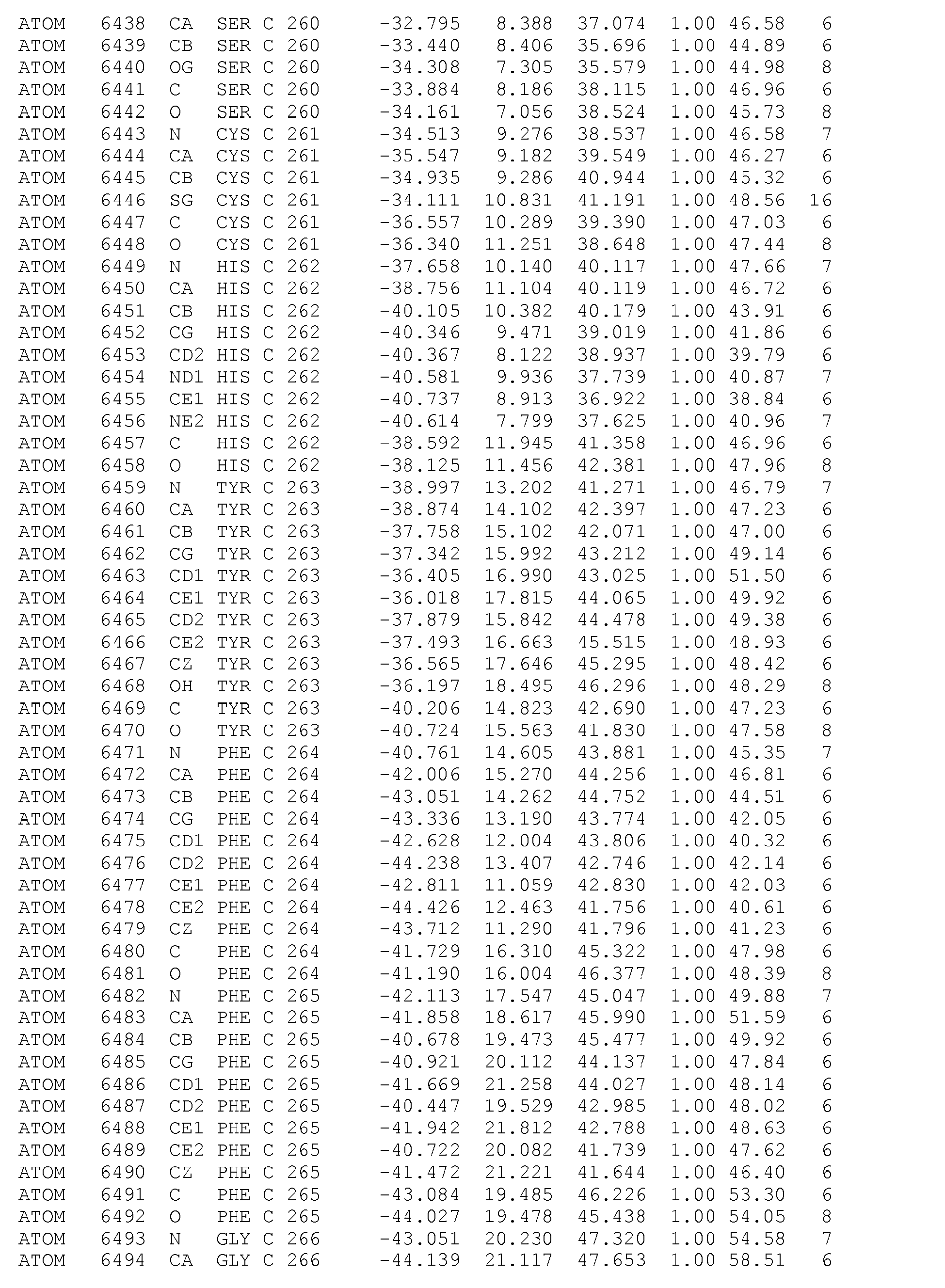

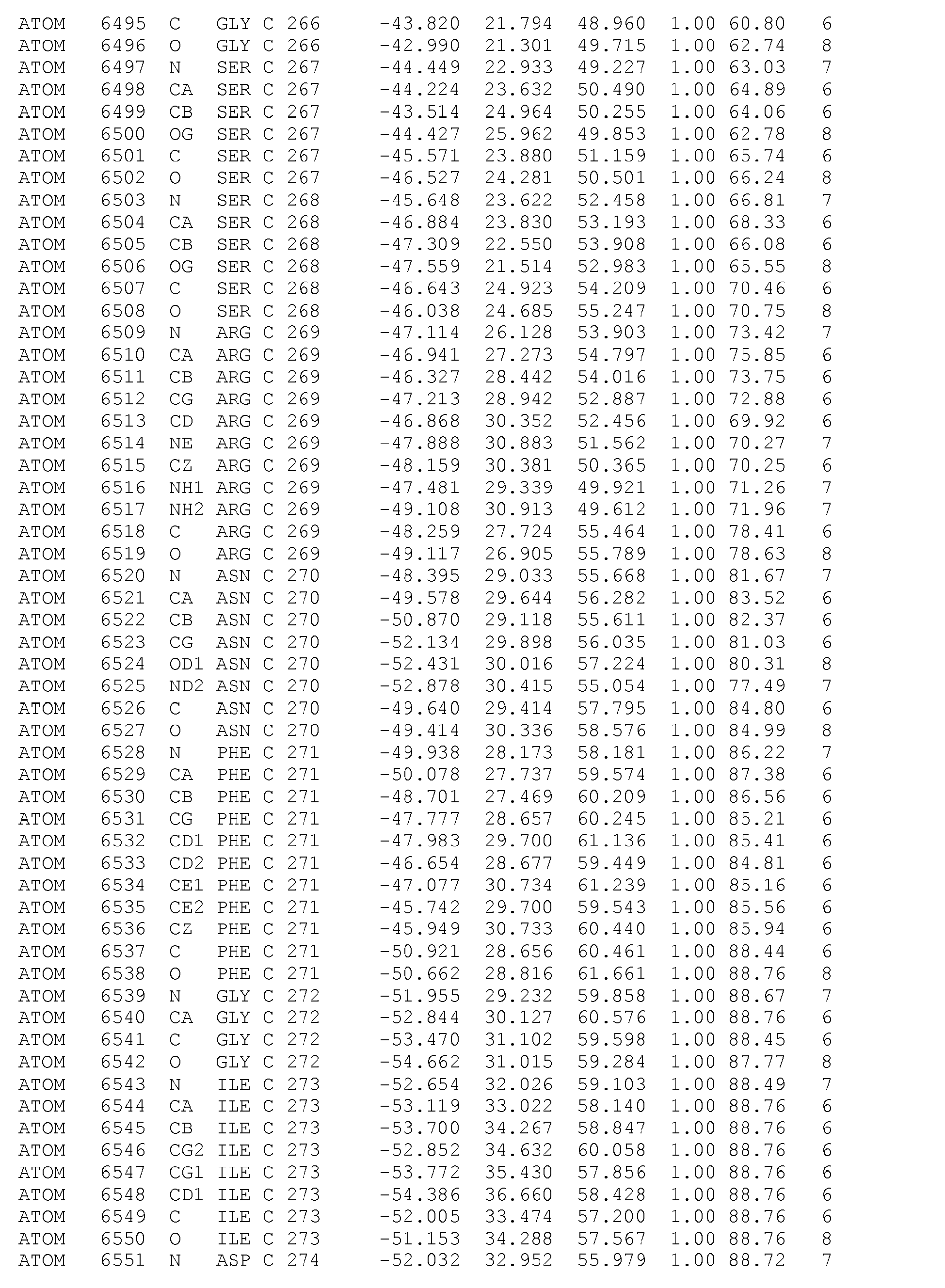

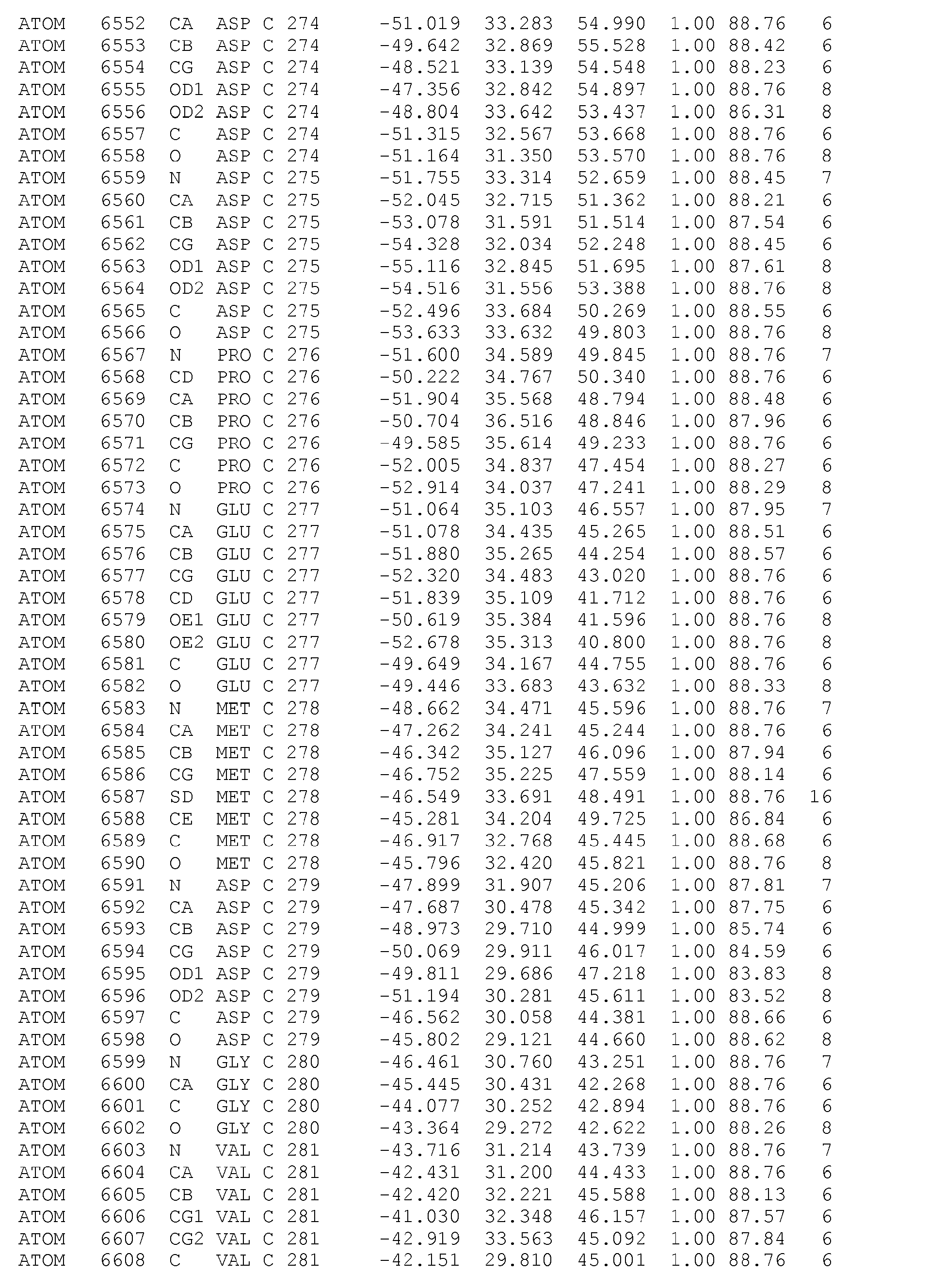

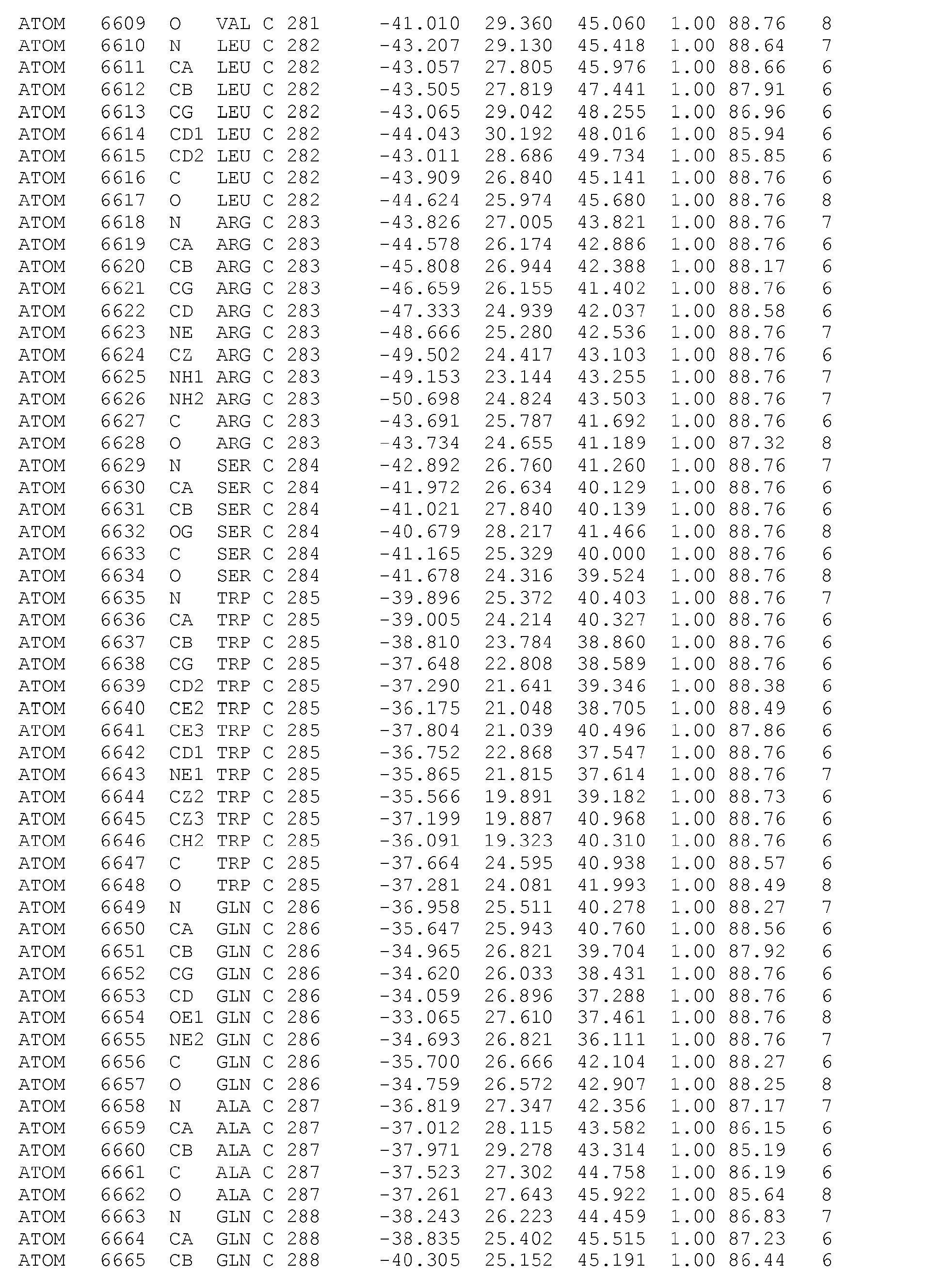

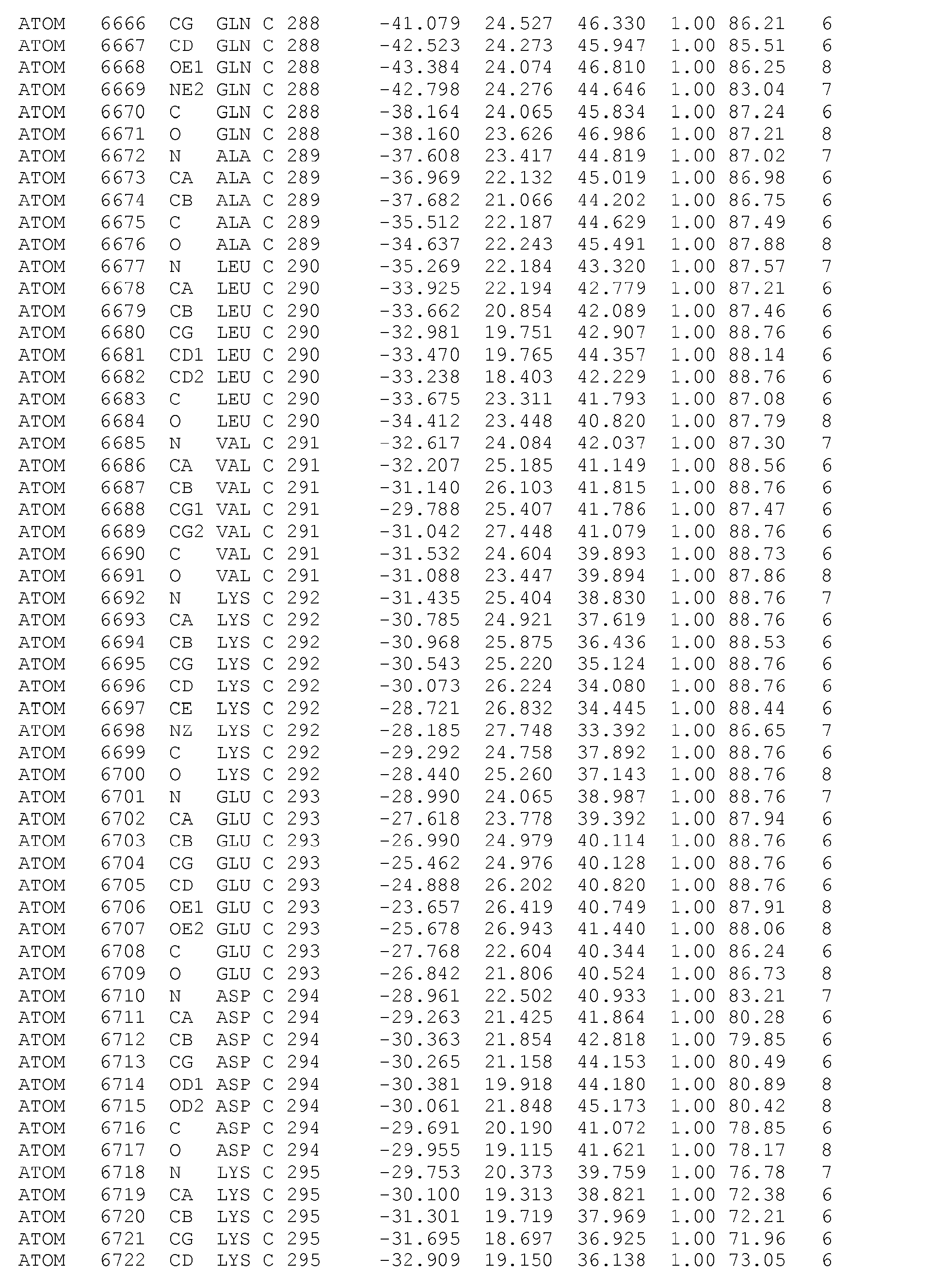

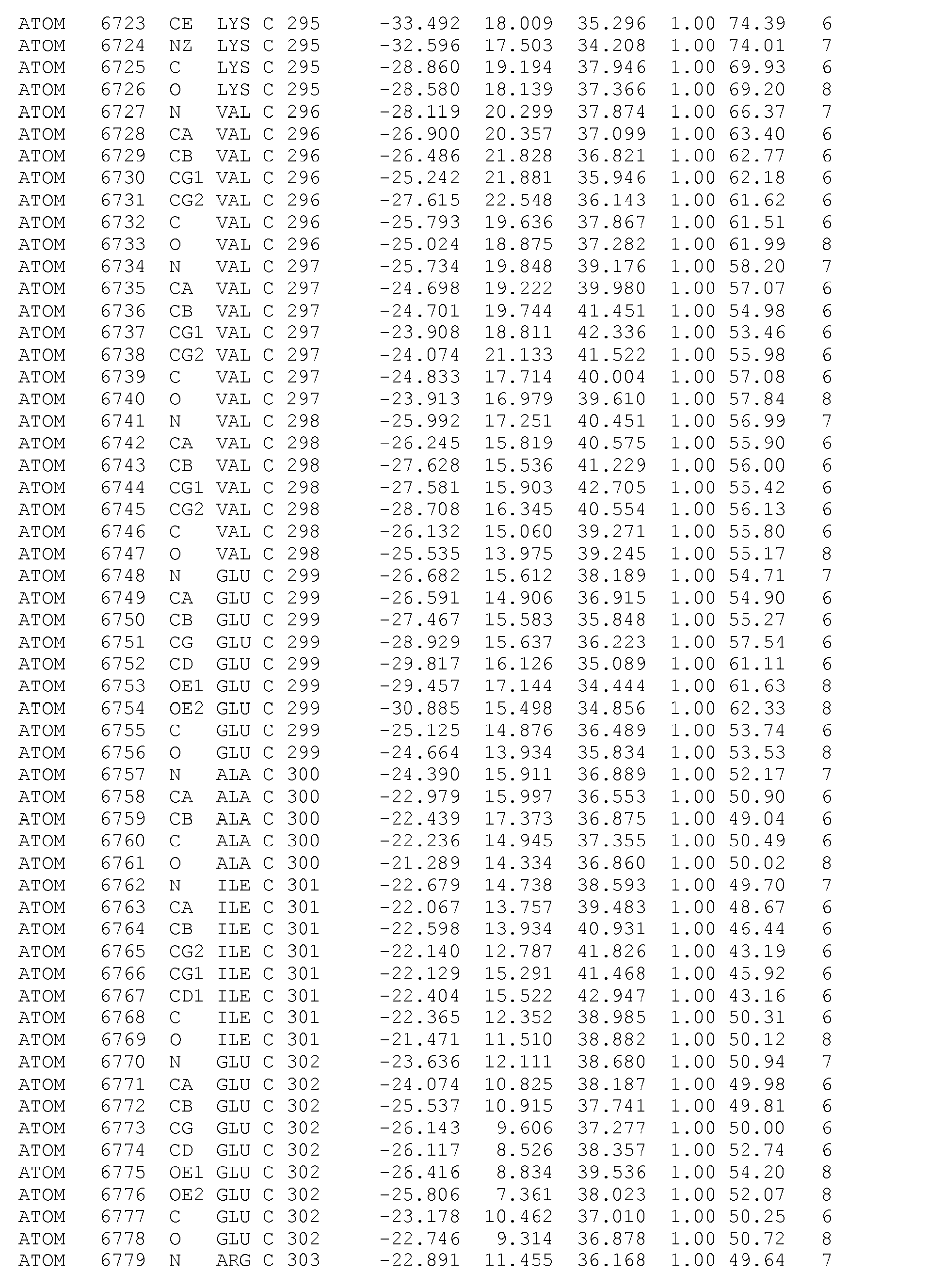

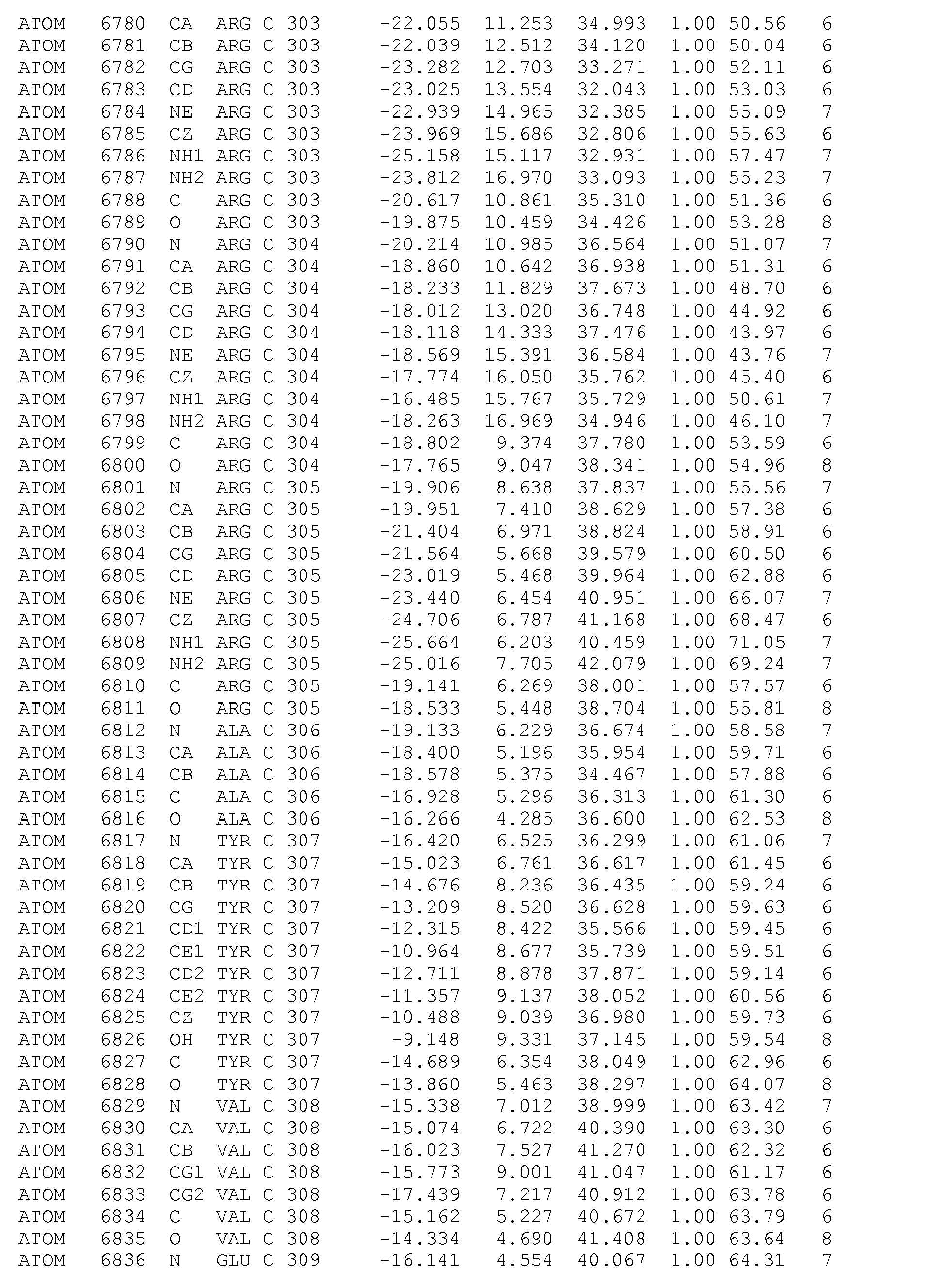

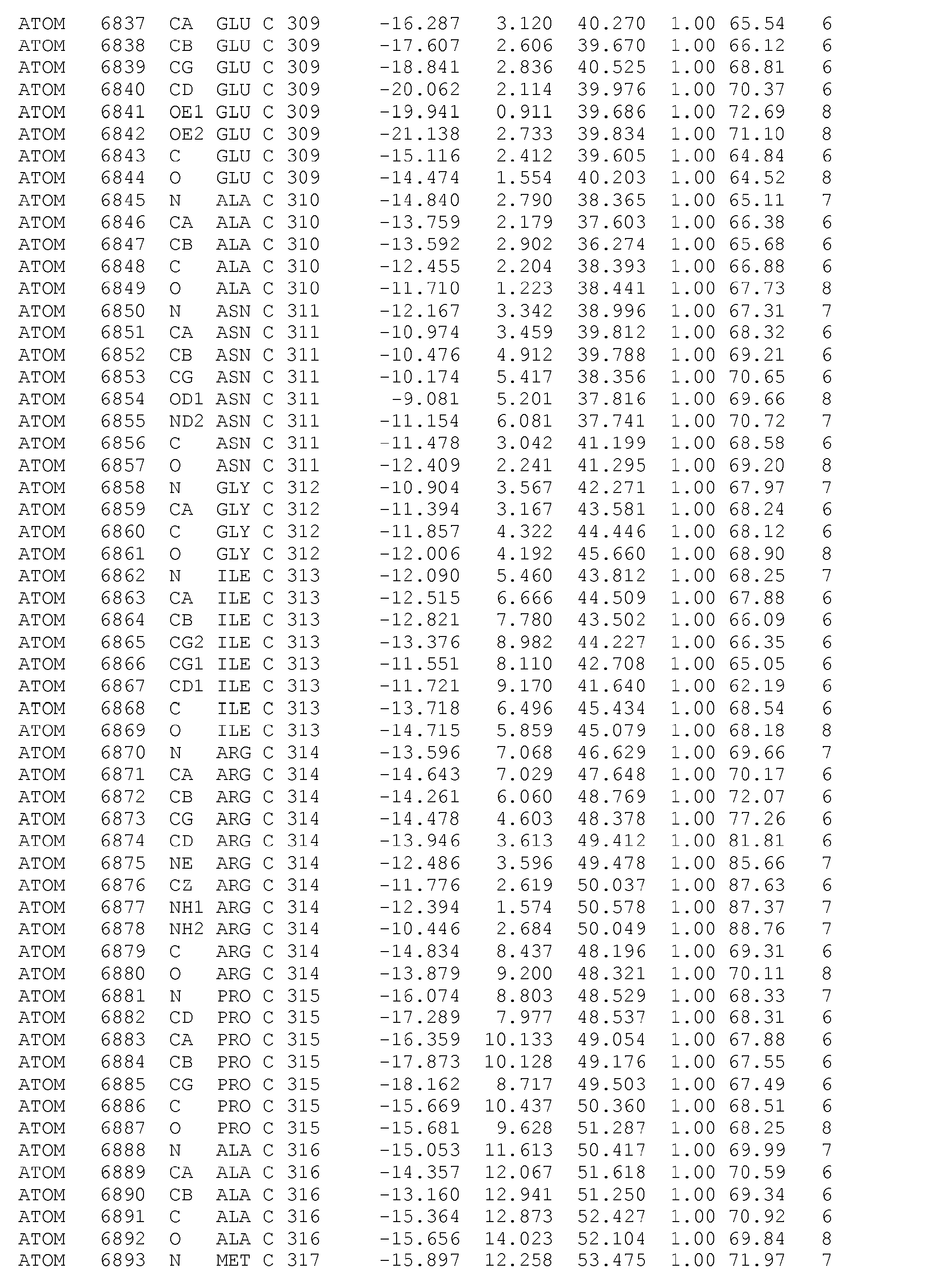

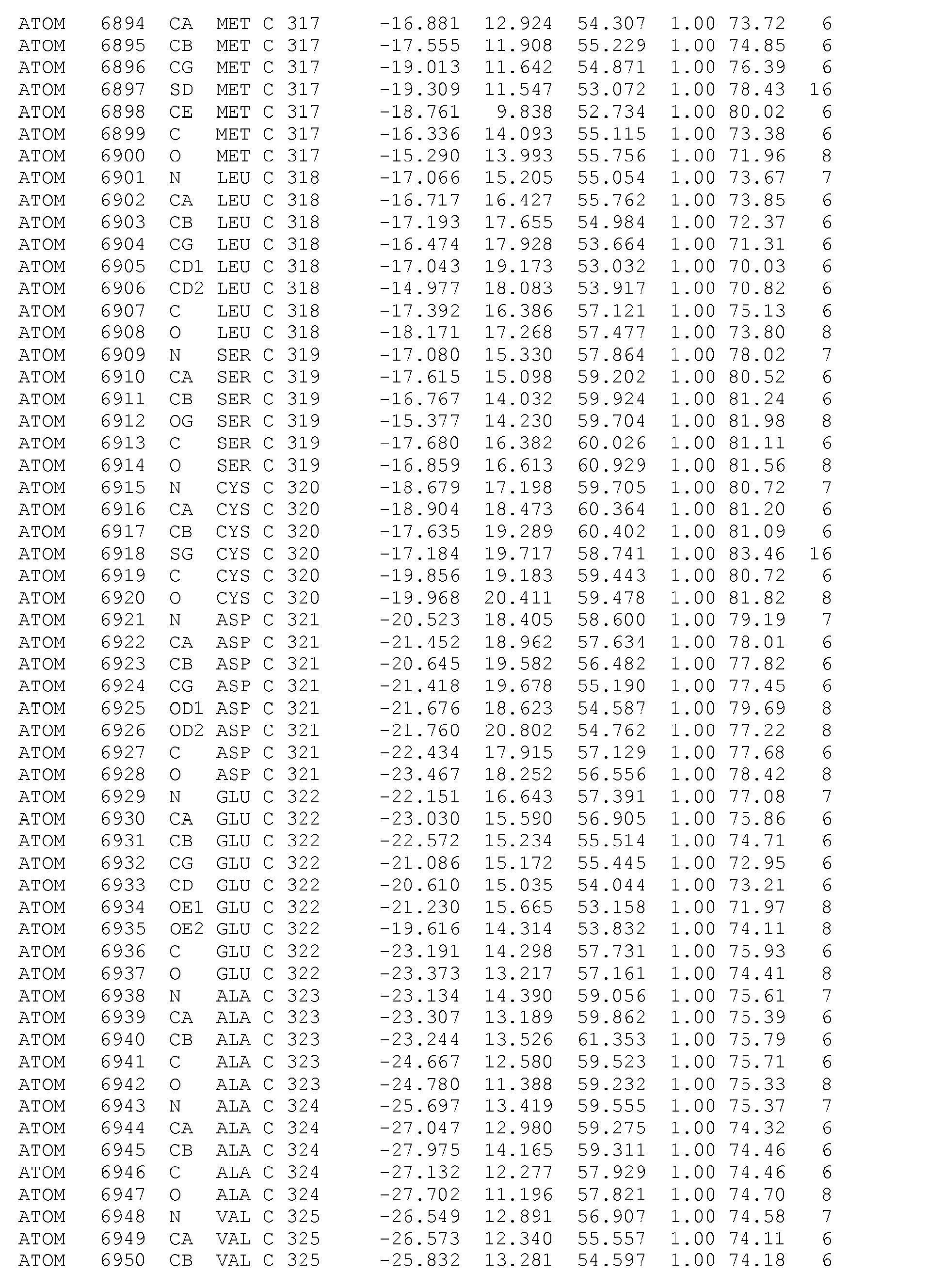

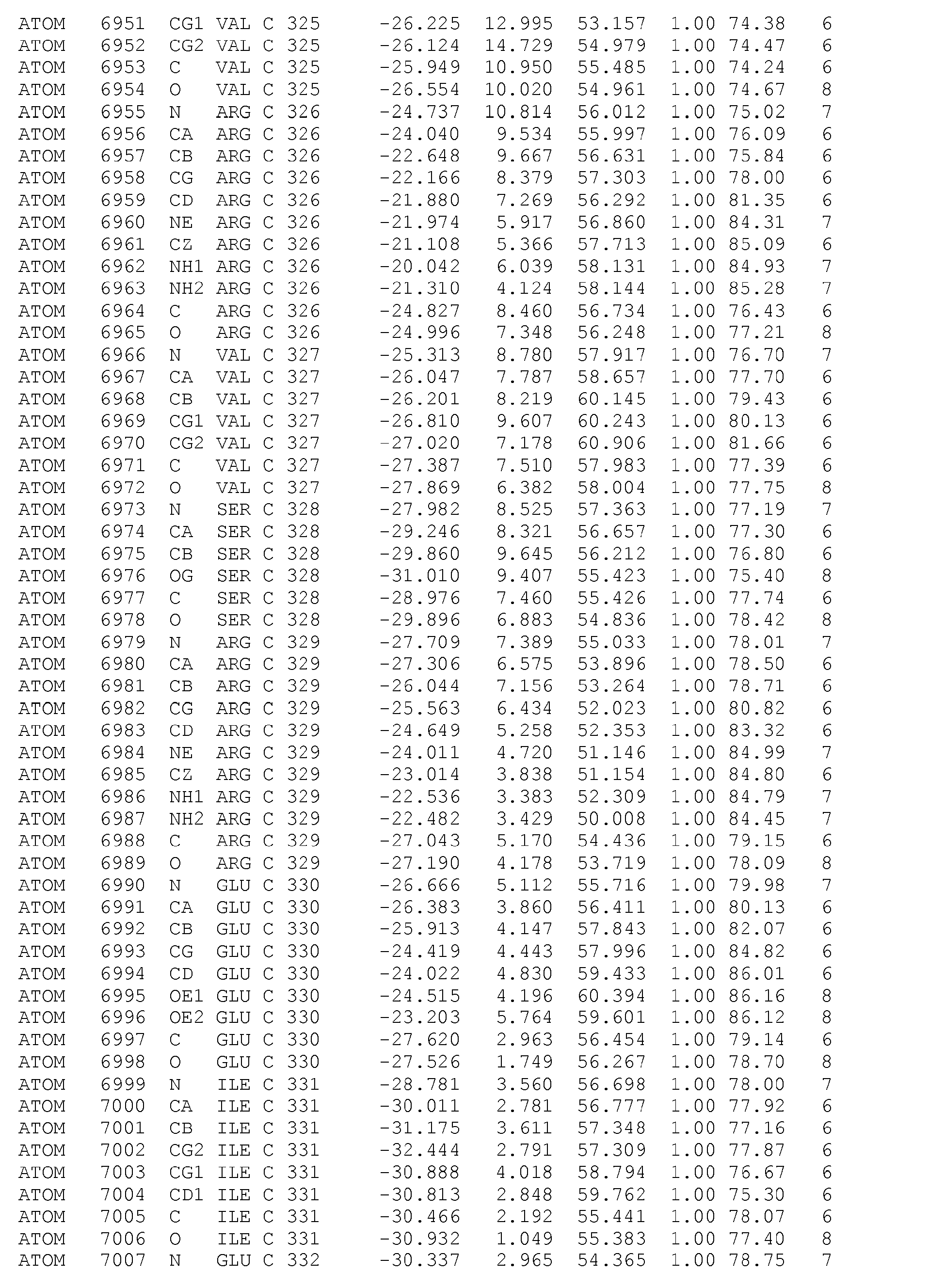

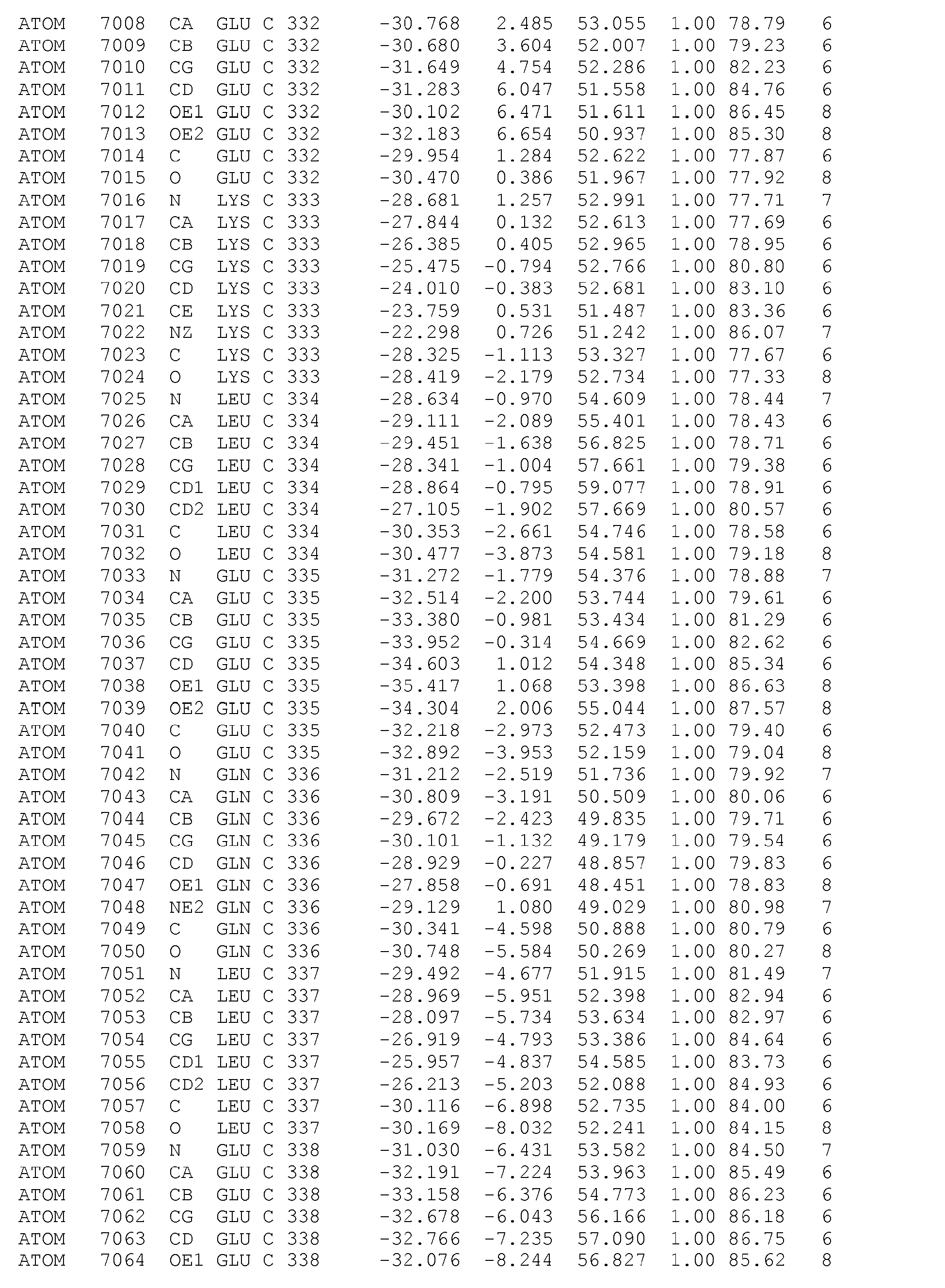

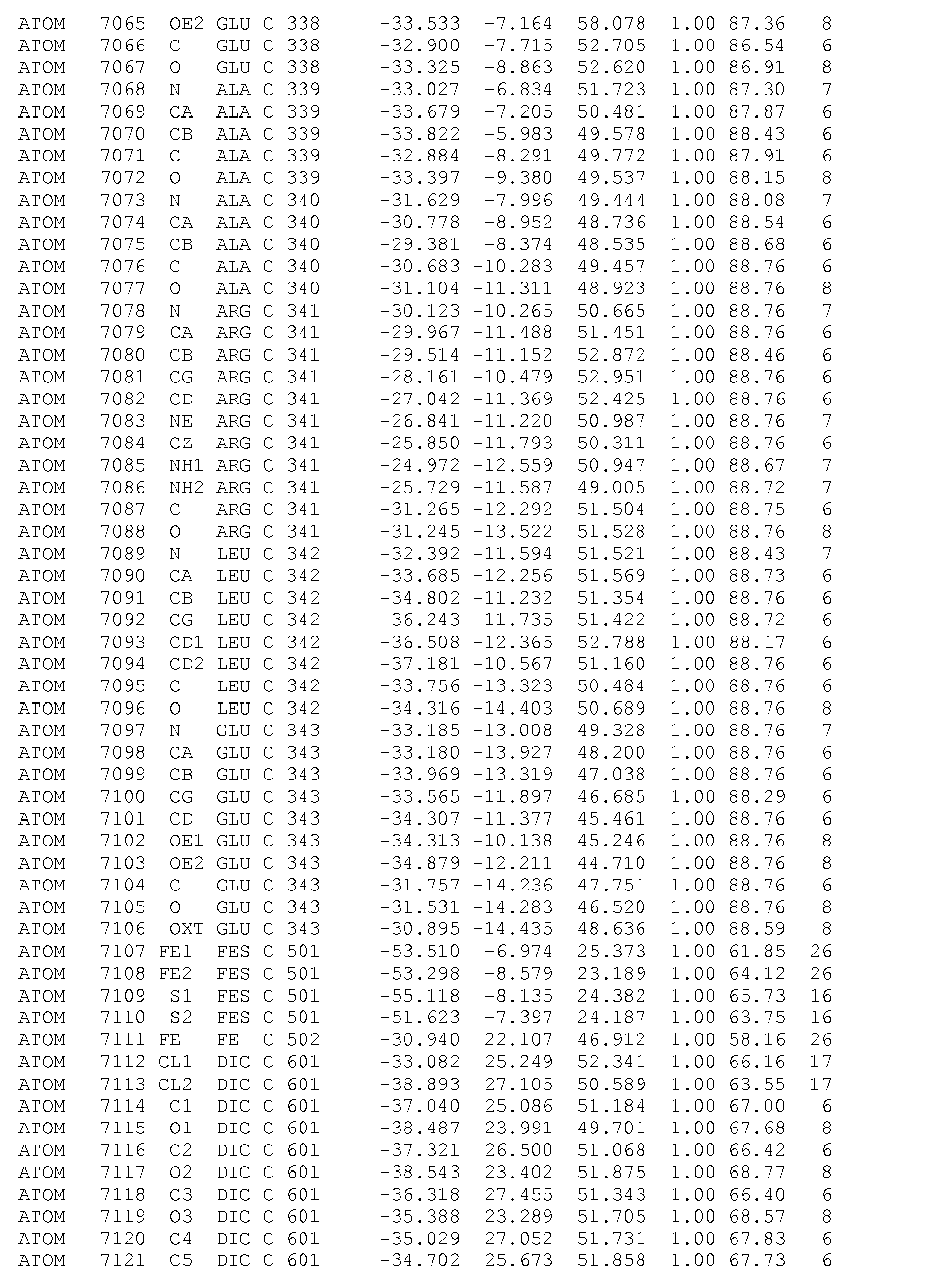

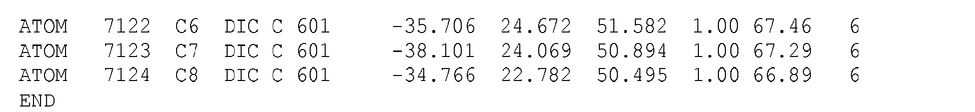

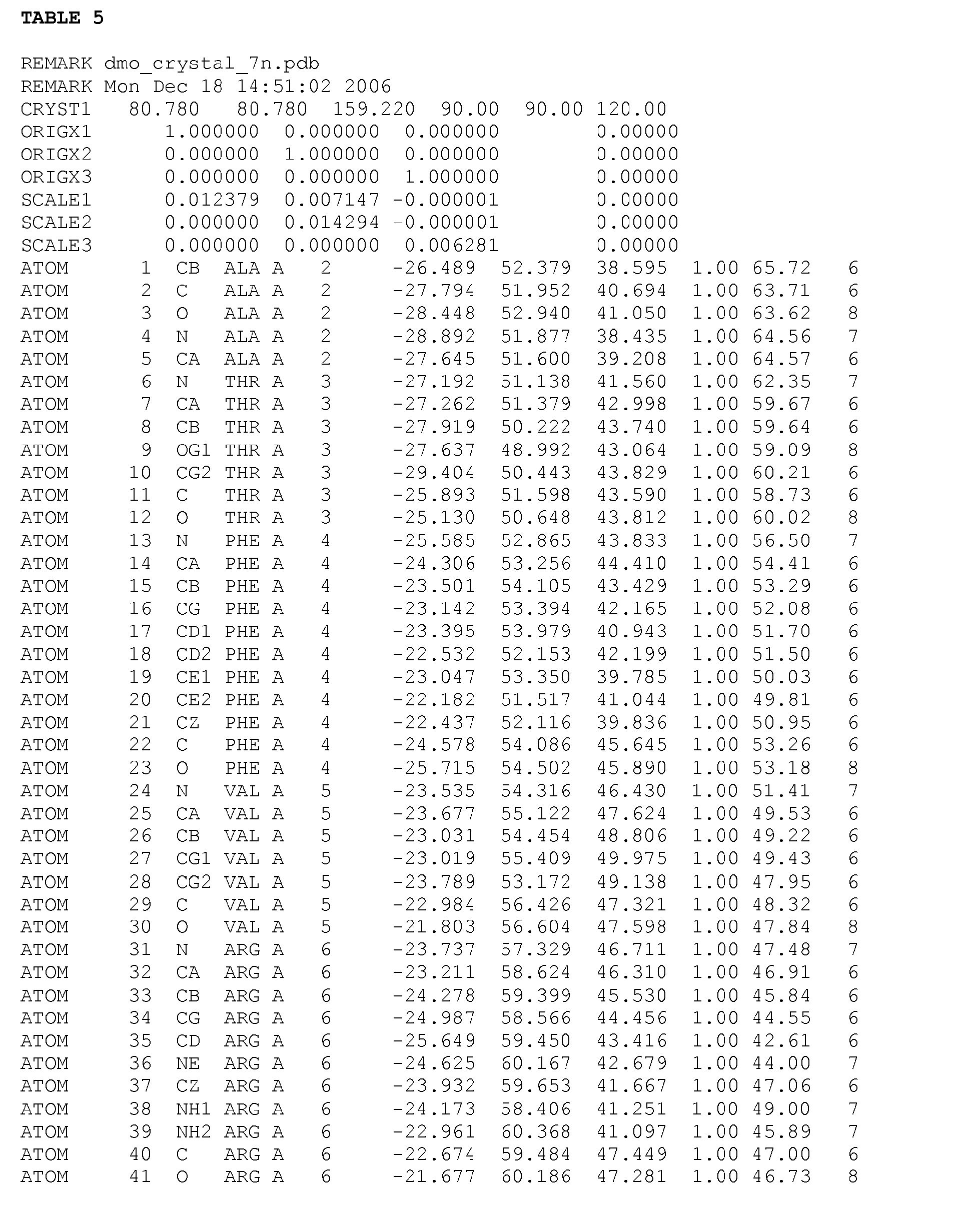

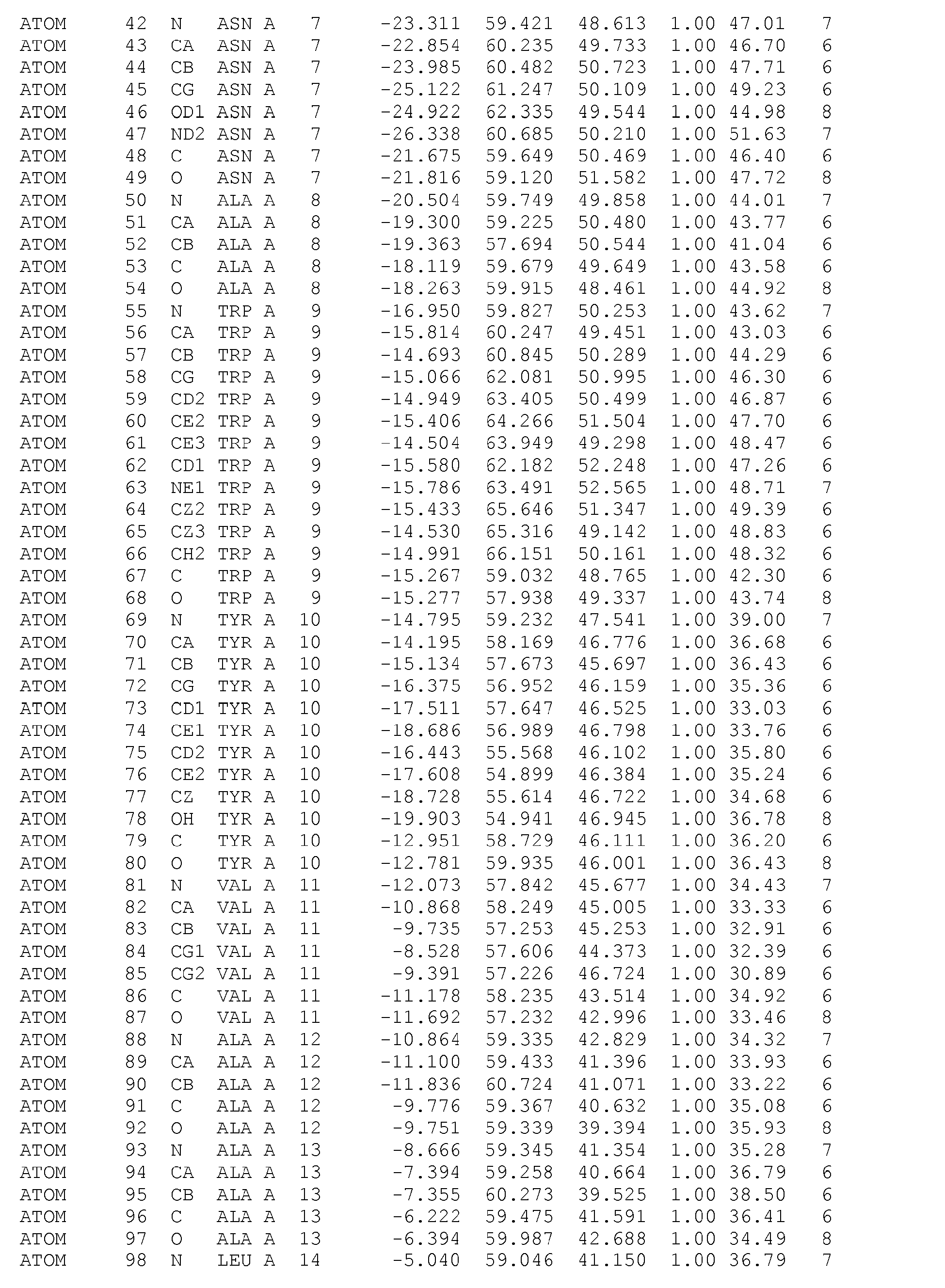

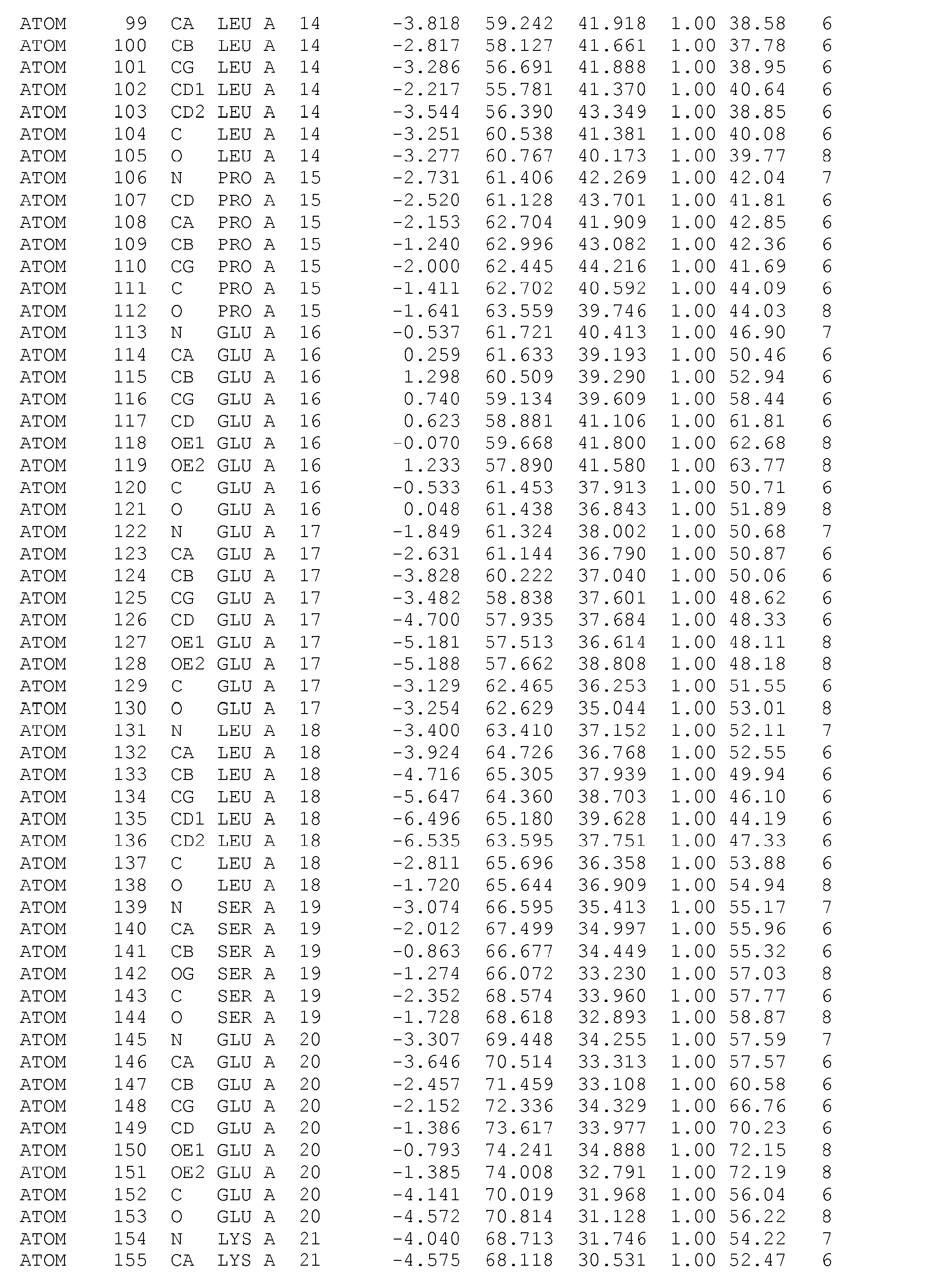

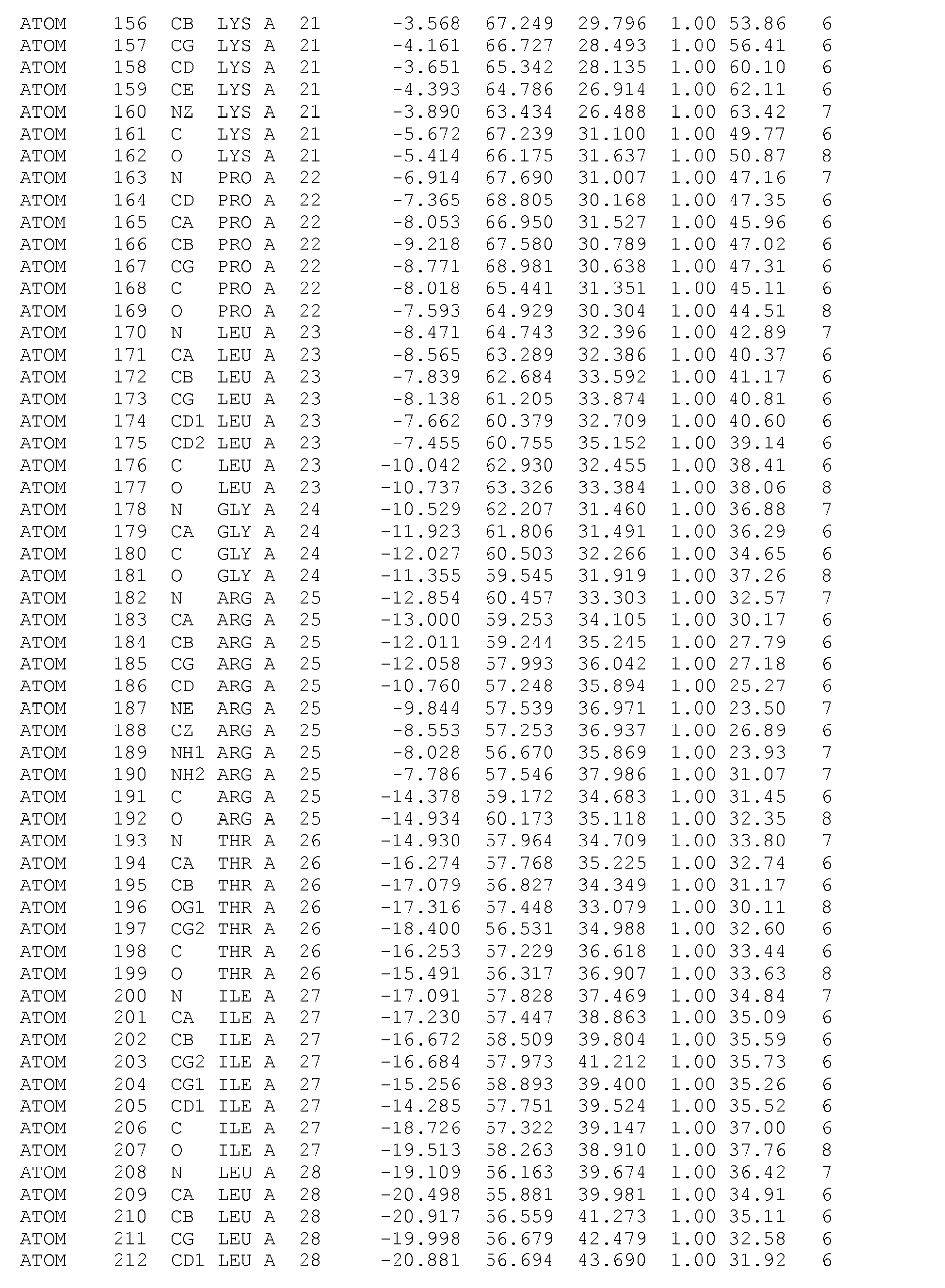

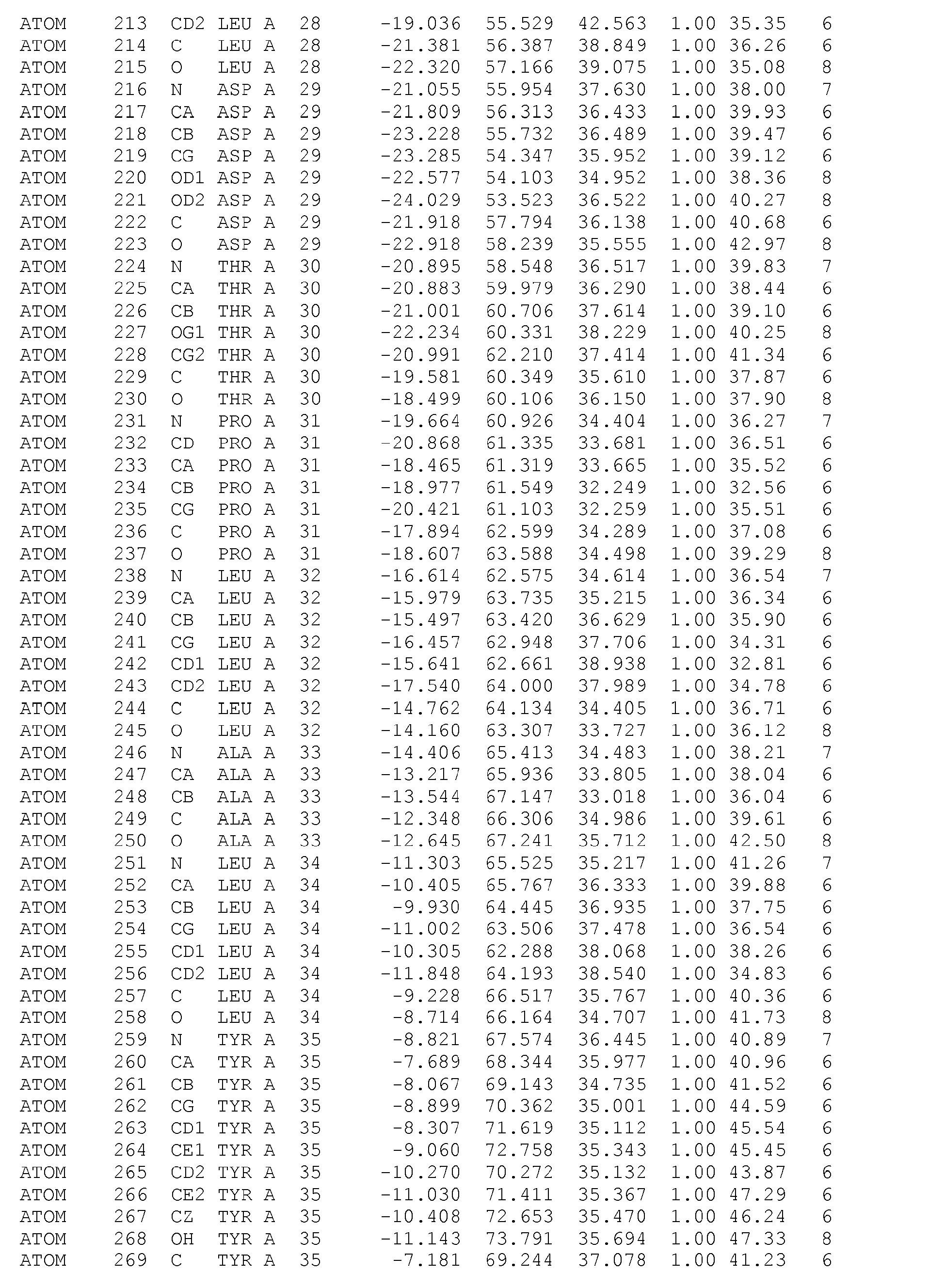

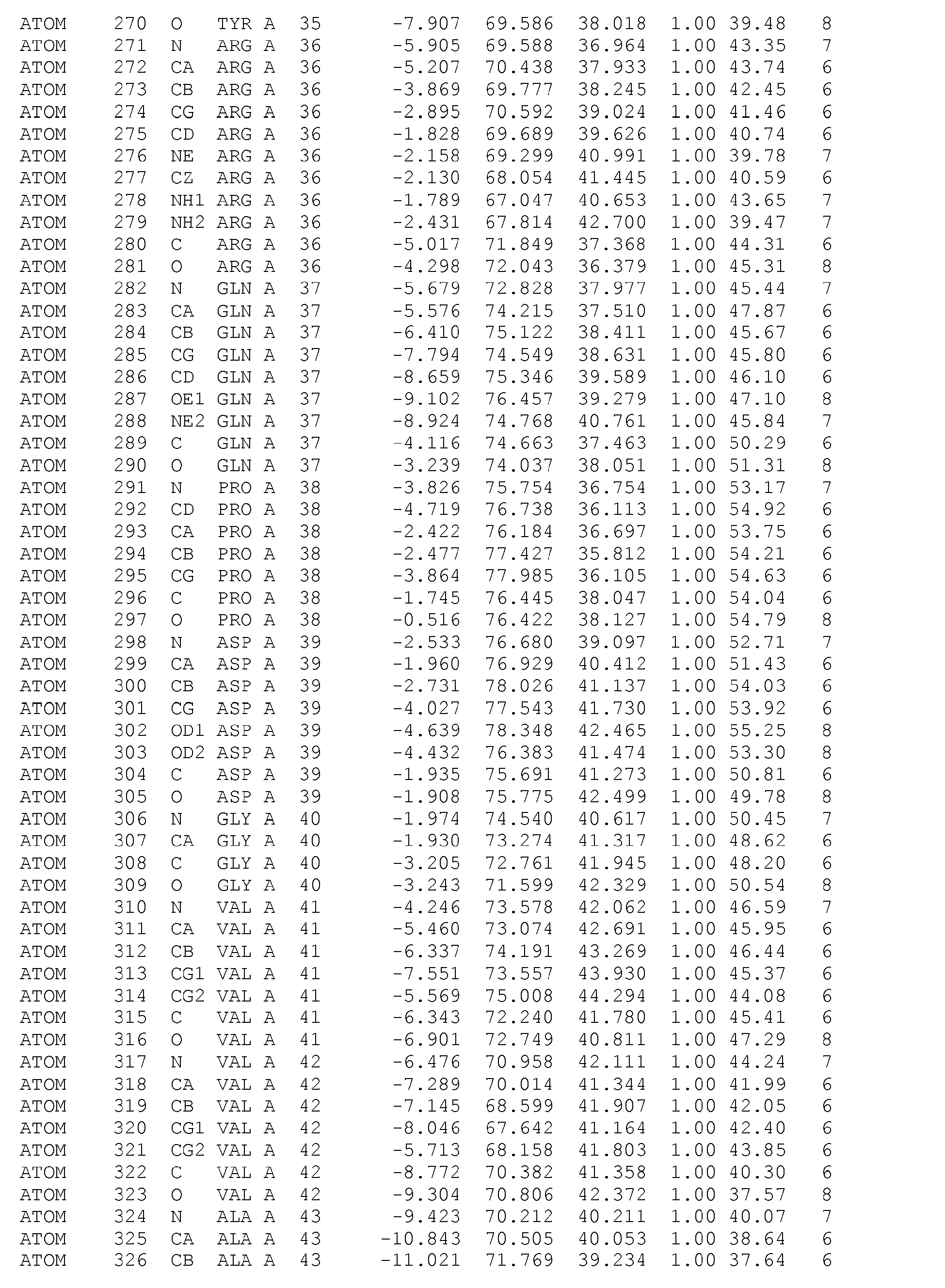

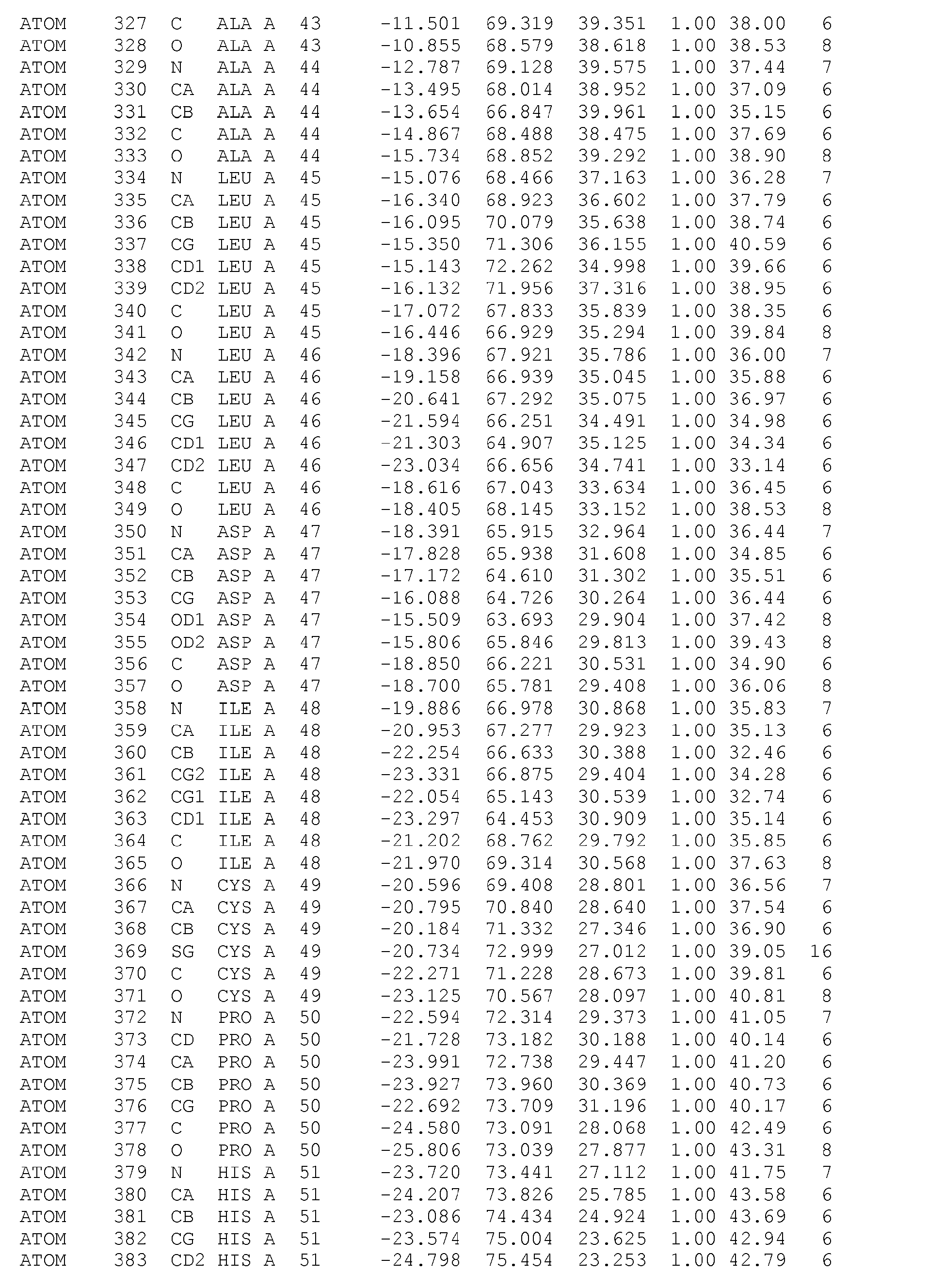

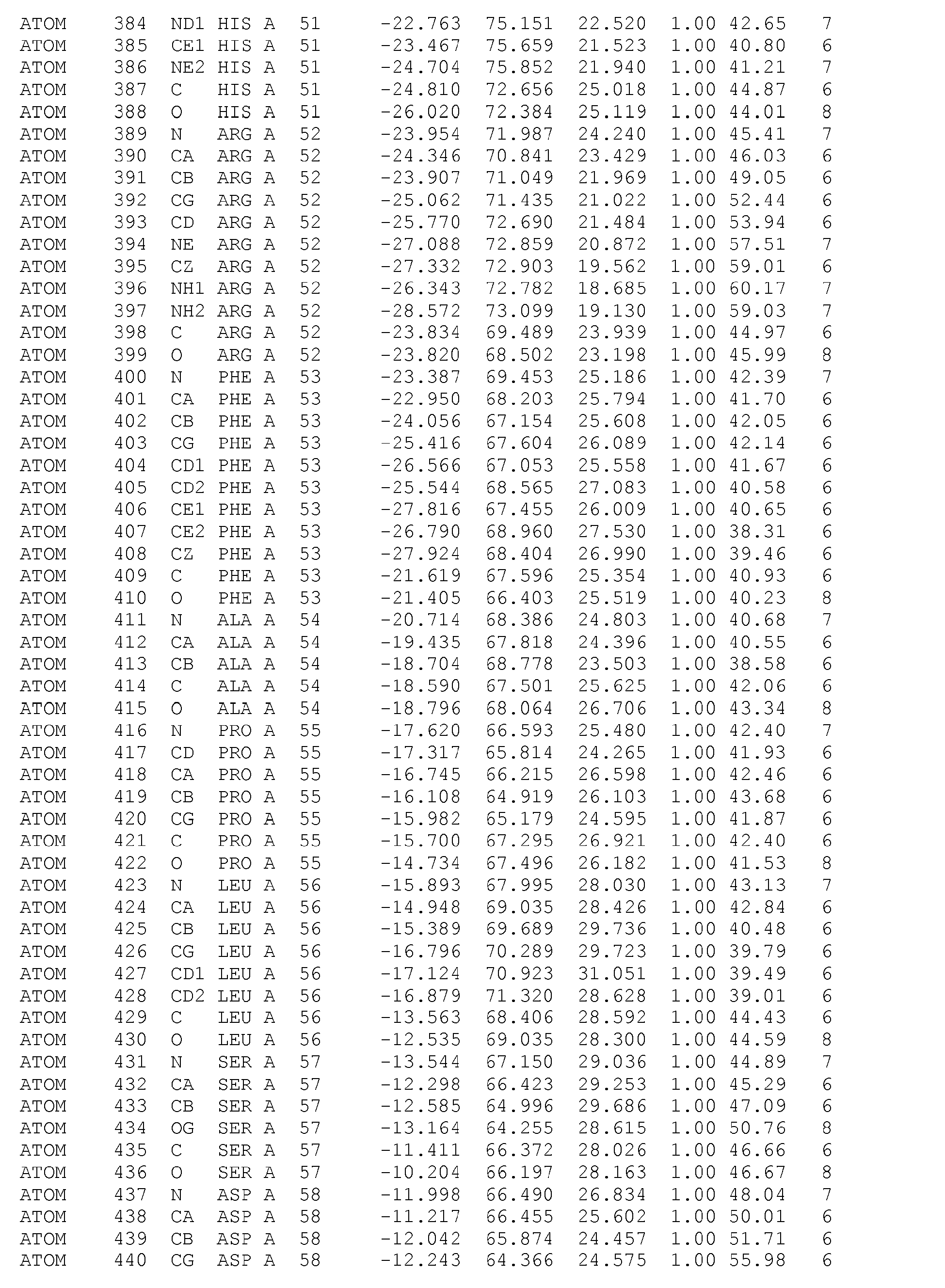

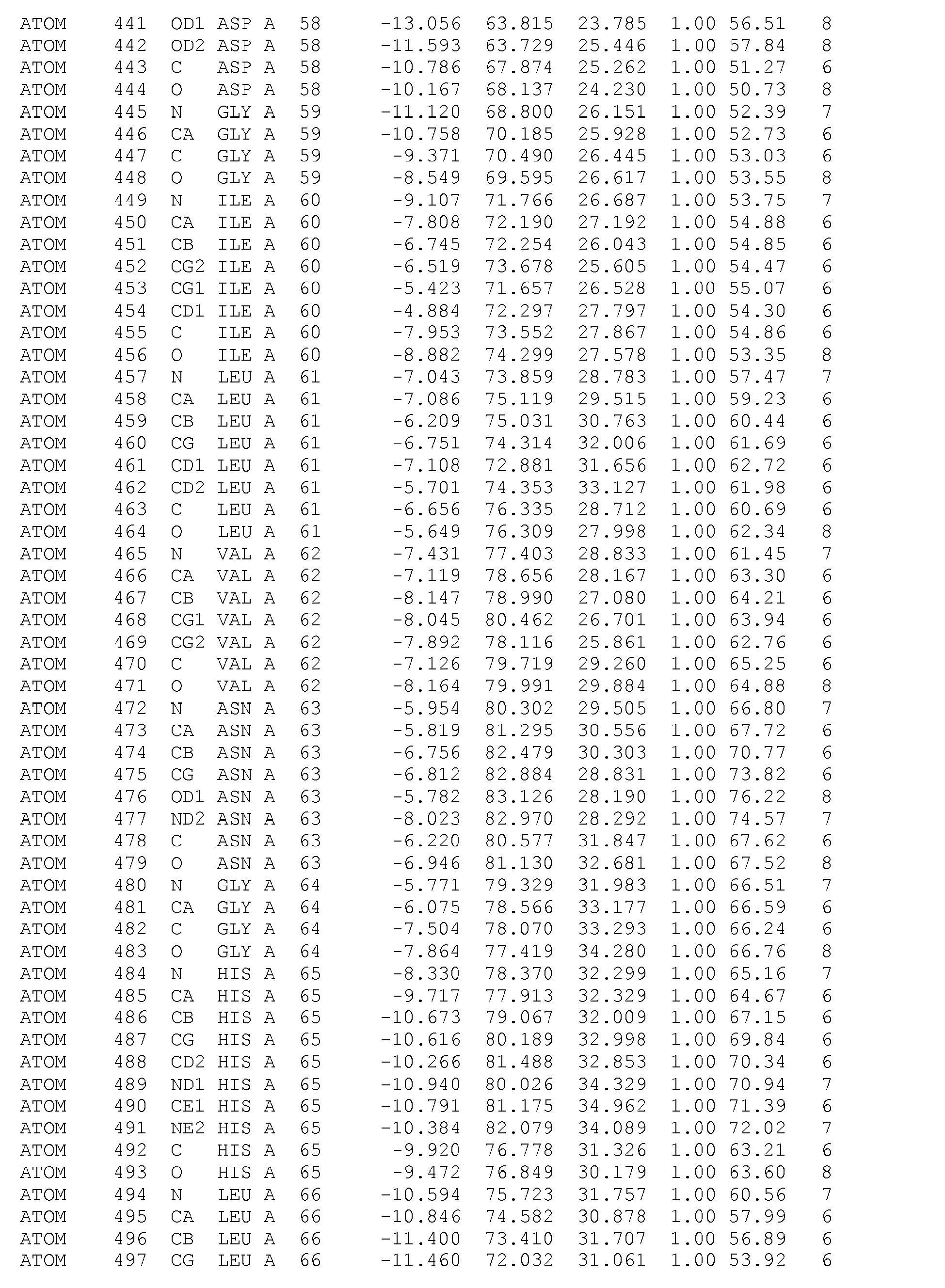

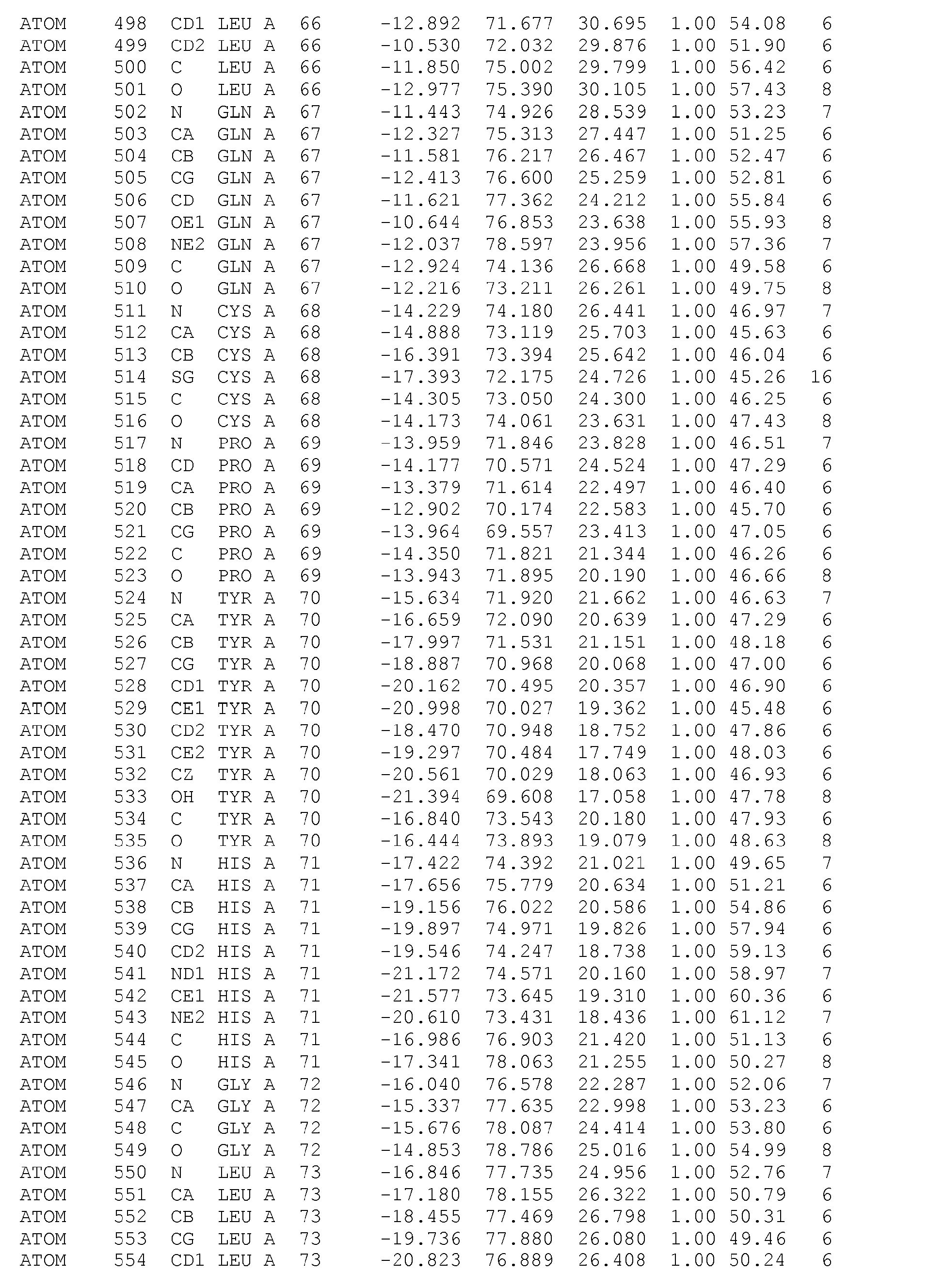

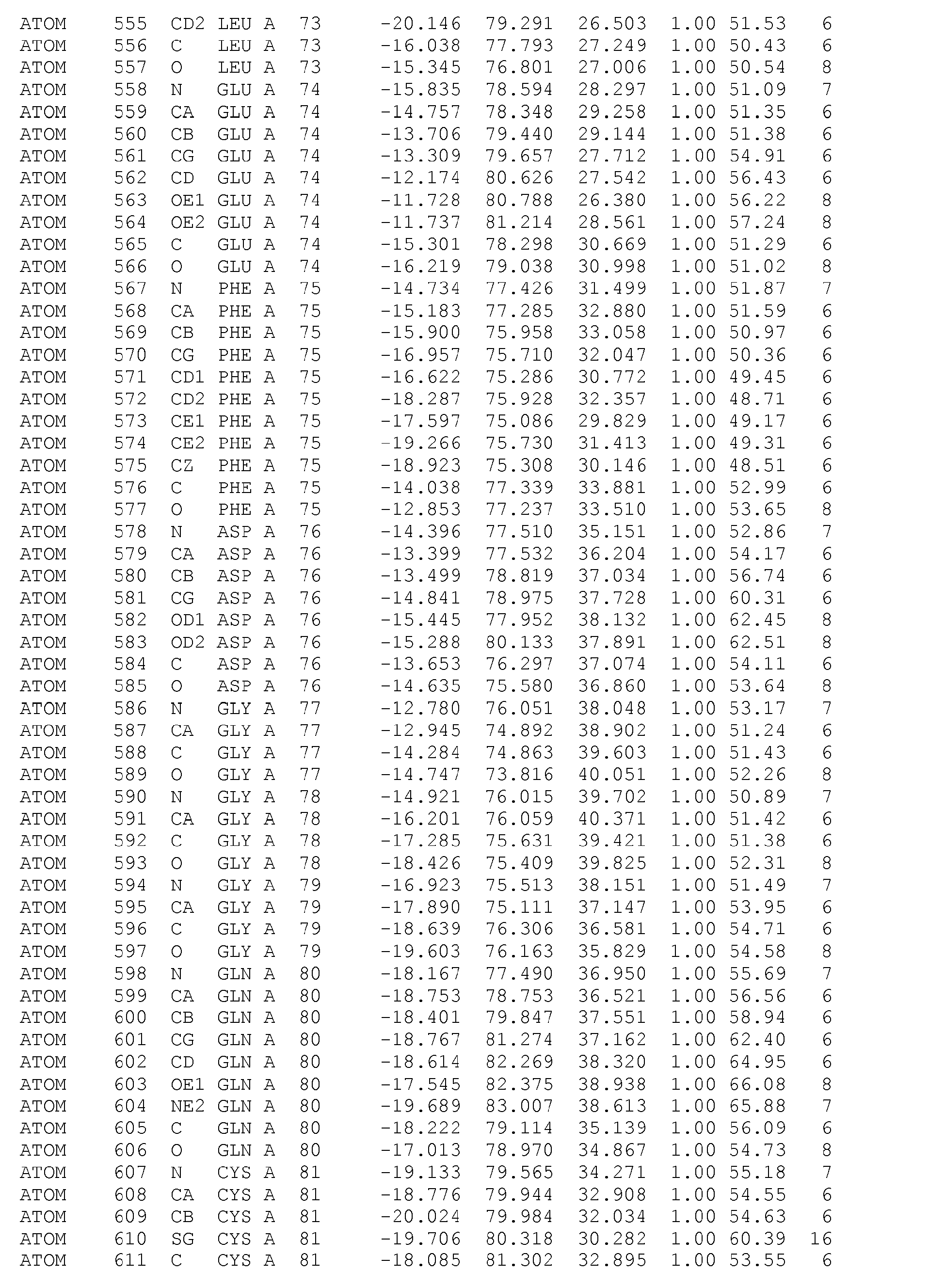

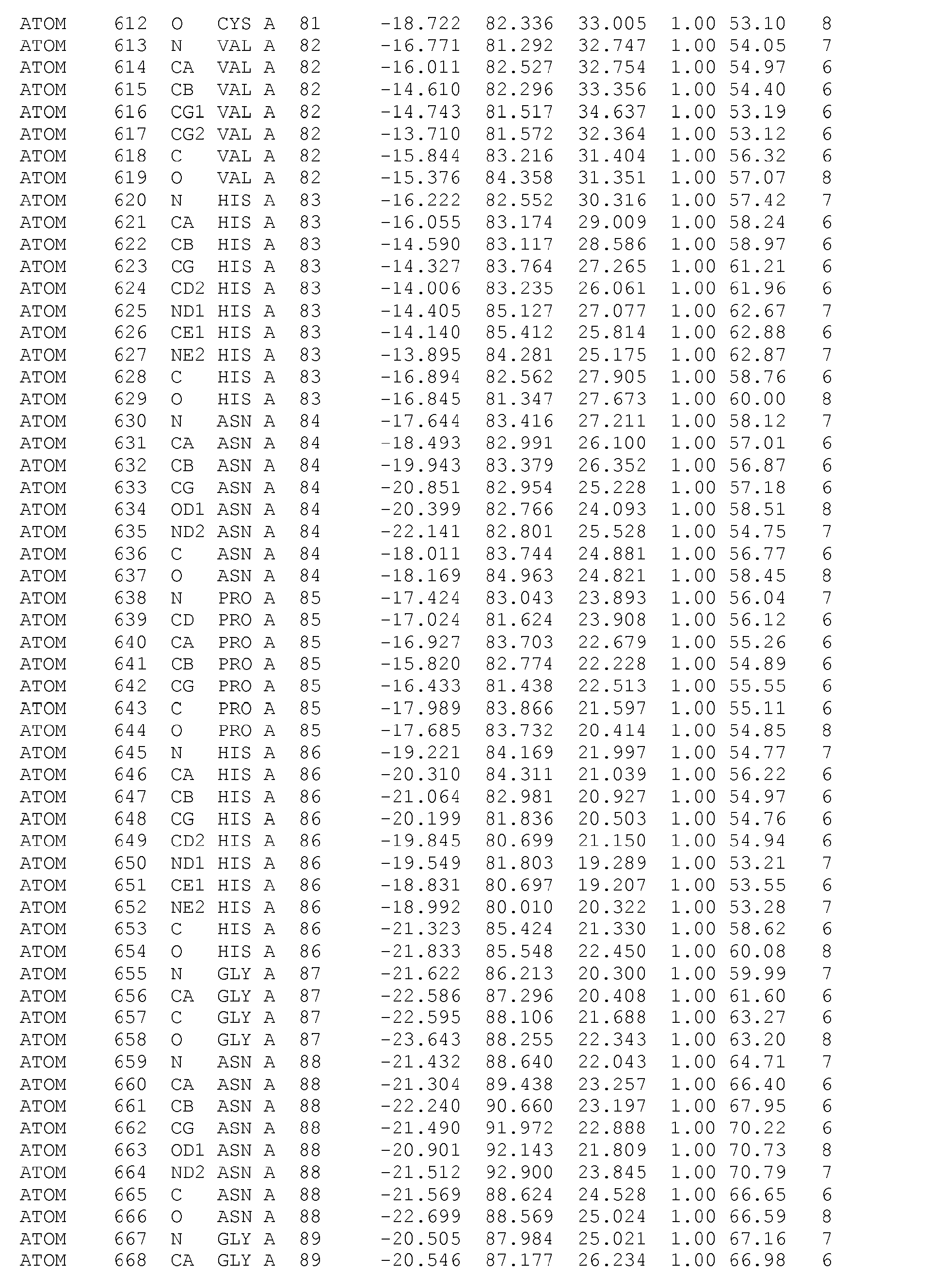

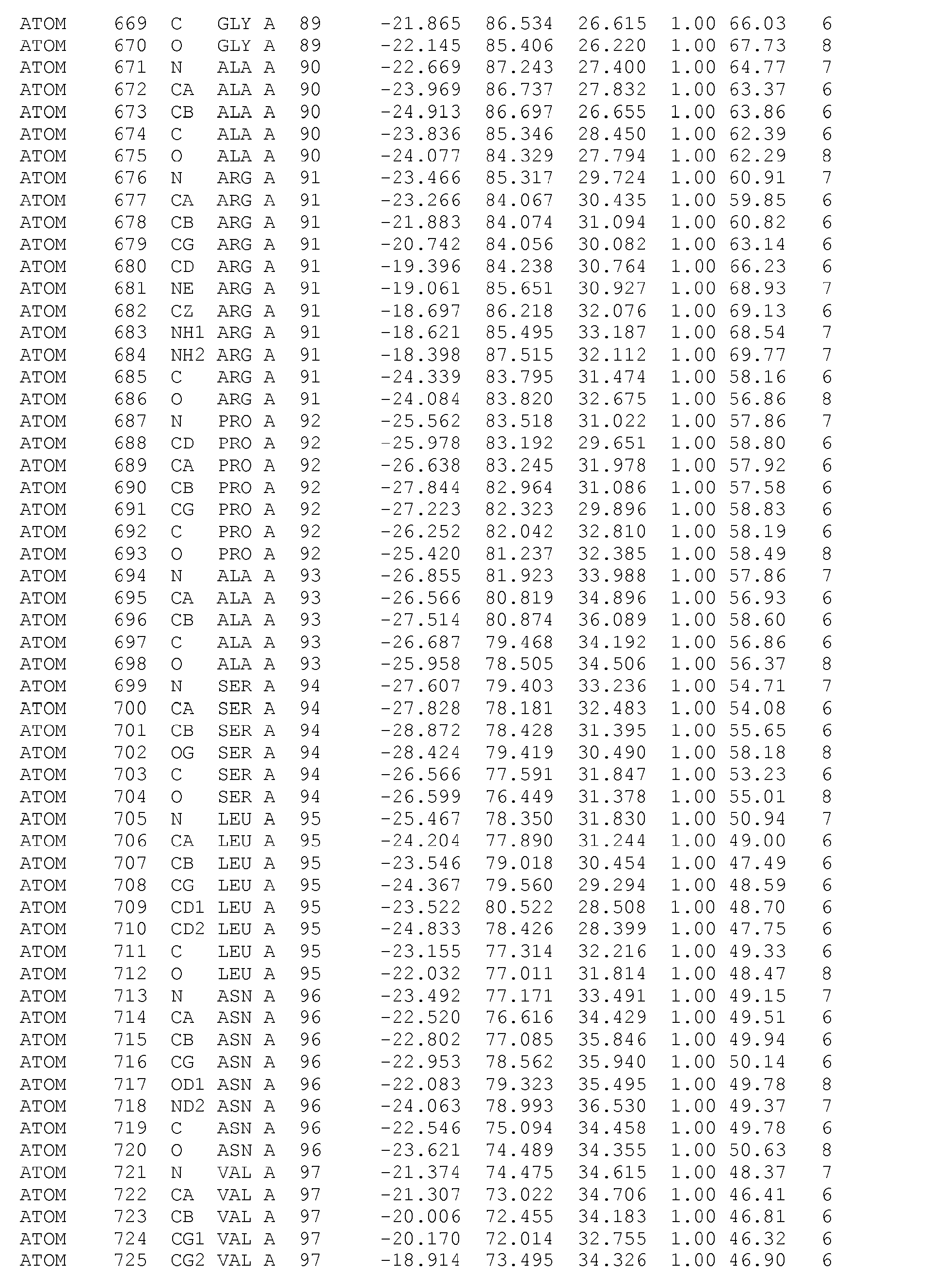

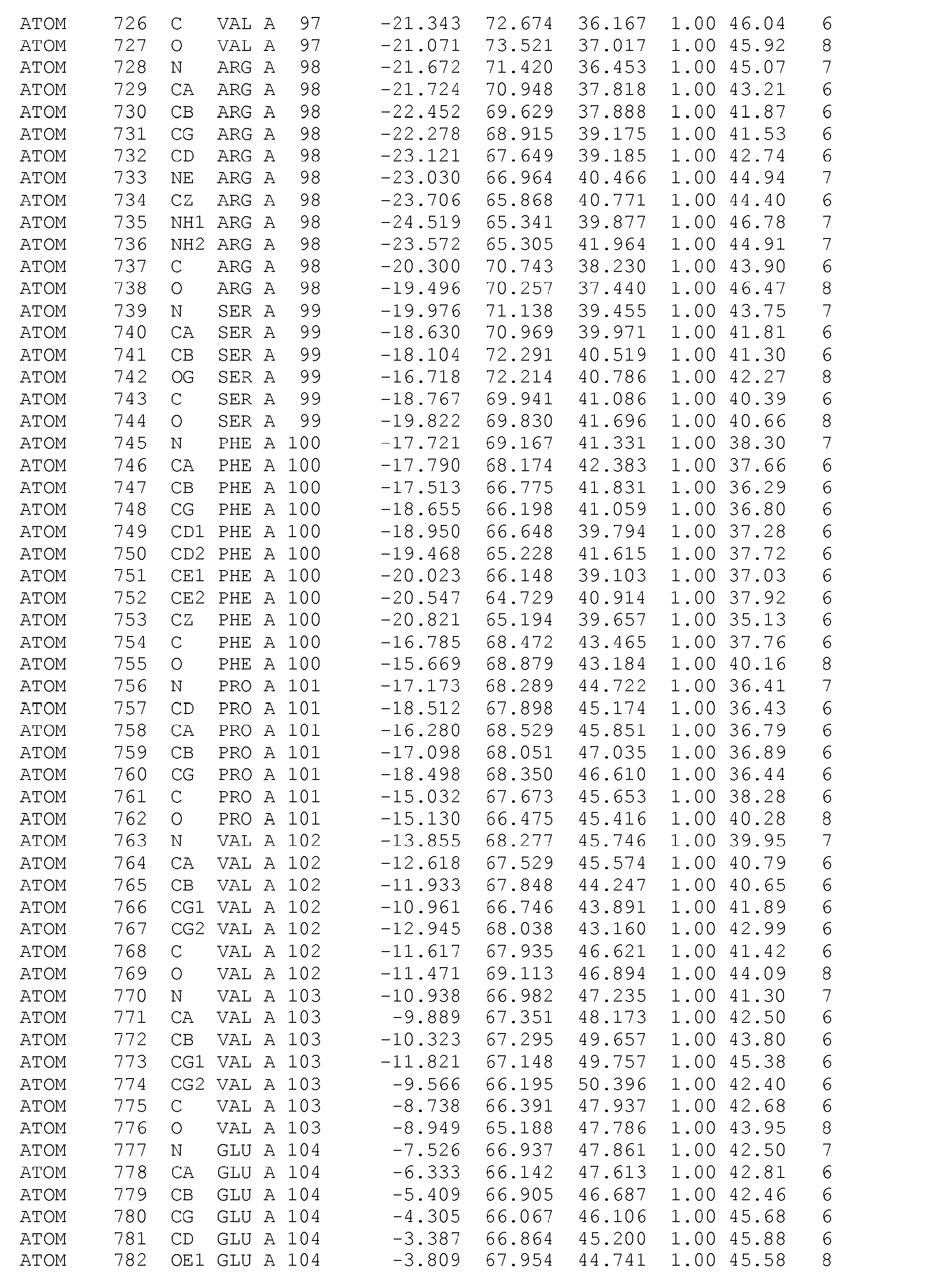

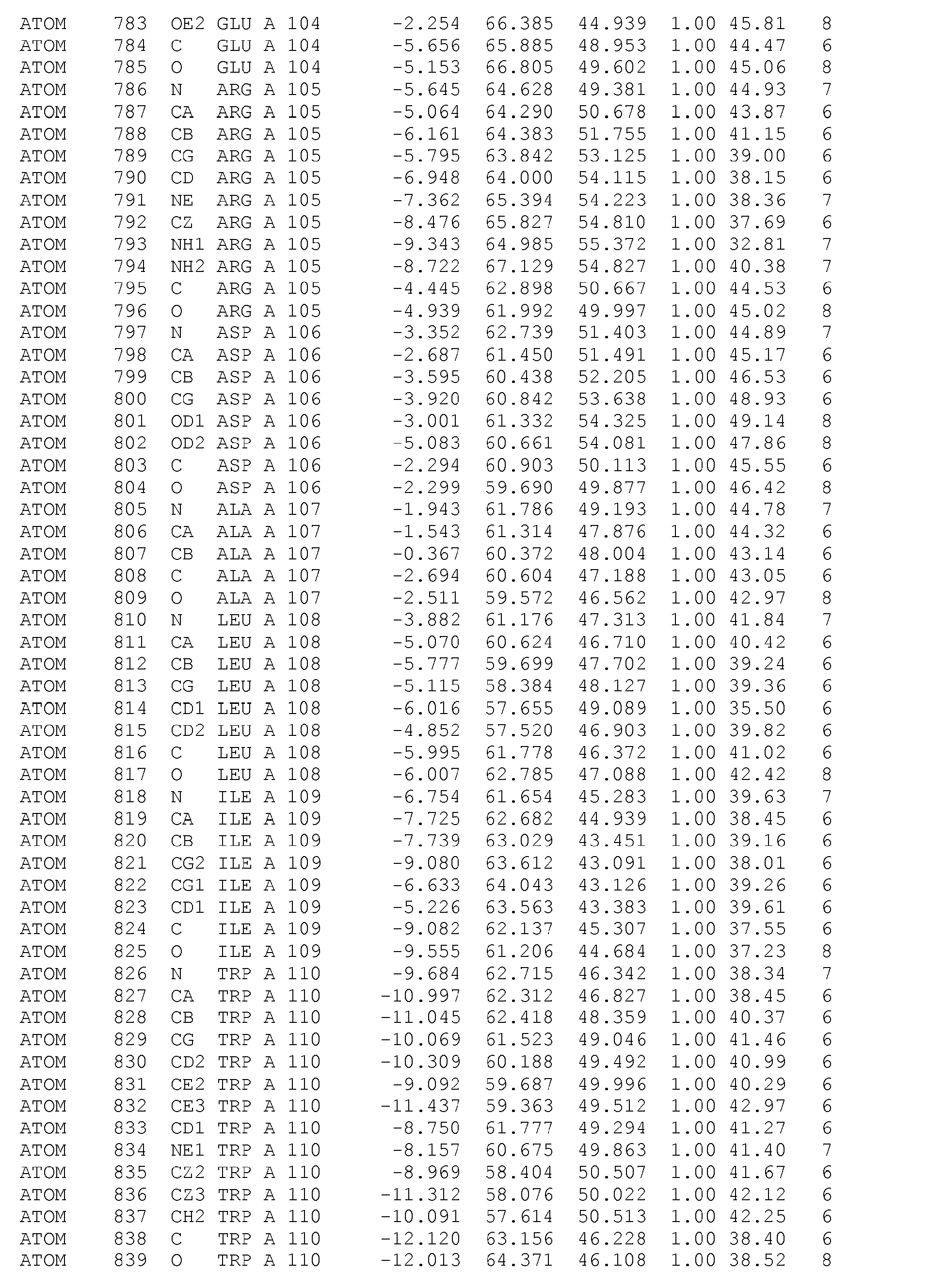

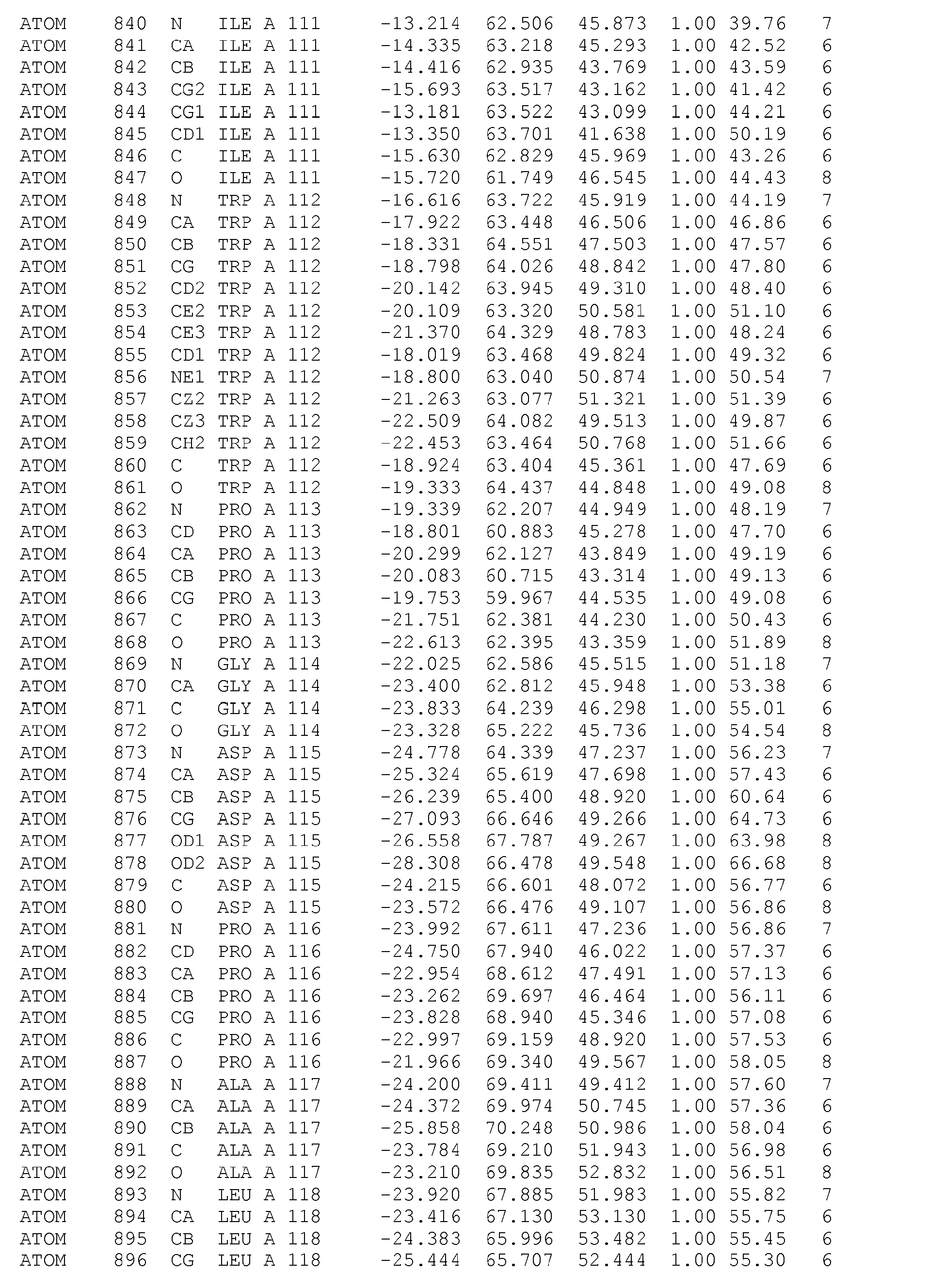

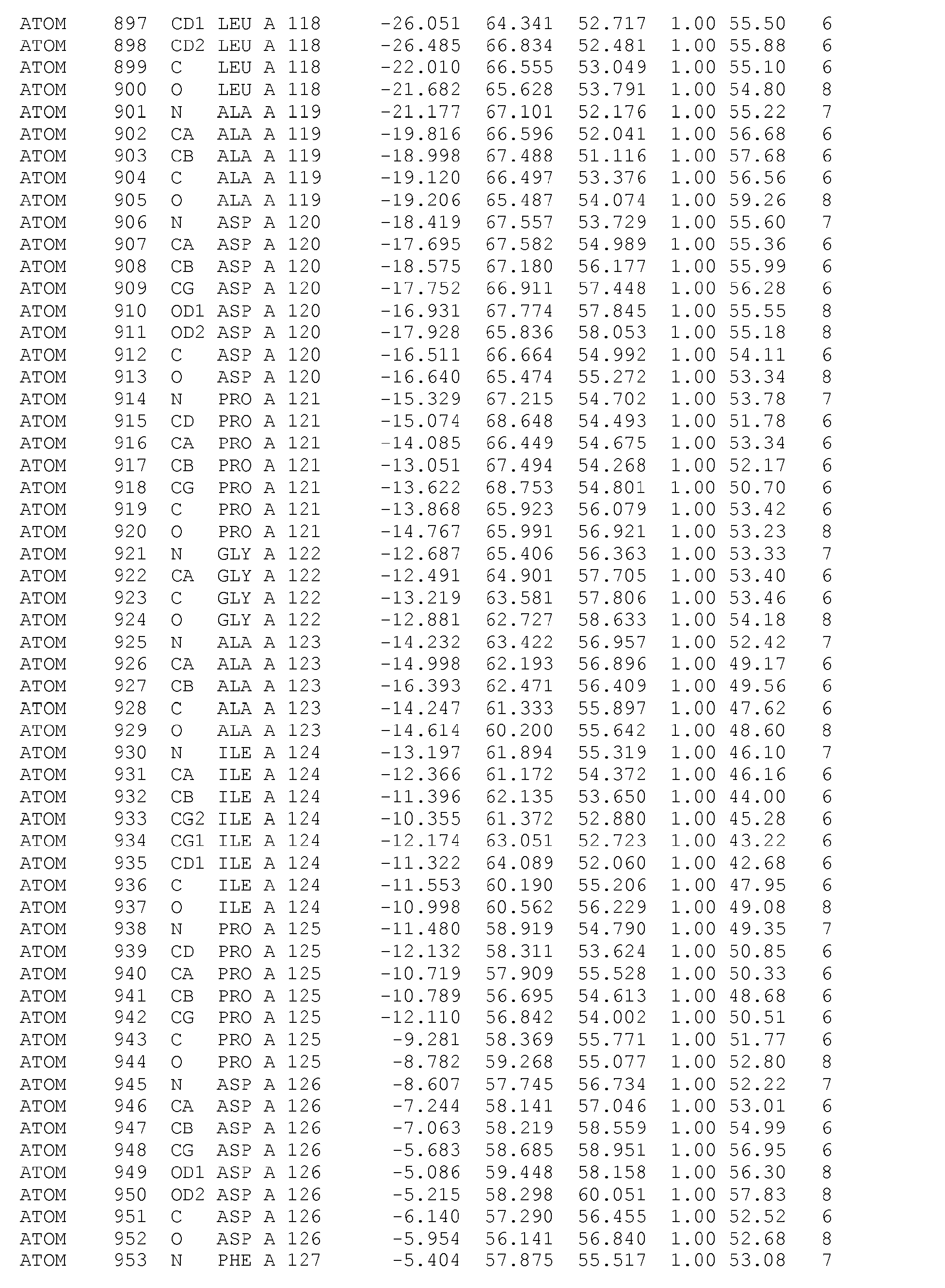

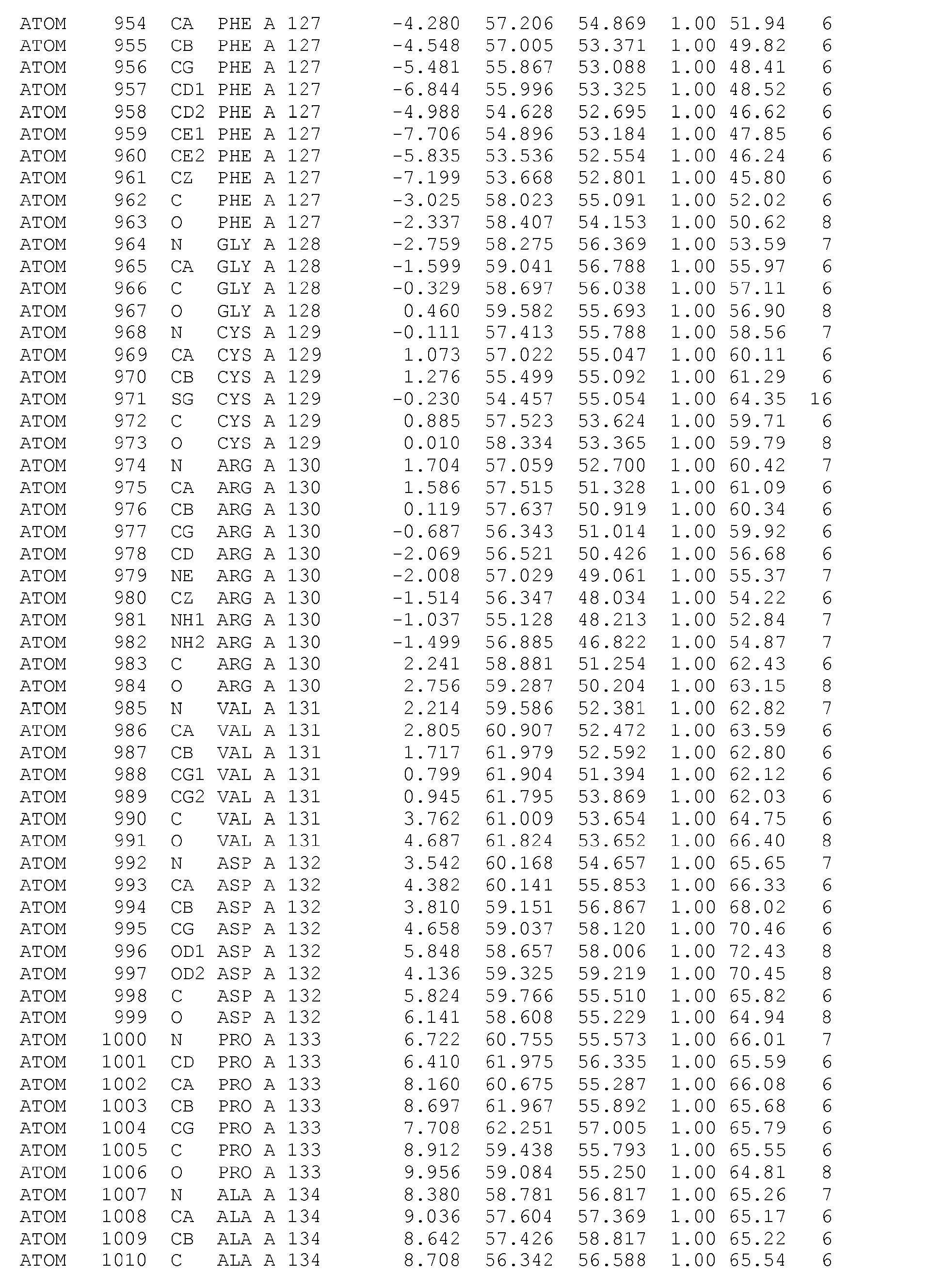

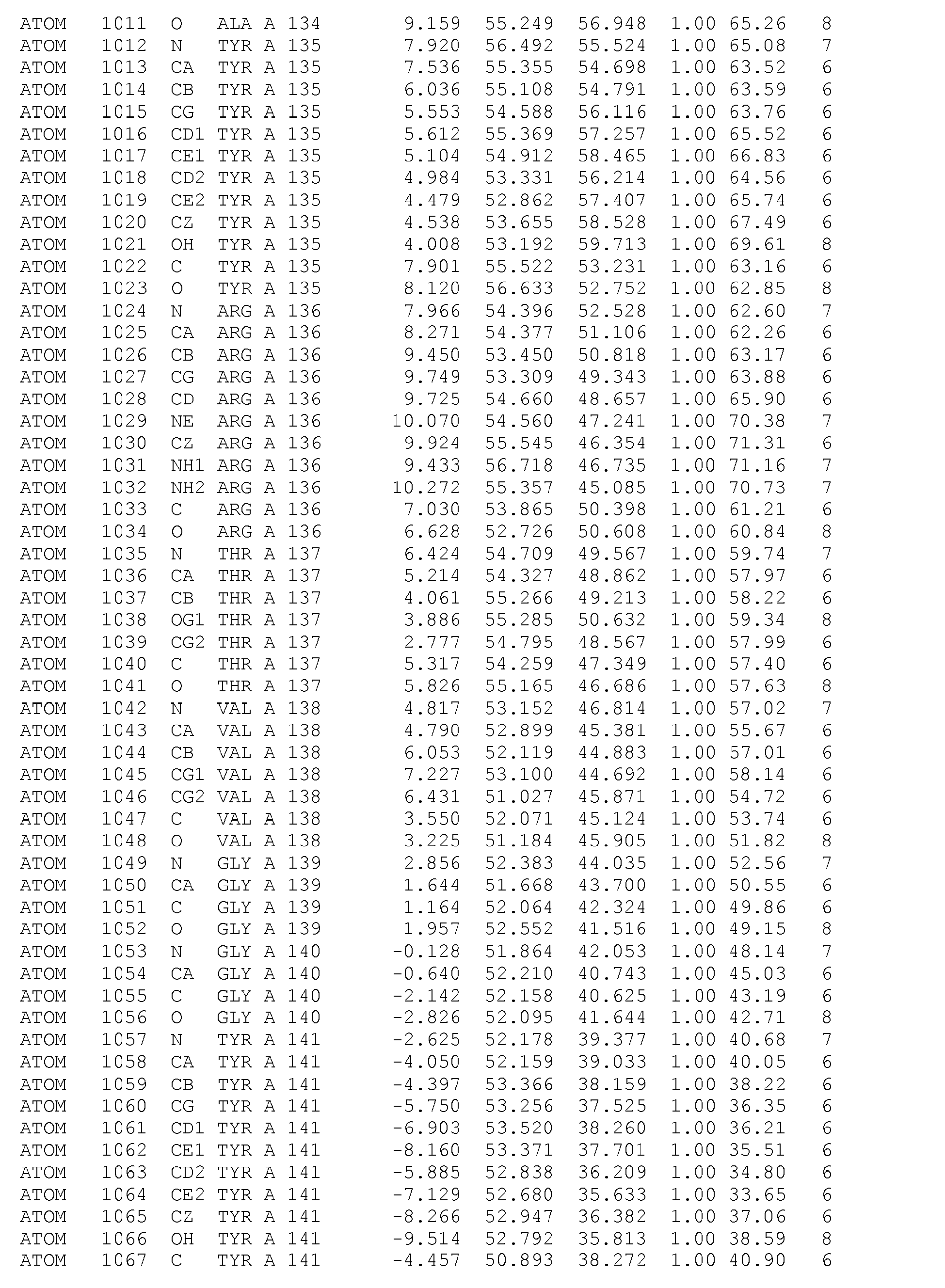

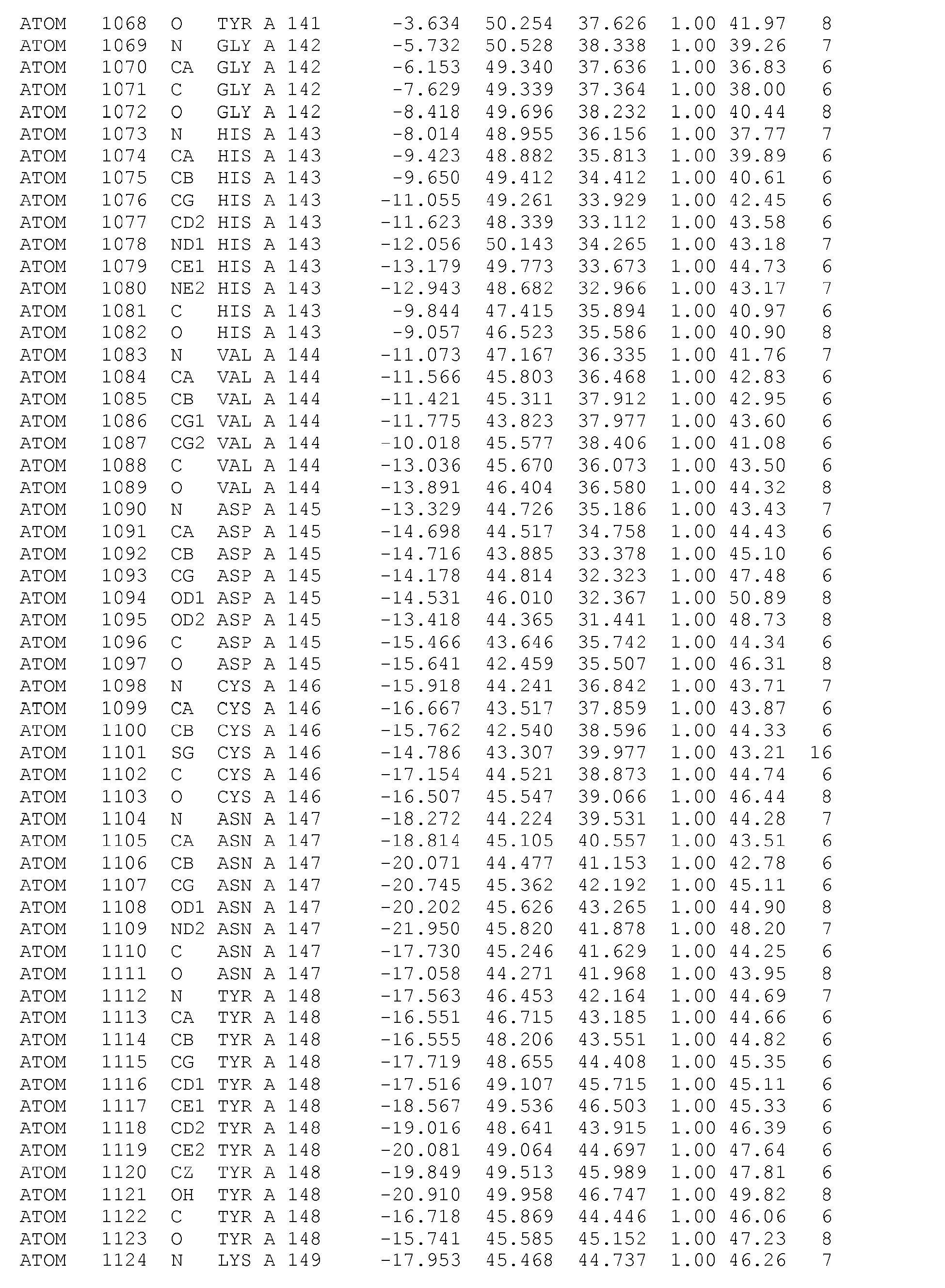

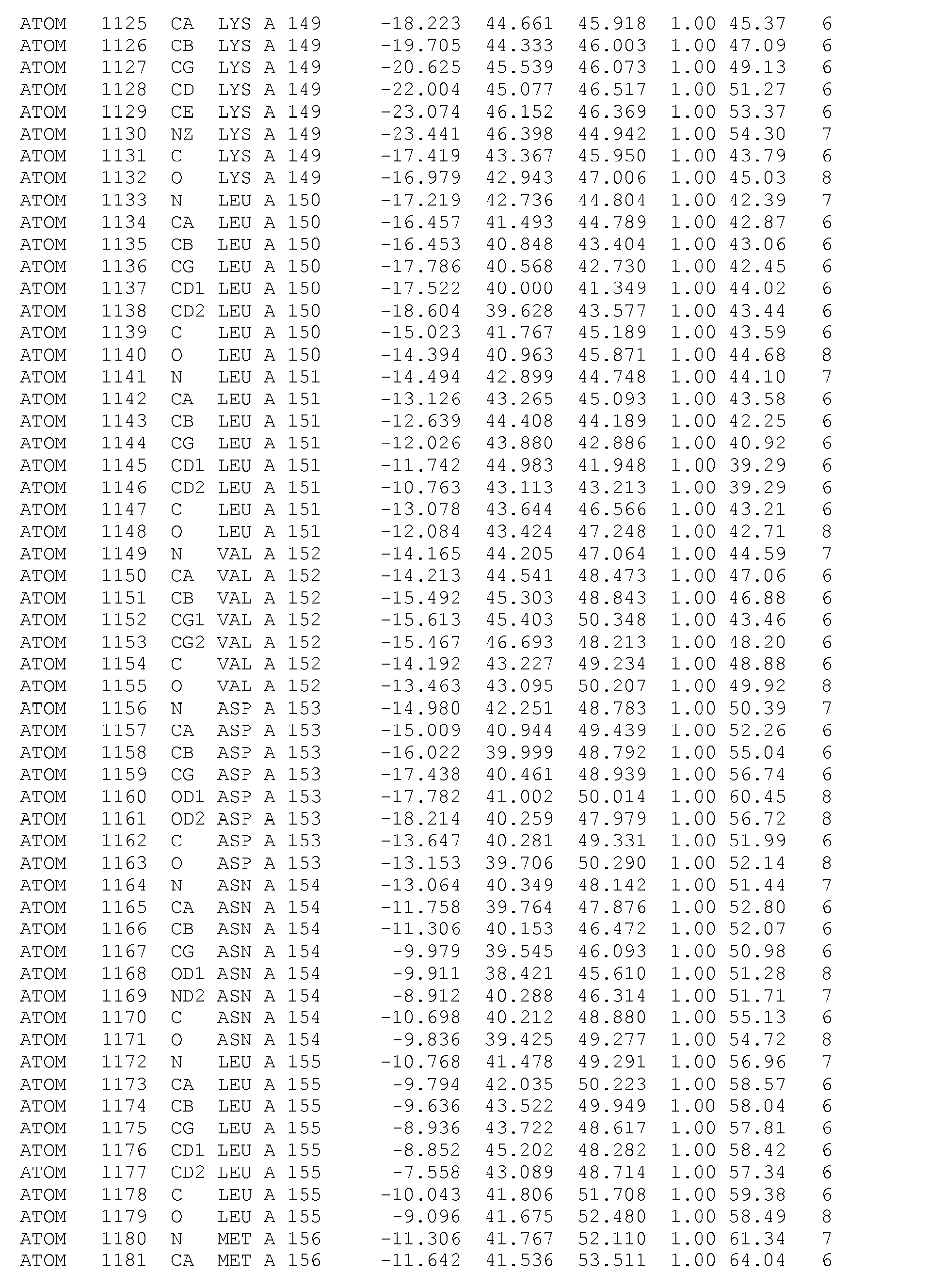

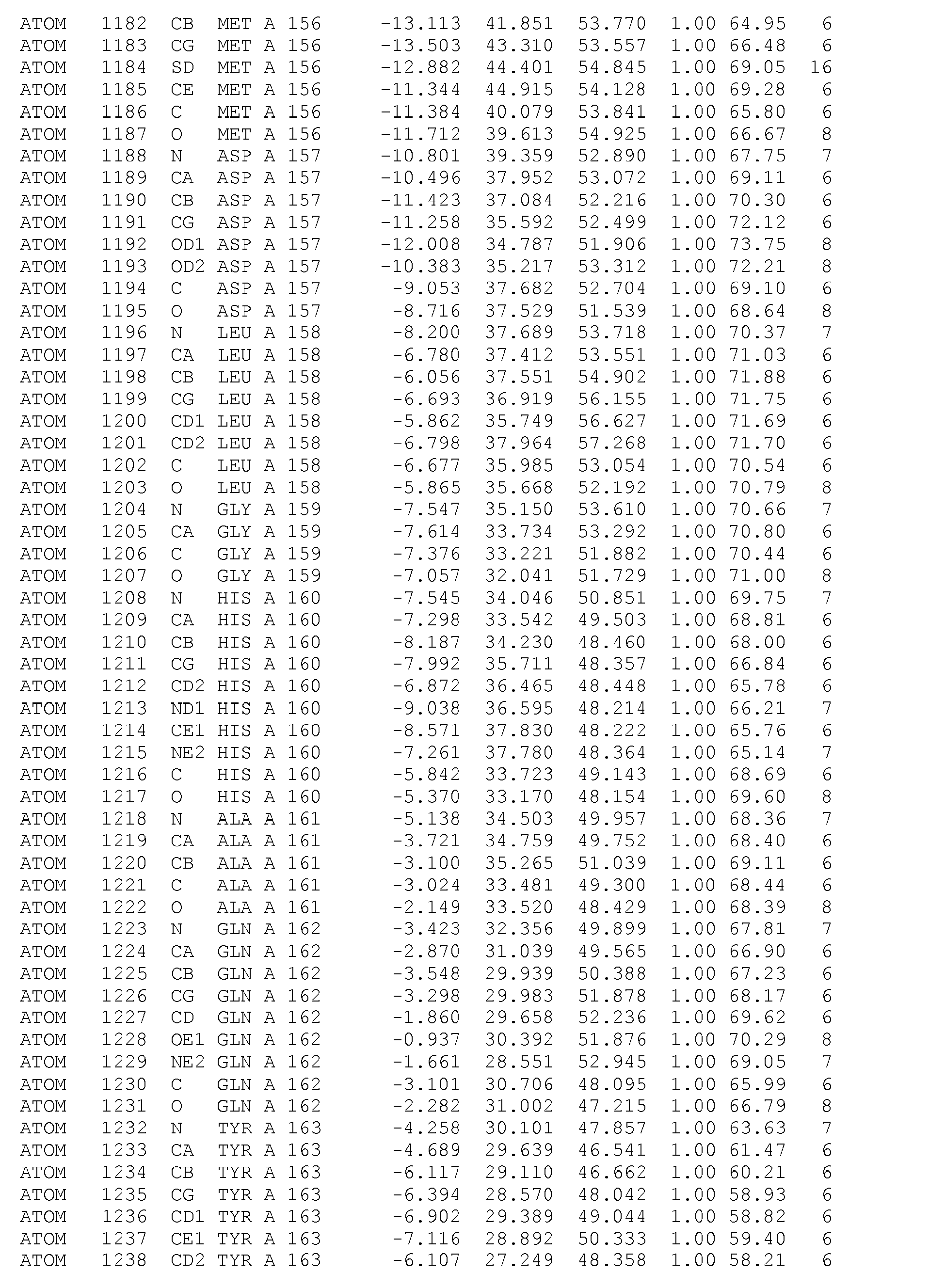

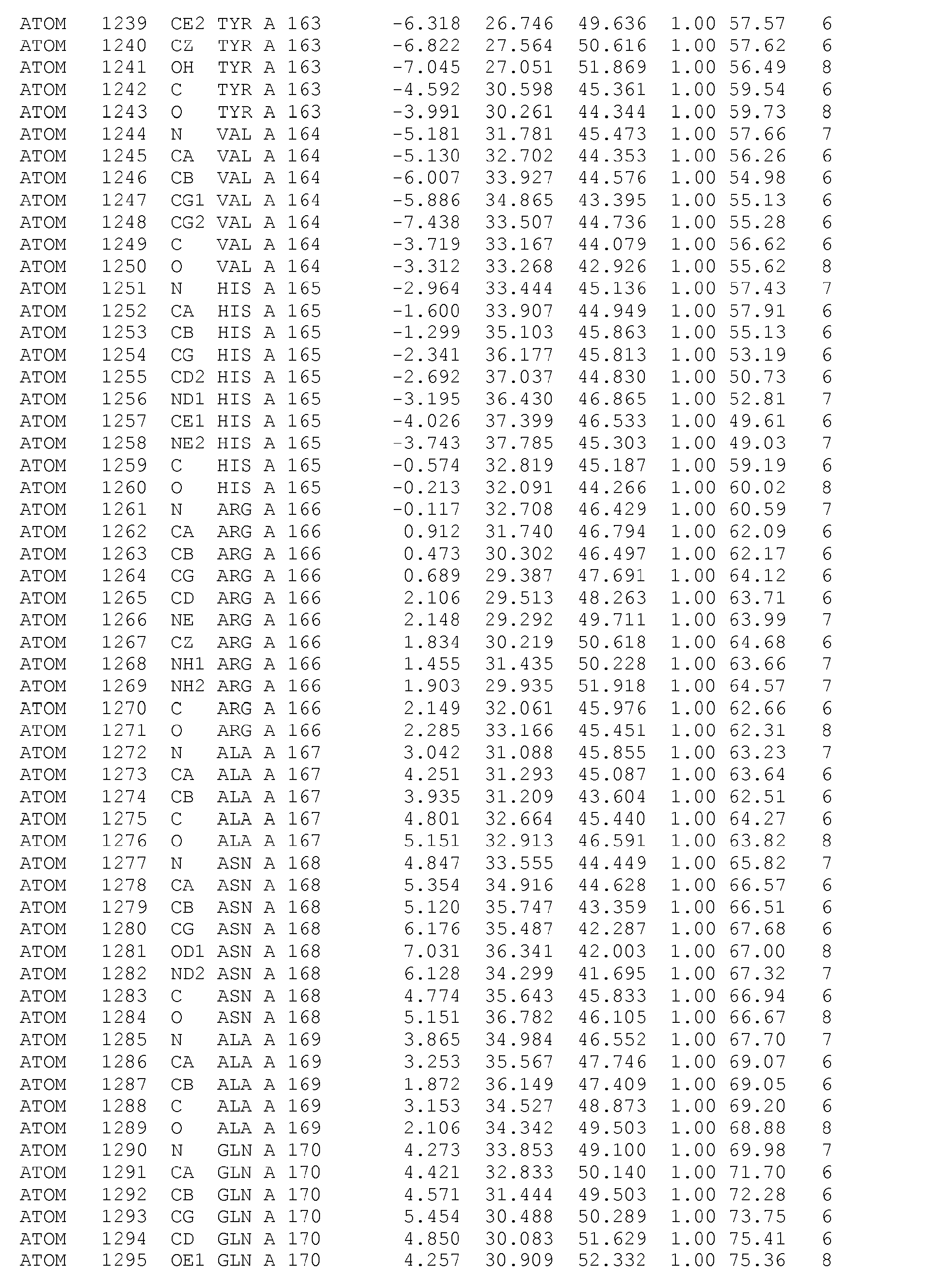

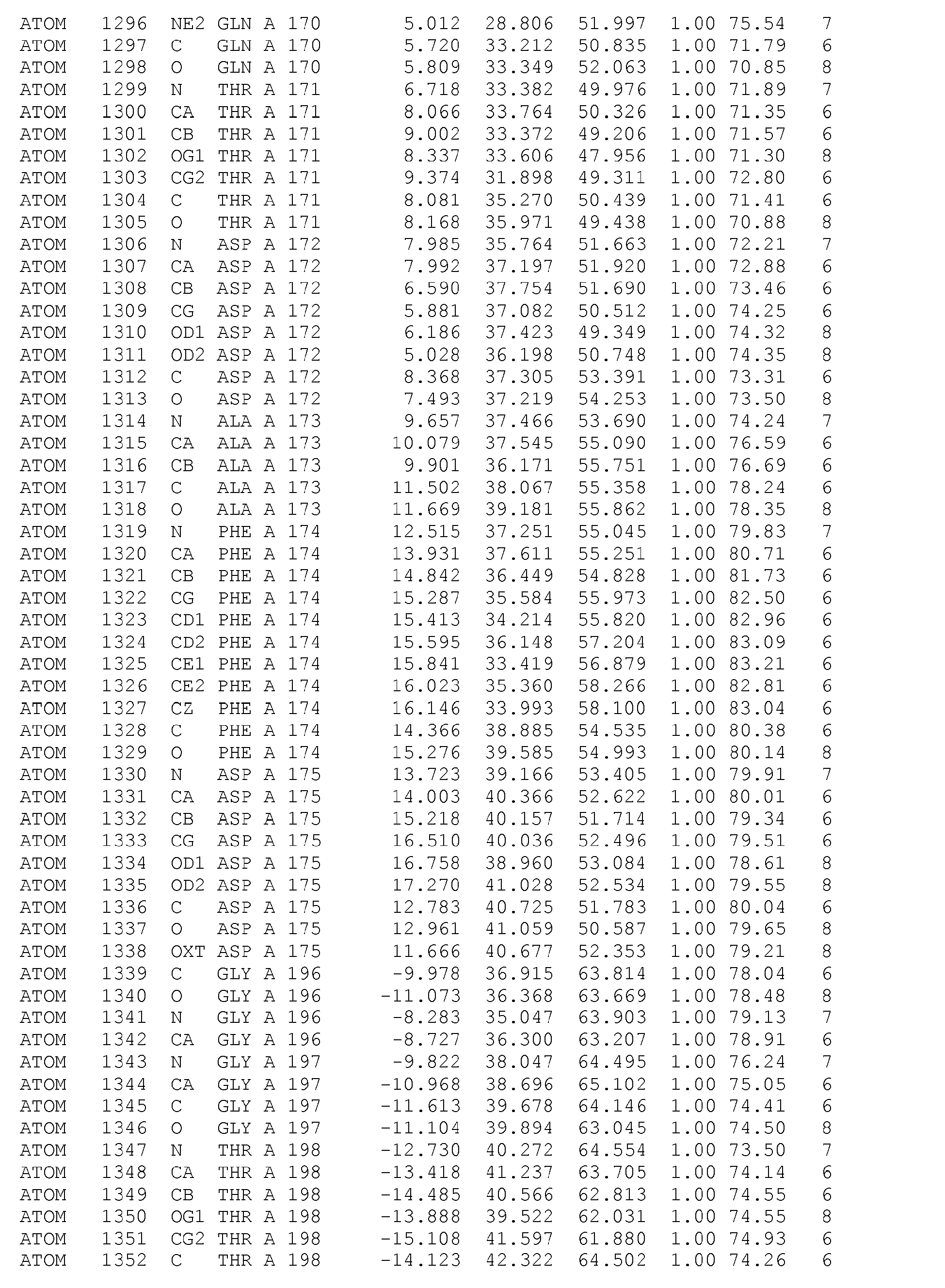

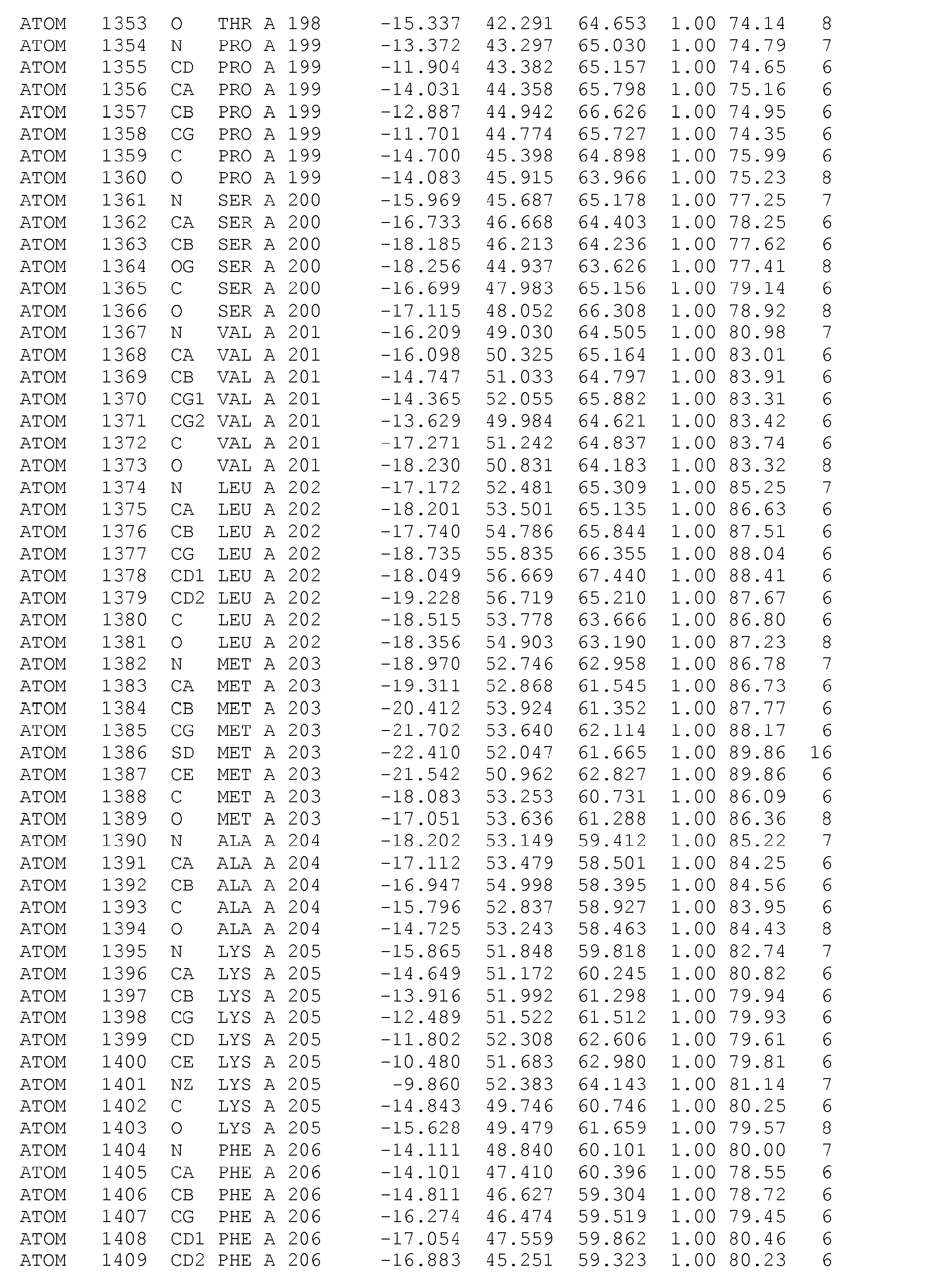

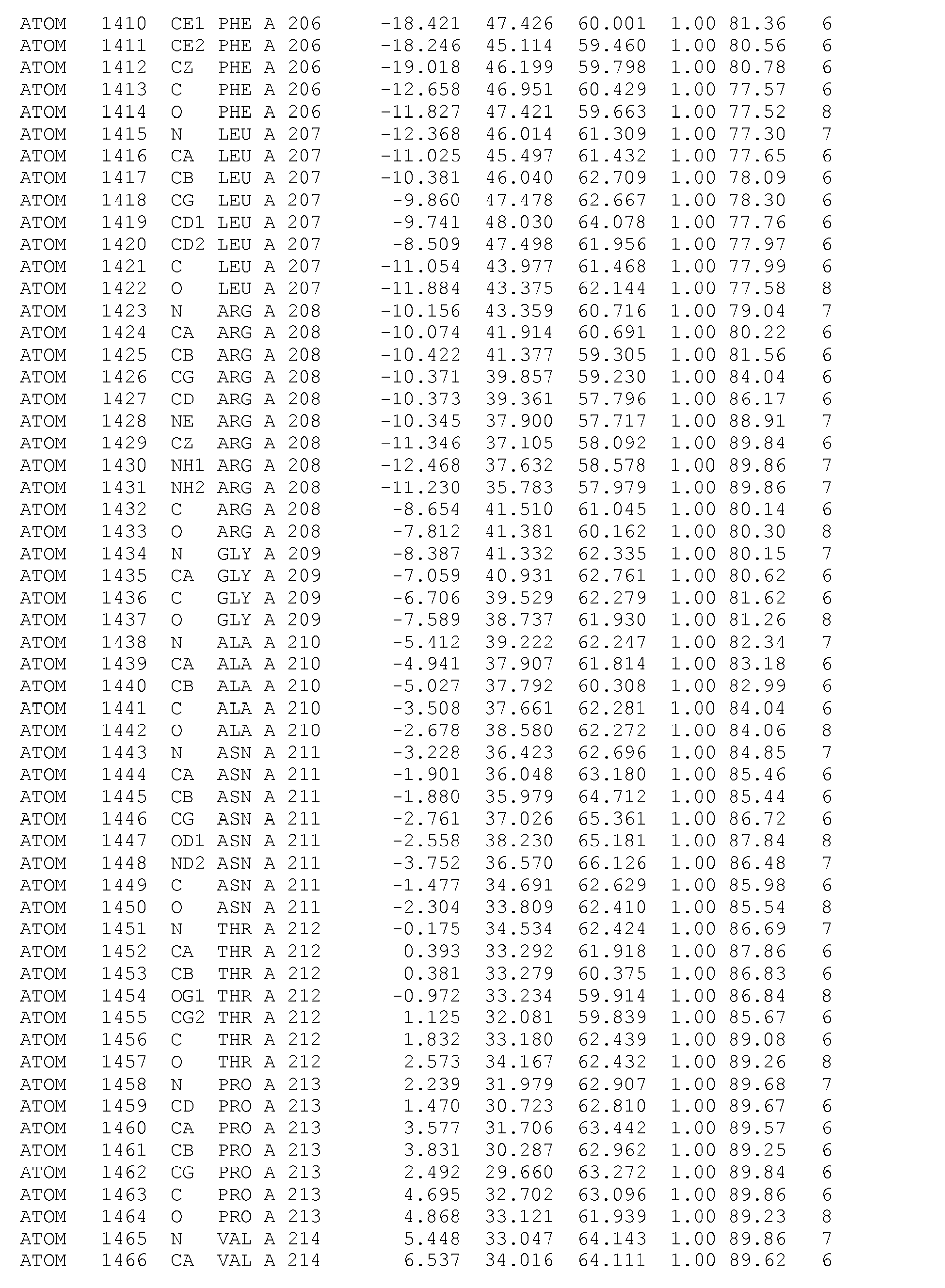

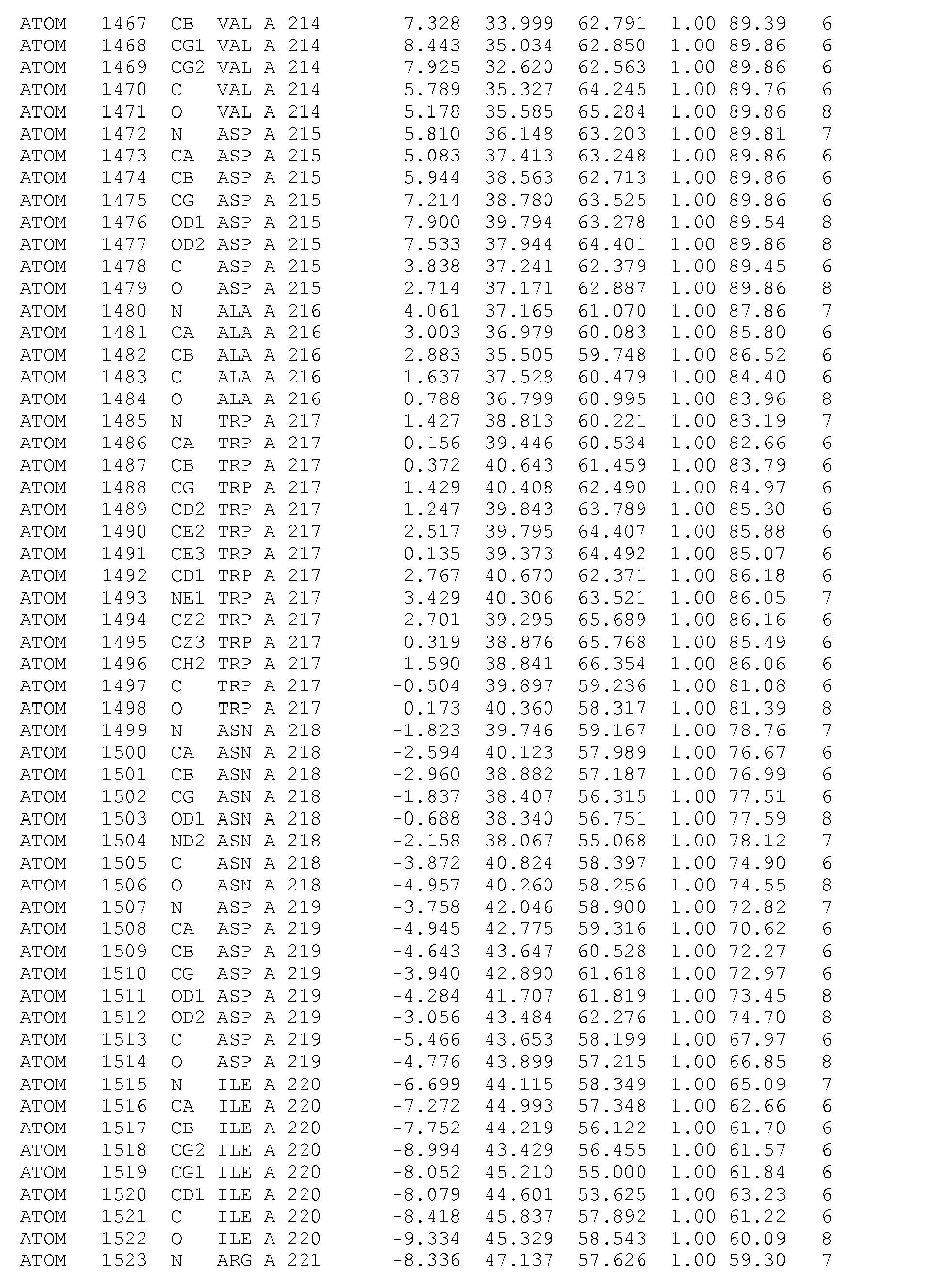

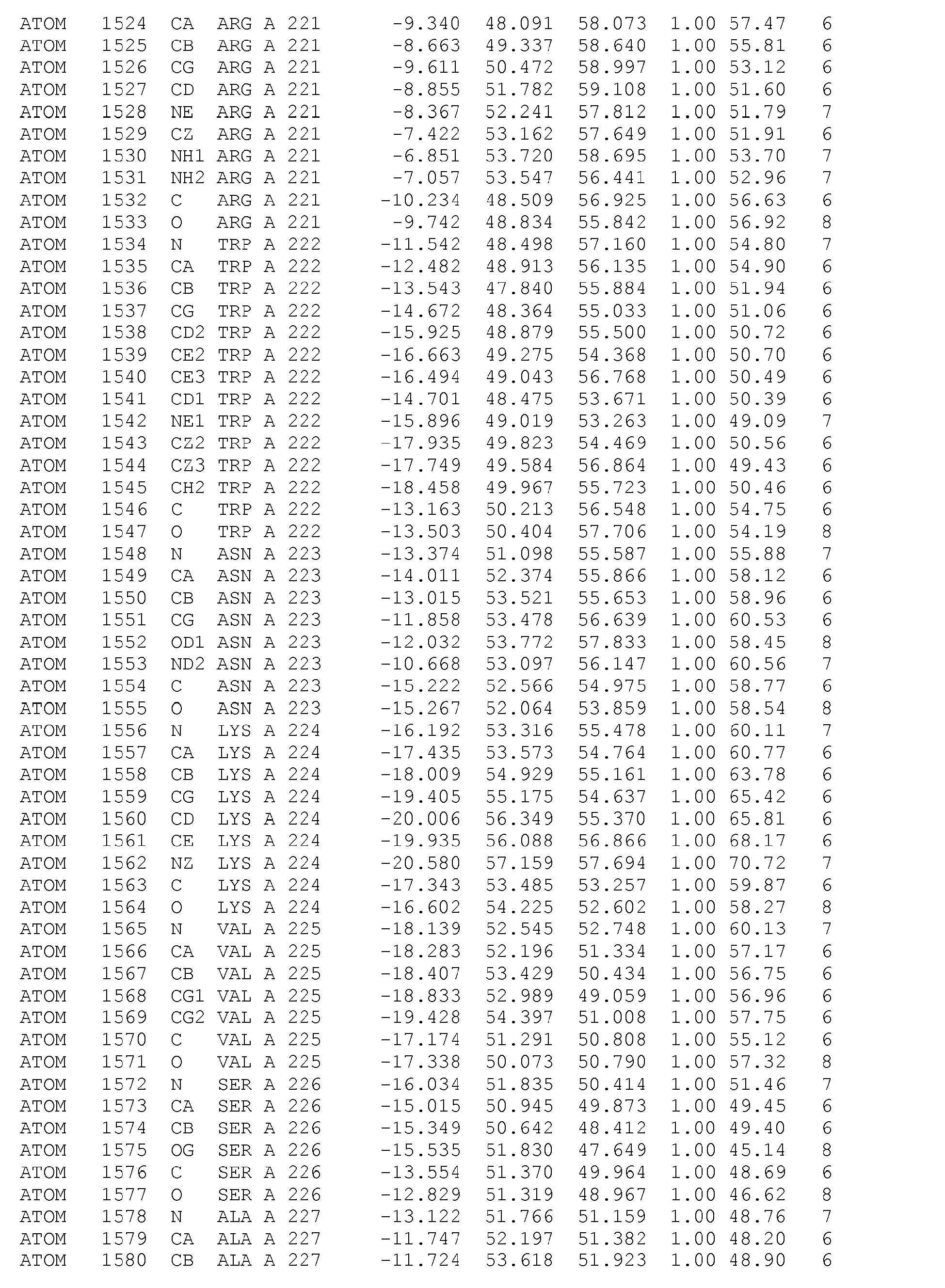

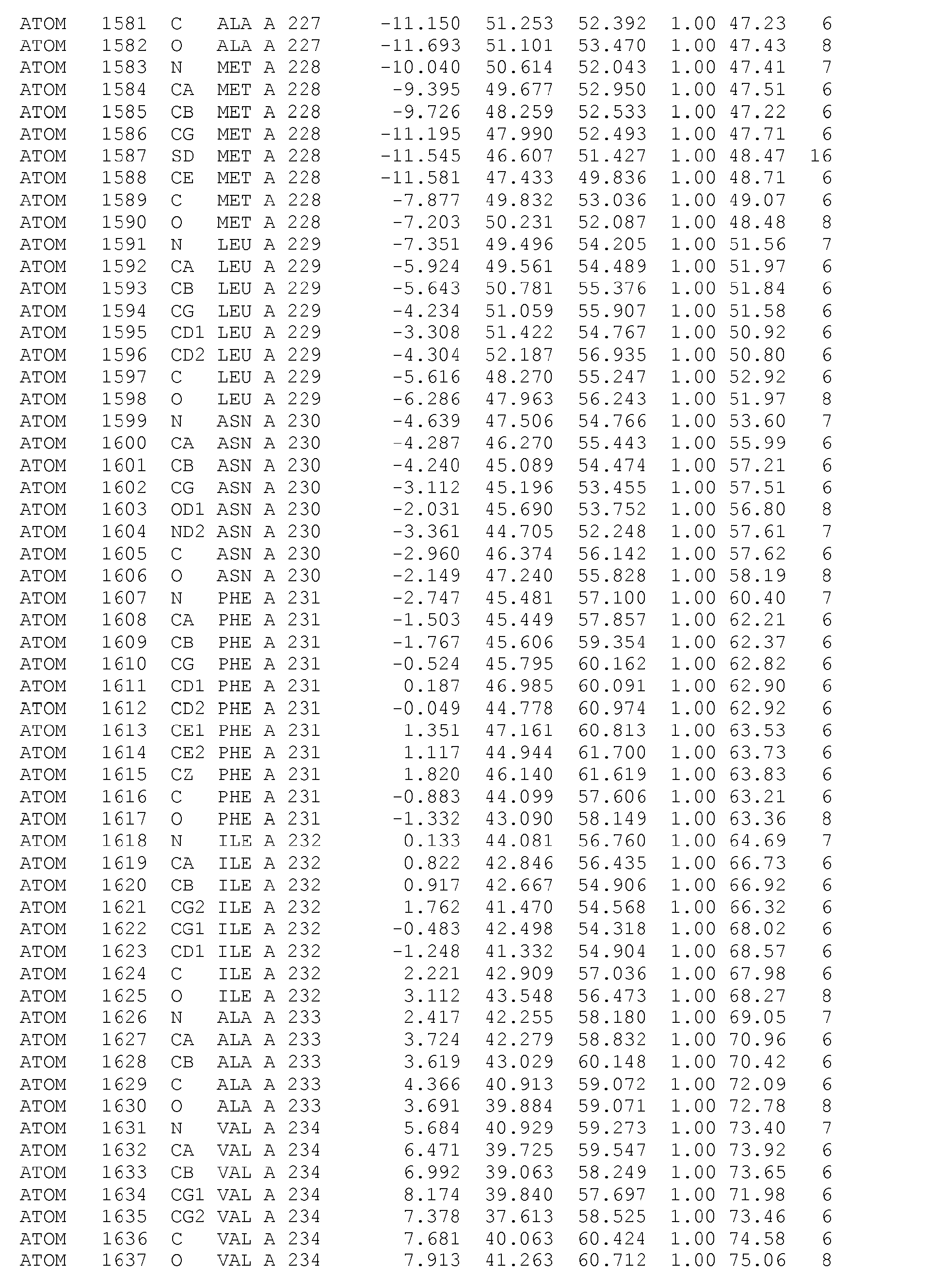

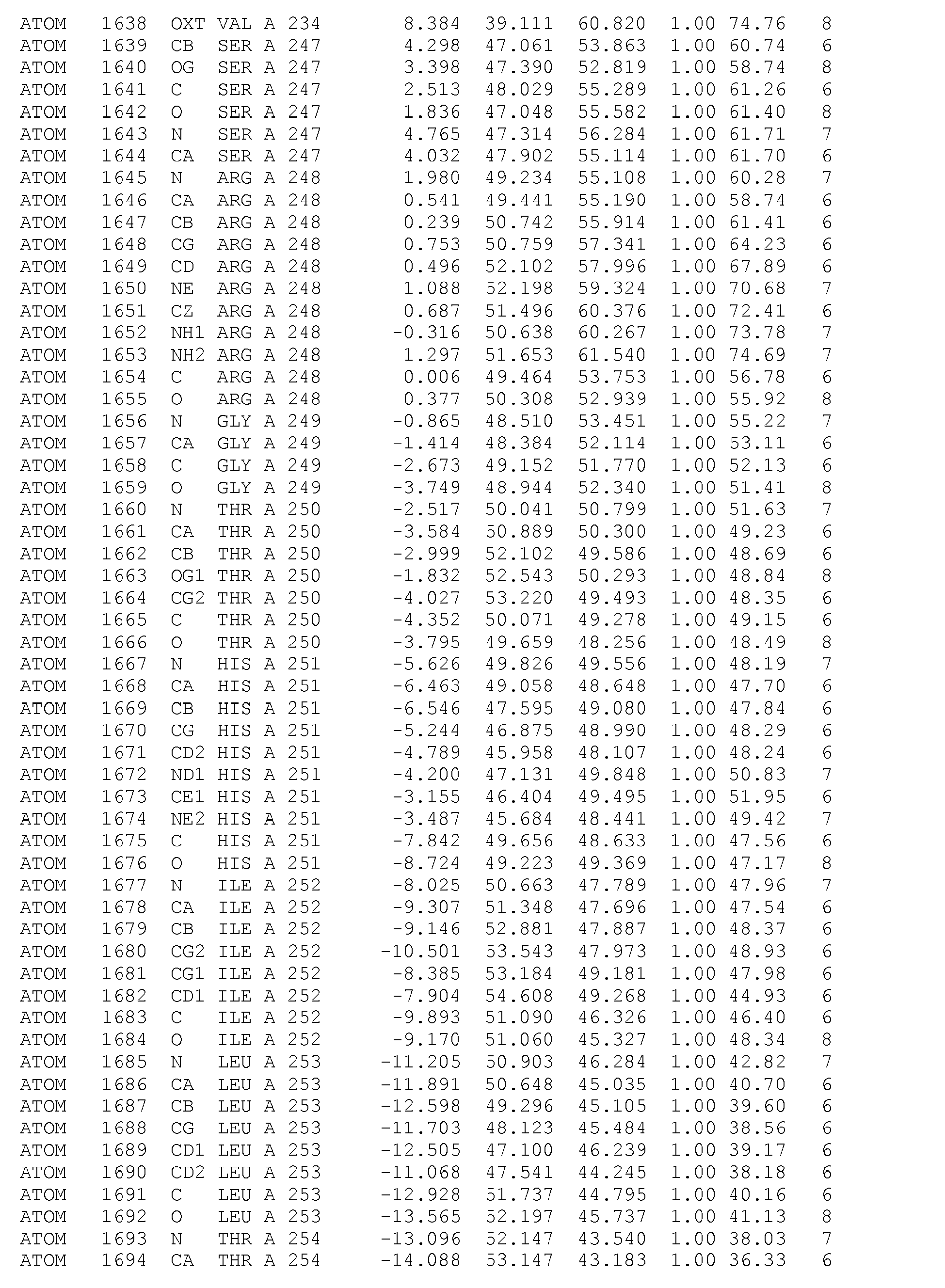

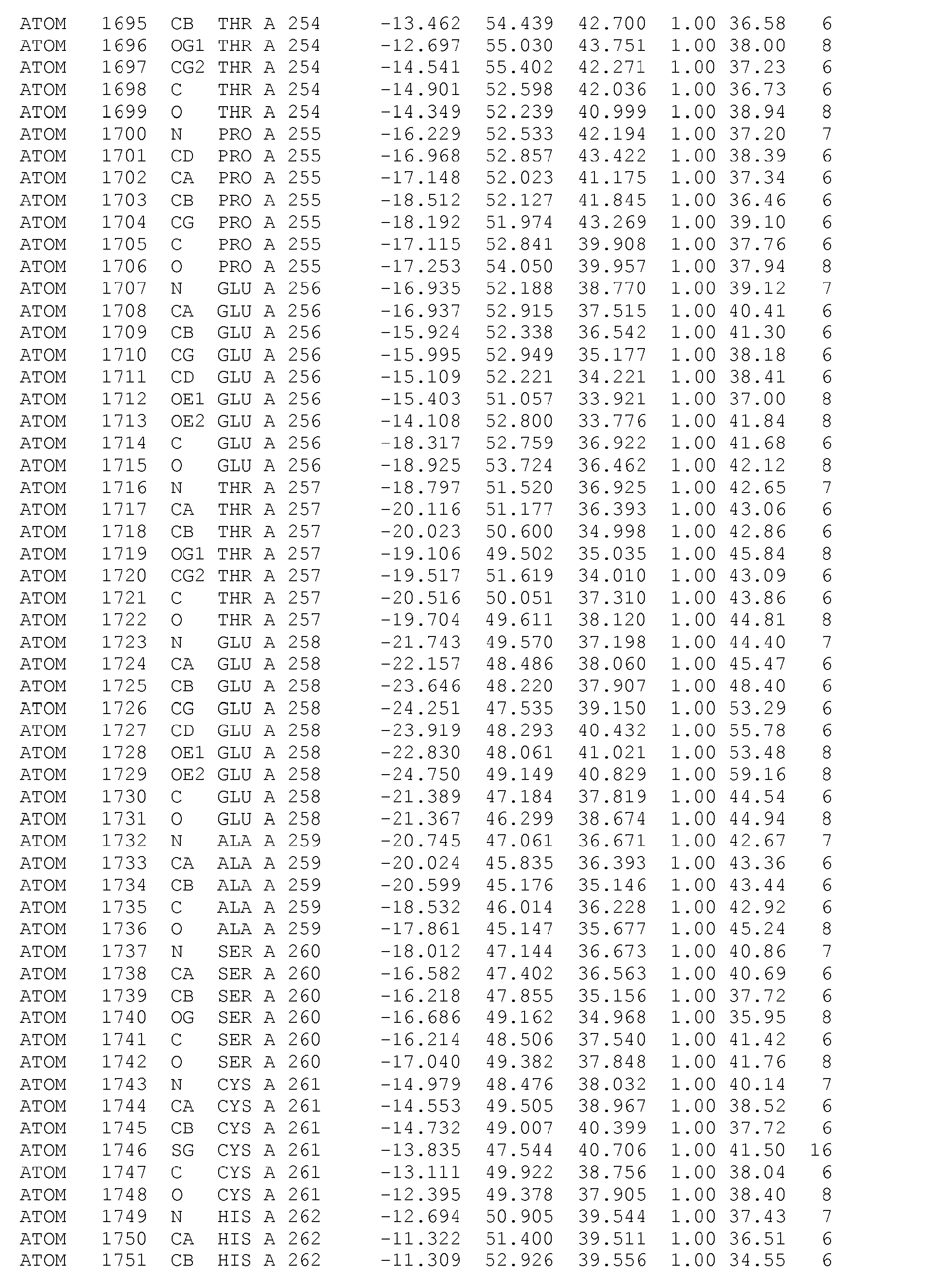

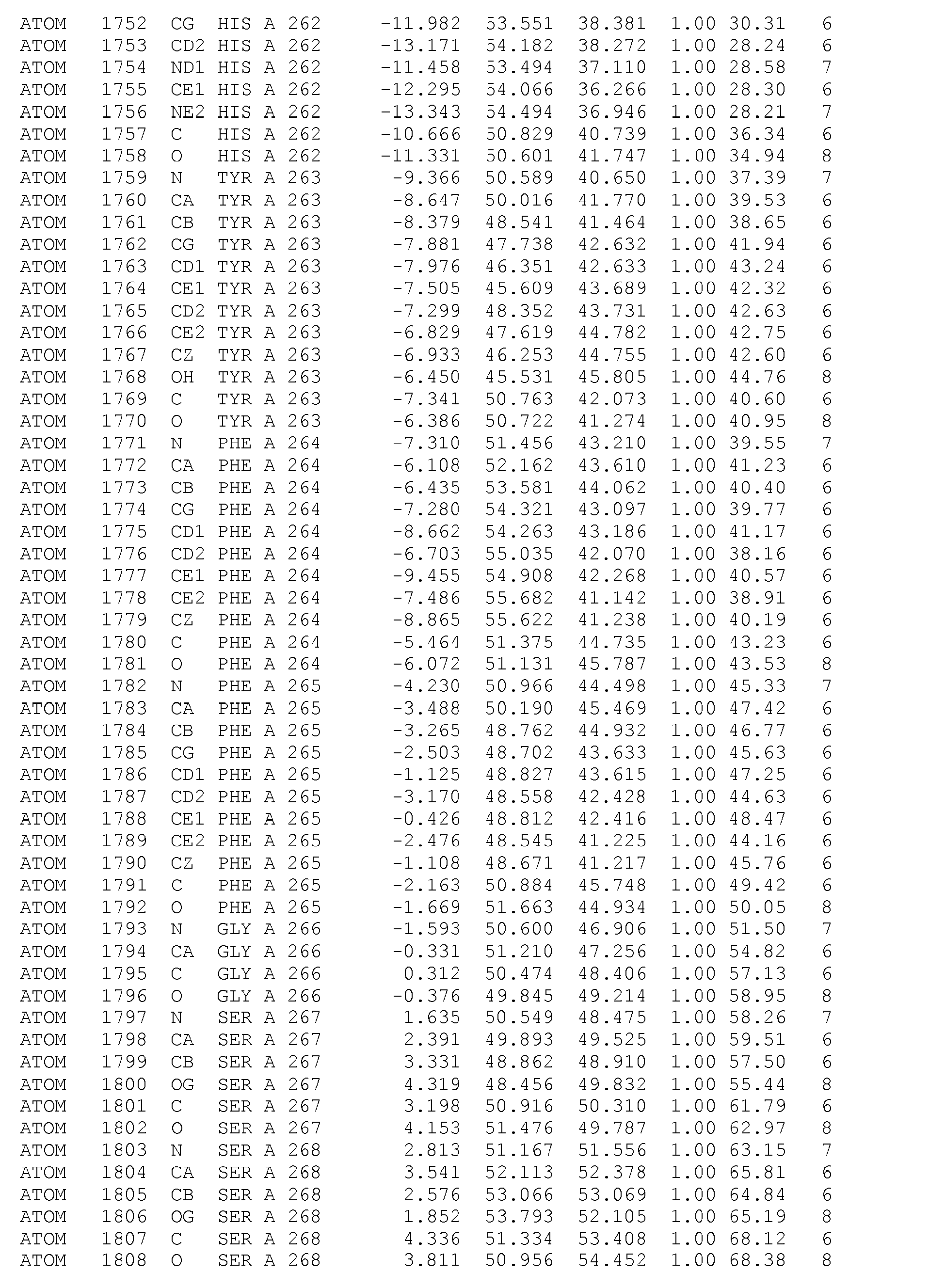

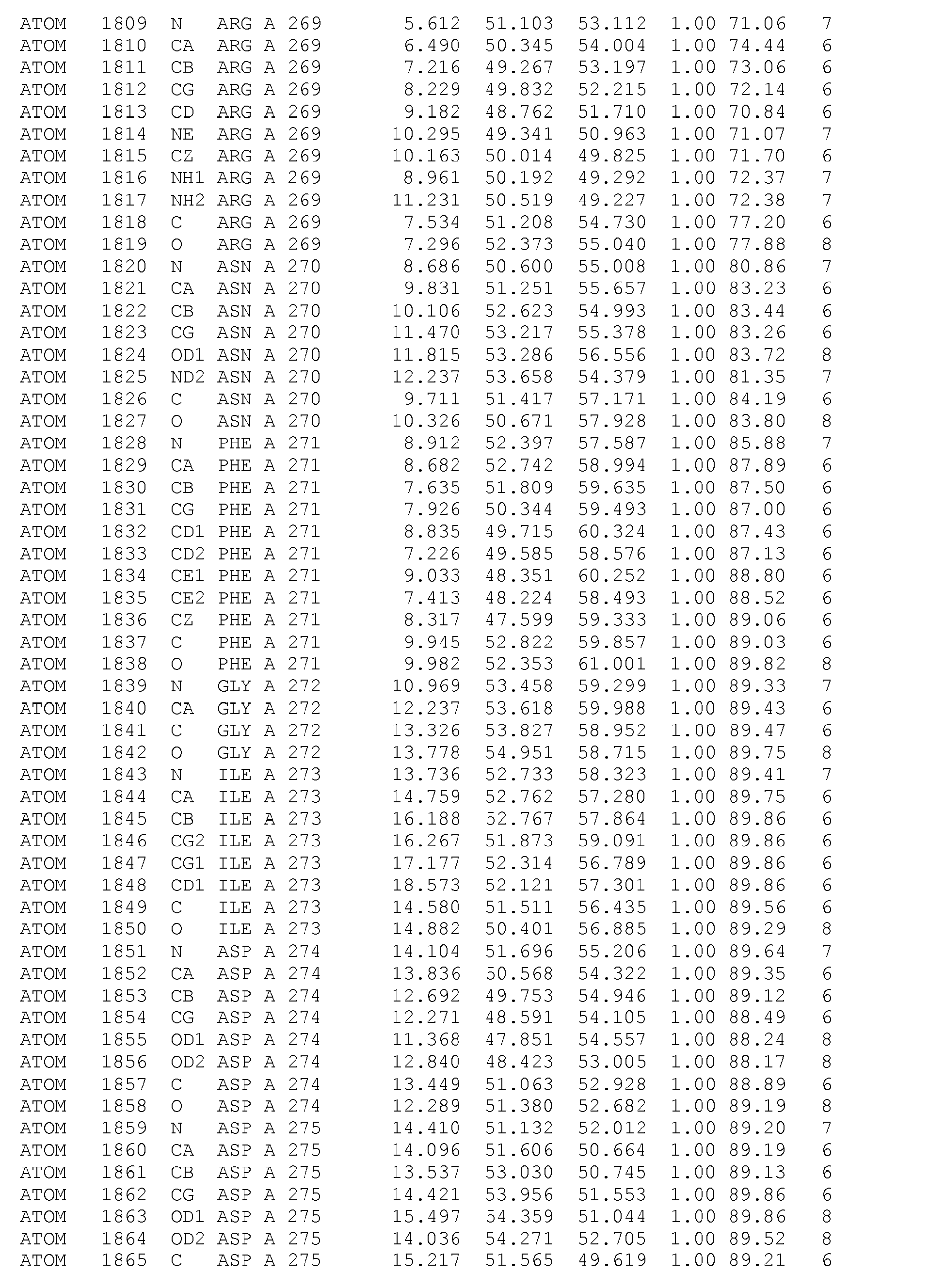

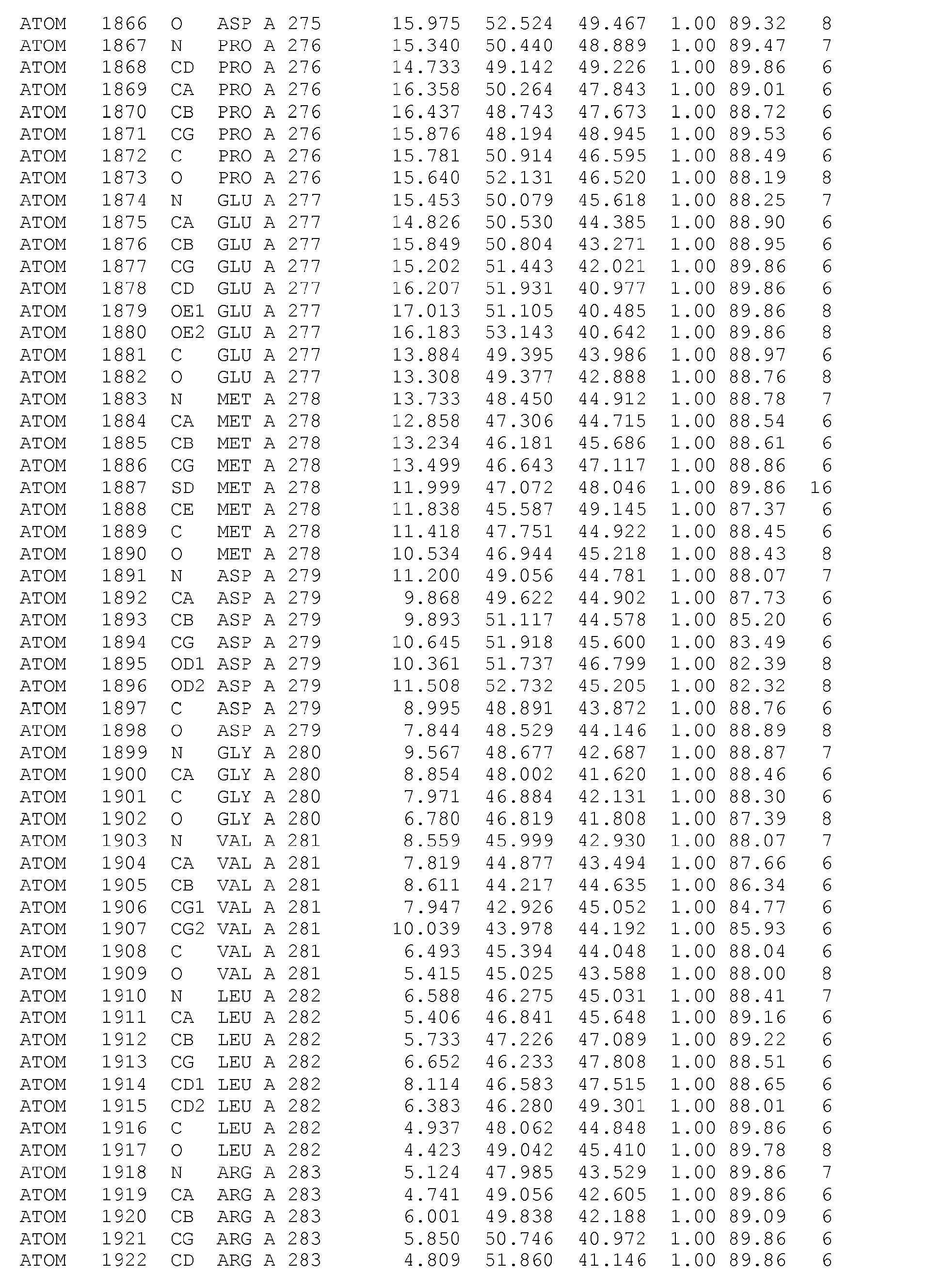

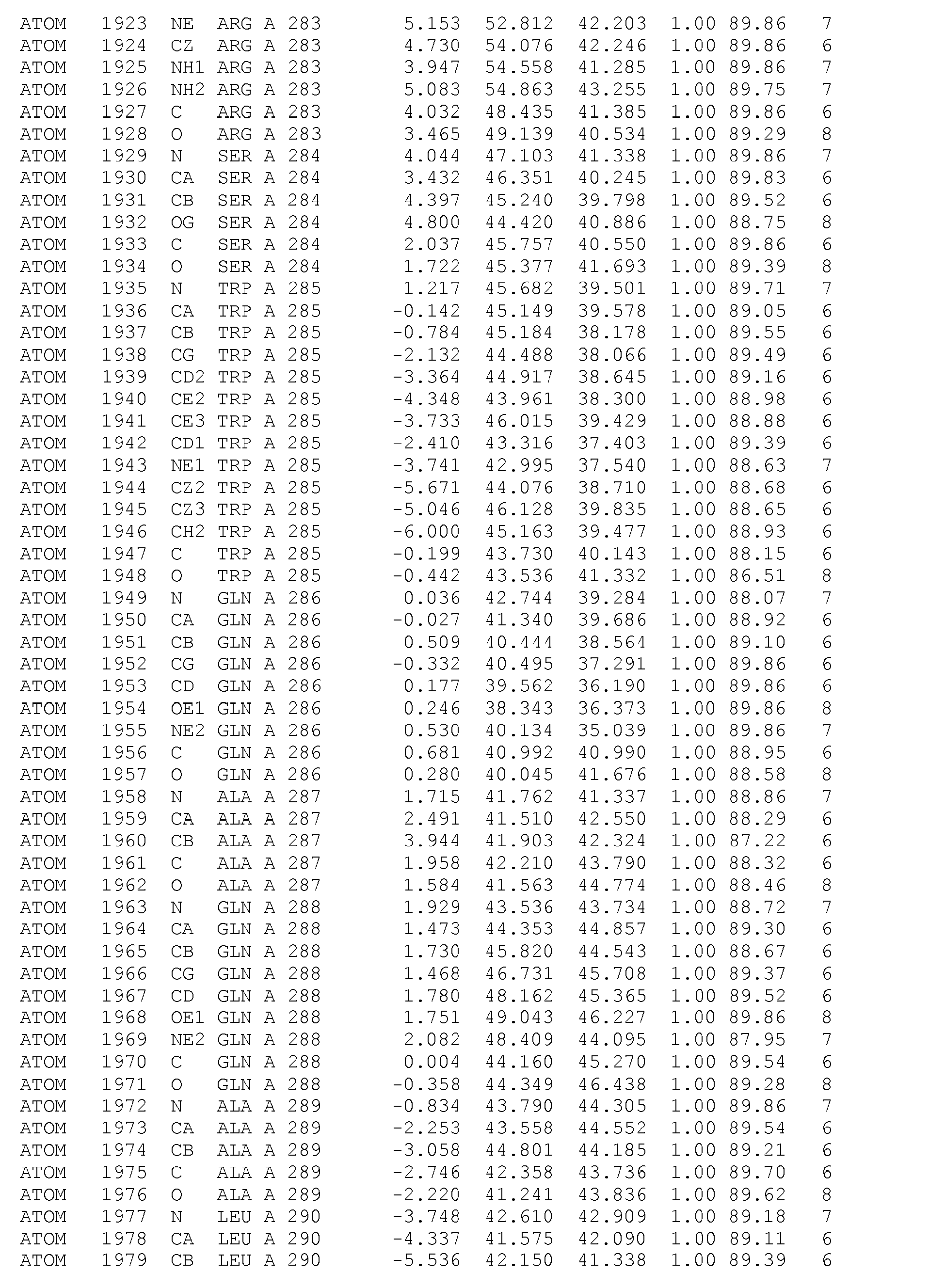

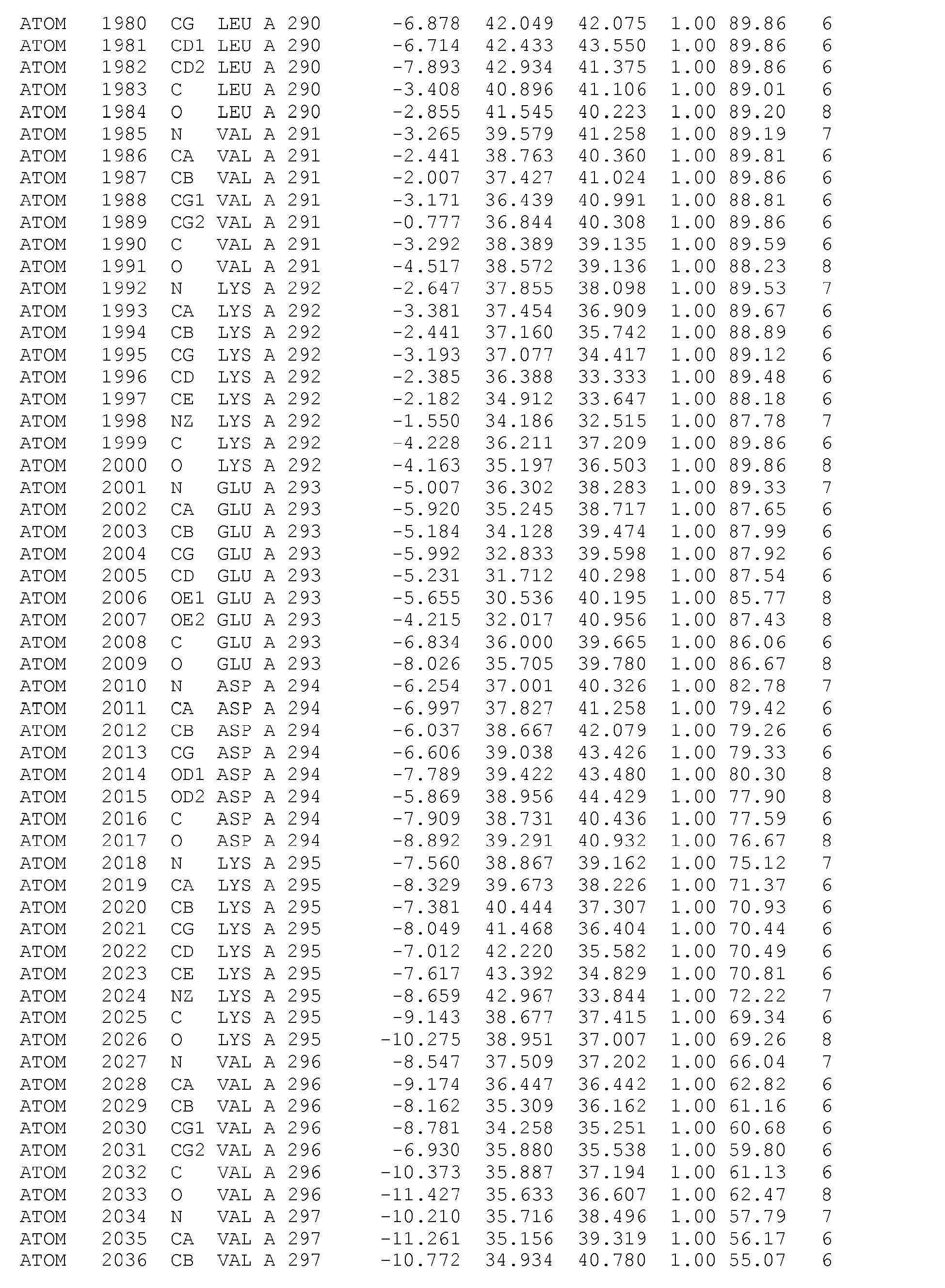

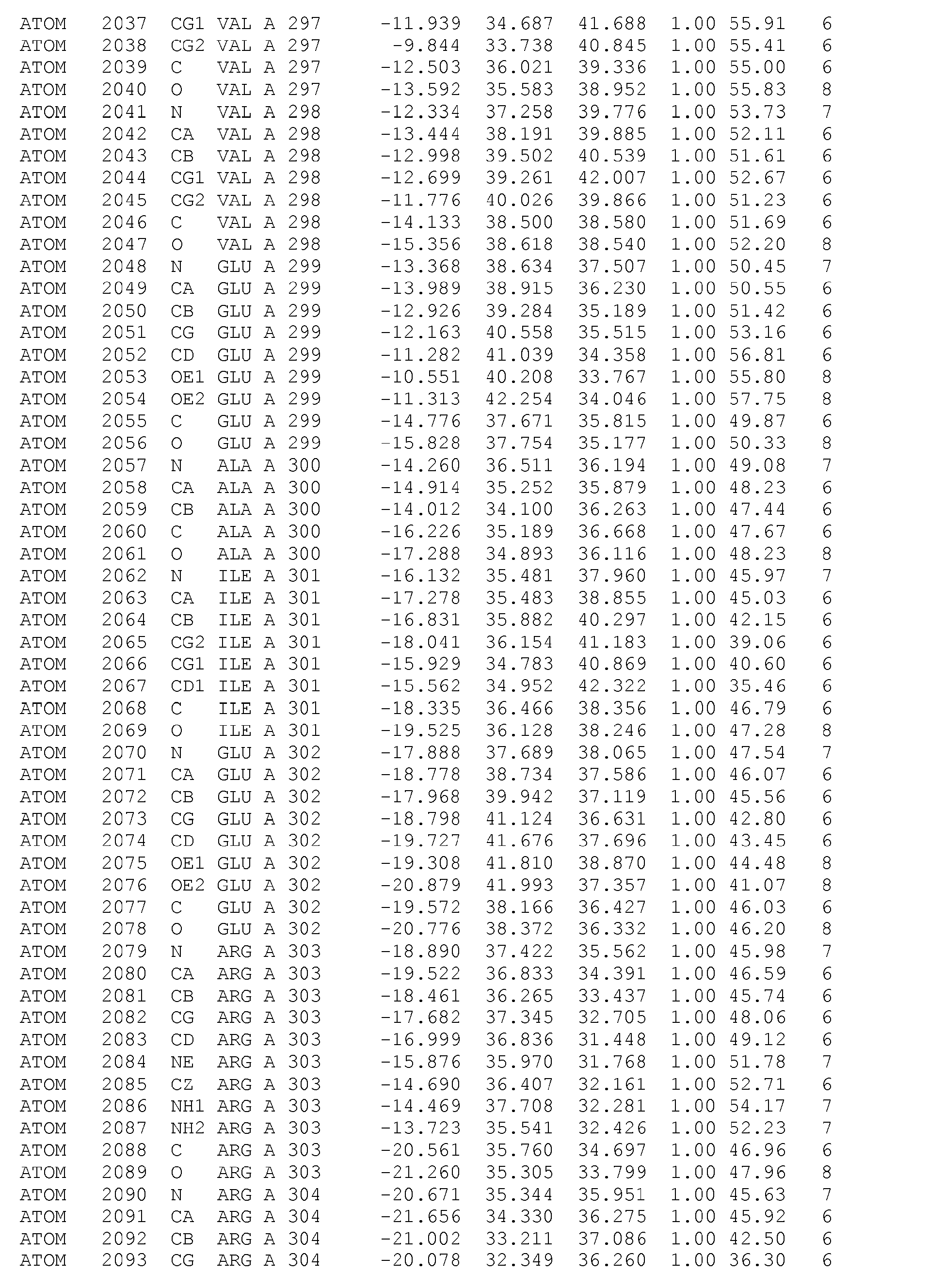

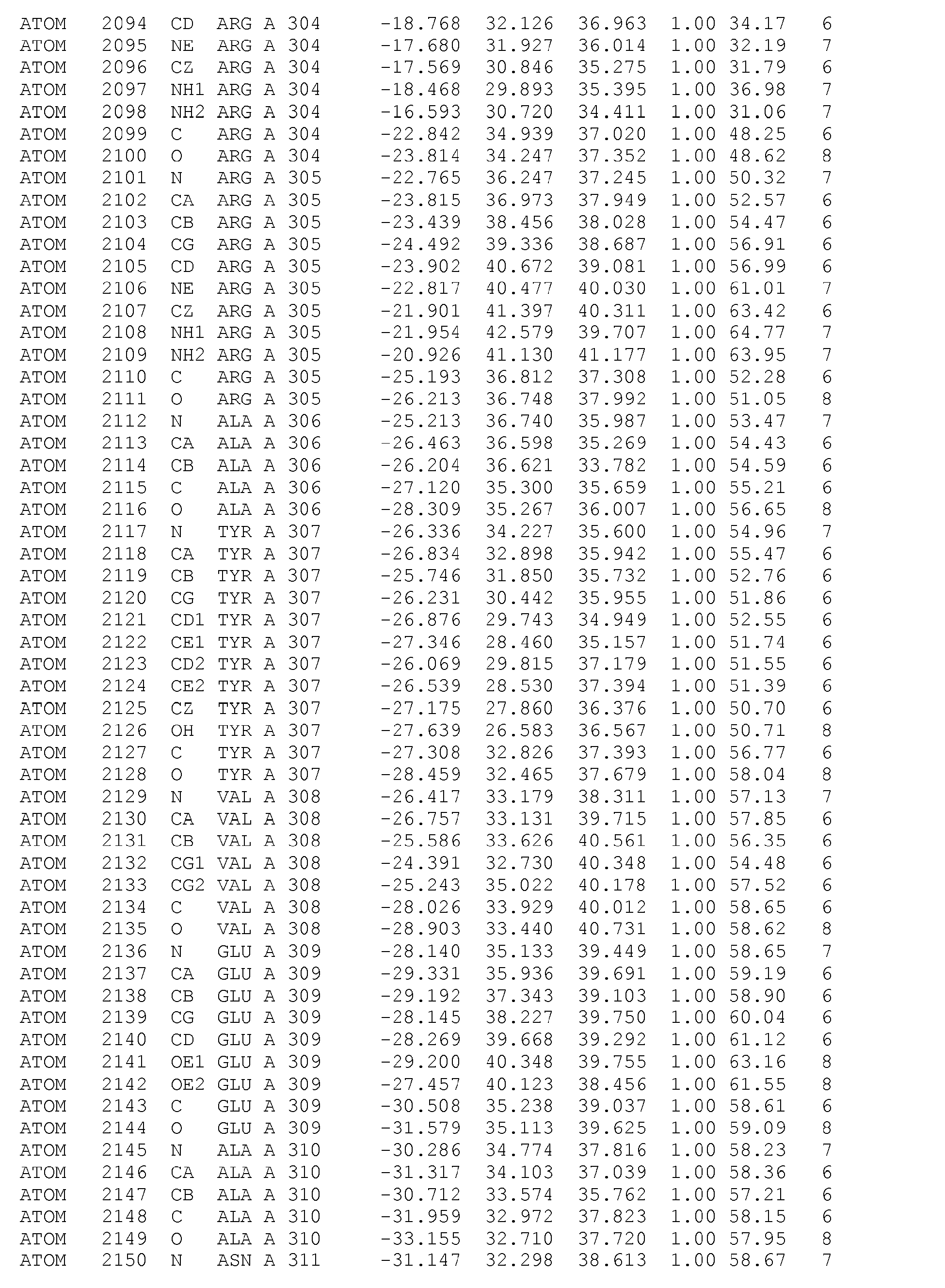

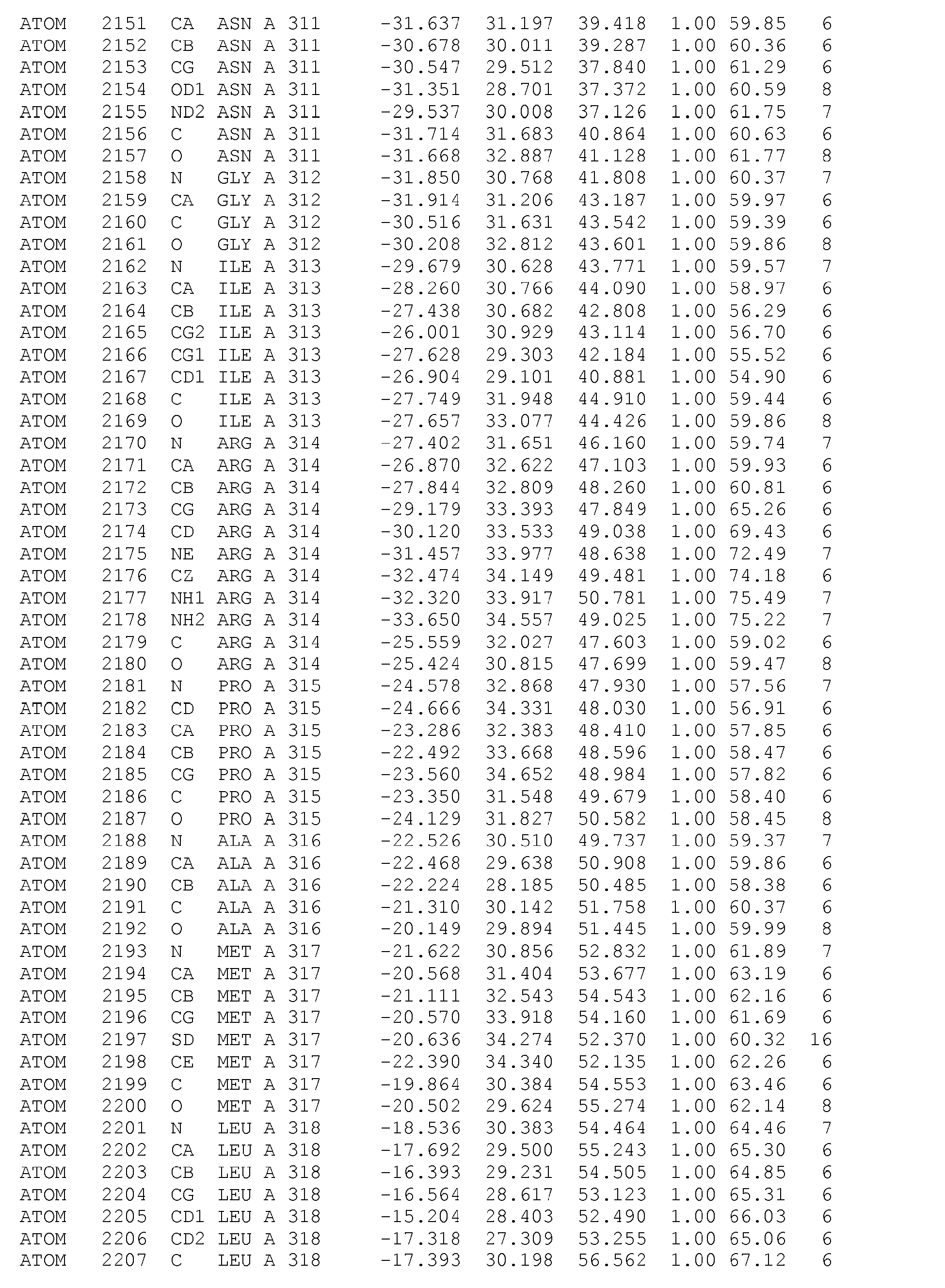

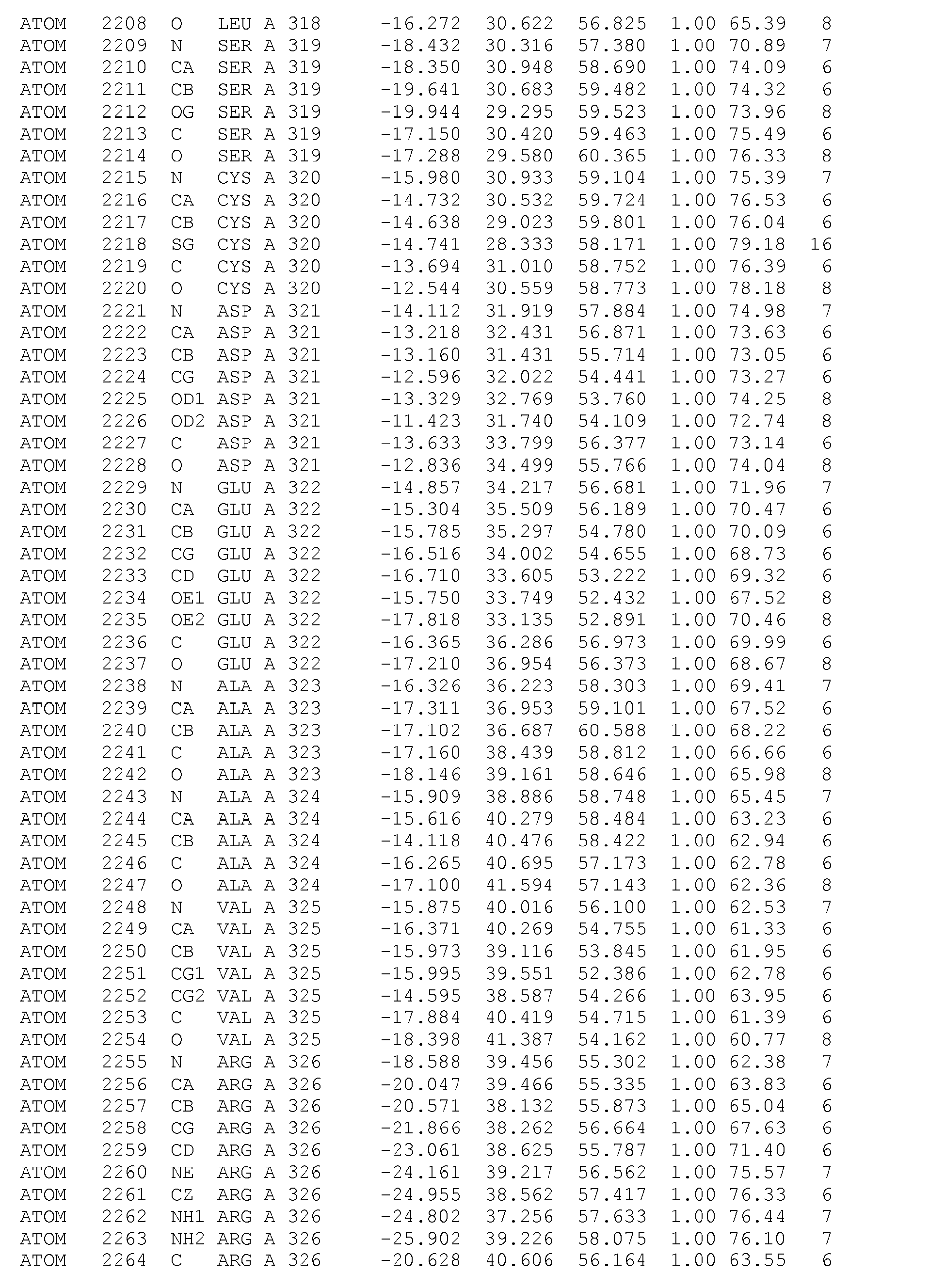

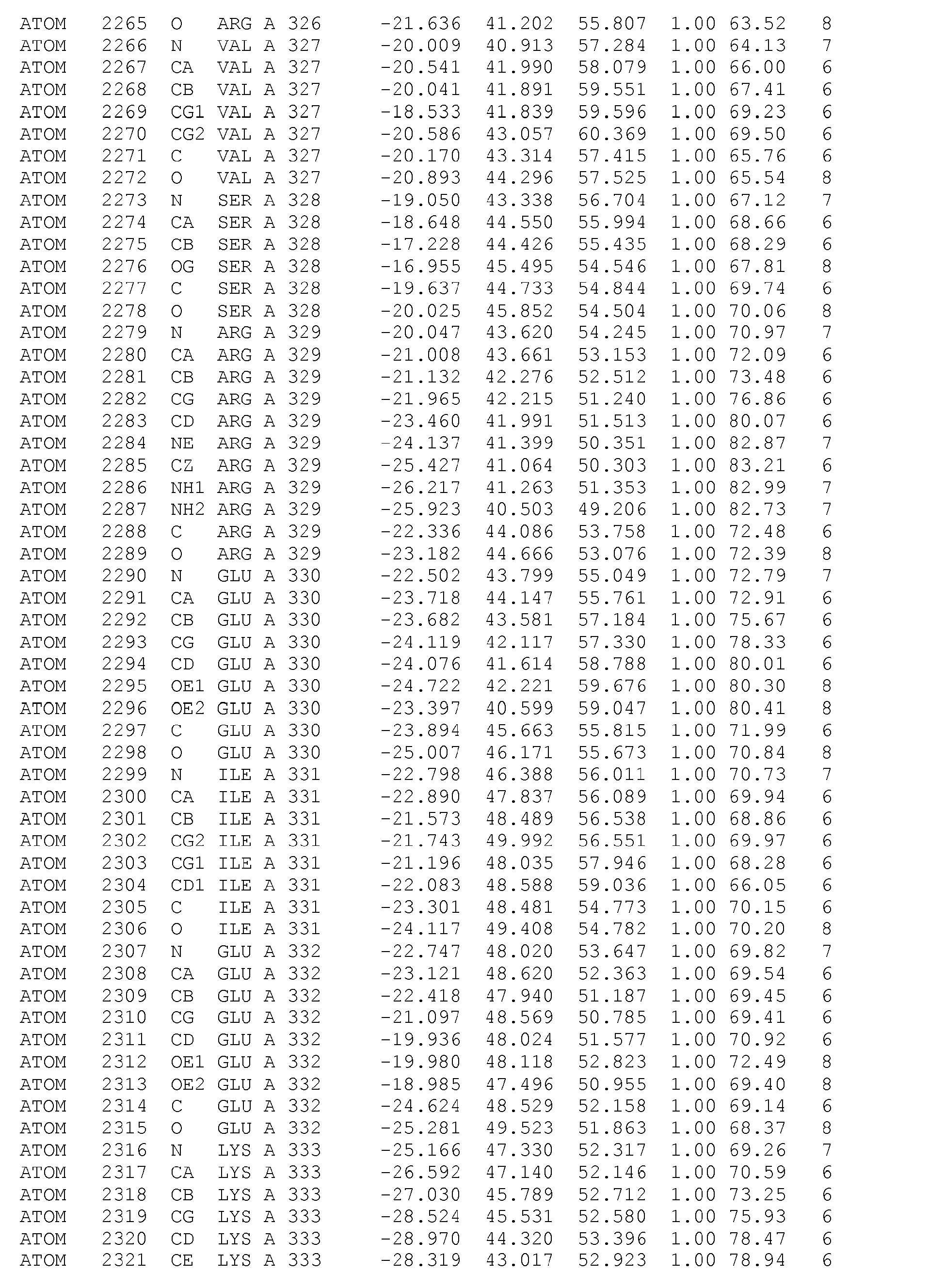

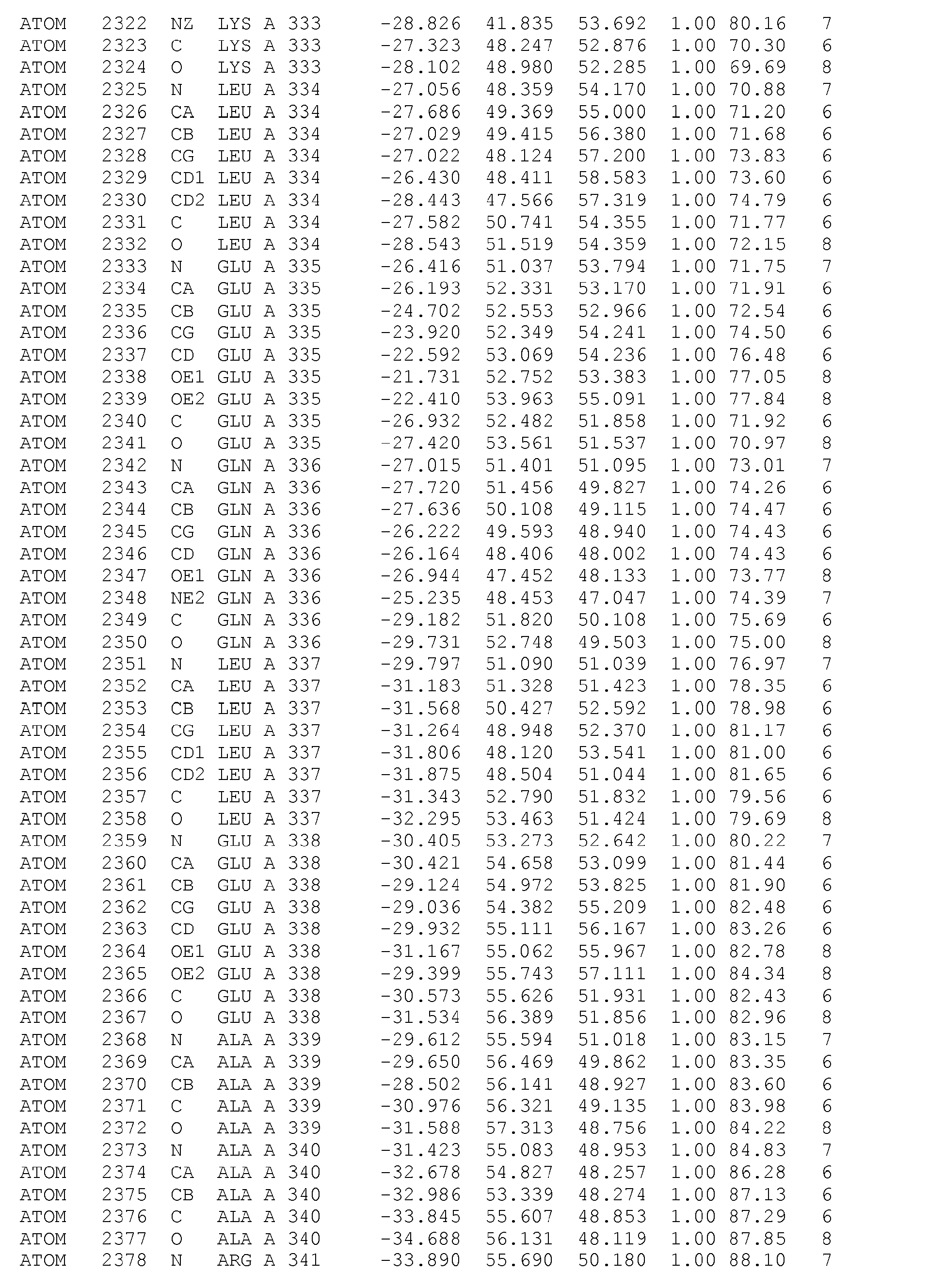

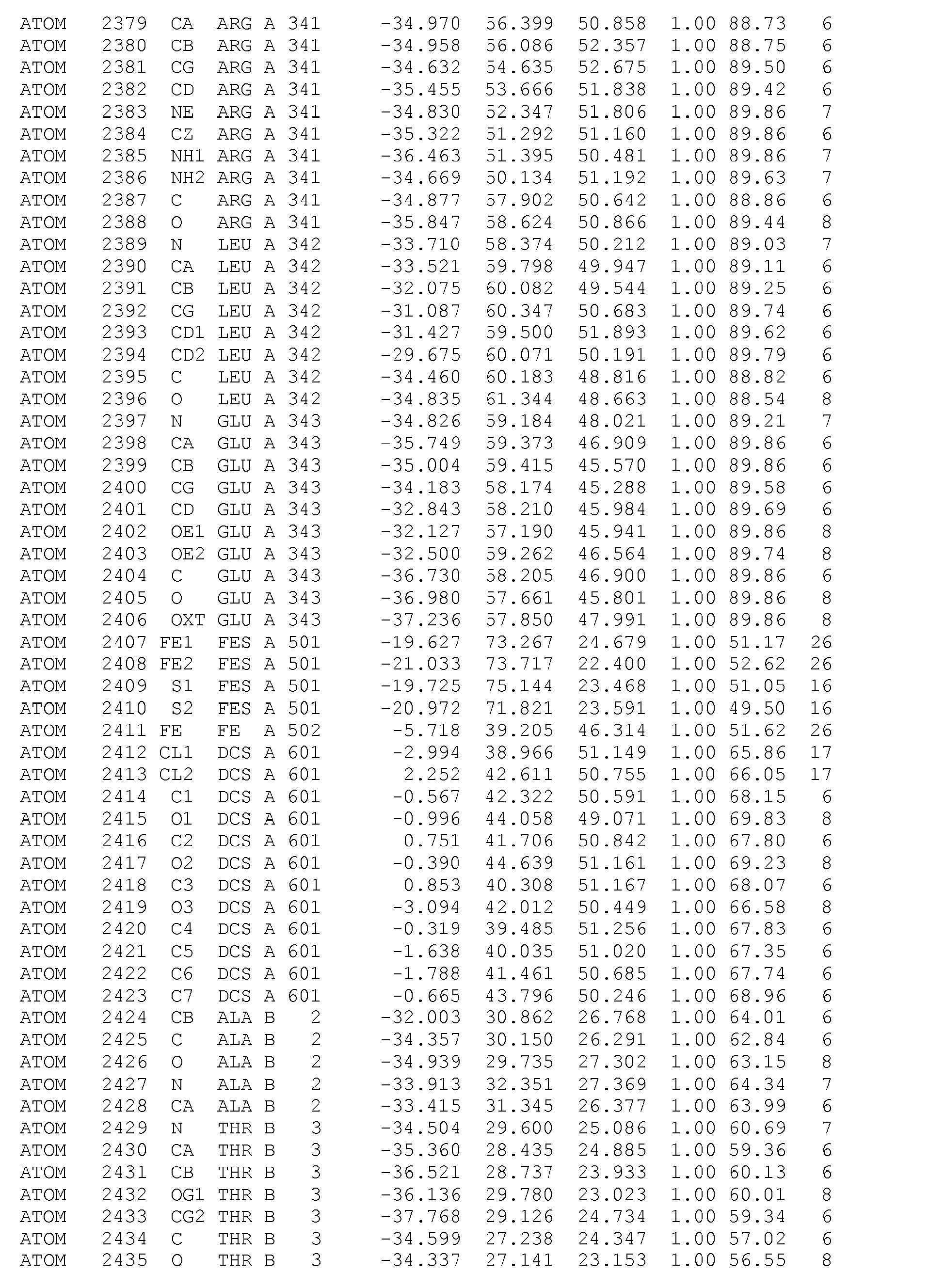

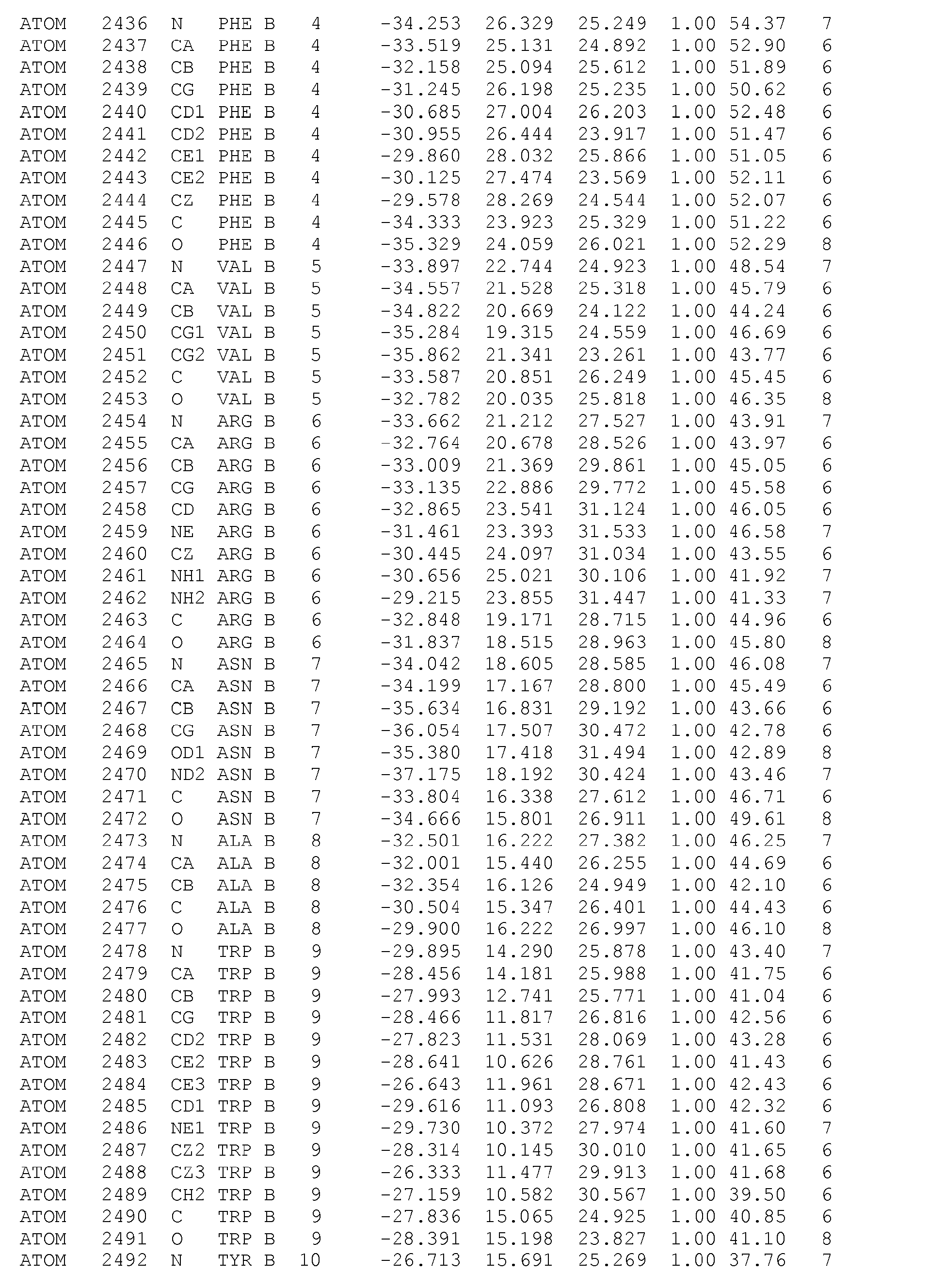

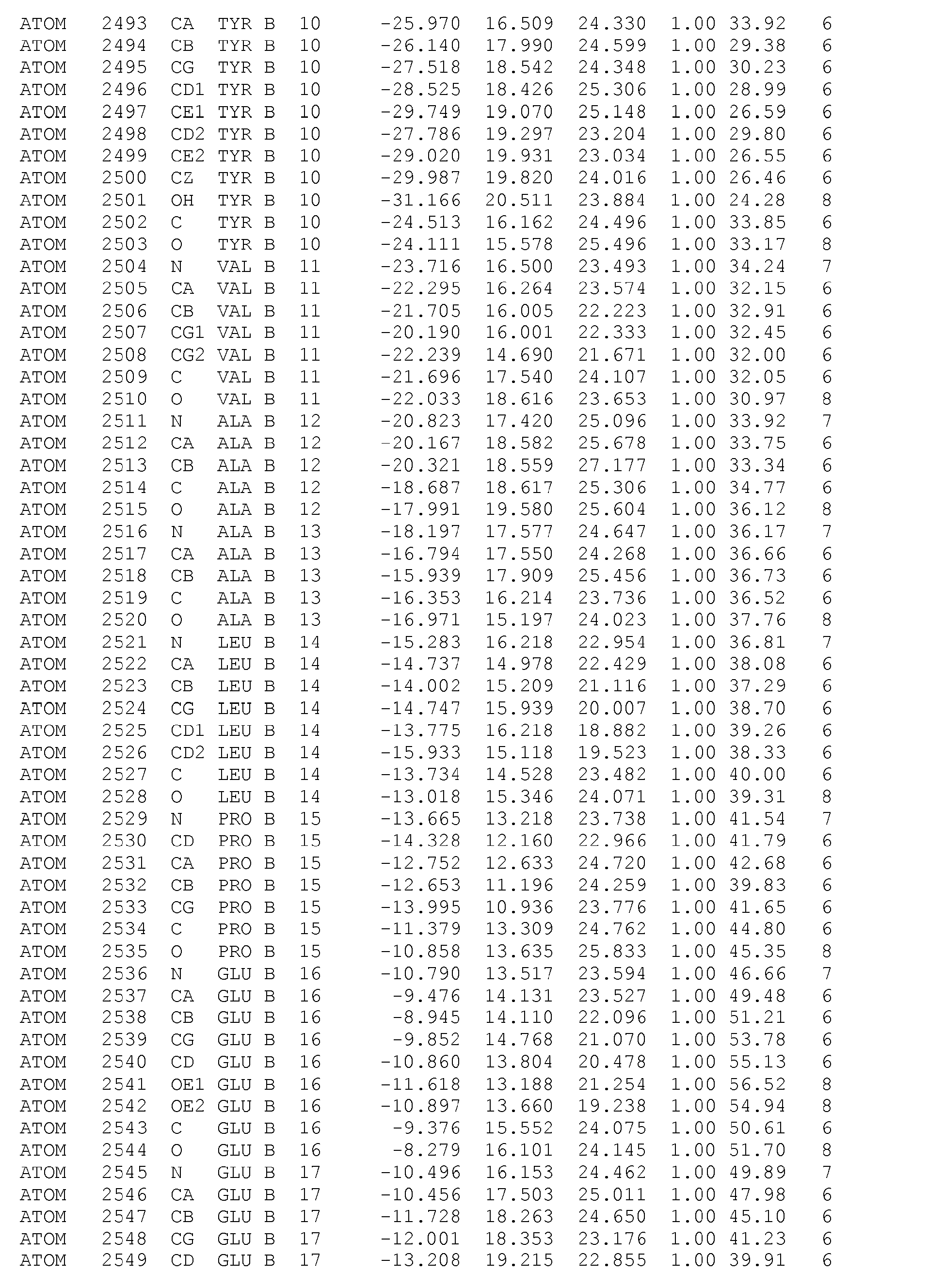

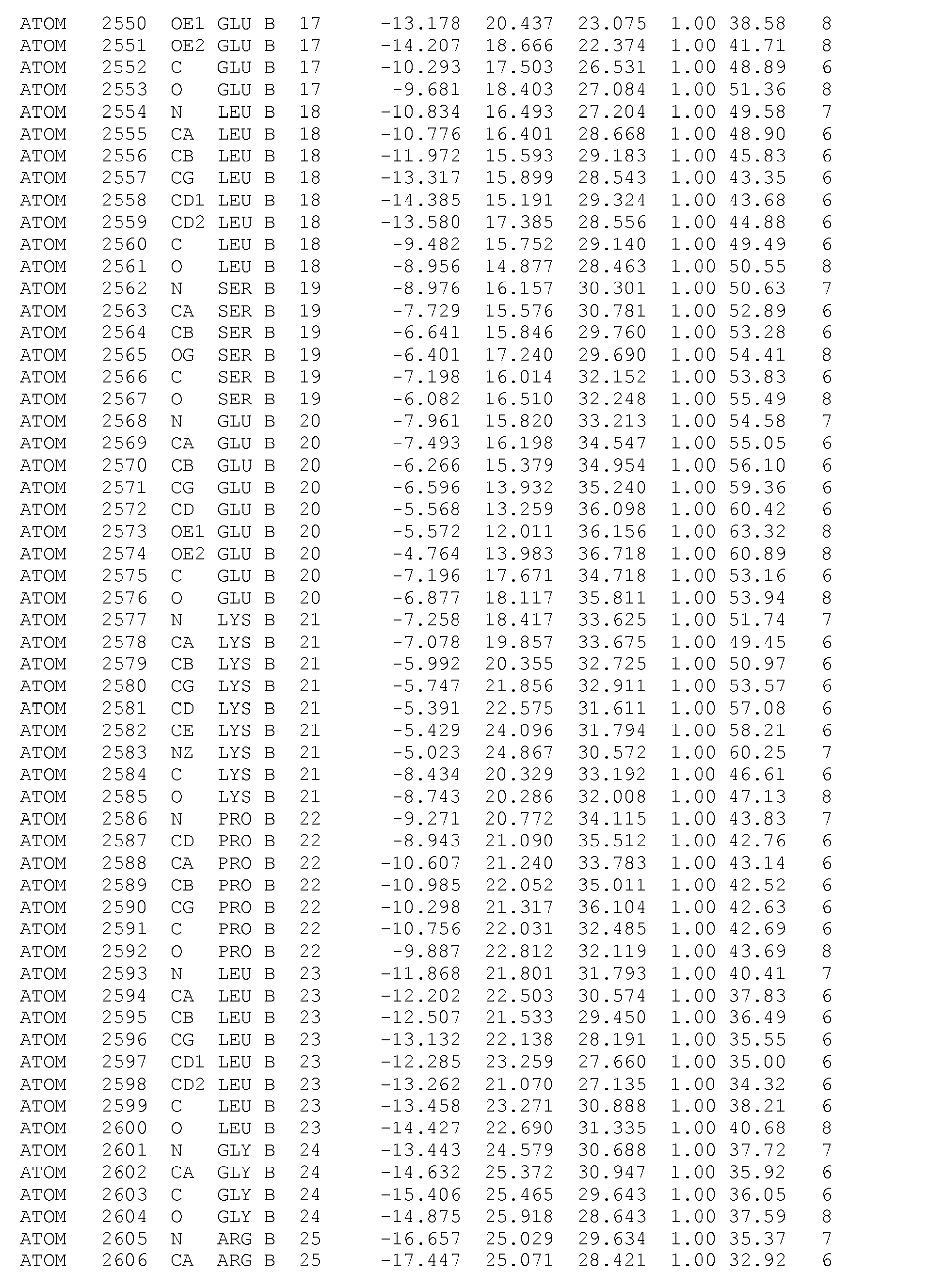

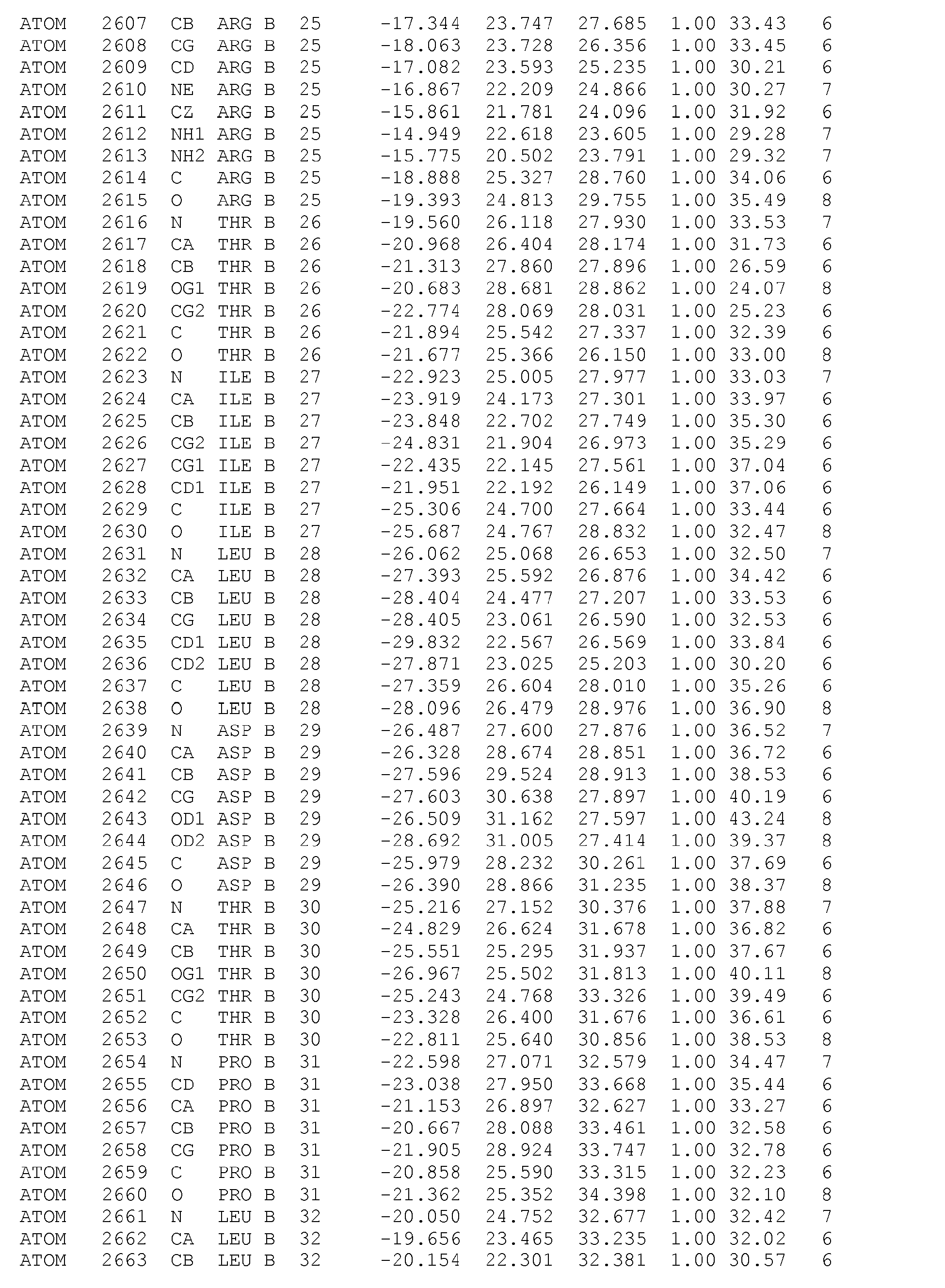

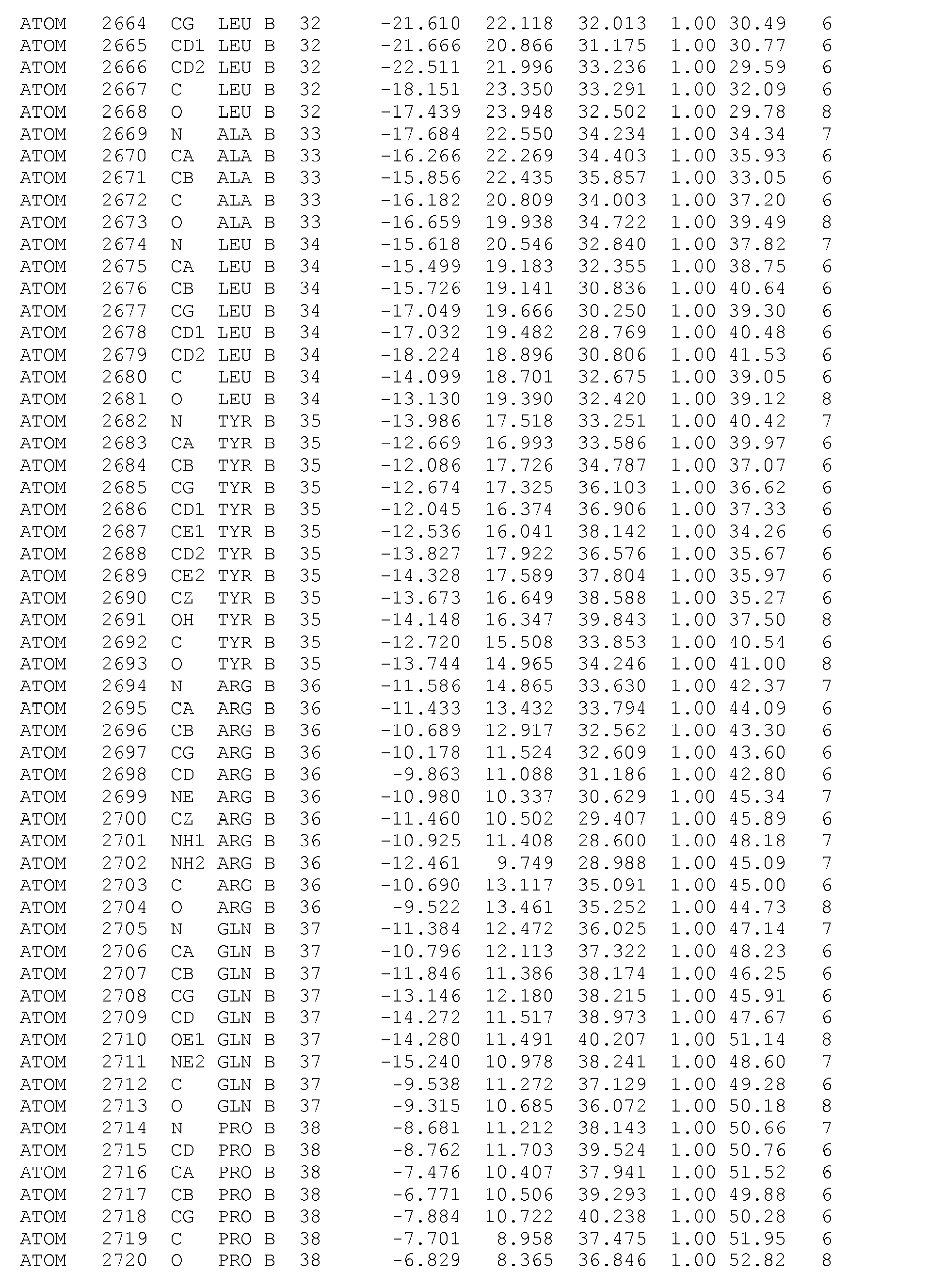

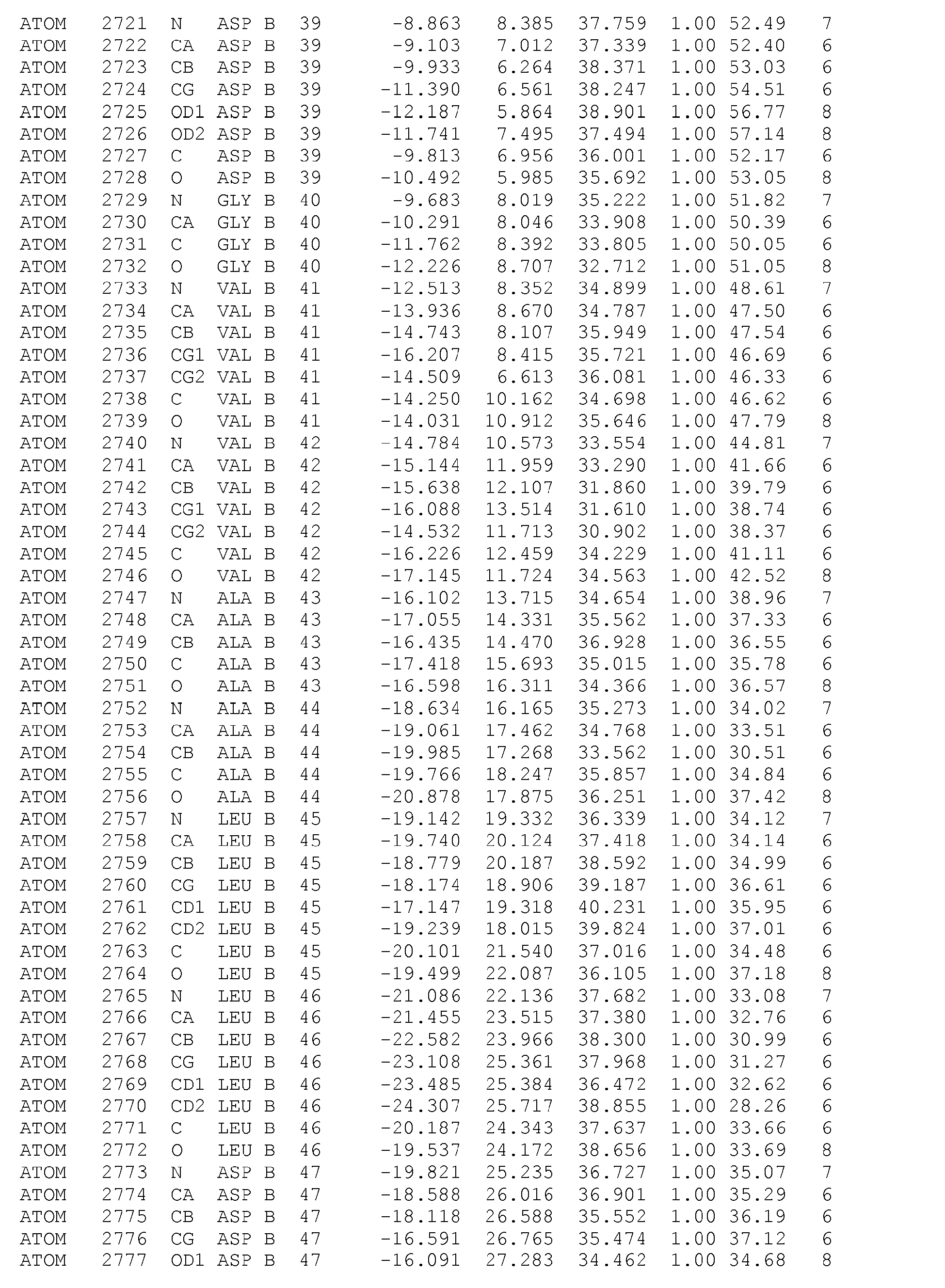

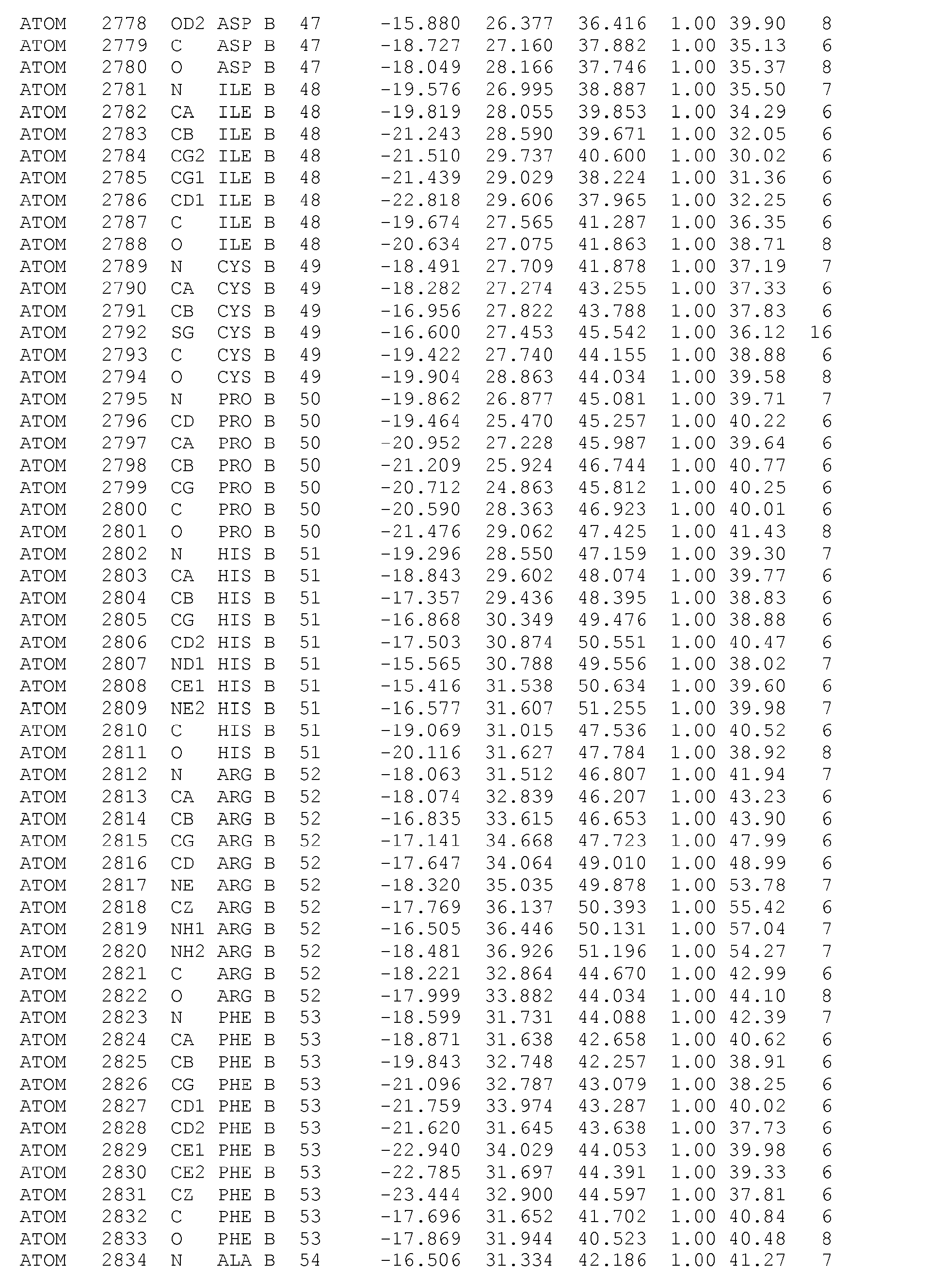

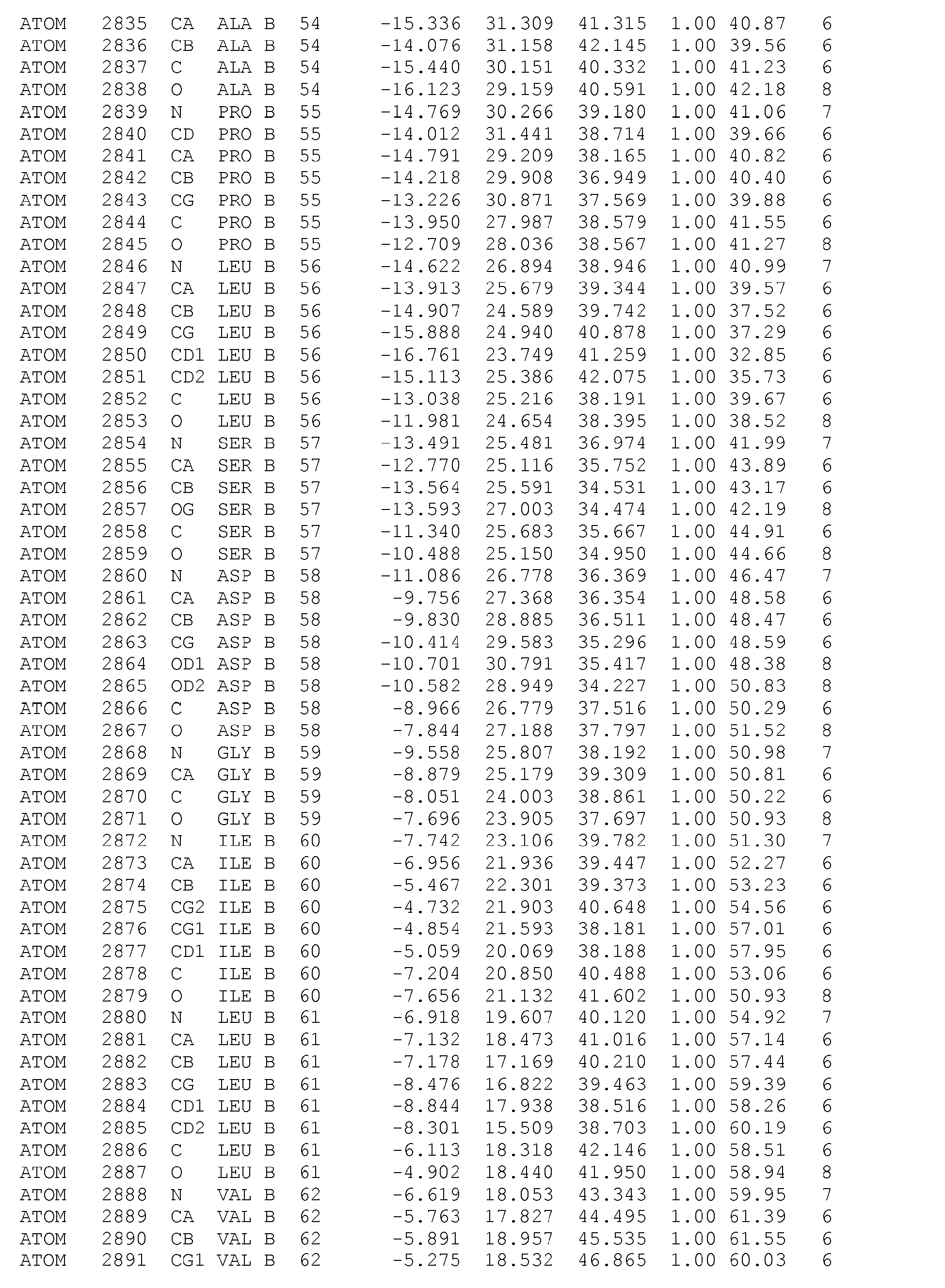

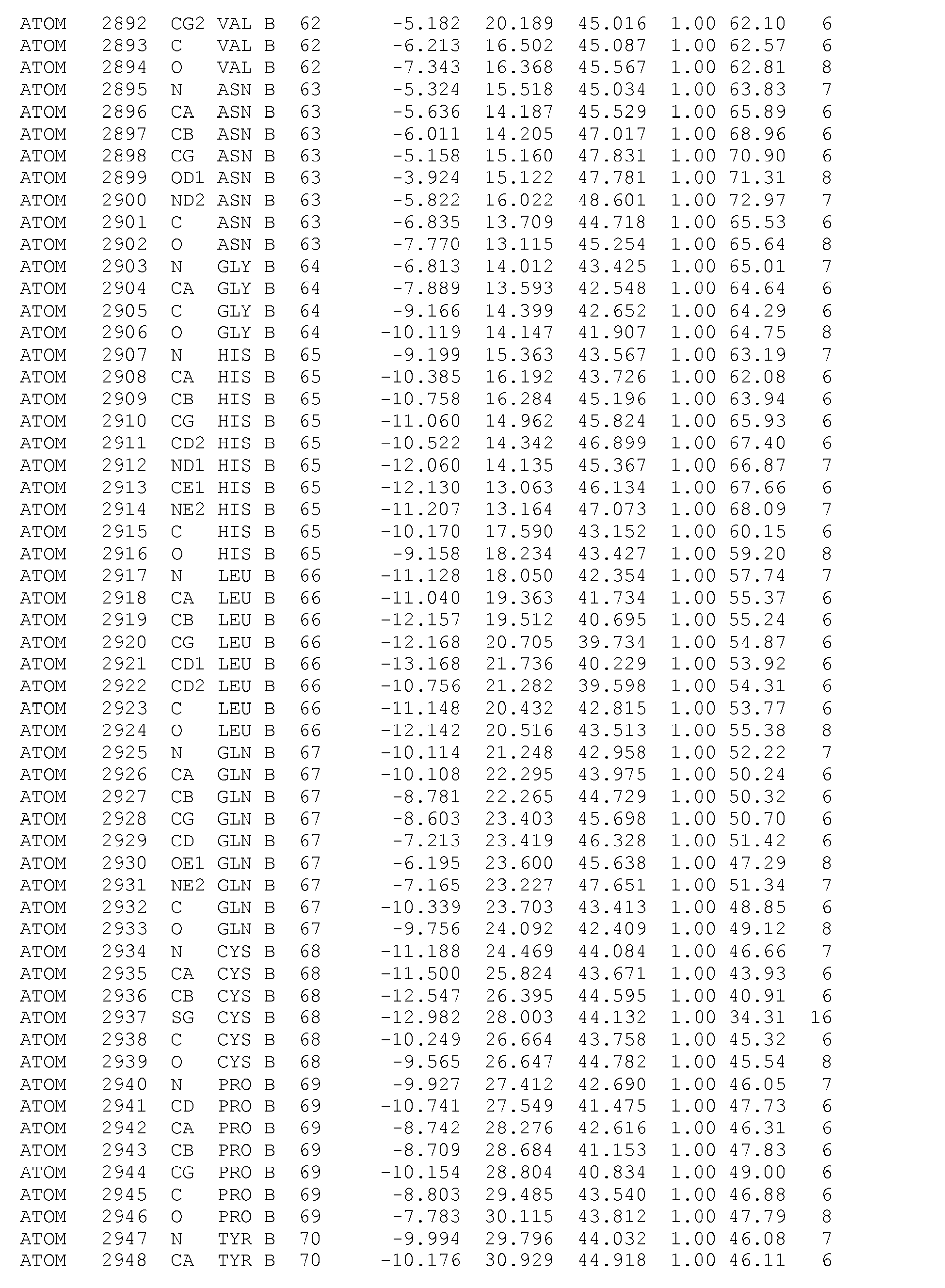

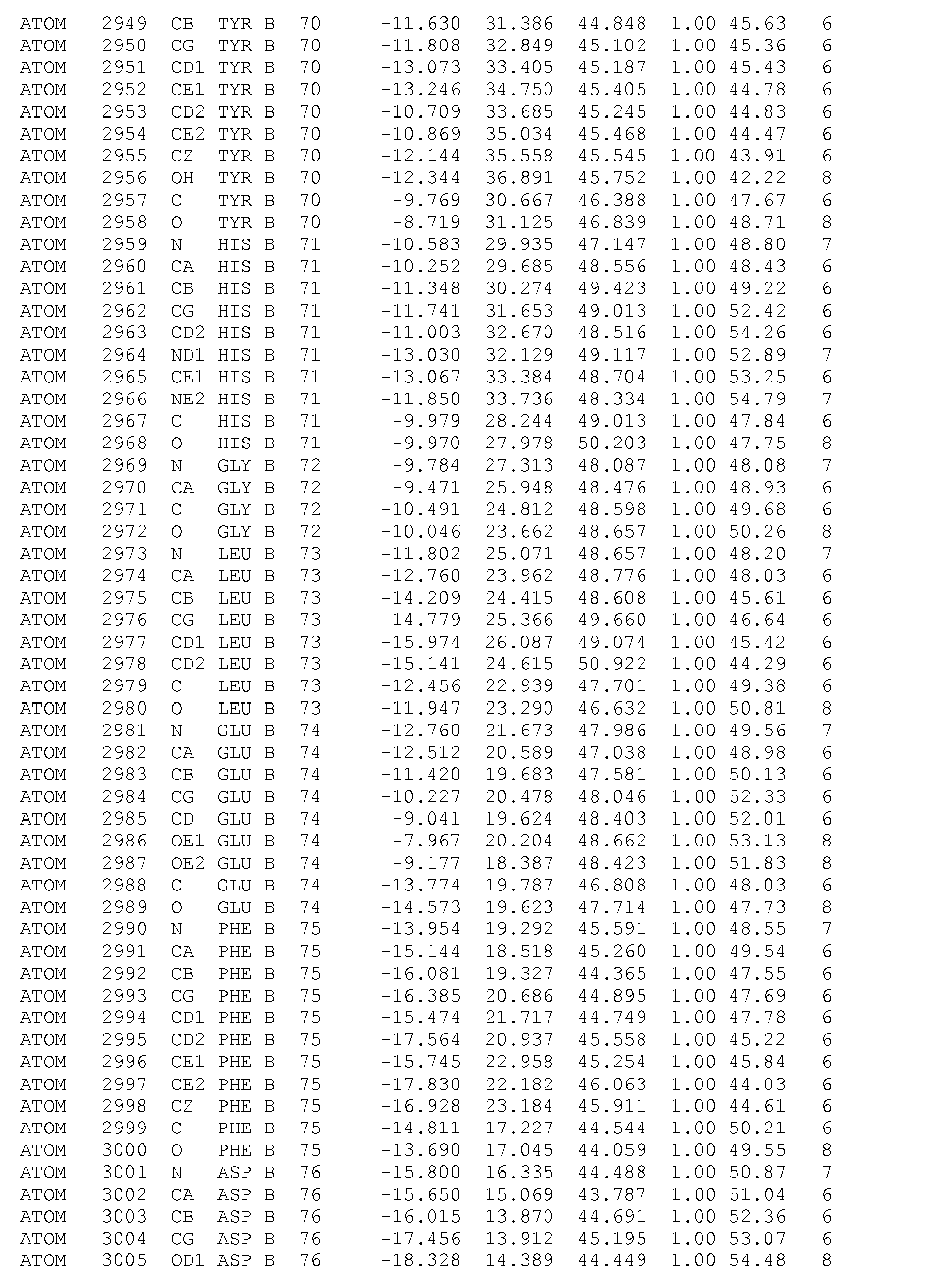

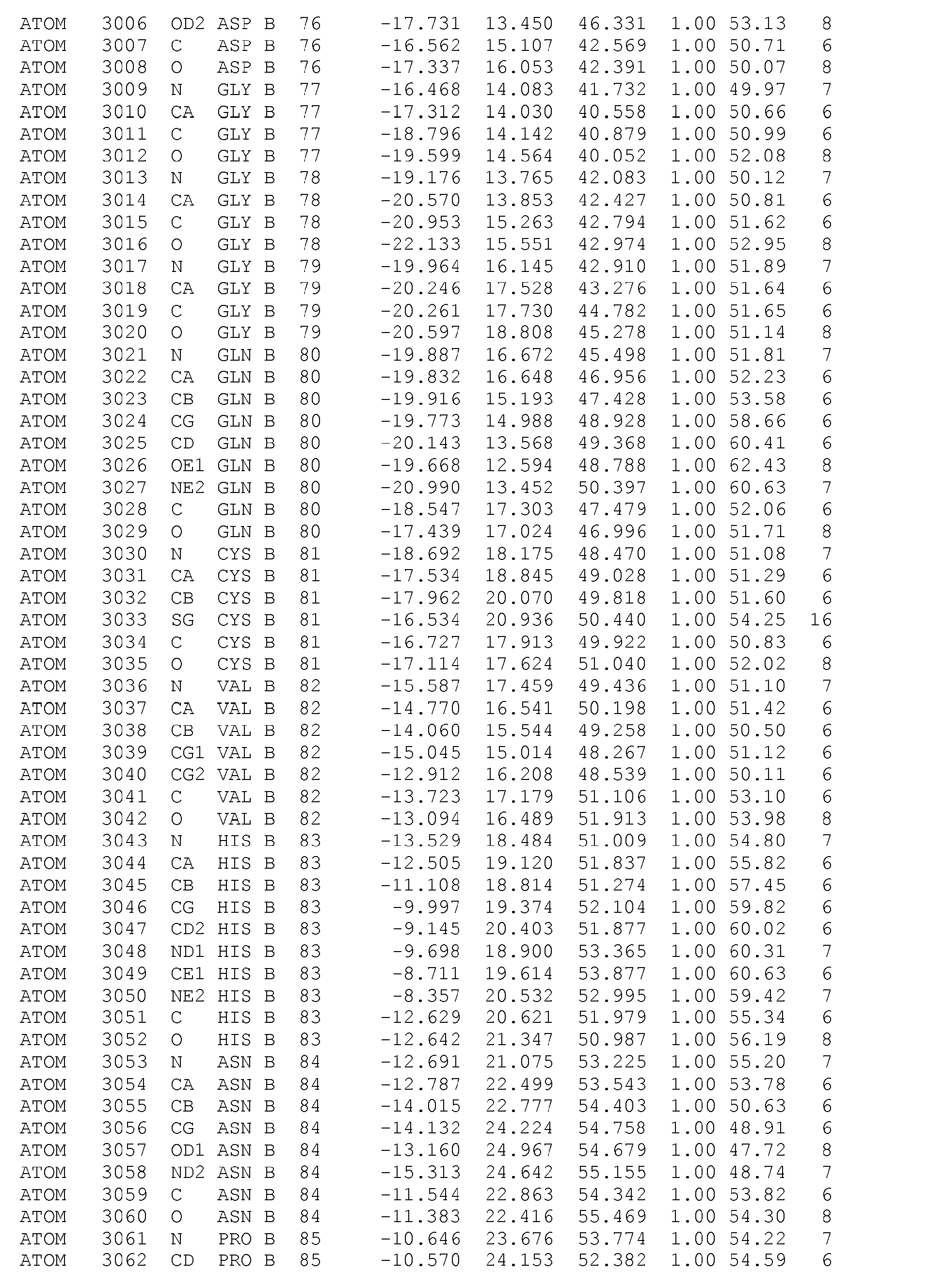

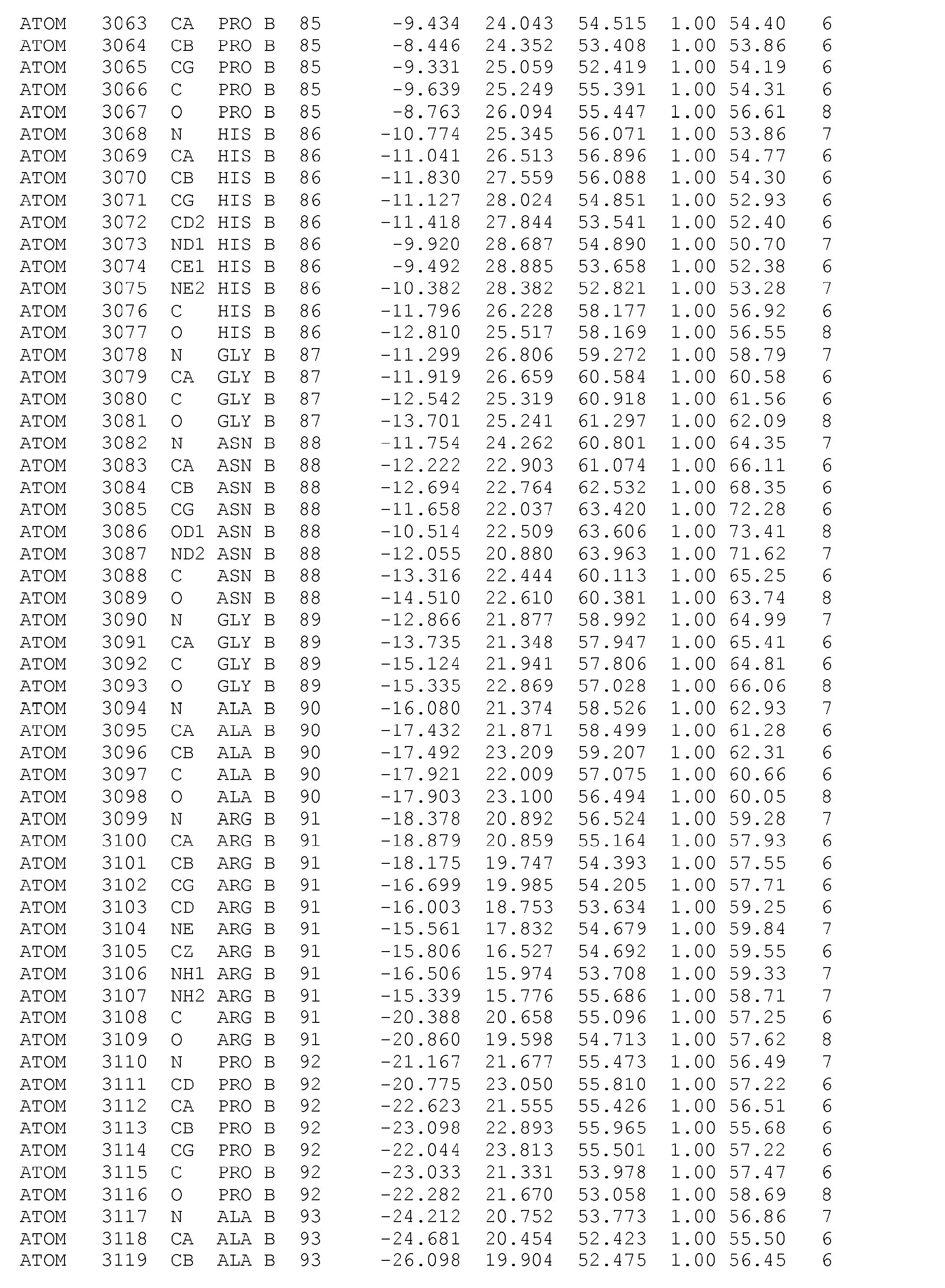

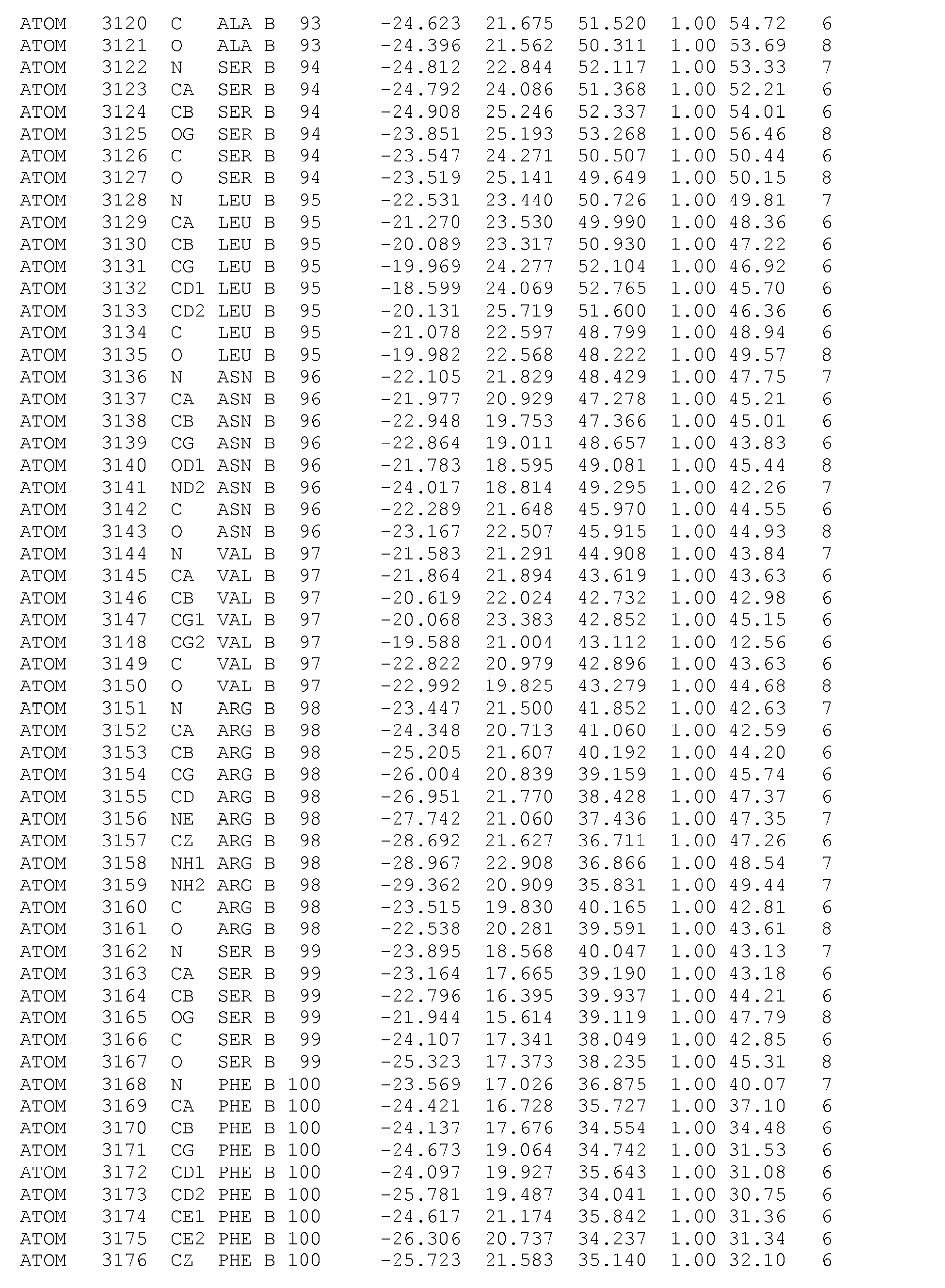

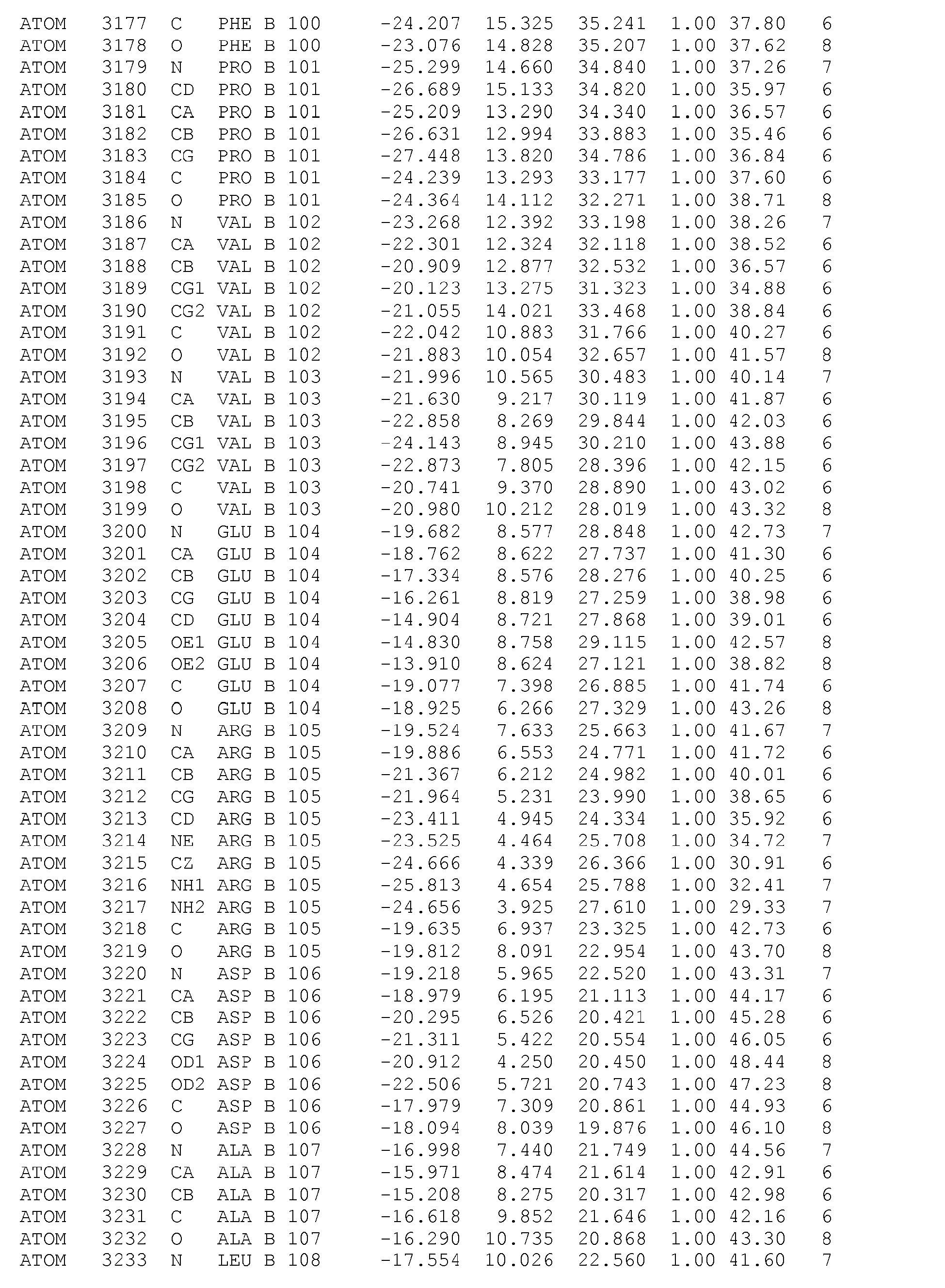

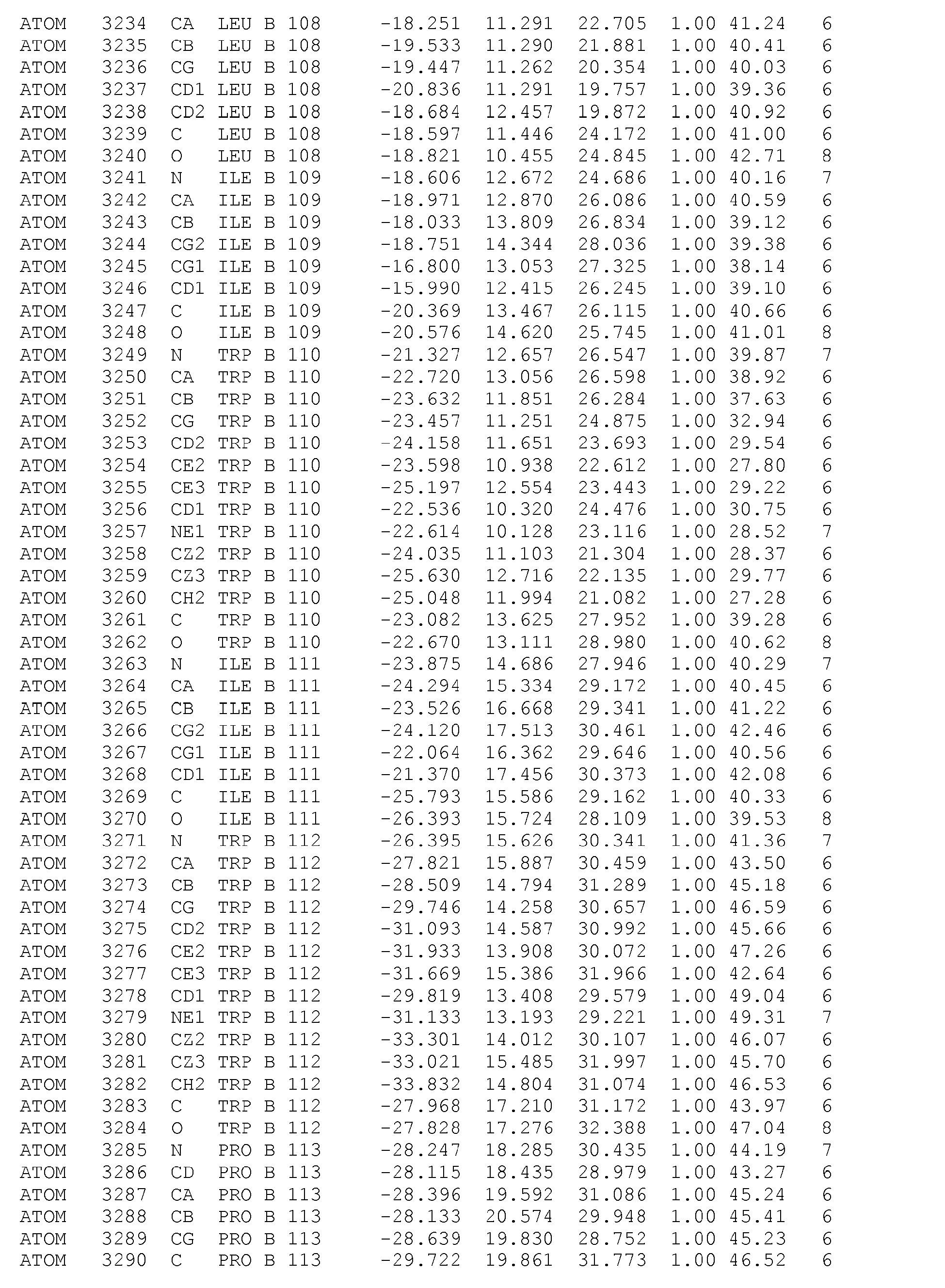

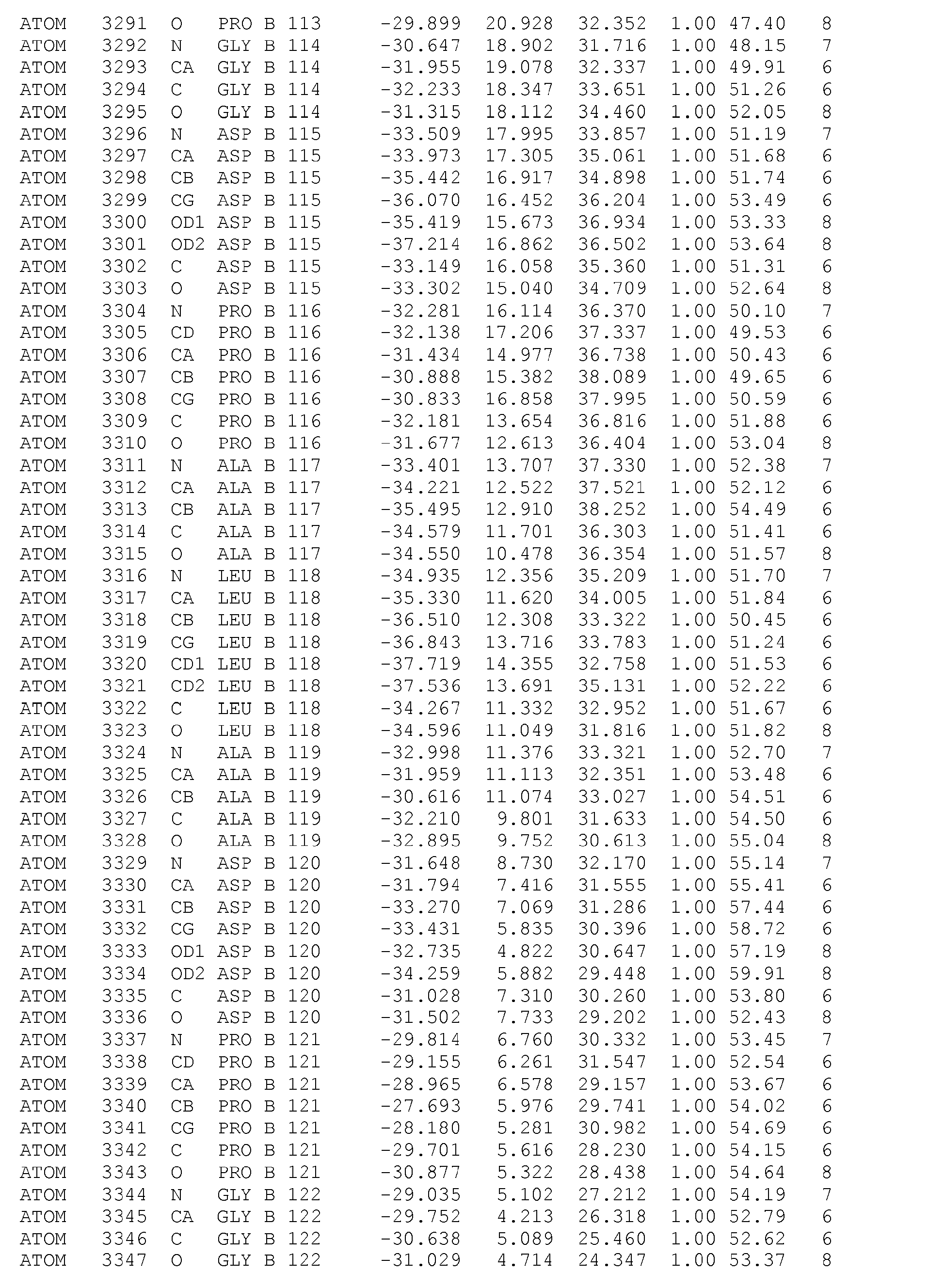

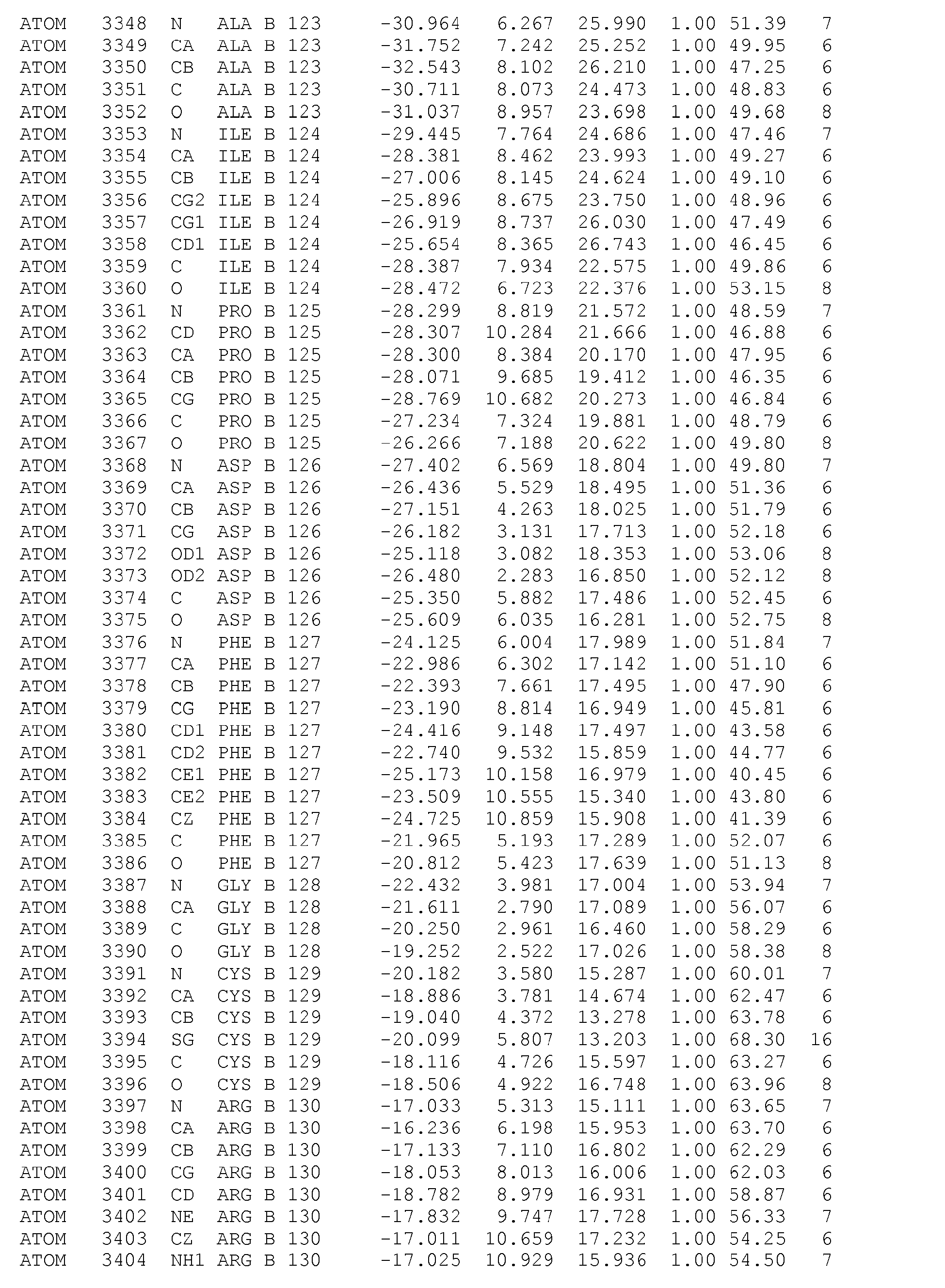

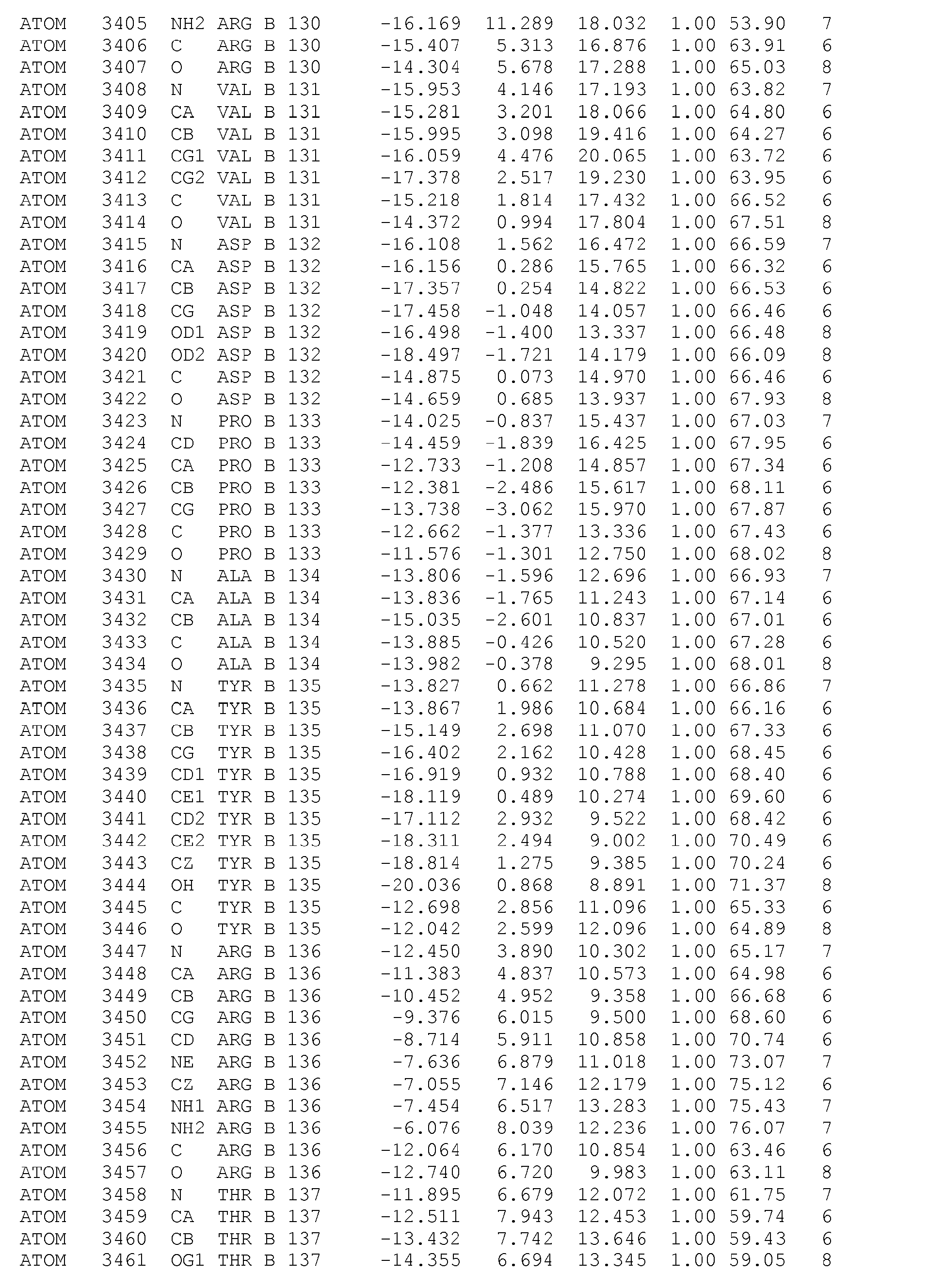

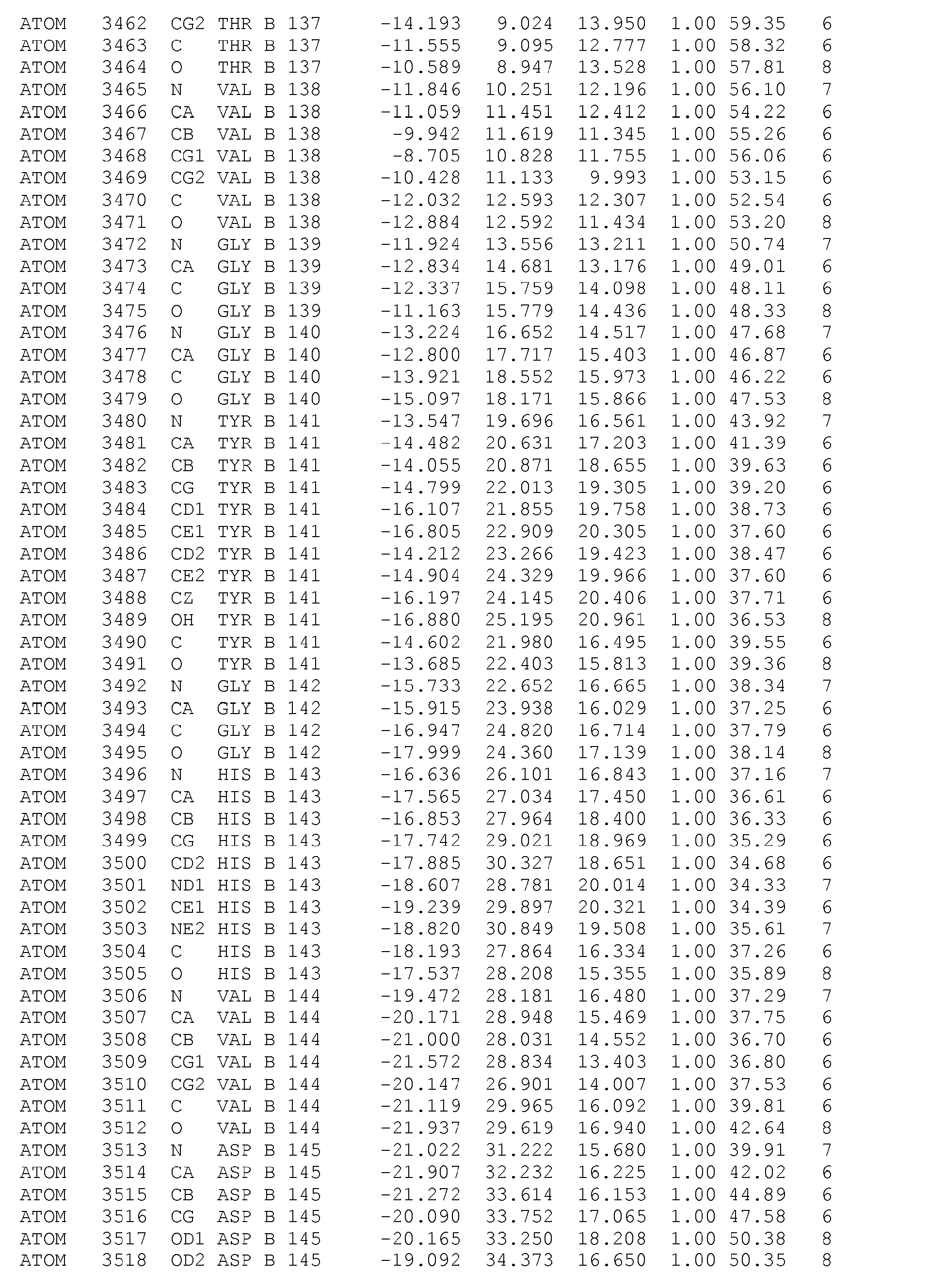

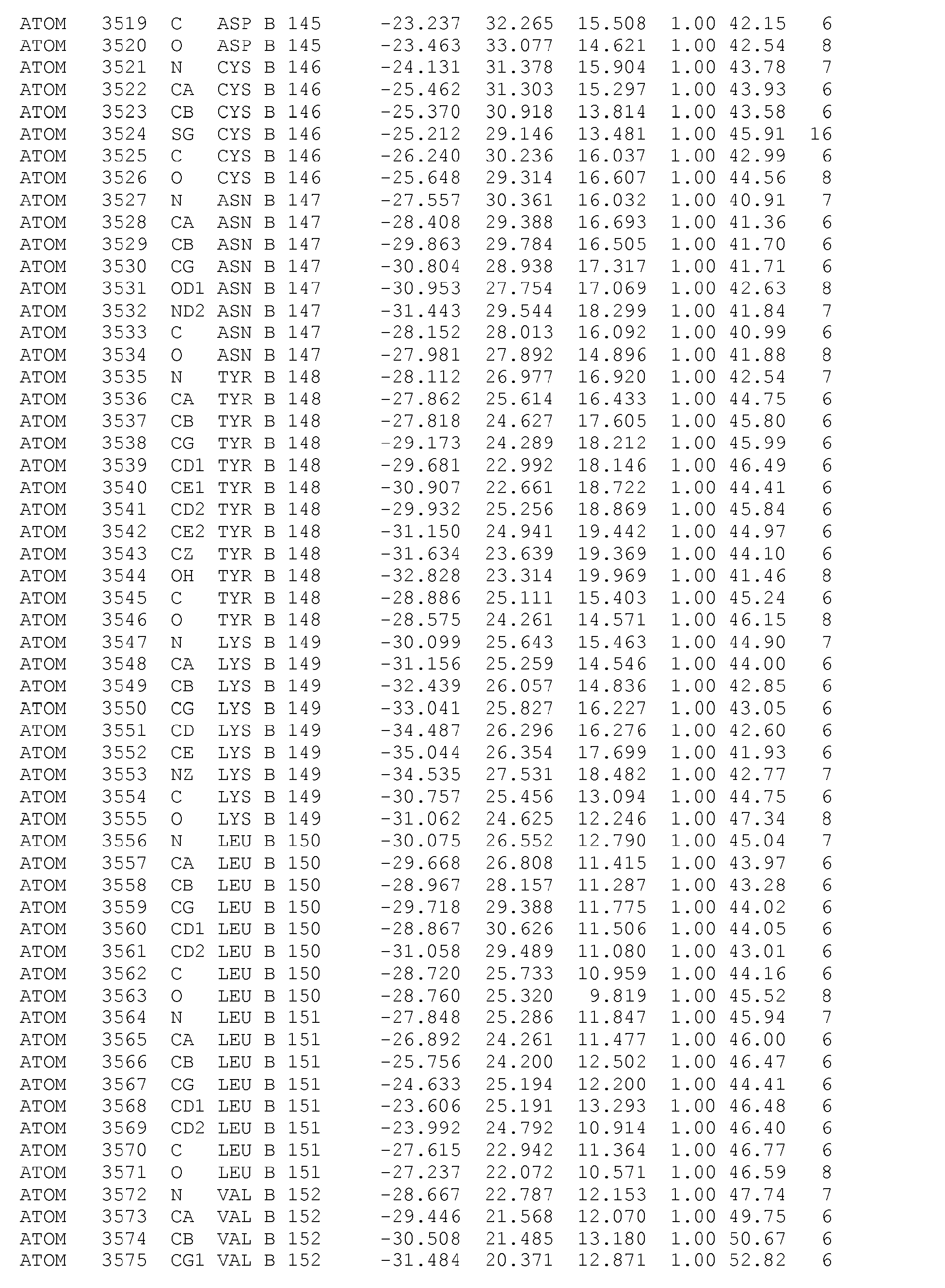

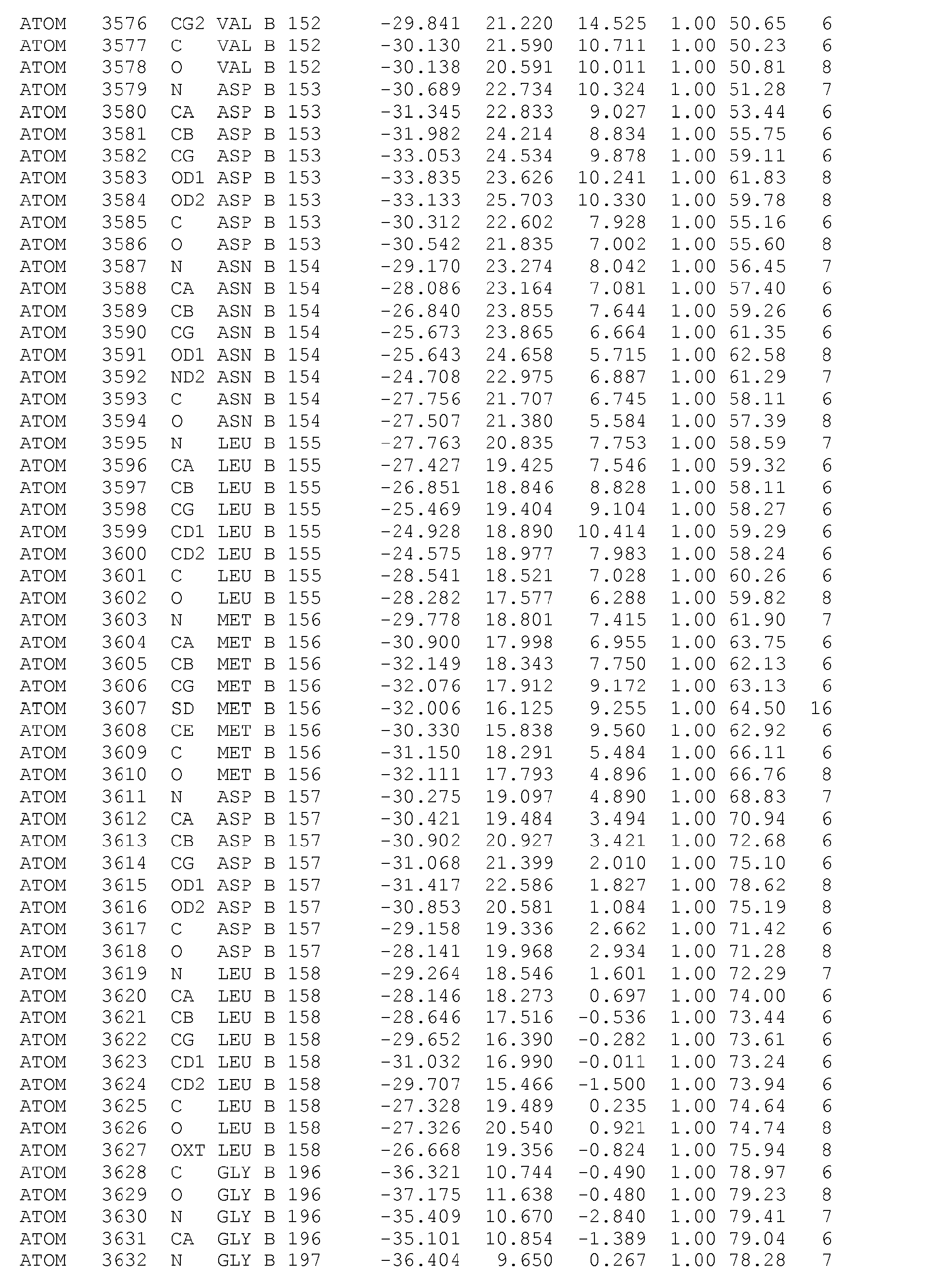

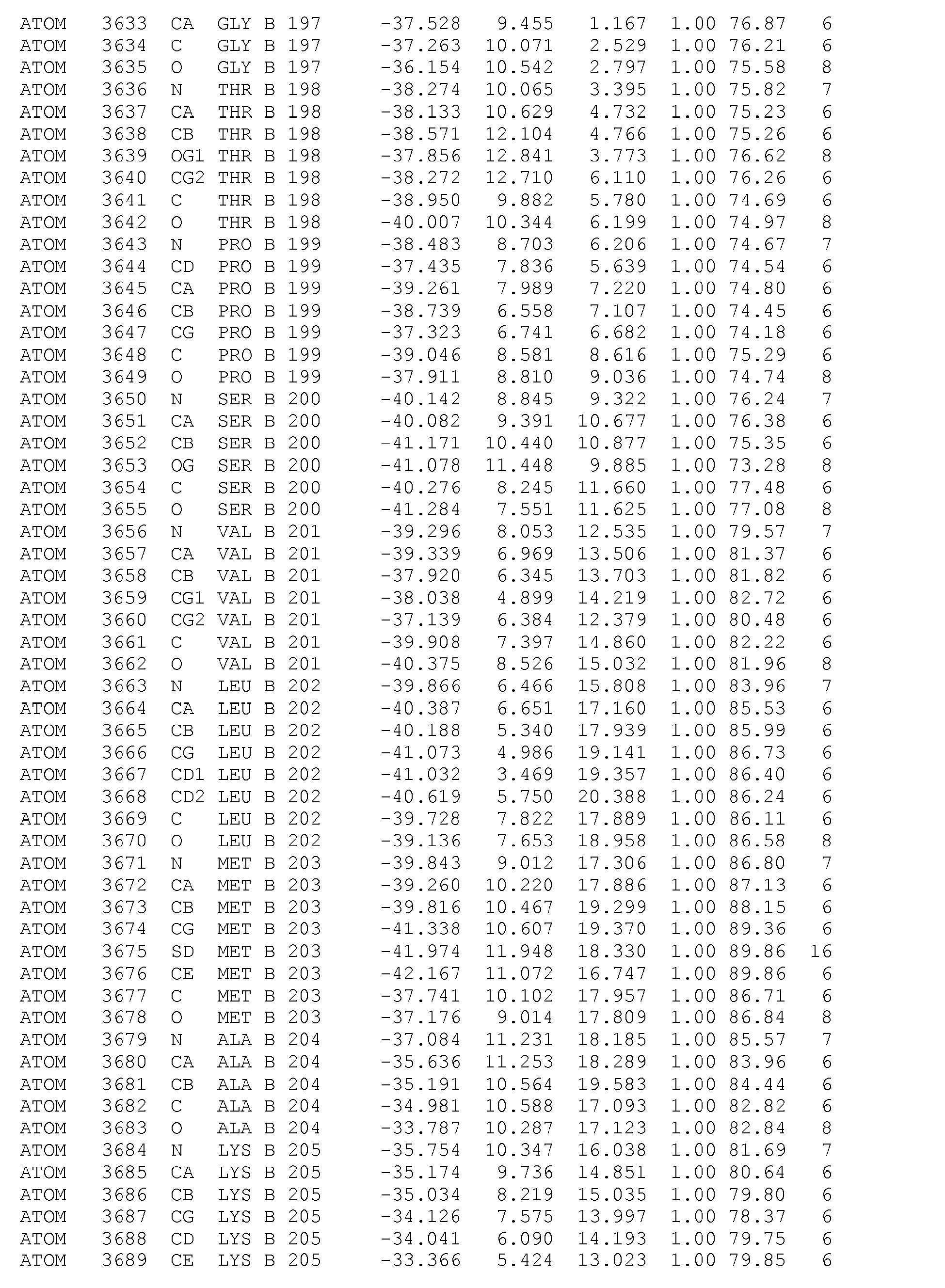

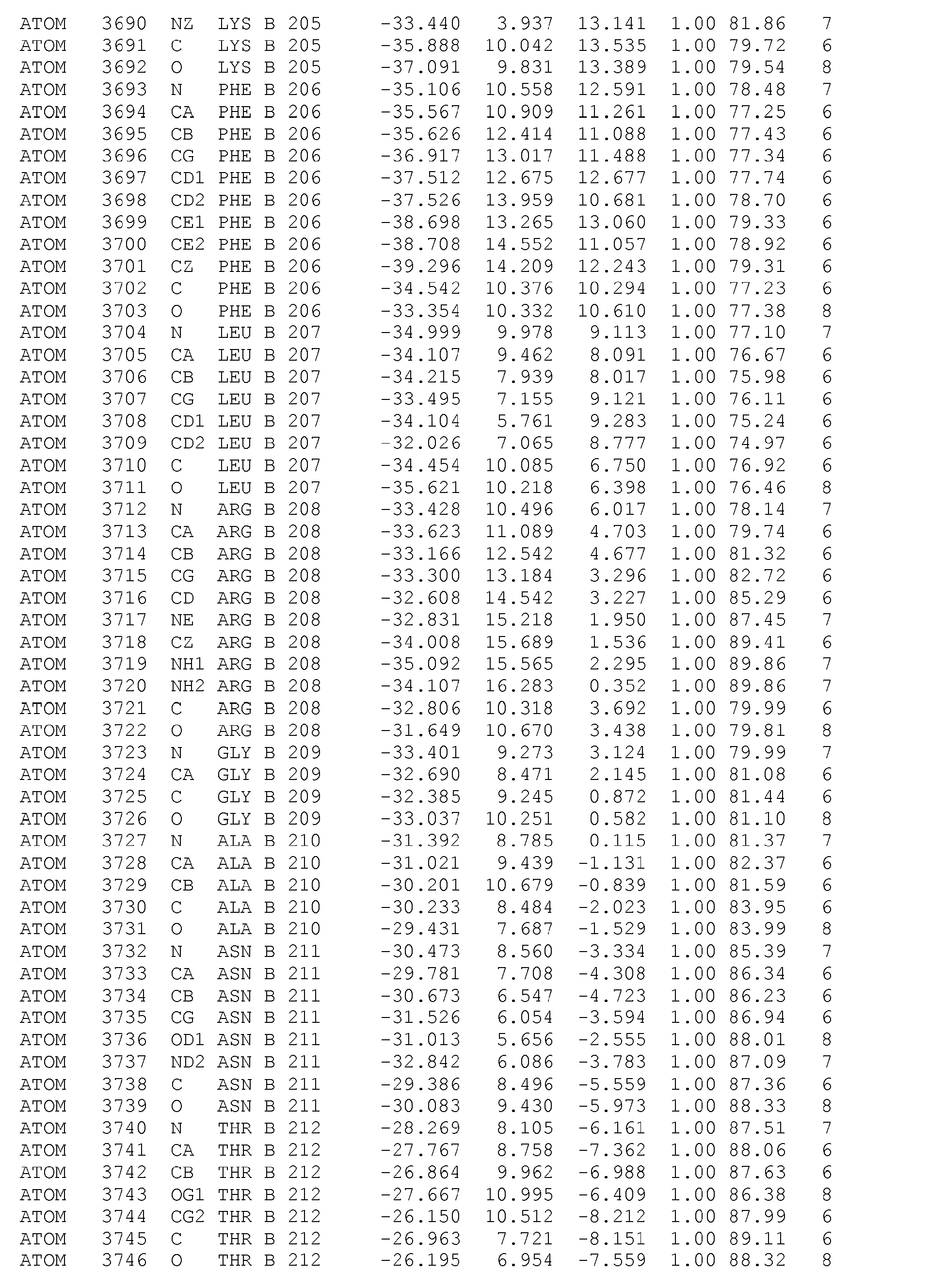

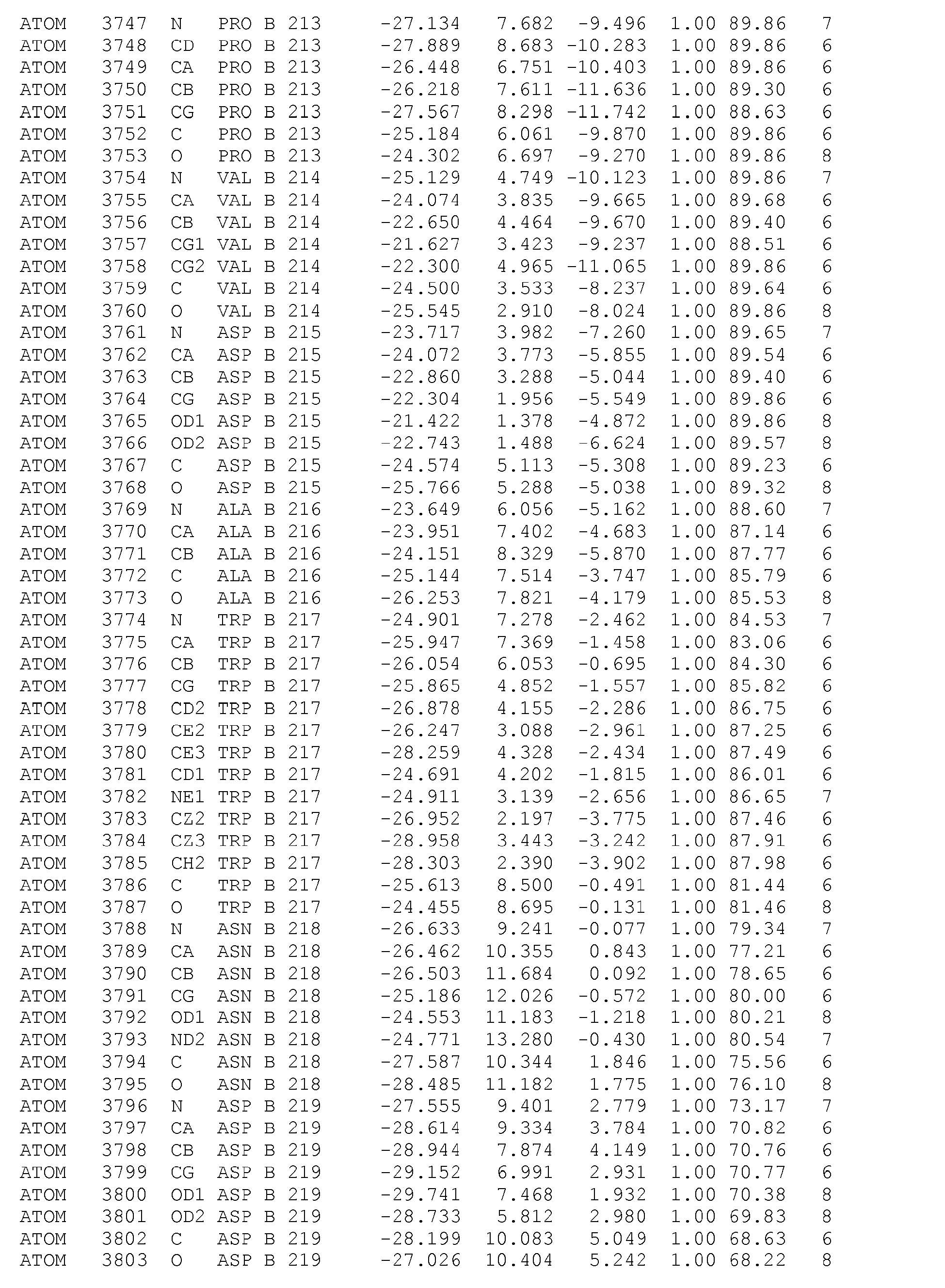

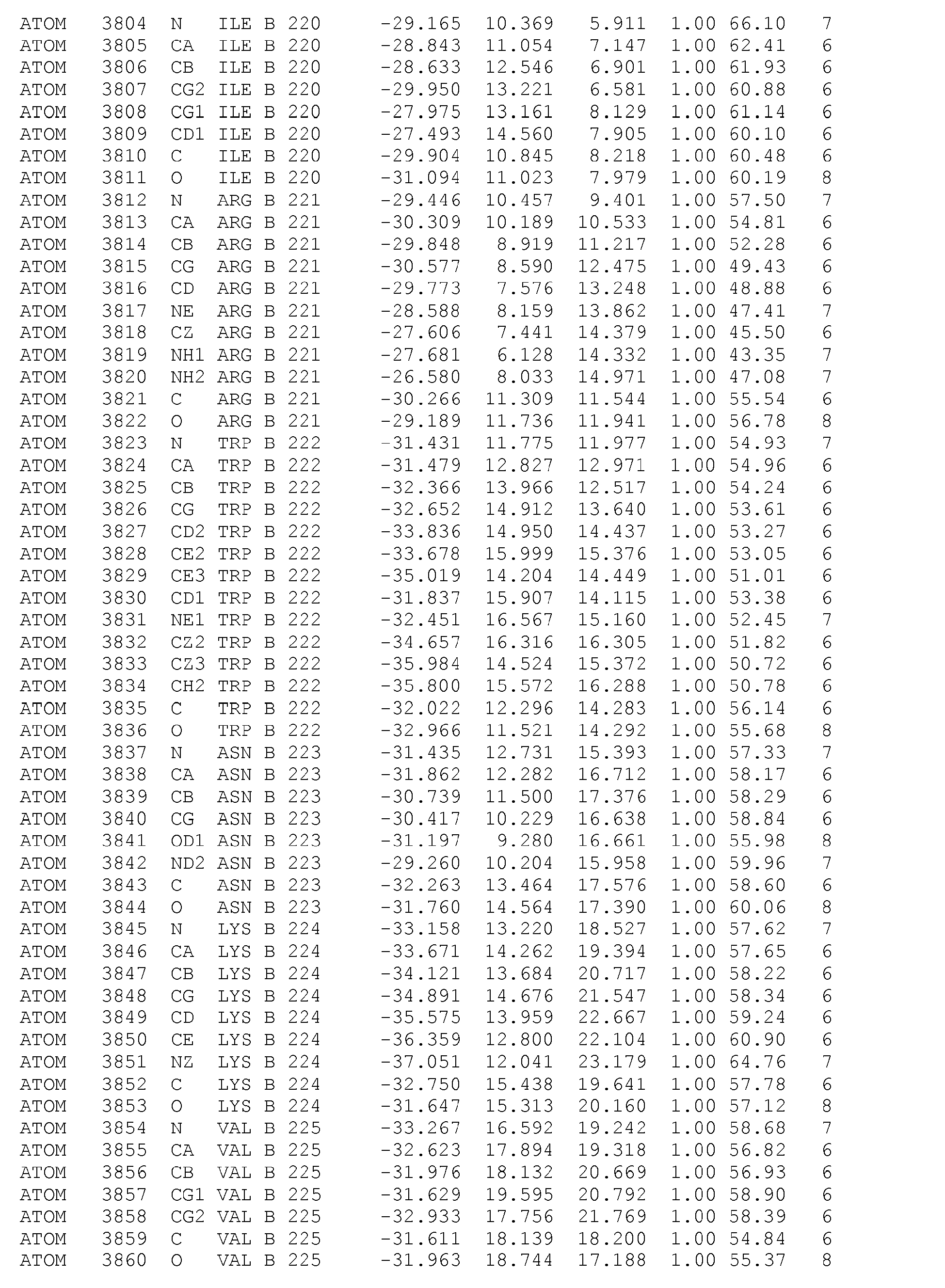

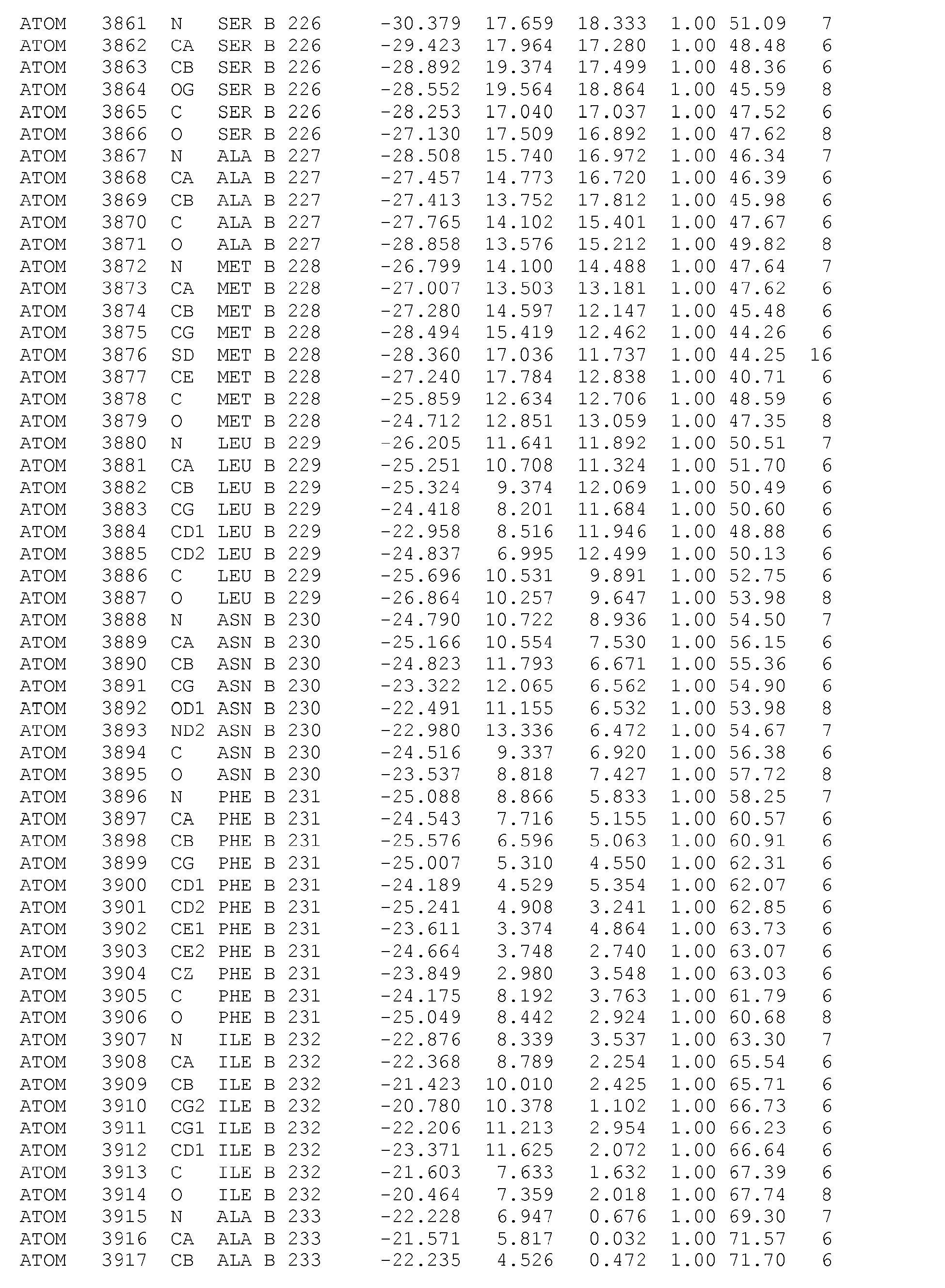

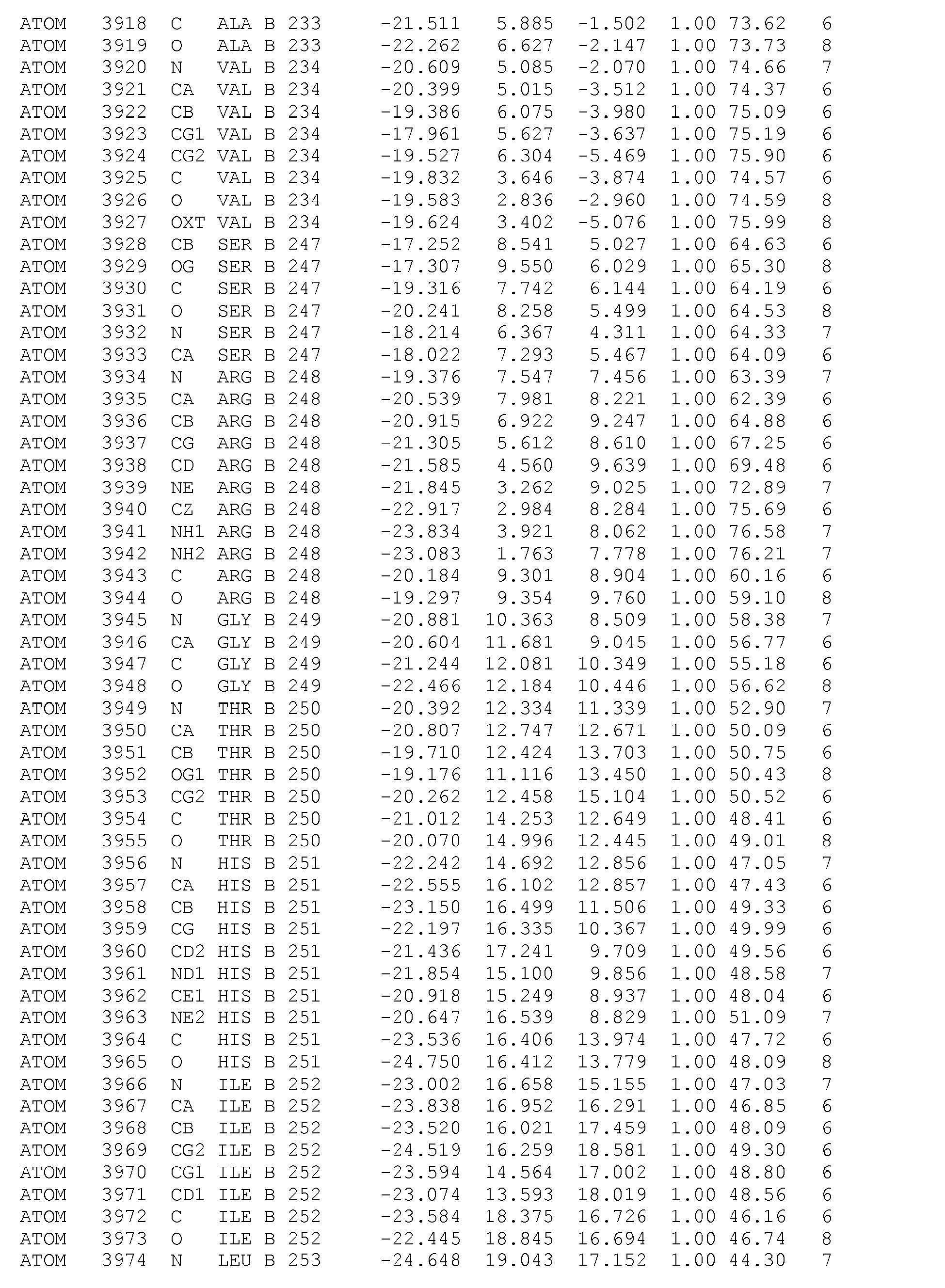

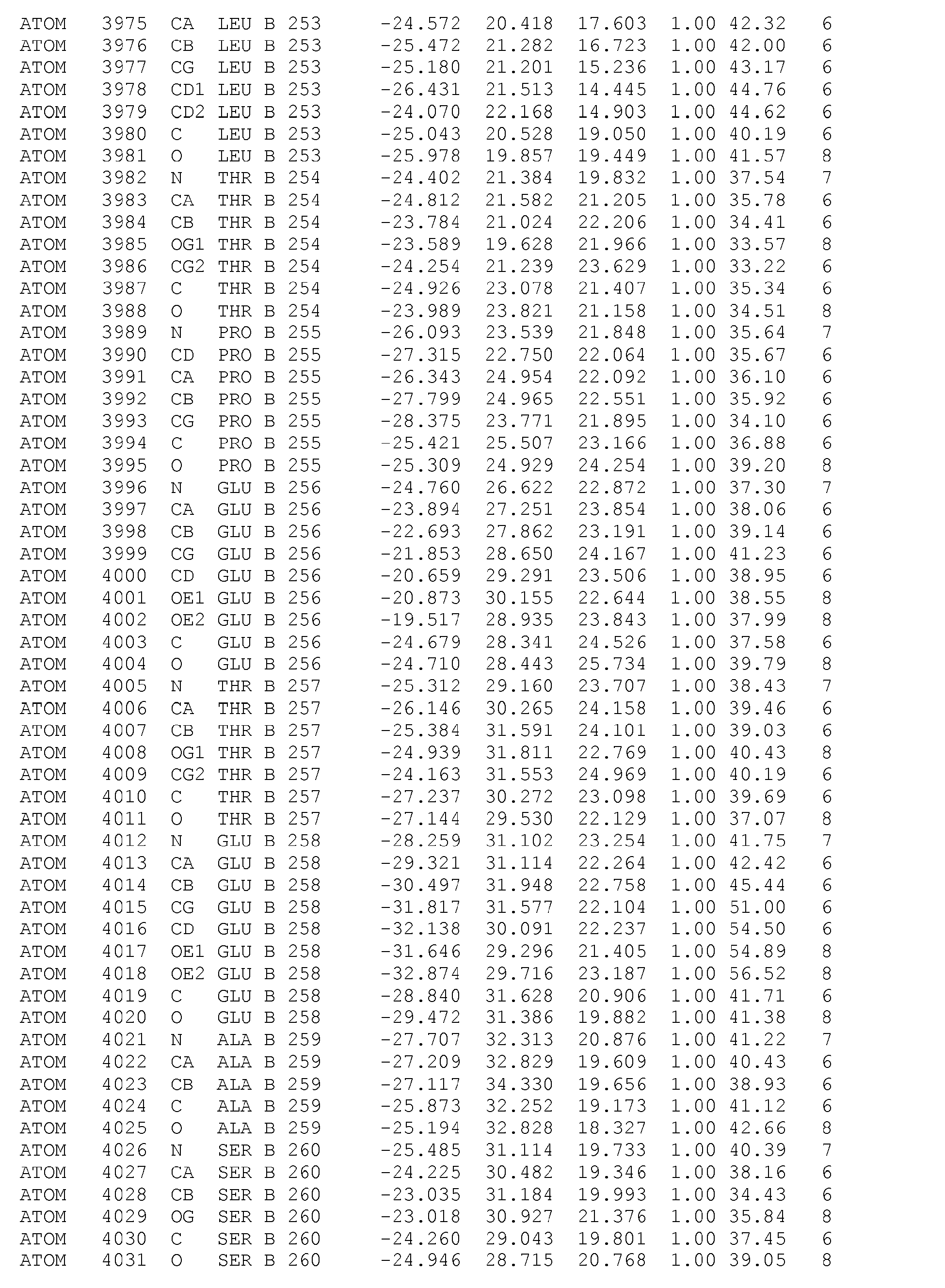

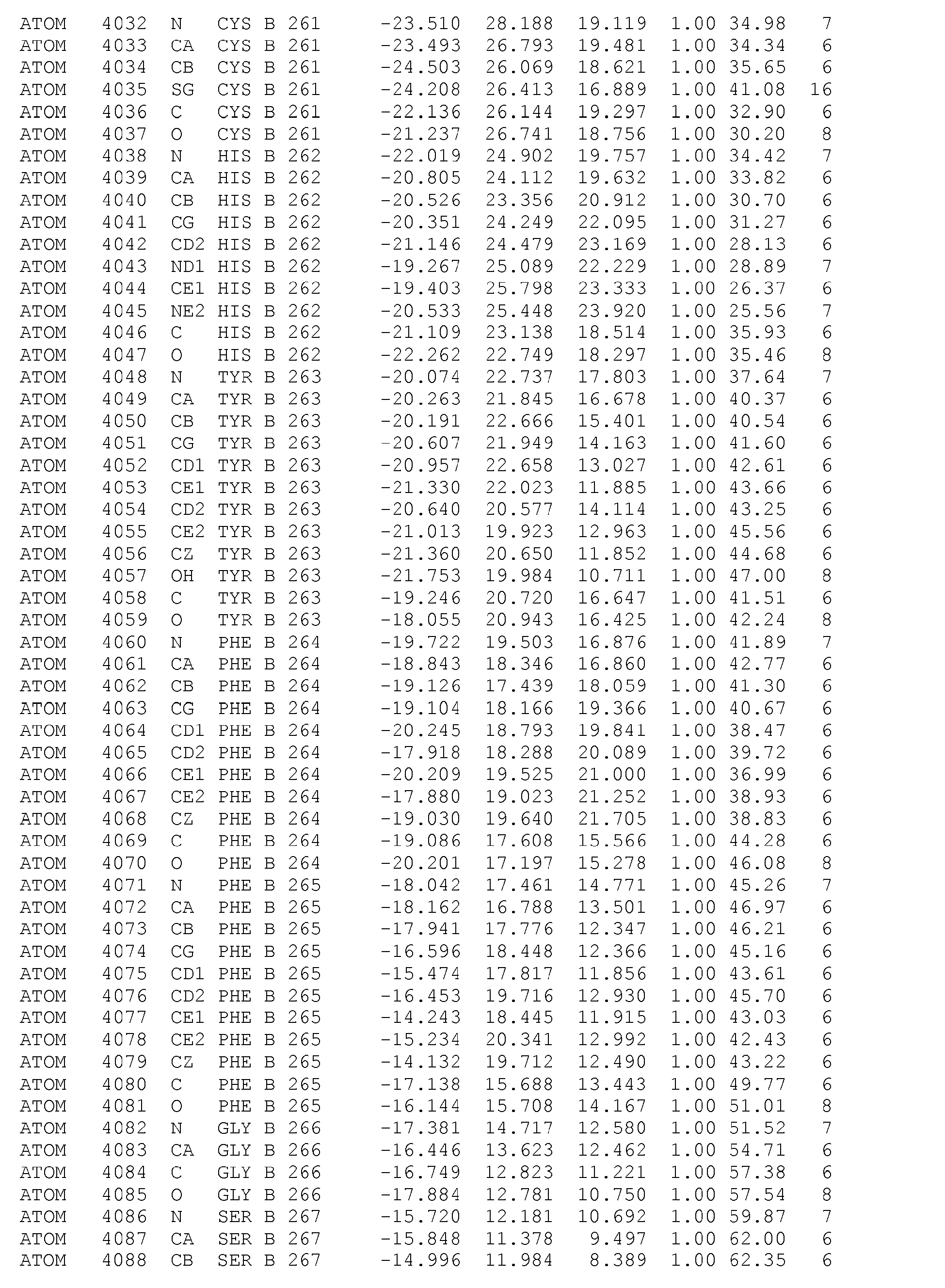

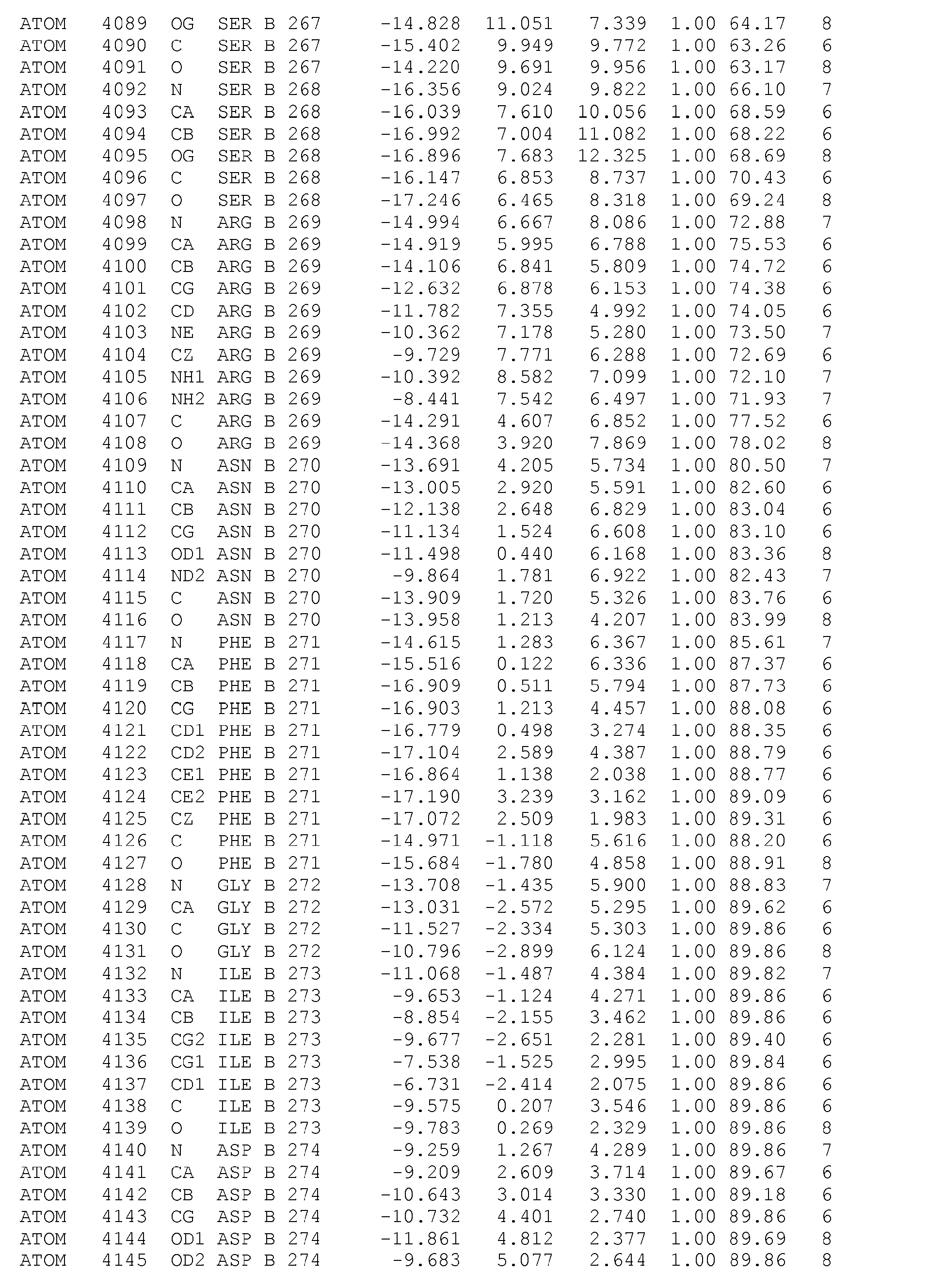

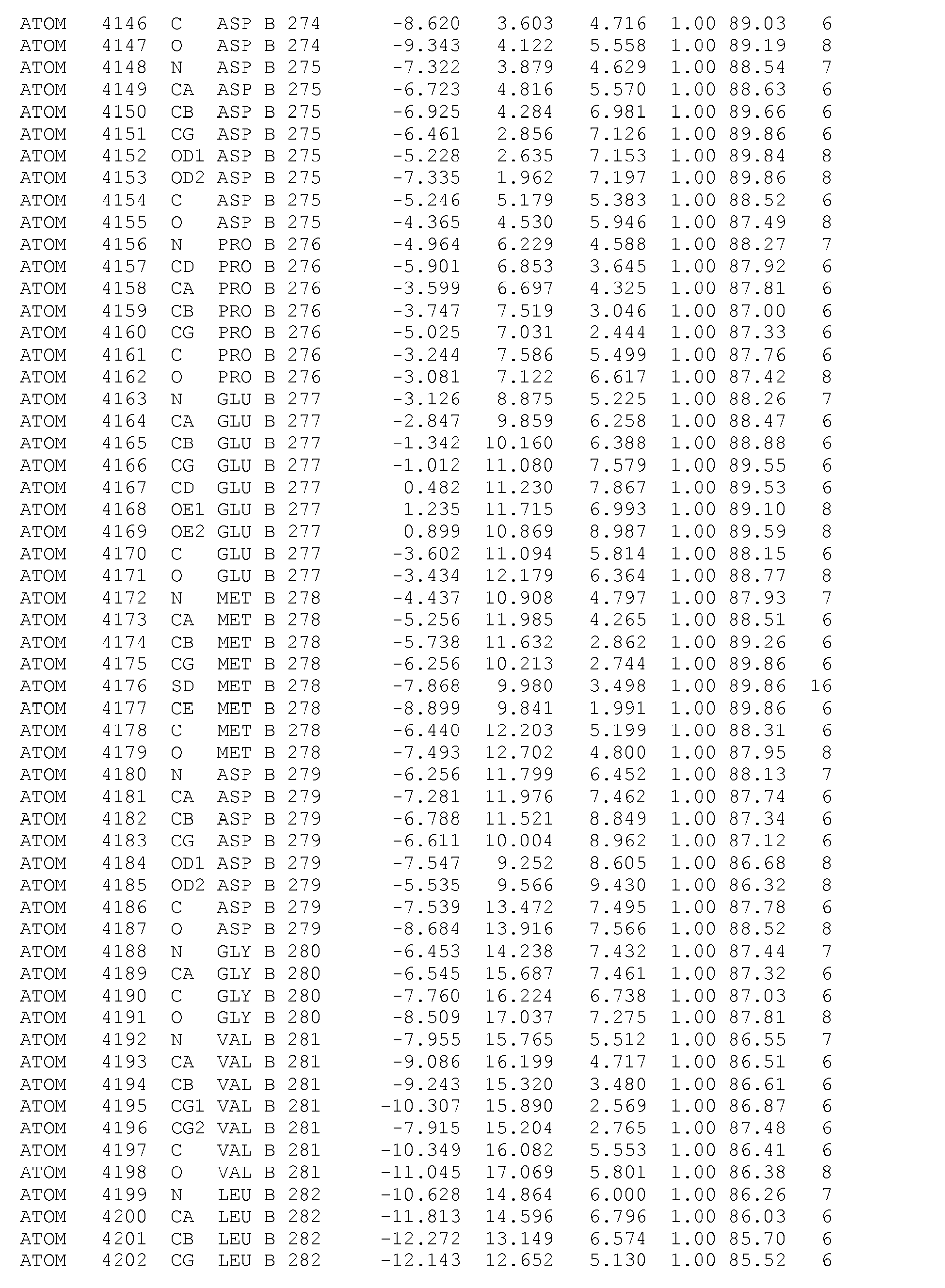

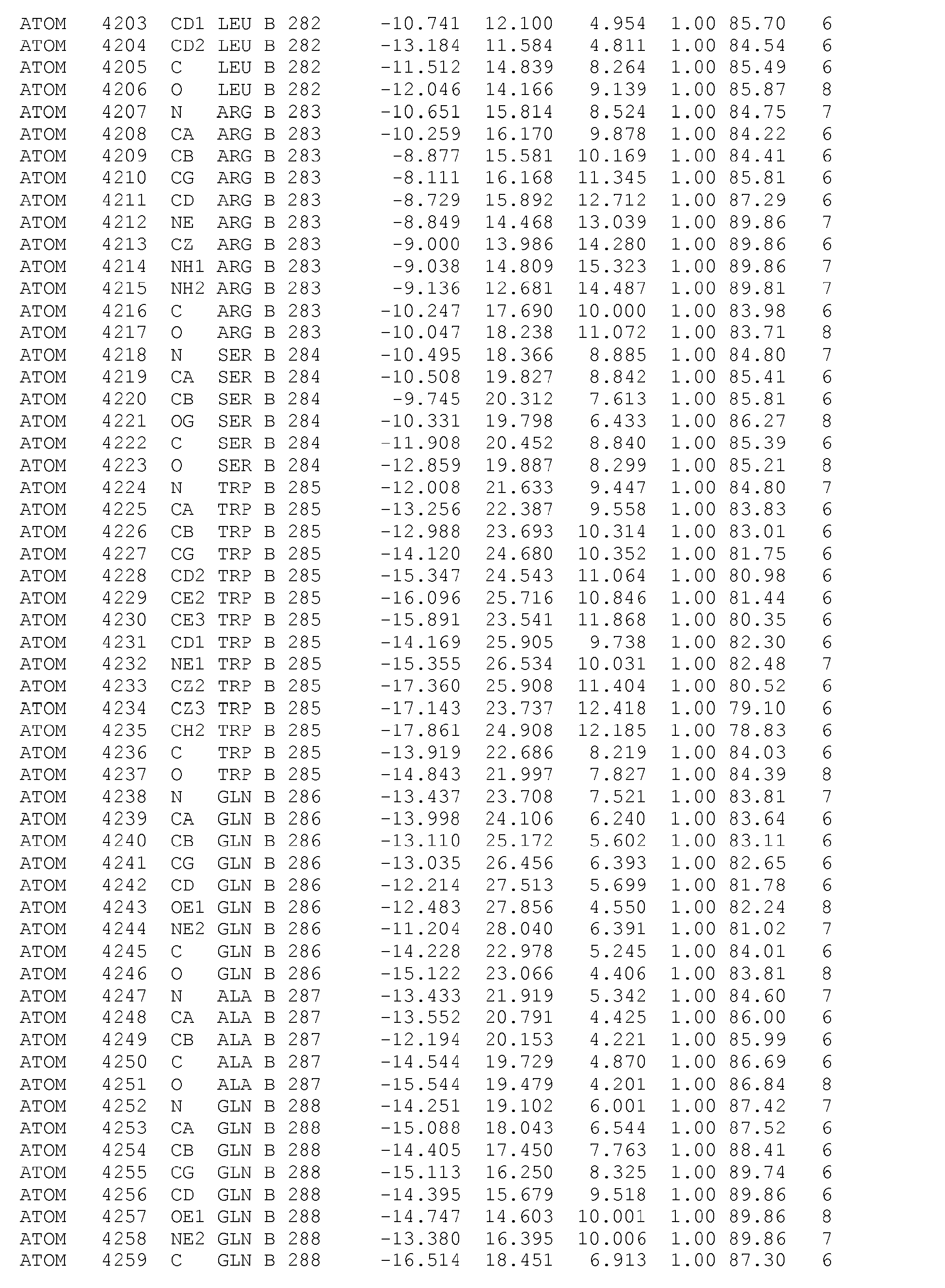

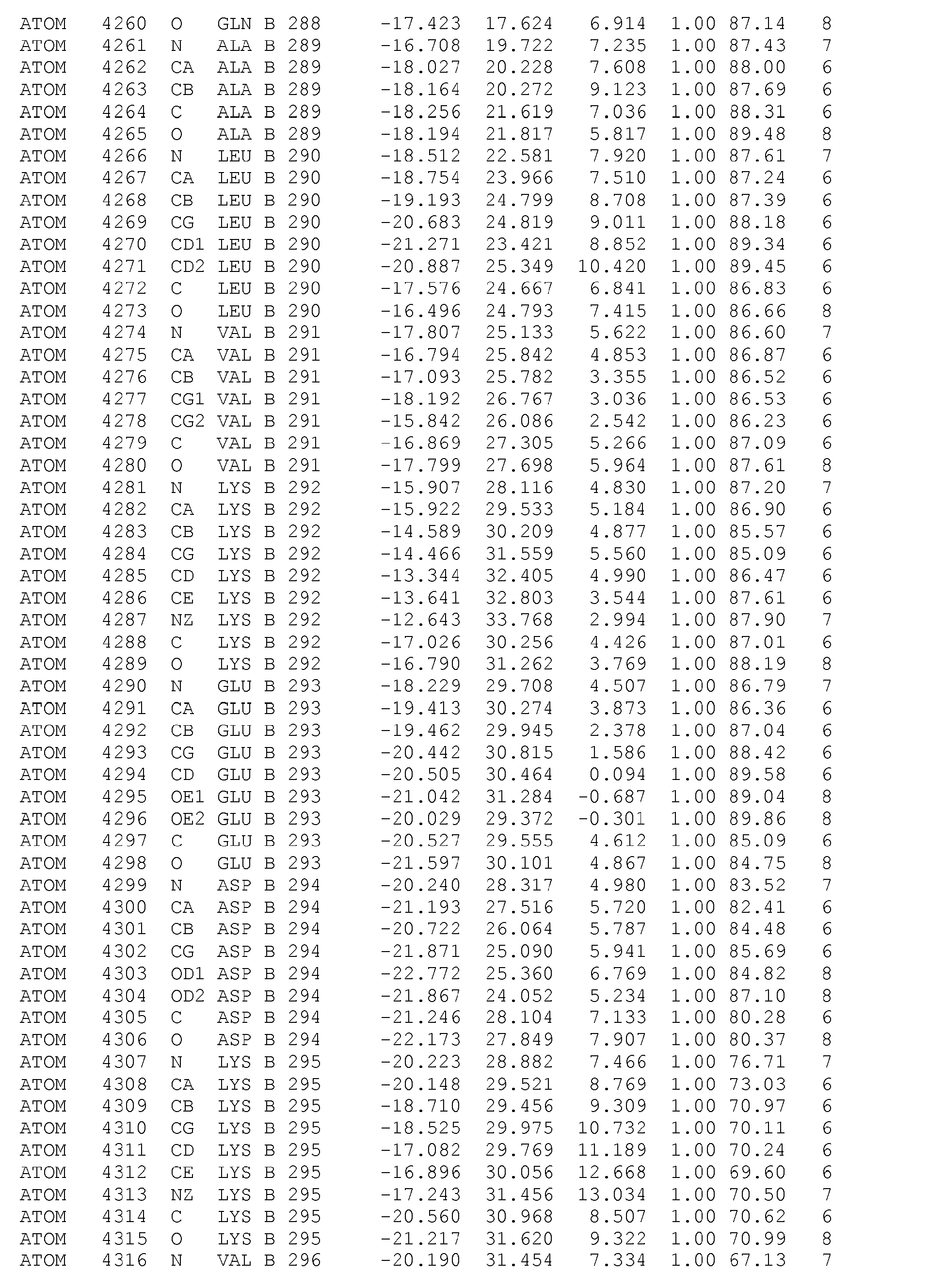

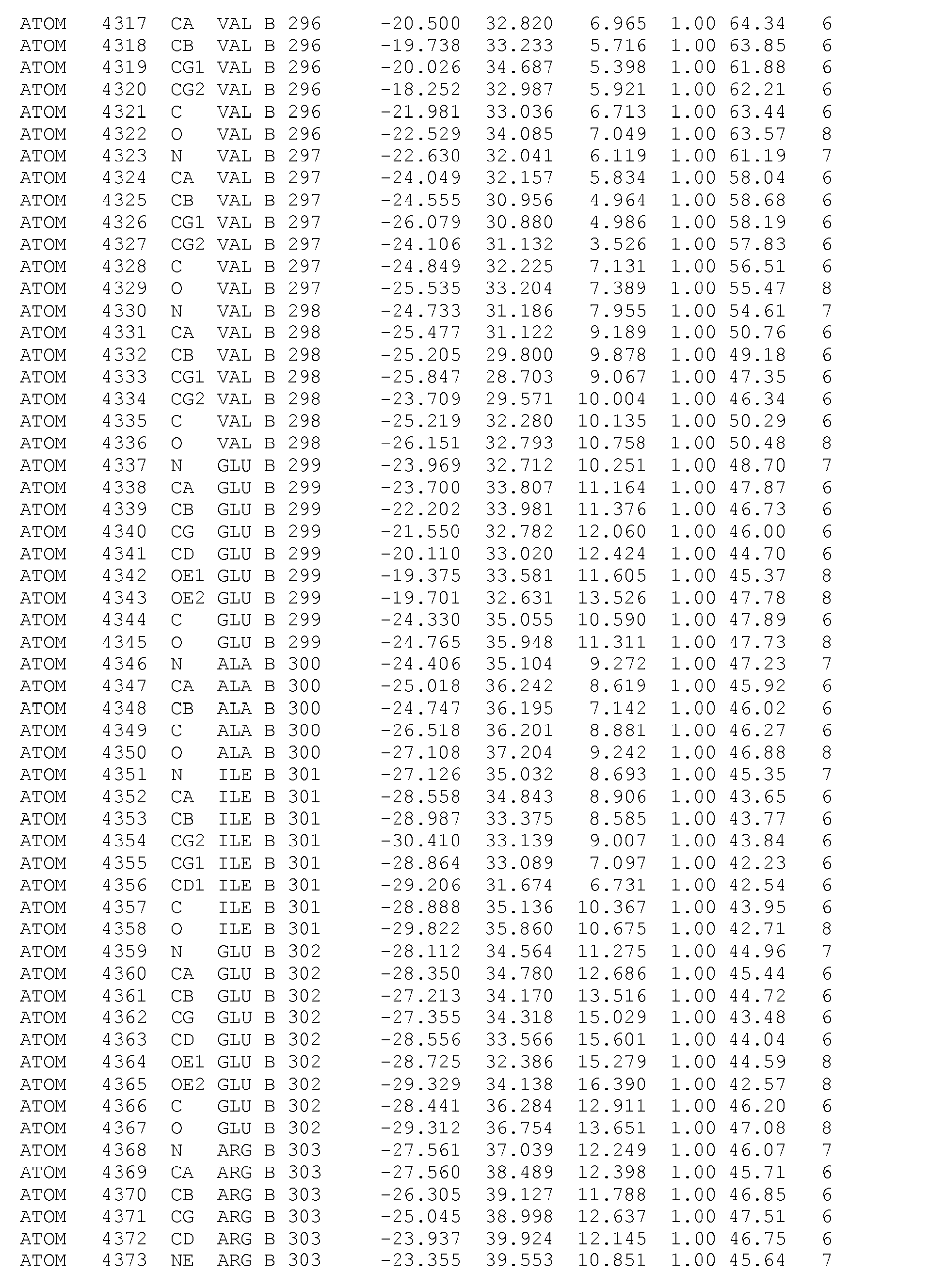

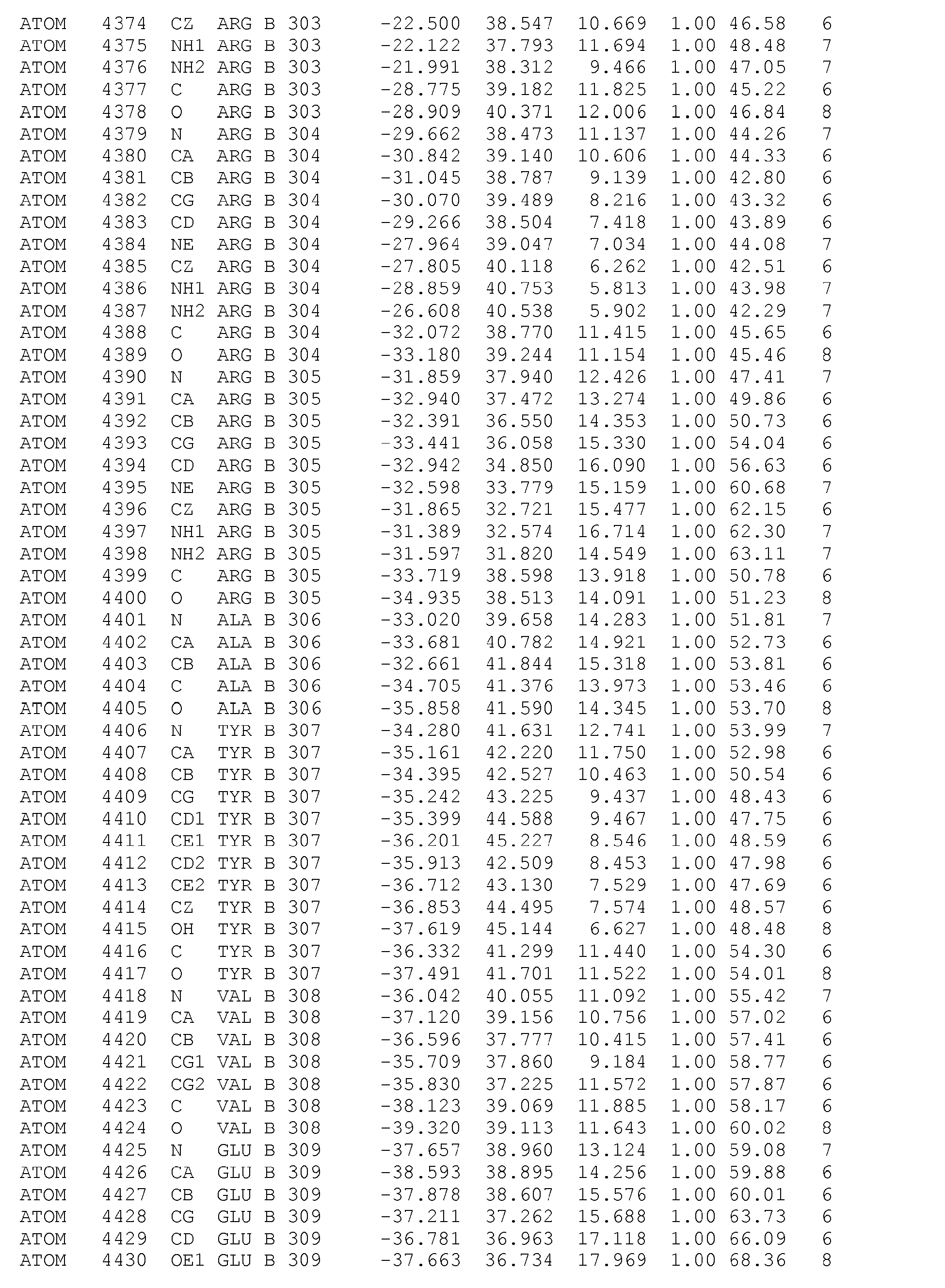

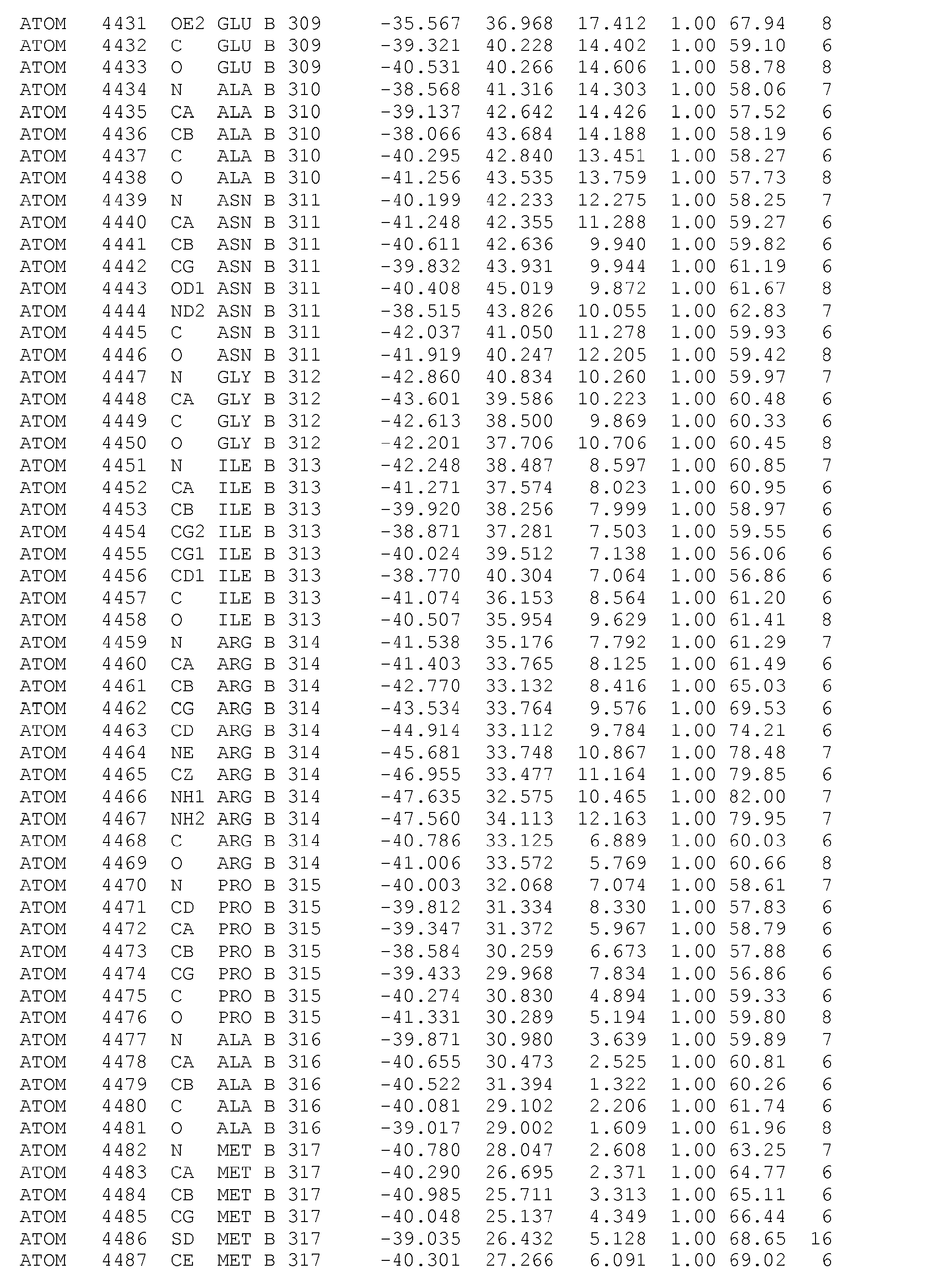

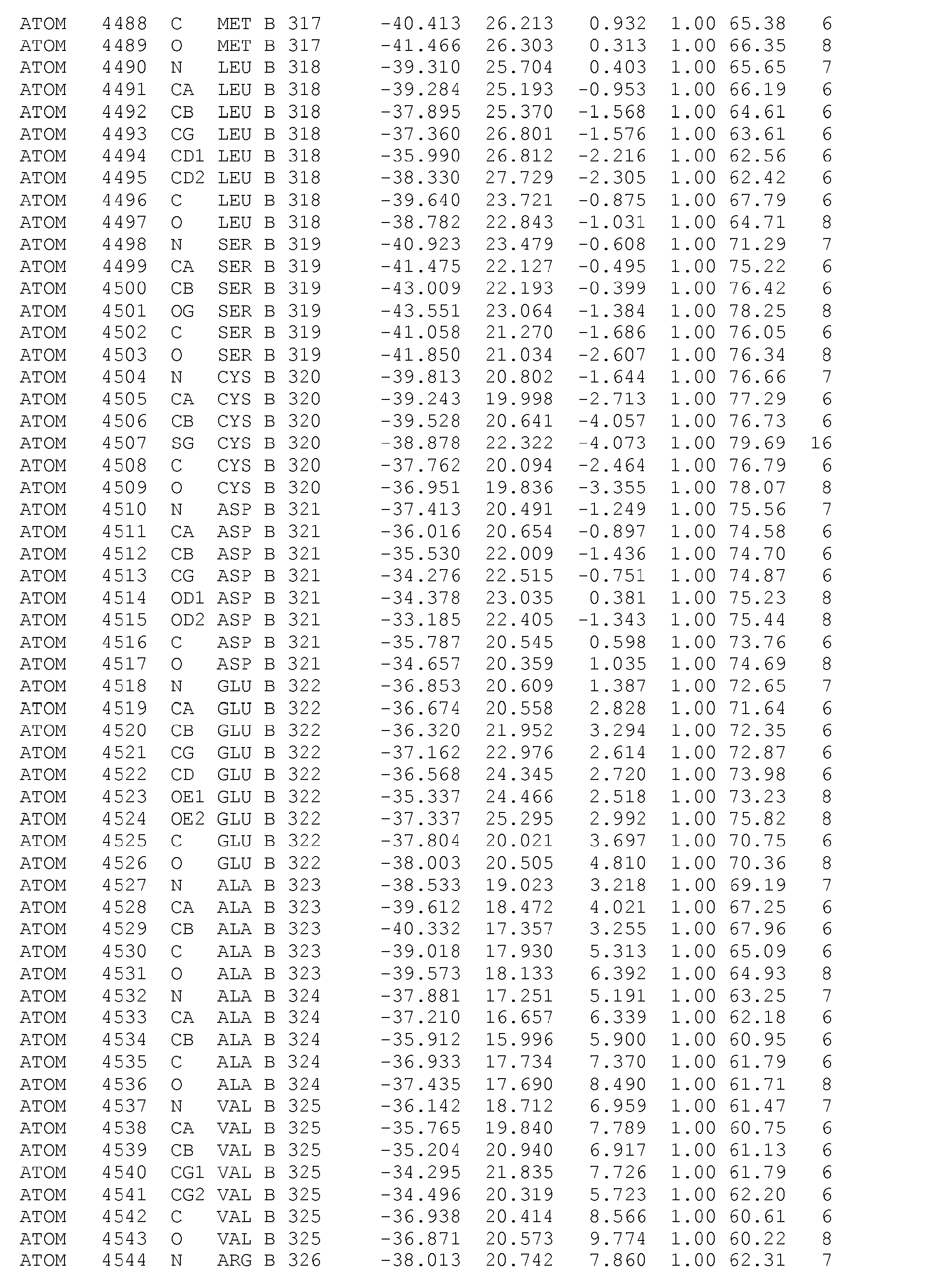

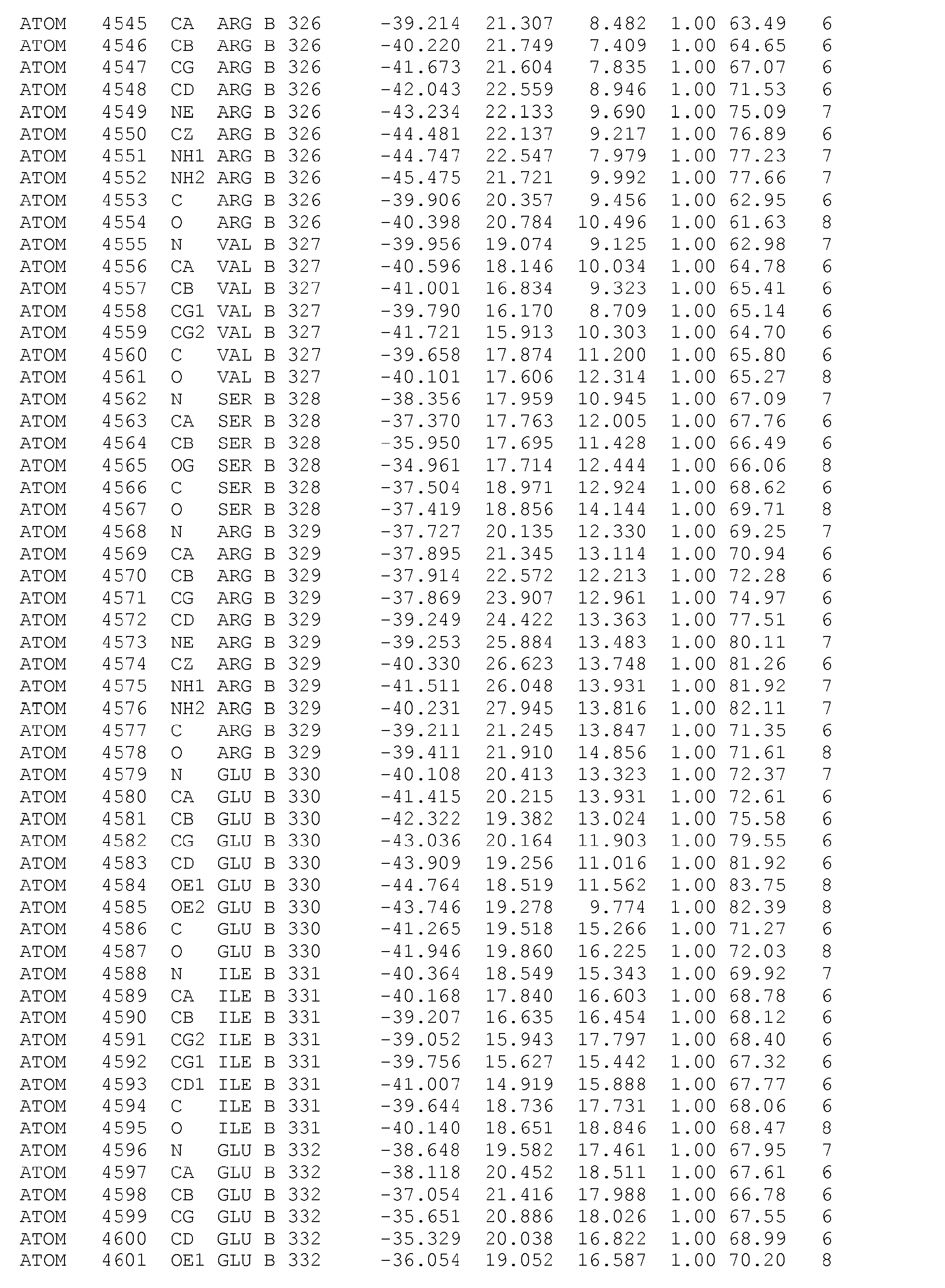

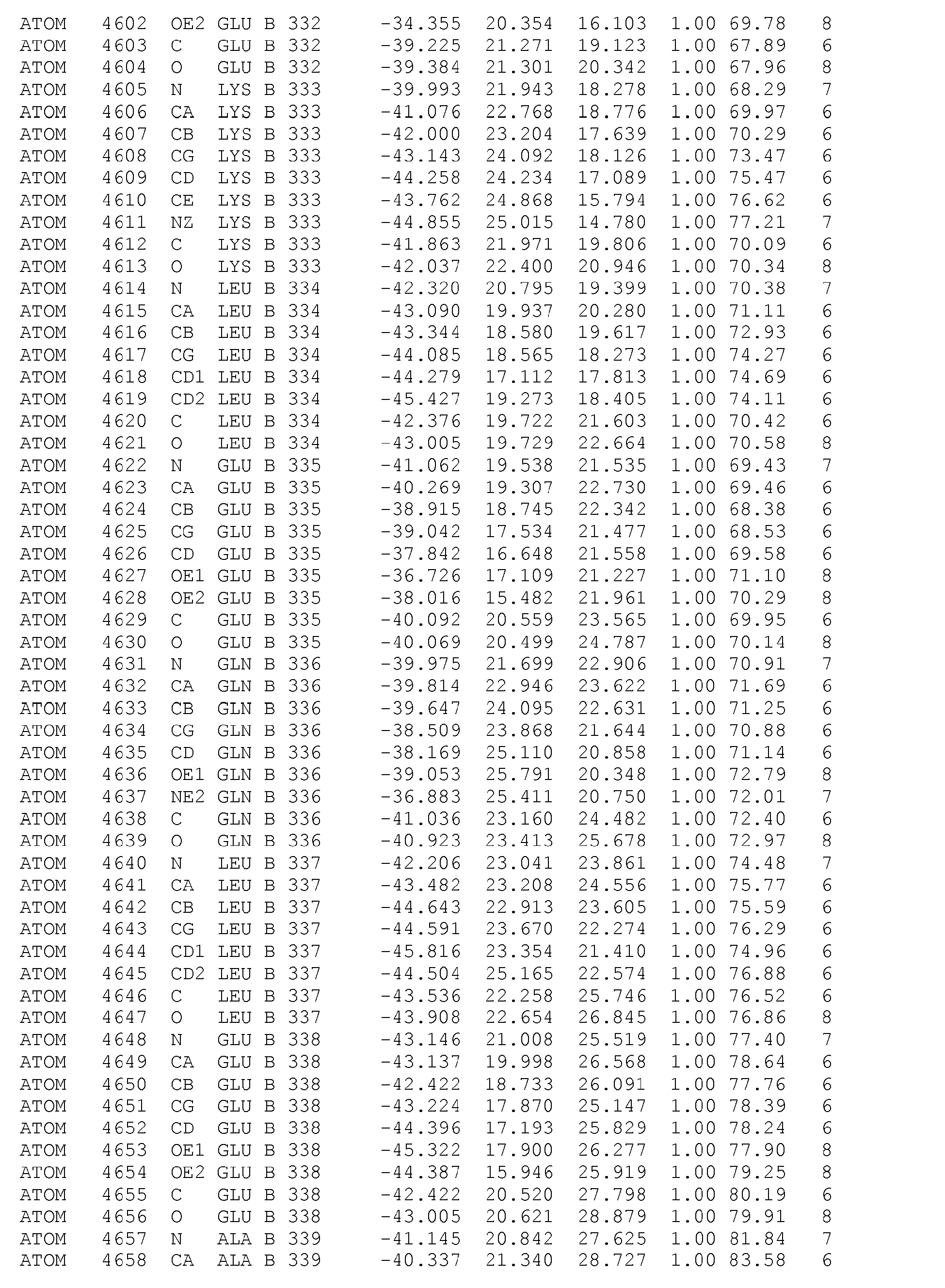

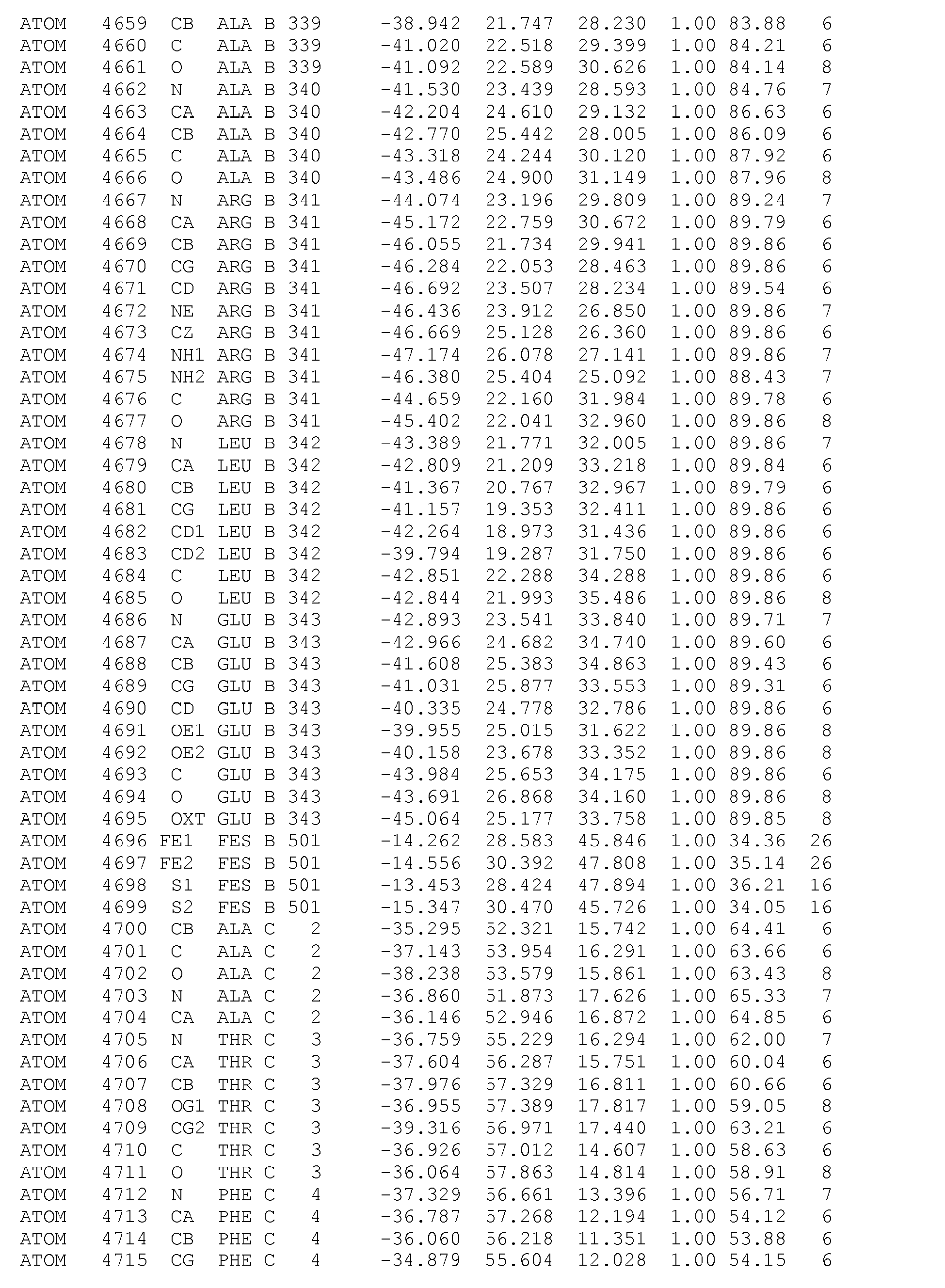

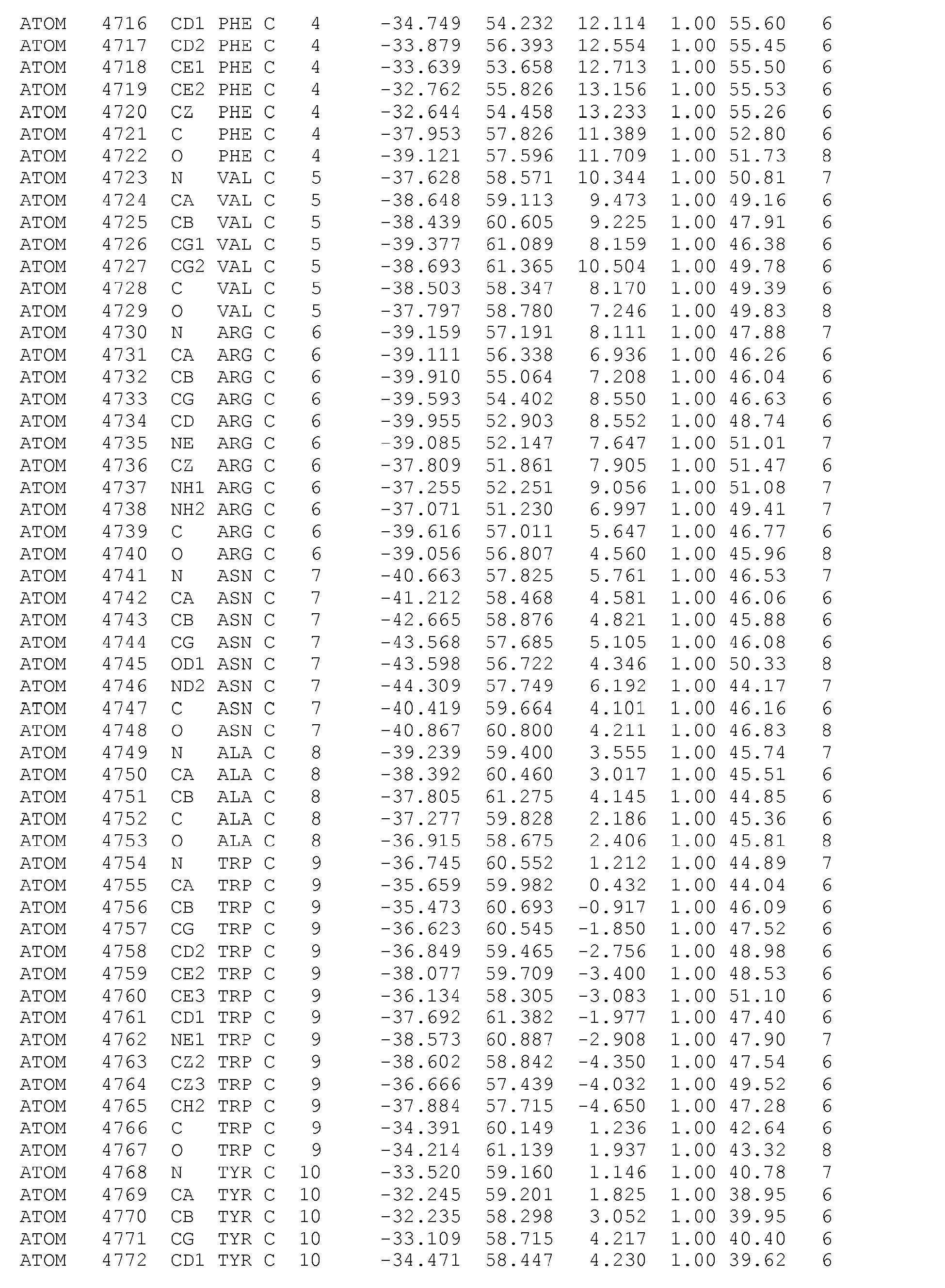

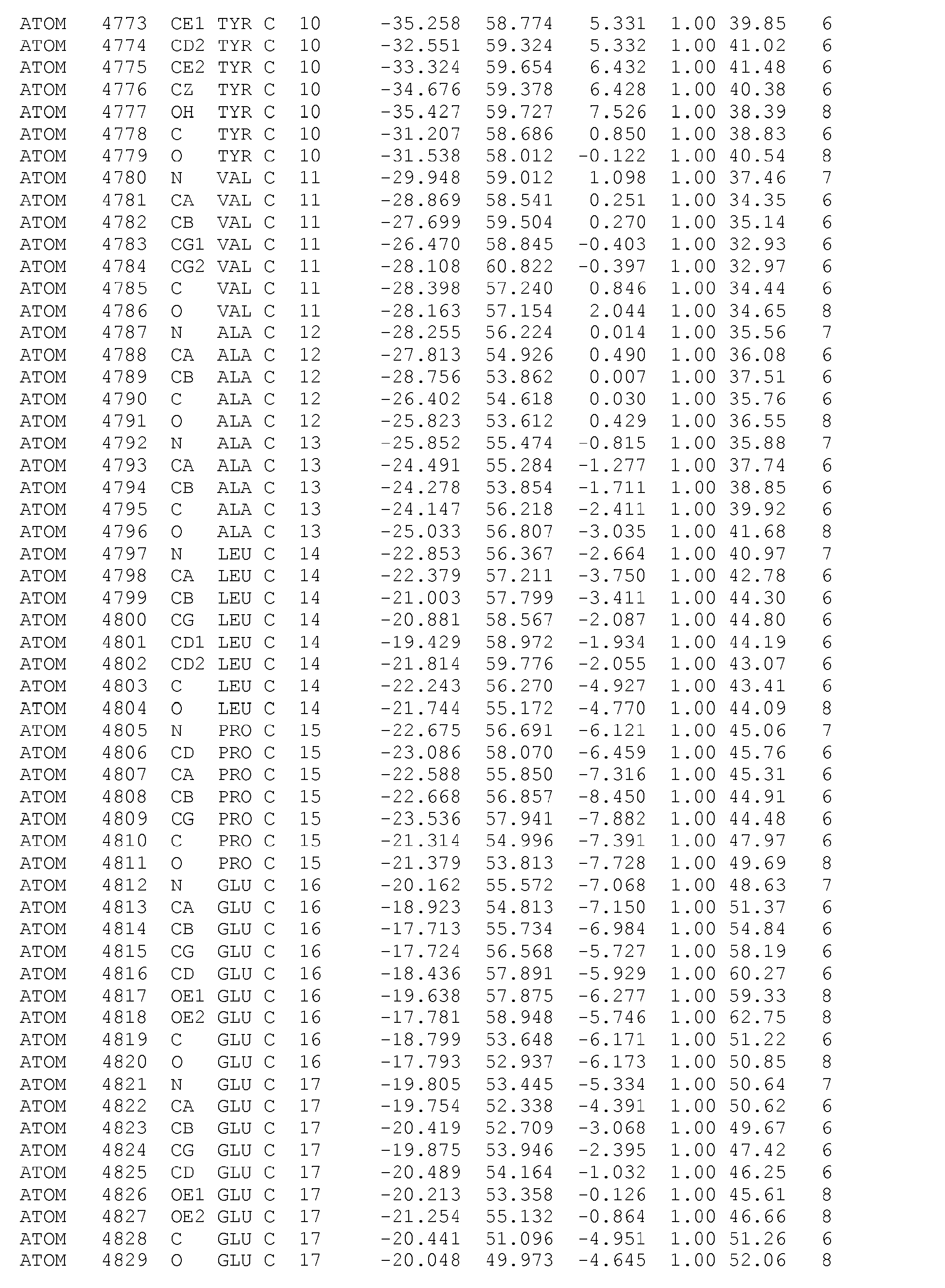

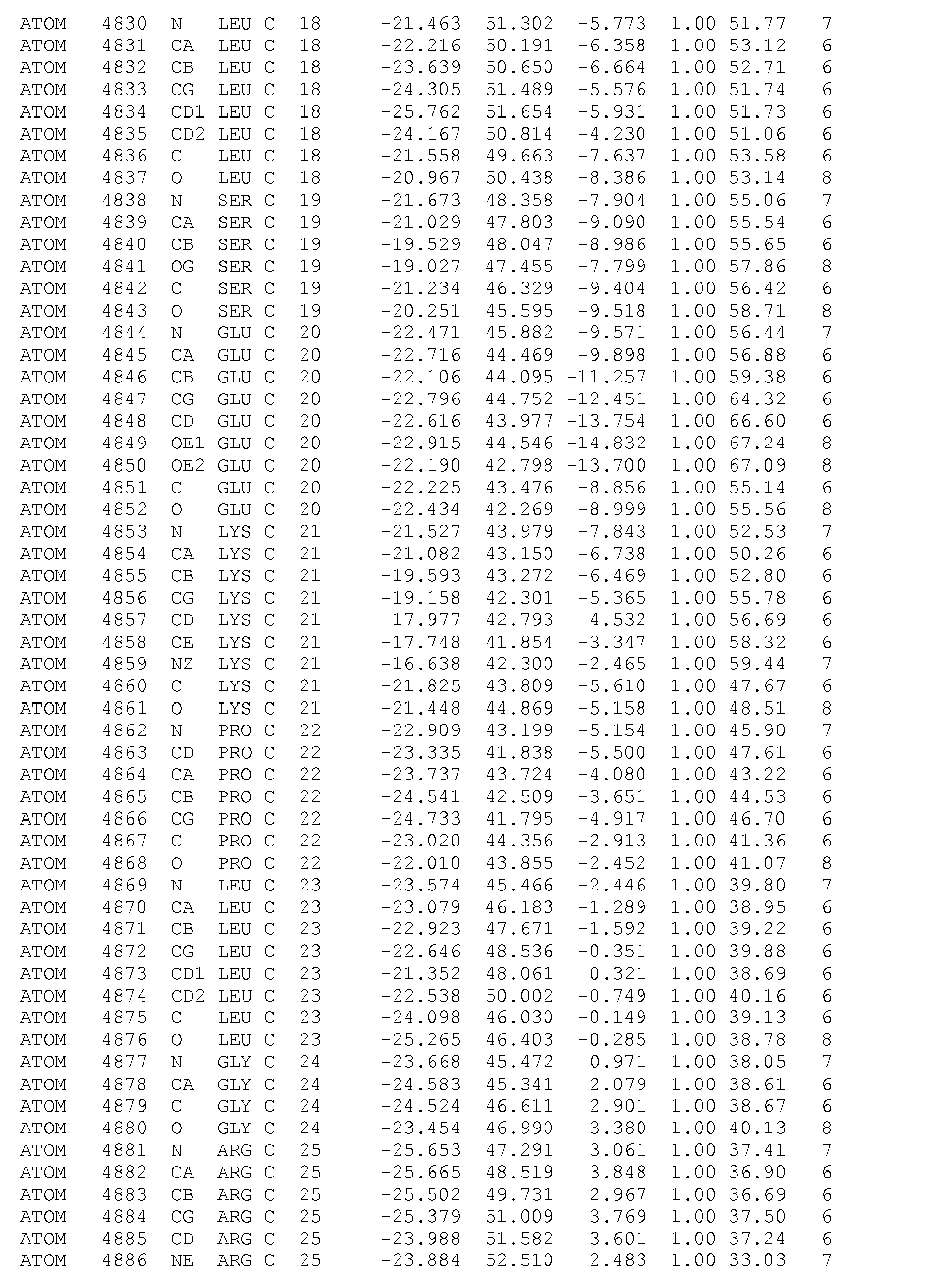

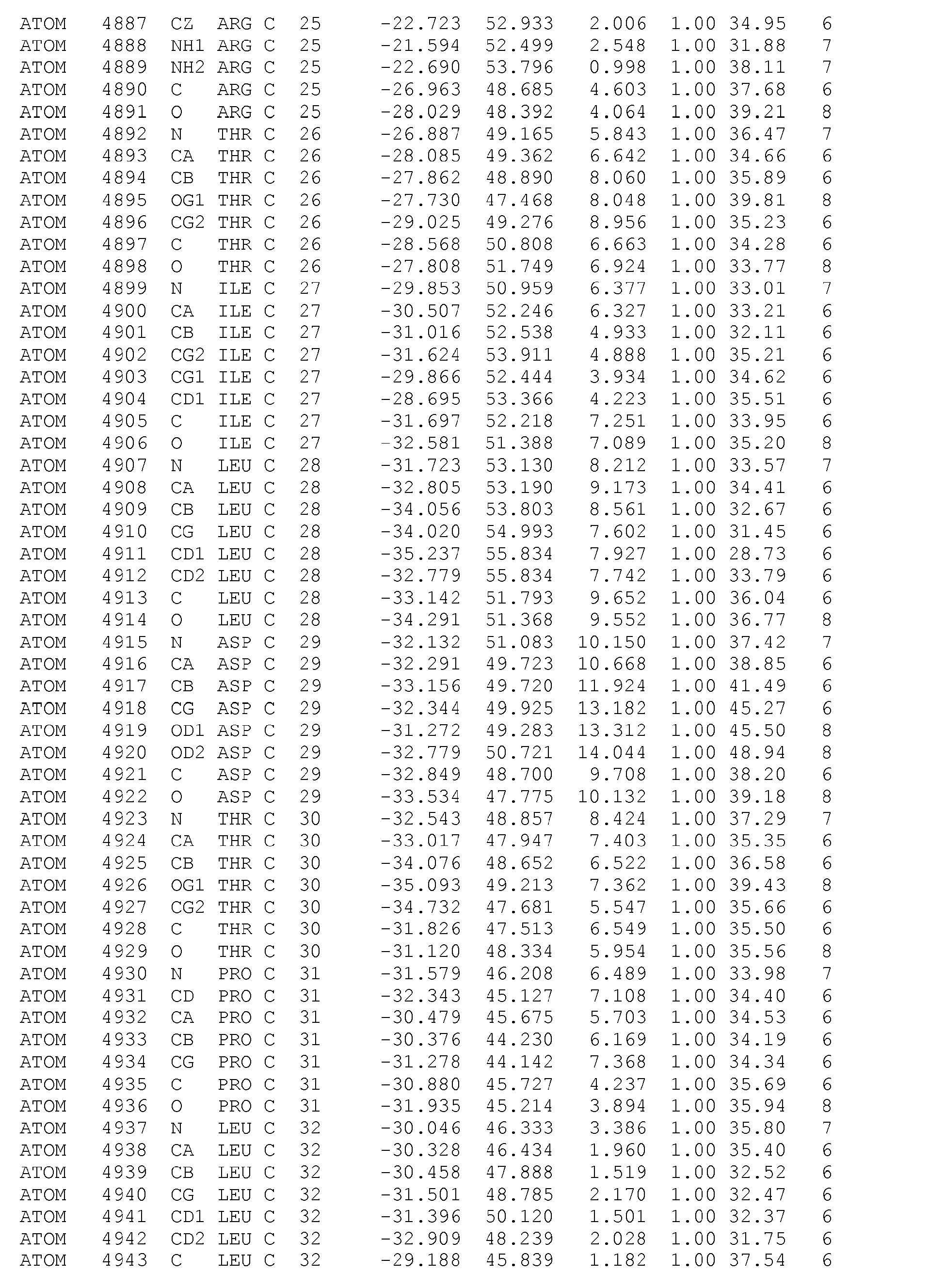

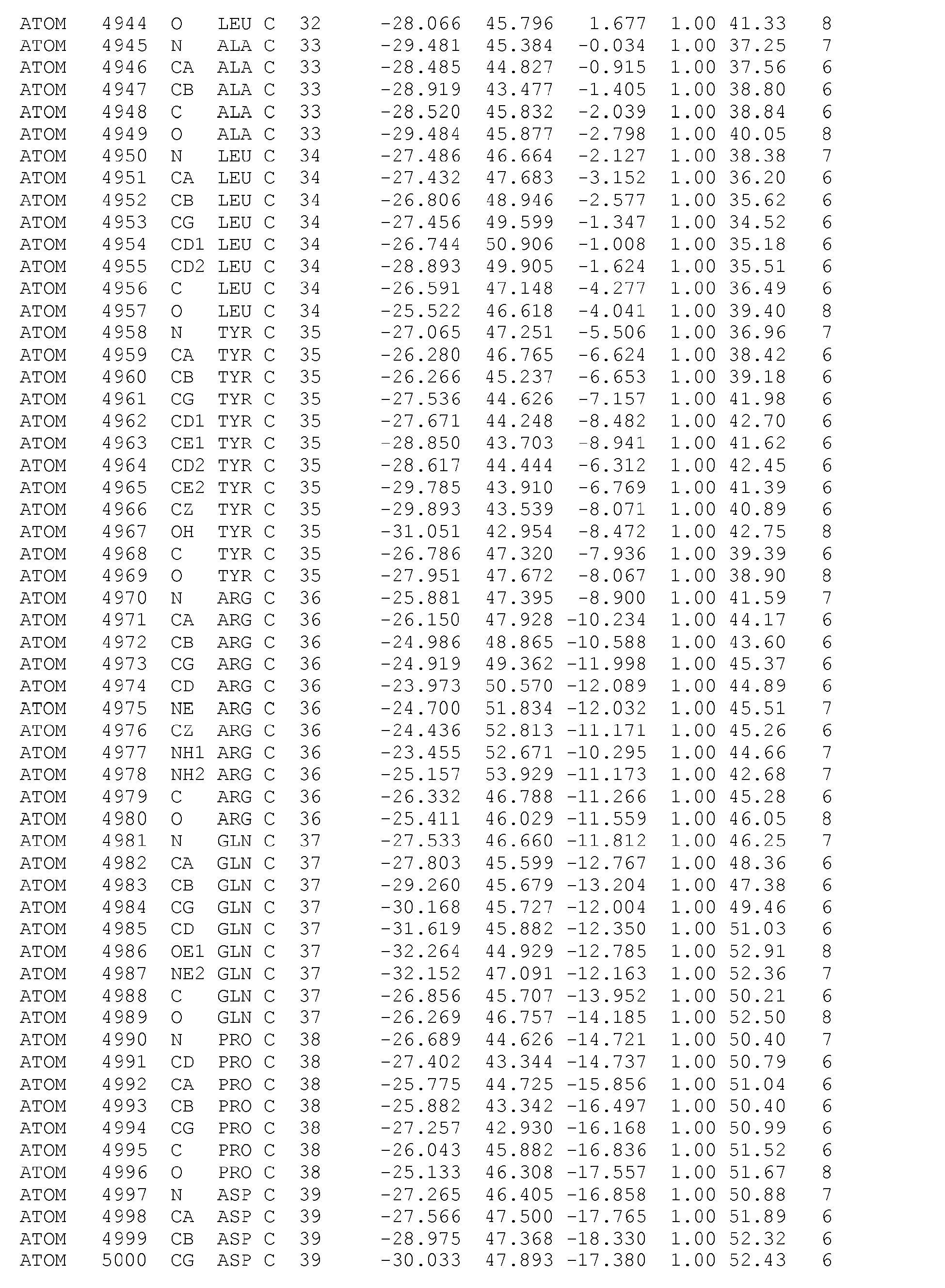

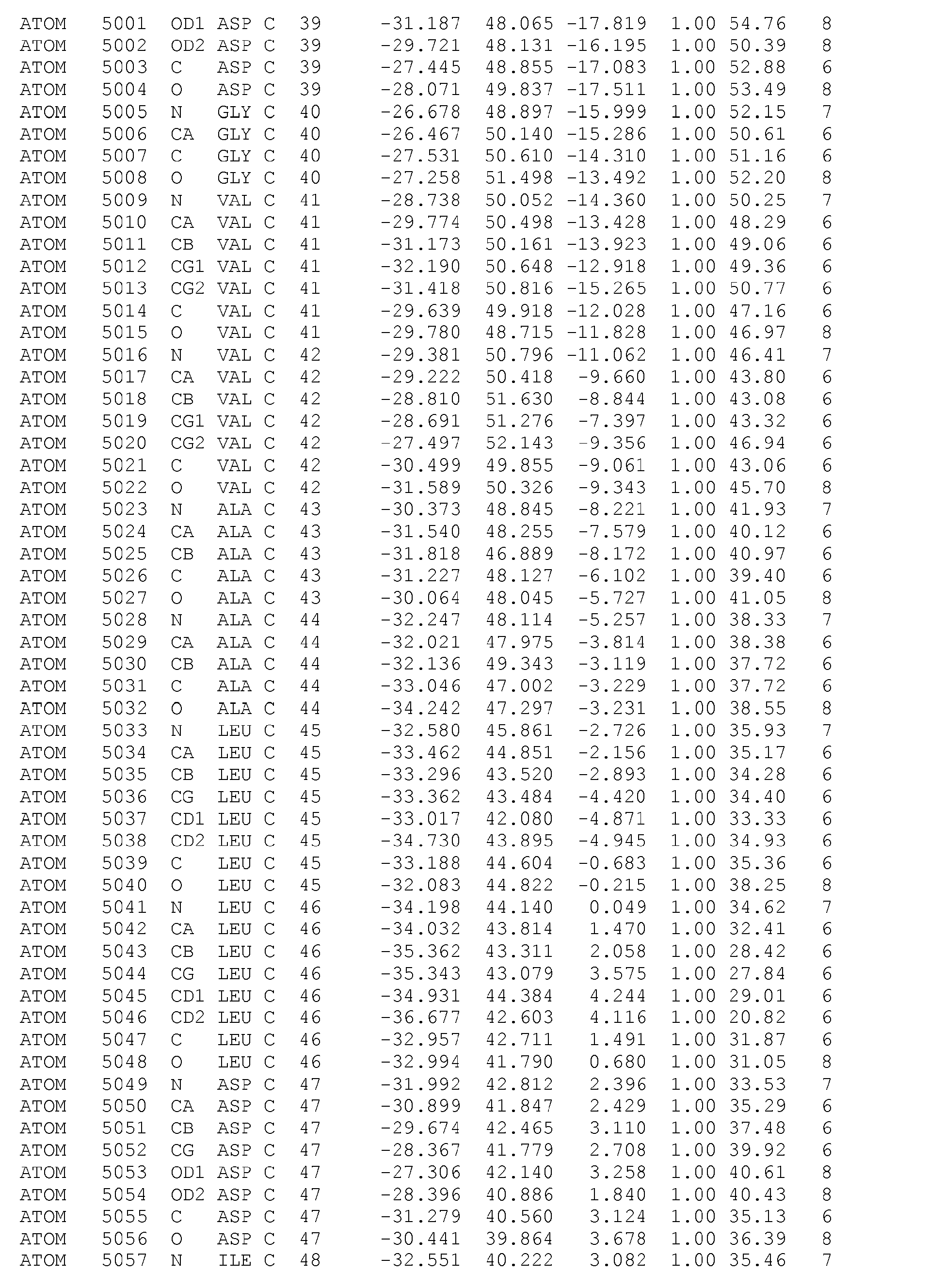

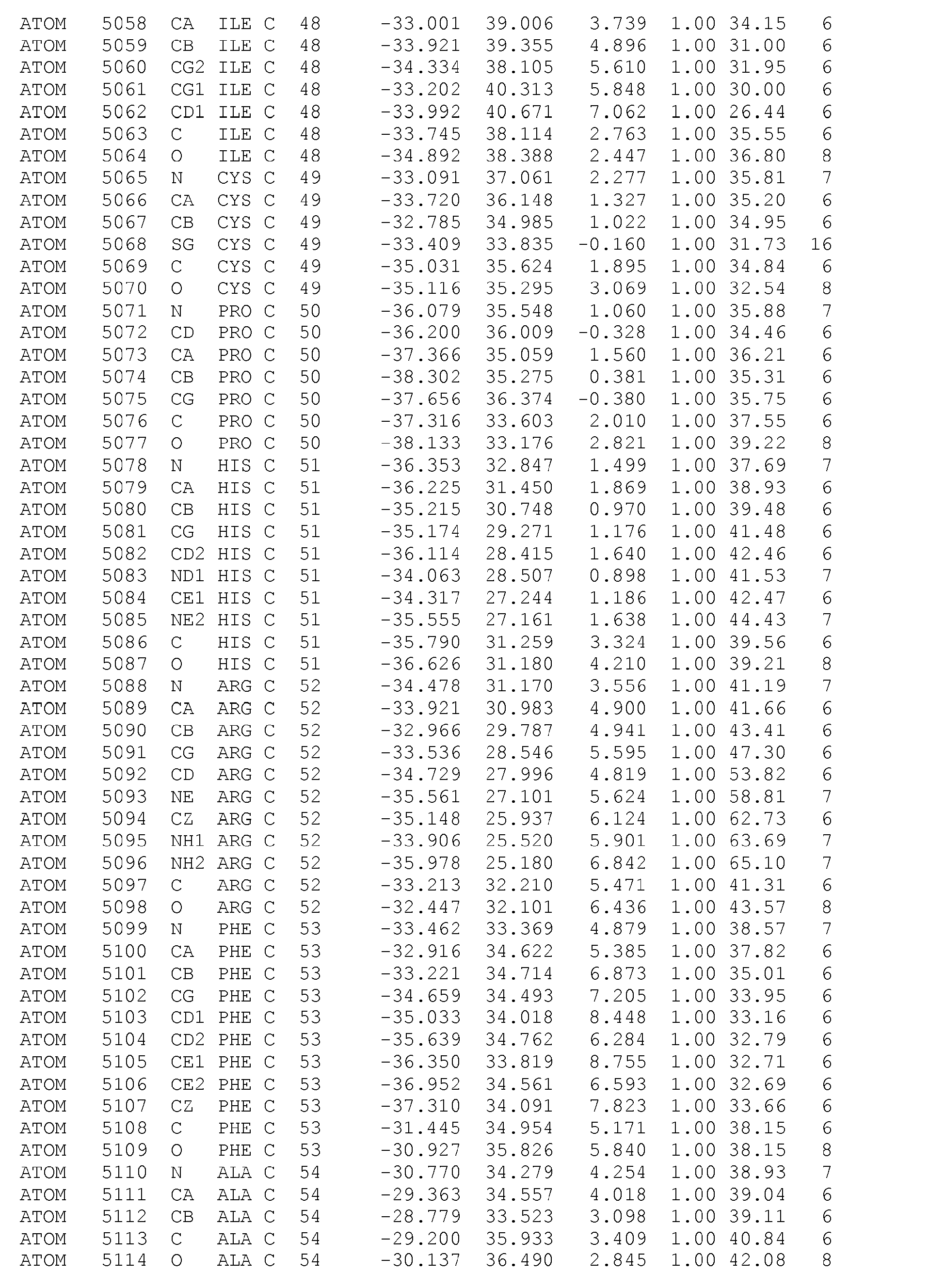

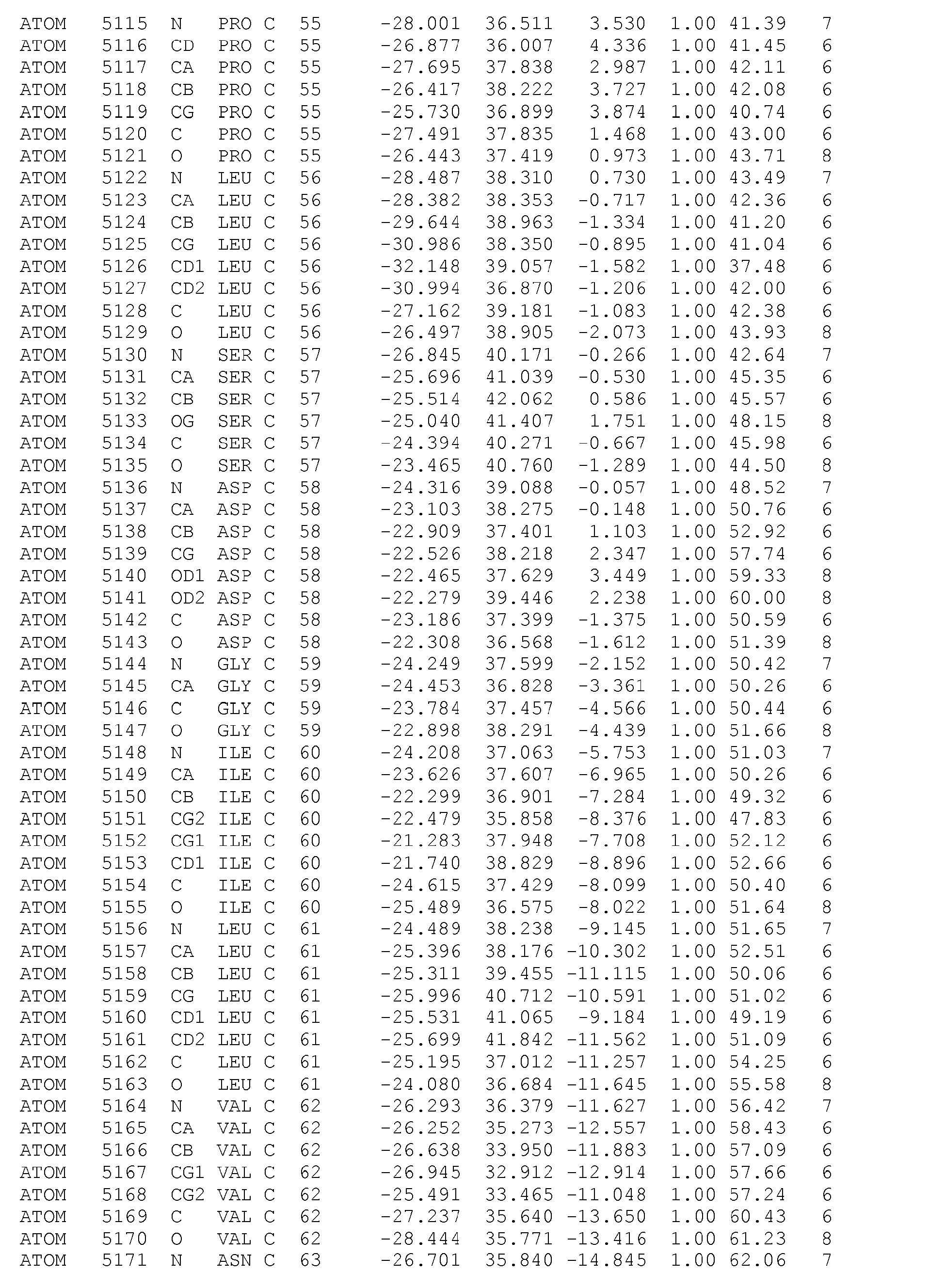

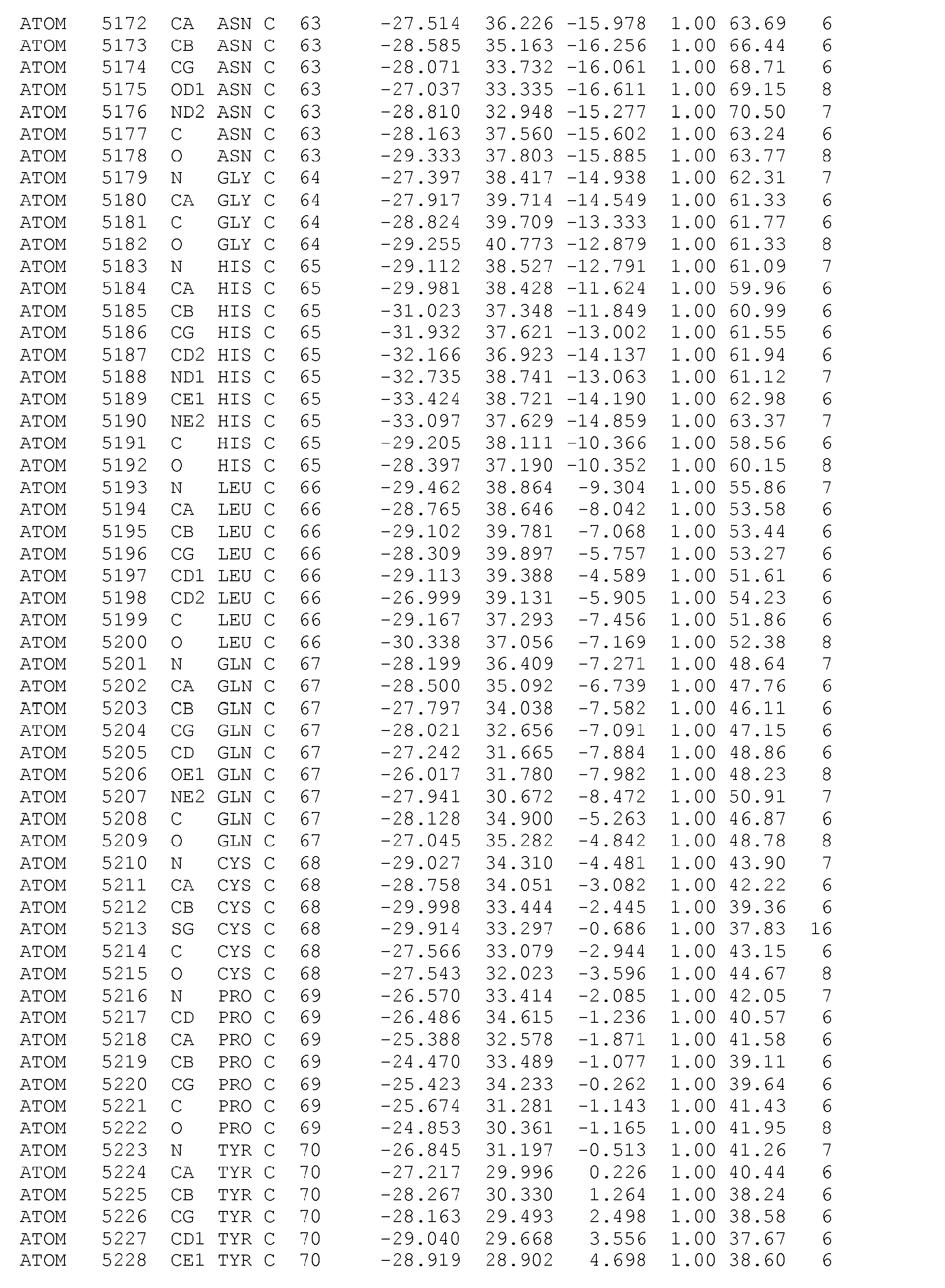

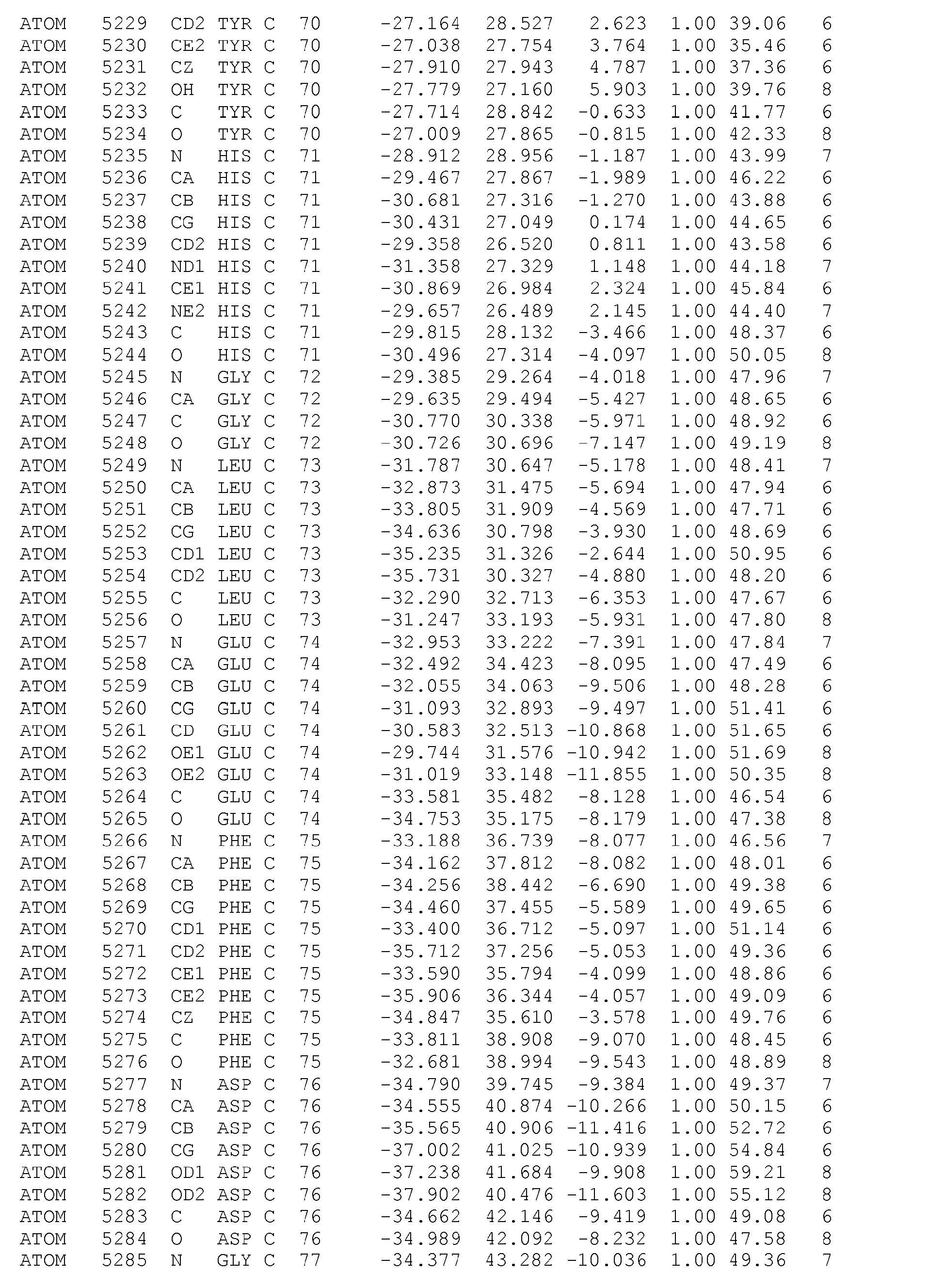

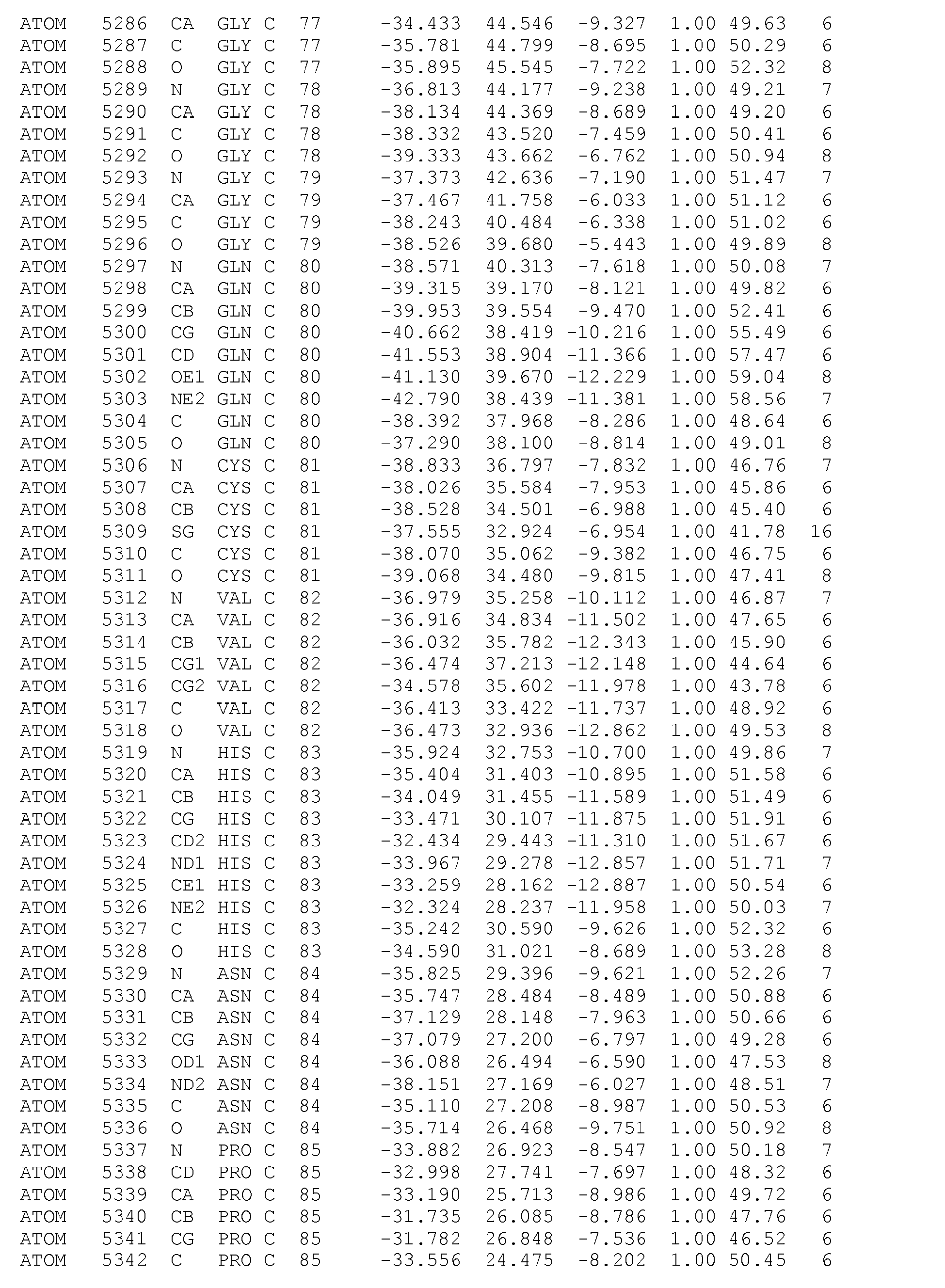

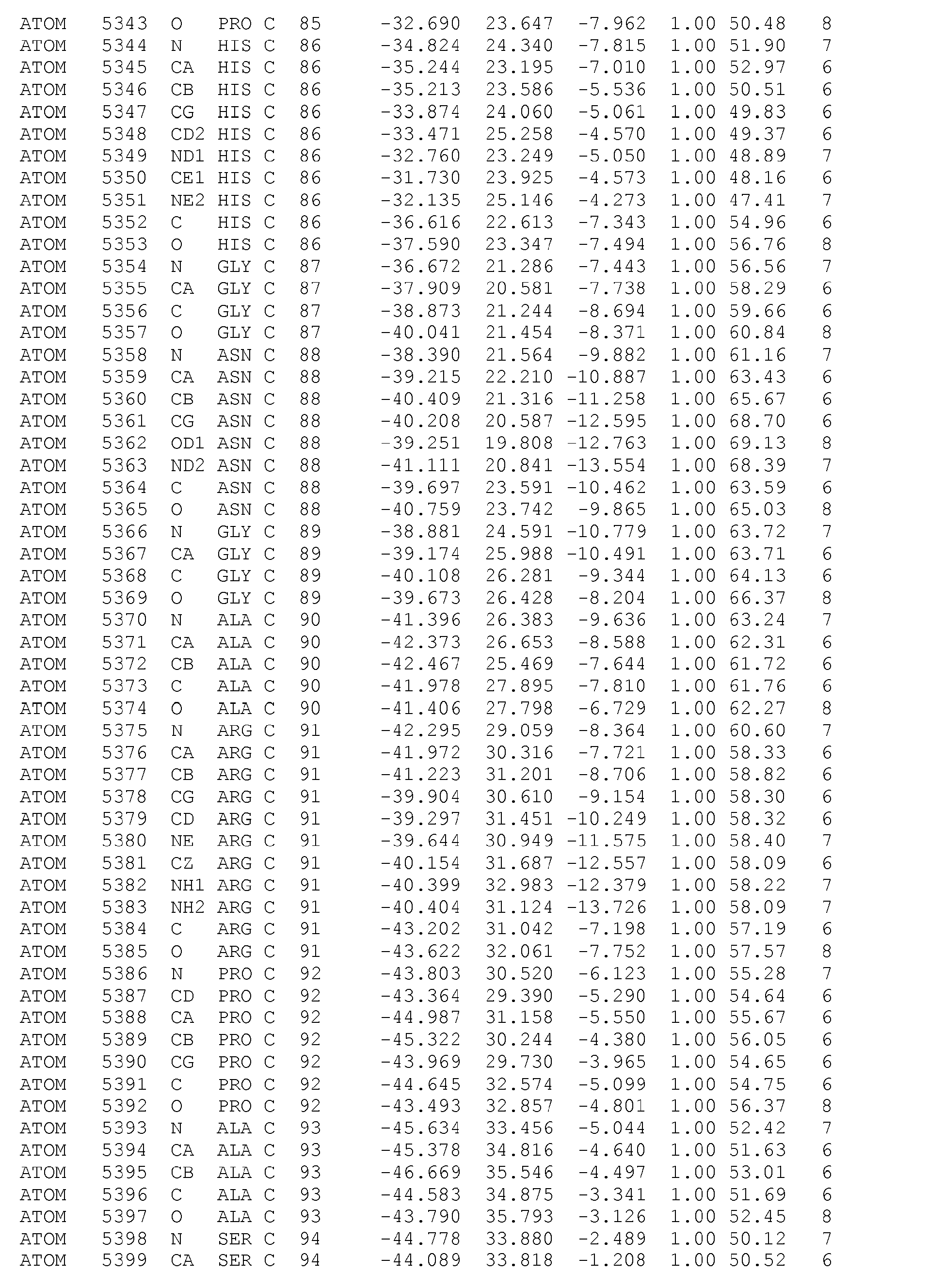

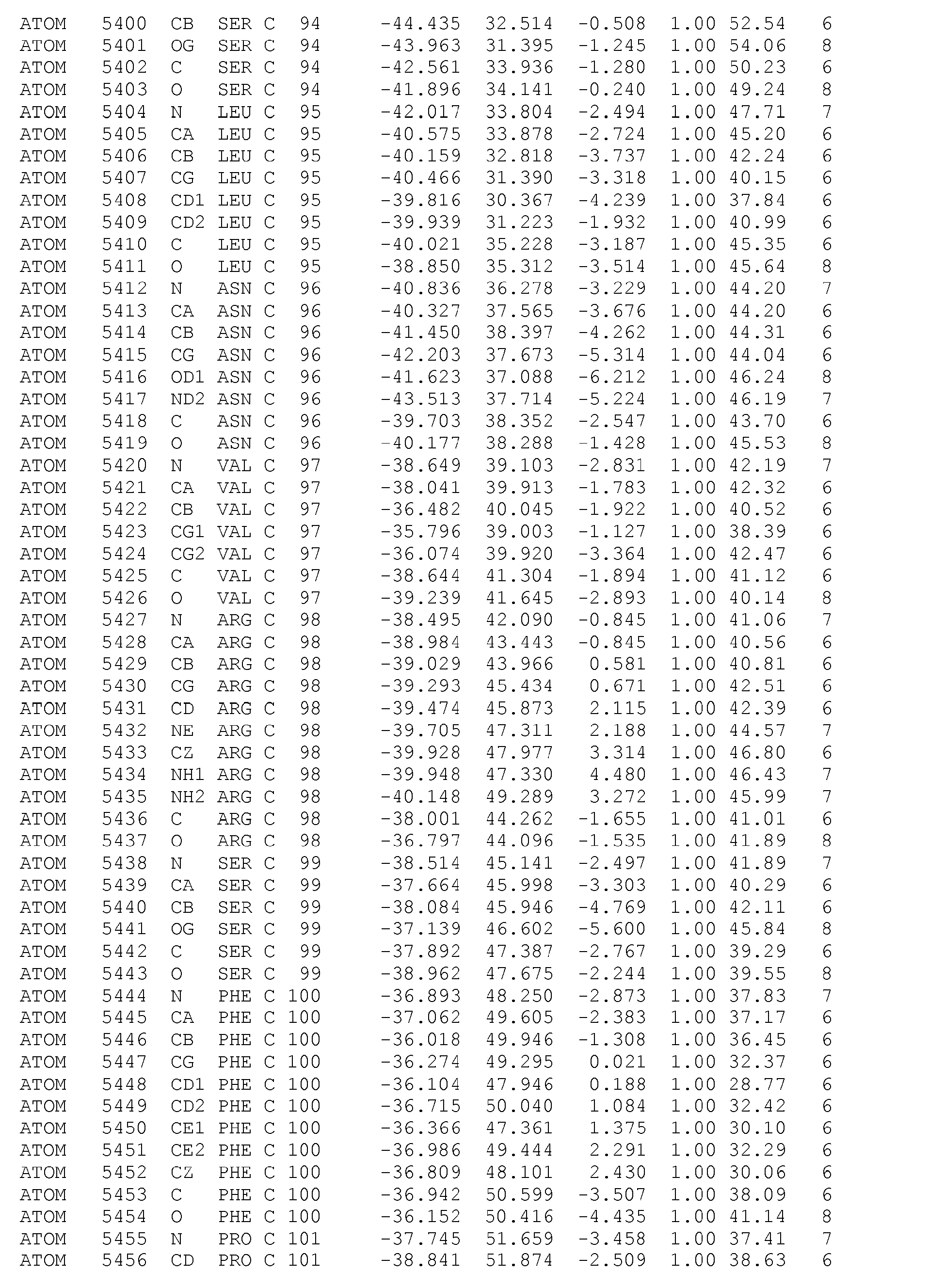

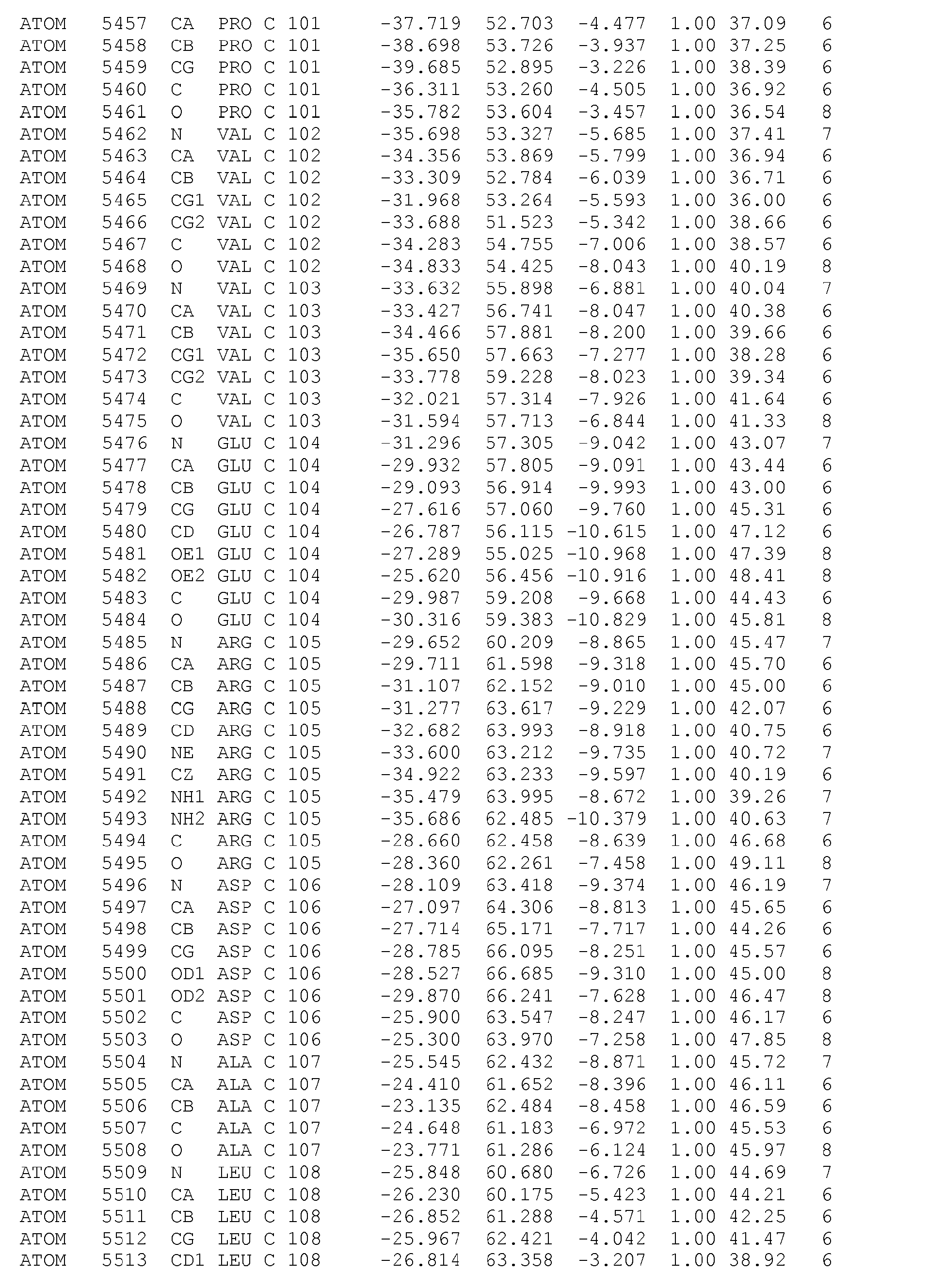

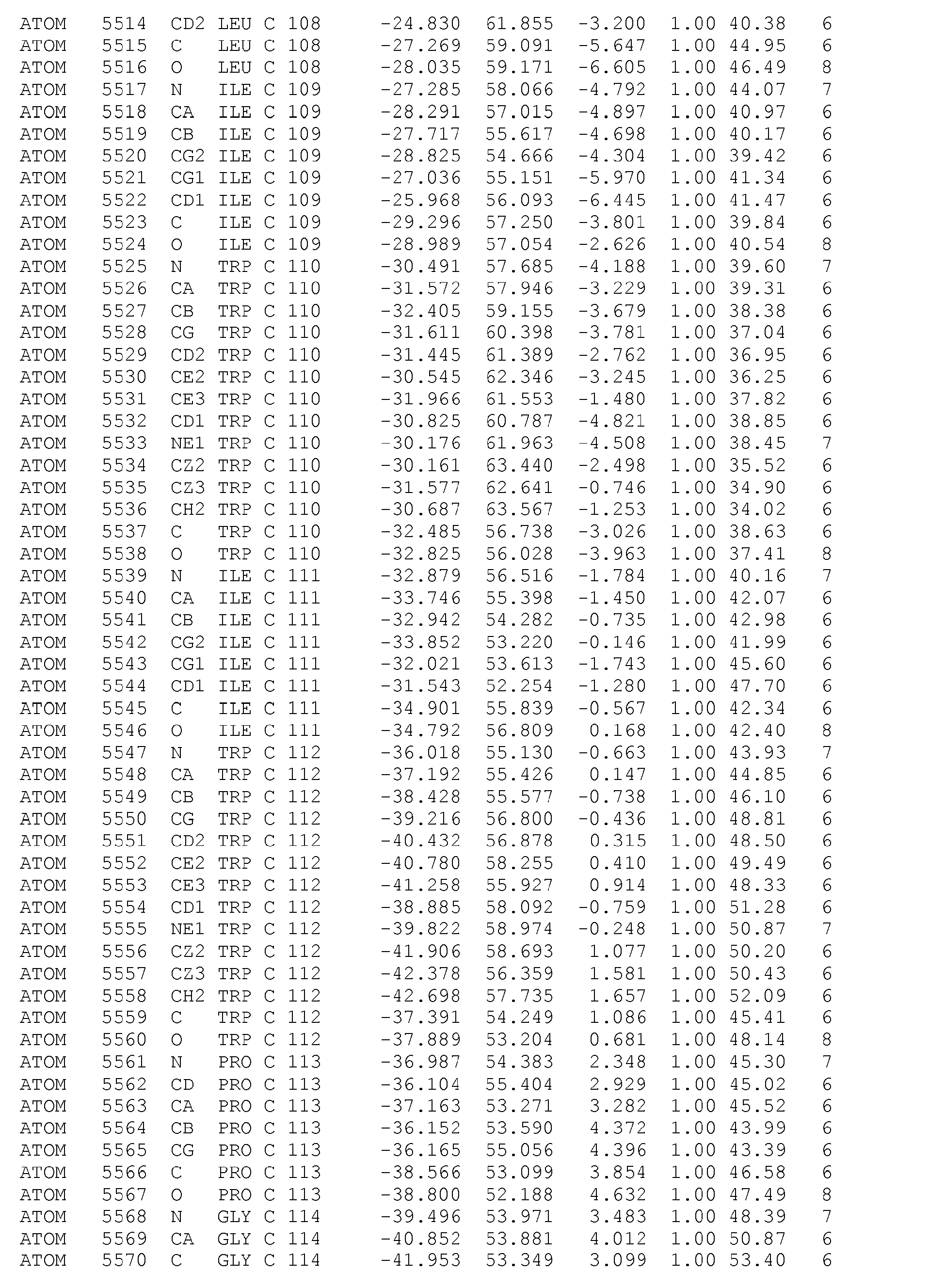

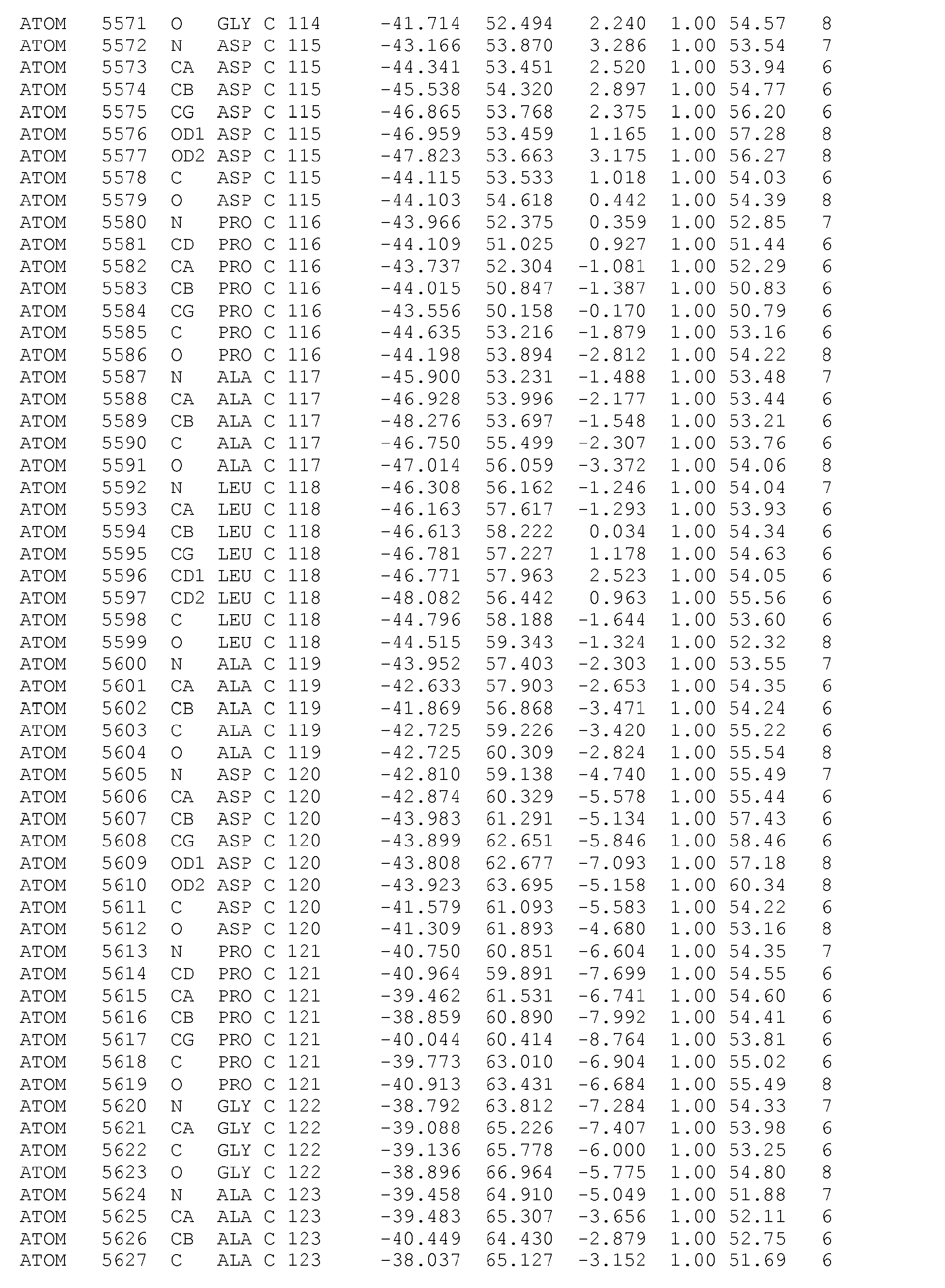

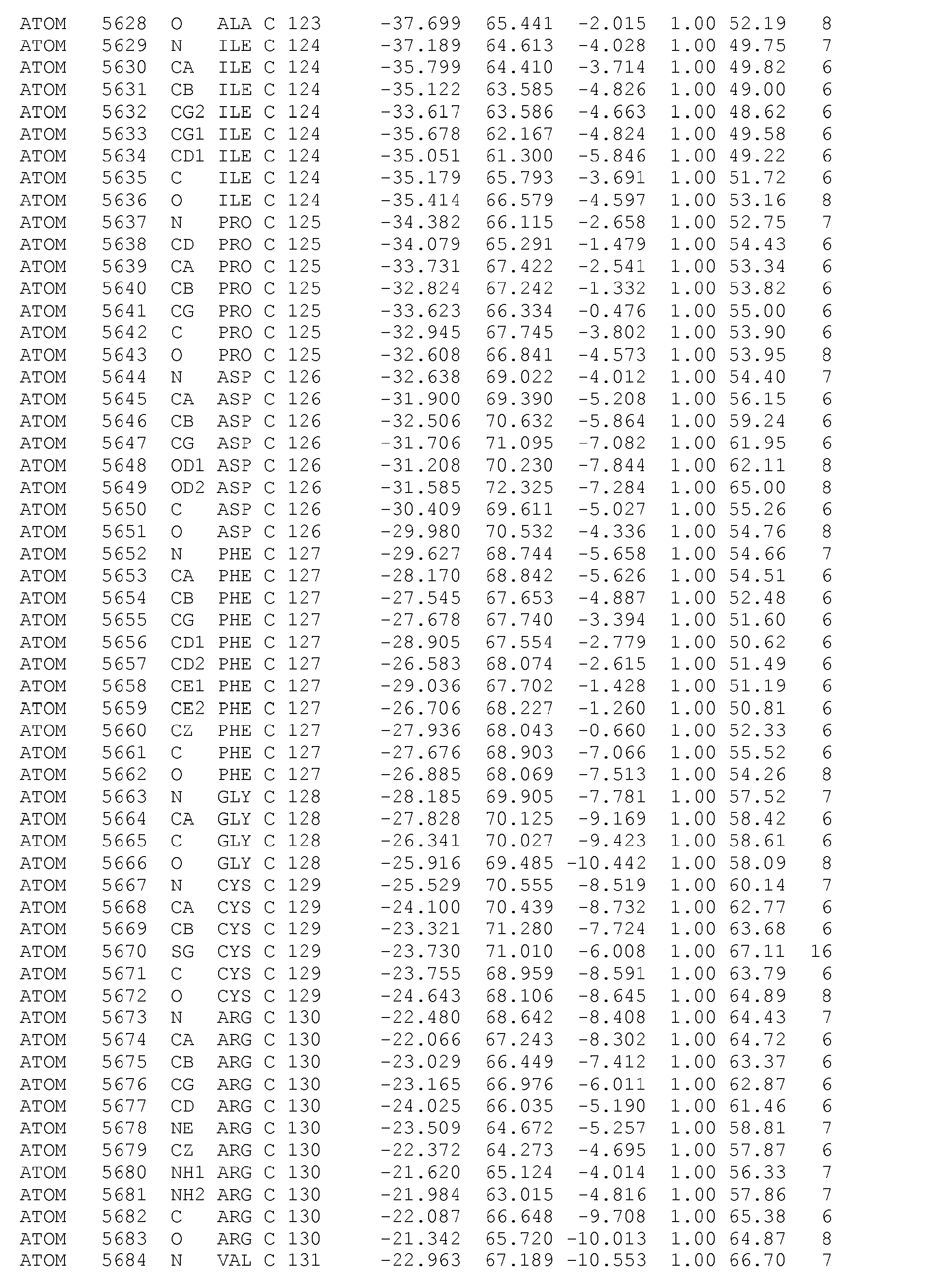

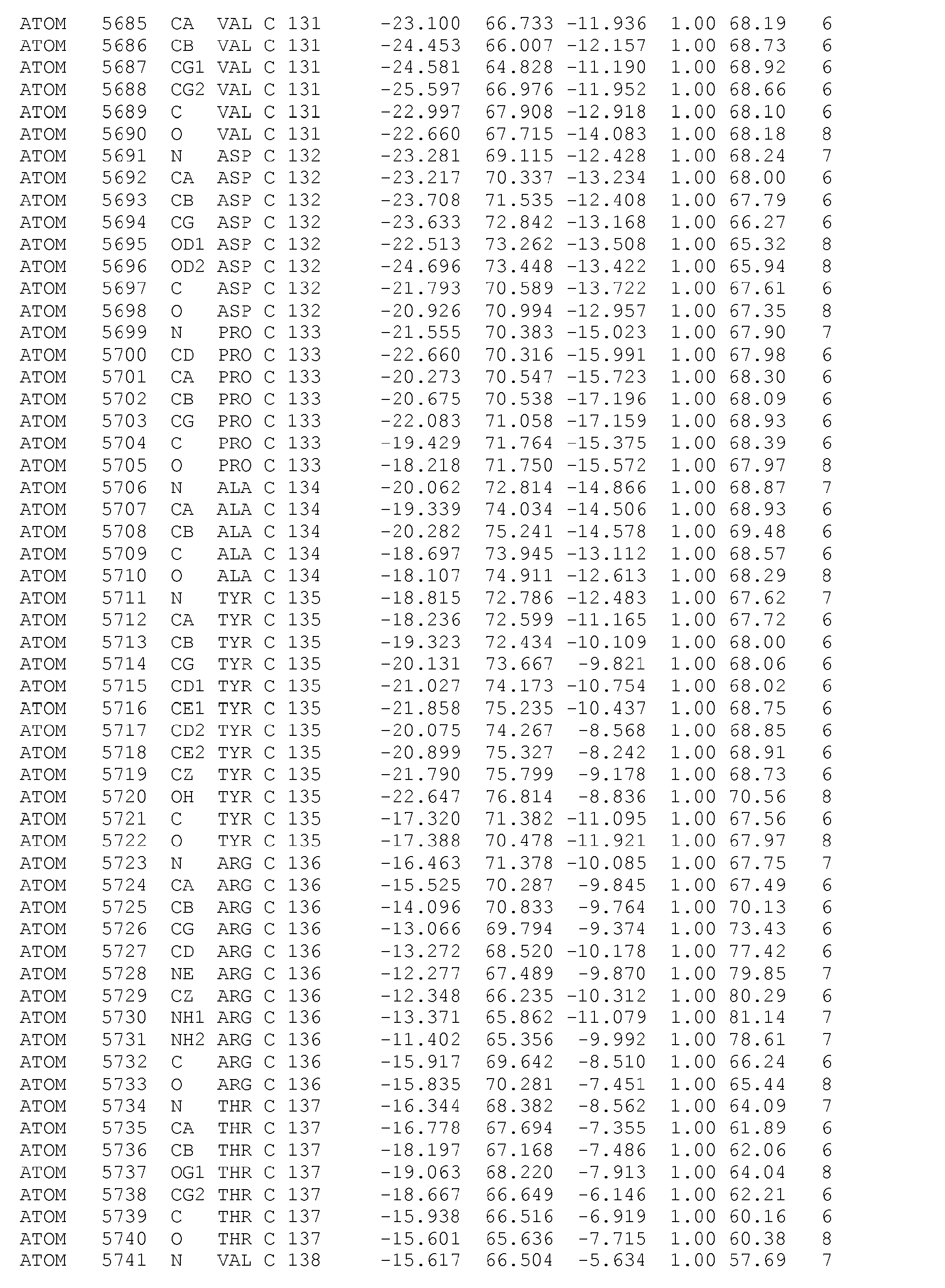

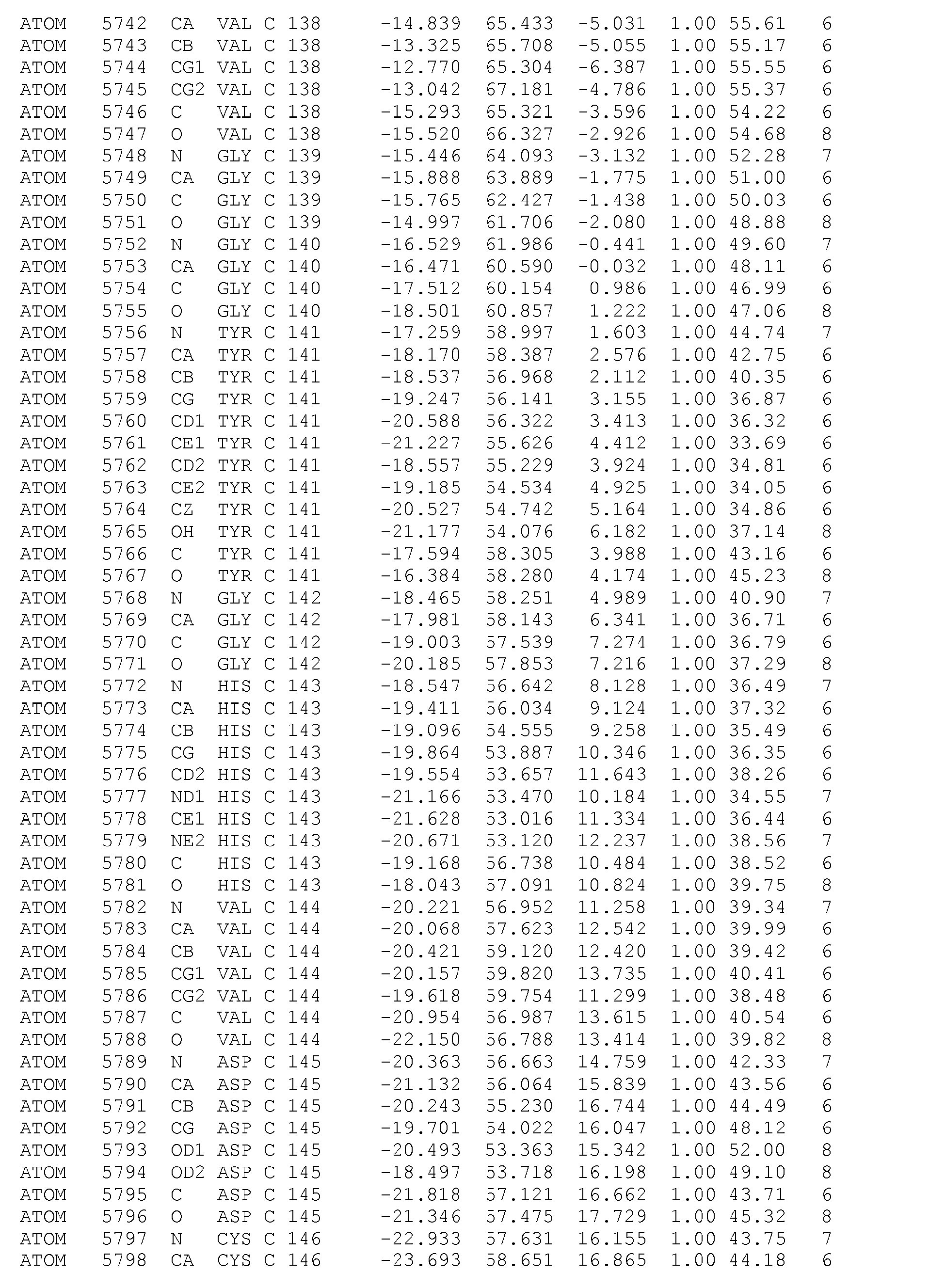

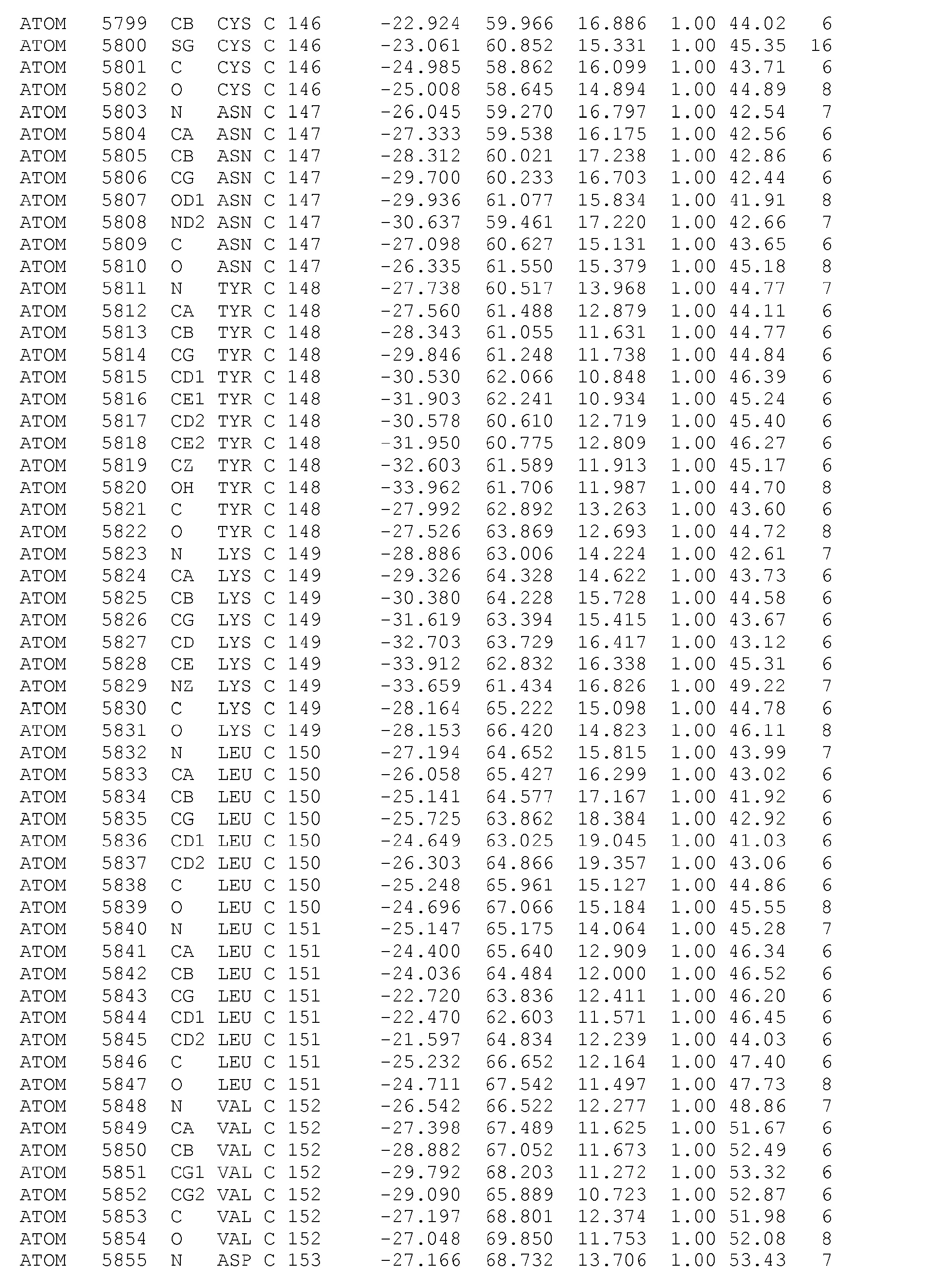

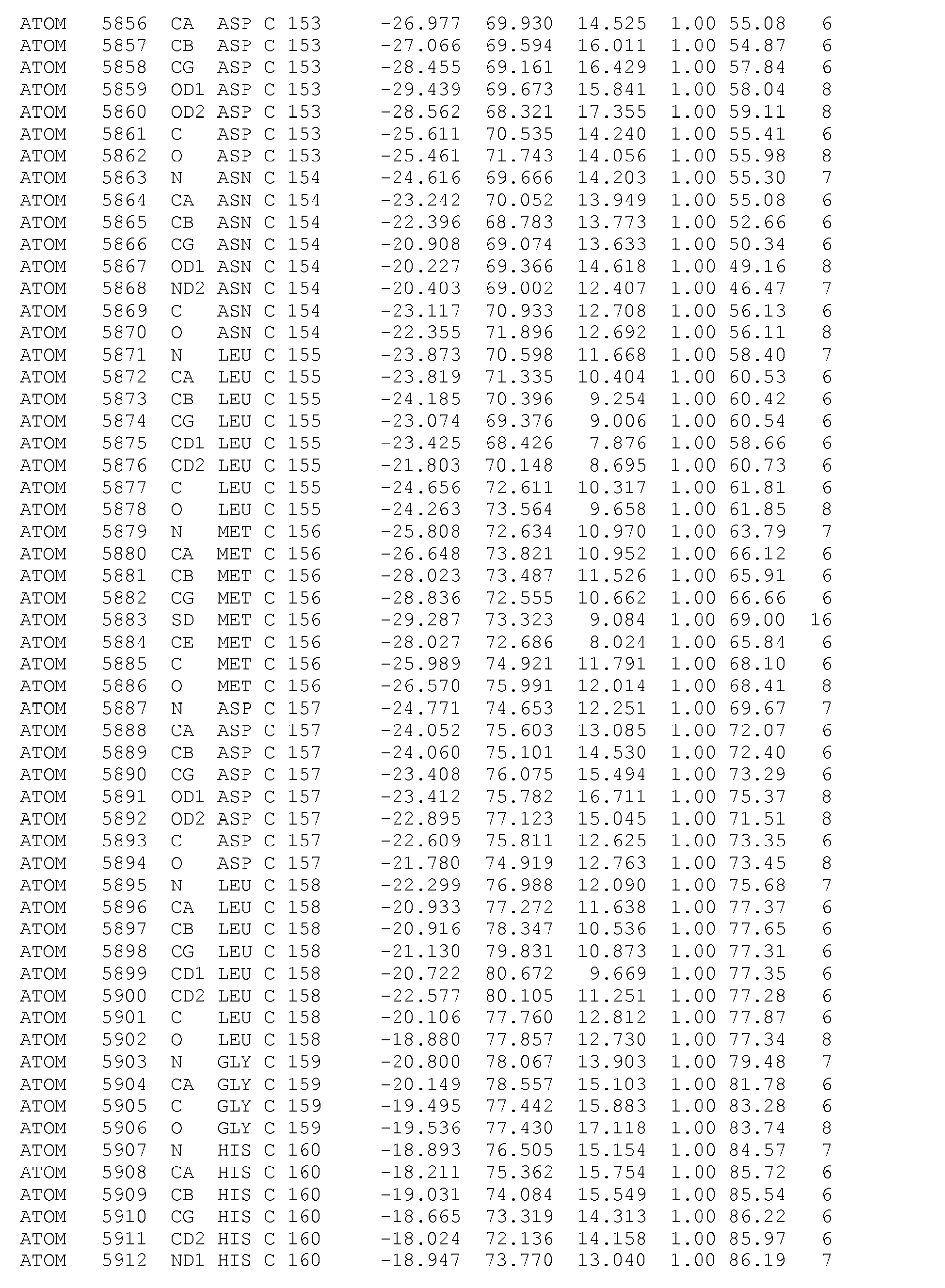

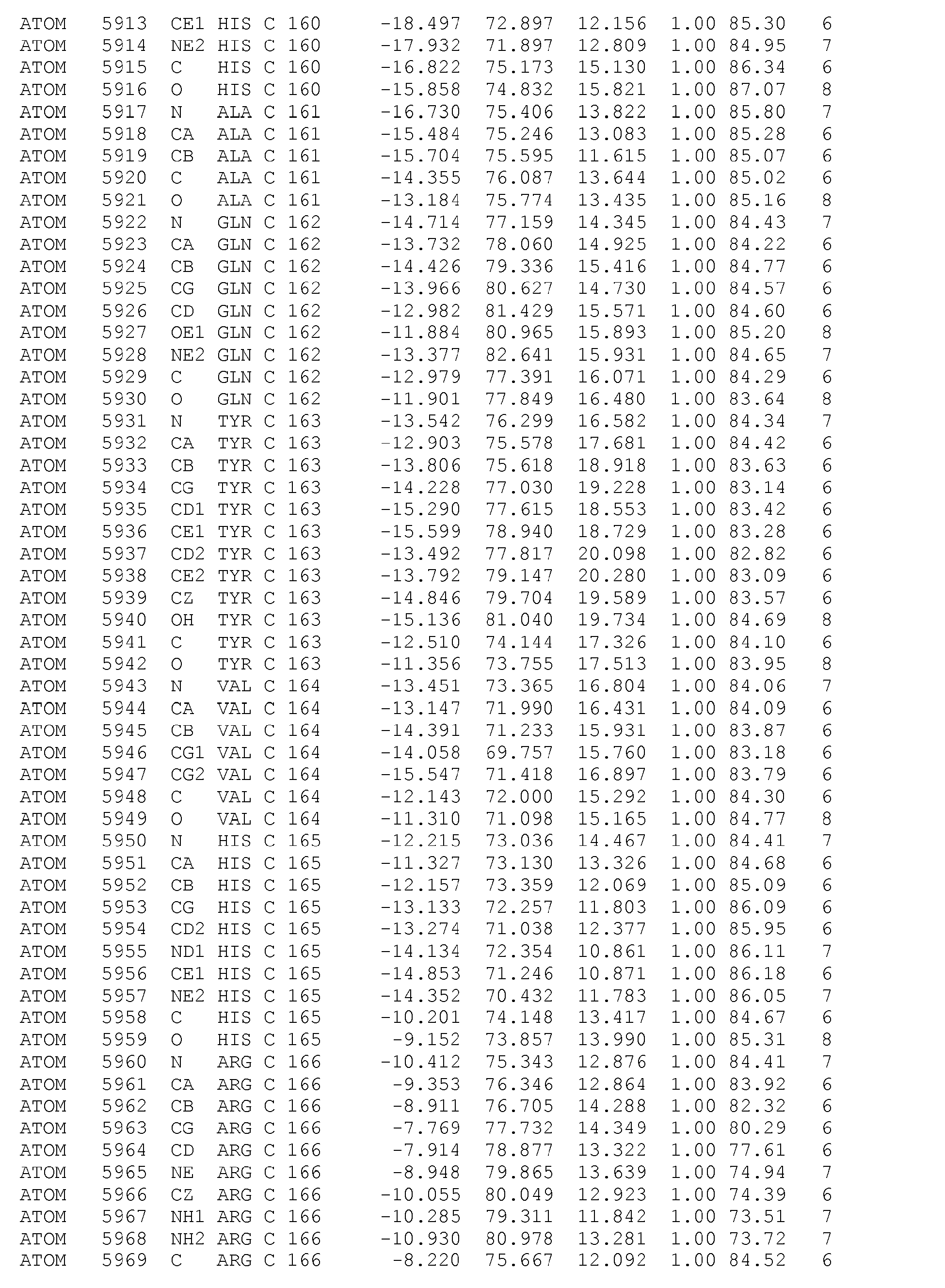

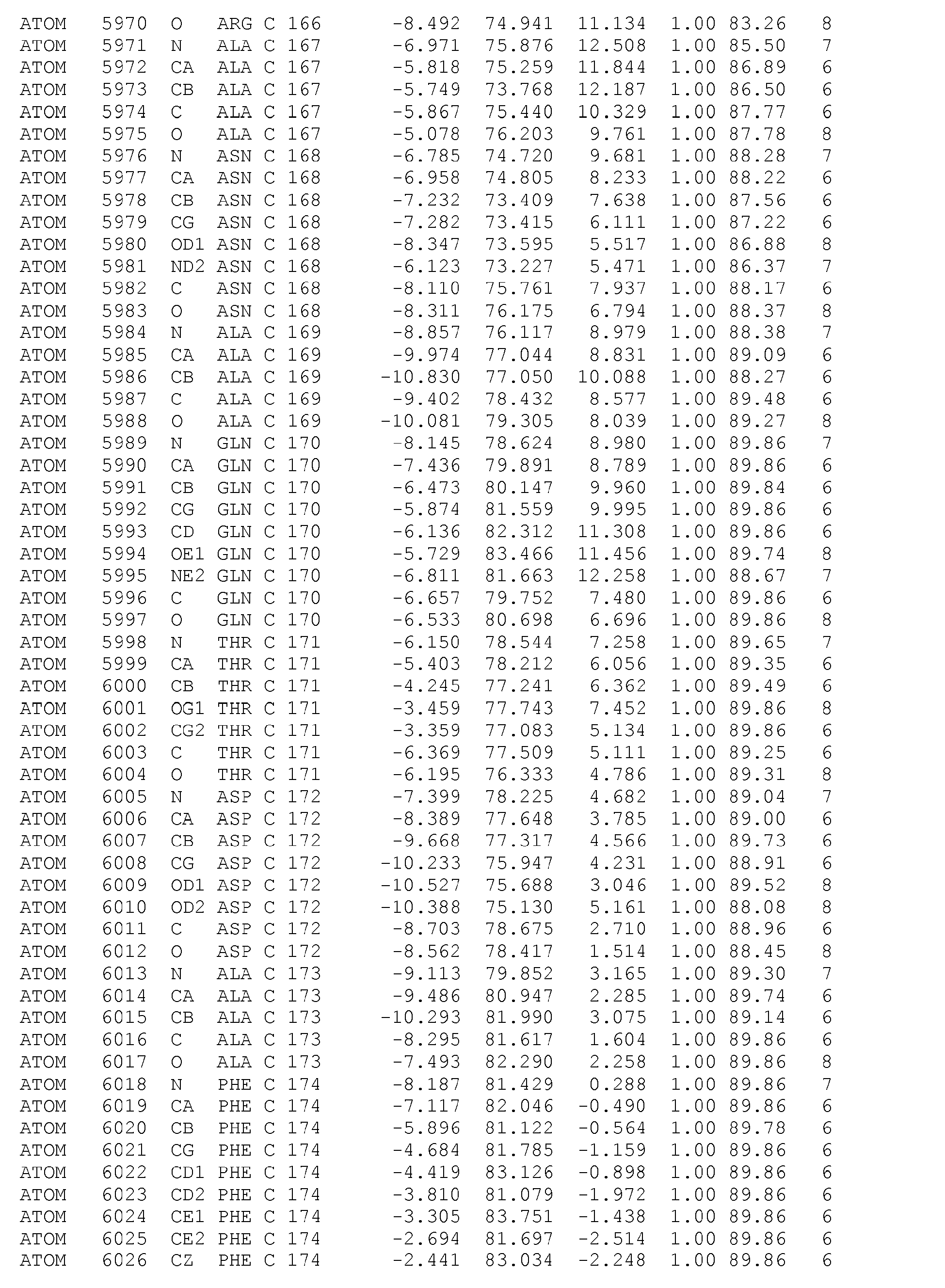

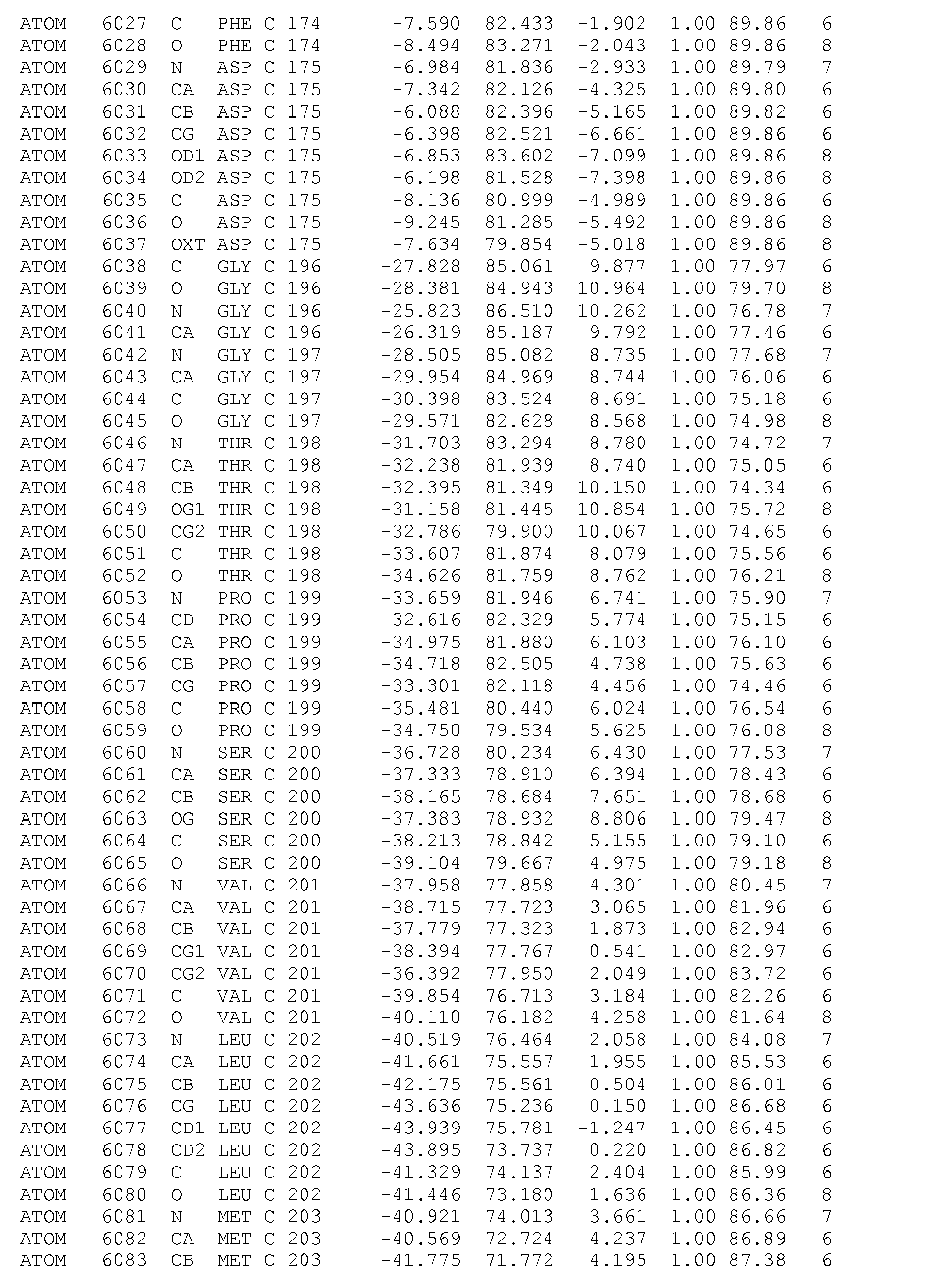

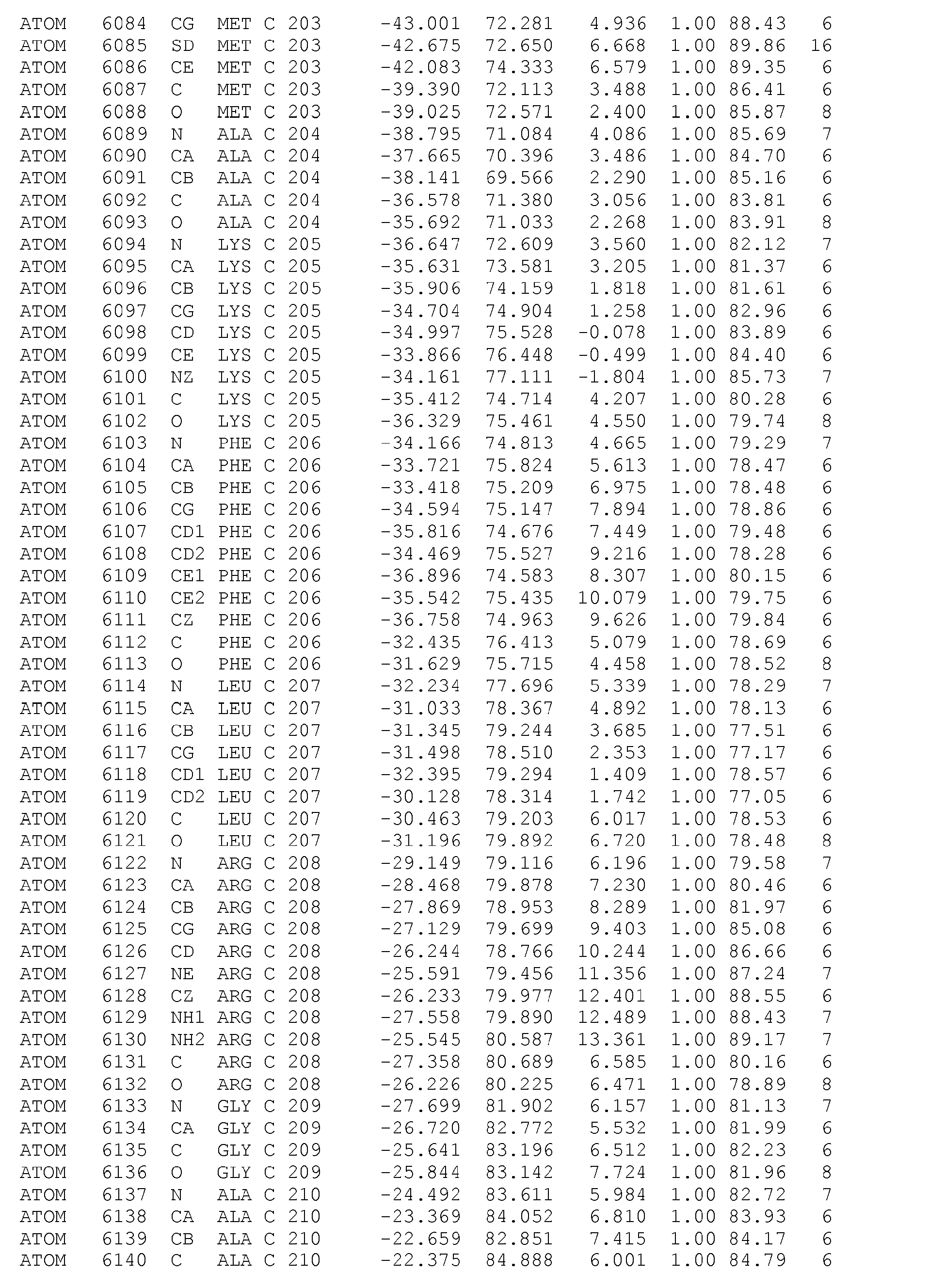

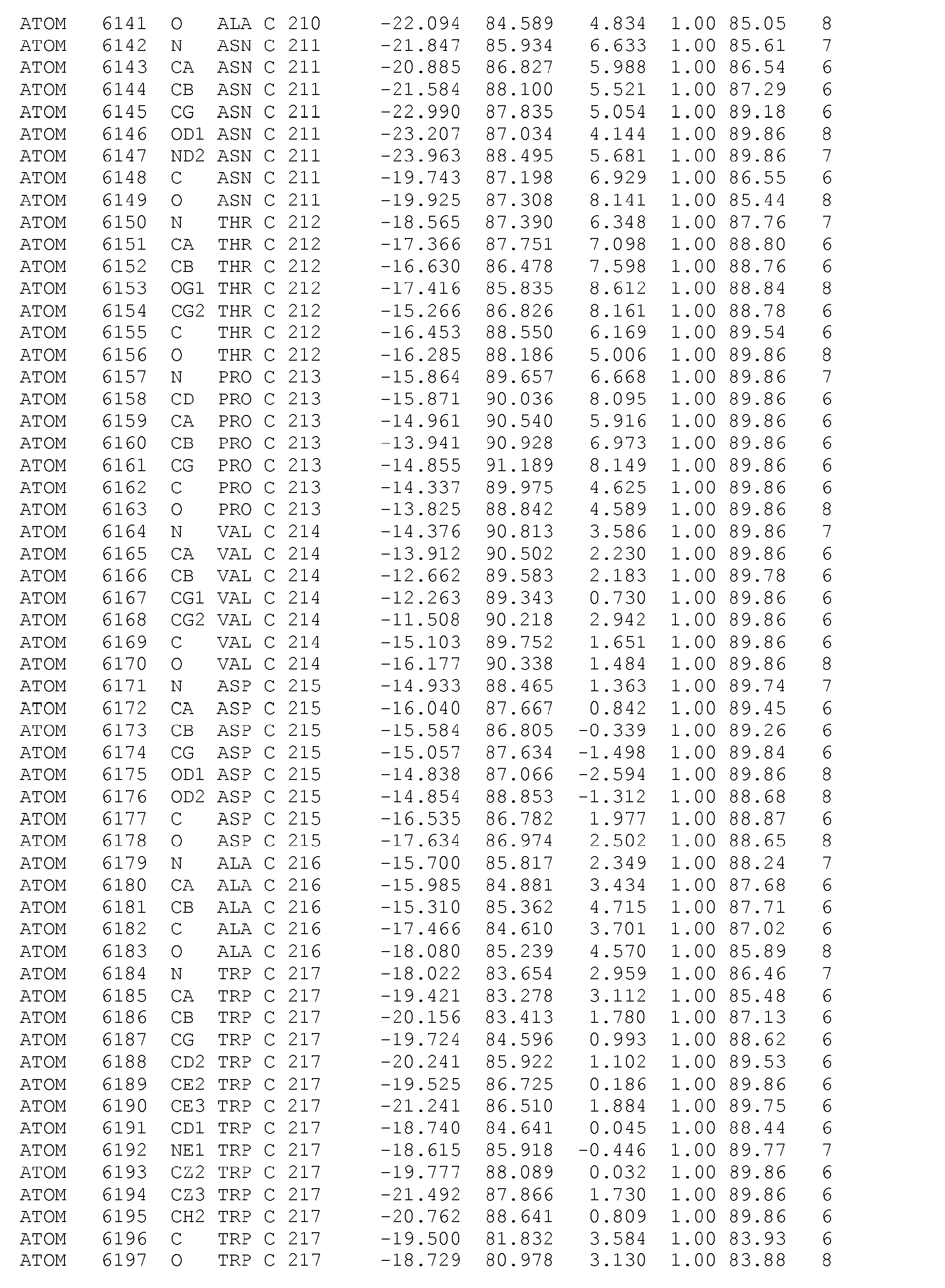

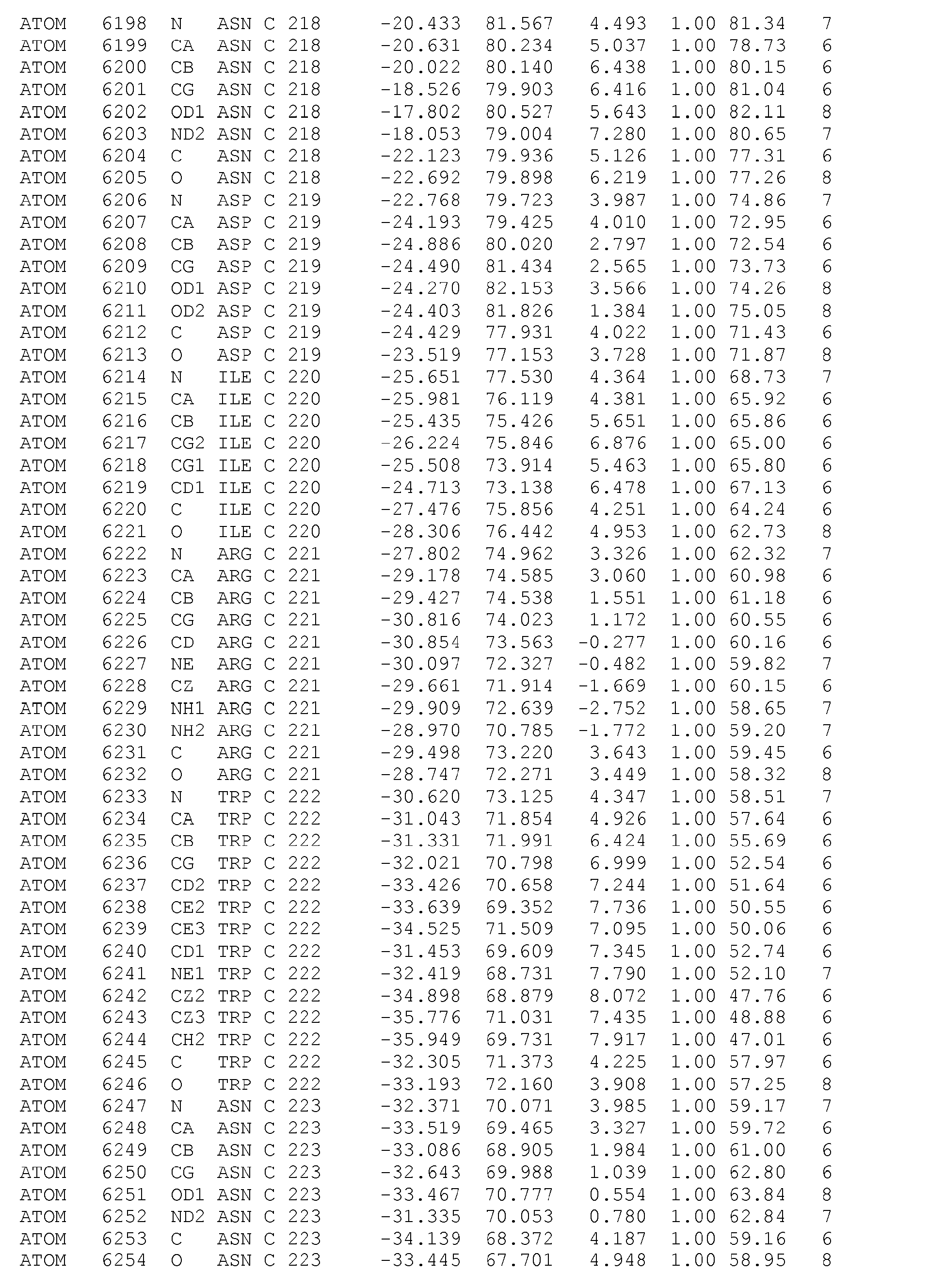

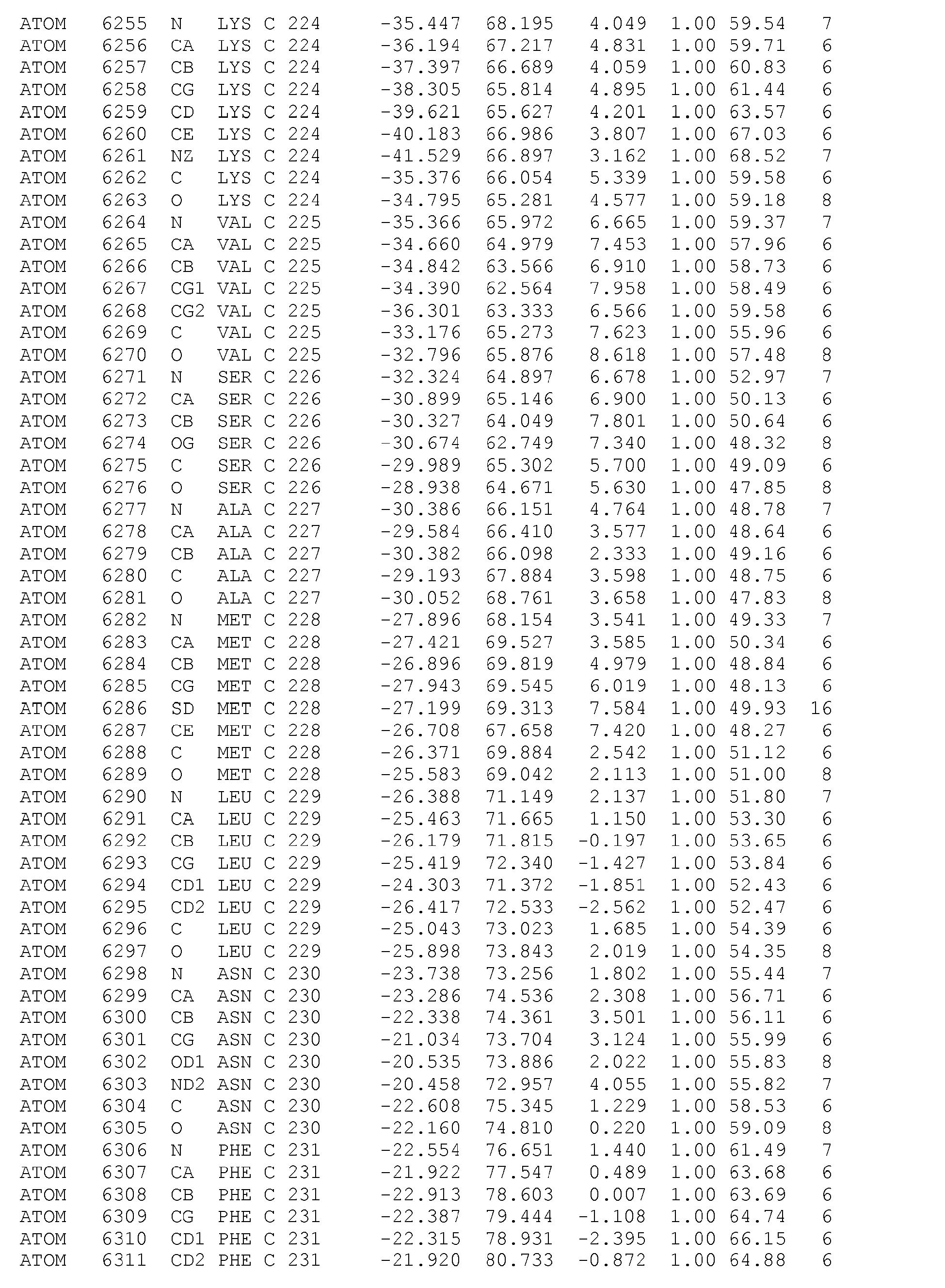

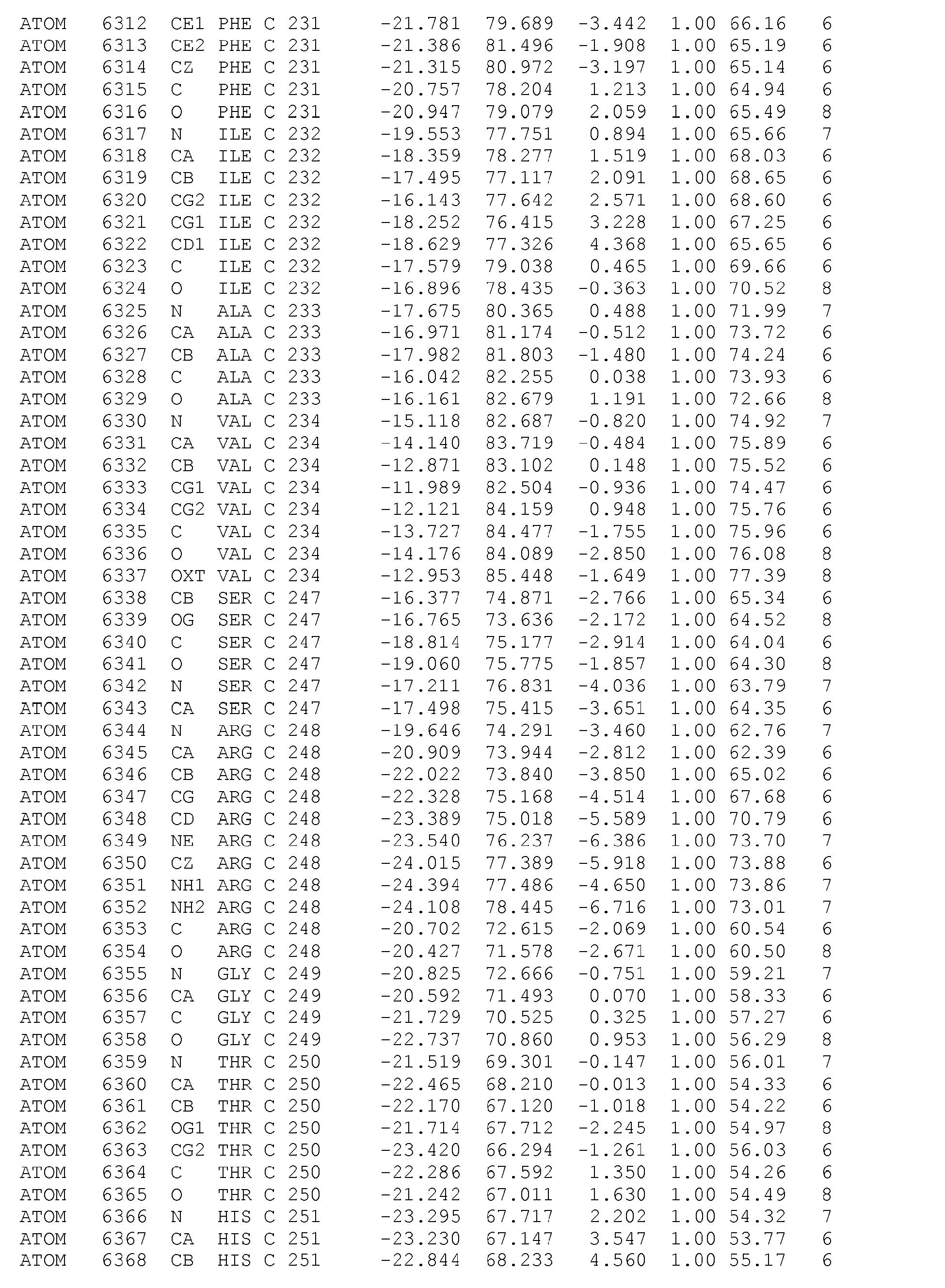

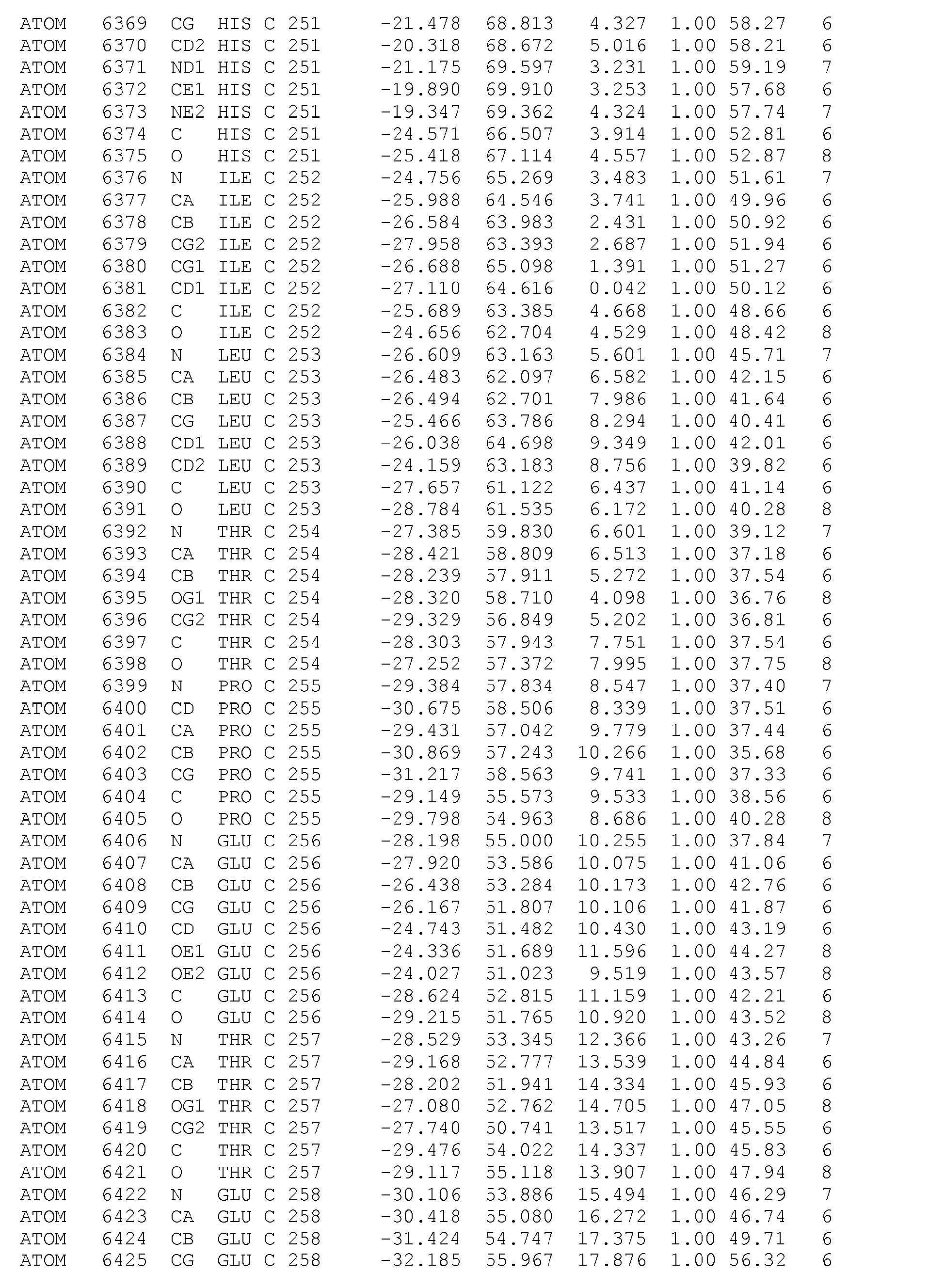

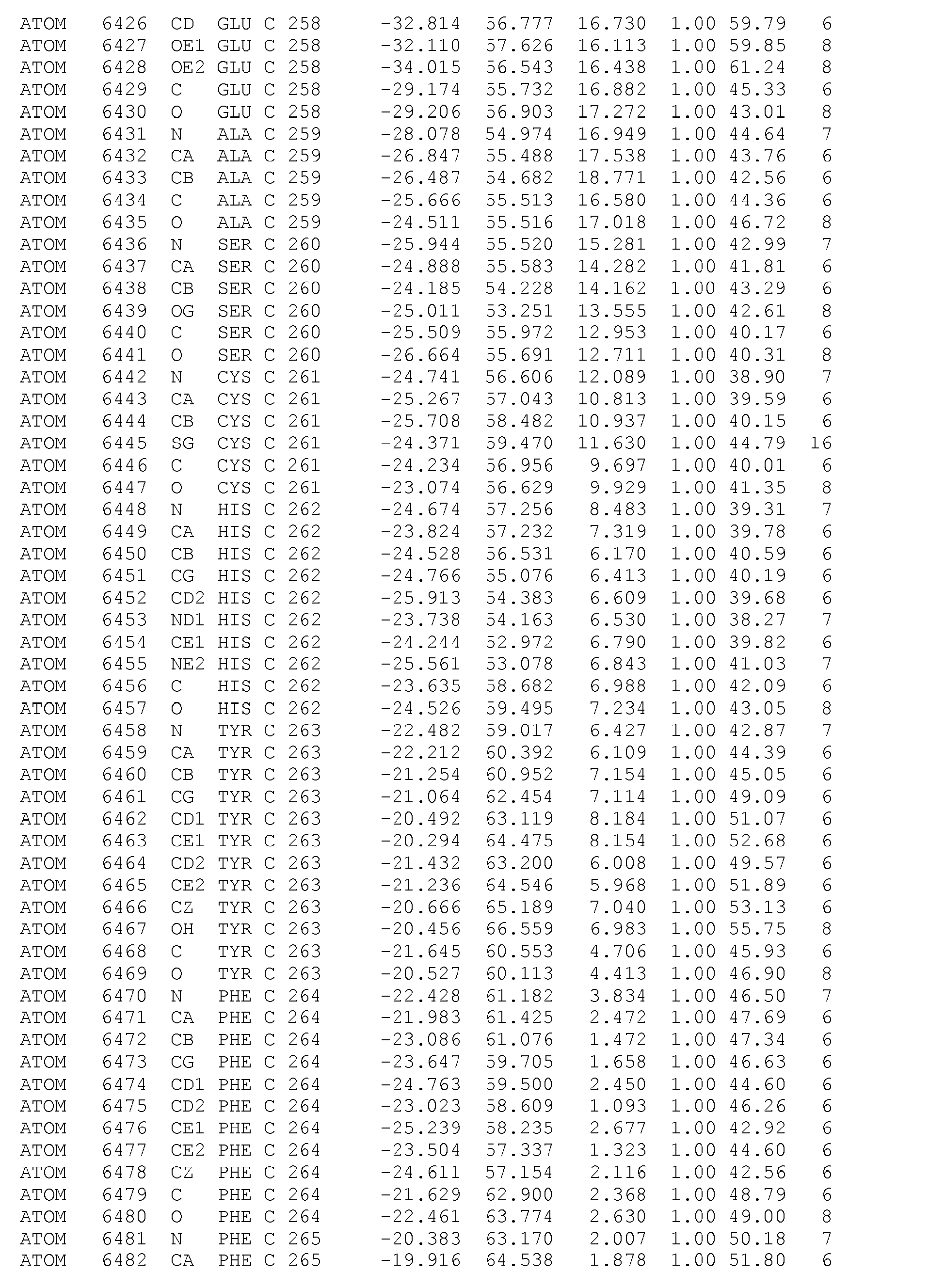

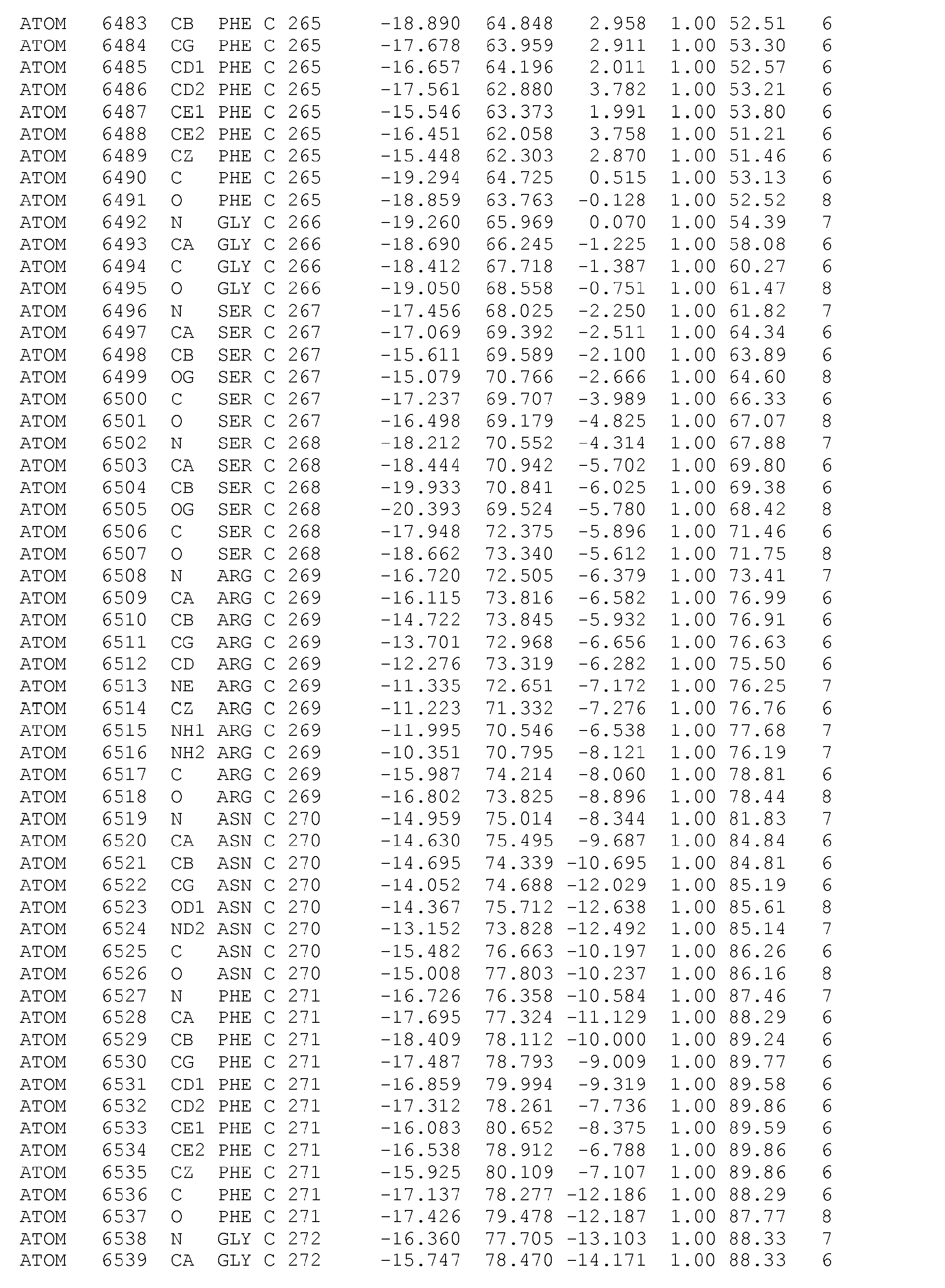

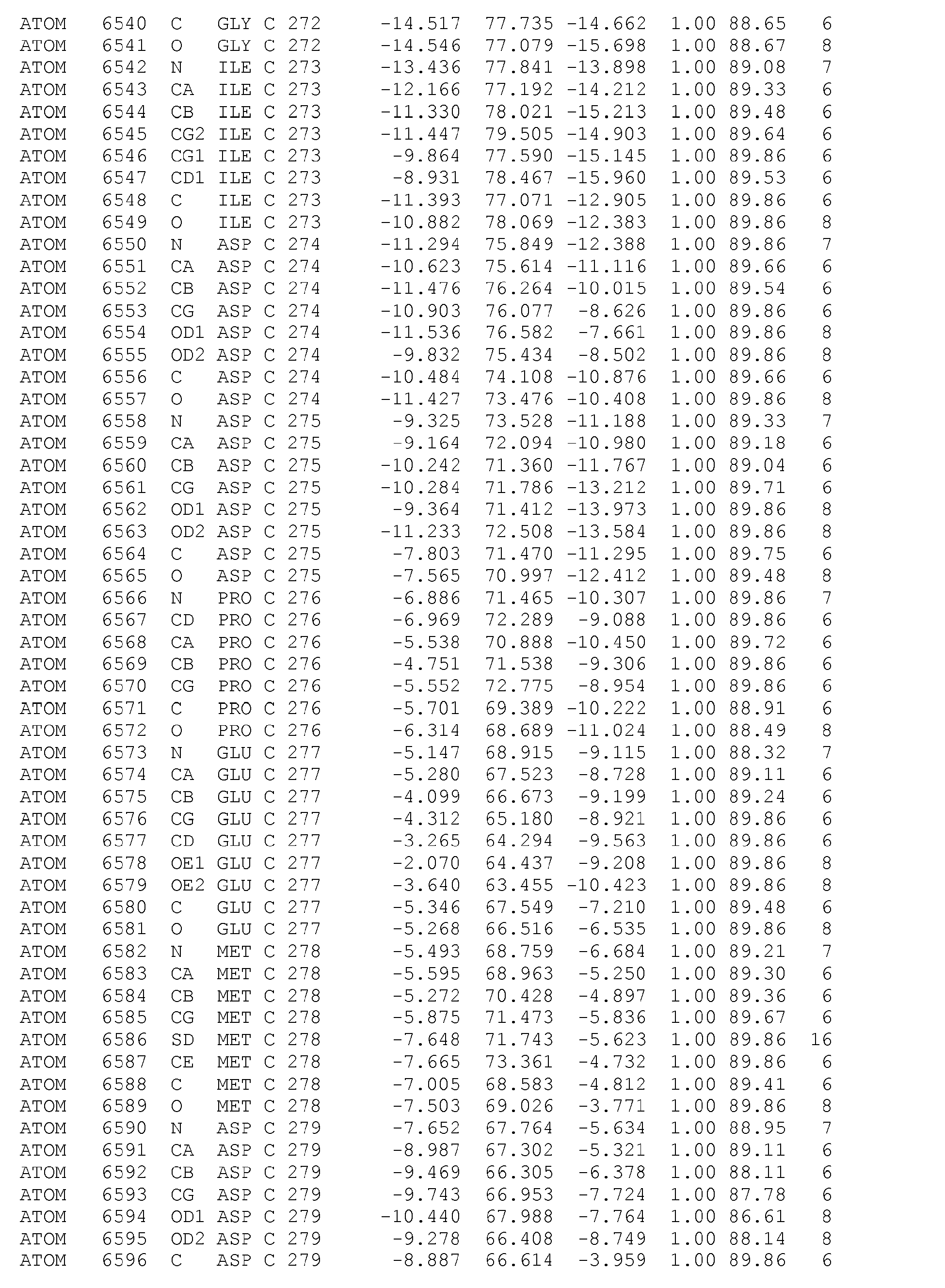

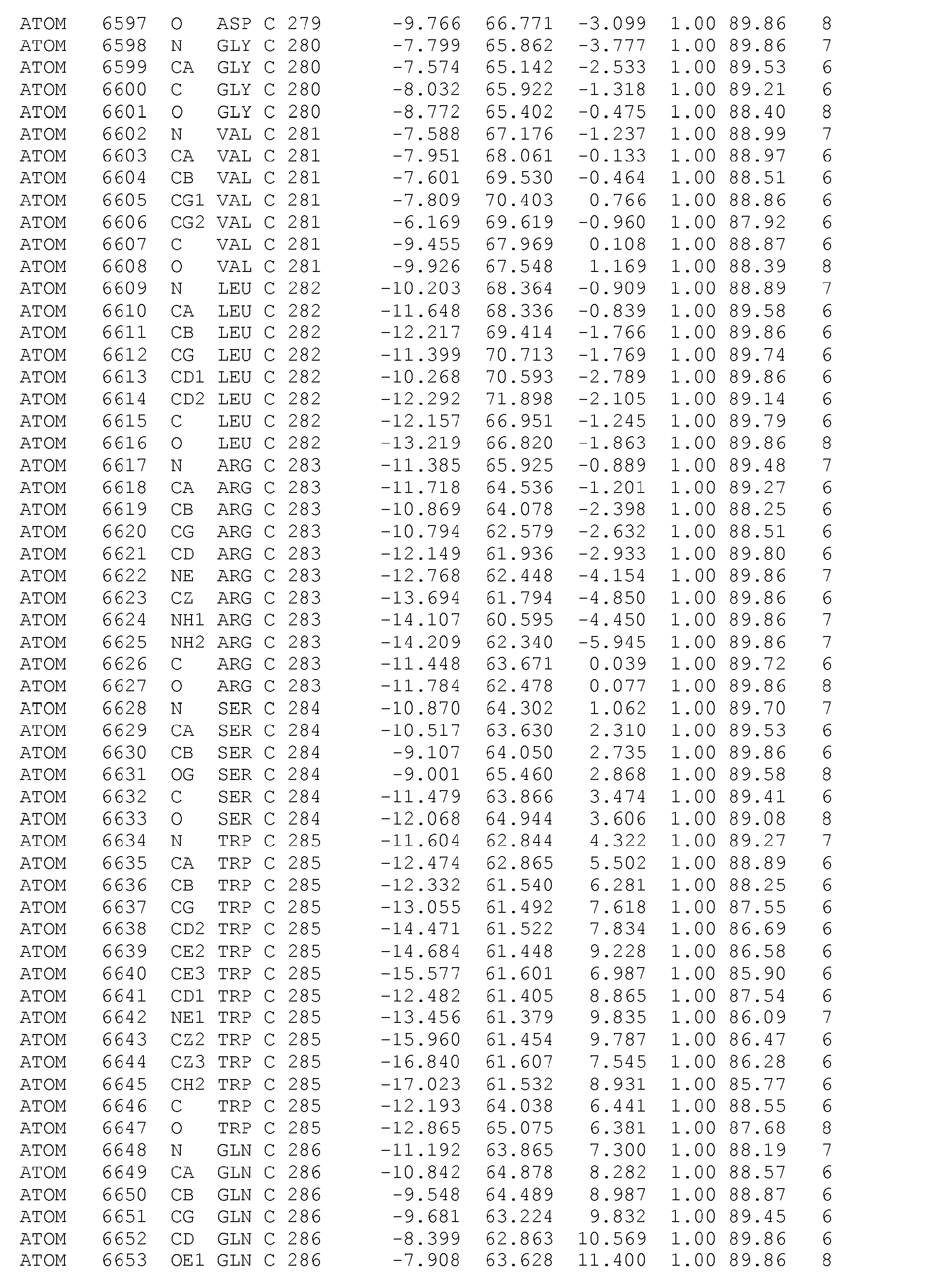

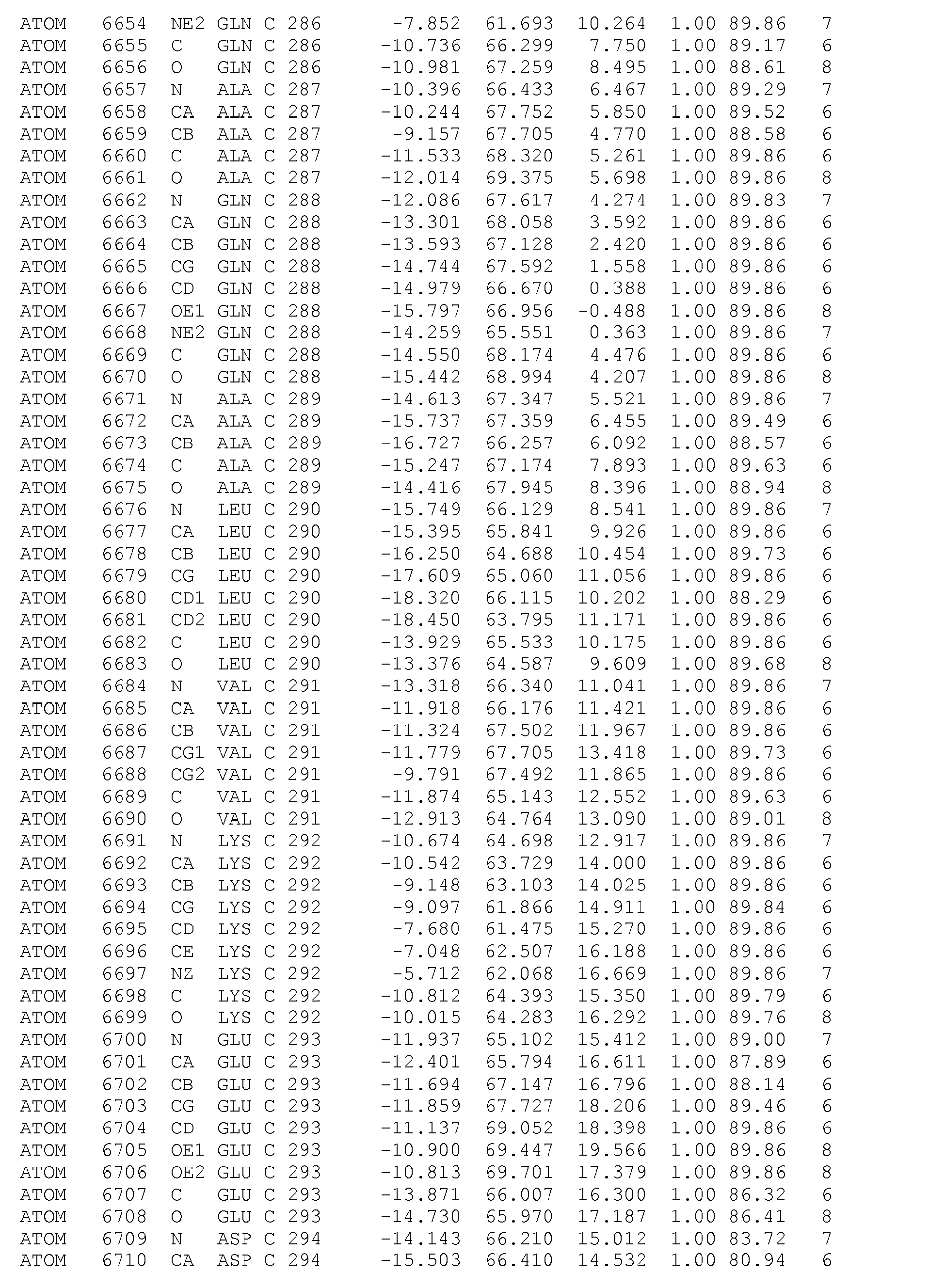

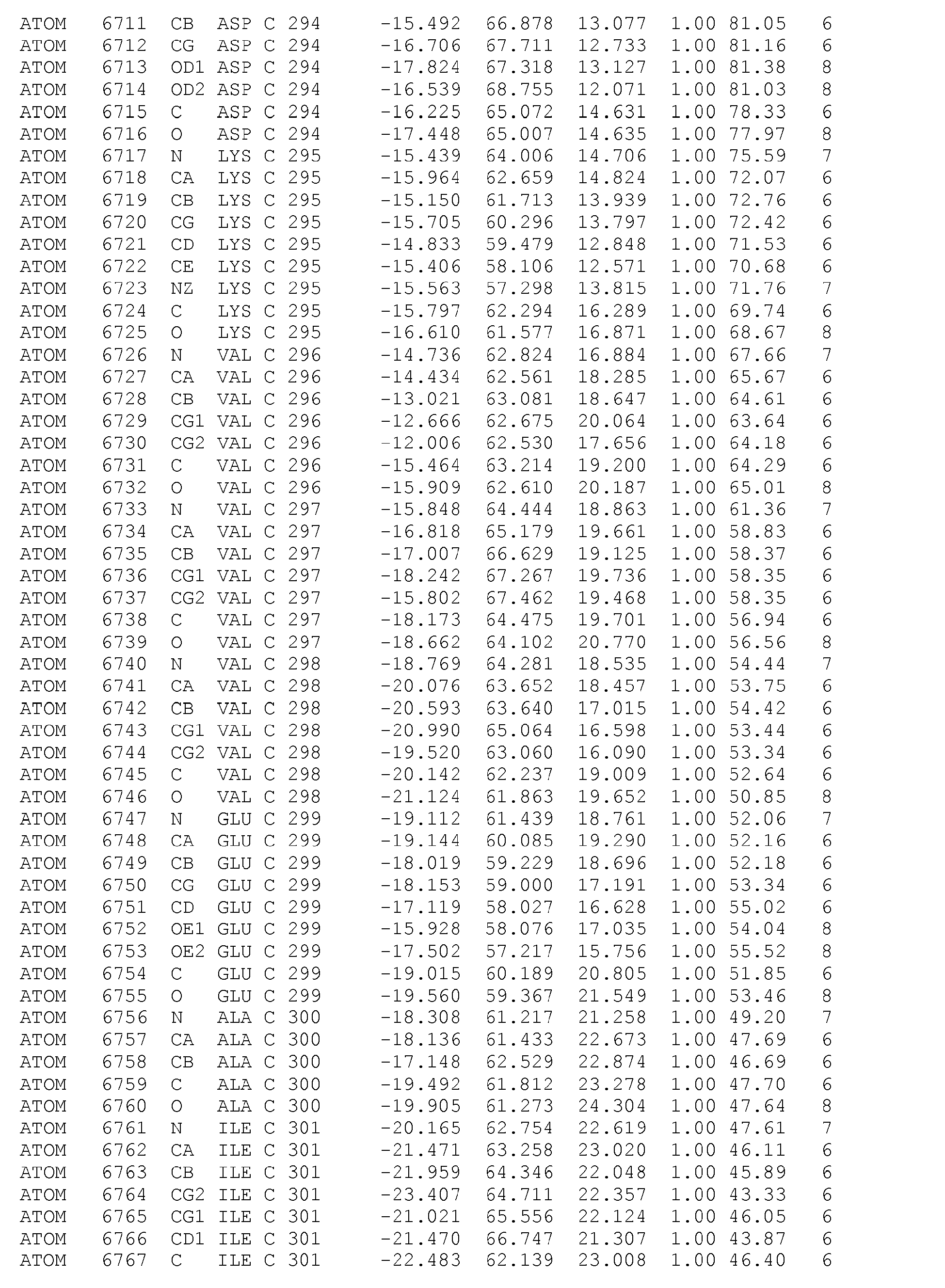

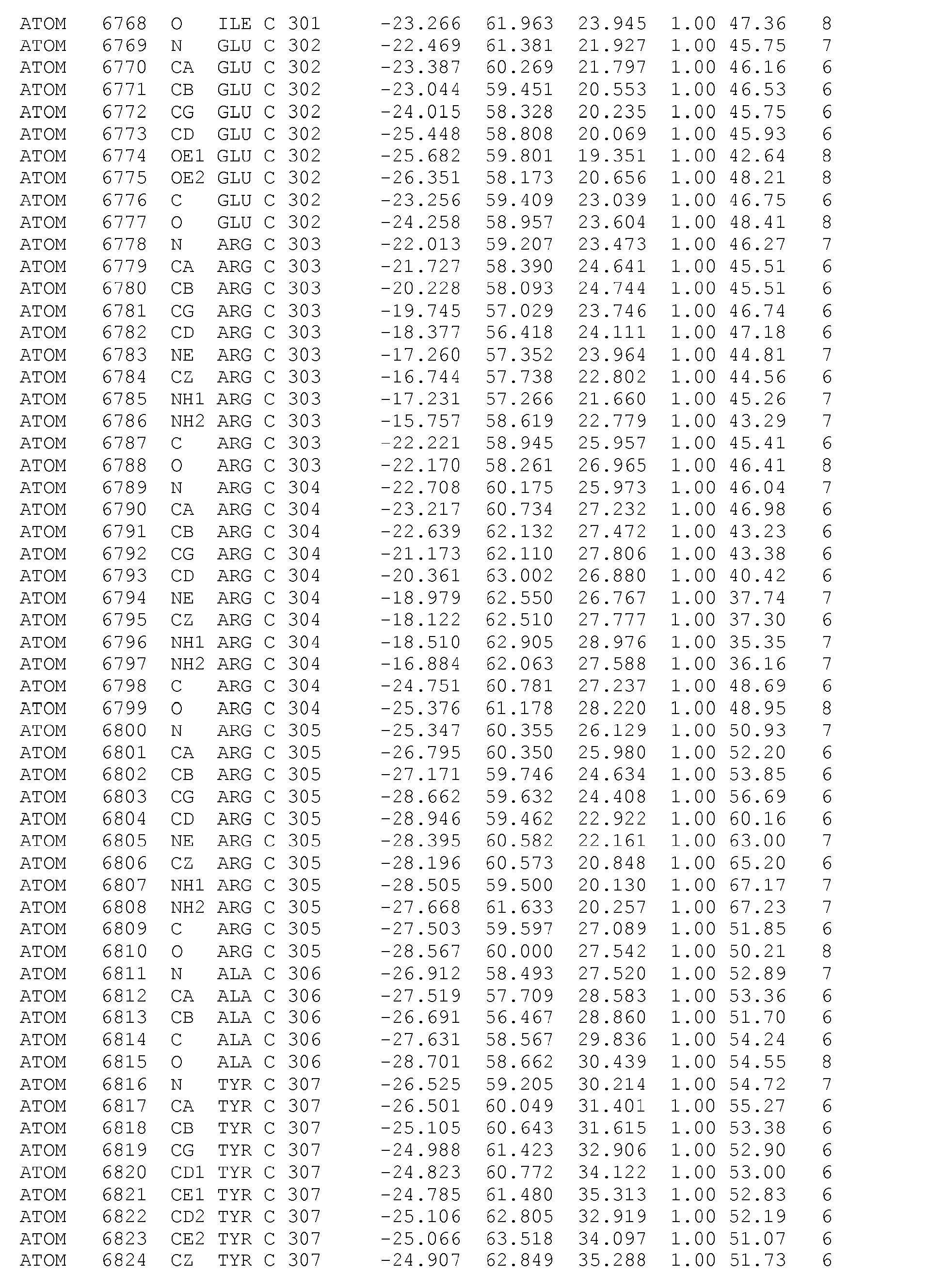

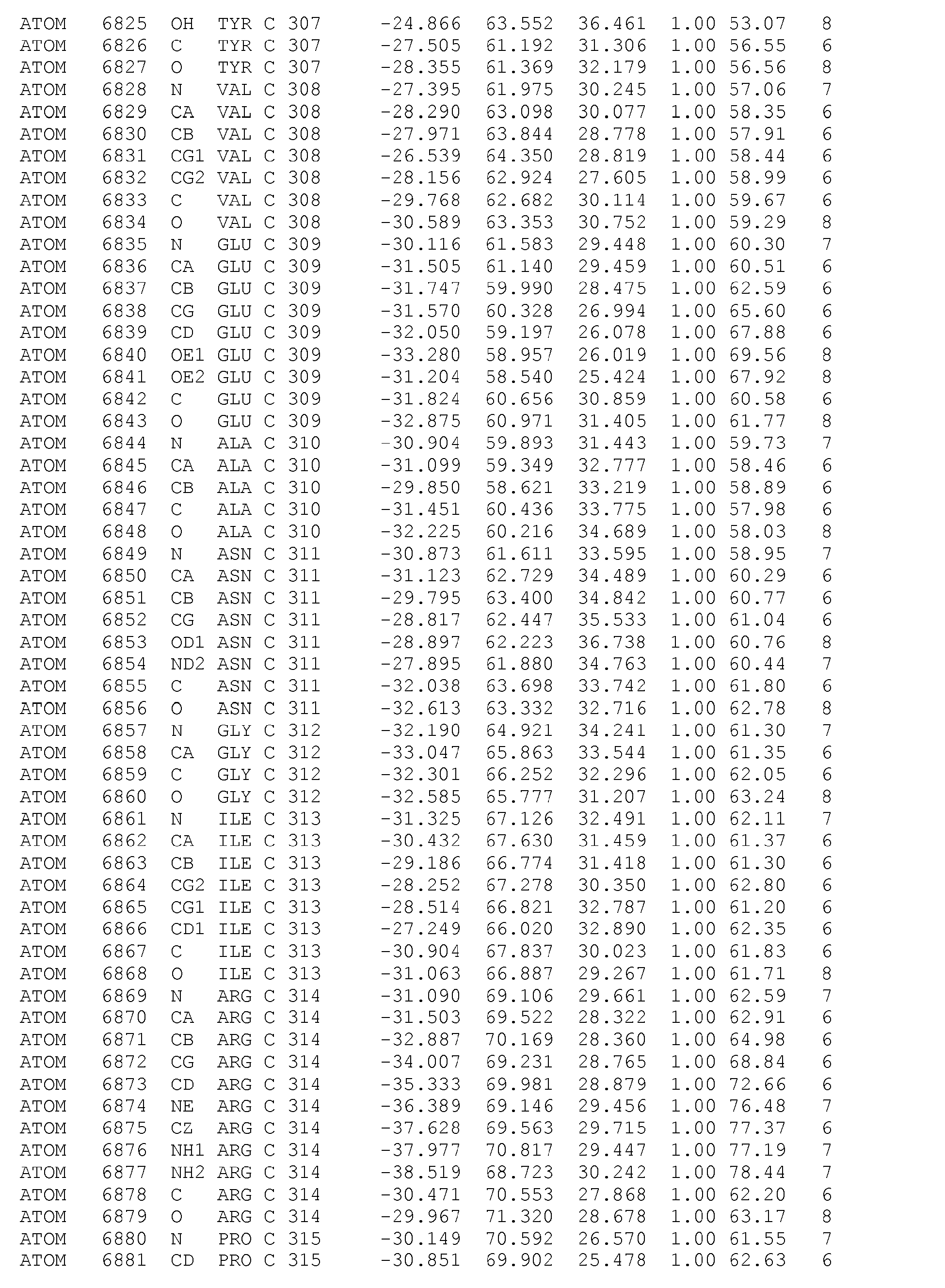

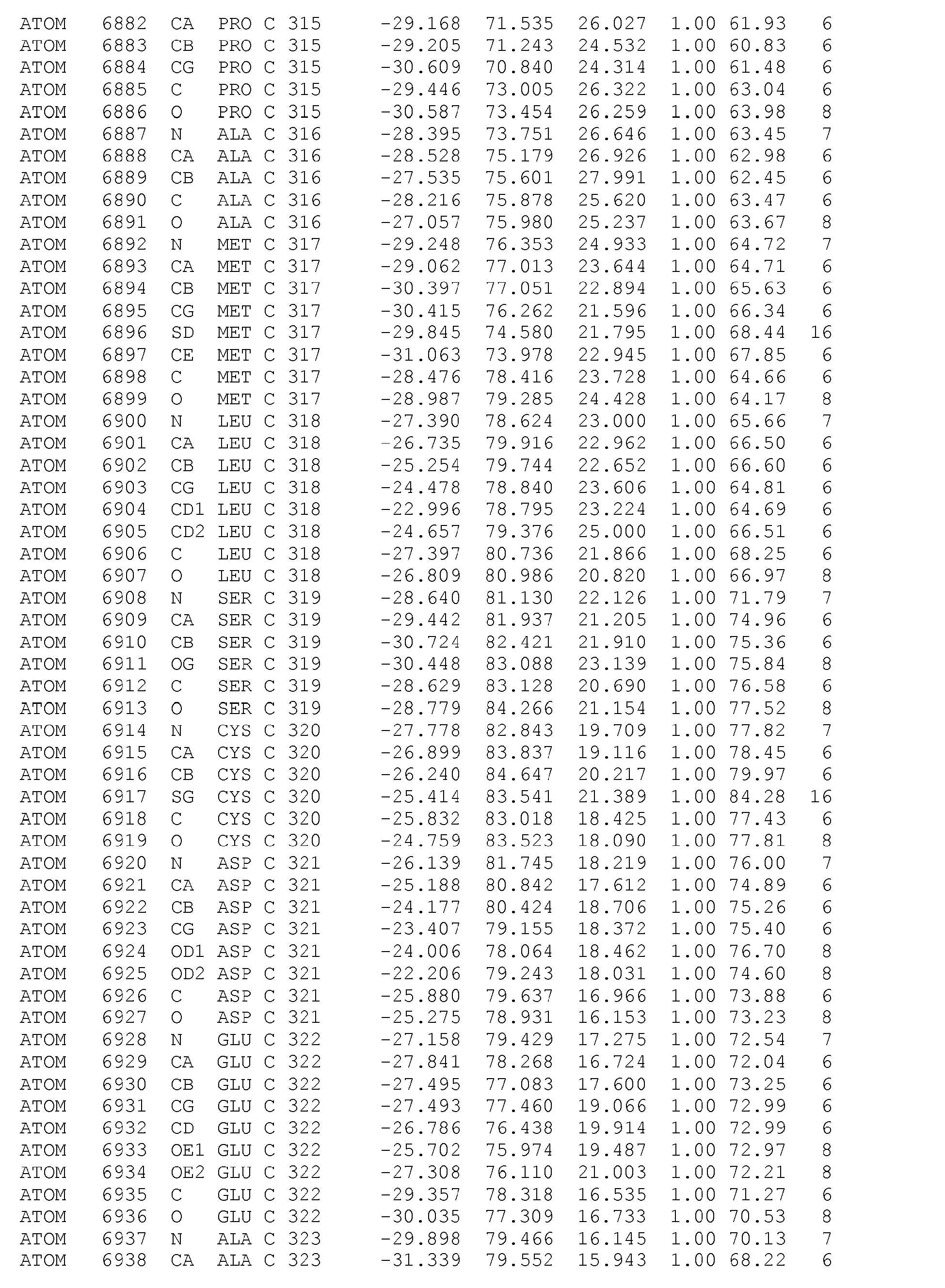

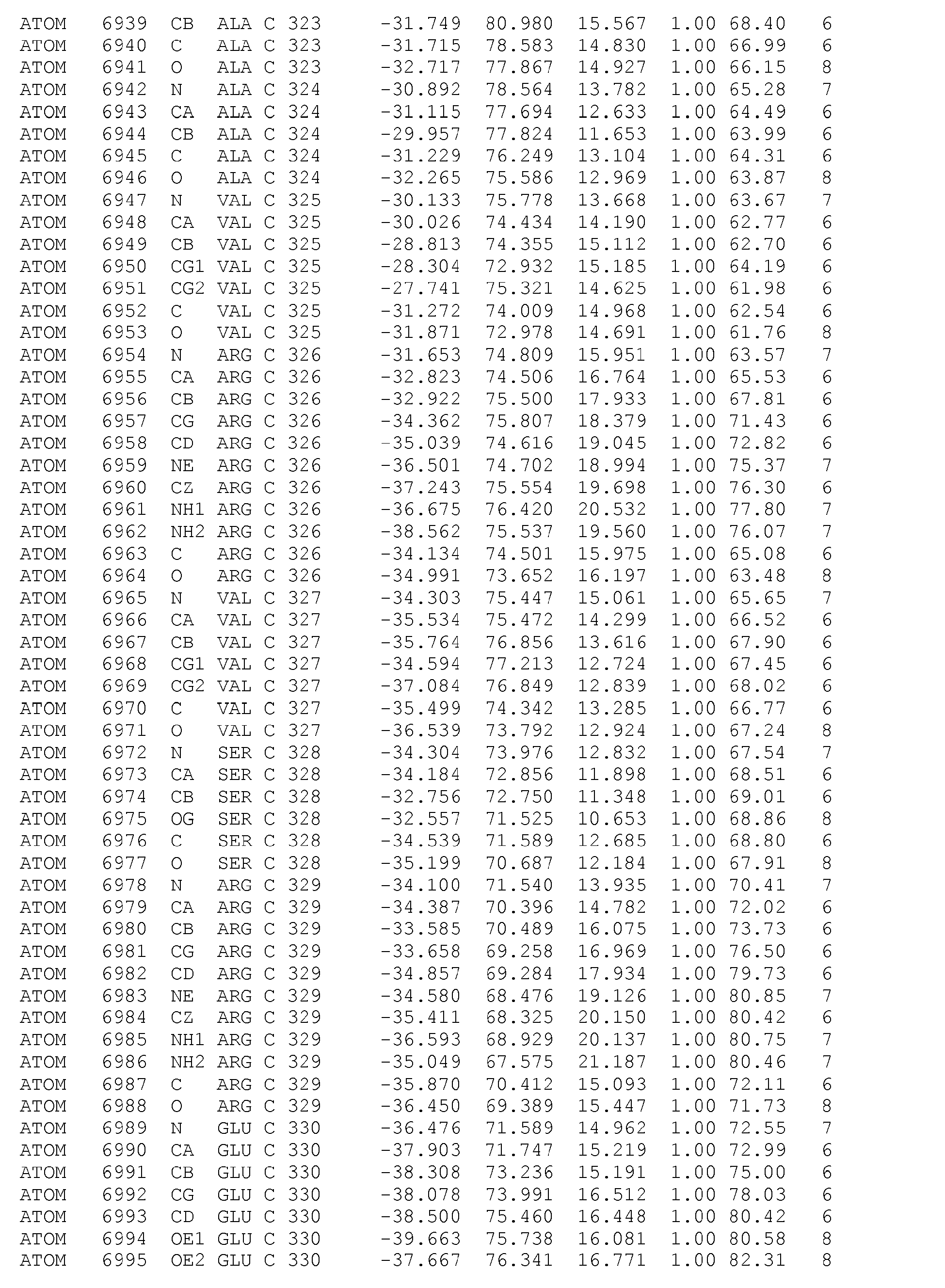

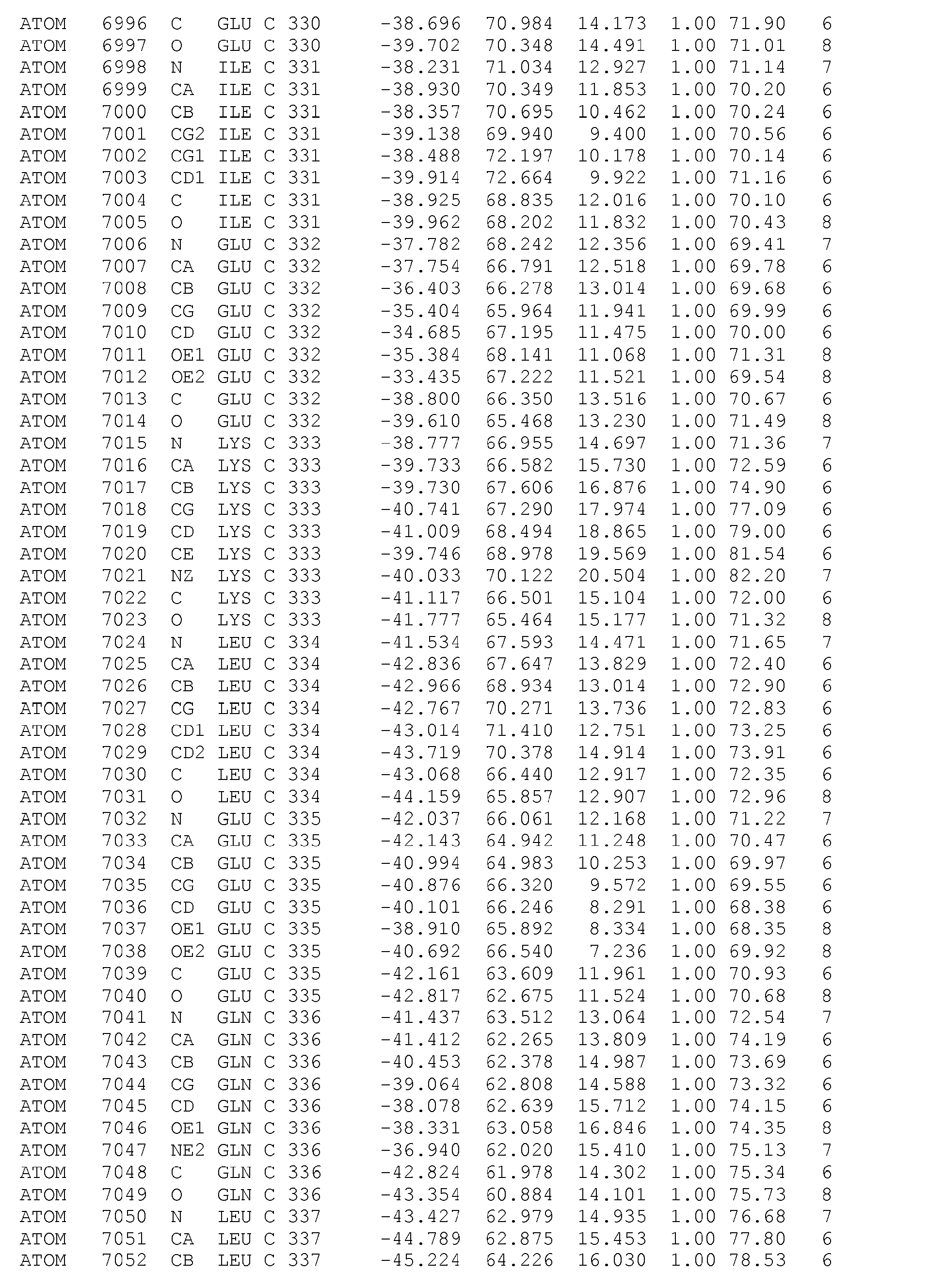

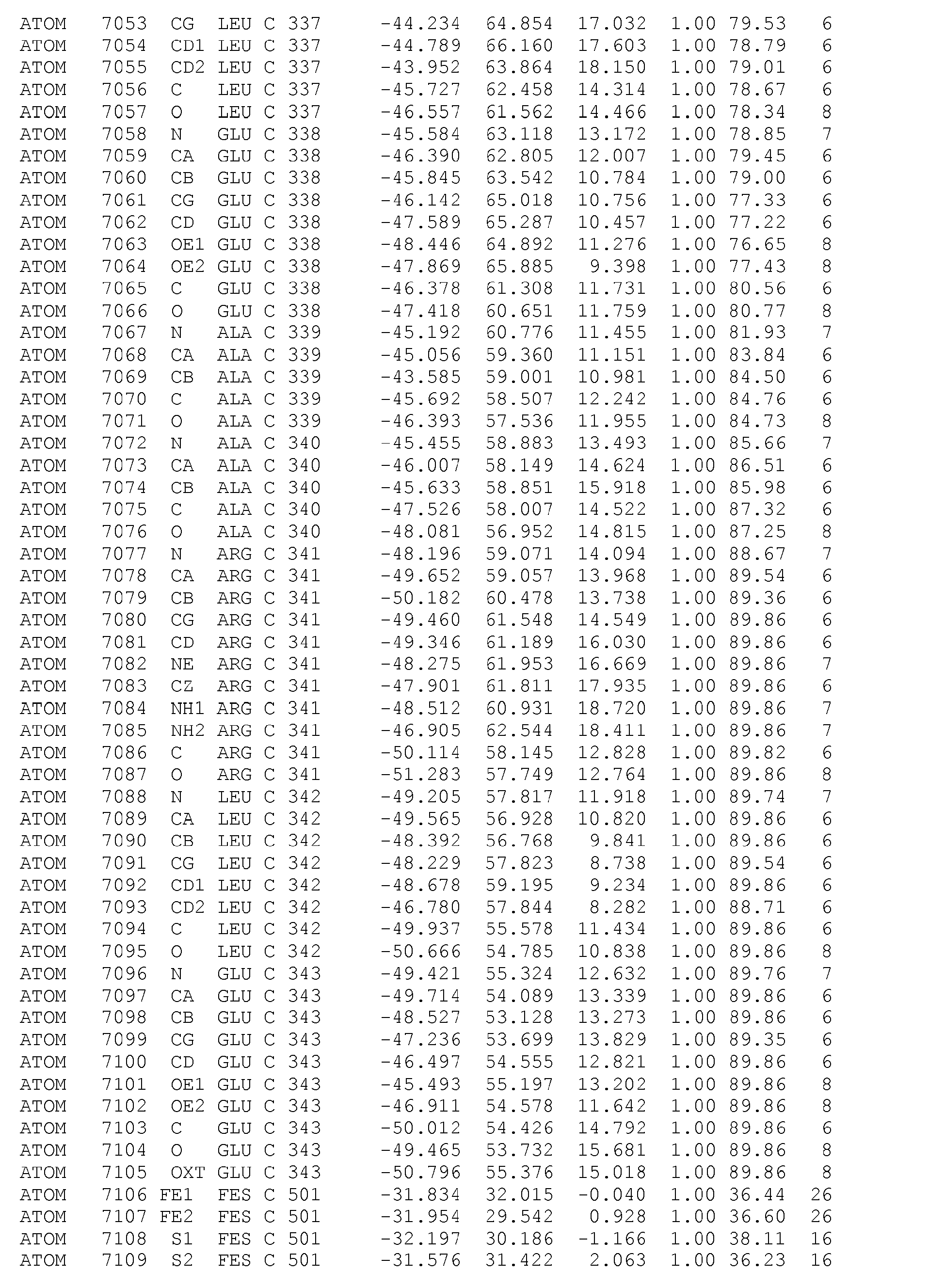

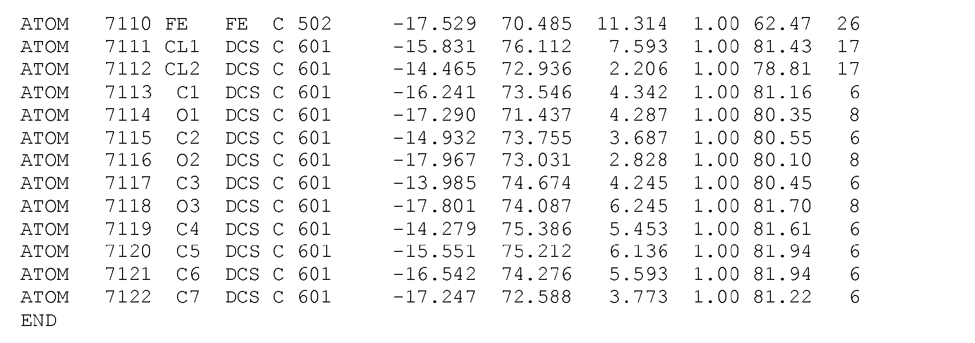

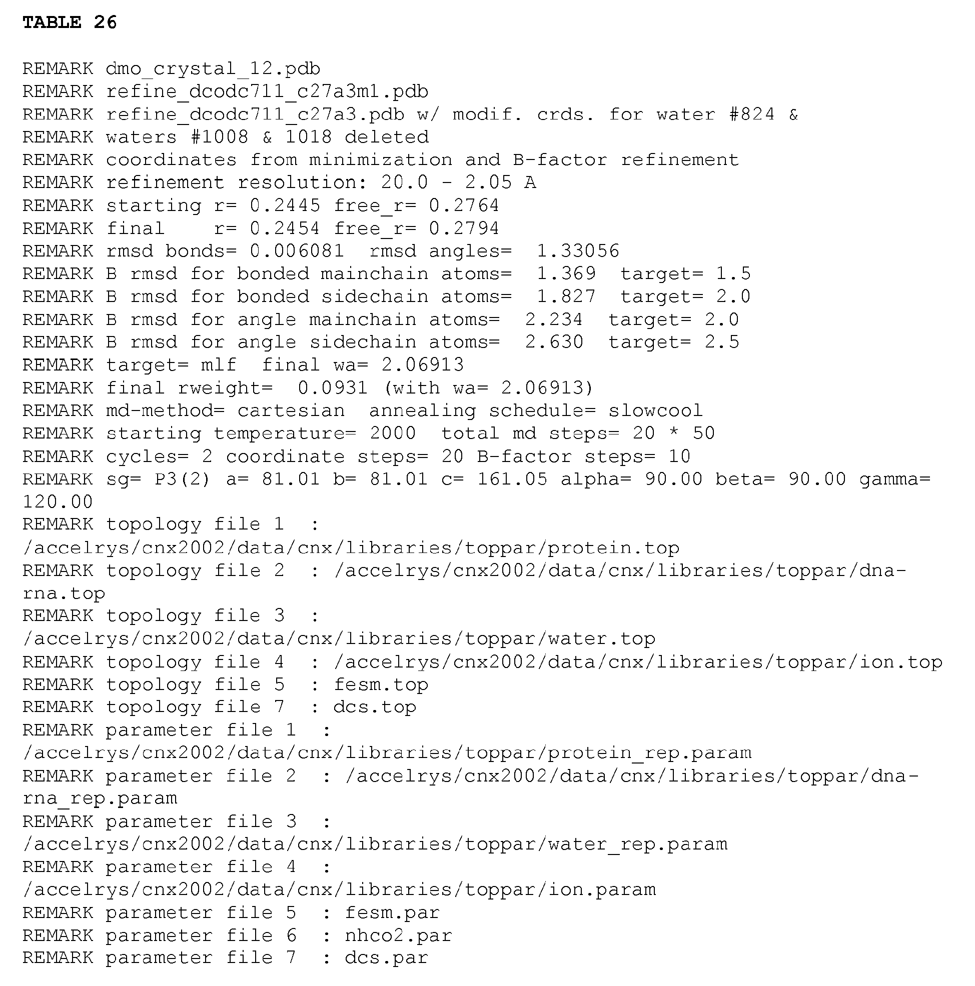

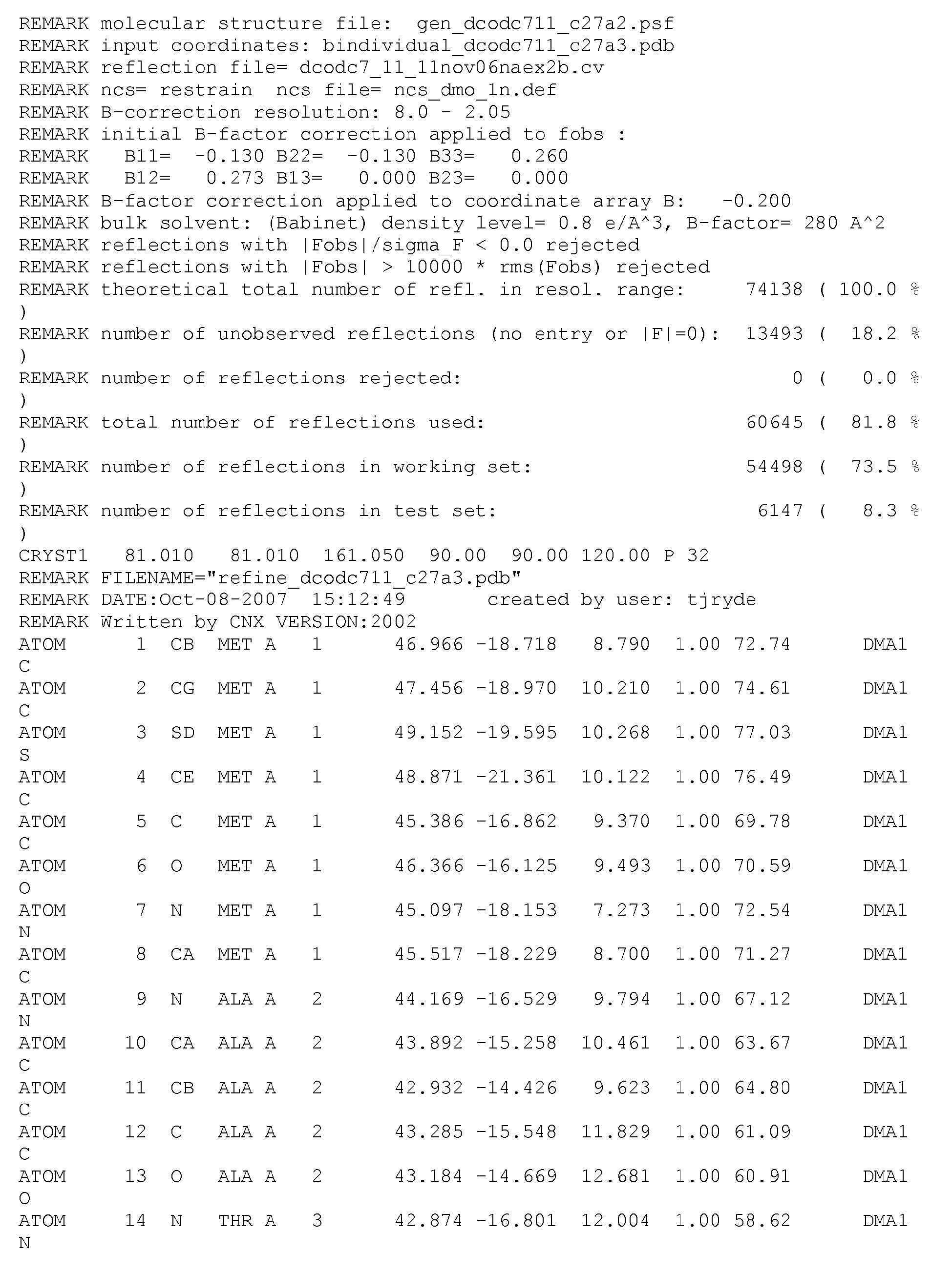

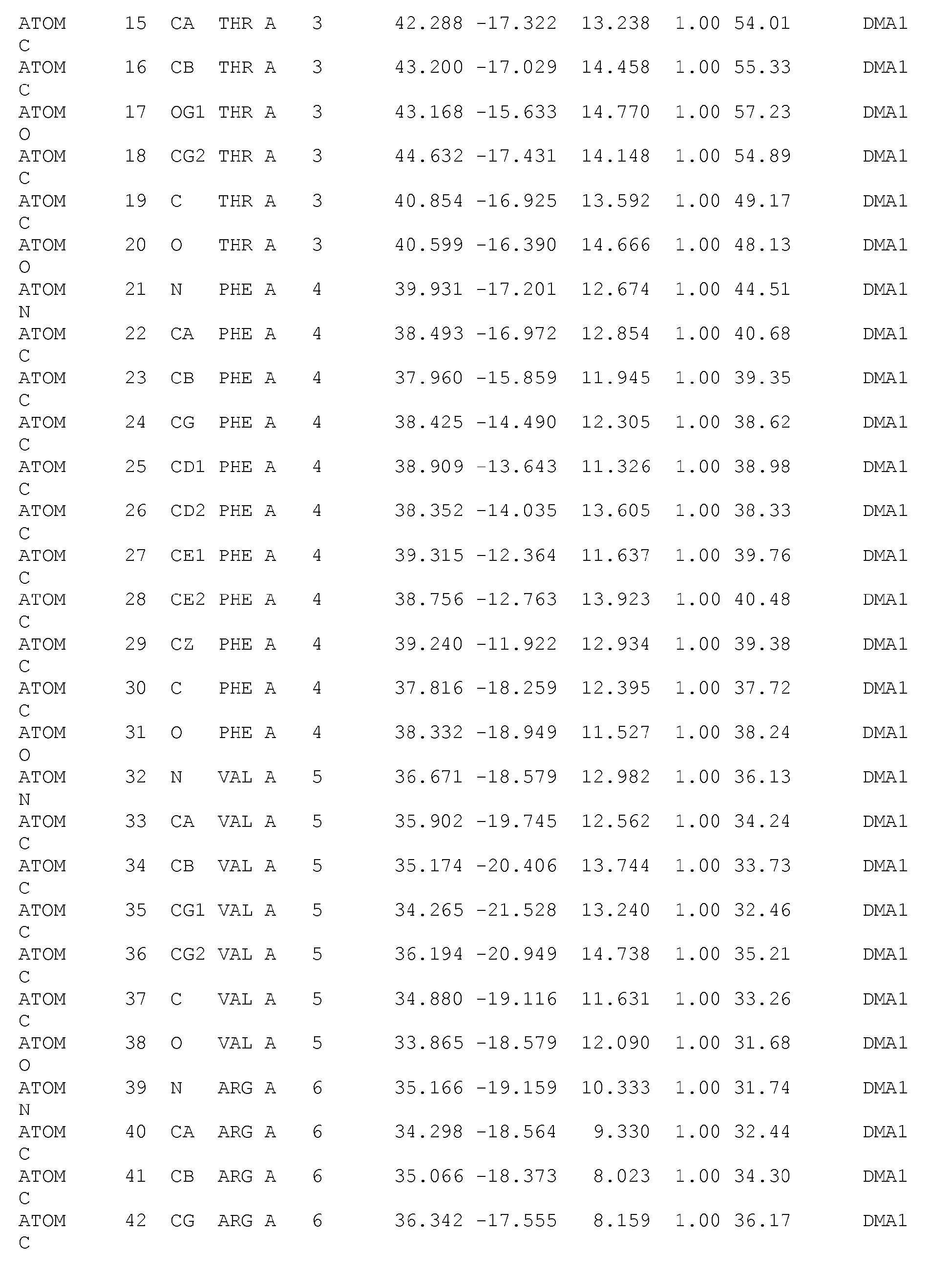

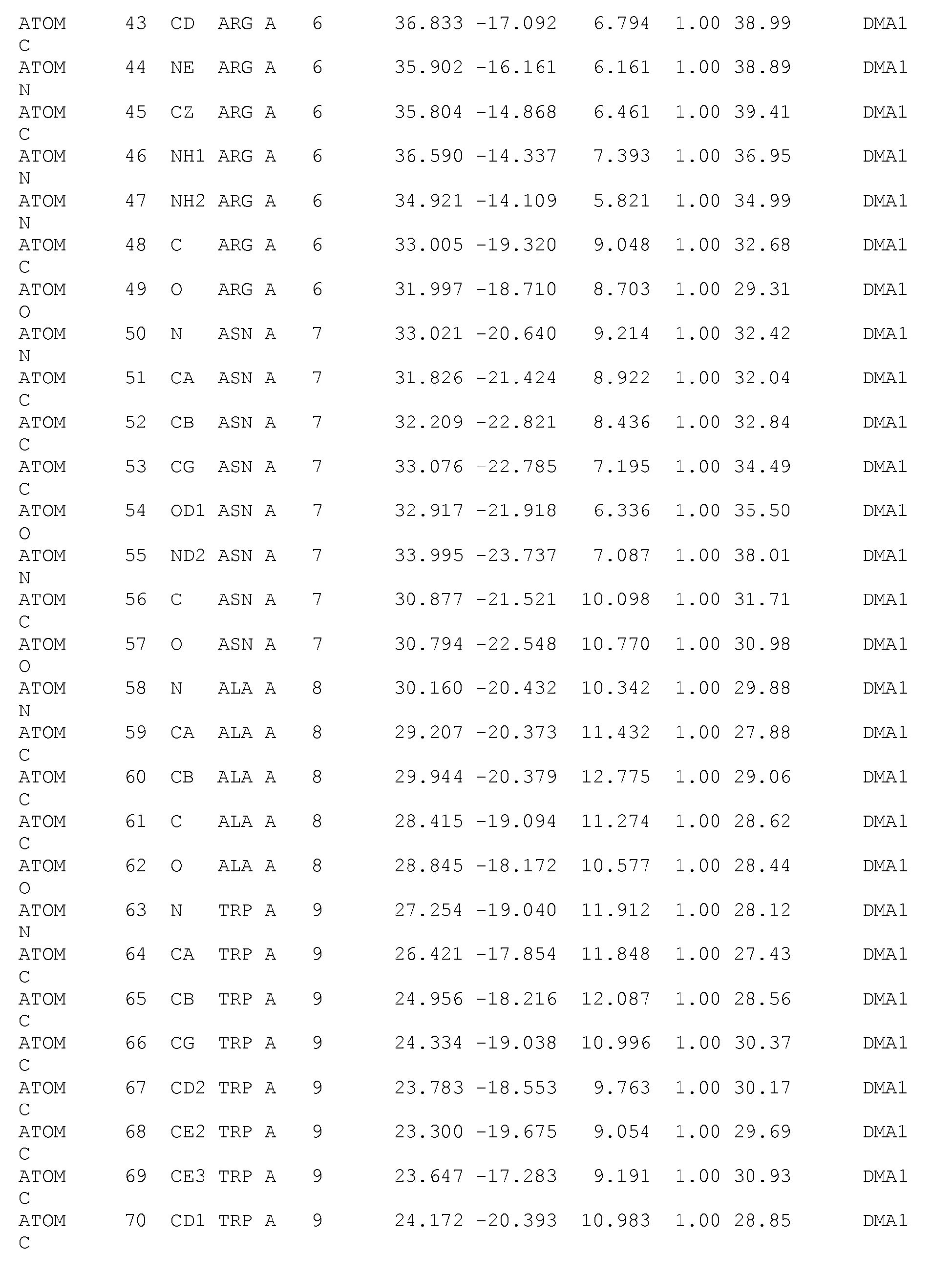

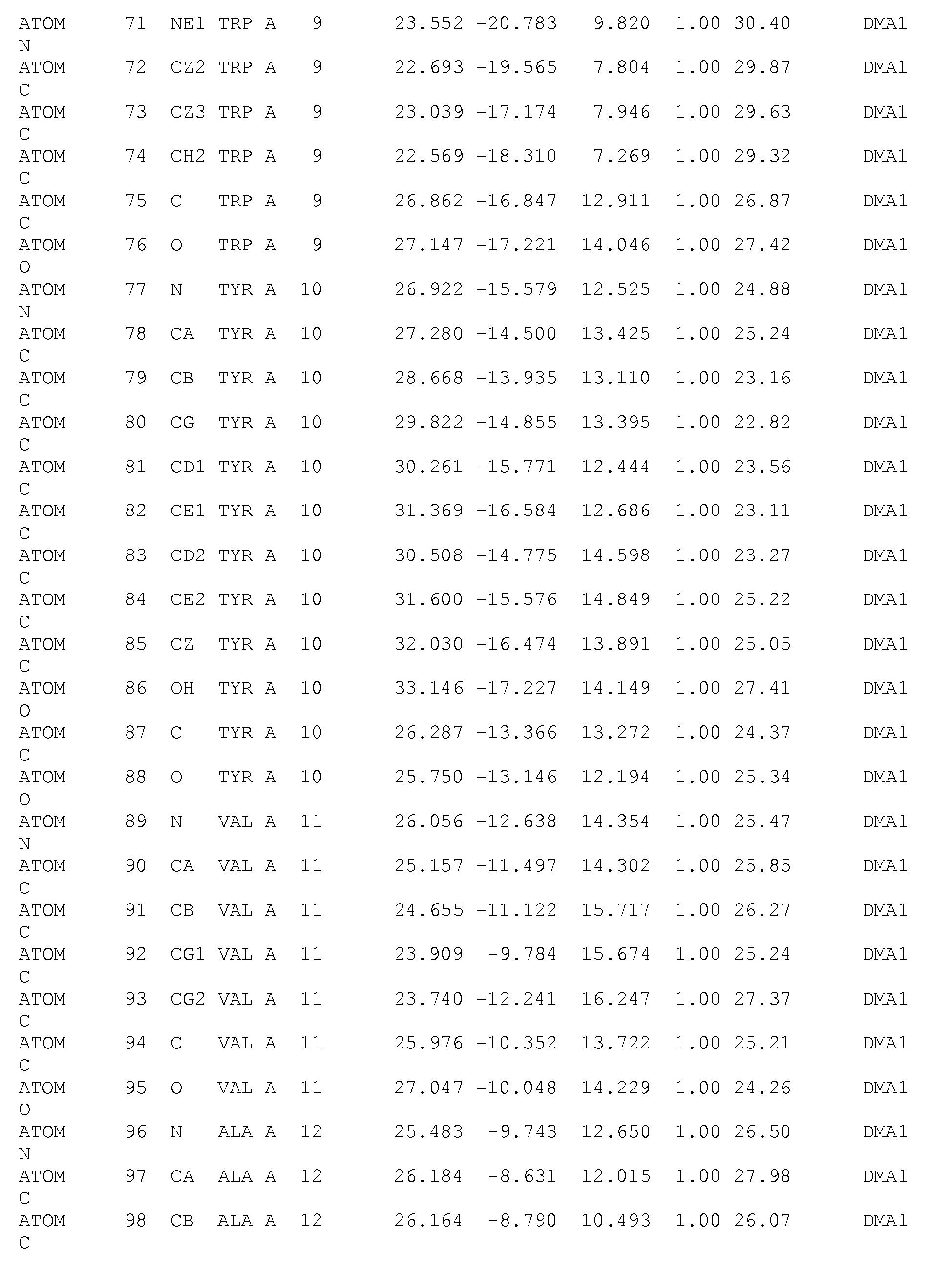

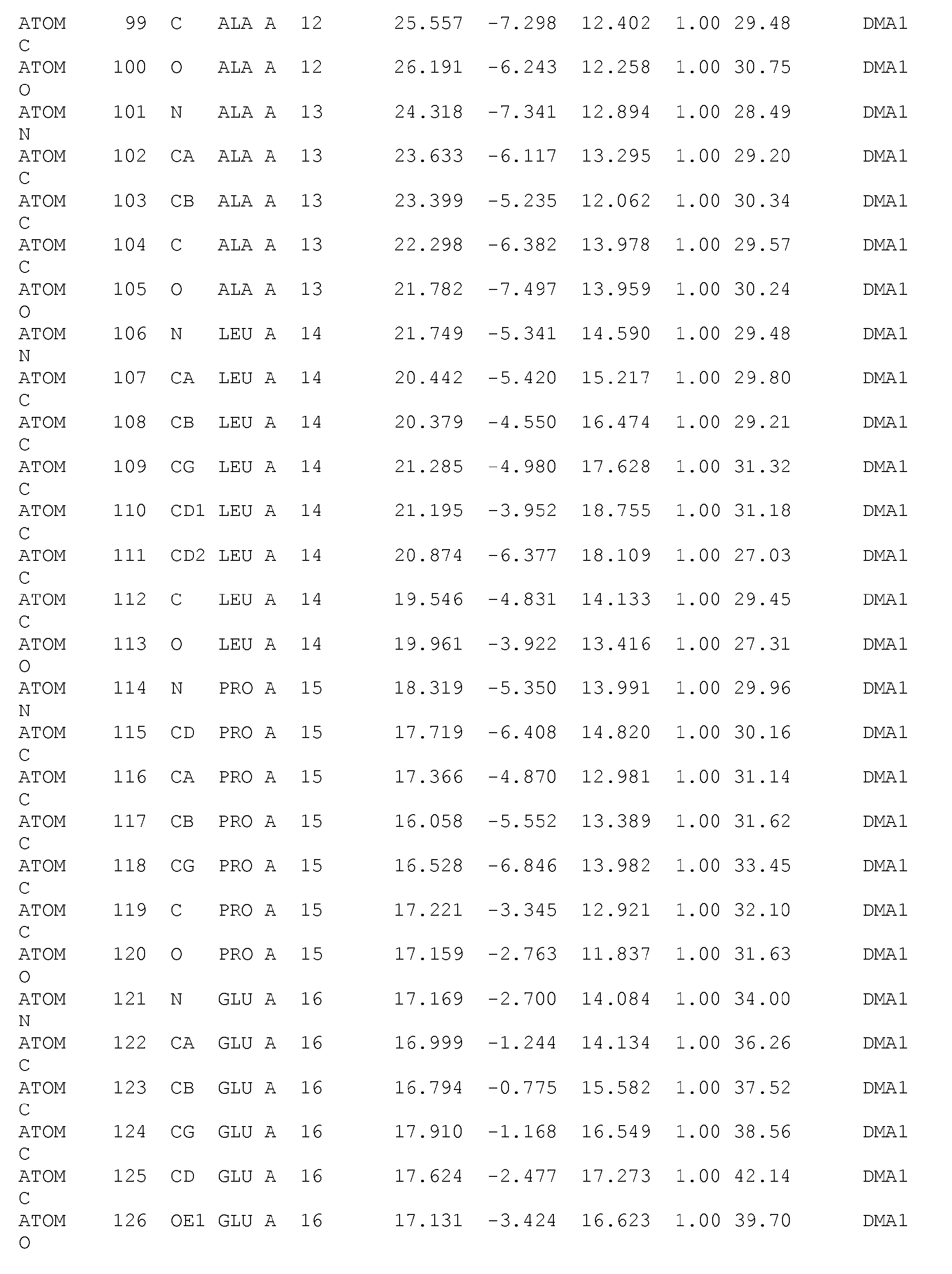

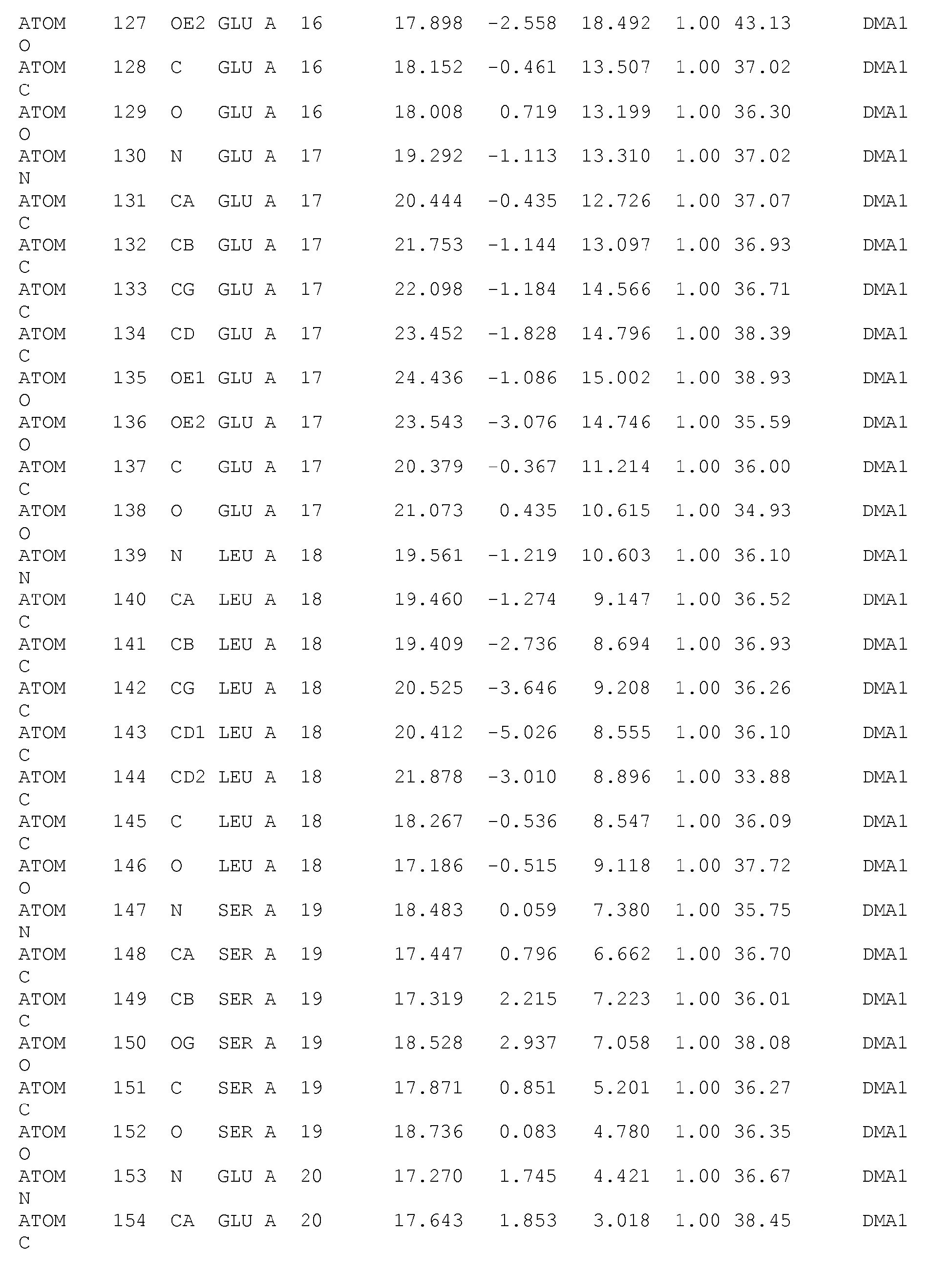

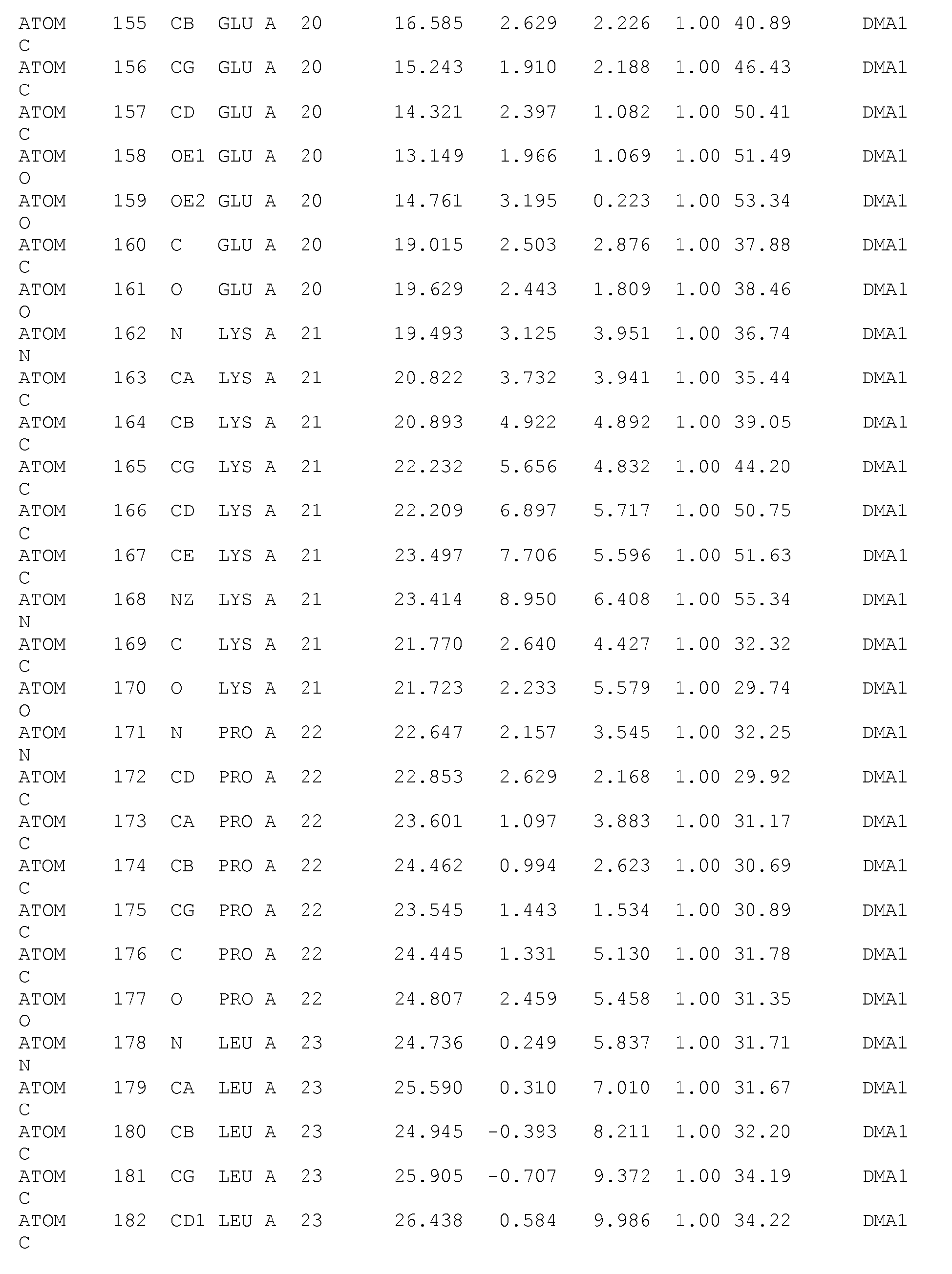

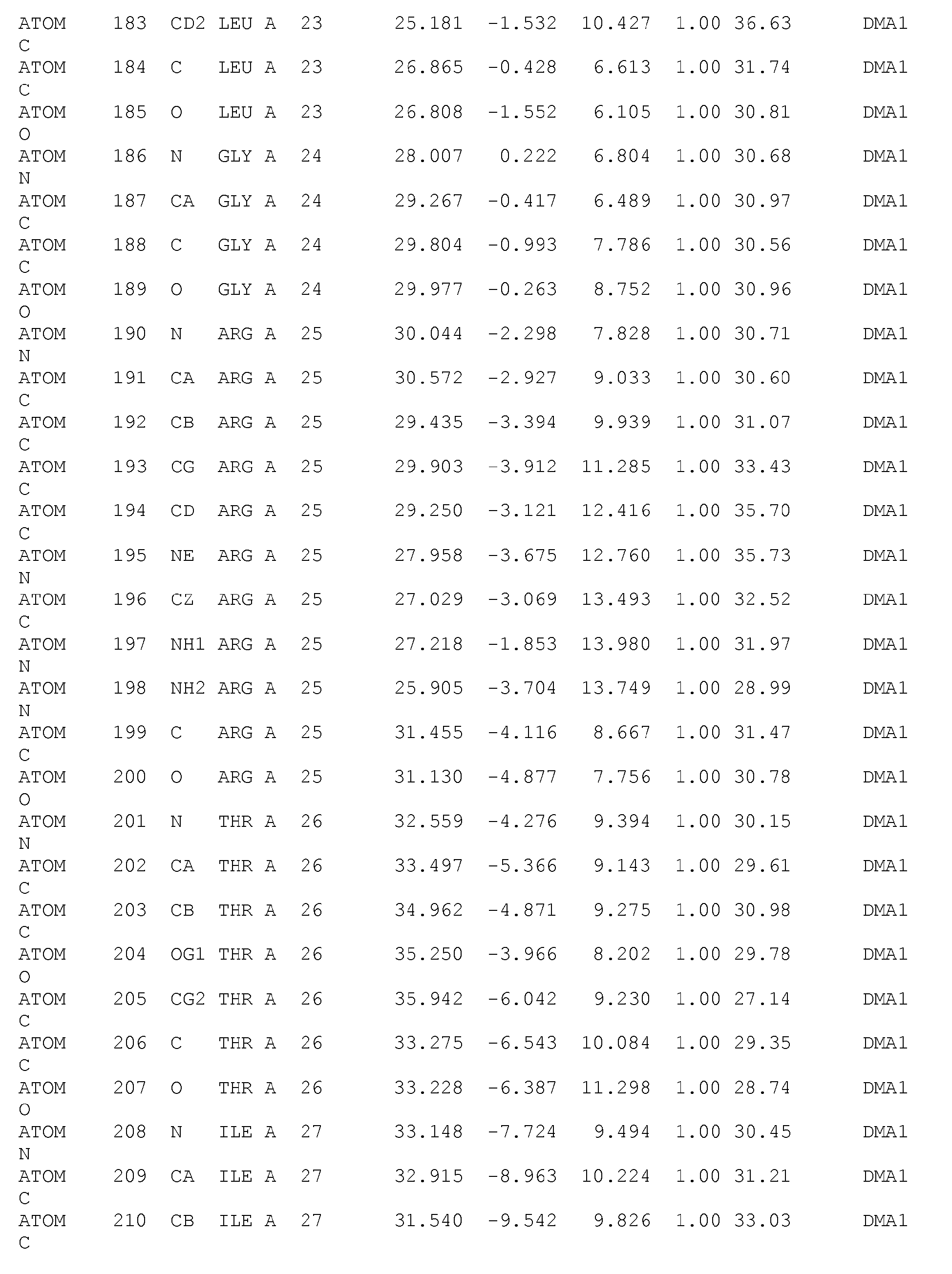

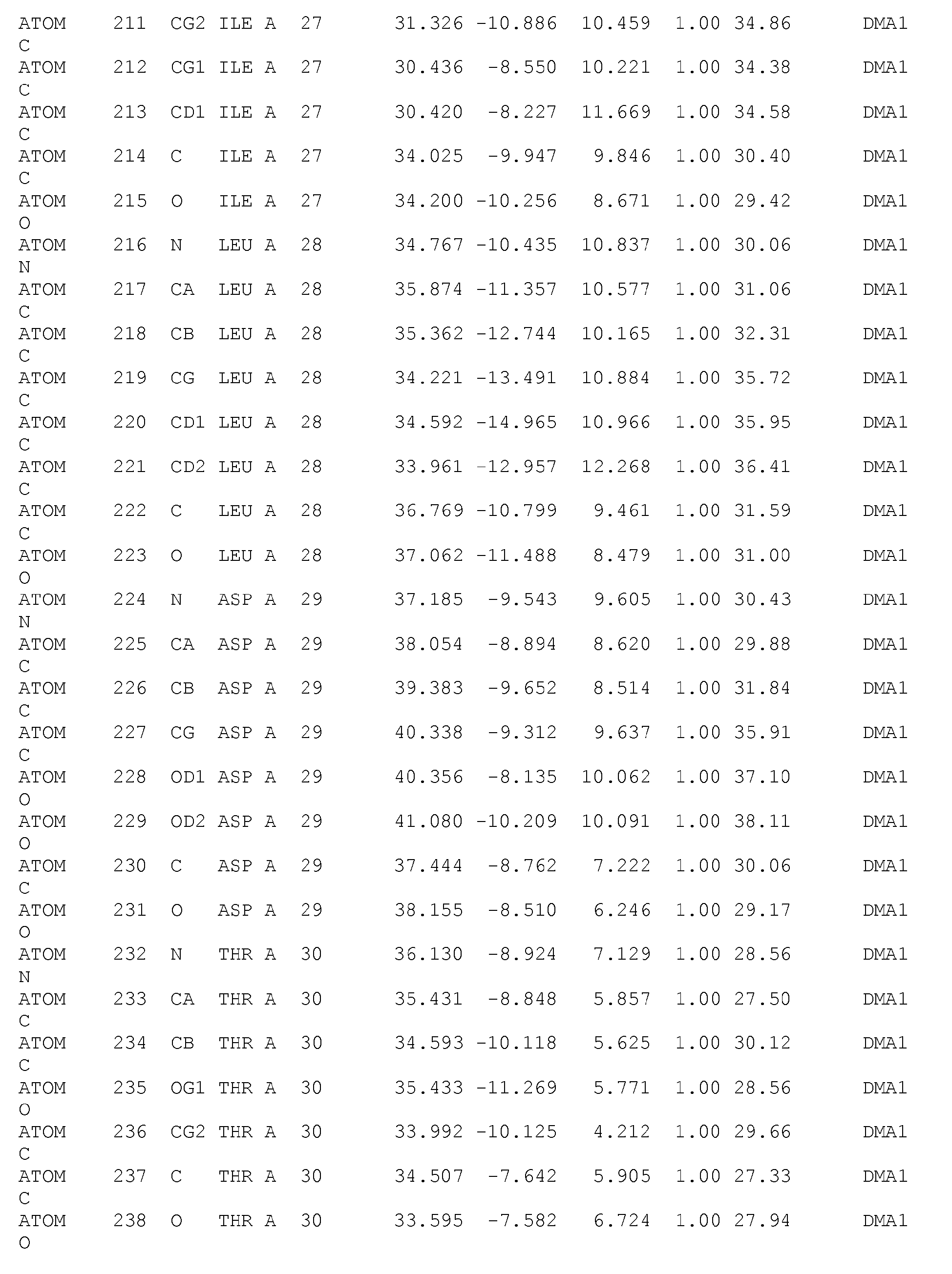

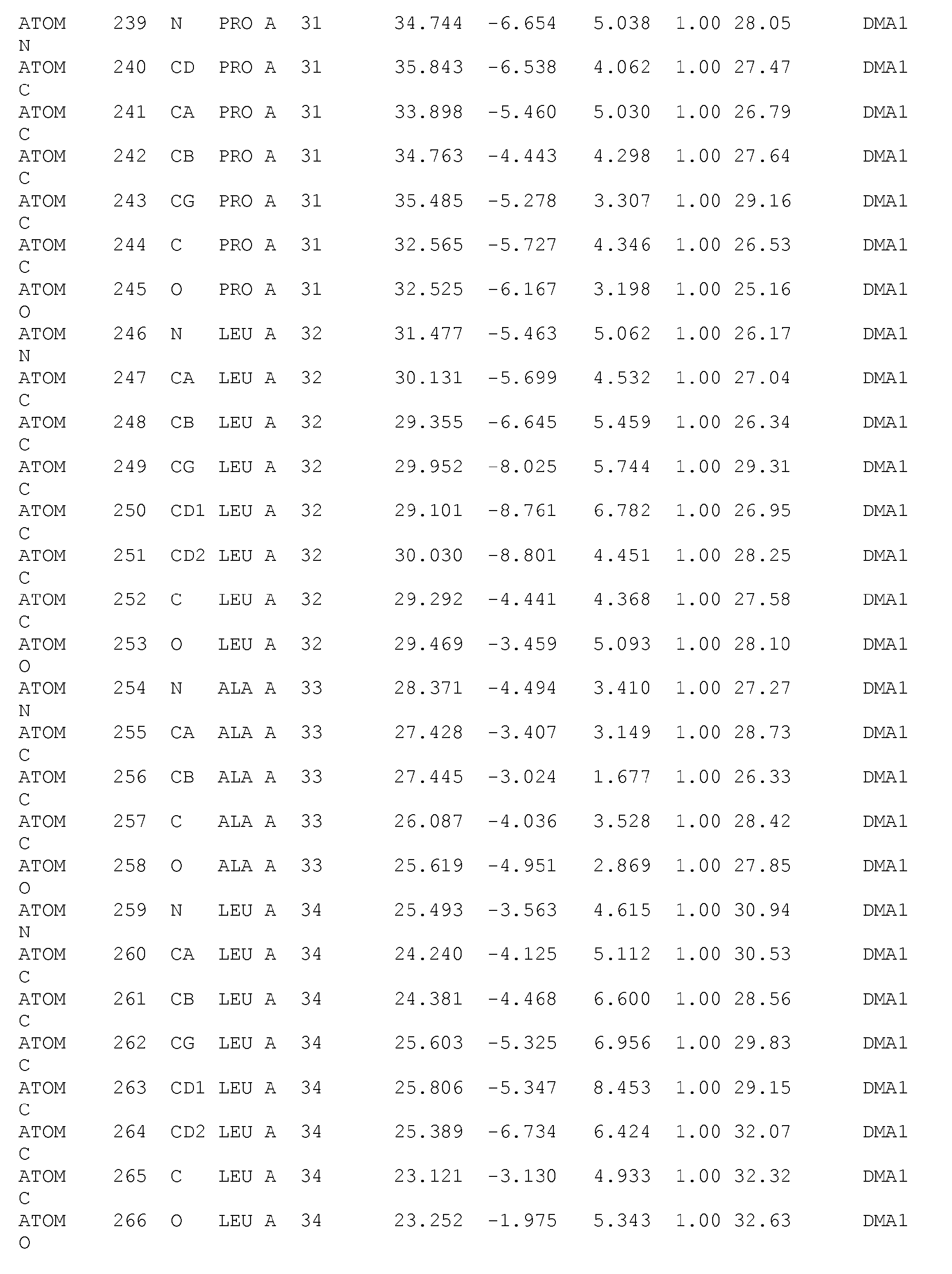

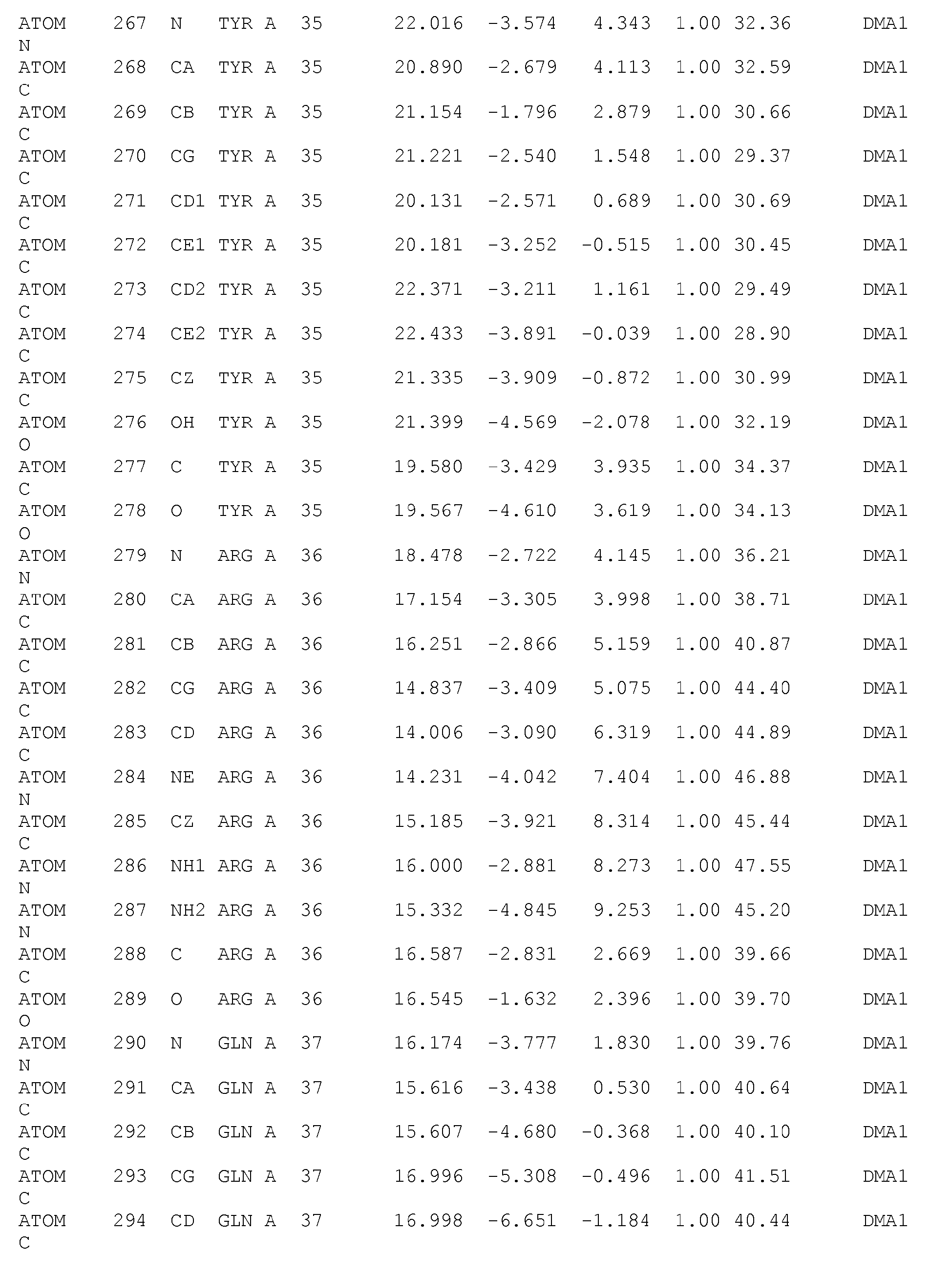

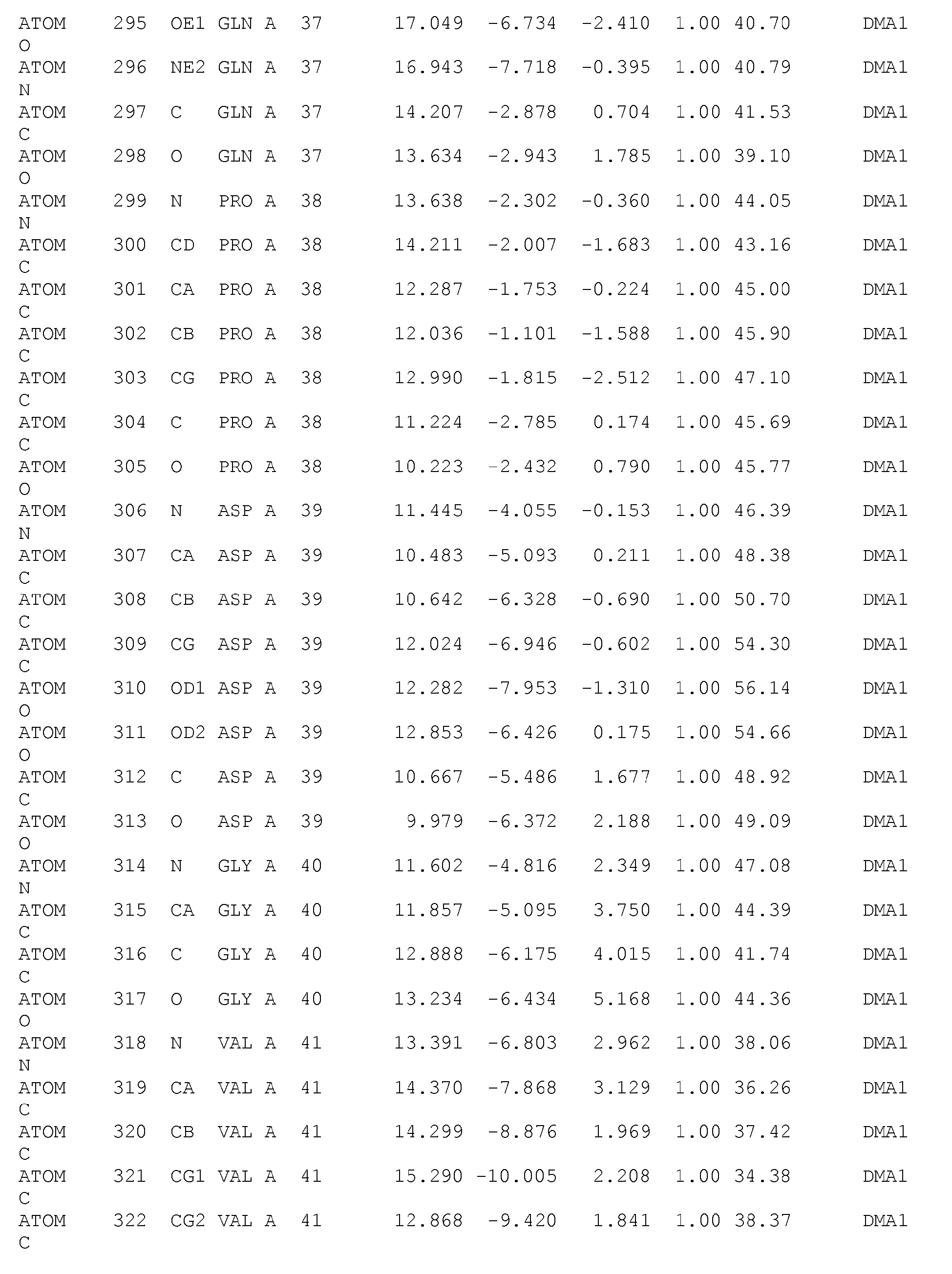

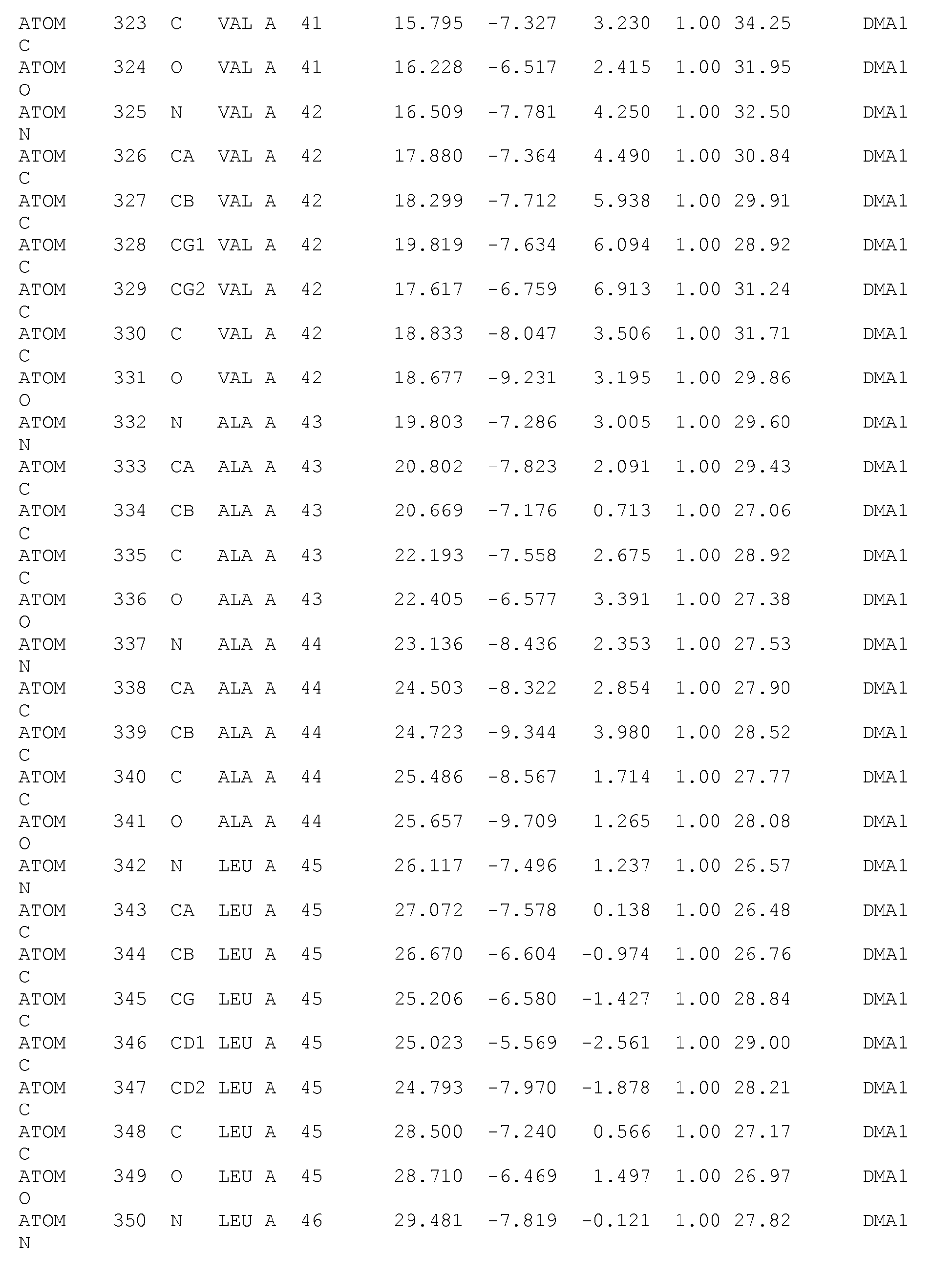

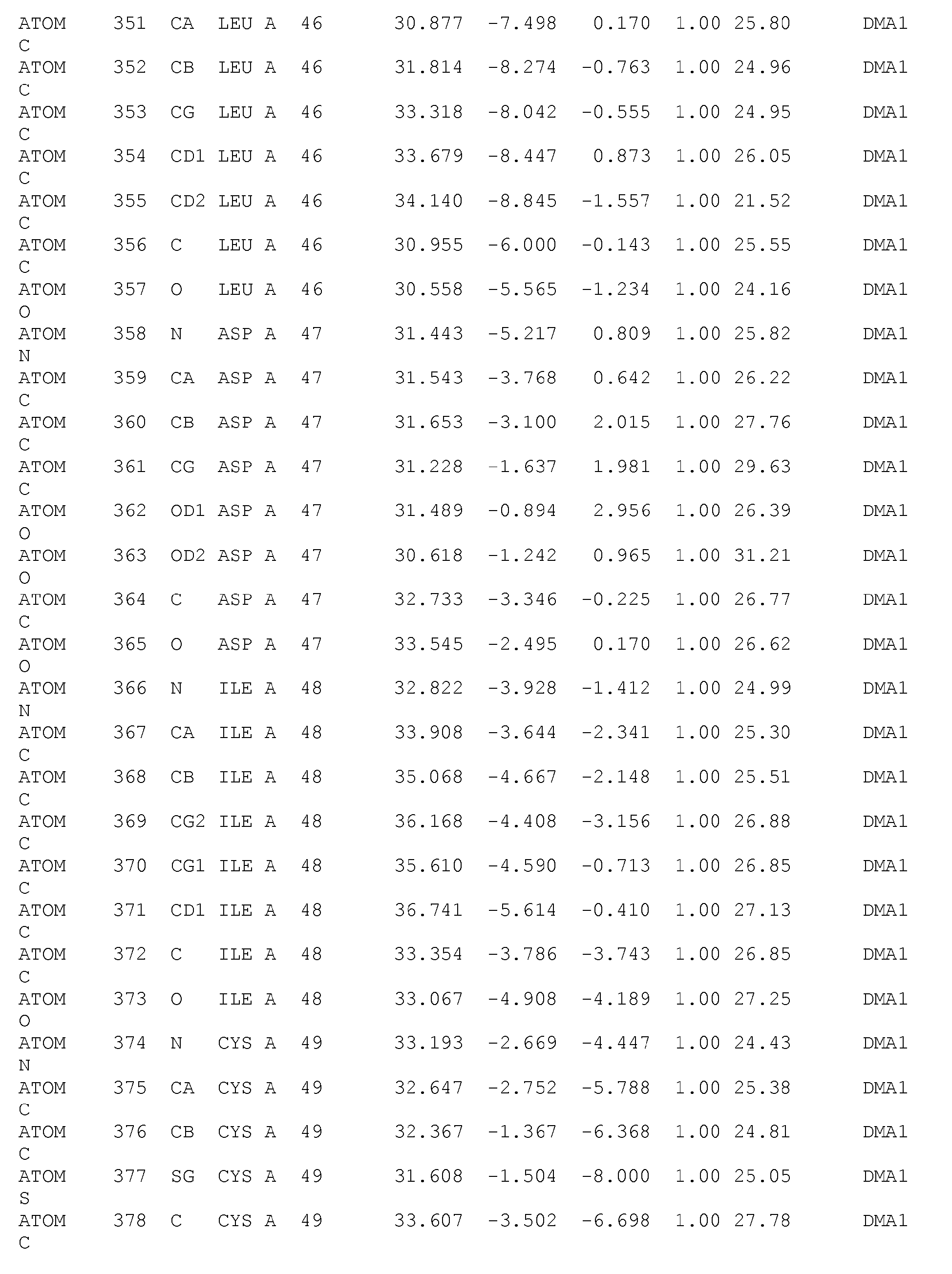

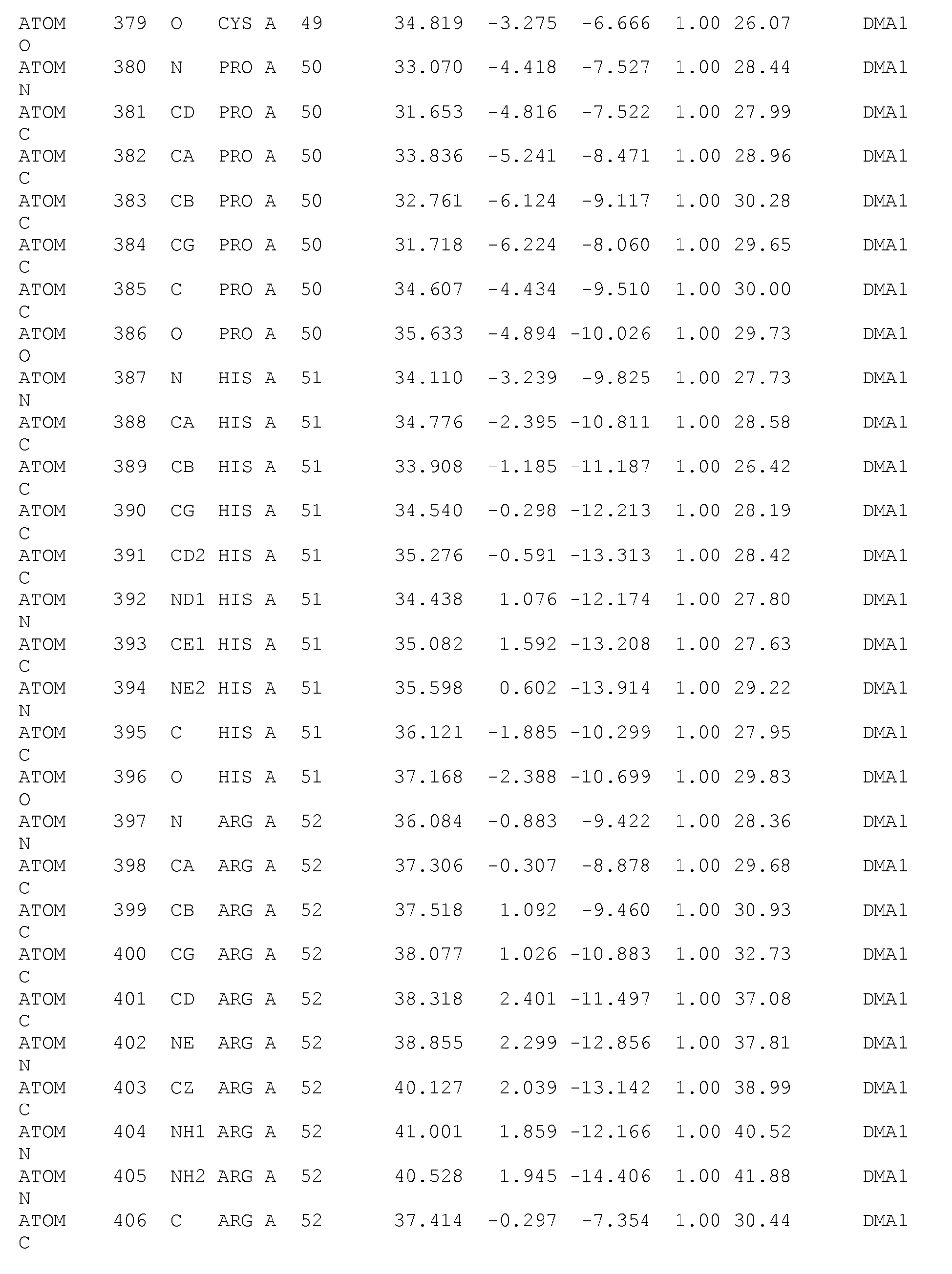

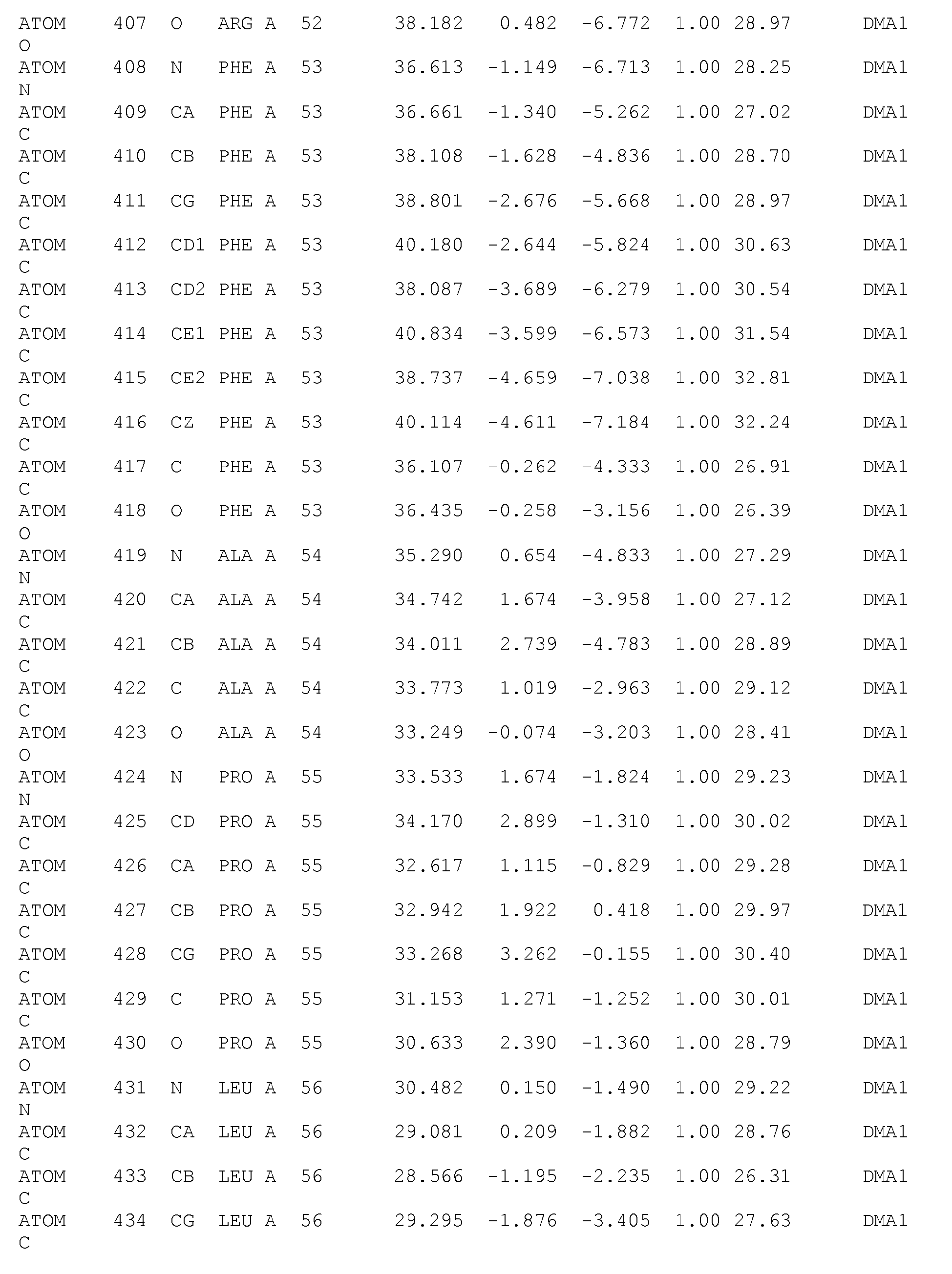

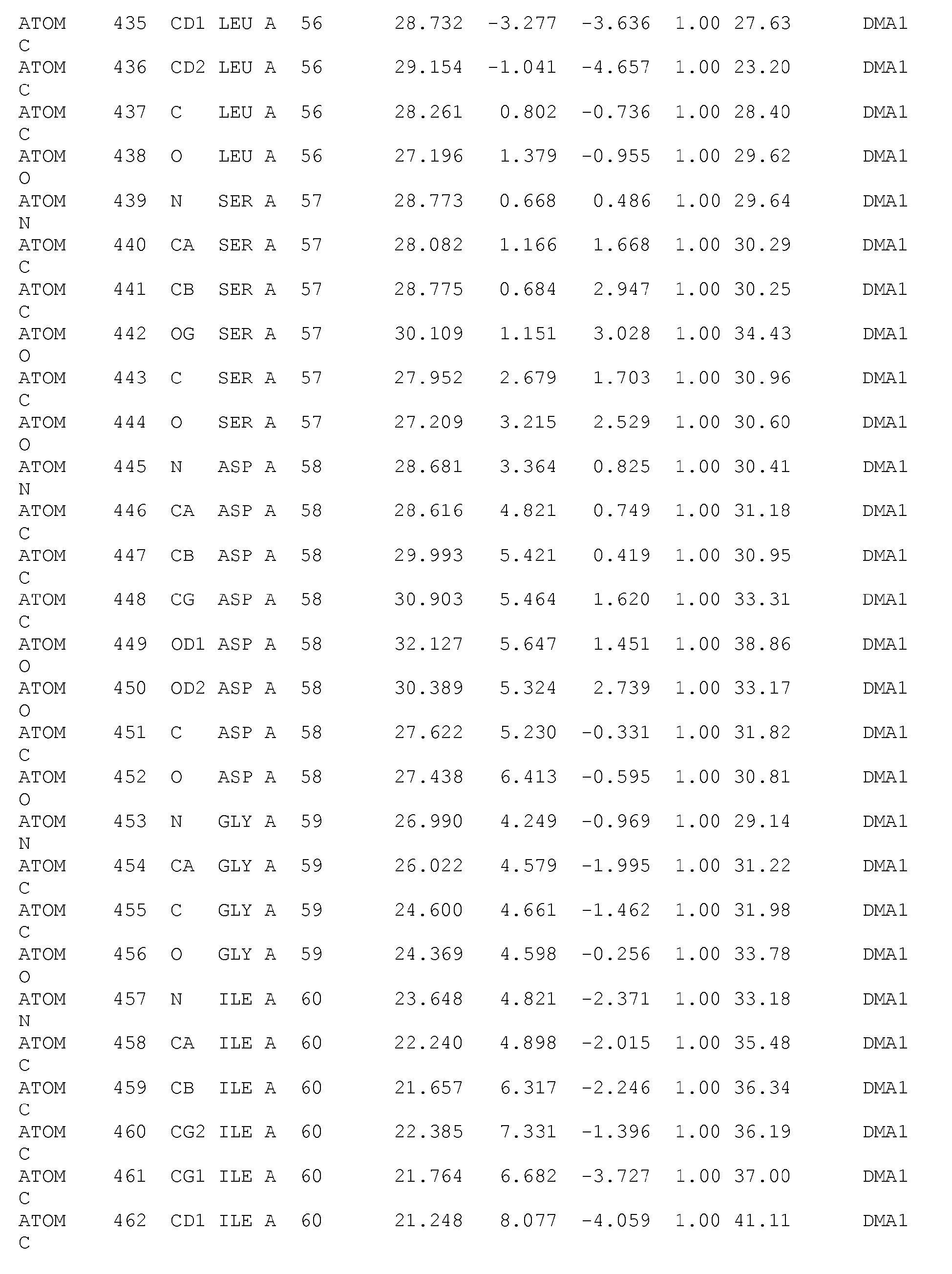

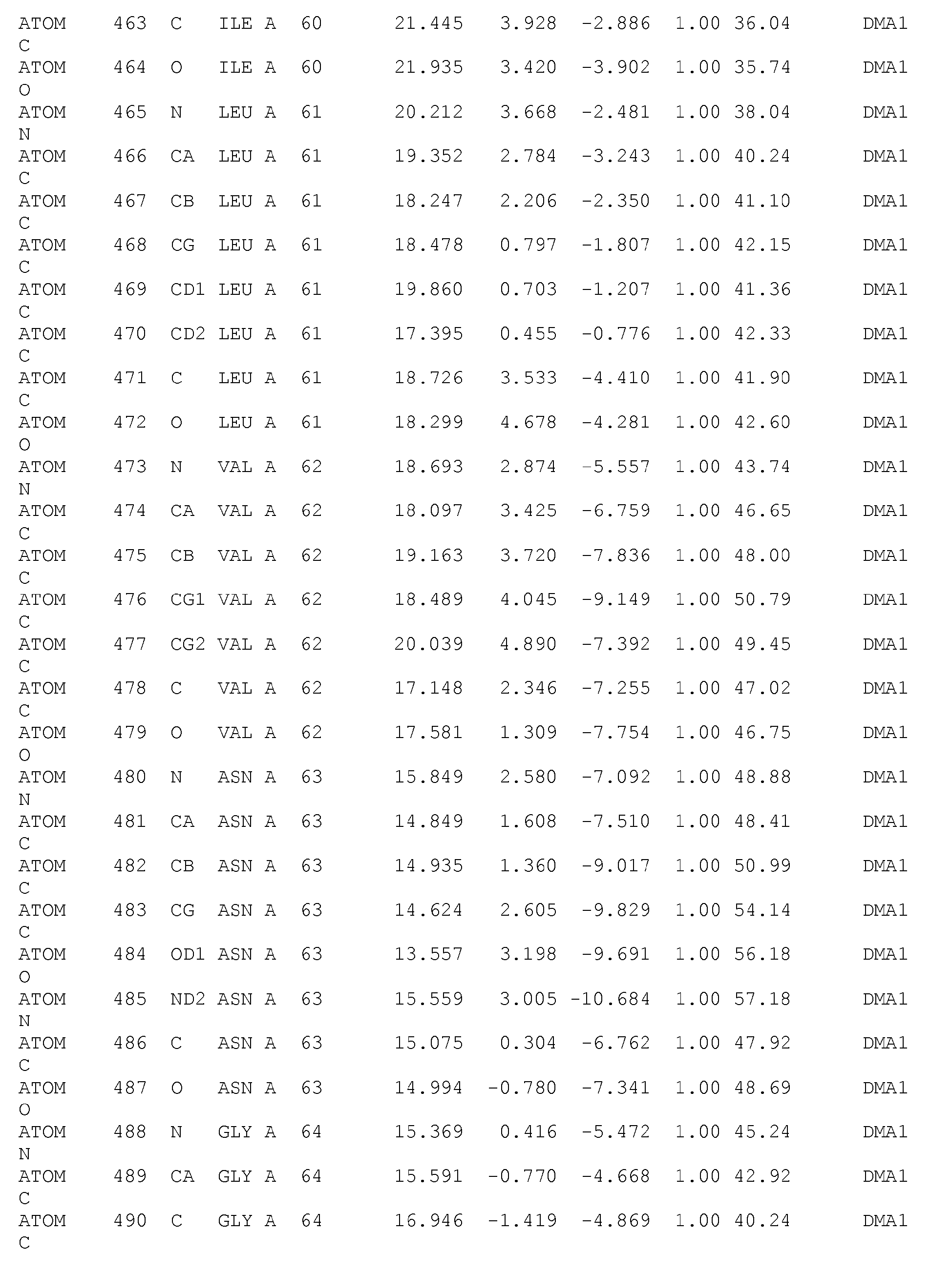

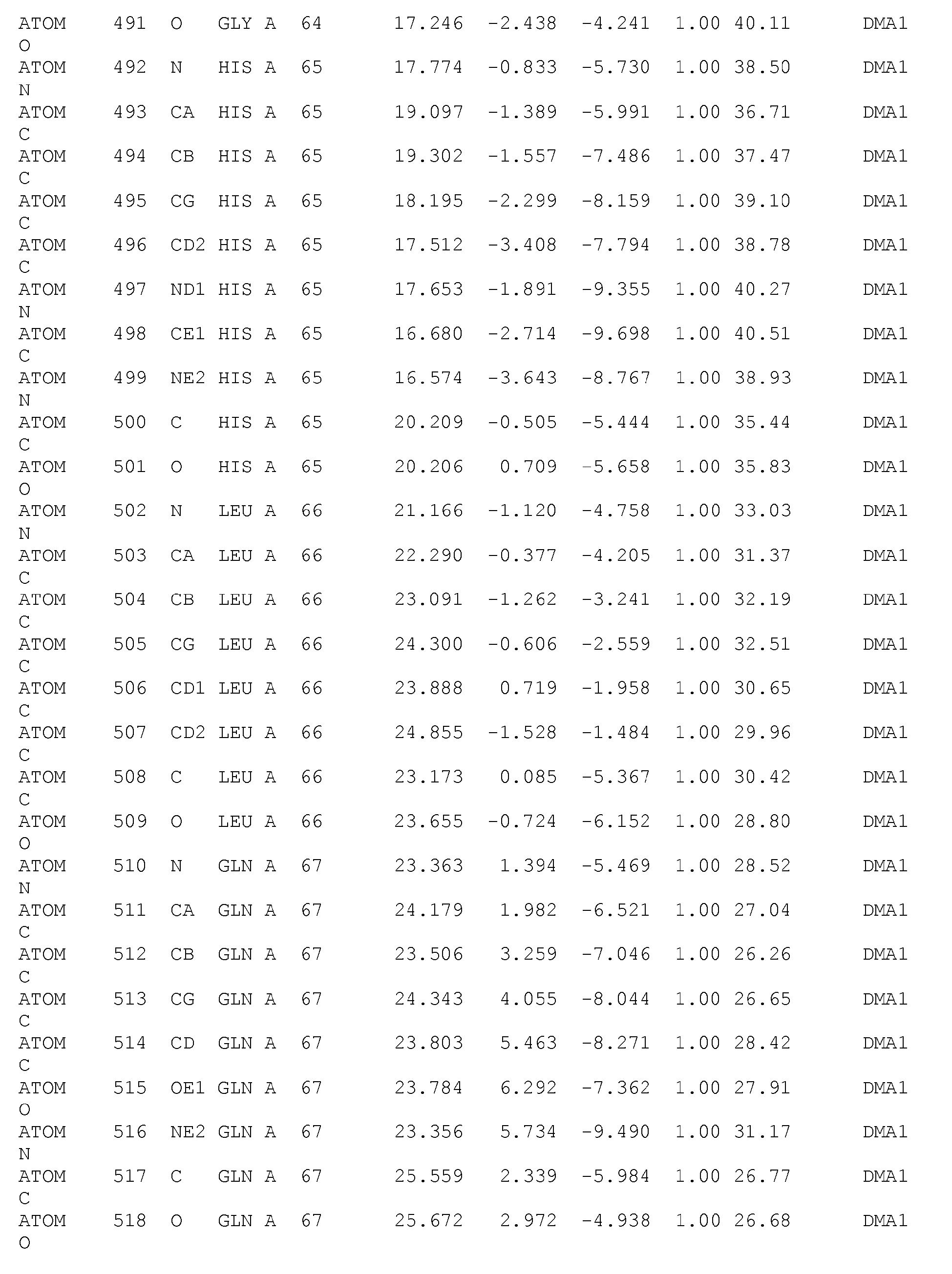

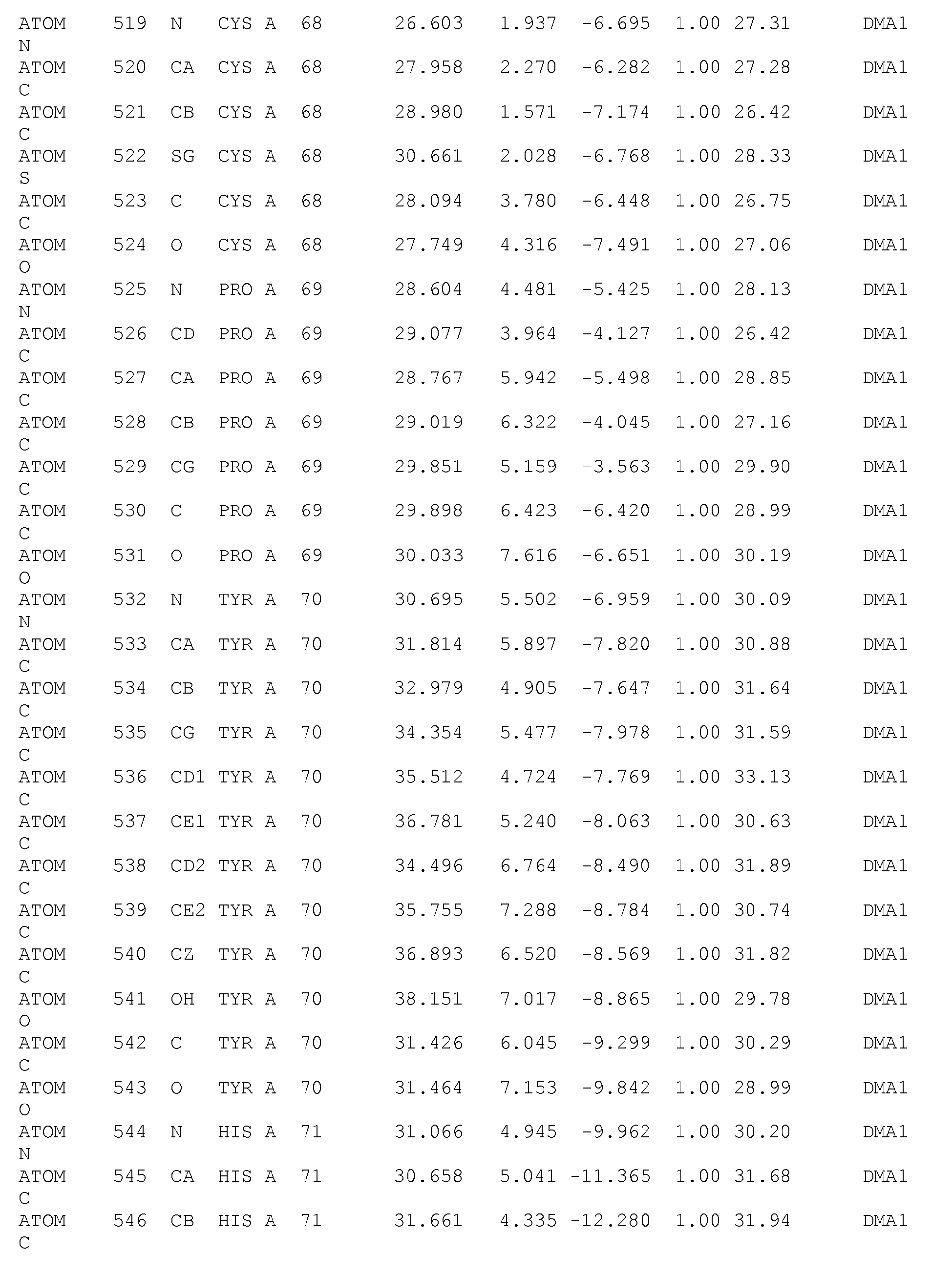

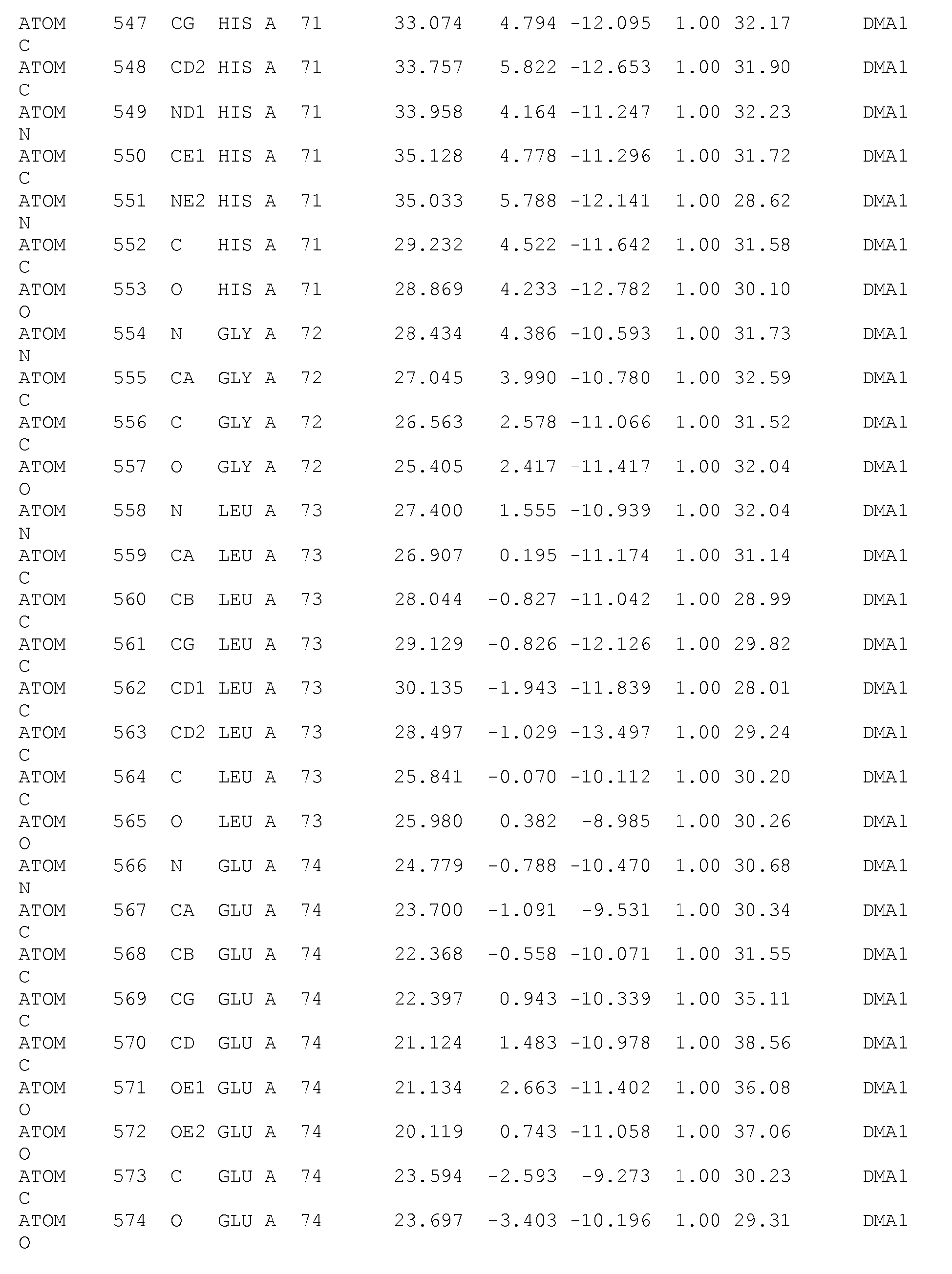

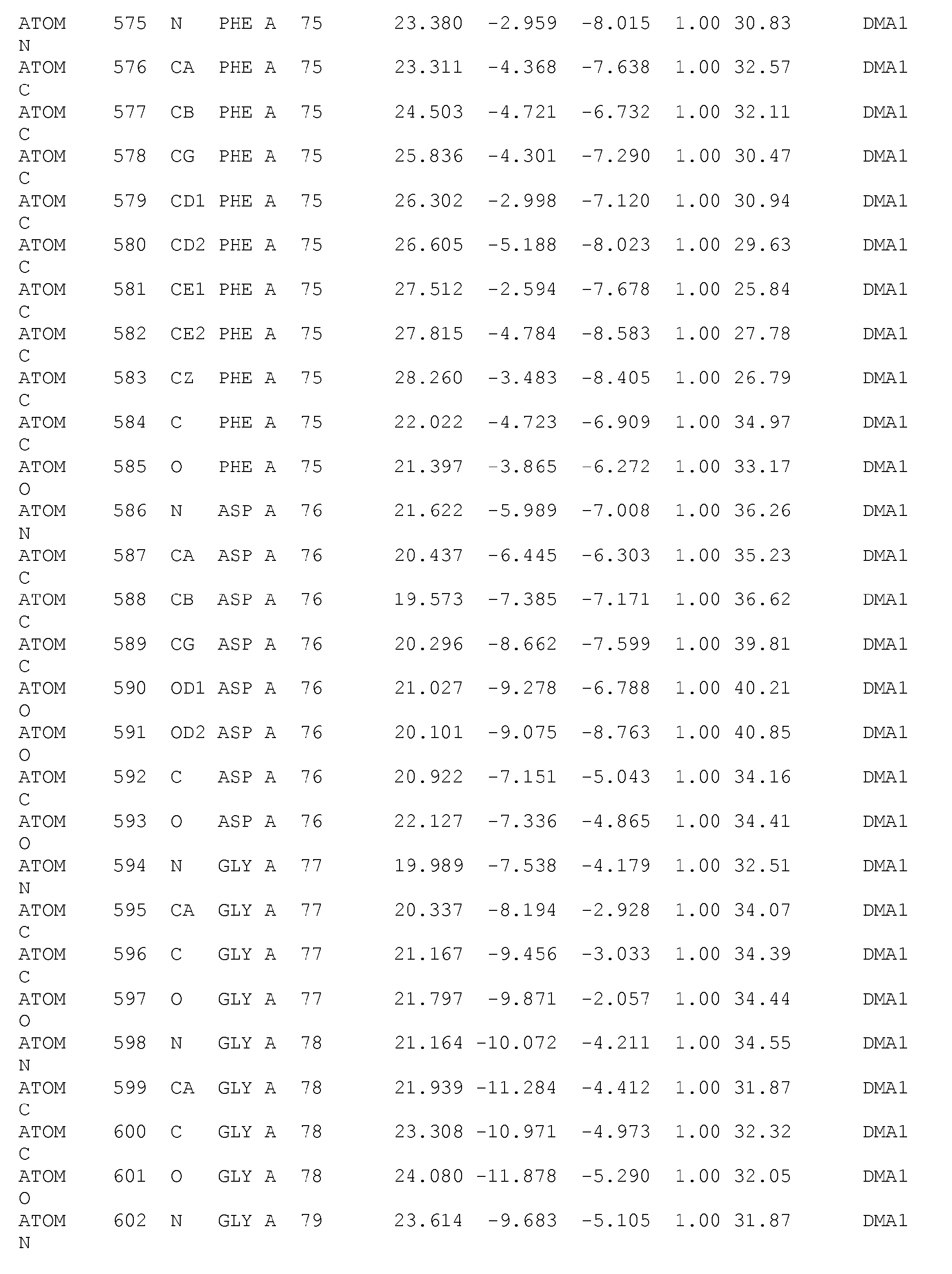

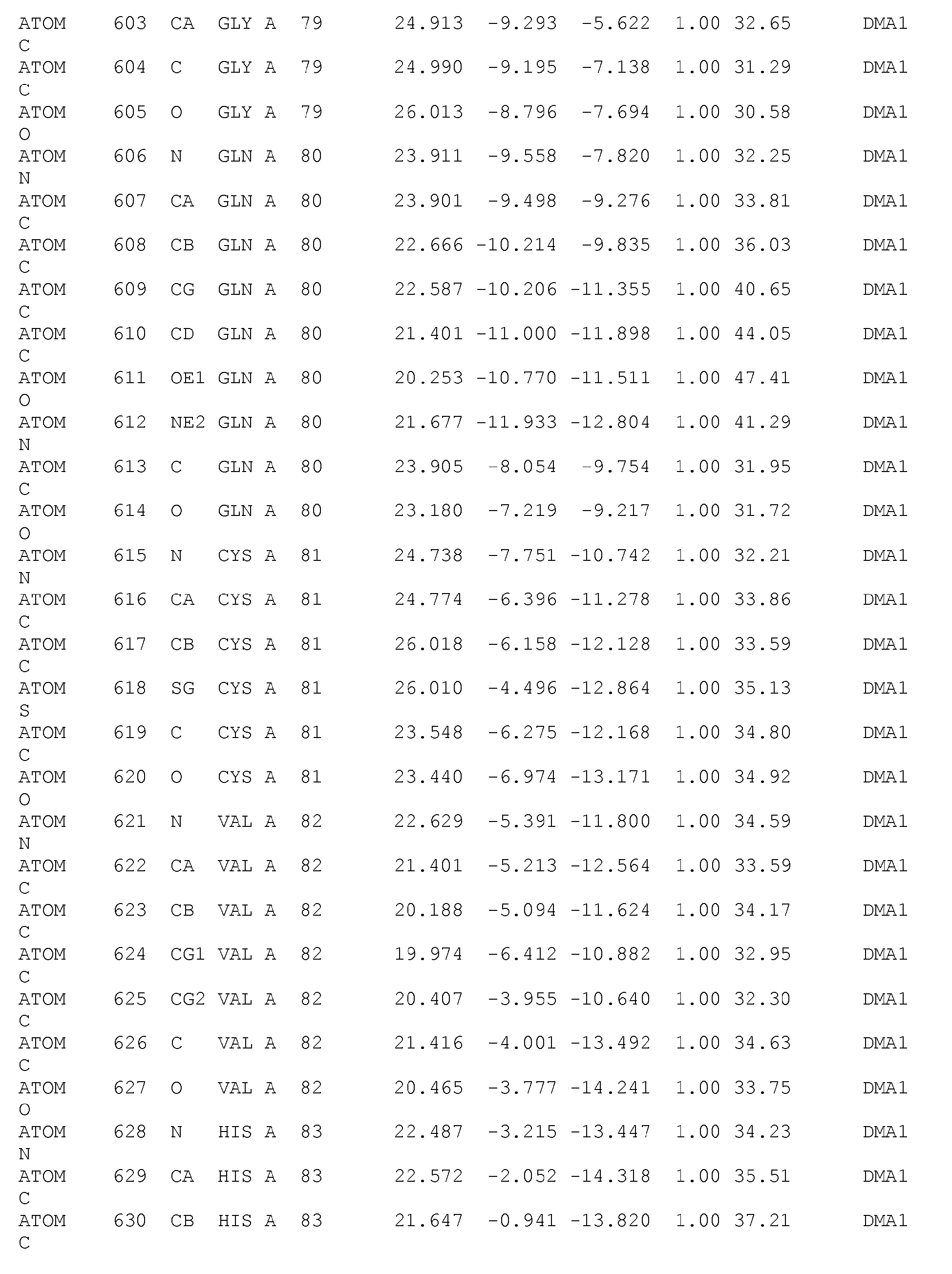

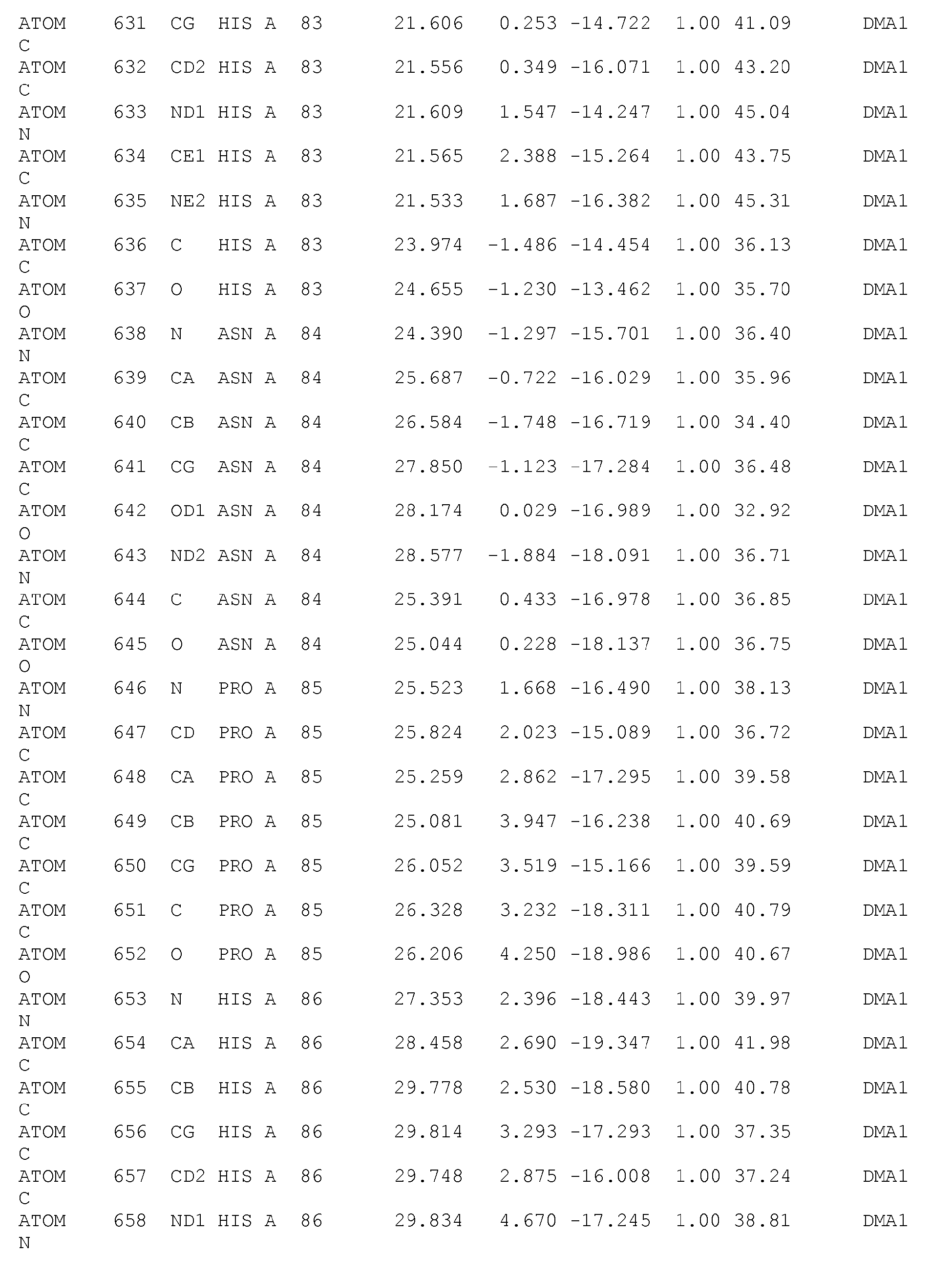

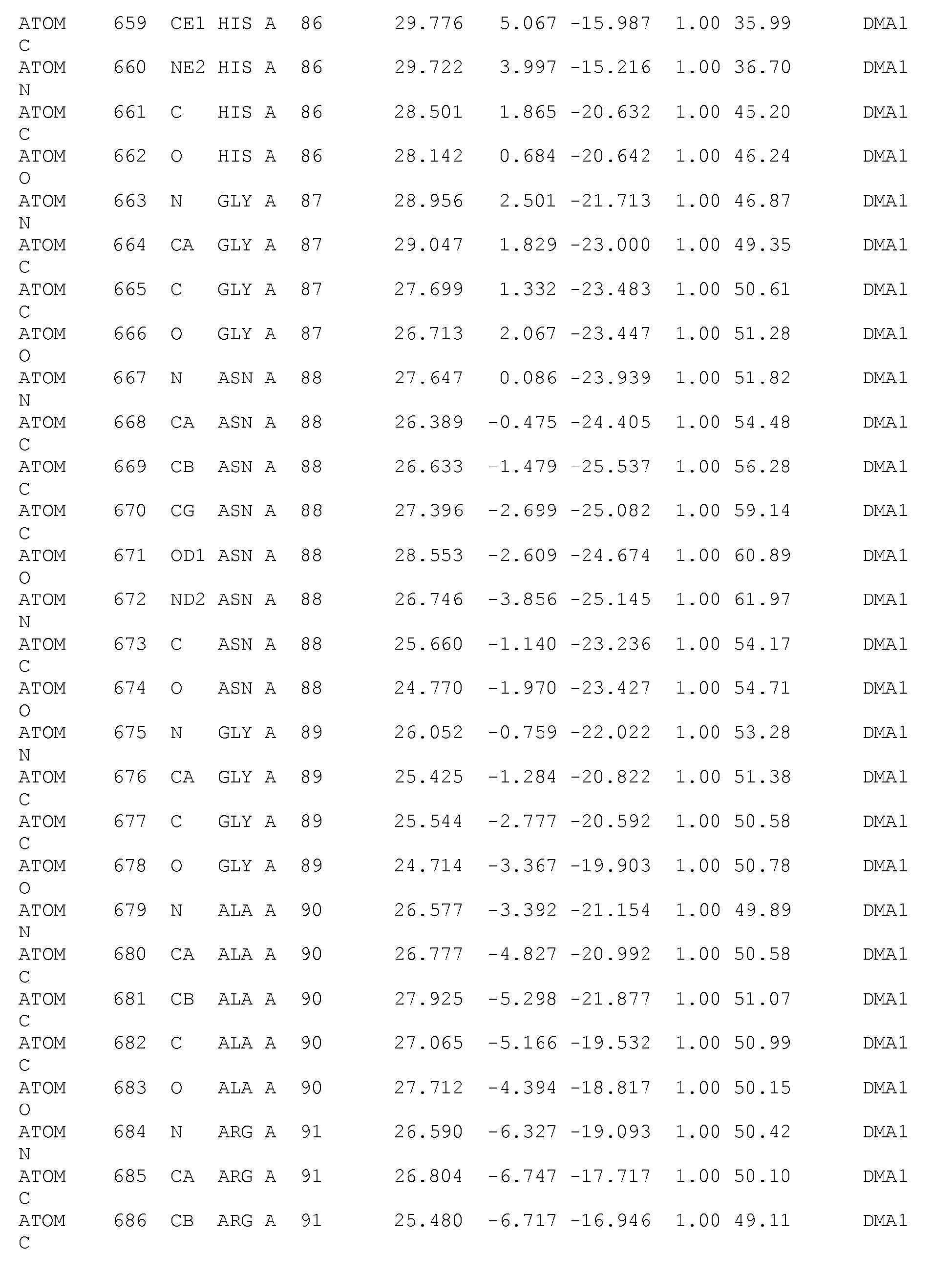

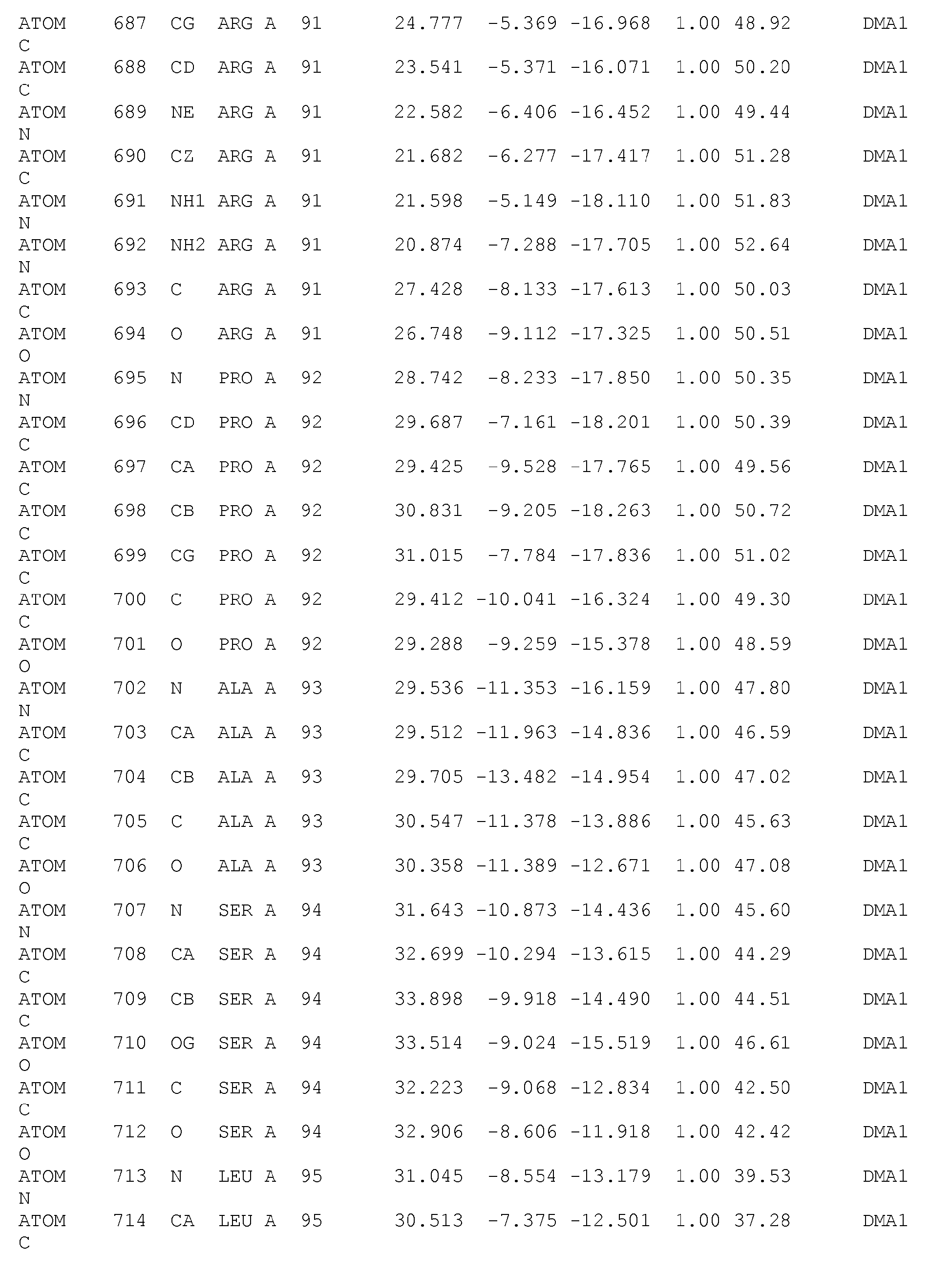

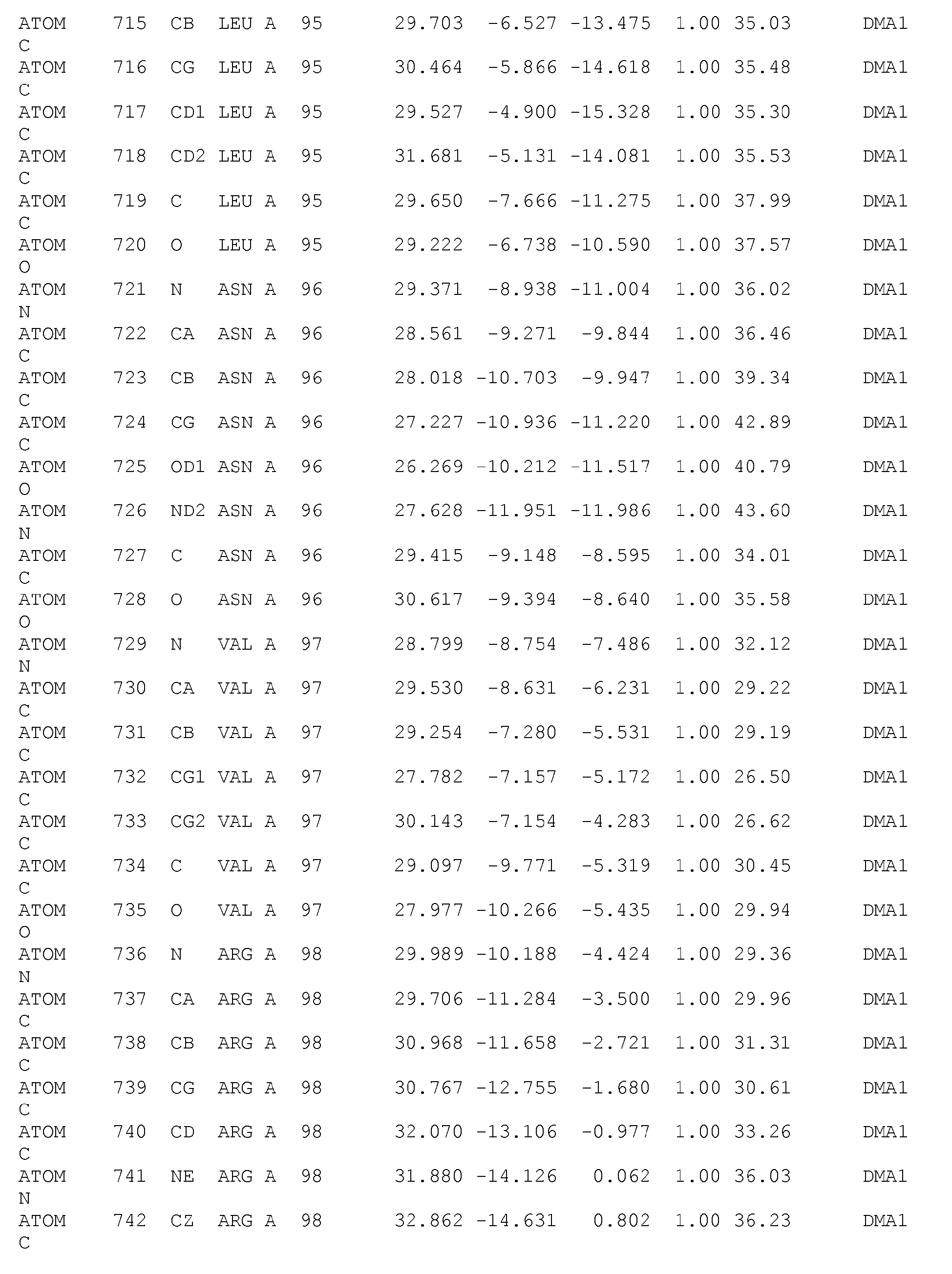

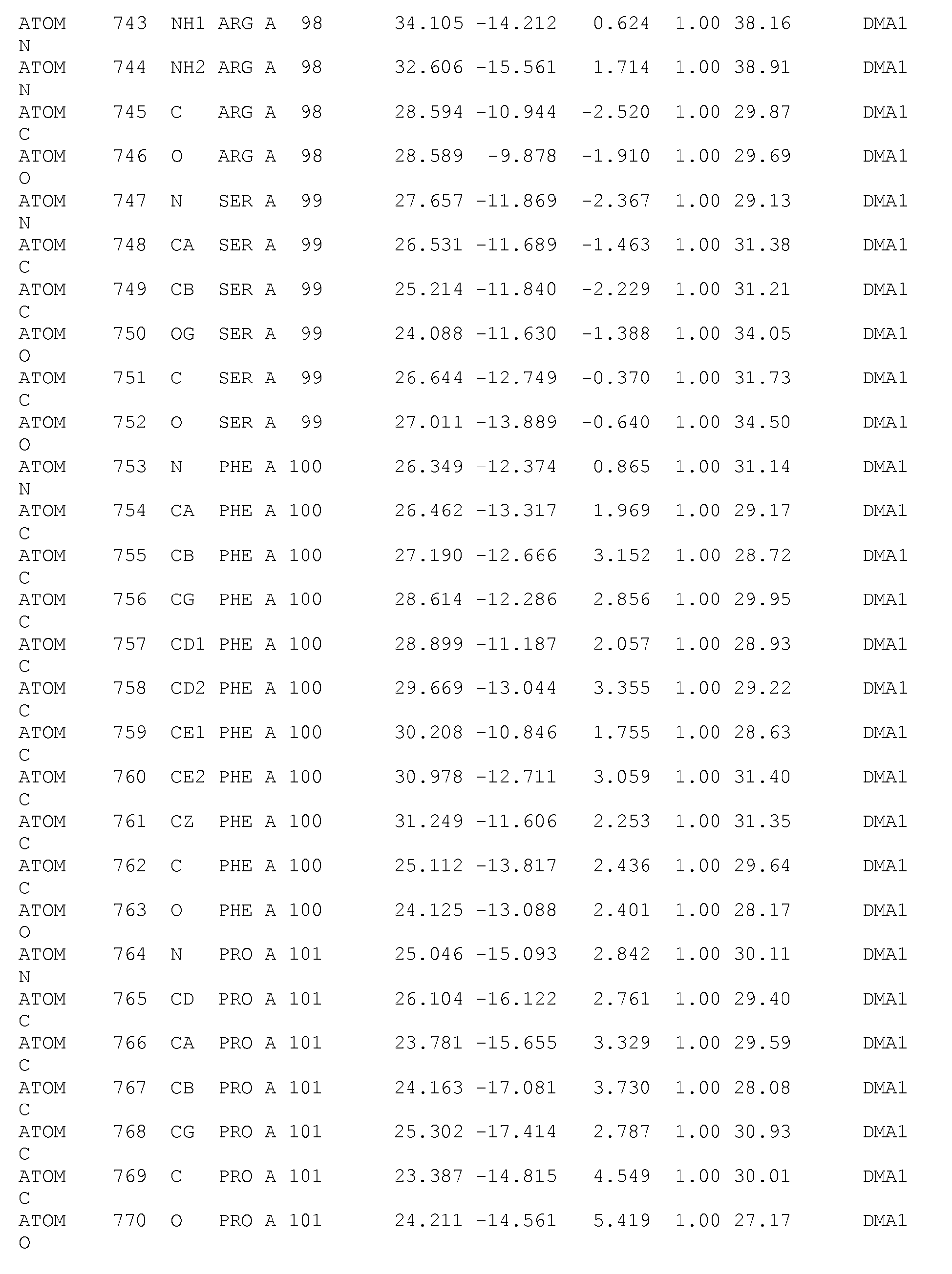

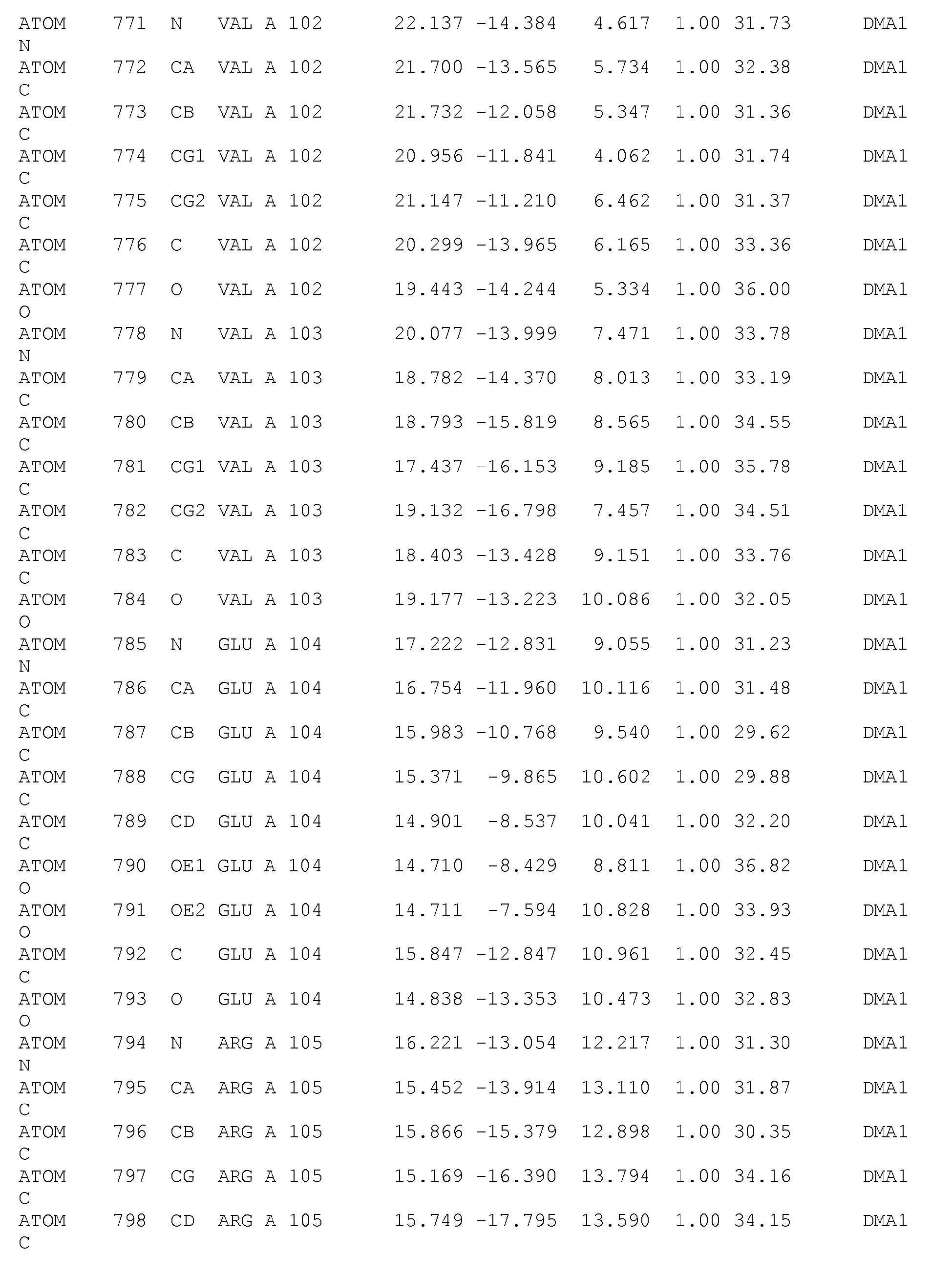

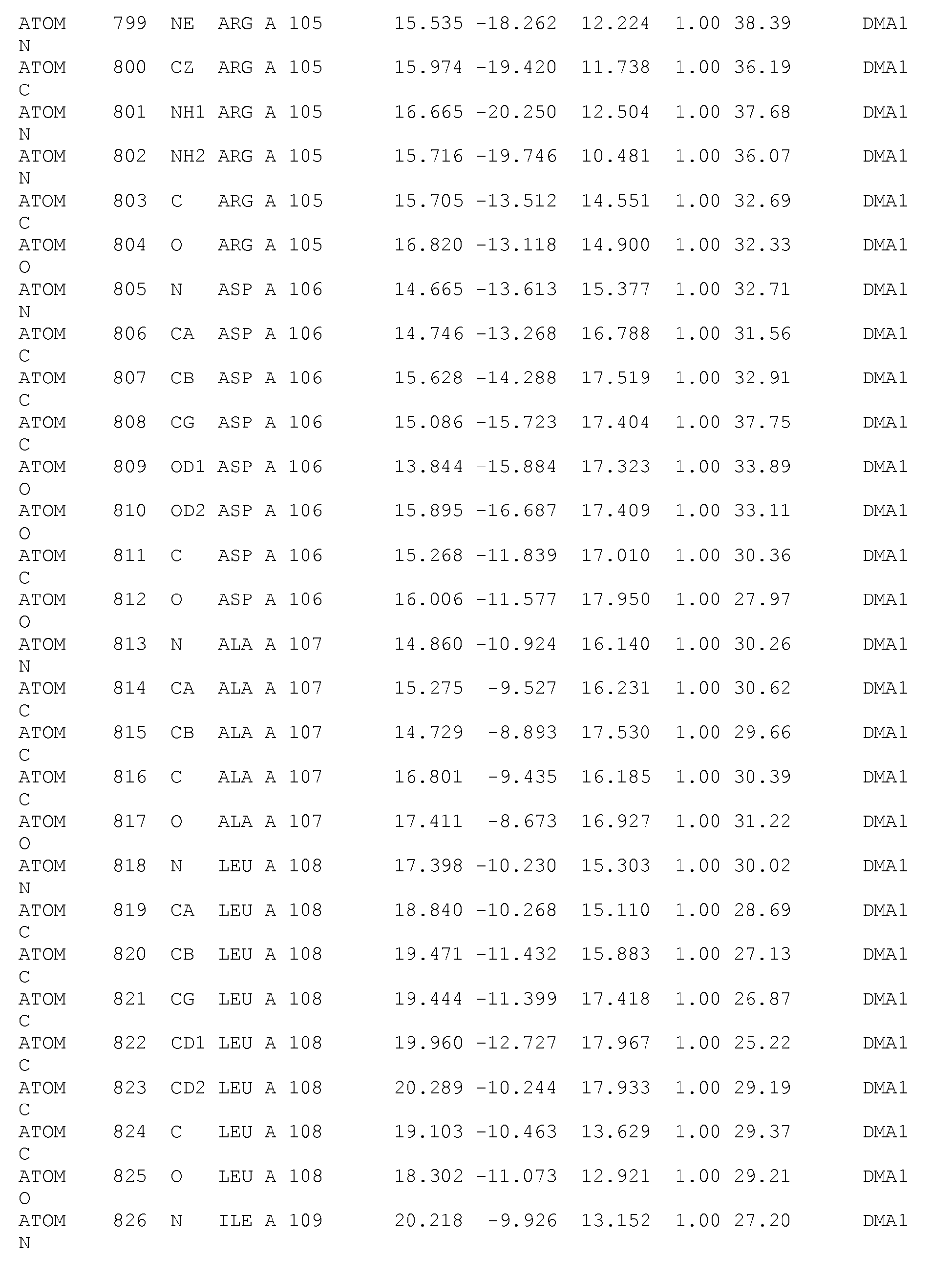

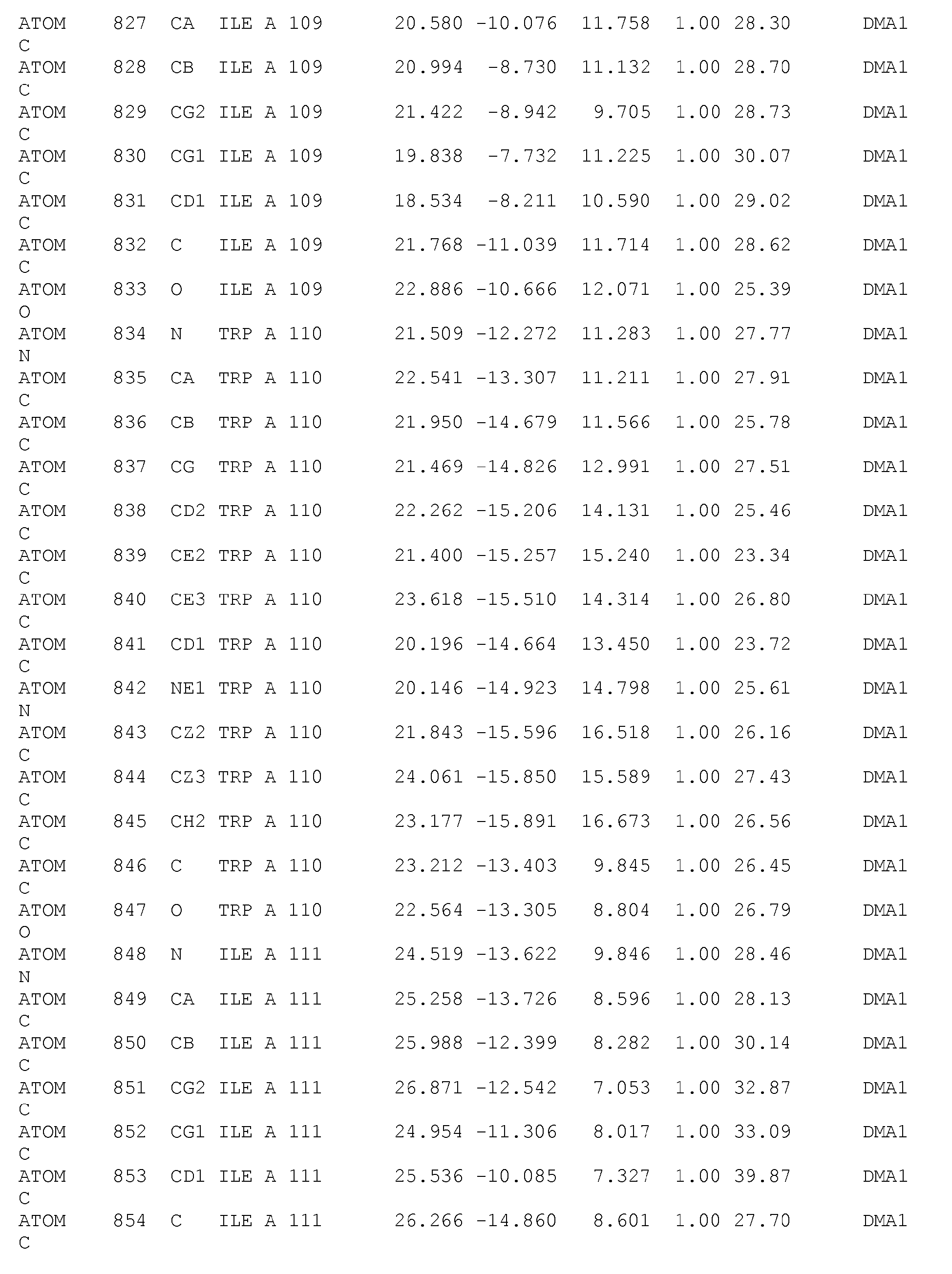

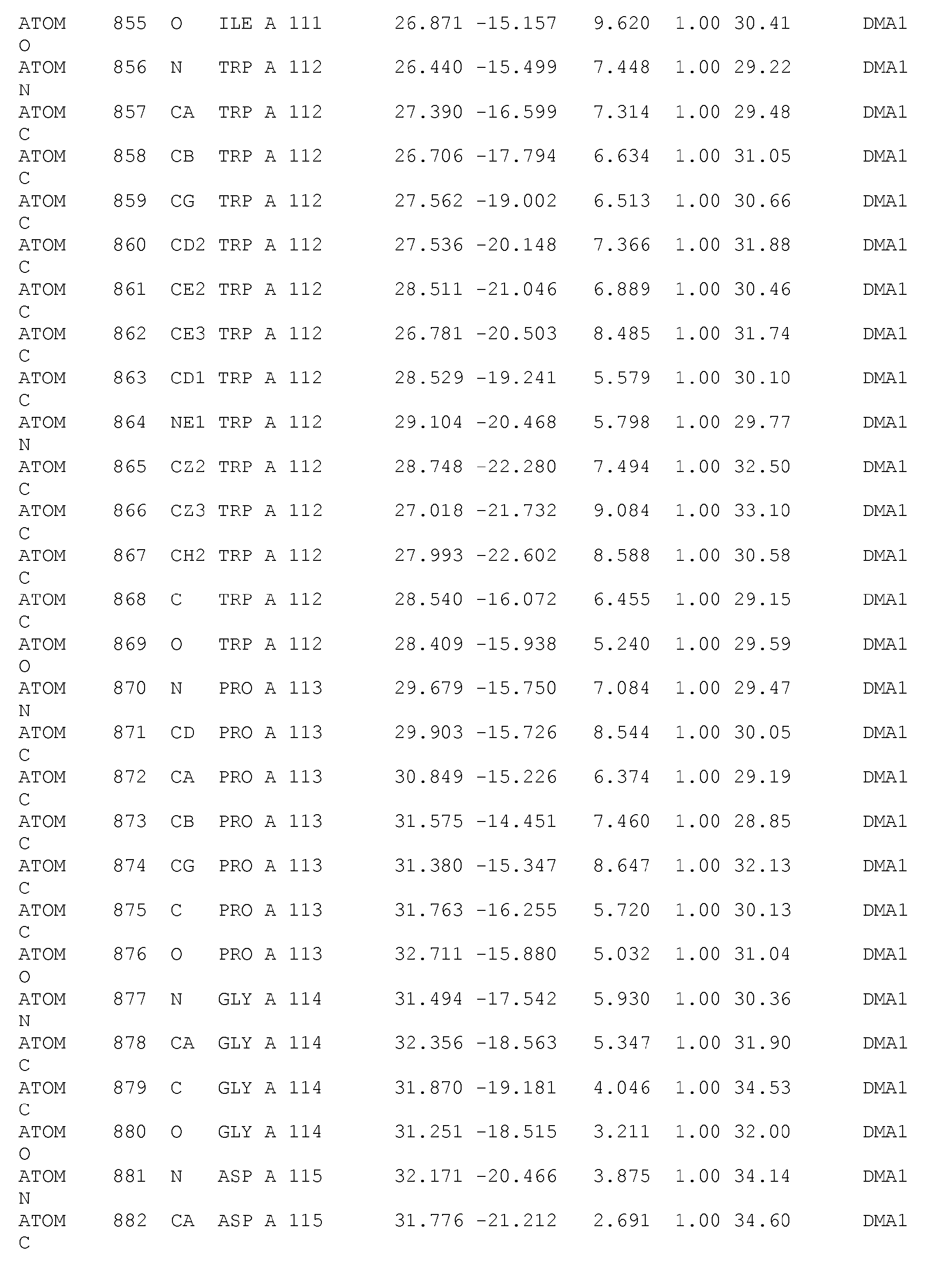

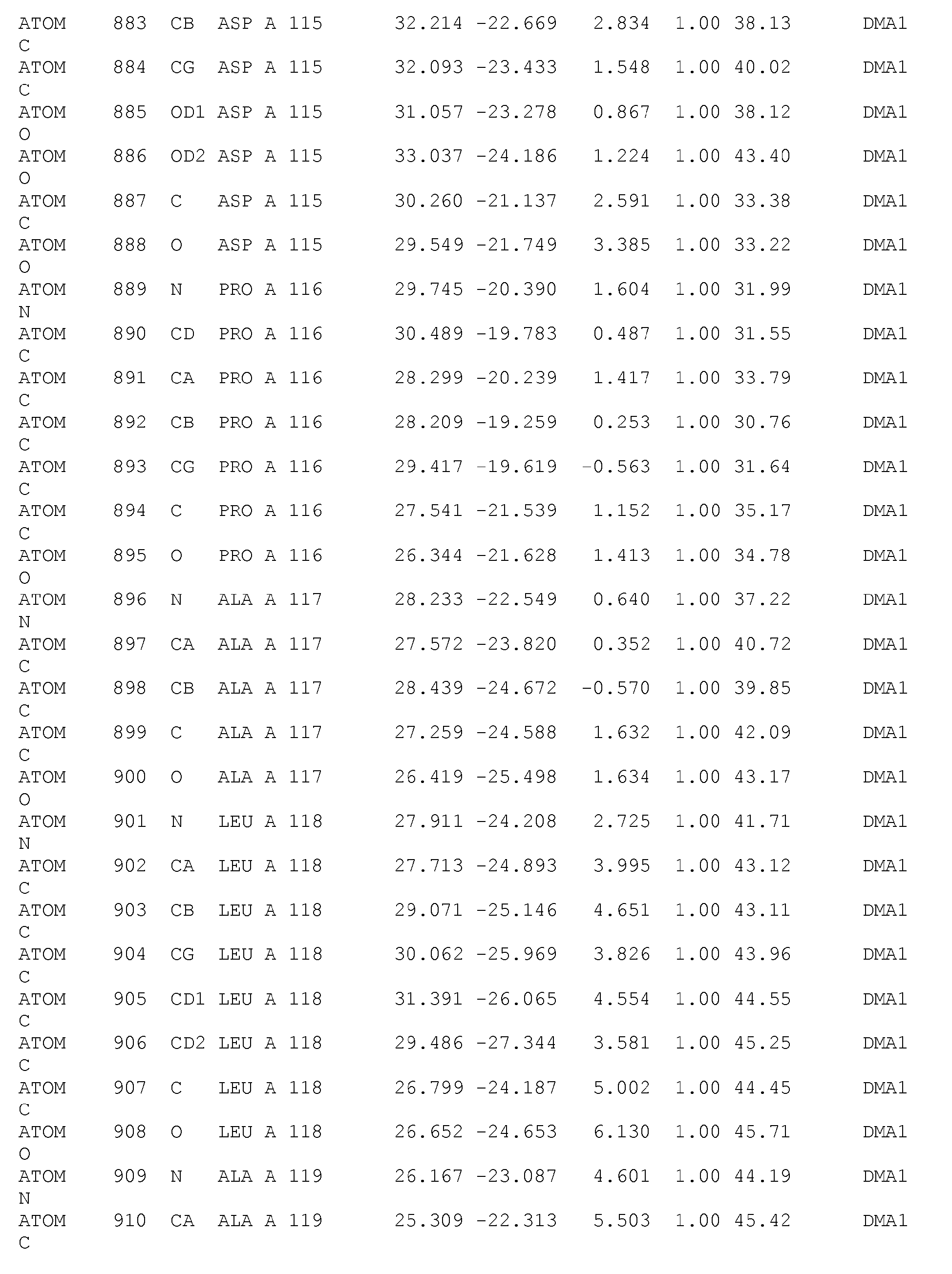

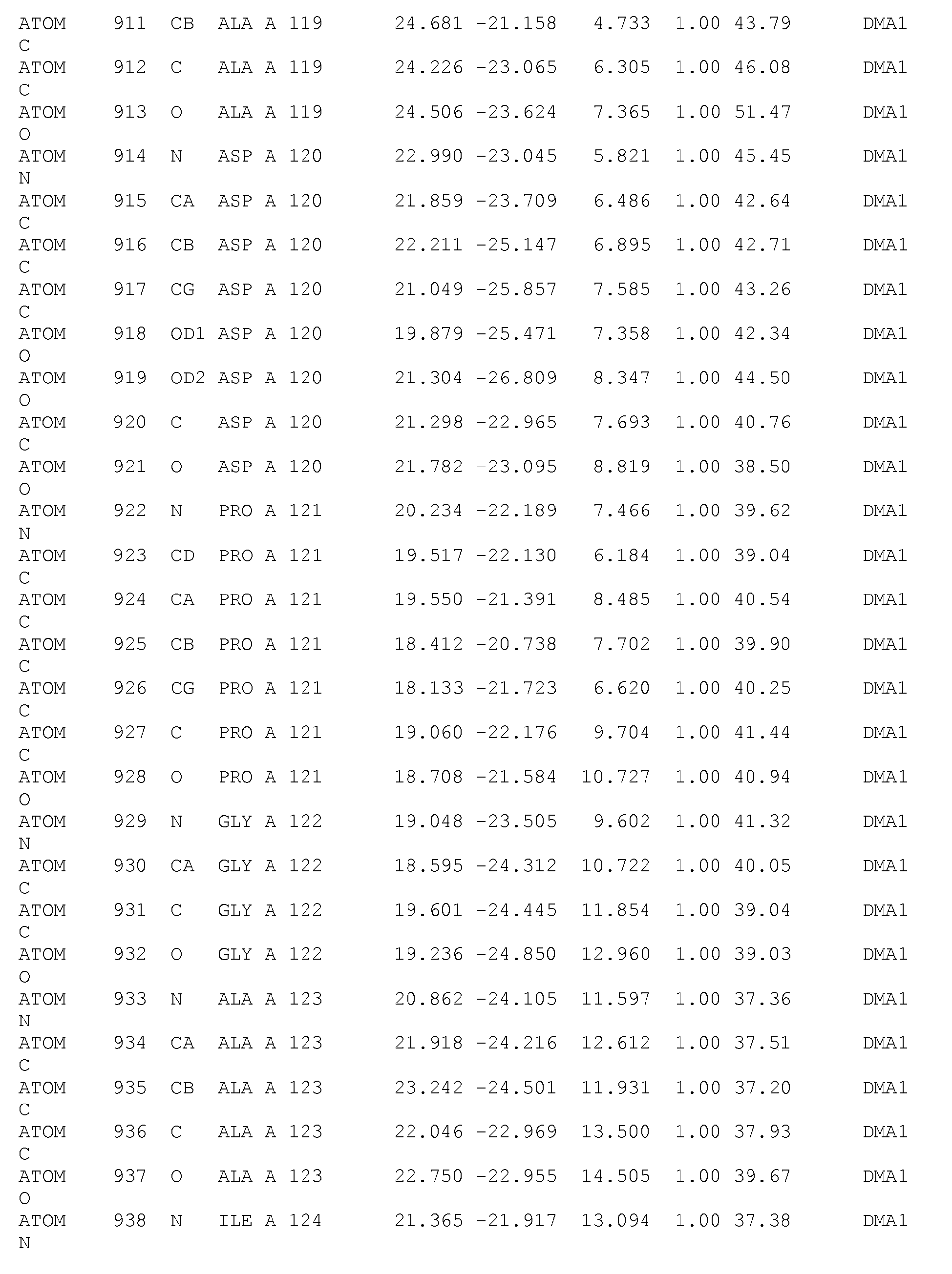

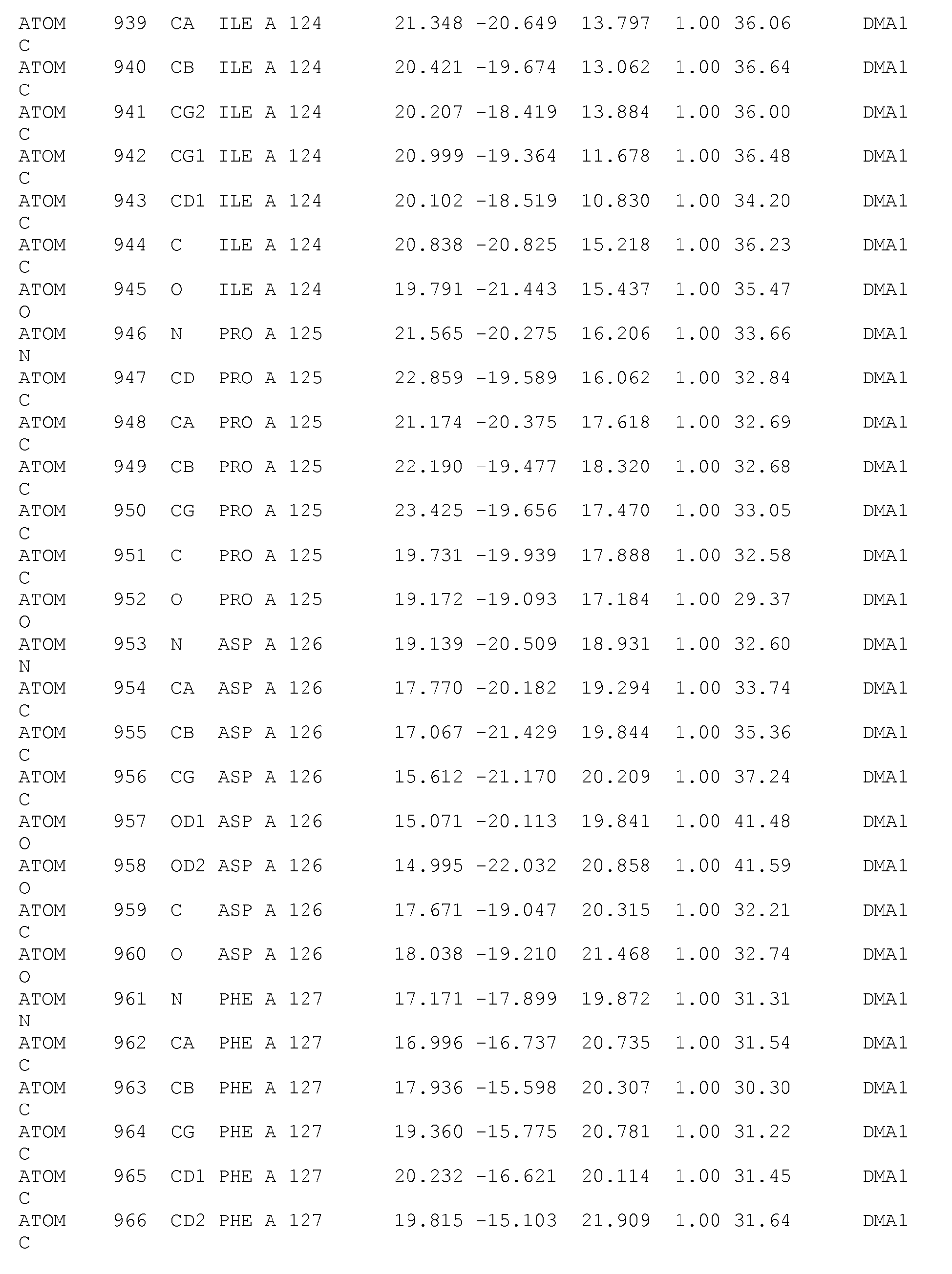

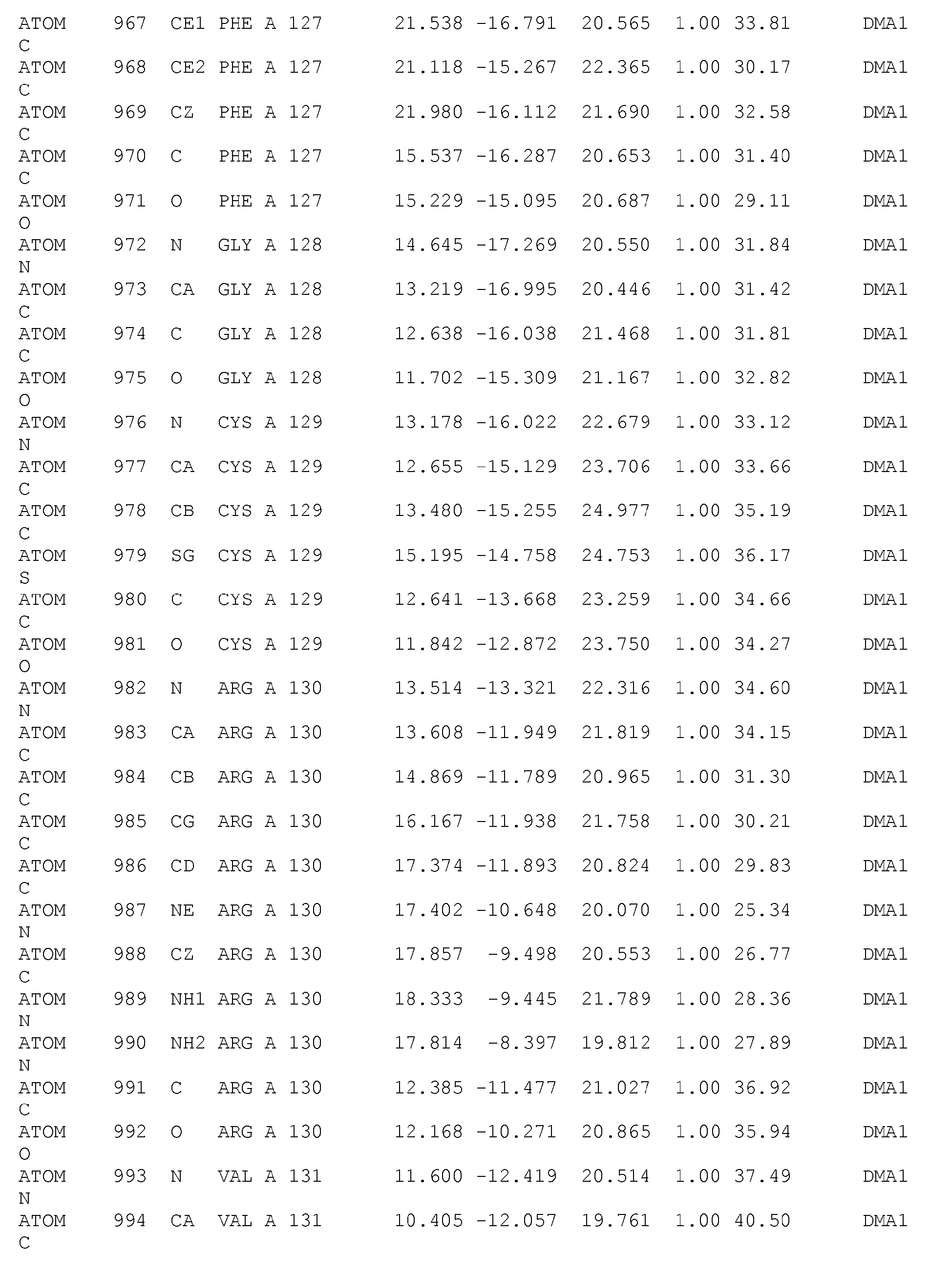

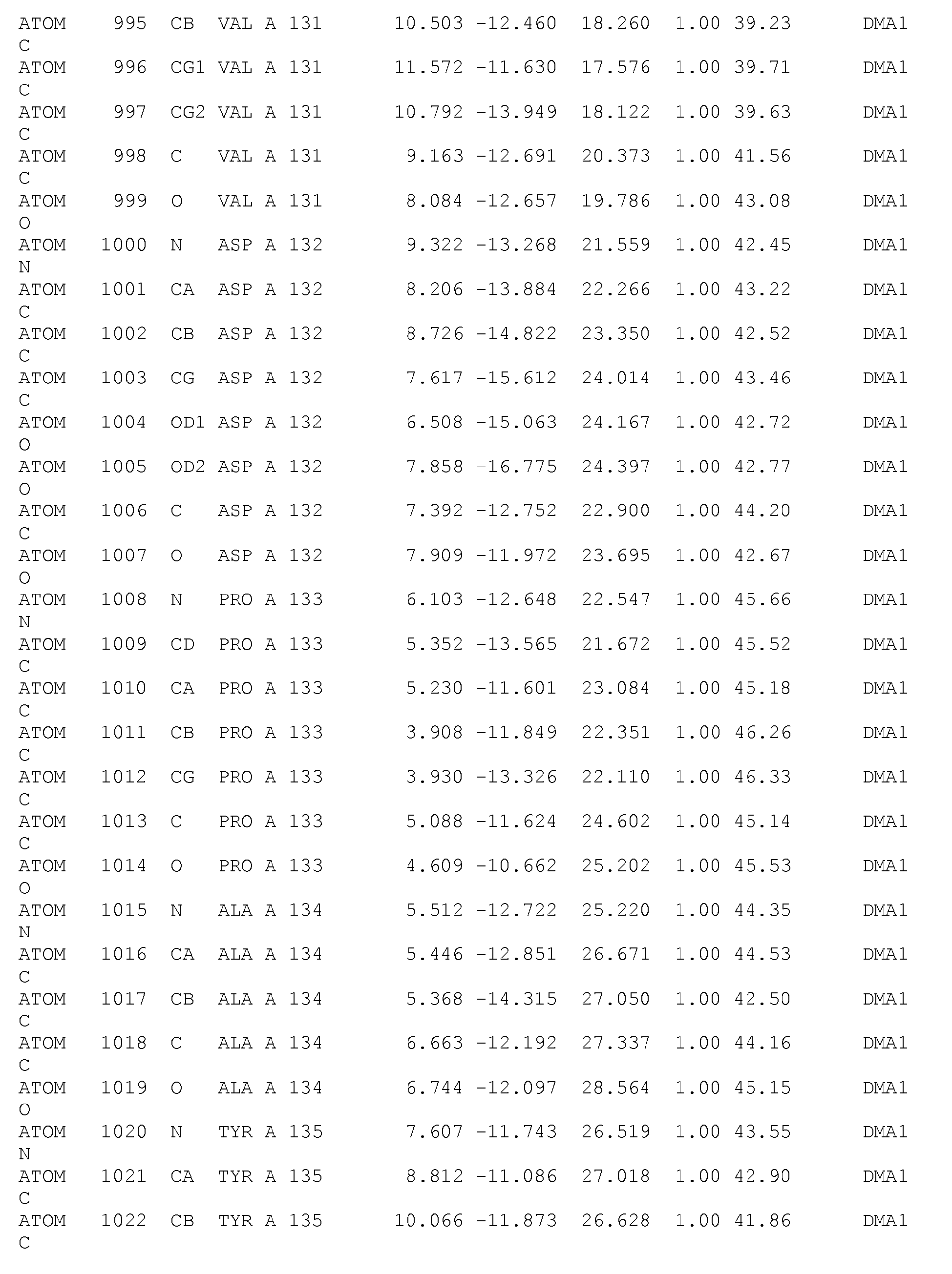

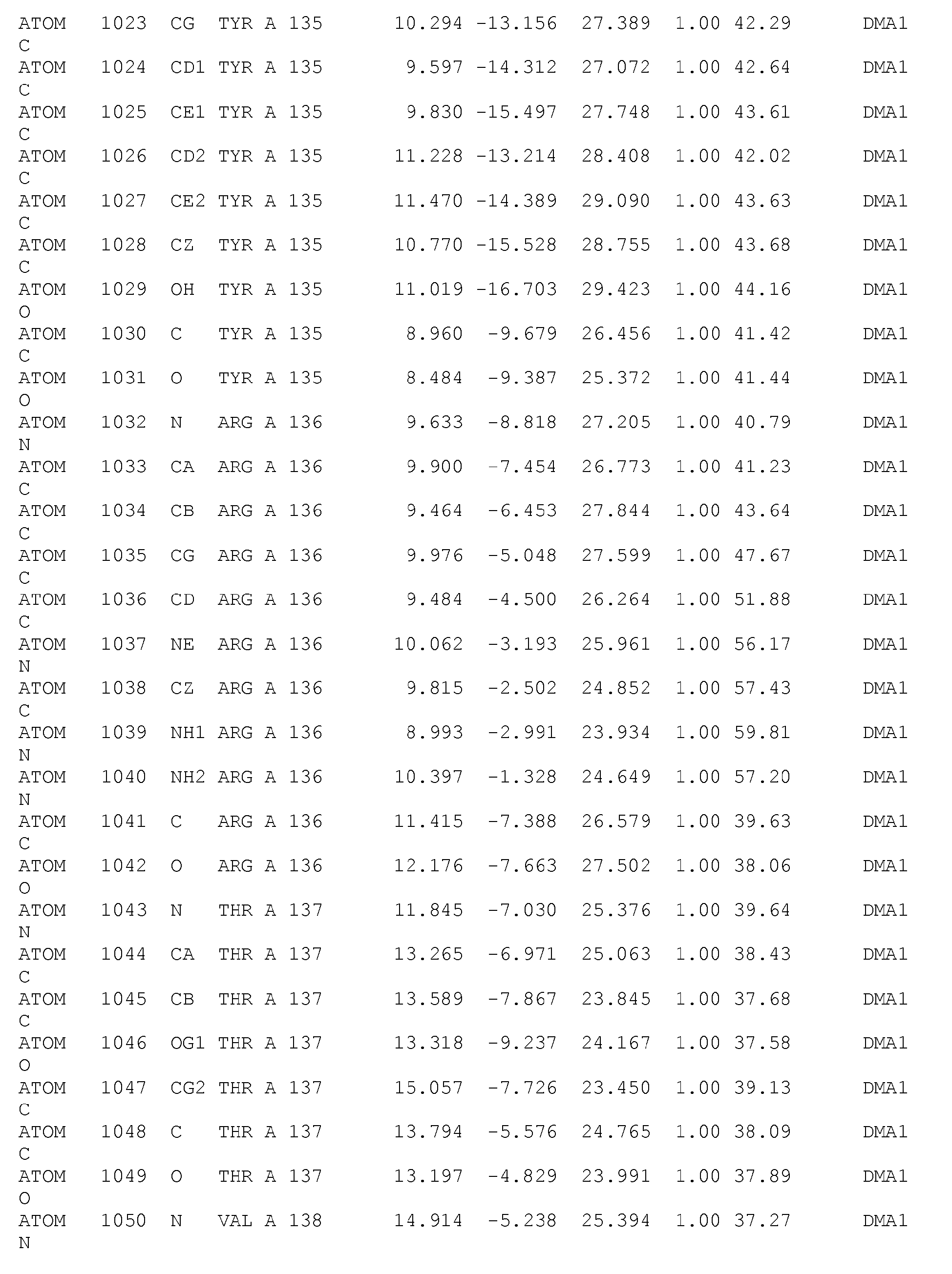

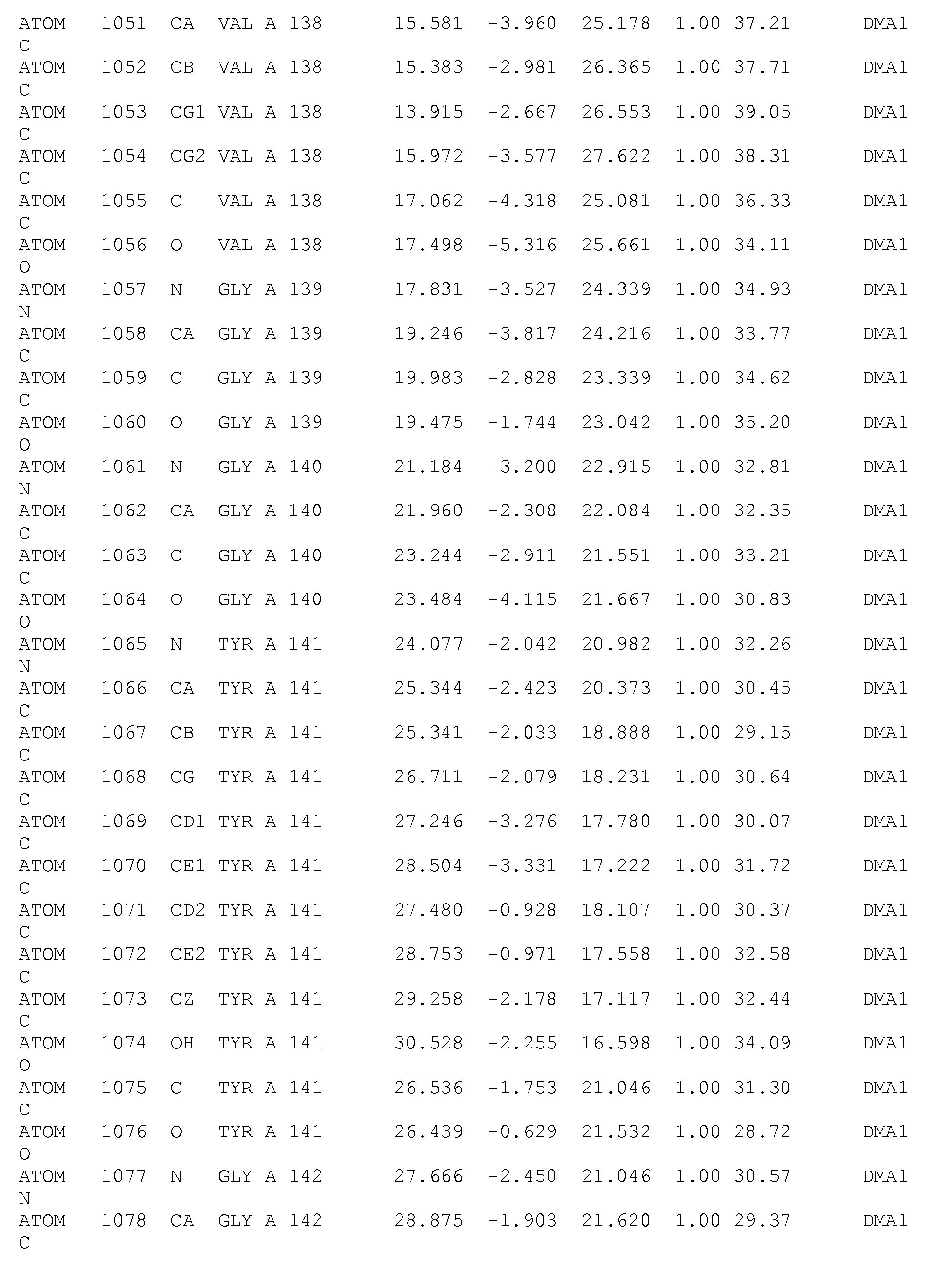

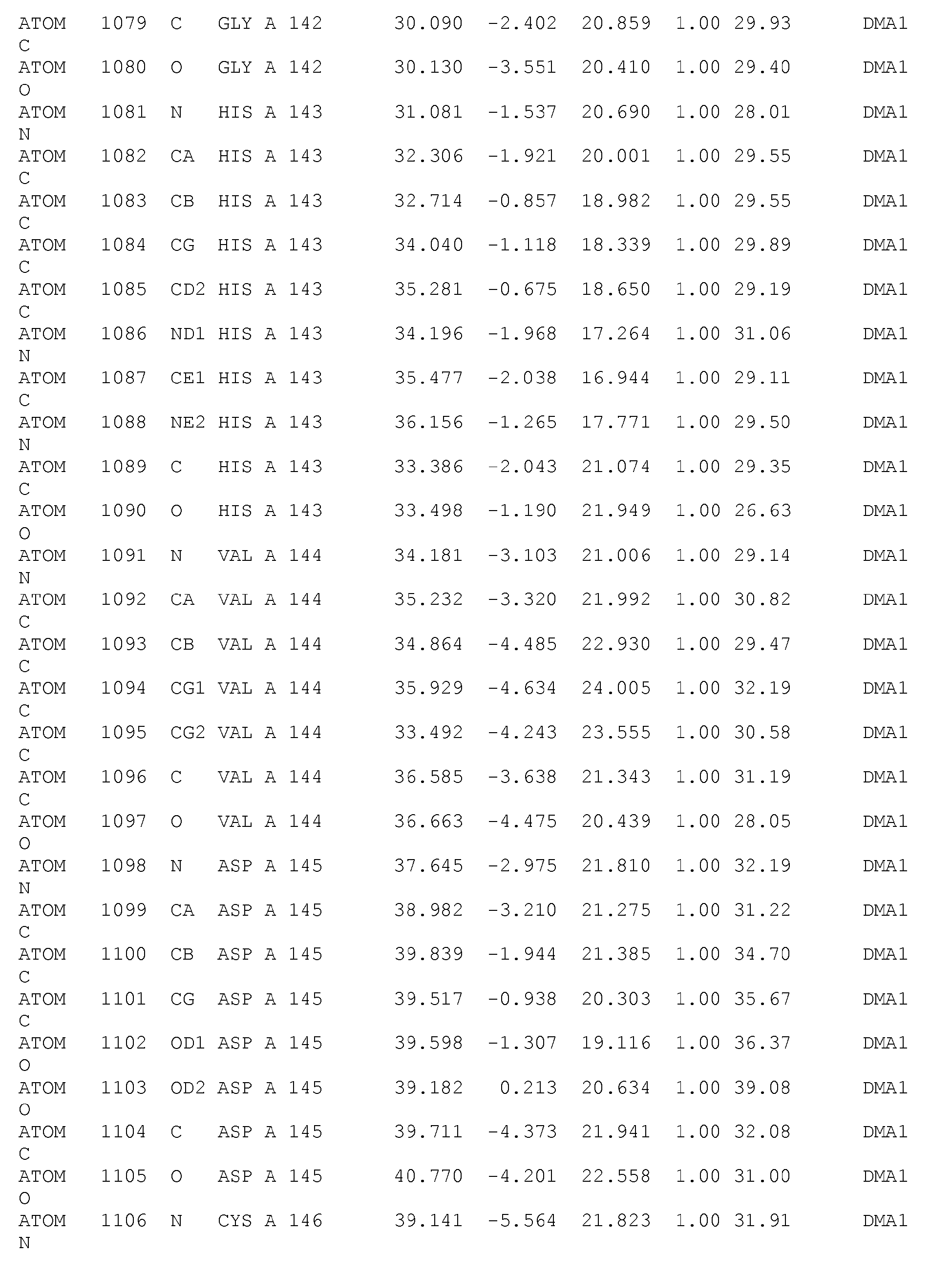

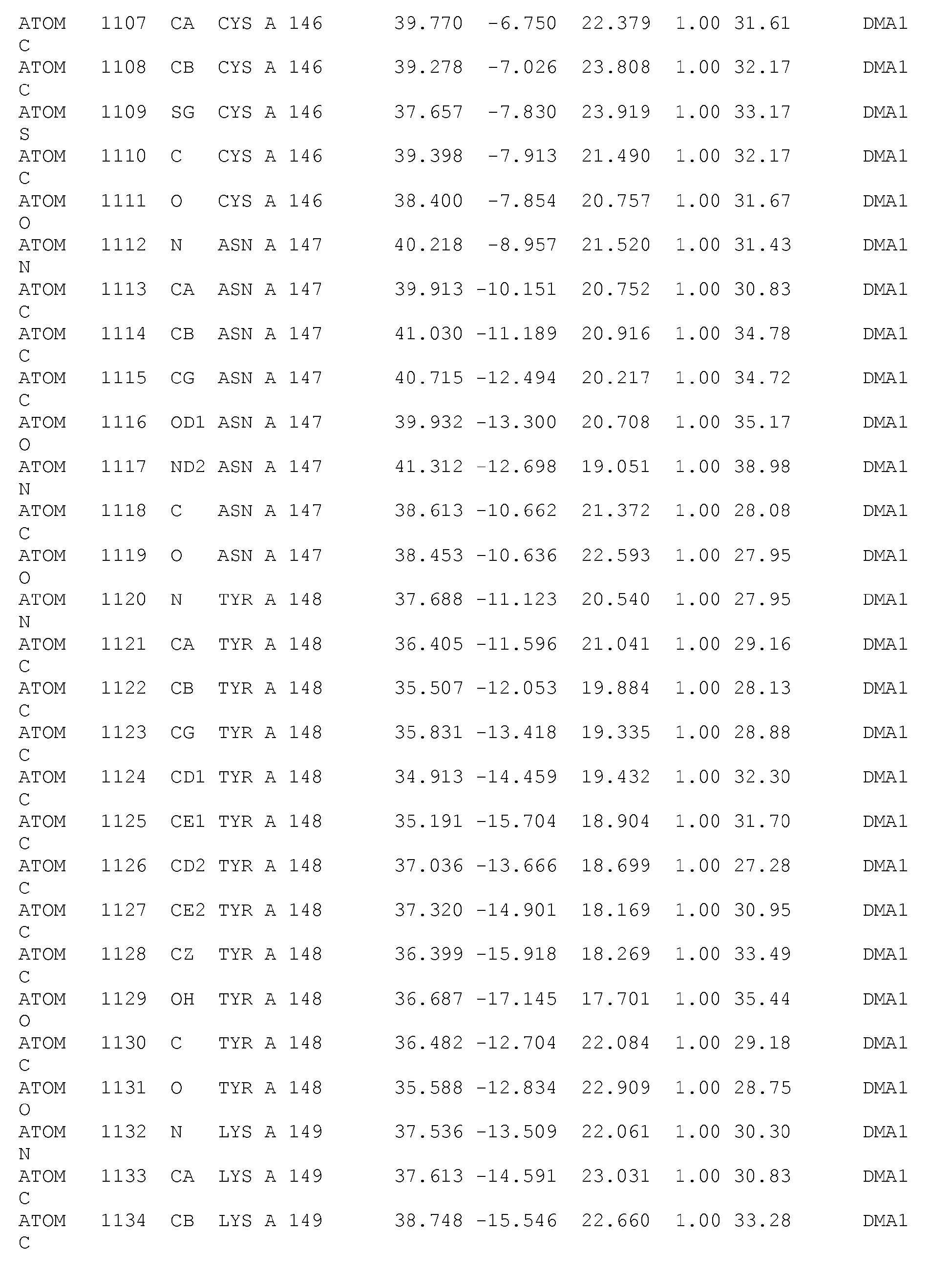

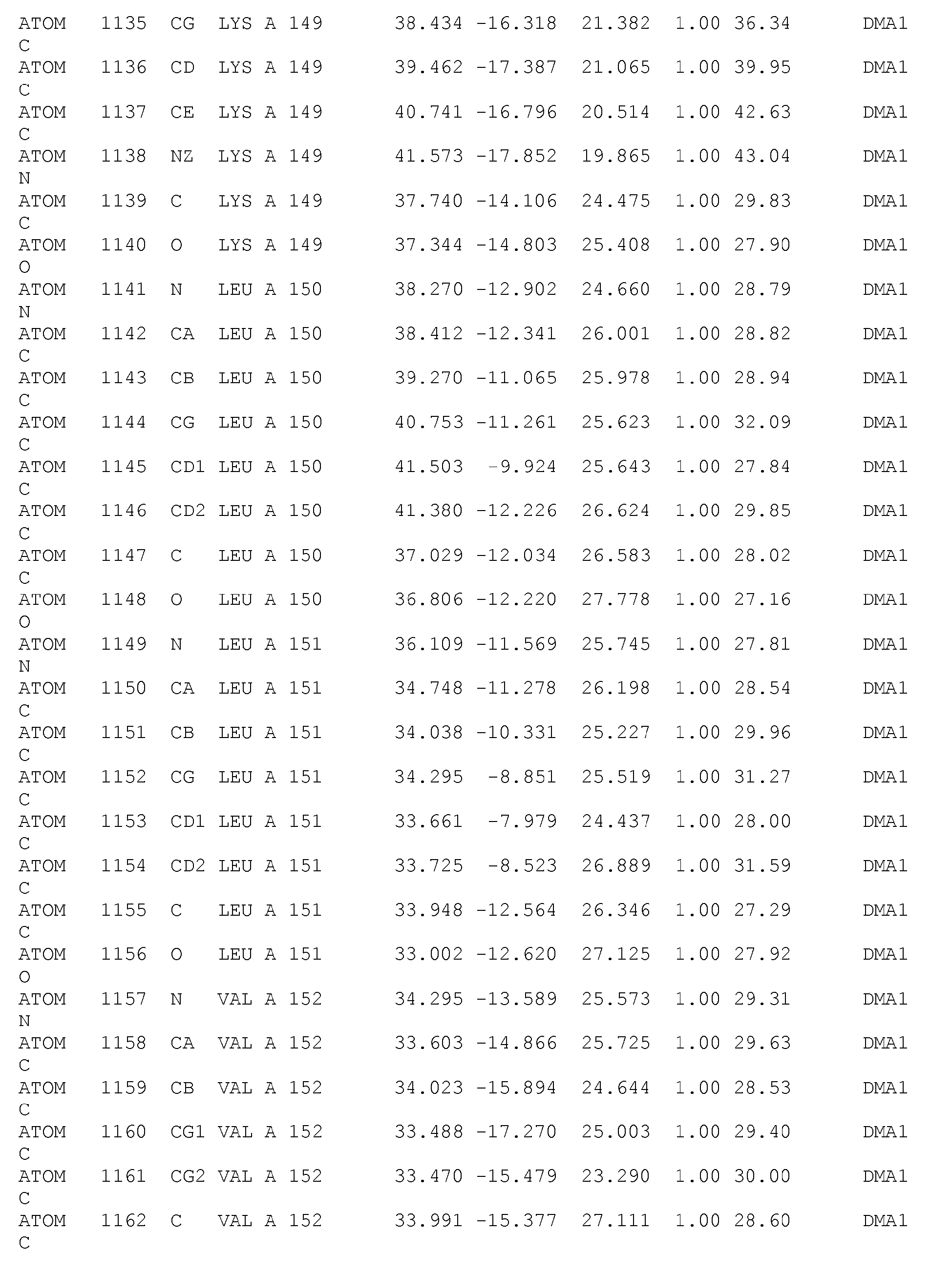

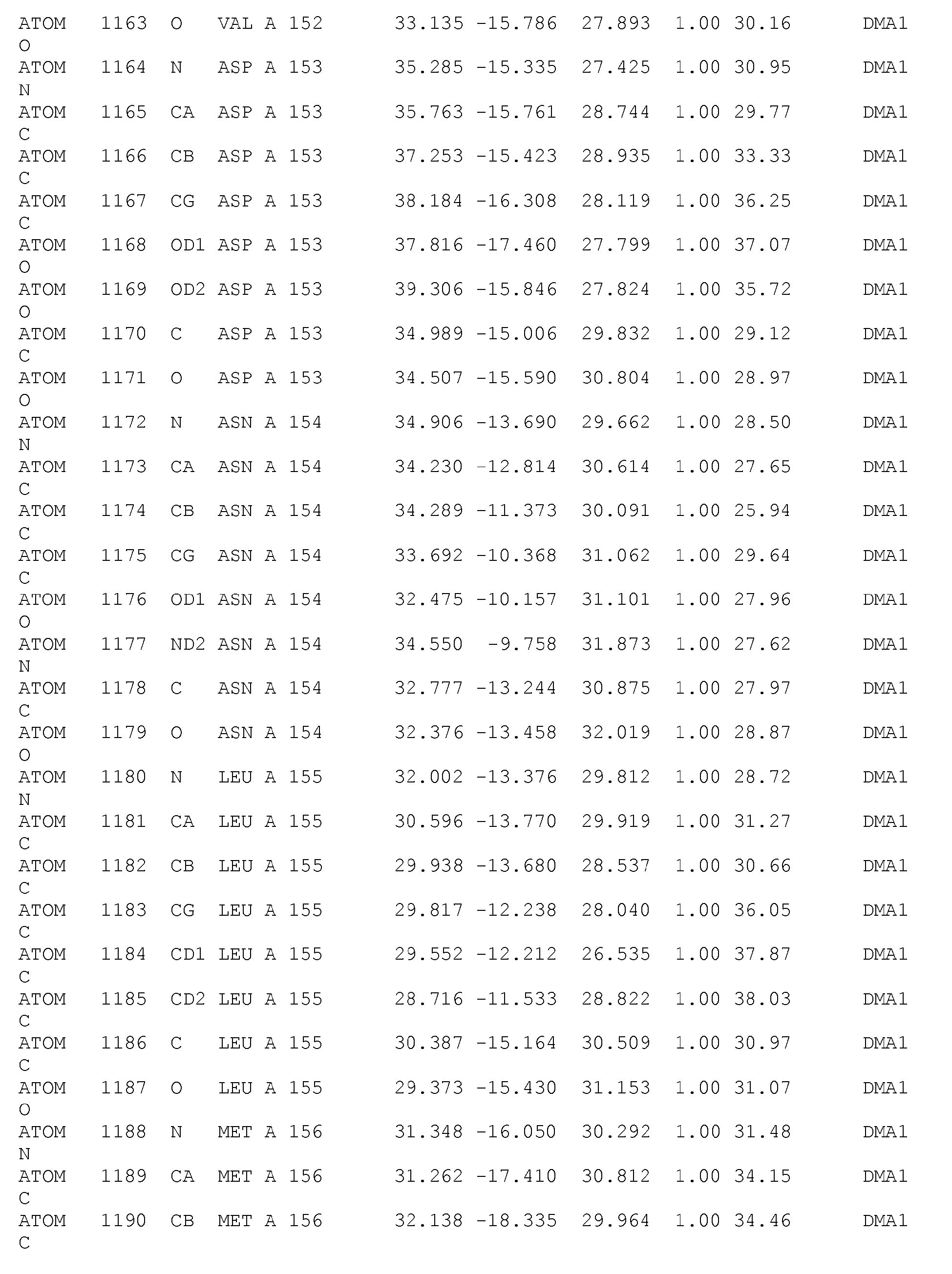

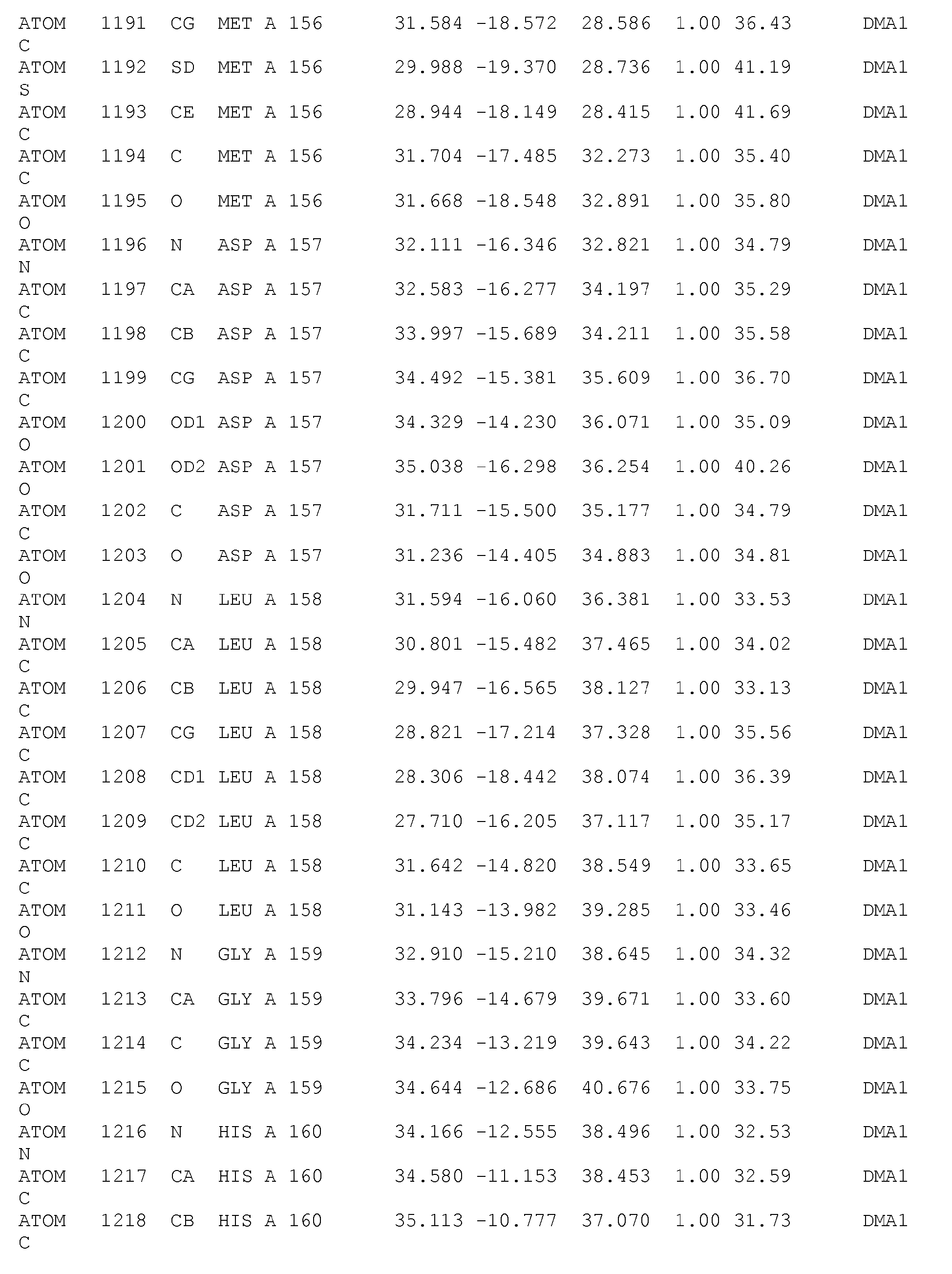

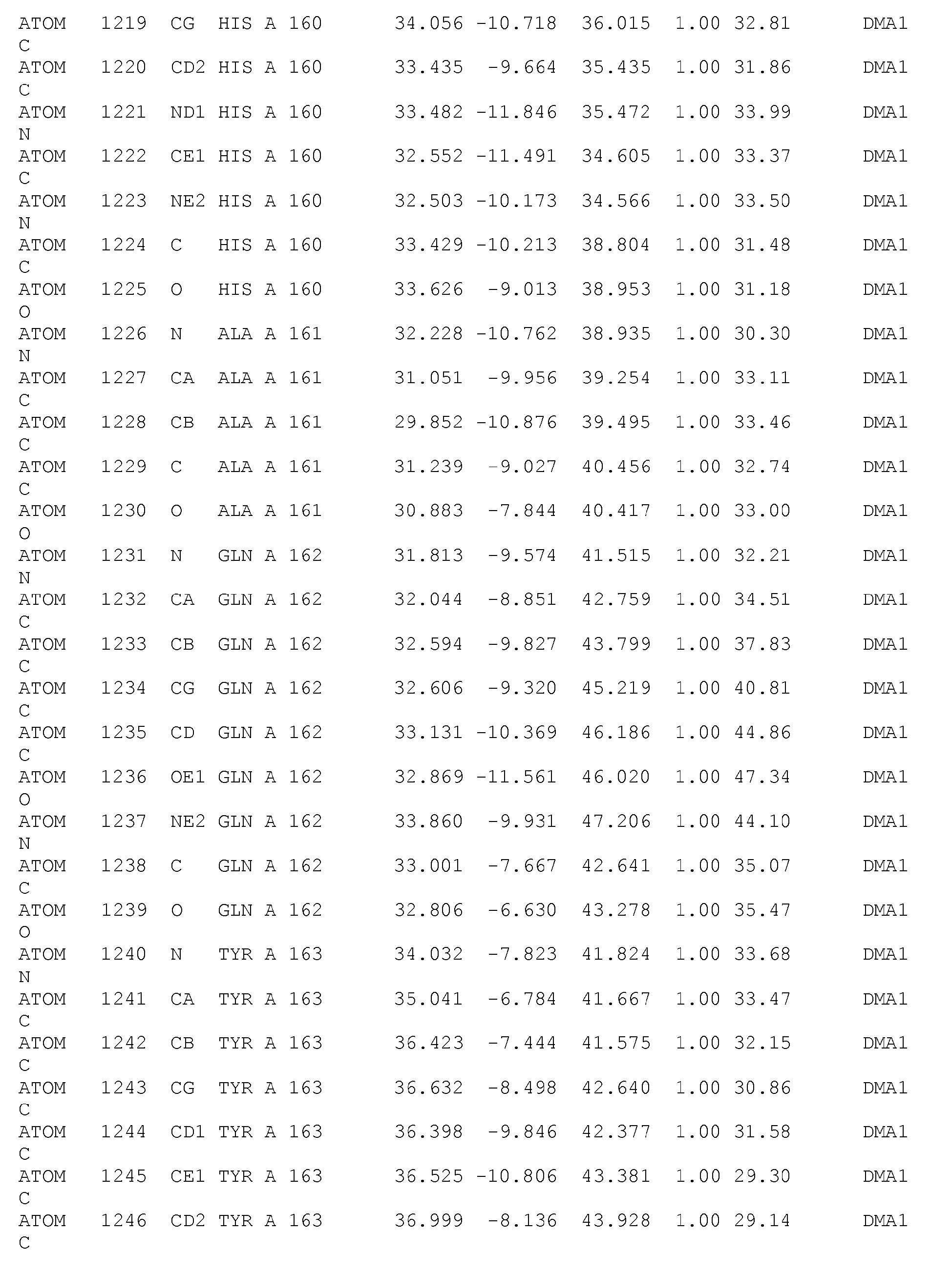

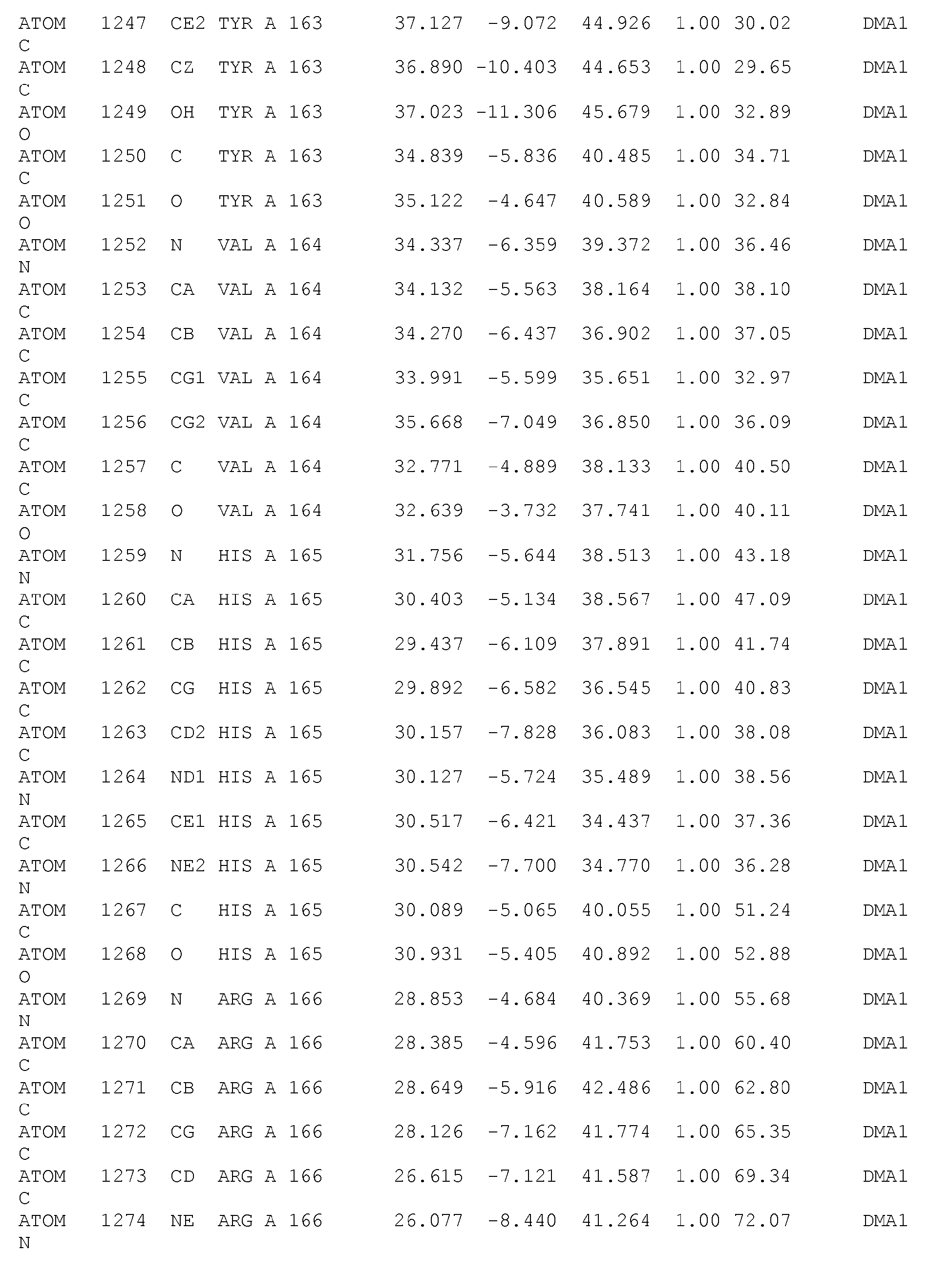

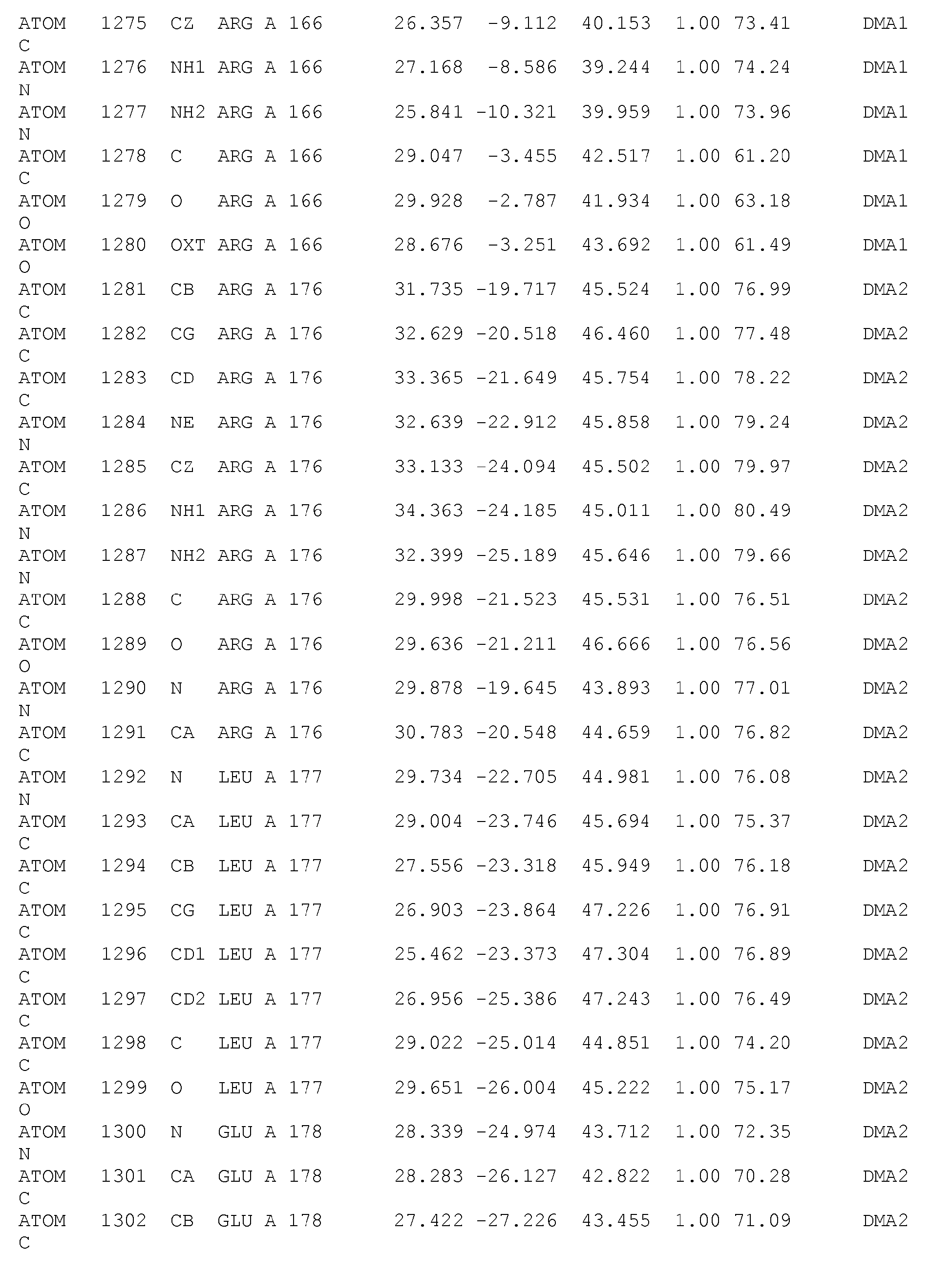

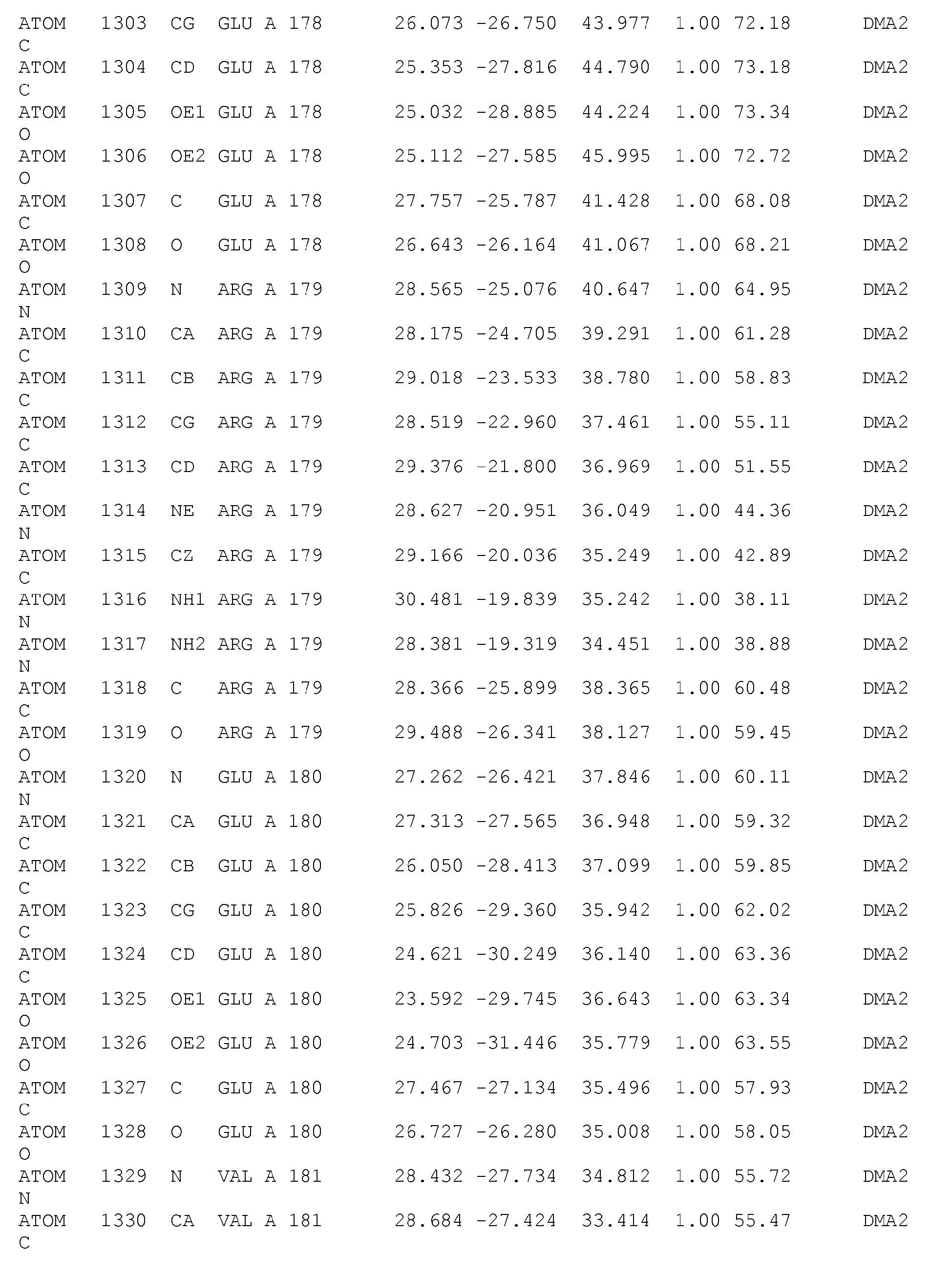

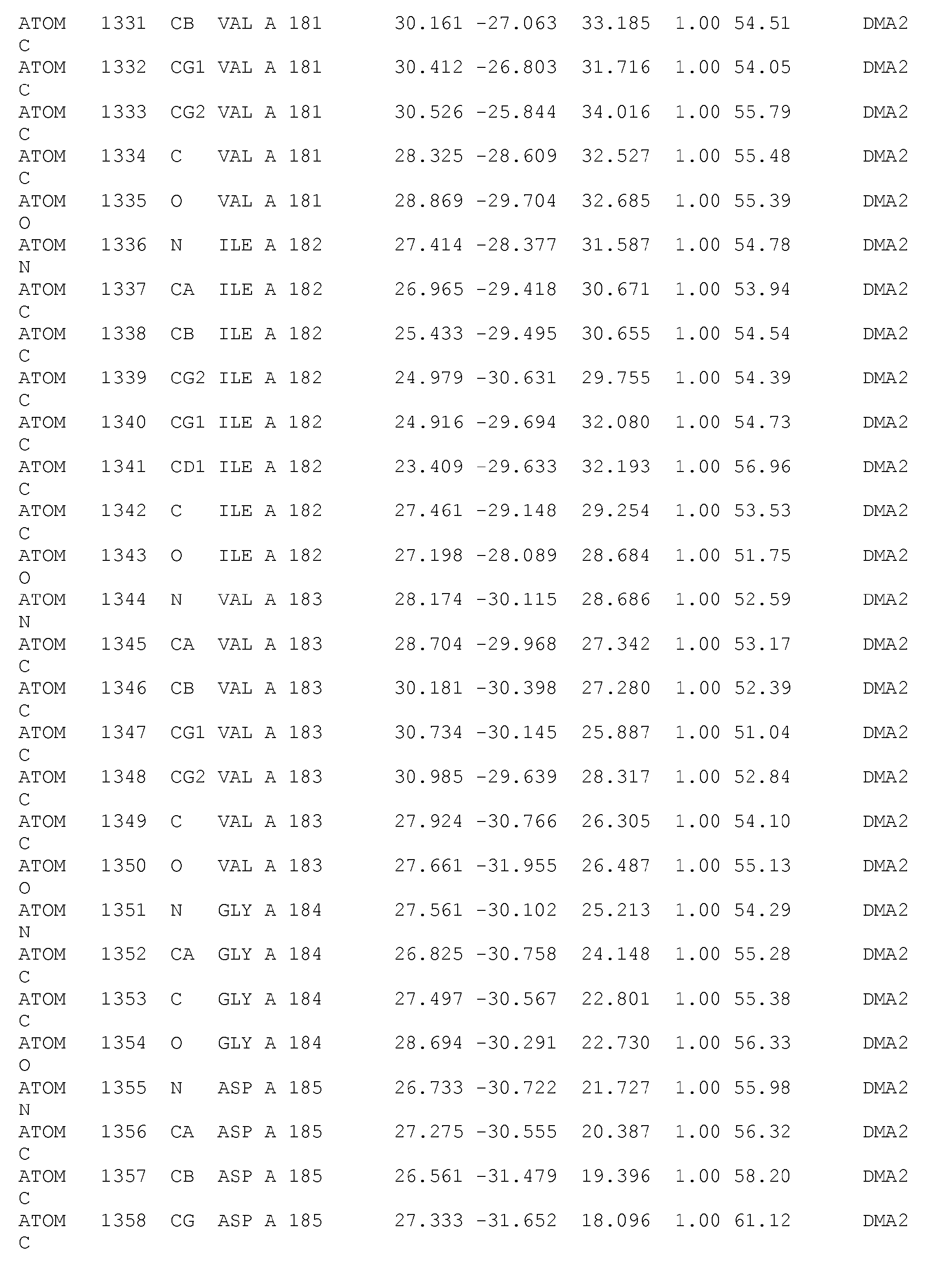

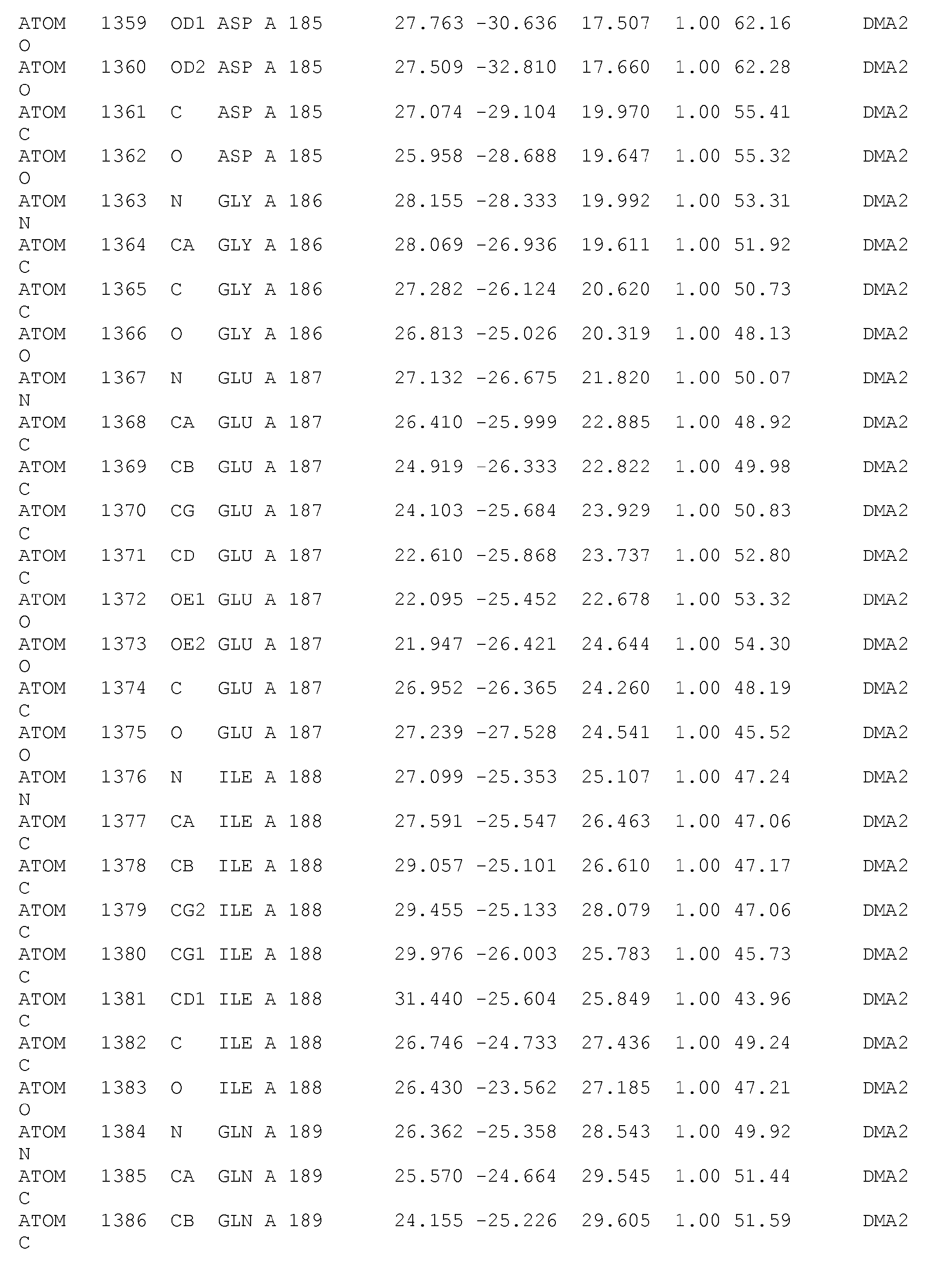

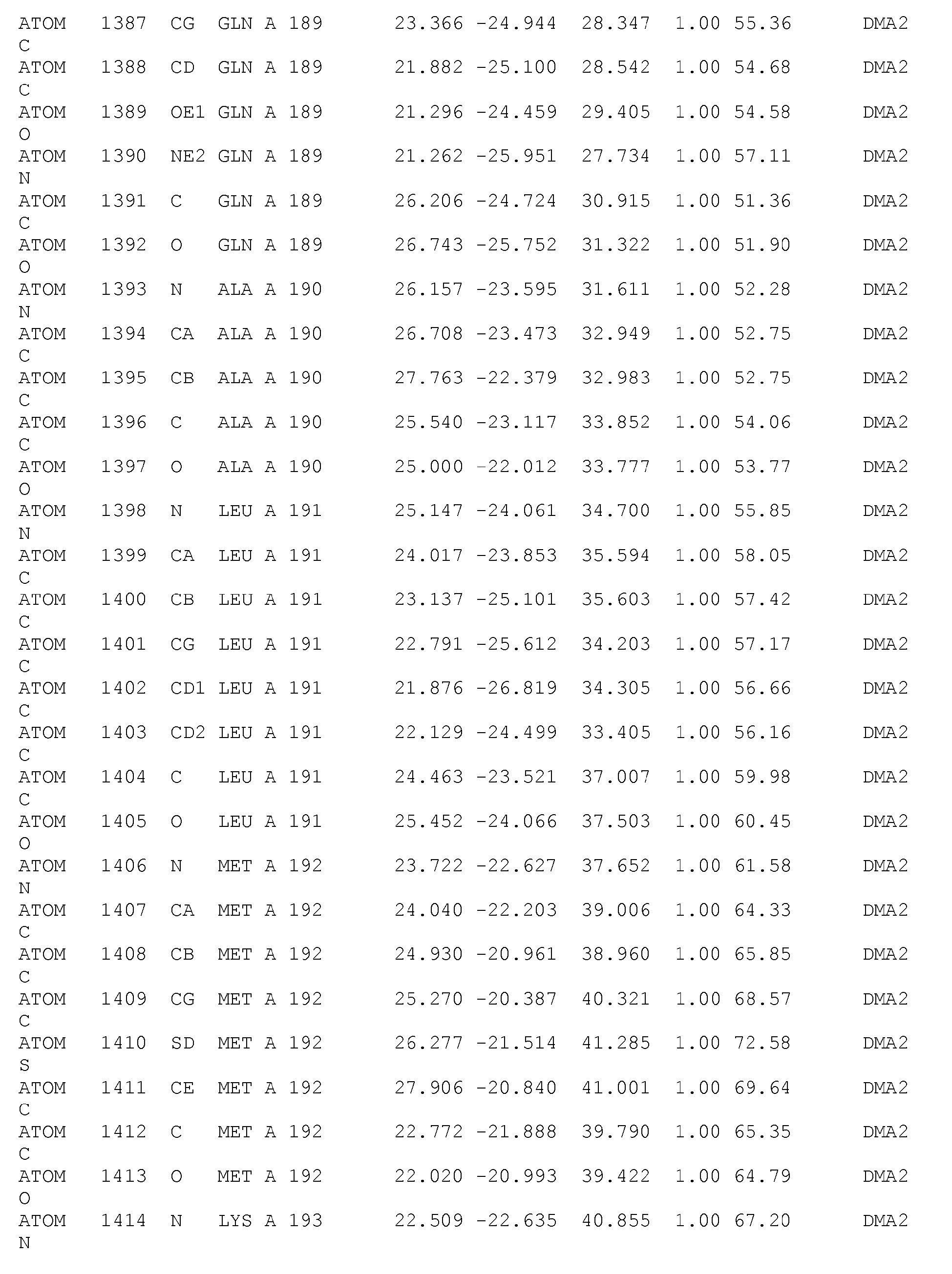

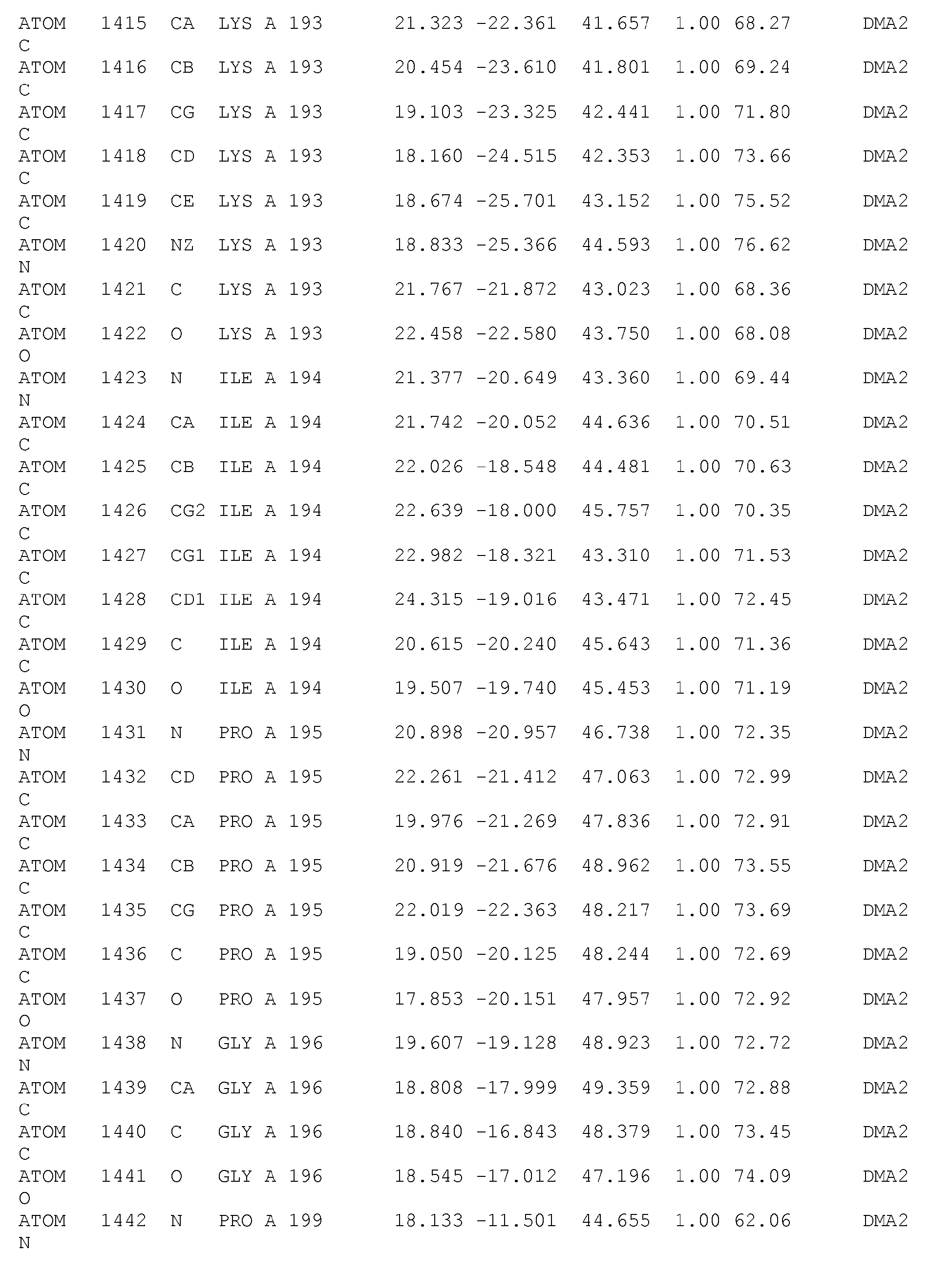

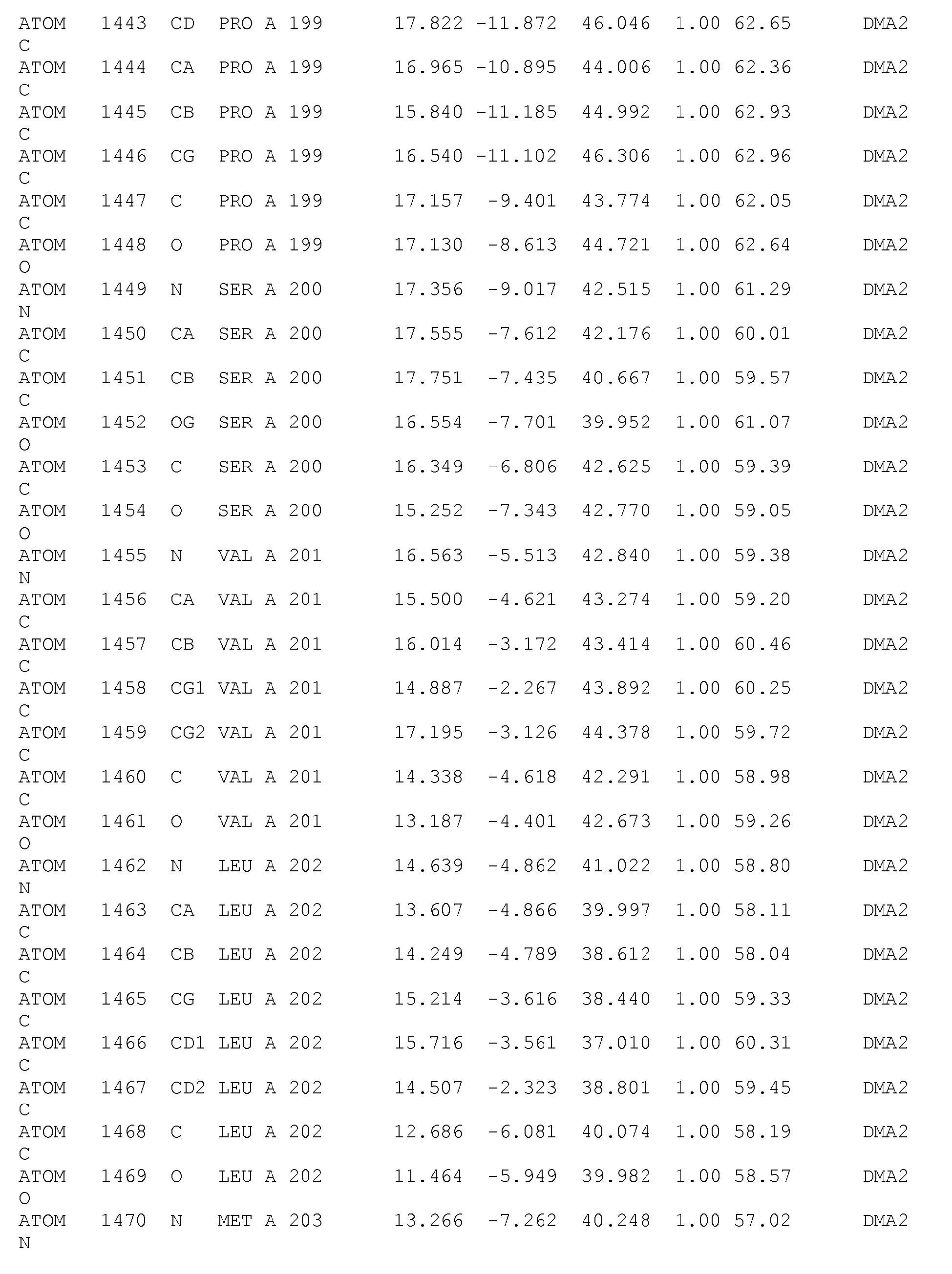

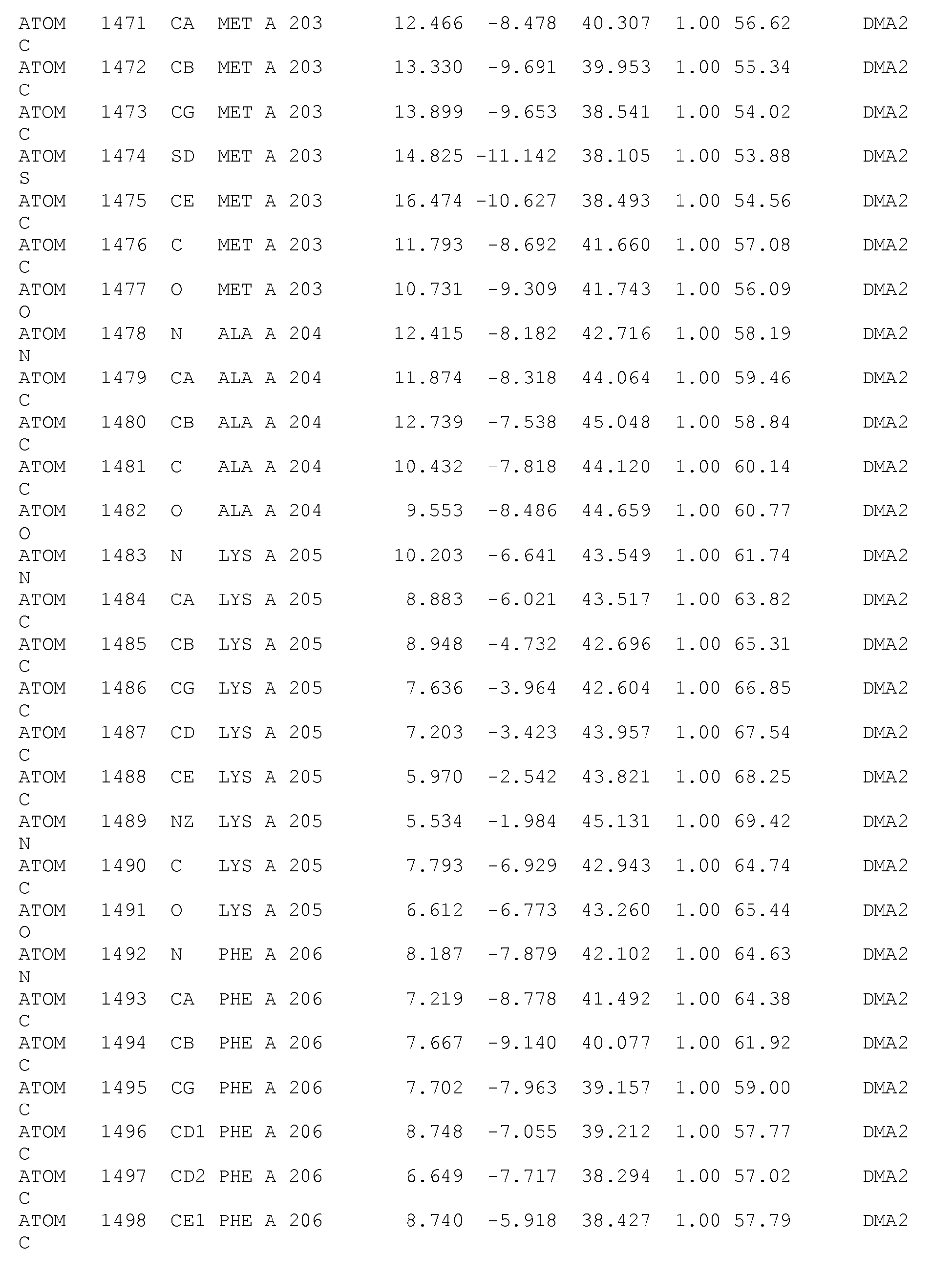

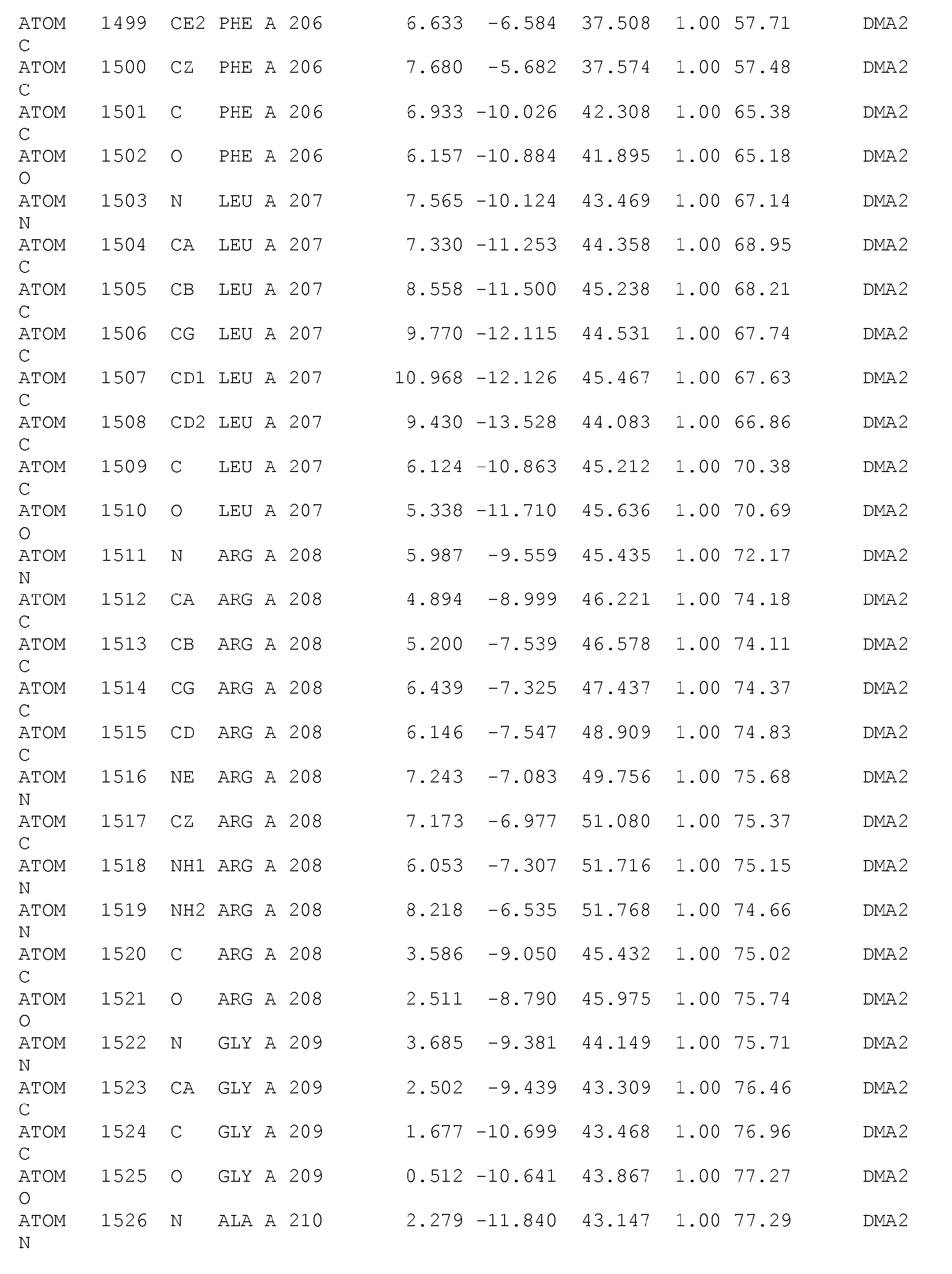

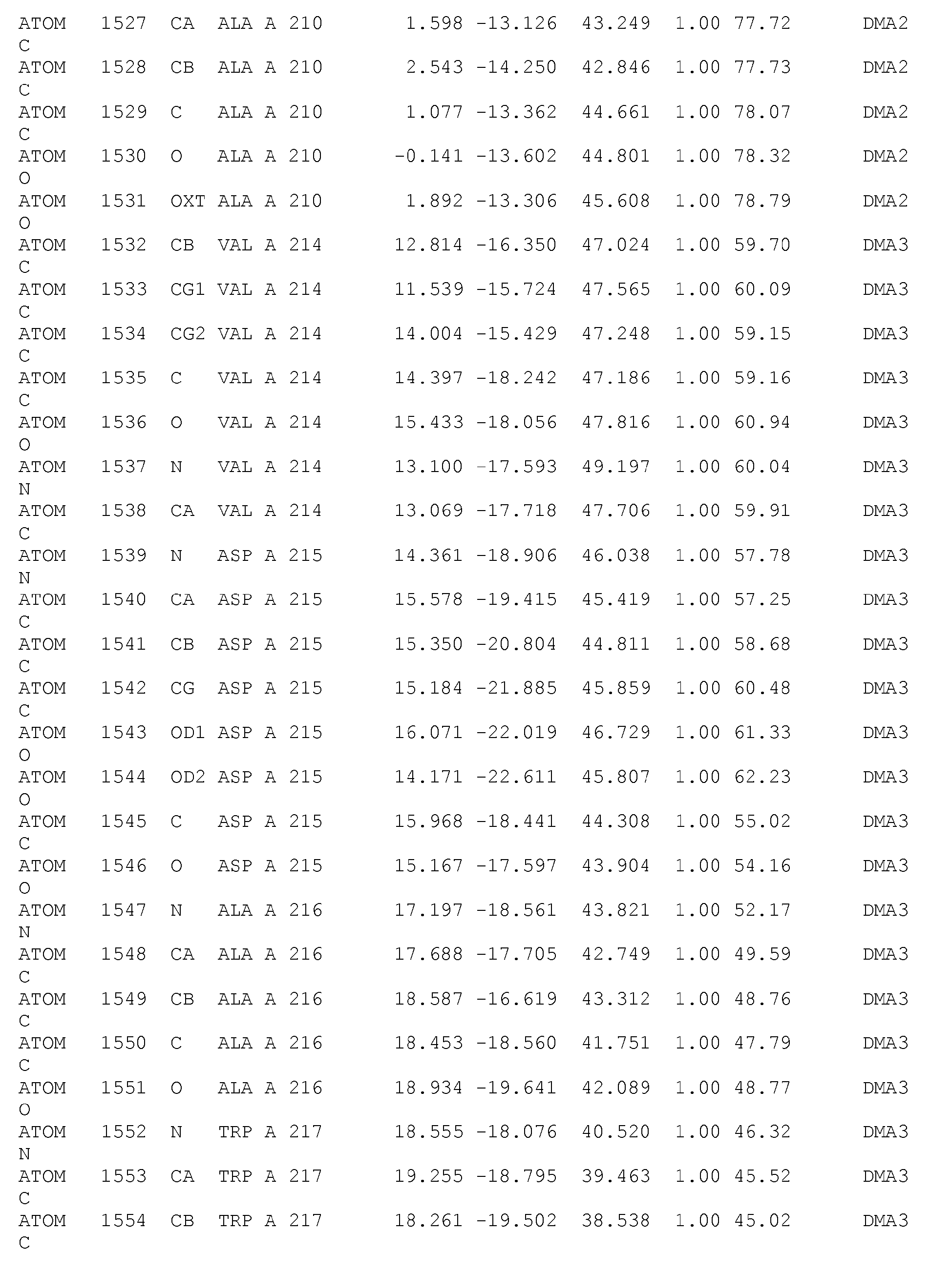

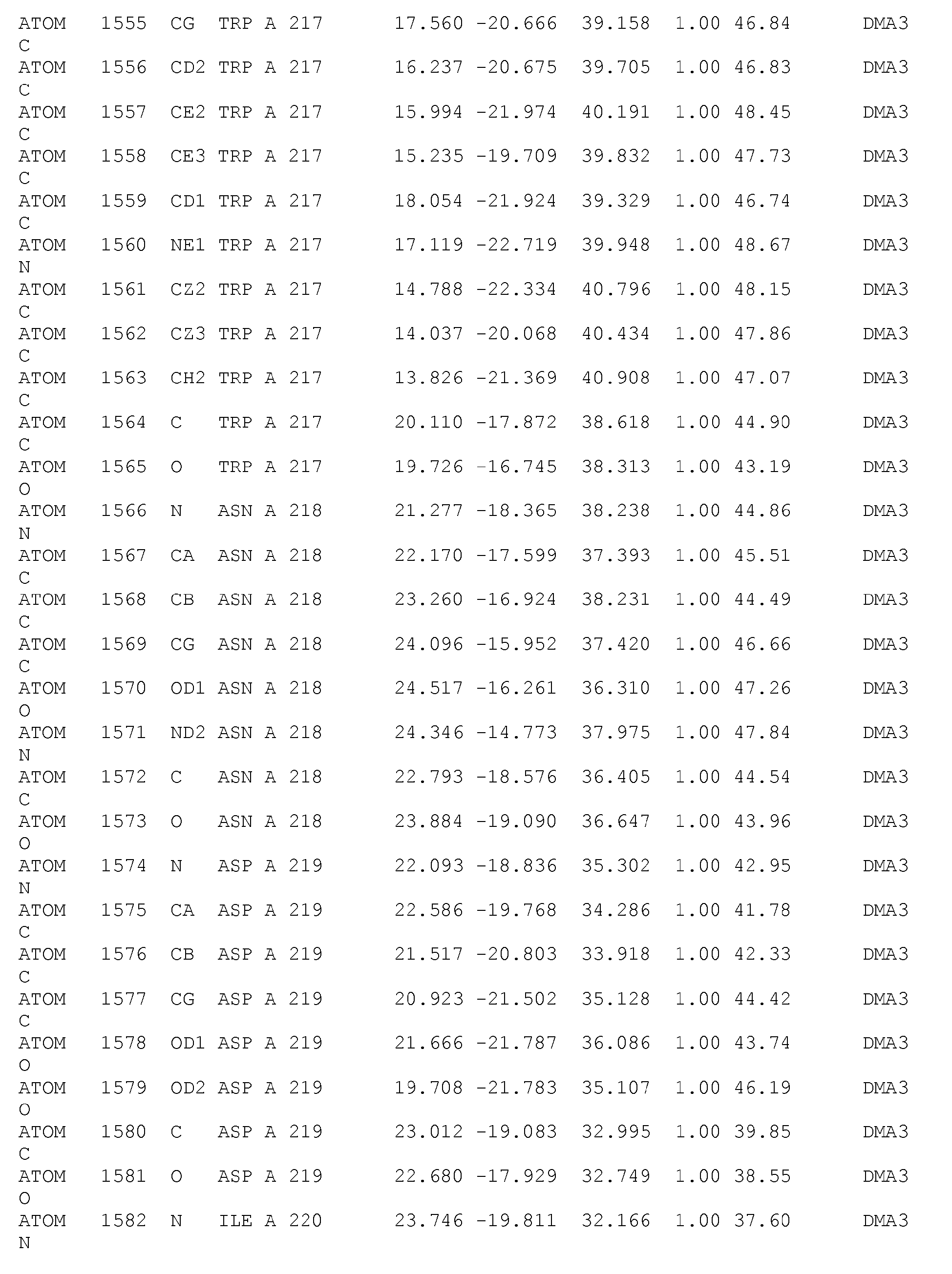

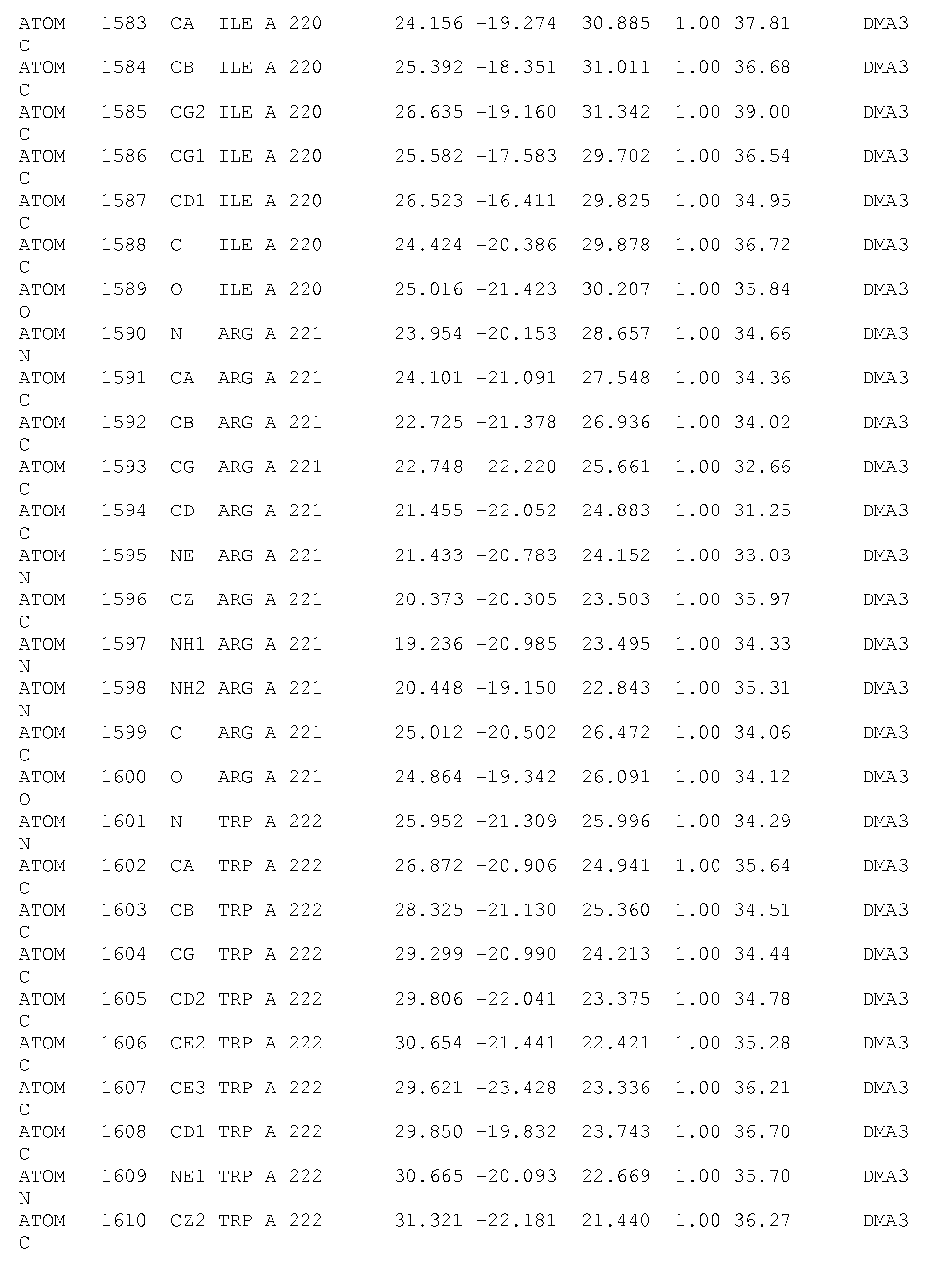

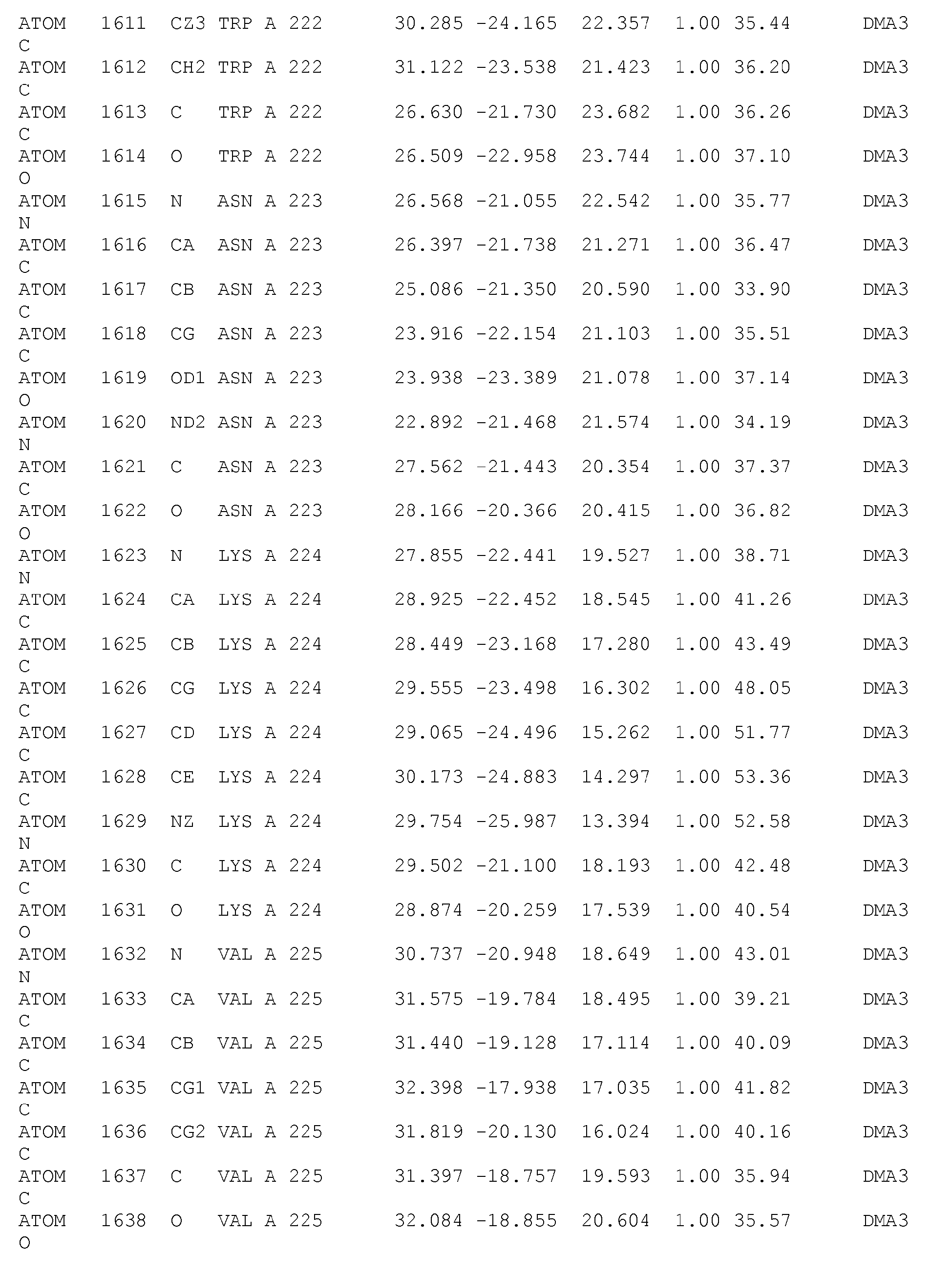

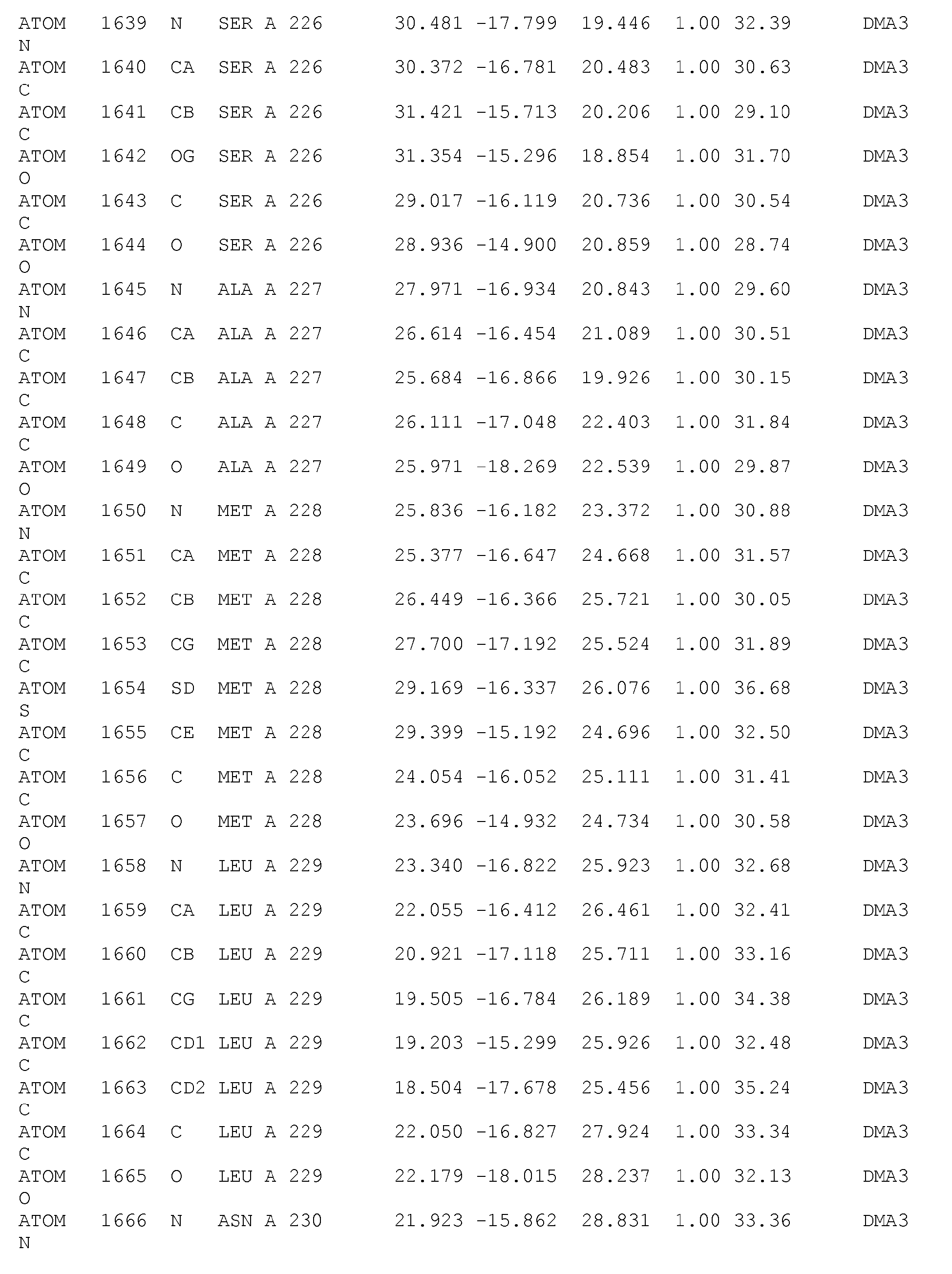

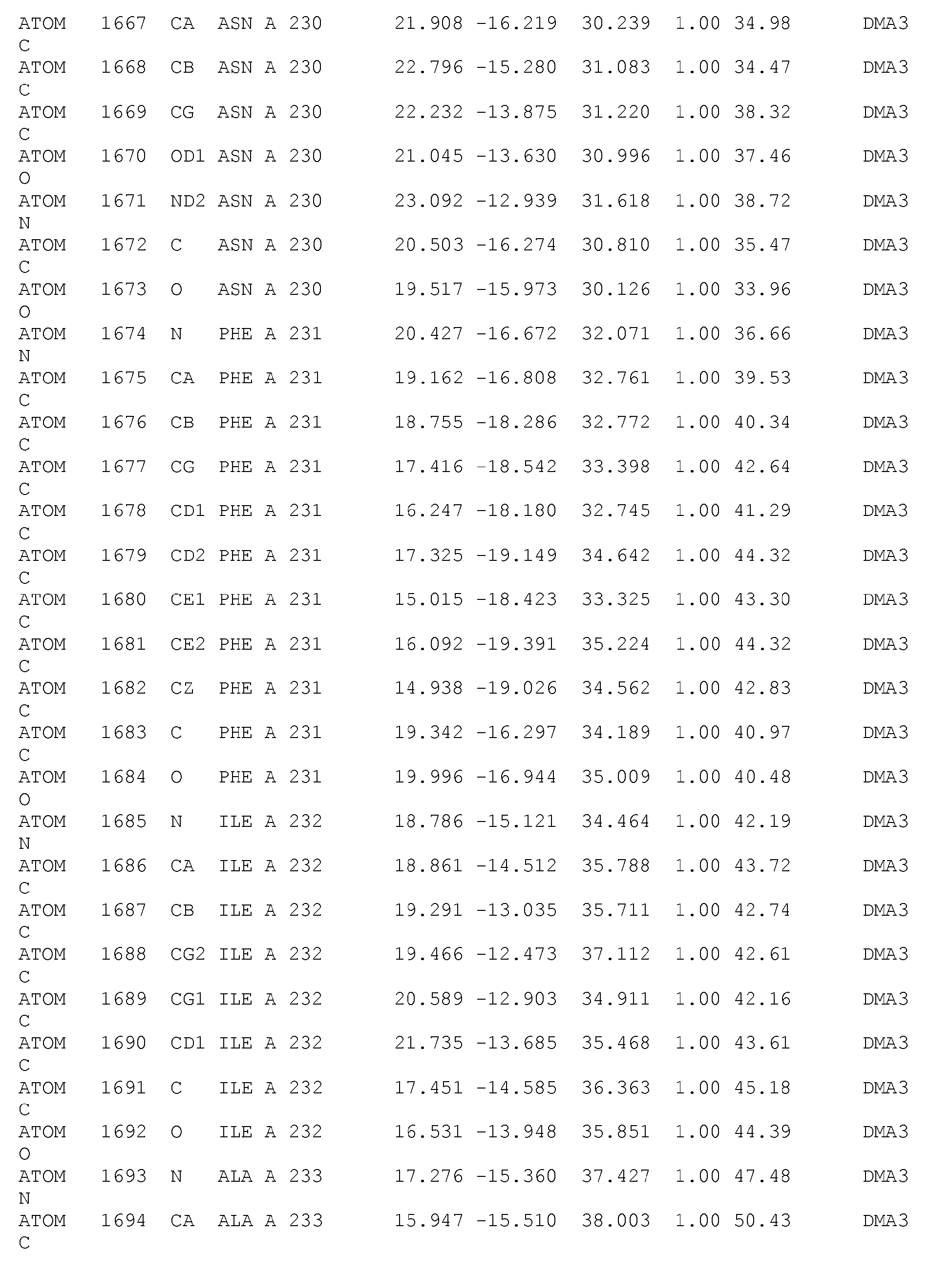

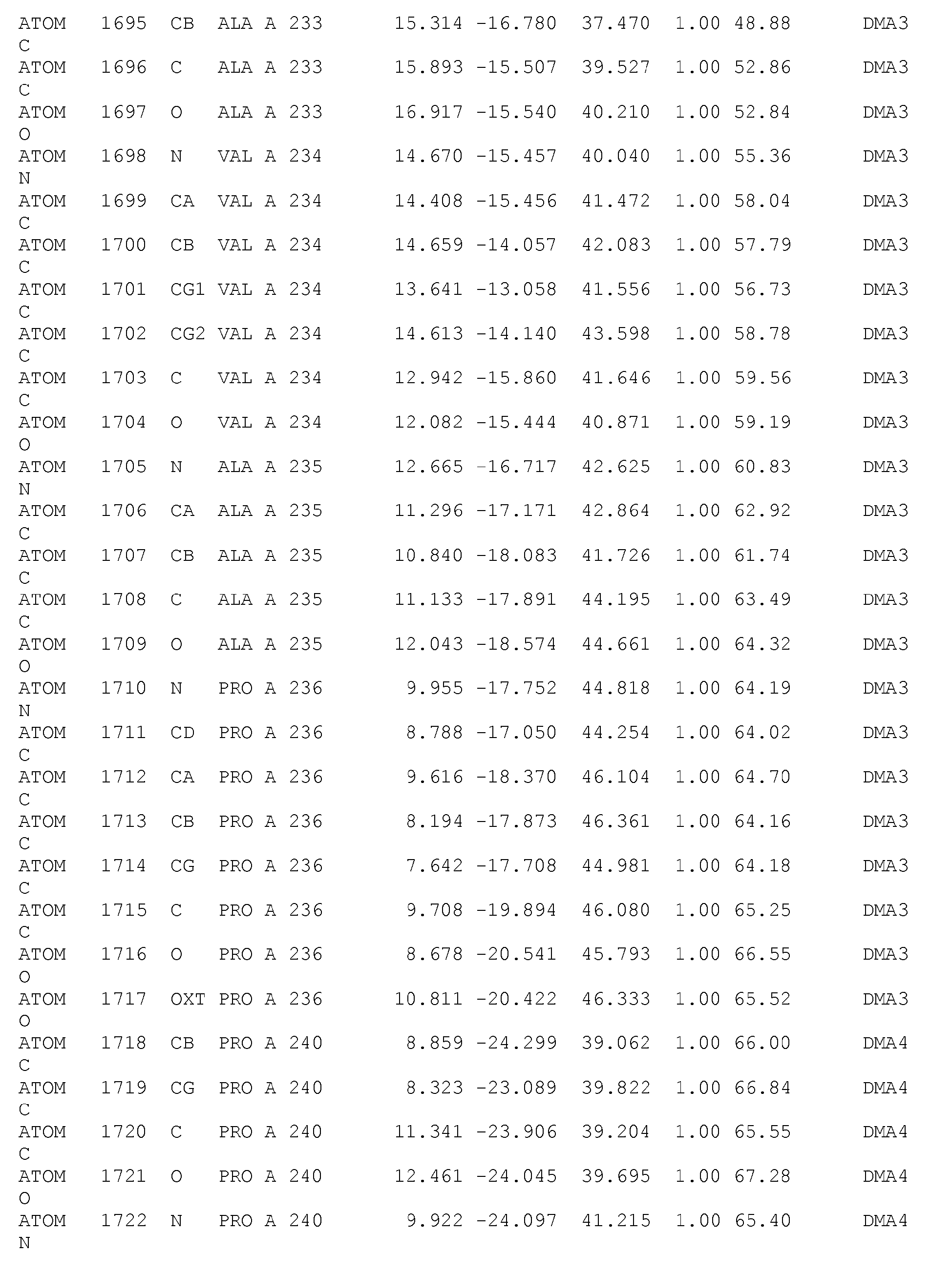

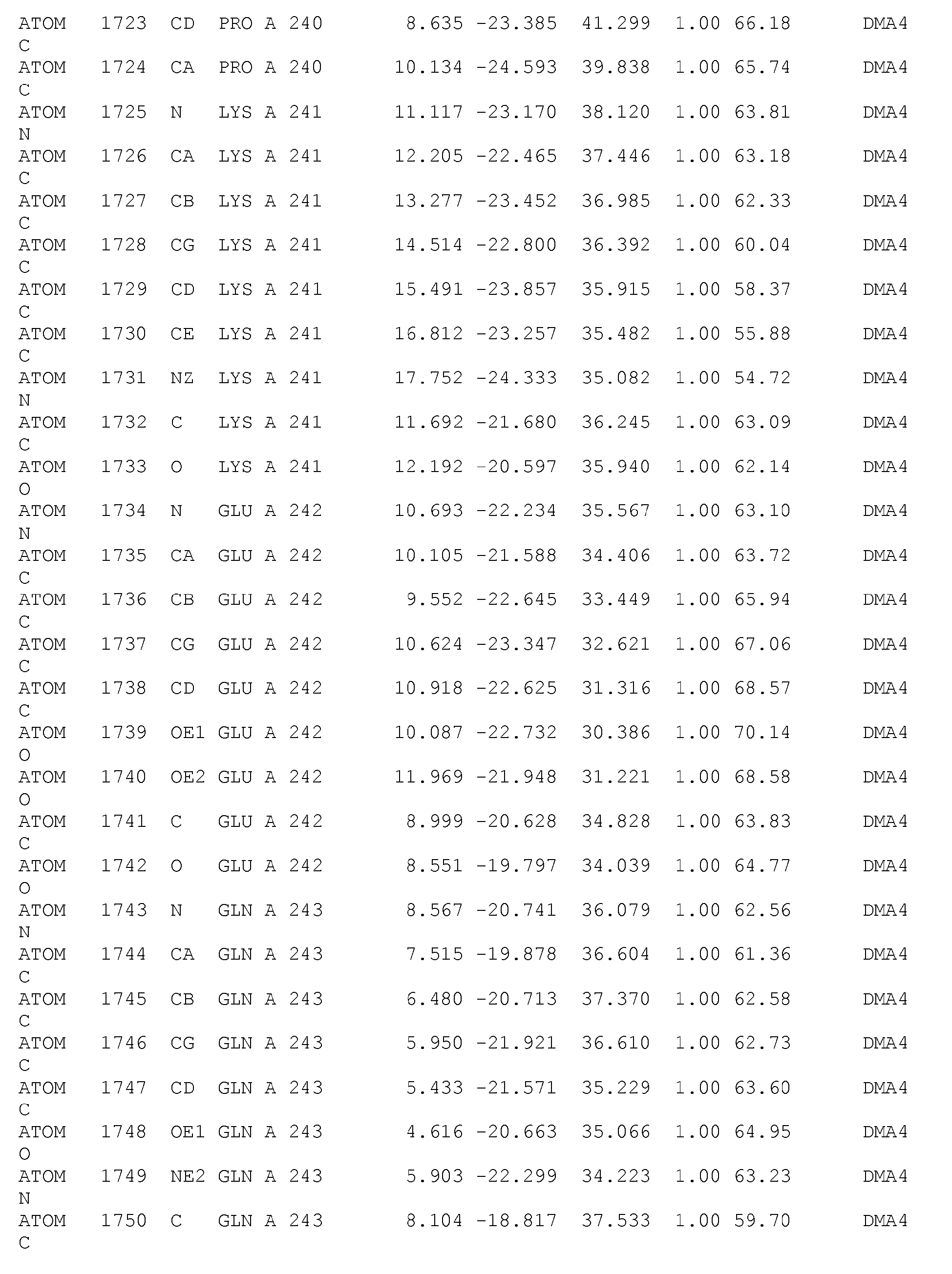

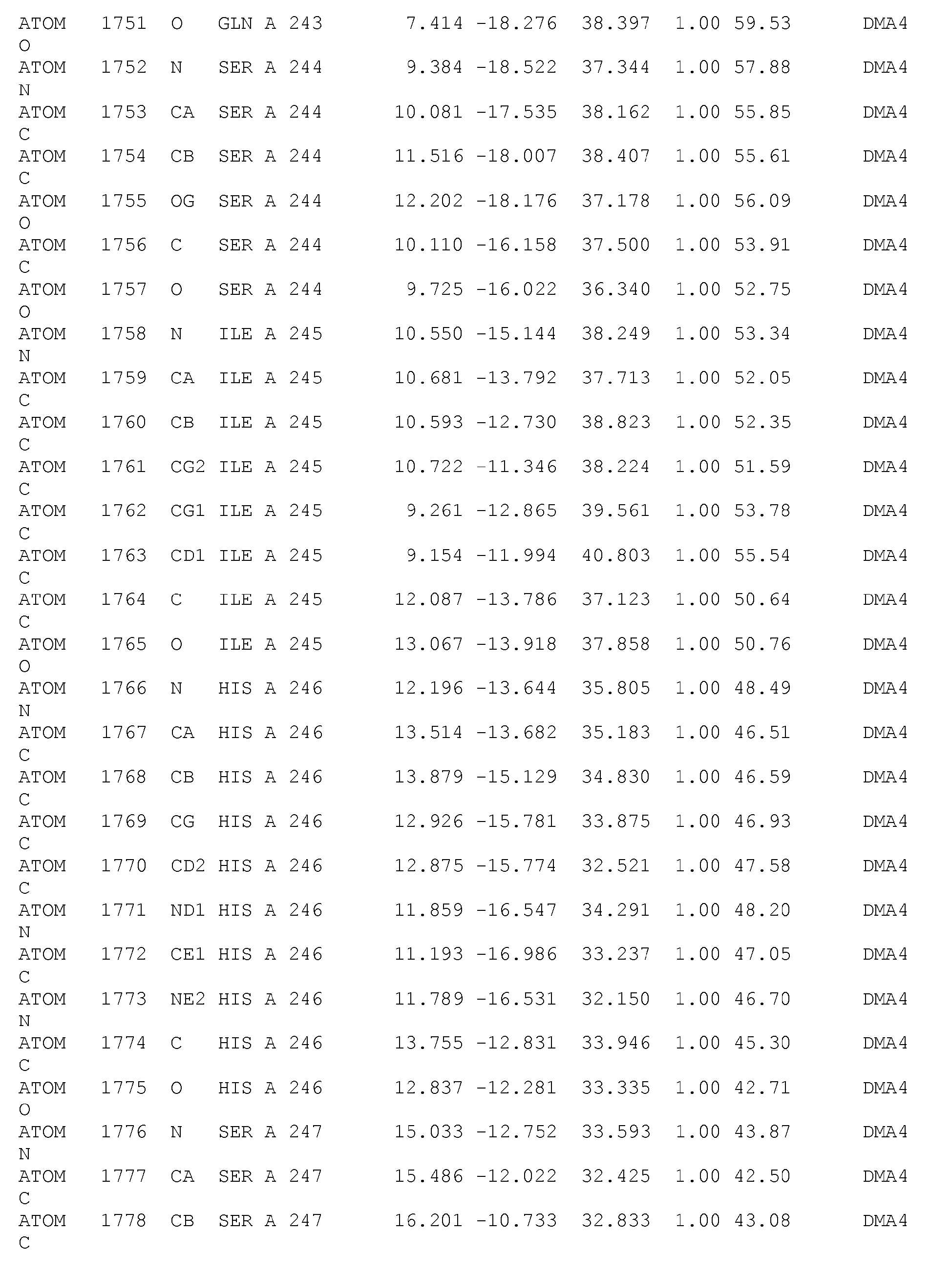

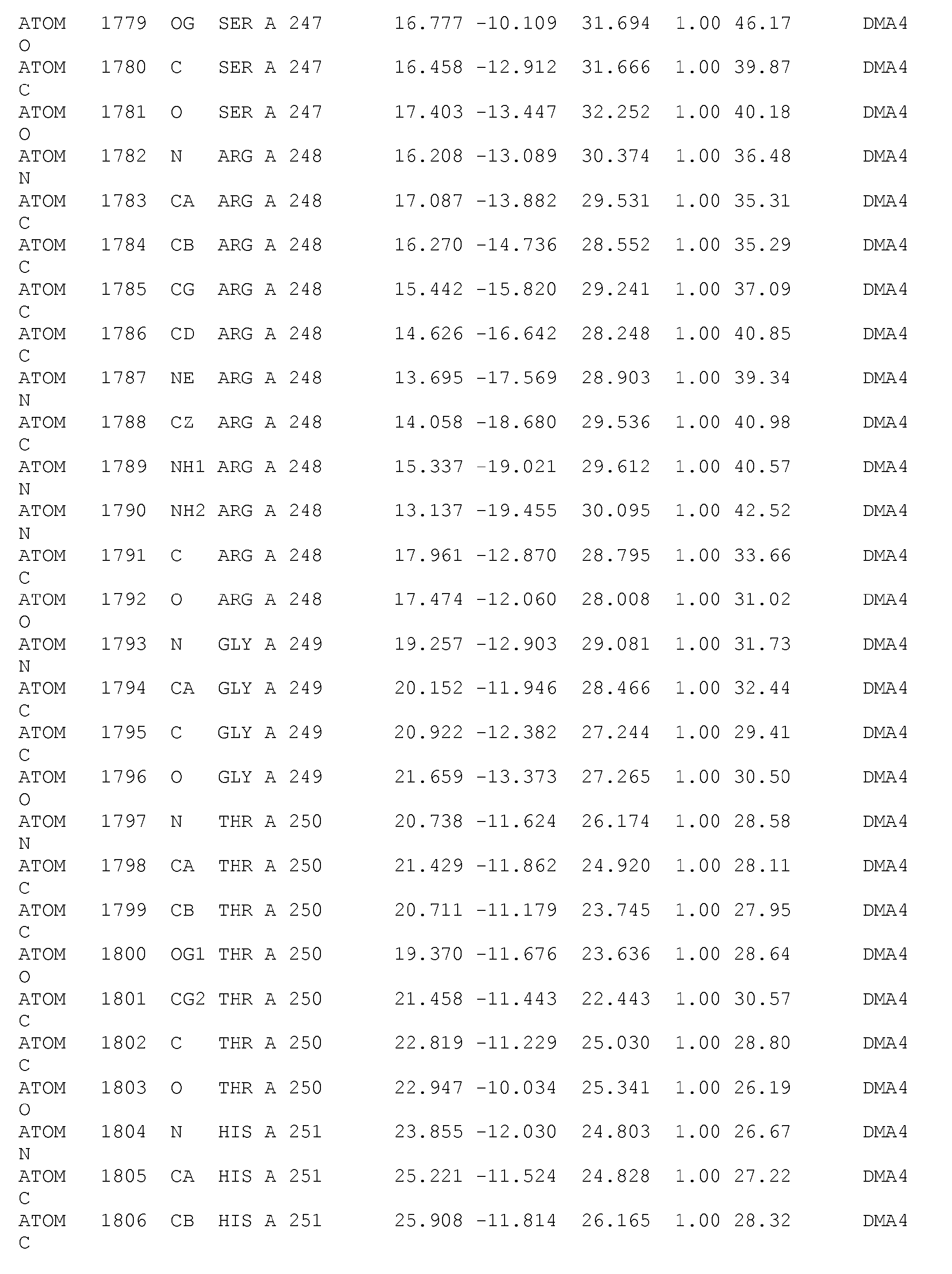

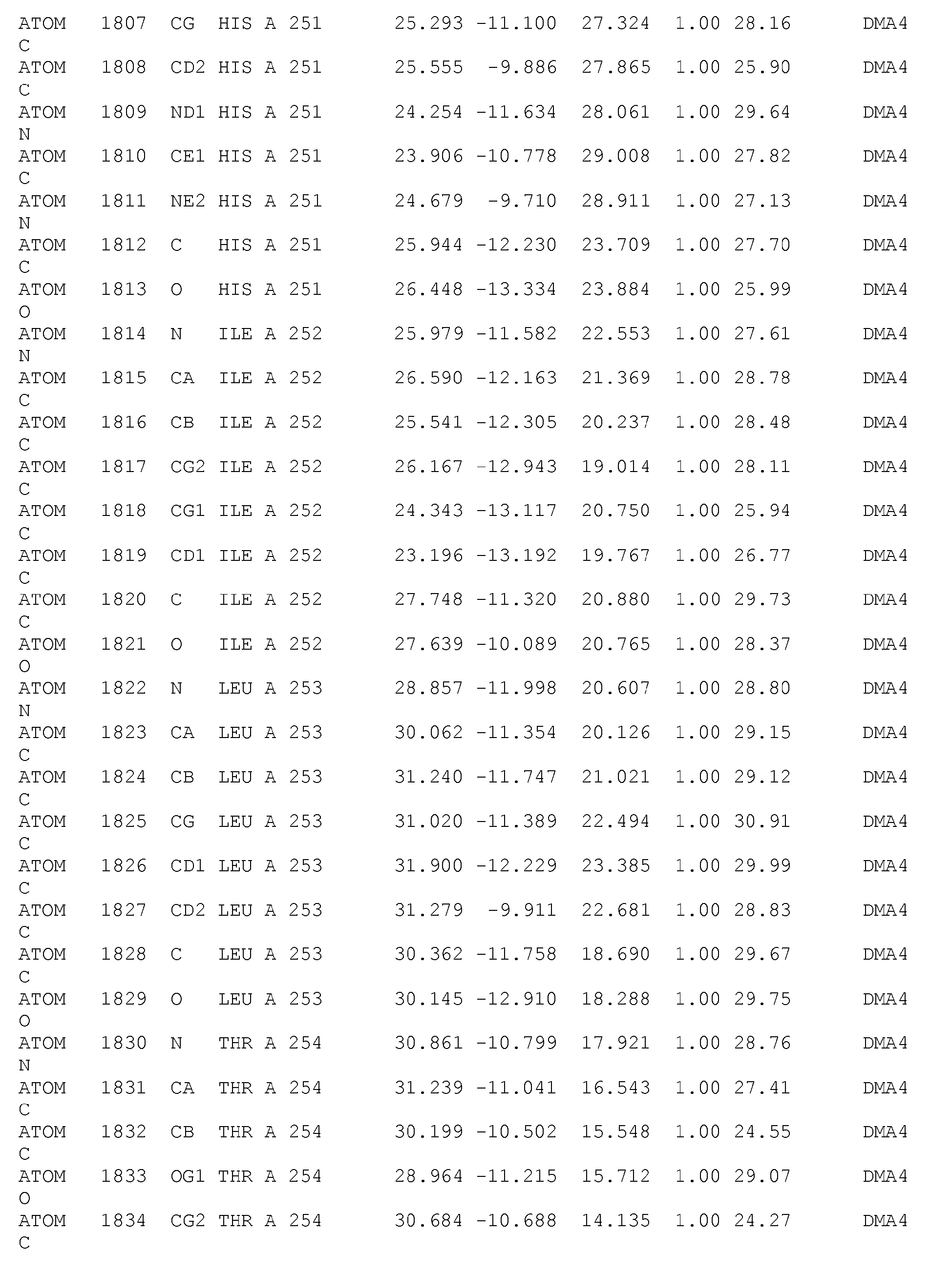

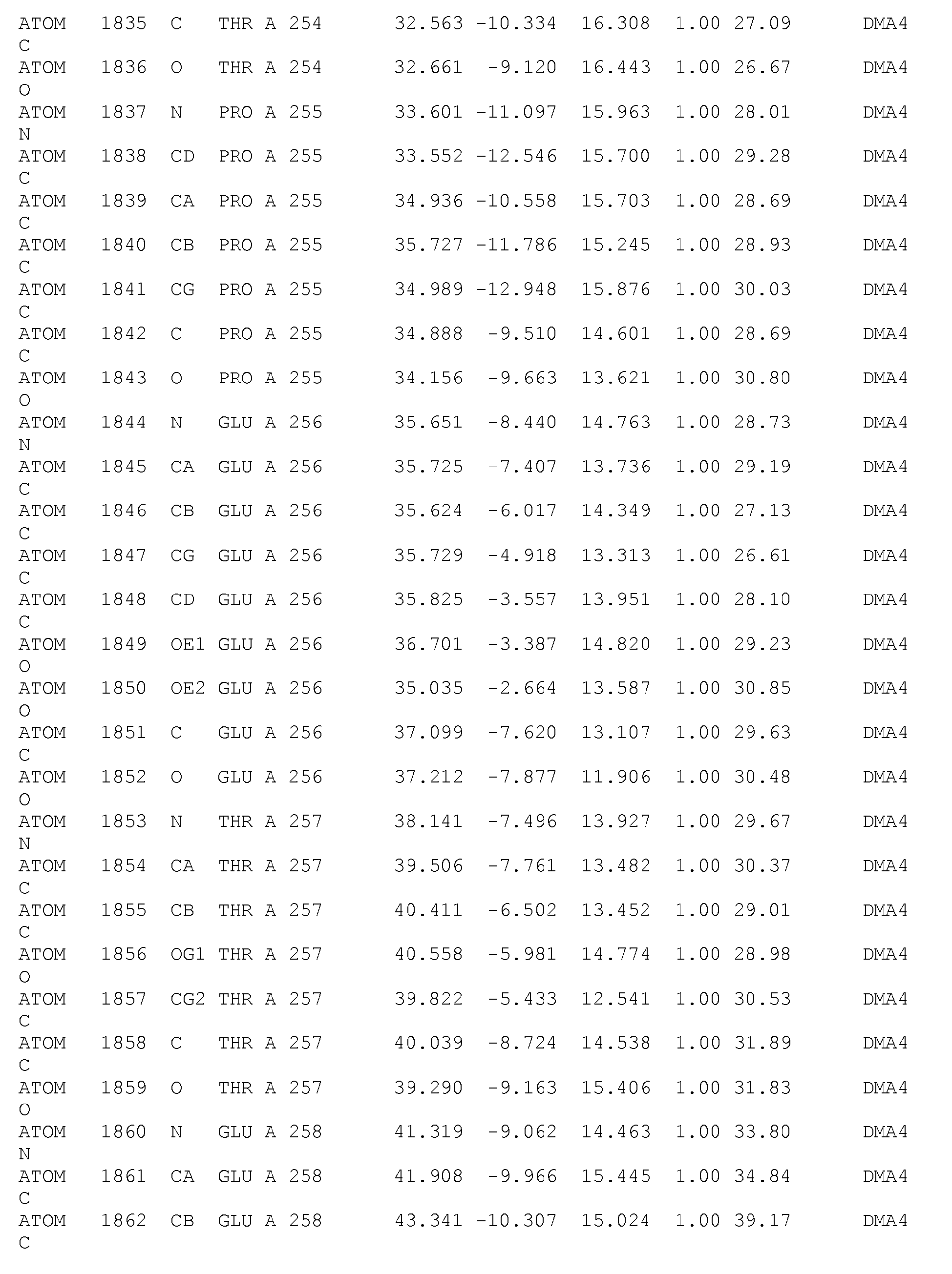

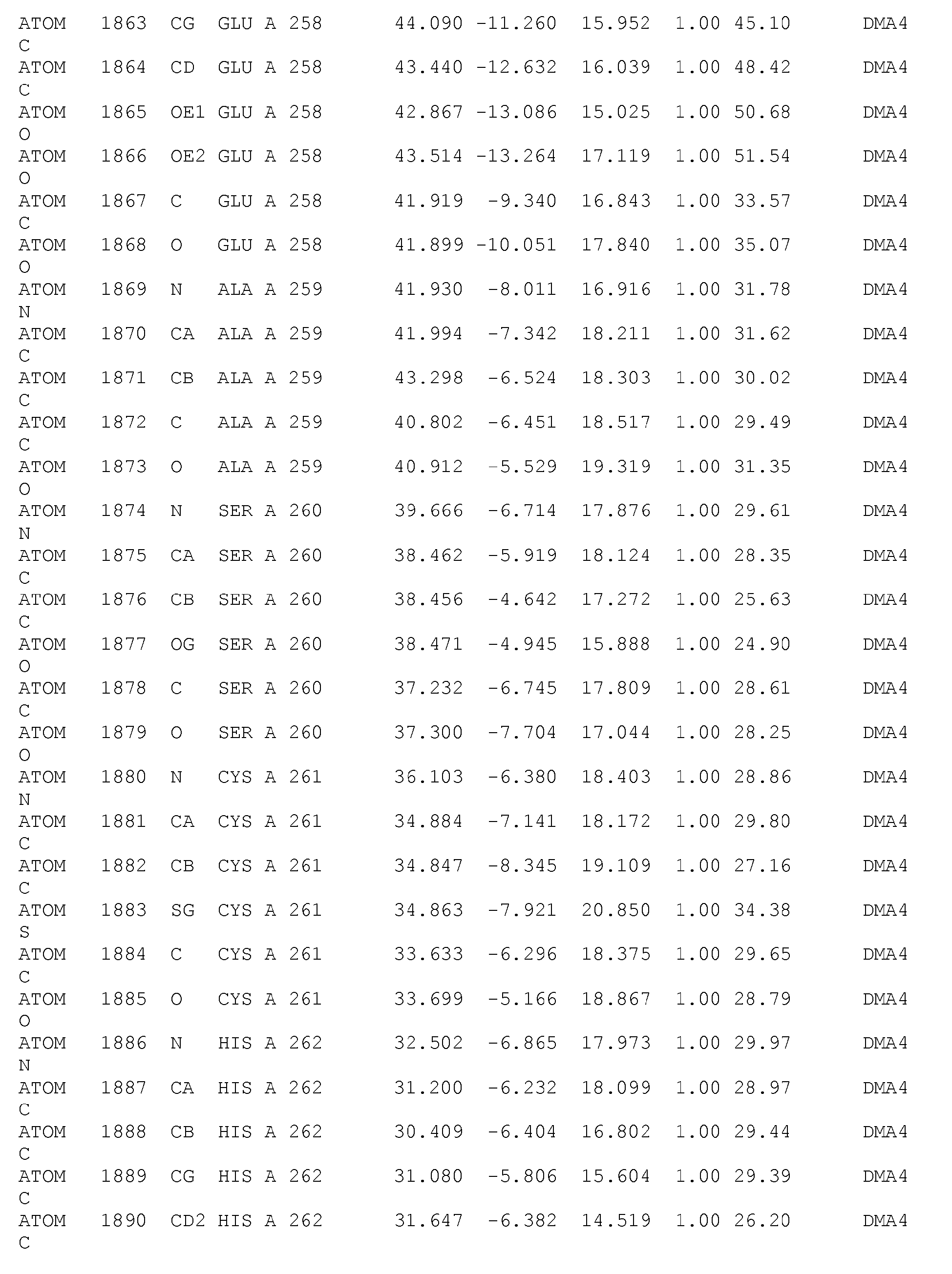

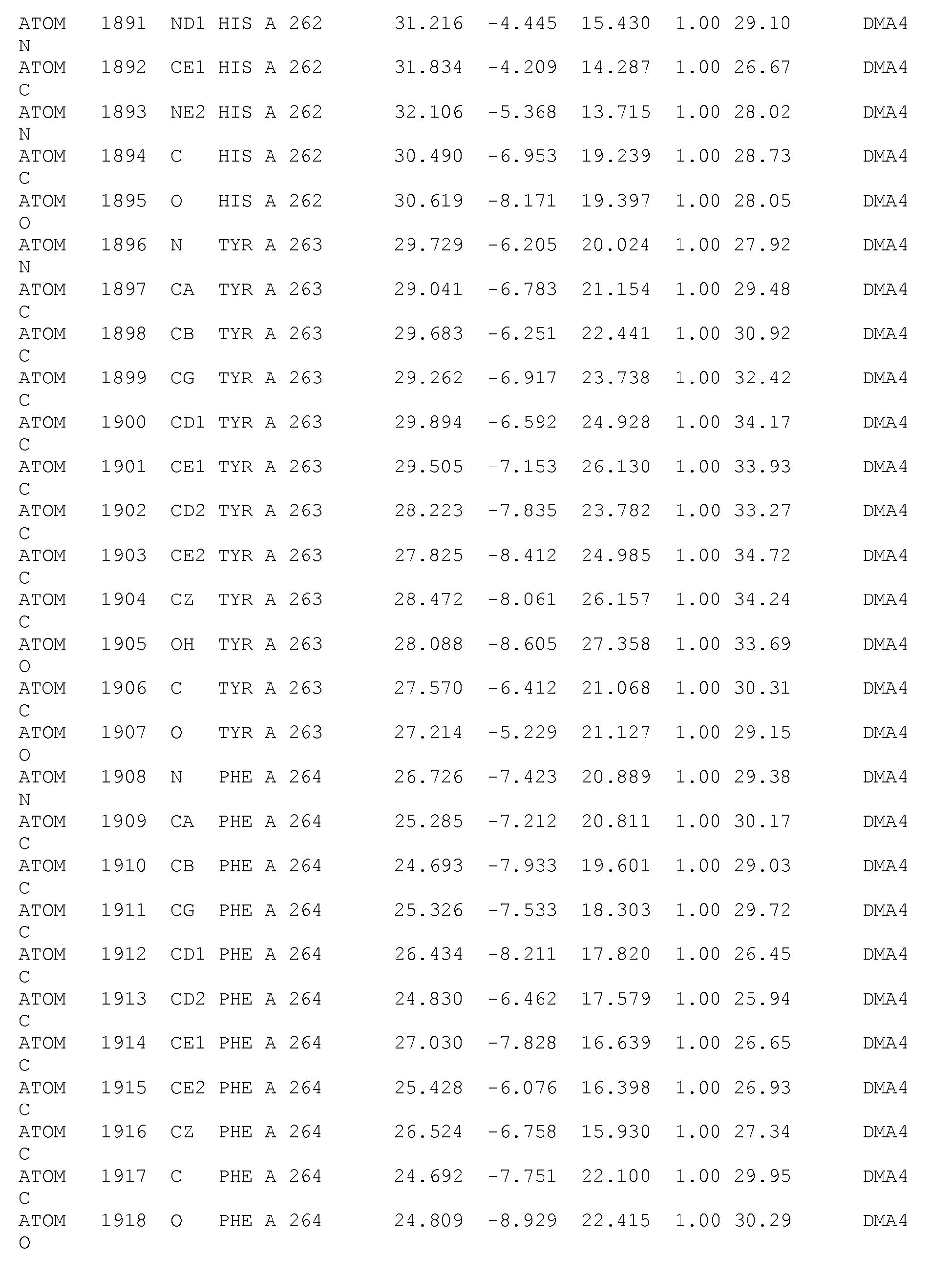

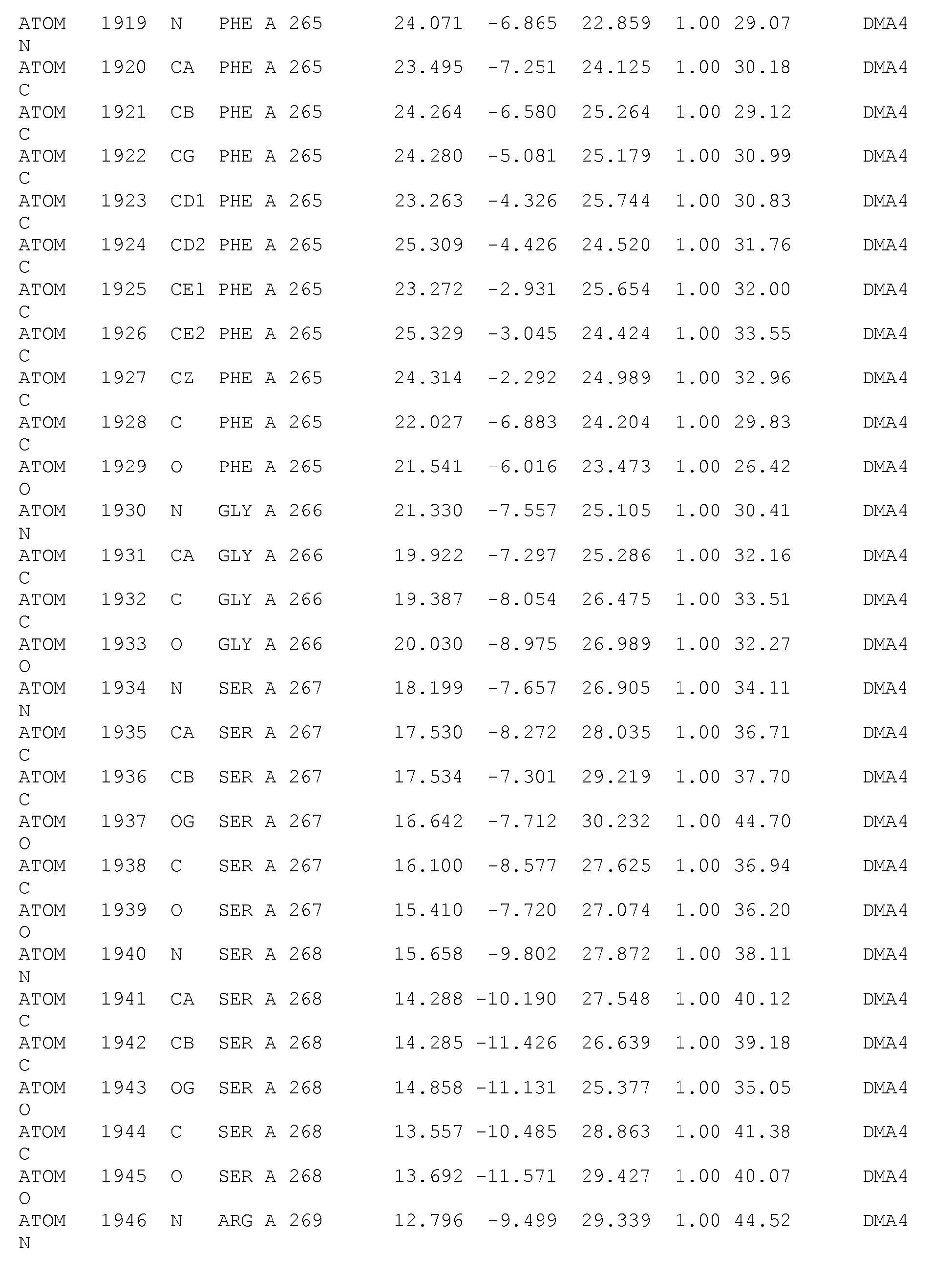

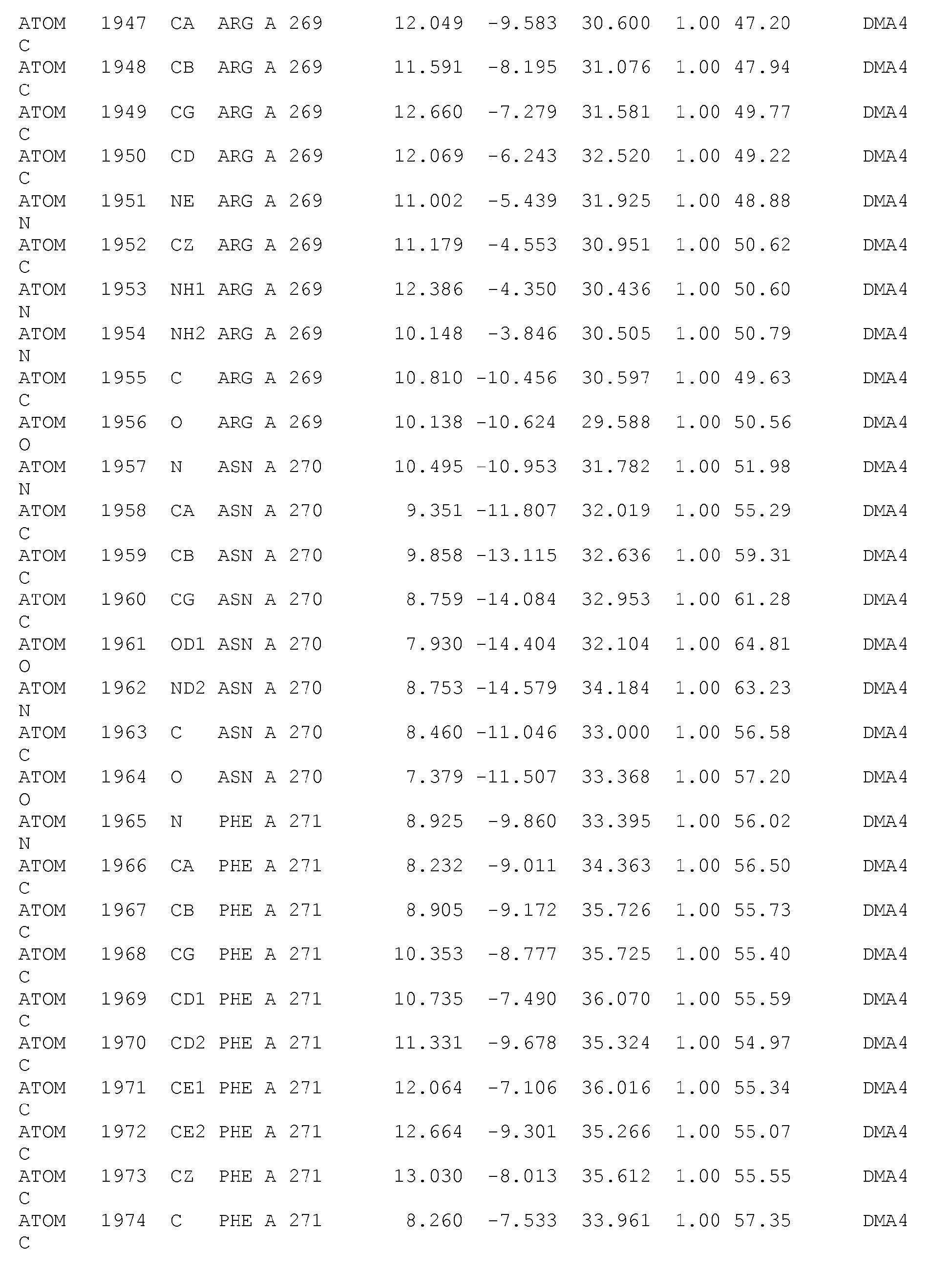

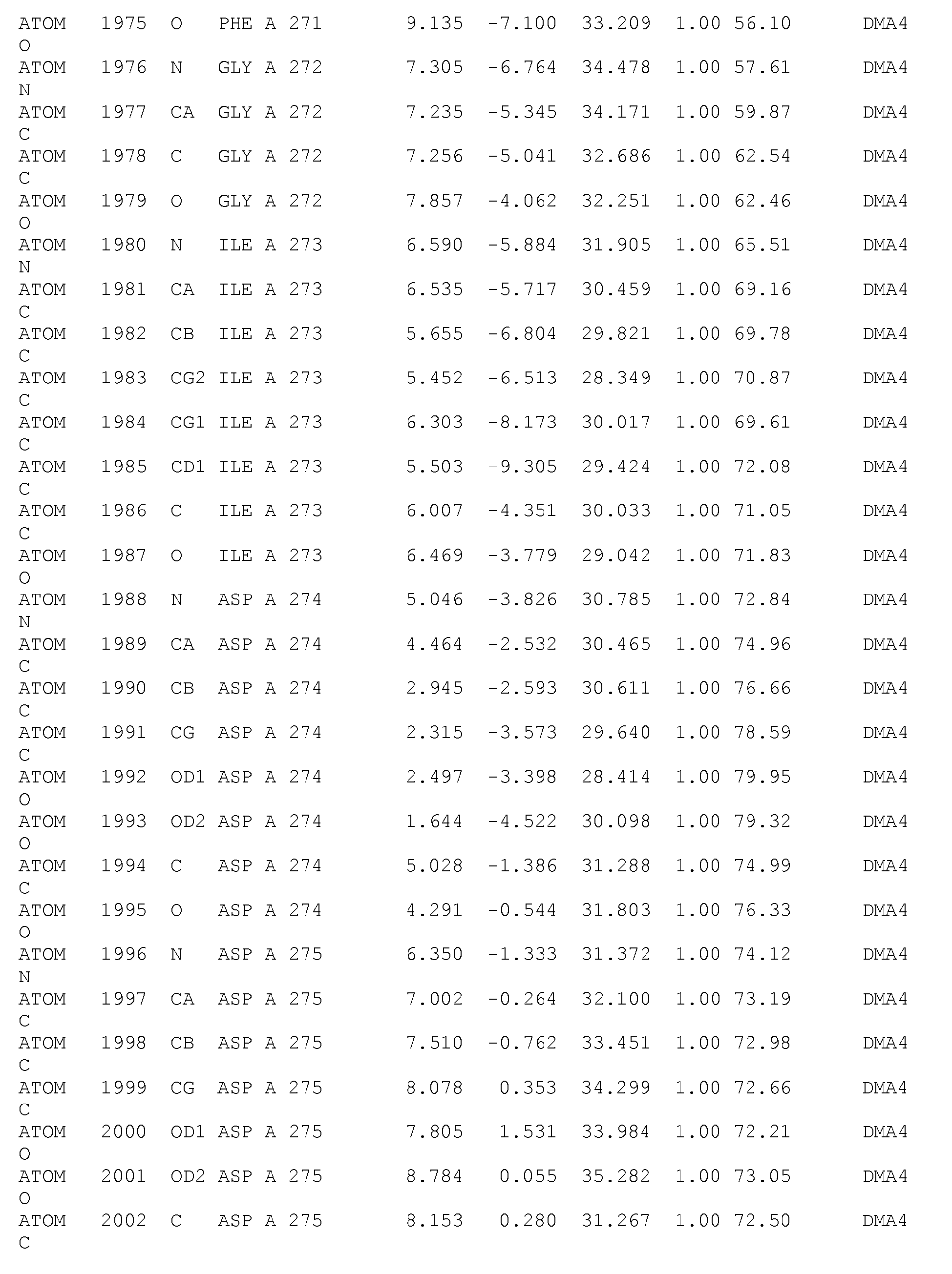

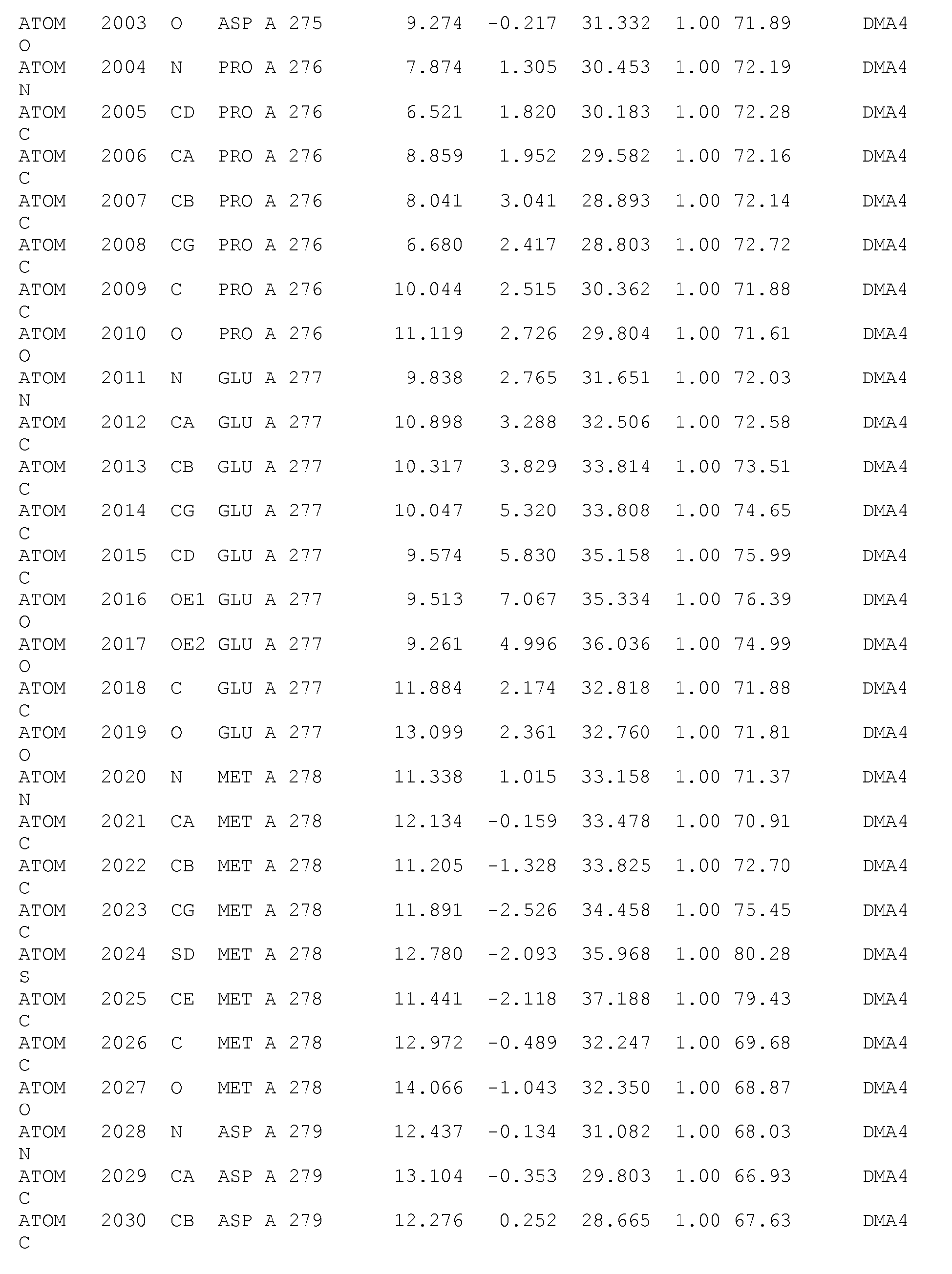

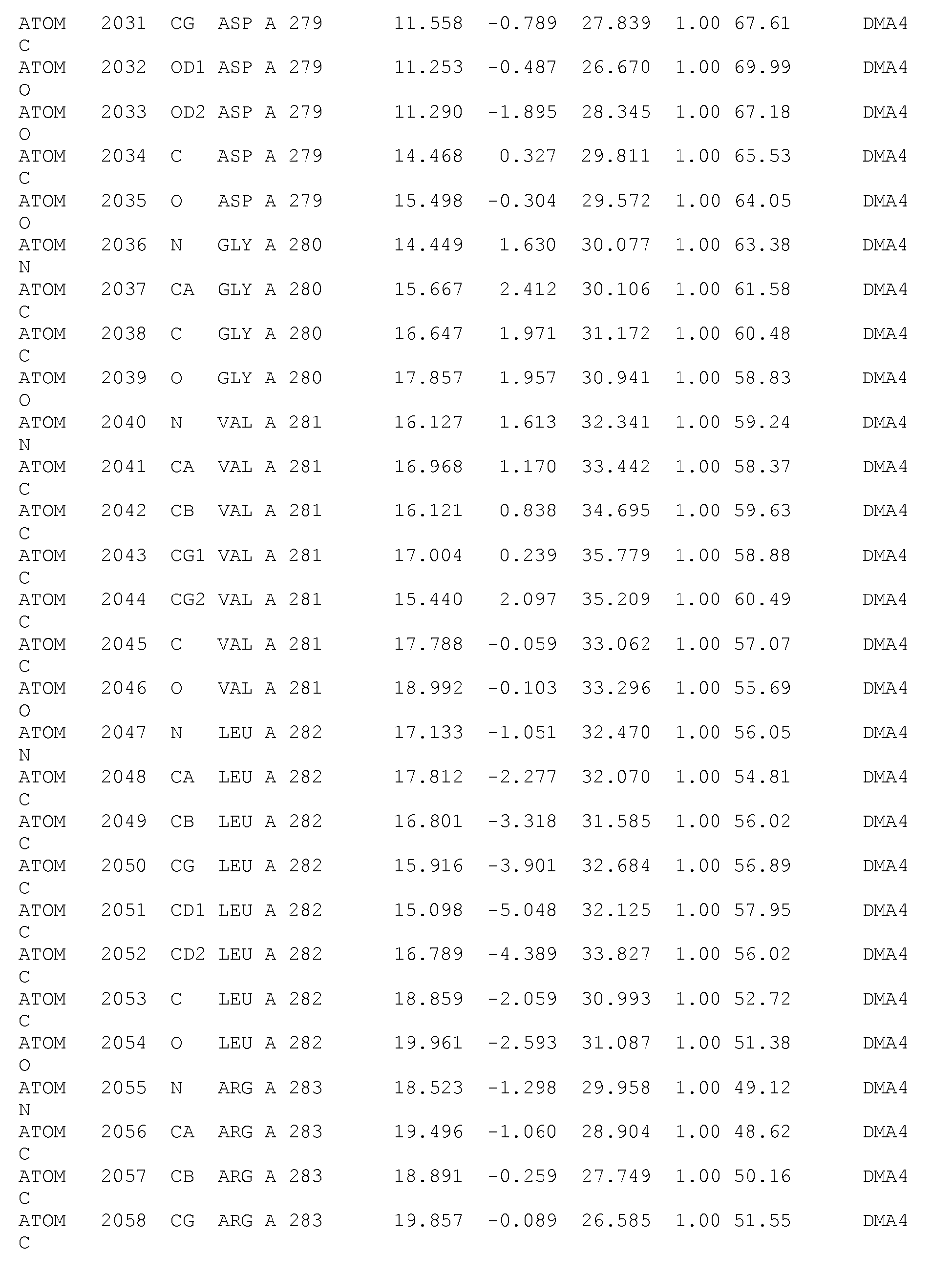

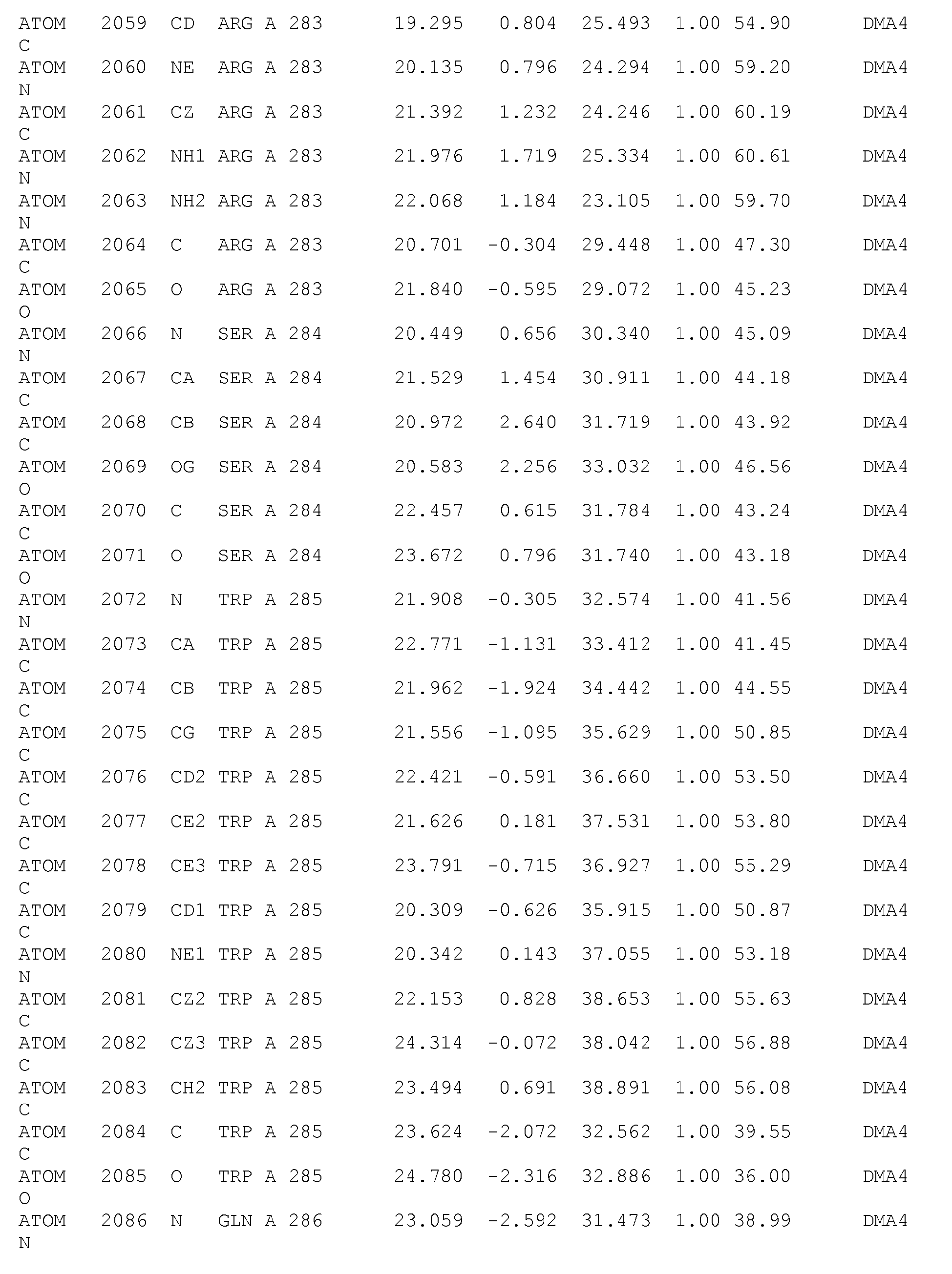

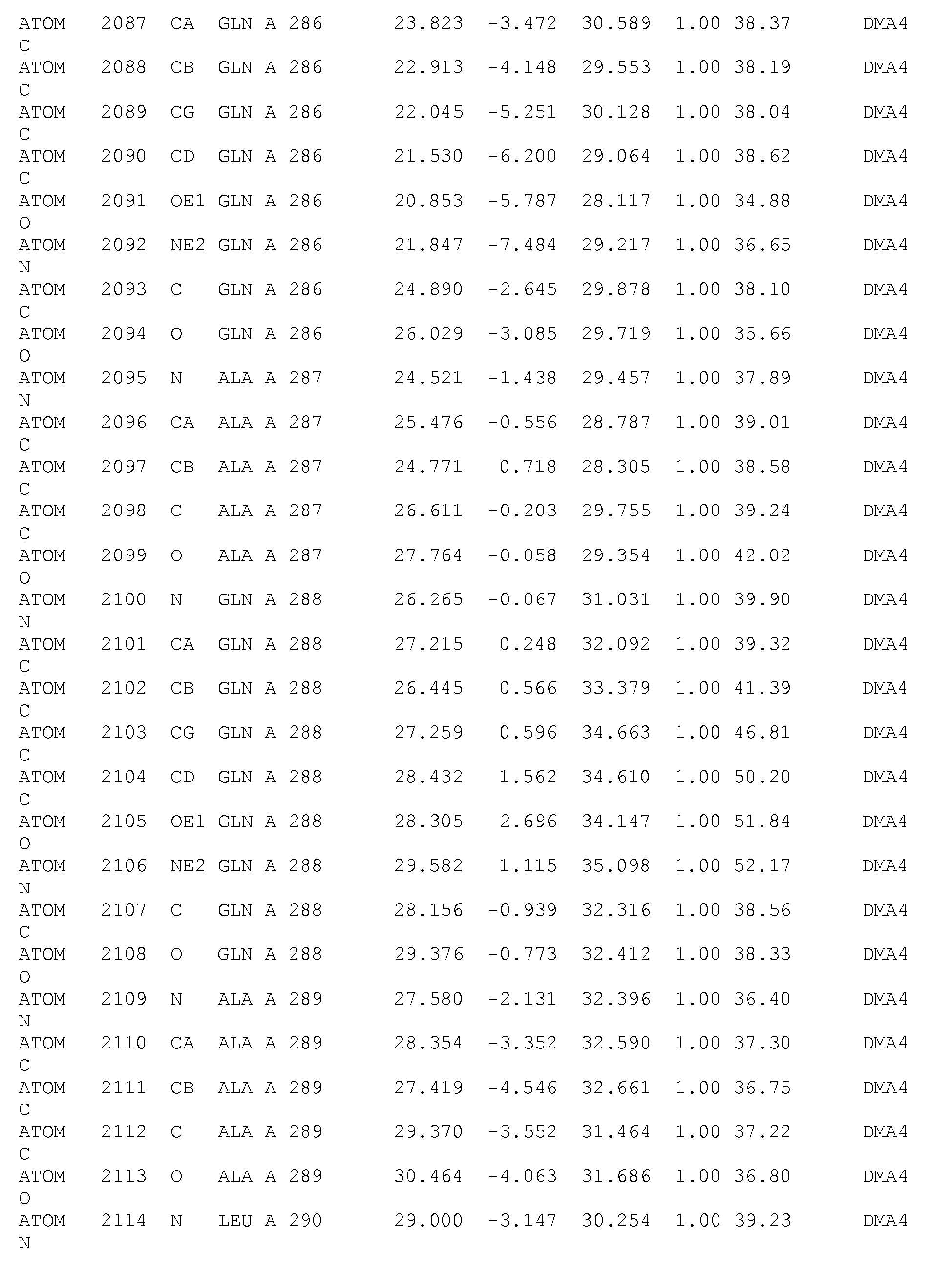

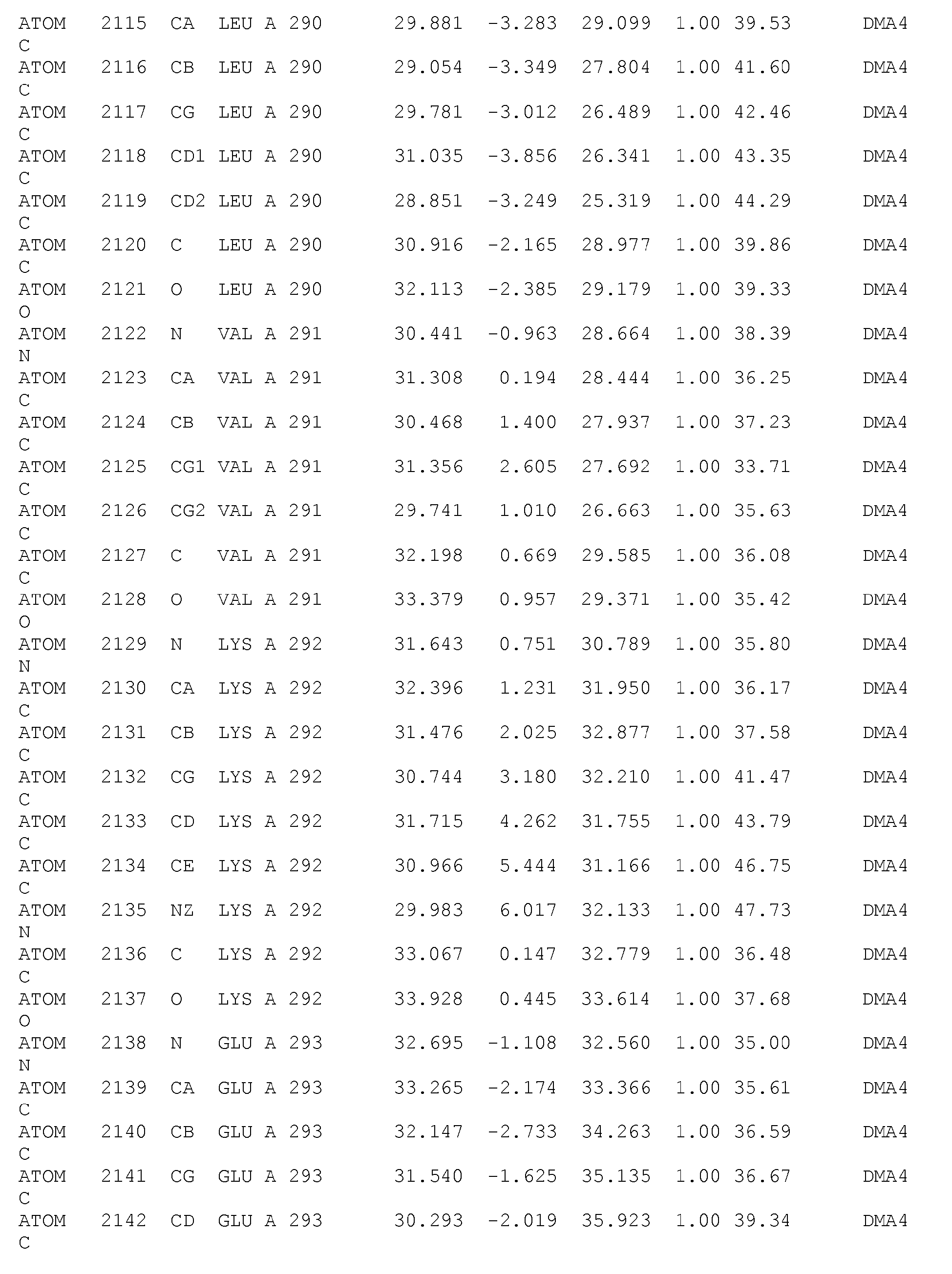

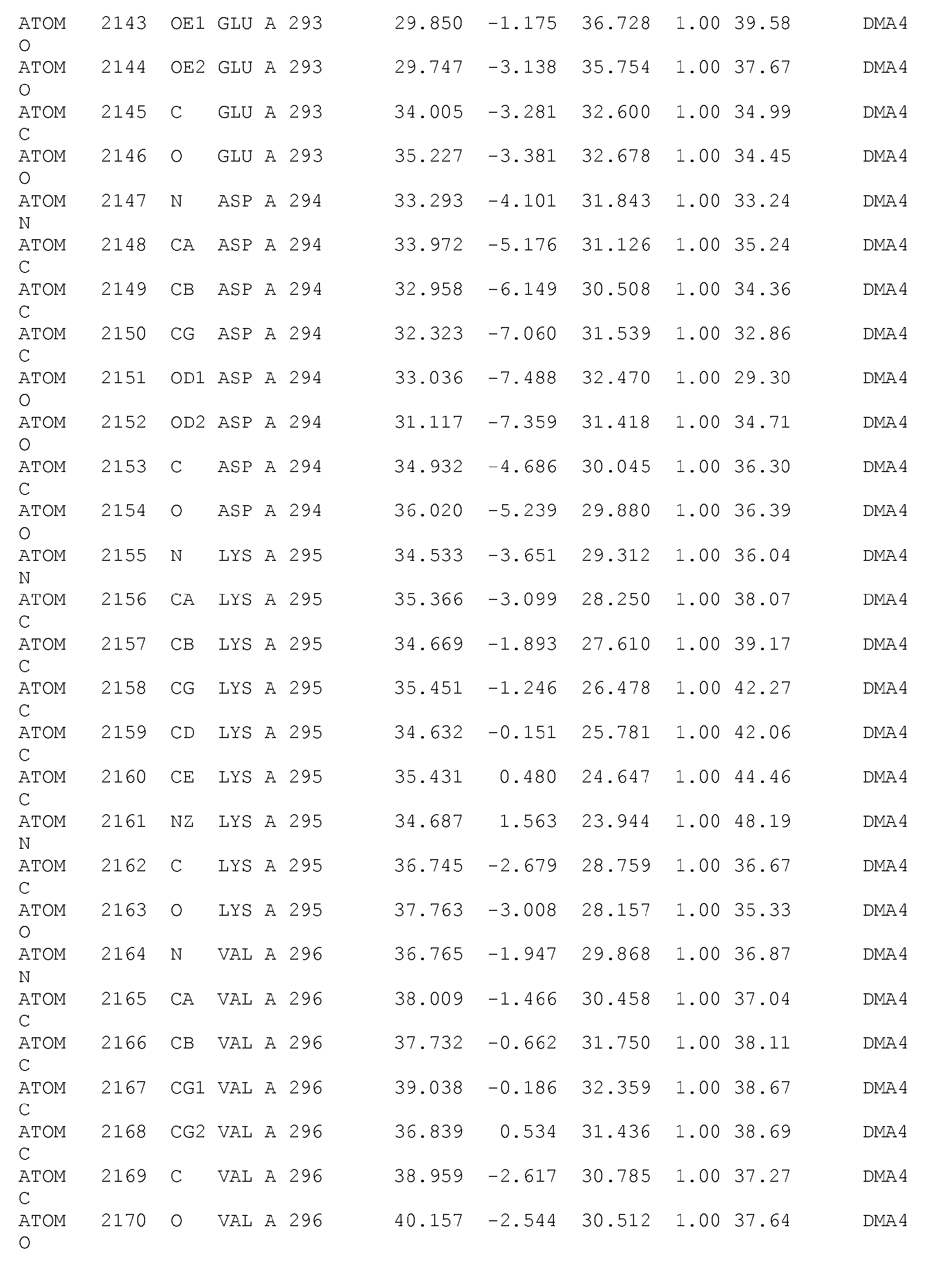

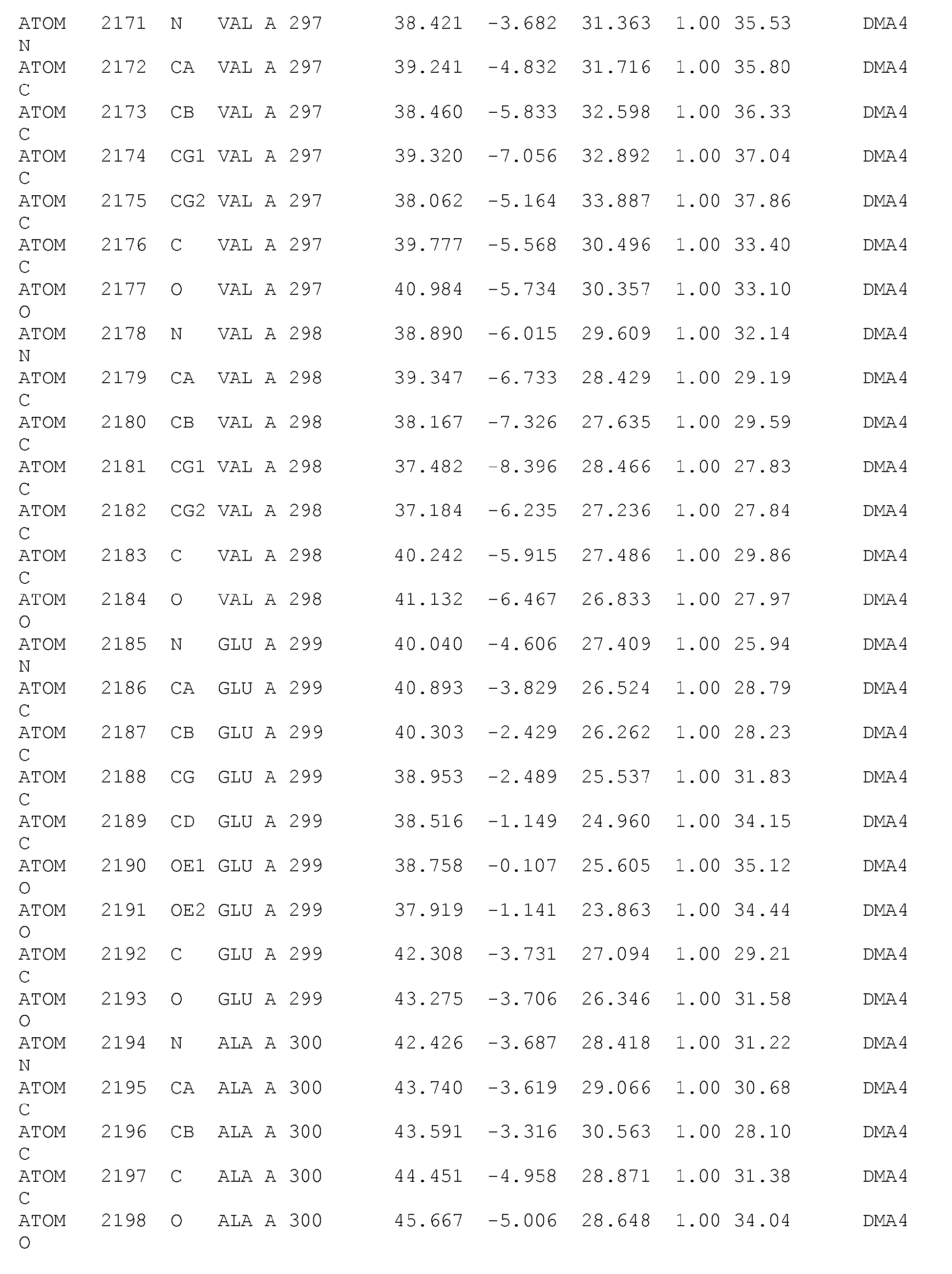

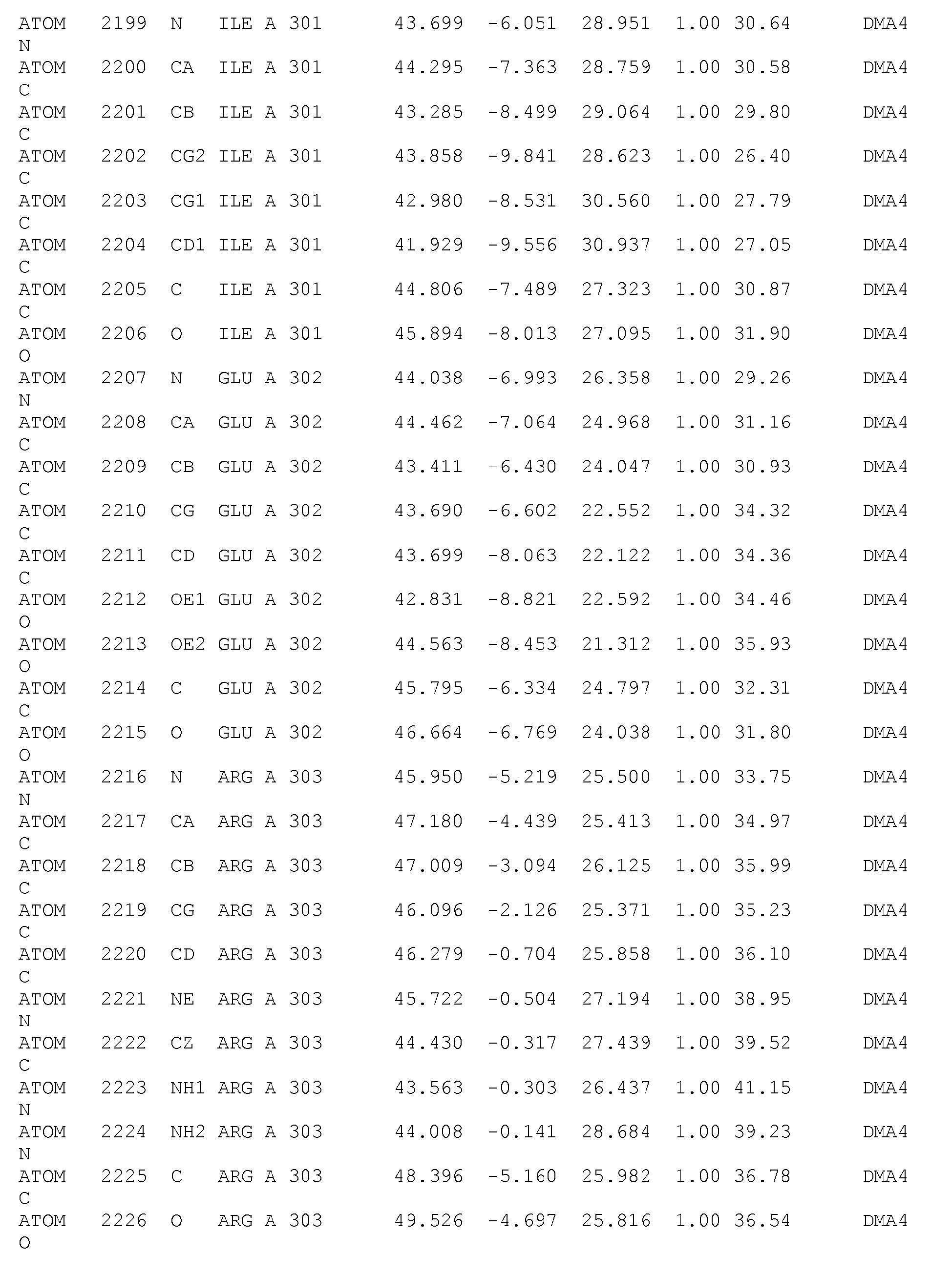

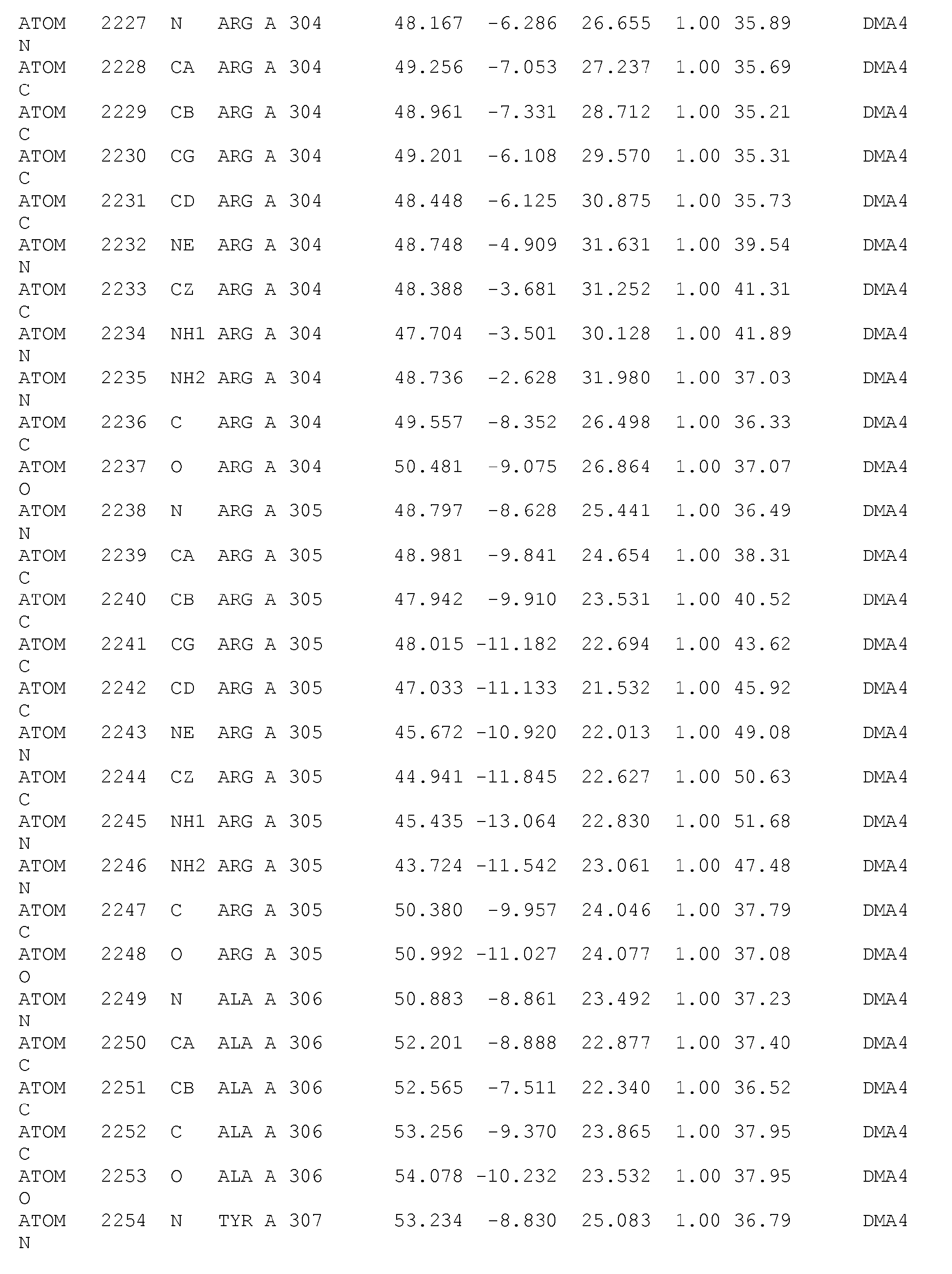

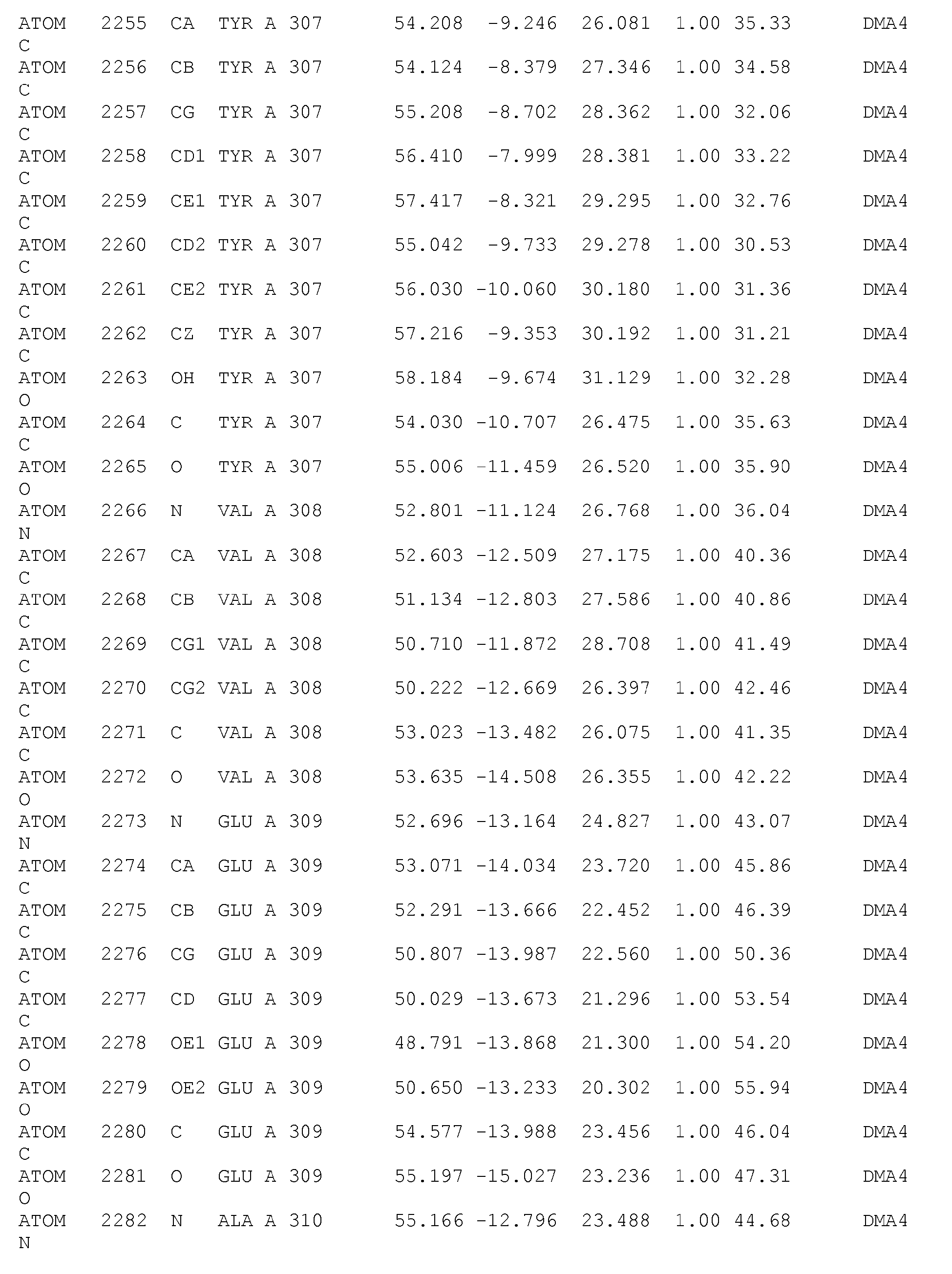

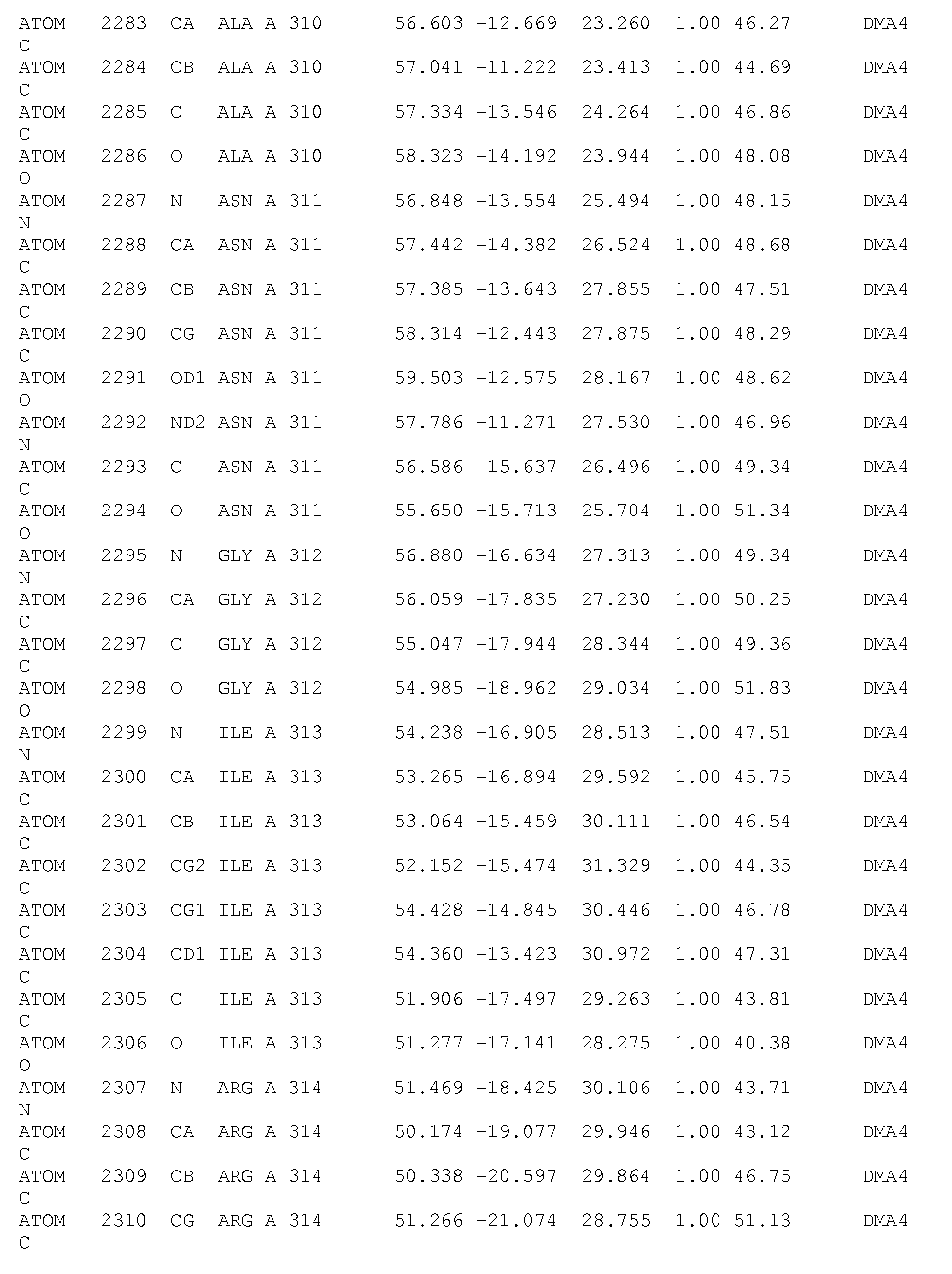

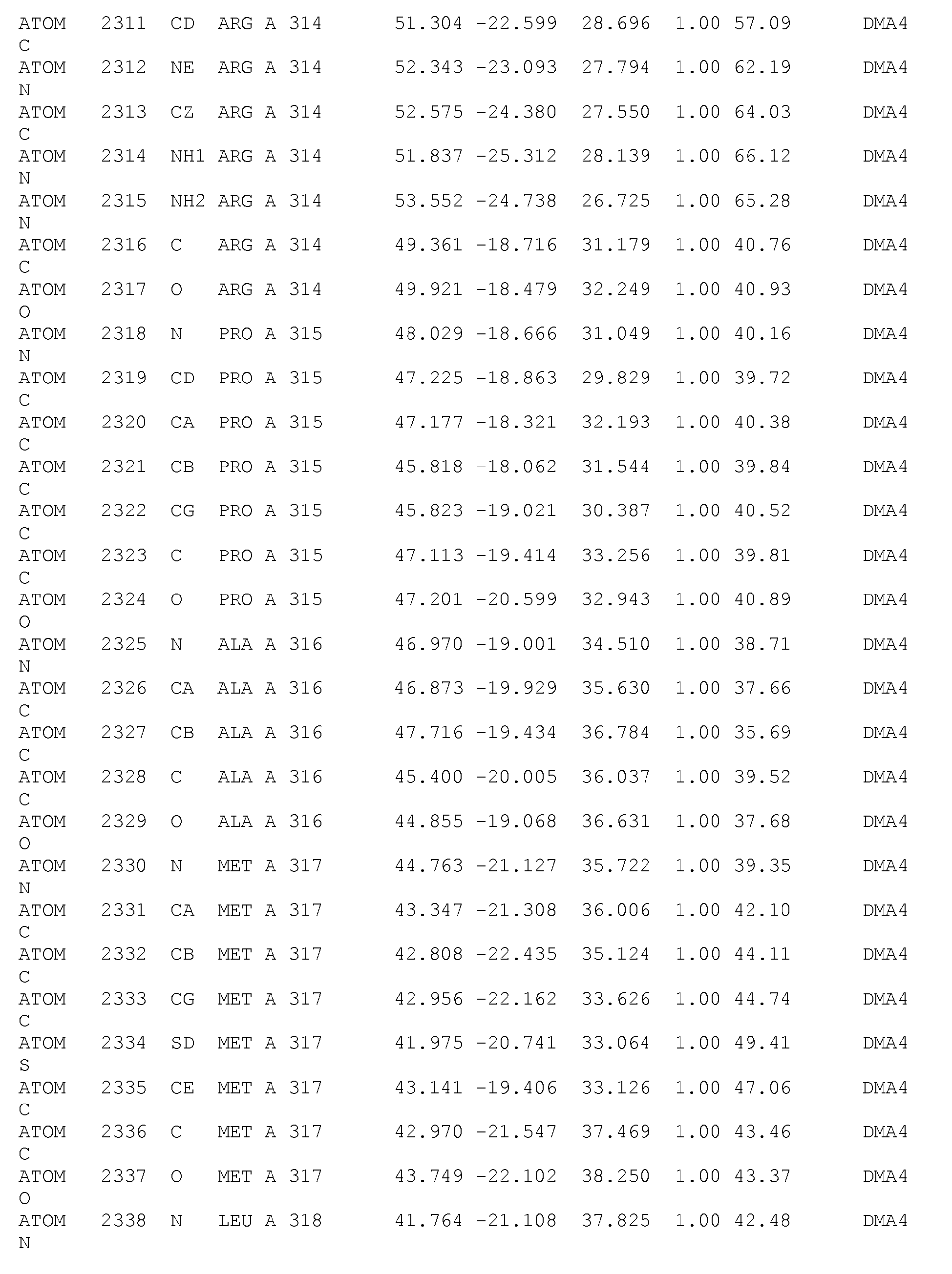

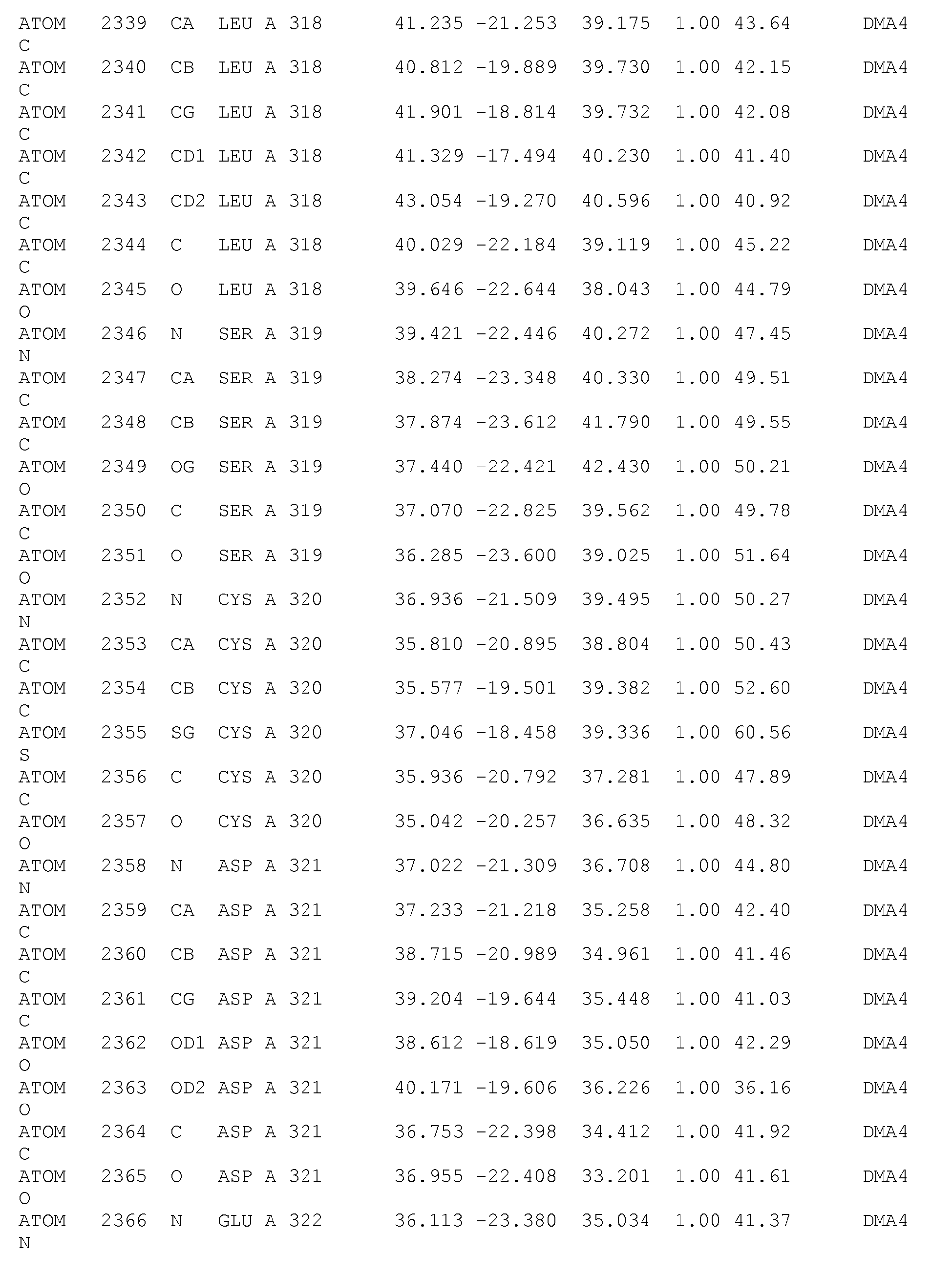

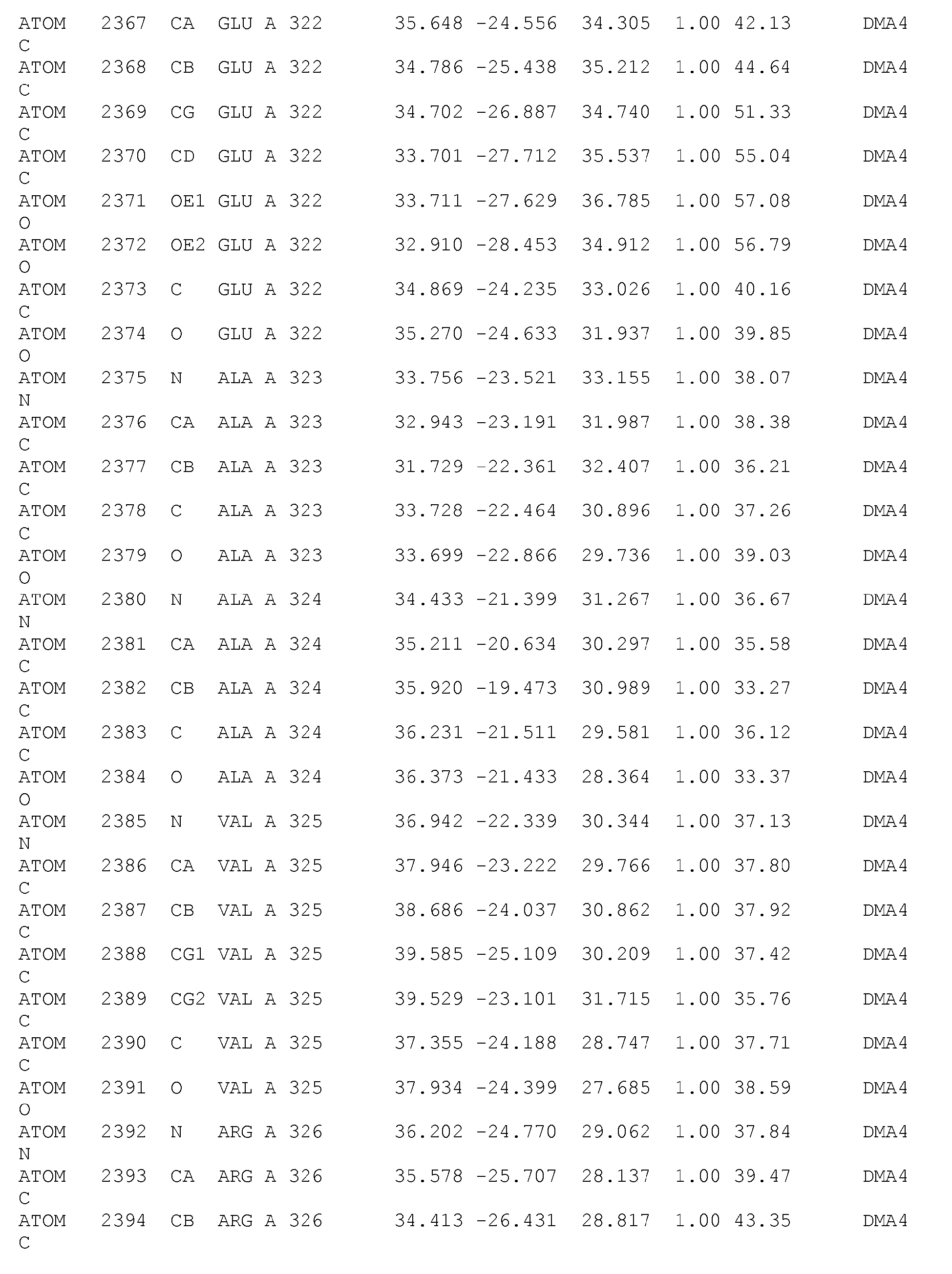

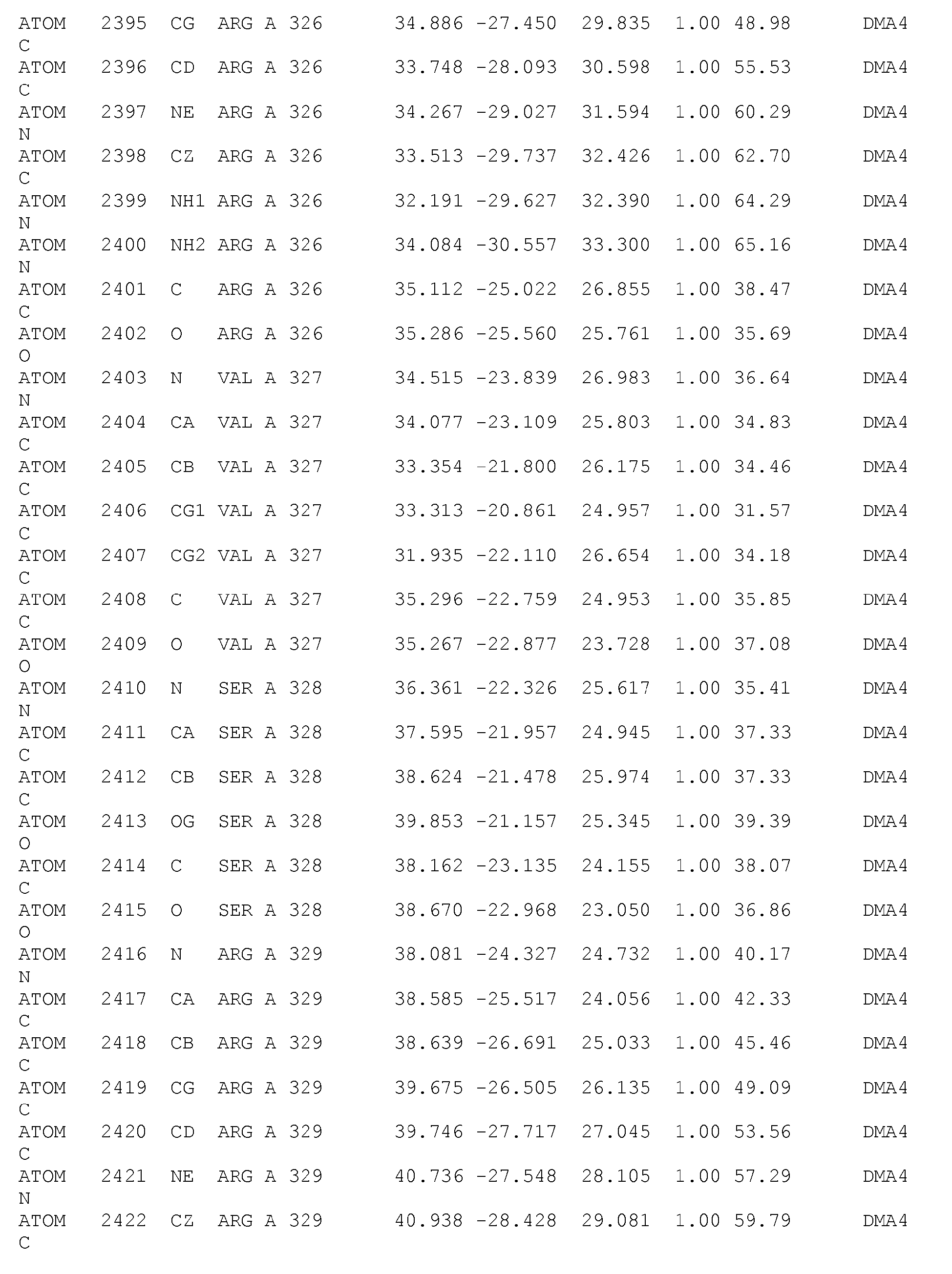

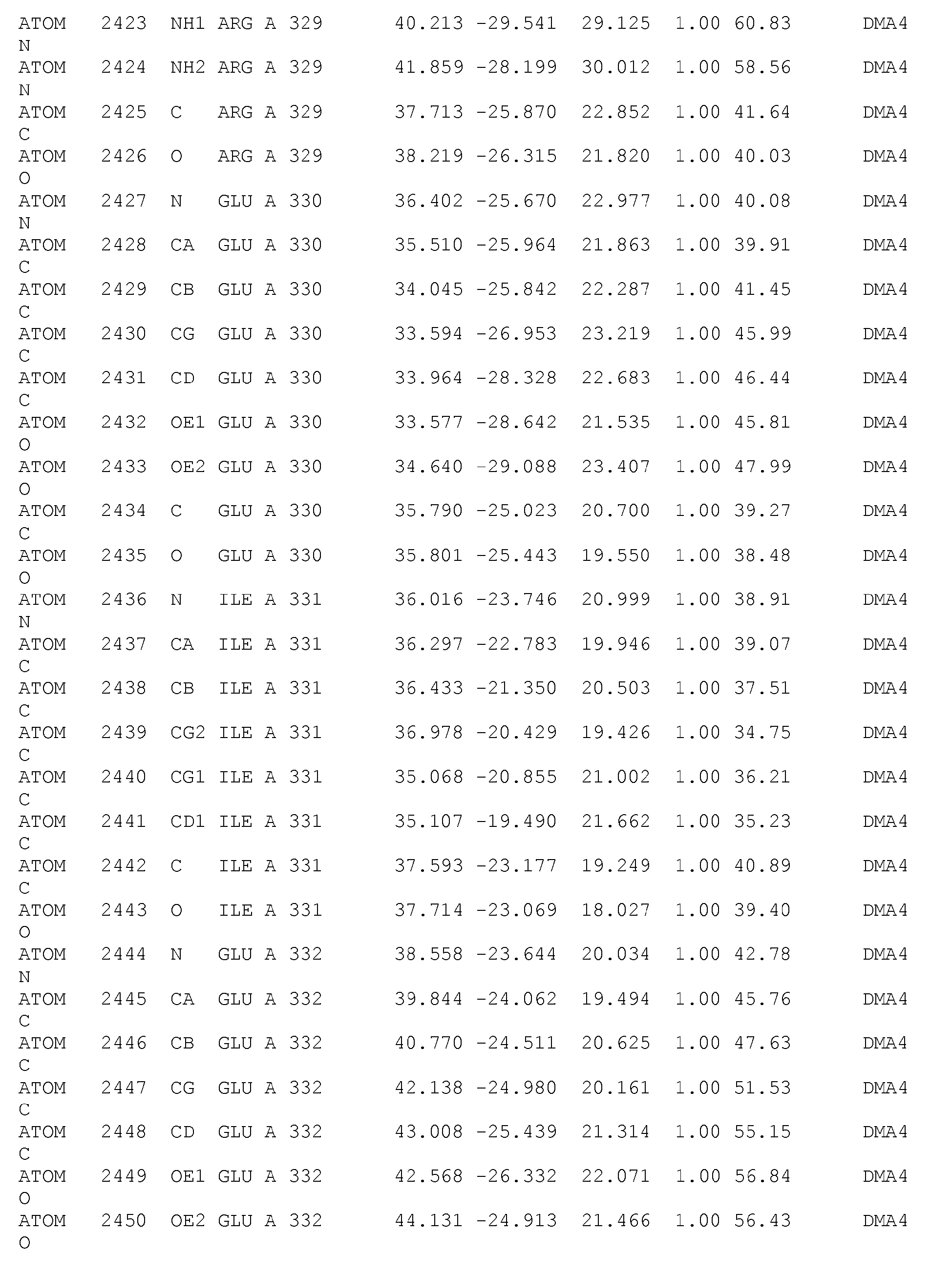

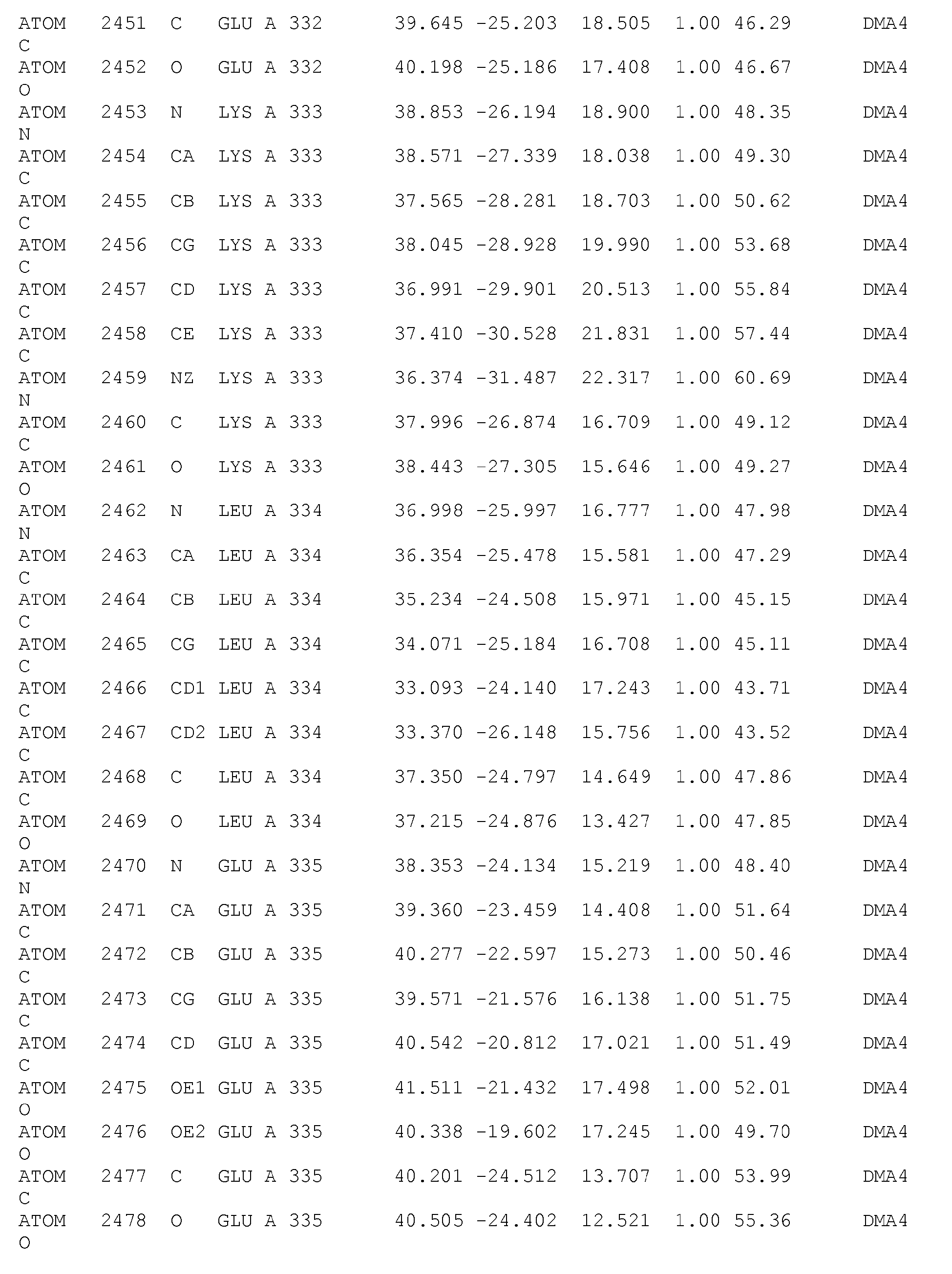

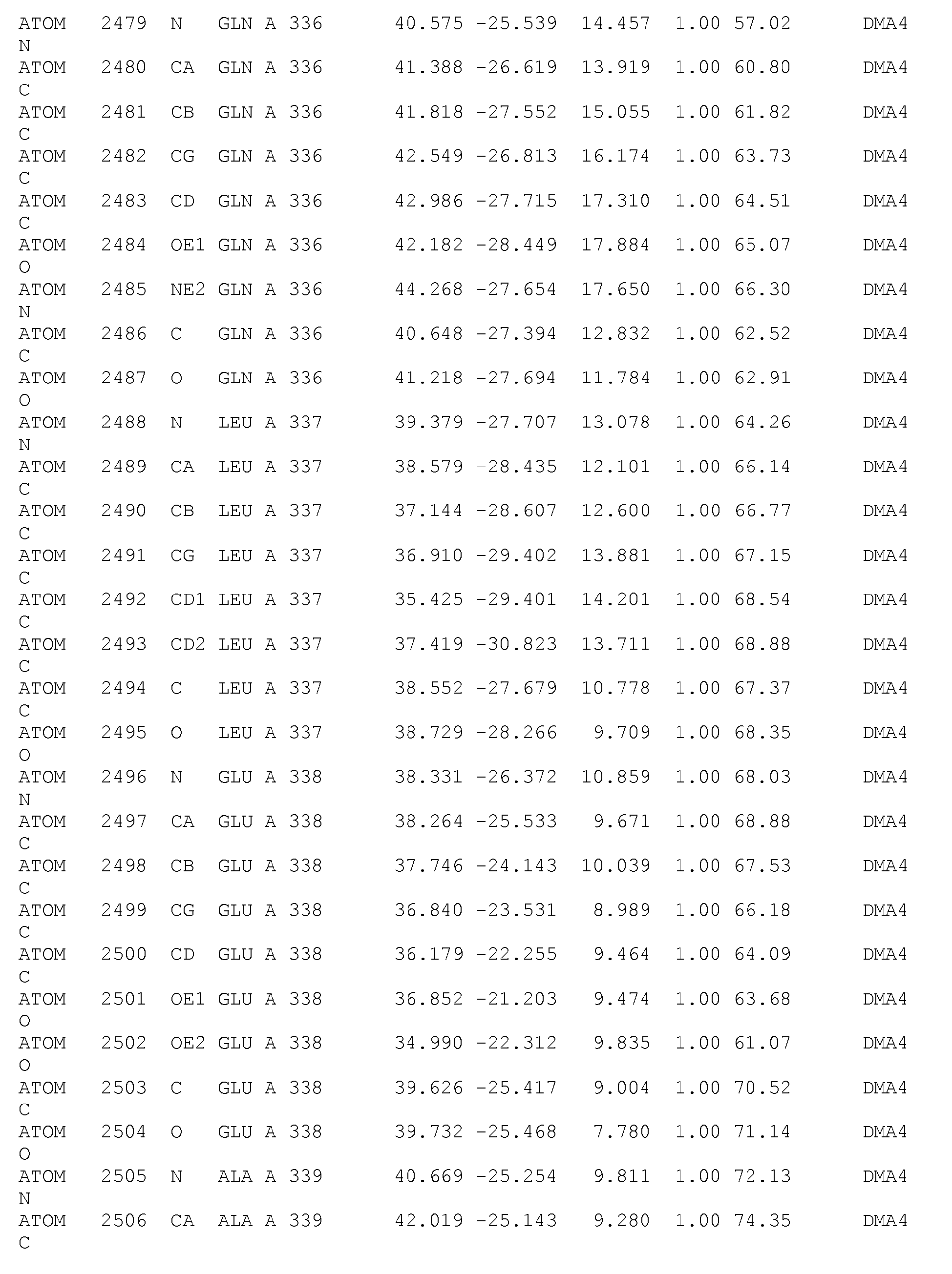

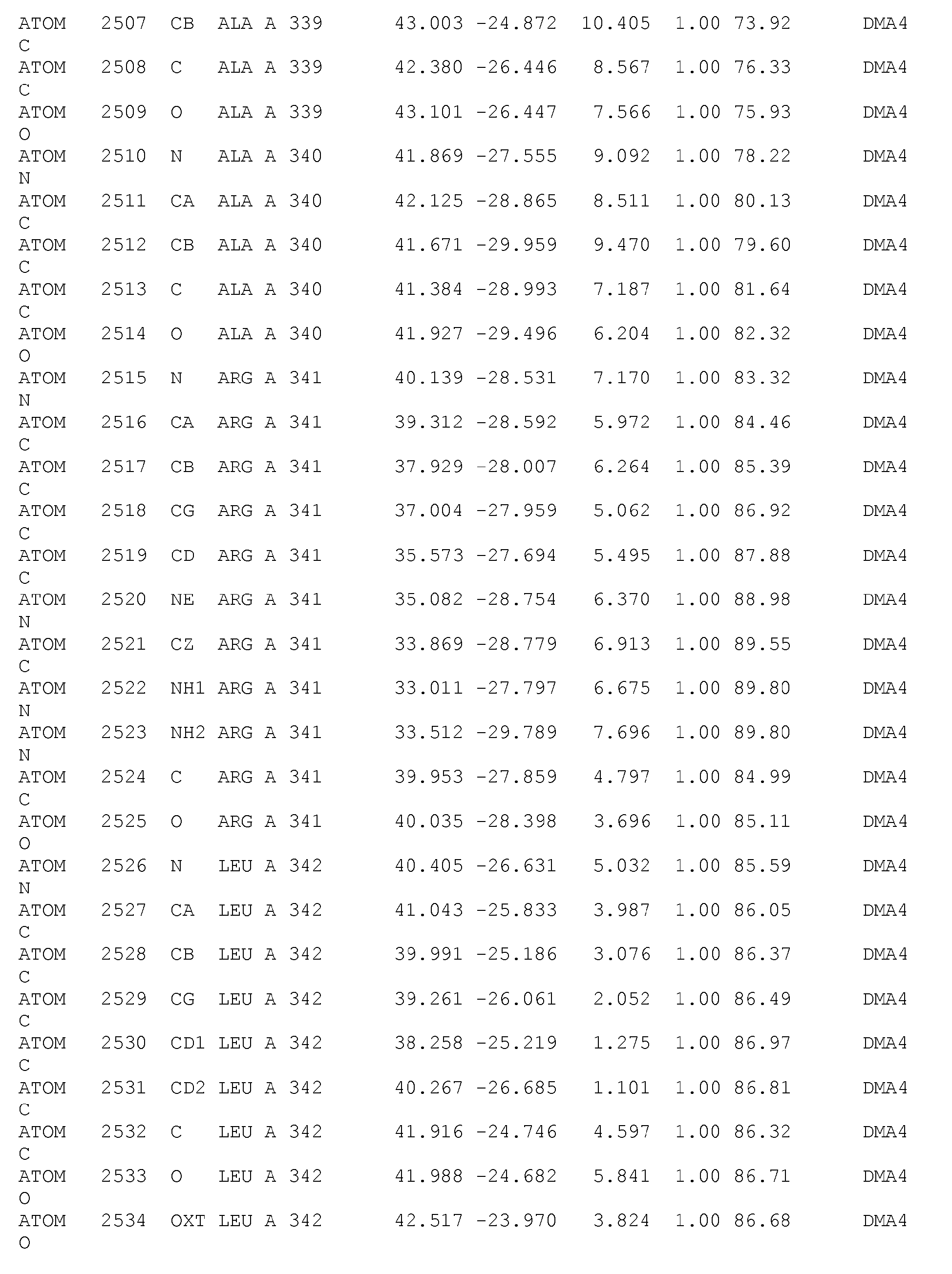

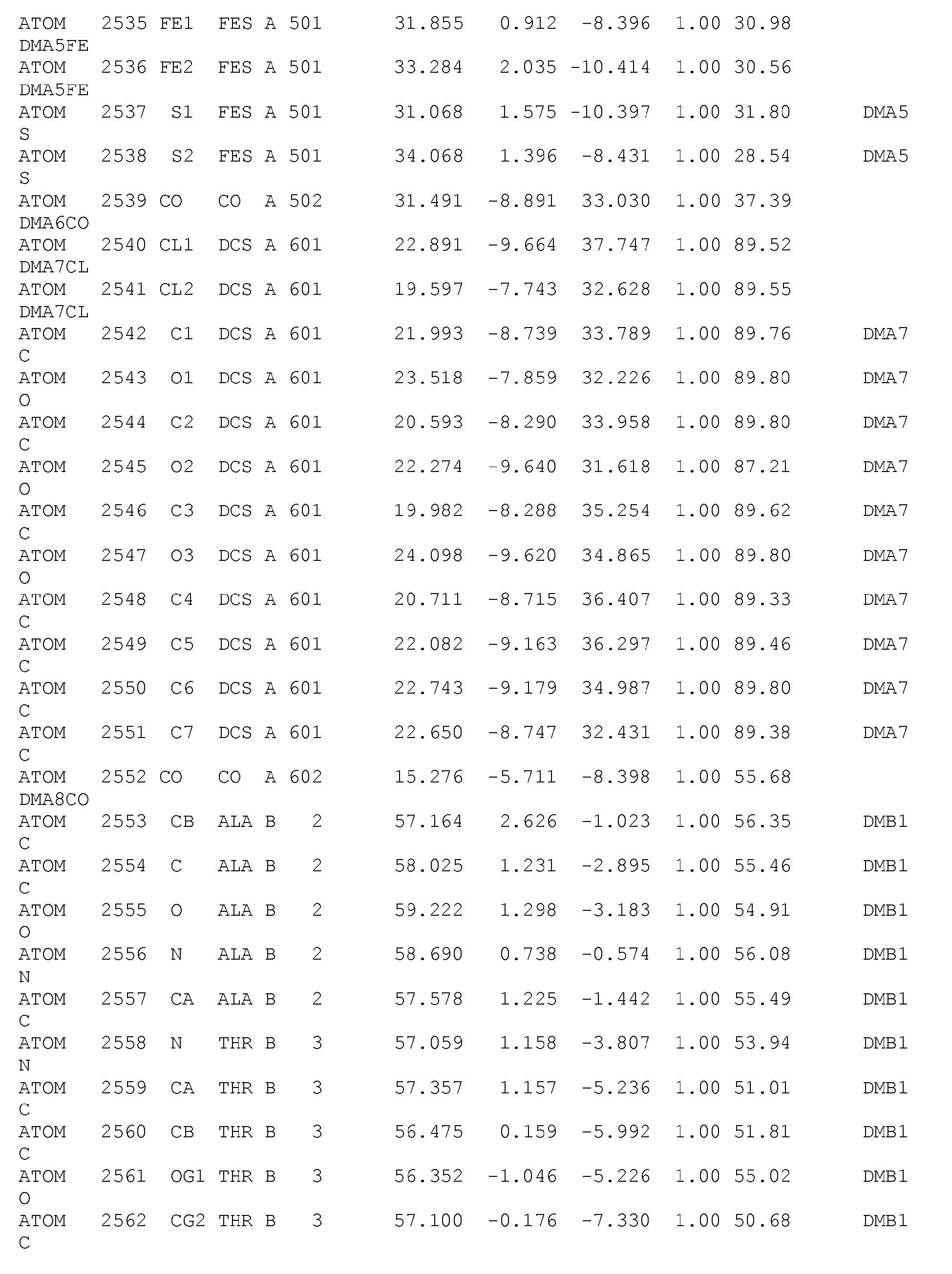

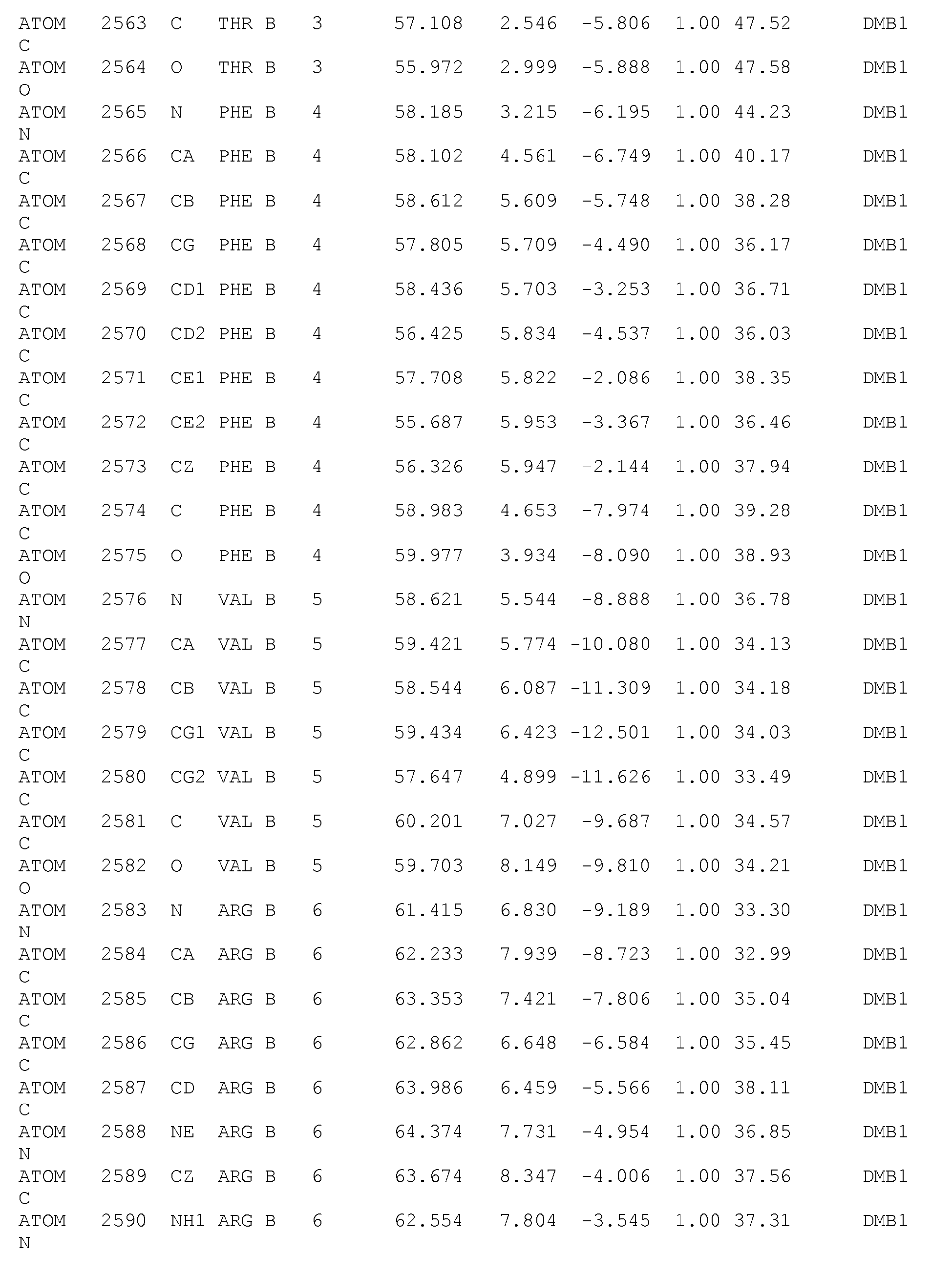

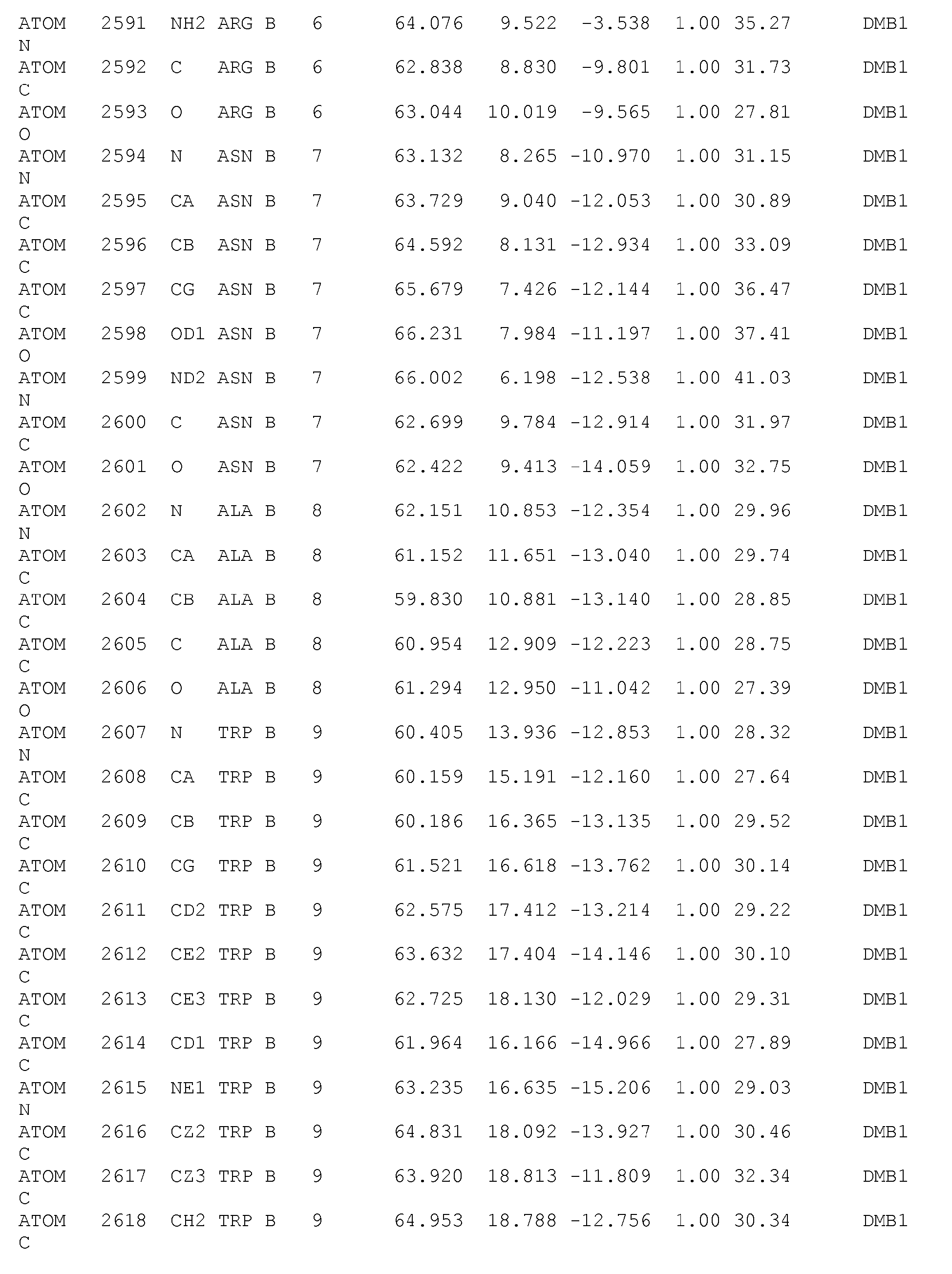

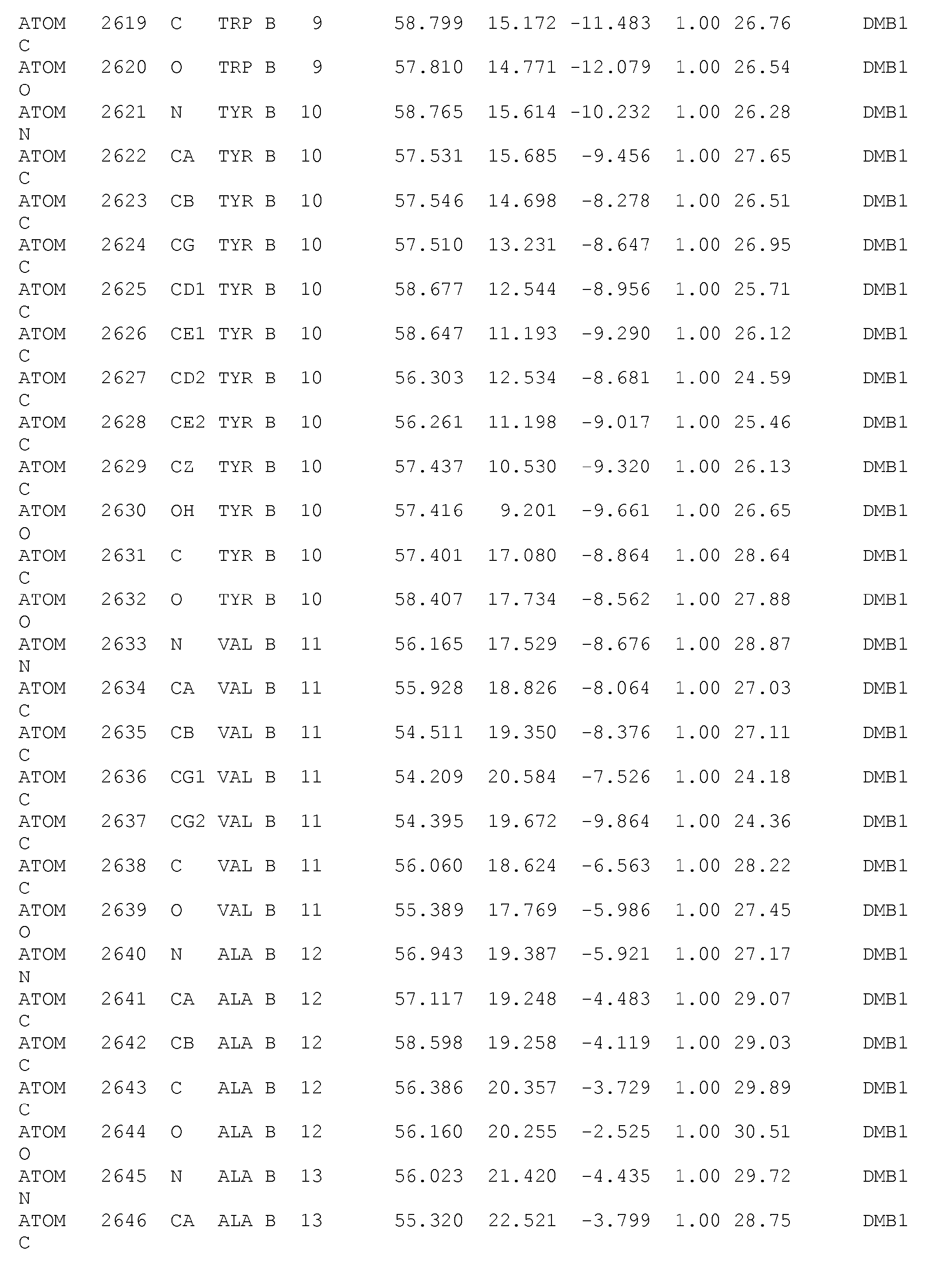

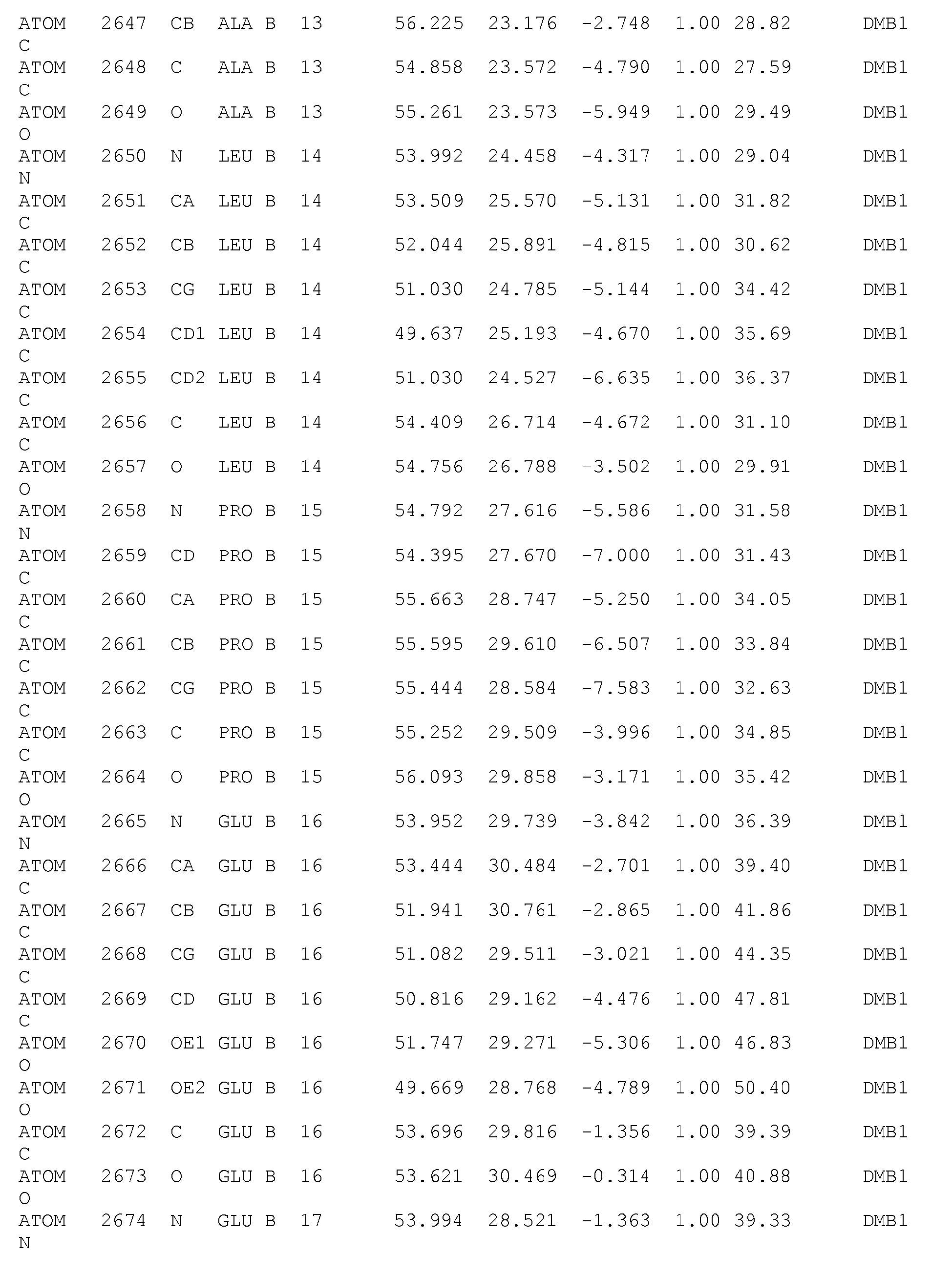

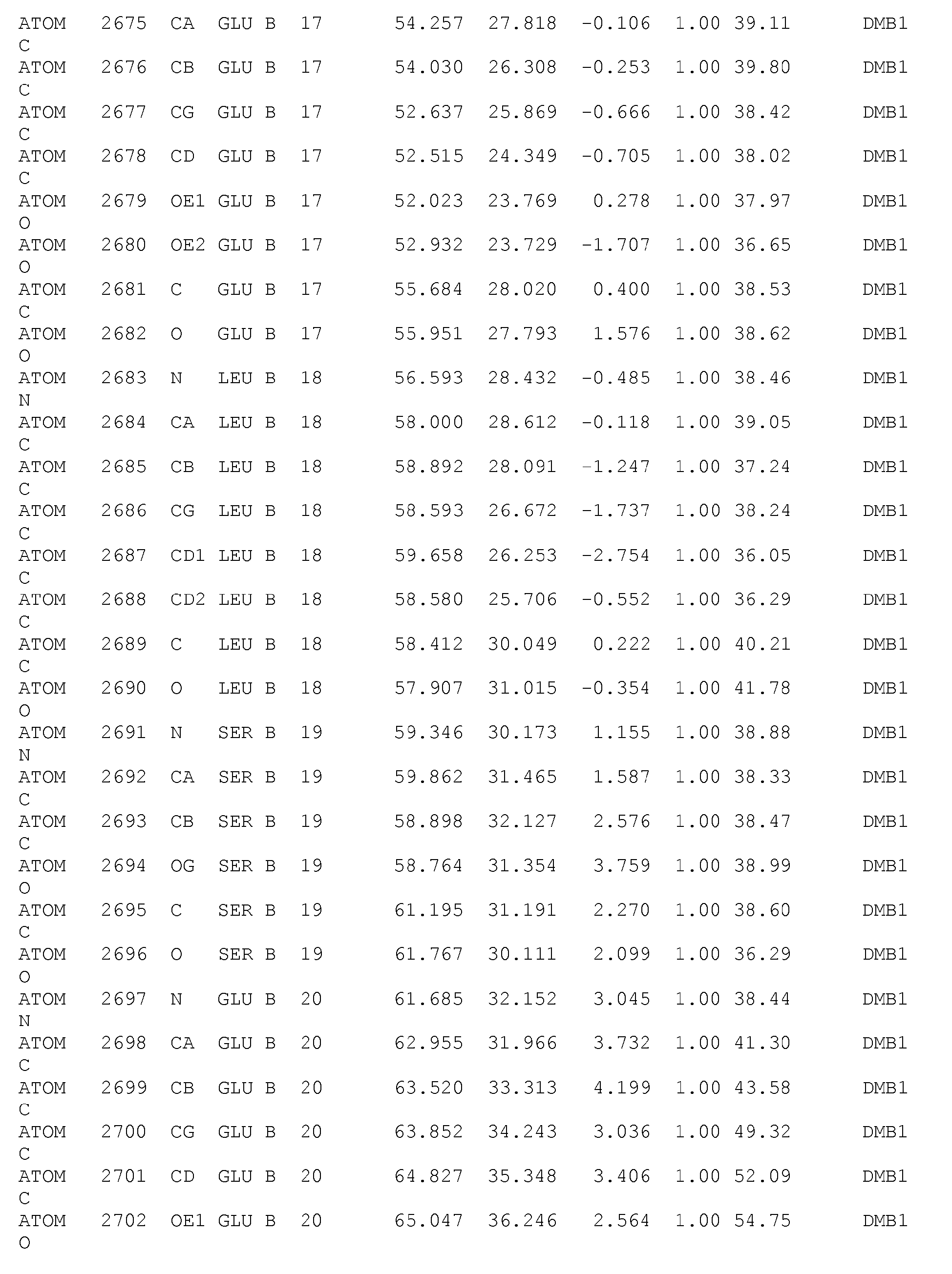

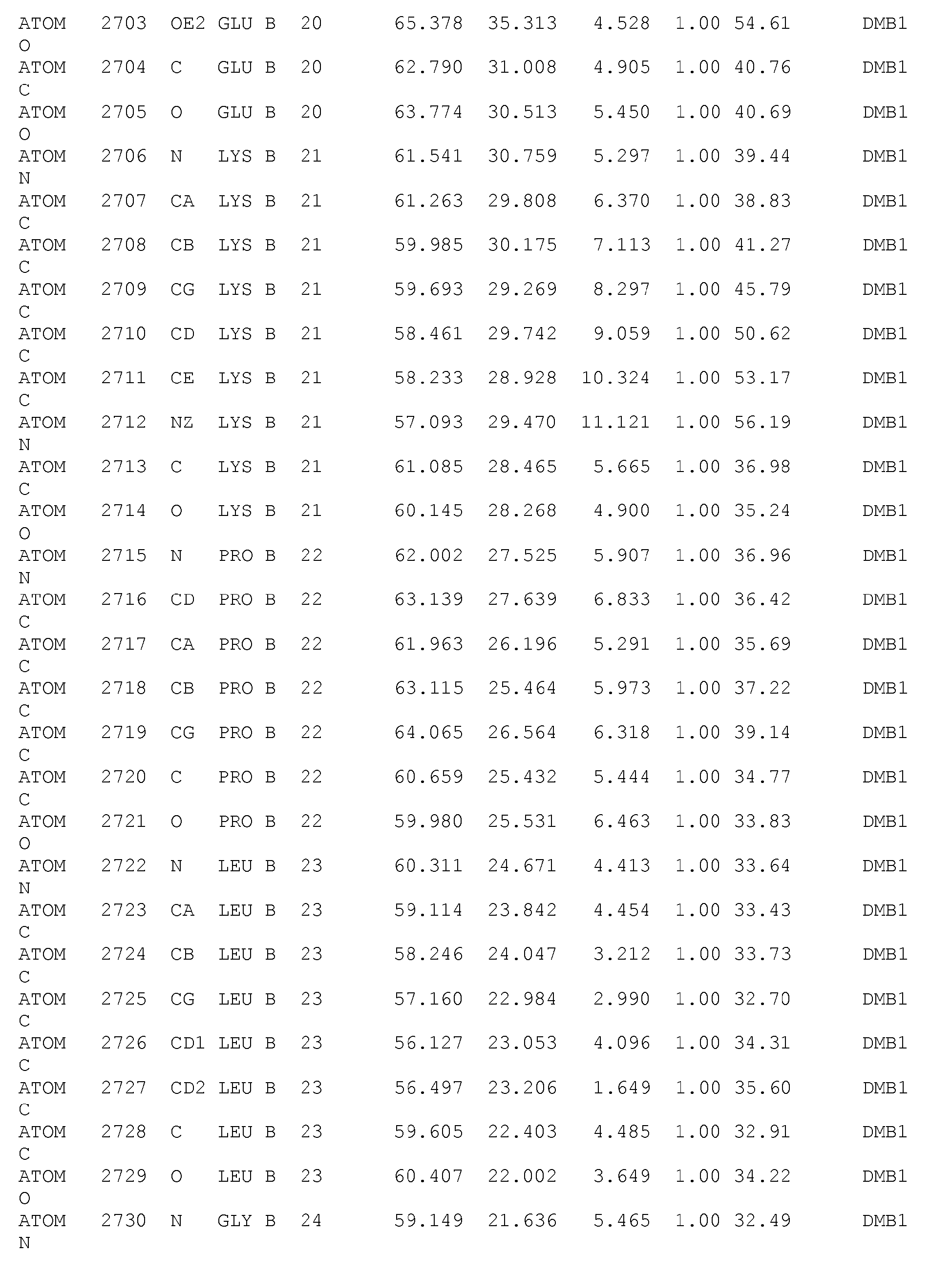

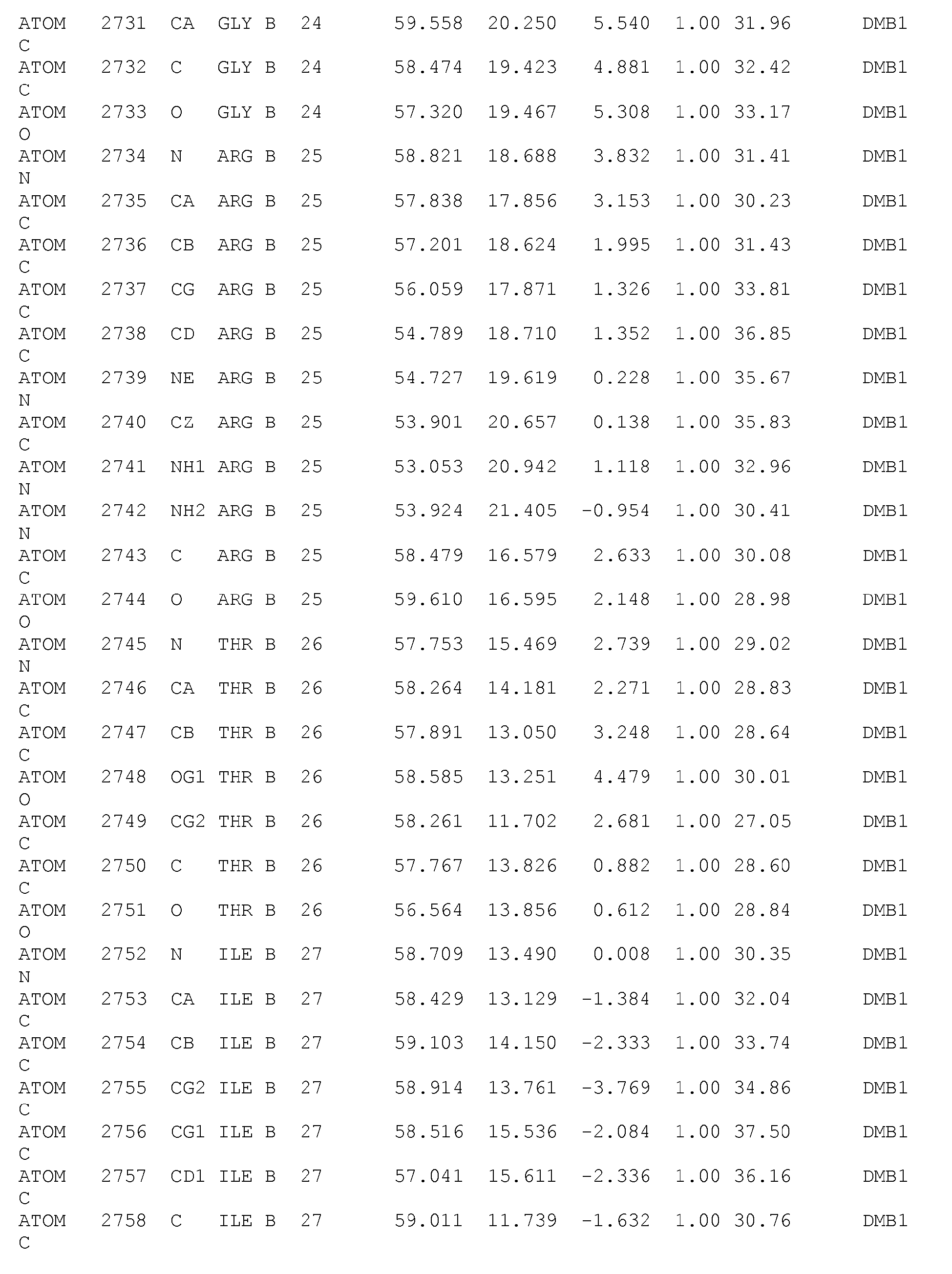

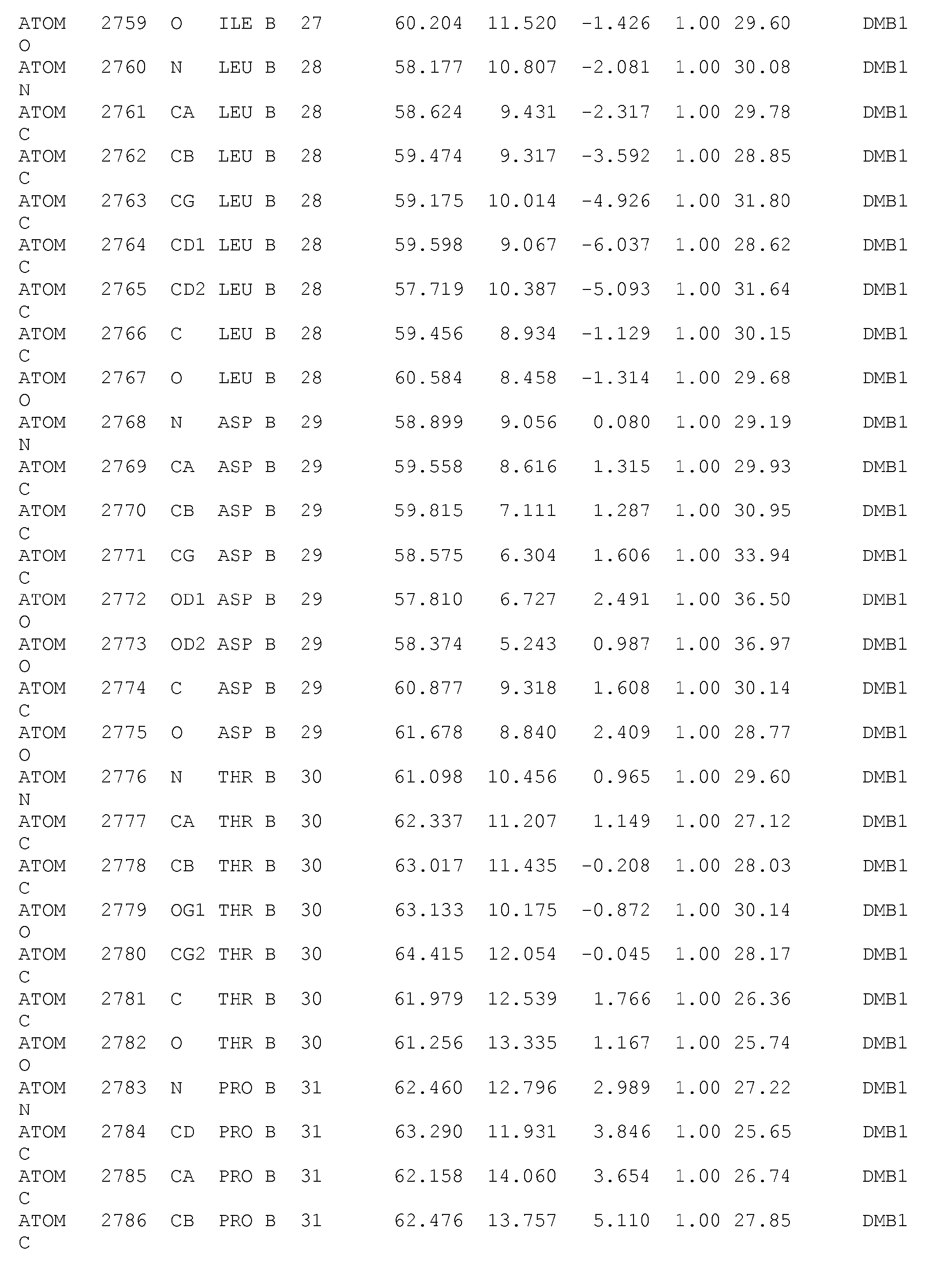

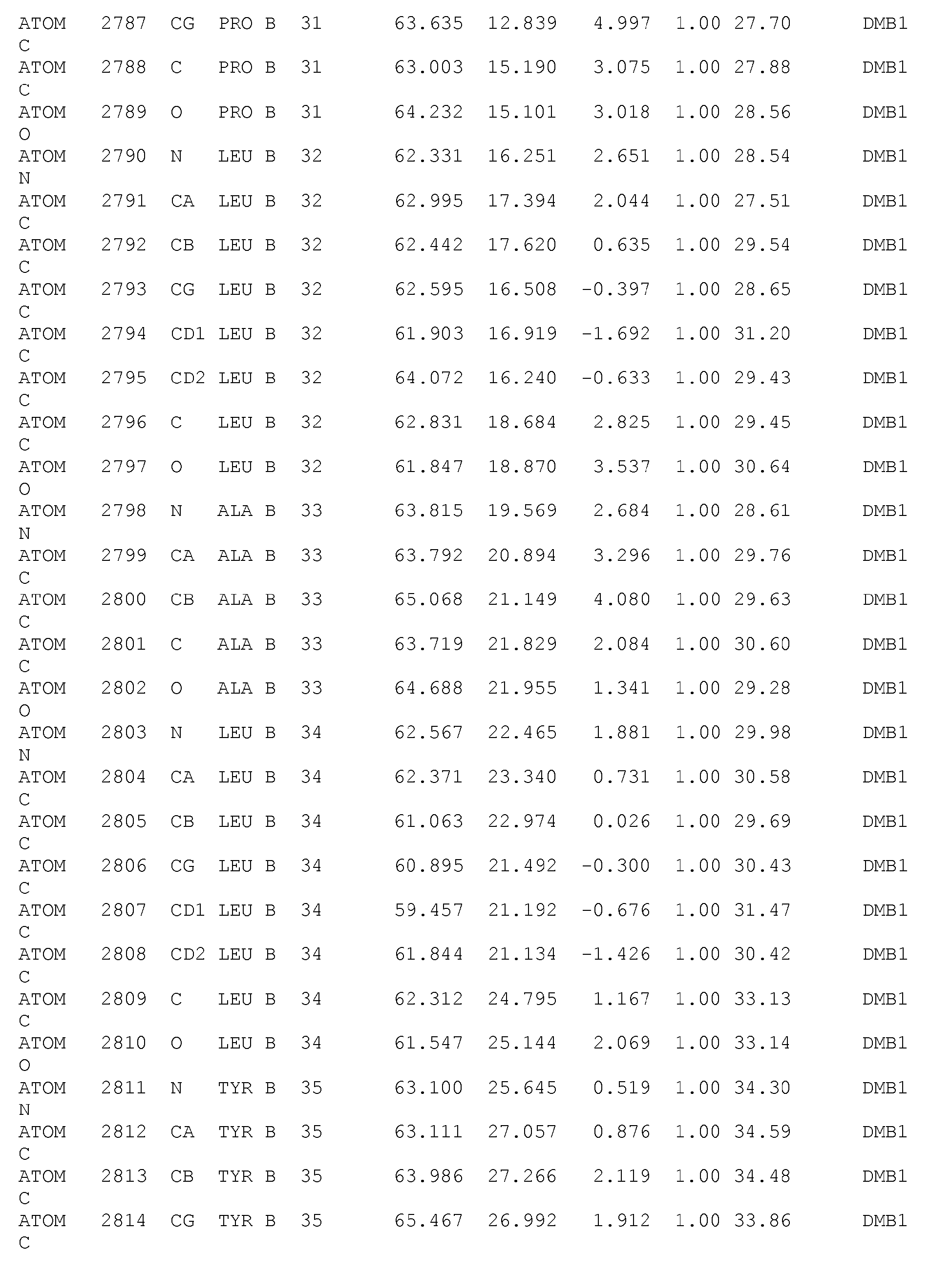

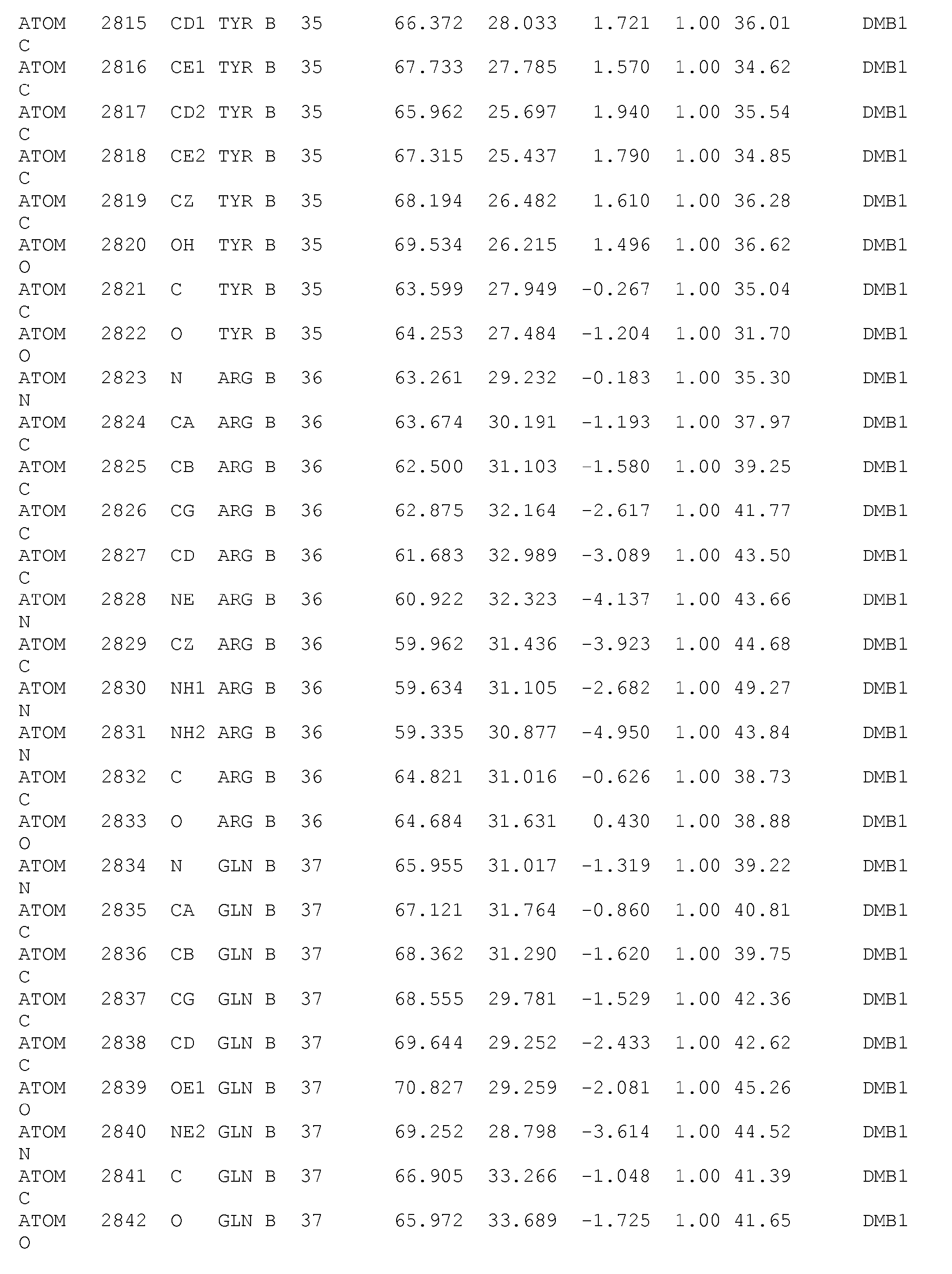

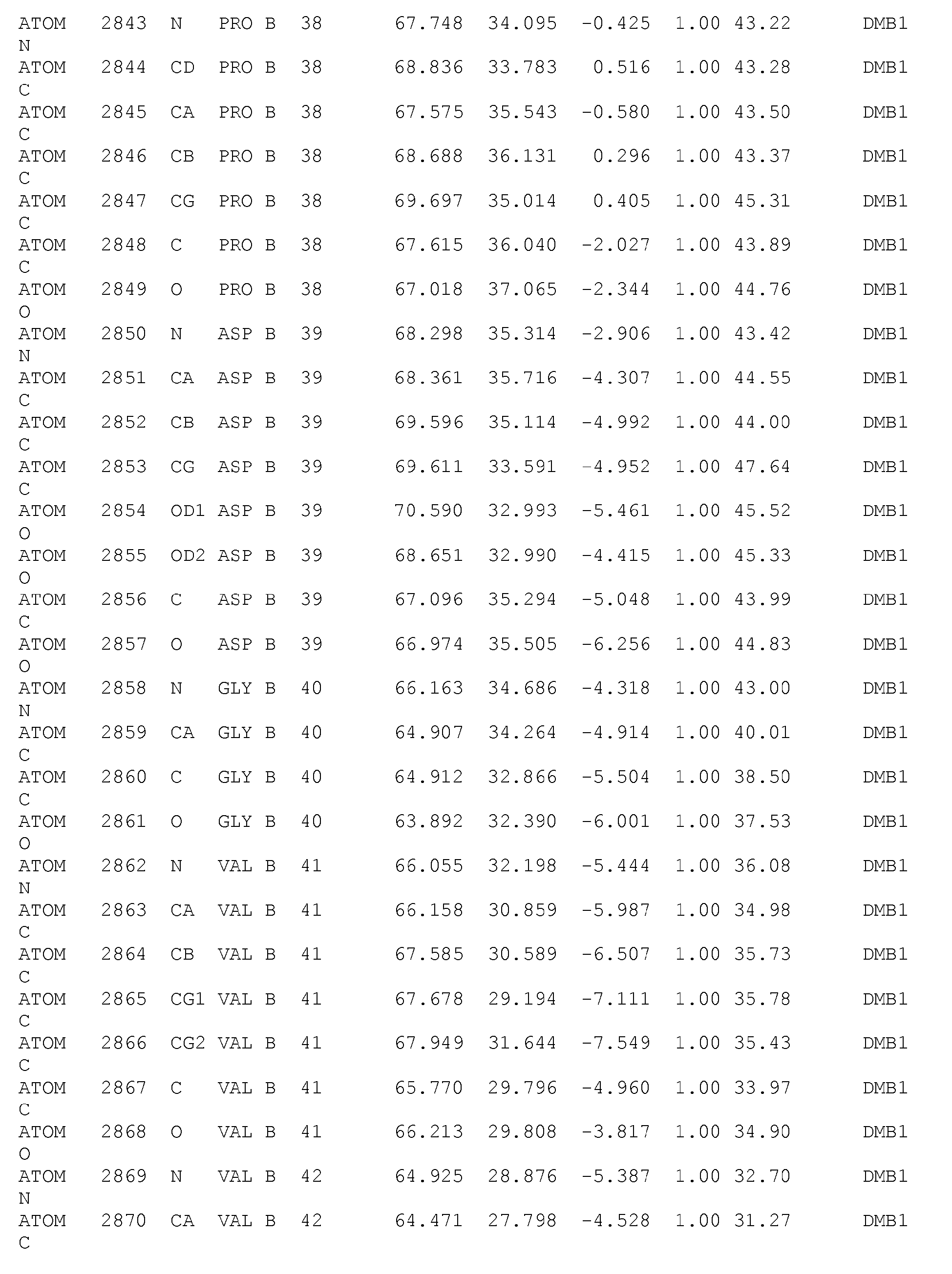

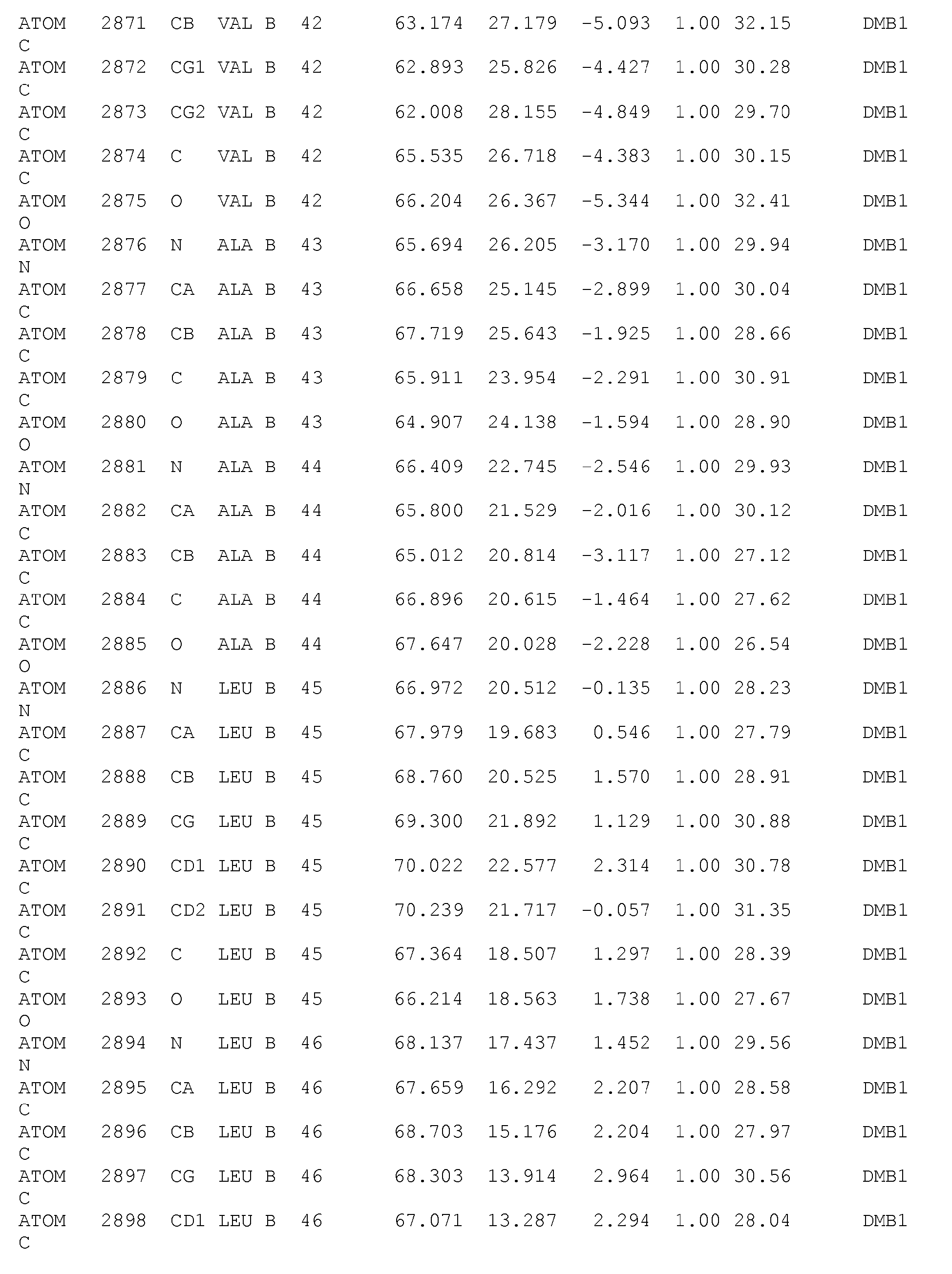

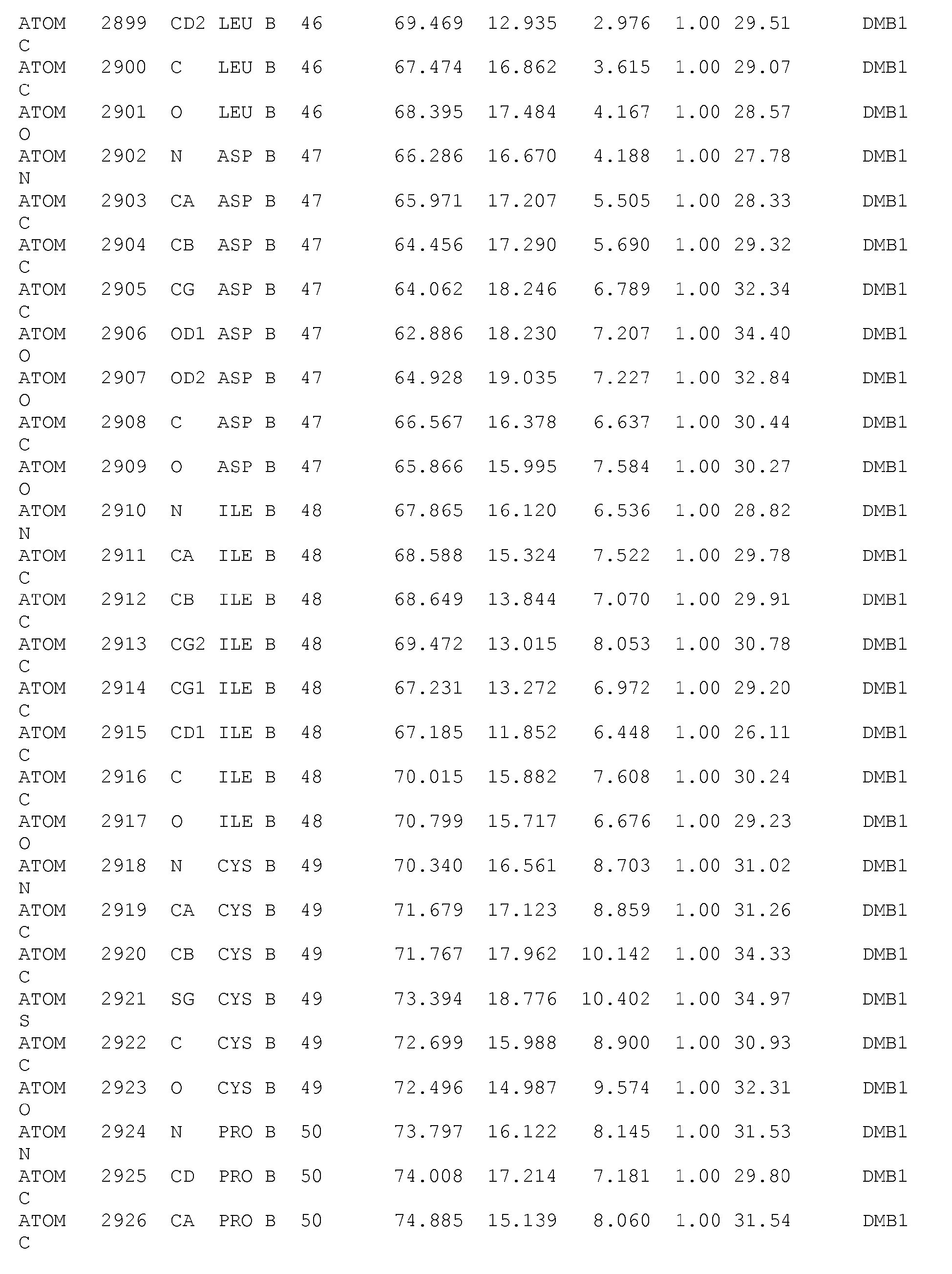

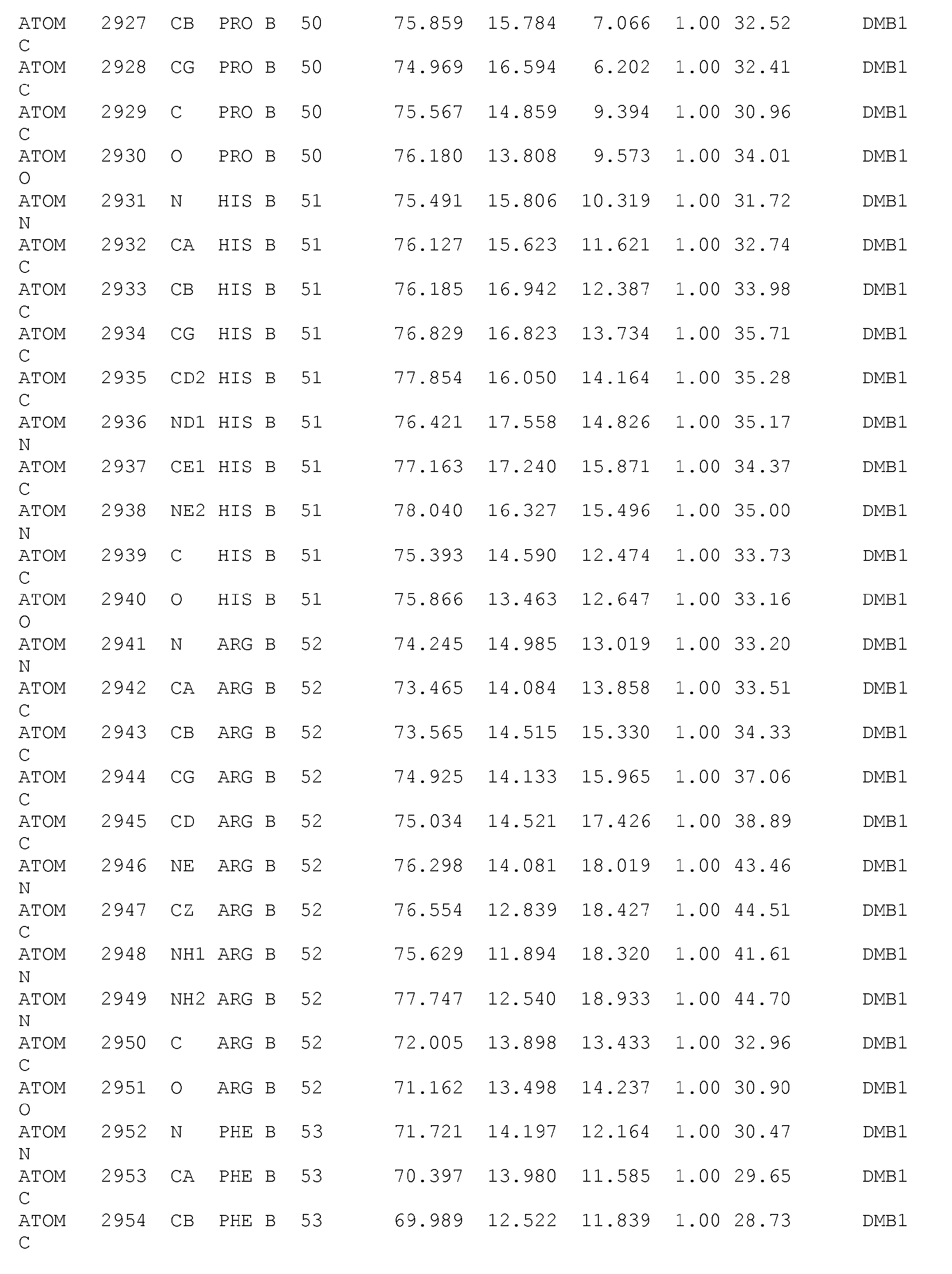

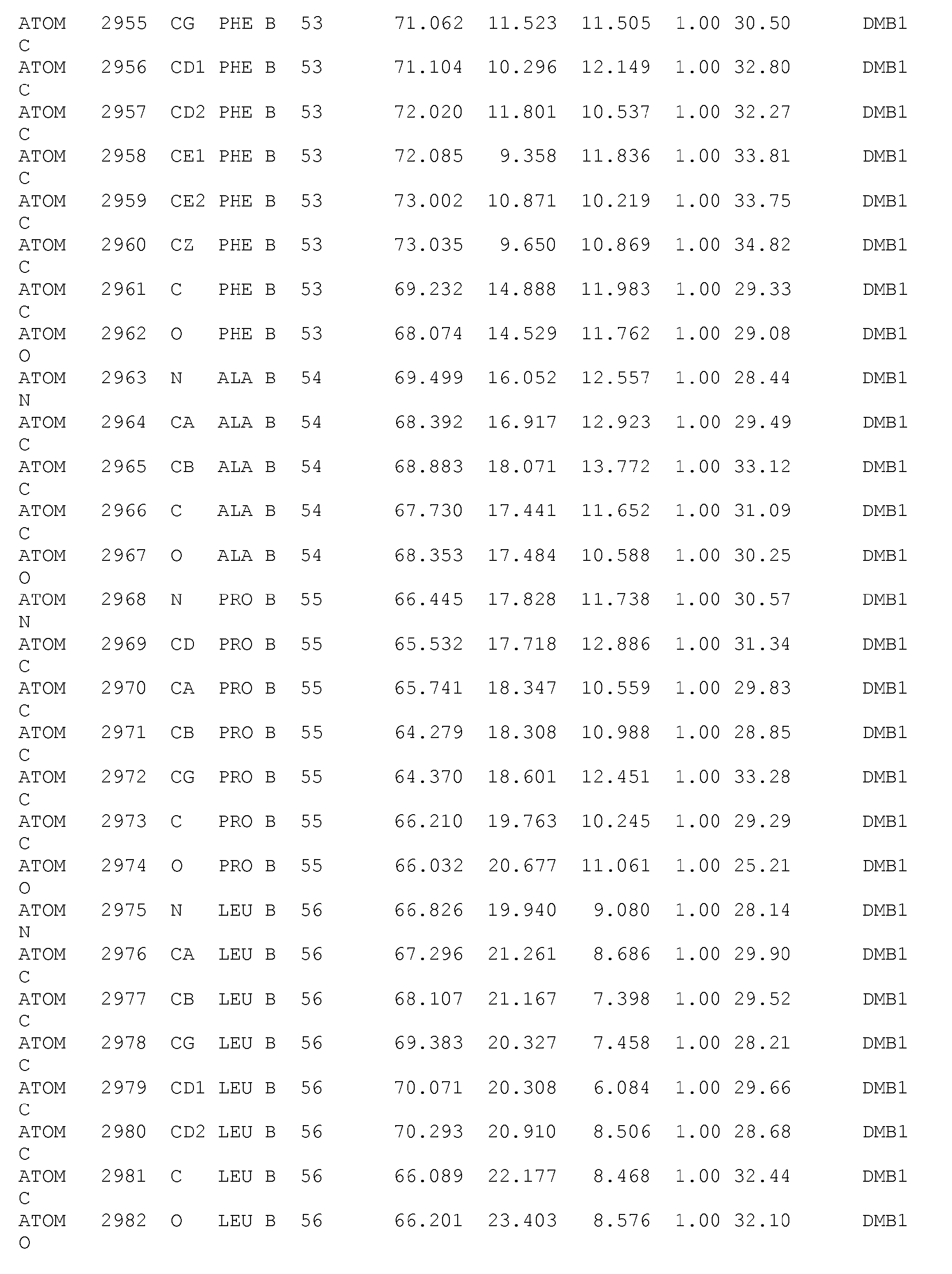

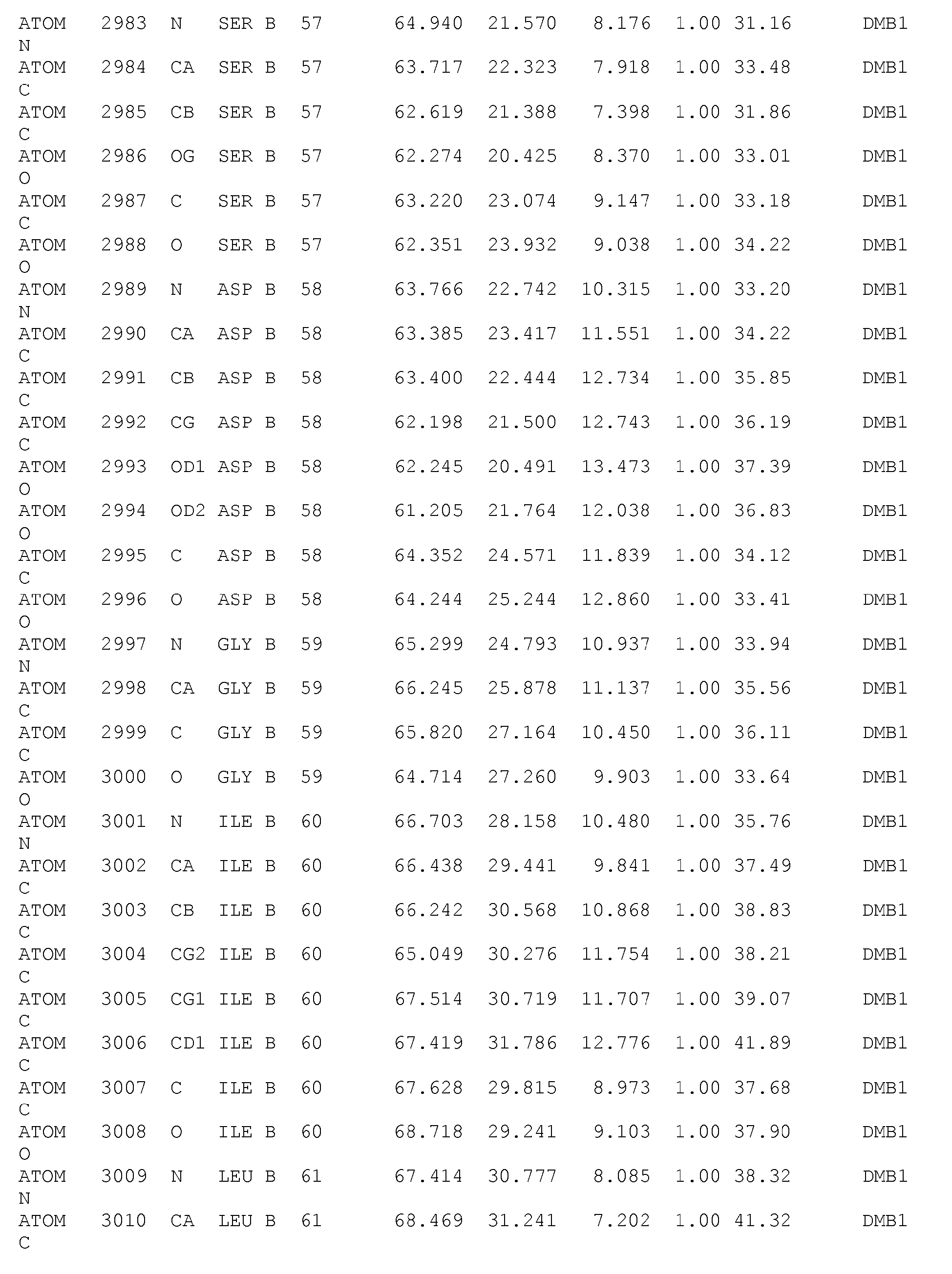

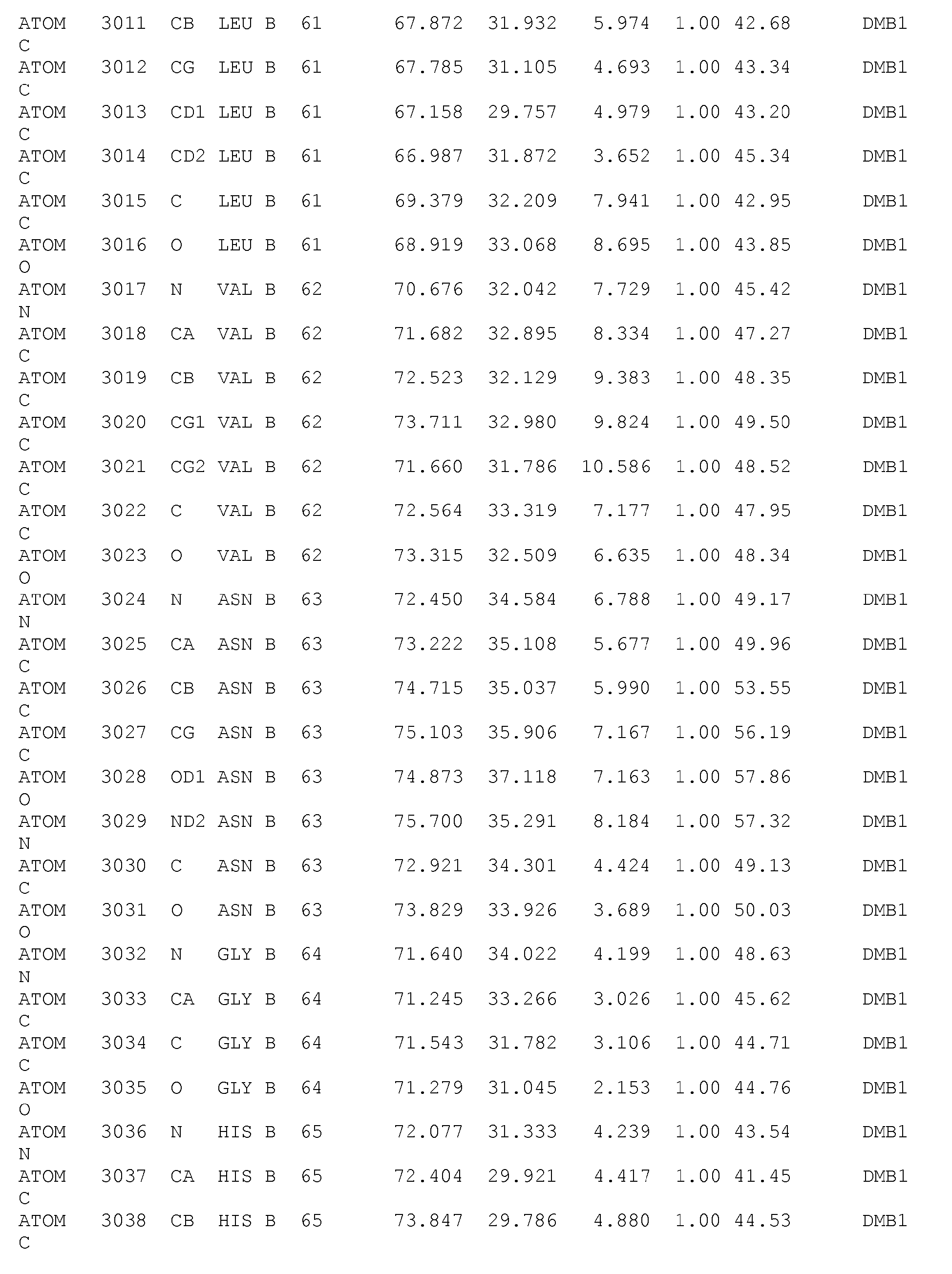

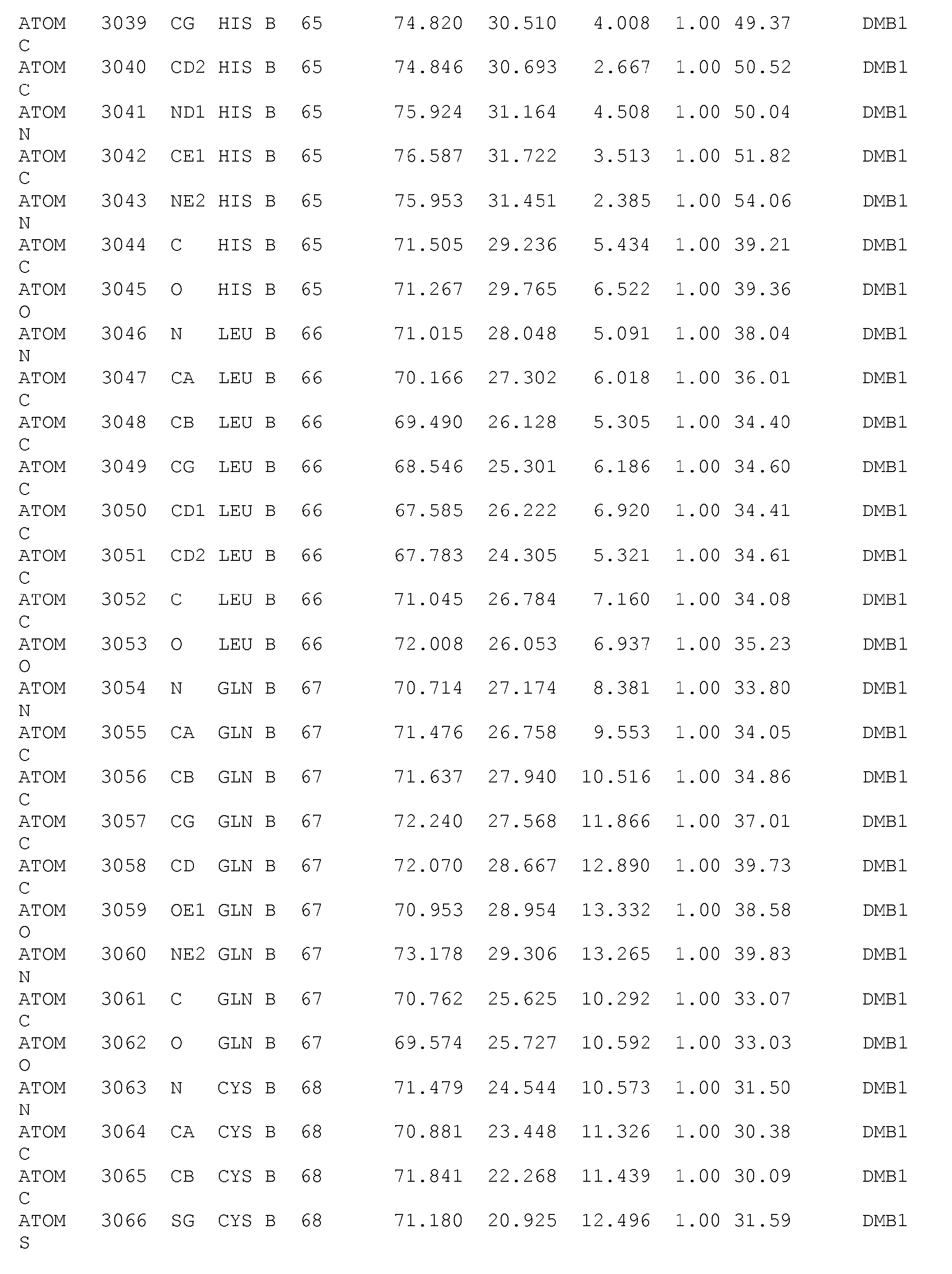

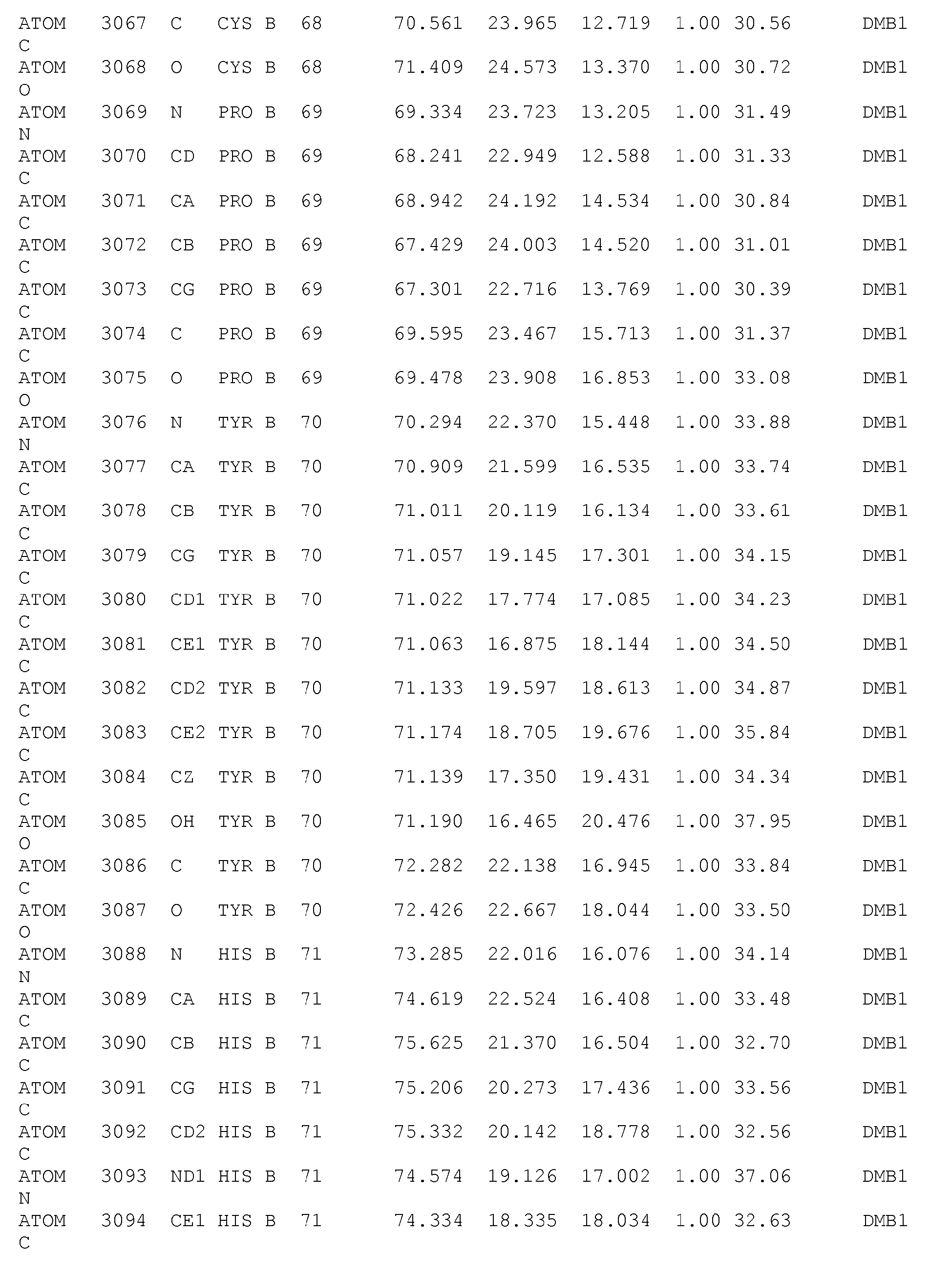

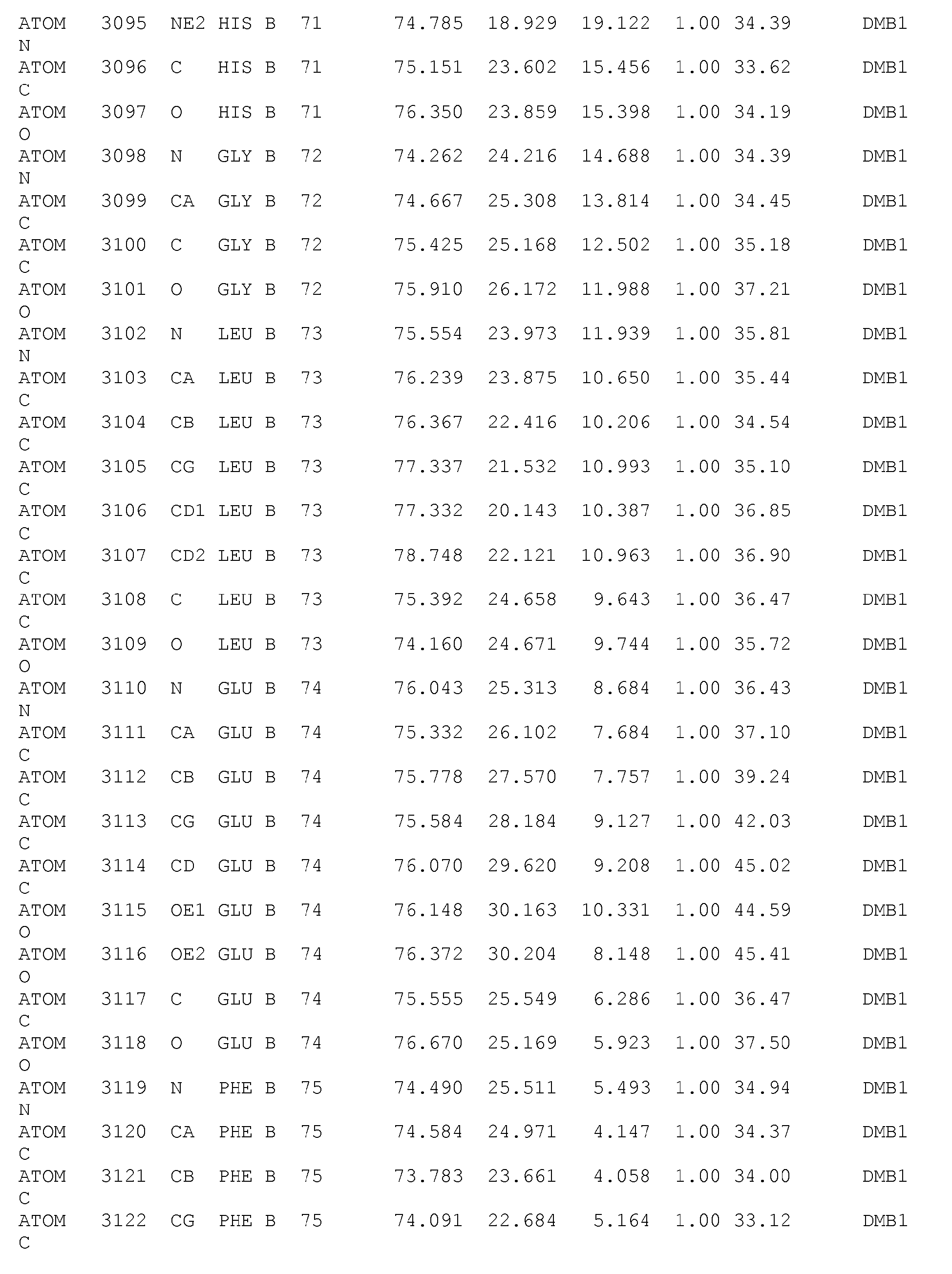

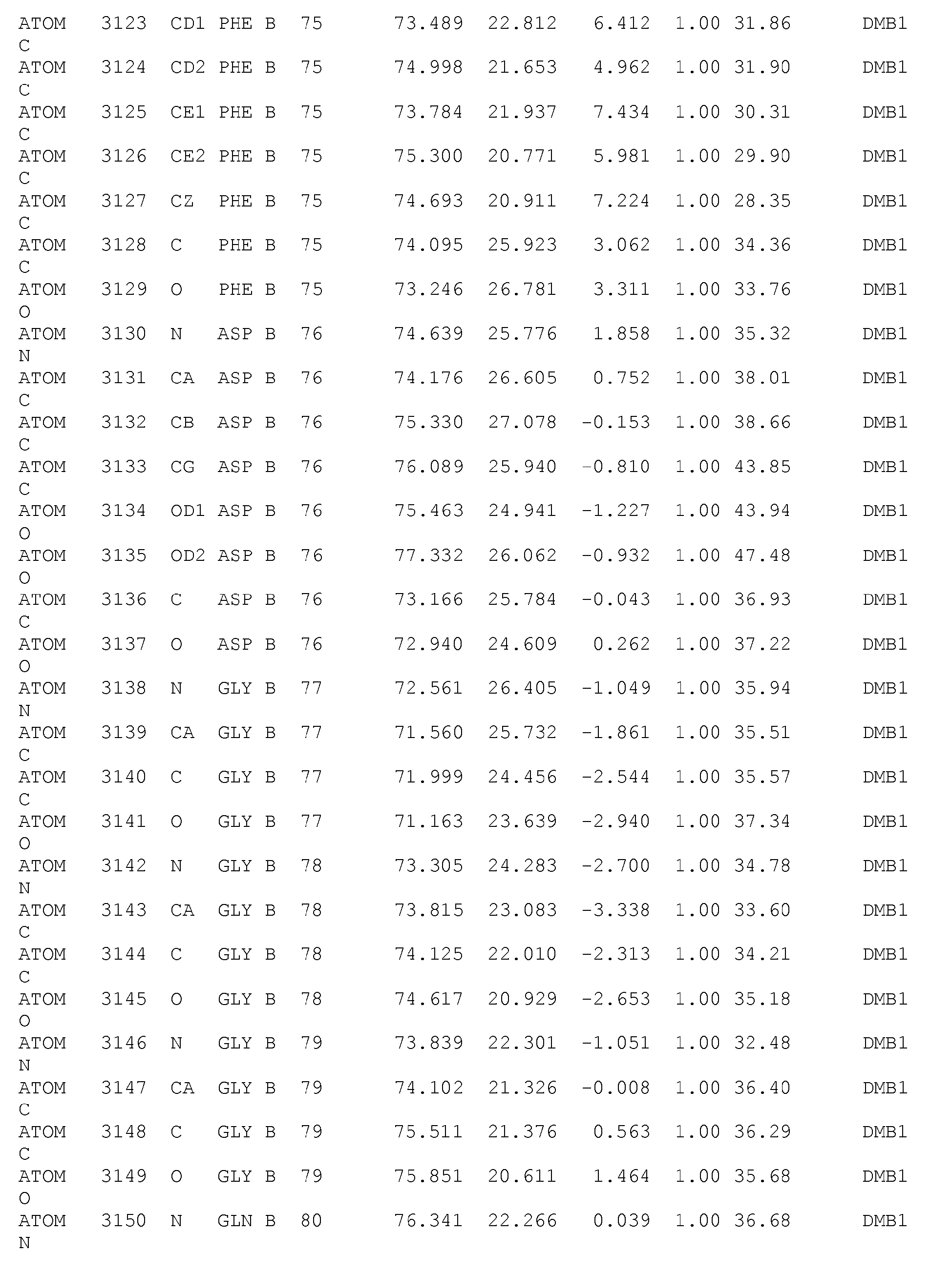

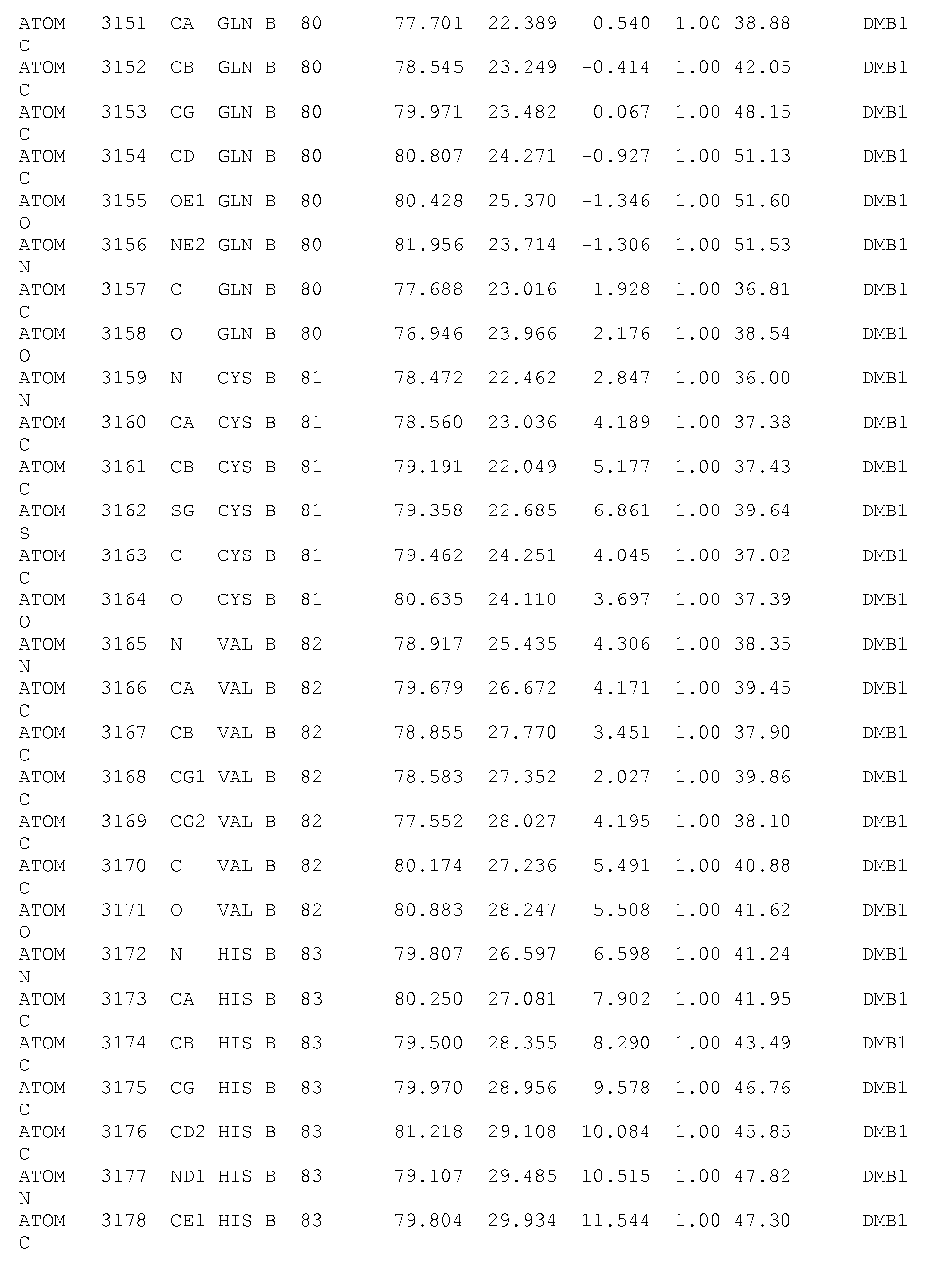

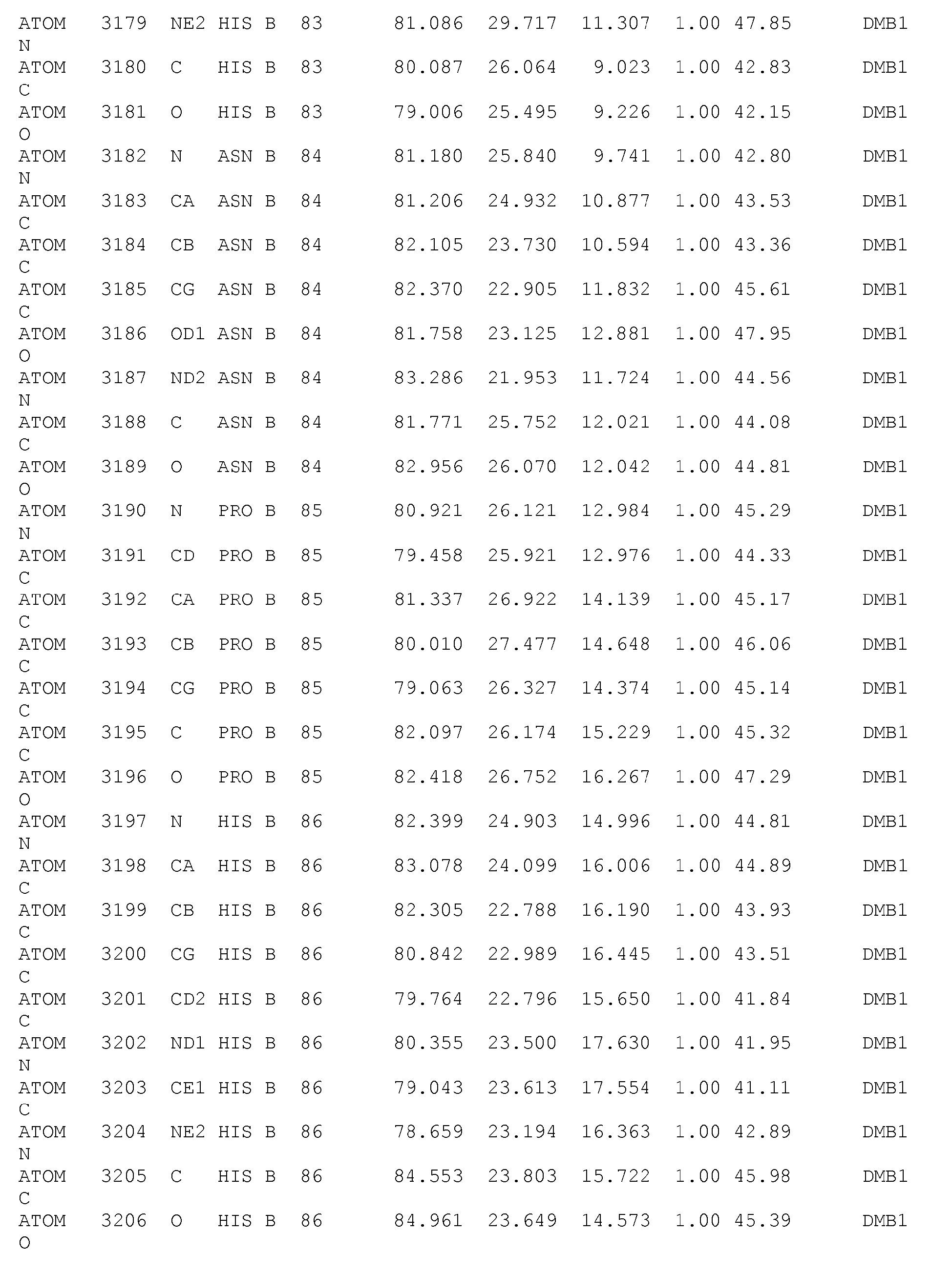

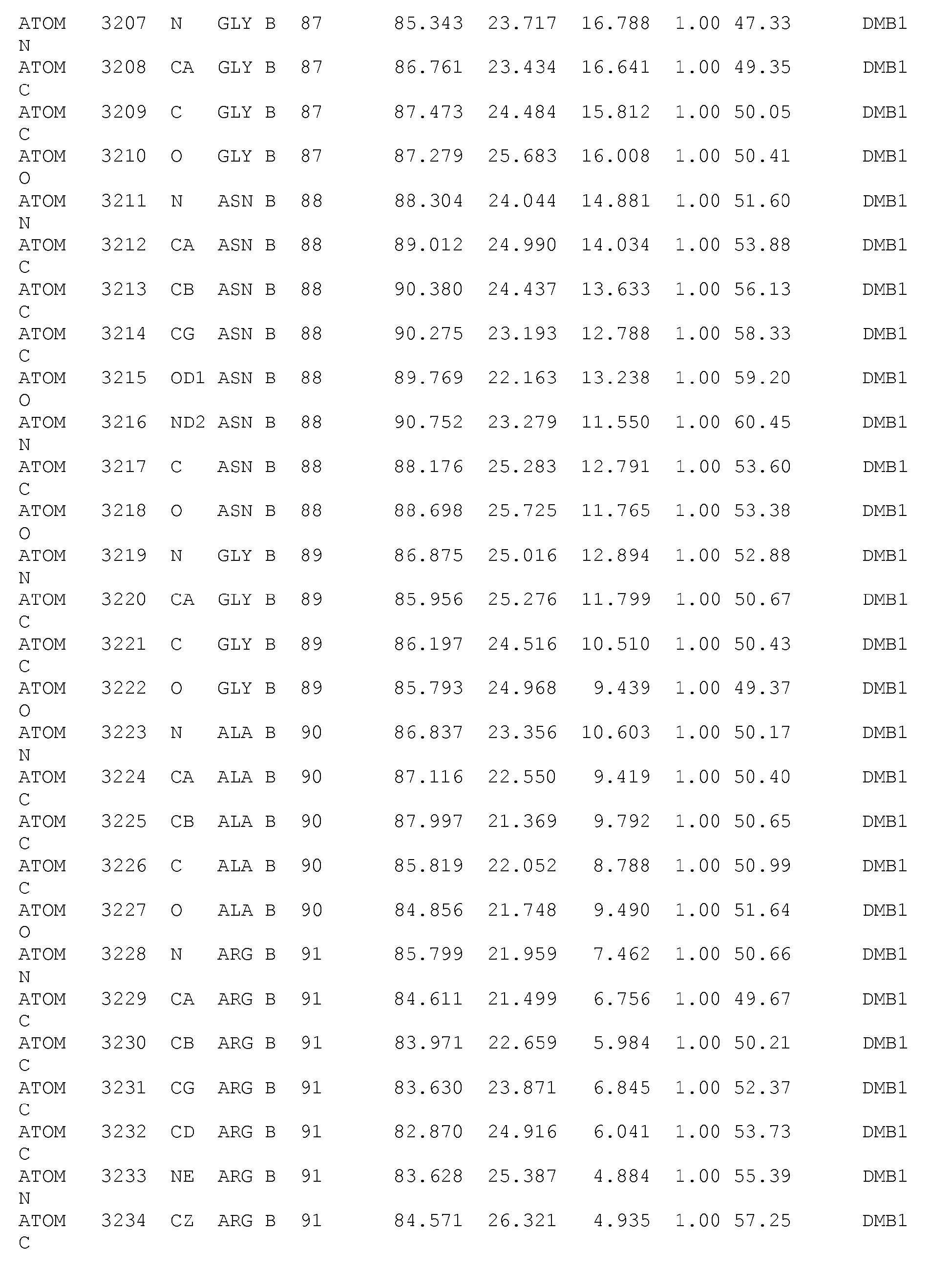

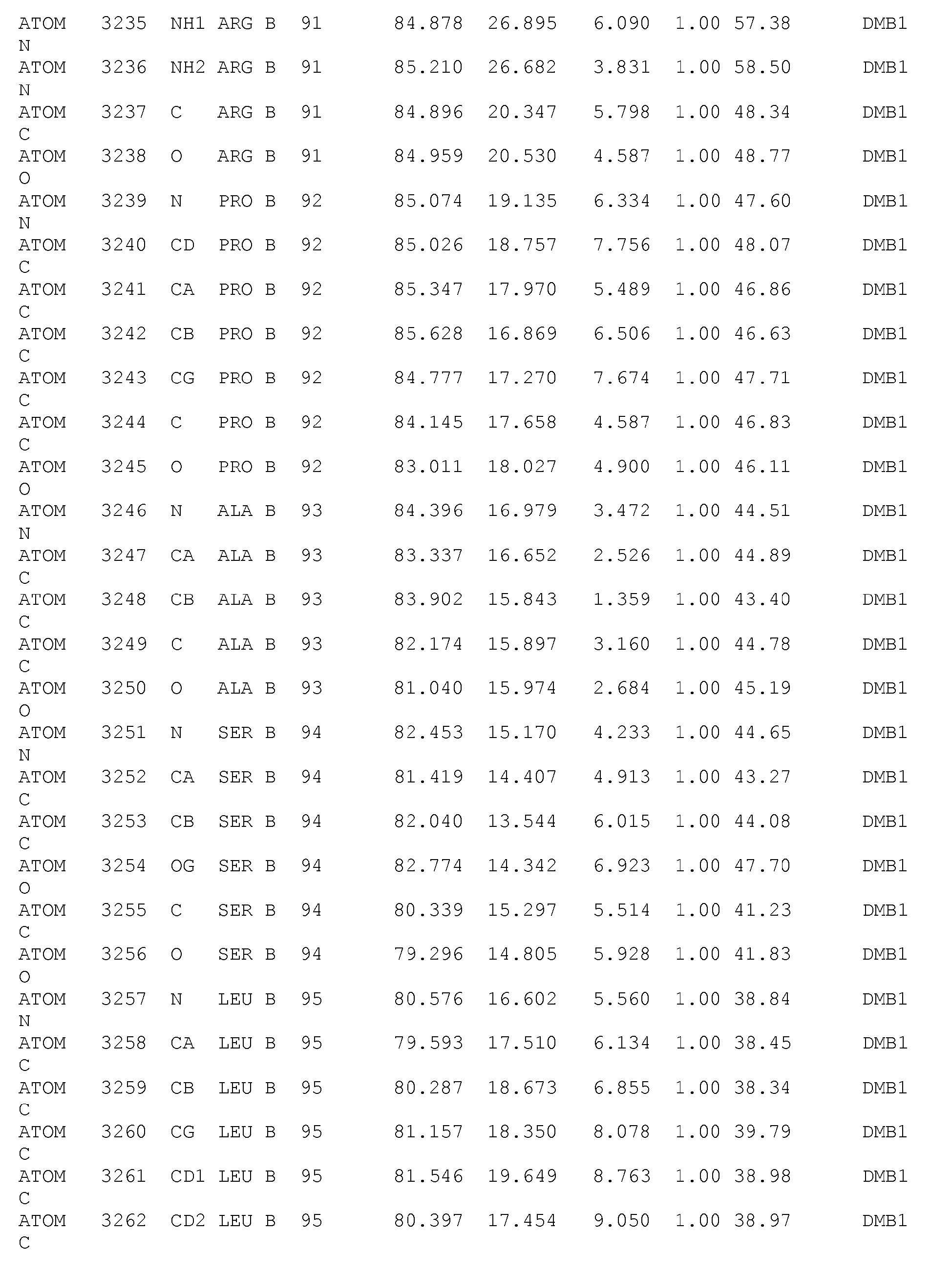

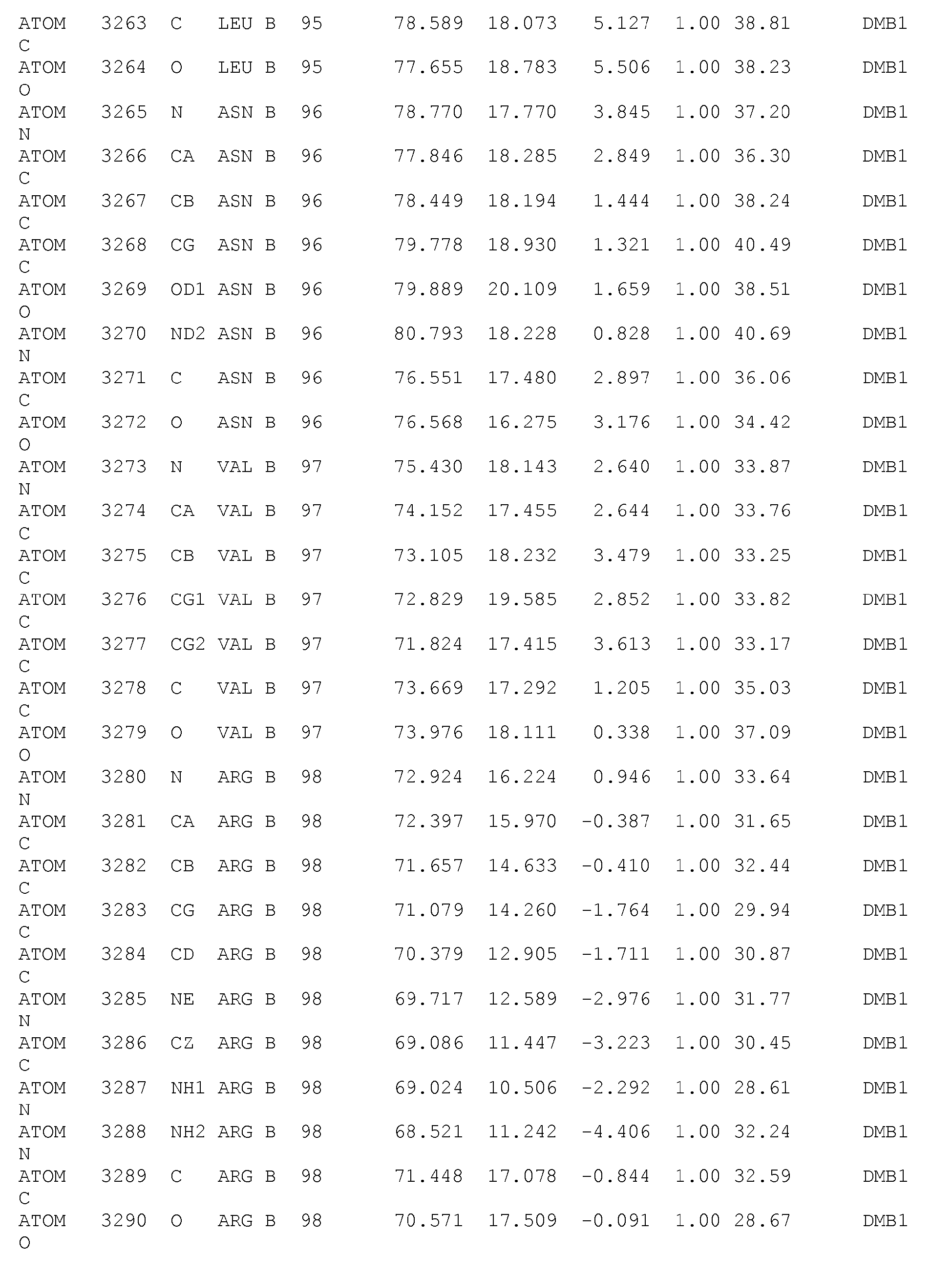

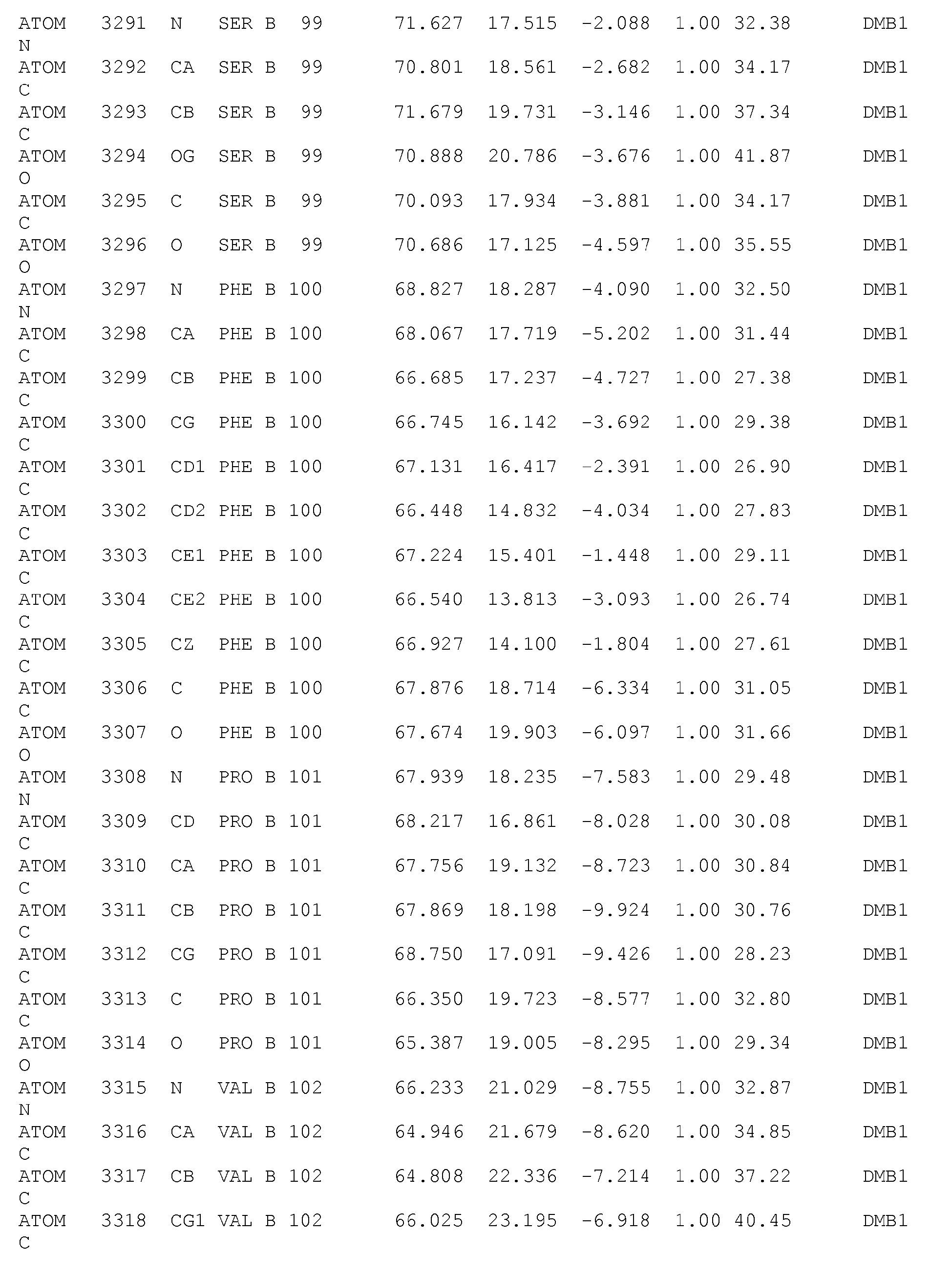

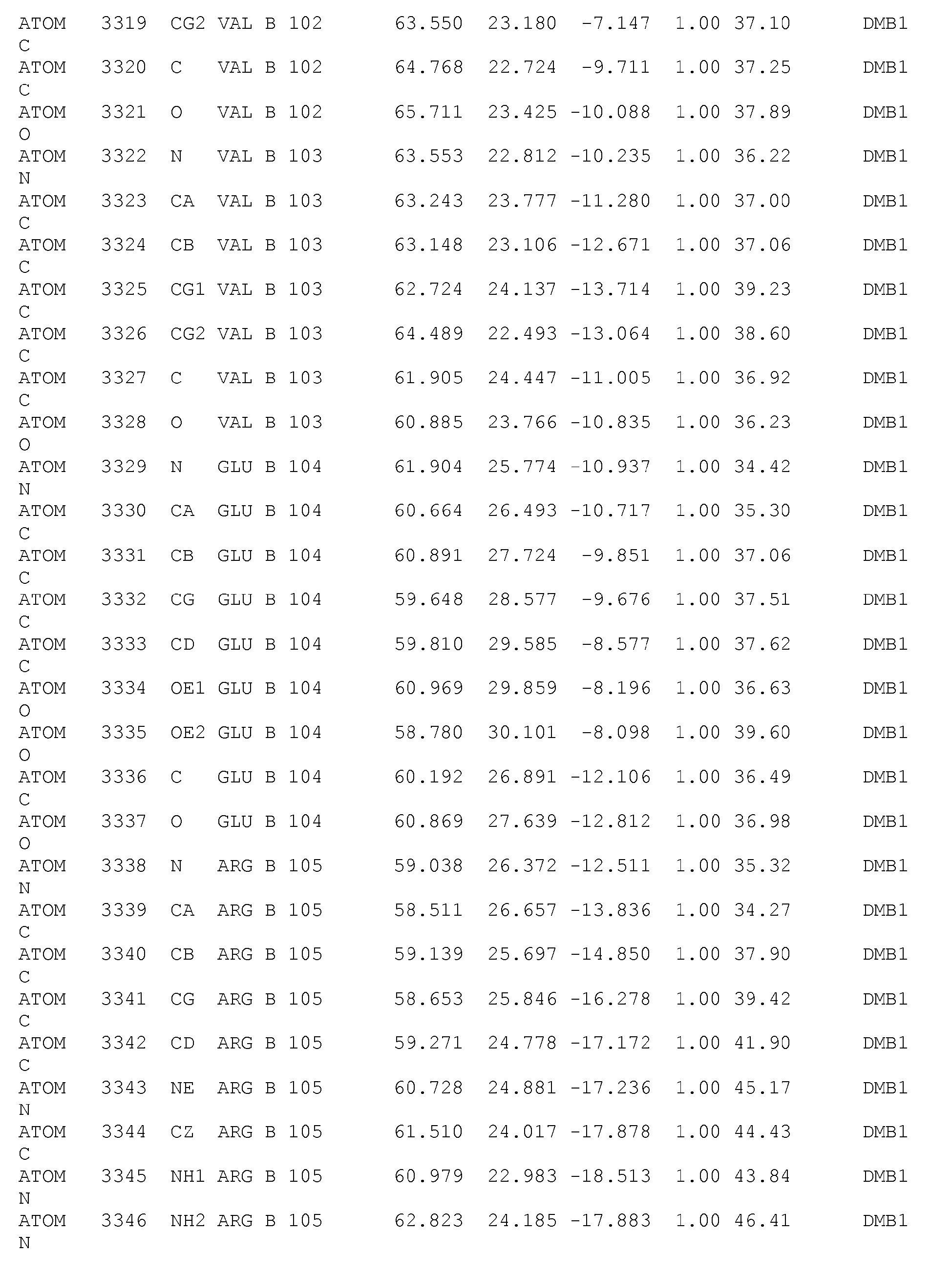

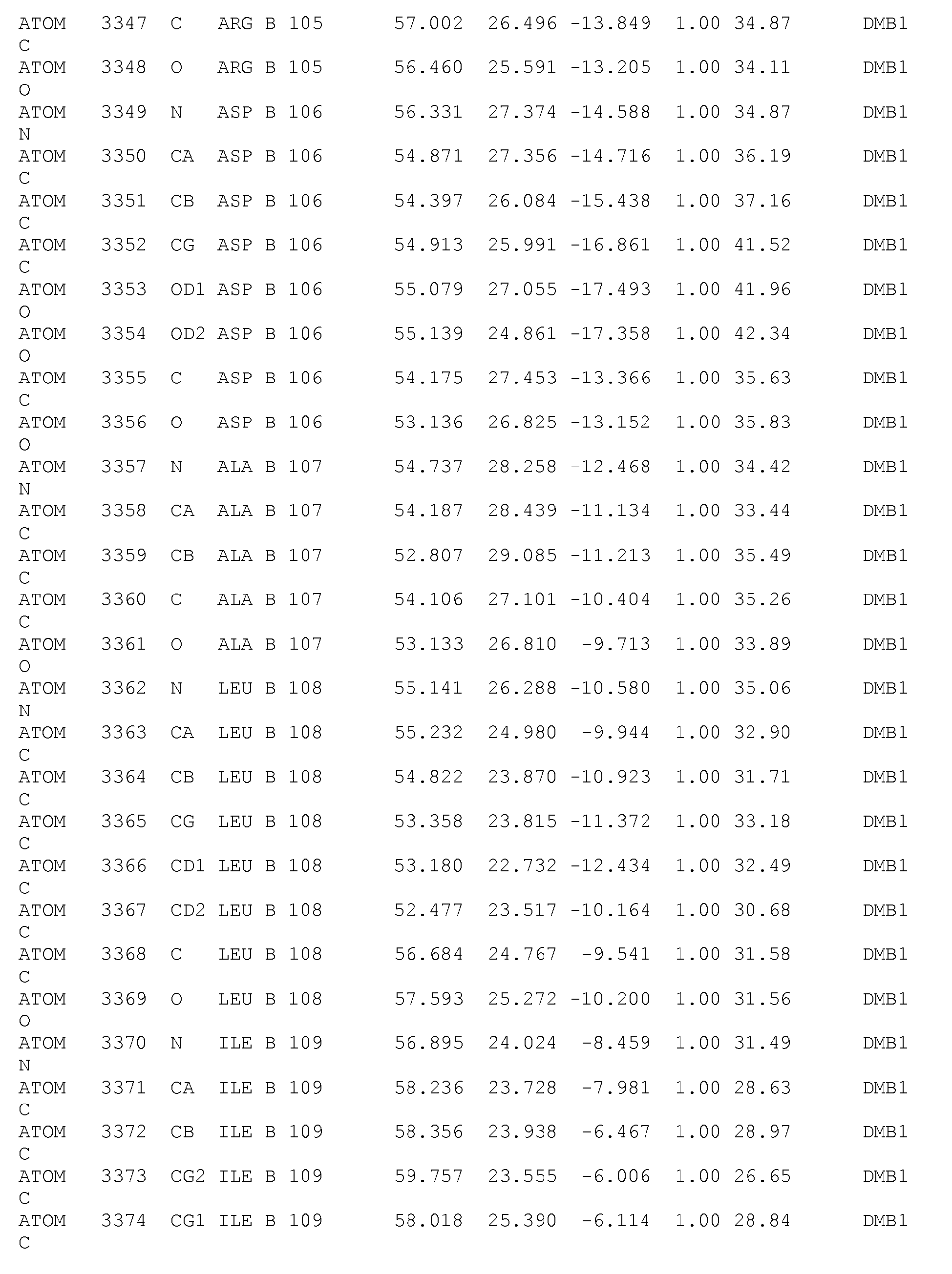

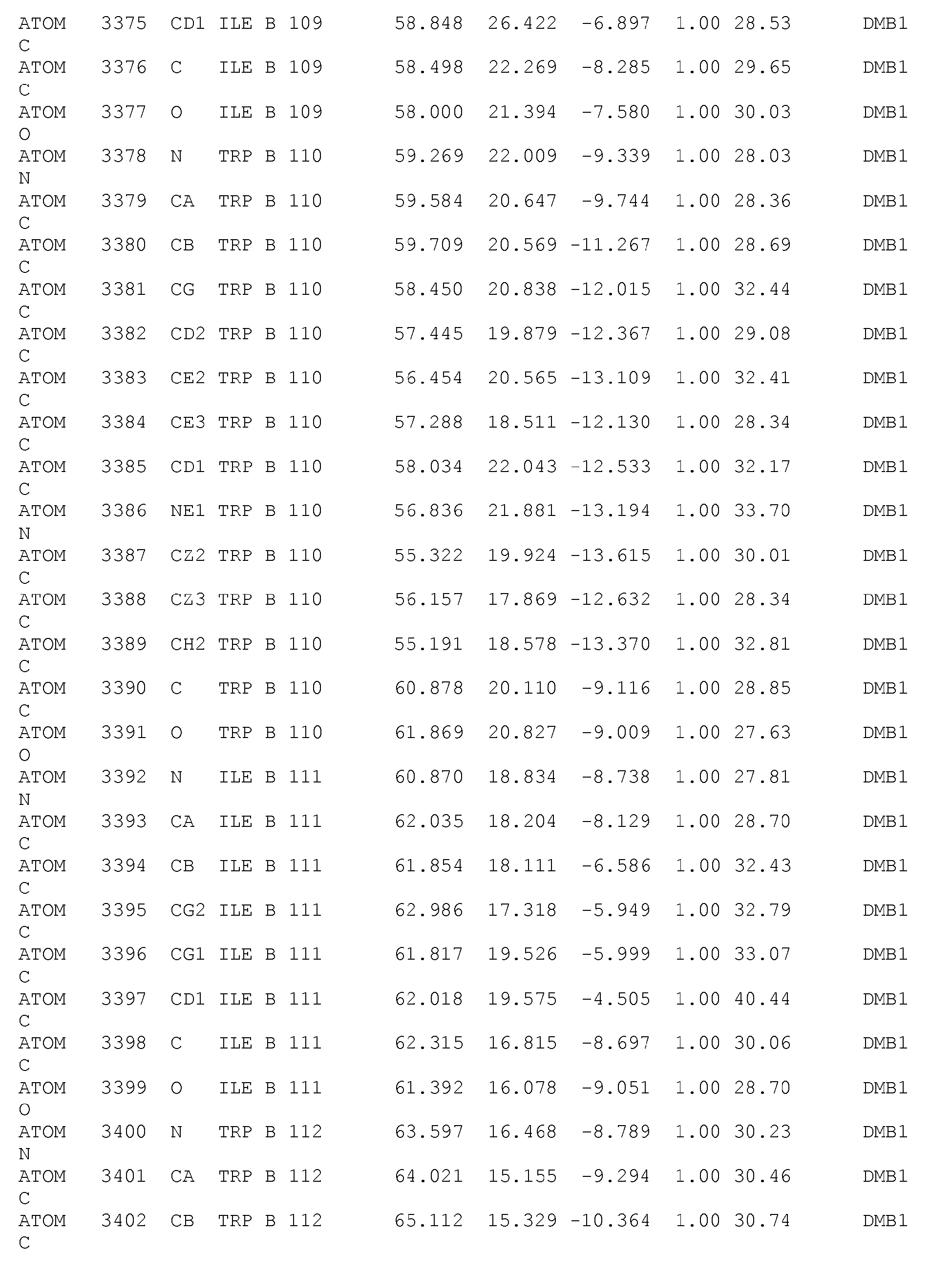

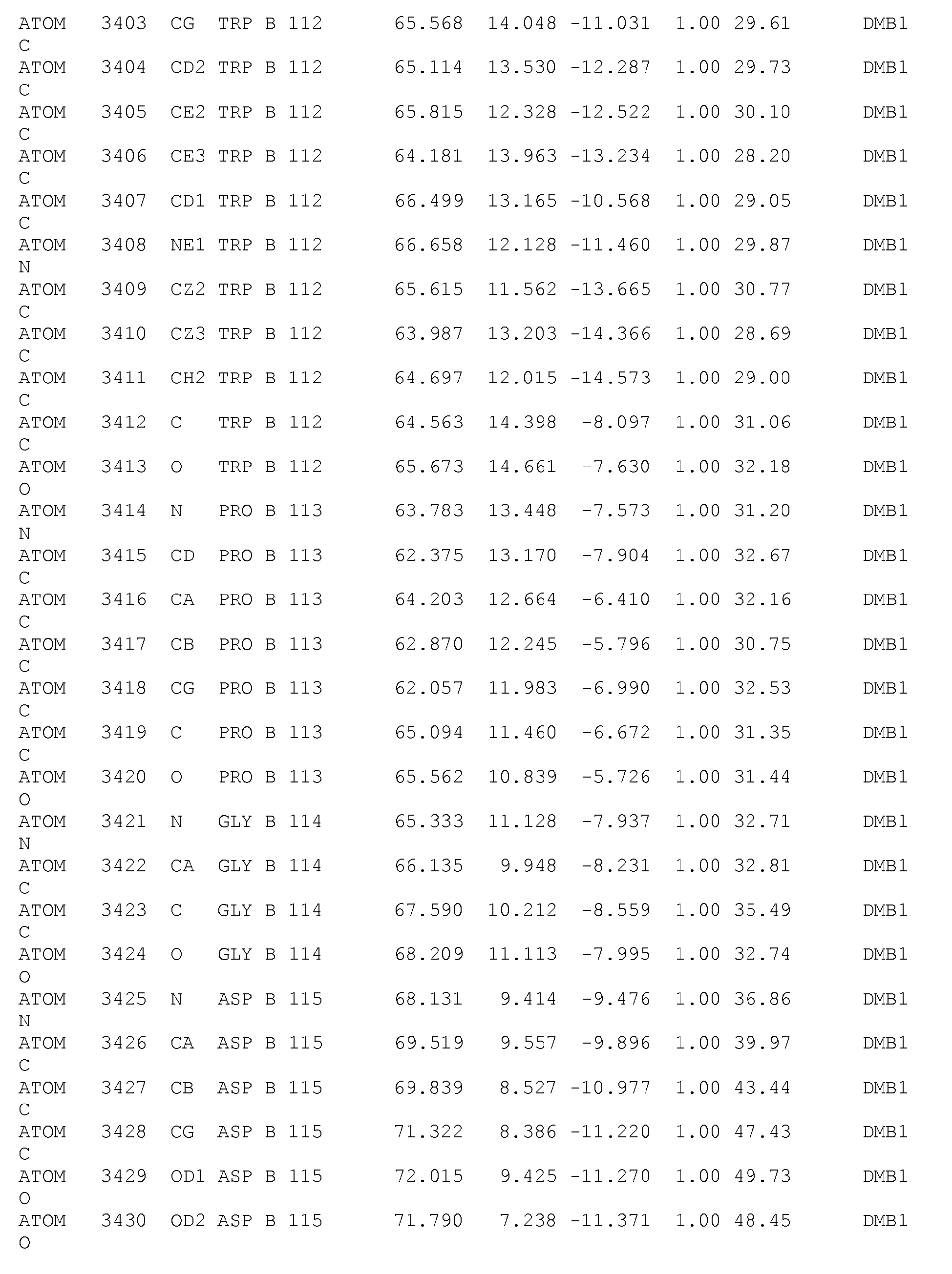

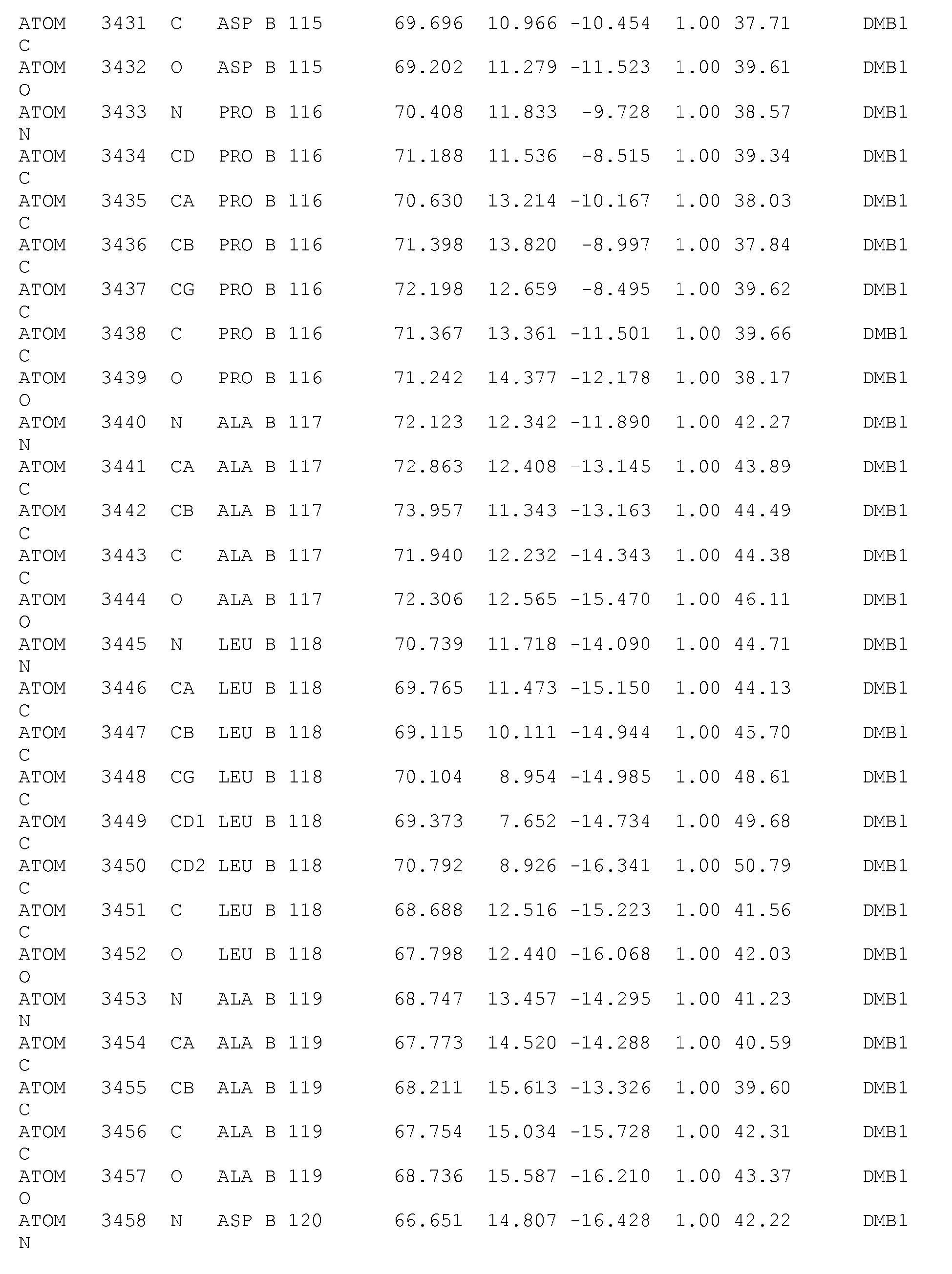

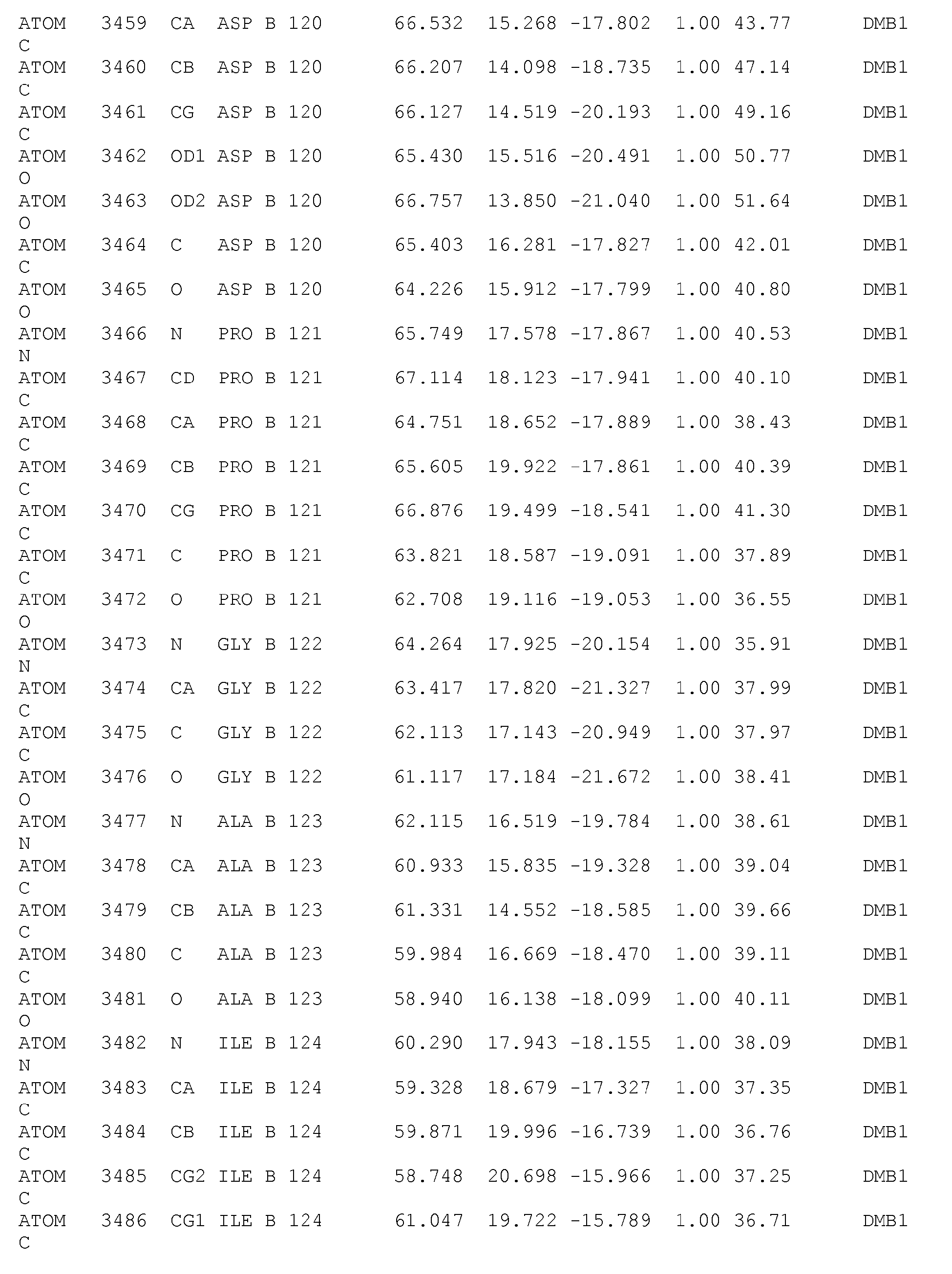

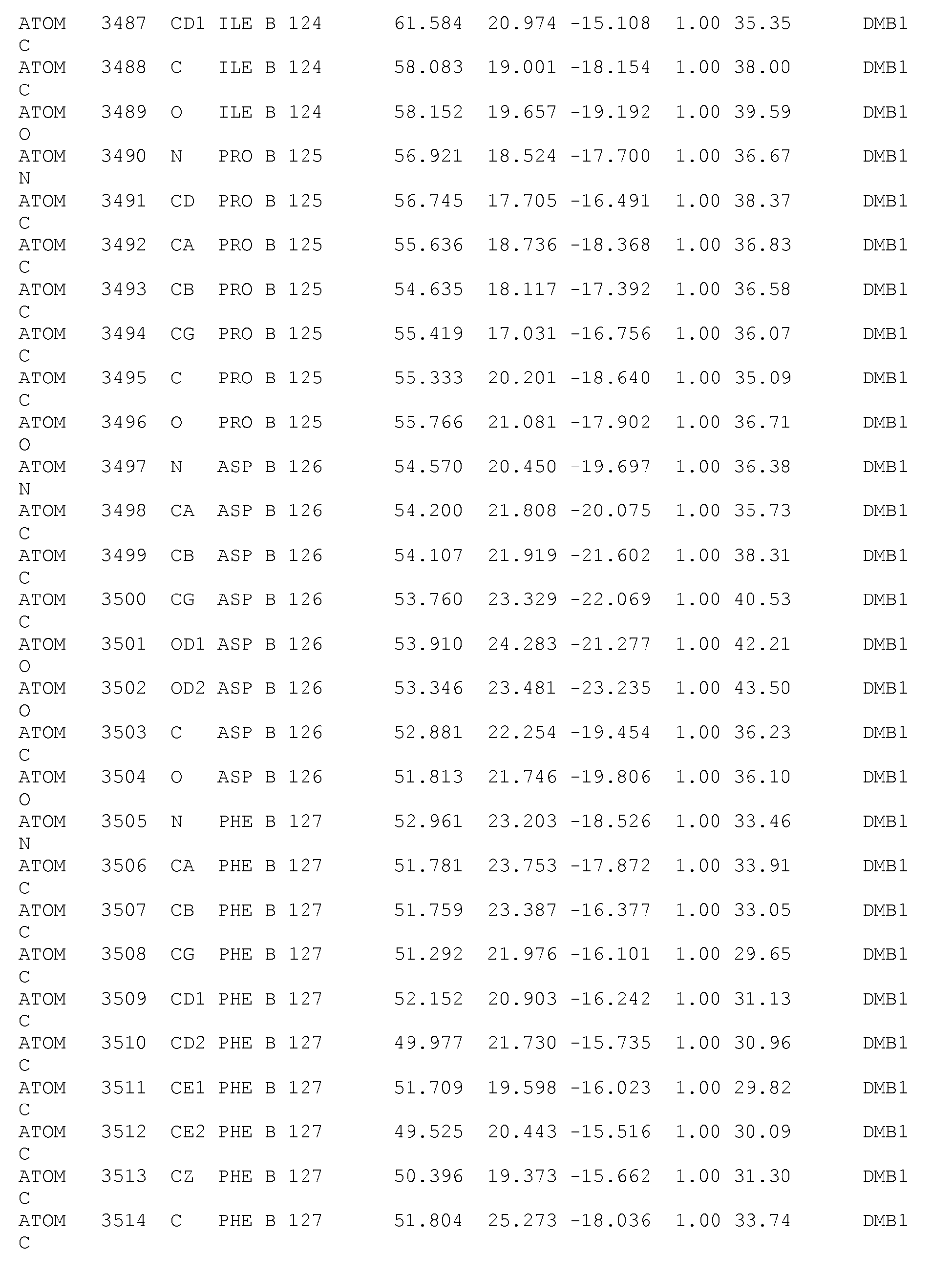

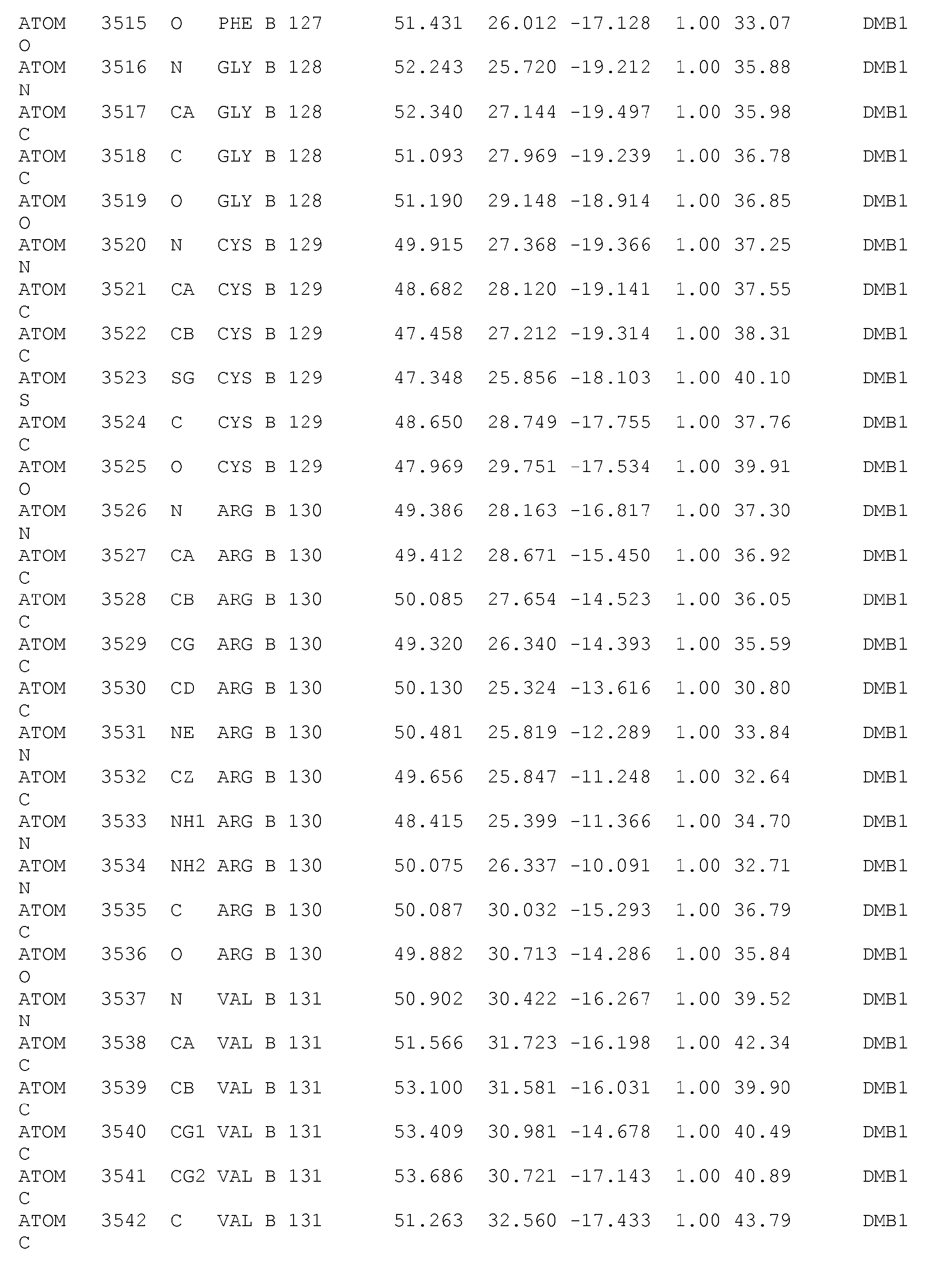

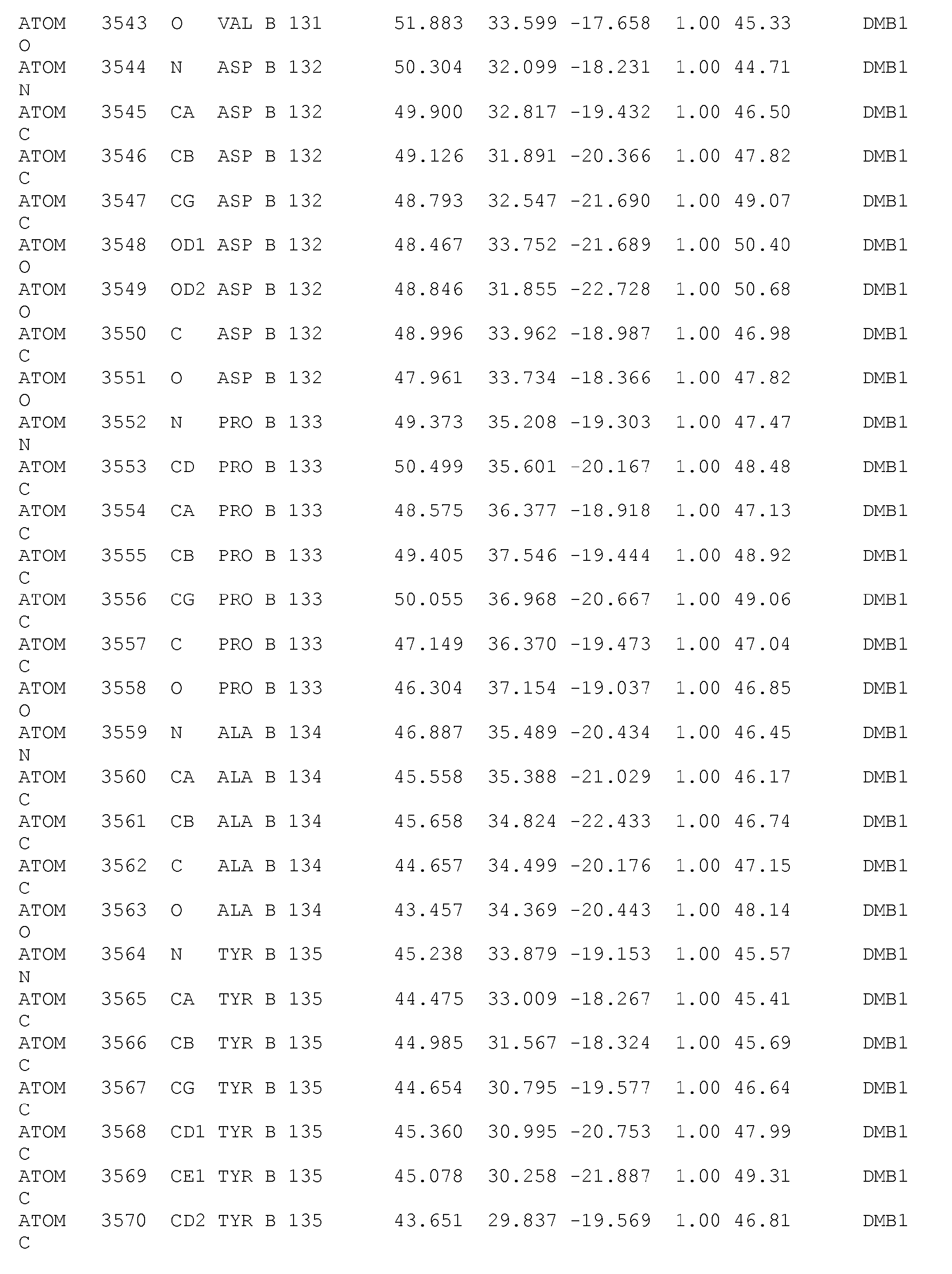

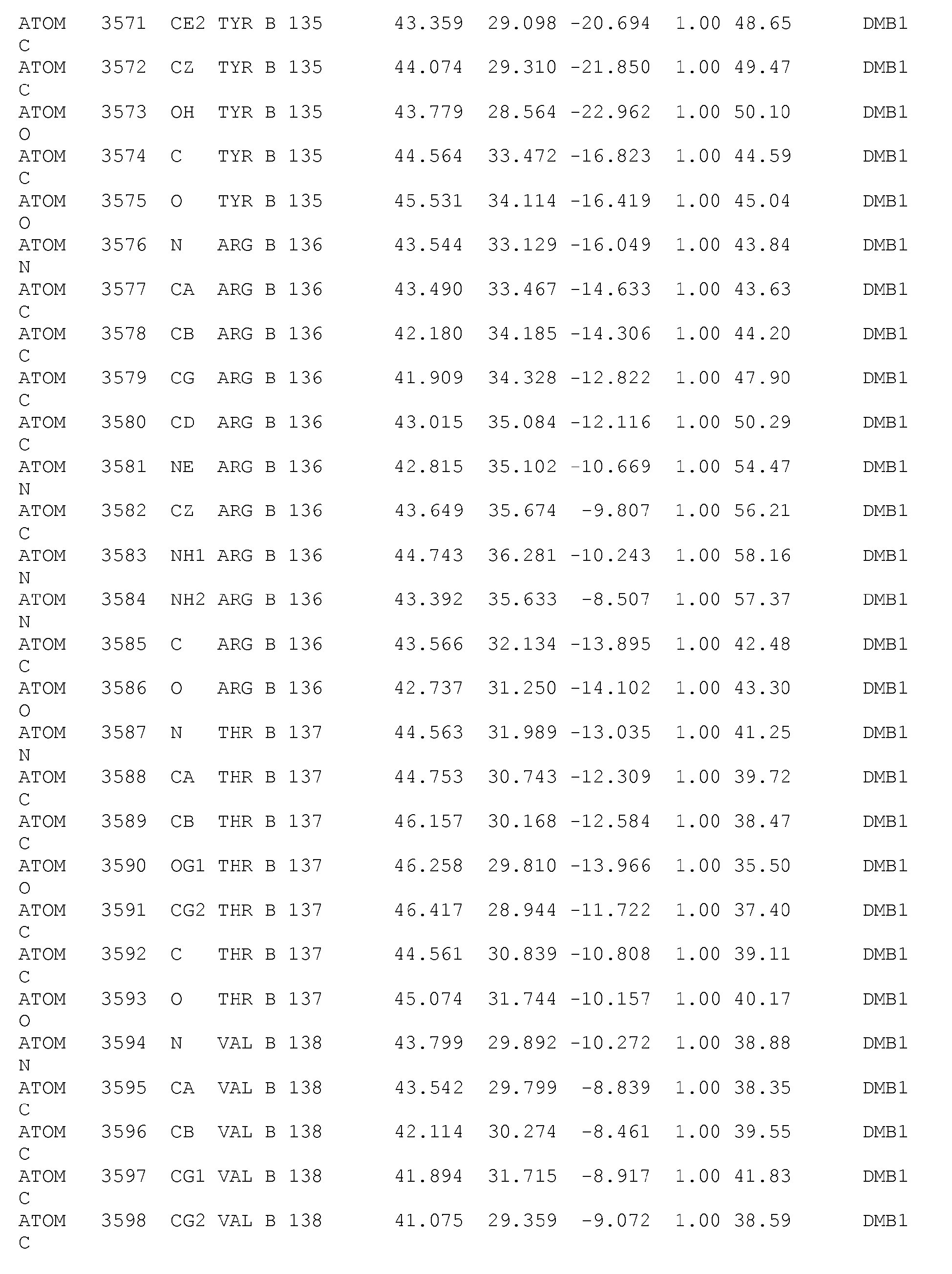

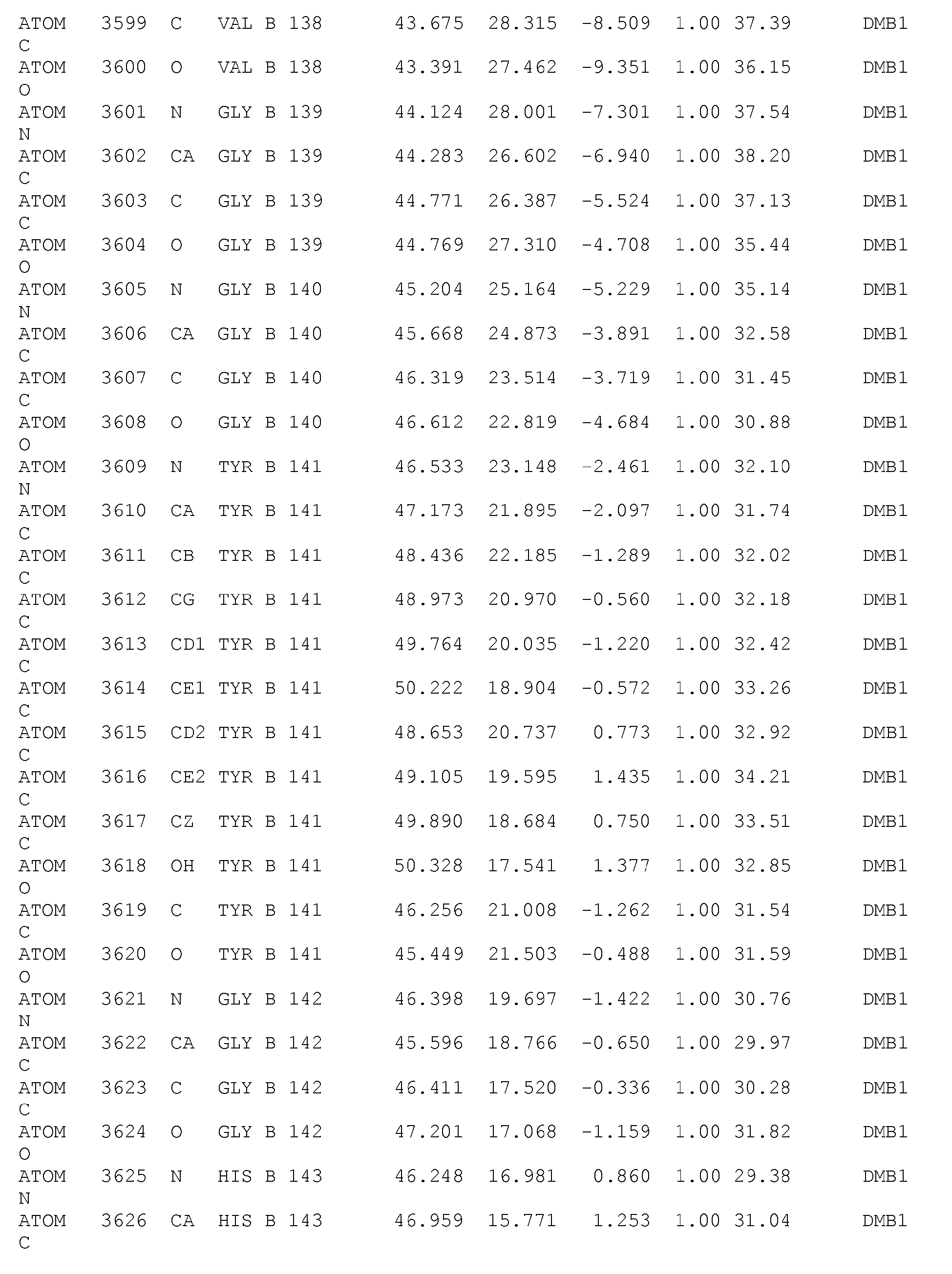

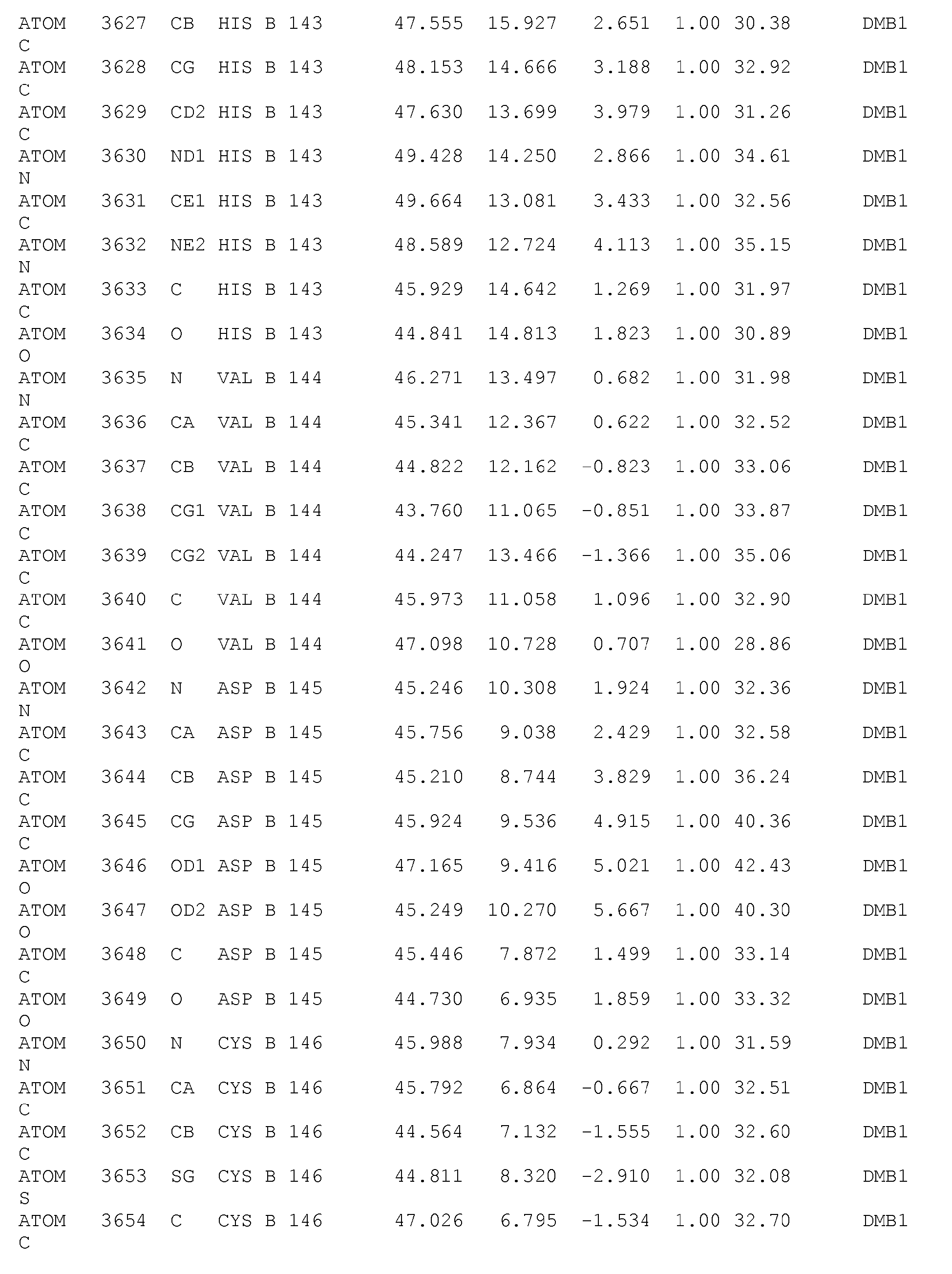

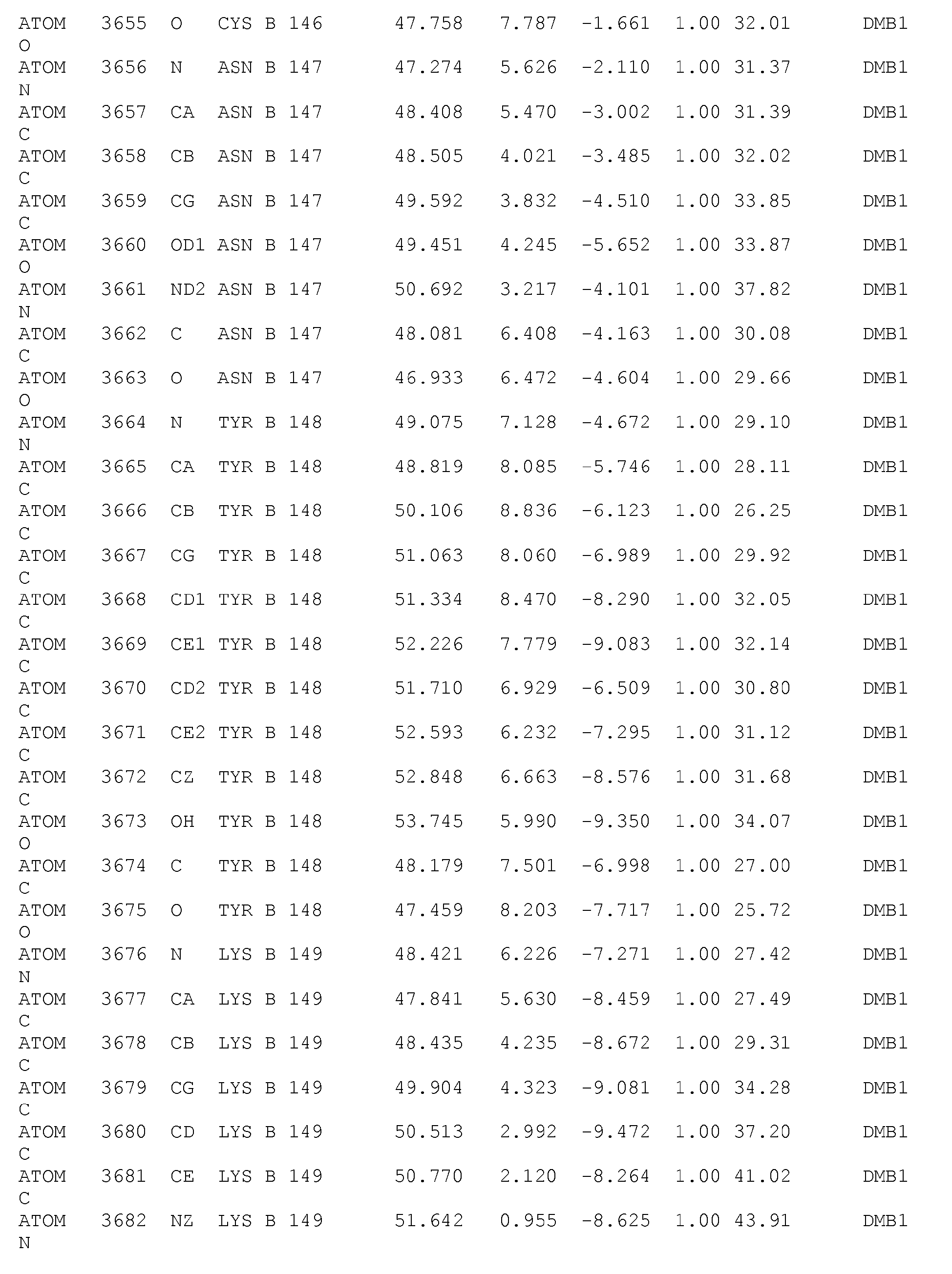

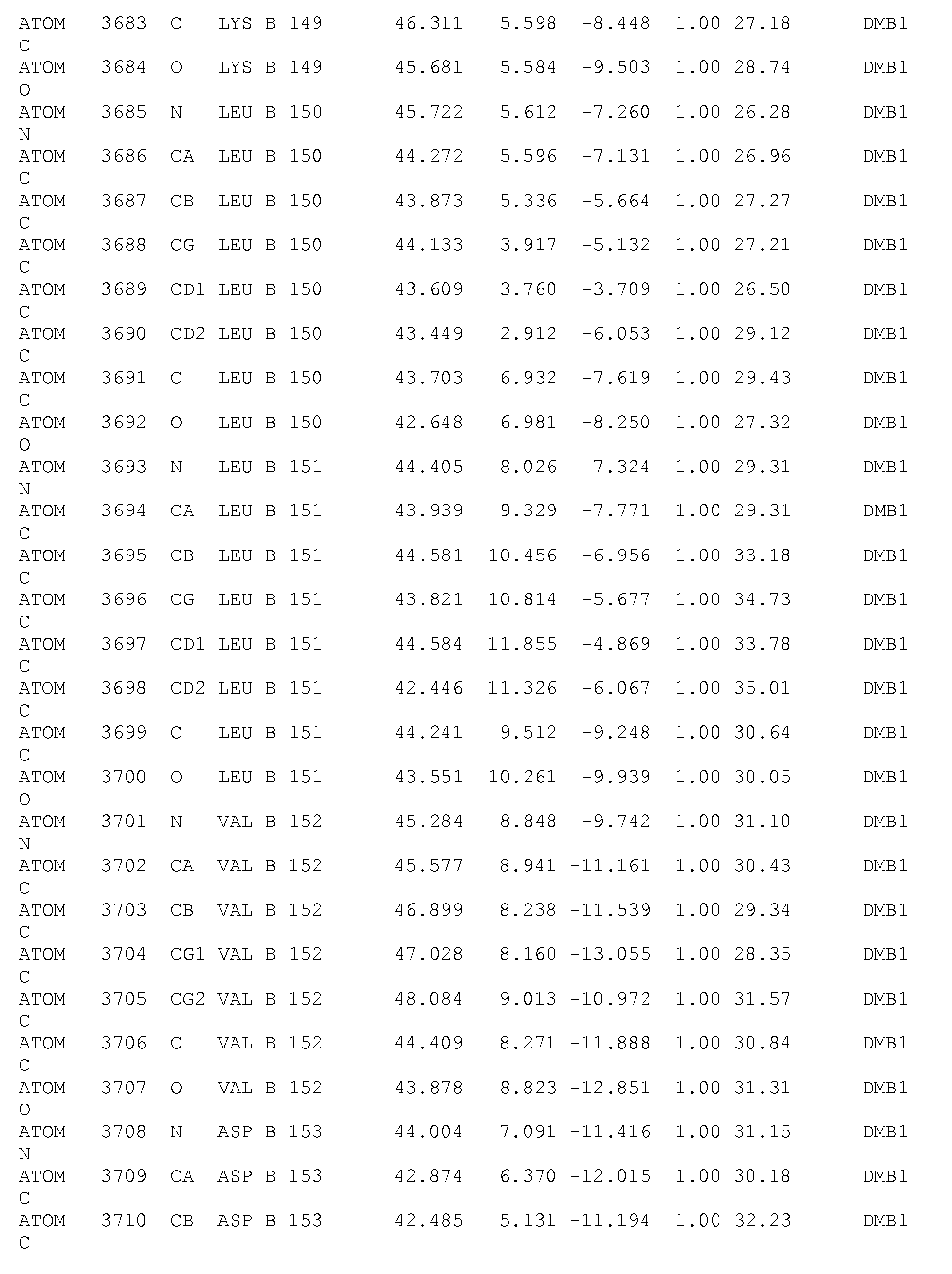

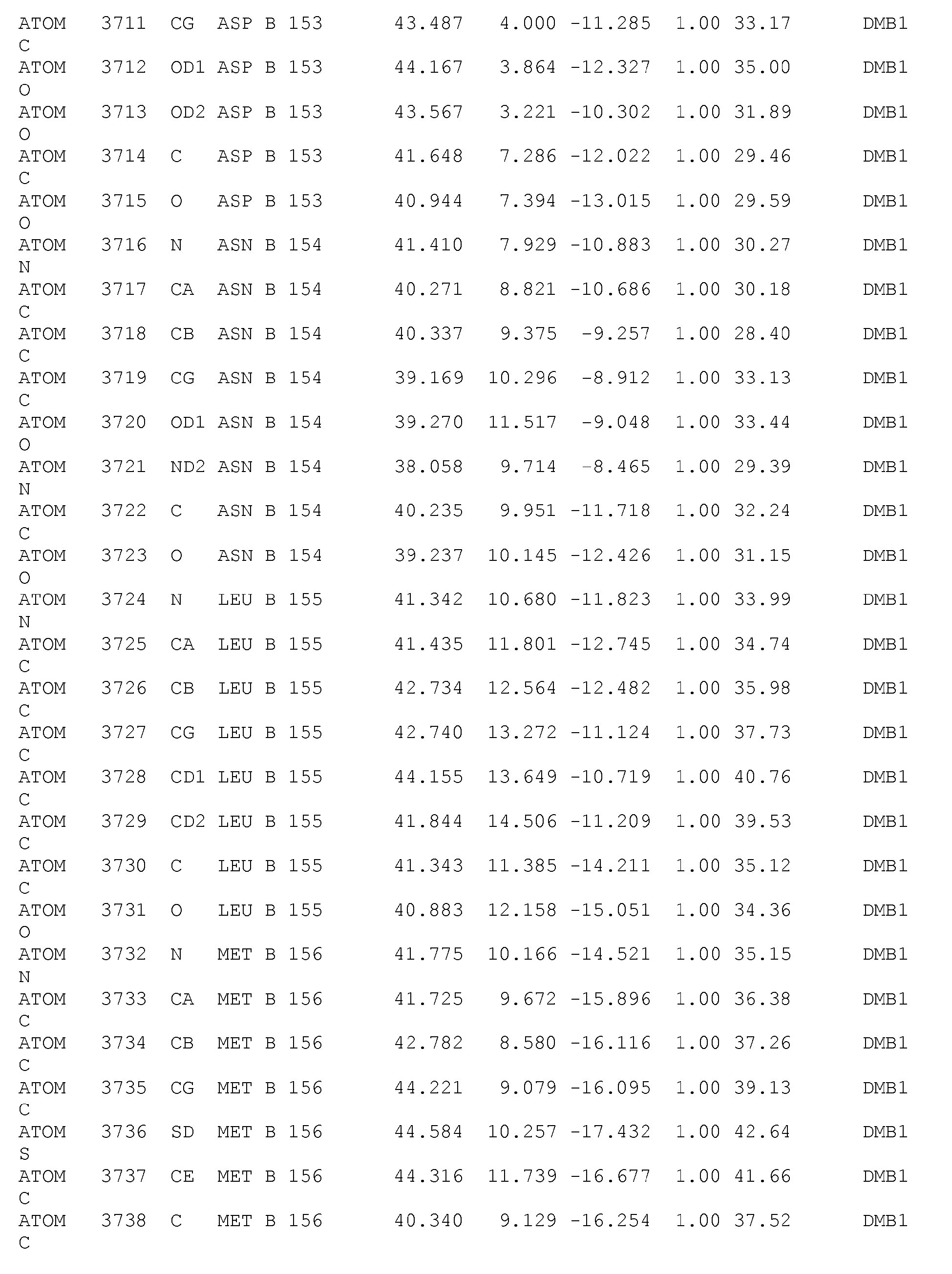

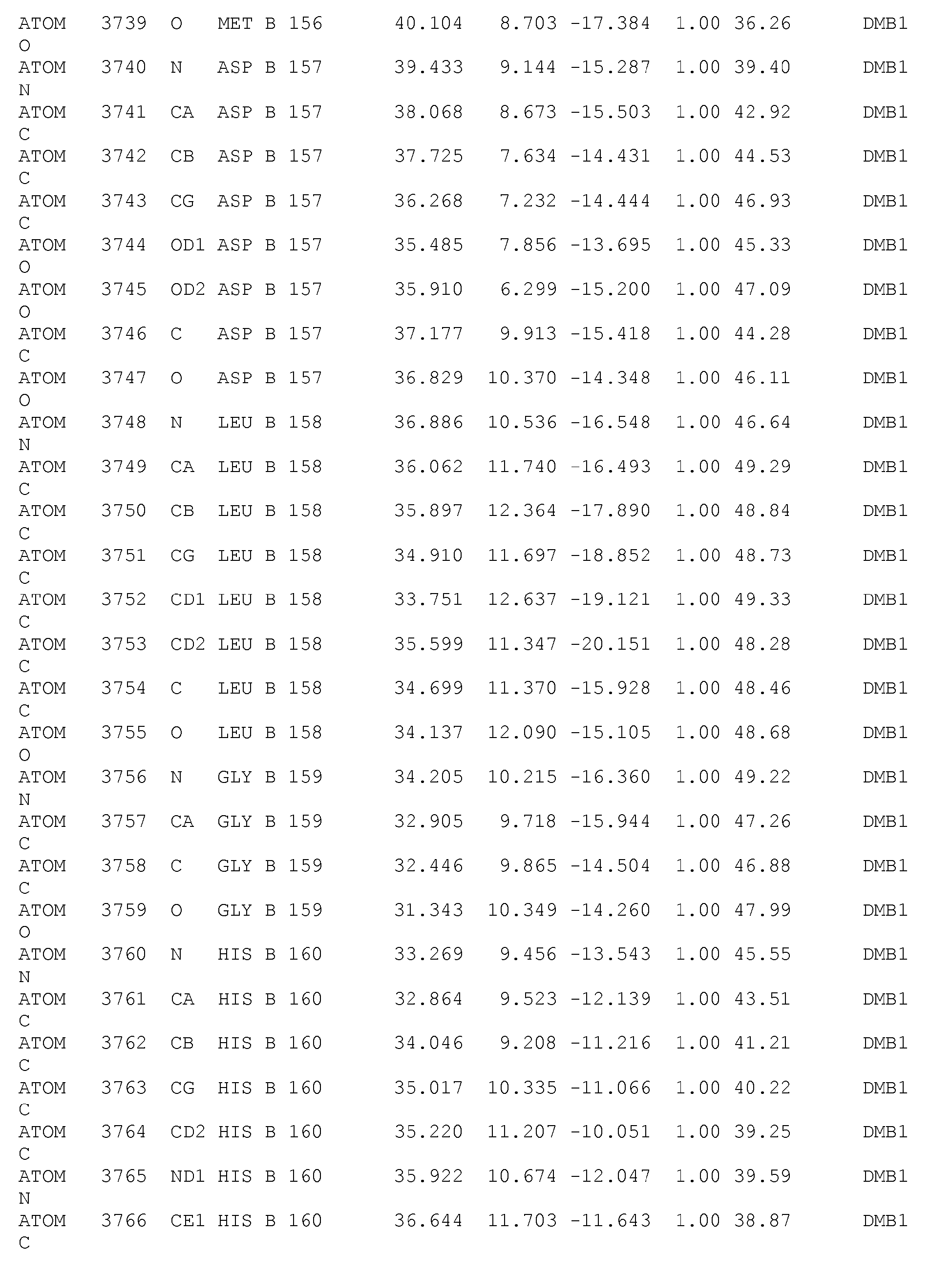

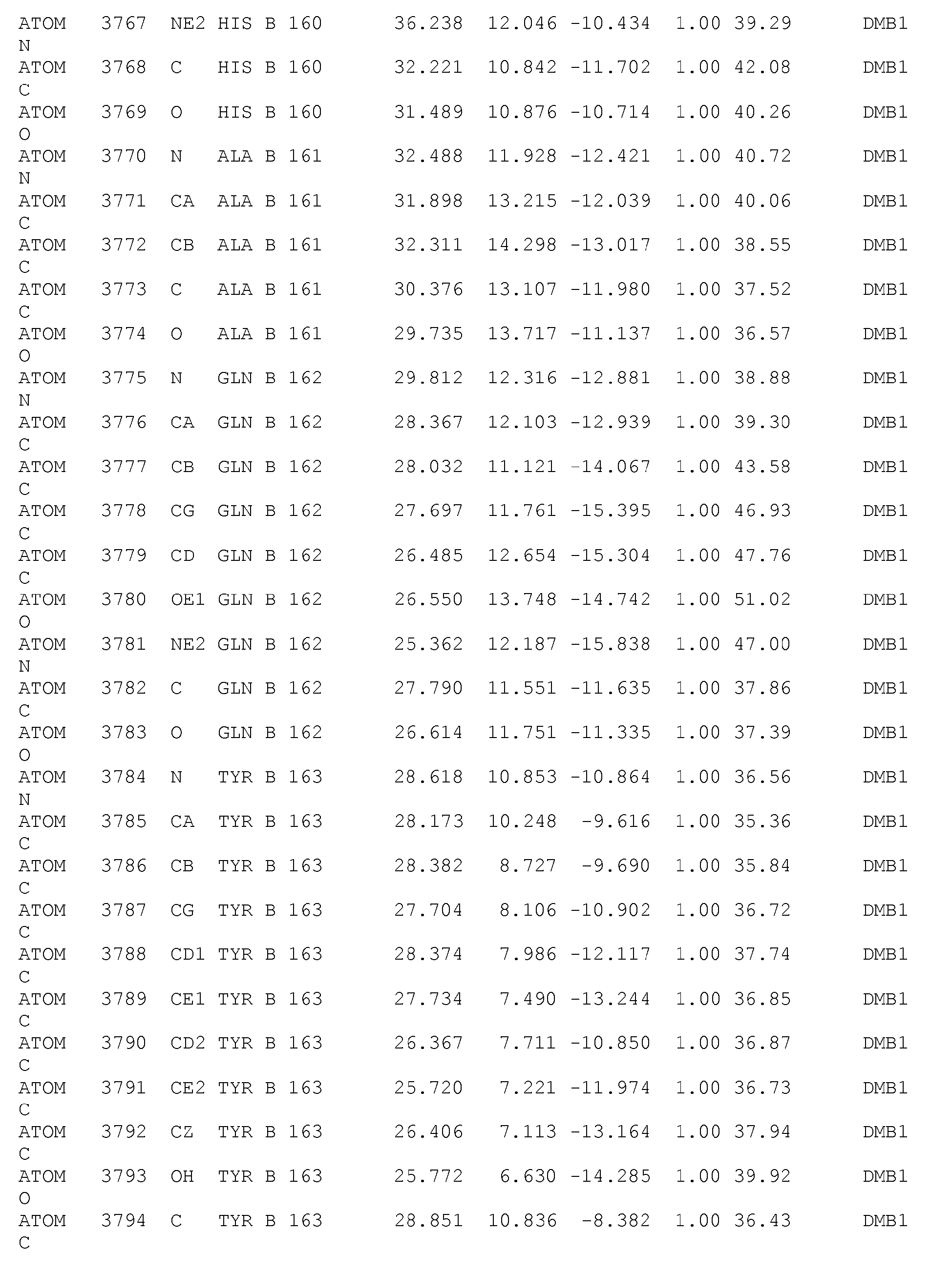

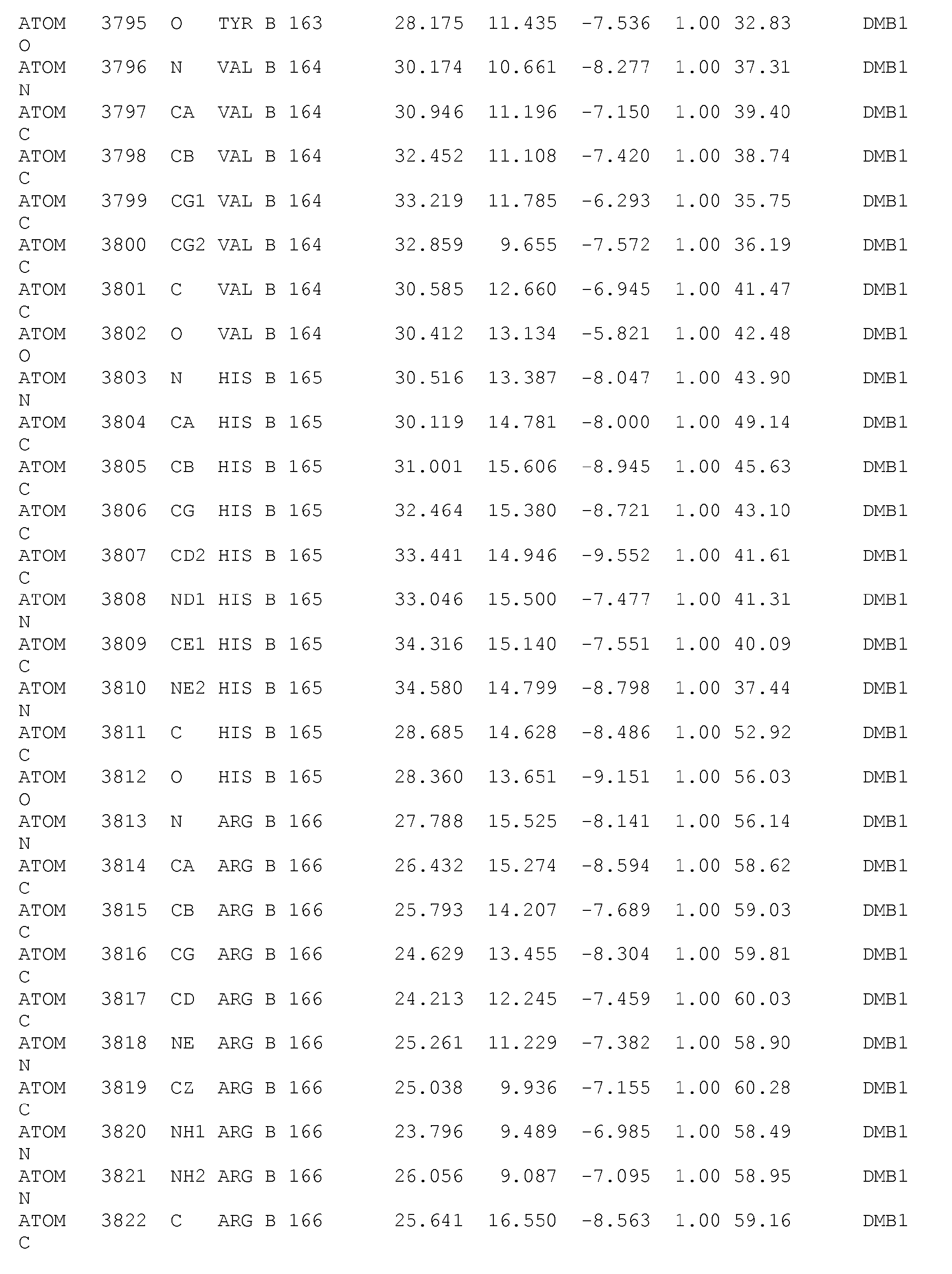

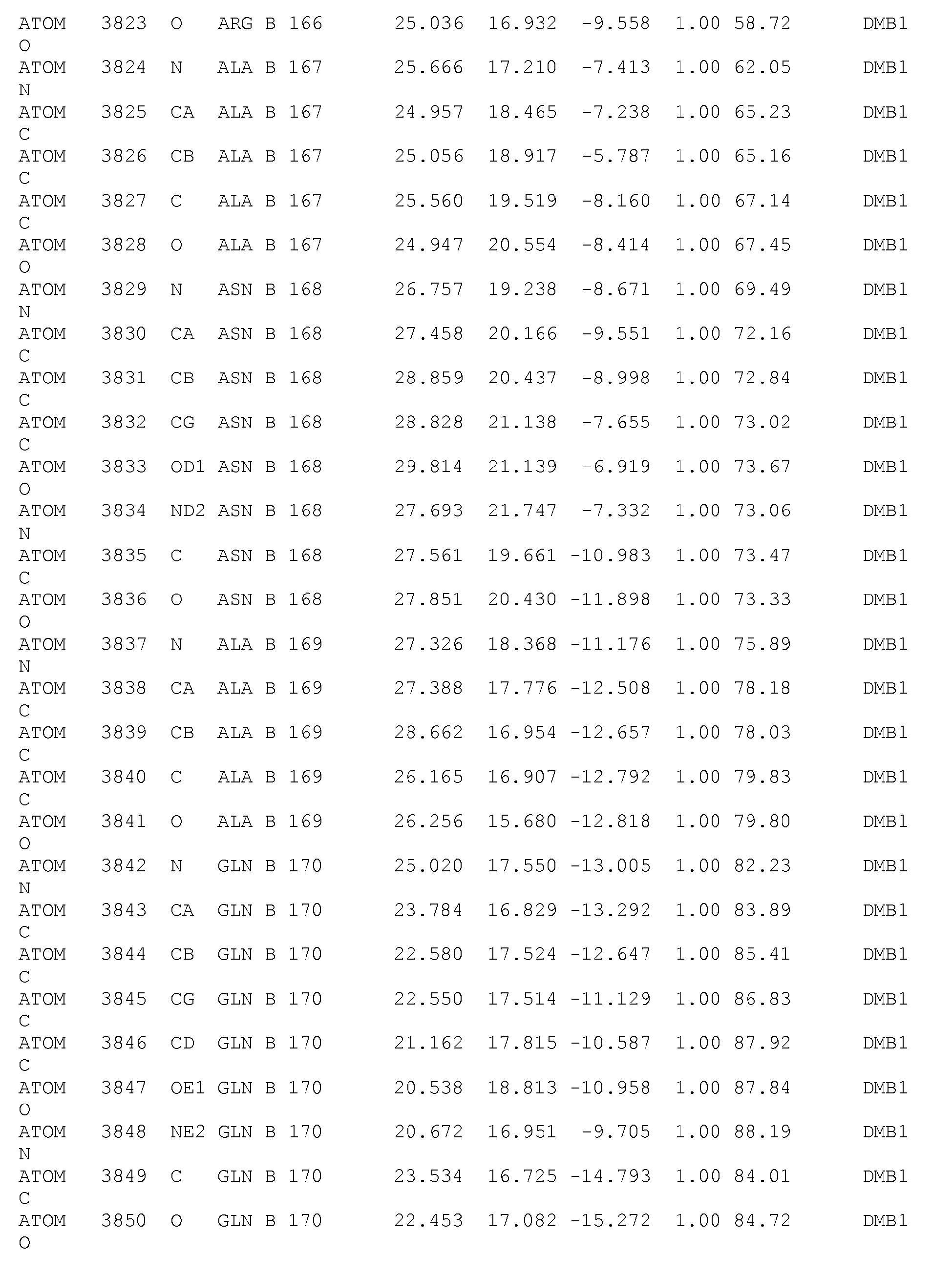

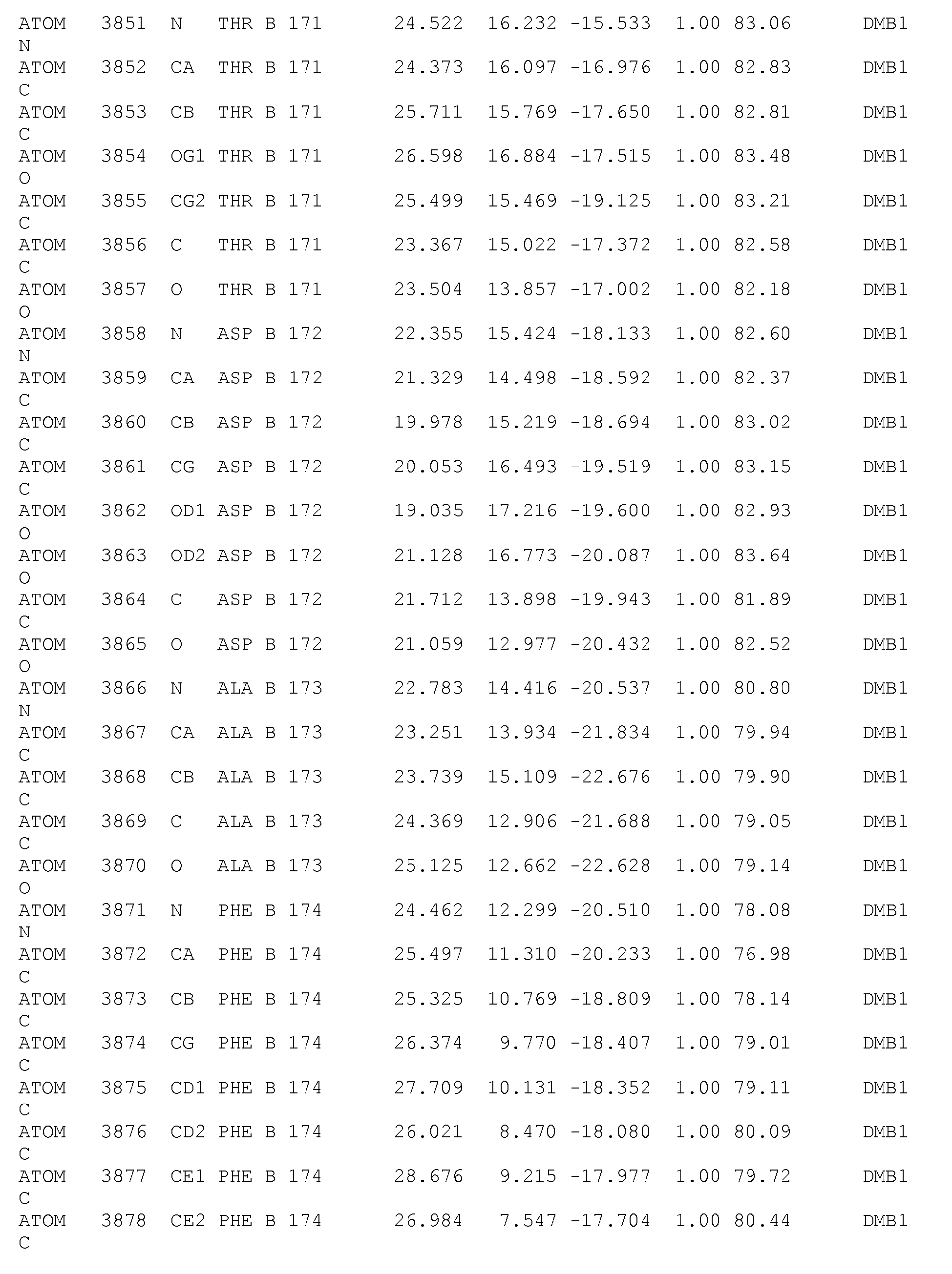

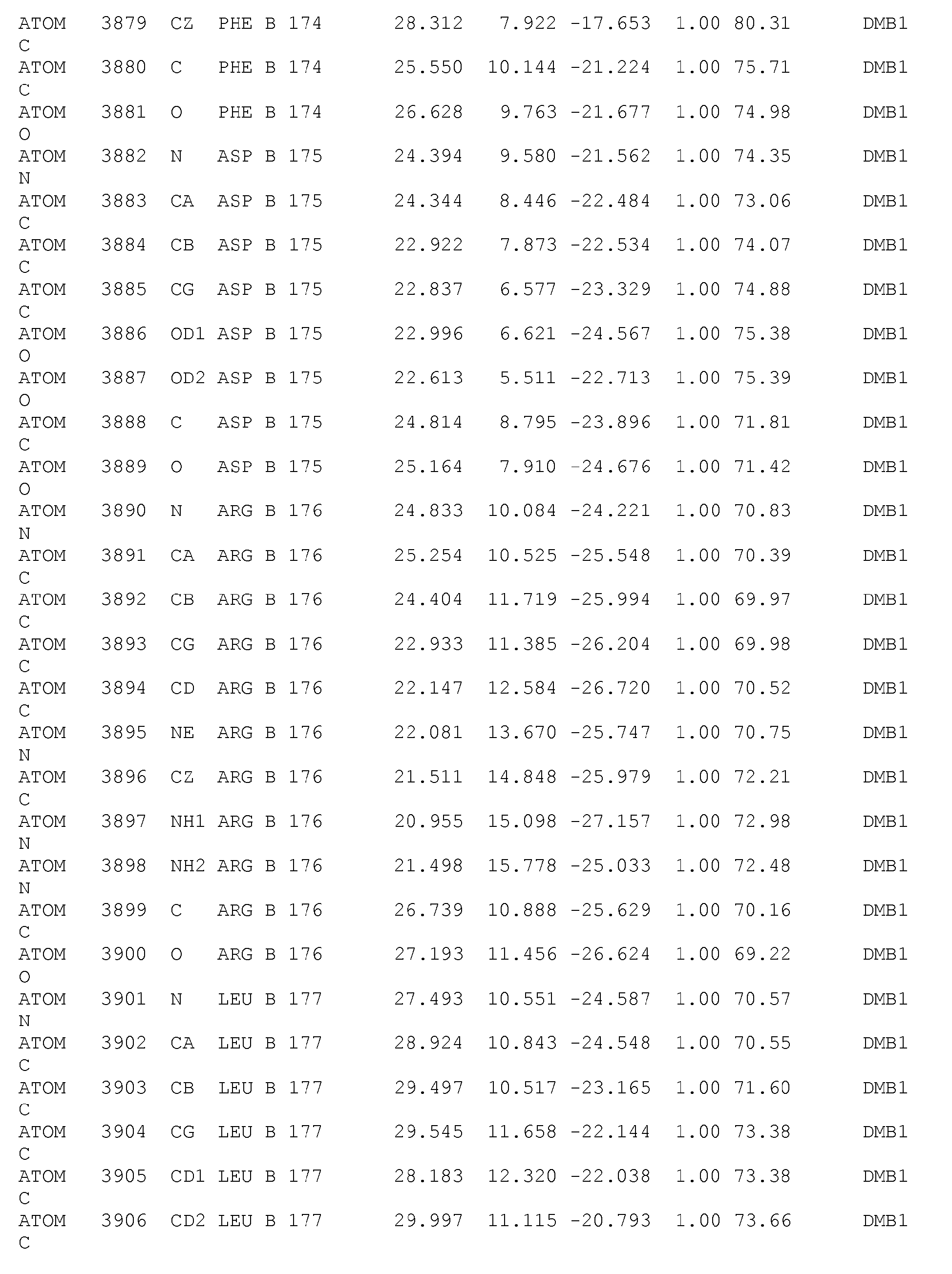

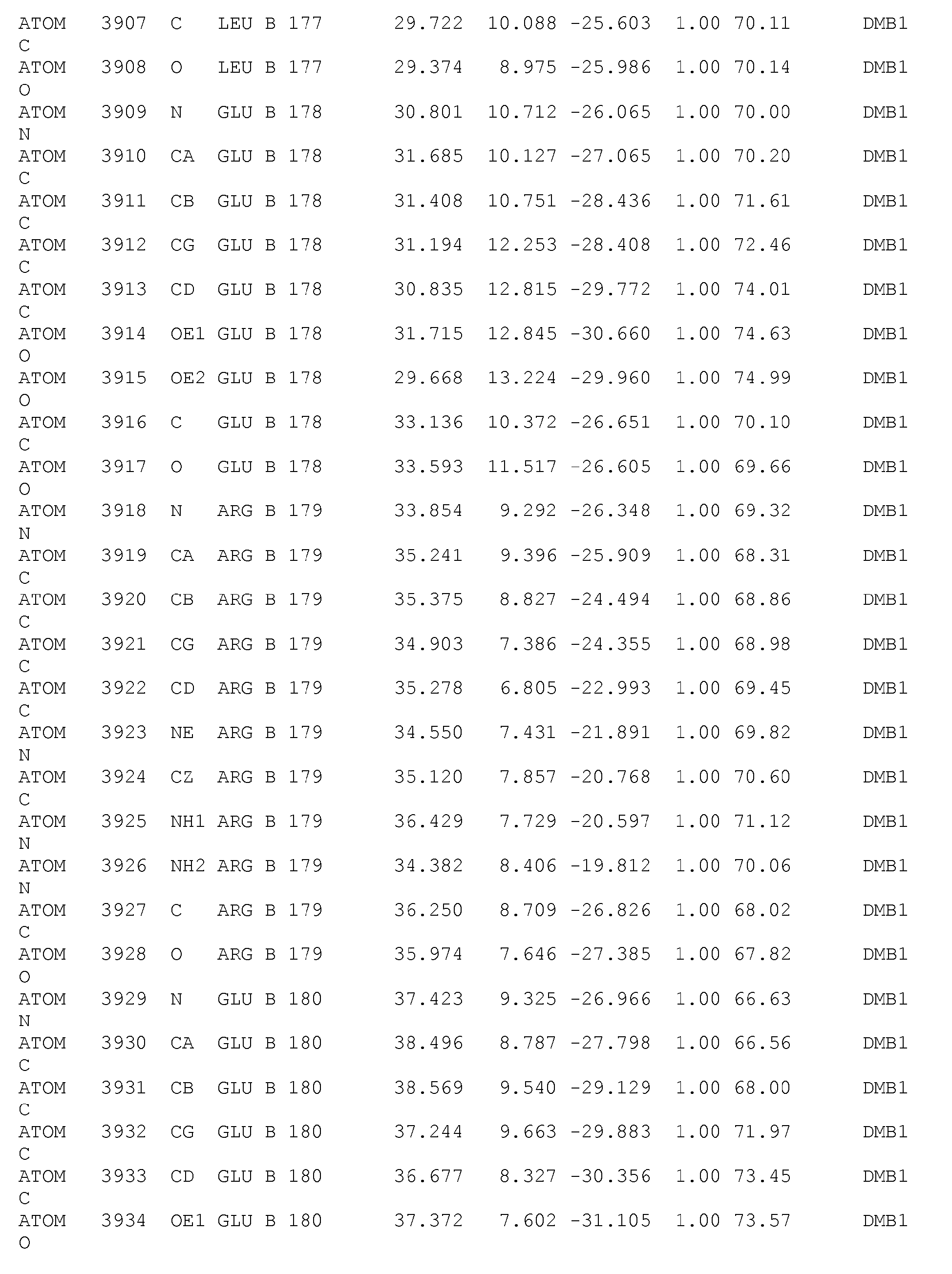

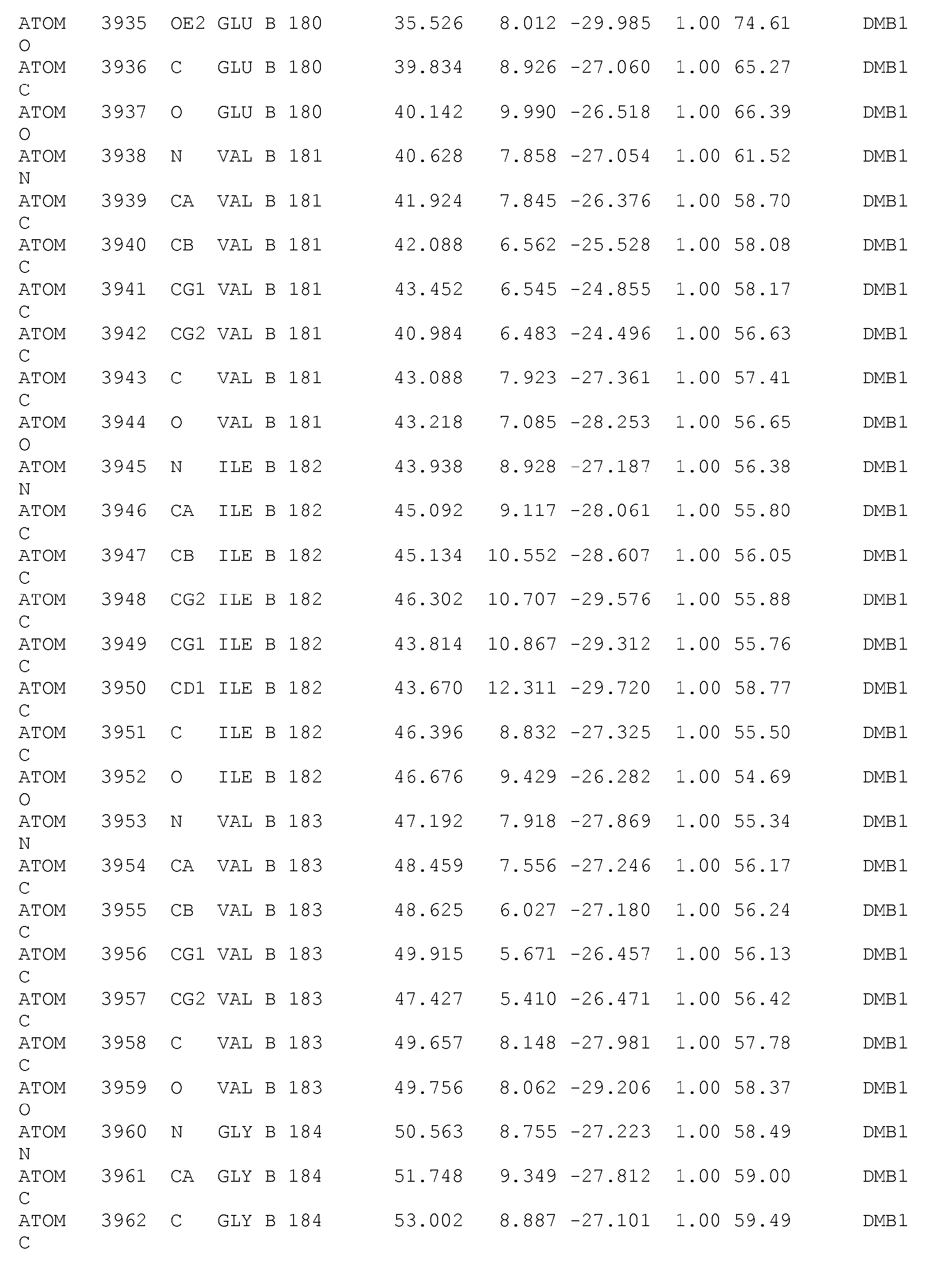

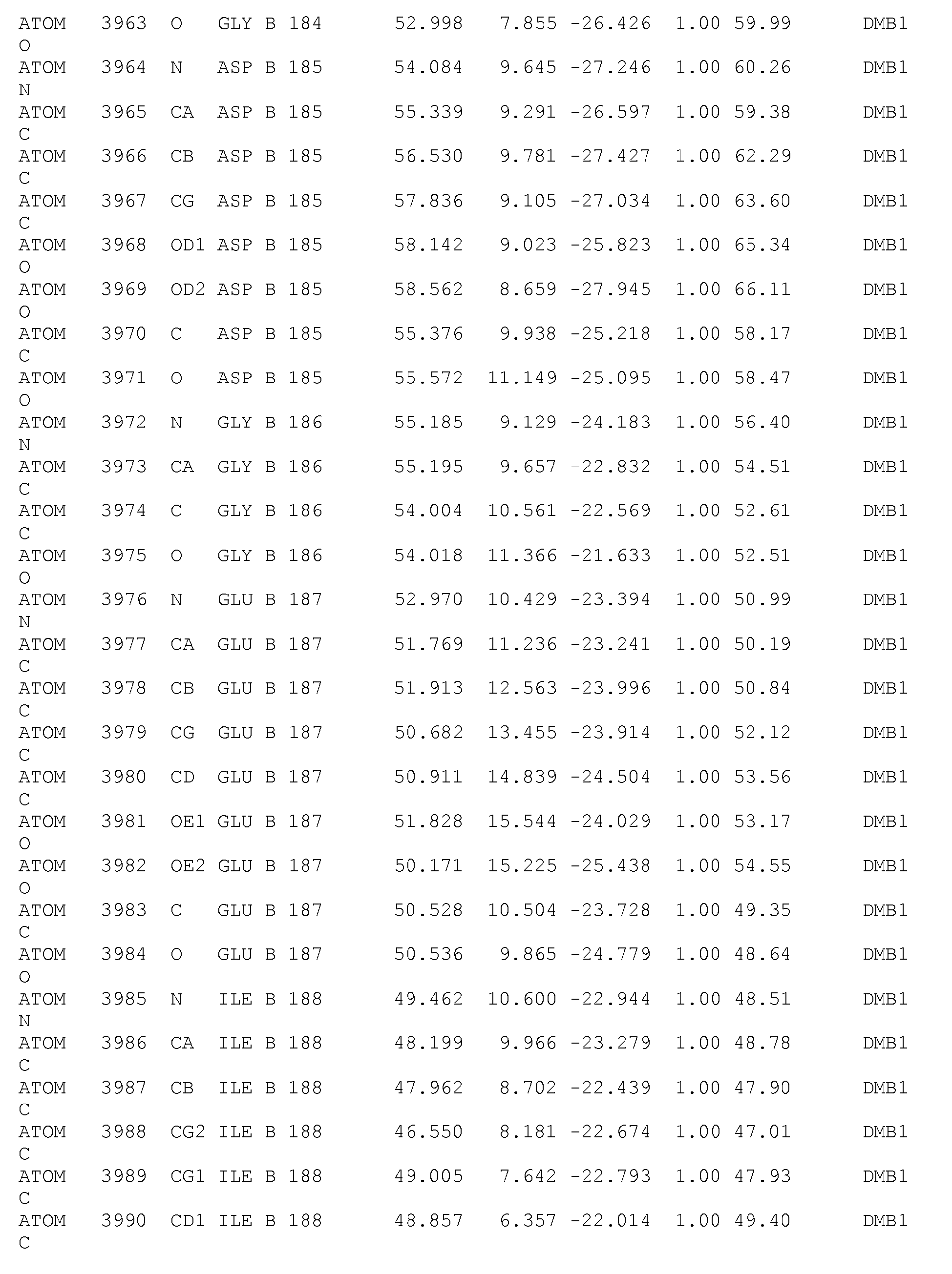

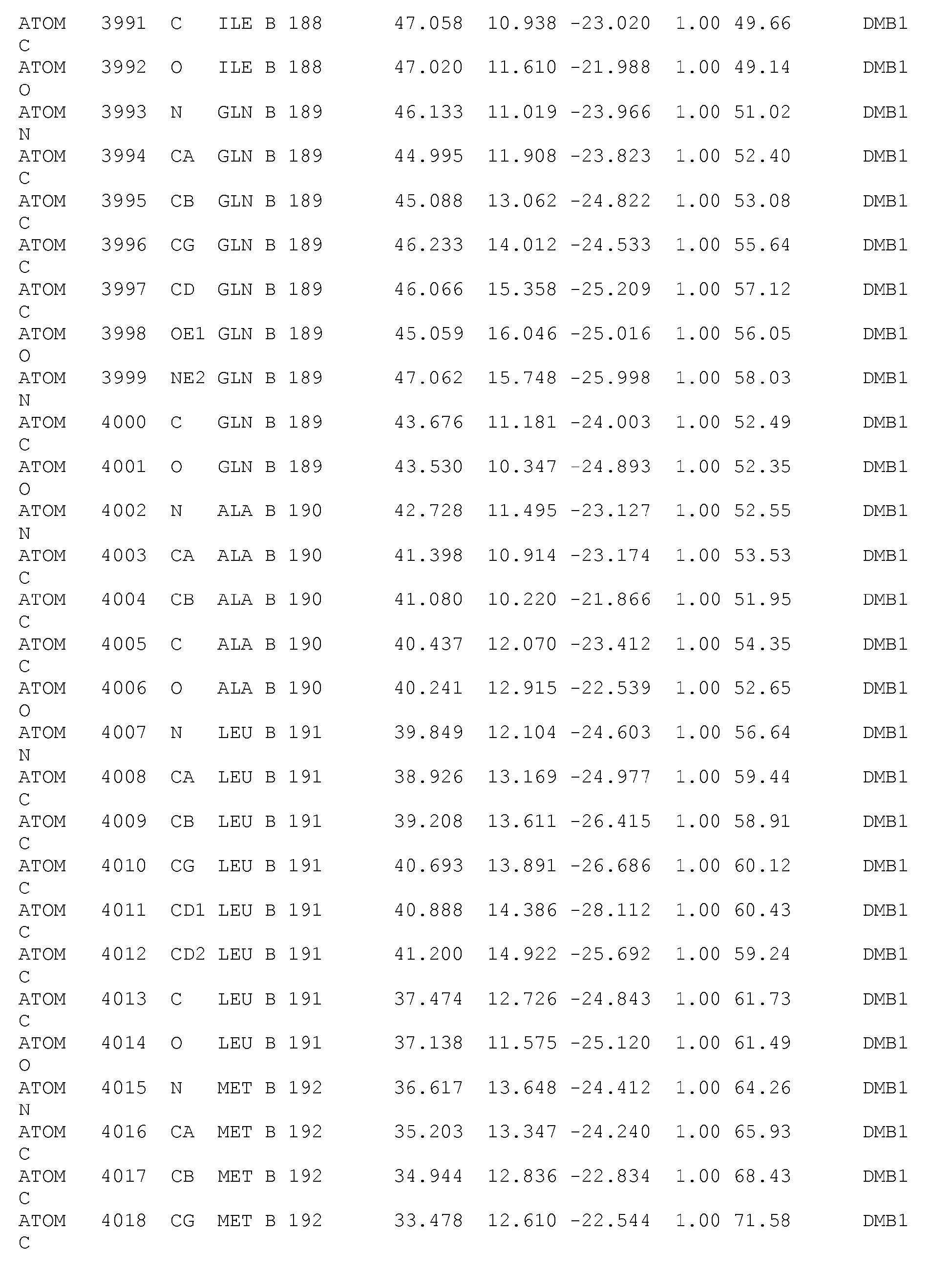

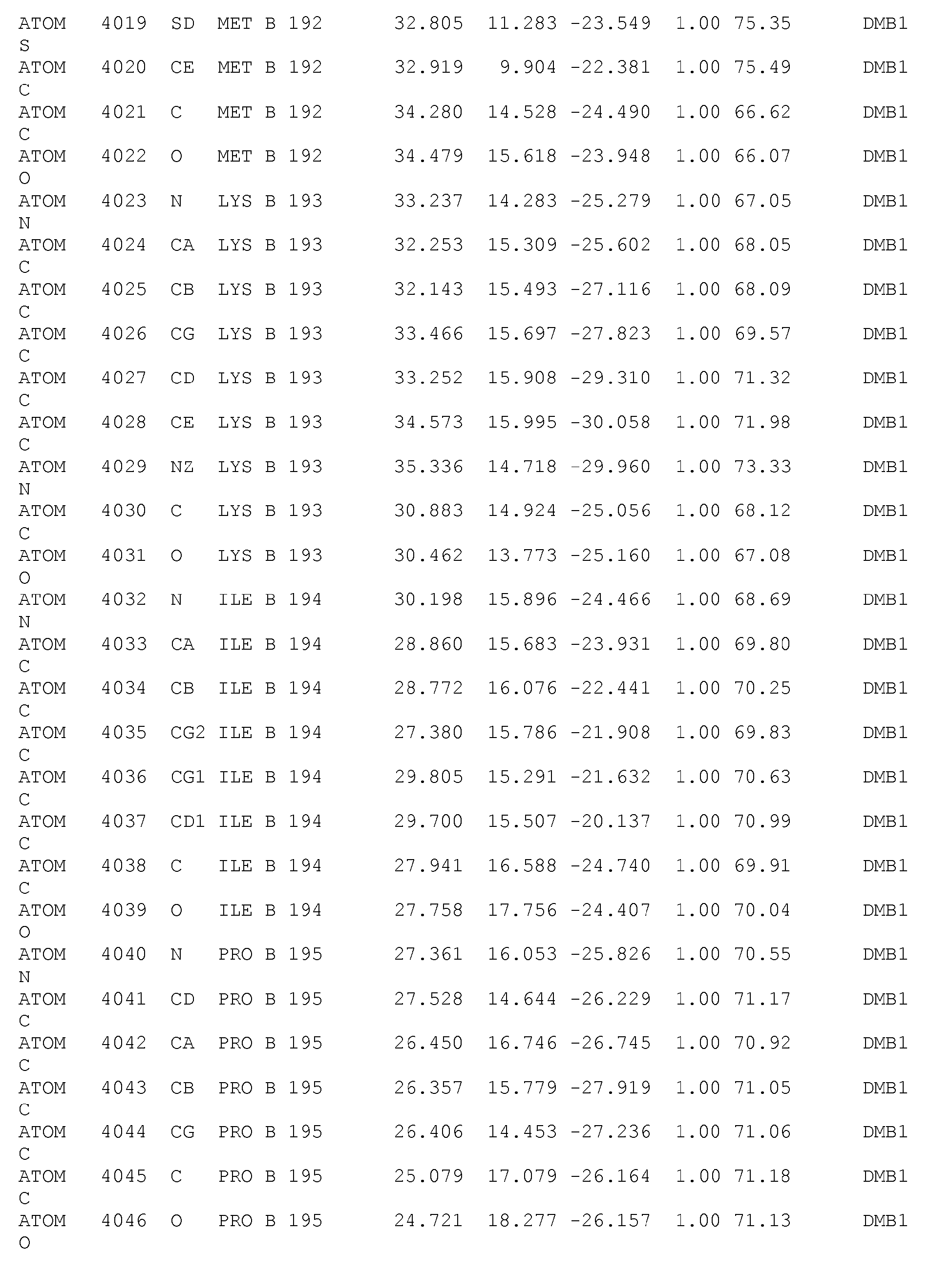

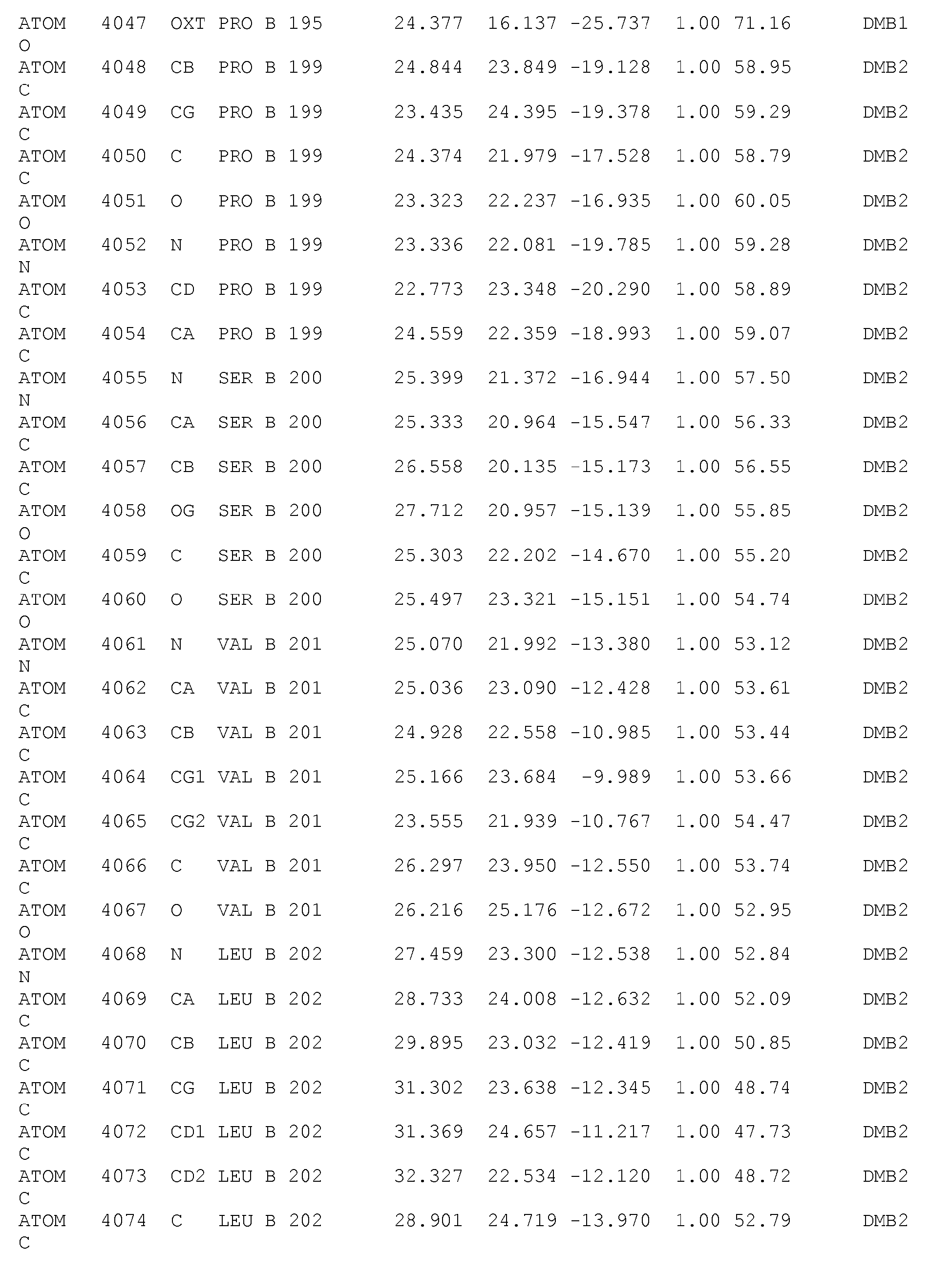

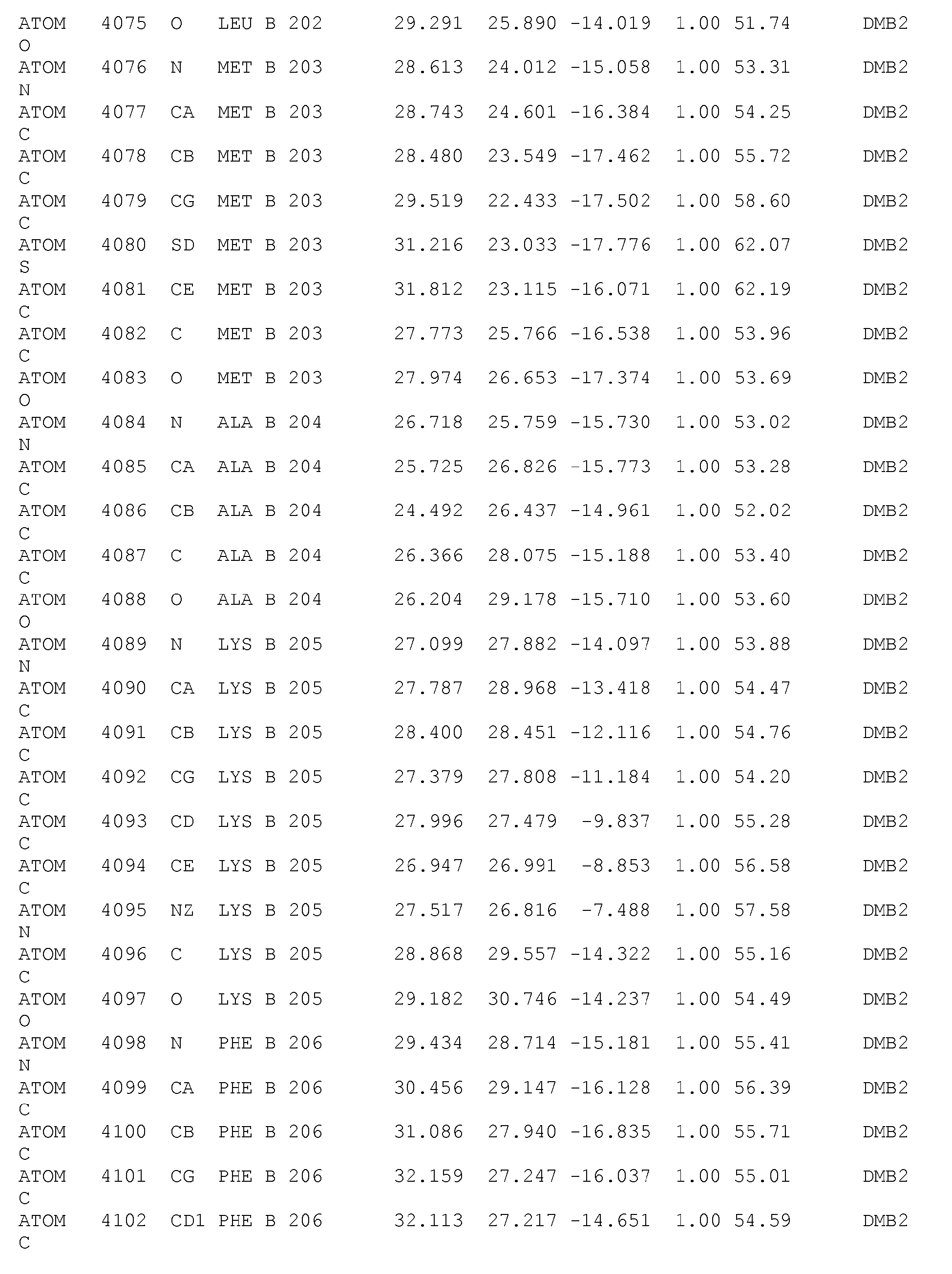

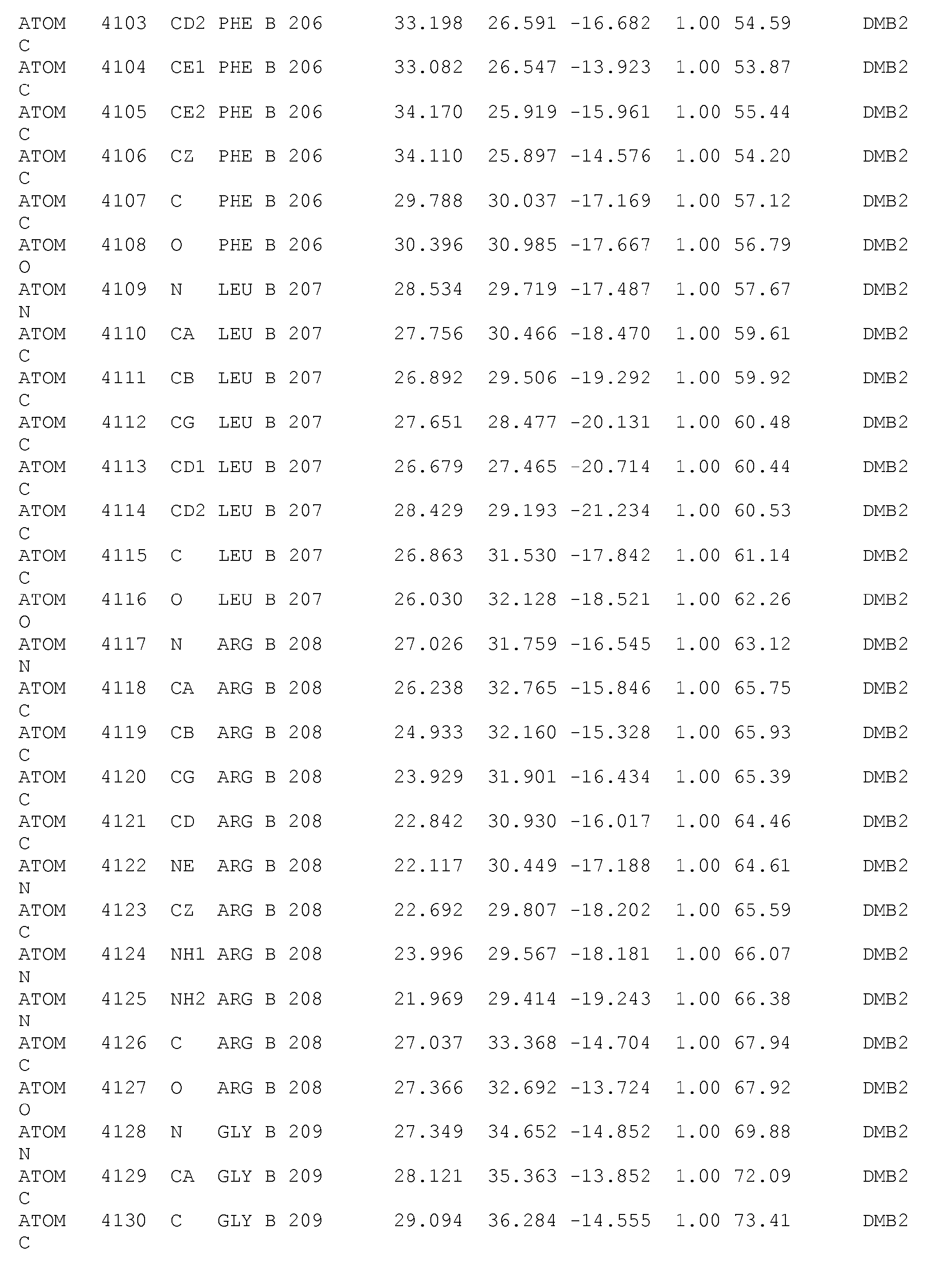

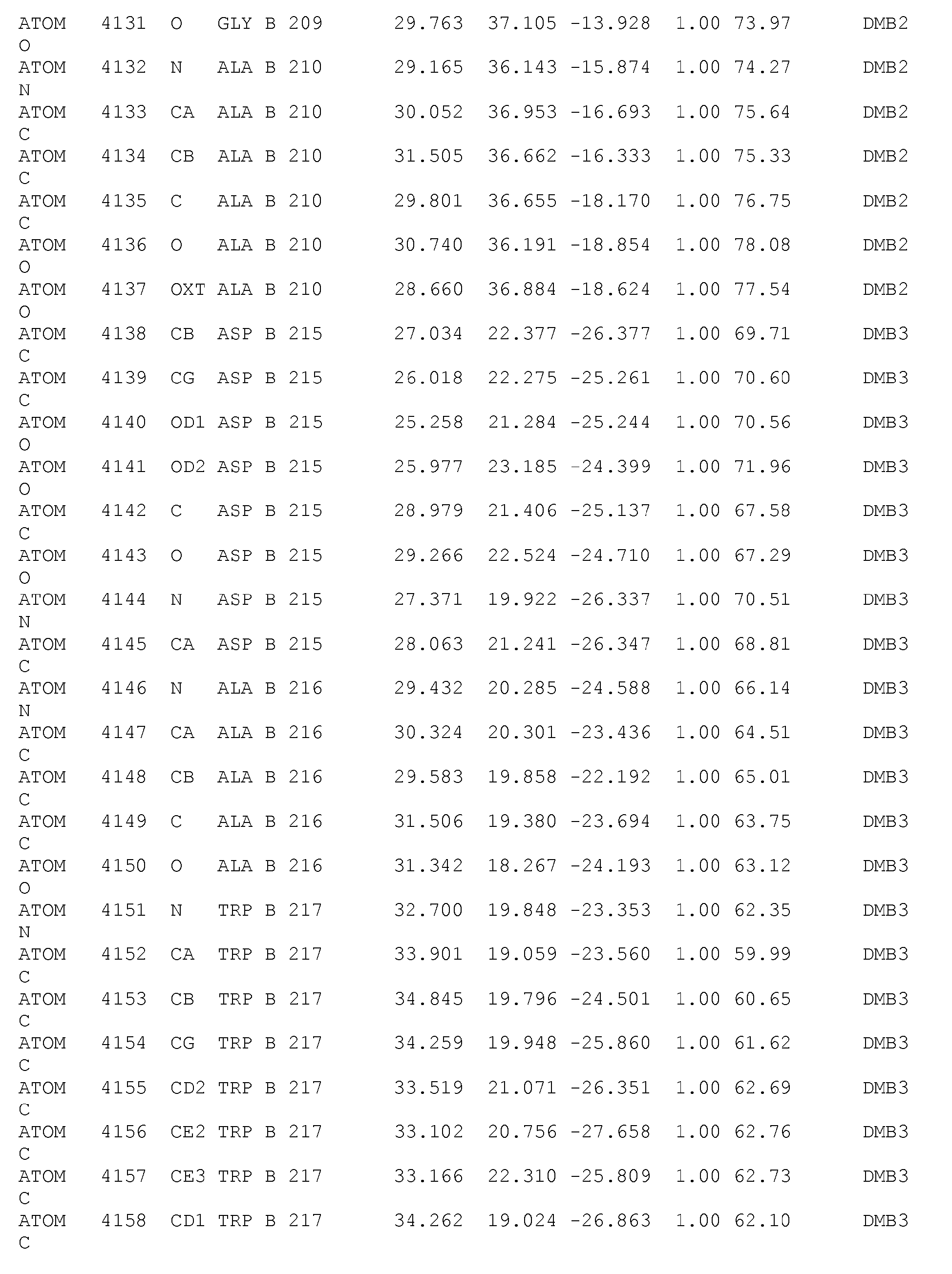

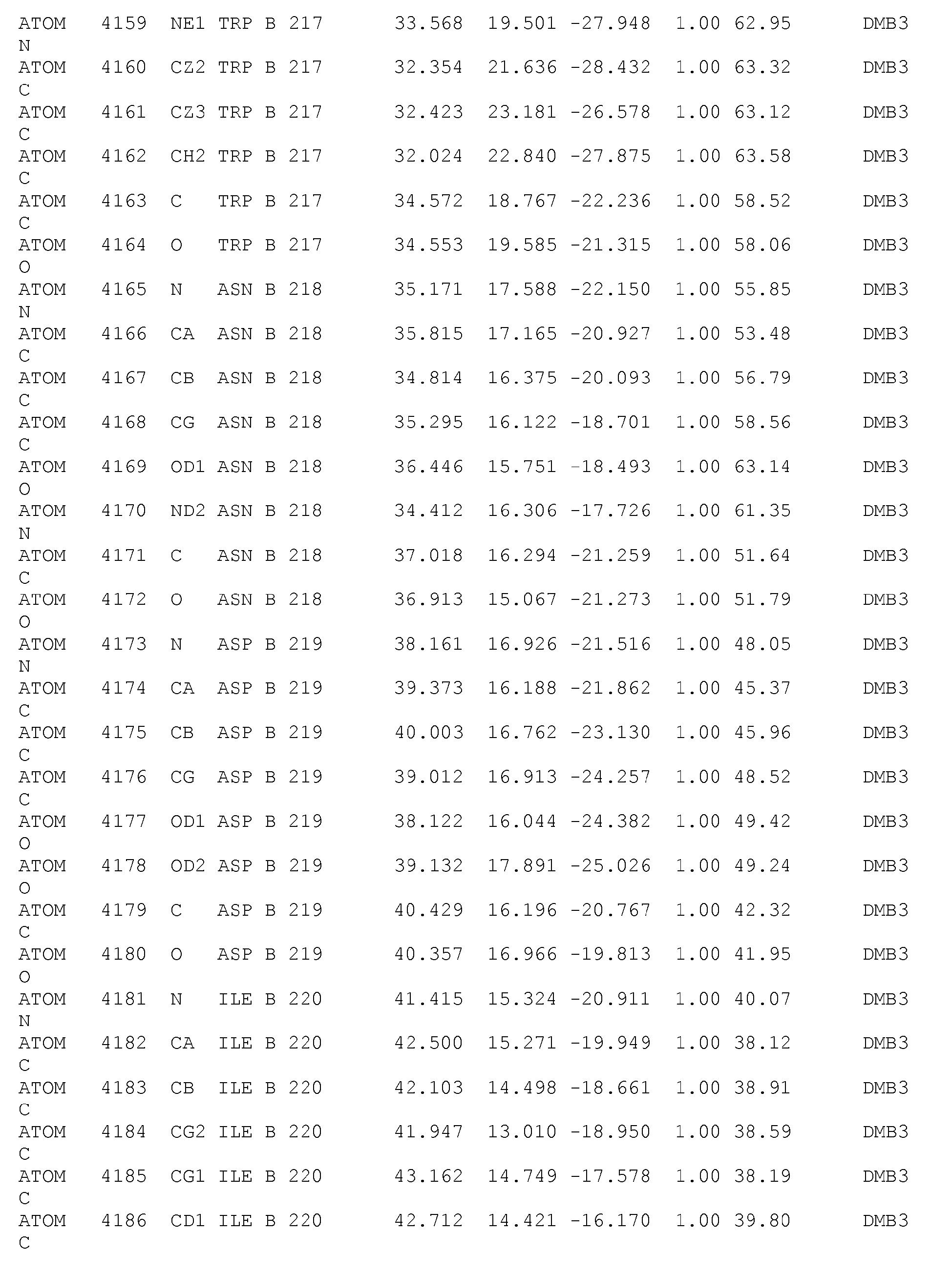

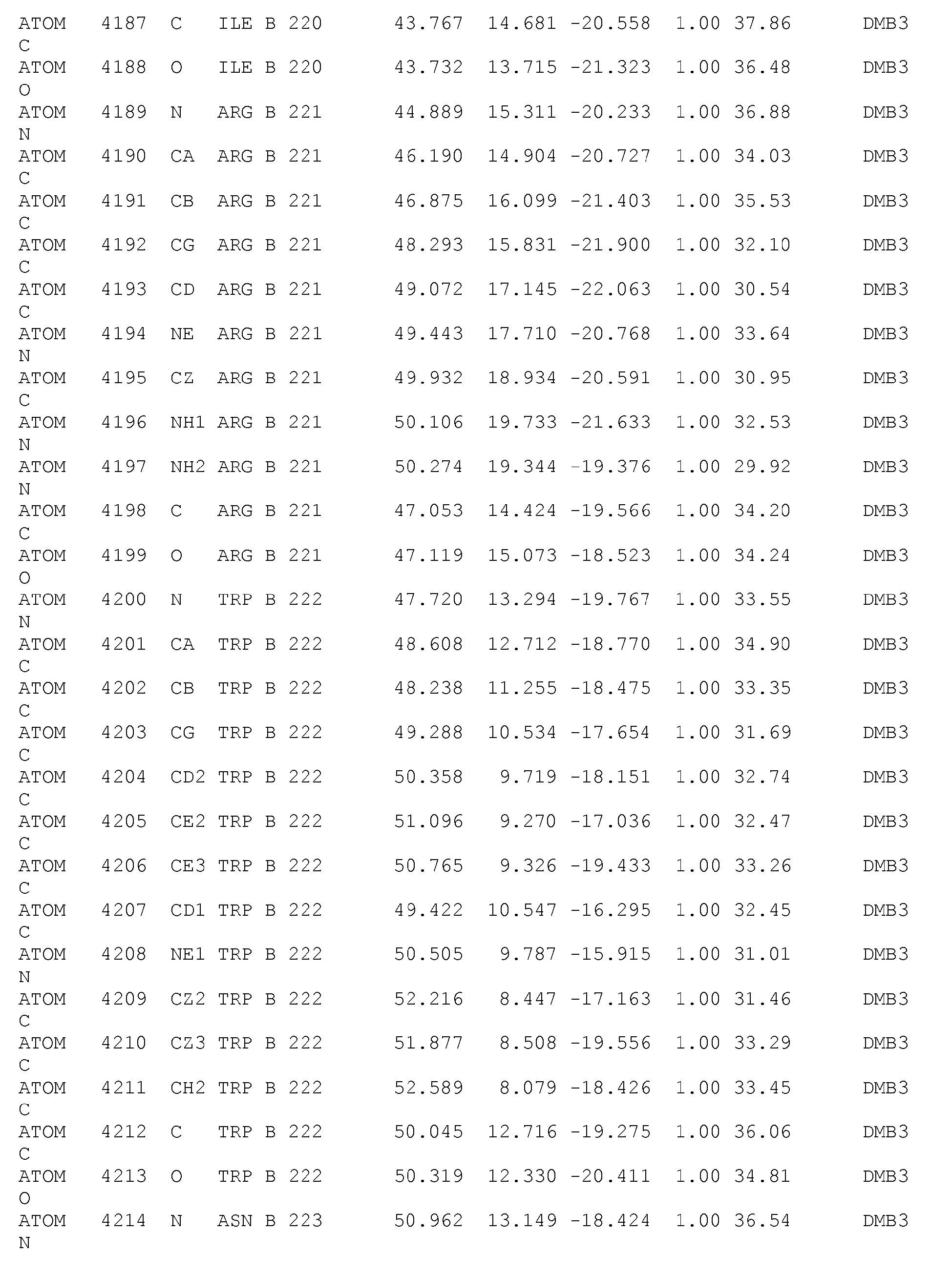

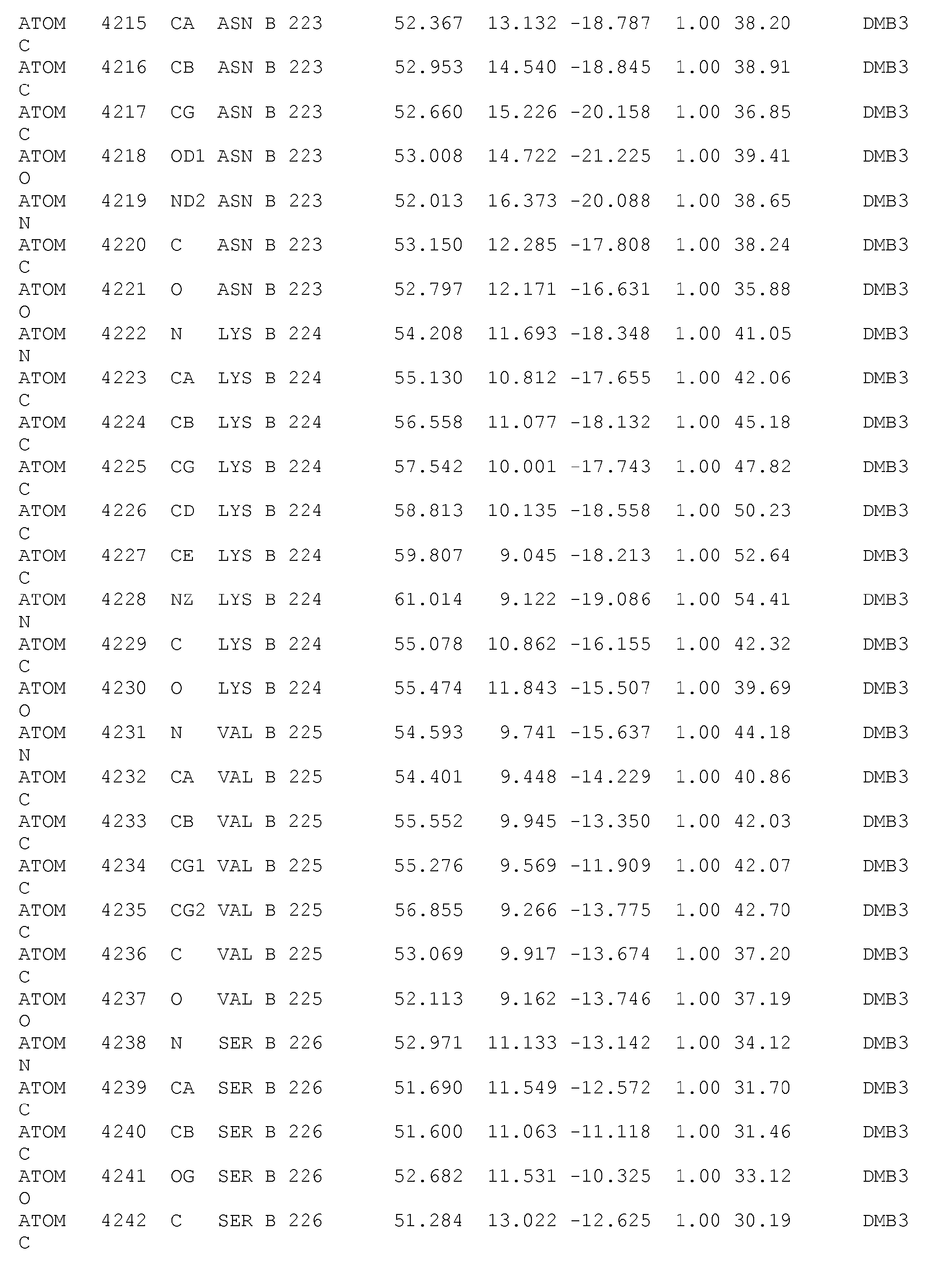

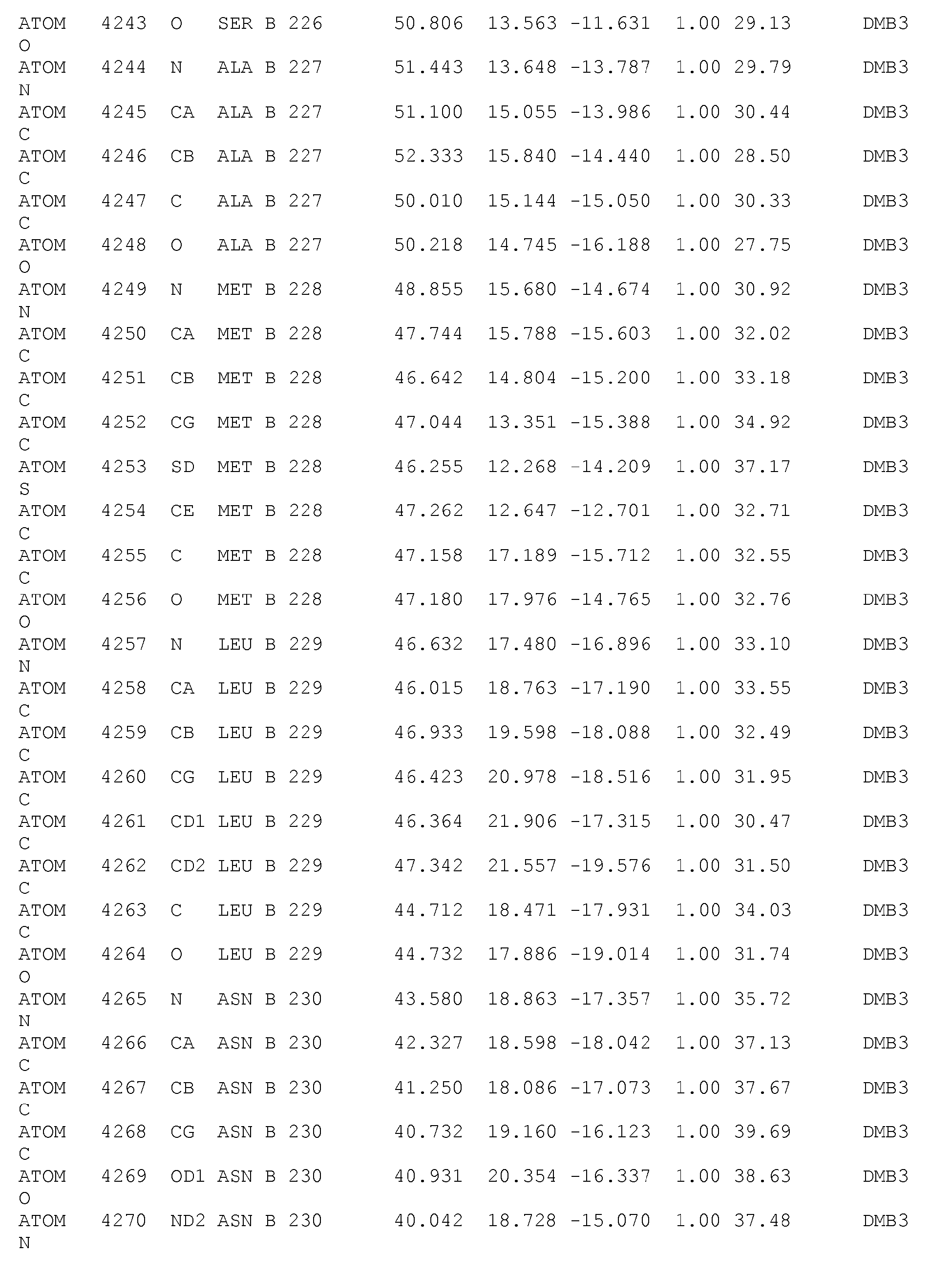

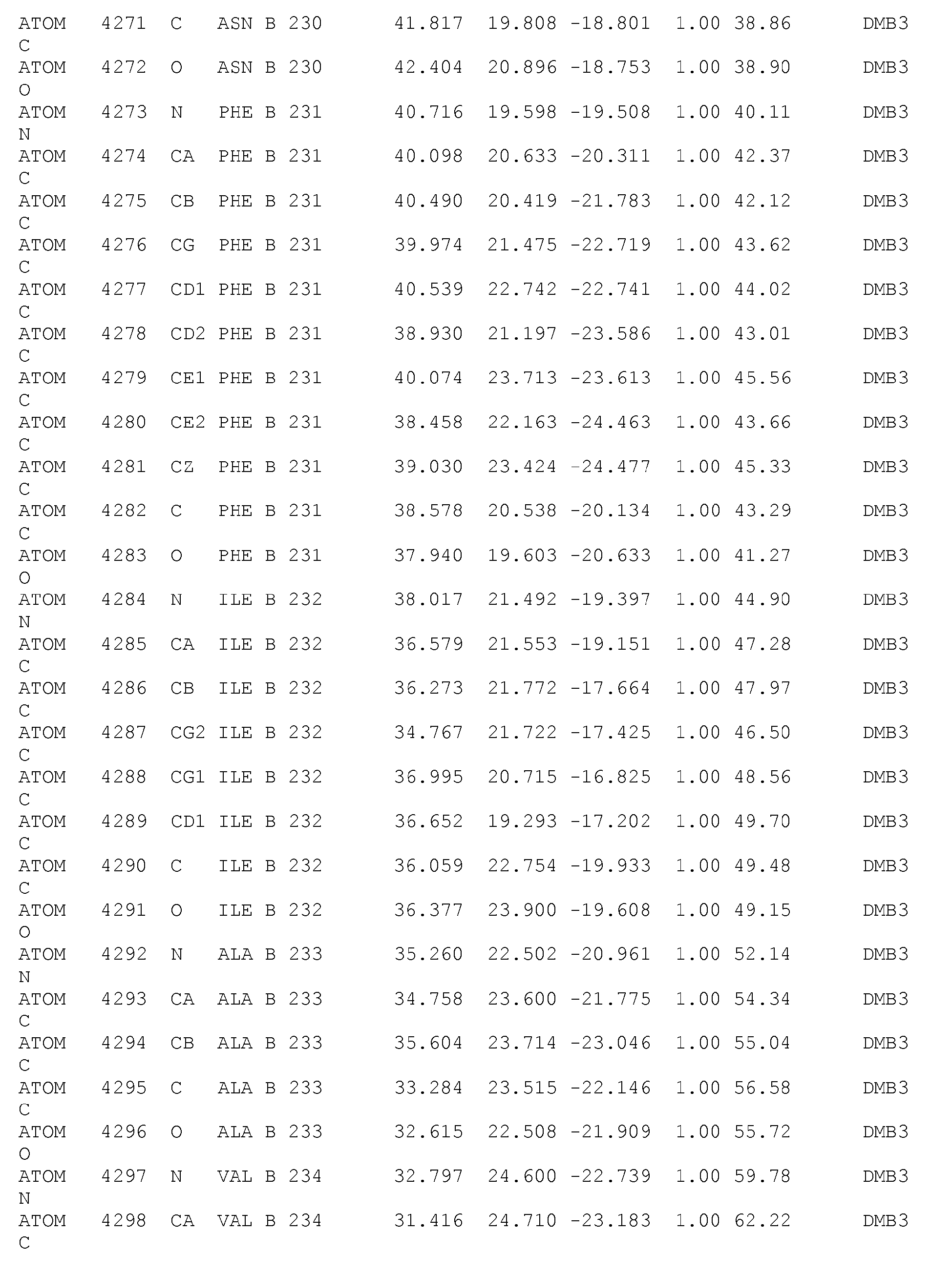

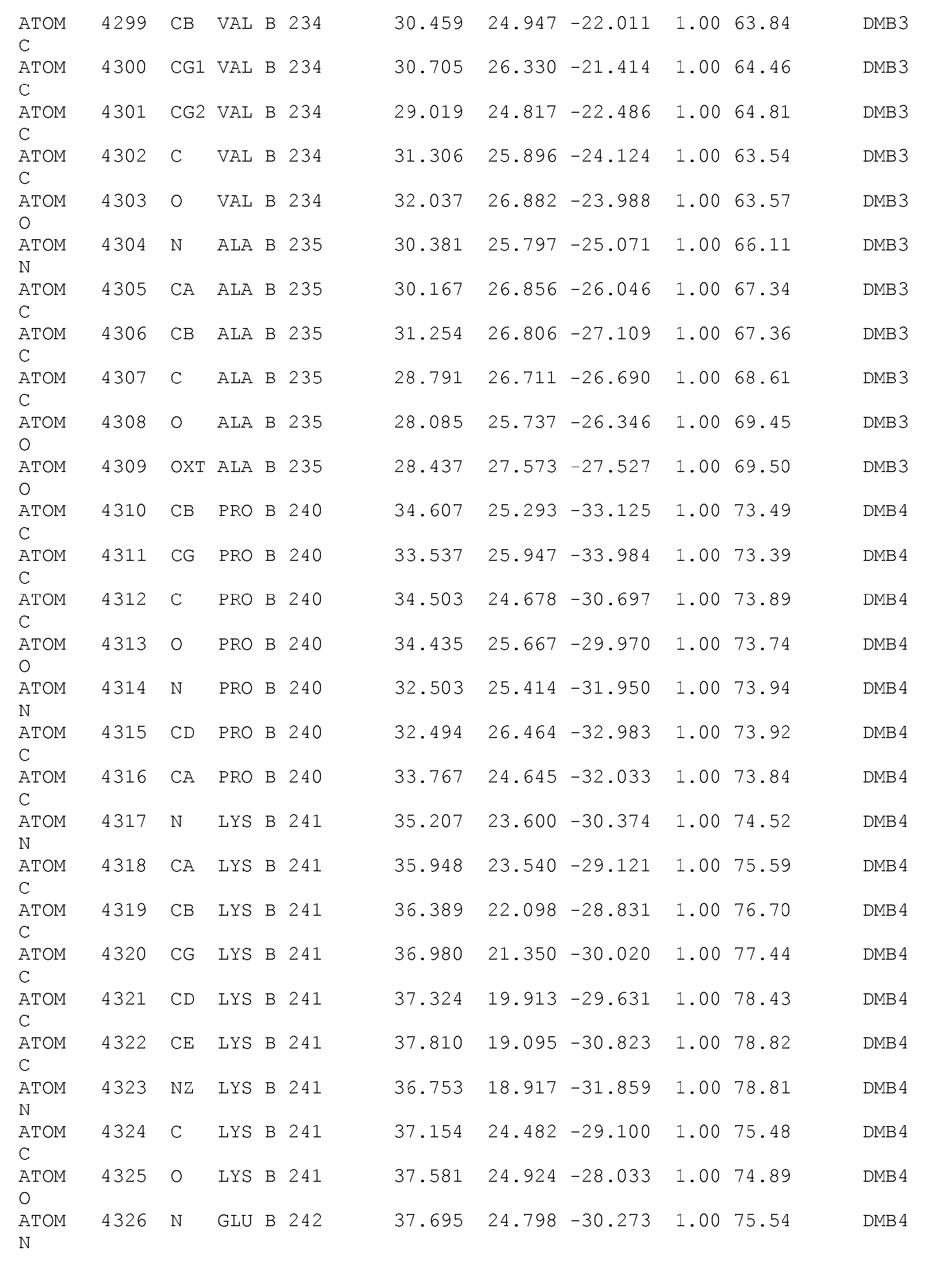

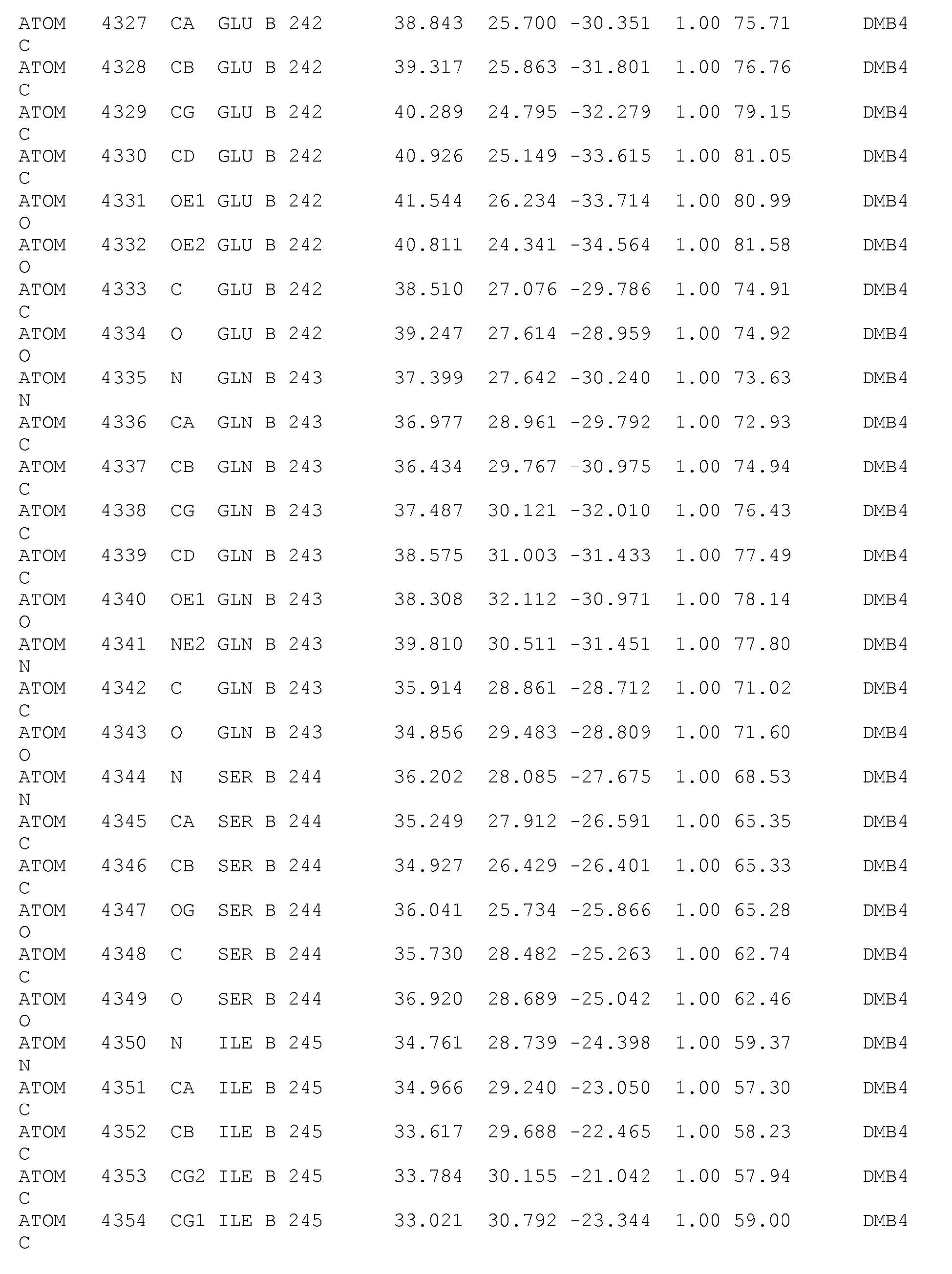

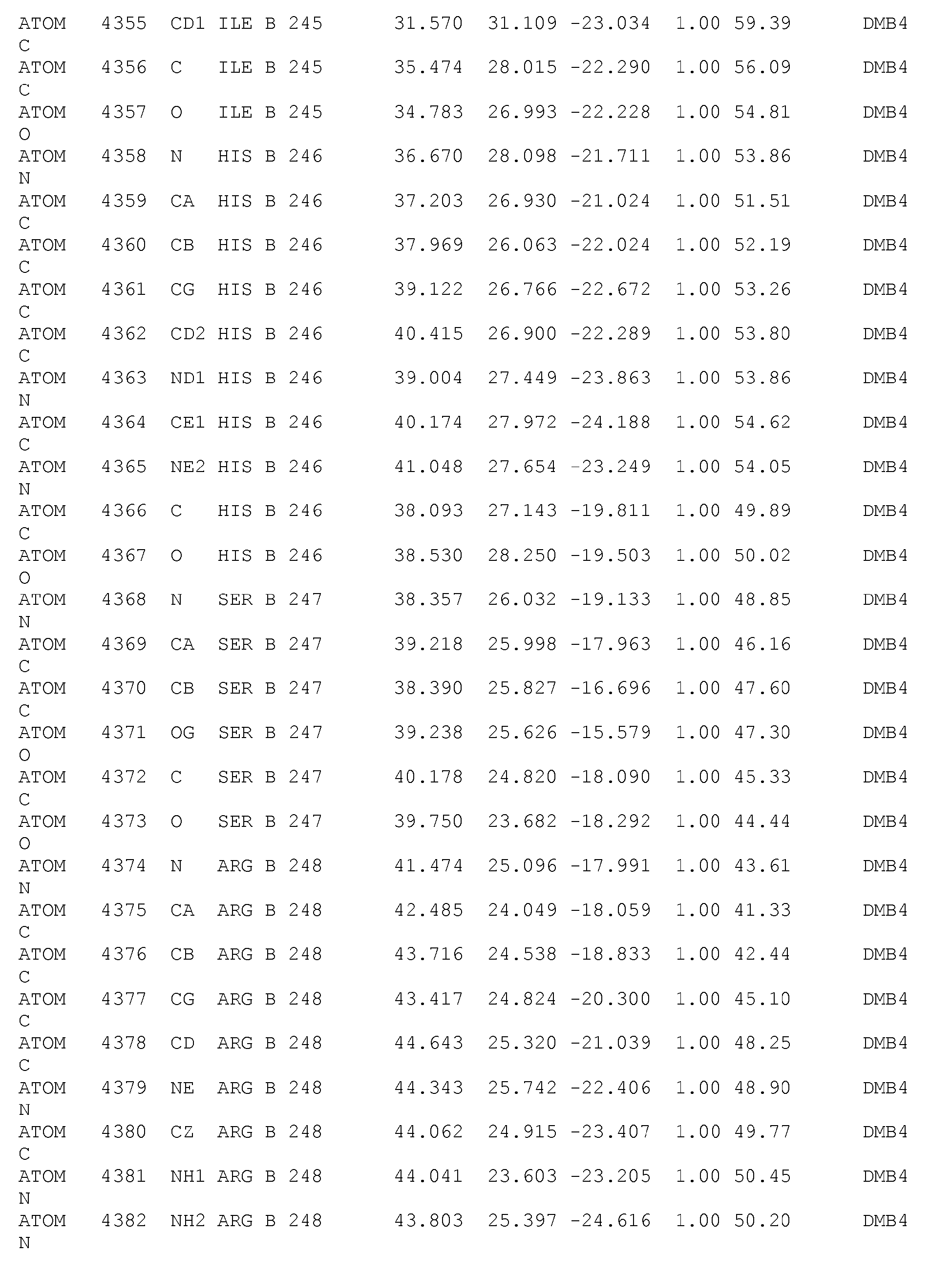

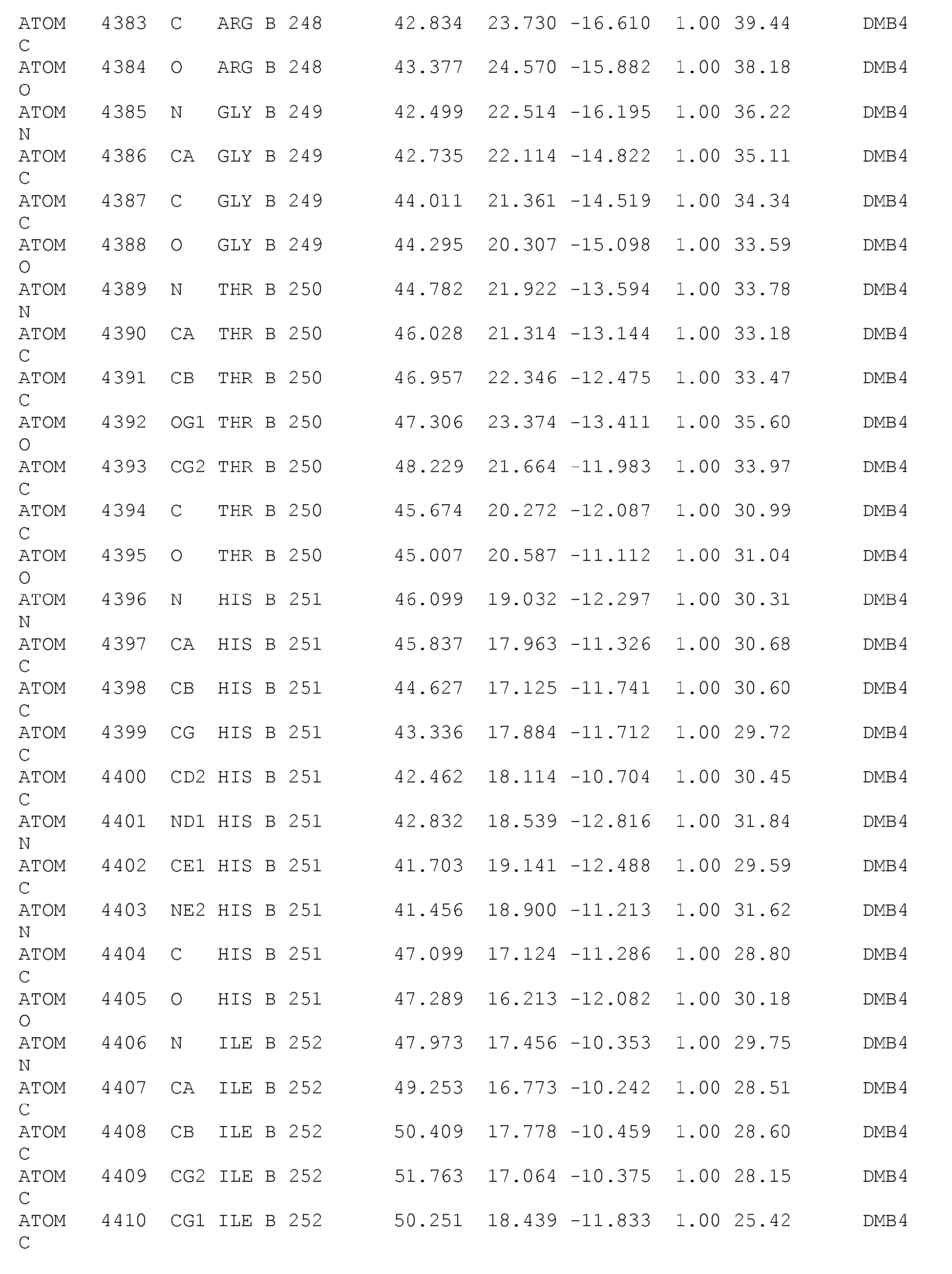

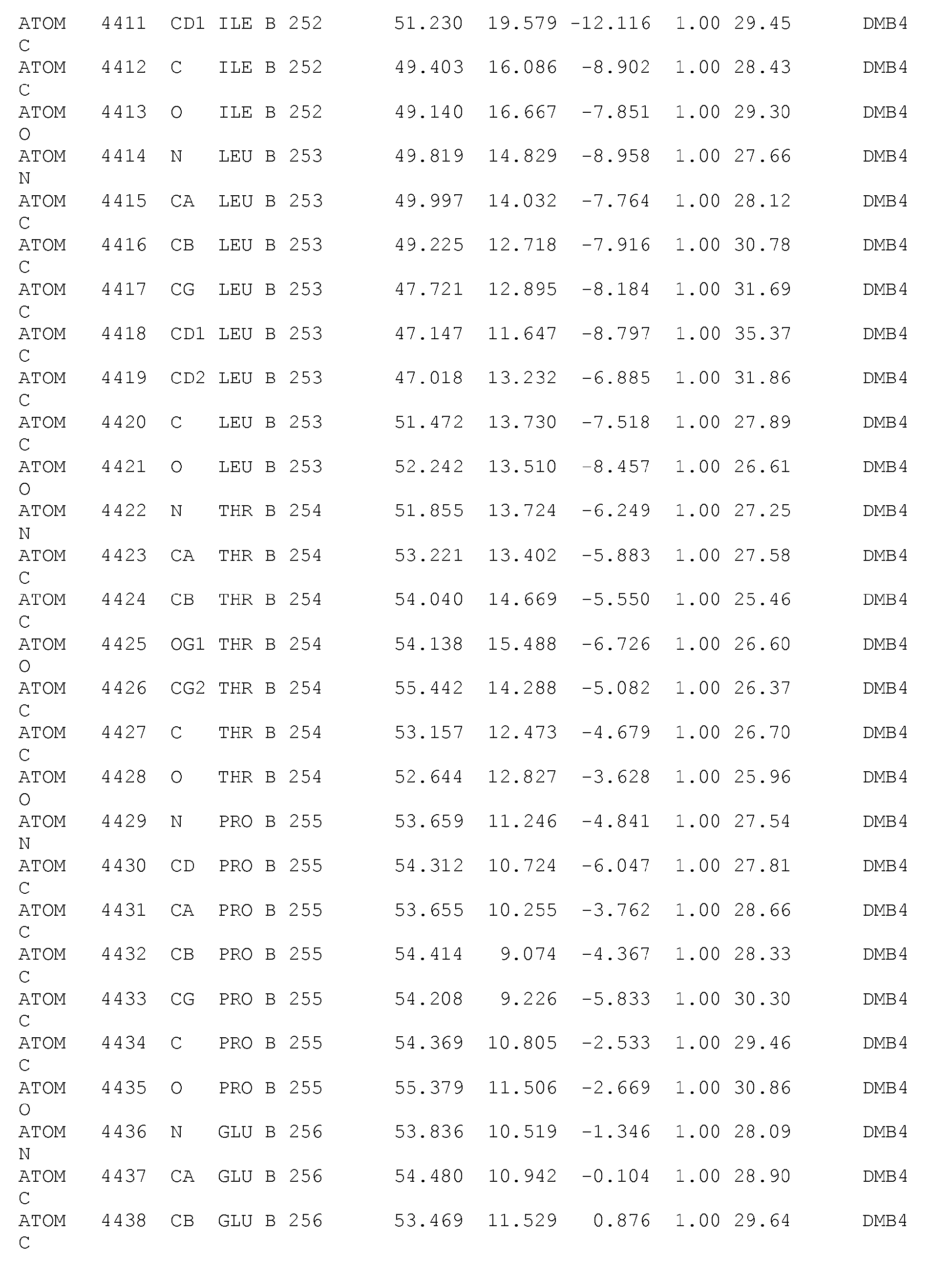

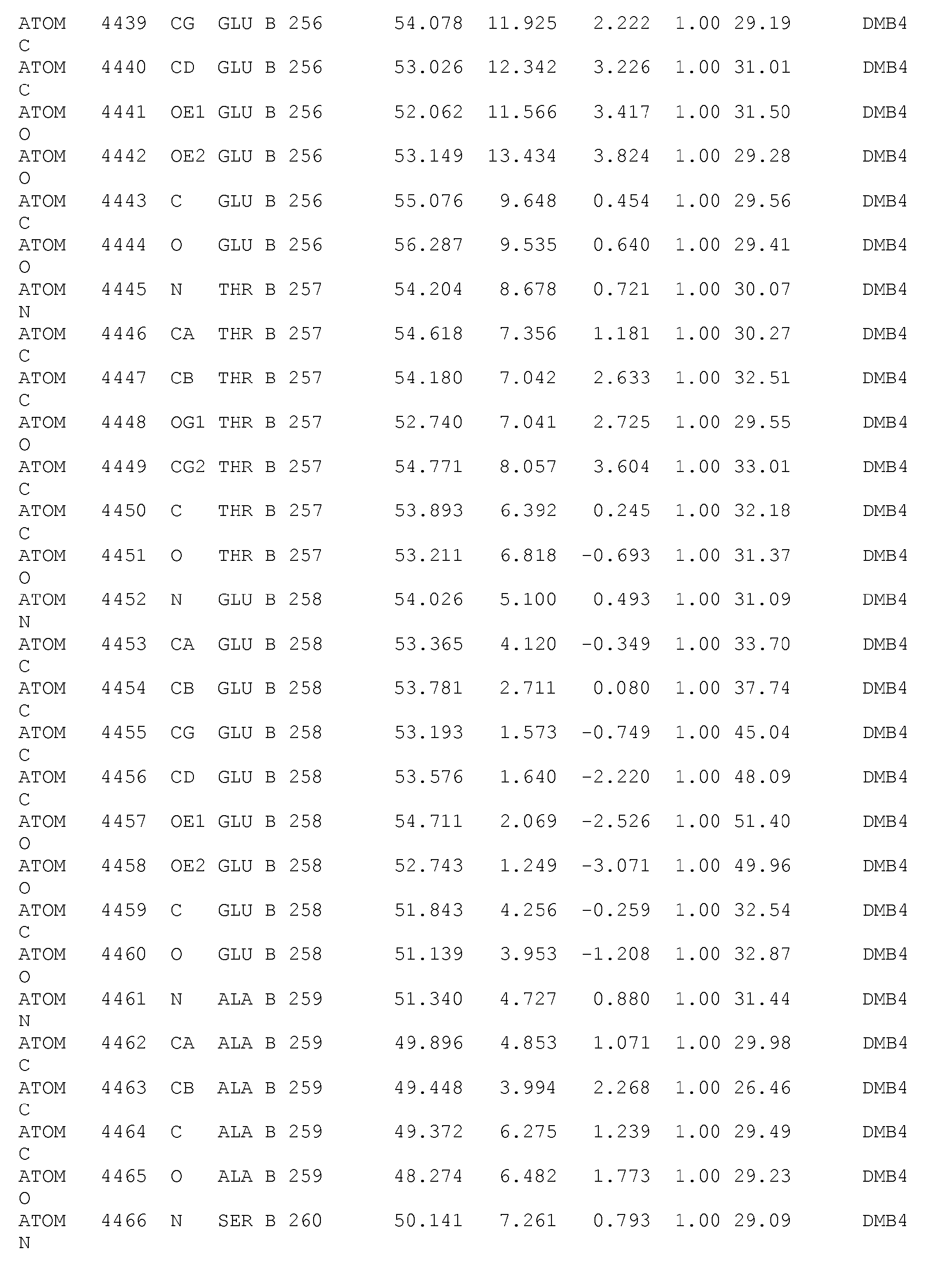

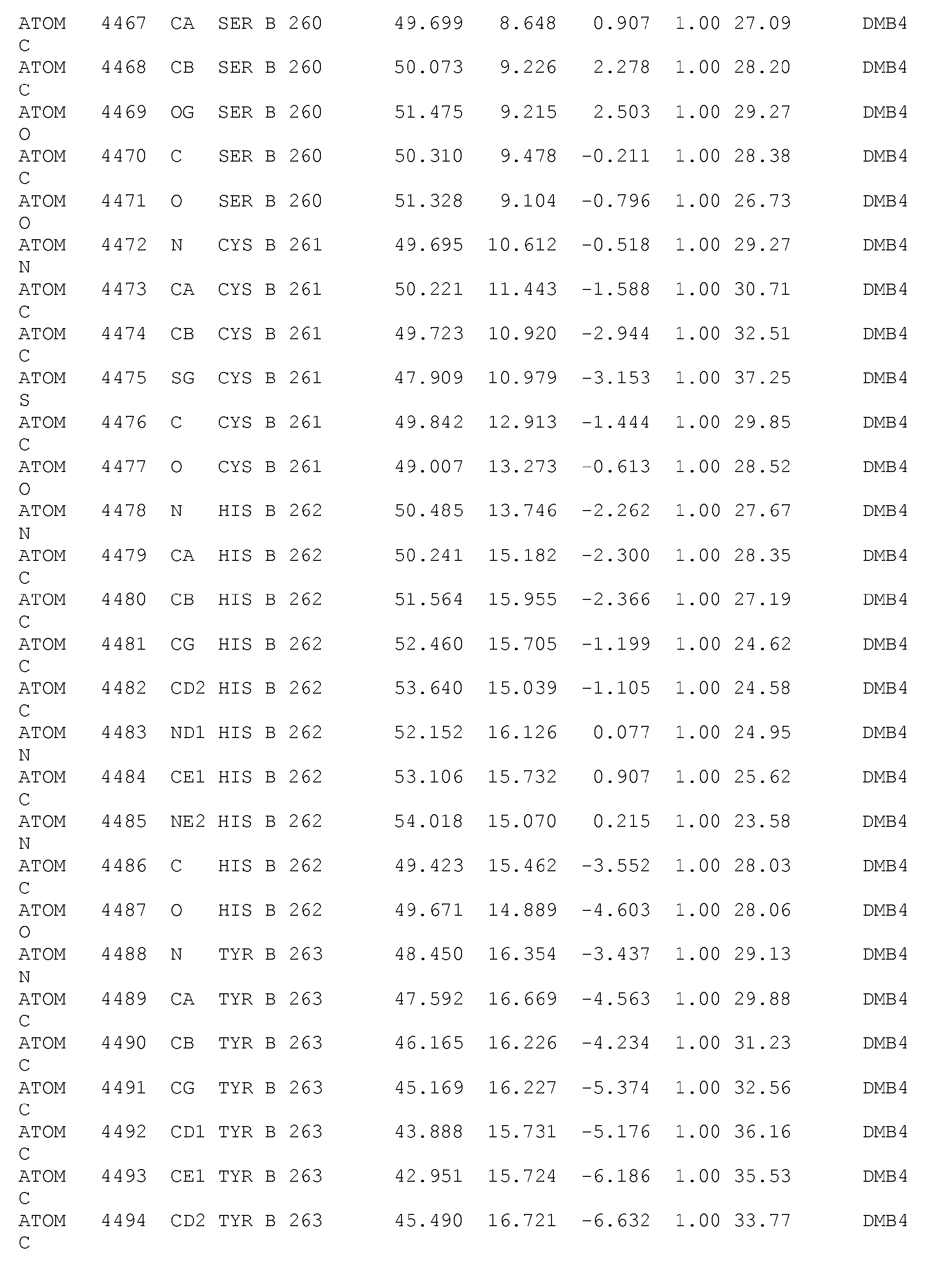

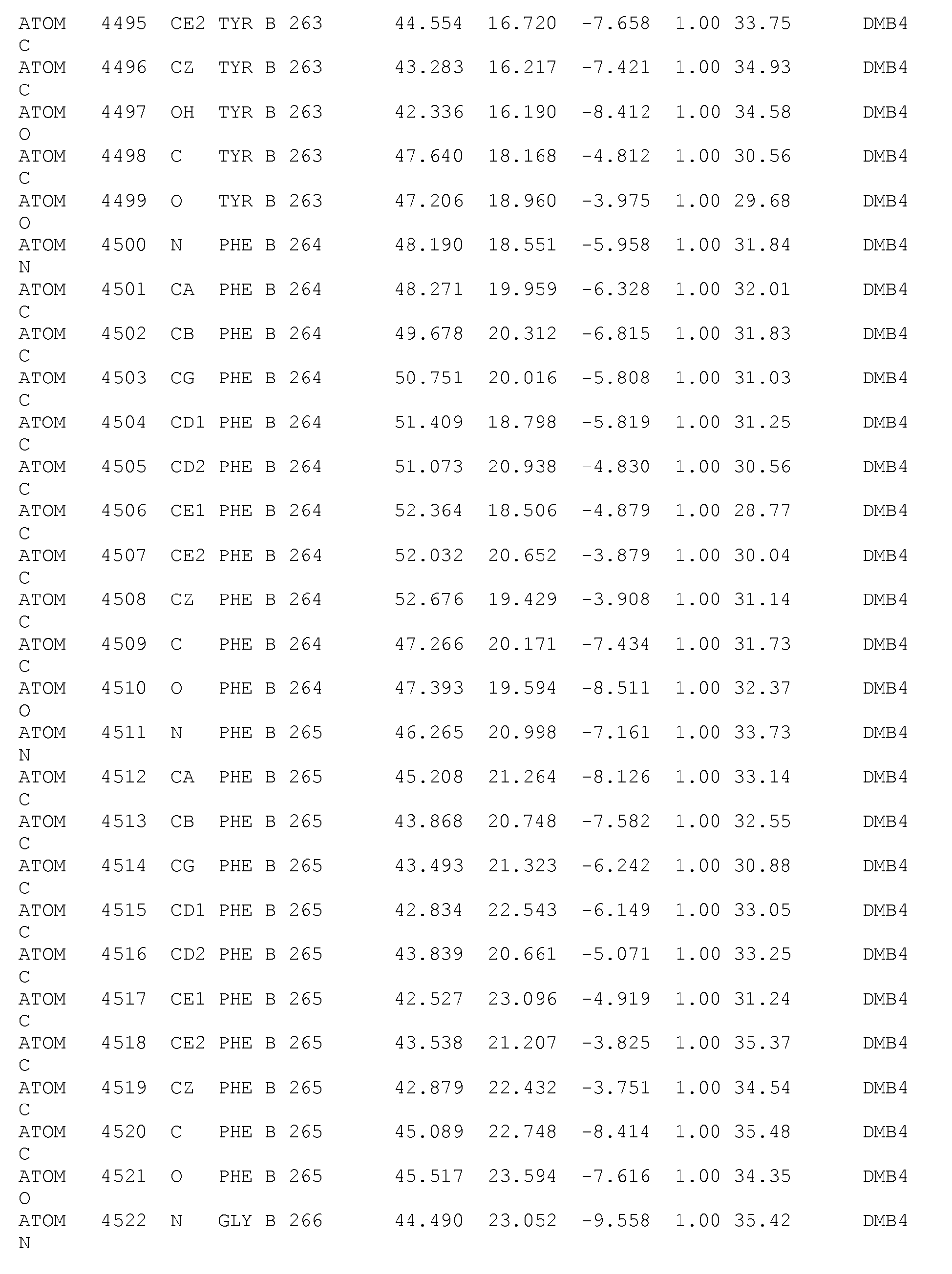

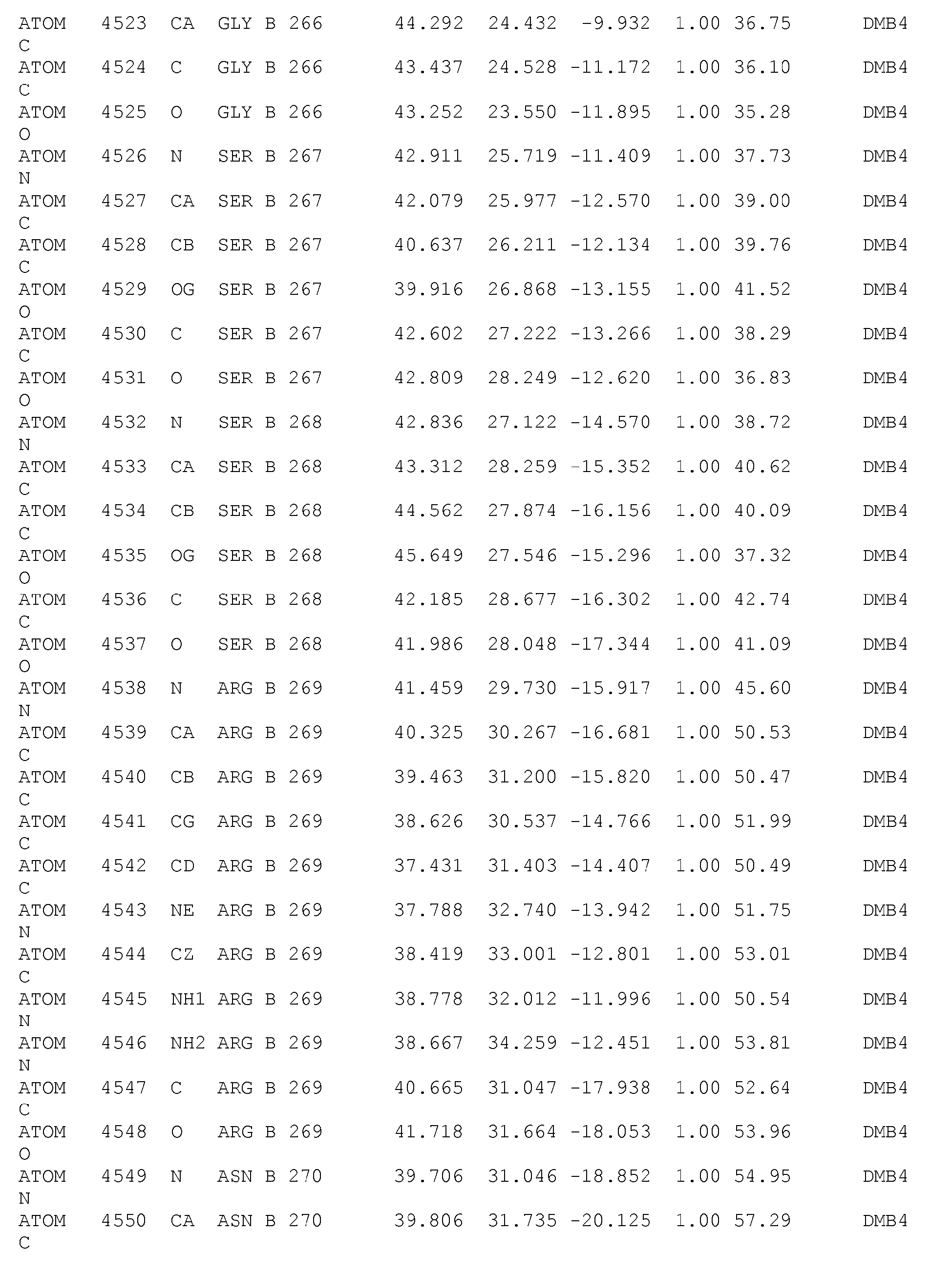

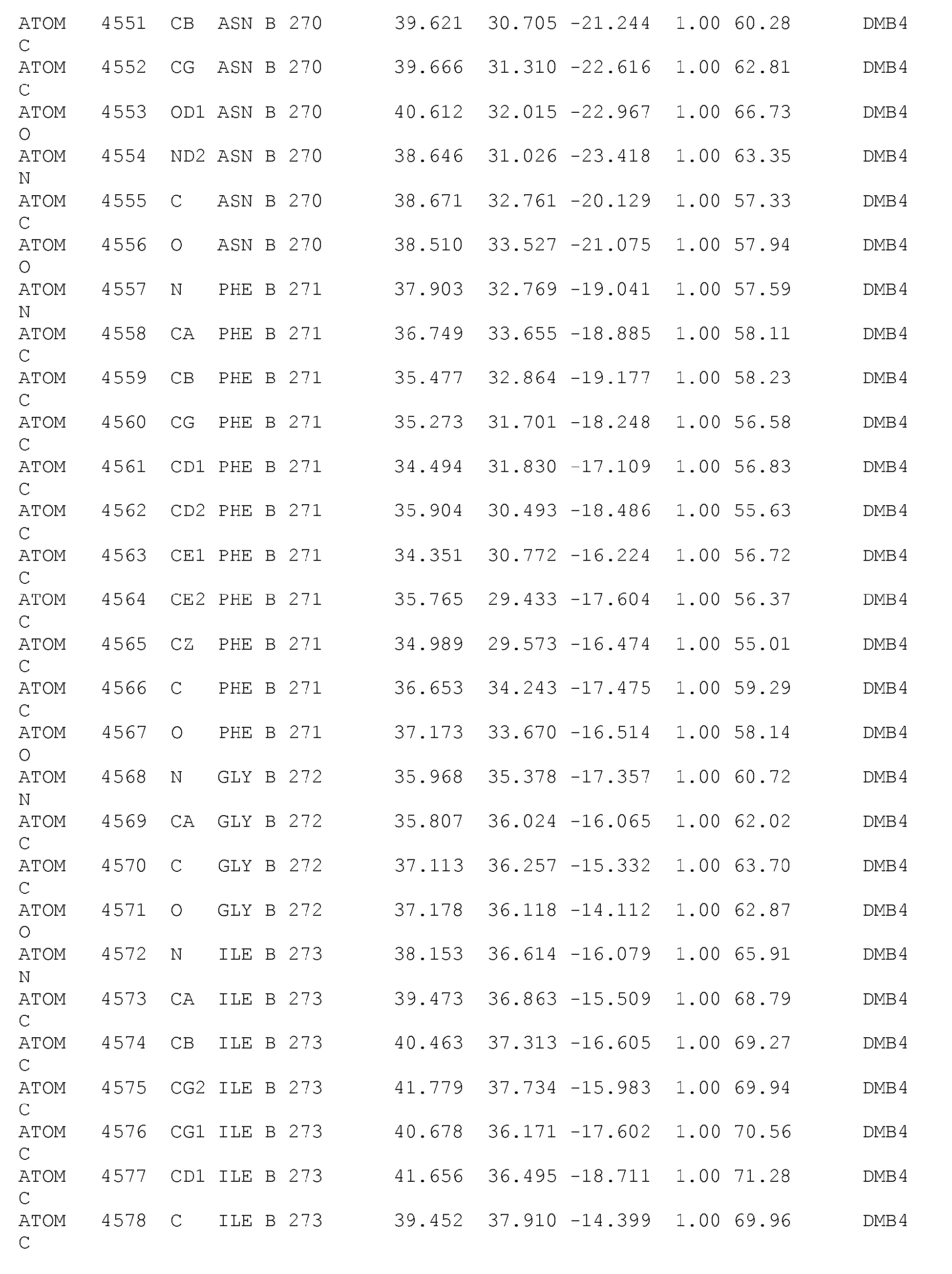

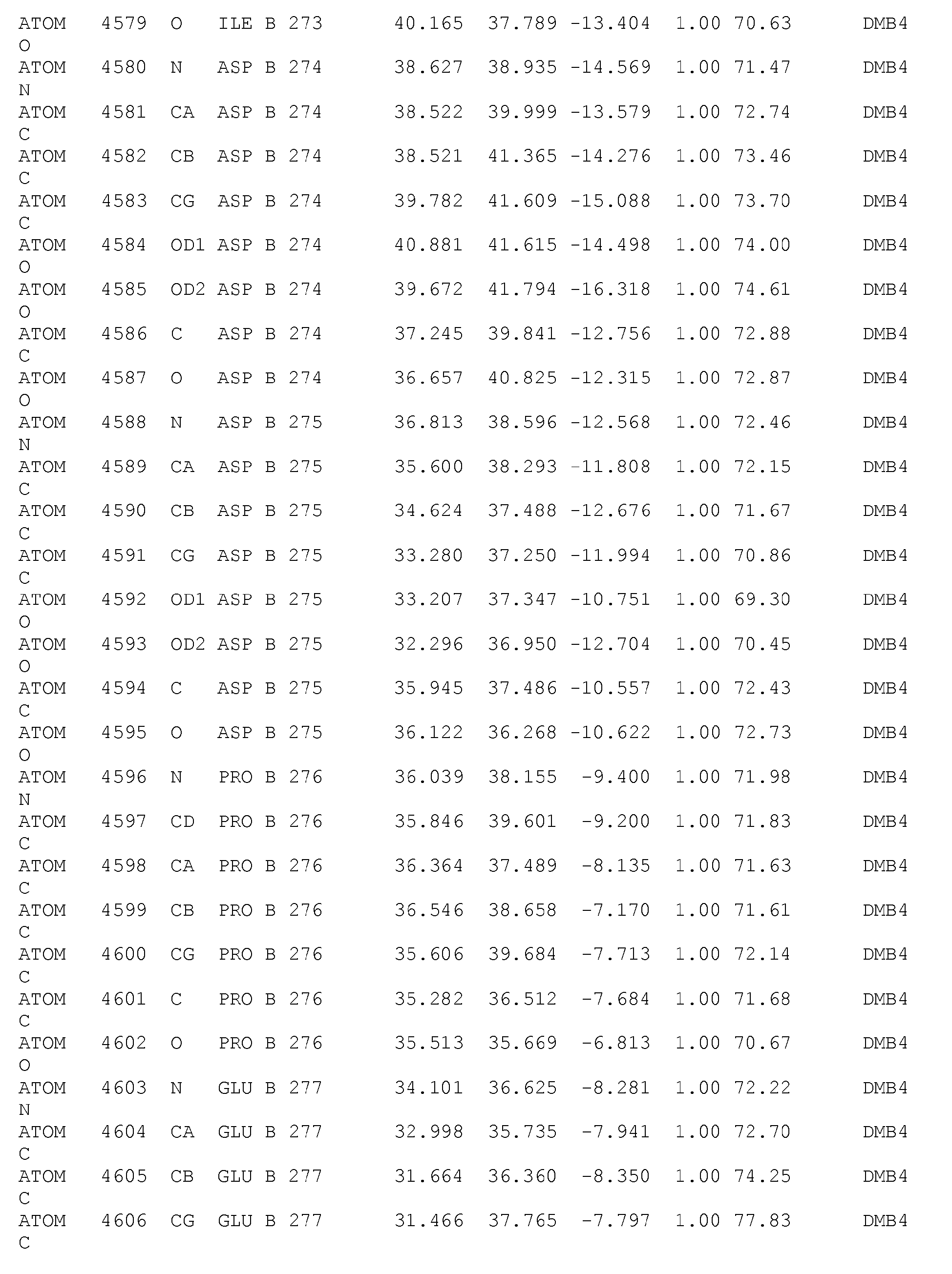

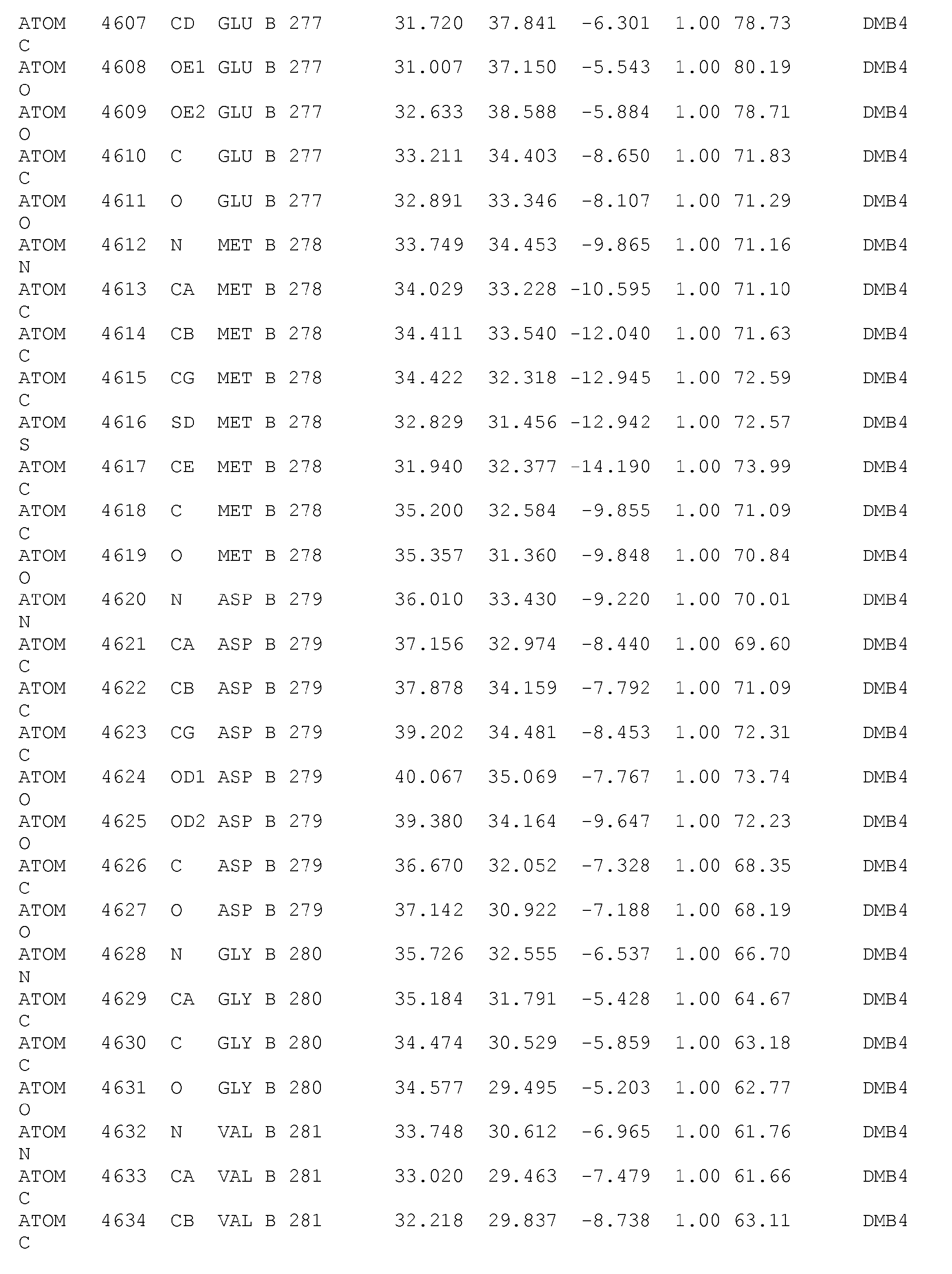

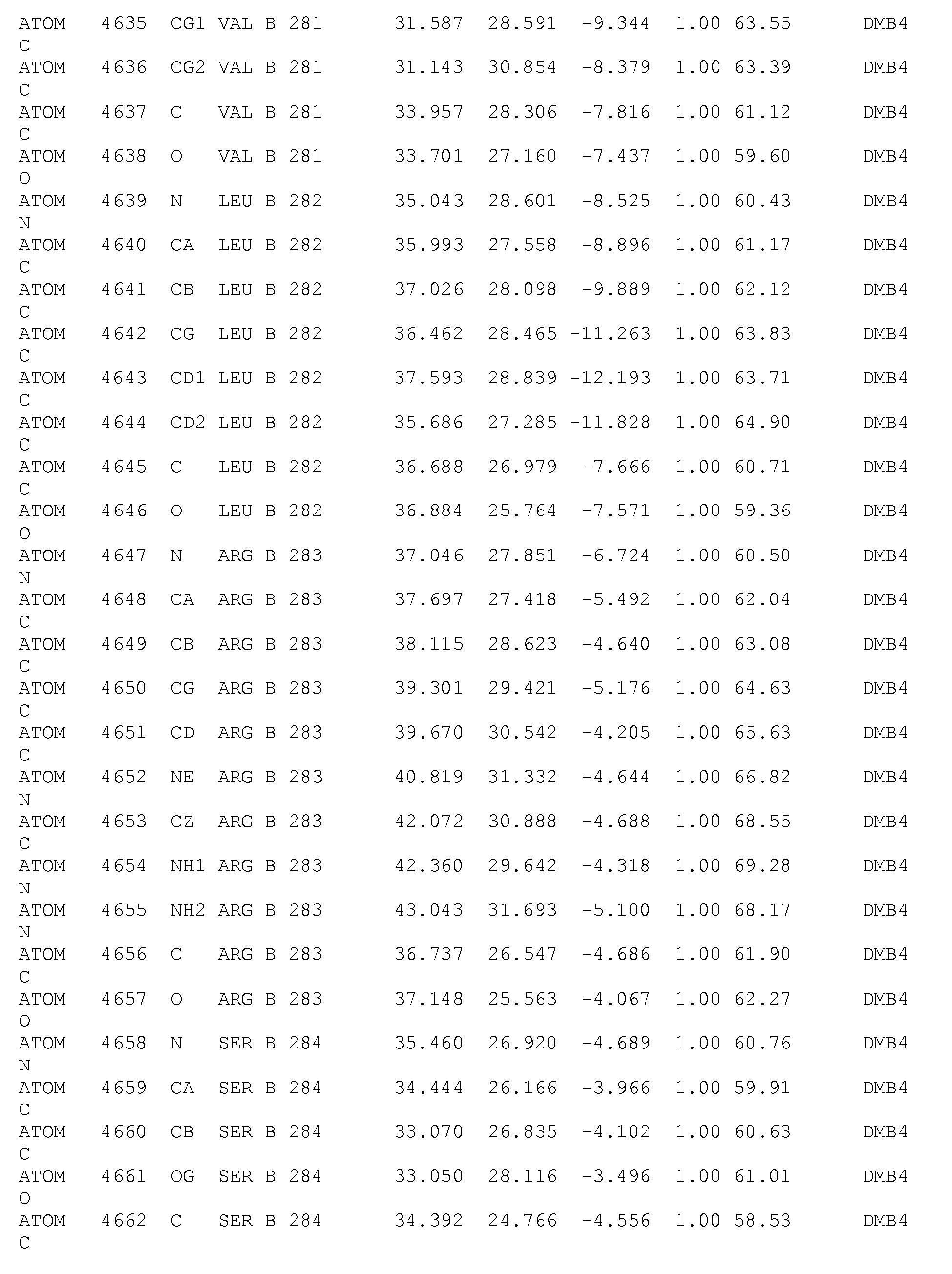

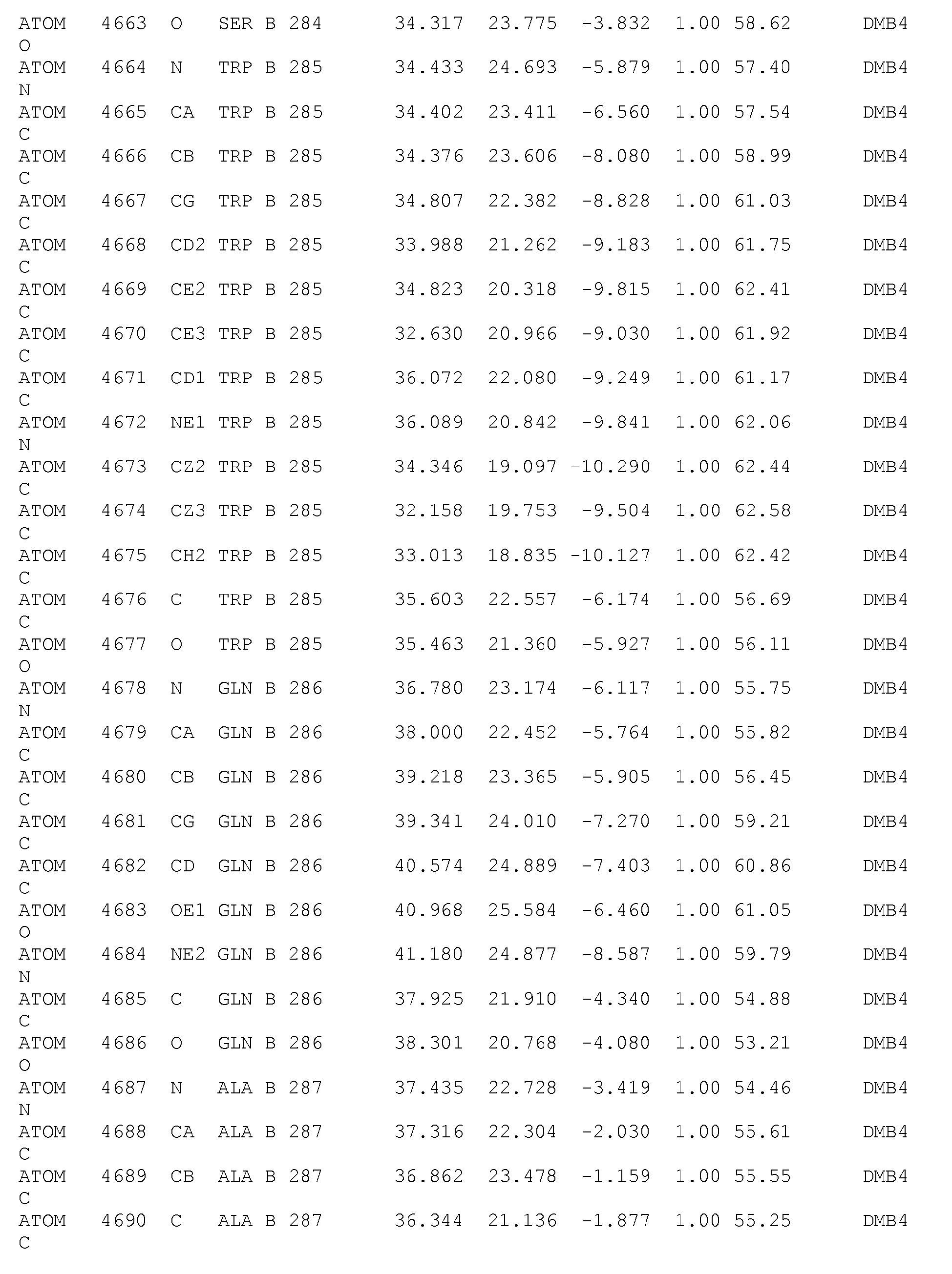

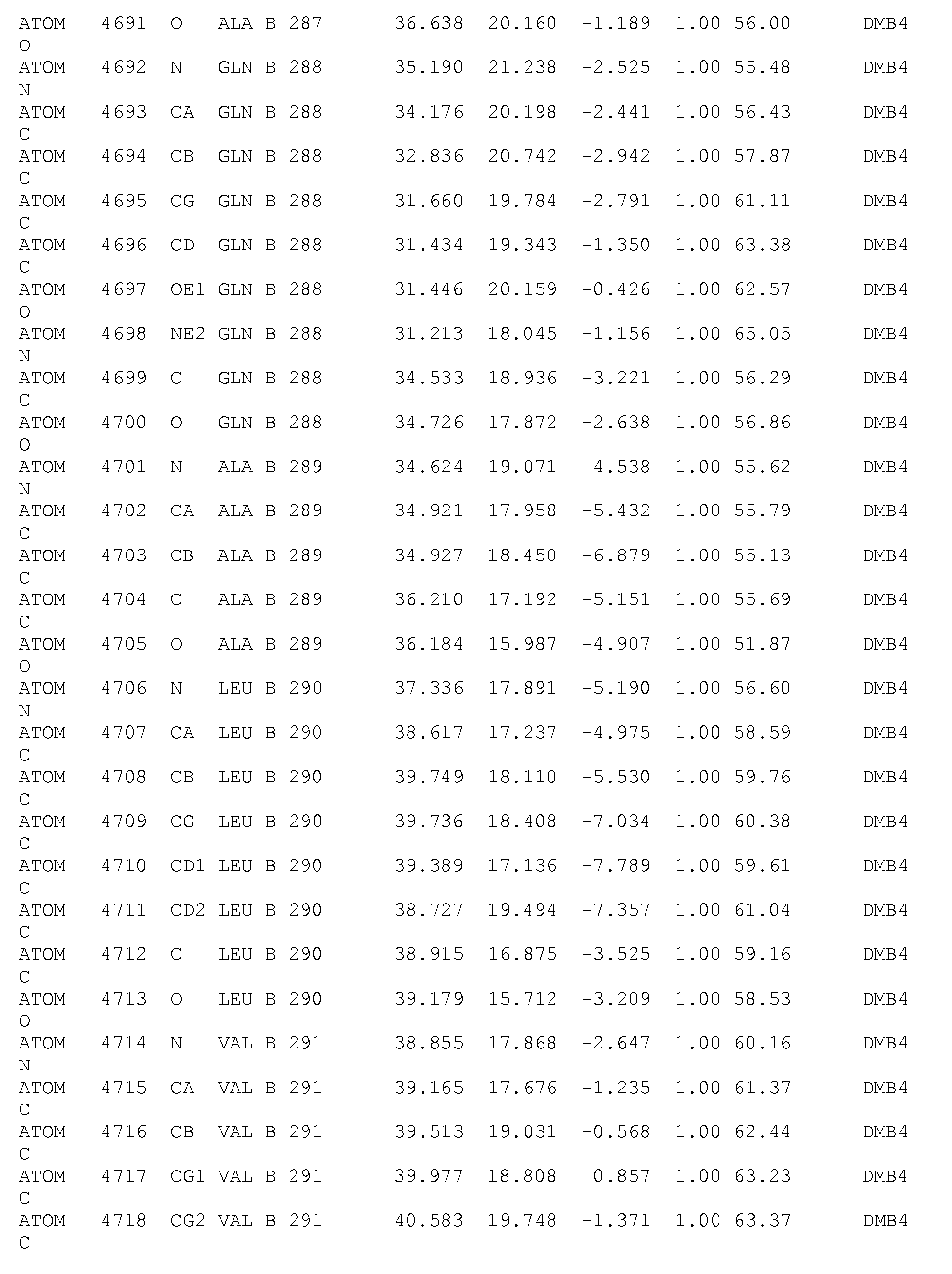

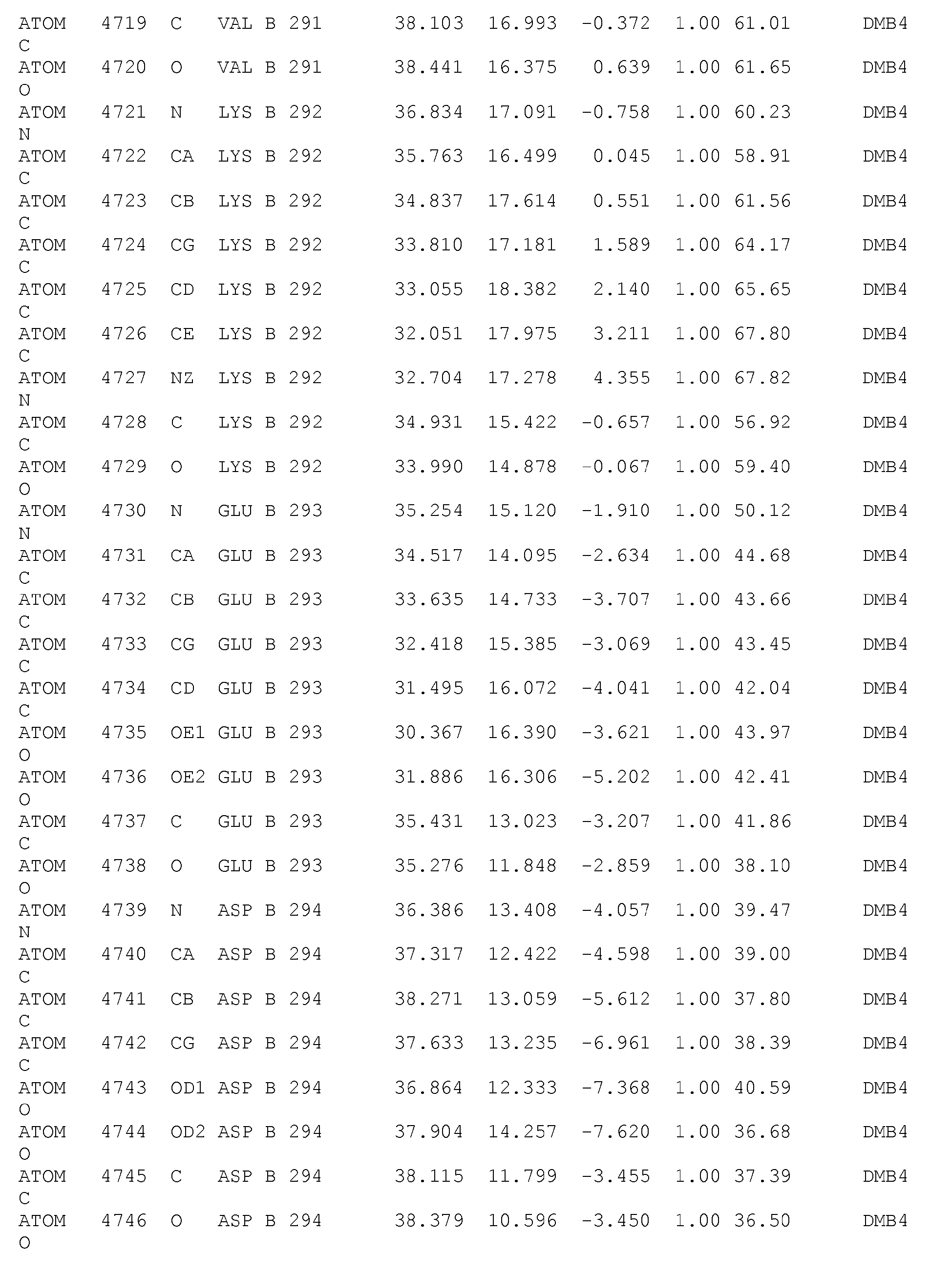

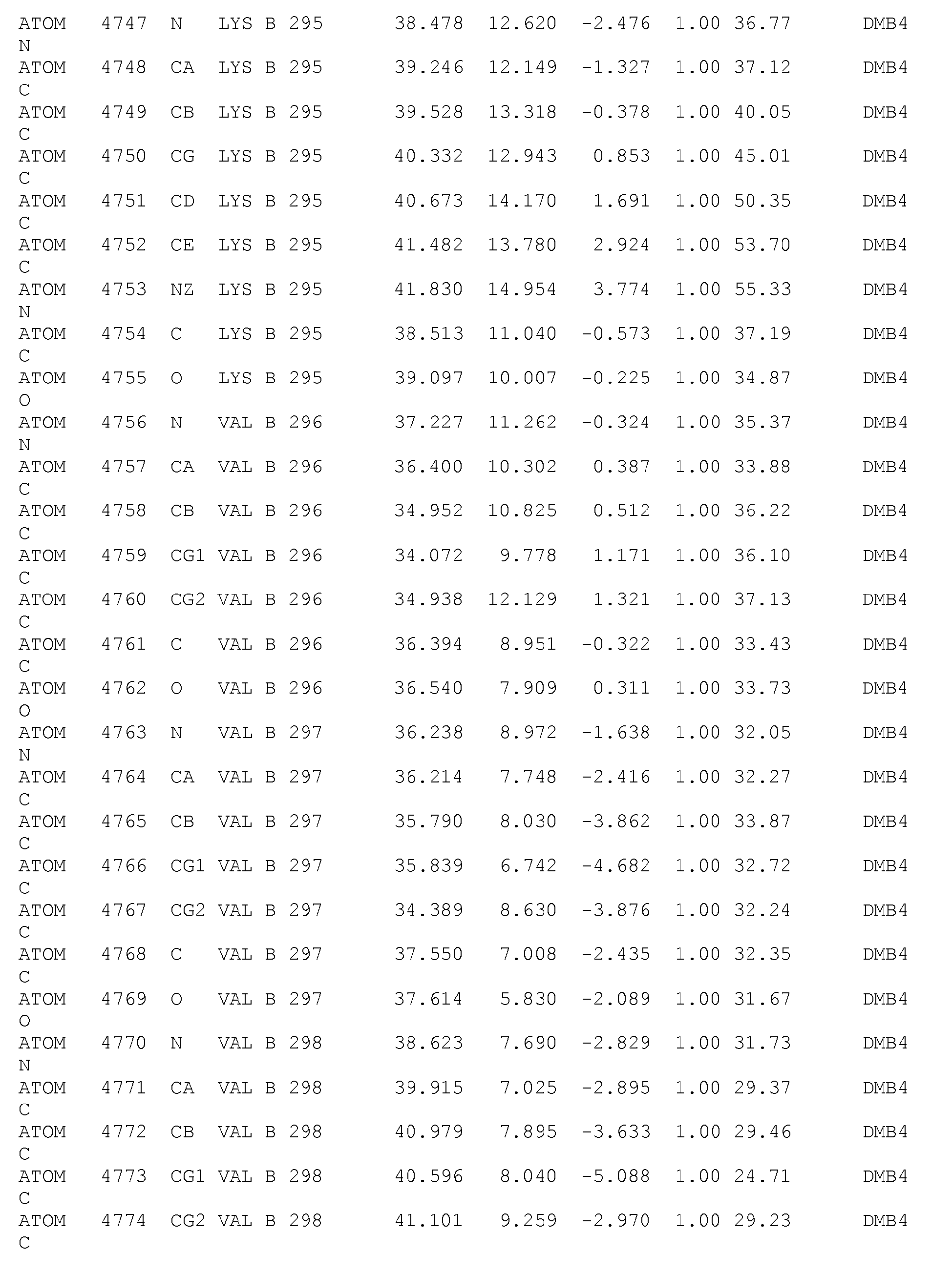

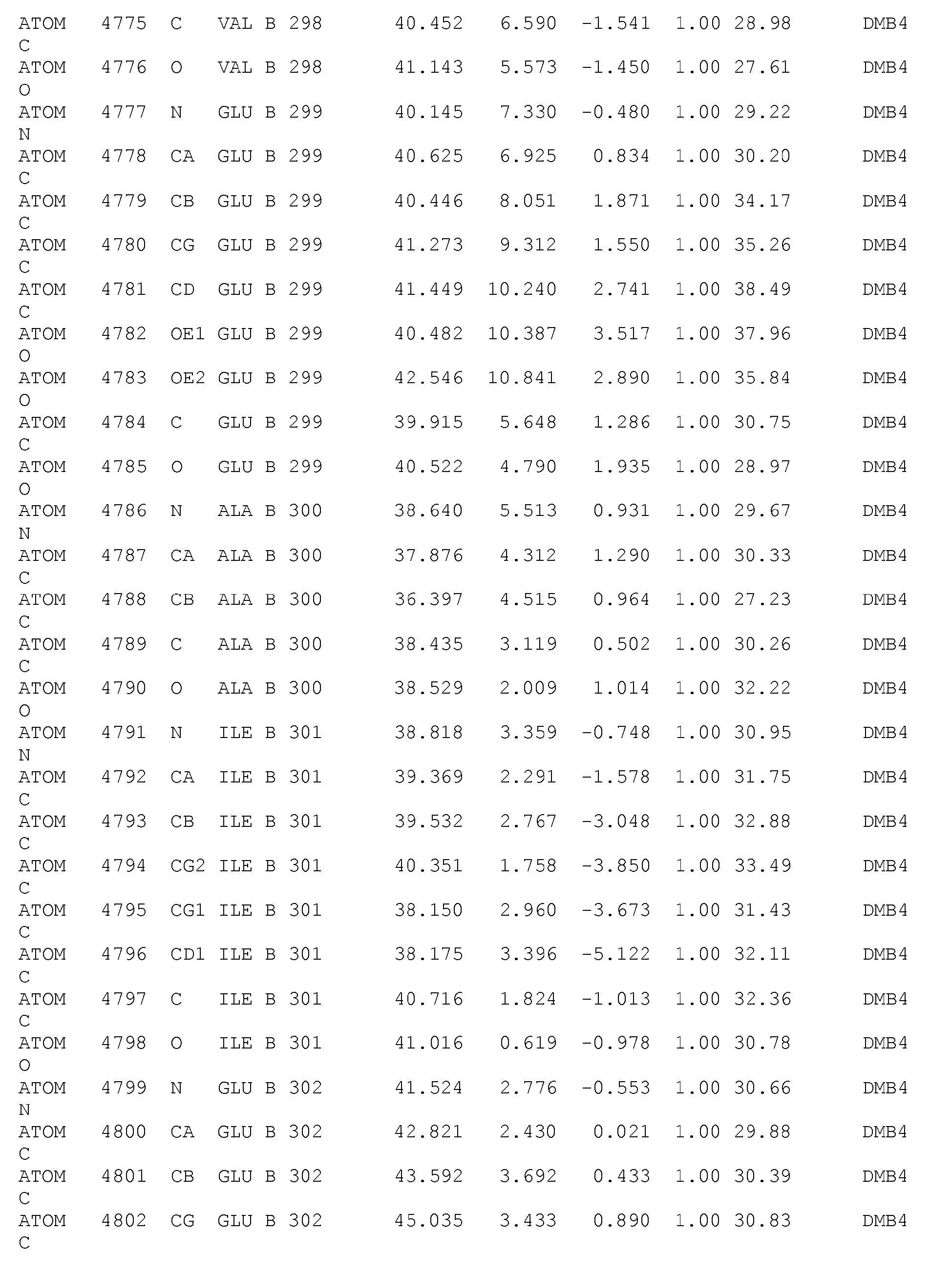

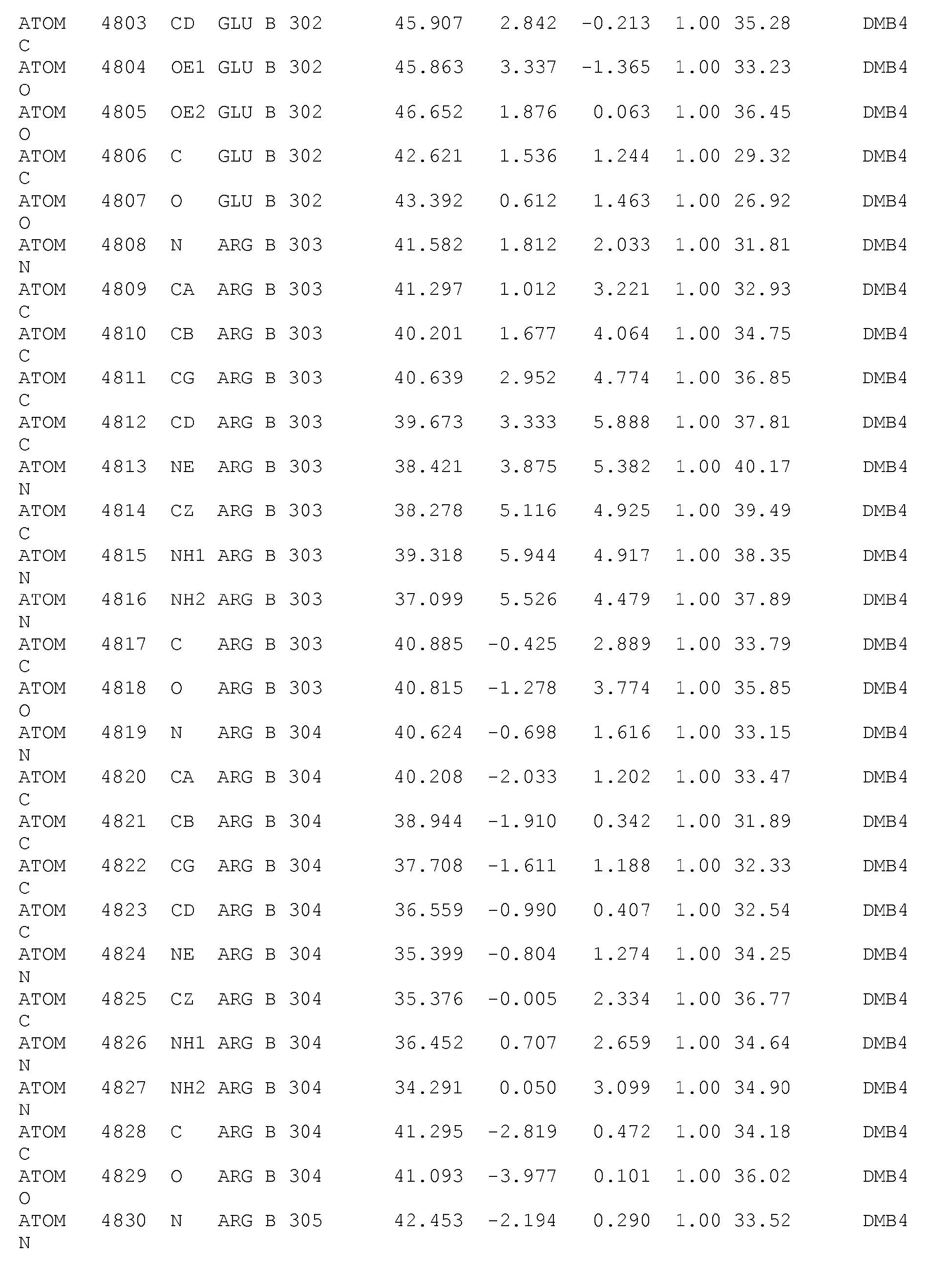

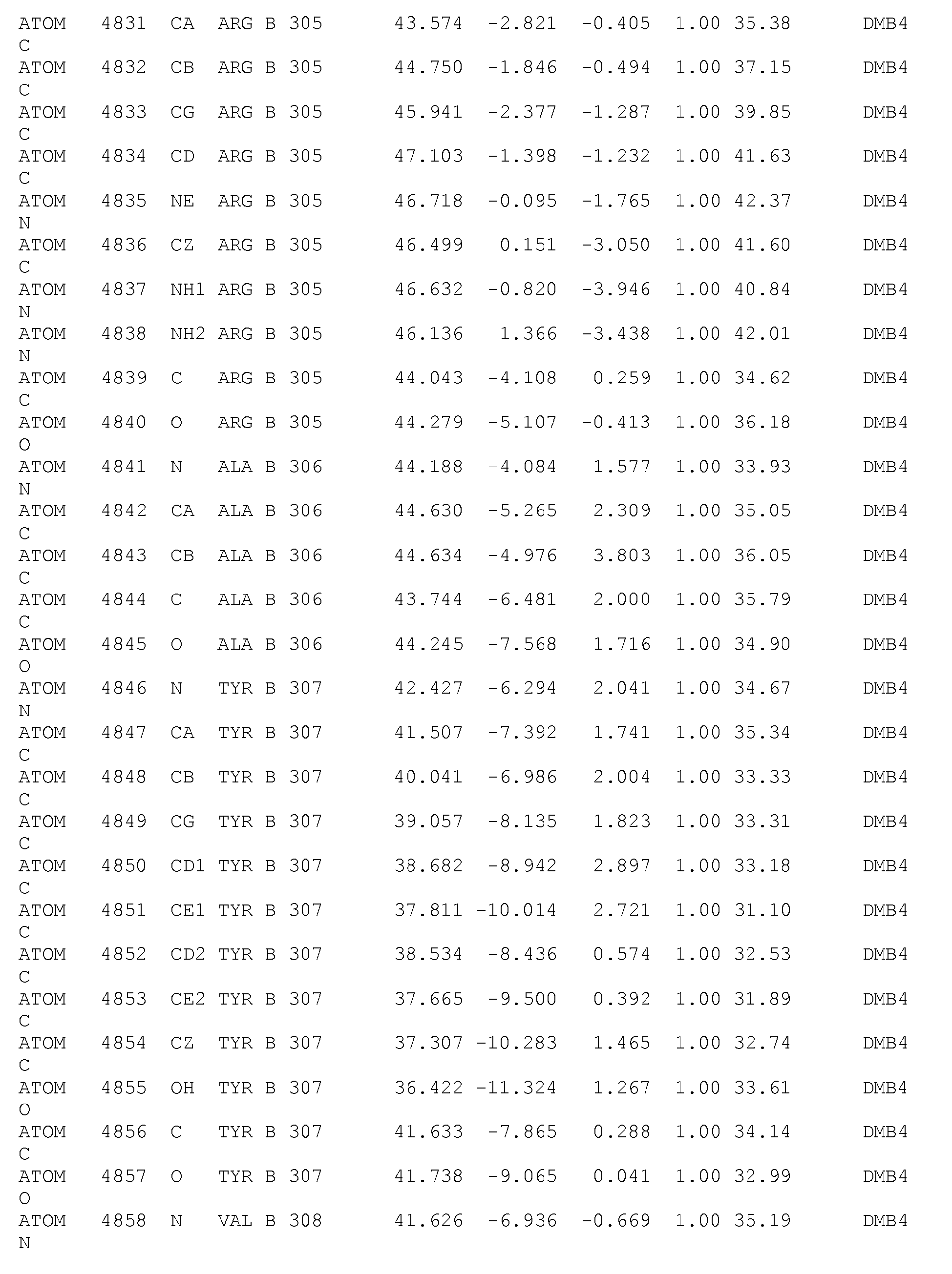

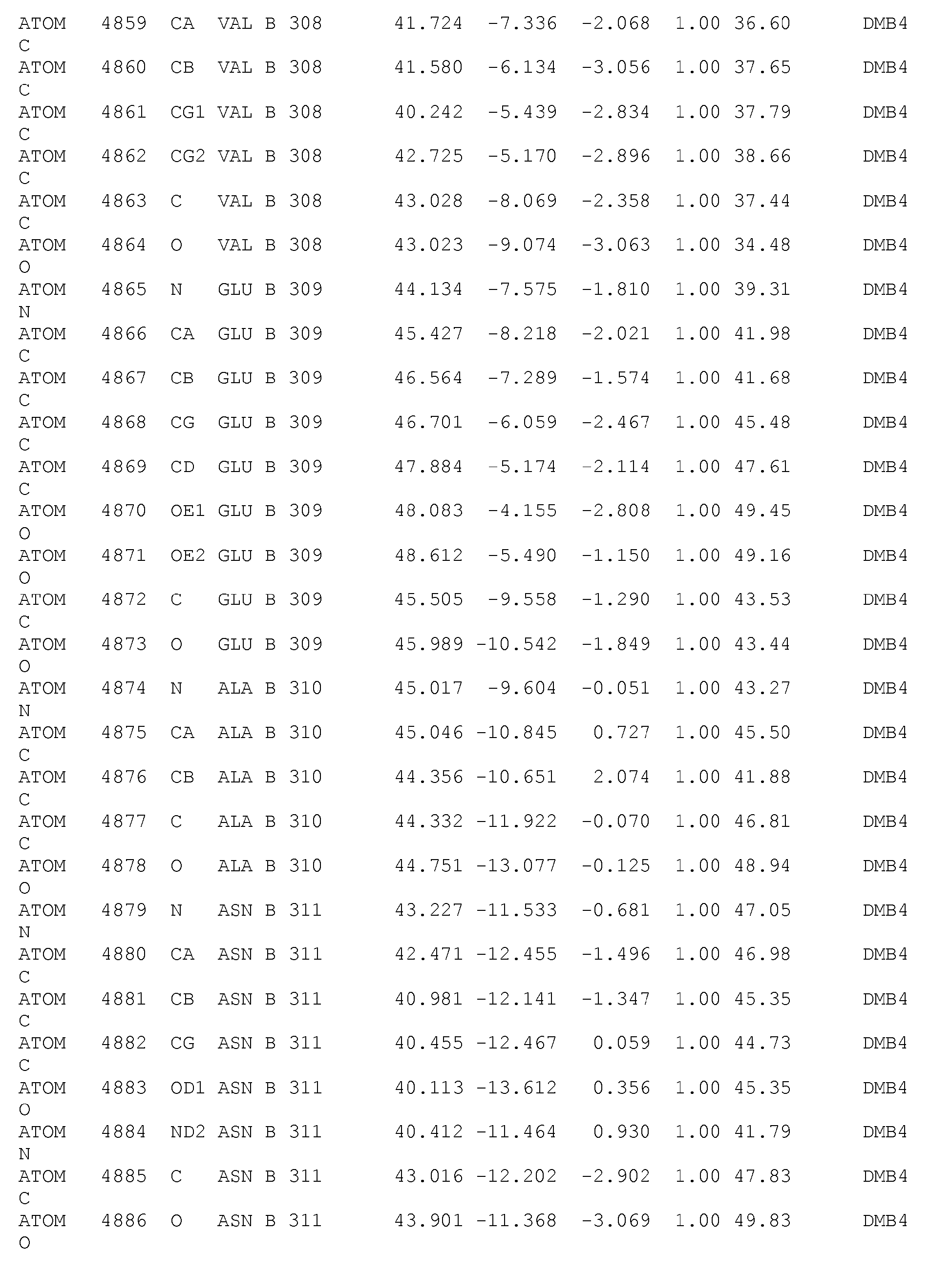

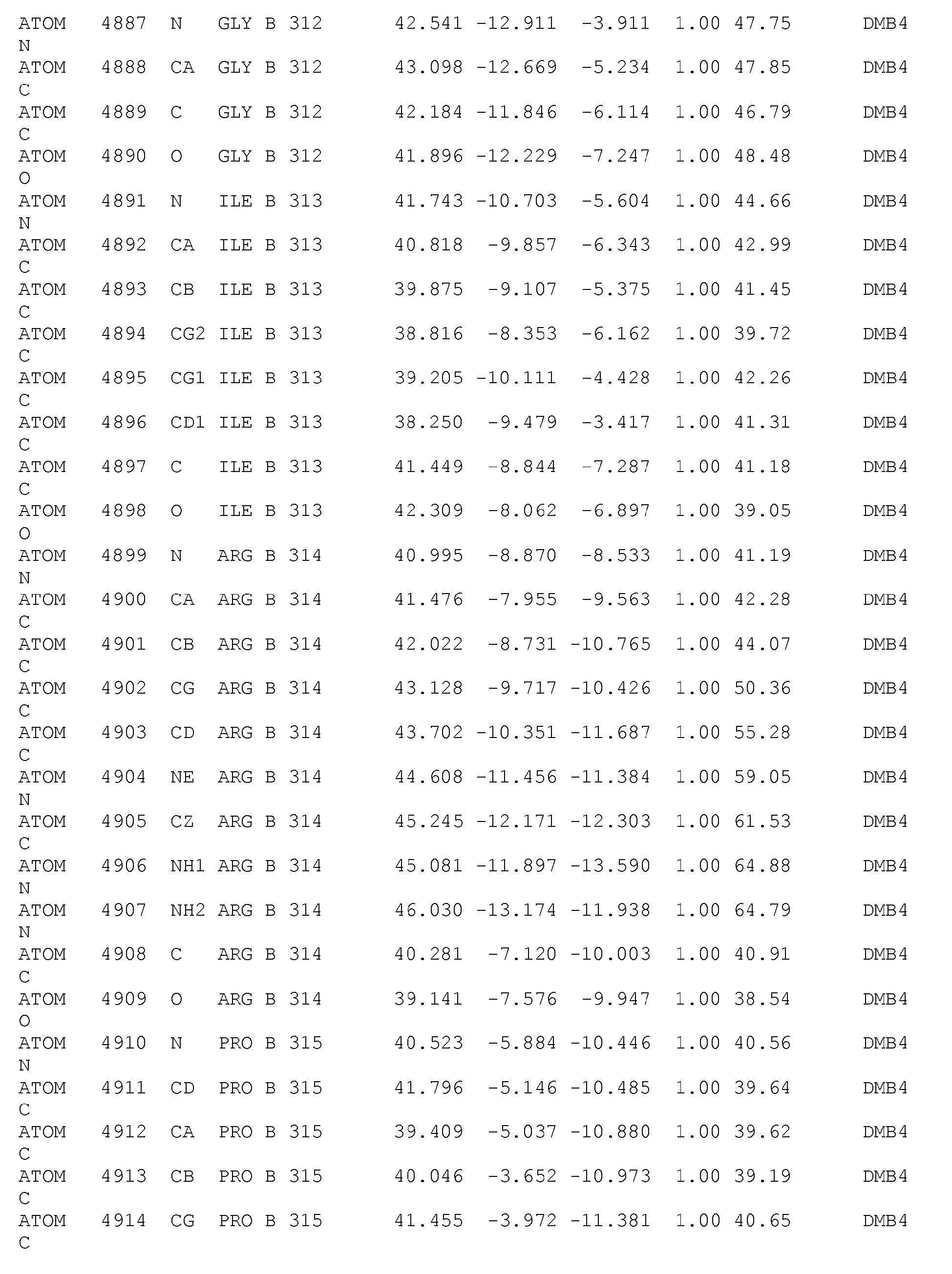

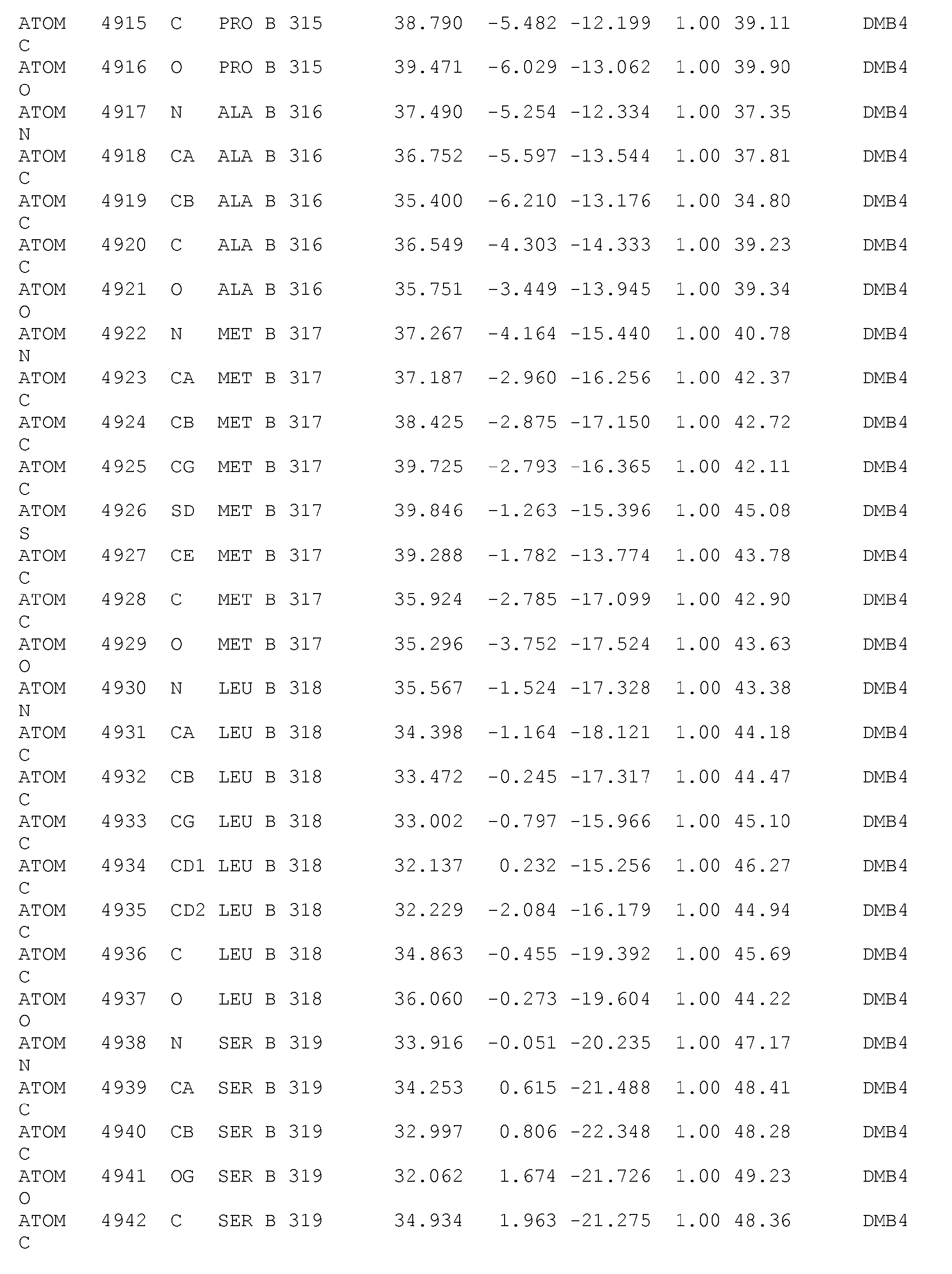

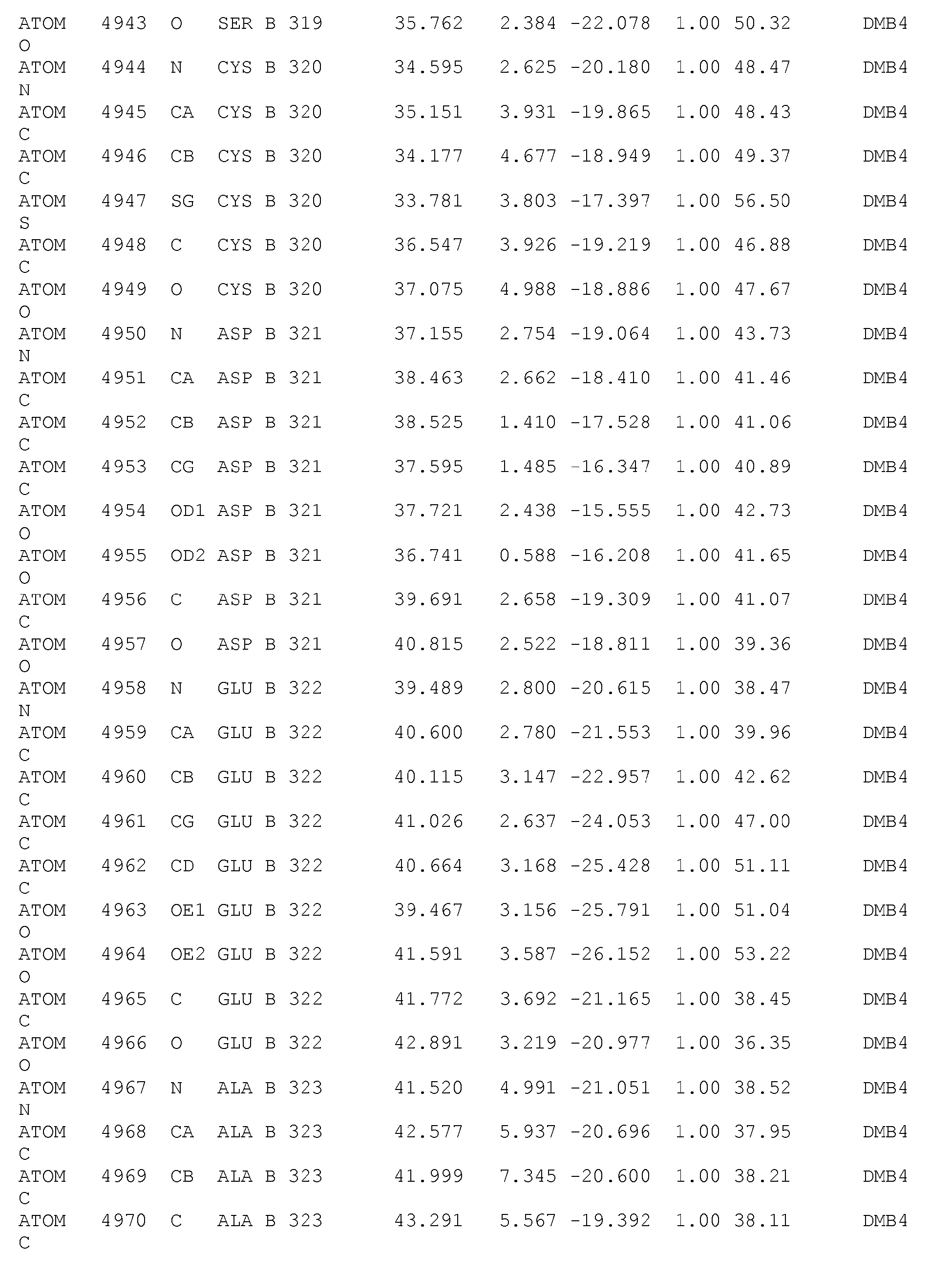

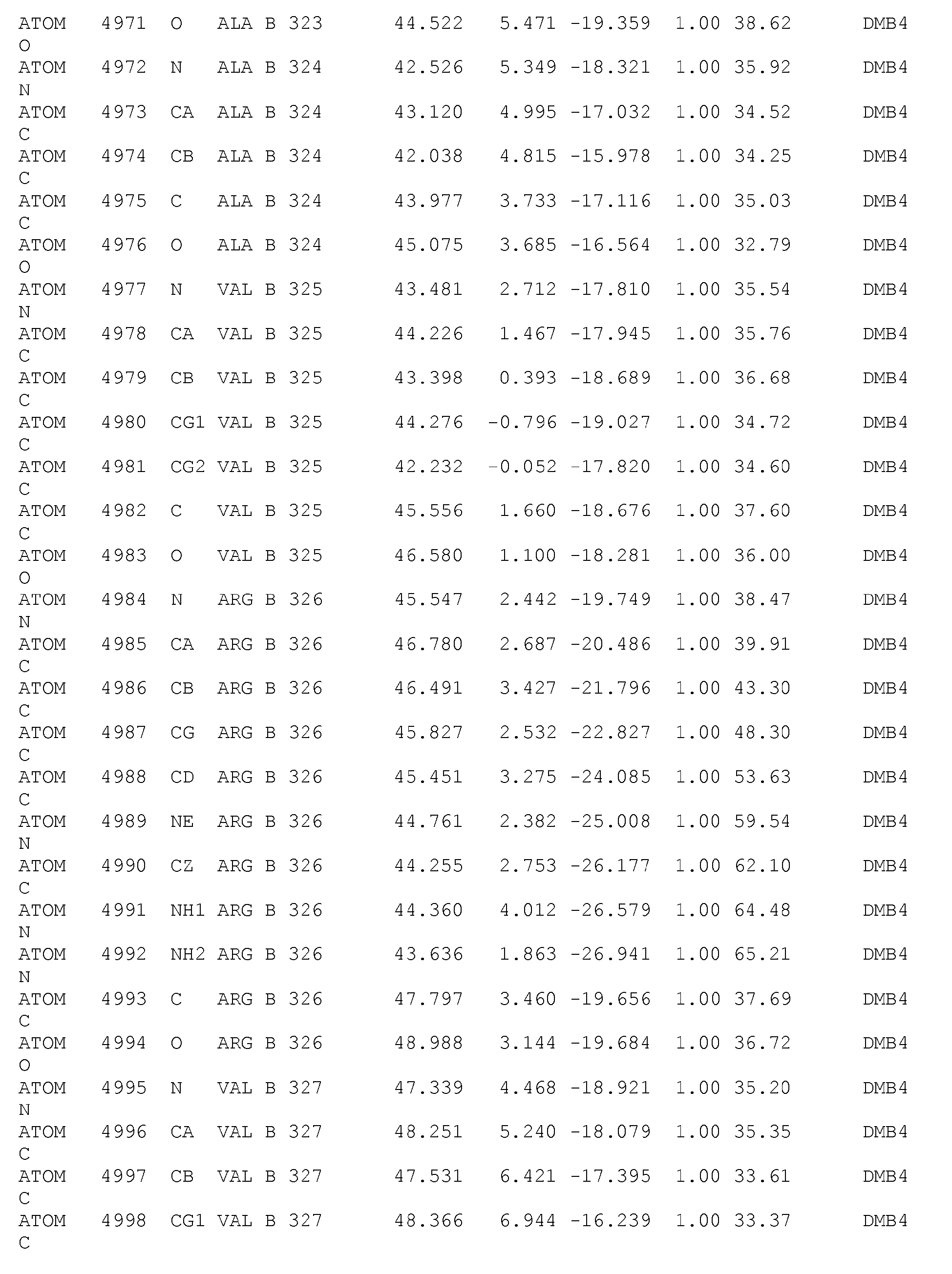

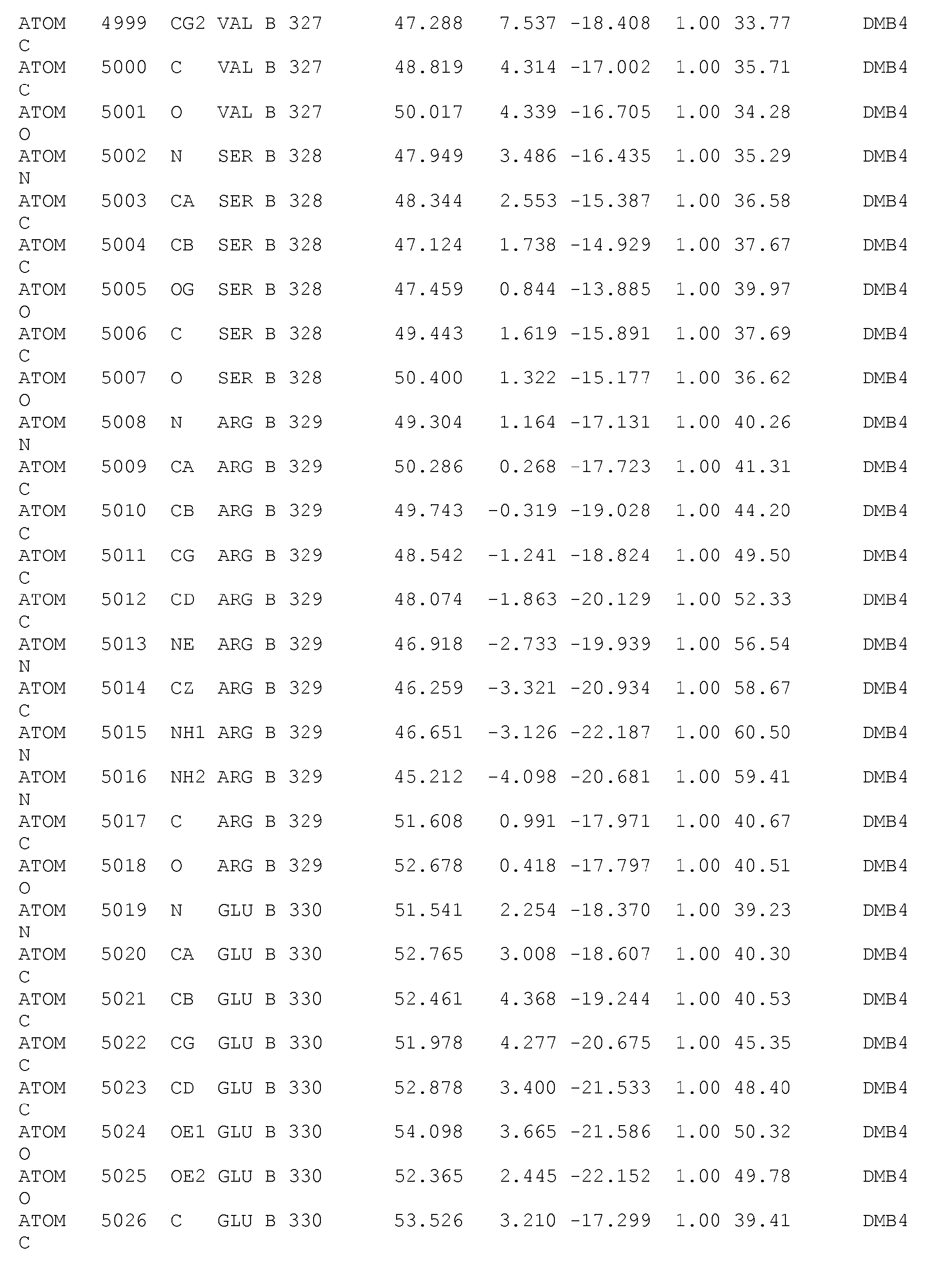

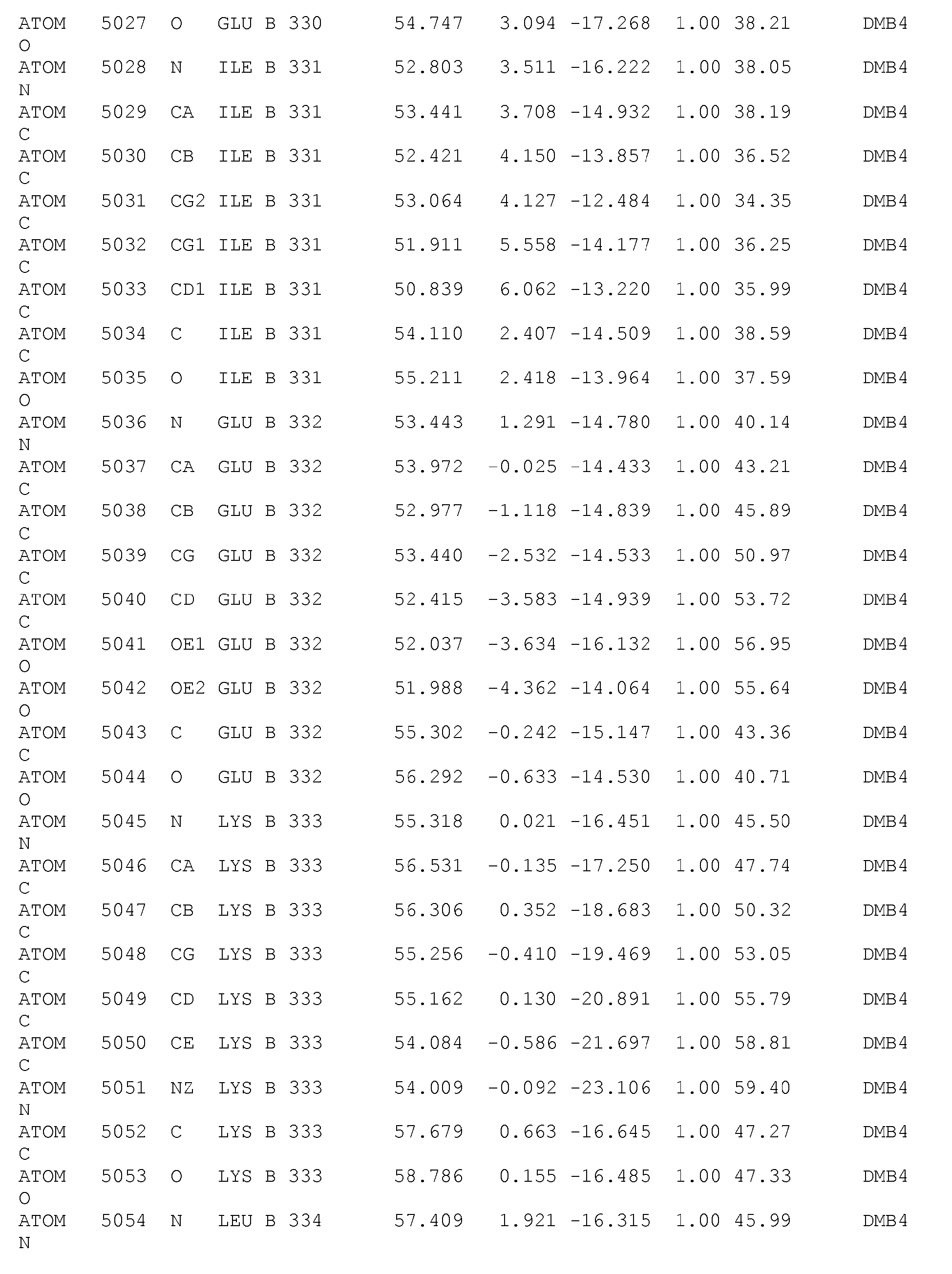

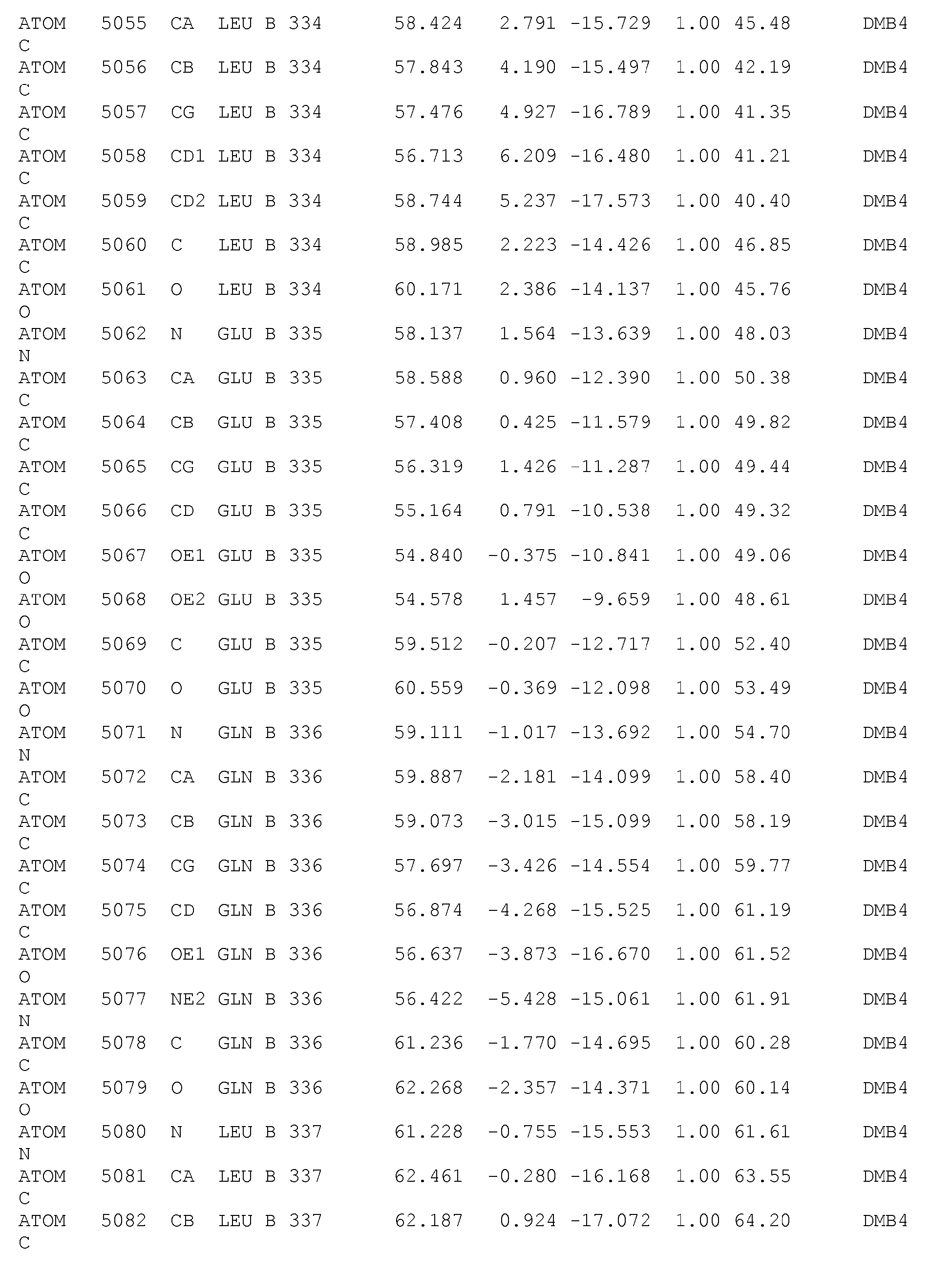

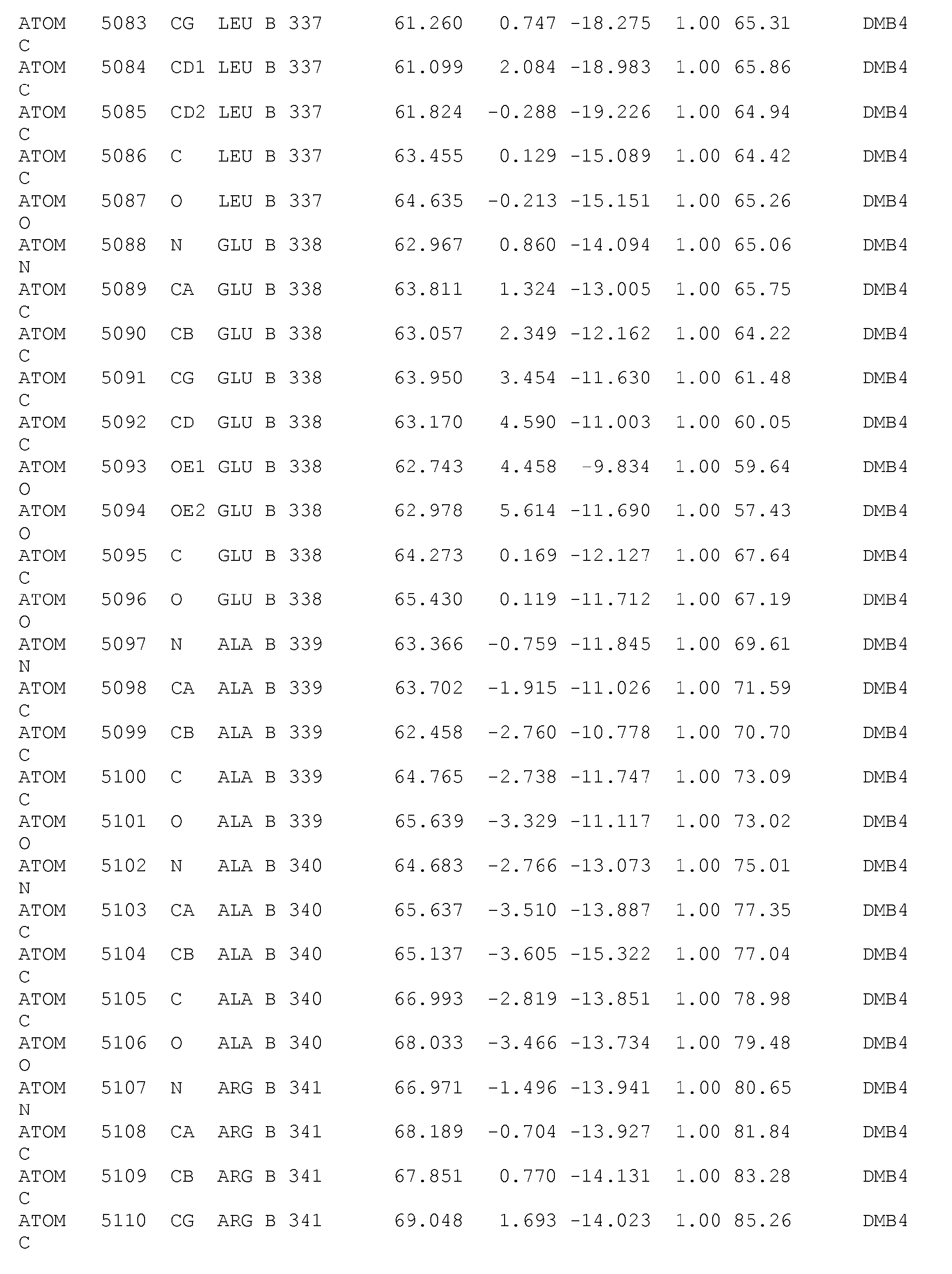

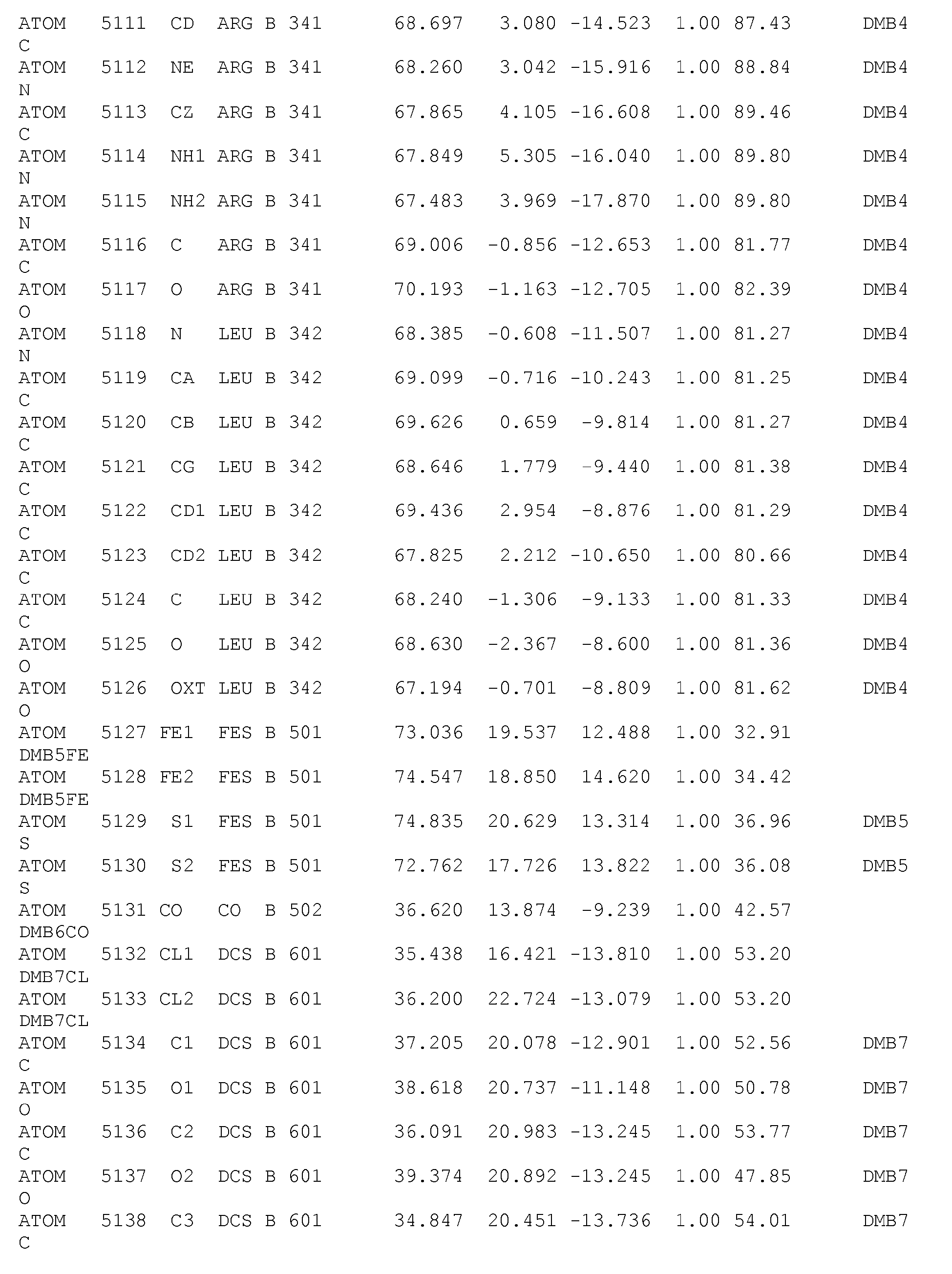

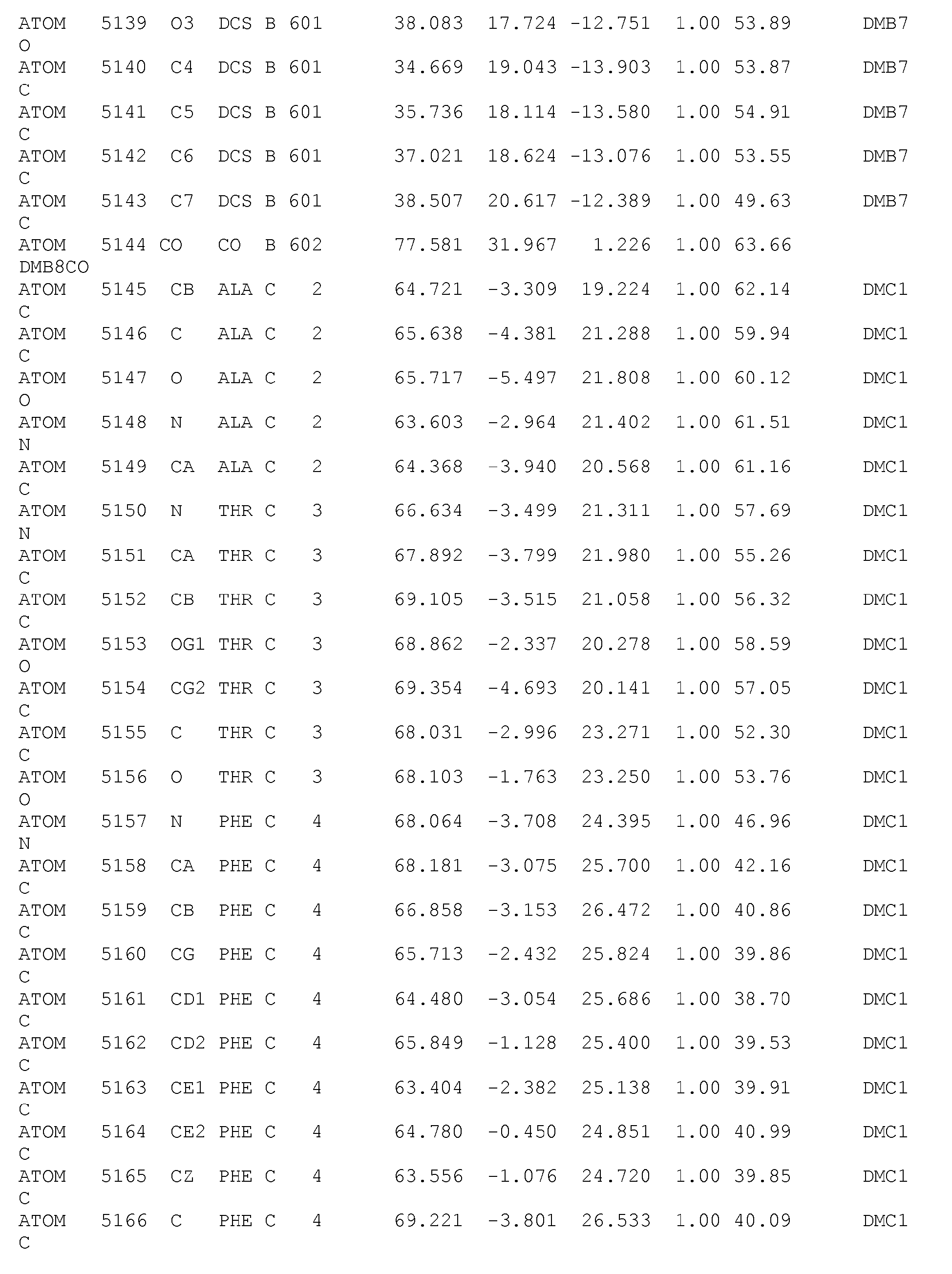

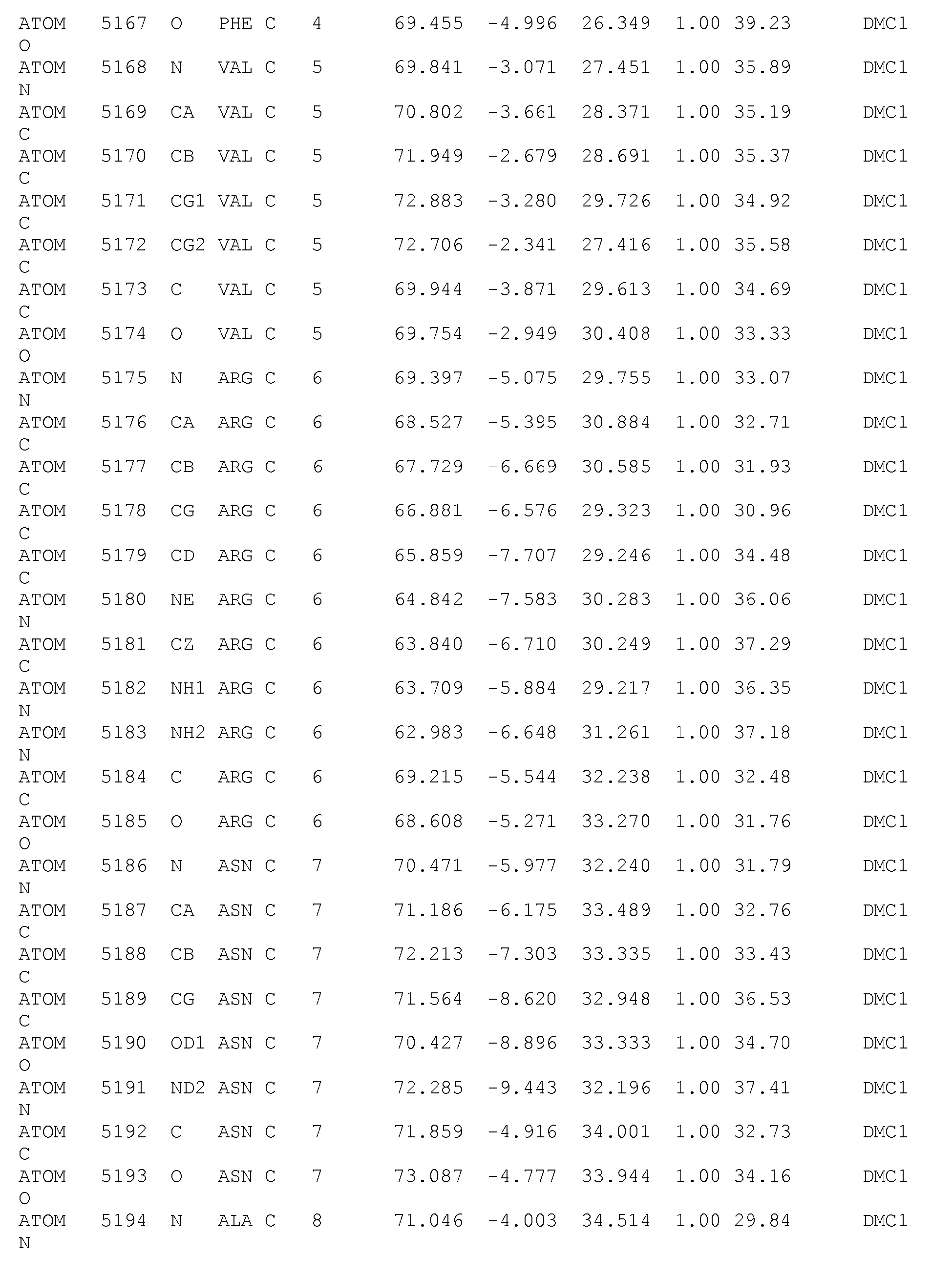

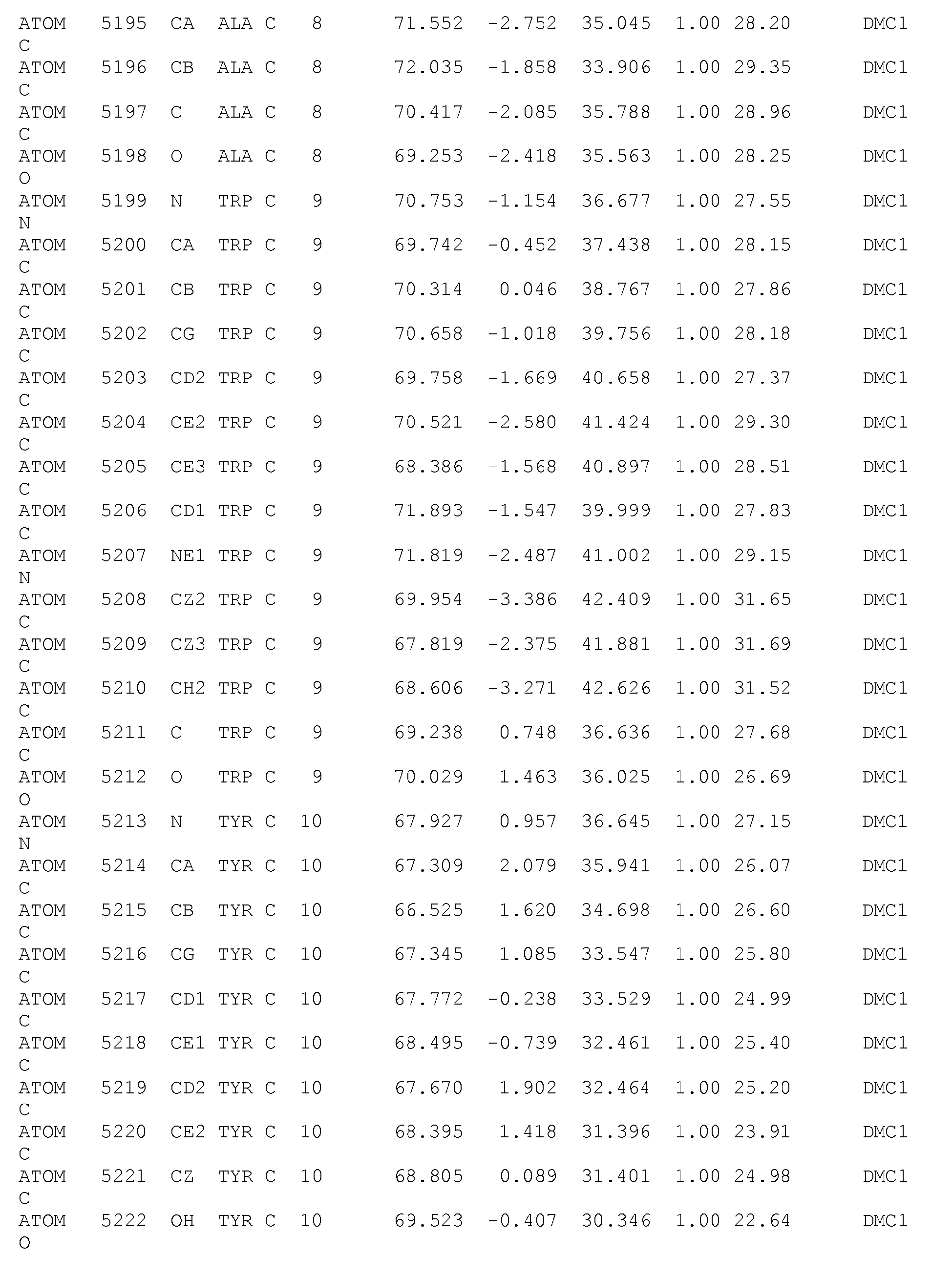

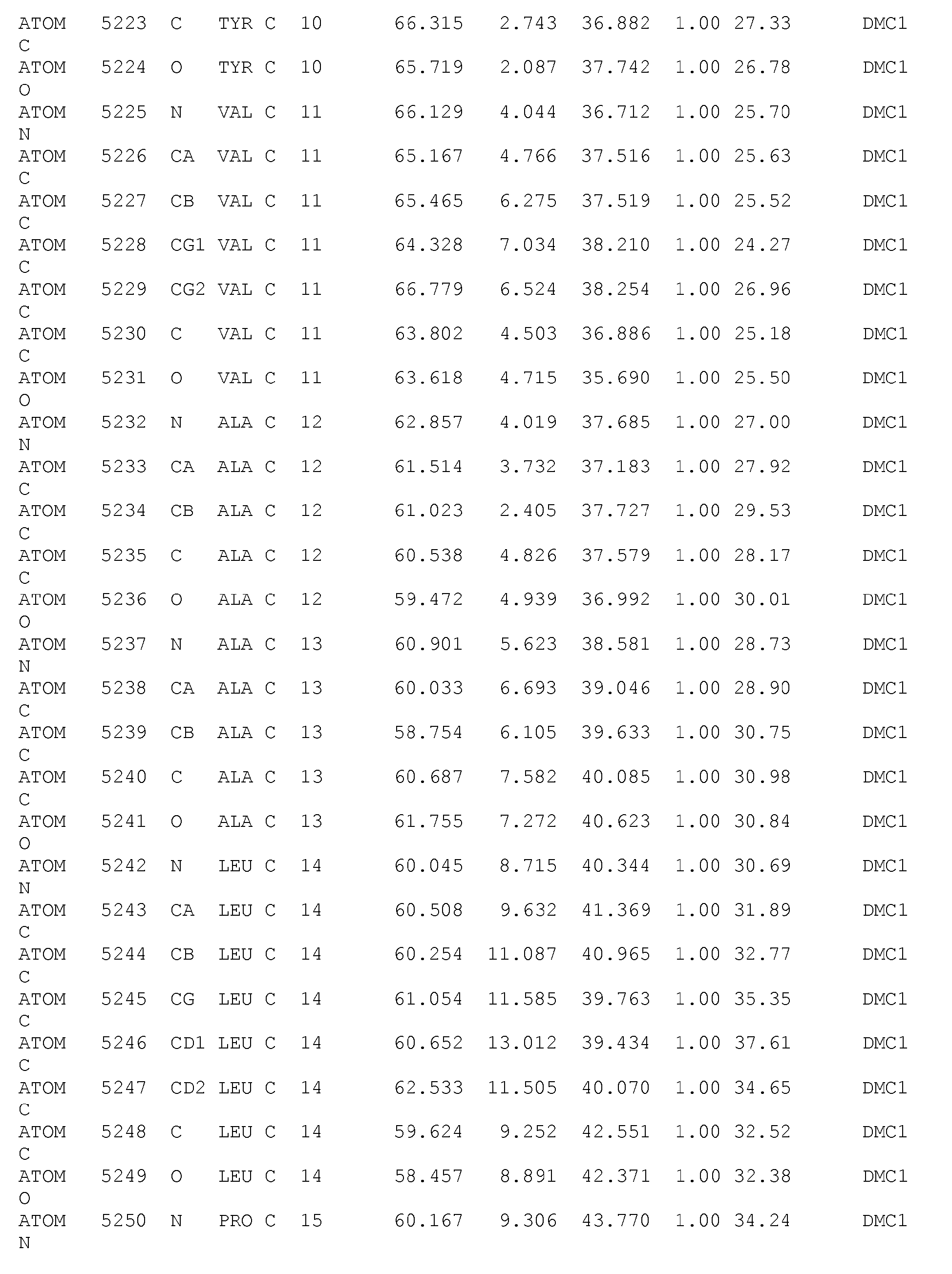

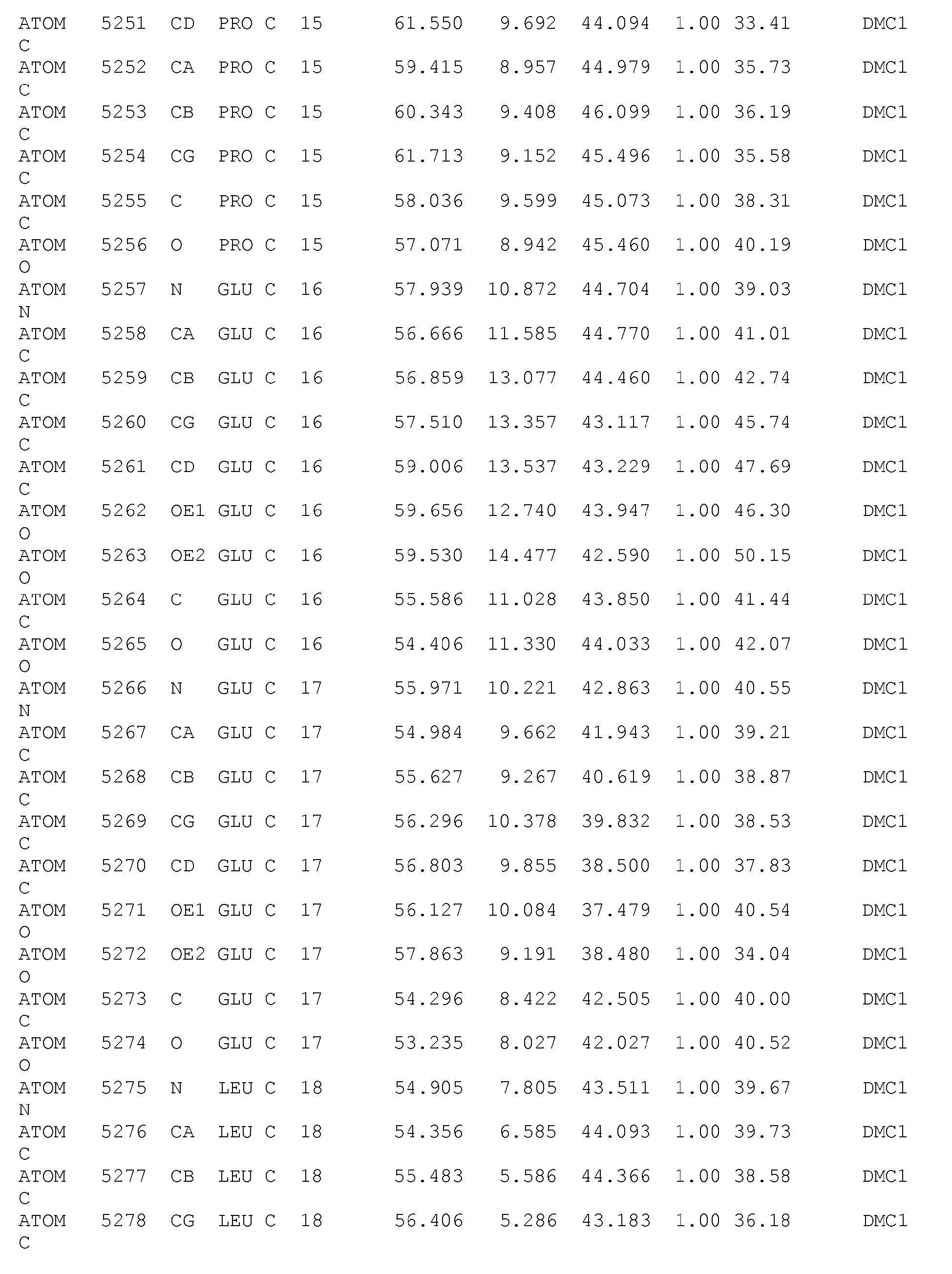

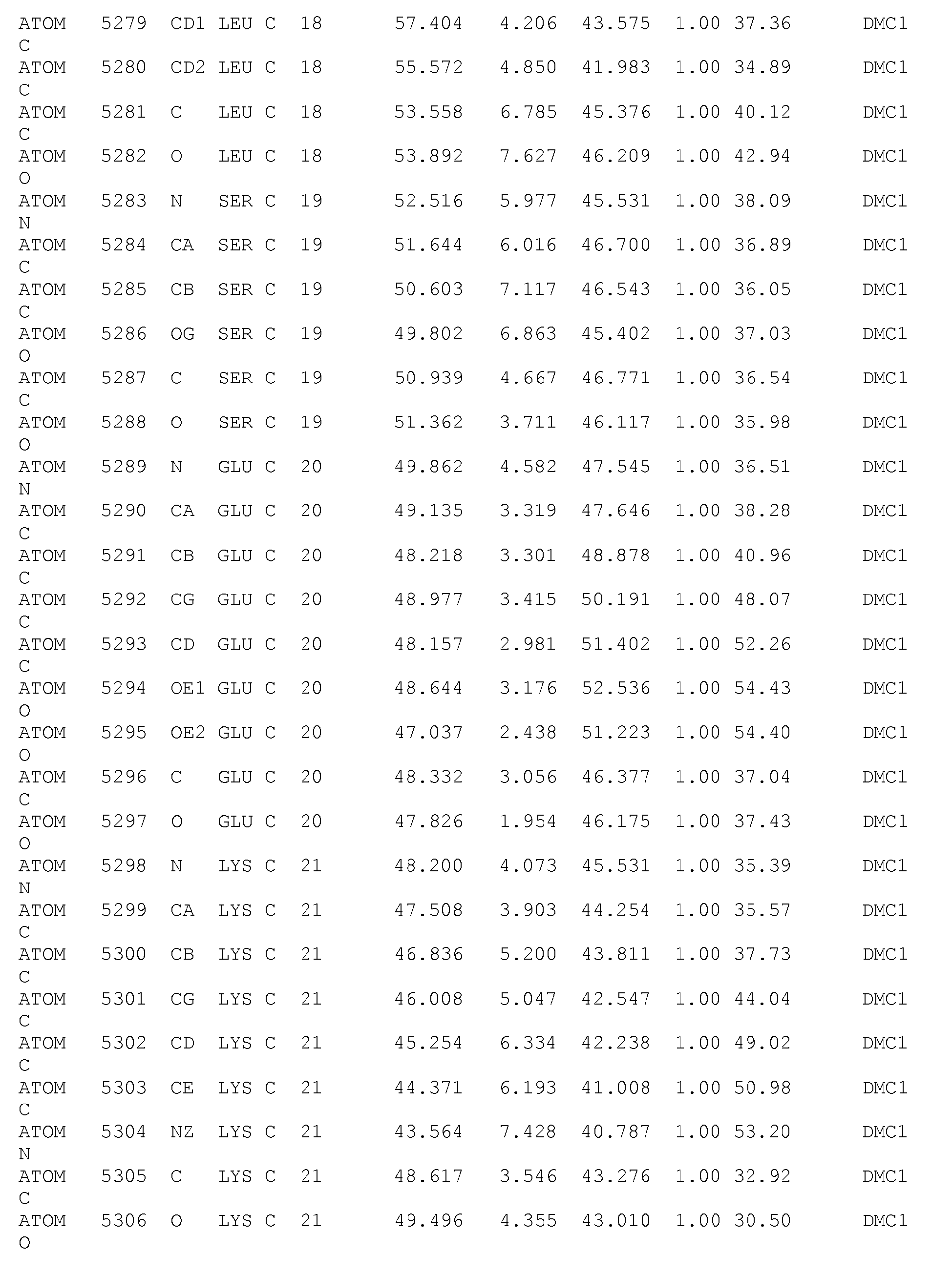

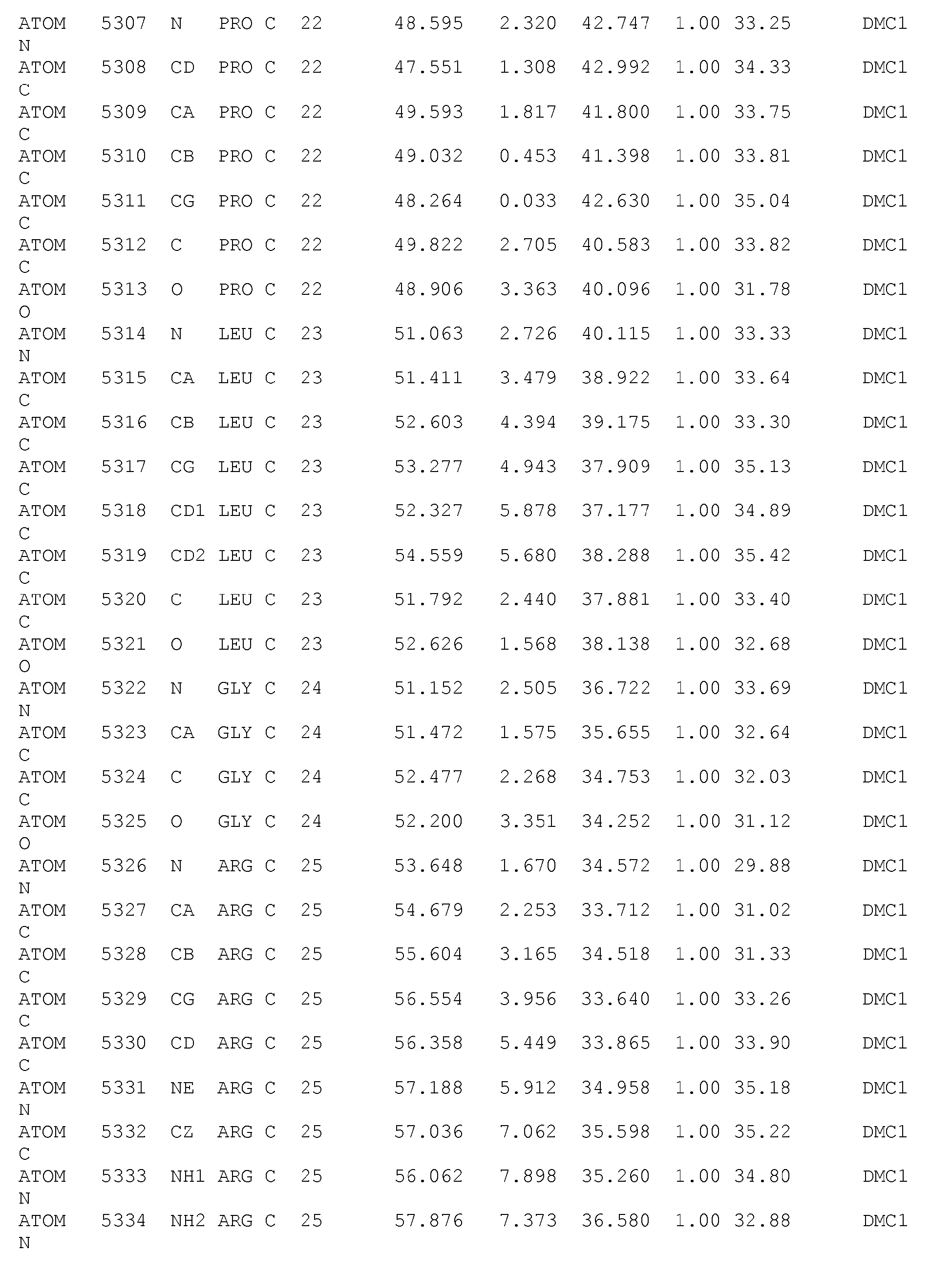

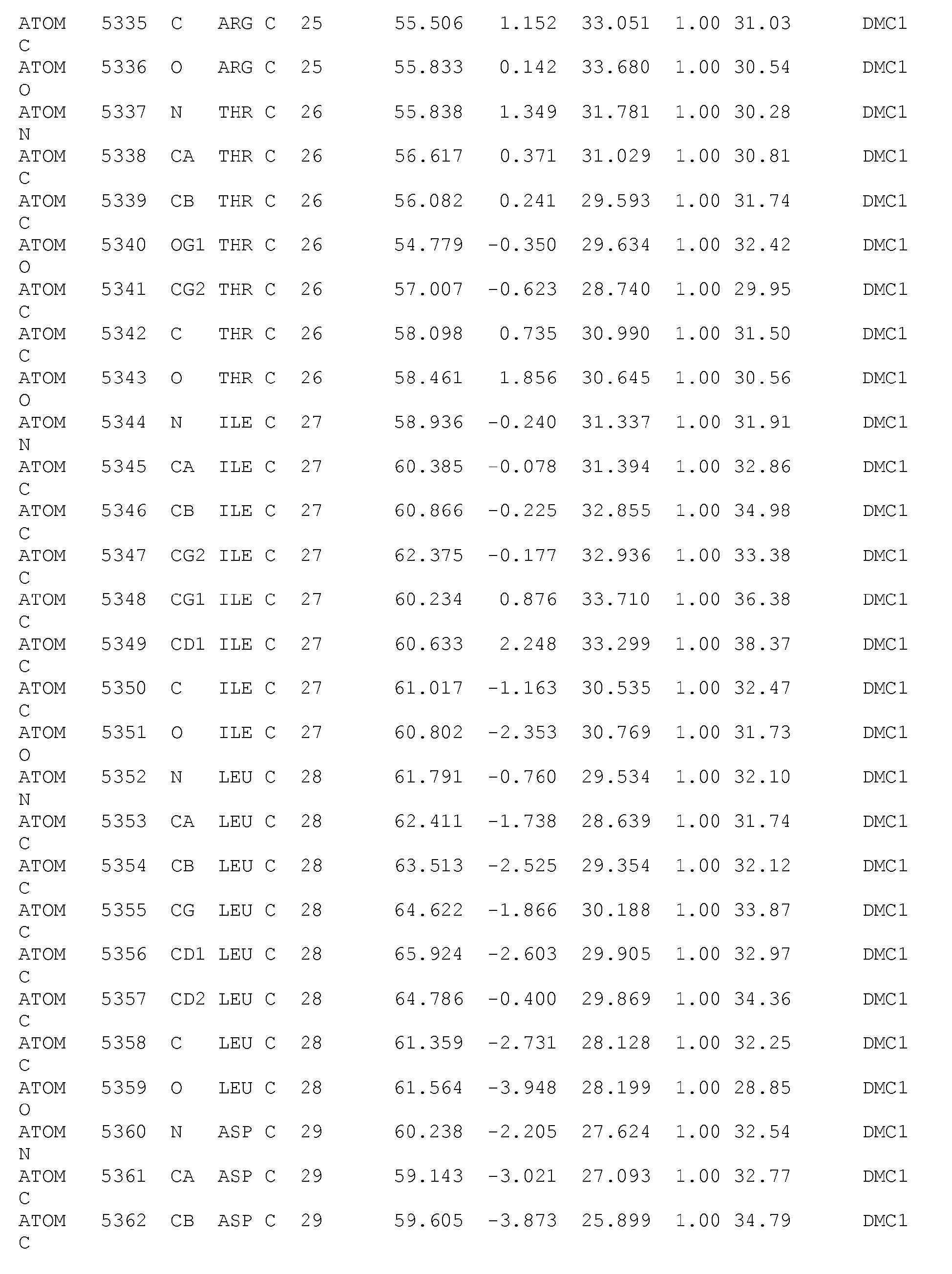

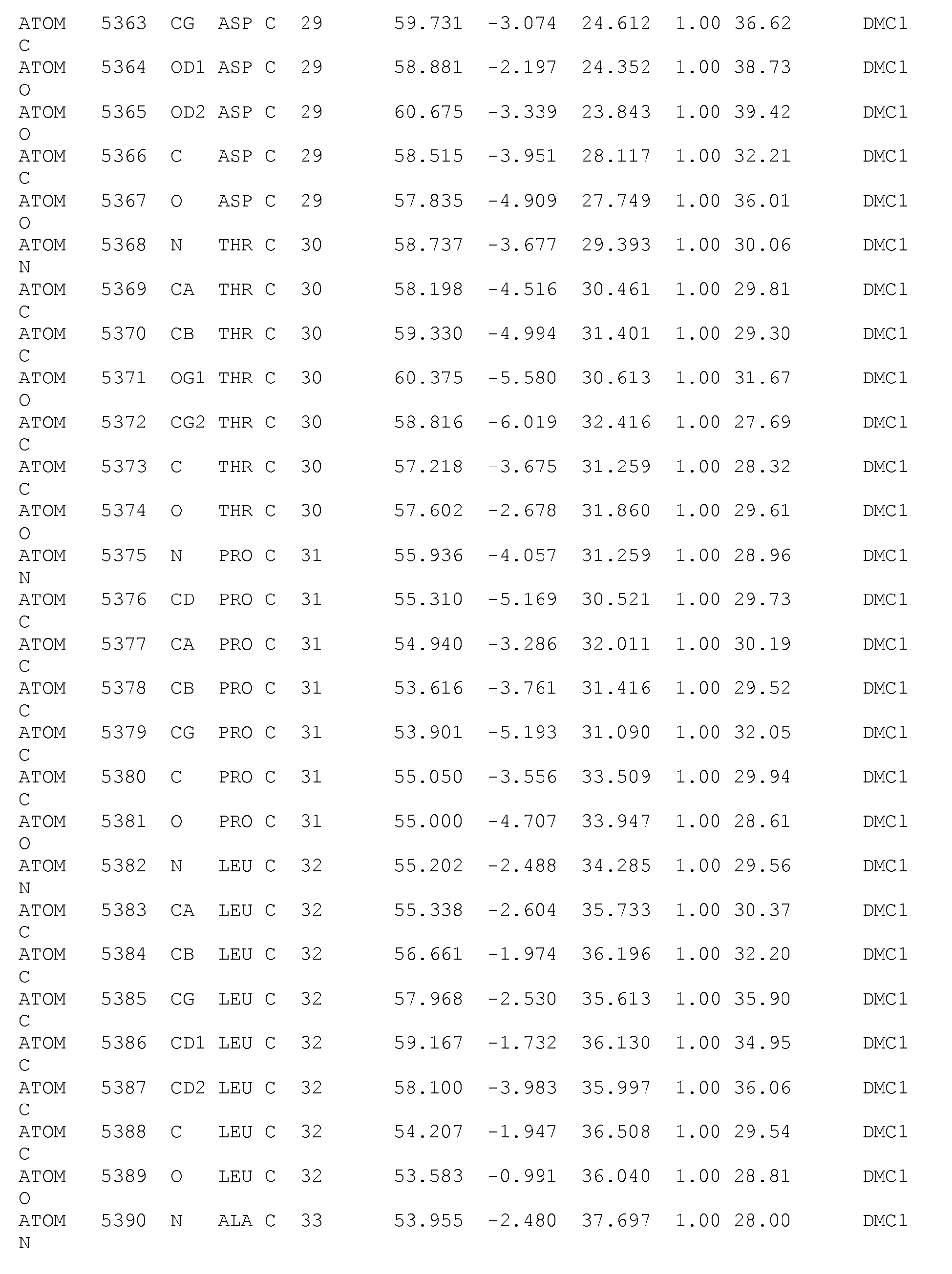

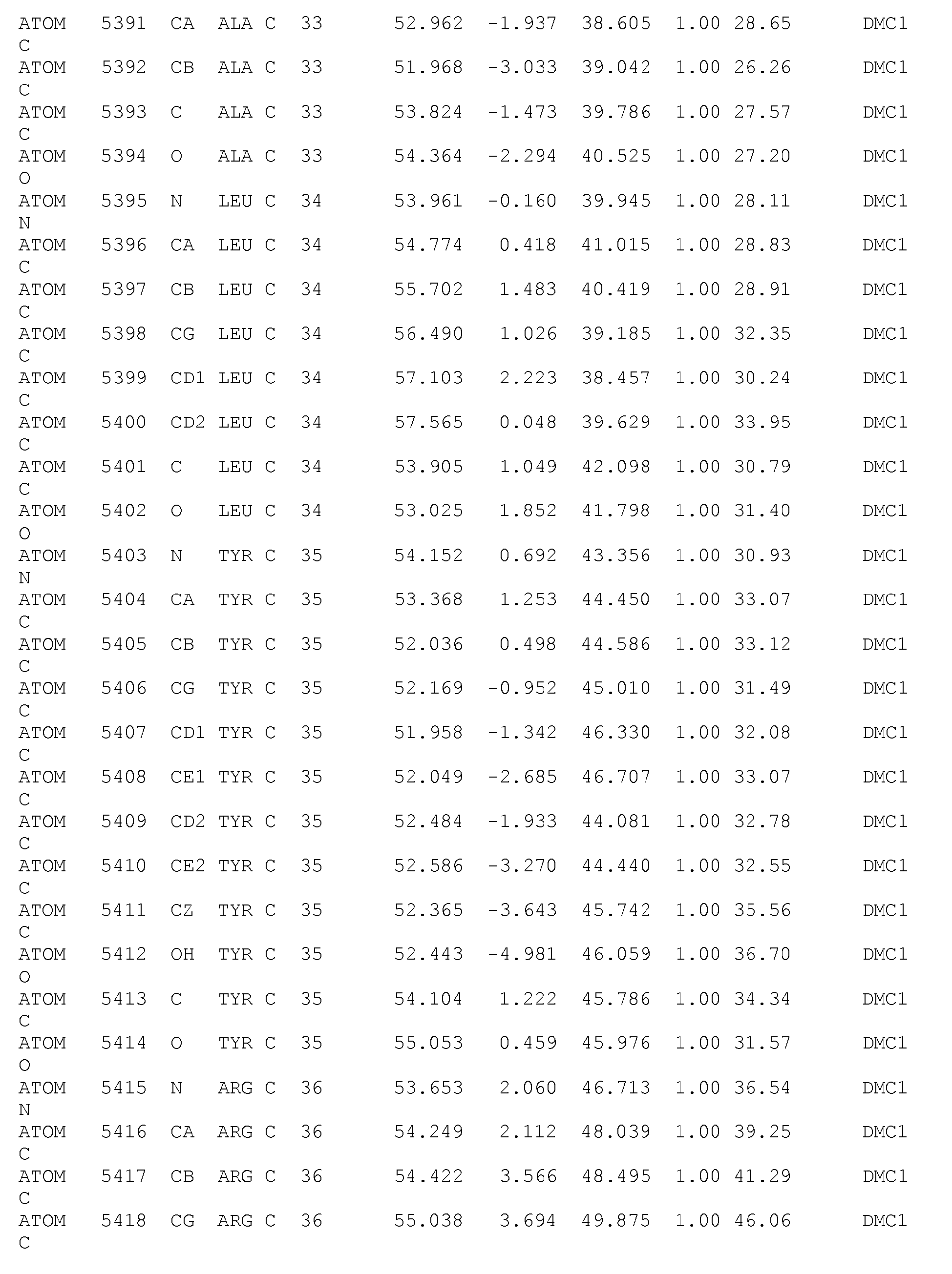

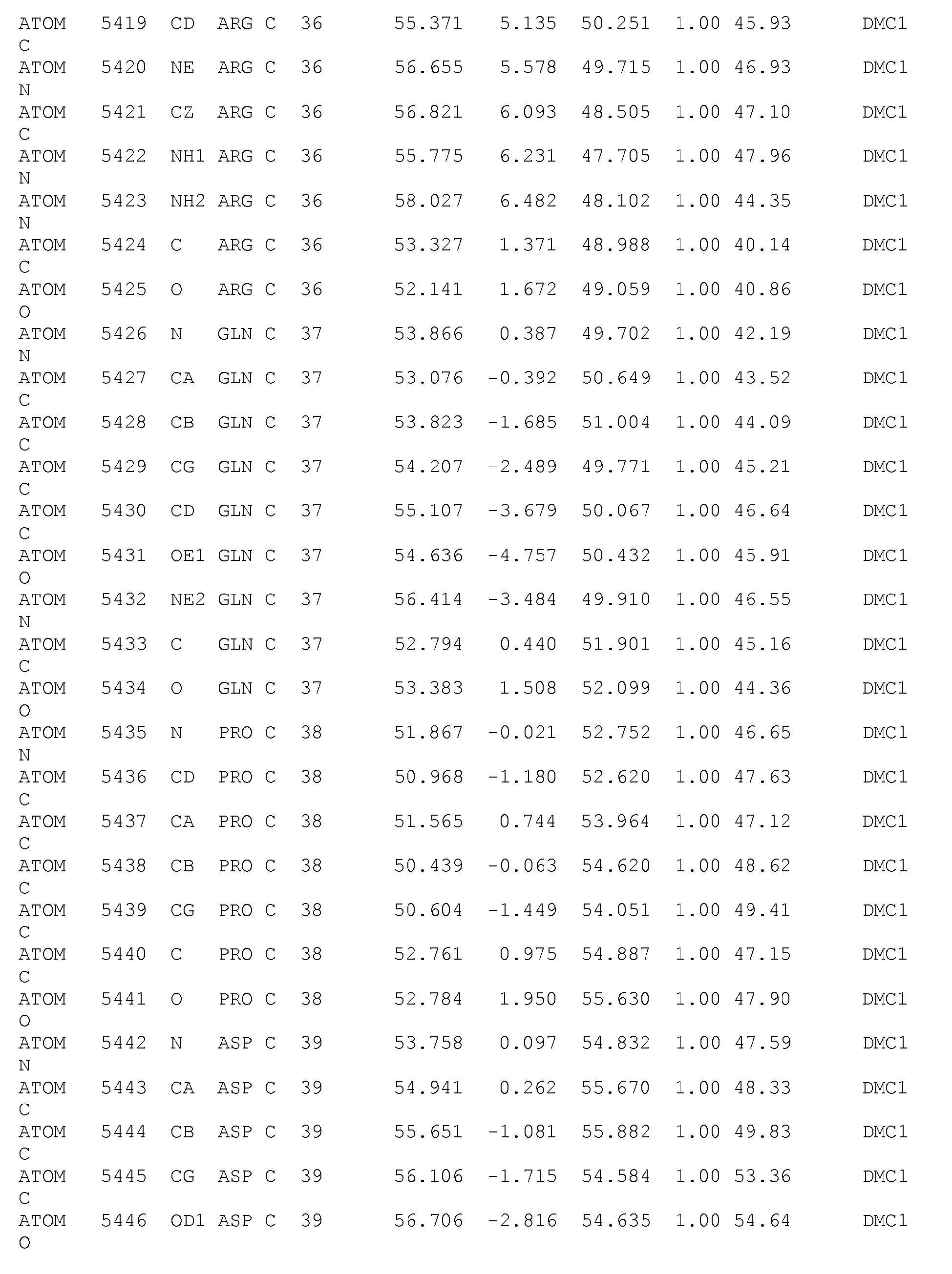

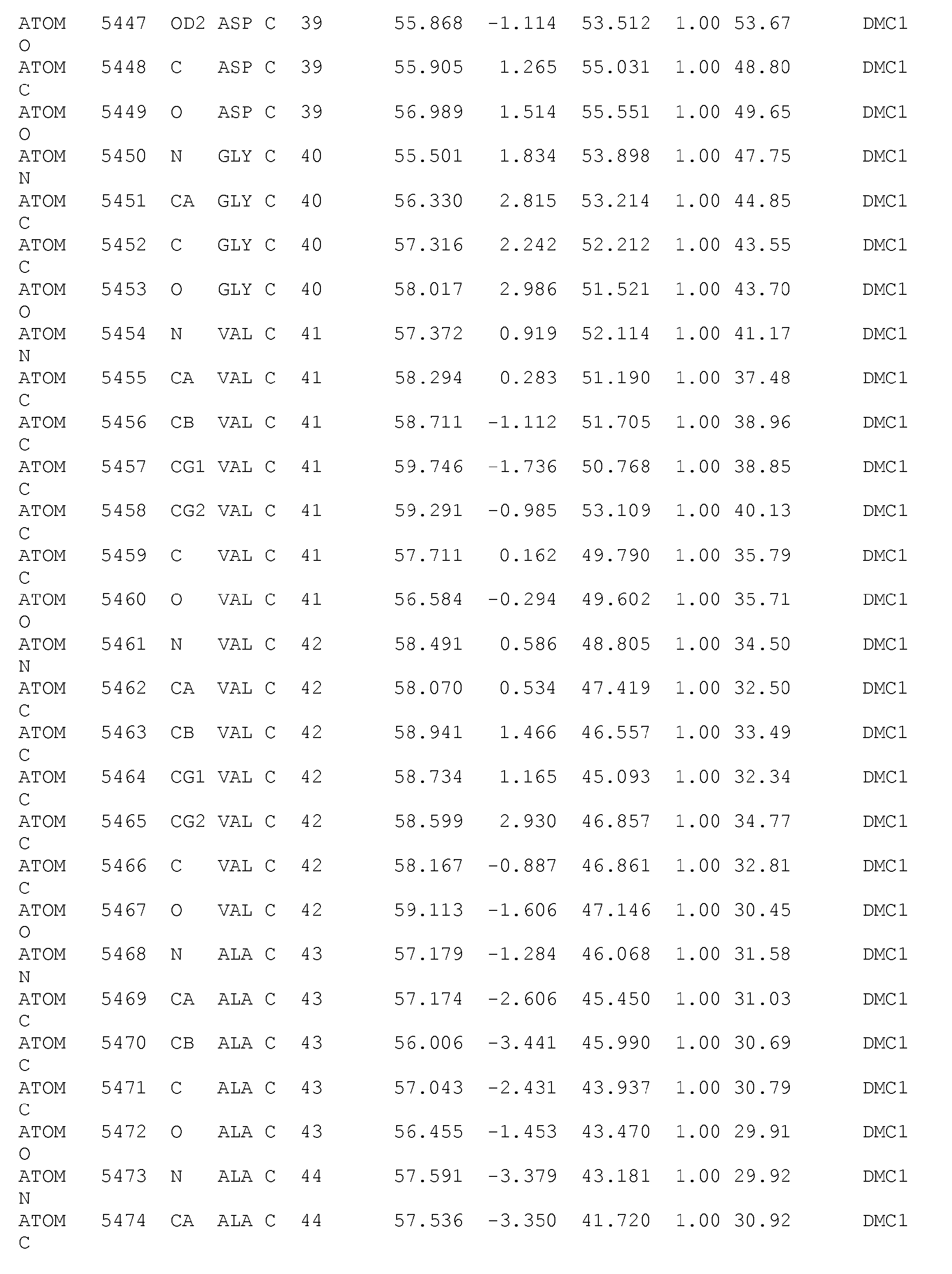

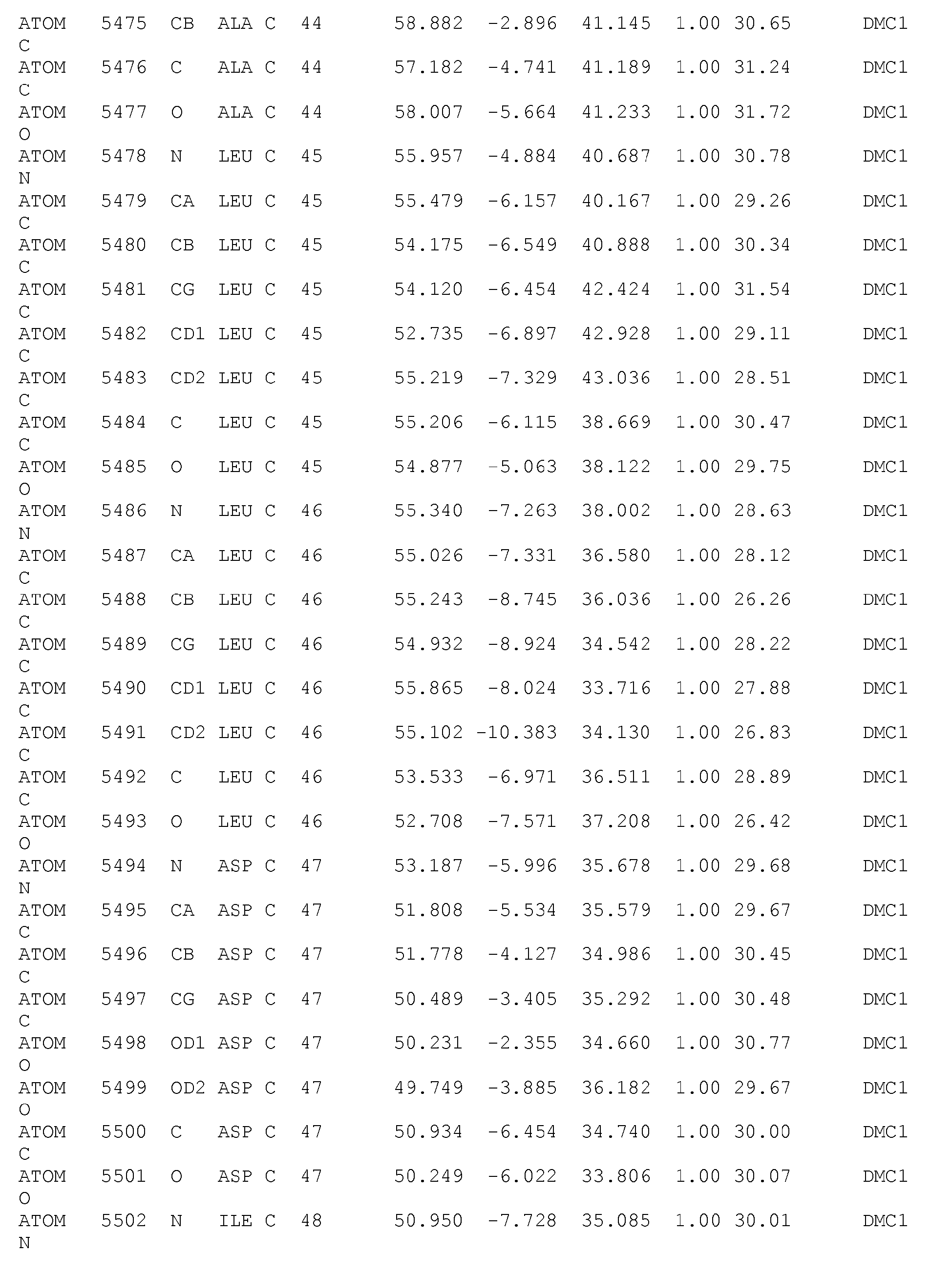

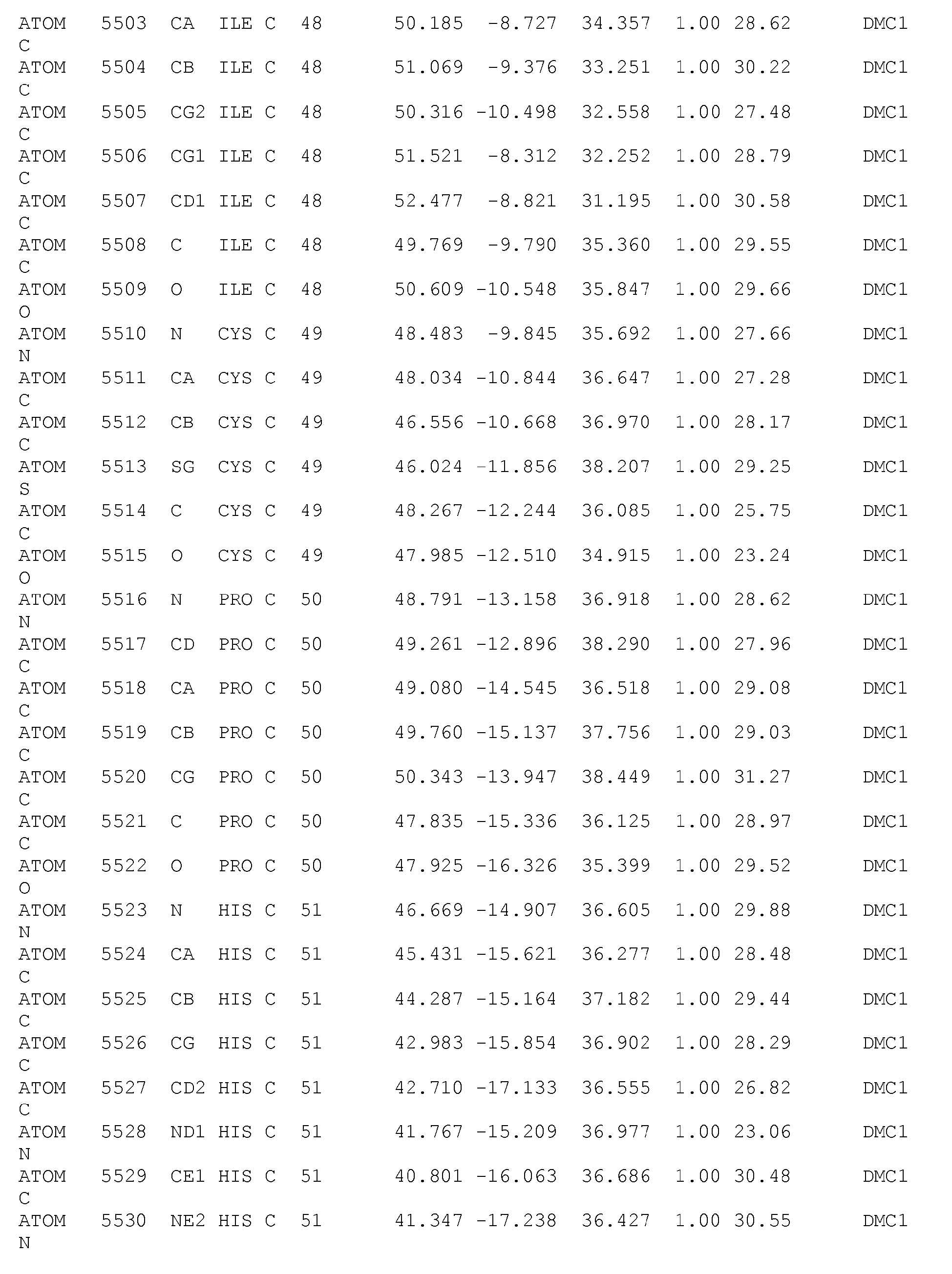

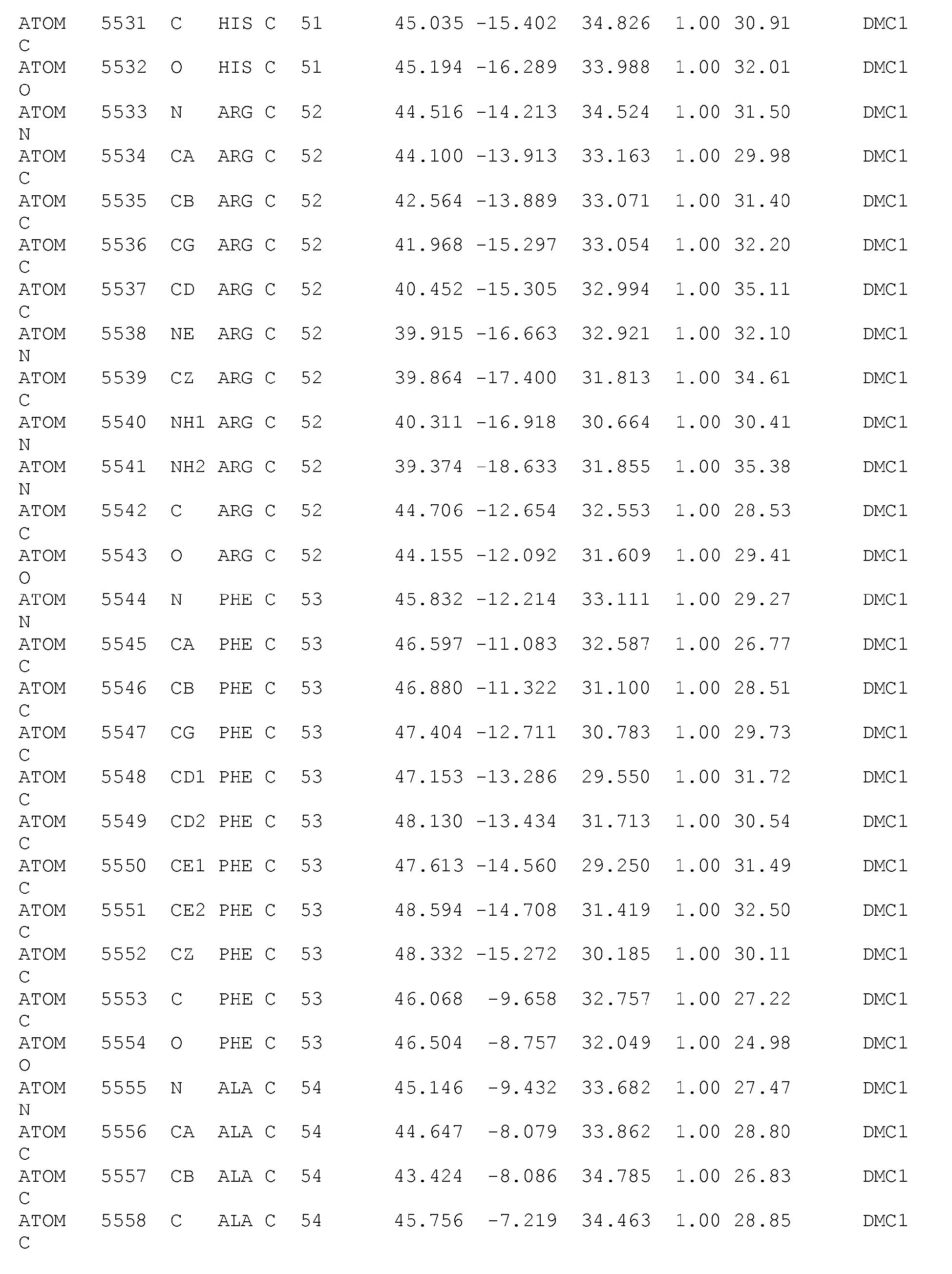

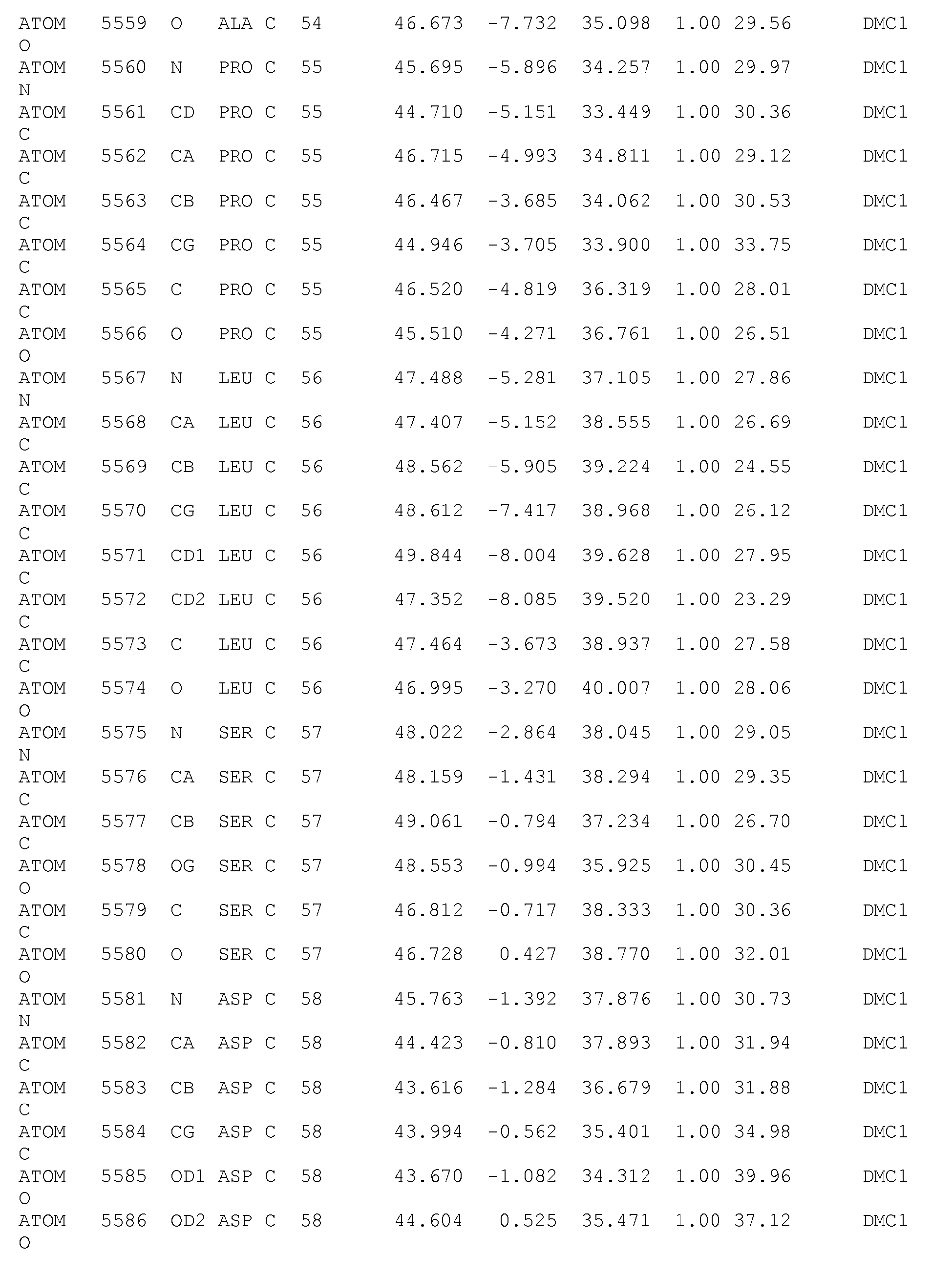

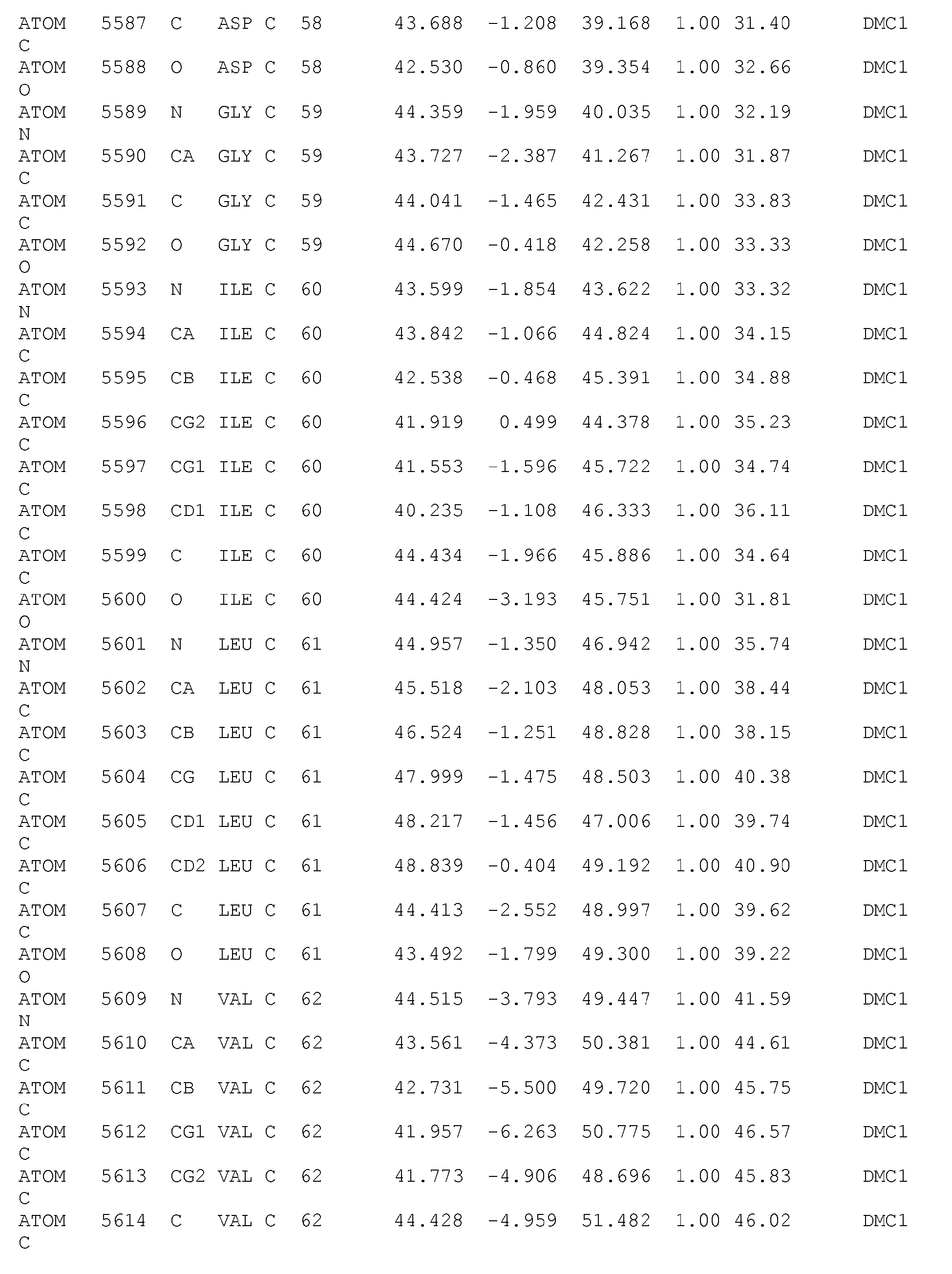

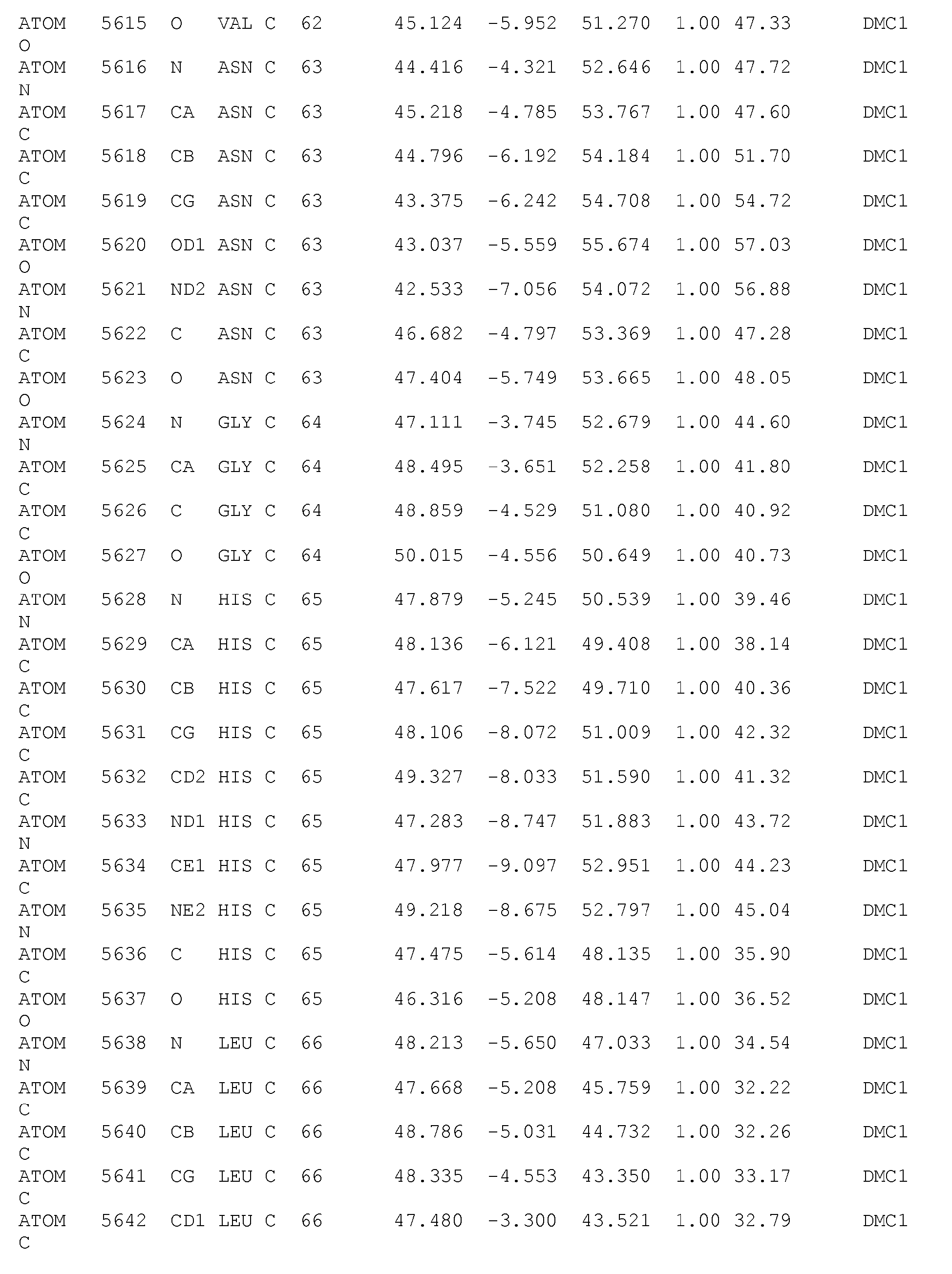

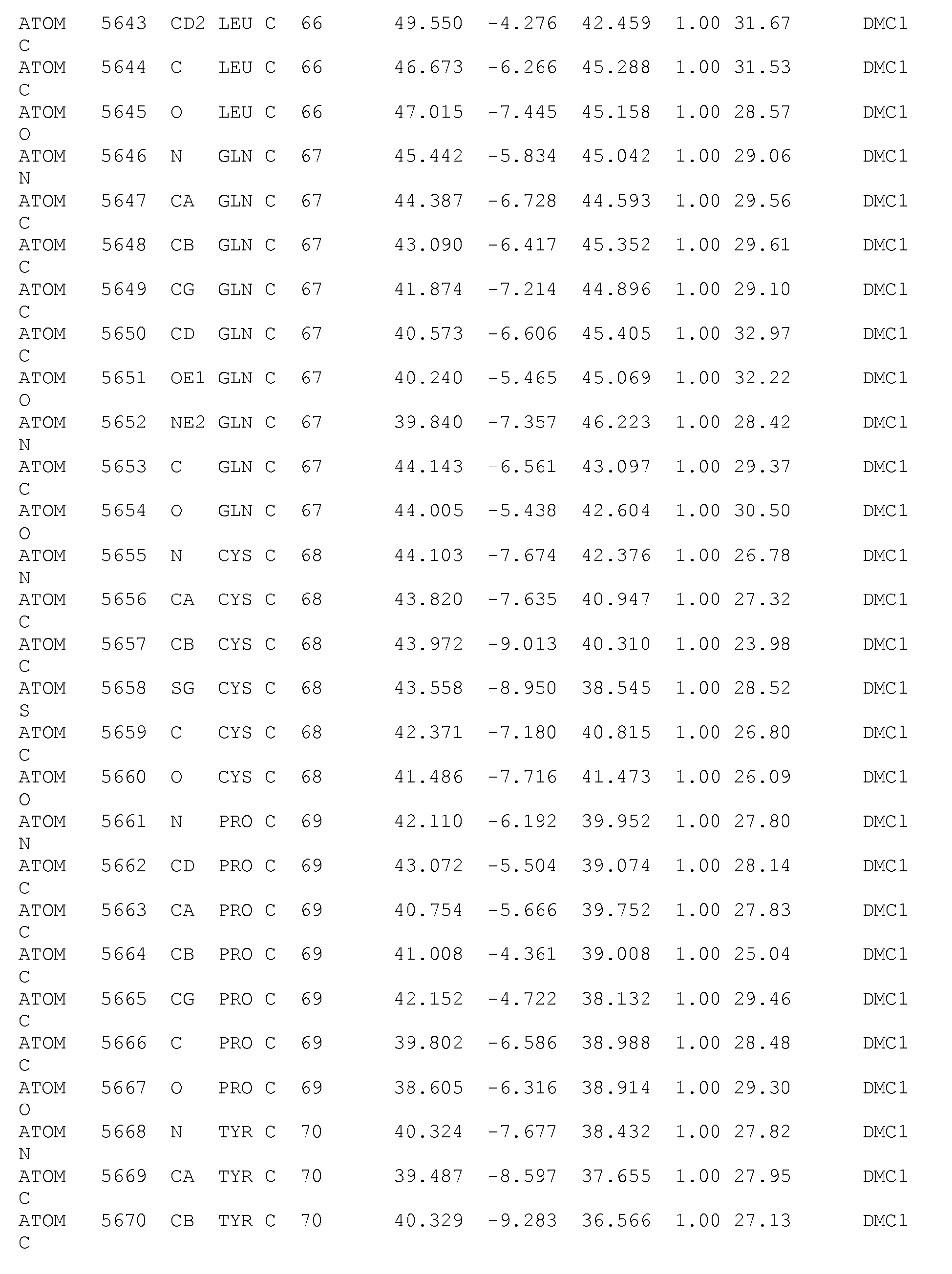

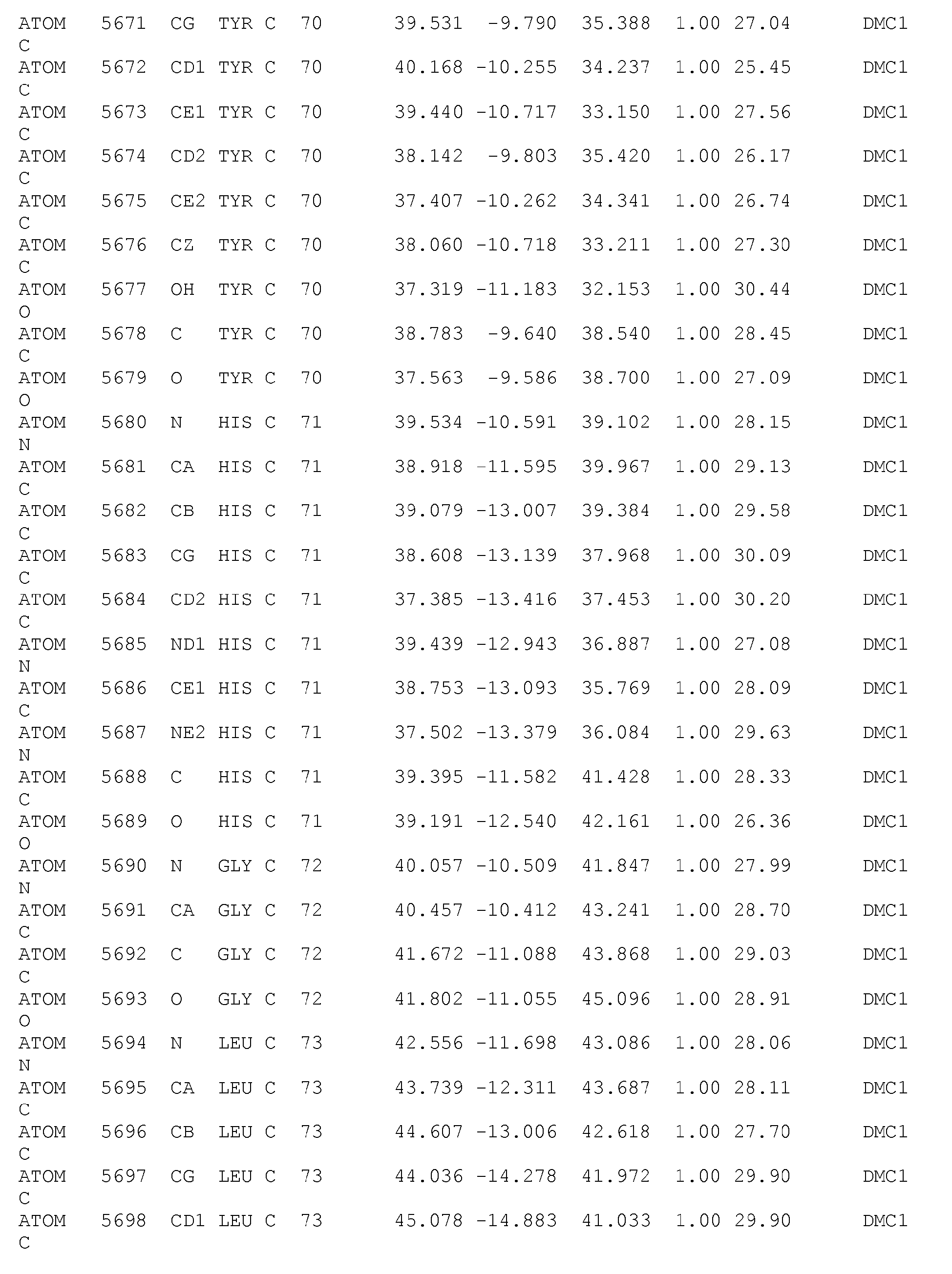

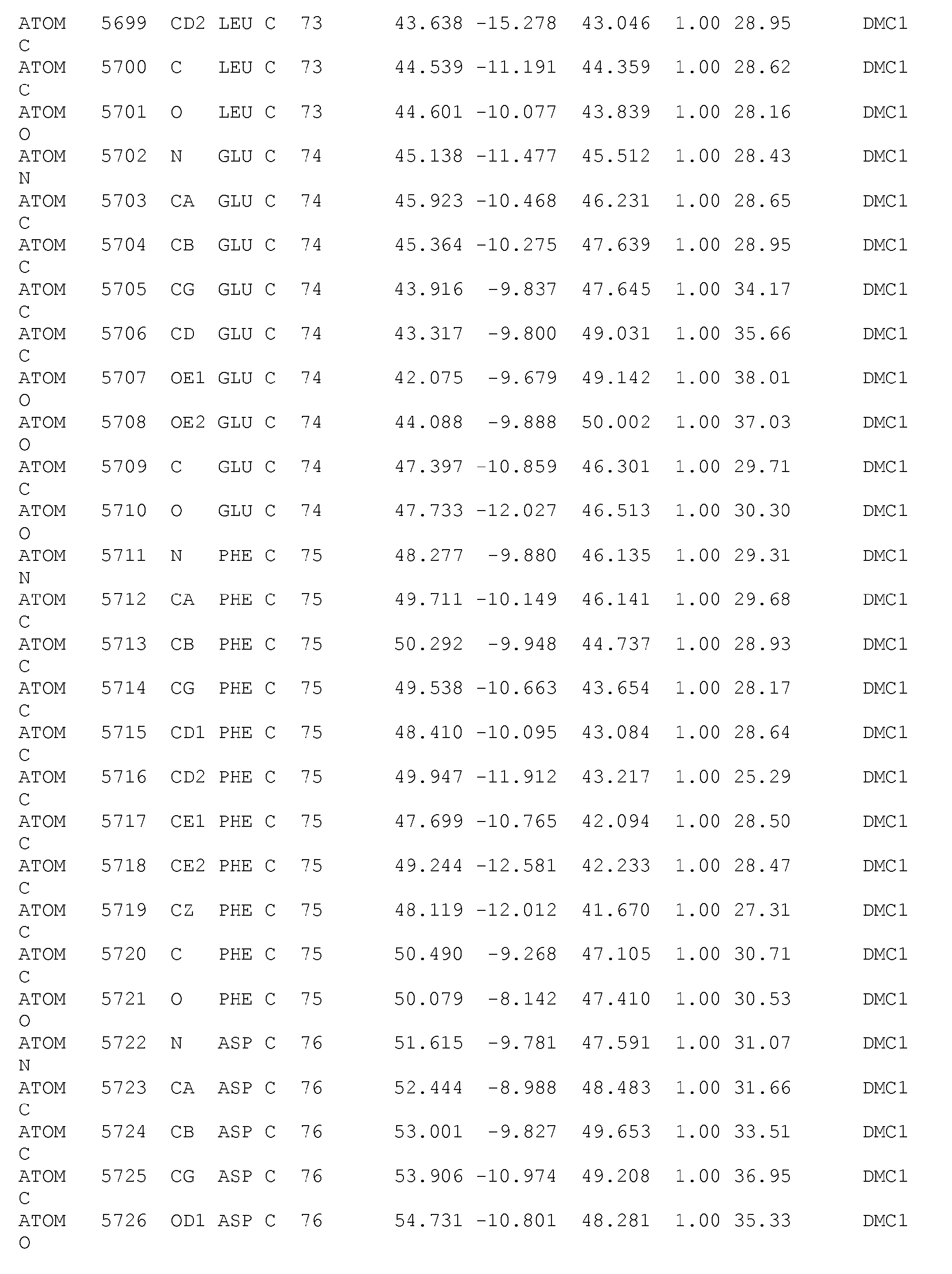

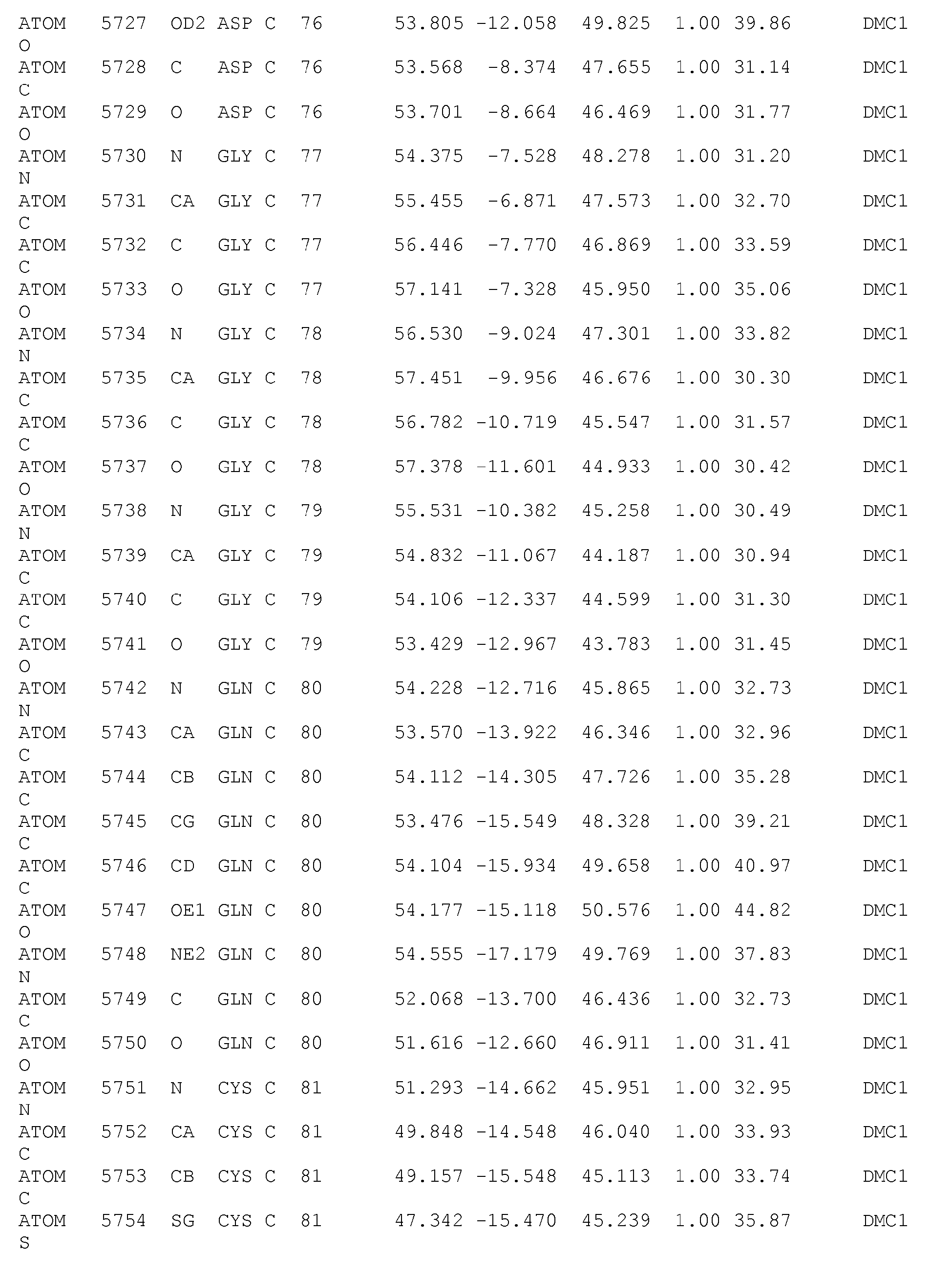

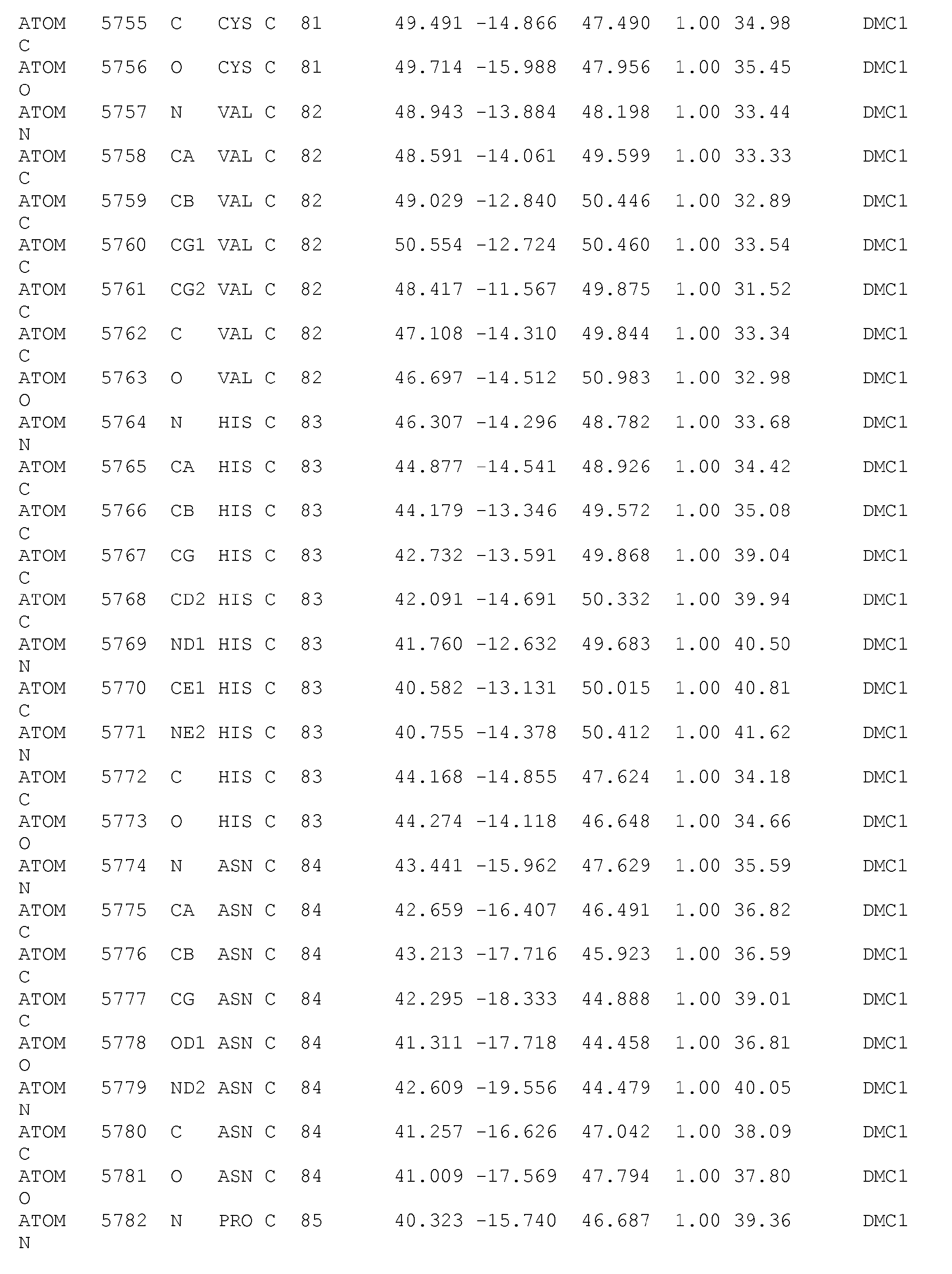

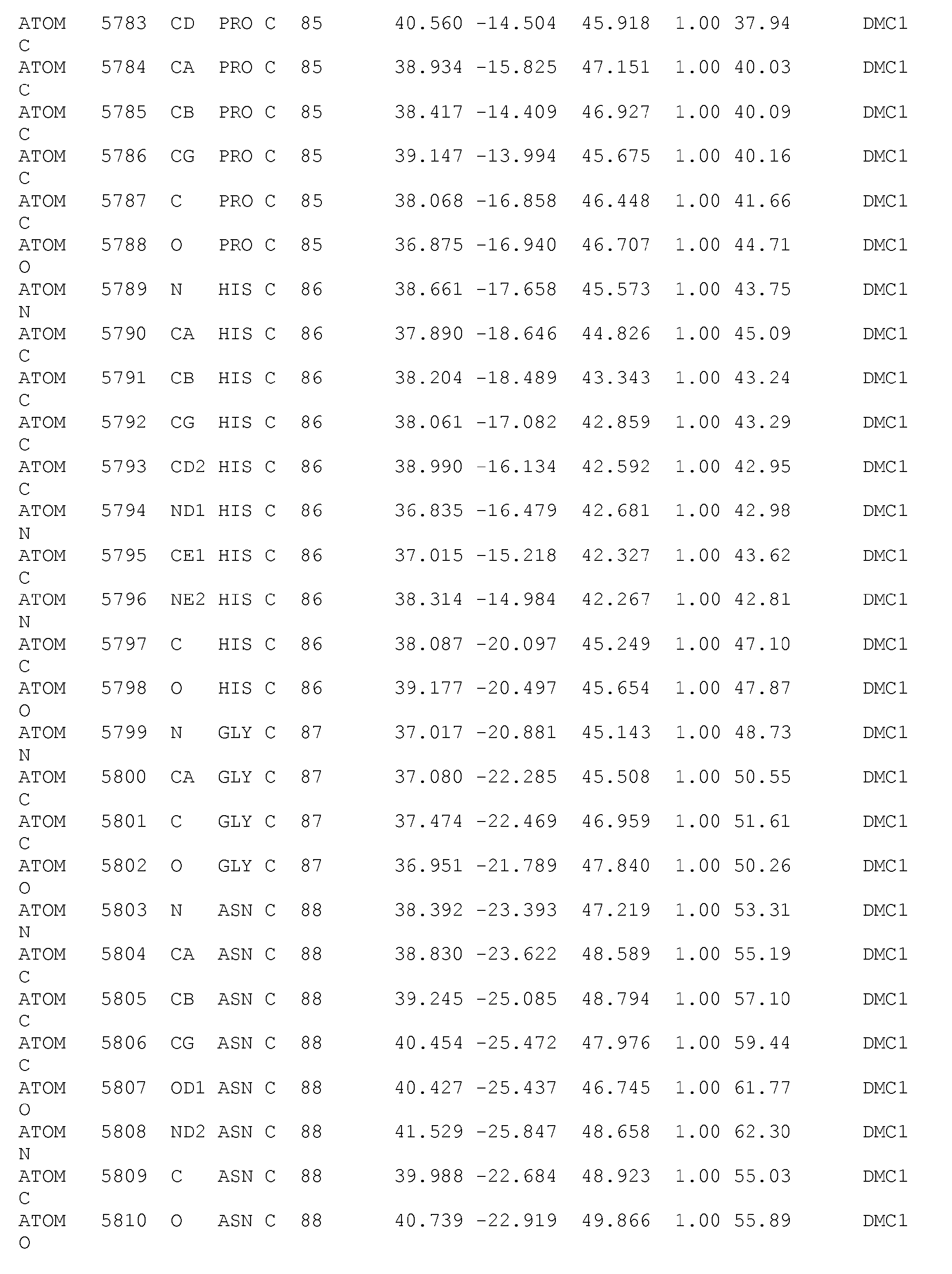

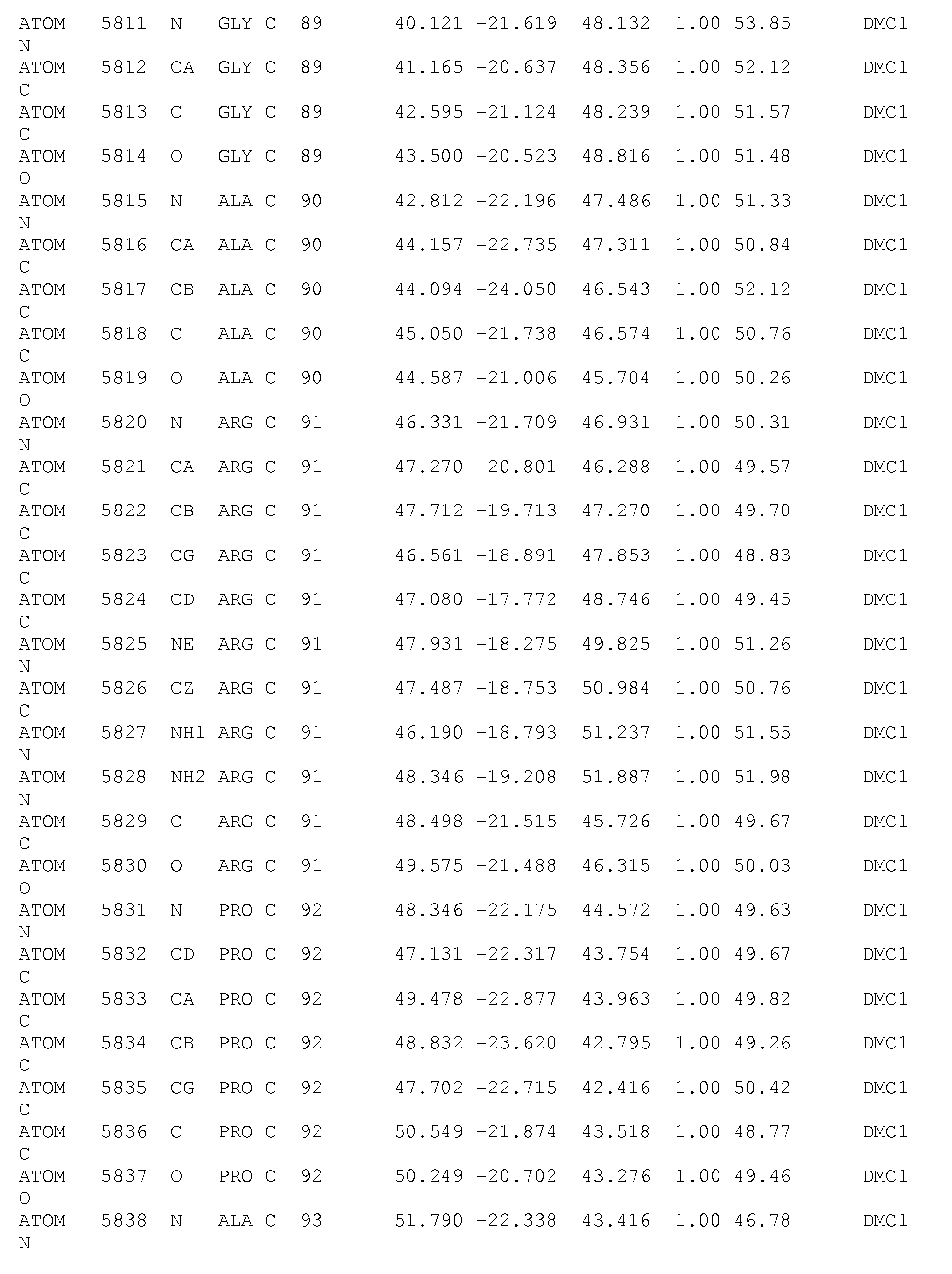

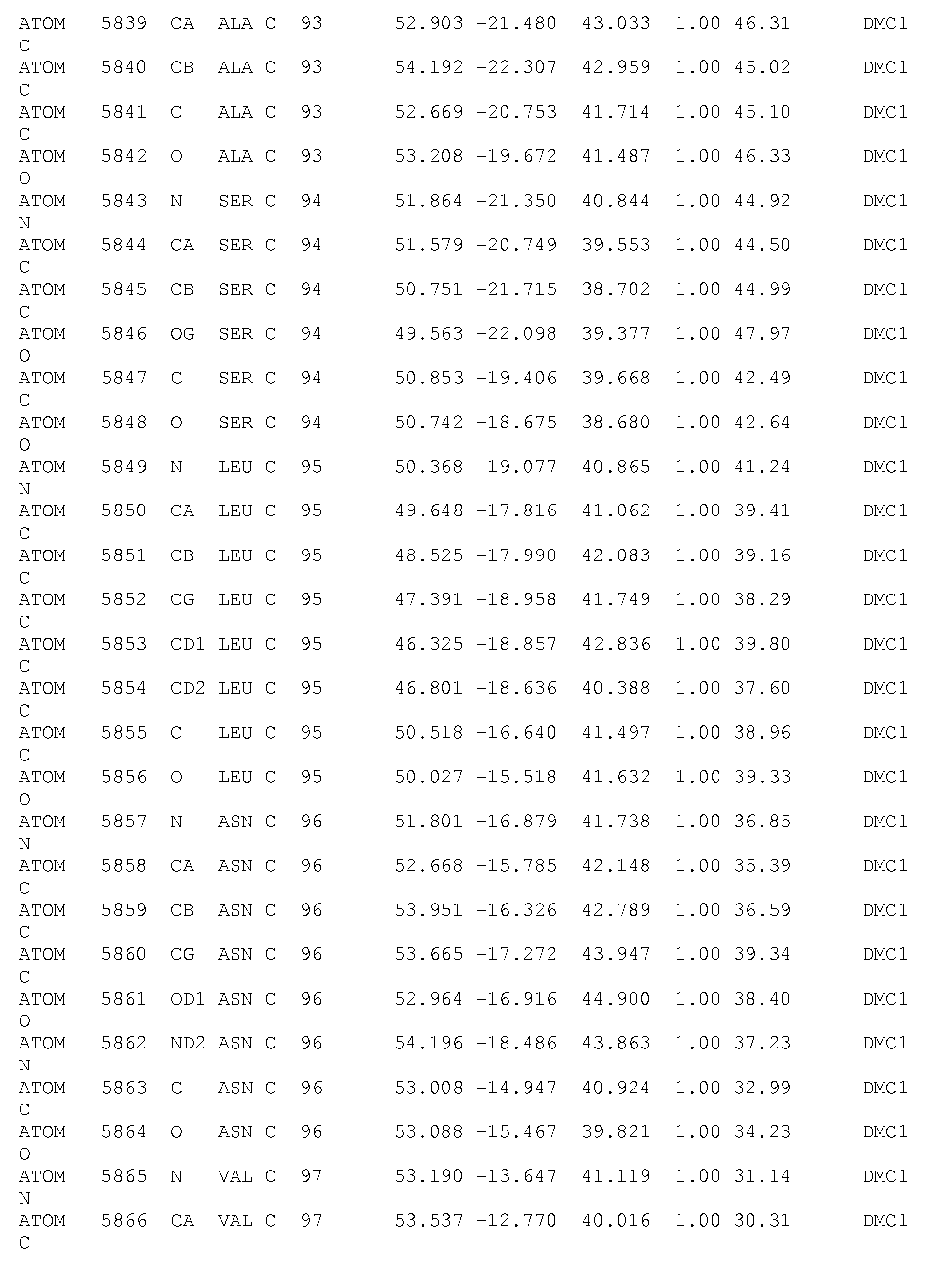

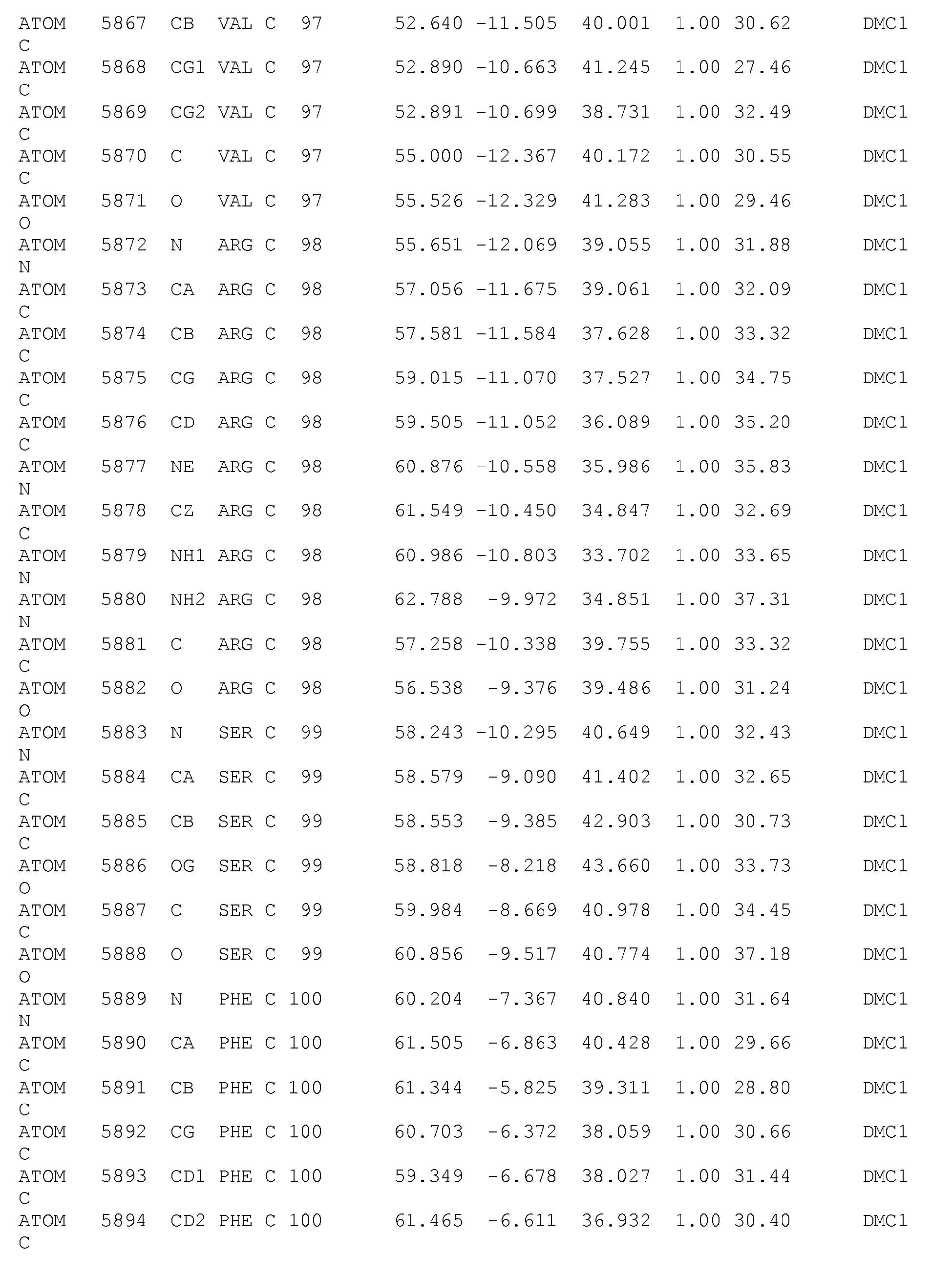

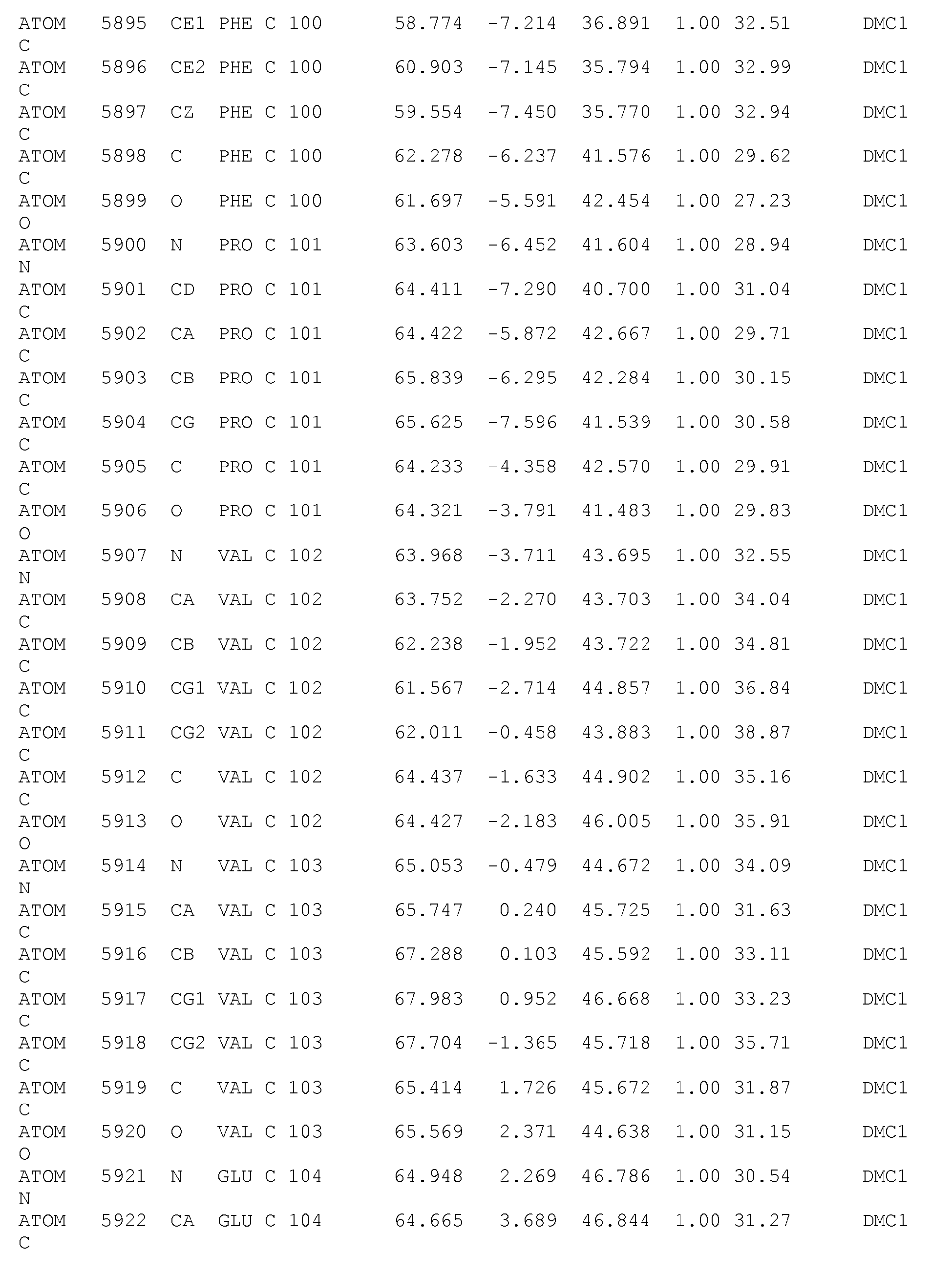

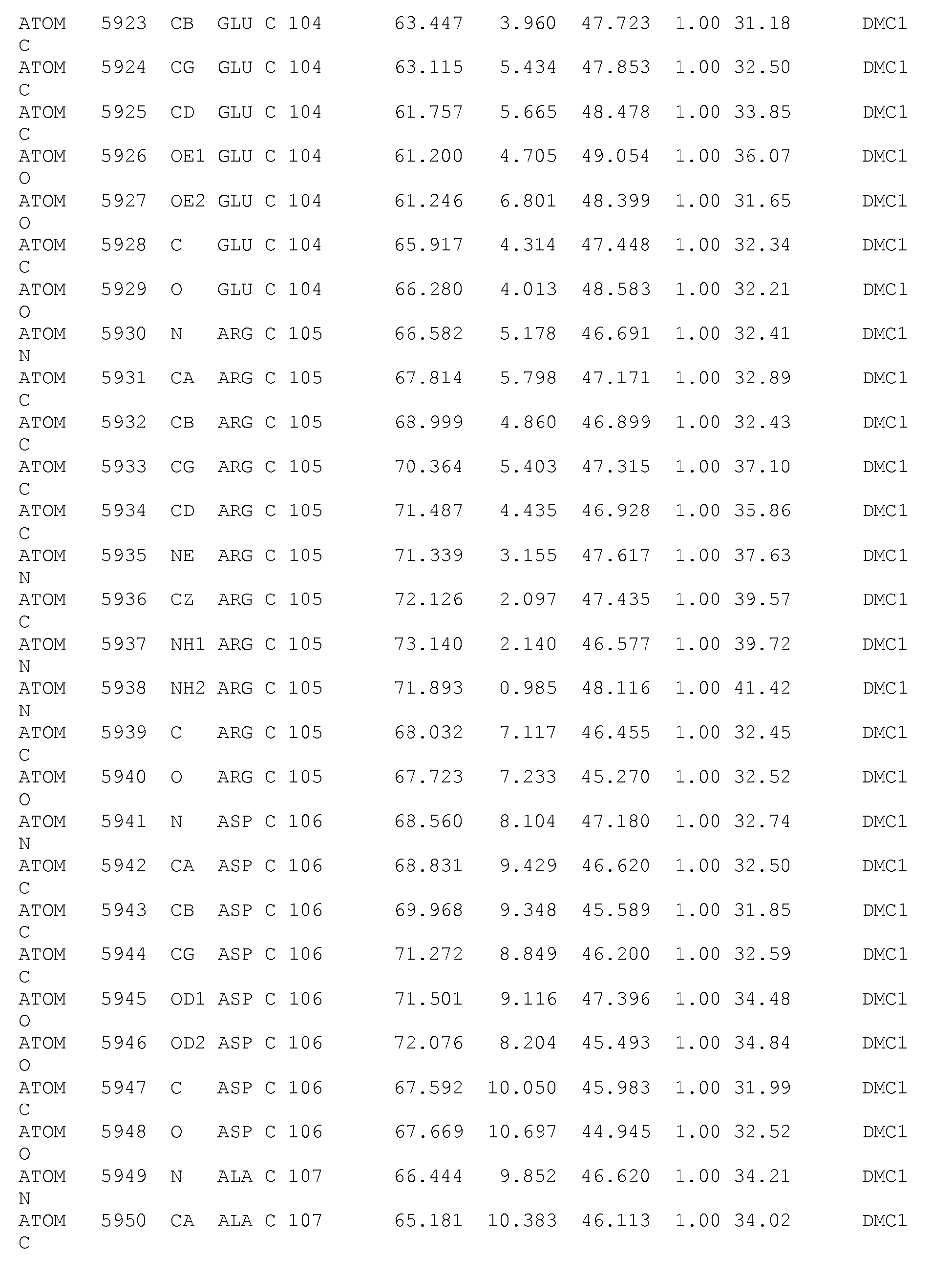

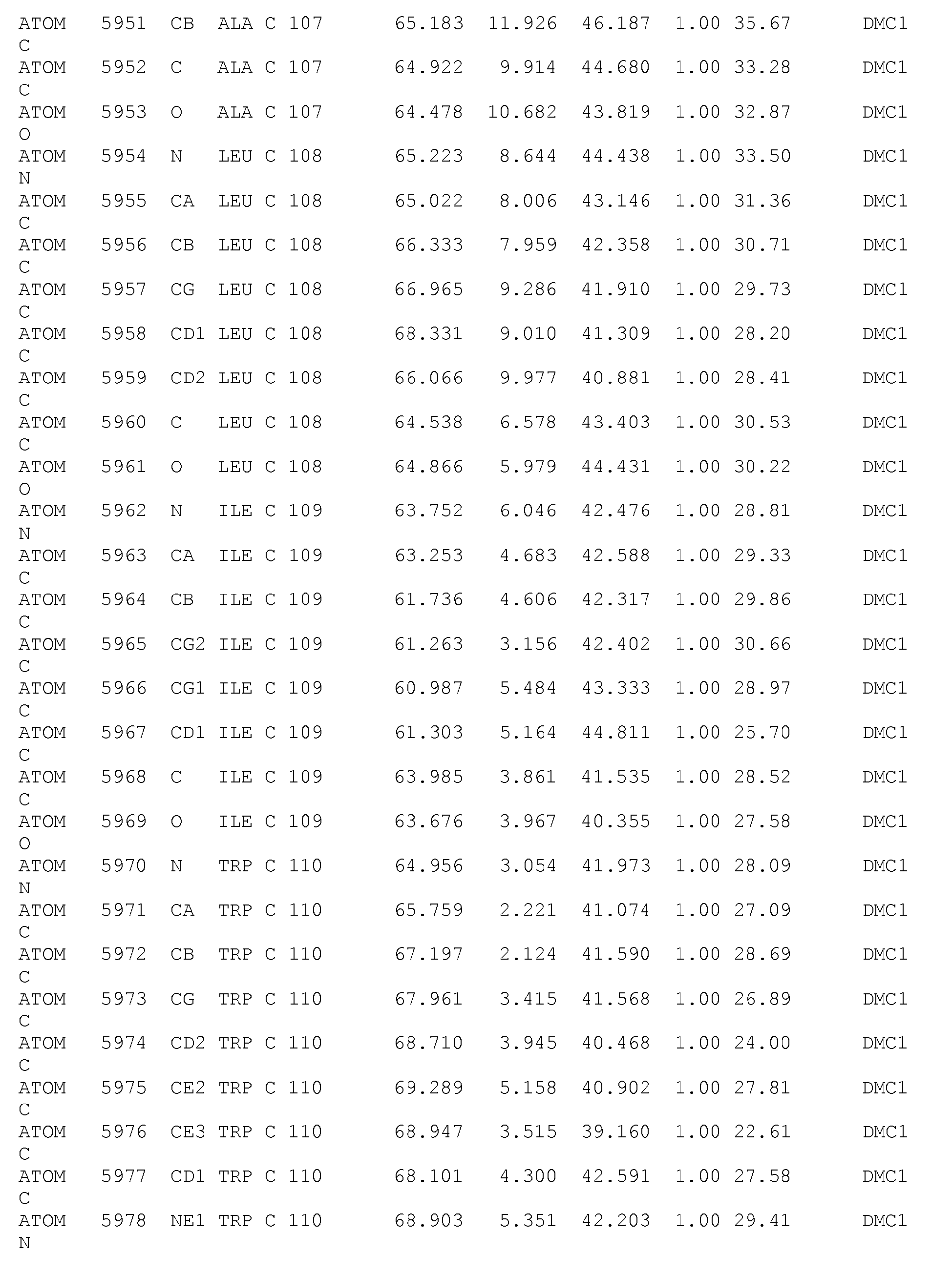

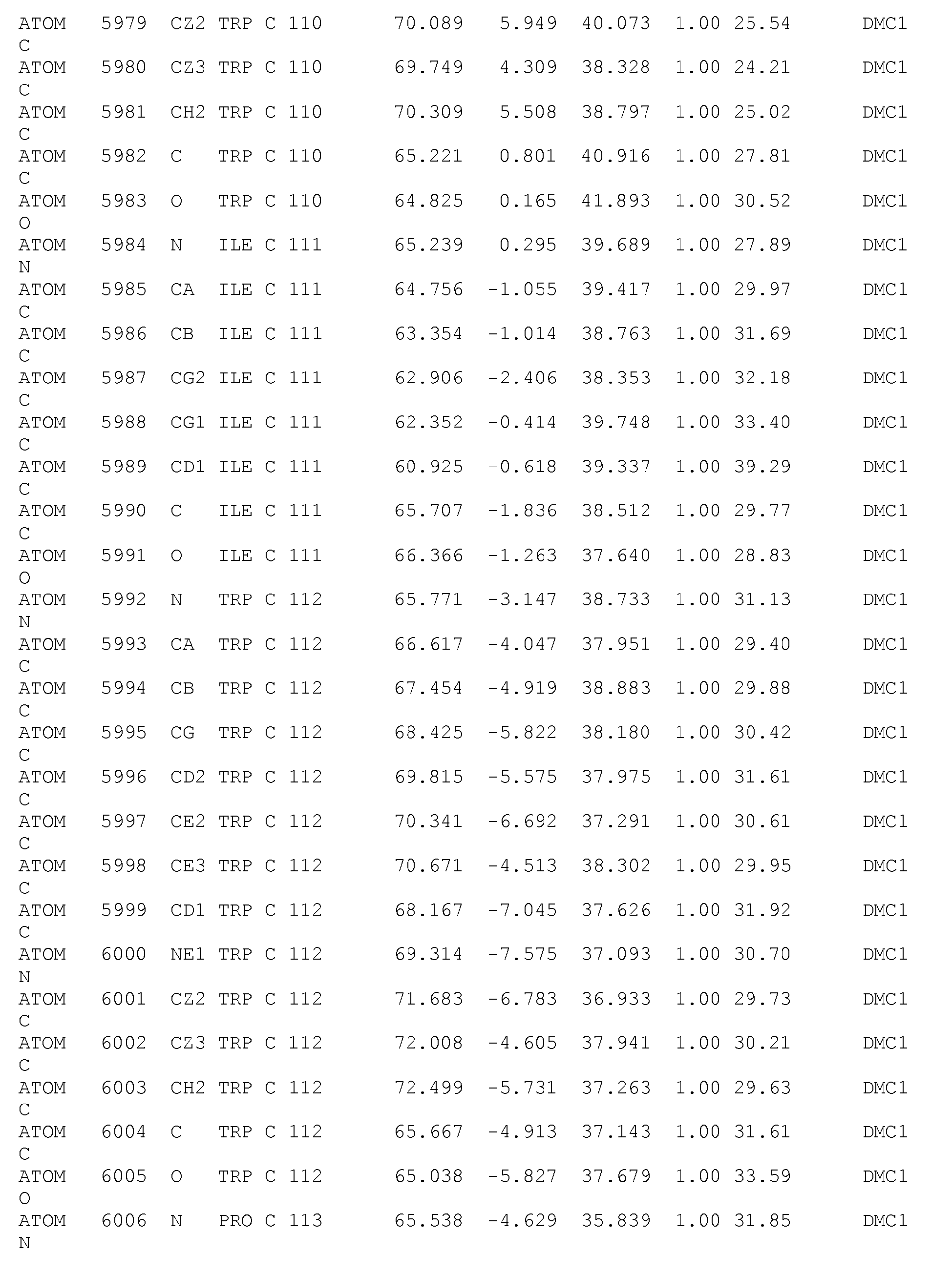

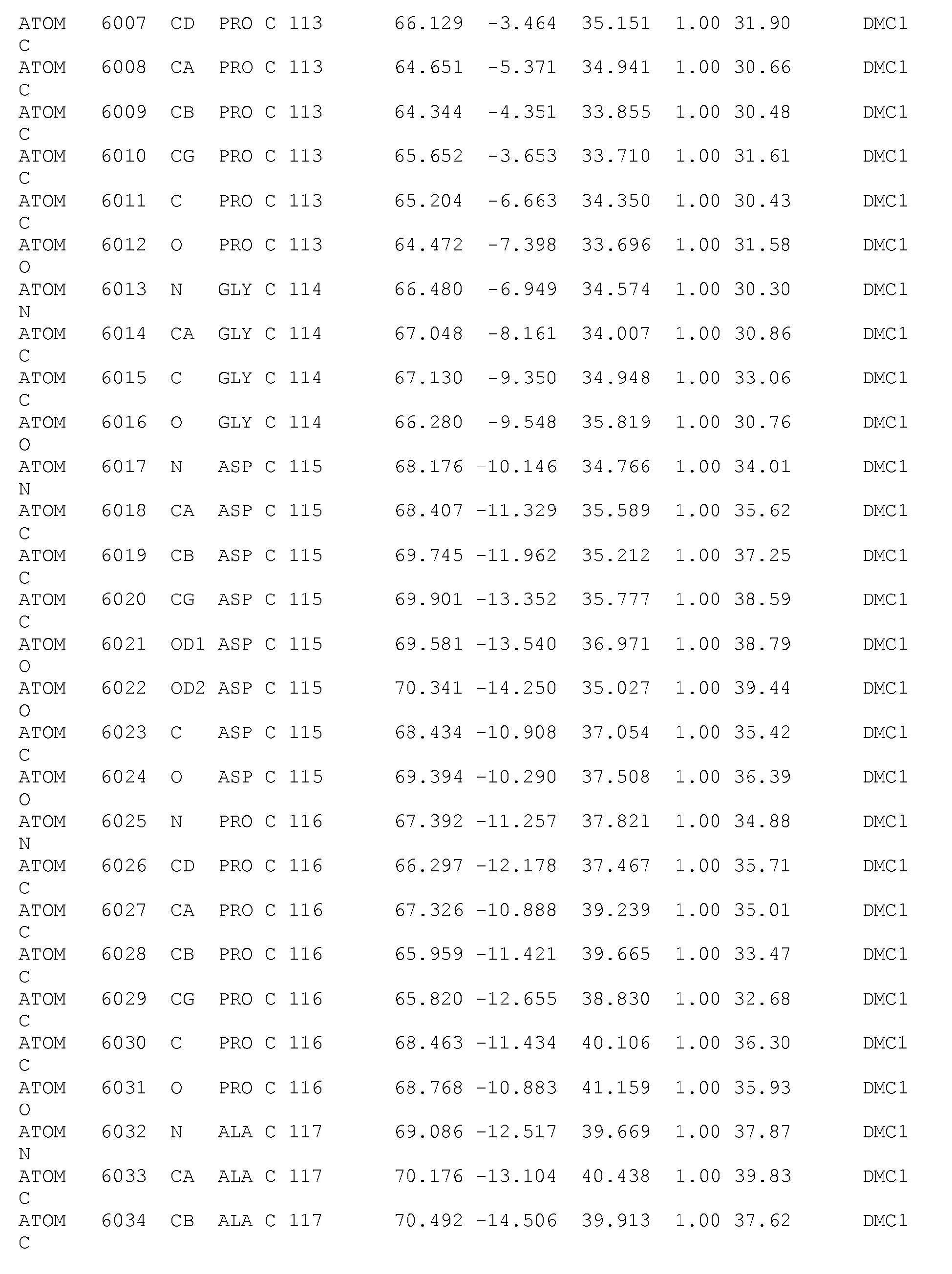

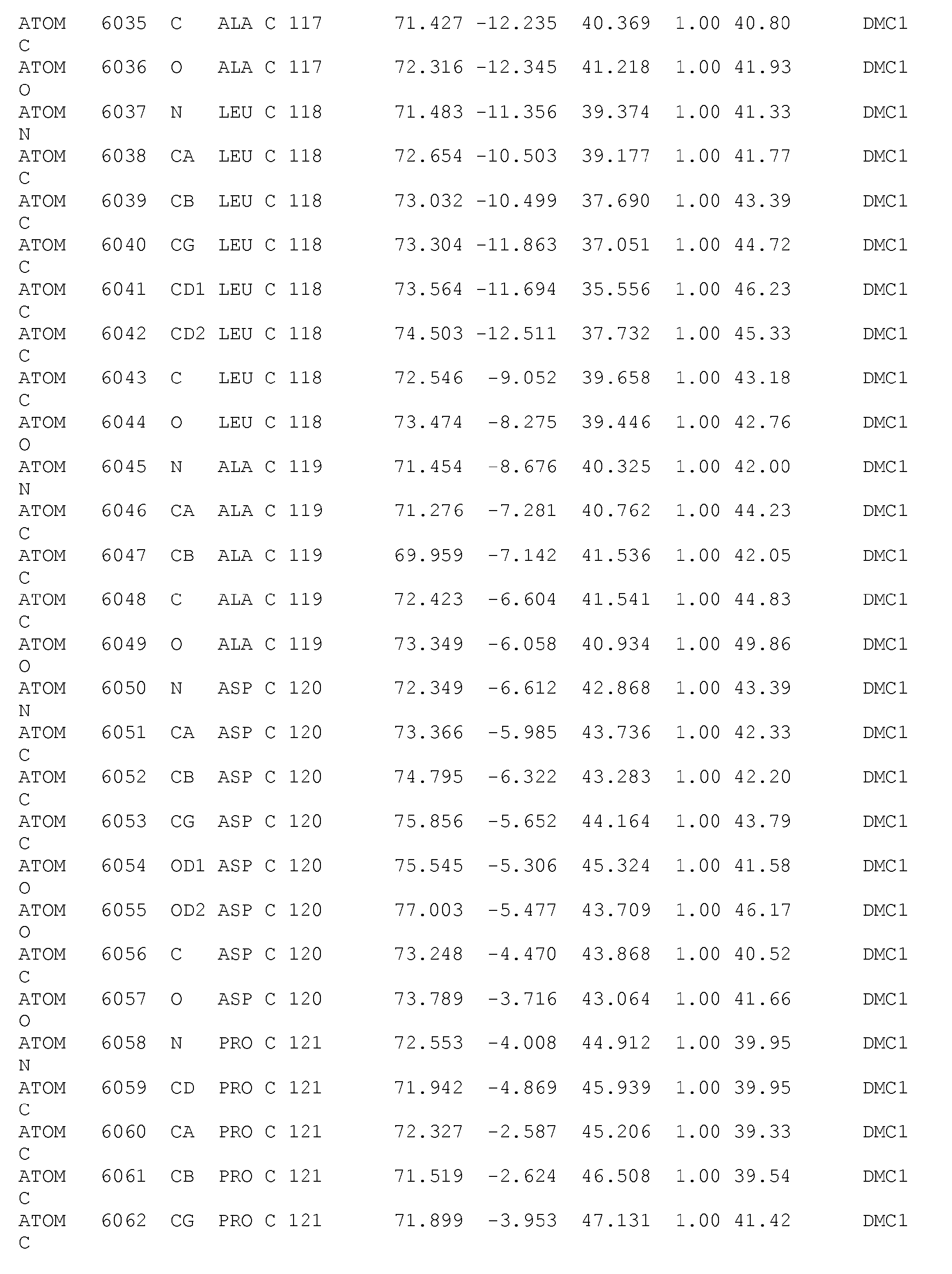

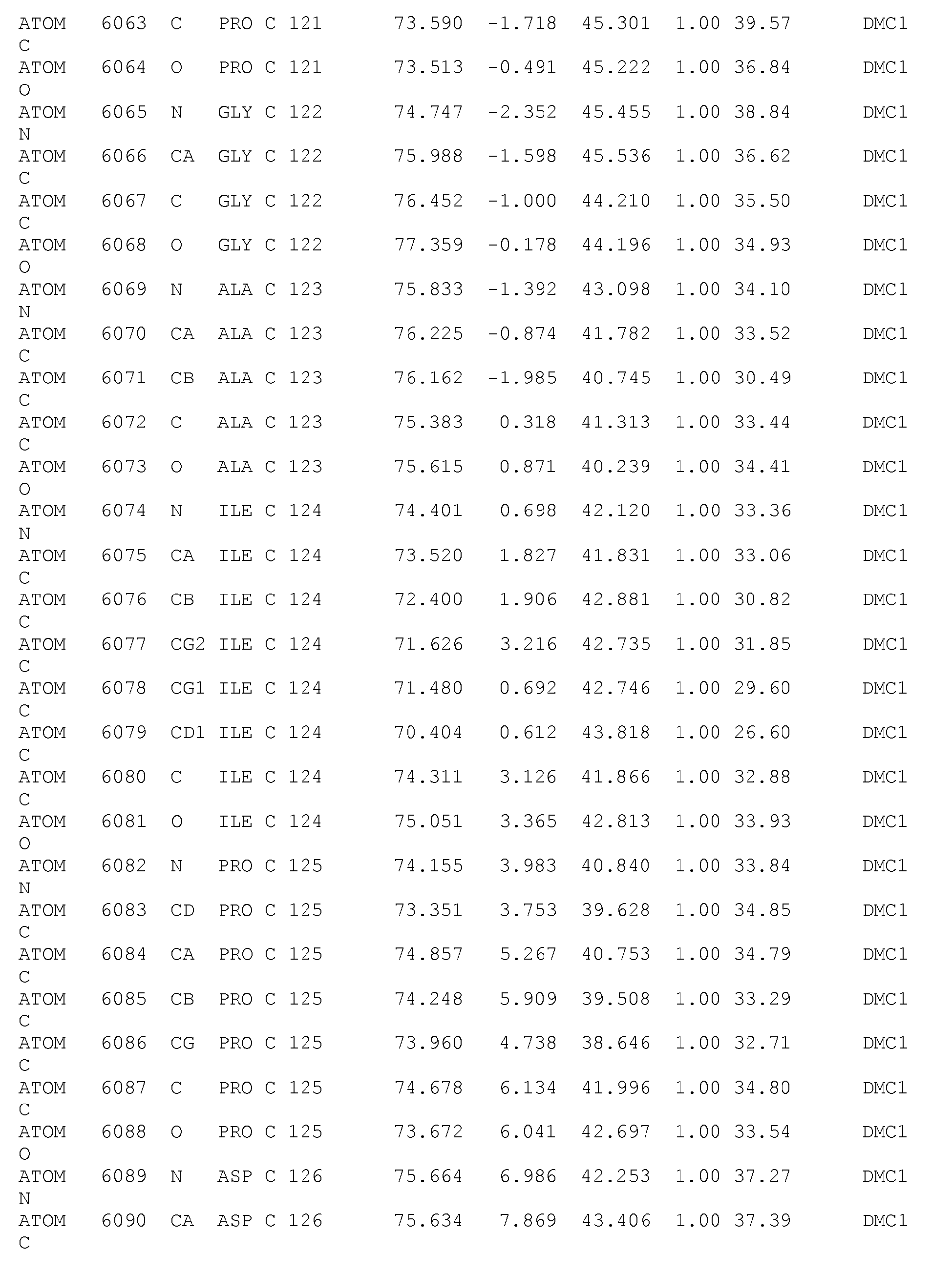

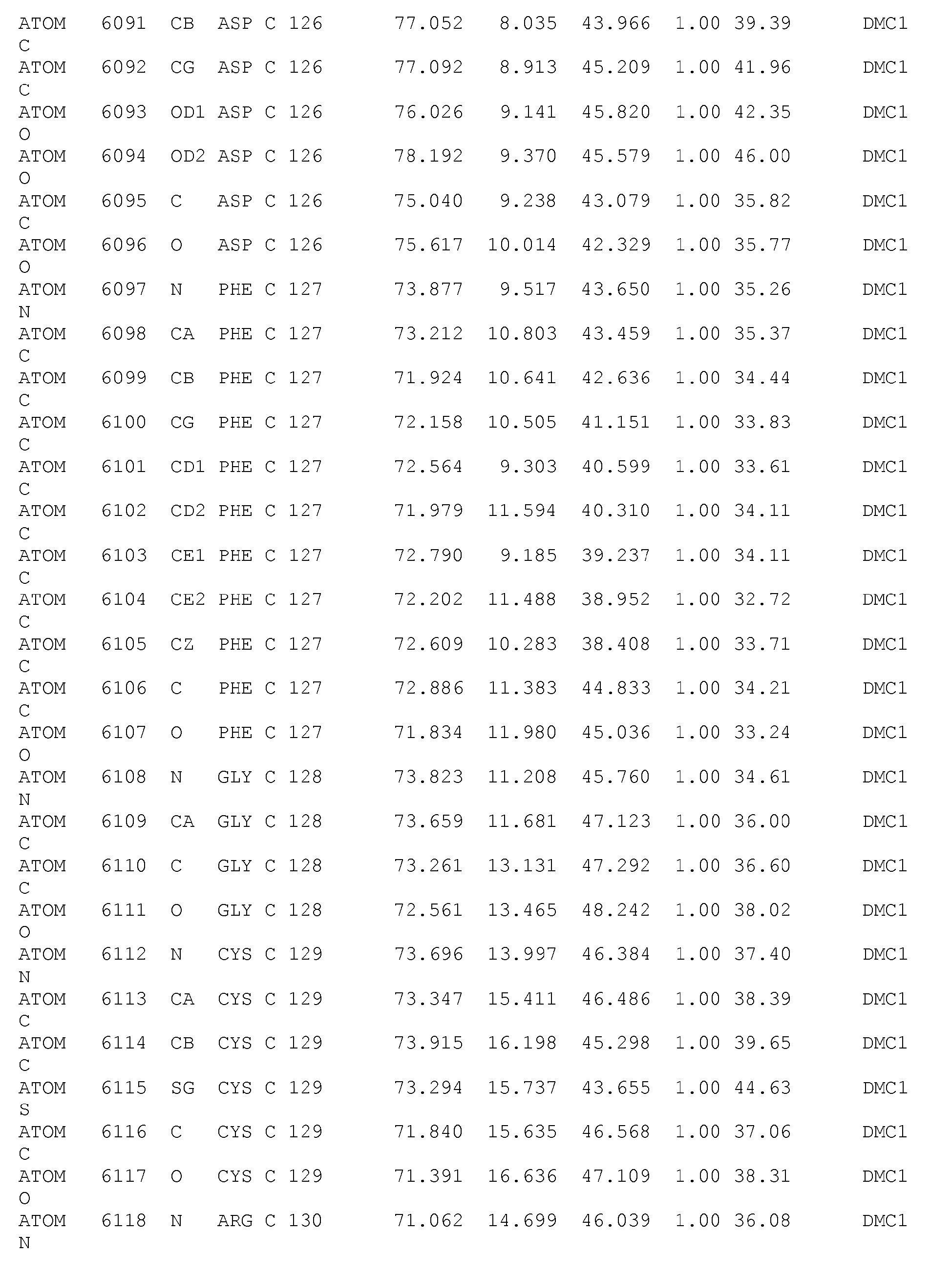

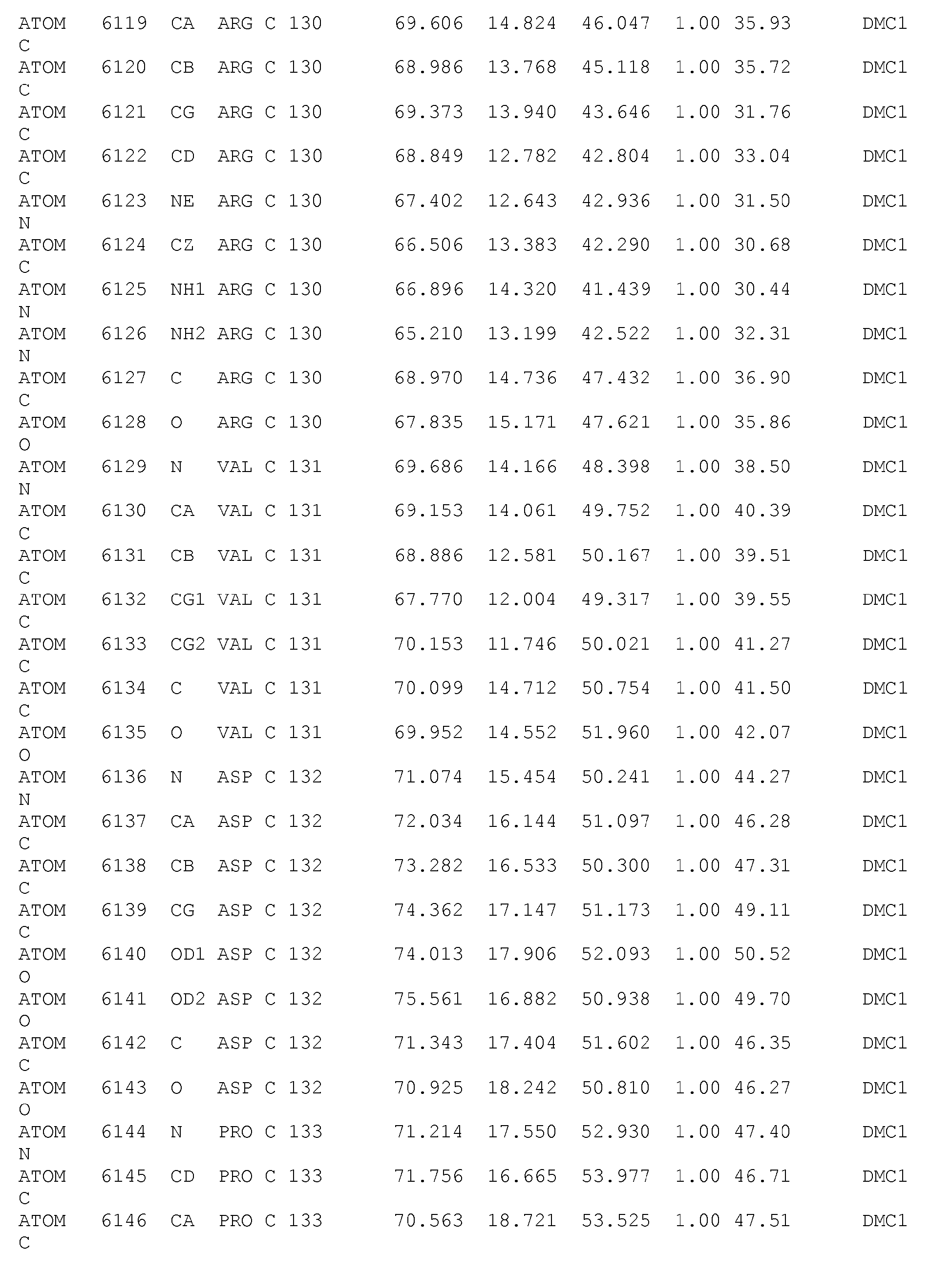

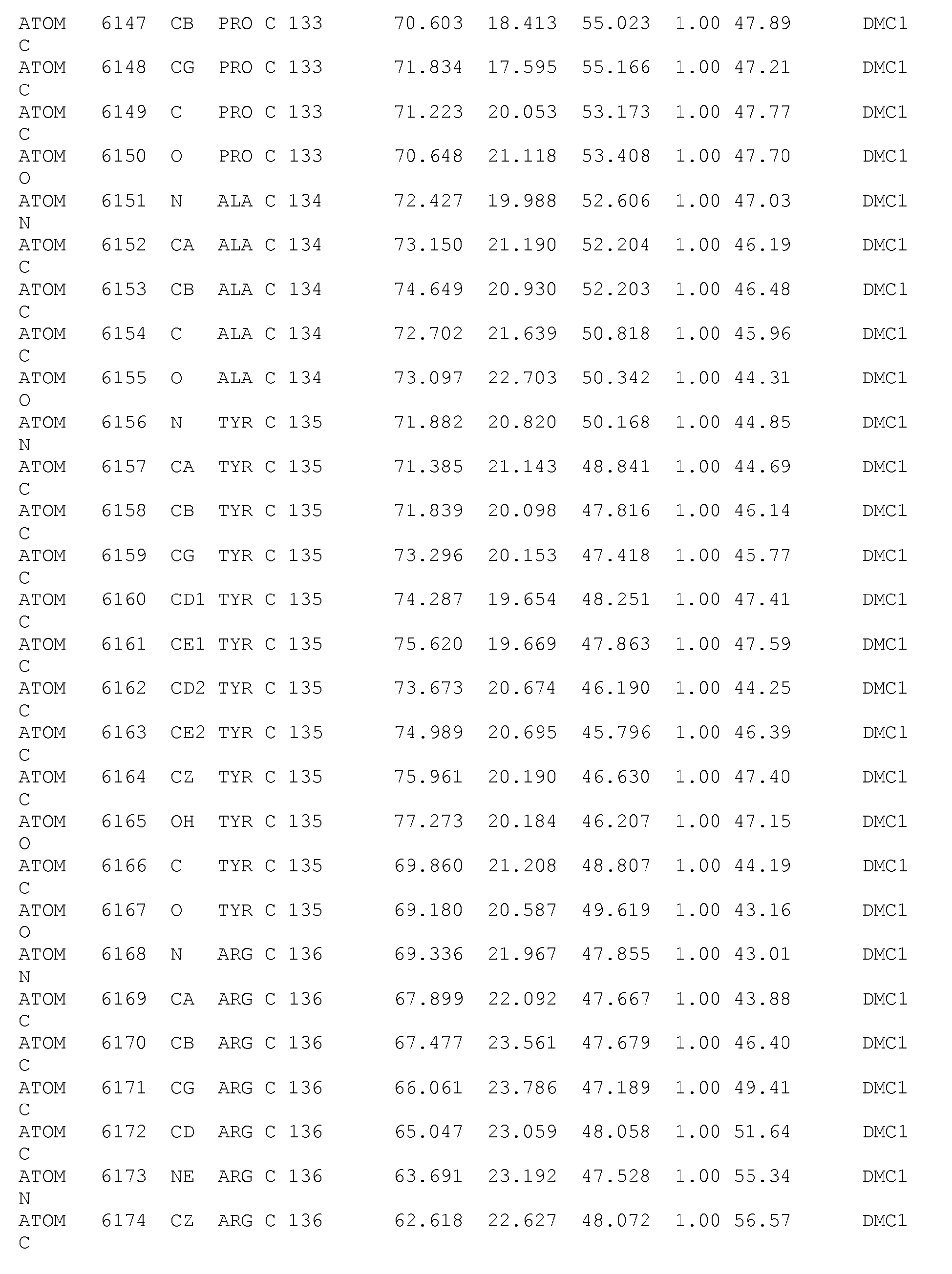

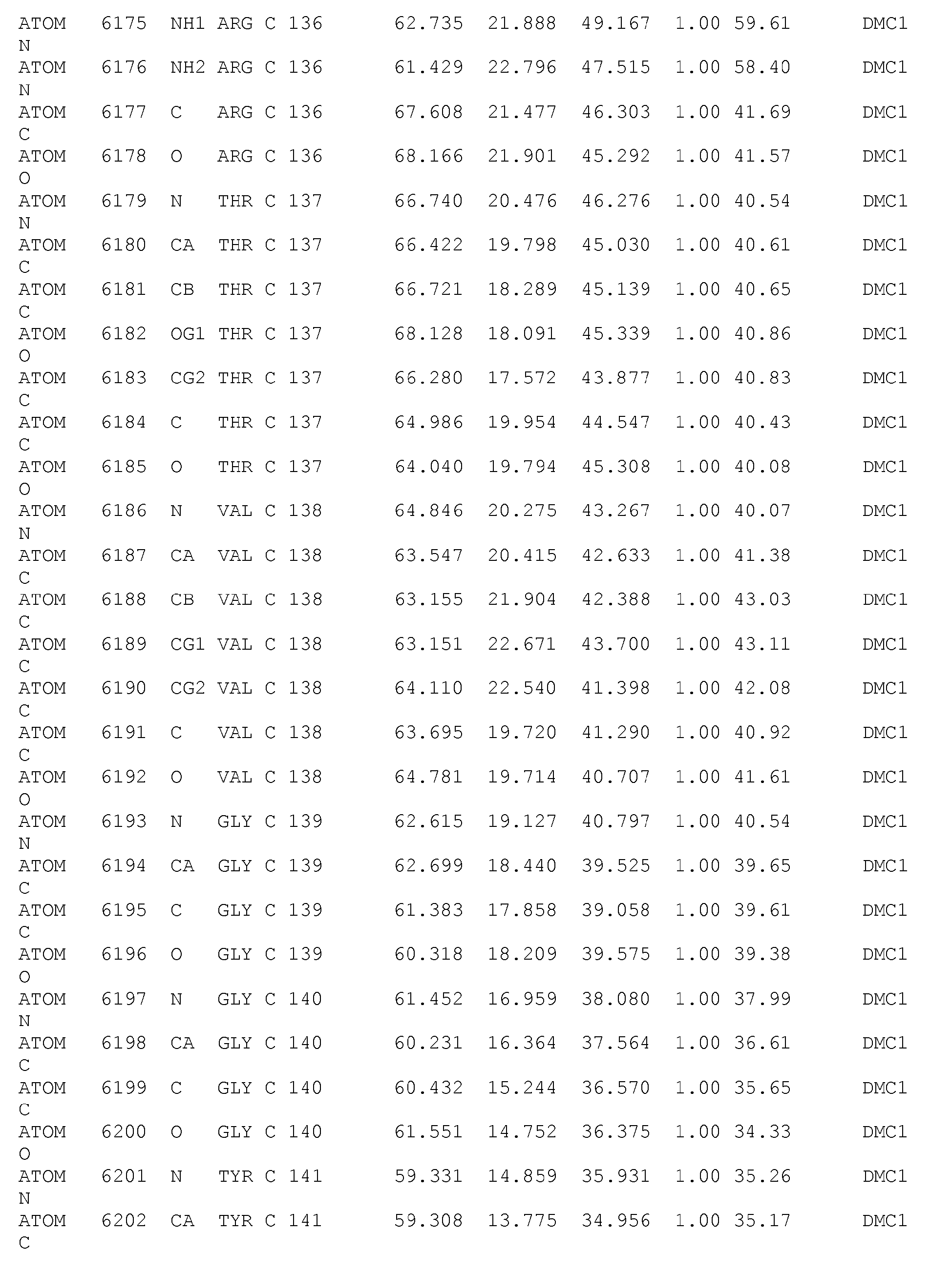

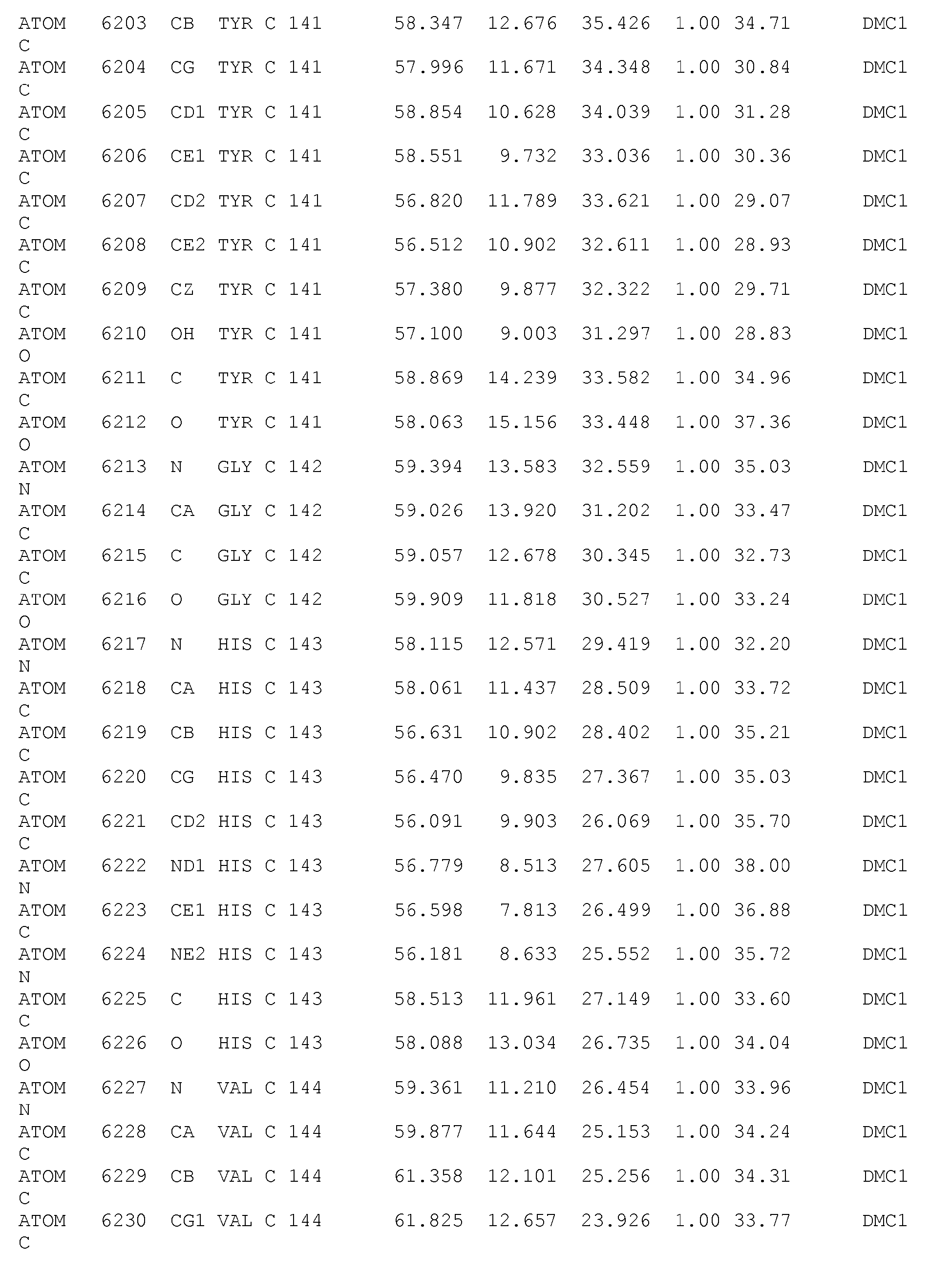

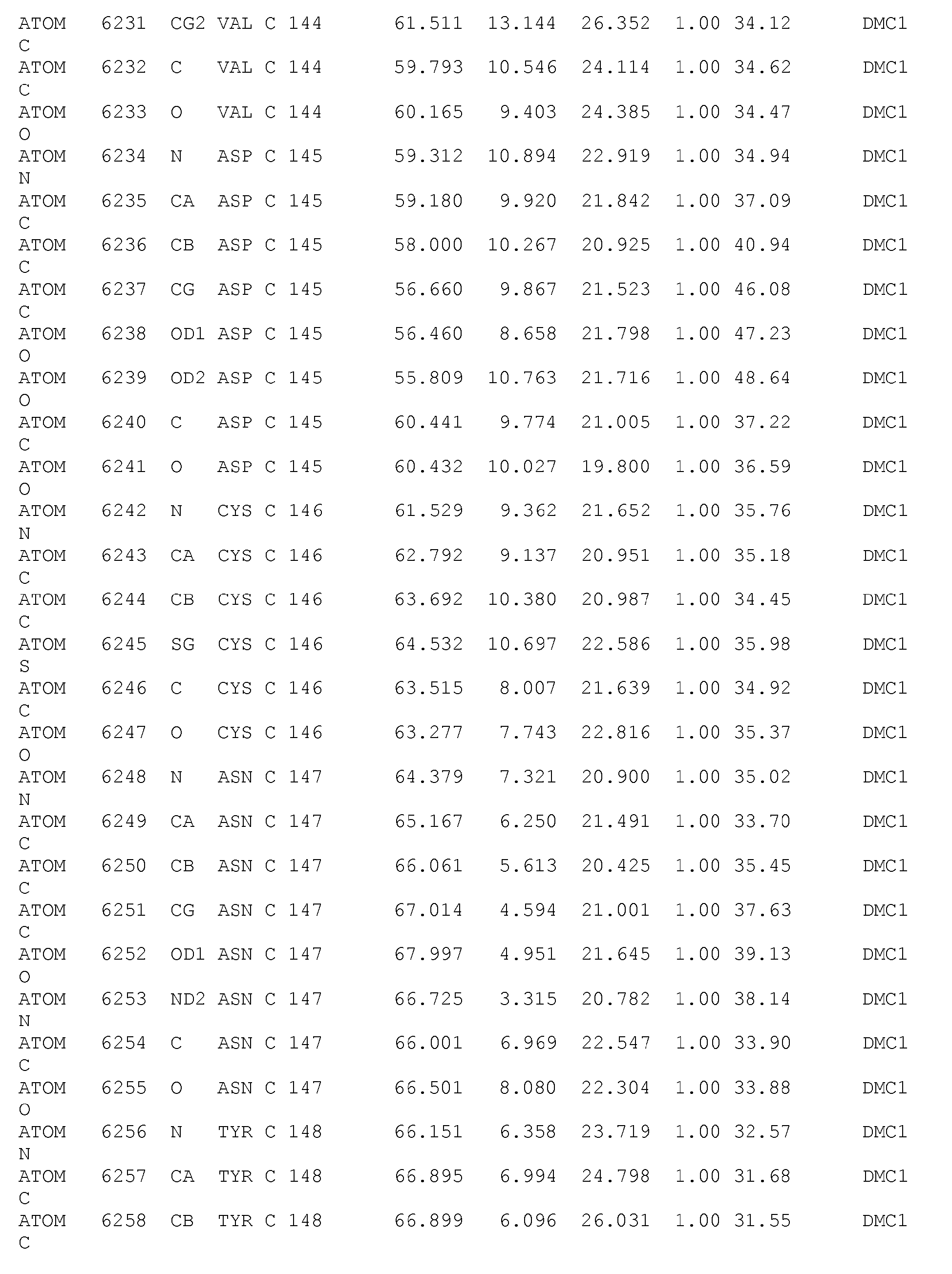

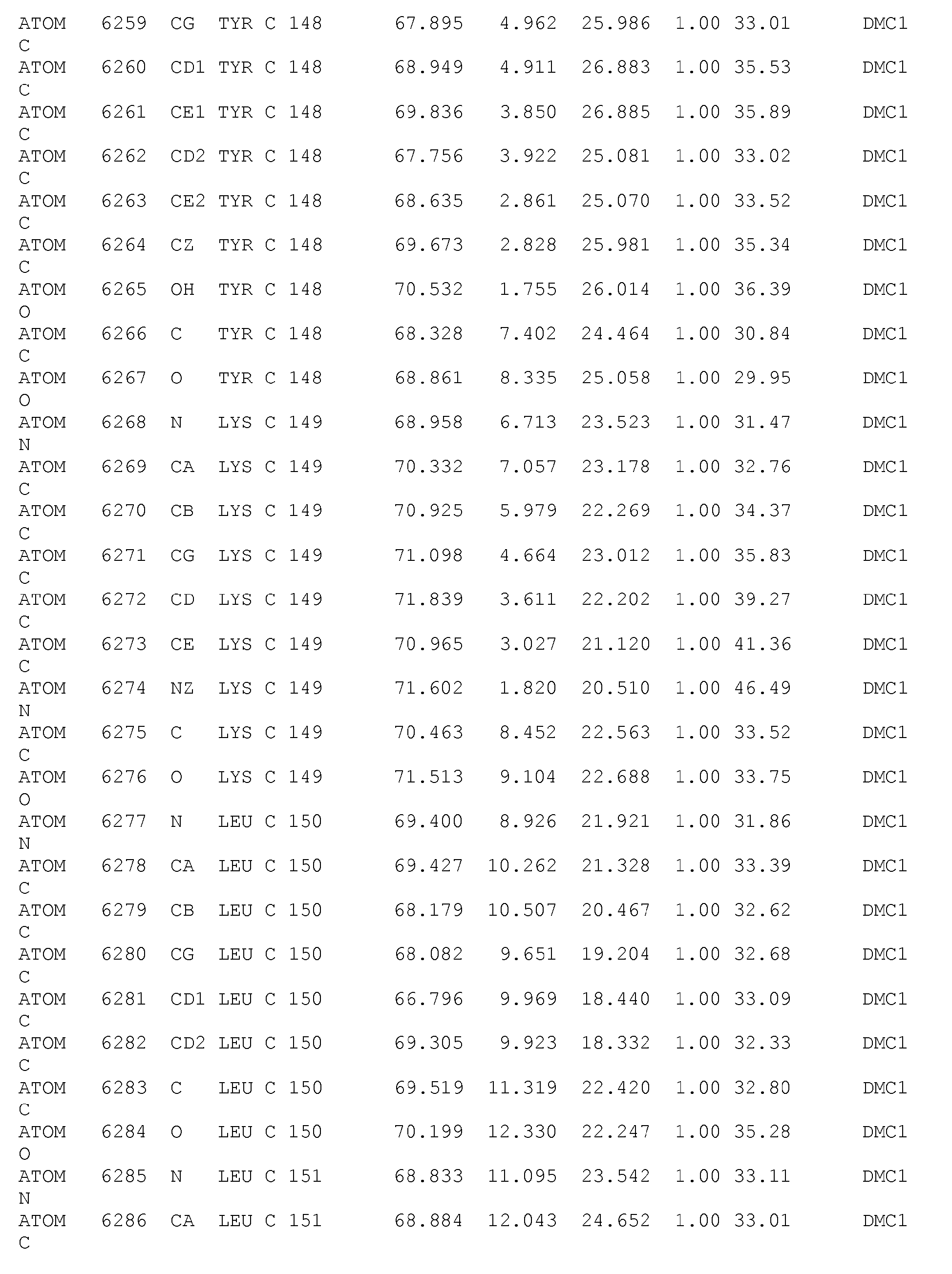

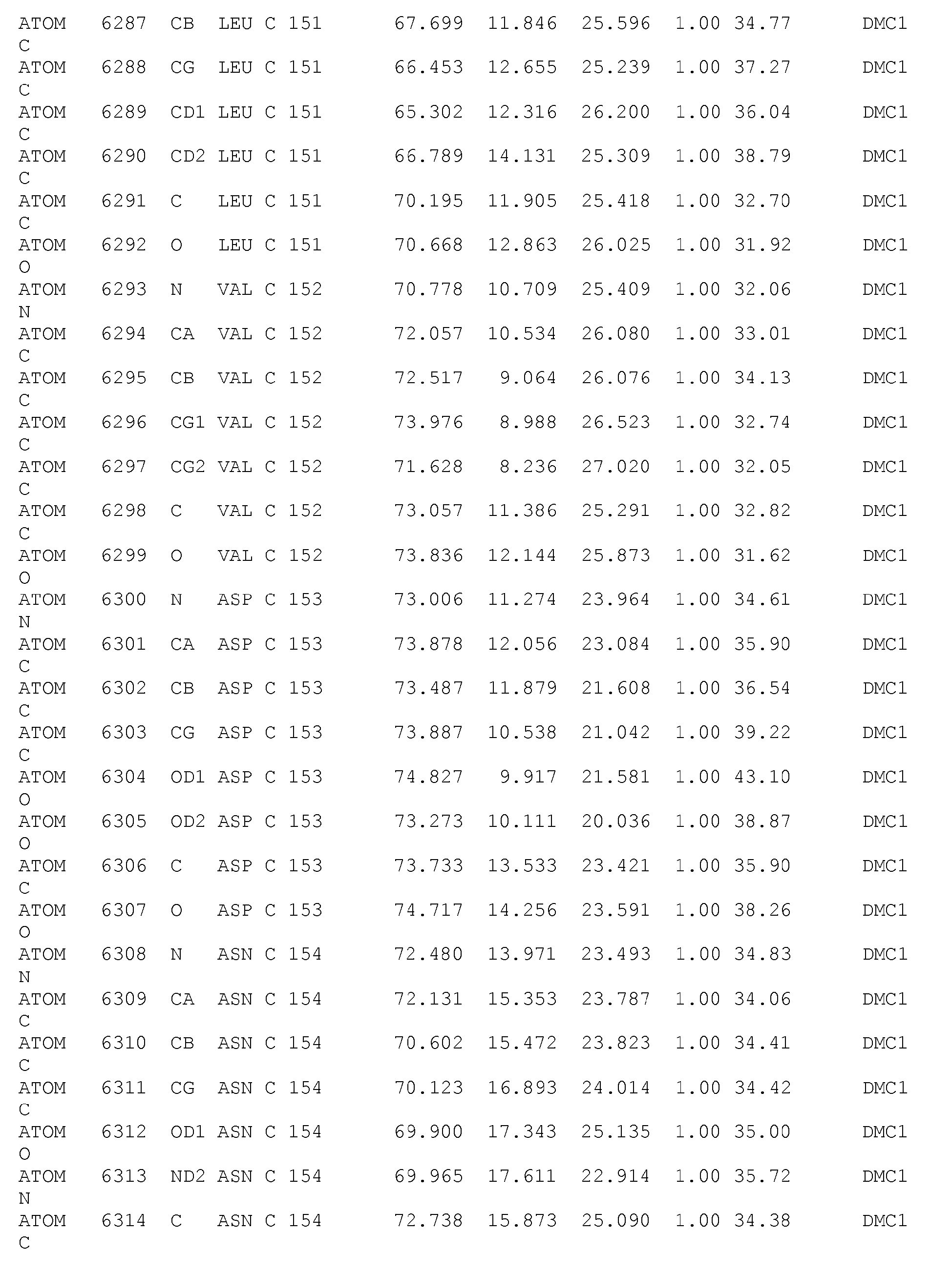

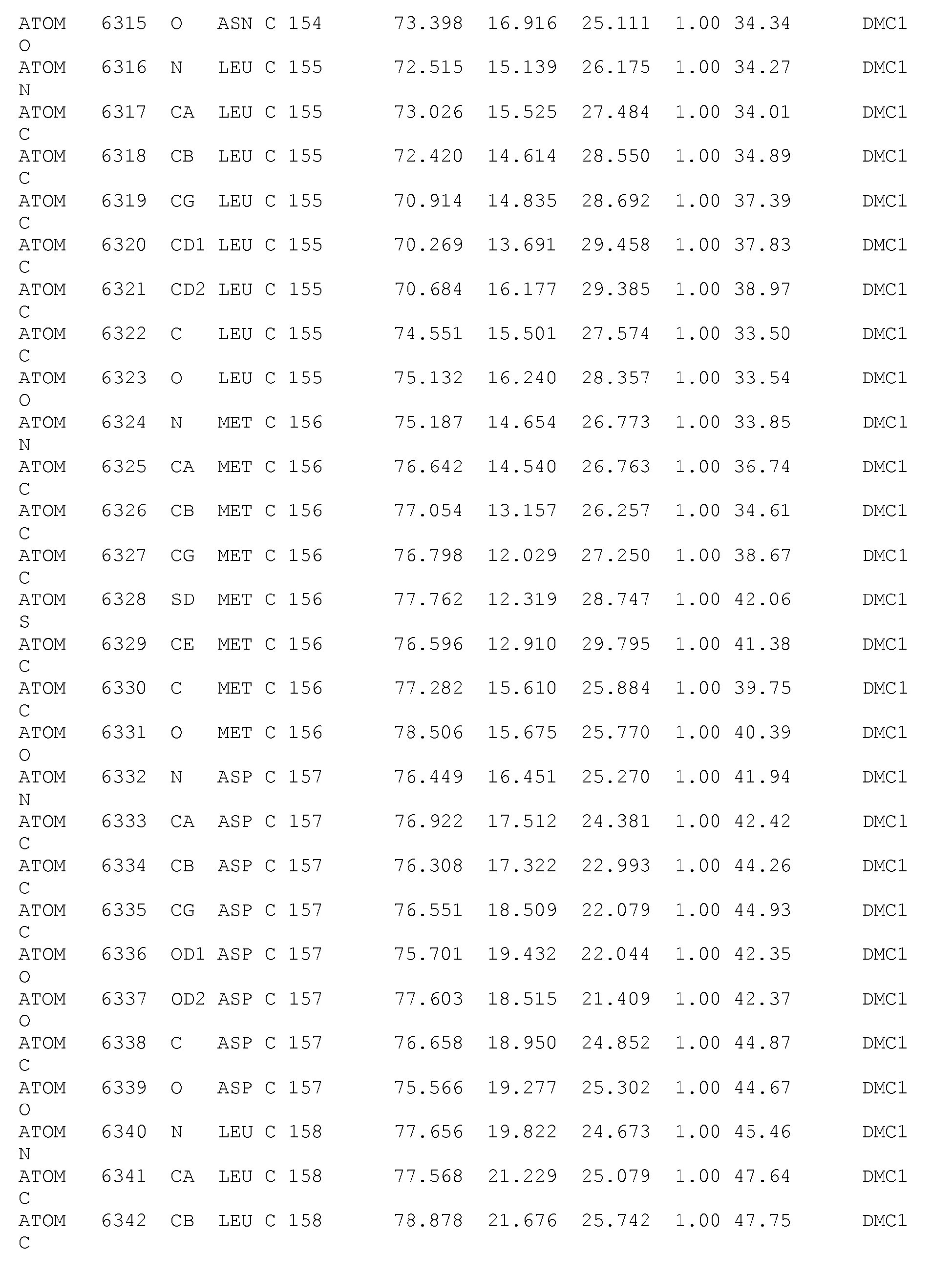

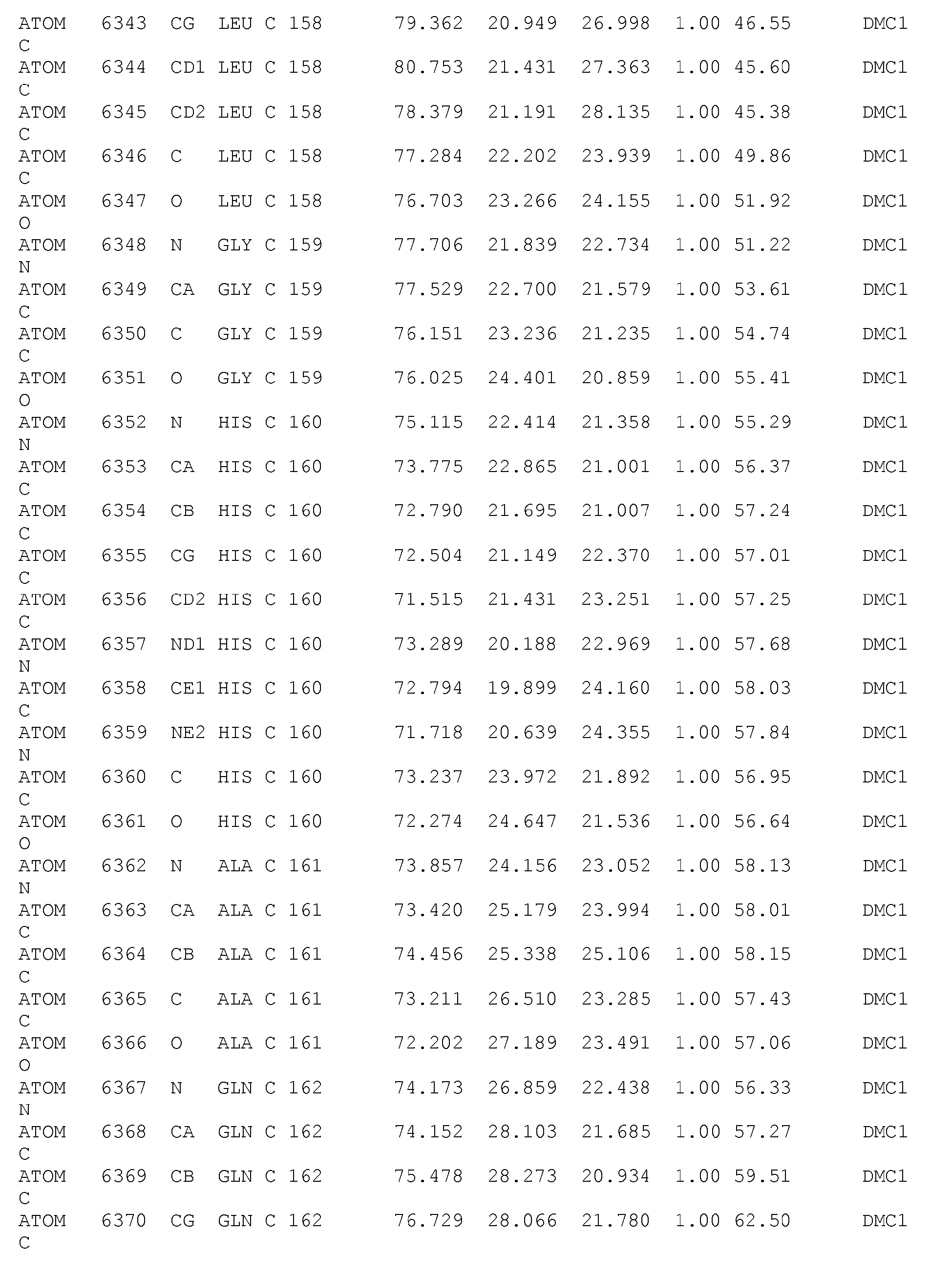

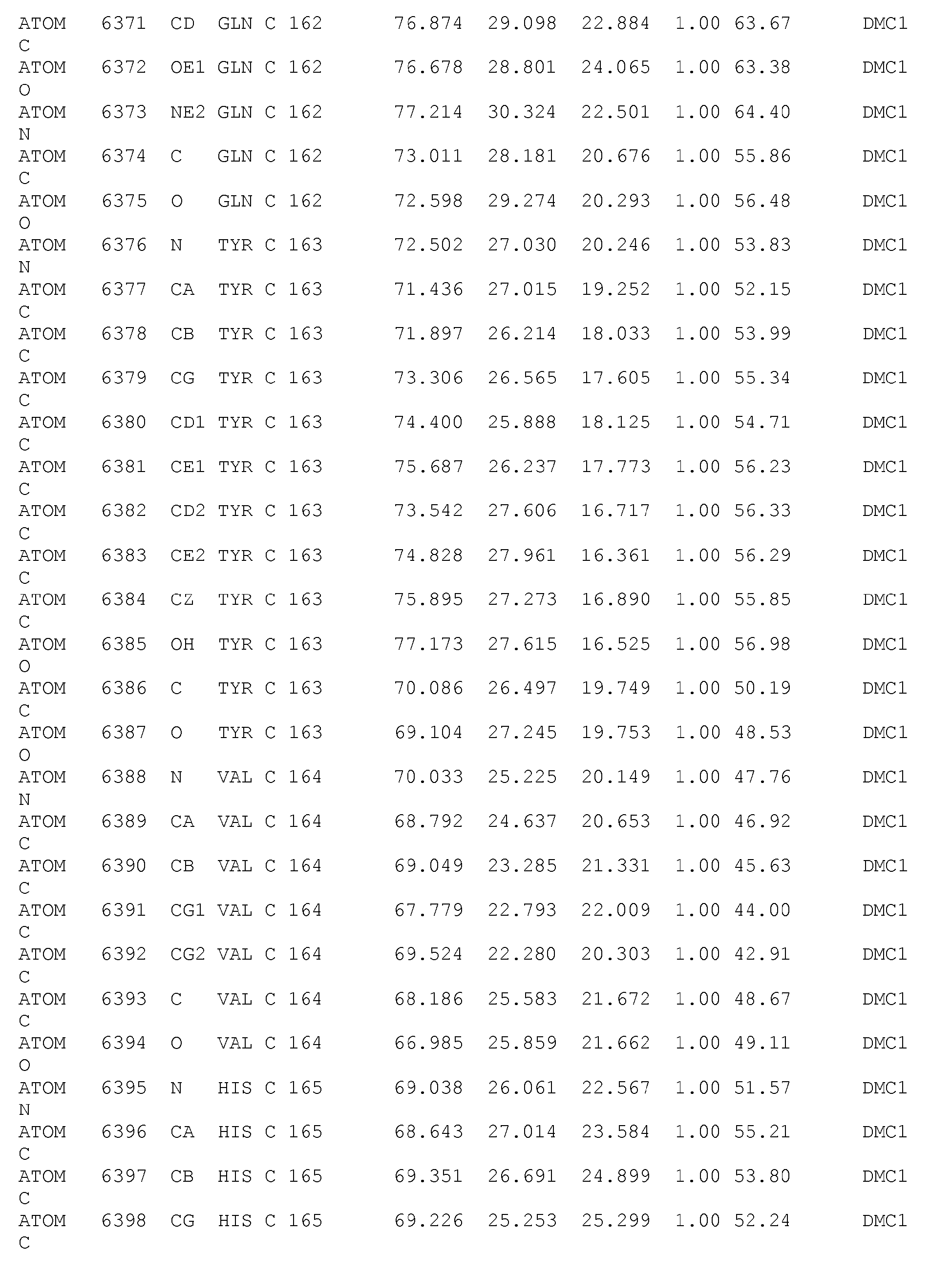

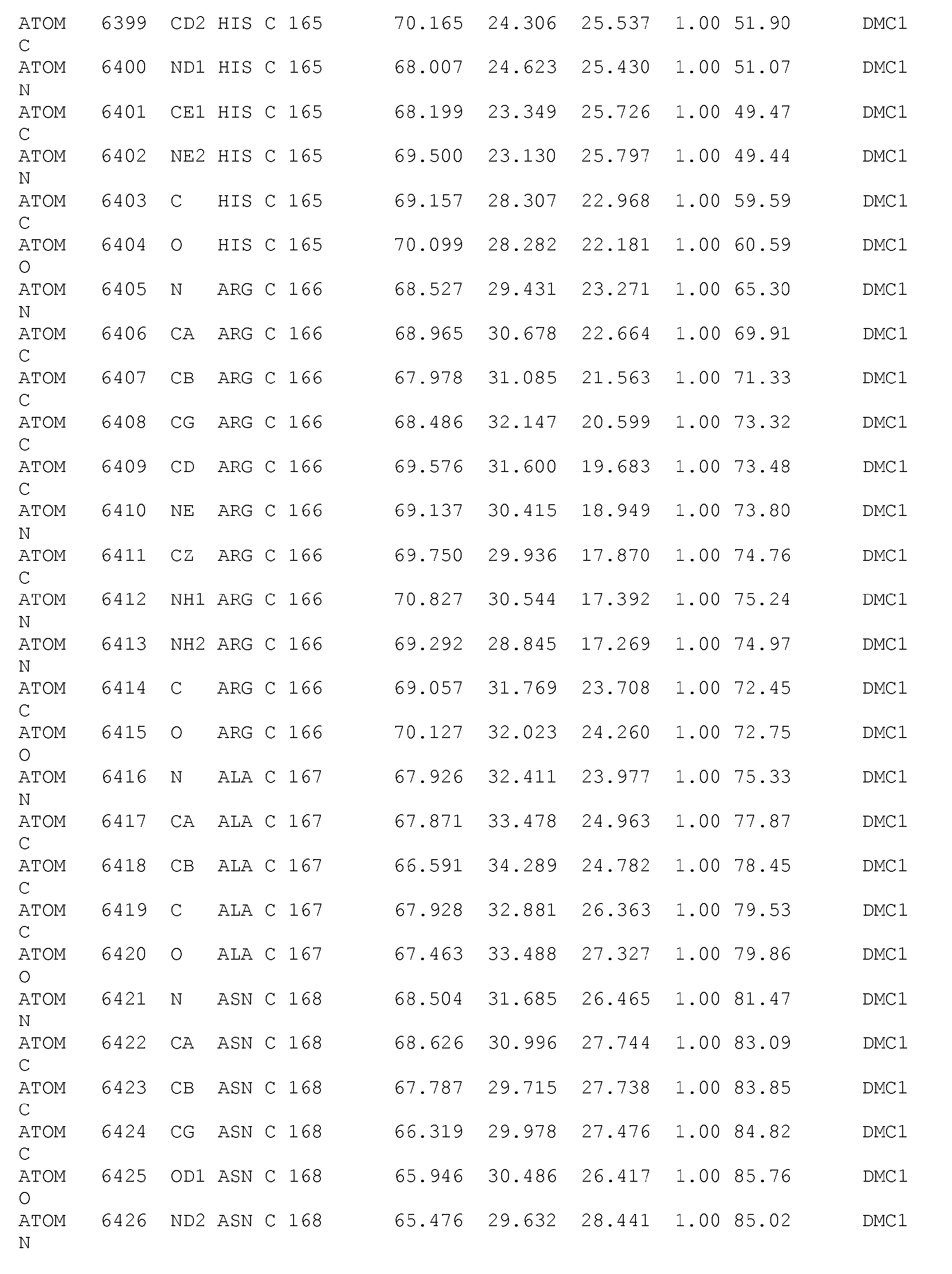

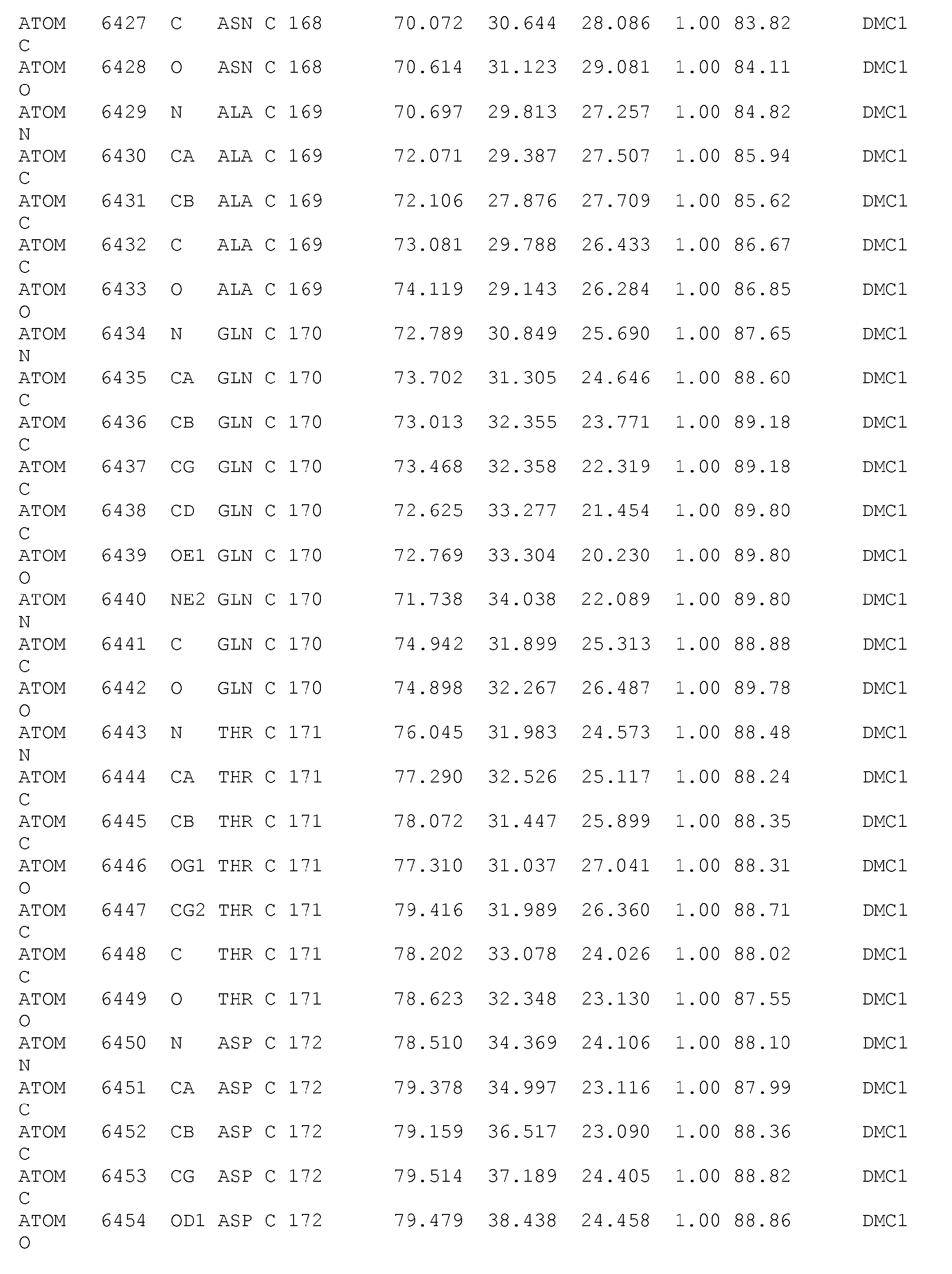

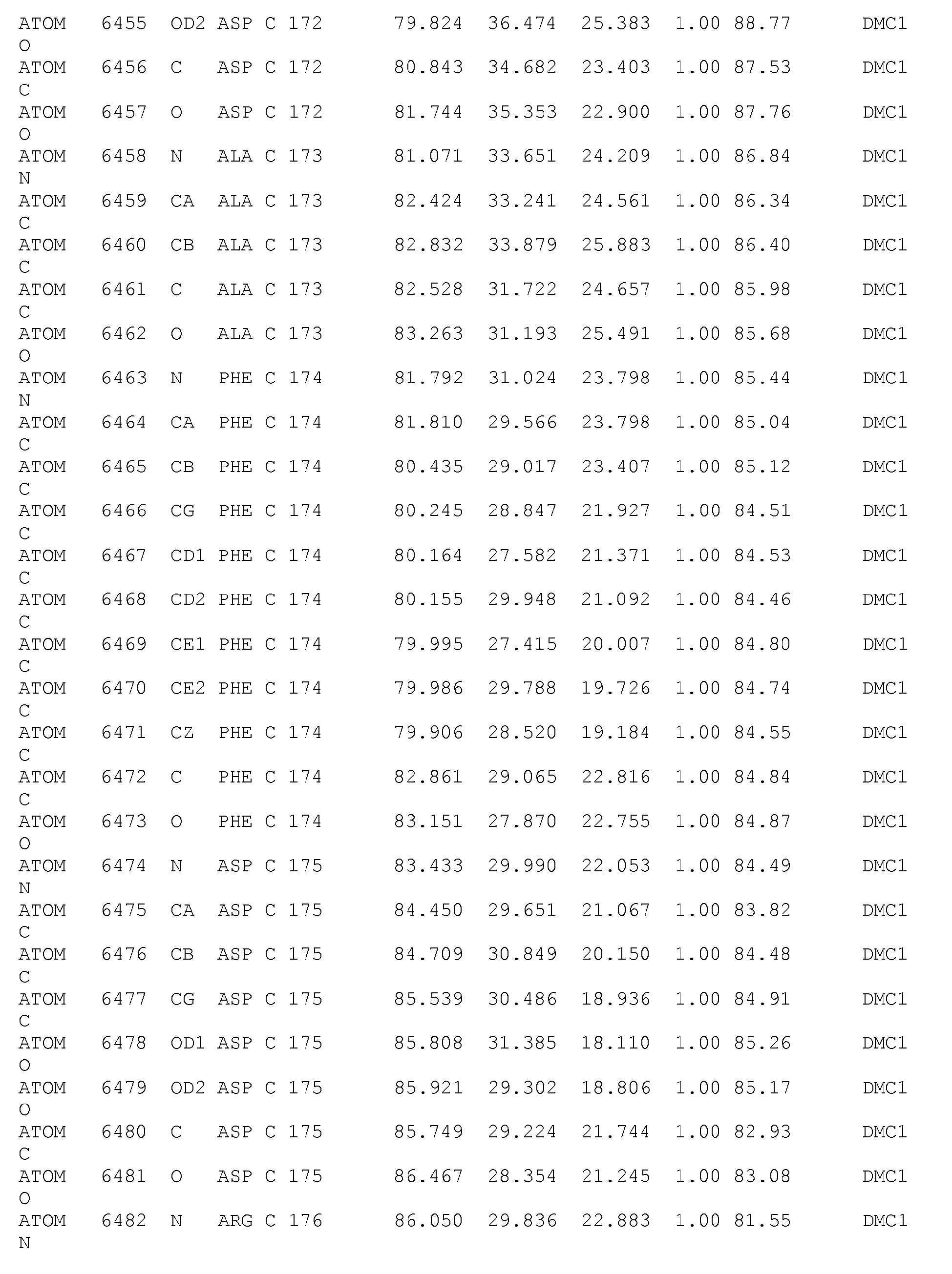

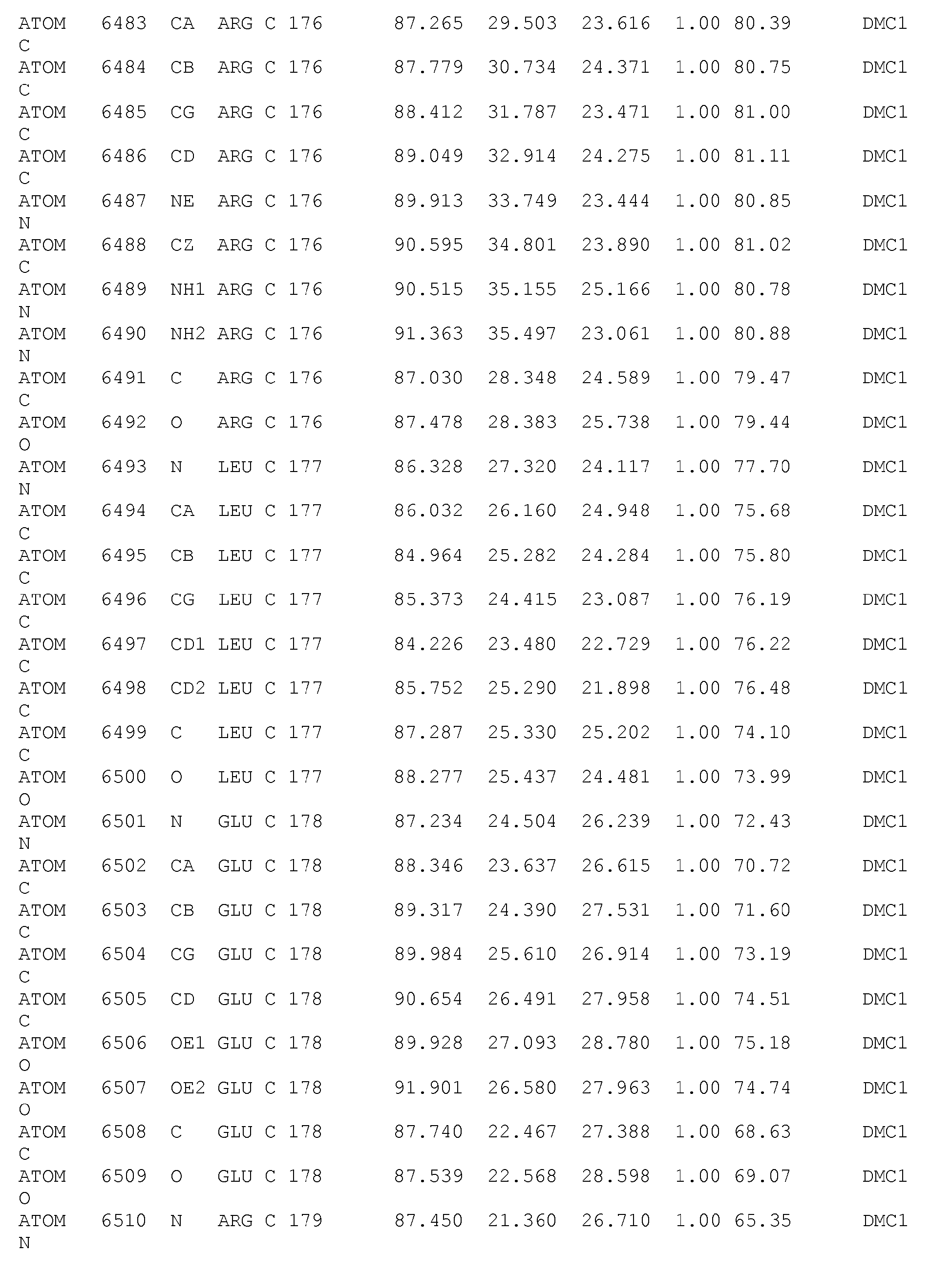

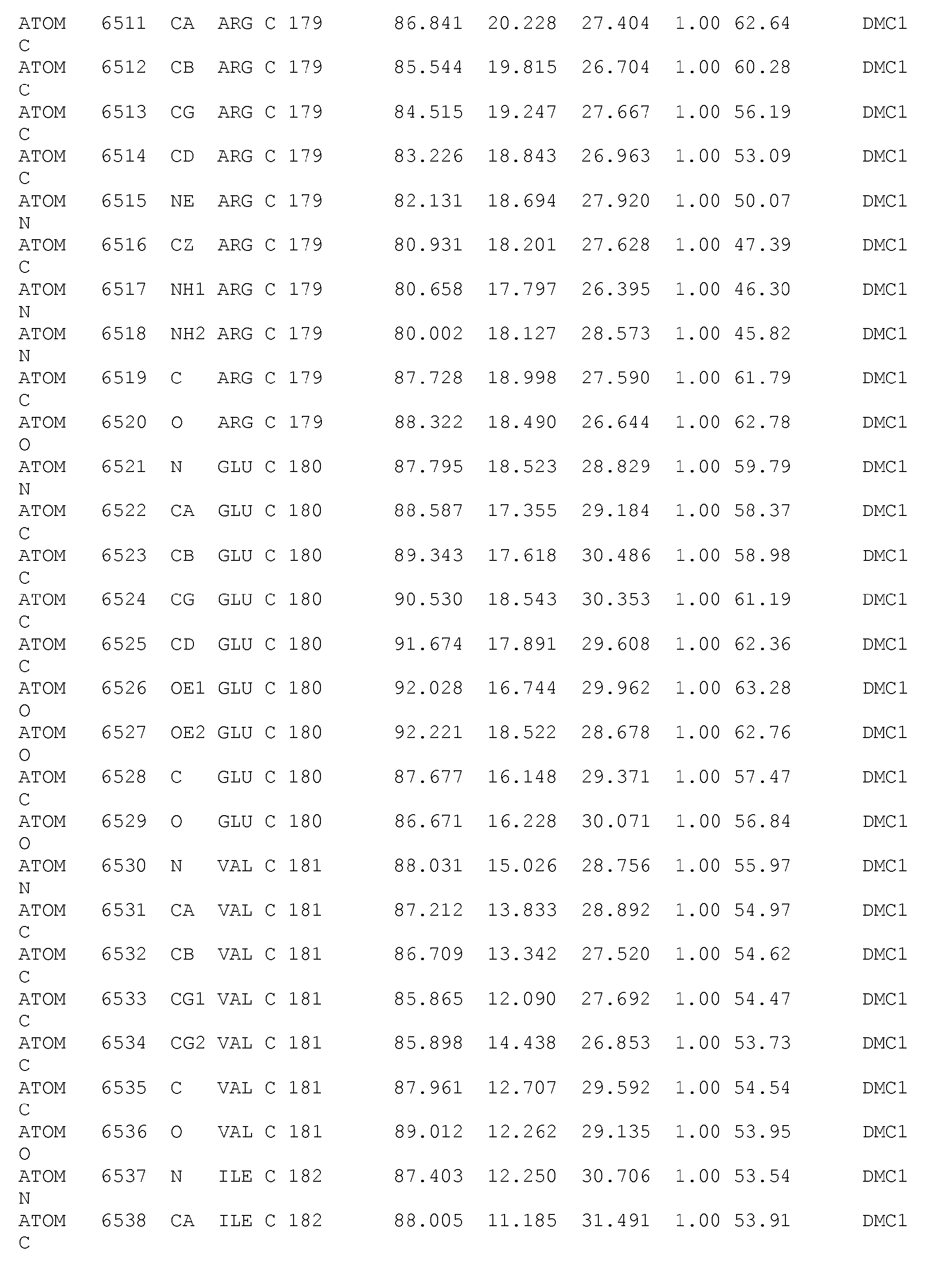

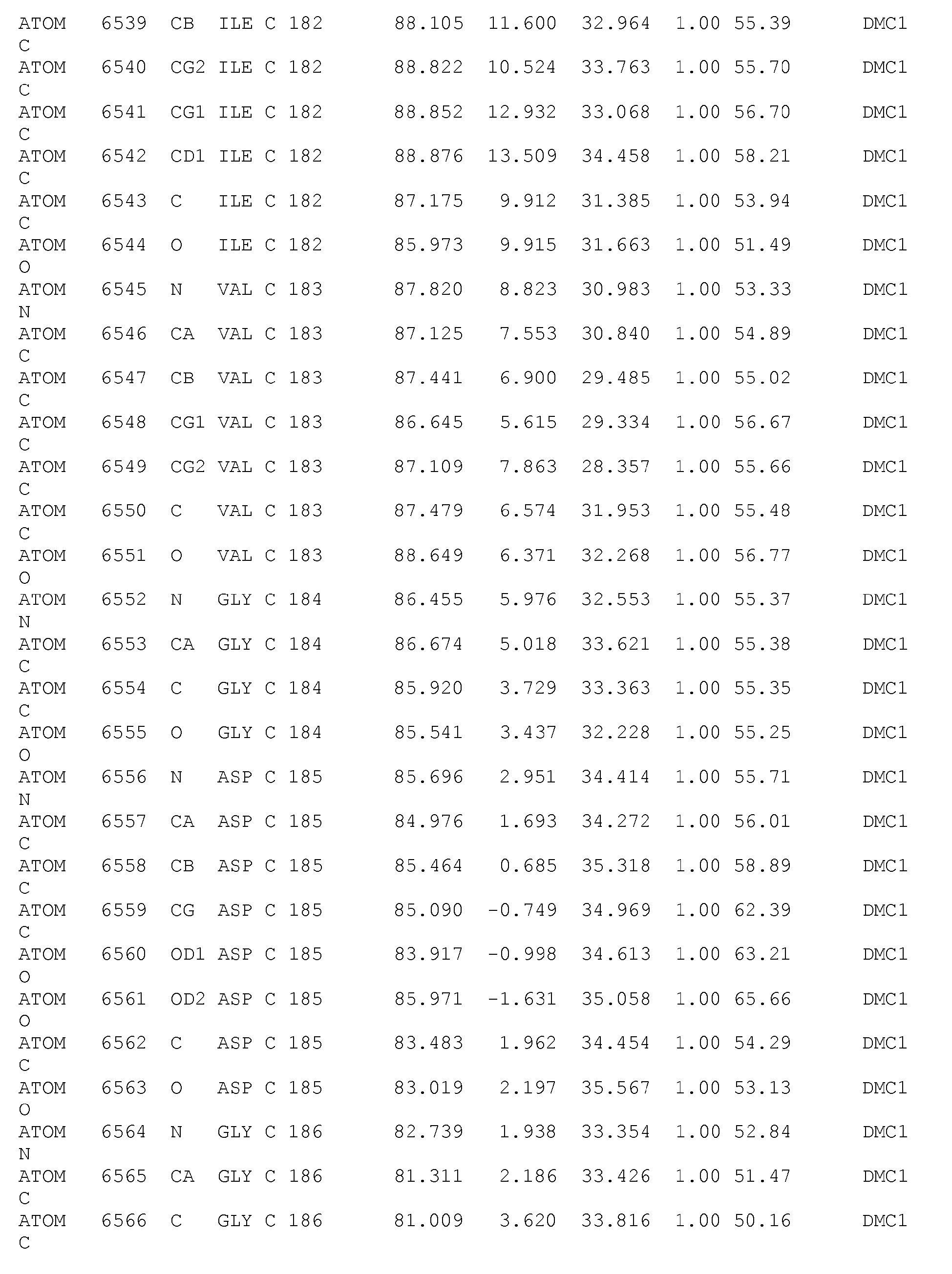

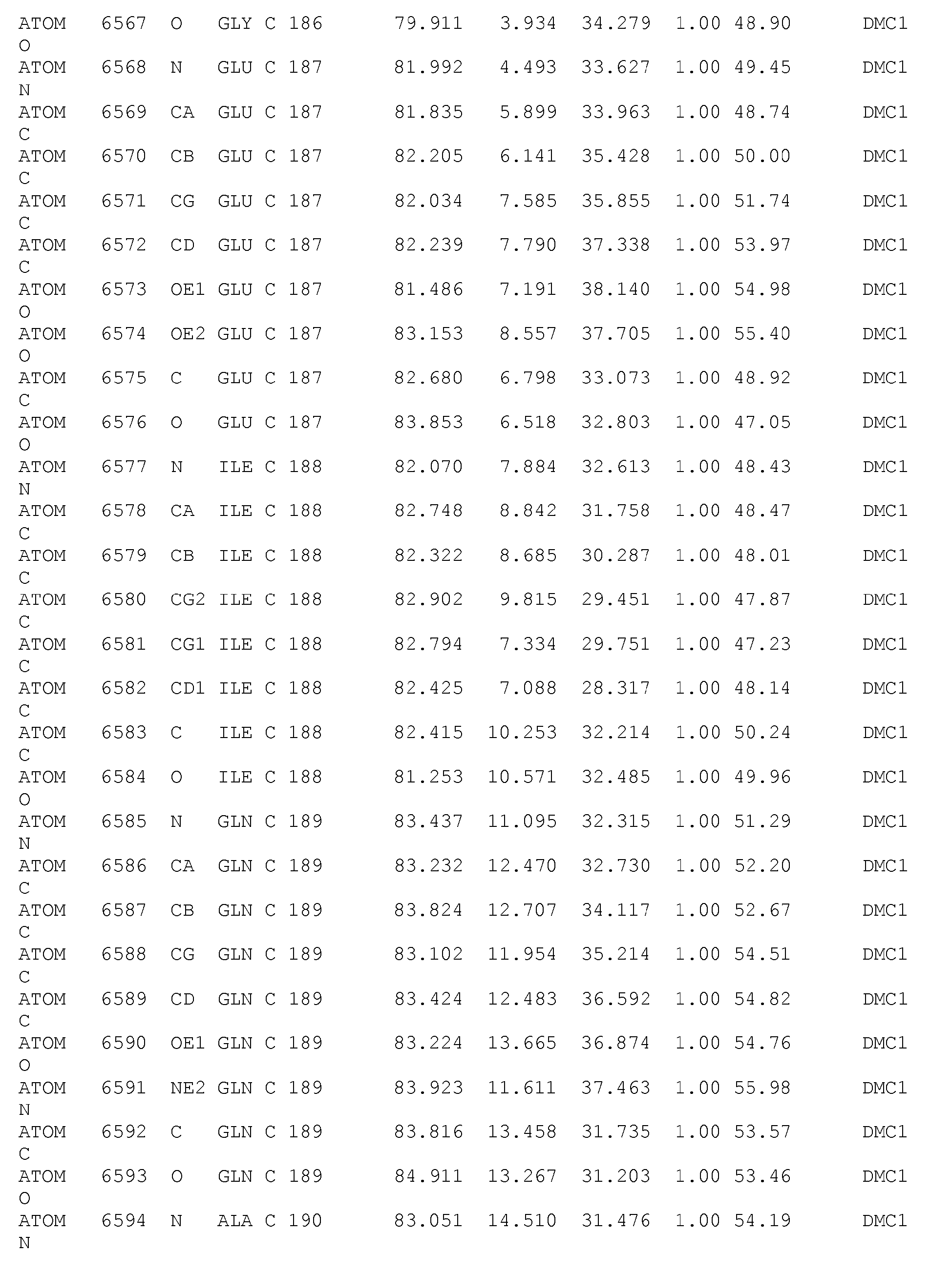

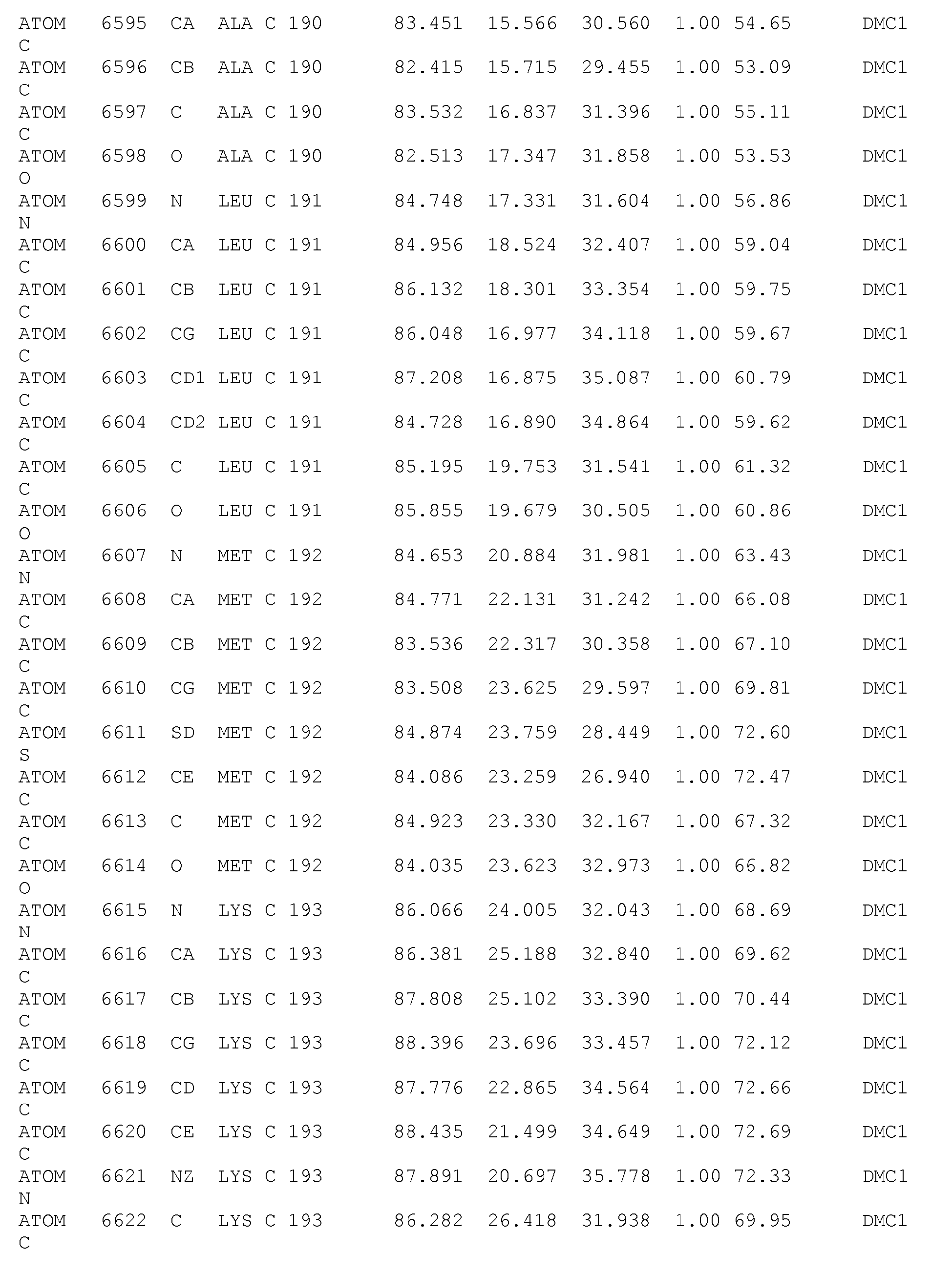

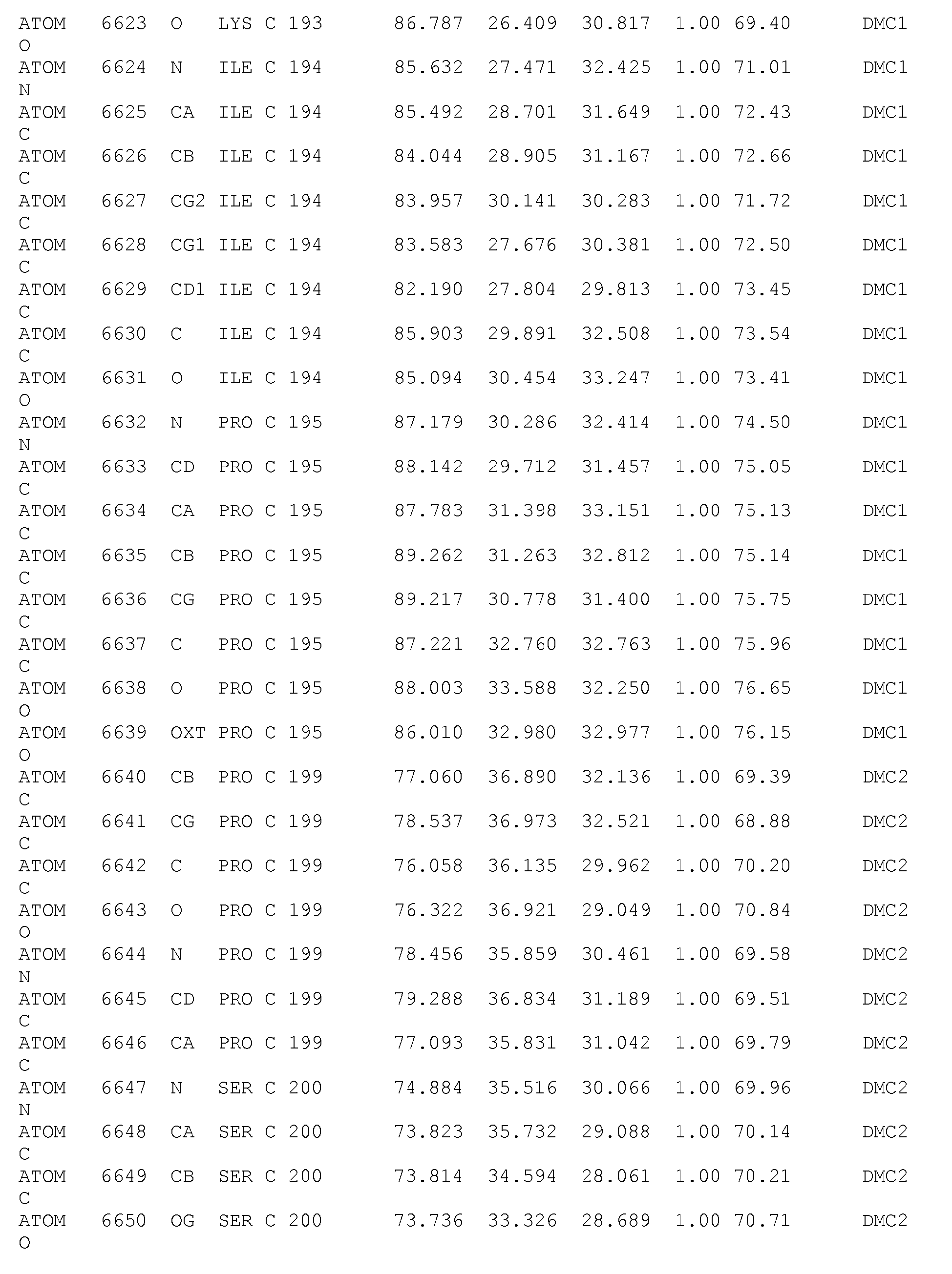

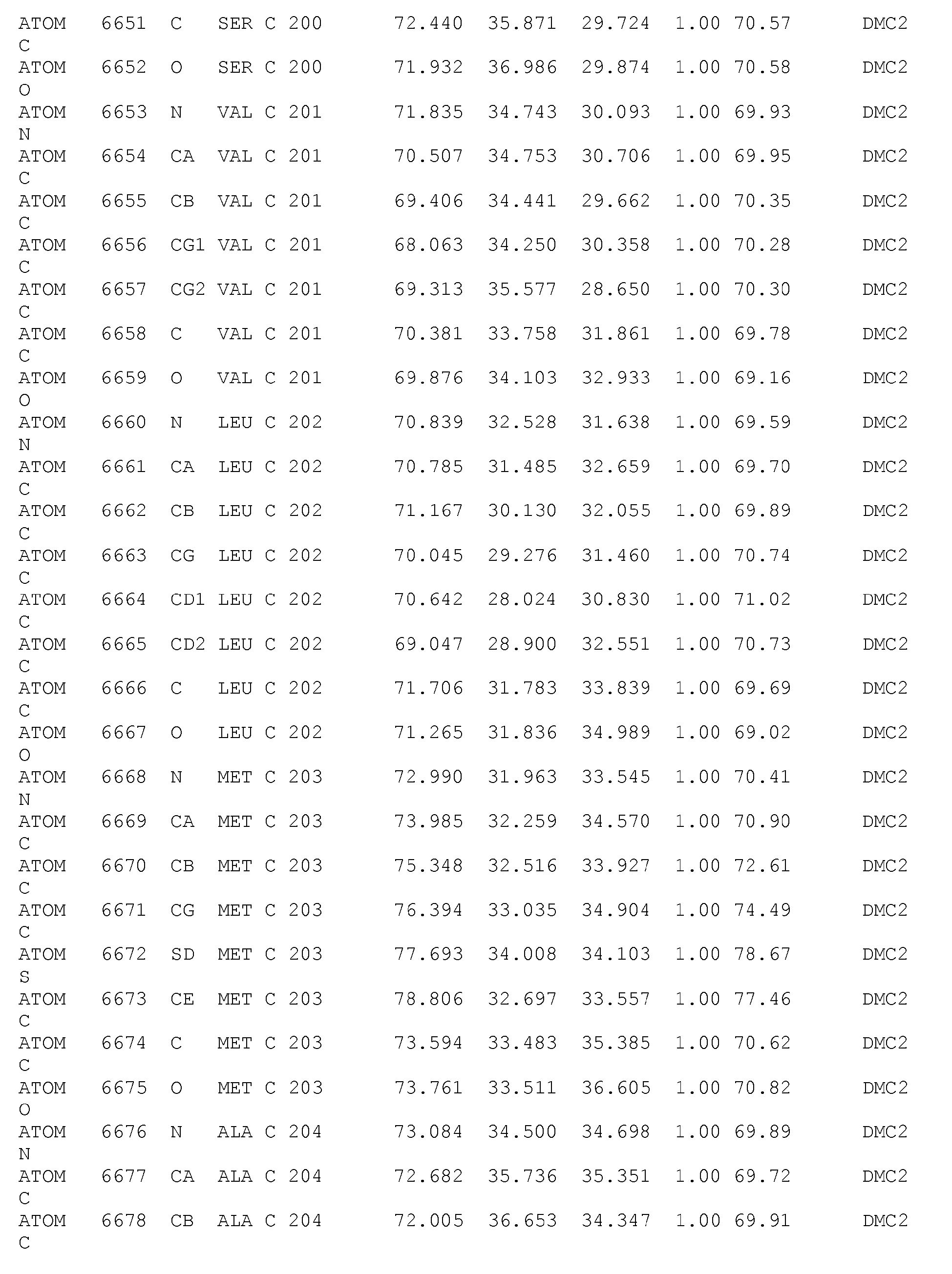

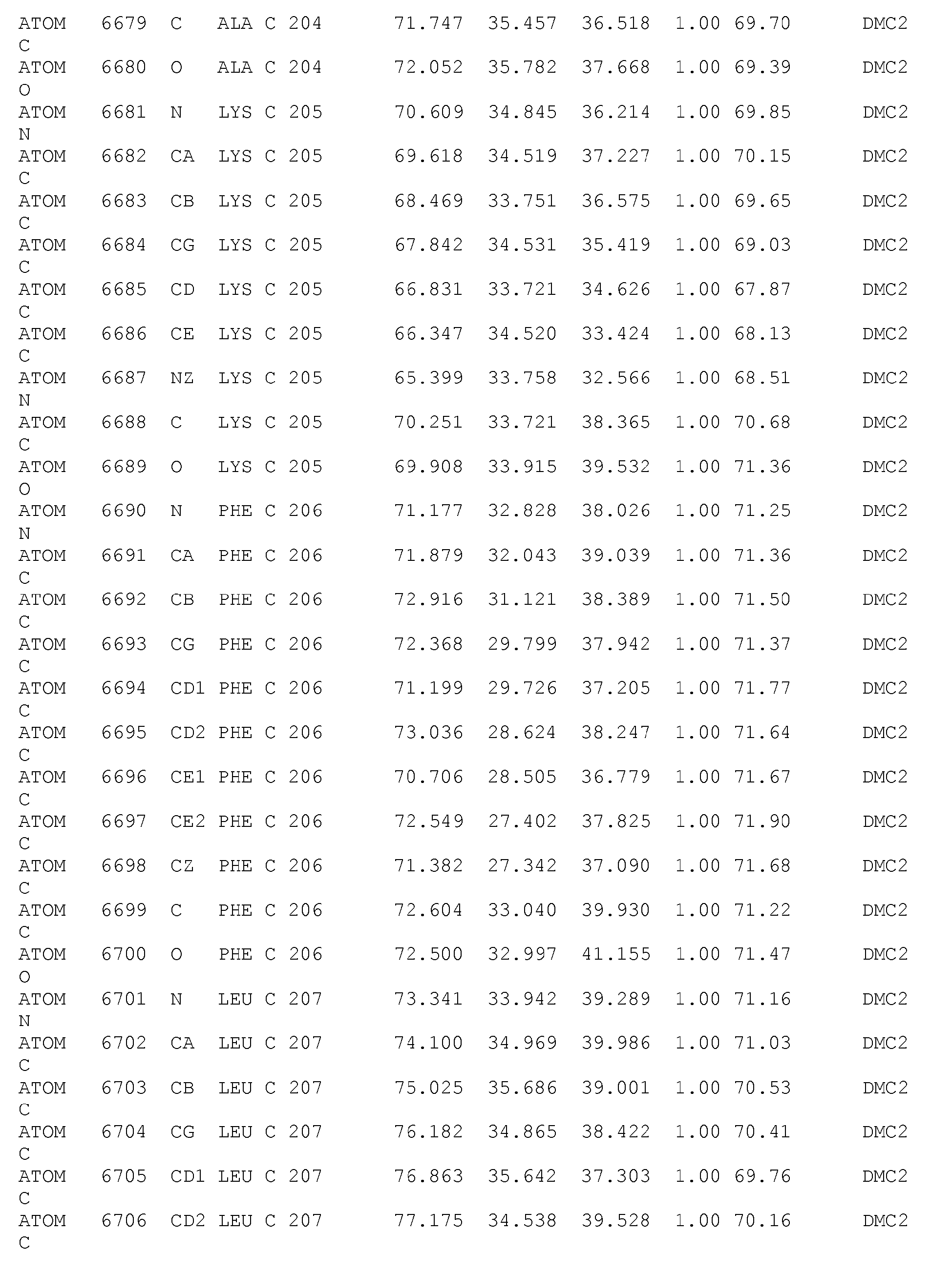

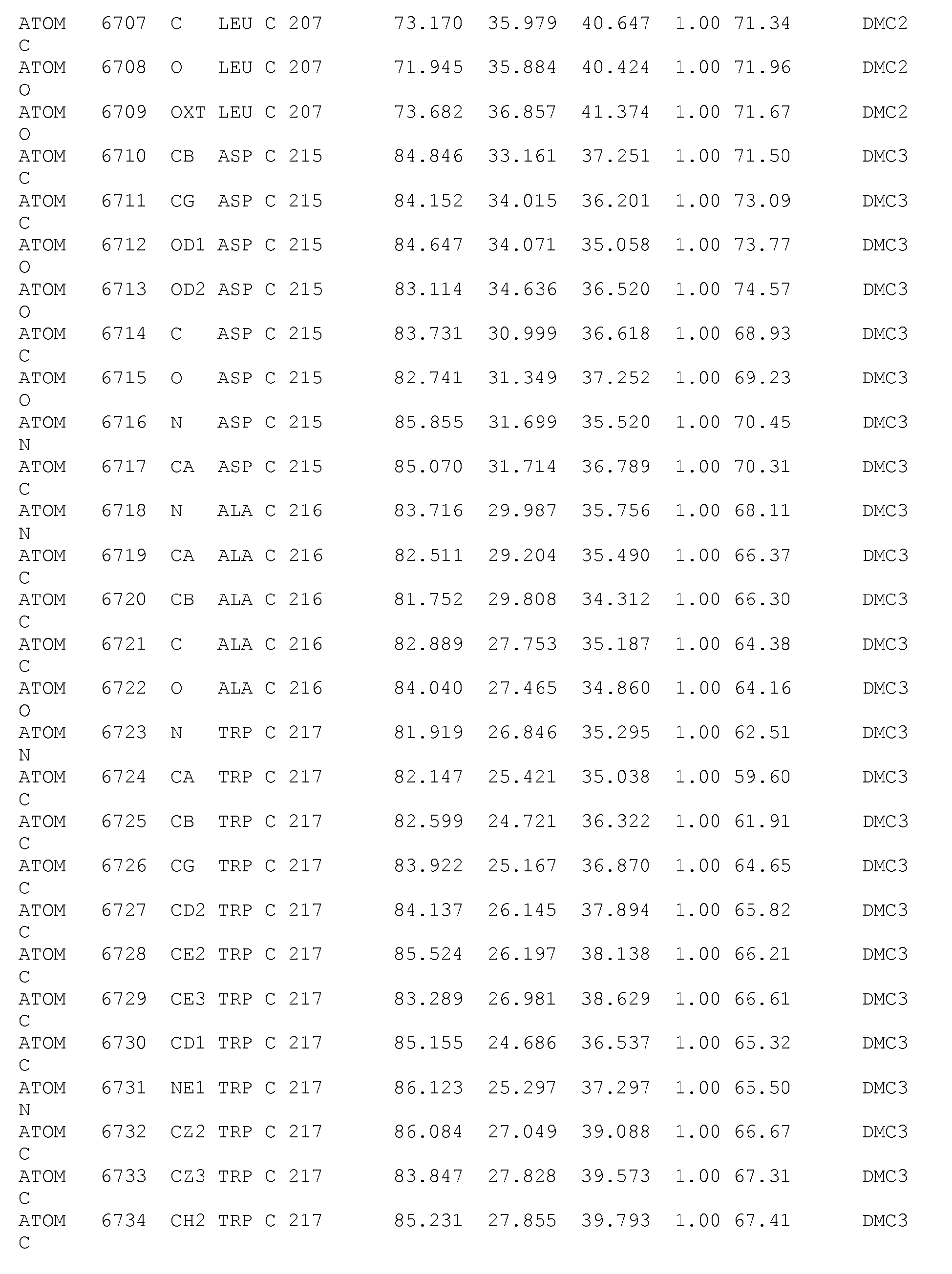

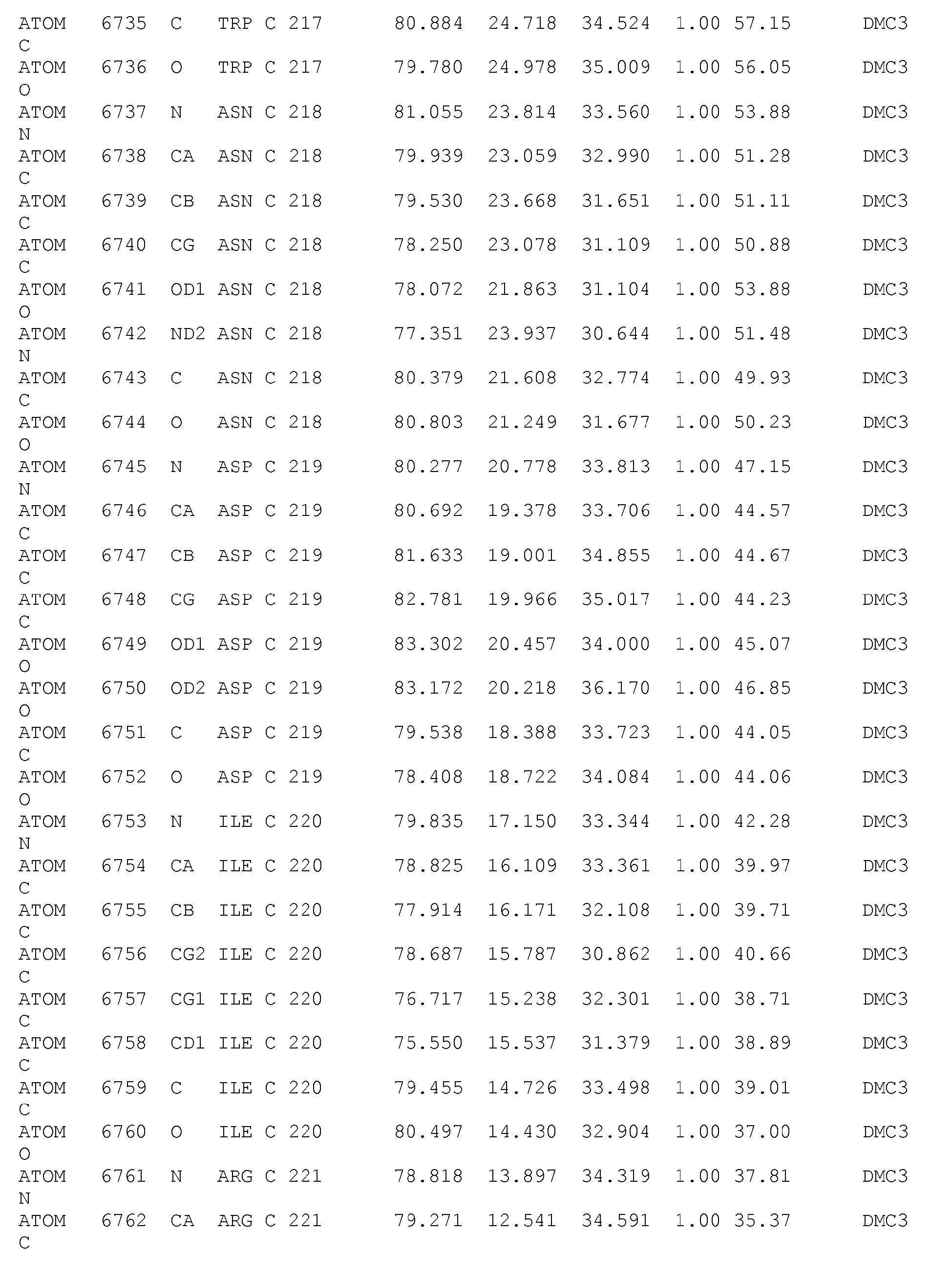

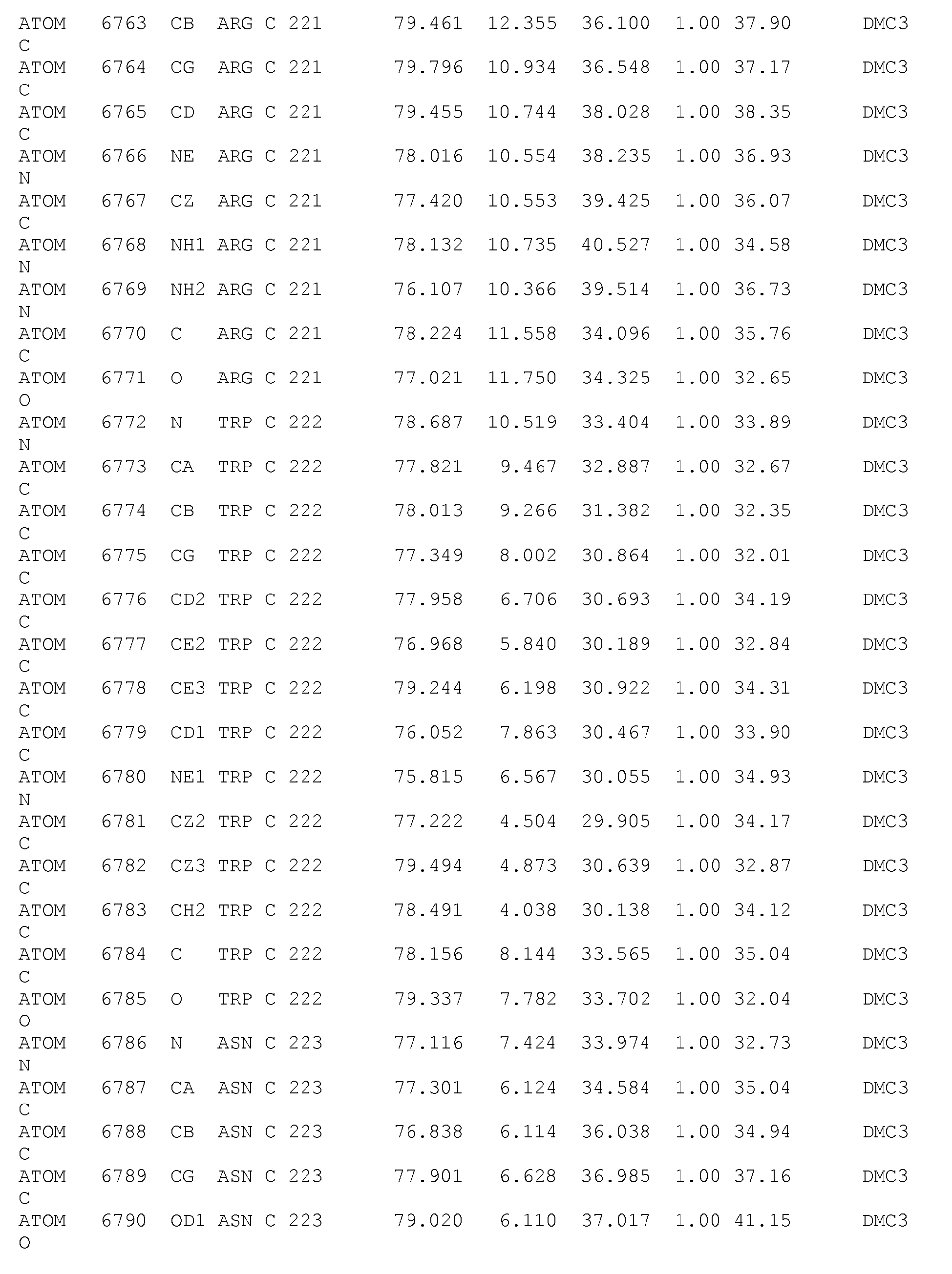

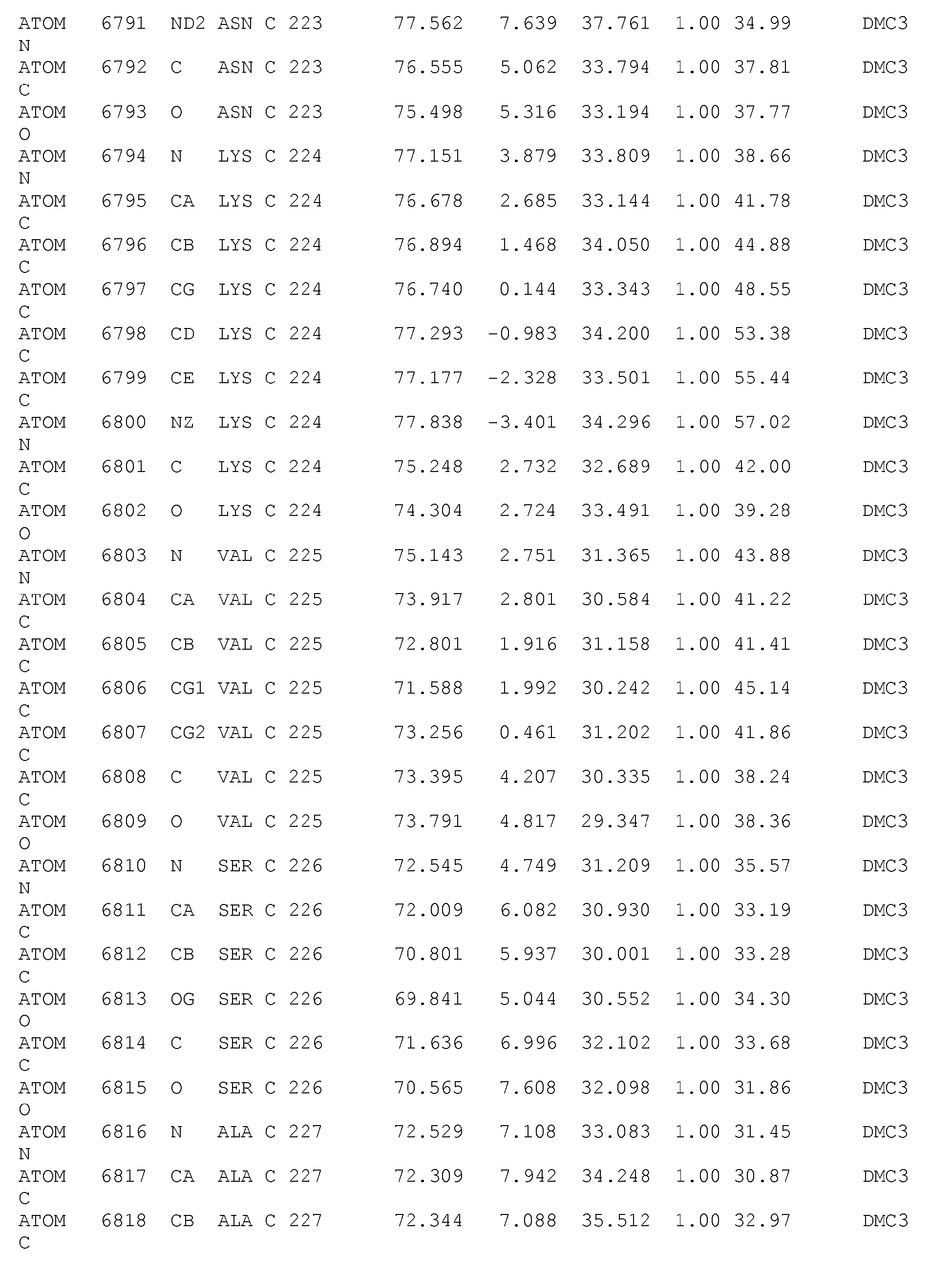

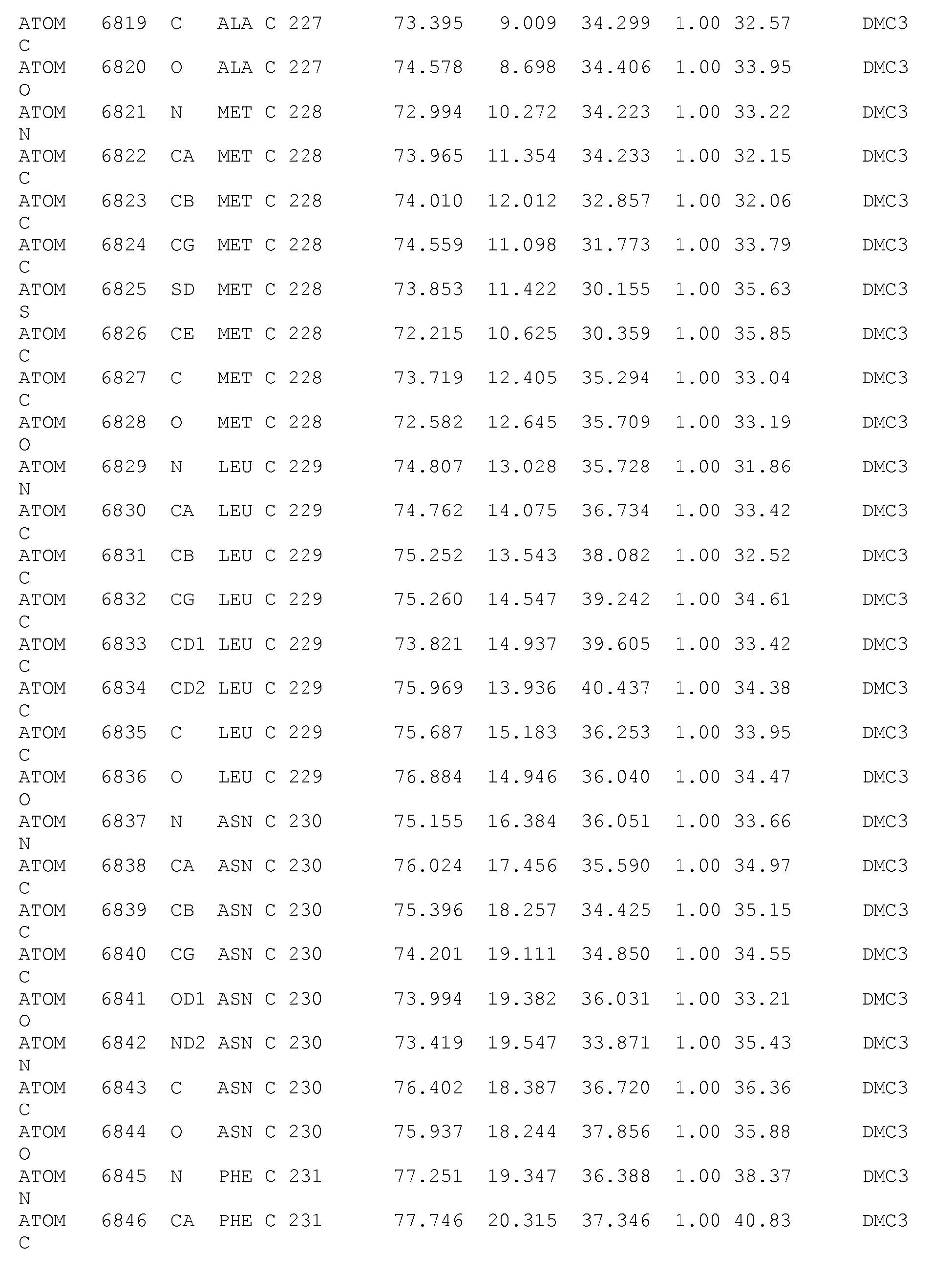

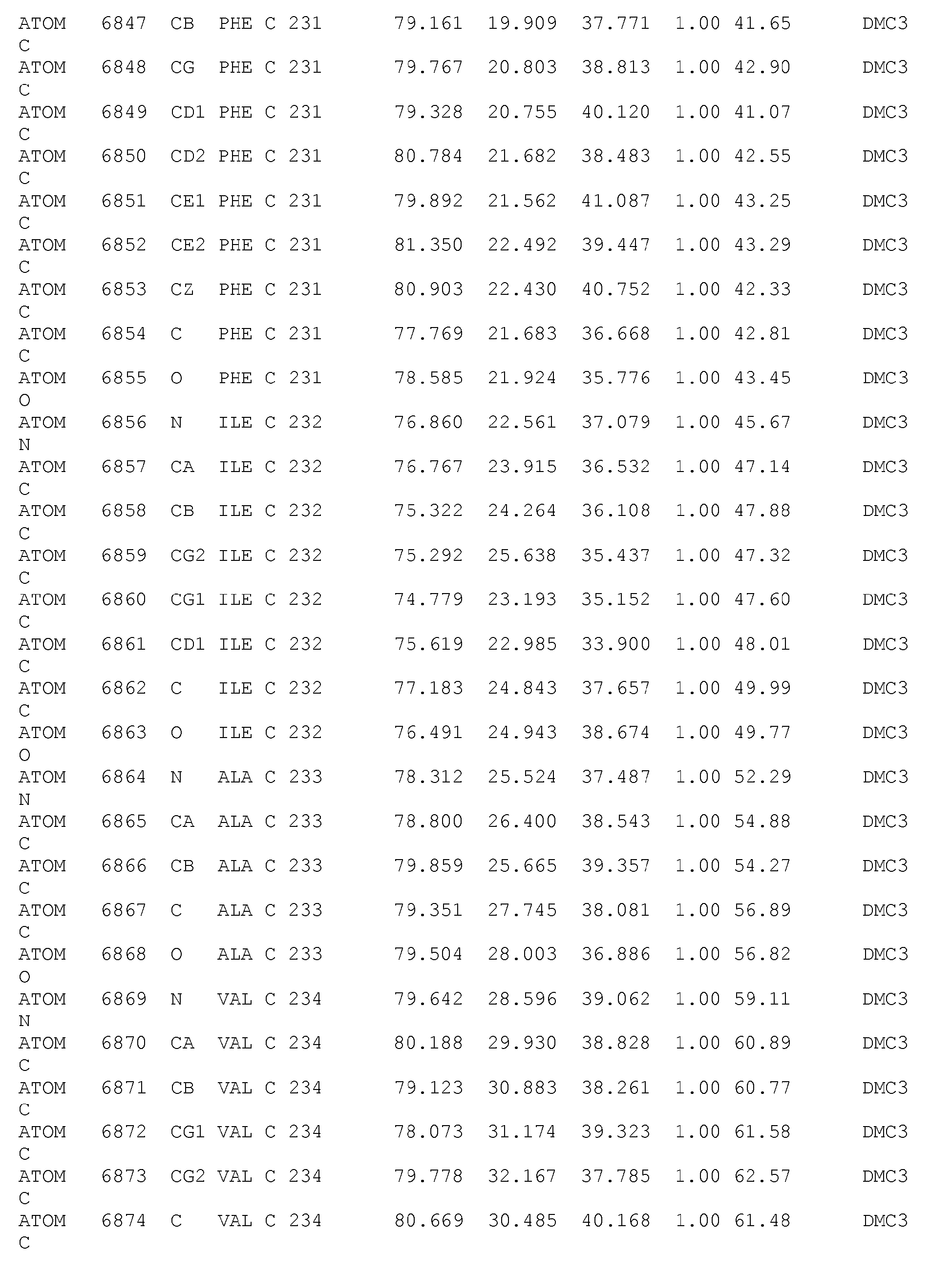

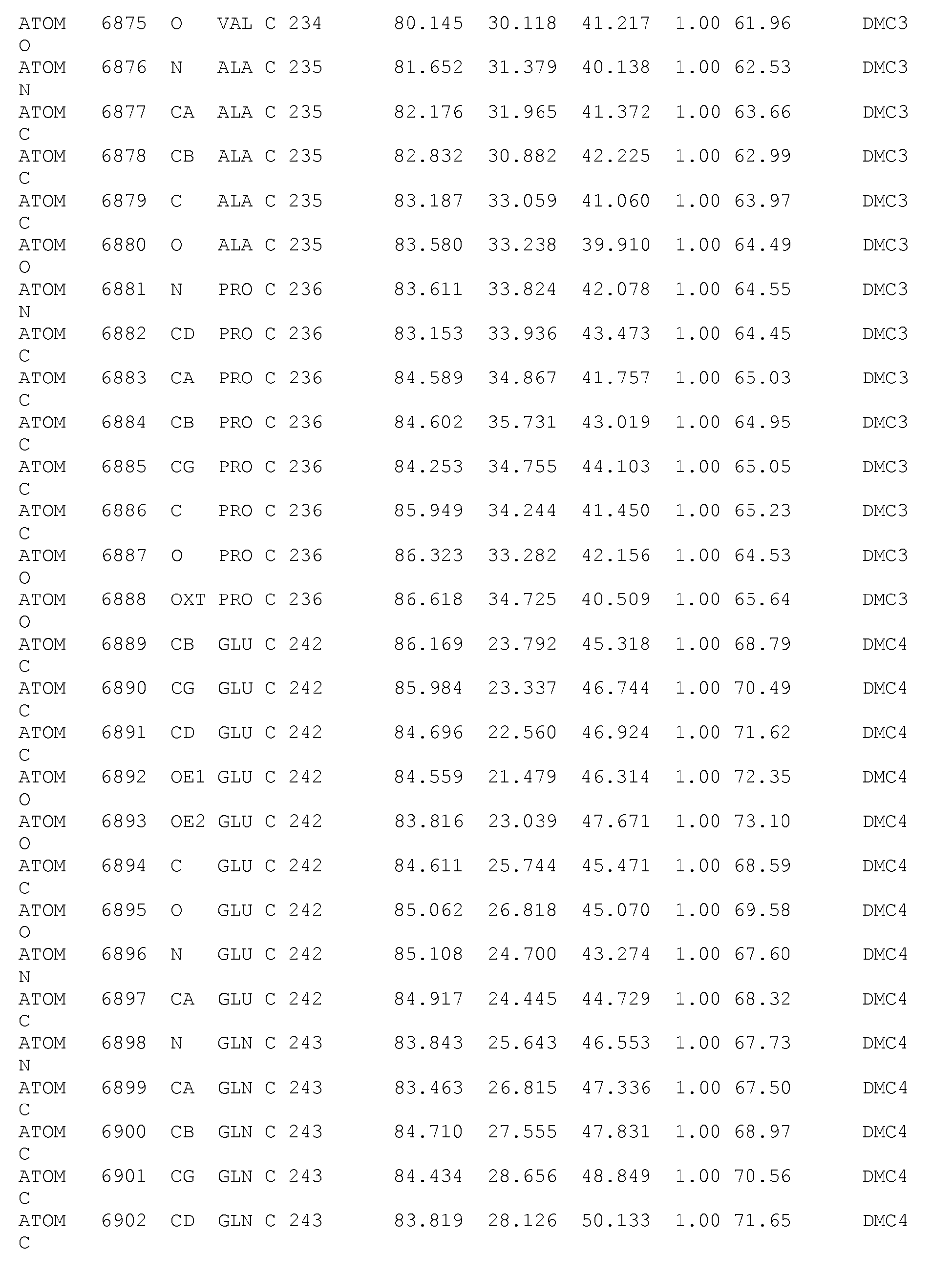

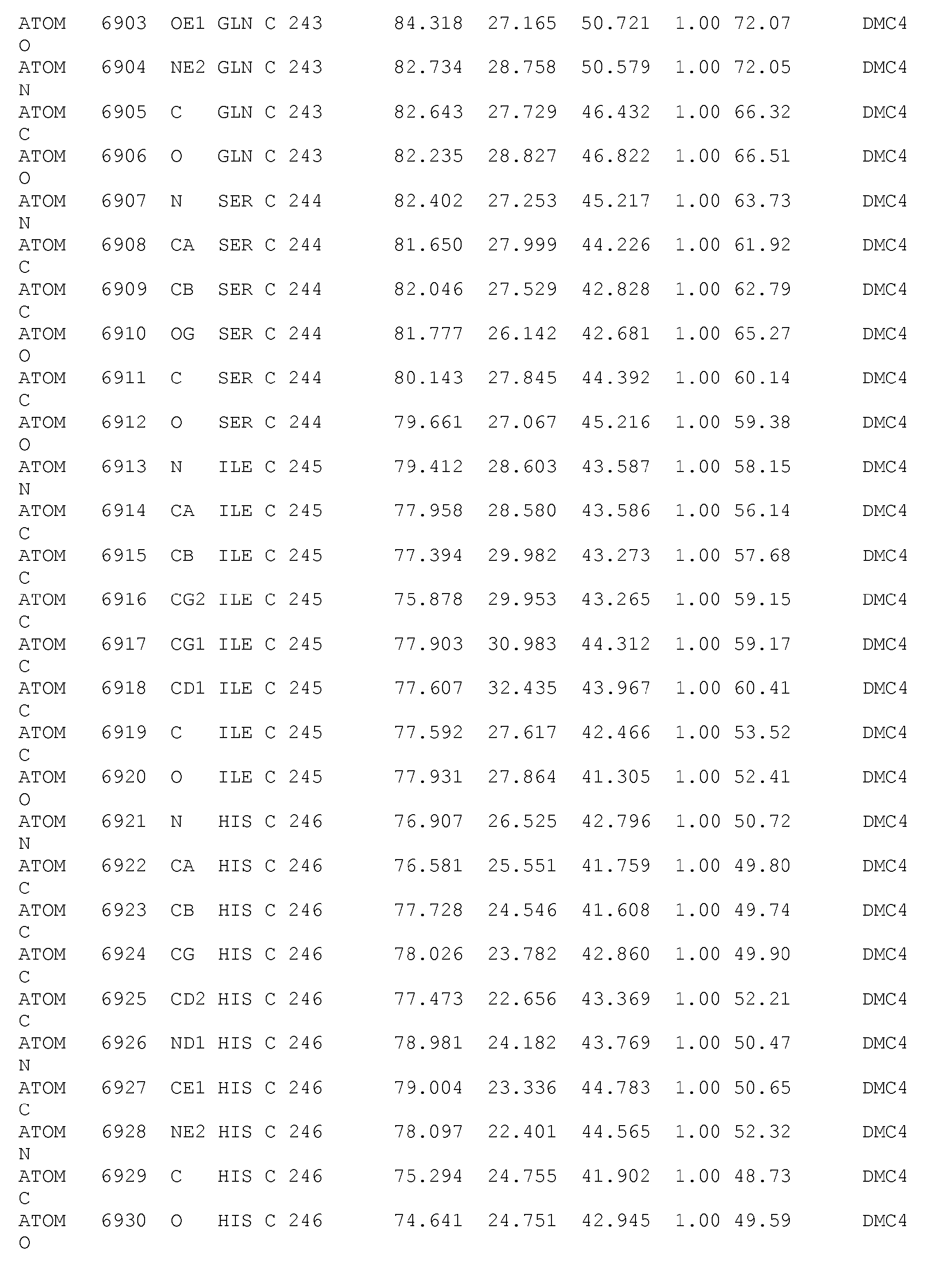

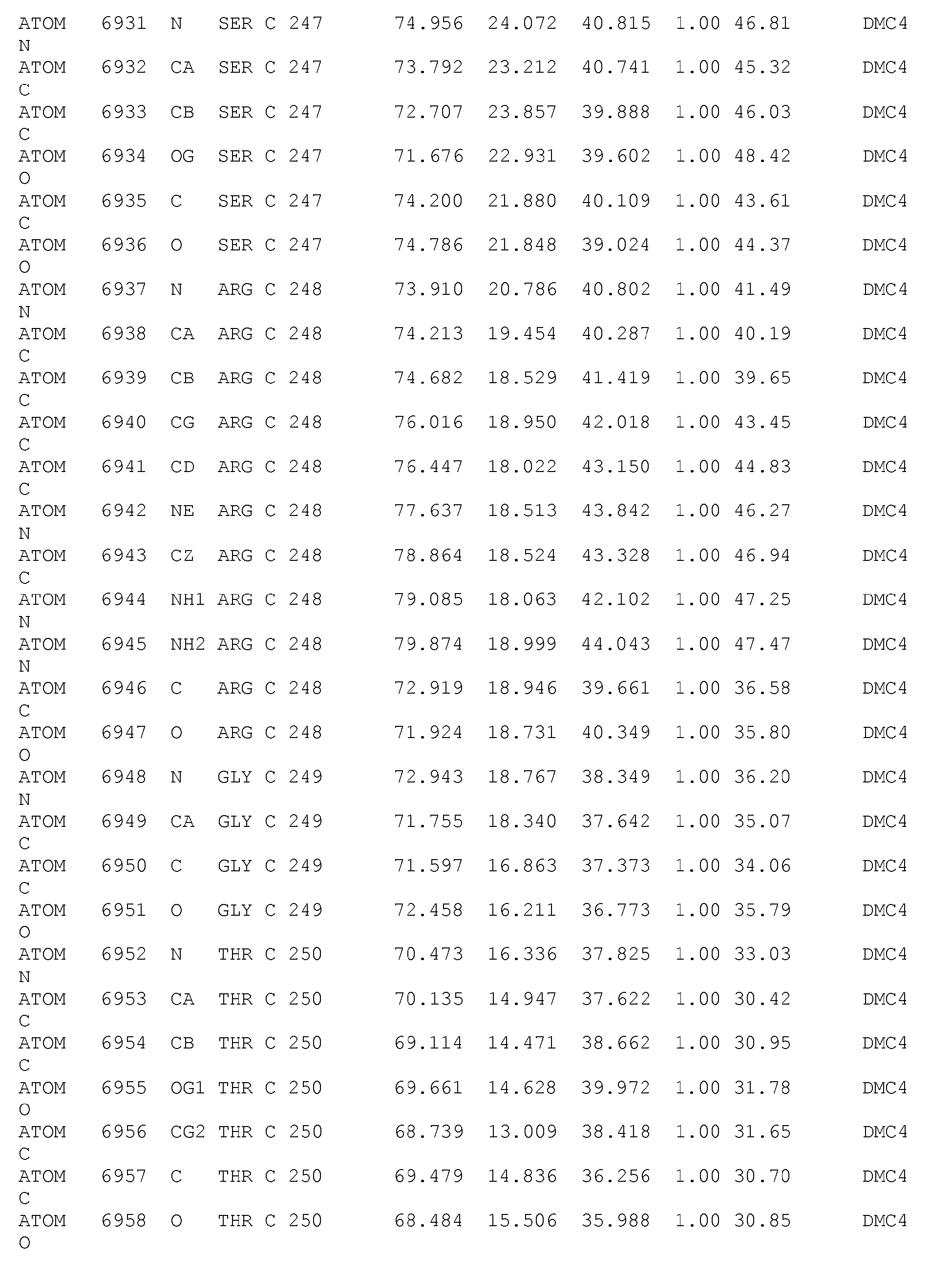

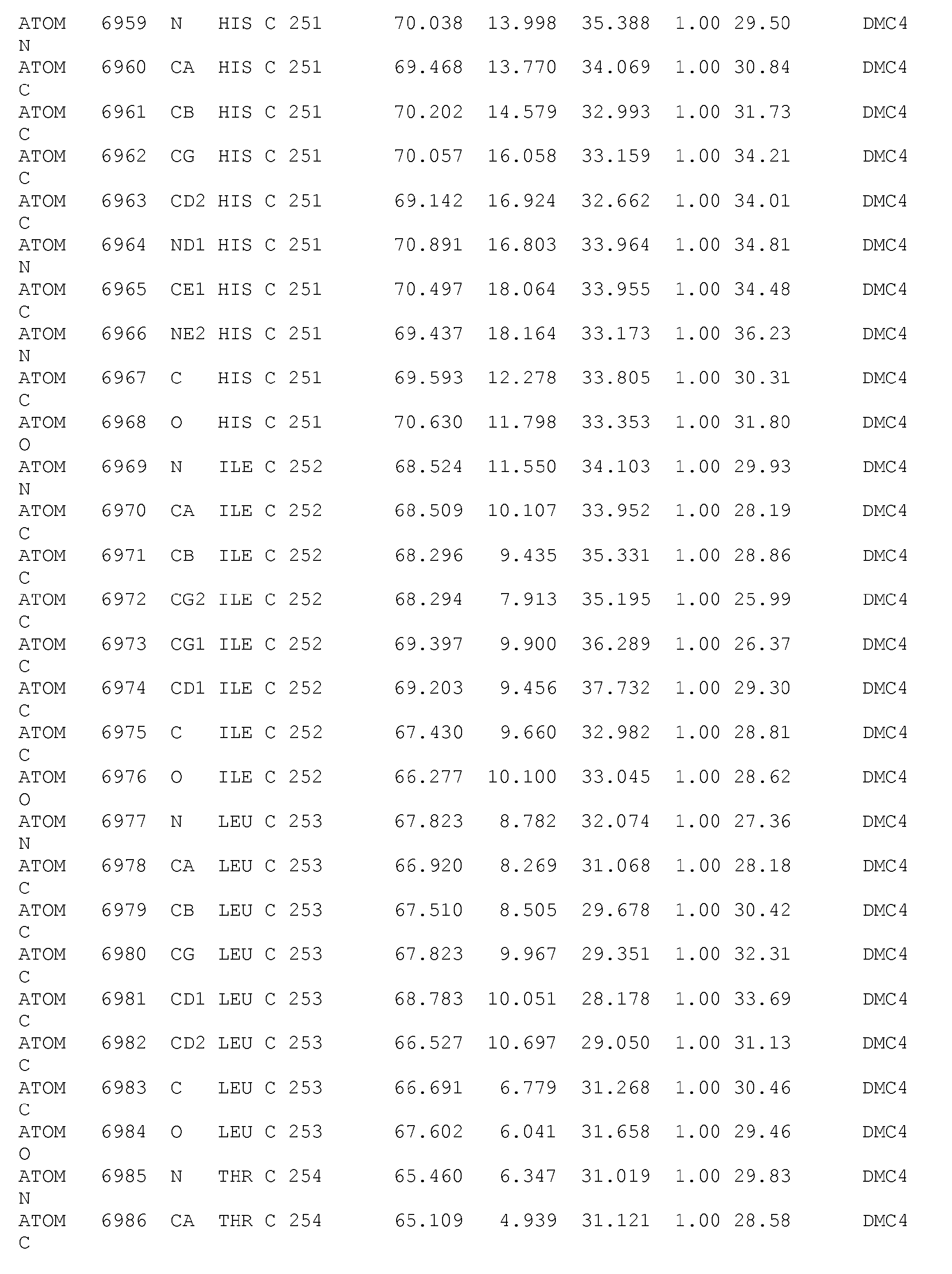

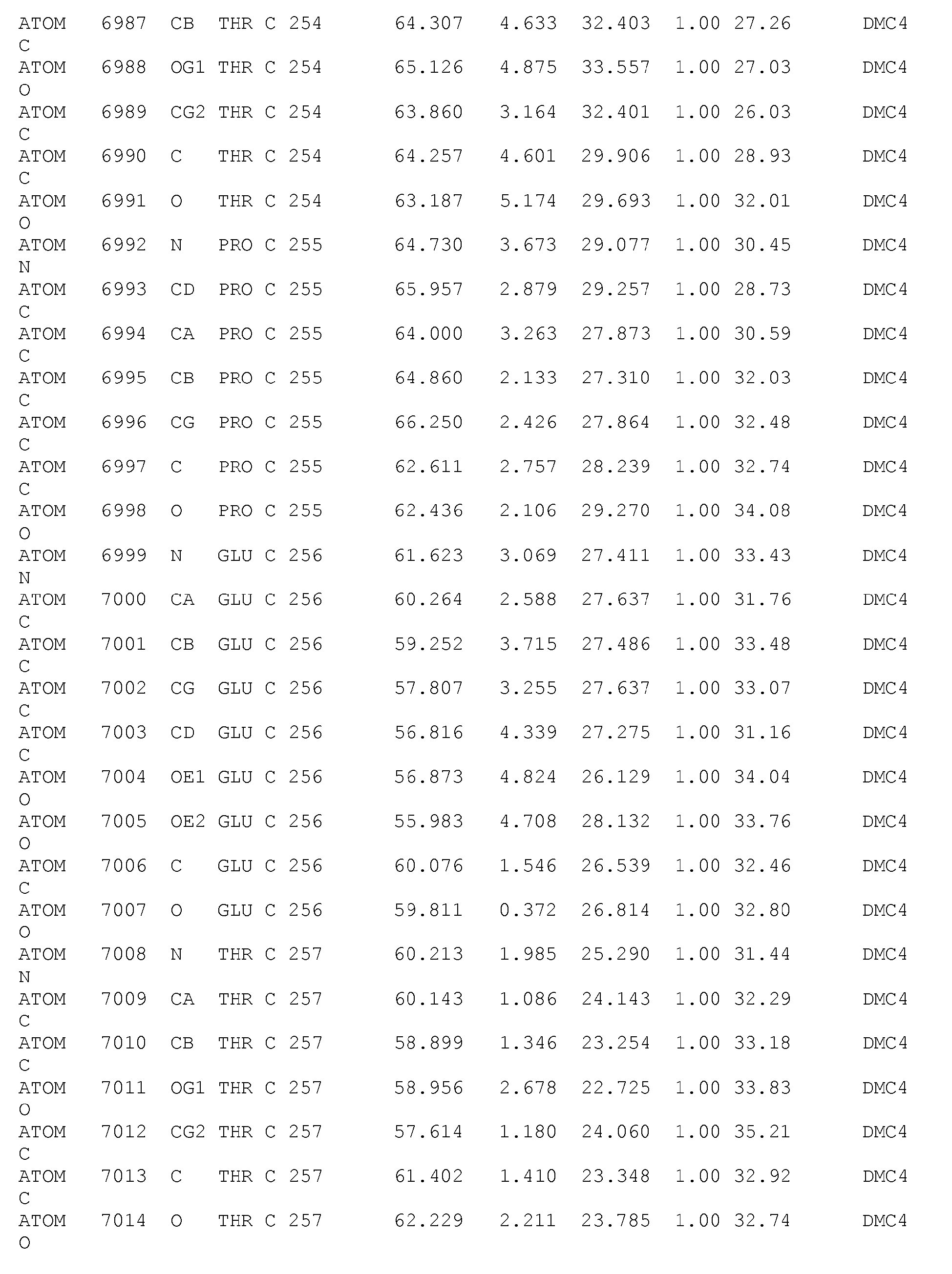

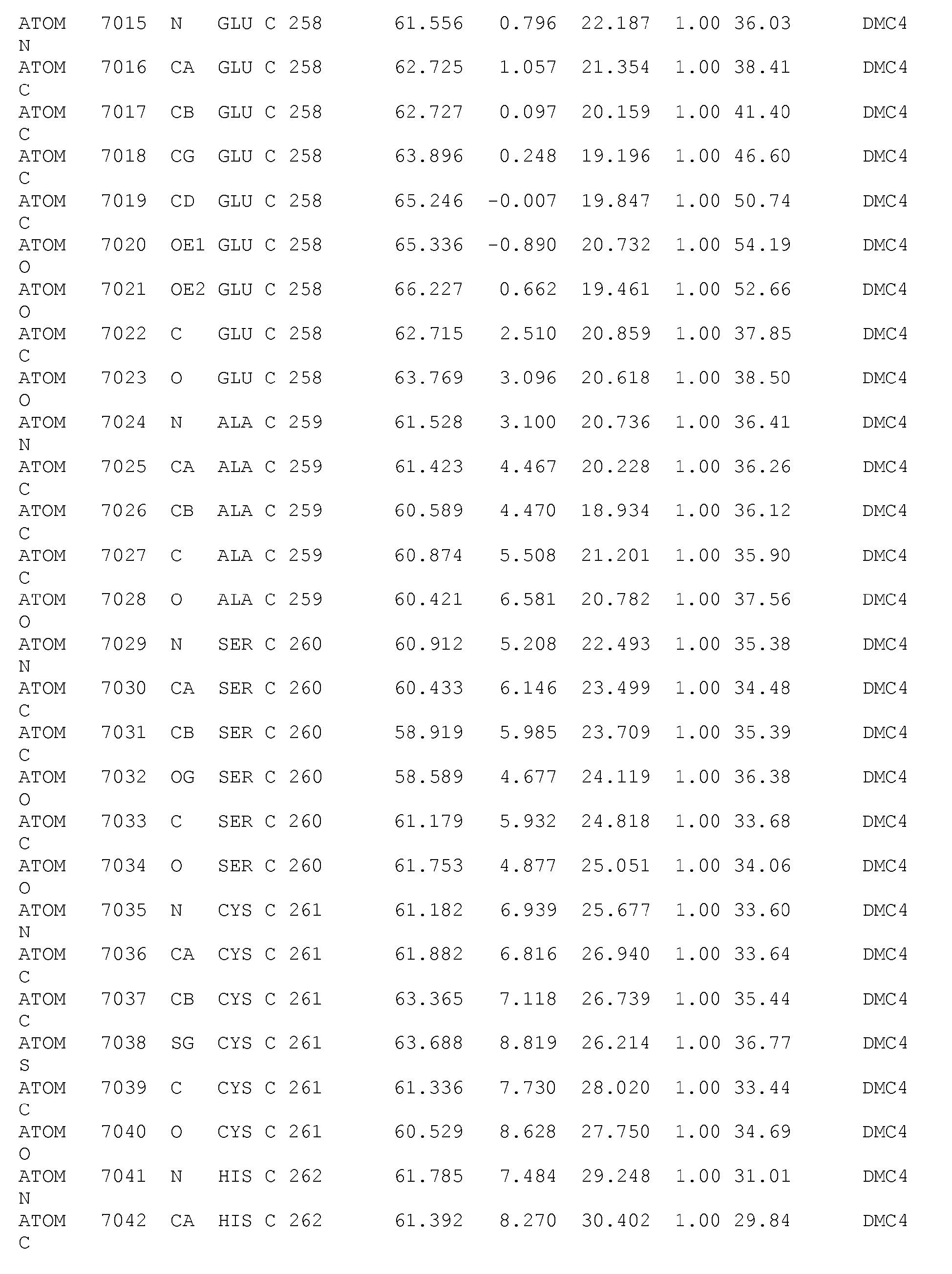

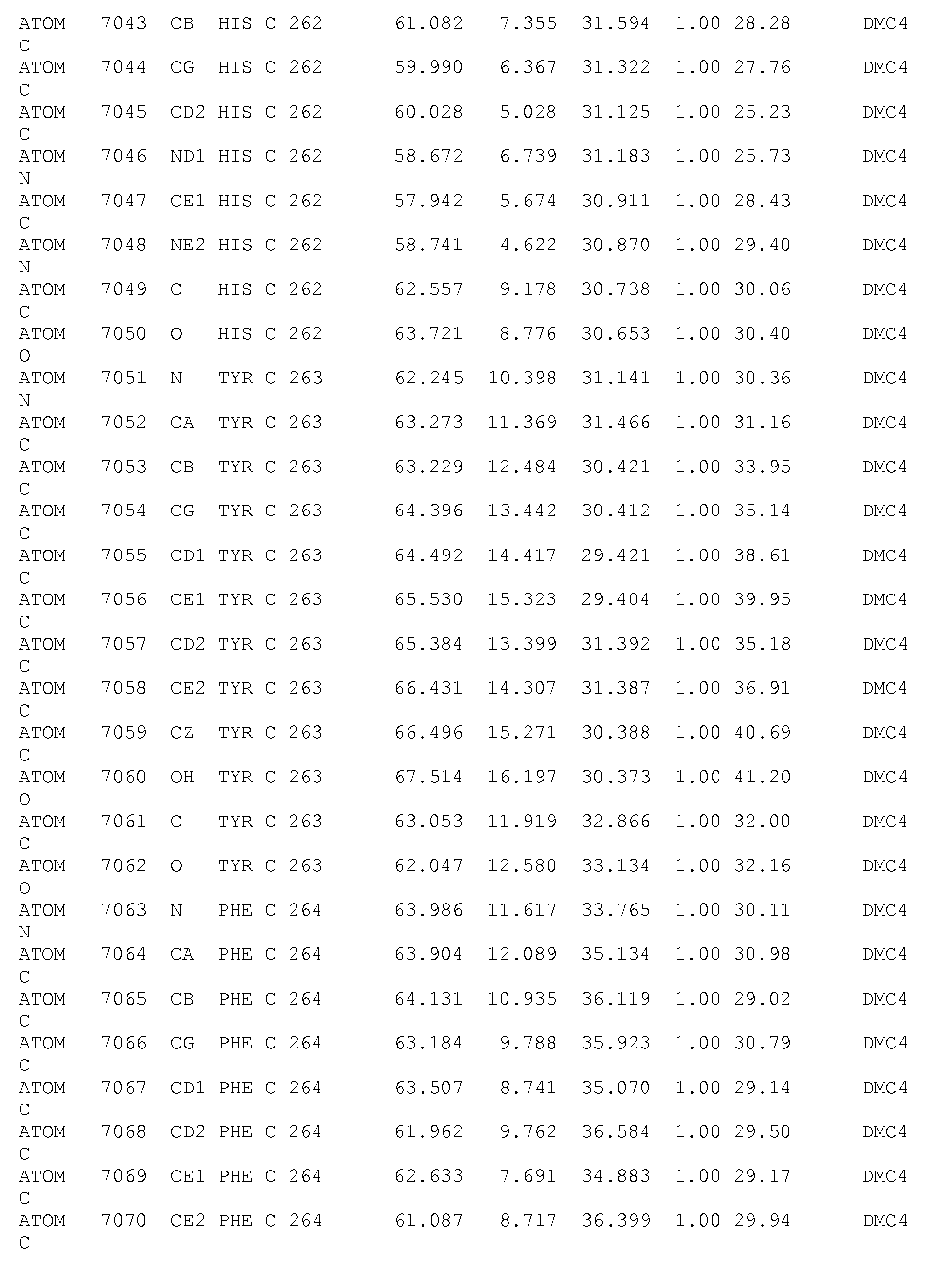

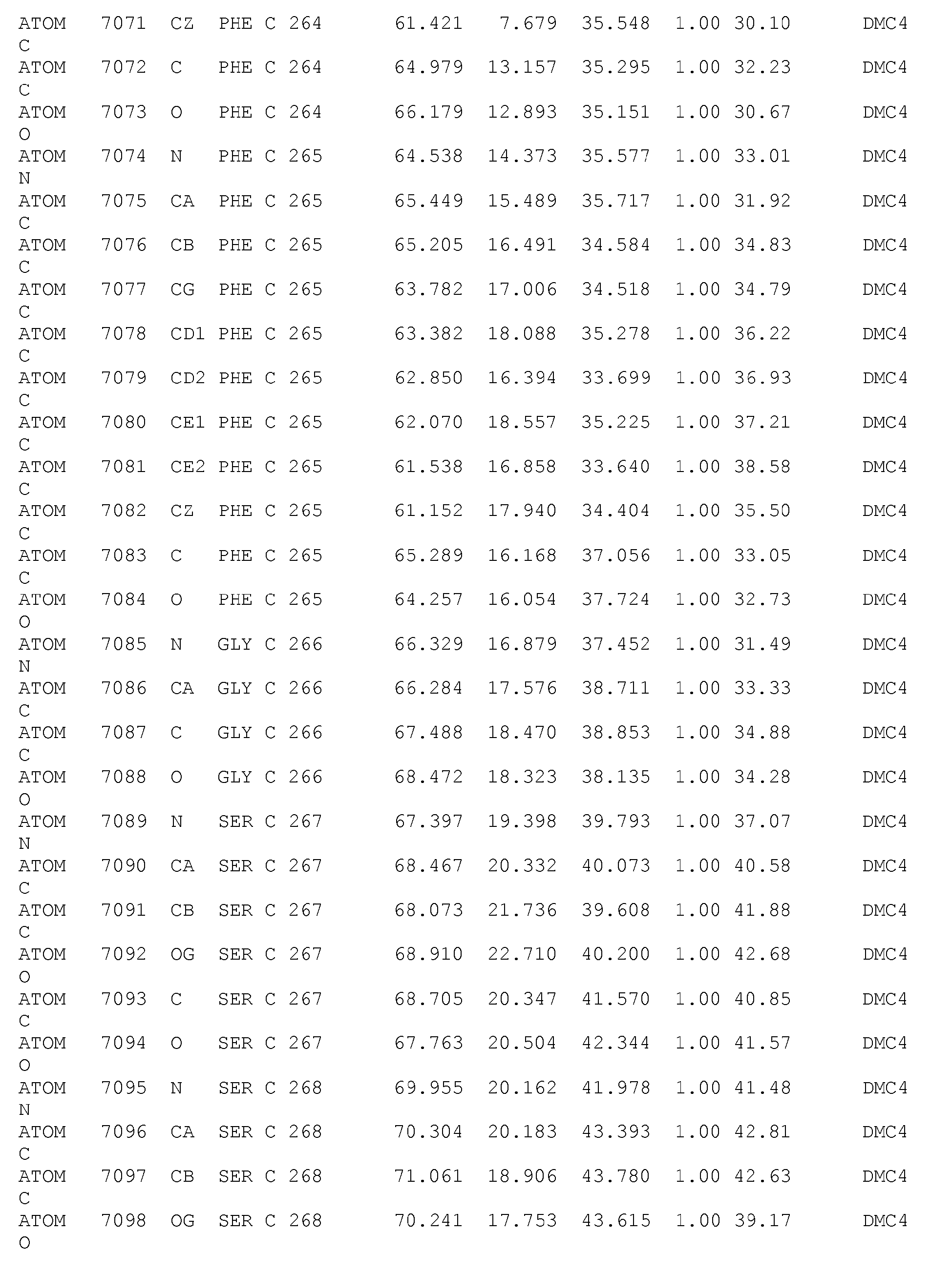

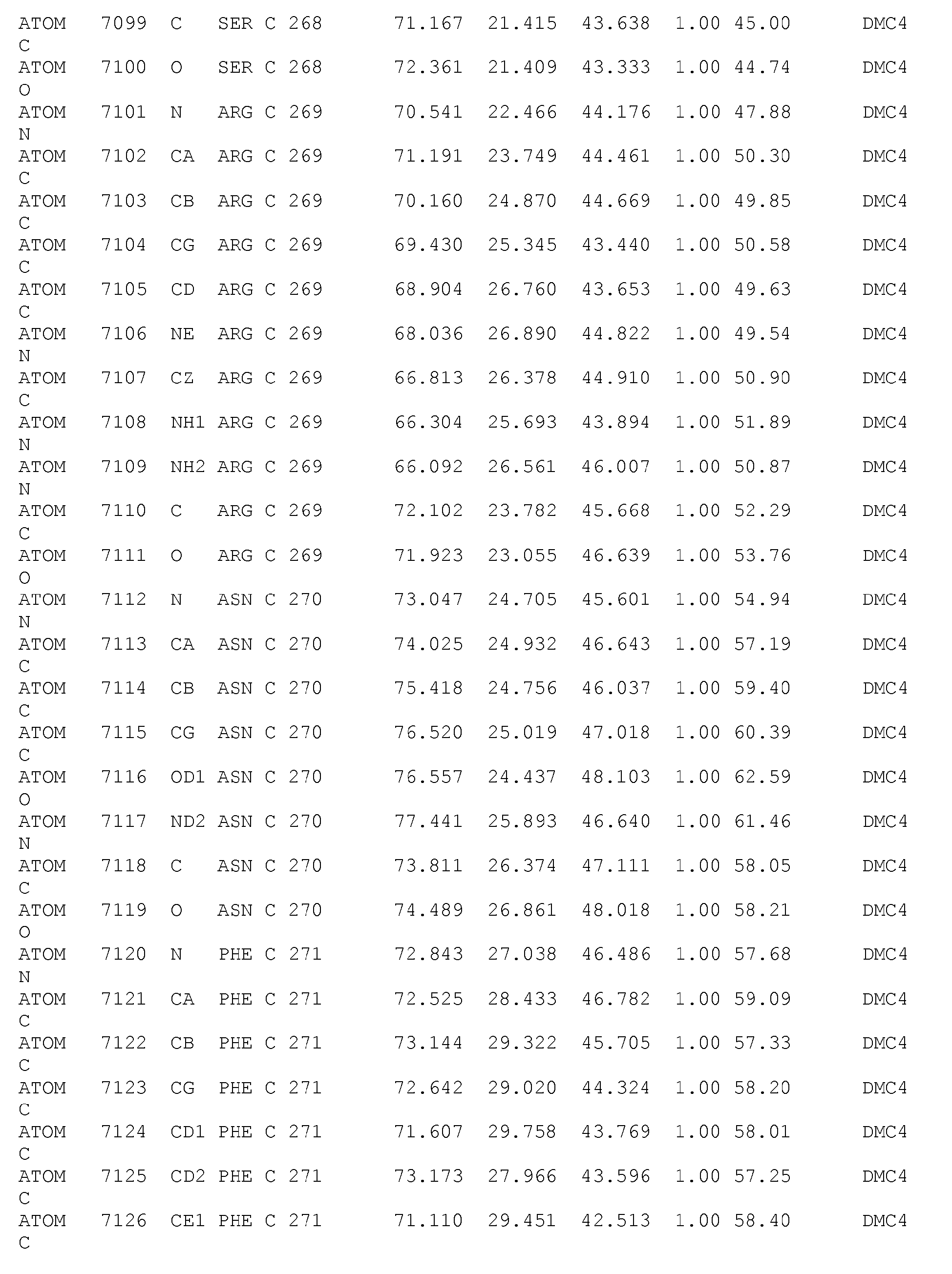

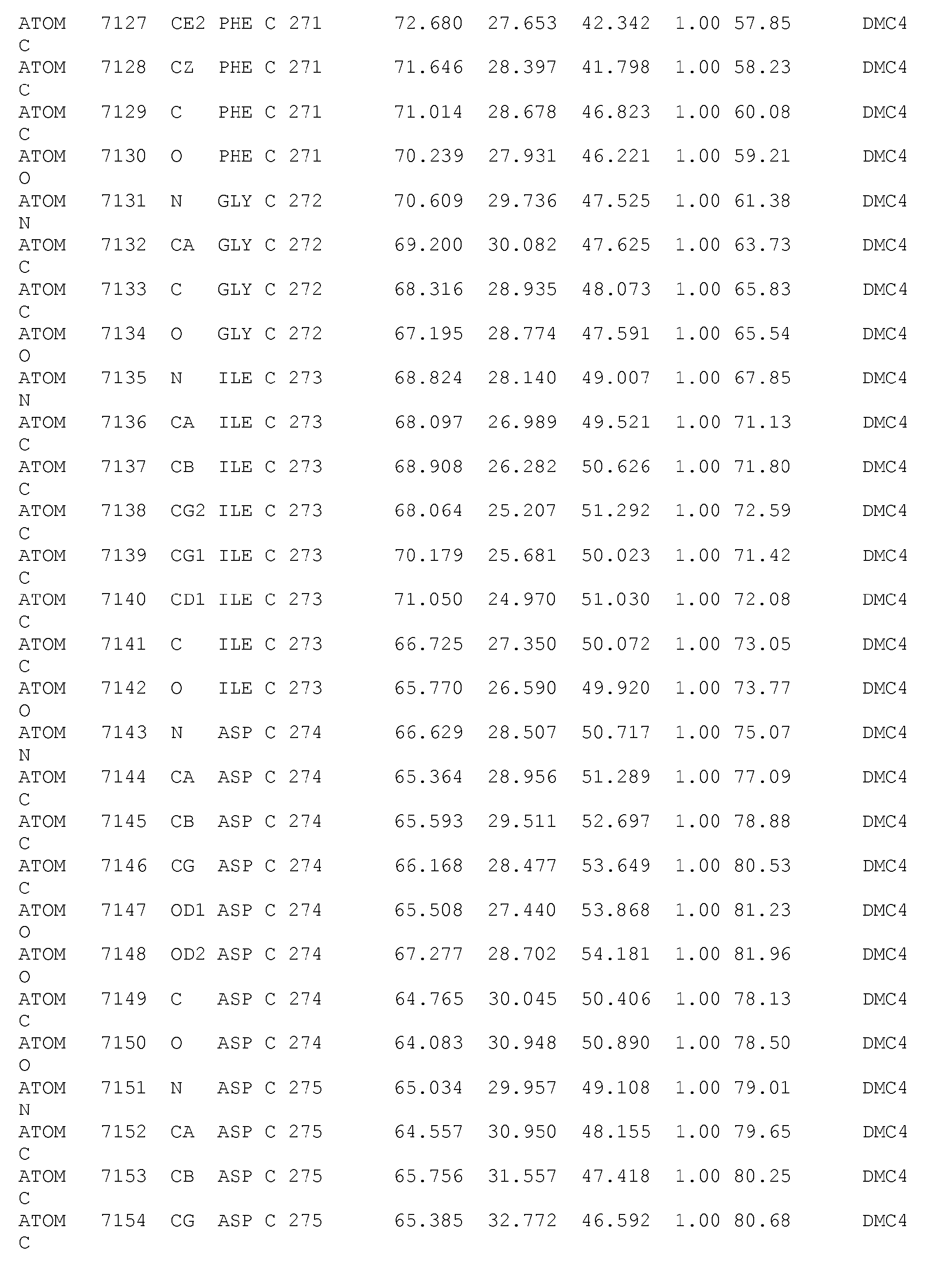

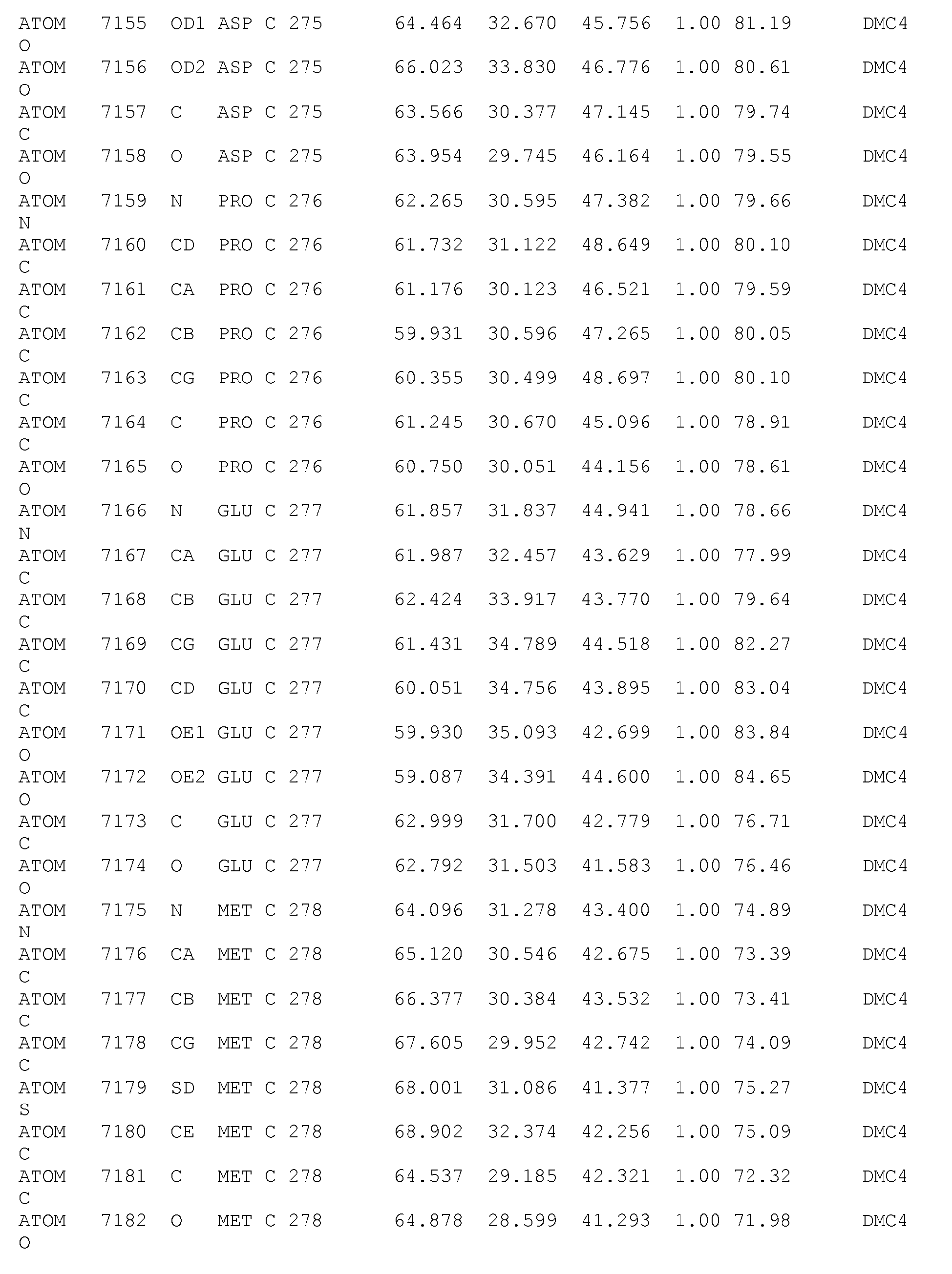

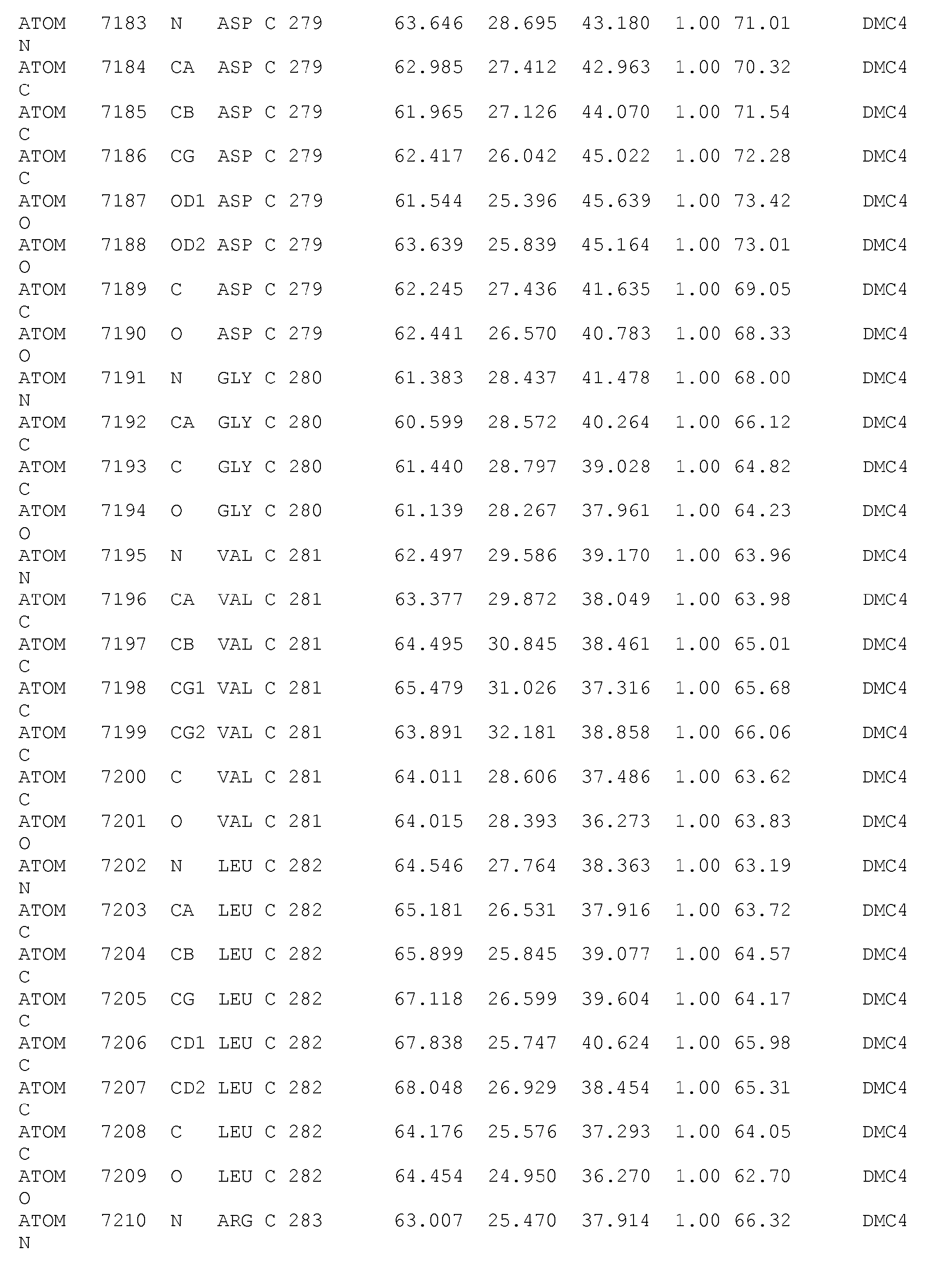

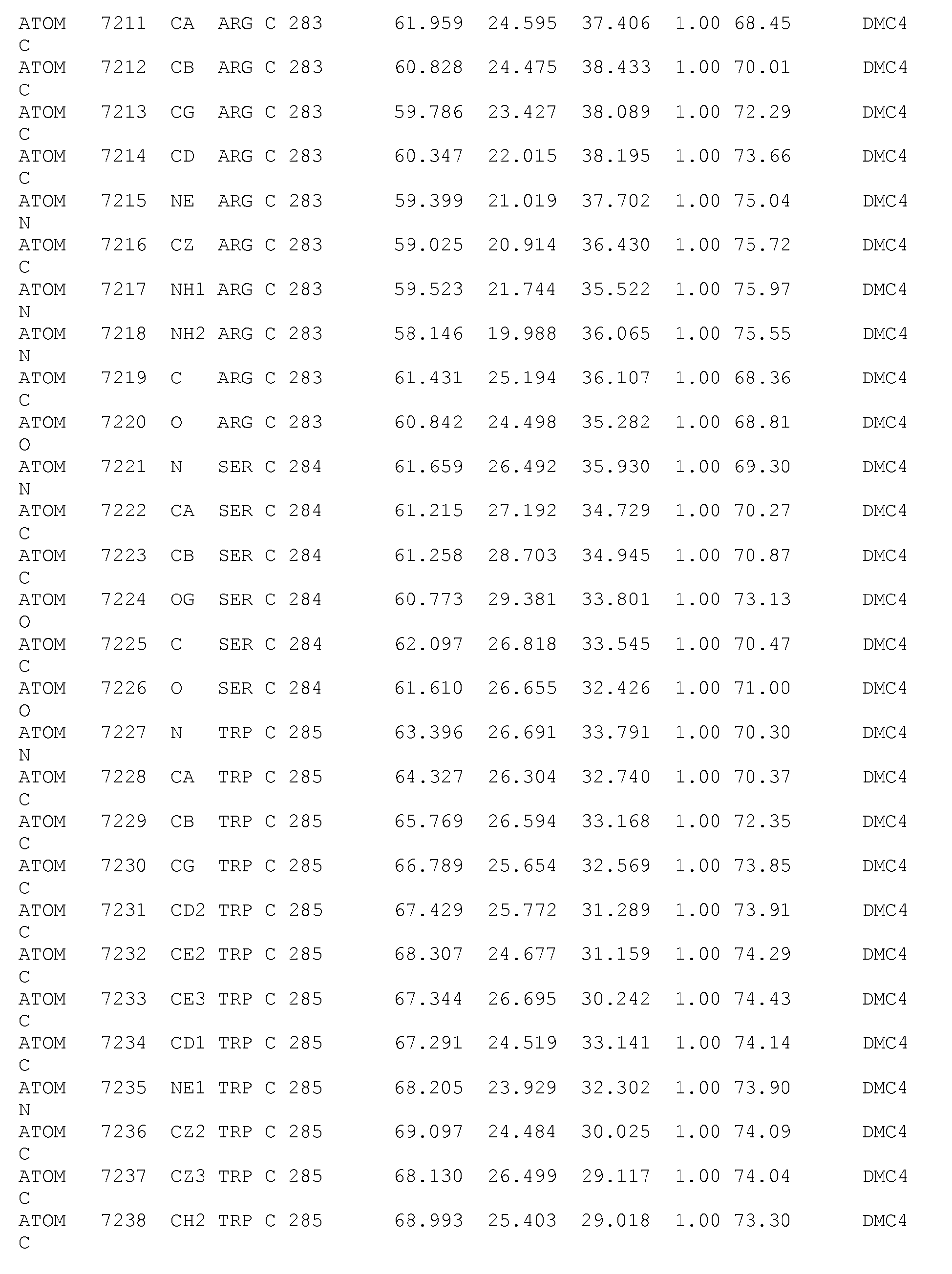

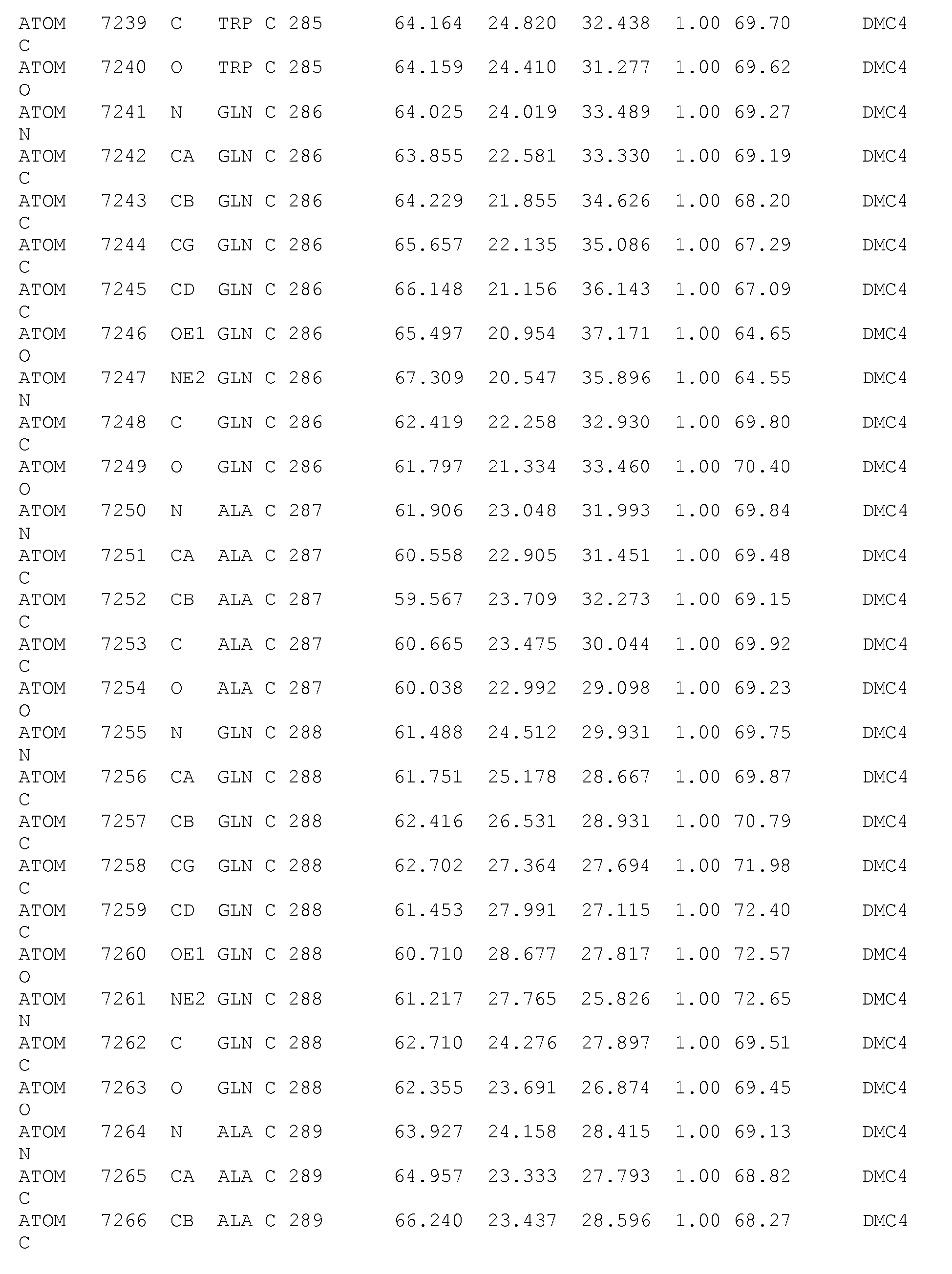

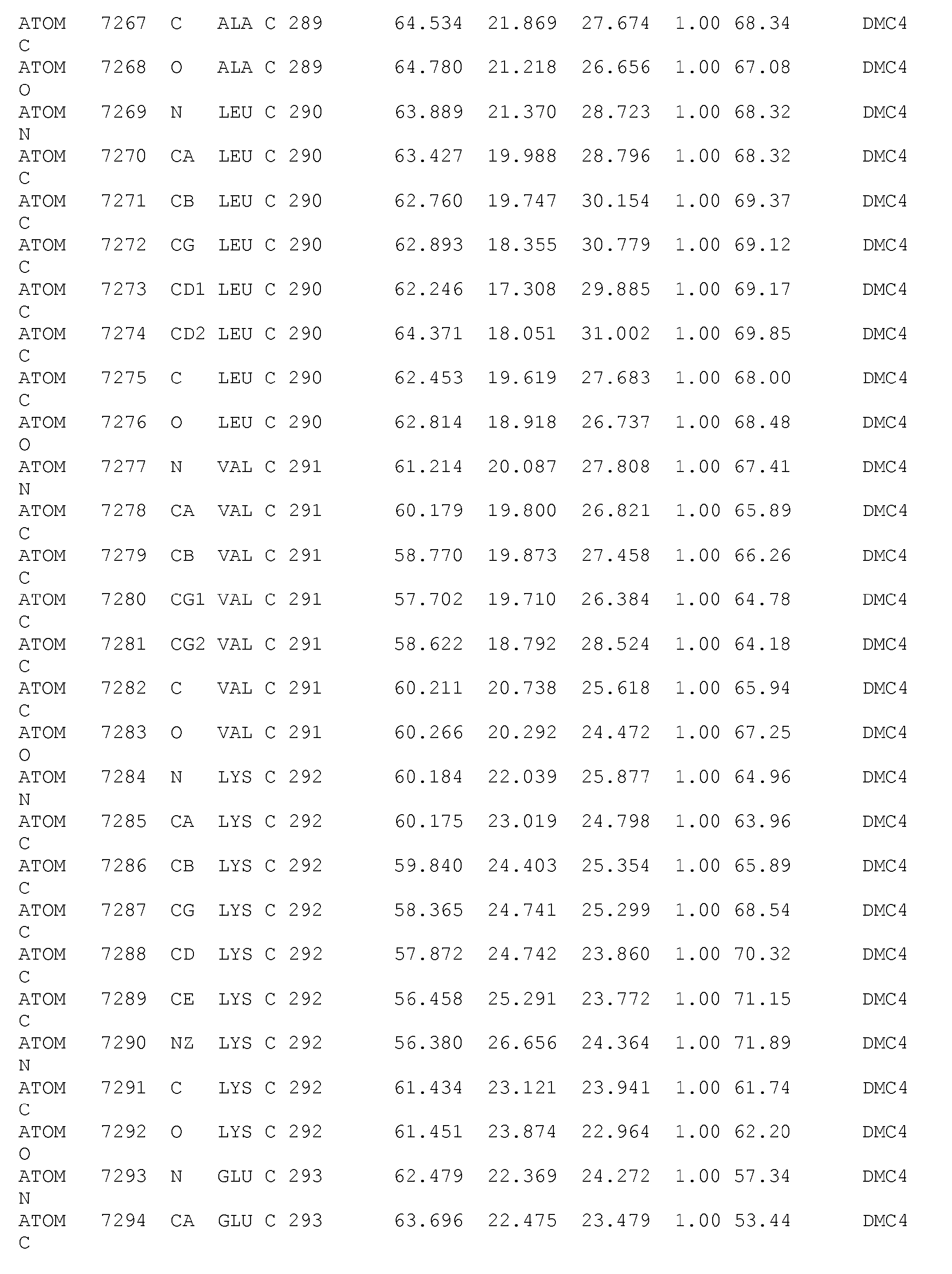

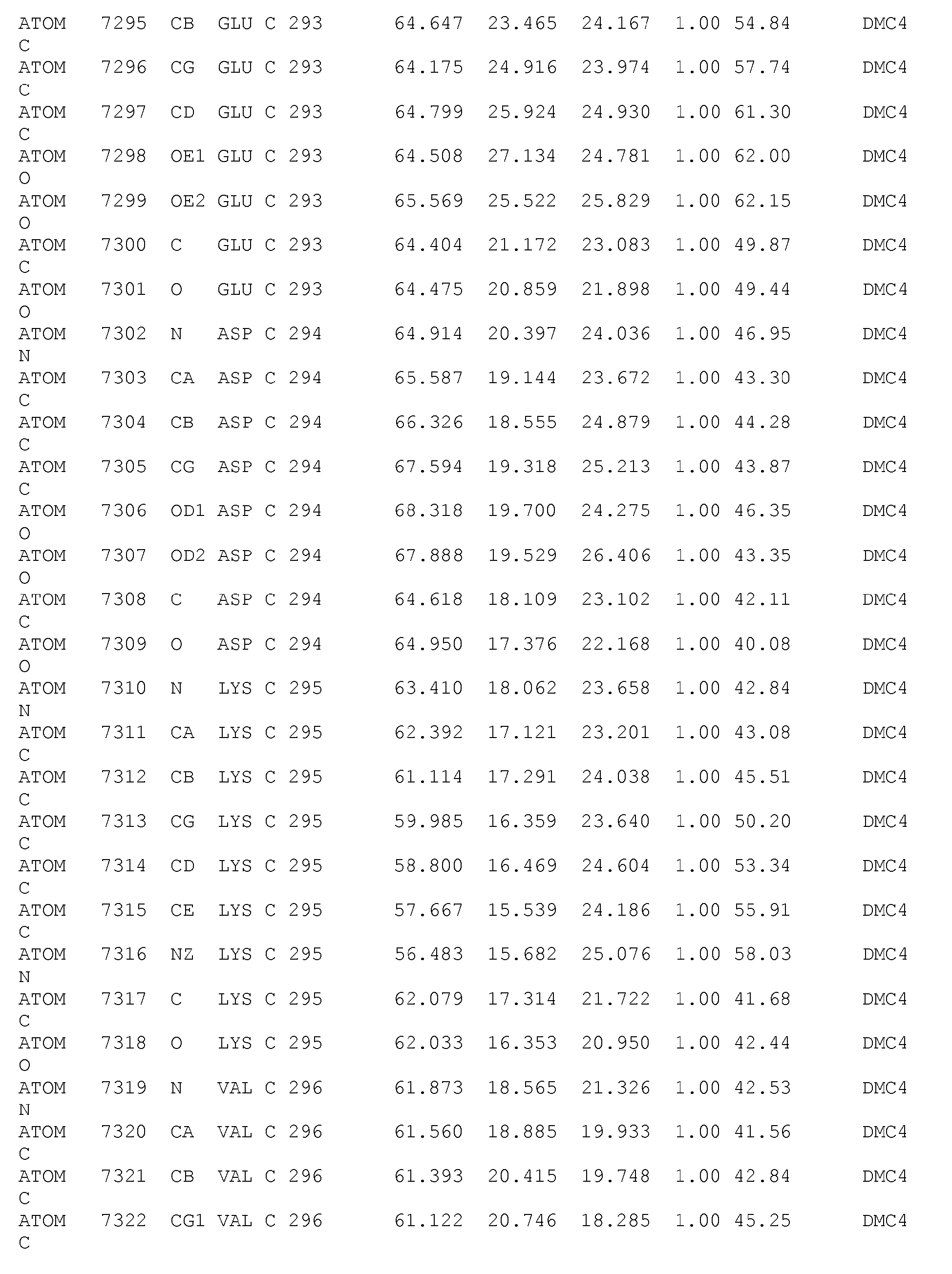

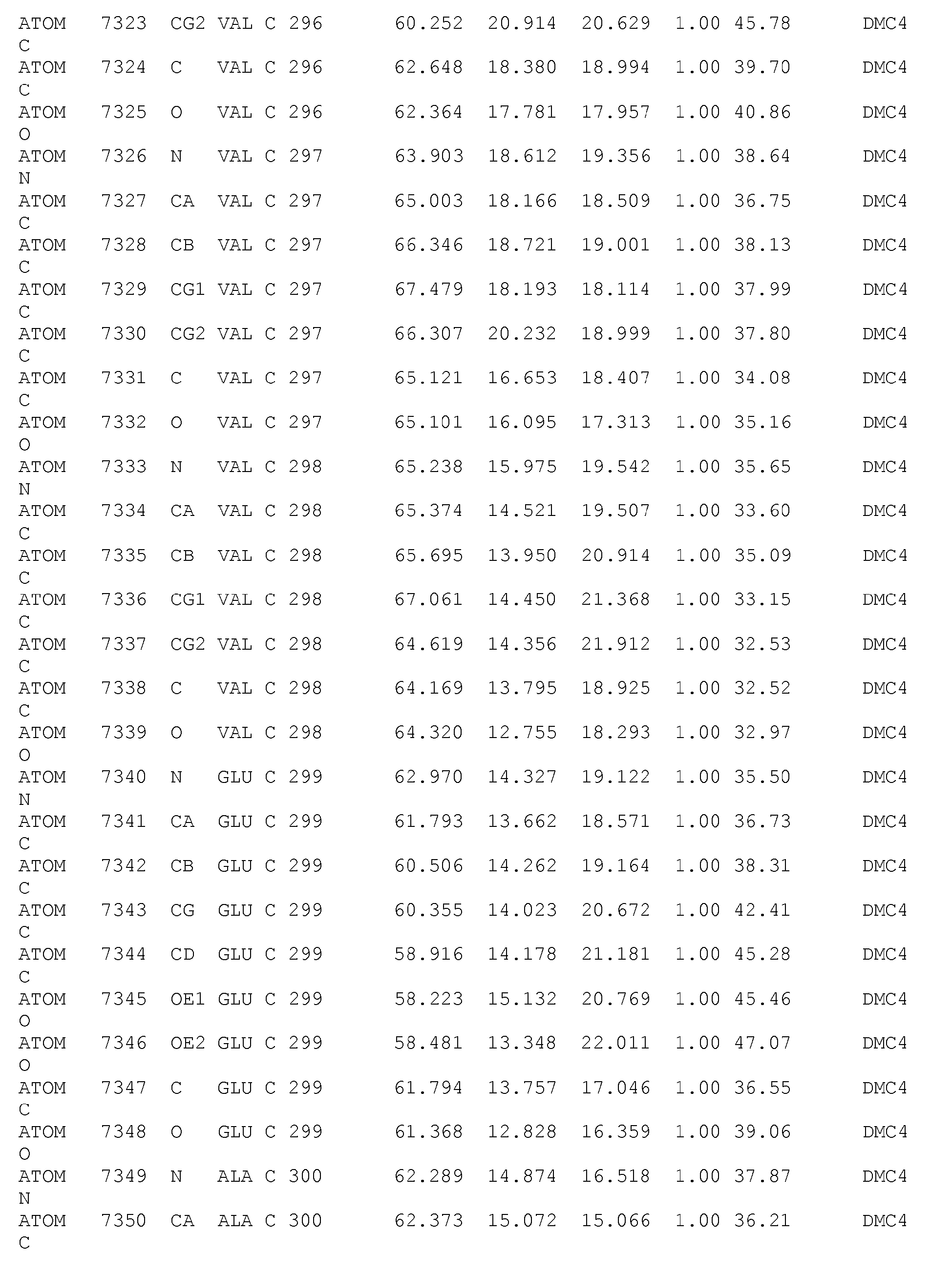

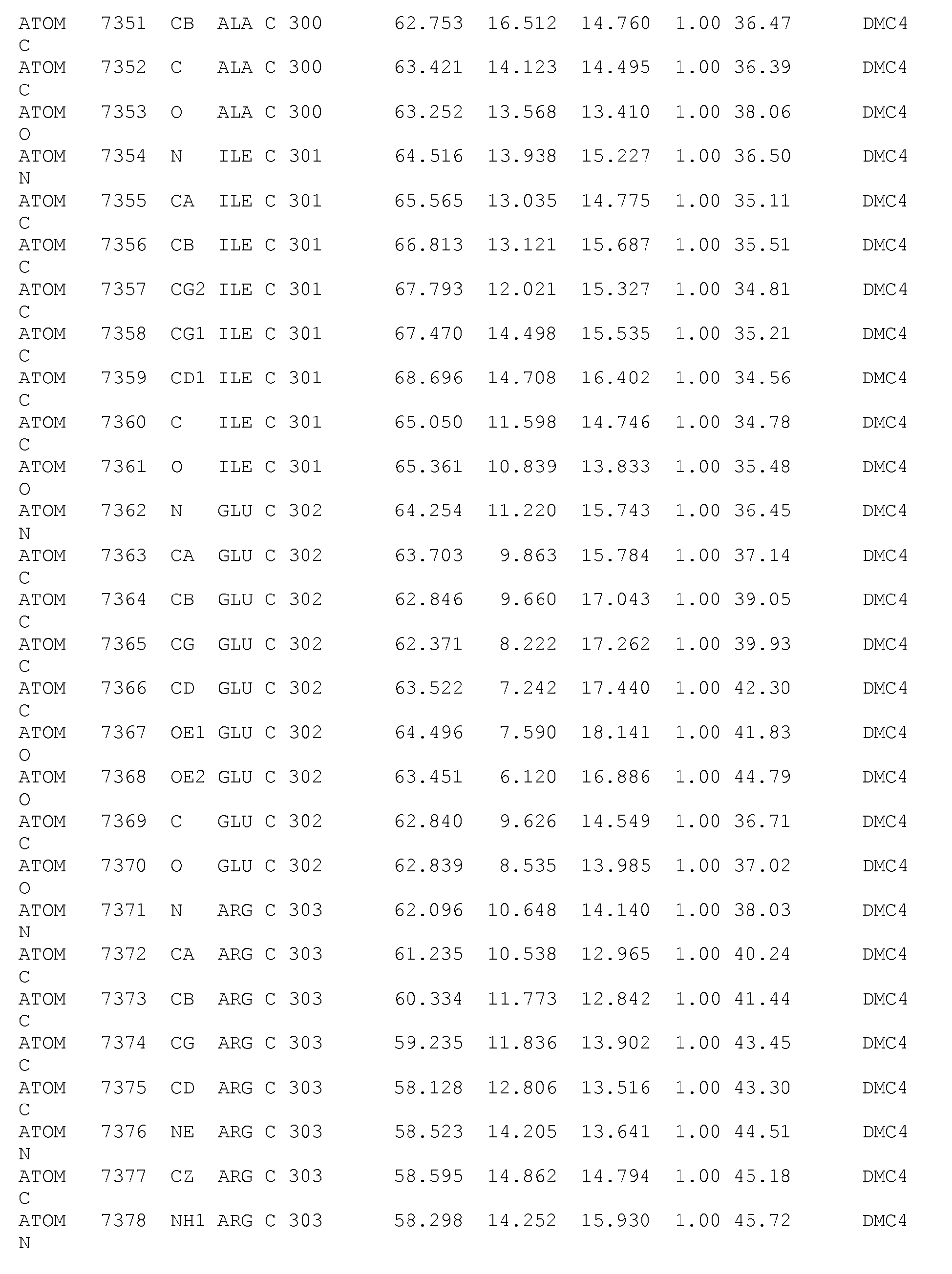

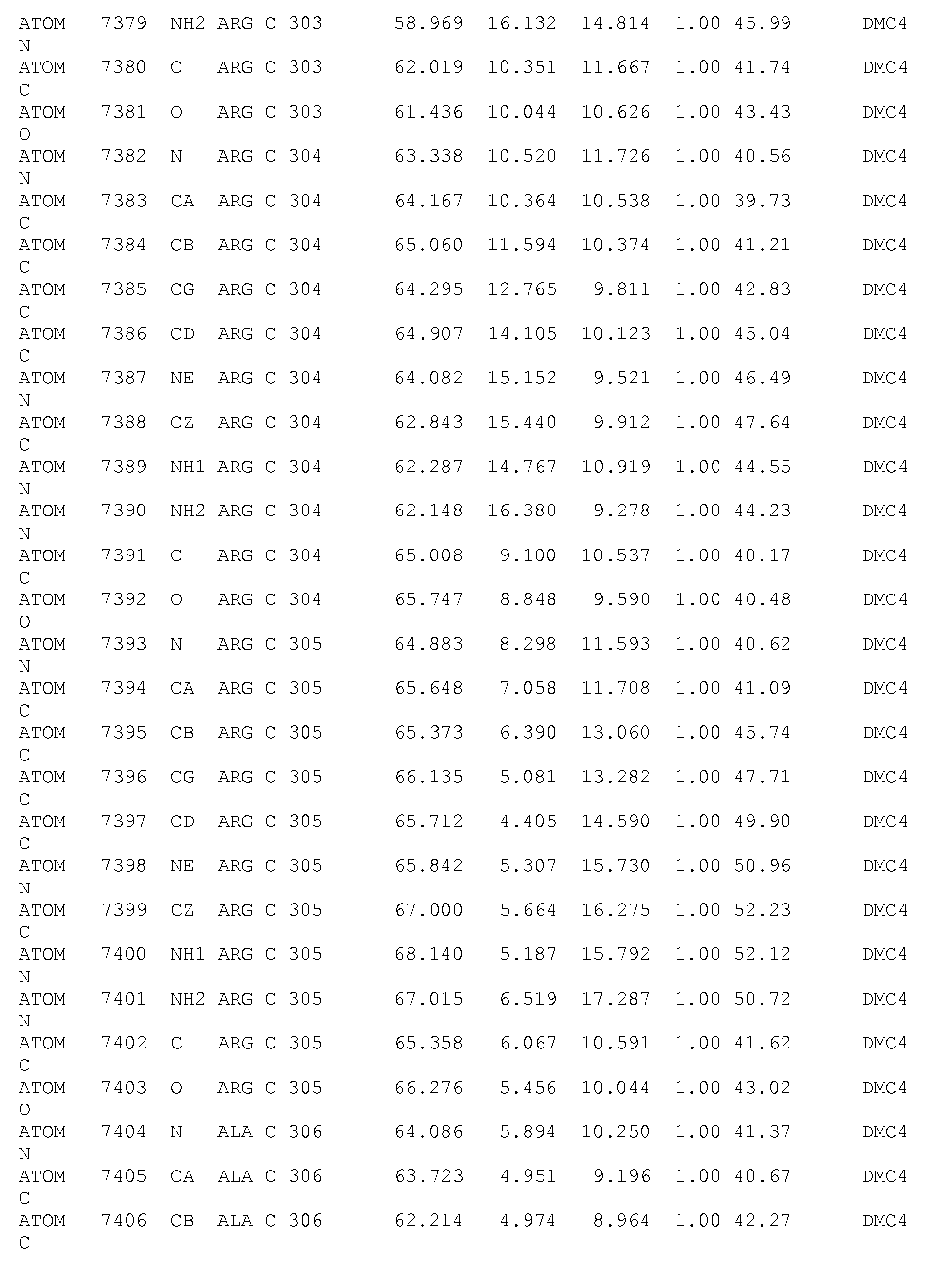

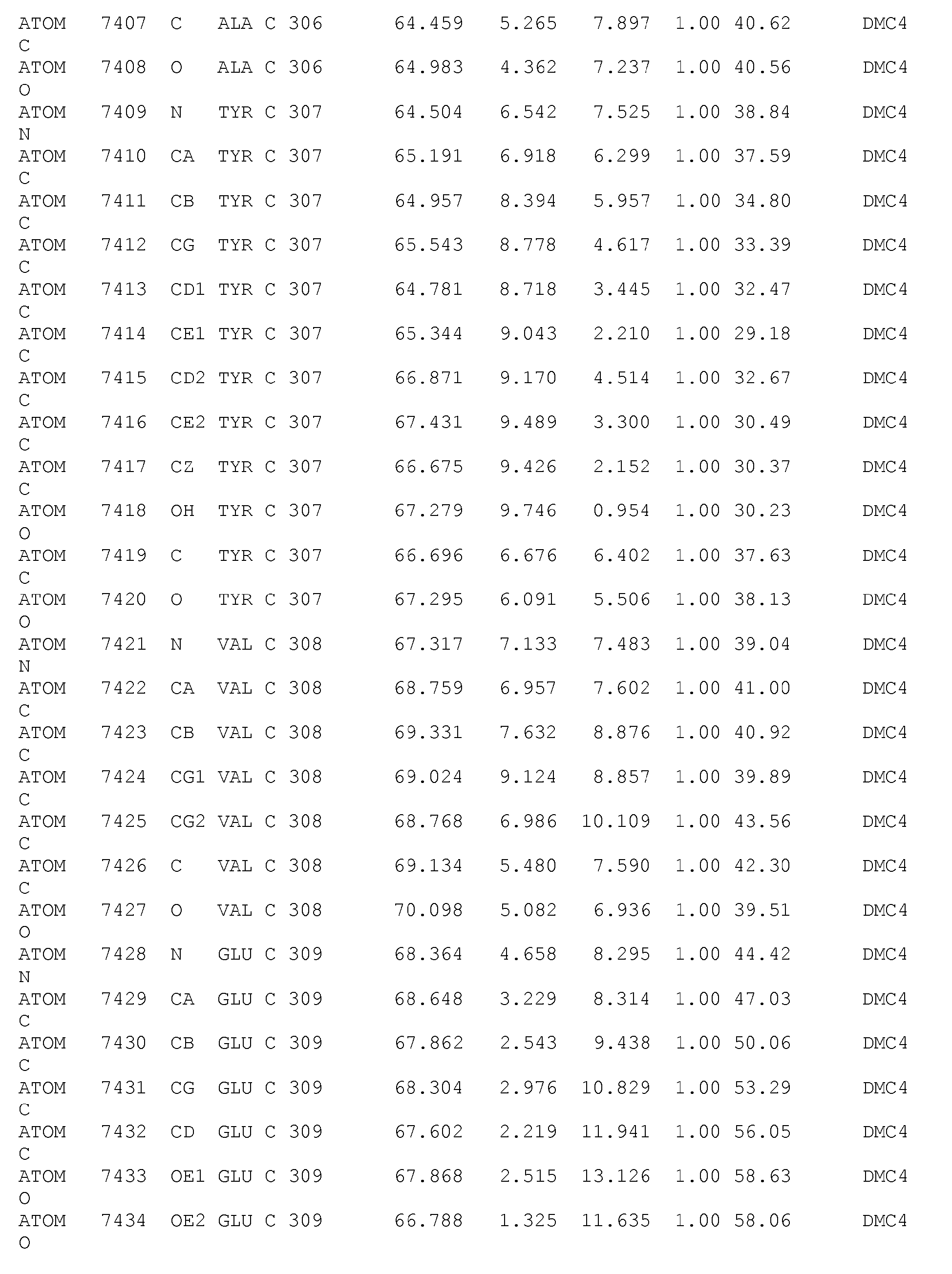

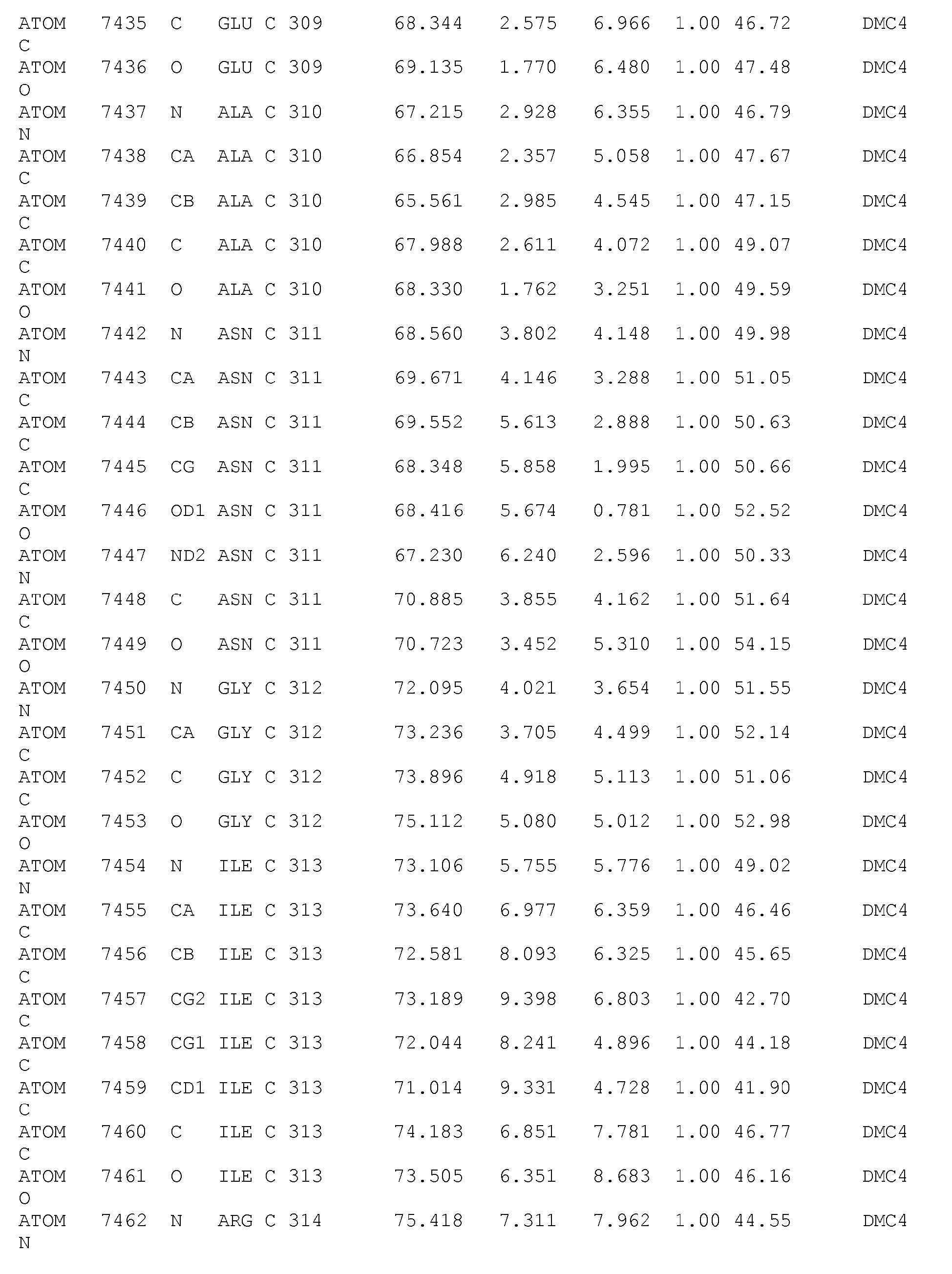

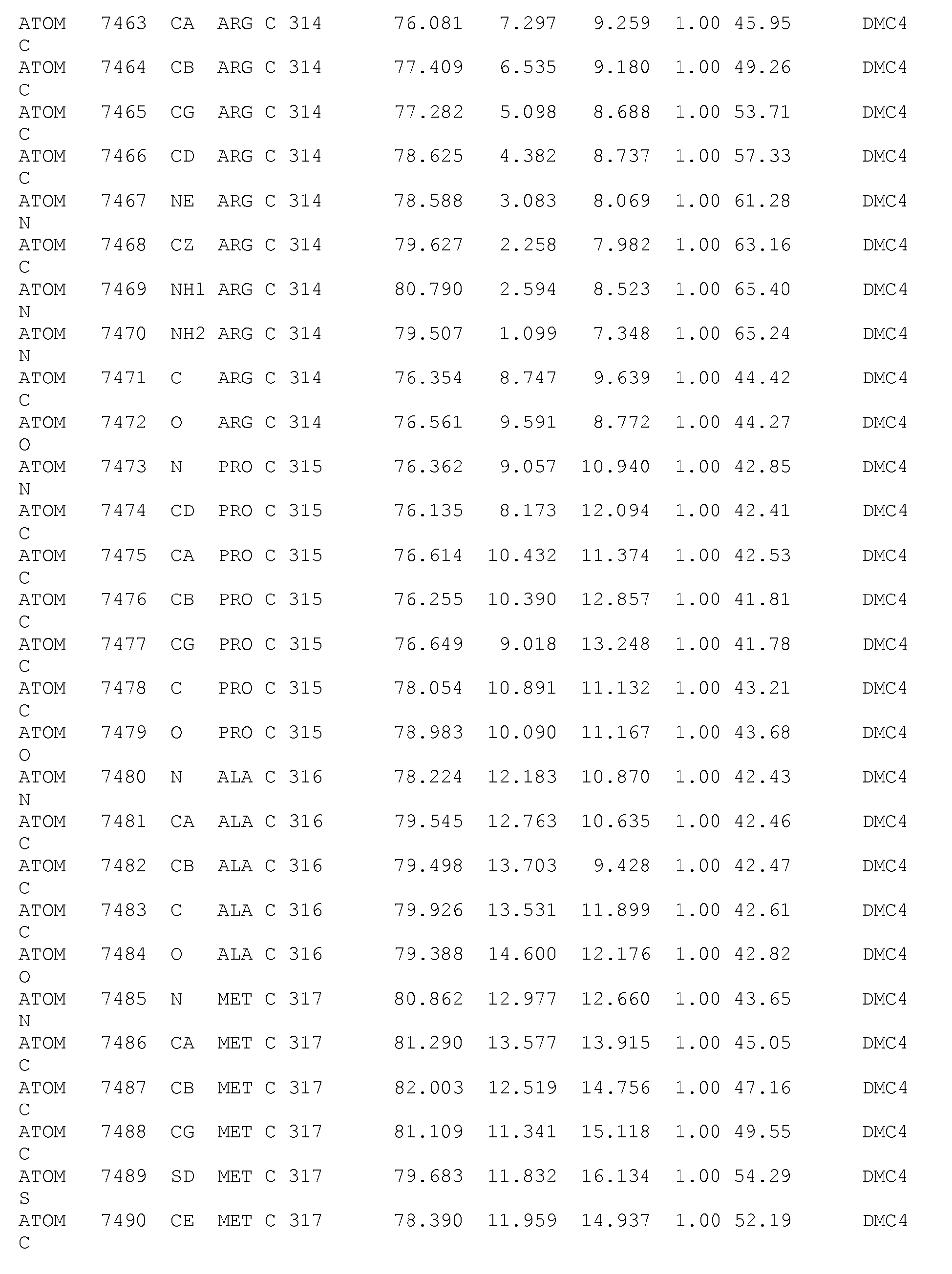

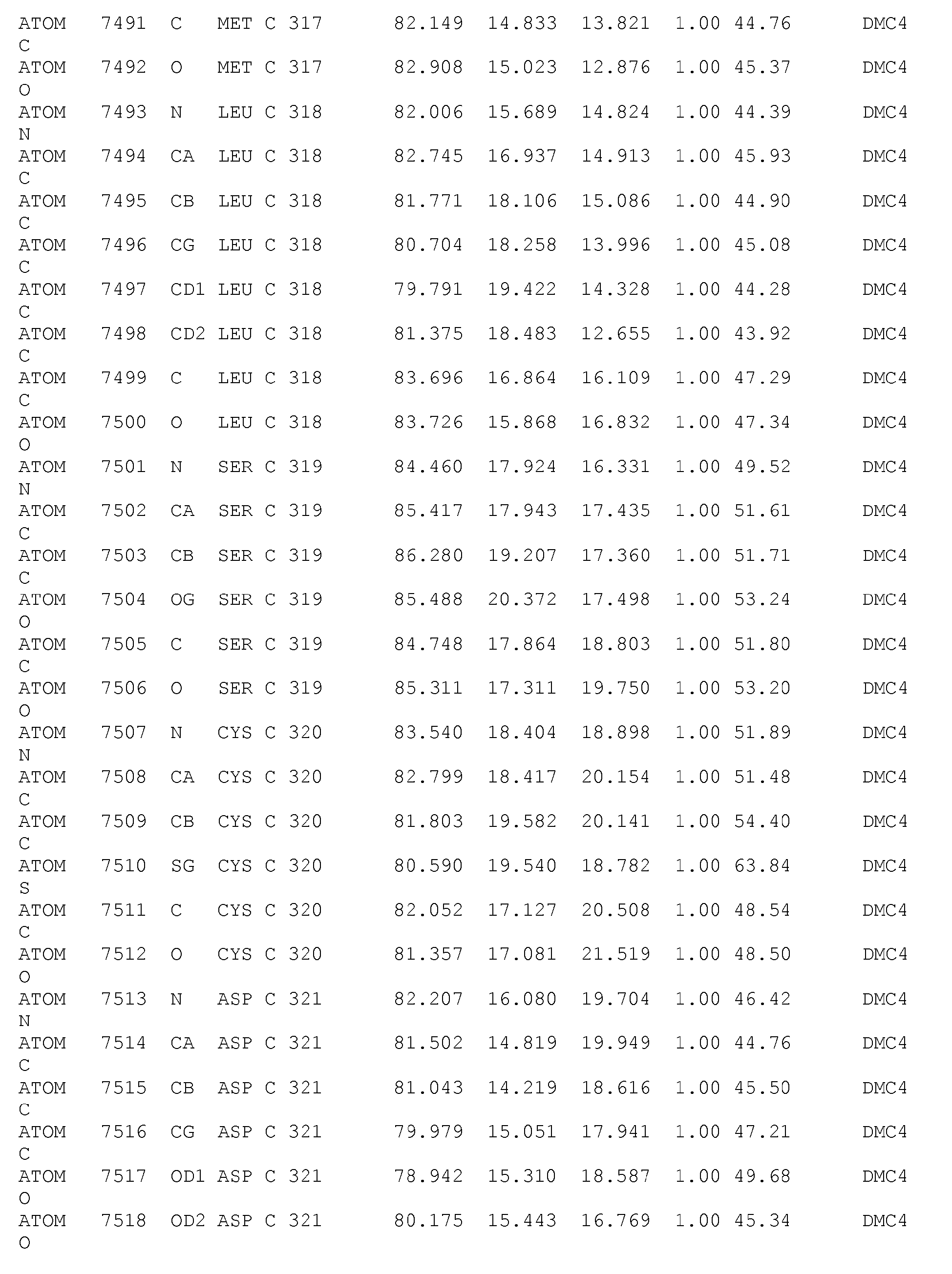

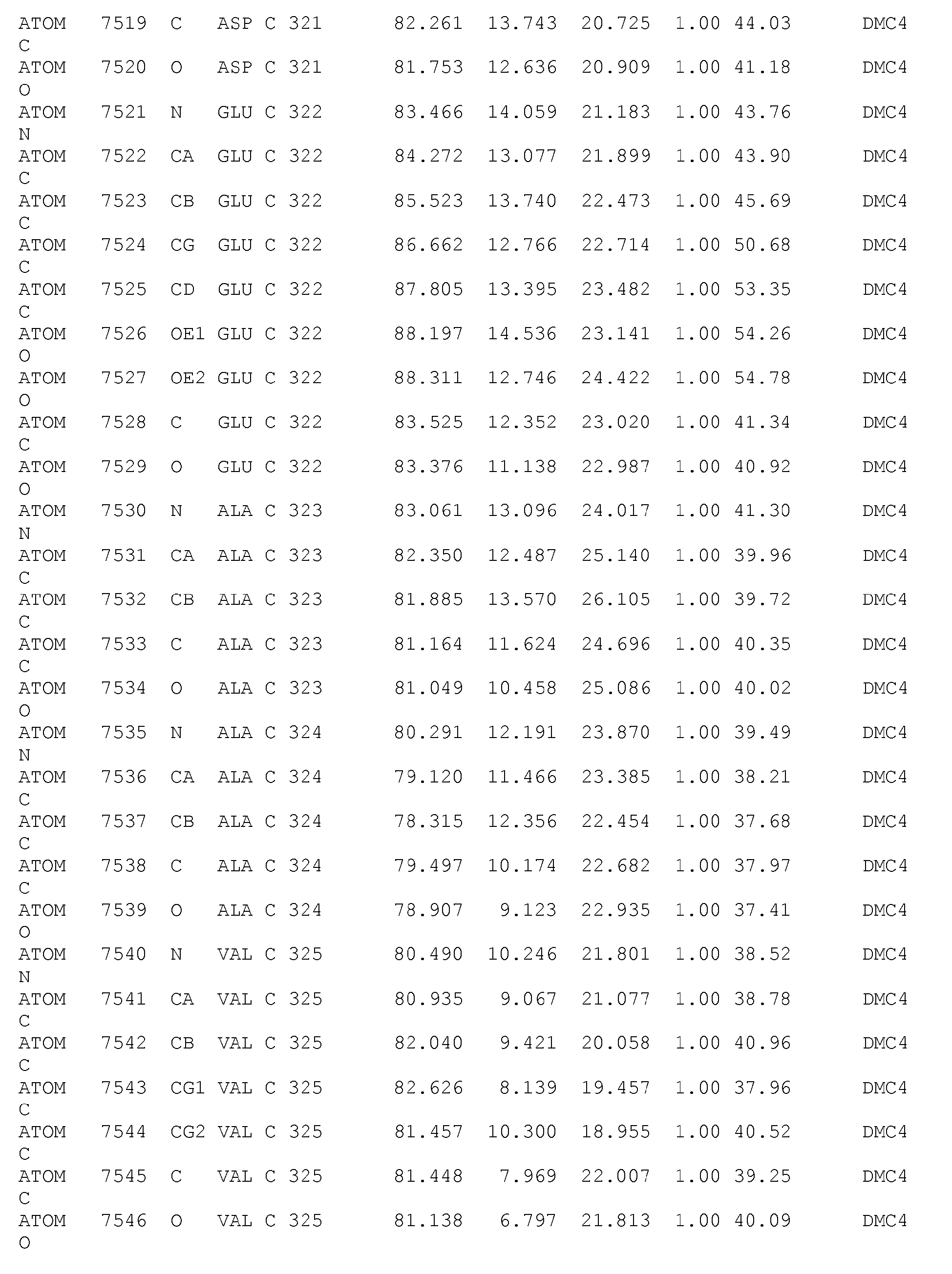

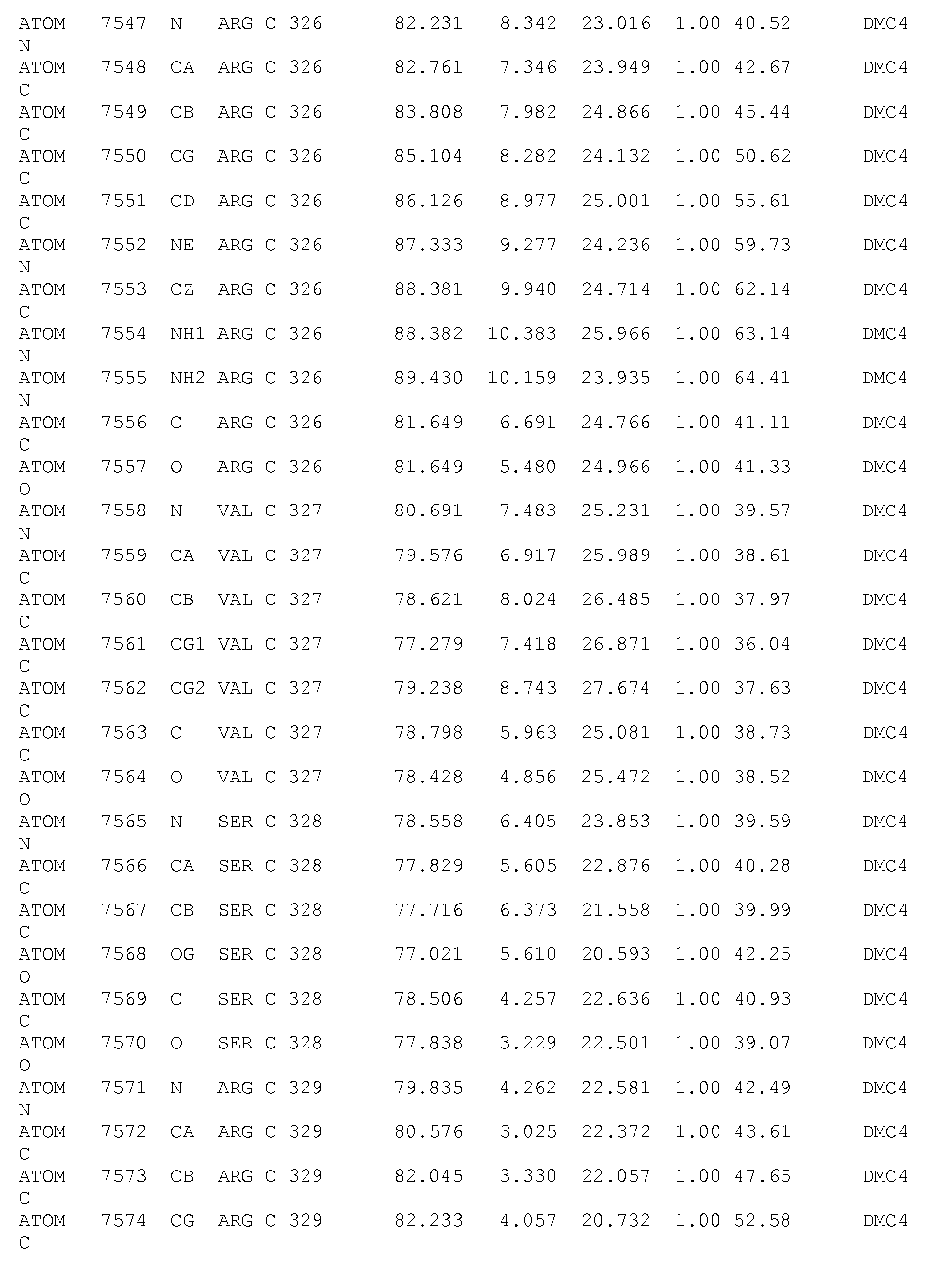

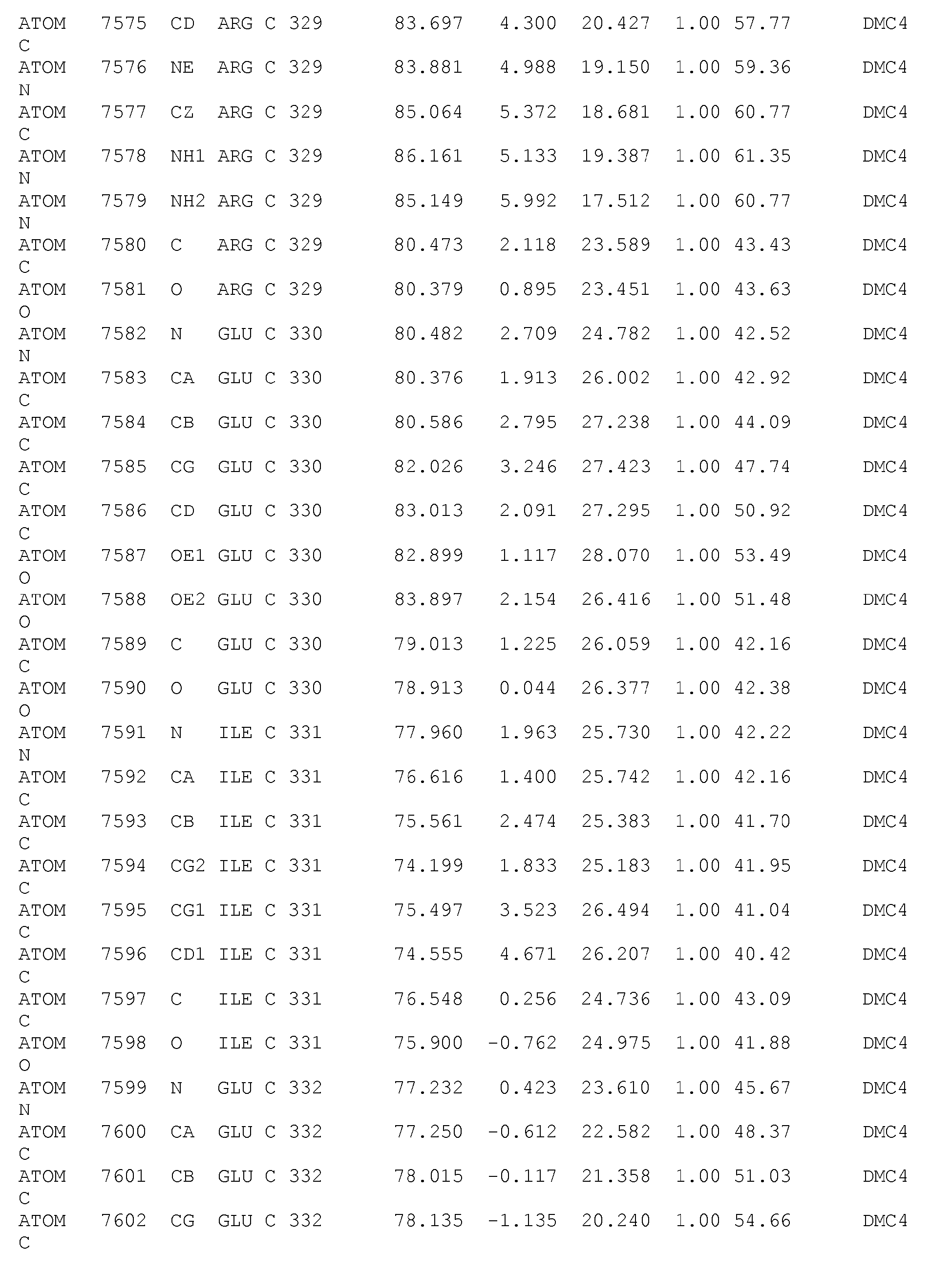

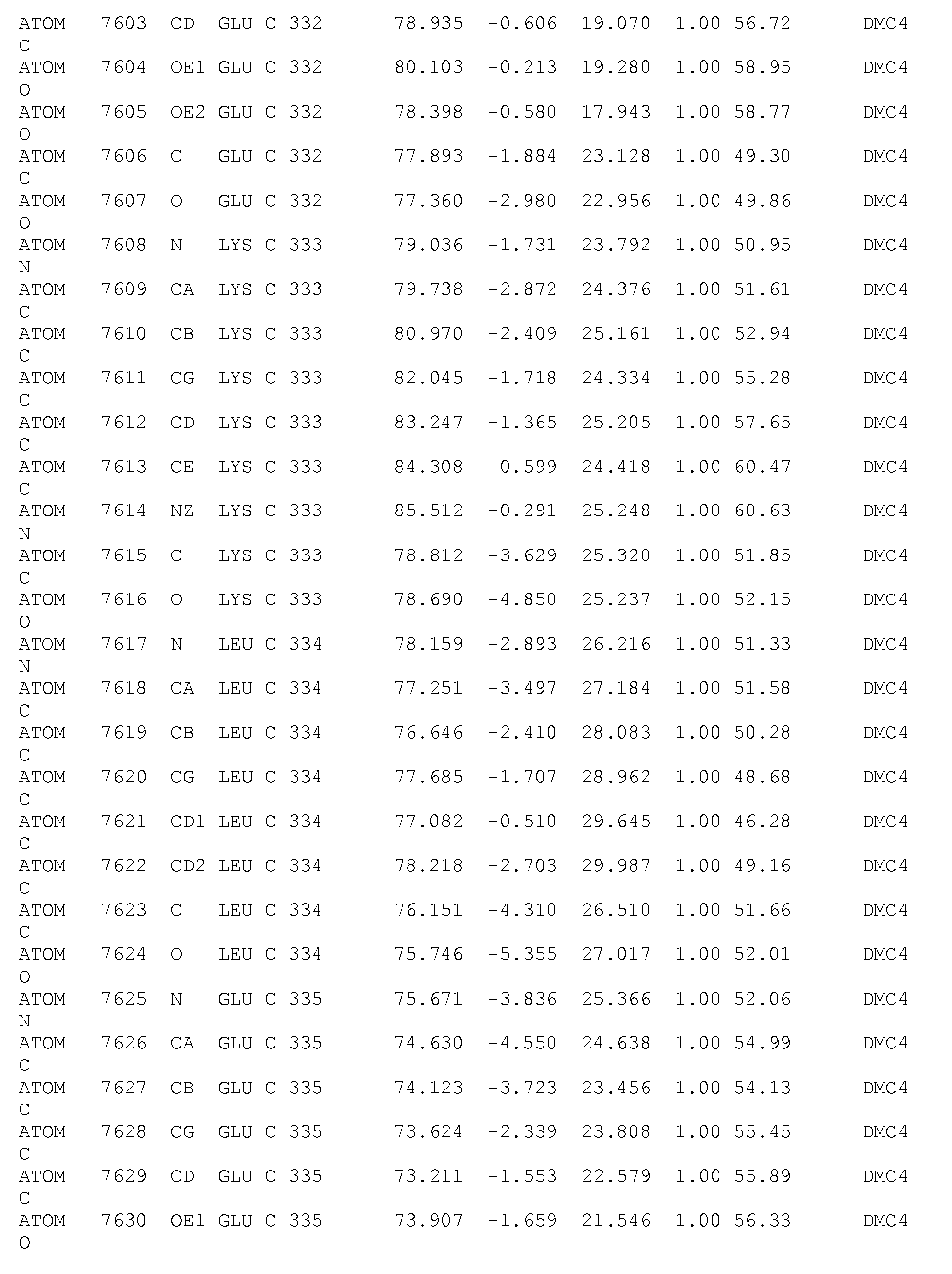

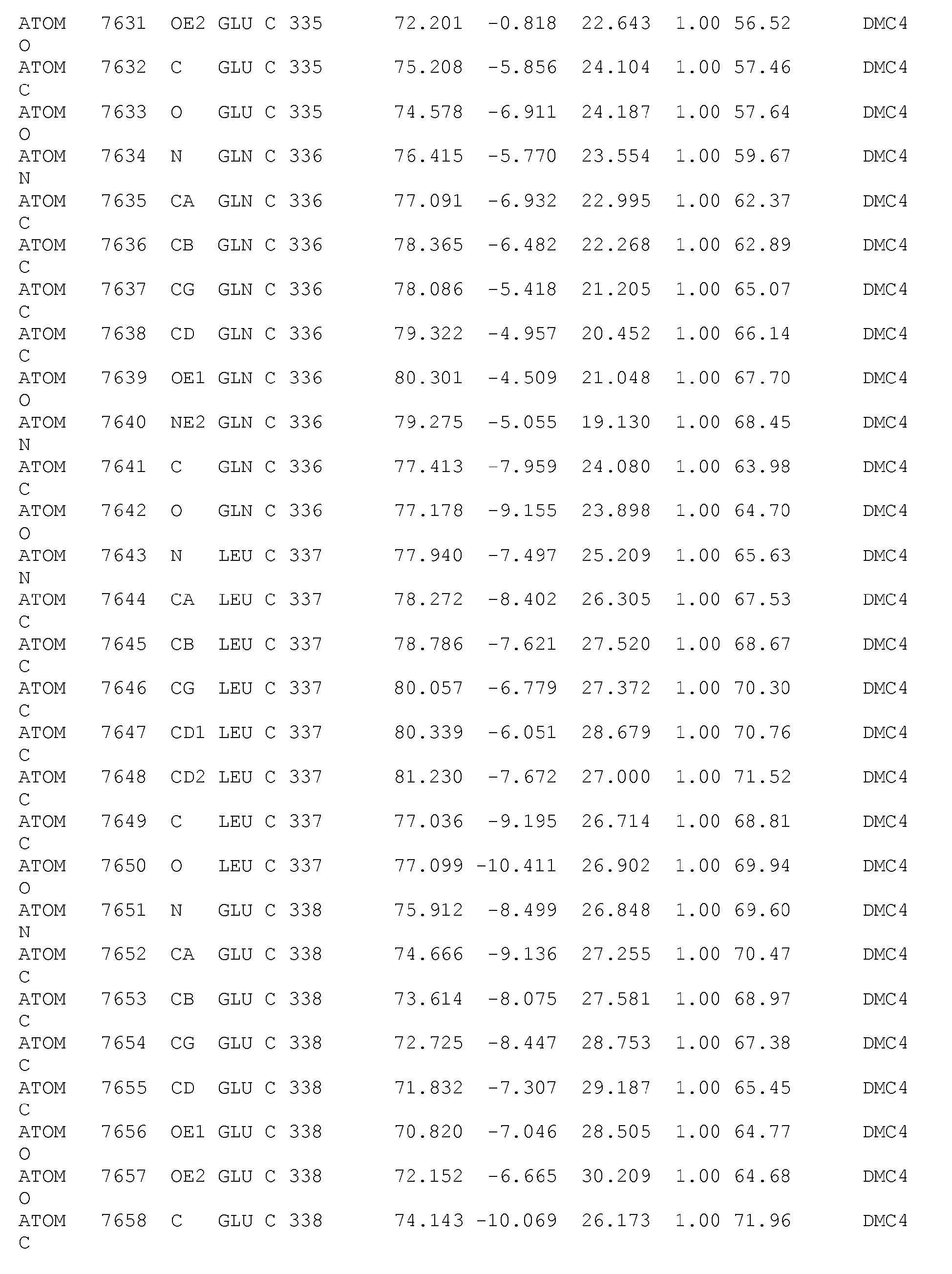

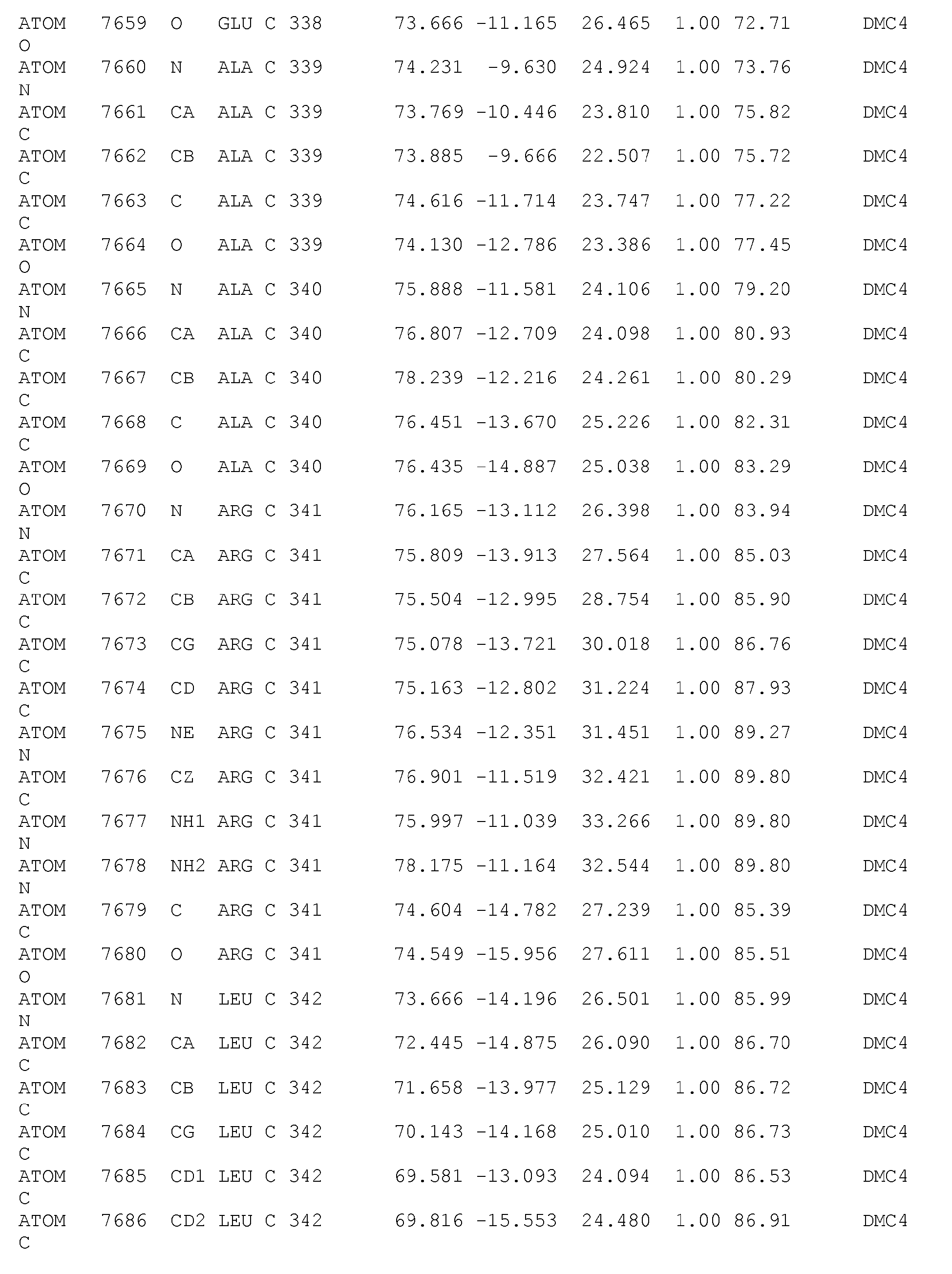

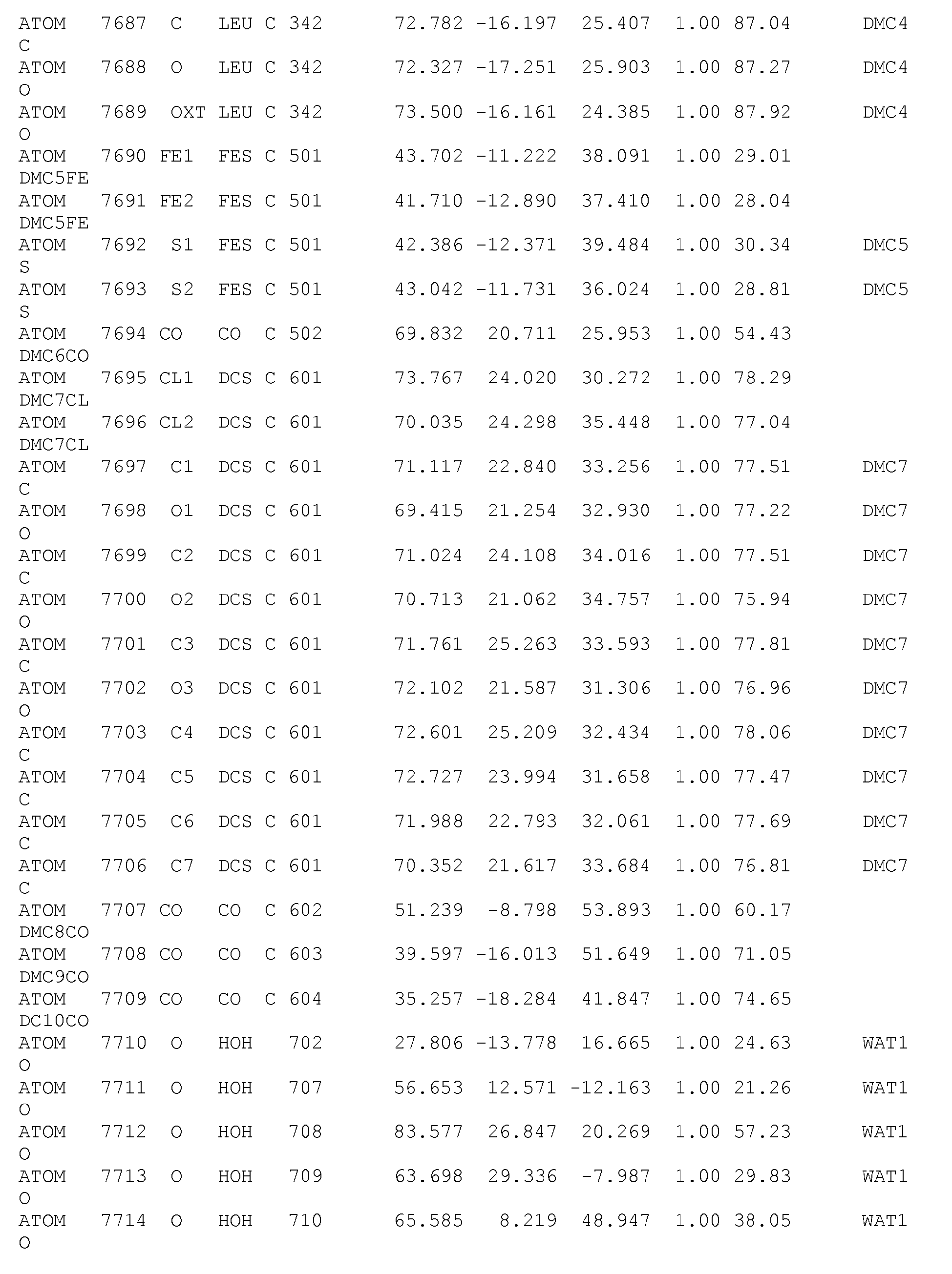

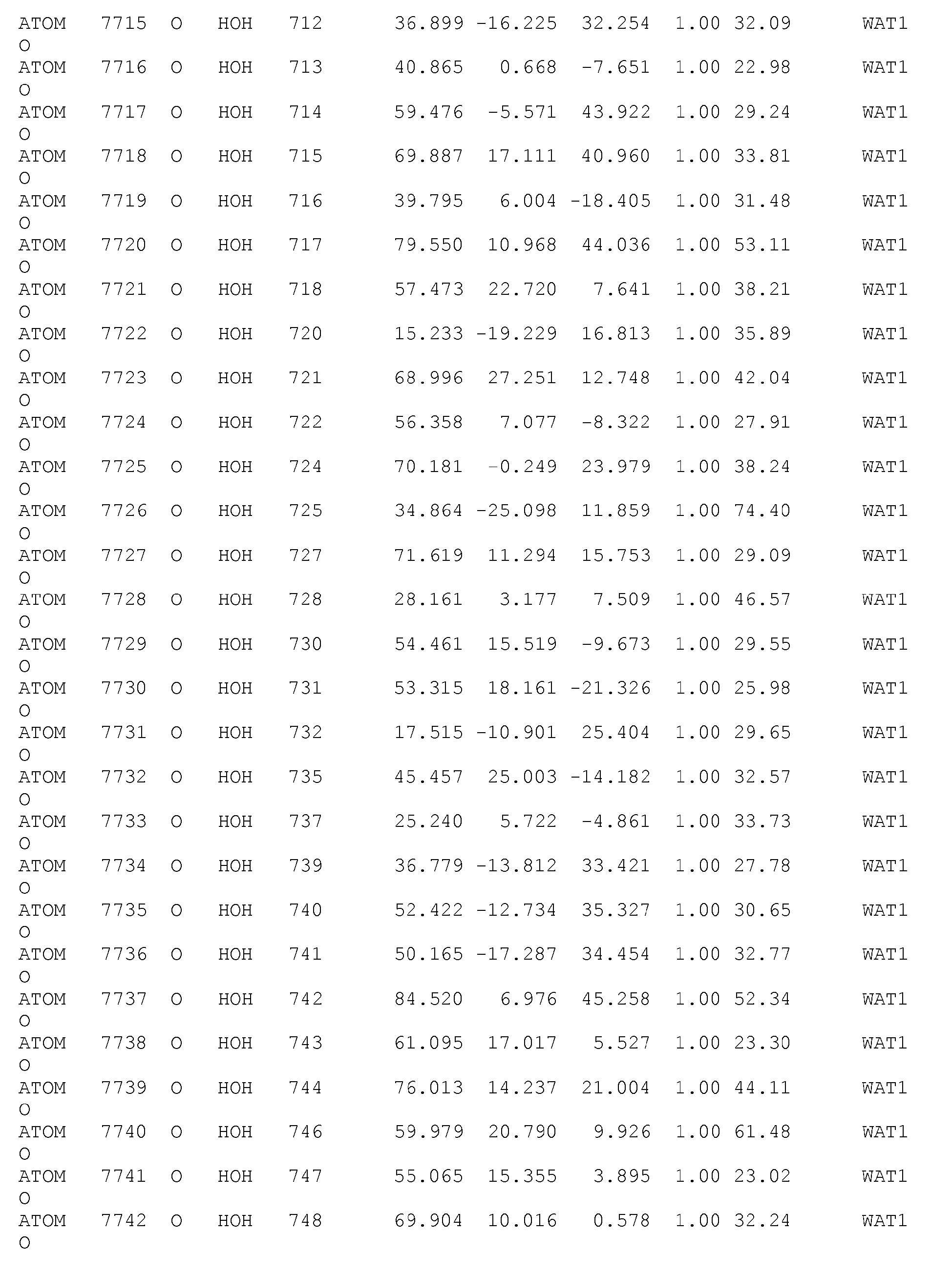

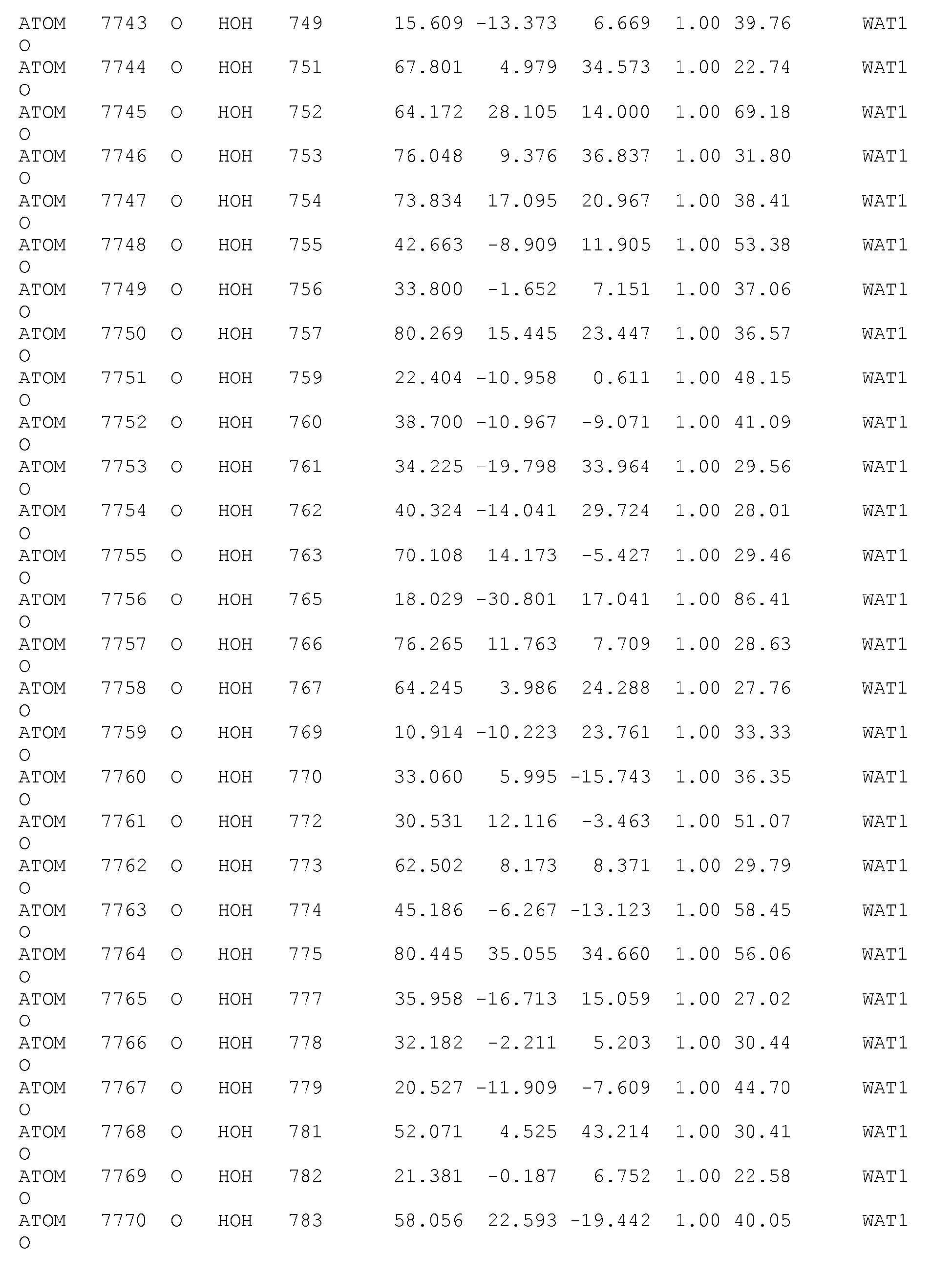

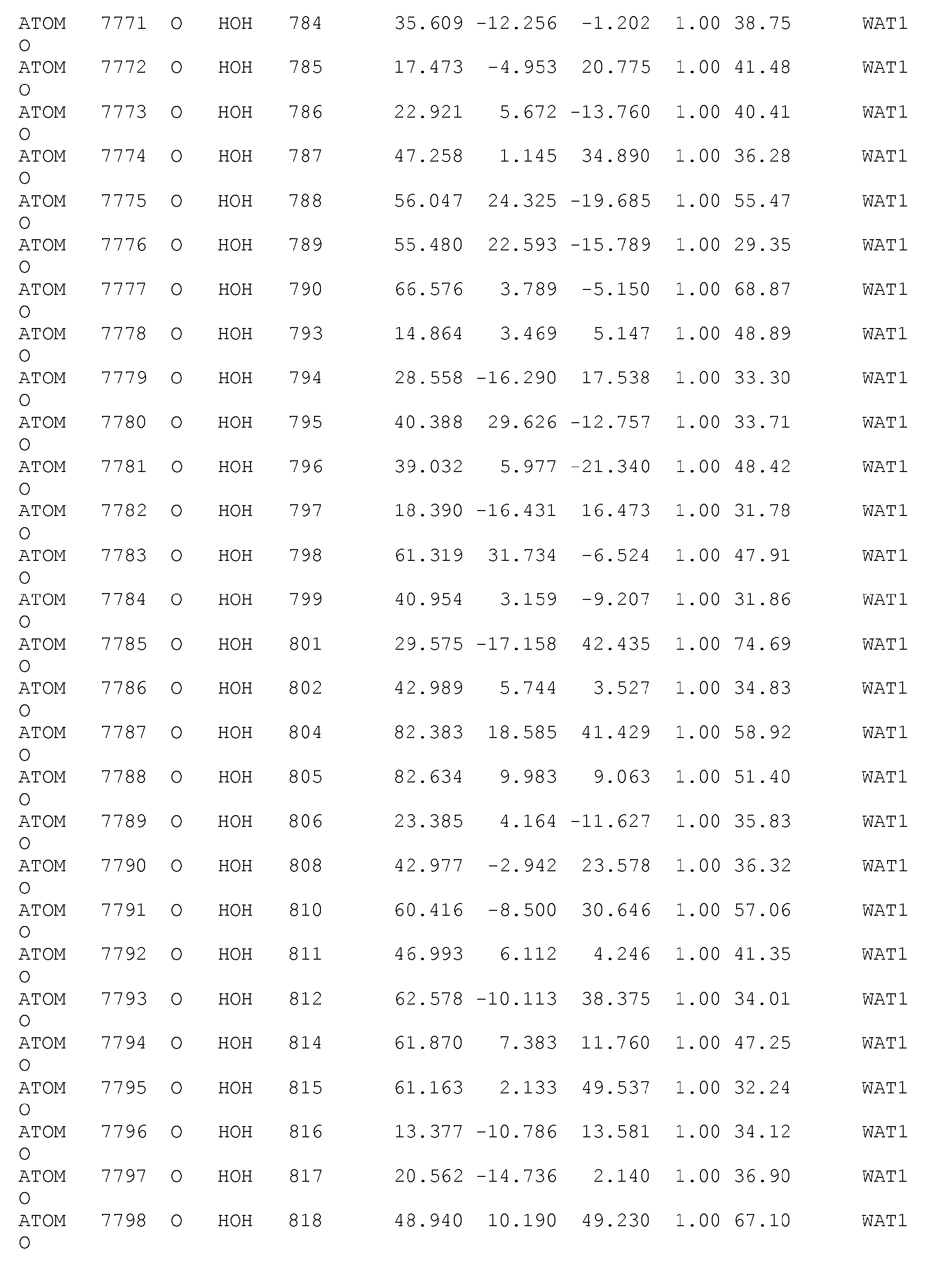

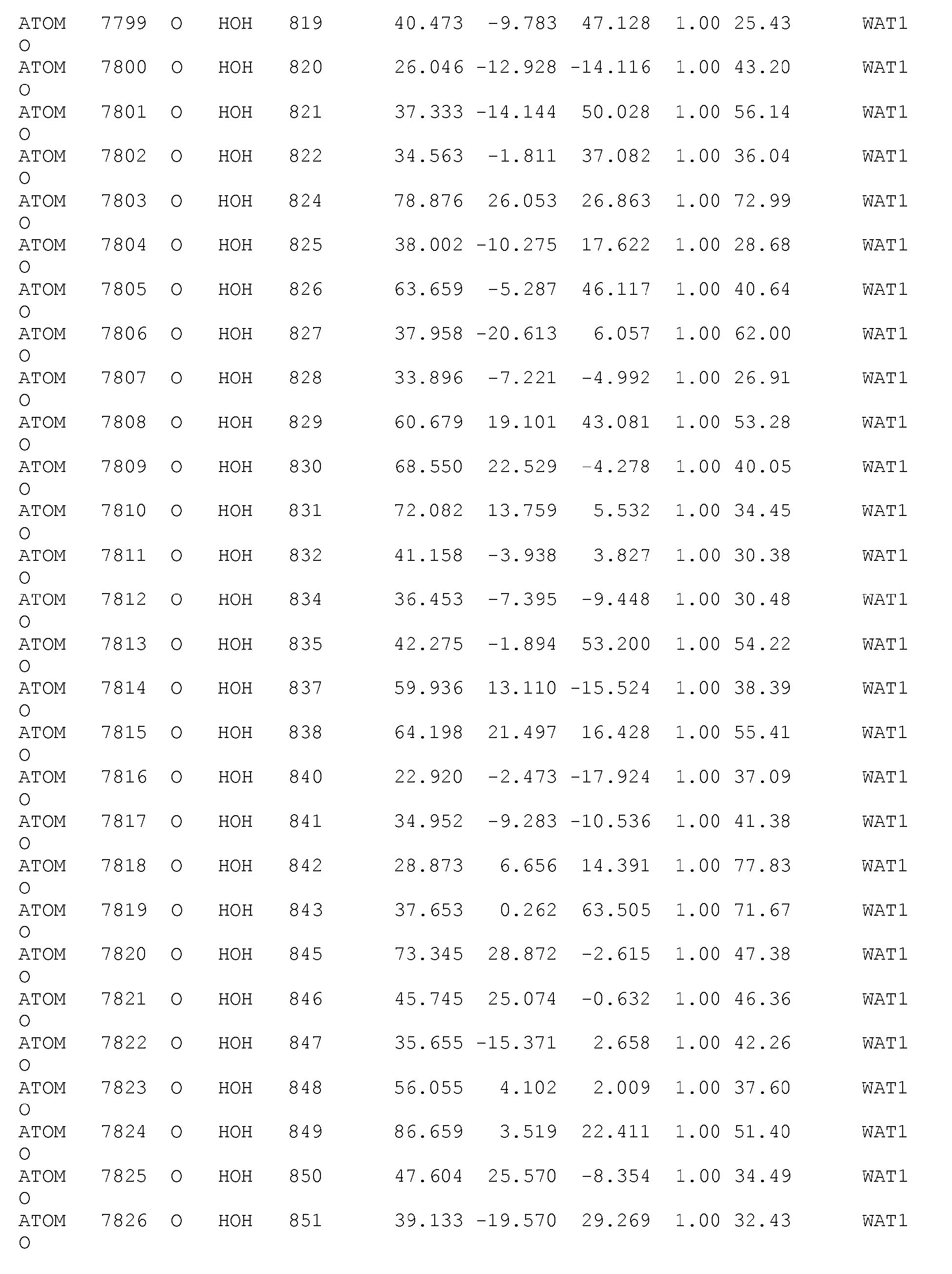

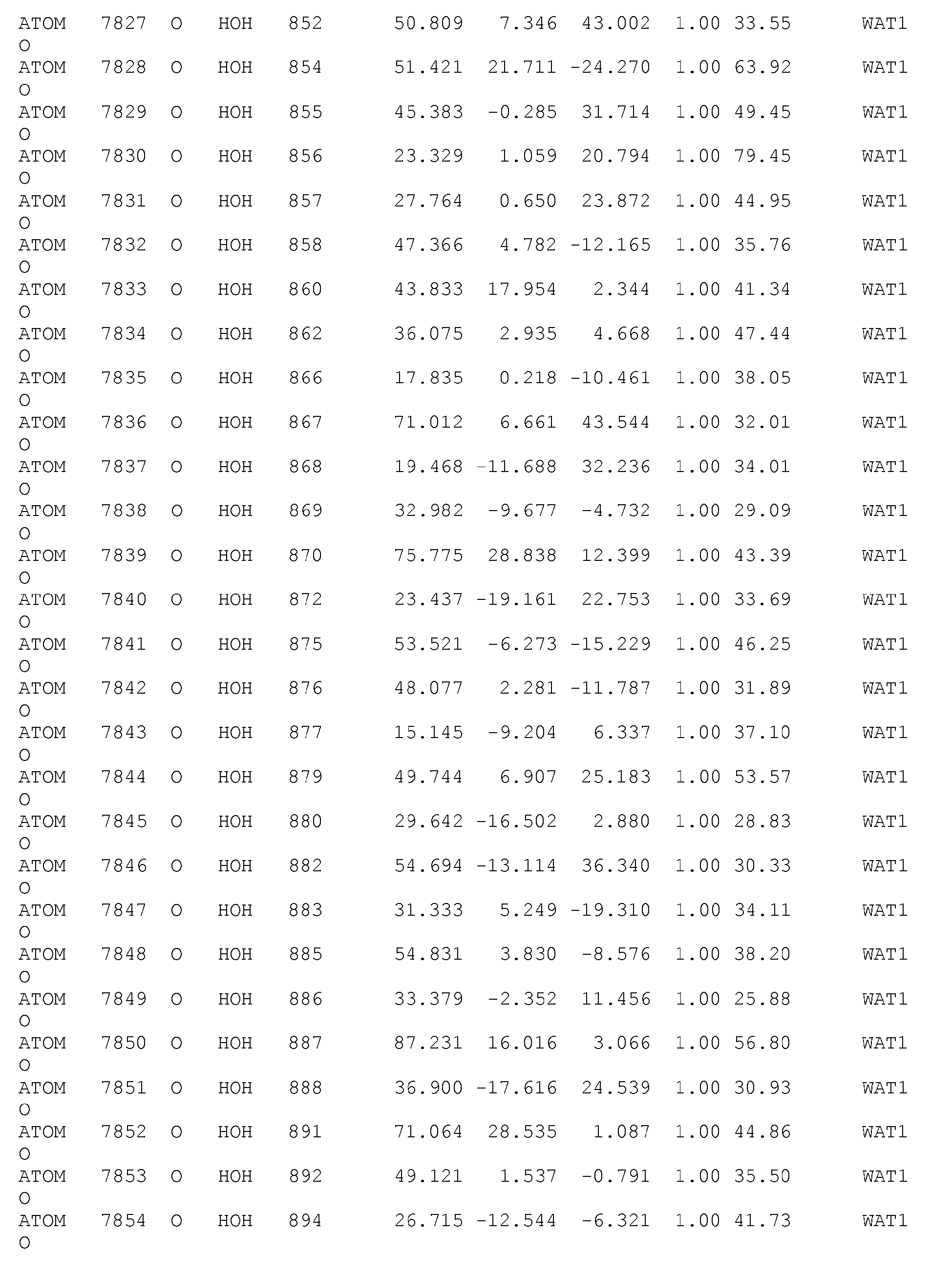

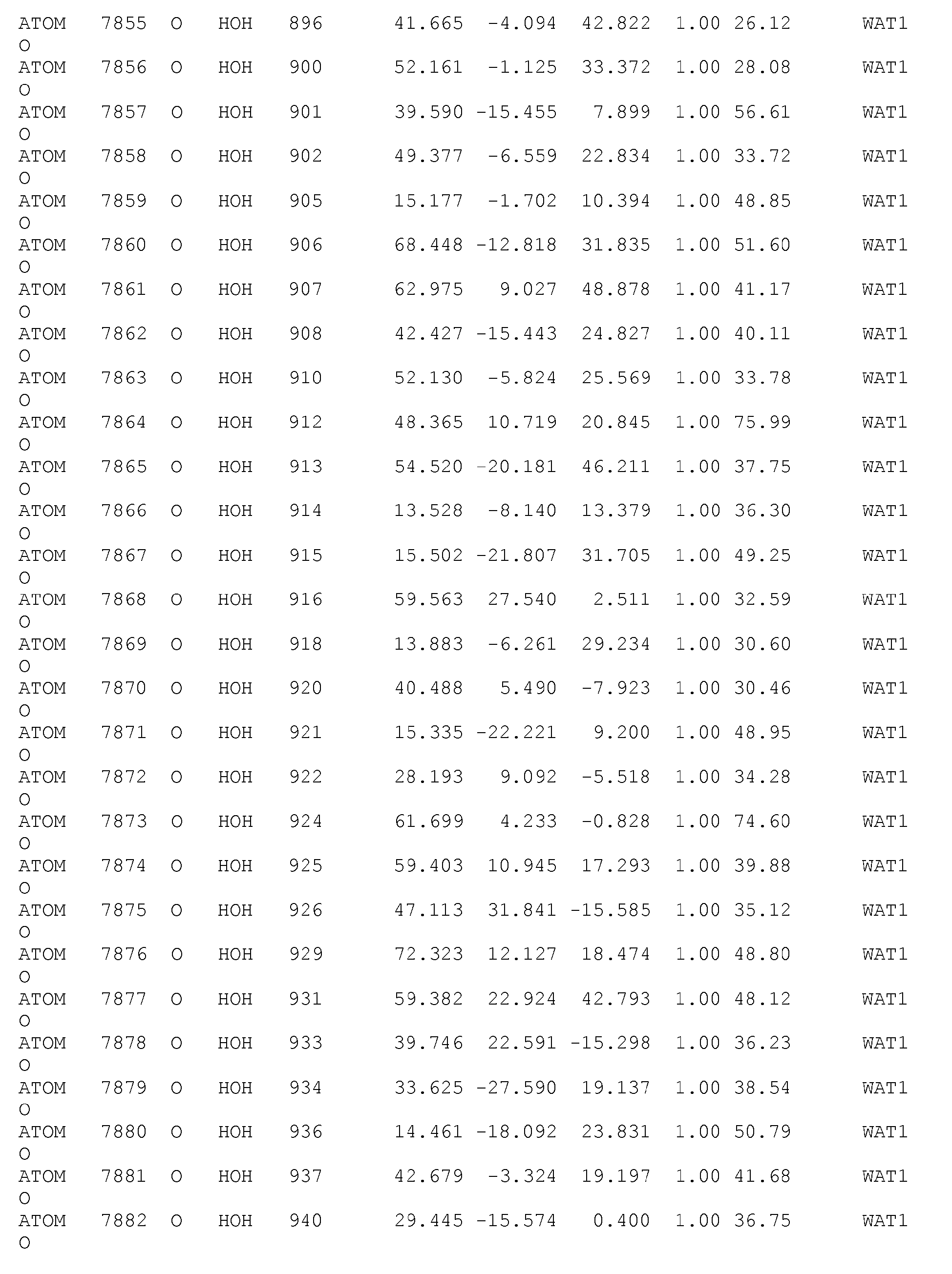

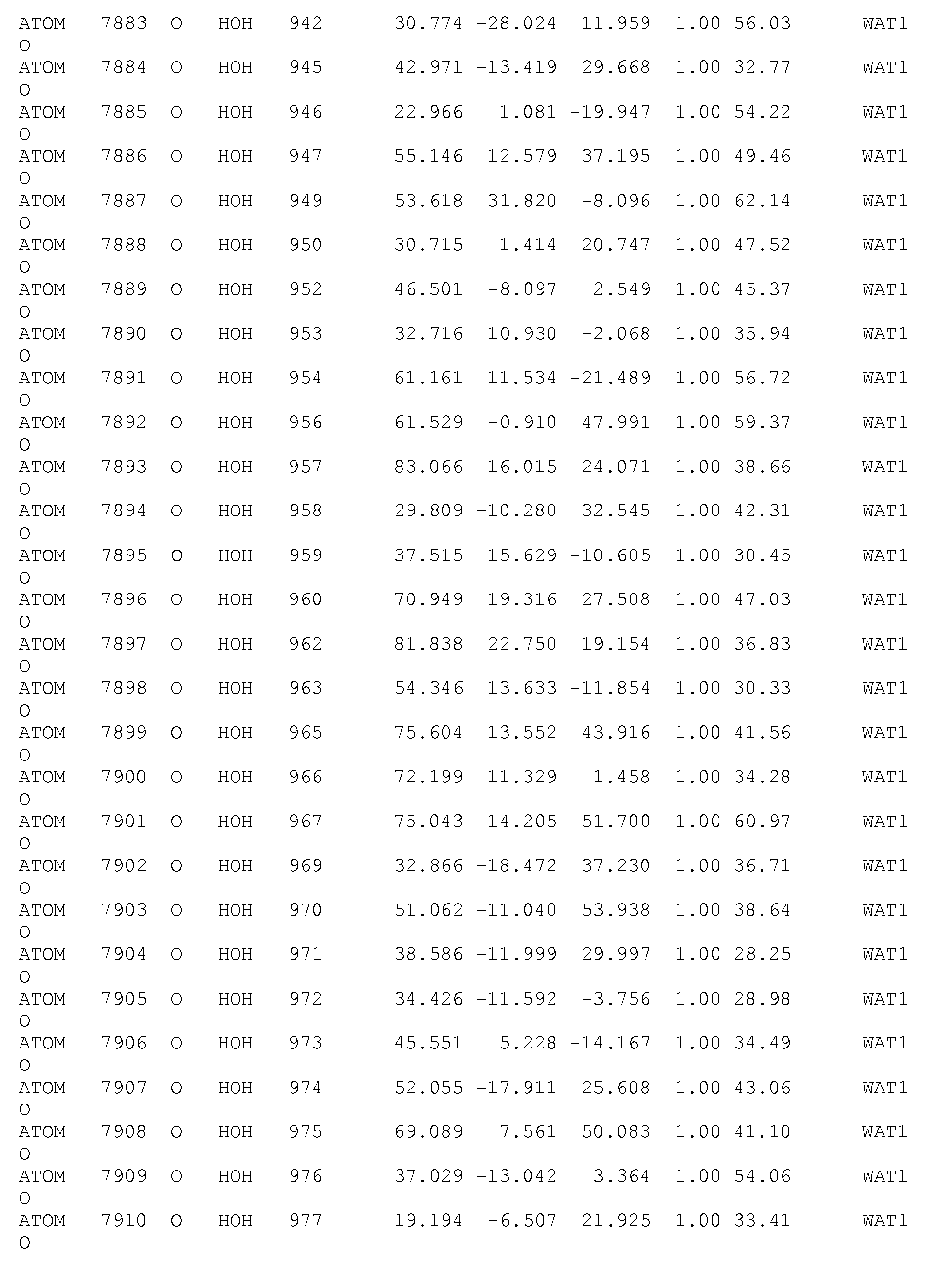

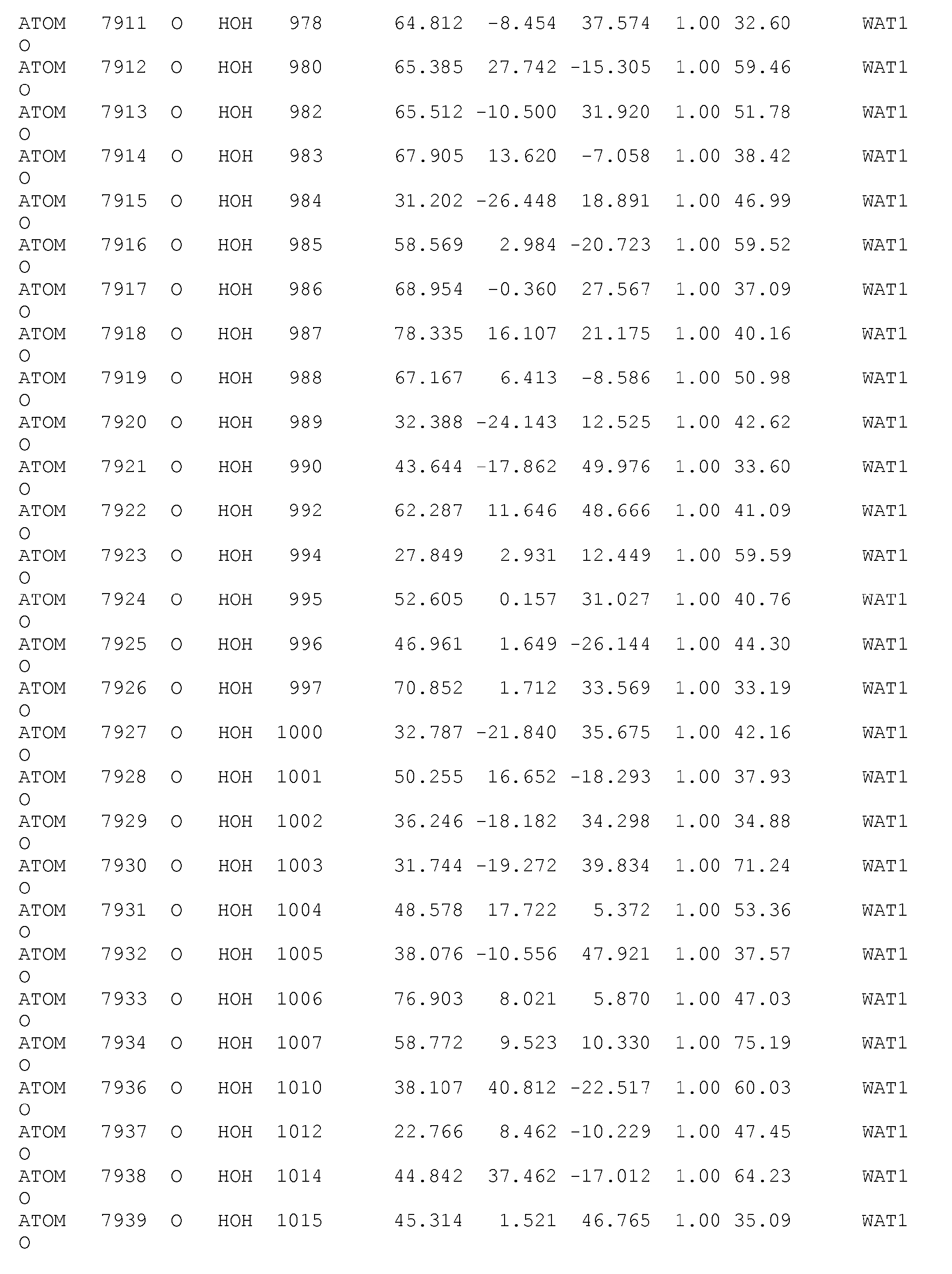

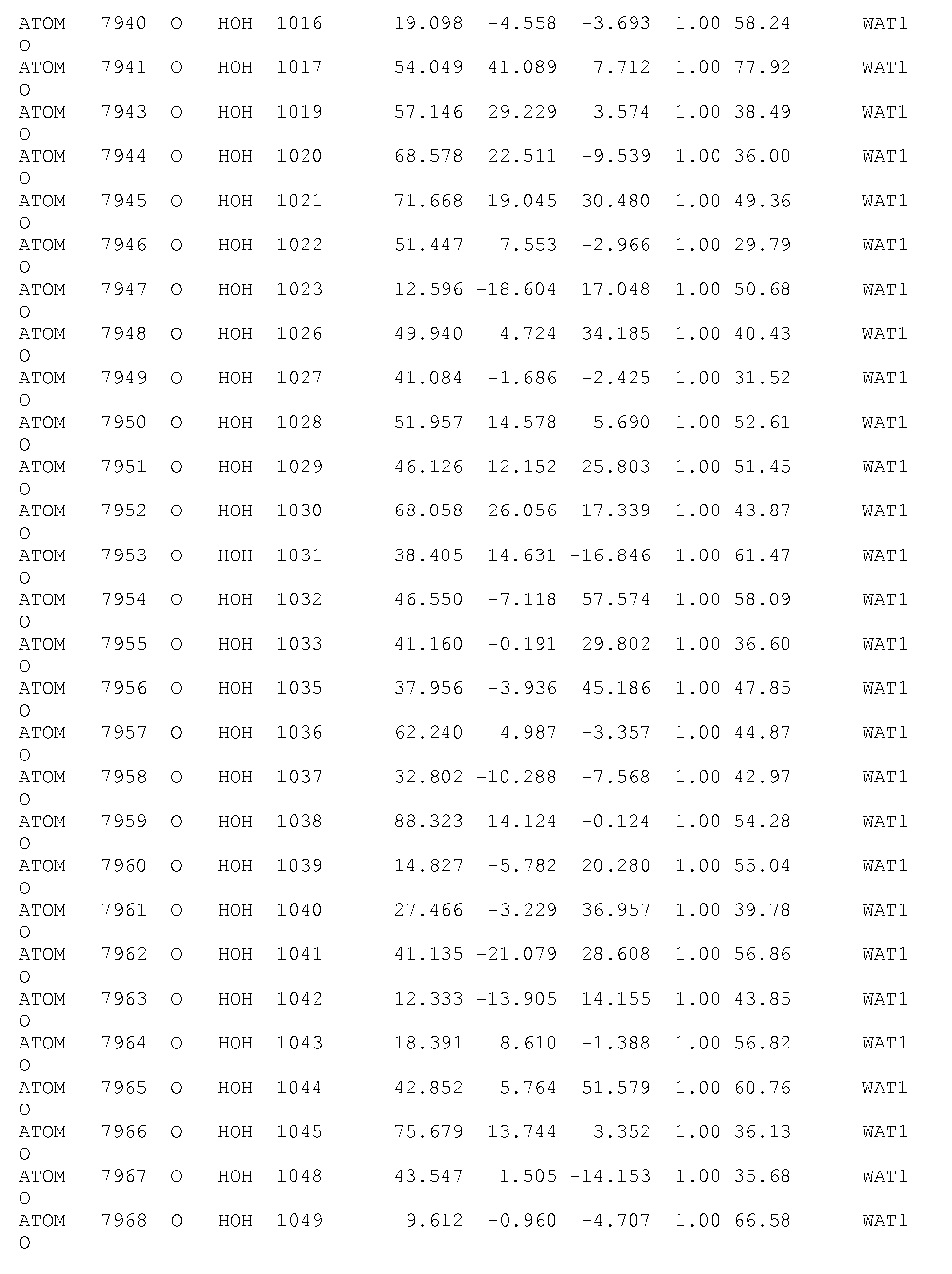

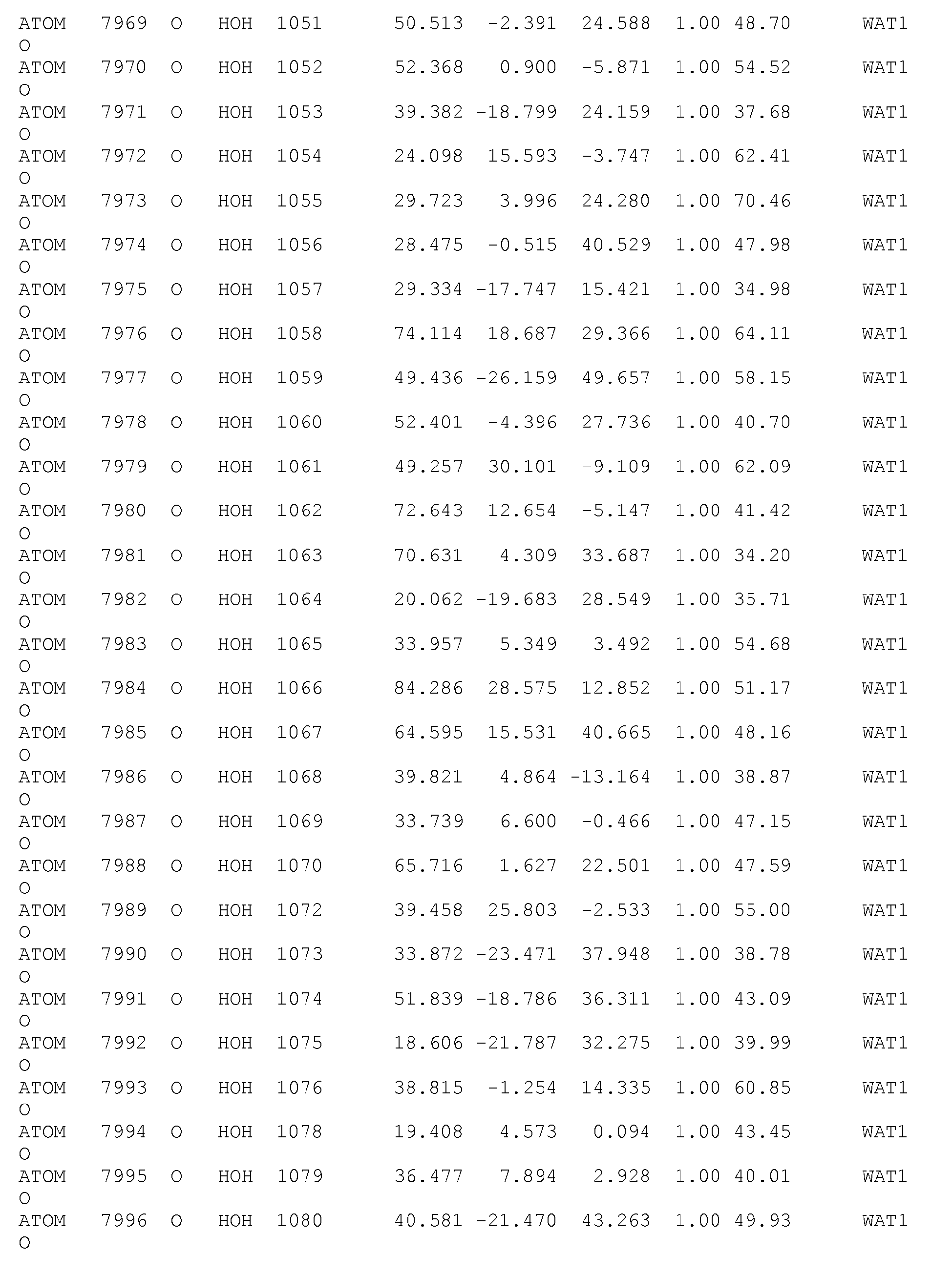

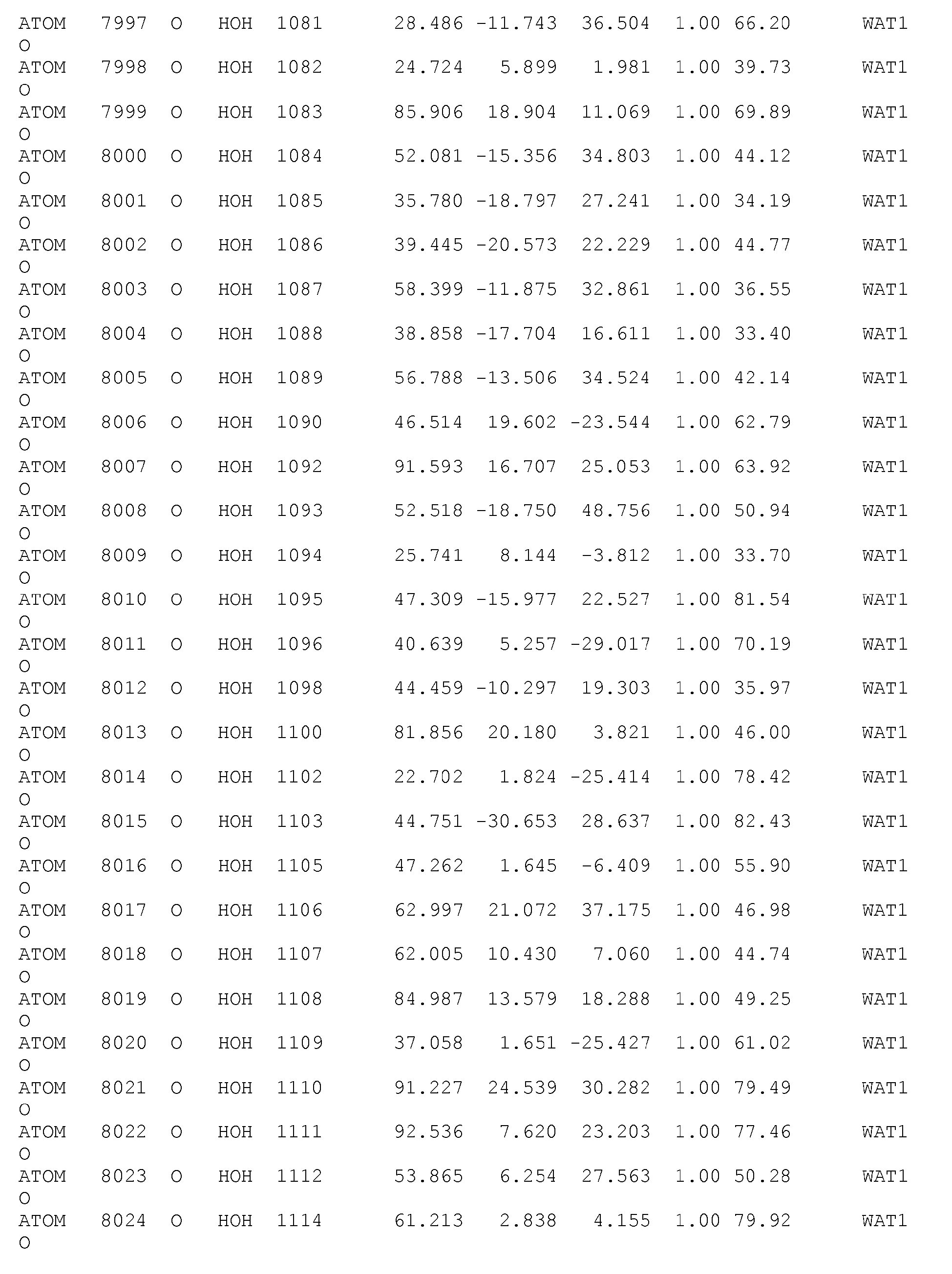

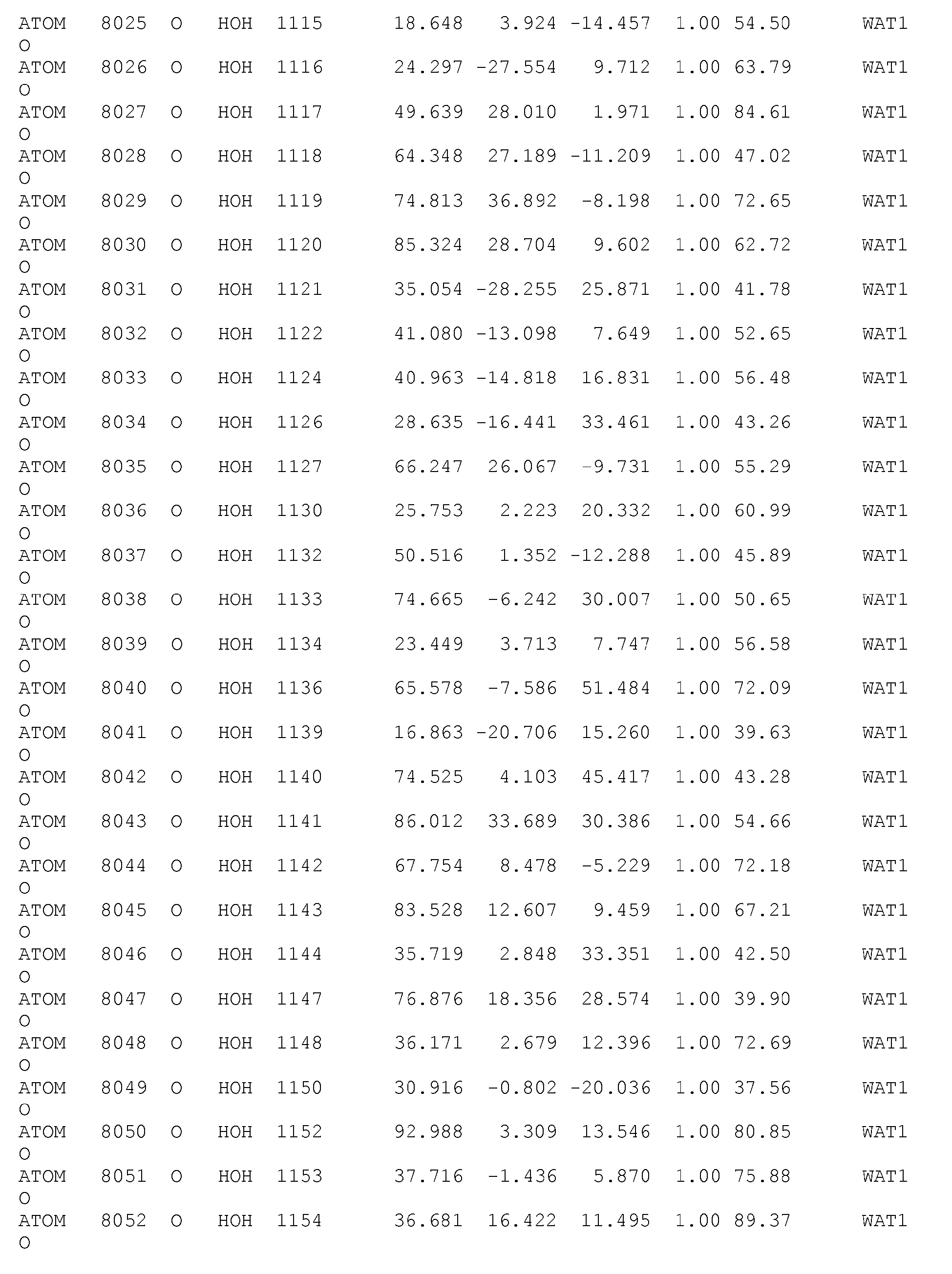

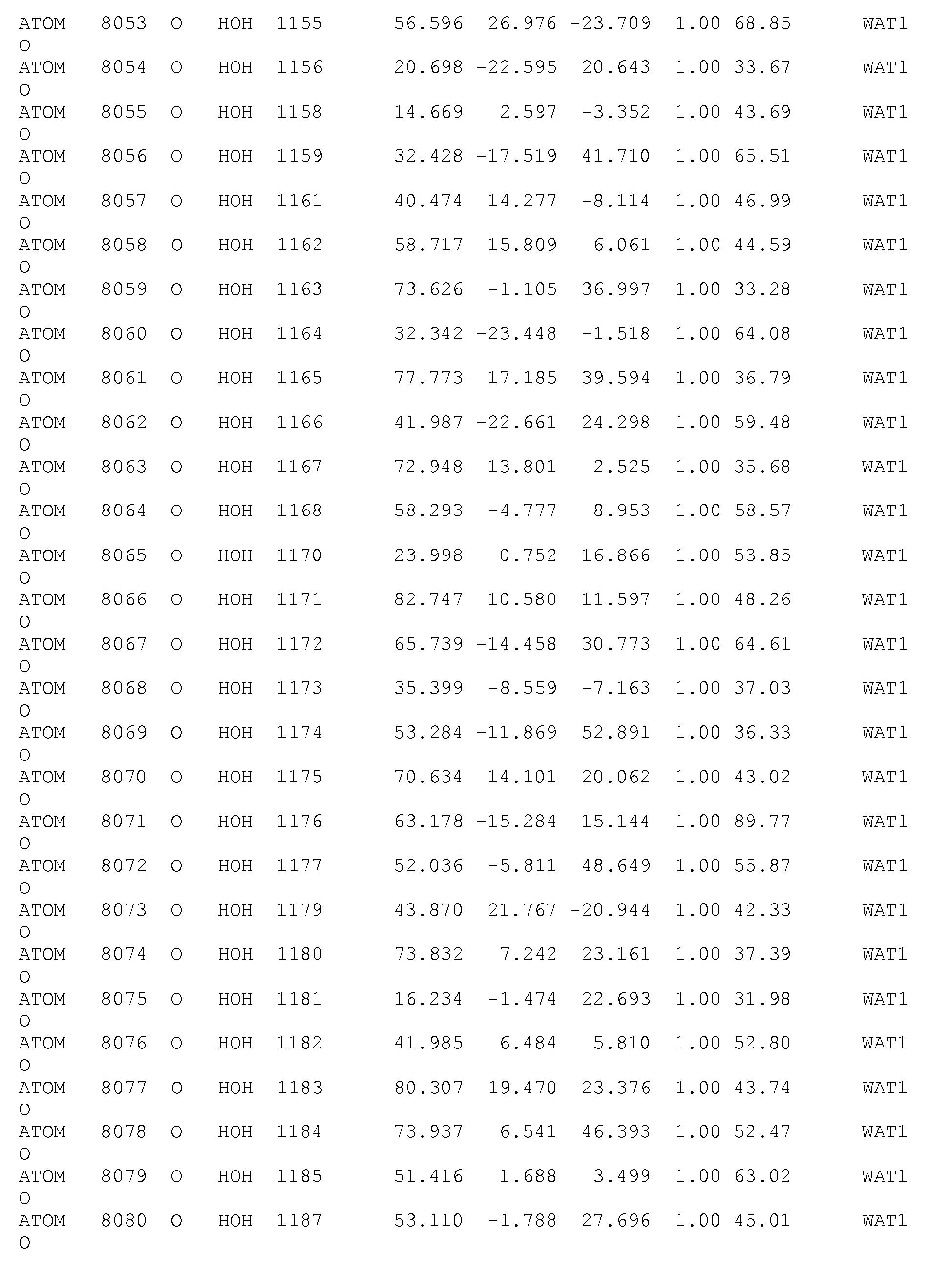

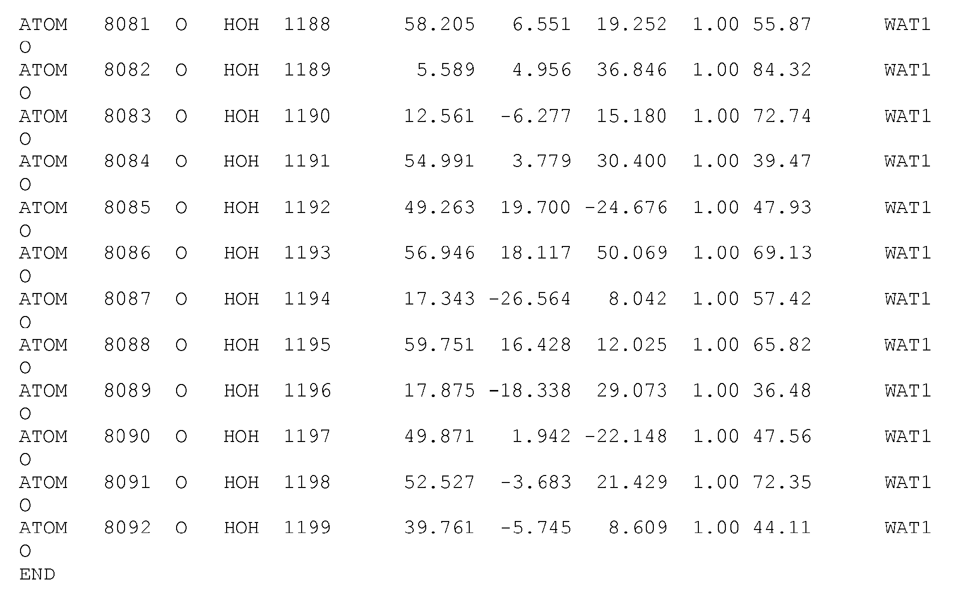

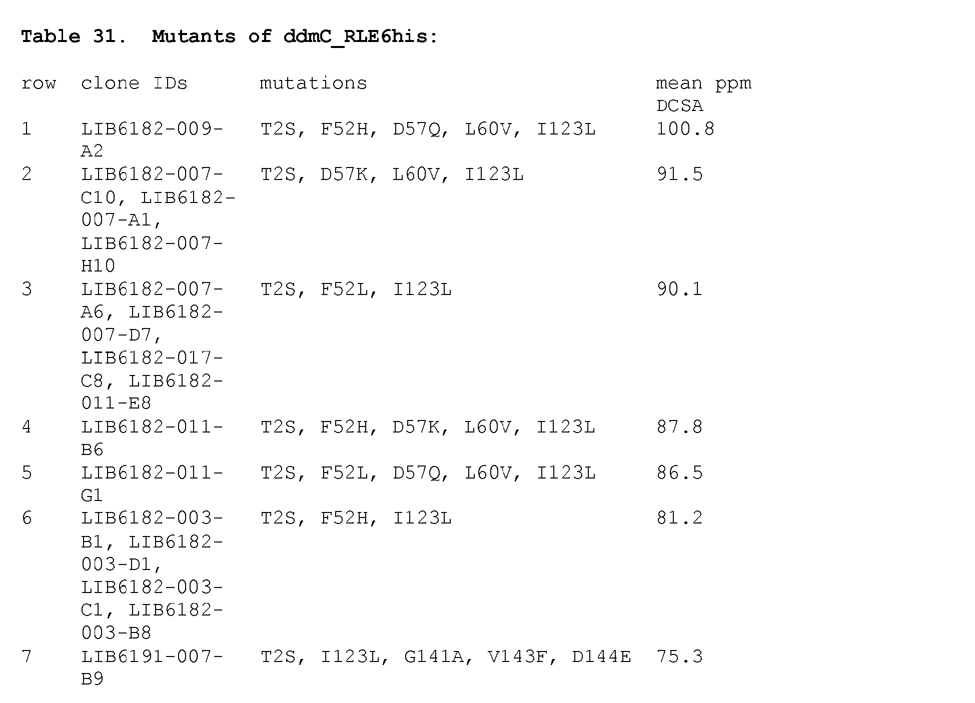

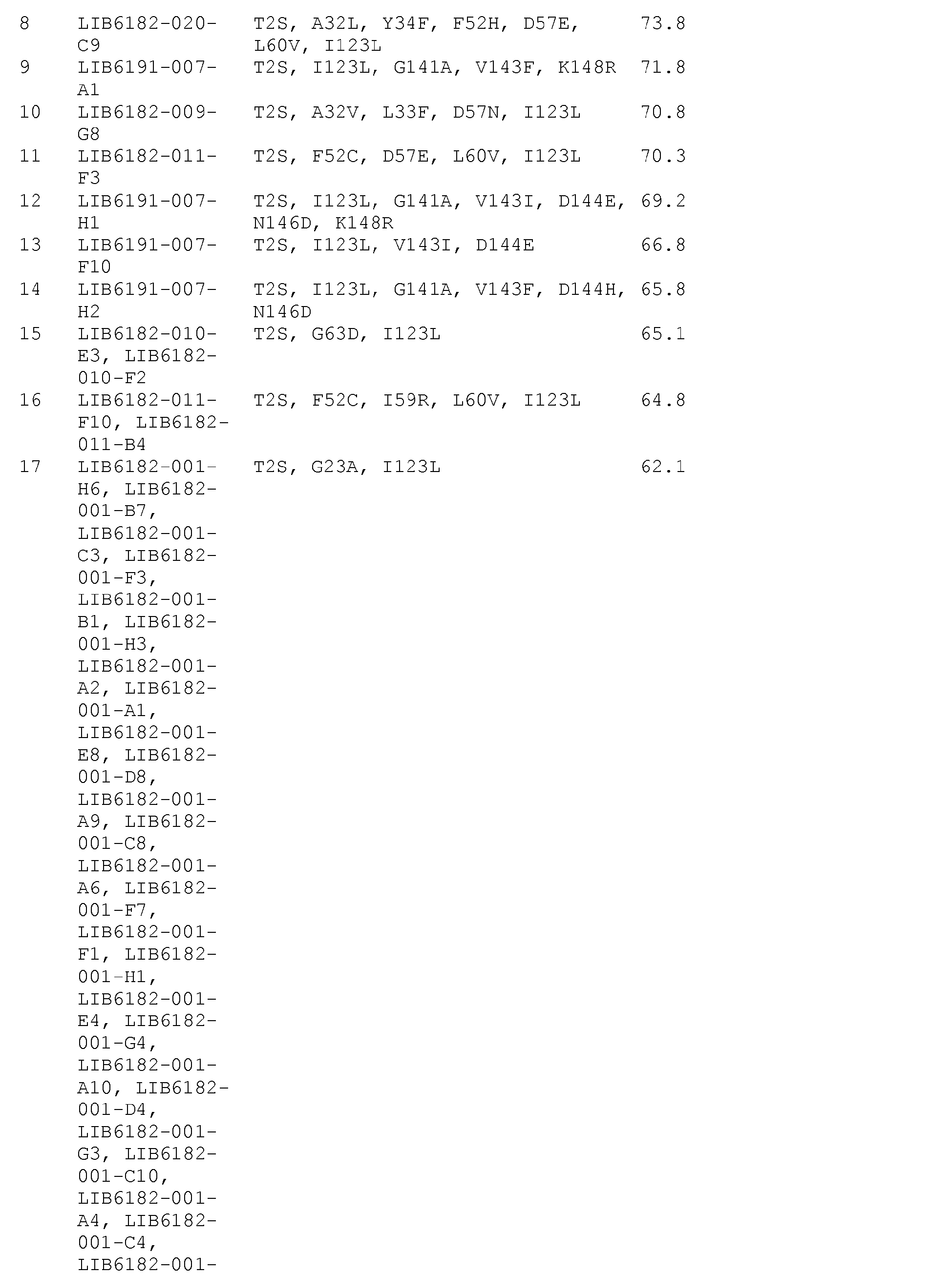

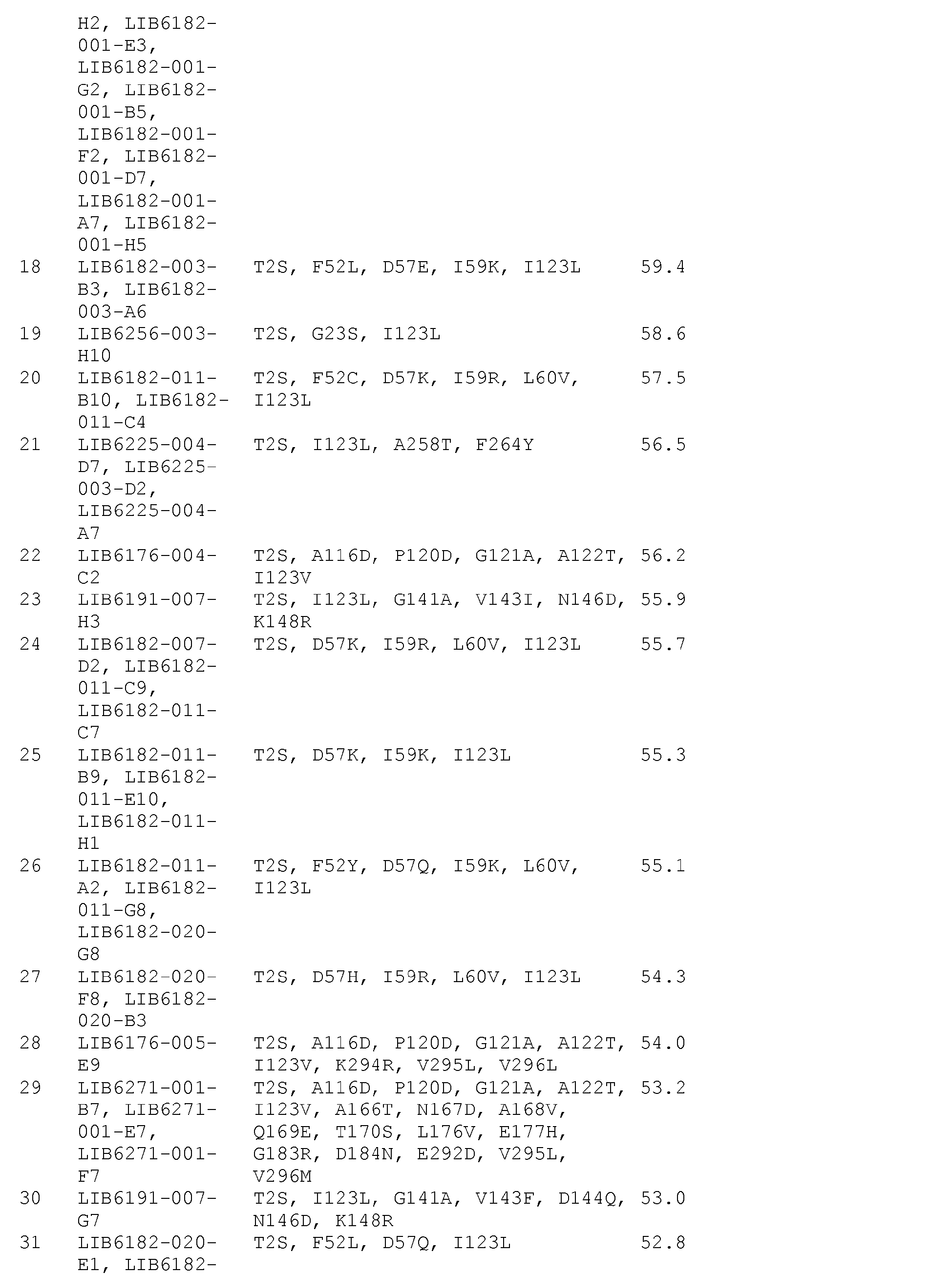

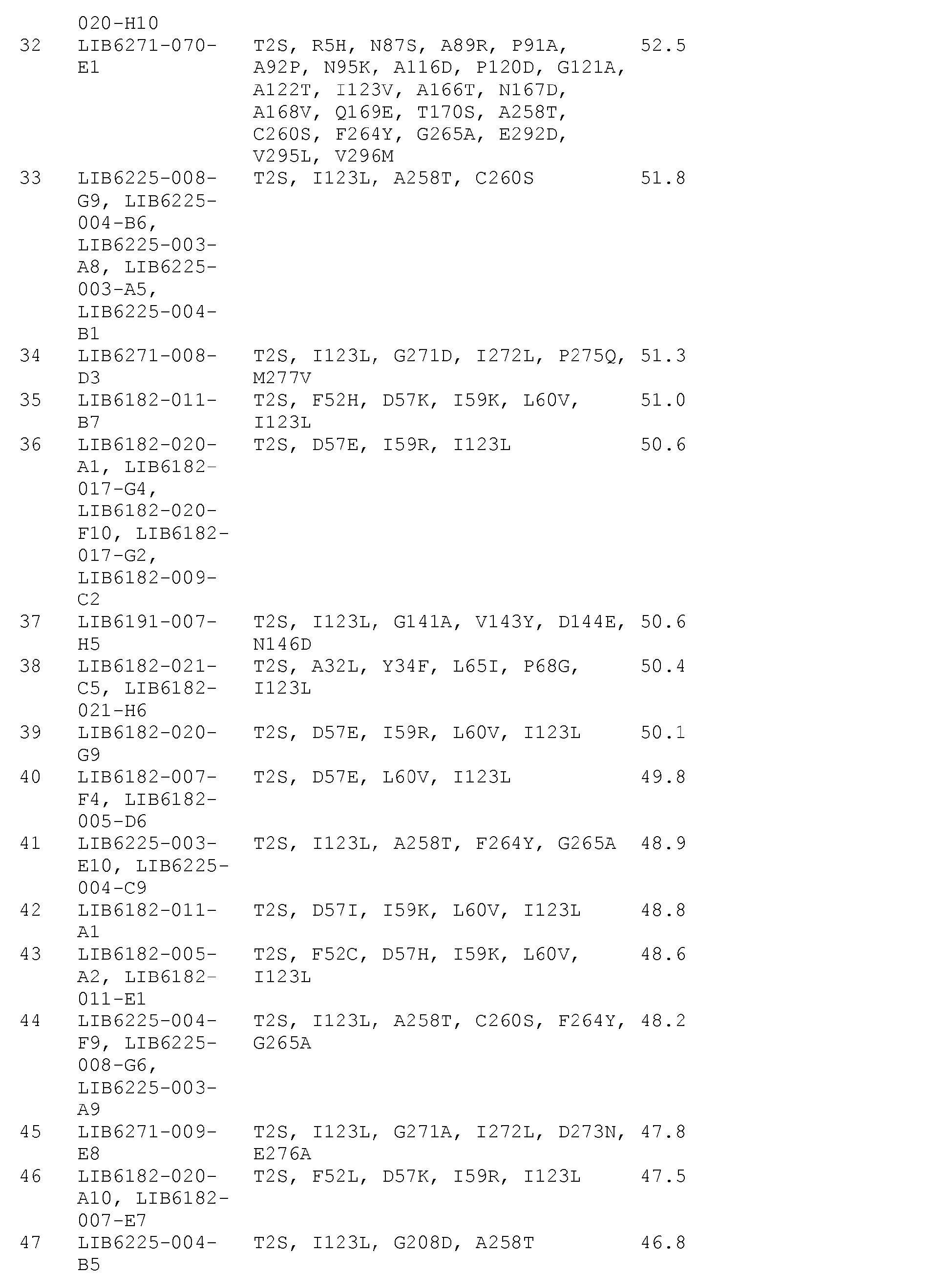

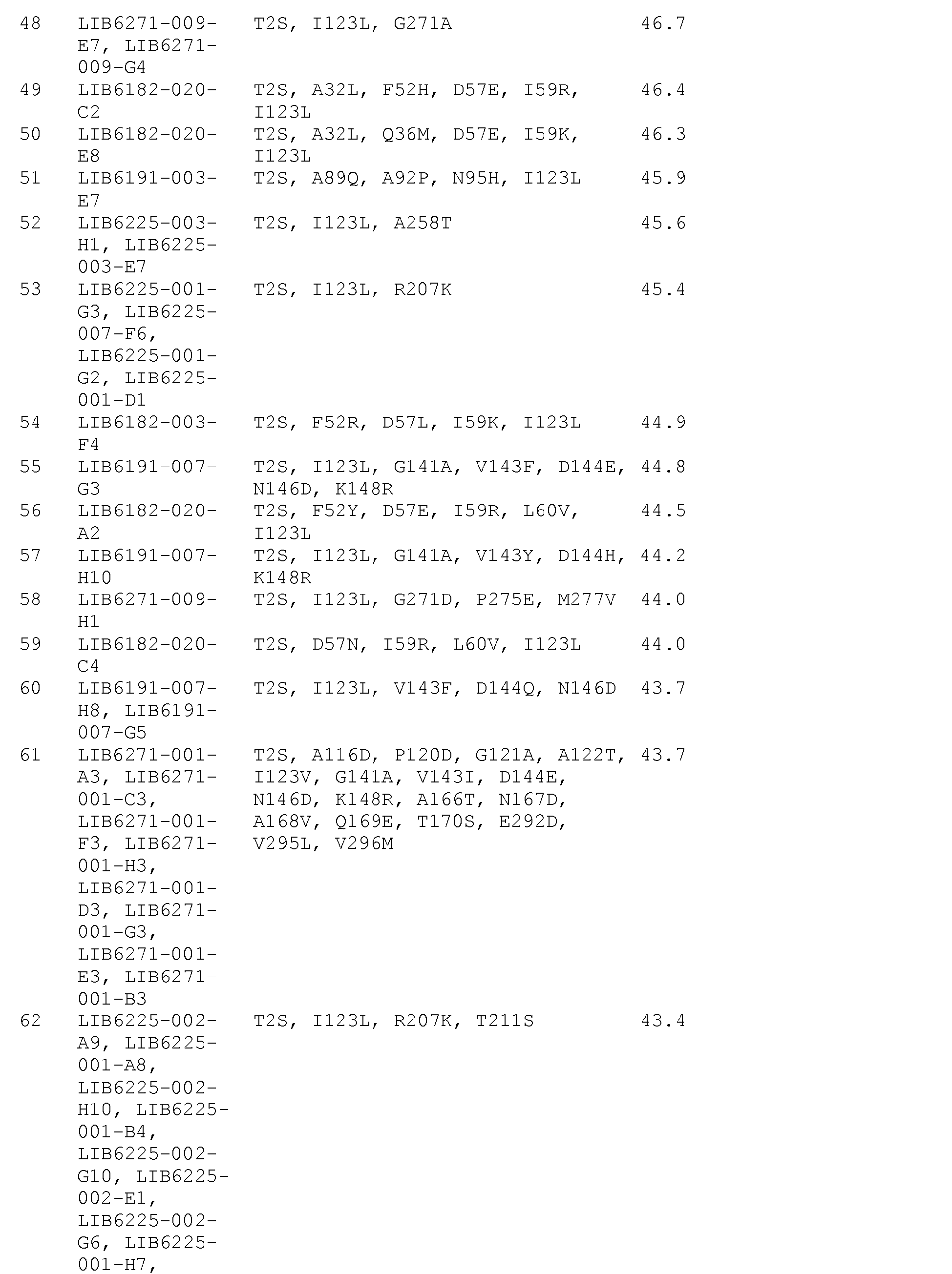

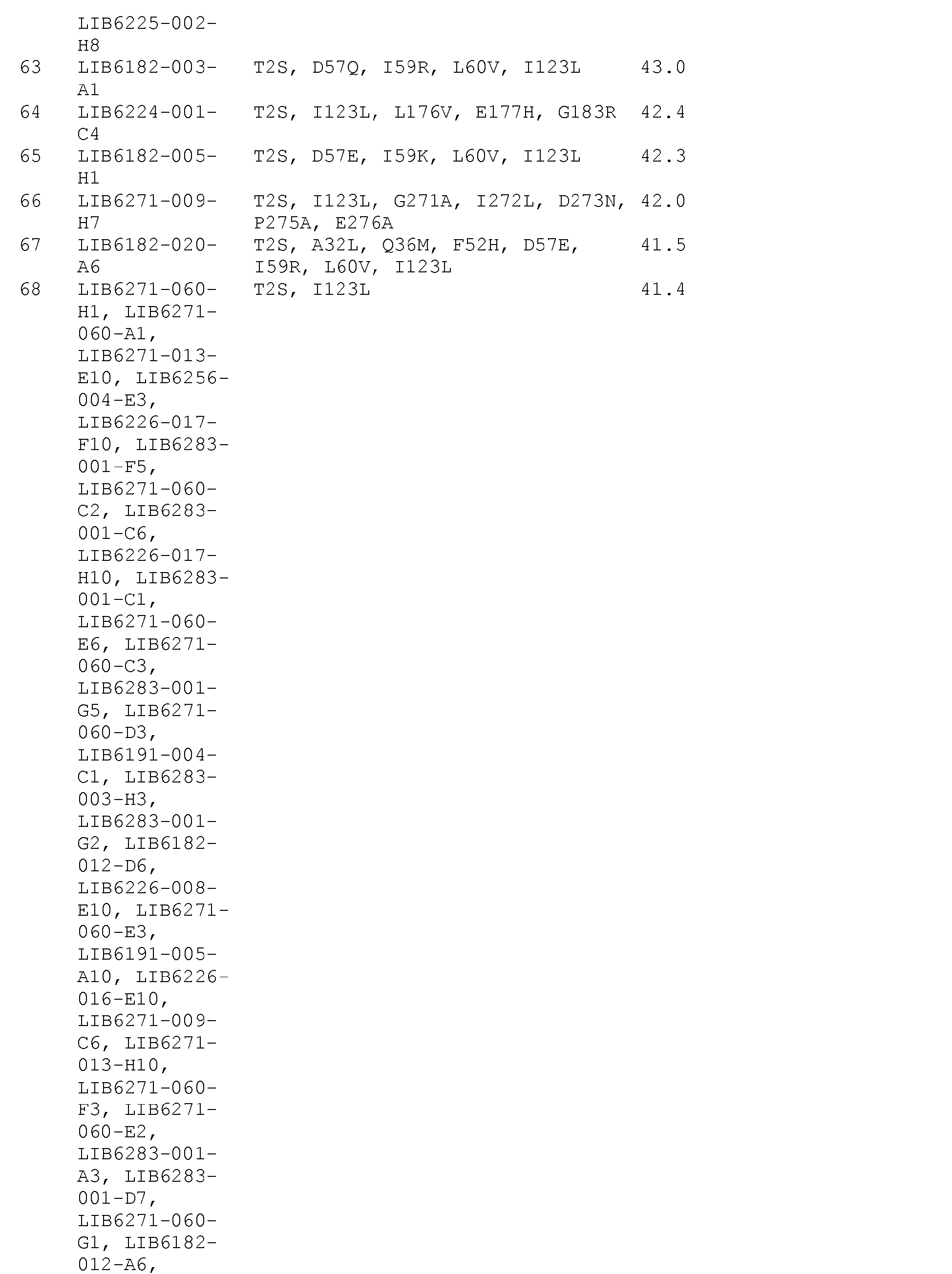

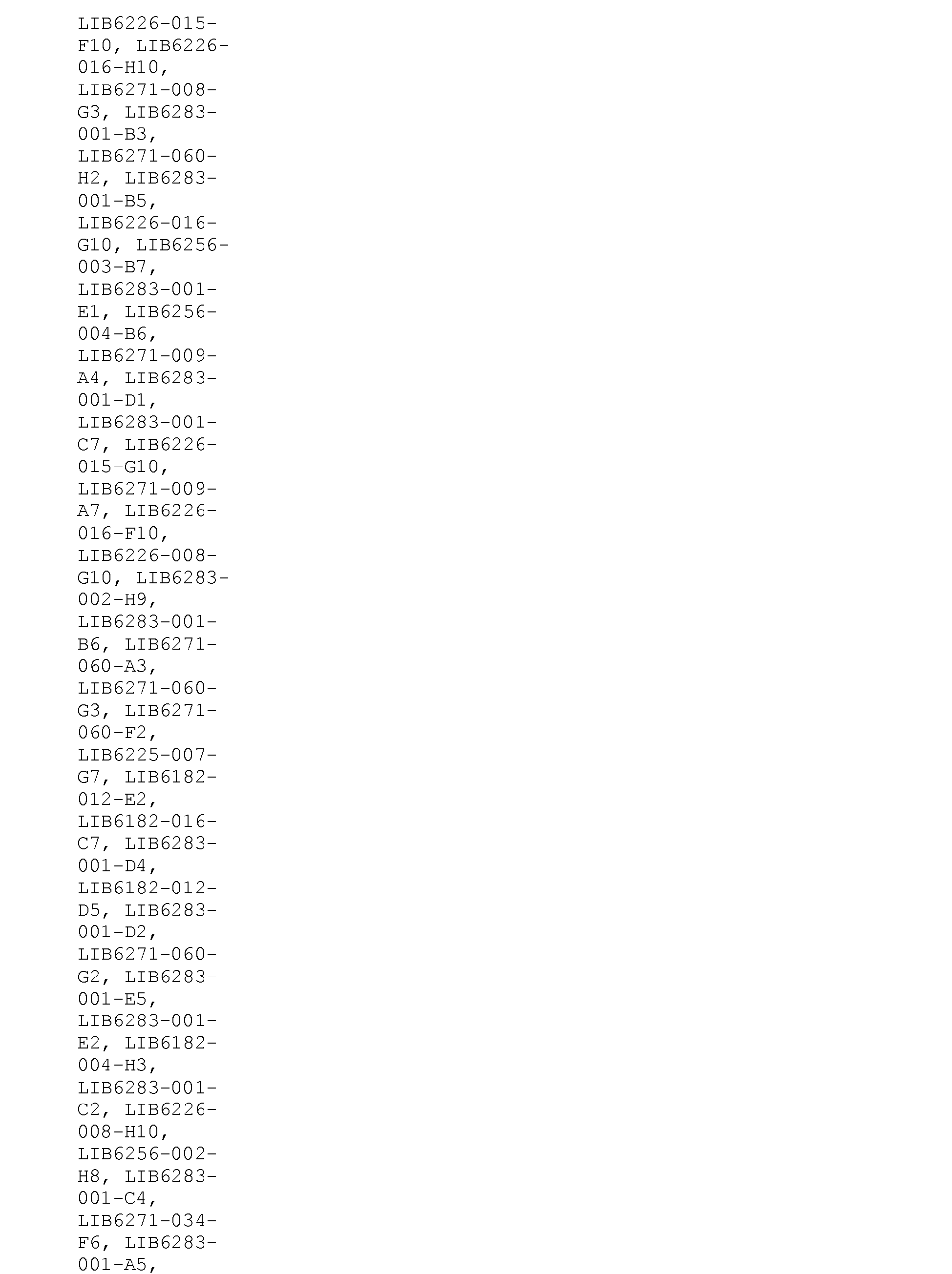

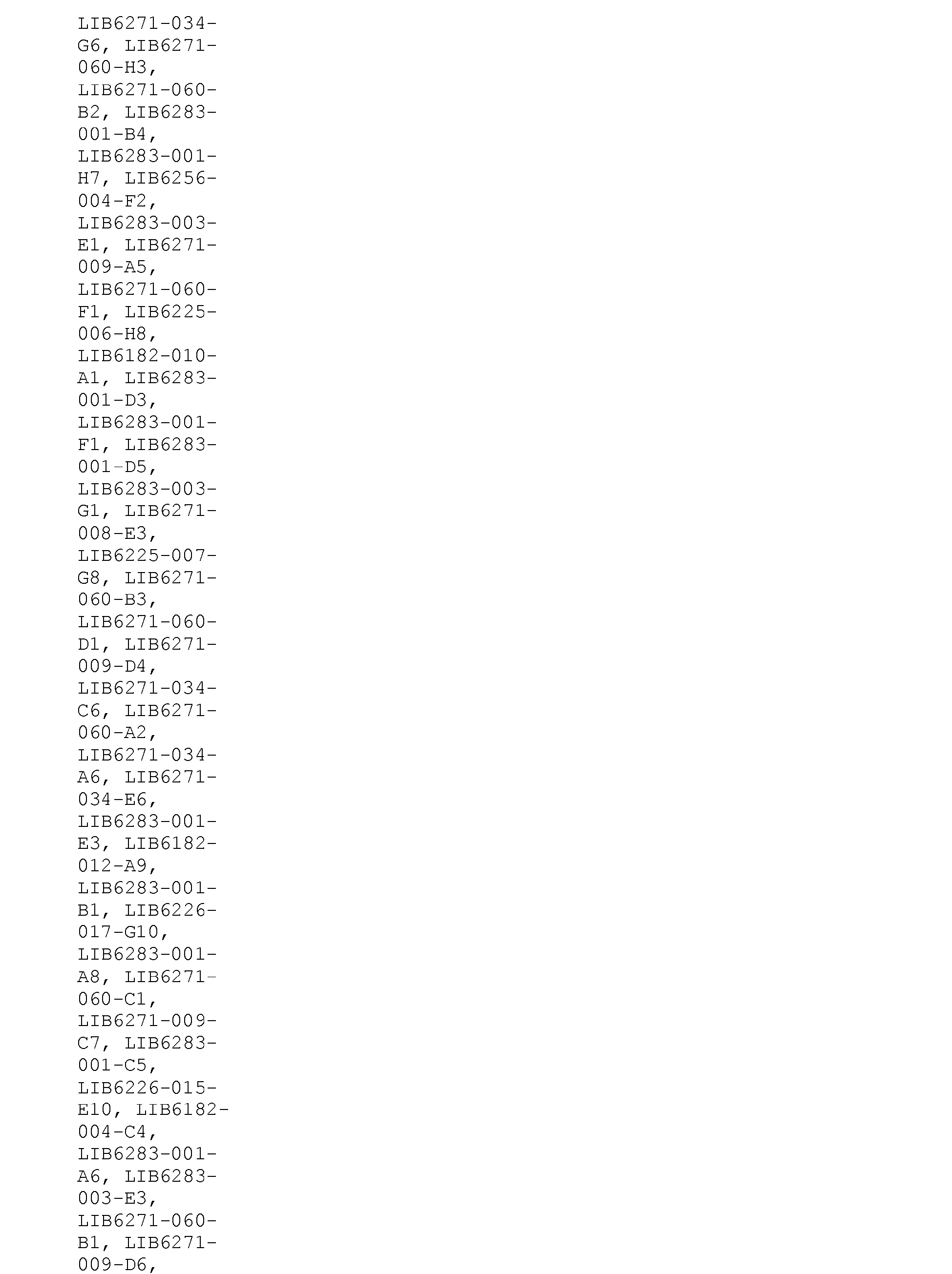

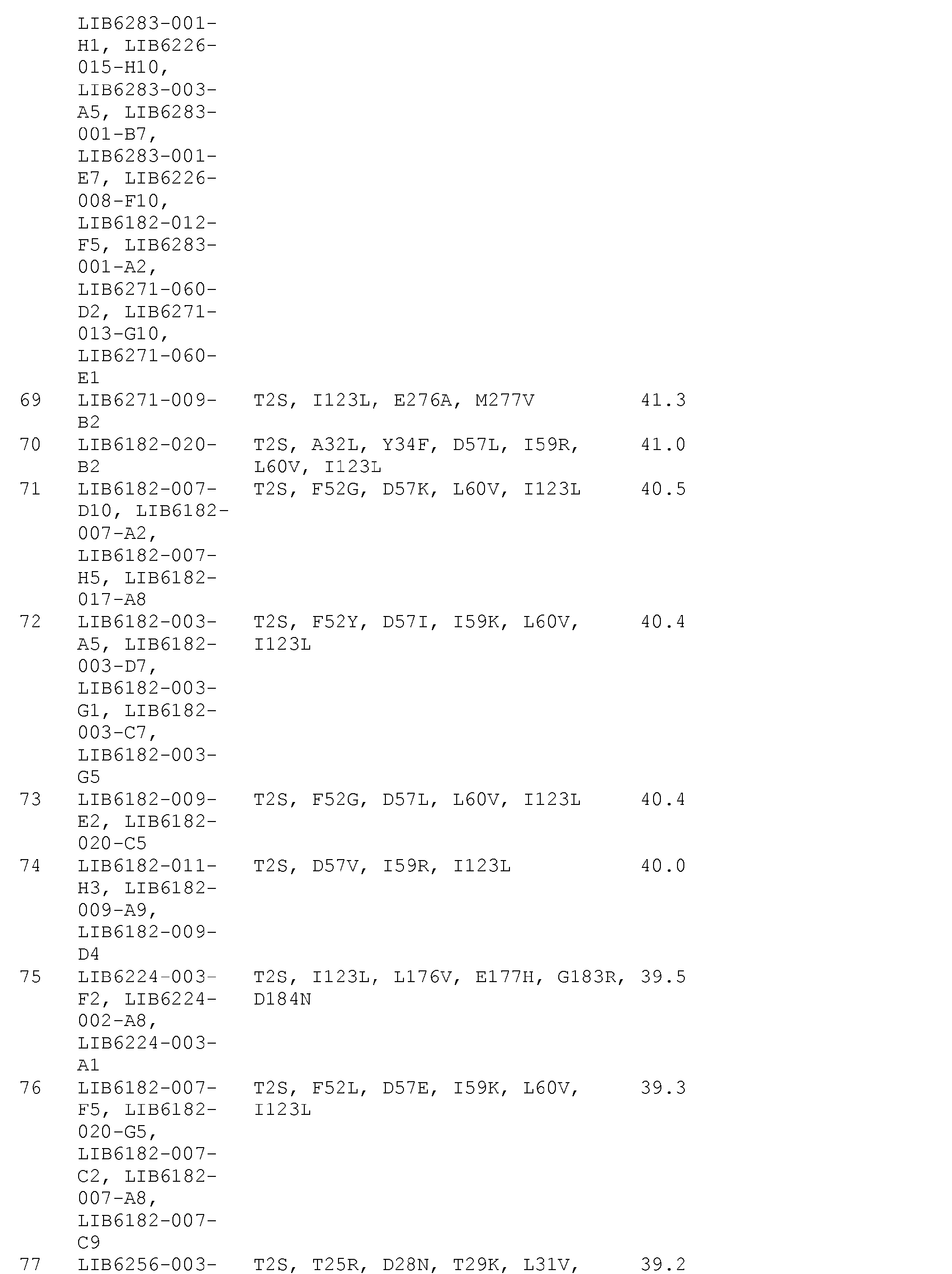

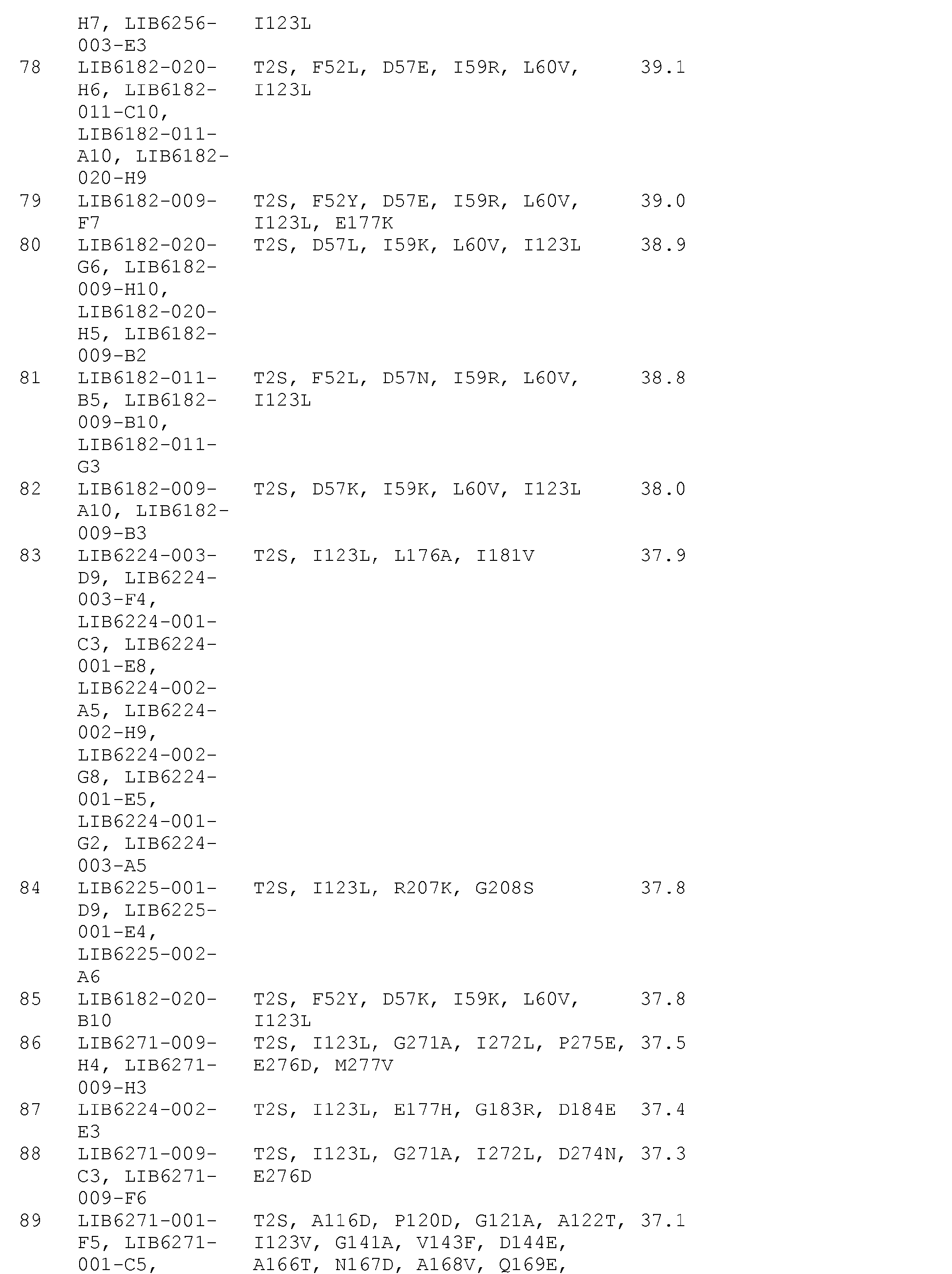

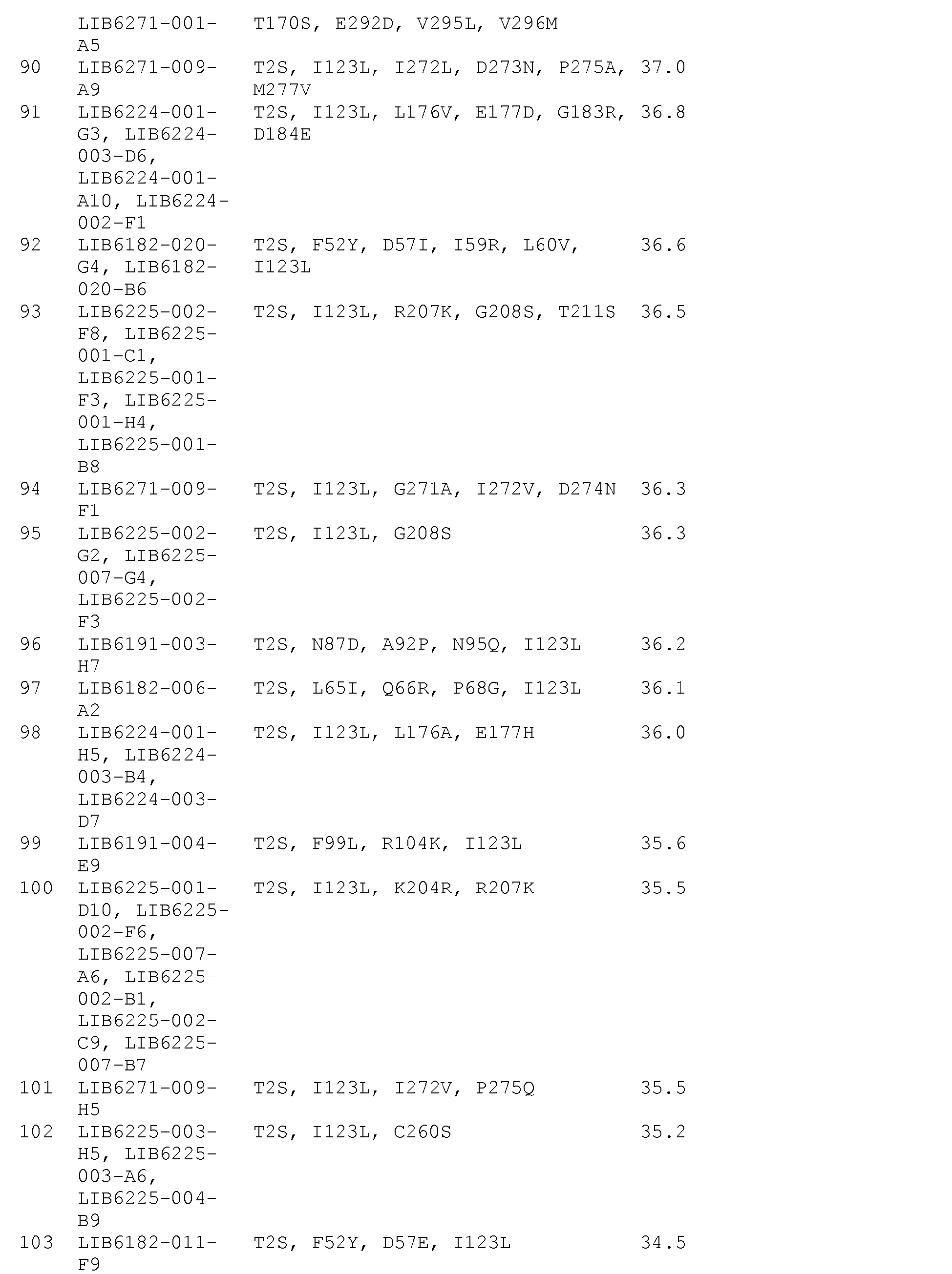

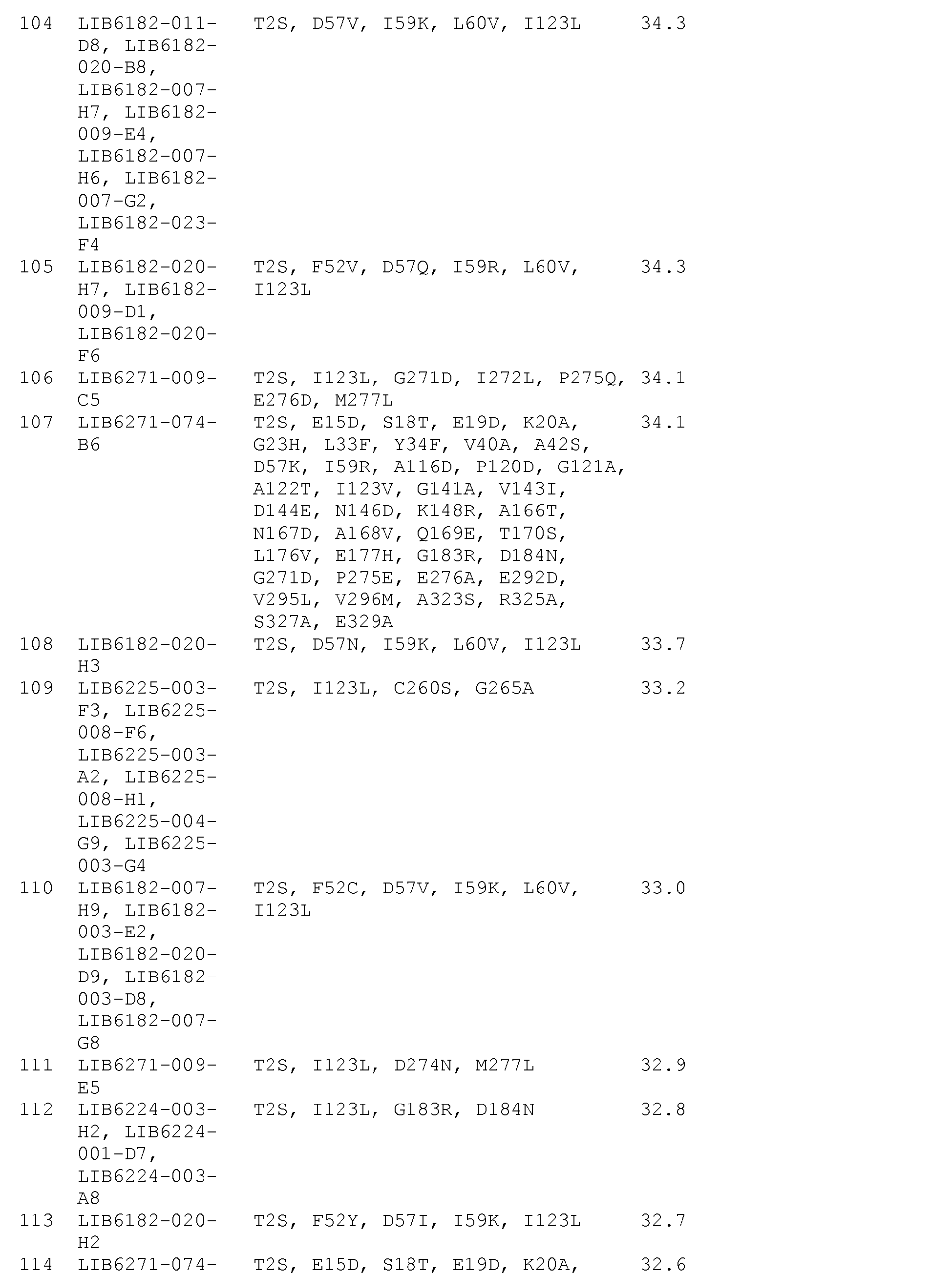

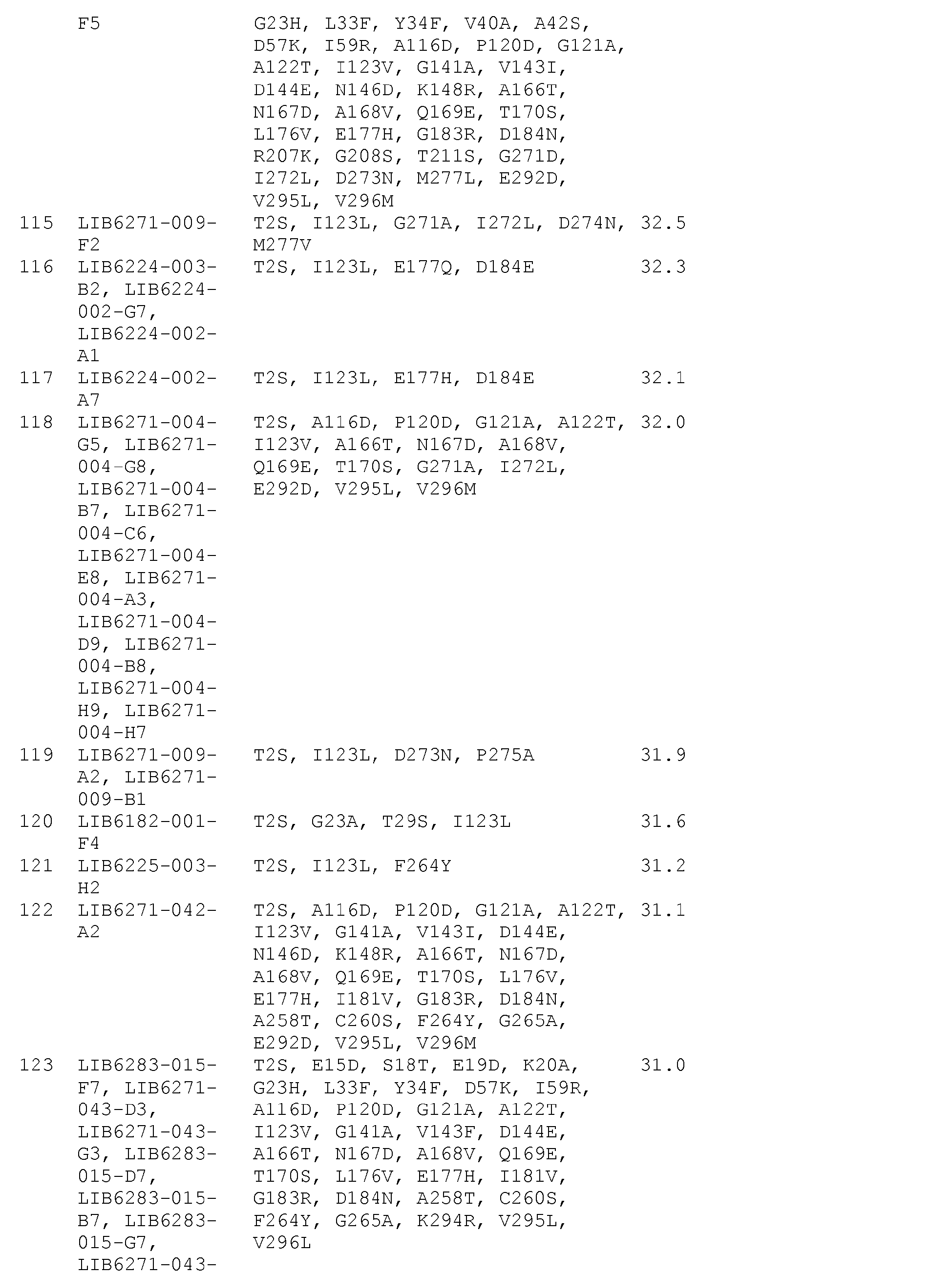

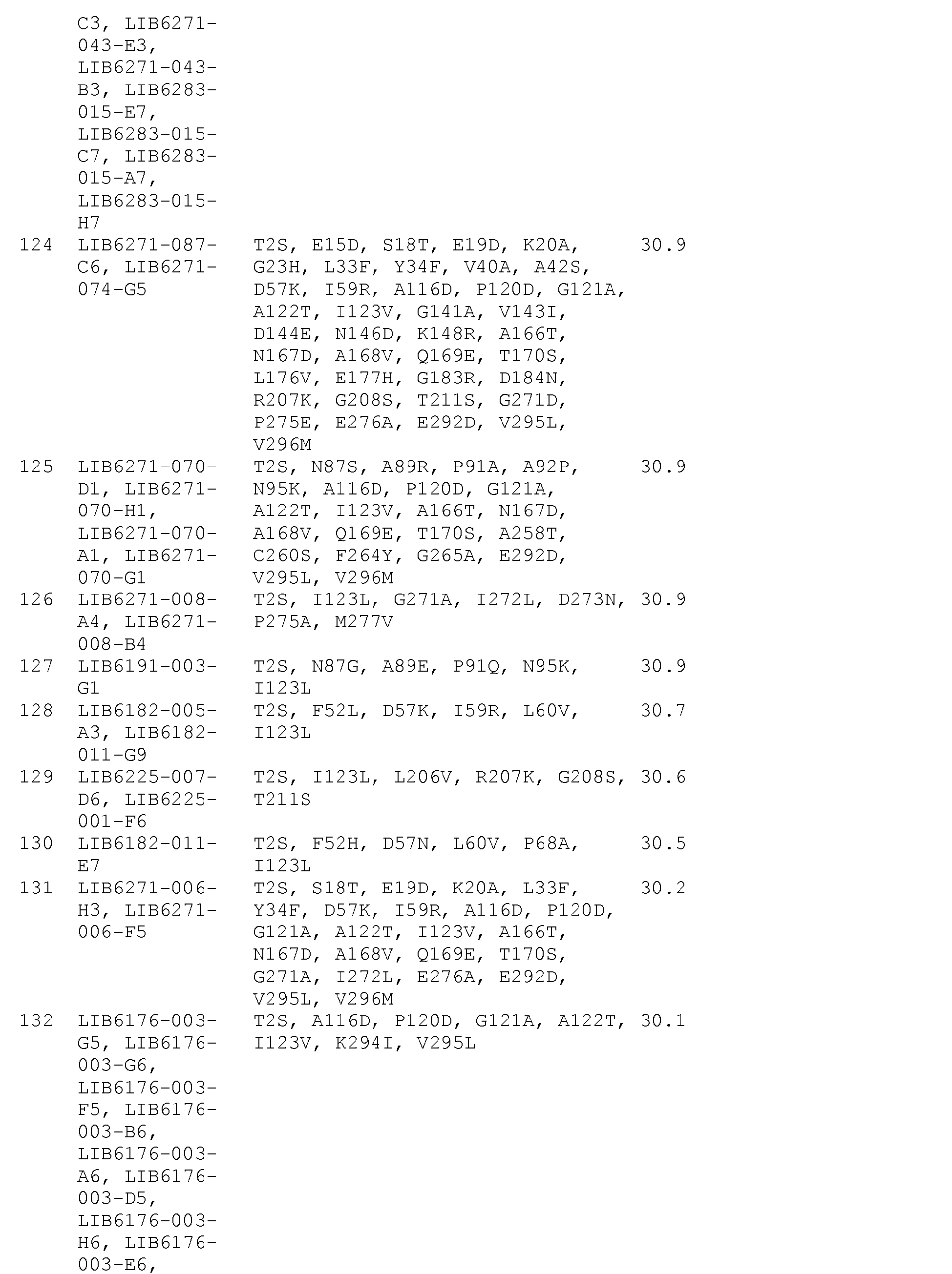

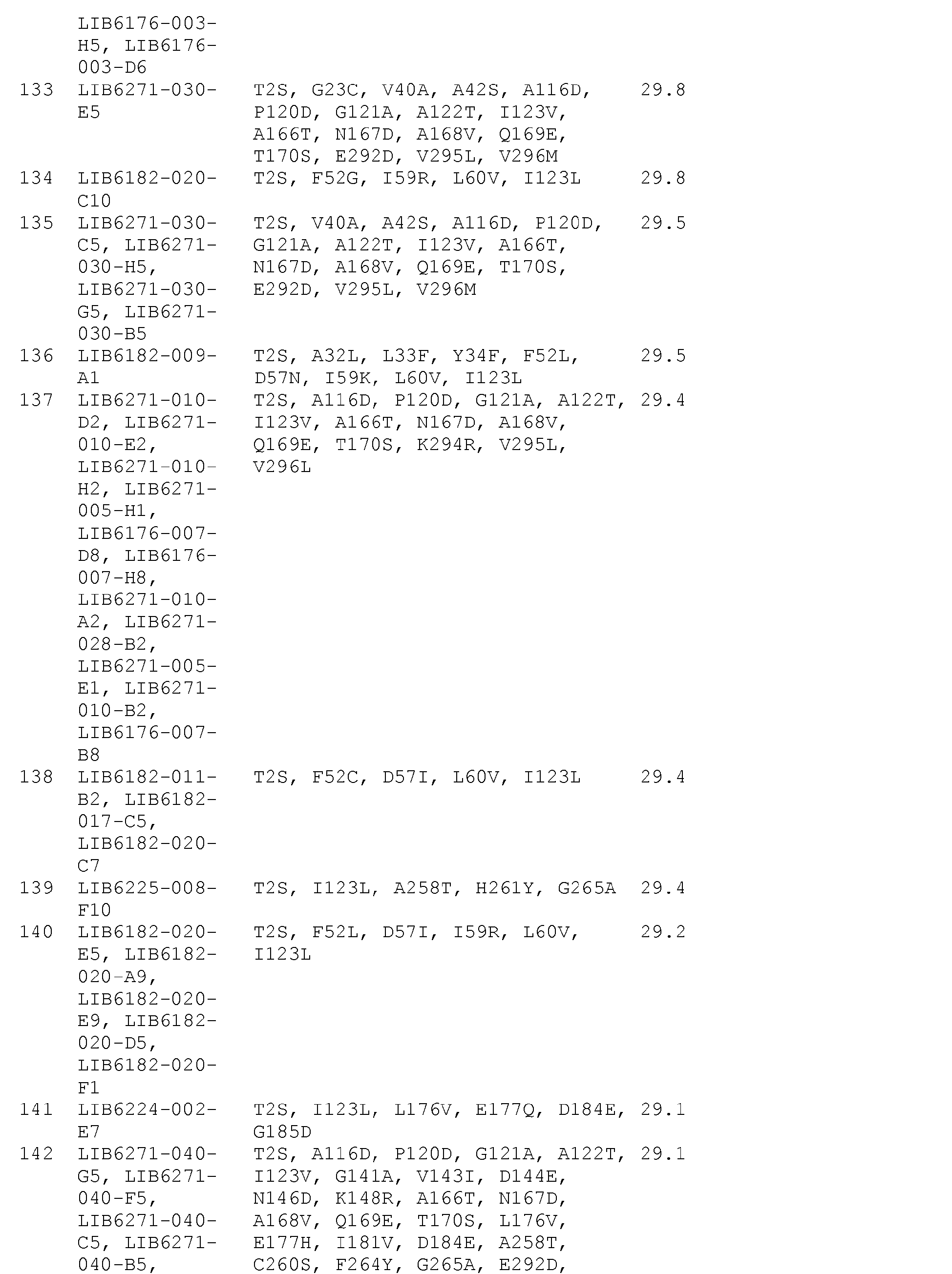

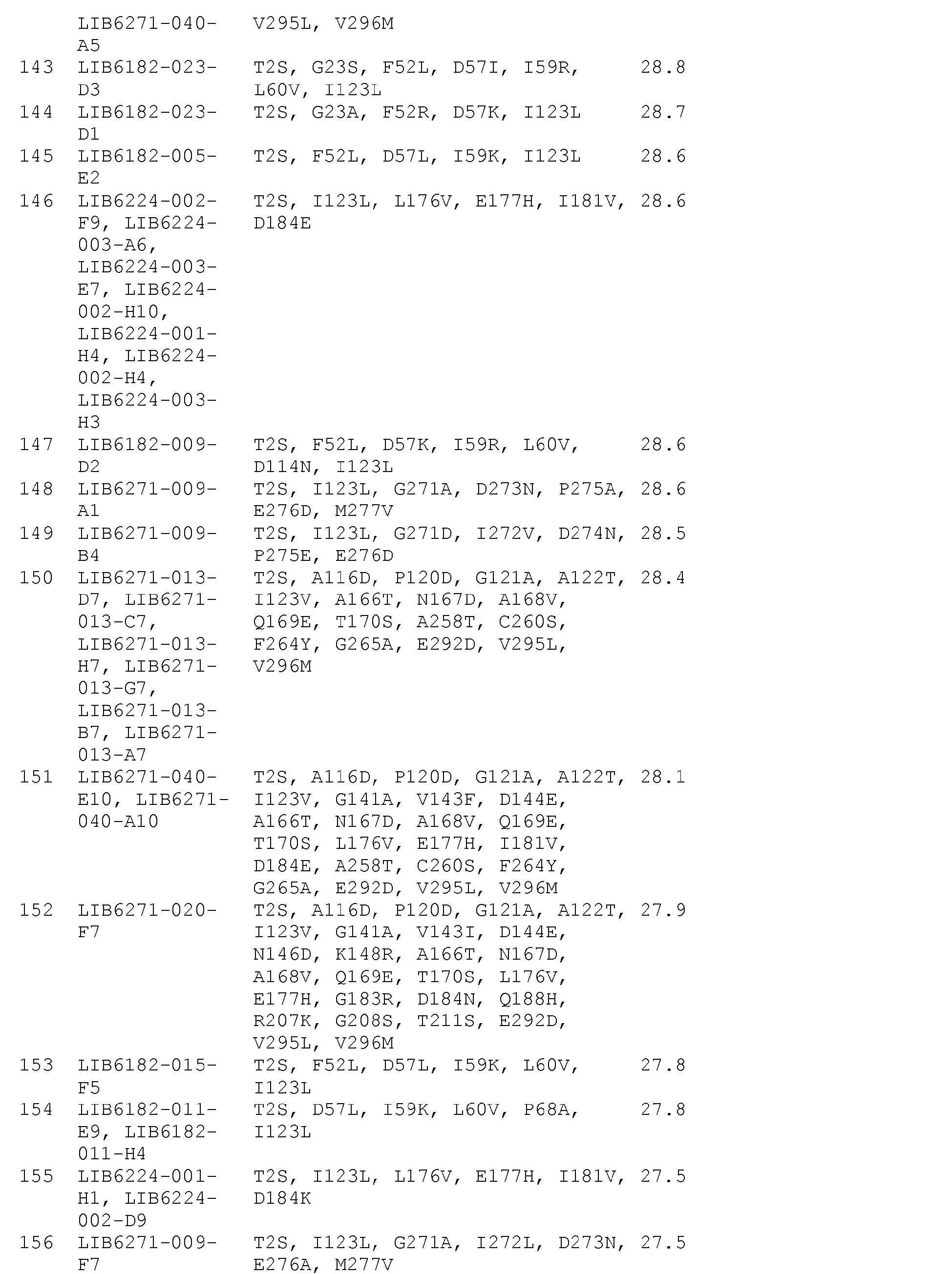

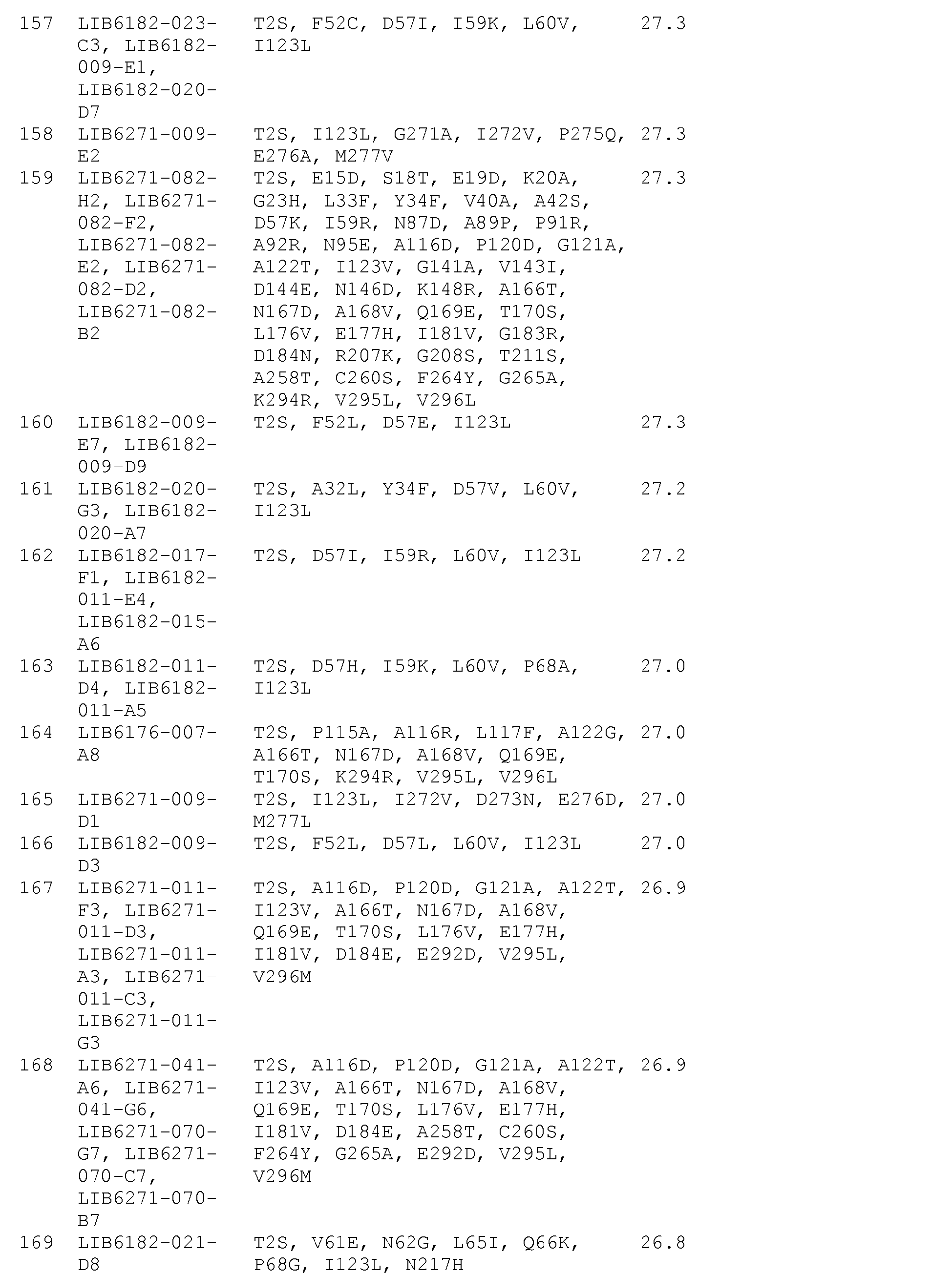

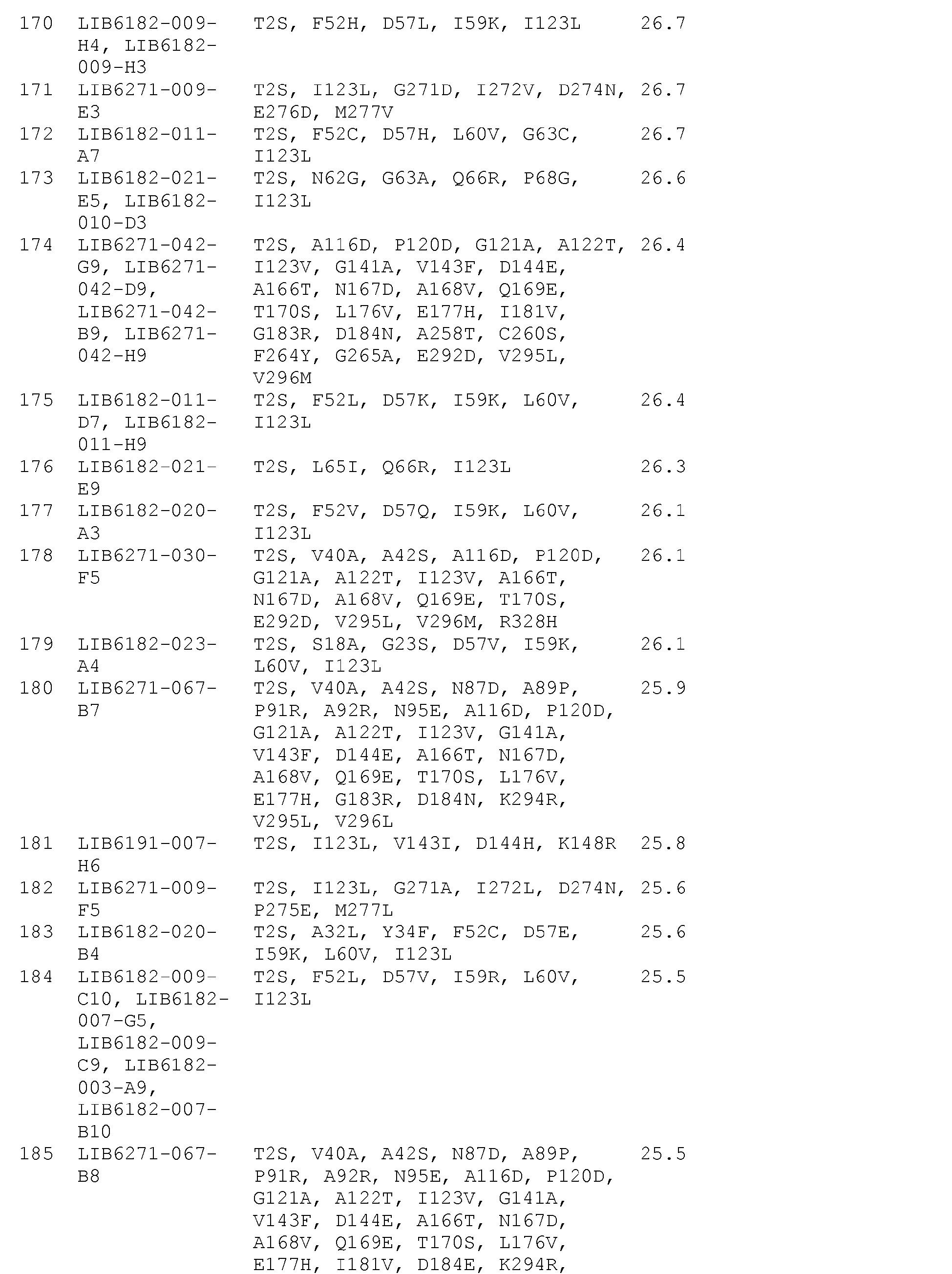

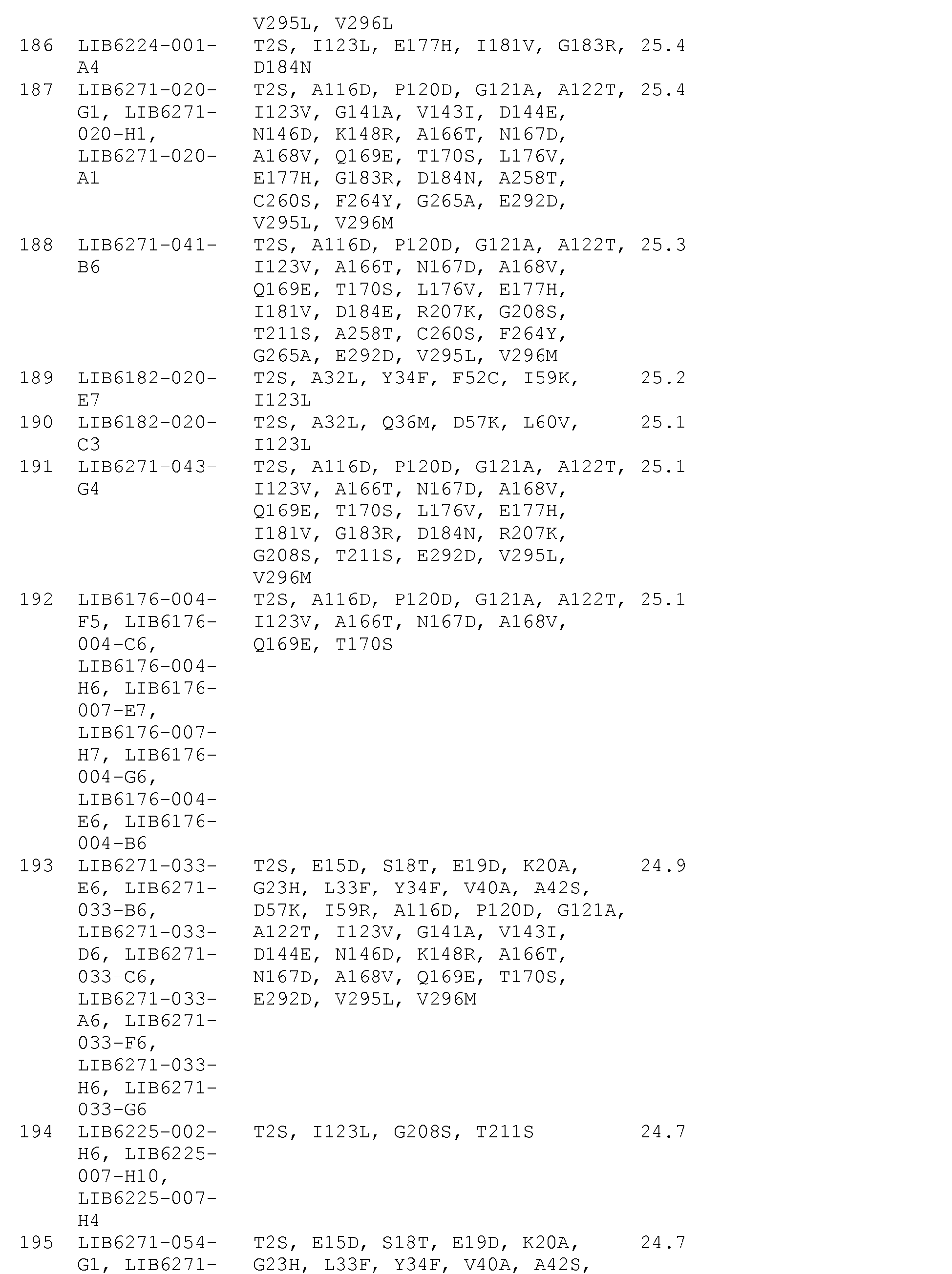

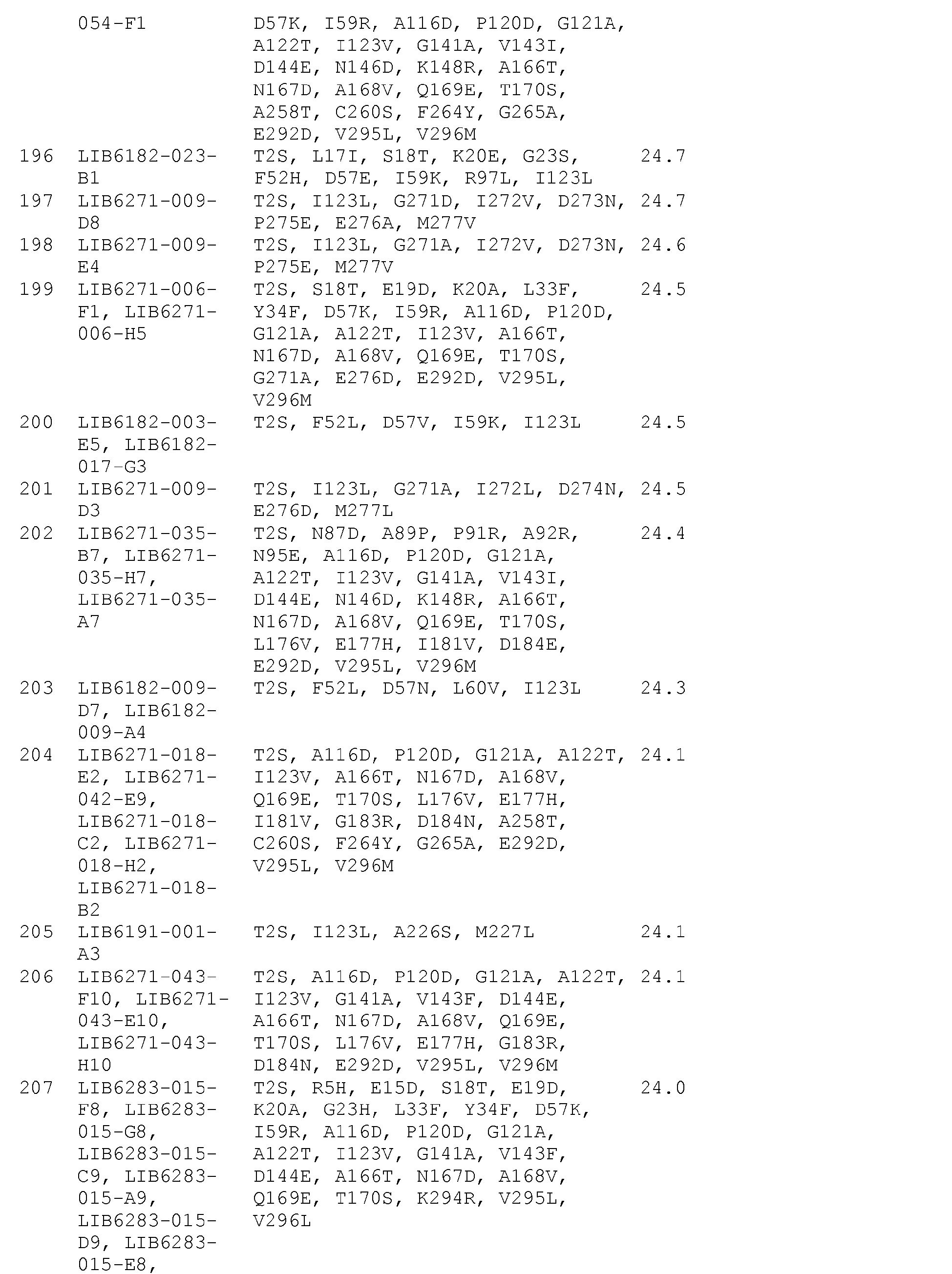

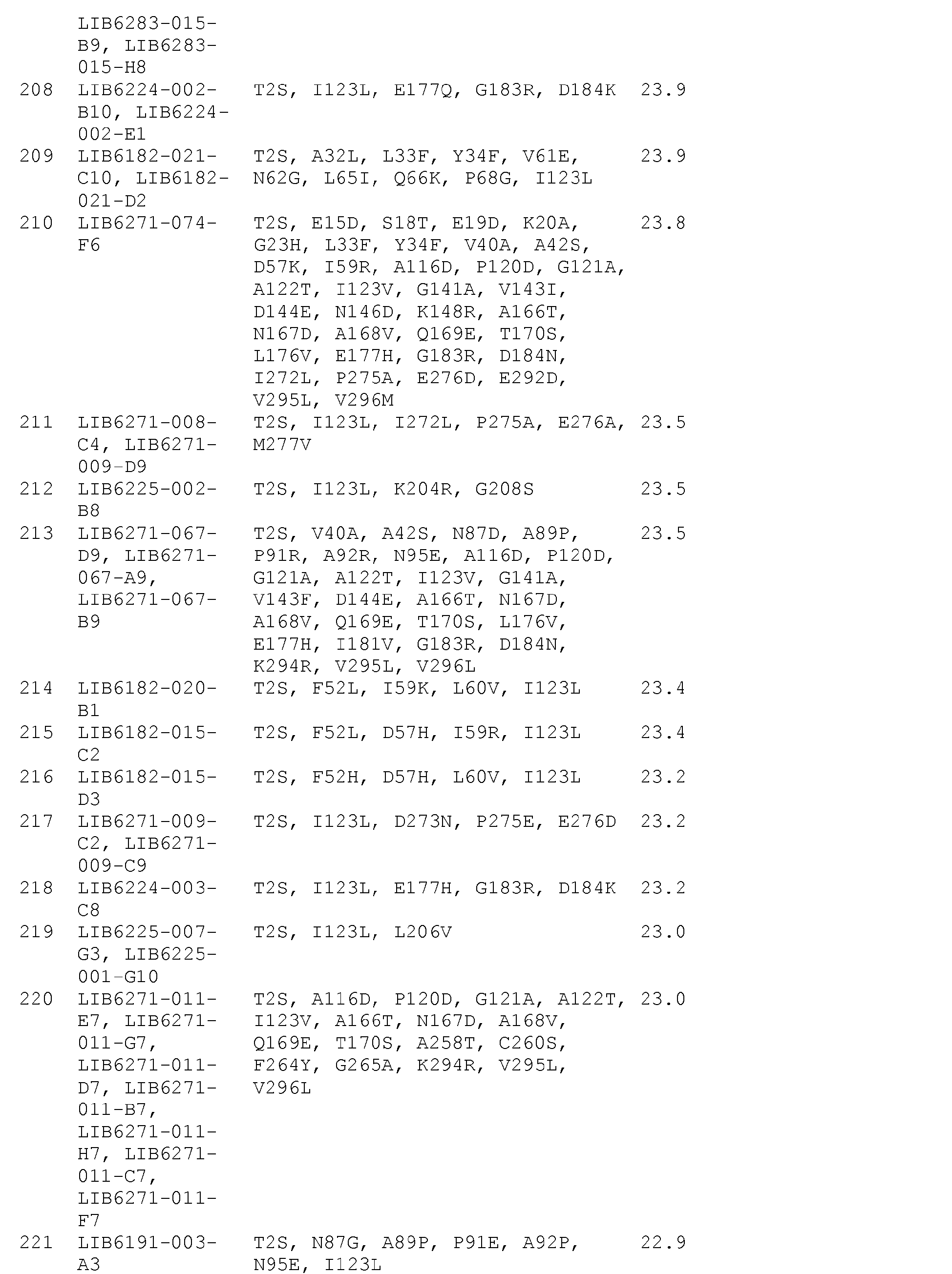

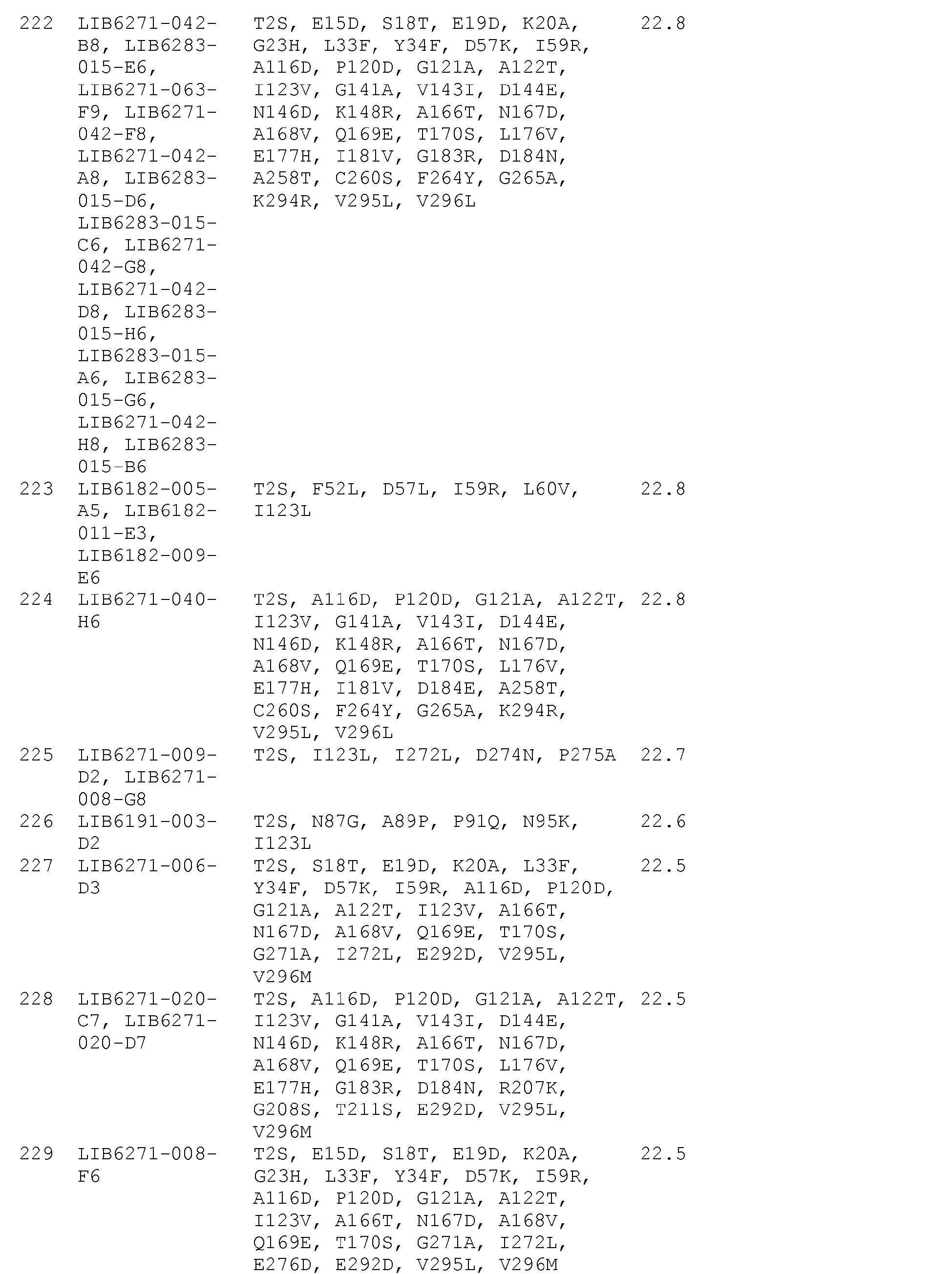

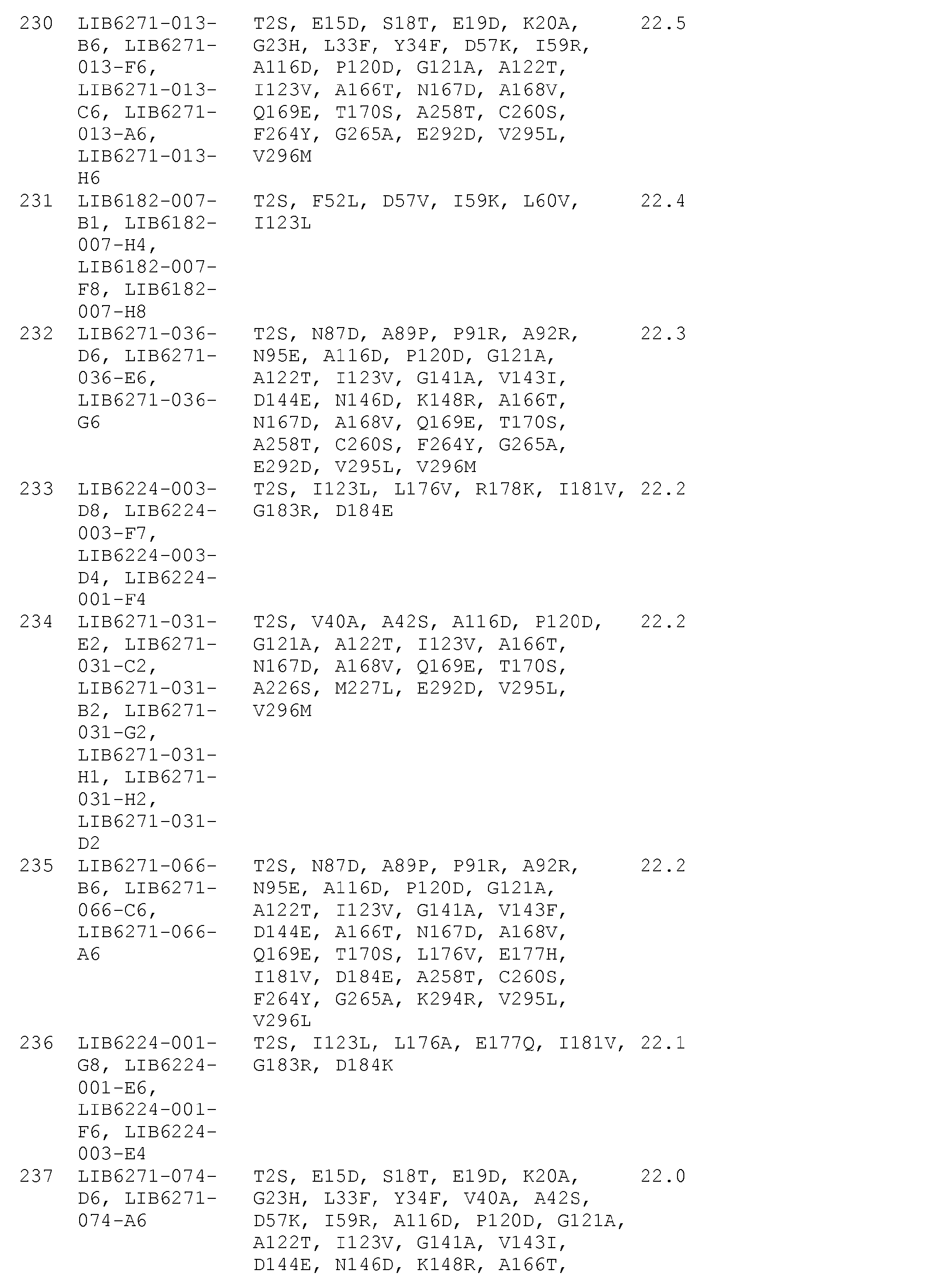

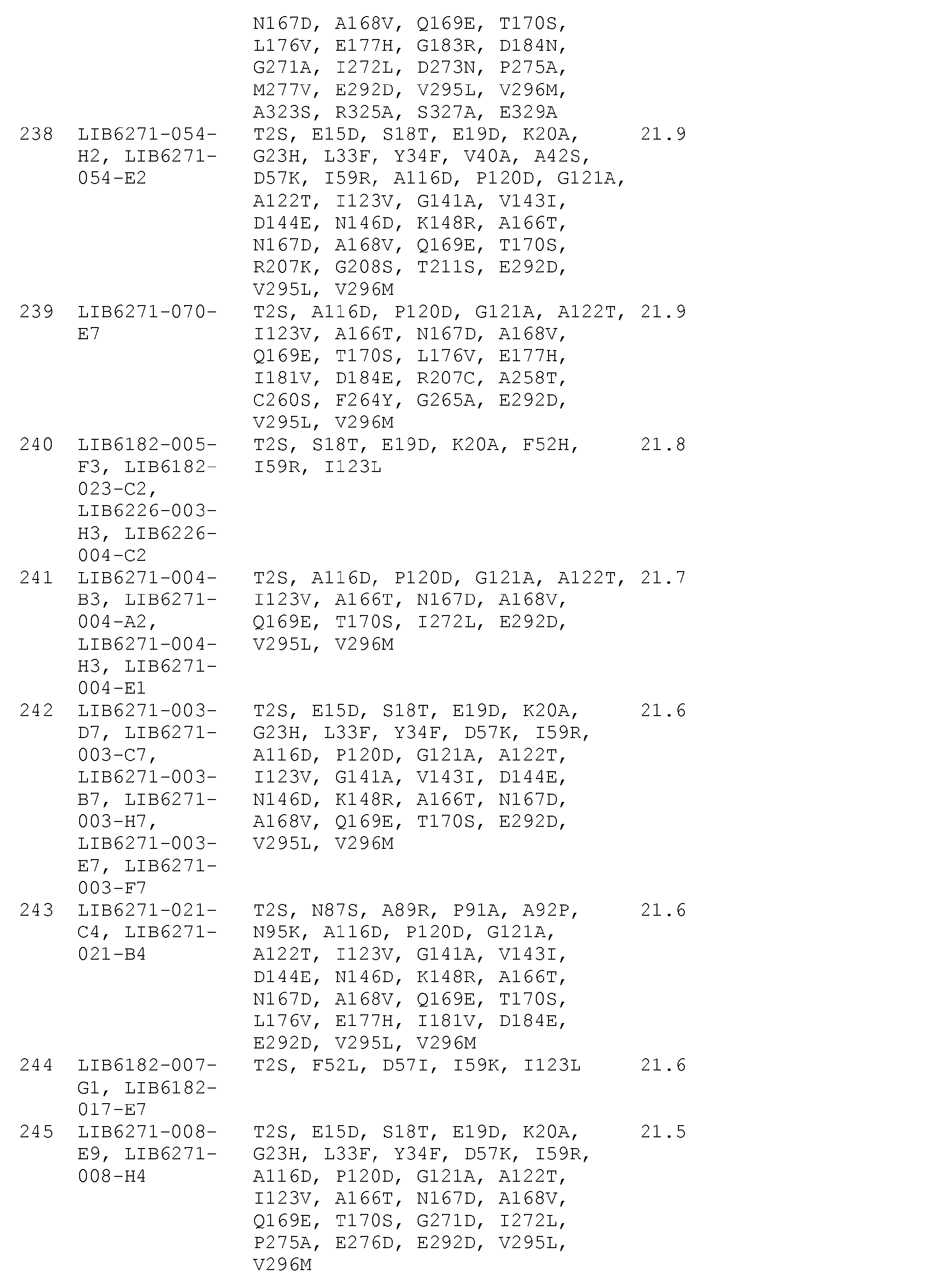

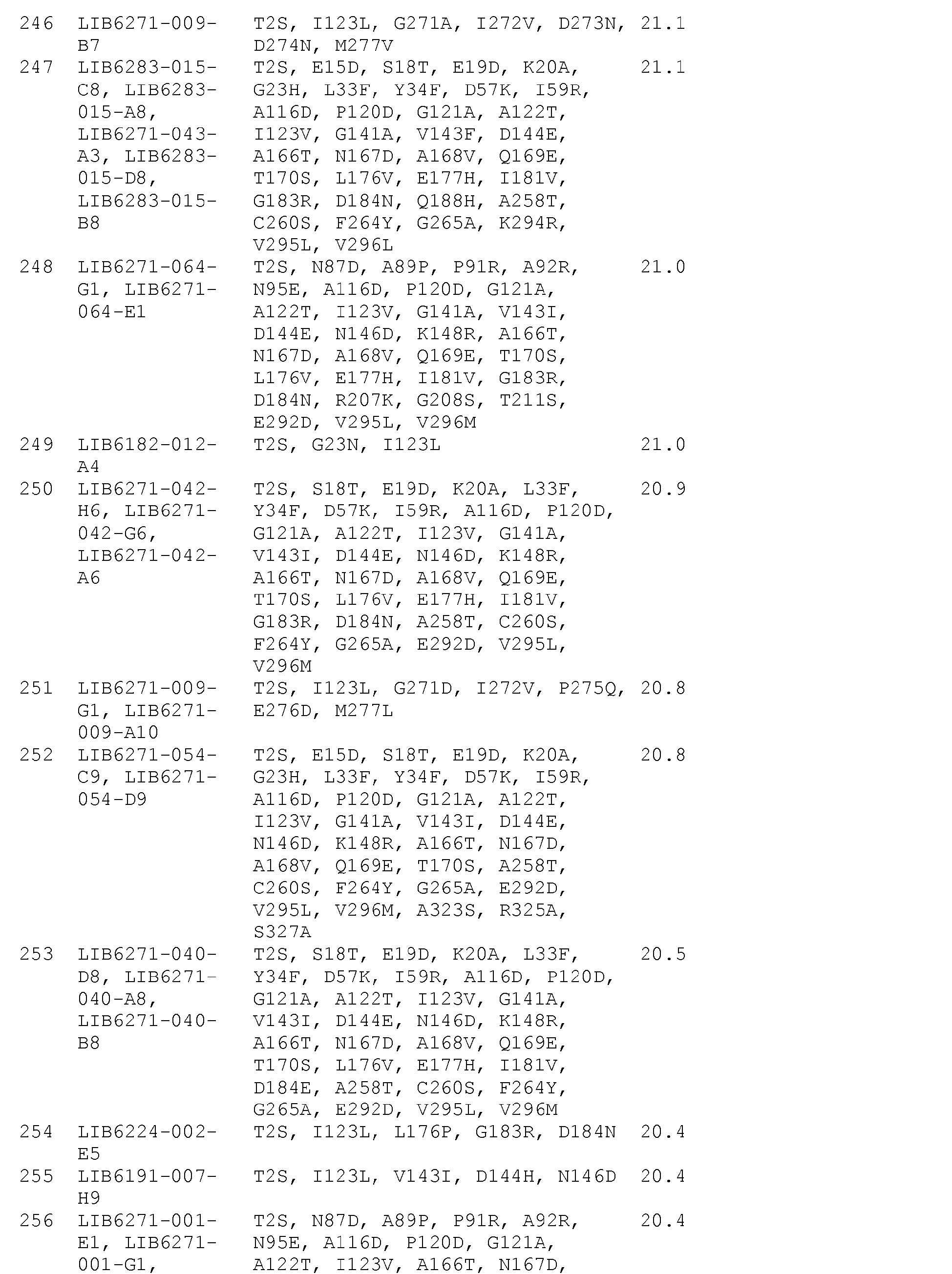

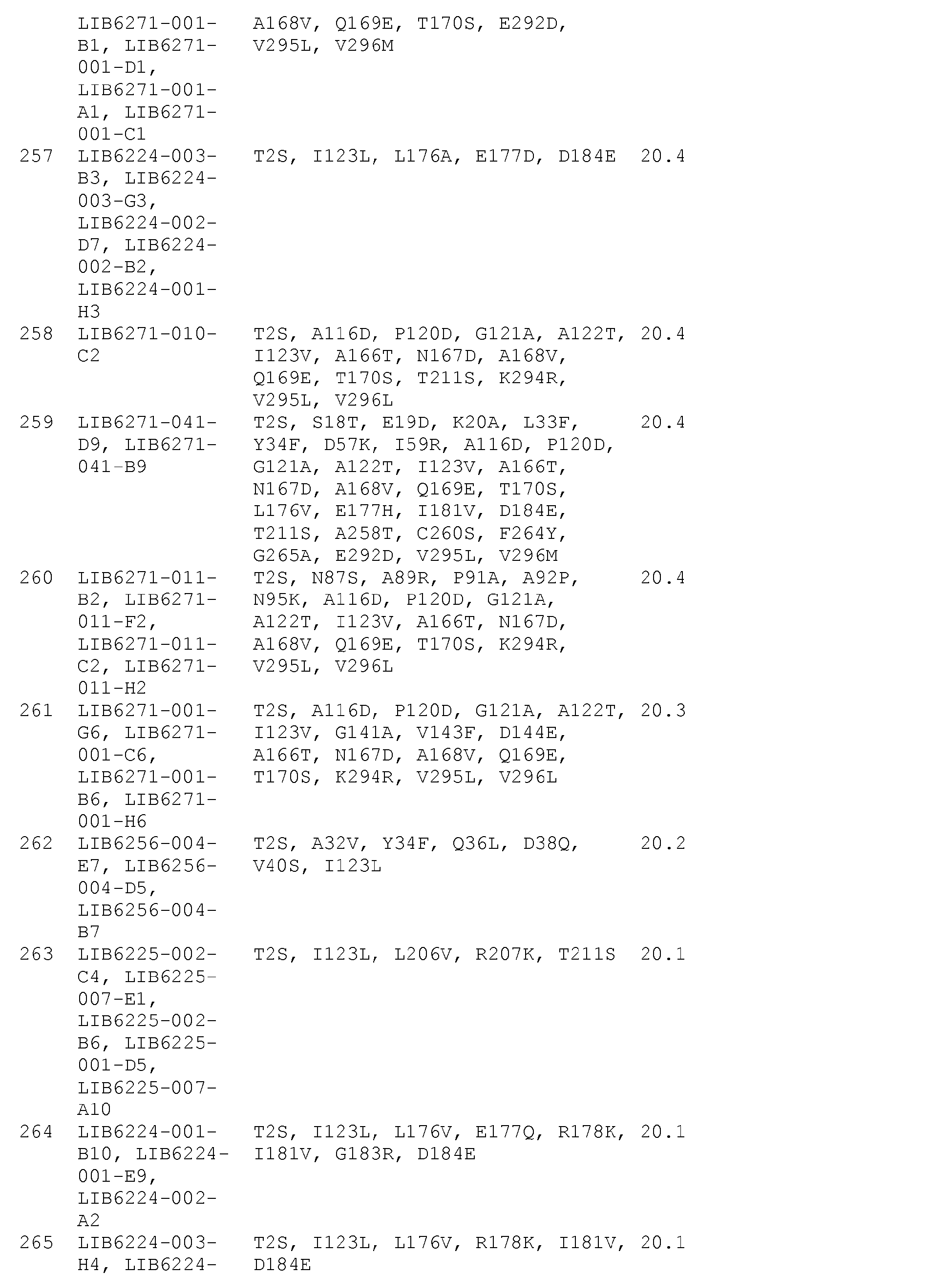

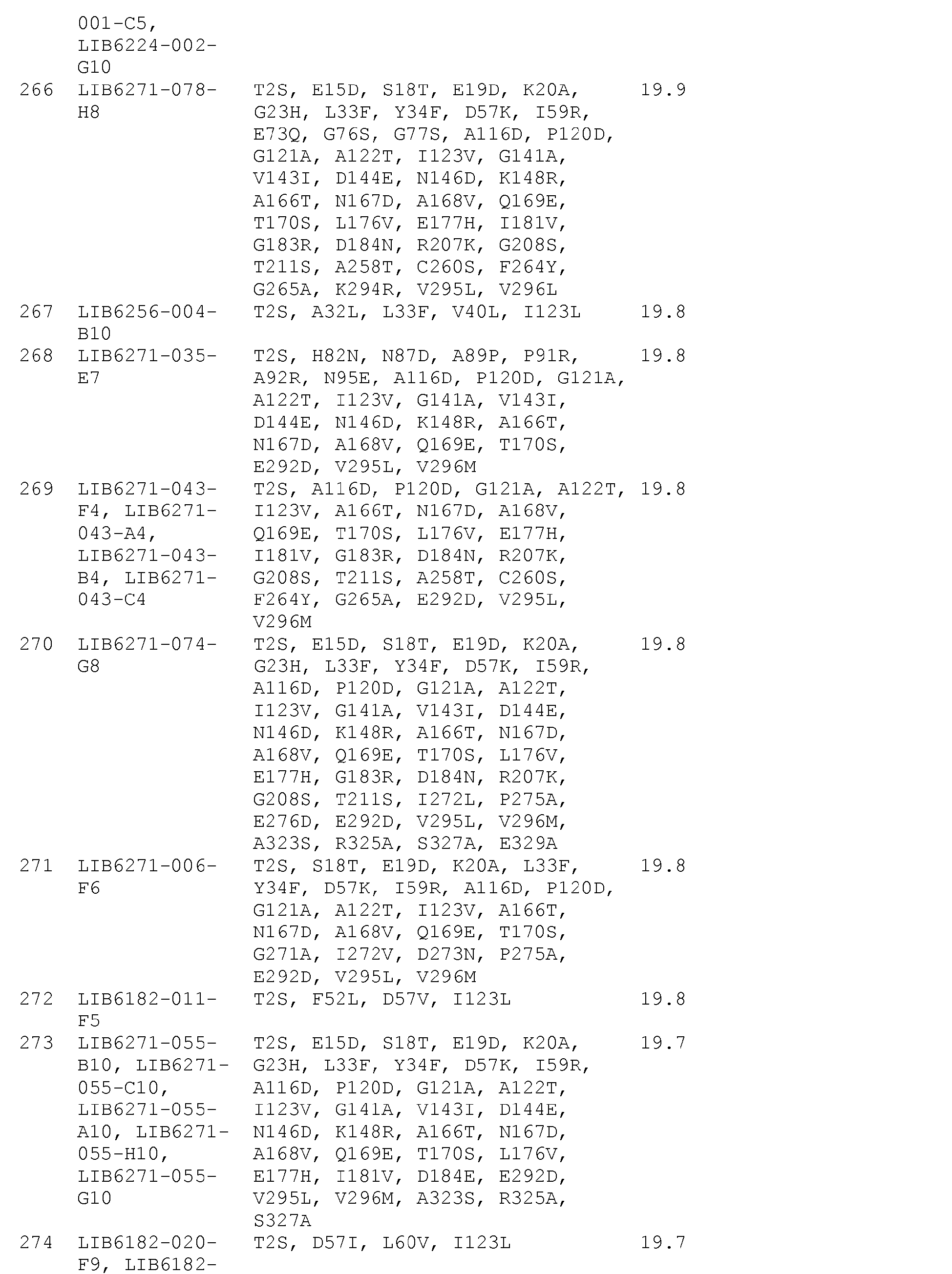

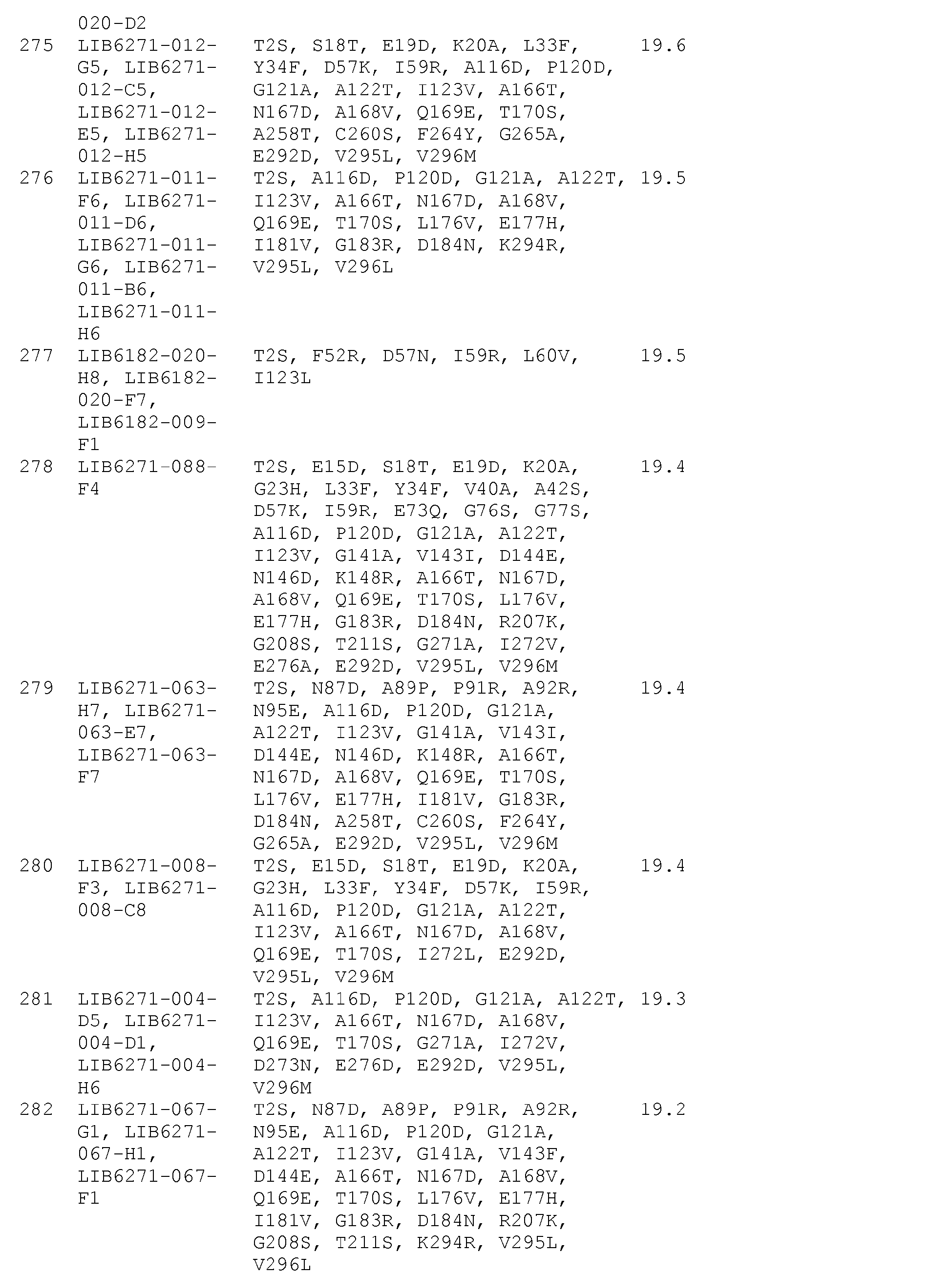

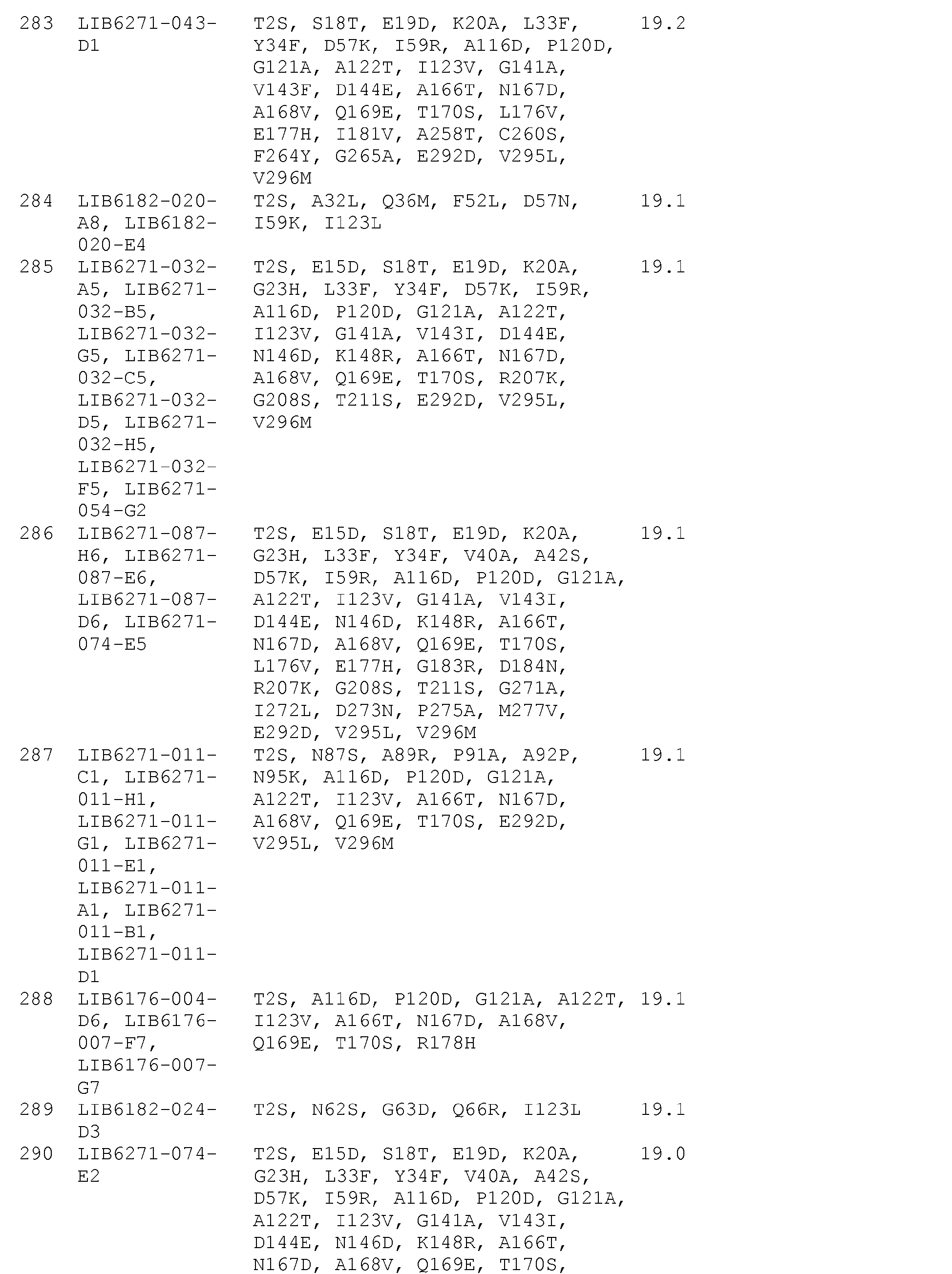

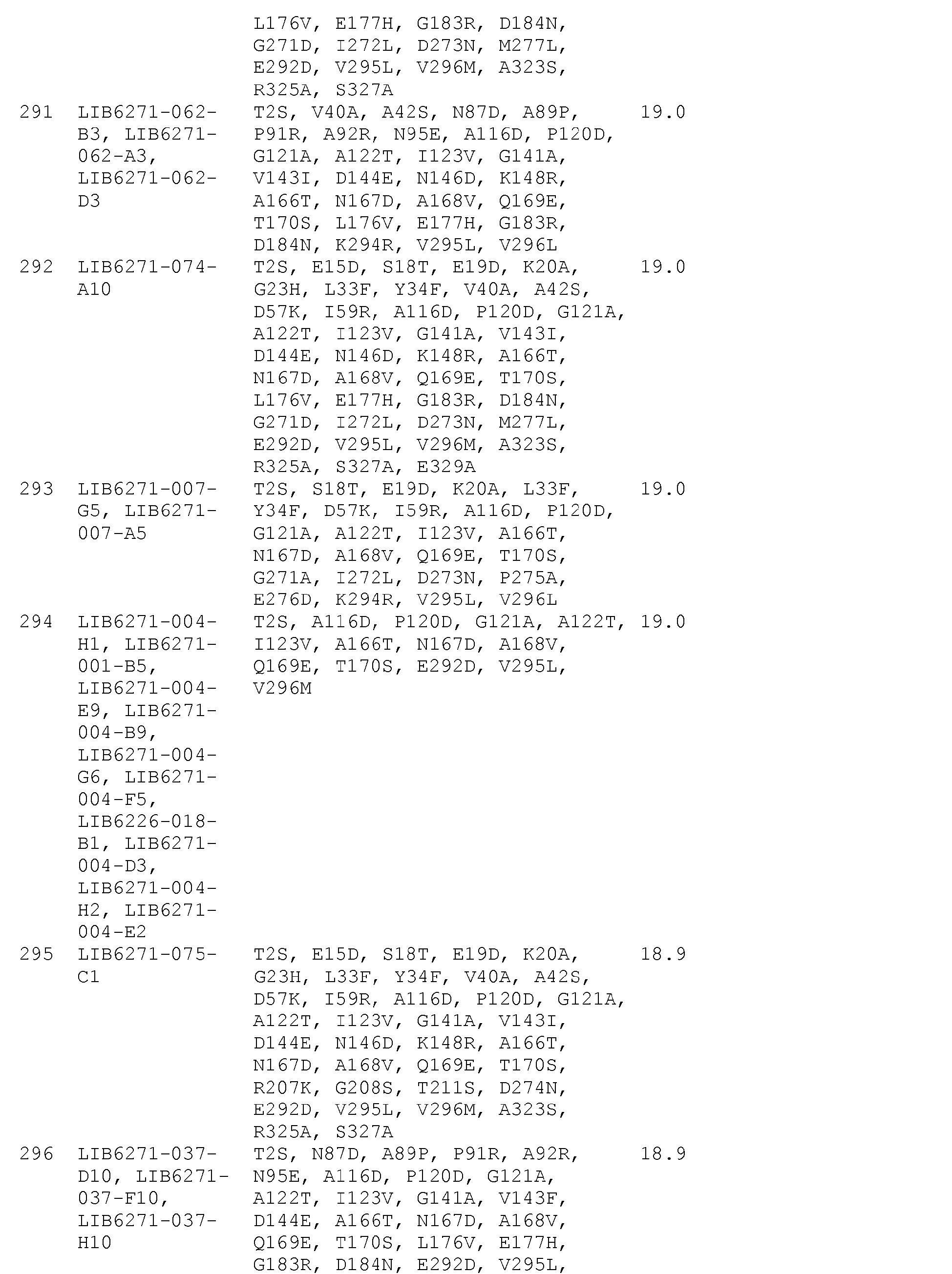

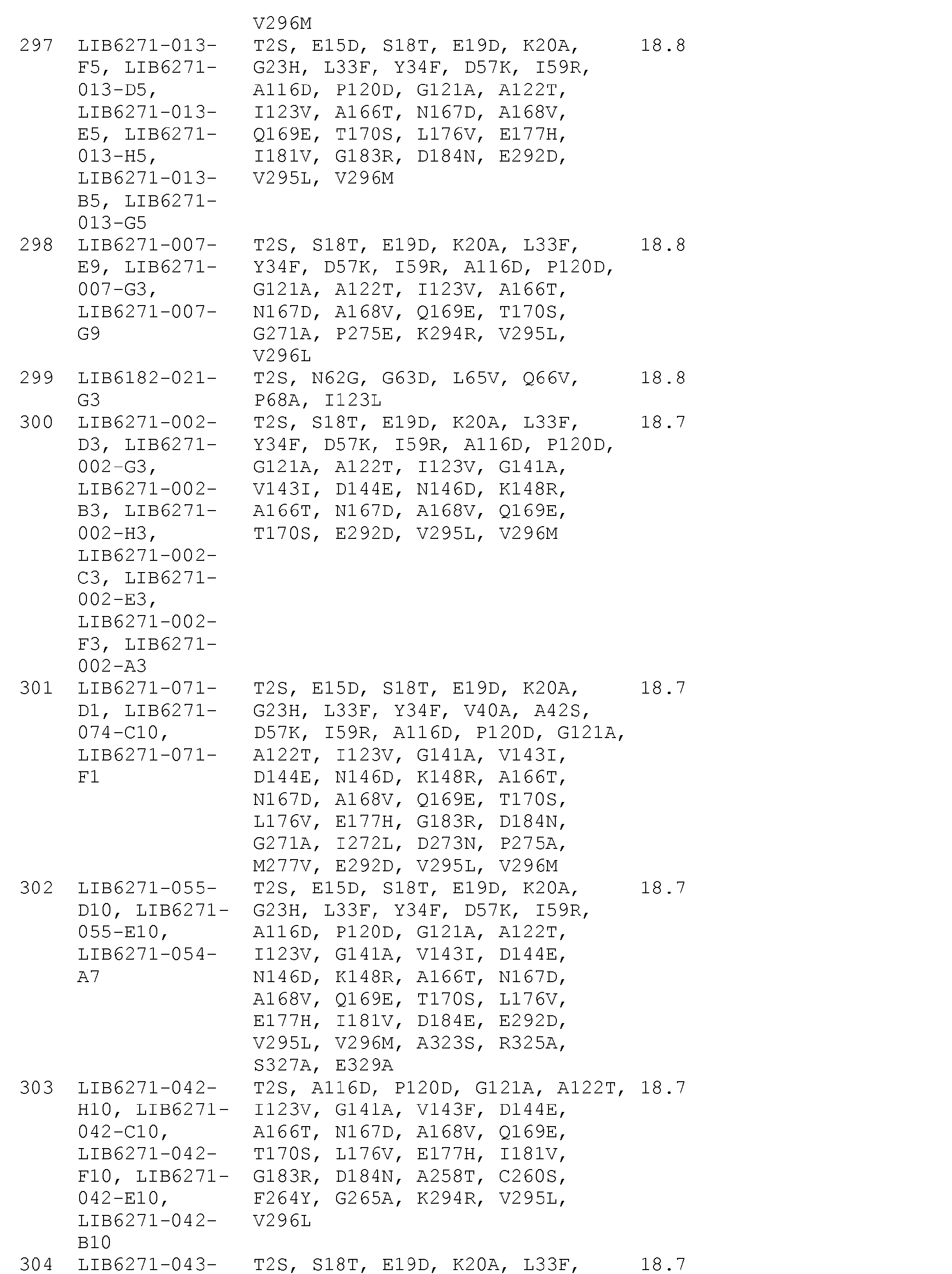

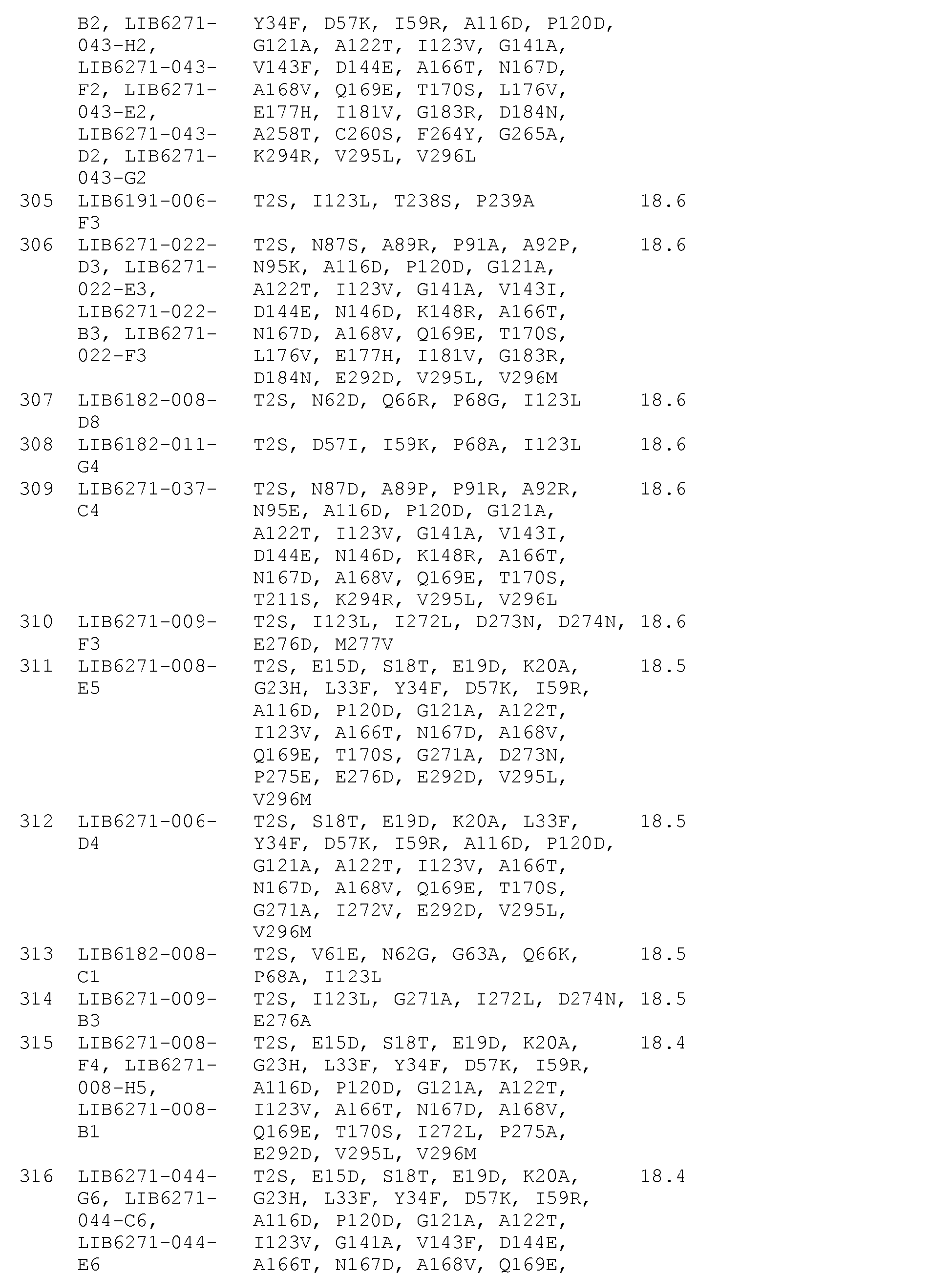

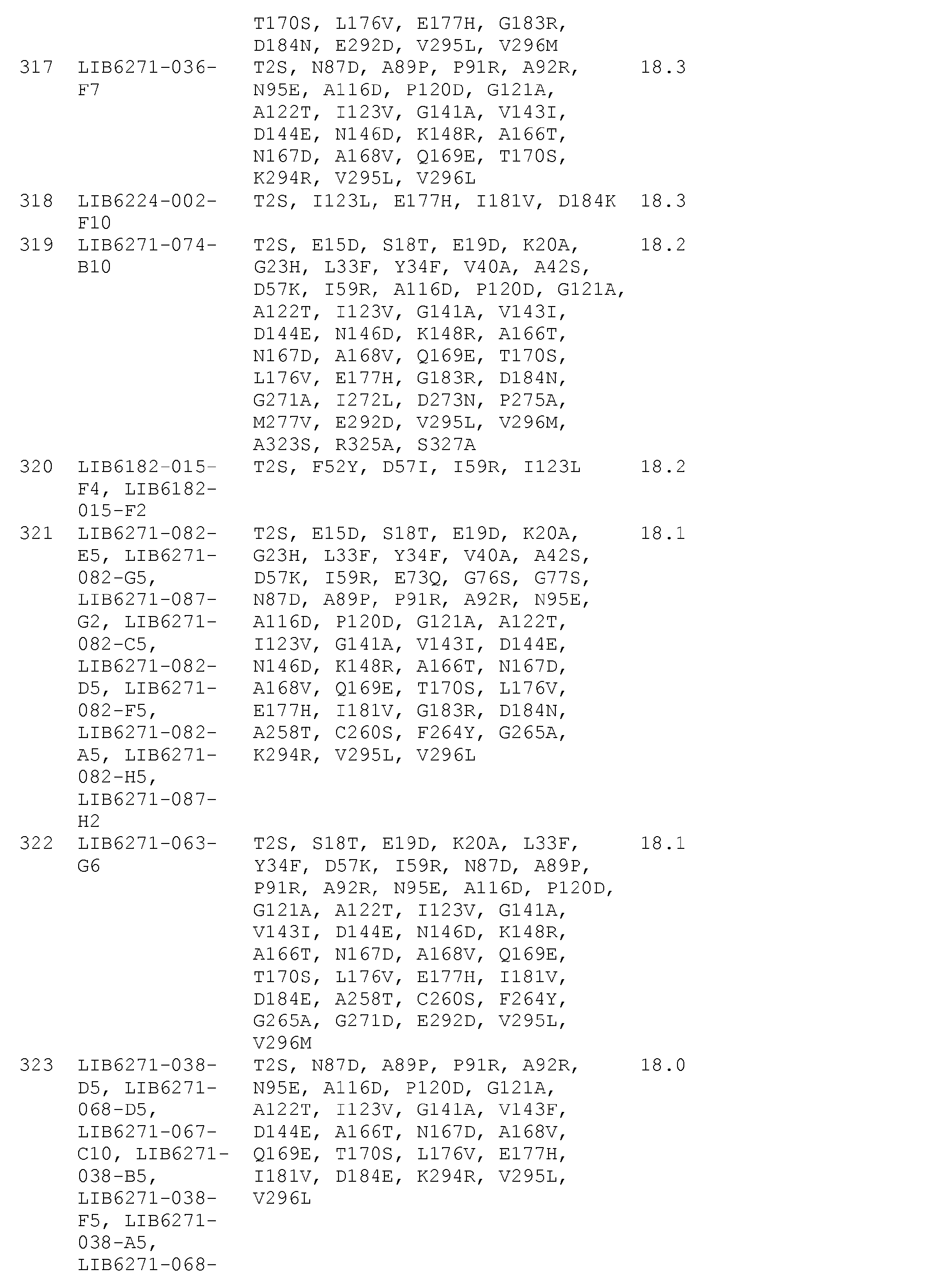

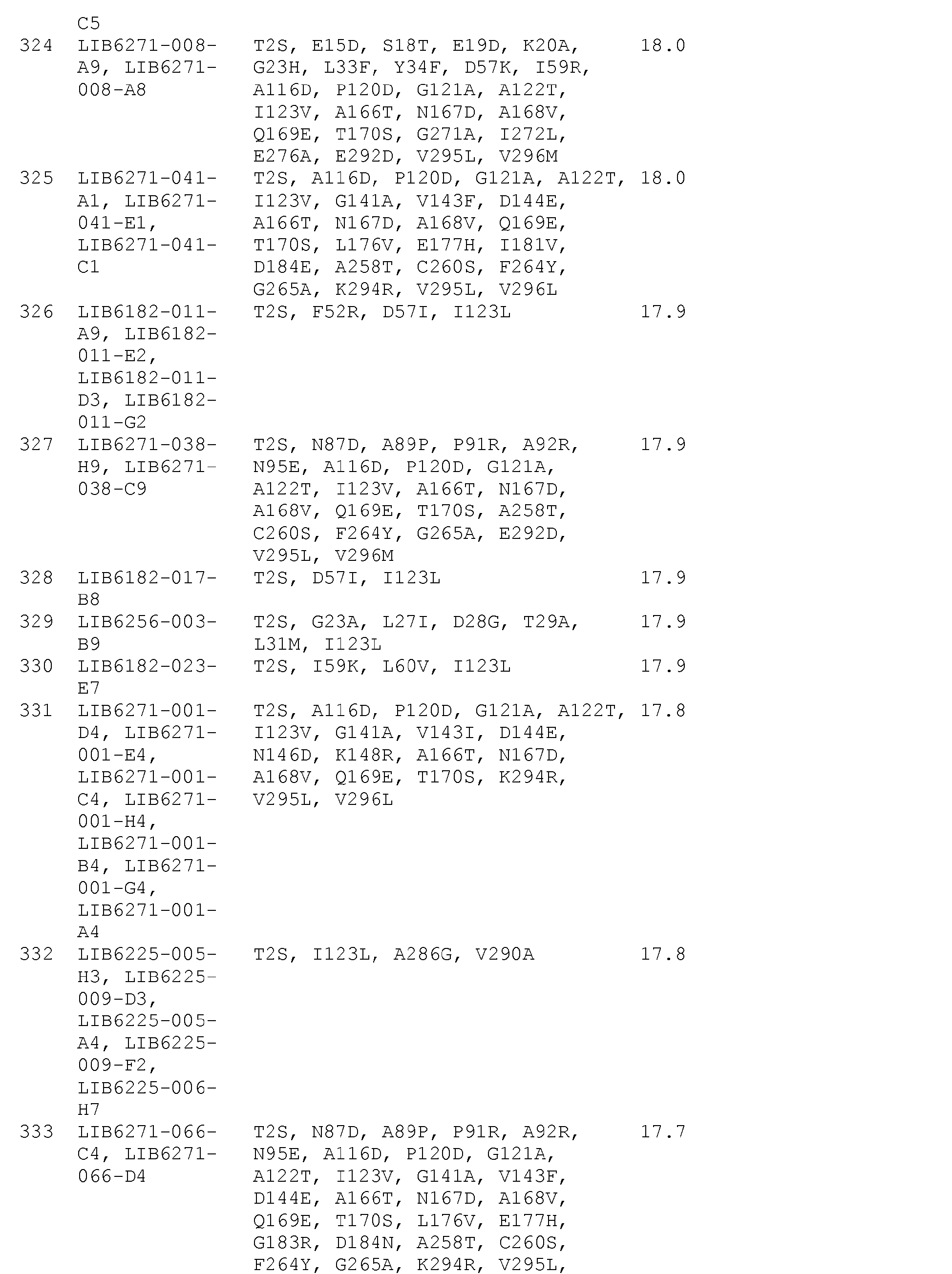

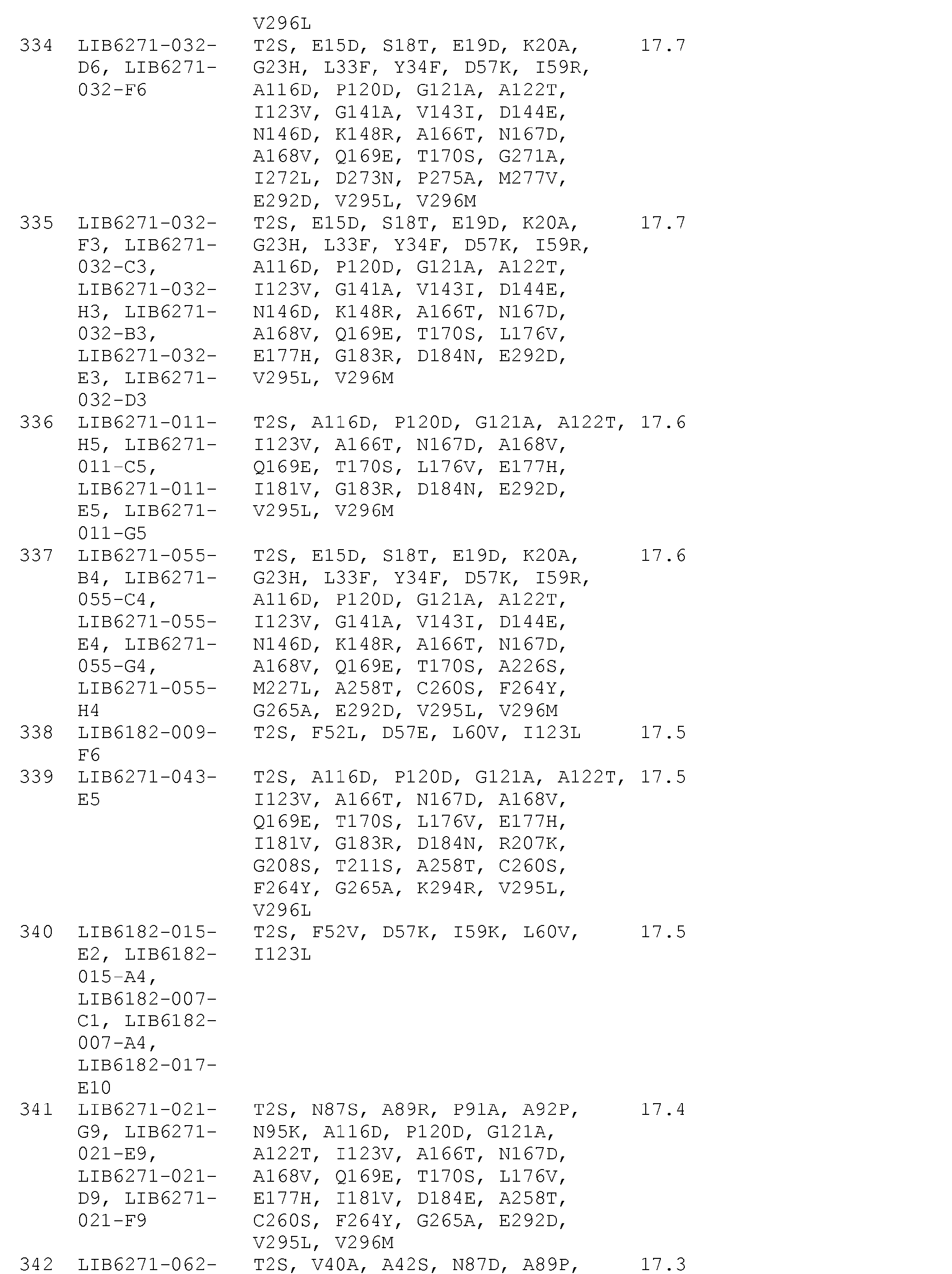

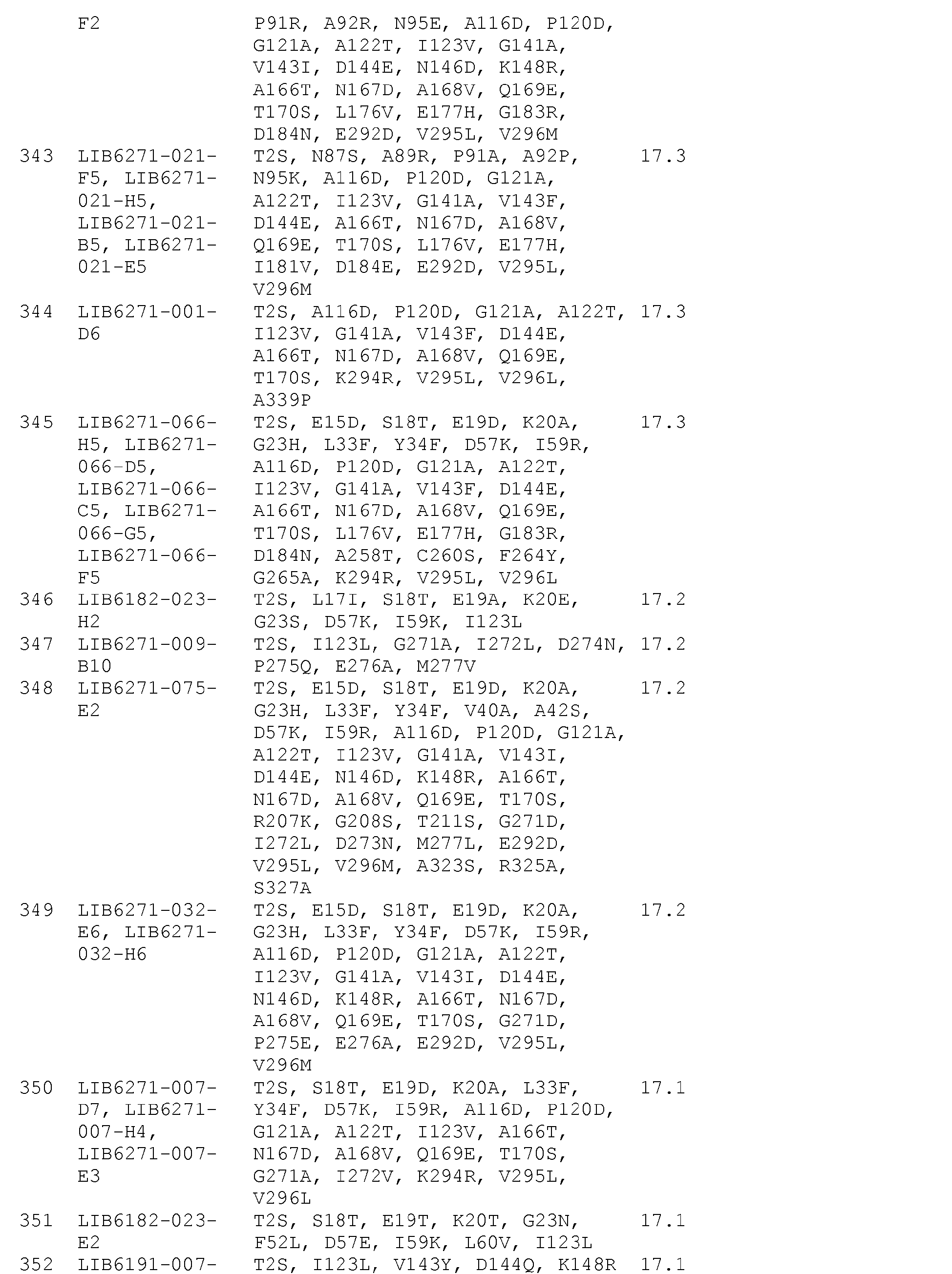

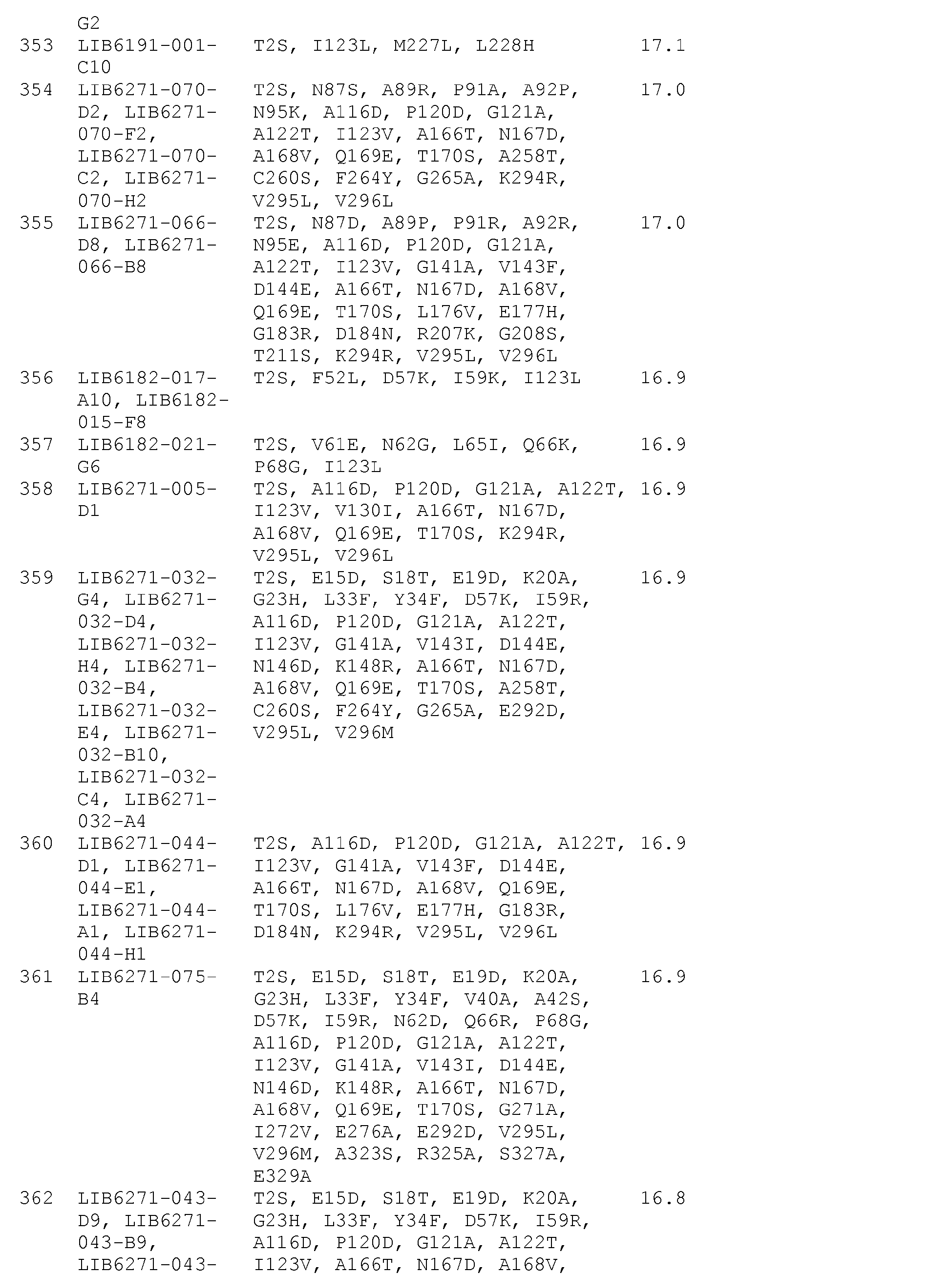

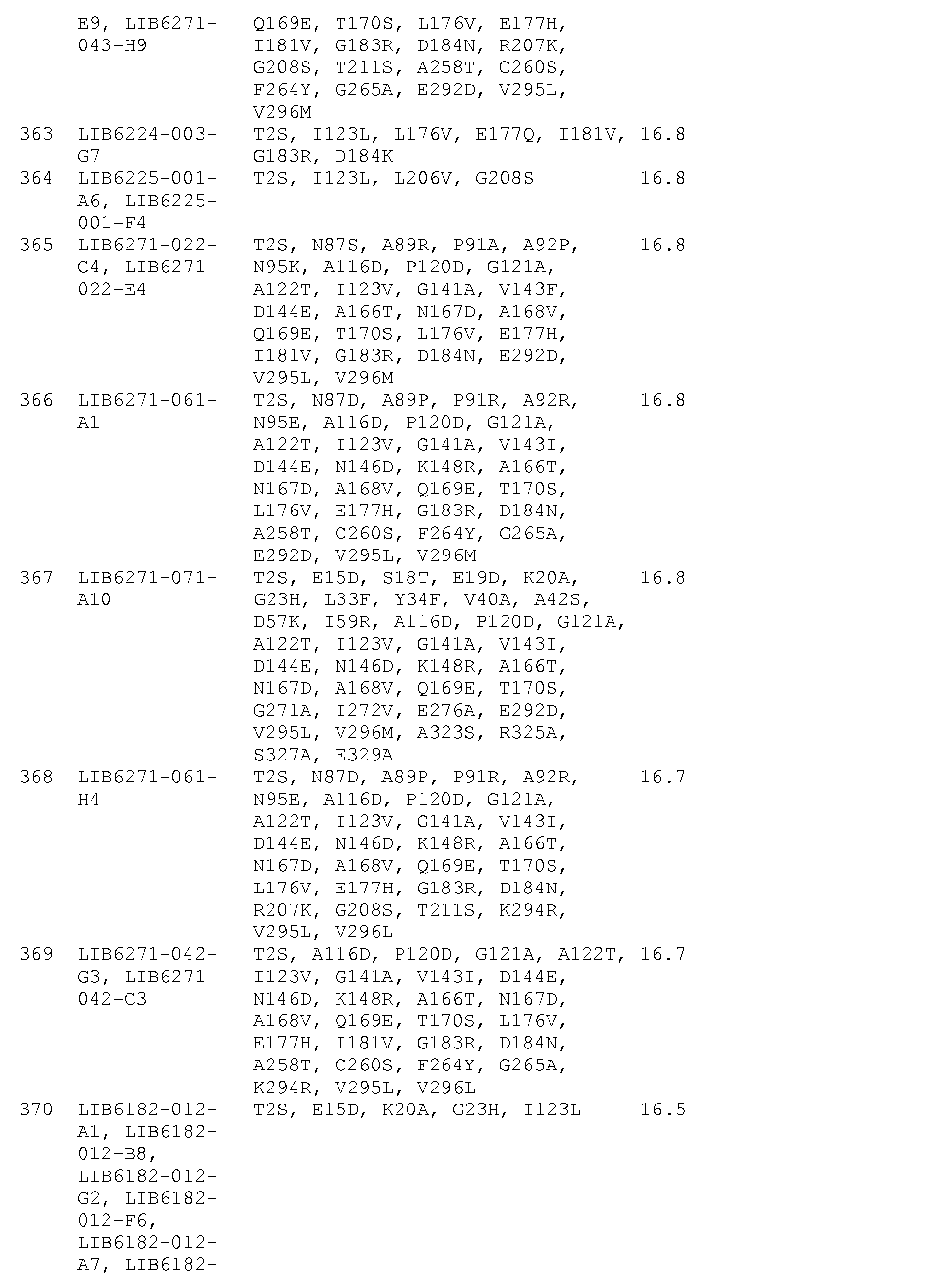

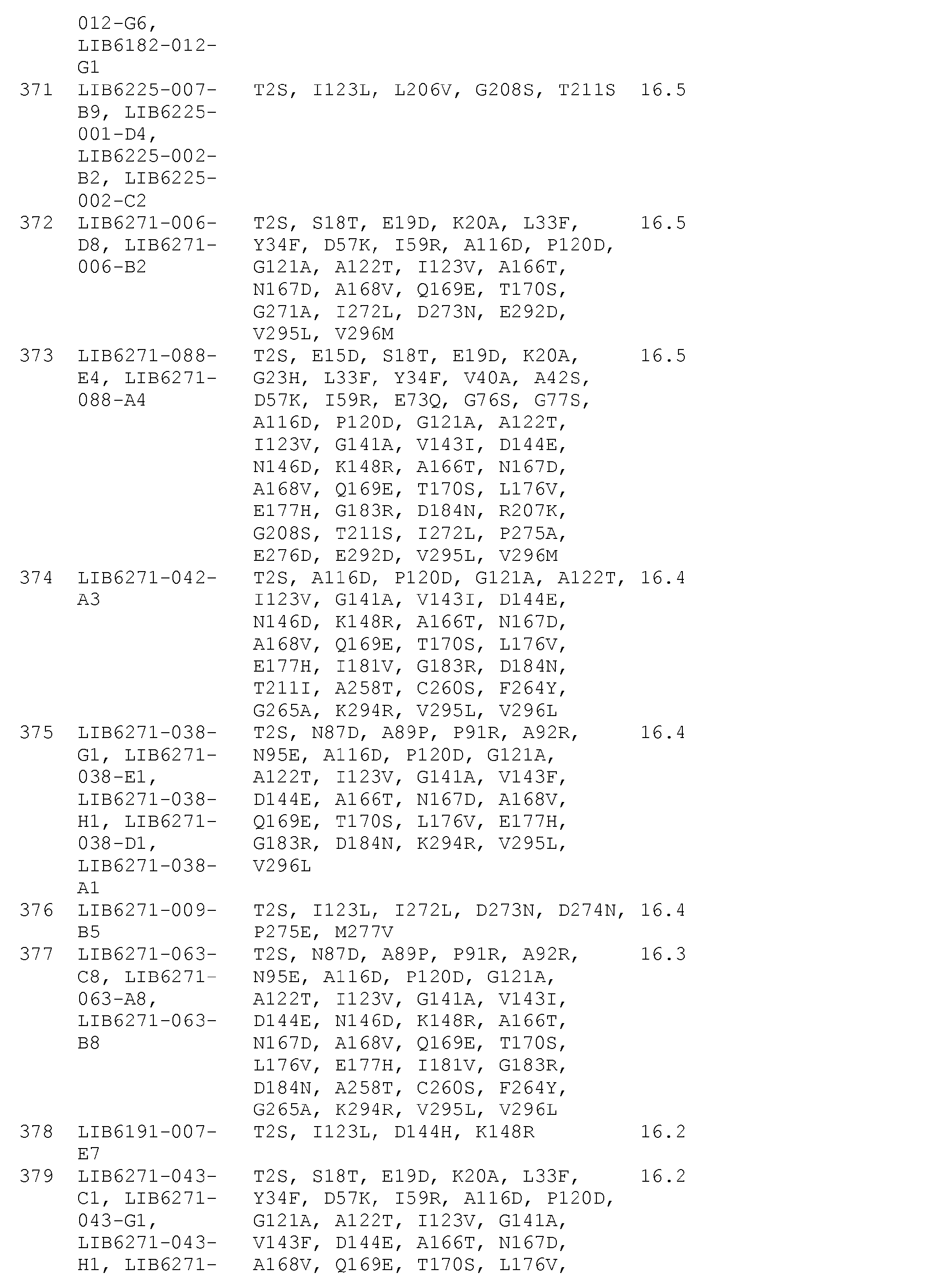

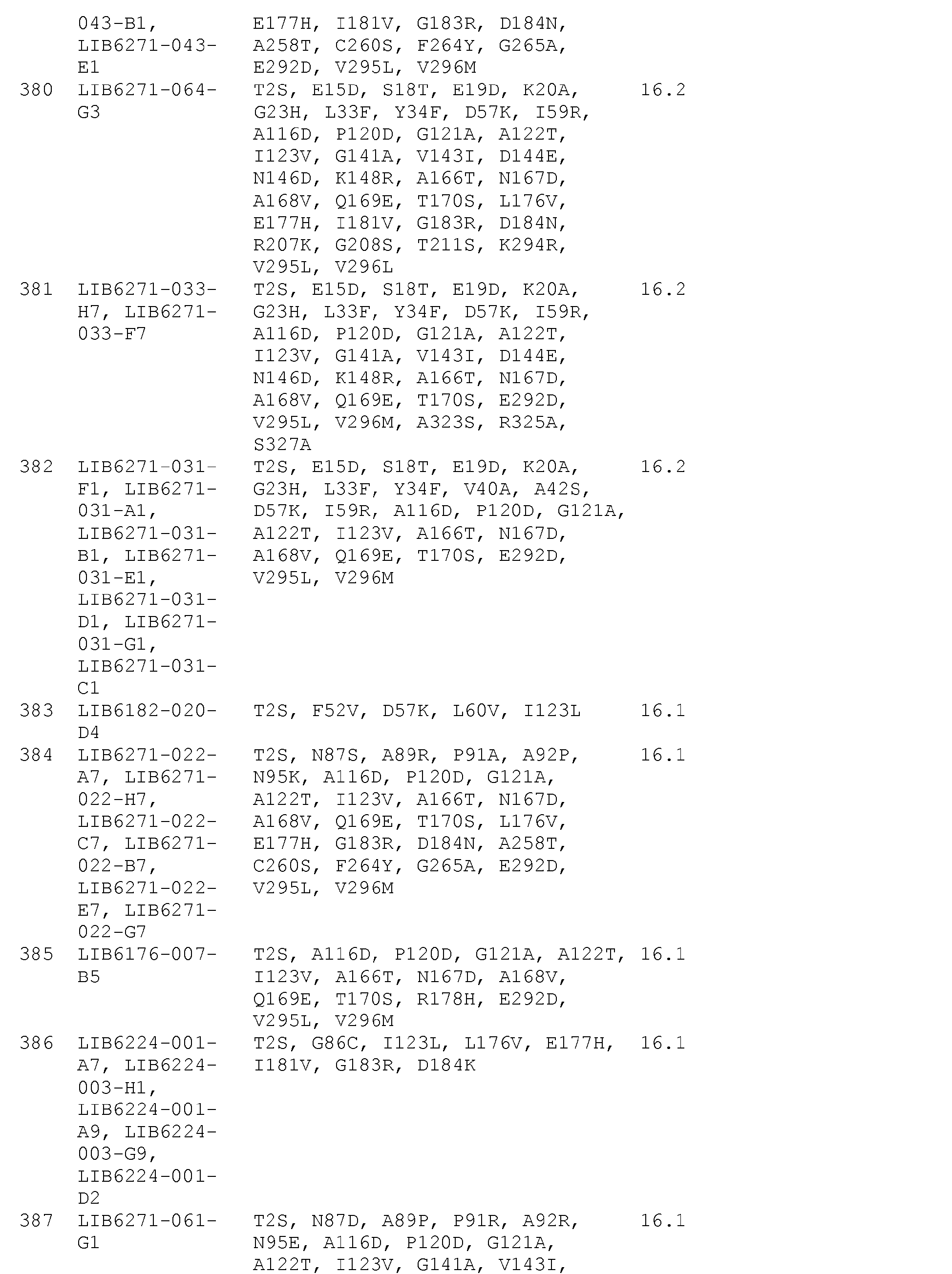

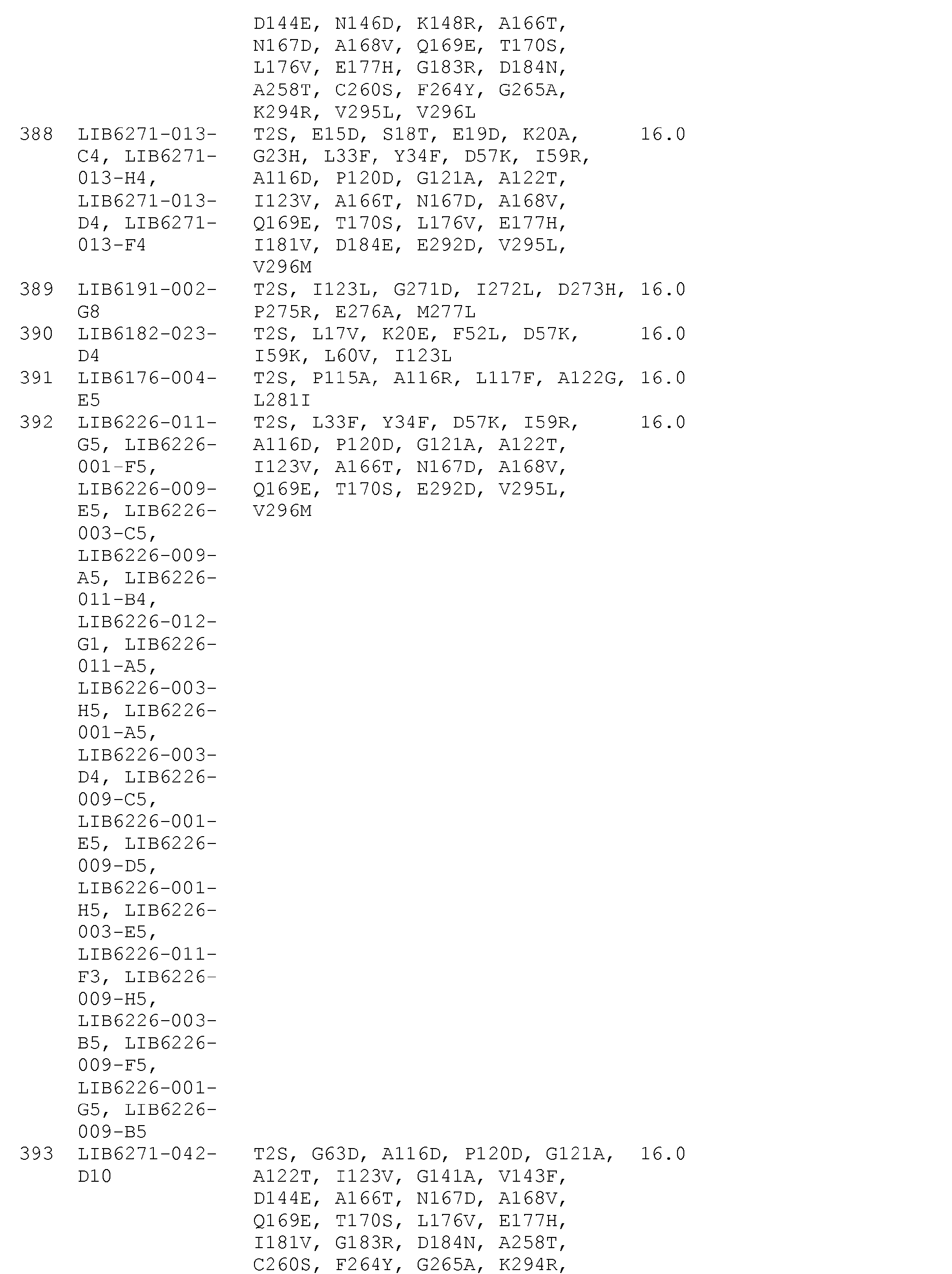

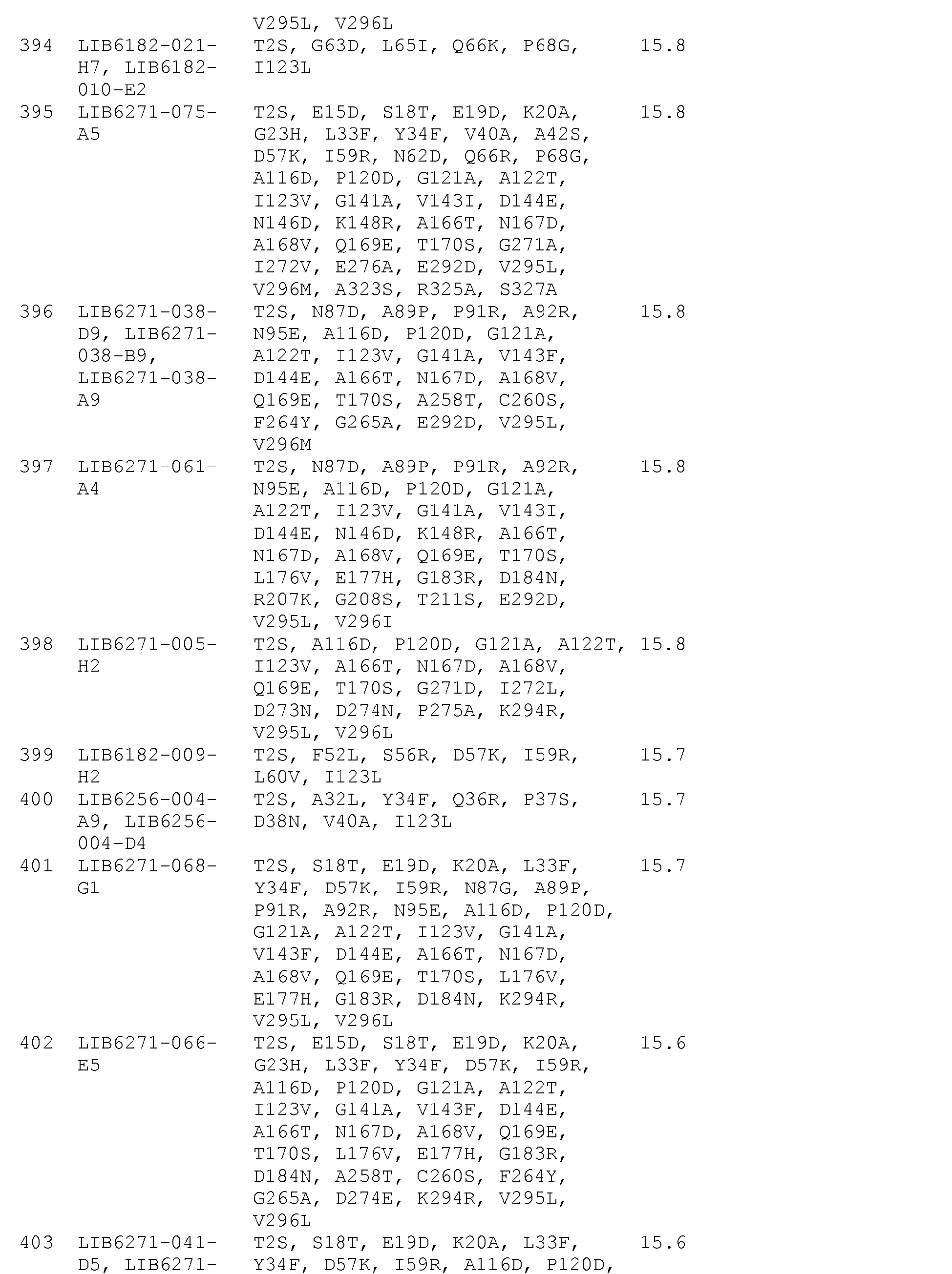

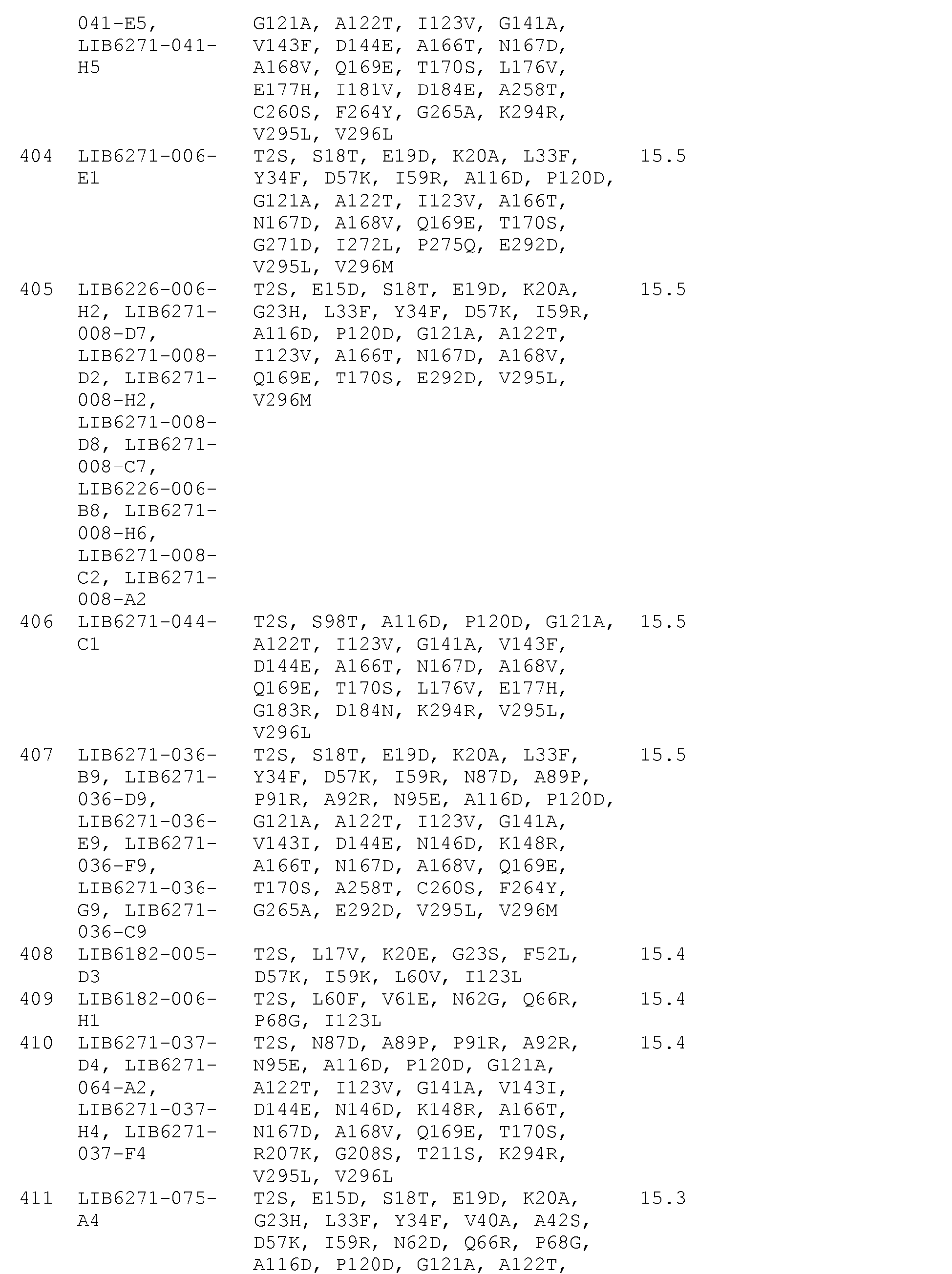

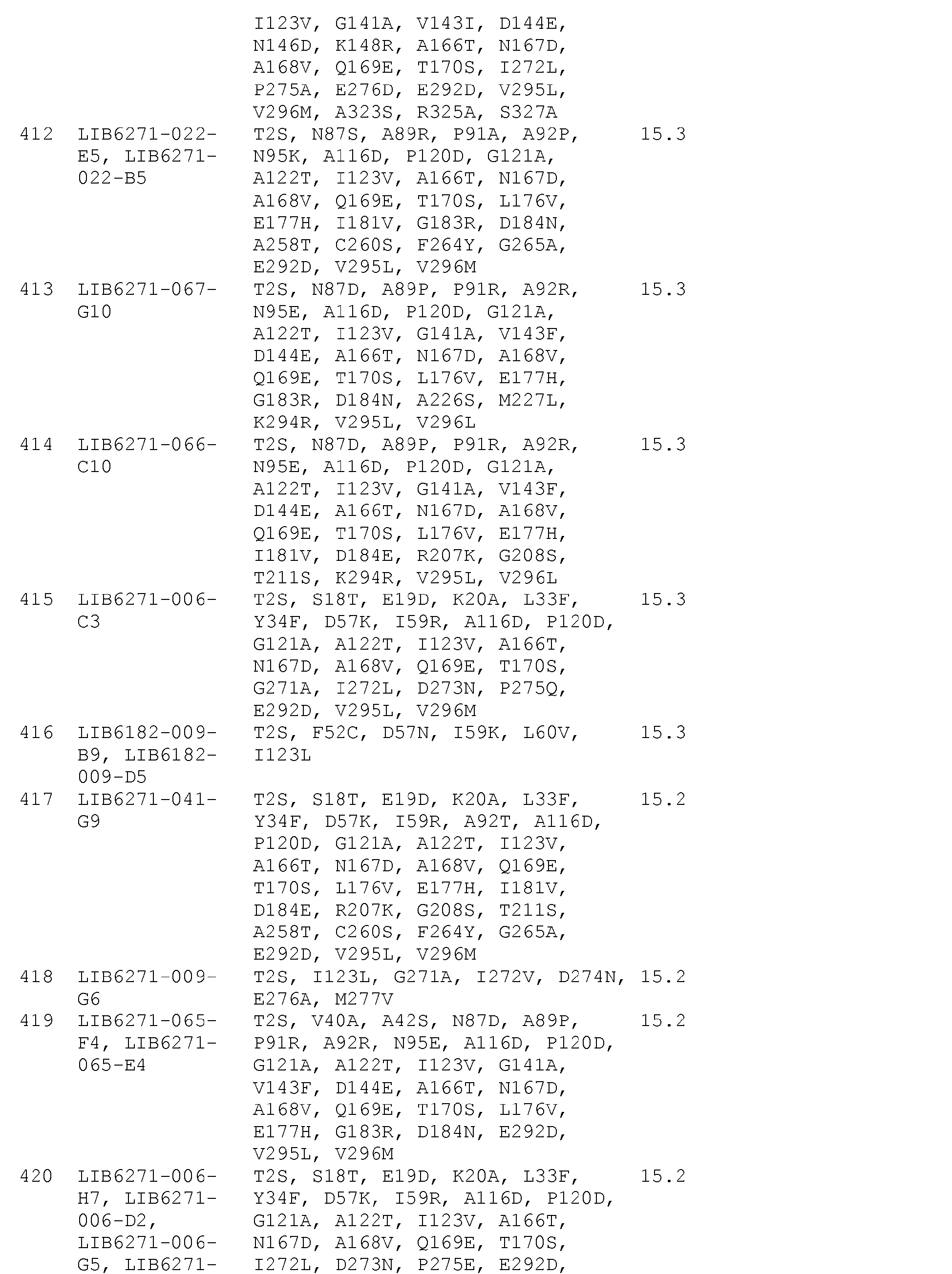

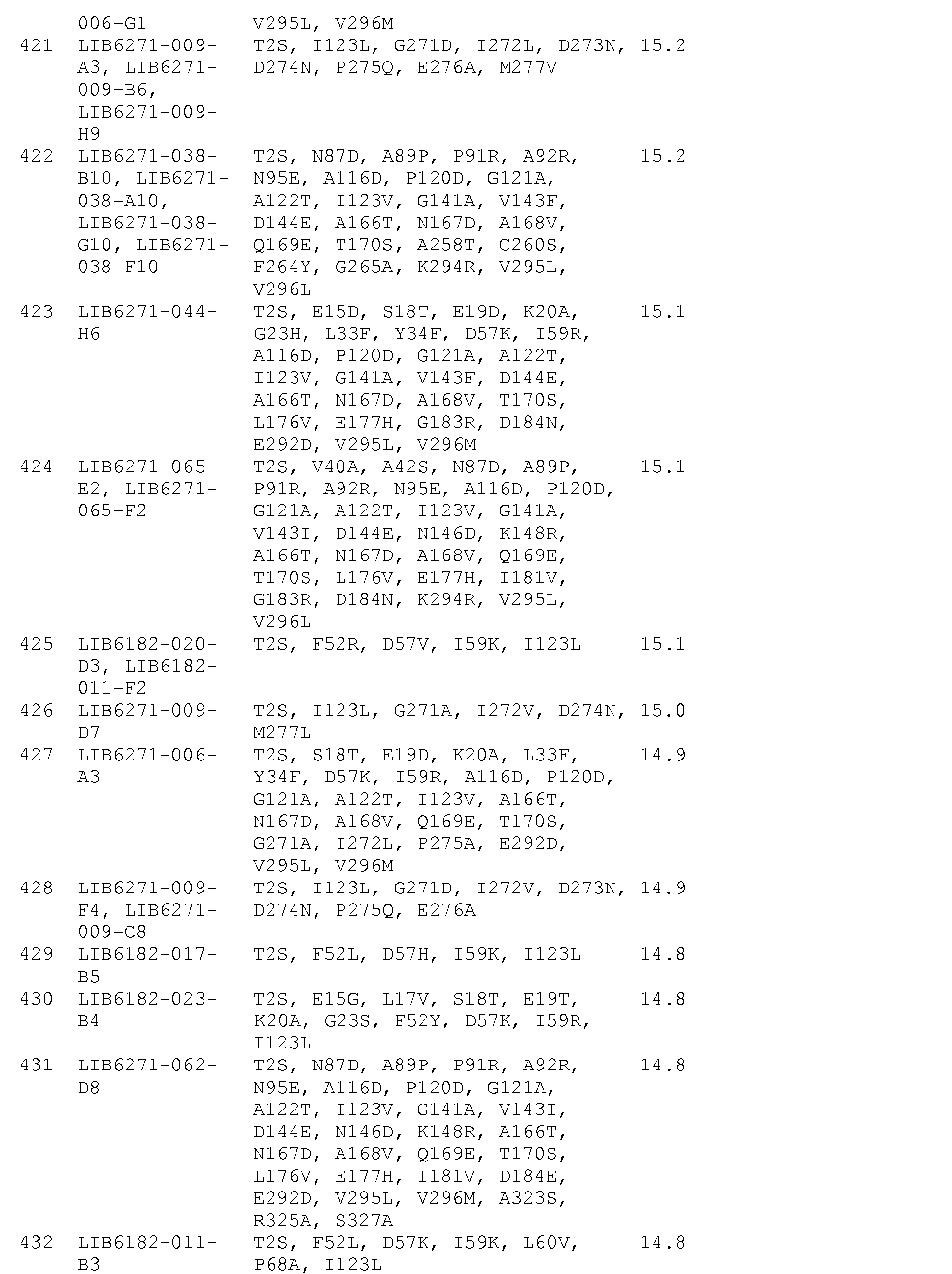

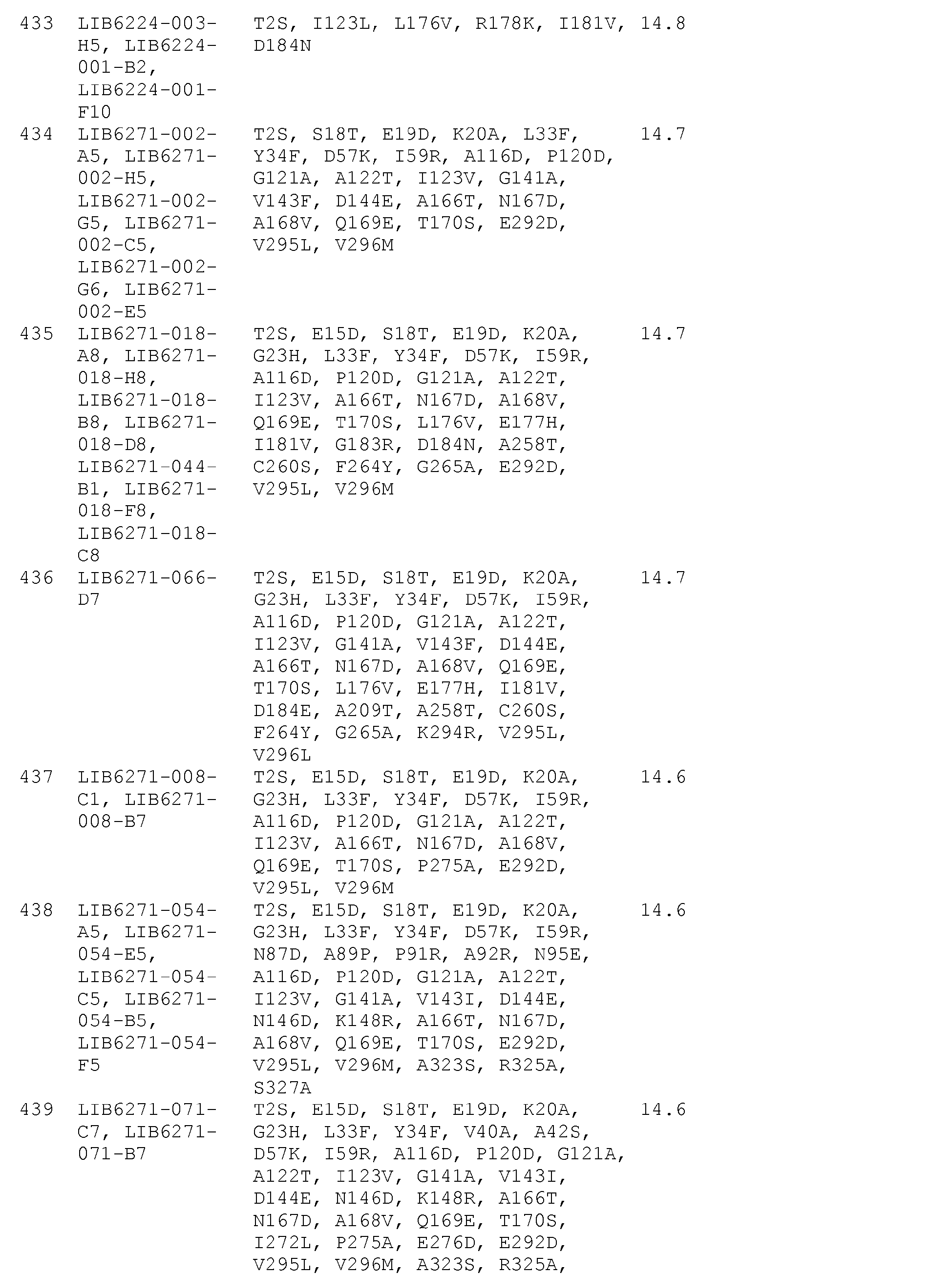

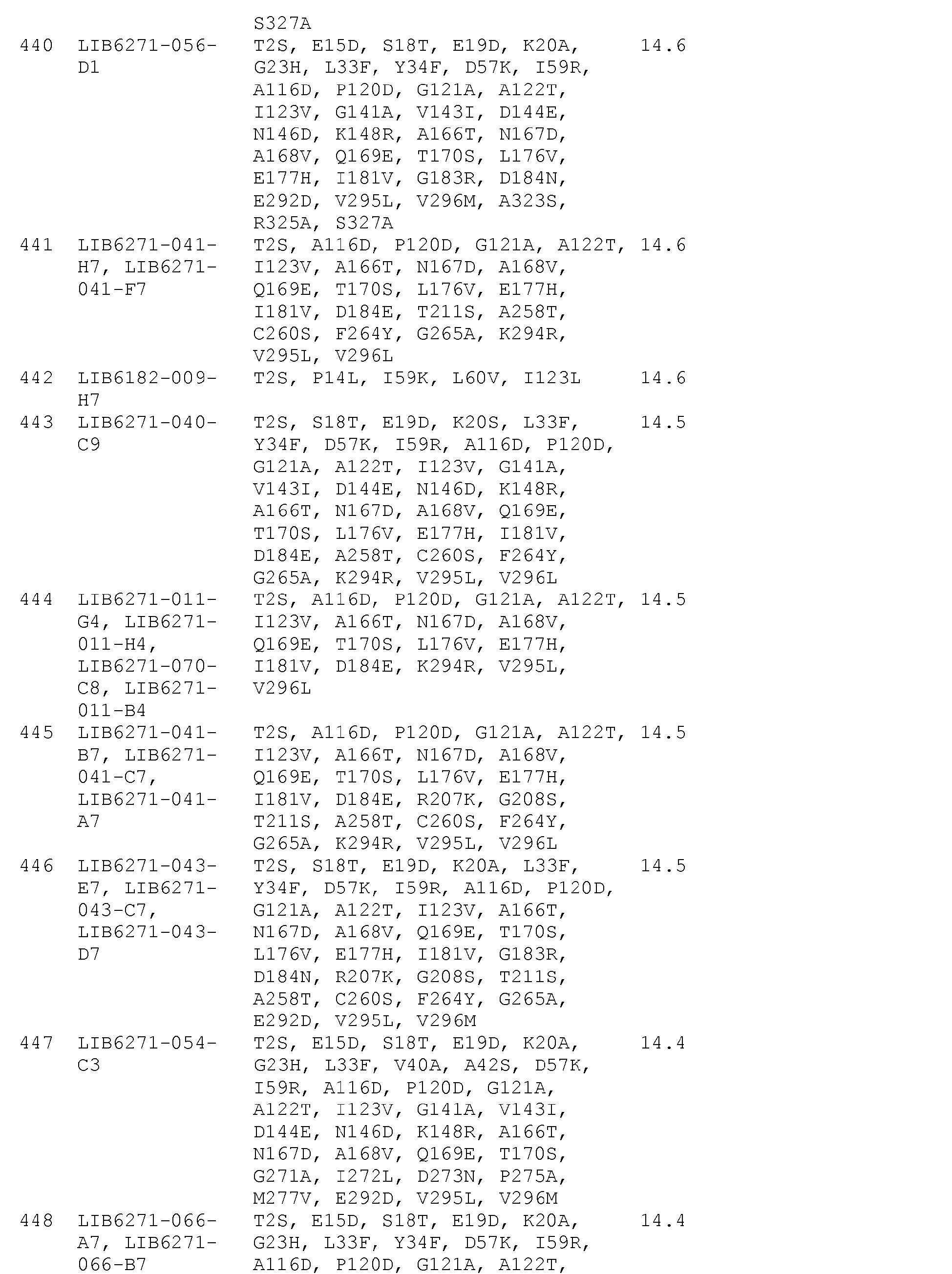

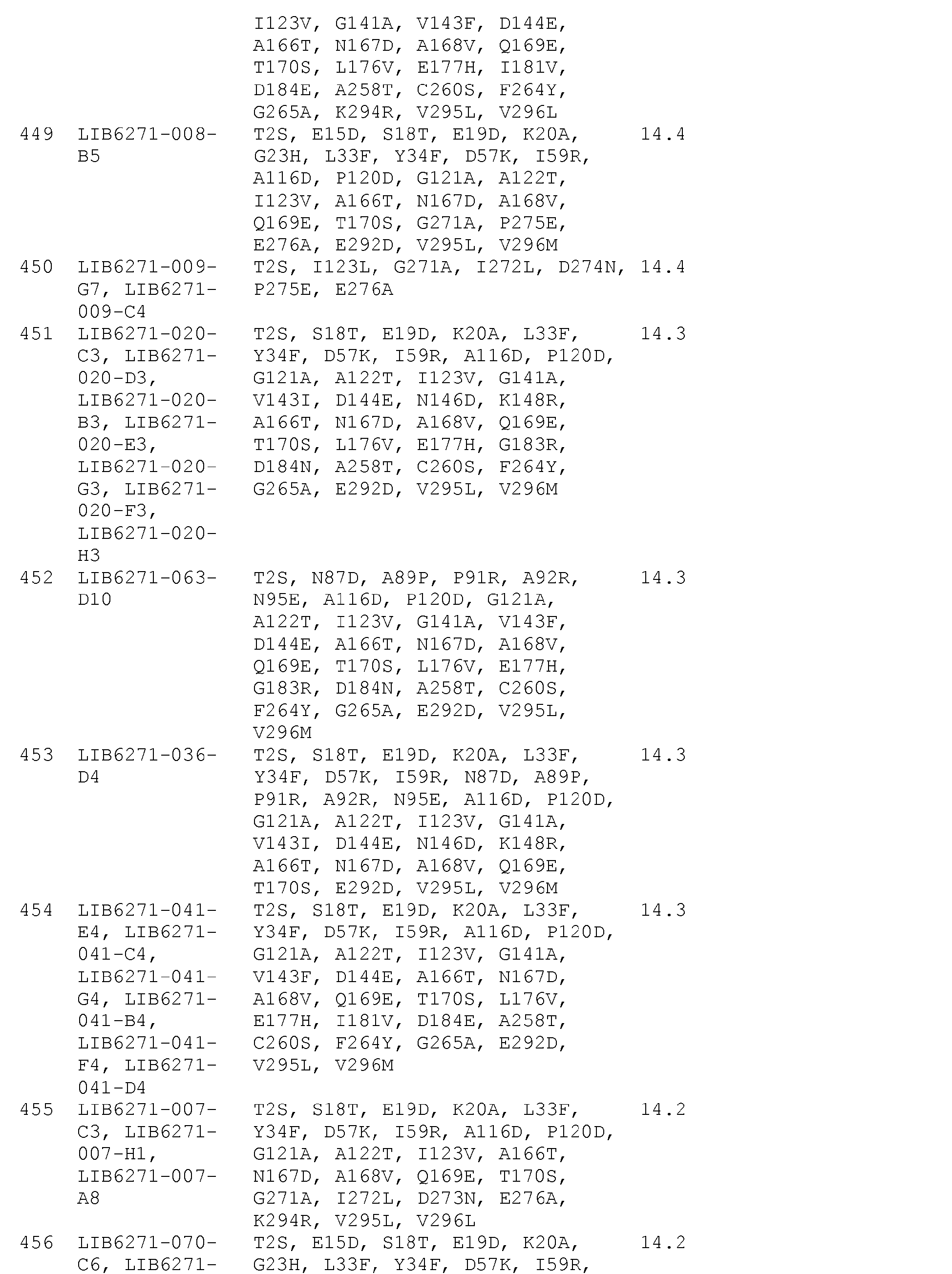

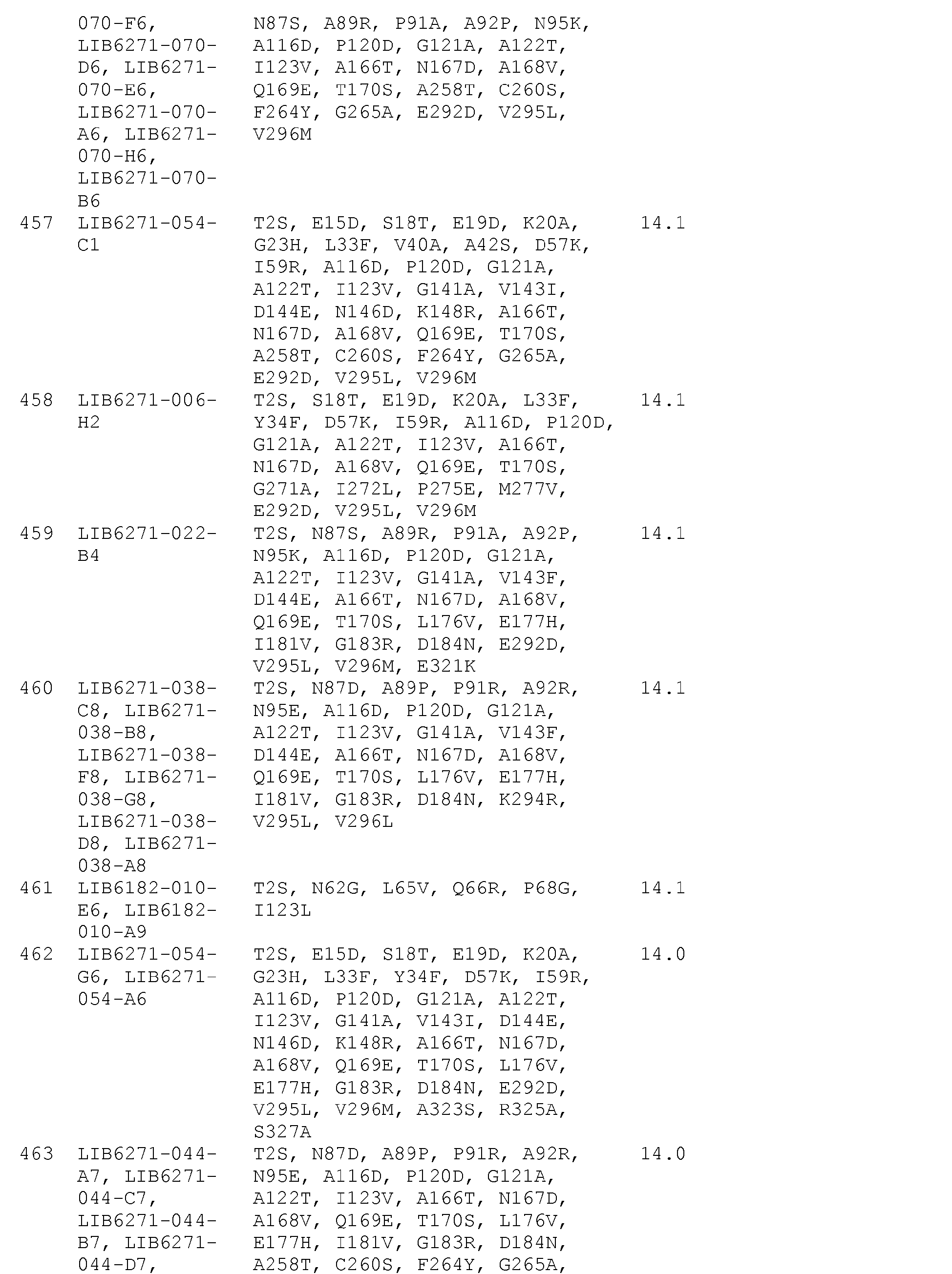

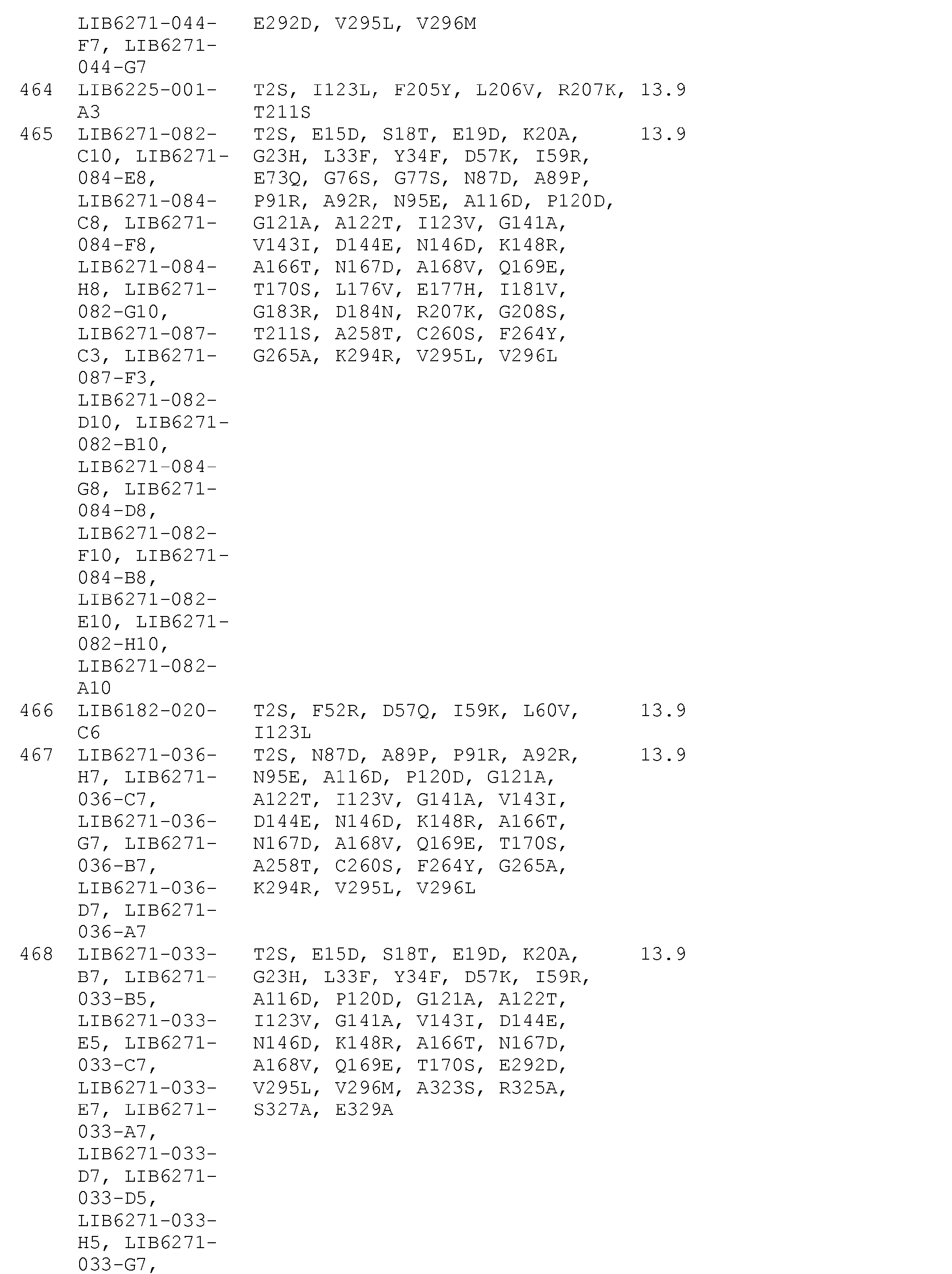

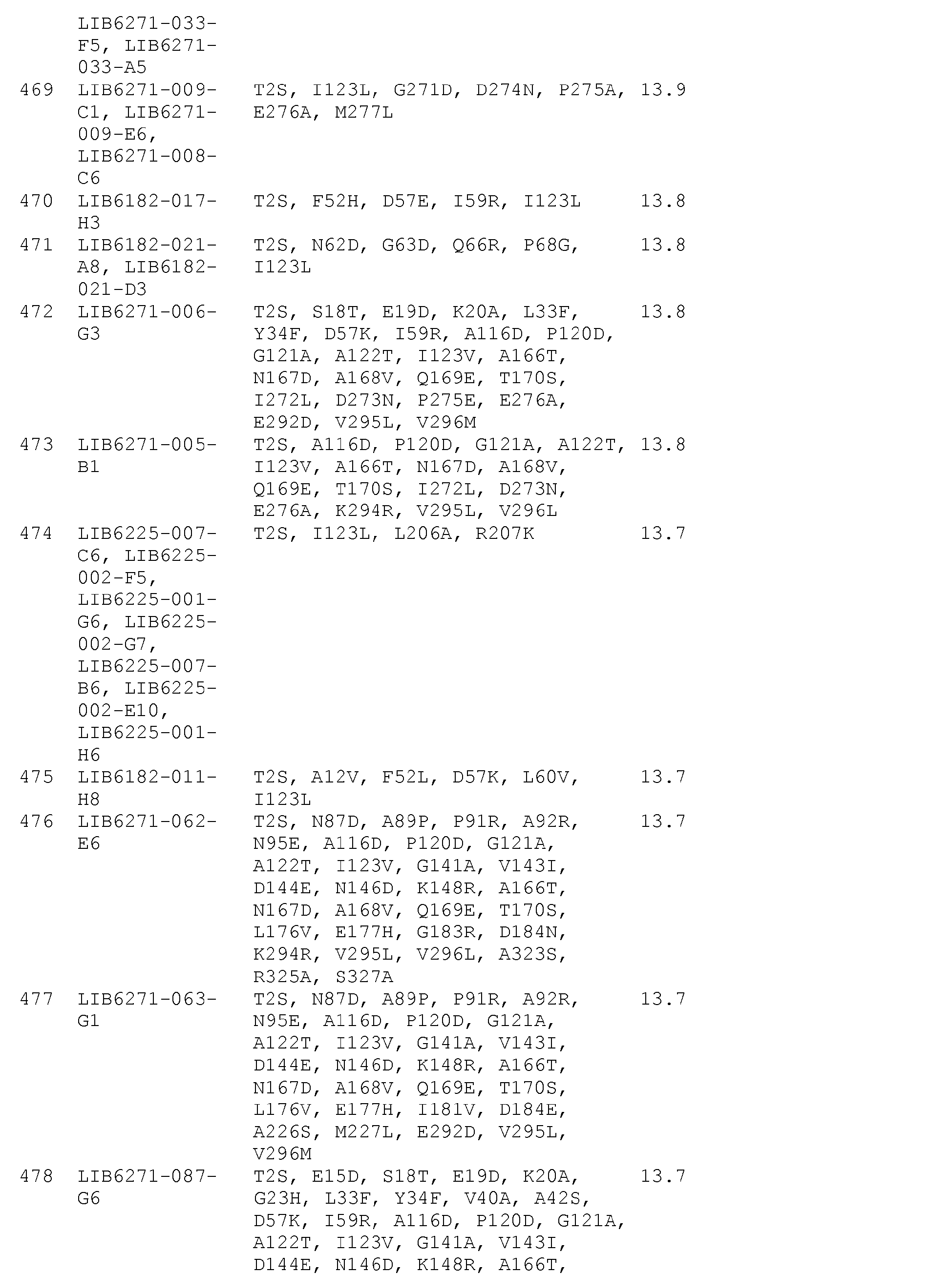

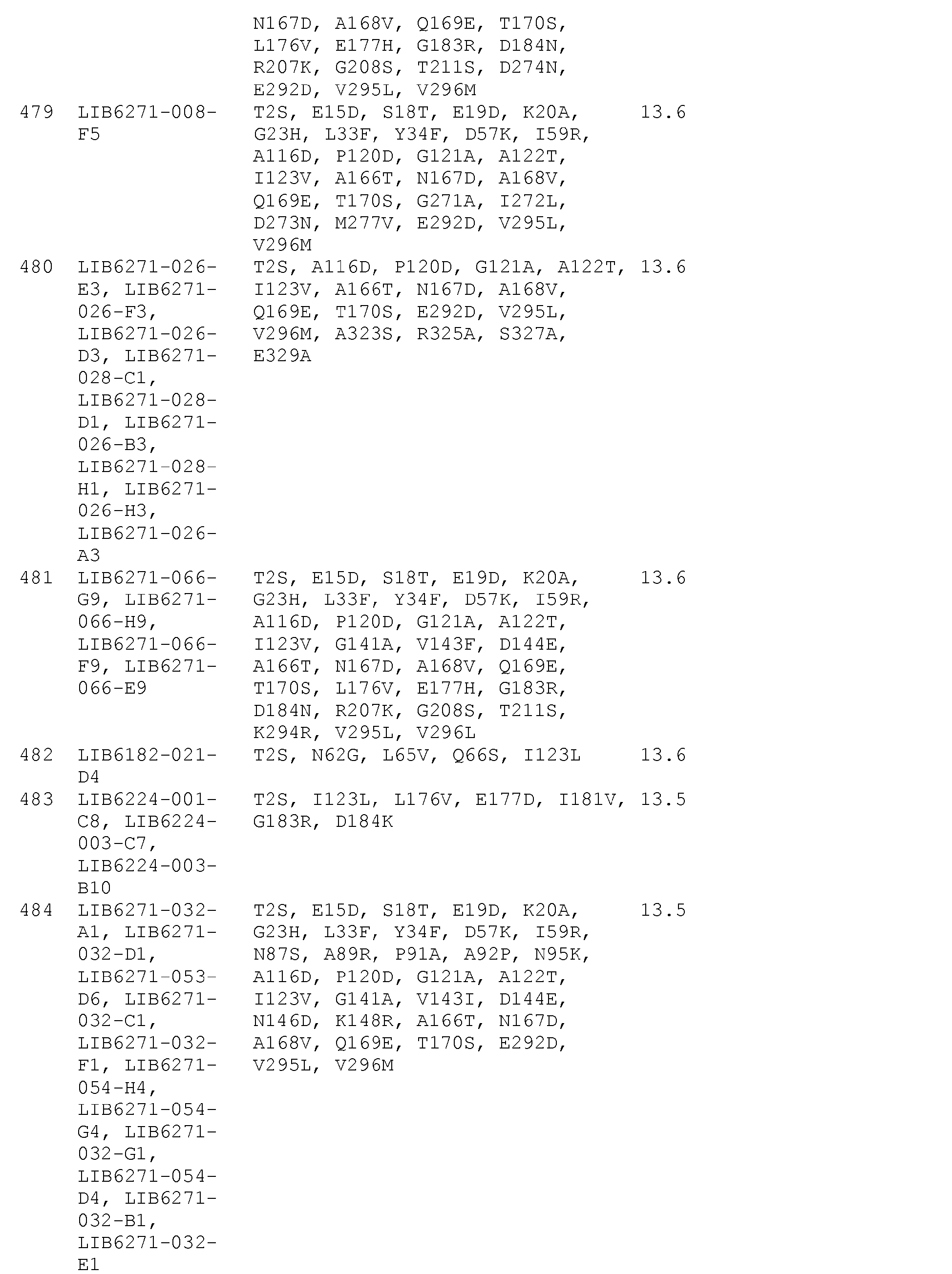

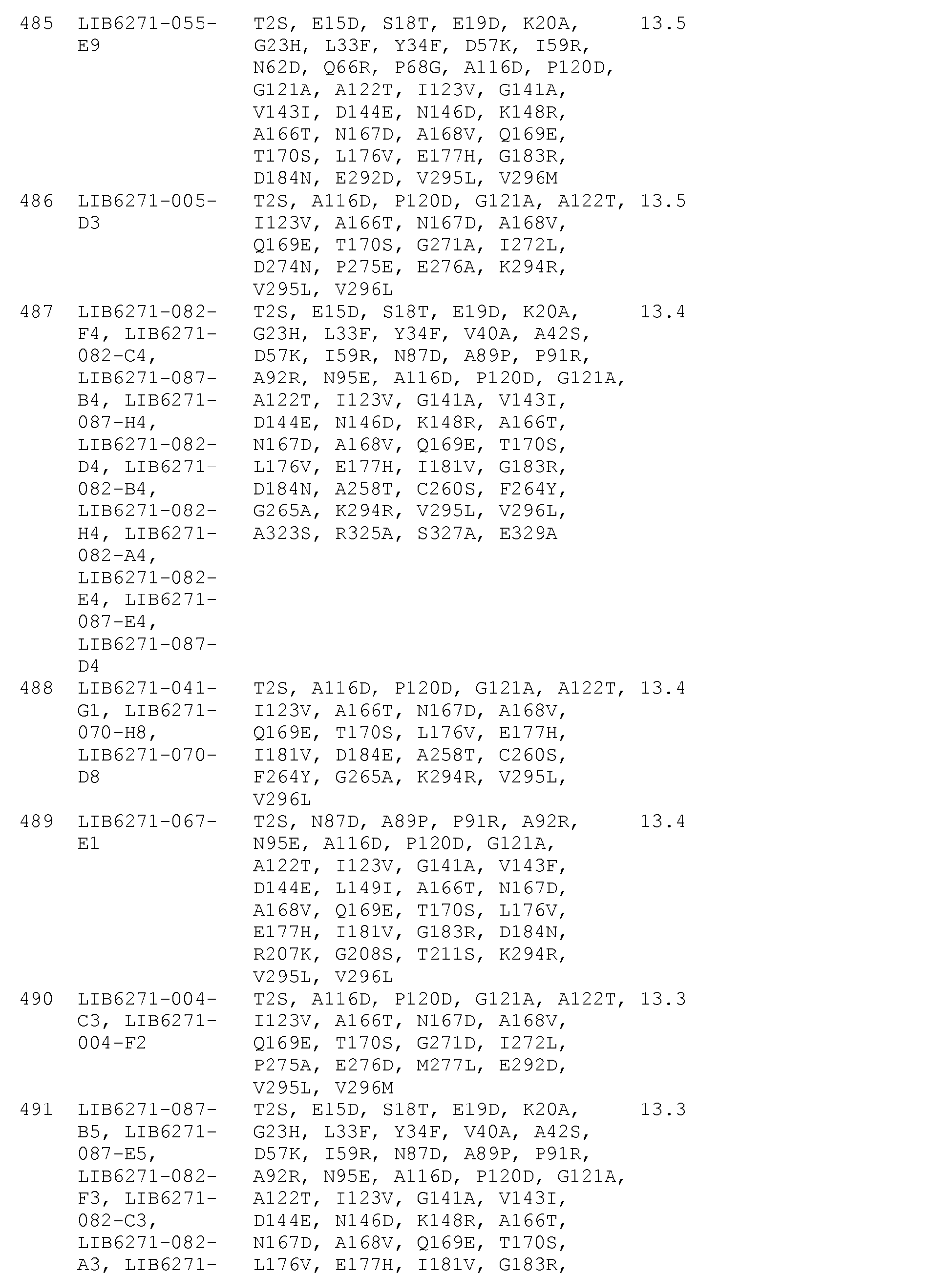

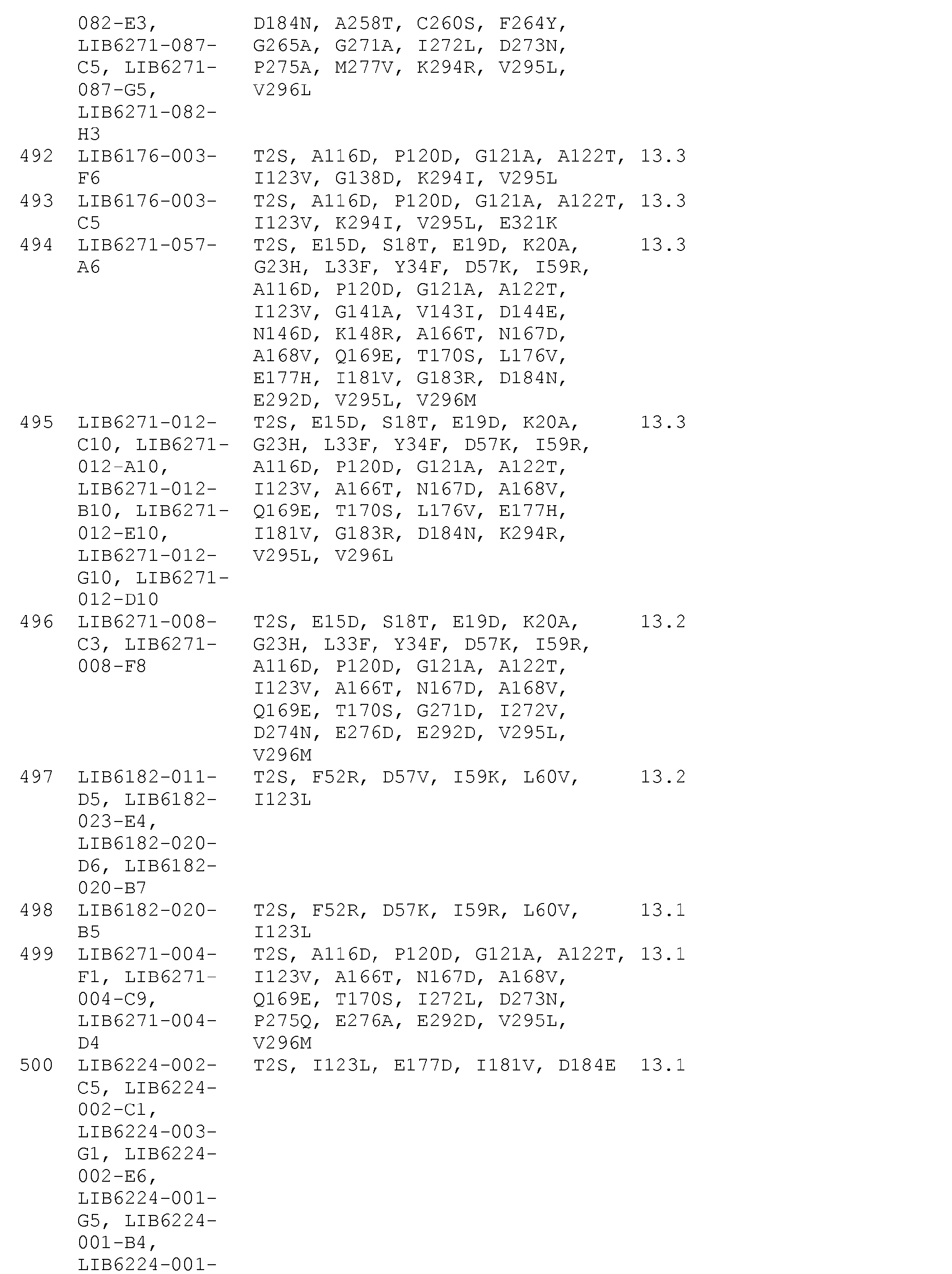

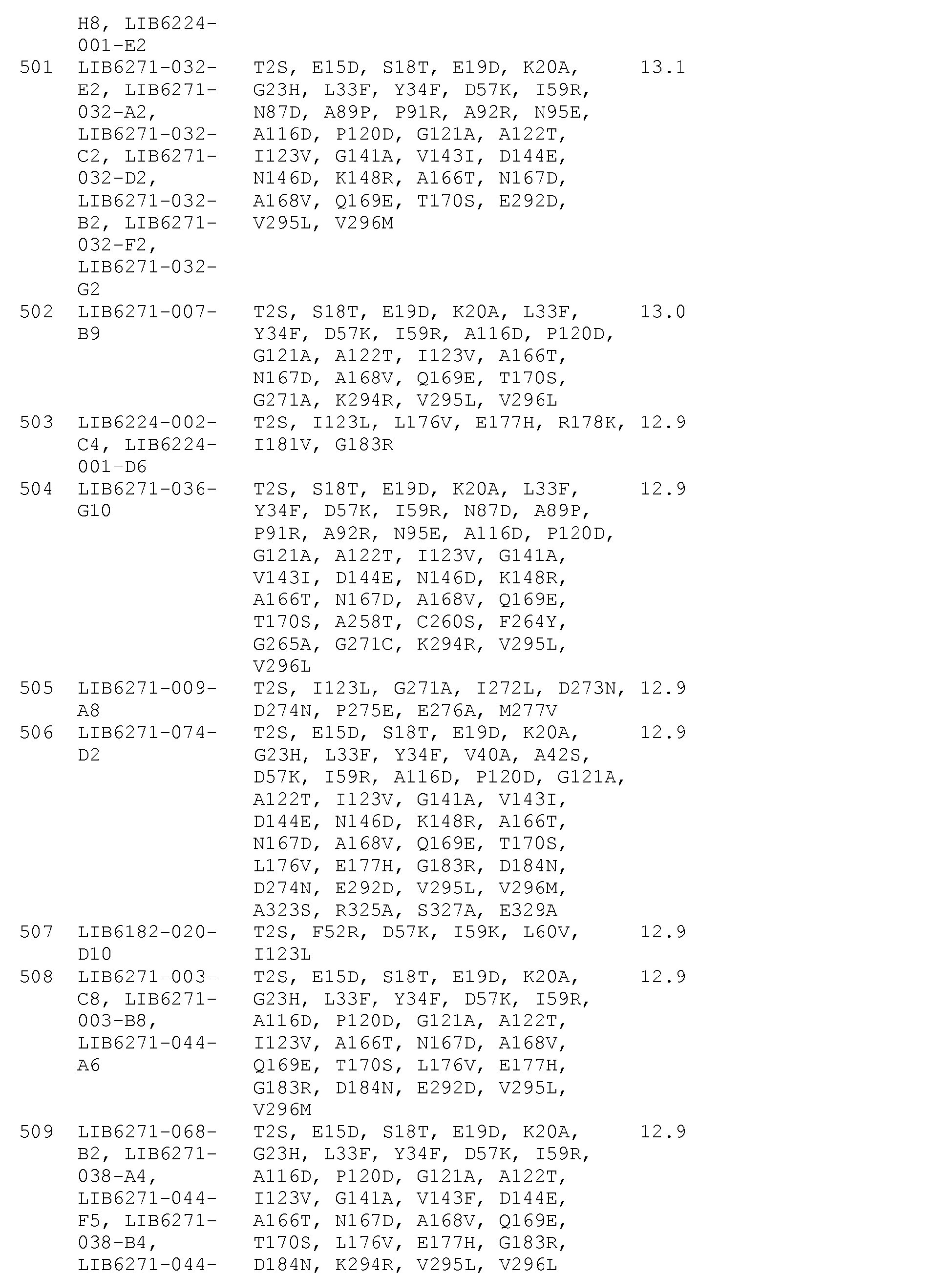

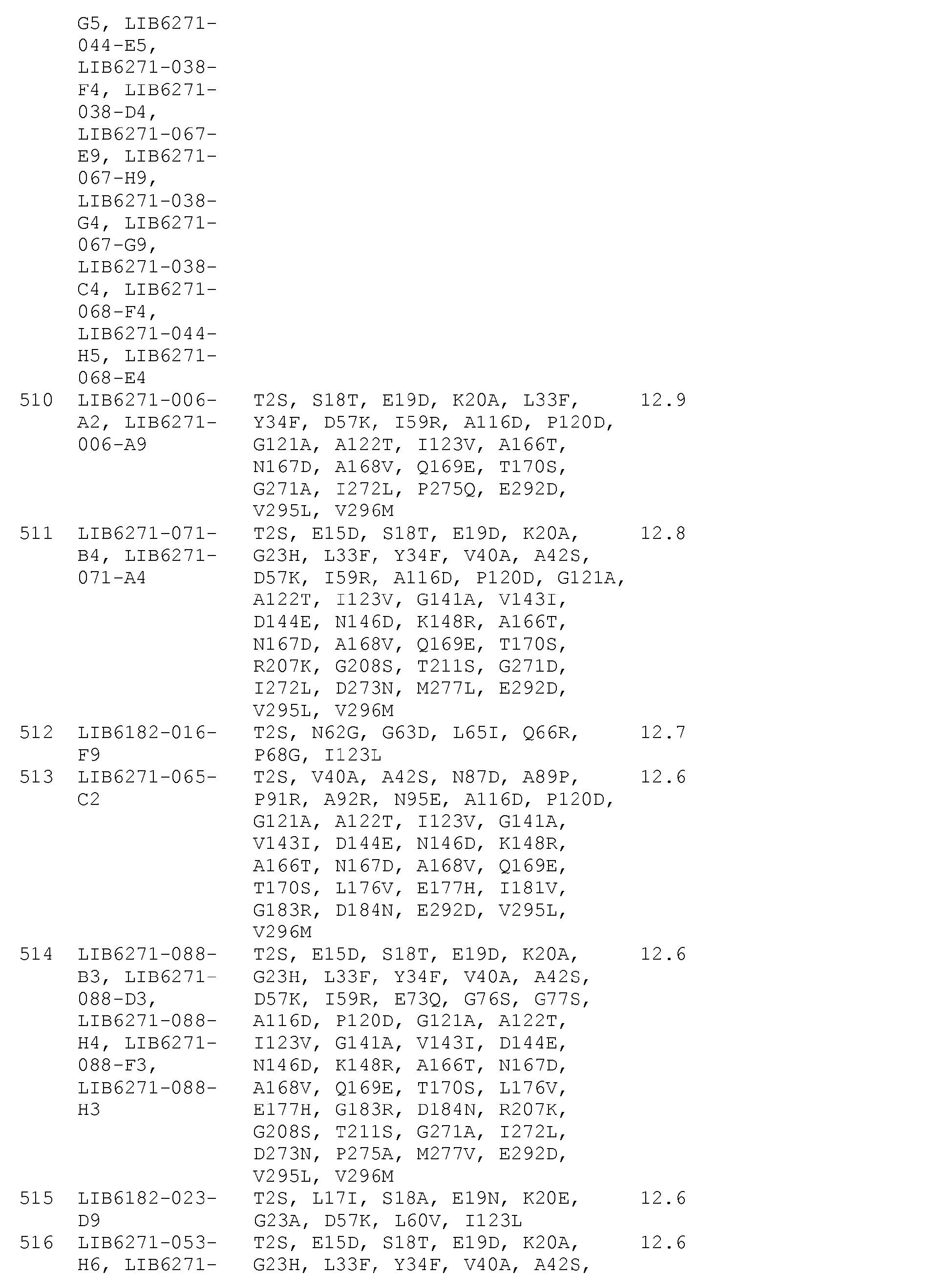

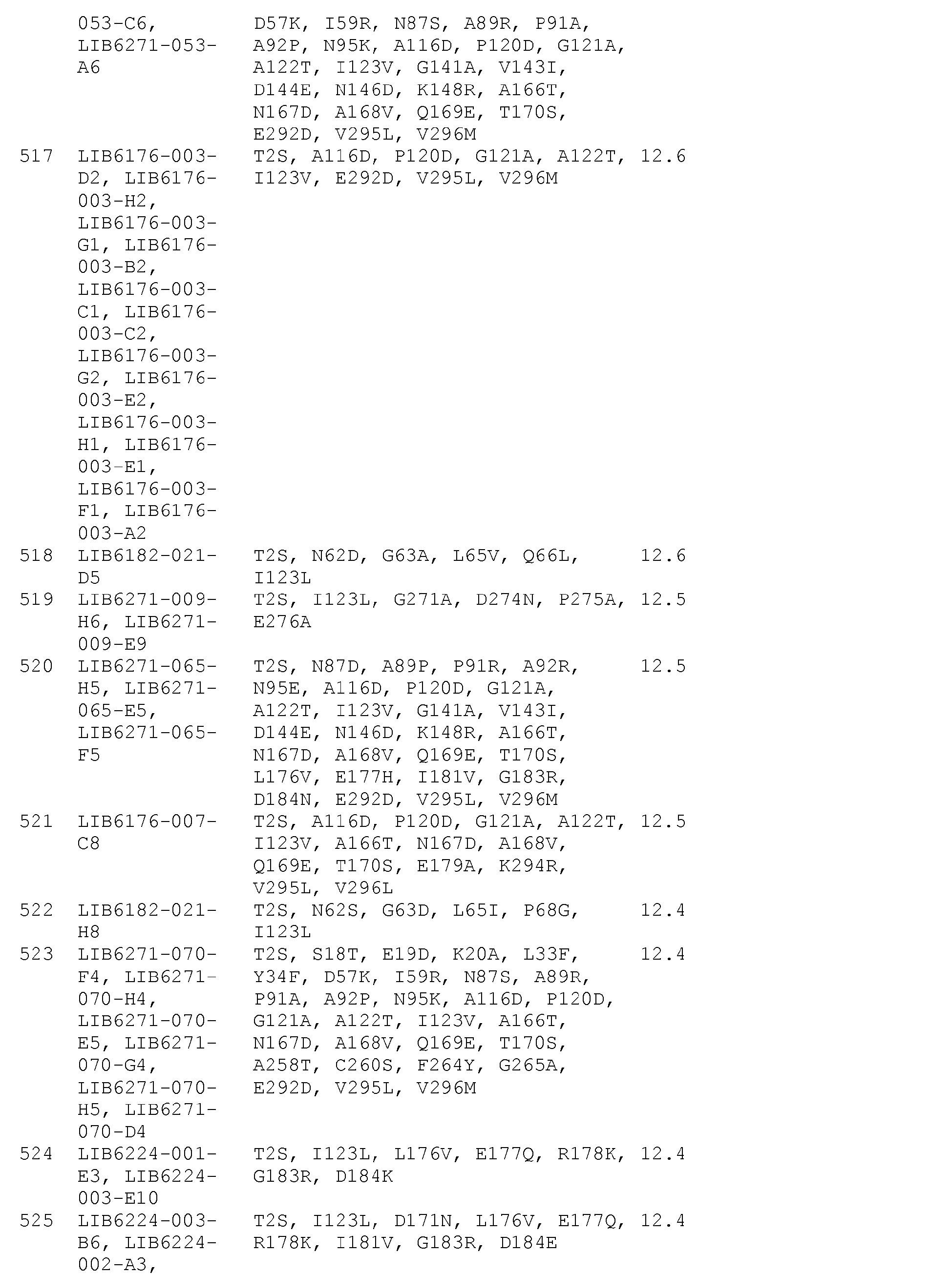

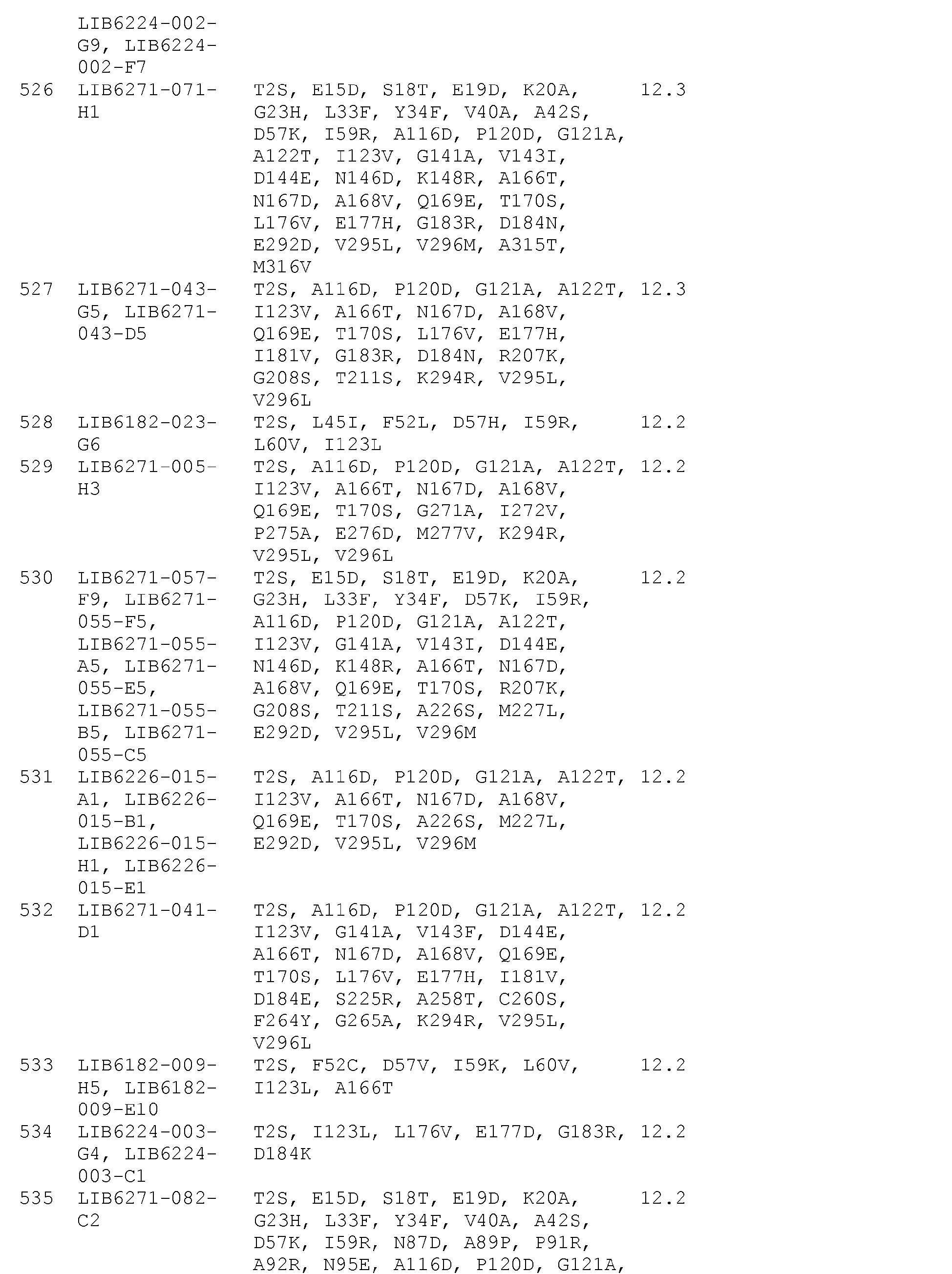

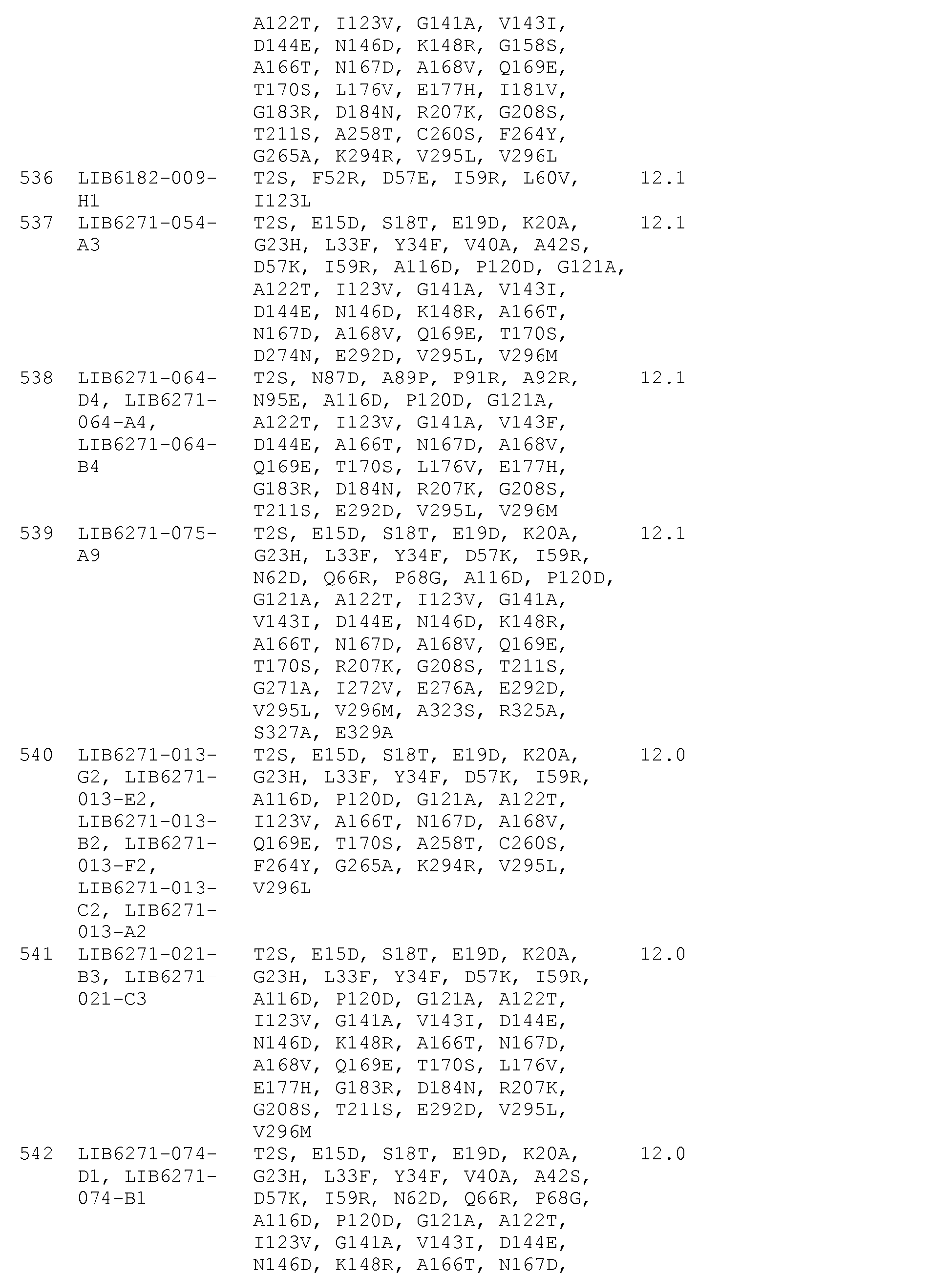

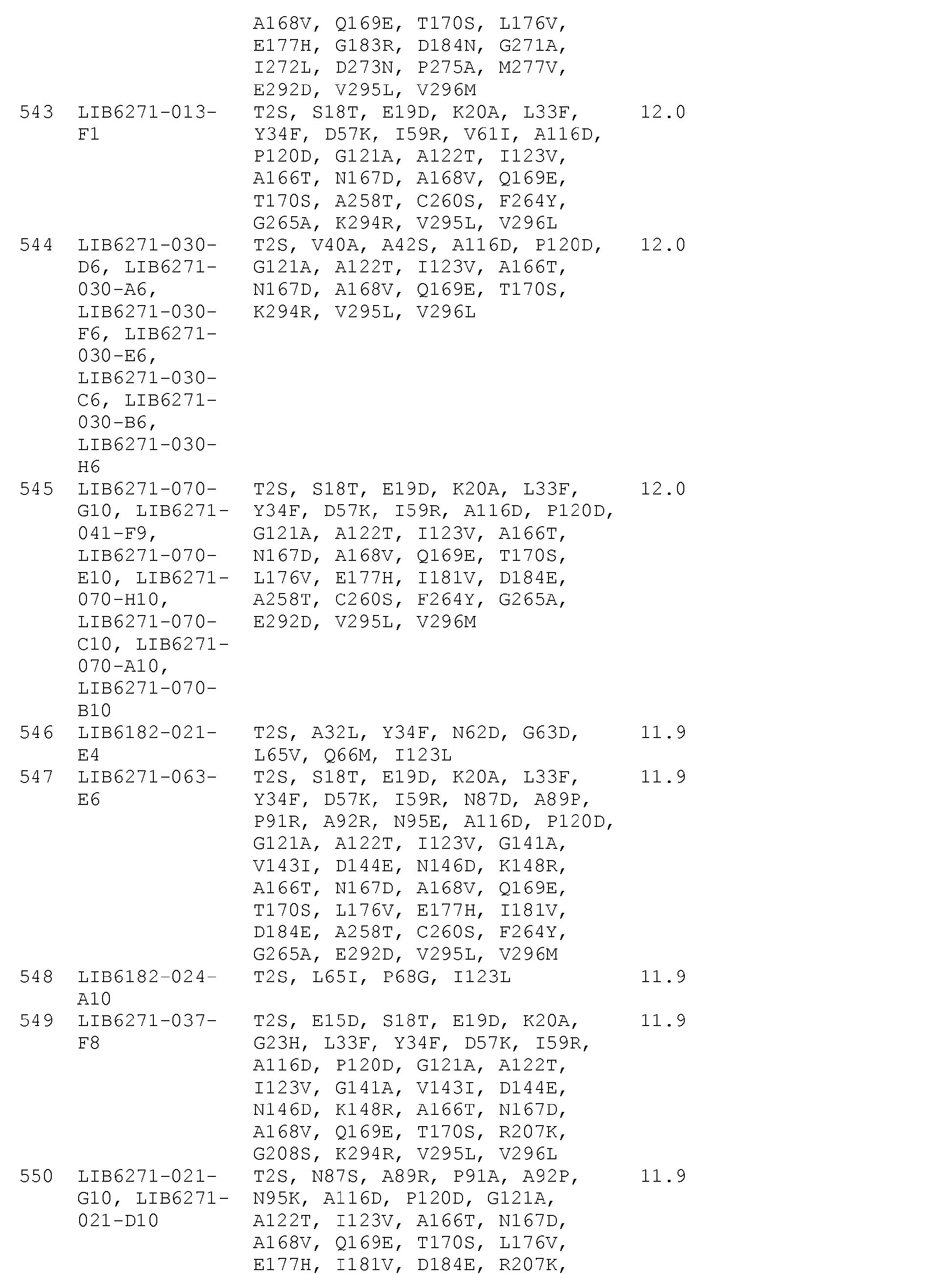

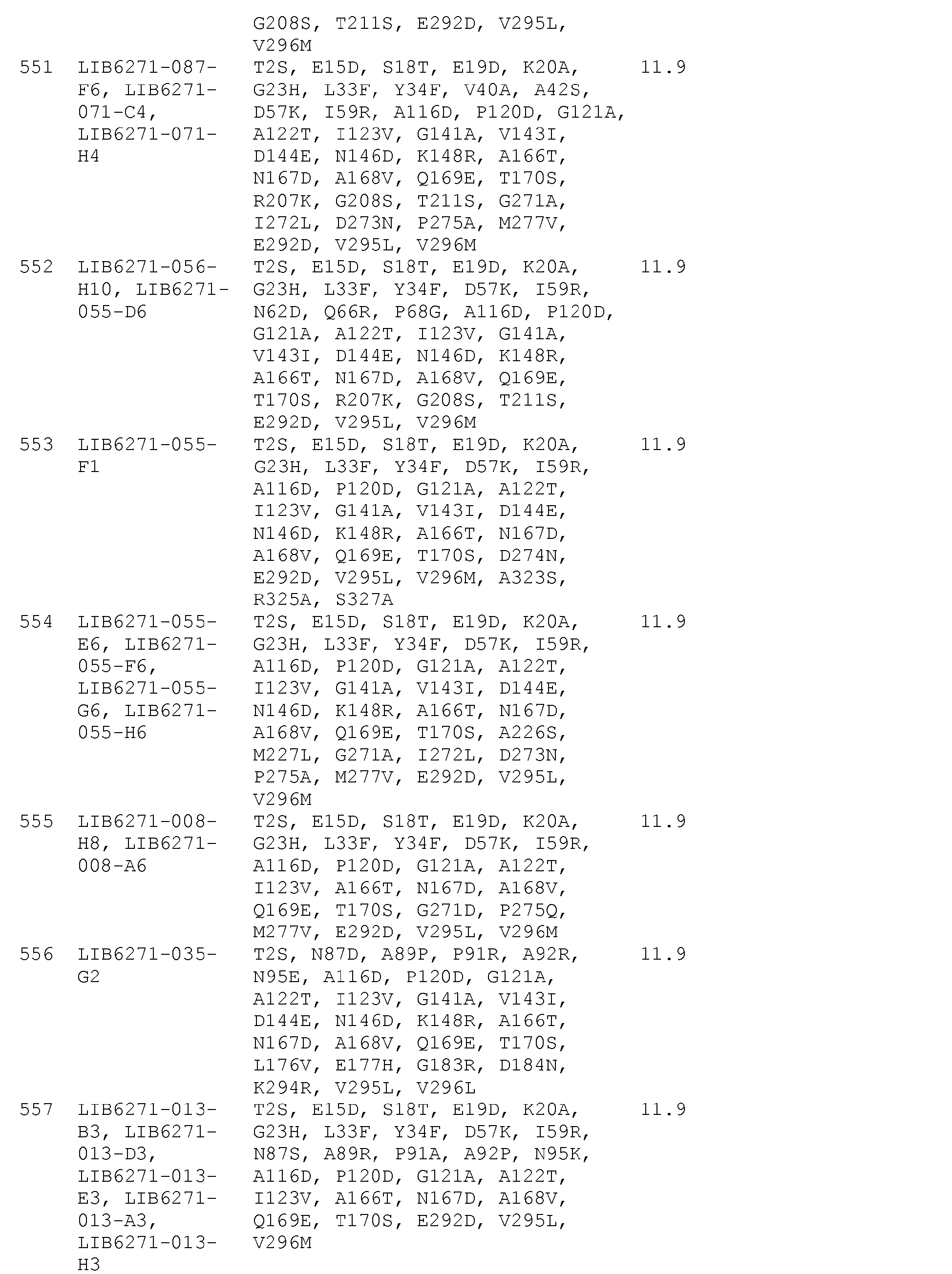

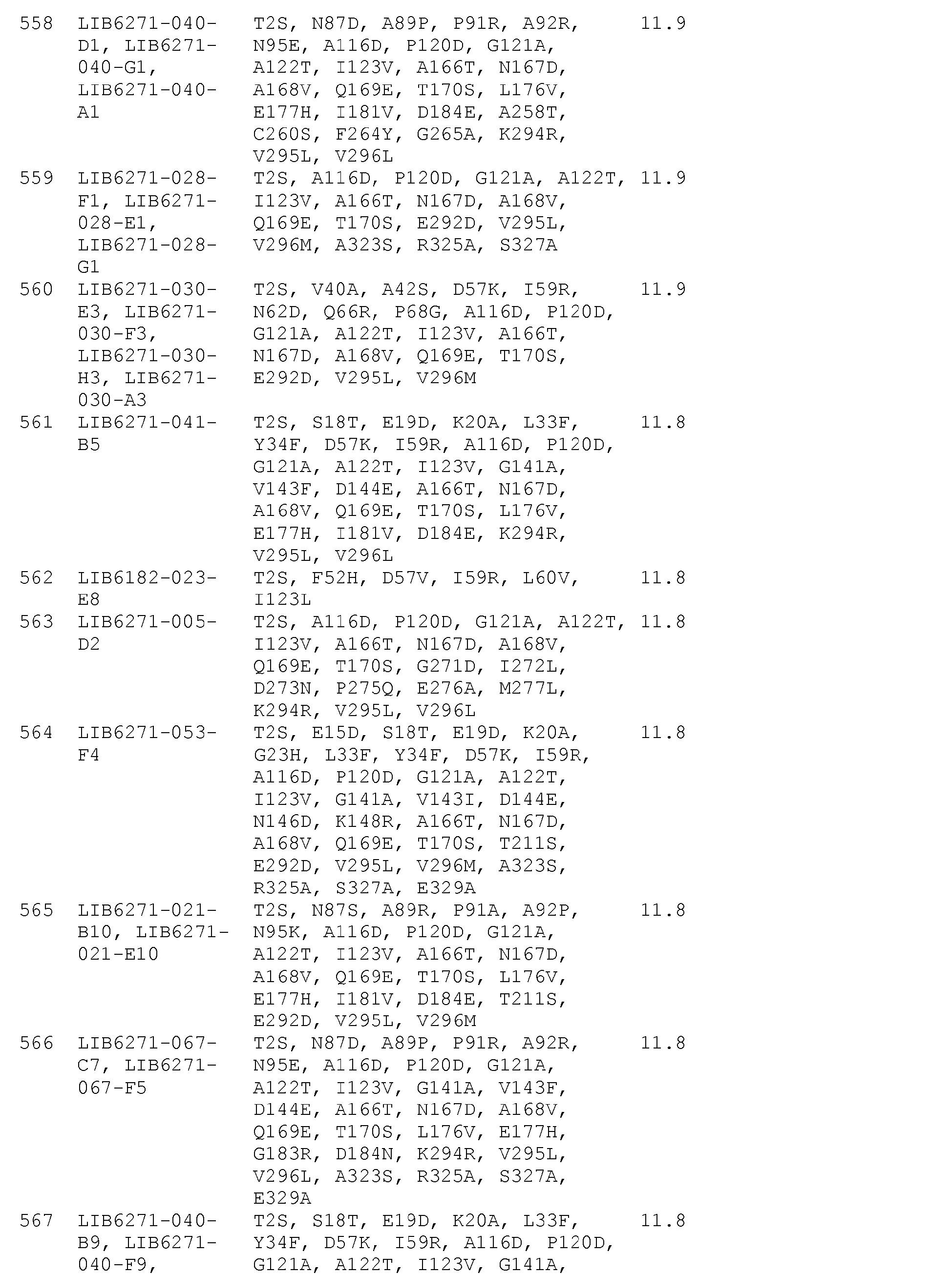

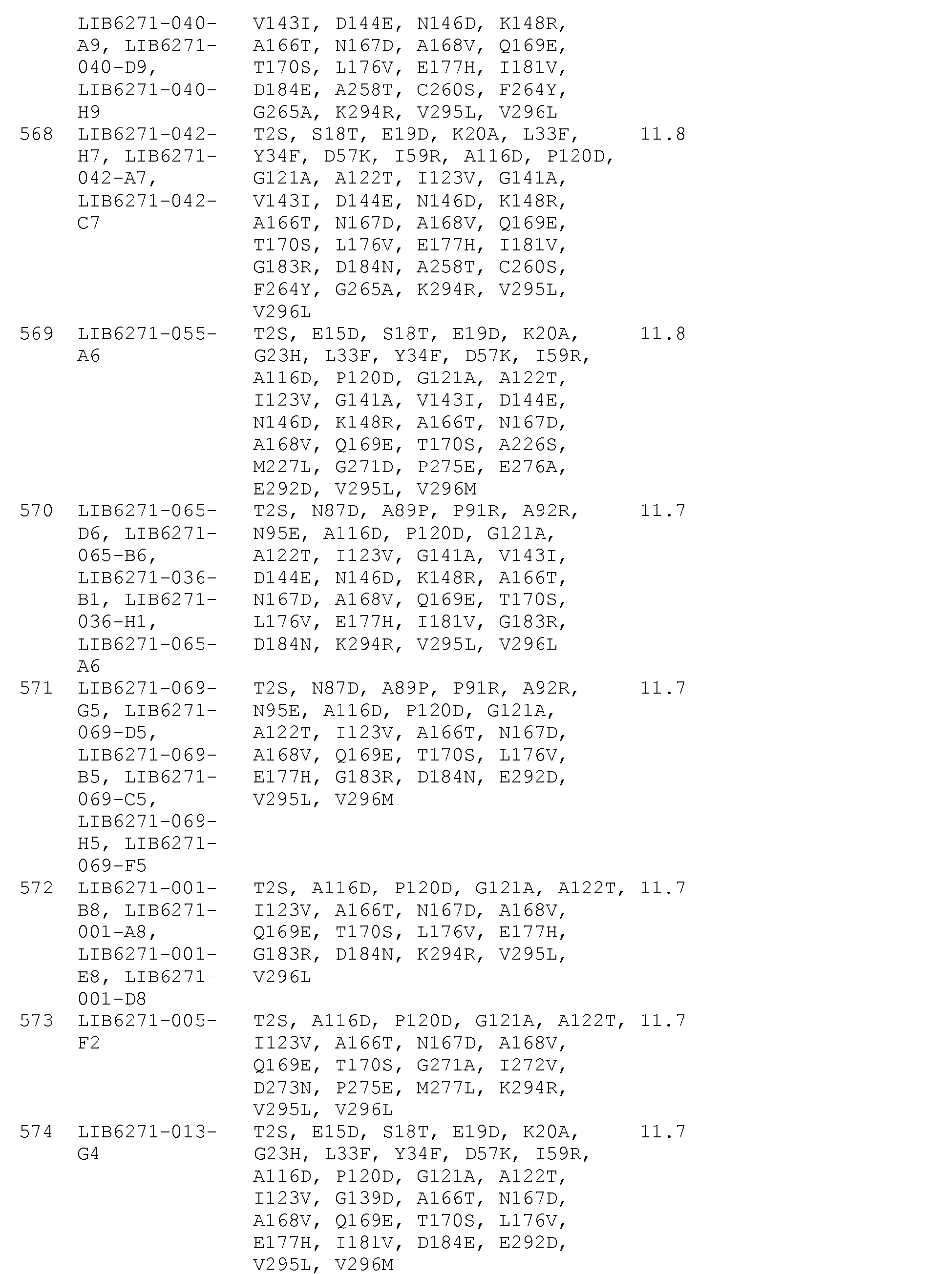

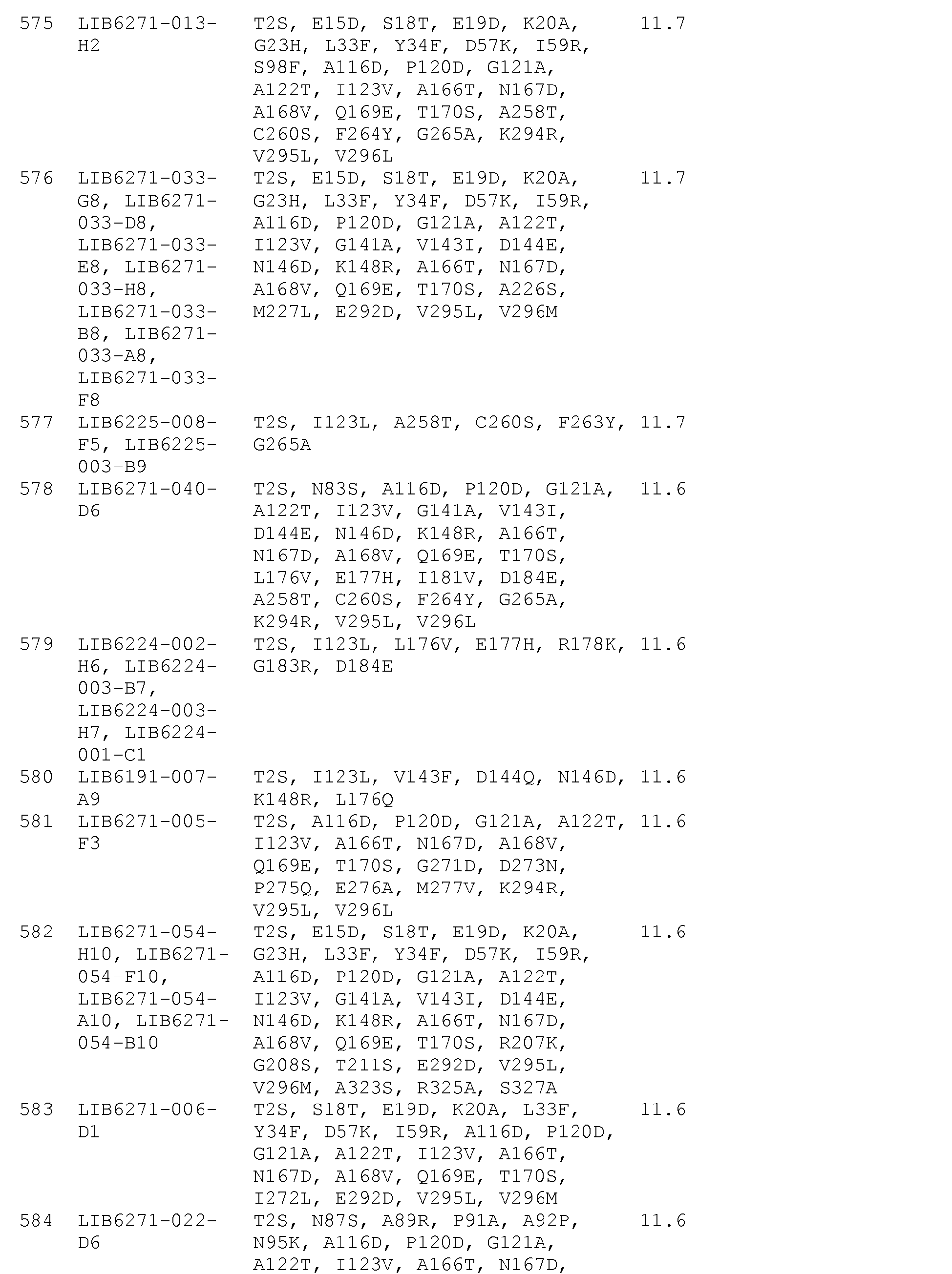

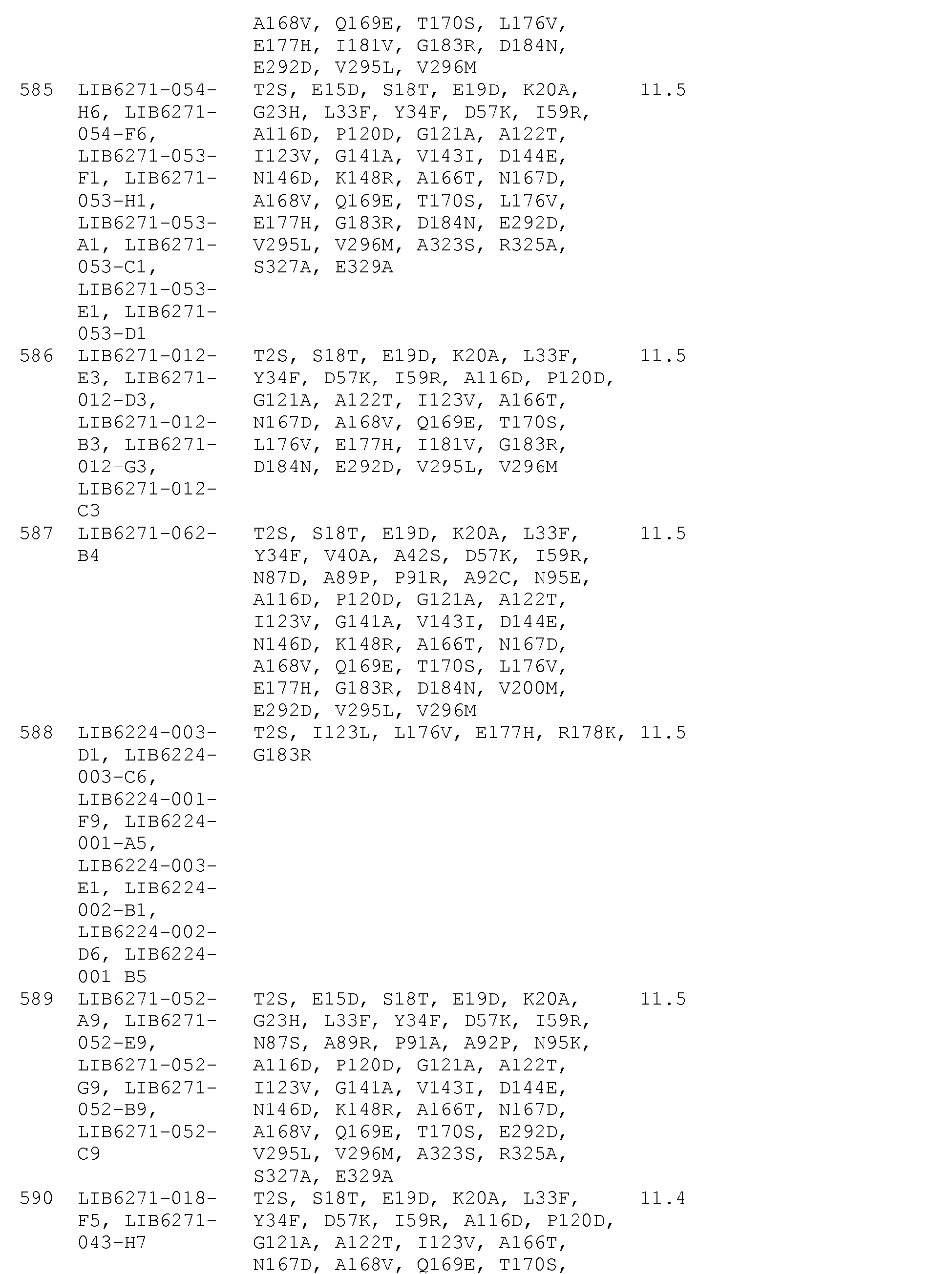

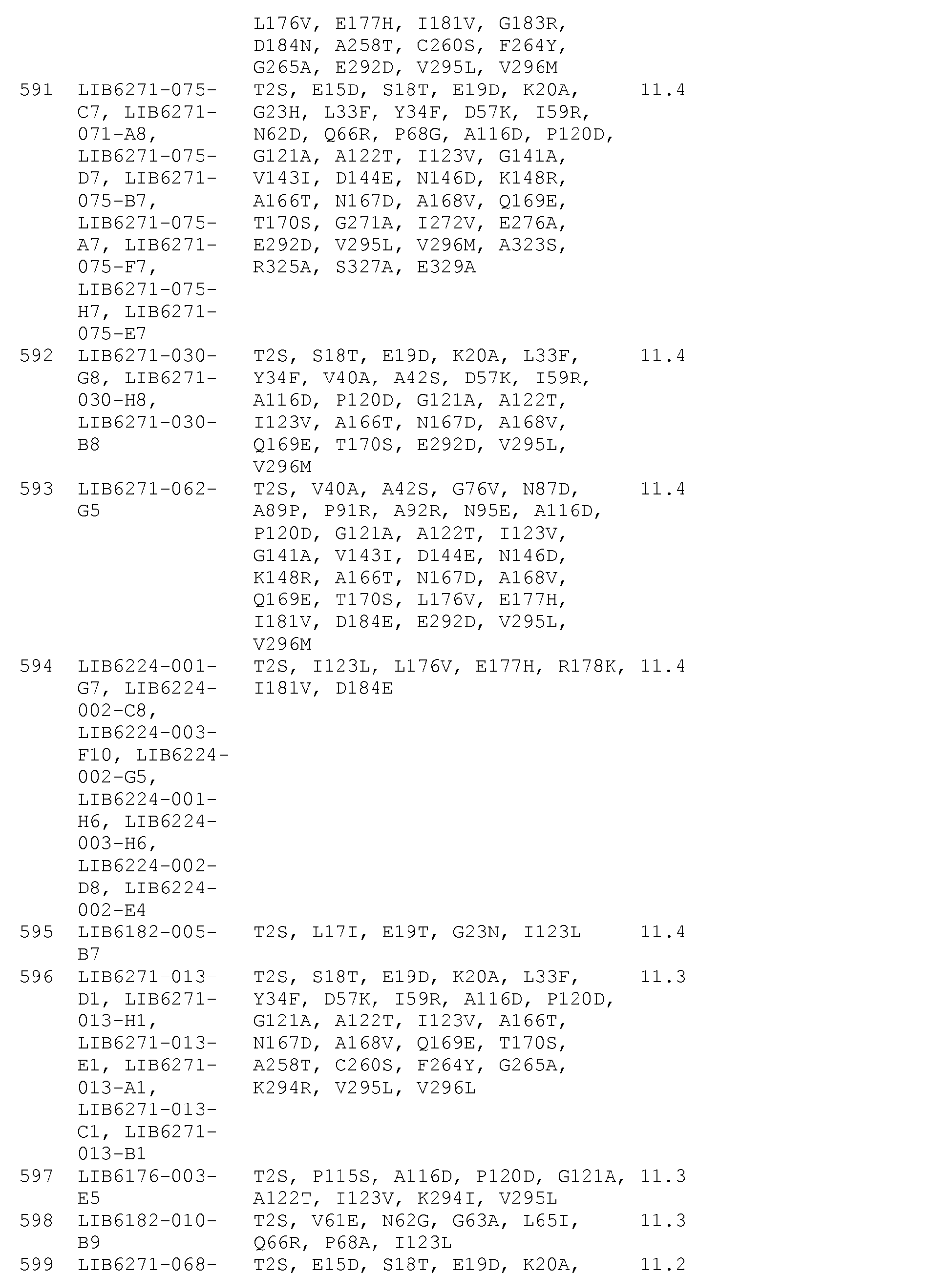

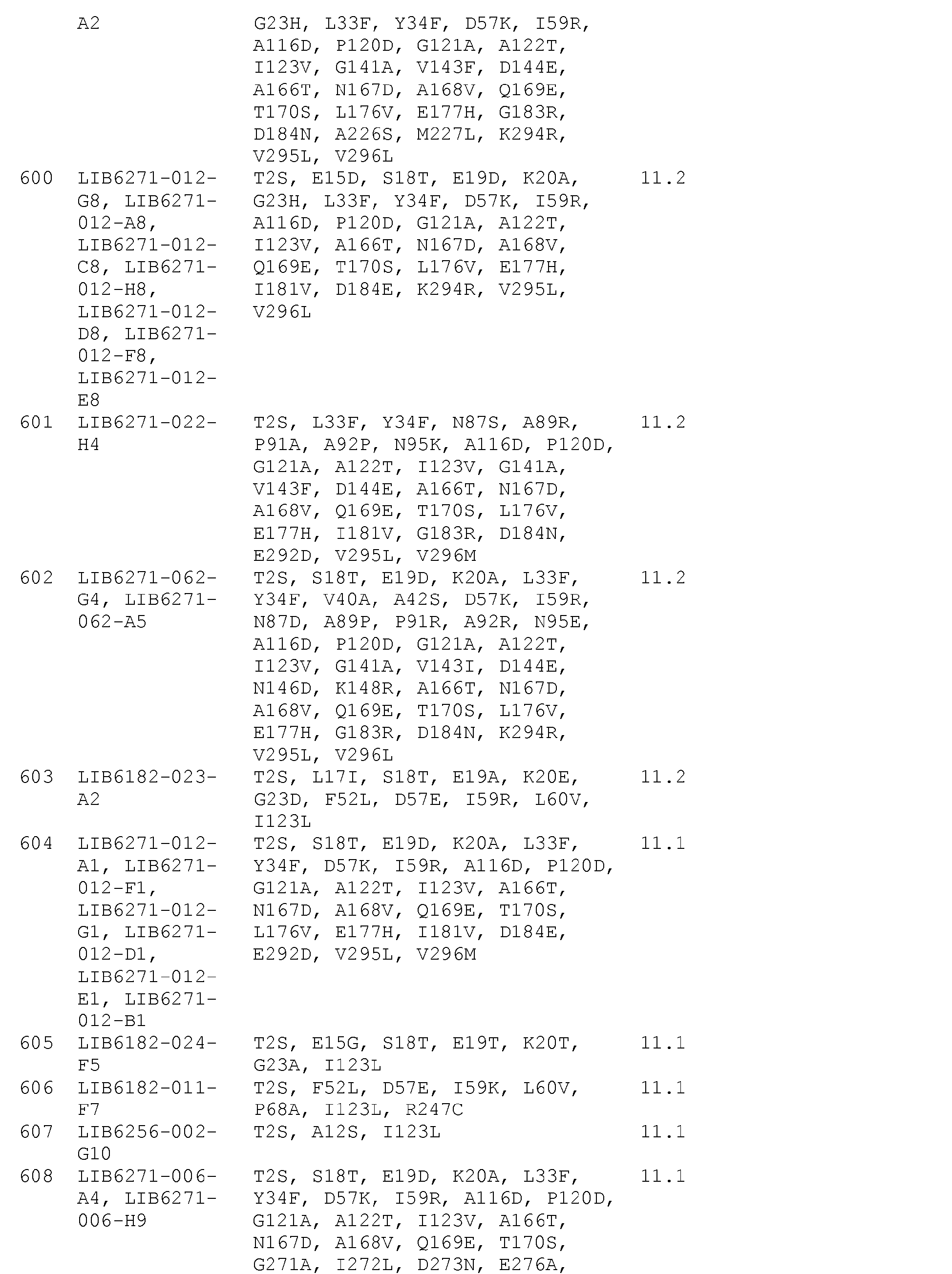

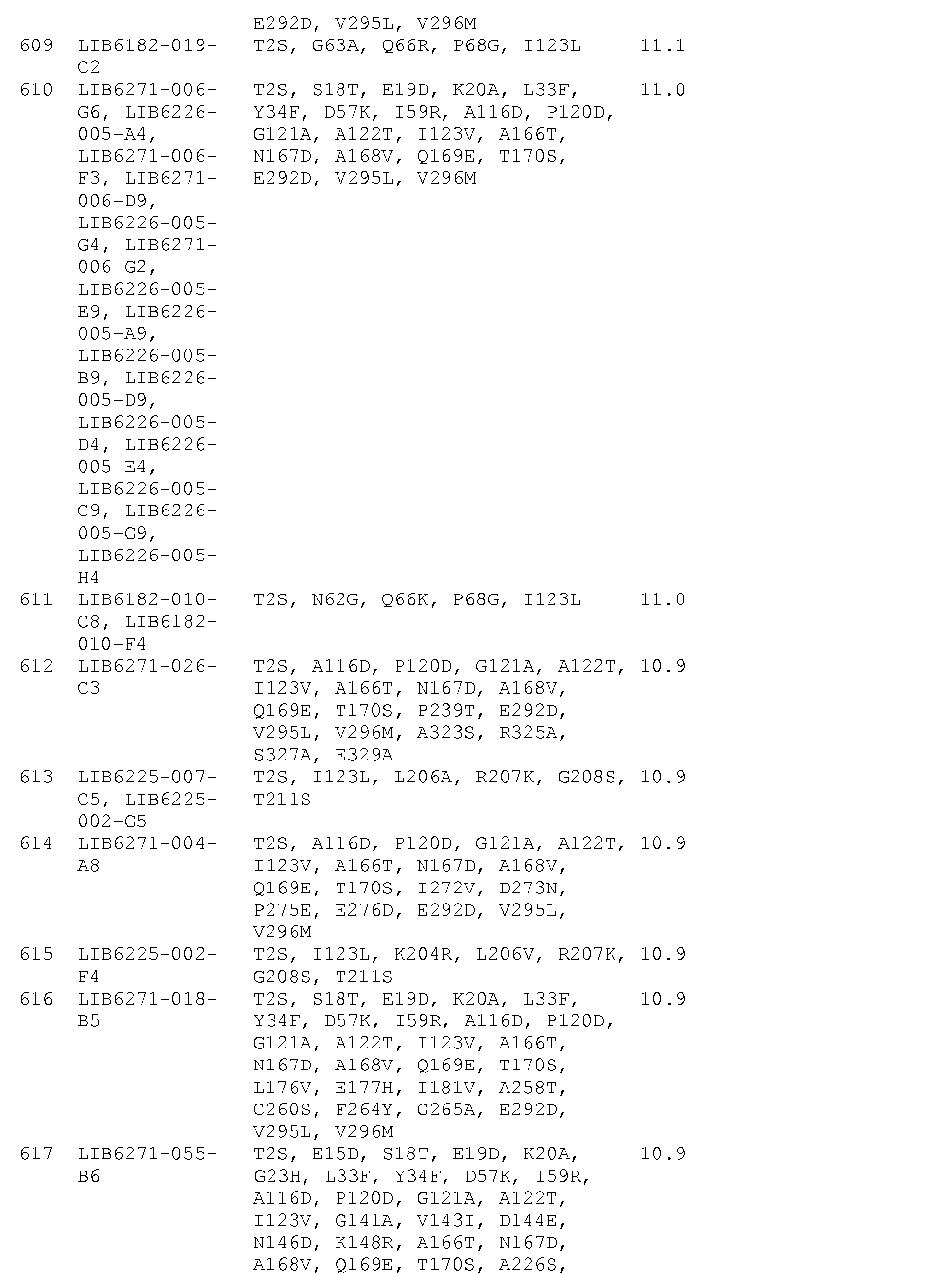

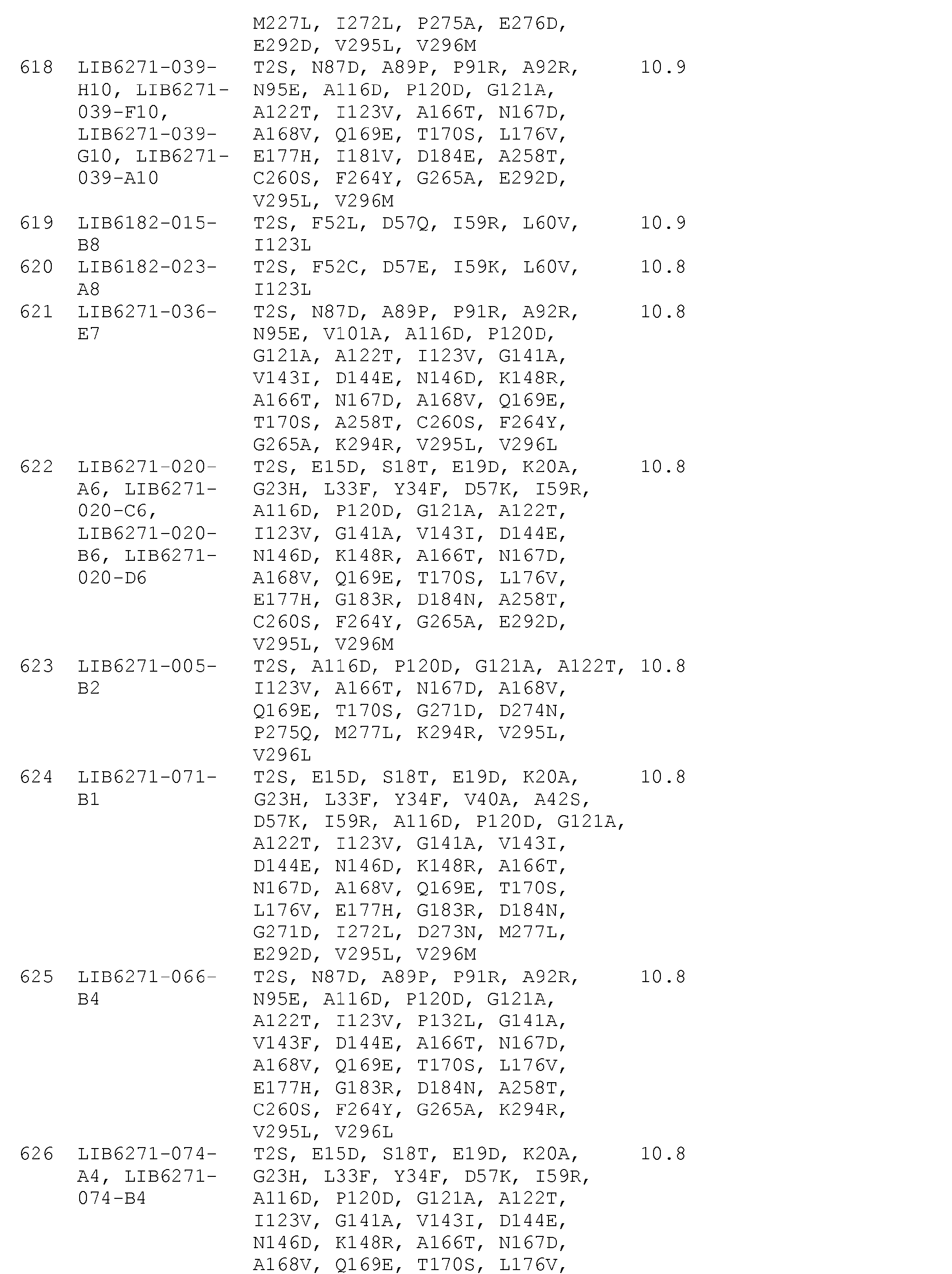

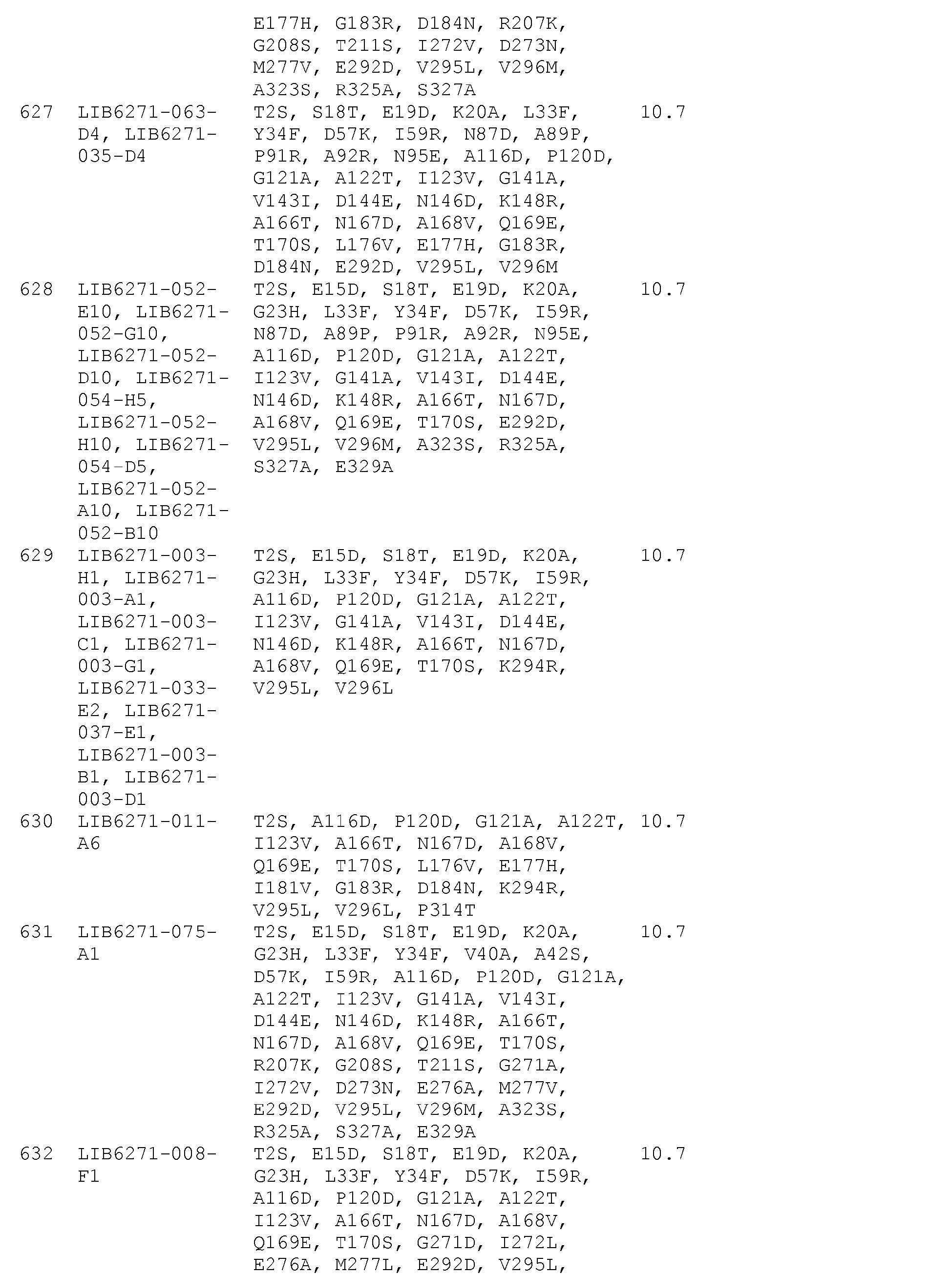

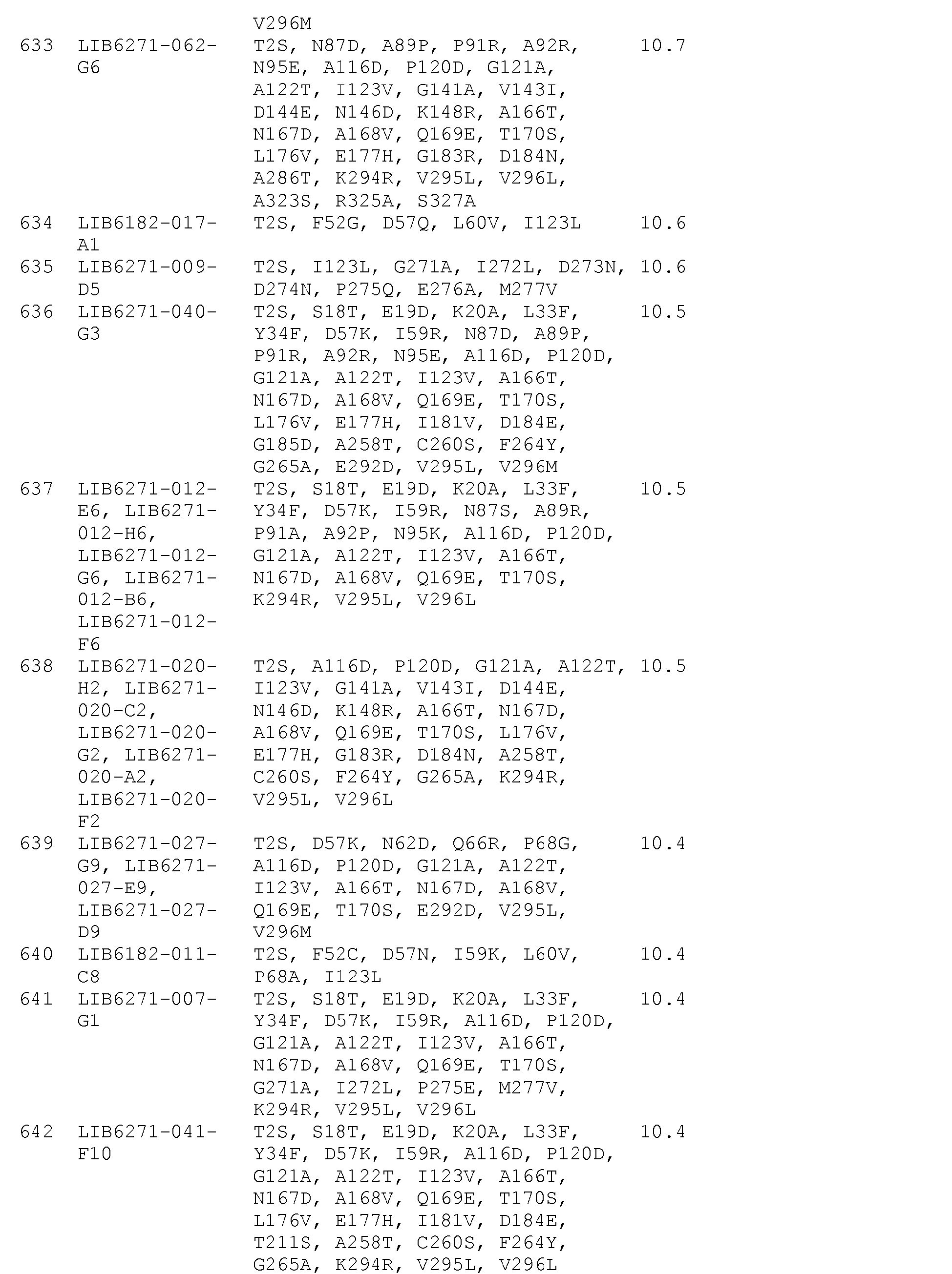

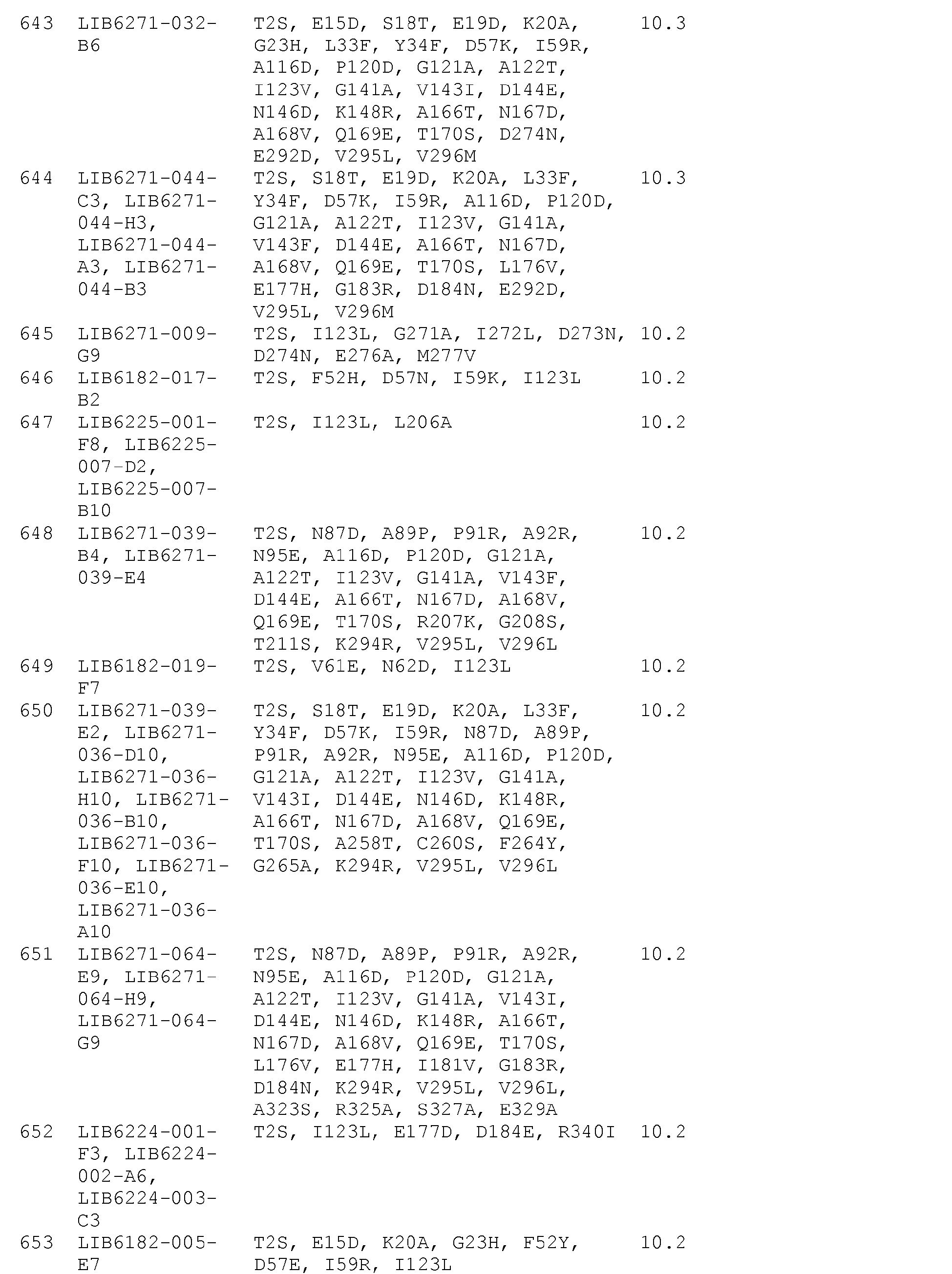

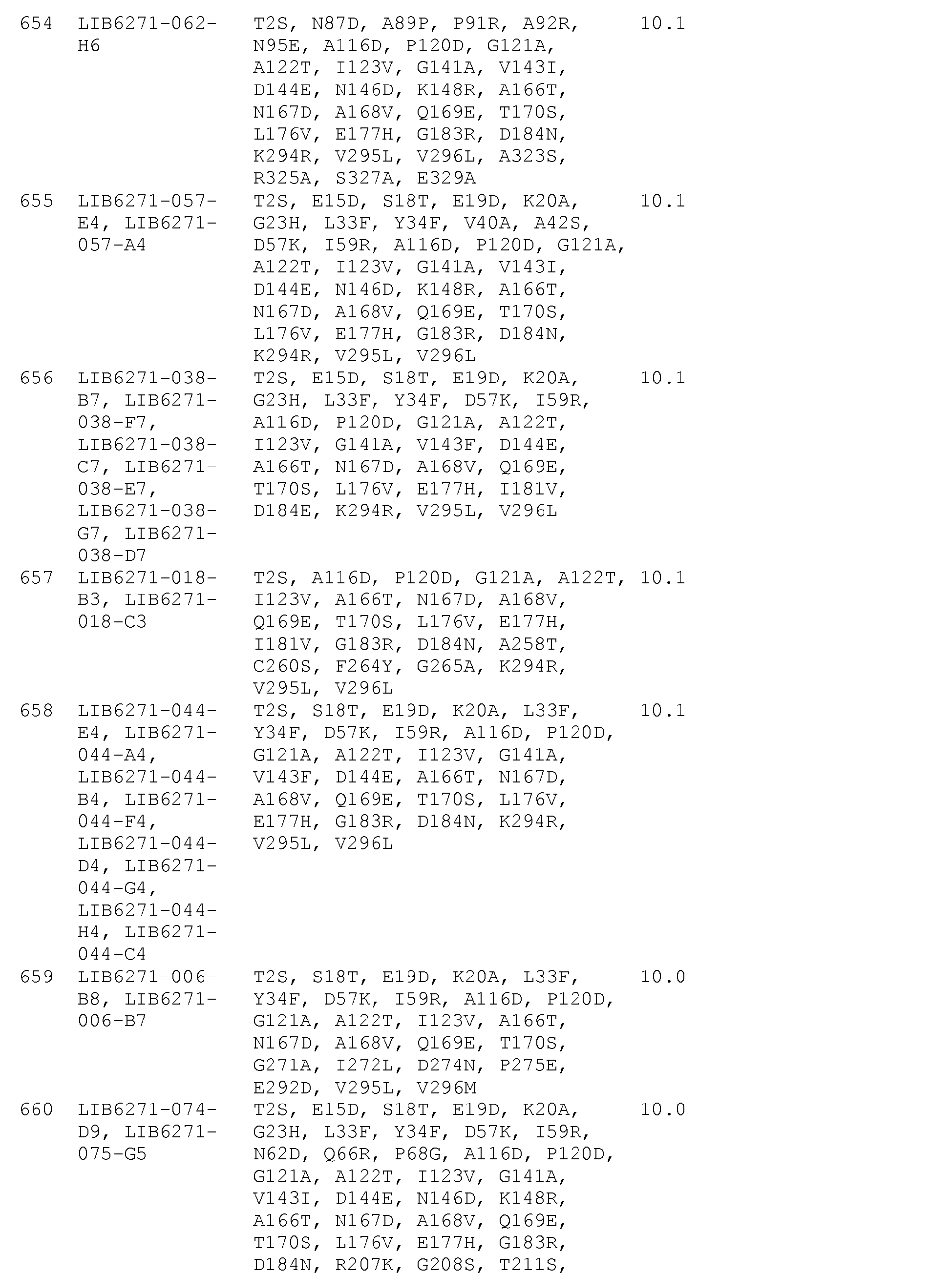

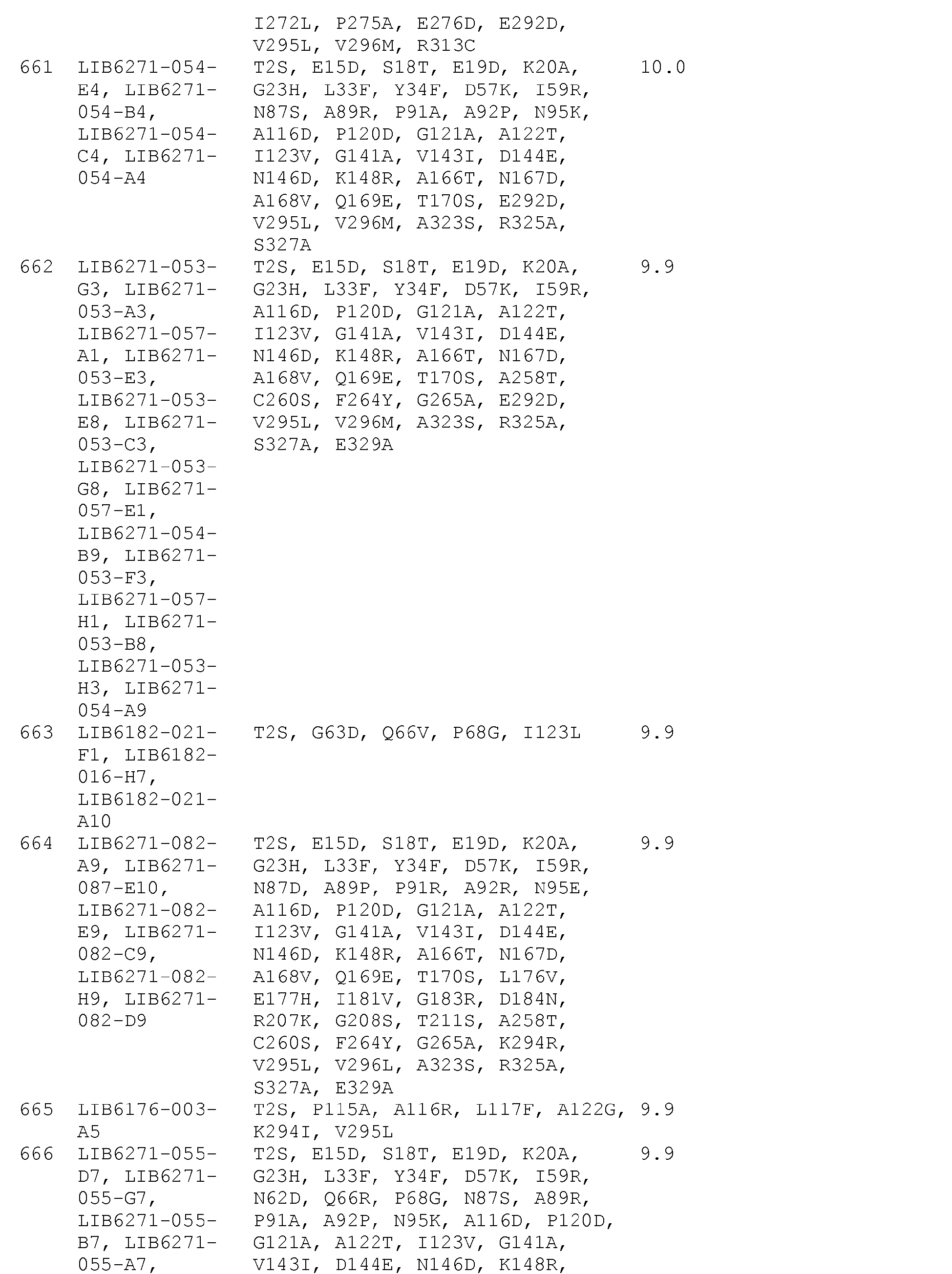

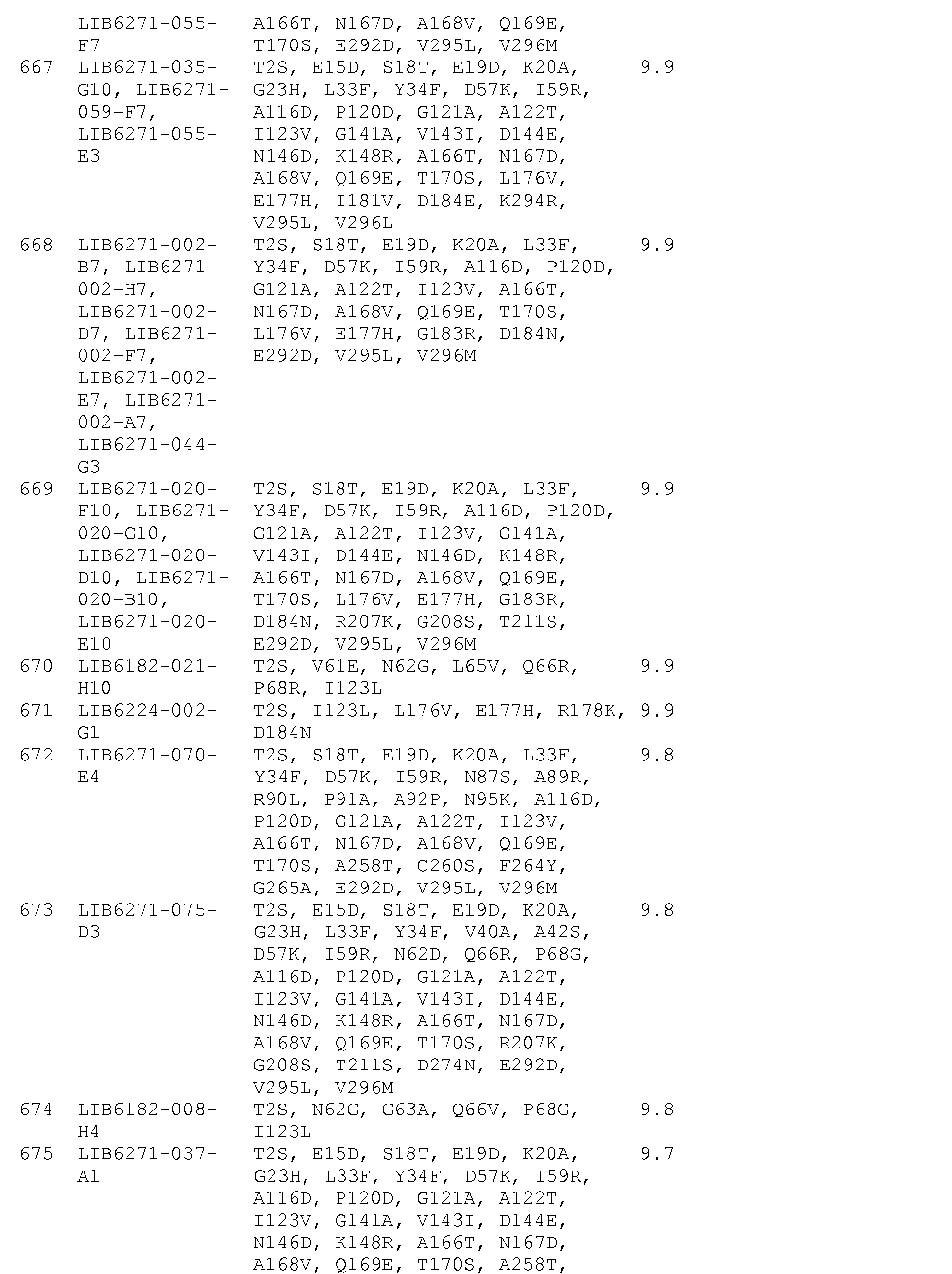

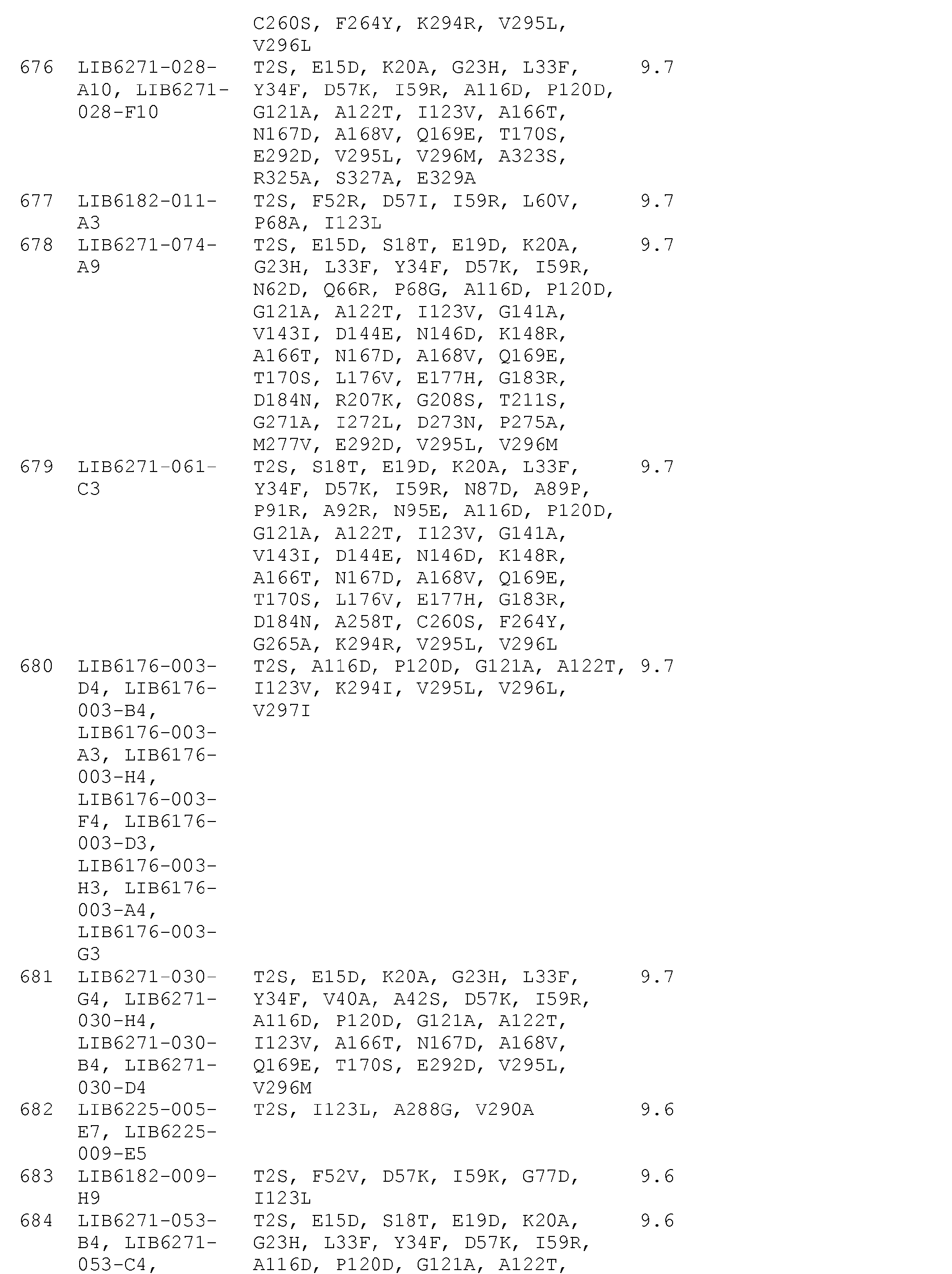

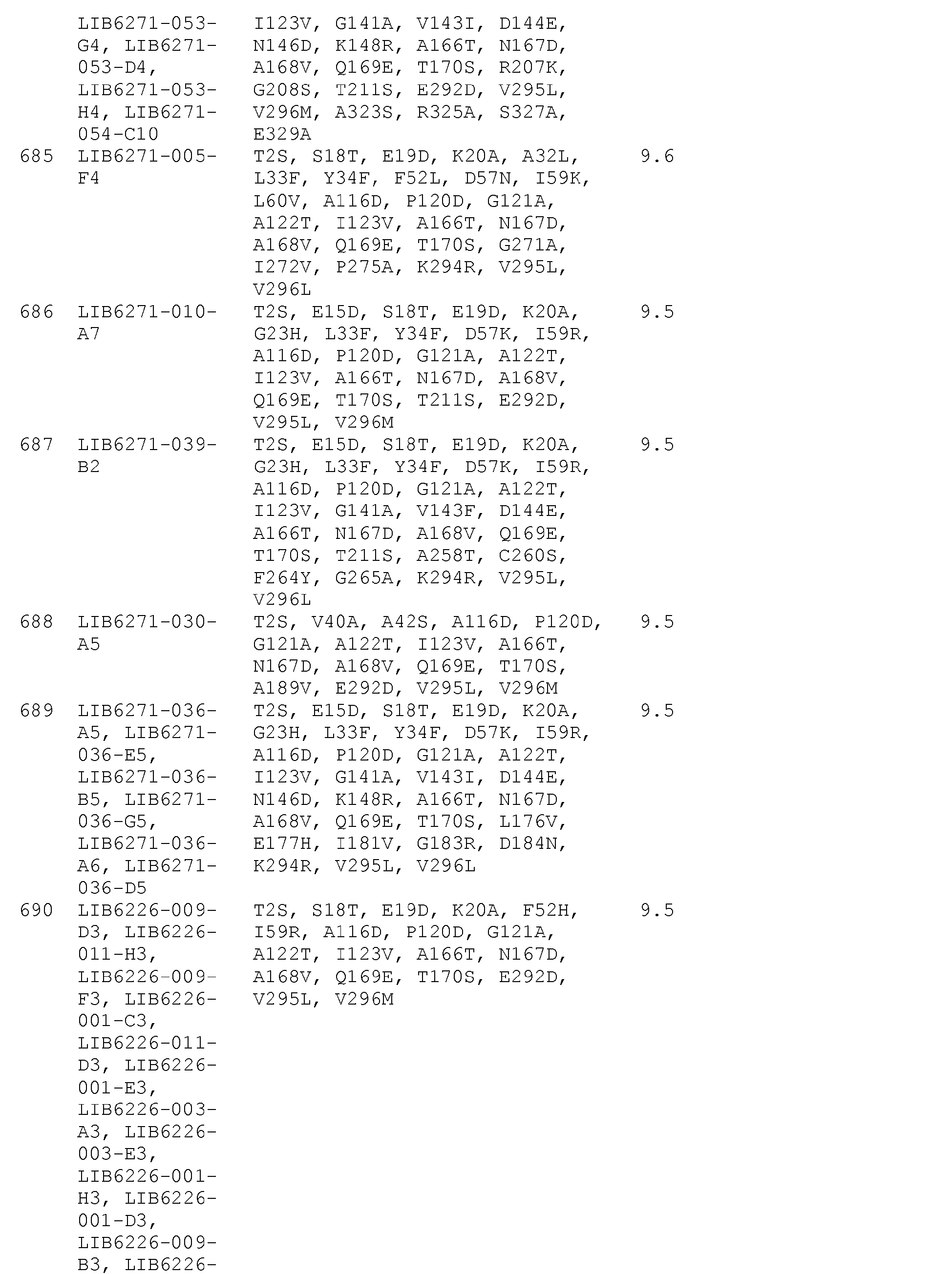

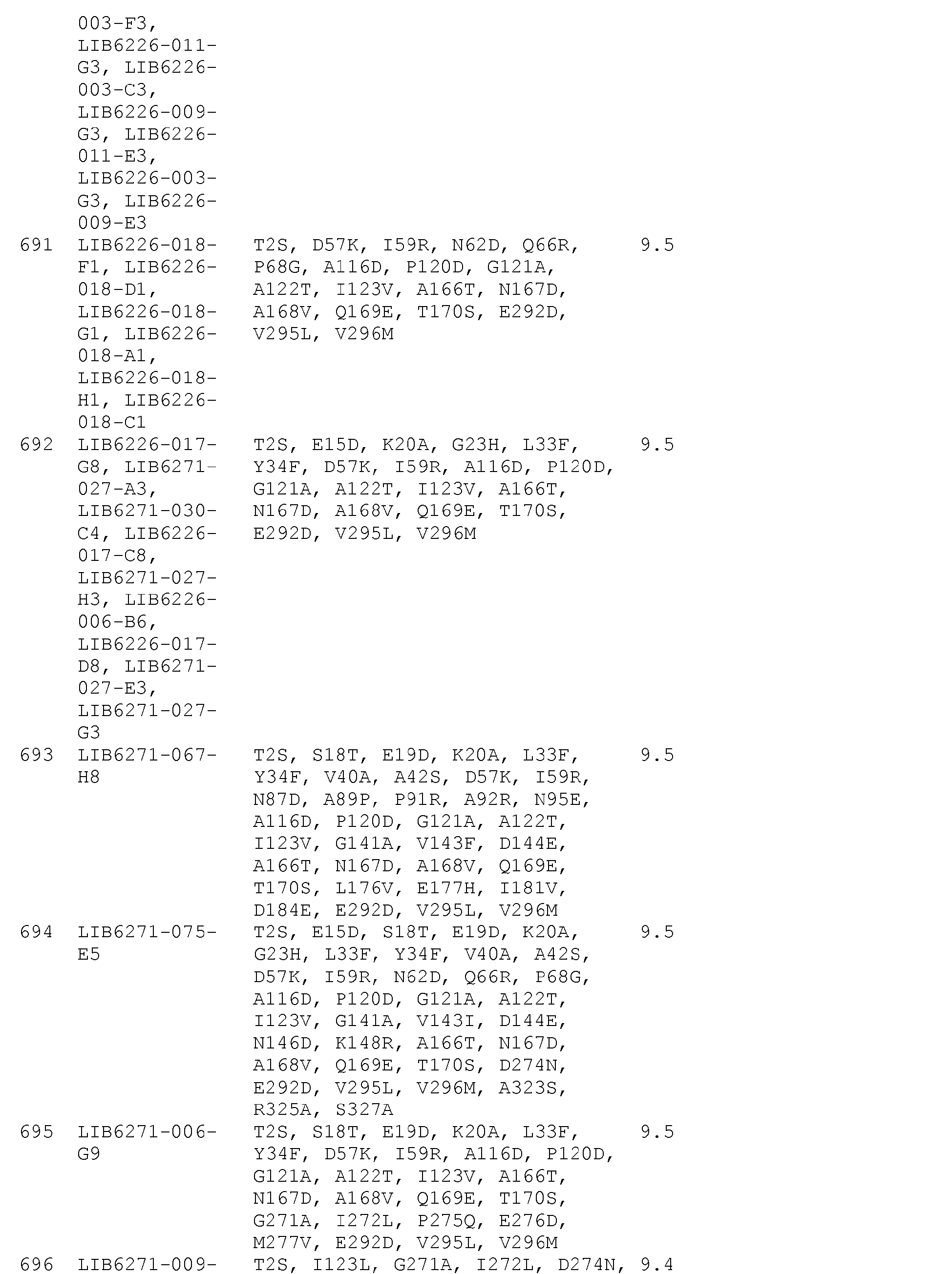

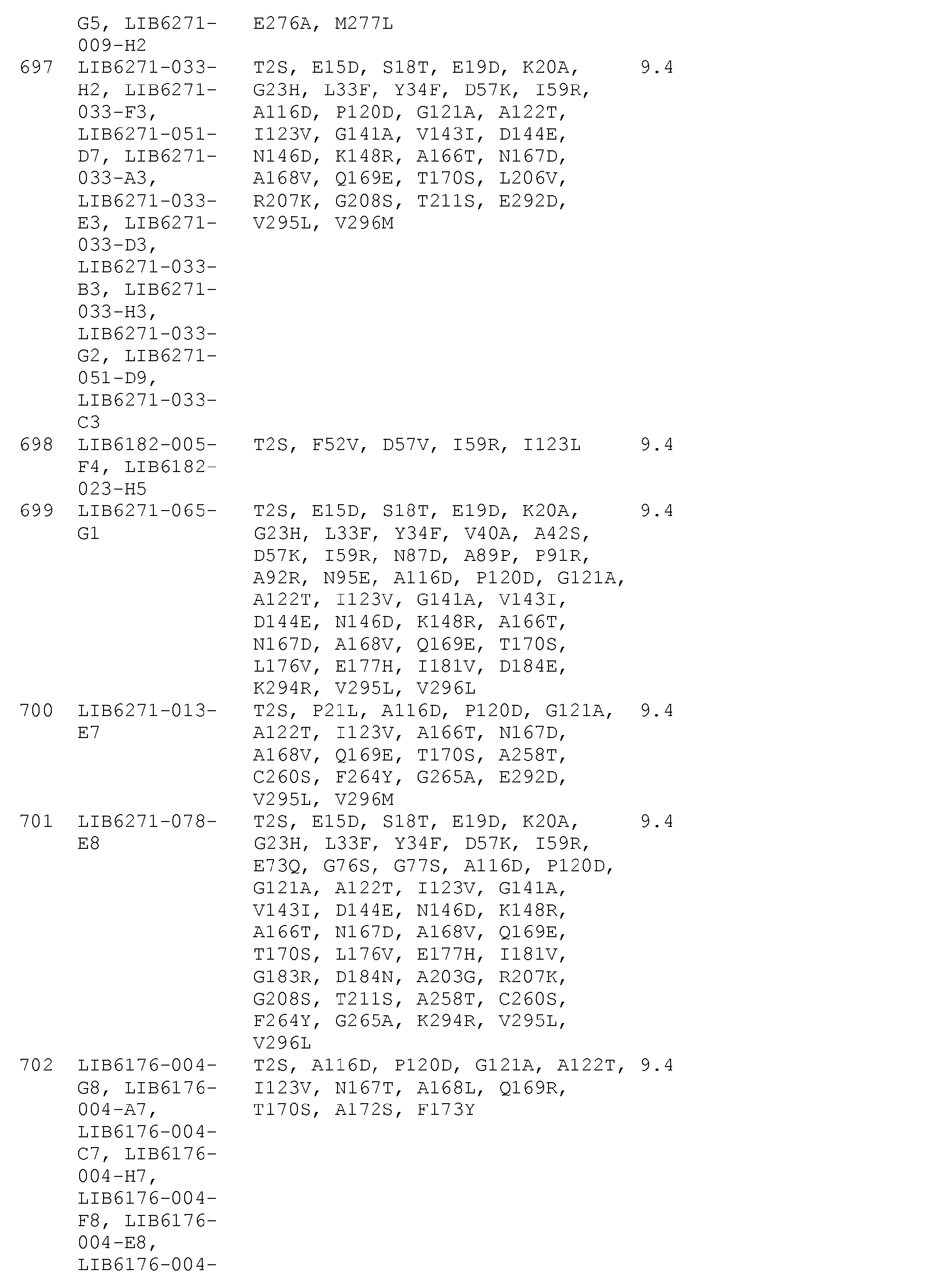

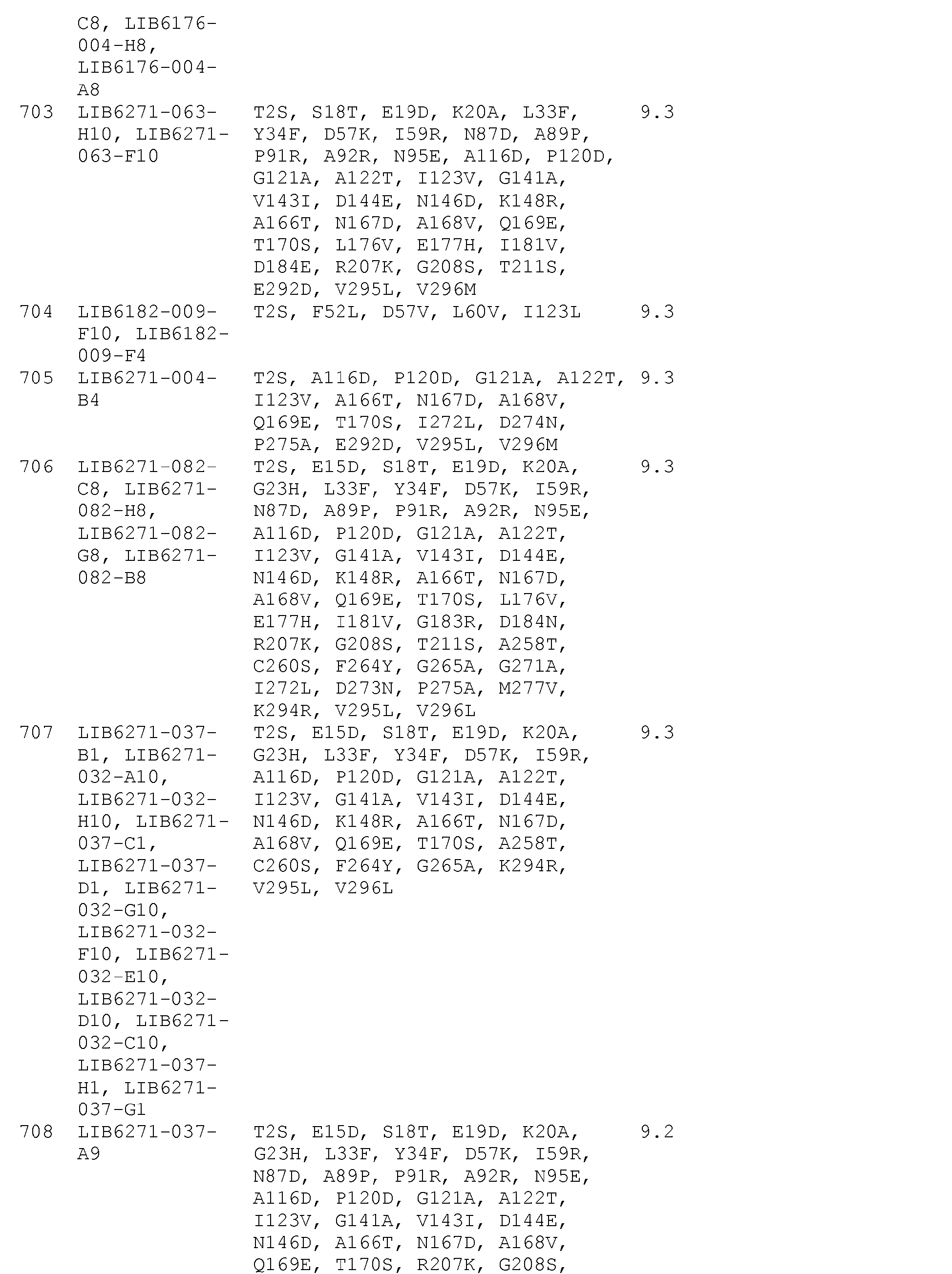

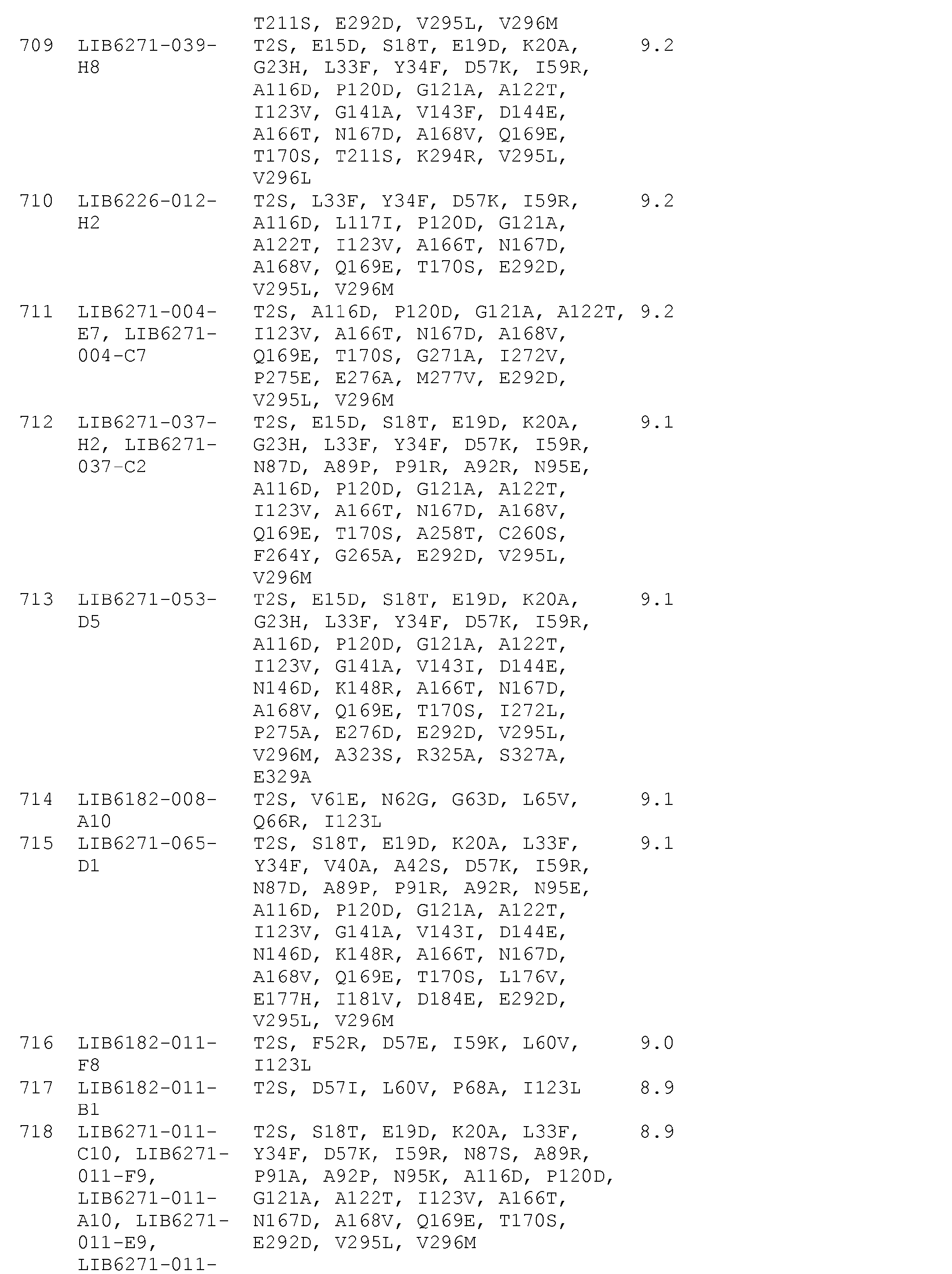

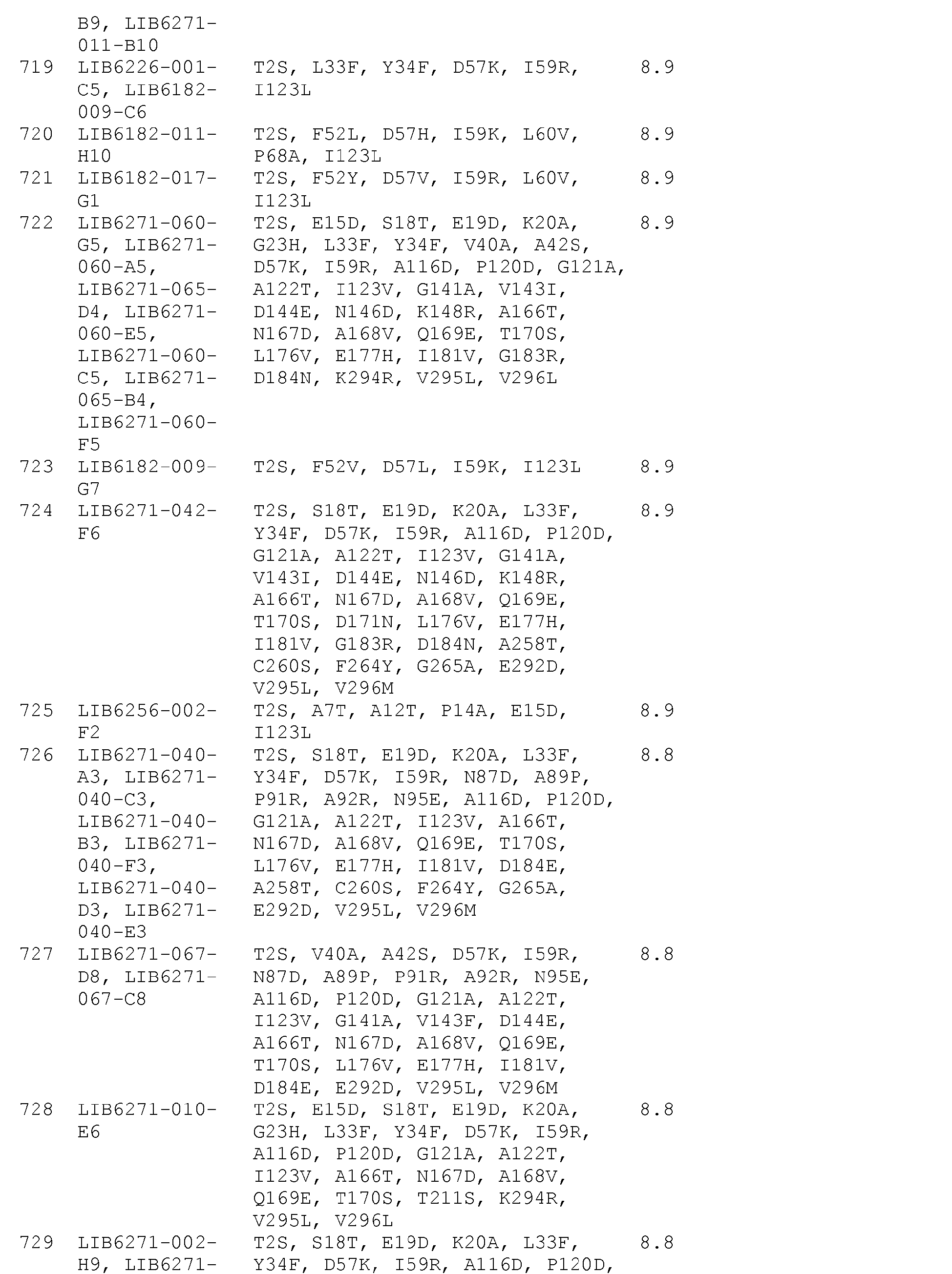

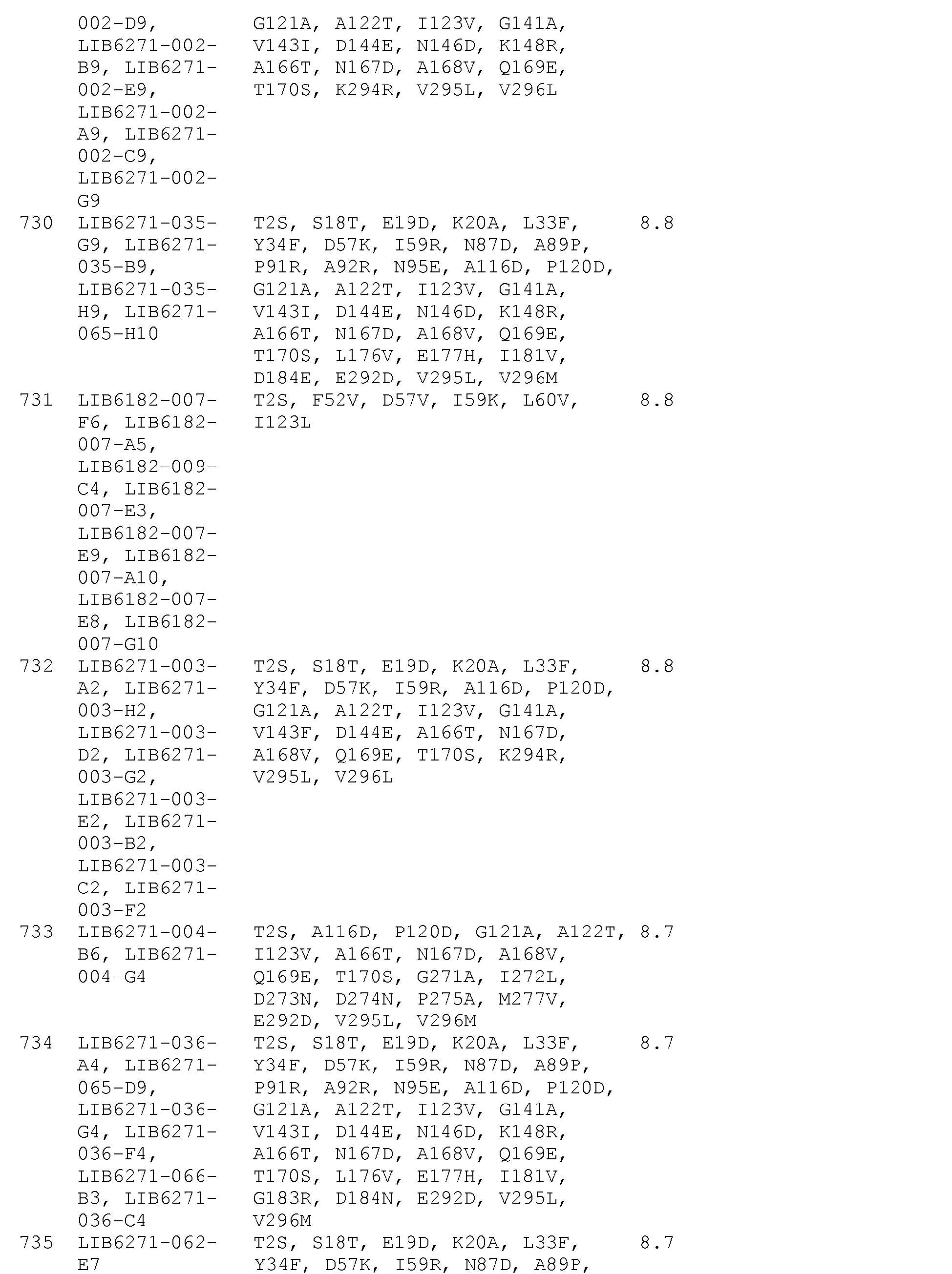

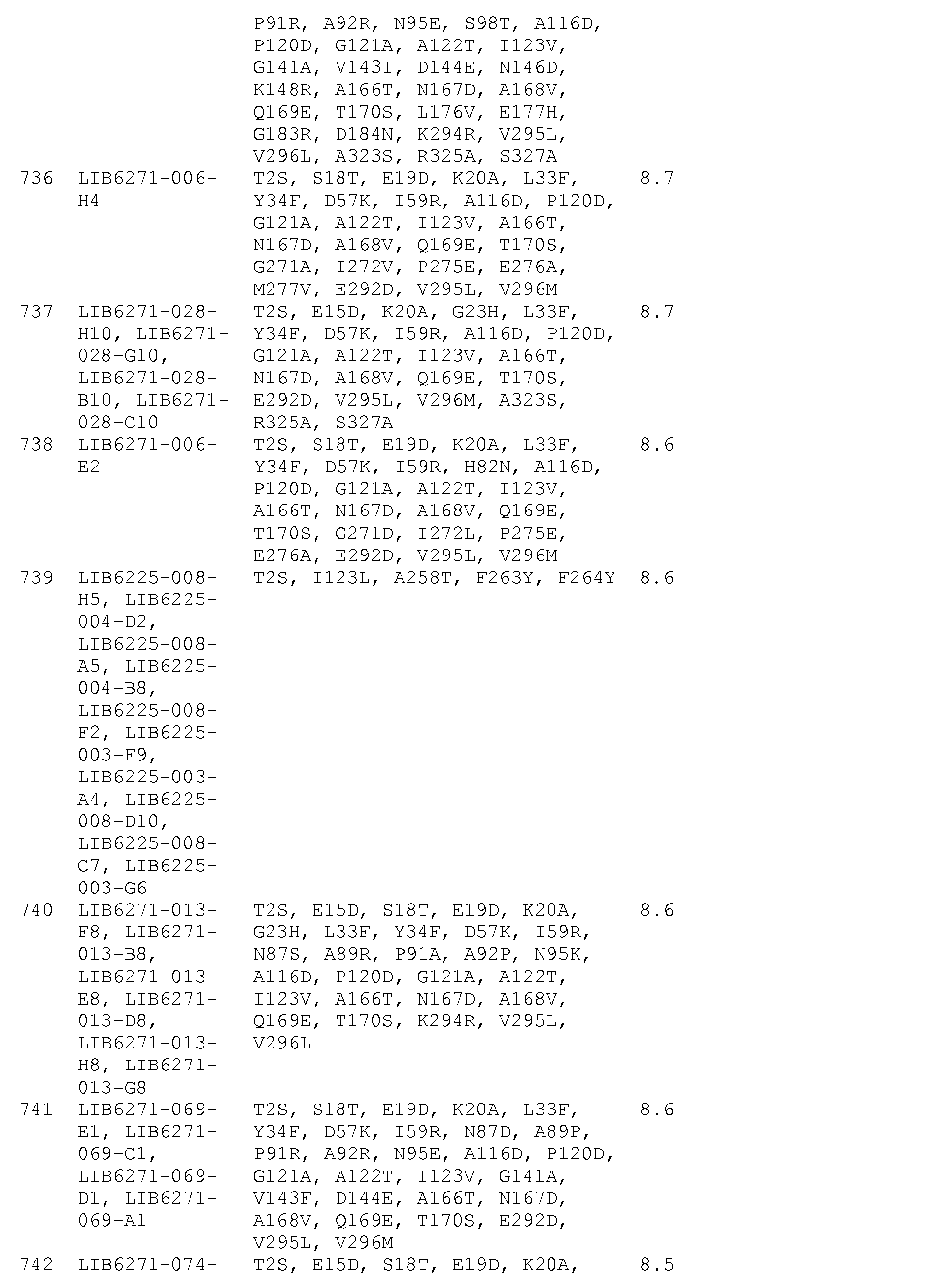

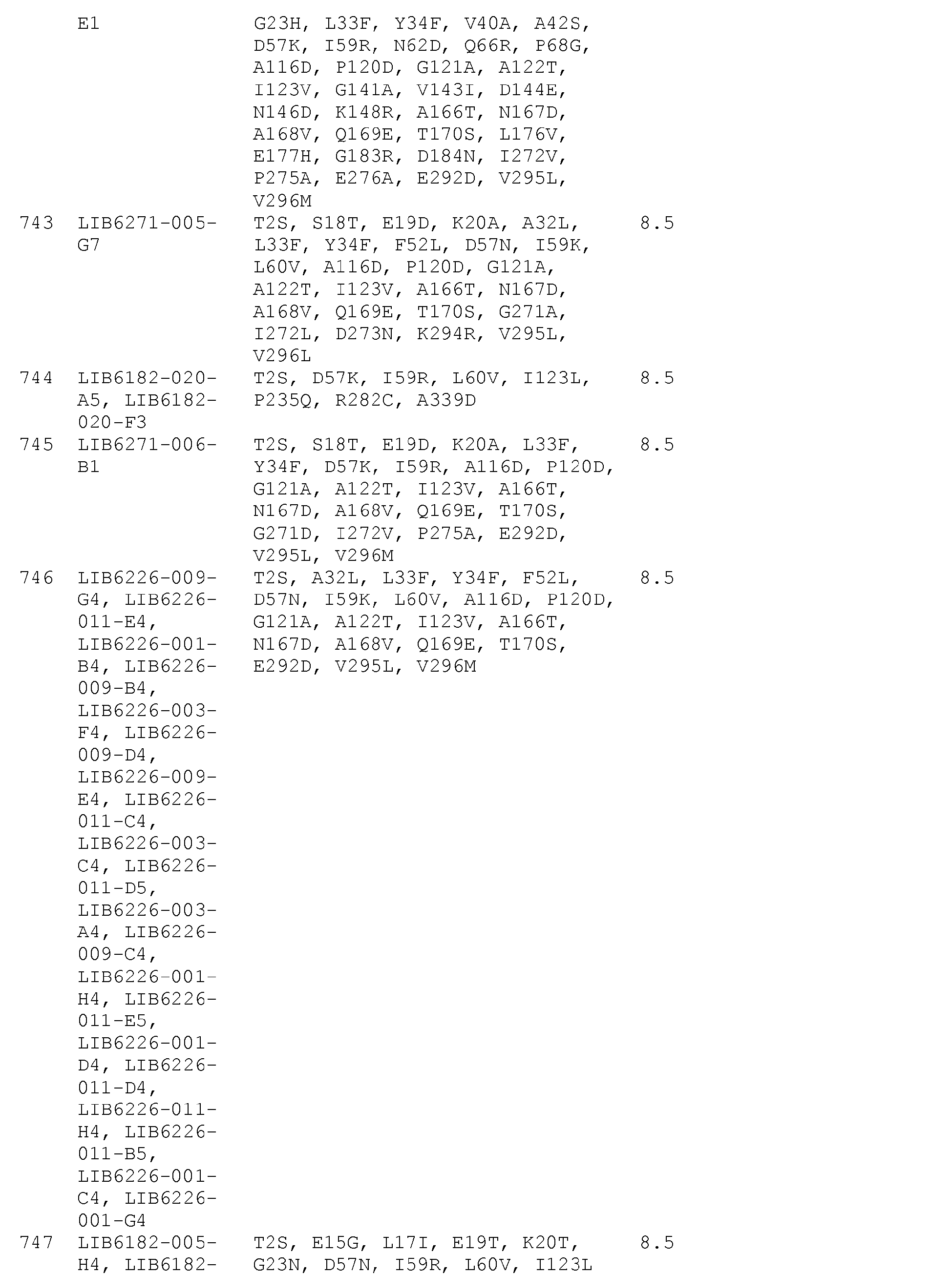

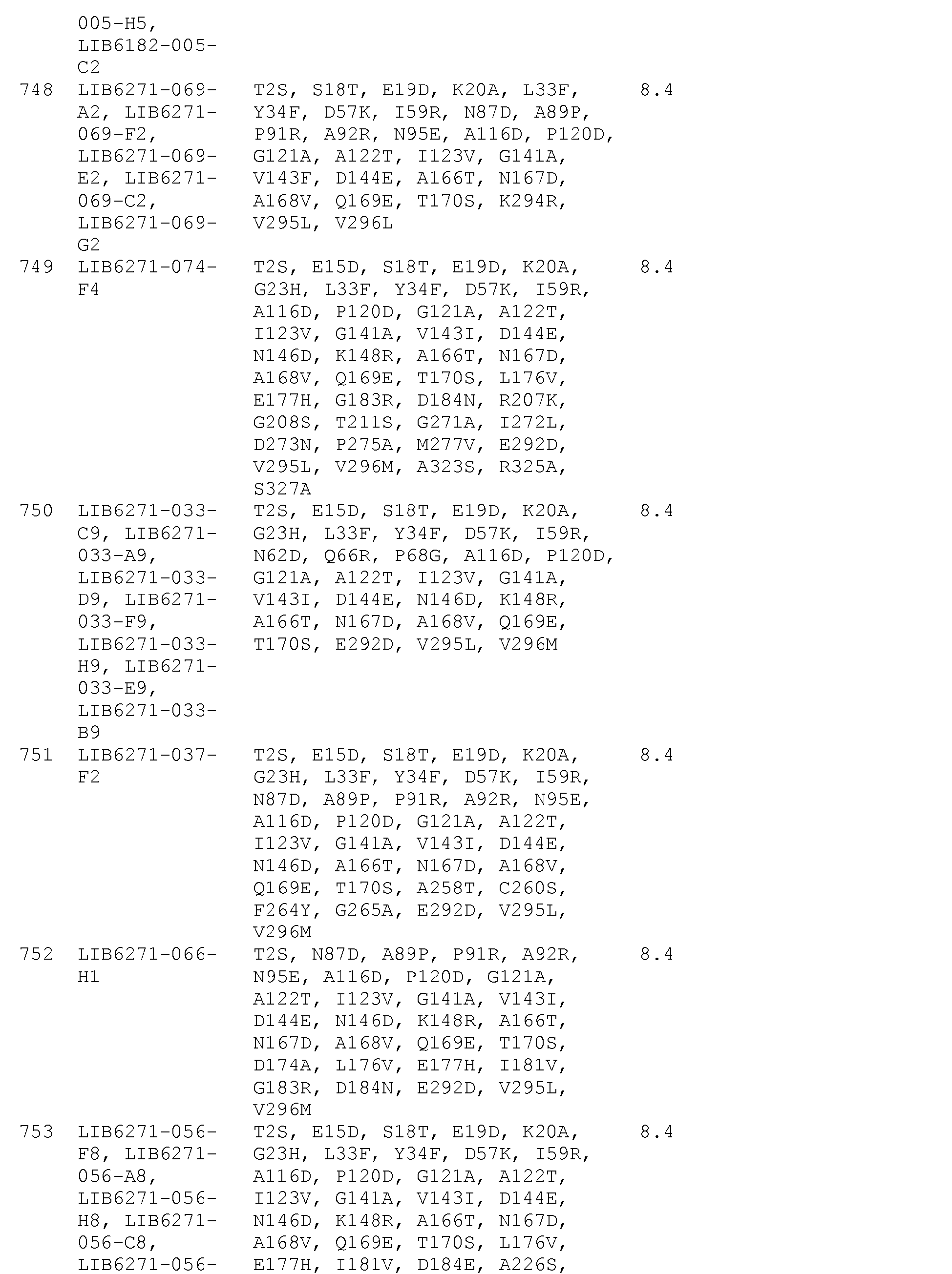

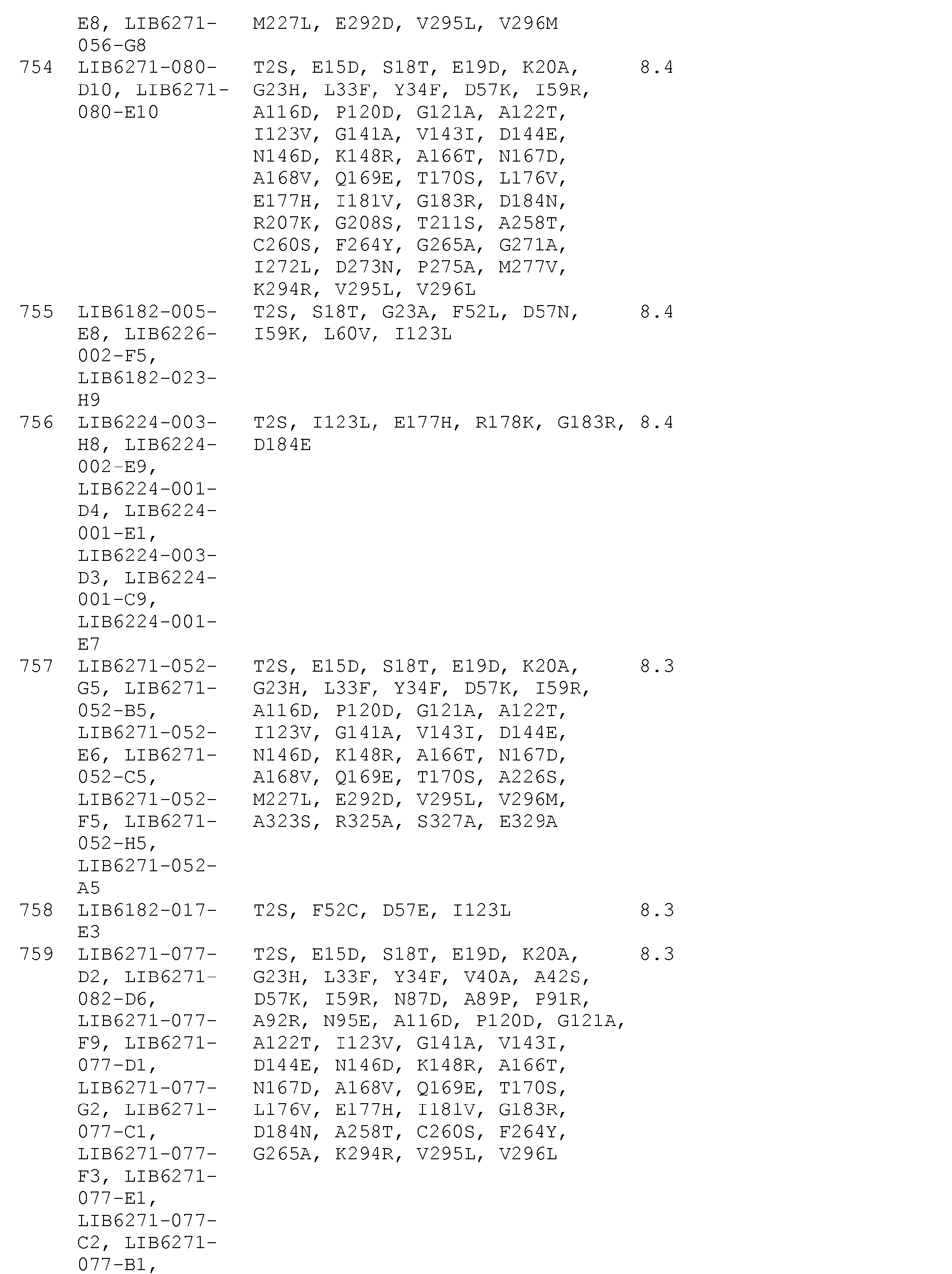

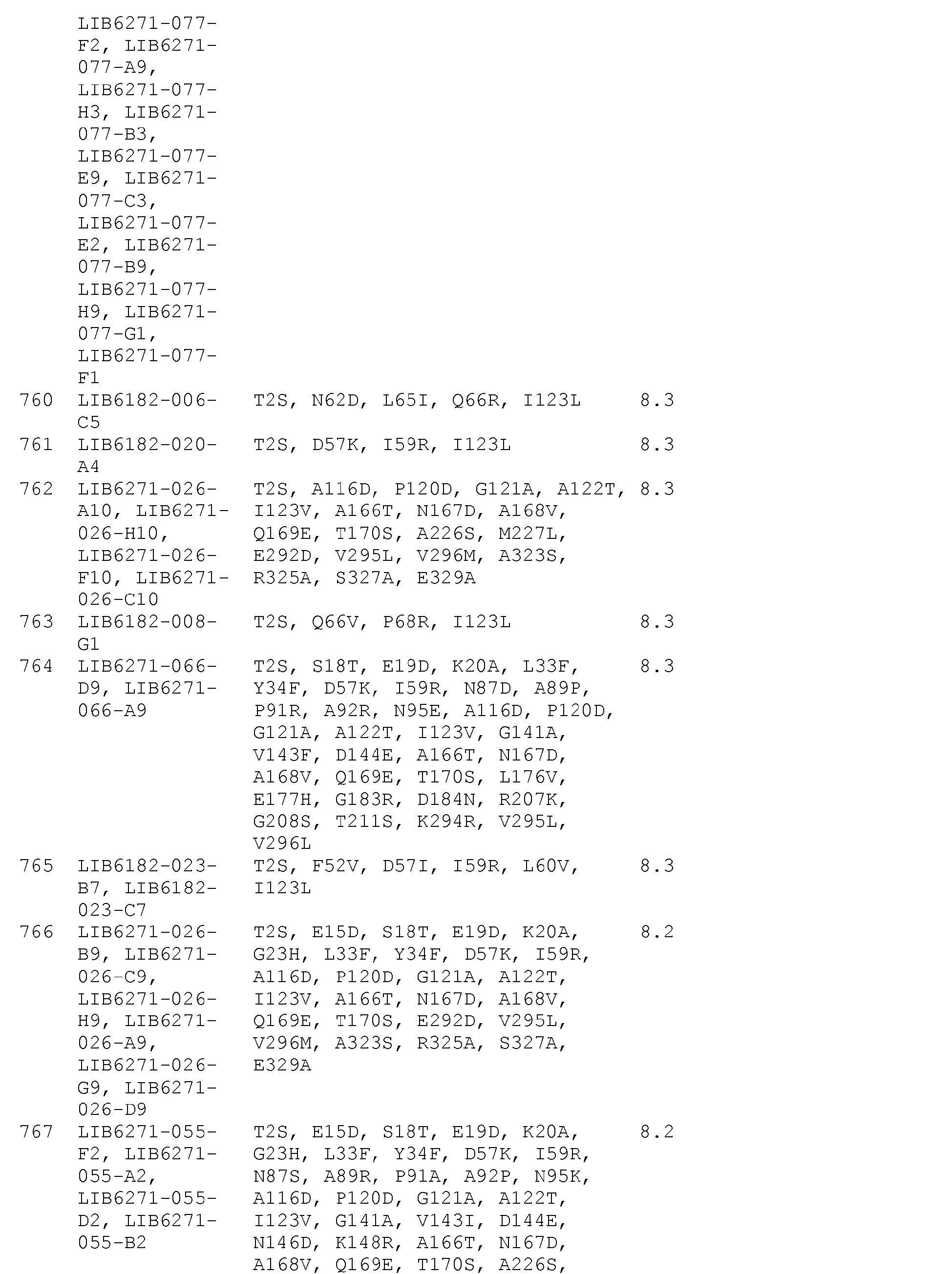

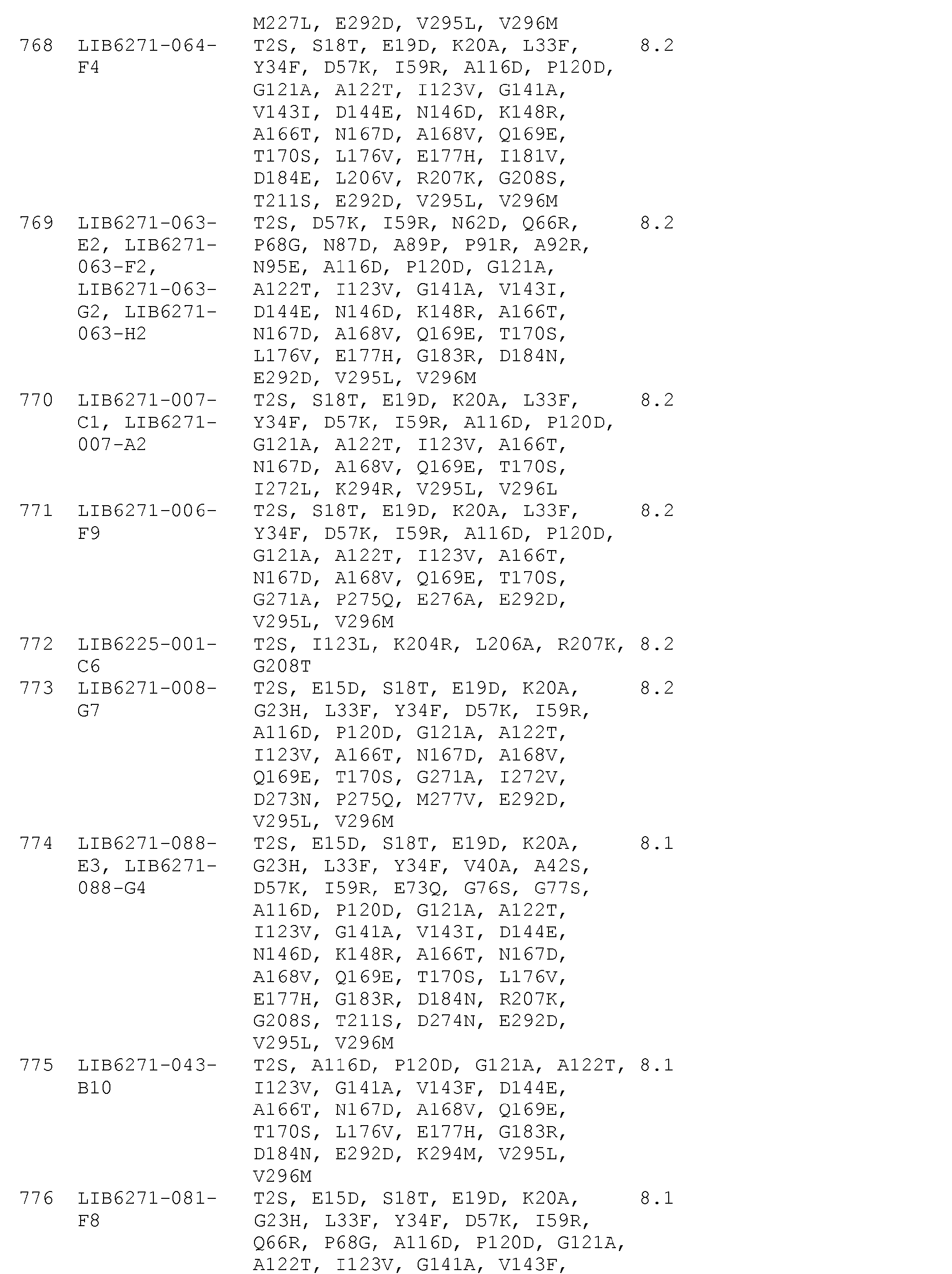

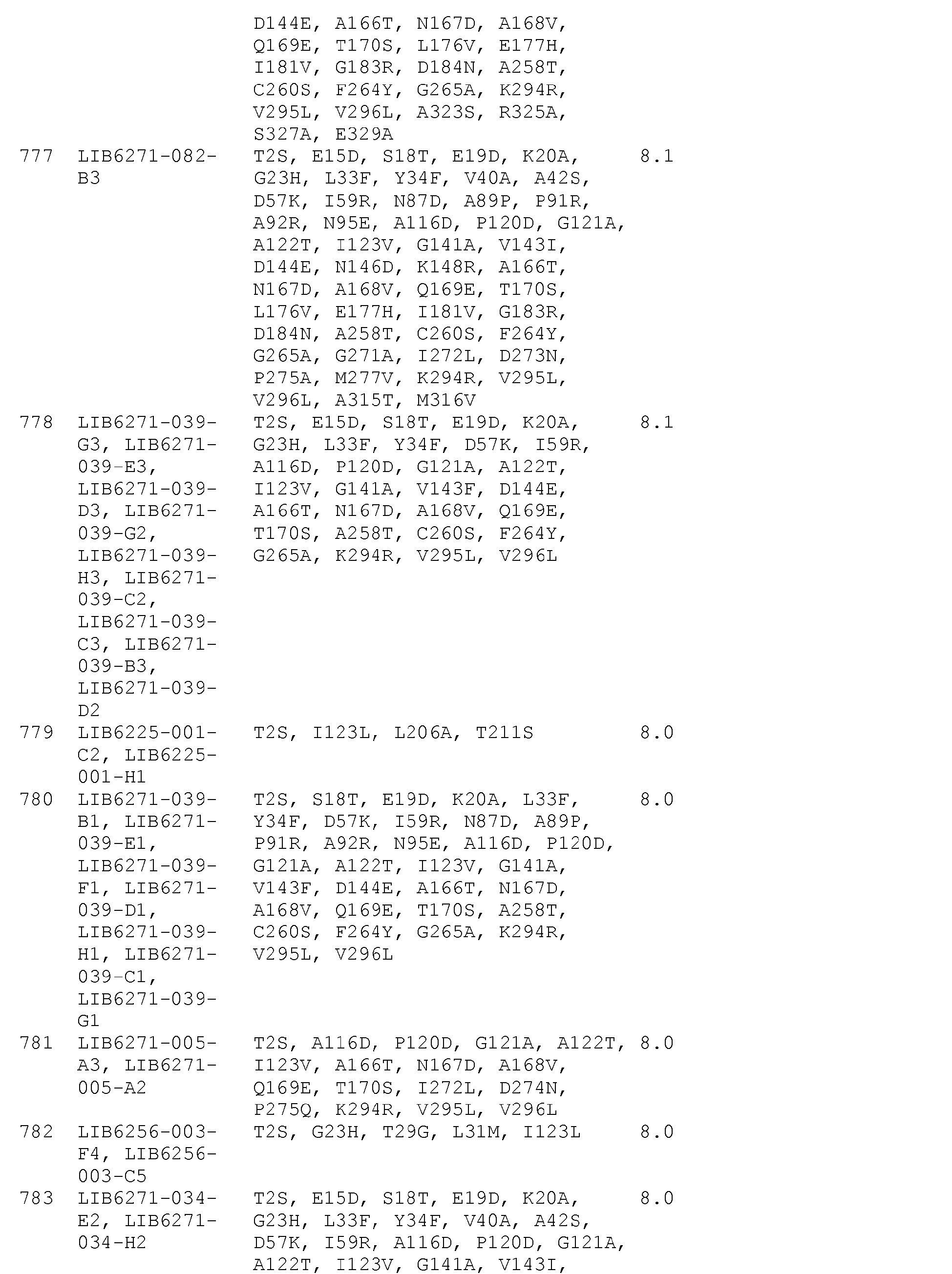

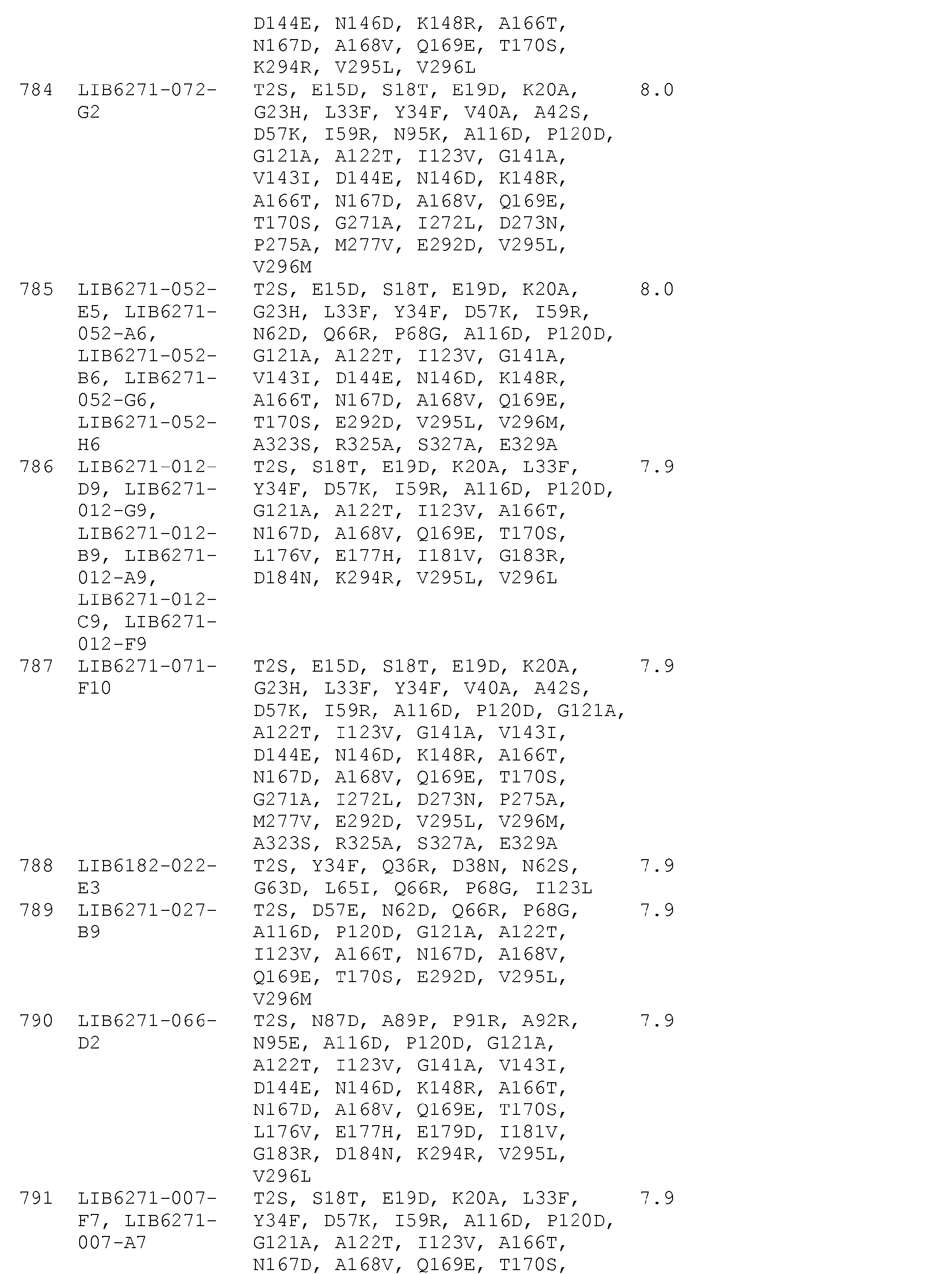

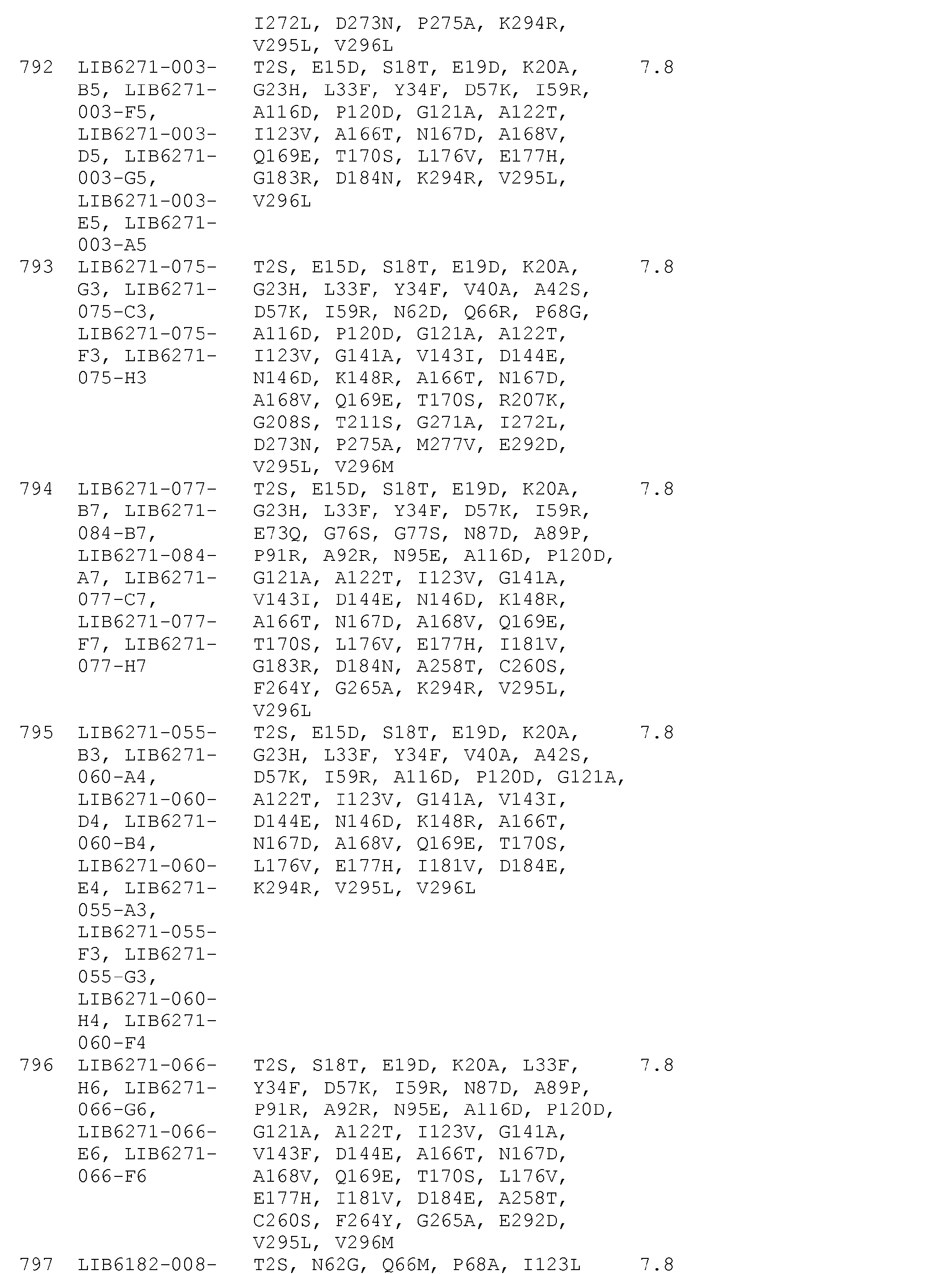

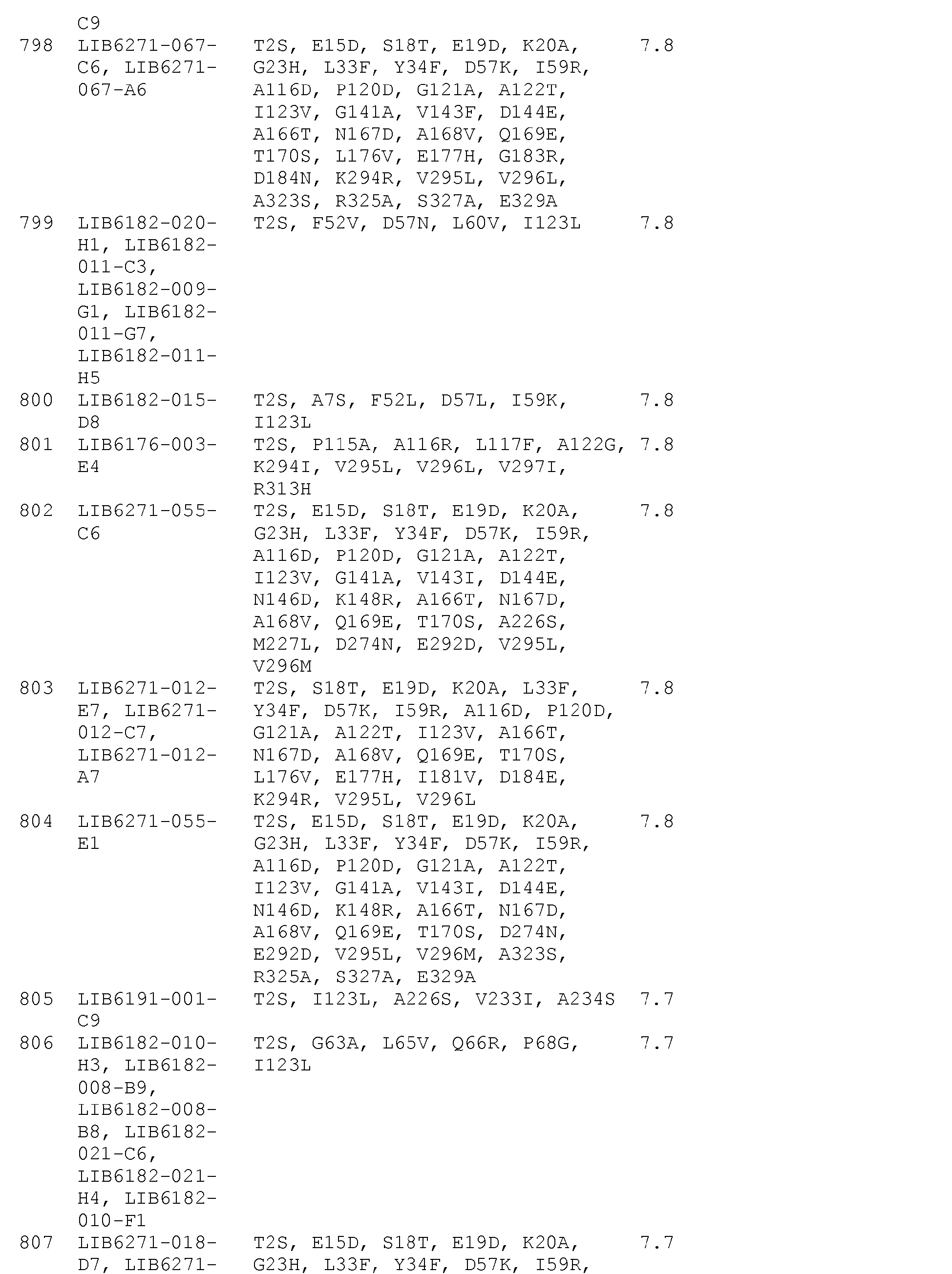

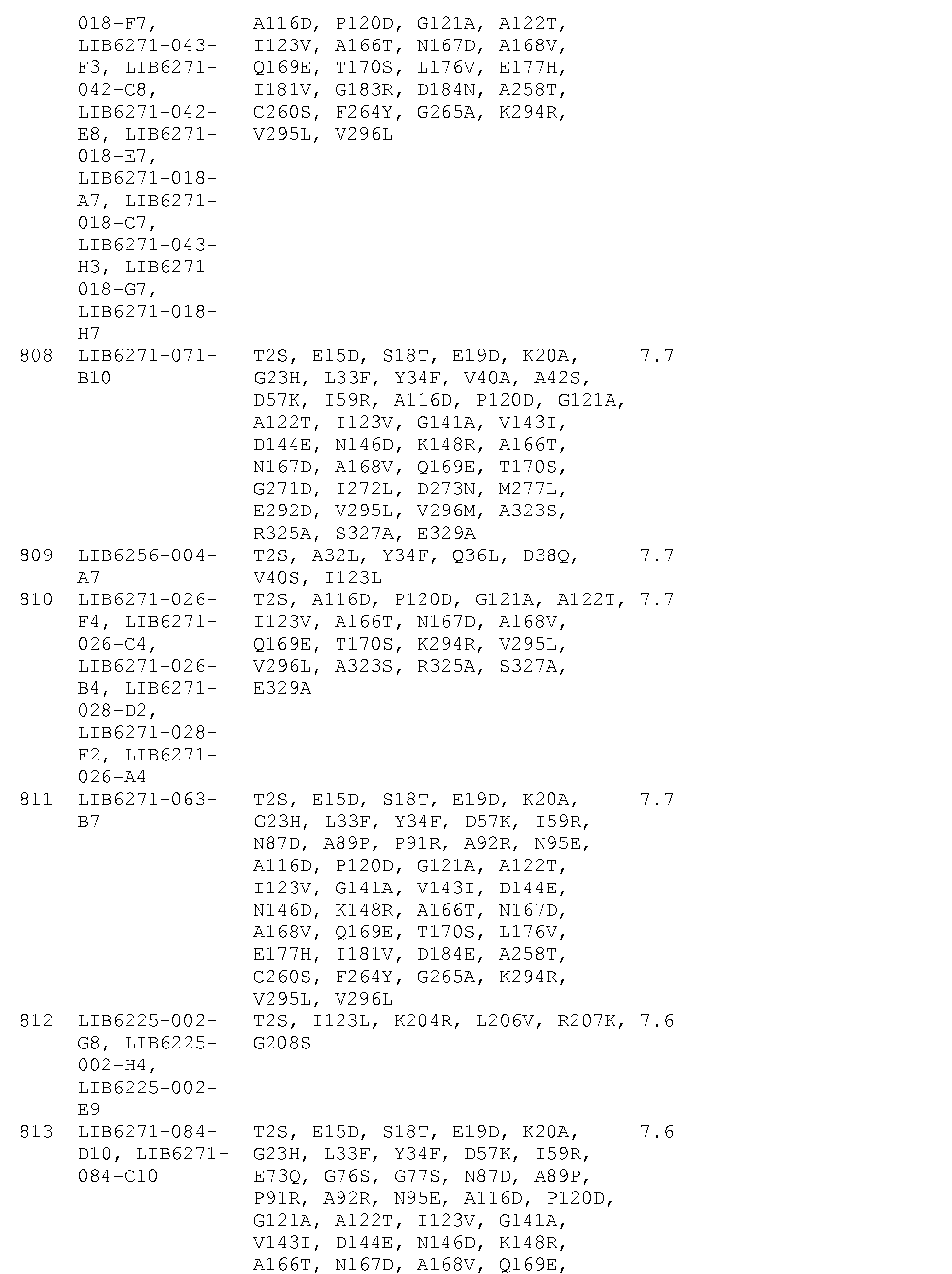

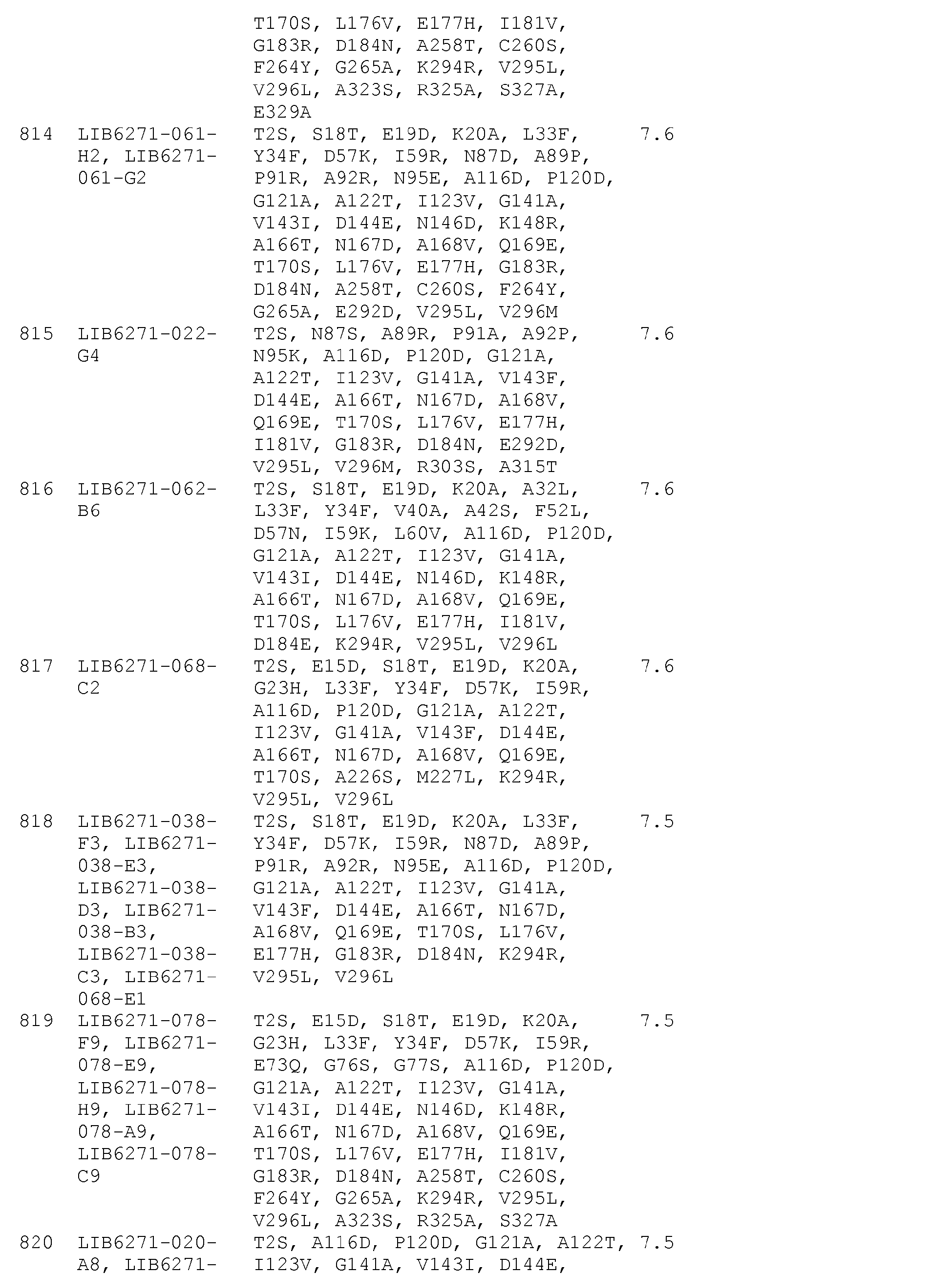

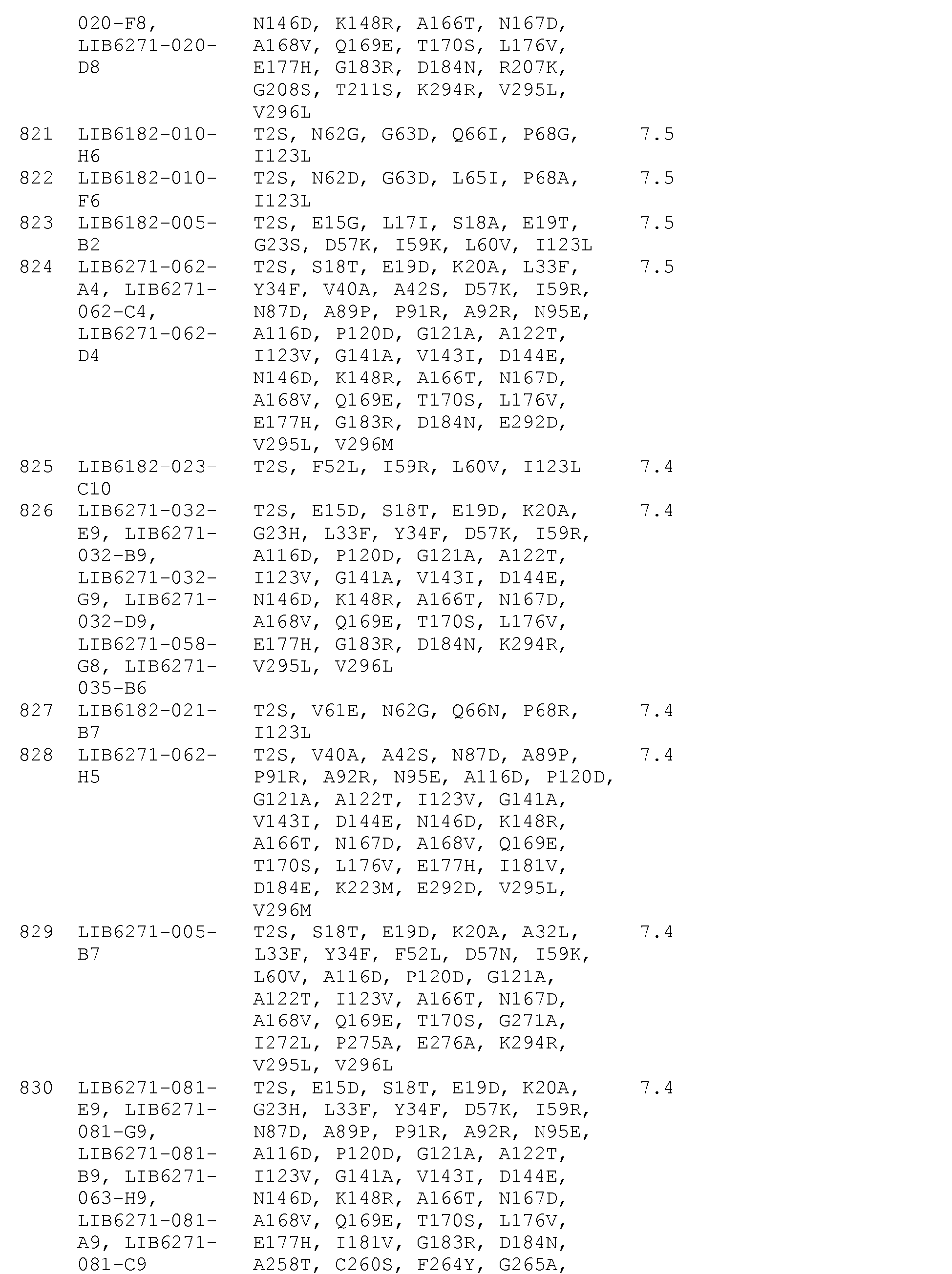

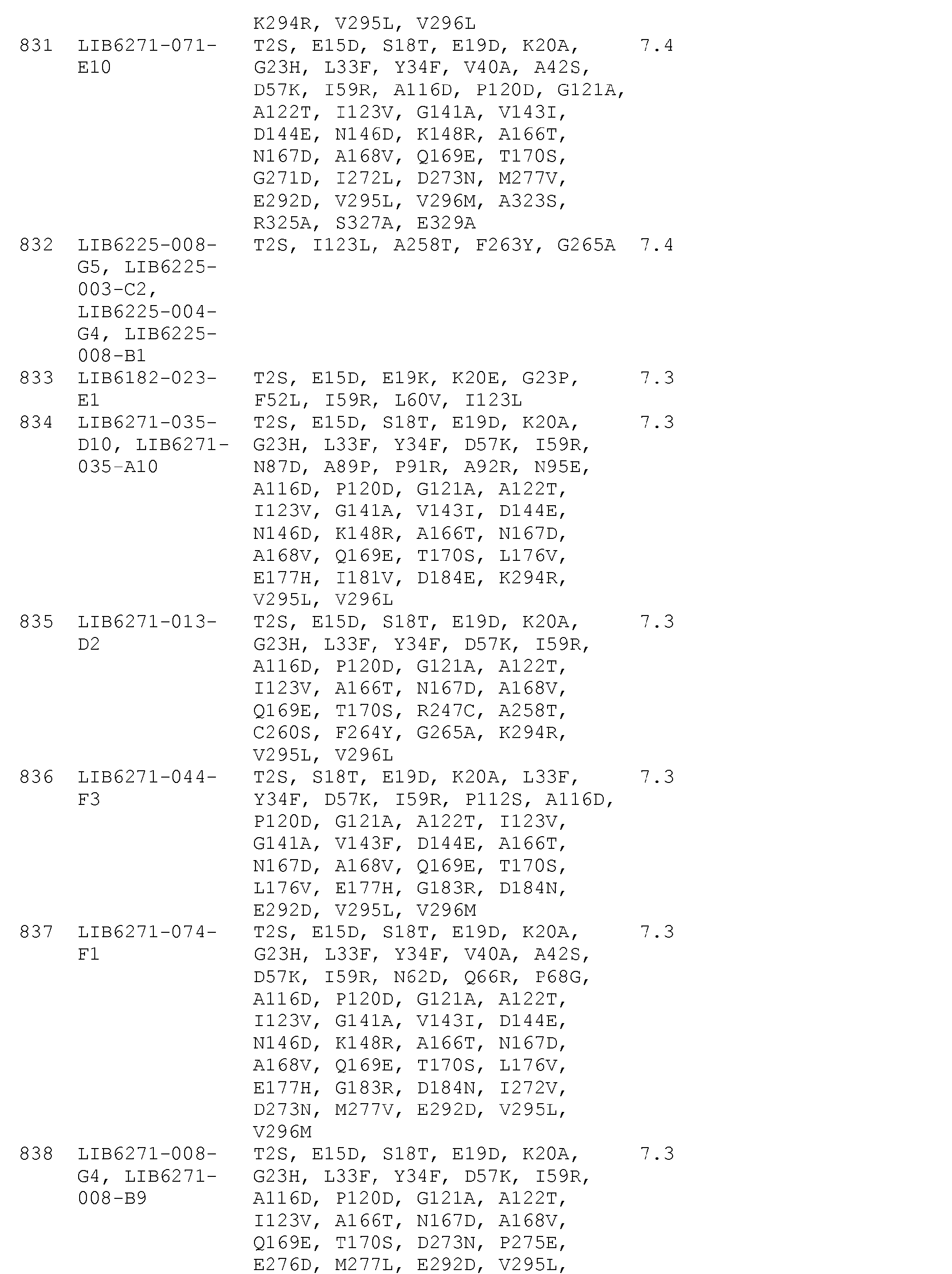

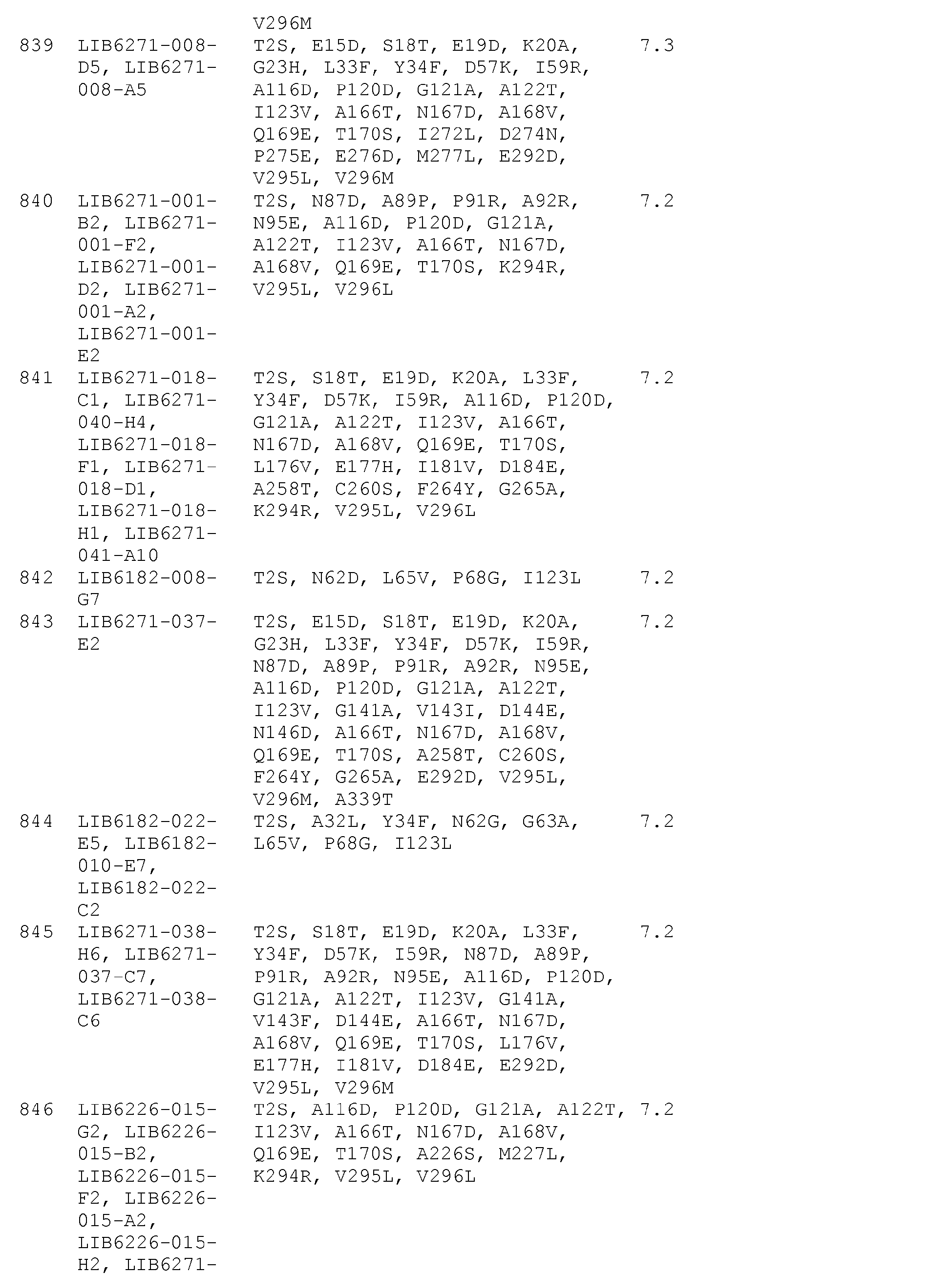

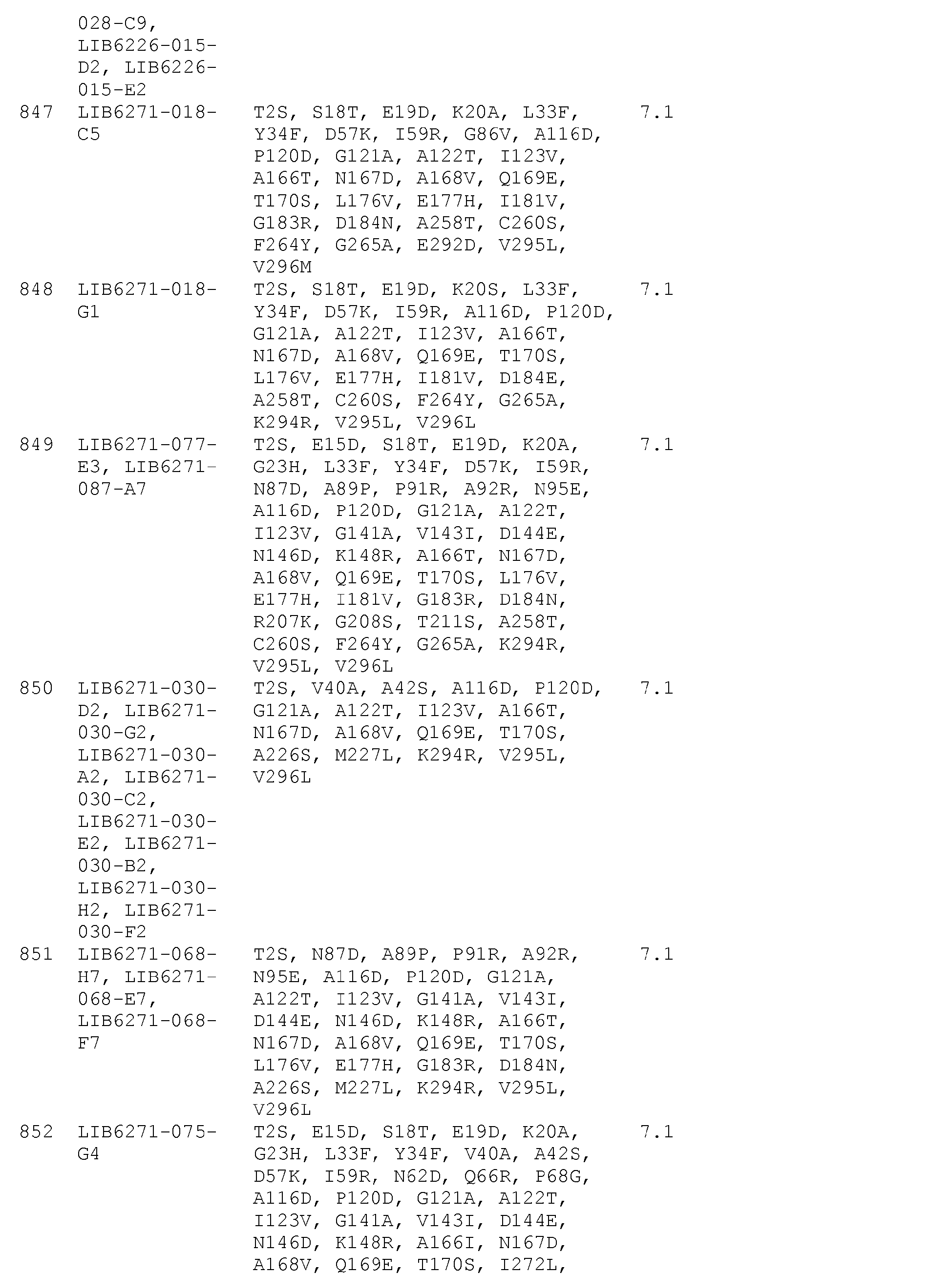

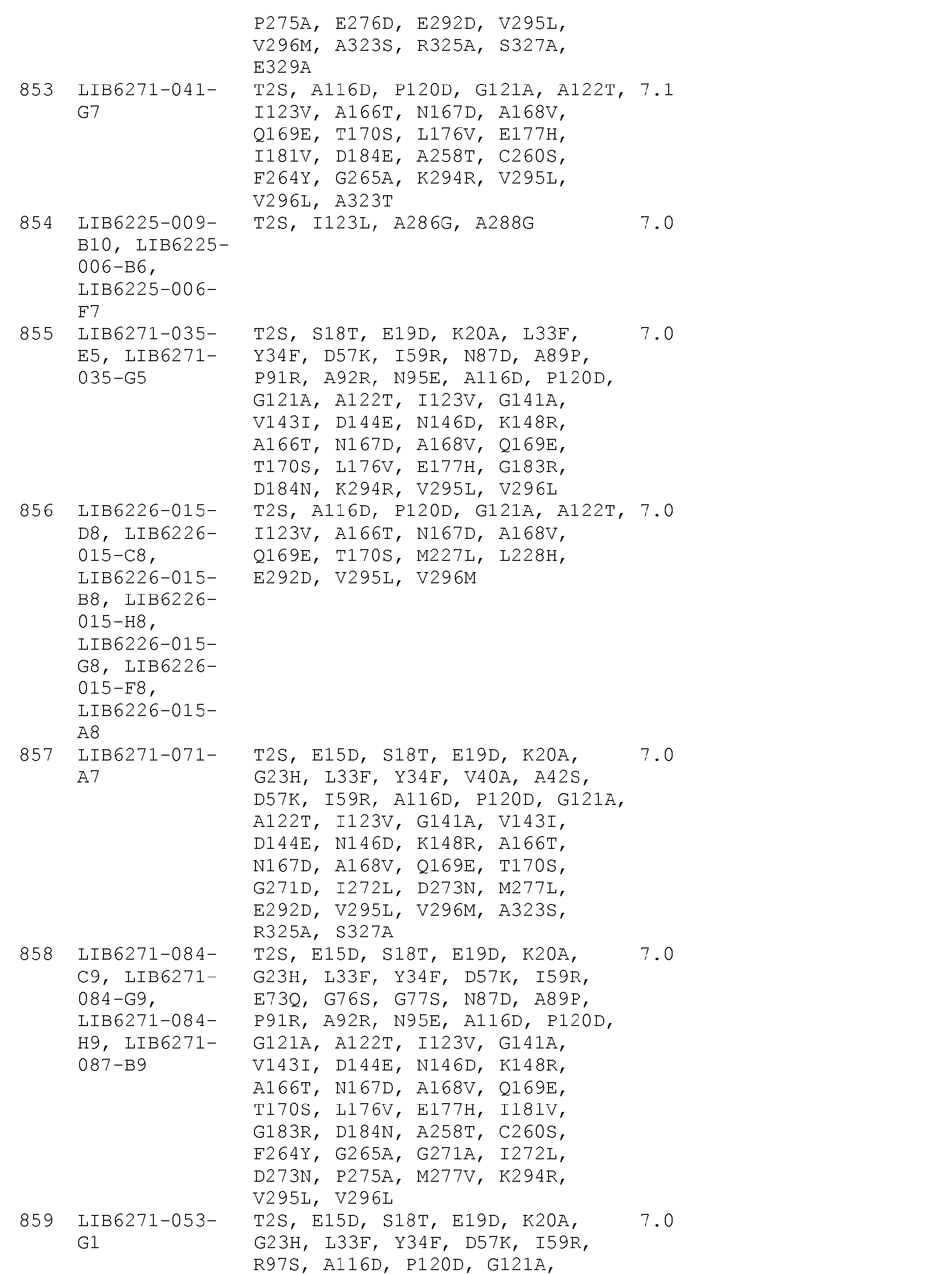

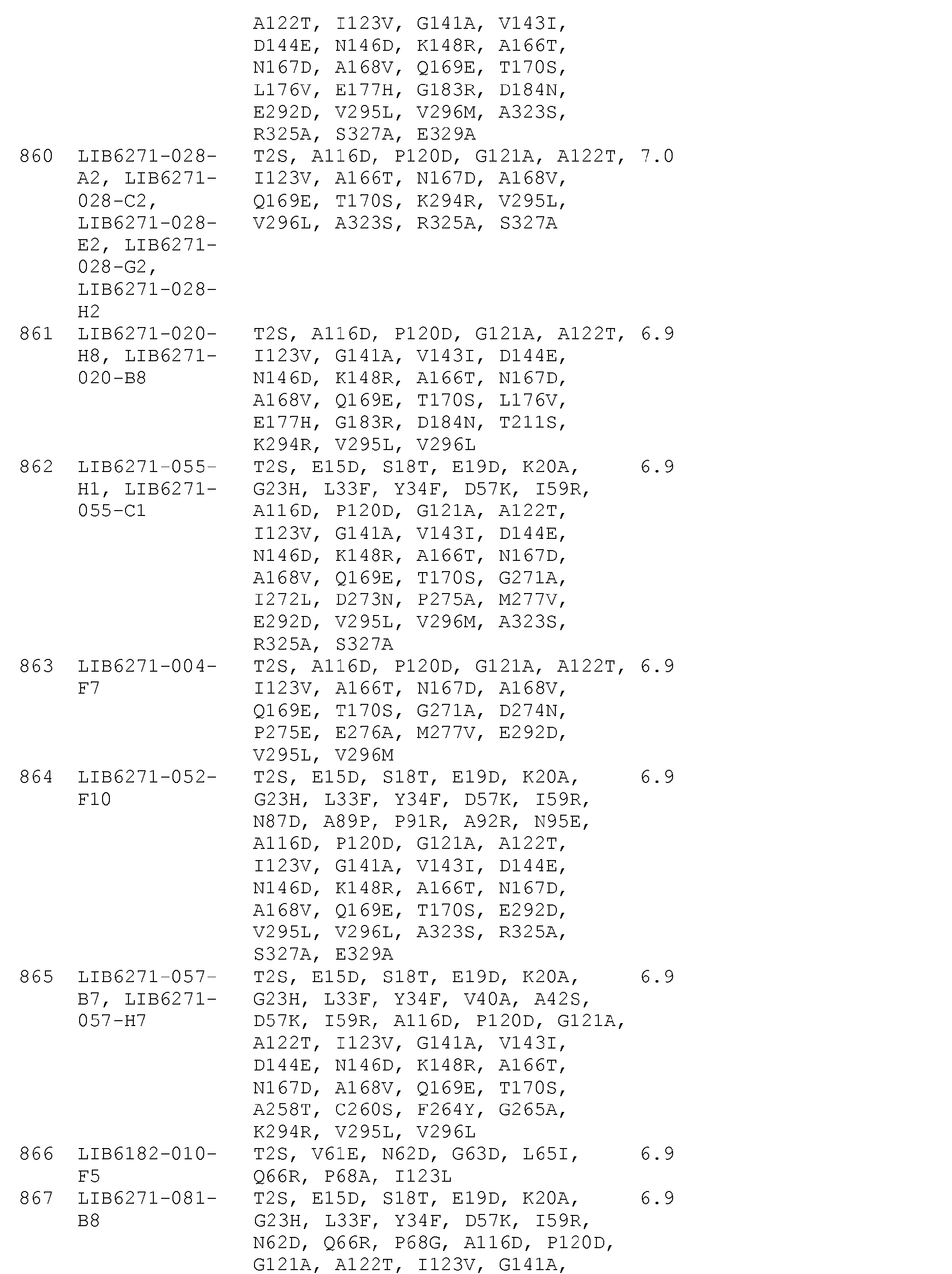

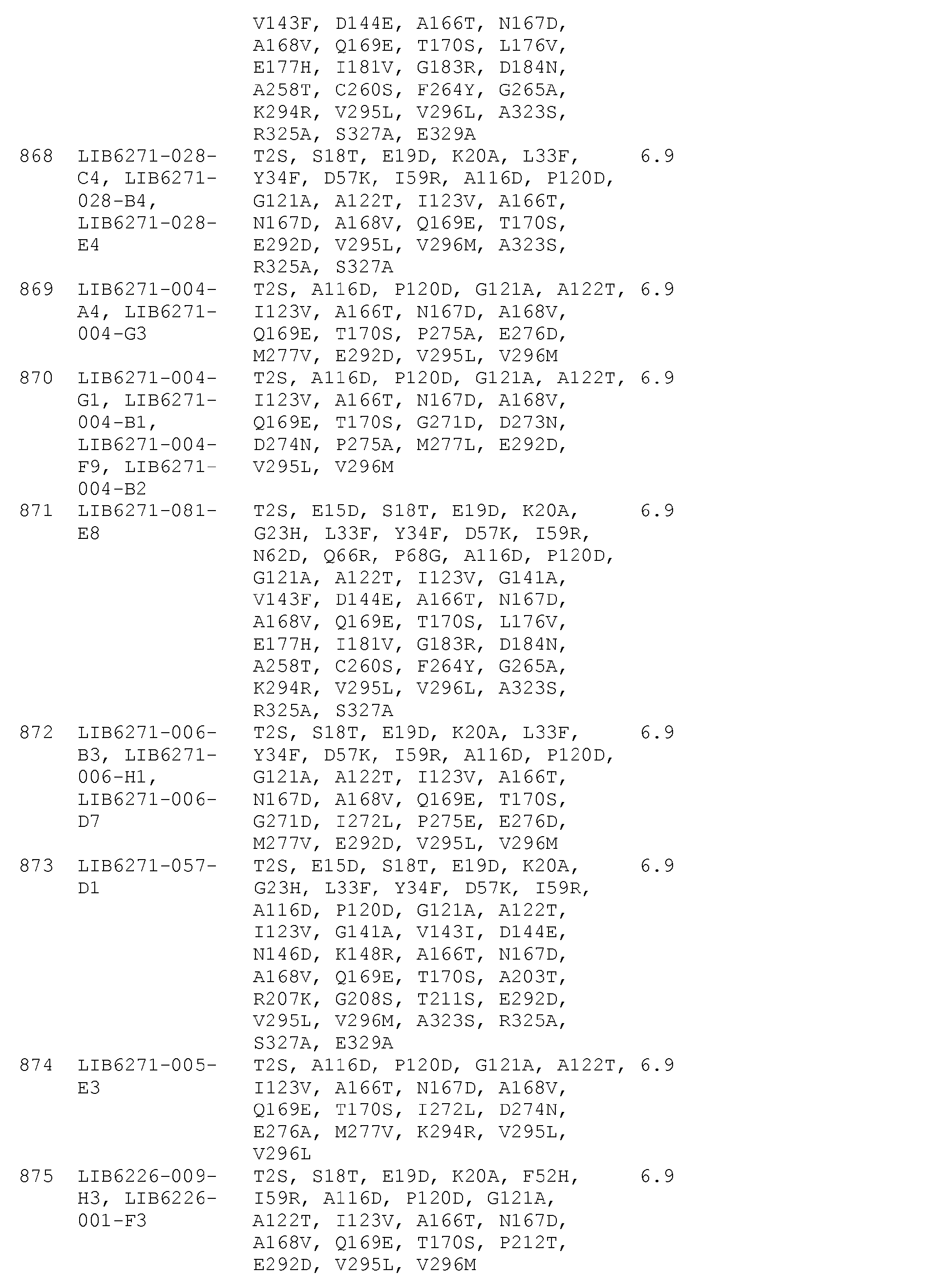

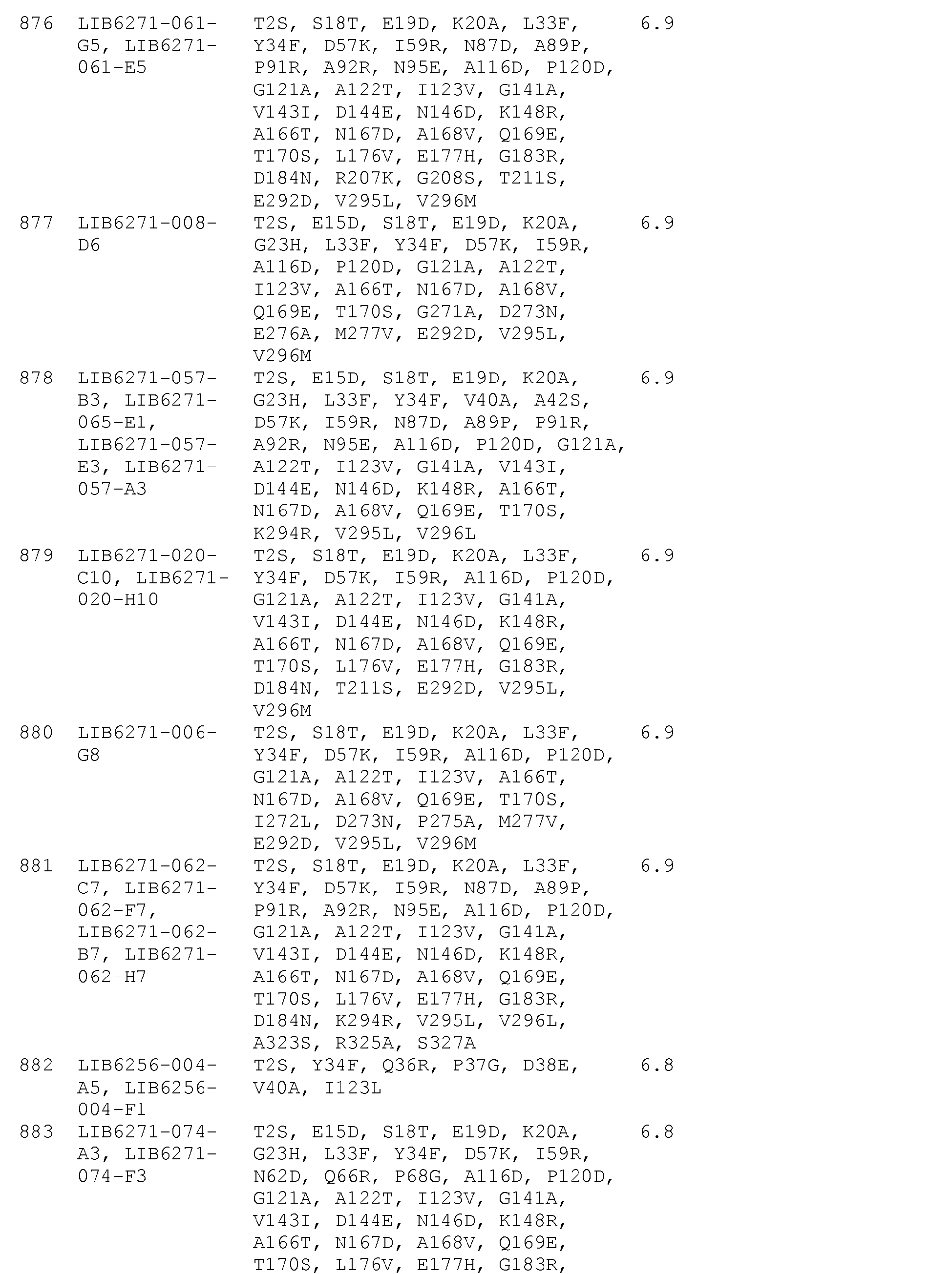

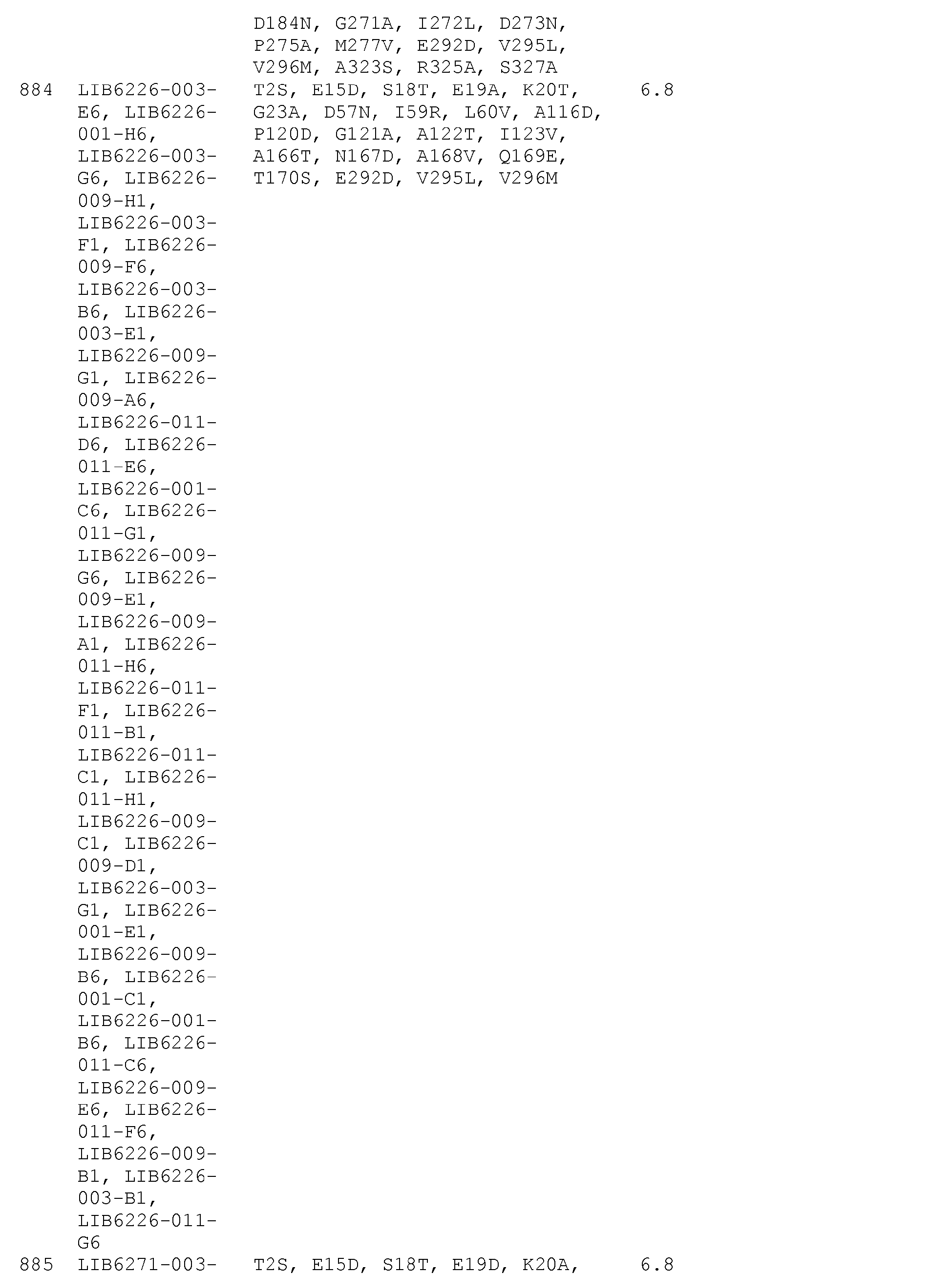

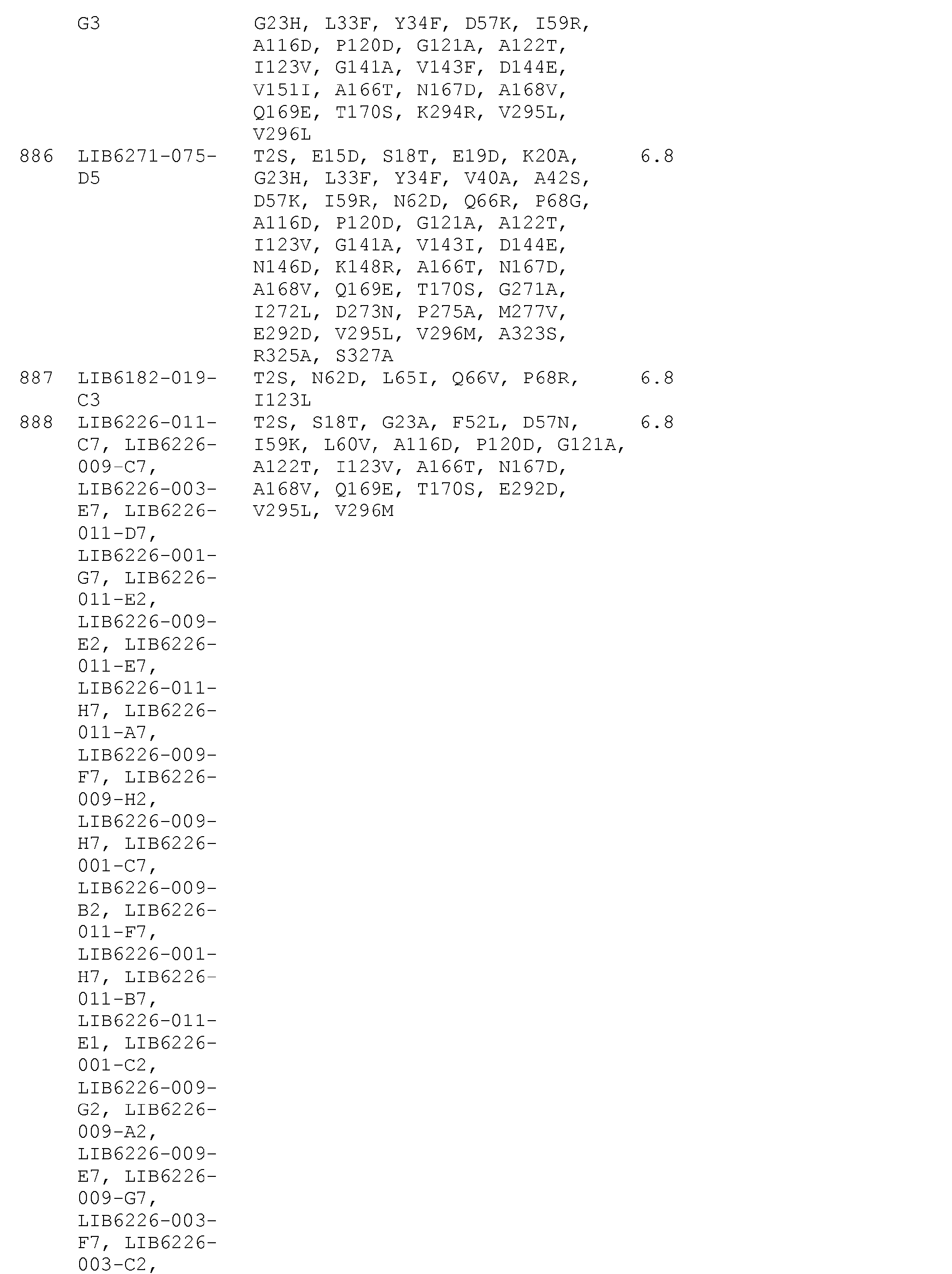

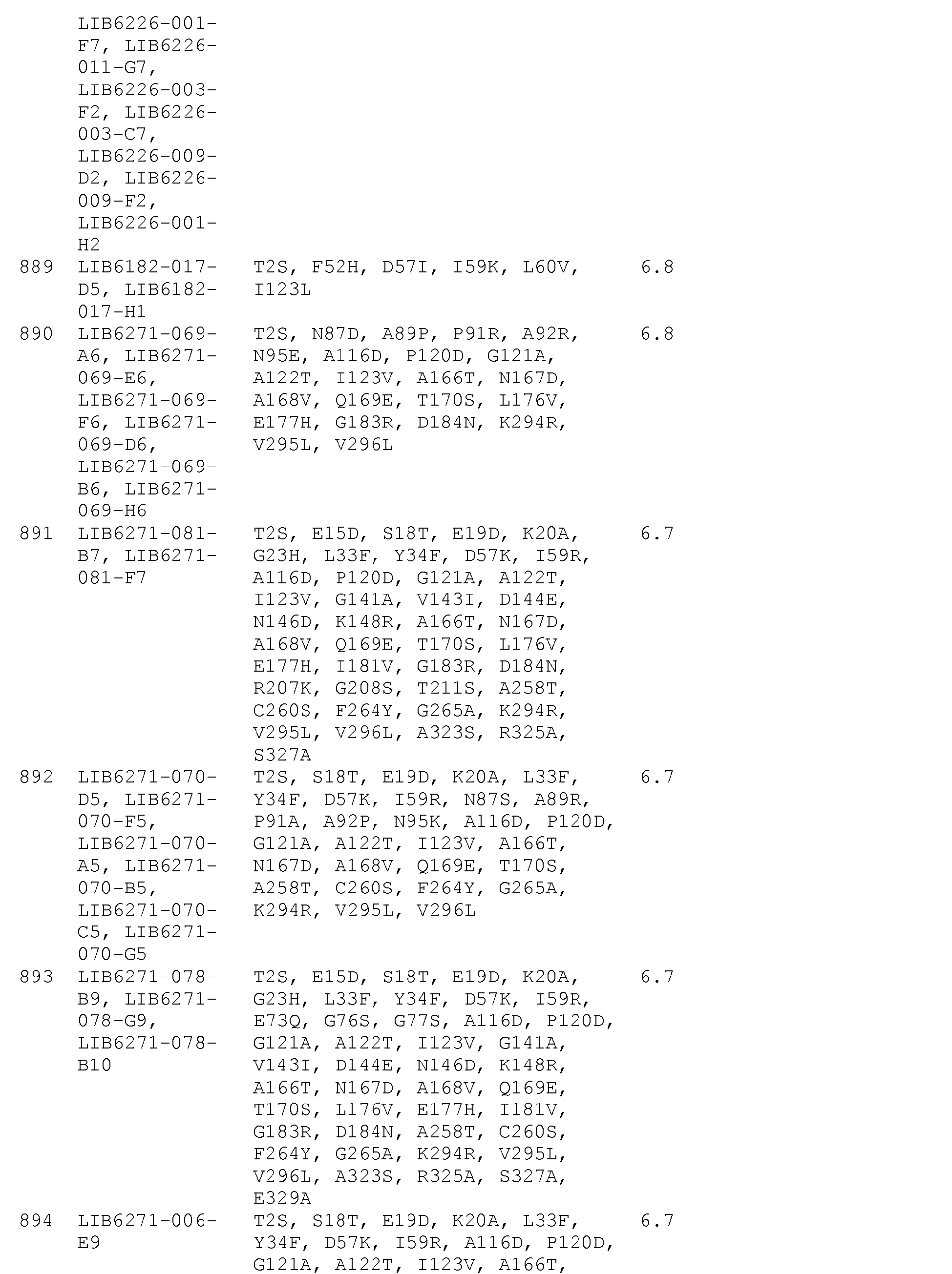

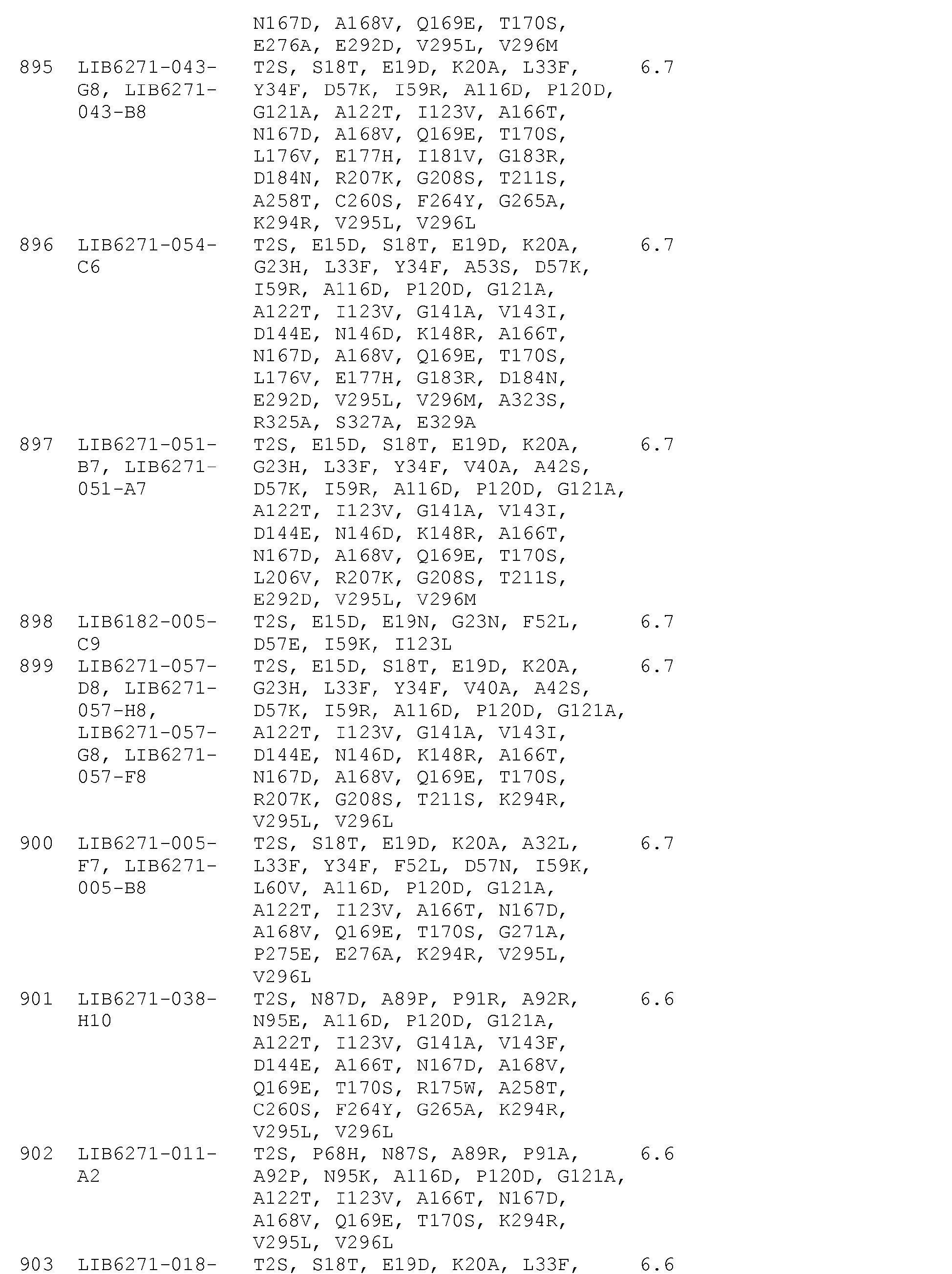

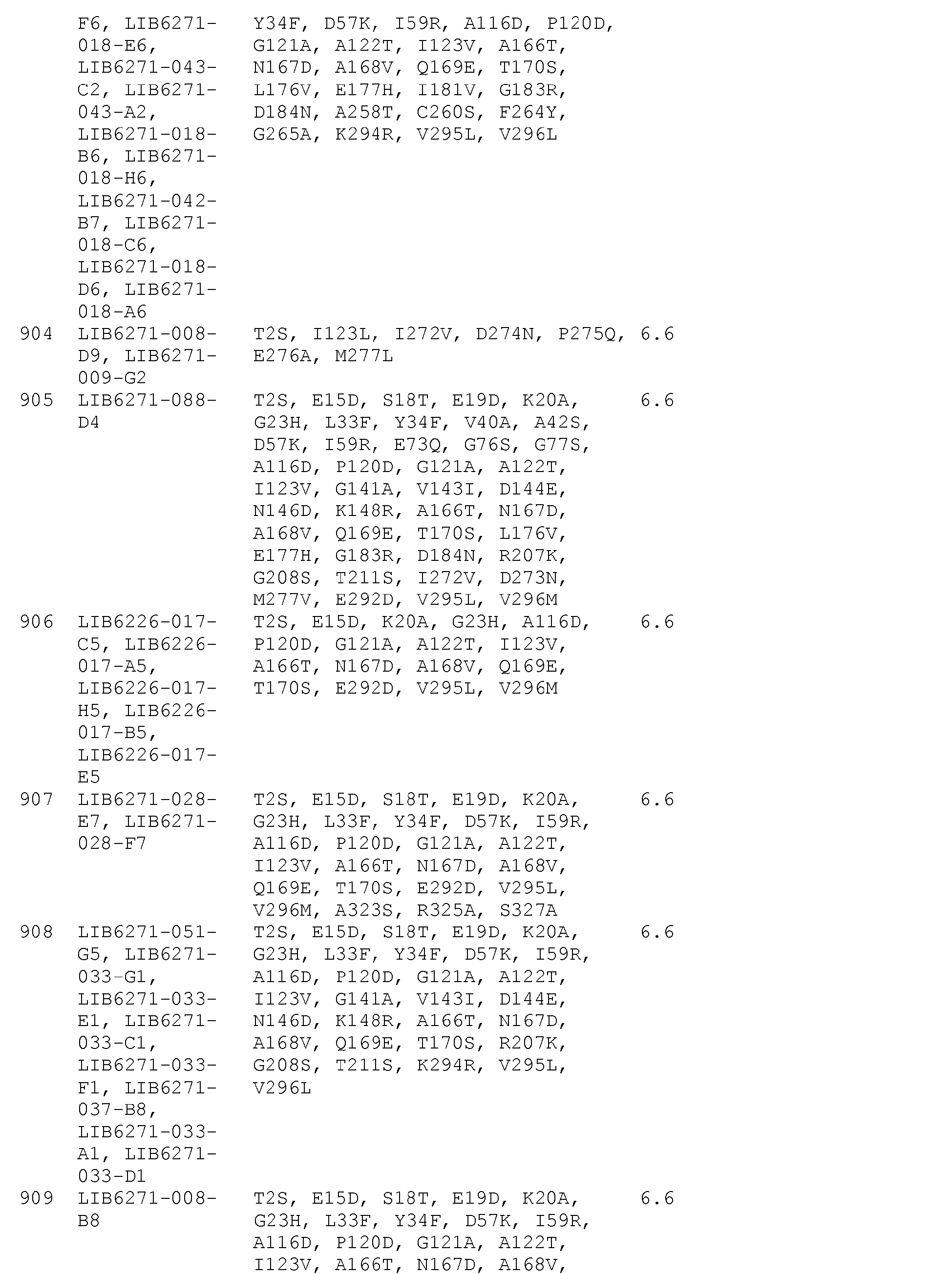

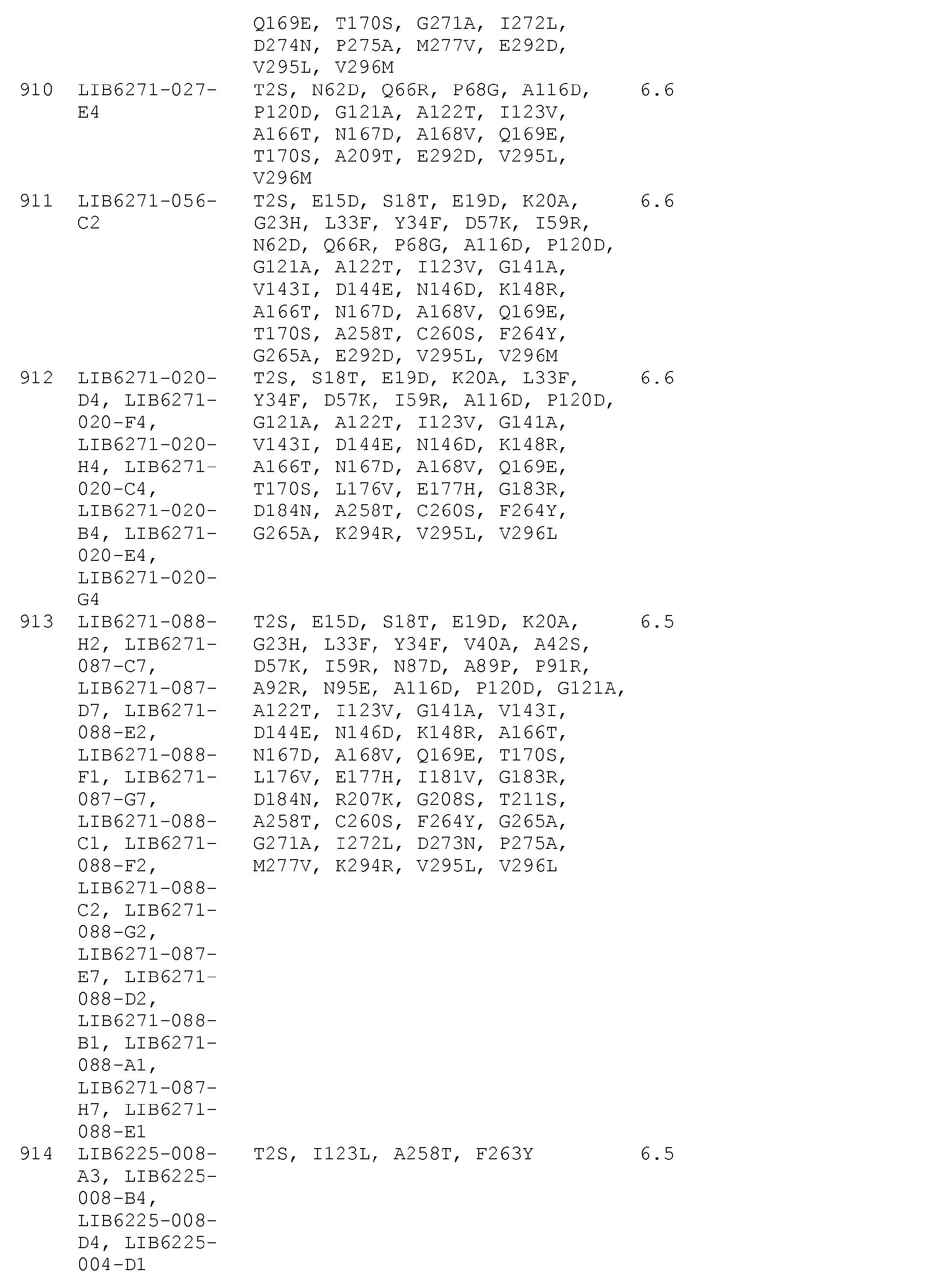

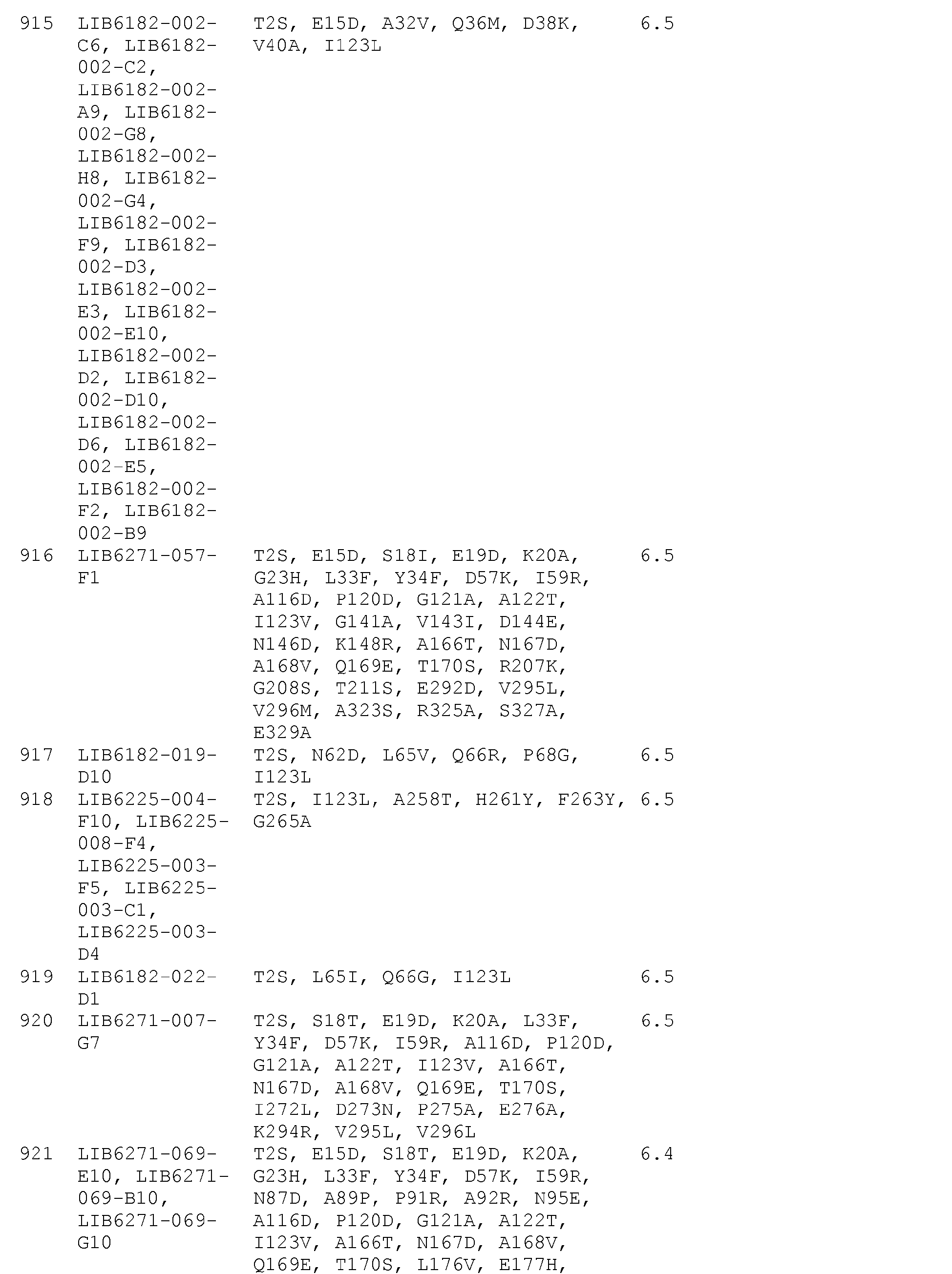

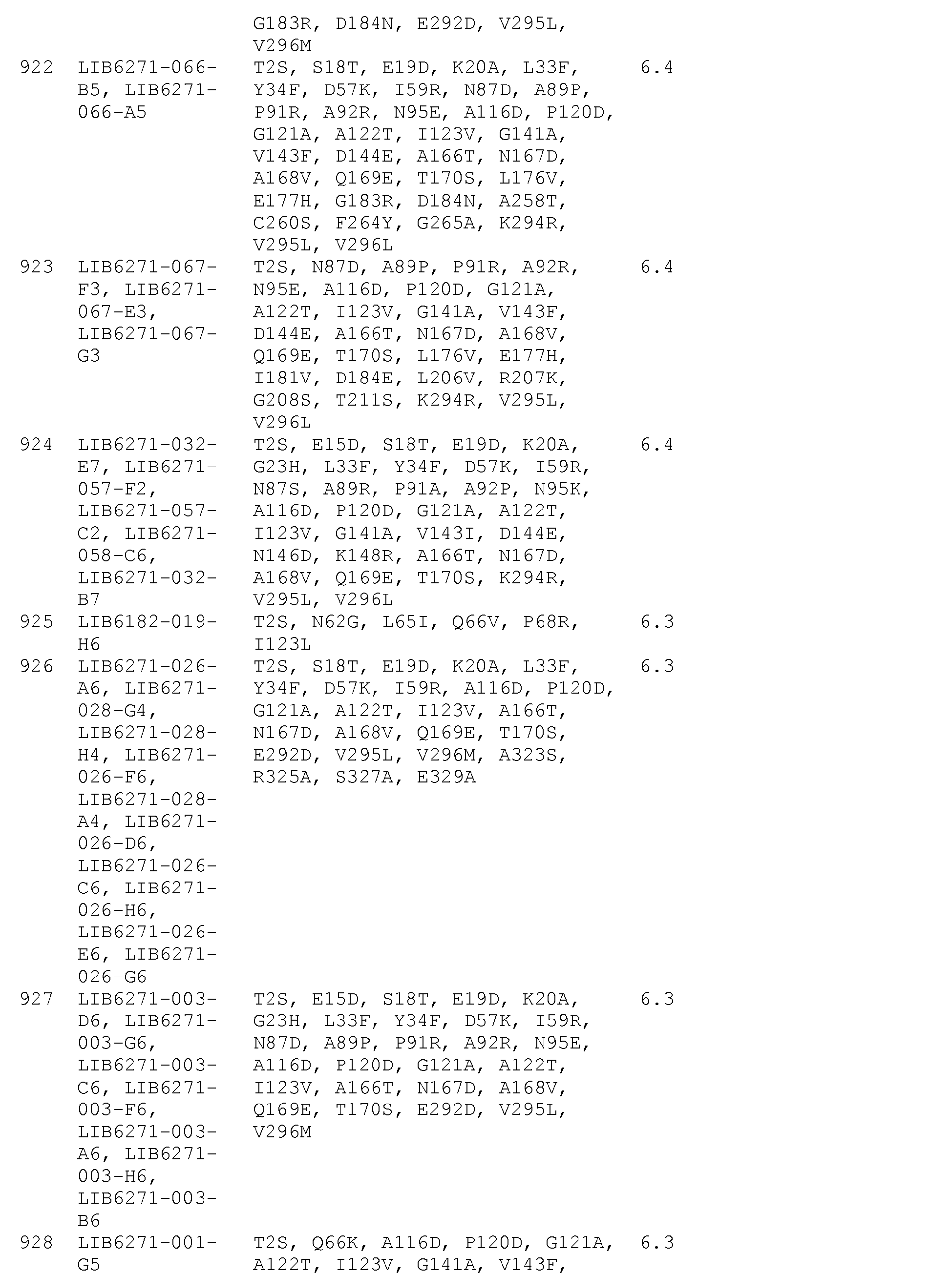

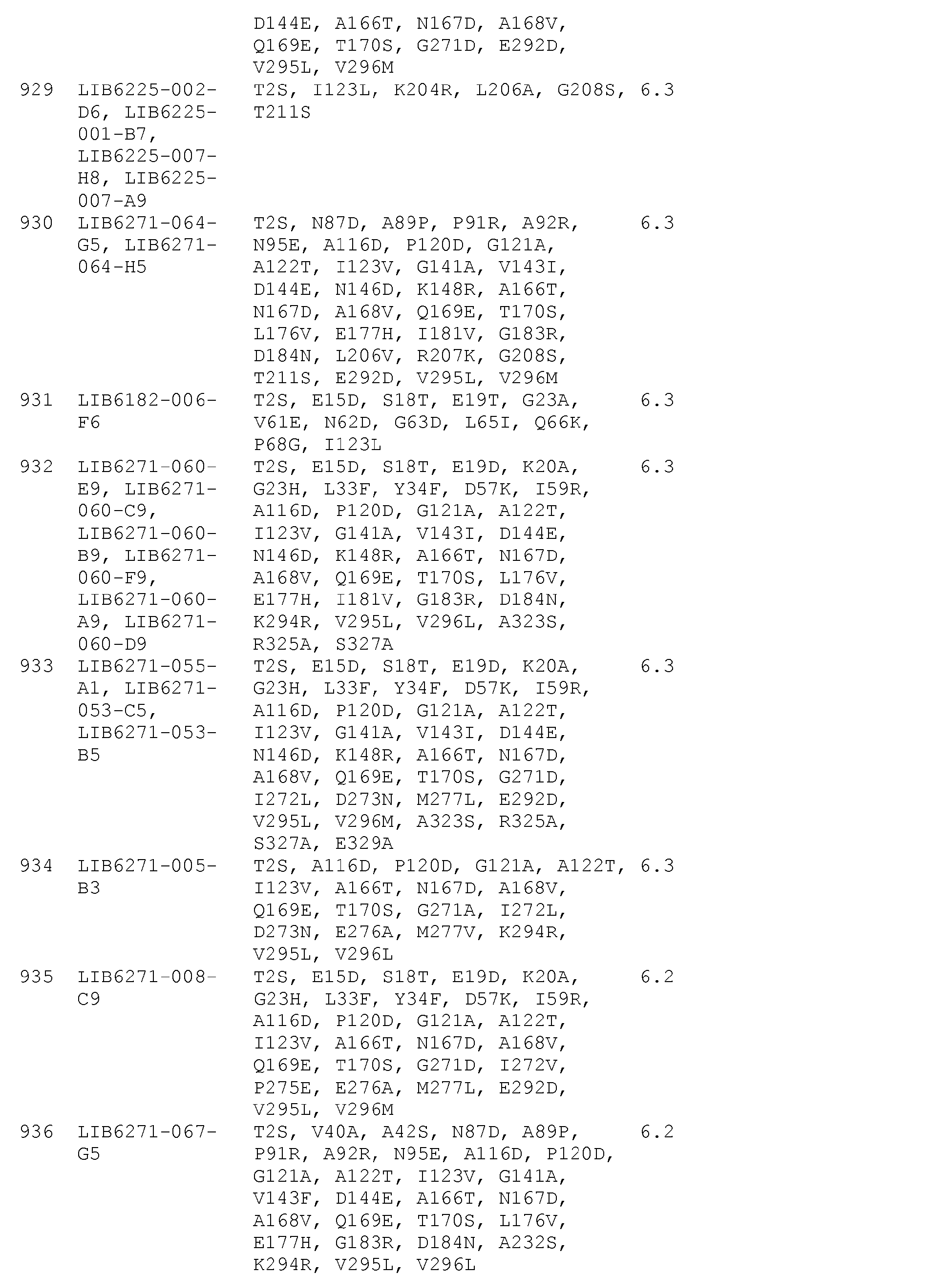

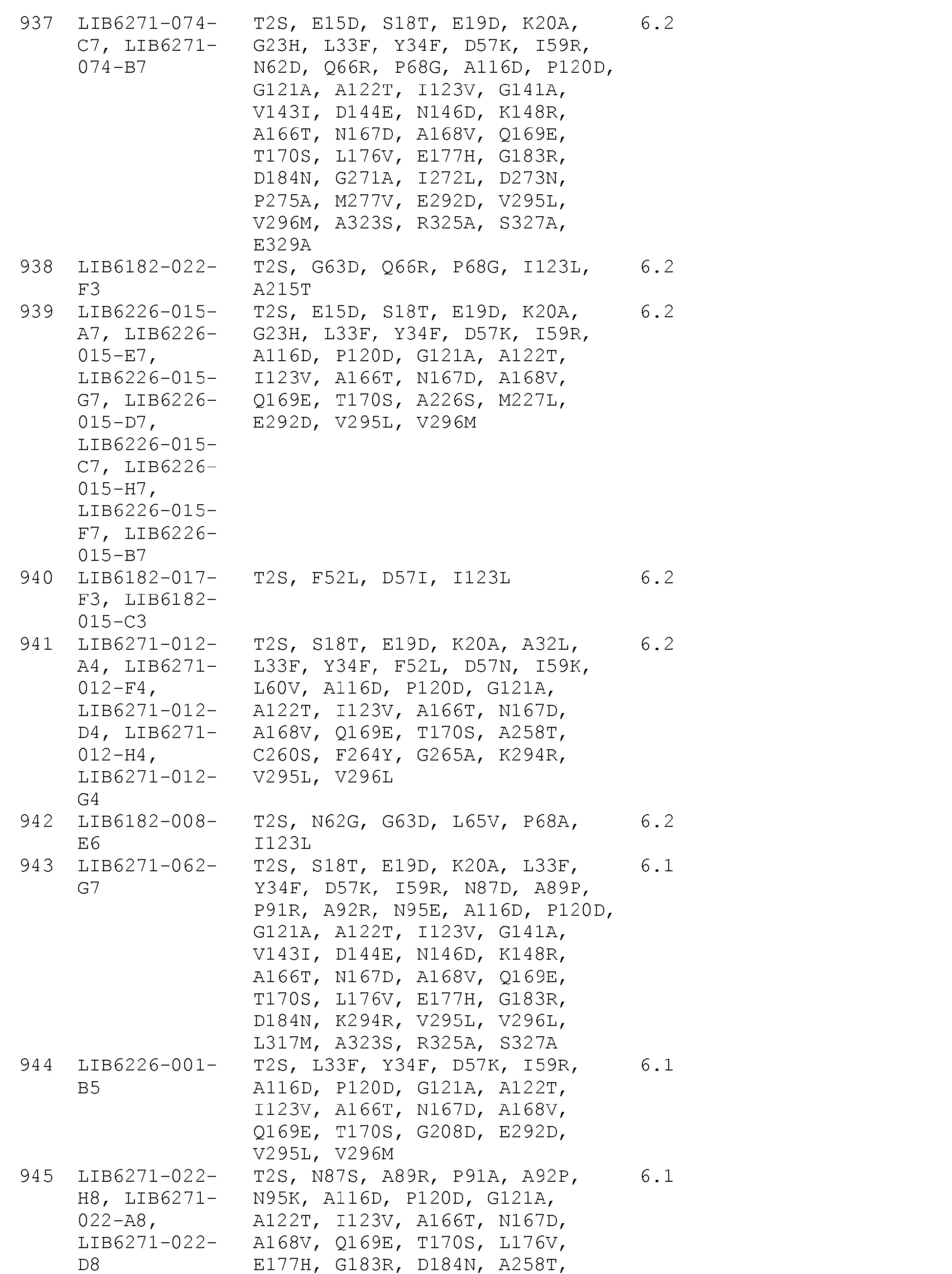

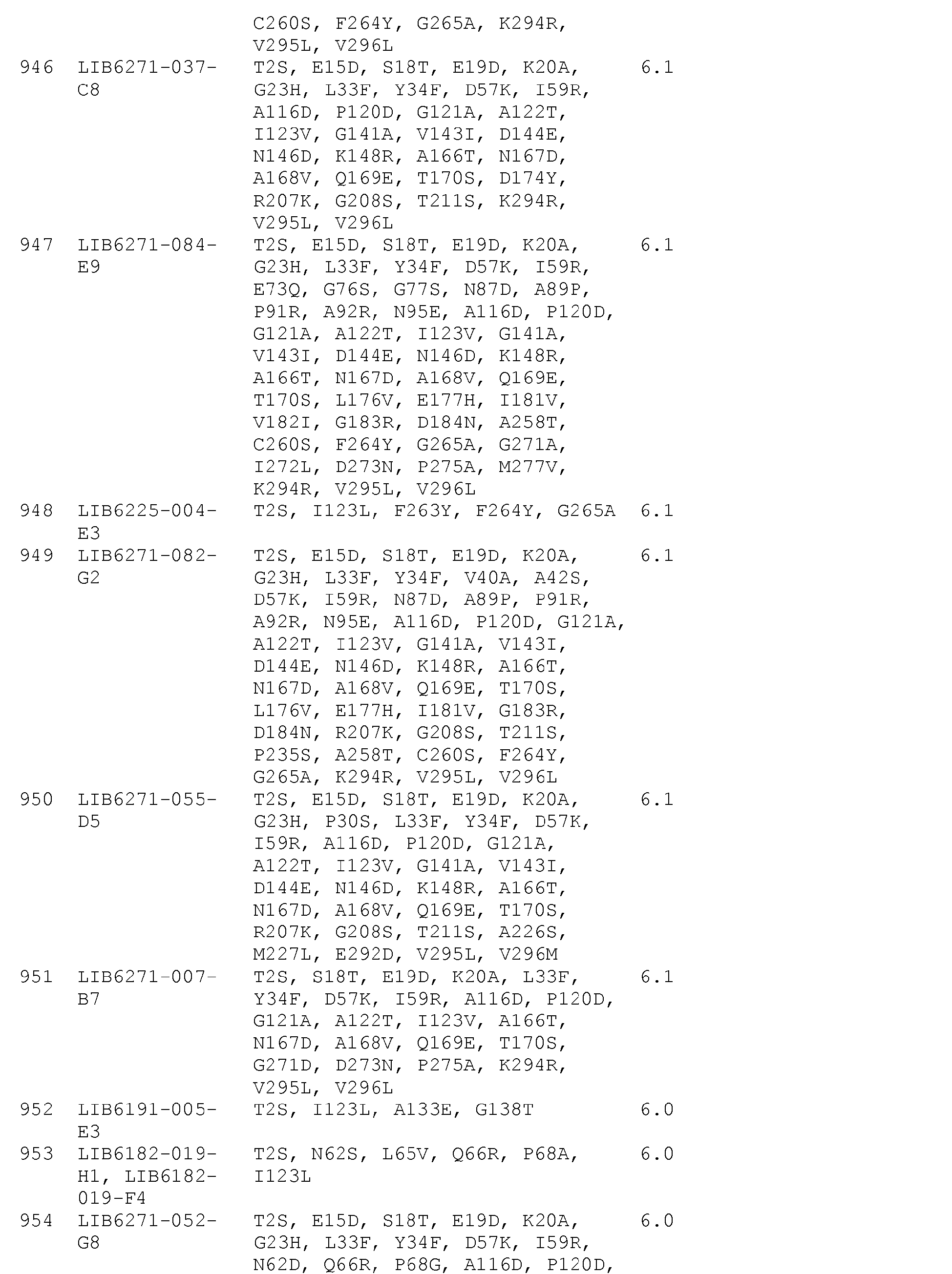

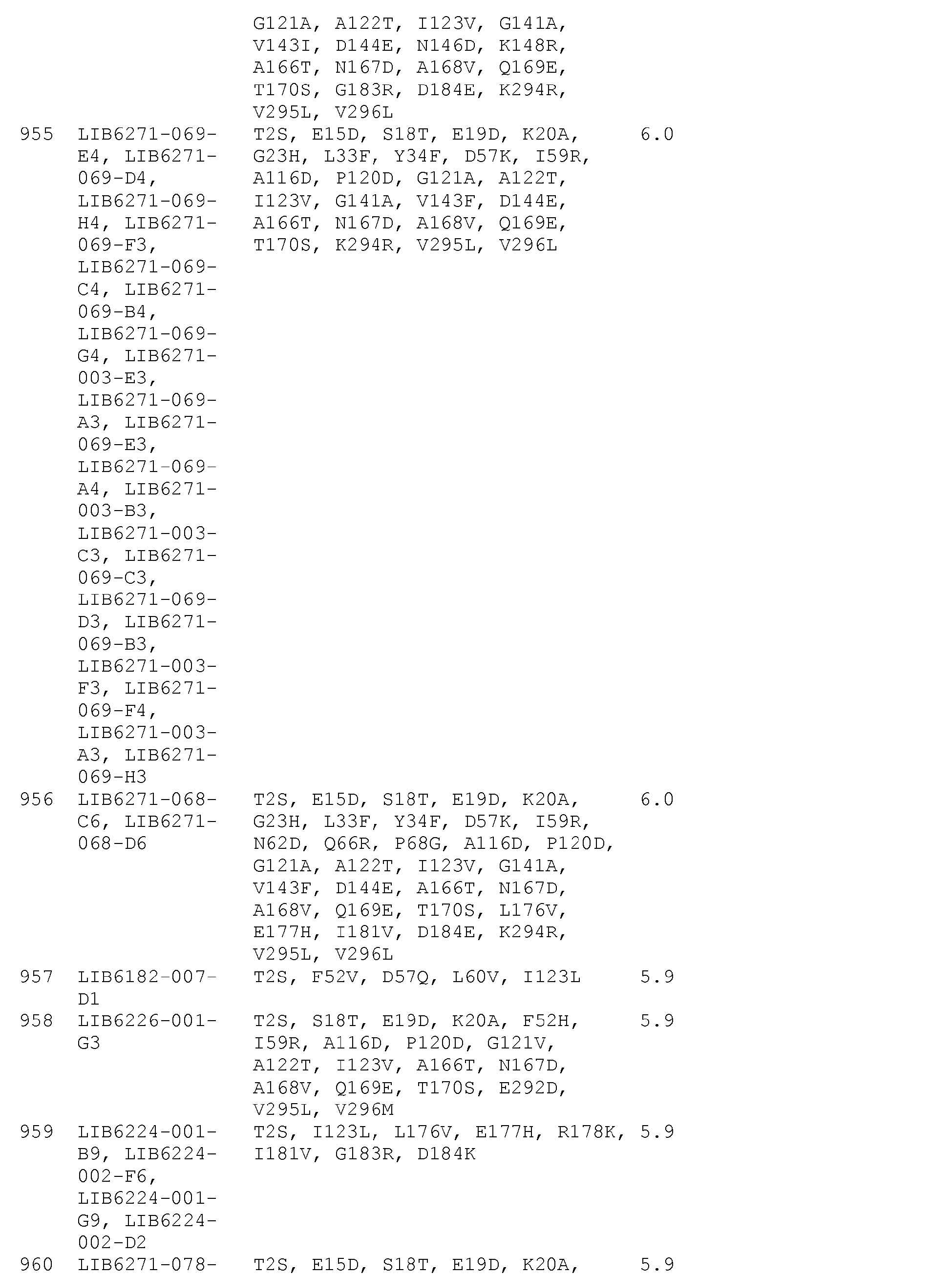

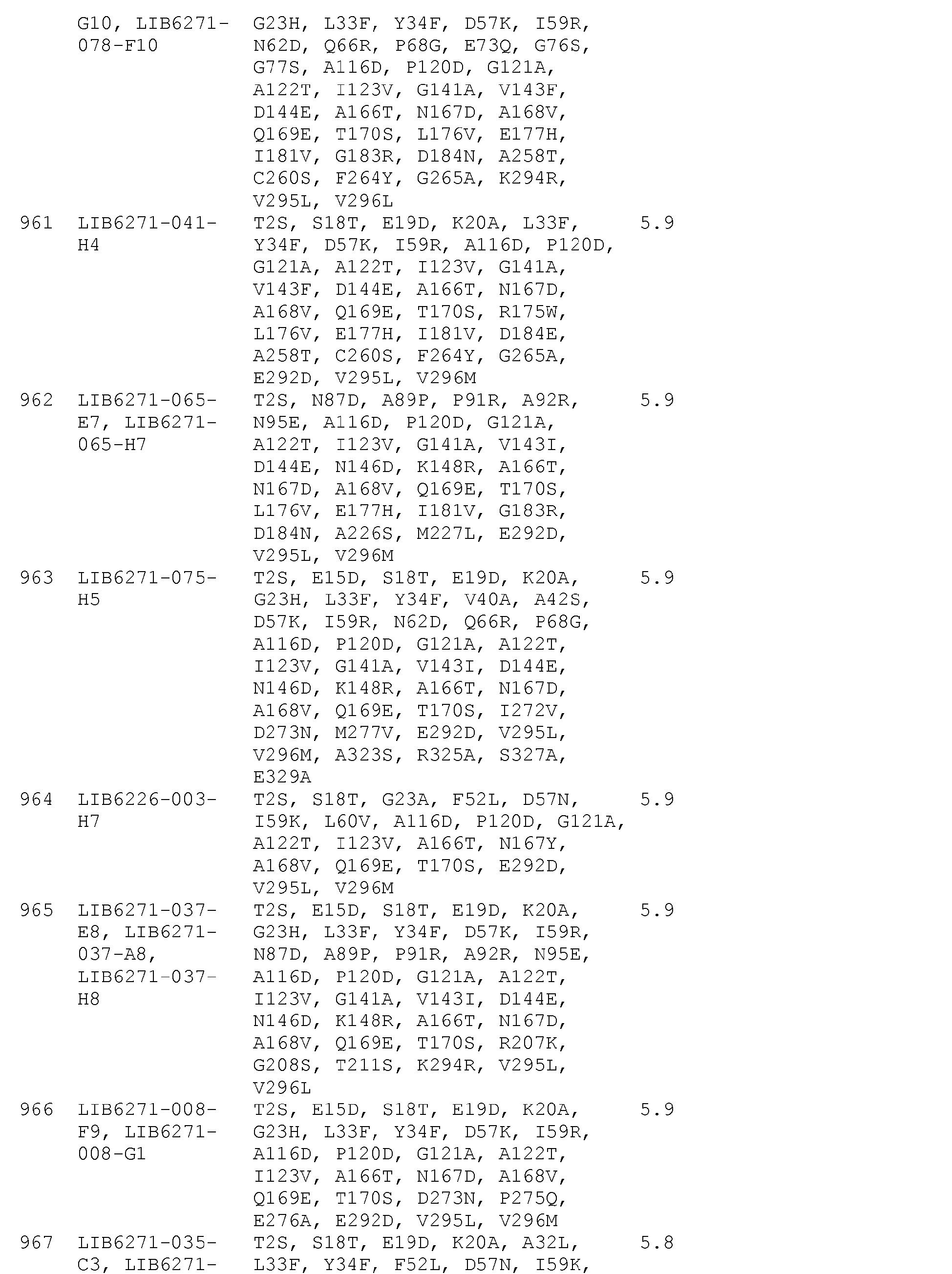

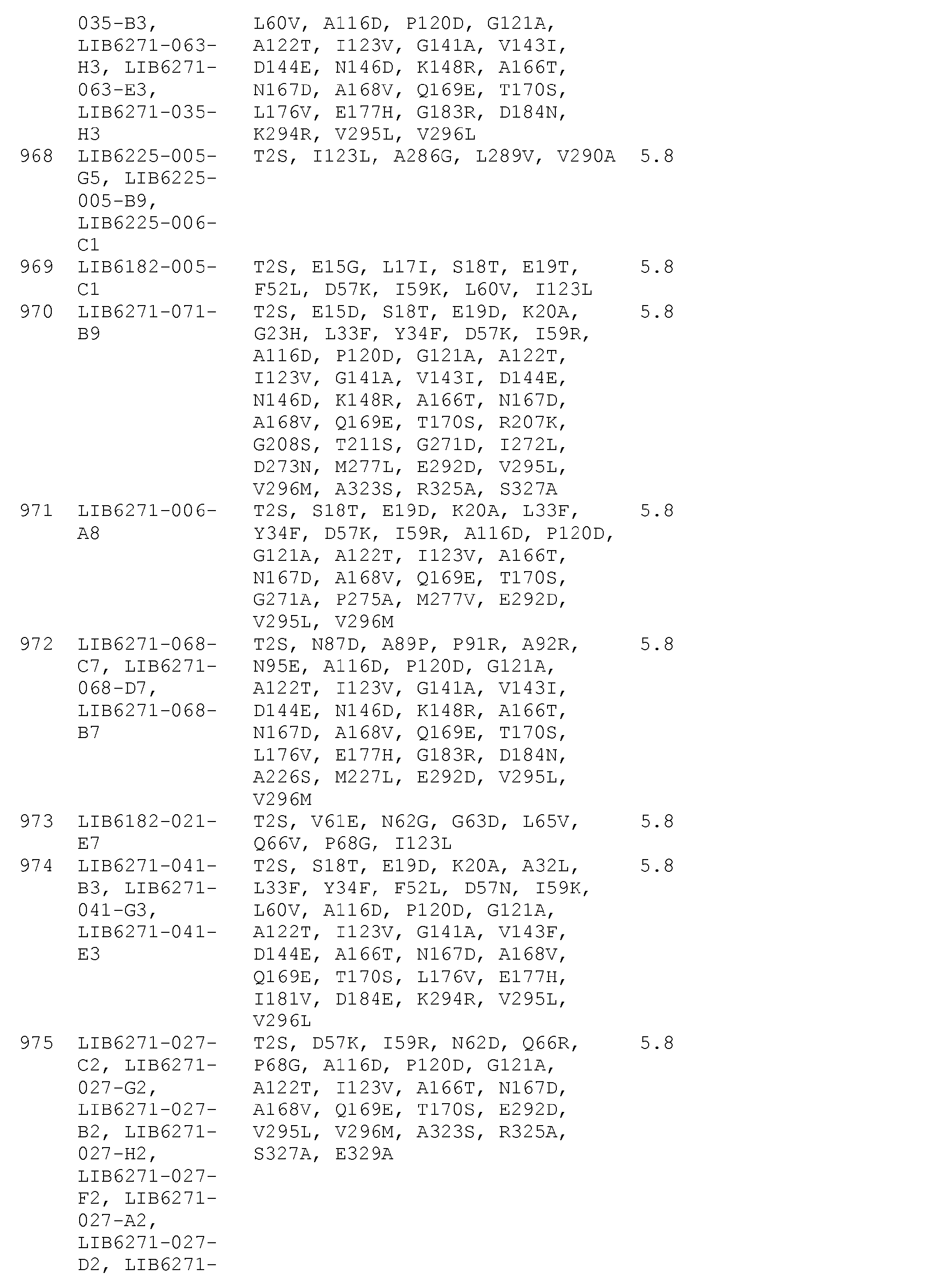

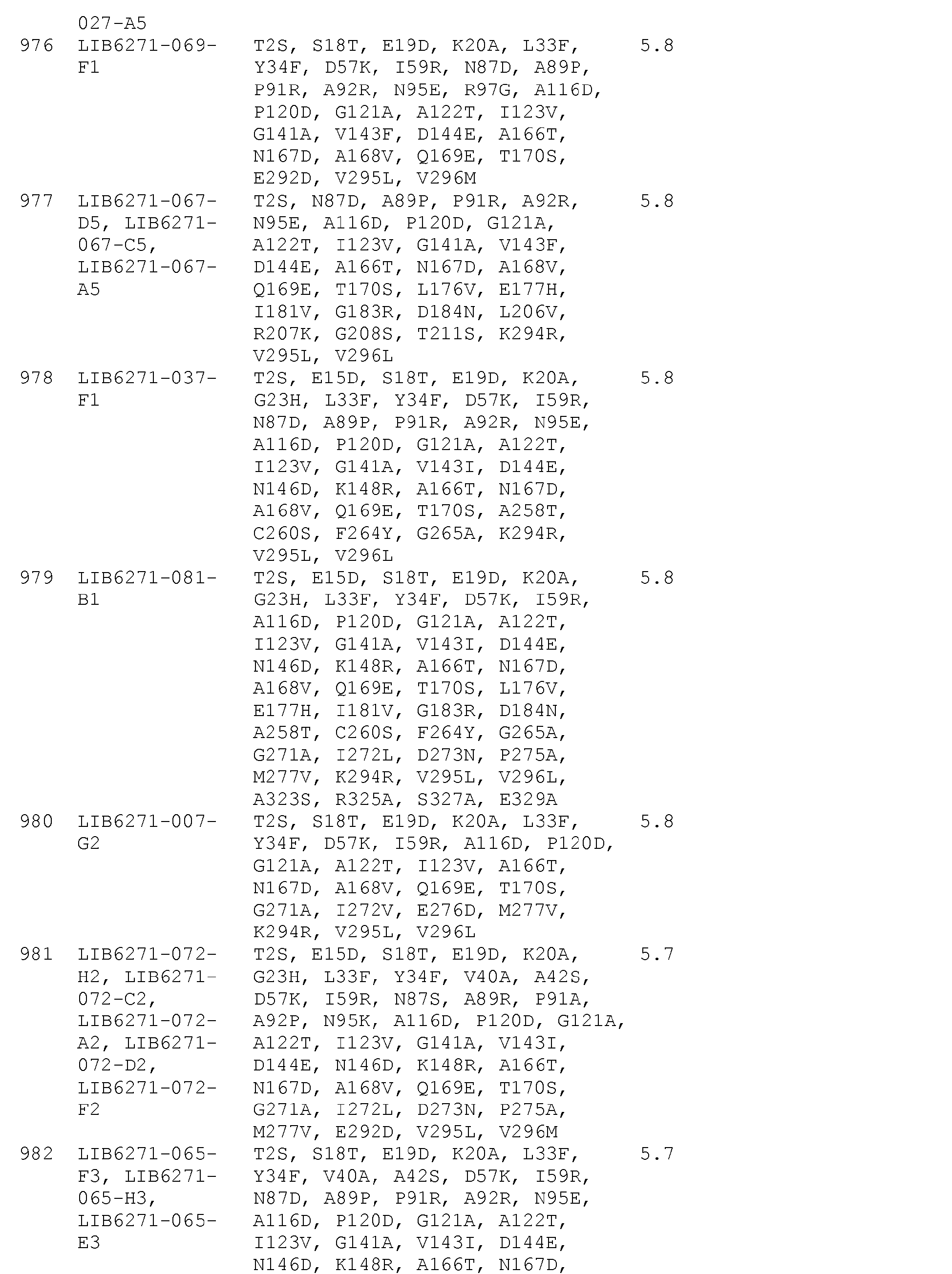

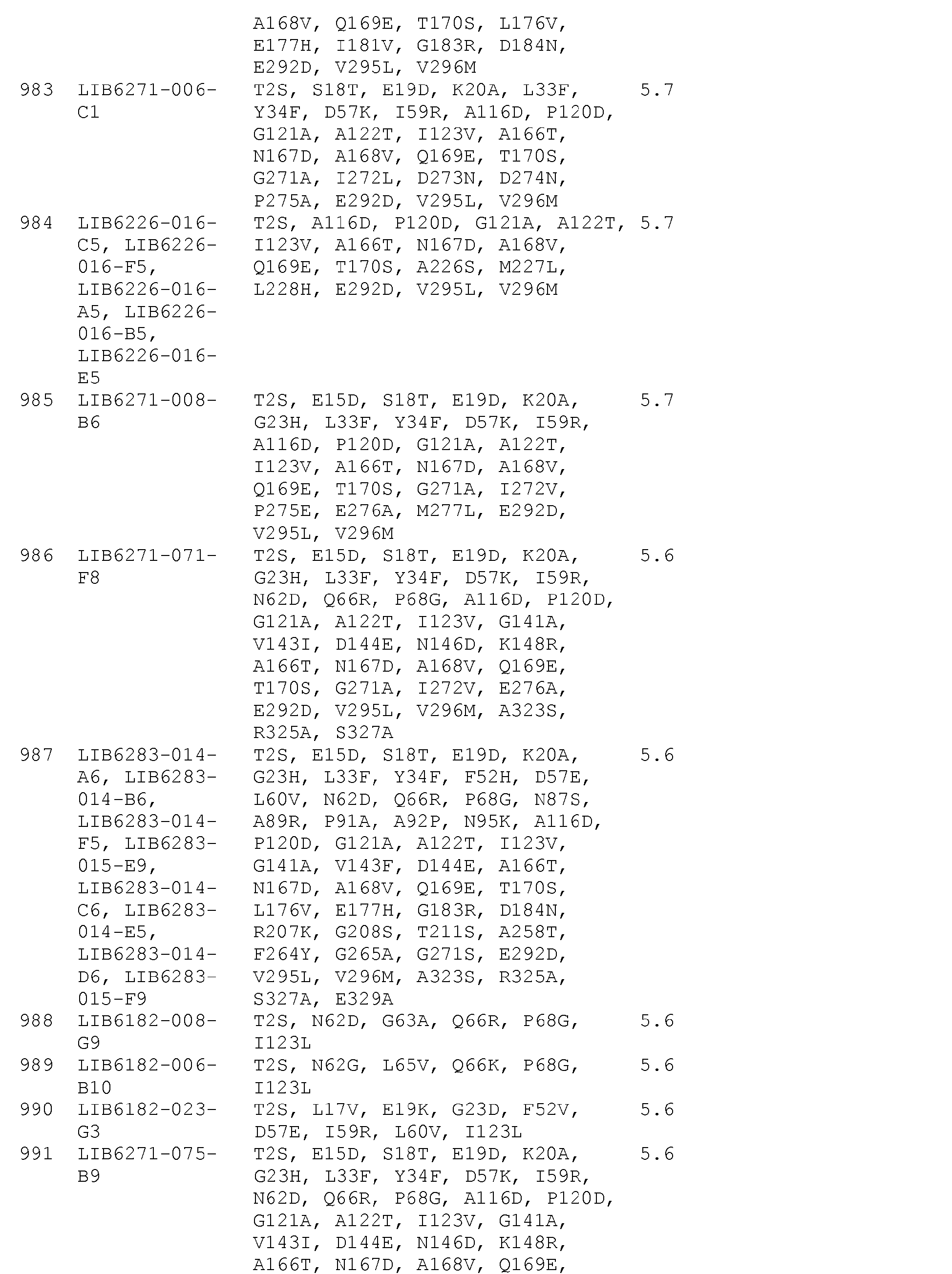

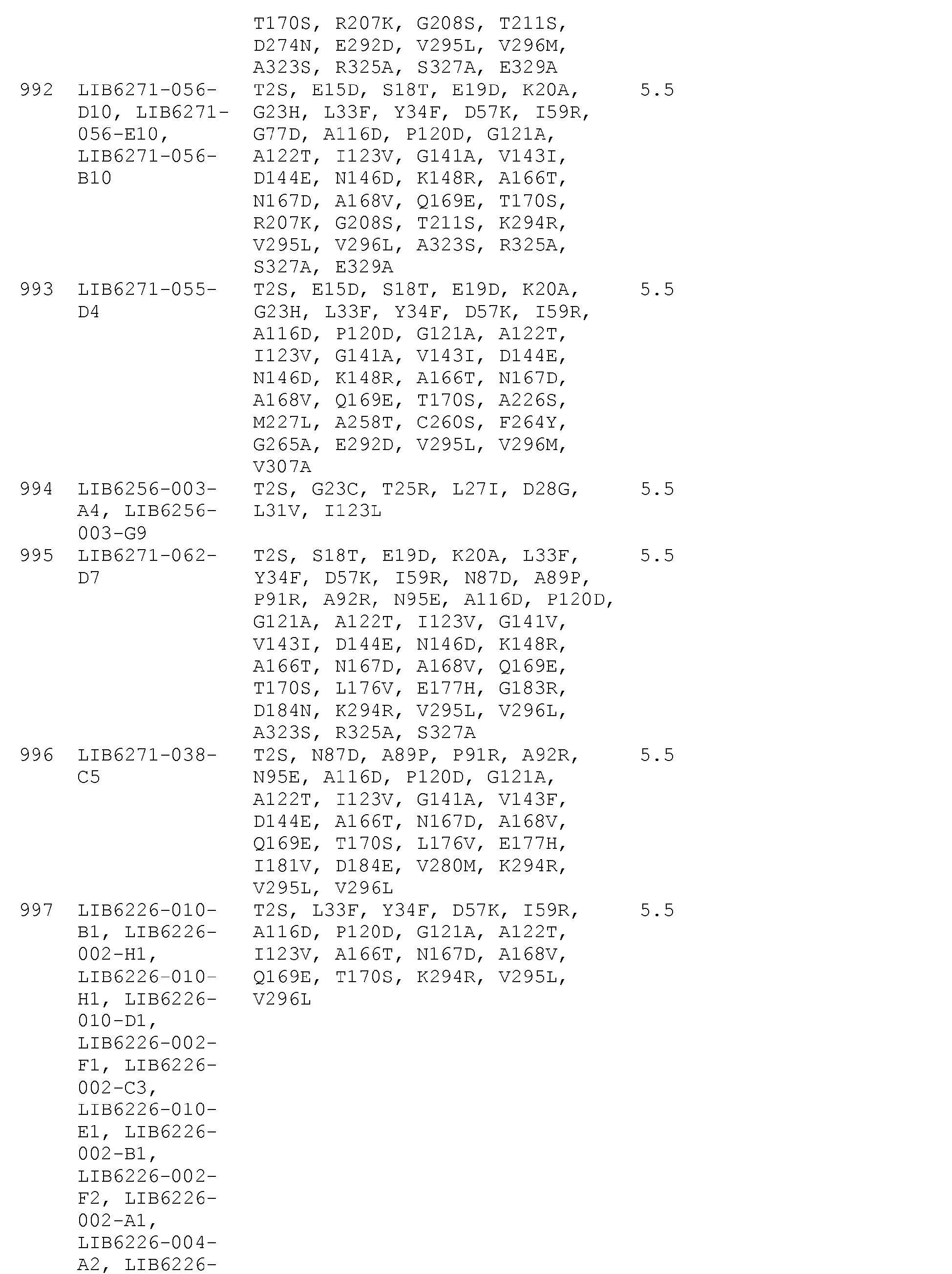

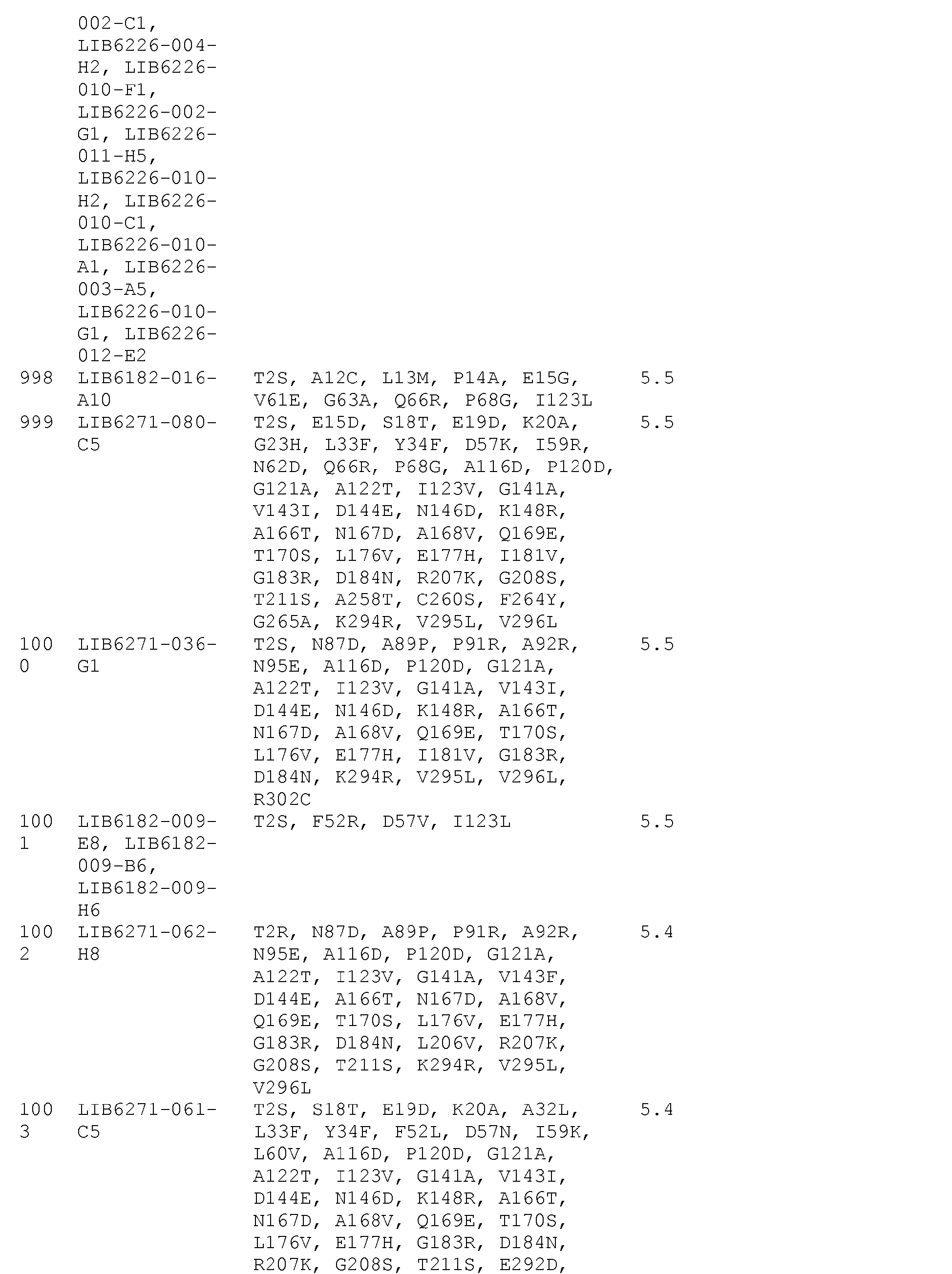

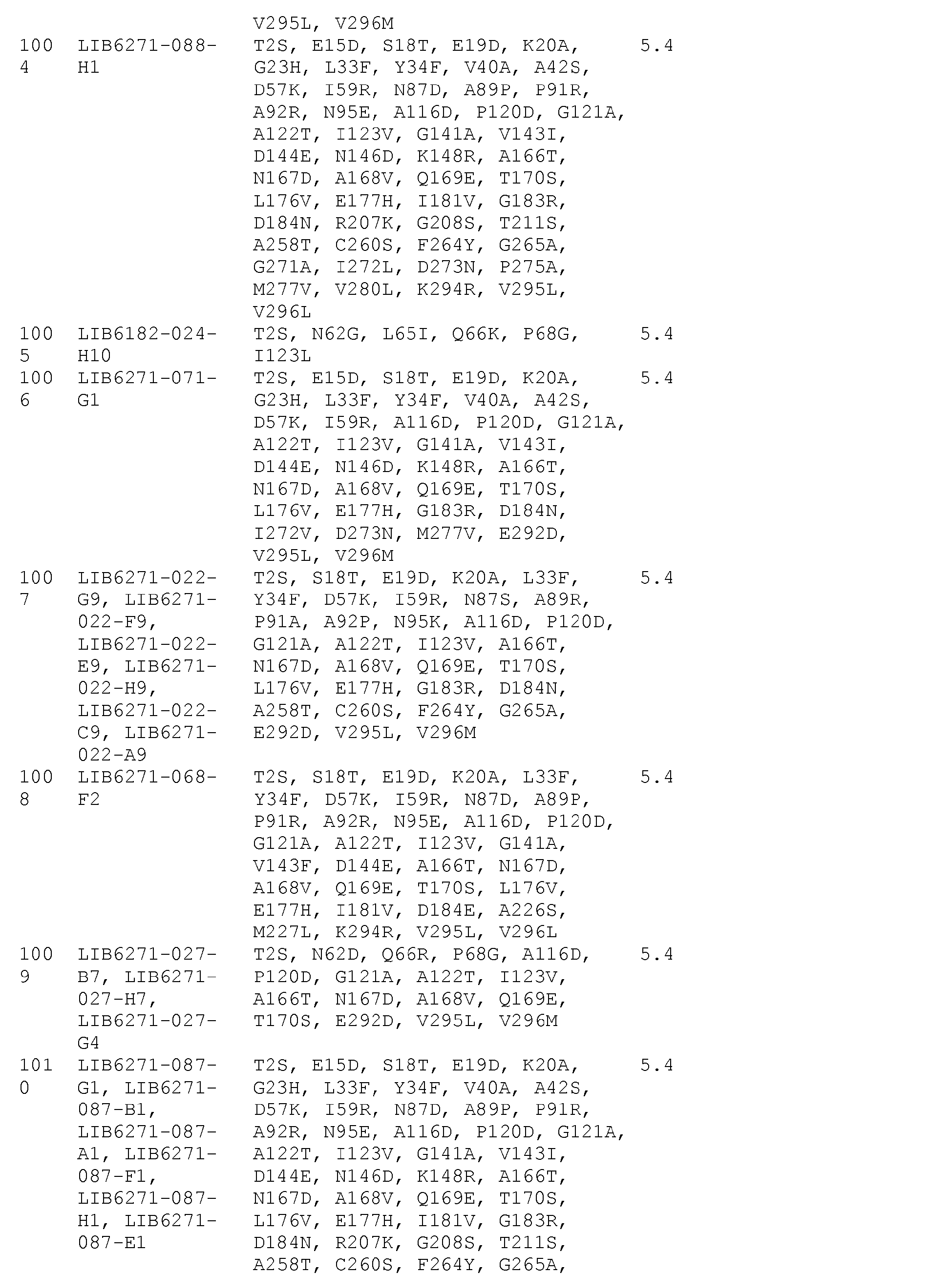

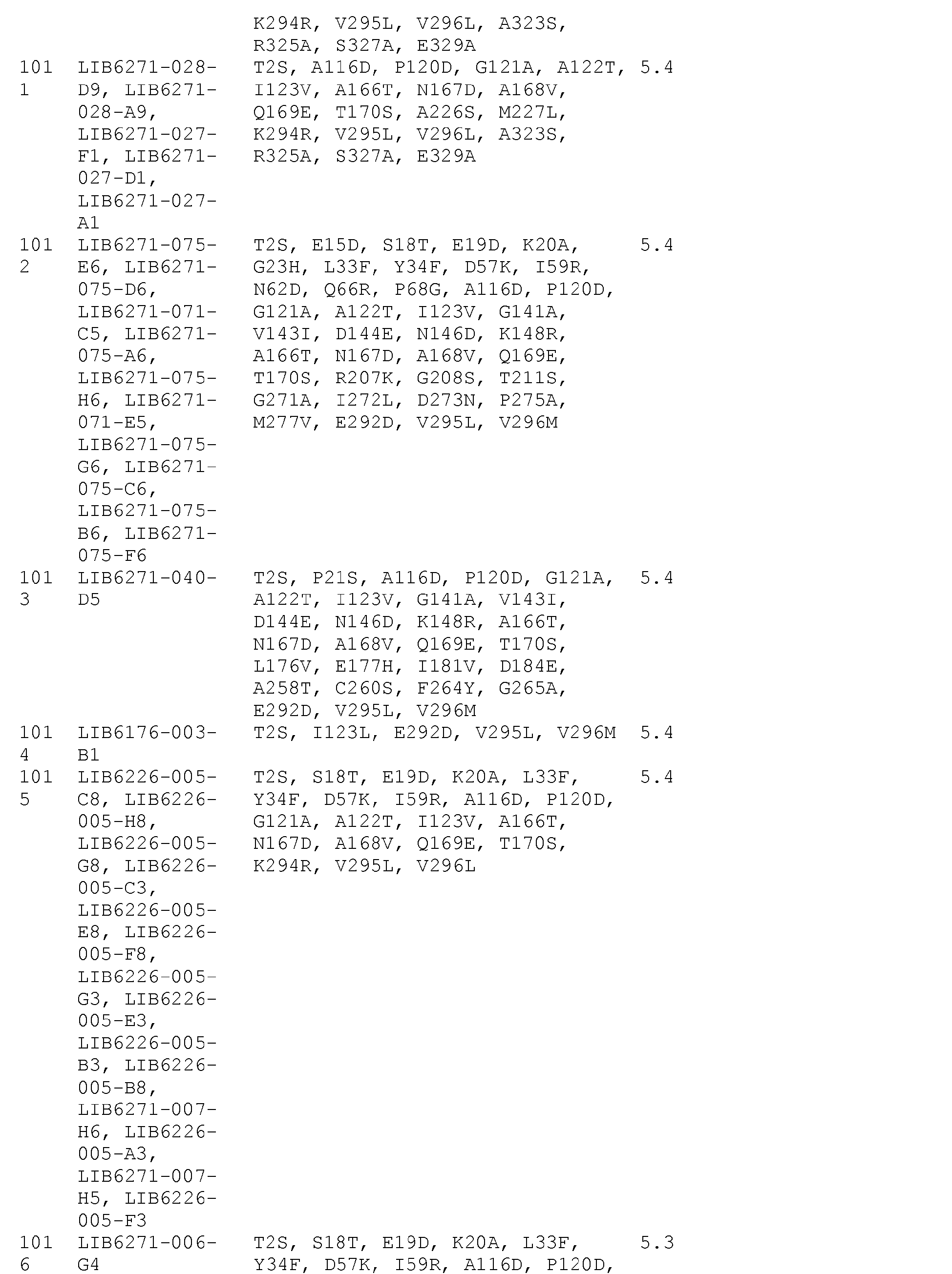

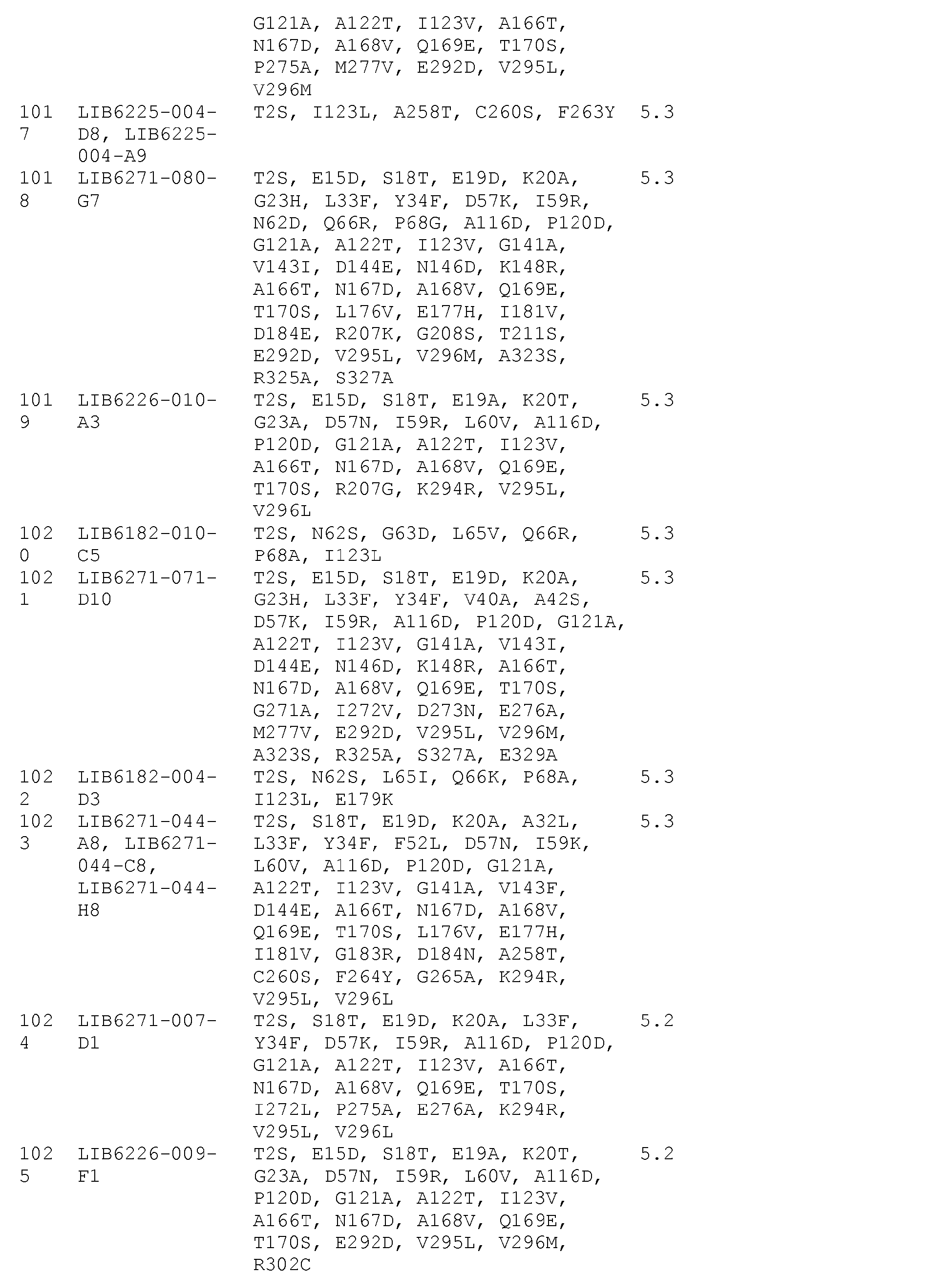

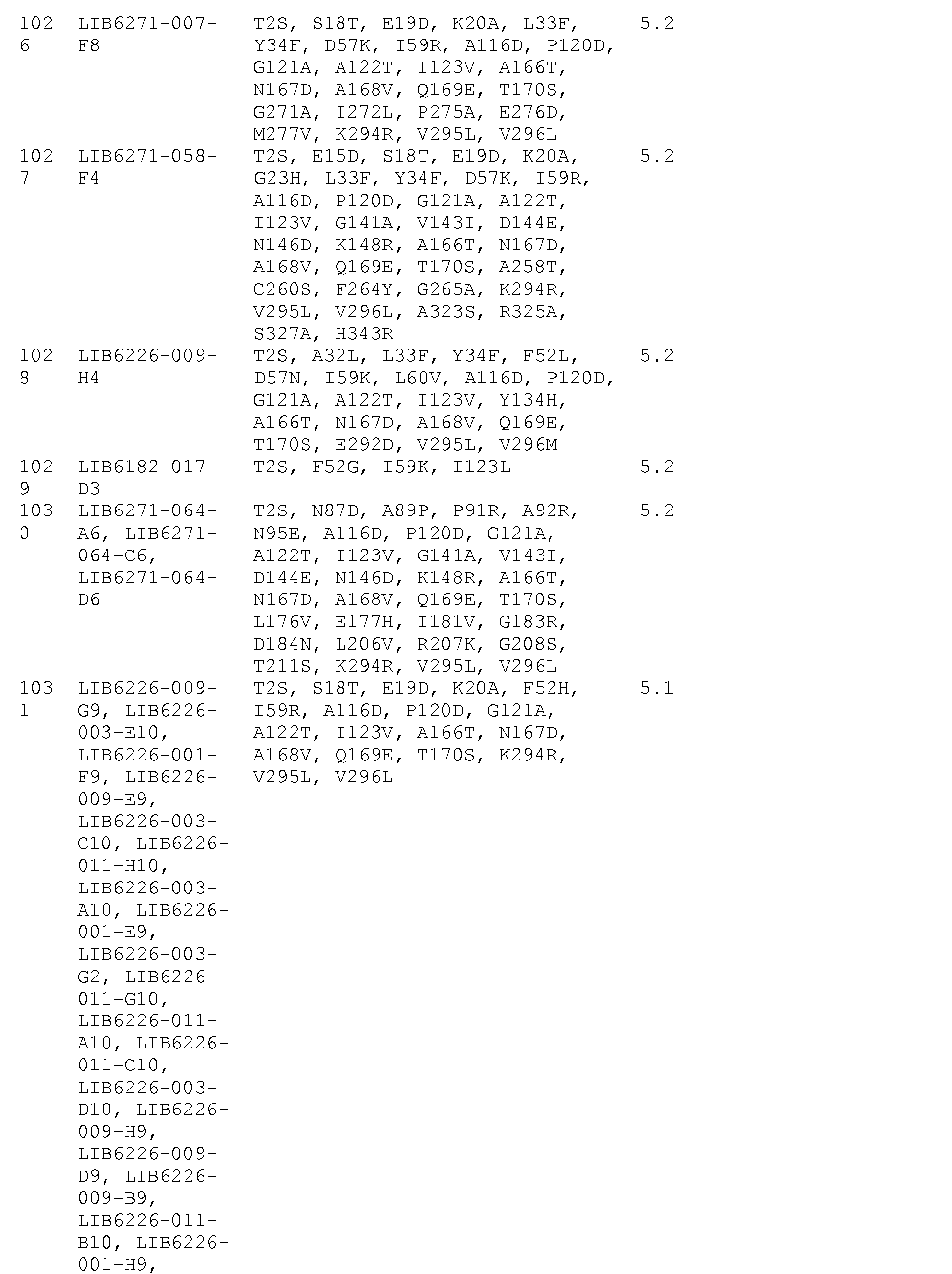

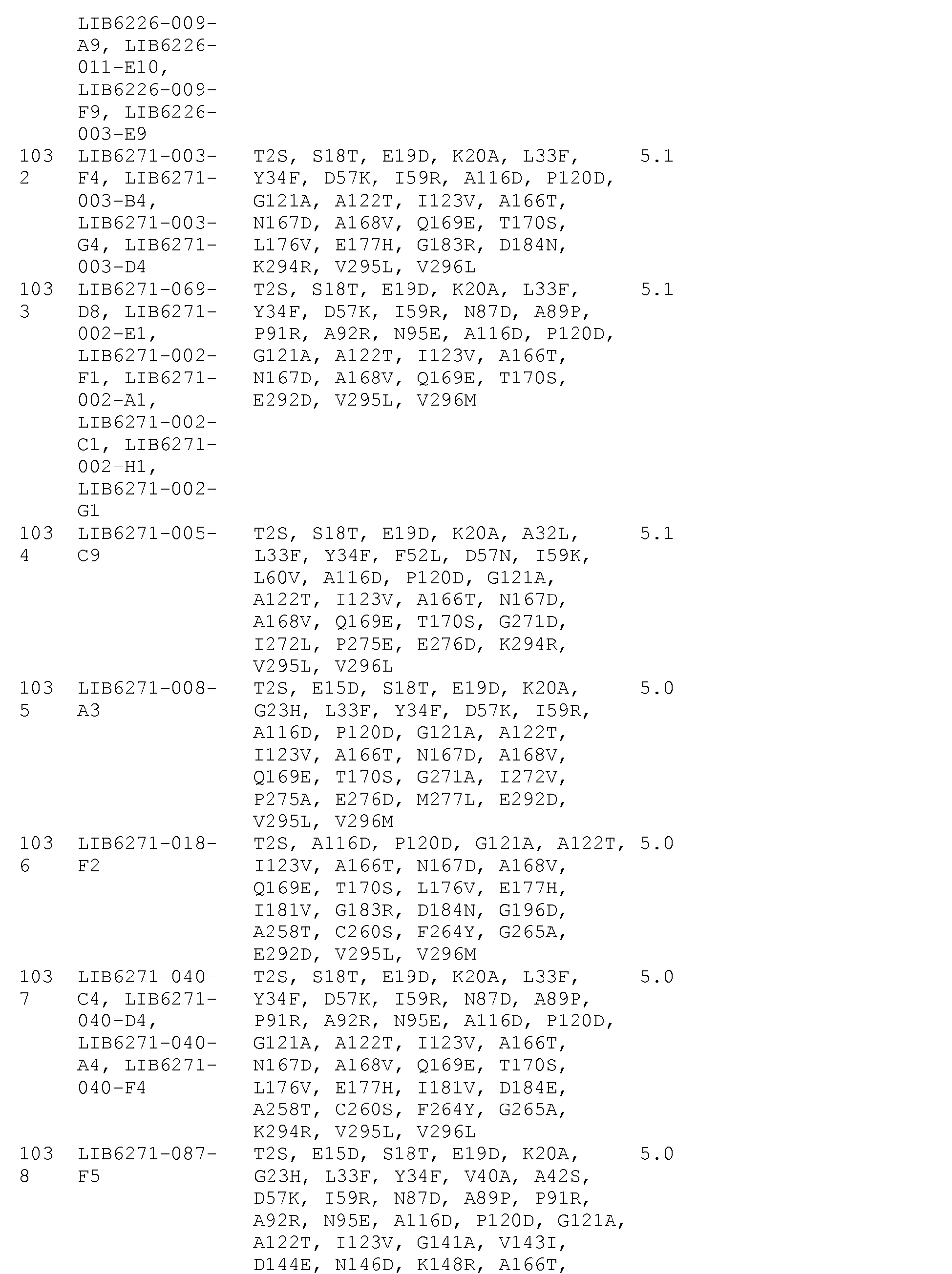

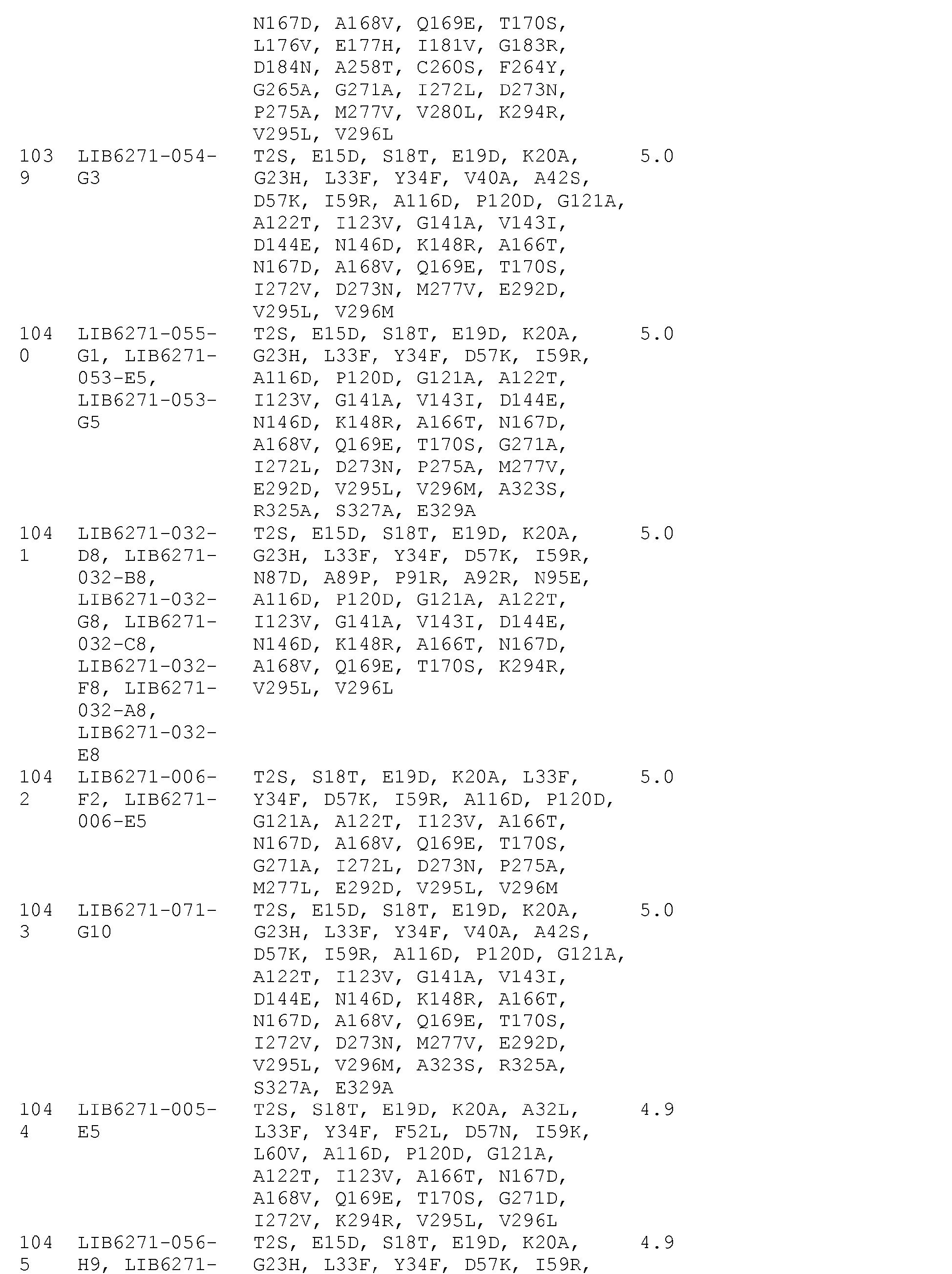

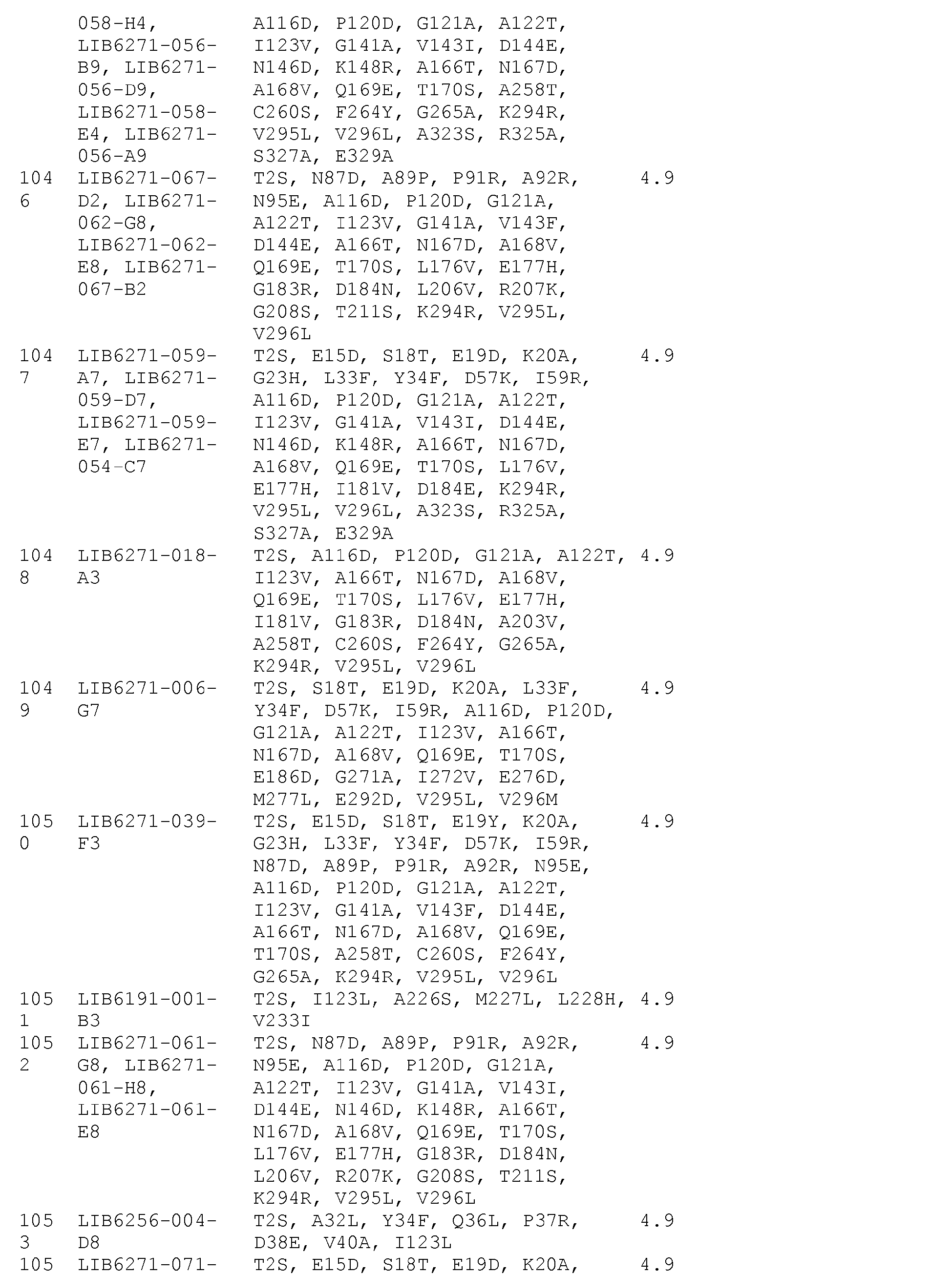

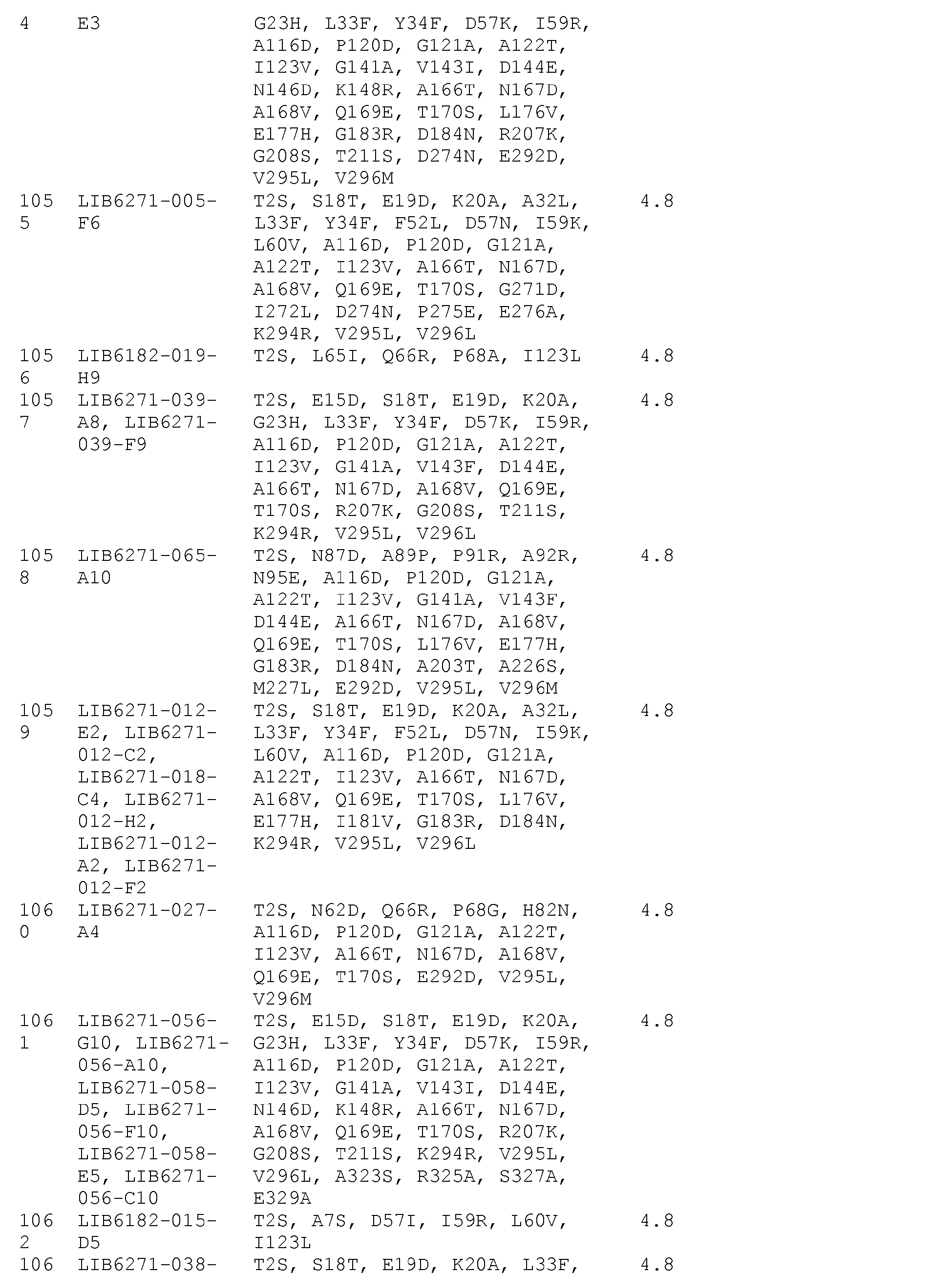

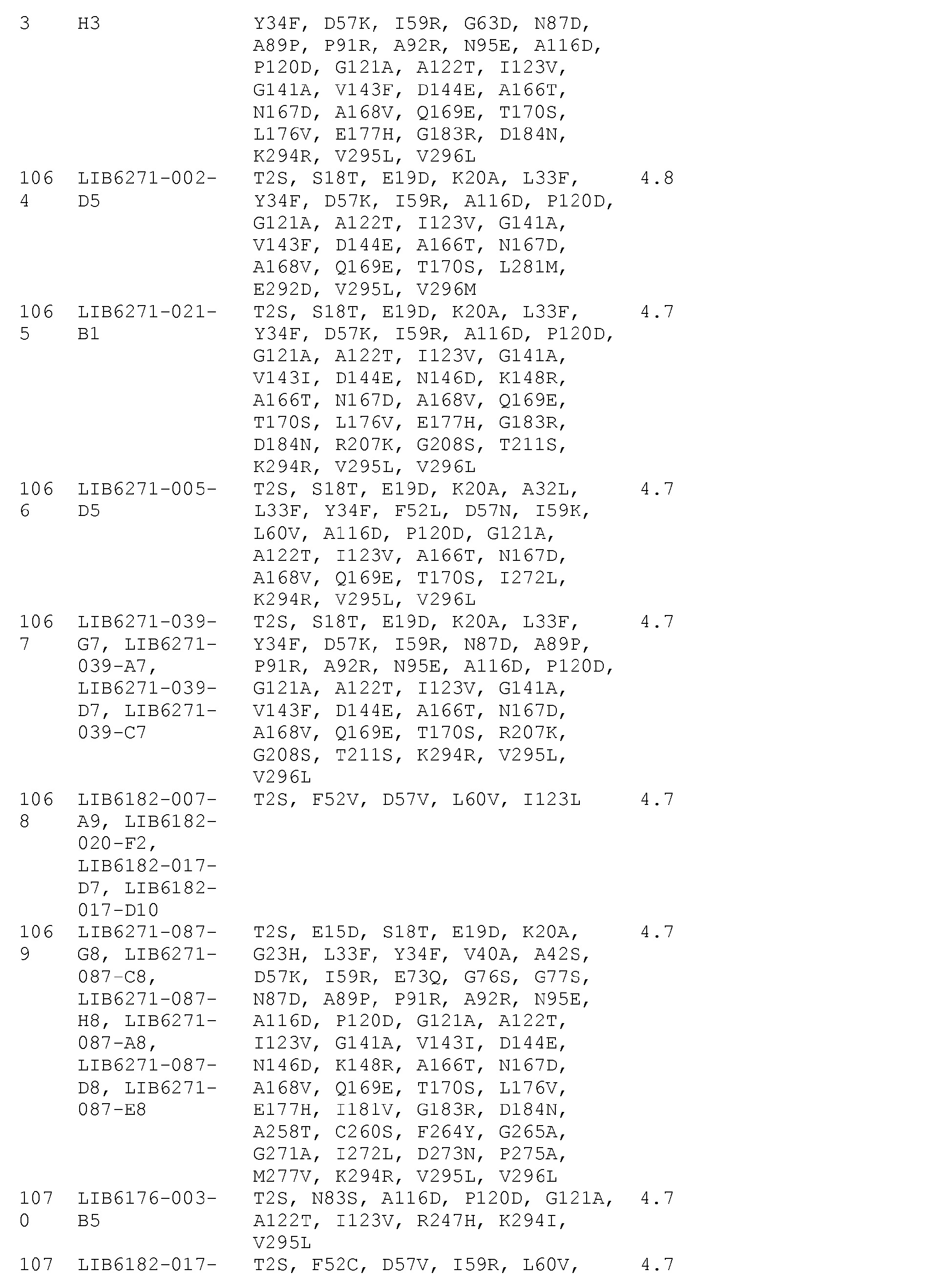

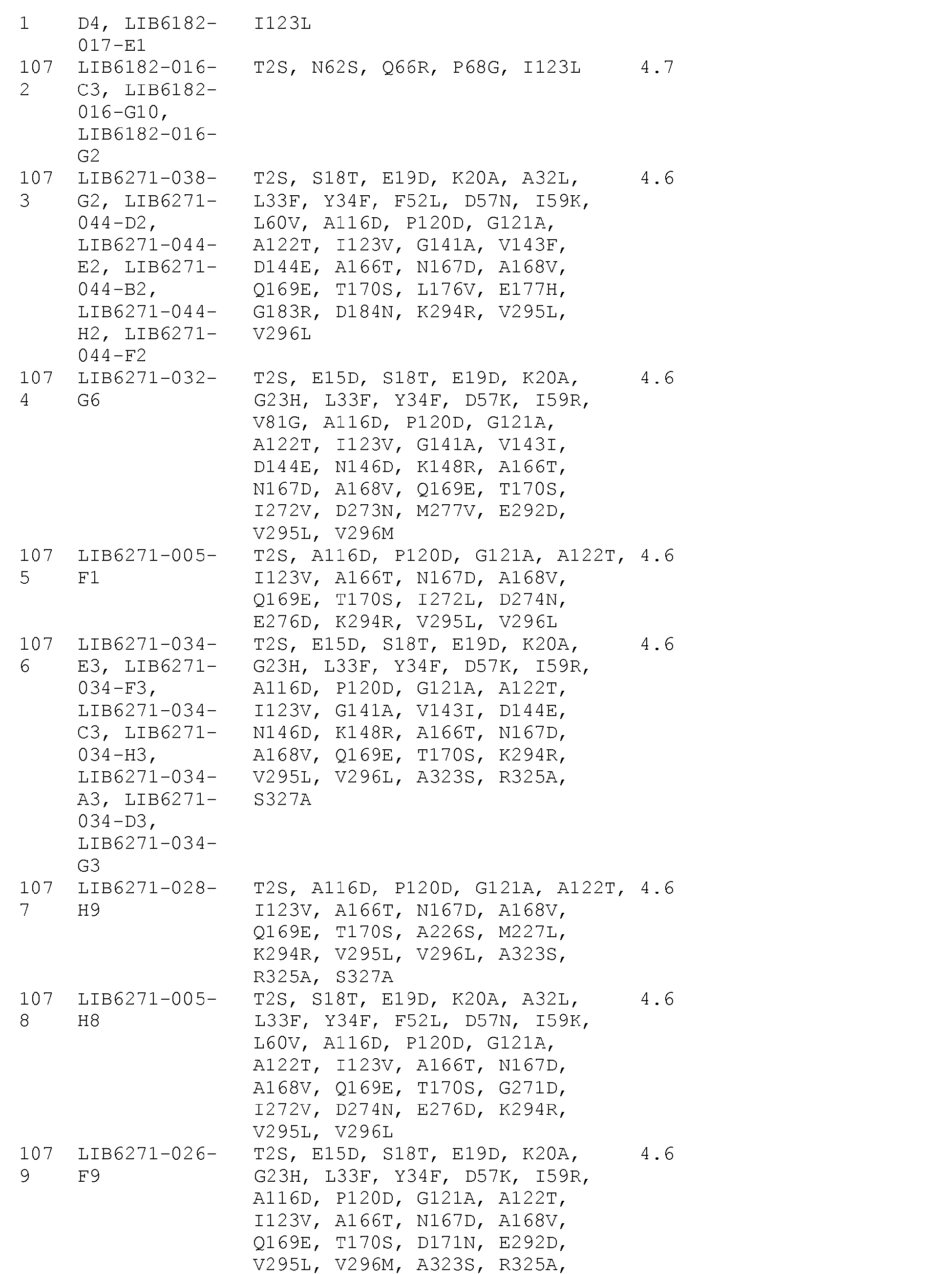

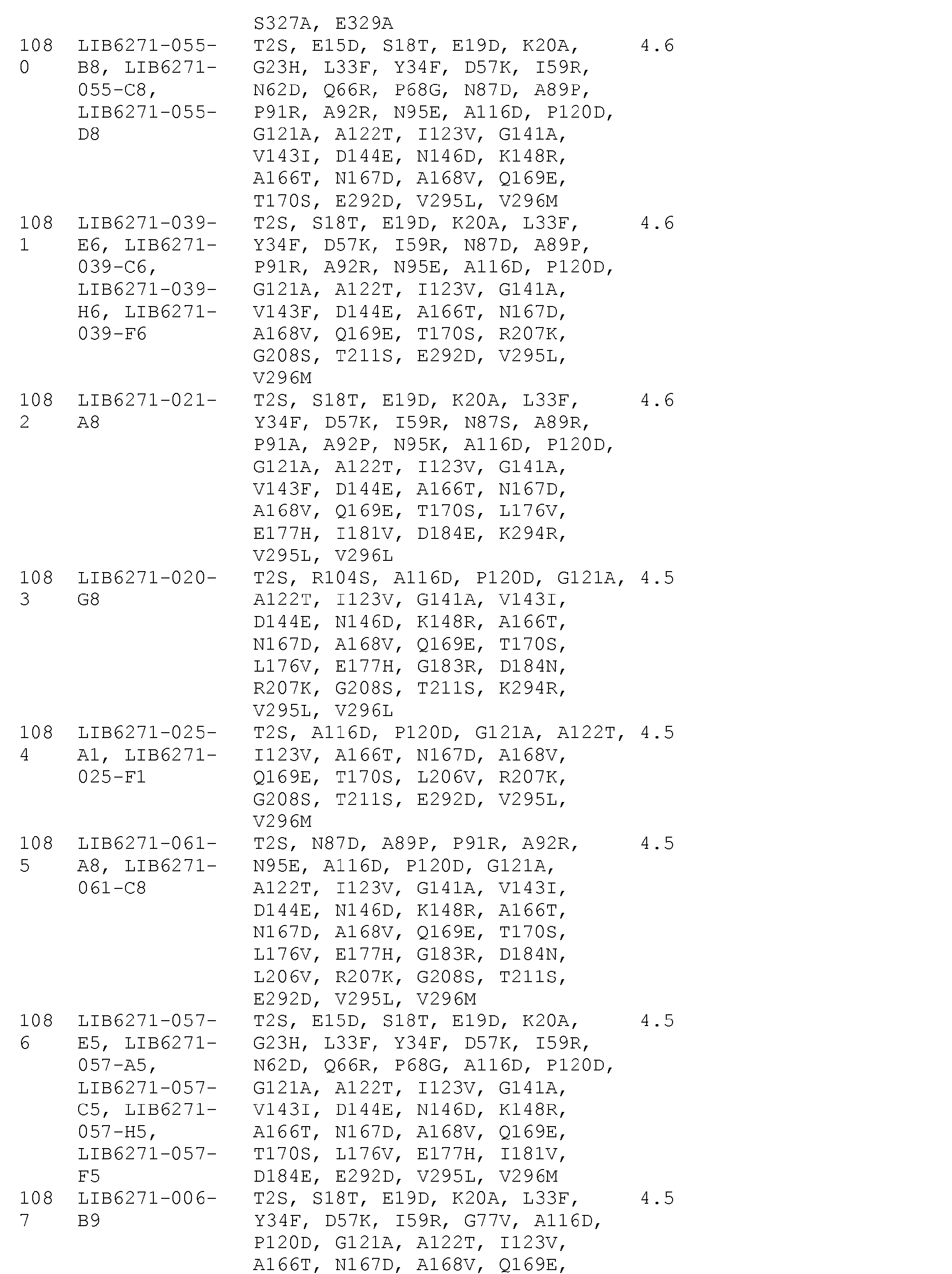

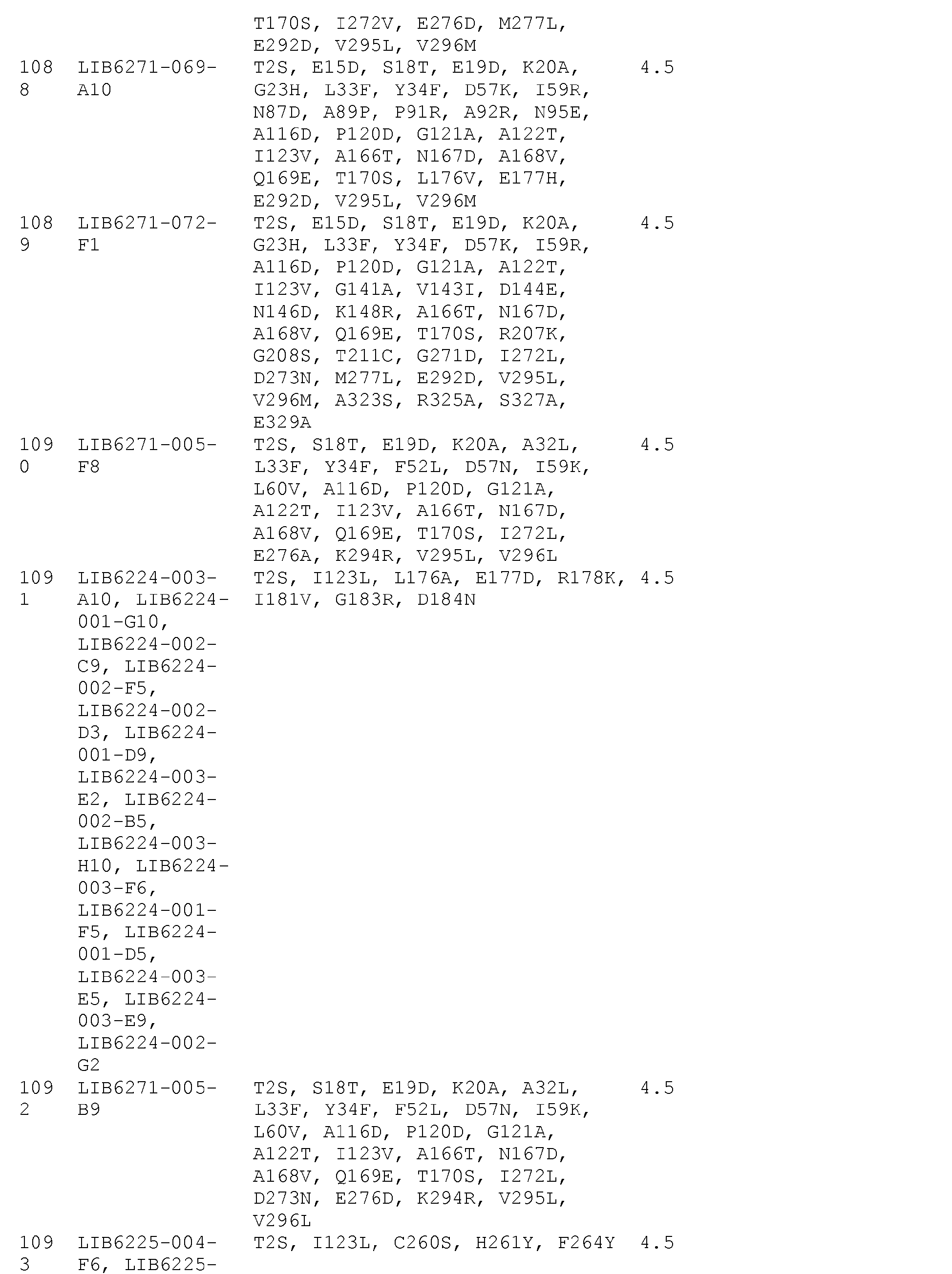

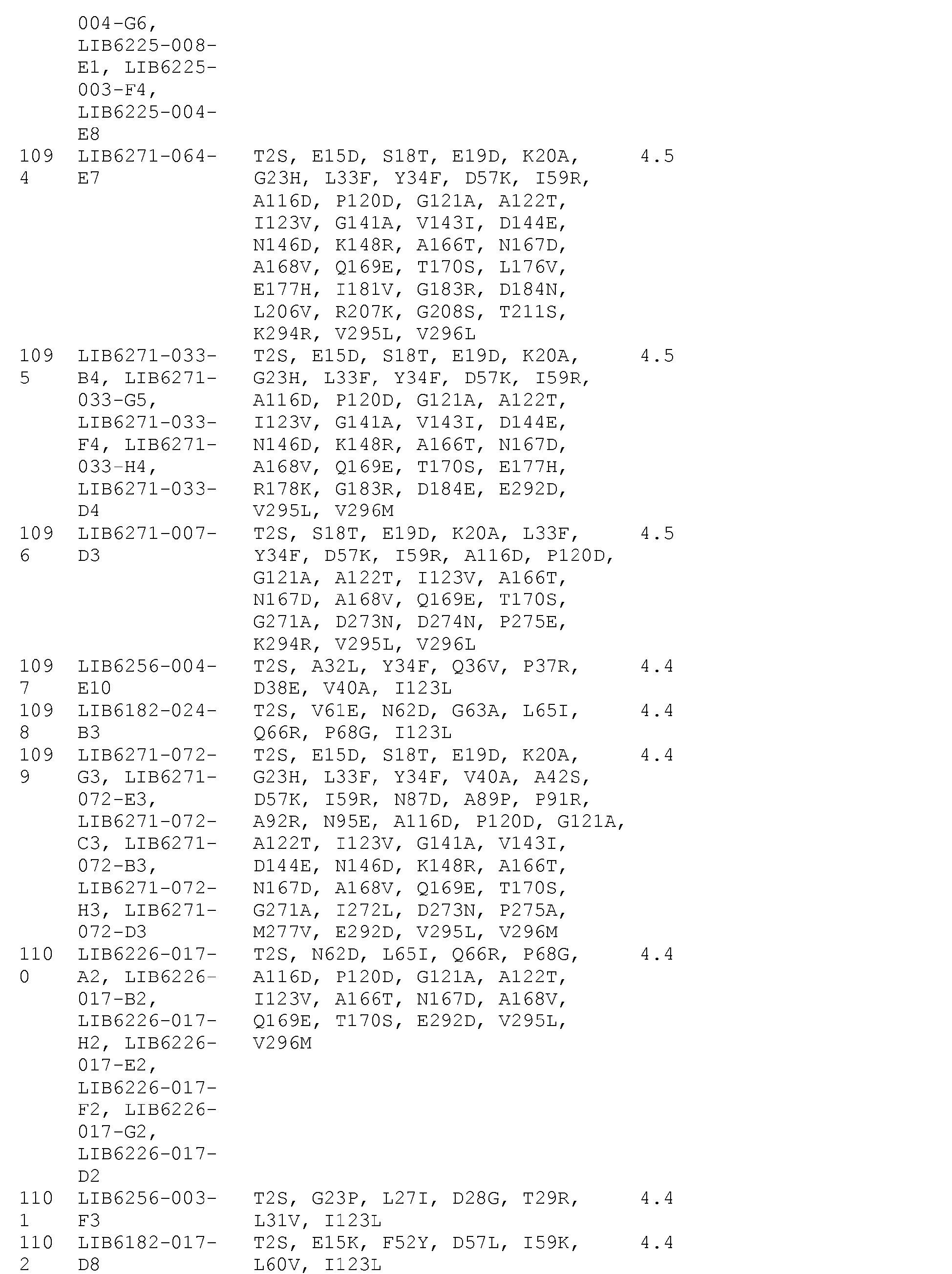

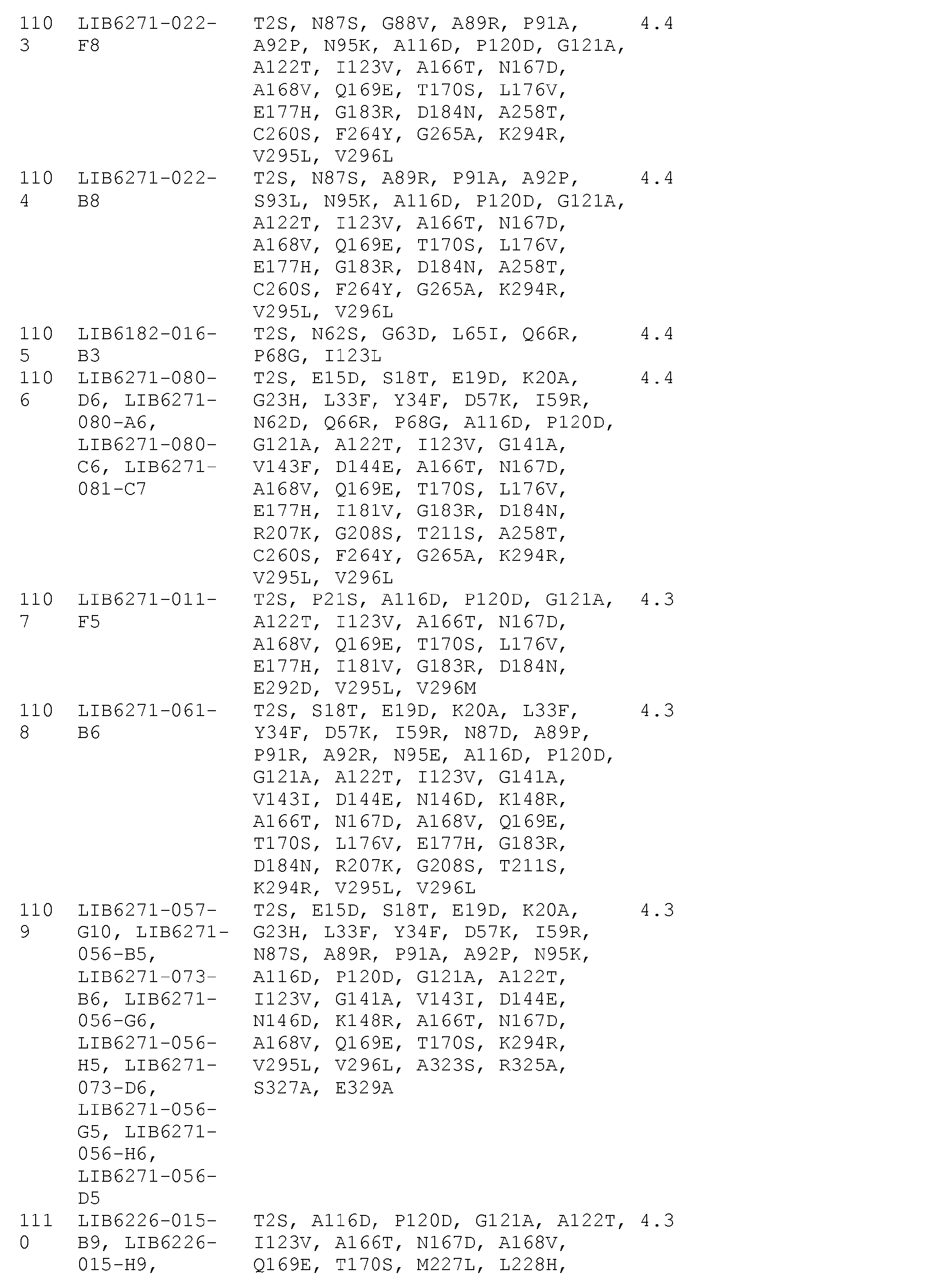

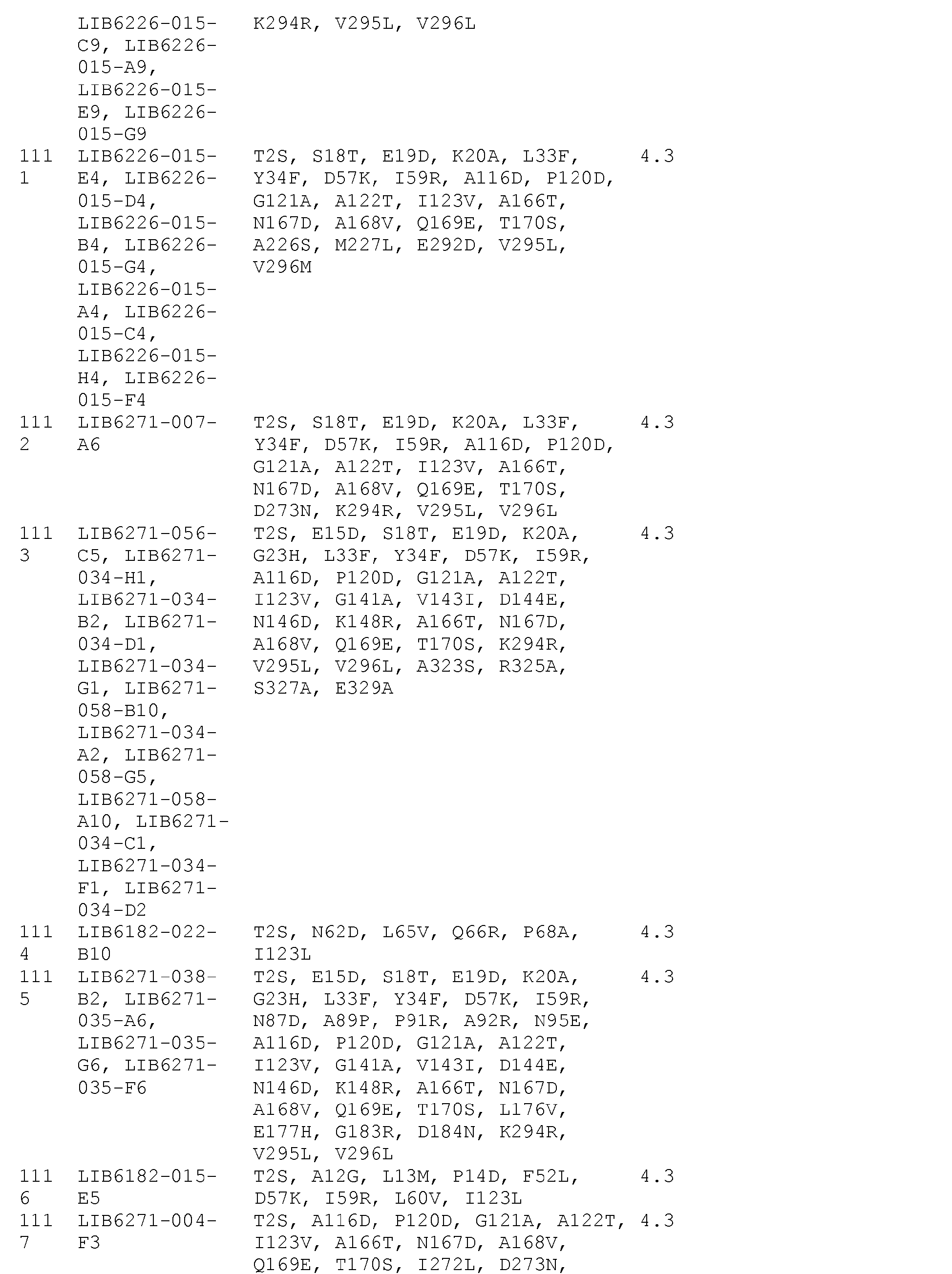

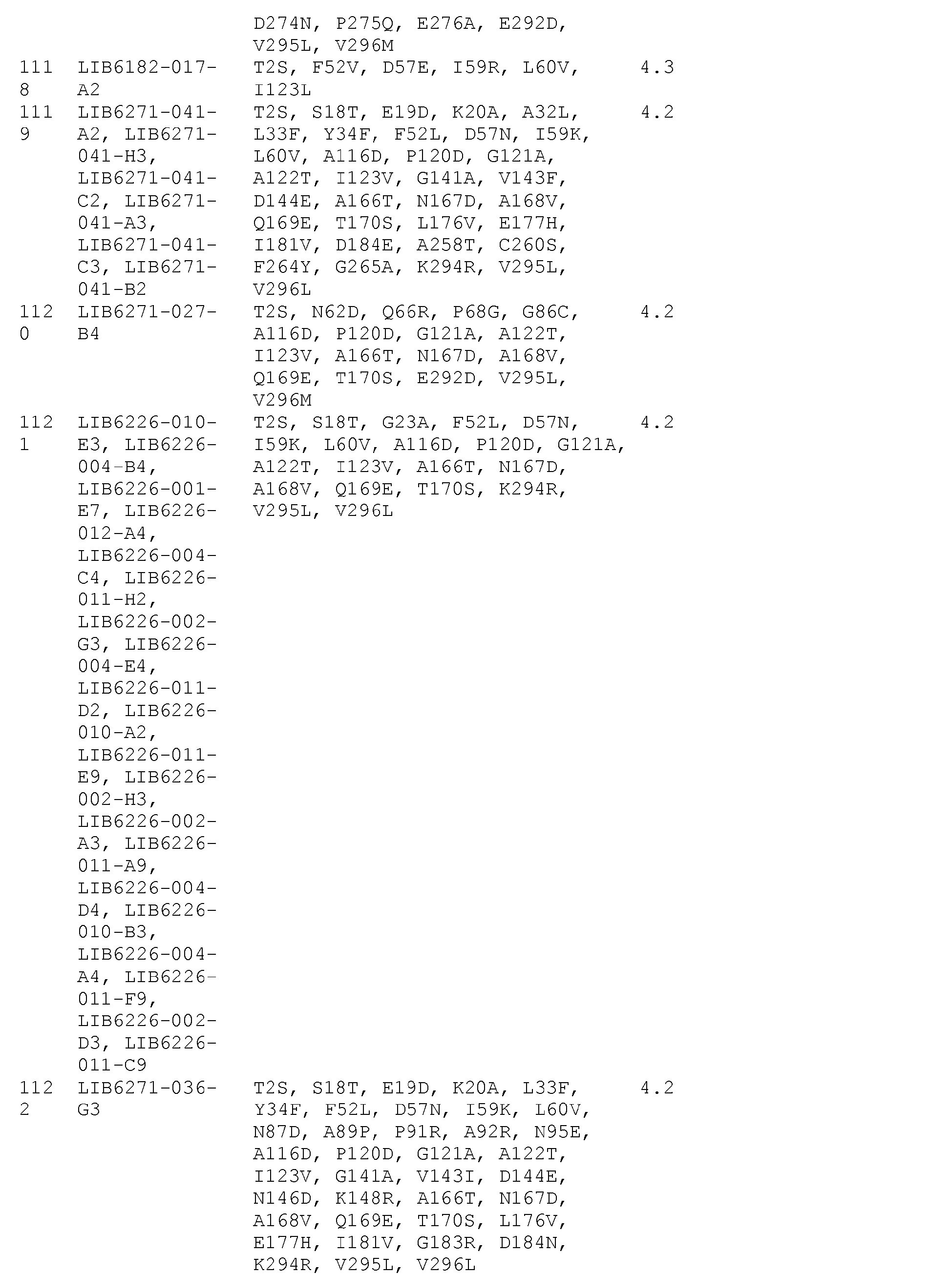

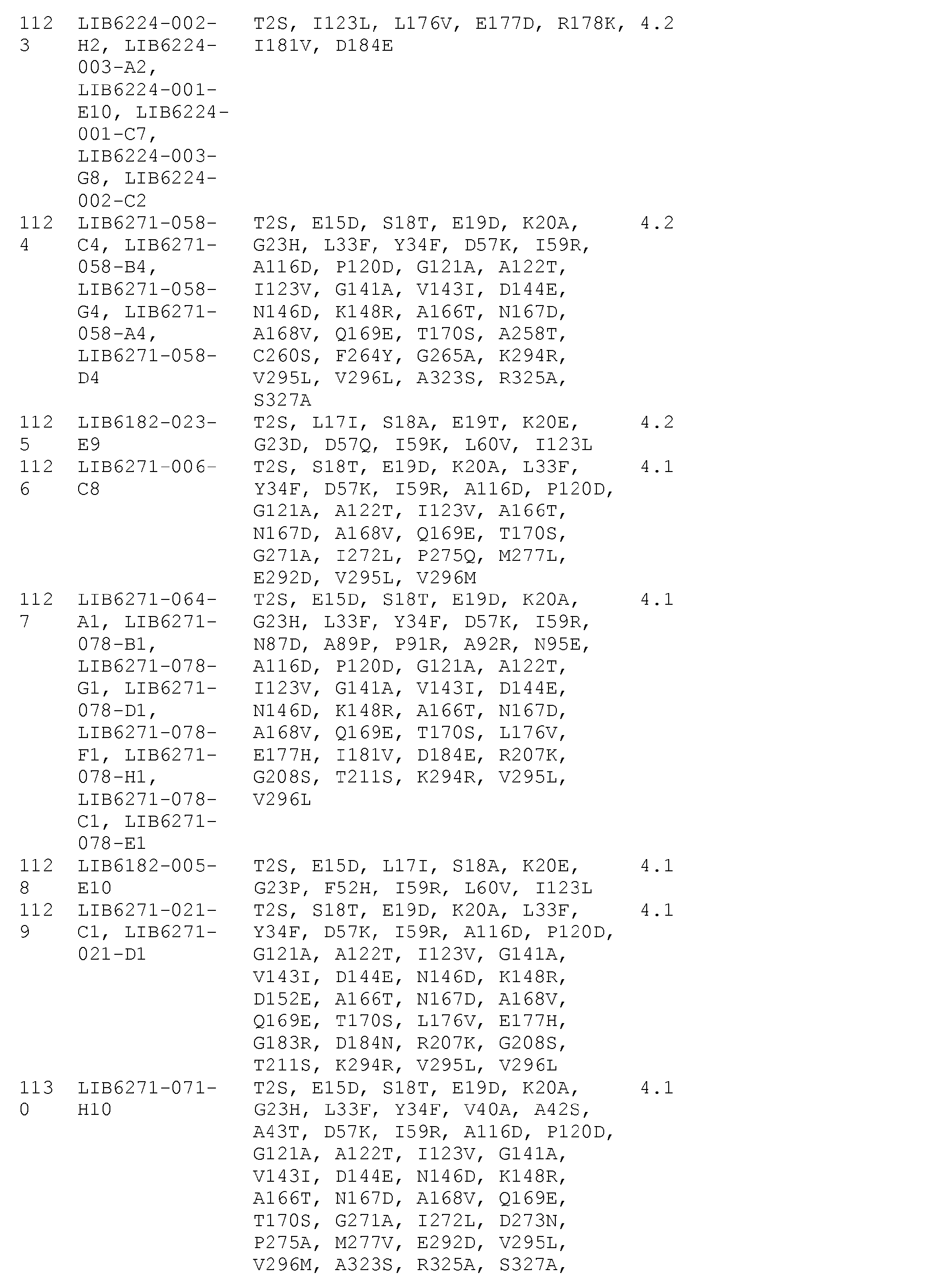

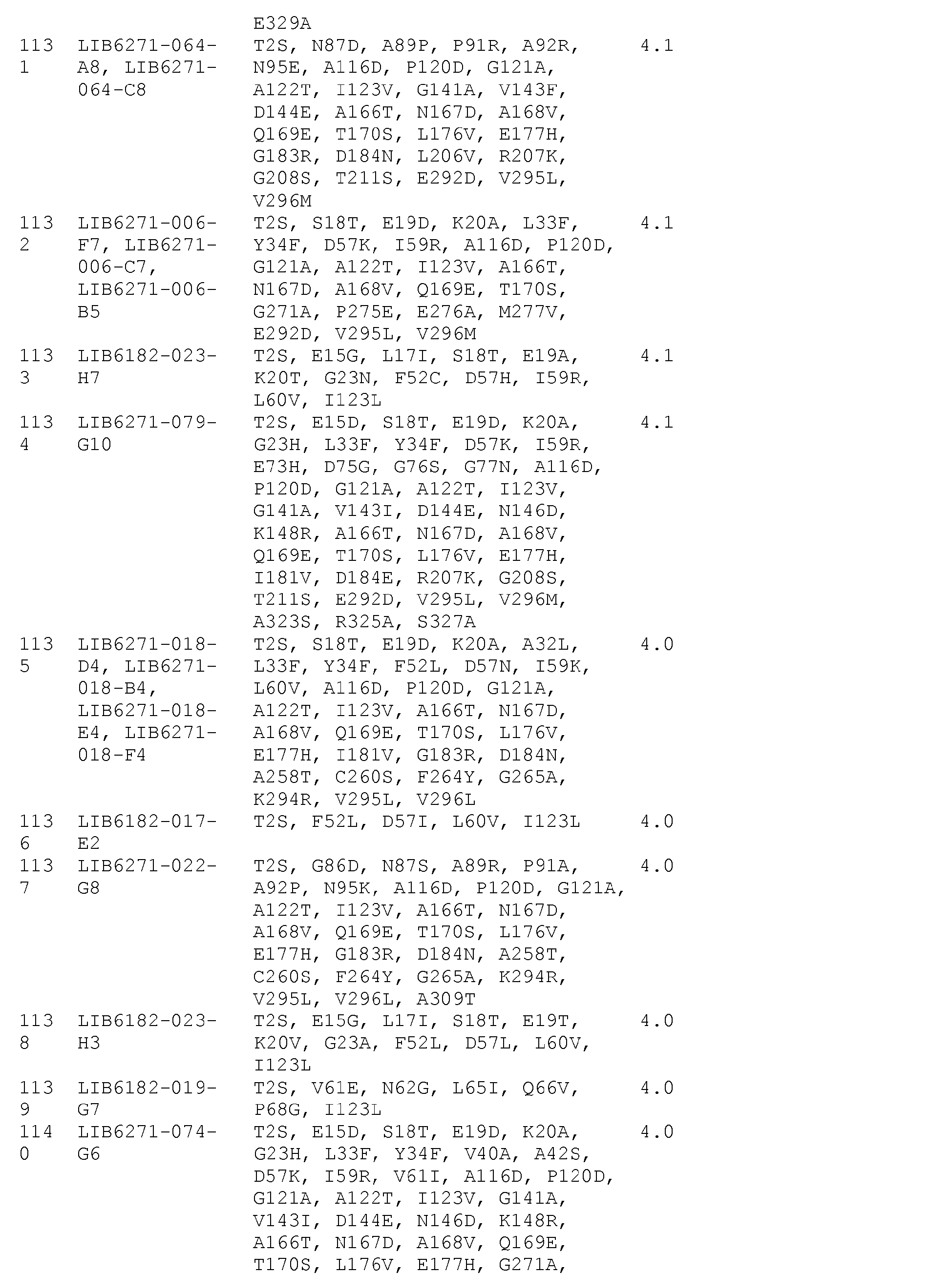

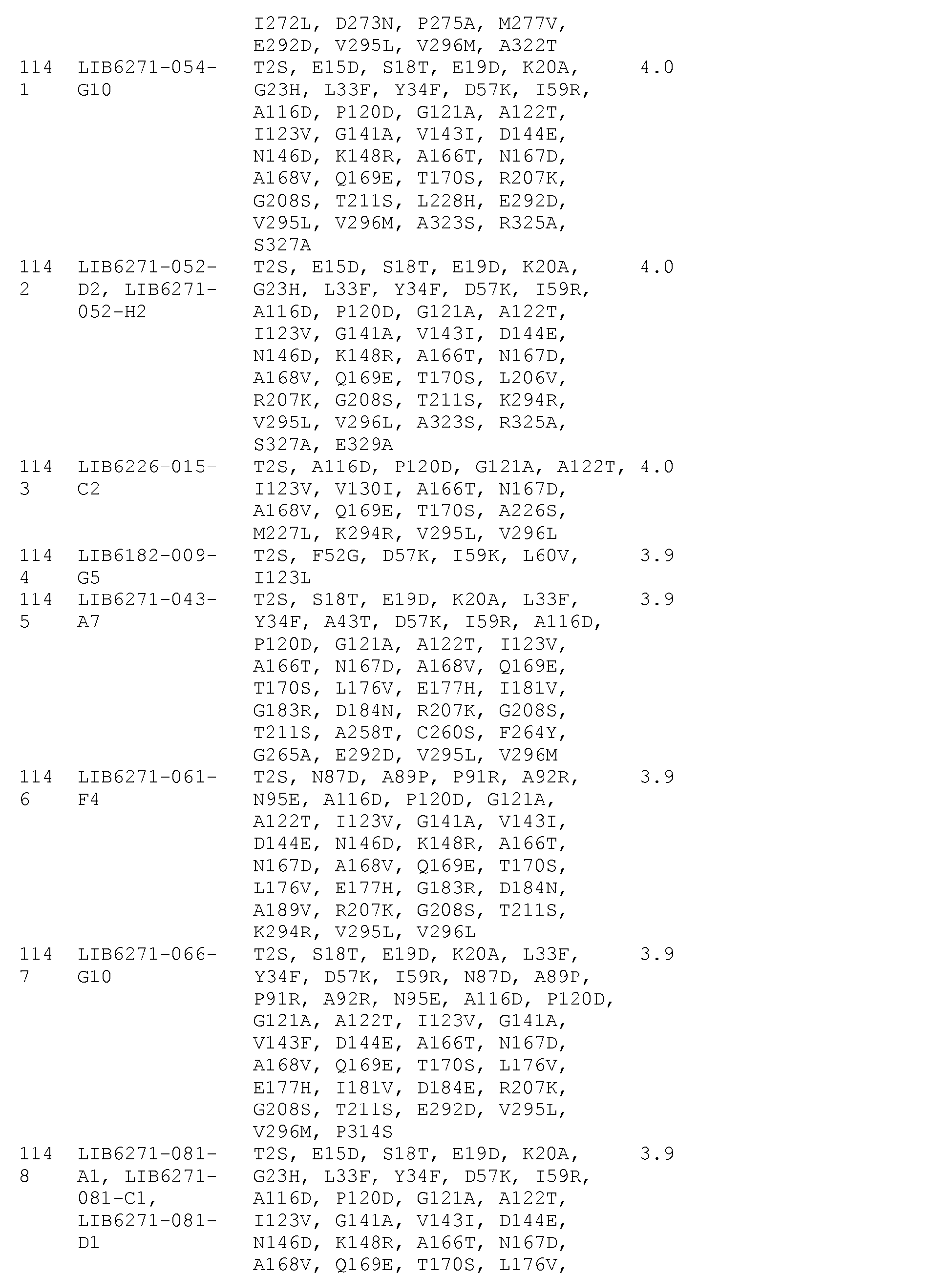

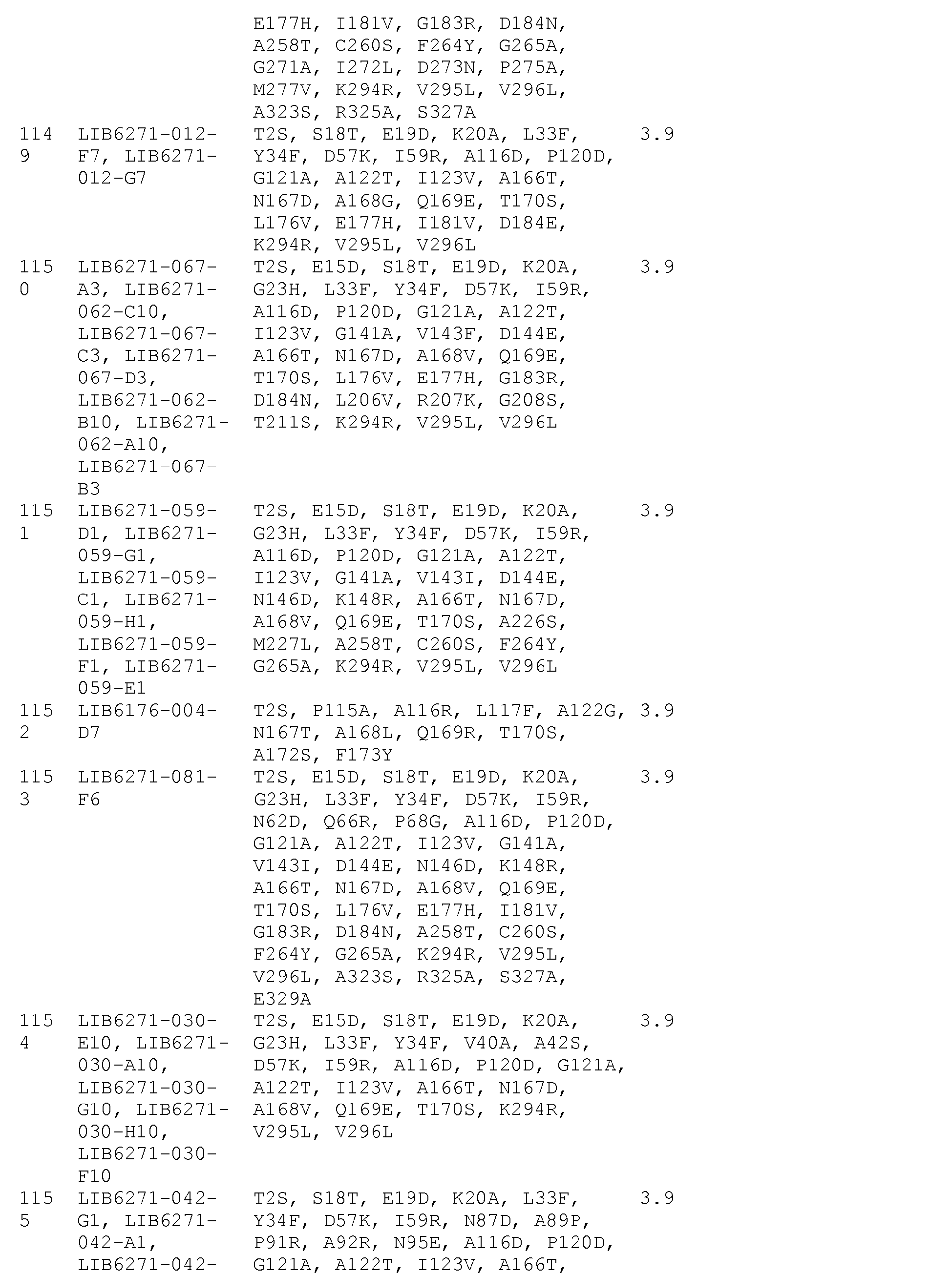

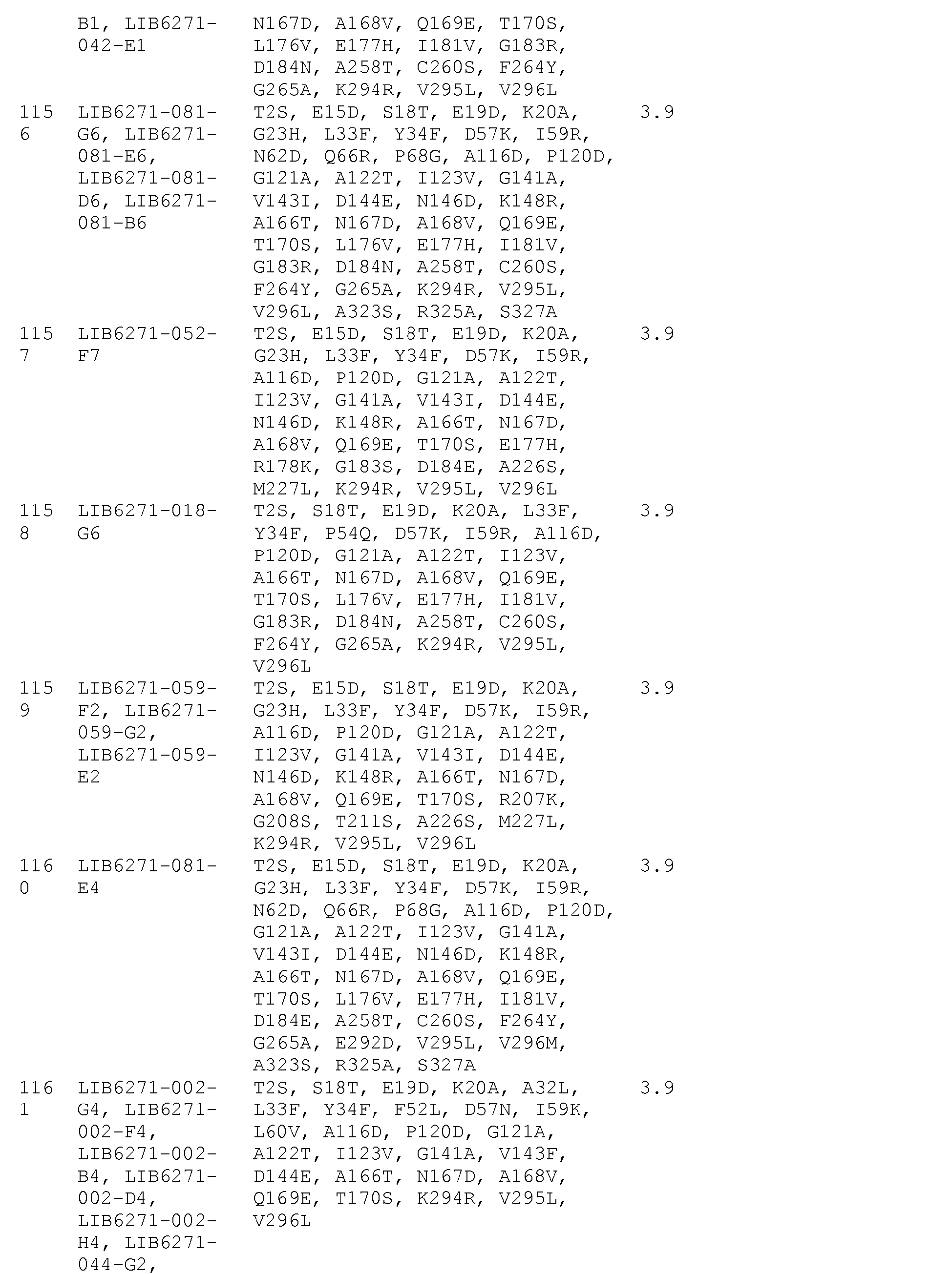

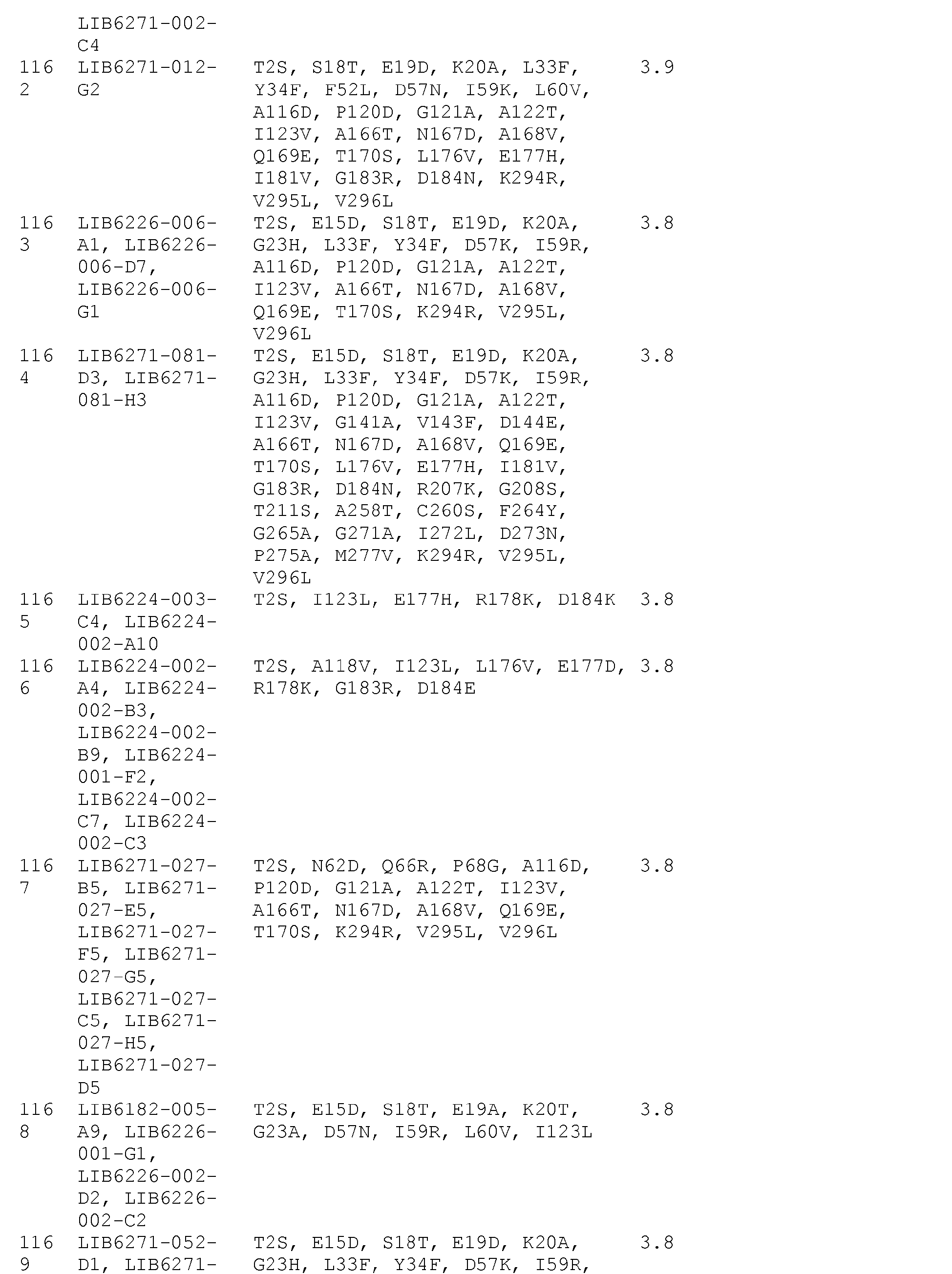

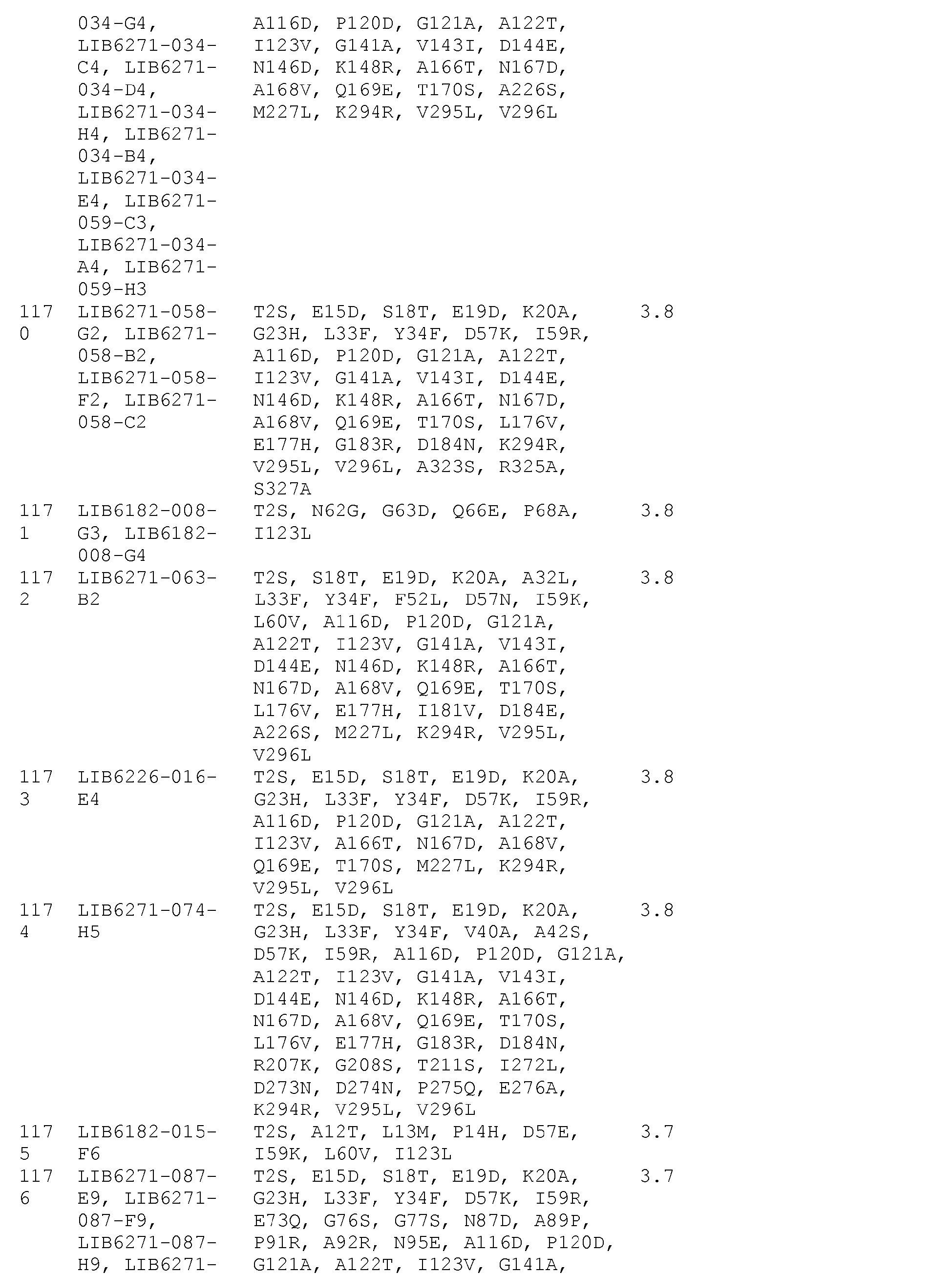

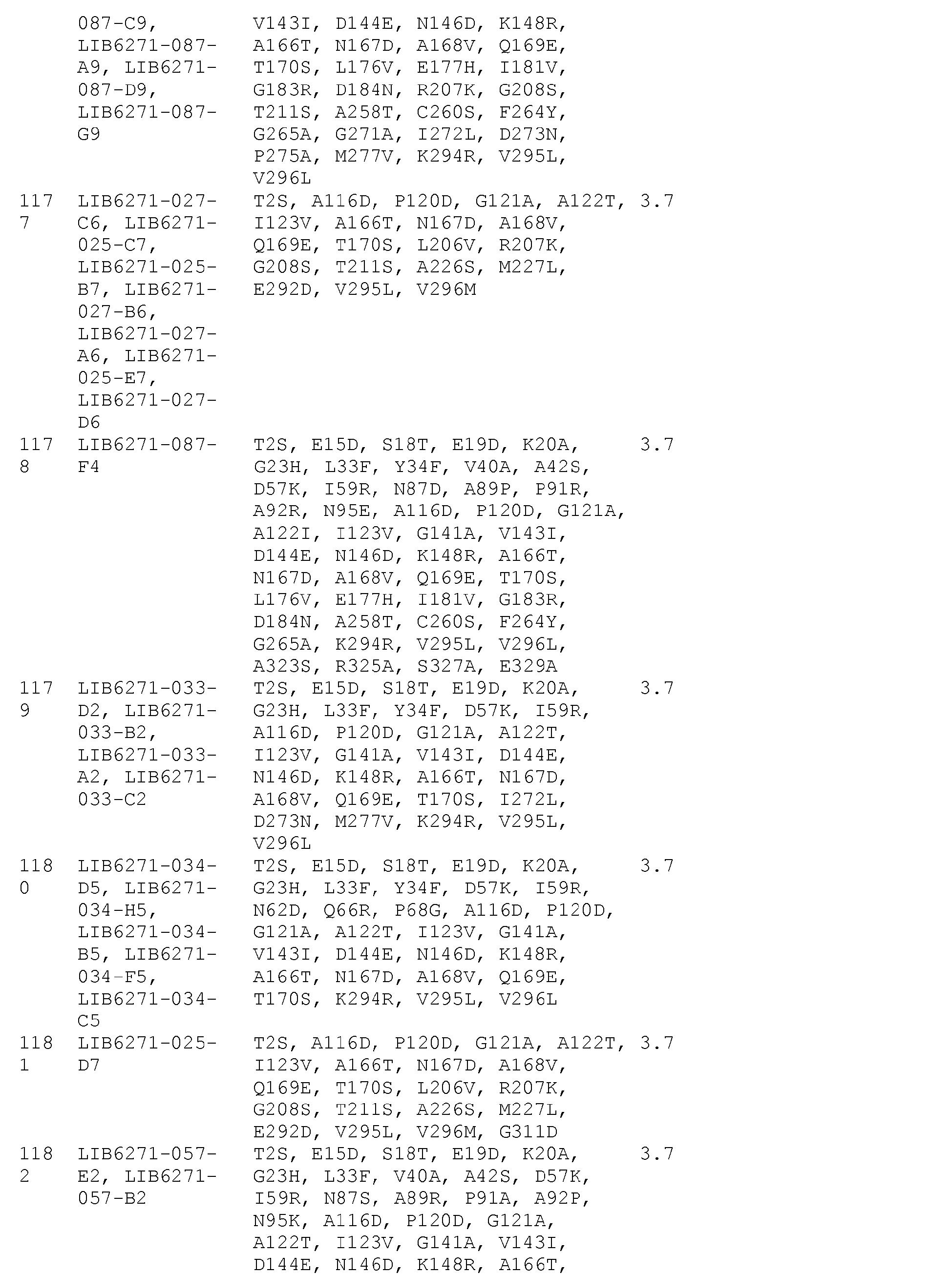

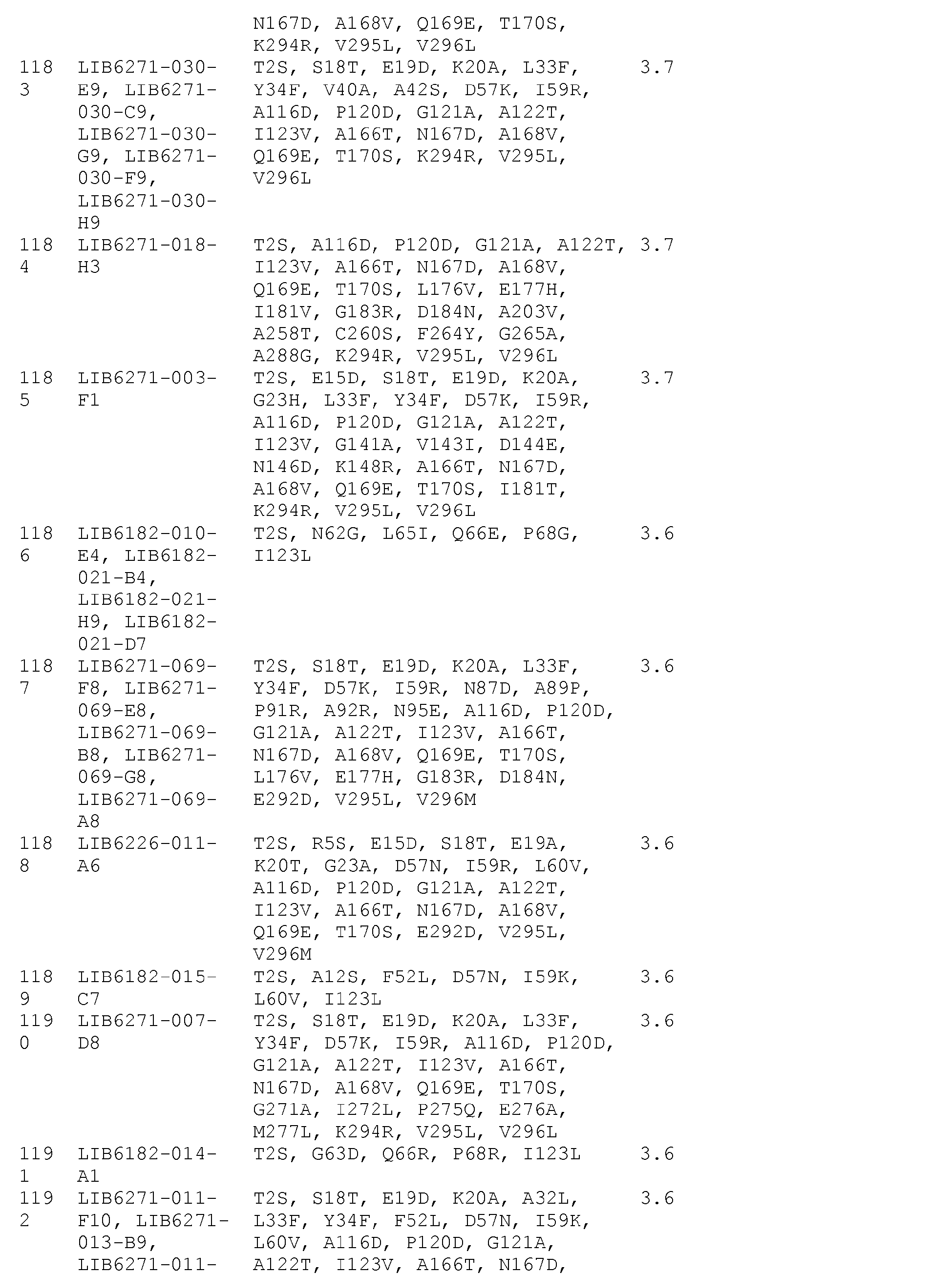

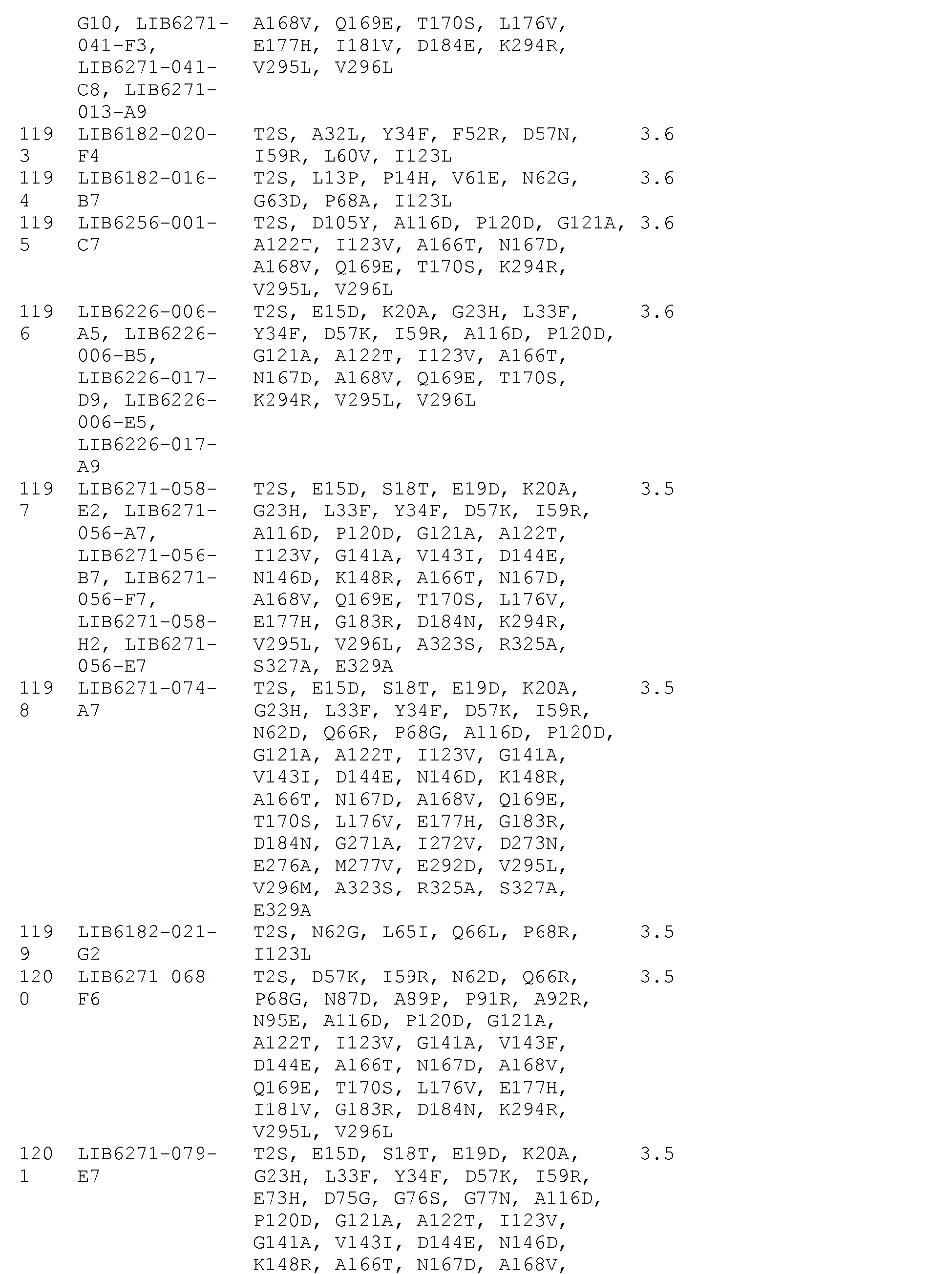

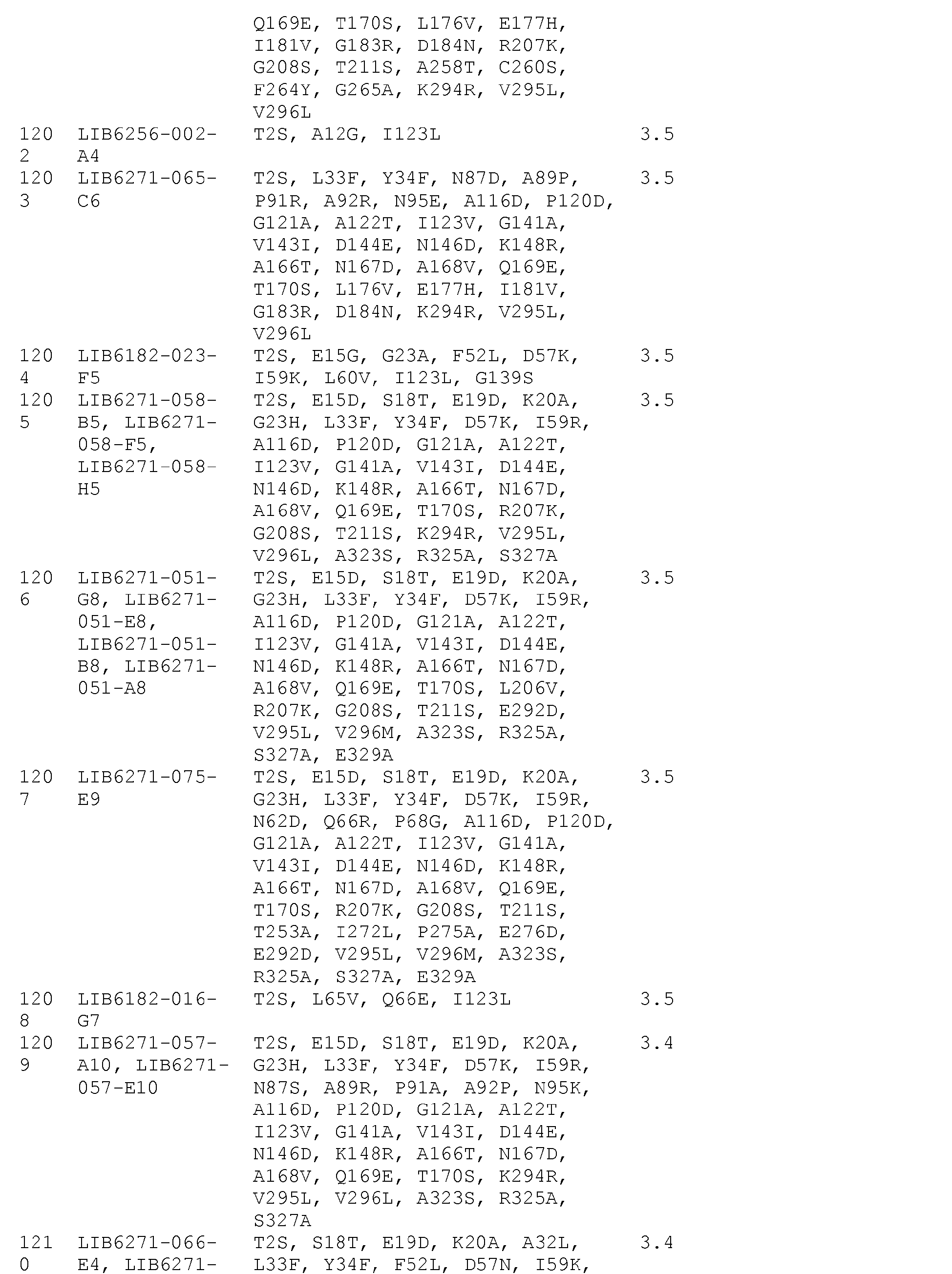

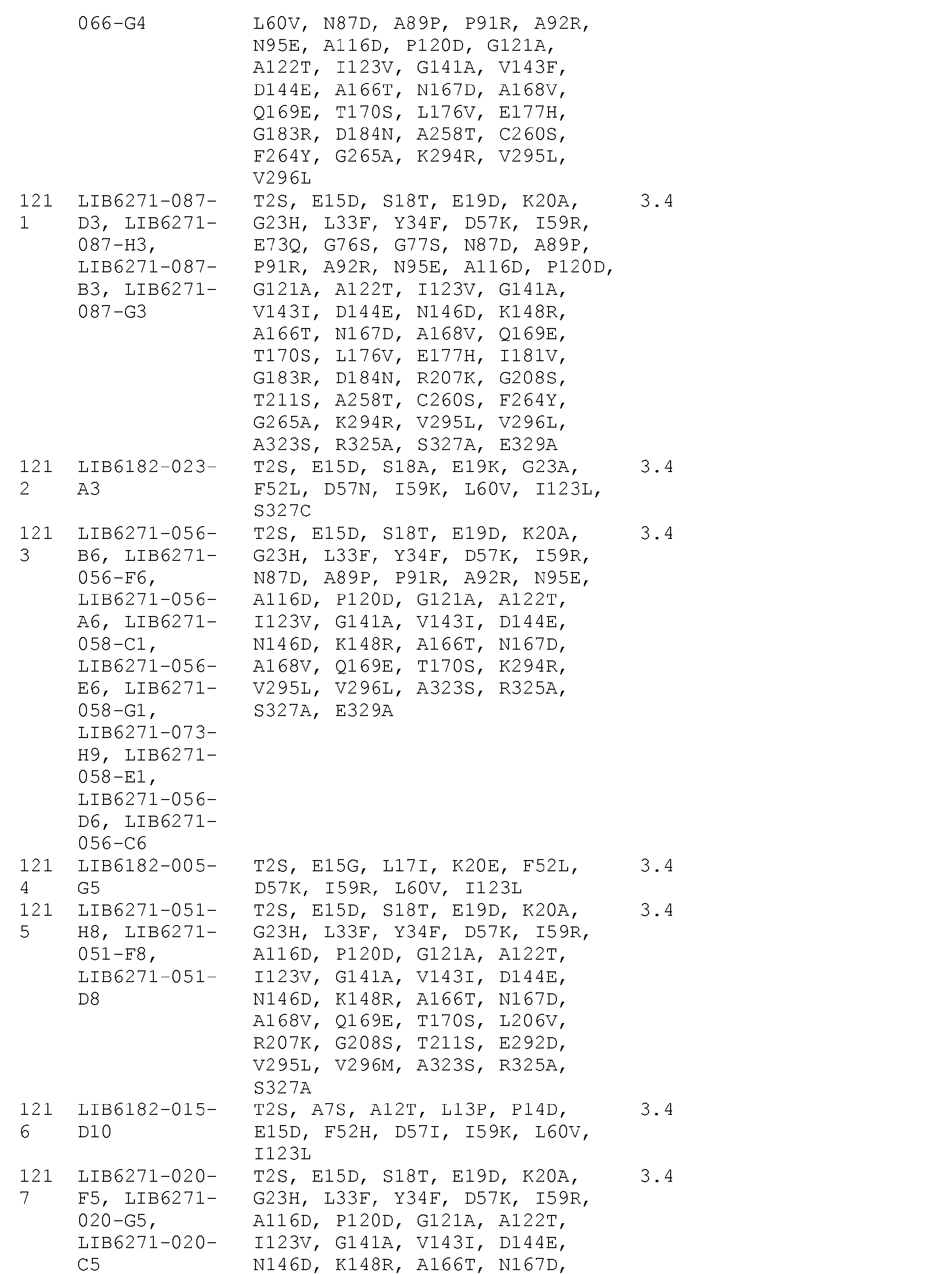

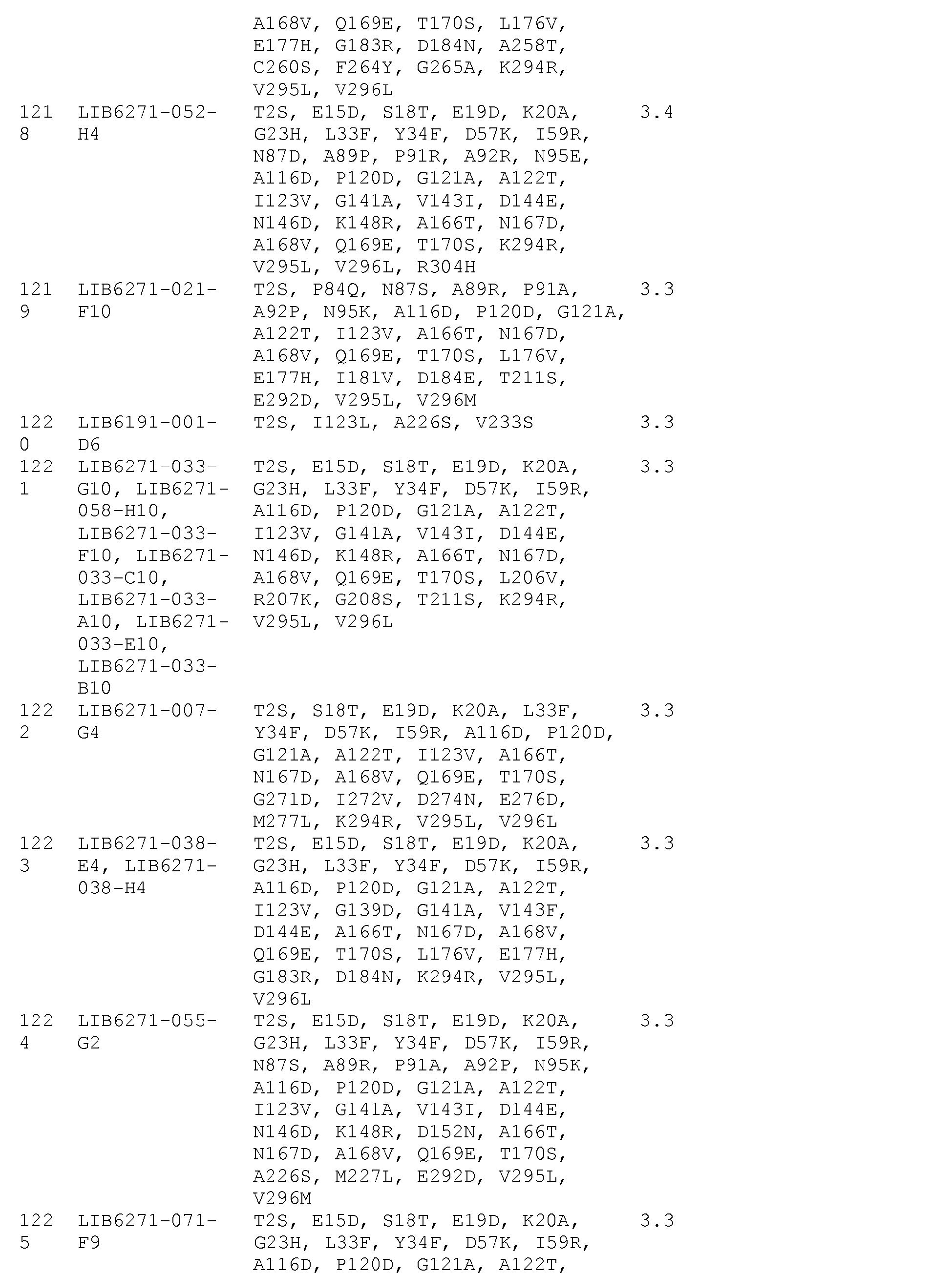

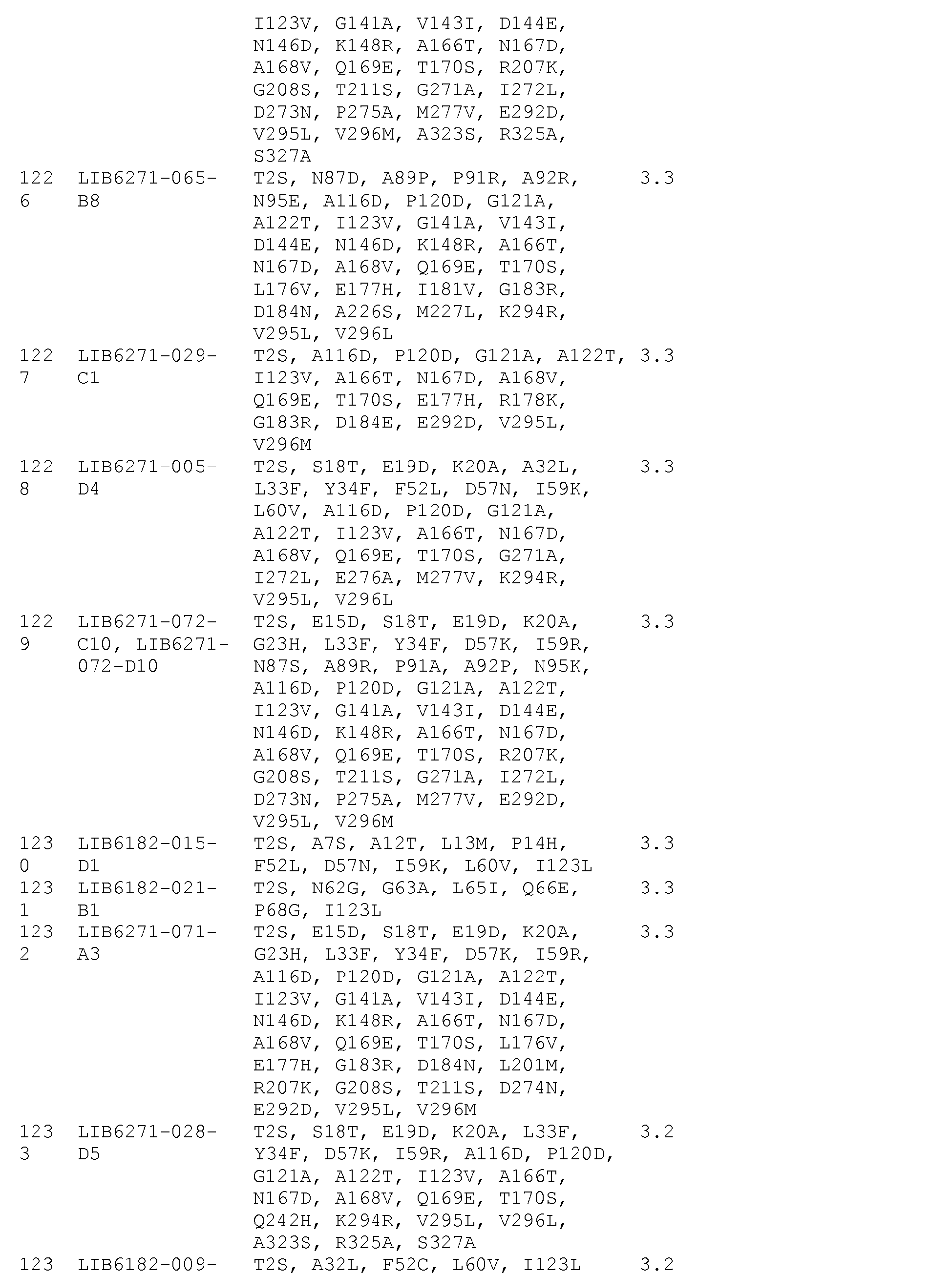

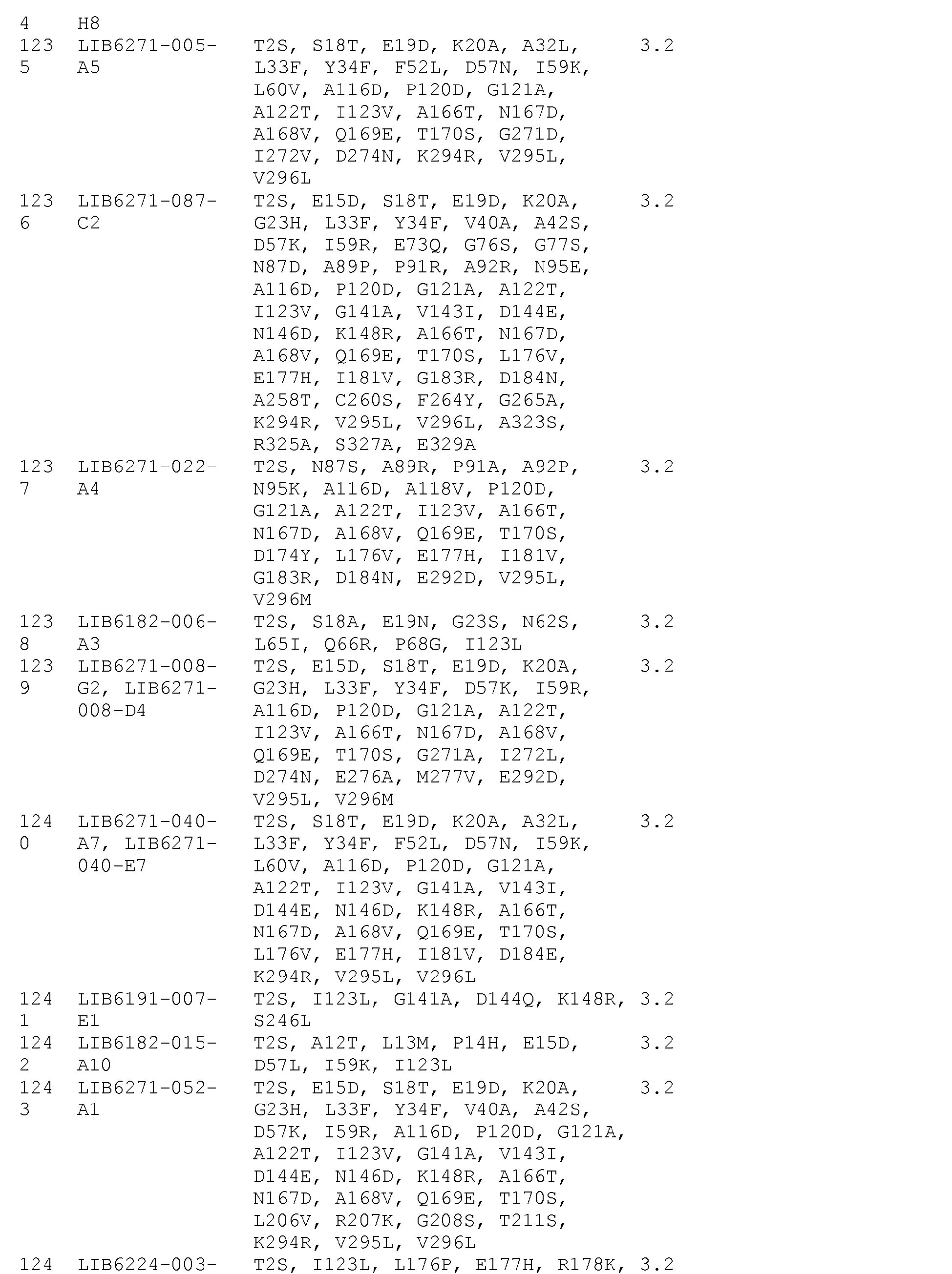

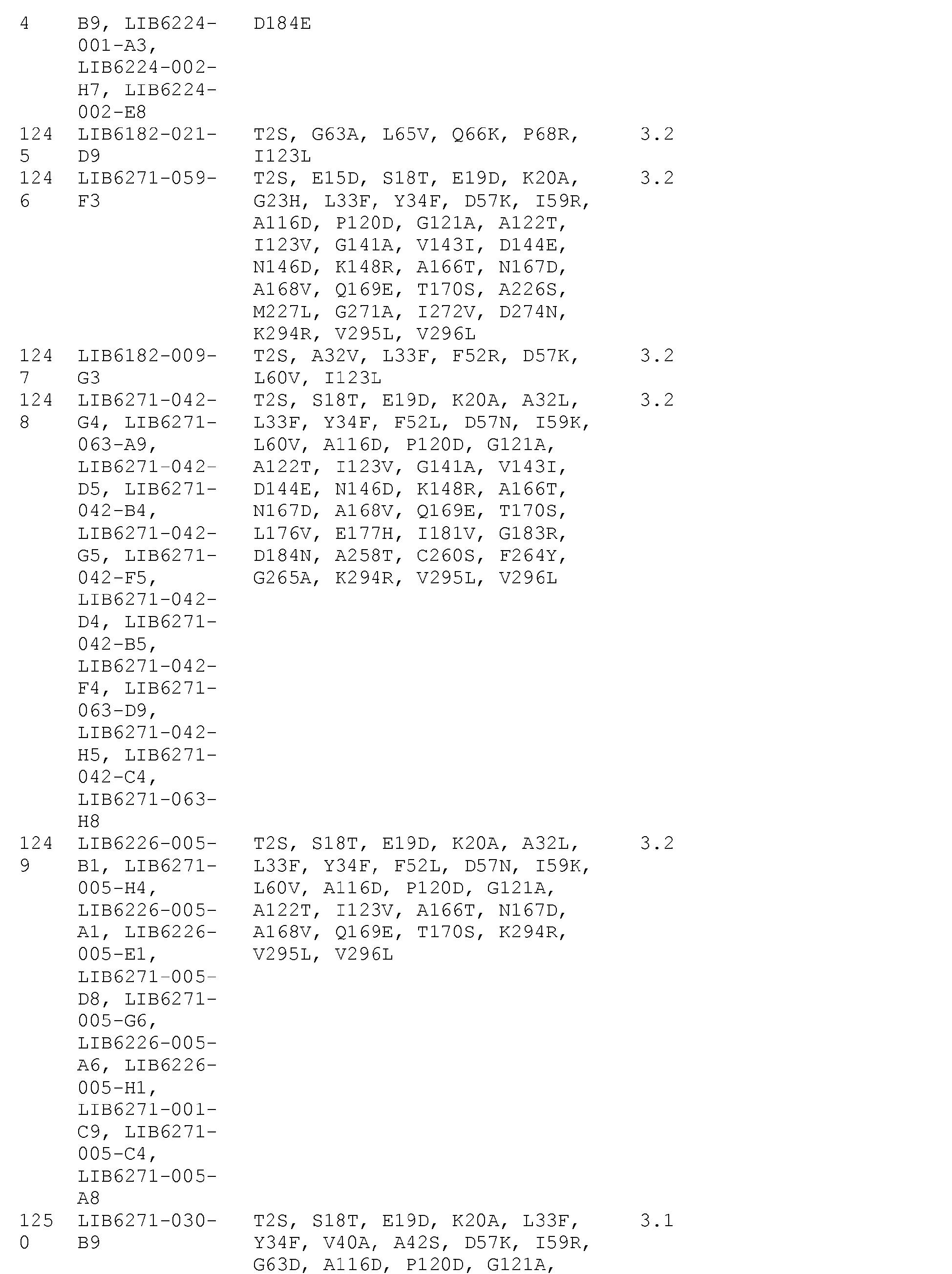

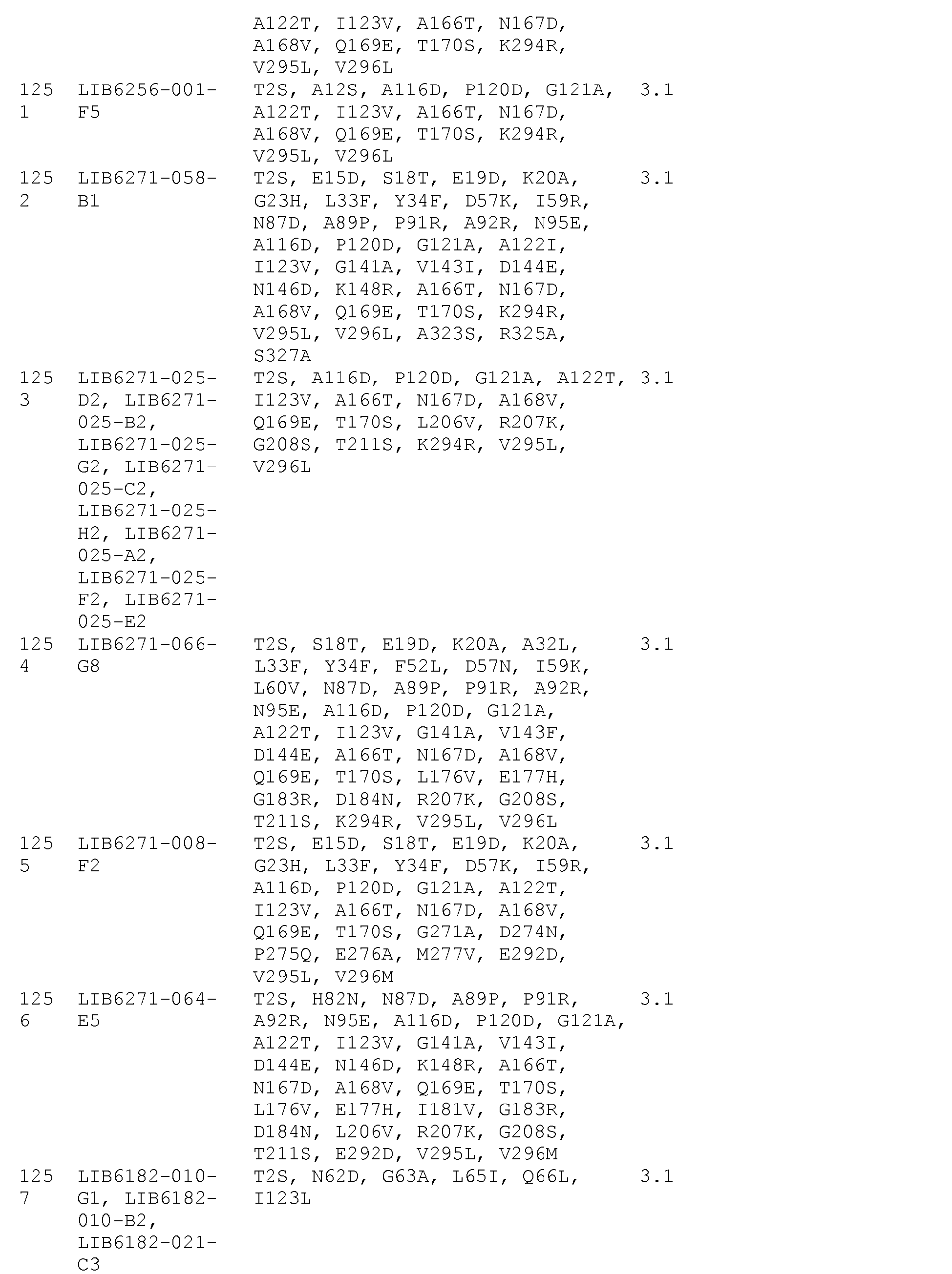

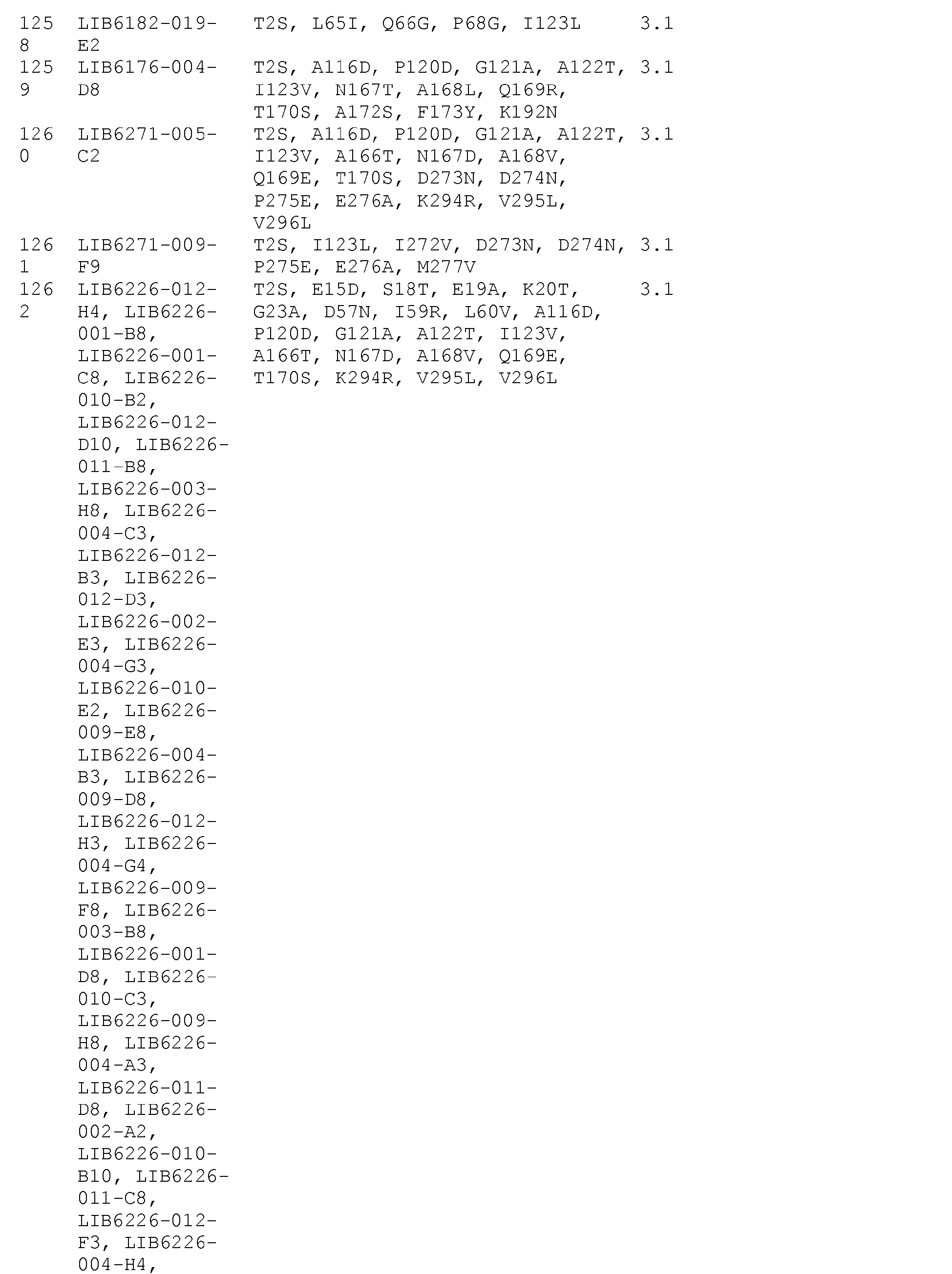

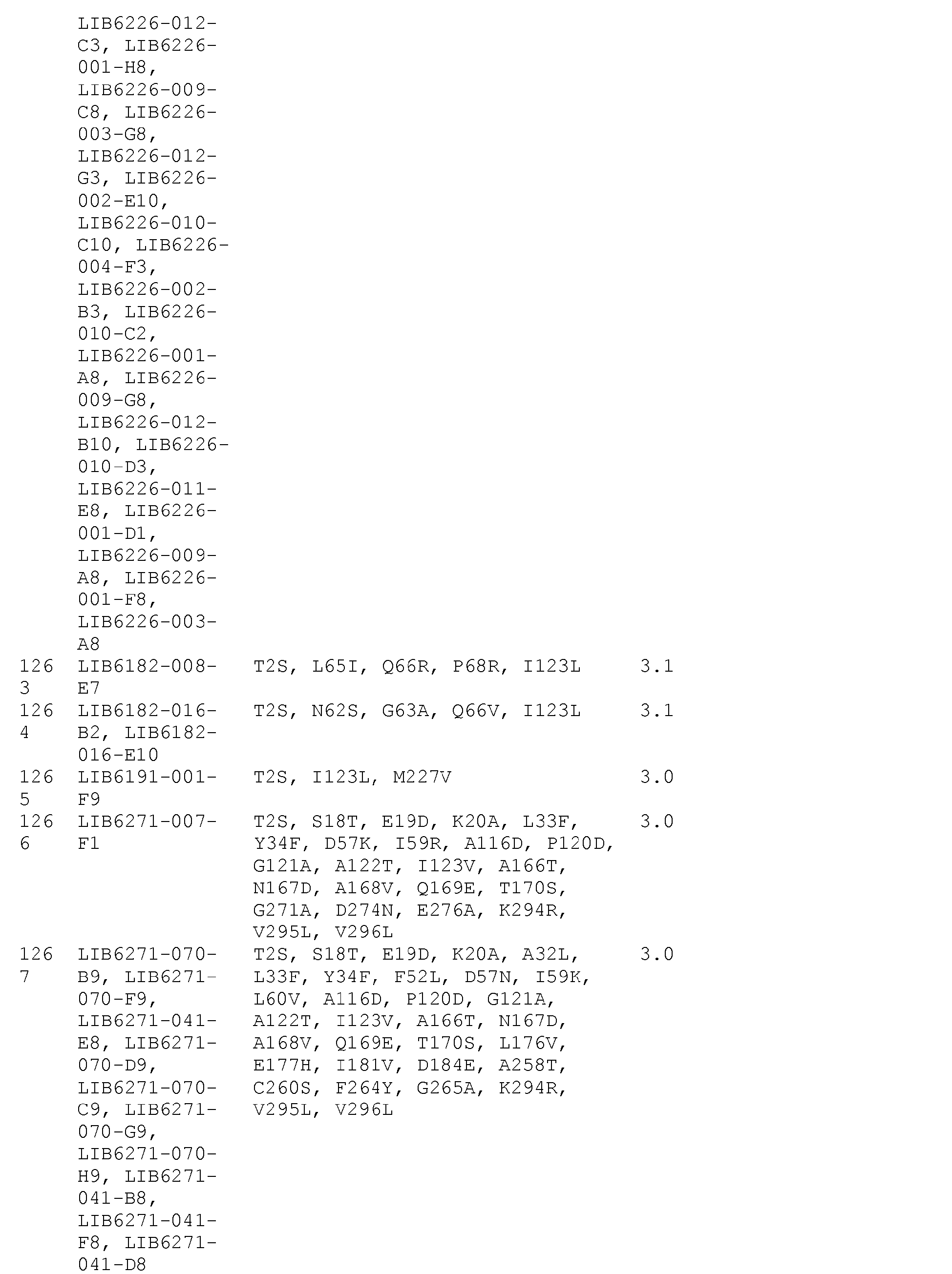

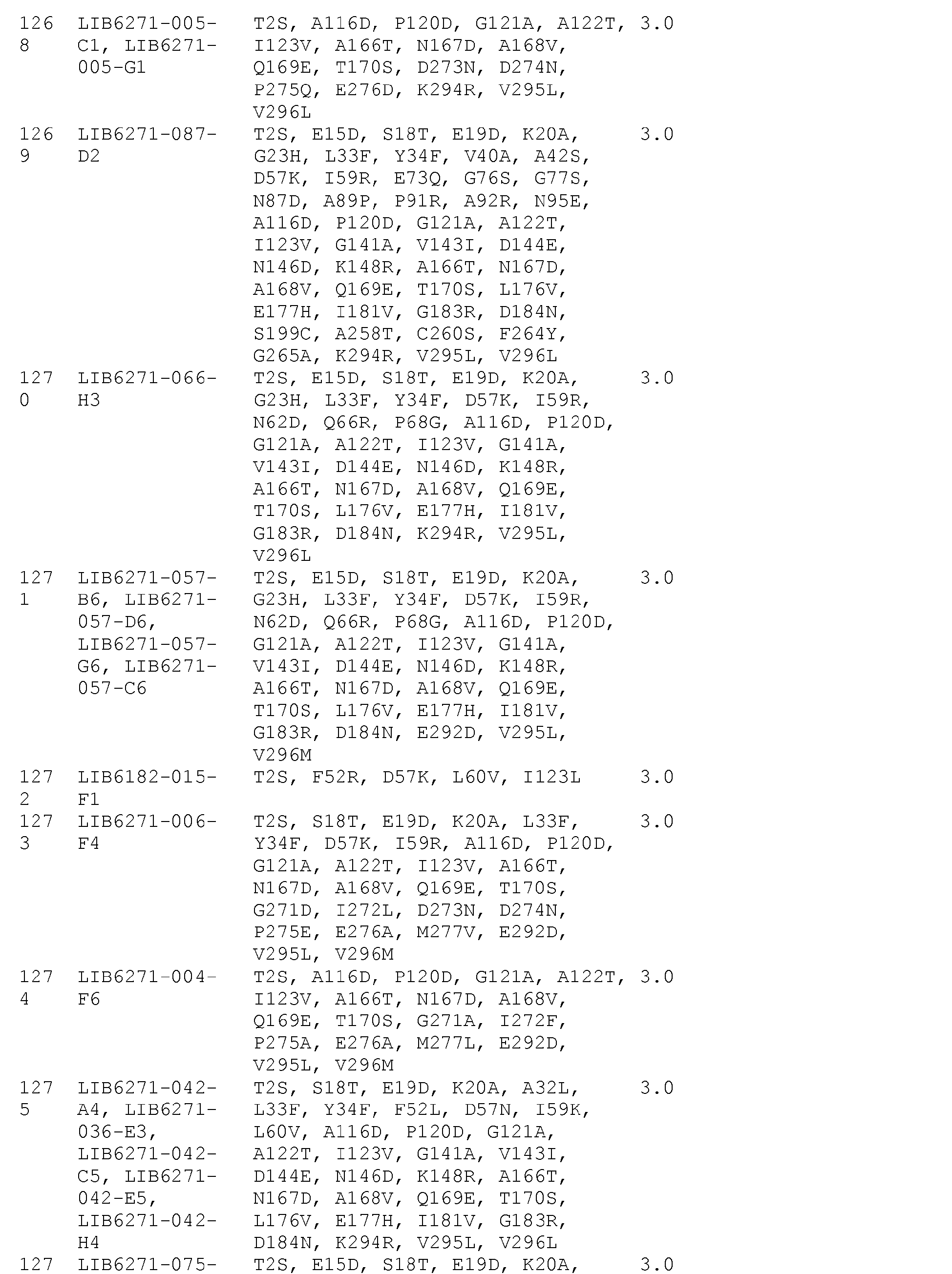

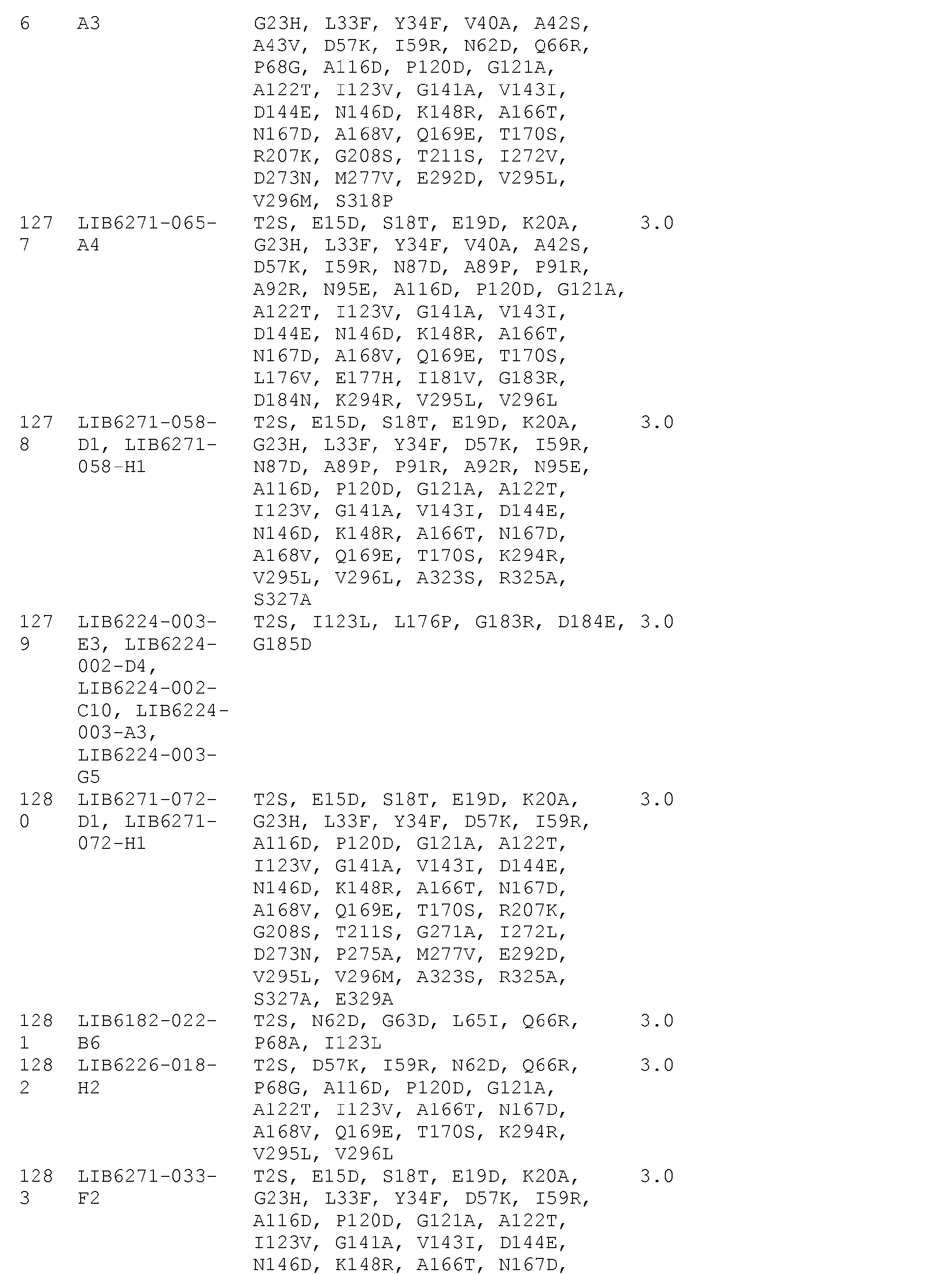

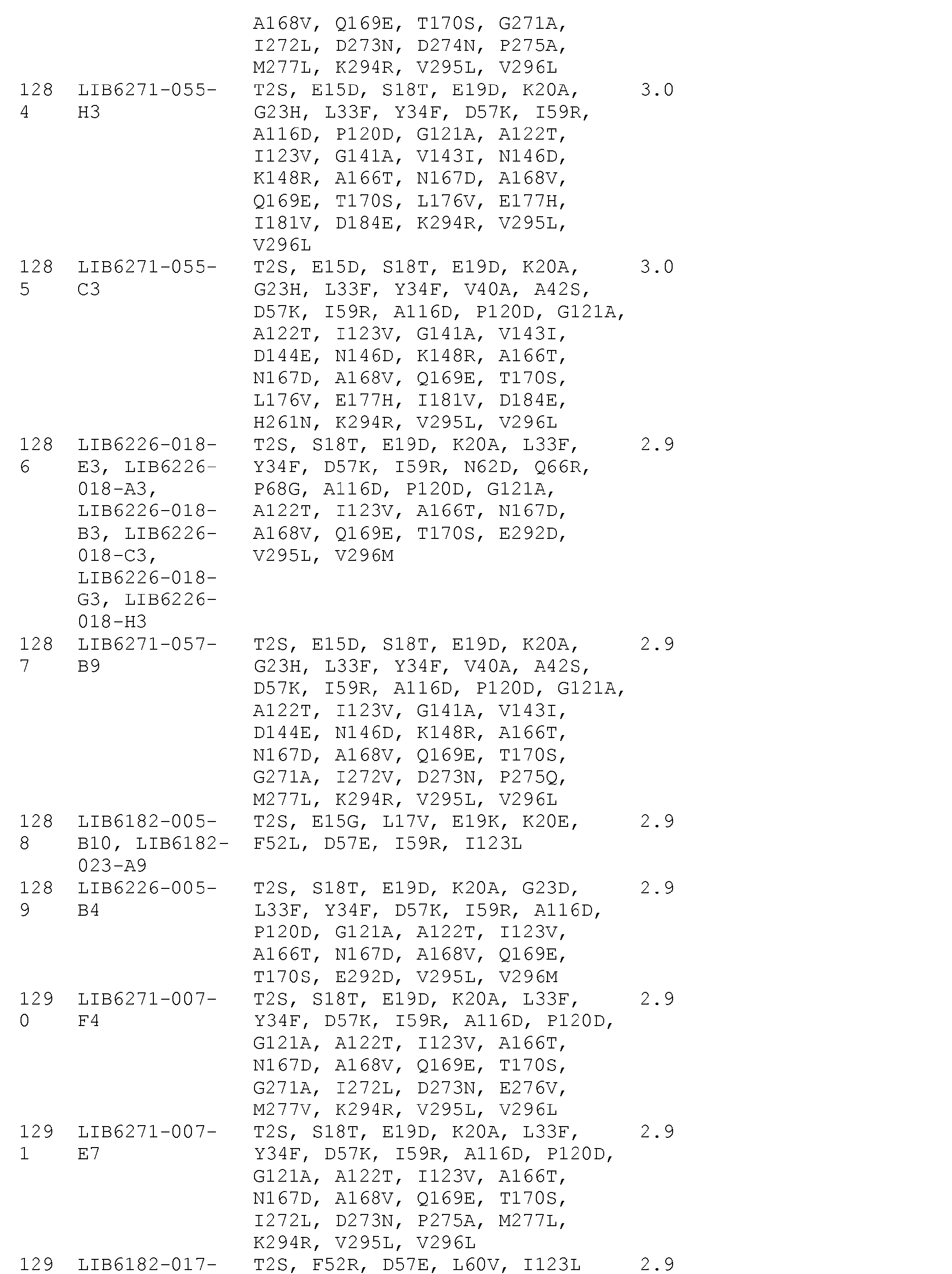

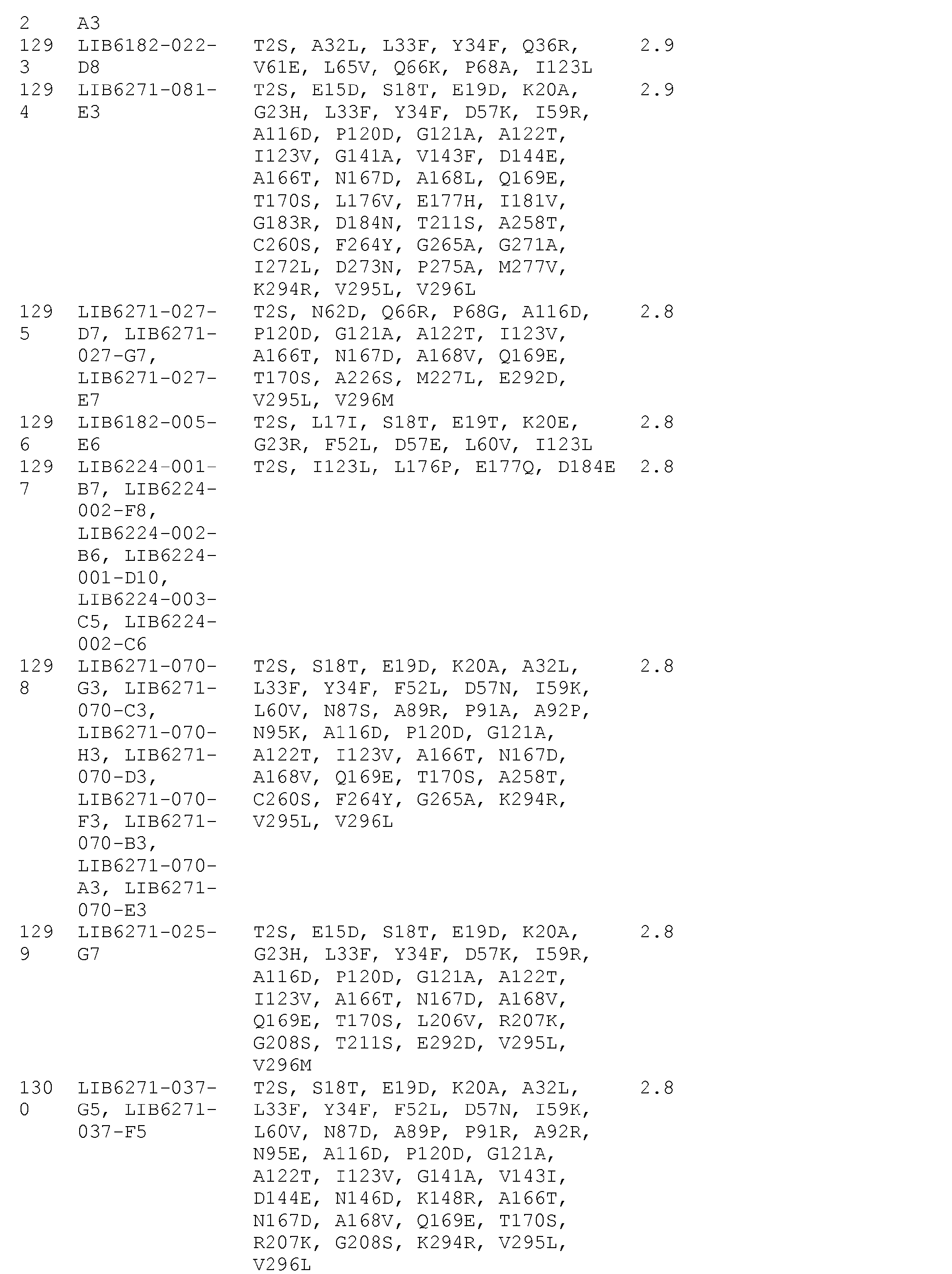

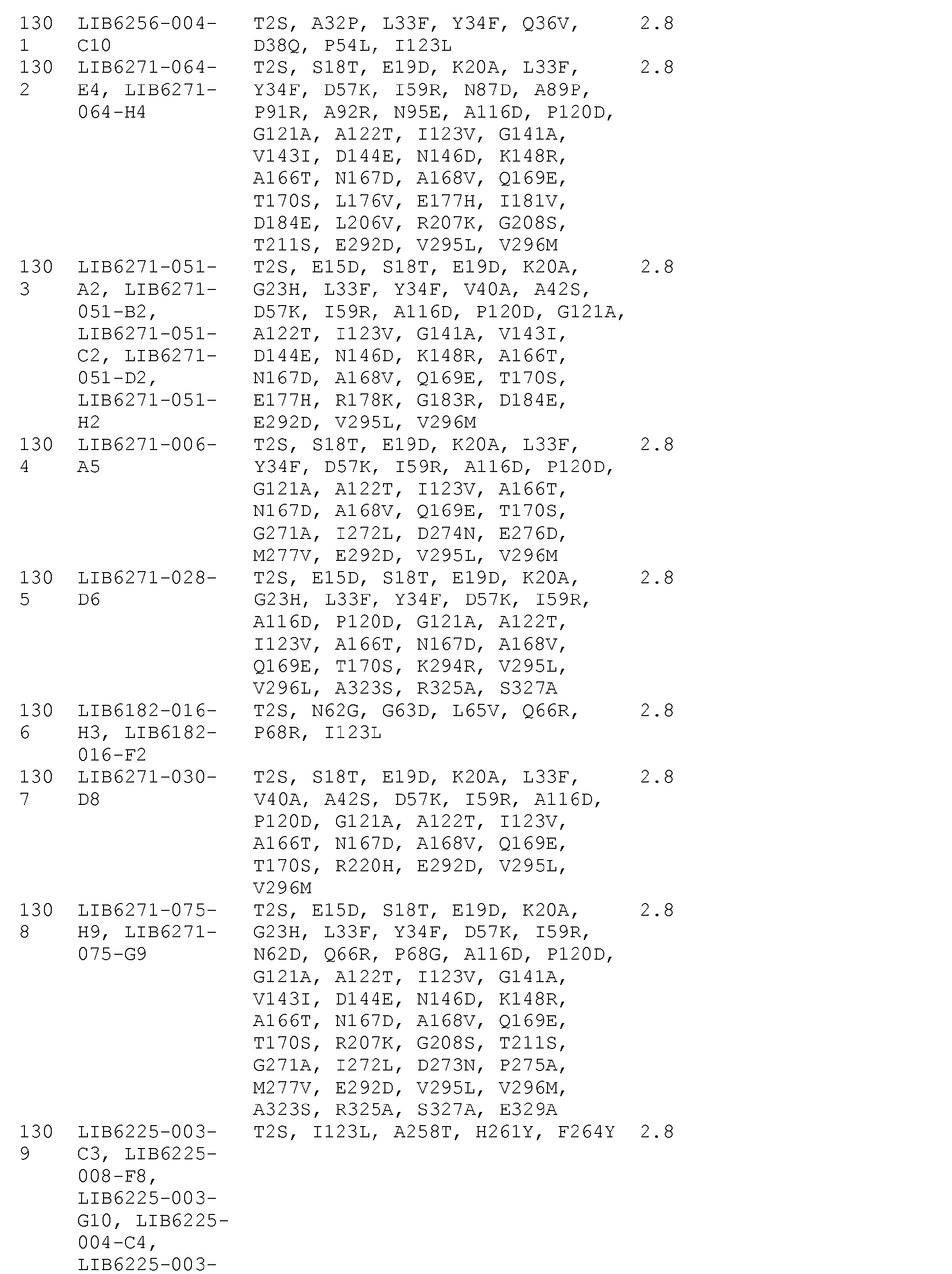

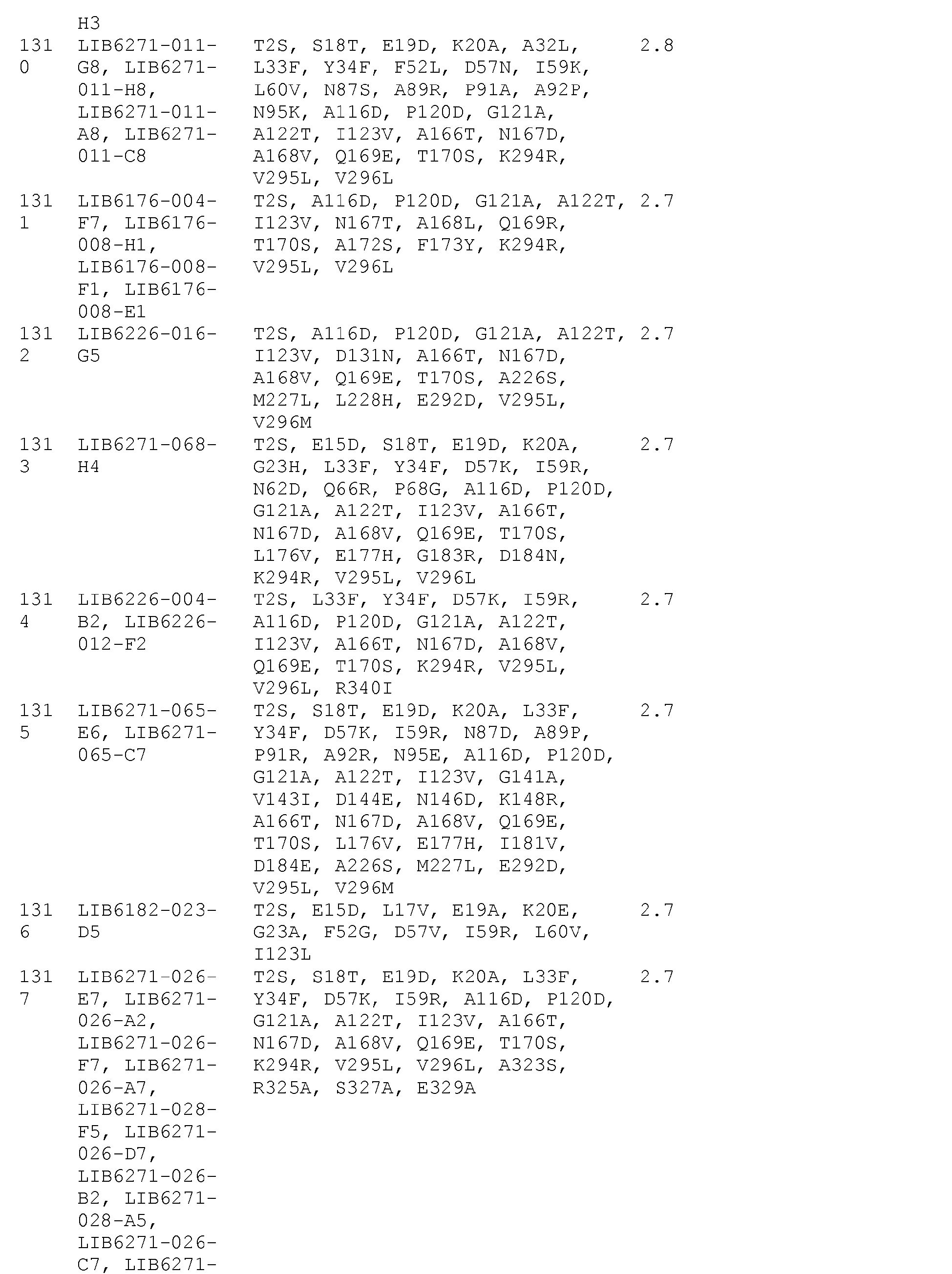

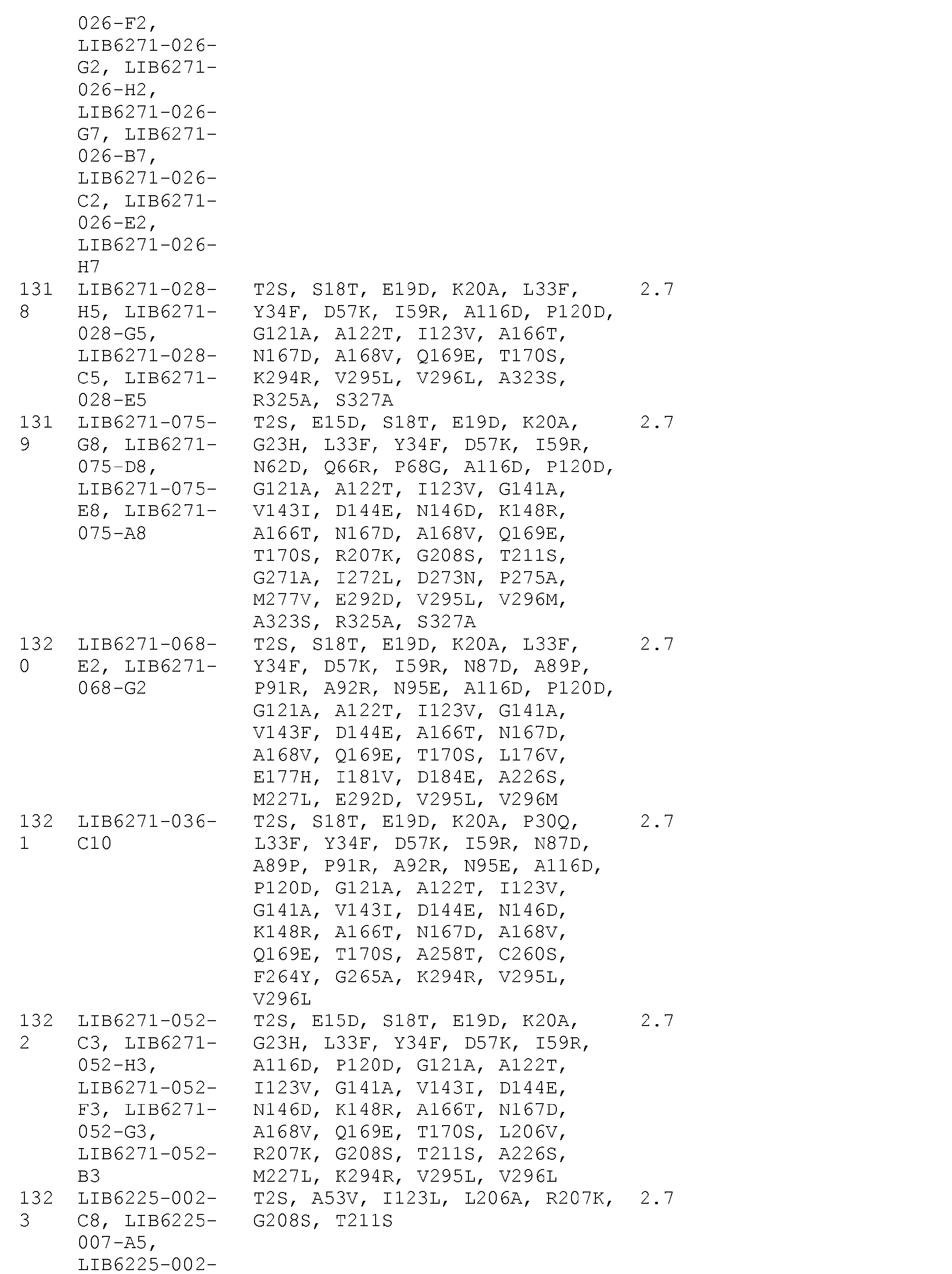

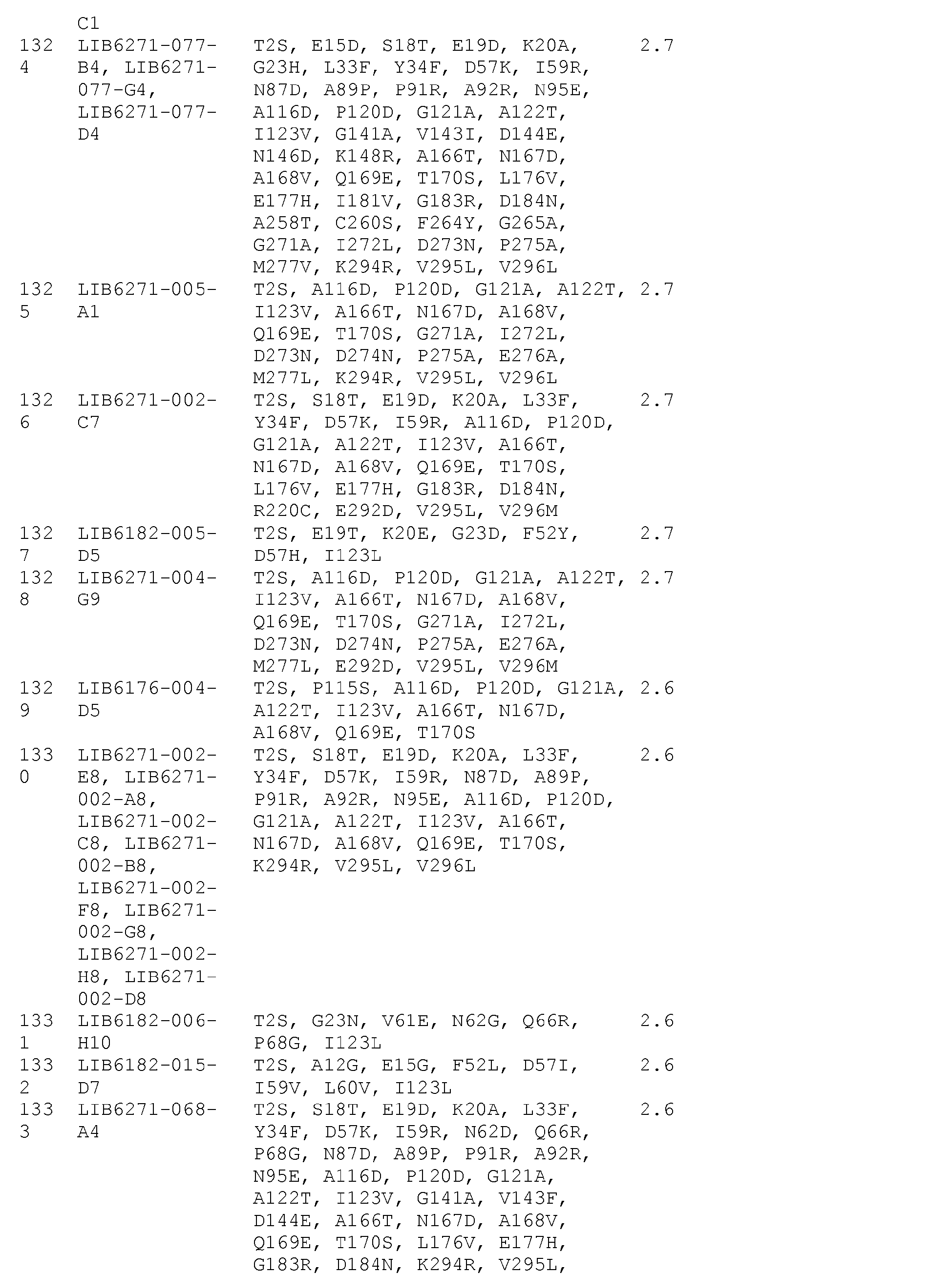

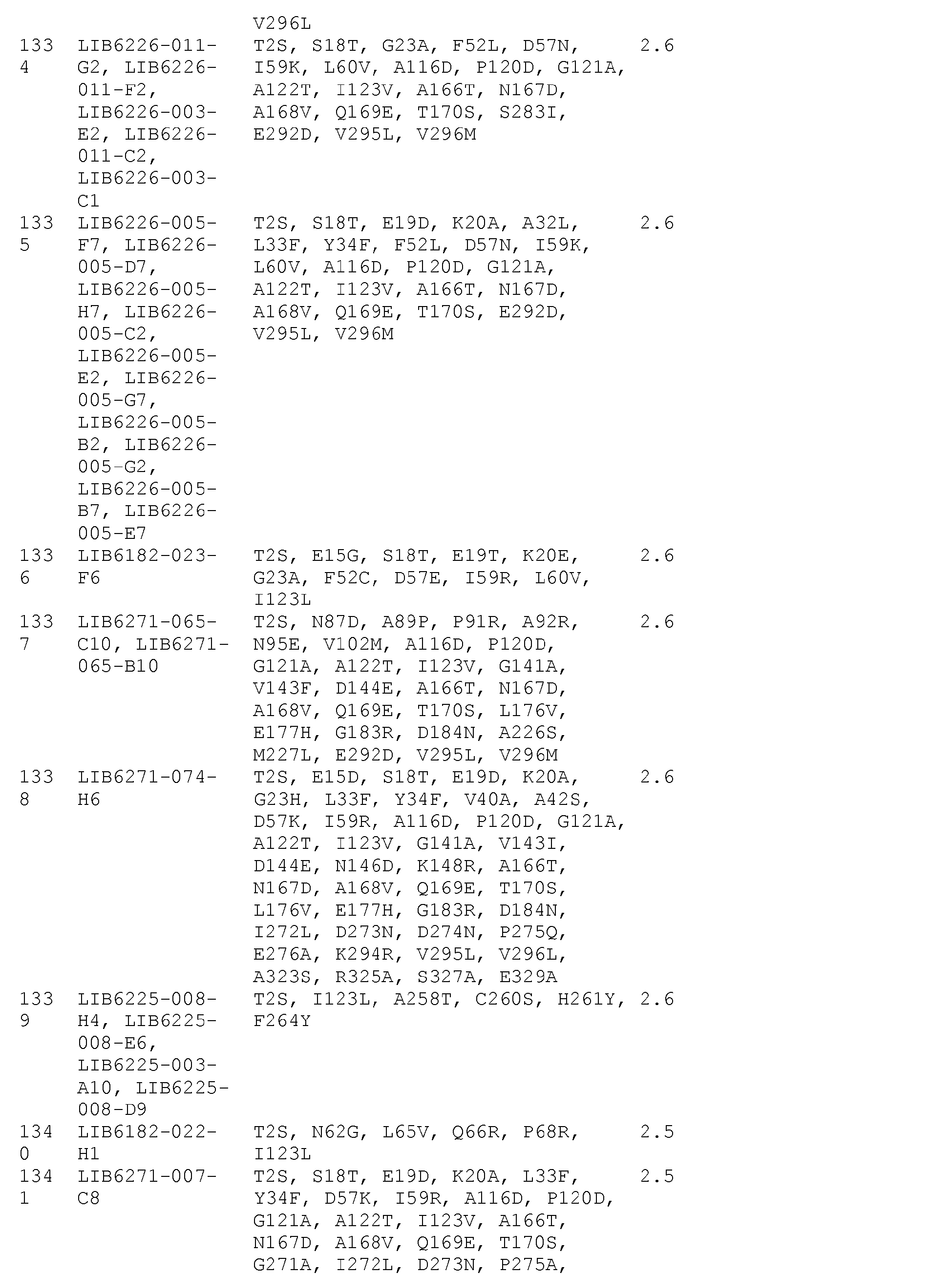

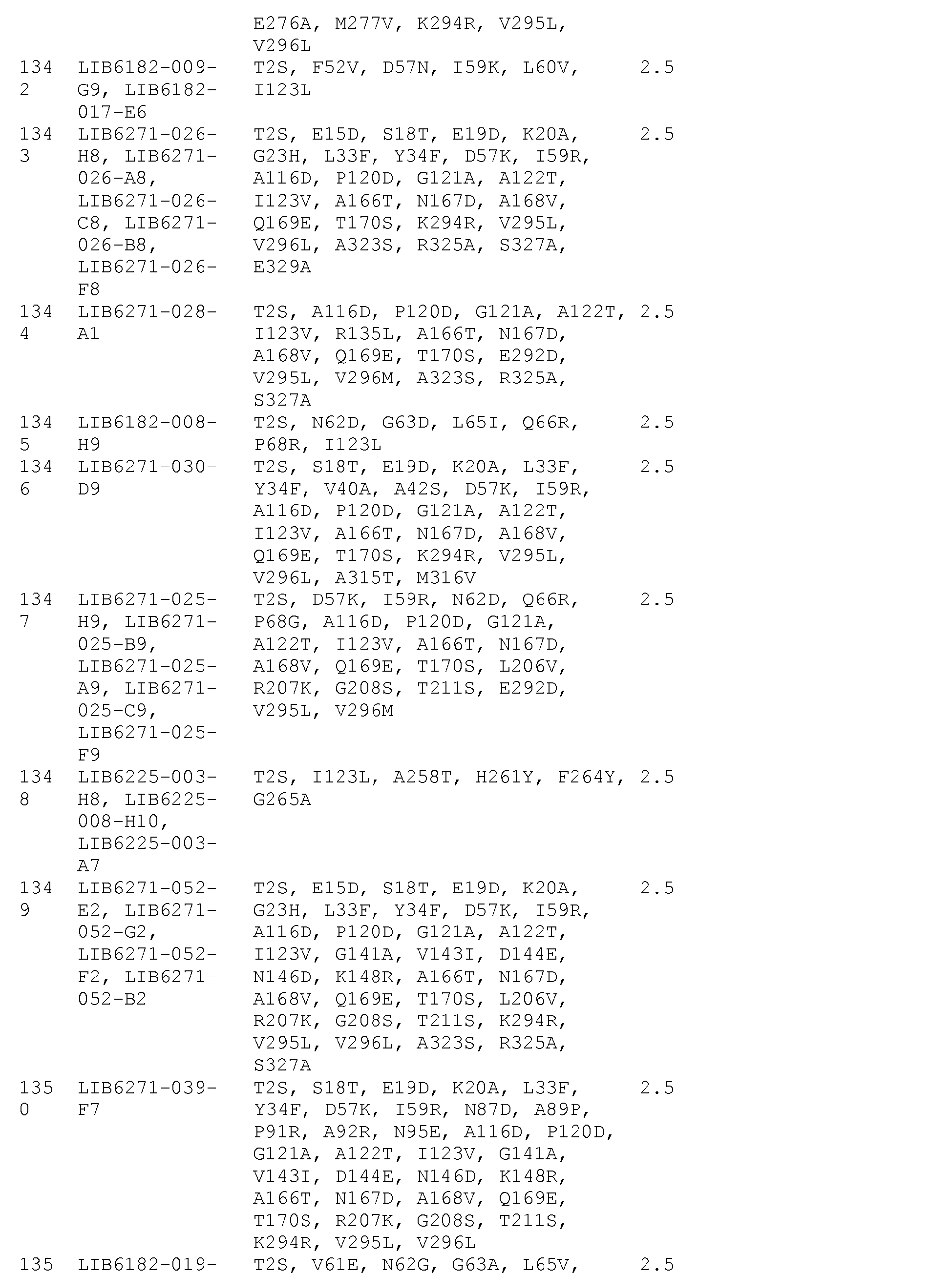

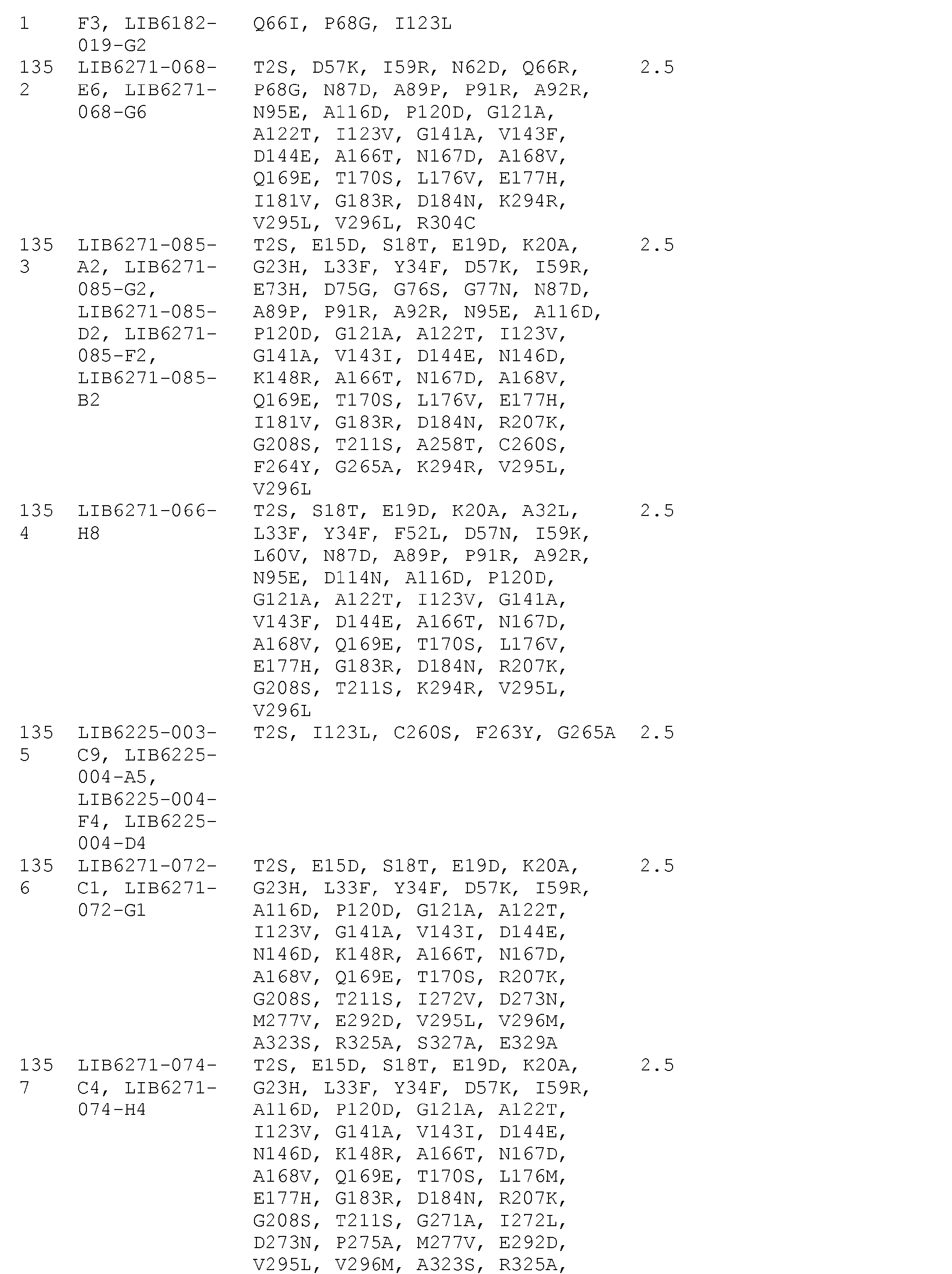

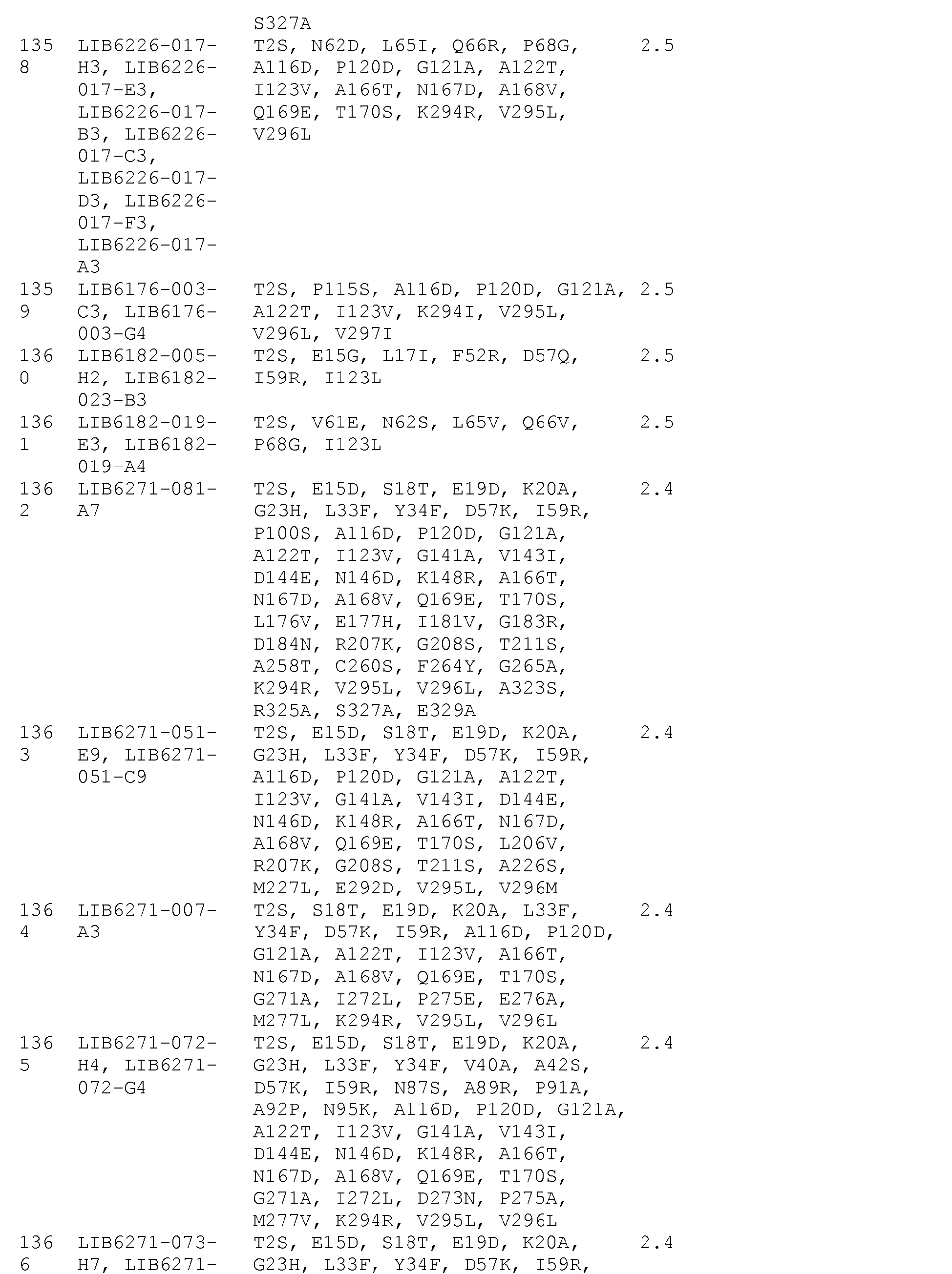

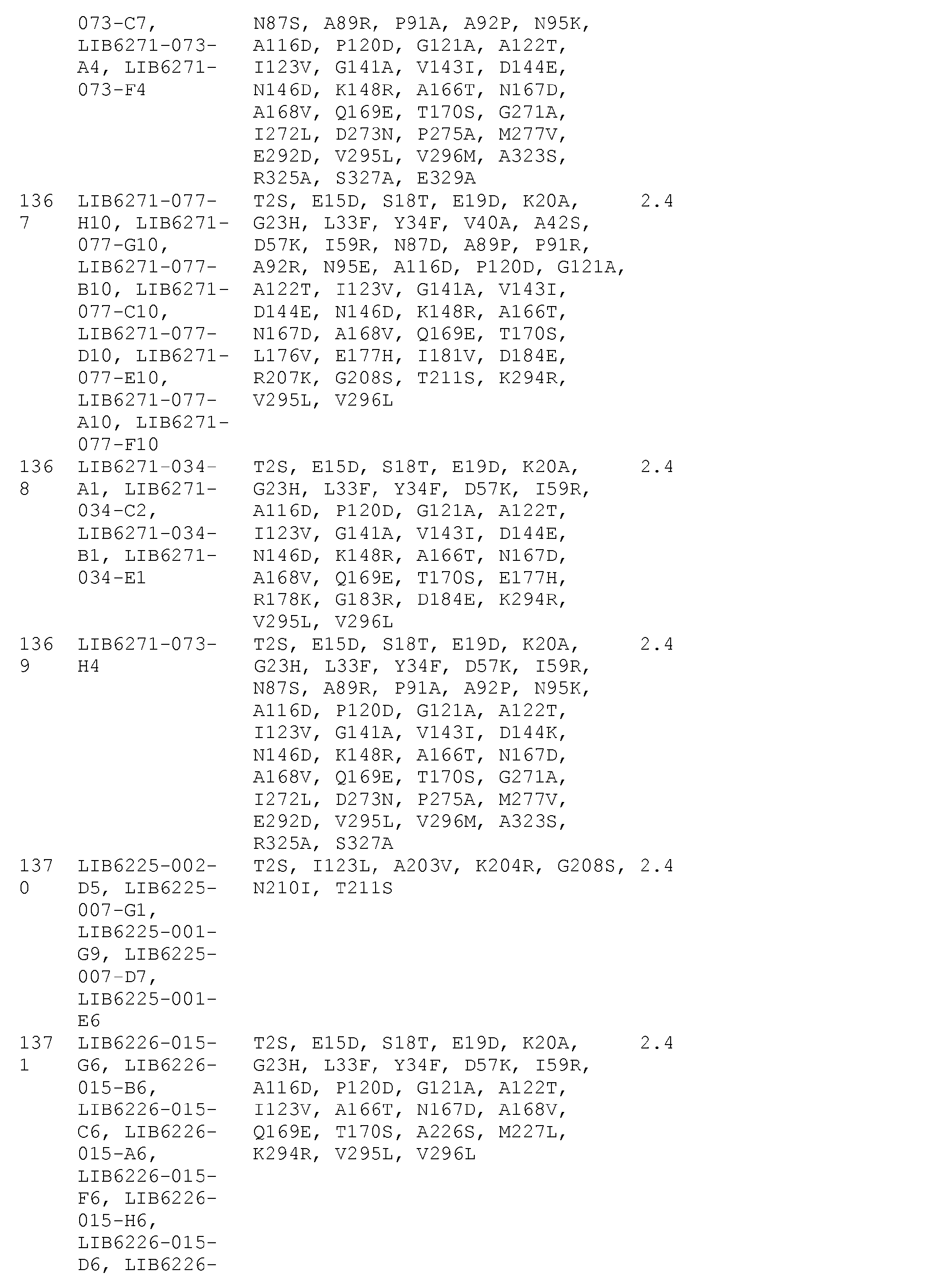

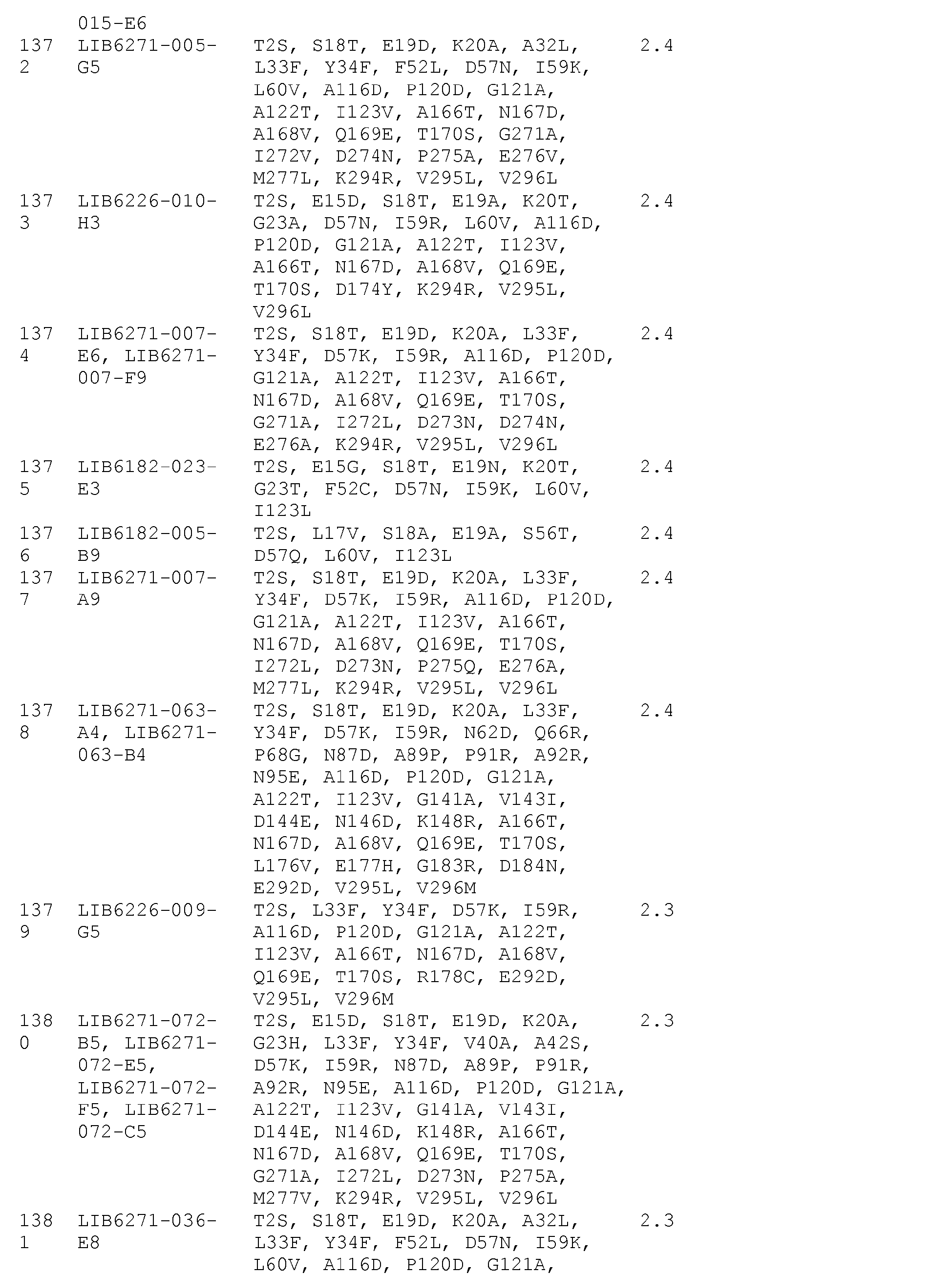

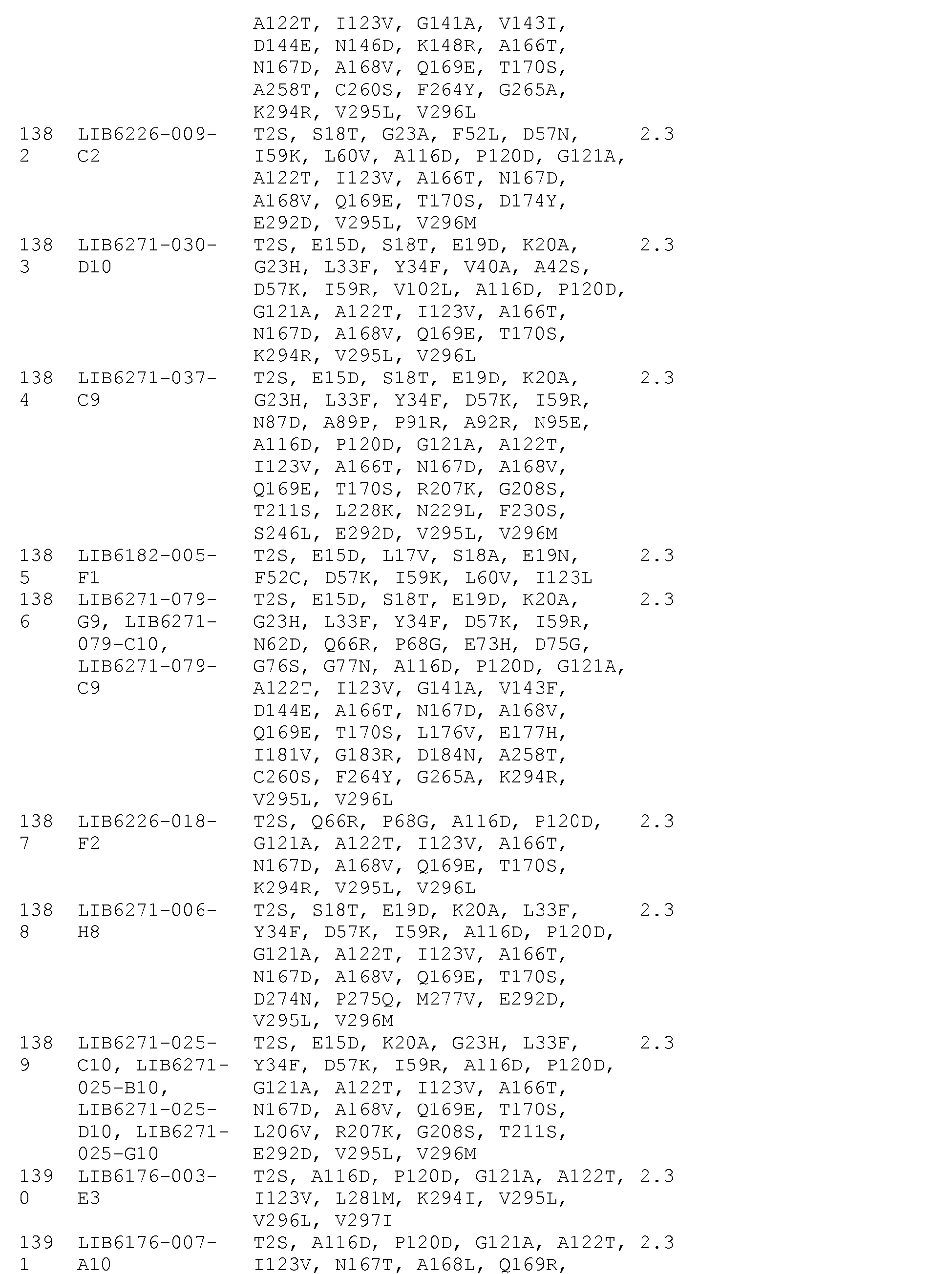

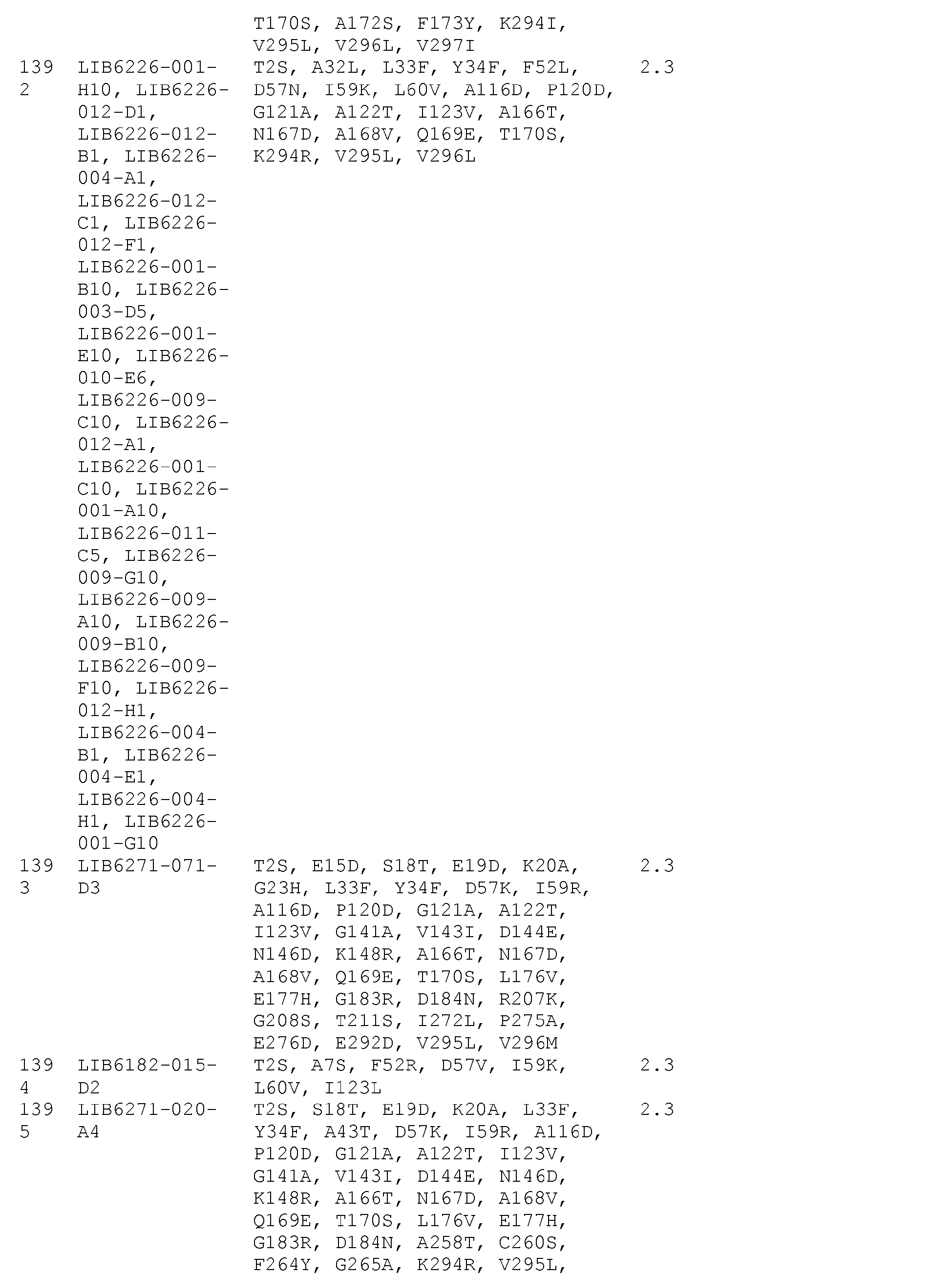

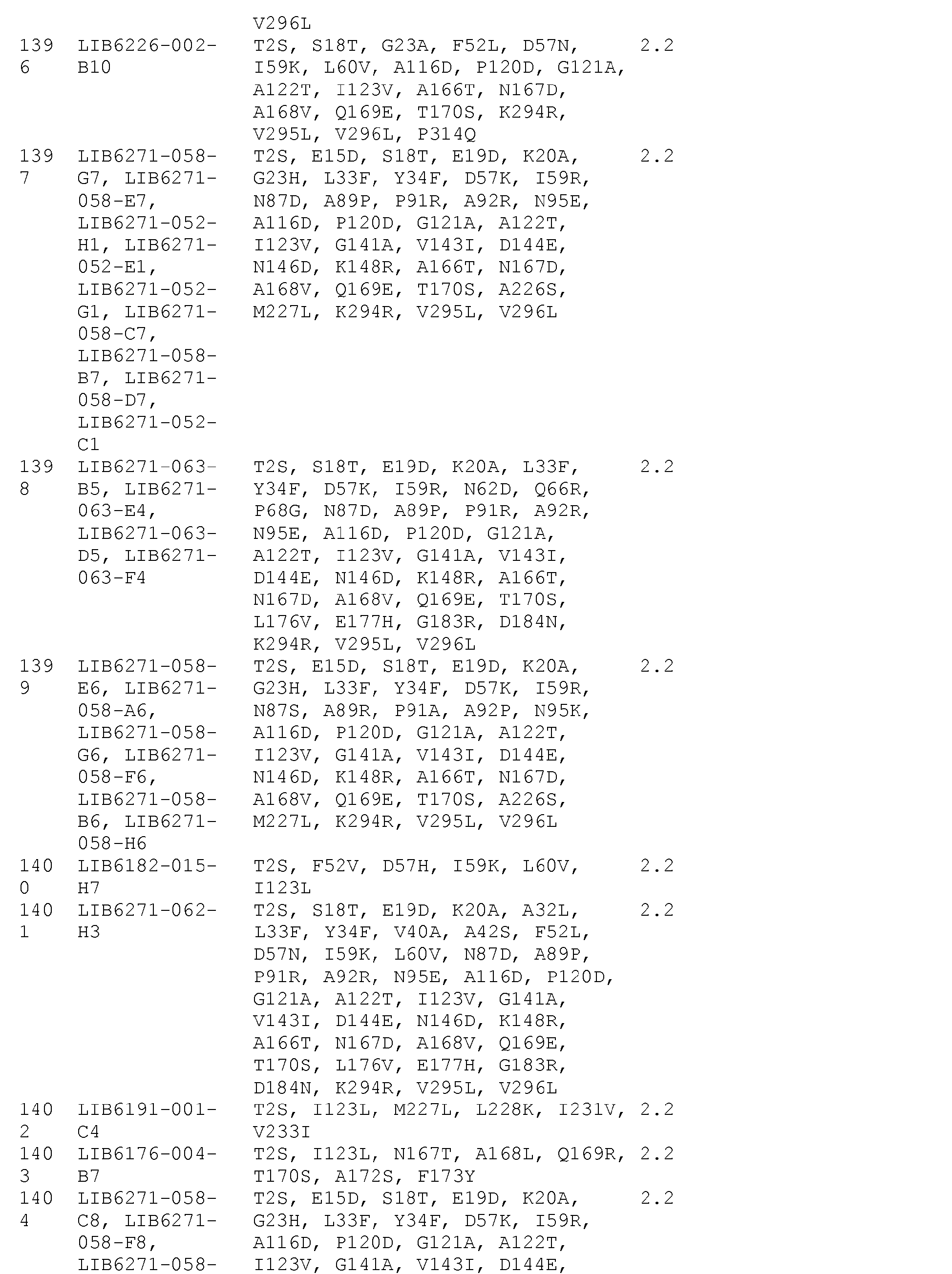

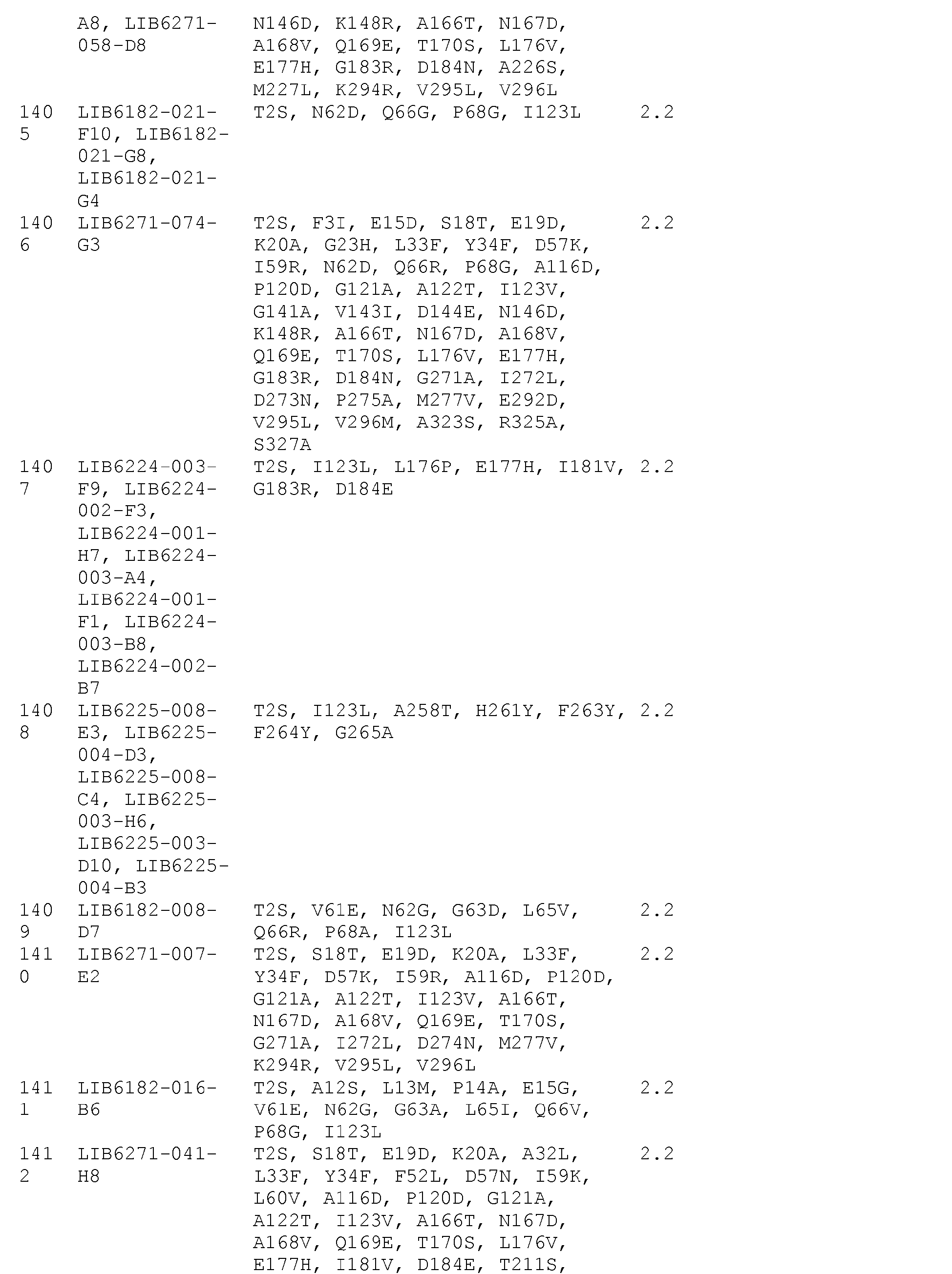

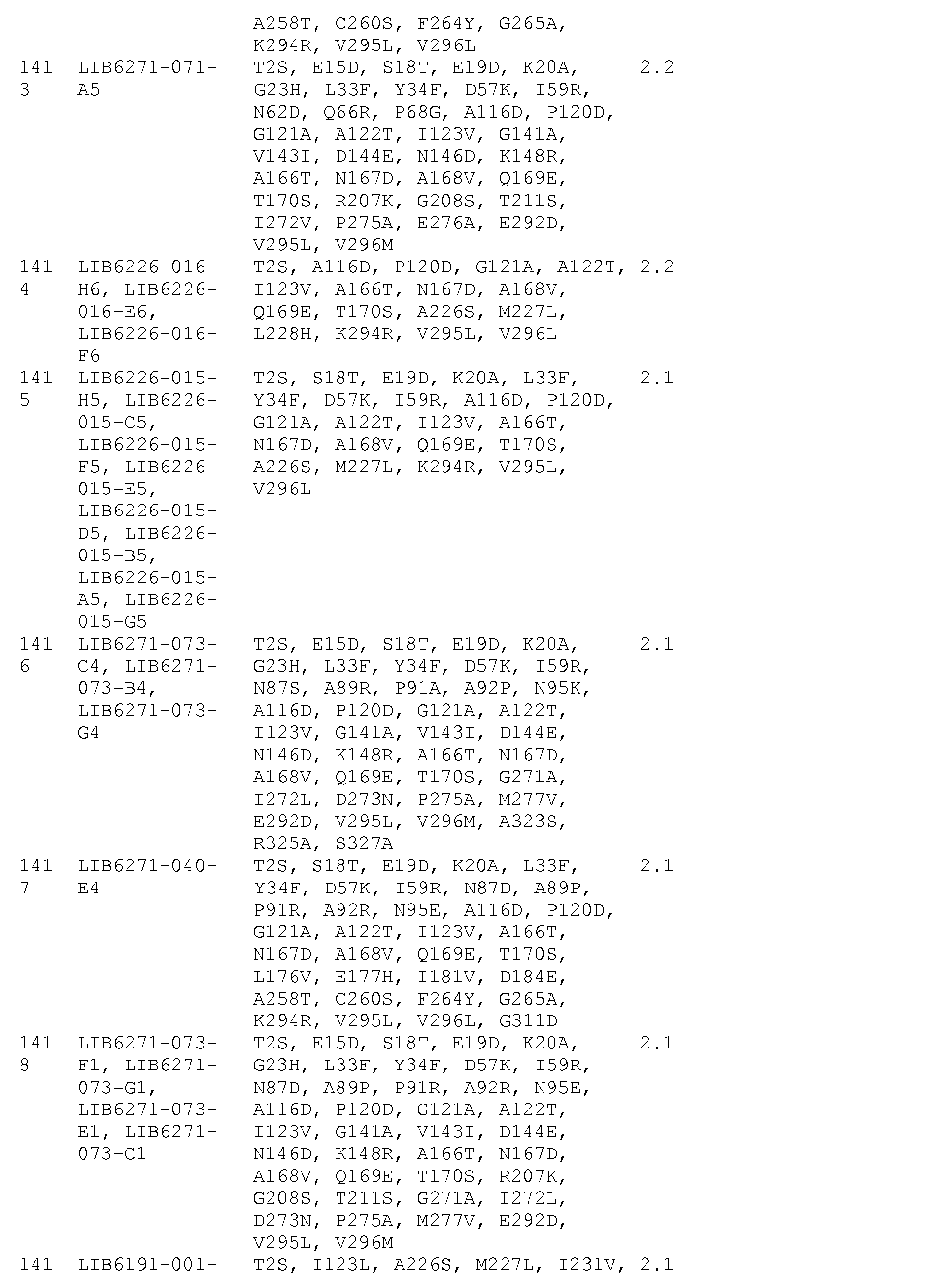

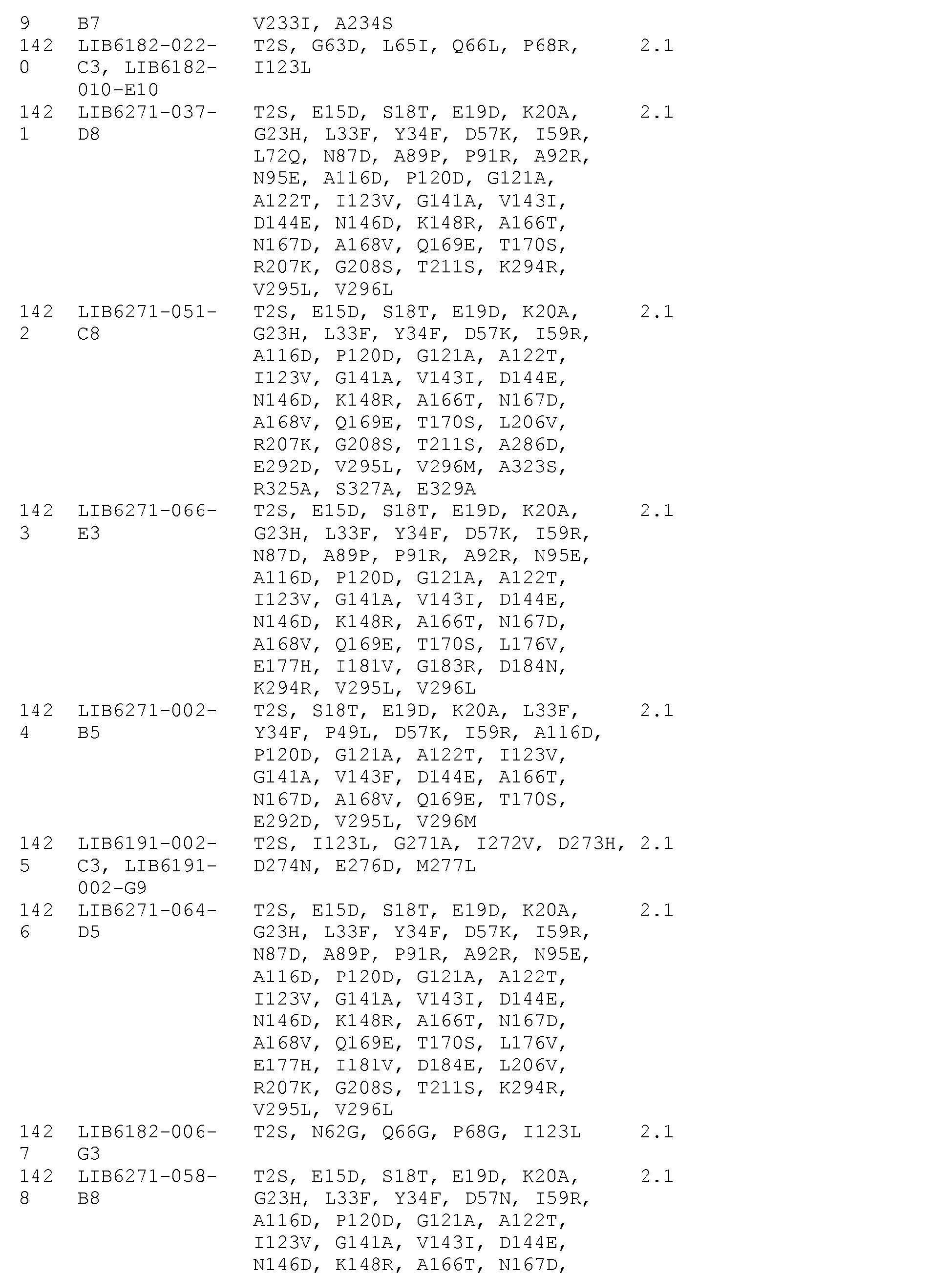

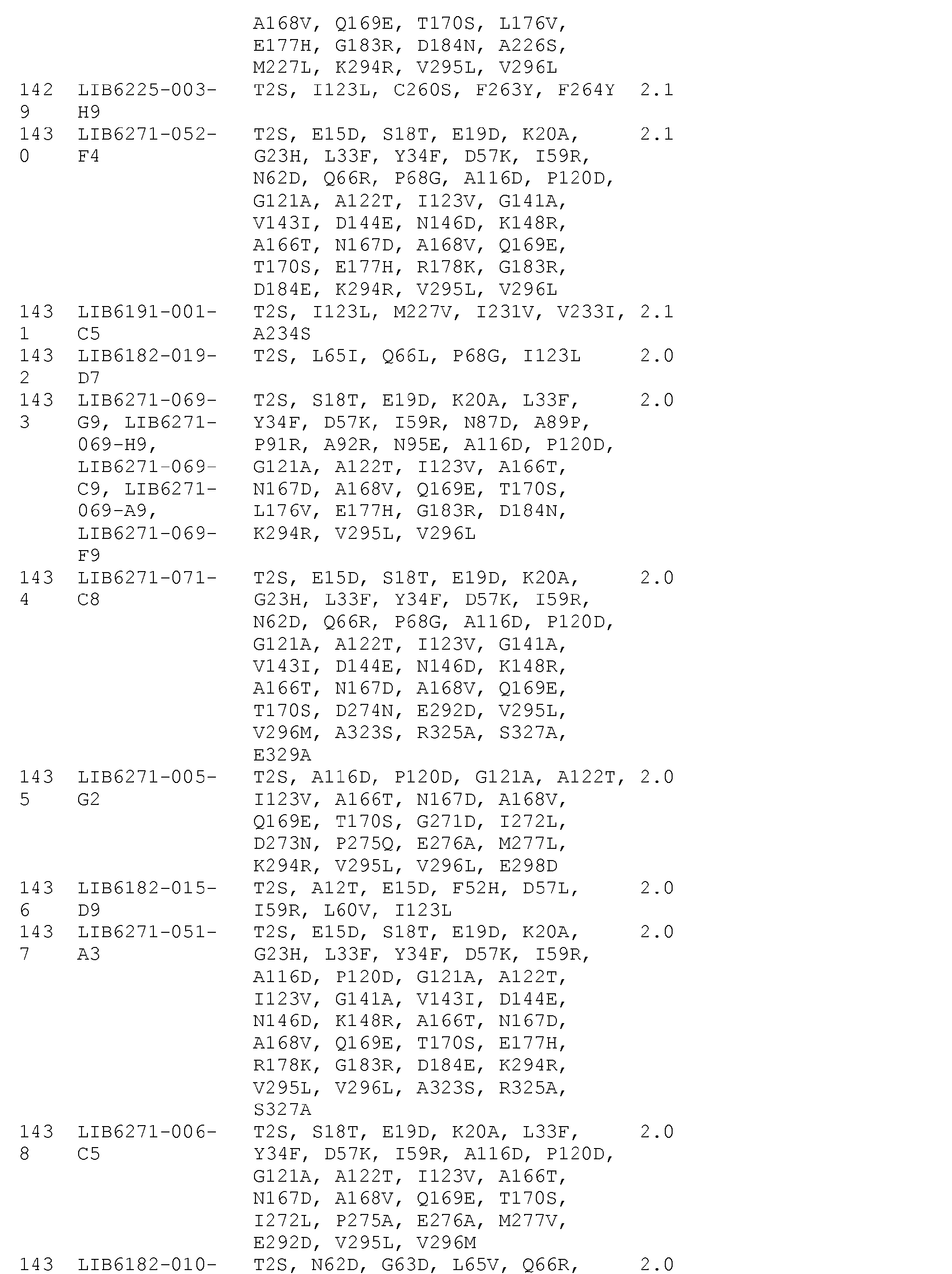

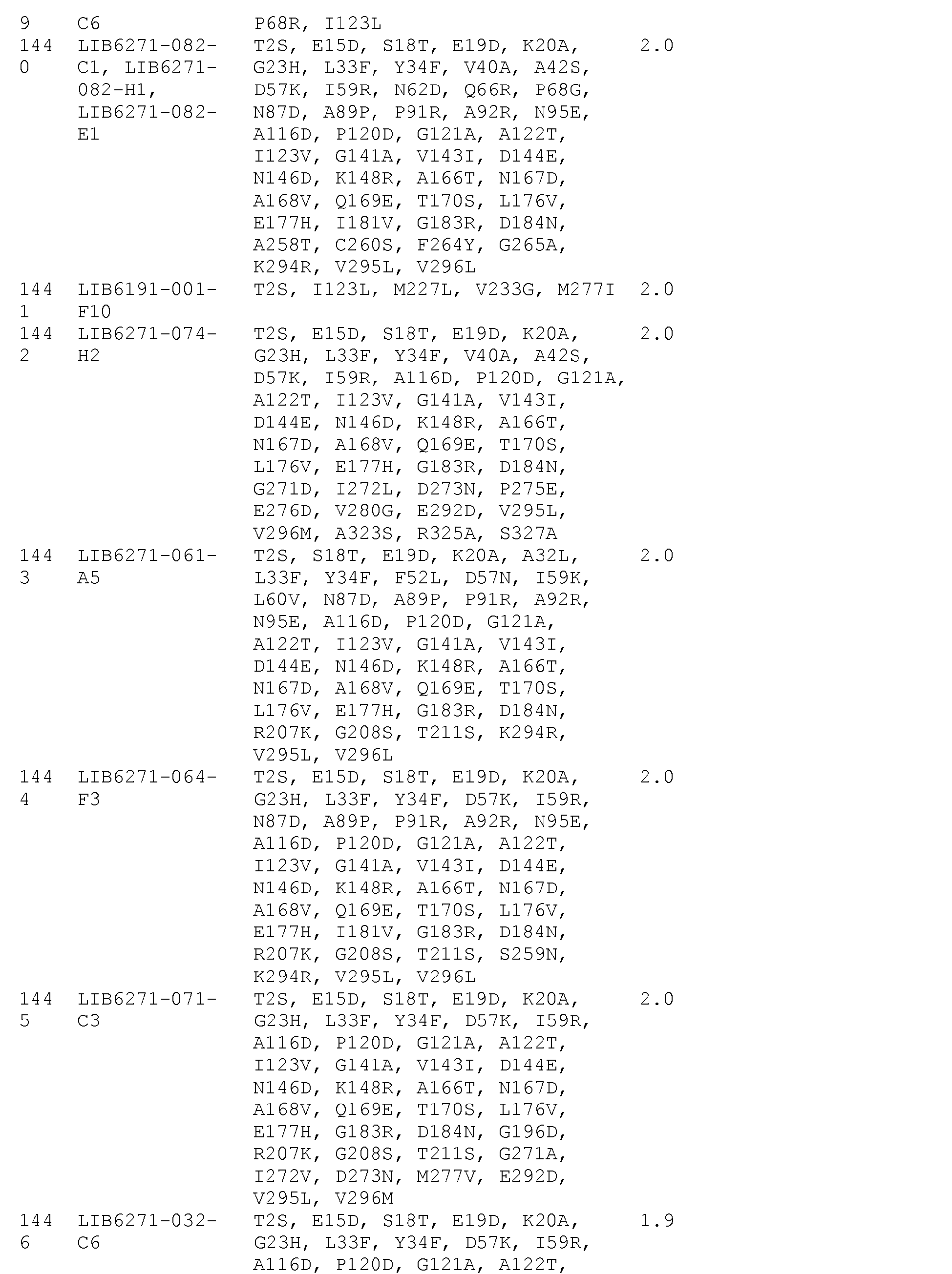

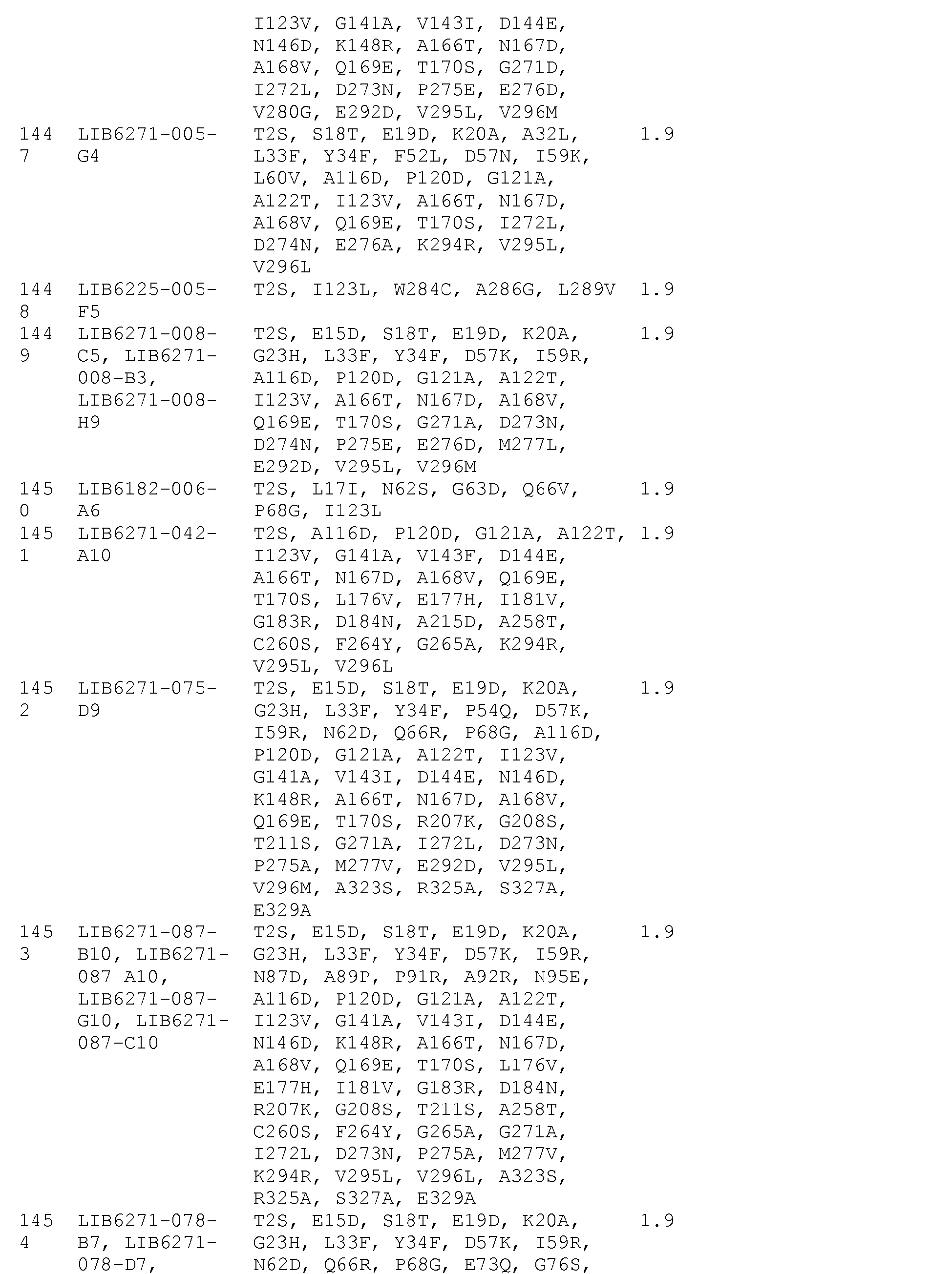

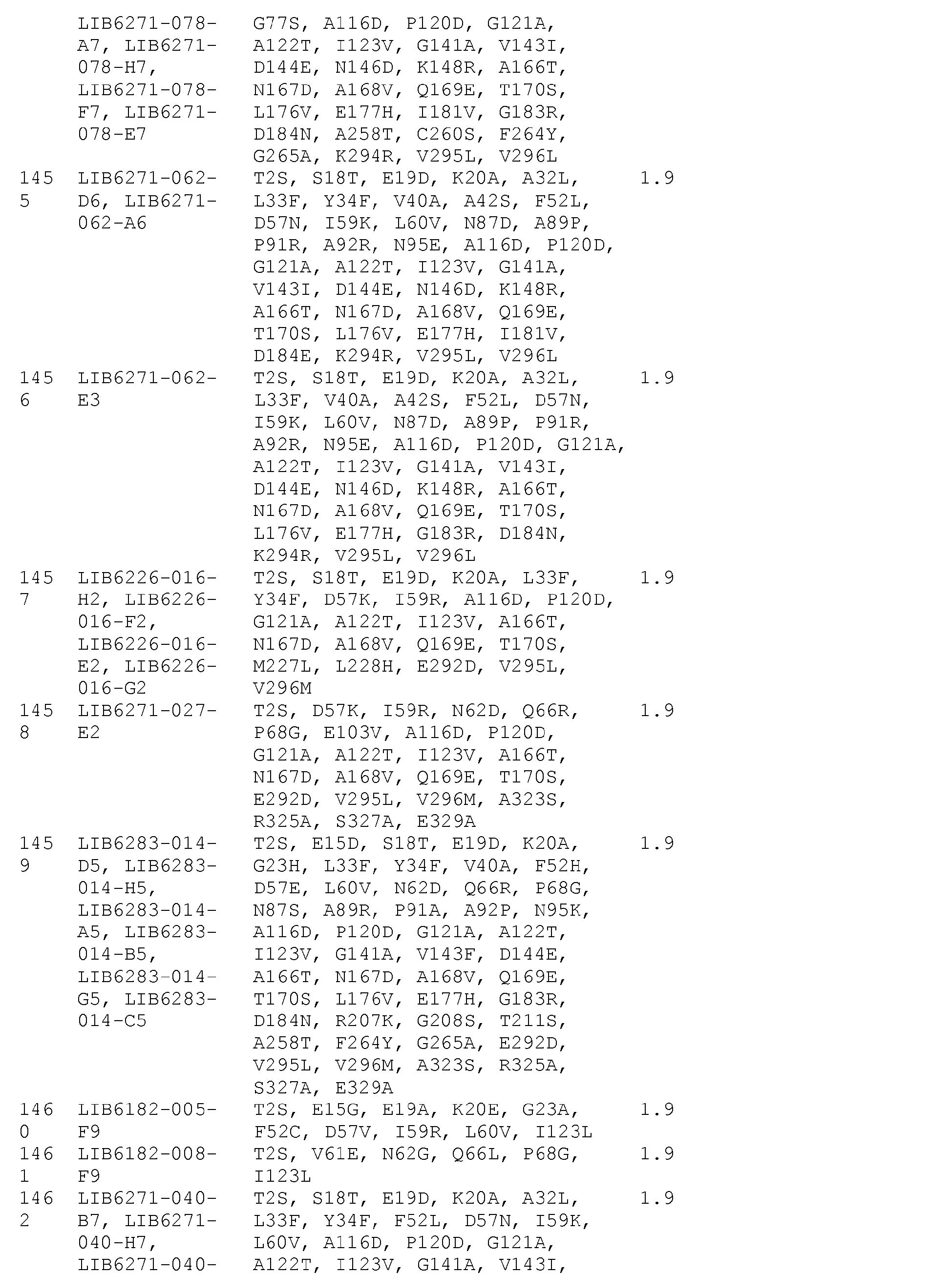

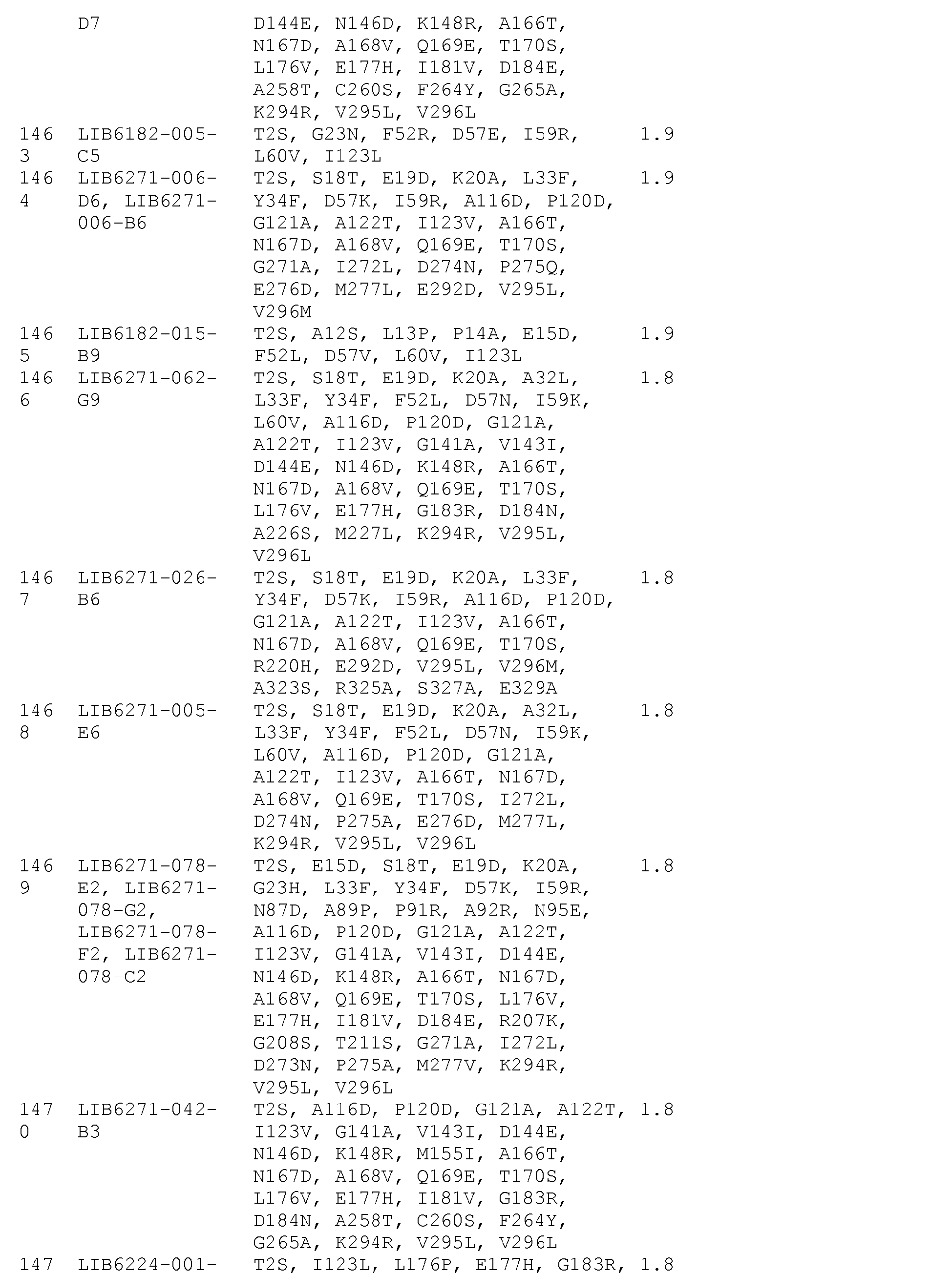

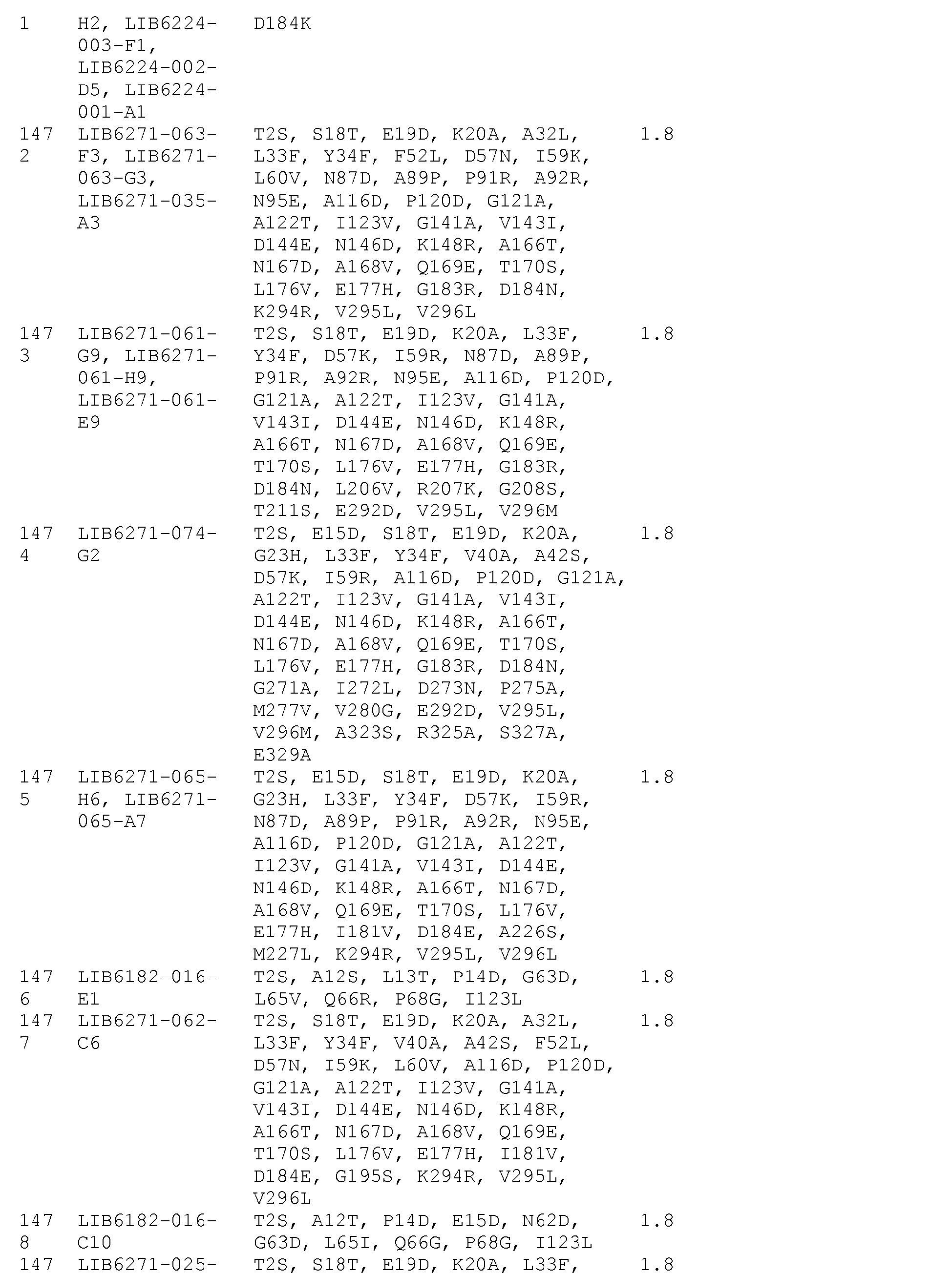

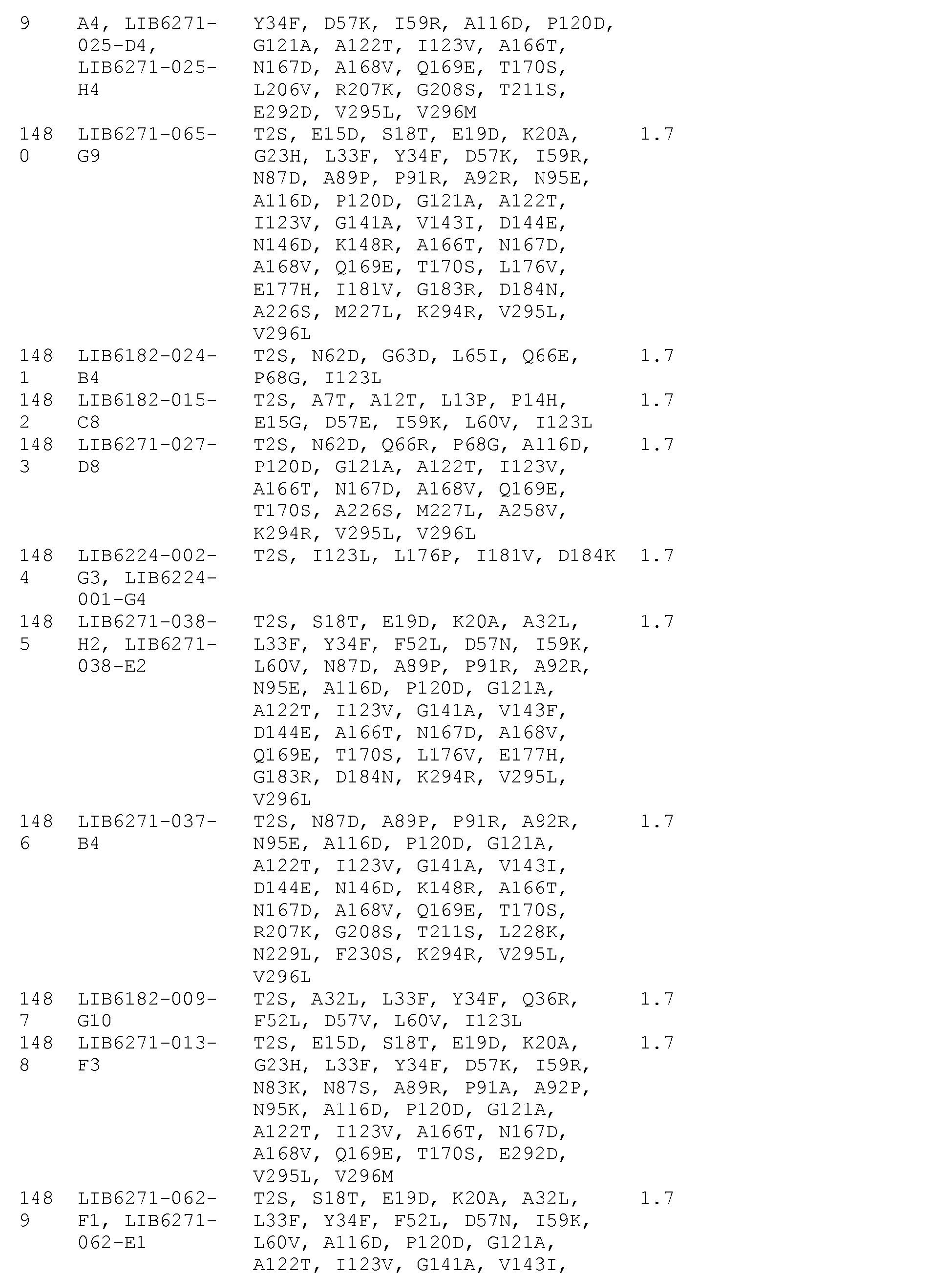

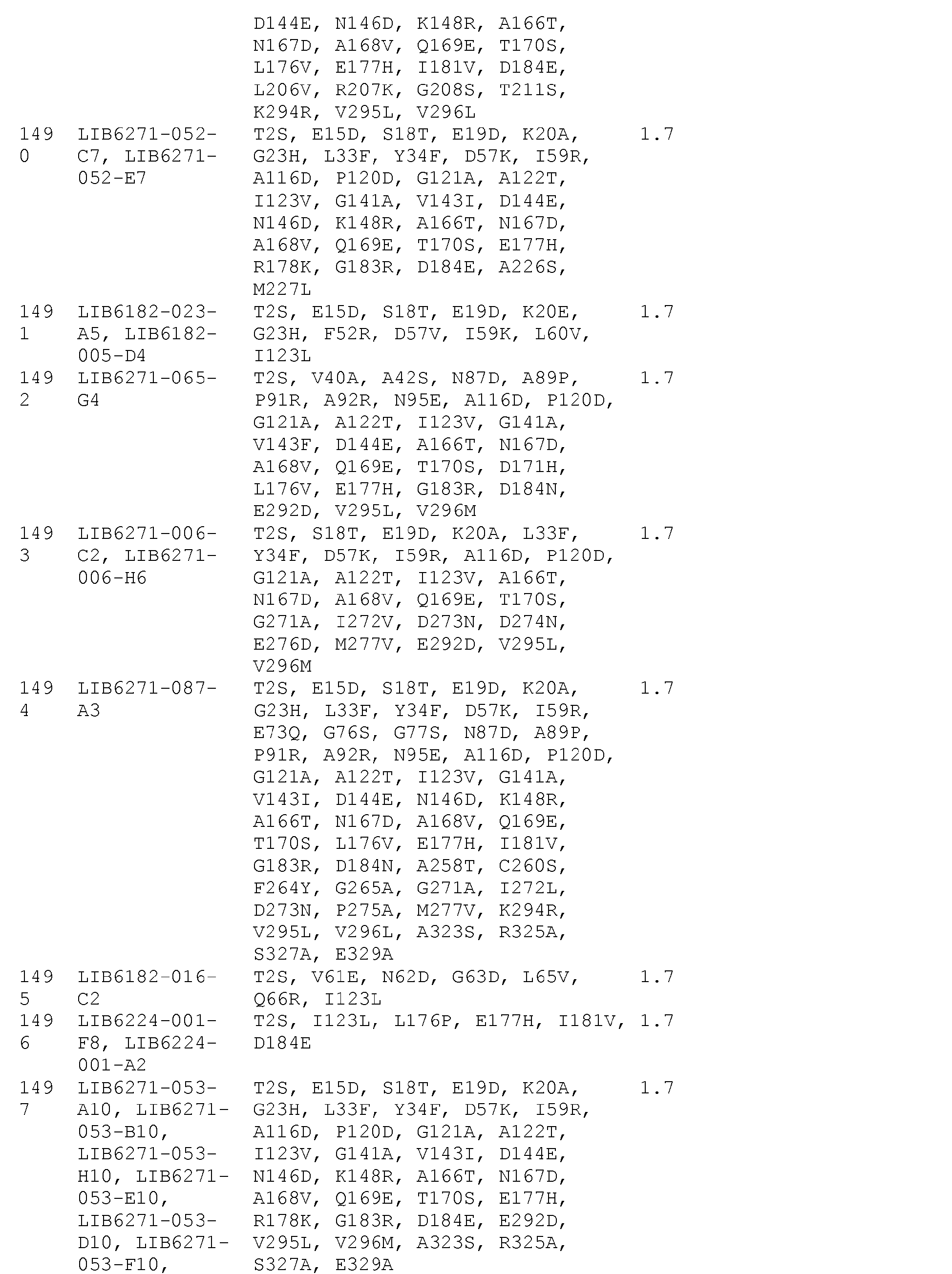

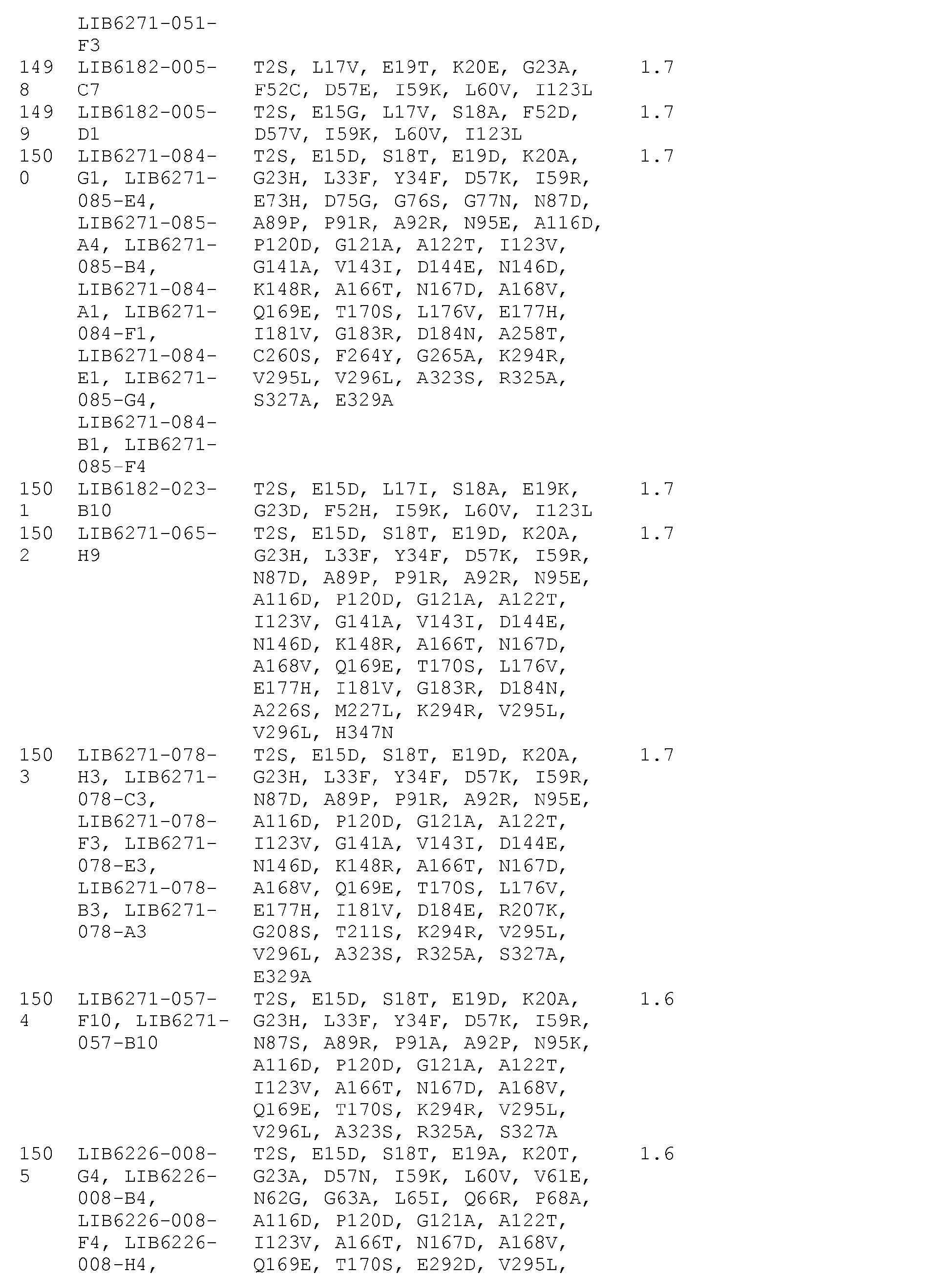

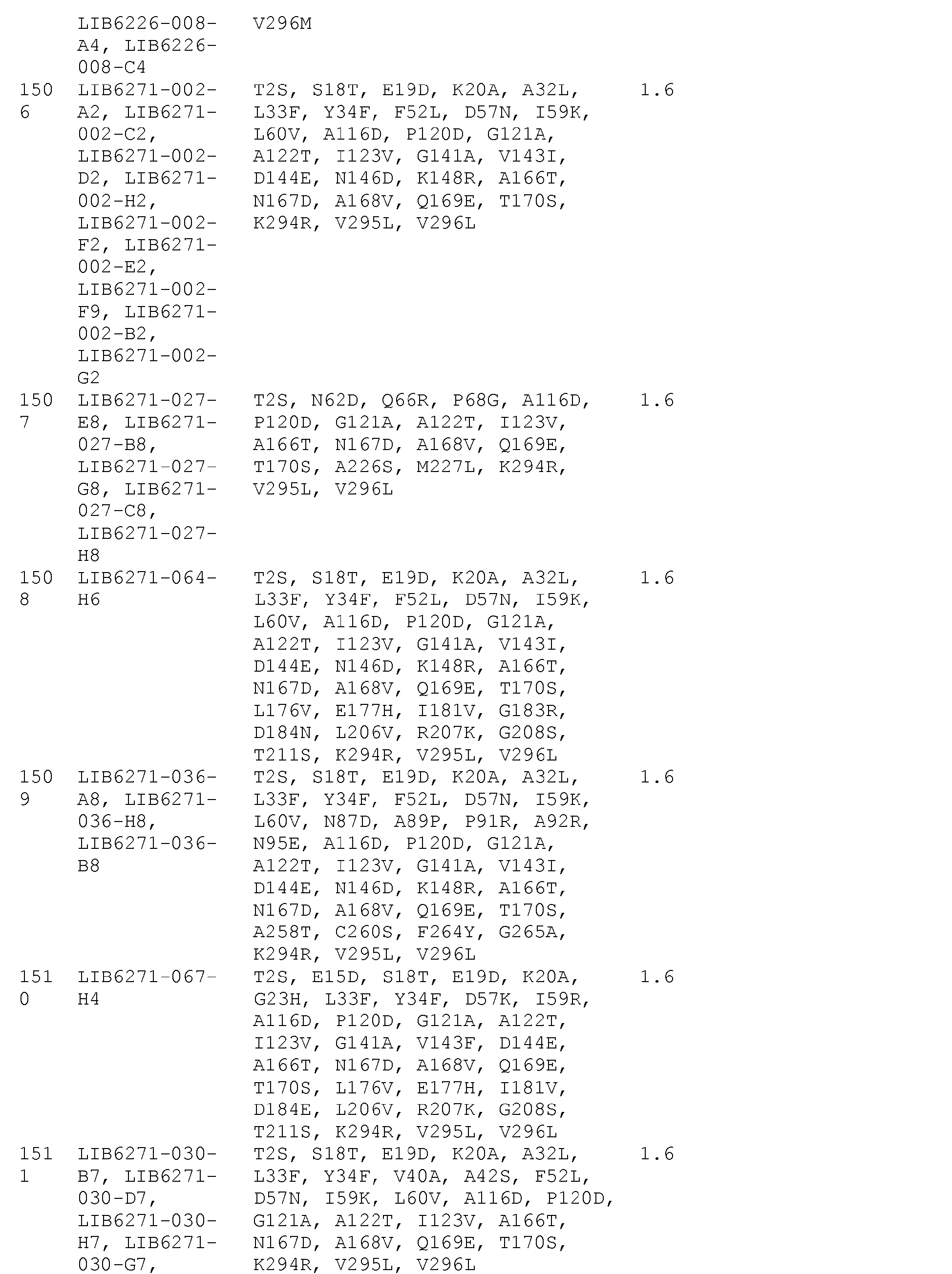

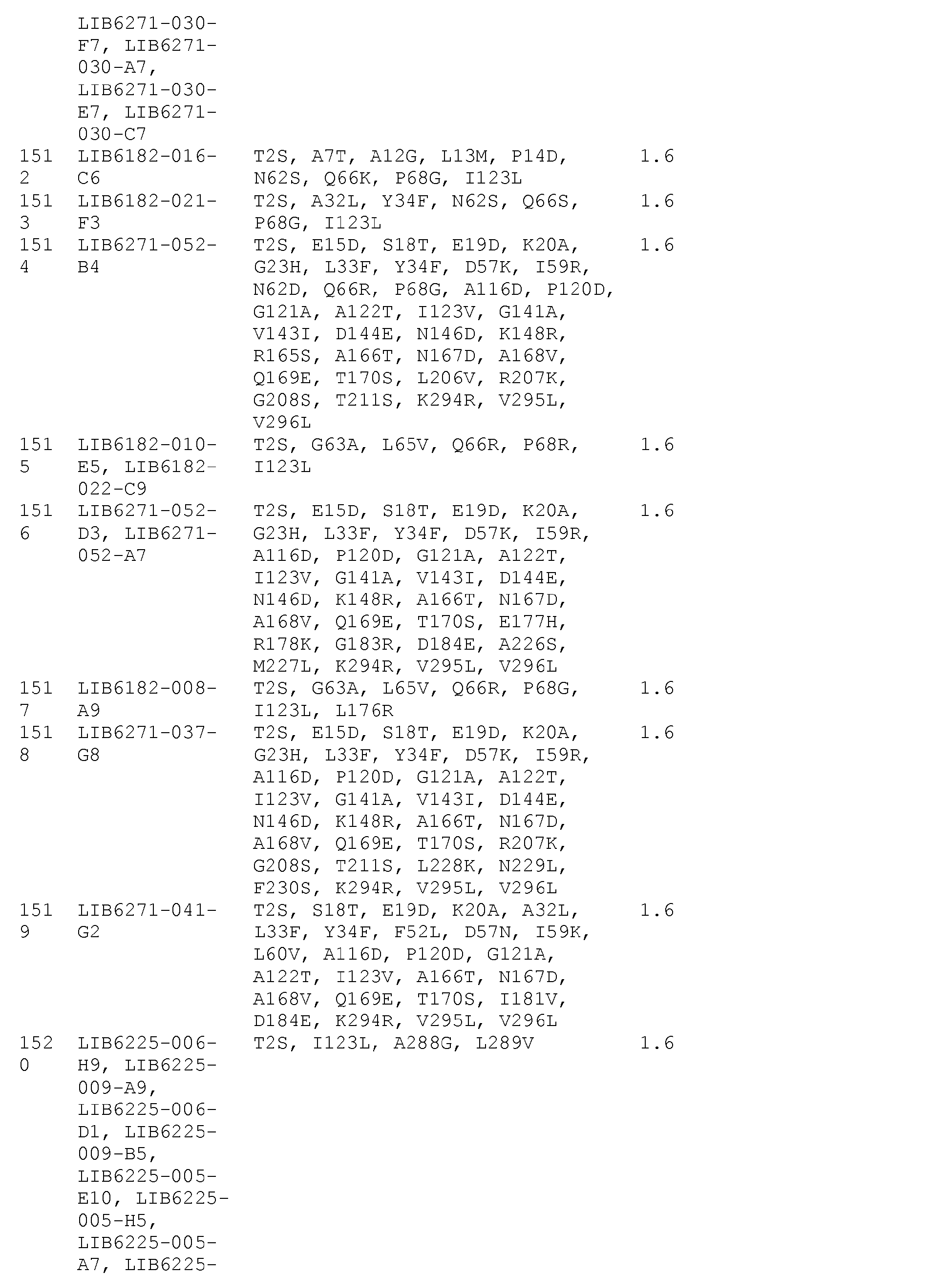

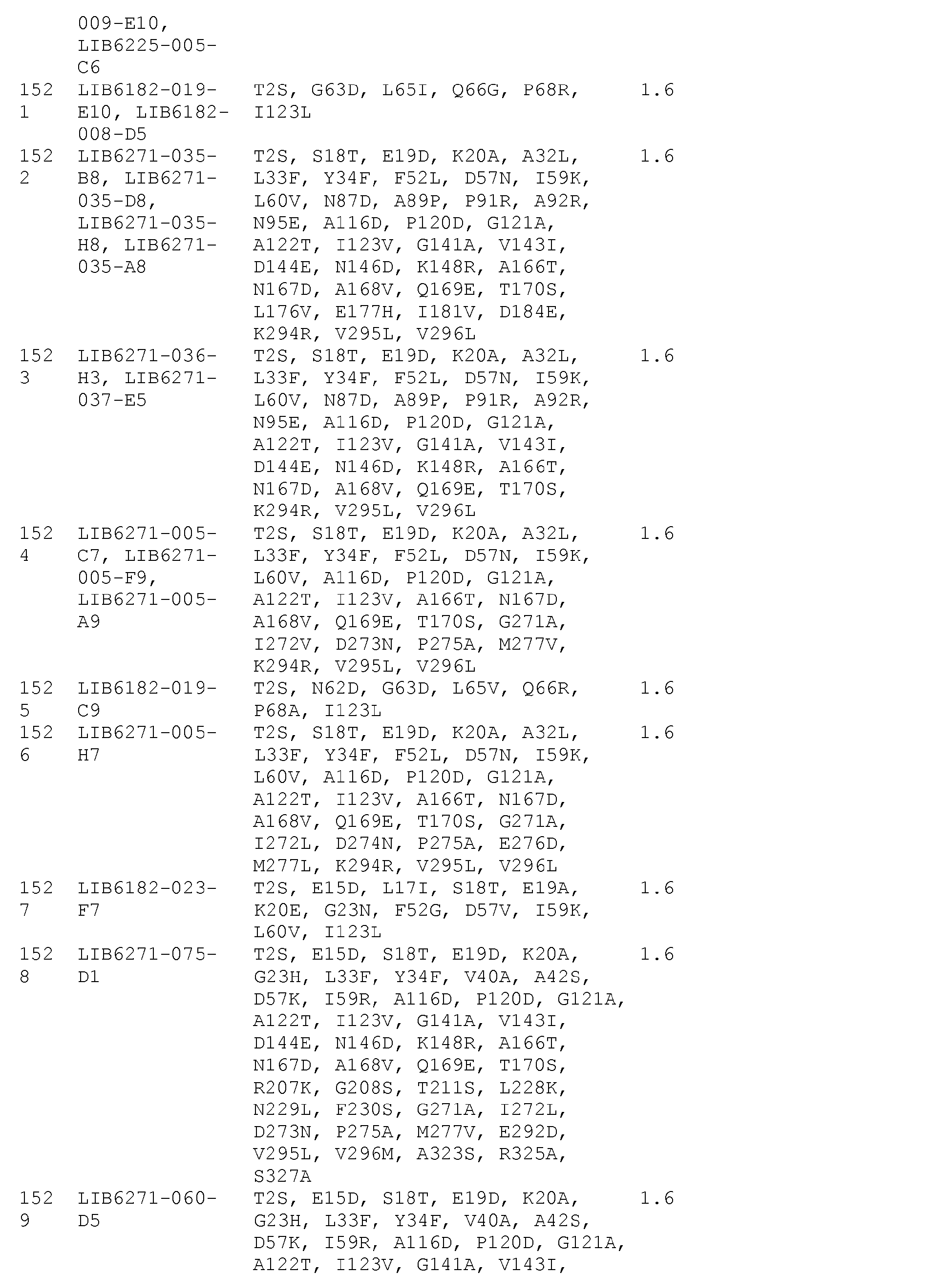

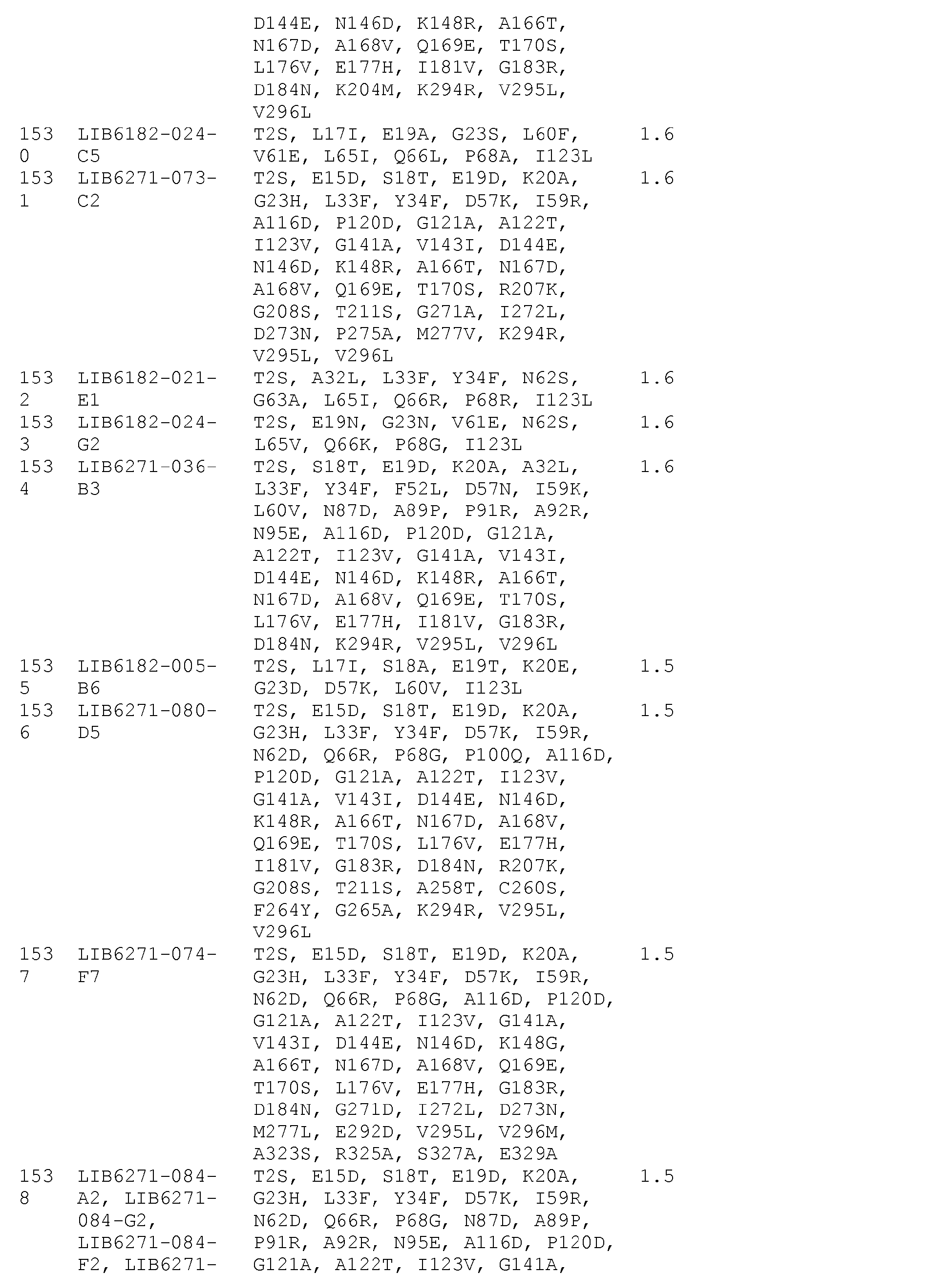

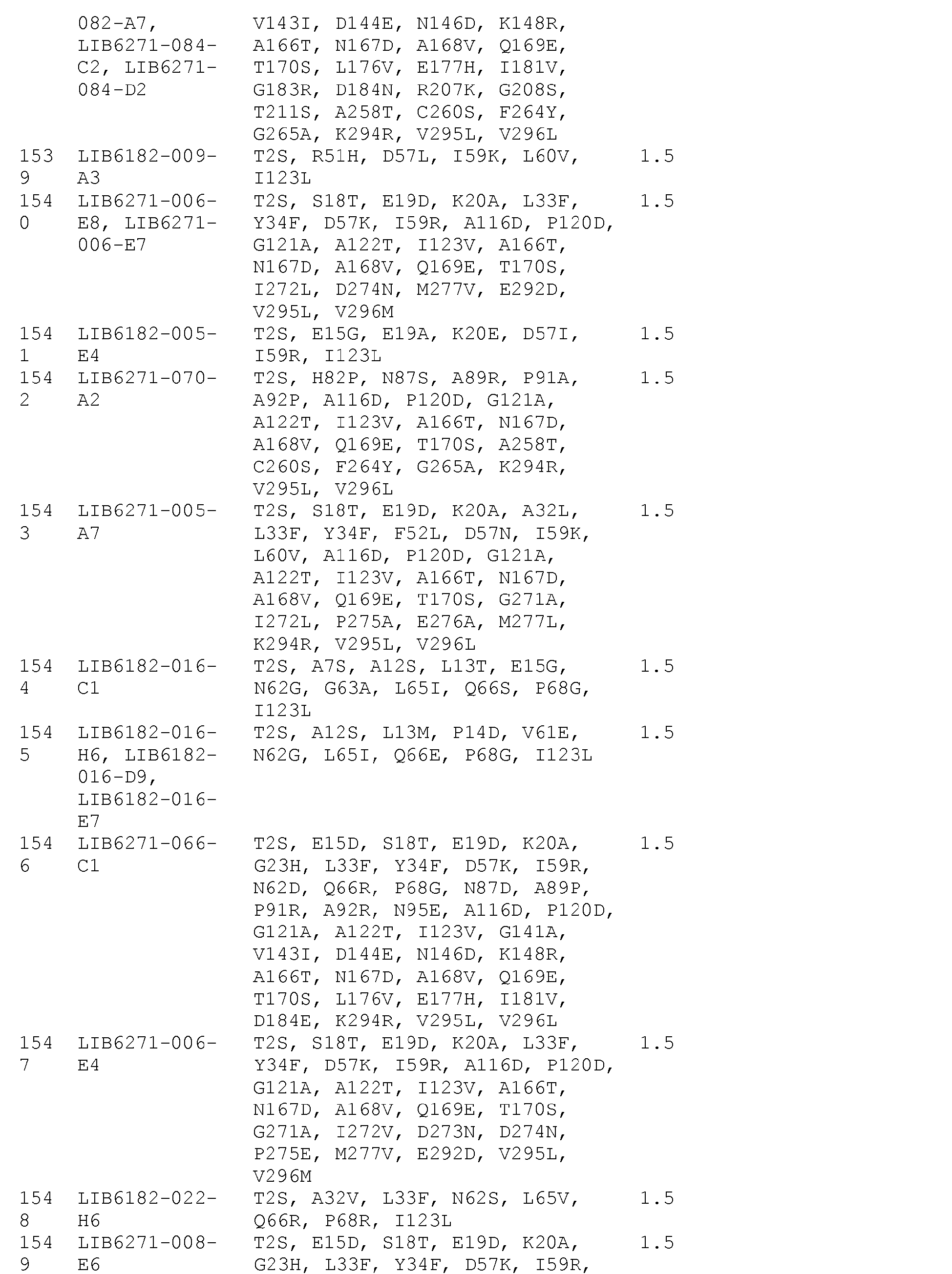

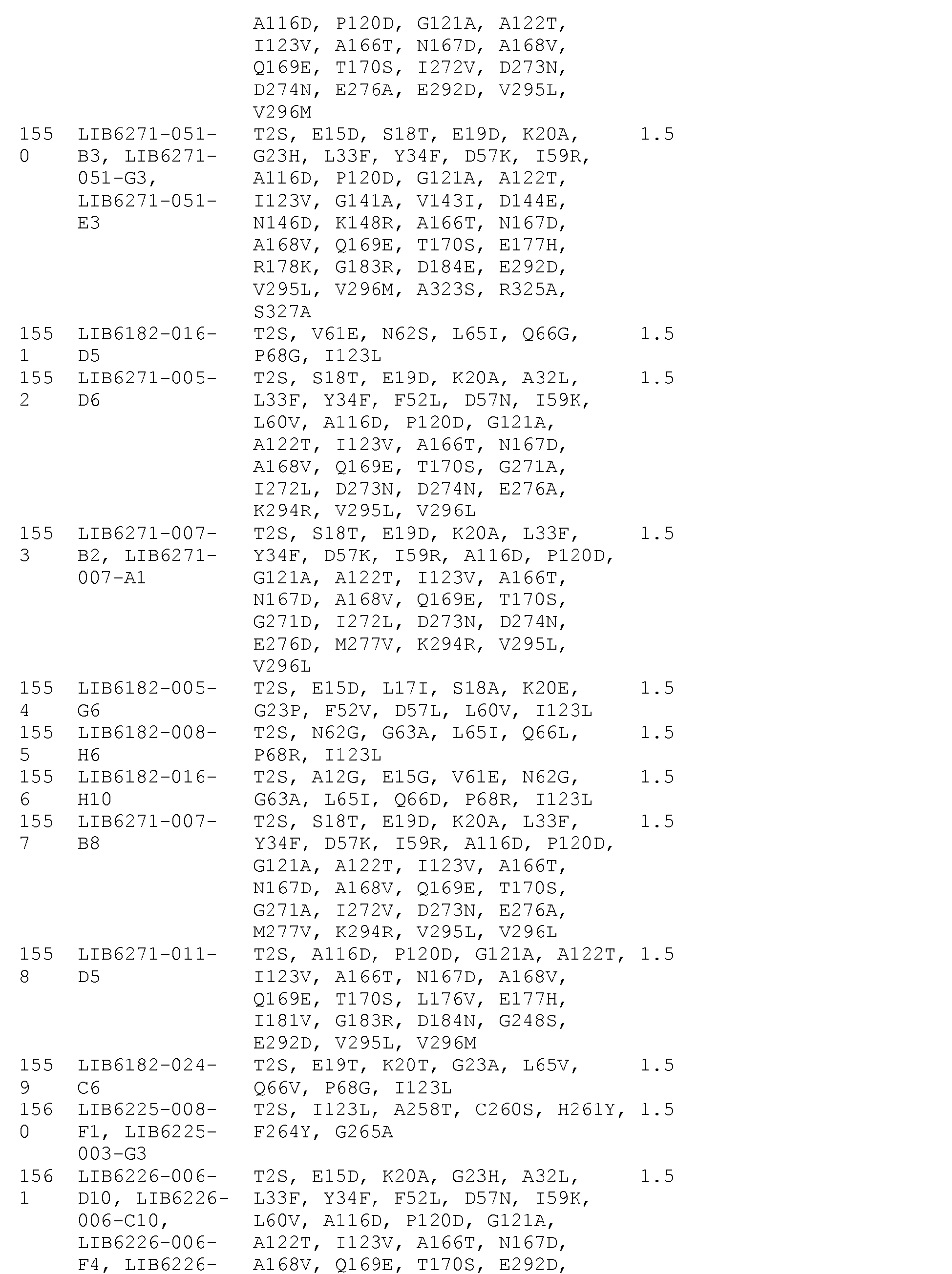

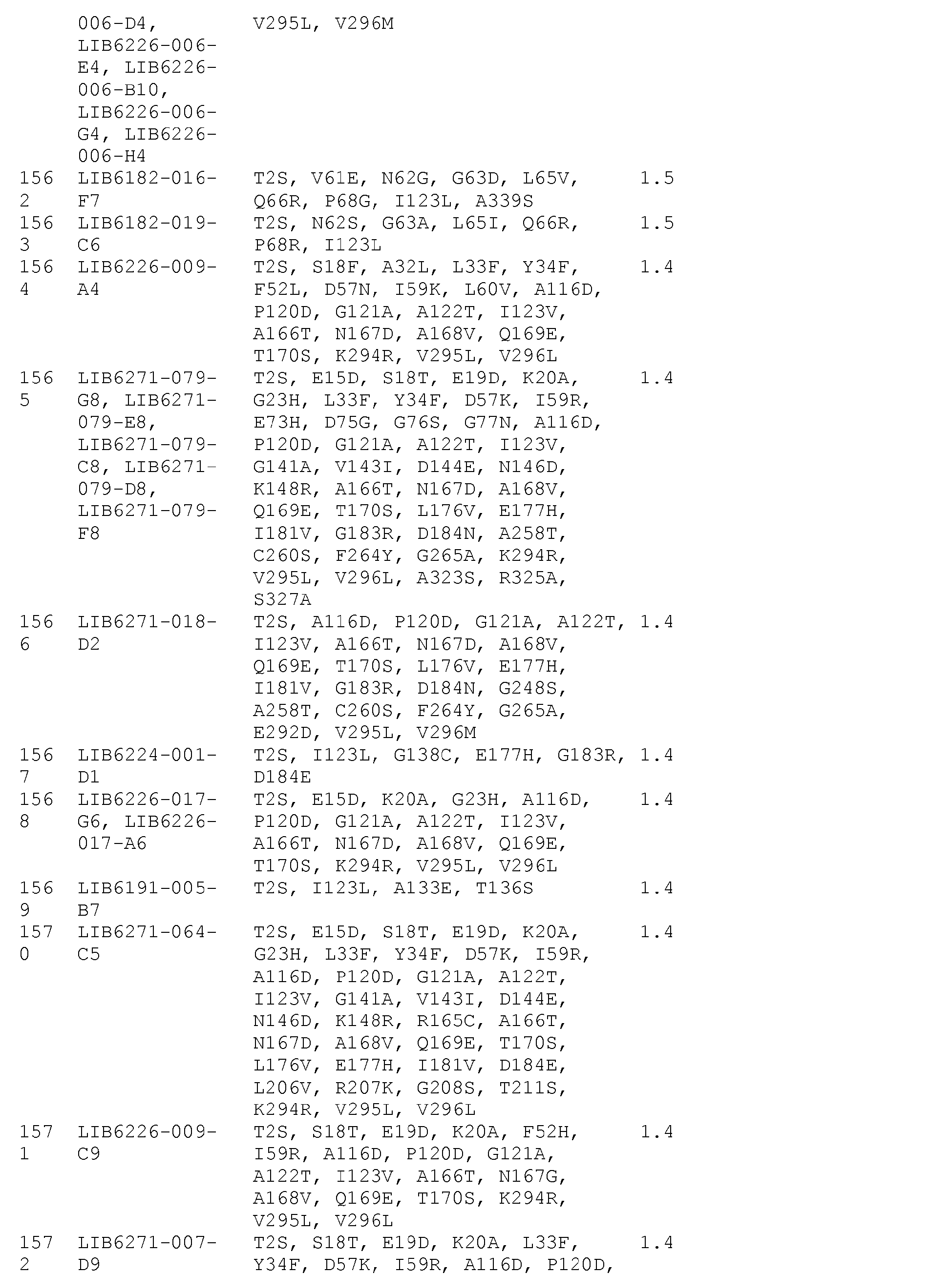

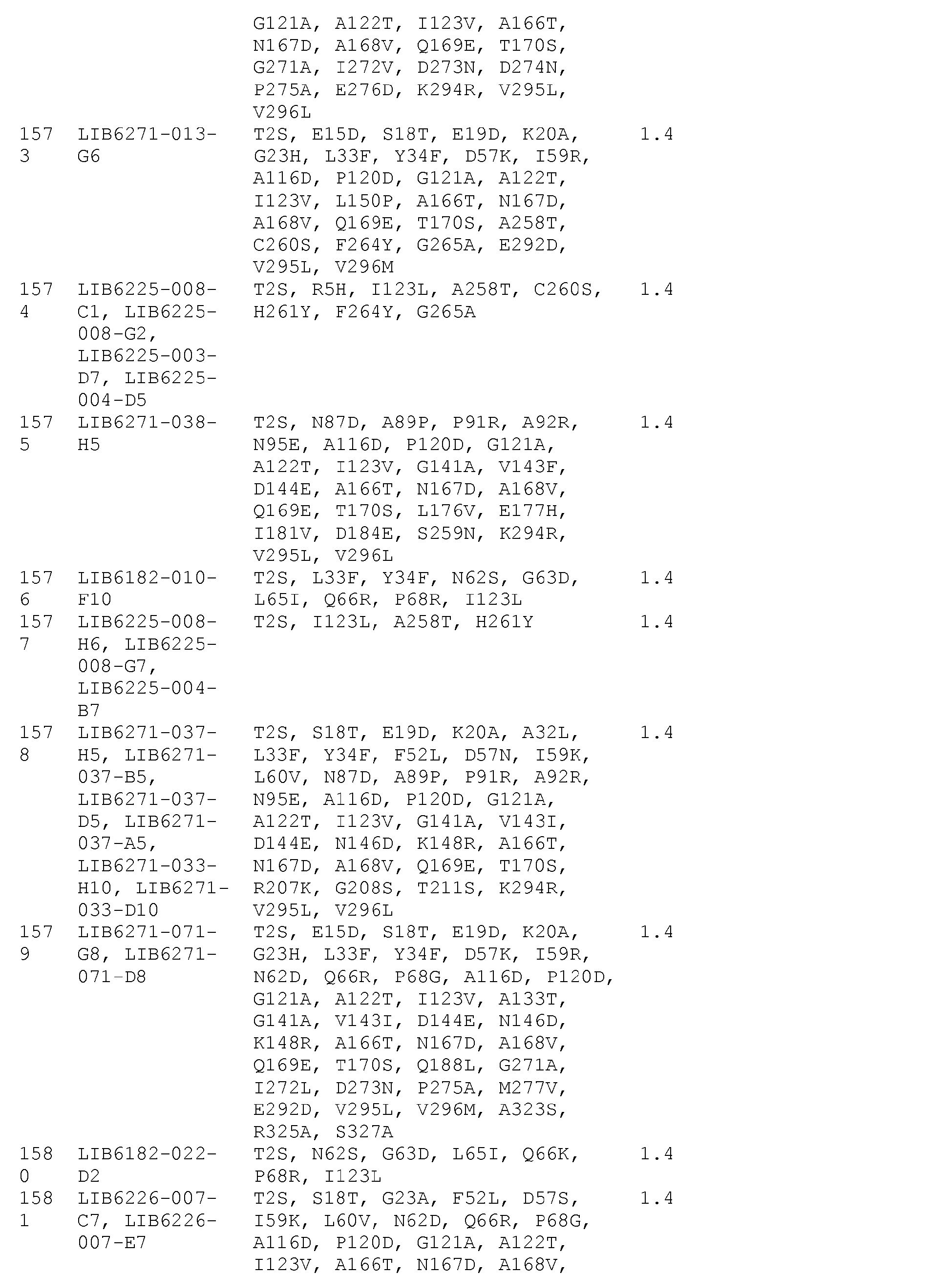

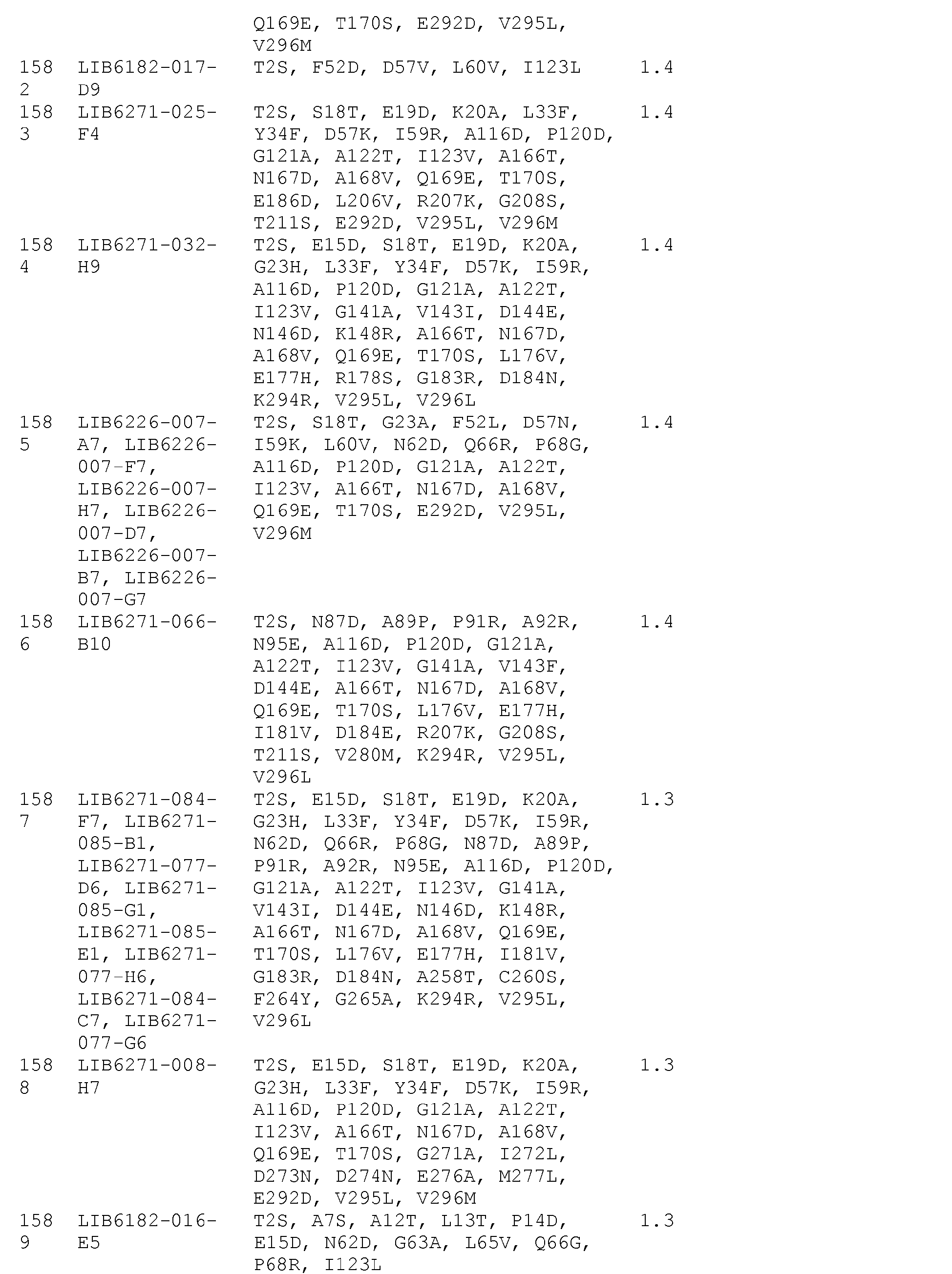

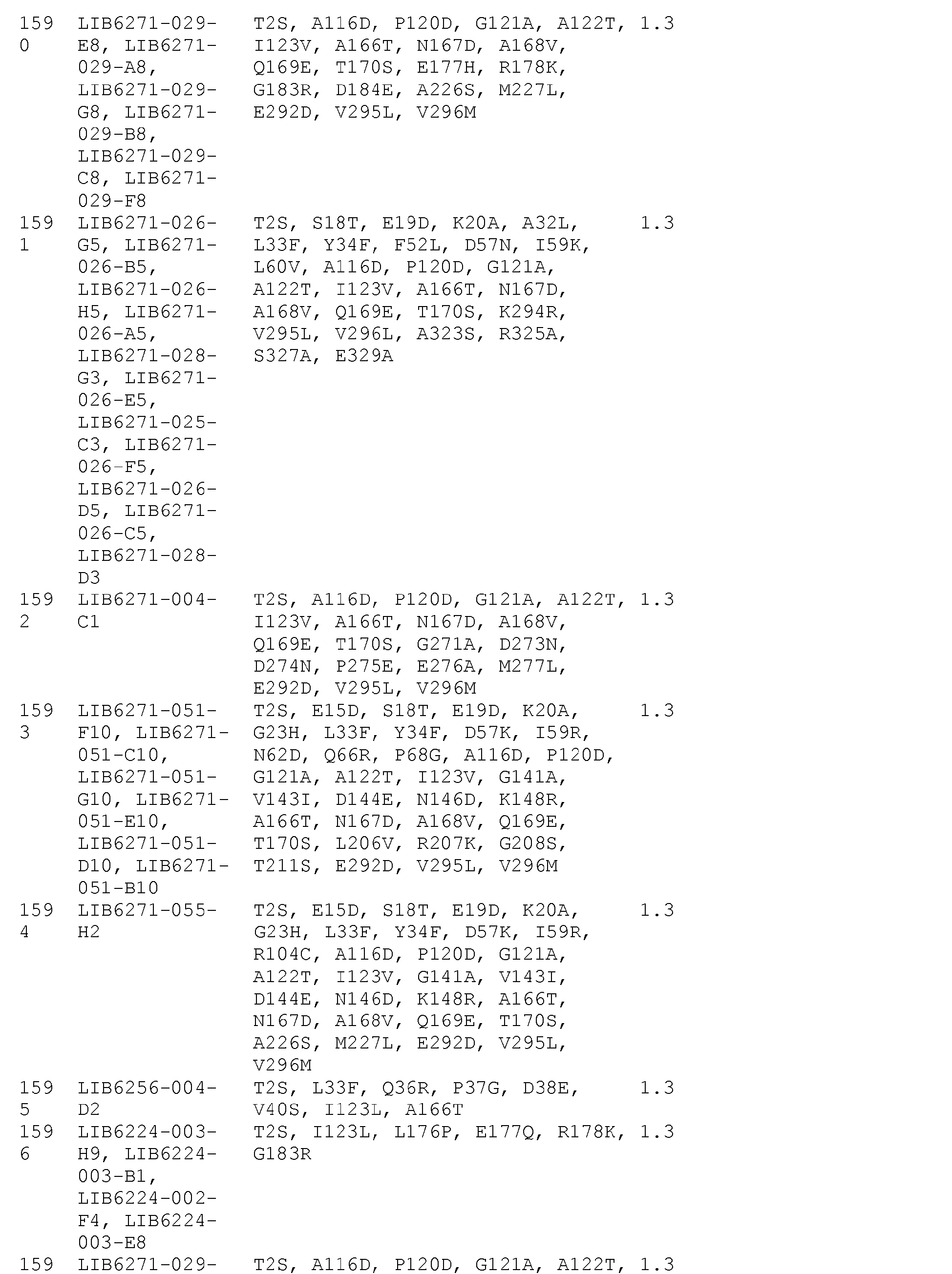

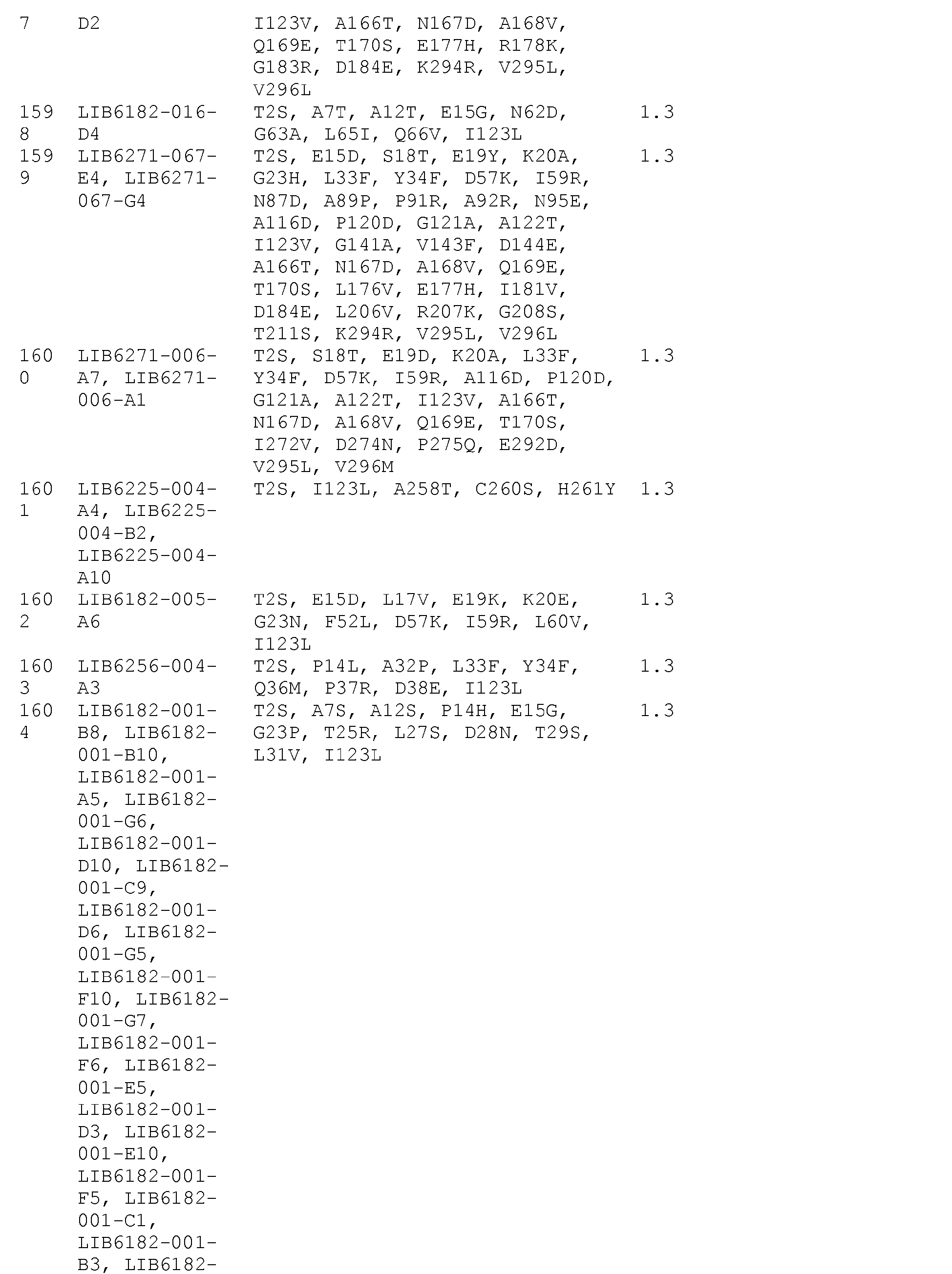

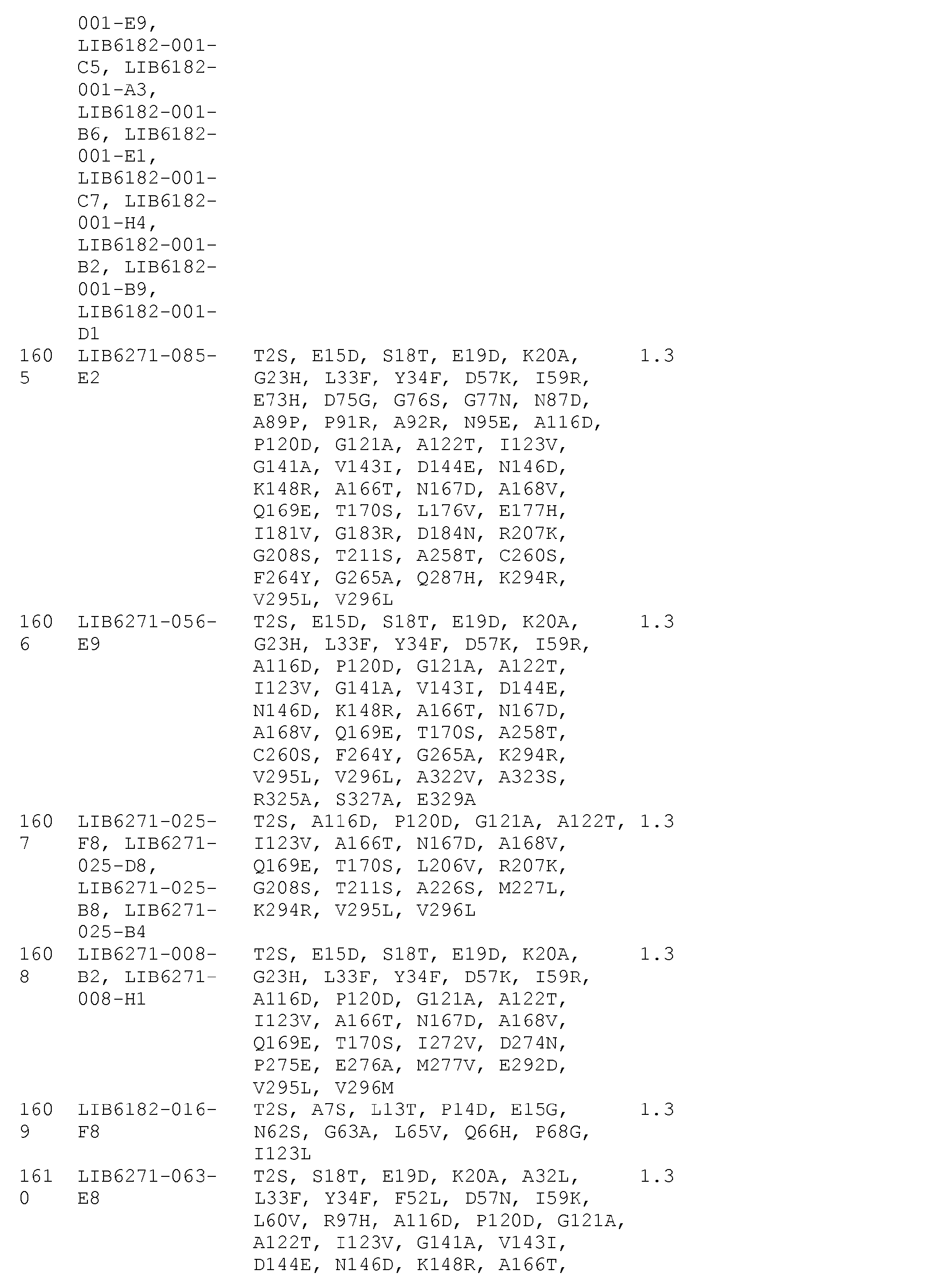

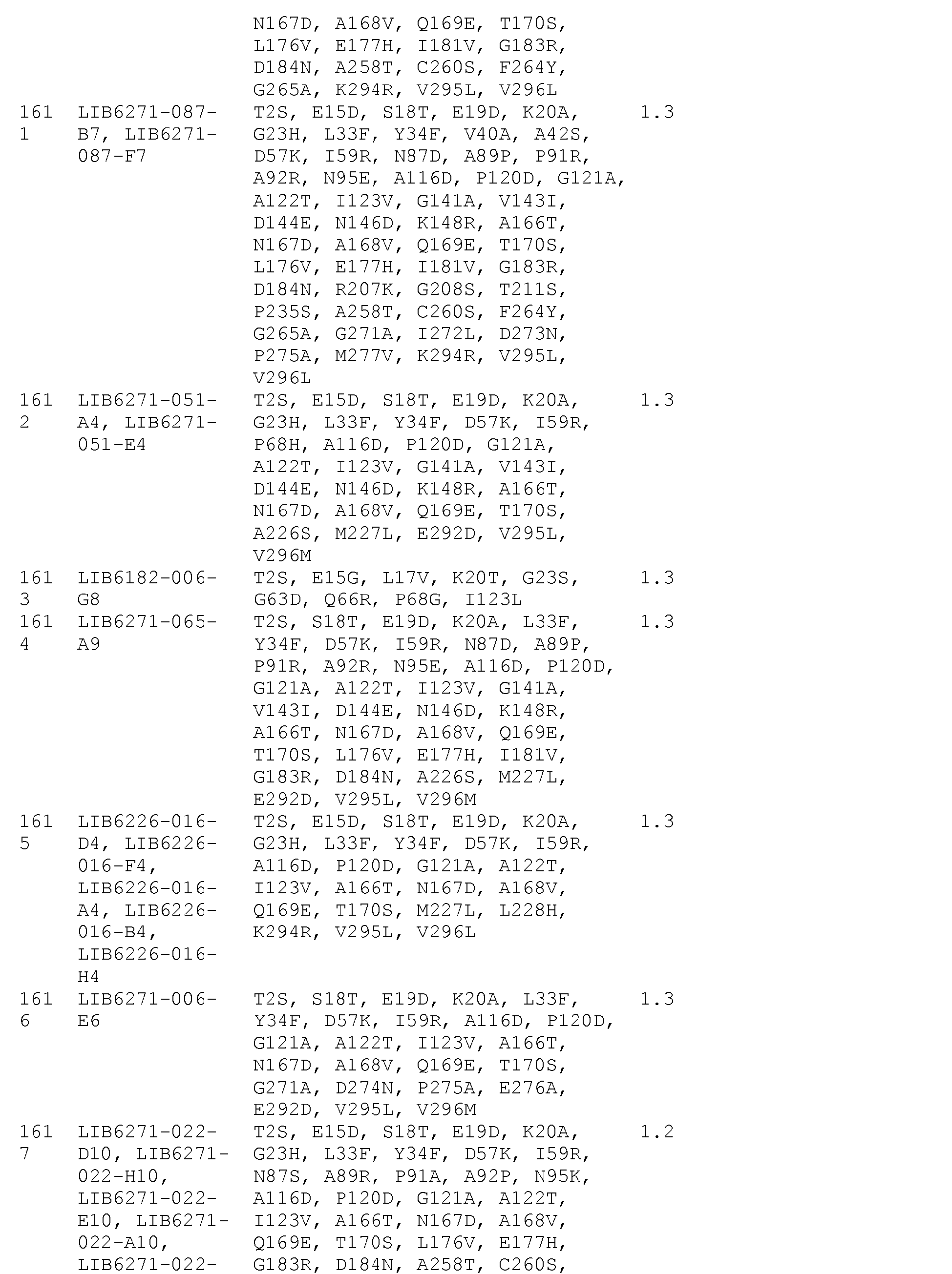

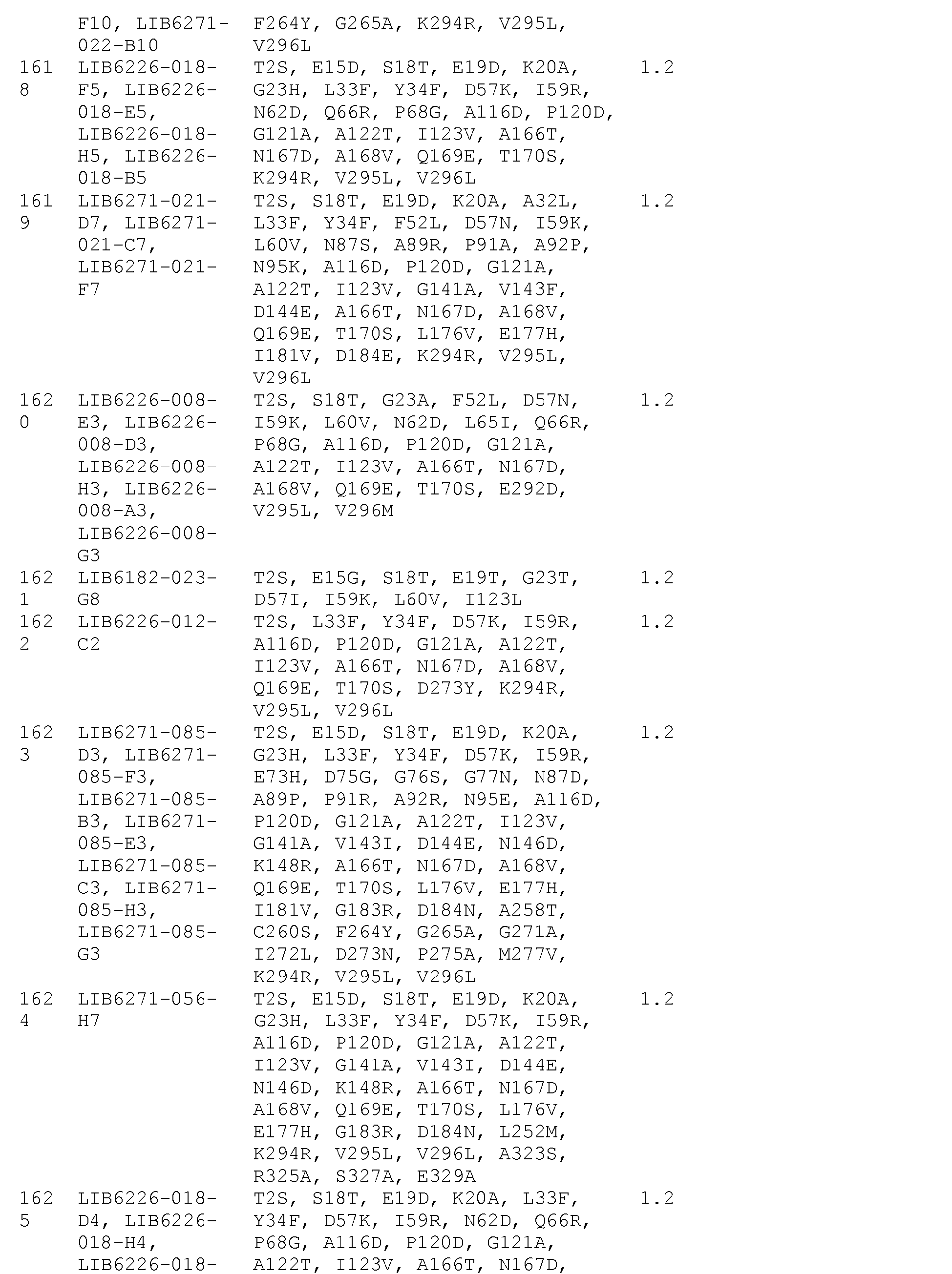

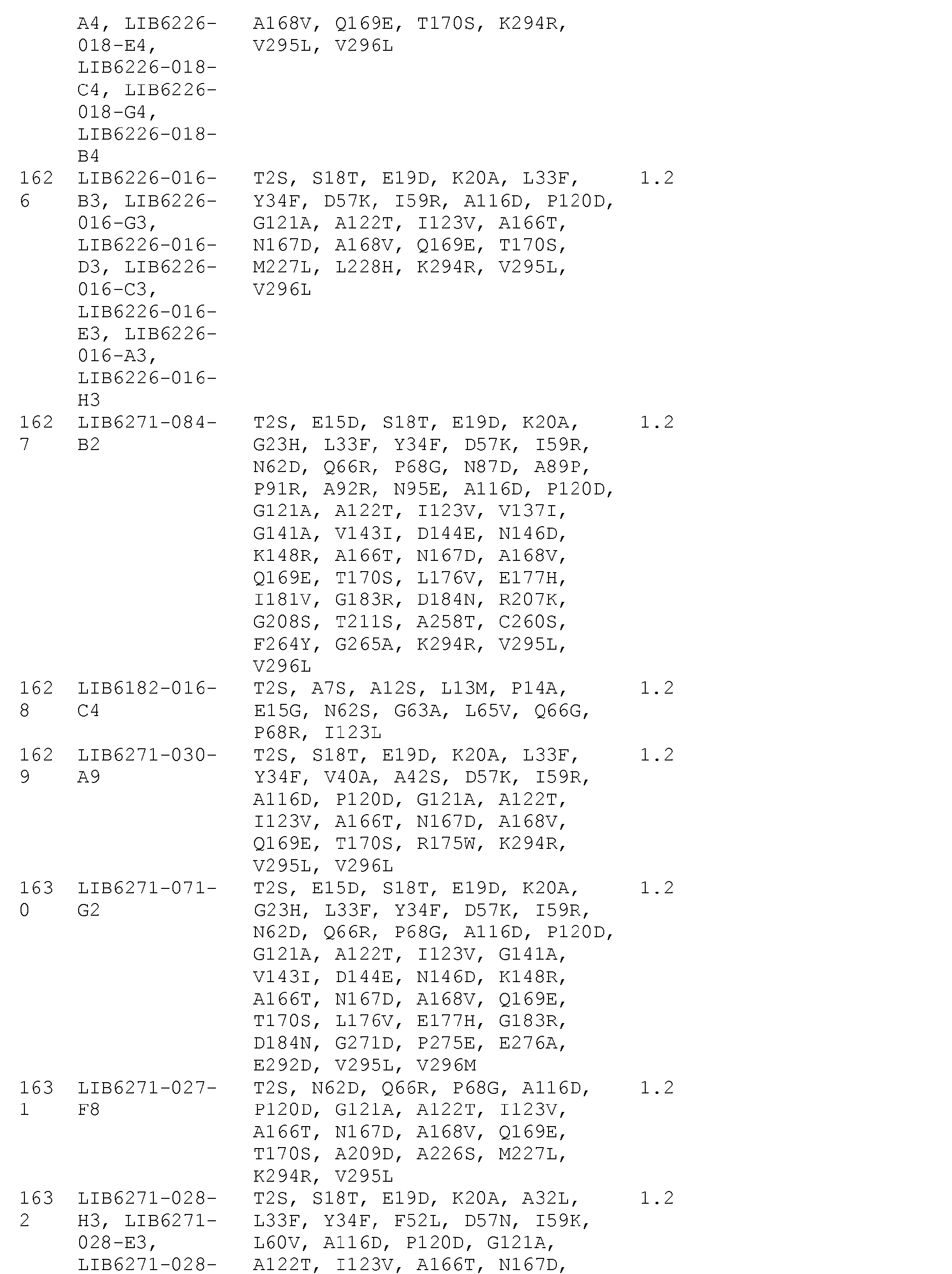

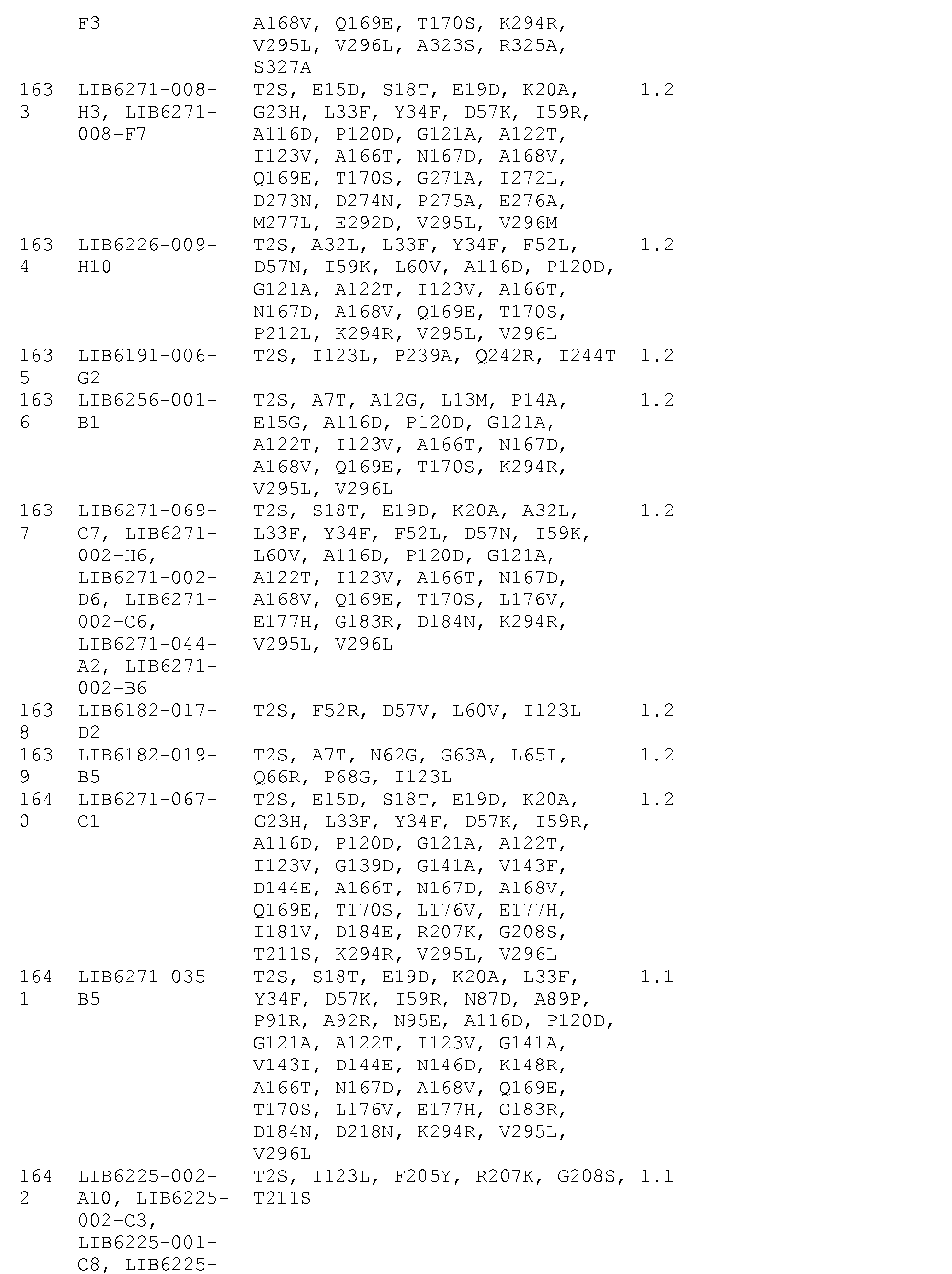

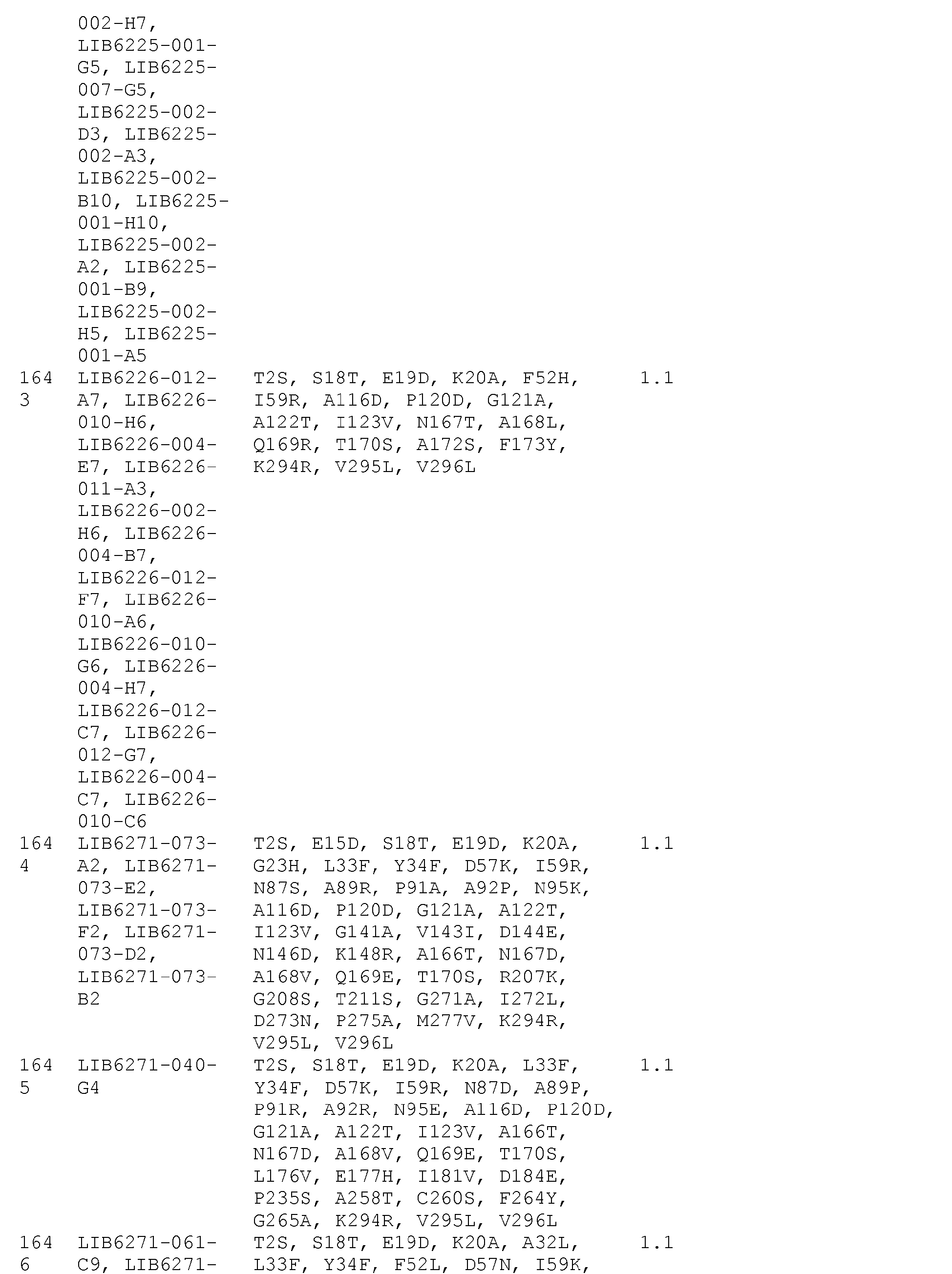

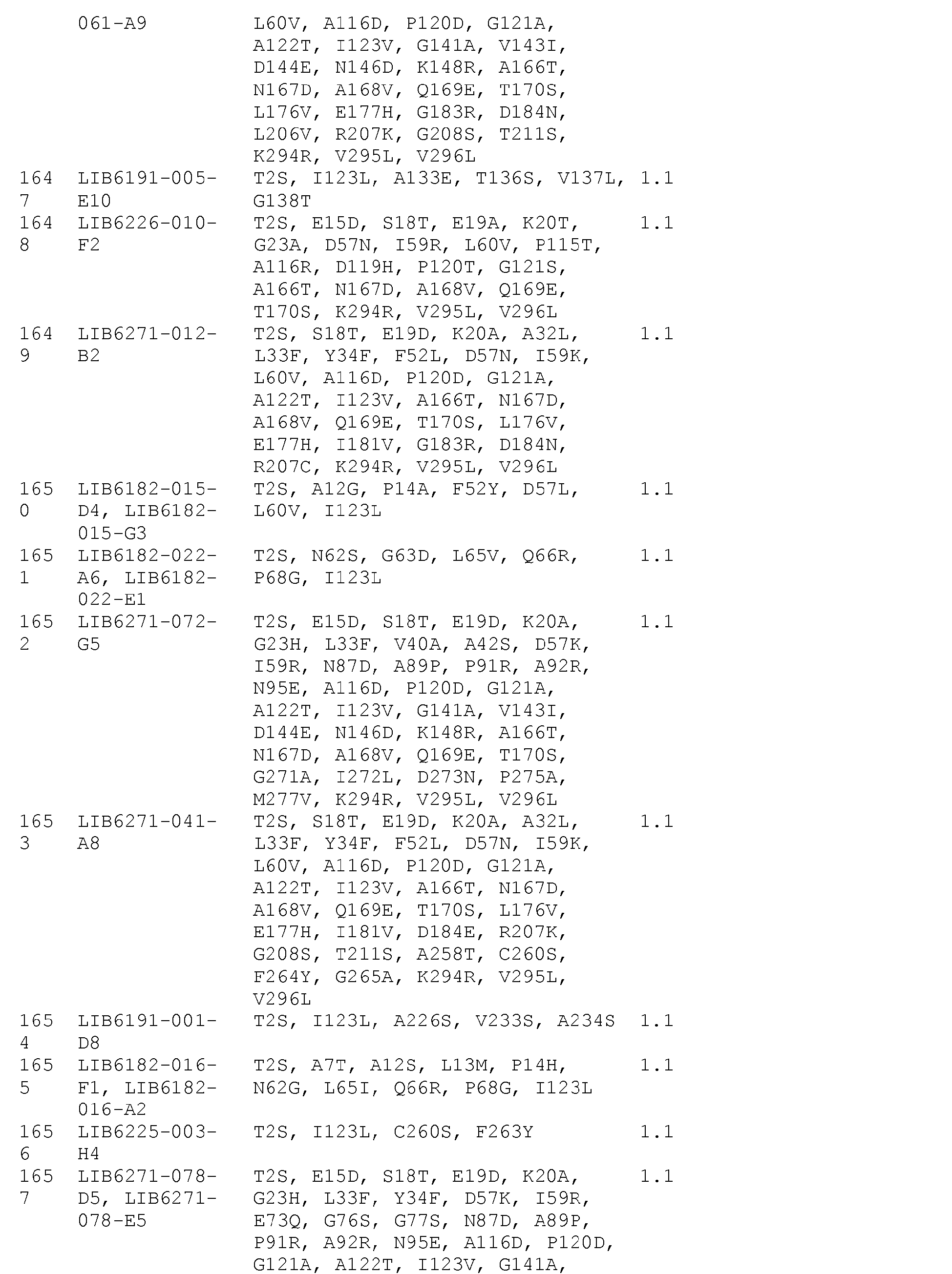

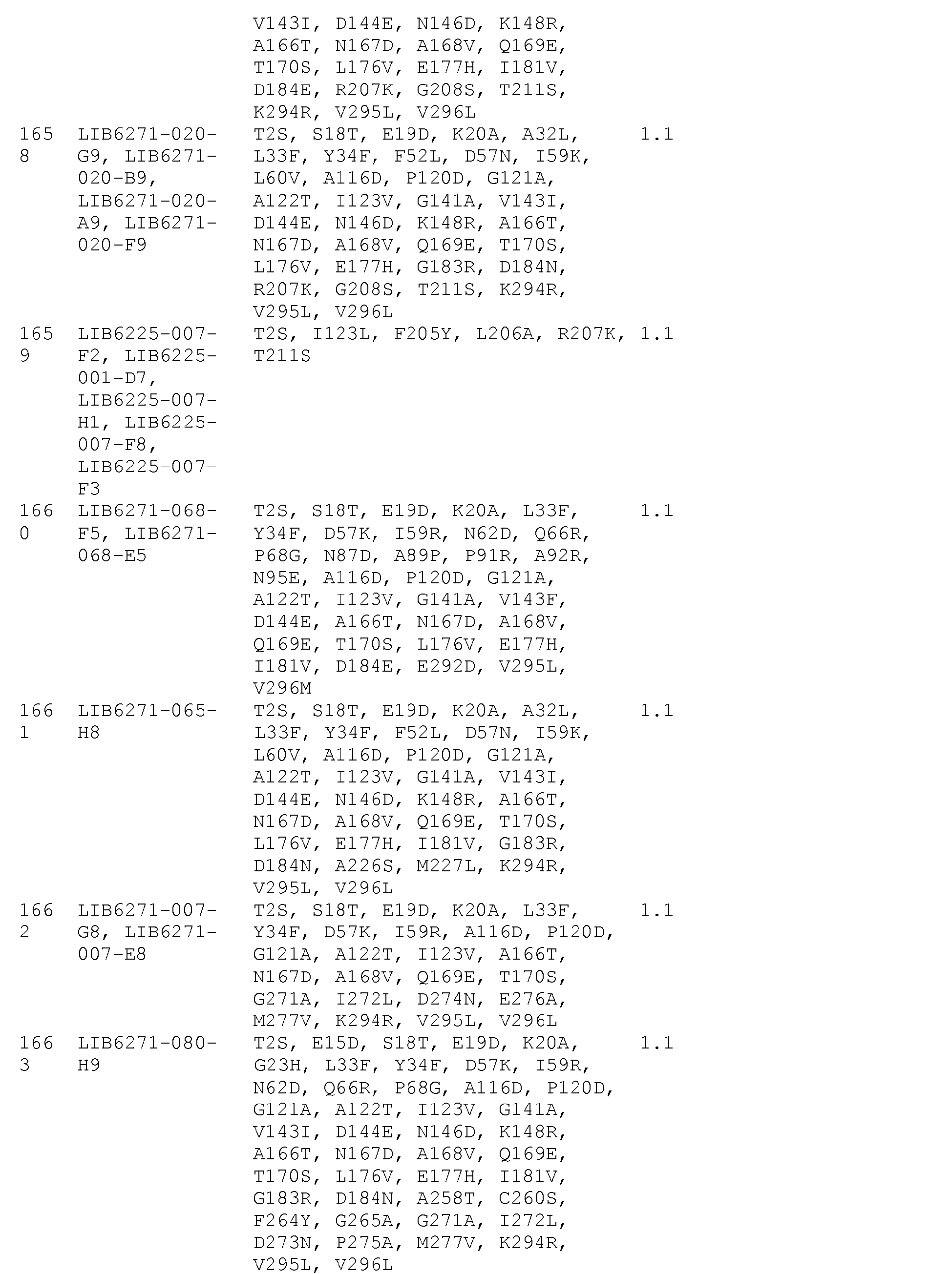

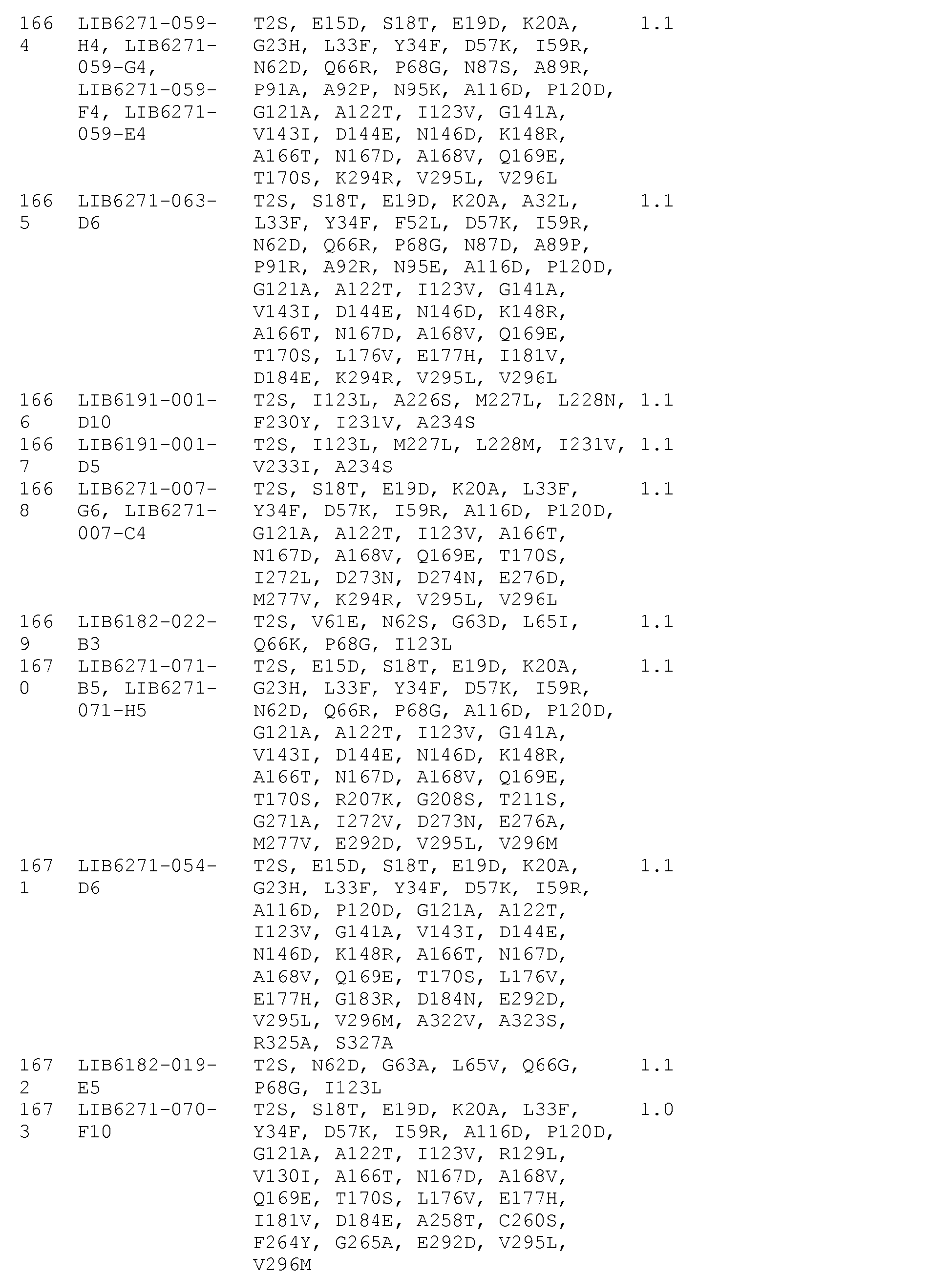

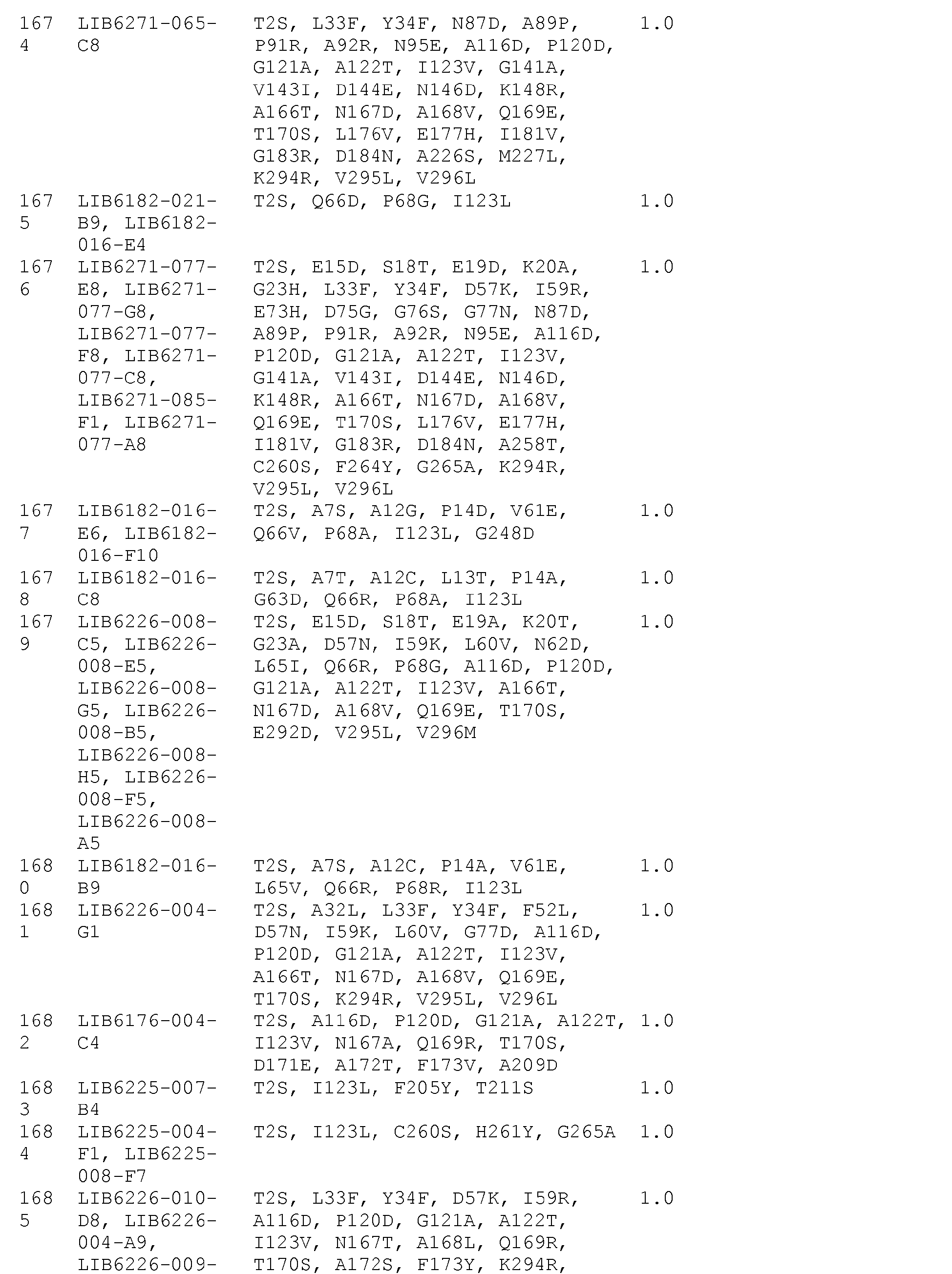

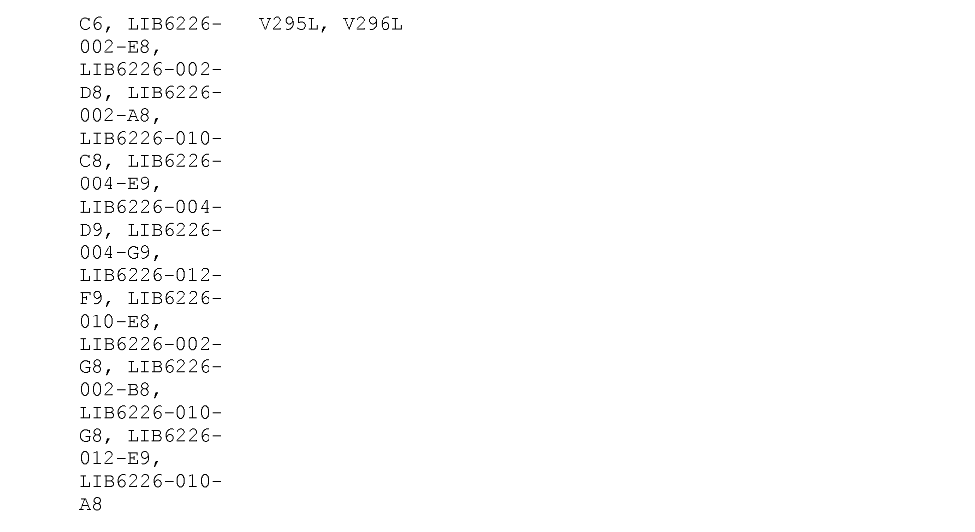

### REFERENCES

The references listed below are incorporated herein by reference to the extent that they supplement, explain, provide a background for, or teach methodology, techniques, and/or compositions employed herein.
U.S. Patents 4,554,101; 7,022,896
Abagyan and Batalov, J. Mol. Biol., 273:355-368, 1997.
Abagyan and Totrov, J. Mol. Biol., 268:678-685, 1997.
Altschul et al., J. Mol. Biol. 215:403-410, 1990.
Bate and Warwicker, J. Mol. Biol. 340:263-276, 2004.
Berman et al., Nucleic Acids Res., 28:235-242, 2000.
Berta et al., Structure, 13: 817-824, 2005.
Brünger, In: X-PLOR Version 3.1. A System for X-ray Crystallography and NMR, Yale University Press, 1992a.
Brünger, Nature, 355:472-474, 1992b.
Buswell and Ribbons, Meth. Enzymol. 161:294-301, 1988.
Carredano et al., J. Mol. Biol. 296:701, 2000.
Carson, J. Appl. Crystallogr., 24: 958-961, 1991.
Chakraborty et al., Arch. Biochem. Biophys., 437:20-28, 2005.
Chothia, J. Mol. Biol. 105:1-14, 1975.
Cleland, W.W., The Enzymes, Vol. XIX, p. 99-157, 1990.
Copeland R. A., The Enzymes ,Wiley, NY, 2000.
Dawson et al., In: Data for Biochemical Research, 411, Oxford Science Publications, 1986.
Dennis et al., PNAS 99:4290-4295, 2002.
Dong et al., J. Bacteriol., 187:2483-2490, 2005.
Edgar, BMC Bioinformatics, 5:113, 2004.
Ferraro et al., Biochem. Biophys. Res. Commun., 338:175-190, 2005.
Fersht, A. chapters 11-12 in "Enzyme Structure and Mechanism", 2nd ed. W.H. Freeman & Co., N.Y., 1985.
Ferraro et al., J. Bacteriol., 188:6986-6994, 2006.
Friemann et al., J. Mol. Biol., 348:1139-1151, 2005.
Furusawa et al., J. Mol. Biol. 342:1041, 2004.
Gakhar et al., J. Bacteriol., 187:7222-7231, 2005.
Gibson and Parales, Curr. Opin. Biotech., 11:236-243, 2000.
Good and Izawa, Methods Enzymol., 24:53-64, 1968.
Gutteridge and Thornton, Trends Biochem. Sci. 30:622-629, 2005.
Hendrickson, Science, 254:51-58, 1991.
Herman et al., J. Biol. Chem., 280(26):24759-24767, 2005.
Iwata et al., Structure, 4:567-579, 1996.
Johnson, K.A., The Enzymes, Vol XX, p. 1-61, 1992.
Jones et al., Acta Cryst., A47:110-119, 1991.
Junker et al., J. Bacteriol. 179:919-927, 1997.
Karplus, Protein Sci. 6:1302-1307, 1997.
Kauppi et al., Structure, 6:571-586, 1998.
Koehntop et al., J. Biol. Inorg. Chem. 10:87-93, 2005.
Kyte et al., J. Mol. Biol. 157:105-132, 1982.
Leahy et al., FEMS Microbiol Rev. 27:449-479, 2003.
Lichtarge and Sowa, Curr. Opin. Struct. Biol. 12:21-27, 2002.
Livingstone et al., CABIOS 9:745-756, 1993.
Matthews, J. Mol. Biol., 33:491-497, 1968.
May, Prot. Engineering 12:707-712, 1999.
McCoy et al., Acta Cryst., D61:458-464, 2005.
McPherson, pp. 94-97 in: Preparation and Analysis of Protein Crystals, John Wiley & Sons, NY, 1982.
Mocz, Protein Sci. 4:1178-1187, 1995.
Morawski et al., J. Bacteriol. 182:1383-1389, 2000.
Morris et al., Bioinformatics 21:2347-2355, 2005.
Navaza, Acta Cryst., A50:157-163, 1994.
Nojiri et al., J. Mol. Biol., 351: 355-370.
Ofran and Rost, J. Mol. Biol. 325:377-387, 2003.
Otwinowski and Minor, Methods Enzymol. 276:307-326, 1997.
Ramagopal et al., Acta Cryst., D59:1020-1027, 2003.
Russell et al. Curr. Opin. Struct. Biol. 14:313-324, 2004.
Sambrook et al., Molecular Cloning: A laboratory manual. Second Edition. Cold Spring Harbor Laboratory Press; 1989.
Shimoni et al., J. Biotechnol. 105:61-5 and 25-26,0, 2003.
Silberstein et al., J. Mol. Biol. 332:1095-1113, 2003.
Stanfel, J. Theor. Biol. 183:195-205, 1996.
Taylor, J. Theor. Biol. 119:205-218, 1986
Terwilliger and Berendzen, Acta Cryst., D55:849-861, 1999.
Terwilliger, Acta Cryst., D55:1863-1871, 1999.
Todd et al., J. Mol. Biol. 307:1113-1143, 2001.
Tsuchiya et al., Prot. Eng. Design Select. 19:412-429, 2006.
Wackett, Enzyme Microb. Technol. 31:577-587, 2002.
Wang et al., Appl. Environ. Microbiol. 63:1623-1626, 1997.
Zamyatin, Prog. Biophys. Mol. Biol. 24:107-123, 1972.

## Claims

1. A crystallized dicamba monooxygenase polypeptide comprising a sequence at least 85% identical to any of SEQ ID NO:1, SEQ ID NO:2, or SEQ ID NO:3.

2. A molecule comprising a binding surface for dicamba, that binds to dicamba with a K_{D} or K_{M} of between 0.1-500µM, wherein the molecule does not comprise an amino acid sequence of any of SEQ ID NOs:1-3.

3. The molecule of claim 2, wherein the K_{D} for dicamba is between 0.1-100µM.

4. The molecule of claim 2, further defined as a polypeptide.

5. An isolated polypeptide comprising dicamba monooxygenase (DMO) activity, wherein the polypeptide comprises a sequence selected from the group consisting of:
a) a polypeptide sequence that when in crystalline form comprises a space group of P3₂;
b) a polypeptide sequence that when in crystalline form comprises a binding site for a substrate, the binding site defined as comprising the characteristics of: (i) a volume of 175-SOOÅ³, (ii) electrostatically accommodative of a negatively charged carboxylate, (iii) accommodative of at least one chlorine moiety if present in the substrate, (iv) accommodative of a planar aromatic ring in the substrate, and (v) displays a distance from an iron atom that activates oxygen in the polypeptide to a carbon of the methoxy group of the substrate, sufficient for catalysis, of about 2.5Å to about 7Å;
c) a polypeptide sequence that when in crystalline form comprises a unit-cell parameter of a=79-81Å, b=79-81Å, and c=158-162 Å;
d) a polypeptide sequence that folds to produce a three-dimensional macromolecular structure **characterized by** the atomic structure coordinates of peptide backbone atoms of any of Tables 1-5, and 25-26, or a macromolecular structure that exhibits a root-mean-square difference (rmsd) in α-carbon positions of less than 2.0Å with the atomic structure coordinates of Tables 1-5, and 25-26, when superimposed on the corresponding backbone atoms described by the structure coordinates of amino acid residues comprising the polypeptide, when 70% or more of the total macromolecular structure α-carbon atoms are used in the superimposition;
e) a polypeptide sequence that folds to produce a three-dimensional macromolecular structure that has the same tertiary and quaternary fold as that **characterized by** the α-carbon coordinates for the structure represented in Tables 1-5, and 25-26;
f) a polypeptide sequence comprising substantially all of the amino acid residues corresponding to H51, A316, L318, C49, P55, V308, F53, D47, A54, L73, I48, I301, Y307, H86, R304, C320, A300, V297, N84, E322, P50, R52, C68, Y70, L95, P315, P31, T30, L46, I313, G87, D321, D29, N154, G89, S94, M317, H71, D157, G72, V296, V298, D58, D153, R314, and R98 of SEQ ID NO:2 or SEQ ID NO:3;
g) a polypeptide sequence comprising a Rieske center domain, further defined as comprising a polypeptide sequence that folds to produce a three-dimensional macromolecular structure **characterized by** the atomic structure coordinates of peptide backbone atoms of any of Tables 1-5, and 25-26, corresponding to amino acid residues 2-124 of SEQ ID NO:2 or SEQ ID NO:3, or a macromolecular structure that exhibits a root-mean-square difference (rmsd) in α-carbon positions of of less than 2.0Å with the atomic structure coordinates of peptide backbone atoms of any of Tables 1-5, and 25-26, corresponding to amino acid residues 2-124 of SEQ ID NO:2 or SEQ ID NO:3 when superimposed on the corresponding backbone atoms described by the structure coordinates of amino acid residues comprising the polypeptide, when 70% or more of the macromolecular structure α-carbon atoms corresponding to amino acid residues 2-124 of SEQ ID NO:2 or SEQ ID NO:3 are used in the superimposition; and
h) a polypeptide sequence comprising a DMO catalytic domain, further defined as comprising a polypeptide sequence that folds to produce a three-dimensional macromolecular structure **characterized by** the atomic structure coordinates of peptide backbone atoms of any of Tables 1-5, and 25-26, corresponding to amino acid residues 125-343 of SEQ ID NO:2 or SEQ ID NO:3, or a macromolecular structure that exhibits a root-mean-square difference (rmsd) in α-carbon positions of of less than 2.0Å with the atomic structure coordinates of peptide backbone atoms of any of Tables 1-5, and 25-26, corresponding to amino acid residues 125-343 of SEQ ID NO:2 or SEQ ID NO:3 when superimposed on the corresponding backbone atoms described by the structure coordinates of amino acid residues comprising the polypeptide, when 70% or more of the macromolecular structure α-carbon atoms corresponding to amino acid residues 125-343 of SEQ ID NO:2 or SEQ ID N0:3 are used in the superimposition;
wherein the polypeptide does not comprise the amino acid sequence of any of SEQ ID NOs:1-3.

6. The isolated polypeptide of claim 5, comprising the secondary structural elements of table 6 or table 8.

7. The isolated polypeptide of claim 5, defined as comprising a polypeptide sequence that when in crystalline form comprises a unit-cell parameter α=β= 90° and γ=120°.

8. The isolated polypeptide of claim 5, further defined as comprising one monomer per asymmetric unit.

9. The isolated polypeptide of claim 5, further defined as a crystal.

10. The isolated polypeptide of claim 5, defined as comprising a polypeptide sequence that when in crystalline form diffracts X-rays for a determination of atomic coordinates at a resolution higher than 3.2Å.

11. The isolated polypeptide of claim 10, wherein
(i) the resolution is about 3.0Å; or
(ii) the resolution is about 2.65Å; or
(iii) the resolution is about 1.9Å.

12. The isolated polypeptide of claim 5, wherein the presence of free iron enhances binding to dicamba.

13. The isolated polypeptide of claim 5, further defined as a folded polypeptide bound to a non-heme iron ion and comprising a Rieske center domain.

14. The isolated polypeptide of claim 5, further defined as a folded polypeptide bound to dicamba.

15. The isolated polypeptide of claim 5, wherein the polypeptide comprises an amino acid sequence with from about 20% to about 99% sequence identity to the polypeptide sequence of any of SEQ ID NOs:1-3.

16. The isolated polypeptide of claim 15, wherein the polypeptide comprises
(i) an amino acid sequence with less than about 95% identity to any of SEQ ID NOs:1-3; or
(ii) the polypeptide comprises an amino acid sequence with less than about 85% identity to any of SEQ ID NOs:1-3; or
(iii) the polypeptide comprises an amino acid sequence with less than about 65% identity to any of SEQ ID NOs:1-3; or
(iv) the polypeptide comprises an amino acid sequence with less than about 45% identity to any of SEQ ID NOs:1-3.

17. The isolated polypeptide of claim 5, wherein the polypeptide comprises a C-terminal domain for donating an electron to a Rieske center, and further comprises an electron transport path from a Rieske center to a catalytic site having a conserved surface with a macromolecular structure formed by the amino acid residues N154, D157 H160, H165, and D294, corresponding to SEQ ID NO:2 or SEQ ID NO:3, or conservative substitutions thereof.

18. The isolated polypeptide of claim 17, wherein the distance for iron FE2 to His71 ND1 is 2.57Å ±0.2-0.3Å; the distance for the His71 NE2 to Asp157 OD1 is 3.00Å ±0.2-0.3Å, the distance for Asp157 OD1 to His160 ND1 is 2.80Å ±0.2-0.3Å, and the distance for His160 NE2 to Fe is 2.43Å ±0.2-0.3Å.

19. The isolated polypeptide of claim 5, wherein the polypeptide comprises a subunit interface region having a conserved surface with a macromolecular structure formed by amino acid residues V325, E322, D321, C320, L318, M317, A316, P315, R314, I313, V308, Y307, R304, I301, A300, V297, V296, E293, R166, V164, Y163, H160, G159, D157, N154, D153, R98, L95, S94, G89, G87, H86, P85, N84, L73, G72, H71, Y70, P69, C68, Q67, D58, P55, A54, F53, R52, H51, P50,148, D47, L46, P31, T30, and D29, corresponding to SEQ ID NO:2 or SEQ ID NO:3, or conservative substitutions thereof.

20. The isolated polypeptide of claim 19, wherein the polypeptide comprises a motif of residues H51a:R52:F53a:Y70a:H71a:H86a:H160c:Y163c:R304c:Y307c:A316c:L318c numbered corresponding to SEQ ID NO:2 or SEQ ID NO:3.

21. The isolated polypeptide of claim 5, further defined as a homotrimer.

22. A plant cell comprising the polypeptide of claim 5.

23. A method for determining the three dimensional structure of a crystallized DMO polypeptide to a resolution of about 3.0Å or better comprising:
(a) obtaining a crystal according to claim 1; and
(b) analyzing the crystal to determine the three dimensional structure of crystallized DMO.

24. The method of claim 23, wherein analyzing comprises subjecting the crystal to diffraction analysis or spectrophotometric analysis.

25. A computer readable data storage medium encoded with computer readable data comprising atomic structural coordinates representing the three dimensional structure of crystallized DMO or a dicamba binding domain thereof.

26. The computer readable data storage medium of claim 25, wherein said computer readable data comprises atomic structural coordinates representing:
(a) a dicamba binding domain defined by structural coordinates of one or more residues according to any of Tables 1-5, 25-26, selected from the group consisting of L155, D157, L158, H160, A161, H165, R166, A169, Q170, D172, A173, A216, W217, N218, I220, N230, I232, A233, V234, S247, R248, G249, T250, H251, Y263, F265, G266, S267, L282, W285, Q286, A287, Q288, A289, L290, and V291 numbered corresponding to SEQ ID NO:2, or conservative substitutions thereof;
(b) an interface domain defined by structure coordinates of one or more residues according to any of Tables 1-5, and 25-26, selected from the group consisting of V325, E322, D321, C320, L318, M317, A316, P315, R314, 1313, V308, Y307, R304, I301, A300, V297, V296, E293, R166, V164, Y163, H160, G159, D157, N154, D153, R98, L95, S94, A93, G89, G87, H86, P85, N84, L73, G72, H71, Y70, P69, C68, Q67, D58, P55, A54, F53, R52, H51, P50, I48, D47, L46, P31, T30, and D29, numbered corresponding to SEQ ID NO:2 or SEQ ID NO:3, or conservative substitutions thereof;
(c) an electron transport path from a Rieske center to a catalytic site defined by structure coordinates of one or more residues according to any of Tables 1-5, and 25-26, selected from the group consisting of N154, D157 H160, H165, and D294, numbered corresponding to SEQ ID NO:2 or SEQ ID NO:3, or conservative substitutions thereof;
(d) a C-terminal domain defined by structure coordinates of one or more residues according to any of Tables 1-5, and 25-26, selected from the group consisting of A323, A324, V325, R326, V327, S328, R329, E330, I331, E332, K333, L334, E335, Q336, L337, E338, A339, A340 numbered corresponding to SEQ ID NO:2 or SEQ ID NO:3; or
(e) a domain of any of (a)- (d) exhibiting a root mean square deviation of amino acid residues, comprising α-carbon backbone atoms, of less than 2Å with the atomic structure coordinates of any of Tables 1-5, and 25-26, when superimposed on the backbone atoms described by the structure coordinates of said amino acids when 70% or more of the macromolecular structure α-carbon atoms are used in the superimposition.

27. The computer readable data storage medium of claim 25, comprising the structural coordinates of any of Tables 1-5, and 25-26.

28. A computer programmed to produce a three-dimensional representation of the data comprised on the computer readable data storage medium of claim 25.

29. The isolated polypeptide of claim 5, wherein the polypeptide comprises
(i) a DMO enzyme having the sequence domain: -W-X₁-X₂-X₃-X₄-L- (SEQ ID NO:152), in which X₁ is Q, F, or H; X₂ is A, D, F, I, R, T, V, W, Y, C, E, G, L, M, Q, or S; X₃ is Q, G, I, V, A, C, D, H, L, M, N, R, S, T, or E; and X₄ is A, C, G, or S; and/or;
(ii) the polypeptide comprises a DMO enzyme having the sequence domain: -N-X₁-Q-, in which X₁ is A, L, C, F, F, I, N, Q, S, V, W, Y, M or T; and/or
(iii) the polypeptide comprises a DMO enzyme having the sequence domain: -W-X₁-D- in which X₁ is N, K, A, C, E, I, L, S, T, W, Y, H, or M; and/or
(iv) the polypeptide comprises a DMO enzyme having the sequence domain: -X₁-X₂-G-X₃-H- (SEQ ID NO: 153) in which X₁ is S, H, or T; X₂ is R, Q, S, T, F, H, N, V, W, Y, C, I, K, L, or M; and and X₃ is T, Q, or M.

30. The isolated polypeptide of claim 5, wherein the polypeptide exhibits an increased level of DMO activity relative to the activity of a wild type DMO.

31. The isolated polypeptide of claim 30, wherein the polypeptide comprises
(i) a substitution at residue R248, numbered according to the numbering of SEQ ID NO:2 or SEQ ID NO:3, selected from the group consisting of: R248C, R248I, R248K, R248L, R248M; and/or
(ii) a DMO enzyme comprising one or more substitution(s) at at least one residue numbered according to the numbering of SEQ ID NO:2 or SEQ ID NO:3, selected from the group consisting of:A169M, N218H, N218M, G266S, L282I, A287C, A287E, A287M, A287S, and Q288E.


SEQUENCE LISTING

<110>  Monsanto Technology LLC

<120>  DMO METHODS AND COMPOSITIONS

<130>  080084ep

<150>  60/884,854
<151>  2007-01-12

<150>  60/939,278
<151>  2007-05-21

<160>  154

<170>  PatentIn version 3.3


<210>  1
<211>  339
<212>  PRT
<213>  Pseudomonas maltophilia

<400>  1

Met Thr Phe Val Arg Asn Ala Trp Tyr Val Ala Ala Leu Pro Glu Glu
1               5                   10                  15


Leu Ser Glu Lys Pro Leu Gly Arg Thr Ile Leu Asp Thr Pro Leu Ala
            20                  25                  30


Leu Tyr Arg Gln Pro Asp Gly Val Val Ala Ala Leu Leu Asp Ile Cys
        35                  40                  45


Pro His Arg Phe Ala Pro Leu Ser Asp Gly Ile Leu Val Asn Gly His
    50                  55                  60


Leu Gln Cys Pro Tyr His Gly Leu Glu Phe Asp Gly Gly Gly Gln Cys
65                  70                  75                  80


Val His Asn Pro His Gly Asn Gly Ala Arg Pro Ala Ser Leu Asn Val
                85                  90                  95


Arg Ser Phe Pro Val Val Glu Arg Asp Ala Leu Ile Trp Ile Trp Pro
            100                 105                 110


Gly Asp Pro Ala Leu Ala Asp Pro Gly Ala Ile Pro Asp Phe Gly Cys
        115                 120                 125


Arg Val Asp Pro Ala Tyr Arg Thr Val Gly Gly Tyr Gly His Val Asp
    130                 135                 140


Cys Asn Tyr Lys Leu Leu Val Asp Asn Leu Met Asp Leu Gly His Ala

145                 150                 155                 160

Gln Tyr Val His Arg Ala Asn Ala Gln Thr Asp Ala Phe Asp Arg Leu
                165                 170                 175

Glu Arg Glu Val Ile Val Gly Asp Gly Glu Ile Gln Ala Leu Met Lys
            180                 185                 190

Ile Pro Gly Gly Thr Pro Ser Val Leu Met Ala Lys Phe Leu Arg Gly
        195                 200                 205

Ala Asn Thr Pro Val Asp Ala Trp Asn Asp Ile Arg Trp Asn Lys Val
    210                 215                 220

Ser Ala Met Leu Asn Phe Ile Ala Val Ala Pro Glu Gly Thr Pro Lys
225                 230                 235                 240

Glu Gln Ser Ile His Ser Arg Gly Thr His Ile Leu Thr Pro Glu Thr
                245                 250                 255

Glu Ala Ser Cys His Tyr Phe Phe Gly Ser Ser Arg Asn Phe Gly Ile
            260                 265                 270

Asp Asp Pro Glu Met Asp Gly Val Leu Arg Ser Trp Gln Ala Gln Ala
        275                 280                 285

Leu Val Lys Glu Asp Lys Val Val Val Glu Ala Ile Glu Arg Arg Arg
    290                 295                 300

Ala Tyr Val Glu Ala Asn Gly Ile Arg Pro Ala Met Leu Ser Cys Asp
305                 310                 315                 320

Glu Ala Ala Val Arg Val Ser Arg Glu Ile Glu Lys Leu Glu Gln Leu
                325                 330                 335

Glu Ala Ala

<210>  2
<211>  349
<212>  PRT
<213>  Artificial

<220>
<223>  Synthetic peptide

<400>  2

Met Ala Thr Phe Val Arg Asn Ala Trp Tyr Val Ala Ala Leu Pro Glu

1               5                   10                  15

Glu Leu Ser Glu Lys Pro Leu Gly Arg Thr Ile Leu Asp Thr Pro Leu
            20                  25                  30

Ala Leu Tyr Arg Gln Pro Asp Gly Val Val Ala Ala Leu Leu Asp Ile
        35                  40                  45

Cys Pro His Arg Phe Ala Pro Leu Ser Asp Gly Ile Leu Val Asn Gly
    50                  55                  60

His Leu Gln Cys Pro Tyr His Gly Leu Glu Phe Asp Gly Gly Gly Gln
65                  70                  75                  80

Cys Val His Asn Pro His Gly Asn Gly Ala Arg Pro Ala Ser Leu Asn
                85                  90                  95

Val Arg Ser Phe Pro Val Val Glu Arg Asp Ala Leu Ile Trp Ile Cys
            100                 105                 110

Pro Gly Asp Pro Ala Leu Ala Asp Pro Gly Ala Ile Pro Asp Phe Gly
        115                 120                 125

Cys Arg Val Asp Pro Ala Tyr Arg Thr Val Gly Gly Tyr Gly His Val
    130                 135                 140

Asp Cys Asn Tyr Lys Leu Leu Val Asp Asn Leu Met Asp Leu Gly His
145                 150                 155                 160

Ala Gln Tyr Val His Arg Ala Asn Ala Gln Thr Asp Ala Phe Asp Arg
                165                 170                 175

Leu Glu Arg Glu Val Ile Val Gly Asp Gly Glu Ile Gln Ala Leu Met
            180                 185                 190

Lys Ile Pro Gly Gly Thr Pro Ser Val Leu Met Ala Lys Phe Leu Arg
        195                 200                 205

Gly Ala Asn Thr Pro Val Asp Ala Trp Asn Asp Ile Arg Trp Asn Lys
    210                 215                 220

Val Ser Ala Met Leu Asn Phe Ile Ala Val Ala Pro Glu Gly Thr Pro
225                 230                 235                 240

Lys Glu Gln Ser Ile His Ser Arg Gly Thr His Ile Leu Thr Pro Glu
                245                 250                 255

Thr Glu Ala Ser Cys His Tyr Phe Phe Gly Ser Ser Arg Asn Phe Gly
            260                 265                 270

Ile Asp Asp Pro Glu Met Asp Gly Val Leu Arg Ser Trp Gln Ala Gln
        275                 280                 285

Ala Leu Val Lys Glu Asp Lys Val Val Val Glu Ala Ile Glu Arg Arg
    290                 295                 300

Arg Ala Tyr Val Glu Ala Asn Gly Ile Arg Pro Ala Met Leu Ser Cys

305                 310                 315                 320

Asp Glu Ala Ala Val Arg Val Ser Arg Glu Ile Glu Lys Leu Glu Gln
                325                 330                 335

Leu Glu Ala Ala Arg Leu Glu His His His His His His
            340                 345

<210>  3
<211>  349
<212>  PRT
<213>  Artificial

<220>
<223>  Synthetic peptide

<400>  3

Met Ala Thr Phe Val Arg Asn Ala Trp Tyr Val Ala Ala Leu Pro Glu
1               5                   10                  15

Glu Leu Ser Glu Lys Pro Leu Gly Arg Thr Ile Leu Asp Thr Pro Leu
            20                  25                  30

Ala Leu Tyr Arg Gln Pro Asp Gly Val Val Ala Ala Leu Leu Asp Ile
        35                  40                  45

Cys Pro His Arg Phe Ala Pro Leu Ser Asp Gly Ile Leu Val Asn Gly
    50                  55                  60

His Leu Gln Cys Pro Tyr His Gly Leu Glu Phe Asp Gly Gly Gly Gln
65                  70                  75                  80

Cys Val His Asn Pro His Gly Asn Gly Ala Arg Pro Ala Ser Leu Asn
                85                  90                  95

Val Arg Ser Phe Pro Val Val Glu Arg Asp Ala Leu Ile Trp Ile Trp
            100                 105                 110

Pro Gly Asp Pro Ala Leu Ala Asp Pro Gly Ala Ile Pro Asp Phe Gly
        115                 120                 125

Cys Arg Val Asp Pro Ala Tyr Arg Thr Val Gly Gly Tyr Gly His Val
    130                 135                 140

Asp Cys Asn Tyr Lys Leu Leu Val Asp Asn Leu Met Asp Leu Gly His
145                 150                 155                 160

Ala Gln Tyr Val His Arg Ala Asn Ala Gln Thr Asp Ala Phe Asp Arg
                165                 170                 175

Leu Glu Arg Glu Val Ile Val Gly Asp Gly Glu Ile Gln Ala Leu Met
            180                 185                 190

Lys Ile Pro Gly Gly Thr Pro Ser Val Leu Met Ala Lys Phe Leu Arg
        195                 200                 205

Gly Ala Asn Thr Pro Val Asp Ala Trp Asn Asp Ile Arg Trp Asn Lys
    210                 215                 220

Val Ser Ala Met Leu Asn Phe Ile Ala Val Ala Pro Glu Gly Thr Pro
225                 230                 235                 240

Lys Glu Gln Ser Ile His Ser Arg Gly Thr His Ile Leu Thr Pro Glu
                245                 250                 255

Thr Glu Ala Ser Cys His Tyr Phe Phe Gly Ser Ser Arg Asn Phe Gly
            260                 265                 270

Ile Asp Asp Pro Glu Met Asp Gly Val Leu Arg Ser Trp Gln Ala Gln
        275                 280                 285

Ala Leu Val Lys Glu Asp Lys Val Val Val Glu Ala Ile Glu Arg Arg
    290                 295                 300

Arg Ala Tyr Val Glu Ala Asn Gly Ile Arg Pro Ala Met Leu Ser Cys
305                 310                 315                 320

Asp Glu Ala Ala Val Arg Val Ser Arg Glu Ile Glu Lys Leu Glu Gln
                325                 330                 335

Leu Glu Ala Ala Arg Leu Glu His His His His His His
            340                 345

<210>  4
<211>  350

<212>  PRT
<213>  Ralstonia eutropha

<400>  4

Met Phe Val Arg Asn Thr Trp Tyr Val Ala Gly Trp Asp Asn Glu Val
1               5                   10                  15

Gly Ala Ser Asn Leu Phe Ser Arg Thr Ile Ile Gly Ile Pro Val Leu
            20                  25                  30

Met Tyr Arg Ala Glu Asp Gly Thr Leu His Ala Leu Glu Asp Arg Cys
        35                  40                  45

Cys His Arg Gly Ala Pro Leu Ser Ile Gly Arg Arg Glu Gly Asp Cys
    50                  55                  60

Val Arg Cys Met Tyr His Gly Leu Lys Phe Asp Thr Ser Gly Arg Cys
65                  70                  75                  80

Ile Glu Ala Pro Ala Gln Gln Arg Ile Pro Pro Gln Ala Arg Val Arg
                85                  90                  95

Val Leu Pro Val Val Glu Arg Asn Arg Trp Ile Trp Ile Trp Met Gly
            100                 105                 110

Asp Pro Glu Ala Ala Asp Pro Ala Leu Ile Pro Asp Thr His Trp Leu
        115                 120                 125

Ala Asp Pro Ala Trp Arg Ser Leu Asp Gly Tyr Ile His Tyr Asp Val
    130                 135                 140

Asn Tyr Leu Leu Ile Ala Asp Asn Leu Leu Asp Phe Ser His Leu Pro
145                 150                 155                 160

Phe Val His Pro Thr Thr Leu Gly Gly Ser Glu Ala Tyr Ala Ala Ala
                165                 170                 175

Gln Pro Lys Val Glu Arg Leu Asp Asp Gly Val Arg Ile Thr Arg Trp
            180                 185                 190

Thr Leu Asn Thr Glu Ala Pro Pro Phe Ala Lys Gln Val Lys Asn Trp
        195                 200                 205

Pro Gly Lys Val Asp Arg Trp Asn Ile Tyr Asn Phe Thr Ile Pro Ala
    210                 215                 220

Ile Leu Leu Met Asp Ser Gly Met Ala Pro Thr Gly Thr Gly Ala Pro
225                 230                 235                 240

Glu Gly Gln Arg Ile Asp Ala Ala Glu Phe Arg Gly Cys Gln Ala Leu
                245                 250                 255

Thr Pro Glu Thr Glu Asn Ser Thr His Tyr Phe Phe Ala His Pro His
            260                 265                 270

Asn Phe Ala Ile Asp Asn Pro Glu Val Thr Arg Ser Ile His Gln Ser
        275                 280                 285

Val Val Thr Ala Phe Asp Glu Asp Arg Asp Ile Ile Thr Ala Gln Gln
    290                 295                 300

Arg Ser Leu Ala Leu Ala Pro Asp Phe Lys Met Val Pro Phe Ser Ile
305                 310                 315                 320

Asp Ala Ala Leu Ser Gln Phe Arg Trp Val Val Glu Arg Arg Val Ala
                325                 330                 335

Asp Glu Ala Ala Gln Ala Gln Gln Arg Gln Ala Lys Glu Ala
            340                 345                 350

<210>  5
<211>  347
<212>  PRT
<213>  Comamonas testosteroni

<400>  5

Met Phe Ile Arg Asn Cys Trp Tyr Val Ala Ala Trp Asp Thr Glu Ile
1               5                   10                  15

Pro Ala Glu Gly Leu Phe His Arg Thr Leu Leu Asn Glu Pro Val Leu
            20                  25                  30

Leu Tyr Arg Asp Thr Gln Gly Arg Val Val Ala Leu Glu Asn Arg Cys
        35                  40                  45

Cys His Arg Ser Ala Pro Leu His Ile Gly Arg Gln Glu Gly Asp Cys
    50                  55                  60

Val Arg Cys Leu Tyr His Gly Leu Lys Phe Asn Pro Ser Gly Ala Cys
65                  70                  75                  80

Val Glu Ile Pro Gly Gln Glu Gln Ile Pro Pro Lys Thr Cys Ile Lys
                85                  90                  95

Ser Tyr Pro Val Val Glu Arg Asn Arg Leu Val Trp Ile Trp Met Gly
            100                 105                 110

Asp Pro Ala Arg Ala Asn Pro Asp Asp Ile Val Asp Tyr Phe Trp His
        115                 120                 125

Asp Ser Pro Glu Trp Arg Met Lys Pro Gly Tyr Ile His Tyr Gln Ala
    130                 135                 140

Asn Tyr Lys Leu Ile Val Asp Asn Leu Leu Asp Phe Thr His Leu Ala
145                 150                 155                 160

Trp Val His Pro Thr Thr Leu Gly Thr Asp Ser Ala Ala Ser Leu Lys
                165                 170                 175

Pro Val Ile Glu Arg Asp Thr Thr Gly Thr Gly Lys Leu Thr Ile Thr
            180                 185                 190

Arg Trp Tyr Leu Asn Asp Asp Met Ser Asn Leu His Lys Gly Val Ala
        195                 200                 205

Lys Phe Glu Gly Lys Ala Asp Arg Trp Gln Ile Tyr Gln Trp Ser Pro
    210                 215                 220

Pro Ala Leu Leu Arg Met Asp Thr Gly Ser Ala Pro Thr Gly Thr Gly
225                 230                 235                 240

Ala Pro Glu Gly Arg Arg Val Pro Glu Ala Val Gln Phe Arg His Thr
                245                 250                 255

Ser Ile Gln Thr Pro Glu Thr Glu Thr Thr Ser His Tyr Trp Phe Cys
            260                 265                 270

Gln Ala Arg Asn Phe Asp Leu Asp Asp Glu Ala Leu Thr Glu Lys Ile
        275                 280                 285

Tyr Gln Gly Val Val Val Ala Phe Glu Glu Asp Arg Thr Met Ile Glu
    290                 295                 300

Ala His Glu Lys Ile Leu Ser Gln Val Pro Asp Arg Pro Met Val Pro
305                 310                 315                 320

Ile Ala Ala Asp Ala Gly Leu Asn Gln Gly Arg Trp Leu Leu Asp Arg
                325                 330                 335

Leu Leu Lys Ala Glu Asn Gly Gly Thr Ala Pro
            340                 345

<210>  6

<211>  346
<212>  PRT
<213>  Comamonas testosteroni

<400>  6

Met Leu Val Lys Asn Thr Trp Tyr Val Ala Gly Met Ala Thr Asp Cys
1               5                   10                  15

Ser Arg Lys Pro Leu Ala Arg Thr Phe Leu Asn Glu Lys Val Val Leu
            20                  25                  30

Phe Arg Thr His Asp Gly His Ala Val Ala Leu Glu Asp Arg Cys Cys
        35                  40                  45

His Arg Leu Ala Pro Leu Ser Leu Gly Asp Val Glu Asp Ala Gly Ile
    50                  55                  60

Arg Cys Arg Tyr His Gly Met Val Phe Asn Ala Ser Gly Ala Cys Val
65                  70                  75                  80

Glu Ile Pro Gly Gln Glu Gln Ile Pro Pro Gly Met Cys Val Arg Arg
                85                  90                  95

Phe Pro Leu Val Glu Arg His Gly Leu Leu Trp Ile Trp Met Gly Asp
            100                 105                 110

Pro Ala Arg Ala Asn Pro Asp Asp Ile Val Asp Glu Leu Trp Asn Gly
        115                 120                 125

Ala Pro Glu Trp Arg Thr Asp Ser Gly Tyr Ile His Tyr Gln Ala Asn
    130                 135                 140

Tyr Gln Leu Ile Val Asp Asn Leu Leu Asp Phe Thr His Leu Ala Trp
145                 150                 155                 160

Val His Pro Thr Thr Leu Gly Thr Asp Ser Ala Ala Ser Leu Lys Pro
                165                 170                 175

Val Ile Glu Arg Asp Thr Thr Gly Thr Gly Lys Leu Thr Ile Thr Arg
            180                 185                 190

Trp Tyr Leu Asn Asp Asp Met Ser Asn Leu His Lys Gly Val Ala Lys
        195                 200                 205

Phe Glu Gly Lys Ala Asp Arg Trp Gln Ile Tyr Gln Trp Ser Pro Pro
    210                 215                 220

Ala Leu Leu Arg Met Asp Thr Gly Ser Ala Pro Thr Gly Thr Gly Ala

225                 230                 235                 240

Pro Glu Gly Arg Arg Val Pro Glu Ala Val Gln Phe Arg His Thr Ser
                245                 250                 255

Ile Gln Thr Pro Glu Thr Glu Thr Thr Ser His Tyr Trp Phe Cys Gln
            260                 265                 270

Ala Arg Asn Phe Asp Leu Asp Asp Glu Ala Leu Thr Glu Lys Ile Tyr
        275                 280                 285

Gln Gly Val Val Val Ala Phe Glu Glu Asp Arg Thr Met Ile Glu Ala
    290                 295                 300

Gln Gln Lys Ile Leu Ser Gln Val Pro Asp Arg Pro Met Val Pro Ile
305                 310                 315                 320

Ala Ala Asp Ala Gly Leu Asn Gln Gly Arg Trp Leu Leu Asp Arg Leu
                325                 330                 335

Leu Lys Ala Glu Asn Gly Gly Thr Ala Pro
            340                 345

<210>  7
<211>  358
<212>  PRT
<213>  Xanthomonas campestris

<400>  7

Met Ser Gln Ser Lys Pro Leu Phe Pro Leu Asn Ala Trp Tyr Ala Val
1               5                   10                  15

Ala Trp Asp His Glu Ile Lys His Val Leu Ser Pro Arg Lys Leu Cys
            20                  25                  30

Asn Leu Asp Val Val Val Tyr Arg Thr Thr Ala Gly Ala Val Val Ala
        35                  40                  45

Leu Glu Asp Ala Cys Trp His Arg Leu Val Pro Leu Ser Met Gly Lys
    50                  55                  60

Leu Arg Gly Asp Asp Val Val Cys Gly Tyr His Gly Leu Val Tyr Asn
65                  70                  75                  80

Thr Gln Gly Arg Cys Val His Met Pro Ser Gln Asp Thr Ile Asn Pro
                85                  90                  95

Ser Ala Cys Val Arg Ser Phe Pro Val Ala Glu Lys His Arg Tyr Val

            100                 105                 110

Trp Ile Trp Pro Gly Asp Pro Ala Lys Ala Asp Thr Arg Leu Ile Pro
        115                 120                 125

Asp Leu His Trp Ser His Asp Pro Ala Trp Ala Gly Asp Gly Arg Thr
    130                 135                 140

Ile His Ala Lys Cys Asp Tyr Arg Leu Val Leu Asp Asn Leu Met Asp
145                 150                 155                 160

Leu Thr His Glu Thr Phe Val His Gly Ser Ser Ile Gly Gln Asp Glu
                165                 170                 175

Val Ala Glu Ala Pro Phe Asp Val Val His Gly Asp Arg Gly Val Ile
            180                 185                 190

Val Ser Arg Trp Met Arg Asn Ile Asp Pro Pro Pro Phe Trp Ala Ser
        195                 200                 205

Gln Ile Ala Arg His Leu Asp Tyr Arg Gly Lys Val Asp Arg Trp Gln
    210                 215                 220

Ile Ile Arg Phe Glu Ala Pro Ser Thr Ile Ala Ile Asp Val Gly Val
225                 230                 235                 240

Ala Ile Ala Gly Thr Gly Ala Pro Glu Gly Asp Arg Ser Gln Gly Val
                245                 250                 255

Asn Gly Tyr Val Leu Asn Thr Ile Thr Pro Glu Thr Asp Thr Thr Cys
            260                 265                 270

His Tyr Phe Trp Ala Phe Met Arg Asn Tyr Ala Leu His Asp Gln Ser
        275                 280                 285

Leu Thr Thr Leu Thr Arg Asp Gly Val Thr Gly Val Phe Gly Glu Asp
    290                 295                 300

Glu Ala Val Leu Glu Ala Gln Gln Arg Ala Ile Asp Ala His Pro Asp
305                 310                 315                 320

His Thr Phe Tyr Asn Leu Asn Val Asp Ala Gly Gly Met Trp Ala Arg
                325                 330                 335

Arg Val Ile Asp Arg Leu Ile Ala Gln Glu Gln Arg Ala Gln Asp Leu
            340                 345                 350

Ser Leu Arg Met Val Gly
        355

<210>  8
<211>  347
<212>  PRT
<213>  Rhodopseudomonas palustris

<400>  8

Met Pro Ala Phe Pro Leu Asn Ala Trp Tyr Ala Ala Ala Trp Asp Ala
1               5                   10                  15

Asp Ile Lys His Ala Leu Phe Pro Arg Thr Ile Cys Asn Lys His Val
            20                  25                  30

Val Met Tyr Arg Lys Ala Asp Gly Ser Val Ala Ala Leu Glu Asp Ala
        35                  40                  45

Cys Trp His Arg Leu Val Pro Leu Ser Lys Gly Arg Leu Glu Gly Asp
    50                  55                  60

Thr Val Val Cys Gly Tyr His Gly Leu Lys Phe Ser Pro Gln Gly Arg
65                  70                  75                  80

Cys Thr Tyr Met Pro Ser Gln Glu Thr Ile Asn Pro Ser Ala Cys Val
                85                  90                  95

Arg Ser Tyr Pro Val Val Glu Arg His Arg Phe Val Trp Leu Trp Met
            100                 105                 110

Gly Asp Pro Ala Leu Ala Asp Pro Ala Leu Val Pro Asp Met His Trp
        115                 120                 125

Asn Asp Asp Pro Ala Trp Ala Gly Asp Gly Lys Thr Ile His Ala Arg
    130                 135                 140

Cys Asp Trp Arg Leu Val Val Asp Asn Leu Met Asp Leu Thr His Glu
145                 150                 155                 160

Thr Tyr Val His Gly Ser Ser Ile Gly Asn Glu Ala Val Ala Glu Ala
                165                 170                 175

Pro Phe Asp Val Thr His Gly Asp Arg Thr Val Thr Val Thr Arg Trp
            180                 185                 190

Met Arg Gly Ile Glu Ala Pro Pro Phe Trp Ala Ala Gln Leu Arg Lys
        195                 200                 205

Pro Gly Pro Val Asp Arg Trp Gln Ile Ile Arg Phe Glu Ala Pro Gly
    210                 215                 220

Thr Val Thr Ile Asp Val Gly Val Ala Pro Ala Gly Ser Gly Ala Pro
225                 230                 235                 240

Glu Gly Asp Arg Ser Gln Gly Val Asn Gly Phe Val Leu Asn Thr Met
                245                 250                 255

Thr Pro Glu Thr Asp Thr Thr Cys His Tyr Phe Trp Ala Phe Val Arg
            260                 265                 270

Asn Tyr Arg Leu Gly Asp Gln Arg Leu Thr Thr Glu Ile Arg Glu Gly
        275                 280                 285

Val Ser Gly Ile Phe Gly Glu Asp Glu Ile Ile Leu Glu Ala Gln Gln
    290                 295                 300

Arg Ala Ile Ser Glu Asn Pro Asp Arg Val Phe Tyr Asn Leu Asn Ile
305                 310                 315                 320

Asp Ala Gly Ala Met Trp Ser Arg Lys Leu Ile Asp Arg Met Val Ala
                325                 330                 335

Lys Glu Ala Ala Pro Arg Leu Gln Ala Ala Glu
            340                 345

<210>  9
<211>  347
<212>  PRT
<213>  Bradyrhizobium sp.

<400>  9

Met Ala Ala Ser Phe Pro Met Asn Ala Trp Tyr Ala Ala Ala Trp Asp
1               5                   10                  15

Ala Glu Val Lys Gln Ala Leu Leu Pro Arg Thr Ile Cys Gly Lys His
            20                  25                  30

Val Val Met Tyr Arg Lys Ala Asp Gly Ser Ile Ala Ala Leu Glu Asp
        35                  40                  45

Ala Cys Trp His Arg Leu Val Pro Leu Ser Lys Gly Arg Leu Glu Gly
    50                  55                  60

Asp Thr Val Val Cys Gly Tyr His Gly Leu Lys Phe Ser Pro Gln Gly
65                  70                  75                  80

Arg Cys Thr Phe Met Pro Ser Gln Glu Thr Ile Asn Pro Ser Ala Cys
                85                  90                  95

Val Arg Ala Tyr Pro Ala Val Glu Arg His Arg Phe Ile Trp Leu Trp
            100                 105                 110

Met Gly Asp Pro Ala Leu Ala Asp Pro Ala Thr Ile Pro Asp Met His
        115                 120                 125

Trp Asn His Asp Pro Ala Trp Ala Gly Asp Gly Lys Thr Ile Gln Val
    130                 135                 140

Lys Cys Asp Tyr Arg Leu Val Val Asp Asn Leu Met Asp Leu Thr His
145                 150                 155                 160

Glu Thr Phe Val His Gly Ser Ser Ile Gly Asn Asp Ala Val Ala Glu
                165                 170                 175

Ala Pro Phe Asp Val Thr His Gly Glu Arg Thr Ala Thr Val Thr Arg
            180                 185                 190

Trp Met Arg Gly Ile Glu Pro Pro Pro Phe Trp Ala Lys Gln Leu Gly
        195                 200                 205

Lys Pro Gly Leu Val Asp Arg Trp Gln Ile Ile Arg Phe Glu Ala Pro
    210                 215                 220

Cys Thr Val Thr Ile Asp Val Gly Val Ala Pro Thr Gly Thr Gly Ala
225                 230                 235                 240

Pro Glu Gly Asp Arg Ser Gln Gly Val Asn Gly Met Val Leu Asn Thr
                245                 250                 255

Ile Thr Pro Glu Thr Asp Lys Thr Cys His Tyr Phe Trp Ala Phe Ala
            260                 265                 270

Arg Asn Tyr Gln Leu Thr Glu Gln Arg Leu Thr Thr Glu Ile Arg Glu
        275                 280                 285

Gly Val Ser Gly Ile Phe Arg Glu Asp Glu Leu Ile Leu Glu Ala Gln
    290                 295                 300

Gln Arg Ala Met Asp Ala Asn Pro Gly Arg Val Phe Tyr Asn Leu Asn
305                 310                 315                 320

Ile Asp Ala Gly Ala Met Trp Ala Arg Arg Ile Ile Asp Arg Met Ile
                325                 330                 335

Ala Arg Glu Thr Pro Leu Arg Glu Ala Ala Glu
            340                 345

<210>  10
<211>  342
<212>  PRT
<213>  Marine gamma proteobacterium HTCC2207

<400>  10

Met Thr Phe Ile Arg Asn Arg Trp Tyr Ile Ala Ala Trp Asp Gly Glu
1               5                   10                  15

Val Ala Asn Ala Pro Leu Ser Arg Lys Ile Cys Gly Glu Thr Ile Val
            20                  25                  30

Leu Tyr Arg Lys Leu Asn Gly Ser Val Val Ala Leu Arg Asp Ala Cys
        35                  40                  45

Pro His Arg Leu Leu Pro Leu Ser Leu Gly Thr Arg Glu Gly Asp Asn
    50                  55                  60

Leu Arg Cys Lys Tyr His Gly Met Leu Ile Gly Pro Asp Gly Ser Pro
65                  70                  75                  80

Glu Glu Met Pro Leu Thr Asn Gln Arg Val Asn Lys Gln Ile Ser Thr
                85                  90                  95

Gln Ser Tyr Asn Val Val Glu Lys Tyr Arg Tyr Ile Trp Val Trp Ile
            100                 105                 110

Gly Glu Gln Asp Lys Ala Asp Pro Glu Thr Val Pro Asp Phe Trp Pro
        115                 120                 125

Cys Asp Ser Glu Gly Trp Val Phe Asp Gly Gly Tyr Met His Val Gln
    130                 135                 140

Cys Asp Tyr Arg Leu Phe Ile Asp Asn Leu Met Asp Leu Thr His Glu
145                 150                 155                 160

Thr Tyr Val His Ala Gly Ser Ile Gly Gln Lys Glu Leu Met Glu Ser
                165                 170                 175

Pro Leu Glu Thr Ser Val Asn Gly Asn Lys Val Thr Leu Ser Arg Trp
            180                 185                 190

Ile Pro Asn Ile Ser Pro Pro Pro Phe Trp Arg Asp Ala Leu Gln Lys
        195                 200                 205

Asp Thr Pro Val Asp Arg Trp Gln Ile Cys Glu Phe Ile Glu Pro Cys
    210                 215                 220

Ser Val Asn Ile Asp Val Gly Val Ser Pro Ile Glu Asn Leu Asp Ser
225                 230                 235                 240

Leu Glu Asp His Asn Ser Gly Val Arg Gly Phe Val Ile Asp Ser Met
                245                 250                 255

Thr Pro Glu Thr Glu Glu Ser Cys His Tyr Phe Trp Gly Met Ala Arg
            260                 265                 270

Asn Phe Arg Ile Asp Asp Gln Gly Leu Thr Gln Arg Ile Arg Ala Gly
        275                 280                 285

Gln Ala Ala Ile Phe His Glu Asp Ile Glu Ile Leu Glu Arg Gln Gln
    290                 295                 300

Gln Ser Ile Ala Asp Asn Pro Asp Met Ala Leu Arg Val Leu Ser Ile
305                 310                 315                 320

Asp Ser Gly Gly Ala His Ala Arg Arg Ser Ile Ser Lys Leu Met Glu
                325                 330                 335

Ile Glu Asn Gly Lys Lys
            340

<210>  11
<211>  349
<212>  PRT
<213>  Pseudoalteromonas atlantica

<400>  11

Met Ser Val Gln Lys Tyr Pro Leu Asn Thr Trp Tyr Val Ala Cys Thr
1               5                   10                  15

Pro Asp Glu Ile Thr Met Ala Pro Phe Ala Arg Lys Ile Cys Gly Ile
            20                  25                  30

Ala Leu Val Phe Phe Arg Asn Thr His Gly Thr Val Val Ala Leu Glu
        35                  40                  45

Asp Phe Cys Pro His Arg Gly Ala Pro Leu Ser Leu Gly Lys Val Glu
    50                  55                  60

Asn Gly Gln Leu Val Cys Gly Tyr His Gly Leu Arg Met Gly Asp Asp
65                  70                  75                  80

Gly Ala Thr Lys Ala Met Pro Asn Gln Arg Val Gln Ala Phe Pro Cys
                85                  90                  95

Ile Gln Arg Tyr Ala Val Val Glu Arg Tyr Gly Tyr Ile Trp Ile Trp
            100                 105                 110

Pro Gly Asp Lys Ser Leu Ala Asp Glu Ser Leu Leu Pro Lys Leu Glu
        115                 120                 125

Trp Pro Asn Asn Pro Asn Trp Gly Tyr Gly Gly Gly Leu Tyr His Ile
    130                 135                 140

Lys Cys Asp Tyr Arg Leu Met Ile Asp Asn Leu Met Asp Leu Thr His
145                 150                 155                 160

Glu Thr Tyr Val His Ala Ser Ser Ile Gly Gln Lys Glu Ile Asp Glu
                165                 170                 175

Ala Pro Val Thr Thr Lys Val Asp Gly Glu Ser Ile Val Thr Ser Arg
            180                 185                 190

Phe Met Asp Asn Val Met Ala Pro Pro Phe Trp Ala Ser Ala Leu Arg
        195                 200                 205

Ala Asn Asp Leu Ala Asp Asp Ile Pro Val Asp Arg Trp Gln Ile Cys
    210                 215                 220

Arg Phe Asn Leu Pro Ser His Ile Met Ile Glu Val Gly Val Ala His
225                 230                 235                 240

Ala Gly Lys Gly Gly Tyr Leu Ala Pro Lys Glu Cys Lys Ala Ser Ser
                245                 250                 255

Ile Val Val Asp Phe Ile Thr Pro Glu Ser Asp His Ser Ile Trp Tyr
            260                 265                 270

Phe Trp Gly Met Ala Arg Asp Phe Lys Pro Gln Asp Ser Glu Leu Thr
        275                 280                 285

Asn Ser Ile Arg Ser Gly Gln Gly Ala Ile Phe Ala Glu Asp Leu Asp
    290                 295                 300

Val Leu Glu Arg Gln Gln Glu Asn Leu Leu Arg His Pro Asp Arg Thr
305                 310                 315                 320

Leu Leu Lys Leu Asp Ile Asp Ala Gly Gly Val Arg Ala Arg Arg Met
                325                 330                 335

Ile Glu Arg Ala Ile Lys Gln Glu Gln Ala Ser Ala Asn
            340                 345

<210>  12
<211>  358
<212>  PRT
<213>  Acinetobacter sp. ADP1

<400>  12

Met Phe Ile Lys Asn Ala Trp Tyr Val Ala Cys Arg Pro Glu Glu Ile
1               5                   10                  15

Gln Asp Lys Pro Leu Gly Arg Thr Ile Cys Gly Glu Lys Ile Val Phe
            20                  25                  30

Tyr Arg Gly Lys Glu Asn Lys Val Ala Ala Val Glu Asp Phe Cys Pro
        35                  40                  45

His Arg Gly Ala Pro Leu Ser Leu Gly Tyr Val Glu Asp Gly His Leu
    50                  55                  60

Val Cys Gly Tyr His Gly Leu Val Met Gly Cys Glu Gly Lys Thr Ile
65                  70                  75                  80

Ala Met Pro Ala Gln Arg Val Gly Gly Phe Pro Cys Asn Lys Ala Tyr
                85                  90                  95

Ala Val Val Glu Lys Tyr Gly Phe Ile Trp Val Trp Pro Gly Glu Lys
            100                 105                 110

Ser Leu Ala Asn Glu Ala Asp Leu Pro Thr Leu Glu Trp Ala Asp His
        115                 120                 125

Pro Glu Trp Ser Tyr Gly Gly Gly Leu Phe His Ile Gln Cys Asp Tyr
    130                 135                 140

Arg Leu Met Ile Asp Asn Leu Met Asp Leu Thr His Glu Thr Tyr Val
145                 150                 155                 160

His Ser Ser Ser Ile Gly Gln Lys Glu Ile Asp Glu Ala Leu Pro Val
                165                 170                 175

Thr Lys Val Asp Gly Asp His Val Val Thr Glu Arg Tyr Met Glu Asn
            180                 185                 190

Ile Ile Ala Pro Pro Phe Trp Gln Met Ala Leu Arg Gly Asn His Leu
        195                 200                 205

Ala Asp Asp Val Pro Val Asp Arg Trp Gln Arg Cys His Phe Phe Ala
    210                 215                 220

Pro Ser Asn Val His Ile Glu Val Gly Val Ala His Ala Gly His Gly
225                 230                 235                 240

Gly Tyr Asn Ala Pro Lys Asp Lys Lys Val Ala Ser Val Val Val Asp
                245                 250                 255

Phe Ile Thr Pro Glu Thr Glu Thr Ser His Trp Tyr Phe Trp Gly Met
            260                 265                 270

Ala Arg Asn Phe Gln Pro Glu Asn Gln Gln Leu Thr Asp Gln Ile Arg
        275                 280                 285

Glu Gly Gln Gly Lys Ile Phe Thr Glu Asp Leu Glu Met Leu Glu Gln
    290                 295                 300

Gln Gln Gln Asn Ile Leu Arg Asn Pro Gln Arg Gln Leu Leu Met Leu
305                 310                 315                 320

Asn Ile Asp Ala Gly Gly Val Gln Ser Arg Lys Ile Ile Asp Arg Leu
                325                 330                 335

Leu Ala Lys Glu Asn Ser Pro Ser Pro Gln Asp Thr Gln Arg Lys Phe
            340                 345                 350

Pro Asn Ile Arg Val Ile
        355

<210>  13
<211>  354
<212>  PRT
<213>  Pseudomonas syringae

<400>  13

Met His Pro Lys Asn Ala Trp Tyr Val Ala Cys Thr Ala Asp Glu Val
1               5                   10                  15

Ala Asp Lys Pro Leu Gly Arg Gln Ile Cys Asn Glu Lys Met Val Phe
            20                  25                  30

Tyr Arg Asp Gln Asn Gln Gln Val Val Ala Val Glu Asp Phe Cys Pro
        35                  40                  45

His Arg Gly Ala Pro Leu Ser Leu Gly Tyr Val Glu Asn Gly Gln Leu
    50                  55                  60

Val Cys Gly Tyr His Gly Leu Val Met Gly Gly Asp Gly Lys Thr Ala
65                  70                  75                  80

Ser Met Pro Gly Gln Arg Val Arg Gly Phe Pro Cys Asn Lys Thr Phe
                85                  90                  95

Ala Ala Ile Glu Arg Tyr Gly Phe Ile Trp Val Trp Pro Gly Glu Arg
            100                 105                 110

Glu Lys Ala Asp Pro Ala Leu Ile His His Leu Glu Trp Ala Val Ser
        115                 120                 125

Asp Glu Trp Ala Tyr Gly Gly Gly Leu Phe His Ile Gln Cys Asp Tyr
    130                 135                 140

Arg Leu Met Ile Asp Asn Leu Met Asp Leu Thr His Glu Thr Tyr Val
145                 150                 155                 160

His Ala Ser Ser Ile Gly Gln Lys Glu Ile Asp Glu Ala Pro Pro Val
                165                 170                 175

Thr Thr Val Glu Gly Glu Glu Val Ile Thr Ala Arg His Met Glu Asn
            180                 185                 190

Ile Met Pro Pro Pro Phe Trp Lys Met Ala Leu Arg Gly Asn Asn Leu
        195                 200                 205

Ala Asp Asp Val Pro Val Asp Arg Trp Gln Ile Cys Arg Phe Thr Pro
    210                 215                 220

Pro Ser His Val Leu Ile Glu Val Gly Val Ala His Ala Gly Lys Gly
225                 230                 235                 240

Gly Tyr His Ala Pro His Glu Phe Lys Ala Ser Ser Ile Val Val Asp
                245                 250                 255

Phe Ile Thr Pro Glu Thr Asp Thr Ser Ile Trp Tyr Phe Trp Gly Met
            260                 265                 270

Ala Arg Asn Phe Lys Pro Ala Asp Glu Gln Leu Thr Ala Thr Ile Arg
        275                 280                 285

Glu Gly Gln His Lys Ile Phe Ser Glu Asp Leu Glu Met Leu Glu Arg
    290                 295                 300

Gln Gln Leu Asn Leu Leu Gln His Pro His Arg Asn Leu Leu Lys Leu
305                 310                 315                 320

Asn Ile Asp Ala Gly Gly Val Gln Ser Arg Lys Ile Leu Glu Arg Leu
                325                 330                 335

Ile Ala Ala Glu Gln Ala Asp Thr Ala Asp Gln Ile Pro Val Ala Ala
            340                 345                 350

Val Lys

<210>  14
<211>  352
<212>  PRT
<213>  Pseudomonas fluorescens

<400>  14

Met Tyr Pro Lys Asn Ala Trp Tyr Val Ala Cys Thr Pro Asp Glu Leu
1               5                   10                  15

Gln Gly Lys Pro Leu Gly Arg Gln Ile Cys Gly Glu His Met Val Phe
            20                  25                  30

Tyr Arg Ala His Glu Gly Arg Val Thr Ala Val Glu Asp Phe Cys Pro
        35                  40                  45

His Arg Gly Ala Pro Leu Ser Leu Gly Tyr Val Glu Asn Gly Asn Leu
    50                  55                  60

Val Cys Gly Tyr His Gly Leu Val Met Gly Cys Asp Gly Lys Thr Val
65                  70                  75                  80

Glu Met Pro Gly Gln Arg Val Arg Gly Phe Pro Cys Asn Lys Thr Phe
                85                  90                  95

Ala Ala Val Glu Arg Tyr Gly Phe Ile Trp Val Trp Pro Gly Asp Gln
            100                 105                 110

Ala Leu Ala Asp Pro Ala Leu Ile His His Leu Glu Trp Ala Asp Asn
        115                 120                 125

Asp Gln Trp Ala Tyr Gly Gly Gly Leu Phe His Ile Gln Cys Asp Tyr
    130                 135                 140

Arg Leu Met Ile Asp Asn Leu Met Asp Leu Thr His Glu Thr Tyr Val
145                 150                 155                 160

His Ala Ser Ser Ile Gly Gln Lys Glu Ile Asp Glu Ala Pro Pro Gln
                165                 170                 175

Thr Thr Val Asp Gly Asp Gln Val Val Thr Ala Arg His Met His Asn
            180                 185                 190

Val Met Pro Pro Pro Phe Trp Arg Met Ala Leu Arg Gly Asn Gln Leu
        195                 200                 205

Ala Asp Asp Val Pro Val Asp Arg Trp Gln Ile Cys Arg Phe Ser Pro
    210                 215                 220

Pro Ser His Val Leu Ile Glu Val Gly Val Ala His Ala Gly His Gly
225                 230                 235                 240

Gly Tyr Asp Ala Pro Ala Gln Tyr Lys Ala Ser Ser Ile Val Val Asp
                245                 250                 255

Phe Ile Thr Pro Glu Ser Asp Thr Ser Ile Trp Tyr Phe Trp Gly Met
            260                 265                 270

Ala Arg Asn Phe Asn Pro Gln Asp Pro Ala Leu Thr Glu Ser Ile Arg
        275                 280                 285

Glu Gly Gln Gly Lys Ile Phe Ser Glu Asp Leu Glu Met Leu Glu Arg
    290                 295                 300

Gln Gln Gln Asn Leu Leu Ala Gln Pro Gln Arg Asn Leu Leu Lys Leu
305                 310                 315                 320

Asn Ile Asp Ala Gly Gly Val Gln Ser Arg Arg Val Leu Glu Arg Leu
                325                 330                 335

Ile Ala Gln Glu Arg Glu Pro Arg Glu Pro Leu Ile Ala Thr Ser Arg
            340                 345                 350

<210>  15
<211>  342
<212>  PRT
<213>  Ralstonia solanacearum

<400>  15

Met Phe Leu Lys Asn Ala Trp Tyr Val Ala Cys Thr Pro Asp Glu Ile
1               5                   10                  15

Ala Asp Lys Pro Leu Gly Arg Arg Ile Cys Gly Glu Arg Met Val Phe
            20                  25                  30

Tyr Arg Gly Pro Glu Gly Lys Met Ala Ala Leu Glu Asp Phe Cys Pro
        35                  40                  45

His Arg Gly Ala Pro Leu Ser Leu Gly Phe Val Arg Asp Gly His Leu
    50                  55                  60

Val Cys Gly Tyr His Gly Leu Thr Met Lys Ala Asp Gly Lys Cys Ala
65                  70                  75                  80

Ser Met Pro Gly Gln Arg Val Gly Gly Phe Pro Cys Ile Arg Gln Phe
                85                  90                  95

Pro Val Val Glu Arg Tyr Gly Phe Ile Trp Val Trp Pro Gly Asp Ala
            100                 105                 110

Glu Leu Ala Asp Pro Ala Gln Ile His His Leu Glu Trp Ala Glu Ser
        115                 120                 125

Lys Ala Trp Ala Tyr Gly Gly Gly Leu Tyr His Ile Gln Cys Asp Tyr
    130                 135                 140

Arg Leu Met Ile Asp Asn Leu Met Asp Leu Thr His Glu Thr Tyr Val
145                 150                 155                 160

His Ala Thr Ser Ile Gly Gln Pro Glu Ile Glu Glu Ala Ala Pro Gln
                165                 170                 175

Thr Arg Val Glu Gly Asp Thr Val Val Thr Ser Arg Phe Met Glu Asn
            180                 185                 190

Ile Met Pro Pro Pro Phe Trp Ala Thr Ala Leu Arg Gly Ala Gly Leu
        195                 200                 205

Ala Asp Asn Val Pro Cys Asp Arg Trp Gln Ile Cys Arg Phe Thr Pro
    210                 215                 220

Pro Ser His Val Leu Ile Glu Val Gly Val Ala His Ala Ser Lys Gly
225                 230                 235                 240

Gly Tyr Asp Ala Gly Pro Glu His Arg Val Gly Ser Ile Val Val Asp
                245                 250                 255

Phe Ile Thr Pro Glu Thr Glu Thr Ser Ile Trp Tyr Phe Trp Gly Met
            260                 265                 270

Ala Arg Asn Phe Arg Val Asp Asp Ala Ala Leu Thr Asp Thr Ile Arg
        275                 280                 285

Gln Gly Gln Gly Lys Ile Phe Gly Glu Asp Leu Asp Met Leu Glu Ser
    290                 295                 300

Gln Gln Arg Asn Leu Leu Ala Tyr Pro Glu Arg Asn Leu Leu Lys Leu
305                 310                 315                 320

Asn Ile Asp Ala Gly Gly Val Gln Ser Arg Arg Val Leu Glu Arg Leu
                325                 330                 335

Leu Glu Arg Glu Arg Gln
            340

<210>  16
<211>  354
<212>  PRT
<213>  Pseudomonas sp.

<400>  16

Met Phe Pro Lys Asn Ala Trp Tyr Val Ala Cys Thr Pro Asp Glu Ile
1               5                   10                  15

Ala Asp Lys Pro Leu Gly Arg Gln Ile Cys Asn Glu Lys Ile Val Phe
            20                  25                  30

Tyr Arg Gly Pro Glu Gly Arg Val Ala Ala Val Glu Asp Phe Cys Pro
        35                  40                  45

His Arg Gly Ala Pro Leu Ser Leu Gly Phe Val Arg Asp Gly Lys Leu
    50                  55                  60

Ile Cys Gly Tyr His Gly Leu Glu Met Gly Cys Glu Gly Lys Thr Leu
65                  70                  75                  80

Ala Met Pro Gly Gln Arg Val Gln Gly Phe Pro Cys Ile Lys Ser Tyr
                85                  90                  95

Ala Val Glu Glu Arg Tyr Gly Phe Ile Trp Val Trp Pro Gly Asp Arg
            100                 105                 110

Glu Leu Ala Asp Pro Ala Leu Ile His His Leu Glu Trp Ala Asp Asn
        115                 120                 125

Pro Glu Trp Ala Tyr Gly Gly Gly Leu Tyr His Ile Ala Cys Asp Tyr
    130                 135                 140

Arg Leu Met Ile Asp Asn Leu Met Asp Leu Thr His Glu Thr Tyr Val
145                 150                 155                 160

His Ala Ser Ser Ile Gly Gln Lys Glu Ile Asp Glu Ala Pro Val Ser
                165                 170                 175

Thr Arg Val Glu Gly Asp Thr Val Ile Thr Ser Arg Tyr Met Asp Asn
            180                 185                 190

Val Met Ala Pro Pro Phe Trp Arg Ala Ala Leu Arg Gly Asn Gly Leu
        195                 200                 205

Ala Asp Asp Val Pro Val Asp Arg Trp Gln Ile Cys Arg Phe Ala Pro
    210                 215                 220

Pro Ser His Val Leu Ile Glu Val Gly Val Ala His Ala Gly Lys Gly
225                 230                 235                 240

Gly Tyr Asp Ala Pro Ala Glu Tyr Lys Ala Gly Ser Ile Val Val Asp
                245                 250                 255

Phe Ile Thr Pro Glu Ser Asp Thr Ser Ile Trp Tyr Phe Trp Gly Met
            260                 265                 270

Ala Arg Asn Phe Arg Pro Gln Gly Thr Glu Leu Thr Glu Thr Ile Arg
        275                 280                 285

Val Gly Gln Gly Lys Ile Phe Ala Glu Asp Leu Asp Met Leu Glu Gln
    290                 295                 300

Gln Gln Arg Asn Leu Leu Ala Tyr Pro Glu Arg Gln Leu Leu Lys Leu
305                 310                 315                 320

Asn Ile Asp Ala Gly Gly Val Gln Ser Arg Arg Val Ile Asp Arg Ile
                325                 330                 335

Leu Ala Ala Glu Gln Glu Ala Ala Asp Ala Ala Leu Ile Ala Arg Ser
            340                 345                 350

Ala Ser

<210>  17
<211>  447
<212>  PRT
<213>  Comamonas sp.

<400>  17

Met Ser Tyr Gln Asn Leu Val Ser Glu Ala Gly Leu Thr Gln Lys Leu
1               5                   10                  15

Leu Ile His Gly Asp Lys Glu Leu Phe Gln His Glu Leu Lys Thr Ile
            20                  25                  30

Phe Ala Arg Asn Trp Leu Phe Leu Thr His Asp Ser Leu Ile Pro Ser
        35                  40                  45

Pro Gly Asp Tyr Val Lys Ala Lys Met Gly Val Asp Glu Val Ile Val
    50                  55                  60

Ser Arg Gln Asn Asp Gly Ser Val Arg Ala Phe Leu Asn Val Cys Arg
65                  70                  75                  80

His Arg Gly Lys Thr Leu Val His Ala Glu Ala Gly Asn Ala Lys Gly
                85                  90                  95

Phe Val Cys Gly Tyr His Gly Trp Gly Tyr Gly Ser Asn Gly Glu Leu
            100                 105                 110

Gln Ser Val Pro Phe Glu Lys Glu Leu Tyr Gly Asp Ala Ile Lys Lys
        115                 120                 125

Lys Cys Leu Gly Leu Lys Glu Val Pro Arg Ile Glu Ser Phe His Gly
    130                 135                 140

Phe Ile Tyr Gly Cys Phe Asp Ala Glu Ala Pro Pro Leu Ile Asp Tyr
145                 150                 155                 160

Leu Gly Asp Ala Ala Trp Tyr Leu Glu Pro Thr Phe Lys Tyr Ser Gly
                165                 170                 175

Gly Leu Glu Leu Val Gly Pro Pro Gly Lys Val Val Val Lys Ala Asn
            180                 185                 190

Trp Lys Ser Phe Ala Glu Asn Phe Val Gly Asp Gly Tyr His Val Gly
        195                 200                 205

Trp Thr His Ala Ala Ala Leu Arg Ala Gly Gln Ser Val Phe Ser Ser
    210                 215                 220

Ile Ala Gly Asn Ala Lys Leu Pro Pro Glu Gly Ala Gly Leu Gln Met
225                 230                 235                 240

Thr Ser Lys Tyr Gly Ser Gly Met Gly Val Phe Trp Gly Tyr Tyr Ser
                245                 250                 255

Gly Asn Phe Ser Ala Asp Met Ile Pro Asp Leu Met Ala Phe Gly Ala
            260                 265                 270

Ala Lys Gln Glu Lys Leu Ala Lys Glu Ile Gly Asp Val Arg Ala Arg
        275                 280                 285

Ile Tyr Arg Ser Phe Leu Asn Gly Thr Ile Phe Pro Asn Asn Ser Phe
    290                 295                 300

Leu Thr Gly Ser Ala Ala Phe Arg Val Trp Asn Pro Ile Asp Glu Asn
305                 310                 315                 320

Thr Thr Glu Val Trp Thr Tyr Ala Phe Val Glu Lys Asp Met Pro Glu
                325                 330                 335

Asp Leu Lys Arg Arg Val Ala Asp Ala Val Gln Arg Ser Ile Gly Pro
            340                 345                 350

Ala Gly Phe Trp Glu Ser Asp Asp Asn Glu Asn Met Glu Thr Met Ser
        355                 360                 365

Gln Asn Gly Lys Lys Tyr Gln Ser Ser Asn Ile Asp Gln Ile Ala Ser
    370                 375                 380

Leu Gly Phe Gly Lys Asp Val Tyr Gly Asp Glu Cys Tyr Pro Gly Val
385                 390                 395                 400

Val Gly Lys Ser Ala Ile Gly Glu Thr Ser Tyr Arg Gly Phe Tyr Arg
                405                 410                 415

Ala Tyr Gln Ala His Ile Ser Ser Ser Asn Trp Ala Glu Phe Glu Asn
            420                 425                 430

Ala Ser Arg Asn Trp His Ile Glu His Thr Lys Thr Thr Asp Arg
        435                 440                 445

<210>  18
<211>  447
<212>  PRT
<213>  Burkholderia cepacia

<400>  18

Met Ser Tyr Gln Asn Leu Val Ser Glu Ala Gly Leu Thr Gln Lys His
1               5                   10                  15

Leu Ile Tyr Gly Asp Lys Glu Leu Phe Gln His Glu Leu Lys Thr Ile
            20                  25                  30

Phe Ala Arg Asn Trp Leu Phe Leu Thr His Asp Ser Leu Ile Pro Ser
        35                  40                  45

Pro Gly Asp Tyr Val Lys Ala Lys Met Gly Val Asp Glu Val Ile Val
    50                  55                  60

Ser Arg Gln Asn Asp Gly Ser Val Arg Ala Phe Leu Asn Val Cys Arg
65                  70                  75                  80

His Arg Gly Lys Thr Ile Val Asp Ala Glu Ala Gly Asn Ala Lys Gly
                85                  90                  95

Phe Val Cys Gly Tyr His Gly Trp Gly Tyr Gly Ser Asn Gly Glu Leu
            100                 105                 110

Gln Ser Val Pro Phe Glu Lys Glu Leu Tyr Gly Asp Ala Ile Lys Lys
        115                 120                 125

Lys Cys Leu Gly Leu Lys Glu Val Pro Arg Ile Glu Ser Phe His Gly
    130                 135                 140

Phe Ile Tyr Gly Cys Phe Asp Ala Glu Ala Pro Pro Leu Ile Asp Tyr
145                 150                 155                 160

Leu Gly Asp Ala Ala Trp Tyr Leu Glu Pro Thr Phe Lys His Ser Gly
                165                 170                 175

Gly Leu Glu Leu Val Gly Pro Pro Gly Lys Val Val Val Lys Ala Asn
            180                 185                 190

Trp Lys Pro Leu Ala Glu Asn Phe Val Gly Asp Val Tyr His Ile Gly
        195                 200                 205

Trp Thr His Ala Ser Ile Leu Arg Ala Gly Gln Ser Ile Phe Ala Pro
    210                 215                 220

Leu Ala Gly Asn Ala Met Phe Pro Pro Glu Gly Ala Gly Leu Gln Met
225                 230                 235                 240

Thr Thr Lys Tyr Gly Ser Gly Ile Gly Val Leu Trp Asp Ala Tyr Ser
                245                 250                 255

Gly Ile Gln Ser Ala Asp Met Val Pro Glu Met Met Ala Phe Gly Gly
            260                 265                 270

Ala Lys Gln Glu Lys Leu Ala Lys Glu Ile Gly Asp Val Arg Ala Arg
        275                 280                 285

Ile Tyr Arg Ser Gln Leu Asn Gly Thr Val Phe Pro Asn Asn Ser Phe
    290                 295                 300

Leu Thr Cys Ser Gly Val Phe Lys Val Phe Asn Pro Ile Asp Glu Asn
305                 310                 315                 320

Thr Thr Glu Val Trp Thr Tyr Ala Ile Val Glu Lys Asp Met Pro Glu
                325                 330                 335

Asp Leu Lys Arg Arg Leu Ala Asp Ala Val Gln Arg Ser Val Gly Pro
            340                 345                 350

Ala Gly Tyr Trp Glu Ser Asp Asp Asn Asp Asn Met Gly Thr Leu Ser
        355                 360                 365

Gln Asn Ala Lys Lys Tyr Gln Ser Ser Asn Ser Asp Leu Ile Ala Asp
    370                 375                 380

Leu Gly Phe Gly Lys Asp Val Tyr Gly Asp Glu Cys Tyr Pro Gly Val
385                 390                 395                 400

Val Gly Lys Ser Ala Ile Ser Glu Thr Ser Tyr Arg Gly Phe Tyr Arg
                405                 410                 415

Ala Tyr Gln Ala His Ile Ser Ser Ser Asn Trp Ala Glu Phe Glu Asn
            420                 425                 430

Thr Ser Arg Asn Trp His Thr Glu Leu Thr Lys Thr Thr Asp Arg
        435                 440                 445

<210>  19
<211>  447
<212>  PRT
<213>  Comamonas testosteroni

<400>  19

Met Ile Tyr Glu Asn Leu Val Ser Glu Ala Gly Leu Thr Gln Lys His
1               5                   10                  15

Leu Ile His Gly Asp Lys Glu Leu Phe Gln His Glu Leu Lys Thr Ile
            20                  25                  30

Phe Ala Arg Asn Trp Leu Phe Leu Thr His Asp Ser Leu Ile Pro Ser
        35                  40                  45

Pro Gly Asp Tyr Val Thr Ala Lys Met Gly Val Asp Glu Val Ile Val
    50                  55                  60

Ser Arg Gln Asn Asp Gly Ser Val Arg Ala Phe Leu Asn Val Cys Arg
65                  70                  75                  80

His Arg Gly Lys Thr Leu Val His Ala Glu Ala Gly Asn Ala Lys Gly
                85                  90                  95

Phe Val Cys Ser Tyr His Gly Trp Gly Phe Gly Ser Asn Gly Glu Leu
            100                 105                 110

Gln Ser Val Pro Phe Glu Lys Glu Leu Tyr Gly Asp Ala Ile Lys Lys
        115                 120                 125

Lys Cys Leu Gly Leu Lys Glu Val Pro Arg Ile Glu Ser Phe His Gly
    130                 135                 140

Phe Ile Tyr Gly Cys Phe Asp Ala Glu Ala Pro Pro Leu Ile Asp Tyr
145                 150                 155                 160

Leu Gly Asp Ala Ala Trp Tyr Leu Glu Pro Ile Phe Lys His Ser Gly
                165                 170                 175

Gly Leu Glu Leu Val Gly Pro Pro Gly Lys Val Val Ile Lys Ala Asn
            180                 185                 190

Trp Lys Ala Pro Ala Glu Asn Phe Val Gly Asp Ala Tyr His Val Gly
        195                 200                 205

Trp Thr His Ala Ala Ser Leu Arg Ser Gly Gln Ser Ile Phe Thr Pro
    210                 215                 220

Leu Ala Gly Asn Ala Met Leu Pro Pro Glu Gly Ala Gly Leu Gln Met
225                 230                 235                 240

Thr Ser Lys Tyr Gly Ser Gly Met Gly Val Leu Trp Asp Ala Tyr Ser
                245                 250                 255

Gly Ile His Ser Ala Asp Leu Val Pro Glu Met Met Ala Phe Gly Gly
            260                 265                 270

Ala Lys Gln Glu Lys Leu Ala Lys Glu Ile Gly Asp Val Arg Ala Arg
        275                 280                 285

Ile Tyr Arg Ser His Leu Asn Cys Thr Val Phe Pro Asn Asn Ser Ile
    290                 295                 300

Leu Thr Cys Ser Gly Val Phe Lys Val Trp Asn Pro Ile Asp Glu Asn
305                 310                 315                 320

Thr Thr Glu Val Trp Thr Tyr Ala Ile Val Glu Lys Asp Met Pro Glu
                325                 330                 335

Asp Leu Lys Arg Arg Leu Ala Asp Ala Val Gln Arg Thr Phe Gly Pro
            340                 345                 350

Ala Gly Phe Trp Glu Ser Asp Asp Asn Asp Asn Met Glu Thr Glu Ser
        355                 360                 365

Gln Asn Ala Lys Lys Tyr Gln Ser Ser Asn Ser Asp Leu Ile Ala Asn
    370                 375                 380

Leu Gly Phe Gly Lys Asp Val Tyr Gly Asp Glu Cys Tyr Pro Gly Val
385                 390                 395                 400

Val Gly Lys Ser Ala Ile Gly Glu Thr Ser Tyr Arg Gly Phe Tyr Arg
                405                 410                 415

Ala Tyr Gln Ala His Ile Ser Ser Ser Asn Trp Ala Glu Phe Glu Asn
            420                 425                 430

Thr Ser Arg Asn Trp His Thr Glu Leu Thr Lys Thr Thr Asp Arg
        435                 440                 445

<210>  20
<211>  470
<212>  PRT
<213>  Rhodococcus sp.

<400>  20

Met Leu Ser Asn Glu Leu Arg Gln Thr Leu Gln Lys Gly Leu His Asp
1               5                   10                  15

Val Asn Ser Asp Trp Thr Val Pro Ala Ala Ile Ile Asn Asp Pro Glu
            20                  25                  30

Val His Asp Val Glu Arg Glu Arg Ile Phe Gly His Ala Trp Val Phe
        35                  40                  45

Leu Ala His Glu Ser Glu Ile Pro Glu Arg Gly Asp Tyr Val Val Arg
    50                  55                  60

Tyr Ile Ser Glu Asp Gln Phe Ile Val Cys Arg Asp Glu Gly Gly Glu
65                  70                  75                  80

Ile Arg Gly His Leu Asn Ala Cys Arg His Arg Gly Met Gln Val Cys
                85                  90                  95

Arg Ala Glu Met Gly Asn Thr Ser His Phe Arg Cys Pro Tyr His Gly
            100                 105                 110

Trp Thr Tyr Ser Asn Thr Gly Ser Leu Val Gly Val Pro Ala Gly Lys
        115                 120                 125

Asp Ala Tyr Gly Asn Gln Leu Lys Lys Ser Asp Trp Asn Leu Arg Pro
    130                 135                 140

Met Pro Asn Leu Ala Ser Tyr Lys Gly Leu Ile Phe Gly Ser Leu Asp
145                 150                 155                 160

Pro His Ala Asp Ser Leu Glu Asp Tyr Leu Gly Asp Leu Lys Phe Tyr
                165                 170                 175

Leu Asp Ile Val Leu Asp Arg Ser Asp Ala Gly Leu Gln Val Val Gly
            180                 185                 190

Ala Pro Gln Arg Trp Val Ile Asp Ala Asn Trp Lys Leu Gly Ala Asp
        195                 200                 205

Asn Phe Val Gly Asp Ala Tyr His Thr Met Met Thr His Arg Ser Met
    210                 215                 220

Val Glu Leu Gly Leu Ala Pro Pro Asp Pro Gln Phe Ala Leu Tyr Gly
225                 230                 235                 240

Glu His Ile His Thr Gly His Gly His Gly Leu Gly Ile Ile Gly Pro
                245                 250                 255

Pro Pro Gly Met Pro Leu Pro Glu Phe Met Gly Leu Pro Glu Asn Ile
            260                 265                 270

Val Glu Glu Leu Glu Arg Arg Leu Thr Pro Glu Gln Val Glu Ile Phe
        275                 280                 285

Arg Pro Thr Ala Phe Ile His Gly Thr Val Phe Pro Asn Leu Ser Ile
    290                 295                 300

Gly Asn Phe Leu Met Gly Lys Asp His Leu Ser Ala Pro Thr Ala Phe
305                 310                 315                 320

Leu Thr Leu Arg Leu Trp His Pro Leu Gly Pro Asp Lys Met Glu Val

325                 330                 335

Met Ser Phe Phe Leu Val Glu Lys Asp Ala Pro Asp Trp Phe Lys Asp
            340                 345                 350

Glu Ser Tyr Lys Ser Tyr Leu Arg Thr Phe Gly Ile Ser Gly Gly Phe
        355                 360                 365

Glu Gln Asp Asp Ala Glu Asn Trp Arg Ser Ile Thr Arg Val Met Gly
    370                 375                 380

Gly Gln Phe Ala Lys Thr Gly Glu Leu Asn Tyr Gln Met Gly Arg Gly
385                 390                 395                 400

Val Leu Glu Pro Asp Pro Asn Trp Thr Gly Pro Gly Glu Ala Tyr Pro
                405                 410                 415

Leu Asp Tyr Ala Glu Ala Asn Gln Arg Asn Phe Leu Glu Tyr Trp Met
            420                 425                 430

Gln Leu Met Leu Ala Glu Ser Pro Leu Arg Asp Gly Asn Ser Asn Gly
        435                 440                 445

Ser Gly Thr Ala Asp Ala Ser Thr Pro Ala Ala Ala Lys Ser Lys Ser
    450                 455                 460

Pro Ala Lys Ala Glu Ala
465                 470

<210>  21
<211>  460
<212>  PRT
<213>  Rhodococcus strain RHA-1

<400>  21

Met Thr Asp Val Gln Cys Glu Pro Ala Leu Ala Gly Arg Lys Pro Lys
1               5                   10                  15

Trp Ala Asp Ala Asp Ile Ala Glu Leu Val Asp Glu Arg Thr Gly Arg
            20                  25                  30

Leu Asp Pro Arg Ile Tyr Thr Asp Glu Ala Leu Tyr Glu Gln Glu Leu
        35                  40                  45

Glu Arg Ile Phe Gly Arg Ser Trp Leu Leu Met Gly His Glu Thr Gln
    50                  55                  60

Ile Pro Lys Ala Gly Asp Phe Met Thr Asn Tyr Met Gly Glu Asp Pro

65                  70                  75                  80

Val Met Val Val Arg Gln Lys Asn Gly Glu Ile Arg Val Phe Leu Asn
                85                  90                  95

Gln Cys Arg His Arg Gly Met Arg Ile Cys Arg Ala Asp Gly Gly Asn
            100                 105                 110

Ala Lys Ser Phe Thr Cys Ser Tyr His Gly Trp Ala Tyr Asp Thr Gly
        115                 120                 125

Gly Asn Leu Val Ser Val Pro Phe Glu Glu Gln Ala Phe Pro Gly Leu
    130                 135                 140

Arg Lys Glu Asp Trp Gly Pro Leu Gln Ala Arg Val Glu Thr Tyr Lys
145                 150                 155                 160

Gly Leu Ile Phe Ala Asn Trp Asp Ala Asp Ala Pro Asp Leu Asp Thr
                165                 170                 175

Tyr Leu Gly Glu Ala Lys Phe Tyr Met Asp His Met Leu Asp Arg Thr
            180                 185                 190

Glu Ala Gly Thr Glu Ala Ile Pro Gly Ile Gln Lys Trp Val Ile Pro
        195                 200                 205

Cys Asn Trp Lys Phe Ala Ala Glu Gln Phe Cys Ser Asp Met Tyr His
    210                 215                 220

Ala Gly Thr Thr Ser His Leu Ser Gly Ile Leu Ala Gly Leu Pro Asp
225                 230                 235                 240

Gly Val Asp Leu Ser Glu Leu Ala Pro Pro Thr Glu Gly Ile Gln Tyr
                245                 250                 255

Arg Ala Thr Trp Gly Gly His Gly Ser Gly Phe Tyr Ile Gly Asp Pro
            260                 265                 270

Asn Leu Leu Leu Ala Ile Met Gly Pro Lys Val Thr Glu Tyr Trp Thr
        275                 280                 285

Gln Gly Pro Ala Ala Glu Lys Ala Ser Glu Arg Leu Gly Ser Thr Glu
    290                 295                 300

Arg Gly Gln Gln Leu Met Ala Gln His Met Thr Ile Phe Pro Thr Cys
305                 310                 315                 320

Ser Phe Leu Pro Gly Ile Asn Thr Ile Arg Ala Trp His Pro Arg Gly
                325                 330                 335

Pro Asn Glu Ile Glu Val Trp Ala Phe Thr Val Val Asp Ala Asp Ala
            340                 345                 350

Pro Glu Glu Met Lys Glu Glu Tyr Arg Gln Gln Thr Leu Arg Thr Phe
        355                 360                 365

Ser Ala Gly Gly Val Phe Glu Gln Asp Asp Gly Glu Asn Trp Val Glu
    370                 375                 380

Ile Gln Gln Val Leu Arg Gly His Lys Ala Arg Ser Arg Pro Phe Asn
385                 390                 395                 400

Ala Glu Met Gly Leu Gly Gln Thr Asp Ser Asp Asn Pro Asp Tyr Pro
                405                 410                 415

Gly Thr Ile Ser Tyr Val Tyr Ser Glu Glu Ala Ala Arg Gly Leu Tyr
            420                 425                 430

Thr Gln Trp Val Arg Met Met Thr Ser Pro Asp Trp Ala Ala Leu Asp
        435                 440                 445

Ala Thr Arg Pro Ala Val Ser Glu Ser Thr His Thr
    450                 455                 460

<210>  22
<211>  449
<212>  PRT
<213>  Rhodococcus sp. strain NCIB 9816-4

<400>  22

Met Asn Tyr Asn Asn Lys Ile Leu Val Ser Glu Ser Gly Leu Ser Gln
1               5                   10                  15

Lys His Leu Ile His Gly Asp Glu Glu Leu Phe Gln His Glu Leu Lys
            20                  25                  30

Thr Ile Phe Ala Arg Asn Trp Leu Phe Leu Thr His Asp Ser Leu Ile
        35                  40                  45

Pro Ala Pro Gly Asp Tyr Val Thr Ala Lys Met Gly Ile Asp Glu Val
    50                  55                  60

Ile Val Ser Arg Gln Asn Asp Gly Ser Ile Arg Ala Phe Leu Asn Val
65                  70                  75                  80

Cys Arg His Arg Gly Lys Thr Leu Val Ser Val Glu Ala Gly Asn Ala
                85                  90                  95

Lys Gly Phe Val Cys Ser Tyr His Gly Trp Gly Phe Gly Ser Asn Gly
            100                 105                 110

Glu Leu Gln Ser Val Pro Phe Glu Lys Asp Leu Tyr Gly Glu Ser Leu
        115                 120                 125

Asn Lys Lys Cys Leu Gly Leu Lys Glu Val Ala Arg Val Glu Ser Phe
    130                 135                 140

His Gly Phe Ile Tyr Gly Cys Phe Asp Gln Glu Ala Pro Pro Leu Met
145                 150                 155                 160

Asp Tyr Leu Gly Asp Ala Ala Trp Tyr Leu Glu Pro Met Phe Lys His
                165                 170                 175

Ser Gly Gly Leu Glu Leu Val Gly Pro Pro Gly Lys Val Val Ile Lys
            180                 185                 190

Ala Asn Trp Lys Ala Pro Ala Glu Asn Phe Val Gly Asp Ala Tyr His
        195                 200                 205

Val Gly Trp Thr His Ala Ser Ser Leu Arg Ser Gly Glu Ser Ile Phe
    210                 215                 220

Ser Ser Leu Ala Gly Asn Ala Ala Leu Pro Pro Glu Gly Ala Gly Leu
225                 230                 235                 240

Gln Met Thr Ser Lys Tyr Gly Ser Gly Met Gly Val Leu Trp Asp Gly
                245                 250                 255

Tyr Ser Gly Val His Ser Ala Asp Leu Val Pro Glu Leu Met Ala Phe
            260                 265                 270

Gly Gly Ala Lys Gln Glu Arg Leu Asn Lys Glu Ile Gly Asp Val Arg
        275                 280                 285

Ala Arg Ile Tyr Arg Ser His Leu Asn Cys Thr Val Phe Pro Asn Asn
    290                 295                 300

Ser Met Leu Thr Cys Ser Gly Val Phe Lys Val Trp Asn Pro Ile Asp
305                 310                 315                 320

Ala Asn Thr Thr Glu Val Trp Thr Tyr Ala Ile Val Glu Lys Asp Met
                325                 330                 335

Pro Glu Asp Leu Lys Arg Arg Leu Ala Asp Ser Val Gln Arg Thr Phe
            340                 345                 350

Gly Pro Ala Gly Phe Trp Glu Ser Asp Asp Asn Asp Asn Met Glu Thr
        355                 360                 365

Ala Ser Gln Asn Gly Lys Lys Tyr Gln Ser Arg Asp Ser Asp Leu Leu
    370                 375                 380

Ser Asn Leu Gly Phe Gly Glu Asp Val Tyr Gly Asp Ala Val Tyr Pro
385                 390                 395                 400

Gly Val Val Gly Lys Ser Ala Ile Gly Glu Thr Ser Tyr Arg Gly Phe
                405                 410                 415

Tyr Arg Ala Tyr Gln Ala His Val Ser Ser Ser Asn Trp Ala Glu Phe
            420                 425                 430

Glu His Ala Ser Ser Thr Trp His Thr Glu Leu Thr Lys Thr Thr Asp
        435                 440                 445

Arg

<210>  23
<211>  339
<212>  PRT
<213>  Artificial

<220>
<223>  Synthetic peptide

<400>  23

Met Ser Phe Val Arg Asn Ala Trp Tyr Val Ala Ala Leu Pro Glu Glu
1               5                   10                  15

Leu Ser Glu Lys Pro Leu Gly Arg Thr Ile Leu Asp Thr Pro Leu Ala
            20                  25                  30

Leu Tyr Arg Gln Pro Asp Gly Val Val Ala Ala Leu Leu Asp Ile Cys
        35                  40                  45

Pro His Arg Phe Ala Pro Leu Ser Asp Gly Ile Leu Val Asn Gly His
    50                  55                  60

Leu Gln Cys Pro Tyr His Gly Leu Glu Phe Asp Gly Gly Gly Gln Cys
65                  70                  75                  80

Val His Asn Pro His Gly Asn Gly Ala Arg Pro Ala Ser Leu Asn Val
                85                  90                  95

Arg Ser Phe Pro Val Val Glu Arg Asp Ala Leu Ile Trp Ile Trp Pro
            100                 105                 110

Gly Asp Pro Ala Leu Ala Asp Pro Gly Ala Leu Pro Asp Phe Gly Cys
        115                 120                 125

Arg Val Asp Pro Ala Tyr Arg Thr Val Gly Gly Tyr Gly His Val Asp
    130                 135                 140

Cys Asn Tyr Lys Leu Leu Val Asp Asn Leu Met Asp Leu Gly His Ala
145                 150                 155                 160

Gln Tyr Val His Arg Ala Asn Ala Gln Thr Asp Ala Phe Asp Arg Leu
                165                 170                 175

Glu Arg Glu Val Ile Val Gly Asp Gly Glu Ile Gln Ala Leu Met Lys
            180                 185                 190

Ile Pro Gly Gly Thr Pro Ser Val Leu Met Ala Lys Phe Leu Arg Gly
        195                 200                 205

Ala Asn Thr Pro Val Asp Ala Trp Asn Asp Ile Arg Trp Asn Lys Val
    210                 215                 220

Ser Ala Met Leu Asn Phe Ile Ala Val Ala Pro Glu Gly Thr Pro Lys
225                 230                 235                 240

Glu Gln Ser Ile His Ser Arg Gly Thr His Ile Leu Thr Pro Glu Thr
                245                 250                 255

Glu Ala Ser Cys His Tyr Phe Phe Gly Ser Ser Arg Asn Phe Gly Ile
            260                 265                 270

Asp Asp Pro Glu Met Asp Gly Val Leu Arg Ser Trp Gln Ala Gln Ala
        275                 280                 285

Leu Val Lys Glu Asp Lys Val Val Val Glu Ala Ile Glu Arg Arg Arg
    290                 295                 300

Ala Tyr Val Glu Ala Asn Gly Ile Arg Pro Ala Met Leu Ser Cys Asp
305                 310                 315                 320

Glu Ala Ala Val Arg Val Ser Arg Glu Ile Glu Lys Leu Glu Gln Leu
                325                 330                 335

Glu Ala Ala

<210> 24
<211> 18
<212> PRT
<213> Artificial

<220>
<223> Synthetic peptide

<400> 24

Ala Ala Val Arg Val Ser Arg Glu Ile Glu Lys Leu Glu Gln Leu Glu
1               5                   10                  15

Ala Ala

<210> 25
<211> 8
<212> PRT
<213> Artificial

<220>
<223> Synthetic peptide

<400> 25

Ile Glu Lys Ile Glu Gln Leu Glu
1               5

<210> 26
<211> 8
<212> PRT
<213> Artificial

<220>
<223> Synthetic peptide

<400> 26

Ile Glu Lys Leu Glu Gln Leu Glu
1               5

<210> 27
<211> 8
<212> PRT
<213> Artificial

<220>
<223> Synthetic peptide

<400> 27

Leu Asp Arg Leu Asp Asp Ile Asp
1               5


<210>  28
<211>  8
<212>  PRT
<213>  Artificial

<220>
<223>  Synthetic peptide

<400>  28

Val His Arg Val His Glu Val Gln

1               5


<210>  29
<211>  8
<212>  PRT
<213>  Artificial

<220>
<223>  Synthetic peptide

<400>  29

Val Asn Arg Val Gln His Val His
1               5


<210>  30
<211>  8
<212>  PRT
<213>  Artificial

<220>
<223>  Synthetic peptide

<400>  30

Val Gln Arg Val Gln His Val Lys
1               5


<210>  31
<211>  8
<212>  PRT
<213>  Artificial

<220>
<223>  Synthetic peptide

<400>  31

Val Lys Arg Val Gln His Val Asn
1               5


<210>  32
<211>  34

<212>  DNA
<213>  Artificial

<220>
<223>  Synthetic primer

<400>  32
tgaacctcgg ccacgcccaa tatgtccatc gcgc                                    34


<210>  33
<211>  36
<212>  DNA
<213>  Artificial

<220>
<223>  Synthetic primer

<400>  33
cgtggccgag gttcatcagg ttgtcgacca gcagct                                  36


<210>  34
<211>  34
<212>  DNA
<213>  Artificial

<220>
<223>  Synthetic primer

<400>  34
ggagaacaag gtcgtcgtcg aggcgatcga gcgc                                    34


<210>  35
<211>  35
<212>  DNA
<213>  Artificial

<220>
<223>  Synthetic primer

<400>  35
cgacgacctt gttctccttg accagcgcct gagcc                                   35


<210>  36
<211>  32
<212>  DNA
<213>  Artificial

<220>
<223>  Synthetic primer

<400>  36
cggcaacgcc caatatgtcc atcgcgccaa cg                                      32


<210>  37
<211>  37
<212>  DNA
<213>  Artificial

<220>
<223>  Synthetic primer

<400>  37
tattgggcgt tgccgaggtc catcaggttg tcgacca                                37

<210>  38
<211>  32
<212>  DNA
<213>  Artificial

<220>
<223>  Synthetic primer

<400>  38
atgtcaatcg cgccaacgcc cagaccgacg cc                                     32

<210>  39
<211>  32
<212>  DNA
<213>  Artificial

<220>
<223>  Synthetic primer

<220>
<221>  modified_base
<222>  (15)..(15)
<223>  t = t/u

<400>  39
tggcgcgatt gacatattgg gcgtggccga gg                                     32

<210>  40
<211>  33
<212>  DNA
<213>  Artificial

<220>
<223>  Synthetic primer

<220>
<221>  modified_base
<222>  (15)..(15)
<223>  u = t/u

<220>
<221>  modified_base
<222>  (15)..(15)
<223>  t = t/u

<400>  40
gctggtcgac gccctgatgg acctcggcca cgc                                    33

<210>  41
<211>  39
<212>  DNA
<213>  Artificial

<220>
<223>  Synthetic primer

<400>  41
agggcgtcga ccagcagctt gtagttgcag gtcgacatgc                              40

<210>  42
<211>  339
<212>  PRT
<213>  Artificial

<220>
<223>  Synthetic peptide

<400>  42

Met Thr Phe Leu Arg Asn Ala Trp Tyr Val Ala Ala Leu Pro Glu Glu
1               5                   10                  15

Leu Ser Glu Lys Pro Leu Gly Arg Thr Ile Leu Asp Thr Pro Leu Ala
            20                  25                  30

Leu Tyr Arg Gln Pro Asp Gly Val Val Ala Ala Leu Leu Asp Ile Cys
        35                  40                  45

Pro His Arg Phe Ala Pro Leu Ser Asp Gly Ile Leu Val Asn Gly His
    50                  55                  60

Leu Gln Cys Pro Tyr His Gly Leu Glu Phe Asp Gly Gly Gly Gln Cys
65                  70                  75                  80

Val His Asn Pro His Gly Asn Gly Ala Arg Pro Ala Ser Leu Asn Val
                85                  90                  95

Arg Ser Phe Pro Val Val Glu Arg Asp Ala Leu Ile Trp Ile Trp Pro
            100                 105                 110

Gly Asp Pro Ala Leu Ala Asp Pro Gly Ala Ile Pro Asp Phe Gly Cys
        115                 120                 125

Arg Val Asp Pro Ala Tyr Arg Thr Val Gly Gly Tyr Gly His Val Asp
    130                 135                 140

Cys Asn Tyr Lys Leu Leu Val Asp Asn Leu Met Asp Leu Gly His Ala
145                 150                 155                 160

Gln Tyr Val His Arg Ala Asn Ala Gln Thr Asp Ala Phe Asp Arg Leu
                165                 170                 175

Glu Arg Glu Val Ile Val Gly Asp Gly Glu Ile Gln Ala Leu Met Lys
            180                 185                 190

Ile Pro Gly Gly Thr Pro Ser Val Leu Met Ala Lys Phe Leu Arg Gly
        195                 200                 205

Ala Asn Thr Pro Val Asp Ala Trp Asn Asp Ile Arg Trp Asn Lys Val
    210                 215                 220

Ser Ala Met Leu Asn Phe Ile Ala Val Ala Pro Glu Gly Thr Pro Lys
225                 230                 235                 240

Glu Gln Ser Ile His Ser Arg Gly Thr His Ile Leu Thr Pro Glu Thr
                245                 250                 255

Glu Ala Ser Cys His Tyr Phe Phe Gly Ser Ser Arg Asn Phe Gly Ile
            260                 265                 270

Asp Asp Pro Glu Met Asp Gly Val Ile Arg Ser Trp Gln Ala Gln Ala
        275                 280                 285

Leu Val Lys Glu Asp Lys Val Val Val Glu Ala Ile Glu Arg Arg Arg
    290                 295                 300

Ala Tyr Val Glu Ala Asn Gly Ile Arg Pro Ala Met Leu Ser Cys Asp
305                 310                 315                 320

Glu Ala Ala Val Arg Val Ser Arg Glu Ile Glu Lys Leu Glu Gln Leu
                325                 330                 335

Glu Ala Ala

<210>  43
<211>  459
<212>  PRT
<213>  Pseudomonas fluorescens

<400>  43

Met Ser Ser Ile Ile Asn Lys Glu Val Gln Glu Ala Pro Leu Lys Trp
1               5                   10                  15

Val Lys Asn Trp Ser Asp Glu Glu Ile Lys Ala Leu Val Asp Glu Glu
            20                  25                  30

Lys Gly Leu Leu Asp Pro Arg Ile Phe Ser Asp Gln Asp Leu Tyr Glu
        35                  40                  45

Ile Glu Leu Glu Arg Val Phe Ala Arg Ser Trp Leu Leu Leu Gly His
    50                  55                  60

Glu Gly His Ile Pro Lys Ala Gly Asp Tyr Leu Thr Thr Tyr Met Gly
65                  70                  75                  80

Glu Asp Pro Val Ile Val Val Arg Gln Lys Asp Arg Ser Ile Lys Val
                85                  90                  95

Phe Leu Asn Gln Cys Arg His Arg Gly Met Arg Ile Glu Arg Ser Asp
            100                 105                 110

Phe Gly Asn Ala Lys Ser Phe Thr Cys Thr Tyr His Gly Trp Ala Tyr
        115                 120                 125

Asp Thr Ala Gly Asn Leu Val Asn Val Pro Tyr Glu Lys Glu Ala Phe
    130                 135                 140

Cys Asp Lys Lys Glu Gly Asp Cys Gly Phe Asp Lys Ala Asp Trp Gly
145                 150                 155                 160

Pro Leu Gln Ala Arg Val Asp Thr Tyr Lys Gly Leu Ile Phe Ala Asn
                165                 170                 175

Trp Asp Thr Glu Ala Pro Asp Leu Lys Thr Tyr Leu Ser Asp Ala Thr
            180                 185                 190

Pro Tyr Met Asp Val Met Leu Asp Arg Thr Glu Ala Val Thr Gln Val
        195                 200                 205

Ile Thr Gly Met Gln Lys Thr Val Ile Pro Cys Asn Trp Lys Phe Ala
    210                 215                 220

Ala Glu Gln Phe Cys Ser Asp Met Tyr His Ala Gly Thr Met Ala His
225                 230                 235                 240

Leu Ser Gly Val Leu Ser Ser Leu Pro Pro Glu Met Asp Leu Ser Gln
                245                 250                 255

Val Lys Leu Pro Ser Ser Gly Asn Gln Phe Arg Ala Lys Trp Gly Gly
            260                 265                 270

His Gly Thr Gly Trp Phe Asn Asp Asp Phe Ala Leu Leu Gln Ala Ile
        275                 280                 285

Met Gly Pro Lys Val Val Asp Tyr Trp Thr Lys Gly Pro Ala Ala Glu
    290                 295                 300

Arg Ala Lys Glu Arg Leu Gly Lys Val Leu Pro Ala Asp Arg Met Val
305                 310                 315                 320

Ala Gln His Met Thr Ile Phe Pro Thr Cys Ser Phe Leu Pro Gly Ile
                325                 330                 335

Asn Thr Val Arg Thr Trp His Pro Arg Gly Pro Asn Glu Ile Glu Val
            340                 345                 350

Trp Ser Phe Ile Val Val Asp Ala Asp Ala Pro Glu Asp Ile Lys Glu
        355                 360                 365

Glu Tyr Arg Arg Lys Asn Ile Phe Thr Phe Asn Gln Gly Gly Thr Tyr
    370                 375                 380

Glu Gln Asp Asp Gly Glu Asn Trp Val Glu Val Gln Arg Gly Leu Arg
385                 390                 395                 400

Gly Tyr Lys Ala Arg Ser Arg Pro Leu Cys Ala Gln Met Gly Ala Gly
                405                 410                 415

Val Pro Asn Lys Asn Asn Pro Glu Phe Pro Gly Lys Thr Ser Tyr Val
            420                 425                 430

Tyr Ser Glu Glu Ala Ala Arg Gly Phe Tyr His His Trp Ser Arg Met
        435                 440                 445

Met Ser Glu Pro Ser Trp Asp Thr Leu Lys Ser
    450                 455

<210>  44
<211>  446
<212>  PRT
<213>  Pseudomonas putida

<400>  44

Met Ser Asp Gln Pro Ile Ile Arg Arg Arg Gln Val Lys Thr Gly Ile
1               5                   10                  15

Ser Asp Ala Arg Ala Asn Asn Ala Lys Thr Gln Ser Gln Tyr Gln Pro
            20                  25                  30

Tyr Lys Asp Ala Ala Trp Gly Phe Ile Asn His Trp Tyr Pro Ala Leu
        35                  40                  45

Phe Thr His Glu Leu Glu Glu Asp Gln Val Gln Gly Ile Gln Ile Cys
    50                  55                  60

Gly Val Pro Ile Val Leu Arg Arg Val Asn Gly Lys Val Phe Ala Leu
65                  70                  75                  80

Lys Asp Gln Cys Leu His Arg Gly Val Arg Leu Ser Glu Lys Pro Thr
                85                  90                  95

Cys Phe Thr Lys Ser Thr Ile Ser Cys Trp Tyr His Gly Phe Thr Phe
            100                 105                 110

Asp Leu Glu Thr Gly Lys Leu Val Thr Ile Val Ala Asn Pro Glu Asp
        115                 120                 125

Lys Leu Ile Gly Thr Thr Gly Val Thr Thr Tyr Pro Val His Glu Val
    130                 135                 140

Asn Gly Met Ile Phe Val Phe Val Arg Glu Asp Asp Phe Pro Asp Glu
145                 150                 155                 160

Asp Val Pro Pro Leu Ala His Asp Leu Pro Phe Arg Phe Pro Glu Arg
                165                 170                 175

Ser Glu Gln Phe Pro His Pro Leu Trp Pro Ser Ser Pro Ser Val Leu
            180                 185                 190

Asp Asp Asn Ala Val Val His Gly Met His Arg Thr Gly Phe Gly Asn
        195                 200                 205

Trp Arg Ile Ala Cys Glu Asn Gly Phe Asp Asn Ala His Ile Leu Val
    210                 215                 220

His Lys Asp Asn Thr Ile Val His Ala Met Asp Trp Val Leu Pro Leu
225                 230                 235                 240

Gly Leu Leu Pro Thr Ser Asp Asp Cys Ile Ala Val Val Glu Asp Asp
                245                 250                 255

Asp Gly Pro Lys Gly Met Met Gln Trp Leu Phe Thr Asp Lys Trp Ala
            260                 265                 270

Pro Val Leu Glu Asn Gln Glu Leu Gly Leu Lys Val Glu Gly Leu Lys
        275                 280                 285

Gly Arg His Tyr Arg Thr Ser Val Val Leu Pro Gly Val Leu Met Val
    290                 295                 300

Glu Asn Trp Pro Glu Glu His Val Val Gln Tyr Glu Trp Tyr Val Pro

305                 310                 315                 320

Ile Thr Asp Asp Thr His Glu Tyr Trp Glu Ile Leu Val Arg Val Cys
                325                 330                 335

Pro Thr Asp Glu Asp Arg Lys Lys Phe Gln Tyr Arg Tyr Asp His Met
            340                 345                 350

Tyr Lys Pro Leu Cys Leu His Gly Phe Asn Asp Ser Asp Leu Tyr Ala
        355                 360                 365

Arg Glu Ala Met Gln Asn Phe Tyr Tyr Asp Gly Thr Gly Trp Asp Asp
    370                 375                 380

Glu Gln Leu Val Ala Thr Asp Ile Ser Pro Ile Thr Trp Arg Lys Leu
385                 390                 395                 400

Ala Ser Arg Trp Asn Arg Gly Ile Ala Lys Pro Gly Arg Gly Val Ala
                405                 410                 415

Gly Ala Val Lys Asp Thr Ser Leu Ile Phe Lys Gln Thr Ala Asp Gly
            420                 425                 430

Lys Arg Pro Gly Tyr Lys Val Glu Gln Ile Lys Glu Asp His
        435                 440                 445

<210>  45
<211>  392
<212>  PRT
<213>  Janthinobacterium sp. strain J3

<400>  45
   Met Ala Asn Val Asp Glu Ala Ile Leu Lys Arg Val Lys Gly Trp Ala
   1               5                   10                  15
   Pro Tyr Val Asp Ala Lys Leu Gly Phe Arg Asn His Trp Tyr Pro Val
               20                  25                  30
   Met Phe Ser Lys Glu Ile Asn Glu Gly Glu Pro Lys Thr Leu Lys Leu
           35                  40                  45
   Leu Gly Glu Asn Leu Leu Val Asn Arg Ile Asp Gly Lys Leu Tyr Cys
       50                  55                  60
   Leu Lys Asp Arg Cys Leu His Arg Gly Val Gln Leu Ser Val Lys Val
   65                  70                  75                  80
   Glu Cys Lys Thr Lys Ser Thr Ile Thr Cys Trp Tyr His Ala Trp Thr
                   85                  90                  95
   Tyr Arg Trp Glu Asp Gly Val Leu Cys Asp Ile Leu Thr Asn Pro Thr
               100                 105                 110
   Ser Ala Gln Ile Gly Arg Gln Lys Leu Lys Thr Tyr Pro Val Gln Glu
           115                 120                 125
   Ala Lys Gly Cys Val Phe Ile Tyr Leu Gly Asp Gly Asp Pro Pro Pro
       130                 135                 140
   Leu Ala Arg Asp Thr Pro Pro Asn Phe Leu Asp Asp Asp Met Glu Ile
   145                 150                 155                 160
   Leu Gly Lys Asn Gln Ile Ile Lys Ser Asn Trp Arg Leu Ala Val Glu
                   165                 170                 175
   Asn Gly Phe Asp Pro Ser His Ile Tyr Ile His Lys Asp Ser Ile Leu
               180                 185                 190
   Val Lys Asp Asn Asp Leu Ala Leu Pro Leu Gly Phe Ala Pro Gly Gly
           195                 200                 205
   Asp Arg Lys Gln Gln Thr Arg Val Val Asp Asp Asp Val Val Gly Arg
       210                 215                 220
   Lys Gly Val Tyr Asp Leu Ile Gly Glu His Gly Val Pro Val Phe Glu
   225                 230                 235                 240
   Gly Thr Ile Gly Gly Glu Val Val Arg Glu Gly Ala Tyr Gly Glu Lys
                   245                 250                 255
   Ile Val Ala Asn Asp Ile Ser Ile Trp Leu Pro Gly Val Leu Lys Val
               260                 265                 270
   Asn Pro Phe Pro Asn Pro Asp Met Met Gln Phe Glu Trp Tyr Val Pro
           275                 280                 285
   Ile Asp Glu Asn Thr His Tyr Tyr Phe Gln Thr Leu Gly Lys Pro Cys
       290                 295                 300
   Ala Asn Asp Glu Glu Arg Lys Lys Tyr Glu Gln Glu Phe Glu Ser Lys
   305                 310                 315                 320
   Trp Lys Pro Met Ala Leu Glu Gly Phe Asn Asn Asp Asp Ile Trp Ala
                   325                 330                 335
   Arg Glu Ala Met Val Asp Phe Tyr Ala Asp Asp Lys Gly Trp Val Asn
               340                 345                 350
   Glu Ile Leu Phe Glu Ser Asp Glu Ala Ile Val Ala Trp Arg Lys Leu
           355                 360                 365
   Ala Ser Glu His Asn Gln Gly Ile Gln Thr Gln Ala His Val Ser Gly
       370                 375                 380
   Leu Glu His His His His His His
   385                 390

<210>  46
<211>  41
<212>  DNA
<213>  Artificial

<220>
<223>  Synthetic primer

<220>
<221>  misc_feature
<222>  (4)..(4)
<223>  v is a, c, or g

<220>
<221>  misc_feature
<222>  (9)..(9)
<223>  m is a, or c

<220>
<221>  misc_feature
<222>  (11)..(11)
<223>  r is a, or g

<220>
<221>  misc_feature
<222>  (16)..(16)
<223>  s is c, or g

<220>
<221>  misc_feature
<222>  (19)..(19)
<223>  s is c, or g

<220>
<221>  misc_feature
<222>  (22)..(22)
<223>  s is c, or g

<220>
<221>  misc_feature
<222>  (23)..(23)
<223>  y is c, or t

<220>
<221>  misc_feature
<222>  (24)..(24)

<223>  m is a, or c

<400>  46
gagvtcgama racttsaasa asymggaagc cgccagactc g                          41


<210>  47
<211>  40
<212>  DNA
<213>  Artificial

<220>
<223>  Synthetic primer

<220>
<221>  misc_feature
<222>  (2)..(2)
<223>  r is a, or g

<220>
<221>  misc_feature
<222>  (3)..(3)
<223>  s is c, or g

<220>
<221>  misc_feature
<222>  (6)..(6)
<223>  s is c, or g

<220>
<221>  misc_feature
<222>  (9)..(9)
<223>  s is c, or g

<220>
<221>  misc_feature
<222>  (14)..(14)
<223>  y is c, or t

<220>
<221>  misc_feature
<222>  (16)..(16)
<223>  k is g, or t

<220>
<221>  misc_feature
<222>  (21)..(21)
<223>  b is c, g, or t

<220>
<221>  misc_feature
<222>  (24)..(24)
<223>  m is a, or c

<400>  47
crsttsttsa agtytktcga bctmcgcggc tgacacggac                          40


<210>  48
<211>  50
<212>  DNA
<213>  Artificial

<220>
<223>  Synthetic primer

<220>
<221>  misc_feature
<222>  (2)..(2)
<223>  s is c, or g

<220>
<221>  misc_feature
<222>  (5)..(5)
<223>  s is c, or g

<220>
<221>  misc_feature
<222>  (8)..(8)
<223>  y is c, or t

<220>
<221>  misc_feature
<222>  (11)..(11)
<223>  r is a, or g

<220>
<221>  misc_feature
<222>  (14)..(14)
<223>  y is c, or t

<220>
<221>  misc_feature
<222>  (19)..(19)
<223>  y is c, or t

<220>
<221>  misc_feature
<222>  (24)..(24)
<223>  m is a, or c

<220>
<221>  misc_feature
<222>  (27)..(27)
<223>  m is a, or c

<400>  48
gstgstgytc rggytagcyg ggamatmcga gaagcttgag cagctcgaag                50


<210>  49
<211>  44
<212>  DNA
<213>  Artificial

<220>
<223>  Synthetic primer

<220>
<221>  misc_feature
<222>  (4)..(4)
<223>  k is g, or t

<220>
<221>  misc_feature
<222>  (9)..(9)

<223>  r is a, or g

<220>
<221>  misc_feature
<222>  (14)..(14)
<223>  r is a, or g

<220>
<221>  misc_feature
<222>  (17)..(17)
<223>  y is c, or t

<220>
<221>  misc_feature
<222>  (20)..(20)
<223>  r is a, or g

<220>
<221>  misc_feature
<222>  (23)..(23)
<223>  s is c, or g

<220>
<221>  misc_feature
<222>  (26)..(26)
<223>  s is c, or g

<220>
<221>  misc_feature
<222>  (27)..(27)
<223>  m is a, or c

<400>  49
gatktcccrg ctarccygar cascasmctt cgtcgcacga cagc                        44


<210>  50
<211>  54
<212>  DNA
<213>  Artificial

<220>
<223>  Synthetic primer

<220>
<221>  misc_feature
<222>  (4)..(4)
<223>  h is a, c, or t

<220>
<221>  misc_feature
<222>  (6)..(6)
<223>  n is a, c, g, or t

<220>
<221>  misc_feature
<222>  (7)..(7)
<223>  y is c, or t

<220>
<221>  misc_feature
<222>  (9)..(9)
<223>  d is a, g, or t

<220>
<221>  misc_feature
<222>  (10)..(10)
<223>  y is c, or t

<220>
<221>  misc_feature
<222>  (12)..(12)
<223>  s is c, or g

<220>
<221>  misc_feature
<222>  (13)..(13)
<223>  n is a, c, g, or t

<220>
<221>  misc_feature
<222>  (15)..(15)
<223>  s is c, or g

<220>
<221>  misc_feature
<222>  (17)..(17)
<223>  w is a, or t

<220>
<221>  misc_feature
<222>  (18)..(18)
<223>  m is a, or c

<220>
<221>  misc_feature
<222>  (19)..(19)
<223>  r is a, or g

<220>
<221>  misc_feature
<222>  (20)..(20)
<223>  w is a, or t

<220>
<221>  misc_feature
<222>  (21)..(21)
<223>  v is a, c, or g

<220>
<221>  misc_feature
<222>  (22)..(22)
<223>  r is a, or g

<220>
<221>  misc_feature
<222>  (23)..(23)
<223>  w is a, or t

<220>
<221>  misc_feature
<222>  (24)..(24)
<223>  r is a, or g

<220>

<221>  misc_feature

<222>  (25)..(25)
<223>  s is c, or g

<220>
<221>  misc_feature
<222>  (26)..(26)
<223>  w is a, or t

<220>
<221>  misc_feature
<222>  (27)..(29)
<223>  k is g, or t

<220>
<221>  misc_feature
<222>  (31)..(31)
<223>  m is a, or c

<220>
<221>  modified_base
<222>  (32)..(32)
<223>  u = t/u

<220>
<221>  modified_base
<222>  (32)..(32)
<223>  t = t/u

<400>  50
ctthcnyady asngsawmrw vrwrswkkka mtttggatct ggcctggtga ccct          54


<210>  51
<211>  54
<212>  DNA
<213>  Artificial

<220>
<223>  Synthetic primer


<220>
<221>  misc_feature
<222>  (19)..(19)
<223>  n is a, c, g, or t

<220>
<221>  misc_feature
<222>  (26)..(26)
<223>  n is a, c, g, or t

<400>  51
atmmmwsywy bwykwtscns trhtrngdaa mgaccgcacg ttcaggctag ccgg          54


<210>  52
<211>  54
<212>  DNA
<213>  Artificial

<220>

<223>  Synthetic primer


<220>
<221>  misc_feature
<222>  (3)..(3)
<223>  n is a, c, g, or t

<220>
<221>  misc_feature
<222>  (9)..(9)
<223>  n is a, c, g, or t

<220>
<221>  misc_feature
<222>  (12)..(12)
<223>  n is a, c, g, or t

<220>
<221>  misc_feature
<222>  (18)..(18)
<223>  n is a, c, g, or t

<220>
<221>  misc_feature
<222>  (27)..(27)
<223>  n is a, c, g, or t

<400>  52
acnctrvknt cncgsasnmt rstrscnttc mcagatttcg gttgtcgcgt tgac          54


<210>  53
<211>  54
<212>  DNA
<213>  Artificial

<220>
<223>  Synthetic primer


<220>
<221>  misc_feature
<222>  (5)..(5)
<223>  n is a, c, g, or t

<220>
<221>  misc_feature
<222>  (14)..(14)
<223>  n is a, c, g, or t

<220>
<221>  misc_feature
<222>  (20)..(20)
<223>  n is a, c, g, or t

<220>
<221>  misc_feature
<222>  (23)..(23)
<223>  n is a, c, g, or t

<220>
<221>  misc_feature

<222>  (29)..(29)
<223>  n is a, c, g, or t

<220>
<221>  modified_base
<222>  (32)..(32)
<223>  t = t/u

<400>  53
ggaangsyas yaknstscgn ganmbyagng mtcaccaggc cagatccaaa tcag          54


<210>  54
<211>  54
<212>  DNA
<213>  Artificial

<220>
<223>  Synthetic primer


<220>
<221>  misc_feature
<222>  (15)..(15)
<223>  n is a, c, g, or t

<220>
<221>  misc_feature
<222>  (18)..(18)
<223>  n is a, c, g, or t

<220>
<221>  misc_feature
<222>  (21)..(21)
<223>  n is a, c, g, or t


<220>
<221>  modified_base
<222>  (32)..(32)
<223>  t = t/u

<400>  54
tcmrkramra kmrmntkntm ntkrvmrsta mttgaacgcc gtcgcgcgta cgtc          54


<210>  55
<211>  54
<212>  DNA
<213>  Artificial

<220>
<223>  Synthetic primer


<220>
<221>  misc_feature
<222>  (11)..(11)
<223>  n is a, c, g, or t

<220>
<221>  misc_feature
<222>  (14)..(14)

<223>  n is a, c, g, or t

<220>
<221>  misc_feature
<222>  (17)..(17)
<223>  n is a, c, g, or t

<400>  55
atasykbyma nkanmankyk mtyktymykg maccagtgct tgcgcttgcc aact          54


<210>  56
<211>  48
<212>  DNA
<213>  Artificial

<220>
<223>  Synthetic primer

<400>  56
agvtcvakar gvtcsamsak vtcvamgmcc gcctgactcg agcatgca                48


<210>  57
<211>  47
<212>  DNA
<213>  Artificial

<220>
<223>  Synthetic primer


<220>
<221>  misc_feature
<222>  (14)..(14)
<223>  n is a, c, g, or t

<220>
<221>  misc_feature
<222>  (20)..(20)
<223>  n is a, c, g, or t

<400>  57
gtrycdccmr mvanvtcvan aravtcgmag cagctcgaag ccgcctg                 47


<210>  58
<211>  47
<212>  DNA
<213>  Artificial

<220>
<223>  Synthetic primer


<220>
<221>  misc_feature
<222>  (9)..(9)
<223>  n is a, c, g, or t

<220>
<221>  misc_feature
<222>  (15)..(15)

<223>  n is a, c, g, or t

<400>  58
tcgabtytnt bgabntbkyk gghgryamcg gactgcggct tcgtcgc                    47


<210>  59
<211>  47
<212>  DNA
<213>  Artificial

<220>
<223>  Synthetic primer


<220>
<221>  misc_feature
<222>  (14)..(14)
<223>  n is a, c, g, or t

<220>
<221>  misc_feature
<222>  (20)..(20)
<223>  n is a, c, g, or t

<400>  59
gtrycdccmr mvanvtcvan aravtcgmag cagctcgaag ccgcctg                    47


<210>  60
<211>  47
<212>  DNA
<213>  Artificial

<220>
<223>  Synthetic primer


<220>
<221>  misc_feature
<222>  (9)..(9)
<223>  n is a, c, g, or t

<220>
<221>  misc_feature
<222>  (15)..(15)
<223>  n is a, c, g, or t

<400>  60
tcgabtytnt bgabntbkyk gghgryamcg gactgcggct tcgtcgc                    47


<210>  61
<211>  48
<212>  DNA
<213>  Artificial

<220>
<223>  Synthetic primer

<400>  61
agvtcvakar gvtcsamsak vtcvamgmcc gcctgatgac taaagccc                   48

<210>  62
<211>  48
<212>  DNA
<213>  Artificial

<220>
<223>  Synthetic primer


<220>
<221>  modified_base
<222>  (29)..(29)
<223>  t = t./u

<400>  62
gcktbgabmt sktsgabcyt mtbgabcmtc gcggctgaca cggactgc                    48


<210>  63
<211>  48
<212>  DNA
<213>  Artificial

<220>
<223>  Synthetic primer

<400>  63
agvtcvakar gvtcsamsak vtcvamgmcc gcctgactcg agcaccac                    48


<210>  64
<211>  48
<212>  DNA
<213>  Artificial

<220>
<223>  Synthetic primer


<220>
<221>  modified_base
<222>  (29)..(29)
<223>  t = t./u

<400>  64
gcktbgabmt sktsgabcyt mtbgabcmtc gcggctgaca cggactgc                    48


<210>  65
<211>  52
<212>  DNA
<213>  Artificial

<220>
<223>  Synthetic primer


<220>
<221>  misc_feature
<222>  (6)..(6)
<223>  n is a, c, g, or t

<220>
<221>  misc_feature
<222>  (13)..(13)
<223>  n is a, c, g, or t

<220>
<221>  modified_base
<222>  (32)..(32)
<223>  t = t/u

<400>  65
ccthcnyady asngsawmrw vrwrswkkka mtctggatct ggcccggcga tc          52


<210>  66
<211>  52
<212>  DNA
<213>  Artificial

<220>
<223>  Synthetic primer


<220>
<221>  misc_feature
<222>  (19)..(19)
<223>  n is a, c, g, or t

<220>
<221>  misc_feature
<222>  (26)..(26)
<223>  n is a, c, g, or t

<400>  66
atmmmwsywy bwykwtscns trhtrngdag mgagcggacg ttgagcgaag cc          52


<210>  67
<211>  50
<212>  DNA
<213>  Artificial

<220>
<223>  Synthetic primer


<220>
<221>  misc_feature
<222>  (3)..(3)
<223>  n is a, c, g, or t

<220>
<221>  misc_feature
<222>  (9)..(9)
<223>  n is a, c, g, or t

<220>
<221>  misc_feature
<222>  (12)..(12)
<223>  n is a, c, g, or t

<220>
<221>  misc_feature

<222>  (18)..(18)
<223>  n is a, c, g, or t

<220>
<221>  misc_feature
<222>  (27)..(27)
<223>  n is a, c, g, or t

<400>  67
atnctrvknt cncgsasnmt rstrscnttc mccgacttcg gctgccgcgt                50


<210>  68
<211>  50
<212>  DNA
<213>  Artificial

<220>
<223>  Synthetic primer


<220>
<221>  misc_feature
<222>  (5)..(5)
<223>  n is a, c, g, or t

<220>
<221>  misc_feature
<222>  (14)..(14)
<223>  n is a, c, g, or t

<220>
<221>  misc_feature
<222>  (20)..(20)
<223>  n is a, c, g, or t

<220>
<221>  misc_feature
<222>  (23)..(23)
<223>  n is a, c, g, or t

<220>
<221>  misc_feature
<222>  (29)..(29)
<223>  n is a, c, g, or t

<220>
<221>  modified_base
<222>  (32)..(32)
<223>  t = t/u

<400>  68
ggaangsyas yaknstscgn ganmbyagna mtcgccgggc cagatccaga                50


<210>  69
<211>  51
<212>  DNA
<213>  Artificial

<220>
<223>  Synthetic primer

<220>
<221>  misc_feature
<222>  (15)..(15)
<223>  n is a, c, g, or t

<220>
<221>  misc_feature
<222>  (18)..(18)
<223>  n is a, c, g, or t

<220>
<221>  misc_feature
<222>  (21)..(21)
<223>  n is a, c, g, or t

<220>
<221>  modified_base
<222>  (32)..(32)
<223>  t = t/u

<400>  69
tcmrkramra kmrmntkntm ntkrvmrsta mtcgagcgcc gccgcgccta t          51


<210>  70
<211>  50
<212>  DNA
<213>  Artificial

<220>
<223>  Synthetic primer


<220>
<221>  misc_feature
<222>  (11)..(11)
<223>  n is a, c, g, or t

<220>
<221>  misc_feature
<222>  (14)..(14)
<223>  n is a, c, g, or t

<220>
<221>  misc_feature
<222>  (17)..(17)
<223>  n is a, c, g, or t

<400>  70
atasykbyma nkanmankyk mtyktymykg maccagcgcc tgagcctgcc          50


<210>  71
<211>  51
<212>  DNA
<213>  Artificial

<220>
<223>  Synthetic primer


<220>

<221>  misc_feature
<222>  (11)..(11)
<223>  n is a, c, g, or t

<400>  71
atawdgssat nkykwtccas cascgsawrm ggagcggacg ttgagcgaag c                51


<210>  72
<211>  51
<212>  DNA
<213>  Artificial

<220>
<223>  Synthetic primer


<220>
<221>  misc_feature
<222>  (20)..(20)
<223>  n is a, c, g, or t

<220>
<221>  modified_base
<222>  (31)..(31)
<223>  t = t/u

<400>  72
cywtscgstg stggawmrmn atsschwtam tctggatctg gcccggcgat c                51


<210>  73
<211>  52
<212>  DNA
<213>  Artificial

<220>
<223>  Synthetic primer


<220>
<221>  misc_feature
<222>  (11)..(11)
<223>  n is a, c, g, or t

<400>  73
ataadgssat ntytwtccas cascgsawrg mgagcggacg ttgagcgaag cc               52


<210>  74
<211>  52
<212>  DNA
<213>  Artificial

<220>
<223>  Synthetic primer


<220>
<221>  misc_feature
<222>  (21)..(21)
<223>  n is a, c, g, or t

<220>
<221>  modified_base
<222>  (32)..(32)
<223>  t = t/u

<400>  74
ccywtscgst gstggawara natsschtta mtctggatct ggcccggcga tc          52


<210>  75
<211>  49
<212>  DNA
<213>  Artificial

<220>
<223>  Synthetic primer

<400>  75
atgghkyyaa bsabcaschk wtswtycttm gaccagcgcc tgagcctgc          49


<210>  76
<211>  50
<212>  DNA
<213>  Artificial

<220>
<223>  Synthetic primer


<220>
<221>  modified_base
<222>  (31)..(31)
<223>  t = t/u

<400>  76
caagrawsaw mdgstgvtsv ttrrmdccam tcgagcgccg ccgcgcctat          50


<210>  77
<211>  49
<212>  DNA
<213>  Artificial

<220>
<223>  Synthetic primer

<400>  77
atggmkycaa bsabcastht wtswtycttg maccagcgcc tgagcctgc          49


<210>  78
<211>  51
<212>  DNA
<213>  Artificial

<220>
<223>  Synthetic primer


<220>
<221>  modified_base
<222>  (32)..(32)

<223>  t = t/u

<400>  78
tcaagrawsa wadastgvts vttgrmkcca mtcgagcgcc gccgcgccta t                51


<210>  79
<211>  48
<212>  DNA
<213>  Artificial

<220>
<223>  Synthetic primer

<400>  79
cstbtrhgsy cbbcbbtytc bswbcsybmg ccgtgcgggt tatggacg                    48


<210>  80
<211>  48
<212>  DNA
<213>  Artificial

<220>
<223>  Synthetic primer

<400>  80
cvrsgvwsvg aravvgvvgr scdyavasmg tccgctcctt cccggtgg                    48


<210>  81
<211>  52
<212>  DNA
<213>  Artificial

<220>
<223>  Synthetic primer

<400>  81
acstbsabgs ygsstkktyt cbswbcgyyg mccgtgcggg ttatggacgc ac               52


<210>  82
<211>  51
<212>  DNA
<213>  Artificial

<220>
<223>  Synthetic primer


<220>
<221>  modified_base
<222>  (32)..(32)
<223>  t = t/u

<400>  82
gcrrcgvwsv garammassc rscvtsvasg mtccgctcct tcccggtggt g                51


<210>  83
<211>  46
<212>  DNA

<213>  Artificial

<220>
<223>  Synthetic primer

<400>  83
ggbygmcgss ghagscgwrs tbcrsaabmc gccccaggat cggcca                46


<210>  84
<211>  48
<212>  DNA
<213>  Artificial

<220>
<223>  Synthetic primer

<400>  84
gvttsygvas ywcgsctdcs scgkcrvcmc ccgcctatcg gaccgtcg              48


<210>  85
<211>  49
<212>  DNA
<213>  Artificial

<220>
<223>  Synthetic primer

<400>  85
gggkygmcgs sscagscgwr stscrsaabc mgccccagga tcggccagc             49


<210>  86
<211>  50
<212>  DNA
<213>  Artificial

<220>
<223>  Synthetic primer

<400>  86
cgvttsygsa sywcgsctgs sscgkcrmcc mccgcctatc ggaccgtcgg            50


<210>  87
<211>  47
<212>  DNA
<213>  Artificial

<220>
<223>  Synthetic primer


<220>
<221>  modified_base
<222>  (30)..(30)
<223>  t = t/u

<400>  87
atagscgsbg hsgkwcykgh agbcgssamt cgacgcggca gccgaag               47

<210> 88
<211> 52
<212> DNA
<213> Artificial

<220>
<223> Synthetic primer


<220>
<221> modified_base
<222> (30)..(30)
<223> t = t/u

<400> 88
atsscgvctd cmrgwmcsdc vscgsctamt gggcatgtcg actgcaacta ca 52


<210> 89
<211> 45
<212> DNA
<213> Artificial

<220>
<223> Synthetic primer


<220>
<221> modified_base
<222> (30)..(30)
<223> t = t/u

<400> 89
atagscgssg hcgkwcyksh agbcgssamt cgacgcggca gccga 45


<210> 90
<211> 52
<212> DNA
<213> Artificial

<220>
<223> Synthetic primer


<220>
<221> modified_base
<222> (30)..(30)
<223> t = t/u

<400> 90
atsscgvctd smrgwmcgdc sscgsctamt gggcatgtcg actgcaacta ca 52


<210> 91
<211> 50
<212> DNA
<213> Artificial


<220>
<223> Synthetic primer

<220>
<221>  modified_base
<222>  (31)..(31)
<223>  t = t/u

<400>  91
cadmtrhwtc kbtcystrhg bygsygssam tggacatatt gggcgtggcc          50


<210>  92
<211>  50
<212>  DNA
<213>  Artificial

<220>
<223>  Synthetic primer

<400>  92
atsscrscrv cdyasrgavm gawdyakhtm gaccggctgg agcgcgaggt          50


<210>  93
<211>  52
<212>  DNA
<213>  Artificial

<220>
<223>  Synthetic primer


<220>
<221>  modified_base
<222>  (32)..(32)
<223>  t = t/u

<400>  93
tcadmtrhwt ckbtcystrh gbtgsygssa mtggacatat tgggcgtggc cg       52


<210>  94
<211>  50
<212>  DNA
<213>  Artificial

<220>
<223>  Synthetic primer

<400>  94
atsscrscav cdyasrgavm gawdyakhtg maccggctgg agcgcgaggt          50


<210>  95
<211>  52
<212>  DNA
<213>  Artificial

<220>
<223>  Synthetic primer

<400>  95
cgcgwmcrst wwgbtmmaky twhawwystk cmcaagcgtc gacgggggta tt       52

<210>  96
<211>  53
<212>  DNA
<213>  Artificial

<220>
<223>  Synthetic primer

<400>  96
ggmasrwwtd warmtkkavc wwasygkwcg cmgatgctca acttcatcgc ggt          53

<210>  97
<211>  42
<212>  DNA
<213>  Artificial

<220>
<223>  Synthetic primer

<400>  97
tggrywytsy kwyywtbcss ggymggtgcc ttccggcgcc ac                      42

<210>  98
<211>  45
<212>  DNA
<213>  Artificial

<220>
<223>  Synthetic primer

<400>  98
crccssgvaw rrwmrsarwr yccmactcgc gcggtaccca tatcc                   45

<210>  99
<211>  43
<212>  DNA
<213>  Artificial

<220>
<223>  Synthetic primer

<400>  99
tggrywytsy gwycttbcss ggygmgtgcc ttccggcgcc acc                     43

<210>  100
<211>  48
<212>  DNA
<213>  Artificial

<220>
<223>  Synthetic primer

<400>  100
ccrccssgva agrwcrsarw ryccmactcg cgcggtaccc atatcctg                48

<210>  101

<211>  52
<212>  DNA
<213>  Artificial

<220>
<223>  Synthetic primer

<400>  101
atdcctggta tgtggcgdsc mygsmcgrsg maactgtccg aaaagccgct cg          52


<210>  102
<211>  53
<212>  DNA
<213>  Artificial

<220>
<223>  Synthetic primer


<220>
<221>  modified_base
<222>  (32)..(32)
<223>  t = t/u

<400>  102
tcsycgkscr kgshcgccac ataccaggha mttgcggagg aaggtcataa ggg         53


<210>  103
<211>  52
<212>  DNA
<213>  Artificial

<220>
<223>  Synthetic primer

<400>  103
atdcctggta tgtggcgdsc mygsmcgrsg maactgtccg aaaagccgct cg          52


<210>  104
<211>  52
<212>  DNA
<213>  Artificial

<220>
<223>  Synthetic primer


<220>
<221>  modified_base
<222>  (32)..(32)
<223>  t = t/u

<400>  104
tcsycgkscr kgshcgccac ataccaggha mttgcggacg aagctcataa gg          52


<210>  105
<211>  52
<212>  DNA
<213>  Artificial

<220>
<223>  Synthetic primer

<400>  105
ccgrsgaadt adccrmsrmg ccgctcvvcc mggacgattc tcgacacacc gc          52


<210>  106
<211>  49
<212>  DNA
<213>  Artificial

<220>
<223>  Synthetic primer

<400>  106
cggbbgagcg gckyskyggh tahttcsycg mggcagcgcc gccacatac              49


<210>  107
<211>  50
<212>  DNA
<213>  Artificial

<220>
<223>  Synthetic primer

<400>  107
tcbvccggas sattmkcrrs dvgsscvtgg mcgctctacc gccagcccga             50


<210>  108
<211>  52
<212>  DNA
<213>  Artificial

<220>
<223>  Synthetic primer

<400>  108
gccabgsscb hsyygmkaat sstccggbvg magcggcttt tcggacagtt cc          52


<210>  109
<211>  46
<212>  DNA
<213>  Artificial

<220>
<223>  Synthetic primer


<220>
<221>  modified_base
<222>  (32)..(32)
<223>  t = t/u

<400>  109
tcsygytctw ccgcvksvsc vasggtbyag mtcgcggcgc tgctcg                 46


<210>  110

<211>  52
<212>  DNA
<213>  Artificial

<220>
<223>  Synthetic primer

<400>  110
actrvaccst bgsbsmbgcg gwagarcrsg magcggtgtg tcgagaatcg tc          52


<210>  111
<211>  51
<212>  DNA
<213>  Artificial

<220>
<223>  Synthetic primer


<220>
<221>  modified_base
<222>  (32)..(32)
<223>  t = t/u

<400>  111
gcbdcgcgcc gctgagcvwm ggcadastgg mtcaacggcc atctccaatg c           51


<210>  112
<211>  52
<212>  DNA
<213>  Artificial

<220>
<223>  Synthetic primer

<400>  112
accasthtgc ckwbgctcag cggcgcghvg mcggtgcgga cagatgtcga gc          52


<210>  113
<211>  51
<212>  DNA
<213>  Artificial

<220>
<223>  Synthetic primer

<400>  113
ctcgwgrrcg vccatvtcvd stgcssctat mcacgggctg gaattcgatg g           51


<210>  114
<211>  49
<212>  DNA
<213>  Artificial

<220>
<223>  Synthetic primer

<400>  114
gatagssgca shbgabatgg bcgyycwcga mggatgccgt cgctcagcg              49

<210>  115
<211>  50
<212>  DNA
<213>  Artificial

<220>
<223>  Synthetic primer

<400>  115
tgvastwcrr crscrrcggg mastgcrycc mataacccgc acggcaatgg                50


<210>  116
<211>  51
<212>  DNA
<213>  Artificial

<220>
<223>  Synthetic primer

<400>  116
tggrygcast kcccgyygsy gyygwastbc magcccgtga taggggcatt g              51


<210>  117
<211>  54
<212>  DNA
<213>  Artificial

<220>
<223>  Synthetic primer

<400>  117
ctatgsccat dwcsastgcr actacargct mgctggtcga caacctgatg gacc           54


<210>  118
<211>  49
<212>  DNA
<213>  Artificial

<220>
<223>  Synthetic primer

<400>  118
cagcytgtag tygcastsgw hatggscata mgccgccgac ggtccgata                 49


<210>  119
<211>  50
<212>  DNA
<213>  Artificial

<220>
<223>  Synthetic primer

<400>  119
gckccvtgmw saactwcrtc gcgrkckccc mcggaaggca ccccgaagga                50


<210>  120

<211>  52
<212>  DNA
<213>  Artificial

<220>
<223>  Synthetic primer

<400>  120
ggggmgmycg cgaygwagtt swkcabggmg mctcaccttg ttccagcgga tg          52


<210>  121
<211>  49
<212>  DNA
<213>  Artificial

<220>
<223>  Synthetic primer

<400>  121
caatttcsvc vtcsasracv scgmsvtgga mcggcgtgat ccgcagctg             49


<210>  122
<211>  50
<212>  DNA
<213>  Artificial

<220>
<223>  Synthetic primer

<400>  122
gtccabskcg sbgtystsga bgbsgaaatt mgcgcgagga gccgaagaaa            50


<210>  123
<211>  48
<212>  DNA
<213>  Artificial

<220>
<223>  Synthetic primer

<400>  123
caatttcsvc vtcsasracv scgmsvtgga mcggcgtgct gcgcagct              48


<210>  124
<211>  49
<212>  DNA
<213>  Artificial

<220>
<223>  Synthetic primer


<220>
<221>  modified_base
<222>  (32)..(32)
<223>  t = t/u

<400>  124
gcrrcggcsv gcgcsvgssc tcgctcvasg mtccgctcct tcccggtgg             49

<210>  125
<211>  50
<212>  DNA
<213>  Artificial

<220>
<223>  Synthetic primer

<400>  125
acstbgagcg agsscbsgcg cbsgccgyyg mccgtgcggg ttatggacgc                50


<210>  126
<211>  52
<212>  DNA
<213>  Artificial

<220>
<223>  Synthetic primer


<220>
<221>  misc_feature
<222>  (21)..(21)
<223>  n is a, c, g, or t

<220>
<221>  modified_base
<222>  (32)..(32)
<223>  t = t/u

<400>  126
ccywcscggt ggtggagarg nacsschwca mtctggatct ggcccggcga tc             52


<210>  127
<211>  51
<212>  DNA
<213>  Artificial

<220>
<223>  Synthetic primer


<220>
<221>  misc_feature
<222>  (11)..(11)
<223>  n is a, c, g, or t

<400>  127
atgwdgssgt ncytctccac caccgsgwrg mgagcggacg ttgagcgaag c              51


<210>  128
<211>  54
<212>  DNA
<213>  Artificial

<220>
<223>  Synthetic primer

<400>  128
gatcccgvgt atcggwmcsd cvscggctat mgggcatgtc gactgcaact acaa          54


<210>  129
<211>  48
<212>  DNA
<213>  Artificial

<220>
<223>  Synthetic primer

<400>  129
catagccgsb ghsgkwccga tacbcgggat mcgacgcggc agccgaag                 48


<210>  130
<211>  52
<212>  DNA

<213>  Artificial

<220>
<223>  Synthetic primer

<400>  130
ggmasgacwd cmrgtggmms mwggtgkscg mcgatgctca acttcatcgc gg            52


<210>  131
<211>  51
<212>  DNA
<213>  Artificial

<220>
<223>  Synthetic primer

<400>  131
gcgsmcaccw kskkccacyk ghwgtcstkc mcaagcgtcg acgggggtat t             51


<210>  132
<211>  50
<212>  DNA
<213>  Artificial

<220>
<223>  Synthetic primer


<220>
<221>  modified_base
<222>  (32)..(32)
<223>  t = t/u

<400>  132
gaaggcascs scvasgagcr gagcryccac mtcgcgcggt acccatatcc               50


<210>  133
<211>  45
<212>  DNA
<213>  Artificial

<220>
<223>  Synthetic primer

<400>  133
agtggrygct cygctcstbg ssgstgcctt mccggcgcca ccgcg                45


<210>  134
<211>  59
<212>  DNA
<213>  Artificial

<220>
<223>  Synthetic primer

<400>  134
ggsygsasar ggaggtgrtc gtcsgcrasg mgtgagatac aggcgctgat gaagattcc      59


<210>  135
<211>  52
<212>  DNA
<213>  Artificial

<220>
<223>  Synthetic primer

<400>  135
ccstygcsga cgaycacctc cytstscrsc mcggtcgaag gcgtcggtct gg          52


<210>  136
<211>  50
<212>  DNA
<213>  Artificial

<220>
<223>  Synthetic primer


<220>
<221>  modified_base
<222>  (32)..(32)
<223>  t = t/u

<400>  136
gcgscasccc gascrtcmtg atggccaagt mtcctgcgcg gcgccaatac            50


<210>  137
<211>  56
<212>  DNA
<213>  Artificial

<220>
<223>  Synthetic primer

<400>  137
aacttggcca tcakgaygst cgggstgscg mccgggaatc ttcatcagcg cctgta        56


<210>  138

<211>  53
<212>  DNA
<213>  Artificial

<220>
<223>  Synthetic primer


<220>
<221>  modified_base
<222>  (32)..(32)
<223>  t = t/u

<400>  138
ccargtwcsy gargrgcgcc aataskcccg mtcgacgctt ggaacgacat ccg          53


<210>  139
<211>  51
<212>  DNA
<213>  Artificial

<220>
<223>  Synthetic primer

<400>  139
acggggstat tggcgcycyt crsgwacytg mgccatcagc acgctcggcg t          51


<210>  140
<211>  51
<212>  DNA
<213>  Artificial

<220>
<223>  Synthetic primer


<220>
<221>  modified_base
<222>  (32)..(32)
<223>  t = t/u

<400>  140
gagrccagcw gcyactattw ctwcgsctcc mtcgcgcaat ttcggcatcg a          51


<210>  141
<211>  54
<212>  DNA
<213>  Artificial

<220>
<223>  Synthetic primer

<400>  141
aggagscgwa gwaatagtrg cwgctggyct mccgtctcgg gggtcaggat atgg          54


<210>  142
<211>  55
<212>  DNA
<213>  Artificial

<220>
<223>  Synthetic primer

<400>  142
tgcgcagctk scaggsccag gscstggyca maggaggaca aggtcgtcgt cgagg          55


<210>  143
<211>  51
<212>  DNA
<213>  Artificial

<220>
<223>  Synthetic primer

<400>  143
ttgrccasgs cctggscctg smagctgcgc magcacgccg tccatctccg g              51


<210>  144
<211>  45
<212>  DNA
<213>  Artificial

<220>
<223>  Synthetic primer

<400>  144
ggmasgacwd cmrgtggmms mwgmgtgagc gcgatgctca acttc                     45


<210>  145
<211>  44
<212>  DNA
<213>  Artificial

<220>
<223>  Synthetic primer

<400>  145
ccwkskkcca cykghwgtcs tkcmcaagcg tcgacggggg tatt                      44


<210>  146
<211>  42
<212>  DNA
<213>  Artificial

<220>
<223>  Synthetic primer

<400>  146
ggcctgvast wcrrcrscrr cmgggcagtg cgtccataac cc                        42


<210>  147
<211>  45
<212>  DNA
<213>  Artificial

<220>
<223>  Synthetic primer

<400>  147
cgyygsygyy gwastbcagg cmcgtgatag gggcattgga gatgg                    45


<210>  148
<211>  39
<212>  DNA
<213>  Artificial

<220>
<223>  Synthetic primer

<400>  148
cgscascccg ascrtcmtmg atggccaagt tcctgcgcg                           39


<210>  149
<211>  40
<212>  DNA
<213>  Artificial

<220>
<223>  Synthetic primer

<400>  149
cakgaygstc gggstgscmg ccgggaatct tcatcagcgc                          40


<210>  150
<211>  50
<212>  DNA
<213>  Artificial

<220>
<223>  Synthetic primer

<400>  150
gtccabhkcc ksgtygtyga bgbcgaaatt mgcgcgagga gccgaagaaa               50


<210>  151
<211>  48
<212>  DNA
<213>  Artificial

<220>
<223>  Synthetic primer

<400>  151
caatttcgvc vtcracracs mggmdvtgga mcggcgtgct gcgcagct                 48


<210>  152
<211>  6
<212>  PRT
<213>  Artificial

<220>
<223>  Synthetic peptide

<220>
<221>  misc_feature

<222>  (2)..(2)
<223>  Xaa is Gln, Phe, or His

<220>
<221>  misc_feature
<222>  (3)..(3)
<223>  Xaa is Ala, Asp, Phe, Ile, Arg, Thr, Val, Trp, Tyr, Cys, Glu, Gly, Leu,
Met, Gln, or Ser

<220>
<221>  misc_feature
<222>  (4)..(4)
<223>  Xaa is  Gln, Gly, Ile, Val, Ala, Cys, Asp, His, Leu, Met, Asn, Arg, Ser,
Thr, or Glu

<220>
<221>  misc_feature
<222>  (5)..(5)
<223>  Xaa is Ala, Cys, Gly, or Ser

<400>  152
Trp Xaa Xaa Xaa Xaa Leu
1               5


<210>  153
<211>  5
<212>  PRT
<213>  Artificial

<220>
<223>  Synthetic peptide

<220>
<221>  misc_feature
<222>  (1)..(1)
<223>  Xaa is Ser, His, or Thr

<220>
<221>  misc_feature
<222>  (2)..(2)
<223>  Xaa is Arg, Gln, Ser, Thr, Phe, His, Asn, Val, Trp, Tyr, Cys, Ile, Lys,
Leu, or Met

<220>
<221>  misc_feature
<222>  (4)..(4)
<223>  Xaa is Thr, Gln, or Met

<400>  153
Xaa Xaa Gly Xaa His
1               5


<210>  154
<211>  345
<212>  PRT
<213>  Artificial

<220>
<223>  Synthetic peptide

<400>  154

Met His His His His His His Ser Phe Val Arg Asn Ala Trp Tyr Val
1               5                   10                  15

Ala Ala Leu Pro Glu Glu Leu Ser Glu Lys Pro Leu Gly Arg Thr Ile
            20                  25                  30

Leu Asp Thr Pro Leu Ala Leu Tyr Arg Gln Pro Asp Gly Val Val Ala
        35                  40                  45

Ala Leu Leu Asp Ile Cys Pro His Arg Phe Ala Pro Leu Ser Asp Gly
    50                  55                  60

Ile Leu Val Asn Gly His Leu Gln Cys Pro Tyr His Gly Leu Glu Phe
65                  70                  75                  80

Asp Gly Gly Gly Gln Cys Val His Asn Pro His Gly Asn Gly Ala Arg
                85                  90                  95

Pro Ala Ser Leu Asn Val Arg Ser Phe Pro Val Val Glu Arg Asp Ala
            100                 105                 110

Leu Ile Trp Ile Trp Pro Gly Asp Pro Ala Leu Ala Asp Pro Gly Ala
        115                 120                 125

Leu Pro Asp Phe Gly Cys Arg Val Asp Pro Ala Tyr Arg Thr Val Gly
    130                 135                 140

Gly Tyr Gly His Val Asp Cys Asn Tyr Lys Leu Leu Val Asp Asn Leu
145                 150                 155                 160

Met Asp Leu Gly His Ala Gln Tyr Val His Arg Ala Asn Ala Gln Thr
                165                 170                 175

Asp Ala Phe Asp Arg Leu Glu Arg Glu Val Ile Val Gly Asp Gly Glu
            180                 185                 190

Ile Gln Ala Leu Met Lys Ile Pro Gly Gly Thr Pro Ser Val Leu Met
        195                 200                 205

Ala Lys Phe Leu Arg Gly Ala Asn Thr Pro Val Asp Ala Trp Asn Asp
    210                 215                 220

Ile Arg Trp Asn Lys Val Ser Ala Met Leu Asn Phe Ile Ala Val Ala
225                 230                 235                 240

Pro Glu Gly Thr Pro Lys Glu Gln Ser Ile His Ser Arg Gly Thr His
                245                 250                 255

Ile Leu Thr Pro Glu Thr Glu Ala Ser Cys His Tyr Phe Phe Gly Ser
            260                 265                 270

Ser Arg Asn Phe Gly Ile Asp Asp Pro Glu Met Asp Gly Val Leu Arg
        275                 280                 285

Ser Trp Gln Ala Gln Ala Leu Val Lys Glu Asp Lys Val Val Val Glu
    290                 295                 300

Ala Ile Glu Arg Arg Arg Ala Tyr Val Glu Ala Asn Gly Ile Arg Pro
305                 310                 315                 320

Ala Met Leu Ser Cys Asp Glu Ala Ala Val Arg Val Ser Arg Glu Ile
                325                 330                 335

Glu Lys Leu Glu Gln Leu Glu Ala Ala
            340                 345

77